# EUROPEAN PATENT SPECIFICATION

(11) **EP 3 498 845 B1**
(45) Date of publication and mention of the grant of the patent: **22.06.2022**
(21) Application number: 18204734.0
(22) Date of filing: 01.04.2015
(51) Int. Cl.: C12N 15/113, C12N 15/63

(54) **CRISPR/CAS-RELATED METHODS AND COMPOSITIONS FOR TREATING HERPES SIMPLEX VIRUS TYPE 1 (HSV-1)**
CRISPR-/CAS-ASSOZIIERTE VERFAHREN UND ZUSAMMENSETZUNGEN ZUR BEHANDLUNG DES HERPES SIMPLEX VIRUS TYP 1 (HSV-1)
PROCÉDÉS ET COMPOSITIONS ASSOCIÉS À CRISPR/CAS POUR LE TRAITEMENT DU VIRUS DE L'HERPÈS SIMPLEX DE TYPE 1 (HSV-1)

(30) Priority: 01.04.2014 US 201461973793 P
(43) Date of publication of application: 19.06.2019
(62) Divisional of application: 15716680.2
(73) Proprietor: Editas Medicine, Inc., Cambridge, MA 02141 (US)
(72) Inventor: MAEDER, Morgan L., Jamaica Plain, MA Massachusetts 02130 (US); FRIEDLAND, Ari E., Boston, MA Massachusetts 02118 (US); WELSTEAD, G. Grant, Cambridge, MA Massachusetts 02139 (US); BUMCROT, David A., Belmont, MA Massachusetts 02478 (US)
(74) Representative: Barker Brettell LLP

(56) References cited:
- WO-A1-2014/093635
- PRASHANT MALI ET AL: "Cas9 as a versatile tool for engineering biology", NATURE METHODS, NATURE PUBLISHING GROUP, GB, vol. 10, no. 10, 1 October 2013 (2013-10-01), pages 957-963, XP002718606, ISSN: 1548-7105, DOI: 10.1038/NMETH.2649 [retrieved on 2013-09-27]
- MARTINE AUBERT ET AL: "In vitro Inactivation of Latent HSV by Targeted Mutagenesis Using an HSV-specific Homing Endonuclease", MOLECULAR THERAPY-NUCLEIC ACIDS, vol. 3, no. 2, 4 February 2014 (2014-02-04), page e146, XP055198360, DOI: 10.1038/mtna.2013.75
- RICHARD L. THOMPSON ET AL: "De Novo Synthesis of VP16 Coordinates the Exit from HSV Latency In Vivo", PLOS PATHOGENS, vol. 5, no. 3, 27 March 2009 (2009-03-27), page e1000352, XP055582188, DOI: 10.1371/journal.ppat.1000352
- WEINHEIMER S P ET AL: "deletion of the VP16 open reading frame of Herpes Simplex Virus Type 1", JOURNAL OF VIROLOGY, THE AMERICAN SOCIETY FOR MICROBIOLOGY, US, vol. 66, no. 1, 1 January 1992 (1992-01-01), pages 258-269, XP002130926, ISSN: 0022-538X
- HIROTAKA EBINA ET AL: "Harnessing the CRISPR/Cas9 system to disrupt latent HIV-1 provirus", SCIENTIFIC REPORTS, vol. 3, 26 August 2013 (2013-08-26), XP055110157, DOI: 10.1038/srep02510
- WEBER NICHOLAS D ET AL: "DNA cleavage enzymes for treatment of persistent viral infections: Recent advances and the pathway forward", VIROLOGY, vol. 454, 31 January 2014 (2014-01-31), pages 353-361, XP028846128, ISSN: 0042-6822, DOI: 10.1016/J.VIROL.2013.12.037
- J. T. SCHIFFER ET AL: "Targeted DNA Mutagenesis for the Cure of Chronic Viral Infections", JOURNAL OF VIROLOGY, vol. 86, no. 17, 20 June 2012 (2012-06-20) , pages 8920-8936, XP055155733, ISSN: 0022-538X, DOI: 10.1128/JVI.00052-12
- A E Friedland ET AL: "Staphyloccocus aureus Cas9: an alternative Cas9 for genome editing applications", , 12 January 2015 (2015-01-12), page 1, XP055292927, DOI: 10.1016/S1525-0016(16)34170-3 Retrieved from the Internet: URL:http://www.editasmedicine.com/document s/AEF Poster Keystone 2015.pdf [retrieved on 2016-08-02]

## Description

### FIELD OF THE INVENTION

The invention relates to CRISPR/CAS-related methods and components for editing of a target nucleic acid sequence, or modulating expression of a target nucleic acid sequence, and applications thereof in connection with herpes simplex virus type 1 (HSV-1).

### BACKGROUND

Herpes simplex virus type 1 (HSV-1) causes intermittent sores of the mouth and mucous membranes. It is a ubiquitous and highly contagious pathogen. The majority of the population develops the infection during childhood. By adulthood, up to 80% of the population in the United States is infected with HSV-1. New HSV-1 infections occur at a rate of 1.6 cases per 100 person years (Langenberg et al., 1999; New England Journal of Medicine 341:1432-1438). The most severe manifestations of HSV-1 infection include, e.g., keratitis, encephalitis, and meningitis.

Infection of HSV-1 is permanent. After initial infection with HSV-1, the virus establishes latent infection that lasts for the lifetime of the host. Initial infection with HSV-1 generally causes painful blistering of the mucous membranes of the lips and mouth or genital region. After initial infection, HSV-1 establishes latent infection in all subjects. Following establishment of latent infection, reactivation of HSV-1 can occur at any point during the lifetime of the subject. Reactivation of HSV-1 is more likely to occur in the elderly and in immunocompromised individuals, including in those who have cancer, those who have HIV/AIDs and in those who have undergone solid organ or hematopoietic stem cell transplant.

HSV-1 encephalitis and HSV-1 meningitis are among the most severe and debilitating types of HSV infections. HSV encephalitis is the most common form of non-epidemic encephalitis. The annual incidence of HSV encephalitis is 0.2-0.4 in 100,000 individuals (Saba et al., 2012; British Medical Journal 344: e3166). Subjects who develop HSV-1 encephalitis and/or meningitis commonly have permanent neurologic sequelae.

Ocular herpes can affect the epithelium of the eye, causing keratitis, or the retina, where it may lead to acute retinal necrosis. Keratitis is the most common form of ocular herpes. HSV-1 keratitis is the most common cause of infectious blindness in the developed world (Dawson et. al., Suvey of Ophthalmology 1976; 21(2): 121-135). Worldwide, there are approximately 1.5 million cases of HSV-related ophthalmologic disease and 40,000 cases of HSV-related blindness or severe monocular visual impairment annually (Krawczyk et. al., Public Library of Science One 2015; 10(1): e0116800. Farooq and Shukla 2012; Survey of Ophthalmology 57(5): 448-462.). HSV-1 retinitis most often affects adults and can cause acute retinal necrosis (ARN). ARN causes permanent visual damage in more than 50% of subjects (Roy et al., Ocular Immunology and Inflammation 2014; 22(3):170-174).

Newborns are a population at particular risk for developing severe HSV-1 infections. The disease is transmitted from the mother to the fetus during childbirth. The chance of maternal-fetal transmission is highest in cases where the mother developed primary HSV infection during pregnancy. The incidence of neonatal herpes is approximately 4-30 per 100,000 births (Brown ZA, et al., 2003; Journal of the American Medical Association; 289(2): 203-209. Dinh T-H, et al., 2008; Sexually Transmitted Disease; 35(1): 19-21). Neonates can develop severe HSV-1 keratitis, retinitis, encephalitis and/or meningitis. Neonatal ocular herpes can result in immediate, permanent vision loss. Ocular HSV-1 puts neonates at risk for later developing ARN. Untreated HSV-1 encephalitis leads to death in 50% of neonates. Even with prompt treatment with antiviral therapy, the majority of neonates who contract HSV-1 encephalitis or meningitis will suffer from permanent neurologic sequelae.

There are no curative or preventative treatments for HSV-1. Therapy is primarily given during acute infection. Primary HSV-1 infections can be treated with antiviral therapy, including acyclovir, valacyclovir and famciclovir. These therapies may reduce viral shedding, decrease pain and improve healing time of lesions. Re-activated, latent infections may resolve without treatment (may be self-limiting) or may be treated with anti-viral therapy. Antiviral therapy may be given prophylactically in certain situations, including during childbirth in a mother with a recent HSV-1 infection or reactivation.

Vaccines are in development for the prevention of HSV-1 infection. However, in controlled clinical trials, vaccination efficacy has been limited. A recent vaccine for both HSV-1 and HSV-2 infections was only 35% effective in preventing HSV-1 infection (Belshe et al., 2012; New England Journal of Medicine 366(1): 34-43).

MARTINE AUBERT ET AL: "In vitro Inactivation of Latent HSV by Targeted Mutagenesis Using an HSV-specific Homing Endonuclease",MOLECULAR THERAPY-NUCLEIC ACIDS, vol. 3, no. 2, 4 February 2014 (2014-02-04), page e146 teaches an inactivation of HSV-1 using a homing endonuclease.

Despite advances in antiretroviral therapies, there remains a need for the treatment and prevention of HSV-1 infection, particularly the treatment and prevention of HSV-1 associated keratitis, retinitis, encephalitis and meningitis. A therapy that can cure, prevent, or treat HSV-1 infections would be superior to the current standard of care.

### SUMMARY OF THE INVENTION

The invention compositions targeting the HSV UL48 gene by means of saCas9 and medical uses thereof as defined in the claims. Compositions targeting the HSV genes UL19, UL30 and UL54 and uses thereof as described in the specification are not part of the invention neither are compositions targeting the UL48 gene using other Cas9 than saCas9. The references to methods of treatment at p. 3, 1. 19; p. 49, 1. 3; as well as in the section headed "Methods to treat, prevent or reduce HSV-1 infection" starting at p. 49 of this description are to be interpreted as references to the compounds, pharmaceutical compositions and medicaments of the present invention for use in a method for treatment of the human (or animal) body by therapy.

Methods and compositions discussed herein, provide for the treatment or prevention of herpes simplex virus type 1 (HSV-1), which causes intermittent sores of the mouth and mucous membranes. HSV-1 is contained within an icosahedral particle. The virus enters the host via infection of epithelial cells within the skin and mucous membranes. The virus produces immediate early genes within the epithelial cells, which encode enzymes and binding proteins necessary for viral synthesis. After primary infection, the virus travels up sensory nerve axons via retrograde transport to the sensory dorsal root ganglion (DRG). Within the DRG, it establishes a latent infection. The latent infection persists for the lifetime of the host. Within the DRG cell, the virus uncoats, viral DNA is transported into the nucleus, and key viral RNAs associated with latency are transcribed (including the LAT RNAs).

In an aspect, the invention provides a CRISPR/Cas system that is capable of knocking out a herpes simplex virus (HSV) viral *UL48* gene, comprising: a gRNA molecule comprising a targeting domain, wherein said targeting domain is configured to target a target position that is within the coding region that is downstream of the first 500bp of said HSV viral *UL48* gene; and an *S. aureus* Cas9 molecule.

In another aspect, the invention provides a nucleic acid that is capable of knocking out a herpes simplex virus (HSV) viral *UL48* gene, comprising: (a) a first nucleotide sequence that encodes a gRNA molecule comprising a targeting domain, wherein said targeting domain is configured to target a target sequence that is within the coding region that is downstream of the first 500bp of the HSV viral *UL48* gene, and (b) a second nucleotide sequence that encodes an *S. aureus* Cas9 molecule.

In another aspect, the invention provides a composition comprising (a) a first nucleic acid molecule comprising a nucleotide sequence that encodes a gRNA molecule comprising a targeting domain, wherein said targeting domain is configured to target a target sequence that is within the coding region that is downstream of the first 500bp of the HSV viral *UL48* gene; and (b) a second nucleic acid molecule comprising a nucleotide sequence that encodes an *S. aureus* Cas9 molecule.

In another aspect, the invention provides a cell comprising the CRISPR/Cas system of the invention, the nucleic acid of the invention, or the composition of the invention.

In another aspect, there is provided a vector comprising (a) the nucleic acid of the invention or (b) the first or second nucleic acid of the composition of the invention.

Methods and compositions discussed herein provide for treatment or prevention of herpes simplex virus type 1 (HSV-1), or its symptoms, e.g., by knocking out one or more of the HSV-1 viral genes, e.g., by knocking out one or more of *UL19, UL30, UL48* and/or *UL54* gene(s). In one aspect, methods and compositions discussed herein may be used to alter one or more of *UL19, UL30, UL48* and/or *UL54* gene(s) to treat or prevent HSV-1 by targeting the gene, e.g., the non-coding or coding regions, e.g., the promoter region, or a transcribed sequence, e.g., intronic or exonic sequence. In an embodiment, coding sequence, e.g., a coding region, e.g., an early coding region, of one or more of *UL19, UL30, UL48* and/or *UL54* gene(s), is targeted for alteration and knockout of expression.

In another aspect, the methods and compositions discussed herein may be used to alter one or or more of *UL19, UL30, UL48* and/or *UL54* gene(s) to treat or prevent herpes simplex virus type 1 (HSV-1) by targeting the coding sequence of one or more of *UL19, UL30, UL48* and/or *UL54* gene(s). In one embodiment, the gene, e.g., the coding sequence of one or more of the *UL19, UL30, UL48* and/or *UL54* gene(s), are targeted to knockout one or more of *UL19, UL30, UL48* and/or *UL54* gene(s), e.g., to eliminate expression of one or more of *UL19, UL30, UL48* and/or *UL54* gene(s), e.g., to knockout one or more copies of one or more of *UL19, UL30, UL48* and/or *UL54* gene(s), e.g., by induction of an alteration comprising a deletion or mutation in one or more of *UL19, UL30, UL48* and/or *UL54* gene(s). In an embodiment, the method provides an alteration that comprises an insertion or deletion. As described herein, a targeted knockout approach is mediated by non-homologous end joining (NHEJ) using a CRISPR/Cas system comprising an enzymatically active Cas9 (eaCas9) molecule.

In one embodiment, an early coding sequence of one or more of *UL19, UL30, UL48* and/or *UL54* gene(s) are targeted to knockout one or more of *UL19, UL30, UL48* and/or *UL54* gene(s). In an embodiment, targeting affects one or more copies of the *UL19, UL30, UL48* and/or *UL54* gene(s). In an embodiment, a targeted knockout approach reduces or eliminates expression of one or more functional *UL19, UL30, UL48* and/or *UL54* gene product(s). In an embodiment, the method provides an alteration that comprises an insertion or deletion.

In another aspect, the methods and compositions discussed herein may be used to alter one or more of *UL19, UL30, UL48* and/or *UL54* gene(s) to treat or prevent HSV-1 by targeting non-coding sequence of the *UL19, UL30, UL48* and/or *UL54* gene(s), e.g., promoter, an enhancer, an intron, 3'UTR, and/or polyadenylation signal. In one embodiment, the gene(s), e.g., the non-coding sequence of one or more *UL19, UL30, UL48* and/or *UL54* gene(s), is targeted to knockout the gene(s), e.g., to eliminate expression of the gene(s), e.g., to knockout one or more copies of the *UL19, UL30, UL48* and/or *UL54* gene(s), e.g., by induction of an alteration comprising a deletion or mutation in the *UL19, UL30, UL48* and/or *UL54* gene(s). In an embodiment, the method provides an alteration that comprises an insertion or deletion.

"HSV-1 target UL19 position", as used herein, refers to a position in the *UL19* gene, which if altered by NHEJ-mediated alteration, results in reduction or elimination of expression of functional *UL19* gene product. In an embodiment, the position is in the *UL19* gene coding region, e.g., an early coding region.

"HSV-1 target UL30 position", as used herein, refers to a position in the *UL30* gene, which if altered by NHEJ-mediated alteration, results in reduction or elimination of expression of functional *UL30* gene product. In an embodiment, the position is in the *UL30* gene coding region, e.g., an early coding region.

"HSV-1 target UL48 position", as used herein, refers to a position in the *UL48* gene, which if altered by NHEJ-mediated alteration, results in reduction or elimination of expression of functional *UL48* gene product. In an embodiment, the position is in the *UL48* gene coding region, e.g., an early coding region.

"HSV-1 target UL54 position", as used herein, refers to a position in the *UL54* gene, which if altered by NHEJ-mediated alteration, results in reduction or elimination of expression of functional *UL54* gene product. In an embodiment, the position is in the *UL54* gene coding region, e.g., an early coding region.

"HSV-1 target position", as used herein, refers to any of a HSV-1 target UL19 target position, a HSV-1 target UL30 target position, a HSV-1 target UL48 target position and/or a HSV-1 target UL54 target position.

In one aspect, disclosed herein is a gRNA molecule, e.g., an isolated or non-naturally occurring gRNA molecule, comprising a targeting domain which is complementary with a target domain from the *UL19, UL30, UL48* or *UL54* gene.

In an embodiment, the targeting domain of the gRNA molecule is configured to provide a cleavage event, e.g., a double strand break or a single strand break, sufficiently close to a HSV-1 target position in the *UL19, UL30, UL48* or *UL54* gene to allow alteration, e.g., alteration associated with NHEJ, of a HSV-1 target position in the *UL19, UL30, UL48* or *UL54* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150 or 200 nucleotides of a HSV-1 target position. The break, e.g., a double strand or single strand break, can be positioned upstream or downstream of a HSV-1 target position in the *UL19, UL30, UL48* or *UL54* gene. In an embodiment, the targeting domain of the gRNA molecule is configured to provide a cleavage event selected from a double strand break and a single strand break, within 500 (e.g., within 500, 400, 300, 250, 200, 150, 100, 80, 60, 40, 20, or 10) nucleotides of a HSV-1 target position.

In an embodiment, a second gRNA molecule comprising a second targeting domain is configured to provide a cleavage event, e.g., a double strand break or a single strand break, sufficiently close to the HSV-1 target position in the *UL19, UL30, UL48* or *UL54* gene, to allow alteration, e.g., alteration associated with NHEJ, of the HSV-1 target position in the *UL19, UL30, UL48* or *UL54* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150 or 200 nucleotides of the target position. In an embodiment, the breaks, e.g., double strand or single strand breaks, are positioned on both sides of a nucleotide of a HSV-1 target position in the *UL19, UL30, UL48* or *UL54* gene. In an embodiment, the breaks, e.g., double strand or single strand breaks, are positioned on one side, e.g., upstream or downstream, of a nucleotide of a HSV-1 target position in the *UL19, UL30, UL48* or *UL54* gene. In an embodiment, the targeting domain of the first and/or second gRNA molecule is configured to provide a cleavage event selected from a double strand break and a single strand break, within 500 (e.g., within 500, 400, 300, 250, 200, 150, 100, 80, 60, 40, 20, or 10) nucleotides of a HSV-1 target position.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150 or 200 nucleotides of a HSV-1 target position. In an embodiment, the first and second gRNA molecules are configured such, that when guiding a Cas9 molecule, e.g., a Cas9 nickase, a single strand break will be accompanied by an additional single strand break, positioned by a second gRNA, sufficiently close to one another to result in alteration of a HSV-1 target position in the *UL19, UL30, UL48* or *UL54* gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 molecule is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position; and the targeting domain of a second gRNA molecule is configured such that a double strand break is positioned downstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position; and the targeting domains of a second and third gRNA molecule are configured such that two single strand breaks are positioned downstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position; and the targeting domains of a third and fourth gRNA molecule are configured such that two single strand breaks are positioned downstream of a HSV-1 target position in the *UL19, UL30, UL48* or *UL54* gene, e.g., within 1, 2, 3, 4, 5, 10, 15, 20, 25, 30, 35, 40, 45, 50, 60, 70, 80, 90, 100, 150 or 200 nucleotides of the target position. In an embodiment, the targeting domain of the first, second, third, and/or fourth gRNA molecule is configured to provide a cleavage event selected from a double strand break and a single strand break, within 500 (e.g., within 500, 400, 300, 250, 200, 150, 100, 80, 60, 40, 20, or 10) nucleotides of a HSV-1 target position.

It is contemplated herein that, in an embodiment, when multiple gRNAs are used to generate (1) two single stranded breaks in close proximity, (2) two double stranded breaks, e.g., flanking a HSV-1 target position (e.g., to remove a piece of DNA, e.g., to create a deletion mutation) or to create more than one indel in the gene, e.g., in a coding region, e.g., an early coding region, (3) one double stranded break and two paired nicks flanking a HSV-1 target position (e.g., to remove a piece of DNA, e.g., to insert a deletion) or (4) four single stranded breaks, two on each side of a position, that they are targeting the same HSV-1 target position. It is further contemplated herein that multiple gRNAs may be used to target more than one HSV-1 target position in the same gene, e.g., one or more of *UL19, UL30, UL48* and/or *UL54* gene(s).

In an embodiment, the targeting domain of the first gRNA molecule and the targeting domain of the second gRNA molecules are complementary to opposite strands of the target nucleic acid molecule. In an embodiment, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule, as described herein.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In an embodiment, the targeting domain comprises a sequence that is the same as, or differs by no more than 1, 2, 3, 4, or 5 nucleotides from, a targeting domain sequence described herein, e.g., from any one of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**. In an embodiment, the targeting domain comprises a sequence that is the same as a targeting domain sequence described herein, e.g., from any one of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**.

In other embodiments, a HSV-1 target position in the coding region, e.g., the early coding region, of the *UL19, UL30, UL48* or *UL54* gene is targeted, e.g., for knockout. In an embodiment, the targeting domain comprises a sequence that is the same as, or differs by no more than 1, 2, 3, 4, or 5 nucleotides from, a targeting domain sequence from any one of **Tables 1A-1G, Tables 2A-2G, Tables 3A-3G, or Tables 4A-4F.**

In an embodiment, the targeting domain is independently selected from those in **Tables 1A-1G.** In an embodiment, the targeting domain is independently selected from **Table 1A.**

In an embodiment, the targeting domain is independently selected from those in **Tables 2A-2G.** In an embodiment, the targeting domain is independently selected from **Table 2A.**

In an embodiment, the targeting domain is independently selected from those in **Tables 3A-3G.** In an embodiment, the targeting domain is independently selected from **Table 3A.**

In an embodiment, the targeting domain is independently selected from those in **Tables 4A-4F.** In an embodiment, the targeting domain is independently selected from **Table 4A.**

In an embodiment, the targeting domain is independently selected from those in **Tables 5A-5E.** In an embodiment, the targeting domain is independently selected from **Table 5A.**

In an embodiment, the targeting domain is independently selected from those in **Tables 6A-6G.** In an embodiment, the targeting domain is independently selected from **Table 6A.**

In an embodiment, the targeting domain is independently selected from those in **Tables 7A-7D.** In an embodiment, the targeting domain is independently selected from **Table 7A.**

In an embodiment, the targeting domain is independently selected from those in **Tables 8A-8E.** In an embodiment, the targeting domain is independently selected from **Table 8A.**

In an embodiment, the targeting domain is independently selected from those in **Tables 9A-9G.** In an embodiment, the targeting domain is independently selected from **Table 9A.**

In an embodiment, the targeting domain is independently selected from those in **Tables 10A-10C.** In an embodiment, the targeting domain is independently selected from **Table 10A.**

In an embodiment, the targeting domain is independently selected from those in **Tables 11A-11E.** In an embodiment, the targeting domain is independently selected from **Table 11A.**

In an embodiment, the targeting domain is independently selected from those in **Tables 12A-12G.** In an embodiment, the targeting domain is independently selected from **Table 12A.**

In an embodiment, the targeting domain is independently selected from those in **Tables 13A-13C.** In an embodiment, the targeting domain is independently selected from **Table 13A.**

In an embodiment, the targeting domain is independently selected from those in **Tables 14A-14E.** In an embodiment, the targeting domain is independently selected from **Table 14A.**

In an embodiment, the targeting domain is independently selected from those in **Tables 15A-15G.** In an embodiment, the targeting domain is independently selected from **Table 15A.**

In an embodiment, the targeting domain is independently selected from those in **Tables 16A-16C.** In an embodiment, the targeting domain is independently selected from **Table 16A.**

In an embodiment, the targeting domain is independently selected from those in **Table 27.**

In an embodiment, when the HSV-1 target position is the *UL19* gene coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, the targetuing domain of each guide RNA is independently selected from any of **Tables 1A-1G, Tables 5A-5E, Tables 6A-6G, or Tables 7A-7D.**

In an embodiment, when the HSV-1 target position is the *UL30* gene coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, the targetuing domain of each guide RNA is independently selected from any of **Tables 2A-2G, Tables 8A-E, Tables 9A-9G, or Tables 10A-10C.**

In an embodiment, when the HSV-1 target position is the *UL48* gene coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, the targetuing domain of each guide RNA is independently selected from any of **Tables 3A-3G, Tables 11A-11E, Tables 12A-12G, or Tables 13A-13C.**

In an embodiment, when the HSV-1 target position is the *UL54* gene coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, the targetuing domain of each guide RNA is independently selected from any of **Tables 4A-4F, Tables 14A-14E, Tables 15A-15G, or Tables 16A-16C.**

In an embodiment, the gRNA, e.g., a gRNA comprising a targeting domain, which is complementary with the *UL19, UL30, UL48* or *UL54* gene, is a modular gRNA. In other embodiments, the gRNA is a unimolecular or chimeric gRNA.

In an embodiment, the targeting domain which is complementary with a target domain from the HSV-1 target position in the *UL19, UL30, UL48* or *UL54* gene is 16 nucleotides or more in length. In an embodiment, the targeting domain is 16 nucleotides in length. In an embodiment, the targeting domain is 17 nucleotides in length. In another embodiment, the targeting domain is 18 nucleotides in length. In still another embodiment, the targeting domain is 19 nucleotides in length. In still another embodiment, the targeting domain is 20 nucleotides in length. In still another embodiment, the targeting domain is 21 nucleotides in length. In still another embodiment, the targeting domain is 22 nucleotides in length. In still another embodiment, the targeting domain is 23 nucleotides in length. In still another embodiment, the targeting domain is 24 nucleotides in length. In still another embodiment, the targeting domain is 25 nucleotides in length. In still another embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule).

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In other embodiments, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In another aspect, disclosed herein is a nucleic acid, e.g., an isolated or non-naturally occurring nucleic acid, e.g., DNA, that comprises (a) a sequence that encodes a gRNA molecule comprising a targeting domain that is complementary with a target domain, e.g., with a HSV-1 target position in *UL19, UL30, UL48* or *UL54* gene as disclosed herein.

In an embodiment, the nucleic acid encodes a gRNA molecule, e.g., a first gRNA molecule, comprising a targeting domain configured to provide a cleavage event, e.g., a double strand break or a single strand break, sufficiently close to a HSV-1 target position in the *UL19, UL30, UL48* or *UL54* gene to allow alteration, e.g., alteration associated with NHEJ, of a HSV-1 target position in the *UL19, UL30, UL48* or *UL54* gene.

In an embodiment, the nucleic acid encodes a gRNA molecule, e.g., the first gRNA molecule, comprising a targeting domain comprising a sequence that is the same as, or differs by no more than 1, 2, 3, 4, or 5 nucleotides from, a targeting domain sequence from any one of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**. In an embodiment, the nucleic acid encodes a gRNA molecule comprising a targeting domain is selected from those in **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**.

In an embodiment, the nucleic acid encodes a modular gRNA, e.g., one or more nucleic acids encode a modular gRNA. In another embodiment, the nucleic acid encodes a chimeric gRNA. The nucleic acid may encode a gRNA, e.g., the first gRNA molecule, comprising a targeting domain comprising 16 nucleotides or more in length. In an embodiment, the nucleic acid encodes a gRNA, e.g., the first gRNA molecule, comprising a targeting domain that is 16 nucleotides in length. In another embodiment, the nucleic acid encodes a gRNA, e.g., the first gRNA molecule, comprising a targeting domain that is 17 nucleotides in length. In yet another embodiment, the nucleic acid encodes a gRNA, e.g., the first gRNA molecule, comprising a targeting domain that is 18 nucleotides in length. In still another embodiment, the nucleic acid encodes a gRNA, e.g., the first gRNA molecule, comprising a targeting domain that is 19 nucleotides in length. In still another embodiment, the nucleic acid encodes a gRNA, e.g., the first gRNA molecule, comprising a targeting domain that is 20 nucleotides in length. In still another embodiment, the nucleic acid encodes a gRNA, e.g., the first gRNA molecule, comprising a targeting domain that is 21 nucleotides in length. In still another embodiment, the nucleic acid encodes a gRNA, e.g., the first gRNA molecule, comprising a targeting domain that is 22 nucleotides in length. In still another embodiment, the nucleic acid encodes a gRNA, e.g., the first gRNA molecule, comprising a targeting domain that is 23 nucleotides in length. In still another embodiment, the nucleic acid encodes a gRNA, e.g., the first gRNA molecule, comprising a targeting domain that is 24 nucleotides in length. In still another embodiment, the nucleic acid encodes a gRNA, e.g., the first gRNA molecule, comprising a targeting domain that is 25 nucleotides in length. In still another embodiment, the nucleic acid encodes a gRNA, e.g., the first gRNA molecule, comprising a targeting domain that is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, a nucleic acid encodes a gRNA comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a gRNA e.g., the first gRNA molecule, comprising a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In an embodiment, a nucleic acid encodes a gRNA comprising e.g., the first gRNA molecule, a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

In an embodiment, a nucleic acid comprises (a) a sequence that encodes a gRNA molecule, e.g., the first gRNA molecule, comprising a targeting domain that is complementary with a target domain in the *UL19, UL30, UL48* or *UL54* gene as disclosed herein, and further comprising (b) a sequence that encodes a Cas9 molecule.

The Cas9 molecule may be a nickase molecule, an enzymatically activating Cas9 (eaCas9) molecule, e.g., an eaCas9 molecule that forms a double strand break in a target nucleic acid and/or an eaCas9 molecule that forms a single strand break in a target nucleic acid. In an embodiment, a single strand break is formed in the strand of the target nucleic acid to which the targeting domain of said gRNA is complementary. In another embodiment, a single strand break is formed in the strand of the target nucleic acid other than the strand to which to which the targeting domain of said gRNA is complementary.

In an embodiment, the eaCas9 molecule catalyzes a double strand break.

In an embodiment, the eaCas9 molecule comprises HNH-like domain cleavage activity but has no, or no significant, N-terminal RuvC-like domain cleavage activity. In another embodiment, the said eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In another embodiment, the eaCas9 molecule is an N-terminal RuvC-like domain nickase, e.g., the eaCas9 molecule comprises a mutation at H840, e.g., H840A. In another embodiment, the eaCas9 molecule is an N-terminal RuvC-like domain nickase, e.g., the eaCas9 molecule comprises a mutation at N863, e.g., N863A.

A nucleic acid disclosed herein may comprise (a) a sequence that encodes a gRNA molecule comprising a targeting domain that is complementary with a target domain in the *CCR5* gene as disclosed herein; (b) a sequence that encodes a Cas9 molecule.

A nucleic acid disclosed herein may comprise (a) a sequence that encodes a gRNA molecule comprising a targeting domain that is complementary with a target domain in the *UL19, UL30, UL48* or *UL54* gene as disclosed herein; (b) a sequence that encodes a Cas9 molecule; and further may comprise (c)(i) a sequence that encodes a second gRNA molecule described herein having a targeting domain that is complementary to a second target domain of the *UL19, UL30, UL48* or *UL54* gene, and optionally, (c)(ii) a sequence that encodes a third gRNA molecule described herein having a targeting domain that is complementary to a third target domain of the *UL19, UL30, UL48* or *UL54* gene; and optionally, (c)(iii) a sequence that encodes a fourth gRNA molecule described herein having a targeting domain that is complementary to a fourth target domain of the *UL19, UL30, UL48* or *UL54* gene.

In an embodiment, a nucleic acid encodes a second gRNA molecule comprising a targeting domain configured to provide a cleavage event, e.g., a double strand break or a single strand break, sufficiently close to a HSV-1 target position in the *UL19, UL30, UL48* or *UL54* gene, to allow alteration, e.g., alteration associated with NHEJ, of a HSV-1 target position in the *UL19, UL30, UL48* or *UL54* gene, either alone or in combination with the break positioned by said first gRNA molecule.

In an embodiment, a nucleic acid encodes a third gRNA molecule comprising a targeting domain configured to provide a cleavage event, e.g., a double strand break or a single strand break, sufficiently close to a HSV-1 target position in the *UL19, UL30, UL48* or *UL54* gene to allow alteration, e.g., alteration associated with NHEJ, of a HSV-1 target position in the *UL19, UL30, UL48* or *UL54* gene, either alone or in combination with the break positioned by the first and/or second gRNA molecule.

In an embodiment, a nucleic acid encodes a fourth gRNA molecule comprising a targeting domain configured to provide a cleavage event, e.g., a double strand break or a single strand break, sufficiently close to a HSV-1 target position in the *UL19, UL30, UL48* or *UL54* gene to allow alteration, e.g., alteration associated with NHEJ, of a HSV-1 target position in the *UL19, UL30, UL48* or *UL54* gene, either alone or in combination with the break positioned by the first gRNA molecule, the second gRNA molecule and/or the third gRNA molecule.

In an embodiment, the nucleic acid encodes a second gRNA molecule. The second gRNA is selected to target the same HSV-1 target position as the first gRNA molecule. Optionally, the nucleic acid may encode a third gRNA, and further optionally, the nucleic acid may encode a fourth gRNA molecule. The third gRNA molecule and the fourth gRNA molecule are selected to target the same HSV-1 target position as the first and second gRNA molecules.

In an embodiment, the nucleic acid encodes a second gRNA molecule comprising a targeting domain comprising a sequence that is the same as, or differs by no more than 1, 2, 3, 4, or 5 nucleotides from, a targeting domain sequence from one **of Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**. In an embodiment, the nucleic acid encodes a second gRNA molecule comprising a targeting domain selected from those in **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27.** In an embodiment, when a third or fourth gRNA molecule are present, the third and fourth gRNA molecules may independently comprise a targeting domain comprising a sequence that is the same as, or differs by no more than 1, 2, 3, 4, or 5 nucleotides from, a targeting domain sequence from one of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27.** In a further embodiment, when a third or fourth gRNA molecule are present, the third and fourth gRNA molecules may independently comprise a targeting domain selected from those in **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, Table 27.**

In an embodiment, the nucleic acid encodes a second gRNA which is a modular gRNA, e.g., wherein one or more nucleic acid molecules encode a modular gRNA. In another embodiment, the nucleic acid encoding a second gRNA is a chimeric gRNA. In another embodiment, when a nucleic acid encodes a third or fourth gRNA, the third and fourth gRNA may be a modular gRNA or a chimeric gRNA. When multiple gRNAs are used, any combination of modular or chimeric gRNAs may be used.

A nucleic acid may encode a second, a third, and/or a fourth gRNA, each independently, comprising a targeting domain comprising 16 nucleotides or more in length. In an embodiment, the nucleic acid encodes a second gRNA comprising a targeting domain that is 16 nucleotides in length. In another embodiment, the nucleic acid encodes a second gRNA comprising a targeting domain that is 17 nucleotides in length. In another embodiment, the nucleic acid encodes a second gRNA comprising a targeting domain that is 18 nucleotides in length. In still another embodiment, the nucleic acid encodes a second gRNA comprising a targeting domain that is 19 nucleotides in length. In still another embodiment, the nucleic acid encodes a second gRNA comprising a targeting domain that is 20 nucleotides in length. In still another embodiment, the nucleic acid encodes a second gRNA comprising a targeting domain that is 21 nucleotides in length. In still another embodiment, the nucleic acid encodes a second gRNA comprising a targeting domain that is 22 nucleotides in length. In still another embodiment, the nucleic acid encodes a second gRNA comprising a targeting domain that is 23 nucleotides in length. In still another embodiment, the nucleic acid encodes a second gRNA comprising a targeting domain that is 24 nucleotides in length. In still another embodiment, the nucleic acid encodes a second gRNA comprising a targeting domain that is 25 nucleotides in length. In still another embodiment, the nucleic acid encodes a second gRNA comprising a targeting domain that is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, a nucleic acid encodes a second, a third, and/or a fourth gRNA, each independently, comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a second, a third, and/or a fourth gRNA comprising a linking domain of no more than 25 nucleotides in length; a proximal and tail domain, that taken together, are at least 20 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 25 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 30 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 40 nucleotides in length; and a targeting domain equal to or greater than16, 17, 18, 19, 20, 21, 22, 23, 24, 25 or 26nucleotides in length.

In an embodiment, a nucleic acid encodes (a) a sequence that encodes a gRNA molecule comprising a targeting domain that is complementary with a target domain in the *UL19, UL30, UL48* or *UL54* gene, as disclosed herein, and (b) a sequence that encodes a Cas9 molecule, e.g., a Cas9 molecule described herein. In an embodiment, (a) and (b) are present on the same nucleic acid molecule, e.g., the same vector, e.g., the same viral vector, e.g., the same adeno-associated virus (AAV) vector. In an embodiment, the nucleic acid molecule is an AAV vector. Exemplary AAV vectors that may be used in any of the described compositions and methods include an AAV2 vector, a modified AAV2 vector, an AAV3 vector, a modified AAV3 vector, an AAV6 vector, a modified AAV6 vector, an AAV8 vector and an AAV9 vector.

In another embodiment, (a) is present on a first nucleic acid molecule, e.g. a first vector, e.g., a first viral vector, e.g., a first AAV vector; and (b) is present on a second nucleic acid molecule, e.g., a second vector, e.g., a second vector, e.g., a second AAV vector. The first and second nucleic acid molecules may be AAV vectors.

In another embodiment, a nucleic acid encodes (a) a sequence that encodes a gRNA molecule comprising a targeting domain that is complementary with a target domain in the *UL19, UL30, UL48* or *UL54* gene as disclosed herein, and (b) a sequence that encodes a Cas9 molecule, e.g., a Cas9 molecule described herein; andfurther comprises (c)(i) a sequence that encodes a second gRNA molecule as described herein, and optionally (c)(ii) a sequence that encodes a third gRNA molecule described herein having a targeting domain that is complementary to a third target domain of the *UL19, UL30, UL48* or *UL54* gene; and optionally, (c)(iii) a sequence that encodes a fourth gRNA molecule described herein having a targeting domain that is complementary to a fourth target domain of the *UL19, UL30, UL48* or *UL54* gene. In an embodiment, the nucleic acid comprises (a), (b) and (c)(i). In an embodiment, the nucleic acid comprises (a), (b), (c)(i) and (c)(ii). In an embodiment, the nucleic acid comprises (a), (b), (c)(i), (c)(ii) and (c)(iii). Each of (a) and (c)(i) may be present on the same nucleic acid molecule, e.g., the same vector, e.g., the same viral vector, e.g., the same adeno-associated virus (AAV) vector. In an embodiment, the nucleic acid molecule is an AAV vector.

In another embodiment, (a) and (c)(i) are on different vectors. For example, (a) may be present on a first nucleic acid molecule, e.g. a first vector, e.g., a first viral vector, e.g., a first AAV vector; and (c)(i) may be present on a second nucleic acid molecule, e.g., a second vector, e.g., a second vector, e.g., a second AAV vector. In an embodiment, the first and second nucleic acid molecules are AAV vectors.

In another embodiment, each of (a), (b), and (c)(i) are present on the same nucleic acid molecule, e.g., the same vector, e.g., the same viral vector, e.g., an AAV vector. In an embodiment, the nucleic acid molecule is an AAV vector. In an alternate embodiment, one of (a), (b), and (c)(i) is encoded on a first nucleic acid molecule, e.g., a first vector, e.g., a first viral vector, e.g., a first AAV vector; and a second and third of (a), (b), and (c)(i) is encoded on a second nucleic acid molecule, e.g., a second vector, e.g., a second vector, e.g., a second AAV vector. The first and second nucleic acid molecule may be AAV vectors.

In an embodiment, (a) is present on a first nucleic acid molecule, e.g., a first vector, e.g., a first viral vector, a first AAV vector; and (b) and (c)(i) are present on a second nucleic acid molecule, e.g., a second vector, e.g., a second vector, e.g., a second AAV vector. The first and second nucleic acid molecule may be AAV vectors.

In another embodiment, (b) is present on a first nucleic acid molecule, e.g., a first vector, e.g., a first viral vector, e.g., a first AAV vector; and (a) and (c)(i) are present on a second nucleic acid molecule, e.g., a second vector, e.g., a second vector, e.g., a second AAV vector. The first and second nucleic acid molecule may be AAV vectors.

In another embodiment, (c)(i) is present on a first nucleic acid molecule, e.g., a first vector, e.g., a first viral vector, e.g., a first AAV vector; and (b) and (a) are present on a second nucleic acid molecule, e.g., a second vector, e.g., a second vector, e.g., a second AAV vector. The first and second nucleic acid molecule may be AAV vectors.

In another embodiment, each of (a), (b) and (c)(i) are present on different nucleic acid molecules, e.g., different vectors, e.g., different viral vectors, e.g., different AAV vector. For example, (a) may be on a first nucleic acid molecule, (b) on a second nucleic acid molecule, and (c)(i) on a third nucleic acid molecule. The first, second and third nucleic acid molecule may be AAV vectors.

In another embodiment, when a third and/or fourth gRNA molecule are present, each of (a), (b), (c)(i), (c)(ii) and (c)(iii) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i), (c)(ii) and (c)(iii) may be present on the different nucleic acid molecules, e.g., different vectors, e.g., the different viral vectors, e.g., different AAV vectors. In a further embodiment, each of (a), (b), (c)(i), (c) (ii) and (c)(iii) may be present on more than one nucleic acid molecule, but fewer than five nucleic acid molecules, e.g., AAV vectors.

The nucleic acids described herein may comprise a promoter operably linked to the sequence that encodes the gRNA molecule of (a), e.g., a promoter described herein. The nucleic acid may further comprise a second promoter operably linked to the sequence that encodes the second, third and/or fourth gRNA molecule of (c), e.g., a promoter described herein. The promoter and second promoter differ from one another. In some embodiments, the promoter and second promoter are the same.

The nucleic acids described herein may further comprise a promoter operably linked to the sequence that encodes the Cas9 molecule of (b), e.g., a promoter described herein.

In another aspect, disclosed herein is a composition comprising (a) a gRNA molecule comprising a targeting domain that is complementary with a target domain in the *UL19, UL30, UL48* or *UL54* gene, as described herein. The composition of (a) may further comprise (b) a Cas9 molecule, e.g., a Cas9 molecule as described herein. A composition of (a) and (b) may further comprise (c) a second, third and/or fourth gRNA molecule, e.g., a second, third and/or fourth gRNA molecule described herein. In an embodiment, the composition is a pharmaceutical composition. The compositions described herein, e.g., pharmaceutical compositions described herein, can be used in the treatment or prevention of HSV-1 in a subject, e.g., in accordance with a method disclosed herein.

In another aspect, disclosed herein is a method of altering a cell, e.g., altering the structure, e.g., altering the sequence, of a target nucleic acid of a cell, comprising contacting said cell with: (a) a gRNA that targets the *UL19, UL30, UL48* or *UL54* gene, e.g., a gRNA as described herein; (b) a Cas9 molecule, e.g., a Cas9 molecule as described herein; and optionally, (c) a second, third and/or fourth gRNA that targets *UL19, UL30, UL48* or *UL54* gene, e.g., a second, third and/or fourth gRNA, as described herein.

In an embodiment, the method comprises contacting said cell with (a) and (b).

In an embodiment, the method comprises contacting said cell with (a), (b), and (c).

The targeting domain of the gRNA of (a) and optionally (c) may be selected from any of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27,** or a targeting domain of a gRNA that differs by no more than 1, 2, 3, 4, or 5 nucleotides from, a targeting domain sequence from any of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27.**

In an embodiment, the method comprises contacting a cell from a subject suffering from or likely to develop HSV-1. The cell may be from a subject that would benefit from having a mutation at a HSV-1 target position.

In an embodiment, the contacting step may be performed *in vivo.*

In an embodiment, the method of altering a cell as described herein comprises acquiring knowledge of the sequence of a HSV-1 target position in said cell, prior to the contacting step. Acquiring knowledge of the sequence of a HSV-1 target position in the cell may be by sequencing one or more of the *UL19, UL30, UL48* and/or *UL54* gene, or a portion of the *UL19, UL30, UL48* and/or *UL54* gene.

In an embodiment, the contacting step of the method comprises contacting the cell with a nucleic acid, e.g., a vector, e.g., an AAV vector, that expresses at least one of (a), (b), and (c). In an embodiment, the contacting step of the method comprises contacting the cell with a nucleic acid, e.g., a vector, e.g., an AAV vector, that expresses each of (a), (b), and (c). In another embodiment, the contacting step of the method comprises delivering to the cell a Cas9 molecule of (b) and a nucleic acid which encodes a gRNA of (a) and optionally, a second gRNA (c)(i) and further optionally, a third gRNA (c)(ii) and/or fourth gRNA (c)(iii).

In an embodiment, the contacting step of the method comprises contacting the cell with a nucleic acid, e.g., a vector, e.g., an AAV vector, that expresses at least one of (a), (b), (c) and (d). In some embodiments, the contacting step of the method comprises contacting the cell with a nucleic acid, e.g., a vector, e.g., an AAV vector, that expresses each of (a), (b), and (c). In another embodiment, the contacting step of the method comprises delivering to the cell a Cas9 molecule of (b), a nucleic acid which encodes a gRNA of (a) and a template nucleic acid of (d), and optionally, a second gRNA (c)(i) and further optionally, a third gRNA (c)(ii) and/or fourth gRNA (c)(iii).

In an embodiment, contacting comprises contacting the cell with a nucleic acid, e.g., a vector, e.g., an AAV vector, e.g., an AAV2 vector, a modified AAV2 vector, an AAV3 vector, a modified AAV3 vector, an AAV6 vector, a modified AAV6 vector, an AAV8 vector or an AAV9 vector, as described herein.

In an embodiment, contacting comprises delivering to the cell a Cas9 molecule of (b), as a protein or an mRNA, and a nucleic acid which encodes a gRNA of (a) and optionally a second, third and/or fourth gRNA of (c).

In an embodiment, contacting comprises delivering to the cell a Cas9 molecule of (b), as a protein or an mRNA, said gRNA of (a), as an RNA, and optionally said second, third and/or fourth gRNA of (c), as an RNA.

In an embodiment, contacting comprises delivering to the cell a gRNA of (a) as an RNA, optionally the second, third and/or fourth gRNA of (c) as an RNA, and a nucleic acid that encodes the Cas9 molecule of (b).

In another aspect, disclosed herein is a method of treating a subject suffering from or likely to develop HSV-1, e.g., altering the structure, e.g., sequence, of a target nucleic acid of the subject, comprising contacting the subject (or a cell from the subject) with:
(a) a gRNA that targets the *UL19, UL30, UL48* or *UL54* gene, e.g., a gRNA disclosed herein;
(b) a Cas9 molecule, e.g., a Cas9 molecule disclosed herein; and
optionally, (c)(i) a second gRNA that targets the *UL19, UL30, UL48* or *UL54* gene, e.g., a second gRNA disclosed herein, and
further optionally, (c)(ii) a third gRNA, and still further optionally, (c)(iii) a fourth gRNA that target the *UL19, UL30, UL48* or *UL54* gene, e.g., a third and fourth gRNA disclosed herein.

In some embodiments, contacting comprises contacting with (a) and (b).

In some embodiments, contacting comprises contacting with (a), (b), and (c)(i).

In some embodiments, contacting comprises contacting with (a), (b), (c)(i) and (c)(ii).

In some embodiments, contacting comprises contacting with (a), (b), (c)(i), (c)(ii) and (c)(iii).

The targeting domain of the gRNA of (a) or (c) (e.g., (c)(i), (c)(ii), or (c)(iii)) may be selected from any of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**, or a targeting domain of a gRNA that differs by no more than 1, 2, 3, 4, or 5 nucleotides from, a targeting domain sequence from any of **Tables 1A-1G, Tables 2A-2G, Tables 3A-3G, Tables 4A-4F, Tables 5A-5E,** T**ables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**.

In an embodiment, the method comprises acquiring knowledge of the sequence at a HSV-1 target position in said subject.

In an embodiment, the method comprises acquiring knowledge of the sequence at a HSV-1 target position in said subject by sequencing one or more of the *UL19, UL30, UL48* and/or *UL54* gene(s) or a portion of the *UL19, UL30, UL48* and/or *UL54* gene.

In an embodiment, the method comprises introducing a mutation at a HSV-1 target position.

In an embodiment, the method comprises introducing a mutation at a HSV-1 target position by NHEJ.

In an embodiment, a cell of the subject is contacted is *in vivo* with (a), (b) and optionally (c)(i), further optionally (c)(ii), and still further optionally (c)(iii).

In an embodiment, the cell of the subject is contacted *in vivo* by intravenous delivery of (a), (b), and optionally (c)(i), further optionally (c)(ii), and still further optionally (c)(iii).

In an embodiment, the contacting step comprises contacting the subject with a nucleic acid, e.g., a vector, e.g., an AAV vector, described herein, e.g., a nucleic acid that encodes at least one of (a), (b), and optionally (c)(i), further optionally (c)(ii), and still further optionally (c)(iii).

In an embodiment, the contacting step comprises delivering to said subject said Cas9 molecule of (b), as a protein or mRNA, and a nucleic acid which encodes (a), and optionally (c)(i), further optionally (c)(ii), and still further optionally (c)(iii).

In an embodiment, the contacting step comprises delivering to the subject the Cas9 molecule of (b), as a protein or mRNA, the gRNA of (a), as an RNA, and optionally the second gRNA of (c)(i), further optionally said third gRNA of (c)(ii), and still further optionally said fourth gRNA of (c)(iii), as an RNA.

In an embodiment, the contacting step comprises delivering to the subject the gRNA of (a), as an RNA, optionally said second gRNA of (c)(i), further optionally said third gRNA of (c)(ii), and still further optionally said fourth gRNA of (c)(iii), as an RNA, a nucleic acid that encodes the Cas9 molecule of (b).

When the method comprises (1) introducing a mutation at a HSV-1 target position by NHEJ or (2) knocking down expression of one or more of the *UL19, UL30, UL48* and/or *UL54* gene(s), e.g., by targeting the promoter region, a Cas9 molecule of (b) and at least one guide RNA, e.g., a guide RNA of (a) are included in the contacting step.

In an embodiment, a cell of the subject is contacted is *in vivo* with (a), (b) and optionally (c)(i), further optionally (c)(ii), and still further optionally (c)(iii). In an embodiment, the cell of the subject is contacted *in vivo* by intravenous delivery of (a), (b) and optionally (c)(i), further optionally (c)(ii), and still further optionally (c)(iii).

In an embodiment, the contacting step comprises contacting the subject with a nucleic acid, e.g., a vector, e.g., an AAV vector, described herein, e.g., a nucleic acid that encodes at least one of (a), (b), and optionally (c)(i), further optionally (c)(ii), and still further optionally (c)(iii).

In an embodiment, the contacting step comprises delivering to said subject said Cas9 molecule of (b), as a protein or mRNA, and a nucleic acid which encodes (a) and optionally (c)(i), further optionally (c)(ii), and still further optionally (c)(iii).

In an embodiment, the contacting step comprises delivering to the subject the Cas9 molecule of (b), as a protein or mRNA, the gRNA of (a), as an RNA, and optionally the second gRNA of (c)(i), further optionally said third gRNA of (c)(ii), and still further optionally said fourth gRNA of (c)(iii), as an RNA.

In an embodiment, the contacting step comprises delivering to the subject the gRNA of (a), as an RNA, optionally said second gRNA of (c)(i), further optionally said third gRNA of (c)(ii), and still further optionally said fourth gRNA of (c)(iii), as an RNA, and a nucleic acid that encodes the Cas9 molecule of (b).

In another aspect, disclosed herein is a reaction mixture comprising a gRNA molecule, a nucleic acid, or a composition described herein, and a cell, e.g., a cell from a subject having, or likely to develop HSV-1, or a subject which would benefit from a mutation at a HSV-1 target position.

In another aspect, disclosed herein is a kit comprising, (a) a gRNA molecule described herein, or nucleic acid that encodes the gRNA, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein or a nucleic acid that encodes (c)(i);
(c)(ii) a third gRNA molecule, e.g., a second gRNA molecule described herein or a nucleic acid that encodes (c)(ii);
(c)(iii) a fourth gRNA molecule, e.g., a second gRNA molecule described herein or a nucleic acid that encodes (c)(iii).

In an embodiment, the kit comprises nucleic acid, e.g., an AAV vector, that encodes one or more of (a), (b), (c)(i), (c)(ii), and (c)(iii).

In yet another aspect, disclosed herein is a gRNA molecule, e.g., a gRNA molecule described herein, for use in treating, or delaying the onset or progression of HSV-1 infection in a subject, e.g., in accordance with a method of treating, or delaying the onset or progression of HSV-1 infection as described herein.

In an embodiment, the gRNA molecule is used in combination with a Cas9 molecule, e.g., a Cas9 molecule described herein. Additionally or alternatively, in an embodiment, the gRNA molecule is used in combination with a second, third and/or fouth gRNA molecule, e.g., a second, third and/or fouth gRNA molecule described herein.

In still another aspect, disclosed herein is use of a gRNA molecule, e.g., a gRNA molecule described herein, in the manufacture of a medicament for treating, or delaying the onset or progression of HSV-1 in a subject, e.g., in accordance with a method of treating, or delaying the onset or progression of HSV-1 as described herein.

In an embodiment, the medicament comprises a Cas9 molecule, e.g., a Cas9 molecule described herein. Additionaly or alternatively, in an embodiment, the medicament comprises a second, third and/or fouth gRNA molecule, e.g., a second, third and/or fouth gRNA molecule described herein.

The gRNA molecules and methods, as disclosed herein, can be used in combination with a governing gRNA molecule. As used herein, a governing gRNA molecule refers to a gRNA molecule comprising a targeting domain which is complementary to a target domain on a nucleic acid that encodes a component of the CRISPR/Cas system introduced into a cell or subject. For example, the methods described herein can further include contacting a cell or subject with a governing gRNA molecule or a nucleic acid encoding a governing molecule. In an embodiment, the governing gRNA molecule targets a nucleic acid that encodes a Cas9 molecule or a nucleic acid that encodes a target gene gRNA molecule. In an embodiment, the governing gRNA comprises a targeting domain that is complementary to a target domain in a sequence that encodes a Cas9 component, e.g., a Cas9 molecule or target gene gRNA molecule. In an embodiment, the target domain is designed with, or has, minimal homology to other nucleic acid sequences in the cell, e.g., to minimize off-target cleavage. For example, the targeting domain on the governing gRNA can be selected to reduce or minimize off-target effects. In an embodiment, a target domain for a governing gRNA can be disposed in the control or coding region of a Cas9 molecule or disposed between a control region and a transcribed region. In an embodiment, a target domain for a governing gRNA can be disposed in the control or coding region of a target gene gRNA molecule or disposed between a control region and a transcribed region for a target gene gRNA. While not wishing to be bound by theory, in an embodiment, it is believed that altering, e.g., inactivating, a nucleic acid that encodes a Cas9 molecule or a nucleic acid that encodes a target gene gRNA molecule can be effected by cleavage of the targeted nucleic acid sequence or by binding of a Cas9 molecule/governing gRNA molecule complex to the targeted nucleic acid sequence.

The compositions, reaction mixtures and kits, as disclosed herein, can also include a governing gRNA molecule, e.g., a governing gRNA molecule disclosed herein.

Unless otherwise defined, all technical and scientific terms used herein have the same meaning as commonly understood by one of ordinary skill in the art to which this invention belongs. In addition, the materials, methods, and examples are illustrative only and not intended to be limiting.

Headings, including numeric and alphabetical headings and subheadings, are for organization and presentation and are not intended to be limiting.

Other features and advantages of the invention will be apparent from the detailed description, drawings, and from the claims.

### BRIEF DESCRIPTION OF THE DRAWINGS

**Figs. 1A-1I** are representations of several exemplary gRNAs.
**Fig. 1A** depicts a modular gRNA molecule derived in part (or modeled on a sequence in part) from *Streptococcus pyogenes (S. pyogenes)* as a duplexed structure (SEQ ID NOS: 42 and 43, respectively, in order of appearance);
**Fig. 1B** depicts a unimolecular (or chimeric) gRNA molecule derived in part from *S*. *pyogenes* as a duplexed structure (SEQ ID NO: 44);
**Fig. 1C** depicts a unimolecular gRNA molecule derived in part from *S. pyogenes* as a duplexed structure (SEQ ID NO: 45);
**Fig. ID** depicts a unimolecular gRNA molecule derived in part from *S. pyogenes* as a duplexed structure (SEQ ID NO: 46);
**Fig. 1E** depicts a unimolecular gRNA molecule derived in part from *S. pyogenes* as a duplexed structure (SEQ ID NO: 47);
**Fig. 1F** depicts a modular gRNA molecule derived in part from *Streptococcus thermophilus (S. thermophilus)* as a duplexed structure (SEQ ID NOS: 48 and 49, respectively, in order of appearance);
**Fig. 1G** depicts an alignment of modular gRNA molecules of *S. pyogenes* and *S*. *thermophilus* (SEQ ID NOS: 50-53, respectively, in order of appearance).
**Figs. 1H-1I** depicts additional exemplary structures of unimolecular gRNA molecules. **Fig. 1H** shows an exemplary structure of a unimolecular gRNA molecule derived in part from *S. pyogenes* as a duplexed structure (SEQ ID NO: 45). **Fig. 1I** shows an exemplary structure of a unimolecular gRNA molecule derived in part from *S. aureus* as a duplexed structure (SEQ ID NO: 40).

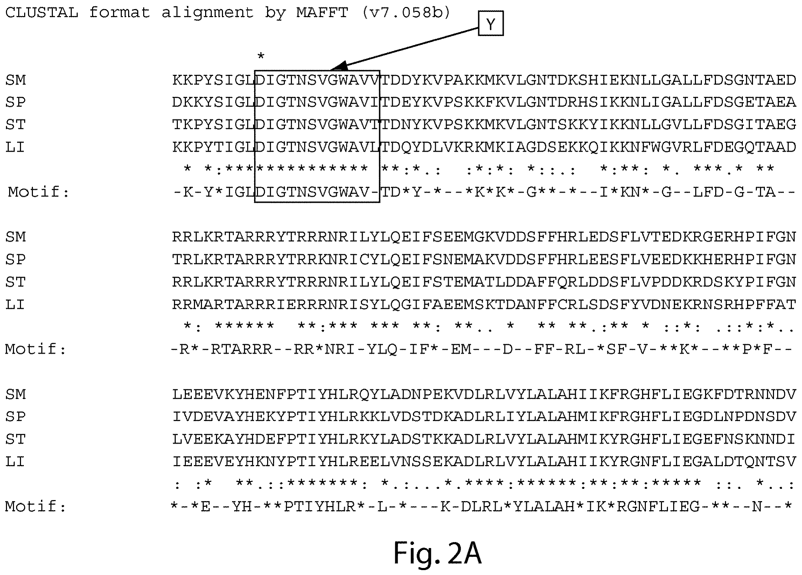

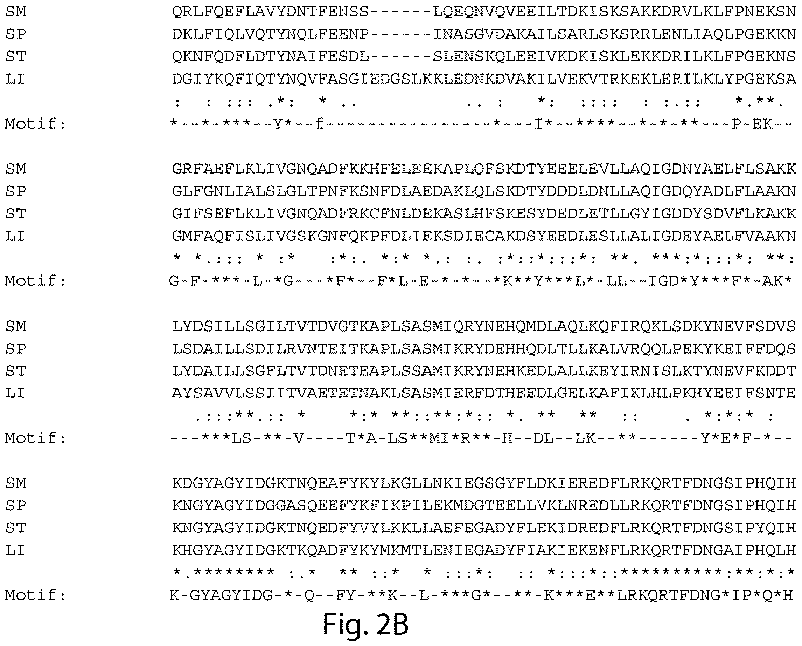

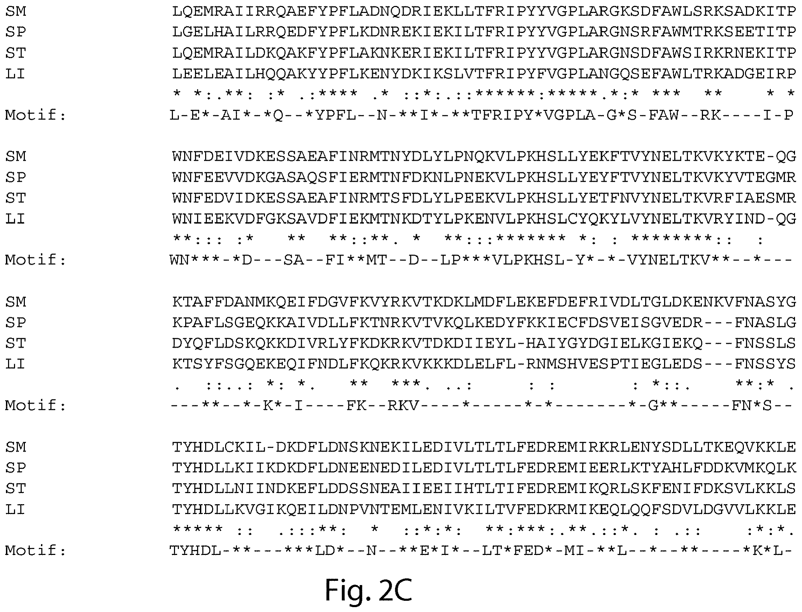

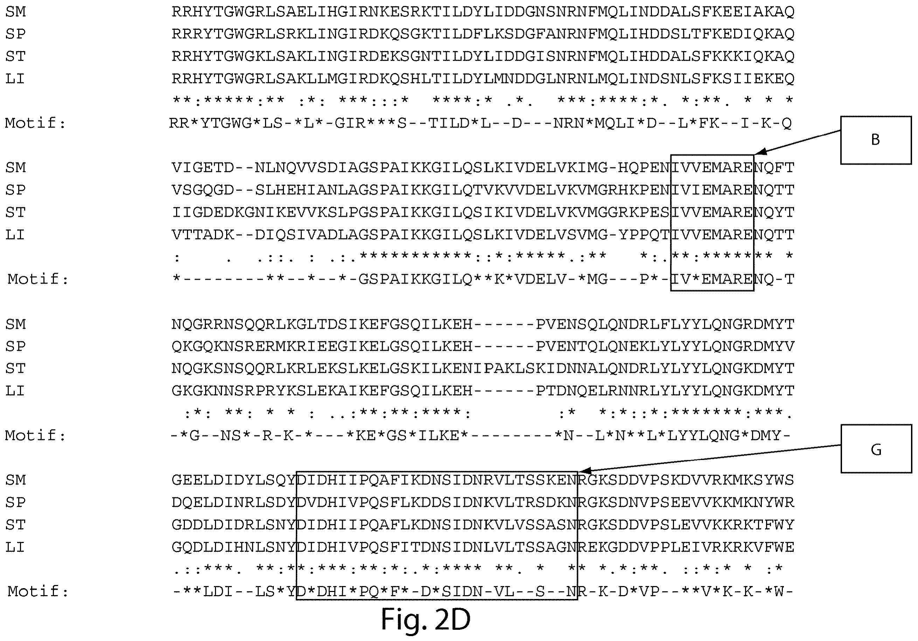

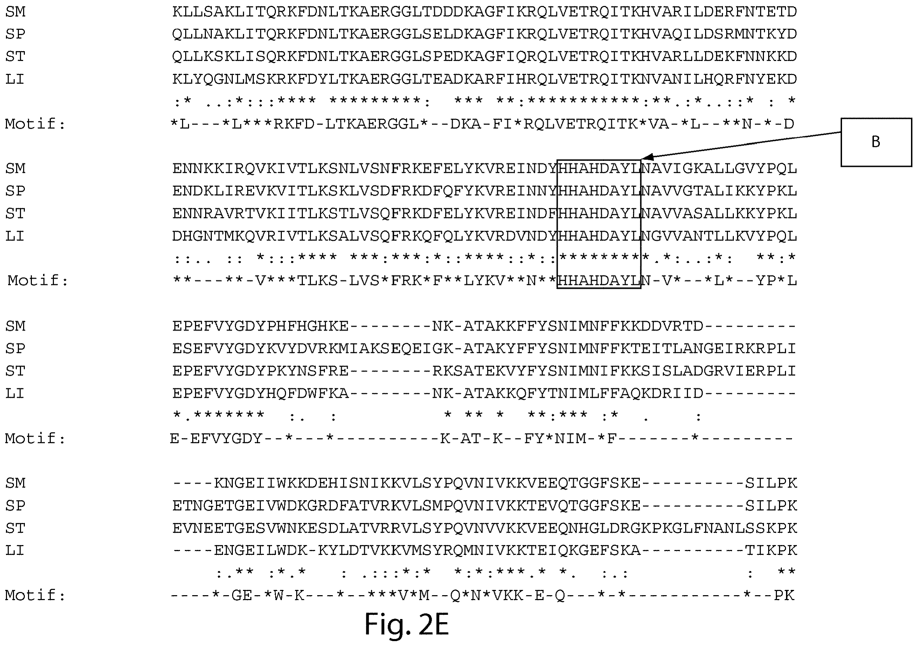

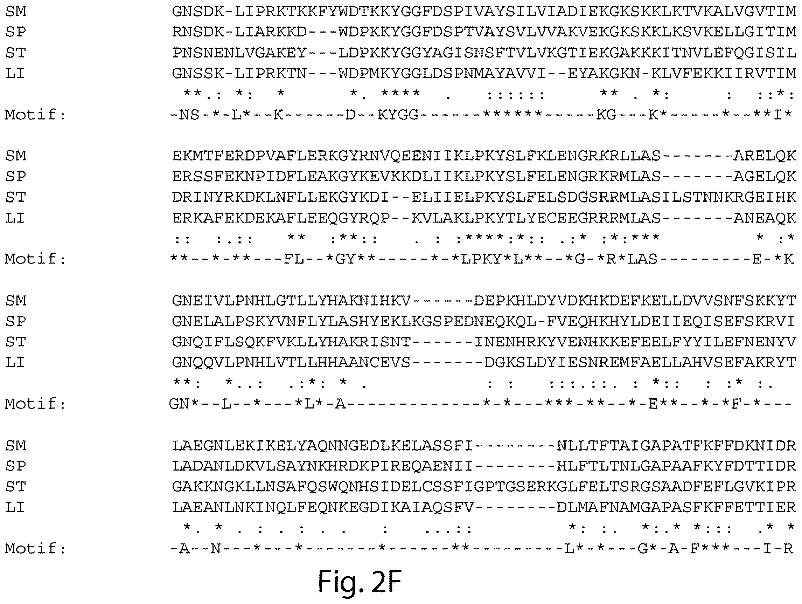

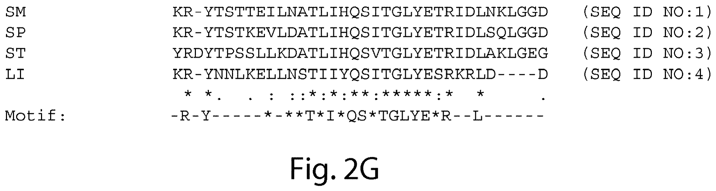

**Figs. 2A-2G** depict an alignment of Cas9 sequences from Chylinski et al. (RNA Biol. 2013; 10(5): 726-737). The N-terminal RuvC-like domain is boxed and indicated with a "Y". The other two RuvC-like domains are boxed and indicated with a "B". The HNH-like domain is boxed and indicated by a "G". Sm: *S. mutans* (SEQ ID NO: 1); Sp: *S. pyogenes* (SEQ ID NO: 2); St: *S. thermophilus* (SEQ ID NO: 3); Li: *L. innocua* (SEQ ID NO: 4). Motif: this is a motif based on the four sequences: residues conserved in all four sequences are indicated by single letter amino acid abbreviation; "^{∗}" indicates any amino acid found in the corresponding position of any of the four sequences; and "-" indicates any amino acid, e.g., any of the 20 naturally occurring amino acids, or absent.
**Figs. 3A-3B** show an alignment of the N-terminal RuvC-like domain from the Cas9 molecules disclosed in Chylinski *et al* (SEQ ID NOS: 54-103, respectively, in order of appearance). The last line of **Fig. 3B** identifies 4 highly conserved residues.
**Figs. 4A-4B** show an alignment of the N-terminal RuvC-like domain from the Cas9 molecules disclosed in Chylinski *et al.* with sequence outliers removed (SEQ ID NOS: 104-177, respectively, in order of appearance). The last line of **Fig. 4B** identifies 3 highly conserved residues.
**Figs. 5A-5C** show an alignment of the HNH-like domain from the Cas9 molecules disclosed in Chylinski *et al* (SEQ ID NOS: 178-252, respectively, in order of appearance). The last line of **Fig. 5C** identifies conserved residues.
**Figs. 6A-6B** show an alignment of the HNH-like domain from the Cas9 molecules disclosed in Chylinski *et al.* with sequence outliers removed (SEQ ID NOS: 253-302, respectively, in order of appearance). The last line of **Fig. 6B** identifies 3 highly conserved residues.

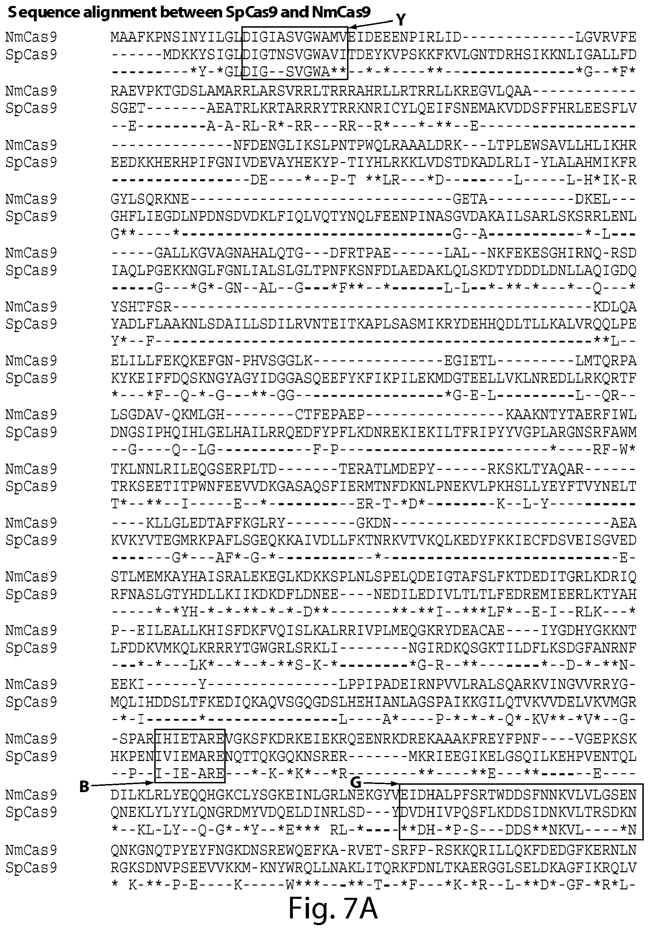

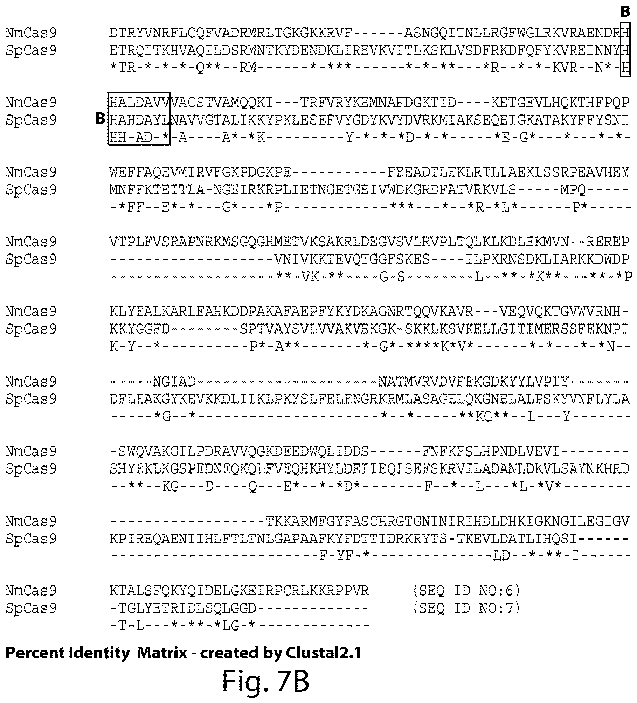

**Figs. 7A-7B** depict an alignment of Cas9 sequences from *S. pyogenes* and *Neisseria meningitidis (N. meningitidis).* The N-terminal RuvC-like domain is boxed and indicated with a "Y". The other two RuvC-like domains are boxed and indicated with a "B". The HNH-like domain is boxed and indicated with a "G". Sp: *S. pyogenes;* Nm: *N. meningitidis.* Motif: this is a motif based on the two sequences: residues conserved in both sequences are indicated by a single amino acid designation; "^{∗}" indicates any amino acid found in the corresponding position of any of the two sequences; "-" indicates any amino acid, e.g., any of the 20 naturally occurring amino acids, and "-" indicates any amino acid, e.g., any of the 20 naturally occurring amino acids, or absent.
**Fig. 8** shows a nucleic acid sequence encoding Cas9 of *N. meningitidis* (SEQ ID NO: 303). Sequence indicated by an "R" is an SV40 NLS; sequence indicated as "G" is an HA tag; and sequence indicated by an "O" is a synthetic NLS sequence; the remaining (unmarked) sequence is the open reading frame (ORF).
**Figs. 9A** and **9B** are schematic representations of the domain organization of *S*. *pyogenes* Cas 9. **Fig. 9A** shows the organization of the Cas9 domains, including amino acid positions, in reference to the two lobes of Cas9 (recognition (REC) and nuclease (NUC) lobes). **Fig. 9B** shows the percent homology of each domain across 83 Cas9 orthologs.
**Fig. 10A** is a schematic showing the plasmid map for the reporter plasmid, pAF025.
**Fig. 10B** is a graph showing the decrease in fluorescence from green fluorescent protein (GFP) in cells transfected with various gRNAs that target HSV-1 target sequences.

### DETAILED DESCRIPTION

### Definitions

"Domain", as used herein, is used to describe segments of a protein or nucleic acid. Unless otherwise indicated, a domain is not required to have any specific functional property.

Calculations of homology or sequence identity between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Governing gRNA molecule", as used herein, refers to a gRNA molecule that comprises a targeting domain that is complementary to a target domain on a nucleic acid that comprises a sequence that encodes a component of the CRISPR/Cas system that is introduced into a cell or subject. A governing gRNA does not target an endogenous cell or subject sequence. In an embodiment, a governing gRNA molecule comprises a targeting domain that is complementary with a target sequence on: (a) a nucleic acid that encodes a Cas9 molecule; (b) a nucleic acid that encodes a gRNA which comprises a targeting domain that targets the *UL19, UL30, UL48* or *UL54* gene (a target gene gRNA); or on more than one nucleic acid that encodes a CRISPR/Cas component, e.g., both (a) and (b). In an embodiment, a nucleic acid molecule that encodes a CRISPR/Cas component, e.g., that encodes a Cas9 molecule or a target gene gRNA, comprises more than one target domain that is complementary with a governing gRNA targeting domain. While not wishing to be bound by theory, in an embodiment, it is believed that a governing gRNA molecule complexes with a Cas9 molecule and results in Cas9 mediated inactivation of the targeted nucleic acid, e.g., by cleavage or by binding to the nucleic acid, and results in cessation or reduction of the production of a CRISPR/Cas system component. In an embodiment, the Cas9 molecule forms two complexes: a complex comprising a Cas9 molecule with a target gene gRNA, which complex will alter the *UL19, UL30, UL48* or *UL54* gene; and a complex comprising a Cas9 molecule with a governing gRNA molecule, which complex will act to prevent further production of a CRISPR/Cas system component, e.g., a Cas9 molecule or a target gene gRNA molecule. In an embodiment, a governing gRNA molecule/Cas9 molecule complex binds to or promotes cleavage of a control region sequence, e.g., a promoter, operably linked to a sequence that encodes a Cas9 molecule, a sequence that encodes a transcribed region, an exon, or an intron, for the Cas9 molecule. In an embodiment, a governing gRNA molecule/Cas9 molecule complex binds to or promotes cleavage of a control region sequence, e.g., a promoter, operably linked to a gRNA molecule, or a sequence that encodes the gRNA molecule. In an embodiment, the governing gRNA, e.g., a Cas9-targeting governing gRNA molecule, or a target gene gRNA-targeting governing gRNA molecule, limits the effect of the Cas9 molecule/target gene gRNA molecule complex-mediated gene targeting. In an embodiment, a governing gRNA places temporal, level of expression, or other limits, on activity of the Cas9 molecule/target gene gRNA molecule complex. In an embodiment, a governing gRNA reduces off-target or other unwanted activity. In an embodiment, a governing gRNA molecule inhibits, e.g., entirely or substantially entirely inhibits, the production of a component of the Cas9 system and thereby limits, or governs, its activity.

"Modulator", as used herein, refers to an entity, e.g., a drug, that can alter the activity (e.g., enzymatic activity, transcriptional activity, or translational activity), amount, distribution, or structure of a subject molecule or genetic sequence. In an embodiment, modulation comprises cleavage, e.g., breaking of a covalent or non-covalent bond, or the forming of a covalent or non-covalent bond, e.g., the attachment of a moiety, to the subject molecule. In an embodiment, a modulator alters the, three dimensional, secondary, tertiary, or quaternary structure, of a subject molecule. A modulator can increase, decrease, initiate, or eliminate a subject activity.

"Large molecule", as used herein, refers to a molecule having a molecular weight of at least 2, 3, 5, 10, 20, 30, 40, 50, 60, 70, 80, 90, or 100 kD. Large molecules include proteins, polypeptides, nucleic acids, biologics, and carbohydrates.

"Polypeptide", as used herein, refers to a polymer of amino acids having less than 100 amino acid residues. In an embodiment, it has less than 50, 20, or 10 amino acid residues.

"Reference molecule", e.g., a reference Cas9 molecule or reference gRNA, as used herein, refers to a molecule to which a subject molecule, e.g., a subject Cas9 molecule of subject gRNA molecule, e.g., a modified or candidate Cas9 molecule is compared. For example, a Cas9 molecule can be characterized as having no more than 10% of the nuclease activity of a reference Cas9 molecule. Examples of reference Cas9 molecules include naturally occurring unmodified Cas9 molecules, e.g., a naturally occurring Cas9 molecule such as a Cas9 molecule of *S. pyogenes, S. aureus* or *S. thermophilus.* In an embodiment, the reference Cas9 molecule is the naturally occurring Cas9 molecule having the closest sequence identity or homology with the Cas9 molecule to which it is being compared. In an embodiment, the reference Cas9 molecule is a sequence, e.g., a naturally occurring or known sequence, which is the parental form on which a change, e.g., a mutation has been made.

"Replacement", or "replaced", as used herein with reference to a modification of a molecule does not require a process limitation but merely indicates that the replacement entity is present.

"Small molecule", as used herein, refers to a compound having a molecular weight less than about 2 kD, e.g., less than about 2 kD, less than about 1.5 kD, less than about 1 kD, or less than about 0.75 kD.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human. In other embodiments, the subject is poultry.

"Treat", "treating" and "treatment", as used herein, mean the treatment of a disease in a mammal, e.g., in a human, including (a) inhibiting the disease, i.e., arresting or preventing its development; (b) relieving the disease, i.e., causing regression of the disease state; and (c) curing the disease.

"Prevent", "preventing" and "prevention", as used herein, means the prevention of a disease in a mammal, e.g., in a human, including (a) avoiding or precluding the disease; (2) affecting the predisposition toward the disease, e.g., preventing at least one symptom of the disease or to delay onset of at least one symptom of the disease.

"X" as used herein in the context of an amino acid sequence, refers to any amino acid (e.g., any of the twenty natural amino acids) unless otherwise specified.

### Herpes simplex virus type 1

Herpes simplex virus type 1 (HSV-1) causes intermittent sores of the mouth and mucous membranes. It is a ubiquitous and highly contagious pathogen. Most subjects develop the infection during childhood. By adulthood, up to 80% of the population in the United States is infected with HSV-1. Initial infection with HSV-1 generally causes painful blistering of the mucous membranes of the lips and mouth.

HSV-1 infection persists for the lifetime of the host. Primary and re-activation infections can cause permanent neurologic sequelae and blindness. There is a considerable need for methods to treat and prevent HSV1 infections.

During primary infection, the virus most often infects cells of the oropharynx and ano-genital region, causing painful vesicles in the affected region. Re-activation of HSV-1 infections most often occurs in the oropharynx and ano-genital region. However, re-activation infections of the eye and central nervous system are the most severe and damaging HSV manifestations, as they can lead to blindness and permanent neurologic disability, respectively.

HSV-1 is contained within an icosahedral particle. The virus enters the host via infection of epithelial cells within the skin and mucous membranes. The virus produces immediate early genes within the epithelial cells, which encode enzymes and binding proteins necessary for viral synthesis. After primary infection, the virus travels up sensory nerve axons via retrograde transport to the sensory dorsal root ganglion (DRG). Within the DRG, it establishes a latent infection. The latent infection persists for the lifetime of the host. Within the DRG cell, the virus uncoats, viral DNA is transported into the nucleus, and key viral RNAs associated with latency are transcribed (including the LAT RNAs).

Host immune defense is very important to combating HSV infection. CD4+ T-cells and CD8+ cells are responsible for recognizing and clearing the pathogen. Subjects with impaired T-cell responses, including those with HIV, those receiving immunosuppressants following organ transplants, and neonates with developing immune systems, are subject to the most severe manifestations of HSV-1 infections.

During the primary infection, subjects generally experience painful blistering in the oral or ano-genital region that lasts 4-15 days. The sores most commonly involve the lips, gums and nasal mucous membranes. HSV-1 primary infections may also involve the ano-genital region, including the vagina, labia, cervix, penis, scrotum, anus and skin around the thighs. Less commonly, HSV-1 primary infection may involve the eyes, central nervous system, the fingers and fingernail beds (herpetic whitlow). The infection is transmitted primarily through saliva and/or sexual activity. The blisters may break, releasing clear fluid that is highly infectious. Primary infection is often accompanied by a flu-like illness, including fever, chills and muscle aches.

Reactivations of latent infections are generally less severe and may be of shorter duration. Reactivation can affect the oral region, the ano-genital region, the eye, the central nervous system (CNS), the fingernails, and the pharynx. Reactivation generally affects the oral region but can also affect other mucous membranes, including those of the ano-genital area, fingernails, and the pharynx. Ophthalmologic disease may also occur, including epithelial keratitis, stromal keratitis and disciform keratitis. Generally, ophthalmologic manifestations of HSV-1 are self-limiting. However, HSV-1 keratitis may, in rare instances, cause scarring, secondary infection with bacterial pathogens and rarely, blindness.

In some cases, reactivation can occur in the central nervous system (CNS) via retrograde transport of the virus into the CNS. Generally, patients who are immune compromised develop HSV-1 induced encephalitis and/or meningitis. HSV-1 encephalitis or meningitis are both extremely severe. Subjects generally experience permanent neurologic damage in spite of treatment with antiviral therapy.

Primary infections and reactivation infections in the CNS, called encephalitis and meningitis, are particularly damaging. In a study of infants with HSV-encephalitis or meningitis treated with high dose antiviral therapy, there was found to be a 4% mortality rate and 69% of survivors had permanent neurologic sequelae (Kimberlin et al., Pediatrics. 2001;108: 230-238). The majority of infants and adults who develop HSV-1 encephalitis or meningitis will experience permanent neurologic damage in spite of treatment with antiviral therapy. Reactivation infections in the CNS occur via anterograde transport of the virus into the CNS. Most commonly, patients who are immune compromised or infants develop HSV-1 induced encephalitis and/or meningitis. Healthy adults may more rarely develop HSV-1 disease of the CNS.

Reactivation infections occur in the eye via anterograde transport of the virus into the eye from the trigeminal ganglion, along the ophthalmic branch of the trigeminal nerve (the fifth cranial nerve) and into the eye. Re-activation of the virus may also occur from within the cornea.. Latency within the trigeminal ganglion is established via one of two mechanisms. First, HSV-1 can travel via retrograde transport along the trigeminal nerve from the eye (after an eye infection) into the trigeminal ganglion. Alternatively, it can spread to the trigeminal ganglion via hematogenous spread following infection of the oral mucosa, genital region, or other extraocular site. After establishing latent infection of the trigeminal ganglion, at any time, particularly in the event of an immunocompromised host, the virus can re-establish infection by traveling anterograde along the trigeminal nerve and into the eye.

Ocular herpes can affect the anterior chamber of the eye, where it causes keratitis, or the posterior chamber, where it causes retinitis. In adults, HSV-1 is responsible for the majority of cases of HSV-retinitis (Pepose et al., Ocular Infection and Immunity 1996; Mosby 1155-1168). HSV-1 retinitis can lead to acute retinal necrosis (ARN), which will destroy the retina within 2 weeks without treatment (Banerjee and Rouse, Human Herpesviruses 2007; Cambridge University Press, Chapter 35). Even with treatment, the risk of permanent visual damage following ARN is higher than 50% (Roy et al., Ocular Immunology and Inflammation 2014; 22(3):170-174).

Keratitis is the most common form of ocular herpes. HSV keratitis can manifest as dentritic keratitis, stromal keratitis, blepharatis and conjunctivitis. HSV-1 is responsible for the majority of HSV-associated keratitis, accounting for 58% of cases (Dawson et. al., Suvey of Ophthalmology 1976; 21(2): 121-135). In the United States, there are approximately 48,000 cases of recurrent or primary HSV-related keratitis infections annually (Liesegang et. al., 1989; 107(8): 1155-1159). Of all cases of HSV-related keratitis, approximately 1.5-3% of subjects experience severe, permanent visual impairment (Wilhelmus et. al., Archives of Ophthalmology 1981; 99(9): 1578-82).

Overall, stromal keratitis represents approximately 15% of keratitis cases and is associated with the highest risk of permanent visual damage. Stromal keratitis results in scarring and irregular astimagtism. Previous ocular HSV infection increases the risk for developing stromal infection, which means that subjects who have had a prior ocular HSV infection have an increased risk for permanent visual damage on reactivation. In children, stromal keratitis represents up to 60% of all keratitis cases so children are particularly at risk for permanent visual damage from HSV-associated keratitis. A retrospective study in the United States from 1950-1982 found that there are approximately 2.6 new or recurrent stromal keratitis cases per 100,000 person years, or approximately 8,000 cases of stromal keratitis annually (Liesegang et. al., 1989; 107(8): 1155-1159). A more recent study in France in 2002 estimated the incidence of new or recurrent stromal keratitis cases to be 9.6 per 100,000 (Labetoulle et al., Ophthalmology 2005; 112(5):888-895). The incidence of HSV-associated keratitis may be increasing in the developed world (Farooq and Shukla 2012; Survey of Ophthalmology 57(5): 448-462).

The compositions and methods described herein can be used for the treatment and prevention of HSV-1 ocular infections, including but not limited to HSV-1 stromal keratitis, HSV-1 retinitis, HSV-1 encephalitis and HSV-1 meningitis.

Newborns are a population at particular risk for developing severe HSV-1 infections. The disease is transmitted from the mother to the fetus during childbirth. The chance of maternal-fetal transmission is highest in cases where the mother developed primary HSV infection during pregnancy. The incidence of neonatal herpes is approximately 4-30 per 100,000 births. Neonates may develop severe HSV-1 encephalitis and/or meningitis. In spite of prompt treatment with antiviral therapy, the rate of permanent neurologic sequelae in newborns infected with HSV-1 is significant.

Primary HSV-1 infections may be treated with antiviral therapy, including acyclovir, valacyclovir and famciclovir. These therapies have been demonstrated to reduce viral shedding, decrease pain and improve healing time of lesions. Re-activation of latent infections may resolve without treatment (it may be self-limiting) or may be treated with anti-viral therapy. Therapy is primarily given during acute infection. There are no curative or preventative treatments. Therapy may be given prophylactically in certain situations, including during childbirth in a mother with a recent HSV-1 infection or reactivation.

### Methods to Treat, Prevent or Reduce HSV-1 Infection

Disclosed herein are the approaches to treat, prevent, or reduce HSV-1 infection, using the compositions and methods described herein.

HSV-1 relies on the genes *UL19, UL30, UL48* and/or *UL54* for infection, proliferation and assembly. Knockout of any of these genes individually or in combination can prevent or treat HSV-1 infections. As the HSV-1 virus establishes latency in discrete, localized regions within the body, local delivery that delivers a treatment in the region of latency can be used. Targeting knockout to a discrete region or regions (e.g., the trigeminal dorsal root ganglion, e.g., the cervical dorsal root gangliq, e.g., the sacral dorsal root ganglia) can reduce or eliminate latent infection by disabling the HSV-1 virus.

Described herein are the approaches to treat or prevent HSV-1 by knocking out viral genes. Methods described herein include the knockout of any of the following HSV-1 encoded genes: *UL19, UL30, UL48* or *UL54,* or any combination thereof (e.g., any two, three or all of the *UL19, UL30, UL48* or *UL54* gene).

*UL19* (also known as VP5) encodes the HSV-1 major capsid protein, VP5. Proper assembly of the viral capsid is known to be an essential part of viral replication, assembly, maturation and infection (Homa et al., Reviews of Medical Virology 1997; 7(2):107-122). RNAi-mediated knockdown of VP5 along with another capsid capsid protein, VP23, *in vitro,* greatly diminished HSV-1 proliferation (Jin et al., PLoS One 2014; 9(5): e96623). Knockout of *UL19* can disable HSV-1 proliferation and therefore prevent, treat or cure HSV-1 infection.

*UL30* encodes the DNA polymerase catalytic subunit (HSV-1 pol). The 5' domain of HSV-1 pol is required for viral replication. Knock out of *UL30* can disable HSV-1 replication and therefore prevent and/or cure HSV-1 infection.

*UL48* encodes the viral protein known as VP16 in HSV-1. VP-16 has been shown to be important in viral egress, the process by which the assembled viral capsid leaves the host nucleus and enters the cytoplasm (Mossman et al., Journal of Virology 2000; 74(14): 6287-6289). Mutation of UL48 in cell culture decreased the ability of HSV-1 to assemble efficiently (Svobodova et al., Journal of Virology 2012; 86(1): 473-483). Knockout of *UL48* can disable HSV-1 assembly and egress and therefore prevent and/or cure HSV-1 infection.

*UL54* encodes ICP27, a highly conserved, multi-functional protein. ICP27 is involved in transcription, RNA processing, RNA export and translation (Sandri-Goldin, Frontiers in Bioscience 2008; 13:5241-5256). ICP27 also shuts off host gene expression during HSV-1 infection. Knockout of *UL54* can disable HSV-1 transcription, translation and RNA processing and therefore prevent and/or cure HSV-1 infection.

Knockout of the genes *UL19, UL30, UL48* or *UL54,* individually or in combination, can reduce HSV-1 infectivity, replication, packaging and can therefore prevent or treat HSV-1 infection.

In addition, knock out of vital HSV-1 genes, e.g., *UL19, UL30, UL48* and *UL54,* individually or in combination, can make HSV-1 more susceptible to antiviral therapy. Mutations in important genes can render HSV-1 and other viruses more susceptible to treatment with antivirals (Zhou et al., Journal of Virology 2014; 88(19): 11121-11129). Knocking out of *UL19, UL30, UL48* and *UL54,* individually or in combination, may be combined with antiviral therapy to prevent or treat HSV-1 infection. The compositions and methods described herein can be used in combination with another antiviral therapy, e.g., another anti-HSV-1 therapy described herein, to treat or prevent HSV-1 infection.

In one approach, one or more of the *UL19, UL30, UL48* and/or *UL54* gene(s) is targeted as a targeted knockout, e.g., to inhibit essential viral functions, including, e.g. viral gene transcription, viral genome replication and viral capsid formation. In an embodiment, said approach comprises knocking out one HSV-1 gene (e.g., *UL19, UL30, UL48* or *UL54* gene). In another embodiment, said approach comprises knocking out two HSV-1 genes, e.g., two of *UL19, UL30, UL48* or *UL54* gene(s). In another embodiment, said approach comprises knocking out three HSV-1 genes, e.g., three or more of *UL19, UL30, UL48* or *UL54* gene(s). In another embodiment, said approach comprises knocking out four HSV-1 genes, e.g., each of *UL19, UL30, UL48* and *UL54* genes.

While not wishing to be bound by theory, it is considered that inhibiting essential viral functions, e.g., viral gene transcription, viral genome replication and viral capsid formation, decreases the duration of primary or recurrent infection and/or decrease shedding of viral particles. Subjects also experience shorter duration(s) of illness, decreased risk of transmission to sexual partners, decreased risk of transmission to the fetus in the case of pregnancy and/or the potential for full clearance of HSV-1 (cure).

Knockout of one or more copies (e.g., 1, 2, 3, 4, 5, 6, 7, 8, 9 or 10 copies) of one or more target genes (e.g., *UL19, UL30, UL48* or *UL54* gene) may be performed prior to disease onset or after disease onset, but preferably early in the disease course.

In an embodiment, the method comprises initiating treatment of a subject prior to disease onset.

In an embodiment, the method comprises initiating treatment of a subject after disease onset.

In an embodiment, the method comprises initiating treatment of a subject well after disease onset, e.g., 1, 2, 3, 4, 5, 6, 7, 8, 9, 10, 12, 16, 24, 36, 48 or more months after onset of HSV-1 infection. In an embodiment, the method comprises initiating treatment of a subject well after disease onset, e.g., 1, 2, 3, 4, 5, 10, 15, 20, 25, 40, 50 or 60 years after onset of HSV-1 infection. While not wishing to be bound by theory it is believed that this may be effective as disease progression is slow in some cases and a subject may present well into the course of illness.

In an embodiment, the method comprises initiating treatment of a subject in an advanced stage of disease, e.g., during acute or latent periods. In an embodiment, the method comprises initiating treatment of a subject in the case of severe, acute disease affecting the central nervous system, eyes, oropharynx, genital region, and/or other region.

Overall, initiation of treatment for subjects at all stages of disease is expected to improve healing, decrease duration of disease and be of benefit to subjects.

In an embodiment, the method comprises initiating treatment of a subject prior to disease expression. In an embodiment, the method comprises initiating treatment of a subject in an early stage of disease, e.g., when a subject has been exposed to HSV-1 or is thought to have been exposed to HSV-1.

In an embodiment, the method comprises initiating treatment of a subject prior to disease expression. In an embodiment, the method comprises initiating treatment of a subject in an early stage of disease, e.g., when a subject has tested positive for HSV-1 infections but has no signs or symptoms.

In an embodiment, the method comprises initiating treatment of a subject at the appearance of painful blistering in or around the mouth, e.g., oral or oropharynx, e.g., in an infant, child, adult or young adult.

In an embodiment, the method comprises initiating treatment of a subject at the appearance of painful blistering in the genital region, e.g., in an infant, child, adult or young adult.

In an embodiment, the method comprises initiating treatment of a subject suspected of having HSV-1 meningitis and/or HSV-1 encephalitis.

In an embodiment, the method comprises initiating treatment at the appearance of any of the following symptoms consistent or associated with HSV-1 meningitis and/or encephalitis: fever, headache, vomiting, photophobia, seizure, decline in level of consciousness, lethargy, or drowsiness.

In an embodiment, the method comprises initiating treatment at the appearance of any of the following signs consistent or associated with HSV meningitis and/or encephalitis: positive CSF culture for HSV-1, elevated WBC in CSF, neck stiffness/positive Brudzinski's sign. In an embodiment, the method comprises initiating treatment in a patient with signs consistent with HSV-1 encephalitis and/or meningitis on EEG, CSF exam, MRI, PCR of CSF specimen, and/or PCR of brain biopsy specimen.

In an embodiment, the method comprises initiating treatment at the appearance of any of the following symptoms consistent or associated with optic HSV-1: pain, photophobia, blurred vision, tearing, redness/injection, loss of vision, floaters, or flashes.

In an embodiment, the method comprises initiating treatment at the appearance of any of the following findings on ophthalmologic exam consistent or associated with optic HSV-1, also known as HSV-1 keratitis: small, raised clear vesicles on corneal epithelium; irregular corneal surface, punctate epithelial erosions; dense stromal infiltrate; ulceration; necrosis; focal, multifocal, or diffuse cellular infiltrates; immune rings; neovascularization; or ghost vessels at any level of the cornea.

In an embodiment, the method comprises initiating treatment at the appearance of any of the following findings on ophthalmologic exam consistent or associated with HSV-1 retinitis or acute retinal necrosis: reduced visual acuity; uveitis; vitritis; scleral injection; inflammation of the anterior and/or vitreous chamber/s; vitreous haze; optic nerve edema; peripheral retinal whitening; retinal tear; retinal detachment; retinal necrosis; evidence of occlusive vasculopathy with arterial involvement, including arterioloar sheathing and arteriolar attenuation.

In an embodiment, the method comprises initiating treatment at the appearance of symptoms and/or signs consistent or associated with either an HSV-1 or an HSV-2 infection of the eye, oropharynx, ano-genital region or central nervous system. While not wishing to be bound by theory, intiating treatment for HSV-1 infection in a case of suspected HSV-1 or HSV-2 infection early in the disease course is beneficial.

In an embodiment, the method comprises initiating treatment *in utero* in case of high risk of maternal-to-fetal transmission.

In an embodiment, the method comprises initiating treatment during pregnancy in case of mother who has active HSV-1 infection or has recent primary HSV-1 infection.

In an embodiment, the method comprises initiating treatment prior to organ transplantation or immediately following organ transplantation.

In an embodiment, the method comprises initiating treatment in case of suspected exposure to HSV-1.

In an embodiment, the method comprises initiating treatment prophylactically, especially in case of suspected HSV-encephalitis or meningitis.

In an embodiment, the method comprises initiating treatment of a subject who suffers from or is at risk of developing severe manifestations of HSV-1 infections, e.g., neonates, subjects with HIV, subjects who are on immunosuppressant therapy following organ transplantation, subjects who have cancer, subjects who are undergoing chemotherapy, subjects who will undergo chemotherapy, subjects who are undergoing radiation therapy, subjects who will undergo radiation therapy.

While not wishing to be bound by theory, it is considered that both HIV positive subjects and post-transplant subjects may experience severe HSV-1 activation or reactivation, including HSV-encephalitis and meningitis, due to immunodeficiency. Neonates are also at risk for severe HSV-encephalitis due to maternal-fetal transmission during childbirth. Inhibiting essential viral functions, e.g., viral gene transcription, viral genome replication and viral capsid formation, may provide superior protection to said populations at risk for severe HSV-1 infections. Subjects may experience lower rates of HSV-1 encephalitis and/or lower rates of severe neurologic sequelae following HSV-1 encephalitis, which will profoundly improve quality of life.

In an embodiment, the method comprises initiating treatment of a subject who has tested positive for HSV-1.

In an embodiment, the method comprises initiating treatment at the appearance of any one or more of the following findings consistent or associated with HSV-1: appearance of blistering in the oropharynx, ano-genital area, oral or ano- genital ulcers and/or flu-like illness.

In an embodiment, the method comprises initiating treatment at the appearance of any of the following findings consistent or associated with HSV-1 infection: fever, headache, body aches, oral or ano-genital blistering, oral ulceration, encephalitis, meningitis or keratitis.

In an embodiment, the method comprises initiating treatment in a subject who has tested positive for HSV-1 infection via viral culture, direct fluorescent antibody study, skin biopsy, PCR, blood serologic test, CSF serologic test, CSF PCR, or brain biopsy. In an embodiment, the method comprises initiating treatment in a subject who has tested positive for HSV-2 infection via diagnostic vitrectomy, endoretinal biopsy, PCR of aqueous fluid, PCR of vitreous sample.

In an embodiment, the method comprises initiating treatment in any subject exposed to HSV-1 and at high risk for severe sequelae from HSV infection.

In some embodiments, a cell is manipulated by editing (e.g., introducing a mutation in) one or more target genes, e.g., *UL19, UL30, UL48* or *UL54* gene. In some embodiments, the expression of one or more target genes (e.g., one or more *UL19, UL30, UL48* or *UL54* gene described herein) is modulated, e.g., *in vivo.*

In an embodiment, the method comprises delivery of gRNA by an AAV. In an embodiment, the method comprises delivery of gRNA by a lentivirus. In an embodiment, the method comprises delivery of gRNA by a nanoparticle. In an embodiment, the method comprises delivery of gRNA by a gel-based AAV for topical therapy.

In an embodiment, the method further comprising treating the subject a second antiviral therapy, e.g., an anti-HSV-1 therapy described herein. The compositions described herein can be administered concurrently with, prior to, or subsequent to, one or more additional therapies or therapeutic agents. The composition and the other therapy or therapeutic agent can be administered in any order. In an embodiment, the effect of the two treatments is synergistic. Exemplary anti-HSV-1 therapies include, but are not limited to, acyclovir, valacyclovir, famciclovir, penciclovir, or a vaccine.

### Methods of Altering UL19, UL30, UL48 and/or UL54 gene(s)

As disclosed herein, a HSV-1 target position, e.g., one or more of *UL19, UL30, UL48* or *UL54* gene(s), can be altered by gene editing, e.g., using CRISPR-Cas9 mediated methods as described herein.

Methods and compositions discussed herein, provide for altering a HSV-1 target position in one or more of the *UL19, UL30, UL48* and/or *UL54* gene(s). A HSV-1 target position can be altered by gene editing, e.g., using CRISPR-Cas9 mediated methods to alter one or more of the *UL19, UL30, UL48* and/or *UL54* gene(s).

An alteration of one or more of the *UL19, UL30, UL48* and/or *UL54* gene(s) can be mediated by any mechanism. Exemplary mechanisms that can be associated with an alteration of one or more of the *UL19, UL30, UL48* and/or *UL54* gene(s) include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion.

In an embodiment, a single strand break is introduced (e.g., positioned by one gRNA molecule) at or in close proximity to a HSV-1 target position in the *UL19, UL30, UL48* and/or *UL54* gene. In an embodiment, a single gRNA molecule (e.g., with a Cas9 nickase) is used to create a single strand break at or in close proximity to the HSV-1 target position, e.g., the gRNA is configured such that the single strand break is positioned either upstream (e.g., within 200 bp upstream) or downstream (e.g., within 200 bp downstream) of the HSV-1 target position. In an embodiment, the break is positioned to avoid unwanted target chromosome elements, such as repeat elements, e.g., an *Alu* repeat.

In an embodiment, a double strand break is introduced (e.g., positioned by one gRNA molecule) at or in close proximity to a HSV-1 target position in the *UL19, UL30, UL48* and/or *UL54* gene. In an embodiment, a single gRNA molecule (e.g., with a Cas9 nuclease other than a Cas9 nickase) is used to create a double strand break at or in close proximity to the HSV-1 target position, e.g., the gRNA molecule is configured such that the double strand break is positioned either upstream (e.g., within 200 bp upstream) or downstream of (e.g., within 200 bp downstream) of a HSV-1 target position. In an embodiment, the break is positioned to avoid unwanted target chromosome elements, such as repeat elements, e.g., an *Alu* repeat.

In an embodiment, two single strand breaks are introduced (e.g., positioned by two gRNA molecules) at or in close proximity to a HSV-1 target position in the *UL19, UL30, UL48* and/or *UL54* gene. In an embodiment, two gRNA molecules (e.g., with one or two Cas9 nickcases) are used to create two single strand breaks at or in close proximity to the HSV-1 target position, e.g., the gRNAs molecules are configured such that both of the single strand breaks are positioned upstream (e.g., within 200 bp upstream) or downstream (e.g., within 200 bp downstream) of the HSV-1 target position. In another embodiment, two gRNA molecules (e.g., with two Cas9 nickcases) are used to create two single strand breaks at or in close proximity to the HSV-1 target position, e.g., the gRNAs molecules are configured such that one single strand break is positioned upstream (e.g., within 200 bp upstream) and a second single strand break is positioned downstream (e.g., within 200 bp downstream) of the HSV-1 target position. In an embodiment, the breaks are positioned to avoid unwanted target chromosome elements, such as repeat elements, e.g., an *Alu* repeat.

In an embodiment, two double strand breaks are introduced (e.g., positioned by two gRNA molecules) at or in close proximity to a HSV-1 target position in the *UL19, UL30, UL48* and/or *UL54* gene. In an embodiment, two gRNA molecules (e.g., with one or two Cas9 nucleases that are not Cas9 nickases) are used to create two double strand breaks to flank a HSV-1 target position, e.g., the gRNA molecules are configured such that one double strand break is positioned upstream (e.g., within 200 bp upstream) and a second double strand break is positioned downstream (e.g., within 200 bp downstream) of the HSV-1 target position. In an embodiment, the breaks are positioned to avoid unwanted target chromosome elements, such as repeat elements, e.g., an *Alu* repeat.

In an embodiment, one double strand break and two single strand breaks are introduced (e.g., positioned by three gRNA molecules) at or in close proximity to a HSV-1 target position in the *UL19, UL30, UL48* and/or *UL54* gene. In an embodiment, three gRNA molecules (e.g., with a Cas9 nuclease other than a Cas9 nickase and one or two Cas9 nickases) to create one double strand break and two single strand breaks to flank a HSV-1 target position, e.g., the gRNA molecules are configured such that the double strand break is positioned upstream or downstream of (e.g., within 200 bp upstream or downstream) of the HSV-1 target position, and the two single strand breaks are positioned at the opposite site, e.g., downstream or upstream (within 200 bp downstream or upstream), of the HSV-1 target position. In an embodiment, the breaks are positioned to avoid unwanted target chromosome elements, such as repeat elements, e.g., an *Alu* repeat.

In an embodiment, four single strand breaks are introduced (e.g., positioned by four gRNA molecules) at or in close proximity to a HSV-1 target position in the *UL19, UL30, UL48* and/or *UL54* gene. In an embodiment, four gRNA molecule (e.g., with one or more Cas9 nickases are used to create four single strand breaks to flank a HSV-1 target position in the *UL19, UL30, UL48* and/or *UL54* gene, e.g., the gRNA molecules are configured such that a first and second single strand breaks are positioned upstream (e.g., within 200 bp upstream) of the HSV-1 target position, and a third and a fourth single stranded breaks are positioned downstream (e.g., within 200 bp downstream) of the HSV-1 target position. In an embodiment, the breaks are positioned to avoid unwanted target chromosome elements, such as repeat elements, e.g., an *Alu* repeat.

In an embodiment, two or more (e.g., three or four) gRNA molecules are used with one Cas9 molecule. In another embodiment, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

### I. gRNA Molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule), sometimes referred to herein as "chimeric" gRNAs, or modular (comprising more than one, and typically two, separate RNA molecules). A gRNA molecule comprises a number of domains. The gRNA molecule domains are described in more detail below.

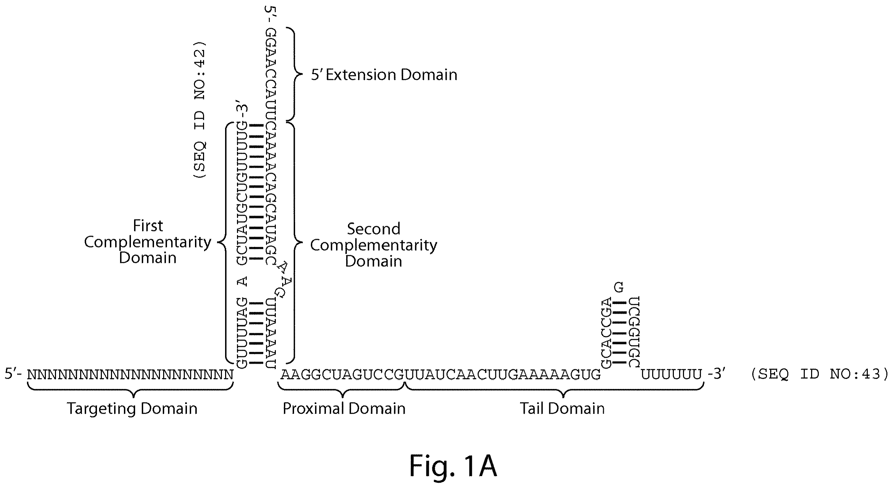

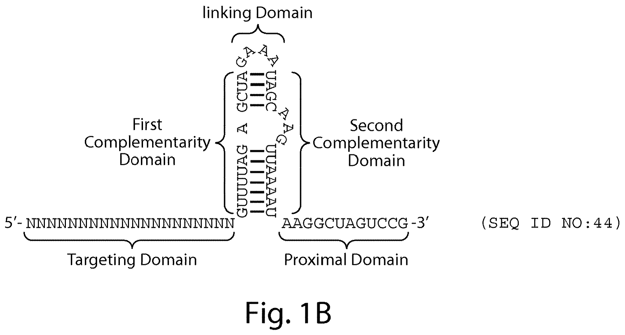

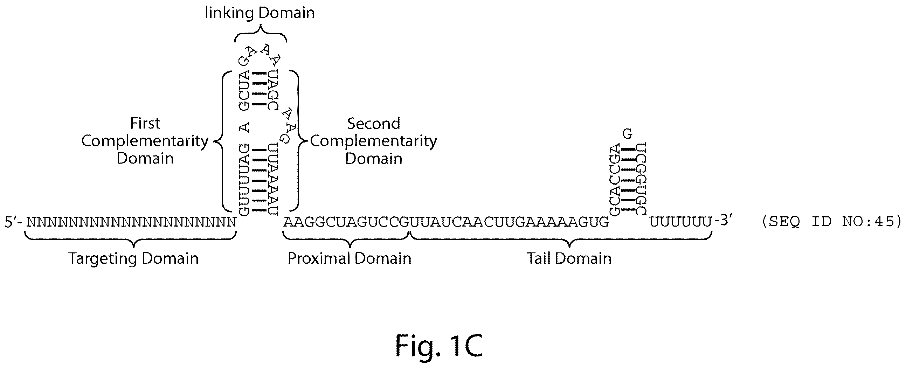

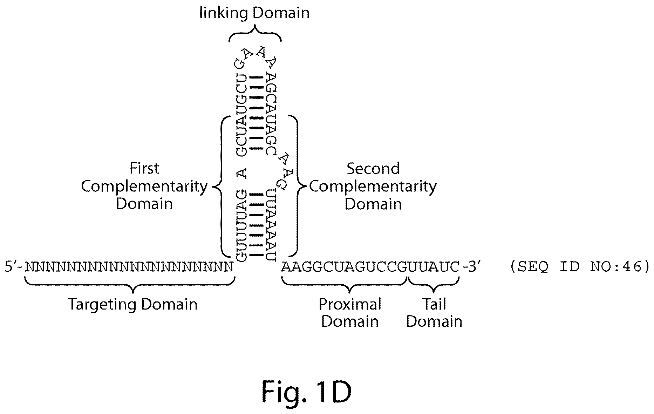

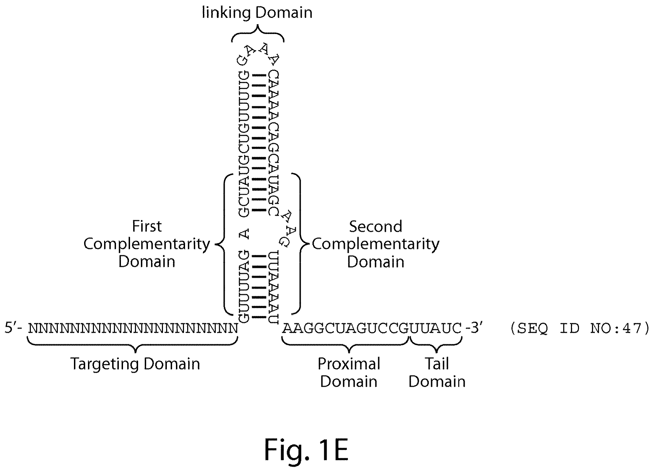

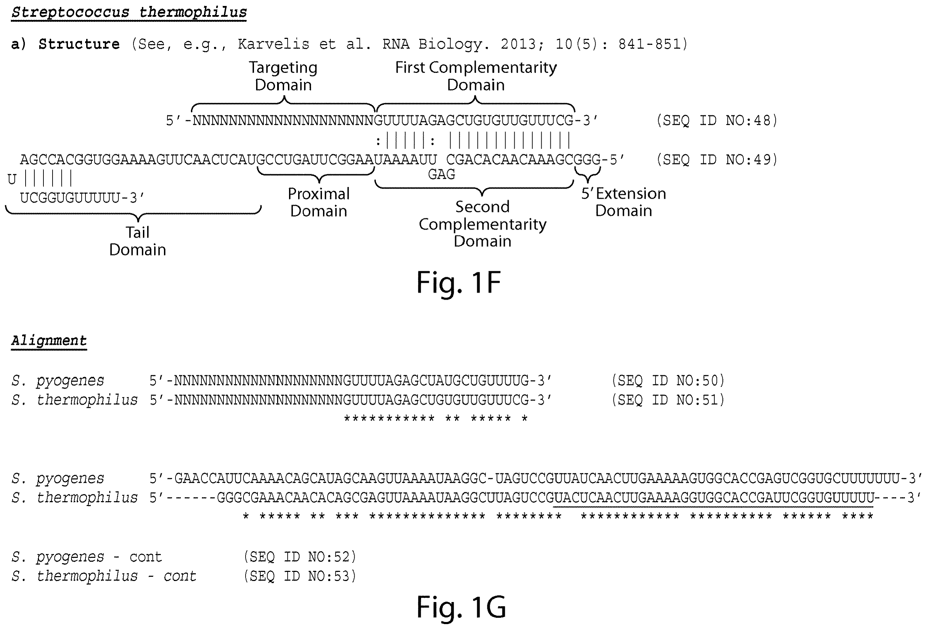

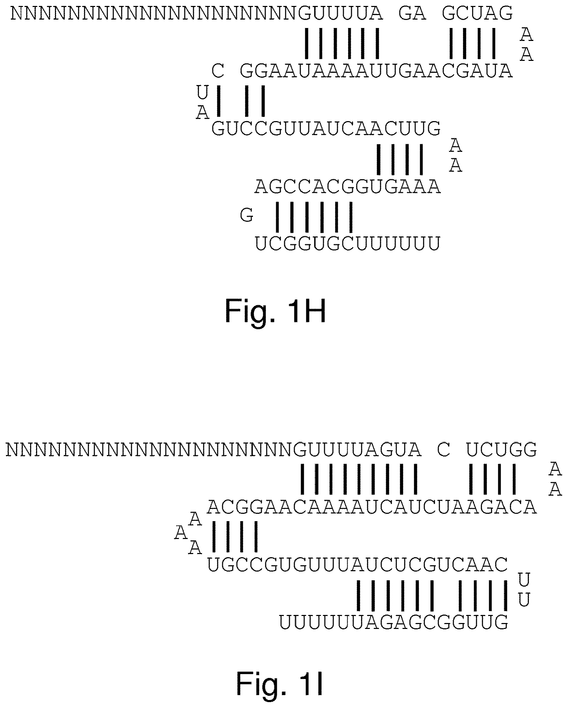

Several exemplary gRNA structures, with domains indicated thereon, are provided in Fig. 1. While not wishing to be bound by theory, in an embodiment, with regard to the three dimensional form, or intra- or inter-strand interactions of an active form of a gRNA, regions of high complementarity are sometimes shown as duplexes in Figs. **1A-1G** and other depictions provided herein.

In an embodiment, a unimolecular, or chimeric, gRNA comprises, preferably from 5' to 3':
a targeting domain (which is complementary to a target nucleic acid in the *UL19, UL30, UL48* or *UL54* gene, e.g., a targeting domain from any of Tables **1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**;
a first complementarity domain;
a linking domain;
a second complementarity domain (which is complementary to the first complementarity domain);
a proximal domain; and
optionally, a tail domain.

In an embodiment, a modular gRNA comprises:
a first strand comprising, preferably from 5' to 3';
   a targeting domain (which is complementary to a target nucleic acid in the *UL19, UL30, UL48* or *UL54* gene, e.g., a targeting domain from **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**); and
   a first complementarity domain; and
a second strand, comprising, preferably from 5' to 3':
   optionally, a 5' extension domain;
   a second complementarity domain;
   a proximal domain; and
   optionally, a tail domain.

The domains are discussed briefly below.

### The Targeting Domain

**Figs. 1A-1G** provide examples of the placement of targeting domains.

The targeting domain comprises a nucleotide sequence that is complementary, e.g., at least 80, 85, 90, or 95% complementary, e.g., fully complementary, to the target sequence on the target nucleic acid. The targeting domain is part of an RNA molecule and will therefore comprise the base uracil (U), while any DNA encoding the gRNA molecule will comprise the base thymine (T). While not wishing to be bound by theory, in an embodiment, it is believed that the complementarity of the targeting domain with the target sequence contributes to specificity of the interaction of the gRNA molecule/Cas9 molecule complex with a target nucleic acid. It is understood that in a targeting domain and target sequence pair, the uracil bases in the targeting domain will pair with the adenine bases in the target sequence. In an embodiment, the target domain itself comprises in the 5' to 3' direction, an optional secondary domain, and a core domain. In an embodiment, the core domain is fully complementary with the target sequence. In an embodiment, the targeting domain is 5 to 50 nucleotides in length. The strand of the target nucleic acid with which the targeting domain is complementary is referred to herein as the complementary strand. Some or all of the nucleotides of the domain can have a modification, e.g., a modification found in Section VIII herein.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

Targeting domains are discussed in more detail below.

### The First Complementarity Domain

**Figs. 1A-1G** provide examples of first complementarity domains.

The first complementarity domain is complementary with the second complementarity domain, and in an embodiment, has sufficient complementarity to the second complementarity domain to form a duplexed region under at least some physiological conditions. In an embodiment, the first complementarity domain is 5 to 30 nucleotides in length. In an embodiment, the first complementarity domain is 5 to 25 nucleotides in length. In an embodiment, the first complementary domain is 7 to 25 nucleotides in length. In an embodiment, the first complementary domain is 7 to 22 nucleotides in length. In an embodiment, the first complementary domain is 7 to 18 nucleotides in length. In an embodiment, the first complementary domain is 7 to 15 nucleotides in length. In an embodiment, the first complementary domain is 5, 6, 7, 8, 9, 10, 11, 12, 13, 14, 15, 16, 17, 18, 19, 20, 21, 22, 23, 24, or 25 nucleotides in length.

In an embodiment, the first complementarity domain comprises 3 subdomains, which, in the 5' to 3' direction are: a 5' subdomain, a central subdomain, and a 3' subdomain. In an embodiment, the 5' subdomain is 4 to 9, e.g., 4, 5, 6, 7, 8 or 9 nucleotides in length. In an embodiment, the central subdomain is 1, 2, or 3, e.g., 1, nucleotide in length. In an embodiment, the 3' subdomain is 3 to 25, e.g., 4 to 22, 4 to 18, or 4 to 10, or 3, 4, 5, 6, 7, 8, 9, 10, 11, 12, 13, 14, 15, 16, 17, 18, 19, 20, 21, 22, 23, 24, or 25 nucleotides in length.

The first complementarity domain can share homology with, or be derived from, a naturally occurring first complementarity domain. In an embodiment, it has at least 50% homology with a first complementarity domain disclosed herein, e.g., an *S. pyogenes, S. aureus* or *S. thermophilus,* first complementarity domain.

Some or all of the nucleotides of the domain can have a modification, e.g., a modification found in Section VIII herein.

First complementarity domains are discussed in more detail below.

### The Linking Domain

**Figs. 1A-1G** provide examples of linking domains.

A linking domain serves to link the first complementarity domain with the second complementarity domain of a unimolecular gRNA. The linking domain can link the first and second complementarity domains covalently or non-covalently. In an embodiment, the linkage is covalent. In an embodiment, the linking domain covalently couples the first and second complementarity domains, see, e.g., **Figs. 1B-1E****.** In an embodiment, the linking domain is, or comprises, a covalent bond interposed between the first complementarity domain and the second complementarity domain. Typically the linking domain comprises one or more, e.g., 2, 3, 4, 5, 6, 7, 8, 9, or 10 nucleotides.

In modular gRNA molecules the two molecules are associated by virtue of the hybridization of the complementarity domains see e.g., **Fig. 1A****.**

A wide variety of linking domains are suitable for use in unimolecular gRNA molecules. Linking domains can consist of a covalent bond, or be as short as one or a few nucleotides, e.g., 1, 2, 3, 4, or 5 nucleotides in length. In an embodiment, a linking domain is 2, 3, 4, 5, 6, 7, 8, 9, 10, 15, 20, or 25 or more nucleotides in length. In an embodiment, a linking domain is 2 to 50, 2 to 40, 2 to 30, 2 to 20, 2 to 10, or 2 to 5 nucleotides in length. In an embodiment, a linking domain shares homology with, or is derived from, a naturally occurring sequence, e.g., the sequence of a tracrRNA that is 5' to the second complementarity domain. In an embodiment, the linking domain has at least 50% homology with a linking domain disclosed herein.

Some or all of the nucleotides of the domain can have a modification, e.g., a modification found in Section VIII herein.

Linking domains are discussed in more detail below.

### The 5' Extension Domain

In an embodiment, a modular gRNA can comprise additional sequence, 5' to the second complementarity domain, referred to herein as the 5' extension domain, see, e.g., **Fig. 1A****.** In an embodiment, the 5' extension domain is, 2 to 10, 2 to 9, 2 to 8, 2 to 7, 2 to 6, 2 to 5, or 2 to 4 nucleotides in length. In an embodiment, the 5' extension domain is 2, 3, 4, 5, 6, 7, 8, 9, or 10 or more nucleotides in length.

### The Second Complementarity Domain

**Figs. 1A-1G** provide examples of second complementarity domains.

The second complementarity domain is complementary with the first complementarity domain, and in an embodiment, has sufficient complementarity to the second complementarity domain to form a duplexed region under at least some physiological conditions. In an embodiment, e.g., as shown in **Figs. 1A-1B****,** the second complementarity domain can include sequence that lacks complementarity with the first complementarity domain, e.g., sequence that loops out from the duplexed region.

In an embodiment, the second complementarity domain is 5 to 27 nucleotides in length. In an embodiment, it is longer than the first complementarity region. In an embodiment the second complementary domain is 7 to 27 nucleotides in length. In an embodiment, the second complementary domain is 7 to 25 nucleotides in length. In an embodiment, the second complementary domain is 7 to 20 nucleotides in length. In an embodiment, the second complementary domain is 7 to 17 nucleotides in length. In an embodiment, the complementary domain is 5, 6, 7, 8, 9, 10, 11, 12, 13, 14, 15, 16, 17, 18, 19, 20, 21, 22, 23, 24 or 25 nucleotides in length.

In an embodiment, the second complementarity domain comprises 3 subdomains, which, in the 5' to 3' direction are: a 5' subdomain, a central subdomain, and a 3' subdomain. In an embodiment, the 5' subdomain is 3 to 25, e.g., 4 to 22, 4 to18, or 4 to 10, or 3, 4, 5, 6, 7, 8, 9, 10, 11, 12, 13, 14, 15, 16, 17, 18, 19, 20, 21, 22, 23, 24, or 25 nucleotides in length. In an embodiment, the central subdomain is 1, 2, 3, 4 or 5, e.g., 3, nucleotides in length. In an embodiment, the 3' subdomain is 4 to 9, e.g., 4, 5, 6, 7, 8 or 9 nucleotides in length.

In an embodiment, the 5' subdomain and the 3' subdomain of the first complementarity domain, are respectively, complementary, e.g., fully complementary, with the 3' subdomain and the 5' subdomain of the second complementarity domain.

The second complementarity domain can share homology with or be derived from a naturally occurring second complementarity domain. In an embodiment, it has at least 50% homology with a second complementarity domain disclosed herein, e.g., an *S. pyogenes, S. aureus* or *S. thermophilus,* first complementarity domain.

Some or all of the nucleotides of the domain can have a modification, e.g., a modification found in Section VIII herein.

### A Proximal domain

**Figs. 1A-1G** provide examples of proximal domains.

In an embodiment, the proximal domain is 5 to 20 nucleotides in length. In an embodiment, the proximal domain can share homology with or be derived from a naturally occurring proximal domain. In an embodiment, it has at least 50% homology with a proximal domain disclosed herein, e.g., an *S. pyogenes, S. aureus* or *S. thermophilus,* proximal domain.

Some or all of the nucleotides of the domain can have a modification, e.g., a modification found in Section VIII herein.

### A Tail Domain

**Figs. 1A-1G** provide examples of tail domains.

As can be seen by inspection of the tail domains in **Figs. 1A-1E****,** a broad spectrum of tail domains are suitable for use in gRNA molecules. In an embodiment, the tail domain is 0 (absent), 1, 2, 3, 4, 5, 6, 7, 8, 9, or 10 nucleotides in length. In embodiment, the tail domain nucleotides are from or share homology with sequence from the 5' end of a naturally occurring tail domain, see e.g., **Fig. 1D** or **Fig. 1E****.** In an embodiment, the tail domain includes sequences that are complementary to each other and which, under at least some physiological conditions, form a duplexed region.

In an embodiment, the tail domain is absent or is 1 to 50 nucleotides in length. In an embodiment, the tail domain can share homology with or be derived from a naturally occurring proximal tail domain. In an embodiment, it has at least 50% homology with a tail domain disclosed herein, e.g., an *S. pyogenes, S. aureus* or *S. thermophilus,* tail domain.

In an embodiment, the tail domain includes nucleotides at the 3' end that are related to the method of in vitro or in vivo transcription. When a T7 promoter is used for in vitro transcription of the gRNA, these nucleotides may be any nucleotides present before the 3' end of the DNA template. When a U6 promoter is used for in vivo transcription, these nucleotides may be the sequence UUUUUU. When alternate pol-III promoters are used, these nucleotides may be various numbers or uracil bases or may include alternate bases.

The domains of gRNA molecules are described in more detail below.

### The Targeting Domain

The "targeting domain" of the gRNA is complementary to the "target domain" on the target nucleic acid. The strand of the target nucleic acid comprising the nucleotide sequence complementary to the core domain of the gRNA is referred to herein as the "complementary strand" of the target nucleic acid. Guidance on the selection of targeting domains can be found, e.g., in Fu Y et al., Nat Biotechnol 2014 (doi: 10.1038/nbt.2808) and Sternberg SH et al., Nature 2014 (doi: 10.1038/nature13011).

In an embodiment, the targeting domain is 16, 17, 18, 19, 20, 21, 22, 23, 24, 25 or 26 nucleotides in length.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, the targeting domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the targeting domain is 20+/-5 nucleotides in length.

In an embodiment, the targeting domain is 20+/-10, 30+/-10, 40+/-10, 50+/-10, 60+/- 10, 70+/-10, 80+/-10, 90+/-10, or 100+/-10 nucleotides, in length.

In an embodiment, the targeting domain is 30+/-10 nucleotides in length.

In an embodiment, the targeting domain is 10 to 100, 10 to 90, 10 to 80, 10 to 70, 10 to 60, 10 to 50, 10 to 40, 10 to 30, 10 to 20 or 10 to 15 nucleotides in length. In another embodiment, the targeting domain is 20 to 100, 20 to 90, 20 to 80, 20 to 70, 20 to 60, 20 to 50, 20 to 40, 20 to 30, or 20 to 25 nucleotides in length.

Typically the targeting domain has full complementarity with the target sequence. In an embodiment the targeting domain has or includes 1, 2, 3, 4, 5, 6, 7 or 8 nucleotides that are not complementary with the corresponding nucleotide of the targeting domain.

In an embodiment, the target domain includes 1, 2, 3, 4 or 5 nucleotides that are complementary with the corresponding nucleotide of the targeting domain within 5 nucleotides of its 5' end. In an embodiment, the target domain includes 1, 2, 3, 4 or 5 nucleotides that are complementary with the corresponding nucleotide of the targeting domain within 5 nucleotides of its 3' end.

In an embodiment, the target domain includes 1, 2, 3, or 4 nucleotides that are not complementary with the corresponding nucleotide of the targeting domain within 5 nucleotides of its 5' end. In an embodiment, the target domain includes 1, 2, 3, or 4 nucleotides that are not complementary with the corresponding nucleotide of the targeting domain within 5 nucleotides of its 3' end.

In an embodiment, the degree of complementarity, together with other properties of the gRNA, is sufficient to allow targeting of a Cas9 molecule to the target nucleic acid.

In some embodiments, the targeting domain comprises two consecutive nucleotides that are not complementary to the target domain ("non-complementary nucleotides"), e.g., two consecutive noncomplementary nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides within 5 nucleotides of the 5' end of the targeting domain, within 5 nucleotides of the 3' end of the targeting domain, or within a region that is more than 5 nucleotides away from one or both ends of the targeting domain, are not complementary to the targeting domain.

In an embodiment, there are no noncomplementary nucleotides within 5 nucleotides of the 5' end of the targeting domain, within 5 nucleotides of the 3' end of the targeting domain, or within a region that is more than 5 nucleotides away from one or both ends of the targeting domain.

In an embodiment, the targeting domain nucleotides do not comprise modifications, e.g., modifications of the type provided in Section VIII. However, in an embodiment, the targeting domain comprises one or more modifications, e.g., modifications that it render it less susceptible to degradation or more bio-compatible, e.g., less immunogenic. By way of example, the backbone of the targeting domain can be modified with a phosphorothioate, or other modification(s) from Section VIII. In an embodiment, a nucleotide of the targeting domain can comprise a 2' modification, e.g., a 2-acetylation, e.g., a 2' methylation, or other modification(s) from Section VIII.

In some embodiments, the targeting domain includes 1, 2, 3, 4, 5, 6, 7 or 8 or more modifications. In an embodiment, the targeting domain includes 1, 2, 3, or 4 modifications within 5 nucleotides of its 5' end. In an embodiment, the targeting domain comprises as many as 1, 2, 3, or 4 modifications within 5 nucleotides of its 3' end.

In some embodiments, the targeting domain comprises modifications at two consecutive nucleotides, e.g., two consecutive nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides are modified within 5 nucleotides of the 5' end of the targeting domain, within 5 nucleotides of the 3' end of the targeting domain, or within a region that is more than 5 nucleotides away from one or both ends of the targeting domain. In an embodiment, no nucleotide is modified within 5 nucleotides of the 5' end of the targeting domain, within 5 nucleotides of the 3' end of the targeting domain, or within a region that is more than 5 nucleotides away from one or both ends of the targeting domain.

Modifications in the targeting domain can be selected so as to not interfere with targeting efficacy, which can be evaluated by testing a candidate modification in the system described in Section IV. gRNAs having a candidate targeting domain having a selected length, sequence, degree of complementarity, or degree of modification, can be evaluated in a system in Section IV. The candidate targeting domain can be placed, either alone, or with one or more other candidate changes in a gRNA molecule/Cas9 molecule system known to be functional with a selected target and evaluated.

In an embodiment, all of the modified nucleotides are complementary to and capable of hybridizing to corresponding nucleotides present in the target domain. In another embodiment, 1, 2, 3, 4, 5, 6, 7 or 8 or more modified nucleotides are not complementary to or capable of hybridizing to corresponding nucleotides present in the target domain.

In an embodiment, the targeting domain comprises, preferably in the 5'→3' direction: a secondary domain and a core domain. These domains are discussed in more detail below.

### The Core Domain and Secondary Domain of the Targeting Domain

The "core domain" of the targeting domain is complementary to the "core domain target" on the target nucleic acid. In an embodiment, the core domain comprises about 8 to about 13 nucleotides from the 3' end of the targeting domain (e.g., the most 3' 8 to 13 nucleotides of the targeting domain).

In an embodiment, the core domain and targeting domain, are independently, 6 +/-2, 7+/-2, 8+/-2, 9+/-2, 10+/-2, 11+/-2, 12+/-2, 13+/-2, 14+/-2, 15+/-2, or 16+-2, nucleotides in length.

In an embodiment, the core domain and targeting domain, are independently, 10+/-2 nucleotides in length.

In an embodiment, the core domain and targeting domain, are independently, 10+/-4 nucleotides in length.

In an embodiment, the core domain and targeting domain are independently 6, 7, 8, 9, 10, 11, 12, 13, 14, 15, 16, 17, or 18 nucleotides in length.

In an embodiment, the core domain and targeting domain are independently 3 to 20, 4 to 20, 5 to 20, 6 to 20, 7 to 20, 8 to 20, 9 to 20 10 to 20 or 15 to 20 nucleotides in length.

In an embodiment, the core domain and targeting domain are independently 3 to 15, e.g., 6 to 15, 7 to 14, 7 to 13, 6 to 12, 7 to 12, 7 to 11, 7 to 10, 8 to 14, 8 to 13, 8 to 12, 8 to 11, 8 to 10 or 8 to 9 nucleotides in length.

The core domain is complementary with the core domain target. Typically the core domain has exact complementarity with the core domain target. In some embodiments, the core domain can have 1, 2, 3, 4 or 5 nucleotides that are not complementary with the corresponding nucleotide of the core domain. In an embodiment, the degree of complementarity, together with other properties of the gRNA, is sufficient to allow targeting of a Cas9 molecule to the target nucleic acid.

The "secondary domain" of the targeting domain of the gRNA is complementary to the "secondary domain target" of the target nucleic acid.

In an embodiment, the secondary domain is positioned 5' to the core domain.

In an embodiment, the secondary domain is absent or optional.

In an embodiment, if the targeting domain is 26 nucleotides in length and the core domain (counted from the 3' end of the targeting domain) is 8 to 13 nucleotides in length, the secondary domain is 12 to 17 nucleotides in length.

In an embodiment, if the targeting domain is 25 nucleotides in length and the core domain (counted from the 3' end of the targeting domain) is 8 to 13 nucleotides in length, the secondary domain is 12 to 17 nucleotides in length.

In an embodiment, if the targeting domain is 24 nucleotides in length and the core domain (counted from the 3' end of the targeting domain) is 8 to 13 nucleotides in length, the secondary domain is 11 to 16 nucleotides in length.

In an embodiment, if the targeting domain is 23 nucleotides in length and the core domain (counted from the 3' end of the targeting domain) is 8 to 13 nucleotides in length, the secondary domain is 10 to 15 nucleotides in length.

In an embodiment, if the targeting domain is 22 nucleotides in length and the core domain (counted from the 3' end of the targeting domain) is 8 to 13 nucleotides in length, the secondary domain is 9 to 14 nucleotides in length.
In an embodiment, if the targeting domain is 21 nucleotides in length and the core domain (counted from the 3' end of the targeting domain) is 8 to 13 nucleotides in length, the secondary domain is 8 to 13 nucleotides in length.

In an embodiment, if the targeting domain is 20 nucleotides in length and the core domain (counted from the 3' end of the targeting domain) is 8 to 13 nucleotides in length, the secondary domain is 7 to 12 nucleotides in length.

In an embodiment, if the targeting domain is 19 nucleotides in length and the core domain (counted from the 3' end of the targeting domain) is 8 to 13 nucleotides in length, the secondary domain is 6 to 11 nucleotides in length.

In an embodiment, if the targeting domain is 18 nucleotides in length and the core domain (counted from the 3' end of the targeting domain) is 8 to 13 nucleotides in length, the secondary domain is 5 to 10 nucleotides in length.

In an embodiment, if the targeting domain is 17 nucleotides in length and the core domain (counted from the 3' end of the targeting domain) is 8 to 13 nucleotides in length, the secondary domain is 4 to 9 nucleotides in length.

In an embodiment, if the targeting domain is 16 nucleotides in length and the core domain (counted from the 3' end of the targeting domain) is 8 to 13 nucleotides in length, the secondary domain is 3 to 8 nucleotides in length.

In an embodiment, the secondary domain is 0, 1, 2, 3, 4, 5, 6, 7, 8, 9, 10, 11, 12, 13, 14 or 15 nucleotides in length.

The secondary domain is complementary with the secondary domain target. Typically the secondary domain has exact complementarity with the secondary domain target. In some embodiments the secondary domain can have 1, 2, 3, 4 or 5 nucleotides that are not complementary with the corresponding nucleotide of the secondary domain. In an embodiment, the degree of complementarity, together with other properties of the gRNA, is sufficient to allow targeting of a Cas9 molecule to the target nucleic acid.

In an embodiment, the core domain nucleotides do not comprise modifications, e.g., modifications of the type provided in Section VIII. However, in an embodiment, the core domain comprises one or more modifications, e.g., modifications that it render it less susceptible to degradation or more bio-compatible, e.g., less immunogenic. By way of example, the backbone of the core domain can be modified with a phosphorothioate, or other modification(s) from Section VIII. In an embodiment a nucleotide of the core domain can comprise a 2' modification, e.g., a 2-acetylation, e.g., a 2' methylation, or other modification(s) from Section VIII. Typically, a core domain will contain no more than 1, 2, or 3 modifications.

Modifications in the core domain can be selected so as to not interfere with targeting efficacy, which can be evaluated by testing a candidate modification in the system described in Section IV. gRNAs having a candidate core domain having a selected length, sequence, degree of complementarity, or degree of modification, can be evaluated in the system described at Section IV. The candidate core domain can be placed, either alone, or with one or more other candidate changes in a gRNA molecule/Cas9 molecule system known to be functional with a selected target and evaluated.

In an embodiment, the secondary domain nucleotides do not comprise modifications, e.g., modifications of the type provided in Section VIII. However, in an embodiment, the secondary domain comprises one or more modifications, e.g., modifications that render it less susceptible to degradation or more bio-compatible, e.g., less immunogenic. By way of example, the backbone of the secondary domain can be modified with a phosphorothioate, or other modification(s) from Section VIII. In an embodiment a nucleotide of the secondary domain can comprise a 2' modification, e.g., a 2-acetylation, e.g., a 2' methylation, or other modification(s) from Section VIII. Typically, a secondary domain will contain no more than 1, 2, or 3 modifications.

Modifications in the secondary domain can be selected to not interfere with targeting efficacy, which can be evaluated by testing a candidate modification in the system described in Section IV. gRNAs having a candidate secondary domain having a selected length, sequence, degree of complementarity, or degree of modification, can be evaluated in the system described at Section IV. The candidate secondary domain can be placed, either alone, or with one or more other candidate changes in a gRNA molecule/Cas9 molecule system known to be functional with a selected target and evaluated.

In an embodiment, (1) the degree of complementarity between the core domain and its target, and (2) the degree of complementarity between the secondary domain and its target, may differ. In an embodiment, (1) may be greater than (2). In an embodiment, (1) may be less than (2). In an embodiment, (1) and (2) are the same, e.g., each may be completely complementary with its target.

In an embodiment, (1) the number of modifications (e.g., modifications from Section VIII) of the nucleotides of the core domain and (2) the number of modifications (e.g., modifications from Section VIII) of the nucleotides of the secondary domain may differ. In an embodiment, (1) may be less than (2). In an embodiment, (1) may be greater than (2). In an embodiment, (1) and (2) may be the same, e.g., each may be free of modifications.

### The First and Second Complementarity Domains

The first complementarity domain is complementary with the second complementarity domain.

Typically the first domain does not have exact complementarity with the second complementarity domain target. In some embodiments, the first complementarity domain can have 1, 2, 3, 4 or 5 nucleotides that are not complementary with the corresponding nucleotide of the second complementarity domain. In an embodiment, 1, 2, 3, 4, 5 or 6, e.g., 3 nucleotides, will not pair in the duplex, and, e.g., form a non-duplexed or looped-out region. In an embodiment, an unpaired, or loop-out, region, e.g., a loop-out of 3 nucleotides, is present on the second complementarity domain. In an embodiment, the unpaired region begins 1, 2, 3, 4, 5, or 6, e.g., 4, nucleotides from the 5' end of the second complementarity domain.

In an embodiment, the degree of complementarity, together with other properties of the gRNA, is sufficient to allow targeting of a Cas9 molecule to the target nucleic acid.

In an embodiment, the first and second complementarity domains are:
independently, 6 +/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length;
independently, 6, 7, 8, 9, 10, 11, 12, 13, 14, 14, 16, 17, 18, 19, 20, 21, 22, 23, 24, 25, or 26 nucleotides in length; or
independently, 5 to 24, 5 to 23, 5 to 22, 5 to 21, 5 to 20, 7 to 18, 9 to 16, or 10 to 14 nucleotides in length.

In an embodiment, the second complementarity domain is longer than the first complementarity domain, e.g., 2, 3, 4, 5, or 6, e.g., 6, nucleotides longer.

In an embodiment, the first and second complementary domains, independently, do not comprise modifications, e.g., modifications of the type provided in Section VIII.

In an embodiment, the first and second complementary domains, independently, comprise one or more modifications, e.g., modifications that the render the domain less susceptible to degradation or more bio-compatible, e.g., less immunogenic. By way of example, the backbone of the domain can be modified with a phosphorothioate, or other modification(s) from Section VIII. In an embodiment a nucleotide of the domain can comprise a 2' modification, e.g., a 2-acetylation, e.g., a 2' methylation, or other modification(s) from Section VIII.

In an embodiment, the first and second complementary domains, independently, include 1, 2, 3, 4, 5, 6, 7 or 8 or more modifications. In an embodiment, the first and second complementary domains, independently, include 1, 2, 3, or 4 modifications within 5 nucleotides of its 5' end. In an embodiment, the first and second complementary domains, independently, include as many as 1, 2, 3, or 4 modifications within 5 nucleotides of its 3' end.

In an embodiment, the first and second complementary domains, independently, include modifications at two consecutive nucleotides, e.g., two consecutive nucleotides that are within 5 nucleotides of the 5' end of the domain, within 5 nucleotides of the 3' end of the domain, or more than 5 nucleotides away from one or both ends of the domain. In an embodiment, the first and second complementary domains, independently, include no two consecutive nucleotides that are modified, within 5 nucleotides of the 5' end of the domain, within 5 nucleotides of the 3' end of the domain, or within a region that is more than 5 nucleotides away from one or both ends of the domain. In an embodiment, the first and second complementary domains, independently, include no nucleotide that is modified within 5 nucleotides of the 5' end of the domain, within 5 nucleotides of the 3' end of the domain, or within a region that is more than 5 nucleotides away from one or both ends of the domain.

Modifications in a complementarity domain can be selected so as to not interfere with targeting efficacy, which can be evaluated by testing a candidate modification in the system described in Section IV. gRNAs having a candidate complementarity domain having a selected length, sequence, degree of complementarity, or degree of modification, can be evaluated in the system described in Section IV. The candidate complementarity domain can be placed, either alone, or with one or more other candidate changes in a gRNA molecule/Cas9 molecule system known to be functional with a selected target and evaluated.

In an embodiment, the first complementarity domain has at least 60, 70, 80, 85%, 90% or 95% homology with, or differs by no more than 1, 2, 3, 4, 5, or 6 nucleotides from, a reference first complementarity domain, e.g., a naturally occurring, e.g., an *S. pyogenes, S. aureus* or *S. thermophilus,* first complementarity domain, or a first complementarity domain described herein, e.g., from **Figs. 1A-1G****.**

In an embodiment, the second complementarity domain has at least 60, 70, 80, 85%, 90%, or 95% homology with, or differs by no more than 1, 2, 3, 4, 5, or 6 nucleotides from, a reference second complementarity domain, e.g., a naturally occurring, e.g., an *S. pyogenes, S. aureus* or *S. thermophilus,* second complementarity domain, or a second complementarity domain described herein, e.g., from **Figs. 1A-1G****.**

The duplexed region formed by first and second complementarity domains is typically 6, 7, 8, 9, 10, 11, 12, 13, 14, 15, 16, 17, 18, 19, 20, 21 or 22 base pairs in length (excluding any looped out or unpaired nucleotides).

In some embodiments, the first and second complementarity domains, when duplexed, comprise 11 paired nucleotides, for example, in the gRNA sequence (one paired strand underlined, one bolded):

NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC (SEQ ID NO:
5).

In some embodiments, the first and second complementarity domains, when duplexed, comprise 15 paired nucleotides, for example in the gRNA sequence (one paired strand underlined, one bolded): 
NNNNNNNNNNNNNNNNNNNNGUUUUAGAGCUAUGCUGAAAAGCAUAGCAAGU
UAAAAUAAGGCUAGUCCGUUAUCAACUUGAAAAAGUGGCACCGAGUCGGUGC
 (SEQ ID NO: 27).

In some embodiments the first and second complementarity domains, when duplexed, comprise 16 paired nucleotides, for example in the gRNA sequence (one paired strand underlined, one bolded):

NNNNNNNNNNNNNNNNNNNNGUUUUAGAGCUAUGCUGGAAACAGCAUAGCAA
GUUAAAAUAAGGCUAGUCCGUUAUCAACUUGAAAAAGUGGCACCGAGUCGGU
GC (SEQ ID NO: 28).

In some embodiments the first and second complementarity domains, when duplexed, comprise 21 paired nucleotides, for example in the gRNA sequence (one paired strand underlined, one bolded):

NNNNNNNNNNNNNNNNNNNNGUUUUAGAGCUAUGCUGUUUUGGAAACAAAAC
AGCAUAGCAAGUUAAAAUAAGGCUAGUCCGUUAUCAACUUGAAAAAGUGGCA
CCGAGUCGGUGC (SEQ ID NO: 29).

In some embodiments, nucleotides are exchanged to remove poly-U tracts, for example in the gRNA sequences (exchanged nucleotides underlined): 
NNNNNNNNNNNNNNNNNNNNGUAUUAGAGCUAGAAAUAGCAAGUUAAUAUAA
GGCUAGUCCGUUAUCAACUUGAAAAAGUGGCACCGAGUCGGUGC (SEQ ID NO:
30);
 
NNNNNNNNNNNNNNNNNNNNGUUUAAGAGCUAGAAAUAGCAAGUUUAAAUAA
GGCUAGUCCGUUAUCAACUUGAAAAAGUGGCACCGAGUCGGUGC (SEQ ID NO:
31);
 or

NNNNNNNNNNNNNNNNNNNNGUAUUAGAGCUAUGCUGUAUUGGAAACAAUAC
AGCAUAGCAAGUUAAUAUAAGGCUAGUCCGUUAUCAACUUGAAAAAGUGGCA
CCGAGUCGGUGC (SEQ ID NO: 32).

### The 5' Extension Domain

In an embodiment, a modular gRNA can comprise additional sequence, 5' to the second complementarity domain. In an embodiment, the 5' extension domain is 2 to 10, 2 to 9, 2 to 8, 2 to 7, 2 to 6, 2 to 5, or 2 to 4 nucleotides in length. In an embodiment, the 5' extension domain is 2, 3, 4, 5, 6, 7, 8, 9, or 10 or more nucleotides in length.

In an embodiment, the 5' extension domain nucleotides do not comprise modifications, e.g., modifications of the type provided in Section VIII. However, in an embodiment, the 5' extension domain comprises one or more modifications, e.g., modifications that it render it less susceptible to degradation or more bio-compatible, e.g., less immunogenic. By way of example, the backbone of the 5' extension domain can be modified with a phosphorothioate, or other modification(s) from Section VIII. In an embodiment, a nucleotide of the 5' extension domain can comprise a 2' modification, e.g., a 2-acetylation, e.g., a 2' methylation, or other modification(s) from Section VIII.

In an embodiment, the 5' extension domain can comprise as many as 1, 2, 3, 4, 5, 6, 7 or 8 modifications. In an embodiment, the 5' extension domain comprises as many as 1, 2, 3, or 4 modifications within 5 nucleotides of its 5' end, e.g., in a modular gRNA molecule. In an embodiment, the 5' extension domain comprises as many as 1, 2, 3, or 4 modifications within 5 nucleotides of its 3' end, e.g., in a modular gRNA molecule.

In an embodiment, the 5' extension domain comprises modifications at two consecutive nucleotides, e.g., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In an embodiment, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In an embodiment, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected so as to not interfere with gRNA molecule efficacy, which can be evaluated by testing a candidate modification in the system described in Section IV. gRNAs having a candidate 5' extension domain having a selected length, sequence, degree of complementarity, or degree of modification, can be evaluated in the system described at Section IV. The candidate 5' extension domain can be placed, either alone, or with one or more other candidate changes in a gRNA molecule/Cas9 molecule system known to be functional with a selected target and evaluated.

In an embodiment, the 5' extension domain has at least 60, 70, 80, 85, 90 or 95% homology with, or differs by no more than 1, 2, 3, 4, 5, or 6 nucleotides from, a reference 5' extension domain, e.g., a naturally occurring, e.g., an *S. pyogenes, S. aureus* or *S. thermophilus,* 5' extension domain, or a 5' extension domain described herein, e.g., from **Figs. 1A-1G****.**

### The Linking Domain

In a unimolecular gRNA molecule the linking domain is disposed between the first and second complementarity domains. In a modular gRNA molecule, the two molecules are associated with one another by the complementarity domains.

In an embodiment, the linking domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the linking domain is 20+/-10, 30+/-10, 40+/-10, 50+/-10, 60+/- 10, 70+/-10, 80+/-10, 90+/-10, or 100+/-10 nucleotides, in length.

In an embodiment, the linking domain is 10 to 100, 10 to 90, 10 to 80, 10 to 70, 10 to 60, 10 to 50, 10 to 40, 10 to 30, 10 to 20 or 10 to 15 nucleotides in length. In other embodiments, the linking domain is 20 to 100, 20 to 90, 20 to 80, 20 to 70, 20 to 60, 20 to 50, 20 to 40, 20 to 30, or 20 to 25 nucleotides in length.

In an embodiment, the linking domain is 1, 2, 3, 4, 5, 6, 7, 8, 9, 10, 11, 12, 13, 14, 15, 16 17, 18, 19, or 20 nucleotides in length.

In an embodiment, the linking domain is a covalent bond.

In an embodiment, the linking domain comprises a duplexed region, typically adjacent to or within 1, 2, or 3 nucleotides of the 3' end of the first complementarity domain and/or the 5- end of the second complementarity domain. In an embodiment, the duplexed region can be 20+/-10 base pairs in length. In an embodiment, the duplexed region can be 10+/-5, 15+/-5, 20+/-5, or 30+/-5 base pairs in length. In an embodiment, the duplexed region can be 1, 2, 3, 4, 5, 6, 7, 8, 9, 10, 11, 12, 13, 14, or 15 base pairs in length.

Typically the sequences forming the duplexed region have exact complementarity with one another, though in some embodiments as many as 1, 2, 3, 4, 5, 6, 7 or 8 nucleotides are not complementary with the corresponding nucleotides.

In an embodiment, the linking domain nucleotides do not comprise modifications, e.g., modifications of the type provided in Section VIII. However, in an embodiment, the linking domain comprises one or more modifications, e.g., modifications that it render it less susceptible to degradation or more bio-compatible, e.g., less immunogenic. By way of example, the backbone of the linking domain can be modified with a phosphorothioate, or other modification(s) from Section VIII. In an embodiment a nucleotide of the linking domain can comprise a 2' modification, e.g., a 2-acetylation, e.g., a 2' methylation, or other modification(s) from Section VIII. In an embodiment, the linking domain can comprise as many as 1, 2, 3, 4, 5, 6, 7 or 8 modifications.

Modifications in a linking domain can be selected so as to not interfere with targeting efficacy, which can be evaluated by testing a candidate modification in the system described in Section IV. gRNAs having a candidate linking domain having a selected length, sequence, degree of complementarity, or degree of modification, can be evaluated a system described in Section IV. A candidate linking domain can be placed, either alone, or with one or more other candidate changes in a gRNA molecule/Cas9 molecule system known to be functional with a selected target and evaluated.

In an embodiment, the linking domain has at least 60, 70, 80, 85, 90 or 95% homology with, or differs by no more than 1, 2, 3, 4, 5 ,or 6 nucleotides from, a reference linking domain, e.g., a linking domain described herein, e.g., from **Figs. 1A-1G****.**

### The Proximal Domain

In an embodiment, the proximal domain is 6 +/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length.

In an embodiment, the proximal domain is 6, 7, 8, 9, 10, 11, 12, 13, 14, 15, 16, 17, 18, 19, 20, 21, 22, 23, 24, 25 or 26 nucleotides in length.

In an embodiment, the proximal domain is 5 to 20, 7, to 18, 9 to 16, or 10 to 14 nucleotides in length.

In an embodiment, the proximal domain nucleotides do not comprise modifications, e.g., modifications of the type provided in Section VIII. However, in an embodiment, the proximal domain comprises one or more modifications, e.g., modifications that it render it less susceptible to degradation or more bio-compatible, e.g., less immunogenic. By way of example, the backbone of the proximal domain can be modified with a phosphorothioate, or other modification(s) from Section VIII. In an embodiment a nucleotide of the proximal domain can comprise a 2' modification, e.g., a 2-acetylation, e.g., a 2' methylation, or other modification(s) from Section VIII.

In an embodiment, the proximal domain can comprise as many as 1, 2, 3, 4, 5, 6, 7 or 8 modifications. In an embodiment, the proximal domain comprises as many as 1, 2, 3, or 4 modifications within 5 nucleotides of its 5' end, e.g., in a modular gRNA molecule. In an embodiment, the target domain comprises as many as 1, 2, 3, or 4 modifications within 5 nucleotides of its 3' end, e.g., in a modular gRNA molecule.

In an embodiment, the proximal domain comprises modifications at two consecutive nucleotides, e.g., two consecutive nucleotides that are within 5 nucleotides of the 5' end of the proximal domain, within 5 nucleotides of the 3' end of the proximal domain, or more than 5 nucleotides away from one or both ends of the proximal domain. In an embodiment, no two consecutive nucleotides are modified within 5 nucleotides of the 5' end of the proximal domain, within 5 nucleotides of the 3' end of the proximal domain, or within a region that is more than 5 nucleotides away from one or both ends of the proximal domain. In an embodiment, no nucleotide is modified within 5 nucleotides of the 5' end of the proximal domain, within 5 nucleotides of the 3' end of the proximal domain, or within a region that is more than 5 nucleotides away from one or both ends of the proximal domain.

Modifications in the proximal domain can be selected so as to not interfere with gRNA molecule efficacy, which can be evaluated by testing a candidate modification in the system described in Section IV. gRNAs having a candidate proximal domain having a selected length, sequence, degree of complementarity, or degree of modification, can be evaluated in the system described at Section IV. The candidate proximal domain can be placed, either alone, or with one or more other candidate changes in a gRNA molecule/Cas9 molecule system known to be functional with a selected target and evaluated.

In an embodiment, the proximal domain has at least 60, 70, 80, 85 90 or 95% homology with, or differs by no more than 1, 2, 3, 4, 5 ,or 6 nucleotides from, a reference proximal domain, e.g., a naturally occurring, e.g., an *S. pyogenes, S. aureus* or *S. thermophilus,* proximal domain, or a proximal domain described herein, e.g., from **Figs. 1A-****1G.**

### The Tail Domain

In an embodiment, the tail domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the tail domain is 20+/-5 nucleotides in length.

In an embodiment, the tail domain is 20+/-10, 30+/-10, 40+/-10, 50+/-10, 60+/-10, 70+/-10, 80+/-10, 90+/-10, or 100+/-10 nucleotides, in length.

In an embodiment, the tail domain is 25+/-10 nucleotides in length.

In an embodiment, the tail domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the tail domain is 20 to 100, 20 to 90, 20 to 80, 20 to 70, 20 to 60, 20 to 50, 20 to 40, 20 to 30, or 20 to 25 nucleotides in length.

In an embodiment, the tail domain is 1 to 20, 1 to 15, 1 to 10, or 1 to 5 nucleotides in length.

In an embodiment, the tail domain nucleotides do not comprise modifications, e.g., modifications of the type provided in Section VIII. However, in an embodiment, the tail domain comprises one or more modifications, e.g., modifications that it render it less susceptible to degradation or more bio-compatible, e.g., less immunogenic. By way of example, the backbone of the tail domain can be modified with a phosphorothioate, or other modification(s) from Section VIII. In an embodiment a nucleotide of the tail domain can comprise a 2' modification, e.g., a 2-acetylation, e.g., a 2' methylation, or other modification(s) from Section VIII.

In some embodiments, the tail domain can have as many as 1, 2, 3, 4, 5, 6, 7 or 8 modifications. In an embodiment, the target domain comprises as many as 1, 2, 3, or 4 modifications within 5 nucleotides of its 5' end. In an embodiment, the target domain comprises as many as 1, 2, 3, or 4 modifications within 5 nucleotides of its 3' end.

In an embodiment, the tail domain comprises a tail duplex domain, which can form a tail duplexed region. In an embodiment, the tail duplexed region can be 3, 4, 5, 6, 7, 8, 9, 10, 11, or 12 base pairs in length. In an embodiment, a further single stranded domain, exists 3' to the tail duplexed domain. In an embodiment, this domain is 3, 4, 5, 6, 7, 8, 9, or 10 nucleotides in length. In an embodiment it is 4 to 6 nucleotides in length.

In an embodiment, the tail domain has at least 60, 70, 80, or 90% homology with, or differs by no more than 1, 2, 3, 4, 5 ,or 6 nucleotides from, a reference tail domain, e.g., a naturally occurring, e.g., an *S. pyogenes, S. aureus* or *S. thermophilus,* tail domain, or a tail domain described herein, e.g., from **Figs. 1A-1G****.**

In an embodiment, the proximal and tail domain, taken together, comprise the following sequences: 
AAGGCUAGUCCGUUAUCAACUUGAAAAAGUGGCACCGAGUCGGUGCU (SEQ ID
NO: 33),
 or 
AAGGCUAGUCCGUUAUCAACUUGAAAAAGUGGCACCGAGUCGGUGGUGC (SEQ
ID NO: 34),
 or 
AAGGCUAGUCCGUUAUCAACUUGAAAAAGUGGCACCGAGUCGGUGCGGAUC
(SEQ ID NO: 35),
 or
AAGGCUAGUCCGUUAUCAACUUGAAAAAGUG (SEQ ID NO: 36), or
AAGGCUAGUCCGUUAUCA (SEQ ID NO: 37), or
AAGGCUAGUCCG (SEQ ID NO: 38).

In an embodiment, the tail domain comprises the 3' sequence UUUUUU, e.g., if a U6 promoter is used for transcription.

In an embodiment, the tail domain comprises the 3' sequence UUUU, e.g., if an H1 promoter is used for transcription.

In an embodiment, tail domain comprises variable numbers of 3' Us depending, e.g., on the termination signal of the pol-III promoter used.

In an embodiment, the tail domain comprises variable 3' sequence derived from the DNA template if a T7 promoter is used.

In an embodiment, the tail domain comprises variable 3' sequence derived from the DNA template, e.g., if in vitro transcription is used to generate the RNA molecule.

In an embodiment, the tail domain comprises variable 3' sequence derived from the DNA template, e., if a pol-II promoter is used to drive transcription.

Modifications in the tail domain can be selected so as to not interfere with targeting efficacy, which can be evaluated by testing a candidate modification in the system described in Section IV. gRNAs having a candidate tail domain having a selected length, sequence, degree of complementarity, or degree of modification, can be evaluated in the system described in Section IV. The candidate tail domain can be placed, either alone, or with one or more other candidate changes in a gRNA molecule/Cas9 molecule system known to be functional with a selected target and evaluated.

In an embodiment, the tail domain comprises modifications at two consecutive nucleotides, e.g., two consecutive nucleotides that are within 5 nucleotides of the 5' end of the tail domain, within 5 nucleotides of the 3' end of the tail domain, or more than 5 nucleotides away from one or both ends of the tail domain. In an embodiment, no two consecutive nucleotides are modified within 5 nucleotides of the 5' end of the tail domain, within 5 nucleotides of the 3' end of the tail domain, or within a region that is more than 5 nucleotides away from one or both ends of the tail domain. In an embodiment, no nucleotide is modified within 5 nucleotides of the 5' end of the tail domain, within 5 nucleotides of the 3' end of the tail domain, or within a region that is more than 5 nucleotides away from one or both ends of the tail domain.

In an embodiment a gRNA has the following structure:
5' [targeting domain]-[first complementarity domain]-[linking domain]-[second complementarity domain]-[proximal domain]-[tail domain]-3'
wherein, the targeting domain comprises a core domain and optionally a secondary domain, and is 10 to 50 nucleotides in length;
the first complementarity domain is 5 to 25 nucleotides in length and, In an embodiment has at least 50, 60, 70, 80, 85, 90 or 95% homology with a reference first complementarity domain disclosed herein;
the linking domain is 1 to 5 nucleotides in length;
the second complementarity domain is 5 to 27 nucleotides in length and, in an embodiment has at least 50, 60, 70, 80, 85, 90 or 95% homology with a reference second complementarity domain disclosed herein;
the proximal domain is 5 to 20 nucleotides in length and, in an embodiment has at least 50, 60, 70, 80, 85, 90 or 95% homology with a reference proximal domain disclosed herein; and
the tail domain is absent or a nucleotide sequence is 1 to 50 nucleotides in length and, in an embodiment has at least 50, 60, 70, 80, 85, 90 or 95% homology with a reference tail domain disclosed herein.

### Exemplary Chimeric gRNAs

In an embodiment, a unimolecular, or chimeric, gRNA comprises, preferably from 5' to 3':
a targeting domain (which is complementary to a target nucleic acid);
a first complementarity domain, e.g., comprising 15, 16, 17, 18, 19, 20, 21, 22, 23, 24, 25, or 26 nucleotides;
a linking domain;
a second complementarity domain (which is complementary to the first complementarity domain);
a proximal domain; and
a tail domain,
wherein,
   (a) the proximal and tail domain, when taken together, comprise at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment, there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number: 
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGCUUUUUU
 (SEQ ID NO: 45). In an embodiment, the unimolecular, or chimeric, gRNA molecule is a S. *pyogenes* gRNA molecule.

In some embodiments, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number: 
NNNNNNNNNNNNNNNNNNNNGUUUUAGUACUCUGGAAACAGAAUCUACUAAA
ACAAGGCAAAAUGCCGUGUUUAUCUCGUCAACUUGUUGGCGAGAUUUUUU
 (SEQ ID NO: 40). In an embodiment, the unimolecular, or chimeric, gRNA molecule is a S. *aureus* gRNA molecule.

### Exemplary Modular gRNAs

In an embodiment, a modular gRNA comprises:
a first strand comprising, preferably from 5' to 3';
      a targeting domain, e.g., comprising 15, 16, 17, 18, 19, 20, 21, 22, 23, 24, 25, or 26 nucleotides;
      a first complementarity domain; and
      a second strand, comprising, preferably from 5' to 3':
         optionally a 5' extension domain;
         a second complementarity domain;
         a proximal domain; and
         a tail domain,
   wherein:
      (a) the proximal and tail domain, when taken together, comprise at least 15, 18, 20, 25, 30, 31, 35, 40, 45, 49, 50, or 53 nucleotides;
      (b) there are at least 15, 18, 20, 25, 30, 31, 35, 40, 45, 49, 50, or 53 nucleotides 3' to the last nucleotide of the second complementarity domain; or
      (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### II. Methods for Designing gRNAs

Methods for designing gRNAs are described herein, including methods for selecting, designing and validating target domains. Exemplary targeting domains are also provided herein. Targeting domains discussed herein can be incorporated into the gRNAs described herein.

Methods for selection and validation of target sequences as well as off-target analyses are described, e.g., in Mali et al., 2013 SCIENCE 339(6121): 823-826; Hsu et al. NAT BIOTECHNOL, 31(9): 827-32; Fu et al., 2014 NAT BIOTECHNOL, doi: 10.1038/nbt.2808. PubMed PMID: 24463574; Heigwer et al., 2014 NAT METHODS 11(2):122-3. doi: 10.1038/nmeth.2812. PubMed PMID: 24481216; Bae et al., 2014 BIOINFORMATICS PubMed PMID: 24463181; Xiao A et al., 2014 BIOINFORMATICS PubMed PMID: 24389662.

For example, a software tool can be used to optimize the choice of gRNA within a user's target sequence, e.g., to minimize total off-target activity across the genome. Off target activity may be other than cleavage. For each possible gRNA choice using *S. pyogenes* Cas9, the tool can identify all off-target sequences (preceding either NAG or NGG PAMs) across the genome that contain up to a certain number (e.g., 1, 2, 3, 4, 5, 6, 7, 8, 9, or 10) of mismatched base-pairs. The cleavage efficiency at each off-target sequence can be predicted, e.g., using an experimentally-derived weighting scheme. Each possible gRNA is then ranked according to its total predicted off-target cleavage; the top-ranked gRNAs represent those that are likely to have the greatest on-target and the least off-target cleavage. Other functions, e.g., automated reagent design for CRISPR construction, primer design for the on-target Surveyor assay, and primer design for high-throughput detection and quantification of off-target cleavage via next-gen sequencing, can also be included in the tool. Candidate gRNA molecules can be evaluated by art-known methods or as described in Section IV herein.

The Targeting domains discussed herein can be incorporated into the gRNAs described herein.

### Strategies to identify gRNAs for S. pyogenes. S. aureus, and N. meningitidis to knock out the UL19 gene

As an example, three strategies were utilized to identify gRNAs for use with S. *pyogenes, S. aureus* and *N. meningitidis* Cas9 enzymes.

In the first strategy, guide RNAs (gRNAs) for use with the *S. pyogenes* **(Tables 1A-1C)** Cas9 were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a S. *pyogenes* Cas9 were ranked into 3 tiers.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the target position (i.e., within the first 500 bp of the coding sequence) and with a high level of orthogonality against the human genome. In order to find a pair for the dual-nickase strategy it was necessary to either extend the distance from the target position or remove the requirement for the 5'G. Tier 2 gRNAs were selected based on location within the first 500 bp of the coding sequence in the HSV gene. Tier 3 gRNAs were selected based on their location in the coding sequence, but downstream of the first 500 bp of the HSV gene. Note that tiers are non-inclusive (each gRNA is listed only once). In certain instances, no gRNA was identified based on the criteria of the particular tier.

As discussed above, gRNAs were identified for single-gRNA nuclease cleavage as well as for a dual-gRNA paired "nickase" strategy, as indicated.

gRNAs for use with the *N. meningitidis* **(Tables 1F-1G)** and *S. aureus* **(Tables 1D-1E)** Cas9s were identified manually by scanning genomic DNA sequence for the presence of PAM sequences. These gRNAs were separated into two tiers for each species. The first tier includes gRNAs selected based on location in the first 500 bp of the coding sequence of the HSV gene. The second tier includes gRNAs selected based on location in the coding sequence, but downstream of the first 500 bp of the HSV gene.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID: 24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case *of S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 5A-5E, 6A-6G** or **7A-7D,** e.g., the targeting domains of 18 or more nucleotides may comprise the 17-mer gRNAs described in **Tables 5A-5E, 6A-6G or 7A-7D.** Targeting domains, disclosed herein, may comprises the 18-mer described in **Tables 5A-5E, 6A-6G or 7A-7D,** e.g., the targeting domains of 19 or more nucleotides may comprise the 18-mer gRNAs described in **Tables 5A-5E, 6A-6G or 7A-7D.** Targeting domains, disclosed herein, may comprises the 19-mer described in **Tables 5A-5E, 6A-6G or 7A-7D,** e.g., the targeting domains of 20 or more nucleotides may comprise the 19-mer gRNAs described in **Tables 5A-5E, 6A-6G or 7A-7D.** Targeting domains, disclosed herein, may comprises the 20-mer gRNAs described in **Tables 5A-5E, 6A-6G or 7A-7D,** e.g., the targeting domains of 21 or more nucleotides may comprise the 20-mer gRNAs described in **Tables 5A-5E, 6A-6G or 7A-7D.** Targeting domains, disclosed herein, may comprises the 21-mer described in **Tables 5A-5E, 6A-6G or 7A-7D,** e.g., the targeting domains of 22 or more nucleotides may comprise the 21-mer gRNAs described in **Tables 5A-5E, 6A-6G or 7A-7D.** Targeting domains, disclosed herein, may comprises the 22-mer described in **Tables 5A-5E, 6A-6G or 7A-7D,** e.g., the targeting domains of 23 or more nucleotides may comprise the 22-mer gRNAs described in **Tables 5A-5E, 6A-6G or 7A-7D.** Targeting domains, disclosed herein, may comprises the 23-mer described in **Tables 5A-5E, 6A-6G or 7A-7D,e.g.,** the targeting domains of 24 or more nucleotides may comprise the 23-mer gRNAs described in **Tables 5A-5E, 6A-6G or 7A-7D.** Targeting domains, disclosed herein, may comprises the 24-mer described in **Tables 5A-5E, 6A-6G or 7A-7D,** e.g., the targeting domains of 25 or more nucleotides may comprise the 24-mer gRNAs described in **Tables 5A-5E, 6A-6G or 7A-7D.** gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

gRNAs were identified and ranked into 5 tiers for *S. pyogenes* **(Tables 5A-5E),** and *N. meningitidis* **(Tables 7A-7D);** and 7 tiers for *S. aureus* **(Tables 6A-6G).** For *S. pyogenes,* and *N. meningitidis,* the targeting domain for tier 1 gRNA molecules were selected based on (1) distance to a target site (e.g., start codon), e.g., within 500bp (e.g., downstream) of the target site (e.g., start codon), (2) a high level of orthogonality and (3) the presence of 5'G. The targeting domain for tier 2 gRNA molecules were selected based on (1) distance to a target site (e.g., start codon), e.g., within 500bp (e.g., downstream) of the target site (e.g., start codon) and (2) a high level of orthogonality. The targeting domain for tier 3 gRNA molecules were selected based on (1) distance to a target site (e.g., start codon), e.g., within 500bp (e.g., downstream) of the target site (e.g., start codon) and (2) the presence of 5'G. The targeting domain for tier 4 gRNA molecules were selected based on distance to a target site (e.g., start codon), e.g., within 500bp (e.g., downstream) of the target site (e.g., start codon). The targeting domain for tier 5 gRNA molecules were selected based on distance to the target site (e.g., start codon), e.g., within reminder of the coding sequence, e.g., downstream of the first 500bp of coding sequence (e.g., anywhere from +500 (relative to the start codon) to the stop codon). For *S. aureus,* the targeting domain for tier 1 gRNA molecules were selected based on (1) distance to a target site (e.g., start codon), e.g., within 500bp (e.g., downstream) of the target site (e.g., start codon), (2) a high level of orthogonality, (3) the presence of 5'G and (4) PAM is NNGRRT. The targeting domain for tier 2 gRNA molecules were selected based on (1) distance to a target site (e.g., start codon), e.g., within 500bp (e.g., downstream) of the target site (e.g., start codon), (2) a high level of orthogonality, and (3) PAM is NNGRRT. The targeting domain for tier 3 gRNA molecules were selected based on (1) distance to a target site (e.g., start codon), e.g., within 500bp (e.g., downstream) of the target site (e.g., start codon) and (2) PAM is NNGRRT. The targeting domain for tier 4 gRNA molecules were selected based on (1) distance to a target site (e.g., start codon), e.g., within 500bp (e.g., downstream) of the target site (e.g., start codon) and (2) PAM is NNGRRV. The targeting domain for tier 5 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon), (2) the presence of 5'G and (3) PAM is NNGRRT. The targeting domain for tier 6 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRT. The targeting domain for tier 7 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes. S. aureus, and N. meningitidis to knock out the UL30 gene

As an example, three strategies were utilized to identify gRNAs for use with S. *pyogenes, S. aureus* and *N. meningitidis* Cas9 enzymes.

In the first strategy, guide RNAs (gRNAs) for use with the *S. pyogenes* **(Tables 2A-2C)** Cas9 were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a S. *pyogenes* Cas9 were ranked into 3 tiers.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the target position (i.e., within the first 500 bp of the coding sequence) and with a high level of orthogonality against the human genome. In order to find a pair for the dual-nickase strategy it was necessary to either extend the distance from the target position or remove the requirement for the 5'G. Tier 2 gRNAs were selected based on loction within the first 500 bp of the coding sequence in the HSV gene. Tier 3 gRNAs were selected based on their location in the coding sequence, but downstream of the first 500 bp of the HSV gene. Note that tiers are non-inclusive (each gRNA is listed only once). In certain instances, no gRNA was identified based on the criteria of the particular tier.

As discussed above, gRNAs were identified for single-gRNA nuclease cleavage as well as for a dual-gRNA paired "nickase" strategy, as indicated.

gRNAs for use with the *N. meningitidis* **(Tables 2F-2G)** and *S. aureus* (Tables **2D-2E)** Cas9s were identified manually by scanning genomic DNA sequence for the presence of PAM sequences. These gRNAs were separated into two tiers for each species. The first tier includes gRNAs selected based on location in the first 500 bp of the coding sequence of the HSV gene. The second tier includes gRNAs selected based on location in the coding sequence, but downstream of the first 500 bp of the HSV gene.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID: 24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case *of S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 8A-8E, 9A-9G or 10A-10C,** e.g., the targeting domains of 18 or more nucleotides may comprise the 17-mer gRNAs described in **Tables 8A-8E, 9A-9G or 10A-10C.** Targeting domains, disclosed herein, may comprises the 18-mer described in **Tables 8A-8E, 9A-9G or 10A-10C,** e.g., the targeting domains of 19 or more nucleotides may comprise the 18-mer gRNAs described in **Tables 8A-8E, 9A-9G or 10A-10C.** Targeting domains, disclosed herein, may comprises the 19-mer described in **Tables 8A-8E, 9A-9G or 10A-10C,** e.g., the targeting domains of 20 or more nucleotides may comprise the 19-mer gRNAs described in **Tables 8A-8E, 9A-9G or 10A-10C.** Targeting domains, disclosed herein, may comprises the 20-mer gRNAs described in **Tables 8A-8E, 9A-9G or 10A-10C,** e.g., the targeting domains of 21 or more nucleotides may comprise the 20-mer gRNAs described in **Tables 8A-8E, 9A-9G or 10A-10C.** Targeting domains, disclosed herein, may comprises the 21-mer described in **Tables 8A-8E, 9A-9G or 10A-10C,** e.g., the targeting domains of 22 or more nucleotides may comprise the 21-mer gRNAs described in **Tables 8A-8E, 9A-9G or 10A-10C.** Targeting domains, disclosed herein, may comprises the 22-mer described in **Tables 8A-8E, 9A-9G or 10A-10C,** e.g., the targeting domains of 23 or more nucleotides may comprise the 22-mer gRNAs described in **Tables 8A-8E, 9A-9G or 10A-10C.** Targeting domains, disclosed herein, may comprises the 23-mer described in **Tables 8A-8E, 9A-9G or 10A-10C,e.g.,** the targeting domains of 24 or more nucleotides may comprise the 23-mer gRNAs described in **Tables 8A-8E, 9A-9G or 10A-10C.** Targeting domains, disclosed herein, may comprises the 24-mer described in **Tables 8A-8E, 9A-9G or 10A-10C,** e.g., the targeting domains of 25 or more nucleotides may comprise the 24-mer gRNAs described in **Tables 8A-8E, 9A-9G or 10A-10C.**gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

gRNAs were identified and ranked into 5 tiers for *S. pyogenes* **(Tables 8A-8E),** and *N. meningitidis* **(Tables 10A-10C)**; and 7 tiers for *S. aureus* **(Tables 9A-9G).** For S. *pyogenes,* and *N. meningitidis,* the targeting domain for tier 1 gRNA molecules were selected based on (1) distance to a target site (e.g., start codon), e.g., within 500bp (e.g., downstream) of the target site (e.g., start codon), (2) a high level of orthogonality and (3) the presence of 5'G. The targeting domain for tier 2 gRNA molecules were selected based on (1) distance to a target site (e.g., start codon), e.g., within 500bp (e.g., downstream) of the target site (e.g., start codon) and (2) a high level of orthogonality. The targeting domain for tier 3 gRNA molecules were selected based on (1) distance to a target site (e.g., start codon), e.g., within 500bp (e.g., downstream) of the target site (e.g., start codon) and (2) the presence of 5'G. The targeting domain for tier 4 gRNA molecules were selected based on distance to a target site (e.g., start codon), e.g., within 500bp (e.g., downstream) of the target site (e.g., start codon). The targeting domain for tier 5 gRNA molecules were selected based on distance to the target site (e.g., start codon), e.g., within reminder of the coding sequence, e.g., downstream of the first 500bp of coding sequence (e.g., anywhere from +500 (relative to the start codon) to the stop codon). For *S. aureus,* the targeting domain for tier 1 gRNA molecules were selected based on (1) distance to a target site (e.g., start codon), e.g., within 500bp (e.g., downstream) of the target site (e.g., start codon), (2) a high level of orthogonality, (3) the presence of 5'G and (4) PAM is NNGRRT. The targeting domain for tier 2 gRNA molecules were selected based on (1) distance to a target site (e.g., start codon), e.g., within 500bp (e.g., downstream) of the target site (e.g., start codon), (2) a high level of orthogonality, and (3) PAM is NNGRRT. The targeting domain for tier 3 gRNA molecules were selected based on (1) distance to a target site (e.g., start codon), e.g., within 500bp (e.g., downstream) of the target site (e.g., start codon) and (2) PAM is NNGRRT. The targeting domain for tier 4 gRNA molecules were selected based on (1) distance to a target site (e.g., start codon), e.g., within 500bp (e.g., downstream) of the target site (e.g., start codon) and (2) PAM is NNGRRV. The targeting domain for tier 5 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon), (2) the presence of 5'G and (3) PAM is NNGRRT. The targeting domain for tier 6 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRT. The targeting domain for tier 7 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes. S. aureus, and N. meningitidis to knock out the UL48 gene

As an example, three strategies were utilized to identify gRNAs for use with S. *pyogenes, S. aureus* and *N. meningitidis* Cas9 enzymes.

In the first strategy, guide RNAs (gRNAs) for use with the *S. pyogenes* **(Tables 3A-3C)** Cas9 were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a S. *pyogenes* Cas9 were ranked into 3 tiers.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the target position (i.e., within the first 500 bp of the coding sequence) and with a high level of orthogonality against the human genome. In order to find a pair for the dual-nickase strategy it was necessary to either extend the distance from the target position or remove the requirement for the 5'G. Tier 2 gRNAs were selected based on loction within the first 500 bp of the coding sequence in the HSV gene. Tier 3 gRNAs were selected based on their location in the coding sequence, but downstream of the first 500 bp of the HSV gene. Note that tiers are non-inclusive (each gRNA is listed only once). In certain instances, no gRNA was identified based on the criteria of the particular tier.

As discussed above, gRNAs were identified for single-gRNA nuclease cleavage as well as for a dual-gRNA paired "nickase" strategy, as indicated.

gRNAs for use with the *N. meningitidis* **(Tables 3F-3G)** and *S. aureus* **(Tables 3D-3E)** Cas9s were identified manually by scanning genomic DNA sequence for the presence of PAM sequences. These gRNAs were separated into two tiers for each species. The first tier includes gRNAs selected based on location in the first 500 bp of the coding sequence of the HSV gene. The second tier includes gRNAs selected based on location in the coding sequence, but downstream of the first 500 bp of the HSV gene.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID: 24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case *of S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 11A-11E, 12A-12G** or **13A-13C,** e.g., the targeting domains of 18 or more nucleotides may comprise the 17-mer gRNAs described in **Tables 11A-11E, 12A-12G or 13A-13C.** Targeting domains, disclosed herein, may comprises the 18-mer described in **Tables 11A-11E, 12A-12G or 13A-13C,** e.g., the targeting domains of 19 or more nucleotides may comprise the 18-mer gRNAs described in **Tables 11A-11E, 12A-12G or 13A-13C.** Targeting domains, disclosed herein, may comprises the 19-mer described in **Tables 11A-11E, 12A-12G or 13A-13C,** e.g., the targeting domains of 20 or more nucleotides may comprise the 19-mer gRNAs described in **Tables 11A-11E, 12A-12G or 13A-13C.** Targeting domains, disclosed herein, may comprises the 20-mer gRNAs described in **Tables 11A-11E, 12A-12G or 13A-13C,** e.g., the targeting domains of 21 or more nucleotides may comprise the 20-mer gRNAs described in **Tables 11A-11E, 12A-12G or 13A-13C.** Targeting domains, disclosed herein, may comprises the 21-mer described in **Tables 11A-11E, 12A-12G or 13A-13C,** e.g., the targeting domains of 22 or more nucleotides may comprise the 21-mer gRNAs described in **Tables 11A-11E, 12A-12G or 13A-13C.** Targeting domains, disclosed herein, may comprises the 22-mer described in **Tables 11A-11E, 12A-12G or 13A-13C,** e.g., the targeting domains of 23 or more nucleotides may comprise the 22-mer gRNAs described in **Tables 11A-11E, 12A-12G or 13A-13C.** Targeting domains, disclosed herein, may comprises the 23-mer described in **Tables 11A-11E, 12A-12G or 13A-13C,**e.g., the targeting domains of 24 or more nucleotides may comprise the 23-mer gRNAs described in **Tables 11A-11E, 12A-12G or 13A-13C.** Targeting domains, disclosed herein, may comprises the 24-mer described in **Tables 11A-11E, 12A-12G or 13A-13C,** e.g., the targeting domains of 25 or more nucleotides may comprise the 24-mer gRNAs described in **Tables 11A-11E, 12A-12G or 13A-13C**.gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

gRNAs were identified and ranked into 5 tiers for *S. pyogenes* **(Tables 11A-11E),** and *N. meningitidis* **(Tables 13A-13C);** and 7 tiers for *S. aureus* **(Tables 12A-12G).** For S. *pyogenes,* and *N. meningitidis,* the targeting domain for tier 1 gRNA molecules were selected based on (1) distance to a target site (e.g., start codon), e.g., within 500bp (e.g., downstream) of the target site (e.g., start codon), (2) a high level of orthogonality and (3) the presence of 5'G. The targeting domain for tier 2 gRNA molecules were selected based on (1) distance to a target site (e.g., start codon), e.g., within 500bp (e.g., downstream) of the target site (e.g., start codon) and (2) a high level of orthogonality. The targeting domain for tier 3 gRNA molecules were selected based on (1) distance to a target site (e.g., start codon), e.g., within 500bp (e.g., downstream) of the target site (e.g., start codon) and (2) the presence of 5'G. The targeting domain for tier 4 gRNA molecules were selected based on distance to a target site (e.g., start codon), e.g., within 500bp (e.g., downstream) of the target site (e.g., start codon). The targeting domain for tier 5 gRNA molecules were selected based on distance to the target site (e.g., start codon), e.g., within reminder of the coding sequence, e.g., downstream of the first 500bp of coding sequence (e.g., anywhere from +500 (relative to the start codon) to the stop codon). For *S. aureus,* the targeting domain for tier 1 gRNA molecules were selected based on (1) distance to a target site (e.g., start codon), e.g., within 500bp (e.g., downstream) of the target site (e.g., start codon), (2) a high level of orthogonality, (3) the presence of 5'G and (4) PAM is NNGRRT. The targeting domain for tier 2 gRNA molecules were selected based on (1) distance to a target site (e.g., start codon), e.g., within 500bp (e.g., downstream) of the target site (e.g., start codon), (2) a high level of orthogonality, and (3) PAM is NNGRRT. The targeting domain for tier 3 gRNA molecules were selected based on (1) distance to a target site (e.g., start codon), e.g., within 500bp (e.g., downstream) of the target site (e.g., start codon) and (2) PAM is NNGRRT. The targeting domain for tier 4 gRNA molecules were selected based on (1) distance to a target site (e.g., start codon), e.g., within 500bp (e.g., downstream) of the target site (e.g., start codon) and (2) PAM is NNGRRV. The targeting domain for tier 5 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon), (2) the presence of 5'G and (3) PAM is NNGRRT. The targeting domain for tier 6 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRT. The targeting domain for tier 7 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes. S. aureus, and N. meningitidis to knock out the UL54 gene

As an example, three strategies were utilized to identify gRNAs for use with S. *pyogenes, S. aureus* and *N. meningitidis* Cas9 enzymes.

In the first strategy, guide RNAs (gRNAs) for use with the *S. pyogenes* **(Tables 4A-4C)** Cas9 were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a S. *pyogenes* Cas9 were ranked into 3 tiers.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the target position (i.e., within the first 500 bp of the coding sequence) and with a high level of orthogonality against the human genome. In order to find a pair for the dual-nickase strategy it was necessary to either extend the distance from the target position or remove the requirement for the 5'G. Tier 2 gRNAs were selected based on location within the first 500 bp of the coding sequence in the HSV gene. Tier 3 gRNAs were selected based on their location in the coding sequence, but downstream of the first 500 bp of the HSV gene. Note that tiers are non-inclusive (each gRNA is listed only once). In certain instances, no gRNA was identified based on the criteria of the particular tier.

As discussed above, gRNAs were identified for single-gRNA nuclease cleavage as well as for a dual-gRNA paired "nickase" strategy, as indicated.

gRNAs for use with the *N. meningitidis* **(Tables 4F)** and *S. aureus* **(Tables 4D-4E)** Cas9s were identified manually by scanning genomic DNA sequence for the presence of PAM sequences. These gRNAs were separated into two tiers for each species. The first tier includes gRNAs selected based on location in the first 500 bp of the coding sequence of the HSV gene. The second tier includes gRNAs selected based on location in the coding sequence, but downstream of the first 500 bp of the HSV gene.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID: 24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case *of S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 14A-14E, 15A-15G or 16A-16C,** e.g., the targeting domains of 18 or more nucleotides may comprise the 17-mer gRNAs described in **Tables 14A-14E, 15A-15G or 16A-16C.** Targeting domains, disclosed herein, may comprises the 18-mer described in **Tables 14A-14E, 15A-15G or 16A-16C,** e.g., the targeting domains of 19 or more nucleotides may comprise the 18-mer gRNAs described in **Tables 14A-14E, 15A-15G or 16A-16C.** Targeting domains, disclosed herein, may comprises the 19-mer described in **Tables 14A-14E, 15A-15G or 16A-16C,** e.g., the targeting domains of 20 or more nucleotides may comprise the 19-mer gRNAs described in **Tables 14A-14E, 15A-15G or 16A-16C.** Targeting domains, disclosed herein, may comprises the 20-mer gRNAs described in **Tables 14A-14E, 15A-15G or 16A-16C,** e.g., the targeting domains of 21 or more nucleotides may comprise the 20-mer gRNAs described in **Tables 14A-14E, 15A-15G or 16A-16C.** Targeting domains, disclosed herein, may comprises the 21-mer described in **Tables 14A-14E, 15A-15G or 16A-16C,** e.g., the targeting domains of 22 or more nucleotides may comprise the 21-mer gRNAs described in **Tables 14A-14E, 15A-15G or 16A-16C.** Targeting domains, disclosed herein, may comprises the 22-mer described in **Tables 14A-14E, 15A-15G or 16A-16C,** e.g., the targeting domains of 23 or more nucleotides may comprise the 22-mer gRNAs described in **Tables 14A-14E, 15A-15G or 16A-16C.** Targeting domains, disclosed herein, may comprises the 23-mer described in **Tables 14A-14E, 15A-15G or** 16A-16C,e.g., the targeting domains of 24 or more nucleotides may comprise the 23-mer gRNAs described in Tables **14A-14E, 15A-15G or 16A-16C.** Targeting domains, disclosed herein, may comprises the 24-mer described in **Tables 14A-14E, 15A-15G or 16A-16C,** e.g., the targeting domains of 25 or more nucleotides may comprise the 24-mer gRNAs described in **Tables 14A-14E, 15A-15G or 16A-16C**.gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

gRNAs were identified and ranked into 5 tiers for *S. pyogenes* **(Tables 14A-14E),** and *N. meningitidis* **(Tables 16A-16C);** and 7 tiers for *S. aureus* **(Tables 15A-15G).** For S. *pyogenes,* and *N. meningitidis,* the targeting domain for tier 1 gRNA molecules were selected based on (1) distance to a target site (e.g., start codon), e.g., within 500bp (e.g., downstream) of the target site (e.g., start codon), (2) a high level of orthogonality and (3) the presence of 5'G. The targeting domain for tier 2 gRNA molecules were selected based on (1) distance to a target site (e.g., start codon), e.g., within 500bp (e.g., downstream) of the target site (e.g., start codon) and (2) a high level of orthogonality. The targeting domain for tier 3 gRNA molecules were selected based on (1) distance to a target site (e.g., start codon), e.g., within 500bp (e.g., downstream) of the target site (e.g., start codon) and (2) the presence of 5'G. The targeting domain for tier 4 gRNA molecules were selected based on distance to a target site (e.g., start codon), e.g., within 500bp (e.g., downstream) of the target site (e.g., start codon). The targeting domain for tier 5 gRNA molecules were selected based on distance to the target site (e.g., start codon), e.g., within reminder of the coding sequence, e.g., downstream of the first 500bp of coding sequence (e.g., anywhere from +500 (relative to the start codon) to the stop codon). For *S. aureus,* the targeting domain for tier 1 gRNA molecules were selected based on (1) distance to a target site (e.g., start codon), e.g., within 500bp (e.g., downstream) of the target site (e.g., start codon), (2) a high level of orthogonality, (3) the presence of 5'G and (4) PAM is NNGRRT. The targeting domain for tier 2 gRNA molecules were selected based on (1) distance to a target site (e.g., start codon), e.g., within 500bp (e.g., downstream) of the target site (e.g., start codon), (2) a high level of orthogonality, and (3) PAM is NNGRRT. The targeting domain for tier 3 gRNA molecules were selected based on (1) distance to a target site (e.g., start codon), e.g., within 500bp (e.g., downstream) of the target site (e.g., start codon) and (2) PAM is NNGRRT. The targeting domain for tier 4 gRNA molecules were selected based on (1) distance to a target site (e.g., start codon), e.g., within 500bp (e.g., downstream) of the target site (e.g., start codon) and (2) PAM is NNGRRV. The targeting domain for tier 5 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon), (2) the presence of 5'G and (3) PAM is NNGRRT. The targeting domain for tier 6 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRT. The targeting domain for tier 7 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRV. Note that tiers are non-inclusive (each gRNA is listed only once for the strategy). In certain instances, no gRNA was identified based on the criteria of the particular tier.

In an embodiment, two or more (e.g., three or four) gRNA molecules are used with one Cas9 molecule. In another embodiment, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

Any of the targeting domains in the tables described herein can be used with a Cas9 nickase molecule to generate a single strand break.

Any of the targeting domains in the tables described herein can be used with a Cas9 nuclease molecule to generate a double strand break.

When two gRNAs designed for use to target two Cas9 molecules, one Cas9 can be one species, the second Cas9 can be from a different species. Both Cas9 species are used to generate a single or double-strand break, as desired.

It is contemplated herein that any upstream gRNA described herein may be paired with any downstream gRNA described herein. When an upstream gRNA designed for use with one species of Cas9 is paired with a downstream gRNA designed for use from a different species of Cas9, both Cas9 species are used to generate a single or double-strand break, as desired.

### Exemplary Targeting Domains

**Table 1A** provides exemplary targeting domains for knocking out the *UL19* gene selected according to first tier parameters. The targeting domains are selected based on location within the first 500bp of the coding sequence of the *UL19* gene and orthogonality against the human genome. It is contemplated herein that the targeting domain hybridizes to the target domain through complementary base pairing. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1A**

| 1st Tier | | | | |
|---|---|---|---|---|
| gRNA Name | DNA Strand | Targeting Domain | Target Site Length | Seq ID |
| HSV1-UL19-3 | - | ACCGCGACCCUCCGGGAUAC | 20 | 387 |
| HSV1-UL19-4 | - | UCCGGGAUACCGGUAUGCCG | 20 | 388 |
| HSV1-UL19-5 | - | AUACCGGUAUGCCGCGGCCA | 20 | 389 |
| HSV1-UL19-97 | - | GCGACCCUCCGGGAUAC | 17 | 390 |
| HSV1-UL19-98 | - | GGGAUACCGGUAUGCCG | 17 | 391 |
| HSV1-UL19-99 | - | CCGGUAUGCCGCGGCCA | 17 | 392 |
| HSV1-UL19-11 | - | GUCGAGUUCGACGCCCUGCU | 20 | 393 |
| HSV1-UL19-12 | - | UCGAGUUCGACGCCCUGCUG | 20 | 394 |
| HSV1-UL19-13 | - | CCUGUCGCUCGUGCGCUUUC | 20 | 395 |
| HSV1-UL19-14 | - | CUCGUGCGCUUUCUGGAGCU | 20 | 396 |
| HSV1-UL19-15 | - | UCGUGCGCUUUCUGGAGCUC | 20 | 397 |
| HSV1-UL19-105 | - | GAGUUCGACGCCCUGCU | 17 | 398 |
| HSV1-UL19-106 | - | AGUUCGACGCCCUGCUG | 17 | 399 |
| HSV1-UL19-107 | - | GUCGCUCGUGCGCUUUC | 17 | 400 |
| HSV1-UL19-108 | - | GUGCGCUUUCUGGAGCU | 17 | 401 |
| HSV1-UL19-109 | - | UGCGCUUUCUGGAGCUC | 17 | 402 |
| HSV1-UL19-17 | - | UCUGGAGCUCGGGUUGUCGG | 20 | 403 |
| HSV1-UL19-18 | - | GUGCGUGUGUACCAAGUUUC | 20 | 404 |
| HSV1-UL19-19 | - | GUGUACCAAGUUUCCGGAGC | 20 | 405 |
| HSV1-UL19-111 | - | GGAGCUCGGGUUGUCGG | 17 | 406 |
| HSV1-UL19-112 | - | CGUGUGUACCAAGUUUC | 17 | 407 |
| HSV1-UL19-113 | - | UACCAAGUUUCCGGAGC | 17 | 408 |
| HSV1-UL19-23 | - | CGAGGGGCGCGUACAGUUCG | 20 | 409 |
| HSV1-UL19-24 | - | CAGCCGCUGAUUGCCCGCGA | 20 | 410 |
| HSV1-UL19-25 | - | UGACCAAGAUUAUCGACCGC | 20 | 411 |
| HSV1-UL19-26 | - | GACCAAGAUUAUCGACCGCC | 20 | 412 |
| HSV1-UL19-117 | - | GGGGCGCGUACAGUUCG | 17 | 413 |
| HSV1-UL19-118 | - | CCGCUGAUUGCCCGCGA | 17 | 414 |
| HSV1-UL19-119 | - | CCAAGAUUAUCGACCGC | 17 | 415 |
| HSV1-UL19-120 | - | CAAGAUUAUCGACCGCC | 17 | 416 |
| HSV1-UL19-31 | - | AGGCCAUCGCCCUGCUCACG | 20 | 417 |
| HSV1-UL19-125 | - | CCAUCGCCCUGCUCACG | 17 | 418 |
| HSV1-UL19-33 | - | CAUCGCCCUGCUCACGGGGG | 20 | 419 |
| HSV1-UL19-127 | - | CGCCCUGCUCACGGGGG | 17 | 420 |
| HSV1-UL19-40 | + | GCUGUUGGAUGGCGCGCAAC | 20 | 421 |
| HSV1-UL19-134 | + | GUUGGAUGGCGCGCAAC | 17 | 422 |
| HSV1-UL19-41 | + | CGCCGAUACCCGUCCCGUCC | 20 | 423 |
| HSV1-UL19-135 | + | CGAUACCCGUCCCGUCC | 17 | 424 |
| HSV1-UL19-58 | + | AUUGUGUGUGGGUUGCUCGA | 20 | 425 |
| HSV1-UL19-59 | + | UUGUGUGUGGGUUGCUCGAU | 20 | 426 |
| HSV1-UL19-152 | + | GUGUGUGGGUUGCUCGA | 17 | 427 |
| HSV1-UL19-153 | + | UGUGUGGGUUGCUCGAU | 17 | 428 |
| HSV1-UL19-64 | + | GAUGGGGUGCGGGCCGUCGC | 20 | 429 |
| HSV1-UL19-65 | + | GGGCCGUCGCGGGCAAUCAG | 20 | 430 |
| HSV1-UL19-158 | + | GGGGUGCGGGCCGUCGC | 17 | 431 |
| HSV1-UL19-159 | + | CCGUCGCGGGCAAUCAG | 17 | 432 |
| HSV1-UL19-67 | + | CGCGGGCAAUCAGCGGCUGG | 20 | 433 |
| HSV1-UL19-161 | + | GGGCAAUCAGCGGCUGG | 17 | 434 |
| HSV1-UL19-69 | + | UCGUUCAUGUAGGCCAGCUC | 20 | 435 |
| HSV1-UL19-70 | + | GUAGGCCAGCUCCGGAAACU | 20 | 436 |
| HSV1-UL19-71 | + | CCAGAAAGCGCACGAGCGAC | 20 | 437 |
| HSV1-UL19-72 | + | CAGAAAGCGCACGAGCGACA | 20 | 438 |
| HSV1-UL19-73 | + | UGUUGCAAUACGACCCCAGC | 20 | 439 |
| HSV1-UL19-74 | + | GUUGCAAUACGACCCCAGCA | 20 | 440 |
| HSV1-UL19-75 | + | CGUCGAACUCGACGUCGUAC | 20 | 441 |
| HSV1-UL19-76 | + | GUCGUACAGGCUGUUUGCAU | 20 | 442 |
| HSV1-UL19-163 | + | UUCAUGUAGGCCAGCUC | 17 | 443 |
| HSV1-UL19-164 | + | GGCCAGCUCCGGAAACU | 17 | 444 |
| HSV1-UL19-165 | + | GAAAGCGCACGAGCGAC | 17 | 445 |
| HSV1-UL19-166 | + | AAAGCGCACGAGCGACA | 17 | 446 |
| HSV1-UL19-167 | + | UGCAAUACGACCCCAGC | 17 | 447 |
| HSV1-UL19-168 | + | GCAAUACGACCCCAGCA | 17 | 448 |
| HSV1-UL19-169 | + | CGAACUCGACGUCGUAC | 17 | 449 |
| HSV1-UL19-170 | + | GUACAGGCUGUUUGCAU | 17 | 450 |
| HSV1-UL19-82 | + | CACCUCGAUCGUGCUAAGGA | 20 | 451 |
| HSV1-UL19-176 | + | CUCGAUCGUGCUAAGGA | 17 | 452 |
| HSV1-UL19-87 | + | GCACCAUGGCCGCGGCAUAC | 20 | 453 |
| HSV1-UL19-181 | + | CCAUGGCCGCGGCAUAC | 17 | 454 |
| HSV1-UL19-91 | + | ACCGGUAUCCCGGAGGGUCG | 20 | 455 |
| HSV1-UL19-92 | + | GUAUCCCGGAGGGUCGCGGU | 20 | 456 |
| HSV1-UL19-93 | + | UAUCCCGGAGGGUCGCGGUU | 20 | 457 |
| HSV1-UL19-185 | + | GGUAUCCCGGAGGGUCG | 17 | 458 |
| HSV1-UL19-186 | + | UCCCGGAGGGUCGCGGU | 17 | 459 |
| HSV1-UL19-187 | + | CCCGGAGGGUCGCGGUU | 17 | 460 |

**Table 1B** provides exemplary targeting domains for knocking out the *UL19* gene selected according to the second tier parameters. The targeting domains are selected based on location within the first 500bp of the coding sequence of the *UL19* gene. It is contemplated herein that the targeting domain hybridizes to the target domain through complementary base pairing. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1B**

| 2nd Tier | | | | |
|---|---|---|---|---|
| gRNA Name | DNA Strand | Targeting Domain | Target Site Length | Seq ID |
| HSV1-UL19-1 | - | CGCUCCCAACCGCGACCCUC | 20 | 461 |
| HSV1-UL19-2 | - | GCUCCCAACCGCGACCCUCC | 20 | 462 |
| HSV1-UL19-6 | - | GCCGCGGCCAUGGUGCCGAC | 20 | 463 |
| HSV1-UL19-7 | - | CCGCGGCCAUGGUGCCGACC | 20 | 464 |
| HSV1-UL19-8 | - | GUCCCUCCUUAGCACGAUCG | 20 | 465 |
| HSV1-UL19-9 | - | CCUCCUUAGCACGAUCGAGG | 20 | 466 |
| HSV1-UL19-10 | - | CGUCGAGUUCGACGCCCUGC | 20 | 467 |
| HSV1-UL19-16 | - | CUUUCUGGAGCUCGGGUUGU | 20 | 468 |
| HSV1-UL19-20 | - | GGAGCUGGCCUACAUGAACG | 20 | 469 |
| HSV1-UL19-21 | - | GAGCUGGCCUACAUGAACGA | 20 | 470 |
| HSV1-UL19-22 | - | AGCUGGCCUACAUGAACGAG | 20 | 471 |
| HSV1-UL19-27 | - | CCUGAACGCCGCCUUCAGCC | 20 | 472 |
| HSV1-UL19-28 | - | CGCCUUCAGCCUGGCCACCG | 20 | 473 |
| HSV1-UL19-29 | - | CGAGGCCAUCGCCCUGCUCA | 20 | 474 |
| HSV1-UL19-30 | - | GAGGCCAUCGCCCUGCUCAC | 20 | 475 |
| HSV1-UL19-32 | - | GGCCAUCGCCCUGCUCACGG | 20 | 476 |
| HSV1-UL19-34 | - | CCUGCUCACGGGGGAGGCCC | 20 | 477 |
| HSV1-UL19-35 | - | CUCACGGGGGAGGCCCUGGA | 20 | 478 |
| HSV1-UL19-36 | - | UCACGGGGGAGGCCCUGGAC | 20 | 479 |
| HSV1-UL19-37 | - | GGGGGAGGCCCUGGACGGGA | 20 | 480 |
| HSV1-UL19-38 | - | GGGGAGGCCCUGGACGGGAC | 20 | 481 |
| HSV1-UL19-39 | - | GCCCUGGACGGGACGGGUAU | 20 | 482 |
| HSV1-UL19-42 | + | GCCGAUACCCGUCCCGUCCA | 20 | 483 |
| HSV1-UL19-43 | + | CCAGGGCCUCCCCCGUGAGC | 20 | 484 |
| HSV1-UL19-44 | + | CAGGGCCUCCCCCGUGAGCA | 20 | 485 |
| HSV1-UL19-45 | + | CUCCCCCGUGAGCAGGGCGA | 20 | 486 |
| HSV1-UL19-46 | + | CGUGAGCAGGGCGAUGGCCU | 20 | 487 |
| HSV1-UL19-47 | + | GAGCAGGGCGAUGGCCUCGG | 20 | 488 |
| HSV1-UL19-48 | + | GGGCGAUGGCCUCGGUGGCC | 20 | 489 |
| HSV1-UL19-49 | + | GGCCUCGGUGGCCAGGCUGA | 20 | 490 |
| HSV1-UL19-50 | + | CUCGGUGGCCAGGCUGAAGG | 20 | 491 |
| HSV1-UL19-51 | + | CCAGGCUGAAGGCGGCGUUC | 20 | 492 |
| HSV1-UL19-52 | + | CAGGCUGAAGGCGGCGUUCA | 20 | 493 |
| HSV1-UL19-53 | + | UGAAGGCGGCGUUCAGGGCC | 20 | 494 |
| HSV1-UL19-54 | + | AGGCGGCGUUCAGGGCCCGG | 20 | 495 |
| HSV1-UL19-55 | + | GGCCCGGCGGUCGAUAAUCU | 20 | 496 |
| HSV1-UL19-56 | + | CUUGGUCAUGUAAUUGUGUG | 20 | 497 |
| HSV1-UL19-57 | + | UUGGUCAUGUAAUUGUGUGU | 20 | 498 |
| HSV1-UL19-60 | + | UGUGUGUGGGUUGCUCGAUG | 20 | 499 |
| HSV1-UL19-61 | + | GUGGGUUGCUCGAUGGGGUG | 20 | 500 |
| HSV1-UL19-62 | + | UGGGUUGCUCGAUGGGGUGC | 20 | 501 |
| HSV1-UL19-63 | + | CGAUGGGGUGCGGGCCGUCG | 20 | 502 |
| HSV1-UL19-66 | + | CGUCGCGGGCAAUCAGCGGC | 20 | 503 |
| HSV1-UL19-68 | + | UACGCGCCCCUCGUUCAUGU | 20 | 504 |
| HSV1-UL19-77 | + | UGUUUGCAUCGGAGCGCACG | 20 | 505 |
| HSV1-UL19-78 | + | GUUUGCAUCGGAGCGCACGC | 20 | 506 |
| HSV1-UL19-79 | + | CGCGGGAAAAAAAAUCAAAC | 20 | 507 |
| HSV1-UL19-80 | + | ACGCCACCUCGAUCGUGCUA | 20 | 508 |
| HSV1-UL19-81 | + | CCACCUCGAUCGUGCUAAGG | 20 | 509 |
| HSV1-UL19-83 | + | GAUCGUGCUAAGGAGGGACC | 20 | 510 |
| HSV1-UL19-84 | + | GUGCUAAGGAGGGACCCGGU | 20 | 511 |
| HSV1-UL19-85 | + | GAGGGACCCGGUCGGCACCA | 20 | 512 |
| HSV1-UL19-86 | + | CCCGGUCGGCACCAUGGCCG | 20 | 513 |
| HSV1-UL19-88 | + | GCCGCGGCAUACCGGUAUCC | 20 | 514 |
| HSV1-UL19-89 | + | GCGGCAUACCGGUAUCCCGG | 20 | 515 |
| HSV1-UL19-90 | + | CGGCAUACCGGUAUCCCGGA | 20 | 516 |
| HSV1-UL19-94 | + | CGGAGGGUCGCGGUUGGGAG | 20 | 517 |
| HSV1-UL19-95 | - | UCCCAACCGCGACCCUC | 17 | 518 |
| HSV1-UL19-96 | - | CCCAACCGCGACCCUCC | 17 | 519 |
| HSV1-UL19-100 | - | GCGGCCAUGGUGCCGAC | 17 | 520 |
| HSV1-UL19-101 | - | CGGCCAUGGUGCCGACC | 17 | 521 |
| HSV1-UL19-102 | - | CCUCCUUAGCACGAUCG | 17 | 522 |
| HSV1-UL19-103 | - | CCUUAGCACGAUCGAGG | 17 | 523 |
| HSV1-UL19-104 | - | CGAGUUCGACGCCCUGC | 17 | 524 |
| HSV1-UL19-110 | - | UCUGGAGCUCGGGUUGU | 17 | 525 |
| HSV1-UL19-114 | - | GCUGGCCUACAUGAACG | 17 | 526 |
| HSV1-UL19-115 | - | CUGGCCUACAUGAACGA | 17 | 527 |
| HSV1-UL19-116 | - | UGGCCUACAUGAACGAG | 17 | 528 |
| HSV1-UL19-121 | - | GAACGCCGCCUUCAGCC | 17 | 529 |
| HSV1-UL19-122 | - | CUUCAGCCUGGCCACCG | 17 | 530 |
| HSV1-UL19-123 | - | GGCCAUCGCCCUGCUCA | 17 | 531 |
| HSV1-UL19-124 | - | GCCAUCGCCCUGCUCAC | 17 | 532 |
| HSV1-UL19-126 | - | CAUCGCCCUGCUCACGG | 17 | 533 |
| HSV1-UL19-128 | - | GCUCACGGGGGAGGCCC | 17 | 534 |
| HSV1-UL19-129 | - | ACGGGGGAGGCCCUGGA | 17 | 535 |
| HSV1-UL19-130 | - | CGGGGGAGGCCCUGGAC | 17 | 536 |
| HSV1-UL19-131 | - | GGAGGCCCUGGACGGGA | 17 | 537 |
| HSV1-UL19-132 | - | GAGGCCCUGGACGGGAC | 17 | 538 |
| HSV1-UL19-133 | - | CUGGACGGGACGGGUAU | 17 | 539 |
| HSV1-UL19-136 | + | GAUACCCGUCCCGUCCA | 17 | 540 |
| HSV1-UL19-137 | + | GGGCCUCCCCCGUGAGC | 17 | 541 |
| HSV1-UL19-138 | + | GGCCUCCCCCGUGAGCA | 17 | 542 |
| HSV1-UL19-139 | + | CCCCGUGAGCAGGGCGA | 17 | 543 |
| HSV1-UL19-140 | + | GAGCAGGGCGAUGGCCU | 17 | 544 |
| HSV1-UL19-141 | + | CAGGGCGAUGGCCUCGG | 17 | 545 |
| HSV1-UL19-142 | + | CGAUGGCCUCGGUGGCC | 17 | 546 |
| HSV1-UL19-143 | + | CUCGGUGGCCAGGCUGA | 17 | 547 |
| HSV1-UL19-144 | + | GGUGGCCAGGCUGAAGG | 17 | 548 |
| HSV1-UL19-145 | + | GGCUGAAGGCGGCGUUC | 17 | 549 |
| HSV1-UL19-146 | + | GCUGAAGGCGGCGUUCA | 17 | 550 |
| HSV1-UL19-147 | + | AGGCGGCGUUCAGGGCC | 17 | 551 |
| HSV1-UL19-148 | + | CGGCGUUCAGGGCCCGG | 17 | 552 |
| HSV1-UL19-149 | + | CCGGCGGUCGAUAAUCU | 17 | 553 |
| HSV1-UL19-150 | + | GGUCAUGUAAUUGUGUG | 17 | 554 |
| HSV1-UL19-151 | + | GUCAUGUAAUUGUGUGU | 17 | 555 |
| HSV1-UL19-154 | + | GUGUGGGUUGCUCGAUG | 17 | 556 |
| HSV1-UL19-155 | + | GGUUGCUCGAUGGGGUG | 17 | 557 |
| HSV1-UL19-156 | + | GUUGCUCGAUGGGGUGC | 17 | 558 |
| HSV1-UL19-157 | + | UGGGGUGCGGGCCGUCG | 17 | 559 |
| HSV1-UL19-160 | + | CGCGGGCAAUCAGCGGC | 17 | 560 |
| HSV1-UL19-162 | + | GCGCCCCUCGUUCAUGU | 17 | 561 |
| HSV1-UL19-171 | + | UUGCAUCGGAGCGCACG | 17 | 562 |
| HSV1-UL19-172 | + | UGCAUCGGAGCGCACGC | 17 | 563 |
| HSV1-UL19-173 | + | GGGAAAAAAAAUCAAAC | 17 | 564 |
| HSV1-UL19-174 | + | CCACCUCGAUCGUGCUA | 17 | 565 |
| HSV1-UL19-175 | + | CCUCGAUCGUGCUAAGG | 17 | 566 |
| HSV1-UL19-177 | + | CGUGCUAAGGAGGGACC | 17 | 567 |
| HSV1-UL19-178 | + | CUAAGGAGGGACCCGGU | 17 | 568 |
| HSV1-UL19-179 | + | GGACCCGGUCGGCACCA | 17 | 569 |
| HSV1-UL19-180 | + | GGUCGGCACCAUGGCCG | 17 | 570 |
| HSV1-UL19-182 | + | GCGGCAUACCGGUAUCC | 17 | 571 |
| HSV1-UL19-183 | + | GCAUACCGGUAUCCCGG | 17 | 572 |
| HSV1-UL19-184 | + | CAUACCGGUAUCCCGGA | 17 | 573 |
| HSV1-UL19-188 | + | AGGGUCGCGGUUGGGAG | 17 | 574 |

**Table 1C** provides exemplary targeting domains for knocking out the *UL19* gene selected according to the third tier parameters. The targeting domains are selected based on location within the coding sequence, but downstream of the first 500bp of the *UL19* gene. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1C**

| 3rd Tier | | | | |
|---|---|---|---|---|
| gRNA Name | DNA Strand | Targeting Domain | Target Site Length | Seq ID |
| HSV1-UL19-189 | - | CGCAACGUCCAGGCCGUCCU | 20 | 575 |
| HSV1-UL19-190 | - | GCAACGUCCAGGCCGUCCUC | 20 | 576 |
| HSV1-UL19-191 | - | CAACGUCCAGGCCGUCCUCG | 20 | 577 |
| HSV1-UL19-192 | - | GUCCUCGGGGCGUUUGAGCG | 20 | 578 |
| HSV1-UL19-193 | - | CGGGGCGUUUGAGCGCGGCA | 20 | 579 |
| HSV1-UL19-194 | - | CCAGAUGCUGCACGUGCUGC | 20 | 580 |
| HSV1-UL19-195 | - | GCUGCACGUGCUGCUGGAAA | 20 | 581 |
| HSV1-UL19-196 | - | GCUGGAAAAGGCGCCGCCCC | 20 | 582 |
| HSV1-UL19-197 | - | GUUGCCGAUGCAACGAUACC | 20 | 583 |
| HSV1-UL19-198 | - | AUGCAACGAUACCUGGACAA | 20 | 584 |
| HSV1-UL19-199 | - | AUACCUGGACAACGGCCGCC | 20 | 585 |
| HSV1-UL19-200 | - | ACAACGGCCGCCUGGCCACC | 20 | 586 |
| HSV1-UL19-201 | - | CAACGGCCGCCUGGCCACCA | 20 | 587 |
| HSV1-UL19-202 | - | CGGCCGCCUGGCCACCAGGG | 20 | 588 |
| HSV1-UL19-203 | - | GCCUGGCCACCAGGGUGGCC | 20 | 589 |
| HSV1-UL19-204 | - | CCUGGCCACCAGGGUGGCCC | 20 | 590 |
| HSV1-UL19-205 | - | CAGGGUGGCCCGGGCGACCC | 20 | 591 |
| HSV1-UL19-206 | - | CUGCGAGACGAGCUUUUUCC | 20 | 592 |
| HSV1-UL19-207 | - | UGCGAGACGAGCUUUUUCCU | 20 | 593 |
| HSV1-UL19-208 | - | GACGAGCUUUUUCCUGGGCA | 20 | 594 |
| HSV1-UL19-209 | - | GAGCUUUUUCCUGGGCAAGG | 20 | 595 |
| HSV1-UL19-210 | - | AGCUUUUUCCUGGGCAAGGC | 20 | 596 |
| HSV1-UL19-211 | - | UGGGCAAGGCGGGCCACCGC | 20 | 597 |
| HSV1-UL19-212 | - | GGGCAAGGCGGGCCACCGCC | 20 | 598 |
| HSV1-UL19-213 | - | CAAGGCGGGCCACCGCCGGG | 20 | 599 |
| HSV1-UL19-214 | - | CCACCGCCGGGAGGCCGUCG | 20 | 600 |
| HSV1-UL19-215 | - | GCCGGGAGGCCGUCGAGGCC | 20 | 601 |
| HSV1-UL19-216 | - | GGCCGUCGAGGCCUGGCUCG | 20 | 602 |
| HSV1-UL19-217 | - | CUGGCUCGUGGACCUCACCA | 20 | 603 |
| HSV1-UL19-218 | - | CACGGCCACGCAGCCCUCCG | 20 | 604 |
| HSV1-UL19-219 | - | CUGACGCAUGCCGACACGCG | 20 | 605 |
| HSV1-UL19-220 | - | UGACGCAUGCCGACACGCGC | 20 | 606 |
| HSV1-UL19-221 | - | CGCAUGCCGACACGCGCGGG | 20 | 607 |
| HSV1-UL19-222 | - | UGCCGACACGCGCGGGCGGC | 20 | 608 |
| HSV1-UL19-223 | - | ACGCGCGGGCGGCCGGUCGA | 20 | 609 |
| HSV1-UL19-224 | - | CGCGCGGGCGGCCGGUCGAC | 20 | 610 |
| HSV1-UL19-225 | - | GCGCGGGCGGCCGGUCGACG | 20 | 611 |
| HSV1-UL19-226 | - | CCUGCAGUCCUUCCUGAAAG | 20 | 612 |
| HSV1-UL19-227 | - | GCAGUCCUUCCUGAAAGUGG | 20 | 613 |
| HSV1-UL19-228 | - | GGACACCGAAGCCGACGUGC | 20 | 614 |
| HSV1-UL19-229 | - | GCCGACGUGCCGGUGACGUA | 20 | 615 |
| HSV1-UL19-230 | - | GCCGGUGACGUACGGCGAGA | 20 | 616 |
| HSV1-UL19-231 | - | UACGGCGAGAUGGUCCUGAA | 20 | 617 |
| HSV1-UL19-232 | - | ACGGCGAGAUGGUCCUGAAC | 20 | 618 |
| HSV1-UL19-233 | - | CGGCGAGAUGGUCCUGAACG | 20 | 619 |
| HSV1-UL19-234 | - | GGUCCUGAACGGGGCCAACC | 20 | 620 |
| HSV1-UL19-235 | - | GAACGGGGCCAACCUGGUCA | 20 | 621 |
| HSV1-UL19-236 | - | CCUGGUCACGGCGCUCGUGA | 20 | 622 |
| HSV1-UL19-237 | - | CUGGUCACGGCGCUCGUGAU | 20 | 623 |
| HSV1-UL19-238 | - | CACGGCGCUCGUGAUGGGCA | 20 | 624 |
| HSV1-UL19-239 | - | GGGCAAGGCCGUGCGAAGUC | 20 | 625 |
| HSV1-UL19-240 | - | CGUGCGAAGUCUGGACGACG | 20 | 626 |
| HSV1-UL19-241 | - | GUGCGAAGUCUGGACGACGU | 20 | 627 |
| HSV1-UL19-242 | - | CGACGUGGGCCGCCACCUGC | 20 | 628 |
| HSV1-UL19-243 | - | CCGCCACCUGCUGGAGAUGC | 20 | 629 |
| HSV1-UL19-244 | - | CCACCUGCUGGAGAUGCAGG | 20 | 630 |
| HSV1-UL19-245 | - | AGGAGCAGCUCGACCUGAAC | 20 | 631 |
| HSV1-UL19-246 | - | CGACCUGAACCGGCAGACGC | 20 | 632 |
| HSV1-UL19-247 | - | CCAGACGACGCGCGUGCGCG | 20 | 633 |
| HSV1-UL19-248 | - | GACGCGCGUGCGCGCGGAUC | 20 | 634 |
| HSV1-UL19-249 | - | CGCGCGGAUCUGGUGUCCAU | 20 | 635 |
| HSV1-UL19-250 | - | GGUGUCCAUCGGCGAGAAGC | 20 | 636 |
| HSV1-UL19-251 | - | CGGCGAGAAGCUGGUCUUUC | 20 | 637 |
| HSV1-UL19-252 | - | CGAGAAGCUGGUCUUUCUGG | 20 | 638 |
| HSV1-UL19-253 | - | GCUGGUCUUUCUGGAGGCCC | 20 | 639 |
| HSV1-UL19-254 | - | CACCAACGUUCCCUACCCCC | 20 | 640 |
| HSV1-UL19-255 | - | CAACGUUCCCUACCCCCUGG | 20 | 641 |
| HSV1-UL19-256 | - | AACGUUCCCUACCCCCUGGU | 20 | 642 |
| HSV1-UL19-257 | - | CUACCCCCUGGUGGGCGCCA | 20 | 643 |
| HSV1-UL19-258 | - | CCUGACGUUCGUCCUGCCCC | 20 | 644 |
| HSV1-UL19-259 | - | CUGACGUUCGUCCUGCCCCU | 20 | 645 |
| HSV1-UL19-260 | - | GCCCCUGGGCCUGUUCAAUC | 20 | 646 |
| HSV1-UL19-261 | - | GGGCCUGUUCAAUCCGGUCA | 20 | 647 |
| HSV1-UL19-262 | - | UGUUCAAUCCGGUCAUGGAA | 20 | 648 |
| HSV1-UL19-263 | - | GAACGGUUUGCCGCGCACGC | 20 | 649 |
| HSV1-UL19-264 | - | AACGGUUUGCCGCGCACGCC | 20 | 650 |
| HSV1-UL19-265 | - | ACGGUUUGCCGCGCACGCCG | 20 | 651 |
| HSV1-UL19-266 | - | GGGGACCUAGUCCCCGCCCC | 20 | 652 |
| HSV1-UL19-267 | - | AGUCCCCGCCCCCGGCCACC | 20 | 653 |
| HSV1-UL19-268 | - | CGCCCCGCCAGCUGUUUUUU | 20 | 654 |
| HSV1-UL19-269 | - | GCCCCGCCAGCUGUUUUUUU | 20 | 655 |
| HSV1-UL19-270 | - | CCCCGCCAGCUGUUUUUUUG | 20 | 656 |
| HSV1-UL19-271 | - | CCCGCCAGCUGUUUUUUUGG | 20 | 657 |
| HSV1-UL19-272 | - | CCAGCUGUUUUUUUGGGGGA | 20 | 658 |
| HSV1-UL19-273 | - | UUUUUGGGGGAAGGACCGCC | 20 | 659 |
| HSV1-UL19-274 | - | CCAGGUGCUGCGCCUGUCUC | 20 | 660 |
| HSV1-UL19-275 | - | CUGUCUCUGGAACACGCGAU | 20 | 661 |
| HSV1-UL19-276 | - | UGUCUCUGGAACACGCGAUC | 20 | 662 |
| HSV1-UL19-277 | - | UUCGCUGAUGAACGUUGACG | 20 | 663 |
| HSV1-UL19-278 | - | GCUGAUGAACGUUGACGCGG | 20 | 664 |
| HSV1-UL19-279 | - | AUGAACGUUGACGCGGCGGU | 20 | 665 |
| HSV1-UL19-280 | - | UGAACGUUGACGCGGCGGUC | 20 | 666 |
| HSV1-UL19-281 | - | GAACGUUGACGCGGCGGUCG | 20 | 667 |
| HSV1-UL19-282 | - | AACGUUGACGCGGCGGUCGG | 20 | 668 |
| HSV1-UL19-283 | - | GUCGAAGCCGCCAAUCCGUA | 20 | 669 |
| HSV1-UL19-284 | - | UCGAAGCCGCCAAUCCGUAC | 20 | 670 |
| HSV1-UL19-285 | - | CGAAGCCGCCAAUCCGUACG | 20 | 671 |
| HSV1-UL19-286 | - | CAAUCCGUACGGGGCGUACG | 20 | 672 |
| HSV1-UL19-287 | - | UCCGUACGGGGCGUACGUGG | 20 | 673 |
| HSV1-UL19-288 | - | CGGGGCGUACGUGGCGGCCC | 20 | 674 |
| HSV1-UL19-289 | - | GCGUACGUGGCGGCCCCGGC | 20 | 675 |
| HSV1-UL19-290 | - | AGCAGCUGUUUUUGAACGCC | 20 | 676 |
| HSV1-UL19-291 | - | GCAGCUGUUUUUGAACGCCU | 20 | 677 |
| HSV1-UL19-292 | - | CAGCUGUUUUUGAACGCCUG | 20 | 678 |
| HSV1-UL19-293 | - | AGCUGUUUUUGAACGCCUGG | 20 | 679 |
| HSV1-UL19-294 | - | GAACGCCUGGGGGCAGCGCC | 20 | 680 |
| HSV1-UL19-295 | - | UGGGGGCAGCGCCUGGCCCA | 20 | 681 |
| HSV1-UL19-296 | - | GGGGGCAGCGCCUGGCCCAC | 20 | 682 |
| HSV1-UL19-297 | - | GGCAGCGCCUGGCCCACGGG | 20 | 683 |
| HSV1-UL19-298 | - | GCAGCGCCUGGCCCACGGGC | 20 | 684 |
| HSV1-UL19-299 | - | UGGCCCACGGGCGGGUCCGA | 20 | 685 |
| HSV1-UL19-300 | - | GGCCCACGGGCGGGUCCGAU | 20 | 686 |
| HSV1-UL19-301 | - | CGGGCGGGUCCGAUGGGUCG | 20 | 687 |
| HSV1-UL19-302 | - | CGGGUCCGAUGGGUCGCGGA | 20 | 688 |
| HSV1-UL19-303 | - | CGCGGAAGGCCAGAUGACCC | 20 | 689 |
| HSV1-UL19-304 | - | GCAGCCCGACAACGCCAACC | 20 | 690 |
| HSV1-UL19-305 | - | CCCCGCGUUCGACUUCUUUG | 20 | 691 |
| HSV1-UL19-306 | - | CCCGCGUUCGACUUCUUUGU | 20 | 692 |
| HSV1-UL19-307 | - | CCGCGUUCGACUUCUUUGUG | 20 | 693 |
| HSV1-UL19-308 | - | CGCGUUCGACUUCUUUGUGG | 20 | 694 |
| HSV1-UL19-309 | - | GUUCGACUUCUUUGUGGGGG | 20 | 695 |
| HSV1-UL19-310 | - | CUUUGUGGGGGUGGCCGACG | 20 | 696 |
| HSV1-UL19-311 | - | GGUGGCCGACGUGGAGCUGC | 20 | 697 |
| HSV1-UL19-312 | - | GUGGCCGACGUGGAGCUGCC | 20 | 698 |
| HSV1-UL19-313 | - | UGGCCGACGUGGAGCUGCCG | 20 | 699 |
| HSV1-UL19-314 | - | GGCCGACGUGGAGCUGCCGG | 20 | 700 |
| HSV1-UL19-315 | - | GCCGACGUGGAGCUGCCGGG | 20 | 701 |
| HSV1-UL19-316 | - | CCGACGUGGAGCUGCCGGGG | 20 | 702 |
| HSV1-UL19-317 | - | CGACGUGGAGCUGCCGGGGG | 20 | 703 |
| HSV1-UL19-318 | - | GCCGGGGGGGGACGUUCCCC | 20 | 704 |
| HSV1-UL19-319 | - | GGGGGGGACGUUCCCCCGGC | 20 | 705 |
| HSV1-UL19-320 | - | GGACGUUCCCCCGGCCGGCC | 20 | 706 |
| HSV1-UL19-321 | - | GACGUUCCCCCGGCCGGCCC | 20 | 707 |
| HSV1-UL19-322 | - | ACGUUCCCCCGGCCGGCCCG | 20 | 708 |
| HSV1-UL19-323 | - | CGUUCCCCCGGCCGGCCCGG | 20 | 709 |
| HSV1-UL19-324 | - | GGCCGGCCCGGGGGAGAUCC | 20 | 710 |
| HSV1-UL19-325 | - | CGGGGGAGAUCCAGGCCACC | 20 | 711 |
| HSV1-UL19-326 | - | GAUCCAGGCCACCUGGCGCG | 20 | 712 |
| HSV1-UL19-327 | - | GCCACCUGGCGCGUGGUGAA | 20 | 713 |
| HSV1-UL19-328 | - | GGUGAACGGCAACUUGCCCC | 20 | 714 |
| HSV1-UL19-329 | - | CUUGCCCCUGGCGCUAUGUC | 20 | 715 |
| HSV1-UL19-330 | - | GCCCCUGGCGCUAUGUCCGG | 20 | 716 |
| HSV1-UL19-331 | - | CGCUAUGUCCGGCGGCGUUC | 20 | 717 |
| HSV1-UL19-332 | - | GCUAUGUCCGGCGGCGUUCC | 20 | 718 |
| HSV1-UL19-333 | - | CGGCGGCGUUCCGGGACGCC | 20 | 719 |
| HSV1-UL19-334 | - | GGCGGCGUUCCGGGACGCCC | 20 | 720 |
| HSV1-UL19-335 | - | GCGGCGUUCCGGGACGCCCG | 20 | 721 |
| HSV1-UL19-336 | - | GUUCCGGGACGCCCGGGGCC | 20 | 722 |
| HSV1-UL19-337 | - | GGACGCCCGGGGCCUGGAGC | 20 | 723 |
| HSV1-UL19-338 | - | GACGCCCGGGGCCUGGAGCU | 20 | 724 |
| HSV1-UL19-339 | - | ACGCCCGGGGCCUGGAGCUG | 20 | 725 |
| HSV1-UL19-340 | - | CGCCCGGGGCCUGGAGCUGG | 20 | 726 |
| HSV1-UL19-341 | - | CCGGGGCCUGGAGCUGGGGG | 20 | 727 |
| HSV1-UL19-342 | - | CGGGGCCUGGAGCUGGGGGU | 20 | 728 |
| HSV1-UL19-343 | - | GGGGGUGGGACGCCACGCCA | 20 | 729 |
| HSV1-UL19-344 | - | GCCACCAUCGCCGCCGUUCG | 20 | 730 |
| HSV1-UL19-345 | - | CCACCAUCGCCGCCGUUCGC | 20 | 731 |
| HSV1-UL19-346 | - | CACCAUCGCCGCCGUUCGCG | 20 | 732 |
| HSV1-UL19-347 | - | GUUCGACGACCGCAACUACC | 20 | 733 |
| HSV1-UL19-348 | - | CGACGACCGCAACUACCCGG | 20 | 734 |
| HSV1-UL19-349 | - | GGCGGUGUUUUACCUGCUGC | 20 | 735 |
| HSV1-UL19-350 | - | CUGCUGCAGGCCGCCAUACA | 20 | 736 |
| HSV1-UL19-351 | - | CGAGCACGUCUUCUGCGCCC | 20 | 737 |
| HSV1-UL19-352 | - | ACGUCUUCUGCGCCCUGGCC | 20 | 738 |
| HSV1-UL19-353 | - | CUGCGCCCUGGCCCGGCUCG | 20 | 739 |
| HSV1-UL19-354 | - | UCCAGUGCAUCACCAGCUAC | 20 | 740 |
| HSV1-UL19-355 | - | CCAGCUACUGGAACAACACG | 20 | 741 |
| HSV1-UL19-356 | - | CUGGAACAACACGCGGUGCG | 20 | 742 |
| HSV1-UL19-357 | - | GUUCGUGAACGACUACUCGC | 20 | 743 |
| HSV1-UL19-358 | - | UCGUACGUCGUGACCUACCU | 20 | 744 |
| HSV1-UL19-359 | - | CGUACGUCGUGACCUACCUC | 20 | 745 |
| HSV1-UL19-360 | - | GUACGUCGUGACCUACCUCG | 20 | 746 |
| HSV1-UL19-361 | - | UACGUCGUGACCUACCUCGG | 20 | 747 |
| HSV1-UL19-362 | - | CCUCGGGGGAGACCUUCCCG | 20 | 748 |
| HSV1-UL19-363 | - | AGACCUUCCCGAGGAGUGCA | 20 | 749 |
| HSV1-UL19-364 | - | AGGAGUGCAUGGCCGUGUAC | 20 | 750 |
| HSV1-UL19-365 | - | GGAGUGCAUGGCCGUGUACC | 20 | 751 |
| HSV1-UL19-366 | - | CAUGGCCGUGUACCGGGACC | 20 | 752 |
| HSV1-UL19-367 | - | GGCCGUGUACCGGGACCUGG | 20 | 753 |
| HSV1-UL19-368 | - | GGACCUGGUGGCGCACGUCG | 20 | 754 |
| HSV1-UL19-369 | - | GGUGGCGCACGUCGAGGCCC | 20 | 755 |
| HSV1-UL19-370 | - | CGUCGAGGCCCUGGCCCAGC | 20 | 756 |
| HSV1-UL19-371 | - | GUUGAUGACUUUACCCUGAC | 20 | 757 |
| HSV1-UL19-372 | - | UGACUUUACCCUGACCGGCC | 20 | 758 |
| HSV1-UL19-373 | - | UACCCUGACCGGCCCGGAGC | 20 | 759 |
| HSV1-UL19-374 | - | ACCCUGACCGGCCCGGAGCU | 20 | 760 |
| HSV1-UL19-375 | - | CUGACCGGCCCGGAGCUGGG | 20 | 761 |
| HSV1-UL19-376 | - | UGACCGGCCCGGAGCUGGGC | 20 | 762 |
| HSV1-UL19-377 | - | CGGCCCGGAGCUGGGCGGGC | 20 | 763 |
| HSV1-UL19-378 | - | GAAUCACCUAAUGCGAGACC | 20 | 764 |
| HSV1-UL19-379 | - | CGCUGCUGCCACCCCUCGUG | 20 | 765 |
| HSV1-UL19-380 | - | GCUGCUGCCACCCCUCGUGU | 20 | 766 |
| HSV1-UL19-381 | - | GGGACUGUGACGCCCUGAUG | 20 | 767 |
| HSV1-UL19-382 | - | UGACGCCCUGAUGCGGCGCG | 20 | 768 |
| HSV1-UL19-383 | - | CCUGAUGCGGCGCGCGGCCC | 20 | 769 |
| HSV1-UL19-384 | - | UGGACCGCCAUCGCGACUGC | 20 | 770 |
| HSV1-UL19-385 | - | GGACCGCCAUCGCGACUGCC | 20 | 771 |
| HSV1-UL19-386 | - | UCGCGACUGCCGGGUUAGCG | 20 | 772 |
| HSV1-UL19-387 | - | CGCGACUGCCGGGUUAGCGC | 20 | 773 |
| HSV1-UL19-388 | - | GCGACUGCCGGGUUAGCGCG | 20 | 774 |
| HSV1-UL19-389 | - | CGACUGCCGGGUUAGCGCGG | 20 | 775 |
| HSV1-UL19-390 | - | GACUGCCGGGUUAGCGCGGG | 20 | 776 |
| HSV1-UL19-391 | - | GGGCCACGACCCCGUGUACG | 20 | 777 |
| HSV1-UL19-392 | - | CCACGACCCCGUGUACGCGG | 20 | 778 |
| HSV1-UL19-393 | - | GUACGCGGCGGCAUGUAACG | 20 | 779 |
| HSV1-UL19-394 | - | GGCAUGUAACGUGGCGACCG | 20 | 780 |
| HSV1-UL19-395 | - | GCGGACUUCAACCGCAACGA | 20 | 781 |
| HSV1-UL19-396 | - | CCAGCUGCUGCACAACACCC | 20 | 782 |
| HSV1-UL19-397 | - | CAACACCCAGGCCCGAGCCG | 20 | 783 |
| HSV1-UL19-398 | - | GGCCCGAGCCGCGGACGCCG | 20 | 784 |
| HSV1-UL19-399 | - | CCGCGGACGCCGCGGAUGAC | 20 | 785 |
| HSV1-UL19-400 | - | CCGCGGAUGACCGGCCGCAC | 20 | 786 |
| HSV1-UL19-401 | - | CGCGGAUGACCGGCCGCACC | 20 | 787 |
| HSV1-UL19-402 | - | GCGGAUGACCGGCCGCACCG | 20 | 788 |
| HSV1-UL19-403 | - | CGGAUGACCGGCCGCACCGG | 20 | 789 |
| HSV1-UL19-404 | - | GGAUGACCGGCCGCACCGGG | 20 | 790 |
| HSV1-UL19-405 | - | UGACCGGCCGCACCGGGGGG | 20 | 791 |
| HSV1-UL19-406 | - | GGCCGCACCGGGGGGCGGAC | 20 | 792 |
| HSV1-UL19-407 | - | CAAGAUUUACUACUACGUGA | 20 | 793 |
| HSV1-UL19-408 | - | UGAUGGUGCCCGCCUUCUCG | 20 | 794 |
| HSV1-UL19-409 | - | GAUGGUGCCCGCCUUCUCGC | 20 | 795 |
| HSV1-UL19-410 | - | AUGGUGCCCGCCUUCUCGCG | 20 | 796 |
| HSV1-UL19-411 | - | GCGGGGCCGCUGCUGCACCG | 20 | 797 |
| HSV1-UL19-412 | - | CGGGGCCGCUGCUGCACCGC | 20 | 798 |
| HSV1-UL19-413 | - | GGGGCCGCUGCUGCACCGCG | 20 | 799 |
| HSV1-UL19-414 | - | GGGCCGCUGCUGCACCGCGG | 20 | 800 |
| HSV1-UL19-415 | - | AUACGCCACCCUGCAGAACA | 20 | 801 |
| HSV1-UL19-416 | - | CGCCACCCUGCAGAACAUGG | 20 | 802 |
| HSV1-UL19-417 | - | CCUGCAGAACAUGGUGGUCC | 20 | 803 |
| HSV1-UL19-418 | - | GUGGUCCCGGAGAUCGCCCC | 20 | 804 |
| HSV1-UL19-419 | - | CCCGGAGAUCGCCCCCGGCG | 20 | 805 |
| HSV1-UL19-420 | - | GUGCCCCAGCGACCCCGUGA | 20 | 806 |
| HSV1-UL19-421 | - | CCCCGCGCACCCCCUGCACC | 20 | 807 |
| HSV1-UL19-422 | - | CCCCCUGCACCCGGCCAAUC | 20 | 808 |
| HSV1-UL19-423 | - | CCUGCACCCGGCCAAUCUGG | 20 | 809 |
| HSV1-UL19-424 | - | GGCCAAUCUGGUGGCCAACA | 20 | 810 |
| HSV1-UL19-425 | - | GUCAACGCCAUGUUUCACAA | 20 | 811 |
| HSV1-UL19-426 | - | UCAACGCCAUGUUUCACAAC | 20 | 812 |
| HSV1-UL19-427 | - | CAUGUUUCACAACGGGCGCG | 20 | 813 |
| HSV1-UL19-428 | - | UCACAACGGGCGCGUGGUAG | 20 | 814 |
| HSV1-UL19-429 | - | AACGGGCGCGUGGUAGUGGA | 20 | 815 |
| HSV1-UL19-430 | - | ACGGGCGCGUGGUAGUGGAC | 20 | 816 |
| HSV1-UL19-431 | - | GCCCGCCAUGCUCACGCUGC | 20 | 817 |
| HSV1-UL19-432 | - | CAUGCUCACGCUGCAGGUGC | 20 | 818 |
| HSV1-UL19-433 | - | GCAGGUGCUGGCCCACAACA | 20 | 819 |
| HSV1-UL19-434 | - | CAACAUGGCCGAACGCACAA | 20 | 820 |
| HSV1-UL19-435 | - | CACAACGGCGCUGCUCUGCU | 20 | 821 |
| HSV1-UL19-436 | - | AACGGCGCUGCUCUGCUCGG | 20 | 822 |
| HSV1-UL19-437 | - | CUGCUCGGCGGCGCCCGACG | 20 | 823 |
| HSV1-UL19-438 | - | UGCUCGGCGGCGCCCGACGC | 20 | 824 |
| HSV1-UL19-439 | - | ACCAACAUGCGCAUAUUCGA | 20 | 825 |
| HSV1-UL19-440 | - | CCAACAUGCGCAUAUUCGAC | 20 | 826 |
| HSV1-UL19-441 | - | CAACAUGCGCAUAUUCGACG | 20 | 827 |
| HSV1-UL19-442 | - | UUCGACGGGGCGUUGCACGC | 20 | 828 |
| HSV1-UL19-443 | - | GCACGCCGGAAUCCUGCUGA | 20 | 829 |
| HSV1-UL19-444 | - | GCUGAUGGCCCCCCAGCAUC | 20 | 830 |
| HSV1-UL19-445 | - | CUGGACCAUACCAUCCAAAA | 20 | 831 |
| HSV1-UL19-446 | - | CCCGUCCACGCGCUGUUCGC | 20 | 832 |
| HSV1-UL19-447 | - | CCGUCCACGCGCUGUUCGCC | 20 | 833 |
| HSV1-UL19-448 | - | CGUCCACGCGCUGUUCGCCG | 20 | 834 |
| HSV1-UL19-449 | - | GUUCGCCGGGGCCGACCACG | 20 | 835 |
| HSV1-UL19-450 | - | GAACGCGCCCAAUUUCCCCC | 20 | 836 |
| HSV1-UL19-451 | - | CGGCCCUGCGCGACCUGUCG | 20 | 837 |
| HSV1-UL19-452 | - | CCUGCGCGACCUGUCGCGGC | 20 | 838 |
| HSV1-UL19-453 | - | CCUGUCGCGGCAGGUCCCCC | 20 | 839 |
| HSV1-UL19-454 | - | GCAGGUCCCCCUGGUCCCCC | 20 | 840 |
| HSV1-UL19-455 | - | CCCCCUGGUCCCCCCGGCUC | 20 | 841 |
| HSV1-UL19-456 | - | CCCCUGGUCCCCCCGGCUCU | 20 | 842 |
| HSV1-UL19-457 | - | CCCUGGUCCCCCCGGCUCUG | 20 | 843 |
| HSV1-UL19-458 | - | CCUGGUCCCCCCGGCUCUGG | 20 | 844 |
| HSV1-UL19-459 | - | GCAGCACGUCCGCGAGAGCG | 20 | 845 |
| HSV1-UL19-460 | - | CACGUCCGCGAGAGCGCGGC | 20 | 846 |
| HSV1-UL19-461 | - | ACGUCCGCGAGAGCGCGGCC | 20 | 847 |
| HSV1-UL19-462 | - | CGUCCGCGAGAGCGCGGCCG | 20 | 848 |
| HSV1-UL19-463 | - | CGCGCUGACCUACGCGCUCA | 20 | 849 |
| HSV1-UL19-464 | - | GCUGACCUACGCGCUCAUGG | 20 | 850 |
| HSV1-UL19-465 | - | CUGACCUACGCGCUCAUGGC | 20 | 851 |
| HSV1-UL19-466 | - | UGACCUACGCGCUCAUGGCG | 20 | 852 |
| HSV1-UL19-467 | - | GUACUUCAAGAUCAGUCCCG | 20 | 853 |
| HSV1-UL19-468 | - | CUUGCAUCAUCAGCUCAAGA | 20 | 854 |
| HSV1-UL19-469 | - | UUGCAUCAUCAGCUCAAGAC | 20 | 855 |
| HSV1-UL19-470 | - | CUCAAGACGGGCCUCCAUCC | 20 | 856 |
| HSV1-UL19-471 | - | UCAAGACGGGCCUCCAUCCC | 20 | 857 |
| HSV1-UL19-472 | - | ACGGGCCUCCAUCCCGGGUU | 20 | 858 |
| HSV1-UL19-473 | - | CGGGCCUCCAUCCCGGGUUU | 20 | 859 |
| HSV1-UL19-474 | - | UGGGUUCACCGUCGUCCGAC | 20 | 860 |
| HSV1-UL19-475 | - | GACUGAGAACGUGCUGUUCU | 20 | 861 |
| HSV1-UL19-476 | - | GCUGUUCUCGGAGCGCGCGU | 20 | 862 |
| HSV1-UL19-477 | - | GUUCUCGGAGCGCGCGUCGG | 20 | 863 |
| HSV1-UL19-478 | - | CGCGUCGGAGGCGUACUUCC | 20 | 864 |
| HSV1-UL19-479 | - | GCGUCGGAGGCGUACUUCCU | 20 | 865 |
| HSV1-UL19-480 | - | GUACUUCCUGGGCCAGCUCC | 20 | 866 |
| HSV1-UL19-481 | - | CUUCCUGGGCCAGCUCCAGG | 20 | 867 |
| HSV1-UL19-482 | - | UGGGCCAGCUCCAGGUGGCC | 20 | 868 |
| HSV1-UL19-483 | - | CAGGUGGCCCGGCACGAAAC | 20 | 869 |
| HSV1-UL19-484 | - | GUGGCCCGGCACGAAACUGG | 20 | 870 |
| HSV1-UL19-485 | - | UGGCCCGGCACGAAACUGGC | 20 | 871 |
| HSV1-UL19-486 | - | GGCCCGGCACGAAACUGGCG | 20 | 872 |
| HSV1-UL19-487 | - | GCCCGGCACGAAACUGGCGG | 20 | 873 |
| HSV1-UL19-488 | - | CCCGGCACGAAACUGGCGGG | 20 | 874 |
| HSV1-UL19-489 | - | CCGGCACGAAACUGGCGGGG | 20 | 875 |
| HSV1-UL19-490 | - | CACCCAGCCGCGCGCGAACG | 20 | 876 |
| HSV1-UL19-491 | - | GCCGCGCGCGAACGUGGACC | 20 | 877 |
| HSV1-UL19-492 | - | CCGCGCGCGAACGUGGACCU | 20 | 878 |
| HSV1-UL19-493 | - | CGCGAACGUGGACCUGGGCG | 20 | 879 |
| HSV1-UL19-494 | - | GCGAACGUGGACCUGGGCGU | 20 | 880 |
| HSV1-UL19-495 | - | CGUGGGCUACACCGCCGUCG | 20 | 881 |
| HSV1-UL19-496 | - | CUACACCGCCGUCGUGGCCA | 20 | 882 |
| HSV1-UL19-497 | - | CCGCAACCCCGUCACCGACA | 20 | 883 |
| HSV1-UL19-498 | - | CGCAACCCCGUCACCGACAU | 20 | 884 |
| HSV1-UL19-499 | - | CCUUCCCCAAAACUUUUACC | 20 | 885 |
| HSV1-UL19-500 | - | CUUCCCCAAAACUUUUACCU | 20 | 886 |
| HSV1-UL19-501 | - | CAAAACUUUUACCUGGGCCG | 20 | 887 |
| HSV1-UL19-502 | - | AAAACUUUUACCUGGGCCGC | 20 | 888 |
| HSV1-UL19-503 | - | AAACUUUUACCUGGGCCGCG | 20 | 889 |
| HSV1-UL19-504 | - | CCGCGGGGCUCCCCCUCUCC | 20 | 890 |
| HSV1-UL19-505 | - | UCCCCCUCUCCUGGACAACG | 20 | 891 |
| HSV1-UL19-506 | - | ACGCGGCAGCCGUGUACCUG | 20 | 892 |
| HSV1-UL19-507 | - | AGCCGUGUACCUGCGGAACG | 20 | 893 |
| HSV1-UL19-508 | - | GUACCUGCGGAACGCGGUCG | 20 | 894 |
| HSV1-UL19-509 | - | CCUGCGGAACGCGGUCGUGG | 20 | 895 |
| HSV1-UL19-510 | - | CUGCGGAACGCGGUCGUGGC | 20 | 896 |
| HSV1-UL19-511 | - | GGUCGUGGCGGGAAACCGCC | 20 | 897 |
| HSV1-UL19-512 | - | GUCGUGGCGGGAAACCGCCU | 20 | 898 |
| HSV1-UL19-513 | - | UCGUGGCGGGAAACCGCCUG | 20 | 899 |
| HSV1-UL19-514 | - | GGCGGGAAACCGCCUGGGGC | 20 | 900 |
| HSV1-UL19-515 | - | GCCCAGCCCGUCCCCGUGUU | 20 | 901 |
| HSV1-UL19-516 | - | CCCAGCCCGUCCCCGUGUUC | 20 | 902 |
| HSV1-UL19-517 | - | CCCCGUGUUCGGGUGCGCCC | 20 | 903 |
| HSV1-UL19-518 | - | UCGGGUGCGCCCAGGUGCCG | 20 | 904 |
| HSV1-UL19-519 | - | GCCCAGGUGCCGCGGCGCGC | 20 | 905 |
| HSV1-UL19-520 | - | CCCAGGUGCCGCGGCGCGCA | 20 | 906 |
| HSV1-UL19-521 | - | GGUGCCGCGGCGCGCAGGGA | 20 | 907 |
| HSV1-UL19-522 | - | CGGCGCGCAGGGAUGGACCA | 20 | 908 |
| HSV1-UL19-523 | - | CGCAGGGAUGGACCACGGCC | 20 | 909 |
| HSV1-UL19-524 | - | CCGACGUCAACUACUUCCGC | 20 | 910 |
| HSV1-UL19-525 | - | UCCGCCGGCCCUGCAACCCC | 20 | 911 |
| HSV1-UL19-526 | - | CCGCCGGCCCUGCAACCCCC | 20 | 912 |
| HSV1-UL19-527 | - | CGCCGGCCCUGCAACCCCCG | 20 | 913 |
| HSV1-UL19-528 | - | AACCCCCGGGGACGCGCCGC | 20 | 914 |
| HSV1-UL19-529 | - | ACCCCCGGGGACGCGCCGCC | 20 | 915 |
| HSV1-UL19-530 | - | CCCCCGGGGACGCGCCGCCG | 20 | 916 |
| HSV1-UL19-531 | - | CCCCGGGGACGCGCCGCCGG | 20 | 917 |
| HSV1-UL19-532 | - | CGCCGCCGGGGGCGUUUACG | 20 | 918 |
| HSV1-UL19-533 | - | GCCGCCGGGGGCGUUUACGC | 20 | 919 |
| HSV1-UL19-534 | - | CCGCCGGGGGCGUUUACGCG | 20 | 920 |
| HSV1-UL19-535 | - | CGCCGGGGGCGUUUACGCGG | 20 | 921 |
| HSV1-UL19-536 | - | GGGCGUUUACGCGGGGGACA | 20 | 922 |
| HSV1-UL19-537 | - | CGUUUACGCGGGGGACAAGG | 20 | 923 |
| HSV1-UL19-538 | - | GUUUACGCGGGGGACAAGGA | 20 | 924 |
| HSV1-UL19-539 | - | UUUACGCGGGGGACAAGGAG | 20 | 925 |
| HSV1-UL19-540 | - | UUACGCGGGGGACAAGGAGG | 20 | 926 |
| HSV1-UL19-541 | - | ACCGCCCUCAUGUACGACCA | 20 | 927 |
| HSV1-UL19-542 | - | ACGGCCAGAGCGACCCGUCC | 20 | 928 |
| HSV1-UL19-543 | - | CGGCCAGAGCGACCCGUCCC | 20 | 929 |
| HSV1-UL19-544 | - | CGACCCGUCCCGGGCCUUCG | 20 | 930 |
| HSV1-UL19-545 | - | GUCCCGGGCCUUCGCGGCCA | 20 | 931 |
| HSV1-UL19-546 | - | UCGCGGCCACGGCCAACCCG | 20 | 932 |
| HSV1-UL19-547 | - | CGCGGCCACGGCCAACCCGU | 20 | 933 |
| HSV1-UL19-548 | - | GCGUCGCAGCGAUUUUCGUA | 20 | 934 |
| HSV1-UL19-549 | - | CGUCGCAGCGAUUUUCGUAC | 20 | 935 |
| HSV1-UL19-550 | - | GUCGCAGCGAUUUUCGUACG | 20 | 936 |
| HSV1-UL19-551 | - | UACGGGGACCUGCUCUAUAA | 20 | 937 |
| HSV1-UL19-552 | - | ACGGGGACCUGCUCUAUAAC | 20 | 938 |
| HSV1-UL19-553 | - | CGGGGACCUGCUCUAUAACG | 20 | 939 |
| HSV1-UL19-554 | - | AACGGGGCCUACCACCUCAA | 20 | 940 |
| HSV1-UL19-555 | - | ACGGGGCCUACCACCUCAAC | 20 | 941 |
| HSV1-UL19-556 | - | CGGGGCCUACCACCUCAACG | 20 | 942 |
| HSV1-UL19-557 | - | CCACCUCAACGGGGCCUCGC | 20 | 943 |
| HSV1-UL19-558 | - | CUGCUUUAAGUUCUUUACGU | 20 | 944 |
| HSV1-UL19-559 | - | CGCCGCCAAACAUCGCUGCC | 20 | 945 |
| HSV1-UL19-560 | - | CUGCCUGGAGCGCCUGAUCG | 20 | 946 |
| HSV1-UL19-561 | - | GGAGCGCCUGAUCGUGGAGA | 20 | 947 |
| HSV1-UL19-562 | - | GAGCGCCUGAUCGUGGAGAC | 20 | 948 |
| HSV1-UL19-563 | - | CCUGAUCGUGGAGACGGGUU | 20 | 949 |
| HSV1-UL19-564 | - | GAUCGUGGAGACGGGUUCGG | 20 | 950 |
| HSV1-UL19-565 | - | GACGGGUUCGGCGGUGUCCA | 20 | 951 |
| HSV1-UL19-566 | - | CGUACAGUUUAAGCGCCCCC | 20 | 952 |
| HSV1-UL19-567 | - | GUACAGUUUAAGCGCCCCCC | 20 | 953 |
| HSV1-UL19-568 | - | UACAGUUUAAGCGCCCCCCG | 20 | 954 |
| HSV1-UL19-569 | - | CCCGGGGUGCCGCGAACUCG | 20 | 955 |
| HSV1-UL19-570 | - | GGGGUGCCGCGAACUCGUGG | 20 | 956 |
| HSV1-UL19-571 | - | GAACUCGUGGAGGACCCGUG | 20 | 957 |
| HSV1-UL19-572 | - | GGACCCGUGUGGCCUGUUUC | 20 | 958 |
| HSV1-UL19-573 | - | CCCGUGUGGCCUGUUUCAGG | 20 | 959 |
| HSV1-UL19-574 | - | CUCCUCCGCAGUGCCCGCAA | 20 | 960 |
| HSV1-UL19-575 | - | UCCUCCGCAGUGCCCGCAAC | 20 | 961 |
| HSV1-UL19-576 | - | CCUCCGCAGUGCCCGCAACG | 20 | 962 |
| HSV1-UL19-577 | - | GCAACGGGGAAGCCCACGCG | 20 | 963 |
| HSV1-UL19-578 | - | CAACGGGGAAGCCCACGCGC | 20 | 964 |
| HSV1-UL19-579 | - | CUAUGACGCAUCCCCGCUCA | 20 | 965 |
| HSV1-UL19-580 | - | UAUGACGCAUCCCCGCUCAA | 20 | 966 |
| HSV1-UL19-581 | - | CGCAUCCCCGCUCAAGGGAC | 20 | 967 |
| HSV1-UL19-582 | - | AACGUCCAGGCCGUCCU | 17 | 968 |
| HSV1-UL19-583 | - | ACGUCCAGGCCGUCCUC | 17 | 969 |
| HSV1-UL19-584 | - | CGUCCAGGCCGUCCUCG | 17 | 970 |
| HSV1-UL19-585 | - | CUCGGGGCGUUUGAGCG | 17 | 971 |
| HSV1-UL19-586 | - | GGCGUUUGAGCGCGGCA | 17 | 972 |
| HSV1-UL19-587 | - | GAUGCUGCACGUGCUGC | 17 | 973 |
| HSV1-UL19-588 | - | GCACGUGCUGCUGGAAA | 17 | 974 |
| HSV1-UL19-589 | - | GGAAAAGGCGCCGCCCC | 17 | 975 |
| HSV1-UL19-590 | - | GCCGAUGCAACGAUACC | 17 | 976 |
| HSV1-UL19-591 | - | CAACGAUACCUGGACAA | 17 | 977 |
| HSV1-UL19-592 | - | CCUGGACAACGGCCGCC | 17 | 978 |
| HSV1-UL19-593 | - | ACGGCCGCCUGGCCACC | 17 | 979 |
| HSV1-UL19-594 | - | CGGCCGCCUGGCCACCA | 17 | 980 |
| HSV1-UL19-595 | - | CCGCCUGGCCACCAGGG | 17 | 981 |
| HSV1-UL19-596 | - | UGGCCACCAGGGUGGCC | 17 | 982 |
| HSV1-UL19-597 | - | GGCCACCAGGGUGGCCC | 17 | 983 |
| HSV1-UL19-598 | - | GGUGGCCCGGGCGACCC | 17 | 984 |
| HSV1-UL19-599 | - | CGAGACGAGCUUUUUCC | 17 | 985 |
| HSV1-UL19-600 | - | GAGACGAGCUUUUUCCU | 17 | 986 |
| HSV1-UL19-601 | - | GAGCUUUUUCCUGGGCA | 17 | 987 |
| HSV1-UL19-602 | - | CUUUUUCCUGGGCAAGG | 17 | 988 |
| HSV1-UL19-603 | - | UUUUUCCUGGGCAAGGC | 17 | 989 |
| HSV1-UL19-604 | - | GCAAGGCGGGCCACCGC | 17 | 990 |
| HSV1-UL19-605 | - | CAAGGCGGGCCACCGCC | 17 | 991 |
| HSV1-UL19-606 | - | GGCGGGCCACCGCCGGG | 17 | 992 |
| HSV1-UL19-607 | - | CCGCCGGGAGGCCGUCG | 17 | 993 |
| HSV1-UL19-608 | - | GGGAGGCCGUCGAGGCC | 17 | 994 |
| HSV1-UL19-609 | - | CGUCGAGGCCUGGCUCG | 17 | 995 |
| HSV1-UL19-610 | - | GCUCGUGGACCUCACCA | 17 | 996 |
| HSV1-UL19-611 | - | GGCCACGCAGCCCUCCG | 17 | 997 |
| HSV1-UL19-612 | - | ACGCAUGCCGACACGCG | 17 | 998 |
| HSV1-UL19-613 | - | CGCAUGCCGACACGCGC | 17 | 999 |
| HSV1-UL19-614 | - | AUGCCGACACGCGCGGG | 17 | 1000 |
| HSV1-UL19-615 | - | CGACACGCGCGGGCGGC | 17 | 1001 |
| HSV1-UL19-616 | - | CGCGGGCGGCCGGUCGA | 17 | 1002 |
| HSV1-UL19-617 | - | GCGGGCGGCCGGUCGAC | 17 | 1003 |
| HSV1-UL19-618 | - | CGGGCGGCCGGUCGACG | 17 | 1004 |
| HSV1-UL19-619 | - | GCAGUCCUUCCUGAAAG | 17 | 1005 |
| HSV1-UL19-620 | - | GUCCUUCCUGAAAGUGG | 17 | 1006 |
| HSV1-UL19-621 | - | CACCGAAGCCGACGUGC | 17 | 1007 |
| HSV1-UL19-622 | - | GACGUGCCGGUGACGUA | 17 | 1008 |
| HSV1-UL19-623 | - | GGUGACGUACGGCGAGA | 17 | 1009 |
| HSV1-UL19-624 | - | GGCGAGAUGGUCCUGAA | 17 | 1010 |
| HSV1-UL19-625 | - | GCGAGAUGGUCCUGAAC | 17 | 1011 |
| HSV1-UL19-626 | - | CGAGAUGGUCCUGAACG | 17 | 1012 |
| HSV1-UL19-627 | - | CCUGAACGGGGCCAACC | 17 | 1013 |
| HSV1-UL19-628 | - | CGGGGCCAACCUGGUCA | 17 | 1014 |
| HSV1-UL19-629 | - | GGUCACGGCGCUCGUGA | 17 | 1015 |
| HSV1-UL19-630 | - | GUCACGGCGCUCGUGAU | 17 | 1016 |
| HSV1-UL19-631 | - | GGCGCUCGUGAUGGGCA | 17 | 1017 |
| HSV1-UL19-632 | - | CAAGGCCGUGCGAAGUC | 17 | 1018 |
| HSV1-UL19-633 | - | GCGAAGUCUGGACGACG | 17 | 1019 |
| HSV1-UL19-634 | - | CGAAGUCUGGACGACGU | 17 | 1020 |
| HSV1-UL19-635 | - | CGUGGGCCGCCACCUGC | 17 | 1021 |
| HSV1-UL19-636 | - | CCACCUGCUGGAGAUGC | 17 | 1022 |
| HSV1-UL19-637 | - | CCUGCUGGAGAUGCAGG | 17 | 1023 |
| HSV1-UL19-638 | - | AGCAGCUCGACCUGAAC | 17 | 1024 |
| HSV1-UL19-639 | - | CCUGAACCGGCAGACGC | 17 | 1025 |
| HSV1-UL19-640 | - | GACGACGCGCGUGCGCG | 17 | 1026 |
| HSV1-UL19-641 | - | GCGCGUGCGCGCGGAUC | 17 | 1027 |
| HSV1-UL19-642 | - | GCGGAUCUGGUGUCCAU | 17 | 1028 |
| HSV1-UL19-643 | - | GUCCAUCGGCGAGAAGC | 17 | 1029 |
| HSV1-UL19-644 | - | CGAGAAGCUGGUCUUUC | 17 | 1030 |
| HSV1-UL19-645 | - | GAAGCUGGUCUUUCUGG | 17 | 1031 |
| HSV1-UL19-646 | - | GGUCUUUCUGGAGGCCC | 17 | 1032 |
| HSV1-UL19-647 | - | CAACGUUCCCUACCCCC | 17 | 1033 |
| HSV1-UL19-648 | - | CGUUCCCUACCCCCUGG | 17 | 1034 |
| HSV1-UL19-649 | - | GUUCCCUACCCCCUGGU | 17 | 1035 |
| HSV1-UL19-650 | - | CCCCCUGGUGGGCGCCA | 17 | 1036 |
| HSV1-UL19-651 | - | GACGUUCGUCCUGCCCC | 17 | 1037 |
| HSV1-UL19-652 | - | ACGUUCGUCCUGCCCCU | 17 | 1038 |
| HSV1-UL19-653 | - | CCUGGGCCUGUUCAAUC | 17 | 1039 |
| HSV1-UL19-654 | - | CCUGUUCAAUCCGGUCA | 17 | 1040 |
| HSV1-UL19-655 | - | UCAAUCCGGUCAUGGAA | 17 | 1041 |
| HSV1-UL19-656 | - | CGGUUUGCCGCGCACGC | 17 | 1042 |
| HSV1-UL19-657 | - | GGUUUGCCGCGCACGCC | 17 | 1043 |
| HSV1-UL19-658 | - | GUUUGCCGCGCACGCCG | 17 | 1044 |
| HSV1-UL19-659 | - | GACCUAGUCCCCGCCCC | 17 | 1045 |
| HSV1-UL19-660 | - | CCCCGCCCCCGGCCACC | 17 | 1046 |
| HSV1-UL19-661 | - | CCCGCCAGCUGUUUUUU | 17 | 1047 |
| HSV1-UL19-662 | - | CCGCCAGCUGUUUUUUU | 17 | 1048 |
| HSV1-UL19-663 | - | CGCCAGCUGUUUUUUUG | 17 | 1049 |
| HSV1-UL19-664 | - | GCCAGCUGUUUUUUUGG | 17 | 1050 |
| HSV1-UL19-665 | - | GCUGUUUUUUUGGGGGA | 17 | 1051 |
| HSV1-UL19-666 | - | UUGGGGGAAGGACCGCC | 17 | 1052 |
| HSV1-UL19-667 | - | GGUGCUGCGCCUGUCUC | 17 | 1053 |
| HSV1-UL19-668 | - | UCUCUGGAACACGCGAU | 17 | 1054 |
| HSV1-UL19-669 | - | CUCUGGAACACGCGAUC | 17 | 1055 |
| HSV1-UL19-670 | - | GCUGAUGAACGUUGACG | 17 | 1056 |
| HSV1-UL19-671 | - | GAUGAACGUUGACGCGG | 17 | 1057 |
| HSV1-UL19-672 | - | AACGUUGACGCGGCGGU | 17 | 1058 |
| HSV1-UL19-673 | - | ACGUUGACGCGGCGGUC | 17 | 1059 |
| HSV1-UL19-674 | - | CGUUGACGCGGCGGUCG | 17 | 1060 |
| HSV1-UL19-675 | - | GUUGACGCGGCGGUCGG | 17 | 1061 |
| HSV1-UL19-676 | - | GAAGCCGCCAAUCCGUA | 17 | 1062 |
| HSV1-UL19-677 | - | AAGCCGCCAAUCCGUAC | 17 | 1063 |
| HSV1-UL19-678 | - | AGCCGCCAAUCCGUACG | 17 | 1064 |
| HSV1-UL19-679 | - | UCCGUACGGGGCGUACG | 17 | 1065 |
| HSV1-UL19-680 | - | GUACGGGGCGUACGUGG | 17 | 1066 |
| HSV1-UL19-681 | - | GGCGUACGUGGCGGCCC | 17 | 1067 |
| HSV1-UL19-682 | - | UACGUGGCGGCCCCGGC | 17 | 1068 |
| HSV1-UL19-683 | - | AGCUGUUUUUGAACGCC | 17 | 1069 |
| HSV1-UL19-684 | - | GCUGUUUUUGAACGCCU | 17 | 1070 |
| HSV1-UL19-685 | - | CUGUUUUUGAACGCCUG | 17 | 1071 |
| HSV1-UL19-686 | - | UGUUUUUGAACGCCUGG | 17 | 1072 |
| HSV1-UL19-687 | - | CGCCUGGGGGCAGCGCC | 17 | 1073 |
| HSV1-UL19-688 | - | GGGCAGCGCCUGGCCCA | 17 | 1074 |
| HSV1-UL19-689 | - | GGCAGCGCCUGGCCCAC | 17 | 1075 |
| HSV1-UL19-690 | - | AGCGCCUGGCCCACGGG | 17 | 1076 |
| HSV1-UL19-691 | - | GCGCCUGGCCCACGGGC | 17 | 1077 |
| HSV1-UL19-692 | - | CCCACGGGCGGGUCCGA | 17 | 1078 |
| HSV1-UL19-693 | - | CCACGGGCGGGUCCGAU | 17 | 1079 |
| HSV1-UL19-694 | - | GCGGGUCCGAUGGGUCG | 17 | 1080 |
| HSV1-UL19-695 | - | GUCCGAUGGGUCGCGGA | 17 | 1081 |
| HSV1-UL19-696 | - | GGAAGGCCAGAUGACCC | 17 | 1082 |
| HSV1-UL19-697 | - | GCCCGACAACGCCAACC | 17 | 1083 |
| HSV1-UL19-698 | - | CGCGUUCGACUUCUUUG | 17 | 1084 |
| HSV1-UL19-699 | - | GCGUUCGACUUCUUUGU | 17 | 1085 |
| HSV1-UL19-700 | - | CGUUCGACUUCUUUGUG | 17 | 1086 |
| HSV1-UL19-701 | - | GUUCGACUUCUUUGUGG | 17 | 1087 |
| HSV1-UL19-702 | - | CGACUUCUUUGUGGGGG | 17 | 1088 |
| HSV1-UL19-703 | - | UGUGGGGGUGGCCGACG | 17 | 1089 |
| HSV1-UL19-704 | - | GGCCGACGUGGAGCUGC | 17 | 1090 |
| HSV1-UL19-705 | - | GCCGACGUGGAGCUGCC | 17 | 1091 |
| HSV1-UL19-706 | - | CCGACGUGGAGCUGCCG | 17 | 1092 |
| HSV1-UL19-707 | - | CGACGUGGAGCUGCCGG | 17 | 1093 |
| HSV1-UL19-708 | - | GACGUGGAGCUGCCGGG | 17 | 1094 |
| HSV1-UL19-709 | - | ACGUGGAGCUGCCGGGG | 17 | 1095 |
| HSV1-UL19-710 | - | CGUGGAGCUGCCGGGGG | 17 | 1096 |
| HSV1-UL19-711 | - | GGGGGGGGACGUUCCCC | 17 | 1097 |
| HSV1-UL19-712 | - | GGGGACGUUCCCCCGGC | 17 | 1098 |
| HSV1-UL19-713 | - | CGUUCCCCCGGCCGGCC | 17 | 1099 |
| HSV1-UL19-714 | - | GUUCCCCCGGCCGGCCC | 17 | 1100 |
| HSV1-UL19-715 | - | UUCCCCCGGCCGGCCCG | 17 | 1101 |
| HSV1-UL19-716 | - | UCCCCCGGCCGGCCCGG | 17 | 1102 |
| HSV1-UL19-717 | - | CGGCCCGGGGGAGAUCC | 17 | 1103 |
| HSV1-UL19-718 | - | GGGAGAUCCAGGCCACC | 17 | 1104 |
| HSV1-UL19-719 | - | CCAGGCCACCUGGCGCG | 17 | 1105 |
| HSV1-UL19-720 | - | ACCUGGCGCGUGGUGAA | 17 | 1106 |
| HSV1-UL19-721 | - | GAACGGCAACUUGCCCC | 17 | 1107 |
| HSV1-UL19-722 | - | GCCCCUGGCGCUAUGUC | 17 | 1108 |
| HSV1-UL19-723 | - | CCUGGCGCUAUGUCCGG | 17 | 1109 |
| HSV1-UL19-724 | - | UAUGUCCGGCGGCGUUC | 17 | 1110 |
| HSV1-UL19-725 | - | AUGUCCGGCGGCGUUCC | 17 | 1111 |
| HSV1-UL19-726 | - | CGGCGUUCCGGGACGCC | 17 | 1112 |
| HSV1-UL19-727 | - | GGCGUUCCGGGACGCCC | 17 | 1113 |
| HSV1-UL19-728 | - | GCGUUCCGGGACGCCCG | 17 | 1114 |
| HSV1-UL19-729 | - | CCGGGACGCCCGGGGCC | 17 | 1115 |
| HSV1-UL19-730 | - | CGCCCGGGGCCUGGAGC | 17 | 1116 |
| HSV1-UL19-731 | - | GCCCGGGGCCUGGAGCU | 17 | 1117 |
| HSV1-UL19-732 | - | CCCGGGGCCUGGAGCUG | 17 | 1118 |
| HSV1-UL19-733 | - | CCGGGGCCUGGAGCUGG | 17 | 1119 |
| HSV1-UL19-734 | - | GGGCCUGGAGCUGGGGG | 17 | 1120 |
| HSV1-UL19-735 | - | GGCCUGGAGCUGGGGGU | 17 | 1121 |
| HSV1-UL19-736 | - | GGUGGGACGCCACGCCA | 17 | 1122 |
| HSV1-UL19-737 | - | ACCAUCGCCGCCGUUCG | 17 | 1123 |
| HSV1-UL19-738 | - | CCAUCGCCGCCGUUCGC | 17 | 1124 |
| HSV1-UL19-739 | - | CAUCGCCGCCGUUCGCG | 17 | 1125 |
| HSV1-UL19-740 | - | CGACGACCGCAACUACC | 17 | 1126 |
| HSV1-UL19-741 | - | CGACCGCAACUACCCGG | 17 | 1127 |
| HSV1-UL19-742 | - | GGUGUUUUACCUGCUGC | 17 | 1128 |
| HSV1-UL19-743 | - | CUGCAGGCCGCCAUACA | 17 | 1129 |
| HSV1-UL19-744 | - | GCACGUCUUCUGCGCCC | 17 | 1130 |
| HSV1-UL19-745 | - | UCUUCUGCGCCCUGGCC | 17 | 1131 |
| HSV1-UL19-746 | - | CGCCCUGGCCCGGCUCG | 17 | 1132 |
| HSV1-UL19-747 | - | AGUGCAUCACCAGCUAC | 17 | 1133 |
| HSV1-UL19-748 | - | GCUACUGGAACAACACG | 17 | 1134 |
| HSV1-UL19-749 | - | GAACAACACGCGGUGCG | 17 | 1135 |
| HSV1-UL19-750 | - | CGUGAACGACUACUCGC | 17 | 1136 |
| HSV1-UL19-751 | - | UACGUCGUGACCUACCU | 17 | 1137 |
| HSV1-UL19-752 | - | ACGUCGUGACCUACCUC | 17 | 1138 |
| HSV1-UL19-753 | - | CGUCGUGACCUACCUCG | 17 | 1139 |
| HSV1-UL19-754 | - | GUCGUGACCUACCUCGG | 17 | 1140 |
| HSV1-UL19-755 | - | CGGGGGAGACCUUCCCG | 17 | 1141 |
| HSV1-UL19-756 | - | CCUUCCCGAGGAGUGCA | 17 | 1142 |
| HSV1-UL19-757 | - | AGUGCAUGGCCGUGUAC | 17 | 1143 |
| HSV1-UL19-758 | - | GUGCAUGGCCGUGUACC | 17 | 1144 |
| HSV1-UL19-759 | - | GGCCGUGUACCGGGACC | 17 | 1145 |
| HSV1-UL19-760 | - | CGUGUACCGGGACCUGG | 17 | 1146 |
| HSV1-UL19-761 | - | CCUGGUGGCGCACGUCG | 17 | 1147 |
| HSV1-UL19-762 | - | GGCGCACGUCGAGGCCC | 17 | 1148 |
| HSV1-UL19-763 | - | CGAGGCCCUGGCCCAGC | 17 | 1149 |
| HSV1-UL19-764 | - | GAUGACUUUACCCUGAC | 17 | 1150 |
| HSV1-UL19-765 | - | CUUUACCCUGACCGGCC | 17 | 1151 |
| HSV1-UL19-766 | - | CCUGACCGGCCCGGAGC | 17 | 1152 |
| HSV1-UL19-767 | - | CUGACCGGCCCGGAGCU | 17 | 1153 |
| HSV1-UL19-768 | - | ACCGGCCCGGAGCUGGG | 17 | 1154 |
| HSV1-UL19-769 | - | CCGGCCCGGAGCUGGGC | 17 | 1155 |
| HSV1-UL19-770 | - | CCCGGAGCUGGGCGGGC | 17 | 1156 |
| HSV1-UL19-771 | - | UCACCUAAUGCGAGACC | 17 | 1157 |
| HSV1-UL19-772 | - | UGCUGCCACCCCUCGUG | 17 | 1158 |
| HSV1-UL19-773 | - | GCUGCCACCCCUCGUGU | 17 | 1159 |
| HSV1-UL19-774 | - | ACUGUGACGCCCUGAUG | 17 | 1160 |
| HSV1-UL19-775 | - | CGCCCUGAUGCGGCGCG | 17 | 1161 |
| HSV1-UL19-776 | - | GAUGCGGCGCGCGGCCC | 17 | 1162 |
| HSV1-UL19-777 | - | ACCGCCAUCGCGACUGC | 17 | 1163 |
| HSV1-UL19-778 | - | CCGCCAUCGCGACUGCC | 17 | 1164 |
| HSV1-UL19-779 | - | CGACUGCCGGGUUAGCG | 17 | 1165 |
| HSV1-UL19-780 | - | GACUGCCGGGUUAGCGC | 17 | 1166 |
| HSV1-UL19-781 | - | ACUGCCGGGUUAGCGCG | 17 | 1167 |
| HSV1-UL19-782 | - | CUGCCGGGUUAGCGCGG | 17 | 1168 |
| HSV1-UL19-783 | - | UGCCGGGUUAGCGCGGG | 17 | 1169 |
| HSV1-UL19-784 | - | CCACGACCCCGUGUACG | 17 | 1170 |
| HSV1-UL19-785 | - | CGACCCCGUGUACGCGG | 17 | 1171 |
| HSV1-UL19-786 | - | CGCGGCGGCAUGUAACG | 17 | 1172 |
| HSV1-UL19-787 | - | AUGUAACGUGGCGACCG | 17 | 1173 |
| HSV1-UL19-788 | - | GACUUCAACCGCAACGA | 17 | 1174 |
| HSV1-UL19-789 | - | GCUGCUGCACAACACCC | 17 | 1175 |
| HSV1-UL19-790 | - | CACCCAGGCCCGAGCCG | 17 | 1176 |
| HSV1-UL19-791 | - | CCGAGCCGCGGACGCCG | 17 | 1177 |
| HSV1-UL19-792 | - | CGGACGCCGCGGAUGAC | 17 | 1178 |
| HSV1-UL19-793 | - | CGGAUGACCGGCCGCAC | 17 | 1179 |
| HSV1-UL19-794 | - | GGAUGACCGGCCGCACC | 17 | 1180 |
| HSV1-UL19-795 | - | GAUGACCGGCCGCACCG | 17 | 1181 |
| HSV1-UL19-796 | - | AUGACCGGCCGCACCGG | 17 | 1182 |
| HSV1-UL19-797 | - | UGACCGGCCGCACCGGG | 17 | 1183 |
| HSV1-UL19-798 | - | CCGGCCGCACCGGGGGG | 17 | 1184 |
| HSV1-UL19-799 | - | CGCACCGGGGGGCGGAC | 17 | 1185 |
| HSV1-UL19-800 | - | GAUUUACUACUACGUGA | 17 | 1186 |
| HSV1-UL19-801 | - | UGGUGCCCGCCUUCUCG | 17 | 1187 |
| HSV1-UL19-802 | - | GGUGCCCGCCUUCUCGC | 17 | 1188 |
| HSV1-UL19-803 | - | GUGCCCGCCUUCUCGCG | 17 | 1189 |
| HSV1-UL19-804 | - | GGGCCGCUGCUGCACCG | 17 | 1190 |
| HSV1-UL19-805 | - | GGCCGCUGCUGCACCGC | 17 | 1191 |
| HSV1-UL19-806 | - | GCCGCUGCUGCACCGCG | 17 | 1192 |
| HSV1-UL19-807 | - | CCGCUGCUGCACCGCGG | 17 | 1193 |
| HSV1-UL19-808 | - | CGCCACCCUGCAGAACA | 17 | 1194 |
| HSV1-UL19-809 | - | CACCCUGCAGAACAUGG | 17 | 1195 |
| HSV1-UL19-810 | - | GCAGAACAUGGUGGUCC | 17 | 1196 |
| HSV1-UL19-811 | - | GUCCCGGAGAUCGCCCC | 17 | 1197 |
| HSV1-UL19-812 | - | GGAGAUCGCCCCCGGCG | 17 | 1198 |
| HSV1-UL19-813 | - | CCCCAGCGACCCCGUGA | 17 | 1199 |
| HSV1-UL19-814 | - | CGCGCACCCCCUGCACC | 17 | 1200 |
| HSV1-UL19-815 | - | CCUGCACCCGGCCAAUC | 17 | 1201 |
| HSV1-UL19-816 | - | GCACCCGGCCAAUCUGG | 17 | 1202 |
| HSV1-UL19-817 | - | CAAUCUGGUGGCCAACA | 17 | 1203 |
| HSV1-UL19-818 | - | AACGCCAUGUUUCACAA | 17 | 1204 |
| HSV1-UL19-819 | - | ACGCCAUGUUUCACAAC | 17 | 1205 |
| HSV1-UL19-820 | - | GUUUCACAACGGGCGCG | 17 | 1206 |
| HSV1-UL19-821 | - | CAACGGGCGCGUGGUAG | 17 | 1207 |
| HSV1-UL19-822 | - | GGGCGCGUGGUAGUGGA | 17 | 1208 |
| HSV1-UL19-823 | - | GGCGCGUGGUAGUGGAC | 17 | 1209 |
| HSV1-UL19-824 | - | CGCCAUGCUCACGCUGC | 17 | 1210 |
| HSV1-UL19-825 | - | GCUCACGCUGCAGGUGC | 17 | 1211 |
| HSV1-UL19-826 | - | GGUGCUGGCCCACAACA | 17 | 1212 |
| HSV1-UL19-827 | - | CAUGGCCGAACGCACAA | 17 | 1213 |
| HSV1-UL19-828 | - | AACGGCGCUGCUCUGCU | 17 | 1214 |
| HSV1-UL19-829 | - | GGCGCUGCUCUGCUCGG | 17 | 1215 |
| HSV1-UL19-830 | - | CUCGGCGGCGCCCGACG | 17 | 1216 |
| HSV1-UL19-831 | - | UCGGCGGCGCCCGACGC | 17 | 1217 |
| HSV1-UL19-832 | - | AACAUGCGCAUAUUCGA | 17 | 1218 |
| HSV1-UL19-833 | - | ACAUGCGCAUAUUCGAC | 17 | 1219 |
| HSV1-UL19-834 | - | CAUGCGCAUAUUCGACG | 17 | 1220 |
| HSV1-UL19-835 | - | GACGGGGCGUUGCACGC | 17 | 1221 |
| HSV1-UL19-836 | - | CGCCGGAAUCCUGCUGA | 17 | 1222 |
| HSV1-UL19-837 | - | GAUGGCCCCCCAGCAUC | 17 | 1223 |
| HSV1-UL19-838 | - | GACCAUACCAUCCAAAA | 17 | 1224 |
| HSV1-UL19-839 | - | GUCCACGCGCUGUUCGC | 17 | 1225 |
| HSV1-UL19-840 | - | UCCACGCGCUGUUCGCC | 17 | 1226 |
| HSV1-UL19-841 | - | CCACGCGCUGUUCGCCG | 17 | 1227 |
| HSV1-UL19-842 | - | CGCCGGGGCCGACCACG | 17 | 1228 |
| HSV1-UL19-843 | - | CGCGCCCAAUUUCCCCC | 17 | 1229 |
| HSV1-UL19-844 | - | CCCUGCGCGACCUGUCG | 17 | 1230 |
| HSV1-UL19-845 | - | GCGCGACCUGUCGCGGC | 17 | 1231 |
| HSV1-UL19-846 | - | GUCGCGGCAGGUCCCCC | 17 | 1232 |
| HSV1-UL19-847 | - | GGUCCCCCUGGUCCCCC | 17 | 1233 |
| HSV1-UL19-848 | - | CCUGGUCCCCCCGGCUC | 17 | 1234 |
| HSV1-UL19-849 | - | CUGGUCCCCCCGGCUCU | 17 | 1235 |
| HSV1-UL19-850 | - | UGGUCCCCCCGGCUCUG | 17 | 1236 |
| HSV1-UL19-851 | - | GGUCCCCCCGGCUCUGG | 17 | 1237 |
| HSV1-UL19-852 | - | GCACGUCCGCGAGAGCG | 17 | 1238 |
| HSV1-UL19-853 | - | GUCCGCGAGAGCGCGGC | 17 | 1239 |
| HSV1-UL19-854 | - | UCCGCGAGAGCGCGGCC | 17 | 1240 |
| HSV1-UL19-855 | - | CCGCGAGAGCGCGGCCG | 17 | 1241 |
| HSV1-UL19-856 | - | GCUGACCUACGCGCUCA | 17 | 1242 |
| HSV1-UL19-857 | - | GACCUACGCGCUCAUGG | 17 | 1243 |
| HSV1-UL19-858 | - | ACCUACGCGCUCAUGGC | 17 | 1244 |
| HSV1-UL19-859 | - | CCUACGCGCUCAUGGCG | 17 | 1245 |
| HSV1-UL19-860 | - | CUUCAAGAUCAGUCCCG | 17 | 1246 |
| HSV1-UL19-861 | - | GCAUCAUCAGCUCAAGA | 17 | 1247 |
| HSV1-UL19-862 | - | CAUCAUCAGCUCAAGAC | 17 | 1248 |
| HSV1-UL19-863 | - | AAGACGGGCCUCCAUCC | 17 | 1249 |
| HSV1-UL19-864 | - | AGACGGGCCUCCAUCCC | 17 | 1250 |
| HSV1-UL19-865 | - | GGCCUCCAUCCCGGGUU | 17 | 1251 |
| HSV1-UL19-866 | - | GCCUCCAUCCCGGGUUU | 17 | 1252 |
| HSV1-UL19-867 | - | GUUCACCGUCGUCCGAC | 17 | 1253 |
| HSV1-UL19-868 | - | UGAGAACGUGCUGUUCU | 17 | 1254 |
| HSV1-UL19-869 | - | GUUCUCGGAGCGCGCGU | 17 | 1255 |
| HSV1-UL19-870 | - | CUCGGAGCGCGCGUCGG | 17 | 1256 |
| HSV1-UL19-871 | - | GUCGGAGGCGUACUUCC | 17 | 1257 |
| HSV1-UL19-872 | - | UCGGAGGCGUACUUCCU | 17 | 1258 |
| HSV1-UL19-873 | - | CUUCCUGGGCCAGCUCC | 17 | 1259 |
| HSV1-UL19-874 | - | CCUGGGCCAGCUCCAGG | 17 | 1260 |
| HSV1-UL19-875 | - | GCCAGCUCCAGGUGGCC | 17 | 1261 |
| HSV1-UL19-876 | - | GUGGCCCGGCACGAAAC | 17 | 1262 |
| HSV1-UL19-877 | - | GCCCGGCACGAAACUGG | 17 | 1263 |
| HSV1-UL19-878 | - | CCCGGCACGAAACUGGC | 17 | 1264 |
| HSV1-UL19-879 | - | CCGGCACGAAACUGGCG | 17 | 1265 |
| HSV1-UL19-880 | - | CGGCACGAAACUGGCGG | 17 | 1266 |
| HSV1-UL19-881 | - | GGCACGAAACUGGCGGG | 17 | 1267 |
| HSV1-UL19-882 | - | GCACGAAACUGGCGGGG | 17 | 1268 |
| HSV1-UL19-883 | - | CCAGCCGCGCGCGAACG | 17 | 1269 |
| HSV1-UL19-884 | - | GCGCGCGAACGUGGACC | 17 | 1270 |
| HSV1-UL19-885 | - | CGCGCGAACGUGGACCU | 17 | 1271 |
| HSV1-UL19-886 | - | GAACGUGGACCUGGGCG | 17 | 1272 |
| HSV1-UL19-887 | - | AACGUGGACCUGGGCGU | 17 | 1273 |
| HSV1-UL19-888 | - | GGGCUACACCGCCGUCG | 17 | 1274 |
| HSV1-UL19-889 | - | CACCGCCGUCGUGGCCA | 17 | 1275 |
| HSV1-UL19-890 | - | CAACCCCGUCACCGACA | 17 | 1276 |
| HSV1-UL19-891 | - | AACCCCGUCACCGACAU | 17 | 1277 |
| HSV1-UL19-892 | - | UCCCCAAAACUUUUACC | 17 | 1278 |
| HSV1-UL19-893 | - | CCCCAAAACUUUUACCU | 17 | 1279 |
| HSV1-UL19-894 | - | AACUUUUACCUGGGCCG | 17 | 1280 |
| HSV1-UL19-895 | - | ACUUUUACCUGGGCCGC | 17 | 1281 |
| HSV1-UL19-896 | - | CUUUUACCUGGGCCGCG | 17 | 1282 |
| HSV1-UL19-897 | - | CGGGGCUCCCCCUCUCC | 17 | 1283 |
| HSV1-UL19-898 | - | CCCUCUCCUGGACAACG | 17 | 1284 |
| HSV1-UL19-899 | - | CGGCAGCCGUGUACCUG | 17 | 1285 |
| HSV1-UL19-900 | - | CGUGUACCUGCGGAACG | 17 | 1286 |
| HSV1-UL19-901 | - | CCUGCGGAACGCGGUCG | 17 | 1287 |
| HSV1-UL19-902 | - | GCGGAACGCGGUCGUGG | 17 | 1288 |
| HSV1-UL19-903 | - | CGGAACGCGGUCGUGGC | 17 | 1289 |
| HSV1-UL19-904 | - | CGUGGCGGGAAACCGCC | 17 | 1290 |
| HSV1-UL19-905 | - | GUGGCGGGAAACCGCCU | 17 | 1291 |
| HSV1-UL19-906 | - | UGGCGGGAAACCGCCUG | 17 | 1292 |
| HSV1-UL19-907 | - | GGGAAACCGCCUGGGGC | 17 | 1293 |
| HSV1-UL19-908 | - | CAGCCCGUCCCCGUGUU | 17 | 1294 |
| HSV1-UL19-909 | - | AGCCCGUCCCCGUGUUC | 17 | 1295 |
| HSV1-UL19-910 | - | CGUGUUCGGGUGCGCCC | 17 | 1296 |
| HSV1-UL19-911 | - | GGUGCGCCCAGGUGCCG | 17 | 1297 |
| HSV1-UL19-912 | - | CAGGUGCCGCGGCGCGC | 17 | 1298 |
| HSV1-UL19-913 | - | AGGUGCCGCGGCGCGCA | 17 | 1299 |
| HSV1-UL19-914 | - | GCCGCGGCGCGCAGGGA | 17 | 1300 |
| HSV1-UL19-915 | - | CGCGCAGGGAUGGACCA | 17 | 1301 |
| HSV1-UL19-916 | - | AGGGAUGGACCACGGCC | 17 | 1302 |
| HSV1-UL19-917 | - | ACGUCAACUACUUCCGC | 17 | 1303 |
| HSV1-UL19-918 | - | GCCGGCCCUGCAACCCC | 17 | 1304 |
| HSV1-UL19-919 | - | CCGGCCCUGCAACCCCC | 17 | 1305 |
| HSV1-UL19-920 | - | CGGCCCUGCAACCCCCG | 17 | 1306 |
| HSV1-UL19-921 | - | CCCCGGGGACGCGCCGC | 17 | 1307 |
| HSV1-UL19-922 | - | CCCGGGGACGCGCCGCC | 17 | 1308 |
| HSV1-UL19-923 | - | CCGGGGACGCGCCGCCG | 17 | 1309 |
| HSV1-UL19-924 | - | CGGGGACGCGCCGCCGG | 17 | 1310 |
| HSV1-UL19-925 | - | CGCCGGGGGCGUUUACG | 17 | 1311 |
| HSV1-UL19-926 | - | GCCGGGGGCGUUUACGC | 17 | 1312 |
| HSV1-UL19-927 | - | CCGGGGGCGUUUACGCG | 17 | 1313 |
| HSV1-UL19-928 | - | CGGGGGCGUUUACGCGG | 17 | 1314 |
| HSV1-UL19-929 | - | CGUUUACGCGGGGGACA | 17 | 1315 |
| HSV1-UL19-930 | - | UUACGCGGGGGACAAGG | 17 | 1316 |
| HSV1-UL19-931 | - | UACGCGGGGGACAAGGA | 17 | 1317 |
| HSV1-UL19-932 | - | ACGCGGGGGACAAGGAG | 17 | 1318 |
| HSV1-UL19-933 | - | CGCGGGGGACAAGGAGG | 17 | 1319 |
| HSV1-UL19-934 | - | GCCCUCAUGUACGACCA | 17 | 1320 |
| HSV1-UL19-935 | - | GCCAGAGCGACCCGUCC | 17 | 1321 |
| HSV1-UL19-936 | - | CCAGAGCGACCCGUCCC | 17 | 1322 |
| HSV1-UL19-937 | - | CCCGUCCCGGGCCUUCG | 17 | 1323 |
| HSV1-UL19-938 | - | CCGGGCCUUCGCGGCCA | 17 | 1324 |
| HSV1-UL19-939 | - | CGGCCACGGCCAACCCG | 17 | 1325 |
| HSV1-UL19-940 | - | GGCCACGGCCAACCCGU | 17 | 1326 |
| HSV1-UL19-941 | - | UCGCAGCGAUUUUCGUA | 17 | 1327 |
| HSV1-UL19-942 | - | CGCAGCGAUUUUCGUAC | 17 | 1328 |
| HSV1-UL19-943 | - | GCAGCGAUUUUCGUACG | 17 | 1329 |
| HSV1-UL19-944 | - | GGGGACCUGCUCUAUAA | 17 | 1330 |
| HSV1-UL19-945 | - | GGGACCUGCUCUAUAAC | 17 | 1331 |
| HSV1-UL19-946 | - | GGACCUGCUCUAUAACG | 17 | 1332 |
| HSV1-UL19-947 | - | GGGGCCUACCACCUCAA | 17 | 1333 |
| HSV1-UL19-948 | - | GGGCCUACCACCUCAAC | 17 | 1334 |
| HSV1-UL19-949 | - | GGCCUACCACCUCAACG | 17 | 1335 |
| HSV1-UL19-950 | - | CCUCAACGGGGCCUCGC | 17 | 1336 |
| HSV1-UL19-951 | - | CUUUAAGUUCUUUACGU | 17 | 1337 |
| HSV1-UL19-952 | - | CGCCAAACAUCGCUGCC | 17 | 1338 |
| HSV1-UL19-953 | - | CCUGGAGCGCCUGAUCG | 17 | 1339 |
| HSV1-UL19-954 | - | GCGCCUGAUCGUGGAGA | 17 | 1340 |
| HSV1-UL19-955 | - | CGCCUGAUCGUGGAGAC | 17 | 1341 |
| HSV1-UL19-956 | - | GAUCGUGGAGACGGGUU | 17 | 1342 |
| HSV1-UL19-957 | - | CGUGGAGACGGGUUCGG | 17 | 1343 |
| HSV1-UL19-958 | - | GGGUUCGGCGGUGUCCA | 17 | 1344 |
| HSV1-UL19-959 | - | ACAGUUUAAGCGCCCCC | 17 | 1345 |
| HSV1-UL19-960 | - | CAGUUUAAGCGCCCCCC | 17 | 1346 |
| HSV1-UL19-961 | - | AGUUUAAGCGCCCCCCG | 17 | 1347 |
| HSV1-UL19-962 | - | GGGGUGCCGCGAACUCG | 17 | 1348 |
| HSV1-UL19-963 | - | GUGCCGCGAACUCGUGG | 17 | 1349 |
| HSV1-UL19-964 | - | CUCGUGGAGGACCCGUG | 17 | 1350 |
| HSV1-UL19-965 | - | CCCGUGUGGCCUGUUUC | 17 | 1351 |
| HSV1-UL19-966 | - | GUGUGGCCUGUUUCAGG | 17 | 1352 |
| HSV1-UL19-967 | - | CUCCGCAGUGCCCGCAA | 17 | 1353 |
| HSV1-UL19-968 | - | UCCGCAGUGCCCGCAAC | 17 | 1354 |
| HSV1-UL19-969 | - | CCGCAGUGCCCGCAACG | 17 | 1355 |
| HSV1-UL19-970 | - | ACGGGGAAGCCCACGCG | 17 | 1356 |
| HSV1-UL19-971 | - | CGGGGAAGCCCACGCGC | 17 | 1357 |
| HSV1-UL19-972 | - | UGACGCAUCCCCGCUCA | 17 | 1358 |
| HSV1-UL19-973 | - | GACGCAUCCCCGCUCAA | 17 | 1359 |
| HSV1-UL19-974 | - | AUCCCCGCUCAAGGGAC | 17 | 1360 |
| HSV1-UL19-975 | + | CAAACGCCCCGAGGACGGCC | 20 | 1361 |
| HSV1-UL19-976 | + | GCGCUCAAACGCCCCGAGGA | 20 | 1362 |
| HSV1-UL19-977 | + | UGCCGCGCUCAAACGCCCCG | 20 | 1363 |
| HSV1-UL19-978 | + | CAGCACGUGCAGCAUCUGGU | 20 | 1364 |
| HSV1-UL19-979 | + | CCAGCAGCACGUGCAGCAUC | 20 | 1365 |
| HSV1-UL19-980 | + | GGCAACAACAAAGCCAGGGG | 20 | 1366 |
| HSV1-UL19-981 | + | AUCGGCAACAACAAAGCCAG | 20 | 1367 |
| HSV1-UL19-982 | + | CAUCGGCAACAACAAAGCCA | 20 | 1368 |
| HSV1-UL19-983 | + | GCAUCGGCAACAACAAAGCC | 20 | 1369 |
| HSV1-UL19-984 | + | UUGUCCAGGUAUCGUUGCAU | 20 | 1370 |
| HSV1-UL19-985 | + | UGGCCAGGCGGCCGUUGUCC | 20 | 1371 |
| HSV1-UL19-986 | + | GGGCCACCCUGGUGGCCAGG | 20 | 1372 |
| HSV1-UL19-987 | + | CCCGGGCCACCCUGGUGGCC | 20 | 1373 |
| HSV1-UL19-988 | + | GGUCGCCCGGGCCACCCUGG | 20 | 1374 |
| HSV1-UL19-989 | + | CAGGGUCGCCCGGGCCACCC | 20 | 1375 |
| HSV1-UL19-990 | + | CUCGGCGACCAGGGUCGCCC | 20 | 1376 |
| HSV1-UL19-991 | + | GCUCGGCGACCAGGGUCGCC | 20 | 1377 |
| HSV1-UL19-992 | + | UCGCUUUAGCUCGGCGACCA | 20 | 1378 |
| HSV1-UL19-993 | + | UUCGCUUUAGCUCGGCGACC | 20 | 1379 |
| HSV1-UL19-994 | + | GCAGAAGCUUCGCUUUAGCU | 20 | 1380 |
| HSV1-UL19-995 | + | GGCGGUGGCCCGCCUUGCCC | 20 | 1381 |
| HSV1-UL19-996 | + | CCUCGACGGCCUCCCGGCGG | 20 | 1382 |
| HSV1-UL19-997 | + | AGGCCUCGACGGCCUCCCGG | 20 | 1383 |
| HSV1-UL19-998 | + | GCCAGGCCUCGACGGCCUCC | 20 | 1384 |
| HSV1-UL19-999 | + | GUCCACGAGCCAGGCCUCGA | 20 | 1385 |
| HSV1-UL19-1000 | + | CGUGGUGAGGUCCACGAGCC | 20 | 1386 |
| HSV1-UL19-1001 | + | AGGGCUGCGUGGCCGUGGUG | 20 | 1387 |
| HSV1-UL19-1002 | + | CACGGAGGGCUGCGUGGCCG | 20 | 1388 |
| HSV1-UL19-1003 | + | CACGGCCACGGAGGGCUGCG | 20 | 1389 |
| HSV1-UL19-1004 | + | AGACGGGGCACGGCCACGGA | 20 | 1390 |
| HSV1-UL19-1005 | + | CAGACGGGGCACGGCCACGG | 20 | 1391 |
| HSV1-UL19-1006 | + | CGUCAGACGGGGCACGGCCA | 20 | 1392 |
| HSV1-UL19-1007 | + | GGCAUGCGUCAGACGGGGCA | 20 | 1393 |
| HSV1-UL19-1008 | + | GUGUCGGCAUGCGUCAGACG | 20 | 1394 |
| HSV1-UL19-1009 | + | CGUGUCGGCAUGCGUCAGAC | 20 | 1395 |
| HSV1-UL19-1010 | + | GCGUGUCGGCAUGCGUCAGA | 20 | 1396 |
| HSV1-UL19-1011 | + | GACCGGCCGCCCGCGCGUGU | 20 | 1397 |
| HSV1-UL19-1012 | + | GUGACGAGCACCCCGUCGAC | 20 | 1398 |
| HSV1-UL19-1013 | + | GCGCUGUUUGAUCGGGGCGG | 20 | 1399 |
| HSV1-UL19-1014 | + | GAGGCGCUGUUUGAUCGGGG | 20 | 1400 |
| HSV1-UL19-1015 | + | CAGGAGGCGCUGUUUGAUCG | 20 | 1401 |
| HSV1-UL19-1016 | + | GCAGGAGGCGCUGUUUGAUC | 20 | 1402 |
| HSV1-UL19-1017 | + | UGCAGGAGGCGCUGUUUGAU | 20 | 1403 |
| HSV1-UL19-1018 | + | CUUUCAGGAAGGACUGCAGG | 20 | 1404 |
| HSV1-UL19-1019 | + | CCACUUUCAGGAAGGACUGC | 20 | 1405 |
| HSV1-UL19-1020 | + | GGUGUCCUCCACUUUCAGGA | 20 | 1406 |
| HSV1-UL19-1021 | + | CUUCGGUGUCCUCCACUUUC | 20 | 1407 |
| HSV1-UL19-1022 | + | CGUCACCGGCACGUCGGCUU | 20 | 1408 |
| HSV1-UL19-1023 | + | GCCGUACGUCACCGGCACGU | 20 | 1409 |
| HSV1-UL19-1024 | + | ACCAUCUCGCCGUACGUCAC | 20 | 1410 |
| HSV1-UL19-1025 | + | UGACCAGGUUGGCCCCGUUC | 20 | 1411 |
| HSV1-UL19-1026 | + | CACGAGCGCCGUGACCAGGU | 20 | 1412 |
| HSV1-UL19-1027 | + | CCAUCACGAGCGCCGUGACC | 20 | 1413 |
| HSV1-UL19-1028 | + | CACGUCGUCCAGACUUCGCA | 20 | 1414 |
| HSV1-UL19-1029 | + | CCUGCAUCUCCAGCAGGUGG | 20 | 1415 |
| HSV1-UL19-1030 | + | CCUCCUGCAUCUCCAGCAGG | 20 | 1416 |
| HSV1-UL19-1031 | + | GCUCCUCCUGCAUCUCCAGC | 20 | 1417 |
| HSV1-UL19-1032 | + | CGUCCAGCGUCUGCCGGUUC | 20 | 1418 |
| HSV1-UL19-1033 | + | CGAGCUCGUCCAGCGUCUGC | 20 | 1419 |
| HSV1-UL19-1034 | + | GCGCACGCGCGUCGUCUGGG | 20 | 1420 |
| HSV1-UL19-1035 | + | CGCGCACGCGCGUCGUCUGG | 20 | 1421 |
| HSV1-UL19-1036 | + | GCGCGCACGCGCGUCGUCUG | 20 | 1422 |
| HSV1-UL19-1037 | + | CGCGCGCACGCGCGUCGUCU | 20 | 1423 |
| HSV1-UL19-1038 | + | CCGCGCGCACGCGCGUCGUC | 20 | 1424 |
| HSV1-UL19-1039 | + | AAAGACCAGCUUCUCGCCGA | 20 | 1425 |
| HSV1-UL19-1040 | + | GGCGUAGAUGCGCUUCUCCA | 20 | 1426 |
| HSV1-UL19-1041 | + | CGGCGUAGAUGCGCUUCUCC | 20 | 1427 |
| HSV1-UL19-1042 | + | GGGGUAGGGAACGUUGGUGG | 20 | 1428 |
| HSV1-UL19-1043 | + | CAGGGGGUAGGGAACGUUGG | 20 | 1429 |
| HSV1-UL19-1044 | + | CACCAGGGGGUAGGGAACGU | 20 | 1430 |
| HSV1-UL19-1045 | + | AUGGCGCCCACCAGGGGGUA | 20 | 1431 |
| HSV1-UL19-1046 | + | CAUGGCGCCCACCAGGGGGU | 20 | 1432 |
| HSV1-UL19-1047 | + | GGUCCAUGGCGCCCACCAGG | 20 | 1433 |
| HSV1-UL19-1048 | + | AGGUCCAUGGCGCCCACCAG | 20 | 1434 |
| HSV1-UL19-1049 | + | CAGGUCCAUGGCGCCCACCA | 20 | 1435 |
| HSV1-UL19-1050 | + | UCAGGUCCAUGGCGCCCACC | 20 | 1436 |
| HSV1-UL19-1051 | + | CAGGACGAACGUCAGGUCCA | 20 | 1437 |
| HSV1-UL19-1052 | + | CCAGGGGCAGGACGAACGUC | 20 | 1438 |
| HSV1-UL19-1053 | + | GAUUGAACAGGCCCAGGGGC | 20 | 1439 |
| HSV1-UL19-1054 | + | ACCGGAUUGAACAGGCCCAG | 20 | 1440 |
| HSV1-UL19-1055 | + | GACCGGAUUGAACAGGCCCA | 20 | 1441 |
| HSV1-UL19-1056 | + | UGACCGGAUUGAACAGGCCC | 20 | 1442 |
| HSV1-UL19-1057 | + | GUUCCAUGACCGGAUUGAAC | 20 | 1443 |
| HSV1-UL19-1058 | + | GCGGCAAACCGUUCCAUGAC | 20 | 1444 |
| HSV1-UL19-1059 | + | GACUAGGUCCCCGGCGUGCG | 20 | 1445 |
| HSV1-UL19-1060 | + | GGGGGCGGGGACUAGGUCCC | 20 | 1446 |
| HSV1-UL19-1061 | + | GGUGGCCGGGGGCGGGGACU | 20 | 1447 |
| HSV1-UL19-1062 | + | GAUCCGGGUGGCCGGGGGCG | 20 | 1448 |
| HSV1-UL19-1063 | + | GGAUCCGGGUGGCCGGGGGC | 20 | 1449 |
| HSV1-UL19-1064 | + | GGGAUCCGGGUGGCCGGGGG | 20 | 1450 |
| HSV1-UL19-1065 | + | GCGGGGAUCCGGGUGGCCGG | 20 | 1451 |
| HSV1-UL19-1066 | + | CGCGGGGAUCCGGGUGGCCG | 20 | 1452 |
| HSV1-UL19-1067 | + | GCGCGGGGAUCCGGGUGGCC | 20 | 1453 |
| HSV1-UL19-1068 | + | GGCGCGGGGAUCCGGGUGGC | 20 | 1454 |
| HSV1-UL19-1069 | + | GGAAGGCGCGGGGAUCCGGG | 20 | 1455 |
| HSV1-UL19-1070 | + | GCGGGAAGGCGCGGGGAUCC | 20 | 1456 |
| HSV1-UL19-1071 | + | GGCGGGAAGGCGCGGGGAUC | 20 | 1457 |
| HSV1-UL19-1072 | + | UGGCGGGGCGGGAAGGCGCG | 20 | 1458 |
| HSV1-UL19-1073 | + | CUGGCGGGGCGGGAAGGCGC | 20 | 1459 |
| HSV1-UL19-1074 | + | GCUGGCGGGGCGGGAAGGCG | 20 | 1460 |
| HSV1-UL19-1075 | + | AAACAGCUGGCGGGGCGGGA | 20 | 1461 |
| HSV1-UL19-1076 | + | AAAAAAACAGCUGGCGGGGC | 20 | 1462 |
| HSV1-UL19-1077 | + | CAAAAAAACAGCUGGCGGGG | 20 | 1463 |
| HSV1-UL19-1078 | + | CCCCAAAAAAACAGCUGGCG | 20 | 1464 |
| HSV1-UL19-1079 | + | CCCCCAAAAAAACAGCUGGC | 20 | 1465 |
| HSV1-UL19-1080 | + | UCCCCCAAAAAAACAGCUGG | 20 | 1466 |
| HSV1-UL19-1081 | + | CCUUCCCCCAAAAAAACAGC | 20 | 1467 |
| HSV1-UL19-1082 | + | GAGACAGGCGCAGCACCUGG | 20 | 1468 |
| HSV1-UL19-1083 | + | CCAGAGACAGGCGCAGCACC | 20 | 1469 |
| HSV1-UL19-1084 | + | CGAUCGCGUGUUCCAGAGAC | 20 | 1470 |
| HSV1-UL19-1085 | + | CAUCAGCGAAGGGUGGCACA | 20 | 1471 |
| HSV1-UL19-1086 | + | CAACGUUCAUCAGCGAAGGG | 20 | 1472 |
| HSV1-UL19-1087 | + | CGUCAACGUUCAUCAGCGAA | 20 | 1473 |
| HSV1-UL19-1088 | + | GCGUCAACGUUCAUCAGCGA | 20 | 1474 |
| HSV1-UL19-1089 | + | CUUCGACGGGGUCGCGGUUA | 20 | 1475 |
| HSV1-UL19-1090 | + | UGGCGGCUUCGACGGGGUCG | 20 | 1476 |
| HSV1-UL19-1091 | + | ACGGAUUGGCGGCUUCGACG | 20 | 1477 |
| HSV1-UL19-1092 | + | UACGGAUUGGCGGCUUCGAC | 20 | 1478 |
| HSV1-UL19-1093 | + | GUACGGAUUGGCGGCUUCGA | 20 | 1479 |
| HSV1-UL19-1094 | + | GUACGCCCCGUACGGAUUGG | 20 | 1480 |
| HSV1-UL19-1095 | + | CACGUACGCCCCGUACGGAU | 20 | 1481 |
| HSV1-UL19-1096 | + | GCCGCCACGUACGCCCCGUA | 20 | 1482 |
| HSV1-UL19-1097 | + | GUCUGCGGCGGGGCCGGCCG | 20 | 1483 |
| HSV1-UL19-1098 | + | UGUCUGCGGCGGGGCCGGCC | 20 | 1484 |
| HSV1-UL19-1099 | + | AUGUCUGCGGCGGGGCCGGC | 20 | 1485 |
| HSV1-UL19-1100 | + | CUGCAUGUCUGCGGCGGGGC | 20 | 1486 |
| HSV1-UL19-1101 | + | GCUGCUGCAUGUCUGCGGCG | 20 | 1487 |
| HSV1-UL19-1102 | + | AGCUGCUGCAUGUCUGCGGC | 20 | 1488 |
| HSV1-UL19-1103 | + | CAGCUGCUGCAUGUCUGCGG | 20 | 1489 |
| HSV1-UL19-1104 | + | AAACAGCUGCUGCAUGUCUG | 20 | 1490 |
| HSV1-UL19-1105 | + | GUGGGCCAGGCGCUGCCCCC | 20 | 1491 |
| HSV1-UL19-1106 | + | AUCGGACCCGCCCGUGGGCC | 20 | 1492 |
| HSV1-UL19-1107 | + | GACCCAUCGGACCCGCCCGU | 20 | 1493 |
| HSV1-UL19-1108 | + | CGACCCAUCGGACCCGCCCG | 20 | 1494 |
| HSV1-UL19-1109 | + | UCUGGCCUUCCGCGACCCAU | 20 | 1495 |
| HSV1-UL19-1110 | + | UAAACUGCUCCGGGGUCAUC | 20 | 1496 |
| HSV1-UL19-1111 | + | GGGCUGCAUAAACUGCUCCG | 20 | 1497 |
| HSV1-UL19-1112 | + | CGGGCUGCAUAAACUGCUCC | 20 | 1498 |
| HSV1-UL19-1113 | + | UCGGGCUGCAUAAACUGCUC | 20 | 1499 |
| HSV1-UL19-1114 | + | AGAGCCAGGUUGGCGUUGUC | 20 | 1500 |
| HSV1-UL19-1115 | + | AAGAGCCAGGUUGGCGUUGU | 20 | 1501 |
| HSV1-UL19-1116 | + | GUGCAGCUCAAGAGCCAGGU | 20 | 1502 |
| HSV1-UL19-1117 | + | CGGGGUGCAGCUCAAGAGCC | 20 | 1503 |
| HSV1-UL19-1118 | + | CCACAAAGAAGUCGAACGCG | 20 | 1504 |
| HSV1-UL19-1119 | + | CCCACAAAGAAGUCGAACGC | 20 | 1505 |
| HSV1-UL19-1120 | + | CCCCACAAAGAAGUCGAACG | 20 | 1506 |
| HSV1-UL19-1121 | + | CCCCCCCGGCAGCUCCACGU | 20 | 1507 |
| HSV1-UL19-1122 | + | GCCGGGGGAACGUCCCCCCC | 20 | 1508 |
| HSV1-UL19-1123 | + | AUCUCCCCCGGGCCGGCCGG | 20 | 1509 |
| HSV1-UL19-1124 | + | GAUCUCCCCCGGGCCGGCCG | 20 | 1510 |
| HSV1-UL19-1125 | + | GGAUCUCCCCCGGGCCGGCC | 20 | 1511 |
| HSV1-UL19-1126 | + | UGGAUCUCCCCCGGGCCGGC | 20 | 1512 |
| HSV1-UL19-1127 | + | GGCCUGGAUCUCCCCCGGGC | 20 | 1513 |
| HSV1-UL19-1128 | + | AGGUGGCCUGGAUCUCCCCC | 20 | 1514 |
| HSV1-UL19-1129 | + | CAGGUGGCCUGGAUCUCCCC | 20 | 1515 |
| HSV1-UL19-1130 | + | UCACCACGCGCCAGGUGGCC | 20 | 1516 |
| HSV1-UL19-1131 | + | GCCGUUCACCACGCGCCAGG | 20 | 1517 |
| HSV1-UL19-1132 | + | GUUGCCGUUCACCACGCGCC | 20 | 1518 |
| HSV1-UL19-1133 | + | GCCGCCGGACAUAGCGCCAG | 20 | 1519 |
| HSV1-UL19-1134 | + | CGCCGCCGGACAUAGCGCCA | 20 | 1520 |
| HSV1-UL19-1135 | + | ACGCCGCCGGACAUAGCGCC | 20 | 1521 |
| HSV1-UL19-1136 | + | CGGGCGUCCCGGAACGCCGC | 20 | 1522 |
| HSV1-UL19-1137 | + | GCUCCAGGCCCCGGGCGUCC | 20 | 1523 |
| HSV1-UL19-1138 | + | CACCCCCAGCUCCAGGCCCC | 20 | 1524 |
| HSV1-UL19-1139 | + | CCACCCCCAGCUCCAGGCCC | 20 | 1525 |
| HSV1-UL19-1140 | + | GGCGUCCCACCCCCAGCUCC | 20 | 1526 |
| HSV1-UL19-1141 | + | UGGUGGCGGGGGCCAUGGCG | 20 | 1527 |
| HSV1-UL19-1142 | + | GGCGAUGGUGGCGGGGGCCA | 20 | 1528 |
| HSV1-UL19-1143 | + | AACGGCGGCGAUGGUGGCGG | 20 | 1529 |
| HSV1-UL19-1144 | + | GAACGGCGGCGAUGGUGGCG | 20 | 1530 |
| HSV1-UL19-1145 | + | CGAACGGCGGCGAUGGUGGC | 20 | 1531 |
| HSV1-UL19-1146 | + | GCGAACGGCGGCGAUGGUGG | 20 | 1532 |
| HSV1-UL19-1147 | + | CCCGCGAACGGCGGCGAUGG | 20 | 1533 |
| HSV1-UL19-1148 | + | CGCCCCGCGAACGGCGGCGA | 20 | 1534 |
| HSV1-UL19-1149 | + | GUCGAACGCCCCGCGAACGG | 20 | 1535 |
| HSV1-UL19-1150 | + | GUCGUCGAACGCCCCGCGAA | 20 | 1536 |
| HSV1-UL19-1151 | + | AAAACACCGCCGGGUAGUUG | 20 | 1537 |
| HSV1-UL19-1152 | + | GCAGCAGGUAAAACACCGCC | 20 | 1538 |
| HSV1-UL19-1153 | + | UGCAGCAGGUAAAACACCGC | 20 | 1539 |
| HSV1-UL19-1154 | + | CGUGUAUGGCGGCCUGCAGC | 20 | 1540 |
| HSV1-UL19-1155 | + | GUGCUCGCUGCCGUGUAUGG | 20 | 1541 |
| HSV1-UL19-1156 | + | GACGUGCUCGCUGCCGUGUA | 20 | 1542 |
| HSV1-UL19-1157 | + | CUGGACCACGAGCCGGGCCA | 20 | 1543 |
| HSV1-UL19-1158 | + | ACUGGACCACGAGCCGGGCC | 20 | 1544 |
| HSV1-UL19-1159 | + | GAUGCACUGGACCACGAGCC | 20 | 1545 |
| HSV1-UL19-1160 | + | UGAUGCACUGGACCACGAGC | 20 | 1546 |
| HSV1-UL19-1161 | + | UCCAGUAGCUGGUGAUGCAC | 20 | 1547 |
| HSV1-UL19-1162 | + | CCGCGUGUUGUUCCAGUAGC | 20 | 1548 |
| HSV1-UL19-1163 | + | GGGAAGGUCUCCCCCGAGGU | 20 | 1549 |
| HSV1-UL19-1164 | + | CCUCGGGAAGGUCUCCCCCG | 20 | 1550 |
| HSV1-UL19-1165 | + | CGGCCAUGCACUCCUCGGGA | 20 | 1551 |
| HSV1-UL19-1166 | + | UACACGGCCAUGCACUCCUC | 20 | 1552 |
| HSV1-UL19-1167 | + | GUACACGGCCAUGCACUCCU | 20 | 1553 |
| HSV1-UL19-1168 | + | CGCCACCAGGUCCCGGUACA | 20 | 1554 |
| HSV1-UL19-1169 | + | CGACGUGCGCCACCAGGUCC | 20 | 1555 |
| HSV1-UL19-1170 | + | GGGCCUCGACGUGCGCCACC | 20 | 1556 |
| HSV1-UL19-1171 | + | GUCAUCAACCAGCUGGGCCA | 20 | 1557 |
| HSV1-UL19-1172 | + | AGUCAUCAACCAGCUGGGCC | 20 | 1558 |
| HSV1-UL19-1173 | + | GGUAAAGUCAUCAACCAGCU | 20 | 1559 |
| HSV1-UL19-1174 | + | GGGUAAAGUCAUCAACCAGC | 20 | 1560 |
| HSV1-UL19-1175 | + | GCCCAGCUCCGGGCCGGUCA | 20 | 1561 |
| HSV1-UL19-1176 | + | CGCCCAGCUCCGGGCCGGUC | 20 | 1562 |
| HSV1-UL19-1177 | + | CUGCCCGCCCAGCUCCGGGC | 20 | 1563 |
| HSV1-UL19-1178 | + | GCGCCUGCCCGCCCAGCUCC | 20 | 1564 |
| HSV1-UL19-1179 | + | UGCGCCUGCCCGCCCAGCUC | 20 | 1565 |
| HSV1-UL19-1180 | + | UCGCAUUAGGUGAUUCAGCU | 20 | 1566 |
| HSV1-UL19-1181 | + | GCAGCGCCGGGUCUCGCAUU | 20 | 1567 |
| HSV1-UL19-1182 | + | CGAGGGGUGGCAGCAGCGCC | 20 | 1568 |
| HSV1-UL19-1183 | + | ACGAGGGGUGGCAGCAGCGC | 20 | 1569 |
| HSV1-UL19-1184 | + | UCACAGUCCCACACGAGGGG | 20 | 1570 |
| HSV1-UL19-1185 | + | GCGUCACAGUCCCACACGAG | 20 | 1571 |
| HSV1-UL19-1186 | + | GGCGUCACAGUCCCACACGA | 20 | 1572 |
| HSV1-UL19-1187 | + | GGGCGUCACAGUCCCACACG | 20 | 1573 |
| HSV1-UL19-1188 | + | CAGGGCCGCGCGCCGCAUCA | 20 | 1574 |
| HSV1-UL19-1189 | + | CCAGGGCCGCGCGCCGCAUC | 20 | 1575 |
| HSV1-UL19-1190 | + | GCAGUCGCGAUGGCGGUCCA | 20 | 1576 |
| HSV1-UL19-1191 | + | GGCAGUCGCGAUGGCGGUCC | 20 | 1577 |
| HSV1-UL19-1192 | + | UAACCCGGCAGUCGCGAUGG | 20 | 1578 |
| HSV1-UL19-1193 | + | CGCUAACCCGGCAGUCGCGA | 20 | 1579 |
| HSV1-UL19-1194 | + | CGUGGCCCCCCGCGCUAACC | 20 | 1580 |
| HSV1-UL19-1195 | + | CCGCCGCGUACACGGGGUCG | 20 | 1581 |
| HSV1-UL19-1196 | + | UACAUGCCGCCGCGUACACG | 20 | 1582 |
| HSV1-UL19-1197 | + | UUACAUGCCGCCGCGUACAC | 20 | 1583 |
| HSV1-UL19-1198 | + | GUUACAUGCCGCCGCGUACA | 20 | 1584 |
| HSV1-UL19-1199 | + | GUCGUUGCGGUUGAAGUCCG | 20 | 1585 |
| HSV1-UL19-1200 | + | GCAGCAGCUGGCCGUCGUUG | 20 | 1586 |
| HSV1-UL19-1201 | + | CCUGGGUGUUGUGCAGCAGC | 20 | 1587 |
| HSV1-UL19-1202 | + | GGCGUCCGCGGCUCGGGCCU | 20 | 1588 |
| HSV1-UL19-1203 | + | CGGCGUCCGCGGCUCGGGCC | 20 | 1589 |
| HSV1-UL19-1204 | + | AUCCGCGGCGUCCGCGGCUC | 20 | 1590 |
| HSV1-UL19-1205 | + | CAUCCGCGGCGUCCGCGGCU | 20 | 1591 |
| HSV1-UL19-1206 | + | CCGGUCAUCCGCGGCGUCCG | 20 | 1592 |
| HSV1-UL19-1207 | + | CCGGUGCGGCCGGUCAUCCG | 20 | 1593 |
| HSV1-UL19-1208 | + | AGUCCGCCCCCCGGUGCGGC | 20 | 1594 |
| HSV1-UL19-1209 | + | GUCCAGUCCGCCCCCCGGUG | 20 | 1595 |
| HSV1-UL19-1210 | + | GCACGGUCCAGUCCGCCCCC | 20 | 1596 |
| HSV1-UL19-1211 | + | GUAGUAAAUCUUGUGGUGCA | 20 | 1597 |
| HSV1-UL19-1212 | + | UCACGUAGUAGUAAAUCUUG | 20 | 1598 |
| HSV1-UL19-1213 | + | CAGCGGCCCCGCGAGAAGGC | 20 | 1599 |
| HSV1-UL19-1214 | + | GCAGCGGCCCCGCGAGAAGG | 20 | 1600 |
| HSV1-UL19-1215 | + | GCAGCAGCGGCCCCGCGAGA | 20 | 1601 |
| HSV1-UL19-1216 | + | GAACCCCCGCGGUGCAGCAG | 20 | 1602 |
| HSV1-UL19-1217 | + | GCGGUCGAAGCGAACCCCCG | 20 | 1603 |
| HSV1-UL19-1218 | + | UCUGCAGGGUGGCGUAUACG | 20 | 1604 |
| HSV1-UL19-1219 | + | GACCACCAUGUUCUGCAGGG | 20 | 1605 |
| HSV1-UL19-1220 | + | CGGGACCACCAUGUUCUGCA | 20 | 1606 |
| HSV1-UL19-1221 | + | CCGGGACCACCAUGUUCUGC | 20 | 1607 |
| HSV1-UL19-1222 | + | CCUCGCCGGGGGCGAUCUCC | 20 | 1608 |
| HSV1-UL19-1223 | + | UCCUCGCCGGGGGCGAUCUC | 20 | 1609 |
| HSV1-UL19-1224 | + | GCUGGGGCACUCCUCGCCGG | 20 | 1610 |
| HSV1-UL19-1225 | + | CGCUGGGGCACUCCUCGCCG | 20 | 1611 |
| HSV1-UL19-1226 | + | UCGCUGGGGCACUCCUCGCC | 20 | 1612 |
| HSV1-UL19-1227 | + | GUCGCUGGGGCACUCCUCGC | 20 | 1613 |
| HSV1-UL19-1228 | + | GGUCCGUCACGGGGUCGCUG | 20 | 1614 |
| HSV1-UL19-1229 | + | GGGUCCGUCACGGGGUCGCU | 20 | 1615 |
| HSV1-UL19-1230 | + | GGGGUCCGUCACGGGGUCGC | 20 | 1616 |
| HSV1-UL19-1231 | + | GGUGCGCGGGGUCCGUCACG | 20 | 1617 |
| HSV1-UL19-1232 | + | GGGUGCGCGGGGUCCGUCAC | 20 | 1618 |
| HSV1-UL19-1233 | + | GGGGUGCGCGGGGUCCGUCA | 20 | 1619 |
| HSV1-UL19-1234 | + | CCGGGUGCAGGGGGUGCGCG | 20 | 1620 |
| HSV1-UL19-1235 | + | GCCGGGUGCAGGGGGUGCGC | 20 | 1621 |
| HSV1-UL19-1236 | + | GGCCGGGUGCAGGGGGUGCG | 20 | 1622 |
| HSV1-UL19-1237 | + | CCAGAUUGGCCGGGUGCAGG | 20 | 1623 |
| HSV1-UL19-1238 | + | ACCAGAUUGGCCGGGUGCAG | 20 | 1624 |
| HSV1-UL19-1239 | + | CACCAGAUUGGCCGGGUGCA | 20 | 1625 |
| HSV1-UL19-1240 | + | CCACCAGAUUGGCCGGGUGC | 20 | 1626 |
| HSV1-UL19-1241 | + | UGUUGGCCACCAGAUUGGCC | 20 | 1627 |
| HSV1-UL19-1242 | + | GUGUUGGCCACCAGAUUGGC | 20 | 1628 |
| HSV1-UL19-1243 | + | GACCGUGUUGGCCACCAGAU | 20 | 1629 |
| HSV1-UL19-1244 | + | AAACAUGGCGUUGACCGUGU | 20 | 1630 |
| HSV1-UL19-1245 | + | CACGCGCCCGUUGUGAAACA | 20 | 1631 |
| HSV1-UL19-1246 | + | ACCUGCAGCGUGAGCAUGGC | 20 | 1632 |
| HSV1-UL19-1247 | + | CACCUGCAGCGUGAGCAUGG | 20 | 1633 |
| HSV1-UL19-1248 | + | CAGCACCUGCAGCGUGAGCA | 20 | 1634 |
| HSV1-UL19-1249 | + | UGUGCGUUCGGCCAUGUUGU | 20 | 1635 |
| HSV1-UL19-1250 | + | UUGUGCGUUCGGCCAUGUUG | 20 | 1636 |
| HSV1-UL19-1251 | + | GAGCAGCGCCGUUGUGCGUU | 20 | 1637 |
| HSV1-UL19-1252 | + | GCGGUGUUGGCGCCCGCGUC | 20 | 1638 |
| HSV1-UL19-1253 | + | CGCGGUGUUGGCGCCCGCGU | 20 | 1639 |
| HSV1-UL19-1254 | + | GUUGGUGGUGGACGCGGUGU | 20 | 1640 |
| HSV1-UL19-1255 | + | GCGCAUGUUGGUGGUGGACG | 20 | 1641 |
| HSV1-UL19-1256 | + | GAAUAUGCGCAUGUUGGUGG | 20 | 1642 |
| HSV1-UL19-1257 | + | GUCGAAUAUGCGCAUGUUGG | 20 | 1643 |
| HSV1-UL19-1258 | + | CCCGUCGAAUAUGCGCAUGU | 20 | 1644 |
| HSV1-UL19-1259 | + | GGGGGCCAUCAGCAGGAUUC | 20 | 1645 |
| HSV1-UL19-1260 | + | GAUGCUGGGGGGCCAUCAGC | 20 | 1646 |
| HSV1-UL19-1261 | + | GGUAUGGUCCAGAUGCUGGG | 20 | 1647 |
| HSV1-UL19-1262 | + | UGGUAUGGUCCAGAUGCUGG | 20 | 1648 |
| HSV1-UL19-1263 | + | AUGGUAUGGUCCAGAUGCUG | 20 | 1649 |
| HSV1-UL19-1264 | + | GAUGGUAUGGUCCAGAUGCU | 20 | 1650 |
| HSV1-UL19-1265 | + | GGAUGGUAUGGUCCAGAUGC | 20 | 1651 |
| HSV1-UL19-1266 | + | AGUCGCCAUUUUGGAUGGUA | 20 | 1652 |
| HSV1-UL19-1267 | + | AAAAUAGUCGCCAUUUUGGA | 20 | 1653 |
| HSV1-UL19-1268 | + | GGUAAAAAUAGUCGCCAUUU | 20 | 1654 |
| HSV1-UL19-1269 | + | ACAGCGCGUGGACGGGGAGG | 20 | 1655 |
| HSV1-UL19-1270 | + | AACAGCGCGUGGACGGGGAG | 20 | 1656 |
| HSV1-UL19-1271 | + | GAACAGCGCGUGGACGGGGA | 20 | 1657 |
| HSV1-UL19-1272 | + | CGAACAGCGCGUGGACGGGG | 20 | 1658 |
| HSV1-UL19-1273 | + | CGGCGAACAGCGCGUGGACG | 20 | 1659 |
| HSV1-UL19-1274 | + | CCGGCGAACAGCGCGUGGAC | 20 | 1660 |
| HSV1-UL19-1275 | + | CCCGGCGAACAGCGCGUGGA | 20 | 1661 |
| HSV1-UL19-1276 | + | CGGCCCCGGCGAACAGCGCG | 20 | 1662 |
| HSV1-UL19-1277 | + | GUUCGCCACGUGGUCGGCCC | 20 | 1663 |
| HSV1-UL19-1278 | + | GGGCGCGUUCGCCACGUGGU | 20 | 1664 |
| HSV1-UL19-1279 | + | AAUUGGGCGCGUUCGCCACG | 20 | 1665 |
| HSV1-UL19-1280 | + | CGCAGGGCCGGGGGGAAAUU | 20 | 1666 |
| HSV1-UL19-1281 | + | GCGCAGGGCCGGGGGGAAAU | 20 | 1667 |
| HSV1-UL19-1282 | + | ACAGGUCGCGCAGGGCCGGG | 20 | 1668 |
| HSV1-UL19-1283 | + | GACAGGUCGCGCAGGGCCGG | 20 | 1669 |
| HSV1-UL19-1284 | + | CGACAGGUCGCGCAGGGCCG | 20 | 1670 |
| HSV1-UL19-1285 | + | GCGACAGGUCGCGCAGGGCC | 20 | 1671 |
| HSV1-UL19-1286 | + | CGCGACAGGUCGCGCAGGGC | 20 | 1672 |
| HSV1-UL19-1287 | + | CUGCCGCGACAGGUCGCGCA | 20 | 1673 |
| HSV1-UL19-1288 | + | CCUGCCGCGACAGGUCGCGC | 20 | 1674 |
| HSV1-UL19-1289 | + | CCAGGGGGACCUGCCGCGAC | 20 | 1675 |
| HSV1-UL19-1290 | + | CCAGAGCCGGGGGGACCAGG | 20 | 1676 |
| HSV1-UL19-1291 | + | CCCAGAGCCGGGGGGACCAG | 20 | 1677 |
| HSV1-UL19-1292 | + | CCCCAGAGCCGGGGGGACCA | 20 | 1678 |
| HSV1-UL19-1293 | + | CCCCCAGAGCCGGGGGGACC | 20 | 1679 |
| HSV1-UL19-1294 | + | AGUUGGCCCCCAGAGCCGGG | 20 | 1680 |
| HSV1-UL19-1295 | + | UAGUUGGCCCCCAGAGCCGG | 20 | 1681 |
| HSV1-UL19-1296 | + | GUAGUUGGCCCCCAGAGCCG | 20 | 1682 |
| HSV1-UL19-1297 | + | AGUAGUUGGCCCCCAGAGCC | 20 | 1683 |
| HSV1-UL19-1298 | + | AAGUAGUUGGCCCCCAGAGC | 20 | 1684 |
| HSV1-UL19-1299 | + | UCGGAUCGACGAAAAGUAGU | 20 | 1685 |
| HSV1-UL19-1300 | + | CGUGCUGCACGACGGGCUGU | 20 | 1686 |
| HSV1-UL19-1301 | + | UCGCGGACGUGCUGCACGAC | 20 | 1687 |
| HSV1-UL19-1302 | + | CUCGCGGACGUGCUGCACGA | 20 | 1688 |
| HSV1-UL19-1303 | + | UCUCCCCGGCCGCGCUCUCG | 20 | 1689 |
| HSV1-UL19-1304 | + | GUAGGUCAGCGCGUUCUCCC | 20 | 1690 |
| HSV1-UL19-1305 | + | GUACCCCGCCAUGAGCGCGU | 20 | 1691 |
| HSV1-UL19-1306 | + | AGCUGAUGAUGCAAGGCCAC | 20 | 1692 |
| HSV1-UL19-1307 | + | GAGCUGAUGAUGCAAGGCCA | 20 | 1693 |
| HSV1-UL19-1308 | + | CGUCUUGAGCUGAUGAUGCA | 20 | 1694 |
| HSV1-UL19-1309 | + | UGAACCCAAACCCGGGAUGG | 20 | 1695 |
| HSV1-UL19-1310 | + | CGGUGAACCCAAACCCGGGA | 20 | 1696 |
| HSV1-UL19-1311 | + | ACGACGGUGAACCCAAACCC | 20 | 1697 |
| HSV1-UL19-1312 | + | GACGACGGUGAACCCAAACC | 20 | 1698 |
| HSV1-UL19-1313 | + | AAAGCGGUCCUGUCGGACGA | 20 | 1699 |
| HSV1-UL19-1314 | + | CAGUCACAAAGCGGUCCUGU | 20 | 1700 |
| HSV1-UL19-1315 | + | GCACGUUCUCAGUCACAAAG | 20 | 1701 |
| HSV1-UL19-1316 | + | GGGCCACCUGGAGCUGGCCC | 20 | 1702 |
| HSV1-UL19-1317 | + | CGUGCCGGGCCACCUGGAGC | 20 | 1703 |
| HSV1-UL19-1318 | + | CAGUUUCGUGCCGGGCCACC | 20 | 1704 |
| HSV1-UL19-1319 | + | CCCCCCGCCAGUUUCGUGCC | 20 | 1705 |
| HSV1-UL19-1320 | + | CCCCCCCGCCAGUUUCGUGC | 20 | 1706 |
| HSV1-UL19-1321 | + | GUCCACGUUCGCGCGCGGCU | 20 | 1707 |
| HSV1-UL19-1322 | + | GGUCCACGUUCGCGCGCGGC | 20 | 1708 |
| HSV1-UL19-1323 | + | CCCAGGUCCACGUUCGCGCG | 20 | 1709 |
| HSV1-UL19-1324 | + | CGGCGGUGUAGCCCACGCCC | 20 | 1710 |
| HSV1-UL19-1325 | + | GGUUGCCGUGGCCACGACGG | 20 | 1711 |
| HSV1-UL19-1326 | + | GACGGUUGCCGUGGCCACGA | 20 | 1712 |
| HSV1-UL19-1327 | + | GGGGUUGCGGACGGUUGCCG | 20 | 1713 |
| HSV1-UL19-1328 | + | GUCGGUGACGGGGUUGCGGA | 20 | 1714 |
| HSV1-UL19-1329 | + | CCAUGUCGGUGACGGGGUUG | 20 | 1715 |
| HSV1-UL19-1330 | + | GGUUGCCCAUGUCGGUGACG | 20 | 1716 |
| HSV1-UL19-1331 | + | AGGUUGCCCAUGUCGGUGAC | 20 | 1717 |
| HSV1-UL19-1332 | + | AAGGUUGCCCAUGUCGGUGA | 20 | 1718 |
| HSV1-UL19-1333 | + | UUGGGGAAGGUUGCCCAUGU | 20 | 1719 |
| HSV1-UL19-1334 | + | CCAGGUAAAAGUUUUGGGGA | 20 | 1720 |
| HSV1-UL19-1335 | + | CGGCCCAGGUAAAAGUUUUG | 20 | 1721 |
| HSV1-UL19-1336 | + | GCGGCCCAGGUAAAAGUUUU | 20 | 1722 |
| HSV1-UL19-1337 | + | CGCGGCCCAGGUAAAAGUUU | 20 | 1723 |
| HSV1-UL19-1338 | + | GAGGGGGAGCCCCGCGGCCC | 20 | 1724 |
| HSV1-UL19-1339 | + | CCAGGAGAGGGGGAGCCCCG | 20 | 1725 |
| HSV1-UL19-1340 | + | GCCGCGUUGUCCAGGAGAGG | 20 | 1726 |
| HSV1-UL19-1341 | + | UGCCGCGUUGUCCAGGAGAG | 20 | 1727 |
| HSV1-UL19-1342 | + | CUGCCGCGUUGUCCAGGAGA | 20 | 1728 |
| HSV1-UL19-1343 | + | GCUGCCGCGUUGUCCAGGAG | 20 | 1729 |
| HSV1-UL19-1344 | + | ACACGGCUGCCGCGUUGUCC | 20 | 1730 |
| HSV1-UL19-1345 | + | GACCGCGUUCCGCAGGUACA | 20 | 1731 |
| HSV1-UL19-1346 | + | CCGCCACGACCGCGUUCCGC | 20 | 1732 |
| HSV1-UL19-1347 | + | CGGGCUGGGCCGGCCCCAGG | 20 | 1733 |
| HSV1-UL19-1348 | + | GGACGGGCUGGGCCGGCCCC | 20 | 1734 |
| HSV1-UL19-1349 | + | AACACGGGGACGGGCUGGGC | 20 | 1735 |
| HSV1-UL19-1350 | + | CCCGAACACGGGGACGGGCU | 20 | 1736 |
| HSV1-UL19-1351 | + | ACCCGAACACGGGGACGGGC | 20 | 1737 |
| HSV1-UL19-1352 | + | GCGCACCCGAACACGGGGAC | 20 | 1738 |
| HSV1-UL19-1353 | + | GGCGCACCCGAACACGGGGA | 20 | 1739 |
| HSV1-UL19-1354 | + | CCUGGGCGCACCCGAACACG | 20 | 1740 |
| HSV1-UL19-1355 | + | ACCUGGGCGCACCCGAACAC | 20 | 1741 |
| HSV1-UL19-1356 | + | CACCUGGGCGCACCCGAACA | 20 | 1742 |
| HSV1-UL19-1357 | + | CCCUGCGCGCCGCGGCACCU | 20 | 1743 |
| HSV1-UL19-1358 | + | UCCCUGCGCGCCGCGGCACC | 20 | 1744 |
| HSV1-UL19-1359 | + | UGGUCCAUCCCUGCGCGCCG | 20 | 1745 |
| HSV1-UL19-1360 | + | CACACACGGCGUCCUGGCCG | 20 | 1746 |
| HSV1-UL19-1361 | + | UAAACUCACACACGGCGUCC | 20 | 1747 |
| HSV1-UL19-1362 | + | GGUGGCGAUAAACUCACACA | 20 | 1748 |
| HSV1-UL19-1363 | + | GACGUCGGUCGACACGGGGG | 20 | 1749 |
| HSV1-UL19-1364 | + | GUUGACGUCGGUCGACACGG | 20 | 1750 |
| HSV1-UL19-1365 | + | AGUUGACGUCGGUCGACACG | 20 | 1751 |
| HSV1-UL19-1366 | + | UAGUUGACGUCGGUCGACAC | 20 | 1752 |
| HSV1-UL19-1367 | + | GUAGUUGACGUCGGUCGACA | 20 | 1753 |
| HSV1-UL19-1368 | + | CCGGCGGAAGUAGUUGACGU | 20 | 1754 |
| HSV1-UL19-1369 | + | CCCGGGGGUUGCAGGGCCGG | 20 | 1755 |
| HSV1-UL19-1370 | + | GUCCCCGGGGGUUGCAGGGC | 20 | 1756 |
| HSV1-UL19-1371 | + | GCGCGUCCCCGGGGGUUGCA | 20 | 1757 |
| HSV1-UL19-1372 | + | GGCGCGUCCCCGGGGGUUGC | 20 | 1758 |
| HSV1-UL19-1373 | + | CCCCGGCGGCGCGUCCCCGG | 20 | 1759 |
| HSV1-UL19-1374 | + | CCCCCGGCGGCGCGUCCCCG | 20 | 1760 |
| HSV1-UL19-1375 | + | GCCCCCGGCGGCGCGUCCCC | 20 | 1761 |
| HSV1-UL19-1376 | + | CGCCCCCGGCGGCGCGUCCC | 20 | 1762 |
| HSV1-UL19-1377 | + | CCCCGCGUAAACGCCCCCGG | 20 | 1763 |
| HSV1-UL19-1378 | + | GUCCCCCGCGUAAACGCCCC | 20 | 1764 |
| HSV1-UL19-1379 | + | GCCGUGGUCGUACAUGAGGG | 20 | 1765 |
| HSV1-UL19-1380 | + | CUGGCCGUGGUCGUACAUGA | 20 | 1766 |
| HSV1-UL19-1381 | + | UCUGGCCGUGGUCGUACAUG | 20 | 1767 |
| HSV1-UL19-1382 | + | GGGACGGGUCGCUCUGGCCG | 20 | 1768 |
| HSV1-UL19-1383 | + | AGGCCCGGGACGGGUCGCUC | 20 | 1769 |
| HSV1-UL19-1384 | + | UGGCCGCGAAGGCCCGGGAC | 20 | 1770 |
| HSV1-UL19-1385 | + | GUGGCCGCGAAGGCCCGGGA | 20 | 1771 |
| HSV1-UL19-1386 | + | GGCCGUGGCCGCGAAGGCCC | 20 | 1772 |
| HSV1-UL19-1387 | + | UGGCCGUGGCCGCGAAGGCC | 20 | 1773 |
| HSV1-UL19-1388 | + | CGGGUUGGCCGUGGCCGCGA | 20 | 1774 |
| HSV1-UL19-1389 | + | CGACGCCCACGGGUUGGCCG | 20 | 1775 |
| HSV1-UL19-1390 | + | UCGCUGCGACGCCCACGGGU | 20 | 1776 |
| HSV1-UL19-1391 | + | AAAAUCGCUGCGACGCCCAC | 20 | 1777 |
| HSV1-UL19-1392 | + | GAAAAUCGCUGCGACGCCCA | 20 | 1778 |
| HSV1-UL19-1393 | + | GGUAGGCCCCGUUAUAGAGC | 20 | 1779 |
| HSV1-UL19-1394 | + | CGAGGCCCCGUUGAGGUGGU | 20 | 1780 |
| HSV1-UL19-1395 | + | CCGGCGAGGCCCCGUUGAGG | 20 | 1781 |
| HSV1-UL19-1396 | + | GCACCGGCGAGGCCCCGUUG | 20 | 1782 |
| HSV1-UL19-1397 | + | GCAGGGGCUGAGCACCGGCG | 20 | 1783 |
| HSV1-UL19-1398 | + | UUAAAGCAGGGGCUGAGCAC | 20 | 1784 |
| HSV1-UL19-1399 | + | ACGUAAAGAACUUAAAGCAG | 20 | 1785 |
| HSV1-UL19-1400 | + | GACGUAAAGAACUUAAAGCA | 20 | 1786 |
| HSV1-UL19-1401 | + | CGACGUAAAGAACUUAAAGC | 20 | 1787 |
| HSV1-UL19-1402 | + | GCGAUGUUUGGCGGCGAUGU | 20 | 1788 |
| HSV1-UL19-1403 | + | CUCCAGGCAGCGAUGUUUGG | 20 | 1789 |
| HSV1-UL19-1404 | + | GCGCUCCAGGCAGCGAUGUU | 20 | 1790 |
| HSV1-UL19-1405 | + | UCUCCACGAUCAGGCGCUCC | 20 | 1791 |
| HSV1-UL19-1406 | + | CCGAACCCGUCUCCACGAUC | 20 | 1792 |
| HSV1-UL19-1407 | + | GUCGCUGGCGGCGGUGGCCG | 20 | 1793 |
| HSV1-UL19-1408 | + | CUGUACGUCGCUGGCGGCGG | 20 | 1794 |
| HSV1-UL19-1409 | + | AAACUGUACGUCGCUGGCGG | 20 | 1795 |
| HSV1-UL19-1410 | + | CUUAAACUGUACGUCGCUGG | 20 | 1796 |
| HSV1-UL19-1411 | + | GCGCUUAAACUGUACGUCGC | 20 | 1797 |
| HSV1-UL19-1412 | + | CGAGUUCGCGGCACCCCGGG | 20 | 1798 |
| HSV1-UL19-1413 | + | ACGAGUUCGCGGCACCCCGG | 20 | 1799 |
| HSV1-UL19-1414 | + | CACGAGUUCGCGGCACCCCG | 20 | 1800 |
| HSV1-UL19-1415 | + | CCACGAGUUCGCGGCACCCC | 20 | 1801 |
| HSV1-UL19-1416 | + | UCCACGAGUUCGCGGCACCC | 20 | 1802 |
| HSV1-UL19-1417 | + | ACGGGUCCUCCACGAGUUCG | 20 | 1803 |
| HSV1-UL19-1418 | + | CCUCCUGAAACAGGCCACAC | 20 | 1804 |
| HSV1-UL19-1419 | + | GCCUCCUGAAACAGGCCACA | 20 | 1805 |
| HSV1-UL19-1420 | + | GCGGGUAGGCCUCCUGAAAC | 20 | 1806 |
| HSV1-UL19-1421 | + | GCUGGCGCAGGUGAGCGGGU | 20 | 1807 |
| HSV1-UL19-1422 | + | GGUCGCUGGCGCAGGUGAGC | 20 | 1808 |
| HSV1-UL19-1423 | + | GGGUCGCUGGCGCAGGUGAG | 20 | 1809 |
| HSV1-UL19-1424 | + | GAGGGCGGGGUCGCUGGCGC | 20 | 1810 |
| HSV1-UL19-1425 | + | GCGGAGGAGGGCGGGGUCGC | 20 | 1811 |
| HSV1-UL19-1426 | + | GGGCACUGCGGAGGAGGGCG | 20 | 1812 |
| HSV1-UL19-1427 | + | CGGGCACUGCGGAGGAGGGC | 20 | 1813 |
| HSV1-UL19-1428 | + | GCGGGCACUGCGGAGGAGGG | 20 | 1814 |
| HSV1-UL19-1429 | + | GUUGCGGGCACUGCGGAGGA | 20 | 1815 |
| HSV1-UL19-1430 | + | CGUUGCGGGCACUGCGGAGG | 20 | 1816 |
| HSV1-UL19-1431 | + | CCCCGUUGCGGGCACUGCGG | 20 | 1817 |
| HSV1-UL19-1432 | + | CUUCCCCGUUGCGGGCACUG | 20 | 1818 |
| HSV1-UL19-1433 | + | CGCGUGGGCUUCCCCGUUGC | 20 | 1819 |
| HSV1-UL19-1434 | + | GCGCGUGGGCUUCCCCGUUG | 20 | 1820 |
| HSV1-UL19-1435 | + | GAAGUGGGUCUCCCGCGCGU | 20 | 1821 |
| HSV1-UL19-1436 | + | CGAAGUGGGUCUCCCGCGCG | 20 | 1822 |
| HSV1-UL19-1437 | + | GACGAGAUACUGCGCGAAGU | 20 | 1823 |
| HSV1-UL19-1438 | + | AGACGAGAUACUGCGCGAAG | 20 | 1824 |
| HSV1-UL19-1439 | + | CAGAGCCAGUCCCUUGAGCG | 20 | 1825 |
| HSV1-UL19-1440 | + | ACAGAGCCAGUCCCUUGAGC | 20 | 1826 |
| HSV1-UL19-1441 | + | UACAGAGCCAGUCCCUUGAG | 20 | 1827 |
| HSV1-UL19-1442 | + | ACGCCCCGAGGACGGCC | 17 | 1828 |
| HSV1-UL19-1443 | + | CUCAAACGCCCCGAGGA | 17 | 1829 |
| HSV1-UL19-1444 | + | CGCGCUCAAACGCCCCG | 17 | 1830 |
| HSV1-UL19-1445 | + | CACGUGCAGCAUCUGGU | 17 | 1831 |
| HSV1-UL19-1446 | + | GCAGCACGUGCAGCAUC | 17 | 1832 |
| HSV1-UL19-1447 | + | AACAACAAAGCCAGGGG | 17 | 1833 |
| HSV1-UL19-1448 | + | GGCAACAACAAAGCCAG | 17 | 1834 |
| HSV1-UL19-1449 | + | CGGCAACAACAAAGCCA | 17 | 1835 |
| HSV1-UL19-1450 | + | UCGGCAACAACAAAGCC | 17 | 1836 |
| HSV1-UL19-1451 | + | UCCAGGUAUCGUUGCAU | 17 | 1837 |
| HSV1-UL19-1452 | + | CCAGGCGGCCGUUGUCC | 17 | 1838 |
| HSV1-UL19-1453 | + | CCACCCUGGUGGCCAGG | 17 | 1839 |
| HSV1-UL19-1454 | + | GGGCCACCCUGGUGGCC | 17 | 1840 |
| HSV1-UL19-1455 | + | CGCCCGGGCCACCCUGG | 17 | 1841 |
| HSV1-UL19-1456 | + | GGUCGCCCGGGCCACCC | 17 | 1842 |
| HSV1-UL19-1457 | + | GGCGACCAGGGUCGCCC | 17 | 1843 |
| HSV1-UL19-1458 | + | CGGCGACCAGGGUCGCC | 17 | 1844 |
| HSV1-UL19-1459 | + | CUUUAGCUCGGCGACCA | 17 | 1845 |
| HSV1-UL19-1460 | + | GCUUUAGCUCGGCGACC | 17 | 1846 |
| HSV1-UL19-1461 | + | GAAGCUUCGCUUUAGCU | 17 | 1847 |
| HSV1-UL19-1462 | + | GGUGGCCCGCCUUGCCC | 17 | 1848 |
| HSV1-UL19-1463 | + | CGACGGCCUCCCGGCGG | 17 | 1849 |
| HSV1-UL19-1464 | + | CCUCGACGGCCUCCCGG | 17 | 1850 |
| HSV1-UL19-1465 | + | AGGCCUCGACGGCCUCC | 17 | 1851 |
| HSV1-UL19-1466 | + | CACGAGCCAGGCCUCGA | 17 | 1852 |
| HSV1-UL19-1467 | + | GGUGAGGUCCACGAGCC | 17 | 1853 |
| HSV1-UL19-1468 | + | GCUGCGUGGCCGUGGUG | 17 | 1854 |
| HSV1-UL19-1469 | + | GGAGGGCUGCGUGGCCG | 17 | 1855 |
| HSV1-UL19-1470 | + | GGCCACGGAGGGCUGCG | 17 | 1856 |
| HSV1-UL19-1471 | + | CGGGGCACGGCCACGGA | 17 | 1857 |
| HSV1-UL19-1472 | + | ACGGGGCACGGCCACGG | 17 | 1858 |
| HSV1-UL19-1473 | + | CAGACGGGGCACGGCCA | 17 | 1859 |
| HSV1-UL19-1474 | + | AUGCGUCAGACGGGGCA | 17 | 1860 |
| HSV1-UL19-1475 | + | UCGGCAUGCGUCAGACG | 17 | 1861 |
| HSV1-UL19-1476 | + | GUCGGCAUGCGUCAGAC | 17 | 1862 |
| HSV1-UL19-1477 | + | UGUCGGCAUGCGUCAGA | 17 | 1863 |
| HSV1-UL19-1478 | + | CGGCCGCCCGCGCGUGU | 17 | 1864 |
| HSV1-UL19-1479 | + | ACGAGCACCCCGUCGAC | 17 | 1865 |
| HSV1-UL19-1480 | + | CUGUUUGAUCGGGGCGG | 17 | 1866 |
| HSV1-UL19-1481 | + | GCGCUGUUUGAUCGGGG | 17 | 1867 |
| HSV1-UL19-1482 | + | GAGGCGCUGUUUGAUCG | 17 | 1868 |
| HSV1-UL19-1483 | + | GGAGGCGCUGUUUGAUC | 17 | 1869 |
| HSV1-UL19-1484 | + | AGGAGGCGCUGUUUGAU | 17 | 1870 |
| HSV1-UL19-1485 | + | UCAGGAAGGACUGCAGG | 17 | 1871 |
| HSV1-UL19-1486 | + | CUUUCAGGAAGGACUGC | 17 | 1872 |
| HSV1-UL19-1487 | + | GUCCUCCACUUUCAGGA | 17 | 1873 |
| HSV1-UL19-1488 | + | CGGUGUCCUCCACUUUC | 17 | 1874 |
| HSV1-UL19-1489 | + | CACCGGCACGUCGGCUU | 17 | 1875 |
| HSV1-UL19-1490 | + | GUACGUCACCGGCACGU | 17 | 1876 |
| HSV1-UL19-1491 | + | AUCUCGCCGUACGUCAC | 17 | 1877 |
| HSV1-UL19-1492 | + | CCAGGUUGGCCCCGUUC | 17 | 1878 |
| HSV1-UL19-1493 | + | GAGCGCCGUGACCAGGU | 17 | 1879 |
| HSV1-UL19-1494 | + | UCACGAGCGCCGUGACC | 17 | 1880 |
| HSV1-UL19-1495 | + | GUCGUCCAGACUUCGCA | 17 | 1881 |
| HSV1-UL19-1496 | + | GCAUCUCCAGCAGGUGG | 17 | 1882 |
| HSV1-UL19-1497 | + | CCUGCAUCUCCAGCAGG | 17 | 1883 |
| HSV1-UL19-1498 | + | CCUCCUGCAUCUCCAGC | 17 | 1884 |
| HSV1-UL19-1499 | + | CCAGCGUCUGCCGGUUC | 17 | 1885 |
| HSV1-UL19-1500 | + | GCUCGUCCAGCGUCUGC | 17 | 1886 |
| HSV1-UL19-1501 | + | CACGCGCGUCGUCUGGG | 17 | 1887 |
| HSV1-UL19-1502 | + | GCACGCGCGUCGUCUGG | 17 | 1888 |
| HSV1-UL19-1503 | + | CGCACGCGCGUCGUCUG | 17 | 1889 |
| HSV1-UL19-1504 | + | GCGCACGCGCGUCGUCU | 17 | 1890 |
| HSV1-UL19-1505 | + | CGCGCACGCGCGUCGUC | 17 | 1891 |
| HSV1-UL19-1506 | + | GACCAGCUUCUCGCCGA | 17 | 1892 |
| HSV1-UL19-1507 | + | GUAGAUGCGCUUCUCCA | 17 | 1893 |
| HSV1-UL19-1508 | + | CGUAGAUGCGCUUCUCC | 17 | 1894 |
| HSV1-UL19-1509 | + | GUAGGGAACGUUGGUGG | 17 | 1895 |
| HSV1-UL19-1510 | + | GGGGUAGGGAACGUUGG | 17 | 1896 |
| HSV1-UL19-1511 | + | CAGGGGGUAGGGAACGU | 17 | 1897 |
| HSV1-UL19-1512 | + | GCGCCCACCAGGGGGUA | 17 | 1898 |
| HSV1-UL19-1513 | + | GGCGCCCACCAGGGGGU | 17 | 1899 |
| HSV1-UL19-1514 | + | CCAUGGCGCCCACCAGG | 17 | 1900 |
| HSV1-UL19-1515 | + | UCCAUGGCGCCCACCAG | 17 | 1901 |
| HSV1-UL19-1516 | + | GUCCAUGGCGCCCACCA | 17 | 1902 |
| HSV1-UL19-1517 | + | GGUCCAUGGCGCCCACC | 17 | 1903 |
| HSV1-UL19-1518 | + | GACGAACGUCAGGUCCA | 17 | 1904 |
| HSV1-UL19-1519 | + | GGGGCAGGACGAACGUC | 17 | 1905 |
| HSV1-UL19-1520 | + | UGAACAGGCCCAGGGGC | 17 | 1906 |
| HSV1-UL19-1521 | + | GGAUUGAACAGGCCCAG | 17 | 1907 |
| HSV1-UL19-1522 | + | CGGAUUGAACAGGCCCA | 17 | 1908 |
| HSV1-UL19-1523 | + | CCGGAUUGAACAGGCCC | 17 | 1909 |
| HSV1-UL19-1524 | + | CCAUGACCGGAUUGAAC | 17 | 1910 |
| HSV1-UL19-1525 | + | GCAAACCGUUCCAUGAC | 17 | 1911 |
| HSV1-UL19-1526 | + | UAGGUCCCCGGCGUGCG | 17 | 1912 |
| HSV1-UL19-1527 | + | GGCGGGGACUAGGUCCC | 17 | 1913 |
| HSV1-UL19-1528 | + | GGCCGGGGGCGGGGACU | 17 | 1914 |
| HSV1-UL19-1529 | + | CCGGGUGGCCGGGGGCG | 17 | 1915 |
| HSV1-UL19-1530 | + | UCCGGGUGGCCGGGGGC | 17 | 1916 |
| HSV1-UL19-1531 | + | AUCCGGGUGGCCGGGGG | 17 | 1917 |
| HSV1-UL19-1532 | + | GGGAUCCGGGUGGCCGG | 17 | 1918 |
| HSV1-UL19-1533 | + | GGGGAUCCGGGUGGCCG | 17 | 1919 |
| HSV1-UL19-1534 | + | CGGGGAUCCGGGUGGCC | 17 | 1920 |
| HSV1-UL19-1535 | + | GCGGGGAUCCGGGUGGC | 17 | 1921 |
| HSV1-UL19-1536 | + | AGGCGCGGGGAUCCGGG | 17 | 1922 |
| HSV1-UL19-1537 | + | GGAAGGCGCGGGGAUCC | 17 | 1923 |
| HSV1-UL19-1538 | + | GGGAAGGCGCGGGGAUC | 17 | 1924 |
| HSV1-UL19-1539 | + | CGGGGCGGGAAGGCGCG | 17 | 1925 |
| HSV1-UL19-1540 | + | GCGGGGCGGGAAGGCGC | 17 | 1926 |
| HSV1-UL19-1541 | + | GGCGGGGCGGGAAGGCG | 17 | 1927 |
| HSV1-UL19-1542 | + | CAGCUGGCGGGGCGGGA | 17 | 1928 |
| HSV1-UL19-1543 | + | AAAACAGCUGGCGGGGC | 17 | 1929 |
| HSV1-UL19-1544 | + | AAAAACAGCUGGCGGGG | 17 | 1930 |
| HSV1-UL19-1545 | + | CAAAAAAACAGCUGGCG | 17 | 1931 |
| HSV1-UL19-1546 | + | CCAAAAAAACAGCUGGC | 17 | 1932 |
| HSV1-UL19-1547 | + | CCCAAAAAAACAGCUGG | 17 | 1933 |
| HSV1-UL19-1548 | + | UCCCCCAAAAAAACAGC | 17 | 1934 |
| HSV1-UL19-1549 | + | ACAGGCGCAGCACCUGG | 17 | 1935 |
| HSV1-UL19-1550 | + | GAGACAGGCGCAGCACC | 17 | 1936 |
| HSV1-UL19-1551 | + | UCGCGUGUUCCAGAGAC | 17 | 1937 |
| HSV1-UL19-1552 | + | CAGCGAAGGGUGGCACA | 17 | 1938 |
| HSV1-UL19-1553 | + | CGUUCAUCAGCGAAGGG | 17 | 1939 |
| HSV1-UL19-1554 | + | CAACGUUCAUCAGCGAA | 17 | 1940 |
| HSV1-UL19-1555 | + | UCAACGUUCAUCAGCGA | 17 | 1941 |
| HSV1-UL19-1556 | + | CGACGGGGUCGCGGUUA | 17 | 1942 |
| HSV1-UL19-1557 | + | CGGCUUCGACGGGGUCG | 17 | 1943 |
| HSV1-UL19-1558 | + | GAUUGGCGGCUUCGACG | 17 | 1944 |
| HSV1-UL19-1559 | + | GGAUUGGCGGCUUCGAC | 17 | 1945 |
| HSV1-UL19-1560 | + | CGGAUUGGCGGCUUCGA | 17 | 1946 |
| HSV1-UL19-1561 | + | CGCCCCGUACGGAUUGG | 17 | 1947 |
| HSV1-UL19-1562 | + | GUACGCCCCGUACGGAU | 17 | 1948 |
| HSV1-UL19-1563 | + | GCCACGUACGCCCCGUA | 17 | 1949 |
| HSV1-UL19-1564 | + | UGCGGCGGGGCCGGCCG | 17 | 1950 |
| HSV1-UL19-1565 | + | CUGCGGCGGGGCCGGCC | 17 | 1951 |
| HSV1-UL19-1566 | + | UCUGCGGCGGGGCCGGC | 17 | 1952 |
| HSV1-UL19-1567 | + | CAUGUCUGCGGCGGGGC | 17 | 1953 |
| HSV1-UL19-1568 | + | GCUGCAUGUCUGCGGCG | 17 | 1954 |
| HSV1-UL19-1569 | + | UGCUGCAUGUCUGCGGC | 17 | 1955 |
| HSV1-UL19-1570 | + | CUGCUGCAUGUCUGCGG | 17 | 1956 |
| HSV1-UL19-1571 | + | CAGCUGCUGCAUGUCUG | 17 | 1957 |
| HSV1-UL19-1572 | + | GGCCAGGCGCUGCCCCC | 17 | 1958 |
| HSV1-UL19-1573 | + | GGACCCGCCCGUGGGCC | 17 | 1959 |
| HSV1-UL19-1574 | + | CCAUCGGACCCGCCCGU | 17 | 1960 |
| HSV1-UL19-1575 | + | CCCAUCGGACCCGCCCG | 17 | 1961 |
| HSV1-UL19-1576 | + | GGCCUUCCGCGACCCAU | 17 | 1962 |
| HSV1-UL19-1577 | + | ACUGCUCCGGGGUCAUC | 17 | 1963 |
| HSV1-UL19-1578 | + | CUGCAUAAACUGCUCCG | 17 | 1964 |
| HSV1-UL19-1579 | + | GCUGCAUAAACUGCUCC | 17 | 1965 |
| HSV1-UL19-1580 | + | GGCUGCAUAAACUGCUC | 17 | 1966 |
| HSV1-UL19-1581 | + | GCCAGGUUGGCGUUGUC | 17 | 1967 |
| HSV1-UL19-1582 | + | AGCCAGGUUGGCGUUGU | 17 | 1968 |
| HSV1-UL19-1583 | + | CAGCUCAAGAGCCAGGU | 17 | 1969 |
| HSV1-UL19-1584 | + | GGUGCAGCUCAAGAGCC | 17 | 1970 |
| HSV1-UL19-1585 | + | CAAAGAAGUCGAACGCG | 17 | 1971 |
| HSV1-UL19-1586 | + | ACAAAGAAGUCGAACGC | 17 | 1972 |
| HSV1-UL19-1587 | + | CACAAAGAAGUCGAACG | 17 | 1973 |
| HSV1-UL19-1588 | + | CCCCGGCAGCUCCACGU | 17 | 1974 |
| HSV1-UL19-1589 | + | GGGGGAACGUCCCCCCC | 17 | 1975 |
| HSV1-UL19-1590 | + | UCCCCCGGGCCGGCCGG | 17 | 1976 |
| HSV1-UL19-1591 | + | CUCCCCCGGGCCGGCCG | 17 | 1977 |
| HSV1-UL19-1592 | + | UCUCCCCCGGGCCGGCC | 17 | 1978 |
| HSV1-UL19-1593 | + | AUCUCCCCCGGGCCGGC | 17 | 1979 |
| HSV1-UL19-1594 | + | CUGGAUCUCCCCCGGGC | 17 | 1980 |
| HSV1-UL19-1595 | + | UGGCCUGGAUCUCCCCC | 17 | 1981 |
| HSV1-UL19-1596 | + | GUGGCCUGGAUCUCCCC | 17 | 1982 |
| HSV1-UL19-1597 | + | CCACGCGCCAGGUGGCC | 17 | 1983 |
| HSV1-UL19-1598 | + | GUUCACCACGCGCCAGG | 17 | 1984 |
| HSV1-UL19-1599 | + | GCCGUUCACCACGCGCC | 17 | 1985 |
| HSV1-UL19-1600 | + | GCCGGACAUAGCGCCAG | 17 | 1986 |
| HSV1-UL19-1601 | + | CGCCGGACAUAGCGCCA | 17 | 1987 |
| HSV1-UL19-1602 | + | CCGCCGGACAUAGCGCC | 17 | 1988 |
| HSV1-UL19-1603 | + | GCGUCCCGGAACGCCGC | 17 | 1989 |
| HSV1-UL19-1604 | + | CCAGGCCCCGGGCGUCC | 17 | 1990 |
| HSV1-UL19-1605 | + | CCCCAGCUCCAGGCCCC | 17 | 1991 |
| HSV1-UL19-1606 | + | CCCCCAGCUCCAGGCCC | 17 | 1992 |
| HSV1-UL19-1607 | + | GUCCCACCCCCAGCUCC | 17 | 1993 |
| HSV1-UL19-1608 | + | UGGCGGGGGCCAUGGCG | 17 | 1994 |
| HSV1-UL19-1609 | + | GAUGGUGGCGGGGGCCA | 17 | 1995 |
| HSV1-UL19-1610 | + | GGCGGCGAUGGUGGCGG | 17 | 1996 |
| HSV1-UL19-1611 | + | CGGCGGCGAUGGUGGCG | 17 | 1997 |
| HSV1-UL19-1612 | + | ACGGCGGCGAUGGUGGC | 17 | 1998 |
| HSV1-UL19-1613 | + | AACGGCGGCGAUGGUGG | 17 | 1999 |
| HSV1-UL19-1614 | + | GCGAACGGCGGCGAUGG | 17 | 2000 |
| HSV1-UL19-1615 | + | CCCGCGAACGGCGGCGA | 17 | 2001 |
| HSV1-UL19-1616 | + | GAACGCCCCGCGAACGG | 17 | 2002 |
| HSV1-UL19-1617 | + | GUCGAACGCCCCGCGAA | 17 | 2003 |
| HSV1-UL19-1618 | + | ACACCGCCGGGUAGUUG | 17 | 2004 |
| HSV1-UL19-1619 | + | GCAGGUAAAACACCGCC | 17 | 2005 |
| HSV1-UL19-1620 | + | AGCAGGUAAAACACCGC | 17 | 2006 |
| HSV1-UL19-1621 | + | GUAUGGCGGCCUGCAGC | 17 | 2007 |
| HSV1-UL19-1622 | + | CUCGCUGCCGUGUAUGG | 17 | 2008 |
| HSV1-UL19-1623 | + | GUGCUCGCUGCCGUGUA | 17 | 2009 |
| HSV1-UL19-1624 | + | GACCACGAGCCGGGCCA | 17 | 2010 |
| HSV1-UL19-1625 | + | GGACCACGAGCCGGGCC | 17 | 2011 |
| HSV1-UL19-1626 | + | GCACUGGACCACGAGCC | 17 | 2012 |
| HSV1-UL19-1627 | + | UGCACUGGACCACGAGC | 17 | 2013 |
| HSV1-UL19-1628 | + | AGUAGCUGGUGAUGCAC | 17 | 2014 |
| HSV1-UL19-1629 | + | CGUGUUGUUCCAGUAGC | 17 | 2015 |
| HSV1-UL19-1630 | + | AAGGUCUCCCCCGAGGU | 17 | 2016 |
| HSV1-UL19-1631 | + | CGGGAAGGUCUCCCCCG | 17 | 2017 |
| HSV1-UL19-1632 | + | CCAUGCACUCCUCGGGA | 17 | 2018 |
| HSV1-UL19-1633 | + | ACGGCCAUGCACUCCUC | 17 | 2019 |
| HSV1-UL19-1634 | + | CACGGCCAUGCACUCCU | 17 | 2020 |
| HSV1-UL19-1635 | + | CACCAGGUCCCGGUACA | 17 | 2021 |
| HSV1-UL19-1636 | + | CGUGCGCCACCAGGUCC | 17 | 2022 |
| HSV1-UL19-1637 | + | CCUCGACGUGCGCCACC | 17 | 2023 |
| HSV1-UL19-1638 | + | AUCAACCAGCUGGGCCA | 17 | 2024 |
| HSV1-UL19-1639 | + | CAUCAACCAGCUGGGCC | 17 | 2025 |
| HSV1-UL19-1640 | + | AAAGUCAUCAACCAGCU | 17 | 2026 |
| HSV1-UL19-1641 | + | UAAAGUCAUCAACCAGC | 17 | 2027 |
| HSV1-UL19-1642 | + | CAGCUCCGGGCCGGUCA | 17 | 2028 |
| HSV1-UL19-1643 | + | CCAGCUCCGGGCCGGUC | 17 | 2029 |
| HSV1-UL19-1644 | + | CCCGCCCAGCUCCGGGC | 17 | 2030 |
| HSV1-UL19-1645 | + | CCUGCCCGCCCAGCUCC | 17 | 2031 |
| HSV1-UL19-1646 | + | GCCUGCCCGCCCAGCUC | 17 | 2032 |
| HSV1-UL19-1647 | + | CAUUAGGUGAUUCAGCU | 17 | 2033 |
| HSV1-UL19-1648 | + | GCGCCGGGUCUCGCAUU | 17 | 2034 |
| HSV1-UL19-1649 | + | GGGGUGGCAGCAGCGCC | 17 | 2035 |
| HSV1-UL19-1650 | + | AGGGGUGGCAGCAGCGC | 17 | 2036 |
| HSV1-UL19-1651 | + | CAGUCCCACACGAGGGG | 17 | 2037 |
| HSV1-UL19-1652 | + | UCACAGUCCCACACGAG | 17 | 2038 |
| HSV1-UL19-1653 | + | GUCACAGUCCCACACGA | 17 | 2039 |
| HSV1-UL19-1654 | + | CGUCACAGUCCCACACG | 17 | 2040 |
| HSV1-UL19-1655 | + | GGCCGCGCGCCGCAUCA | 17 | 2041 |
| HSV1-UL19-1656 | + | GGGCCGCGCGCCGCAUC | 17 | 2042 |
| HSV1-UL19-1657 | + | GUCGCGAUGGCGGUCCA | 17 | 2043 |
| HSV1-UL19-1658 | + | AGUCGCGAUGGCGGUCC | 17 | 2044 |
| HSV1-UL19-1659 | + | CCCGGCAGUCGCGAUGG | 17 | 2045 |
| HSV1-UL19-1660 | + | UAACCCGGCAGUCGCGA | 17 | 2046 |
| HSV1-UL19-1661 | + | GGCCCCCCGCGCUAACC | 17 | 2047 |
| HSV1-UL19-1662 | + | CCGCGUACACGGGGUCG | 17 | 2048 |
| HSV1-UL19-1663 | + | AUGCCGCCGCGUACACG | 17 | 2049 |
| HSV1-UL19-1664 | + | CAUGCCGCCGCGUACAC | 17 | 2050 |
| HSV1-UL19-1665 | + | ACAUGCCGCCGCGUACA | 17 | 2051 |
| HSV1-UL19-1666 | + | GUUGCGGUUGAAGUCCG | 17 | 2052 |
| HSV1-UL19-1667 | + | GCAGCUGGCCGUCGUUG | 17 | 2053 |
| HSV1-UL19-1668 | + | GGGUGUUGUGCAGCAGC | 17 | 2054 |
| HSV1-UL19-1669 | + | GUCCGCGGCUCGGGCCU | 17 | 2055 |
| HSV1-UL19-1670 | + | CGUCCGCGGCUCGGGCC | 17 | 2056 |
| HSV1-UL19-1671 | + | CGCGGCGUCCGCGGCUC | 17 | 2057 |
| HSV1-UL19-1672 | + | CCGCGGCGUCCGCGGCU | 17 | 2058 |
| HSV1-UL19-1673 | + | GUCAUCCGCGGCGUCCG | 17 | 2059 |
| HSV1-UL19-1674 | + | GUGCGGCCGGUCAUCCG | 17 | 2060 |
| HSV1-UL19-1675 | + | CCGCCCCCCGGUGCGGC | 17 | 2061 |
| HSV1-UL19-1676 | + | CAGUCCGCCCCCCGGUG | 17 | 2062 |
| HSV1-UL19-1677 | + | CGGUCCAGUCCGCCCCC | 17 | 2063 |
| HSV1-UL19-1678 | + | GUAAAUCUUGUGGUGCA | 17 | 2064 |
| HSV1-UL19-1679 | + | CGUAGUAGUAAAUCUUG | 17 | 2065 |
| HSV1-UL19-1680 | + | CGGCCCCGCGAGAAGGC | 17 | 2066 |
| HSV1-UL19-1681 | + | GCGGCCCCGCGAGAAGG | 17 | 2067 |
| HSV1-UL19-1682 | + | GCAGCGGCCCCGCGAGA | 17 | 2068 |
| HSV1-UL19-1683 | + | CCCCCGCGGUGCAGCAG | 17 | 2069 |
| HSV1-UL19-1684 | + | GUCGAAGCGAACCCCCG | 17 | 2070 |
| HSV1-UL19-1685 | + | GCAGGGUGGCGUAUACG | 17 | 2071 |
| HSV1-UL19-1686 | + | CACCAUGUUCUGCAGGG | 17 | 2072 |
| HSV1-UL19-1687 | + | GACCACCAUGUUCUGCA | 17 | 2073 |
| HSV1-UL19-1688 | + | GGACCACCAUGUUCUGC | 17 | 2074 |
| HSV1-UL19-1689 | + | CGCCGGGGGCGAUCUCC | 17 | 2075 |
| HSV1-UL19-1690 | + | UCGCCGGGGGCGAUCUC | 17 | 2076 |
| HSV1-UL19-1691 | + | GGGGCACUCCUCGCCGG | 17 | 2077 |
| HSV1-UL19-1692 | + | UGGGGCACUCCUCGCCG | 17 | 2078 |
| HSV1-UL19-1693 | + | CUGGGGCACUCCUCGCC | 17 | 2079 |
| HSV1-UL19-1694 | + | GCUGGGGCACUCCUCGC | 17 | 2080 |
| HSV1-UL19-1695 | + | CCGUCACGGGGUCGCUG | 17 | 2081 |
| HSV1-UL19-1696 | + | UCCGUCACGGGGUCGCU | 17 | 2082 |
| HSV1-UL19-1697 | + | GUCCGUCACGGGGUCGC | 17 | 2083 |
| HSV1-UL19-1698 | + | GCGCGGGGUCCGUCACG | 17 | 2084 |
| HSV1-UL19-1699 | + | UGCGCGGGGUCCGUCAC | 17 | 2085 |
| HSV1-UL19-1700 | + | GUGCGCGGGGUCCGUCA | 17 | 2086 |
| HSV1-UL19-1701 | + | GGUGCAGGGGGUGCGCG | 17 | 2087 |
| HSV1-UL19-1702 | + | GGGUGCAGGGGGUGCGC | 17 | 2088 |
| HSV1-UL19-1703 | + | CGGGUGCAGGGGGUGCG | 17 | 2089 |
| HSV1-UL19-1704 | + | GAUUGGCCGGGUGCAGG | 17 | 2090 |
| HSV1-UL19-1705 | + | AGAUUGGCCGGGUGCAG | 17 | 2091 |
| HSV1-UL19-1706 | + | CAGAUUGGCCGGGUGCA | 17 | 2092 |
| HSV1-UL19-1707 | + | CCAGAUUGGCCGGGUGC | 17 | 2093 |
| HSV1-UL19-1708 | + | UGGCCACCAGAUUGGCC | 17 | 2094 |
| HSV1-UL19-1709 | + | UUGGCCACCAGAUUGGC | 17 | 2095 |
| HSV1-UL19-1710 | + | CGUGUUGGCCACCAGAU | 17 | 2096 |
| HSV1-UL19-1711 | + | CAUGGCGUUGACCGUGU | 17 | 2097 |
| HSV1-UL19-1712 | + | GCGCCCGUUGUGAAACA | 17 | 2098 |
| HSV1-UL19-1713 | + | UGCAGCGUGAGCAUGGC | 17 | 2099 |
| HSV1-UL19-1714 | + | CUGCAGCGUGAGCAUGG | 17 | 2100 |
| HSV1-UL19-1715 | + | CACCUGCAGCGUGAGCA | 17 | 2101 |
| HSV1-UL19-1716 | + | GCGUUCGGCCAUGUUGU | 17 | 2102 |
| HSV1-UL19-1717 | + | UGCGUUCGGCCAUGUUG | 17 | 2103 |
| HSV1-UL19-1718 | + | CAGCGCCGUUGUGCGUU | 17 | 2104 |
| HSV1-UL19-1719 | + | GUGUUGGCGCCCGCGUC | 17 | 2105 |
| HSV1-UL19-1720 | + | GGUGUUGGCGCCCGCGU | 17 | 2106 |
| HSV1-UL19-1721 | + | GGUGGUGGACGCGGUGU | 17 | 2107 |
| HSV1-UL19-1722 | + | CAUGUUGGUGGUGGACG | 17 | 2108 |
| HSV1-UL19-1723 | + | UAUGCGCAUGUUGGUGG | 17 | 2109 |
| HSV1-UL19-1724 | + | GAAUAUGCGCAUGUUGG | 17 | 2110 |
| HSV1-UL19-1725 | + | GUCGAAUAUGCGCAUGU | 17 | 2111 |
| HSV1-UL19-1726 | + | GGCCAUCAGCAGGAUUC | 17 | 2112 |
| HSV1-UL19-1727 | + | GCUGGGGGGCCAUCAGC | 17 | 2113 |
| HSV1-UL19-1728 | + | AUGGUCCAGAUGCUGGG | 17 | 2114 |
| HSV1-UL19-1729 | + | UAUGGUCCAGAUGCUGG | 17 | 2115 |
| HSV1-UL19-1730 | + | GUAUGGUCCAGAUGCUG | 17 | 2116 |
| HSV1-UL19-1731 | + | GGUAUGGUCCAGAUGCU | 17 | 2117 |
| HSV1-UL19-1732 | + | UGGUAUGGUCCAGAUGC | 17 | 2118 |
| HSV1-UL19-1733 | + | CGCCAUUUUGGAUGGUA | 17 | 2119 |
| HSV1-UL19-1734 | + | AUAGUCGCCAUUUUGGA | 17 | 2120 |
| HSV1-UL19-1735 | + | AAAAAUAGUCGCCAUUU | 17 | 2121 |
| HSV1-UL19-1736 | + | GCGCGUGGACGGGGAGG | 17 | 2122 |
| HSV1-UL19-1737 | + | AGCGCGUGGACGGGGAG | 17 | 2123 |
| HSV1-UL19-1738 | + | CAGCGCGUGGACGGGGA | 17 | 2124 |
| HSV1-UL19-1739 | + | ACAGCGCGUGGACGGGG | 17 | 2125 |
| HSV1-UL19-1740 | + | CGAACAGCGCGUGGACG | 17 | 2126 |
| HSV1-UL19-1741 | + | GCGAACAGCGCGUGGAC | 17 | 2127 |
| HSV1-UL19-1742 | + | GGCGAACAGCGCGUGGA | 17 | 2128 |
| HSV1-UL19-1743 | + | CCCCGGCGAACAGCGCG | 17 | 2129 |
| HSV1-UL19-1744 | + | CGCCACGUGGUCGGCCC | 17 | 2130 |
| HSV1-UL19-1745 | + | CGCGUUCGCCACGUGGU | 17 | 2131 |
| HSV1-UL19-1746 | + | UGGGCGCGUUCGCCACG | 17 | 2132 |
| HSV1-UL19-1747 | + | AGGGCCGGGGGGAAAUU | 17 | 2133 |
| HSV1-UL19-1748 | + | CAGGGCCGGGGGGAAAU | 17 | 2134 |
| HSV1-UL19-1749 | + | GGUCGCGCAGGGCCGGG | 17 | 2135 |
| HSV1-UL19-1750 | + | AGGUCGCGCAGGGCCGG | 17 | 2136 |
| HSV1-UL19-1751 | + | CAGGUCGCGCAGGGCCG | 17 | 2137 |
| HSV1-UL19-1752 | + | ACAGGUCGCGCAGGGCC | 17 | 2138 |
| HSV1-UL19-1753 | + | GACAGGUCGCGCAGGGC | 17 | 2139 |
| HSV1-UL19-1754 | + | CCGCGACAGGUCGCGCA | 17 | 2140 |
| HSV1-UL19-1755 | + | GCCGCGACAGGUCGCGC | 17 | 2141 |
| HSV1-UL19-1756 | + | GGGGGACCUGCCGCGAC | 17 | 2142 |
| HSV1-UL19-1757 | + | GAGCCGGGGGGACCAGG | 17 | 2143 |
| HSV1-UL19-1758 | + | AGAGCCGGGGGGACCAG | 17 | 2144 |
| HSV1-UL19-1759 | + | CAGAGCCGGGGGGACCA | 17 | 2145 |
| HSV1-UL19-1760 | + | CCAGAGCCGGGGGGACC | 17 | 2146 |
| HSV1-UL19-1761 | + | UGGCCCCCAGAGCCGGG | 17 | 2147 |
| HSV1-UL19-1762 | + | UUGGCCCCCAGAGCCGG | 17 | 2148 |
| HSV1-UL19-1763 | + | GUUGGCCCCCAGAGCCG | 17 | 2149 |
| HSV1-UL19-1764 | + | AGUUGGCCCCCAGAGCC | 17 | 2150 |
| HSV1-UL19-1765 | + | UAGUUGGCCCCCAGAGC | 17 | 2151 |
| HSV1-UL19-1766 | + | GAUCGACGAAAAGUAGU | 17 | 2152 |
| HSV1-UL19-1767 | + | GCUGCACGACGGGCUGU | 17 | 2153 |
| HSV1-UL19-1768 | + | CGGACGUGCUGCACGAC | 17 | 2154 |
| HSV1-UL19-1769 | + | GCGGACGUGCUGCACGA | 17 | 2155 |
| HSV1-UL19-1770 | + | CCCCGGCCGCGCUCUCG | 17 | 2156 |
| HSV1-UL19-1771 | + | GGUCAGCGCGUUCUCCC | 17 | 2157 |
| HSV1-UL19-1772 | + | CCCCGCCAUGAGCGCGU | 17 | 2158 |
| HSV1-UL19-1773 | + | UGAUGAUGCAAGGCCAC | 17 | 2159 |
| HSV1-UL19-1774 | + | CUGAUGAUGCAAGGCCA | 17 | 2160 |
| HSV1-UL19-1775 | + | CUUGAGCUGAUGAUGCA | 17 | 2161 |
| HSV1-UL19-1776 | + | ACCCAAACCCGGGAUGG | 17 | 2162 |
| HSV1-UL19-1777 | + | UGAACCCAAACCCGGGA | 17 | 2163 |
| HSV1-UL19-1778 | + | ACGGUGAACCCAAACCC | 17 | 2164 |
| HSV1-UL19-1779 | + | GACGGUGAACCCAAACC | 17 | 2165 |
| HSV1-UL19-1780 | + | GCGGUCCUGUCGGACGA | 17 | 2166 |
| HSV1-UL19-1781 | + | UCACAAAGCGGUCCUGU | 17 | 2167 |
| HSV1-UL19-1782 | + | CGUUCUCAGUCACAAAG | 17 | 2168 |
| HSV1-UL19-1783 | + | CCACCUGGAGCUGGCCC | 17 | 2169 |
| HSV1-UL19-1784 | + | GCCGGGCCACCUGGAGC | 17 | 2170 |
| HSV1-UL19-1785 | + | UUUCGUGCCGGGCCACC | 17 | 2171 |
| HSV1-UL19-1786 | + | CCCGCCAGUUUCGUGCC | 17 | 2172 |
| HSV1-UL19-1787 | + | CCCCGCCAGUUUCGUGC | 17 | 2173 |
| HSV1-UL19-1788 | + | CACGUUCGCGCGCGGCU | 17 | 2174 |
| HSV1-UL19-1789 | + | CCACGUUCGCGCGCGGC | 17 | 2175 |
| HSV1-UL19-1790 | + | AGGUCCACGUUCGCGCG | 17 | 2176 |
| HSV1-UL19-1791 | + | CGGUGUAGCCCACGCCC | 17 | 2177 |
| HSV1-UL19-1792 | + | UGCCGUGGCCACGACGG | 17 | 2178 |
| HSV1-UL19-1793 | + | GGUUGCCGUGGCCACGA | 17 | 2179 |
| HSV1-UL19-1794 | + | GUUGCGGACGGUUGCCG | 17 | 2180 |
| HSV1-UL19-1795 | + | GGUGACGGGGUUGCGGA | 17 | 2181 |
| HSV1-UL19-1796 | + | UGUCGGUGACGGGGUUG | 17 | 2182 |
| HSV1-UL19-1797 | + | UGCCCAUGUCGGUGACG | 17 | 2183 |
| HSV1-UL19-1798 | + | UUGCCCAUGUCGGUGAC | 17 | 2184 |
| HSV1-UL19-1799 | + | GUUGCCCAUGUCGGUGA | 17 | 2185 |
| HSV1-UL19-1800 | + | GGGAAGGUUGCCCAUGU | 17 | 2186 |
| HSV1-UL19-1801 | + | GGUAAAAGUUUUGGGGA | 17 | 2187 |
| HSV1-UL19-1802 | + | CCCAGGUAAAAGUUUUG | 17 | 2188 |
| HSV1-UL19-1803 | + | GCCCAGGUAAAAGUUUU | 17 | 2189 |
| HSV1-UL19-1804 | + | GGCCCAGGUAAAAGUUU | 17 | 2190 |
| HSV1-UL19-1805 | + | GGGGAGCCCCGCGGCCC | 17 | 2191 |
| HSV1-UL19-1806 | + | GGAGAGGGGGAGCCCCG | 17 | 2192 |
| HSV1-UL19-1807 | + | GCGUUGUCCAGGAGAGG | 17 | 2193 |
| HSV1-UL19-1808 | + | CGCGUUGUCCAGGAGAG | 17 | 2194 |
| HSV1-UL19-1809 | + | CCGCGUUGUCCAGGAGA | 17 | 2195 |
| HSV1-UL19-1810 | + | GCCGCGUUGUCCAGGAG | 17 | 2196 |
| HSV1-UL19-1811 | + | CGGCUGCCGCGUUGUCC | 17 | 2197 |
| HSV1-UL19-1812 | + | CGCGUUCCGCAGGUACA | 17 | 2198 |
| HSV1-UL19-1813 | + | CCACGACCGCGUUCCGC | 17 | 2199 |
| HSV1-UL19-1814 | + | GCUGGGCCGGCCCCAGG | 17 | 2200 |
| HSV1-UL19-1815 | + | CGGGCUGGGCCGGCCCC | 17 | 2201 |
| HSV1-UL19-1816 | + | ACGGGGACGGGCUGGGC | 17 | 2202 |
| HSV1-UL19-1817 | + | GAACACGGGGACGGGCU | 17 | 2203 |
| HSV1-UL19-1818 | + | CGAACACGGGGACGGGC | 17 | 2204 |
| HSV1-UL19-1819 | + | CACCCGAACACGGGGAC | 17 | 2205 |
| HSV1-UL19-1820 | + | GCACCCGAACACGGGGA | 17 | 2206 |
| HSV1-UL19-1821 | + | GGGCGCACCCGAACACG | 17 | 2207 |
| HSV1-UL19-1822 | + | UGGGCGCACCCGAACAC | 17 | 2208 |
| HSV1-UL19-1823 | + | CUGGGCGCACCCGAACA | 17 | 2209 |
| HSV1-UL19-1824 | + | UGCGCGCCGCGGCACCU | 17 | 2210 |
| HSV1-UL19-1825 | + | CUGCGCGCCGCGGCACC | 17 | 2211 |
| HSV1-UL19-1826 | + | UCCAUCCCUGCGCGCCG | 17 | 2212 |
| HSV1-UL19-1827 | + | ACACGGCGUCCUGGCCG | 17 | 2213 |
| HSV1-UL19-1828 | + | ACUCACACACGGCGUCC | 17 | 2214 |
| HSV1-UL19-1829 | + | GGCGAUAAACUCACACA | 17 | 2215 |
| HSV1-UL19-1830 | + | GUCGGUCGACACGGGGG | 17 | 2216 |
| HSV1-UL19-1831 | + | GACGUCGGUCGACACGG | 17 | 2217 |
| HSV1-UL19-1832 | + | UGACGUCGGUCGACACG | 17 | 2218 |
| HSV1-UL19-1833 | + | UUGACGUCGGUCGACAC | 17 | 2219 |
| HSV1-UL19-1834 | + | GUUGACGUCGGUCGACA | 17 | 2220 |
| HSV1-UL19-1835 | + | GCGGAAGUAGUUGACGU | 17 | 2221 |
| HSV1-UL19-1836 | + | GGGGGUUGCAGGGCCGG | 17 | 2222 |
| HSV1-UL19-1837 | + | CCCGGGGGUUGCAGGGC | 17 | 2223 |
| HSV1-UL19-1838 | + | CGUCCCCGGGGGUUGCA | 17 | 2224 |
| HSV1-UL19-1839 | + | GCGUCCCCGGGGGUUGC | 17 | 2225 |
| HSV1-UL19-1840 | + | CGGCGGCGCGUCCCCGG | 17 | 2226 |
| HSV1-UL19-1841 | + | CCGGCGGCGCGUCCCCG | 17 | 2227 |
| HSV1-UL19-1842 | + | CCCGGCGGCGCGUCCCC | 17 | 2228 |
| HSV1-UL19-1843 | + | CCCCGGCGGCGCGUCCC | 17 | 2229 |
| HSV1-UL19-1844 | + | CGCGUAAACGCCCCCGG | 17 | 2230 |
| HSV1-UL19-1845 | + | CCCCGCGUAAACGCCCC | 17 | 2231 |
| HSV1-UL19-1846 | + | GUGGUCGUACAUGAGGG | 17 | 2232 |
| HSV1-UL19-1847 | + | GCCGUGGUCGUACAUGA | 17 | 2233 |
| HSV1-UL19-1848 | + | GGCCGUGGUCGUACAUG | 17 | 2234 |
| HSV1-UL19-1849 | + | ACGGGUCGCUCUGGCCG | 17 | 2235 |
| HSV1-UL19-1850 | + | CCCGGGACGGGUCGCUC | 17 | 2236 |
| HSV1-UL19-1851 | + | CCGCGAAGGCCCGGGAC | 17 | 2237 |
| HSV1-UL19-1852 | + | GCCGCGAAGGCCCGGGA | 17 | 2238 |
| HSV1-UL19-1853 | + | CGUGGCCGCGAAGGCCC | 17 | 2239 |
| HSV1-UL19-1854 | + | CCGUGGCCGCGAAGGCC | 17 | 2240 |
| HSV1-UL19-1855 | + | GUUGGCCGUGGCCGCGA | 17 | 2241 |
| HSV1-UL19-1856 | + | CGCCCACGGGUUGGCCG | 17 | 2242 |
| HSV1-UL19-1857 | + | CUGCGACGCCCACGGGU | 17 | 2243 |
| HSV1-UL19-1858 | + | AUCGCUGCGACGCCCAC | 17 | 2244 |
| HSV1-UL19-1859 | + | AAUCGCUGCGACGCCCA | 17 | 2245 |
| HSV1-UL19-1860 | + | AGGCCCCGUUAUAGAGC | 17 | 2246 |
| HSV1-UL19-1861 | + | GGCCCCGUUGAGGUGGU | 17 | 2247 |
| HSV1-UL19-1862 | + | GCGAGGCCCCGUUGAGG | 17 | 2248 |
| HSV1-UL19-1863 | + | CCGGCGAGGCCCCGUUG | 17 | 2249 |
| HSV1-UL19-1864 | + | GGGGCUGAGCACCGGCG | 17 | 2250 |
| HSV1-UL19-1865 | + | AAGCAGGGGCUGAGCAC | 17 | 2251 |
| HSV1-UL19-1866 | + | UAAAGAACUUAAAGCAG | 17 | 2252 |
| HSV1-UL19-1867 | + | GUAAAGAACUUAAAGCA | 17 | 2253 |
| HSV1-UL19-1868 | + | CGUAAAGAACUUAAAGC | 17 | 2254 |
| HSV1-UL19-1869 | + | AUGUUUGGCGGCGAUGU | 17 | 2255 |
| HSV1-UL19-1870 | + | CAGGCAGCGAUGUUUGG | 17 | 2256 |
| HSV1-UL19-1871 | + | CUCCAGGCAGCGAUGUU | 17 | 2257 |
| HSV1-UL19-1872 | + | CCACGAUCAGGCGCUCC | 17 | 2258 |
| HSV1-UL19-1873 | + | AACCCGUCUCCACGAUC | 17 | 2259 |
| HSV1-UL19-1874 | + | GCUGGCGGCGGUGGCCG | 17 | 2260 |
| HSV1-UL19-1875 | + | UACGUCGCUGGCGGCGG | 17 | 2261 |
| HSV1-UL19-1876 | + | CUGUACGUCGCUGGCGG | 17 | 2262 |
| HSV1-UL19-1877 | + | AAACUGUACGUCGCUGG | 17 | 2263 |
| HSV1-UL19-1878 | + | CUUAAACUGUACGUCGC | 17 | 2264 |
| HSV1-UL19-1879 | + | GUUCGCGGCACCCCGGG | 17 | 2265 |
| HSV1-UL19-1880 | + | AGUUCGCGGCACCCCGG | 17 | 2266 |
| HSV1-UL19-1881 | + | GAGUUCGCGGCACCCCG | 17 | 2267 |
| HSV1-UL19-1882 | + | CGAGUUCGCGGCACCCC | 17 | 2268 |
| HSV1-UL19-1883 | + | ACGAGUUCGCGGCACCC | 17 | 2269 |
| HSV1-UL19-1884 | + | GGUCCUCCACGAGUUCG | 17 | 2270 |
| HSV1-UL19-1885 | + | CCUGAAACAGGCCACAC | 17 | 2271 |
| HSV1-UL19-1886 | + | UCCUGAAACAGGCCACA | 17 | 2272 |
| HSV1-UL19-1887 | + | GGUAGGCCUCCUGAAAC | 17 | 2273 |
| HSV1-UL19-1888 | + | GGCGCAGGUGAGCGGGU | 17 | 2274 |
| HSV1-UL19-1889 | + | CGCUGGCGCAGGUGAGC | 17 | 2275 |
| HSV1-UL19-1890 | + | UCGCUGGCGCAGGUGAG | 17 | 2276 |
| HSV1-UL19-1891 | + | GGCGGGGUCGCUGGCGC | 17 | 2277 |
| HSV1-UL19-1892 | + | GAGGAGGGCGGGGUCGC | 17 | 2278 |
| HSV1-UL19-1893 | + | CACUGCGGAGGAGGGCG | 17 | 2279 |
| HSV1-UL19-1894 | + | GCACUGCGGAGGAGGGC | 17 | 2280 |
| HSV1-UL19-1895 | + | GGCACUGCGGAGGAGGG | 17 | 2281 |
| HSV1-UL19-1896 | + | GCGGGCACUGCGGAGGA | 17 | 2282 |
| HSV1-UL19-1897 | + | UGCGGGCACUGCGGAGG | 17 | 2283 |
| HSV1-UL19-1898 | + | CGUUGCGGGCACUGCGG | 17 | 2284 |
| HSV1-UL19-1899 | + | CCCCGUUGCGGGCACUG | 17 | 2285 |
| HSV1-UL19-1900 | + | GUGGGCUUCCCCGUUGC | 17 | 2286 |
| HSV1-UL19-1901 | + | CGUGGGCUUCCCCGUUG | 17 | 2287 |
| HSV1-UL19-1902 | + | GUGGGUCUCCCGCGCGU | 17 | 2288 |
| HSV1-UL19-1903 | + | AGUGGGUCUCCCGCGCG | 17 | 2289 |
| HSV1-UL19-1904 | + | GAGAUACUGCGCGAAGU | 17 | 2290 |
| HSV1-UL19-1905 | + | CGAGAUACUGCGCGAAG | 17 | 2291 |
| HSV1-UL19-1906 | + | AGCCAGUCCCUUGAGCG | 17 | 2292 |
| HSV1-UL19-1907 | + | GAGCCAGUCCCUUGAGC | 17 | 2293 |
| HSV1-UL19-1908 | + | AGAGCCAGUCCCUUGAG | 17 | 2294 |

**Table 1D** provides exemplary targeting domains for knocking out the *UL19* gene selected according to the first tier parameters. The targeting domains are selected based on location within first 500bp of the coding sequence of the *UL19* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1D**

| 1st Tier | | | | |
|---|---|---|---|---|
| gRNA Name | DNA Strand | Targeting Domain | Target Site Length | Seq ID |
| HSV1-UL19-1909 | - | CCGCUCCCAACCGCGACCCU | 20 | 2295 |
| HSV1-UL19-1 | - | CGCUCCCAACCGCGACCCUC | 20 | 461 |
| HSV1-UL19-1911 | - | UGCCGCGGCCAUGGUGCCGA | 20 | 2296 |
| HSV1-UL19-1912 | - | CGGGUCCCUCCUUAGCACGA | 20 | 2297 |
| HSV1-UL19-1913 | - | UGCAAACAGCCUGUACGACG | 20 | 2298 |
| HSV1-UL19-1914 | - | ACGUCGAGUUCGACGCCCUG | 20 | 2299 |
| HSV1-UL19-10 | - | CGUCGAGUUCGACGCCCUGC | 20 | 467 |
| HSV1-UL19-1916 | - | CCCUGUCGCUCGUGCGCUUU | 20 | 2300 |
| HSV1-UL19-13 | - | CCUGUCGCUCGUGCGCUUUC | 20 | 395 |
| HSV1-UL19-1918 | - | GCUCGUGCGCUUUCUGGAGC | 20 | 2301 |
| HSV1-UL19-1919 | - | CGUGCGUGUGUACCAAGUUU | 20 | 2302 |
| HSV1-UL19-18 | - | GUGCGUGUGUACCAAGUUUC | 20 | 404 |
| HSV1-UL19-1921 | - | AGUUUCCGGAGCUGGCCUAC | 20 | 2303 |
| HSV1-UL19-1922 | - | UCCGGAGCUGGCCUACAUGA | 20 | 2304 |
| HSV1-UL19-1923 | - | CGGAGCUGGCCUACAUGAAC | 20 | 2305 |
| HSV1-UL19-20 | - | GGAGCUGGCCUACAUGAACG | 20 | 469 |
| HSV1-UL19-1925 | - | GAACGAGGGGCGCGUACAGU | 20 | 2306 |
| HSV1-UL19-1926 | - | CCGCGACGGCCCGCACCCCA | 20 | 2307 |
| HSV1-UL19-1927 | - | AUGACCAAGAUUAUCGACCG | 20 | 2308 |
| HSV1-UL19-1928 | - | AGAUUAUCGACCGCCGGGCC | 20 | 2309 |
| HSV1-UL19-1929 | - | CGCCGCCUUCAGCCUGGCCA | 20 | 2310 |
| HSV1-UL19-1930 | - | CCGAGGCCAUCGCCCUGCUC | 20 | 2311 |
| HSV1-UL19-29 | - | CGAGGCCAUCGCCCUGCUCA | 20 | 474 |
| HSV1-UL19-30 | - | GAGGCCAUCGCCCUGCUCAC | 20 | 475 |
| HSV1-UL19-31 | - | AGGCCAUCGCCCUGCUCACG | 20 | 417 |
| HSV1-UL19-32 | - | GGCCAUCGCCCUGCUCACGG | 20 | 476 |
| HSV1-UL19-1935 | - | CCCUGCUCACGGGGGAGGCC | 20 | 2312 |
| HSV1-UL19-1936 | - | GCUCACGGGGGAGGCCCUGG | 20 | 2313 |
| HSV1-UL19-35 | - | CUCACGGGGGAGGCCCUGGA | 20 | 478 |
| HSV1-UL19-1938 | - | CGGGGGAGGCCCUGGACGGG | 20 | 2314 |
| HSV1-UL19-1939 | - | CUCCCAACCGCGACCCU | 17 | 2315 |
| HSV1-UL19-95 | - | UCCCAACCGCGACCCUC | 17 | 518 |
| HSV1-UL19-1941 | - | CGCGGCCAUGGUGCCGA | 17 | 2316 |
| HSV1-UL19-1942 | - | GUCCCUCCUUAGCACGA | 17 | 2317 |
| HSV1-UL19-1943 | - | AAACAGCCUGUACGACG | 17 | 2318 |
| HSV1-UL19-1944 | - | UCGAGUUCGACGCCCUG | 17 | 2319 |
| HSV1-UL19-104 | - | CGAGUUCGACGCCCUGC | 17 | 524 |
| HSV1-UL19-1946 | - | UGUCGCUCGUGCGCUUU | 17 | 2320 |
| HSV1-UL19-107 | - | GUCGCUCGUGCGCUUUC | 17 | 400 |
| HSV1-UL19-1948 | - | CGUGCGCUUUCUGGAGC | 17 | 2321 |
| HSV1-UL19-1949 | - | GCGUGUGUACCAAGUUU | 17 | 2322 |
| HSV1-UL19-112 | - | CGUGUGUACCAAGUUUC | 17 | 407 |
| HSV1-UL19-1951 | - | UUCCGGAGCUGGCCUAC | 17 | 2323 |
| HSV1-UL19-1952 | - | GGAGCUGGCCUACAUGA | 17 | 2324 |
| HSV1-UL19-1953 | - | AGCUGGCCUACAUGAAC | 17 | 2325 |
| HSV1-UL19-114 | - | GCUGGCCUACAUGAACG | 17 | 526 |
| HSV1-UL19-1955 | - | CGAGGGGCGCGUACAGU | 17 | 2326 |
| HSV1-UL19-1956 | - | CGACGGCCCGCACCCCA | 17 | 2327 |
| HSV1-UL19-1957 | - | ACCAAGAUUAUCGACCG | 17 | 2328 |
| HSV1-UL19-1958 | - | UUAUCGACCGCCGGGCC | 17 | 2329 |
| HSV1-UL19-1959 | - | CGCCUUCAGCCUGGCCA | 17 | 2330 |
| HSV1-UL19-1960 | - | AGGCCAUCGCCCUGCUC | 17 | 2331 |
| HSV1-UL19-123 | - | GGCCAUCGCCCUGCUCA | 17 | 531 |
| HSV1-UL19-124 | - | GCCAUCGCCCUGCUCAC | 17 | 532 |
| HSV1-UL19-125 | - | CCAUCGCCCUGCUCACG | 17 | 418 |
| HSV1-UL19-126 | - | CAUCGCCCUGCUCACGG | 17 | 533 |
| HSV1-UL19-1965 | - | UGCUCACGGGGGAGGCC | 17 | 2332 |
| HSV1-UL19-1966 | - | CACGGGGGAGGCCCUGG | 17 | 2333 |
| HSV1-UL19-129 | - | ACGGGGGAGGCCCUGGA | 17 | 535 |
| HSV1-UL19-1968 | - | GGGAGGCCCUGGACGGG | 17 | 2334 |
| HSV1-UL19-93 | + | UAUCCCGGAGGGUCGCGGUU | 20 | 457 |
| HSV1-UL19-92 | + | GUAUCCCGGAGGGUCGCGGU | 20 | 456 |
| HSV1-UL19-1971 | + | GGUAUCCCGGAGGGUCGCGG | 20 | 2335 |
| HSV1-UL19-1972 | + | CGCGGCAUACCGGUAUCCCG | 20 | 2336 |
| HSV1-UL19-88 | + | GCCGCGGCAUACCGGUAUCC | 20 | 514 |
| HSV1-UL19-1974 | + | GGCCGCGGCAUACCGGUAUC | 20 | 2337 |
| HSV1-UL19-81 | + | CCACCUCGAUCGUGCUAAGG | 20 | 509 |
| HSV1-UL19-1976 | + | GCCACCUCGAUCGUGCUAAG | 20 | 2338 |
| HSV1-UL19-80 | + | ACGCCACCUCGAUCGUGCUA | 20 | 508 |
| HSV1-UL19-1978 | + | GACGCCACCUCGAUCGUGCU | 20 | 2339 |
| HSV1-UL19-78 | + | GUUUGCAUCGGAGCGCACGC | 20 | 506 |
| HSV1-UL19-77 | + | UGUUUGCAUCGGAGCGCACG | 20 | 505 |
| HSV1-UL19-1981 | + | CUGUUUGCAUCGGAGCGCAC | 20 | 2340 |
| HSV1-UL19-76 | + | GUCGUACAGGCUGUUUGCAU | 20 | 442 |
| HSV1-UL19-1983 | + | CGUCGUACAGGCUGUUUGCA | 20 | 2341 |
| HSV1-UL19-1984 | + | AAUACGACCCCAGCAGGGCG | 20 | 2342 |
| HSV1-UL19-1985 | + | GUGUUGCAAUACGACCCCAG | 20 | 2343 |
| HSV1-UL19-1986 | + | UCCAGAAAGCGCACGAGCGA | 20 | 2344 |
| HSV1-UL19-1987 | + | ACCCGAGCUCCAGAAAGCGC | 20 | 2345 |
| HSV1-UL19-1988 | + | GCCACCGACAACCCGAGCUC | 20 | 2346 |
| HSV1-UL19-1989 | + | ACACGCACGCCACCGACAAC | 20 | 2347 |
| HSV1-UL19-69 | + | UCGUUCAUGUAGGCCAGCUC | 20 | 435 |
| HSV1-UL19-1991 | + | CUCGUUCAUGUAGGCCAGCU | 20 | 2348 |
| HSV1-UL19-1992 | + | CAAUCAGCGGCUGGUGGACC | 20 | 2349 |
| HSV1-UL19-1993 | + | UCGCGGGCAAUCAGCGGCUG | 20 | 2350 |
| HSV1-UL19-1994 | + | UCGAUGGGGUGCGGGCCGUC | 20 | 2351 |
| HSV1-UL19-1995 | + | UGUGGGUUGCUCGAUGGGGU | 20 | 2352 |
| HSV1-UL19-58 | + | AUUGUGUGUGGGUUGCUCGA | 20 | 425 |
| HSV1-UL19-1997 | + | AAUUGUGUGUGGGUUGCUCG | 20 | 2353 |
| HSV1-UL19-1998 | + | UCUUGGUCAUGUAAUUGUGU | 20 | 2354 |
| HSV1-UL19-1999 | + | GCCAGGCUGAAGGCGGCGUU | 20 | 2355 |
| HSV1-UL19-2000 | + | CGAUGGCCUCGGUGGCCAGG | 20 | 2356 |
| HSV1-UL19-2001 | + | UCCAGGGCCUCCCCCGUGAG | 20 | 2357 |
| HSV1-UL19-2002 | + | UCCCGUCCAGGGCCUCCCCC | 20 | 2358 |
| HSV1-UL19-2003 | + | GCGCCGAUACCCGUCCCGUC | 20 | 2359 |
| HSV1-UL19-187 | + | CCCGGAGGGUCGCGGUU | 17 | 460 |
| HSV1-UL19-186 | + | UCCCGGAGGGUCGCGGU | 17 | 459 |
| HSV1-UL19-2006 | + | AUCCCGGAGGGUCGCGG | 17 | 2360 |
| HSV1-UL19-2007 | + | GGCAUACCGGUAUCCCG | 17 | 2361 |
| HSV1-UL19-182 | + | GCGGCAUACCGGUAUCC | 17 | 571 |
| HSV1-UL19-2009 | + | CGCGGCAUACCGGUAUC | 17 | 2362 |
| HSV1-UL19-175 | + | CCUCGAUCGUGCUAAGG | 17 | 566 |
| HSV1-UL19-2011 | + | ACCUCGAUCGUGCUAAG | 17 | 2363 |
| HSV1-UL19-174 | + | CCACCUCGAUCGUGCUA | 17 | 565 |
| HSV1-UL19-2013 | + | GCCACCUCGAUCGUGCU | 17 | 2364 |
| HSV1-UL19-172 | + | UGCAUCGGAGCGCACGC | 17 | 563 |
| HSV1-UL19-171 | + | UUGCAUCGGAGCGCACG | 17 | 562 |
| HSV1-UL19-2016 | + | UUUGCAUCGGAGCGCAC | 17 | 2365 |
| HSV1-UL19-170 | + | GUACAGGCUGUUUGCAU | 17 | 450 |
| HSV1-UL19-2018 | + | CGUACAGGCUGUUUGCA | 17 | 2366 |
| HSV1-UL19-2019 | + | ACGACCCCAGCAGGGCG | 17 | 2367 |
| HSV1-UL19-2020 | + | UUGCAAUACGACCCCAG | 17 | 2368 |
| HSV1-UL19-2021 | + | AGAAAGCGCACGAGCGA | 17 | 2369 |
| HSV1-UL19-2022 | + | CGAGCUCCAGAAAGCGC | 17 | 2370 |
| HSV1-UL19-2023 | + | ACCGACAACCCGAGCUC | 17 | 2371 |
| HSV1-UL19-2024 | + | CGCACGCCACCGACAAC | 17 | 2372 |
| HSV1-UL19-163 | + | UUCAUGUAGGCCAGCUC | 17 | 443 |
| HSV1-UL19-2026 | + | GUUCAUGUAGGCCAGCU | 17 | 2373 |
| HSV1-UL19-2027 | + | UCAGCGGCUGGUGGACC | 17 | 2374 |
| HSV1-UL19-2028 | + | CGGGCAAUCAGCGGCUG | 17 | 2375 |
| HSV1-UL19-2029 | + | AUGGGGUGCGGGCCGUC | 17 | 2376 |
| HSV1-UL19-2030 | + | GGGUUGCUCGAUGGGGU | 17 | 2377 |
| HSV1-UL19-152 | + | GUGUGUGGGUUGCUCGA | 17 | 427 |
| HSV1-UL19-2032 | + | UGUGUGUGGGUUGCUCG | 17 | 2378 |
| HSV1-UL19-2033 | + | UGGUCAUGUAAUUGUGU | 17 | 2379 |
| HSV1-UL19-2034 | + | AGGCUGAAGGCGGCGUU | 17 | 2380 |
| HSV1-UL19-2035 | + | UGGCCUCGGUGGCCAGG | 17 | 2381 |
| HSV1-UL19-2036 | + | AGGGCCUCCCCCGUGAG | 17 | 2382 |
| HSV1-UL19-2037 | + | CGUCCAGGGCCUCCCCC | 17 | 2383 |
| HSV1-UL19-2038 | + | CCGAUACCCGUCCCGUC | 17 | 2384 |

**Table 1E** provides exemplary targeting domains for knocking out the *UL19* gene selected according to the second tier parameters. The targeting domains are selected based on location within the coding sequence, but downstream of the first 500bp of the *UL19* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1E**

| 2nd Tier | | | | |
|---|---|---|---|---|
| gRNA Name | DNA Strand | Targeting Domain | Target Site Length | Seq ID |
| HSV1-UL19-189 | - | CGCAACGUCCAGGCCGUCCU | 20 | 575 |
| HSV1-UL19-2040 | - | CCAGGCCGUCCUCGGGGCGU | 20 | 2385 |
| HSV1-UL19-2041 | - | ACCAGAUGCUGCACGUGCUG | 20 | 2386 |
| HSV1-UL19-194 | - | CCAGAUGCUGCACGUGCUGC | 20 | 580 |
| HSV1-UL19-2043 | - | UGUUGCCGAUGCAACGAUAC | 20 | 2387 |
| HSV1-UL19-2044 | - | GACAACGGCCGCCUGGCCAC | 20 | 2388 |
| HSV1-UL19-2045 | - | CGCCUGGCCACCAGGGUGGC | 20 | 2389 |
| HSV1-UL19-2046 | - | GGCCCGGGCGACCCUGGUCG | 20 | 2390 |
| HSV1-UL19-2047 | - | ACCCUGGUCGCCGAGCUAAA | 20 | 2391 |
| HSV1-UL19-2048 | - | CGAGCUAAAGCGAAGCUUCU | 20 | 2392 |
| HSV1-UL19-2049 | - | UAAAGCGAAGCUUCUGCGAG | 20 | 2393 |
| HSV1-UL19-2050 | - | UCUGCGAGACGAGCUUUUUC | 20 | 2394 |
| HSV1-UL19-2051 | - | CGAGCUUUUUCCUGGGCAAG | 20 | 2395 |
| HSV1-UL19-2052 | - | CUGGGCAAGGCGGGCCACCG | 20 | 2396 |
| HSV1-UL19-211 | - | UGGGCAAGGCGGGCCACCGC | 20 | 597 |
| HSV1-UL19-212 | - | GGGCAAGGCGGGCCACCGCC | 20 | 598 |
| HSV1-UL19-2055 | - | GGGCCACCGCCGGGAGGCCG | 20 | 2397 |
| HSV1-UL19-2056 | - | AGGCCGUCGAGGCCUGGCUC | 20 | 2398 |
| HSV1-UL19-2057 | - | UCUGACGCAUGCCGACACGC | 20 | 2399 |
| HSV1-UL19-2058 | - | CACGCGCGGGCGGCCGGUCG | 20 | 2400 |
| HSV1-UL19-223 | - | ACGCGCGGGCGGCCGGUCGA | 20 | 609 |
| HSV1-UL19-2060 | - | AGCGCCUCCUGCAGUCCUUC | 20 | 2401 |
| HSV1-UL19-2061 | - | UCCUGCAGUCCUUCCUGAAA | 20 | 2402 |
| HSV1-UL19-226 | - | CCUGCAGUCCUUCCUGAAAG | 20 | 612 |
| HSV1-UL19-2063 | - | UGCAGUCCUUCCUGAAAGUG | 20 | 2403 |
| HSV1-UL19-2064 | - | CUUCCUGAAAGUGGAGGACA | 20 | 2404 |
| HSV1-UL19-2065 | - | CGACGUGCCGGUGACGUACG | 20 | 2405 |
| HSV1-UL19-2066 | - | UGACGUACGGCGAGAUGGUC | 20 | 2406 |
| HSV1-UL19-2067 | - | GUACGGCGAGAUGGUCCUGA | 20 | 2407 |
| HSV1-UL19-231 | - | UACGGCGAGAUGGUCCUGAA | 20 | 617 |
| HSV1-UL19-2069 | - | ACCUGGUCACGGCGCUCGUG | 20 | 2408 |
| HSV1-UL19-2070 | - | CUCGUGAUGGGCAAGGCCGU | 20 | 2409 |
| HSV1-UL19-2071 | - | UGGGCAAGGCCGUGCGAAGU | 20 | 2410 |
| HSV1-UL19-2072 | - | CCGUGCGAAGUCUGGACGAC | 20 | 2411 |
| HSV1-UL19-2073 | - | ACGACGUGGGCCGCCACCUG | 20 | 2412 |
| HSV1-UL19-242 | - | CGACGUGGGCCGCCACCUGC | 20 | 628 |
| HSV1-UL19-2075 | - | GCCGCCACCUGCUGGAGAUG | 20 | 2413 |
| HSV1-UL19-243 | - | CCGCCACCUGCUGGAGAUGC | 20 | 629 |
| HSV1-UL19-2077 | - | GCCACCUGCUGGAGAUGCAG | 20 | 2414 |
| HSV1-UL19-244 | - | CCACCUGCUGGAGAUGCAGG | 20 | 630 |
| HSV1-UL19-2079 | - | UGCAGGAGGAGCAGCUCGAC | 20 | 2415 |
| HSV1-UL19-2080 | - | UCGACCUGAACCGGCAGACG | 20 | 2416 |
| HSV1-UL19-2081 | - | CCUGAACCGGCAGACGCUGG | 20 | 2417 |
| HSV1-UL19-2082 | - | CCGGCAGACGCUGGACGAGC | 20 | 2418 |
| HSV1-UL19-2083 | - | GGCAGACGCUGGACGAGCUC | 20 | 2419 |
| HSV1-UL19-2084 | - | CCCAGACGACGCGCGUGCGC | 20 | 2420 |
| HSV1-UL19-2085 | - | CGCGGAUCUGGUGUCCAUCG | 20 | 2421 |
| HSV1-UL19-2086 | - | CGGAUCUGGUGUCCAUCGGC | 20 | 2422 |
| HSV1-UL19-2087 | - | UCGGCGAGAAGCUGGUCUUU | 20 | 2423 |
| HSV1-UL19-251 | - | CGGCGAGAAGCUGGUCUUUC | 20 | 637 |
| HSV1-UL19-2089 | - | AGCUGGUCUUUCUGGAGGCC | 20 | 2424 |
| HSV1-UL19-253 | - | GCUGGUCUUUCUGGAGGCCC | 20 | 639 |
| HSV1-UL19-2091 | - | UGGUCUUUCUGGAGGCCCUG | 20 | 2425 |
| HSV1-UL19-2092 | - | CCAACGUUCCCUACCCCCUG | 20 | 2426 |
| HSV1-UL19-2093 | - | CCUACCCCCUGGUGGGCGCC | 20 | 2427 |
| HSV1-UL19-2094 | - | ACCUGACGUUCGUCCUGCCC | 20 | 2428 |
| HSV1-UL19-2095 | - | UGGGCCUGUUCAAUCCGGUC | 20 | 2429 |
| HSV1-UL19-261 | - | GGGCCUGUUCAAUCCGGUCA | 20 | 647 |
| HSV1-UL19-2097 | - | GGAACGGUUUGCCGCGCACG | 20 | 2430 |
| HSV1-UL19-263 | - | GAACGGUUUGCCGCGCACGC | 20 | 649 |
| HSV1-UL19-264 | - | AACGGUUUGCCGCGCACGCC | 20 | 650 |
| HSV1-UL19-2100 | - | UAGUCCCCGCCCCCGGCCAC | 20 | 2431 |
| HSV1-UL19-2101 | - | CCGCCCCGCCAGCUGUUUUU | 20 | 2432 |
| HSV1-UL19-268 | - | CGCCCCGCCAGCUGUUUUUU | 20 | 654 |
| HSV1-UL19-269 | - | GCCCCGCCAGCUGUUUUUUU | 20 | 655 |
| HSV1-UL19-270 | - | CCCCGCCAGCUGUUUUUUUG | 20 | 656 |
| HSV1-UL19-271 | - | CCCGCCAGCUGUUUUUUUGG | 20 | 657 |
| HSV1-UL19-2106 | - | GCCAGCUGUUUUUUUGGGGG | 20 | 2433 |
| HSV1-UL19-2107 | - | GCCAGGUGCUGCGCCUGUCU | 20 | 2434 |
| HSV1-UL19-274 | - | CCAGGUGCUGCGCCUGUCUC | 20 | 660 |
| HSV1-UL19-2109 | - | CCUGUCUCUGGAACACGCGA | 20 | 2435 |
| HSV1-UL19-275 | - | CUGUCUCUGGAACACGCGAU | 20 | 661 |
| HSV1-UL19-2111 | - | CCGUGUGCCACCCUUCGCUG | 20 | 2436 |
| HSV1-UL19-2112 | - | GAUGAACGUUGACGCGGCGG | 20 | 2437 |
| HSV1-UL19-279 | - | AUGAACGUUGACGCGGCGGU | 20 | 665 |
| HSV1-UL19-280 | - | UGAACGUUGACGCGGCGGUC | 20 | 666 |
| HSV1-UL19-2115 | - | GGGCCUUAACCGCGACCCCG | 20 | 2438 |
| HSV1-UL19-2116 | - | CGUCGAAGCCGCCAAUCCGU | 20 | 2439 |
| HSV1-UL19-283 | - | GUCGAAGCCGCCAAUCCGUA | 20 | 669 |
| HSV1-UL19-2118 | - | CAGACAUGCAGCAGCUGUUU | 20 | 2440 |
| HSV1-UL19-2119 | - | CAGCAGCUGUUUUUGAACGC | 20 | 2441 |
| HSV1-UL19-290 | - | AGCAGCUGUUUUUGAACGCC | 20 | 676 |
| HSV1-UL19-291 | - | GCAGCUGUUUUUGAACGCCU | 20 | 677 |
| HSV1-UL19-2122 | - | CUGGGGGCAGCGCCUGGCCC | 20 | 2442 |
| HSV1-UL19-2123 | - | GGGCAGCGCCUGGCCCACGG | 20 | 2443 |
| HSV1-UL19-2124 | - | CUGGCCCACGGGCGGGUCCG | 20 | 2444 |
| HSV1-UL19-2125 | - | ACGGGCGGGUCCGAUGGGUC | 20 | 2445 |
| HSV1-UL19-301 | - | CGGGCGGGUCCGAUGGGUCG | 20 | 687 |
| HSV1-UL19-2127 | - | UCGCGGAAGGCCAGAUGACC | 20 | 2446 |
| HSV1-UL19-303 | - | CGCGGAAGGCCAGAUGACCC | 20 | 689 |
| HSV1-UL19-2129 | - | CGACAACGCCAACCUGGCUC | 20 | 2447 |
| HSV1-UL19-2130 | - | ACCCCGCGUUCGACUUCUUU | 20 | 2448 |
| HSV1-UL19-305 | - | CCCCGCGUUCGACUUCUUUG | 20 | 691 |
| HSV1-UL19-306 | - | CCCGCGUUCGACUUCUUUGU | 20 | 692 |
| HSV1-UL19-2133 | - | UCUUUGUGGGGGUGGCCGAC | 20 | 2449 |
| HSV1-UL19-310 | - | CUUUGUGGGGGUGGCCGACG | 20 | 696 |
| HSV1-UL19-2135 | - | GGGUGGCCGACGUGGAGCUG | 20 | 2450 |
| HSV1-UL19-311 | - | GGUGGCCGACGUGGAGCUGC | 20 | 697 |
| HSV1-UL19-312 | - | GUGGCCGACGUGGAGCUGCC | 20 | 698 |
| HSV1-UL19-313 | - | UGGCCGACGUGGAGCUGCCG | 20 | 699 |
| HSV1-UL19-314 | - | GGCCGACGUGGAGCUGCCGG | 20 | 700 |
| HSV1-UL19-315 | - | GCCGACGUGGAGCUGCCGGG | 20 | 701 |
| HSV1-UL19-316 | - | CCGACGUGGAGCUGCCGGGG | 20 | 702 |
| HSV1-UL19-2142 | - | GGGACGUUCCCCCGGCCGGC | 20 | 2451 |
| HSV1-UL19-320 | - | GGACGUUCCCCCGGCCGGCC | 20 | 706 |
| HSV1-UL19-321 | - | GACGUUCCCCCGGCCGGCCC | 20 | 707 |
| HSV1-UL19-322 | - | ACGUUCCCCCGGCCGGCCCG | 20 | 708 |
| HSV1-UL19-323 | - | CGUUCCCCCGGCCGGCCCGG | 20 | 709 |
| HSV1-UL19-2147 | - | UCCAGGCCACCUGGCGCGUG | 20 | 2452 |
| HSV1-UL19-2148 | - | GCGCUAUGUCCGGCGGCGUU | 20 | 2453 |
| HSV1-UL19-331 | - | CGCUAUGUCCGGCGGCGUUC | 20 | 717 |
| HSV1-UL19-2150 | - | CCGGCGGCGUUCCGGGACGC | 20 | 2454 |
| HSV1-UL19-333 | - | CGGCGGCGUUCCGGGACGCC | 20 | 719 |
| HSV1-UL19-2152 | - | CGUUCCGGGACGCCCGGGGC | 20 | 2455 |
| HSV1-UL19-336 | - | GUUCCGGGACGCCCGGGGCC | 20 | 722 |
| HSV1-UL19-2154 | - | GGGACGCCCGGGGCCUGGAG | 20 | 2456 |
| HSV1-UL19-337 | - | GGACGCCCGGGGCCUGGAGC | 20 | 723 |
| HSV1-UL19-338 | - | GACGCCCGGGGCCUGGAGCU | 20 | 724 |
| HSV1-UL19-2157 | - | CCCGGGGCCUGGAGCUGGGG | 20 | 2457 |
| HSV1-UL19-341 | - | CCGGGGCCUGGAGCUGGGGG | 20 | 727 |
| HSV1-UL19-2159 | - | CGCCACCAUCGCCGCCGUUC | 20 | 2458 |
| HSV1-UL19-344 | - | GCCACCAUCGCCGCCGUUCG | 20 | 730 |
| HSV1-UL19-2161 | - | GCAGGCCGCCAUACACGGCA | 20 | 2459 |
| HSV1-UL19-2162 | - | GUCCAGUGCAUCACCAGCUA | 20 | 2460 |
| HSV1-UL19-354 | - | UCCAGUGCAUCACCAGCUAC | 20 | 740 |
| HSV1-UL19-2164 | - | ACACGCGGUGCGCGGCGUUC | 20 | 2461 |
| HSV1-UL19-2165 | - | CUCGUACGUCGUGACCUACC | 20 | 2462 |
| HSV1-UL19-358 | - | UCGUACGUCGUGACCUACCU | 20 | 744 |
| HSV1-UL19-359 | - | CGUACGUCGUGACCUACCUC | 20 | 745 |
| HSV1-UL19-360 | - | GUACGUCGUGACCUACCUCG | 20 | 746 |
| HSV1-UL19-361 | - | UACGUCGUGACCUACCUCGG | 20 | 747 |
| HSV1-UL19-2170 | - | CUACCUCGGGGGAGACCUUC | 20 | 2463 |
| HSV1-UL19-2171 | - | ACCUCGGGGGAGACCUUCCC | 20 | 2464 |
| HSV1-UL19-362 | - | CCUCGGGGGAGACCUUCCCG | 20 | 748 |
| HSV1-UL19-2173 | - | GAGGAGUGCAUGGCCGUGUA | 20 | 2465 |
| HSV1-UL19-364 | - | AGGAGUGCAUGGCCGUGUAC | 20 | 750 |
| HSV1-UL19-2175 | - | CCGGGACCUGGUGGCGCACG | 20 | 2466 |
| HSV1-UL19-2176 | - | AUGACUUUACCCUGACCGGC | 20 | 2467 |
| HSV1-UL19-372 | - | UGACUUUACCCUGACCGGCC | 20 | 758 |
| HSV1-UL19-2178 | - | UUACCCUGACCGGCCCGGAG | 20 | 2468 |
| HSV1-UL19-2179 | - | CCUGACCGGCCCGGAGCUGG | 20 | 2469 |
| HSV1-UL19-2180 | - | GCUGGGCGGGCAGGCGCAAG | 20 | 2470 |
| HSV1-UL19-2181 | - | GCGGGCAGGCGCAAGCCGAG | 20 | 2471 |
| HSV1-UL19-2182 | - | GCCGAGCUGAAUCACCUAAU | 20 | 2472 |
| HSV1-UL19-2183 | - | GCGCUGCUGCCACCCCUCGU | 20 | 2473 |
| HSV1-UL19-379 | - | CGCUGCUGCCACCCCUCGUG | 20 | 765 |
| HSV1-UL19-2185 | - | CCCUGAUGCGGCGCGCGGCC | 20 | 2474 |
| HSV1-UL19-2186 | - | CUGGACCGCCAUCGCGACUG | 20 | 2475 |
| HSV1-UL19-2187 | - | AUCGCGACUGCCGGGUUAGC | 20 | 2476 |
| HSV1-UL19-386 | - | UCGCGACUGCCGGGUUAGCG | 20 | 772 |
| HSV1-UL19-387 | - | CGCGACUGCCGGGUUAGCGC | 20 | 773 |
| HSV1-UL19-388 | - | GCGACUGCCGGGUUAGCGCG | 20 | 774 |
| HSV1-UL19-2191 | - | CGGCAUGUAACGUGGCGACC | 20 | 2477 |
| HSV1-UL19-2192 | - | CUGCUGCACAACACCCAGGC | 20 | 2478 |
| HSV1-UL19-2193 | - | ACAACACCCAGGCCCGAGCC | 20 | 2479 |
| HSV1-UL19-2194 | - | AGGCCCGAGCCGCGGACGCC | 20 | 2480 |
| HSV1-UL19-2195 | - | GCCGCGGAUGACCGGCCGCA | 20 | 2481 |
| HSV1-UL19-400 | - | CCGCGGAUGACCGGCCGCAC | 20 | 786 |
| HSV1-UL19-401 | - | CGCGGAUGACCGGCCGCACC | 20 | 787 |
| HSV1-UL19-402 | - | GCGGAUGACCGGCCGCACCG | 20 | 788 |
| HSV1-UL19-2199 | - | AUGACCGGCCGCACCGGGGG | 20 | 2482 |
| HSV1-UL19-2200 | - | CGGCCGCACCGGGGGGCGGA | 20 | 2483 |
| HSV1-UL19-2201 | - | GUGAUGGUGCCCGCCUUCUC | 20 | 2484 |
| HSV1-UL19-408 | - | UGAUGGUGCCCGCCUUCUCG | 20 | 794 |
| HSV1-UL19-2203 | - | CGCGGGGCCGCUGCUGCACC | 20 | 2485 |
| HSV1-UL19-411 | - | GCGGGGCCGCUGCUGCACCG | 20 | 797 |
| HSV1-UL19-412 | - | CGGGGCCGCUGCUGCACCGC | 20 | 798 |
| HSV1-UL19-2206 | - | ACCGCGUAUACGCCACCCUG | 20 | 2486 |
| HSV1-UL19-2207 | - | CCCUGCAGAACAUGGUGGUC | 20 | 2487 |
| HSV1-UL19-417 | - | CCUGCAGAACAUGGUGGUCC | 20 | 803 |
| HSV1-UL19-2209 | - | GGUCCCGGAGAUCGCCCCCG | 20 | 2488 |
| HSV1-UL19-2210 | - | UCCCGGAGAUCGCCCCCGGC | 20 | 2489 |
| HSV1-UL19-419 | - | CCCGGAGAUCGCCCCCGGCG | 20 | 805 |
| HSV1-UL19-2212 | - | AGUGCCCCAGCGACCCCGUG | 20 | 2490 |
| HSV1-UL19-2213 | - | GGUCAACGCCAUGUUUCACA | 20 | 2491 |
| HSV1-UL19-2214 | - | UUCACAACGGGCGCGUGGUA | 20 | 2492 |
| HSV1-UL19-2215 | - | CAACGGGCGCGUGGUAGUGG | 20 | 2493 |
| HSV1-UL19-2216 | - | GGUGCUGGCCCACAACAUGG | 20 | 2494 |
| HSV1-UL19-2217 | - | UCUGCUCGGCGGCGCCCGAC | 20 | 2495 |
| HSV1-UL19-2218 | - | CACCAACAUGCGCAUAUUCG | 20 | 2496 |
| HSV1-UL19-439 | - | ACCAACAUGCGCAUAUUCGA | 20 | 825 |
| HSV1-UL19-2220 | - | AUUCGACGGGGCGUUGCACG | 20 | 2497 |
| HSV1-UL19-442 | - | UUCGACGGGGCGUUGCACGC | 20 | 828 |
| HSV1-UL19-2222 | - | UGCUGAUGGCCCCCCAGCAU | 20 | 2498 |
| HSV1-UL19-2223 | - | CCCCGUCCACGCGCUGUUCG | 20 | 2499 |
| HSV1-UL19-446 | - | CCCGUCCACGCGCUGUUCGC | 20 | 832 |
| HSV1-UL19-2225 | - | UCGCCGGGGCCGACCACGUG | 20 | 2500 |
| HSV1-UL19-2226 | - | UCCCCCUGGUCCCCCCGGCU | 20 | 2501 |
| HSV1-UL19-455 | - | CCCCCUGGUCCCCCCGGCUC | 20 | 841 |
| HSV1-UL19-456 | - | CCCCUGGUCCCCCCGGCUCU | 20 | 842 |
| HSV1-UL19-2229 | - | GCCCGUCGUGCAGCACGUCC | 20 | 2502 |
| HSV1-UL19-2230 | - | CCGUCGUGCAGCACGUCCGC | 20 | 2503 |
| HSV1-UL19-2231 | - | GCACGUCCGCGAGAGCGCGG | 20 | 2504 |
| HSV1-UL19-460 | - | CACGUCCGCGAGAGCGCGGC | 20 | 846 |
| HSV1-UL19-461 | - | ACGUCCGCGAGAGCGCGGCC | 20 | 847 |
| HSV1-UL19-462 | - | CGUCCGCGAGAGCGCGGCCG | 20 | 848 |
| HSV1-UL19-2235 | - | UCCGCGAGAGCGCGGCCGGG | 20 | 2505 |
| HSV1-UL19-2236 | - | CGCUGACCUACGCGCUCAUG | 20 | 2506 |
| HSV1-UL19-464 | - | GCUGACCUACGCGCUCAUGG | 20 | 850 |
| HSV1-UL19-2238 | - | CCUUGCAUCAUCAGCUCAAG | 20 | 2507 |
| HSV1-UL19-2239 | - | GCUCAAGACGGGCCUCCAUC | 20 | 2508 |
| HSV1-UL19-2240 | - | GACGGGCCUCCAUCCCGGGU | 20 | 2509 |
| HSV1-UL19-2241 | - | UUGGGUUCACCGUCGUCCGA | 20 | 2510 |
| HSV1-UL19-2242 | - | CCGACAGGACCGCUUUGUGA | 20 | 2511 |
| HSV1-UL19-2243 | - | GACAGGACCGCUUUGUGACU | 20 | 2512 |
| HSV1-UL19-2244 | - | UGACUGAGAACGUGCUGUUC | 20 | 2513 |
| HSV1-UL19-475 | - | GACUGAGAACGUGCUGUUCU | 20 | 861 |
| HSV1-UL19-2246 | - | UGCUGUUCUCGGAGCGCGCG | 20 | 2514 |
| HSV1-UL19-476 | - | GCUGUUCUCGGAGCGCGCGU | 20 | 862 |
| HSV1-UL19-2248 | - | GCGCGUCGGAGGCGUACUUC | 20 | 2515 |
| HSV1-UL19-2249 | - | CCAGCUCCAGGUGGCCCGGC | 20 | 2516 |
| HSV1-UL19-2250 | - | GGUGGCCCGGCACGAAACUG | 20 | 2517 |
| HSV1-UL19-484 | - | GUGGCCCGGCACGAAACUGG | 20 | 870 |
| HSV1-UL19-485 | - | UGGCCCGGCACGAAACUGGC | 20 | 871 |
| HSV1-UL19-486 | - | GGCCCGGCACGAAACUGGCG | 20 | 872 |
| HSV1-UL19-487 | - | GCCCGGCACGAAACUGGCGG | 20 | 873 |
| HSV1-UL19-2255 | - | UCACGCUCACCCAGCCGCGC | 20 | 2518 |
| HSV1-UL19-2256 | - | UCACCCAGCCGCGCGCGAAC | 20 | 2519 |
| HSV1-UL19-2257 | - | AGCCGCGCGCGAACGUGGAC | 20 | 2520 |
| HSV1-UL19-2258 | - | GCGCGAACGUGGACCUGGGC | 20 | 2521 |
| HSV1-UL19-2259 | - | UCCGCAACCCCGUCACCGAC | 20 | 2522 |
| HSV1-UL19-2260 | - | ACCUUCCCCAAAACUUUUAC | 20 | 2523 |
| HSV1-UL19-2261 | - | CCAAAACUUUUACCUGGGCC | 20 | 2524 |
| HSV1-UL19-501 | - | CAAAACUUUUACCUGGGCCG | 20 | 887 |
| HSV1-UL19-2263 | - | GCCGCGGGGCUCCCCCUCUC | 20 | 2525 |
| HSV1-UL19-2264 | - | AACGCGGCAGCCGUGUACCU | 20 | 2526 |
| HSV1-UL19-506 | - | ACGCGGCAGCCGUGUACCUG | 20 | 892 |
| HSV1-UL19-2266 | - | ACCUGCGGAACGCGGUCGUG | 20 | 2527 |
| HSV1-UL19-509 | - | CCUGCGGAACGCGGUCGUGG | 20 | 895 |
| HSV1-UL19-510 | - | CUGCGGAACGCGGUCGUGGC | 20 | 896 |
| HSV1-UL19-2269 | - | CGGUCGUGGCGGGAAACCGC | 20 | 2528 |
| HSV1-UL19-511 | - | GGUCGUGGCGGGAAACCGCC | 20 | 897 |
| HSV1-UL19-2271 | - | GGCCCAGCCCGUCCCCGUGU | 20 | 2529 |
| HSV1-UL19-2272 | - | CGCCCAGGUGCCGCGGCGCG | 20 | 2530 |
| HSV1-UL19-519 | - | GCCCAGGUGCCGCGGCGCGC | 20 | 905 |
| HSV1-UL19-2274 | - | AGGUGCCGCGGCGCGCAGGG | 20 | 2531 |
| HSV1-UL19-2275 | - | GCGCAGGGAUGGACCACGGC | 20 | 2532 |
| HSV1-UL19-2276 | - | CCACGGCCAGGACGCCGUGU | 20 | 2533 |
| HSV1-UL19-2277 | - | UUCCGCCGGCCCUGCAACCC | 20 | 2534 |
| HSV1-UL19-525 | - | UCCGCCGGCCCUGCAACCCC | 20 | 911 |
| HSV1-UL19-526 | - | CCGCCGGCCCUGCAACCCCC | 20 | 912 |
| HSV1-UL19-2280 | - | CAACCCCCGGGGACGCGCCG | 20 | 2535 |
| HSV1-UL19-528 | - | AACCCCCGGGGACGCGCCGC | 20 | 914 |
| HSV1-UL19-529 | - | ACCCCCGGGGACGCGCCGCC | 20 | 915 |
| HSV1-UL19-2283 | - | GCGCCGCCGGGGGCGUUUAC | 20 | 2536 |
| HSV1-UL19-532 | - | CGCCGCCGGGGGCGUUUACG | 20 | 918 |
| HSV1-UL19-533 | - | GCCGCCGGGGGCGUUUACGC | 20 | 919 |
| HSV1-UL19-534 | - | CCGCCGGGGGCGUUUACGCG | 20 | 920 |
| HSV1-UL19-2287 | - | GGGGCGUUUACGCGGGGGAC | 20 | 2537 |
| HSV1-UL19-536 | - | GGGCGUUUACGCGGGGGACA | 20 | 922 |
| HSV1-UL19-2289 | - | GCGUUUACGCGGGGGACAAG | 20 | 2538 |
| HSV1-UL19-537 | - | CGUUUACGCGGGGGACAAGG | 20 | 923 |
| HSV1-UL19-538 | - | GUUUACGCGGGGGACAAGGA | 20 | 924 |
| HSV1-UL19-539 | - | UUUACGCGGGGGACAAGGAG | 20 | 925 |
| HSV1-UL19-2293 | - | CCCUCAUGUACGACCACGGC | 20 | 2539 |
| HSV1-UL19-2294 | - | CACGGCCAGAGCGACCCGUC | 20 | 2540 |
| HSV1-UL19-2295 | - | UUCGCGGCCACGGCCAACCC | 20 | 2541 |
| HSV1-UL19-2296 | - | GGCGUCGCAGCGAUUUUCGU | 20 | 2542 |
| HSV1-UL19-548 | - | GCGUCGCAGCGAUUUUCGUA | 20 | 934 |
| HSV1-UL19-549 | - | CGUCGCAGCGAUUUUCGUAC | 20 | 935 |
| HSV1-UL19-2299 | - | GUACGGGGACCUGCUCUAUA | 20 | 2543 |
| HSV1-UL19-551 | - | UACGGGGACCUGCUCUAUAA | 20 | 937 |
| HSV1-UL19-2301 | - | UAACGGGGCCUACCACCUCA | 20 | 2544 |
| HSV1-UL19-554 | - | AACGGGGCCUACCACCUCAA | 20 | 940 |
| HSV1-UL19-2303 | - | UCGCCGCCAAACAUCGCUGC | 20 | 2545 |
| HSV1-UL19-559 | - | CGCCGCCAAACAUCGCUGCC | 20 | 945 |
| HSV1-UL19-2305 | - | GCUGCCUGGAGCGCCUGAUC | 20 | 2546 |
| HSV1-UL19-560 | - | CUGCCUGGAGCGCCUGAUCG | 20 | 946 |
| HSV1-UL19-2307 | - | UGGAGCGCCUGAUCGUGGAG | 20 | 2547 |
| HSV1-UL19-2308 | - | ACGUACAGUUUAAGCGCCCC | 20 | 2548 |
| HSV1-UL19-566 | - | CGUACAGUUUAAGCGCCCCC | 20 | 952 |
| HSV1-UL19-2310 | - | UAAGCGCCCCCCGGGGUGCC | 20 | 2549 |
| HSV1-UL19-2311 | - | CCCCGGGGUGCCGCGAACUC | 20 | 2550 |
| HSV1-UL19-569 | - | CCCGGGGUGCCGCGAACUCG | 20 | 955 |
| HSV1-UL19-2313 | - | CGGGGUGCCGCGAACUCGUG | 20 | 2551 |
| HSV1-UL19-2314 | - | AGGACCCGUGUGGCCUGUUU | 20 | 2552 |
| HSV1-UL19-572 | - | GGACCCGUGUGGCCUGUUUC | 20 | 958 |
| HSV1-UL19-2316 | - | CCUCCUCCGCAGUGCCCGCA | 20 | 2553 |
| HSV1-UL19-574 | - | CUCCUCCGCAGUGCCCGCAA | 20 | 960 |
| HSV1-UL19-575 | - | UCCUCCGCAGUGCCCGCAAC | 20 | 961 |
| HSV1-UL19-576 | - | CCUCCGCAGUGCCCGCAACG | 20 | 962 |
| HSV1-UL19-2320 | - | CGCAACGGGGAAGCCCACGC | 20 | 2554 |
| HSV1-UL19-577 | - | GCAACGGGGAAGCCCACGCG | 20 | 963 |
| HSV1-UL19-578 | - | CAACGGGGAAGCCCACGCGC | 20 | 964 |
| HSV1-UL19-2323 | - | UCUAUGACGCAUCCCCGCUC | 20 | 2555 |
| HSV1-UL19-579 | - | CUAUGACGCAUCCCCGCUCA | 20 | 965 |
| HSV1-UL19-582 | - | AACGUCCAGGCCGUCCU | 17 | 968 |
| HSV1-UL19-2326 | - | GGCCGUCCUCGGGGCGU | 17 | 2556 |
| HSV1-UL19-2327 | - | AGAUGCUGCACGUGCUG | 17 | 2557 |
| HSV1-UL19-587 | - | GAUGCUGCACGUGCUGC | 17 | 973 |
| HSV1-UL19-2329 | - | UGCCGAUGCAACGAUAC | 17 | 2558 |
| HSV1-UL19-2330 | - | AACGGCCGCCUGGCCAC | 17 | 2559 |
| HSV1-UL19-2331 | - | CUGGCCACCAGGGUGGC | 17 | 2560 |
| HSV1-UL19-2332 | - | CCGGGCGACCCUGGUCG | 17 | 2561 |
| HSV1-UL19-2333 | - | CUGGUCGCCGAGCUAAA | 17 | 2562 |
| HSV1-UL19-2334 | - | GCUAAAGCGAAGCUUCU | 17 | 2563 |
| HSV1-UL19-2335 | - | AGCGAAGCUUCUGCGAG | 17 | 2564 |
| HSV1-UL19-2336 | - | GCGAGACGAGCUUUUUC | 17 | 2565 |
| HSV1-UL19-2337 | - | GCUUUUUCCUGGGCAAG | 17 | 2566 |
| HSV1-UL19-2338 | - | GGCAAGGCGGGCCACCG | 17 | 2567 |
| HSV1-UL19-604 | - | GCAAGGCGGGCCACCGC | 17 | 990 |
| HSV1-UL19-605 | - | CAAGGCGGGCCACCGCC | 17 | 991 |
| HSV1-UL19-2341 | - | CCACCGCCGGGAGGCCG | 17 | 2568 |
| HSV1-UL19-2342 | - | CCGUCGAGGCCUGGCUC | 17 | 2569 |
| HSV1-UL19-2343 | - | GACGCAUGCCGACACGC | 17 | 2570 |
| HSV1-UL19-2344 | - | GCGCGGGCGGCCGGUCG | 17 | 2571 |
| HSV1-UL19-616 | - | CGCGGGCGGCCGGUCGA | 17 | 1002 |
| HSV1-UL19-2346 | - | GCCUCCUGCAGUCCUUC | 17 | 2572 |
| HSV1-UL19-2347 | - | UGCAGUCCUUCCUGAAA | 17 | 2573 |
| HSV1-UL19-619 | - | GCAGUCCUUCCUGAAAG | 17 | 1005 |
| HSV1-UL19-2349 | - | AGUCCUUCCUGAAAGUG | 17 | 2574 |
| HSV1-UL19-2350 | - | CCUGAAAGUGGAGGACA | 17 | 2575 |
| HSV1-UL19-2351 | - | CGUGCCGGUGACGUACG | 17 | 2576 |
| HSV1-UL19-2352 | - | CGUACGGCGAGAUGGUC | 17 | 2577 |
| HSV1-UL19-2353 | - | CGGCGAGAUGGUCCUGA | 17 | 2578 |
| HSV1-UL19-624 | - | GGCGAGAUGGUCCUGAA | 17 | 1010 |
| HSV1-UL19-2355 | - | UGGUCACGGCGCUCGUG | 17 | 2579 |
| HSV1-UL19-2356 | - | GUGAUGGGCAAGGCCGU | 17 | 2580 |
| HSV1-UL19-2357 | - | GCAAGGCCGUGCGAAGU | 17 | 2581 |
| HSV1-UL19-2358 | - | UGCGAAGUCUGGACGAC | 17 | 2582 |
| HSV1-UL19-2359 | - | ACGUGGGCCGCCACCUG | 17 | 2583 |
| HSV1-UL19-635 | - | CGUGGGCCGCCACCUGC | 17 | 1021 |
| HSV1-UL19-2361 | - | GCCACCUGCUGGAGAUG | 17 | 2584 |
| HSV1-UL19-636 | - | CCACCUGCUGGAGAUGC | 17 | 1022 |
| HSV1-UL19-2363 | - | ACCUGCUGGAGAUGCAG | 17 | 2585 |
| HSV1-UL19-637 | - | CCUGCUGGAGAUGCAGG | 17 | 1023 |
| HSV1-UL19-2365 | - | AGGAGGAGCAGCUCGAC | 17 | 2586 |
| HSV1-UL19-2366 | - | ACCUGAACCGGCAGACG | 17 | 2587 |
| HSV1-UL19-2367 | - | GAACCGGCAGACGCUGG | 17 | 2588 |
| HSV1-UL19-2368 | - | GCAGACGCUGGACGAGC | 17 | 2589 |
| HSV1-UL19-2369 | - | AGACGCUGGACGAGCUC | 17 | 2590 |
| HSV1-UL19-2370 | - | AGACGACGCGCGUGCGC | 17 | 2591 |
| HSV1-UL19-2371 | - | GGAUCUGGUGUCCAUCG | 17 | 2592 |
| HSV1-UL19-2372 | - | AUCUGGUGUCCAUCGGC | 17 | 2593 |
| HSV1-UL19-2373 | - | GCGAGAAGCUGGUCUUU | 17 | 2594 |
| HSV1-UL19-644 | - | CGAGAAGCUGGUCUUUC | 17 | 1030 |
| HSV1-UL19-2375 | - | UGGUCUUUCUGGAGGCC | 17 | 2595 |
| HSV1-UL19-646 | - | GGUCUUUCUGGAGGCCC | 17 | 1032 |
| HSV1-UL19-2377 | - | UCUUUCUGGAGGCCCUG | 17 | 2596 |
| HSV1-UL19-2378 | - | ACGUUCCCUACCCCCUG | 17 | 2597 |
| HSV1-UL19-2379 | - | ACCCCCUGGUGGGCGCC | 17 | 2598 |
| HSV1-UL19-2380 | - | UGACGUUCGUCCUGCCC | 17 | 2599 |
| HSV1-UL19-2381 | - | GCCUGUUCAAUCCGGUC | 17 | 2600 |
| HSV1-UL19-654 | - | CCUGUUCAAUCCGGUCA | 17 | 1040 |
| HSV1-UL19-2383 | - | ACGGUUUGCCGCGCACG | 17 | 2601 |
| HSV1-UL19-656 | - | CGGUUUGCCGCGCACGC | 17 | 1042 |
| HSV1-UL19-657 | - | GGUUUGCCGCGCACGCC | 17 | 1043 |
| HSV1-UL19-2386 | - | UCCCCGCCCCCGGCCAC | 17 | 2602 |
| HSV1-UL19-2387 | - | CCCCGCCAGCUGUUUUU | 17 | 2603 |
| HSV1-UL19-661 | - | CCCGCCAGCUGUUUUUU | 17 | 1047 |
| HSV1-UL19-662 | - | CCGCCAGCUGUUUUUUU | 17 | 1048 |
| HSV1-UL19-663 | - | CGCCAGCUGUUUUUUUG | 17 | 1049 |
| HSV1-UL19-664 | - | GCCAGCUGUUUUUUUGG | 17 | 1050 |
| HSV1-UL19-2392 | - | AGCUGUUUUUUUGGGGG | 17 | 2604 |
| HSV1-UL19-2393 | - | AGGUGCUGCGCCUGUCU | 17 | 2605 |
| HSV1-UL19-667 | - | GGUGCUGCGCCUGUCUC | 17 | 1053 |
| HSV1-UL19-2395 | - | GUCUCUGGAACACGCGA | 17 | 2606 |
| HSV1-UL19-668 | - | UCUCUGGAACACGCGAU | 17 | 1054 |
| HSV1-UL19-2397 | - | UGUGCCACCCUUCGCUG | 17 | 2607 |
| HSV1-UL19-2398 | - | GAACGUUGACGCGGCGG | 17 | 2608 |
| HSV1-UL19-672 | - | AACGUUGACGCGGCGGU | 17 | 1058 |
| HSV1-UL19-673 | - | ACGUUGACGCGGCGGUC | 17 | 1059 |
| HSV1-UL19-2401 | - | CCUUAACCGCGACCCCG | 17 | 2609 |
| HSV1-UL19-2402 | - | CGAAGCCGCCAAUCCGU | 17 | 2610 |
| HSV1-UL19-676 | - | GAAGCCGCCAAUCCGUA | 17 | 1062 |
| HSV1-UL19-2404 | - | ACAUGCAGCAGCUGUUU | 17 | 2611 |
| HSV1-UL19-2405 | - | CAGCUGUUUUUGAACGC | 17 | 2612 |
| HSV1-UL19-683 | - | AGCUGUUUUUGAACGCC | 17 | 1069 |
| HSV1-UL19-684 | - | GCUGUUUUUGAACGCCU | 17 | 1070 |
| HSV1-UL19-2408 | - | GGGGCAGCGCCUGGCCC | 17 | 2613 |
| HSV1-UL19-2409 | - | CAGCGCCUGGCCCACGG | 17 | 2614 |
| HSV1-UL19-2410 | - | GCCCACGGGCGGGUCCG | 17 | 2615 |
| HSV1-UL19-2411 | - | GGCGGGUCCGAUGGGUC | 17 | 2616 |
| HSV1-UL19-694 | - | GCGGGUCCGAUGGGUCG | 17 | 1080 |
| HSV1-UL19-2413 | - | CGGAAGGCCAGAUGACC | 17 | 2617 |
| HSV1-UL19-696 | - | GGAAGGCCAGAUGACCC | 17 | 1082 |
| HSV1-UL19-2415 | - | CAACGCCAACCUGGCUC | 17 | 2618 |
| HSV1-UL19-2416 | - | CCGCGUUCGACUUCUUU | 17 | 2619 |
| HSV1-UL19-698 | - | CGCGUUCGACUUCUUUG | 17 | 1084 |
| HSV1-UL19-699 | - | GCGUUCGACUUCUUUGU | 17 | 1085 |
| HSV1-UL19-2419 | - | UUGUGGGGGUGGCCGAC | 17 | 2620 |
| HSV1-UL19-703 | - | UGUGGGGGUGGCCGACG | 17 | 1089 |
| HSV1-UL19-2421 | - | UGGCCGACGUGGAGCUG | 17 | 2621 |
| HSV1-UL19-704 | - | GGCCGACGUGGAGCUGC | 17 | 1090 |
| HSV1-UL19-705 | - | GCCGACGUGGAGCUGCC | 17 | 1091 |
| HSV1-UL19-706 | - | CCGACGUGGAGCUGCCG | 17 | 1092 |
| HSV1-UL19-707 | - | CGACGUGGAGCUGCCGG | 17 | 1093 |
| HSV1-UL19-708 | - | GACGUGGAGCUGCCGGG | 17 | 1094 |
| HSV1-UL19-709 | - | ACGUGGAGCUGCCGGGG | 17 | 1095 |
| HSV1-UL19-2428 | - | ACGUUCCCCCGGCCGGC | 17 | 2622 |
| HSV1-UL19-713 | - | CGUUCCCCCGGCCGGCC | 17 | 1099 |
| HSV1-UL19-714 | - | GUUCCCCCGGCCGGCCC | 17 | 1100 |
| HSV1-UL19-715 | - | UUCCCCCGGCCGGCCCG | 17 | 1101 |
| HSV1-UL19-716 | - | UCCCCCGGCCGGCCCGG | 17 | 1102 |
| HSV1-UL19-2433 | - | AGGCCACCUGGCGCGUG | 17 | 2623 |
| HSV1-UL19-2434 | - | CUAUGUCCGGCGGCGUU | 17 | 2624 |
| HSV1-UL19-724 | - | UAUGUCCGGCGGCGUUC | 17 | 1110 |
| HSV1-UL19-2436 | - | GCGGCGUUCCGGGACGC | 17 | 2625 |
| HSV1-UL19-726 | - | CGGCGUUCCGGGACGCC | 17 | 1112 |
| HSV1-UL19-2438 | - | UCCGGGACGCCCGGGGC | 17 | 2626 |
| HSV1-UL19-729 | - | CCGGGACGCCCGGGGCC | 17 | 1115 |
| HSV1-UL19-2440 | - | ACGCCCGGGGCCUGGAG | 17 | 2627 |
| HSV1-UL19-730 | - | CGCCCGGGGCCUGGAGC | 17 | 1116 |
| HSV1-UL19-731 | - | GCCCGGGGCCUGGAGCU | 17 | 1117 |
| HSV1-UL19-2443 | - | GGGGCCUGGAGCUGGGG | 17 | 2628 |
| HSV1-UL19-734 | - | GGGCCUGGAGCUGGGGG | 17 | 1120 |
| HSV1-UL19-2445 | - | CACCAUCGCCGCCGUUC | 17 | 2629 |
| HSV1-UL19-737 | - | ACCAUCGCCGCCGUUCG | 17 | 1123 |
| HSV1-UL19-2447 | - | GGCCGCCAUACACGGCA | 17 | 2630 |
| HSV1-UL19-2448 | - | CAGUGCAUCACCAGCUA | 17 | 2631 |
| HSV1-UL19-747 | - | AGUGCAUCACCAGCUAC | 17 | 1133 |
| HSV1-UL19-2450 | - | CGCGGUGCGCGGCGUUC | 17 | 2632 |
| HSV1-UL19-2451 | - | GUACGUCGUGACCUACC | 17 | 2633 |
| HSV1-UL19-751 | - | UACGUCGUGACCUACCU | 17 | 1137 |
| HSV1-UL19-752 | - | ACGUCGUGACCUACCUC | 17 | 1138 |
| HSV1-UL19-753 | - | CGUCGUGACCUACCUCG | 17 | 1139 |
| HSV1-UL19-754 | - | GUCGUGACCUACCUCGG | 17 | 1140 |
| HSV1-UL19-2456 | - | CCUCGGGGGAGACCUUC | 17 | 2634 |
| HSV1-UL19-2457 | - | UCGGGGGAGACCUUCCC | 17 | 2635 |
| HSV1-UL19-755 | - | CGGGGGAGACCUUCCCG | 17 | 1141 |
| HSV1-UL19-2459 | - | GAGUGCAUGGCCGUGUA | 17 | 2636 |
| HSV1-UL19-757 | - | AGUGCAUGGCCGUGUAC | 17 | 1143 |
| HSV1-UL19-2461 | - | GGACCUGGUGGCGCACG | 17 | 2637 |
| HSV1-UL19-2462 | - | ACUUUACCCUGACCGGC | 17 | 2638 |
| HSV1-UL19-765 | - | CUUUACCCUGACCGGCC | 17 | 1151 |
| HSV1-UL19-2464 | - | CCCUGACCGGCCCGGAG | 17 | 2639 |
| HSV1-UL19-2465 | - | GACCGGCCCGGAGCUGG | 17 | 2640 |
| HSV1-UL19-2466 | - | GGGCGGGCAGGCGCAAG | 17 | 2641 |
| HSV1-UL19-2467 | - | GGCAGGCGCAAGCCGAG | 17 | 2642 |
| HSV1-UL19-2468 | - | GAGCUGAAUCACCUAAU | 17 | 2643 |
| HSV1-UL19-2469 | - | CUGCUGCCACCCCUCGU | 17 | 2644 |
| HSV1-UL19-772 | - | UGCUGCCACCCCUCGUG | 17 | 1158 |
| HSV1-UL19-2471 | - | UGAUGCGGCGCGCGGCC | 17 | 2645 |
| HSV1-UL19-2472 | - | GACCGCCAUCGCGACUG | 17 | 2646 |
| HSV1-UL19-2473 | - | GCGACUGCCGGGUUAGC | 17 | 2647 |
| HSV1-UL19-779 | - | CGACUGCCGGGUUAGCG | 17 | 1165 |
| HSV1-UL19-780 | - | GACUGCCGGGUUAGCGC | 17 | 1166 |
| HSV1-UL19-781 | - | ACUGCCGGGUUAGCGCG | 17 | 1167 |
| HSV1-UL19-2477 | - | CAUGUAACGUGGCGACC | 17 | 2648 |
| HSV1-UL19-2478 | - | CUGCACAACACCCAGGC | 17 | 2649 |
| HSV1-UL19-2479 | - | ACACCCAGGCCCGAGCC | 17 | 2650 |
| HSV1-UL19-2480 | - | CCCGAGCCGCGGACGCC | 17 | 2651 |
| HSV1-UL19-2481 | - | GCGGAUGACCGGCCGCA | 17 | 2652 |
| HSV1-UL19-793 | - | CGGAUGACCGGCCGCAC | 17 | 1179 |
| HSV1-UL19-794 | - | GGAUGACCGGCCGCACC | 17 | 1180 |
| HSV1-UL19-795 | - | GAUGACCGGCCGCACCG | 17 | 1181 |
| HSV1-UL19-2485 | - | ACCGGCCGCACCGGGGG | 17 | 2653 |
| HSV1-UL19-2486 | - | CCGCACCGGGGGGCGGA | 17 | 2654 |
| HSV1-UL19-2487 | - | AUGGUGCCCGCCUUCUC | 17 | 2655 |
| HSV1-UL19-801 | - | UGGUGCCCGCCUUCUCG | 17 | 1187 |
| HSV1-UL19-2489 | - | GGGGCCGCUGCUGCACC | 17 | 2656 |
| HSV1-UL19-804 | - | GGGCCGCUGCUGCACCG | 17 | 1190 |
| HSV1-UL19-805 | - | GGCCGCUGCUGCACCGC | 17 | 1191 |
| HSV1-UL19-2492 | - | GCGUAUACGCCACCCUG | 17 | 2657 |
| HSV1-UL19-2493 | - | UGCAGAACAUGGUGGUC | 17 | 2658 |
| HSV1-UL19-810 | - | GCAGAACAUGGUGGUCC | 17 | 1196 |
| HSV1-UL19-2495 | - | CCCGGAGAUCGCCCCCG | 17 | 2659 |
| HSV1-UL19-2496 | - | CGGAGAUCGCCCCCGGC | 17 | 2660 |
| HSV1-UL19-812 | - | GGAGAUCGCCCCCGGCG | 17 | 1198 |
| HSV1-UL19-2498 | - | GCCCCAGCGACCCCGUG | 17 | 2661 |
| HSV1-UL19-2499 | - | CAACGCCAUGUUUCACA | 17 | 2662 |
| HSV1-UL19-2500 | - | ACAACGGGCGCGUGGUA | 17 | 2663 |
| HSV1-UL19-2501 | - | CGGGCGCGUGGUAGUGG | 17 | 2664 |
| HSV1-UL19-2502 | - | GCUGGCCCACAACAUGG | 17 | 2665 |
| HSV1-UL19-2503 | - | GCUCGGCGGCGCCCGAC | 17 | 2666 |
| HSV1-UL19-2504 | - | CAACAUGCGCAUAUUCG | 17 | 2667 |
| HSV1-UL19-832 | - | AACAUGCGCAUAUUCGA | 17 | 1218 |
| HSV1-UL19-2506 | - | CGACGGGGCGUUGCACG | 17 | 2668 |
| HSV1-UL19-835 | - | GACGGGGCGUUGCACGC | 17 | 1221 |
| HSV1-UL19-2508 | - | UGAUGGCCCCCCAGCAU | 17 | 2669 |
| HSV1-UL19-2509 | - | CGUCCACGCGCUGUUCG | 17 | 2670 |
| HSV1-UL19-839 | - | GUCCACGCGCUGUUCGC | 17 | 1225 |
| HSV1-UL19-2511 | - | CCGGGGCCGACCACGUG | 17 | 2671 |
| HSV1-UL19-2512 | - | CCCUGGUCCCCCCGGCU | 17 | 2672 |
| HSV1-UL19-848 | - | CCUGGUCCCCCCGGCUC | 17 | 1234 |
| HSV1-UL19-849 | - | CUGGUCCCCCCGGCUCU | 17 | 1235 |
| HSV1-UL19-2515 | - | CGUCGUGCAGCACGUCC | 17 | 2673 |
| HSV1-UL19-2516 | - | UCGUGCAGCACGUCCGC | 17 | 2674 |
| HSV1-UL19-2517 | - | CGUCCGCGAGAGCGCGG | 17 | 2675 |
| HSV1-UL19-853 | - | GUCCGCGAGAGCGCGGC | 17 | 1239 |
| HSV1-UL19-854 | - | UCCGCGAGAGCGCGGCC | 17 | 1240 |
| HSV1-UL19-855 | - | CCGCGAGAGCGCGGCCG | 17 | 1241 |
| HSV1-UL19-2521 | - | GCGAGAGCGCGGCCGGG | 17 | 2676 |
| HSV1-UL19-2522 | - | UGACCUACGCGCUCAUG | 17 | 2677 |
| HSV1-UL19-857 | - | GACCUACGCGCUCAUGG | 17 | 1243 |
| HSV1-UL19-2524 | - | UGCAUCAUCAGCUCAAG | 17 | 2678 |
| HSV1-UL19-2525 | - | CAAGACGGGCCUCCAUC | 17 | 2679 |
| HSV1-UL19-2526 | - | GGGCCUCCAUCCCGGGU | 17 | 2680 |
| HSV1-UL19-2527 | - | GGUUCACCGUCGUCCGA | 17 | 2681 |
| HSV1-UL19-2528 | - | ACAGGACCGCUUUGUGA | 17 | 2682 |
| HSV1-UL19-2529 | - | AGGACCGCUUUGUGACU | 17 | 2683 |
| HSV1-UL19-2530 | - | CUGAGAACGUGCUGUUC | 17 | 2684 |
| HSV1-UL19-868 | - | UGAGAACGUGCUGUUCU | 17 | 1254 |
| HSV1-UL19-2532 | - | UGUUCUCGGAGCGCGCG | 17 | 2685 |
| HSV1-UL19-869 | - | GUUCUCGGAGCGCGCGU | 17 | 1255 |
| HSV1-UL19-2534 | - | CGUCGGAGGCGUACUUC | 17 | 2686 |
| HSV1-UL19-2535 | - | GCUCCAGGUGGCCCGGC | 17 | 2687 |
| HSV1-UL19-2536 | - | GGCCCGGCACGAAACUG | 17 | 2688 |
| HSV1-UL19-877 | - | GCCCGGCACGAAACUGG | 17 | 1263 |
| HSV1-UL19-878 | - | CCCGGCACGAAACUGGC | 17 | 1264 |
| HSV1-UL19-879 | - | CCGGCACGAAACUGGCG | 17 | 1265 |
| HSV1-UL19-880 | - | CGGCACGAAACUGGCGG | 17 | 1266 |
| HSV1-UL19-2541 | - | CGCUCACCCAGCCGCGC | 17 | 2689 |
| HSV1-UL19-2542 | - | CCCAGCCGCGCGCGAAC | 17 | 2690 |
| HSV1-UL19-2543 | - | CGCGCGCGAACGUGGAC | 17 | 2691 |
| HSV1-UL19-2544 | - | CGAACGUGGACCUGGGC | 17 | 2692 |
| HSV1-UL19-2545 | - | GCAACCCCGUCACCGAC | 17 | 2693 |
| HSV1-UL19-2546 | - | UUCCCCAAAACUUUUAC | 17 | 2694 |
| HSV1-UL19-2547 | - | AAACUUUUACCUGGGCC | 17 | 2695 |
| HSV1-UL19-894 | - | AACUUUUACCUGGGCCG | 17 | 1280 |
| HSV1-UL19-2549 | - | GCGGGGCUCCCCCUCUC | 17 | 2696 |
| HSV1-UL19-2550 | - | GCGGCAGCCGUGUACCU | 17 | 2697 |
| HSV1-UL19-899 | - | CGGCAGCCGUGUACCUG | 17 | 1285 |
| HSV1-UL19-2552 | - | UGCGGAACGCGGUCGUG | 17 | 2698 |
| HSV1-UL19-902 | - | GCGGAACGCGGUCGUGG | 17 | 1288 |
| HSV1-UL19-903 | - | CGGAACGCGGUCGUGGC | 17 | 1289 |
| HSV1-UL19-2555 | - | UCGUGGCGGGAAACCGC | 17 | 2699 |
| HSV1-UL19-904 | - | CGUGGCGGGAAACCGCC | 17 | 1290 |
| HSV1-UL19-2557 | - | CCAGCCCGUCCCCGUGU | 17 | 2700 |
| HSV1-UL19-2558 | - | CCAGGUGCCGCGGCGCG | 17 | 2701 |
| HSV1-UL19-912 | - | CAGGUGCCGCGGCGCGC | 17 | 1298 |
| HSV1-UL19-2560 | - | UGCCGCGGCGCGCAGGG | 17 | 2702 |
| HSV1-UL19-2561 | - | CAGGGAUGGACCACGGC | 17 | 2703 |
| HSV1-UL19-2562 | - | CGGCCAGGACGCCGUGU | 17 | 2704 |
| HSV1-UL19-2563 | - | CGCCGGCCCUGCAACCC | 17 | 2705 |
| HSV1-UL19-918 | - | GCCGGCCCUGCAACCCC | 17 | 1304 |
| HSV1-UL19-919 | - | CCGGCCCUGCAACCCCC | 17 | 1305 |
| HSV1-UL19-2566 | - | CCCCCGGGGACGCGCCG | 17 | 2706 |
| HSV1-UL19-921 | - | CCCCGGGGACGCGCCGC | 17 | 1307 |
| HSV1-UL19-922 | - | CCCGGGGACGCGCCGCC | 17 | 1308 |
| HSV1-UL19-2569 | - | CCGCCGGGGGCGUUUAC | 17 | 2707 |
| HSV1-UL19-925 | - | CGCCGGGGGCGUUUACG | 17 | 1311 |
| HSV1-UL19-926 | - | GCCGGGGGCGUUUACGC | 17 | 1312 |
| HSV1-UL19-927 | - | CCGGGGGCGUUUACGCG | 17 | 1313 |
| HSV1-UL19-2573 | - | GCGUUUACGCGGGGGAC | 17 | 2708 |
| HSV1-UL19-929 | - | CGUUUACGCGGGGGACA | 17 | 1315 |
| HSV1-UL19-2575 | - | UUUACGCGGGGGACAAG | 17 | 2709 |
| HSV1-UL19-930 | - | UUACGCGGGGGACAAGG | 17 | 1316 |
| HSV1-UL19-931 | - | UACGCGGGGGACAAGGA | 17 | 1317 |
| HSV1-UL19-932 | - | ACGCGGGGGACAAGGAG | 17 | 1318 |
| HSV1-UL19-2579 | - | UCAUGUACGACCACGGC | 17 | 2710 |
| HSV1-UL19-2580 | - | GGCCAGAGCGACCCGUC | 17 | 2711 |
| HSV1-UL19-2581 | - | GCGGCCACGGCCAACCC | 17 | 2712 |
| HSV1-UL19-2582 | - | GUCGCAGCGAUUUUCGU | 17 | 2713 |
| HSV1-UL19-941 | - | UCGCAGCGAUUUUCGUA | 17 | 1327 |
| HSV1-UL19-942 | - | CGCAGCGAUUUUCGUAC | 17 | 1328 |
| HSV1-UL19-2585 | - | CGGGGACCUGCUCUAUA | 17 | 2714 |
| HSV1-UL19-944 | - | GGGGACCUGCUCUAUAA | 17 | 1330 |
| HSV1-UL19-2587 | - | CGGGGCCUACCACCUCA | 17 | 2715 |
| HSV1-UL19-947 | - | GGGGCCUACCACCUCAA | 17 | 1333 |
| HSV1-UL19-2589 | - | CCGCCAAACAUCGCUGC | 17 | 2716 |
| HSV1-UL19-952 | - | CGCCAAACAUCGCUGCC | 17 | 1338 |
| HSV1-UL19-2591 | - | GCCUGGAGCGCCUGAUC | 17 | 2717 |
| HSV1-UL19-953 | - | CCUGGAGCGCCUGAUCG | 17 | 1339 |
| HSV1-UL19-2593 | - | AGCGCCUGAUCGUGGAG | 17 | 2718 |
| HSV1-UL19-2594 | - | UACAGUUUAAGCGCCCC | 17 | 2719 |
| HSV1-UL19-959 | - | ACAGUUUAAGCGCCCCC | 17 | 1345 |
| HSV1-UL19-2596 | - | GCGCCCCCCGGGGUGCC | 17 | 2720 |
| HSV1-UL19-2597 | - | CGGGGUGCCGCGAACUC | 17 | 2721 |
| HSV1-UL19-962 | - | GGGGUGCCGCGAACUCG | 17 | 1348 |
| HSV1-UL19-2599 | - | GGUGCCGCGAACUCGUG | 17 | 2722 |
| HSV1-UL19-2600 | - | ACCCGUGUGGCCUGUUU | 17 | 2723 |
| HSV1-UL19-965 | - | CCCGUGUGGCCUGUUUC | 17 | 1351 |
| HSV1-UL19-2602 | - | CCUCCGCAGUGCCCGCA | 17 | 2724 |
| HSV1-UL19-967 | - | CUCCGCAGUGCCCGCAA | 17 | 1353 |
| HSV1-UL19-968 | - | UCCGCAGUGCCCGCAAC | 17 | 1354 |
| HSV1-UL19-969 | - | CCGCAGUGCCCGCAACG | 17 | 1355 |
| HSV1-UL19-2606 | - | AACGGGGAAGCCCACGC | 17 | 2725 |
| HSV1-UL19-970 | - | ACGGGGAAGCCCACGCG | 17 | 1356 |
| HSV1-UL19-971 | - | CGGGGAAGCCCACGCGC | 17 | 1357 |
| HSV1-UL19-2609 | - | AUGACGCAUCCCCGCUC | 17 | 2726 |
| HSV1-UL19-972 | - | UGACGCAUCCCCGCUCA | 17 | 1358 |
| HSV1-UL19-2611 | + | UCAAACGCCCCGAGGACGGC | 20 | 2727 |
| HSV1-UL19-2612 | + | GUGCCGCGCUCAAACGCCCC | 20 | 2728 |
| HSV1-UL19-2613 | + | CCGUGCCGCGCUCAAACGCC | 20 | 2729 |
| HSV1-UL19-983 | + | GCAUCGGCAACAACAAAGCC | 20 | 1369 |
| HSV1-UL19-2615 | + | UGCAUCGGCAACAACAAAGC | 20 | 2730 |
| HSV1-UL19-2616 | + | AGCUCGGCGACCAGGGUCGC | 20 | 2731 |
| HSV1-UL19-2617 | + | CUUCGCUUUAGCUCGGCGAC | 20 | 2732 |
| HSV1-UL19-2618 | + | CCAGGAAAAAGCUCGUCUCG | 20 | 2733 |
| HSV1-UL19-995 | + | GGCGGUGGCCCGCCUUGCCC | 20 | 1381 |
| HSV1-UL19-2620 | + | CGGCGGUGGCCCGCCUUGCC | 20 | 2734 |
| HSV1-UL19-2621 | + | GCGUGGCCGUGGUGAGGUCC | 20 | 2735 |
| HSV1-UL19-2622 | + | CGGAGGGCUGCGUGGCCGUG | 20 | 2736 |
| HSV1-UL19-2623 | + | UCAGACGGGGCACGGCCACG | 20 | 2737 |
| HSV1-UL19-1006 | + | CGUCAGACGGGGCACGGCCA | 20 | 1392 |
| HSV1-UL19-2625 | + | GCGUCAGACGGGGCACGGCC | 20 | 2738 |
| HSV1-UL19-1010 | + | GCGUGUCGGCAUGCGUCAGA | 20 | 1396 |
| HSV1-UL19-2627 | + | CGCGUGUCGGCAUGCGUCAG | 20 | 2739 |
| HSV1-UL19-2628 | + | GUUUGAUCGGGGCGGUGGUG | 20 | 2740 |
| HSV1-UL19-1017 | + | UGCAGGAGGCGCUGUUUGAU | 20 | 1403 |
| HSV1-UL19-2630 | + | CUGCAGGAGGCGCUGUUUGA | 20 | 2741 |
| HSV1-UL19-1019 | + | CCACUUUCAGGAAGGACUGC | 20 | 1405 |
| HSV1-UL19-2632 | + | UCCACUUUCAGGAAGGACUG | 20 | 2742 |
| HSV1-UL19-2633 | + | CGGUGUCCUCCACUUUCAGG | 20 | 2743 |
| HSV1-UL19-1021 | + | CUUCGGUGUCCUCCACUUUC | 20 | 1407 |
| HSV1-UL19-2635 | + | GCUUCGGUGUCCUCCACUUU | 20 | 2744 |
| HSV1-UL19-2636 | + | GUGACCAGGUUGGCCCCGUU | 20 | 2745 |
| HSV1-UL19-2637 | + | UUCGCACGGCCUUGCCCAUC | 20 | 2746 |
| HSV1-UL19-2638 | + | CCAGCGUCUGCCGGUUCAGG | 20 | 2747 |
| HSV1-UL19-2639 | + | GCGUCGUCUGGGGGGCGCUC | 20 | 2748 |
| HSV1-UL19-1036 | + | GCGCGCACGCGCGUCGUCUG | 20 | 1422 |
| HSV1-UL19-1037 | + | CGCGCGCACGCGCGUCGUCU | 20 | 1423 |
| HSV1-UL19-1038 | + | CCGCGCGCACGCGCGUCGUC | 20 | 1424 |
| HSV1-UL19-2643 | + | UCCGCGCGCACGCGCGUCGU | 20 | 2749 |
| HSV1-UL19-2644 | + | GAAAGACCAGCUUCUCGCCG | 20 | 2750 |
| HSV1-UL19-2645 | + | AUGCGCUUCUCCAGGGCCUC | 20 | 2751 |
| HSV1-UL19-2646 | + | GCGGCGUAGAUGCGCUUCUC | 20 | 2752 |
| HSV1-UL19-1045 | + | AUGGCGCCCACCAGGGGGUA | 20 | 1431 |
| HSV1-UL19-1046 | + | CAUGGCGCCCACCAGGGGGU | 20 | 1432 |
| HSV1-UL19-2649 | + | CCAUGGCGCCCACCAGGGGG | 20 | 2753 |
| HSV1-UL19-1049 | + | CAGGUCCAUGGCGCCCACCA | 20 | 1435 |
| HSV1-UL19-1050 | + | UCAGGUCCAUGGCGCCCACC | 20 | 1436 |
| HSV1-UL19-2652 | + | GUCAGGUCCAUGGCGCCCAC | 20 | 2754 |
| HSV1-UL19-2653 | + | UGAACAGGCCCAGGGGCAGG | 20 | 2755 |
| HSV1-UL19-2654 | + | GGAUUGAACAGGCCCAGGGG | 20 | 2756 |
| HSV1-UL19-1056 | + | UGACCGGAUUGAACAGGCCC | 20 | 1442 |
| HSV1-UL19-2656 | + | AUGACCGGAUUGAACAGGCC | 20 | 2757 |
| HSV1-UL19-2657 | + | CAAACCGUUCCAUGACCGGA | 20 | 2758 |
| HSV1-UL19-2658 | + | CGCGGCAAACCGUUCCAUGA | 20 | 2759 |
| HSV1-UL19-1063 | + | GGAUCCGGGUGGCCGGGGGC | 20 | 1449 |
| HSV1-UL19-1064 | + | GGGAUCCGGGUGGCCGGGGG | 20 | 1450 |
| HSV1-UL19-2661 | + | GGGGAUCCGGGUGGCCGGGG | 20 | 2760 |
| HSV1-UL19-1067 | + | GCGCGGGGAUCCGGGUGGCC | 20 | 1453 |
| HSV1-UL19-1068 | + | GGCGCGGGGAUCCGGGUGGC | 20 | 1454 |
| HSV1-UL19-2664 | + | AGGCGCGGGGAUCCGGGUGG | 20 | 2761 |
| HSV1-UL19-2665 | + | GGGCGGGAAGGCGCGGGGAU | 20 | 2762 |
| HSV1-UL19-1073 | + | CUGGCGGGGCGGGAAGGCGC | 20 | 1459 |
| HSV1-UL19-1074 | + | GCUGGCGGGGCGGGAAGGCG | 20 | 1460 |
| HSV1-UL19-2668 | + | AGCUGGCGGGGCGGGAAGGC | 20 | 2763 |
| HSV1-UL19-1076 | + | AAAAAAACAGCUGGCGGGGC | 20 | 1462 |
| HSV1-UL19-1077 | + | CAAAAAAACAGCUGGCGGGG | 20 | 1463 |
| HSV1-UL19-2671 | + | CCAAAAAAACAGCUGGCGGG | 20 | 2764 |
| HSV1-UL19-1080 | + | UCCCCCAAAAAAACAGCUGG | 20 | 1466 |
| HSV1-UL19-2673 | + | UUCCCCCAAAAAAACAGCUG | 20 | 2765 |
| HSV1-UL19-2674 | + | ACGGUCCCGAUCGCGUGUUC | 20 | 2766 |
| HSV1-UL19-2675 | + | CGCGUCAACGUUCAUCAGCG | 20 | 2767 |
| HSV1-UL19-2676 | + | CGCCGCGUCAACGUUCAUCA | 20 | 2768 |
| HSV1-UL19-1093 | + | GUACGGAUUGGCGGCUUCGA | 20 | 1479 |
| HSV1-UL19-2678 | + | CGUACGGAUUGGCGGCUUCG | 20 | 2769 |
| HSV1-UL19-2679 | + | GGCCGCCACGUACGCCCCGU | 20 | 2770 |
| HSV1-UL19-1099 | + | AUGUCUGCGGCGGGGCCGGC | 20 | 1485 |
| HSV1-UL19-2681 | + | CAUGUCUGCGGCGGGGCCGG | 20 | 2771 |
| HSV1-UL19-1103 | + | CAGCUGCUGCAUGUCUGCGG | 20 | 1489 |
| HSV1-UL19-2683 | + | ACAGCUGCUGCAUGUCUGCG | 20 | 2772 |
| HSV1-UL19-2684 | + | GCGACCCAUCGGACCCGCCC | 20 | 2773 |
| HSV1-UL19-2685 | + | AUCUGGCCUUCCGCGACCCA | 20 | 2774 |
| HSV1-UL19-1113 | + | UCGGGCUGCAUAAACUGCUC | 20 | 1499 |
| HSV1-UL19-2687 | + | GUCGGGCUGCAUAAACUGCU | 20 | 2775 |
| HSV1-UL19-2688 | + | CAAGAGCCAGGUUGGCGUUG | 20 | 2776 |
| HSV1-UL19-2689 | + | UCGAACGCGGGGUGCAGCUC | 20 | 2777 |
| HSV1-UL19-1120 | + | CCCCACAAAGAAGUCGAACG | 20 | 1506 |
| HSV1-UL19-2691 | + | CCCCCACAAAGAAGUCGAAC | 20 | 2778 |
| HSV1-UL19-2692 | + | CGGCCACCCCCACAAAGAAG | 20 | 2779 |
| HSV1-UL19-2693 | + | CCACGUCGGCCACCCCCACA | 20 | 2780 |
| HSV1-UL19-1123 | + | AUCUCCCCCGGGCCGGCCGG | 20 | 1509 |
| HSV1-UL19-1124 | + | GAUCUCCCCCGGGCCGGCCG | 20 | 1510 |
| HSV1-UL19-1125 | + | GGAUCUCCCCCGGGCCGGCC | 20 | 1511 |
| HSV1-UL19-1126 | + | UGGAUCUCCCCCGGGCCGGC | 20 | 1512 |
| HSV1-UL19-2698 | + | CUGGAUCUCCCCCGGGCCGG | 20 | 2781 |
| HSV1-UL19-2699 | + | CCAGGUGGCCUGGAUCUCCC | 20 | 2782 |
| HSV1-UL19-2700 | + | UUCACCACGCGCCAGGUGGC | 20 | 2783 |
| HSV1-UL19-1135 | + | ACGCCGCCGGACAUAGCGCC | 20 | 1521 |
| HSV1-UL19-2702 | + | AACGCCGCCGGACAUAGCGC | 20 | 2784 |
| HSV1-UL19-2703 | + | CCGGGCGUCCCGGAACGCCG | 20 | 2785 |
| HSV1-UL19-1137 | + | GCUCCAGGCCCCGGGCGUCC | 20 | 1523 |
| HSV1-UL19-2705 | + | AGCUCCAGGCCCCGGGCGUC | 20 | 2786 |
| HSV1-UL19-2706 | + | CCCACCCCCAGCUCCAGGCC | 20 | 2787 |
| HSV1-UL19-1145 | + | CGAACGGCGGCGAUGGUGGC | 20 | 1531 |
| HSV1-UL19-1146 | + | GCGAACGGCGGCGAUGGUGG | 20 | 1532 |
| HSV1-UL19-2709 | + | CGCGAACGGCGGCGAUGGUG | 20 | 2788 |
| HSV1-UL19-2710 | + | UUGCGGUCGUCGAACGCCCC | 20 | 2789 |
| HSV1-UL19-2711 | + | CCGCCGGGUAGUUGCGGUCG | 20 | 2790 |
| HSV1-UL19-2712 | + | CUGCAGCAGGUAAAACACCG | 20 | 2791 |
| HSV1-UL19-2713 | + | CCACGAGCCGGGCCAGGGCG | 20 | 2792 |
| HSV1-UL19-2714 | + | CACUGGACCACGAGCCGGGC | 20 | 2793 |
| HSV1-UL19-2715 | + | GUGAUGCACUGGACCACGAG | 20 | 2794 |
| HSV1-UL19-2716 | + | AGCUGGUGAUGCACUGGACC | 20 | 2795 |
| HSV1-UL19-2717 | + | UUCCAGUAGCUGGUGAUGCA | 20 | 2796 |
| HSV1-UL19-2718 | + | AGACCAGCGAGUAGUCGUUC | 20 | 2797 |
| HSV1-UL19-2719 | + | GGUCACGACGUACGAGACCA | 20 | 2798 |
| HSV1-UL19-2720 | + | CCCGAGGUAGGUCACGACGU | 20 | 2799 |
| HSV1-UL19-2721 | + | ACUCCUCGGGAAGGUCUCCC | 20 | 2800 |
| HSV1-UL19-1166 | + | UACACGGCCAUGCACUCCUC | 20 | 1552 |
| HSV1-UL19-1167 | + | GUACACGGCCAUGCACUCCU | 20 | 1553 |
| HSV1-UL19-2724 | + | GGUACACGGCCAUGCACUCC | 20 | 2801 |
| HSV1-UL19-2725 | + | AAGUCAUCAACCAGCUGGGC | 20 | 2802 |
| HSV1-UL19-2726 | + | AGGGUAAAGUCAUCAACCAG | 20 | 2803 |
| HSV1-UL19-2727 | + | CCGCCCAGCUCCGGGCCGGU | 20 | 2804 |
| HSV1-UL19-2728 | + | UUGCGCCUGCCCGCCCAGCU | 20 | 2805 |
| HSV1-UL19-2729 | + | CACGAGGGGUGGCAGCAGCG | 20 | 2806 |
| HSV1-UL19-1187 | + | GGGCGUCACAGUCCCACACG | 20 | 1573 |
| HSV1-UL19-2731 | + | AGGGCGUCACAGUCCCACAC | 20 | 2807 |
| HSV1-UL19-2732 | + | UCAGGGCGUCACAGUCCCAC | 20 | 2808 |
| HSV1-UL19-2733 | + | UCCAGGGCCGCGCGCCGCAU | 20 | 2809 |
| HSV1-UL19-2734 | + | CGGCAGUCGCGAUGGCGGUC | 20 | 2810 |
| HSV1-UL19-1198 | + | GUUACAUGCCGCCGCGUACA | 20 | 1584 |
| HSV1-UL19-2736 | + | CGUUACAUGCCGCCGCGUAC | 20 | 2811 |
| HSV1-UL19-2737 | + | GCAGCUGGCCGUCGUUGCGG | 20 | 2812 |
| HSV1-UL19-2738 | + | GCGGCGUCCGCGGCUCGGGC | 20 | 2813 |
| HSV1-UL19-2739 | + | UCAUCCGCGGCGUCCGCGGC | 20 | 2814 |
| HSV1-UL19-2740 | + | AGCAGCGGCCCCGCGAGAAG | 20 | 2815 |
| HSV1-UL19-2741 | + | CGGUGCAGCAGCGGCCCCGC | 20 | 2816 |
| HSV1-UL19-2742 | + | CGCGGUGCAGCAGCGGCCCC | 20 | 2817 |
| HSV1-UL19-2743 | + | GUGGCGUAUACGCGGUCGAA | 20 | 2818 |
| HSV1-UL19-2744 | + | GCAGGGUGGCGUAUACGCGG | 20 | 2819 |
| HSV1-UL19-2745 | + | UCCGGGACCACCAUGUUCUG | 20 | 2820 |
| HSV1-UL19-1223 | + | UCCUCGCCGGGGGCGAUCUC | 20 | 1609 |
| HSV1-UL19-2747 | + | CUCCUCGCCGGGGGCGAUCU | 20 | 2821 |
| HSV1-UL19-1226 | + | UCGCUGGGGCACUCCUCGCC | 20 | 1612 |
| HSV1-UL19-1227 | + | GUCGCUGGGGCACUCCUCGC | 20 | 1613 |
| HSV1-UL19-2750 | + | GGUCGCUGGGGCACUCCUCG | 20 | 2822 |
| HSV1-UL19-1230 | + | GGGGUCCGUCACGGGGUCGC | 20 | 1616 |
| HSV1-UL19-2752 | + | CGGGGUCCGUCACGGGGUCG | 20 | 2823 |
| HSV1-UL19-1233 | + | GGGGUGCGCGGGGUCCGUCA | 20 | 1619 |
| HSV1-UL19-2754 | + | GGGGGUGCGCGGGGUCCGUC | 20 | 2824 |
| HSV1-UL19-1236 | + | GGCCGGGUGCAGGGGGUGCG | 20 | 1622 |
| HSV1-UL19-2756 | + | UGGCCGGGUGCAGGGGGUGC | 20 | 2825 |
| HSV1-UL19-1239 | + | CACCAGAUUGGCCGGGUGCA | 20 | 1625 |
| HSV1-UL19-1240 | + | CCACCAGAUUGGCCGGGUGC | 20 | 1626 |
| HSV1-UL19-2759 | + | GCCACCAGAUUGGCCGGGUG | 20 | 2826 |
| HSV1-UL19-2760 | + | CGUGUUGGCCACCAGAUUGG | 20 | 2827 |
| HSV1-UL19-2761 | + | UCCACUACCACGCGCCCGUU | 20 | 2828 |
| HSV1-UL19-2762 | + | GCACCUGCAGCGUGAGCAUG | 20 | 2829 |
| HSV1-UL19-2763 | + | UGUGGGCCAGCACCUGCAGC | 20 | 2830 |
| HSV1-UL19-2764 | + | GUUGUGCGUUCGGCCAUGUU | 20 | 2831 |
| HSV1-UL19-2765 | + | CCGCGUCGGGCGCCGCCGAG | 20 | 2832 |
| HSV1-UL19-2766 | + | GGCGCCCGCGUCGGGCGCCG | 20 | 2833 |
| HSV1-UL19-2767 | + | ACGCGGUGUUGGCGCCCGCG | 20 | 2834 |
| HSV1-UL19-2768 | + | CGAAUAUGCGCAUGUUGGUG | 20 | 2835 |
| HSV1-UL19-2769 | + | UUCCGGCGUGCAACGCCCCG | 20 | 2836 |
| HSV1-UL19-2770 | + | AGAUGCUGGGGGGCCAUCAG | 20 | 2837 |
| HSV1-UL19-1263 | + | AUGGUAUGGUCCAGAUGCUG | 20 | 1649 |
| HSV1-UL19-1264 | + | GAUGGUAUGGUCCAGAUGCU | 20 | 1650 |
| HSV1-UL19-1265 | + | GGAUGGUAUGGUCCAGAUGC | 20 | 1651 |
| HSV1-UL19-2774 | + | UGGAUGGUAUGGUCCAGAUG | 20 | 2838 |
| HSV1-UL19-2775 | + | GGGUAAAAAUAGUCGCCAUU | 20 | 2839 |
| HSV1-UL19-1271 | + | GAACAGCGCGUGGACGGGGA | 20 | 1657 |
| HSV1-UL19-1272 | + | CGAACAGCGCGUGGACGGGG | 20 | 1658 |
| HSV1-UL19-2778 | + | GCGAACAGCGCGUGGACGGG | 20 | 2840 |
| HSV1-UL19-1273 | + | CGGCGAACAGCGCGUGGACG | 20 | 1659 |
| HSV1-UL19-1274 | + | CCGGCGAACAGCGCGUGGAC | 20 | 1660 |
| HSV1-UL19-1275 | + | CCCGGCGAACAGCGCGUGGA | 20 | 1661 |
| HSV1-UL19-2782 | + | CCCCGGCGAACAGCGCGUGG | 20 | 2841 |
| HSV1-UL19-2783 | + | UCGGCCCCGGCGAACAGCGC | 20 | 2842 |
| HSV1-UL19-2784 | + | UCGCCACGUGGUCGGCCCCG | 20 | 2843 |
| HSV1-UL19-2785 | + | CGCGCAGGGCCGGGGGGAAA | 20 | 2844 |
| HSV1-UL19-1282 | + | ACAGGUCGCGCAGGGCCGGG | 20 | 1668 |
| HSV1-UL19-1283 | + | GACAGGUCGCGCAGGGCCGG | 20 | 1669 |
| HSV1-UL19-1284 | + | CGACAGGUCGCGCAGGGCCG | 20 | 1670 |
| HSV1-UL19-1285 | + | GCGACAGGUCGCGCAGGGCC | 20 | 1671 |
| HSV1-UL19-1286 | + | CGCGACAGGUCGCGCAGGGC | 20 | 1672 |
| HSV1-UL19-2791 | + | CCGCGACAGGUCGCGCAGGG | 20 | 2845 |
| HSV1-UL19-2792 | + | ACCUGCCGCGACAGGUCGCG | 20 | 2846 |
| HSV1-UL19-1291 | + | CCCAGAGCCGGGGGGACCAG | 20 | 1677 |
| HSV1-UL19-1292 | + | CCCCAGAGCCGGGGGGACCA | 20 | 1678 |
| HSV1-UL19-1293 | + | CCCCCAGAGCCGGGGGGACC | 20 | 1679 |
| HSV1-UL19-2796 | + | GCCCCCAGAGCCGGGGGGAC | 20 | 2847 |
| HSV1-UL19-1295 | + | UAGUUGGCCCCCAGAGCCGG | 20 | 1681 |
| HSV1-UL19-1296 | + | GUAGUUGGCCCCCAGAGCCG | 20 | 1682 |
| HSV1-UL19-1297 | + | AGUAGUUGGCCCCCAGAGCC | 20 | 1683 |
| HSV1-UL19-1298 | + | AAGUAGUUGGCCCCCAGAGC | 20 | 1684 |
| HSV1-UL19-2801 | + | AAAGUAGUUGGCCCCCAGAG | 20 | 2848 |
| HSV1-UL19-2802 | + | GACGAAAAGUAGUUGGCCCC | 20 | 2849 |
| HSV1-UL19-2803 | + | CACGACGGGCUGUCGGAUCG | 20 | 2850 |
| HSV1-UL19-2804 | + | ACGUGCUGCACGACGGGCUG | 20 | 2851 |
| HSV1-UL19-2805 | + | UCUCGCGGACGUGCUGCACG | 20 | 2852 |
| HSV1-UL19-2806 | + | UUCUCCCCGGCCGCGCUCUC | 20 | 2853 |
| HSV1-UL19-2807 | + | UGAUCUUGAAGUACCCCGCC | 20 | 2854 |
| HSV1-UL19-2808 | + | GCAAGGCCACGGGACUGAUC | 20 | 2855 |
| HSV1-UL19-1307 | + | GAGCUGAUGAUGCAAGGCCA | 20 | 1693 |
| HSV1-UL19-2810 | + | UGAGCUGAUGAUGCAAGGCC | 20 | 2856 |
| HSV1-UL19-2811 | + | ACCCGGGAUGGAGGCCCGUC | 20 | 2857 |
| HSV1-UL19-1310 | + | CGGUGAACCCAAACCCGGGA | 20 | 1696 |
| HSV1-UL19-2813 | + | ACGGUGAACCCAAACCCGGG | 20 | 2858 |
| HSV1-UL19-1312 | + | GACGACGGUGAACCCAAACC | 20 | 1698 |
| HSV1-UL19-2815 | + | GGACGACGGUGAACCCAAAC | 20 | 2859 |
| HSV1-UL19-2816 | + | AGCGGUCCUGUCGGACGACG | 20 | 2860 |
| HSV1-UL19-2817 | + | UCAGUCACAAAGCGGUCCUG | 20 | 2861 |
| HSV1-UL19-2818 | + | ACGCCUCCGACGCGCGCUCC | 20 | 2862 |
| HSV1-UL19-2819 | + | GUACGCCUCCGACGCGCGCU | 20 | 2863 |
| HSV1-UL19-1316 | + | GGGCCACCUGGAGCUGGCCC | 20 | 1702 |
| HSV1-UL19-2821 | + | CGGGCCACCUGGAGCUGGCC | 20 | 2864 |
| HSV1-UL19-1318 | + | CAGUUUCGUGCCGGGCCACC | 20 | 1704 |
| HSV1-UL19-2823 | + | CCAGUUUCGUGCCGGGCCAC | 20 | 2865 |
| HSV1-UL19-2824 | + | ACCCCCCCGCCAGUUUCGUG | 20 | 2866 |
| HSV1-UL19-2825 | + | UCGCGCGCGGCUGGGUGAGC | 20 | 2867 |
| HSV1-UL19-2826 | + | CCACGUUCGCGCGCGGCUGG | 20 | 2868 |
| HSV1-UL19-2827 | + | AGGUCCACGUUCGCGCGCGG | 20 | 2869 |
| HSV1-UL19-2828 | + | CCCAUGUCGGUGACGGGGUU | 20 | 2870 |
| HSV1-UL19-1332 | + | AAGGUUGCCCAUGUCGGUGA | 20 | 1718 |
| HSV1-UL19-2830 | + | GAAGGUUGCCCAUGUCGGUG | 20 | 2871 |
| HSV1-UL19-1335 | + | CGGCCCAGGUAAAAGUUUUG | 20 | 1721 |
| HSV1-UL19-1336 | + | GCGGCCCAGGUAAAAGUUUU | 20 | 1722 |
| HSV1-UL19-1337 | + | CGCGGCCCAGGUAAAAGUUU | 20 | 1723 |
| HSV1-UL19-2834 | + | CCGCGGCCCAGGUAAAAGUU | 20 | 2872 |
| HSV1-UL19-1340 | + | GCCGCGUUGUCCAGGAGAGG | 20 | 1726 |
| HSV1-UL19-1341 | + | UGCCGCGUUGUCCAGGAGAG | 20 | 1727 |
| HSV1-UL19-1342 | + | CUGCCGCGUUGUCCAGGAGA | 20 | 1728 |
| HSV1-UL19-1343 | + | GCUGCCGCGUUGUCCAGGAG | 20 | 1729 |
| HSV1-UL19-2839 | + | GGCUGCCGCGUUGUCCAGGA | 20 | 2873 |
| HSV1-UL19-2840 | + | ACGGCUGCCGCGUUGUCCAG | 20 | 2874 |
| HSV1-UL19-1344 | + | ACACGGCUGCCGCGUUGUCC | 20 | 1730 |
| HSV1-UL19-2842 | + | UACACGGCUGCCGCGUUGUC | 20 | 2875 |
| HSV1-UL19-2843 | + | CACCCGAACACGGGGACGGG | 20 | 2876 |
| HSV1-UL19-2844 | + | GGGCGCACCCGAACACGGGG | 20 | 2877 |
| HSV1-UL19-1355 | + | ACCUGGGCGCACCCGAACAC | 20 | 1741 |
| HSV1-UL19-1356 | + | CACCUGGGCGCACCCGAACA | 20 | 1742 |
| HSV1-UL19-2847 | + | GCACCUGGGCGCACCCGAAC | 20 | 2878 |
| HSV1-UL19-2848 | + | GCCGCGGCACCUGGGCGCAC | 20 | 2879 |
| HSV1-UL19-2849 | + | AUCCCUGCGCGCCGCGGCAC | 20 | 2880 |
| HSV1-UL19-1366 | + | UAGUUGACGUCGGUCGACAC | 20 | 1752 |
| HSV1-UL19-1367 | + | GUAGUUGACGUCGGUCGACA | 20 | 1753 |
| HSV1-UL19-2852 | + | AGUAGUUGACGUCGGUCGAC | 20 | 2881 |
| HSV1-UL19-1369 | + | CCCGGGGGUUGCAGGGCCGG | 20 | 1755 |
| HSV1-UL19-2854 | + | CCCCGGGGGUUGCAGGGCCG | 20 | 2882 |
| HSV1-UL19-2855 | + | CGGCGCGUCCCCGGGGGUUG | 20 | 2883 |
| HSV1-UL19-1375 | + | GCCCCCGGCGGCGCGUCCCC | 20 | 1761 |
| HSV1-UL19-1376 | + | CGCCCCCGGCGGCGCGUCCC | 20 | 1762 |
| HSV1-UL19-2858 | + | ACGCCCCCGGCGGCGCGUCC | 20 | 2884 |
| HSV1-UL19-2859 | + | CUCUGGCCGUGGUCGUACAU | 20 | 2885 |
| HSV1-UL19-2860 | + | CGCUCUGGCCGUGGUCGUAC | 20 | 2886 |
| HSV1-UL19-2861 | + | CGUGGCCGCGAAGGCCCGGG | 20 | 2887 |
| HSV1-UL19-1387 | + | UGGCCGUGGCCGCGAAGGCC | 20 | 1773 |
| HSV1-UL19-2863 | + | UUGGCCGUGGCCGCGAAGGC | 20 | 2888 |
| HSV1-UL19-2864 | + | CCCACGGGUUGGCCGUGGCC | 20 | 2889 |
| HSV1-UL19-2865 | + | CGAAAAUCGCUGCGACGCCC | 20 | 2890 |
| HSV1-UL19-2866 | + | GUUAUAGAGCAGGUCCCCGU | 20 | 2891 |
| HSV1-UL19-2867 | + | UGAGGUGGUAGGCCCCGUUA | 20 | 2892 |
| HSV1-UL19-2868 | + | UGAGCACCGGCGAGGCCCCG | 20 | 2893 |
| HSV1-UL19-2869 | + | AAAGCAGGGGCUGAGCACCG | 20 | 2894 |
| HSV1-UL19-2870 | + | UAAAGAACUUAAAGCAGGGG | 20 | 2895 |
| HSV1-UL19-1401 | + | CGACGUAAAGAACUUAAAGC | 20 | 1787 |
| HSV1-UL19-2872 | + | CCGACGUAAAGAACUUAAAG | 20 | 2896 |
| HSV1-UL19-2873 | + | CGGCGAUGUCGGCCGACGUA | 20 | 2897 |
| HSV1-UL19-2874 | + | GGCGGUGGCCGUGGACACCG | 20 | 2898 |
| HSV1-UL19-2875 | + | CGUCGCUGGCGGCGGUGGCC | 20 | 2899 |
| HSV1-UL19-1414 | + | CACGAGUUCGCGGCACCCCG | 20 | 1800 |
| HSV1-UL19-1415 | + | CCACGAGUUCGCGGCACCCC | 20 | 1801 |
| HSV1-UL19-1416 | + | UCCACGAGUUCGCGGCACCC | 20 | 1802 |
| HSV1-UL19-2879 | + | CUCCACGAGUUCGCGGCACC | 20 | 2900 |
| HSV1-UL19-2880 | + | ACAGGCCACACGGGUCCUCC | 20 | 2901 |
| HSV1-UL19-2881 | + | GGCCUCCUGAAACAGGCCAC | 20 | 2902 |
| HSV1-UL19-2882 | + | CAGGUGAGCGGGUAGGCCUC | 20 | 2903 |
| HSV1-UL19-2883 | + | GGGGUCGCUGGCGCAGGUGA | 20 | 2904 |
| HSV1-UL19-2884 | + | GGGCGGGGUCGCUGGCGCAG | 20 | 2905 |
| HSV1-UL19-1428 | + | GCGGGCACUGCGGAGGAGGG | 20 | 1814 |
| HSV1-UL19-2886 | + | UGCGGGCACUGCGGAGGAGG | 20 | 2906 |
| HSV1-UL19-2887 | + | CCGUUGCGGGCACUGCGGAG | 20 | 2907 |
| HSV1-UL19-1431 | + | CCCCGUUGCGGGCACUGCGG | 20 | 1817 |
| HSV1-UL19-2889 | + | UCCCCGUUGCGGGCACUGCG | 20 | 2908 |
| HSV1-UL19-1432 | + | CUUCCCCGUUGCGGGCACUG | 20 | 1818 |
| HSV1-UL19-2891 | + | GCUUCCCCGUUGCGGGCACU | 20 | 2909 |
| HSV1-UL19-2892 | + | CGCGCGUGGGCUUCCCCGUU | 20 | 2910 |
| HSV1-UL19-2893 | + | GCGAAGUGGGUCUCCCGCGC | 20 | 2911 |
| HSV1-UL19-2894 | + | UAGACGAGAUACUGCGCGAA | 20 | 2912 |
| HSV1-UL19-2895 | + | CGUCAUAGACGAGAUACUGC | 20 | 2913 |
| HSV1-UL19-2896 | + | UGAGCGGGGAUGCGUCAUAG | 20 | 2914 |
| HSV1-UL19-1440 | + | ACAGAGCCAGUCCCUUGAGC | 20 | 1826 |
| HSV1-UL19-1441 | + | UACAGAGCCAGUCCCUUGAG | 20 | 1827 |
| HSV1-UL19-2899 | + | UUACAGAGCCAGUCCCUUGA | 20 | 2915 |
| HSV1-UL19-2900 | + | AACGCCCCGAGGACGGC | 17 | 2916 |
| HSV1-UL19-2901 | + | CCGCGCUCAAACGCCCC | 17 | 2917 |
| HSV1-UL19-2902 | + | UGCCGCGCUCAAACGCC | 17 | 2918 |
| HSV1-UL19-1450 | + | UCGGCAACAACAAAGCC | 17 | 1836 |
| HSV1-UL19-2904 | + | AUCGGCAACAACAAAGC | 17 | 2919 |
| HSV1-UL19-2905 | + | UCGGCGACCAGGGUCGC | 17 | 2920 |
| HSV1-UL19-2906 | + | CGCUUUAGCUCGGCGAC | 17 | 2921 |
| HSV1-UL19-2907 | + | GGAAAAAGCUCGUCUCG | 17 | 2922 |
| HSV1-UL19-1462 | + | GGUGGCCCGCCUUGCCC | 17 | 1848 |
| HSV1-UL19-2909 | + | CGGUGGCCCGCCUUGCC | 17 | 2923 |
| HSV1-UL19-2910 | + | UGGCCGUGGUGAGGUCC | 17 | 2924 |
| HSV1-UL19-2911 | + | AGGGCUGCGUGGCCGUG | 17 | 2925 |
| HSV1-UL19-2912 | + | GACGGGGCACGGCCACG | 17 | 2926 |
| HSV1-UL19-1473 | + | CAGACGGGGCACGGCCA | 17 | 1859 |
| HSV1-UL19-2914 | + | UCAGACGGGGCACGGCC | 17 | 2927 |
| HSV1-UL19-1477 | + | UGUCGGCAUGCGUCAGA | 17 | 1863 |
| HSV1-UL19-2916 | + | GUGUCGGCAUGCGUCAG | 17 | 2928 |
| HSV1-UL19-2917 | + | UGAUCGGGGCGGUGGUG | 17 | 2929 |
| HSV1-UL19-1484 | + | AGGAGGCGCUGUUUGAU | 17 | 1870 |
| HSV1-UL19-2919 | + | CAGGAGGCGCUGUUUGA | 17 | 2930 |
| HSV1-UL19-1486 | + | CUUUCAGGAAGGACUGC | 17 | 1872 |
| HSV1-UL19-2921 | + | ACUUUCAGGAAGGACUG | 17 | 2931 |
| HSV1-UL19-2922 | + | UGUCCUCCACUUUCAGG | 17 | 2932 |
| HSV1-UL19-1488 | + | CGGUGUCCUCCACUUUC | 17 | 1874 |
| HSV1-UL19-2924 | + | UCGGUGUCCUCCACUUU | 17 | 2933 |
| HSV1-UL19-2925 | + | ACCAGGUUGGCCCCGUU | 17 | 2934 |
| HSV1-UL19-2926 | + | GCACGGCCUUGCCCAUC | 17 | 2935 |
| HSV1-UL19-2927 | + | GCGUCUGCCGGUUCAGG | 17 | 2936 |
| HSV1-UL19-2928 | + | UCGUCUGGGGGGCGCUC | 17 | 2937 |
| HSV1-UL19-1503 | + | CGCACGCGCGUCGUCUG | 17 | 1889 |
| HSV1-UL19-1504 | + | GCGCACGCGCGUCGUCU | 17 | 1890 |
| HSV1-UL19-1505 | + | CGCGCACGCGCGUCGUC | 17 | 1891 |
| HSV1-UL19-2932 | + | GCGCGCACGCGCGUCGU | 17 | 2938 |
| HSV1-UL19-2933 | + | AGACCAGCUUCUCGCCG | 17 | 2939 |
| HSV1-UL19-2934 | + | CGCUUCUCCAGGGCCUC | 17 | 2940 |
| HSV1-UL19-2935 | + | GCGUAGAUGCGCUUCUC | 17 | 2941 |
| HSV1-UL19-1512 | + | GCGCCCACCAGGGGGUA | 17 | 1898 |
| HSV1-UL19-1513 | + | GGCGCCCACCAGGGGGU | 17 | 1899 |
| HSV1-UL19-2938 | + | UGGCGCCCACCAGGGGG | 17 | 2942 |
| HSV1-UL19-1516 | + | GUCCAUGGCGCCCACCA | 17 | 1902 |
| HSV1-UL19-1517 | + | GGUCCAUGGCGCCCACC | 17 | 1903 |
| HSV1-UL19-2941 | + | AGGUCCAUGGCGCCCAC | 17 | 2943 |
| HSV1-UL19-2942 | + | ACAGGCCCAGGGGCAGG | 17 | 2944 |
| HSV1-UL19-2943 | + | UUGAACAGGCCCAGGGG | 17 | 2945 |
| HSV1-UL19-1523 | + | CCGGAUUGAACAGGCCC | 17 | 1909 |
| HSV1-UL19-2945 | + | ACCGGAUUGAACAGGCC | 17 | 2946 |
| HSV1-UL19-2946 | + | ACCGUUCCAUGACCGGA | 17 | 2947 |
| HSV1-UL19-2947 | + | GGCAAACCGUUCCAUGA | 17 | 2948 |
| HSV1-UL19-1530 | + | UCCGGGUGGCCGGGGGC | 17 | 1916 |
| HSV1-UL19-1531 | + | AUCCGGGUGGCCGGGGG | 17 | 1917 |
| HSV1-UL19-2950 | + | GAUCCGGGUGGCCGGGG | 17 | 2949 |
| HSV1-UL19-1534 | + | CGGGGAUCCGGGUGGCC | 17 | 1920 |
| HSV1-UL19-1535 | + | GCGGGGAUCCGGGUGGC | 17 | 1921 |
| HSV1-UL19-2953 | + | CGCGGGGAUCCGGGUGG | 17 | 2950 |
| HSV1-UL19-2954 | + | CGGGAAGGCGCGGGGAU | 17 | 2951 |
| HSV1-UL19-1540 | + | GCGGGGCGGGAAGGCGC | 17 | 1926 |
| HSV1-UL19-1541 | + | GGCGGGGCGGGAAGGCG | 17 | 1927 |
| HSV1-UL19-2957 | + | UGGCGGGGCGGGAAGGC | 17 | 2952 |
| HSV1-UL19-1543 | + | AAAACAGCUGGCGGGGC | 17 | 1929 |
| HSV1-UL19-1544 | + | AAAAACAGCUGGCGGGG | 17 | 1930 |
| HSV1-UL19-2960 | + | AAAAAACAGCUGGCGGG | 17 | 2953 |
| HSV1-UL19-1547 | + | CCCAAAAAAACAGCUGG | 17 | 1933 |
| HSV1-UL19-2962 | + | CCCCAAAAAAACAGCUG | 17 | 2954 |
| HSV1-UL19-2963 | + | GUCCCGAUCGCGUGUUC | 17 | 2955 |
| HSV1-UL19-2964 | + | GUCAACGUUCAUCAGCG | 17 | 2956 |
| HSV1-UL19-2965 | + | CGCGUCAACGUUCAUCA | 17 | 2957 |
| HSV1-UL19-1560 | + | CGGAUUGGCGGCUUCGA | 17 | 1946 |
| HSV1-UL19-2967 | + | ACGGAUUGGCGGCUUCG | 17 | 2958 |
| HSV1-UL19-2968 | + | CGCCACGUACGCCCCGU | 17 | 2959 |
| HSV1-UL19-1566 | + | UCUGCGGCGGGGCCGGC | 17 | 1952 |
| HSV1-UL19-2970 | + | GUCUGCGGCGGGGCCGG | 17 | 2960 |
| HSV1-UL19-1570 | + | CUGCUGCAUGUCUGCGG | 17 | 1956 |
| HSV1-UL19-2972 | + | GCUGCUGCAUGUCUGCG | 17 | 2961 |
| HSV1-UL19-2973 | + | ACCCAUCGGACCCGCCC | 17 | 2962 |
| HSV1-UL19-2974 | + | UGGCCUUCCGCGACCCA | 17 | 2963 |
| HSV1-UL19-1580 | + | GGCUGCAUAAACUGCUC | 17 | 1966 |
| HSV1-UL19-2976 | + | GGGCUGCAUAAACUGCU | 17 | 2964 |
| HSV1-UL19-2977 | + | GAGCCAGGUUGGCGUUG | 17 | 2965 |
| HSV1-UL19-2978 | + | AACGCGGGGUGCAGCUC | 17 | 2966 |
| HSV1-UL19-1587 | + | CACAAAGAAGUCGAACG | 17 | 1973 |
| HSV1-UL19-2980 | + | CCACAAAGAAGUCGAAC | 17 | 2967 |
| HSV1-UL19-2981 | + | CCACCCCCACAAAGAAG | 17 | 2968 |
| HSV1-UL19-2982 | + | CGUCGGCCACCCCCACA | 17 | 2969 |
| HSV1-UL19-1590 | + | UCCCCCGGGCCGGCCGG | 17 | 1976 |
| HSV1-UL19-1591 | + | CUCCCCCGGGCCGGCCG | 17 | 1977 |
| HSV1-UL19-1592 | + | UCUCCCCCGGGCCGGCC | 17 | 1978 |
| HSV1-UL19-1593 | + | AUCUCCCCCGGGCCGGC | 17 | 1979 |
| HSV1-UL19-2987 | + | GAUCUCCCCCGGGCCGG | 17 | 2970 |
| HSV1-UL19-2988 | + | GGUGGCCUGGAUCUCCC | 17 | 2971 |
| HSV1-UL19-2989 | + | ACCACGCGCCAGGUGGC | 17 | 2972 |
| HSV1-UL19-1602 | + | CCGCCGGACAUAGCGCC | 17 | 1988 |
| HSV1-UL19-2991 | + | GCCGCCGGACAUAGCGC | 17 | 2973 |
| HSV1-UL19-2992 | + | GGCGUCCCGGAACGCCG | 17 | 2974 |
| HSV1-UL19-1604 | + | CCAGGCCCCGGGCGUCC | 17 | 1990 |
| HSV1-UL19-2994 | + | UCCAGGCCCCGGGCGUC | 17 | 2975 |
| HSV1-UL19-2995 | + | ACCCCCAGCUCCAGGCC | 17 | 2976 |
| HSV1-UL19-1612 | + | ACGGCGGCGAUGGUGGC | 17 | 1998 |
| HSV1-UL19-1613 | + | AACGGCGGCGAUGGUGG | 17 | 1999 |
| HSV1-UL19-2998 | + | GAACGGCGGCGAUGGUG | 17 | 2977 |
| HSV1-UL19-2999 | + | CGGUCGUCGAACGCCCC | 17 | 2978 |
| HSV1-UL19-3000 | + | CCGGGUAGUUGCGGUCG | 17 | 2979 |
| HSV1-UL19-3001 | + | CAGCAGGUAAAACACCG | 17 | 2980 |
| HSV1-UL19-3002 | + | CGAGCCGGGCCAGGGCG | 17 | 2981 |
| HSV1-UL19-3003 | + | UGGACCACGAGCCGGGC | 17 | 2982 |
| HSV1-UL19-3004 | + | AUGCACUGGACCACGAG | 17 | 2983 |
| HSV1-UL19-3005 | + | UGGUGAUGCACUGGACC | 17 | 2984 |
| HSV1-UL19-3006 | + | CAGUAGCUGGUGAUGCA | 17 | 2985 |
| HSV1-UL19-3007 | + | CCAGCGAGUAGUCGUUC | 17 | 2986 |
| HSV1-UL19-3008 | + | CACGACGUACGAGACCA | 17 | 2987 |
| HSV1-UL19-3009 | + | GAGGUAGGUCACGACGU | 17 | 2988 |
| HSV1-UL19-3010 | + | CCUCGGGAAGGUCUCCC | 17 | 2989 |
| HSV1-UL19-1633 | + | ACGGCCAUGCACUCCUC | 17 | 2019 |
| HSV1-UL19-1634 | + | CACGGCCAUGCACUCCU | 17 | 2020 |
| HSV1-UL19-3013 | + | ACACGGCCAUGCACUCC | 17 | 2990 |
| HSV1-UL19-3014 | + | UCAUCAACCAGCUGGGC | 17 | 2991 |
| HSV1-UL19-3015 | + | GUAAAGUCAUCAACCAG | 17 | 2992 |
| HSV1-UL19-3016 | + | CCCAGCUCCGGGCCGGU | 17 | 2993 |
| HSV1-UL19-3017 | + | CGCCUGCCCGCCCAGCU | 17 | 2994 |
| HSV1-UL19-3018 | + | GAGGGGUGGCAGCAGCG | 17 | 2995 |
| HSV1-UL19-1654 | + | CGUCACAGUCCCACACG | 17 | 2040 |
| HSV1-UL19-3020 | + | GCGUCACAGUCCCACAC | 17 | 2996 |
| HSV1-UL19-3021 | + | GGGCGUCACAGUCCCAC | 17 | 2997 |
| HSV1-UL19-3022 | + | AGGGCCGCGCGCCGCAU | 17 | 2998 |
| HSV1-UL19-3023 | + | CAGUCGCGAUGGCGGUC | 17 | 2999 |
| HSV1-UL19-1665 | + | ACAUGCCGCCGCGUACA | 17 | 2051 |
| HSV1-UL19-3025 | + | UACAUGCCGCCGCGUAC | 17 | 3000 |
| HSV1-UL19-3026 | + | GCUGGCCGUCGUUGCGG | 17 | 3001 |
| HSV1-UL19-3027 | + | GCGUCCGCGGCUCGGGC | 17 | 3002 |
| HSV1-UL19-3028 | + | UCCGCGGCGUCCGCGGC | 17 | 3003 |
| HSV1-UL19-3029 | + | AGCGGCCCCGCGAGAAG | 17 | 3004 |
| HSV1-UL19-3030 | + | UGCAGCAGCGGCCCCGC | 17 | 3005 |
| HSV1-UL19-3031 | + | GGUGCAGCAGCGGCCCC | 17 | 3006 |
| HSV1-UL19-3032 | + | GCGUAUACGCGGUCGAA | 17 | 3007 |
| HSV1-UL19-3033 | + | GGGUGGCGUAUACGCGG | 17 | 3008 |
| HSV1-UL19-3034 | + | GGGACCACCAUGUUCUG | 17 | 3009 |
| HSV1-UL19-1690 | + | UCGCCGGGGGCGAUCUC | 17 | 2076 |
| HSV1-UL19-3036 | + | CUCGCCGGGGGCGAUCU | 17 | 3010 |
| HSV1-UL19-1693 | + | CUGGGGCACUCCUCGCC | 17 | 2079 |
| HSV1-UL19-1694 | + | GCUGGGGCACUCCUCGC | 17 | 2080 |
| HSV1-UL19-3039 | + | CGCUGGGGCACUCCUCG | 17 | 3011 |
| HSV1-UL19-1697 | + | GUCCGUCACGGGGUCGC | 17 | 2083 |
| HSV1-UL19-3041 | + | GGUCCGUCACGGGGUCG | 17 | 3012 |
| HSV1-UL19-1700 | + | GUGCGCGGGGUCCGUCA | 17 | 2086 |
| HSV1-UL19-3043 | + | GGUGCGCGGGGUCCGUC | 17 | 3013 |
| HSV1-UL19-1703 | + | CGGGUGCAGGGGGUGCG | 17 | 2089 |
| HSV1-UL19-3045 | + | CCGGGUGCAGGGGGUGC | 17 | 3014 |
| HSV1-UL19-1706 | + | CAGAUUGGCCGGGUGCA | 17 | 2092 |
| HSV1-UL19-1707 | + | CCAGAUUGGCCGGGUGC | 17 | 2093 |
| HSV1-UL19-3048 | + | ACCAGAUUGGCCGGGUG | 17 | 3015 |
| HSV1-UL19-3049 | + | GUUGGCCACCAGAUUGG | 17 | 3016 |
| HSV1-UL19-3050 | + | ACUACCACGCGCCCGUU | 17 | 3017 |
| HSV1-UL19-3051 | + | CCUGCAGCGUGAGCAUG | 17 | 3018 |
| HSV1-UL19-3052 | + | GGGCCAGCACCUGCAGC | 17 | 3019 |
| HSV1-UL19-3053 | + | GUGCGUUCGGCCAUGUU | 17 | 3020 |
| HSV1-UL19-3054 | + | CGUCGGGCGCCGCCGAG | 17 | 3021 |
| HSV1-UL19-3055 | + | GCCCGCGUCGGGCGCCG | 17 | 3022 |
| HSV1-UL19-3056 | + | CGGUGUUGGCGCCCGCG | 17 | 3023 |
| HSV1-UL19-3057 | + | AUAUGCGCAUGUUGGUG | 17 | 3024 |
| HSV1-UL19-3058 | + | CGGCGUGCAACGCCCCG | 17 | 3025 |
| HSV1-UL19-3059 | + | UGCUGGGGGGCCAUCAG | 17 | 3026 |
| HSV1-UL19-1730 | + | GUAUGGUCCAGAUGCUG | 17 | 2116 |
| HSV1-UL19-1731 | + | GGUAUGGUCCAGAUGCU | 17 | 2117 |
| HSV1-UL19-1732 | + | UGGUAUGGUCCAGAUGC | 17 | 2118 |
| HSV1-UL19-3063 | + | AUGGUAUGGUCCAGAUG | 17 | 3027 |
| HSV1-UL19-3064 | + | UAAAAAUAGUCGCCAUU | 17 | 3028 |
| HSV1-UL19-1738 | + | CAGCGCGUGGACGGGGA | 17 | 2124 |
| HSV1-UL19-1739 | + | ACAGCGCGUGGACGGGG | 17 | 2125 |
| HSV1-UL19-3067 | + | AACAGCGCGUGGACGGG | 17 | 3029 |
| HSV1-UL19-1740 | + | CGAACAGCGCGUGGACG | 17 | 2126 |
| HSV1-UL19-1741 | + | GCGAACAGCGCGUGGAC | 17 | 2127 |
| HSV1-UL19-1742 | + | GGCGAACAGCGCGUGGA | 17 | 2128 |
| HSV1-UL19-3071 | + | CGGCGAACAGCGCGUGG | 17 | 3030 |
| HSV1-UL19-3072 | + | GCCCCGGCGAACAGCGC | 17 | 3031 |
| HSV1-UL19-3073 | + | CCACGUGGUCGGCCCCG | 17 | 3032 |
| HSV1-UL19-3074 | + | GCAGGGCCGGGGGGAAA | 17 | 3033 |
| HSV1-UL19-1749 | + | GGUCGCGCAGGGCCGGG | 17 | 2135 |
| HSV1-UL19-1750 | + | AGGUCGCGCAGGGCCGG | 17 | 2136 |
| HSV1-UL19-1751 | + | CAGGUCGCGCAGGGCCG | 17 | 2137 |
| HSV1-UL19-1752 | + | ACAGGUCGCGCAGGGCC | 17 | 2138 |
| HSV1-UL19-1753 | + | GACAGGUCGCGCAGGGC | 17 | 2139 |
| HSV1-UL19-3080 | + | CGACAGGUCGCGCAGGG | 17 | 3034 |
| HSV1-UL19-3081 | + | UGCCGCGACAGGUCGCG | 17 | 3035 |
| HSV1-UL19-1758 | + | AGAGCCGGGGGGACCAG | 17 | 2144 |
| HSV1-UL19-1759 | + | CAGAGCCGGGGGGACCA | 17 | 2145 |
| HSV1-UL19-1760 | + | CCAGAGCCGGGGGGACC | 17 | 2146 |
| HSV1-UL19-3085 | + | CCCAGAGCCGGGGGGAC | 17 | 3036 |
| HSV1-UL19-1762 | + | UUGGCCCCCAGAGCCGG | 17 | 2148 |
| HSV1-UL19-1763 | + | GUUGGCCCCCAGAGCCG | 17 | 2149 |
| HSV1-UL19-1764 | + | AGUUGGCCCCCAGAGCC | 17 | 2150 |
| HSV1-UL19-1765 | + | UAGUUGGCCCCCAGAGC | 17 | 2151 |
| HSV1-UL19-3090 | + | GUAGUUGGCCCCCAGAG | 17 | 3037 |
| HSV1-UL19-3091 | + | GAAAAGUAGUUGGCCCC | 17 | 3038 |
| HSV1-UL19-3092 | + | GACGGGCUGUCGGAUCG | 17 | 3039 |
| HSV1-UL19-3093 | + | UGCUGCACGACGGGCUG | 17 | 3040 |
| HSV1-UL19-3094 | + | CGCGGACGUGCUGCACG | 17 | 3041 |
| HSV1-UL19-3095 | + | UCCCCGGCCGCGCUCUC | 17 | 3042 |
| HSV1-UL19-3096 | + | UCUUGAAGUACCCCGCC | 17 | 3043 |
| HSV1-UL19-3097 | + | AGGCCACGGGACUGAUC | 17 | 3044 |
| HSV1-UL19-1774 | + | CUGAUGAUGCAAGGCCA | 17 | 2160 |
| HSV1-UL19-3099 | + | GCUGAUGAUGCAAGGCC | 17 | 3045 |
| HSV1-UL19-3100 | + | CGGGAUGGAGGCCCGUC | 17 | 3046 |
| HSV1-UL19-1777 | + | UGAACCCAAACCCGGGA | 17 | 2163 |
| HSV1-UL19-3102 | + | GUGAACCCAAACCCGGG | 17 | 3047 |
| HSV1-UL19-1779 | + | GACGGUGAACCCAAACC | 17 | 2165 |
| HSV1-UL19-3104 | + | CGACGGUGAACCCAAAC | 17 | 3048 |
| HSV1-UL19-3105 | + | GGUCCUGUCGGACGACG | 17 | 3049 |
| HSV1-UL19-3106 | + | GUCACAAAGCGGUCCUG | 17 | 3050 |
| HSV1-UL19-3107 | + | CCUCCGACGCGCGCUCC | 17 | 3051 |
| HSV1-UL19-3108 | + | CGCCUCCGACGCGCGCU | 17 | 3052 |
| HSV1-UL19-1783 | + | CCACCUGGAGCUGGCCC | 17 | 2169 |
| HSV1-UL19-3110 | + | GCCACCUGGAGCUGGCC | 17 | 3053 |
| HSV1-UL19-1785 | + | UUUCGUGCCGGGCCACC | 17 | 2171 |
| HSV1-UL19-3112 | + | GUUUCGUGCCGGGCCAC | 17 | 3054 |
| HSV1-UL19-3113 | + | CCCCCGCCAGUUUCGUG | 17 | 3055 |
| HSV1-UL19-3114 | + | CGCGCGGCUGGGUGAGC | 17 | 3056 |
| HSV1-UL19-3115 | + | CGUUCGCGCGCGGCUGG | 17 | 3057 |
| HSV1-UL19-3116 | + | UCCACGUUCGCGCGCGG | 17 | 3058 |
| HSV1-UL19-3117 | + | AUGUCGGUGACGGGGUU | 17 | 3059 |
| HSV1-UL19-1799 | + | GUUGCCCAUGUCGGUGA | 17 | 2185 |
| HSV1-UL19-3119 | + | GGUUGCCCAUGUCGGUG | 17 | 3060 |
| HSV1-UL19-1802 | + | CCCAGGUAAAAGUUUUG | 17 | 2188 |
| HSV1-UL19-1803 | + | GCCCAGGUAAAAGUUUU | 17 | 2189 |
| HSV1-UL19-1804 | + | GGCCCAGGUAAAAGUUU | 17 | 2190 |
| HSV1-UL19-3123 | + | CGGCCCAGGUAAAAGUU | 17 | 3061 |
| HSV1-UL19-1807 | + | GCGUUGUCCAGGAGAGG | 17 | 2193 |
| HSV1-UL19-1808 | + | CGCGUUGUCCAGGAGAG | 17 | 2194 |
| HSV1-UL19-1809 | + | CCGCGUUGUCCAGGAGA | 17 | 2195 |
| HSV1-UL19-1810 | + | GCCGCGUUGUCCAGGAG | 17 | 2196 |
| HSV1-UL19-3128 | + | UGCCGCGUUGUCCAGGA | 17 | 3062 |
| HSV1-UL19-3129 | + | GCUGCCGCGUUGUCCAG | 17 | 3063 |
| HSV1-UL19-1811 | + | CGGCUGCCGCGUUGUCC | 17 | 2197 |
| HSV1-UL19-3131 | + | ACGGCUGCCGCGUUGUC | 17 | 3064 |
| HSV1-UL19-3132 | + | CCGAACACGGGGACGGG | 17 | 3065 |
| HSV1-UL19-3133 | + | CGCACCCGAACACGGGG | 17 | 3066 |
| HSV1-UL19-1822 | + | UGGGCGCACCCGAACAC | 17 | 2208 |
| HSV1-UL19-1823 | + | CUGGGCGCACCCGAACA | 17 | 2209 |
| HSV1-UL19-3136 | + | CCUGGGCGCACCCGAAC | 17 | 3067 |
| HSV1-UL19-3137 | + | GCGGCACCUGGGCGCAC | 17 | 3068 |
| HSV1-UL19-3138 | + | CCUGCGCGCCGCGGCAC | 17 | 3069 |
| HSV1-UL19-1833 | + | UUGACGUCGGUCGACAC | 17 | 2219 |
| HSV1-UL19-1834 | + | GUUGACGUCGGUCGACA | 17 | 2220 |
| HSV1-UL19-3141 | + | AGUUGACGUCGGUCGAC | 17 | 3070 |
| HSV1-UL19-1836 | + | GGGGGUUGCAGGGCCGG | 17 | 2222 |
| HSV1-UL19-3143 | + | CGGGGGUUGCAGGGCCG | 17 | 3071 |
| HSV1-UL19-3144 | + | CGCGUCCCCGGGGGUUG | 17 | 3072 |
| HSV1-UL19-1842 | + | CCCGGCGGCGCGUCCCC | 17 | 2228 |
| HSV1-UL19-1843 | + | CCCCGGCGGCGCGUCCC | 17 | 2229 |
| HSV1-UL19-3147 | + | CCCCCGGCGGCGCGUCC | 17 | 3073 |
| HSV1-UL19-3148 | + | UGGCCGUGGUCGUACAU | 17 | 3074 |
| HSV1-UL19-3149 | + | UCUGGCCGUGGUCGUAC | 17 | 3075 |
| HSV1-UL19-3150 | + | GGCCGCGAAGGCCCGGG | 17 | 3076 |
| HSV1-UL19-1854 | + | CCGUGGCCGCGAAGGCC | 17 | 2240 |
| HSV1-UL19-3152 | + | GCCGUGGCCGCGAAGGC | 17 | 3077 |
| HSV1-UL19-3153 | + | ACGGGUUGGCCGUGGCC | 17 | 3078 |
| HSV1-UL19-3154 | + | AAAUCGCUGCGACGCCC | 17 | 3079 |
| HSV1-UL19-3155 | + | AUAGAGCAGGUCCCCGU | 17 | 3080 |
| HSV1-UL19-3156 | + | GGUGGUAGGCCCCGUUA | 17 | 3081 |
| HSV1-UL19-3157 | + | GCACCGGCGAGGCCCCG | 17 | 3082 |
| HSV1-UL19-3158 | + | GCAGGGGCUGAGCACCG | 17 | 3083 |
| HSV1-UL19-3159 | + | AGAACUUAAAGCAGGGG | 17 | 3084 |
| HSV1-UL19-1868 | + | CGUAAAGAACUUAAAGC | 17 | 2254 |
| HSV1-UL19-3161 | + | ACGUAAAGAACUUAAAG | 17 | 3085 |
| HSV1-UL19-3162 | + | CGAUGUCGGCCGACGUA | 17 | 3086 |
| HSV1-UL19-3163 | + | GGUGGCCGUGGACACCG | 17 | 3087 |
| HSV1-UL19-3164 | + | CGCUGGCGGCGGUGGCC | 17 | 3088 |
| HSV1-UL19-1881 | + | GAGUUCGCGGCACCCCG | 17 | 2267 |
| HSV1-UL19-1882 | + | CGAGUUCGCGGCACCCC | 17 | 2268 |
| HSV1-UL19-1883 | + | ACGAGUUCGCGGCACCC | 17 | 2269 |
| HSV1-UL19-3168 | + | CACGAGUUCGCGGCACC | 17 | 3089 |
| HSV1-UL19-3169 | + | GGCCACACGGGUCCUCC | 17 | 3090 |
| HSV1-UL19-3170 | + | CUCCUGAAACAGGCCAC | 17 | 3091 |
| HSV1-UL19-3171 | + | GUGAGCGGGUAGGCCUC | 17 | 3092 |
| HSV1-UL19-3172 | + | GUCGCUGGCGCAGGUGA | 17 | 3093 |
| HSV1-UL19-3173 | + | CGGGGUCGCUGGCGCAG | 17 | 3094 |
| HSV1-UL19-1895 | + | GGCACUGCGGAGGAGGG | 17 | 2281 |
| HSV1-UL19-3175 | + | GGGCACUGCGGAGGAGG | 17 | 3095 |
| HSV1-UL19-3176 | + | UUGCGGGCACUGCGGAG | 17 | 3096 |
| HSV1-UL19-1898 | + | CGUUGCGGGCACUGCGG | 17 | 2284 |
| HSV1-UL19-3178 | + | CCGUUGCGGGCACUGCG | 17 | 3097 |
| HSV1-UL19-1899 | + | CCCCGUUGCGGGCACUG | 17 | 2285 |
| HSV1-UL19-3180 | + | UCCCCGUUGCGGGCACU | 17 | 3098 |
| HSV1-UL19-3181 | + | GCGUGGGCUUCCCCGUU | 17 | 3099 |
| HSV1-UL19-3182 | + | AAGUGGGUCUCCCGCGC | 17 | 3100 |
| HSV1-UL19-3183 | + | ACGAGAUACUGCGCGAA | 17 | 3101 |
| HSV1-UL19-3184 | + | CAUAGACGAGAUACUGC | 17 | 3102 |
| HSV1-UL19-3185 | + | GCGGGGAUGCGUCAUAG | 17 | 3103 |
| HSV1-UL19-1907 | + | GAGCCAGUCCCUUGAGC | 17 | 2293 |
| HSV1-UL19-1908 | + | AGAGCCAGUCCCUUGAG | 17 | 2294 |
| HSV1-UL19-3188 | + | CAGAGCCAGUCCCUUGA | 17 | 3104 |

**Table 1F** provides exemplary targeting domains for knocking out the *UL19* gene selected according to the first tier parameters. The targeting domains are selected based on location within first 500bp of the coding sequence of the *UL19* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1F**

| 1st Tier | | | | |
|---|---|---|---|---|
| gRNA Name | DNA Strand | Targeting Domain | Target Site Length | Seq ID |
| HSV1-UL19-3189 | - | GUGGCGUCGCAUCGACGCCU | 20 | 3105 |
| HSV1-UL19-3190 | - | ACAGUUCGAGGUCCACCAGC | 20 | 3106 |
| HSV1-UL19-3191 | - | ACCCACACACAAUUACAUGA | 20 | 3107 |
| HSV1-UL19-3192 | - | GCGUCGCAUCGACGCCU | 17 | 3108 |
| HSV1-UL19-3193 | - | GUUCGAGGUCCACCAGC | 17 | 3109 |
| HSV1-UL19-3194 | - | CACACACAAUUACAUGA | 17 | 3110 |

**Table 1G** provides exemplary targeting domains for knocking out the *UL19* gene selected according to the second tier parameters. The targeting domains are selected based on location within the coding sequence, but downstream of the first 500bp of the *UL19* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1G**

| 2nd Tier | | | | |
|---|---|---|---|---|
| gRNA Name | DNA Strand | Targeting Domain | Target Site Length | Seq ID |
| HSV1-UL19-3195 | - | CGGCCAACCCGUGGGCGUCG | 20 | 3111 |
| HSV1-UL19-3196 | - | GGCGGACUGGACCGUGCACC | 20 | 3112 |
| HSV1-UL19-3197 | - | CCAACCCGUGGGCGUCG | 17 | 3113 |
| HSV1-UL19-3198 | - | GGACUGGACCGUGCACC | 17 | 3114 |
| HSV1-UL19-3199 | + | GCGCGGCAAACCGUUCCAUG | 20 | 3115 |
| HSV1-UL19-3200 | + | GGGCCGCCACGUACGCCCCG | 20 | 3116 |
| HSV1-UL19-1181 | + | GCAGCGCCGGGUCUCGCAUU | 20 | 1567 |
| HSV1-UL19-3202 | + | UGGCGUUGACCGUGUUGGCC | 20 | 3117 |
| HSV1-UL19-3203 | + | CAGAUGCUGGGGGGCCAUCA | 20 | 3118 |
| HSV1-UL19-3204 | + | CGGCAAACCGUUCCAUG | 17 | 3119 |
| HSV1-UL19-3205 | + | CCGCCACGUACGCCCCG | 17 | 3120 |
| HSV1-UL19-1648 | + | GCGCCGGGUCUCGCAUU | 17 | 2034 |
| HSV1-UL19-3207 | + | CGUUGACCGUGUUGGCC | 17 | 3121 |
| HSV1-UL19-3208 | + | AUGCUGGGGGGCCAUCA | 17 | 3122 |

**Table 2A** provides exemplary targeting domains for knocking out the *UL30* gene selected according to first tier parameters. The targeting domains are selected based on location within the first 500bp of the coding sequence of the *UL30* gene and orthogonality against the human genome. It is contemplated herein that the targeting domain hybridizes to the target domain through complementary base pairing. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2A**

| 1st Tier | | | | |
|---|---|---|---|---|
| gRNA Name | DNA Strand | Targeting Domain | Target Site Length | Seq ID |
| HSV1-UL30-4 | - | GUCCCCCGGAGGAAAGUCGG | 20 | 3123 |
| HSV1-UL30-5 | - | CCGGAGGAAAGUCGGCGGCC | 20 | 3124 |
| HSV1-UL30-11 | - | UUUGCGCCCGCCGGCCCUCG | 20 | 3125 |
| HSV1-UL30-18 | - | GGGACGCAACAGAAGCCGAC | 20 | 3126 |
| HSV1-UL30-19 | - | GGACGCAACAGAAGCCGACC | 20 | 3127 |
| HSV1-UL30-20 | - | AAUUUCGAUUCAUCGCCCCG | 20 | 3128 |
| HSV1-UL30-21 | - | AUUUCGAUUCAUCGCCCCGC | 20 | 3129 |
| HSV1-UL30-22 | - | AUUCAUCGCCCCGCGGGUGC | 20 | 3130 |
| HSV1-UL30-25 | - | GCCCCCCCGGAGAAGCGCGC | 20 | 3131 |
| HSV1-UL30-27 | - | CCCCCCGGAGAAGCGCGCCG | 20 | 3132 |
| HSV1-UL30-28 | - | AAGCGCGCCGGGGUGCACGA | 20 | 3133 |
| HSV1-UL30-29 | - | UCACCUCAAGCGCGCCCCCA | 20 | 3134 |
| HSV1-UL30-30 | - | CGCGCCCCCAAGGUGUACUG | 20 | 3135 |
| HSV1-UL30-31 | - | GCGCCCCCAAGGUGUACUGC | 20 | 3136 |
| HSV1-UL30-32 | - | CGCCCCCAAGGUGUACUGCG | 20 | 3137 |
| HSV1-UL30-33 | - | GCCCCCAAGGUGUACUGCGG | 20 | 3138 |
| HSV1-UL30-36 | - | GAGCGCGACGUCCUCCGCGU | 20 | 3139 |
| HSV1-UL30-37 | - | AGCGCGACGUCCUCCGCGUC | 20 | 3140 |
| HSV1-UL30-38 | - | CGACGUCCUCCGCGUCGGGU | 20 | 3141 |
| HSV1-UL30-39 | - | GACGUCCUCCGCGUCGGGUC | 20 | 3142 |
| HSV1-UL30-40 | - | GUCCUCCGCGUCGGGUCGGG | 20 | 3143 |
| HSV1-UL30-50 | - | GGCGUGGACCACGCCCCGGC | 20 | 3144 |
| HSV1-UL30-51 | - | GCGUGGACCACGCCCCGGCG | 20 | 3145 |
| HSV1-UL30-52 | - | CUUUCACGUGUACGACAUCC | 20 | 3146 |
| HSV1-UL30-53 | - | GUACGACAUCCUGGAGAACG | 20 | 3147 |
| HSV1-UL30-54 | - | GAGAACGUGGAGCACGCGUA | 20 | 3148 |
| HSV1-UL30-56 | + | ACGCGUGCUCCACGUUCUCC | 20 | 3149 |
| HSV1-UL30-57 | + | GAUGUCGUACACGUGAAAGA | 20 | 3150 |
| HSV1-UL30-61 | + | CGUGAAAGACGGUGACGGUG | 20 | 3151 |
| HSV1-UL30-62 | + | ACGGUGGGGUUGAACCCCGC | 20 | 3152 |
| HSV1-UL30-64 | + | GGUGGGGUUGAACCCCGCCG | 20 | 3153 |
| HSV1-UL30-65 | + | GGUUGAACCCCGCCGGGGCG | 20 | 3154 |
| HSV1-UL30-68 | + | AGAAGCCGCCCGACCCGACG | 20 | 3155 |
| HSV1-UL30-69 | + | AGCCGCCCGACCCGACGCGG | 20 | 3156 |
| HSV1-UL30-70 | + | GUCCCCCCCGCAGUACACCU | 20 | 3157 |
| HSV1-UL30-74 | + | ACACCUUGGGGGCGCGCUUG | 20 | 3158 |
| HSV1-UL30-75 | + | GAGGUGACCGUCGUGCACCC | 20 | 3159 |
| HSV1-UL30-80 | + | CCCCGGCGCGCUUCUCCGGG | 20 | 3160 |
| HSV1-UL30-82 | + | GCAUCCUCGUCCAGCACCCG | 20 | 3161 |
| HSV1-UL30-84 | + | AUCCUCGUCCAGCACCCGCG | 20 | 3162 |
| HSV1-UL30-87 | + | GCUAUAGUACGUAUGGCGCU | 20 | 3163 |
| HSV1-UL30-88 | + | UAGUACGUAUGGCGCUGGGU | 20 | 3164 |
| HSV1-UL30-89 | + | CGUAUGGCGCUGGGUUGGCC | 20 | 3165 |
| HSV1-UL30-91 | + | GGCUUCUGUUGCGUCCCGAC | 20 | 3166 |
| HSV1-UL30-92 | + | GCUUCUGUUGCGUCCCGACU | 20 | 3167 |
| HSV1-UL30-93 | + | CUUCUGUUGCGUCCCGACUG | 20 | 3168 |
| HSV1-UL30-94 | + | GUUGCGUCCCGACUGGGGCG | 20 | 3169 |
| HSV1-UL30-95 | + | CGUCCCGACUGGGGCGAGGU | 20 | 3170 |
| HSV1-UL30-101 | + | GUUUUGCCUCAAACAAGGCG | 20 | 3171 |
| HSV1-UL30-102 | + | UUUUGCCUCAAACAAGGCGG | 20 | 3172 |
| HSV1-UL30-103 | + | UCAAACAAGGCGGGGGUCCC | 20 | 3173 |
| HSV1-UL30-110 | + | GCCGGCGGGCGCAAAAAACC | 20 | 3174 |
| HSV1-UL30-111 | + | AAAAAACCCGGACGCCGCCC | 20 | 3175 |
| HSV1-UL30-114 | + | UGGCCGCCGACUUUCCUCCG | 20 | 3176 |
| HSV1-UL30-115 | + | GGCCGCCGACUUUCCUCCGG | 20 | 3177 |
| HSV1-UL30-121 | - | GUCCCCCGGAGGAAAGU | 17 | 3178 |
| HSV1-UL30-125 | - | AAAGUCGGCGGCCAGGG | 17 | 3179 |
| HSV1-UL30-128 | - | GGGUUUUUUGCGCCCGC | 17 | 3180 |
| HSV1-UL30-136 | - | ACGCAACAGAAGCCGAC | 17 | 3181 |
| HSV1-UL30-137 | - | CGCAACAGAAGCCGACC | 17 | 3182 |
| HSV1-UL30-138 | - | UUCGAUUCAUCGCCCCG | 17 | 3183 |
| HSV1-UL30-139 | - | UCGAUUCAUCGCCCCGC | 17 | 3184 |
| HSV1-UL30-140 | - | CAUCGCCCCGCGGGUGC | 17 | 3185 |
| HSV1-UL30-144 | - | CCCCGGAGAAGCGCGCC | 17 | 3186 |
| HSV1-UL30-146 | - | CGCGCCGGGGUGCACGA | 17 | 3187 |
| HSV1-UL30-147 | - | CCUCAAGCGCGCCCCCA | 17 | 3188 |
| HSV1-UL30-148 | - | GCCCCCAAGGUGUACUG | 17 | 3189 |
| HSV1-UL30-150 | - | CCCCAAGGUGUACUGCG | 17 | 3190 |
| HSV1-UL30-155 | - | GCGACGUCCUCCGCGUC | 17 | 3191 |
| HSV1-UL30-156 | - | CGUCCUCCGCGUCGGGU | 17 | 3192 |
| HSV1-UL30-157 | - | GUCCUCCGCGUCGGGUC | 17 | 3193 |
| HSV1-UL30-158 | - | CUCCGCGUCGGGUCGGG | 17 | 3194 |
| HSV1-UL30-163 | - | CGGCGCUCGCGCCUGUG | 17 | 3195 |
| HSV1-UL30-164 | - | CGCUCGCGCCUGUGGGG | 17 | 3196 |
| HSV1-UL30-167 | - | CGUGGACCACGCCCCGG | 17 | 3197 |
| HSV1-UL30-168 | - | GUGGACCACGCCCCGGC | 17 | 3198 |
| HSV1-UL30-169 | - | UGGACCACGCCCCGGCG | 17 | 3199 |
| HSV1-UL30-170 | - | UCACGUGUACGACAUCC | 17 | 3200 |
| HSV1-UL30-171 | - | CGACAUCCUGGAGAACG | 17 | 3201 |
| HSV1-UL30-172 | - | AACGUGGAGCACGCGUA | 17 | 3202 |
| HSV1-UL30-175 | + | GUCGUACACGUGAAAGA | 17 | 3203 |
| HSV1-UL30-178 | + | UGAAAGACGGUGACGGU | 17 | 3204 |
| HSV1-UL30-182 | + | GGGGUUGAACCCCGCCG | 17 | 3205 |
| HSV1-UL30-185 | + | CACAGGCGCGAGCGCCG | 17 | 3206 |
| HSV1-UL30-186 | + | AGCCGCCCGACCCGACG | 17 | 3207 |
| HSV1-UL30-187 | + | CGCCCGACCCGACGCGG | 17 | 3208 |
| HSV1-UL30-188 | + | CCCCCCGCAGUACACCU | 17 | 3209 |
| HSV1-UL30-189 | + | CCCCCGCAGUACACCUU | 17 | 3210 |
| HSV1-UL30-190 | + | CCCCGCAGUACACCUUG | 17 | 3211 |
| HSV1-UL30-193 | + | GUGACCGUCGUGCACCC | 17 | 3212 |
| HSV1-UL30-196 | + | CCCGGCGCGCUUCUCCG | 17 | 3213 |
| HSV1-UL30-197 | + | CCGGCGCGCUUCUCCGG | 17 | 3214 |
| HSV1-UL30-198 | + | CGGCGCGCUUCUCCGGG | 17 | 3215 |
| HSV1-UL30-199 | + | GGCGCGCUUCUCCGGGG | 17 | 3216 |
| HSV1-UL30-202 | + | CUCGUCCAGCACCCGCG | 17 | 3217 |
| HSV1-UL30-203 | + | AUUCGCUAUAGUACGUA | 17 | 3218 |
| HSV1-UL30-204 | + | UAUAGUACGUAUGGCGC | 17 | 3219 |
| HSV1-UL30-205 | + | AUAGUACGUAUGGCGCU | 17 | 3220 |
| HSV1-UL30-206 | + | UACGUAUGGCGCUGGGU | 17 | 3221 |
| HSV1-UL30-209 | + | UUCUGUUGCGUCCCGAC | 17 | 3222 |
| HSV1-UL30-210 | + | UCUGUUGCGUCCCGACU | 17 | 3223 |
| HSV1-UL30-211 | + | CUGUUGCGUCCCGACUG | 17 | 3224 |
| HSV1-UL30-212 | + | GCGUCCCGACUGGGGCG | 17 | 3225 |
| HSV1-UL30-215 | + | CGACUGGGGCGAGGUAG | 17 | 3226 |
| HSV1-UL30-228 | + | GGCGGGCGCAAAAAACC | 17 | 3227 |
| HSV1-UL30-230 | + | GGCCGCCGACUUUCCUC | 17 | 3228 |
| HSV1-UL30-231 | + | GCCGCCGACUUUCCUCC | 17 | 3229 |
| HSV1-UL30-232 | + | CCGCCGACUUUCCUCCG | 17 | 3230 |
| HSV1-UL30-233 | + | CGCCGACUUUCCUCCGG | 17 | 3231 |

**Table 2B** provides exemplary targeting domains for knocking out the *UL30* gene selected according to the second tier parameters. The targeting domains are selected based on location within the first 500bp of the coding sequence of the *UL30* gene. It is contemplated herein that the targeting domain hybridizes to the target domain through complementary base pairing. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2B**

| 2nd Tier | | | | |
|---|---|---|---|---|
| gRNA Name | DNA Strand | Targeting Domain | Target Site Length | Seq ID |
| HSV1-UL30-1 | - | GGCGGCGGCCCGCUGUCCCC | 20 | 3232 |
| HSV1-UL30-2 | - | GGCGGCCCGCUGUCCCCCGG | 20 | 3233 |
| HSV1-UL30-3 | - | GCUGUCCCCCGGAGGAAAGU | 20 | 3234 |
| HSV1-UL30-6 | - | CGGAGGAAAGUCGGCGGCCA | 20 | 3235 |
| HSV1-UL30-7 | - | AGGAAAGUCGGCGGCCAGGG | 20 | 3236 |
| HSV1-UL30-8 | - | UCGGCGGCCAGGGCGGCGUC | 20 | 3237 |
| HSV1-UL30-9 | - | CGGCGGCCAGGGCGGCGUCC | 20 | 3238 |
| HSV1-UL30-10 | - | UCCGGGUUUUUUGCGCCCGC | 20 | 3239 |
| HSV1-UL30-12 | - | CCGGCCCUCGCGGAGCCAGC | 20 | 3240 |
| HSV1-UL30-13 | - | CGGCCCUCGCGGAGCCAGCC | 20 | 3241 |
| HSV1-UL30-14 | - | GGCCCUCGCGGAGCCAGCCG | 20 | 3242 |
| HSV1-UL30-15 | - | GGGGACCCCCGCCUUGUUUG | 20 | 3243 |
| HSV1-UL30-16 | - | AACCCCUACCUCGCCCCAGU | 20 | 3244 |
| HSV1-UL30-17 | - | ACCCCUACCUCGCCCCAGUC | 20 | 3245 |
| HSV1-UL30-23 | - | CGCCCCGCGGGUGCUGGACG | 20 | 3246 |
| HSV1-UL30-24 | - | GCUGGACGAGGAUGCCCCCC | 20 | 3247 |
| HSV1-UL30-26 | - | CCCCCCCGGAGAAGCGCGCC | 20 | 3248 |
| HSV1-UL30-34 | - | CCCCCAAGGUGUACUGCGGG | 20 | 3249 |
| HSV1-UL30-35 | - | CCCCAAGGUGUACUGCGGGG | 20 | 3250 |
| HSV1-UL30-41 | - | GCGUCGGGUCGGGCGGCUUC | 20 | 3251 |
| HSV1-UL30-42 | - | GGUCGGGCGGCUUCUGGCCG | 20 | 3252 |
| HSV1-UL30-43 | - | GGCCGCGGCGCUCGCGCCUG | 20 | 3253 |
| HSV1-UL30-44 | - | GCCGCGGCGCUCGCGCCUGU | 20 | 3254 |
| HSV1-UL30-45 | - | CCGCGGCGCUCGCGCCUGUG | 20 | 3255 |
| HSV1-UL30-46 | - | CGGCGCUCGCGCCUGUGGGG | 20 | 3256 |
| HSV1-UL30-47 | - | CUCGCGCCUGUGGGGCGGCG | 20 | 3257 |
| HSV1-UL30-48 | - | GGGCGGCGUGGACCACGCCC | 20 | 3258 |
| HSV1-UL30-49 | - | CGGCGUGGACCACGCCCCGG | 20 | 3259 |
| HSV1-UL30-55 | - | GCACGCGUACGGCAUGCGCG | 20 | 3260 |
| HSV1-UL30-58 | + | GUACACGUGAAAGACGGUGA | 20 | 3261 |
| HSV1-UL30-59 | + | CACGUGAAAGACGGUGACGG | 20 | 3262 |
| HSV1-UL30-60 | + | ACGUGAAAGACGGUGACGGU | 20 | 3263 |
| HSV1-UL30-63 | + | CGGUGGGGUUGAACCCCGCC | 20 | 3264 |
| HSV1-UL30-66 | + | CGUGGUCCACGCCGCCCCAC | 20 | 3265 |
| HSV1-UL30-67 | + | CCCCACAGGCGCGAGCGCCG | 20 | 3266 |
| HSV1-UL30-71 | + | UCCCCCCCGCAGUACACCUU | 20 | 3267 |
| HSV1-UL30-72 | + | CCCCCCCGCAGUACACCUUG | 20 | 3268 |
| HSV1-UL30-73 | + | CCCCCCGCAGUACACCUUGG | 20 | 3269 |
| HSV1-UL30-76 | + | UGCACCCCGGCGCGCUUCUC | 20 | 3270 |
| HSV1-UL30-77 | + | GCACCCCGGCGCGCUUCUCC | 20 | 3271 |
| HSV1-UL30-78 | + | CACCCCGGCGCGCUUCUCCG | 20 | 3272 |
| HSV1-UL30-79 | + | ACCCCGGCGCGCUUCUCCGG | 20 | 3273 |
| HSV1-UL30-81 | + | CCCGGCGCGCUUCUCCGGGG | 20 | 3274 |
| HSV1-UL30-83 | + | CAUCCUCGUCCAGCACCCGC | 20 | 3275 |
| HSV1-UL30-85 | + | CGCAUUCGCUAUAGUACGUA | 20 | 3276 |
| HSV1-UL30-86 | + | CGCUAUAGUACGUAUGGCGC | 20 | 3277 |
| HSV1-UL30-90 | + | UGGCGCUGGGUUGGCCCGGU | 20 | 3278 |
| HSV1-UL30-96 | + | GUCCCGACUGGGGCGAGGUA | 20 | 3279 |
| HSV1-UL30-97 | + | UCCCGACUGGGGCGAGGUAG | 20 | 3280 |
| HSV1-UL30-98 | + | UAAAAGUUUUGCCUCAAACA | 20 | 3281 |
| HSV1-UL30-99 | + | AAGUUUUGCCUCAAACAAGG | 20 | 3282 |
| HSV1-UL30-100 | + | AGUUUUGCCUCAAACAAGGC | 20 | 3283 |
| HSV1-UL30-104 | + | ACAAGGCGGGGGUCCCCGGC | 20 | 3284 |
| HSV1-UL30-105 | + | GGUCCCCGGCUGGCUCCGCG | 20 | 3285 |
| HSV1-UL30-106 | + | GUCCCCGGCUGGCUCCGCGA | 20 | 3286 |
| HSV1-UL30-107 | + | CCGGCUGGCUCCGCGAGGGC | 20 | 3287 |
| HSV1-UL30-108 | + | GCUGGCUCCGCGAGGGCCGG | 20 | 3288 |
| HSV1-UL30-109 | + | CUGGCUCCGCGAGGGCCGGC | 20 | 3289 |
| HSV1-UL30-112 | + | CCUGGCCGCCGACUUUCCUC | 20 | 3290 |
| HSV1-UL30-113 | + | CUGGCCGCCGACUUUCCUCC | 20 | 3291 |
| HSV1-UL30-116 | + | GACUUUCCUCCGGGGGACAG | 20 | 3292 |
| HSV1-UL30-117 | + | ACUUUCCUCCGGGGGACAGC | 20 | 3293 |
| HSV1-UL30-118 | + | GGACAGCGGGCCGCCGCCAC | 20 | 3294 |
| HSV1-UL30-119 | - | GGCGGCCCGCUGUCCCC | 17 | 3295 |
| HSV1-UL30-120 | - | GGCCCGCUGUCCCCCGG | 17 | 3296 |
| HSV1-UL30-122 | - | CCCCGGAGGAAAGUCGG | 17 | 3297 |
| HSV1-UL30-123 | - | GAGGAAAGUCGGCGGCC | 17 | 3298 |
| HSV1-UL30-124 | - | AGGAAAGUCGGCGGCCA | 17 | 3299 |
| HSV1-UL30-126 | - | GCGGCCAGGGCGGCGUC | 17 | 3300 |
| HSV1-UL30-127 | - | CGGCCAGGGCGGCGUCC | 17 | 3301 |
| HSV1-UL30-129 | - | GCGCCCGCCGGCCCUCG | 17 | 3302 |
| HSV1-UL30-130 | - | GCCCUCGCGGAGCCAGC | 17 | 3303 |
| HSV1-UL30-131 | - | CCCUCGCGGAGCCAGCC | 17 | 3304 |
| HSV1-UL30-132 | - | CCUCGCGGAGCCAGCCG | 17 | 3305 |
| HSV1-UL30-133 | - | GACCCCCGCCUUGUUUG | 17 | 3306 |
| HSV1-UL30-134 | - | CCCUACCUCGCCCCAGU | 17 | 3307 |
| HSV1-UL30-135 | - | CCUACCUCGCCCCAGUC | 17 | 3308 |
| HSV1-UL30-141 | - | CCCGCGGGUGCUGGACG | 17 | 3309 |
| HSV1-UL30-142 | - | GGACGAGGAUGCCCCCC | 17 | 3310 |
| HSV1-UL30-143 | - | CCCCCGGAGAAGCGCGC | 17 | 3311 |
| HSV1-UL30-145 | - | CCCGGAGAAGCGCGCCG | 17 | 3312 |
| HSV1-UL30-149 | - | CCCCCAAGGUGUACUGC | 17 | 3313 |
| HSV1-UL30-151 | - | CCCAAGGUGUACUGCGG | 17 | 3314 |
| HSV1-UL30-152 | - | CCAAGGUGUACUGCGGG | 17 | 3315 |
| HSV1-UL30-153 | - | CAAGGUGUACUGCGGGG | 17 | 3316 |
| HSV1-UL30-154 | - | CGCGACGUCCUCCGCGU | 17 | 3317 |
| HSV1-UL30-159 | - | UCGGGUCGGGCGGCUUC | 17 | 3318 |
| HSV1-UL30-160 | - | CGGGCGGCUUCUGGCCG | 17 | 3319 |
| HSV1-UL30-161 | - | CGCGGCGCUCGCGCCUG | 17 | 3320 |
| HSV1-UL30-162 | - | GCGGCGCUCGCGCCUGU | 17 | 3321 |
| HSV1-UL30-165 | - | GCGCCUGUGGGGCGGCG | 17 | 3322 |
| HSV1-UL30-166 | - | CGGCGUGGACCACGCCC | 17 | 3323 |
| HSV1-UL30-173 | - | CGCGUACGGCAUGCGCG | 17 | 3324 |
| HSV1-UL30-174 | + | CGUGCUCCACGUUCUCC | 17 | 3325 |
| HSV1-UL30-176 | + | CACGUGAAAGACGGUGA | 17 | 3326 |
| HSV1-UL30-177 | + | GUGAAAGACGGUGACGG | 17 | 3327 |
| HSV1-UL30-179 | + | GAAAGACGGUGACGGUG | 17 | 3328 |
| HSV1-UL30-180 | + | GUGGGGUUGAACCCCGC | 17 | 3329 |
| HSV1-UL30-181 | + | UGGGGUUGAACCCCGCC | 17 | 3330 |
| HSV1-UL30-183 | + | UGAACCCCGCCGGGGCG | 17 | 3331 |
| HSV1-UL30-184 | + | GGUCCACGCCGCCCCAC | 17 | 3332 |
| HSV1-UL30-191 | + | CCCGCAGUACACCUUGG | 17 | 3333 |
| HSV1-UL30-192 | + | CCUUGGGGGCGCGCUUG | 17 | 3334 |
| HSV1-UL30-194 | + | ACCCCGGCGCGCUUCUC | 17 | 3335 |
| HSV1-UL30-195 | + | CCCCGGCGCGCUUCUCC | 17 | 3336 |
| HSV1-UL30-200 | + | UCCUCGUCCAGCACCCG | 17 | 3337 |
| HSV1-UL30-201 | + | CCUCGUCCAGCACCCGC | 17 | 3338 |
| HSV1-UL30-207 | + | AUGGCGCUGGGUUGGCC | 17 | 3339 |
| HSV1-UL30-208 | + | CGCUGGGUUGGCCCGGU | 17 | 3340 |
| HSV1-UL30-213 | + | CCCGACUGGGGCGAGGU | 17 | 3341 |
| HSV1-UL30-214 | + | CCGACUGGGGCGAGGUA | 17 | 3342 |
| HSV1-UL30-216 | + | AAGUUUUGCCUCAAACA | 17 | 3343 |
| HSV1-UL30-217 | + | UUUUGCCUCAAACAAGG | 17 | 3344 |
| HSV1-UL30-218 | + | UUUGCCUCAAACAAGGC | 17 | 3345 |
| HSV1-UL30-219 | + | UUGCCUCAAACAAGGCG | 17 | 3346 |
| HSV1-UL30-220 | + | UGCCUCAAACAAGGCGG | 17 | 3347 |
| HSV1-UL30-221 | + | AACAAGGCGGGGGUCCC | 17 | 3348 |
| HSV1-UL30-222 | + | AGGCGGGGGUCCCCGGC | 17 | 3349 |
| HSV1-UL30-223 | + | CCCCGGCUGGCUCCGCG | 17 | 3350 |
| HSV1-UL30-224 | + | CCCGGCUGGCUCCGCGA | 17 | 3351 |
| HSV1-UL30-225 | + | GCUGGCUCCGCGAGGGC | 17 | 3352 |
| HSV1-UL30-226 | + | GGCUCCGCGAGGGCCGG | 17 | 3353 |
| HSV1-UL30-227 | + | GCUCCGCGAGGGCCGGC | 17 | 3354 |
| HSV1-UL30-229 | + | AAACCCGGACGCCGCCC | 17 | 3355 |
| HSV1-UL30-234 | + | UUUCCUCCGGGGGACAG | 17 | 3356 |
| HSV1-UL30-235 | + | UUCCUCCGGGGGACAGC | 17 | 3357 |
| HSV1-UL30-236 | + | CAGCGGGCCGCCGCCAC | 17 | 3358 |

**Table 2C** provides exemplary targeting domains for knocking out the *UL30* gene selected according to the third tier parameters. The targeting domains are selected based on location within the coding sequence, but downstream of the first 500bp of the *UL30* gene. It is contemplated herein that the targeting domain hybridizes to the target domain through complementary base pairing. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2C**

| 3rd Tier | | | | |
|---|---|---|---|---|
| gRNA Name | DNA Strand | Targeting Domain | Target Site Length | Seq ID |
| HSV1-UL30-421 | - | TATGGACGCCATCACACCGA | 20 | 3359 |
| HSV1-UL30-422 | - | ATGGACGCCATCACACCGAC | 20 | 3360 |
| HSV1-UL30-423 | - | TGGACGCCATCACACCGACG | 20 | 3361 |
| HSV1-UL30-424 | - | GGGGACCGTCATCACGCTCC | 20 | 3362 |
| HSV1-UL30-425 | - | GGGACCGTCATCACGCTCCT | 20 | 3363 |
| HSV1-UL30-426 | - | CACGCTCCTGGGCCTGACTC | 20 | 3364 |
| HSV1-UL30-427 | - | CTCCTGGGCCTGACTCCGGA | 20 | 3365 |
| HSV1-UL30-428 | - | GCCTGACTCCGGAAGGCCAC | 20 | 3366 |
| HSV1-UL30-429 | - | CCTGACTCCGGAAGGCCACC | 20 | 3367 |
| HSV1-UL30-430 | - | GACTCCGGAAGGCCACCGGG | 20 | 3368 |
| HSV1-UL30-431 | - | CGGGTGGCCGTTCACGTTTA | 20 | 3369 |
| HSV1-UL30-432 | - | CCGTTCACGTTTACGGCACG | 20 | 3370 |
| HSV1-UL30-433 | - | GCAGTACTTTTACATGAACA | 20 | 3371 |
| HSV1-UL30-434 | - | GTACTTTTACATGAACAAGG | 20 | 3372 |
| HSV1-UL30-435 | - | CTTTTACATGAACAAGGAGG | 20 | 3373 |
| HSV1-UL30-436 | - | TGAACAAGGAGGAGGTCGAC | 20 | 3374 |
| HSV1-UL30-437 | - | ACGAGATCTCTGCGAGCGCA | 20 | 3375 |
| HSV1-UL30-438 | - | TCTCTGCGAGCGCATGGCCG | 20 | 3376 |
| HSV1-UL30-439 | - | CGCGGCCCTGCGCGAGTCCC | 20 | 3377 |
| HSV1-UL30-440 | - | GCGGCCCTGCGCGAGTCCCC | 20 | 3378 |
| HSV1-UL30-441 | - | TCCCCGGGCGCGTCGTTCCG | 20 | 3379 |
| HSV1-UL30-442 | - | GTCGTTCCGCGGCATCTCCG | 20 | 3380 |
| HSV1-UL30-443 | - | CATCTCCGCGGACCACTTCG | 20 | 3381 |
| HSV1-UL30-444 | - | CTCCGCGGACCACTTCGAGG | 20 | 3382 |
| HSV1-UL30-445 | - | CGCGGACCACTTCGAGGCGG | 20 | 3383 |
| HSV1-UL30-446 | - | GGACCACTTCGAGGCGGAGG | 20 | 3384 |
| HSV1-UL30-447 | - | CCACTTCGAGGCGGAGGTGG | 20 | 3385 |
| HSV1-UL30-448 | - | TACCGCGTCTACGTCCGAAG | 20 | 3386 |
| HSV1-UL30-449 | - | ACCGCGTCTACGTCCGAAGC | 20 | 3387 |
| HSV1-UL30-450 | - | CCTGTGCGACAACTTCTGCC | 20 | 3388 |
| HSV1-UL30-451 | - | CCCGGCCATCAAGAAGTACG | 20 | 3389 |
| HSV1-UL30-452 | - | CCGGCCATCAAGAAGTACGA | 20 | 3390 |
| HSV1-UL30-453 | - | GCCATCAAGAAGTACGAGGG | 20 | 3391 |
| HSV1-UL30-454 | - | CCATCAAGAAGTACGAGGGT | 20 | 3392 |
| HSV1-UL30-455 | - | CATCAAGAAGTACGAGGGTG | 20 | 3393 |
| HSV1-UL30-456 | - | GTGGGGTCGACGCCACCACC | 20 | 3394 |
| HSV1-UL30-457 | - | CGCCACCACCCGGTTCATCC | 20 | 3395 |
| HSV1-UL30-458 | - | CGGTTCATCCTGGACAACCC | 20 | 3396 |
| HSV1-UL30-459 | - | GGTTCATCCTGGACAACCCC | 20 | 3397 |
| HSV1-UL30-460 | - | AACCCCGGGTTCGTCACCTT | 20 | 3398 |
| HSV1-UL30-461 | - | CCGGGTTCGTCACCTTCGGC | 20 | 3399 |
| HSV1-UL30-462 | - | CGGCTGGTACCGTCTCAAAC | 20 | 3400 |
| HSV1-UL30-463 | - | GGCTGGTACCGTCTCAAACC | 20 | 3401 |
| HSV1-UL30-464 | - | GGTACCGTCTCAAACCGGGC | 20 | 3402 |
| HSV1-UL30-465 | - | GAACAACACGCTAGCCCAGC | 20 | 3403 |
| HSV1-UL30-466 | - | CAACACGCTAGCCCAGCCGG | 20 | 3404 |
| HSV1-UL30-467 | - | AGCCCAGCCGGCGGCCCCGA | 20 | 3405 |
| HSV1-UL30-468 | - | CCGGCGGCCCCGATGGCCTT | 20 | 3406 |
| HSV1-UL30-469 | - | CGGCGGCCCCGATGGCCTTC | 20 | 3407 |
| HSV1-UL30-470 | - | CGACGTCGAGTTTAACTGTA | 20 | 3408 |
| HSV1-UL30-471 | - | CGTCGAGTTTAACTGTACGG | 20 | 3409 |
| HSV1-UL30-472 | - | TAACTGTACGGCGGACAACC | 20 | 3410 |
| HSV1-UL30-473 | - | GGCGGACAACCTGGCCATCG | 20 | 3411 |
| HSV1-UL30-474 | - | GCGGACAACCTGGCCATCGA | 20 | 3412 |
| HSV1-UL30-475 | - | CGGACAACCTGGCCATCGAG | 20 | 3413 |
| HSV1-UL30-476 | - | GGACAACCTGGCCATCGAGG | 20 | 3414 |
| HSV1-UL30-477 | - | GACAACCTGGCCATCGAGGG | 20 | 3415 |
| HSV1-UL30-478 | - | GGGGGGCATGAGCGACCTAC | 20 | 3416 |
| HSV1-UL30-479 | - | GTGCTTCGATATCGAATGCA | 20 | 3417 |
| HSV1-UL30-480 | - | CTTCGATATCGAATGCAAGG | 20 | 3418 |
| HSV1-UL30-481 | - | TTCGATATCGAATGCAAGGC | 20 | 3419 |
| HSV1-UL30-482 | - | TCGATATCGAATGCAAGGCG | 20 | 3420 |
| HSV1-UL30-483 | - | CGATATCGAATGCAAGGCGG | 20 | 3421 |
| HSV1-UL30-484 | - | GATATCGAATGCAAGGCGGG | 20 | 3422 |
| HSV1-UL30-485 | - | ATATCGAATGCAAGGCGGGG | 20 | 3423 |
| HSV1-UL30-486 | - | TATCGAATGCAAGGCGGGGG | 20 | 3424 |
| HSV1-UL30-487 | - | CGAATGCAAGGCGGGGGGGG | 20 | 3425 |
| HSV1-UL30-488 | - | GGCGGGGGGGGAGGACGAGC | 20 | 3426 |
| HSV1-UL30-489 | - | GGAGGACGAGCTGGCCTTTC | 20 | 3427 |
| HSV1-UL30-490 | - | GGACGAGCTGGCCTTTCCGG | 20 | 3428 |
| HSV1-UL30-491 | - | GAGCTGGCCTTTCCGGTGGC | 20 | 3429 |
| HSV1-UL30-492 | - | AGCTGGCCTTTCCGGTGGCC | 20 | 3430 |
| HSV1-UL30-493 | - | CTTTCCGGTGGCCGGGCACC | 20 | 3431 |
| HSV1-UL30-494 | - | TCCGGTGGCCGGGCACCCGG | 20 | 3432 |
| HSV1-UL30-495 | - | GGCCGGGCACCCGGAGGACC | 20 | 3433 |
| HSV1-UL30-496 | - | CGACCTGTCCACCACCGCCC | 20 | 3434 |
| HSV1-UL30-497 | - | CACGTCCTCCTGTTTTCGCT | 20 | 3435 |
| HSV1-UL30-498 | - | CGAATCCCACCTGAACGAGC | 20 | 3436 |
| HSV1-UL30-499 | - | ATCCCACCTGAACGAGCTGG | 20 | 3437 |
| HSV1-UL30-500 | - | ACCTGAACGAGCTGGCGGCC | 20 | 3438 |
| HSV1-UL30-501 | - | CCTGAACGAGCTGGCGGCCA | 20 | 3439 |
| HSV1-UL30-502 | - | CTGAACGAGCTGGCGGCCAG | 20 | 3440 |
| HSV1-UL30-503 | - | CAGGGGCCTGCCCACGCCCG | 20 | 3441 |
| HSV1-UL30-504 | - | CCTGCCCACGCCCGTGGTTC | 20 | 3442 |
| HSV1-UL30-505 | - | CAGCGAATTCGAGATGCTGT | 20 | 3443 |
| HSV1-UL30-506 | - | ATGACCCTTGTGAAACAGTA | 20 | 3444 |
| HSV1-UL30-507 | - | TACGGCCCCGAGTTCGTGAC | 20 | 3445 |
| HSV1-UL30-508 | - | ACGGCCCCGAGTTCGTGACC | 20 | 3446 |
| HSV1-UL30-509 | - | ACAACATCATCAACTTCGAC | 20 | 3447 |
| HSV1-UL30-510 | - | CTTCGACTGGCCCTTCTTGC | 20 | 3448 |
| HSV1-UL30-511 | - | CTTCTTGCTGGCCAAGCTGA | 20 | 3449 |
| HSV1-UL30-512 | - | CAAGCTGACGGACATTTACA | 20 | 3450 |
| HSV1-UL30-513 | - | GGACATTTACAAGGTCCCCC | 20 | 3451 |
| HSV1-UL30-514 | - | ATTTACAAGGTCCCCCTGGA | 20 | 3452 |
| HSV1-UL30-515 | - | TTTACAAGGTCCCCCTGGAC | 20 | 3453 |
| HSV1-UL30-516 | - | AAGGTCCCCCTGGACGGGTA | 20 | 3454 |
| HSV1-UL30-517 | - | GACGGGTACGGCCGCATGAA | 20 | 3455 |
| HSV1-UL30-518 | - | GGTACGGCCGCATGAACGGC | 20 | 3456 |
| HSV1-UL30-519 | - | GTACGGCCGCATGAACGGCC | 20 | 3457 |
| HSV1-UL30-520 | - | TACGGCCGCATGAACGGCCG | 20 | 3458 |
| HSV1-UL30-521 | - | GCCGGGGCGTGTTTCGCGTG | 20 | 3459 |
| HSV1-UL30-522 | - | CCGGGGCGTGTTTCGCGTGT | 20 | 3460 |
| HSV1-UL30-523 | - | GTGTTTCGCGTGTGGGACAT | 20 | 3461 |
| HSV1-UL30-524 | - | CCAGAAGCGCAGCAAGATAA | 20 | 3462 |
| HSV1-UL30-525 | - | CGCAGCAAGATAAAGGTGAA | 20 | 3463 |
| HSV1-UL30-526 | - | CAAGATAAAGGTGAACGGCA | 20 | 3464 |
| HSV1-UL30-527 | - | ATGGTGAACATCGACATGTA | 20 | 3465 |
| HSV1-UL30-528 | - | TGGTGAACATCGACATGTAC | 20 | 3466 |
| HSV1-UL30-529 | - | GAGCTACAAGCTCAACGCCG | 20 | 3467 |
| HSV1-UL30-530 | - | CGTGGCCGAAGCCGTCCTGA | 20 | 3468 |
| HSV1-UL30-531 | - | CGTCCTGAAGGACAAGAAGA | 20 | 3469 |
| HSV1-UL30-532 | - | ATCCCCGCCTACTACGCCGC | 20 | 3470 |
| HSV1-UL30-533 | - | TCCCCGCCTACTACGCCGCC | 20 | 3471 |
| HSV1-UL30-534 | - | GCCGCCGGGCCCGCGCAACG | 20 | 3472 |
| HSV1-UL30-535 | - | CCGCCGGGCCCGCGCAACGC | 20 | 3473 |
| HSV1-UL30-536 | - | CGCCGGGCCCGCGCAACGCG | 20 | 3474 |
| HSV1-UL30-537 | - | CCCGCGCAACGCGGGGTGAT | 20 | 3475 |
| HSV1-UL30-538 | - | GATCGGCGAGTACTGCATAC | 20 | 3476 |
| HSV1-UL30-539 | - | CTGCATACAGGATTCCCTGC | 20 | 3477 |
| HSV1-UL30-540 | - | CATACAGGATTCCCTGCTGG | 20 | 3478 |
| HSV1-UL30-541 | - | ATACAGGATTCCCTGCTGGT | 20 | 3479 |
| HSV1-UL30-542 | - | TTTTAAGTTTTTGCCCCATC | 20 | 3480 |
| HSV1-UL30-543 | - | TTTGCCCCATCTGGAGCTCT | 20 | 3481 |
| HSV1-UL30-544 | - | GCTCTCGGCCGTCGCGCGCT | 20 | 3482 |
| HSV1-UL30-545 | - | CTCGGCCGTCGCGCGCTTGG | 20 | 3483 |
| HSV1-UL30-546 | - | TCGGCCGTCGCGCGCTTGGC | 20 | 3484 |
| HSV1-UL30-547 | - | ATCACCCGCACCATCTACGA | 20 | 3485 |
| HSV1-UL30-548 | - | CTTTACGTGCCTGCTGCGCC | 20 | 3486 |
| HSV1-UL30-549 | - | GCTGCGCCTGGCCGACCAGA | 20 | 3487 |
| HSV1-UL30-550 | - | CTGCGCCTGGCCGACCAGAA | 20 | 3488 |
| HSV1-UL30-551 | - | CCAGAAGGGCTTTATTCTGC | 20 | 3489 |
| HSV1-UL30-552 | - | CTTTATTCTGCCGGACACCC | 20 | 3490 |
| HSV1-UL30-553 | - | TTTATTCTGCCGGACACCCA | 20 | 3491 |
| HSV1-UL30-554 | - | TTATTCTGCCGGACACCCAG | 20 | 3492 |
| HSV1-UL30-555 | - | CGGACACCCAGGGGCGATTT | 20 | 3493 |
| HSV1-UL30-556 | - | GGACACCCAGGGGCGATTTA | 20 | 3494 |
| HSV1-UL30-557 | - | GACACCCAGGGGCGATTTAG | 20 | 3495 |
| HSV1-UL30-558 | - | CAGGGGCGATTTAGGGGCGC | 20 | 3496 |
| HSV1-UL30-559 | - | AGGGGCGATTTAGGGGCGCC | 20 | 3497 |
| HSV1-UL30-560 | - | GGGGCGATTTAGGGGCGCCG | 20 | 3498 |
| HSV1-UL30-561 | - | GGGCGATTTAGGGGCGCCGG | 20 | 3499 |
| HSV1-UL30-562 | - | GGCGATTTAGGGGCGCCGGG | 20 | 3500 |
| HSV1-UL30-563 | - | GCGATTTAGGGGCGCCGGGG | 20 | 3501 |
| HSV1-UL30-564 | - | ATTTAGGGGCGCCGGGGGGG | 20 | 3502 |
| HSV1-UL30-565 | - | GGGGGAGGCGCCCAAGCGTC | 20 | 3503 |
| HSV1-UL30-566 | - | CCAAGCGTCCGGCCGCAGCC | 20 | 3504 |
| HSV1-UL30-567 | - | CAAGCGTCCGGCCGCAGCCC | 20 | 3505 |
| HSV1-UL30-568 | - | GCGTCCGGCCGCAGCCCGGG | 20 | 3506 |
| HSV1-UL30-569 | - | GGCCGCAGCCCGGGAGGACG | 20 | 3507 |
| HSV1-UL30-570 | - | CAGCCCGGGAGGACGAGGAG | 20 | 3508 |
| HSV1-UL30-571 | - | GGAGGACGAGGAGCGGCCAG | 20 | 3509 |
| HSV1-UL30-572 | - | GGACGAGGAGCGGCCAGAGG | 20 | 3510 |
| HSV1-UL30-573 | - | CGAGGAGCGGCCAGAGGAGG | 20 | 3511 |
| HSV1-UL30-574 | - | GAGGAGCGGCCAGAGGAGGA | 20 | 3512 |
| HSV1-UL30-575 | - | AGGAGCGGCCAGAGGAGGAG | 20 | 3513 |
| HSV1-UL30-576 | - | GGAGCGGCCAGAGGAGGAGG | 20 | 3514 |
| HSV1-UL30-577 | - | GCGGCCAGAGGAGGAGGGGG | 20 | 3515 |
| HSV1-UL30-578 | - | AGAGGAGGAGGGGGAGGACG | 20 | 3516 |
| HSV1-UL30-579 | - | GGAGGACGAGGACGAACGCG | 20 | 3517 |
| HSV1-UL30-580 | - | GGACGAGGACGAACGCGAGG | 20 | 3518 |
| HSV1-UL30-581 | - | GACGAGGACGAACGCGAGGA | 20 | 3519 |
| HSV1-UL30-582 | - | GAGGACGAACGCGAGGAGGG | 20 | 3520 |
| HSV1-UL30-583 | - | AGGACGAACGCGAGGAGGGC | 20 | 3521 |
| HSV1-UL30-584 | - | GGACGAACGCGAGGAGGGCG | 20 | 3522 |
| HSV1-UL30-585 | - | GACGAACGCGAGGAGGGCGG | 20 | 3523 |
| HSV1-UL30-586 | - | GCGAGGAGGGCGGGGGCGAG | 20 | 3524 |
| HSV1-UL30-587 | - | CGAGGAGGGCGGGGGCGAGC | 20 | 3525 |
| HSV1-UL30-588 | - | GGGCGGGGGCGAGCGGGAGC | 20 | 3526 |
| HSV1-UL30-589 | - | CGGGGGCGAGCGGGAGCCGG | 20 | 3527 |
| HSV1-UL30-590 | - | GGGGGCGAGCGGGAGCCGGA | 20 | 3528 |
| HSV1-UL30-591 | - | AGCGGGAGCCGGAGGGCGCG | 20 | 3529 |
| HSV1-UL30-592 | - | GCGGGAGCCGGAGGGCGCGC | 20 | 3530 |
| HSV1-UL30-593 | - | GAGGGCGCGCGGGAGACCGC | 20 | 3531 |
| HSV1-UL30-594 | - | GCGCGCGGGAGACCGCCGGC | 20 | 3532 |
| HSV1-UL30-595 | - | GGAGACCGCCGGCAGGCACG | 20 | 3533 |
| HSV1-UL30-596 | - | GAGACCGCCGGCAGGCACGT | 20 | 3534 |
| HSV1-UL30-597 | - | AGACCGCCGGCAGGCACGTG | 20 | 3535 |
| HSV1-UL30-598 | - | CGGCAGGCACGTGGGGTACC | 20 | 3536 |
| HSV1-UL30-599 | - | GGCAGGCACGTGGGGTACCA | 20 | 3537 |
| HSV1-UL30-600 | - | GCAGGCACGTGGGGTACCAG | 20 | 3538 |
| HSV1-UL30-601 | - | CAGGCACGTGGGGTACCAGG | 20 | 3539 |
| HSV1-UL30-602 | - | ACGTGGGGTACCAGGGGGCC | 20 | 3540 |
| HSV1-UL30-603 | - | CGTGGGGTACCAGGGGGCCA | 20 | 3541 |
| HSV1-UL30-604 | - | AGGGTCCTTGACCCCACTTC | 20 | 3542 |
| HSV1-UL30-605 | - | GGGTCCTTGACCCCACTTCC | 20 | 3543 |
| HSV1-UL30-606 | - | CGGGTTTCACGTGAACCCCG | 20 | 3544 |
| HSV1-UL30-607 | - | GTTTCACGTGAACCCCGTGG | 20 | 3545 |
| HSV1-UL30-608 | - | CCTGTACCCCAGCATCATCC | 20 | 3546 |
| HSV1-UL30-609 | - | GCTTCAGCACGCTCTCCCTG | 20 | 3547 |
| HSV1-UL30-610 | - | CTTCAGCACGCTCTCCCTGA | 20 | 3548 |
| HSV1-UL30-611 | - | CTCCCTGAGGGCCGACGCAG | 20 | 3549 |
| HSV1-UL30-612 | - | GGCCGACGCAGTGGCGCACC | 20 | 3550 |
| HSV1-UL30-613 | - | CGACGCAGTGGCGCACCTGG | 20 | 3551 |
| HSV1-UL30-614 | - | CGCAGTGGCGCACCTGGAGG | 20 | 3552 |
| HSV1-UL30-615 | - | GCAGTGGCGCACCTGGAGGC | 20 | 3553 |
| HSV1-UL30-616 | - | GGCGCACCTGGAGGCGGGCA | 20 | 3554 |
| HSV1-UL30-617 | - | GGAGGCGGGCAAGGACTACC | 20 | 3555 |
| HSV1-UL30-618 | - | CAAGGACTACCTGGAGATCG | 20 | 3556 |
| HSV1-UL30-619 | - | GGACTACCTGGAGATCGAGG | 20 | 3557 |
| HSV1-UL30-620 | - | GACTACCTGGAGATCGAGGT | 20 | 3558 |
| HSV1-UL30-621 | - | ACTACCTGGAGATCGAGGTG | 20 | 3559 |
| HSV1-UL30-622 | - | CTACCTGGAGATCGAGGTGG | 20 | 3560 |
| HSV1-UL30-623 | - | TACCTGGAGATCGAGGTGGG | 20 | 3561 |
| HSV1-UL30-624 | - | ACCTGGAGATCGAGGTGGGG | 20 | 3562 |
| HSV1-UL30-625 | - | AGATCGAGGTGGGGGGGCGA | 20 | 3563 |
| HSV1-UL30-626 | - | GCGACGGCTGTTCTTCGTCA | 20 | 3564 |
| HSV1-UL30-627 | - | GCCTCCTCAGCATCCTCCTG | 20 | 3565 |
| HSV1-UL30-628 | - | CCTCCTCAGCATCCTCCTGC | 20 | 3566 |
| HSV1-UL30-629 | - | TCAGCATCCTCCTGCGGGAC | 20 | 3567 |
| HSV1-UL30-630 | - | TGCGAAAGCAGATCCGCTCG | 20 | 3568 |
| HSV1-UL30-631 | - | GATTCCCCAGAGCAGCCCCG | 20 | 3569 |
| HSV1-UL30-632 | - | TCCCCAGAGCAGCCCCGAGG | 20 | 3570 |
| HSV1-UL30-633 | - | CCCCGAGGAGGCCGTGCTCC | 20 | 3571 |
| HSV1-UL30-634 | - | CGTGCTCCTGGACAAGCAGC | 20 | 3572 |
| HSV1-UL30-635 | - | CAAGCAGCAGGCCGCCATCA | 20 | 3573 |
| HSV1-UL30-636 | - | CATCAAGGTCGTGTGTAACT | 20 | 3574 |
| HSV1-UL30-637 | - | GTCGTGTGTAACTCGGTGTA | 20 | 3575 |
| HSV1-UL30-638 | - | TCGTGTGTAACTCGGTGTAC | 20 | 3576 |
| HSV1-UL30-639 | - | TAACTCGGTGTACGGGTTCA | 20 | 3577 |
| HSV1-UL30-640 | - | AACTCGGTGTACGGGTTCAC | 20 | 3578 |
| HSV1-UL30-641 | - | GGGTTCACGGGAGTGCAGCA | 20 | 3579 |
| HSV1-UL30-642 | - | GTGCCTGCACGTTGCCGCGA | 20 | 3580 |
| HSV1-UL30-643 | - | GCCGCGACGGTGACGACCAT | 20 | 3581 |
| HSV1-UL30-644 | - | GCGAGTACGTCCACGCGCGC | 20 | 3582 |
| HSV1-UL30-645 | - | CGAGTACGTCCACGCGCGCT | 20 | 3583 |
| HSV1-UL30-646 | - | GTACGTCCACGCGCGCTGGG | 20 | 3584 |
| HSV1-UL30-647 | - | GGCGGCCTTCGAACAGCTCC | 20 | 3585 |
| HSV1-UL30-648 | - | ACAGCTCCTGGCCGATTTCC | 20 | 3586 |
| HSV1-UL30-649 | - | GCTCCTGGCCGATTTCCCGG | 20 | 3587 |
| HSV1-UL30-650 | - | CCTGGCCGATTTCCCGGAGG | 20 | 3588 |
| HSV1-UL30-651 | - | GCGGCCGACATGCGCGCCCC | 20 | 3589 |
| HSV1-UL30-652 | - | CGGCCGACATGCGCGCCCCC | 20 | 3590 |
| HSV1-UL30-653 | - | TATTCCATGCGCATCATCTA | 20 | 3591 |
| HSV1-UL30-654 | - | ATTCCATGCGCATCATCTAC | 20 | 3592 |
| HSV1-UL30-655 | - | TTCCATGCGCATCATCTACG | 20 | 3593 |
| HSV1-UL30-656 | - | GCGCATCATCTACGGGGACA | 20 | 3594 |
| HSV1-UL30-657 | - | TCCATCTTTGTGCTGTGCCG | 20 | 3595 |
| HSV1-UL30-658 | - | TGTGCTGTGCCGCGGCCTCA | 20 | 3596 |
| HSV1-UL30-659 | - | TGCCGCGGCCTCACGGCCGC | 20 | 3597 |
| HSV1-UL30-660 | - | GCCGCGGCCTCACGGCCGCC | 20 | 3598 |
| HSV1-UL30-661 | - | CCTCACGGCCGCCGGGCTGA | 20 | 3599 |
| HSV1-UL30-662 | - | GGCCGCCGGGCTGACGGCCG | 20 | 3600 |
| HSV1-UL30-663 | - | GCCGCCGGGCTGACGGCCGT | 20 | 3601 |
| HSV1-UL30-664 | - | GACGGCCGTGGGCGACAAGA | 20 | 3602 |
| HSV1-UL30-665 | - | ATCGCCAAGAAAAAGTACAT | 20 | 3603 |
| HSV1-UL30-666 | - | AAGTACATCGGCGTCATCTA | 20 | 3604 |
| HSV1-UL30-667 | - | AGTACATCGGCGTCATCTAC | 20 | 3605 |
| HSV1-UL30-668 | - | GTACATCGGCGTCATCTACG | 20 | 3606 |
| HSV1-UL30-669 | - | TACATCGGCGTCATCTACGG | 20 | 3607 |
| HSV1-UL30-670 | - | CGGGGGTAAGATGCTCATCA | 20 | 3608 |
| HSV1-UL30-671 | - | GGGGGTAAGATGCTCATCAA | 20 | 3609 |
| HSV1-UL30-672 | - | TAAGATGCTCATCAAGGGCG | 20 | 3610 |
| HSV1-UL30-673 | - | GCTCATCAAGGGCGTGGATC | 20 | 3611 |
| HSV1-UL30-674 | - | CGTTTATCAACCGCACCTCC | 20 | 3612 |
| HSV1-UL30-675 | - | GTTTATCAACCGCACCTCCA | 20 | 3613 |
| HSV1-UL30-676 | - | CAACCGCACCTCCAGGGCCC | 20 | 3614 |
| HSV1-UL30-677 | - | TTTTACGACGATACCGTCTC | 20 | 3615 |
| HSV1-UL30-678 | - | CGACGATACCGTCTCCGGAG | 20 | 3616 |
| HSV1-UL30-679 | - | CGCGTTAGCCGAGCGCCCCG | 20 | 3617 |
| HSV1-UL30-680 | - | GTTAGCCGAGCGCCCCGCGG | 20 | 3618 |
| HSV1-UL30-681 | - | CCGAGCGCCCCGCGGAGGAG | 20 | 3619 |
| HSV1-UL30-682 | - | GCGCCCCGCGGAGGAGTGGC | 20 | 3620 |
| HSV1-UL30-683 | - | GCTGGCGCGACCCCTGCCCG | 20 | 3621 |
| HSV1-UL30-684 | - | CTGGCGCGACCCCTGCCCGA | 20 | 3622 |
| HSV1-UL30-685 | - | ACCCCTGCCCGAGGGACTGC | 20 | 3623 |
| HSV1-UL30-686 | - | CCCGAGGGACTGCAGGCGTT | 20 | 3624 |
| HSV1-UL30-687 | - | CCGAGGGACTGCAGGCGTTC | 20 | 3625 |
| HSV1-UL30-688 | - | CGAGGGACTGCAGGCGTTCG | 20 | 3626 |
| HSV1-UL30-689 | - | CCGTCCTCGTAGACGCCCAT | 20 | 3627 |
| HSV1-UL30-690 | - | CCATCGGCGCATCACCGACC | 20 | 3628 |
| HSV1-UL30-691 | - | GGCGCATCACCGACCCGGAG | 20 | 3629 |
| HSV1-UL30-692 | - | GCGCATCACCGACCCGGAGA | 20 | 3630 |
| HSV1-UL30-693 | - | CGACCCGGAGAGGGACATCC | 20 | 3631 |
| HSV1-UL30-694 | - | CGCGTACACCAACAAGCGCC | 20 | 3632 |
| HSV1-UL30-695 | - | CAAGCGCCTGGCCCACCTGA | 20 | 3633 |
| HSV1-UL30-696 | - | GACGGTGTATTACAAGCTCA | 20 | 3634 |
| HSV1-UL30-697 | - | GCTCATGGCCCGCCGCGCGC | 20 | 3635 |
| HSV1-UL30-698 | - | CGCGCAGGTCCCGTCCATCA | 20 | 3636 |
| HSV1-UL30-699 | - | AGGTCCCGTCCATCAAGGAC | 20 | 3637 |
| HSV1-UL30-700 | - | CCGGATCCCGTACGTGATCG | 20 | 3638 |
| HSV1-UL30-701 | - | GATCGTGGCCCAGACCCGCG | 20 | 3639 |
| HSV1-UL30-702 | - | GGCCCAGACCCGCGAGGTAG | 20 | 3640 |
| HSV1-UL30-703 | - | GACCCGCGAGGTAGAGGAGA | 20 | 3641 |
| HSV1-UL30-704 | - | AGGTAGAGGAGACGGTCGCG | 20 | 3642 |
| HSV1-UL30-705 | - | AGAGGAGACGGTCGCGCGGC | 20 | 3643 |
| HSV1-UL30-706 | - | GAGCTAGACGCCGCCGCCCC | 20 | 3644 |
| HSV1-UL30-707 | - | AGCTAGACGCCGCCGCCCCA | 20 | 3645 |
| HSV1-UL30-708 | - | GCTAGACGCCGCCGCCCCAG | 20 | 3646 |
| HSV1-UL30-709 | - | GGACGAGCCCGCCCCCCCCG | 20 | 3647 |
| HSV1-UL30-710 | - | CCCCGCGGCCCTGCCCTCCC | 20 | 3648 |
| HSV1-UL30-711 | - | CCTCCCCGGCCAAGCGCCCC | 20 | 3649 |
| HSV1-UL30-712 | - | CTCCCCGGCCAAGCGCCCCC | 20 | 3650 |
| HSV1-UL30-713 | - | GCCGTCGCCTGCCGACCCCC | 20 | 3651 |
| HSV1-UL30-714 | - | CCGTCGCCTGCCGACCCCCC | 20 | 3652 |
| HSV1-UL30-715 | - | TCGCCTGCCGACCCCCCGGG | 20 | 3653 |
| HSV1-UL30-716 | - | GTCCAAGCCCCGCAAGCTGC | 20 | 3654 |
| HSV1-UL30-717 | - | CAAGCTGCTGGTGTCCGAGC | 20 | 3655 |
| HSV1-UL30-718 | - | GCTGGTGTCCGAGCTGGCCG | 20 | 3656 |
| HSV1-UL30-719 | - | CCCGCATACGCCATTGCCCA | 20 | 3657 |
| HSV1-UL30-720 | - | CCACGGCGTCGCCCTGAACA | 20 | 3658 |
| HSV1-UL30-721 | - | CTATTACTTCTCCCACCTGT | 20 | 3659 |
| HSV1-UL30-722 | - | TATTACTTCTCCCACCTGTT | 20 | 3660 |
| HSV1-UL30-723 | - | ATTACTTCTCCCACCTGTTG | 20 | 3661 |
| HSV1-UL30-724 | - | TTACTTCTCCCACCTGTTGG | 20 | 3662 |
| HSV1-UL30-725 | - | CTTCTCCCACCTGTTGGGGG | 20 | 3663 |
| HSV1-UL30-726 | - | GGCGGCGTGCGTGACATTCA | 20 | 3664 |
| HSV1-UL30-727 | - | GTGACATTCAAGGCCCTGTT | 20 | 3665 |
| HSV1-UL30-728 | - | TGACATTCAAGGCCCTGTTT | 20 | 3666 |
| HSV1-UL30-729 | - | TCACCGAGAGTCTGTTAAAA | 20 | 3667 |
| HSV1-UL30-730 | - | AAAGGTTTATTCCCGAAGTG | 20 | 3668 |
| HSV1-UL30-731 | - | TCCCGAAGTGTGGCACCCCC | 20 | 3669 |
| HSV1-UL30-732 | - | GTGGCACCCCCCGGACGACG | 20 | 3670 |
| HSV1-UL30-733 | - | CCCCGGACGACGTGGCCGCG | 20 | 3671 |
| HSV1-UL30-734 | - | ACGACGTGGCCGCGCGGCTC | 20 | 3672 |
| HSV1-UL30-735 | - | GCCGCGCGGCTCCGGACCGC | 20 | 3673 |
| HSV1-UL30-736 | - | CCGCGCGGCTCCGGACCGCA | 20 | 3674 |
| HSV1-UL30-737 | - | CGGCTCCGGACCGCAGGGTT | 20 | 3675 |
| HSV1-UL30-738 | - | GGCTCCGGACCGCAGGGTTC | 20 | 3676 |
| HSV1-UL30-739 | - | GCTCCGGACCGCAGGGTTCG | 20 | 3677 |
| HSV1-UL30-740 | - | CCGGACCGCAGGGTTCGGGG | 20 | 3678 |
| HSV1-UL30-741 | - | GACCGCAGGGTTCGGGGCGG | 20 | 3679 |
| HSV1-UL30-742 | - | ACCGCAGGGTTCGGGGCGGT | 20 | 3680 |
| HSV1-UL30-743 | - | GGGTTCGGGGCGGTGGGTGC | 20 | 3681 |
| HSV1-UL30-744 | - | GGCGGTGGGTGCCGGCGCTA | 20 | 3682 |
| HSV1-UL30-745 | - | GGTGGGTGCCGGCGCTACGG | 20 | 3683 |
| HSV1-UL30-746 | - | GGGTGCCGGCGCTACGGCGG | 20 | 3684 |
| HSV1-UL30-747 | - | GGACGCCATCACACCGA | 17 | 3685 |
| HSV1-UL30-748 | - | GACGCCATCACACCGAC | 17 | 3686 |
| HSV1-UL30-749 | - | ACGCCATCACACCGACG | 17 | 3687 |
| HSV1-UL30-750 | - | GACCGTCATCACGCTCC | 17 | 3688 |
| HSV1-UL30-751 | - | ACCGTCATCACGCTCCT | 17 | 3689 |
| HSV1-UL30-752 | - | GCTCCTGGGCCTGACTC | 17 | 3690 |
| HSV1-UL30-753 | - | CTGGGCCTGACTCCGGA | 17 | 3691 |
| HSV1-UL30-754 | - | TGACTCCGGAAGGCCAC | 17 | 3692 |
| HSV1-UL30-755 | - | GACTCCGGAAGGCCACC | 17 | 3693 |
| HSV1-UL30-756 | - | TCCGGAAGGCCACCGGG | 17 | 3694 |
| HSV1-UL30-757 | - | GTGGCCGTTCACGTTTA | 17 | 3695 |
| HSV1-UL30-758 | - | TTCACGTTTACGGCACG | 17 | 3696 |
| HSV1-UL30-759 | - | GTACTTTTACATGAACA | 17 | 3697 |
| HSV1-UL30-760 | - | CTTTTACATGAACAAGG | 17 | 3698 |
| HSV1-UL30-761 | - | TTACATGAACAAGGAGG | 17 | 3699 |
| HSV1-UL30-762 | - | ACAAGGAGGAGGTCGAC | 17 | 3700 |
| HSV1-UL30-763 | - | AGATCTCTGCGAGCGCA | 17 | 3701 |
| HSV1-UL30-764 | - | CTGCGAGCGCATGGCCG | 17 | 3702 |
| HSV1-UL30-765 | - | GGCCCTGCGCGAGTCCC | 17 | 3703 |
| HSV1-UL30-766 | - | GCCCTGCGCGAGTCCCC | 17 | 3704 |
| HSV1-UL30-767 | - | CCGGGCGCGTCGTTCCG | 17 | 3705 |
| HSV1-UL30-768 | - | GTTCCGCGGCATCTCCG | 17 | 3706 |
| HSV1-UL30-769 | - | CTCCGCGGACCACTTCG | 17 | 3707 |
| HSV1-UL30-770 | - | CGCGGACCACTTCGAGG | 17 | 3708 |
| HSV1-UL30-771 | - | GGACCACTTCGAGGCGG | 17 | 3709 |
| HSV1-UL30-772 | - | CCACTTCGAGGCGGAGG | 17 | 3710 |
| HSV1-UL30-773 | - | CTTCGAGGCGGAGGTGG | 17 | 3711 |
| HSV1-UL30-774 | - | CGCGTCTACGTCCGAAG | 17 | 3712 |
| HSV1-UL30-775 | - | GCGTCTACGTCCGAAGC | 17 | 3713 |
| HSV1-UL30-776 | - | GTGCGACAACTTCTGCC | 17 | 3714 |
| HSV1-UL30-777 | - | GGCCATCAAGAAGTACG | 17 | 3715 |
| HSV1-UL30-778 | - | GCCATCAAGAAGTACGA | 17 | 3716 |
| HSV1-UL30-779 | - | ATCAAGAAGTACGAGGG | 17 | 3717 |
| HSV1-UL30-780 | - | TCAAGAAGTACGAGGGT | 17 | 3718 |
| HSV1-UL30-781 | - | CAAGAAGTACGAGGGTG | 17 | 3719 |
| HSV1-UL30-782 | - | GGGTCGACGCCACCACC | 17 | 3720 |
| HSV1-UL30-783 | - | CACCACCCGGTTCATCC | 17 | 3721 |
| HSV1-UL30-784 | - | TTCATCCTGGACAACCC | 17 | 3722 |
| HSV1-UL30-785 | - | TCATCCTGGACAACCCC | 17 | 3723 |
| HSV1-UL30-786 | - | CCCGGGTTCGTCACCTT | 17 | 3724 |
| HSV1-UL30-787 | - | GGTTCGTCACCTTCGGC | 17 | 3725 |
| HSV1-UL30-788 | - | CTGGTACCGTCTCAAAC | 17 | 3726 |
| HSV1-UL30-789 | - | TGGTACCGTCTCAAACC | 17 | 3727 |
| HSV1-UL30-790 | - | ACCGTCTCAAACCGGGC | 17 | 3728 |
| HSV1-UL30-791 | - | CAACACGCTAGCCCAGC | 17 | 3729 |
| HSV1-UL30-792 | - | CACGCTAGCCCAGCCGG | 17 | 3730 |
| HSV1-UL30-793 | - | CCAGCCGGCGGCCCCGA | 17 | 3731 |
| HSV1-UL30-794 | - | GCGGCCCCGATGGCCTT | 17 | 3732 |
| HSV1-UL30-795 | - | CGGCCCCGATGGCCTTC | 17 | 3733 |
| HSV1-UL30-796 | - | CGTCGAGTTTAACTGTA | 17 | 3734 |
| HSV1-UL30-797 | - | CGAGTTTAACTGTACGG | 17 | 3735 |
| HSV1-UL30-798 | - | CTGTACGGCGGACAACC | 17 | 3736 |
| HSV1-UL30-799 | - | GGACAACCTGGCCATCG | 17 | 3737 |
| HSV1-UL30-800 | - | GACAACCTGGCCATCGA | 17 | 3738 |
| HSV1-UL30-801 | - | ACAACCTGGCCATCGAG | 17 | 3739 |
| HSV1-UL30-802 | - | CAACCTGGCCATCGAGG | 17 | 3740 |
| HSV1-UL30-803 | - | AACCTGGCCATCGAGGG | 17 | 3741 |
| HSV1-UL30-804 | - | GGGCATGAGCGACCTAC | 17 | 3742 |
| HSV1-UL30-805 | - | CTTCGATATCGAATGCA | 17 | 3743 |
| HSV1-UL30-806 | - | CGATATCGAATGCAAGG | 17 | 3744 |
| HSV1-UL30-807 | - | GATATCGAATGCAAGGC | 17 | 3745 |
| HSV1-UL30-808 | - | ATATCGAATGCAAGGCG | 17 | 3746 |
| HSV1-UL30-809 | - | TATCGAATGCAAGGCGG | 17 | 3747 |
| HSV1-UL30-810 | - | ATCGAATGCAAGGCGGG | 17 | 3748 |
| HSV1-UL30-811 | - | TCGAATGCAAGGCGGGG | 17 | 3749 |
| HSV1-UL30-812 | - | CGAATGCAAGGCGGGGG | 17 | 3750 |
| HSV1-UL30-813 | - | ATGCAAGGCGGGGGGGG | 17 | 3751 |
| HSV1-UL30-814 | - | GGGGGGGGAGGACGAGC | 17 | 3752 |
| HSV1-UL30-815 | - | GGACGAGCTGGCCTTTC | 17 | 3753 |
| HSV1-UL30-816 | - | CGAGCTGGCCTTTCCGG | 17 | 3754 |
| HSV1-UL30-817 | - | CTGGCCTTTCCGGTGGC | 17 | 3755 |
| HSV1-UL30-818 | - | TGGCCTTTCCGGTGGCC | 17 | 3756 |
| HSV1-UL30-819 | - | TCCGGTGGCCGGGCACC | 17 | 3757 |
| HSV1-UL30-820 | - | GGTGGCCGGGCACCCGG | 17 | 3758 |
| HSV1-UL30-821 | - | CGGGCACCCGGAGGACC | 17 | 3759 |
| HSV1-UL30-822 | - | CCTGTCCACCACCGCCC | 17 | 3760 |
| HSV1-UL30-823 | - | GTCCTCCTGTTTTCGCT | 17 | 3761 |
| HSV1-UL30-824 | - | ATCCCACCTGAACGAGC | 17 | 3762 |
| HSV1-UL30-825 | - | CCACCTGAACGAGCTGG | 17 | 3763 |
| HSV1-UL30-826 | - | TGAACGAGCTGGCGGCC | 17 | 3764 |
| HSV1-UL30-827 | - | GAACGAGCTGGCGGCCA | 17 | 3765 |
| HSV1-UL30-828 | - | AACGAGCTGGCGGCCAG | 17 | 3766 |
| HSV1-UL30-829 | - | GGGCCTGCCCACGCCCG | 17 | 3767 |
| HSV1-UL30-830 | - | GCCCACGCCCGTGGTTC | 17 | 3768 |
| HSV1-UL30-831 | - | CGAATTCGAGATGCTGT | 17 | 3769 |
| HSV1-UL30-832 | - | ACCCTTGTGAAACAGTA | 17 | 3770 |
| HSV1-UL30-833 | - | GGCCCCGAGTTCGTGAC | 17 | 3771 |
| HSV1-UL30-834 | - | GCCCCGAGTTCGTGACC | 17 | 3772 |
| HSV1-UL30-835 | - | ACATCATCAACTTCGAC | 17 | 3773 |
| HSV1-UL30-836 | - | CGACTGGCCCTTCTTGC | 17 | 3774 |
| HSV1-UL30-837 | - | CTTGCTGGCCAAGCTGA | 17 | 3775 |
| HSV1-UL30-838 | - | GCTGACGGACATTTACA | 17 | 3776 |
| HSV1-UL30-839 | - | CATTTACAAGGTCCCCC | 17 | 3777 |
| HSV1-UL30-840 | - | TACAAGGTCCCCCTGGA | 17 | 3778 |
| HSV1-UL30-841 | - | ACAAGGTCCCCCTGGAC | 17 | 3779 |
| HSV1-UL30-842 | - | GTCCCCCTGGACGGGTA | 17 | 3780 |
| HSV1-UL30-843 | - | GGGTACGGCCGCATGAA | 17 | 3781 |
| HSV1-UL30-844 | - | ACGGCCGCATGAACGGC | 17 | 3782 |
| HSV1-UL30-845 | - | CGGCCGCATGAACGGCC | 17 | 3783 |
| HSV1-UL30-846 | - | GGCCGCATGAACGGCCG | 17 | 3784 |
| HSV1-UL30-847 | - | GGGGCGTGTTTCGCGTG | 17 | 3785 |
| HSV1-UL30-848 | - | GGGCGTGTTTCGCGTGT | 17 | 3786 |
| HSV1-UL30-849 | - | TTTCGCGTGTGGGACAT | 17 | 3787 |
| HSV1-UL30-850 | - | GAAGCGCAGCAAGATAA | 17 | 3788 |
| HSV1-UL30-851 | - | AGCAAGATAAAGGTGAA | 17 | 3789 |
| HSV1-UL30-852 | - | GATAAAGGTGAACGGCA | 17 | 3790 |
| HSV1-UL30-853 | - | GTGAACATCGACATGTA | 17 | 3791 |
| HSV1-UL30-854 | - | TGAACATCGACATGTAC | 17 | 3792 |
| HSV1-UL30-855 | - | CTACAAGCTCAACGCCG | 17 | 3793 |
| HSV1-UL30-856 | - | GGCCGAAGCCGTCCTGA | 17 | 3794 |
| HSV1-UL30-857 | - | CCTGAAGGACAAGAAGA | 17 | 3795 |
| HSV1-UL30-858 | - | CCCGCCTACTACGCCGC | 17 | 3796 |
| HSV1-UL30-859 | - | CCGCCTACTACGCCGCC | 17 | 3797 |
| HSV1-UL30-860 | - | GCCGGGCCCGCGCAACG | 17 | 3798 |
| HSV1-UL30-861 | - | CCGGGCCCGCGCAACGC | 17 | 3799 |
| HSV1-UL30-862 | - | CGGGCCCGCGCAACGCG | 17 | 3800 |
| HSV1-UL30-863 | - | GCGCAACGCGGGGTGAT | 17 | 3801 |
| HSV1-UL30-864 | - | CGGCGAGTACTGCATAC | 17 | 3802 |
| HSV1-UL30-865 | - | CATACAGGATTCCCTGC | 17 | 3803 |
| HSV1-UL30-866 | - | ACAGGATTCCCTGCTGG | 17 | 3804 |
| HSV1-UL30-867 | - | CAGGATTCCCTGCTGGT | 17 | 3805 |
| HSV1-UL30-868 | - | TAAGTTTTTGCCCCATC | 17 | 3806 |
| HSV1-UL30-869 | - | GCCCCATCTGGAGCTCT | 17 | 3807 |
| HSV1-UL30-870 | - | CTCGGCCGTCGCGCGCT | 17 | 3808 |
| HSV1-UL30-871 | - | GGCCGTCGCGCGCTTGG | 17 | 3809 |
| HSV1-UL30-872 | - | GCCGTCGCGCGCTTGGC | 17 | 3810 |
| HSV1-UL30-873 | - | ACCCGCACCATCTACGA | 17 | 3811 |
| HSV1-UL30-874 | - | TACGTGCCTGCTGCGCC | 17 | 3812 |
| HSV1-UL30-875 | - | GCGCCTGGCCGACCAGA | 17 | 3813 |
| HSV1-UL30-876 | - | CGCCTGGCCGACCAGAA | 17 | 3814 |
| HSV1-UL30-877 | - | GAAGGGCTTTATTCTGC | 17 | 3815 |
| HSV1-UL30-878 | - | TATTCTGCCGGACACCC | 17 | 3816 |
| HSV1-UL30-879 | - | ATTCTGCCGGACACCCA | 17 | 3817 |
| HSV1-UL30-880 | - | TTCTGCCGGACACCCAG | 17 | 3818 |
| HSV1-UL30-881 | - | ACACCCAGGGGCGATTT | 17 | 3819 |
| HSV1-UL30-882 | - | CACCCAGGGGCGATTTA | 17 | 3820 |
| HSV1-UL30-883 | - | ACCCAGGGGCGATTTAG | 17 | 3821 |
| HSV1-UL30-884 | - | GGGCGATTTAGGGGCGC | 17 | 3822 |
| HSV1-UL30-885 | - | GGCGATTTAGGGGCGCC | 17 | 3823 |
| HSV1-UL30-886 | - | GCGATTTAGGGGCGCCG | 17 | 3824 |
| HSV1-UL30-887 | - | CGATTTAGGGGCGCCGG | 17 | 3825 |
| HSV1-UL30-888 | - | GATTTAGGGGCGCCGGG | 17 | 3826 |
| HSV1-UL30-889 | - | ATTTAGGGGCGCCGGGG | 17 | 3827 |
| HSV1-UL30-890 | - | TAGGGGCGCCGGGGGGG | 17 | 3828 |
| HSV1-UL30-891 | - | GGAGGCGCCCAAGCGTC | 17 | 3829 |
| HSV1-UL30-892 | - | AGCGTCCGGCCGCAGCC | 17 | 3830 |
| HSV1-UL30-893 | - | GCGTCCGGCCGCAGCCC | 17 | 3831 |
| HSV1-UL30-894 | - | TCCGGCCGCAGCCCGGG | 17 | 3832 |
| HSV1-UL30-895 | - | CGCAGCCCGGGAGGACG | 17 | 3833 |
| HSV1-UL30-896 | - | CCCGGGAGGACGAGGAG | 17 | 3834 |
| HSV1-UL30-897 | - | GGACGAGGAGCGGCCAG | 17 | 3835 |
| HSV1-UL30-898 | - | CGAGGAGCGGCCAGAGG | 17 | 3836 |
| HSV1-UL30-899 | - | GGAGCGGCCAGAGGAGG | 17 | 3837 |
| HSV1-UL30-900 | - | GAGCGGCCAGAGGAGGA | 17 | 3838 |
| HSV1-UL30-901 | - | AGCGGCCAGAGGAGGAG | 17 | 3839 |
| HSV1-UL30-902 | - | GCGGCCAGAGGAGGAGG | 17 | 3840 |
| HSV1-UL30-903 | - | GCCAGAGGAGGAGGGGG | 17 | 3841 |
| HSV1-UL30-904 | - | GGAGGAGGGGGAGGACG | 17 | 3842 |
| HSV1-UL30-905 | - | GGACGAGGACGAACGCG | 17 | 3843 |
| HSV1-UL30-906 | - | CGAGGACGAACGCGAGG | 17 | 3844 |
| HSV1-UL30-907 | - | GAGGACGAACGCGAGGA | 17 | 3845 |
| HSV1-UL30-908 | - | GACGAACGCGAGGAGGG | 17 | 3846 |
| HSV1-UL30-909 | - | ACGAACGCGAGGAGGGC | 17 | 3847 |
| HSV1-UL30-910 | - | CGAACGCGAGGAGGGCG | 17 | 3848 |
| HSV1-UL30-911 | - | GAACGCGAGGAGGGCGG | 17 | 3849 |
| HSV1-UL30-912 | - | AGGAGGGCGGGGGCGAG | 17 | 3850 |
| HSV1-UL30-913 | - | GGAGGGCGGGGGCGAGC | 17 | 3851 |
| HSV1-UL30-914 | - | CGGGGGCGAGCGGGAGC | 17 | 3852 |
| HSV1-UL30-915 | - | GGGCGAGCGGGAGCCGG | 17 | 3853 |
| HSV1-UL30-916 | - | GGCGAGCGGGAGCCGGA | 17 | 3854 |
| HSV1-UL30-917 | - | GGGAGCCGGAGGGCGCG | 17 | 3855 |
| HSV1-UL30-918 | - | GGAGCCGGAGGGCGCGC | 17 | 3856 |
| HSV1-UL30-919 | - | GGCGCGCGGGAGACCGC | 17 | 3857 |
| HSV1-UL30-920 | - | CGCGGGAGACCGCCGGC | 17 | 3858 |
| HSV1-UL30-921 | - | GACCGCCGGCAGGCACG | 17 | 3859 |
| HSV1-UL30-922 | - | ACCGCCGGCAGGCACGT | 17 | 3860 |
| HSV1-UL30-923 | - | CCGCCGGCAGGCACGTG | 17 | 3861 |
| HSV1-UL30-924 | - | CAGGCACGTGGGGTACC | 17 | 3862 |
| HSV1-UL30-925 | - | AGGCACGTGGGGTACCA | 17 | 3863 |
| HSV1-UL30-926 | - | GGCACGTGGGGTACCAG | 17 | 3864 |
| HSV1-UL30-927 | - | GCACGTGGGGTACCAGG | 17 | 3865 |
| HSV1-UL30-928 | - | TGGGGTACCAGGGGGCC | 17 | 3866 |
| HSV1-UL30-929 | - | GGGGTACCAGGGGGCCA | 17 | 3867 |
| HSV1-UL30-930 | - | GTCCTTGACCCCACTTC | 17 | 3868 |
| HSV1-UL30-931 | - | TCCTTGACCCCACTTCC | 17 | 3869 |
| HSV1-UL30-932 | - | GTTTCACGTGAACCCCG | 17 | 3870 |
| HSV1-UL30-933 | - | TCACGTGAACCCCGTGG | 17 | 3871 |
| HSV1-UL30-934 | - | GTACCCCAGCATCATCC | 17 | 3872 |
| HSV1-UL30-935 | - | TCAGCACGCTCTCCCTG | 17 | 3873 |
| HSV1-UL30-936 | - | CAGCACGCTCTCCCTGA | 17 | 3874 |
| HSV1-UL30-937 | - | CCTGAGGGCCGACGCAG | 17 | 3875 |
| HSV1-UL30-938 | - | CGACGCAGTGGCGCACC | 17 | 3876 |
| HSV1-UL30-939 | - | CGCAGTGGCGCACCTGG | 17 | 3877 |
| HSV1-UL30-940 | - | AGTGGCGCACCTGGAGG | 17 | 3878 |
| HSV1-UL30-941 | - | GTGGCGCACCTGGAGGC | 17 | 3879 |
| HSV1-UL30-942 | - | GCACCTGGAGGCGGGCA | 17 | 3880 |
| HSV1-UL30-943 | - | GGCGGGCAAGGACTACC | 17 | 3881 |
| HSV1-UL30-944 | - | GGACTACCTGGAGATCG | 17 | 3882 |
| HSV1-UL30-945 | - | CTACCTGGAGATCGAGG | 17 | 3883 |
| HSV1-UL30-946 | - | TACCTGGAGATCGAGGT | 17 | 3884 |
| HSV1-UL30-947 | - | ACCTGGAGATCGAGGTG | 17 | 3885 |
| HSV1-UL30-948 | - | CCTGGAGATCGAGGTGG | 17 | 3886 |
| HSV1-UL30-949 | - | CTGGAGATCGAGGTGGG | 17 | 3887 |
| HSV1-UL30-950 | - | TGGAGATCGAGGTGGGG | 17 | 3888 |
| HSV1-UL30-951 | - | TCGAGGTGGGGGGGCGA | 17 | 3889 |
| HSV1-UL30-952 | - | ACGGCTGTTCTTCGTCA | 17 | 3890 |
| HSV1-UL30-953 | - | TCCTCAGCATCCTCCTG | 17 | 3891 |
| HSV1-UL30-954 | - | CCTCAGCATCCTCCTGC | 17 | 3892 |
| HSV1-UL30-955 | - | GCATCCTCCTGCGGGAC | 17 | 3893 |
| HSV1-UL30-956 | - | GAAAGCAGATCCGCTCG | 17 | 3894 |
| HSV1-UL30-957 | - | TCCCCAGAGCAGCCCCG | 17 | 3895 |
| HSV1-UL30-958 | - | CCAGAGCAGCCCCGAGG | 17 | 3896 |
| HSV1-UL30-959 | - | CGAGGAGGCCGTGCTCC | 17 | 3897 |
| HSV1-UL30-960 | - | GCTCCTGGACAAGCAGC | 17 | 3898 |
| HSV1-UL30-961 | - | GCAGCAGGCCGCCATCA | 17 | 3899 |
| HSV1-UL30-962 | - | CAAGGTCGTGTGTAACT | 17 | 3900 |
| HSV1-UL30-963 | - | GTGTGTAACTCGGTGTA | 17 | 3901 |
| HSV1-UL30-964 | - | TGTGTAACTCGGTGTAC | 17 | 3902 |
| HSV1-UL30-965 | - | CTCGGTGTACGGGTTCA | 17 | 3903 |
| HSV1-UL30-966 | - | TCGGTGTACGGGTTCAC | 17 | 3904 |
| HSV1-UL30-967 | - | TTCACGGGAGTGCAGCA | 17 | 3905 |
| HSV1-UL30-968 | - | CCTGCACGTTGCCGCGA | 17 | 3906 |
| HSV1-UL30-969 | - | GCGACGGTGACGACCAT | 17 | 3907 |
| HSV1-UL30-970 | - | AGTACGTCCACGCGCGC | 17 | 3908 |
| HSV1-UL30-971 | - | GTACGTCCACGCGCGCT | 17 | 3909 |
| HSV1-UL30-972 | - | CGTCCACGCGCGCTGGG | 17 | 3910 |
| HSV1-UL30-973 | - | GGCCTTCGAACAGCTCC | 17 | 3911 |
| HSV1-UL30-974 | - | GCTCCTGGCCGATTTCC | 17 | 3912 |
| HSV1-UL30-975 | - | CCTGGCCGATTTCCCGG | 17 | 3913 |
| HSV1-UL30-976 | - | GGCCGATTTCCCGGAGG | 17 | 3914 |
| HSV1-UL30-977 | - | GCCGACATGCGCGCCCC | 17 | 3915 |
| HSV1-UL30-978 | - | CCGACATGCGCGCCCCC | 17 | 3916 |
| HSV1-UL30-979 | - | TCCATGCGCATCATCTA | 17 | 3917 |
| HSV1-UL30-980 | - | CCATGCGCATCATCTAC | 17 | 3918 |
| HSV1-UL30-981 | - | CATGCGCATCATCTACG | 17 | 3919 |
| HSV1-UL30-982 | - | CATCATCTACGGGGACA | 17 | 3920 |
| HSV1-UL30-983 | - | ATCTTTGTGCTGTGCCG | 17 | 3921 |
| HSV1-UL30-984 | - | GCTGTGCCGCGGCCTCA | 17 | 3922 |
| HSV1-UL30-985 | - | CGCGGCCTCACGGCCGC | 17 | 3923 |
| HSV1-UL30-986 | - | GCGGCCTCACGGCCGCC | 17 | 3924 |
| HSV1-UL30-987 | - | CACGGCCGCCGGGCTGA | 17 | 3925 |
| HSV1-UL30-988 | - | CGCCGGGCTGACGGCCG | 17 | 3926 |
| HSV1-UL30-989 | - | GCCGGGCTGACGGCCGT | 17 | 3927 |
| HSV1-UL30-990 | - | GGCCGTGGGCGACAAGA | 17 | 3928 |
| HSV1-UL30-991 | - | GCCAAGAAAAAGTACAT | 17 | 3929 |
| HSV1-UL30-992 | - | TACATCGGCGTCATCTA | 17 | 3930 |
| HSV1-UL30-993 | - | ACATCGGCGTCATCTAC | 17 | 3931 |
| HSV1-UL30-994 | - | CATCGGCGTCATCTACG | 17 | 3932 |
| HSV1-UL30-995 | - | ATCGGCGTCATCTACGG | 17 | 3933 |
| HSV1-UL30-996 | - | GGGTAAGATGCTCATCA | 17 | 3934 |
| HSV1-UL30-997 | - | GGTAAGATGCTCATCAA | 17 | 3935 |
| HSV1-UL30-998 | - | GATGCTCATCAAGGGCG | 17 | 3936 |
| HSV1-UL30-999 | - | CATCAAGGGCGTGGATC | 17 | 3937 |
| HSV1-UL30-1000 | - | TTATCAACCGCACCTCC | 17 | 3938 |
| HSV1-UL30-1001 | - | TATCAACCGCACCTCCA | 17 | 3939 |
| HSV1-UL30-1002 | - | CCGCACCTCCAGGGCCC | 17 | 3940 |
| HSV1-UL30-1003 | - | TACGACGATACCGTCTC | 17 | 3941 |
| HSV1-UL30-1004 | - | CGATACCGTCTCCGGAG | 17 | 3942 |
| HSV1-UL30-1005 | - | GTTAGCCGAGCGCCCCG | 17 | 3943 |
| HSV1-UL30-1006 | - | AGCCGAGCGCCCCGCGG | 17 | 3944 |
| HSV1-UL30-1007 | - | AGCGCCCCGCGGAGGAG | 17 | 3945 |
| HSV1-UL30-1008 | - | CCCCGCGGAGGAGTGGC | 17 | 3946 |
| HSV1-UL30-1009 | - | GGCGCGACCCCTGCCCG | 17 | 3947 |
| HSV1-UL30-1010 | - | GCGCGACCCCTGCCCGA | 17 | 3948 |
| HSV1-UL30-1011 | - | CCTGCCCGAGGGACTGC | 17 | 3949 |
| HSV1-UL30-1012 | - | GAGGGACTGCAGGCGTT | 17 | 3950 |
| HSV1-UL30-1013 | - | AGGGACTGCAGGCGTTC | 17 | 3951 |
| HSV1-UL30-1014 | - | GGGACTGCAGGCGTTCG | 17 | 3952 |
| HSV1-UL30-1015 | - | TCCTCGTAGACGCCCAT | 17 | 3953 |
| HSV1-UL30-1016 | - | TCGGCGCATCACCGACC | 17 | 3954 |
| HSV1-UL30-1017 | - | GCATCACCGACCCGGAG | 17 | 3955 |
| HSV1-UL30-1018 | - | CATCACCGACCCGGAGA | 17 | 3956 |
| HSV1-UL30-1019 | - | CCCGGAGAGGGACATCC | 17 | 3957 |
| HSV1-UL30-1020 | - | GTACACCAACAAGCGCC | 17 | 3958 |
| HSV1-UL30-1021 | - | GCGCCTGGCCCACCTGA | 17 | 3959 |
| HSV1-UL30-1022 | - | GGTGTATTACAAGCTCA | 17 | 3960 |
| HSV1-UL30-1023 | - | CATGGCCCGCCGCGCGC | 17 | 3961 |
| HSV1-UL30-1024 | - | GCAGGTCCCGTCCATCA | 17 | 3962 |
| HSV1-UL30-1025 | - | TCCCGTCCATCAAGGAC | 17 | 3963 |
| HSV1-UL30-1026 | - | GATCCCGTACGTGATCG | 17 | 3964 |
| HSV1-UL30-1027 | - | CGTGGCCCAGACCCGCG | 17 | 3965 |
| HSV1-UL30-1028 | - | CCAGACCCGCGAGGTAG | 17 | 3966 |
| HSV1-UL30-1029 | - | CCGCGAGGTAGAGGAGA | 17 | 3967 |
| HSV1-UL30-1030 | - | TAGAGGAGACGGTCGCG | 17 | 3968 |
| HSV1-UL30-1031 | - | GGAGACGGTCGCGCGGC | 17 | 3969 |
| HSV1-UL30-1032 | - | CTAGACGCCGCCGCCCC | 17 | 3970 |
| HSV1-UL30-1033 | - | TAGACGCCGCCGCCCCA | 17 | 3971 |
| HSV1-UL30-1034 | - | AGACGCCGCCGCCCCAG | 17 | 3972 |
| HSV1-UL30-1035 | - | CGAGCCCGCCCCCCCCG | 17 | 3973 |
| HSV1-UL30-1036 | - | CGCGGCCCTGCCCTCCC | 17 | 3974 |
| HSV1-UL30-1037 | - | CCCCGGCCAAGCGCCCC | 17 | 3975 |
| HSV1-UL30-1038 | - | CCCGGCCAAGCGCCCCC | 17 | 3976 |
| HSV1-UL30-1039 | - | GTCGCCTGCCGACCCCC | 17 | 3977 |
| HSV1-UL30-1040 | - | TCGCCTGCCGACCCCCC | 17 | 3978 |
| HSV1-UL30-1041 | - | CCTGCCGACCCCCCGGG | 17 | 3979 |
| HSV1-UL30-1042 | - | CAAGCCCCGCAAGCTGC | 17 | 3980 |
| HSV1-UL30-1043 | - | GCTGCTGGTGTCCGAGC | 17 | 3981 |
| HSV1-UL30-1044 | - | GGTGTCCGAGCTGGCCG | 17 | 3982 |
| HSV1-UL30-1045 | - | GCATACGCCATTGCCCA | 17 | 3983 |
| HSV1-UL30-1046 | - | CGGCGTCGCCCTGAACA | 17 | 3984 |
| HSV1-UL30-1047 | - | TTACTTCTCCCACCTGT | 17 | 3985 |
| HSV1-UL30-1048 | - | TACTTCTCCCACCTGTT | 17 | 3986 |
| HSV1-UL30-1049 | - | ACTTCTCCCACCTGTTG | 17 | 3987 |
| HSV1-UL30-1050 | - | CTTCTCCCACCTGTTGG | 17 | 3988 |
| HSV1-UL30-1051 | - | CTCCCACCTGTTGGGGG | 17 | 3989 |
| HSV1-UL30-1052 | - | GGCGTGCGTGACATTCA | 17 | 3990 |
| HSV1-UL30-1053 | - | ACATTCAAGGCCCTGTT | 17 | 3991 |
| HSV1-UL30-1054 | - | CATTCAAGGCCCTGTTT | 17 | 3992 |
| HSV1-UL30-1055 | - | CCGAGAGTCTGTTAAAA | 17 | 3993 |
| HSV1-UL30-1056 | - | GGTTTATTCCCGAAGTG | 17 | 3994 |
| HSV1-UL30-1057 | - | CGAAGTGTGGCACCCCC | 17 | 3995 |
| HSV1-UL30-1058 | - | GCACCCCCCGGACGACG | 17 | 3996 |
| HSV1-UL30-1059 | - | CGGACGACGTGGCCGCG | 17 | 3997 |
| HSV1-UL30-1060 | - | ACGTGGCCGCGCGGCTC | 17 | 3998 |
| HSV1-UL30-1061 | - | GCGCGGCTCCGGACCGC | 17 | 3999 |
| HSV1-UL30-1062 | - | CGCGGCTCCGGACCGCA | 17 | 4000 |
| HSV1-UL30-1063 | - | CTCCGGACCGCAGGGTT | 17 | 4001 |
| HSV1-UL30-1064 | - | TCCGGACCGCAGGGTTC | 17 | 4002 |
| HSV1-UL30-1065 | - | CCGGACCGCAGGGTTCG | 17 | 4003 |
| HSV1-UL30-1066 | - | GACCGCAGGGTTCGGGG | 17 | 4004 |
| HSV1-UL30-1067 | - | CGCAGGGTTCGGGGCGG | 17 | 4005 |
| HSV1-UL30-1068 | - | GCAGGGTTCGGGGCGGT | 17 | 4006 |
| HSV1-UL30-1069 | - | TTCGGGGCGGTGGGTGC | 17 | 4007 |
| HSV1-UL30-1070 | - | GGTGGGTGCCGGCGCTA | 17 | 4008 |
| HSV1-UL30-1071 | - | GGGTGCCGGCGCTACGG | 17 | 4009 |
| HSV1-UL30-1072 | - | TGCCGGCGCTACGGCGG | 17 | 4010 |
| HSV1-UL30-1073 | + | AGTTTCCTCCGCCGTAGCGC | 20 | 4011 |
| HSV1-UL30-1074 | + | ACCCACCGCCCCGAACCCTG | 20 | 4012 |
| HSV1-UL30-1075 | + | CCGCCCCGAACCCTGCGGTC | 20 | 4013 |
| HSV1-UL30-1076 | + | CCCTGCGGTCCGGAGCCGCG | 20 | 4014 |
| HSV1-UL30-1077 | + | AGCCGCGCGGCCACGTCGTC | 20 | 4015 |
| HSV1-UL30-1078 | + | GCCGCGCGGCCACGTCGTCC | 20 | 4016 |
| HSV1-UL30-1079 | + | CCGCGCGGCCACGTCGTCCG | 20 | 4017 |
| HSV1-UL30-1080 | + | CGCGCGGCCACGTCGTCCGG | 20 | 4018 |
| HSV1-UL30-1081 | + | GCGCGGCCACGTCGTCCGGG | 20 | 4019 |
| HSV1-UL30-1082 | + | GTCCGGGGGGTGCCACACTT | 20 | 4020 |
| HSV1-UL30-1083 | + | TCCGGGGGGTGCCACACTTC | 20 | 4021 |
| HSV1-UL30-1084 | + | AAACCTTTTTAACAGACTCT | 20 | 4022 |
| HSV1-UL30-1085 | + | TAACAGACTCTCGGTGATCT | 20 | 4023 |
| HSV1-UL30-1086 | + | TCTTGGCGTTATTCCCAAAC | 20 | 4024 |
| HSV1-UL30-1087 | + | CTTGGCGTTATTCCCAAACA | 20 | 4025 |
| HSV1-UL30-1088 | + | TCACGCACGCCGCCCCCAAC | 20 | 4026 |
| HSV1-UL30-1089 | + | CGCACGCCGCCCCCAACAGG | 20 | 4027 |
| HSV1-UL30-1090 | + | GCACGCCGCCCCCAACAGGT | 20 | 4028 |
| HSV1-UL30-1091 | + | AGAAGTAATAGTCCGTGTTC | 20 | 4029 |
| HSV1-UL30-1092 | + | GAAGTAATAGTCCGTGTTCA | 20 | 4030 |
| HSV1-UL30-1093 | + | CCGTGTTCAGGGCGACGCCG | 20 | 4031 |
| HSV1-UL30-1094 | + | CGTGTTCAGGGCGACGCCGT | 20 | 4032 |
| HSV1-UL30-1095 | + | CAGGGCGACGCCGTGGGCAA | 20 | 4033 |
| HSV1-UL30-1096 | + | GCCGTGGGCAATGGCGTATG | 20 | 4034 |
| HSV1-UL30-1097 | + | CCGTGGGCAATGGCGTATGC | 20 | 4035 |
| HSV1-UL30-1098 | + | AATGGCGTATGCGGGATCCT | 20 | 4036 |
| HSV1-UL30-1099 | + | TGCGGGATCCTCGGCCAGCT | 20 | 4037 |
| HSV1-UL30-1100 | + | GCTCGGACACCAGCAGCTTG | 20 | 4038 |
| HSV1-UL30-1101 | + | CTCGGACACCAGCAGCTTGC | 20 | 4039 |
| HSV1-UL30-1102 | + | TCGGACACCAGCAGCTTGCG | 20 | 4040 |
| HSV1-UL30-1103 | + | CACCAGCAGCTTGCGGGGCT | 20 | 4041 |
| HSV1-UL30-1104 | + | CGGGGCTTGGACGCGCCTCC | 20 | 4042 |
| HSV1-UL30-1105 | + | GGGGCTTGGACGCGCCTCCC | 20 | 4043 |
| HSV1-UL30-1106 | + | GGGCTTGGACGCGCCTCCCG | 20 | 4044 |
| HSV1-UL30-1107 | + | GGCTTGGACGCGCCTCCCGG | 20 | 4045 |
| HSV1-UL30-1108 | + | GCTTGGACGCGCCTCCCGGG | 20 | 4046 |
| HSV1-UL30-1109 | + | GGACGCGCCTCCCGGGGGGT | 20 | 4047 |
| HSV1-UL30-1110 | + | GCGCCTCCCGGGGGGTCGGC | 20 | 4048 |
| HSV1-UL30-1111 | + | CCCGGGGGGTCGGCAGGCGA | 20 | 4049 |
| HSV1-UL30-1112 | + | CGGCAGGCGACGGCGTCTCC | 20 | 4050 |
| HSV1-UL30-1113 | + | GGCAGGCGACGGCGTCTCCC | 20 | 4051 |
| HSV1-UL30-1114 | + | GCAGGCGACGGCGTCTCCCG | 20 | 4052 |
| HSV1-UL30-1115 | + | CAGGCGACGGCGTCTCCCGG | 20 | 4053 |
| HSV1-UL30-1116 | + | CGGCGTCTCCCGGGGGCGCT | 20 | 4054 |
| HSV1-UL30-1117 | + | GTCTCCCGGGGGCGCTTGGC | 20 | 4055 |
| HSV1-UL30-1118 | + | TCTCCCGGGGGCGCTTGGCC | 20 | 4056 |
| HSV1-UL30-1119 | + | CTCCCGGGGGCGCTTGGCCG | 20 | 4057 |
| HSV1-UL30-1120 | + | CCGGGGGCGCTTGGCCGGGG | 20 | 4058 |
| HSV1-UL30-1121 | + | CGGGGGCGCTTGGCCGGGGA | 20 | 4059 |
| HSV1-UL30-1122 | + | GGCGCTTGGCCGGGGAGGGC | 20 | 4060 |
| HSV1-UL30-1123 | + | GCGCTTGGCCGGGGAGGGCA | 20 | 4061 |
| HSV1-UL30-1124 | + | GGCCGGGGAGGGCAGGGCCG | 20 | 4062 |
| HSV1-UL30-1125 | + | GCCGGGGAGGGCAGGGCCGC | 20 | 4063 |
| HSV1-UL30-1126 | + | CCGGGGAGGGCAGGGCCGCG | 20 | 4064 |
| HSV1-UL30-1127 | + | CGGGGAGGGCAGGGCCGCGG | 20 | 4065 |
| HSV1-UL30-1128 | + | GGGGAGGGCAGGGCCGCGGG | 20 | 4066 |
| HSV1-UL30-1129 | + | GGGAGGGCAGGGCCGCGGGG | 20 | 4067 |
| HSV1-UL30-1130 | + | GGAGGGCAGGGCCGCGGGGG | 20 | 4068 |
| HSV1-UL30-1131 | + | GGGCAGGGCCGCGGGGGGGG | 20 | 4069 |
| HSV1-UL30-1132 | + | GGCAGGGCCGCGGGGGGGGC | 20 | 4070 |
| HSV1-UL30-1133 | + | GGGGGGGCGGGCTCGTCCCC | 20 | 4071 |
| HSV1-UL30-1134 | + | GGGGGGCGGGCTCGTCCCCT | 20 | 4072 |
| HSV1-UL30-1135 | + | GGGGGCGGGCTCGTCCCCTG | 20 | 4073 |
| HSV1-UL30-1136 | + | GGCGGGCTCGTCCCCTGGGG | 20 | 4074 |
| HSV1-UL30-1137 | + | GGGCTCGTCCCCTGGGGCGG | 20 | 4075 |
| HSV1-UL30-1138 | + | GGGCGGCGGCGTCTAGCTCG | 20 | 4076 |
| HSV1-UL30-1139 | + | CGGCGGCGTCTAGCTCGCGG | 20 | 4077 |
| HSV1-UL30-1140 | + | GGCGGCGTCTAGCTCGCGGA | 20 | 4078 |
| HSV1-UL30-1141 | + | GGCGTCTAGCTCGCGGAGGG | 20 | 4079 |
| HSV1-UL30-1142 | + | CGACCGTCTCCTCTACCTCG | 20 | 4080 |
| HSV1-UL30-1143 | + | GACCGTCTCCTCTACCTCGC | 20 | 4081 |
| HSV1-UL30-1144 | + | TCTCCTCTACCTCGCGGGTC | 20 | 4082 |
| HSV1-UL30-1145 | + | CTCCTCTACCTCGCGGGTCT | 20 | 4083 |
| HSV1-UL30-1146 | + | GTCTGGGCCACGATCACGTA | 20 | 4084 |
| HSV1-UL30-1147 | + | TCTGGGCCACGATCACGTAC | 20 | 4085 |
| HSV1-UL30-1148 | + | CCACGATCACGTACGGGATC | 20 | 4086 |
| HSV1-UL30-1149 | + | GTACGGGATCCGGTCCTTGA | 20 | 4087 |
| HSV1-UL30-1150 | + | GGGATCCGGTCCTTGATGGA | 20 | 4088 |
| HSV1-UL30-1151 | + | GGATCCGGTCCTTGATGGAC | 20 | 4089 |
| HSV1-UL30-1152 | + | TGATGGACGGGACCTGCGCG | 20 | 4090 |
| HSV1-UL30-1153 | + | TGGACGGGACCTGCGCGCGG | 20 | 4091 |
| HSV1-UL30-1154 | + | GGACGGGACCTGCGCGCGGC | 20 | 4092 |
| HSV1-UL30-1155 | + | TGAGCTTGTAATACACCGTC | 20 | 4093 |
| HSV1-UL30-1156 | + | GCTTGTAATACACCGTCAGG | 20 | 4094 |
| HSV1-UL30-1157 | + | CTTGTAATACACCGTCAGGT | 20 | 4095 |
| HSV1-UL30-1158 | + | AATACACCGTCAGGTGGGCC | 20 | 4096 |
| HSV1-UL30-1159 | + | CAGGTGGGCCAGGCGCTTGT | 20 | 4097 |
| HSV1-UL30-1160 | + | CGCTTGTTGGTGTACGCGCG | 20 | 4098 |
| HSV1-UL30-1161 | + | GCTTGTTGGTGTACGCGCGC | 20 | 4099 |
| HSV1-UL30-1162 | + | GCGCGGGTGTCTGCTCAGTT | 20 | 4100 |
| HSV1-UL30-1163 | + | CGGGTGTCTGCTCAGTTCGG | 20 | 4101 |
| HSV1-UL30-1164 | + | GTCTGCTCAGTTCGGCGGTG | 20 | 4102 |
| HSV1-UL30-1165 | + | CGGCGGTGAGGACAAAGTCC | 20 | 4103 |
| HSV1-UL30-1166 | + | AAGTCCTGGATGTCCCTCTC | 20 | 4104 |
| HSV1-UL30-1167 | + | AGTCCTGGATGTCCCTCTCC | 20 | 4105 |
| HSV1-UL30-1168 | + | CTGGATGTCCCTCTCCGGGT | 20 | 4106 |
| HSV1-UL30-1169 | + | CCGGGTCGGTGATGCGCCGA | 20 | 4107 |
| HSV1-UL30-1170 | + | CGGGTCGGTGATGCGCCGAT | 20 | 4108 |
| HSV1-UL30-1171 | + | TGCGCCGATGGGCGTCTACG | 20 | 4109 |
| HSV1-UL30-1172 | + | CCGATGGGCGTCTACGAGGA | 20 | 4110 |
| HSV1-UL30-1173 | + | CCCGAACGCCTGCAGTCCCT | 20 | 4111 |
| HSV1-UL30-1174 | + | CCGAACGCCTGCAGTCCCTC | 20 | 4112 |
| HSV1-UL30-1175 | + | ACGCCTGCAGTCCCTCGGGC | 20 | 4113 |
| HSV1-UL30-1176 | + | CGCCTGCAGTCCCTCGGGCA | 20 | 4114 |
| HSV1-UL30-1177 | + | GCCTGCAGTCCCTCGGGCAG | 20 | 4115 |
| HSV1-UL30-1178 | + | TCGCGCCAGCCACTCCTCCG | 20 | 4116 |
| HSV1-UL30-1179 | + | CGCGCCAGCCACTCCTCCGC | 20 | 4117 |
| HSV1-UL30-1180 | + | GCGCCAGCCACTCCTCCGCG | 20 | 4118 |
| HSV1-UL30-1181 | + | CCACTCCTCCGCGGGGCGCT | 20 | 4119 |
| HSV1-UL30-1182 | + | CGCGGGGCGCTCGGCTAACG | 20 | 4120 |
| HSV1-UL30-1183 | + | GGGGCGCTCGGCTAACGCGG | 20 | 4121 |
| HSV1-UL30-1184 | + | GGCTAACGCGGCGGCCGCTC | 20 | 4122 |
| HSV1-UL30-1185 | + | CGCGGCGGCCGCTCCGGAGA | 20 | 4123 |
| HSV1-UL30-1186 | + | CGGTATCGTCGTAAAACAGC | 20 | 4124 |
| HSV1-UL30-1187 | + | CGTAAAACAGCAGGTCGACC | 20 | 4125 |
| HSV1-UL30-1188 | + | GTAAAACAGCAGGTCGACCA | 20 | 4126 |
| HSV1-UL30-1189 | + | CAGCAGGTCGACCAGGGCCC | 20 | 4127 |
| HSV1-UL30-1190 | + | CAGGTCGACCAGGGCCCTGG | 20 | 4128 |
| HSV1-UL30-1191 | + | CGACCAGGGCCCTGGAGGTG | 20 | 4129 |
| HSV1-UL30-1192 | + | GACGCCGATGTACTTTTTCT | 20 | 4130 |
| HSV1-UL30-1193 | + | CTTGGCGATCAGCAGCAGCT | 20 | 4131 |
| HSV1-UL30-1194 | + | CTTTTCGCACTCGAGTTTGA | 20 | 4132 |
| HSV1-UL30-1195 | + | TTTTCGCACTCGAGTTTGAT | 20 | 4133 |
| HSV1-UL30-1196 | + | TTTCGCACTCGAGTTTGATG | 20 | 4134 |
| HSV1-UL30-1197 | + | TTCGCACTCGAGTTTGATGG | 20 | 4135 |
| HSV1-UL30-1198 | + | TCGCACTCGAGTTTGATGGG | 20 | 4136 |
| HSV1-UL30-1199 | + | GAAACAGCGCGCGCGAGATG | 20 | 4137 |
| HSV1-UL30-1200 | + | GCTCGCCATCTTGTCGCCCA | 20 | 4138 |
| HSV1-UL30-1201 | + | GTCGCCCACGGCCGTCAGCC | 20 | 4139 |
| HSV1-UL30-1202 | + | GCCCACGGCCGTCAGCCCGG | 20 | 4140 |
| HSV1-UL30-1203 | + | CCGTCAGCCCGGCGGCCGTG | 20 | 4141 |
| HSV1-UL30-1204 | + | GCCCGGCGGCCGTGAGGCCG | 20 | 4142 |
| HSV1-UL30-1205 | + | GCCGCGGCACAGCACAAAGA | 20 | 4143 |
| HSV1-UL30-1206 | + | GTCCCCGTAGATGATGCGCA | 20 | 4144 |
| HSV1-UL30-1207 | + | GTAGATGATGCGCATGGAAT | 20 | 4145 |
| HSV1-UL30-1208 | + | TAGATGATGCGCATGGAATA | 20 | 4146 |
| HSV1-UL30-1209 | + | GATGCGCATGGAATAGGGCC | 20 | 4147 |
| HSV1-UL30-1210 | + | ATGCGCATGGAATAGGGCCC | 20 | 4148 |
| HSV1-UL30-1211 | + | TGCGCATGGAATAGGGCCCG | 20 | 4149 |
| HSV1-UL30-1212 | + | GCGCATGGAATAGGGCCCGG | 20 | 4150 |
| HSV1-UL30-1213 | + | GGGCCCGGGGGCGCGCATGT | 20 | 4151 |
| HSV1-UL30-1214 | + | GCGCGCATGTCGGCCGCCTC | 20 | 4152 |
| HSV1-UL30-1215 | + | CGCGCATGTCGGCCGCCTCC | 20 | 4153 |
| HSV1-UL30-1216 | + | GTCGGCCGCCTCCGGGAAAT | 20 | 4154 |
| HSV1-UL30-1217 | + | CCGCCTCCGGGAAATCGGCC | 20 | 4155 |
| HSV1-UL30-1218 | + | ATCGGCCAGGAGCTGTTCGA | 20 | 4156 |
| HSV1-UL30-1219 | + | CGAAGGCCGCCCAGCGCGCG | 20 | 4157 |
| HSV1-UL30-1220 | + | AGCGCGCGTGGACGTACTCG | 20 | 4158 |
| HSV1-UL30-1221 | + | GCGCGCGTGGACGTACTCGC | 20 | 4159 |
| HSV1-UL30-1222 | + | GGGTCGCGAGCAGCATCTCG | 20 | 4160 |
| HSV1-UL30-1223 | + | GAGCAGCATCTCGCGGCCGA | 20 | 4161 |
| HSV1-UL30-1224 | + | GCCGATGGTCGTCACCGTCG | 20 | 4162 |
| HSV1-UL30-1225 | + | TCACCGTCGCGGCAACGTGC | 20 | 4163 |
| HSV1-UL30-1226 | + | GTCGCGGCAACGTGCAGGCA | 20 | 4164 |
| HSV1-UL30-1227 | + | CGGCAACGTGCAGGCACGGC | 20 | 4165 |
| HSV1-UL30-1228 | + | CGAGTTACACACGACCTTGA | 20 | 4166 |
| HSV1-UL30-1229 | + | GTTACACACGACCTTGATGG | 20 | 4167 |
| HSV1-UL30-1230 | + | TGGCGGCCTGCTGCTTGTCC | 20 | 4168 |
| HSV1-UL30-1231 | + | CTGCTGCTTGTCCAGGAGCA | 20 | 4169 |
| HSV1-UL30-1232 | + | GTCCAGGAGCACGGCCTCCT | 20 | 4170 |
| HSV1-UL30-1233 | + | TCCAGGAGCACGGCCTCCTC | 20 | 4171 |
| HSV1-UL30-1234 | + | CCAGGAGCACGGCCTCCTCG | 20 | 4172 |
| HSV1-UL30-1235 | + | CGGCCTCCTCGGGGCTGCTC | 20 | 4173 |
| HSV1-UL30-1236 | + | GGCCTCCTCGGGGCTGCTCT | 20 | 4174 |
| HSV1-UL30-1237 | + | GCCTCCTCGGGGCTGCTCTG | 20 | 4175 |
| HSV1-UL30-1238 | + | TGCTCTGGGGAATCCGCGAG | 20 | 4176 |
| HSV1-UL30-1239 | + | CGAGCGGATCTGCTTTCGCA | 20 | 4177 |
| HSV1-UL30-1240 | + | GCATGGCGAGCCAGTCCCGC | 20 | 4178 |
| HSV1-UL30-1241 | + | TGGCGAGCCAGTCCCGCAGG | 20 | 4179 |
| HSV1-UL30-1242 | + | AGTCCCGCAGGAGGATGCTG | 20 | 4180 |
| HSV1-UL30-1243 | + | CCCGCAGGAGGATGCTGAGG | 20 | 4181 |
| HSV1-UL30-1244 | + | GCCCCCCCACCTCGATCTCC | 20 | 4182 |
| HSV1-UL30-1245 | + | GGTAGTCCTTGCCCGCCTCC | 20 | 4183 |
| HSV1-UL30-1246 | + | CTCCAGGTGCGCCACTGCGT | 20 | 4184 |
| HSV1-UL30-1247 | + | GCGCCACTGCGTCGGCCCTC | 20 | 4185 |
| HSV1-UL30-1248 | + | CGCCACTGCGTCGGCCCTCA | 20 | 4186 |
| HSV1-UL30-1249 | + | GGGAGAGCGTGCTGAAGCAC | 20 | 4187 |
| HSV1-UL30-1250 | + | GCGTGCTGAAGCACAGGTTG | 20 | 4188 |
| HSV1-UL30-1251 | + | CGTGCTGAAGCACAGGTTGT | 20 | 4189 |
| HSV1-UL30-1252 | + | TGAAGCACAGGTTGTGGGCC | 20 | 4190 |
| HSV1-UL30-1253 | + | GTTGTGGGCCTGGATGATGC | 20 | 4191 |
| HSV1-UL30-1254 | + | TTGTGGGCCTGGATGATGCT | 20 | 4192 |
| HSV1-UL30-1255 | + | TGTGGGCCTGGATGATGCTG | 20 | 4193 |
| HSV1-UL30-1256 | + | CCTGGATGATGCTGGGGTAC | 20 | 4194 |
| HSV1-UL30-1257 | + | GATGATGCTGGGGTACAGGC | 20 | 4195 |
| HSV1-UL30-1258 | + | GGCAAAGTCGAACACCACCA | 20 | 4196 |
| HSV1-UL30-1259 | + | GCAAAGTCGAACACCACCAC | 20 | 4197 |
| HSV1-UL30-1260 | + | CAAAGTCGAACACCACCACG | 20 | 4198 |
| HSV1-UL30-1261 | + | CACGGGGTTCACGTGAAACC | 20 | 4199 |
| HSV1-UL30-1262 | + | GTTCACGTGAAACCCGGAAG | 20 | 4200 |
| HSV1-UL30-1263 | + | TTCACGTGAAACCCGGAAGT | 20 | 4201 |
| HSV1-UL30-1264 | + | TCACGTGAAACCCGGAAGTG | 20 | 4202 |
| HSV1-UL30-1265 | + | GAAACCCGGAAGTGGGGTCA | 20 | 4203 |
| HSV1-UL30-1266 | + | GGAAGTGGGGTCAAGGACCC | 20 | 4204 |
| HSV1-UL30-1267 | + | GGTCAAGGACCCTGGCCCCC | 20 | 4205 |
| HSV1-UL30-1268 | + | CTGGTACCCCACGTGCCTGC | 20 | 4206 |
| HSV1-UL30-1269 | + | GTACCCCACGTGCCTGCCGG | 20 | 4207 |
| HSV1-UL30-1270 | + | GCGGTCTCCCGCGCGCCCTC | 20 | 4208 |
| HSV1-UL30-1271 | + | TCGTCCTCCCCCTCCTCCTC | 20 | 4209 |
| HSV1-UL30-1272 | + | CTGGCCGCTCCTCGTCCTCC | 20 | 4210 |
| HSV1-UL30-1273 | + | TGGCCGCTCCTCGTCCTCCC | 20 | 4211 |
| HSV1-UL30-1274 | + | CTCCTCGTCCTCCCGGGCTG | 20 | 4212 |
| HSV1-UL30-1275 | + | TCGTCCTCCCGGGCTGCGGC | 20 | 4213 |
| HSV1-UL30-1276 | + | CCGGGCTGCGGCCGGACGCT | 20 | 4214 |
| HSV1-UL30-1277 | + | CGGGCTGCGGCCGGACGCTT | 20 | 4215 |
| HSV1-UL30-1278 | + | ACGCTTGGGCGCCTCCCCCC | 20 | 4216 |
| HSV1-UL30-1279 | + | CGGCGCCCCTAAATCGCCCC | 20 | 4217 |
| HSV1-UL30-1280 | + | GGCGCCCCTAAATCGCCCCT | 20 | 4218 |
| HSV1-UL30-1281 | + | CTAAATCGCCCCTGGGTGTC | 20 | 4219 |
| HSV1-UL30-1282 | + | CCGGCAGAATAAAGCCCTTC | 20 | 4220 |
| HSV1-UL30-1283 | + | CAGAATAAAGCCCTTCTGGT | 20 | 4221 |
| HSV1-UL30-1284 | + | TAAAGCCCTTCTGGTCGGCC | 20 | 4222 |
| HSV1-UL30-1285 | + | TCTGGTCGGCCAGGCGCAGC | 20 | 4223 |
| HSV1-UL30-1286 | + | GCAGCAGGCACGTAAAGACG | 20 | 4224 |
| HSV1-UL30-1287 | + | ACGTAAAGACGCGGATCTGC | 20 | 4225 |
| HSV1-UL30-1288 | + | GATCTGCTGGCCGTCGTAGA | 20 | 4226 |
| HSV1-UL30-1289 | + | GCTGGCCGTCGTAGATGGTG | 20 | 4227 |
| HSV1-UL30-1290 | + | CTGGCCGTCGTAGATGGTGC | 20 | 4228 |
| HSV1-UL30-1291 | + | AATACCCGCCAAGCGCGCGA | 20 | 4229 |
| HSV1-UL30-1292 | + | CGACGGCCGAGAGCTCCAGA | 20 | 4230 |
| HSV1-UL30-1293 | + | GACGGCCGAGAGCTCCAGAT | 20 | 4231 |
| HSV1-UL30-1294 | + | ACGGCCGAGAGCTCCAGATG | 20 | 4232 |
| HSV1-UL30-1295 | + | GCAAAAACTTAAAAAACAGC | 20 | 4233 |
| HSV1-UL30-1296 | + | AAAACAGCTGGCCCACCAGC | 20 | 4234 |
| HSV1-UL30-1297 | + | AAACAGCTGGCCCACCAGCA | 20 | 4235 |
| HSV1-UL30-1298 | + | GCCGATCACCCCGCGTTGCG | 20 | 4236 |
| HSV1-UL30-1299 | + | CCGATCACCCCGCGTTGCGC | 20 | 4237 |
| HSV1-UL30-1300 | + | CACCCCGCGTTGCGCGGGCC | 20 | 4238 |
| HSV1-UL30-1301 | + | CCCGCGTTGCGCGGGCCCGG | 20 | 4239 |
| HSV1-UL30-1302 | + | CGCGGGCCCGGCGGCGTAGT | 20 | 4240 |
| HSV1-UL30-1303 | + | GGGCCCGGCGGCGTAGTAGG | 20 | 4241 |
| HSV1-UL30-1304 | + | GGCCCGGCGGCGTAGTAGGC | 20 | 4242 |
| HSV1-UL30-1305 | + | GCCCGGCGGCGTAGTAGGCG | 20 | 4243 |
| HSV1-UL30-1306 | + | CGGGGATGTCGCGATAGCTC | 20 | 4244 |
| HSV1-UL30-1307 | + | GGTCCTTCTTCTTGTCCTTC | 20 | 4245 |
| HSV1-UL30-1308 | + | CTTCTTCTTGTCCTTCAGGA | 20 | 4246 |
| HSV1-UL30-1309 | + | CTTGTCCTTCAGGACGGCTT | 20 | 4247 |
| HSV1-UL30-1310 | + | CTTCAGGACGGCTTCGGCCA | 20 | 4248 |
| HSV1-UL30-1311 | + | GCTCGAGAGCTTGATCTTGT | 20 | 4249 |
| HSV1-UL30-1312 | + | CCTTTATCTTGCTGCGCTTC | 20 | 4250 |
| HSV1-UL30-1313 | + | TCTTGCTGCGCTTCTGGAAG | 20 | 4251 |
| HSV1-UL30-1314 | + | TGCGCTTCTGGAAGTGGCTC | 20 | 4252 |
| HSV1-UL30-1315 | + | CCCACACGCGAAACACGCCC | 20 | 4253 |
| HSV1-UL30-1316 | + | ACACGCCCCGGCCGTTCATG | 20 | 4254 |
| HSV1-UL30-1317 | + | TCATGCGGCCGTACCCGTCC | 20 | 4255 |
| HSV1-UL30-1318 | + | CATGCGGCCGTACCCGTCCA | 20 | 4256 |
| HSV1-UL30-1319 | + | ATGCGGCCGTACCCGTCCAG | 20 | 4257 |
| HSV1-UL30-1320 | + | TGCGGCCGTACCCGTCCAGG | 20 | 4258 |
| HSV1-UL30-1321 | + | CTTGTAAATGTCCGTCAGCT | 20 | 4259 |
| HSV1-UL30-1322 | + | CGTCAGCTTGGCCAGCAAGA | 20 | 4260 |
| HSV1-UL30-1323 | + | GTCAGCTTGGCCAGCAAGAA | 20 | 4261 |
| HSV1-UL30-1324 | + | GAAGTTGATGATGTTGTACC | 20 | 4262 |
| HSV1-UL30-1325 | + | GTTGTACCCGGTCACGAACT | 20 | 4263 |
| HSV1-UL30-1326 | + | TTGTACCCGGTCACGAACTC | 20 | 4264 |
| HSV1-UL30-1327 | + | TGTACCCGGTCACGAACTCG | 20 | 4265 |
| HSV1-UL30-1328 | + | CGGGGCCGTACTGTTTCACA | 20 | 4266 |
| HSV1-UL30-1329 | + | GGGGCCGTACTGTTTCACAA | 20 | 4267 |
| HSV1-UL30-1330 | + | CTGTTTCACAAGGGTCATGA | 20 | 4268 |
| HSV1-UL30-1331 | + | GCTGTCGAATTCCAGAACCA | 20 | 4269 |
| HSV1-UL30-1332 | + | CTGTCGAATTCCAGAACCAC | 20 | 4270 |
| HSV1-UL30-1333 | + | GAATTCCAGAACCACGGGCG | 20 | 4271 |
| HSV1-UL30-1334 | + | AATTCCAGAACCACGGGCGT | 20 | 4272 |
| HSV1-UL30-1335 | + | CCAGAACCACGGGCGTGGGC | 20 | 4273 |
| HSV1-UL30-1336 | + | CACGGGCGTGGGCAGGCCCC | 20 | 4274 |
| HSV1-UL30-1337 | + | CCCTGGCCGCCAGCTCGTTC | 20 | 4275 |
| HSV1-UL30-1338 | + | TGGCCGCCAGCTCGTTCAGG | 20 | 4276 |
| HSV1-UL30-1339 | + | GGCCGCCAGCTCGTTCAGGT | 20 | 4277 |
| HSV1-UL30-1340 | + | CAGCTCGTTCAGGTGGGATT | 20 | 4278 |
| HSV1-UL30-1341 | + | AGCTCGTTCAGGTGGGATTC | 20 | 4279 |
| HSV1-UL30-1342 | + | GCTCGTTCAGGTGGGATTCG | 20 | 4280 |
| HSV1-UL30-1343 | + | CGTTCAGGTGGGATTCGGGG | 20 | 4281 |
| HSV1-UL30-1344 | + | GTGGGATTCGGGGAGGTCGC | 20 | 4282 |
| HSV1-UL30-1345 | + | CGCAGGAACCGAGCGAAAAC | 20 | 4283 |
| HSV1-UL30-1346 | + | AGGAACCGAGCGAAAACAGG | 20 | 4284 |
| HSV1-UL30-1347 | + | AAAACAGGAGGACGTGCTCC | 20 | 4285 |
| HSV1-UL30-1348 | + | AAACAGGAGGACGTGCTCCA | 20 | 4286 |
| HSV1-UL30-1349 | + | CAGGAGGACGTGCTCCAGGG | 20 | 4287 |
| HSV1-UL30-1350 | + | GAGGACGTGCTCCAGGGCGG | 20 | 4288 |
| HSV1-UL30-1351 | + | GACGTGCTCCAGGGCGGTGG | 20 | 4289 |
| HSV1-UL30-1352 | + | GCTCCAGGGCGGTGGTGGAC | 20 | 4290 |
| HSV1-UL30-1353 | + | GGACAGGTCGTAGAGCAGAC | 20 | 4291 |
| HSV1-UL30-1354 | + | CGTAGAGCAGACAGGATATC | 20 | 4292 |
| HSV1-UL30-1355 | + | GACAGGATATCTGGATGACC | 20 | 4293 |
| HSV1-UL30-1356 | + | ATCTGGATGACCAGGTCCTC | 20 | 4294 |
| HSV1-UL30-1357 | + | TCTGGATGACCAGGTCCTCC | 20 | 4295 |
| HSV1-UL30-1358 | + | GACCAGGTCCTCCGGGTGCC | 20 | 4296 |
| HSV1-UL30-1359 | + | TCCTCCGGGTGCCCGGCCAC | 20 | 4297 |
| HSV1-UL30-1360 | + | CGGGTGCCCGGCCACCGGAA | 20 | 4298 |
| HSV1-UL30-1361 | + | AAGCACATGAGCTTGTATGC | 20 | 4299 |
| HSV1-UL30-1362 | + | ACATGAGCTTGTATGCCGGT | 20 | 4300 |
| HSV1-UL30-1363 | + | GTCGCTCATGCCCCCCTCGA | 20 | 4301 |
| HSV1-UL30-1364 | + | TCATGCCCCCCTCGATGGCC | 20 | 4302 |
| HSV1-UL30-1365 | + | ACAGTTAAACTCGACGTCGC | 20 | 4303 |
| HSV1-UL30-1366 | + | GACGTCGCTGGATGTCCCGA | 20 | 4304 |
| HSV1-UL30-1367 | + | CTGGATGTCCCGAAGGCCAT | 20 | 4305 |
| HSV1-UL30-1368 | + | TGGATGTCCCGAAGGCCATC | 20 | 4306 |
| HSV1-UL30-1369 | + | GGATGTCCCGAAGGCCATCG | 20 | 4307 |
| HSV1-UL30-1370 | + | CCGAAGGCCATCGGGGCCGC | 20 | 4308 |
| HSV1-UL30-1371 | + | AGGCCATCGGGGCCGCCGGC | 20 | 4309 |
| HSV1-UL30-1372 | + | GGCCATCGGGGCCGCCGGCT | 20 | 4310 |
| HSV1-UL30-1373 | + | GCTGGGCTAGCGTGTTGTTC | 20 | 4311 |
| HSV1-UL30-1374 | + | GCTAGCGTGTTGTTCCGGCC | 20 | 4312 |
| HSV1-UL30-1375 | + | TGTTCCGGCCCGGTTTGAGA | 20 | 4313 |
| HSV1-UL30-1376 | + | TTTGAGACGGTACCAGCCGA | 20 | 4314 |
| HSV1-UL30-1377 | + | CCAGCCGAAGGTGACGAACC | 20 | 4315 |
| HSV1-UL30-1378 | + | CAGCCGAAGGTGACGAACCC | 20 | 4316 |
| HSV1-UL30-1379 | + | AGCCGAAGGTGACGAACCCG | 20 | 4317 |
| HSV1-UL30-1380 | + | TGACGAACCCGGGGTTGTCC | 20 | 4318 |
| HSV1-UL30-1381 | + | CGGGGTTGTCCAGGATGAAC | 20 | 4319 |
| HSV1-UL30-1382 | + | GGGGTTGTCCAGGATGAACC | 20 | 4320 |
| HSV1-UL30-1383 | + | GTTGTCCAGGATGAACCGGG | 20 | 4321 |
| HSV1-UL30-1384 | + | GTCCAGGATGAACCGGGTGG | 20 | 4322 |
| HSV1-UL30-1385 | + | CCCACCCTCGTACTTCTTGA | 20 | 4323 |
| HSV1-UL30-1386 | + | CCCTCGTACTTCTTGATGGC | 20 | 4324 |
| HSV1-UL30-1387 | + | CCTCGTACTTCTTGATGGCC | 20 | 4325 |
| HSV1-UL30-1388 | + | CCGGGCAGAAGTTGTCGCAC | 20 | 4326 |
| HSV1-UL30-1389 | + | ACGACAGCACACGCCCGCTT | 20 | 4327 |
| HSV1-UL30-1390 | + | GCCCGCTTCGGACGTAGACG | 20 | 4328 |
| HSV1-UL30-1391 | + | GTAGACGCGGTAAAACAGAG | 20 | 4329 |
| HSV1-UL30-1392 | + | TAGACGCGGTAAAACAGAGC | 20 | 4330 |
| HSV1-UL30-1393 | + | AGACGCGGTAAAACAGAGCG | 20 | 4331 |
| HSV1-UL30-1394 | + | CGTCTCGTAGTAGTACACGT | 20 | 4332 |
| HSV1-UL30-1395 | + | CCACCACCTCCGCCTCGAAG | 20 | 4333 |
| HSV1-UL30-1396 | + | CTCCGCCTCGAAGTGGTCCG | 20 | 4334 |
| HSV1-UL30-1397 | + | AGTGGTCCGCGGAGATGCCG | 20 | 4335 |
| HSV1-UL30-1398 | + | ATGCCGCGGAACGACGCGCC | 20 | 4336 |
| HSV1-UL30-1399 | + | TGCCGCGGAACGACGCGCCC | 20 | 4337 |
| HSV1-UL30-1400 | + | GCCGCGGAACGACGCGCCCG | 20 | 4338 |
| HSV1-UL30-1401 | + | ACGCGCCCGGGGACTCGCGC | 20 | 4339 |
| HSV1-UL30-1402 | + | CGCGCCCGGGGACTCGCGCA | 20 | 4340 |
| HSV1-UL30-1403 | + | CGGGGACTCGCGCAGGGCCG | 20 | 4341 |
| HSV1-UL30-1404 | + | ATGCGCTCGCAGAGATCTCG | 20 | 4342 |
| HSV1-UL30-1405 | + | TGCGCTCGCAGAGATCTCGT | 20 | 4343 |
| HSV1-UL30-1406 | + | GCGCTCGCAGAGATCTCGTG | 20 | 4344 |
| HSV1-UL30-1407 | + | CGCAGAGATCTCGTGGGGCG | 20 | 4345 |
| HSV1-UL30-1408 | + | CTCGTGGGGCGCGGCATTGT | 20 | 4346 |
| HSV1-UL30-1409 | + | CCGCGTGCCGTAAACGTGAA | 20 | 4347 |
| HSV1-UL30-1410 | + | CGTAAACGTGAACGGCCACC | 20 | 4348 |
| HSV1-UL30-1411 | + | AAACGTGAACGGCCACCCGG | 20 | 4349 |
| HSV1-UL30-1412 | + | ACGGCCACCCGGTGGCCTTC | 20 | 4350 |
| HSV1-UL30-1413 | + | CCCGGTGGCCTTCCGGAGTC | 20 | 4351 |
| HSV1-UL30-1414 | + | GGCCTTCCGGAGTCAGGCCC | 20 | 4352 |
| HSV1-UL30-1415 | + | CAGGCCCAGGAGCGTGATGA | 20 | 4353 |
| HSV1-UL30-1416 | + | AGCGTGATGACGGTCCCCGT | 20 | 4354 |
| HSV1-UL30-1417 | + | GACGGTCCCCGTCGGTGTGA | 20 | 4355 |
| HSV1-UL30-1418 | + | TGGCGTCCATAAACCGCGCG | 20 | 4356 |
| HSV1-UL30-1419 | + | GGCGTCCATAAACCGCGCGT | 20 | 4357 |
| HSV1-UL30-1420 | + | GCGTCCATAAACCGCGCGTG | 20 | 4358 |
| HSV1-UL30-1421 | + | CGTCCATAAACCGCGCGTGG | 20 | 4359 |
| HSV1-UL30-1422 | + | TTCCTCCGCCGTAGCGC | 17 | 4360 |
| HSV1-UL30-1423 | + | CACCGCCCCGAACCCTG | 17 | 4361 |
| HSV1-UL30-1424 | + | CCCCGAACCCTGCGGTC | 17 | 4362 |
| HSV1-UL30-1425 | + | TGCGGTCCGGAGCCGCG | 17 | 4363 |
| HSV1-UL30-1426 | + | CGCGCGGCCACGTCGTC | 17 | 4364 |
| HSV1-UL30-1427 | + | GCGCGGCCACGTCGTCC | 17 | 4365 |
| HSV1-UL30-1428 | + | CGCGGCCACGTCGTCCG | 17 | 4366 |
| HSV1-UL30-1429 | + | GCGGCCACGTCGTCCGG | 17 | 4367 |
| HSV1-UL30-1430 | + | CGGCCACGTCGTCCGGG | 17 | 4368 |
| HSV1-UL30-1431 | + | CGGGGGGTGCCACACTT | 17 | 4369 |
| HSV1-UL30-1432 | + | GGGGGGTGCCACACTTC | 17 | 4370 |
| HSV1-UL30-1433 | + | CCTTTTTAACAGACTCT | 17 | 4371 |
| HSV1-UL30-1434 | + | CAGACTCTCGGTGATCT | 17 | 4372 |
| HSV1-UL30-1435 | + | TGGCGTTATTCCCAAAC | 17 | 4373 |
| HSV1-UL30-1436 | + | GGCGTTATTCCCAAACA | 17 | 4374 |
| HSV1-UL30-1437 | + | CGCACGCCGCCCCCAAC | 17 | 4375 |
| HSV1-UL30-1438 | + | ACGCCGCCCCCAACAGG | 17 | 4376 |
| HSV1-UL30-1439 | + | CGCCGCCCCCAACAGGT | 17 | 4377 |
| HSV1-UL30-1440 | + | AGTAATAGTCCGTGTTC | 17 | 4378 |
| HSV1-UL30-1441 | + | GTAATAGTCCGTGTTCA | 17 | 4379 |
| HSV1-UL30-1442 | + | TGTTCAGGGCGACGCCG | 17 | 4380 |
| HSV1-UL30-1443 | + | GTTCAGGGCGACGCCGT | 17 | 4381 |
| HSV1-UL30-1444 | + | GGCGACGCCGTGGGCAA | 17 | 4382 |
| HSV1-UL30-1445 | + | GTGGGCAATGGCGTATG | 17 | 4383 |
| HSV1-UL30-1446 | + | TGGGCAATGGCGTATGC | 17 | 4384 |
| HSV1-UL30-1447 | + | GGCGTATGCGGGATCCT | 17 | 4385 |
| HSV1-UL30-1448 | + | GGGATCCTCGGCCAGCT | 17 | 4386 |
| HSV1-UL30-1449 | + | CGGACACCAGCAGCTTG | 17 | 4387 |
| HSV1-UL30-1450 | + | GGACACCAGCAGCTTGC | 17 | 4388 |
| HSV1-UL30-1451 | + | GACACCAGCAGCTTGCG | 17 | 4389 |
| HSV1-UL30-1452 | + | CAGCAGCTTGCGGGGCT | 17 | 4390 |
| HSV1-UL30-1453 | + | GGCTTGGACGCGCCTCC | 17 | 4391 |
| HSV1-UL30-1454 | + | GCTTGGACGCGCCTCCC | 17 | 4392 |
| HSV1-UL30-1455 | + | CTTGGACGCGCCTCCCG | 17 | 4393 |
| HSV1-UL30-1456 | + | TTGGACGCGCCTCCCGG | 17 | 4394 |
| HSV1-UL30-1457 | + | TGGACGCGCCTCCCGGG | 17 | 4395 |
| HSV1-UL30-1458 | + | CGCGCCTCCCGGGGGGT | 17 | 4396 |
| HSV1-UL30-1459 | + | CCTCCCGGGGGGTCGGC | 17 | 4397 |
| HSV1-UL30-1460 | + | GGGGGGTCGGCAGGCGA | 17 | 4398 |
| HSV1-UL30-1461 | + | CAGGCGACGGCGTCTCC | 17 | 4399 |
| HSV1-UL30-1462 | + | AGGCGACGGCGTCTCCC | 17 | 4400 |
| HSV1-UL30-1463 | + | GGCGACGGCGTCTCCCG | 17 | 4401 |
| HSV1-UL30-1464 | + | GCGACGGCGTCTCCCGG | 17 | 4402 |
| HSV1-UL30-1465 | + | CGTCTCCCGGGGGCGCT | 17 | 4403 |
| HSV1-UL30-1466 | + | TCCCGGGGGCGCTTGGC | 17 | 4404 |
| HSV1-UL30-1467 | + | CCCGGGGGCGCTTGGCC | 17 | 4405 |
| HSV1-UL30-1468 | + | CCGGGGGCGCTTGGCCG | 17 | 4406 |
| HSV1-UL30-1469 | + | GGGGCGCTTGGCCGGGG | 17 | 4407 |
| HSV1-UL30-1470 | + | GGGCGCTTGGCCGGGGA | 17 | 4408 |
| HSV1-UL30-1471 | + | GCTTGGCCGGGGAGGGC | 17 | 4409 |
| HSV1-UL30-1472 | + | CTTGGCCGGGGAGGGCA | 17 | 4410 |
| HSV1-UL30-1473 | + | CGGGGAGGGCAGGGCCG | 17 | 4411 |
| HSV1-UL30-1474 | + | GGGGAGGGCAGGGCCGC | 17 | 4412 |
| HSV1-UL30-1475 | + | GGGAGGGCAGGGCCGCG | 17 | 4413 |
| HSV1-UL30-1476 | + | GGAGGGCAGGGCCGCGG | 17 | 4414 |
| HSV1-UL30-1477 | + | GAGGGCAGGGCCGCGGG | 17 | 4415 |
| HSV1-UL30-1478 | + | AGGGCAGGGCCGCGGGG | 17 | 4416 |
| HSV1-UL30-1479 | + | GGGCAGGGCCGCGGGGG | 17 | 4417 |
| HSV1-UL30-1480 | + | CAGGGCCGCGGGGGGGG | 17 | 4418 |
| HSV1-UL30-1481 | + | AGGGCCGCGGGGGGGGC | 17 | 4419 |
| HSV1-UL30-1482 | + | GGGGCGGGCTCGTCCCC | 17 | 4420 |
| HSV1-UL30-1483 | + | GGGCGGGCTCGTCCCCT | 17 | 4421 |
| HSV1-UL30-1484 | + | GGCGGGCTCGTCCCCTG | 17 | 4422 |
| HSV1-UL30-1485 | + | GGGCTCGTCCCCTGGGG | 17 | 4423 |
| HSV1-UL30-1486 | + | CTCGTCCCCTGGGGCGG | 17 | 4424 |
| HSV1-UL30-1487 | + | CGGCGGCGTCTAGCTCG | 17 | 4425 |
| HSV1-UL30-1488 | + | CGGCGTCTAGCTCGCGG | 17 | 4426 |
| HSV1-UL30-1489 | + | GGCGTCTAGCTCGCGGA | 17 | 4427 |
| HSV1-UL30-1490 | + | GTCTAGCTCGCGGAGGG | 17 | 4428 |
| HSV1-UL30-1491 | + | CCGTCTCCTCTACCTCG | 17 | 4429 |
| HSV1-UL30-1492 | + | CGTCTCCTCTACCTCGC | 17 | 4430 |
| HSV1-UL30-1493 | + | CCTCTACCTCGCGGGTC | 17 | 4431 |
| HSV1-UL30-1494 | + | CTCTACCTCGCGGGTCT | 17 | 4432 |
| HSV1-UL30-1495 | + | TGGGCCACGATCACGTA | 17 | 4433 |
| HSV1-UL30-1496 | + | GGGCCACGATCACGTAC | 17 | 4434 |
| HSV1-UL30-1497 | + | CGATCACGTACGGGATC | 17 | 4435 |
| HSV1-UL30-1498 | + | CGGGATCCGGTCCTTGA | 17 | 4436 |
| HSV1-UL30-1499 | + | ATCCGGTCCTTGATGGA | 17 | 4437 |
| HSV1-UL30-1500 | + | TCCGGTCCTTGATGGAC | 17 | 4438 |
| HSV1-UL30-1501 | + | TGGACGGGACCTGCGCG | 17 | 4439 |
| HSV1-UL30-1502 | + | ACGGGACCTGCGCGCGG | 17 | 4440 |
| HSV1-UL30-1503 | + | CGGGACCTGCGCGCGGC | 17 | 4441 |
| HSV1-UL30-1504 | + | GCTTGTAATACACCGTC | 17 | 4442 |
| HSV1-UL30-1505 | + | TGTAATACACCGTCAGG | 17 | 4443 |
| HSV1-UL30-1506 | + | GTAATACACCGTCAGGT | 17 | 4444 |
| HSV1-UL30-1507 | + | ACACCGTCAGGTGGGCC | 17 | 4445 |
| HSV1-UL30-1508 | + | GTGGGCCAGGCGCTTGT | 17 | 4446 |
| HSV1-UL30-1509 | + | TTGTTGGTGTACGCGCG | 17 | 4447 |
| HSV1-UL30-1510 | + | TGTTGGTGTACGCGCGC | 17 | 4448 |
| HSV1-UL30-1511 | + | CGGGTGTCTGCTCAGTT | 17 | 4449 |
| HSV1-UL30-1512 | + | GTGTCTGCTCAGTTCGG | 17 | 4450 |
| HSV1-UL30-1513 | + | TGCTCAGTTCGGCGGTG | 17 | 4451 |
| HSV1-UL30-1514 | + | CGGTGAGGACAAAGTCC | 17 | 4452 |
| HSV1-UL30-1515 | + | TCCTGGATGTCCCTCTC | 17 | 4453 |
| HSV1-UL30-1516 | + | CCTGGATGTCCCTCTCC | 17 | 4454 |
| HSV1-UL30-1517 | + | GATGTCCCTCTCCGGGT | 17 | 4455 |
| HSV1-UL30-1518 | + | GGTCGGTGATGCGCCGA | 17 | 4456 |
| HSV1-UL30-1519 | + | GTCGGTGATGCGCCGAT | 17 | 4457 |
| HSV1-UL30-1520 | + | GCCGATGGGCGTCTACG | 17 | 4458 |
| HSV1-UL30-1521 | + | ATGGGCGTCTACGAGGA | 17 | 4459 |
| HSV1-UL30-1522 | + | GAACGCCTGCAGTCCCT | 17 | 4460 |
| HSV1-UL30-1523 | + | AACGCCTGCAGTCCCTC | 17 | 4461 |
| HSV1-UL30-1524 | + | CCTGCAGTCCCTCGGGC | 17 | 4462 |
| HSV1-UL30-1525 | + | CTGCAGTCCCTCGGGCA | 17 | 4463 |
| HSV1-UL30-1526 | + | TGCAGTCCCTCGGGCAG | 17 | 4464 |
| HSV1-UL30-1527 | + | CGCCAGCCACTCCTCCG | 17 | 4465 |
| HSV1-UL30-1528 | + | GCCAGCCACTCCTCCGC | 17 | 4466 |
| HSV1-UL30-1529 | + | CCAGCCACTCCTCCGCG | 17 | 4467 |
| HSV1-UL30-1530 | + | CTCCTCCGCGGGGCGCT | 17 | 4468 |
| HSV1-UL30-1531 | + | GGGGCGCTCGGCTAACG | 17 | 4469 |
| HSV1-UL30-1532 | + | GCGCTCGGCTAACGCGG | 17 | 4470 |
| HSV1-UL30-1533 | + | TAACGCGGCGGCCGCTC | 17 | 4471 |
| HSV1-UL30-1534 | + | GGCGGCCGCTCCGGAGA | 17 | 4472 |
| HSV1-UL30-1535 | + | TATCGTCGTAAAACAGC | 17 | 4473 |
| HSV1-UL30-1536 | + | AAAACAGCAGGTCGACC | 17 | 4474 |
| HSV1-UL30-1537 | + | AAACAGCAGGTCGACCA | 17 | 4475 |
| HSV1-UL30-1538 | + | CAGGTCGACCAGGGCCC | 17 | 4476 |
| HSV1-UL30-1539 | + | GTCGACCAGGGCCCTGG | 17 | 4477 |
| HSV1-UL30-1540 | + | CCAGGGCCCTGGAGGTG | 17 | 4478 |
| HSV1-UL30-1541 | + | GCCGATGTACTTTTTCT | 17 | 4479 |
| HSV1-UL30-1542 | + | GGCGATCAGCAGCAGCT | 17 | 4480 |
| HSV1-UL30-1543 | + | TTCGCACTCGAGTTTGA | 17 | 4481 |
| HSV1-UL30-1544 | + | TCGCACTCGAGTTTGAT | 17 | 4482 |
| HSV1-UL30-1545 | + | CGCACTCGAGTTTGATG | 17 | 4483 |
| HSV1-UL30-1546 | + | GCACTCGAGTTTGATGG | 17 | 4484 |
| HSV1-UL30-1547 | + | CACTCGAGTTTGATGGG | 17 | 4485 |
| HSV1-UL30-1548 | + | ACAGCGCGCGCGAGATG | 17 | 4486 |
| HSV1-UL30-1549 | + | CGCCATCTTGTCGCCCA | 17 | 4487 |
| HSV1-UL30-1550 | + | GCCCACGGCCGTCAGCC | 17 | 4488 |
| HSV1-UL30-1551 | + | CACGGCCGTCAGCCCGG | 17 | 4489 |
| HSV1-UL30-1552 | + | TCAGCCCGGCGGCCGTG | 17 | 4490 |
| HSV1-UL30-1553 | + | CGGCGGCCGTGAGGCCG | 17 | 4491 |
| HSV1-UL30-1554 | + | GCGGCACAGCACAAAGA | 17 | 4492 |
| HSV1-UL30-1555 | + | CCCGTAGATGATGCGCA | 17 | 4493 |
| HSV1-UL30-1556 | + | GATGATGCGCATGGAAT | 17 | 4494 |
| HSV1-UL30-1557 | + | ATGATGCGCATGGAATA | 17 | 4495 |
| HSV1-UL30-1558 | + | GCGCATGGAATAGGGCC | 17 | 4496 |
| HSV1-UL30-1559 | + | CGCATGGAATAGGGCCC | 17 | 4497 |
| HSV1-UL30-1560 | + | GCATGGAATAGGGCCCG | 17 | 4498 |
| HSV1-UL30-1561 | + | CATGGAATAGGGCCCGG | 17 | 4499 |
| HSV1-UL30-1562 | + | CCCGGGGGCGCGCATGT | 17 | 4500 |
| HSV1-UL30-1563 | + | CGCATGTCGGCCGCCTC | 17 | 4501 |
| HSV1-UL30-1564 | + | GCATGTCGGCCGCCTCC | 17 | 4502 |
| HSV1-UL30-1565 | + | GGCCGCCTCCGGGAAAT | 17 | 4503 |
| HSV1-UL30-1566 | + | CCTCCGGGAAATCGGCC | 17 | 4504 |
| HSV1-UL30-1567 | + | GGCCAGGAGCTGTTCGA | 17 | 4505 |
| HSV1-UL30-1568 | + | AGGCCGCCCAGCGCGCG | 17 | 4506 |
| HSV1-UL30-1569 | + | GCGCGTGGACGTACTCG | 17 | 4507 |
| HSV1-UL30-1570 | + | CGCGTGGACGTACTCGC | 17 | 4508 |
| HSV1-UL30-1571 | + | TCGCGAGCAGCATCTCG | 17 | 4509 |
| HSV1-UL30-1572 | + | CAGCATCTCGCGGCCGA | 17 | 4510 |
| HSV1-UL30-1573 | + | GATGGTCGTCACCGTCG | 17 | 4511 |
| HSV1-UL30-1574 | + | CCGTCGCGGCAACGTGC | 17 | 4512 |
| HSV1-UL30-1575 | + | GCGGCAACGTGCAGGCA | 17 | 4513 |
| HSV1-UL30-1576 | + | CAACGTGCAGGCACGGC | 17 | 4514 |
| HSV1-UL30-1577 | + | GTTACACACGACCTTGA | 17 | 4515 |
| HSV1-UL30-1578 | + | ACACACGACCTTGATGG | 17 | 4516 |
| HSV1-UL30-1579 | + | CGGCCTGCTGCTTGTCC | 17 | 4517 |
| HSV1-UL30-1580 | + | CTGCTTGTCCAGGAGCA | 17 | 4518 |
| HSV1-UL30-1581 | + | CAGGAGCACGGCCTCCT | 17 | 4519 |
| HSV1-UL30-1582 | + | AGGAGCACGGCCTCCTC | 17 | 4520 |
| HSV1-UL30-1583 | + | GGAGCACGGCCTCCTCG | 17 | 4521 |
| HSV1-UL30-1584 | + | CCTCCTCGGGGCTGCTC | 17 | 4522 |
| HSV1-UL30-1585 | + | CTCCTCGGGGCTGCTCT | 17 | 4523 |
| HSV1-UL30-1586 | + | TCCTCGGGGCTGCTCTG | 17 | 4524 |
| HSV1-UL30-1587 | + | TCTGGGGAATCCGCGAG | 17 | 4525 |
| HSV1-UL30-1588 | + | GCGGATCTGCTTTCGCA | 17 | 4526 |
| HSV1-UL30-1589 | + | TGGCGAGCCAGTCCCGC | 17 | 4527 |
| HSV1-UL30-1590 | + | CGAGCCAGTCCCGCAGG | 17 | 4528 |
| HSV1-UL30-1591 | + | CCCGCAGGAGGATGCTG | 17 | 4529 |
| HSV1-UL30-1592 | + | GCAGGAGGATGCTGAGG | 17 | 4530 |
| HSV1-UL30-1593 | + | CCCCCACCTCGATCTCC | 17 | 4531 |
| HSV1-UL30-1594 | + | AGTCCTTGCCCGCCTCC | 17 | 4532 |
| HSV1-UL30-1595 | + | CAGGTGCGCCACTGCGT | 17 | 4533 |
| HSV1-UL30-1596 | + | CCACTGCGTCGGCCCTC | 17 | 4534 |
| HSV1-UL30-1597 | + | CACTGCGTCGGCCCTCA | 17 | 4535 |
| HSV1-UL30-1598 | + | AGAGCGTGCTGAAGCAC | 17 | 4536 |
| HSV1-UL30-1599 | + | TGCTGAAGCACAGGTTG | 17 | 4537 |
| HSV1-UL30-1600 | + | GCTGAAGCACAGGTTGT | 17 | 4538 |
| HSV1-UL30-1601 | + | AGCACAGGTTGTGGGCC | 17 | 4539 |
| HSV1-UL30-1602 | + | GTGGGCCTGGATGATGC | 17 | 4540 |
| HSV1-UL30-1603 | + | TGGGCCTGGATGATGCT | 17 | 4541 |
| HSV1-UL30-1604 | + | GGGCCTGGATGATGCTG | 17 | 4542 |
| HSV1-UL30-1605 | + | GGATGATGCTGGGGTAC | 17 | 4543 |
| HSV1-UL30-1606 | + | GATGCTGGGGTACAGGC | 17 | 4544 |
| HSV1-UL30-1607 | + | AAAGTCGAACACCACCA | 17 | 4545 |
| HSV1-UL30-1608 | + | AAGTCGAACACCACCAC | 17 | 4546 |
| HSV1-UL30-1609 | + | AGTCGAACACCACCACG | 17 | 4547 |
| HSV1-UL30-1610 | + | GGGGTTCACGTGAAACC | 17 | 4548 |
| HSV1-UL30-1611 | + | CACGTGAAACCCGGAAG | 17 | 4549 |
| HSV1-UL30-1612 | + | ACGTGAAACCCGGAAGT | 17 | 4550 |
| HSV1-UL30-1613 | + | CGTGAAACCCGGAAGTG | 17 | 4551 |
| HSV1-UL30-1614 | + | ACCCGGAAGTGGGGTCA | 17 | 4552 |
| HSV1-UL30-1615 | + | AGTGGGGTCAAGGACCC | 17 | 4553 |
| HSV1-UL30-1616 | + | CAAGGACCCTGGCCCCC | 17 | 4554 |
| HSV1-UL30-1617 | + | GTACCCCACGTGCCTGC | 17 | 4555 |
| HSV1-UL30-1618 | + | CCCCACGTGCCTGCCGG | 17 | 4556 |
| HSV1-UL30-1619 | + | GTCTCCCGCGCGCCCTC | 17 | 4557 |
| HSV1-UL30-1620 | + | TCCTCCCCCTCCTCCTC | 17 | 4558 |
| HSV1-UL30-1621 | + | GCCGCTCCTCGTCCTCC | 17 | 4559 |
| HSV1-UL30-1622 | + | CCGCTCCTCGTCCTCCC | 17 | 4560 |
| HSV1-UL30-1623 | + | CTCGTCCTCCCGGGCTG | 17 | 4561 |
| HSV1-UL30-1624 | + | TCCTCCCGGGCTGCGGC | 17 | 4562 |
| HSV1-UL30-1625 | + | GGCTGCGGCCGGACGCT | 17 | 4563 |
| HSV1-UL30-1626 | + | GCTGCGGCCGGACGCTT | 17 | 4564 |
| HSV1-UL30-1627 | + | CTTGGGCGCCTCCCCCC | 17 | 4565 |
| HSV1-UL30-1628 | + | CGCCCCTAAATCGCCCC | 17 | 4566 |
| HSV1-UL30-1629 | + | GCCCCTAAATCGCCCCT | 17 | 4567 |
| HSV1-UL30-1630 | + | AATCGCCCCTGGGTGTC | 17 | 4568 |
| HSV1-UL30-1631 | + | GCAGAATAAAGCCCTTC | 17 | 4569 |
| HSV1-UL30-1632 | + | AATAAAGCCCTTCTGGT | 17 | 4570 |
| HSV1-UL30-1633 | + | AGCCCTTCTGGTCGGCC | 17 | 4571 |
| HSV1-UL30-1634 | + | GGTCGGCCAGGCGCAGC | 17 | 4572 |
| HSV1-UL30-1635 | + | GCAGGCACGTAAAGACG | 17 | 4573 |
| HSV1-UL30-1636 | + | TAAAGACGCGGATCTGC | 17 | 4574 |
| HSV1-UL30-1637 | + | CTGCTGGCCGTCGTAGA | 17 | 4575 |
| HSV1-UL30-1638 | + | GGCCGTCGTAGATGGTG | 17 | 4576 |
| HSV1-UL30-1639 | + | GCCGTCGTAGATGGTGC | 17 | 4577 |
| HSV1-UL30-1640 | + | ACCCGCCAAGCGCGCGA | 17 | 4578 |
| HSV1-UL30-1641 | + | CGGCCGAGAGCTCCAGA | 17 | 4579 |
| HSV1-UL30-1642 | + | GGCCGAGAGCTCCAGAT | 17 | 4580 |
| HSV1-UL30-1643 | + | GCCGAGAGCTCCAGATG | 17 | 4581 |
| HSV1-UL30-1644 | + | AAAACTTAAAAAACAGC | 17 | 4582 |
| HSV1-UL30-1645 | + | ACAGCTGGCCCACCAGC | 17 | 4583 |
| HSV1-UL30-1646 | + | CAGCTGGCCCACCAGCA | 17 | 4584 |
| HSV1-UL30-1647 | + | GATCACCCCGCGTTGCG | 17 | 4585 |
| HSV1-UL30-1648 | + | ATCACCCCGCGTTGCGC | 17 | 4586 |
| HSV1-UL30-1649 | + | CCCGCGTTGCGCGGGCC | 17 | 4587 |
| HSV1-UL30-1650 | + | GCGTTGCGCGGGCCCGG | 17 | 4588 |
| HSV1-UL30-1651 | + | GGGCCCGGCGGCGTAGT | 17 | 4589 |
| HSV1-UL30-1652 | + | CCCGGCGGCGTAGTAGG | 17 | 4590 |
| HSV1-UL30-1653 | + | CCGGCGGCGTAGTAGGC | 17 | 4591 |
| HSV1-UL30-1654 | + | CGGCGGCGTAGTAGGCG | 17 | 4592 |
| HSV1-UL30-1655 | + | GGATGTCGCGATAGCTC | 17 | 4593 |
| HSV1-UL30-1656 | + | CCTTCTTCTTGTCCTTC | 17 | 4594 |
| HSV1-UL30-1657 | + | CTTCTTGTCCTTCAGGA | 17 | 4595 |
| HSV1-UL30-1658 | + | GTCCTTCAGGACGGCTT | 17 | 4596 |
| HSV1-UL30-1659 | + | CAGGACGGCTTCGGCCA | 17 | 4597 |
| HSV1-UL30-1660 | + | CGAGAGCTTGATCTTGT | 17 | 4598 |
| HSV1-UL30-1661 | + | TTATCTTGCTGCGCTTC | 17 | 4599 |
| HSV1-UL30-1662 | + | TGCTGCGCTTCTGGAAG | 17 | 4600 |
| HSV1-UL30-1663 | + | GCTTCTGGAAGTGGCTC | 17 | 4601 |
| HSV1-UL30-1664 | + | ACACGCGAAACACGCCC | 17 | 4602 |
| HSV1-UL30-1665 | + | CGCCCCGGCCGTTCATG | 17 | 4603 |
| HSV1-UL30-1666 | + | TGCGGCCGTACCCGTCC | 17 | 4604 |
| HSV1-UL30-1667 | + | GCGGCCGTACCCGTCCA | 17 | 4605 |
| HSV1-UL30-1668 | + | CGGCCGTACCCGTCCAG | 17 | 4606 |
| HSV1-UL30-1669 | + | GGCCGTACCCGTCCAGG | 17 | 4607 |
| HSV1-UL30-1670 | + | GTAAATGTCCGTCAGCT | 17 | 4608 |
| HSV1-UL30-1671 | + | CAGCTTGGCCAGCAAGA | 17 | 4609 |
| HSV1-UL30-1672 | + | AGCTTGGCCAGCAAGAA | 17 | 4610 |
| HSV1-UL30-1673 | + | GTTGATGATGTTGTACC | 17 | 4611 |
| HSV1-UL30-1674 | + | GTACCCGGTCACGAACT | 17 | 4612 |
| HSV1-UL30-1675 | + | TACCCGGTCACGAACTC | 17 | 4613 |
| HSV1-UL30-1676 | + | ACCCGGTCACGAACTCG | 17 | 4614 |
| HSV1-UL30-1677 | + | GGCCGTACTGTTTCACA | 17 | 4615 |
| HSV1-UL30-1678 | + | GCCGTACTGTTTCACAA | 17 | 4616 |
| HSV1-UL30-1679 | + | TTTCACAAGGGTCATGA | 17 | 4617 |
| HSV1-UL30-1680 | + | GTCGAATTCCAGAACCA | 17 | 4618 |
| HSV1-UL30-1681 | + | TCGAATTCCAGAACCAC | 17 | 4619 |
| HSV1-UL30-1682 | + | TTCCAGAACCACGGGCG | 17 | 4620 |
| HSV1-UL30-1683 | + | TCCAGAACCACGGGCGT | 17 | 4621 |
| HSV1-UL30-1684 | + | GAACCACGGGCGTGGGC | 17 | 4622 |
| HSV1-UL30-1685 | + | GGGCGTGGGCAGGCCCC | 17 | 4623 |
| HSV1-UL30-1686 | + | TGGCCGCCAGCTCGTTC | 17 | 4624 |
| HSV1-UL30-1687 | + | CCGCCAGCTCGTTCAGG | 17 | 4625 |
| HSV1-UL30-1688 | + | CGCCAGCTCGTTCAGGT | 17 | 4626 |
| HSV1-UL30-1689 | + | CTCGTTCAGGTGGGATT | 17 | 4627 |
| HSV1-UL30-1690 | + | TCGTTCAGGTGGGATTC | 17 | 4628 |
| HSV1-UL30-1691 | + | CGTTCAGGTGGGATTCG | 17 | 4629 |
| HSV1-UL30-1692 | + | TCAGGTGGGATTCGGGG | 17 | 4630 |
| HSV1-UL30-1693 | + | GGATTCGGGGAGGTCGC | 17 | 4631 |
| HSV1-UL30-1694 | + | AGGAACCGAGCGAAAAC | 17 | 4632 |
| HSV1-UL30-1695 | + | AACCGAGCGAAAACAGG | 17 | 4633 |
| HSV1-UL30-1696 | + | ACAGGAGGACGTGCTCC | 17 | 4634 |
| HSV1-UL30-1697 | + | CAGGAGGACGTGCTCCA | 17 | 4635 |
| HSV1-UL30-1698 | + | GAGGACGTGCTCCAGGG | 17 | 4636 |
| HSV1-UL30-1699 | + | GACGTGCTCCAGGGCGG | 17 | 4637 |
| HSV1-UL30-1700 | + | GTGCTCCAGGGCGGTGG | 17 | 4638 |
| HSV1-UL30-1701 | + | CCAGGGCGGTGGTGGAC | 17 | 4639 |
| HSV1-UL30-1702 | + | CAGGTCGTAGAGCAGAC | 17 | 4640 |
| HSV1-UL30-1703 | + | AGAGCAGACAGGATATC | 17 | 4641 |
| HSV1-UL30-1704 | + | AGGATATCTGGATGACC | 17 | 4642 |
| HSV1-UL30-1705 | + | TGGATGACCAGGTCCTC | 17 | 4643 |
| HSV1-UL30-1706 | + | GGATGACCAGGTCCTCC | 17 | 4644 |
| HSV1-UL30-1707 | + | CAGGTCCTCCGGGTGCC | 17 | 4645 |
| HSV1-UL30-1708 | + | TCCGGGTGCCCGGCCAC | 17 | 4646 |
| HSV1-UL30-1709 | + | GTGCCCGGCCACCGGAA | 17 | 4647 |
| HSV1-UL30-1710 | + | CACATGAGCTTGTATGC | 17 | 4648 |
| HSV1-UL30-1711 | + | TGAGCTTGTATGCCGGT | 17 | 4649 |
| HSV1-UL30-1712 | + | GCTCATGCCCCCCTCGA | 17 | 4650 |
| HSV1-UL30-1713 | + | TGCCCCCCTCGATGGCC | 17 | 4651 |
| HSV1-UL30-1714 | + | GTTAAACTCGACGTCGC | 17 | 4652 |
| HSV1-UL30-1715 | + | GTCGCTGGATGTCCCGA | 17 | 4653 |
| HSV1-UL30-1716 | + | GATGTCCCGAAGGCCAT | 17 | 4654 |
| HSV1-UL30-1717 | + | ATGTCCCGAAGGCCATC | 17 | 4655 |
| HSV1-UL30-1718 | + | TGTCCCGAAGGCCATCG | 17 | 4656 |
| HSV1-UL30-1719 | + | AAGGCCATCGGGGCCGC | 17 | 4657 |
| HSV1-UL30-1720 | + | CCATCGGGGCCGCCGGC | 17 | 4658 |
| HSV1-UL30-1721 | + | CATCGGGGCCGCCGGCT | 17 | 4659 |
| HSV1-UL30-1722 | + | GGGCTAGCGTGTTGTTC | 17 | 4660 |
| HSV1-UL30-1723 | + | AGCGTGTTGTTCCGGCC | 17 | 4661 |
| HSV1-UL30-1724 | + | TCCGGCCCGGTTTGAGA | 17 | 4662 |
| HSV1-UL30-1725 | + | GAGACGGTACCAGCCGA | 17 | 4663 |
| HSV1-UL30-1726 | + | GCCGAAGGTGACGAACC | 17 | 4664 |
| HSV1-UL30-1727 | + | CCGAAGGTGACGAACCC | 17 | 4665 |
| HSV1-UL30-1728 | + | CGAAGGTGACGAACCCG | 17 | 4666 |
| HSV1-UL30-1729 | + | CGAACCCGGGGTTGTCC | 17 | 4667 |
| HSV1-UL30-1730 | + | GGTTGTCCAGGATGAAC | 17 | 4668 |
| HSV1-UL30-1731 | + | GTTGTCCAGGATGAACC | 17 | 4669 |
| HSV1-UL30-1732 | + | GTCCAGGATGAACCGGG | 17 | 4670 |
| HSV1-UL30-1733 | + | CAGGATGAACCGGGTGG | 17 | 4671 |
| HSV1-UL30-1734 | + | ACCCTCGTACTTCTTGA | 17 | 4672 |
| HSV1-UL30-1735 | + | TCGTACTTCTTGATGGC | 17 | 4673 |
| HSV1-UL30-1736 | + | CGTACTTCTTGATGGCC | 17 | 4674 |
| HSV1-UL30-1737 | + | GGCAGAAGTTGTCGCAC | 17 | 4675 |
| HSV1-UL30-1738 | + | ACAGCACACGCCCGCTT | 17 | 4676 |
| HSV1-UL30-1739 | + | CGCTTCGGACGTAGACG | 17 | 4677 |
| HSV1-UL30-1740 | + | GACGCGGTAAAACAGAG | 17 | 4678 |
| HSV1-UL30-1741 | + | ACGCGGTAAAACAGAGC | 17 | 4679 |
| HSV1-UL30-1742 | + | CGCGGTAAAACAGAGCG | 17 | 4680 |
| HSV1-UL30-1743 | + | CTCGTAGTAGTACACGT | 17 | 4681 |
| HSV1-UL30-1744 | + | CCACCTCCGCCTCGAAG | 17 | 4682 |
| HSV1-UL30-1745 | + | CGCCTCGAAGTGGTCCG | 17 | 4683 |
| HSV1-UL30-1746 | + | GGTCCGCGGAGATGCCG | 17 | 4684 |
| HSV1-UL30-1747 | + | CCGCGGAACGACGCGCC | 17 | 4685 |
| HSV1-UL30-1748 | + | CGCGGAACGACGCGCCC | 17 | 4686 |
| HSV1-UL30-1749 | + | GCGGAACGACGCGCCCG | 17 | 4687 |
| HSV1-UL30-1750 | + | CGCCCGGGGACTCGCGC | 17 | 4688 |
| HSV1-UL30-1751 | + | GCCCGGGGACTCGCGCA | 17 | 4689 |
| HSV1-UL30-1752 | + | GGACTCGCGCAGGGCCG | 17 | 4690 |
| HSV1-UL30-1753 | + | CGCTCGCAGAGATCTCG | 17 | 4691 |
| HSV1-UL30-1754 | + | GCTCGCAGAGATCTCGT | 17 | 4692 |
| HSV1-UL30-1755 | + | CTCGCAGAGATCTCGTG | 17 | 4693 |
| HSV1-UL30-1756 | + | AGAGATCTCGTGGGGCG | 17 | 4694 |
| HSV1-UL30-1757 | + | GTGGGGCGCGGCATTGT | 17 | 4695 |
| HSV1-UL30-1758 | + | CGTGCCGTAAACGTGAA | 17 | 4696 |
| HSV1-UL30-1759 | + | AAACGTGAACGGCCACC | 17 | 4697 |
| HSV1-UL30-1760 | + | CGTGAACGGCCACCCGG | 17 | 4698 |
| HSV1-UL30-1761 | + | GCCACCCGGTGGCCTTC | 17 | 4699 |
| HSV1-UL30-1762 | + | GGTGGCCTTCCGGAGTC | 17 | 4700 |
| HSV1-UL30-1763 | + | CTTCCGGAGTCAGGCCC | 17 | 4701 |
| HSV1-UL30-1764 | + | GCCCAGGAGCGTGATGA | 17 | 4702 |
| HSV1-UL30-1765 | + | GTGATGACGGTCCCCGT | 17 | 4703 |
| HSV1-UL30-1766 | + | GGTCCCCGTCGGTGTGA | 17 | 4704 |
| HSV1-UL30-1767 | + | CGTCCATAAACCGCGCG | 17 | 4705 |
| HSV1-UL30-1768 | + | GTCCATAAACCGCGCGT | 17 | 4706 |
| HSV1-UL30-1769 | + | TCCATAAACCGCGCGTG | 17 | 4707 |
| HSV1-UL30-1770 | + | CCATAAACCGCGCGTGG | 17 | 4708 |

**Table 2D** provides exemplary targeting domains for knocking out the *UL30* gene selected according to the first tier parameters. The targeting domains are selected based on location within first 500bp of the coding sequence of the *UL30* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2D**

| 1st Tier | | | | |
|---|---|---|---|---|
| gRNA Name | DNA Strand | Targeting Domain | Target Site Length | Seq ID |
| HSV1-UL30-237 | - | UGGCGGCGGCCCGCUGUCCC | 20 | 4709 |
| HSV1-UL30-1 | - | GGCGGCGGCCCGCUGUCCCC | 20 | 3232 |
| HSV1-UL30-239 | - | CGGCGGCCCGCUGUCCCCCG | 20 | 4710 |
| HSV1-UL30-2 | - | GGCGGCCCGCUGUCCCCCGG | 20 | 3233 |
| HSV1-UL30-241 | - | CCCGGAGGAAAGUCGGCGGC | 20 | 4711 |
| HSV1-UL30-242 | - | GUCGGCGGCCAGGGCGGCGU | 20 | 4712 |
| HSV1-UL30-243 | - | UUUUGCGCCCGCCGGCCCUC | 20 | 4713 |
| HSV1-UL30-11 | - | UUUGCGCCCGCCGGCCCUCG | 20 | 3125 |
| HSV1-UL30-245 | - | GCCGGCCCUCGCGGAGCCAG | 20 | 4714 |
| HSV1-UL30-12 | - | CCGGCCCUCGCGGAGCCAGC | 20 | 3240 |
| HSV1-UL30-13 | - | CGGCCCUCGCGGAGCCAGCC | 20 | 3241 |
| HSV1-UL30-248 | - | GCCGGGGACCCCCGCCUUGU | 20 | 4715 |
| HSV1-UL30-249 | - | CAACCCCUACCUCGCCCCAG | 20 | 4716 |
| HSV1-UL30-16 | - | AACCCCUACCUCGCCCCAGU | 20 | 3244 |
| HSV1-UL30-251 | - | UCGCCCCAGUCGGGACGCAA | 20 | 4717 |
| HSV1-UL30-252 | - | CGGGACGCAACAGAAGCCGA | 20 | 4718 |
| HSV1-UL30-253 | - | CCAGCGCCAUACGUACUAUA | 20 | 4719 |
| HSV1-UL30-254 | - | UACGUACUAUAGCGAAUGCG | 20 | 4720 |
| HSV1-UL30-255 | - | GAAUUUCGAUUCAUCGCCCC | 20 | 4721 |
| HSV1-UL30-256 | - | GAUUCAUCGCCCCGCGGGUG | 20 | 4722 |
| HSV1-UL30-257 | - | CAUCGCCCCGCGGGUGCUGG | 20 | 4723 |
| HSV1-UL30-258 | - | UCGCCCCGCGGGUGCUGGAC | 20 | 4724 |
| HSV1-UL30-259 | - | UGCUGGACGAGGAUGCCCCC | 20 | 4725 |
| HSV1-UL30-24 | - | GCUGGACGAGGAUGCCCCCC | 20 | 3247 |
| HSV1-UL30-261 | - | UGGACGAGGAUGCCCCCCCG | 20 | 4726 |
| HSV1-UL30-262 | - | UGCCCCCCCGGAGAAGCGCG | 20 | 4727 |
| HSV1-UL30-25 | - | GCCCCCCCGGAGAAGCGCGC | 20 | 3131 |
| HSV1-UL30-264 | - | GCGCGCCCCCAAGGUGUACU | 20 | 4728 |
| HSV1-UL30-30 | - | CGCGCCCCCAAGGUGUACUG | 20 | 3135 |
| HSV1-UL30-31 | - | GCGCCCCCAAGGUGUACUGC | 20 | 3136 |
| HSV1-UL30-32 | - | CGCCCCCAAGGUGUACUGCG | 20 | 3137 |
| HSV1-UL30-33 | - | GCCCCCAAGGUGUACUGCGG | 20 | 3138 |
| HSV1-UL30-34 | - | CCCCCAAGGUGUACUGCGGG | 20 | 3249 |
| HSV1-UL30-270 | - | CAAGGUGUACUGCGGGGGGG | 20 | 4729 |
| HSV1-UL30-271 | - | CGAGCGCGACGUCCUCCGCG | 20 | 4730 |
| HSV1-UL30-272 | - | GCGACGUCCUCCGCGUCGGG | 20 | 4731 |
| HSV1-UL30-273 | - | UGGCCGCGGCGCUCGCGCCU | 20 | 4732 |
| HSV1-UL30-43 | - | GGCCGCGGCGCUCGCGCCUG | 20 | 3253 |
| HSV1-UL30-275 | - | GCUCGCGCCUGUGGGGCGGC | 20 | 4733 |
| HSV1-UL30-276 | - | GCGGCGUGGACCACGCCCCG | 20 | 4734 |
| HSV1-UL30-49 | - | CGGCGUGGACCACGCCCCGG | 20 | 3259 |
| HSV1-UL30-278 | - | UCUUUCACGUGUACGACAUC | 20 | 4735 |
| HSV1-UL30-52 | - | CUUUCACGUGUACGACAUCC | 20 | 3146 |
| HSV1-UL30-280 | - | UUCACGUGUACGACAUCCUG | 20 | 4736 |
| HSV1-UL30-281 | - | UGUACGACAUCCUGGAGAAC | 20 | 4737 |
| HSV1-UL30-53 | - | GUACGACAUCCUGGAGAACG | 20 | 3147 |
| HSV1-UL30-283 | - | CGGCGGCCCGCUGUCCC | 17 | 4738 |
| HSV1-UL30-119 | - | GGCGGCCCGCUGUCCCC | 17 | 3295 |
| HSV1-UL30-285 | - | CGGCCCGCUGUCCCCCG | 17 | 4739 |
| HSV1-UL30-120 | - | GGCCCGCUGUCCCCCGG | 17 | 3296 |
| HSV1-UL30-287 | - | GGAGGAAAGUCGGCGGC | 17 | 4740 |
| HSV1-UL30-288 | - | GGCGGCCAGGGCGGCGU | 17 | 4741 |
| HSV1-UL30-289 | - | UGCGCCCGCCGGCCCUC | 17 | 4742 |
| HSV1-UL30-129 | - | GCGCCCGCCGGCCCUCG | 17 | 3302 |
| HSV1-UL30-291 | - | GGCCCUCGCGGAGCCAG | 17 | 4743 |
| HSV1-UL30-130 | - | GCCCUCGCGGAGCCAGC | 17 | 3303 |
| HSV1-UL30-131 | - | CCCUCGCGGAGCCAGCC | 17 | 3304 |
| HSV1-UL30-294 | - | GGGGACCCCCGCCUUGU | 17 | 4744 |
| HSV1-UL30-295 | - | CCCCUACCUCGCCCCAG | 17 | 4745 |
| HSV1-UL30-134 | - | CCCUACCUCGCCCCAGU | 17 | 3307 |
| HSV1-UL30-297 | - | CCCCAGUCGGGACGCAA | 17 | 4746 |
| HSV1-UL30-298 | - | GACGCAACAGAAGCCGA | 17 | 4747 |
| HSV1-UL30-299 | - | GCGCCAUACGUACUAUA | 17 | 4748 |
| HSV1-UL30-300 | - | GUACUAUAGCGAAUGCG | 17 | 4749 |
| HSV1-UL30-301 | - | UUUCGAUUCAUCGCCCC | 17 | 4750 |
| HSV1-UL30-302 | - | UCAUCGCCCCGCGGGUG | 17 | 4751 |
| HSV1-UL30-303 | - | CGCCCCGCGGGUGCUGG | 17 | 4752 |
| HSV1-UL30-304 | - | CCCCGCGGGUGCUGGAC | 17 | 4753 |
| HSV1-UL30-305 | - | UGGACGAGGAUGCCCCC | 17 | 4754 |
| HSV1-UL30-142 | - | GGACGAGGAUGCCCCCC | 17 | 3310 |
| HSV1-UL30-307 | - | ACGAGGAUGCCCCCCCG | 17 | 4755 |
| HSV1-UL30-308 | - | CCCCCCGGAGAAGCGCG | 17 | 4756 |
| HSV1-UL30-143 | - | CCCCCGGAGAAGCGCGC | 17 | 3311 |
| HSV1-UL30-310 | - | CGCCCCCAAGGUGUACU | 17 | 4757 |
| HSV1-UL30-148 | - | GCCCCCAAGGUGUACUG | 17 | 3189 |
| HSV1-UL30-149 | - | CCCCCAAGGUGUACUGC | 17 | 3313 |
| HSV1-UL30-150 | - | CCCCAAGGUGUACUGCG | 17 | 3190 |
| HSV1-UL30-151 | - | CCCAAGGUGUACUGCGG | 17 | 3314 |
| HSV1-UL30-152 | - | CCAAGGUGUACUGCGGG | 17 | 3315 |
| HSV1-UL30-316 | - | GGUGUACUGCGGGGGGG | 17 | 4758 |
| HSV1-UL30-317 | - | GCGCGACGUCCUCCGCG | 17 | 4759 |
| HSV1-UL30-318 | - | ACGUCCUCCGCGUCGGG | 17 | 4760 |
| HSV1-UL30-319 | - | CCGCGGCGCUCGCGCCU | 17 | 4761 |
| HSV1-UL30-161 | - | CGCGGCGCUCGCGCCUG | 17 | 3320 |
| HSV1-UL30-321 | - | CGCGCCUGUGGGGCGGC | 17 | 4762 |
| HSV1-UL30-322 | - | GCGUGGACCACGCCCCG | 17 | 4763 |
| HSV1-UL30-167 | - | CGUGGACCACGCCCCGG | 17 | 3197 |
| HSV1-UL30-324 | - | UUCACGUGUACGACAUC | 17 | 4764 |
| HSV1-UL30-170 | - | UCACGUGUACGACAUCC | 17 | 3200 |
| HSV1-UL30-326 | - | ACGUGUACGACAUCCUG | 17 | 4765 |
| HSV1-UL30-327 | - | ACGACAUCCUGGAGAAC | 17 | 4766 |
| HSV1-UL30-171 | - | CGACAUCCUGGAGAACG | 17 | 3201 |
| HSV1-UL30-329 | + | UACGCGUGCUCCACGUUCUC | 20 | 4767 |
| HSV1-UL30-330 | + | UUCUCCAGGAUGUCGUACAC | 20 | 4768 |
| HSV1-UL30-331 | + | ACACGUGAAAGACGGUGACG | 20 | 4769 |
| HSV1-UL30-59 | + | CACGUGAAAGACGGUGACGG | 20 | 3262 |
| HSV1-UL30-333 | + | GAAAGACGGUGACGGUGGGG | 20 | 4770 |
| HSV1-UL30-334 | + | GACGGUGGGGUUGAACCCCG | 20 | 4771 |
| HSV1-UL30-62 | + | ACGGUGGGGUUGAACCCCGC | 20 | 3152 |
| HSV1-UL30-336 | + | GUCCACGCCGCCCCACAGGC | 20 | 4772 |
| HSV1-UL30-337 | + | ACAGGCGCGAGCGCCGCGGC | 20 | 4773 |
| HSV1-UL30-338 | + | CAGAAGCCGCCCGACCCGAC | 20 | 4774 |
| HSV1-UL30-68 | + | AGAAGCCGCCCGACCCGACG | 20 | 3155 |
| HSV1-UL30-340 | + | AAGCCGCCCGACCCGACGCG | 20 | 4775 |
| HSV1-UL30-341 | + | CGUCCCCCCCGCAGUACACC | 20 | 4776 |
| HSV1-UL30-70 | + | GUCCCCCCCGCAGUACACCU | 20 | 3157 |
| HSV1-UL30-71 | + | UCCCCCCCGCAGUACACCUU | 20 | 3267 |
| HSV1-UL30-344 | + | AGUACACCUUGGGGGCGCGC | 20 | 4777 |
| HSV1-UL30-345 | + | GUGCACCCCGGCGCGCUUCU | 20 | 4778 |
| HSV1-UL30-76 | + | UGCACCCCGGCGCGCUUCUC | 20 | 3270 |
| HSV1-UL30-77 | + | GCACCCCGGCGCGCUUCUCC | 20 | 3271 |
| HSV1-UL30-78 | + | CACCCCGGCGCGCUUCUCCG | 20 | 3272 |
| HSV1-UL30-79 | + | ACCCCGGCGCGCUUCUCCGG | 20 | 3273 |
| HSV1-UL30-350 | + | GGCAUCCUCGUCCAGCACCC | 20 | 4779 |
| HSV1-UL30-82 | + | GCAUCCUCGUCCAGCACCCG | 20 | 3161 |
| HSV1-UL30-352 | + | CGUCCAGCACCCGCGGGGCG | 20 | 4780 |
| HSV1-UL30-353 | + | AGCACCCGCGGGGCGAUGAA | 20 | 4781 |
| HSV1-UL30-354 | + | UCGCUAUAGUACGUAUGGCG | 20 | 4782 |
| HSV1-UL30-355 | + | CGGCUUCUGUUGCGUCCCGA | 20 | 4783 |
| HSV1-UL30-91 | + | GGCUUCUGUUGCGUCCCGAC | 20 | 3166 |
| HSV1-UL30-357 | + | UCUGUUGCGUCCCGACUGGG | 20 | 4784 |
| HSV1-UL30-358 | + | GCGUCCCGACUGGGGCGAGG | 20 | 4785 |
| HSV1-UL30-95 | + | CGUCCCGACUGGGGCGAGGU | 20 | 3170 |
| HSV1-UL30-360 | + | AAAGUUUUGCCUCAAACAAG | 20 | 4786 |
| HSV1-UL30-99 | + | AAGUUUUGCCUCAAACAAGG | 20 | 3282 |
| HSV1-UL30-100 | + | AGUUUUGCCUCAAACAAGGC | 20 | 3283 |
| HSV1-UL30-363 | + | GGGGGUCCCCGGCUGGCUCC | 20 | 4787 |
| HSV1-UL30-364 | + | GGGUCCCCGGCUGGCUCCGC | 20 | 4788 |
| HSV1-UL30-365 | + | GGCUGGCUCCGCGAGGGCCG | 20 | 4789 |
| HSV1-UL30-366 | + | GGCCGGCGGGCGCAAAAAAC | 20 | 4790 |
| HSV1-UL30-367 | + | CCCUGGCCGCCGACUUUCCU | 20 | 4791 |
| HSV1-UL30-112 | + | CCUGGCCGCCGACUUUCCUC | 20 | 3290 |
| HSV1-UL30-113 | + | CUGGCCGCCGACUUUCCUCC | 20 | 3291 |
| HSV1-UL30-114 | + | UGGCCGCCGACUUUCCUCCG | 20 | 3176 |
| HSV1-UL30-371 | + | CGACUUUCCUCCGGGGGACA | 20 | 4792 |
| HSV1-UL30-372 | + | GGGACAGCGGGCCGCCGCCA | 20 | 4793 |
| HSV1-UL30-118 | + | GGACAGCGGGCCGCCGCCAC | 20 | 3294 |
| HSV1-UL30-374 | + | GCGUGCUCCACGUUCUC | 17 | 4794 |
| HSV1-UL30-375 | + | UCCAGGAUGUCGUACAC | 17 | 4795 |
| HSV1-UL30-376 | + | CGUGAAAGACGGUGACG | 17 | 4796 |
| HSV1-UL30-177 | + | GUGAAAGACGGUGACGG | 17 | 3327 |
| HSV1-UL30-378 | + | AGACGGUGACGGUGGGG | 17 | 4797 |
| HSV1-UL30-379 | + | GGUGGGGUUGAACCCCG | 17 | 4798 |
| HSV1-UL30-180 | + | GUGGGGUUGAACCCCGC | 17 | 3329 |
| HSV1-UL30-381 | + | CACGCCGCCCCACAGGC | 17 | 4799 |
| HSV1-UL30-382 | + | GGCGCGAGCGCCGCGGC | 17 | 4800 |
| HSV1-UL30-383 | + | AAGCCGCCCGACCCGAC | 17 | 4801 |
| HSV1-UL30-186 | + | AGCCGCCCGACCCGACG | 17 | 3207 |
| HSV1-UL30-385 | + | CCGCCCGACCCGACGCG | 17 | 4802 |
| HSV1-UL30-386 | + | CCCCCCCGCAGUACACC | 17 | 4803 |
| HSV1-UL30-188 | + | CCCCCCGCAGUACACCU | 17 | 3209 |
| HSV1-UL30-189 | + | CCCCCGCAGUACACCUU | 17 | 3210 |
| HSV1-UL30-389 | + | ACACCUUGGGGGCGCGC | 17 | 4804 |
| HSV1-UL30-390 | + | CACCCCGGCGCGCUUCU | 17 | 4805 |
| HSV1-UL30-194 | + | ACCCCGGCGCGCUUCUC | 17 | 3335 |
| HSV1-UL30-195 | + | CCCCGGCGCGCUUCUCC | 17 | 3336 |
| HSV1-UL30-196 | + | CCCGGCGCGCUUCUCCG | 17 | 3213 |
| HSV1-UL30-197 | + | CCGGCGCGCUUCUCCGG | 17 | 3214 |
| HSV1-UL30-395 | + | AUCCUCGUCCAGCACCC | 17 | 4806 |
| HSV1-UL30-200 | + | UCCUCGUCCAGCACCCG | 17 | 3337 |
| HSV1-UL30-397 | + | CCAGCACCCGCGGGGCG | 17 | 4807 |
| HSV1-UL30-398 | + | ACCCGCGGGGCGAUGAA | 17 | 4808 |
| HSV1-UL30-399 | + | CUAUAGUACGUAUGGCG | 17 | 4809 |
| HSV1-UL30-400 | + | CUUCUGUUGCGUCCCGA | 17 | 4810 |
| HSV1-UL30-209 | + | UUCUGUUGCGUCCCGAC | 17 | 3222 |
| HSV1-UL30-402 | + | GUUGCGUCCCGACUGGG | 17 | 4811 |
| HSV1-UL30-403 | + | UCCCGACUGGGGCGAGG | 17 | 4812 |
| HSV1-UL30-213 | + | CCCGACUGGGGCGAGGU | 17 | 3341 |
| HSV1-UL30-405 | + | GUUUUGCCUCAAACAAG | 17 | 4813 |
| HSV1-UL30-217 | + | UUUUGCCUCAAACAAGG | 17 | 3344 |
| HSV1-UL30-218 | + | UUUGCCUCAAACAAGGC | 17 | 3345 |
| HSV1-UL30-408 | + | GGUCCCCGGCUGGCUCC | 17 | 4814 |
| HSV1-UL30-409 | + | UCCCCGGCUGGCUCCGC | 17 | 4815 |
| HSV1-UL30-410 | + | UGGCUCCGCGAGGGCCG | 17 | 4816 |
| HSV1-UL30-411 | + | CGGCGGGCGCAAAAAAC | 17 | 4817 |
| HSV1-UL30-412 | + | UGGCCGCCGACUUUCCU | 17 | 4818 |
| HSV1-UL30-230 | + | GGCCGCCGACUUUCCUC | 17 | 3228 |
| HSV1-UL30-231 | + | GCCGCCGACUUUCCUCC | 17 | 3229 |
| HSV1-UL30-232 | + | CCGCCGACUUUCCUCCG | 17 | 3230 |
| HSV1-UL30-416 | + | CUUUCCUCCGGGGGACA | 17 | 4819 |
| HSV1-UL30-417 | + | ACAGCGGGCCGCCGCCA | 17 | 4820 |
| HSV1-UL30-236 | + | CAGCGGGCCGCCGCCAC | 17 | 3358 |

**Table 2E** provides exemplary targeting domains for knocking out the *UL30* gene selected according to the second tier parameters. The targeting domains are selected based on location within the coding sequence (but downstream of the first 500bp) of the *UL30* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2E**

| 2nd Tier | | | | |
|---|---|---|---|---|
| gRNA Name | DNA Strand | Targeting Domain | Target Site Length | Seq ID |
| HSV1-UL30-1771 | - | UUAUGGACGCCAUCACACCG | 20 | 4821 |
| HSV1-UL30-1772 | - | UAUGGACGCCAUCACACCGA | 20 | 4822 |
| HSV1-UL30-1773 | - | AUGGACGCCAUCACACCGAC | 20 | 4823 |
| HSV1-UL30-1774 | - | CGGGGACCGUCAUCACGCUC | 20 | 4824 |
| HSV1-UL30-1775 | - | UCACGCUCCUGGGCCUGACU | 20 | 4825 |
| HSV1-UL30-1776 | - | CACGCUCCUGGGCCUGACUC | 20 | 4826 |
| HSV1-UL30-1777 | - | GGCCUGACUCCGGAAGGCCA | 20 | 4827 |
| HSV1-UL30-1778 | - | GCACGCGGCAGUACUUUUAC | 20 | 4828 |
| HSV1-UL30-1779 | - | GGCAGUACUUUUACAUGAAC | 20 | 4829 |
| HSV1-UL30-1780 | - | GCAGUACUUUUACAUGAACA | 20 | 4830 |
| HSV1-UL30-1781 | - | AGUACUUUUACAUGAACAAG | 20 | 4831 |
| HSV1-UL30-1782 | - | GUACUUUUACAUGAACAAGG | 20 | 4832 |
| HSV1-UL30-1783 | - | CACCUACAAUGCCGCGCCCC | 20 | 4833 |
| HSV1-UL30-1784 | - | CCGCGCCCCACGAGAUCUCU | 20 | 4834 |
| HSV1-UL30-1785 | - | GCGCAUGGCCGCGGCCCUGC | 20 | 4835 |
| HSV1-UL30-1786 | - | CCGCGGCCCUGCGCGAGUCC | 20 | 4836 |
| HSV1-UL30-1787 | - | CGUCGUUCCGCGGCAUCUCC | 20 | 4837 |
| HSV1-UL30-1788 | - | CGGCAUCUCCGCGGACCACU | 20 | 4838 |
| HSV1-UL30-1789 | - | UCUCCGCGGACCACUUCGAG | 20 | 4839 |
| HSV1-UL30-1790 | - | CUCCGCGGACCACUUCGAGG | 20 | 4840 |
| HSV1-UL30-1791 | - | ACCACUUCGAGGCGGAGGUG | 20 | 4841 |
| HSV1-UL30-1792 | - | CCACUUCGAGGCGGAGGUGG | 20 | 4842 |
| HSV1-UL30-1793 | - | GCGCACCGACGUGUACUACU | 20 | 4843 |
| HSV1-UL30-1794 | - | CUGUUUUACCGCGUCUACGU | 20 | 4844 |
| HSV1-UL30-1795 | - | UUACCGCGUCUACGUCCGAA | 20 | 4845 |
| HSV1-UL30-1796 | - | ACAACUUCUGCCCGGCCAUC | 20 | 4846 |
| HSV1-UL30-1797 | - | CUGCCCGGCCAUCAAGAAGU | 20 | 4847 |
| HSV1-UL30-1798 | - | GCCCGGCCAUCAAGAAGUAC | 20 | 4848 |
| HSV1-UL30-1799 | - | GGCCAUCAAGAAGUACGAGG | 20 | 4849 |
| HSV1-UL30-1800 | - | GCCAUCAAGAAGUACGAGGG | 20 | 4850 |
| HSV1-UL30-1801 | - | ACGCCACCACCCGGUUCAUC | 20 | 4851 |
| HSV1-UL30-1802 | - | CCGGUUCAUCCUGGACAACC | 20 | 4852 |
| HSV1-UL30-1803 | - | UCGGCUGGUACCGUCUCAAA | 20 | 4853 |
| HSV1-UL30-1804 | - | UGGUACCGUCUCAAACCGGG | 20 | 4854 |
| HSV1-UL30-1805 | - | GGUACCGUCUCAAACCGGGC | 20 | 4855 |
| HSV1-UL30-1806 | - | GCCGGCGGCCCCGAUGGCCU | 20 | 4856 |
| HSV1-UL30-1807 | - | CCGGCGGCCCCGAUGGCCUU | 20 | 4857 |
| HSV1-UL30-1808 | - | CUUCGGGACAUCCAGCGACG | 20 | 4858 |
| HSV1-UL30-1809 | - | ACGUCGAGUUUAACUGUACG | 20 | 4859 |
| HSV1-UL30-1810 | - | UACGGCGGACAACCUGGCCA | 20 | 4860 |
| HSV1-UL30-1811 | - | CGGCGGACAACCUGGCCAUC | 20 | 4861 |
| HSV1-UL30-1812 | - | GGCGGACAACCUGGCCAUCG | 20 | 4862 |
| HSV1-UL30-1813 | - | GCGGACAACCUGGCCAUCGA | 20 | 4863 |
| HSV1-UL30-1814 | - | CGGACAACCUGGCCAUCGAG | 20 | 4864 |
| HSV1-UL30-1815 | - | ACCUGGCCAUCGAGGGGGGC | 20 | 4865 |
| HSV1-UL30-1816 | - | CAAGCUCAUGUGCUUCGAUA | 20 | 4866 |
| HSV1-UL30-1817 | - | GCUUCGAUAUCGAAUGCAAG | 20 | 4867 |
| HSV1-UL30-1818 | - | CUUCGAUAUCGAAUGCAAGG | 20 | 4868 |
| HSV1-UL30-1819 | - | UUCGAUAUCGAAUGCAAGGC | 20 | 4869 |
| HSV1-UL30-1820 | - | UCGAUAUCGAAUGCAAGGCG | 20 | 4870 |
| HSV1-UL30-1821 | - | CGAUAUCGAAUGCAAGGCGG | 20 | 4871 |
| HSV1-UL30-1822 | - | GAUAUCGAAUGCAAGGCGGG | 20 | 4872 |
| HSV1-UL30-1823 | - | AUAUCGAAUGCAAGGCGGGG | 20 | 4873 |
| HSV1-UL30-1824 | - | UAUCGAAUGCAAGGCGGGGG | 20 | 4874 |
| HSV1-UL30-1825 | - | UCGAAUGCAAGGCGGGGGGG | 20 | 4875 |
| HSV1-UL30-1826 | - | AUGCAAGGCGGGGGGGGAGG | 20 | 4876 |
| HSV1-UL30-1827 | - | CGAGCUGGCCUUUCCGGUGG | 20 | 4877 |
| HSV1-UL30-1828 | - | CCUUUCCGGUGGCCGGGCAC | 20 | 4878 |
| HSV1-UL30-1829 | - | CUUUCCGGUGGCCGGGCACC | 20 | 4879 |
| HSV1-UL30-1830 | - | UUCCGGUGGCCGGGCACCCG | 20 | 4880 |
| HSV1-UL30-1831 | - | ACGACCUGUCCACCACCGCC | 20 | 4881 |
| HSV1-UL30-1832 | - | CGACCUGUCCACCACCGCCC | 20 | 4882 |
| HSV1-UL30-1833 | - | GCUCGGUUCCUGCGACCUCC | 20 | 4883 |
| HSV1-UL30-1834 | - | GCGACCUCCCCGAAUCCCAC | 20 | 4884 |
| HSV1-UL30-1835 | - | CCUCCCCGAAUCCCACCUGA | 20 | 4885 |
| HSV1-UL30-1836 | - | CACCUGAACGAGCUGGCGGC | 20 | 4886 |
| HSV1-UL30-1837 | - | ACCUGAACGAGCUGGCGGCC | 20 | 4887 |
| HSV1-UL30-1838 | - | GCCUGCCCACGCCCGUGGUU | 20 | 4888 |
| HSV1-UL30-1839 | - | CCUGCCCACGCCCGUGGUUC | 20 | 4889 |
| HSV1-UL30-1840 | - | CGUGGUUCUGGAAUUCGACA | 20 | 4890 |
| HSV1-UL30-1841 | - | UCUGGAAUUCGACAGCGAAU | 20 | 4891 |
| HSV1-UL30-1842 | - | UGUUGGCCUUCAUGACCCUU | 20 | 4892 |
| HSV1-UL30-1843 | - | CCUUGUGAAACAGUACGGCC | 20 | 4893 |
| HSV1-UL30-1844 | - | GUACGGCCCCGAGUUCGUGA | 20 | 4894 |
| HSV1-UL30-1845 | - | CCUUCUUGCUGGCCAAGCUG | 20 | 4895 |
| HSV1-UL30-1846 | - | CGGACAUUUACAAGGUCCCC | 20 | 4896 |
| HSV1-UL30-1847 | - | CAUUUACAAGGUCCCCCUGG | 20 | 4897 |
| HSV1-UL30-1848 | - | CCCUGGACGGGUACGGCCGC | 20 | 4898 |
| HSV1-UL30-1849 | - | GGGUACGGCCGCAUGAACGG | 20 | 4899 |
| HSV1-UL30-1850 | - | GGUACGGCCGCAUGAACGGC | 20 | 4900 |
| HSV1-UL30-1851 | - | GGCCGGGGCGUGUUUCGCGU | 20 | 4901 |
| HSV1-UL30-1852 | - | GCCGGGGCGUGUUUCGCGUG | 20 | 4902 |
| HSV1-UL30-1853 | - | UUCGCGUGUGGGACAUAGGC | 20 | 4903 |
| HSV1-UL30-1854 | - | ACAUAGGCCAGAGCCACUUC | 20 | 4904 |
| HSV1-UL30-1855 | - | AGAAGCGCAGCAAGAUAAAG | 20 | 4905 |
| HSV1-UL30-1856 | - | AGAUAAAGGUGAACGGCAUG | 20 | 4906 |
| HSV1-UL30-1857 | - | CAUGGUGAACAUCGACAUGU | 20 | 4907 |
| HSV1-UL30-1858 | - | AUGGUGAACAUCGACAUGUA | 20 | 4908 |
| HSV1-UL30-1859 | - | UAACCGACAAGAUCAAGCUC | 20 | 4909 |
| HSV1-UL30-1860 | - | CUACAAGCUCAACGCCGUGG | 20 | 4910 |
| HSV1-UL30-1861 | - | ACGCCGUGGCCGAAGCCGUC | 20 | 4911 |
| HSV1-UL30-1862 | - | CCGUGGCCGAAGCCGUCCUG | 20 | 4912 |
| HSV1-UL30-1863 | - | CCGAAGCCGUCCUGAAGGAC | 20 | 4913 |
| HSV1-UL30-1864 | - | AAGCCGUCCUGAAGGACAAG | 20 | 4914 |
| HSV1-UL30-1865 | - | CCGUCCUGAAGGACAAGAAG | 20 | 4915 |
| HSV1-UL30-1866 | - | UGAAGGACAAGAAGAAGGAC | 20 | 4916 |
| HSV1-UL30-1867 | - | CAUCCCCGCCUACUACGCCG | 20 | 4917 |
| HSV1-UL30-1868 | - | CGCCGCCGGGCCCGCGCAAC | 20 | 4918 |
| HSV1-UL30-534 | - | GCCGCCGGGCCCGCGCAACG | 20 | 3472 |
| HSV1-UL30-1870 | - | CGCGCAACGCGGGGUGAUCG | 20 | 4919 |
| HSV1-UL30-1871 | - | UGAUCGGCGAGUACUGCAUA | 20 | 4920 |
| HSV1-UL30-1872 | - | GCAUACAGGAUUCCCUGCUG | 20 | 4921 |
| HSV1-UL30-1873 | - | UUUUUAAGUUUUUGCCCCAU | 20 | 4922 |
| HSV1-UL30-1874 | - | UUUUAAGUUUUUGCCCCAUC | 20 | 4923 |
| HSV1-UL30-1875 | - | UCUCGGCCGUCGCGCGCUUG | 20 | 4924 |
| HSV1-UL30-1876 | - | GCCUGCUGCGCCUGGCCGAC | 20 | 4925 |
| HSV1-UL30-1877 | - | UGCUGCGCCUGGCCGACCAG | 20 | 4926 |
| HSV1-UL30-1878 | - | ACCAGAAGGGCUUUAUUCUG | 20 | 4927 |
| HSV1-UL30-1879 | - | GCUUUAUUCUGCCGGACACC | 20 | 4928 |
| HSV1-UL30-1880 | - | CUUUAUUCUGCCGGACACCC | 20 | 4929 |
| HSV1-UL30-1881 | - | CCGGACACCCAGGGGCGAUU | 20 | 4930 |
| HSV1-UL30-1882 | - | CGGACACCCAGGGGCGAUUU | 20 | 4931 |
| HSV1-UL30-1883 | - | CCAGGGGCGAUUUAGGGGCG | 20 | 4932 |
| HSV1-UL30-1884 | - | CAGGGGCGAUUUAGGGGCGC | 20 | 4933 |
| HSV1-UL30-1885 | - | AGGGGCGAUUUAGGGGCGCC | 20 | 4934 |
| HSV1-UL30-1886 | - | GGGGCGAUUUAGGGGCGCCG | 20 | 4935 |
| HSV1-UL30-1887 | - | GGGCGAUUUAGGGGCGCCGG | 20 | 4936 |
| HSV1-UL30-1888 | - | GGCGAUUUAGGGGCGCCGGG | 20 | 4937 |
| HSV1-UL30-1889 | - | GCGAUUUAGGGGCGCCGGGG | 20 | 4938 |
| HSV1-UL30-1890 | - | CCCAAGCGUCCGGCCGCAGC | 20 | 4939 |
| HSV1-UL30-1891 | - | CCAAGCGUCCGGCCGCAGCC | 20 | 4940 |
| HSV1-UL30-1892 | - | CAAGCGUCCGGCCGCAGCCC | 20 | 4941 |
| HSV1-UL30-1893 | - | AGCGUCCGGCCGCAGCCCGG | 20 | 4942 |
| HSV1-UL30-1894 | - | UCCGGCCGCAGCCCGGGAGG | 20 | 4943 |
| HSV1-UL30-1895 | - | CGGCCGCAGCCCGGGAGGAC | 20 | 4944 |
| HSV1-UL30-569 | - | GGCCGCAGCCCGGGAGGACG | 20 | 3507 |
| HSV1-UL30-1897 | - | CCGGGAGGACGAGGAGCGGC | 20 | 4945 |
| HSV1-UL30-1898 | - | GGGAGGACGAGGAGCGGCCA | 20 | 4946 |
| HSV1-UL30-571 | - | GGAGGACGAGGAGCGGCCAG | 20 | 3509 |
| HSV1-UL30-1900 | - | AGGACGAGGAGCGGCCAGAG | 20 | 4947 |
| HSV1-UL30-572 | - | GGACGAGGAGCGGCCAGAGG | 20 | 3510 |
| HSV1-UL30-1902 | - | ACGAGGAGCGGCCAGAGGAG | 20 | 4948 |
| HSV1-UL30-573 | - | CGAGGAGCGGCCAGAGGAGG | 20 | 3511 |
| HSV1-UL30-574 | - | GAGGAGCGGCCAGAGGAGGA | 20 | 3512 |
| HSV1-UL30-575 | - | AGGAGCGGCCAGAGGAGGAG | 20 | 3513 |
| HSV1-UL30-576 | - | GGAGCGGCCAGAGGAGGAGG | 20 | 3514 |
| HSV1-UL30-1907 | - | AGCGGCCAGAGGAGGAGGGG | 20 | 4949 |
| HSV1-UL30-1908 | - | GCCAGAGGAGGAGGGGGAGG | 20 | 4950 |
| HSV1-UL30-1909 | - | CAGAGGAGGAGGGGGAGGAC | 20 | 4951 |
| HSV1-UL30-1910 | - | GGAGGAGGGGGAGGACGAGG | 20 | 4952 |
| HSV1-UL30-1911 | - | GGGGGAGGACGAGGACGAAC | 20 | 4953 |
| HSV1-UL30-1912 | - | GGGAGGACGAGGACGAACGC | 20 | 4954 |
| HSV1-UL30-579 | - | GGAGGACGAGGACGAACGCG | 20 | 3517 |
| HSV1-UL30-1914 | - | AGGACGAGGACGAACGCGAG | 20 | 4955 |
| HSV1-UL30-1915 | - | CGAGGACGAACGCGAGGAGG | 20 | 4956 |
| HSV1-UL30-582 | - | GAGGACGAACGCGAGGAGGG | 20 | 3520 |
| HSV1-UL30-583 | - | AGGACGAACGCGAGGAGGGC | 20 | 3521 |
| HSV1-UL30-1918 | - | CGAACGCGAGGAGGGCGGGG | 20 | 4957 |
| HSV1-UL30-1919 | - | CGCGAGGAGGGCGGGGGCGA | 20 | 4958 |
| HSV1-UL30-586 | - | GCGAGGAGGGCGGGGGCGAG | 20 | 3524 |
| HSV1-UL30-587 | - | CGAGGAGGGCGGGGGCGAGC | 20 | 3525 |
| HSV1-UL30-1922 | - | AGGGCGGGGGCGAGCGGGAG | 20 | 4959 |
| HSV1-UL30-588 | - | GGGCGGGGGCGAGCGGGAGC | 20 | 3526 |
| HSV1-UL30-1924 | - | GCGGGGGCGAGCGGGAGCCG | 20 | 4960 |
| HSV1-UL30-1925 | - | GAGCGGGAGCCGGAGGGCGC | 20 | 4961 |
| HSV1-UL30-591 | - | AGCGGGAGCCGGAGGGCGCG | 20 | 3529 |
| HSV1-UL30-592 | - | GCGGGAGCCGGAGGGCGCGC | 20 | 3530 |
| HSV1-UL30-1928 | - | GGGAGACCGCCGGCAGGCAC | 20 | 4962 |
| HSV1-UL30-595 | - | GGAGACCGCCGGCAGGCACG | 20 | 3533 |
| HSV1-UL30-1930 | - | CCGGCAGGCACGUGGGGUAC | 20 | 4963 |
| HSV1-UL30-1931 | - | CGGCAGGCACGUGGGGUACC | 20 | 4964 |
| HSV1-UL30-1932 | - | GGCAGGCACGUGGGGUACCA | 20 | 4965 |
| HSV1-UL30-1933 | - | CACGUGGGGUACCAGGGGGC | 20 | 4966 |
| HSV1-UL30-1934 | - | CAGGGUCCUUGACCCCACUU | 20 | 4967 |
| HSV1-UL30-1935 | - | ACCCCACUUCCGGGUUUCAC | 20 | 4968 |
| HSV1-UL30-1936 | - | UGUGCUUCAGCACGCUCUCC | 20 | 4969 |
| HSV1-UL30-1937 | - | UGCUUCAGCACGCUCUCCCU | 20 | 4970 |
| HSV1-UL30-1938 | - | GGGCCGACGCAGUGGCGCAC | 20 | 4971 |
| HSV1-UL30-1939 | - | GGCCGACGCAGUGGCGCACC | 20 | 4972 |
| HSV1-UL30-1940 | - | ACGCAGUGGCGCACCUGGAG | 20 | 4973 |
| HSV1-UL30-1941 | - | UGGCGCACCUGGAGGCGGGC | 20 | 4974 |
| HSV1-UL30-1942 | - | UGGAGGCGGGCAAGGACUAC | 20 | 4975 |
| HSV1-UL30-1943 | - | GGAGGCGGGCAAGGACUACC | 20 | 4976 |
| HSV1-UL30-1944 | - | GGGCAAGGACUACCUGGAGA | 20 | 4977 |
| HSV1-UL30-1945 | - | AGGACUACCUGGAGAUCGAG | 20 | 4978 |
| HSV1-UL30-1946 | - | GGACUACCUGGAGAUCGAGG | 20 | 4979 |
| HSV1-UL30-1947 | - | GACUACCUGGAGAUCGAGGU | 20 | 4980 |
| HSV1-UL30-1948 | - | ACUACCUGGAGAUCGAGGUG | 20 | 4981 |
| HSV1-UL30-1949 | - | CUACCUGGAGAUCGAGGUGG | 20 | 4982 |
| HSV1-UL30-1950 | - | UUCUUCGUCAAGGCUCACGU | 20 | 4983 |
| HSV1-UL30-1951 | - | CUUCGUCAAGGCUCACGUGC | 20 | 4984 |
| HSV1-UL30-1952 | - | UCGUCAAGGCUCACGUGCGA | 20 | 4985 |
| HSV1-UL30-1953 | - | AGCCUCCUCAGCAUCCUCCU | 20 | 4986 |
| HSV1-UL30-1954 | - | GCCUCCUCAGCAUCCUCCUG | 20 | 4987 |
| HSV1-UL30-1955 | - | CUGCGGGACUGGCUCGCCAU | 20 | 4988 |
| HSV1-UL30-1956 | - | AUGCGAAAGCAGAUCCGCUC | 20 | 4989 |
| HSV1-UL30-1957 | - | AGAUCCGCUCGCGGAUUCCC | 20 | 4990 |
| HSV1-UL30-1958 | - | GCGGAUUCCCCAGAGCAGCC | 20 | 4991 |
| HSV1-UL30-1959 | - | GGAUUCCCCAGAGCAGCCCC | 20 | 4992 |
| HSV1-UL30-1960 | - | GAUUCCCCAGAGCAGCCCCG | 20 | 4993 |
| HSV1-UL30-1961 | - | GCCCCGAGGAGGCCGUGCUC | 20 | 4994 |
| HSV1-UL30-1962 | - | GGUCGUGUGUAACUCGGUGU | 20 | 4995 |
| HSV1-UL30-1963 | - | GUAACUCGGUGUACGGGUUC | 20 | 4996 |
| HSV1-UL30-1964 | - | UAACUCGGUGUACGGGUUCA | 20 | 4997 |
| HSV1-UL30-1965 | - | AACUCGGUGUACGGGUUCAC | 20 | 4998 |
| HSV1-UL30-1966 | - | CGGGUUCACGGGAGUGCAGC | 20 | 4999 |
| HSV1-UL30-1967 | - | GACGGUGACGACCAUCGGCC | 20 | 5000 |
| HSV1-UL30-1968 | - | CGAGAUGCUGCUCGCGACCC | 20 | 5001 |
| HSV1-UL30-1969 | - | CGCGAGUACGUCCACGCGCG | 20 | 5002 |
| HSV1-UL30-1970 | - | CCACGCGCGCUGGGCGGCCU | 20 | 5003 |
| HSV1-UL30-1971 | - | AACAGCUCCUGGCCGAUUUC | 20 | 5004 |
| HSV1-UL30-1972 | - | ACAGCUCCUGGCCGAUUUCC | 20 | 5005 |
| HSV1-UL30-1973 | - | GGCGGCCGACAUGCGCGCCC | 20 | 5006 |
| HSV1-UL30-1974 | - | CUAUUCCAUGCGCAUCAUCU | 20 | 5007 |
| HSV1-UL30-1975 | - | UAUUCCAUGCGCAUCAUCUA | 20 | 5008 |
| HSV1-UL30-1976 | - | AUUCCAUGCGCAUCAUCUAC | 20 | 5009 |
| HSV1-UL30-1977 | - | UGCGCAUCAUCUACGGGGAC | 20 | 5010 |
| HSV1-UL30-1978 | - | GUGCCGCGGCCUCACGGCCG | 20 | 5011 |
| HSV1-UL30-1979 | - | CGGCCGCCGGGCUGACGGCC | 20 | 5012 |
| HSV1-UL30-1980 | - | CGGCCGUGGGCGACAAGAUG | 20 | 5013 |
| HSV1-UL30-1981 | - | GUUUCUGCCCCCCAUCAAAC | 20 | 5014 |
| HSV1-UL30-1982 | - | GCCCCCCAUCAAACUCGAGU | 20 | 5015 |
| HSV1-UL30-1983 | - | CCAAGCUGCUGCUGAUCGCC | 20 | 5016 |
| HSV1-UL30-1984 | - | AAAGUACAUCGGCGUCAUCU | 20 | 5017 |
| HSV1-UL30-1985 | - | AAGUACAUCGGCGUCAUCUA | 20 | 5018 |
| HSV1-UL30-1986 | - | AGUACAUCGGCGUCAUCUAC | 20 | 5019 |
| HSV1-UL30-1987 | - | ACGGGGGUAAGAUGCUCAUC | 20 | 5020 |
| HSV1-UL30-1988 | - | GUAAGAUGCUCAUCAAGGGC | 20 | 5021 |
| HSV1-UL30-1989 | - | GCGUUUAUCAACCGCACCUC | 20 | 5022 |
| HSV1-UL30-1990 | - | GUUUUACGACGAUACCGUCU | 20 | 5023 |
| HSV1-UL30-1991 | - | UUUUACGACGAUACCGUCUC | 20 | 5024 |
| HSV1-UL30-1992 | - | CGGAGCGGCCGCCGCGUUAG | 20 | 5025 |
| HSV1-UL30-1993 | - | CCGCGUUAGCCGAGCGCCCC | 20 | 5026 |
| HSV1-UL30-1994 | - | CGCGUUAGCCGAGCGCCCCG | 20 | 5027 |
| HSV1-UL30-1995 | - | CGUUAGCCGAGCGCCCCGCG | 20 | 5028 |
| HSV1-UL30-1996 | - | GUUAGCCGAGCGCCCCGCGG | 20 | 5029 |
| HSV1-UL30-1997 | - | GUGGCUGGCGCGACCCCUGC | 20 | 5030 |
| HSV1-UL30-1998 | - | GGCUGGCGCGACCCCUGCCC | 20 | 5031 |
| HSV1-UL30-1999 | - | GCUGGCGCGACCCCUGCCCG | 20 | 5032 |
| HSV1-UL30-2000 | - | GCCCGAGGGACUGCAGGCGU | 20 | 5033 |
| HSV1-UL30-2001 | - | CCCGAGGGACUGCAGGCGUU | 20 | 5034 |
| HSV1-UL30-2002 | - | CCCAUCGGCGCAUCACCGAC | 20 | 5035 |
| HSV1-UL30-2003 | - | CCAUCGGCGCAUCACCGACC | 20 | 5036 |
| HSV1-UL30-2004 | - | AUCGGCGCAUCACCGACCCG | 20 | 5037 |
| HSV1-UL30-2005 | - | CGGCGCAUCACCGACCCGGA | 20 | 5038 |
| HSV1-UL30-2006 | - | GGCGCAUCACCGACCCGGAG | 20 | 5039 |
| HSV1-UL30-2007 | - | CCGACCCGGAGAGGGACAUC | 20 | 5040 |
| HSV1-UL30-2008 | - | CCAGGACUUUGUCCUCACCG | 20 | 5041 |
| HSV1-UL30-2009 | - | ACUUUGUCCUCACCGCCGAA | 20 | 5042 |
| HSV1-UL30-2010 | - | GCGCGCAGGUCCCGUCCAUC | 20 | 5043 |
| HSV1-UL30-2011 | - | CAGGUCCCGUCCAUCAAGGA | 20 | 5044 |
| HSV1-UL30-2012 | - | CGUGAUCGUGGCCCAGACCC | 20 | 5045 |
| HSV1-UL30-2013 | - | CGUGGCCCAGACCCGCGAGG | 20 | 5046 |
| HSV1-UL30-2014 | - | UGGCCCAGACCCGCGAGGUA | 20 | 5047 |
| HSV1-UL30-2015 | - | GGCCCAGACCCGCGAGGUAG | 20 | 5048 |
| HSV1-UL30-2016 | - | CGCGCGGCUGGCCGCCCUCC | 20 | 5049 |
| HSV1-UL30-2017 | - | CGAGCUAGACGCCGCCGCCC | 20 | 5050 |
| HSV1-UL30-2018 | - | GAGCUAGACGCCGCCGCCCC | 20 | 5051 |
| HSV1-UL30-2019 | - | AGCUAGACGCCGCCGCCCCA | 20 | 5052 |
| HSV1-UL30-2020 | - | AGACGCCGCCGCCCCAGGGG | 20 | 5053 |
| HSV1-UL30-2021 | - | CCCUCCCCGGCCAAGCGCCC | 20 | 5054 |
| HSV1-UL30-2022 | - | CCUCCCCGGCCAAGCGCCCC | 20 | 5055 |
| HSV1-UL30-2023 | - | CUCCCCGGCCAAGCGCCCCC | 20 | 5056 |
| HSV1-UL30-2024 | - | CGCCGUCGCCUGCCGACCCC | 20 | 5057 |
| HSV1-UL30-2025 | - | GCCGUCGCCUGCCGACCCCC | 20 | 5058 |
| HSV1-UL30-2026 | - | CCGUCGCCUGCCGACCCCCC | 20 | 5059 |
| HSV1-UL30-2027 | - | GCCCCGCAAGCUGCUGGUGU | 20 | 5060 |
| HSV1-UL30-2028 | - | GCUGCUGGUGUCCGAGCUGG | 20 | 5061 |
| HSV1-UL30-2029 | - | UGCUGGUGUCCGAGCUGGCC | 20 | 5062 |
| HSV1-UL30-2030 | - | CCAUUGCCCACGGCGUCGCC | 20 | 5063 |
| HSV1-UL30-2031 | - | CCCACGGCGUCGCCCUGAAC | 20 | 5064 |
| HSV1-UL30-2032 | - | ACUAUUACUUCUCCCACCUG | 20 | 5065 |
| HSV1-UL30-2033 | - | CUAUUACUUCUCCCACCUGU | 20 | 5066 |
| HSV1-UL30-2034 | - | UAUUACUUCUCCCACCUGUU | 20 | 5067 |
| HSV1-UL30-2035 | - | CGUGACAUUCAAGGCCCUGU | 20 | 5068 |
| HSV1-UL30-2036 | - | GUGACAUUCAAGGCCCUGUU | 20 | 5069 |
| HSV1-UL30-2037 | - | UGACAUUCAAGGCCCUGUUU | 20 | 5070 |
| HSV1-UL30-2038 | - | UGGGAAUAACGCCAAGAUCA | 20 | 5071 |
| HSV1-UL30-2039 | - | GGAAUAACGCCAAGAUCACC | 20 | 5072 |
| HSV1-UL30-2040 | - | UCUGUUAAAAAGGUUUAUUC | 20 | 5073 |
| HSV1-UL30-2041 | - | UUCCCGAAGUGUGGCACCCC | 20 | 5074 |
| HSV1-UL30-2042 | - | GACGACGUGGCCGCGCGGCU | 20 | 5075 |
| HSV1-UL30-2043 | - | GGCCGCGCGGCUCCGGACCG | 20 | 5076 |
| HSV1-UL30-2044 | - | GCGGCUCCGGACCGCAGGGU | 20 | 5077 |
| HSV1-UL30-2045 | - | CGGCUCCGGACCGCAGGGUU | 20 | 5078 |
| HSV1-UL30-2046 | - | GGACCGCAGGGUUCGGGGCG | 20 | 5079 |
| HSV1-UL30-2047 | - | CGGUGGGUGCCGGCGCUACG | 20 | 5080 |
| HSV1-UL30-2048 | - | GGUGGGUGCCGGCGCUACGG | 20 | 5081 |
| HSV1-UL30-2049 | - | UGGGUGCCGGCGCUACGGCG | 20 | 5082 |
| HSV1-UL30-2050 | - | GGGUGCCGGCGCUACGGCGG | 20 | 5083 |
| HSV1-UL30-2051 | - | GCUACGGCGGAGGAAACUCG | 20 | 5084 |
| HSV1-UL30-2052 | - | GAAACUCGUCGAAUGUUGCA | 20 | 5085 |
| HSV1-UL30-2053 | - | UGGACGCCAUCACACCG | 17 | 5086 |
| HSV1-UL30-2054 | - | GGACGCCAUCACACCGA | 17 | 5087 |
| HSV1-UL30-2055 | - | GACGCCAUCACACCGAC | 17 | 5088 |
| HSV1-UL30-2056 | - | GGACCGUCAUCACGCUC | 17 | 5089 |
| HSV1-UL30-2057 | - | CGCUCCUGGGCCUGACU | 17 | 5090 |
| HSV1-UL30-2058 | - | GCUCCUGGGCCUGACUC | 17 | 5091 |
| HSV1-UL30-2059 | - | CUGACUCCGGAAGGCCA | 17 | 5092 |
| HSV1-UL30-2060 | - | CGCGGCAGUACUUUUAC | 17 | 5093 |
| HSV1-UL30-2061 | - | AGUACUUUUACAUGAAC | 17 | 5094 |
| HSV1-UL30-2062 | - | GUACUUUUACAUGAACA | 17 | 5095 |
| HSV1-UL30-2063 | - | ACUUUUACAUGAACAAG | 17 | 5096 |
| HSV1-UL30-2064 | - | CUUUUACAUGAACAAGG | 17 | 5097 |
| HSV1-UL30-2065 | - | CUACAAUGCCGCGCCCC | 17 | 5098 |
| HSV1-UL30-2066 | - | CGCCCCACGAGAUCUCU | 17 | 5099 |
| HSV1-UL30-2067 | - | CAUGGCCGCGGCCCUGC | 17 | 5100 |
| HSV1-UL30-2068 | - | CGGCCCUGCGCGAGUCC | 17 | 5101 |
| HSV1-UL30-2069 | - | CGUUCCGCGGCAUCUCC | 17 | 5102 |
| HSV1-UL30-2070 | - | CAUCUCCGCGGACCACU | 17 | 5103 |
| HSV1-UL30-2071 | - | CCGCGGACCACUUCGAG | 17 | 5104 |
| HSV1-UL30-2072 | - | CGCGGACCACUUCGAGG | 17 | 5105 |
| HSV1-UL30-2073 | - | ACUUCGAGGCGGAGGUG | 17 | 5106 |
| HSV1-UL30-2074 | - | CUUCGAGGCGGAGGUGG | 17 | 5107 |
| HSV1-UL30-2075 | - | CACCGACGUGUACUACU | 17 | 5108 |
| HSV1-UL30-2076 | - | UUUUACCGCGUCUACGU | 17 | 5109 |
| HSV1-UL30-2077 | - | CCGCGUCUACGUCCGAA | 17 | 5110 |
| HSV1-UL30-2078 | - | ACUUCUGCCCGGCCAUC | 17 | 5111 |
| HSV1-UL30-2079 | - | CCCGGCCAUCAAGAAGU | 17 | 5112 |
| HSV1-UL30-2080 | - | CGGCCAUCAAGAAGUAC | 17 | 5113 |
| HSV1-UL30-2081 | - | CAUCAAGAAGUACGAGG | 17 | 5114 |
| HSV1-UL30-2082 | - | AUCAAGAAGUACGAGGG | 17 | 5115 |
| HSV1-UL30-2083 | - | CCACCACCCGGUUCAUC | 17 | 5116 |
| HSV1-UL30-2084 | - | GUUCAUCCUGGACAACC | 17 | 5117 |
| HSV1-UL30-2085 | - | GCUGGUACCGUCUCAAA | 17 | 5118 |
| HSV1-UL30-2086 | - | UACCGUCUCAAACCGGG | 17 | 5119 |
| HSV1-UL30-2087 | - | ACCGUCUCAAACCGGGC | 17 | 5120 |
| HSV1-UL30-2088 | - | GGCGGCCCCGAUGGCCU | 17 | 5121 |
| HSV1-UL30-2089 | - | GCGGCCCCGAUGGCCUU | 17 | 5122 |
| HSV1-UL30-2090 | - | CGGGACAUCCAGCGACG | 17 | 5123 |
| HSV1-UL30-2091 | - | UCGAGUUUAACUGUACG | 17 | 5124 |
| HSV1-UL30-2092 | - | GGCGGACAACCUGGCCA | 17 | 5125 |
| HSV1-UL30-2093 | - | CGGACAACCUGGCCAUC | 17 | 5126 |
| HSV1-UL30-2094 | - | GGACAACCUGGCCAUCG | 17 | 5127 |
| HSV1-UL30-2095 | - | GACAACCUGGCCAUCGA | 17 | 5128 |
| HSV1-UL30-2096 | - | ACAACCUGGCCAUCGAG | 17 | 5129 |
| HSV1-UL30-2097 | - | UGGCCAUCGAGGGGGGC | 17 | 5130 |
| HSV1-UL30-2098 | - | GCUCAUGUGCUUCGAUA | 17 | 5131 |
| HSV1-UL30-2099 | - | UCGAUAUCGAAUGCAAG | 17 | 5132 |
| HSV1-UL30-2100 | - | CGAUAUCGAAUGCAAGG | 17 | 5133 |
| HSV1-UL30-2101 | - | GAUAUCGAAUGCAAGGC | 17 | 5134 |
| HSV1-UL30-2102 | - | AUAUCGAAUGCAAGGCG | 17 | 5135 |
| HSV1-UL30-2103 | - | UAUCGAAUGCAAGGCGG | 17 | 5136 |
| HSV1-UL30-2104 | - | AUCGAAUGCAAGGCGGG | 17 | 5137 |
| HSV1-UL30-2105 | - | UCGAAUGCAAGGCGGGG | 17 | 5138 |
| \HSV1-UL30-2106 | - | CGAAUGCAAGGCGGGGG | 17 | 5139 |
| HSV1-UL30-2107 | - | AAUGCAAGGCGGGGGGG | 17 | 5140 |
| HSV1-UL30-2108 | - | CAAGGCGGGGGGGGAGG | 17 | 5141 |
| HSV1-UL30-2109 | - | GCUGGCCUUUCCGGUGG | 17 | 5142 |
| HSV1-UL30-2110 | - | UUCCGGUGGCCGGGCAC | 17 | 5143 |
| HSV1-UL30-2111 | - | UCCGGUGGCCGGGCACC | 17 | 5144 |
| HSV1-UL30-2112 | - | CGGUGGCCGGGCACCCG | 17 | 5145 |
| HSV1-UL30-2113 | - | ACCUGUCCACCACCGCC | 17 | 5146 |
| HSV1-UL30-2114 | - | CCUGUCCACCACCGCCC | 17 | 5147 |
| HSV1-UL30-2115 | - | CGGUUCCUGCGACCUCC | 17 | 5148 |
| HSV1-UL30-2116 | - | ACCUCCCCGAAUCCCAC | 17 | 5149 |
| HSV1-UL30-2117 | - | CCCCGAAUCCCACCUGA | 17 | 5150 |
| HSV1-UL30-2118 | - | CUGAACGAGCUGGCGGC | 17 | 5151 |
| HSV1-UL30-2119 | - | UGAACGAGCUGGCGGCC | 17 | 5152 |
| HSV1-UL30-2120 | - | UGCCCACGCCCGUGGUU | 17 | 5153 |
| HSV1-UL30-2121 | - | GCCCACGCCCGUGGUUC | 17 | 5154 |
| HSV1-UL30-2122 | - | GGUUCUGGAAUUCGACA | 17 | 5155 |
| HSV1-UL30-2123 | - | GGAAUUCGACAGCGAAU | 17 | 5156 |
| HSV1-UL30-2124 | - | UGGCCUUCAUGACCCUU | 17 | 5157 |
| HSV1-UL30-2125 | - | UGUGAAACAGUACGGCC | 17 | 5158 |
| HSV1-UL30-2126 | - | CGGCCCCGAGUUCGUGA | 17 | 5159 |
| HSV1-UL30-2127 | - | UCUUGCUGGCCAAGCUG | 17 | 5160 |
| HSV1-UL30-2128 | - | ACAUUUACAAGGUCCCC | 17 | 5161 |
| HSV1-UL30-2129 | - | UUACAAGGUCCCCCUGG | 17 | 5162 |
| HSV1-UL30-2130 | - | UGGACGGGUACGGCCGC | 17 | 5163 |
| HSV1-UL30-2131 | - | UACGGCCGCAUGAACGG | 17 | 5164 |
| HSV1-UL30-2132 | - | ACGGCCGCAUGAACGGC | 17 | 5165 |
| HSV1-UL30-2133 | - | CGGGGCGUGUUUCGCGU | 17 | 5166 |
| HSV1-UL30-2134 | - | GGGGCGUGUUUCGCGUG | 17 | 5167 |
| HSV1-UL30-2135 | - | GCGUGUGGGACAUAGGC | 17 | 5168 |
| HSV1-UL30-2136 | - | UAGGCCAGAGCCACUUC | 17 | 5169 |
| HSV1-UL30-2137 | - | AGCGCAGCAAGAUAAAG | 17 | 5170 |
| HSV1-UL30-2138 | - | UAAAGGUGAACGGCAUG | 17 | 5171 |
| HSV1-UL30-2139 | - | GGUGAACAUCGACAUGU | 17 | 5172 |
| HSV1-UL30-2140 | - | GUGAACAUCGACAUGUA | 17 | 5173 |
| HSV1-UL30-2141 | - | CCGACAAGAUCAAGCUC | 17 | 5174 |
| HSV1-UL30-2142 | - | CAAGCUCAACGCCGUGG | 17 | 5175 |
| HSV1-UL30-2143 | - | CCGUGGCCGAAGCCGUC | 17 | 5176 |
| HSV1-UL30-2144 | - | UGGCCGAAGCCGUCCUG | 17 | 5177 |
| HSV1-UL30-2145 | - | AAGCCGUCCUGAAGGAC | 17 | 5178 |
| HSV1-UL30-2146 | - | CCGUCCUGAAGGACAAG | 17 | 5179 |
| HSV1-UL30-2147 | - | UCCUGAAGGACAAGAAG | 17 | 5180 |
| HSV1-UL30-2148 | - | AGGACAAGAAGAAGGAC | 17 | 5181 |
| HSV1-UL30-2149 | - | CCCCGCCUACUACGCCG | 17 | 5182 |
| HSV1-UL30-2150 | - | CGCCGGGCCCGCGCAAC | 17 | 5183 |
| HSV1-UL30-860 | - | GCCGGGCCCGCGCAACG | 17 | 3798 |
| HSV1-UL30-2152 | - | GCAACGCGGGGUGAUCG | 17 | 5184 |
| HSV1-UL30-2153 | - | UCGGCGAGUACUGCAUA | 17 | 5185 |
| HSV1-UL30-2154 | - | UACAGGAUUCCCUGCUG | 17 | 5186 |
| HSV1-UL30-2155 | - | UUAAGUUUUUGCCCCAU | 17 | 5187 |
| HSV1-UL30-2156 | - | UAAGUUUUUGCCCCAUC | 17 | 5188 |
| HSV1-UL30-2157 | - | CGGCCGUCGCGCGCUUG | 17 | 5189 |
| HSV1-UL30-2158 | - | UGCUGCGCCUGGCCGAC | 17 | 5190 |
| HSV1-UL30-2159 | - | UGCGCCUGGCCGACCAG | 17 | 5191 |
| HSV1-UL30-2160 | - | AGAAGGGCUUUAUUCUG | 17 | 5192 |
| HSV1-UL30-2161 | - | UUAUUCUGCCGGACACC | 17 | 5193 |
| HSV1-UL30-2162 | - | UAUUCUGCCGGACACCC | 17 | 5194 |
| HSV1-UL30-2163 | - | GACACCCAGGGGCGAUU | 17 | 5195 |
| HSV1-UL30-2164 | - | ACACCCAGGGGCGAUUU | 17 | 5196 |
| HSV1-UL30-2165 | - | GGGGCGAUUUAGGGGCG | 17 | 5197 |
| HSV1-UL30-2166 | - | GGGCGAUUUAGGGGCGC | 17 | 5198 |
| HSV1-UL30-2167 | - | GGCGAUUUAGGGGCGCC | 17 | 5199 |
| HSV1-UL30-2168 | - | GCGAUUUAGGGGCGCCG | 17 | 5200 |
| HSV1-UL30-2169 | - | CGAUUUAGGGGCGCCGG | 17 | 5201 |
| HSV1-UL30-2170 | - | GAUUUAGGGGCGCCGGG | 17 | 5202 |
| HSV1-UL30-2171 | - | AUUUAGGGGCGCCGGGG | 17 | 5203 |
| HSV1-UL30-2172 | - | AAGCGUCCGGCCGCAGC | 17 | 5204 |
| HSV1-UL30-2173 | - | AGCGUCCGGCCGCAGCC | 17 | 5205 |
| HSV1-UL30-2174 | - | GCGUCCGGCCGCAGCCC | 17 | 5206 |
| HSV1-UL30-2175 | - | GUCCGGCCGCAGCCCGG | 17 | 5207 |
| HSV1-UL30-2176 | - | GGCCGCAGCCCGGGAGG | 17 | 5208 |
| HSV1-UL30-2177 | - | CCGCAGCCCGGGAGGAC | 17 | 5209 |
| HSV1-UL30-895 | - | CGCAGCCCGGGAGGACG | 17 | 3833 |
| HSV1-UL30-2179 | - | GGAGGACGAGGAGCGGC | 17 | 5210 |
| HSV1-UL30-2180 | - | AGGACGAGGAGCGGCCA | 17 | 5211 |
| HSV1-UL30-897 | - | GGACGAGGAGCGGCCAG | 17 | 3835 |
| HSV1-UL30-2182 | - | ACGAGGAGCGGCCAGAG | 17 | 5212 |
| HSV1-UL30-898 | - | CGAGGAGCGGCCAGAGG | 17 | 3836 |
| HSV1-UL30-2184 | - | AGGAGCGGCCAGAGGAG | 17 | 5213 |
| HSV1-UL30-899 | - | GGAGCGGCCAGAGGAGG | 17 | 3837 |
| HSV1-UL30-900 | - | GAGCGGCCAGAGGAGGA | 17 | 3838 |
| HSV1-UL30-901 | - | AGCGGCCAGAGGAGGAG | 17 | 3839 |
| HSV1-UL30-902 | - | GCGGCCAGAGGAGGAGG | 17 | 3840 |
| HSV1-UL30-2189 | - | GGCCAGAGGAGGAGGGG | 17 | 5214 |
| HSV1-UL30-2190 | - | AGAGGAGGAGGGGGAGG | 17 | 5215 |
| HSV1-UL30-2191 | - | AGGAGGAGGGGGAGGAC | 17 | 5216 |
| HSV1-UL30-2192 | - | GGAGGGGGAGGACGAGG | 17 | 5217 |
| HSV1-UL30-2193 | - | GGAGGACGAGGACGAAC | 17 | 5218 |
| HSV1-UL30-2194 | - | AGGACGAGGACGAACGC | 17 | 5219 |
| HSV1-UL30-905 | - | GGACGAGGACGAACGCG | 17 | 3843 |
| HSV1-UL30-2196 | - | ACGAGGACGAACGCGAG | 17 | 5220 |
| HSV1-UL30-2197 | - | GGACGAACGCGAGGAGG | 17 | 5221 |
| HSV1-UL30-908 | - | GACGAACGCGAGGAGGG | 17 | 3846 |
| HSV1-UL30-909 | - | ACGAACGCGAGGAGGGC | 17 | 3847 |
| HSV1-UL30-2200 | - | ACGCGAGGAGGGCGGGG | 17 | 5222 |
| HSV1-UL30-2201 | - | GAGGAGGGCGGGGGCGA | 17 | 5223 |
| HSV1-UL30-912 | - | AGGAGGGCGGGGGCGAG | 17 | 3850 |
| HSV1-UL30-913 | - | GGAGGGCGGGGGCGAGC | 17 | 3851 |
| HSV1-UL30-2204 | - | GCGGGGGCGAGCGGGAG | 17 | 5224 |
| HSV1-UL30-914 | - | CGGGGGCGAGCGGGAGC | 17 | 3852 |
| HSV1-UL30-2206 | - | GGGGCGAGCGGGAGCCG | 17 | 5225 |
| HSV1-UL30-2207 | - | CGGGAGCCGGAGGGCGC | 17 | 5226 |
| HSV1-UL30-917 | - | GGGAGCCGGAGGGCGCG | 17 | 3855 |
| HSV1-UL30-918 | - | GGAGCCGGAGGGCGCGC | 17 | 3856 |
| HSV1-UL30-2210 | - | AGACCGCCGGCAGGCAC | 17 | 5227 |
| HSV1-UL30-921 | - | GACCGCCGGCAGGCACG | 17 | 3859 |
| HSV1-UL30-2212 | - | GCAGGCACGUGGGGUAC | 17 | 5228 |
| HSV1-UL30-2213 | - | CAGGCACGUGGGGUACC | 17 | 5229 |
| HSV1-UL30-2214 | - | AGGCACGUGGGGUACCA | 17 | 5230 |
| HSV1-UL30-2215 | - | GUGGGGUACCAGGGGGC | 17 | 5231 |
| HSV1-UL30-2216 | - | GGUCCUUGACCCCACUU | 17 | 5232 |
| HSV1-UL30-2217 | - | CCACUUCCGGGUUUCAC | 17 | 5233 |
| HSV1-UL30-2218 | - | GCUUCAGCACGCUCUCC | 17 | 5234 |
| HSV1-UL30-2219 | - | UUCAGCACGCUCUCCCU | 17 | 5235 |
| HSV1-UL30-2220 | - | CCGACGCAGUGGCGCAC | 17 | 5236 |
| HSV1-UL30-2221 | - | CGACGCAGUGGCGCACC | 17 | 5237 |
| HSV1-UL30-2222 | - | CAGUGGCGCACCUGGAG | 17 | 5238 |
| HSV1-UL30-2223 | - | CGCACCUGGAGGCGGGC | 17 | 5239 |
| HSV1-UL30-2224 | - | AGGCGGGCAAGGACUAC | 17 | 5240 |
| HSV1-UL30-2225 | - | GGCGGGCAAGGACUACC | 17 | 5241 |
| HSV1-UL30-2226 | - | CAAGGACUACCUGGAGA | 17 | 5242 |
| HSV1-UL30-2227 | - | ACUACCUGGAGAUCGAG | 17 | 5243 |
| HSV1-UL30-2228 | - | CUACCUGGAGAUCGAGG | 17 | 5244 |
| HSV1-UL30-2229 | - | UACCUGGAGAUCGAGGU | 17 | 5245 |
| HSV1-UL30-2230 | - | ACCUGGAGAUCGAGGUG | 17 | 5246 |
| HSV1-UL30-2231 | - | CCUGGAGAUCGAGGUGG | 17 | 5247 |
| HSV1-UL30-2232 | - | UUCGUCAAGGCUCACGU | 17 | 5248 |
| HSV1-UL30-2233 | - | CGUCAAGGCUCACGUGC | 17 | 5249 |
| HSV1-UL30-2234 | - | UCAAGGCUCACGUGCGA | 17 | 5250 |
| HSV1-UL30-2235 | - | CUCCUCAGCAUCCUCCU | 17 | 5251 |
| HSV1-UL30-2236 | - | UCCUCAGCAUCCUCCUG | 17 | 5252 |
| HSV1-UL30-2237 | - | CGGGACUGGCUCGCCAU | 17 | 5253 |
| HSV1-UL30-2238 | - | CGAAAGCAGAUCCGCUC | 17 | 5254 |
| HSV1-UL30-2239 | - | UCCGCUCGCGGAUUCCC | 17 | 5255 |
| HSV1-UL30-2240 | - | GAUUCCCCAGAGCAGCC | 17 | 5256 |
| HSV1-UL30-2241 | - | UUCCCCAGAGCAGCCCC | 17 | 5257 |
| HSV1-UL30-2242 | - | UCCCCAGAGCAGCCCCG | 17 | 5258 |
| HSV1-UL30-2243 | - | CCGAGGAGGCCGUGCUC | 17 | 5259 |
| HSV1-UL30-2244 | - | CGUGUGUAACUCGGUGU | 17 | 5260 |
| HSV1-UL30-2245 | - | ACUCGGUGUACGGGUUC | 17 | 5261 |
| HSV1-UL30-2246 | - | CUCGGUGUACGGGUUCA | 17 | 5262 |
| HSV1-UL30-2247 | - | UCGGUGUACGGGUUCAC | 17 | 5263 |
| HSV1-UL30-2248 | - | GUUCACGGGAGUGCAGC | 17 | 5264 |
| HSV1-UL30-2249 | - | GGUGACGACCAUCGGCC | 17 | 5265 |
| HSV1-UL30-2250 | - | GAUGCUGCUCGCGACCC | 17 | 5266 |
| HSV1-UL30-2251 | - | GAGUACGUCCACGCGCG | 17 | 5267 |
| HSV1-UL30-2252 | - | CGCGCGCUGGGCGGCCU | 17 | 5268 |
| HSV1-UL30-2253 | - | AGCUCCUGGCCGAUUUC | 17 | 5269 |
| HSV1-UL30-2254 | - | GCUCCUGGCCGAUUUCC | 17 | 5270 |
| HSV1-UL30-2255 | - | GGCCGACAUGCGCGCCC | 17 | 5271 |
| HSV1-UL30-2256 | - | UUCCAUGCGCAUCAUCU | 17 | 5272 |
| HSV1-UL30-2257 | - | UCCAUGCGCAUCAUCUA | 17 | 5273 |
| HSV1-UL30-2258 | - | CCAUGCGCAUCAUCUAC | 17 | 5274 |
| HSV1-UL30-2259 | - | GCAUCAUCUACGGGGAC | 17 | 5275 |
| HSV1-UL30-2260 | - | CCGCGGCCUCACGGCCG | 17 | 5276 |
| HSV1-UL30-2261 | - | CCGCCGGGCUGACGGCC | 17 | 5277 |
| HSV1-UL30-2262 | - | CCGUGGGCGACAAGAUG | 17 | 5278 |
| HSV1-UL30-2263 | - | UCUGCCCCCCAUCAAAC | 17 | 5279 |
| HSV1-UL30-2264 | - | CCCCAUCAAACUCGAGU | 17 | 5280 |
| HSV1-UL30-2265 | - | AGCUGCUGCUGAUCGCC | 17 | 5281 |
| HSV1-UL30-2266 | - | GUACAUCGGCGUCAUCU | 17 | 5282 |
| HSV1-UL30-2267 | - | UACAUCGGCGUCAUCUA | 17 | 5283 |
| HSV1-UL30-2268 | - | ACAUCGGCGUCAUCUAC | 17 | 5284 |
| HSV1-UL30-2269 | - | GGGGUAAGAUGCUCAUC | 17 | 5285 |
| HSV1-UL30-2270 | - | AGAUGCUCAUCAAGGGC | 17 | 5286 |
| HSV1-UL30-2271 | - | UUUAUCAACCGCACCUC | 17 | 5287 |
| HSV1-UL30-2272 | - | UUACGACGAUACCGUCU | 17 | 5288 |
| HSV1-UL30-2273 | - | UACGACGAUACCGUCUC | 17 | 5289 |
| HSV1-UL30-2274 | - | AGCGGCCGCCGCGUUAG | 17 | 5290 |
| HSV1-UL30-2275 | - | CGUUAGCCGAGCGCCCC | 17 | 5291 |
| HSV1-UL30-2276 | - | GUUAGCCGAGCGCCCCG | 17 | 5292 |
| HSV1-UL30-2277 | - | UAGCCGAGCGCCCCGCG | 17 | 5293 |
| HSV1-UL30-1006 | - | AGCCGAGCGCCCCGCGG | 17 | 3944 |
| HSV1-UL30-2279 | - | GCUGGCGCGACCCCUGC | 17 | 5294 |
| HSV1-UL30-2280 | - | UGGCGCGACCCCUGCCC | 17 | 5295 |
| HSV1-UL30-2281 | - | GGCGCGACCCCUGCCCG | 17 | 5296 |
| HSV1-UL30-2282 | - | CGAGGGACUGCAGGCGU | 17 | 5297 |
| HSV1-UL30-2283 | - | GAGGGACUGCAGGCGUU | 17 | 5298 |
| HSV1-UL30-2284 | - | AUCGGCGCAUCACCGAC | 17 | 5299 |
| HSV1-UL30-2285 | - | UCGGCGCAUCACCGACC | 17 | 5300 |
| HSV1-UL30-2286 | - | GGCGCAUCACCGACCCG | 17 | 5301 |
| HSV1-UL30-2287 | - | CGCAUCACCGACCCGGA | 17 | 5302 |
| HSV1-UL30-2288 | - | GCAUCACCGACCCGGAG | 17 | 5303 |
| HSV1-UL30-2289 | - | ACCCGGAGAGGGACAUC | 17 | 5304 |
| HSV1-UL30-2290 | - | GGACUUUGUCCUCACCG | 17 | 5305 |
| HSV1-UL30-2291 | - | UUGUCCUCACCGCCGAA | 17 | 5306 |
| HSV1-UL30-2292 | - | CGCAGGUCCCGUCCAUC | 17 | 5307 |
| HSV1-UL30-2293 | - | GUCCCGUCCAUCAAGGA | 17 | 5308 |
| HSV1-UL30-2294 | - | GAUCGUGGCCCAGACCC | 17 | 5309 |
| HSV1-UL30-2295 | - | GGCCCAGACCCGCGAGG | 17 | 5310 |
| HSV1-UL30-2296 | - | CCCAGACCCGCGAGGUA | 17 | 5311 |
| HSV1-UL30-2297 | - | CCAGACCCGCGAGGUAG | 17 | 5312 |
| HSV1-UL30-2298 | - | GCGGCUGGCCGCCCUCC | 17 | 5313 |
| HSV1-UL30-2299 | - | GCUAGACGCCGCCGCCC | 17 | 5314 |
| HSV1-UL30-2300 | - | CUAGACGCCGCCGCCCC | 17 | 5315 |
| HSV1-UL30-2301 | - | UAGACGCCGCCGCCCCA | 17 | 5316 |
| HSV1-UL30-2302 | - | CGCCGCCGCCCCAGGGG | 17 | 5317 |
| HSV1-UL30-2303 | - | UCCCCGGCCAAGCGCCC | 17 | 5318 |
| HSV1-UL30-1037 | - | CCCCGGCCAAGCGCCCC | 17 | 3975 |
| HSV1-UL30-1038 | - | CCCGGCCAAGCGCCCCC | 17 | 3976 |
| HSV1-UL30-2306 | - | CGUCGCCUGCCGACCCC | 17 | 5319 |
| HSV1-UL30-2307 | - | GUCGCCUGCCGACCCCC | 17 | 5320 |
| HSV1-UL30-2308 | - | UCGCCUGCCGACCCCCC | 17 | 5321 |
| HSV1-UL30-2309 | - | CCGCAAGCUGCUGGUGU | 17 | 5322 |
| HSV1-UL30-2310 | - | GCUGGUGUCCGAGCUGG | 17 | 5323 |
| HSV1-UL30-2311 | - | UGGUGUCCGAGCUGGCC | 17 | 5324 |
| HSV1-UL30-2312 | - | UUGCCCACGGCGUCGCC | 17 | 5325 |
| HSV1-UL30-2313 | - | ACGGCGUCGCCCUGAAC | 17 | 5326 |
| HSV1-UL30-2314 | - | AUUACUUCUCCCACCUG | 17 | 5327 |
| HSV1-UL30-2315 | - | UUACUUCUCCCACCUGU | 17 | 5328 |
| HSV1-UL30-2316 | - | UACUUCUCCCACCUGUU | 17 | 5329 |
| HSV1-UL30-2317 | - | GACAUUCAAGGCCCUGU | 17 | 5330 |
| HSV1-UL30-2318 | - | ACAUUCAAGGCCCUGUU | 17 | 5331 |
| HSV1-UL30-2319 | - | CAUUCAAGGCCCUGUUU | 17 | 5332 |
| HSV1-UL30-2320 | - | GAAUAACGCCAAGAUCA | 17 | 5333 |
| HSV1-UL30-2321 | - | AUAACGCCAAGAUCACC | 17 | 5334 |
| HSV1-UL30-2322 | - | GUUAAAAAGGUUUAUUC | 17 | 5335 |
| HSV1-UL30-2323 | - | CCGAAGUGUGGCACCCC | 17 | 5336 |
| HSV1-UL30-2324 | - | GACGUGGCCGCGCGGCU | 17 | 5337 |
| HSV1-UL30-2325 | - | CGCGCGGCUCCGGACCG | 17 | 5338 |
| HSV1-UL30-2326 | - | GCUCCGGACCGCAGGGU | 17 | 5339 |
| HSV1-UL30-2327 | - | CUCCGGACCGCAGGGUU | 17 | 5340 |
| HSV1-UL30-2328 | - | CCGCAGGGUUCGGGGCG | 17 | 5341 |
| HSV1-UL30-2329 | - | UGGGUGCCGGCGCUACG | 17 | 5342 |
| HSV1-UL30-2330 | - | GGGUGCCGGCGCUACGG | 17 | 5343 |
| HSV1-UL30-2331 | - | GUGCCGGCGCUACGGCG | 17 | 5344 |
| HSV1-UL30-2332 | - | UGCCGGCGCUACGGCGG | 17 | 5345 |
| HSV1-UL30-2333 | - | ACGGCGGAGGAAACUCG | 17 | 5346 |
| HSV1-UL30-2334 | - | ACUCGUCGAAUGUUGCA | 17 | 5347 |
| HSV1-UL30-2335 | + | AAGGCUCUAUGCAACAUUCG | 20 | 5348 |
| HSV1-UL30-2336 | + | UAGCGCCGGCACCCACCGCC | 20 | 5349 |
| HSV1-UL30-2337 | + | ACCGCCCCGAACCCUGCGGU | 20 | 5350 |
| HSV1-UL30-2338 | + | CCGCCCCGAACCCUGCGGUC | 20 | 5351 |
| HSV1-UL30-2339 | + | GAGCCGCGCGGCCACGUCGU | 20 | 5352 |
| HSV1-UL30-2340 | + | AGCCGCGCGGCCACGUCGUC | 20 | 5353 |
| HSV1-UL30-2341 | + | GCCGCGCGGCCACGUCGUCC | 20 | 5354 |
| HSV1-UL30-2342 | + | CCGCGCGGCCACGUCGUCCG | 20 | 5355 |
| HSV1-UL30-2343 | + | CGUCCGGGGGGUGCCACACU | 20 | 5356 |
| HSV1-UL30-2344 | + | GUCCGGGGGGUGCCACACUU | 20 | 5357 |
| HSV1-UL30-2345 | + | UCCGGGGGGUGCCACACUUC | 20 | 5358 |
| HSV1-UL30-2346 | + | AUCUUGGCGUUAUUCCCAAA | 20 | 5359 |
| HSV1-UL30-2347 | + | CGUUAUUCCCAAACAGGGCC | 20 | 5360 |
| HSV1-UL30-2348 | + | ACGCACGCCGCCCCCAACAG | 20 | 5361 |
| HSV1-UL30-1089 | + | CGCACGCCGCCCCCAACAGG | 20 | 4027 |
| HSV1-UL30-2350 | + | GCACGCCGCCCCCAACAGGU | 20 | 5362 |
| HSV1-UL30-2351 | + | ACGCCGCCCCCAACAGGUGG | 20 | 5363 |
| HSV1-UL30-2352 | + | GAGAAGUAAUAGUCCGUGUU | 20 | 5364 |
| HSV1-UL30-2353 | + | UCCGUGUUCAGGGCGACGCC | 20 | 5365 |
| HSV1-UL30-2354 | + | CGCCGUGGGCAAUGGCGUAU | 20 | 5366 |
| HSV1-UL30-2355 | + | GCCGUGGGCAAUGGCGUAUG | 20 | 5367 |
| HSV1-UL30-2356 | + | AUGCGGGAUCCUCGGCCAGC | 20 | 5368 |
| HSV1-UL30-2357 | + | AGCUCGGACACCAGCAGCUU | 20 | 5369 |
| HSV1-UL30-2358 | + | GCUCGGACACCAGCAGCUUG | 20 | 5370 |
| HSV1-UL30-2359 | + | ACACCAGCAGCUUGCGGGGC | 20 | 5371 |
| HSV1-UL30-2360 | + | GCGGGGCUUGGACGCGCCUC | 20 | 5372 |
| HSV1-UL30-2361 | + | CGGGGCUUGGACGCGCCUCC | 20 | 5373 |
| HSV1-UL30-2362 | + | GGGGCUUGGACGCGCCUCCC | 20 | 5374 |
| HSV1-UL30-2363 | + | GGGCUUGGACGCGCCUCCCG | 20 | 5375 |
| HSV1-UL30-2364 | + | UCGGCAGGCGACGGCGUCUC | 20 | 5376 |
| HSV1-UL30-2365 | + | CGGCAGGCGACGGCGUCUCC | 20 | 5377 |
| HSV1-UL30-2366 | + | GGCAGGCGACGGCGUCUCCC | 20 | 5378 |
| HSV1-UL30-2367 | + | CGUCUCCCGGGGGCGCUUGG | 20 | 5379 |
| HSV1-UL30-2368 | + | GUCUCCCGGGGGCGCUUGGC | 20 | 5380 |
| HSV1-UL30-2369 | + | UCUCCCGGGGGCGCUUGGCC | 20 | 5381 |
| HSV1-UL30-2370 | + | CUCCCGGGGGCGCUUGGCCG | 20 | 5382 |
| HSV1-UL30-2371 | + | CCCGGGGGCGCUUGGCCGGG | 20 | 5383 |
| HSV1-UL30-2372 | + | GGGCGCUUGGCCGGGGAGGG | 20 | 5384 |
| HSV1-UL30-2373 | + | UGGCCGGGGAGGGCAGGGCC | 20 | 5385 |
| HSV1-UL30-1124 | + | GGCCGGGGAGGGCAGGGCCG | 20 | 4062 |
| HSV1-UL30-1125 | + | GCCGGGGAGGGCAGGGCCGC | 20 | 4063 |
| HSV1-UL30-1126 | + | CCGGGGAGGGCAGGGCCGCG | 20 | 4064 |
| HSV1-UL30-1127 | + | CGGGGAGGGCAGGGCCGCGG | 20 | 4065 |
| HSV1-UL30-1128 | + | GGGGAGGGCAGGGCCGCGGG | 20 | 4066 |
| HSV1-UL30-2379 | + | AGGGCAGGGCCGCGGGGGGG | 20 | 5386 |
| HSV1-UL30-2380 | + | GGGGGGGGCGGGCUCGUCCC | 20 | 5387 |
| HSV1-UL30-2381 | + | GGGGGGGCGGGCUCGUCCCC | 20 | 5388 |
| HSV1-UL30-2382 | + | GGGGCGGCGGCGUCUAGCUC | 20 | 5389 |
| HSV1-UL30-2383 | + | GGGCGGCGGCGUCUAGCUCG | 20 | 5390 |
| HSV1-UL30-2384 | + | GCGGCGGCGUCUAGCUCGCG | 20 | 5391 |
| HSV1-UL30-2385 | + | GCGACCGUCUCCUCUACCUC | 20 | 5392 |
| HSV1-UL30-2386 | + | GUCUCCUCUACCUCGCGGGU | 20 | 5393 |
| HSV1-UL30-2387 | + | GGUCUGGGCCACGAUCACGU | 20 | 5394 |
| HSV1-UL30-2388 | + | GUCUGGGCCACGAUCACGUA | 20 | 5395 |
| HSV1-UL30-2389 | + | CGUACGGGAUCCGGUCCUUG | 20 | 5396 |
| HSV1-UL30-2390 | + | CGGGAUCCGGUCCUUGAUGG | 20 | 5397 |
| HSV1-UL30-2391 | + | GGGAUCCGGUCCUUGAUGGA | 20 | 5398 |
| HSV1-UL30-2392 | + | AUGGACGGGACCUGCGCGCG | 20 | 5399 |
| HSV1-UL30-2393 | + | GGACCUGCGCGCGGCGGGCC | 20 | 5400 |
| HSV1-UL30-2394 | + | AGCUUGUAAUACACCGUCAG | 20 | 5401 |
| HSV1-UL30-2395 | + | GCGCUUGUUGGUGUACGCGC | 20 | 5402 |
| HSV1-UL30-2396 | + | GGUGUCUGCUCAGUUCGGCG | 20 | 5403 |
| HSV1-UL30-2397 | + | UGUCUGCUCAGUUCGGCGGU | 20 | 5404 |
| HSV1-UL30-2398 | + | UCGGCGGUGAGGACAAAGUC | 20 | 5405 |
| HSV1-UL30-2399 | + | AAAGUCCUGGAUGUCCCUCU | 20 | 5406 |
| HSV1-UL30-2400 | + | UCCGGGUCGGUGAUGCGCCG | 20 | 5407 |
| HSV1-UL30-2401 | + | UGAUGCGCCGAUGGGCGUCU | 20 | 5408 |
| HSV1-UL30-2402 | + | AUGCGCCGAUGGGCGUCUAC | 20 | 5409 |
| HSV1-UL30-2403 | + | GGGCGUCUACGAGGACGGCC | 20 | 5410 |
| HSV1-UL30-2404 | + | CCCCGAACGCCUGCAGUCCC | 20 | 5411 |
| HSV1-UL30-2405 | + | AACGCCUGCAGUCCCUCGGG | 20 | 5412 |
| HSV1-UL30-2406 | + | ACGCCUGCAGUCCCUCGGGC | 20 | 5413 |
| HSV1-UL30-2407 | + | GUCGCGCCAGCCACUCCUCC | 20 | 5414 |
| HSV1-UL30-2408 | + | UCGCGCCAGCCACUCCUCCG | 20 | 5415 |
| HSV1-UL30-2409 | + | CGGCUAACGCGGCGGCCGCU | 20 | 5416 |
| HSV1-UL30-2410 | + | GGCUAACGCGGCGGCCGCUC | 20 | 5417 |
| HSV1-UL30-2411 | + | UCGUAAAACAGCAGGUCGAC | 20 | 5418 |
| HSV1-UL30-2412 | + | ACAGCAGGUCGACCAGGGCC | 20 | 5419 |
| HSV1-UL30-2413 | + | CAGCAGGUCGACCAGGGCCC | 20 | 5420 |
| HSV1-UL30-2414 | + | GCACCAGAUCCACGCCCUUG | 20 | 5421 |
| HSV1-UL30-2415 | + | UGGCGAUCAGCAGCAGCUUG | 20 | 5422 |
| HSV1-UL30-2416 | + | UGGUGAACGUCUUUUCGCAC | 20 | 5423 |
| HSV1-UL30-2417 | + | UCUUUUCGCACUCGAGUUUG | 20 | 5424 |
| HSV1-UL30-2418 | + | CUUUUCGCACUCGAGUUUGA | 20 | 5425 |
| HSV1-UL30-2419 | + | UUUUCGCACUCGAGUUUGAU | 20 | 5426 |
| HSV1-UL30-2420 | + | UUUCGCACUCGAGUUUGAUG | 20 | 5427 |
| HSV1-UL30-2421 | + | CACUCGAGUUUGAUGGGGGG | 20 | 5428 |
| HSV1-UL30-2422 | + | GGGGGGCAGAAACAGCGCGC | 20 | 5429 |
| HSV1-UL30-2423 | + | CGGCCGUCAGCCCGGCGGCC | 20 | 5430 |
| HSV1-UL30-2424 | + | GGCCGCGGCACAGCACAAAG | 20 | 5431 |
| HSV1-UL30-1205 | + | GCCGCGGCACAGCACAAAGA | 20 | 4143 |
| HSV1-UL30-2426 | + | UGUCCCCGUAGAUGAUGCGC | 20 | 5432 |
| HSV1-UL30-2427 | + | GUCCCCGUAGAUGAUGCGCA | 20 | 5433 |
| HSV1-UL30-2428 | + | CGUAGAUGAUGCGCAUGGAA | 20 | 5434 |
| HSV1-UL30-2429 | + | UGAUGCGCAUGGAAUAGGGC | 20 | 5435 |
| HSV1-UL30-2430 | + | GAUGCGCAUGGAAUAGGGCC | 20 | 5436 |
| HSV1-UL30-2431 | + | AUGCGCAUGGAAUAGGGCCC | 20 | 5437 |
| HSV1-UL30-2432 | + | GGCGCGCAUGUCGGCCGCCU | 20 | 5438 |
| HSV1-UL30-2433 | + | GCGCGCAUGUCGGCCGCCUC | 20 | 5439 |
| HSV1-UL30-2434 | + | CGCGCAUGUCGGCCGCCUCC | 20 | 5440 |
| HSV1-UL30-2435 | + | GCCGCCUCCGGGAAAUCGGC | 20 | 5441 |
| HSV1-UL30-2436 | + | CCGCCUCCGGGAAAUCGGCC | 20 | 5442 |
| HSV1-UL30-2437 | + | GGAAAUCGGCCAGGAGCUGU | 20 | 5443 |
| HSV1-UL30-2438 | + | UCGAAGGCCGCCCAGCGCGC | 20 | 5444 |
| HSV1-UL30-2439 | + | CAGCGCGCGUGGACGUACUC | 20 | 5445 |
| HSV1-UL30-2440 | + | CGUGGACGUACUCGCGGGUC | 20 | 5446 |
| HSV1-UL30-2441 | + | GCGGCAACGUGCAGGCACGG | 20 | 5447 |
| HSV1-UL30-2442 | + | CGGCAACGUGCAGGCACGGC | 20 | 5448 |
| HSV1-UL30-2443 | + | GGAGUCCGUGCUGCACUCCC | 20 | 5449 |
| HSV1-UL30-2444 | + | CACUCCCGUGAACCCGUACA | 20 | 5450 |
| HSV1-UL30-2445 | + | AUGGCGGCCUGCUGCUUGUC | 20 | 5451 |
| HSV1-UL30-2446 | + | UGGCGGCCUGCUGCUUGUCC | 20 | 5452 |
| HSV1-UL30-2447 | + | UGUCCAGGAGCACGGCCUCC | 20 | 5453 |
| HSV1-UL30-2448 | + | GUCCAGGAGCACGGCCUCCU | 20 | 5454 |
| HSV1-UL30-2449 | + | ACGGCCUCCUCGGGGCUGCU | 20 | 5455 |
| HSV1-UL30-2450 | + | CGGCCUCCUCGGGGCUGCUC | 20 | 5456 |
| HSV1-UL30-2451 | + | GGCCUCCUCGGGGCUGCUCU | 20 | 5457 |
| HSV1-UL30-2452 | + | GCCUCCUCGGGGCUGCUCUG | 20 | 5458 |
| HSV1-UL30-2453 | + | GGGGCUGCUCUGGGGAAUCC | 20 | 5459 |
| HSV1-UL30-2454 | + | CUGCUCUGGGGAAUCCGCGA | 20 | 5460 |
| HSV1-UL30-2455 | + | AGCGGAUCUGCUUUCGCAUG | 20 | 5461 |
| HSV1-UL30-2456 | + | CGCAUGGCGAGCCAGUCCCG | 20 | 5462 |
| HSV1-UL30-2457 | + | GCAUGGCGAGCCAGUCCCGC | 20 | 5463 |
| HSV1-UL30-2458 | + | AUGGCGAGCCAGUCCCGCAG | 20 | 5464 |
| HSV1-UL30-2459 | + | GCCAGUCCCGCAGGAGGAUG | 20 | 5465 |
| HSV1-UL30-2460 | + | CAGUCCCGCAGGAGGAUGCU | 20 | 5466 |
| HSV1-UL30-2461 | + | AGUCCCGCAGGAGGAUGCUG | 20 | 5467 |
| HSV1-UL30-2462 | + | CUGAGGAGGCUCUCUCGCAC | 20 | 5468 |
| HSV1-UL30-2463 | + | UCUCUCGCACGUGAGCCUUG | 20 | 5469 |
| HSV1-UL30-2464 | + | CUCGCACGUGAGCCUUGACG | 20 | 5470 |
| HSV1-UL30-2465 | + | UGCGCCACUGCGUCGGCCCU | 20 | 5471 |
| HSV1-UL30-2466 | + | GCGCCACUGCGUCGGCCCUC | 20 | 5472 |
| HSV1-UL30-2467 | + | CGCCACUGCGUCGGCCCUCA | 20 | 5473 |
| HSV1-UL30-2468 | + | CCACUGCGUCGGCCCUCAGG | 20 | 5474 |
| HSV1-UL30-2469 | + | CGGCCCUCAGGGAGAGCGUG | 20 | 5475 |
| HSV1-UL30-2470 | + | AGCGUGCUGAAGCACAGGUU | 20 | 5476 |
| HSV1-UL30-2471 | + | CUGAAGCACAGGUUGUGGGC | 20 | 5477 |
| HSV1-UL30-2472 | + | GGUUGUGGGCCUGGAUGAUG | 20 | 5478 |
| HSV1-UL30-2473 | + | GUUGUGGGCCUGGAUGAUGC | 20 | 5479 |
| HSV1-UL30-2474 | + | UGGGGUACAGGCUGGCAAAG | 20 | 5480 |
| HSV1-UL30-2475 | + | UGGCAAAGUCGAACACCACC | 20 | 5481 |
| HSV1-UL30-2476 | + | GGCAAAGUCGAACACCACCA | 20 | 5482 |
| HSV1-UL30-2477 | + | AACACCACCACGGGGUUCAC | 20 | 5483 |
| HSV1-UL30-2478 | + | CCACGGGGUUCACGUGAAAC | 20 | 5484 |
| HSV1-UL30-2479 | + | CACGGGGUUCACGUGAAACC | 20 | 5485 |
| HSV1-UL30-2480 | + | GGUUCACGUGAAACCCGGAA | 20 | 5486 |
| HSV1-UL30-2481 | + | GUUCACGUGAAACCCGGAAG | 20 | 5487 |
| HSV1-UL30-2482 | + | UGAAACCCGGAAGUGGGGUC | 20 | 5488 |
| HSV1-UL30-2483 | + | UCUGGCCGCUCCUCGUCCUC | 20 | 5489 |
| HSV1-UL30-2484 | + | CUCGUCCUCCCGGGCUGCGG | 20 | 5490 |
| HSV1-UL30-2485 | + | CCCGGGCUGCGGCCGGACGC | 20 | 5491 |
| HSV1-UL30-2486 | + | CCGGCGCCCCUAAAUCGCCC | 20 | 5492 |
| HSV1-UL30-2487 | + | AAUCGCCCCUGGGUGUCCGG | 20 | 5493 |
| HSV1-UL30-2488 | + | CGCAGCAGGCACGUAAAGAC | 20 | 5494 |
| HSV1-UL30-2489 | + | UGCUGGCCGUCGUAGAUGGU | 20 | 5495 |
| HSV1-UL30-2490 | + | ACCCGCCAAGCGCGCGACGG | 20 | 5496 |
| HSV1-UL30-2491 | + | CCGCCAAGCGCGCGACGGCC | 20 | 5497 |
| HSV1-UL30-2492 | + | GCGACGGCCGAGAGCUCCAG | 20 | 5498 |
| HSV1-UL30-2493 | + | CGACGGCCGAGAGCUCCAGA | 20 | 5499 |
| HSV1-UL30-2494 | + | AAAAACAGCUGGCCCACCAG | 20 | 5500 |
| HSV1-UL30-2495 | + | AAAACAGCUGGCCCACCAGC | 20 | 5501 |
| HSV1-UL30-2496 | + | AAACAGCUGGCCCACCAGCA | 20 | 5502 |
| HSV1-UL30-2497 | + | CGCCGAUCACCCCGCGUUGC | 20 | 5503 |
| HSV1-UL30-2498 | + | CGGGCCCGGCGGCGUAGUAG | 20 | 5504 |
| HSV1-UL30-2499 | + | GGGCCCGGCGGCGUAGUAGG | 20 | 5505 |
| HSV1-UL30-2500 | + | GGCCCGGCGGCGUAGUAGGC | 20 | 5506 |
| HSV1-UL30-2501 | + | AGGUCCUUCUUCUUGUCCUU | 20 | 5507 |
| HSV1-UL30-2502 | + | GGACGGCUUCGGCCACGGCG | 20 | 5508 |
| HSV1-UL30-2503 | + | CACGGCGUUGAGCUUGUAGC | 20 | 5509 |
| HSV1-UL30-2504 | + | CGGCGUUGAGCUUGUAGCUC | 20 | 5510 |
| HSV1-UL30-2505 | + | ACCUUUAUCUUGCUGCGCUU | 20 | 5511 |
| HSV1-UL30-2506 | + | CCUUUAUCUUGCUGCGCUUC | 20 | 5512 |
| HSV1-UL30-2507 | + | CUCUGGCCUAUGUCCCACAC | 20 | 5513 |
| HSV1-UL30-2508 | + | UUCAUGCGGCCGUACCCGUC | 20 | 5514 |
| HSV1-UL30-2509 | + | UCAUGCGGCCGUACCCGUCC | 20 | 5515 |
| HSV1-UL30-2510 | + | CAUGCGGCCGUACCCGUCCA | 20 | 5516 |
| HSV1-UL30-2511 | + | AUGCGGCCGUACCCGUCCAG | 20 | 5517 |
| HSV1-UL30-2512 | + | UGUCCGUCAGCUUGGCCAGC | 20 | 5518 |
| HSV1-UL30-2513 | + | CCGUCAGCUUGGCCAGCAAG | 20 | 5519 |
| HSV1-UL30-2514 | + | UGGCCAGCAAGAAGGGCCAG | 20 | 5520 |
| HSV1-UL30-2515 | + | UGAUGAUGUUGUACCCGGUC | 20 | 5521 |
| HSV1-UL30-2516 | + | UGUUGUACCCGGUCACGAAC | 20 | 5522 |
| HSV1-UL30-2517 | + | GUUGUACCCGGUCACGAACU | 20 | 5523 |
| HSV1-UL30-2518 | + | UCGGGGCCGUACUGUUUCAC | 20 | 5524 |
| HSV1-UL30-2519 | + | CGUACUGUUUCACAAGGGUC | 20 | 5525 |
| HSV1-UL30-2520 | + | UCAUGAAGGCCAACAGCAUC | 20 | 5526 |
| HSV1-UL30-2521 | + | ACAGCAUCUCGAAUUCGCUG | 20 | 5527 |
| HSV1-UL30-2522 | + | UCGAAUUCGCUGUCGAAUUC | 20 | 5528 |
| HSV1-UL30-2523 | + | CGCUGUCGAAUUCCAGAACC | 20 | 5529 |
| HSV1-UL30-2524 | + | CGAAUUCCAGAACCACGGGC | 20 | 5530 |
| HSV1-UL30-2525 | + | CUGGCCGCCAGCUCGUUCAG | 20 | 5531 |
| HSV1-UL30-2526 | + | UGGCCGCCAGCUCGUUCAGG | 20 | 5532 |
| HSV1-UL30-2527 | + | CCAGCUCGUUCAGGUGGGAU | 20 | 5533 |
| HSV1-UL30-2528 | + | CAGCUCGUUCAGGUGGGAUU | 20 | 5534 |
| HSV1-UL30-2529 | + | AGCUCGUUCAGGUGGGAUUC | 20 | 5535 |
| HSV1-UL30-2530 | + | GCUCGUUCAGGUGGGAUUCG | 20 | 5536 |
| HSV1-UL30-2531 | + | GGUGGGAUUCGGGGAGGUCG | 20 | 5537 |
| HSV1-UL30-2532 | + | GUGGGAUUCGGGGAGGUCGC | 20 | 5538 |
| HSV1-UL30-2533 | + | AUUCGGGGAGGUCGCAGGAA | 20 | 5539 |
| HSV1-UL30-2534 | + | GGGGAGGUCGCAGGAACCGA | 20 | 5540 |
| HSV1-UL30-2535 | + | UCGCAGGAACCGAGCGAAAA | 20 | 5541 |
| HSV1-UL30-1345 | + | CGCAGGAACCGAGCGAAAAC | 20 | 4283 |
| HSV1-UL30-2537 | + | CAGGAACCGAGCGAAAACAG | 20 | 5542 |
| HSV1-UL30-2538 | + | GAAAACAGGAGGACGUGCUC | 20 | 5543 |
| HSV1-UL30-2539 | + | GGACGUGCUCCAGGGCGGUG | 20 | 5544 |
| HSV1-UL30-2540 | + | GGGCGGUGGUGGACAGGUCG | 20 | 5545 |
| HSV1-UL30-2541 | + | UGGACAGGUCGUAGAGCAGA | 20 | 5546 |
| HSV1-UL30-2542 | + | UCGUAGAGCAGACAGGAUAU | 20 | 5547 |
| HSV1-UL30-2543 | + | UAUCUGGAUGACCAGGUCCU | 20 | 5548 |
| HSV1-UL30-2544 | + | GUCCUCCGGGUGCCCGGCCA | 20 | 5549 |
| HSV1-UL30-2545 | + | UCCUCCGGGUGCCCGGCCAC | 20 | 5550 |
| HSV1-UL30-2546 | + | CCCCCGCCUUGCAUUCGAUA | 20 | 5551 |
| HSV1-UL30-2547 | + | UGCAUUCGAUAUCGAAGCAC | 20 | 5552 |
| HSV1-UL30-2548 | + | UACAGUUAAACUCGACGUCG | 20 | 5553 |
| HSV1-UL30-2549 | + | ACUCGACGUCGCUGGAUGUC | 20 | 5554 |
| HSV1-UL30-2550 | + | GCUGGAUGUCCCGAAGGCCA | 20 | 5555 |
| HSV1-UL30-2551 | + | CUGGAUGUCCCGAAGGCCAU | 20 | 5556 |
| HSV1-UL30-2552 | + | AAGGCCAUCGGGGCCGCCGG | 20 | 5557 |
| HSV1-UL30-2553 | + | GCGUGUUGUUCCGGCCCGGU | 20 | 5558 |
| HSV1-UL30-2554 | + | CCGGUUUGAGACGGUACCAG | 20 | 5559 |
| HSV1-UL30-2555 | + | GACGGUACCAGCCGAAGGUG | 20 | 5560 |
| HSV1-UL30-2556 | + | ACCAGCCGAAGGUGACGAAC | 20 | 5561 |
| HSV1-UL30-2557 | + | CCAGCCGAAGGUGACGAACC | 20 | 5562 |
| HSV1-UL30-2558 | + | GUGACGAACCCGGGGUUGUC | 20 | 5563 |
| HSV1-UL30-2559 | + | CGAACCCGGGGUUGUCCAGG | 20 | 5564 |
| HSV1-UL30-2560 | + | CCGGGGUUGUCCAGGAUGAA | 20 | 5565 |
| HSV1-UL30-2561 | + | ACCCUCGUACUUCUUGAUGG | 20 | 5566 |
| HSV1-UL30-2562 | + | CGUACUUCUUGAUGGCCGGG | 20 | 5567 |
| HSV1-UL30-2563 | + | UACGACAGCACACGCCCGCU | 20 | 5568 |
| HSV1-UL30-2564 | + | CGGACGUAGACGCGGUAAAA | 20 | 5569 |
| HSV1-UL30-2565 | + | CGUAGACGCGGUAAAACAGA | 20 | 5570 |
| HSV1-UL30-2566 | + | GUAGACGCGGUAAAACAGAG | 20 | 5571 |
| HSV1-UL30-2567 | + | UGCGCUCCACCACCUCCGCC | 20 | 5572 |
| HSV1-UL30-2568 | + | CCUCCGCCUCGAAGUGGUCC | 20 | 5573 |
| HSV1-UL30-2569 | + | CUCCGCCUCGAAGUGGUCCG | 20 | 5574 |
| HSV1-UL30-2570 | + | AAGUGGUCCGCGGAGAUGCC | 20 | 5575 |
| HSV1-UL30-2571 | + | AGUGGUCCGCGGAGAUGCCG | 20 | 5576 |
| HSV1-UL30-2572 | + | GAUGCCGCGGAACGACGCGC | 20 | 5577 |
| HSV1-UL30-2573 | + | AUGCCGCGGAACGACGCGCC | 20 | 5578 |
| HSV1-UL30-2574 | + | UGCCGCGGAACGACGCGCCC | 20 | 5579 |
| HSV1-UL30-2575 | + | GACGCGCCCGGGGACUCGCG | 20 | 5580 |
| HSV1-UL30-2576 | + | GGGCCGCGGCCAUGCGCUCG | 20 | 5581 |
| HSV1-UL30-2577 | + | CAUGCGCUCGCAGAGAUCUC | 20 | 5582 |
| HSV1-UL30-2578 | + | AUGCGCUCGCAGAGAUCUCG | 20 | 5583 |
| HSV1-UL30-2579 | + | UACUGCCGCGUGCCGUAAAC | 20 | 5584 |
| HSV1-UL30-2580 | + | AACGGCCACCCGGUGGCCUU | 20 | 5585 |
| HSV1-UL30-2581 | + | ACGGCCACCCGGUGGCCUUC | 20 | 5586 |
| HSV1-UL30-2582 | + | UGGCCUUCCGGAGUCAGGCC | 20 | 5587 |
| HSV1-UL30-2583 | + | GGCCUUCCGGAGUCAGGCCC | 20 | 5588 |
| HSV1-UL30-2584 | + | GCUCUAUGCAACAUUCG | 17 | 5589 |
| HSV1-UL30-2585 | + | CGCCGGCACCCACCGCC | 17 | 5590 |
| HSV1-UL30-2586 | + | GCCCCGAACCCUGCGGU | 17 | 5591 |
| HSV1-UL30-2587 | + | CCCCGAACCCUGCGGUC | 17 | 5592 |
| HSV1-UL30-2588 | + | CCGCGCGGCCACGUCGU | 17 | 5593 |
| HSV1-UL30-2589 | + | CGCGCGGCCACGUCGUC | 17 | 5594 |
| HSV1-UL30-2590 | + | GCGCGGCCACGUCGUCC | 17 | 5595 |
| HSV1-UL30-2591 | + | CGCGGCCACGUCGUCCG | 17 | 5596 |
| HSV1-UL30-2592 | + | CCGGGGGGUGCCACACU | 17 | 5597 |
| HSV1-UL30-2593 | + | CGGGGGGUGCCACACUU | 17 | 5598 |
| HSV1-UL30-2594 | + | GGGGGGUGCCACACUUC | 17 | 5599 |
| HSV1-UL30-2595 | + | UUGGCGUUAUUCCCAAA | 17 | 5600 |
| HSV1-UL30-2596 | + | UAUUCCCAAACAGGGCC | 17 | 5601 |
| HSV1-UL30-2597 | + | CACGCCGCCCCCAACAG | 17 | 5602 |
| HSV1-UL30-1438 | + | ACGCCGCCCCCAACAGG | 17 | 4376 |
| HSV1-UL30-2599 | + | CGCCGCCCCCAACAGGU | 17 | 5603 |
| HSV1-UL30-2600 | + | CCGCCCCCAACAGGUGG | 17 | 5604 |
| HSV1-UL30-2601 | + | AAGUAAUAGUCCGUGUU | 17 | 5605 |
| HSV1-UL30-2602 | + | GUGUUCAGGGCGACGCC | 17 | 5606 |
| HSV1-UL30-2603 | + | CGUGGGCAAUGGCGUAU | 17 | 5607 |
| HSV1-UL30-2604 | + | GUGGGCAAUGGCGUAUG | 17 | 5608 |
| HSV1-UL30-2605 | + | CGGGAUCCUCGGCCAGC | 17 | 5609 |
| HSV1-UL30-2606 | + | UCGGACACCAGCAGCUU | 17 | 5610 |
| HSV1-UL30-2607 | + | CGGACACCAGCAGCUUG | 17 | 5611 |
| HSV1-UL30-2608 | + | CCAGCAGCUUGCGGGGC | 17 | 5612 |
| HSV1-UL30-2609 | + | GGGCUUGGACGCGCCUC | 17 | 5613 |
| HSV1-UL30-2610 | + | GGCUUGGACGCGCCUCC | 17 | 5614 |
| HSV1-UL30-2611 | + | GCUUGGACGCGCCUCCC | 17 | 5615 |
| HSV1-UL30-2612 | + | CUUGGACGCGCCUCCCG | 17 | 5616 |
| HSV1-UL30-2613 | + | GCAGGCGACGGCGUCUC | 17 | 5617 |
| HSV1-UL30-2614 | + | CAGGCGACGGCGUCUCC | 17 | 5618 |
| HSV1-UL30-2615 | + | AGGCGACGGCGUCUCCC | 17 | 5619 |
| HSV1-UL30-2616 | + | CUCCCGGGGGCGCUUGG | 17 | 5620 |
| HSV1-UL30-2617 | + | UCCCGGGGGCGCUUGGC | 17 | 5621 |
| HSV1-UL30-2618 | + | CCCGGGGGCGCUUGGCC | 17 | 5622 |
| HSV1-UL30-2619 | + | CCGGGGGCGCUUGGCCG | 17 | 5623 |
| HSV1-UL30-2620 | + | GGGGGCGCUUGGCCGGG | 17 | 5624 |
| HSV1-UL30-2621 | + | CGCUUGGCCGGGGAGGG | 17 | 5625 |
| HSV1-UL30-2622 | + | CCGGGGAGGGCAGGGCC | 17 | 5626 |
| HSV1-UL30-1473 | + | CGGGGAGGGCAGGGCCG | 17 | 4411 |
| HSV1-UL30-1474 | + | GGGGAGGGCAGGGCCGC | 17 | 4412 |
| HSV1-UL30-1475 | + | GGGAGGGCAGGGCCGCG | 17 | 4413 |
| HSV1-UL30-1476 | + | GGAGGGCAGGGCCGCGG | 17 | 4414 |
| HSV1-UL30-1477 | + | GAGGGCAGGGCCGCGGG | 17 | 4415 |
| HSV1-UL30-2628 | + | GCAGGGCCGCGGGGGGG | 17 | 5627 |
| HSV1-UL30-2629 | + | GGGGGCGGGCUCGUCCC | 17 | 5628 |
| HSV1-UL30-2630 | + | GGGGCGGGCUCGUCCCC | 17 | 5629 |
| HSV1-UL30-2631 | + | GCGGCGGCGUCUAGCUC | 17 | 5630 |
| HSV1-UL30-2632 | + | CGGCGGCGUCUAGCUCG | 17 | 5631 |
| HSV1-UL30-2633 | + | GCGGCGUCUAGCUCGCG | 17 | 5632 |
| HSV1-UL30-2634 | + | ACCGUCUCCUCUACCUC | 17 | 5633 |
| HSV1-UL30-2635 | + | UCCUCUACCUCGCGGGU | 17 | 5634 |
| HSV1-UL30-2636 | + | CUGGGCCACGAUCACGU | 17 | 5635 |
| HSV1-UL30-2637 | + | UGGGCCACGAUCACGUA | 17 | 5636 |
| HSV1-UL30-2638 | + | ACGGGAUCCGGUCCUUG | 17 | 5637 |
| HSV1-UL30-2639 | + | GAUCCGGUCCUUGAUGG | 17 | 5638 |
| HSV1-UL30-2640 | + | AUCCGGUCCUUGAUGGA | 17 | 5639 |
| HSV1-UL30-2641 | + | GACGGGACCUGCGCGCG | 17 | 5640 |
| HSV1-UL30-2642 | + | CCUGCGCGCGGCGGGCC | 17 | 5641 |
| HSV1-UL30-2643 | + | UUGUAAUACACCGUCAG | 17 | 5642 |
| HSV1-UL30-2644 | + | CUUGUUGGUGUACGCGC | 17 | 5643 |
| HSV1-UL30-2645 | + | GUCUGCUCAGUUCGGCG | 17 | 5644 |
| HSV1-UL30-2646 | + | CUGCUCAGUUCGGCGGU | 17 | 5645 |
| HSV1-UL30-2647 | + | GCGGUGAGGACAAAGUC | 17 | 5646 |
| HSV1-UL30-2648 | + | GUCCUGGAUGUCCCUCU | 17 | 5647 |
| HSV1-UL30-2649 | + | GGGUCGGUGAUGCGCCG | 17 | 5648 |
| HSV1-UL30-2650 | + | UGCGCCGAUGGGCGUCU | 17 | 5649 |
| HSV1-UL30-2651 | + | CGCCGAUGGGCGUCUAC | 17 | 5650 |
| HSV1-UL30-2652 | + | CGUCUACGAGGACGGCC | 17 | 5651 |
| HSV1-UL30-2653 | + | CGAACGCCUGCAGUCCC | 17 | 5652 |
| HSV1-UL30-2654 | + | GCCUGCAGUCCCUCGGG | 17 | 5653 |
| HSV1-UL30-2655 | + | CCUGCAGUCCCUCGGGC | 17 | 5654 |
| HSV1-UL30-2656 | + | GCGCCAGCCACUCCUCC | 17 | 5655 |
| HSV1-UL30-2657 | + | CGCCAGCCACUCCUCCG | 17 | 5656 |
| HSV1-UL30-2658 | + | CUAACGCGGCGGCCGCU | 17 | 5657 |
| HSV1-UL30-2659 | + | UAACGCGGCGGCCGCUC | 17 | 5658 |
| HSV1-UL30-2660 | + | UAAAACAGCAGGUCGAC | 17 | 5659 |
| HSV1-UL30-2661 | + | GCAGGUCGACCAGGGCC | 17 | 5660 |
| HSV1-UL30-2662 | + | CAGGUCGACCAGGGCCC | 17 | 5661 |
| HSV1-UL30-2663 | + | CCAGAUCCACGCCCUUG | 17 | 5662 |
| HSV1-UL30-2664 | + | CGAUCAGCAGCAGCUUG | 17 | 5663 |
| HSV1-UL30-2665 | + | UGAACGUCUUUUCGCAC | 17 | 5664 |
| HSV1-UL30-2666 | + | UUUCGCACUCGAGUUUG | 17 | 5665 |
| HSV1-UL30-2667 | + | UUCGCACUCGAGUUUGA | 17 | 5666 |
| HSV1-UL30-2668 | + | UCGCACUCGAGUUUGAU | 17 | 5667 |
| HSV1-UL30-2669 | + | CGCACUCGAGUUUGAUG | 17 | 5668 |
| HSV1-UL30-2670 | + | UCGAGUUUGAUGGGGGG | 17 | 5669 |
| HSV1-UL30-2671 | + | GGGCAGAAACAGCGCGC | 17 | 5670 |
| HSV1-UL30-2672 | + | CCGUCAGCCCGGCGGCC | 17 | 5671 |
| HSV1-UL30-2673 | + | CGCGGCACAGCACAAAG | 17 | 5672 |
| HSV1-UL30-1554 | + | GCGGCACAGCACAAAGA | 17 | 4492 |
| HSV1-UL30-2675 | + | CCCCGUAGAUGAUGCGC | 17 | 5673 |
| HSV1-UL30-2676 | + | CCCGUAGAUGAUGCGCA | 17 | 5674 |
| HSV1-UL30-2677 | + | AGAUGAUGCGCAUGGAA | 17 | 5675 |
| HSV1-UL30-2678 | + | UGCGCAUGGAAUAGGGC | 17 | 5676 |
| HSV1-UL30-2679 | + | GCGCAUGGAAUAGGGCC | 17 | 5677 |
| HSV1-UL30-2680 | + | CGCAUGGAAUAGGGCCC | 17 | 5678 |
| HSV1-UL30-2681 | + | GCGCAUGUCGGCCGCCU | 17 | 5679 |
| HSV1-UL30-2682 | + | CGCAUGUCGGCCGCCUC | 17 | 5680 |
| HSV1-UL30-2683 | + | GCAUGUCGGCCGCCUCC | 17 | 5681 |
| HSV1-UL30-2684 | + | GCCUCCGGGAAAUCGGC | 17 | 5682 |
| HSV1-UL30-2685 | + | CCUCCGGGAAAUCGGCC | 17 | 5683 |
| HSV1-UL30-2686 | + | AAUCGGCCAGGAGCUGU | 17 | 5684 |
| HSV1-UL30-2687 | + | AAGGCCGCCCAGCGCGC | 17 | 5685 |
| HSV1-UL30-2688 | + | CGCGCGUGGACGUACUC | 17 | 5686 |
| HSV1-UL30-2689 | + | GGACGUACUCGCGGGUC | 17 | 5687 |
| HSV1-UL30-2690 | + | GCAACGUGCAGGCACGG | 17 | 5688 |
| HSV1-UL30-2691 | + | CAACGUGCAGGCACGGC | 17 | 5689 |
| HSV1-UL30-2692 | + | GUCCGUGCUGCACUCCC | 17 | 5690 |
| HSV1-UL30-2693 | + | UCCCGUGAACCCGUACA | 17 | 5691 |
| HSV1-UL30-2694 | + | GCGGCCUGCUGCUUGUC | 17 | 5692 |
| HSV1-UL30-2695 | + | CGGCCUGCUGCUUGUCC | 17 | 5693 |
| HSV1-UL30-2696 | + | CCAGGAGCACGGCCUCC | 17 | 5694 |
| HSV1-UL30-2697 | + | CAGGAGCACGGCCUCCU | 17 | 5695 |
| HSV1-UL30-2698 | + | GCCUCCUCGGGGCUGCU | 17 | 5696 |
| HSV1-UL30-2699 | + | CCUCCUCGGGGCUGCUC | 17 | 5697 |
| HSV1-UL30-2700 | + | CUCCUCGGGGCUGCUCU | 17 | 5698 |
| HSV1-UL30-2701 | + | UCCUCGGGGCUGCUCUG | 17 | 5699 |
| HSV1-UL30-2702 | + | GCUGCUCUGGGGAAUCC | 17 | 5700 |
| HSV1-UL30-2703 | + | CUCUGGGGAAUCCGCGA | 17 | 5701 |
| HSV1-UL30-2704 | + | GGAUCUGCUUUCGCAUG | 17 | 5702 |
| HSV1-UL30-2705 | + | AUGGCGAGCCAGUCCCG | 17 | 5703 |
| HSV1-UL30-2706 | + | UGGCGAGCCAGUCCCGC | 17 | 5704 |
| HSV1-UL30-2707 | + | GCGAGCCAGUCCCGCAG | 17 | 5705 |
| HSV1-UL30-2708 | + | AGUCCCGCAGGAGGAUG | 17 | 5706 |
| HSV1-UL30-2709 | + | UCCCGCAGGAGGAUGCU | 17 | 5707 |
| HSV1-UL30-2710 | + | CCCGCAGGAGGAUGCUG | 17 | 5708 |
| HSV1-UL30-2711 | + | AGGAGGCUCUCUCGCAC | 17 | 5709 |
| HSV1-UL30-2712 | + | CUCGCACGUGAGCCUUG | 17 | 5710 |
| HSV1-UL30-2713 | + | GCACGUGAGCCUUGACG | 17 | 5711 |
| HSV1-UL30-2714 | + | GCCACUGCGUCGGCCCU | 17 | 5712 |
| HSV1-UL30-2715 | + | CCACUGCGUCGGCCCUC | 17 | 5713 |
| HSV1-UL30-2716 | + | CACUGCGUCGGCCCUCA | 17 | 5714 |
| HSV1-UL30-2717 | + | CUGCGUCGGCCCUCAGG | 17 | 5715 |
| HSV1-UL30-2718 | + | CCCUCAGGGAGAGCGUG | 17 | 5716 |
| HSV1-UL30-2719 | + | GUGCUGAAGCACAGGUU | 17 | 5717 |
| HSV1-UL30-2720 | + | AAGCACAGGUUGUGGGC | 17 | 5718 |
| HSV1-UL30-2721 | + | UGUGGGCCUGGAUGAUG | 17 | 5719 |
| HSV1-UL30-2722 | + | GUGGGCCUGGAUGAUGC | 17 | 5720 |
| HSV1-UL30-2723 | + | GGUACAGGCUGGCAAAG | 17 | 5721 |
| HSV1-UL30-2724 | + | CAAAGUCGAACACCACC | 17 | 5722 |
| HSV1-UL30-2725 | + | AAAGUCGAACACCACCA | 17 | 5723 |
| HSV1-UL30-2726 | + | ACCACCACGGGGUUCAC | 17 | 5724 |
| HSV1-UL30-2727 | + | CGGGGUUCACGUGAAAC | 17 | 5725 |
| HSV1-UL30-2728 | + | GGGGUUCACGUGAAACC | 17 | 5726 |
| HSV1-UL30-2729 | + | UCACGUGAAACCCGGAA | 17 | 5727 |
| HSV1-UL30-2730 | + | CACGUGAAACCCGGAAG | 17 | 5728 |
| HSV1-UL30-2731 | + | AACCCGGAAGUGGGGUC | 17 | 5729 |
| HSV1-UL30-2732 | + | GGCCGCUCCUCGUCCUC | 17 | 5730 |
| HSV1-UL30-2733 | + | GUCCUCCCGGGCUGCGG | 17 | 5731 |
| HSV1-UL30-2734 | + | GGGCUGCGGCCGGACGC | 17 | 5732 |
| HSV1-UL30-2735 | + | GCGCCCCUAAAUCGCCC | 17 | 5733 |
| HSV1-UL30-2736 | + | CGCCCCUGGGUGUCCGG | 17 | 5734 |
| HSV1-UL30-2737 | + | AGCAGGCACGUAAAGAC | 17 | 5735 |
| HSV1-UL30-2738 | + | UGGCCGUCGUAGAUGGU | 17 | 5736 |
| HSV1-UL30-2739 | + | CGCCAAGCGCGCGACGG | 17 | 5737 |
| HSV1-UL30-2740 | + | CCAAGCGCGCGACGGCC | 17 | 5738 |
| HSV1-UL30-2741 | + | ACGGCCGAGAGCUCCAG | 17 | 5739 |
| HSV1-UL30-2742 | + | CGGCCGAGAGCUCCAGA | 17 | 5740 |
| HSV1-UL30-2743 | + | AACAGCUGGCCCACCAG | 17 | 5741 |
| HSV1-UL30-2744 | + | ACAGCUGGCCCACCAGC | 17 | 5742 |
| HSV1-UL30-2745 | + | CAGCUGGCCCACCAGCA | 17 | 5743 |
| HSV1-UL30-2746 | + | CGAUCACCCCGCGUUGC | 17 | 5744 |
| HSV1-UL30-2747 | + | GCCCGGCGGCGUAGUAG | 17 | 5745 |
| HSV1-UL30-2748 | + | CCCGGCGGCGUAGUAGG | 17 | 5746 |
| HSV1-UL30-2749 | + | CCGGCGGCGUAGUAGGC | 17 | 5747 |
| HSV1-UL30-2750 | + | UCCUUCUUCUUGUCCUU | 17 | 5748 |
| HSV1-UL30-2751 | + | CGGCUUCGGCCACGGCG | 17 | 5749 |
| HSV1-UL30-2752 | + | GGCGUUGAGCUUGUAGC | 17 | 5750 |
| HSV1-UL30-2753 | + | CGUUGAGCUUGUAGCUC | 17 | 5751 |
| HSV1-UL30-2754 | + | UUUAUCUUGCUGCGCUU | 17 | 5752 |
| HSV1-UL30-2755 | + | UUAUCUUGCUGCGCUUC | 17 | 5753 |
| HSV1-UL30-2756 | + | UGGCCUAUGUCCCACAC | 17 | 5754 |
| HSV1-UL30-2757 | + | AUGCGGCCGUACCCGUC | 17 | 5755 |
| HSV1-UL30-2758 | + | UGCGGCCGUACCCGUCC | 17 | 5756 |
| HSV1-UL30-2759 | + | GCGGCCGUACCCGUCCA | 17 | 5757 |
| HSV1-UL30-2760 | + | CGGCCGUACCCGUCCAG | 17 | 5758 |
| HSV1-UL30-2761 | + | CCGUCAGCUUGGCCAGC | 17 | 5759 |
| HSV1-UL30-2762 | + | UCAGCUUGGCCAGCAAG | 17 | 5760 |
| HSV1-UL30-2763 | + | CCAGCAAGAAGGGCCAG | 17 | 5761 |
| HSV1-UL30-2764 | + | UGAUGUUGUACCCGGUC | 17 | 5762 |
| HSV1-UL30-2765 | + | UGUACCCGGUCACGAAC | 17 | 5763 |
| HSV1-UL30-2766 | + | GUACCCGGUCACGAACU | 17 | 5764 |
| HSV1-UL30-2767 | + | GGGCCGUACUGUUUCAC | 17 | 5765 |
| HSV1-UL30-2768 | + | ACUGUUUCACAAGGGUC | 17 | 5766 |
| HSV1-UL30-2769 | + | UGAAGGCCAACAGCAUC | 17 | 5767 |
| HSV1-UL30-2770 | + | GCAUCUCGAAUUCGCUG | 17 | 5768 |
| HSV1-UL30-2771 | + | AAUUCGCUGUCGAAUUC | 17 | 5769 |
| HSV1-UL30-2772 | + | UGUCGAAUUCCAGAACC | 17 | 5770 |
| HSV1-UL30-2773 | + | AUUCCAGAACCACGGGC | 17 | 5771 |
| HSV1-UL30-2774 | + | GCCGCCAGCUCGUUCAG | 17 | 5772 |
| HSV1-UL30-2775 | + | CCGCCAGCUCGUUCAGG | 17 | 5773 |
| HSV1-UL30-2776 | + | GCUCGUUCAGGUGGGAU | 17 | 5774 |
| HSV1-UL30-2777 | + | CUCGUUCAGGUGGGAUU | 17 | 5775 |
| HSV1-UL30-2778 | + | UCGUUCAGGUGGGAUUC | 17 | 5776 |
| HSV1-UL30-2779 | + | CGUUCAGGUGGGAUUCG | 17 | 5777 |
| HSV1-UL30-2780 | + | GGGAUUCGGGGAGGUCG | 17 | 5778 |
| HSV1-UL30-2781 | + | GGAUUCGGGGAGGUCGC | 17 | 5779 |
| HSV1-UL30-2782 | + | CGGGGAGGUCGCAGGAA | 17 | 5780 |
| HSV1-UL30-2783 | + | GAGGUCGCAGGAACCGA | 17 | 5781 |
| HSV1-UL30-2784 | + | CAGGAACCGAGCGAAAA | 17 | 5782 |
| HSV1-UL30-1694 | + | AGGAACCGAGCGAAAAC | 17 | 4632 |
| HSV1-UL30-2786 | + | GAACCGAGCGAAAACAG | 17 | 5783 |
| HSV1-UL30-2787 | + | AACAGGAGGACGUGCUC | 17 | 5784 |
| HSV1-UL30-2788 | + | CGUGCUCCAGGGCGGUG | 17 | 5785 |
| HSV1-UL30-2789 | + | CGGUGGUGGACAGGUCG | 17 | 5786 |
| HSV1-UL30-2790 | + | ACAGGUCGUAGAGCAGA | 17 | 5787 |
| HSV1-UL30-2791 | + | UAGAGCAGACAGGAUAU | 17 | 5788 |
| HSV1-UL30-2792 | + | CUGGAUGACCAGGUCCU | 17 | 5789 |
| HSV1-UL30-2793 | + | CUCCGGGUGCCCGGCCA | 17 | 5790 |
| HSV1-UL30-2794 | + | UCCGGGUGCCCGGCCAC | 17 | 5791 |
| HSV1-UL30-2795 | + | CCGCCUUGCAUUCGAUA | 17 | 5792 |
| HSV1-UL30-2796 | + | AUUCGAUAUCGAAGCAC | 17 | 5793 |
| HSV1-UL30-2797 | + | AGUUAAACUCGACGUCG | 17 | 5794 |
| HSV1-UL30-2798 | + | CGACGUCGCUGGAUGUC | 17 | 5795 |
| HSV1-UL30-2799 | + | GGAUGUCCCGAAGGCCA | 17 | 5796 |
| HSV1-UL30-2800 | + | GAUGUCCCGAAGGCCAU | 17 | 5797 |
| HSV1-UL30-2801 | + | GCCAUCGGGGCCGCCGG | 17 | 5798 |
| HSV1-UL30-2802 | + | UGUUGUUCCGGCCCGGU | 17 | 5799 |
| HSV1-UL30-2803 | + | GUUUGAGACGGUACCAG | 17 | 5800 |
| HSV1-UL30-2804 | + | GGUACCAGCCGAAGGUG | 17 | 5801 |
| HSV1-UL30-2805 | + | AGCCGAAGGUGACGAAC | 17 | 5802 |
| HSV1-UL30-2806 | + | GCCGAAGGUGACGAACC | 17 | 5803 |
| HSV1-UL30-2807 | + | ACGAACCCGGGGUUGUC | 17 | 5804 |
| HSV1-UL30-2808 | + | ACCCGGGGUUGUCCAGG | 17 | 5805 |
| HSV1-UL30-2809 | + | GGGUUGUCCAGGAUGAA | 17 | 5806 |
| HSV1-UL30-2810 | + | CUCGUACUUCUUGAUGG | 17 | 5807 |
| HSV1-UL30-2811 | + | ACUUCUUGAUGGCCGGG | 17 | 5808 |
| HSV1-UL30-2812 | + | GACAGCACACGCCCGCU | 17 | 5809 |
| HSV1-UL30-2813 | + | ACGUAGACGCGGUAAAA | 17 | 5810 |
| HSV1-UL30-2814 | + | AGACGCGGUAAAACAGA | 17 | 5811 |
| HSV1-UL30-2815 | + | GACGCGGUAAAACAGAG | 17 | 5812 |
| HSV1-UL30-2816 | + | GCUCCACCACCUCCGCC | 17 | 5813 |
| HSV1-UL30-2817 | + | CCGCCUCGAAGUGGUCC | 17 | 5814 |
| HSV1-UL30-2818 | + | CGCCUCGAAGUGGUCCG | 17 | 5815 |
| HSV1-UL30-2819 | + | UGGUCCGCGGAGAUGCC | 17 | 5816 |
| HSV1-UL30-2820 | + | GGUCCGCGGAGAUGCCG | 17 | 5817 |
| HSV1-UL30-2821 | + | GCCGCGGAACGACGCGC | 17 | 5818 |
| HSV1-UL30-1747 | + | CCGCGGAACGACGCGCC | 17 | 4685 |
| HSV1-UL30-1748 | + | CGCGGAACGACGCGCCC | 17 | 4686 |
| HSV1-UL30-2824 | + | GCGCCCGGGGACUCGCG | 17 | 5819 |
| HSV1-UL30-2825 | + | CCGCGGCCAUGCGCUCG | 17 | 5820 |
| HSV1-UL30-2826 | + | GCGCUCGCAGAGAUCUC | 17 | 5821 |
| HSV1-UL30-2827 | + | CGCUCGCAGAGAUCUCG | 17 | 5822 |
| HSV1-UL30-2828 | + | UGCCGCGUGCCGUAAAC | 17 | 5823 |
| HSV1-UL30-2829 | + | GGCCACCCGGUGGCCUU | 17 | 5824 |
| HSV1-UL30-2830 | + | GCCACCCGGUGGCCUUC | 17 | 5825 |
| HSV1-UL30-2831 | + | CCUUCCGGAGUCAGGCC | 17 | 5826 |
| HSV1-UL30-2832 | + | CUUCCGGAGUCAGGCCC | 17 | 5827 |

**Table** 2F provides exemplary targeting domains for knocking out the *UL30* gene selected according to the first tier parameters. The targeting domains are selected based on location within first 500bp of the coding sequence of the *UL30* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2F**

| 1st Tier | | | | |
|---|---|---|---|---|
| gRNA Name | DNA Strand | Targeting Domain | Target Site Length | Seq ID |
| HSV1-UL30-419 | - | ACUAUAGCGAAUGCGAUGAA | 20 | 5828 |
| HSV1-UL30-420 | - | AUAGCGAAUGCGAUGAA | 17 | 5829 |

**Table 2G** provides targeting domains for knocking out the *UL30* gene selected according to the second tier parameters. The targeting domains are selected based on location within the coding sequence (but downstream of the first 500bp) of the *UL30* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2G**

| 2nd Tier | | | | |
|---|---|---|---|---|
| gRNA Name | DNA Strand | Targeting Domain | Target Site Length | Seq ID |
| HSV1-UL30-1857 | - | CAUGGUGAACAUCGACAUGU | 20 | 4907 |
| HSV1-UL30-2834 | - | GUGAUCGGCGAGUACUGCAU | 20 | 5830 |
| HSV1-UL30-2835 | - | UUAUUCUGCCGGACACCCAG | 20 | 5831 |
| HSV1-UL30-2836 | - | CAUGCGAAAGCAGAUCCGCU | 20 | 5832 |
| HSV1-UL30-2837 | - | GCGGCCUUCGAACAGCUCCU | 20 | 5833 |
| HSV1-UL30-2139 | - | GGUGAACAUCGACAUGU | 17 | 5172 |
| HSV1-UL30-2839 | - | AUCGGCGAGUACUGCAU | 17 | 5834 |
| HSV1-UL30-2840 | - | UUCUGCCGGACACCCAG | 17 | 5835 |
| HSV1-UL30-2841 | - | GCGAAAGCAGAUCCGCU | 17 | 5836 |
| HSV1-UL30-2842 | - | GCCUUCGAACAGCUCCU | 17 | 5837 |
| HSV1-UL30-2525 | + | CUGGCCGCCAGCUCGUUCAG | 20 | 5531 |
| HSV1-UL30-2774 | + | GCCGCCAGCUCGUUCAG | 17 | 5772 |

**Table** 3A provides exemplary targeting domains for knocking out the *UL48* gene selected according to first tier parameters. The targeting domains are selected based on location within the first 500bp of the coding sequence of the *UL48* gene and orthogonality against the human genome. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3A**

| 1st Tier | | | | |
|---|---|---|---|---|
| gRNA Name | DNA Strand | Targeting Domain | Target Site Length | Seq ID |
| HSV1-UL48-8 | - | CGGGGGUCCCAAAAACACCC | 20 | 5838 |
| HSV1-UL48-9 | - | GGGUCCCAAAAACACCCCGG | 20 | 5839 |
| HSV1-UL48-10 | - | GGCCCCCCCGCUGUACGCAA | 20 | 5840 |
| HSV1-UL48-11 | - | GCCCCCCCGCUGUACGCAAC | 20 | 5841 |
| HSV1-UL48-12 | - | CCCCCCCGCUGUACGCAACG | 20 | 5842 |
| HSV1-UL48-13 | - | CGCAACGGGGCGCCUGAGCC | 20 | 5843 |
| HSV1-UL48-14 | - | UAACCGUCUCCUCGACGACU | 20 | 5844 |
| HSV1-UL48-15 | - | AACCGUCUCCUCGACGACUU | 20 | 5845 |
| HSV1-UL48-16 | - | CGACGACUUGGGCUUUAGCG | 20 | 5846 |
| HSV1-UL48-17 | - | GACGACUUGGGCUUUAGCGC | 20 | 5847 |
| HSV1-UL48-126 | - | GGGUCCCAAAAACACCC | 17 | 5848 |
| HSV1-UL48-127 | - | UCCCAAAAACACCCCGG | 17 | 5849 |
| HSV1-UL48-128 | - | CCCCCCGCUGUACGCAA | 17 | 5850 |
| HSV1-UL48-129 | - | CCCCCGCUGUACGCAAC | 17 | 5851 |
| HSV1-UL48-130 | - | CCCCGCUGUACGCAACG | 17 | 5852 |
| HSV1-UL48-131 | - | AACGGGGCGCCUGAGCC | 17 | 5853 |
| HSV1-UL48-132 | - | CCGUCUCCUCGACGACU | 17 | 5854 |
| HSV1-UL48-133 | - | CGUCUCCUCGACGACUU | 17 | 5855 |
| HSV1-UL48-134 | - | CGACUUGGGCUUUAGCG | 17 | 5856 |
| HSV1-UL48-135 | - | GACUUGGGCUUUAGCGC | 17 | 5857 |
| HSV1-UL48-19 | - | CAUGCUCGAUACCUGGAACG | 20 | 5858 |
| HSV1-UL48-20 | - | CUGGAACGAGGAUCUGUUUU | 20 | 5859 |
| HSV1-UL48-21 | - | CGACCAACGCCGACCUGUAC | 20 | 5860 |
| HSV1-UL48-22 | - | GACCAACGCCGACCUGUACC | 20 | 5861 |
| HSV1-UL48-23 | - | AUCAACGCUGCCCAGCGAUG | 20 | 5862 |
| HSV1-UL48-24 | - | AACGCUGCCCAGCGAUGUGG | 20 | 5863 |
| HSV1-UL48-25 | - | UGCCCAGCGAUGUGGUGGAA | 20 | 5864 |
| HSV1-UL48-137 | - | GCUCGAUACCUGGAACG | 17 | 5865 |
| HSV1-UL48-138 | - | GAACGAGGAUCUGUUUU | 17 | 5866 |
| HSV1-UL48-139 | - | CCAACGCCGACCUGUAC | 17 | 5867 |
| HSV1-UL48-140 | - | CAACGCCGACCUGUACC | 17 | 5868 |
| HSV1-UL48-141 | - | AACGCUGCCCAGCGAUG | 17 | 5869 |
| HSV1-UL48-142 | - | GCUGCCCAGCGAUGUGG | 17 | 5870 |
| HSV1-UL48-143 | - | CCAGCGAUGUGGUGGAA | 17 | 5871 |
| HSV1-UL48-31 | - | CAUUCGCGCCCACGGCGACG | 20 | 5872 |
| HSV1-UL48-32 | - | CGUGGCCUUCCCUACGCUUC | 20 | 5873 |
| HSV1-UL48-33 | - | ACGCUUCCGGCCACCCGCGA | 20 | 5874 |
| HSV1-UL48-34 | - | CCGGCCACCCGCGACGGCCU | 20 | 5875 |
| HSV1-UL48-35 | - | CGGCCACCCGCGACGGCCUC | 20 | 5876 |
| HSV1-UL48-36 | - | GUUUCUUCCACGCCGAGCUA | 20 | 5877 |
| HSV1-UL48-37 | - | UUUCUUCCACGCCGAGCUAC | 20 | 5878 |
| HSV1-UL48-38 | - | UCCACGCCGAGCUACGGGCG | 20 | 5879 |
| HSV1-UL48-39 | - | CCACGCCGAGCUACGGGCGC | 20 | 5880 |
| HSV1-UL48-149 | - | UCGCGCCCACGGCGACG | 17 | 5881 |
| HSV1-UL48-150 | - | GGCCUUCCCUACGCUUC | 17 | 5882 |
| HSV1-UL48-151 | - | CUUCCGGCCACCCGCGA | 17 | 5883 |
| HSV1-UL48-152 | - | GCCACCCGCGACGGCCU | 17 | 5884 |
| HSV1-UL48-153 | - | CCACCCGCGACGGCCUC | 17 | 5885 |
| HSV1-UL48-154 | - | UCUUCCACGCCGAGCUA | 17 | 5886 |
| HSV1-UL48-155 | - | CUUCCACGCCGAGCUAC | 17 | 5887 |
| HSV1-UL48-156 | - | ACGCCGAGCUACGGGCG | 17 | 5888 |
| HSV1-UL48-157 | - | CGCCGAGCUACGGGCGC | 17 | 5889 |
| HSV1-UL48-41 | + | CUCCUCCCGCGCCCGUAGCU | 20 | 5890 |
| HSV1-UL48-43 | + | GCGCUUCGUAGUAGAGCCCG | 20 | 5891 |
| HSV1-UL48-45 | + | GUAGAGCCCGAGGCCGUCGC | 20 | 5892 |
| HSV1-UL48-159 | + | CUCCCGCGCCCGUAGCU | 17 | 5893 |
| HSV1-UL48-161 | + | CUUCGUAGUAGAGCCCG | 17 | 5894 |
| HSV1-UL48-163 | + | GAGCCCGAGGCCGUCGC | 17 | 5895 |
| HSV1-UL48-48 | + | UCGCGGGUGGCCGGAAGCGU | 20 | 5896 |
| HSV1-UL48-166 | + | CGGGUGGCCGGAAGCGU | 17 | 5897 |
| HSV1-UL48-51 | + | UAGGGAAGGCCACGUCGCCG | 20 | 5898 |
| HSV1-UL48-52 | + | AGGGAAGGCCACGUCGCCGU | 20 | 5899 |
| HSV1-UL48-53 | + | CGUGGGCGCGAAUGUCGAUU | 20 | 5900 |
| HSV1-UL48-54 | + | GUGGGCGCGAAUGUCGAUUU | 20 | 5901 |
| HSV1-UL48-169 | + | GGAAGGCCACGUCGCCG | 17 | 5902 |
| HSV1-UL48-170 | + | GAAGGCCACGUCGCCGU | 17 | 5903 |
| HSV1-UL48-171 | + | GGGCGCGAAUGUCGAUU | 17 | 5904 |
| HSV1-UL48-172 | + | GGCGCGAAUGUCGAUUU | 17 | 5905 |
| HSV1-UL48-58 | + | CCCCCAUUCCACCACAUCGC | 20 | 5906 |
| HSV1-UL48-176 | + | CCAUUCCACCACAUCGC | 17 | 5907 |
| HSV1-UL48-64 | + | CUCCCGGUACAGGUCGGCGU | 20 | 5908 |
| HSV1-UL48-65 | + | CGGUACAGGUCGGCGUUGGU | 20 | 5909 |
| HSV1-UL48-66 | + | CGAAAACAGAUCCUCGUUCC | 20 | 5910 |
| HSV1-UL48-67 | + | CUCGUUCCAGGUAUCGAGCA | 20 | 5911 |
| HSV1-UL48-68 | + | AUCGAGCAUGGUACAUAGCG | 20 | 5912 |
| HSV1-UL48-69 | + | UCGAGCAUGGUACAUAGCGC | 20 | 5913 |
| HSV1-UL48-70 | + | CGAGCAUGGUACAUAGCGCG | 20 | 5914 |
| HSV1-UL48-71 | + | CGCUAAAGCCCAAGUCGUCG | 20 | 5915 |
| HSV1-UL48-182 | + | CCGGUACAGGUCGGCGU | 17 | 5916 |
| HSV1-UL48-183 | + | UACAGGUCGGCGUUGGU | 17 | 5917 |
| HSV1-UL48-184 | + | AAACAGAUCCUCGUUCC | 17 | 5918 |
| HSV1-UL48-185 | + | GUUCCAGGUAUCGAGCA | 17 | 5919 |
| HSV1-UL48-186 | + | GAGCAUGGUACAUAGCG | 17 | 5920 |
| HSV1-UL48-187 | + | AGCAUGGUACAUAGCGC | 17 | 5921 |
| HSV1-UL48-188 | + | GCAUGGUACAUAGCGCG | 17 | 5922 |
| HSV1-UL48-189 | + | UAAAGCCCAAGUCGUCG | 17 | 5923 |
| HSV1-UL48-73 | + | CGUCGAGGAGACGGUUAAAG | 20 | 5924 |
| HSV1-UL48-74 | + | GUCGAGGAGACGGUUAAAGA | 20 | 5925 |
| HSV1-UL48-191 | + | CGAGGAGACGGUUAAAG | 17 | 5926 |
| HSV1-UL48-192 | + | GAGGAGACGGUUAAAGA | 17 | 5927 |
| HSV1-UL48-76 | + | GAGACGGUUAAAGAGGGCGG | 20 | 5928 |
| HSV1-UL48-77 | + | AGACGGUUAAAGAGGGCGGC | 20 | 5929 |
| HSV1-UL48-78 | + | GACGGUUAAAGAGGGCGGCG | 20 | 5930 |
| HSV1-UL48-79 | + | ACGGUUAAAGAGGGCGGCGG | 20 | 5931 |
| HSV1-UL48-80 | + | CGGUUAAAGAGGGCGGCGGG | 20 | 5932 |
| HSV1-UL48-194 | + | ACGGUUAAAGAGGGCGG | 17 | 5933 |
| HSV1-UL48-195 | + | CGGUUAAAGAGGGCGGC | 17 | 5934 |
| HSV1-UL48-196 | + | GGUUAAAGAGGGCGGCG | 17 | 5935 |
| HSV1-UL48-197 | + | GUUAAAGAGGGCGGCGG | 17 | 5936 |
| HSV1-UL48-198 | + | UUAAAGAGGGCGGCGGG | 17 | 5937 |
| HSV1-UL48-97 | + | GCGCCCCGUUGCGUACAGCG | 20 | 5938 |
| HSV1-UL48-98 | + | CGCCCCGUUGCGUACAGCGG | 20 | 5939 |
| HSV1-UL48-99 | + | GCCCCGUUGCGUACAGCGGG | 20 | 5940 |
| HSV1-UL48-100 | + | CCCCGUUGCGUACAGCGGGG | 20 | 5941 |
| HSV1-UL48-215 | + | CCCCGUUGCGUACAGCG | 17 | 5942 |
| HSV1-UL48-216 | + | CCCGUUGCGUACAGCGG | 17 | 5943 |
| HSV1-UL48-217 | + | CCGUUGCGUACAGCGGG | 17 | 5944 |
| HSV1-UL48-218 | + | CGUUGCGUACAGCGGGG | 17 | 5945 |
| HSV1-UL48-102 | + | CGUACAGCGGGGGGGCCGCC | 20 | 5946 |
| HSV1-UL48-103 | + | GUACAGCGGGGGGGCCGCCG | 20 | 5947 |
| HSV1-UL48-104 | + | GGGGGCCGCCGGGGUGUUUU | 20 | 5948 |
| HSV1-UL48-105 | + | GGGGCCGCCGGGGUGUUUUU | 20 | 5949 |
| HSV1-UL48-220 | + | ACAGCGGGGGGGCCGCC | 17 | 5950 |
| HSV1-UL48-221 | + | CAGCGGGGGGGCCGCCG | 17 | 5951 |
| HSV1-UL48-222 | + | GGCCGCCGGGGUGUUUU | 17 | 5952 |
| HSV1-UL48-223 | + | GCCGCCGGGGUGUUUUU | 17 | 5953 |
| HSV1-UL48-107 | + | GUGUUUUUGGGACCCCCGGC | 20 | 5954 |
| HSV1-UL48-225 | + | UUUUUGGGACCCCCGGC | 17 | 5955 |
| HSV1-UL48-108 | + | UGUUUUUGGGACCCCCGGCC | 20 | 5956 |
| HSV1-UL48-226 | + | UUUUGGGACCCCCGGCC | 17 | 5957 |
| HSV1-UL48-118 | + | CAAACAGCUCGUCGACCAAG | 20 | 5958 |
| HSV1-UL48-236 | + | ACAGCUCGUCGACCAAG | 17 | 5959 |

**Table** 3B provides exemplary targeting domains for knocking out the *UL48* gene selected according to the second tier parameters. The targeting domains are selected based on location within the first 500bp of the coding sequence of the *UL48* gene. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3B**

| 2nd Tier | | | | |
|---|---|---|---|---|
| gRNA Name | DNA Strand | Targeting Domain | Target Site Length | Seq ID |
| HSV1-UL48-1 | - | GCUGUUUGCCGACAUGAACG | 20 | 5960 |
| HSV1-UL48-2 | - | UUUGCCGACAUGAACGCGGA | 20 | 5961 |
| HSV1-UL48-3 | - | UUCGCCACCGCCCCCCCGCC | 20 | 5962 |
| HSV1-UL48-4 | - | CCACCGCCCCCCCGCCCGGC | 20 | 5963 |
| HSV1-UL48-5 | - | CACCGCCCCCCCGCCCGGCC | 20 | 5964 |
| HSV1-UL48-6 | - | ACCGCCCCCCCGCCCGGCCG | 20 | 5965 |
| HSV1-UL48-7 | - | CCGCCCCCCCGCCCGGCCGG | 20 | 5966 |
| HSV1-UL48-18 | - | UAUGUACCAUGCUCGAUACC | 20 | 5967 |
| HSV1-UL48-26 | - | GCCCAGCGAUGUGGUGGAAU | 20 | 5968 |
| HSV1-UL48-27 | - | CCCAGCGAUGUGGUGGAAUG | 20 | 5969 |
| HSV1-UL48-28 | - | CCAGCGAUGUGGUGGAAUGG | 20 | 5970 |
| HSV1-UL48-29 | - | CAGCGAUGUGGUGGAAUGGG | 20 | 5971 |
| HSV1-UL48-30 | - | CAAAUCGACAUUCGCGCCCA | 20 | 5972 |
| HSV1-UL48-40 | - | CGCCGAGCUACGGGCGCGGG | 20 | 5973 |
| HSV1-UL48-119 | - | GUUUGCCGACAUGAACG | 17 | 5974 |
| HSV1-UL48-120 | - | GCCGACAUGAACGCGGA | 17 | 5975 |
| HSV1-UL48-121 | - | GCCACCGCCCCCCCGCC | 17 | 5976 |
| HSV1-UL48-122 | - | CCGCCCCCCCGCCCGGC | 17 | 5977 |
| HSV1-UL48-123 | - | CGCCCCCCCGCCCGGCC | 17 | 5978 |
| HSV1-UL48-124 | - | GCCCCCCCGCCCGGCCG | 17 | 5979 |
| HSV1-UL48-125 | - | CCCCCCCGCCCGGCCGG | 17 | 5980 |
| HSV1-UL48-136 | - | GUACCAUGCUCGAUACC | 17 | 5981 |
| HSV1-UL48-144 | - | CAGCGAUGUGGUGGAAU | 17 | 5982 |
| HSV1-UL48-145 | - | AGCGAUGUGGUGGAAUG | 17 | 5983 |
| HSV1-UL48-146 | - | GCGAUGUGGUGGAAUGG | 17 | 5984 |
| HSV1-UL48-147 | - | CGAUGUGGUGGAAUGGG | 17 | 5985 |
| HSV1-UL48-148 | - | AUCGACAUUCGCGCCCA | 17 | 5986 |
| HSV1-UL48-158 | - | CGAGCUACGGGCGCGGG | 17 | 5987 |
| HSV1-UL48-42 | + | CCCGCGCCCGUAGCUCGGCG | 20 | 5988 |
| HSV1-UL48-44 | + | AGUAGAGCCCGAGGCCGUCG | 20 | 5989 |
| HSV1-UL48-46 | + | GAGCCCGAGGCCGUCGCGGG | 20 | 5990 |
| HSV1-UL48-47 | + | CCGAGGCCGUCGCGGGUGGC | 20 | 5991 |
| HSV1-UL48-49 | + | CGCGGGUGGCCGGAAGCGUA | 20 | 5992 |
| HSV1-UL48-50 | + | GGUGGCCGGAAGCGUAGGGA | 20 | 5993 |
| HSV1-UL48-55 | + | AAUGUCGAUUUGGGUGCGUU | 20 | 5994 |
| HSV1-UL48-56 | + | AUGUCGAUUUGGGUGCGUUC | 20 | 5995 |
| HSV1-UL48-57 | + | UGUCGAUUUGGGUGCGUUCG | 20 | 5996 |
| HSV1-UL48-59 | + | CCCCAUUCCACCACAUCGCU | 20 | 5997 |
| HSV1-UL48-60 | + | CAUCGCUGGGCAGCGUUGAU | 20 | 5998 |
| HSV1-UL48-61 | + | UUGAUAGGAAUUUACACUCC | 20 | 5999 |
| HSV1-UL48-62 | + | GGAAUUUACACUCCCGGUAC | 20 | 6000 |
| HSV1-UL48-63 | + | UUUACACUCCCGGUACAGGU | 20 | 6001 |
| HSV1-UL48-72 | + | AGCCCAAGUCGUCGAGGAGA | 20 | 6002 |
| HSV1-UL48-75 | + | GAGGAGACGGUUAAAGAGGG | 20 | 6003 |
| HSV1-UL48-81 | + | GGUUAAAGAGGGCGGCGGGG | 20 | 6004 |
| HSV1-UL48-82 | + | AAAGAGGGCGGCGGGGGGGA | 20 | 6005 |
| HSV1-UL48-83 | + | AAGAGGGCGGCGGGGGGGAC | 20 | 6006 |
| HSV1-UL48-84 | + | GGCGGCGGGGGGGACGGGCA | 20 | 6007 |
| HSV1-UL48-85 | + | GCGGCGGGGGGGACGGGCAU | 20 | 6008 |
| HSV1-UL48-86 | + | GCGGGGGGGACGGGCAUGGG | 20 | 6009 |
| HSV1-UL48-87 | + | CGGGGGGGACGGGCAUGGGU | 20 | 6010 |
| HSV1-UL48-88 | + | GGGGGGGACGGGCAUGGGUG | 20 | 6011 |
| HSV1-UL48-89 | + | GGGGACGGGCAUGGGUGGGG | 20 | 6012 |
| HSV1-UL48-90 | + | GGGACGGGCAUGGGUGGGGA | 20 | 6013 |
| HSV1-UL48-91 | + | UGGGUGGGGAGGGCAUGAGC | 20 | 6014 |
| HSV1-UL48-92 | + | GGGUGGGGAGGGCAUGAGCU | 20 | 6015 |
| HSV1-UL48-93 | + | GGGAGGGCAUGAGCUGGGCC | 20 | 6016 |
| HSV1-UL48-94 | + | GCAUGAGCUGGGCCUGGCUC | 20 | 6017 |
| HSV1-UL48-95 | + | AGGCGCCCCGUUGCGUACAG | 20 | 6018 |
| HSV1-UL48-96 | + | GGCGCCCCGUUGCGUACAGC | 20 | 6019 |
| HSV1-UL48-101 | + | GCGUACAGCGGGGGGGCCGC | 20 | 6020 |
| HSV1-UL48-106 | + | CGGGGUGUUUUUGGGACCCC | 20 | 6021 |
| HSV1-UL48-109 | + | UUUUGGGACCCCCGGCCGGG | 20 | 6022 |
| HSV1-UL48-110 | + | UUUGGGACCCCCGGCCGGGC | 20 | 6023 |
| HSV1-UL48-111 | + | UUGGGACCCCCGGCCGGGCG | 20 | 6024 |
| HSV1-UL48-112 | + | UGGGACCCCCGGCCGGGCGG | 20 | 6025 |
| HSV1-UL48-113 | + | GGGACCCCCGGCCGGGCGGG | 20 | 6026 |
| HSV1-UL48-114 | + | GGACCCCCGGCCGGGCGGGG | 20 | 6027 |
| HSV1-UL48-115 | + | CCCCCGGCCGGGCGGGGGGG | 20 | 6028 |
| HSV1-UL48-116 | + | CCGGCCGGGCGGGGGGGCGG | 20 | 6029 |
| HSV1-UL48-117 | + | AGCGCCGUCCGCGUUCAUGU | 20 | 6030 |
| HSV1-UL48-160 | + | GCGCCCGUAGCUCGGCG | 17 | 6031 |
| HSV1-UL48-162 | + | AGAGCCCGAGGCCGUCG | 17 | 6032 |
| HSV1-UL48-164 | + | CCCGAGGCCGUCGCGGG | 17 | 6033 |
| HSV1-UL48-165 | + | AGGCCGUCGCGGGUGGC | 17 | 6034 |
| HSV1-UL48-167 | + | GGGUGGCCGGAAGCGUA | 17 | 6035 |
| HSV1-UL48-168 | + | GGCCGGAAGCGUAGGGA | 17 | 6036 |
| HSV1-UL48-173 | + | GUCGAUUUGGGUGCGUU | 17 | 6037 |
| HSV1-UL48-174 | + | UCGAUUUGGGUGCGUUC | 17 | 6038 |
| HSV1-UL48-175 | + | CGAUUUGGGUGCGUUCG | 17 | 6039 |
| HSV1-UL48-177 | + | CAUUCCACCACAUCGCU | 17 | 6040 |
| HSV1-UL48-178 | + | CGCUGGGCAGCGUUGAU | 17 | 6041 |
| HSV1-UL48-179 | + | AUAGGAAUUUACACUCC | 17 | 6042 |
| HSV1-UL48-180 | + | AUUUACACUCCCGGUAC | 17 | 6043 |
| HSV1-UL48-181 | + | ACACUCCCGGUACAGGU | 17 | 6044 |
| HSV1-UL48-190 | + | CCAAGUCGUCGAGGAGA | 17 | 6045 |
| HSV1-UL48-193 | + | GAGACGGUUAAAGAGGG | 17 | 6046 |
| HSV1-UL48-199 | + | UAAAGAGGGCGGCGGGG | 17 | 6047 |
| HSV1-UL48-200 | + | GAGGGCGGCGGGGGGGA | 17 | 6048 |
| HSV1-UL48-201 | + | AGGGCGGCGGGGGGGAC | 17 | 6049 |
| HSV1-UL48-202 | + | GGCGGGGGGGACGGGCA | 17 | 6050 |
| HSV1-UL48-203 | + | GCGGGGGGGACGGGCAU | 17 | 6051 |
| HSV1-UL48-204 | + | GGGGGGACGGGCAUGGG | 17 | 6052 |
| HSV1-UL48-205 | + | GGGGGACGGGCAUGGGU | 17 | 6053 |
| HSV1-UL48-206 | + | GGGGACGGGCAUGGGUG | 17 | 6054 |
| HSV1-UL48-207 | + | GACGGGCAUGGGUGGGG | 17 | 6055 |
| HSV1-UL48-208 | + | ACGGGCAUGGGUGGGGA | 17 | 6056 |
| HSV1-UL48-209 | + | GUGGGGAGGGCAUGAGC | 17 | 6057 |
| HSV1-UL48-210 | + | UGGGGAGGGCAUGAGCU | 17 | 6058 |
| HSV1-UL48-211 | + | AGGGCAUGAGCUGGGCC | 17 | 6059 |
| HSV1-UL48-212 | + | UGAGCUGGGCCUGGCUC | 17 | 6060 |
| HSV1-UL48-213 | + | CGCCCCGUUGCGUACAG | 17 | 6061 |
| HSV1-UL48-214 | + | GCCCCGUUGCGUACAGC | 17 | 6062 |
| HSV1-UL48-219 | + | UACAGCGGGGGGGCCGC | 17 | 6063 |
| HSV1-UL48-224 | + | GGUGUUUUUGGGACCCC | 17 | 6064 |
| HSV1-UL48-227 | + | UGGGACCCCCGGCCGGG | 17 | 6065 |
| HSV1-UL48-228 | + | GGGACCCCCGGCCGGGC | 17 | 6066 |
| HSV1-UL48-229 | + | GGACCCCCGGCCGGGCG | 17 | 6067 |
| HSV1-UL48-230 | + | GACCCCCGGCCGGGCGG | 17 | 6068 |
| HSV1-UL48-231 | + | ACCCCCGGCCGGGCGGG | 17 | 6069 |
| HSV1-UL48-232 | + | CCCCCGGCCGGGCGGGG | 17 | 6070 |
| HSV1-UL48-233 | + | CCGGCCGGGCGGGGGGG | 17 | 6071 |
| HSV1-UL48-234 | + | GCCGGGCGGGGGGGCGG | 17 | 6072 |
| HSV1-UL48-235 | + | GCCGUCCGCGUUCAUGU | 17 | 6073 |

**Table** 3C provides exemplary targeting domains for knocking out the *UL48* gene selected according to the third tier parameters. The targeting domains are selected based on location within the coding sequence, but downstream of the first 500bp of the *UL48* gene. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3C**

| 3rd Tier | | | | |
|---|---|---|---|---|
| gRNA Name | DNA Strand | Targeting Domain | Target Site Length | Seq ID |
| HSV1-UL48-1126 | - | CGUGUUGGCCAACUUCUGCU | 20 | 6074 |
| HSV1-UL48-237 | - | ACUUCUGCUCGGCCCUGUAC | 20 | 6075 |
| HSV1-UL48-238 | - | GGUACCUGCGCGCCAGCGUC | 20 | 6076 |
| HSV1-UL48-239 | - | CGUCCGGCAGCUGCACCGCC | 20 | 6077 |
| HSV1-UL48-240 | - | CACCGCCAGGCGCACAUGCG | 20 | 6078 |
| HSV1-UL48-241 | - | GCGCGGACGCGAUCGCGACC | 20 | 6079 |
| HSV1-UL48-242 | - | CGCGGACGCGAUCGCGACCU | 20 | 6080 |
| HSV1-UL48-243 | - | AAUGCUGCGCGCCACGAUCG | 20 | 6081 |
| HSV1-UL48-244 | - | UGCGCGCCACGAUCGCGGAC | 20 | 6082 |
| HSV1-UL48-245 | - | CUACCGAGAGACCGCUCGUC | 20 | 6083 |
| HSV1-UL48-246 | - | UUUUGACCCGCGAGAUCCUA | 20 | 6084 |
| HSV1-UL48-247 | - | UUUGACCCGCGAGAUCCUAU | 20 | 6085 |
| HSV1-UL48-248 | - | CGUACGCCGAGCAGAUGAUG | 20 | 6086 |
| HSV1-UL48-249 | - | UGACUGCCUCUGUUGCGACC | 20 | 6087 |
| HSV1-UL48-250 | - | UCUGUUGCGACCUGGAGAGC | 20 | 6088 |
| HSV1-UL48-251 | - | CCUGGAGAGCUGGCGUCAGU | 20 | 6089 |
| HSV1-UL48-252 | - | GGAGAGCUGGCGUCAGUUGG | 20 | 6090 |
| HSV1-UL48-253 | - | GAGAGCUGGCGUCAGUUGGC | 20 | 6091 |
| HSV1-UL48-254 | - | CAGCCCUUCAUGUUCGUCAA | 20 | 6092 |
| HSV1-UL48-255 | - | UCAACGGAGCGCUCACCGUC | 20 | 6093 |
| HSV1-UL48-256 | - | CAACGGAGCGCUCACCGUCC | 20 | 6094 |
| HSV1-UL48-257 | - | AACGGAGCGCUCACCGUCCG | 20 | 6095 |
| HSV1-UL48-258 | - | CGUCCGGGGAGUGCCAAUCG | 20 | 6096 |
| HSV1-UL48-259 | - | GAGUGCCAAUCGAGGCCCGC | 20 | 6097 |
| HSV1-UL48-260 | - | CAAUCGAGGCCCGCCGGCUG | 20 | 6098 |
| HSV1-UL48-261 | - | AAUCGAGGCCCGCCGGCUGC | 20 | 6099 |
| HSV1-UL48-262 | - | CGAGCACCUUAACCUCCCGC | 20 | 6100 |
| HSV1-UL48-263 | - | CCUCCCGCUGGUGCGCAGCG | 20 | 6101 |
| HSV1-UL48-264 | - | GCUGGUGCGCAGCGCGGCUA | 20 | 6102 |
| HSV1-UL48-265 | - | GGUGCGCAGCGCGGCUACGG | 20 | 6103 |
| HSV1-UL48-266 | - | AGCGCGGCUACGGAGGAGCC | 20 | 6104 |
| HSV1-UL48-267 | - | GCGCGGCUACGGAGGAGCCA | 20 | 6105 |
| HSV1-UL48-268 | - | CGCGGCUACGGAGGAGCCAG | 20 | 6106 |
| HSV1-UL48-269 | - | ACGACCCCUCCCACCCUGCA | 20 | 6107 |
| HSV1-UL48-270 | - | UCCCACCCUGCAUGGCAACC | 20 | 6108 |
| HSV1-UL48-271 | - | GGCAACCAGGCCCGCGCCUC | 20 | 6109 |
| HSV1-UL48-272 | - | GCAACCAGGCCCGCGCCUCU | 20 | 6110 |
| HSV1-UL48-273 | - | CCGCGCCUCUGGGUACUUUA | 20 | 6111 |
| HSV1-UL48-274 | - | GGUACUUUAUGGUGUUGAUU | 20 | 6112 |
| HSV1-UL48-275 | - | GUACUUUAUGGUGUUGAUUC | 20 | 6113 |
| HSV1-UL48-276 | - | GGUGUUGAUUCGGGCGAAGU | 20 | 6114 |
| HSV1-UL48-277 | - | CUUCACGACCUCGCCCUCCG | 20 | 6115 |
| HSV1-UL48-278 | - | CACGACCUCGCCCUCCGAGG | 20 | 6116 |
| HSV1-UL48-279 | - | CGCCCUCCGAGGCGGUCAUG | 20 | 6117 |
| HSV1-UL48-280 | - | GCCCUCCGAGGCGGUCAUGC | 20 | 6118 |
| HSV1-UL48-281 | - | GCGCGUACGAAAAACAAUUA | 20 | 6119 |
| HSV1-UL48-282 | - | CGCGUACGAAAAACAAUUAC | 20 | 6120 |
| HSV1-UL48-283 | - | CAAUUACGGGUCUACCAUCG | 20 | 6121 |
| HSV1-UL48-284 | - | AAUUACGGGUCUACCAUCGA | 20 | 6122 |
| HSV1-UL48-285 | - | CGAGGGCCUGCUCGAUCUCC | 20 | 6123 |
| HSV1-UL48-286 | - | GGACGACGACGCCCCCGAAG | 20 | 6124 |
| HSV1-UL48-287 | - | CGACGACGCCCCCGAAGAGG | 20 | 6125 |
| HSV1-UL48-288 | - | GACGACGCCCCCGAAGAGGC | 20 | 6126 |
| HSV1-UL48-289 | - | ACGACGCCCCCGAAGAGGCG | 20 | 6127 |
| HSV1-UL48-290 | - | CGCCCCCGAAGAGGCGGGGC | 20 | 6128 |
| HSV1-UL48-291 | - | CCCCGAAGAGGCGGGGCUGG | 20 | 6129 |
| HSV1-UL48-292 | - | GCGCCUGUCCUUUCUCCCCG | 20 | 6130 |
| HSV1-UL48-293 | - | CGCCUGUCCUUUCUCCCCGC | 20 | 6131 |
| HSV1-UL48-294 | - | ACACACGCGCAGACUGUCGA | 20 | 6132 |
| HSV1-UL48-295 | - | CCCCCCGACCGAUGUCAGCC | 20 | 6133 |
| HSV1-UL48-296 | - | CCCCCGACCGAUGUCAGCCU | 20 | 6134 |
| HSV1-UL48-297 | - | CCCCGACCGAUGUCAGCCUG | 20 | 6135 |
| HSV1-UL48-298 | - | CCCGACCGAUGUCAGCCUGG | 20 | 6136 |
| HSV1-UL48-299 | - | GGGGACGAGCUCCACUUAGA | 20 | 6137 |
| HSV1-UL48-300 | - | CGAGCUCCACUUAGACGGCG | 20 | 6138 |
| HSV1-UL48-301 | - | CCACUUAGACGGCGAGGACG | 20 | 6139 |
| HSV1-UL48-302 | - | AGACGGCGAGGACGUGGCGA | 20 | 6140 |
| HSV1-UL48-303 | - | CGCGCUAGACGAUUUCGAUC | 20 | 6141 |
| HSV1-UL48-304 | - | CGAUUUCGAUCUGGACAUGU | 20 | 6142 |
| HSV1-UL48-305 | - | GAUUUCGAUCUGGACAUGUU | 20 | 6143 |
| HSV1-UL48-306 | - | AUUUCGAUCUGGACAUGUUG | 20 | 6144 |
| HSV1-UL48-307 | - | UUUCGAUCUGGACAUGUUGG | 20 | 6145 |
| HSV1-UL48-308 | - | GAUCUGGACAUGUUGGGGGA | 20 | 6146 |
| HSV1-UL48-309 | - | AUCUGGACAUGUUGGGGGAC | 20 | 6147 |
| HSV1-UL48-310 | - | UCUGGACAUGUUGGGGGACG | 20 | 6148 |
| HSV1-UL48-311 | - | GUUGGGGGACGGGGAUUCCC | 20 | 6149 |
| HSV1-UL48-312 | - | UUGGGGGACGGGGAUUCCCC | 20 | 6150 |
| HSV1-UL48-313 | - | UGGGGGACGGGGAUUCCCCG | 20 | 6151 |
| HSV1-UL48-314 | - | GGACGGGGAUUCCCCGGGGC | 20 | 6152 |
| HSV1-UL48-315 | - | GACGGGGAUUCCCCGGGGCC | 20 | 6153 |
| HSV1-UL48-316 | - | CCCCACGACUCCGCCCCCUA | 20 | 6154 |
| HSV1-UL48-317 | - | CUCCGCCCCCUACGGCGCUC | 20 | 6155 |
| HSV1-UL48-318 | - | CCCCUACGGCGCUCUGGAUA | 20 | 6156 |
| HSV1-UL48-319 | - | CAGAUGUUUACCGAUGCCCU | 20 | 6157 |
| HSV1-UL48-320 | - | GCCCUUGGAAUUGACGAGUA | 20 | 6158 |
| HSV1-UL48-321 | - | CUUGGAAUUGACGAGUACGG | 20 | 6159 |
| HSV1-UL48-322 | - | UUGGAAUUGACGAGUACGGU | 20 | 6160 |
| HSV1-UL48-323 | + | CACCGUACUCGUCAAUUCCA | 20 | 6161 |
| HSV1-UL48-324 | + | ACCGUACUCGUCAAUUCCAA | 20 | 6162 |
| HSV1-UL48-325 | + | CUCGUCAAUUCCAAGGGCAU | 20 | 6163 |
| HSV1-UL48-326 | + | CAUCUGCUCAAACUCGAAGU | 20 | 6164 |
| HSV1-UL48-327 | + | GGCCAUAUCCAGAGCGCCGU | 20 | 6165 |
| HSV1-UL48-328 | + | GCCAUAUCCAGAGCGCCGUA | 20 | 6166 |
| HSV1-UL48-329 | + | CCAUAUCCAGAGCGCCGUAG | 20 | 6167 |
| HSV1-UL48-330 | + | CAUAUCCAGAGCGCCGUAGG | 20 | 6168 |
| HSV1-UL48-331 | + | AUCCAGAGCGCCGUAGGGGG | 20 | 6169 |
| HSV1-UL48-332 | + | CGCCGUAGGGGGCGGAGUCG | 20 | 6170 |
| HSV1-UL48-333 | + | GCCGUAGGGGGCGGAGUCGU | 20 | 6171 |
| HSV1-UL48-334 | + | CCGUAGGGGGCGGAGUCGUG | 20 | 6172 |
| HSV1-UL48-335 | + | CGUAGGGGGCGGAGUCGUGG | 20 | 6173 |
| HSV1-UL48-336 | + | GUAGGGGGCGGAGUCGUGGG | 20 | 6174 |
| HSV1-UL48-337 | + | GAGUCGUGGGGGGUAAAUCC | 20 | 6175 |
| HSV1-UL48-338 | + | UGGGGGGUAAAUCCCGGCCC | 20 | 6176 |
| HSV1-UL48-339 | + | GGGGGGUAAAUCCCGGCCCC | 20 | 6177 |
| HSV1-UL48-340 | + | GGGGGUAAAUCCCGGCCCCG | 20 | 6178 |
| HSV1-UL48-341 | + | AUCGAAAUCGUCUAGCGCGU | 20 | 6179 |
| HSV1-UL48-342 | + | CCACGUCCUCGCCGUCUAAG | 20 | 6180 |
| HSV1-UL48-343 | + | CUAAGUGGAGCUCGUCCCCC | 20 | 6181 |
| HSV1-UL48-344 | + | CUCGUCCCCCAGGCUGACAU | 20 | 6182 |
| HSV1-UL48-345 | + | UCCCCCAGGCUGACAUCGGU | 20 | 6183 |
| HSV1-UL48-346 | + | CCCCCAGGCUGACAUCGGUC | 20 | 6184 |
| HSV1-UL48-347 | + | CCCCAGGCUGACAUCGGUCG | 20 | 6185 |
| HSV1-UL48-348 | + | CCCAGGCUGACAUCGGUCGG | 20 | 6186 |
| HSV1-UL48-349 | + | CCAGGCUGACAUCGGUCGGG | 20 | 6187 |
| HSV1-UL48-350 | + | CAGGCUGACAUCGGUCGGGG | 20 | 6188 |
| HSV1-UL48-351 | + | CAGUCUGCGCGUGUGUCCCG | 20 | 6189 |
| HSV1-UL48-352 | + | AGUCUGCGCGUGUGUCCCGC | 20 | 6190 |
| HSV1-UL48-353 | + | GUCUGCGCGUGUGUCCCGCG | 20 | 6191 |
| HSV1-UL48-354 | + | CGUGUGUCCCGCGGGGAGAA | 20 | 6192 |
| HSV1-UL48-355 | + | GUCCCGCGGGGAGAAAGGAC | 20 | 6193 |
| HSV1-UL48-356 | + | GCGGGGAGAAAGGACAGGCG | 20 | 6194 |
| HSV1-UL48-357 | + | AGCCGCCAGCCCCGCCUCUU | 20 | 6195 |
| HSV1-UL48-358 | + | GCCGCCAGCCCCGCCUCUUC | 20 | 6196 |
| HSV1-UL48-359 | + | CCGCCAGCCCCGCCUCUUCG | 20 | 6197 |
| HSV1-UL48-360 | + | CGCCAGCCCCGCCUCUUCGG | 20 | 6198 |
| HSV1-UL48-361 | + | UCUUCGGGGGCGUCGUCGUC | 20 | 6199 |
| HSV1-UL48-362 | + | CUUCGGGGGCGUCGUCGUCC | 20 | 6200 |
| HSV1-UL48-363 | + | CGUCGUCCGGGAGAUCGAGC | 20 | 6201 |
| HSV1-UL48-364 | + | GAGAUCGAGCAGGCCCUCGA | 20 | 6202 |
| HSV1-UL48-365 | + | AAUUGUUUUUCGUACGCGCG | 20 | 6203 |
| HSV1-UL48-366 | + | GUGUUCCCGCAUGACCGCCU | 20 | 6204 |
| HSV1-UL48-367 | + | UUCCCGCAUGACCGCCUCGG | 20 | 6205 |
| HSV1-UL48-368 | + | UCCCGCAUGACCGCCUCGGA | 20 | 6206 |
| HSV1-UL48-369 | + | CAUGACCGCCUCGGAGGGCG | 20 | 6207 |
| HSV1-UL48-370 | + | GGAGGGCGAGGUCGUGAAGC | 20 | 6208 |
| HSV1-UL48-371 | + | CAACACCAUAAAGUACCCAG | 20 | 6209 |
| HSV1-UL48-372 | + | CCAUAAAGUACCCAGAGGCG | 20 | 6210 |
| HSV1-UL48-373 | + | CAUAAAGUACCCAGAGGCGC | 20 | 6211 |
| HSV1-UL48-374 | + | AGUACCCAGAGGCGCGGGCC | 20 | 6212 |
| HSV1-UL48-375 | + | CGCGGGCCUGGUUGCCAUGC | 20 | 6213 |
| HSV1-UL48-376 | + | GCGGGCCUGGUUGCCAUGCA | 20 | 6214 |
| HSV1-UL48-377 | + | GGCCUGGUUGCCAUGCAGGG | 20 | 6215 |
| HSV1-UL48-378 | + | GCCUGGUUGCCAUGCAGGGU | 20 | 6216 |
| HSV1-UL48-379 | + | UGGUUGCCAUGCAGGGUGGG | 20 | 6217 |
| HSV1-UL48-380 | + | GGUUGCCAUGCAGGGUGGGA | 20 | 6218 |
| HSV1-UL48-381 | + | GUUGCCAUGCAGGGUGGGAG | 20 | 6219 |
| HSV1-UL48-382 | + | AGGGUGGGAGGGGUCGUCAA | 20 | 6220 |
| HSV1-UL48-383 | + | GGGGUCGUCAACGGCGCCCC | 20 | 6221 |
| HSV1-UL48-384 | + | GUAGCCGCGCUGCGCACCAG | 20 | 6222 |
| HSV1-UL48-385 | + | UAGCCGCGCUGCGCACCAGC | 20 | 6223 |
| HSV1-UL48-386 | + | CCGCGCUGCGCACCAGCGGG | 20 | 6224 |
| HSV1-UL48-387 | + | UGCGCACCAGCGGGAGGUUA | 20 | 6225 |
| HSV1-UL48-388 | + | GGUUAAGGUGCUCGCGAAUG | 20 | 6226 |
| HSV1-UL48-389 | + | UGUGGUUUAGCUCCCGCAGC | 20 | 6227 |
| HSV1-UL48-390 | + | GGUUUAGCUCCCGCAGCCGG | 20 | 6228 |
| HSV1-UL48-391 | + | GUUUAGCUCCCGCAGCCGGC | 20 | 6229 |
| HSV1-UL48-392 | + | CGCAGCCGGCGGGCCUCGAU | 20 | 6230 |
| HSV1-UL48-393 | + | GGGCCUCGAUUGGCACUCCC | 20 | 6231 |
| HSV1-UL48-394 | + | CUCGAUUGGCACUCCCCGGA | 20 | 6232 |
| HSV1-UL48-395 | + | CGCUCCGUUGACGAACAUGA | 20 | 6233 |
| HSV1-UL48-396 | + | GCUCCGUUGACGAACAUGAA | 20 | 6234 |
| HSV1-UL48-397 | + | CGUUGACGAACAUGAAGGGC | 20 | 6235 |
| HSV1-UL48-398 | + | CCAACUGACGCCAGCUCUCC | 20 | 6236 |
| HSV1-UL48-399 | + | AGCUCUCCAGGUCGCAACAG | 20 | 6237 |
| HSV1-UL48-400 | + | CGCAACAGAGGCAGUCAAAC | 20 | 6238 |
| HSV1-UL48-401 | + | ACAGAGGCAGUCAAACAGGU | 20 | 6239 |
| HSV1-UL48-402 | + | CAGAGGCAGUCAAACAGGUC | 20 | 6240 |
| HSV1-UL48-403 | + | GUCGGGCCGCAUCAUCUGCU | 20 | 6241 |
| HSV1-UL48-404 | + | CAUCAUCUGCUCGGCGUACG | 20 | 6242 |
| HSV1-UL48-405 | + | GCUCGGCGUACGCGGCCCAU | 20 | 6243 |
| HSV1-UL48-406 | + | ACGCGGCCCAUAGGAUCUCG | 20 | 6244 |
| HSV1-UL48-407 | + | CGCGGCCCAUAGGAUCUCGC | 20 | 6245 |
| HSV1-UL48-408 | + | CAAAACACGCGCCAGACGAG | 20 | 6246 |
| HSV1-UL48-409 | + | GCGCCAGACGAGCGGUCUCU | 20 | 6247 |
| HSV1-UL48-410 | + | GUAGUACCUGUCCGCGAUCG | 20 | 6248 |
| HSV1-UL48-411 | + | UGGCGCGCAGCAUUUCUCCC | 20 | 6249 |
| HSV1-UL48-412 | + | CGCGUCCGCGCAUGUGCGCC | 20 | 6250 |
| HSV1-UL48-413 | + | GUCCGCGCAUGUGCGCCUGG | 20 | 6251 |
| HSV1-UL48-414 | + | GCGCCUGGCGGUGCAGCUGC | 20 | 6252 |
| HSV1-UL48-415 | + | GCGGUGCAGCUGCCGGACGC | 20 | 6253 |
| HSV1-UL48-416 | + | GCUGCCGGACGCUGGCGCGC | 20 | 6254 |
| HSV1-UL48-417 | + | GGACGCUGGCGCGCAGGUAC | 20 | 6255 |
| HSV1-UL48-418 | + | UGGCGCGCAGGUACCGGUAC | 20 | 6256 |
| HSV1-UL48-419 | + | GGCGCGCAGGUACCGGUACA | 20 | 6257 |
| HSV1-UL48-420 | + | GUACAGGGCCGAGCAGAAGU | 20 | 6258 |
| HSV1-UL48-421 | + | CGAGCAGAAGUUGGCCAACA | 20 | 6259 |
| HSV1-UL48-422 | - | GUUGGCCAACUUCUGCU | 17 | 6260 |
| HSV1-UL48-423 | - | UCUGCUCGGCCCUGUAC | 17 | 6261 |
| HSV1-UL48-424 | - | ACCUGCGCGCCAGCGUC | 17 | 6262 |
| HSV1-UL48-425 | - | CCGGCAGCUGCACCGCC | 17 | 6263 |
| HSV1-UL48-426 | - | CGCCAGGCGCACAUGCG | 17 | 6264 |
| HSV1-UL48-427 | - | CGGACGCGAUCGCGACC | 17 | 6265 |
| HSV1-UL48-428 | - | GGACGCGAUCGCGACCU | 17 | 6266 |
| HSV1-UL48-429 | - | GCUGCGCGCCACGAUCG | 17 | 6267 |
| HSV1-UL48-430 | - | GCGCCACGAUCGCGGAC | 17 | 6268 |
| HSV1-UL48-431 | - | CCGAGAGACCGCUCGUC | 17 | 6269 |
| HSV1-UL48-432 | - | UGACCCGCGAGAUCCUA | 17 | 6270 |
| HSV1-UL48-433 | - | GACCCGCGAGAUCCUAU | 17 | 6271 |
| HSV1-UL48-434 | - | ACGCCGAGCAGAUGAUG | 17 | 6272 |
| HSV1-UL48-435 | - | CUGCCUCUGUUGCGACC | 17 | 6273 |
| HSV1-UL48-436 | - | GUUGCGACCUGGAGAGC | 17 | 6274 |
| HSV1-UL48-437 | - | GGAGAGCUGGCGUCAGU | 17 | 6275 |
| HSV1-UL48-438 | - | GAGCUGGCGUCAGUUGG | 17 | 6276 |
| HSV1-UL48-439 | - | AGCUGGCGUCAGUUGGC | 17 | 6277 |
| HSV1-UL48-440 | - | CCCUUCAUGUUCGUCAA | 17 | 6278 |
| HSV1-UL48-441 | - | ACGGAGCGCUCACCGUC | 17 | 6279 |
| HSV1-UL48-442 | - | CGGAGCGCUCACCGUCC | 17 | 6280 |
| HSV1-UL48-443 | - | GGAGCGCUCACCGUCCG | 17 | 6281 |
| HSV1-UL48-444 | - | CCGGGGAGUGCCAAUCG | 17 | 6282 |
| HSV1-UL48-445 | - | UGCCAAUCGAGGCCCGC | 17 | 6283 |
| HSV1-UL48-446 | - | UCGAGGCCCGCCGGCUG | 17 | 6284 |
| HSV1-UL48-447 | - | CGAGGCCCGCCGGCUGC | 17 | 6285 |
| HSV1-UL48-448 | - | GCACCUUAACCUCCCGC | 17 | 6286 |
| HSV1-UL48-449 | - | CCCGCUGGUGCGCAGCG | 17 | 6287 |
| HSV1-UL48-450 | - | GGUGCGCAGCGCGGCUA | 17 | 6288 |
| HSV1-UL48-451 | - | GCGCAGCGCGGCUACGG | 17 | 6289 |
| HSV1-UL48-452 | - | GCGGCUACGGAGGAGCC | 17 | 6290 |
| HSV1-UL48-453 | - | CGGCUACGGAGGAGCCA | 17 | 6291 |
| HSV1-UL48-454 | - | GGCUACGGAGGAGCCAG | 17 | 6292 |
| HSV1-UL48-455 | - | ACCCCUCCCACCCUGCA | 17 | 6293 |
| HSV1-UL48-456 | - | CACCCUGCAUGGCAACC | 17 | 6294 |
| HSV1-UL48-457 | - | AACCAGGCCCGCGCCUC | 17 | 6295 |
| HSV1-UL48-458 | - | ACCAGGCCCGCGCCUCU | 17 | 6296 |
| HSV1-UL48-459 | - | CGCCUCUGGGUACUUUA | 17 | 6297 |
| HSV1-UL48-460 | - | ACUUUAUGGUGUUGAUU | 17 | 6298 |
| HSV1-UL48-461 | - | CUUUAUGGUGUUGAUUC | 17 | 6299 |
| HSV1-UL48-462 | - | GUUGAUUCGGGCGAAGU | 17 | 6300 |
| HSV1-UL48-463 | - | CACGACCUCGCCCUCCG | 17 | 6301 |
| HSV1-UL48-464 | - | GACCUCGCCCUCCGAGG | 17 | 6302 |
| HSV1-UL48-465 | - | CCUCCGAGGCGGUCAUG | 17 | 6303 |
| HSV1-UL48-466 | - | CUCCGAGGCGGUCAUGC | 17 | 6304 |
| HSV1-UL48-467 | - | CGUACGAAAAACAAUUA | 17 | 6305 |
| HSV1-UL48-468 | - | GUACGAAAAACAAUUAC | 17 | 6306 |
| HSV1-UL48-469 | - | UUACGGGUCUACCAUCG | 17 | 6307 |
| HSV1-UL48-470 | - | UACGGGUCUACCAUCGA | 17 | 6308 |
| HSV1-UL48-471 | - | GGGCCUGCUCGAUCUCC | 17 | 6309 |
| HSV1-UL48-472 | - | CGACGACGCCCCCGAAG | 17 | 6310 |
| HSV1-UL48-473 | - | CGACGCCCCCGAAGAGG | 17 | 6311 |
| HSV1-UL48-474 | - | GACGCCCCCGAAGAGGC | 17 | 6312 |
| HSV1-UL48-475 | - | ACGCCCCCGAAGAGGCG | 17 | 6313 |
| HSV1-UL48-476 | - | CCCCGAAGAGGCGGGGC | 17 | 6314 |
| HSV1-UL48-477 | - | CGAAGAGGCGGGGCUGG | 17 | 6315 |
| HSV1-UL48-478 | - | CCUGUCCUUUCUCCCCG | 17 | 6316 |
| HSV1-UL48-479 | - | CUGUCCUUUCUCCCCGC | 17 | 6317 |
| HSV1-UL48-480 | - | CACGCGCAGACUGUCGA | 17 | 6318 |
| HSV1-UL48-481 | - | CCCGACCGAUGUCAGCC | 17 | 6319 |
| HSV1-UL48-482 | - | CCGACCGAUGUCAGCCU | 17 | 6320 |
| HSV1-UL48-483 | - | CGACCGAUGUCAGCCUG | 17 | 6321 |
| HSV1-UL48-484 | - | GACCGAUGUCAGCCUGG | 17 | 6322 |
| HSV1-UL48-485 | - | GACGAGCUCCACUUAGA | 17 | 6323 |
| HSV1-UL48-486 | - | GCUCCACUUAGACGGCG | 17 | 6324 |
| HSV1-UL48-487 | - | CUUAGACGGCGAGGACG | 17 | 6325 |
| HSV1-UL48-488 | - | CGGCGAGGACGUGGCGA | 17 | 6326 |
| HSV1-UL48-489 | - | GCUAGACGAUUUCGAUC | 17 | 6327 |
| HSV1-UL48-490 | - | UUUCGAUCUGGACAUGU | 17 | 6328 |
| HSV1-UL48-491 | - | UUCGAUCUGGACAUGUU | 17 | 6329 |
| HSV1-UL48-492 | - | UCGAUCUGGACAUGUUG | 17 | 6330 |
| HSV1-UL48-493 | - | CGAUCUGGACAUGUUGG | 17 | 6331 |
| HSV1-UL48-494 | - | CUGGACAUGUUGGGGGA | 17 | 6332 |
| HSV1-UL48-495 | - | UGGACAUGUUGGGGGAC | 17 | 6333 |
| HSV1-UL48-496 | - | GGACAUGUUGGGGGACG | 17 | 6334 |
| HSV1-UL48-497 | - | GGGGGACGGGGAUUCCC | 17 | 6335 |
| HSV1-UL48-498 | - | GGGGACGGGGAUUCCCC | 17 | 6336 |
| HSV1-UL48-499 | - | GGGACGGGGAUUCCCCG | 17 | 6337 |
| HSV1-UL48-500 | - | CGGGGAUUCCCCGGGGC | 17 | 6338 |
| HSV1-UL48-501 | - | GGGGAUUCCCCGGGGCC | 17 | 6339 |
| HSV1-UL48-502 | - | CACGACUCCGCCCCCUA | 17 | 6340 |
| HSV1-UL48-503 | - | CGCCCCCUACGGCGCUC | 17 | 6341 |
| HSV1-UL48-504 | - | CUACGGCGCUCUGGAUA | 17 | 6342 |
| HSV1-UL48-505 | - | AUGUUUACCGAUGCCCU | 17 | 6343 |
| HSV1-UL48-506 | - | CUUGGAAUUGACGAGUA | 17 | 6344 |
| HSV1-UL48-507 | - | GGAAUUGACGAGUACGG | 17 | 6345 |
| HSV1-UL48-508 | - | GAAUUGACGAGUACGGU | 17 | 6346 |
| HSV1-UL48-509 | + | CGUACUCGUCAAUUCCA | 17 | 6347 |
| HSV1-UL48-510 | + | GUACUCGUCAAUUCCAA | 17 | 6348 |
| HSV1-UL48-511 | + | GUCAAUUCCAAGGGCAU | 17 | 6349 |
| HSV1-UL48-512 | + | CUGCUCAAACUCGAAGU | 17 | 6350 |
| HSV1-UL48-513 | + | CAUAUCCAGAGCGCCGU | 17 | 6351 |
| HSV1-UL48-514 | + | AUAUCCAGAGCGCCGUA | 17 | 6352 |
| HSV1-UL48-515 | + | UAUCCAGAGCGCCGUAG | 17 | 6353 |
| HSV1-UL48-516 | + | AUCCAGAGCGCCGUAGG | 17 | 6354 |
| HSV1-UL48-517 | + | CAGAGCGCCGUAGGGGG | 17 | 6355 |
| HSV1-UL48-518 | + | CGUAGGGGGCGGAGUCG | 17 | 6356 |
| HSV1-UL48-519 | + | GUAGGGGGCGGAGUCGU | 17 | 6357 |
| HSV1-UL48-520 | + | UAGGGGGCGGAGUCGUG | 17 | 6358 |
| HSV1-UL48-521 | + | AGGGGGCGGAGUCGUGG | 17 | 6359 |
| HSV1-UL48-522 | + | GGGGGCGGAGUCGUGGG | 17 | 6360 |
| HSV1-UL48-523 | + | UCGUGGGGGGUAAAUCC | 17 | 6361 |
| HSV1-UL48-524 | + | GGGGUAAAUCCCGGCCC | 17 | 6362 |
| HSV1-UL48-525 | + | GGGUAAAUCCCGGCCCC | 17 | 6363 |
| HSV1-UL48-526 | + | GGUAAAUCCCGGCCCCG | 17 | 6364 |
| HSV1-UL48-527 | + | GAAAUCGUCUAGCGCGU | 17 | 6365 |
| HSV1-UL48-528 | + | CGUCCUCGCCGUCUAAG | 17 | 6366 |
| HSV1-UL48-529 | + | AGUGGAGCUCGUCCCCC | 17 | 6367 |
| HSV1-UL48-530 | + | GUCCCCCAGGCUGACAU | 17 | 6368 |
| HSV1-UL48-531 | + | CCCAGGCUGACAUCGGU | 17 | 6369 |
| HSV1-UL48-532 | + | CCAGGCUGACAUCGGUC | 17 | 6370 |
| HSV1-UL48-533 | + | CAGGCUGACAUCGGUCG | 17 | 6371 |
| HSV1-UL48-534 | + | AGGCUGACAUCGGUCGG | 17 | 6372 |
| HSV1-UL48-535 | + | GGCUGACAUCGGUCGGG | 17 | 6373 |
| HSV1-UL48-536 | + | GCUGACAUCGGUCGGGG | 17 | 6374 |
| HSV1-UL48-537 | + | UCUGCGCGUGUGUCCCG | 17 | 6375 |
| HSV1-UL48-538 | + | CUGCGCGUGUGUCCCGC | 17 | 6376 |
| HSV1-UL48-539 | + | UGCGCGUGUGUCCCGCG | 17 | 6377 |
| HSV1-UL48-540 | + | GUGUCCCGCGGGGAGAA | 17 | 6378 |
| HSV1-UL48-541 | + | CCGCGGGGAGAAAGGAC | 17 | 6379 |
| HSV1-UL48-542 | + | GGGAGAAAGGACAGGCG | 17 | 6380 |
| HSV1-UL48-543 | + | CGCCAGCCCCGCCUCUU | 17 | 6381 |
| HSV1-UL48-544 | + | GCCAGCCCCGCCUCUUC | 17 | 6382 |
| HSV1-UL48-545 | + | CCAGCCCCGCCUCUUCG | 17 | 6383 |
| HSV1-UL48-546 | + | CAGCCCCGCCUCUUCGG | 17 | 6384 |
| HSV1-UL48-547 | + | UCGGGGGCGUCGUCGUC | 17 | 6385 |
| HSV1-UL48-548 | + | CGGGGGCGUCGUCGUCC | 17 | 6386 |
| HSV1-UL48-549 | + | CGUCCGGGAGAUCGAGC | 17 | 6387 |
| HSV1-UL48-550 | + | AUCGAGCAGGCCCUCGA | 17 | 6388 |
| HSV1-UL48-551 | + | UGUUUUUCGUACGCGCG | 17 | 6389 |
| HSV1-UL48-552 | + | UUCCCGCAUGACCGCCU | 17 | 6390 |
| HSV1-UL48-553 | + | CCGCAUGACCGCCUCGG | 17 | 6391 |
| HSV1-UL48-554 | + | CGCAUGACCGCCUCGGA | 17 | 6392 |
| HSV1-UL48-555 | + | GACCGCCUCGGAGGGCG | 17 | 6393 |
| HSV1-UL48-556 | + | GGGCGAGGUCGUGAAGC | 17 | 6394 |
| HSV1-UL48-557 | + | CACCAUAAAGUACCCAG | 17 | 6395 |
| HSV1-UL48-558 | + | UAAAGUACCCAGAGGCG | 17 | 6396 |
| HSV1-UL48-559 | + | AAAGUACCCAGAGGCGC | 17 | 6397 |
| HSV1-UL48-560 | + | ACCCAGAGGCGCGGGCC | 17 | 6398 |
| HSV1-UL48-561 | + | GGGCCUGGUUGCCAUGC | 17 | 6399 |
| HSV1-UL48-562 | + | GGCCUGGUUGCCAUGCA | 17 | 6400 |
| HSV1-UL48-563 | + | CUGGUUGCCAUGCAGGG | 17 | 6401 |
| HSV1-UL48-564 | + | UGGUUGCCAUGCAGGGU | 17 | 6402 |
| HSV1-UL48-565 | + | UUGCCAUGCAGGGUGGG | 17 | 6403 |
| HSV1-UL48-566 | + | UGCCAUGCAGGGUGGGA | 17 | 6404 |
| HSV1-UL48-567 | + | GCCAUGCAGGGUGGGAG | 17 | 6405 |
| HSV1-UL48-568 | + | GUGGGAGGGGUCGUCAA | 17 | 6406 |
| HSV1-UL48-569 | + | GUCGUCAACGGCGCCCC | 17 | 6407 |
| HSV1-UL48-570 | + | GCCGCGCUGCGCACCAG | 17 | 6408 |
| HSV1-UL48-571 | + | CCGCGCUGCGCACCAGC | 17 | 6409 |
| HSV1-UL48-572 | + | CGCUGCGCACCAGCGGG | 17 | 6410 |
| HSV1-UL48-573 | + | GCACCAGCGGGAGGUUA | 17 | 6411 |
| HSV1-UL48-574 | + | UAAGGUGCUCGCGAAUG | 17 | 6412 |
| HSV1-UL48-575 | + | GGUUUAGCUCCCGCAGC | 17 | 6413 |
| HSV1-UL48-576 | + | UUAGCUCCCGCAGCCGG | 17 | 6414 |
| HSV1-UL48-577 | + | UAGCUCCCGCAGCCGGC | 17 | 6415 |
| HSV1-UL48-578 | + | AGCCGGCGGGCCUCGAU | 17 | 6416 |
| HSV1-UL48-579 | + | CCUCGAUUGGCACUCCC | 17 | 6417 |
| HSV1-UL48-580 | + | GAUUGGCACUCCCCGGA | 17 | 6418 |
| HSV1-UL48-581 | + | UCCGUUGACGAACAUGA | 17 | 6419 |
| HSV1-UL48-582 | + | CCGUUGACGAACAUGAA | 17 | 6420 |
| HSV1-UL48-583 | + | UGACGAACAUGAAGGGC | 17 | 6421 |
| HSV1-UL48-584 | + | ACUGACGCCAGCUCUCC | 17 | 6422 |
| HSV1-UL48-585 | + | UCUCCAGGUCGCAACAG | 17 | 6423 |
| HSV1-UL48-586 | + | AACAGAGGCAGUCAAAC | 17 | 6424 |
| HSV1-UL48-587 | + | GAGGCAGUCAAACAGGU | 17 | 6425 |
| HSV1-UL48-588 | + | AGGCAGUCAAACAGGUC | 17 | 6426 |
| HSV1-UL48-589 | + | GGGCCGCAUCAUCUGCU | 17 | 6427 |
| HSV1-UL48-590 | + | CAUCUGCUCGGCGUACG | 17 | 6428 |
| HSV1-UL48-591 | + | CGGCGUACGCGGCCCAU | 17 | 6429 |
| HSV1-UL48-592 | + | CGGCCCAUAGGAUCUCG | 17 | 6430 |
| HSV1-UL48-593 | + | GGCCCAUAGGAUCUCGC | 17 | 6431 |
| HSV1-UL48-594 | + | AACACGCGCCAGACGAG | 17 | 6432 |
| HSV1-UL48-595 | + | CCAGACGAGCGGUCUCU | 17 | 6433 |
| HSV1-UL48-596 | + | GUACCUGUCCGCGAUCG | 17 | 6434 |
| HSV1-UL48-597 | + | CGCGCAGCAUUUCUCCC | 17 | 6435 |
| HSV1-UL48-598 | + | GUCCGCGCAUGUGCGCC | 17 | 6436 |
| HSV1-UL48-599 | + | CGCGCAUGUGCGCCUGG | 17 | 6437 |
| HSV1-UL48-600 | + | CCUGGCGGUGCAGCUGC | 17 | 6438 |
| HSV1-UL48-601 | + | GUGCAGCUGCCGGACGC | 17 | 6439 |
| HSV1-UL48-602 | + | GCCGGACGCUGGCGCGC | 17 | 6440 |
| HSV1-UL48-603 | + | CGCUGGCGCGCAGGUAC | 17 | 6441 |
| HSV1-UL48-604 | + | CGCGCAGGUACCGGUAC | 17 | 6442 |
| HSV1-UL48-605 | + | GCGCAGGUACCGGUACA | 17 | 6443 |
| HSV1-UL48-606 | + | CAGGGCCGAGCAGAAGU | 17 | 6444 |
| HSV1-UL48-607 | + | GCAGAAGUUGGCCAACA | 17 | 6445 |

**Table 3D** provides exemplary targeting domains for knocking out the *UL48* gene selected according to the first tier parameters. The targeting domains are selected based on location within first 500bp of the coding sequence of the *UL48* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3D**

| 1st Tier | | | | |
|---|---|---|---|---|
| gRNA Name | DNA Strand | Targeting Domain | Target Site Length | Seq ID |
| HSV1-UL48-608 | - | UCGACGAGCUGUUUGCCGAC | 20 | 6446 |
| HSV1-UL48-609 | - | AGCUGUUUGCCGACAUGAAC | 20 | 6447 |
| HSV1-UL48-610 | - | GCCACCGCCCCCCCGCCCGG | 20 | 6448 |
| HSV1-UL48-4 | - | CCACCGCCCCCCCGCCCGGC | 20 | 5963 |
| HSV1-UL48-5 | - | CACCGCCCCCCCGCCCGGCC | 20 | 5964 |
| HSV1- UL48-613 | - | CGGCCCCCCCGCUGUACGCA | 20 | 6449 |
| HSV1-UL48-10 | - | GGCCCCCCCGCUGUACGCAA | 20 | 5840 |
| HSV1-UL48-615 | - | CGCUGUACGCAACGGGGCGC | 20 | 6450 |
| HSV1-UL48-616 | - | UUAACCGUCUCCUCGACGAC | 20 | 6451 |
| HSV1-UL48-617 | - | UCGACGACUUGGGCUUUAGC | 20 | 6452 |
| HSV1-UL48-618 | - | CUAUGUACCAUGCUCGAUAC | 20 | 6453 |
| HSV1-UL48-18 | - | UAUGUACCAUGCUCGAUACC | 20 | 5967 |
| HSV1-UL48-620 | - | UACCAUGCUCGAUACCUGGA | 20 | 6454 |
| HSV1-UL48-621 | - | CCAUGCUCGAUACCUGGAAC | 20 | 6455 |
| HSV1-UL48-622 | - | CCGACCAACGCCGACCUGUA | 20 | 6456 |
| HSV1-UL48-21 | - | CGACCAACGCCGACCUGUAC | 20 | 5860 |
| HSV1-UL48-22 | - | GACCAACGCCGACCUGUACC | 20 | 5861 |
| HSV1-UL48-625 | - | CAACGCUGCCCAGCGAUGUG | 20 | 6457 |
| HSV1-UL48-24 | - | AACGCUGCCCAGCGAUGUGG | 20 | 5863 |
| HSV1-UL48-627 | - | CUGCCCAGCGAUGUGGUGGA | 20 | 6458 |
| HSV1-UL48-25 | - | UGCCCAGCGAUGUGGUGGAA | 20 | 5864 |
| HSV1-UL48-26 | - | GCCCAGCGAUGUGGUGGAAU | 20 | 5968 |
| HSV1-UL48-27 | - | CCCAGCGAUGUGGUGGAAUG | 20 | 5969 |
| HSV1-UL48-28 | - | CCAGCGAUGUGGUGGAAUGG | 20 | 5970 |
| HSV1-UL48-632 | - | AUGGGGGGACGCGUACGUCC | 20 | 6459 |
| HSV1-UL48-633 | - | UCCGGCCACCCGCGACGGCC | 20 | 6460 |
| HSV1-UL48-634 | - | CGACGGCCUCGGGCUCUACU | 20 | 6461 |
| HSV1-UL48-635 | - | GCUCUCUCGUUUCUUCCACG | 20 | 6462 |
| HSV1-UL48-636 | - | CGUUUCUUCCACGCCGAGCU | 20 | 6463 |
| HSV1-UL48-637 | - | UUCCACGCCGAGCUACGGGC | 20 | 6464 |
| HSV1-UL48-38 | - | UCCACGCCGAGCUACGGGCG | 20 | 5879 |
| HSV1-UL48-39 | - | CCACGCCGAGCUACGGGCGC | 20 | 5880 |
| HSV1-UL48-640 | - | ACGCCGAGCUACGGGCGCGG | 20 | 6465 |
| HSV1-UL48-40 | - | CGCCGAGCUACGGGCGCGGG | 20 | 5973 |
| HSV1-UL48-642 | - | CCGAGCUACGGGCGCGGGAG | 20 | 6466 |
| HSV1-UL48-643 | - | ACGAGCUGUUUGCCGAC | 17 | 6467 |
| HSV1-UL48-644 | - | UGUUUGCCGACAUGAAC | 17 | 6468 |
| HSV1-UL48-645 | - | ACCGCCCCCCCGCCCGG | 17 | 6469 |
| HSV1-UL48-122 | - | CCGCCCCCCCGCCCGGC | 17 | 5977 |
| HSV1-UL48-123 | - | CGCCCCCCCGCCCGGCC | 17 | 5978 |
| HSV1-UL48-648 | - | CCCCCCCGCUGUACGCA | 17 | 6470 |
| HSV1-UL48-128 | - | CCCCCCGCUGUACGCAA | 17 | 5850 |
| HSV1-UL48-650 | - | UGUACGCAACGGGGCGC | 17 | 6471 |
| HSV1-UL48-651 | - | ACCGUCUCCUCGACGAC | 17 | 6472 |
| HSV1-UL48-652 | - | ACGACUUGGGCUUUAGC | 17 | 6473 |
| HSV1-UL48-653 | - | UGUACCAUGCUCGAUAC | 17 | 6474 |
| HSV1-UL48-136 | - | GUACCAUGCUCGAUACC | 17 | 5981 |
| HSV1-UL48-655 | - | CAUGCUCGAUACCUGGA | 17 | 6475 |
| HSV1-UL48-656 | - | UGCUCGAUACCUGGAAC | 17 | 6476 |
| HSV1-UL48-657 | - | ACCAACGCCGACCUGUA | 17 | 6477 |
| HSV1-UL48-139 | - | CCAACGCCGACCUGUAC | 17 | 5867 |
| HSV1-UL48-140 | - | CAACGCCGACCUGUACC | 17 | 5868 |
| HSV1-UL48-660 | - | CGCUGCCCAGCGAUGUG | 17 | 6478 |
| HSV1-UL48-142 | - | GCUGCCCAGCGAUGUGG | 17 | 5870 |
| HSV1-UL48-662 | - | CCCAGCGAUGUGGUGGA | 17 | 6479 |
| HSV1-UL48-143 | - | CCAGCGAUGUGGUGGAA | 17 | 5871 |
| HSV1-UL48-144 | - | CAGCGAUGUGGUGGAAU | 17 | 5982 |
| HSV1-UL48-145 | - | AGCGAUGUGGUGGAAUG | 17 | 5983 |
| HSV1-UL48-146 | - | GCGAUGUGGUGGAAUGG | 17 | 5984 |
| HSV1-UL48-667 | - | GGGGGACGCGUACGUCC | 17 | 6480 |
| HSV1-UL48-668 | - | GGCCACCCGCGACGGCC | 17 | 6481 |
| HSV1-UL48-669 | - | CGGCCUCGGGCUCUACU | 17 | 6482 |
| HSV1-UL48-670 | - | CUCUCGUUUCUUCCACG | 17 | 6483 |
| HSV1-UL48-671 | - | UUCUUCCACGCCGAGCU | 17 | 6484 |
| HSV1-UL48-672 | - | CACGCCGAGCUACGGGC | 17 | 6485 |
| HSV1-UL48-156 | - | ACGCCGAGCUACGGGCG | 17 | 5888 |
| HSV1-UL48-157 | - | CGCCGAGCUACGGGCGC | 17 | 5889 |
| HSV1-UL48-675 | - | CCGAGCUACGGGCGCGG | 17 | 6486 |
| HSV1-UL48-158 | - | CGAGCUACGGGCGCGGG | 17 | 5987 |
| HSV1-UL48-677 | - | AGCUACGGGCGCGGGAG | 17 | 6487 |
| HSV1-UL48-678 | + | CGGCAAACAGCUCGUCGACC | 20 | 6488 |
| HSV1-UL48-679 | + | GGCCGGGCGGGGGGGCGGUG | 20 | 6489 |
| HSV1-UL48-112 | + | UGGGACCCCCGGCCGGGCGG | 20 | 6025 |
| HSV1-UL48-111 | + | UUGGGACCCCCGGCCGGGCG | 20 | 6024 |
| HSV1-UL48-110 | + | UUUGGGACCCCCGGCCGGGC | 20 | 6023 |
| HSV1-UL48-109 | + | UUUUGGGACCCCCGGCCGGG | 20 | 6022 |
| HSV1-UL48-684 | + | UUUUUGGGACCCCCGGCCGG | 20 | 6490 |
| HSV1-UL48-685 | + | GGUGUUUUUGGGACCCCCGG | 20 | 6491 |
| HSV1-UL48-104 | + | GGGGGCCGCCGGGGUGUUUU | 20 | 5948 |
| HSV1-UL48-687 | + | GGGGGGCCGCCGGGGUGUUU | 20 | 6492 |
| HSV1-UL48-101 | + | GCGUACAGCGGGGGGGCCGC | 20 | 6020 |
| HSV1-UL48-689 | + | UGCGUACAGCGGGGGGGCCG | 20 | 6493 |
| HSV1-UL48-98 | + | CGCCCCGUUGCGUACAGCGG | 20 | 5939 |
| HSV1-UL48-97 | + | GCGCCCCGUUGCGUACAGCG | 20 | 5938 |
| HSV1-UL48-96 | + | GGCGCCCCGUUGCGUACAGC | 20 | 6019 |
| HSV1-UL48-95 | + | AGGCGCCCCGUUGCGUACAG | 20 | 6018 |
| HSV1-UL48-694 | + | CAGGCGCCCCGUUGCGUACA | 20 | 6494 |
| HSV1- UL48-695 | + | AUGGGUGGGGAGGGCAUGAG | 20 | 6495 |
| HSV1-UL48-696 | + | CGGGCAUGGGUGGGGAGGGC | 20 | 6496 |
| HSV1-UL48-697 | + | GGGGGACGGGCAUGGGUGGG | 20 | 6497 |
| HSV1-UL48-88 | + | GGGGGGGACGGGCAUGGGUG | 20 | 6011 |
| HSV1-UL48-87 | + | CGGGGGGGACGGGCAUGGGU | 20 | 6010 |
| HSV1-UL48-86 | + | GCGGGGGGGACGGGCAUGGG | 20 | 6009 |
| HSV1-UL48-701 | + | GGCGGGGGGGACGGGCAUGG | 20 | 6498 |
| HSV1-UL48-702 | + | GGGCGGCGGGGGGGACGGGC | 20 | 6499 |
| HSV1-UL48-703 | + | UAAAGAGGGCGGCGGGGGGG | 20 | 6500 |
| HSV1-UL48-80 | + | CGGUUAAAGAGGGCGGCGGG | 20 | 5932 |
| HSV1-UL48-79 | + | ACGGUUAAAGAGGGCGGCGG | 20 | 5931 |
| HSV1-UL48-78 | + | GACGGUUAAAGAGGGCGGCG | 20 | 5930 |
| HSV1-UL48-77 | + | AGACGGUUAAAGAGGGCGGC | 20 | 5929 |
| HSV1-UL48-76 | + | GAGACGGUUAAAGAGGGCGG | 20 | 5928 |
| HSV1-UL48-709 | + | GGAGACGGUUAAAGAGGGCG | 20 | 6501 |
| HSV1-UL48-710 | + | UCGUCGAGGAGACGGUUAAA | 20 | 6502 |
| HSV1-UL48-711 | + | AGUCGUCGAGGAGACGGUUA | 20 | 6503 |
| HSV1-UL48-71 | + | CGCUAAAGCCCAAGUCGUCG | 20 | 5915 |
| HSV1-UL48-713 | + | GCGCUAAAGCCCAAGUCGUC | 20 | 6504 |
| HSV1-UL48-714 | + | CCGCGCUAAAGCCCAAGUCG | 20 | 6505 |
| HSV1-UL48-68 | + | AUCGAGCAUGGUACAUAGCG | 20 | 5912 |
| HSV1-UL48-716 | + | UAUCGAGCAUGGUACAUAGC | 20 | 6506 |
| HSV1-UL48-717 | + | ACAGAUCCUCGUUCCAGGUA | 20 | 6507 |
| HSV1-UL48-718 | + | GUCGGCGUUGGUCGGUAGCG | 20 | 6508 |
| HSV1-UL48-60 | + | CAUCGCUGGGCAGCGUUGAU | 20 | 5998 |
| HSV1-UL48-720 | + | ACAUCGCUGGGCAGCGUUGA | 20 | 6509 |
| HSV1-UL48-721 | + | CCCCCCAUUCCACCACAUCG | 20 | 6510 |
| HSV1-UL48-56 | + | AUGUCGAUUUGGGUGCGUUC | 20 | 5995 |
| HSV1-UL48-55 | + | AAUGUCGAUUUGGGUGCGUU | 20 | 5994 |
| HSV1-UL48-724 | + | GAAUGUCGAUUUGGGUGCGU | 20 | 6511 |
| HSV1-UL48-725 | + | CCGUGGGCGCGAAUGUCGAU | 20 | 6512 |
| HSV1-UL48-726 | + | AAGGCCACGUCGCCGUGGGC | 20 | 6513 |
| HSV1-UL48-727 | + | GUAGGGAAGGCCACGUCGCC | 20 | 6514 |
| HSV1-UL48-49 | + | CGCGGGUGGCCGGAAGCGUA | 20 | 5992 |
| HSV1-UL48-48 | + | UCGCGGGUGGCCGGAAGCGU | 20 | 5896 |
| HSV1-UL48-730 | + | GUCGCGGGUGGCCGGAAGCG | 20 | 6515 |
| HSV1-UL48-47 | + | CCGAGGCCGUCGCGGGUGGC | 20 | 5991 |
| HSV1-UL48-732 | + | CCCGAGGCCGUCGCGGGUGG | 20 | 6516 |
| HSV1-UL48-733 | + | UAGUAGAGCCCGAGGCCGUC | 20 | 6517 |
| HSV1-UL48-734 | + | AGAGCGCUUCGUAGUAGAGC | 20 | 6518 |
| HSV1-UL48-735 | + | AACGAGAGAGCGCUUCGUAG | 20 | 6519 |
| HSV1-UL48-736 | + | GCUCGGCGUGGAAGAAACGA | 20 | 6520 |
| HSV1-UL48-737 | + | UAGCUCGGCGUGGAAGAAAC | 20 | 6521 |
| HSV1-UL48-738 | + | CGUAGCUCGGCGUGGAAGAA | 20 | 6522 |
| HSV1-UL48-739 | + | GCGCCCGUAGCUCGGCGUGG | 20 | 6523 |
| HSV1-UL48-42 | + | CCCGCGCCCGUAGCUCGGCG | 20 | 5988 |
| HSV1-UL48-741 | + | UCCCGCGCCCGUAGCUCGGC | 20 | 6524 |
| HSV1-UL48-742 | + | CAAACAGCUCGUCGACC | 17 | 6525 |
| HSV1-UL48-743 | + | CGGGCGGGGGGGCGGUG | 17 | 6526 |
| HSV1-UL48-230 | + | GACCCCCGGCCGGGCGG | 17 | 6068 |
| HSV1-UL48-229 | + | GGACCCCCGGCCGGGCG | 17 | 6067 |
| HSV1-UL48-228 | + | GGGACCCCCGGCCGGGC | 17 | 6066 |
| HSV1-UL48-227 | + | UGGGACCCCCGGCCGGG | 17 | 6065 |
| HSV1-UL48-748 | + | UUGGGACCCCCGGCCGG | 17 | 6527 |
| HSV1-UL48-749 | + | GUUUUUGGGACCCCCGG | 17 | 6528 |
| HSV1-UL48-222 | + | GGCCGCCGGGGUGUUUU | 17 | 5952 |
| HSV1-UL48-751 | + | GGGCCGCCGGGGUGUUU | 17 | 6529 |
| HSV1-UL48-219 | + | UACAGCGGGGGGGCCGC | 17 | 6063 |
| HSV1-UL48-753 | + | GUACAGCGGGGGGGCCG | 17 | 6530 |
| HSV1-UL48-216 | + | CCCGUUGCGUACAGCGG | 17 | 5943 |
| HSV1-UL48-215 | + | CCCCGUUGCGUACAGCG | 17 | 5942 |
| HSV1-UL48-214 | + | GCCCCGUUGCGUACAGC | 17 | 6062 |
| HSV1-UL48-213 | + | CGCCCCGUUGCGUACAG | 17 | 6061 |
| HSV1-UL48-758 | + | GCGCCCCGUUGCGUACA | 17 | 6531 |
| HSV1-UL48-759 | + | GGUGGGGAGGGCAUGAG | 17 | 6532 |
| HSV1-UL48-760 | + | GCAUGGGUGGGGAGGGC | 17 | 6533 |
| HSV1-UL48-761 | + | GGACGGGCAUGGGUGGG | 17 | 6534 |
| HSV1-UL48-206 | + | GGGGACGGGCAUGGGUG | 17 | 6054 |
| HSV1-UL48-205 | + | GGGGGACGGGCAUGGGU | 17 | 6053 |
| HSV1-UL48-204 | + | GGGGGGACGGGCAUGGG | 17 | 6052 |
| HSV1-UL48-765 | + | GGGGGGGACGGGCAUGG | 17 | 6535 |
| HSV1-UL48-766 | + | CGGCGGGGGGGACGGGC | 17 | 6536 |
| HSV1-UL48-767 | + | AGAGGGCGGCGGGGGGG | 17 | 6537 |
| HSV1-UL48-198 | + | UUAAAGAGGGCGGCGGG | 17 | 5937 |
| HSV1-UL48-197 | + | GUUAAAGAGGGCGGCGG | 17 | 5936 |
| HSV1-UL48-196 | + | GGUUAAAGAGGGCGGCG | 17 | 5935 |
| HSV1-UL48-195 | + | CGGUUAAAGAGGGCGGC | 17 | 5934 |
| HSV1-UL48-194 | + | ACGGUUAAAGAGGGCGG | 17 | 5933 |
| HSV1-UL48-773 | + | GACGGUUAAAGAGGGCG | 17 | 6538 |
| HSV1-UL48-774 | + | UCGAGGAGACGGUUAAA | 17 | 6539 |
| HSV1-UL48-775 | + | CGUCGAGGAGACGGUUA | 17 | 6540 |
| HSV1-UL48-189 | + | UAAAGCCCAAGUCGUCG | 17 | 5923 |
| HSV1-UL48-777 | + | CUAAAGCCCAAGUCGUC | 17 | 6541 |
| HSV1-UL48-778 | + | CGCUAAAGCCCAAGUCG | 17 | 6542 |
| HSV1-UL48-186 | + | GAGCAUGGUACAUAGCG | 17 | 5920 |
| HSV1- UL48-780 | + | CGAGCAUGGUACAUAGC | 17 | 6543 |
| HSV1-UL48-781 | + | GAUCCUCGUUCCAGGUA | 17 | 6544 |
| HSV1-UL48-782 | + | GGCGUUGGUCGGUAGCG | 17 | 6545 |
| HSV1-UL48-178 | + | CGCUGGGCAGCGUUGAU | 17 | 6041 |
| HSV1-UL48-784 | + | UCGCUGGGCAGCGUUGA | 17 | 6546 |
| HSV1-UL48-785 | + | CCCAUUCCACCACAUCG | 17 | 6547 |
| HSV1-UL48-174 | + | UCGAUUUGGGUGCGUUC | 17 | 6038 |
| HSV1-UL48-173 | + | GUCGAUUUGGGUGCGUU | 17 | 6037 |
| HSV1-UL48-788 | + | UGUCGAUUUGGGUGCGU | 17 | 6548 |
| HSV1-UL48-789 | + | UGGGCGCGAAUGUCGAU | 17 | 6549 |
| HSV1-UL48-790 | + | GCCACGUCGCCGUGGGC | 17 | 6550 |
| HSV1-UL48-791 | + | GGGAAGGCCACGUCGCC | 17 | 6551 |
| HSV1-UL48-167 | + | GGGUGGCCGGAAGCGUA | 17 | 6035 |
| HSV1-UL48-166 | + | CGGGUGGCCGGAAGCGU | 17 | 5897 |
| HSV1-UL48-794 | + | GCGGGUGGCCGGAAGCG | 17 | 6552 |
| HSV1-UL48-165 | + | AGGCCGUCGCGGGUGGC | 17 | 6034 |
| HSV1-UL48-796 | + | GAGGCCGUCGCGGGUGG | 17 | 6553 |
| HSV1-UL48-797 | + | UAGAGCCCGAGGCCGUC | 17 | 6554 |
| HSV1-UL48-798 | + | GCGCUUCGUAGUAGAGC | 17 | 6555 |
| HSV1-UL48-799 | + | GAGAGAGCGCUUCGUAG | 17 | 6556 |
| HSV1-UL48-800 | + | CGGCGUGGAAGAAACGA | 17 | 6557 |
| HSV1-UL48-801 | + | CUCGGCGUGGAAGAAAC | 17 | 6558 |
| HSV1-UL48-802 | + | AGCUCGGCGUGGAAGAA | 17 | 6559 |
| HSV1-UL48-803 | + | CCCGUAGCUCGGCGUGG | 17 | 6560 |
| HSV1-UL48-160 | + | GCGCCCGUAGCUCGGCG | 17 | 6031 |
| HSV1-UL48-805 | + | CGCGCCCGUAGCUCGGC | 17 | 6561 |

**Table 3E** provides exemplary targeting domains for knocking out the *UL48* gene selected according to the second tier parameters. The targeting domains are selected based on location within the coding sequence, but downstream of the first 500bp of the *UL48* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3E**

| 2nd Tier | | | | |
|---|---|---|---|---|
| gRNA Name | DNA Strand | Targeting Domain | Target Site Length | Seq ID |
| HSV1-UL48-806 | - | GCACCGCCAGGCGCACAUGC | 20 | 6562 |
| HSV1-UL48-807 | - | UGCGCGGACGCGAUCGCGAC | 20 | 6563 |
| HSV1-UL48-241 | - | GCGCGGACGCGAUCGCGACC | 20 | 6079 |
| HSV1-UL48-242 | - | CGCGGACGCGAUCGCGACCU | 20 | 6080 |
| HSV1-UL48-810 | - | CGGACGCGAUCGCGACCUGG | 20 | 6564 |
| HSV1-UL48-811 | - | AAAUGCUGCGCGCCACGAUC | 20 | 6565 |
| HSV1-UL48-812 | - | ACGAUCGCGGACAGGUACUA | 20 | 6566 |
| HSV1-UL48-813 | - | GAUCGCGGACAGGUACUACC | 20 | 6567 |
| HSV1-UL48-814 | - | UUUGUAUCUAUUUUUGACCC | 20 | 6568 |
| HSV1-UL48-815 | - | UUUUUGACCCGCGAGAUCCU | 20 | 6569 |
| HSV1-UL48-816 | - | GAUCCUAUGGGCCGCGUACG | 20 | 6570 |
| HSV1-UL48-817 | - | UUGACUGCCUCUGUUGCGAC | 20 | 6571 |
| HSV1-UL48-249 | - | UGACUGCCUCUGUUGCGACC | 20 | 6087 |
| HSV1-UL48-819 | - | ACUGCCUCUGUUGCGACCUG | 20 | 6572 |
| HSV1-UL48-820 | - | UGGAGAGCUGGCGUCAGUUG | 20 | 6573 |
| HSV1-UL48-821 | - | CCAGCCCUUCAUGUUCGUCA | 20 | 6574 |
| HSV1-UL48-254 | - | CAGCCCUUCAUGUUCGUCAA | 20 | 6092 |
| HSV1-UL48-823 | - | GUCAACGGAGCGCUCACCGU | 20 | 6575 |
| HSV1-UL48-255 | - | UCAACGGAGCGCUCACCGUC | 20 | 6093 |
| HSV1-UL48-256 | - | CAACGGAGCGCUCACCGUCC | 20 | 6094 |
| HSV1-UL48-257 | - | AACGGAGCGCUCACCGUCCG | 20 | 6095 |
| HSV1-UL48-827 | - | CACCGUCCGGGGAGUGCCAA | 20 | 6576 |
| HSV1-UL48-828 | - | CCAAUCGAGGCCCGCCGGCU | 20 | 6577 |
| HSV1-UL48-260 | - | CAAUCGAGGCCCGCCGGCUG | 20 | 6098 |
| HSV1-UL48-261 | - | AAUCGAGGCCCGCCGGCUGC | 20 | 6099 |
| HSV1-UL48-831 | - | GCGGGAGCUAAACCACAUUC | 20 | 6578 |
| HSV1-UL48-832 | - | CGCUGGUGCGCAGCGCGGCU | 20 | 6579 |
| HSV1-UL48-264 | - | GCUGGUGCGCAGCGCGGCUA | 20 | 6102 |
| HSV1-UL48-834 | - | UGGUGCGCAGCGCGGCUACG | 20 | 6580 |
| HSV1-UL48-265 | - | GGUGCGCAGCGCGGCUACGG | 20 | 6103 |
| HSV1-UL48-836 | - | CAGCGCGGCUACGGAGGAGC | 20 | 6581 |
| HSV1-UL48-266 | - | AGCGCGGCUACGGAGGAGCC | 20 | 6104 |
| HSV1-UL48-838 | - | UGGCAACCAGGCCCGCGCCU | 20 | 6582 |
| HSV1-UL48-839 | - | GGGUACUUUAUGGUGUUGAU | 20 | 6583 |
| HSV1-UL48-840 | - | ACUUUAUGGUGUUGAUUCGG | 20 | 6584 |
| HSV1-UL48-841 | - | UGGUGUUGAUUCGGGCGAAG | 20 | 6585 |
| HSV1-UL48-842 | - | CAGCUUCACGACCUCGCCCU | 20 | 6586 |
| HSV1-UL48-843 | - | UCGCCCUCCGAGGCGGUCAU | 20 | 6587 |
| HSV1-UL48-279 | - | CGCCCUCCGAGGCGGUCAUG | 20 | 6117 |
| HSV1-UL48-280 | - | GCCCUCCGAGGCGGUCAUGC | 20 | 6118 |
| HSV1-UL48-846 | - | ACGCGUACAGCCGCGCGCGU | 20 | 6588 |
| HSV1-UL48-847 | - | CGCGCGUACGAAAAACAAUU | 20 | 6589 |
| HSV1-UL48-848 | - | AAACAAUUACGGGUCUACCA | 20 | 6590 |
| HSV1-UL48-849 | - | ACAAUUACGGGUCUACCAUC | 20 | 6591 |
| HSV1-UL48-850 | - | UCGAGGGCCUGCUCGAUCUC | 20 | 6592 |
| HSV1-UL48-851 | - | UCUCCCGGACGACGACGCCC | 20 | 6593 |
| HSV1-UL48-852 | - | CCCGGACGACGACGCCCCCG | 20 | 6594 |
| HSV1-UL48-853 | - | ACGACGACGCCCCCGAAGAG | 20 | 6595 |
| HSV1-UL48-287 | - | CGACGACGCCCCCGAAGAGG | 20 | 6125 |
| HSV1-UL48-855 | - | CGCGCCUGUCCUUUCUCCCC | 20 | 6596 |
| HSV1-UL48-292 | - | GCGCCUGUCCUUUCUCCCCG | 20 | 6130 |
| HSV1-UL48-857 | - | CCCCCCCGACCGAUGUCAGC | 20 | 6597 |
| HSV1-UL48-295 | - | CCCCCCGACCGAUGUCAGCC | 20 | 6133 |
| HSV1-UL48-296 | - | CCCCCGACCGAUGUCAGCCU | 20 | 6134 |
| HSV1-UL48-297 | - | CCCCGACCGAUGUCAGCCUG | 20 | 6135 |
| HSV1-UL48-861 | - | GACCGAUGUCAGCCUGGGGG | 20 | 6598 |
| HSV1-UL48-862 | - | GGACGAGCUCCACUUAGACG | 20 | 6599 |
| HSV1-UL48-863 | - | ACGAGCUCCACUUAGACGGC | 20 | 6600 |
| HSV1-UL48-864 | - | ACGCGCUAGACGAUUUCGAU | 20 | 6601 |
| HSV1-UL48-865 | - | ACGAUUUCGAUCUGGACAUG | 20 | 6602 |
| HSV1-UL48-304 | - | CGAUUUCGAUCUGGACAUGU | 20 | 6142 |
| HSV1-UL48-305 | - | GAUUUCGAUCUGGACAUGUU | 20 | 6143 |
| HSV1-UL48-306 | - | AUUUCGAUCUGGACAUGUUG | 20 | 6144 |
| HSV1-UL48-869 | - | CGAUCUGGACAUGUUGGGGG | 20 | 6603 |
| HSV1-UL48-308 | - | GAUCUGGACAUGUUGGGGGA | 20 | 6146 |
| HSV1-UL48-309 | - | AUCUGGACAUGUUGGGGGAC | 20 | 6147 |
| HSV1-UL48-872 | - | UGUUGGGGGACGGGGAUUCC | 20 | 6604 |
| HSV1-UL48-311 | - | GUUGGGGGACGGGGAUUCCC | 20 | 6149 |
| HSV1-UL48-874 | - | GGGACGGGGAUUCCCCGGGG | 20 | 6605 |
| HSV1-UL48-314 | - | GGACGGGGAUUCCCCGGGGC | 20 | 6152 |
| HSV1-UL48-876 | - | ACUCCGCCCCCUACGGCGCU | 20 | 6606 |
| HSV1-UL48-877 | - | CGCUCUGGAUAUGGCCGACU | 20 | 6607 |
| HSV1-UL48-878 | - | GGAUAUGGCCGACUUCGAGU | 20 | 6608 |
| HSV1-UL48-879 | - | GCAGAUGUUUACCGAUGCCC | 20 | 6609 |
| HSV1-UL48-319 | - | CAGAUGUUUACCGAUGCCCU | 20 | 6157 |
| HSV1-UL48-881 | - | UACCGAUGCCCUUGGAAUUG | 20 | 6610 |
| HSV1-UL48-882 | - | CCUUGGAAUUGACGAGUACG | 20 | 6611 |
| HSV1-UL48-883 | - | CCGCCAGGCGCACAUGC | 17 | 6612 |
| HSV1-UL48-884 | - | GCGGACGCGAUCGCGAC | 17 | 6613 |
| HSV1-UL48-427 | - | CGGACGCGAUCGCGACC | 17 | 6265 |
| HSV1-UL48-428 | - | GGACGCGAUCGCGACCU | 17 | 6266 |
| HSV1-UL48-887 | - | ACGCGAUCGCGACCUGG | 17 | 6614 |
| HSV1-UL48-888 | - | UGCUGCGCGCCACGAUC | 17 | 6615 |
| HSV1-UL48-889 | - | AUCGCGGACAGGUACUA | 17 | 6616 |
| HSV1-UL48-890 | - | CGCGGACAGGUACUACC | 17 | 6617 |
| HSV1-UL48-891 | - | GUAUCUAUUUUUGACCC | 17 | 6618 |
| HSV1-UL48-892 | - | UUGACCCGCGAGAUCCU | 17 | 6619 |
| HSV1-UL48-893 | - | CCUAUGGGCCGCGUACG | 17 | 6620 |
| HSV1-UL48-894 | - | ACUGCCUCUGUUGCGAC | 17 | 6621 |
| HSV1-UL48-435 | - | CUGCCUCUGUUGCGACC | 17 | 6273 |
| HSV1-UL48-896 | - | GCCUCUGUUGCGACCUG | 17 | 6622 |
| HSV1-UL48-897 | - | AGAGCUGGCGUCAGUUG | 17 | 6623 |
| HSV1-UL48-898 | - | GCCCUUCAUGUUCGUCA | 17 | 6624 |
| HSV1-UL48-440 | - | CCCUUCAUGUUCGUCAA | 17 | 6278 |
| HSV1-UL48-900 | - | AACGGAGCGCUCACCGU | 17 | 6625 |
| HSV1-UL48-441 | - | ACGGAGCGCUCACCGUC | 17 | 6279 |
| HSV1-UL48-442 | - | CGGAGCGCUCACCGUCC | 17 | 6280 |
| HSV1-UL48-443 | - | GGAGCGCUCACCGUCCG | 17 | 6281 |
| HSV1-UL48-904 | - | CGUCCGGGGAGUGCCAA | 17 | 6626 |
| HSV1-UL48-905 | - | AUCGAGGCCCGCCGGCU | 17 | 6627 |
| HSV1-UL48-446 | - | UCGAGGCCCGCCGGCUG | 17 | 6284 |
| HSV1-UL48-447 | - | CGAGGCCCGCCGGCUGC | 17 | 6285 |
| HSV1-UL48-908 | - | GGAGCUAAACCACAUUC | 17 | 6628 |
| HSV1-UL48-909 | - | UGGUGCGCAGCGCGGCU | 17 | 6629 |
| HSV1-UL48-450 | - | GGUGCGCAGCGCGGCUA | 17 | 6288 |
| HSV1-UL48-911 | - | UGCGCAGCGCGGCUACG | 17 | 6630 |
| HSV1-UL48-451 | - | GCGCAGCGCGGCUACGG | 17 | 6289 |
| HSV1-UL48-913 | - | CGCGGCUACGGAGGAGC | 17 | 6631 |
| HSV1-UL48-452 | - | GCGGCUACGGAGGAGCC | 17 | 6290 |
| HSV1-UL48-915 | - | CAACCAGGCCCGCGCCU | 17 | 6632 |
| HSV1-UL48-916 | - | UACUUUAUGGUGUUGAU | 17 | 6633 |
| HSV1-UL48-917 | - | UUAUGGUGUUGAUUCGG | 17 | 6634 |
| HSV1-UL48-918 | - | UGUUGAUUCGGGCGAAG | 17 | 6635 |
| HSV1-UL48-919 | - | CUUCACGACCUCGCCCU | 17 | 6636 |
| HSV1-UL48-920 | - | CCCUCCGAGGCGGUCAU | 17 | 6637 |
| HSV1-UL48-465 | - | CCUCCGAGGCGGUCAUG | 17 | 6303 |
| HSV1-UL48-466 | - | CUCCGAGGCGGUCAUGC | 17 | 6304 |
| HSV1-UL48-923 | - | CGUACAGCCGCGCGCGU | 17 | 6638 |
| HSV1-UL48-924 | - | GCGUACGAAAAACAAUU | 17 | 6639 |
| HSV1-UL48-925 | - | CAAUUACGGGUCUACCA | 17 | 6640 |
| HSV1-UL48-926 | - | AUUACGGGUCUACCAUC | 17 | 6641 |
| HSV1-UL48-927 | - | AGGGCCUGCUCGAUCUC | 17 | 6642 |
| HSV1-UL48-928 | - | CCCGGACGACGACGCCC | 17 | 6643 |
| HSV1-UL48-929 | - | GGACGACGACGCCCCCG | 17 | 6644 |
| HSV1-UL48-930 | - | ACGACGCCCCCGAAGAG | 17 | 6645 |
| HSV1-UL48-473 | - | CGACGCCCCCGAAGAGG | 17 | 6311 |
| HSV1-UL48-932 | - | GCCUGUCCUUUCUCCCC | 17 | 6646 |
| HSV1-UL48-478 | - | CCUGUCCUUUCUCCCCG | 17 | 6316 |
| HSV1-UL48-934 | - | CCCCGACCGAUGUCAGC | 17 | 6647 |
| HSV1-UL48-481 | - | CCCGACCGAUGUCAGCC | 17 | 6319 |
| HSV1-UL48-482 | - | CCGACCGAUGUCAGCCU | 17 | 6320 |
| HSV1-UL48-483 | - | CGACCGAUGUCAGCCUG | 17 | 6321 |
| HSV1-UL48-938 | - | CGAUGUCAGCCUGGGGG | 17 | 6648 |
| HSV1-UL48-939 | - | CGAGCUCCACUUAGACG | 17 | 6649 |
| HSV1-UL48-940 | - | AGCUCCACUUAGACGGC | 17 | 6650 |
| HSV1-UL48-941 | - | CGCUAGACGAUUUCGAU | 17 | 6651 |
| HSV1- UL48-942 | - | AUUUCGAUCUGGACAUG | 17 | 6652 |
| HSV1- UL48-490 | - | UUUCGAUCUGGACAUGU | 17 | 6328 |
| HSV1- UL48-491 | - | UUCGAUCUGGACAUGUU | 17 | 6329 |
| HSV1- UL48-492 | - | UCGAUCUGGACAUGUUG | 17 | 6330 |
| HSV1- UL48-946 | - | UCUGGACAUGUUGGGGG | 17 | 6653 |
| HSV1- UL48-494 | - | CUGGACAUGUUGGGGGA | 17 | 6332 |
| HSV1- UL48-495 | - | UGGACAUGUUGGGGGAC | 17 | 6333 |
| HSV1- UL48-949 | - | UGGGGGACGGGGAUUCC | 17 | 6654 |
| HSV1-UL48-497 | - | GGGGGACGGGGAUUCCC | 17 | 6335 |
| HSV1-UL48-951 | - | ACGGGGAUUCCCCGGGG | 17 | 6655 |
| HSV1- UL48-500 | - | CGGGGAUUCCCCGGGGC | 17 | 6338 |
| HSV1-UL48-953 | - | CCGCCCCCUACGGCGCU | 17 | 6656 |
| HSV1-UL48-954 | - | UCUGGAUAUGGCCGACU | 17 | 6657 |
| HSV1-UL48-955 | - | UAUGGCCGACUUCGAGU | 17 | 6658 |
| HSV1-UL48-956 | - | GAUGUUUACCGAUGCCC | 17 | 6659 |
| HSV1-UL48-505 | - | AUGUUUACCGAUGCCCU | 17 | 6343 |
| HSV1-UL48-958 | - | CGAUGCCCUUGGAAUUG | 17 | 6660 |
| HSV1- UL48-959 | - | UGGAAUUGACGAGUACG | 17 | 6661 |
| HSV1-UL48-960 | + | GGUACCGGUACAGGGCCGAG | 20 | 6662 |
| HSV1- UL48-961 | + | GCGCAGGUACCGGUACAGGG | 20 | 6663 |
| HSV1-UL48-962 | + | CUGGCGCGCAGGUACCGGUA | 20 | 6664 |
| HSV1-UL48-963 | + | UGCGCCUGGCGGUGCAGCUG | 20 | 6665 |
| HSV1-UL48-964 | + | AAAAACAAAACACGCGCCAG | 20 | 6666 |
| HSV1-UL48-965 | + | UACGCGGCCCAUAGGAUCUC | 20 | 6667 |
| HSV1-UL48-966 | + | UGCUCGGCGUACGCGGCCCA | 20 | 6668 |
| HSV1-UL48-967 | + | AACAGAGGCAGUCAAACAGG | 20 | 6669 |
| HSV1-UL48-968 | + | GCCAGCUCUCCAGGUCGCAA | 20 | 6670 |
| HSV1-UL48-397 | + | CGUUGACGAACAUGAAGGGC | 20 | 6235 |
| HSV1-UL48-970 | + | CCGUUGACGAACAUGAAGGG | 20 | 6671 |
| HSV1-UL48-971 | + | GCGCUCCGUUGACGAACAUG | 20 | 6672 |
| HSV1-UL48-972 | + | UGAGCGCUCCGUUGACGAAC | 20 | 6673 |
| HSV1-UL48-973 | + | GGACGGUGAGCGCUCCGUUG | 20 | 6674 |
| HSV1-UL48-974 | + | CGAUUGGCACUCCCCGGACG | 20 | 6675 |
| HSV1-UL48-975 | + | CGGGCCUCGAUUGGCACUCC | 20 | 6676 |
| HSV1-UL48-976 | + | UGGUUUAGCUCCCGCAGCCG | 20 | 6677 |
| HSV1-UL48-977 | + | AGCGGGAGGUUAAGGUGCUC | 20 | 6678 |
| HSV1-UL48-385 | + | UAGCCGCGCUGCGCACCAGC | 20 | 6223 |
| HSV1-UL48-384 | + | GUAGCCGCGCUGCGCACCAG | 20 | 6222 |
| HSV1-UL48-980 | + | CGUAGCCGCGCUGCGCACCA | 20 | 6679 |
| HSV1-UL48-379 | + | UGGUUGCCAUGCAGGGUGGG | 20 | 6217 |
| HSV1-UL48-982 | + | CUGGUUGCCAUGCAGGGUGG | 20 | 6680 |
| HSV1-UL48-378 | + | GCCUGGUUGCCAUGCAGGGU | 20 | 6216 |
| HSV1-UL48-377 | + | GGCCUGGUUGCCAUGCAGGG | 20 | 6215 |
| HSV1-UL48-985 | + | GGGCCUGGUUGCCAUGCAGG | 20 | 6681 |
| HSV1-UL48-986 | + | GCGCGGGCCUGGUUGCCAUG | 20 | 6682 |
| HSV1-UL48-987 | + | ACCAUAAAGUACCCAGAGGC | 20 | 6683 |
| HSV1-UL48-988 | + | AAUCAACACCAUAAAGUACC | 20 | 6684 |
| HSV1-UL48-989 | + | GAAUACGAGUCCAACUUCGC | 20 | 6685 |
| HSV1-UL48-990 | + | CGAGGUCGUGAAGCUGGAAU | 20 | 6686 |
| HSV1-UL48-370 | + | GGAGGGCGAGGUCGUGAAGC | 20 | 6208 |
| HSV1-UL48-992 | + | CGGAGGGCGAGGUCGUGAAG | 20 | 6687 |
| HSV1-UL48-993 | + | CCGCCUCGGAGGGCGAGGUC | 20 | 6688 |
| HSV1-UL48-994 | + | CCGCAUGACCGCCUCGGAGG | 20 | 6689 |
| HSV1-UL48-995 | + | GUUCCCGCAUGACCGCCUCG | 20 | 6690 |
| HSV1-UL48-366 | + | GUGUUCCCGCAUGACCGCCU | 20 | 6204 |
| HSV1-UL48-997 | + | CGUGUUCCCGCAUGACCGCC | 20 | 6691 |
| HSV1-UL48-998 | + | GGGCGUCGUCGUCCGGGAGA | 20 | 6692 |
| HSV1-UL48-362 | + | CUUCGGGGGCGUCGUCGUCC | 20 | 6200 |
| HSV1-UL48-361 | + | UCUUCGGGGGCGUCGUCGUC | 20 | 6199 |
| HSV1-UL48-1001 | + | CUCUUCGGGGGCGUCGUCGU | 20 | 6693 |
| HSV1-UL48-358 | + | GCCGCCAGCCCCGCCUCUUC | 20 | 6196 |
| HSV1-UL48-357 | + | AGCCGCCAGCCCCGCCUCUU | 20 | 6195 |
| HSV1-UL48-1004 | + | GAGCCGCCAGCCCCGCCUCU | 20 | 6694 |
| HSV1-UL48-356 | + | GCGGGGAGAAAGGACAGGCG | 20 | 6194 |
| HSV1-UL48-1006 | + | CGCGGGGAGAAAGGACAGGC | 20 | 6695 |
| HSV1-UL48-1007 | + | GCGUGUGUCCCGCGGGGAGA | 20 | 6696 |
| HSV1-UL48-1008 | + | CUGCGCGUGUGUCCCGCGGG | 20 | 6697 |
| HSV1-UL48-353 | + | GUCUGCGCGUGUGUCCCGCG | 20 | 6191 |
| HSV1-UL48-352 | + | AGUCUGCGCGUGUGUCCCGC | 20 | 6190 |
| HSV1-UL48-351 | + | CAGUCUGCGCGUGUGUCCCG | 20 | 6189 |
| HSV1-UL48-1012 | + | ACAGUCUGCGCGUGUGUCCC | 20 | 6698 |
| HSV1-UL48-348 | + | CCCAGGCUGACAUCGGUCGG | 20 | 6186 |
| HSV1-UL48-347 | + | CCCCAGGCUGACAUCGGUCG | 20 | 6185 |
| HSV1-UL48-346 | + | CCCCCAGGCUGACAUCGGUC | 20 | 6184 |
| HSV1-UL48-345 | + | UCCCCCAGGCUGACAUCGGU | 20 | 6183 |
| HSV1-UL48-1017 | + | GUCCCCCAGGCUGACAUCGG | 20 | 6699 |
| HSV1-UL48-342 | + | CCACGUCCUCGCCGUCUAAG | 20 | 6180 |
| HSV1-UL48-1019 | + | GCCACGUCCUCGCCGUCUAA | 20 | 6700 |
| HSV1-UL48-1020 | + | CGUCCCCCAACAUGUCCAGA | 20 | 6701 |
| HSV1-UL48-340 | + | GGGGGUAAAUCCCGGCCCCG | 20 | 6178 |
| HSV1-UL48-339 | + | GGGGGGUAAAUCCCGGCCCC | 20 | 6177 |
| HSV1-UL48-338 | + | UGGGGGGUAAAUCCCGGCCC | 20 | 6176 |
| HSV1-UL48-1024 | + | GUGGGGGGUAAAUCCCGGCC | 20 | 6702 |
| HSV1-UL48-334 | + | CCGUAGGGGGCGGAGUCGUG | 20 | 6172 |
| HSV1-UL48-333 | + | GCCGUAGGGGGCGGAGUCGU | 20 | 6171 |
| HSV1-UL48-332 | + | CGCCGUAGGGGGCGGAGUCG | 20 | 6170 |
| HSV1-UL48-1028 | + | GCGCCGUAGGGGGCGGAGUC | 20 | 6703 |
| HSV1-UL48-331 | + | AUCCAGAGCGCCGUAGGGGG | 20 | 6169 |
| HSV1-UL48-1030 | + | UAUCCAGAGCGCCGUAGGGG | 20 | 6704 |
| HSV1-UL48-328 | + | GCCAUAUCCAGAGCGCCGUA | 20 | 6166 |
| HSV1-UL48-327 | + | GGCCAUAUCCAGAGCGCCGU | 20 | 6165 |
| HSV1-UL48-1033 | + | CGGCCAUAUCCAGAGCGCCG | 20 | 6705 |
| HSV1-UL48-1034 | + | AACUCGAAGUCGGCCAUAUC | 20 | 6706 |
| HSV1-UL48-1035 | + | CGGUAAACAUCUGCUCAAAC | 20 | 6707 |
| HSV1-UL48-1036 | + | CCACCGUACUCGUCAAUUCC | 20 | 6708 |
| HSV1-UL48-1037 | + | ACCGGUACAGGGCCGAG | 17 | 6709 |
| HSV1-UL48-1038 | + | CAGGUACCGGUACAGGG | 17 | 6710 |
| HSV1-UL48-1039 | + | GCGCGCAGGUACCGGUA | 17 | 6711 |
| HSV1-UL48-1040 | + | GCCUGGCGGUGCAGCUG | 17 | 6712 |
| HSV1-UL48-1041 | + | AACAAAACACGCGCCAG | 17 | 6713 |
| HSV1-UL48-1042 | + | GCGGCCCAUAGGAUCUC | 17 | 6714 |
| HSV1-UL48-1043 | + | UCGGCGUACGCGGCCCA | 17 | 6715 |
| HSV1-UL48-1044 | + | AGAGGCAGUCAAACAGG | 17 | 6716 |
| HSV1-UL48-1045 | + | AGCUCUCCAGGUCGCAA | 17 | 6717 |
| HSV1-UL48-583 | + | UGACGAACAUGAAGGGC | 17 | 6421 |
| HSV1-UL48-1047 | + | UUGACGAACAUGAAGGG | 17 | 6718 |
| HSV1-UL48-1048 | + | CUCCGUUGACGAACAUG | 17 | 6719 |
| HSV1-UL48-1049 | + | GCGCUCCGUUGACGAAC | 17 | 6720 |
| HSV1-UL48-1050 | + | CGGUGAGCGCUCCGUUG | 17 | 6721 |
| HSV1-UL48-1051 | + | UUGGCACUCCCCGGACG | 17 | 6722 |
| HSV1-UL48-1052 | + | GCCUCGAUUGGCACUCC | 17 | 6723 |
| HSV1-UL48-1053 | + | UUUAGCUCCCGCAGCCG | 17 | 6724 |
| HSV1-UL48-1054 | + | GGGAGGUUAAGGUGCUC | 17 | 6725 |
| HSV1-UL48-571 | + | CCGCGCUGCGCACCAGC | 17 | 6409 |
| HSV1-UL48-570 | + | GCCGCGCUGCGCACCAG | 17 | 6408 |
| HSV1-UL48-1057 | + | AGCCGCGCUGCGCACCA | 17 | 6726 |
| HSV1-UL48-565 | + | UUGCCAUGCAGGGUGGG | 17 | 6403 |
| HSV1-UL48-1059 | + | GUUGCCAUGCAGGGUGG | 17 | 6727 |
| HSV1-UL48-564 | + | UGGUUGCCAUGCAGGGU | 17 | 6402 |
| HSV1-UL48-563 | + | CUGGUUGCCAUGCAGGG | 17 | 6401 |
| HSV1-UL48-1062 | + | CCUGGUUGCCAUGCAGG | 17 | 6728 |
| HSV1-UL48-1063 | + | CGGGCCUGGUUGCCAUG | 17 | 6729 |
| HSV1-UL48-1064 | + | AUAAAGUACCCAGAGGC | 17 | 6730 |
| HSV1-UL48-1065 | + | CAACACCAUAAAGUACC | 17 | 6731 |
| HSV1-UL48-1066 | + | UACGAGUCCAACUUCGC | 17 | 6732 |
| HSV1-UL48-1067 | + | GGUCGUGAAGCUGGAAU | 17 | 6733 |
| HSV1-UL48-556 | + | GGGCGAGGUCGUGAAGC | 17 | 6394 |
| HSV1-UL48-1069 | + | AGGGCGAGGUCGUGAAG | 17 | 6734 |
| HSV1-UL48-1070 | + | CCUCGGAGGGCGAGGUC | 17 | 6735 |
| HSV1-UL48-1071 | + | CAUGACCGCCUCGGAGG | 17 | 6736 |
| HSV1-UL48-1072 | + | CCCGCAUGACCGCCUCG | 17 | 6737 |
| HSV1-UL48-552 | + | UUCCCGCAUGACCGCCU | 17 | 6390 |
| HSV1-UL48-1074 | + | GUUCCCGCAUGACCGCC | 17 | 6738 |
| HSV1-UL48-1075 | + | CGUCGUCGUCCGGGAGA | 17 | 6739 |
| HSV1-UL48-548 | + | CGGGGGCGUCGUCGUCC | 17 | 6386 |
| HSV1-UL48-547 | + | UCGGGGGCGUCGUCGUC | 17 | 6385 |
| HSV1-UL48-1078 | + | UUCGGGGGCGUCGUCGU | 17 | 6740 |
| HSV1-UL48-544 | + | GCCAGCCCCGCCUCUUC | 17 | 6382 |
| HSV1-UL48-543 | + | CGCCAGCCCCGCCUCUU | 17 | 6381 |
| HSV1-UL48-1081 | + | CCGCCAGCCCCGCCUCU | 17 | 6741 |
| HSV1-UL48-542 | + | GGGAGAAAGGACAGGCG | 17 | 6380 |
| HSV1-UL48-1083 | + | GGGGAGAAAGGACAGGC | 17 | 6742 |
| HSV1-UL48-1084 | + | UGUGUCCCGCGGGGAGA | 17 | 6743 |
| HSV1-UL48-1085 | + | CGCGUGUGUCCCGCGGG | 17 | 6744 |
| HSV1-UL48-539 | + | UGCGCGUGUGUCCCGCG | 17 | 6377 |
| HSV1-UL48-538 | + | CUGCGCGUGUGUCCCGC | 17 | 6376 |
| HSV1-UL48-537 | + | UCUGCGCGUGUGUCCCG | 17 | 6375 |
| HSV1-UL48-1089 | + | GUCUGCGCGUGUGUCCC | 17 | 6745 |
| HSV1-UL48-534 | + | AGGCUGACAUCGGUCGG | 17 | 6372 |
| HSV1-UL48-533 | + | CAGGCUGACAUCGGUCG | 17 | 6371 |
| HSV1-UL48-532 | + | CCAGGCUGACAUCGGUC | 17 | 6370 |
| HSV1-UL48-531 | + | CCCAGGCUGACAUCGGU | 17 | 6369 |
| HSV1-UL48-1094 | + | CCCCAGGCUGACAUCGG | 17 | 6746 |
| HSV1-UL48-528 | + | CGUCCUCGCCGUCUAAG | 17 | 6366 |
| HSV1-UL48-1096 | + | ACGUCCUCGCCGUCUAA | 17 | 6747 |
| HSV1-UL48-1097 | + | CCCCCAACAUGUCCAGA | 17 | 6748 |
| HSV1-UL48-526 | + | GGUAAAUCCCGGCCCCG | 17 | 6364 |
| HSV1-UL48-525 | + | GGGUAAAUCCCGGCCCC | 17 | 6363 |
| HSV1-UL48-524 | + | GGGGUAAAUCCCGGCCC | 17 | 6362 |
| HSV1-UL48-1101 | + | GGGGGUAAAUCCCGGCC | 17 | 6749 |
| HSV1-UL48-520 | + | UAGGGGGCGGAGUCGUG | 17 | 6358 |
| HSV1-UL48-519 | + | GUAGGGGGCGGAGUCGU | 17 | 6357 |
| HSV1-UL48-518 | + | CGUAGGGGGCGGAGUCG | 17 | 6356 |
| HSV1-UL48-1105 | + | CCGUAGGGGGCGGAGUC | 17 | 6750 |
| HSV1-UL48-517 | + | CAGAGCGCCGUAGGGGG | 17 | 6355 |
| HSV1-UL48-1107 | + | CCAGAGCGCCGUAGGGG | 17 | 6751 |
| HSV1-UL48-514 | + | AUAUCCAGAGCGCCGUA | 17 | 6352 |
| HSV1-UL48-513 | + | CAUAUCCAGAGCGCCGU | 17 | 6351 |
| HSV1-UL48-1110 | + | CCAUAUCCAGAGCGCCG | 17 | 6752 |
| HSV1-UL48-1111 | + | UCGAAGUCGGCCAUAUC | 17 | 6753 |
| HSV1-UL48-1112 | + | UAAACAUCUGCUCAAAC | 17 | 6754 |
| HSV1-UL48-1113 | + | CCGUACUCGUCAAUUCC | 17 | 6755 |

**Table 3F** provides exemplary targeting domains for knocking out the *UL48* gene selected according to the first tier parameters. The targeting domains are selected based on location within first 500bp of the coding sequence of the *UL48* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3F**

| 1st Tier | | | | |
|---|---|---|---|---|
| gRNA Name | DNA Strand | Targeting Domain | Target Site Length | Seq ID |
| HSV1-UL48-1114 | + | CACGUCGCCGUGGGCGCGAA | 20 | 6756 |
| HSV1-UL48-1115 | + | GUCGCCGUGGGCGCGAA | 20 | 6757 |

**Table 3G** provides exemplary targeting domains for knocking out the *UL48* gene selected according to the second tier parameters. The targeting domains are selected based on location within the coding sequence (but downstream of the first 500bp) of the *UL48* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3G**

| 2nd Tier | | | | |
|---|---|---|---|---|
| gRNA Name | DNA Strand | Targeting Domain | Target Site Length | Seq ID |
| HSV1-UL48-1116 | - | CGCCUCUGGGUACUUUAUGG | 20 | 6758 |
| HSV1-UL48-1117 | - | AUGGCGCAUGCCGACGCGCU | 20 | 6759 |
| HSV1-UL48-308 | - | GAUCUGGACAUGUUGGGGGA | 20 | 6146 |
| HSV1-UL48-874 | - | GGGACGGGGAUUCCCCGGGG | 20 | 6605 |
| HSV1-UL48-1120 | - | CUCUGGGUACUUUAUGG | 17 | 6760 |
| HSV1-UL48-1121 | - | GCGCAUGCCGACGCGCU | 17 | 6761 |
| HSV1-UL48-494 | - | CUGGACAUGUUGGGGGA | 17 | 6332 |
| HSV1-UL48-951 | - | ACGGGGAUUCCCCGGGG | 17 | 6655 |
| HSV1-UL48-1124 | + | UAGCUCCCGCAGCCGGCGGG | 20 | 6762 |
| HSV1-UL48-1125 | + | CUCCCGCAGCCGGCGGG | 20 | 6763 |

**Table 4A** provides exemplary targeting domains for knocking out the *UL54* gene selected according to first tier parameters. The targeting domains are selected based on location within the first 500bp of the coding sequence of the *UL54* gene and orthogonality against the human genome. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4A**

| 1st Tier | | | | |
|---|---|---|---|---|
| gRNA Name | DNA Strand | Targeting Domain | Target Site Length | Seq ID |
| HSV1-UL54-1 | - | GGACCUCUCCGACAGCGAUC | 20 | 6764 |
| HSV1-UL54-2 | - | CUCCGACAGCGAUCUGGACG | 20 | 6765 |
| HSV1-UL54-3 | - | CCGCCGCGACGACCUGGAAU | 20 | 6766 |
| HSV1-UL54-4 | - | CAGCGGGGAGUGUUCCUCGU | 20 | 6767 |
| HSV1-UL54-5 | - | UUCCUCGUCGGACGAGGACA | 20 | 6768 |
| HSV1-UL54-6 | - | GAUACUCGACGCCGCUCGCC | 20 | 6769 |
| HSV1-UL54-7 | - | ACUCGACGCCGCUCGCCCGG | 20 | 6770 |
| HSV1-UL54-8 | - | CACCCAGACGCCUCGUCCGA | 20 | 6771 |
| HSV1-UL54-9 | - | GCCUCGUCCGACGGAGCGGC | 20 | 6772 |
| HSV1-UL54-10 | - | ACAGUGUGUGGUCGCGCCUC | 20 | 6773 |
| HSV1-UL54-11 | - | CAGUGUGUGGUCGCGCCUCG | 20 | 6774 |
| HSV1-UL54-12 | - | UGUGGUCGCGCCUCGGGGCC | 20 | 6775 |
| HSV1-UL54-13 | - | UUGCUCCCCCGAGCAGCACG | 20 | 6776 |
| HSV1-UL54-14 | - | UCGGGGUCGCGGUGGUCCCG | 20 | 6777 |
| HSV1-UL54-15 | - | CGGGGCUGCCGAUGGUUUGU | 20 | 6778 |
| HSV1-UL54-16 | - | AUGGUUUGUCGGACCCCCGC | 20 | 6779 |
| HSV1-UL54-17 | + | GCGGGGGUCCGACAAACCAU | 20 | 6780 |
| HSV1-UL54-18 | + | CGACAAACCAUCGGCAGCCC | 20 | 6781 |
| HSV1-UL54-19 | + | GACGCCCACGGCGUCCGCCG | 20 | 6782 |
| HSV1-UL54-20 | + | CCUUGCCCCCGUGCUGCUCG | 20 | 6783 |
| HSV1-UL54-21 | + | CUUGCCCCCGUGCUGCUCGG | 20 | 6784 |
| HSV1-UL54-22 | + | CGGGGGAGCAAGACGGUCGC | 20 | 6785 |
| HSV1-UL54-23 | + | GGGGGAGCAAGACGGUCGCC | 20 | 6786 |
| HSV1-UL54-24 | + | CGAGGCGCGACCACACACUG | 20 | 6787 |
| HSV1-UL54-25 | + | GAGGCGCGACCACACACUGU | 20 | 6788 |
| HSV1-UL54-26 | + | UUGGGGCCCUGCCGCUCCGU | 20 | 6789 |
| HSV1-UL54-27 | + | CCCUGCCGCUCCGUCGGACG | 20 | 6790 |
| HSV1-UL54-28 | + | GCUCCGUCGGACGAGGCGUC | 20 | 6791 |
| HSV1-UL54-29 | + | CUCCGUCGGACGAGGCGUCU | 20 | 6792 |
| HSV1-UL54-30 | + | GGUCUUCUGGACGAGACGGG | 20 | 6793 |
| HSV1-UL54-31 | + | GGGCGAGCGGCGUCGAGUAU | 20 | 6794 |
| HSV1-UL54-32 | + | UCCGGUCCGUCCUCUCCGUG | 20 | 6795 |
| HSV1-UL54-33 | + | UUCCAUGUCCUCGUCCGACG | 20 | 6796 |
| HSV1-UL54-34 | + | UGUCCGAUUCCAGGUCGUCG | 20 | 6797 |
| HSV1-UL54-35 | + | CCGAUUCCAGGUCGUCGCGG | 20 | 6798 |
| HSV1-UL54-36 | + | CGGCUCUCCGCCGGCUCGGG | 20 | 6799 |
| HSV1-UL54-37 | + | GUCCUCGUCCAGAUCGCUGU | 20 | 6800 |
| HSV1-UL54-38 | + | CGUCCAGAUCGCUGUCGGAG | 20 | 6801 |
| HSV1-UL54-39 | + | GAUCGCUGUCGGAGAGGUCC | 20 | 6802 |
| HSV1-UL54-40 | + | UGUCGGAGAGGUCCAGGCCG | 20 | 6803 |
| HSV1-UL54-41 | - | CCUCUCCGACAGCGAUC | 17 | 6804 |
| HSV1-UL54-42 | - | CGACAGCGAUCUGGACG | 17 | 6805 |
| HSV1-UL54-43 | - | CCGCGACGACCUGGAAU | 17 | 6806 |
| HSV1-UL54-44 | - | CGGGGAGUGUUCCUCGU | 17 | 6807 |
| HSV1-UL54-45 | - | CUCGUCGGACGAGGACA | 17 | 6808 |
| HSV1-UL54-46 | - | ACUCGACGCCGCUCGCC | 17 | 6809 |
| HSV1-UL54-47 | - | CGACGCCGCUCGCCCGG | 17 | 6810 |
| HSV1-UL54-48 | - | CCAGACGCCUCGUCCGA | 17 | 6811 |
| HSV1-UL54-49 | - | UCGUCCGACGGAGCGGC | 17 | 6812 |
| HSV1-UL54-50 | - | GUGUGUGGUCGCGCCUC | 17 | 6813 |
| HSV1-UL54-51 | - | UGUGUGGUCGCGCCUCG | 17 | 6814 |
| HSV1-UL54-52 | - | GGUCGCGCCUCGGGGCC | 17 | 6815 |
| HSV1-UL54-53 | - | CUCCCCCGAGCAGCACG | 17 | 6816 |
| HSV1-UL54-54 | - | GGGUCGCGGUGGUCCCG | 17 | 6817 |
| HSV1-UL54-55 | - | GGCUGCCGAUGGUUUGU | 17 | 6818 |
| HSV1-UL54-56 | - | GUUUGUCGGACCCCCGC | 17 | 6819 |
| HSV1-UL54-57 | + | GGGGUCCGACAAACCAU | 17 | 6820 |
| HSV1-UL54-58 | + | CAAACCAUCGGCAGCCC | 17 | 6821 |
| HSV1-UL54-59 | + | GCCCACGGCGUCCGCCG | 17 | 6822 |
| HSV1-UL54-60 | + | UGCCCCCGUGCUGCUCG | 17 | 6823 |
| HSV1-UL54-61 | + | GCCCCCGUGCUGCUCGG | 17 | 6824 |
| HSV1-UL54-62 | + | GGGAGCAAGACGGUCGC | 17 | 6825 |
| HSV1-UL54-63 | + | GGAGCAAGACGGUCGCC | 17 | 6826 |
| HSV1-UL54-64 | + | GGCGCGACCACACACUG | 17 | 6827 |
| HSV1-UL54-65 | + | GCGCGACCACACACUGU | 17 | 6828 |
| HSV1-UL54-66 | + | GGGCCCUGCCGCUCCGU | 17 | 6829 |
| HSV1-UL54-67 | + | UGCCGCUCCGUCGGACG | 17 | 6830 |
| HSV1-UL54-68 | + | CCGUCGGACGAGGCGUC | 17 | 6831 |
| HSV1-UL54-69 | + | CGUCGGACGAGGCGUCU | 17 | 6832 |
| HSV1-UL54-70 | + | CUUCUGGACGAGACGGG | 17 | 6833 |
| HSV1-UL54-71 | + | CGAGCGGCGUCGAGUAU | 17 | 6834 |
| HSV1-UL54-72 | + | GGUCCGUCCUCUCCGUG | 17 | 6835 |
| HSV1-UL54-73 | + | CAUGUCCUCGUCCGACG | 17 | 6836 |
| HSV1-UL54-74 | + | CCGAUUCCAGGUCGUCG | 17 | 6837 |
| HSV1-UL54-75 | + | AUUCCAGGUCGUCGCGG | 17 | 6838 |
| HSV1-UL54-76 | + | CUCUCCGCCGGCUCGGG | 17 | 6839 |
| HSV1-UL54-77 | + | CUCGUCCAGAUCGCUGU | 17 | 6840 |
| HSV1-UL54-78 | + | CCAGAUCGCUGUCGGAG | 17 | 6841 |
| HSV1-UL54-79 | + | CGCUGUCGGAGAGGUCC | 17 | 6842 |
| HSV1-UL54-80 | + | CGGAGAGGUCCAGGCCG | 17 | 6843 |

**Table 4B** provides exemplary targeting domains for knocking out the *UL54* gene selected according to the second tier parameters. The targeting domains are selected based on location within the first 500bp of the coding sequence of the *UL54* gene. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4B**

| 2nd Tier | | | | |
|---|---|---|---|---|
| gRNA Name | DNA Strand | Targeting Domain | Target Site Length | Seq ID |
| HSV1-UL54-81 | - | AUUGAUAUGCUAAUUGACCU | 20 | 6844 |
| HSV1-UL54-82 | - | UAUGCUAAUUGACCUCGGCC | 20 | 6845 |
| HSV1-UL54-83 | - | GGACGAGGACCCCCCCGAGC | 20 | 6846 |
| HSV1-UL54-84 | - | CGAGGACCCCCCCGAGCCGG | 20 | 6847 |
| HSV1-UL54-85 | - | GGAGAGCCGCCGCGACGACC | 20 | 6848 |
| HSV1-UL54-86 | - | GACCUGGAAUCGGACAGCAG | 20 | 6849 |
| HSV1-UL54-87 | - | ACCUGGAAUCGGACAGCAGC | 20 | 6850 |
| HSV1-UL54-88 | - | CCUGGAAUCGGACAGCAGCG | 20 | 6851 |
| HSV1-UL54-89 | - | GGAGUGUUCCUCGUCGGACG | 20 | 6852 |
| HSV1-UL54-90 | - | GAGGACAUGGAAGACCCCCA | 20 | 6853 |
| HSV1-UL54-91 | - | CAUGGAAGACCCCCACGGAG | 20 | 6854 |
| HSV1-UL54-92 | - | GAAGACCCCCACGGAGAGGA | 20 | 6855 |
| HSV1-UL54-93 | - | CCCCCACGGAGAGGACGGAC | 20 | 6856 |
| HSV1-UL54-94 | - | CCGUCUCGUCCAGAAGACCC | 20 | 6857 |
| HSV1-UL54-95 | - | AGACGCCUCGUCCGACGGAG | 20 | 6858 |
| HSV1-UL54-96 | - | CCUCGUCCGACGGAGCGGCA | 20 | 6859 |
| HSV1-UL54-97 | - | AACCAGCGCCCCACAGUGUG | 20 | 6860 |
| HSV1-UL54-98 | - | CACAGUGUGUGGUCGCGCCU | 20 | 6861 |
| HSV1-UL54-99 | - | UCUUGCUCCCCCGAGCAGCA | 20 | 6862 |
| HSV1-UL54-100 | - | CUUGCUCCCCCGAGCAGCAC | 20 | 6863 |
| HSV1-UL54-101 | - | UGCUCCCCCGAGCAGCACGG | 20 | 6864 |
| HSV1-UL54-102 | - | CCCCGAGCAGCACGGGGGCA | 20 | 6865 |
| HSV1-UL54-103 | - | CGAGCAGCACGGGGGCAAGG | 20 | 6866 |
| HSV1-UL54-104 | - | ACCAAAGCCCAGCCUGCCCG | 20 | 6867 |
| HSV1-UL54-105 | - | AAAGCCCAGCCUGCCCGCGG | 20 | 6868 |
| HSV1-UL54-106 | - | CCUGCCCGCGGCGGACGCCG | 20 | 6869 |
| HSV1-UL54-107 | - | CUGCCCGCGGCGGACGCCGU | 20 | 6870 |
| HSV1-UL54-108 | - | GCGGACGCCGUGGGCGUCGC | 20 | 6871 |
| HSV1-UL54-109 | - | CGGACGCCGUGGGCGUCGCA | 20 | 6872 |
| HSV1-UL54-110 | - | GGACGCCGUGGGCGUCGCAG | 20 | 6873 |
| HSV1-UL54-111 | - | GCCGUGGGCGUCGCAGGGGU | 20 | 6874 |
| HSV1-UL54-112 | - | CCGUGGGCGUCGCAGGGGUC | 20 | 6875 |
| HSV1-UL54-113 | - | CGUGGGCGUCGCAGGGGUCG | 20 | 6876 |
| HSV1-UL54-114 | - | CGUCGCAGGGGUCGGGGUCG | 20 | 6877 |
| HSV1-UL54-115 | - | CGCAGGGGUCGGGGUCGCGG | 20 | 6878 |
| HSV1-UL54-116 | - | GGUCGGGGUCGCGGUGGUCC | 20 | 6879 |
| HSV1-UL54-117 | - | GUCGGGGUCGCGGUGGUCCC | 20 | 6880 |
| HSV1-UL54-118 | - | GGUGGUCCCGGGGCUGCCGA | 20 | 6881 |
| HSV1-UL54-119 | + | GACAAACCAUCGGCAGCCCC | 20 | 6882 |
| HSV1-UL54-120 | + | CCCGACCCCUGCGACGCCCA | 20 | 6883 |
| HSV1-UL54-121 | + | ACGCCCACGGCGUCCGCCGC | 20 | 6884 |
| HSV1-UL54-122 | + | CCACGGCGUCCGCCGCGGGC | 20 | 6885 |
| HSV1-UL54-123 | + | GGCGUCCGCCGCGGGCAGGC | 20 | 6886 |
| HSV1-UL54-124 | + | GCGUCCGCCGCGGGCAGGCU | 20 | 6887 |
| HSV1-UL54-125 | + | GCCGCGGGCAGGCUGGGCUU | 20 | 6888 |
| HSV1-UL54-126 | + | CGGGCAGGCUGGGCUUUGGU | 20 | 6889 |
| HSV1-UL54-127 | + | GCAGGCUGGGCUUUGGUCGG | 20 | 6890 |
| HSV1-UL54-128 | + | CAGGCUGGGCUUUGGUCGGU | 20 | 6891 |
| HSV1-UL54-129 | + | AGGCUGGGCUUUGGUCGGUG | 20 | 6892 |
| HSV1-UL54-130 | + | GGCUGGGCUUUGGUCGGUGG | 20 | 6893 |
| HSV1-UL54-131 | + | GGGCUUUGGUCGGUGGGGGU | 20 | 6894 |
| HSV1-UL54-132 | + | CUUUGGUCGGUGGGGGUUGG | 20 | 6895 |
| HSV1-UL54-133 | + | UGGUCGGUGGGGGUUGGAGG | 20 | 6896 |
| HSV1-UL54-134 | + | GGUCGGUGGGGGUUGGAGGC | 20 | 6897 |
| HSV1-UL54-135 | + | CACCUUGCCCCCGUGCUGCU | 20 | 6898 |
| HSV1-UL54-136 | + | ACCUUGCCCCCGUGCUGCUC | 20 | 6899 |
| HSV1-UL54-137 | + | UGCUGCUCGGGGGAGCAAGA | 20 | 6900 |
| HSV1-UL54-138 | + | AAGACGGUCGCCGGGCCCCG | 20 | 6901 |
| HSV1-UL54-139 | + | AGGCGCGACCACACACUGUG | 20 | 6902 |
| HSV1-UL54-140 | + | GACCACACACUGUGGGGCGC | 20 | 6903 |
| HSV1-UL54-141 | + | ACACUGUGGGGCGCUGGUUG | 20 | 6904 |
| HSV1-UL54-142 | + | GGGCGCUGGUUGAGGAUCGU | 20 | 6905 |
| HSV1-UL54-143 | + | GGCGCUGGUUGAGGAUCGUU | 20 | 6906 |
| HSV1-UL54-144 | + | GCGCUGGUUGAGGAUCGUUG | 20 | 6907 |
| HSV1-UL54-145 | + | CGGACGAGGCGUCUGGGUGC | 20 | 6908 |
| HSV1-UL54-146 | + | GGACGAGGCGUCUGGGUGCU | 20 | 6909 |
| HSV1-UL54-147 | + | CGUCUGGGUGCUGGGUACGC | 20 | 6910 |
| HSV1-UL54-148 | + | GUCUGGGUGCUGGGUACGCC | 20 | 6911 |
| HSV1-UL54-149 | + | UCUGGGUGCUGGGUACGCCG | 20 | 6912 |
| HSV1-UL54-150 | + | CUGGGUACGCCGGGGUCUUC | 20 | 6913 |
| HSV1-UL54-151 | + | CCGGGGUCUUCUGGACGAGA | 20 | 6914 |
| HSV1-UL54-152 | + | CGGGGUCUUCUGGACGAGAC | 20 | 6915 |
| HSV1-UL54-153 | + | GGACGAGACGGGCGGACCGC | 20 | 6916 |
| HSV1-UL54-154 | + | GACGAGACGGGCGGACCGCC | 20 | 6917 |
| HSV1-UL54-155 | + | CGGGCGGACCGCCGGGCGAG | 20 | 6918 |
| HSV1-UL54-156 | + | GCGGCGUCGAGUAUCGGCUC | 20 | 6919 |
| HSV1-UL54-157 | + | GCUCCGGUCCGUCCUCUCCG | 20 | 6920 |
| HSV1-UL54-158 | + | CUCCGGUCCGUCCUCUCCGU | 20 | 6921 |
| HSV1-UL54-159 | + | CCGGUCCGUCCUCUCCGUGG | 20 | 6922 |
| HSV1-UL54-160 | + | CCCCGCUGCUGUCCGAUUCC | 20 | 6923 |
| HSV1-UL54-161 | + | UCGUCGCGGCGGCUCUCCGC | 20 | 6924 |
| HSV1-UL54-162 | + | GCGGCGGCUCUCCGCCGGCU | 20 | 6925 |
| HSV1-UL54-163 | + | CGGCGGCUCUCCGCCGGCUC | 20 | 6926 |
| HSV1-UL54-164 | + | GGCGGCUCUCCGCCGGCUCG | 20 | 6927 |
| HSV1-UL54-165 | + | GCGGCUCUCCGCCGGCUCGG | 20 | 6928 |
| HSV1-UL54-166 | + | GGCUCUCCGCCGGCUCGGGG | 20 | 6929 |
| HSV1-UL54-167 | - | GAUAUGCUAAUUGACCU | 17 | 6930 |
| HSV1-UL54-168 | - | GCUAAUUGACCUCGGCC | 17 | 6931 |
| HSV1-UL54-169 | - | CGAGGACCCCCCCGAGC | 17 | 6932 |
| HSV1-UL54-170 | - | GGACCCCCCCGAGCCGG | 17 | 6933 |
| HSV1-UL54-171 | - | GAGCCGCCGCGACGACC | 17 | 6934 |
| HSV1-UL54-172 | - | CUGGAAUCGGACAGCAG | 17 | 6935 |
| HSV1-UL54-173 | - | UGGAAUCGGACAGCAGC | 17 | 6936 |
| HSV1-UL54-174 | - | GGAAUCGGACAGCAGCG | 17 | 6937 |
| HSV1-UL54-175 | - | GUGUUCCUCGUCGGACG | 17 | 6938 |
| HSV1-UL54-176 | - | GACAUGGAAGACCCCCA | 17 | 6939 |
| HSV1-UL54-177 | - | GGAAGACCCCCACGGAG | 17 | 6940 |
| HSV1-UL54-178 | - | GACCCCCACGGAGAGGA | 17 | 6941 |
| HSV1-UL54-179 | - | CCACGGAGAGGACGGAC | 17 | 6942 |
| HSV1-UL54-180 | - | UCUCGUCCAGAAGACCC | 17 | 6943 |
| HSV1-UL54-181 | - | CGCCUCGUCCGACGGAG | 17 | 6944 |
| HSV1-UL54-182 | - | CGUCCGACGGAGCGGCA | 17 | 6945 |
| HSV1-UL54-183 | - | CAGCGCCCCACAGUGUG | 17 | 6946 |
| HSV1-UL54-184 | - | AGUGUGUGGUCGCGCCU | 17 | 6947 |
| HSV1-UL54-185 | - | UGCUCCCCCGAGCAGCA | 17 | 6948 |
| HSV1-UL54-186 | - | GCUCCCCCGAGCAGCAC | 17 | 6949 |
| HSV1-UL54-187 | - | UCCCCCGAGCAGCACGG | 17 | 6950 |
| HSV1-UL54-188 | - | CGAGCAGCACGGGGGCA | 17 | 6951 |
| HSV1-UL54-189 | - | GCAGCACGGGGGCAAGG | 17 | 6952 |
| HSV1-UL54-190 | - | AAAGCCCAGCCUGCCCG | 17 | 6953 |
| HSV1-UL54-191 | - | GCCCAGCCUGCCCGCGG | 17 | 6954 |
| HSV1-UL54-192 | - | GCCCGCGGCGGACGCCG | 17 | 6955 |
| HSV1-UL54-193 | - | CCCGCGGCGGACGCCGU | 17 | 6956 |
| HSV1-UL54-194 | - | GACGCCGUGGGCGUCGC | 17 | 6957 |
| HSV1-UL54-195 | - | ACGCCGUGGGCGUCGCA | 17 | 6958 |
| HSV1-UL54-196 | - | CGCCGUGGGCGUCGCAG | 17 | 6959 |
| HSV1-UL54-197 | - | GUGGGCGUCGCAGGGGU | 17 | 6960 |
| HSV1-UL54-198 | - | UGGGCGUCGCAGGGGUC | 17 | 6961 |
| HSV1-UL54-199 | - | GGGCGUCGCAGGGGUCG | 17 | 6962 |
| HSV1-UL54-200 | - | CGCAGGGGUCGGGGUCG | 17 | 6963 |
| HSV1-UL54-201 | - | AGGGGUCGGGGUCGCGG | 17 | 6964 |
| HSV1-UL54-202 | - | CGGGGUCGCGGUGGUCC | 17 | 6965 |
| HSV1-UL54-203 | - | GGGGUCGCGGUGGUCCC | 17 | 6966 |
| HSV1-UL54-204 | - | GGUCCCGGGGCUGCCGA | 17 | 6967 |
| HSV1-UL54-205 | + | AAACCAUCGGCAGCCCC | 17 | 6968 |
| HSV1-UL54-206 | + | GACCCCUGCGACGCCCA | 17 | 6969 |
| HSV1-UL54-207 | + | CCCACGGCGUCCGCCGC | 17 | 6970 |
| HSV1-UL54-208 | + | CGGCGUCCGCCGCGGGC | 17 | 6971 |
| HSV1-UL54-209 | + | GUCCGCCGCGGGCAGGC | 17 | 6972 |
| HSV1-UL54-210 | + | UCCGCCGCGGGCAGGCU | 17 | 6973 |
| HSV1-UL54-211 | + | GCGGGCAGGCUGGGCUU | 17 | 6974 |
| HSV1-UL54-212 | + | GCAGGCUGGGCUUUGGU | 17 | 6975 |
| HSV1-UL54-213 | + | GGCUGGGCUUUGGUCGG | 17 | 6976 |
| HSV1-UL54-214 | + | GCUGGGCUUUGGUCGGU | 17 | 6977 |
| HSV1-UL54-215 | + | CUGGGCUUUGGUCGGUG | 17 | 6978 |
| HSV1-UL54-216 | + | UGGGCUUUGGUCGGUGG | 17 | 6979 |
| HSV1-UL54-217 | + | CUUUGGUCGGUGGGGGU | 17 | 6980 |
| HSV1-UL54-218 | + | UGGUCGGUGGGGGUUGG | 17 | 6981 |
| HSV1-UL54-219 | + | UCGGUGGGGGUUGGAGG | 17 | 6982 |
| HSV1-UL54-220 | + | CGGUGGGGGUUGGAGGC | 17 | 6983 |
| HSV1-UL54-221 | + | CUUGCCCCCGUGCUGCU | 17 | 6984 |
| HSV1-UL54-222 | + | UUGCCCCCGUGCUGCUC | 17 | 6985 |
| HSV1-UL54-223 | + | UGCUCGGGGGAGCAAGA | 17 | 6986 |
| HSV1-UL54-224 | + | ACGGUCGCCGGGCCCCG | 17 | 6987 |
| HSV1-UL54-225 | + | CGCGACCACACACUGUG | 17 | 6988 |
| HSV1-UL54-226 | + | CACACACUGUGGGGCGC | 17 | 6989 |
| HSV1-UL54-227 | + | CUGUGGGGCGCUGGUUG | 17 | 6990 |
| HSV1-UL54-228 | + | CGCUGGUUGAGGAUCGU | 17 | 6991 |
| HSV1-UL54-229 | + | GCUGGUUGAGGAUCGUU | 17 | 6992 |
| HSV1-UL54-230 | + | CUGGUUGAGGAUCGUUG | 17 | 6993 |
| HSV1-UL54-231 | + | ACGAGGCGUCUGGGUGC | 17 | 6994 |
| HSV1-UL54-232 | + | CGAGGCGUCUGGGUGCU | 17 | 6995 |
| HSV1-UL54-233 | + | CUGGGUGCUGGGUACGC | 17 | 6996 |
| HSV1-UL54-234 | + | UGGGUGCUGGGUACGCC | 17 | 6997 |
| HSV1-UL54-235 | + | GGGUGCUGGGUACGCCG | 17 | 6998 |
| HSV1-UL54-236 | + | GGUACGCCGGGGUCUUC | 17 | 6999 |
| HSV1-UL54-237 | + | GGGUCUUCUGGACGAGA | 17 | 7000 |
| HSV1-UL54-238 | + | GGUCUUCUGGACGAGAC | 17 | 7001 |
| HSV1-UL54-239 | + | CGAGACGGGCGGACCGC | 17 | 7002 |
| HSV1-UL54-240 | + | GAGACGGGCGGACCGCC | 17 | 7003 |
| HSV1-UL54-241 | + | GCGGACCGCCGGGCGAG | 17 | 7004 |
| HSV1-UL54-242 | + | GCGUCGAGUAUCGGCUC | 17 | 7005 |
| HSV1-UL54-243 | + | CCGGUCCGUCCUCUCCG | 17 | 7006 |
| HSV1-UL54-244 | + | CGGUCCGUCCUCUCCGU | 17 | 7007 |
| HSV1-UL54-245 | + | GUCCGUCCUCUCCGUGG | 17 | 7008 |
| HSV1-UL54-246 | + | CGCUGCUGUCCGAUUCC | 17 | 7009 |
| HSV1-UL54-247 | + | UCGCGGCGGCUCUCCGC | 17 | 7010 |
| HSV1-UL54-248 | + | GCGGCUCUCCGCCGGCU | 17 | 7011 |
| HSV1-UL54-249 | + | CGGCUCUCCGCCGGCUC | 17 | 7012 |
| HSV1-UL54-250 | + | GGCUCUCCGCCGGCUCG | 17 | 7013 |
| HSV1-UL54-251 | + | GCUCUCCGCCGGCUCGG | 17 | 7014 |
| HSV1-UL54-252 | + | UCUCCGCCGGCUCGGGG | 17 | 7015 |

**Table 4C** provides exemplary targeting domains for knocking out the *UL54* gene selected according to the third tier parameters. The targeting domains are selected based on location within the coding sequence, but downstream of the first 500bp of the *UL54* gene. It is contemplated herein that the targeting domain hybridizes to the target domain through complementary base pairing. Any of the targeting domains in the table can be used with a *S*. *pyogenes* Cas9 molecule that gives double stranded cleavage. Any of the targeting domains in the table can be used with a *S*. *pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4C**

| 3rd Tier | | | | |
|---|---|---|---|---|
| gRNA Name | DNA Strand | Targeting Domain | Target Site Length | Seq ID |
| HSV1-UL54-253 | - | AGAACCAAUCGCAACCCUGG | 20 | 7016 |
| HSV1-UL54-254 | - | AACCCUGGGGGACCCCGCCC | 20 | 7017 |
| HSV1-UL54-255 | - | ACCCUGGGGGACCCCGCCCC | 20 | 7018 |
| HSV1-UL54-256 | - | CCCUGGGGGACCCCGCCCCG | 20 | 7019 |
| HSV1-UL54-257 | - | UGGGGGACCCCGCCCCGGGG | 20 | 7020 |
| HSV1-UL54-258 | - | GGGGGACCCCGCCCCGGGGC | 20 | 7021 |
| HSV1-UL54-259 | - | GGGGACCCCGCCCCGGGGCG | 20 | 7022 |
| HSV1-UL54-260 | - | GACCCCGCCCCGGGGCGGGG | 20 | 7023 |
| HSV1-UL54-261 | - | CCGCCCCGGGGCGGGGUGGA | 20 | 7024 |
| HSV1-UL54-262 | - | CCCGGGGCGGGGUGGACGGA | 20 | 7025 |
| HSV1-UL54-263 | - | GCGGGGUGGACGGACGGCCC | 20 | 7026 |
| HSV1-UL54-264 | - | GACGGCCCCGGCGCCCCCCA | 20 | 7027 |
| HSV1-UL54-265 | - | CCCCGGCGCCCCCCAUGGCG | 20 | 7028 |
| HSV1-UL54-266 | - | GCGCCCCCCAUGGCGAGGCG | 20 | 7029 |
| HSV1-UL54-267 | - | CCCCAUGGCGAGGCGUGGCG | 20 | 7030 |
| HSV1-UL54-268 | - | AGUGAGCAGCCCGACCCACC | 20 | 7031 |
| HSV1-UL54-269 | - | GAGCAGCCCGACCCACCCGG | 20 | 7032 |
| HSV1-UL54-270 | - | CCGACCCACCCGGAGGCCAG | 20 | 7033 |
| HSV1-UL54-271 | - | CACCCGGAGGCCAGCGGACA | 20 | 7034 |
| HSV1-UL54-272 | - | ACCCGGAGGCCAGCGGACAC | 20 | 7035 |
| HSV1-UL54-273 | - | CCCGGAGGCCAGCGGACACG | 20 | 7036 |
| HSV1-UL54-274 | - | ACCCCCCCCGCUAAUGACGC | 20 | 7037 |
| HSV1-UL54-275 | - | GGCGAUUGCCCCCCCGCCCG | 20 | 7038 |
| HSV1-UL54-276 | - | GCCCGCGGACCCCCGCGCCC | 20 | 7039 |
| HSV1-UL54-277 | - | GGACCCCCGCGCCCCGGCCC | 20 | 7040 |
| HSV1-UL54-278 | - | CGCCCCGGCCCCGGAGCGAA | 20 | 7041 |
| HSV1-UL54-279 | - | ACACCAUCGACGCCACCACG | 20 | 7042 |
| HSV1-UL54-280 | - | CAUCGACGCCACCACGCGGU | 20 | 7043 |
| HSV1-UL54-281 | - | GCGCUCCAUCUCCGAGCGCG | 20 | 7044 |
| HSV1-UL54-282 | - | CUCCAUCUCCGAGCGCGCGG | 20 | 7045 |
| HSV1-UL54-283 | - | GACCGCAUCAGCGAGAGCUU | 20 | 7046 |
| HSV1-UL54-284 | - | GAGCUUUGGCCGCAGCGCAC | 20 | 7047 |
| HSV1-UL54-285 | - | CAGGUCAUGCACGACCCCUU | 20 | 7048 |
| HSV1-UL54-286 | - | AGGUCAUGCACGACCCCUUU | 20 | 7049 |
| HSV1-UL54-287 | - | GGUCAUGCACGACCCCUUUG | 20 | 7050 |
| HSV1-UL54-288 | - | GUCAUGCACGACCCCUUUGG | 20 | 7051 |
| HSV1-UL54-289 | - | UCAUGCACGACCCCUUUGGG | 20 | 7052 |
| HSV1-UL54-290 | - | UUCCCGCCGCGAAUAGCCCC | 20 | 7053 |
| HSV1-UL54-291 | - | UCCCGCCGCGAAUAGCCCCU | 20 | 7054 |
| HSV1-UL54-292 | - | CGCGAAUAGCCCCUGGGCCC | 20 | 7055 |
| HSV1-UL54-293 | - | UAGCCCCUGGGCCCCGGUGC | 20 | 7056 |
| HSV1-UL54-294 | - | CCCCUGGGCCCCGGUGCUGG | 20 | 7057 |
| HSV1-UL54-295 | - | CCCUGGGCCCCGGUGCUGGC | 20 | 7058 |
| HSV1-UL54-296 | - | GCCCCGGUGCUGGCGGGCCA | 20 | 7059 |
| HSV1-UL54-297 | - | CCGGUGCUGGCGGGCCAAGG | 20 | 7060 |
| HSV1-UL54-298 | - | CGGUGCUGGCGGGCCAAGGA | 20 | 7061 |
| HSV1-UL54-299 | - | CCUUUGACGCCGAGACCAGA | 20 | 7062 |
| HSV1-UL54-300 | - | CUUUGACGCCGAGACCAGAC | 20 | 7063 |
| HSV1-UL54-301 | - | CCGAGACCAGACGGGUCUCC | 20 | 7064 |
| HSV1-UL54-302 | - | CGAGACCAGACGGGUCUCCU | 20 | 7065 |
| HSV1-UL54-303 | - | ACGGGUCUCCUGGGAAACCU | 20 | 7066 |
| HSV1-UL54-304 | - | UGGGAAACCUUGGUCGCCCA | 20 | 7067 |
| HSV1-UL54-305 | - | AGCCUCUAUCGCACUUUUGC | 20 | 7068 |
| HSV1-UL54-306 | - | GCACUUUUGCCGGCAAUCCU | 20 | 7069 |
| HSV1-UL54-307 | - | CACUUUUGCCGGCAAUCCUC | 20 | 7070 |
| HSV1-UL54-308 | - | UCGGGCCGCAUCGACCGCCA | 20 | 7071 |
| HSV1-UL54-309 | - | GCGCCAAGAAAAUUUCAUCG | 20 | 7072 |
| HSV1-UL54-310 | - | AGAAAAUUUCAUCGAGGCGC | 20 | 7073 |
| HSV1-UL54-311 | - | GGCCUCCGCCGACGAGACGC | 20 | 7074 |
| HSV1-UL54-312 | - | CCGCCGACGAGACGCUGGCG | 20 | 7075 |
| HSV1-UL54-313 | - | CAACCUGCCGCUGCGCCCCC | 20 | 7076 |
| HSV1-UL54-314 | - | CGCCCCCAGGACCCCAUUAU | 20 | 7077 |
| HSV1-UL54-315 | - | GCCCCCAGGACCCCAUUAUC | 20 | 7078 |
| HSV1-UL54-316 | - | CCCCAUUAUCGGGACGACCG | 20 | 7079 |
| HSV1-UL54-317 | - | CGGGACGACCGCGGCUGUGC | 20 | 7080 |
| HSV1-UL54-318 | - | AUAACCUCGCCACGCGCCUG | 20 | 7081 |
| HSV1-UL54-319 | - | CUUUCUCCAGUGCUACCUGA | 20 | 7082 |
| HSV1-UL54-320 | - | CAGUGCUACCUGAAGGCGCG | 20 | 7083 |
| HSV1-UL54-321 | - | CUGAAGGCGCGAGGCCUGUG | 20 | 7084 |
| HSV1-UL54-322 | - | GGCGCGAGGCCUGUGCGGCC | 20 | 7085 |
| HSV1-UL54-323 | - | GCCUGGACGAACUGUGUUCG | 20 | 7086 |
| HSV1-UL54-324 | - | UGGACGAACUGUGUUCGCGG | 20 | 7087 |
| HSV1-UL54-325 | - | ACUGUGUUCGCGGCGGCGUC | 20 | 7088 |
| HSV1-UL54-326 | - | GUGUUCGCGGCGGCGUCUGG | 20 | 7089 |
| HSV1-UL54-327 | - | GCGGCGUCUGGCGGACAUUA | 20 | 7090 |
| HSV1-UL54-328 | - | AUCCUUCGUGUUUGUCAUUC | 20 | 7091 |
| HSV1-UL54-329 | - | UCGUGUUUGUCAUUCUGGCC | 20 | 7092 |
| HSV1-UL54-330 | - | CUCGCCAACCGCGUCGAGCG | 20 | 7093 |
| HSV1-UL54-331 | - | CCGCGUCGAGCGUGGCGUCG | 20 | 7094 |
| HSV1-UL54-332 | - | GAGAUCGACUACGCGACCCU | 20 | 7095 |
| HSV1-UL54-333 | - | GACUACGCGACCCUUGGUGU | 20 | 7096 |
| HSV1-UL54-334 | - | ACUACGCGACCCUUGGUGUC | 20 | 7097 |
| HSV1-UL54-335 | - | CUACGCGACCCUUGGUGUCG | 20 | 7098 |
| HSV1-UL54-336 | - | GCGACCCUUGGUGUCGGGGU | 20 | 7099 |
| HSV1-UL54-337 | - | AAGAUGCAUUUCUACCUCCC | 20 | 7100 |
| HSV1-UL54-338 | - | AGAUGCAUUUCUACCUCCCC | 20 | 7101 |
| HSV1-UL54-339 | - | GAUGCAUUUCUACCUCCCCG | 20 | 7102 |
| HSV1-UL54-340 | - | CUACCUCCCCGGGGCCUGCA | 20 | 7103 |
| HSV1-UL54-341 | - | CCUCCCCGGGGCCUGCAUGG | 20 | 7104 |
| HSV1-UL54-342 | - | CUCCCCGGGGCCUGCAUGGC | 20 | 7105 |
| HSV1-UL54-343 | - | AAUCCUAGACACGCACCGCC | 20 | 7106 |
| HSV1-UL54-344 | - | GAGUCGUGUCUGCGAGUUGA | 20 | 7107 |
| HSV1-UL54-345 | - | GUCGCCCCCCCGUACGUGCA | 20 | 7108 |
| HSV1-UL54-346 | + | AAAUAUUUGCCGUGCACGUA | 20 | 7109 |
| HSV1-UL54-347 | + | AAUAUUUGCCGUGCACGUAC | 20 | 7110 |
| HSV1-UL54-348 | + | AUAUUUGCCGUGCACGUACG | 20 | 7111 |
| HSV1-UL54-349 | + | UAUUUGCCGUGCACGUACGG | 20 | 7112 |
| HSV1-UL54-350 | + | AUUUGCCGUGCACGUACGGG | 20 | 7113 |
| HSV1-UL54-351 | + | UUUGCCGUGCACGUACGGGG | 20 | 7114 |
| HSV1-UL54-352 | + | CGGGGGGGCGACGAUGUGAC | 20 | 7115 |
| HSV1-UL54-353 | + | AGACACGACUCGAACACUCC | 20 | 7116 |
| HSV1-UL54-354 | + | CACGACUCGAACACUCCUGG | 20 | 7117 |
| HSV1-UL54-355 | + | ACUCCUGGCGGUGCGUGUCU | 20 | 7118 |
| HSV1-UL54-356 | + | GCGUGUCUAGGAUUUCGAUC | 20 | 7119 |
| HSV1-UL54-357 | + | UUCGAUCAGGCCCGCCAUGC | 20 | 7120 |
| HSV1-UL54-358 | + | CAGGCCCGCCAUGCAGGCCC | 20 | 7121 |
| HSV1-UL54-359 | + | AGGCCCGCCAUGCAGGCCCC | 20 | 7122 |
| HSV1-UL54-360 | + | GGCCCGCCAUGCAGGCCCCG | 20 | 7123 |
| HSV1-UL54-361 | + | CCGCCAUGCAGGCCCCGGGG | 20 | 7124 |
| HSV1-UL54-362 | + | UCUCUCCGACCCCGACACCA | 20 | 7125 |
| HSV1-UL54-363 | + | CUCUCCGACCCCGACACCAA | 20 | 7126 |
| HSV1-UL54-364 | + | CCGCGACGCCACGCUCGACG | 20 | 7127 |
| HSV1-UL54-365 | + | GACGCCACGCUCGACGCGGU | 20 | 7128 |
| HSV1-UL54-366 | + | CUCGACGCGGUUGGCGAGCC | 20 | 7129 |
| HSV1-UL54-367 | + | GGCCAGAAUGACAAACACGA | 20 | 7130 |
| HSV1-UL54-368 | + | GCCGCGAACACAGUUCGUCC | 20 | 7131 |
| HSV1-UL54-369 | + | ACAGUUCGUCCAGGCCGCAC | 20 | 7132 |
| HSV1-UL54-370 | + | CGCACAGGCCUCGCGCCUUC | 20 | 7133 |
| HSV1-UL54-371 | + | CUCGCGCCUUCAGGUAGCAC | 20 | 7134 |
| HSV1-UL54-372 | + | CUUCAGGUAGCACUGGAGAA | 20 | 7135 |
| HSV1-UL54-373 | + | UUCAGGUAGCACUGGAGAAA | 20 | 7136 |
| HSV1-UL54-374 | + | AGCACUGGAGAAAGGGCCGC | 20 | 7137 |
| HSV1-UL54-375 | + | GAGAAAGGGCCGCAGGCGCG | 20 | 7138 |
| HSV1-UL54-376 | + | AGGGCCGCAGGCGCGUGGCG | 20 | 7139 |
| HSV1-UL54-377 | + | GAGGUUAUCCAGCACAGCCG | 20 | 7140 |
| HSV1-UL54-378 | + | AGCCGCGGUCGUCCCGAUAA | 20 | 7141 |
| HSV1-UL54-379 | + | GCCGCGGUCGUCCCGAUAAU | 20 | 7142 |
| HSV1-UL54-380 | + | CCGCGGUCGUCCCGAUAAUG | 20 | 7143 |
| HSV1-UL54-381 | + | UCGUCCCGAUAAUGGGGUCC | 20 | 7144 |
| HSV1-UL54-382 | + | CGUCCCGAUAAUGGGGUCCU | 20 | 7145 |
| HSV1-UL54-383 | + | GUCCCGAUAAUGGGGUCCUG | 20 | 7146 |
| HSV1-UL54-384 | + | UCCCGAUAAUGGGGUCCUGG | 20 | 7147 |
| HSV1-UL54-385 | + | AUGGGGUCCUGGGGGCGCAG | 20 | 7148 |
| HSV1-UL54-386 | + | GGUCCUGGGGGCGCAGCGGC | 20 | 7149 |
| HSV1-UL54-387 | + | GGGGGCGCAGCGGCAGGUUG | 20 | 7150 |
| HSV1-UL54-388 | + | GGCGCAGCGGCAGGUUGUGG | 20 | 7151 |
| HSV1-UL54-389 | + | GCACCACGCCAGCGUCUCGU | 20 | 7152 |
| HSV1-UL54-390 | + | CCACGCCAGCGUCUCGUCGG | 20 | 7153 |
| HSV1-UL54-391 | + | CGCCAGCGUCUCGUCGGCGG | 20 | 7154 |
| HSV1-UL54-392 | + | GCGCCUCGAUGAAAUUUUCU | 20 | 7155 |
| HSV1-UL54-393 | + | GCGCAGCACGCAGUCGCGCA | 20 | 7156 |
| HSV1-UL54-394 | + | CACGCAGUCGCGCAUGGCCU | 20 | 7157 |
| HSV1-UL54-395 | + | GCAGUCGCGCAUGGCCUUGG | 20 | 7158 |
| HSV1-UL54-396 | + | CAUGGCCUUGGCGGUCGAUG | 20 | 7159 |
| HSV1-UL54-397 | + | UUGGCGGUCGAUGCGGCCCG | 20 | 7160 |
| HSV1-UL54-398 | + | CGAUGCGGCCCGAGGAUUGC | 20 | 7161 |
| HSV1-UL54-399 | + | UGCCGGCAAAAGUGCGAUAG | 20 | 7162 |
| HSV1-UL54-400 | + | GCAAAAGUGCGAUAGAGGCU | 20 | 7163 |
| HSV1-UL54-401 | + | CAAAAGUGCGAUAGAGGCUC | 20 | 7164 |
| HSV1-UL54-402 | + | UGCGAUAGAGGCUCGGGCCG | 20 | 7165 |
| HSV1-UL54-403 | + | GCGAUAGAGGCUCGGGCCGU | 20 | 7166 |
| HSV1-UL54-404 | + | GCUCGGGCCGUGGGCGACCA | 20 | 7167 |
| HSV1-UL54-405 | + | GUGGGCGACCAAGGUUUCCC | 20 | 7168 |
| HSV1-UL54-406 | + | GGUUUCCCAGGAGACCCGUC | 20 | 7169 |
| HSV1-UL54-407 | + | CCAGGAGACCCGUCUGGUCU | 20 | 7170 |
| HSV1-UL54-408 | + | CCGUCUGGUCUCGGCGUCAA | 20 | 7171 |
| HSV1-UL54-409 | + | CGUCUGGUCUCGGCGUCAAA | 20 | 7172 |
| HSV1-UL54-410 | + | CGGCGUCAAAGGGCCCUCCU | 20 | 7173 |
| HSV1-UL54-411 | + | CCUCCUUGGCCCGCCAGCAC | 20 | 7174 |
| HSV1-UL54-412 | + | CUCCUUGGCCCGCCAGCACC | 20 | 7175 |
| HSV1-UL54-413 | + | UCCUUGGCCCGCCAGCACCG | 20 | 7176 |
| HSV1-UL54-414 | + | GCCCGCCAGCACCGGGGCCC | 20 | 7177 |
| HSV1-UL54-415 | + | CCCGCCAGCACCGGGGCCCA | 20 | 7178 |
| HSV1-UL54-416 | + | CCGCCAGCACCGGGGCCCAG | 20 | 7179 |
| HSV1-UL54-417 | + | CGGGGCCCAGGGGCUAUUCG | 20 | 7180 |
| HSV1-UL54-418 | + | GGCCCAGGGGCUAUUCGCGG | 20 | 7181 |
| HSV1-UL54-419 | + | GCCCAGGGGCUAUUCGCGGC | 20 | 7182 |
| HSV1-UL54-420 | + | GGGCUAUUCGCGGCGGGAAA | 20 | 7183 |
| HSV1-UL54-421 | + | GGGAAACGGCUGCCCCCCAA | 20 | 7184 |
| HSV1-UL54-422 | + | GGAAACGGCUGCCCCCCAAA | 20 | 7185 |
| HSV1-UL54-423 | + | GAAACGGCUGCCCCCCAAAG | 20 | 7186 |
| HSV1-UL54-424 | + | CGUGCAUGACCUGUGCGCUG | 20 | 7187 |
| HSV1-UL54-425 | + | GGCCAAAGCUCUCGCUGAUG | 20 | 7188 |
| HSV1-UL54-426 | + | GCGGUCGACCGCCGCGCGCU | 20 | 7189 |
| HSV1-UL54-427 | + | GACCGCCGCGCGCUCGGAGA | 20 | 7190 |
| HSV1-UL54-428 | + | CGCGCUCGGAGAUGGAGCGC | 20 | 7191 |
| HSV1-UL54-429 | + | GGAGCGCAGGACCAACCGCG | 20 | 7192 |
| HSV1-UL54-430 | + | GCGCAGGACCAACCGCGUGG | 20 | 7193 |
| HSV1-UL54-431 | + | CAACCGCGUGGUGGCGUCGA | 20 | 7194 |
| HSV1-UL54-432 | + | CGUGGUGGCGUCGAUGGUGU | 20 | 7195 |
| HSV1-UL54-433 | + | GGUGGCGUCGAUGGUGUCGG | 20 | 7196 |
| HSV1-UL54-434 | + | GGCGUCGAUGGUGUCGGCGG | 20 | 7197 |
| HSV1-UL54-435 | + | GCGUCGAUGGUGUCGGCGGC | 20 | 7198 |
| HSV1-UL54-436 | + | GCGGCGGGCGCCUUUCGCUC | 20 | 7199 |
| HSV1-UL54-437 | + | CGGCGGGCGCCUUUCGCUCC | 20 | 7200 |
| HSV1-UL54-438 | + | GGCGGGCGCCUUUCGCUCCG | 20 | 7201 |
| HSV1-UL54-439 | + | GGCGCCUUUCGCUCCGGGGC | 20 | 7202 |
| HSV1-UL54-440 | + | GCGCCUUUCGCUCCGGGGCC | 20 | 7203 |
| HSV1-UL54-441 | + | CGCCUUUCGCUCCGGGGCCG | 20 | 7204 |
| HSV1-UL54-442 | + | UUCGCUCCGGGGCCGGGGCG | 20 | 7205 |
| HSV1-UL54-443 | + | UCGCUCCGGGGCCGGGGCGC | 20 | 7206 |
| HSV1-UL54-444 | + | CGCUCCGGGGCCGGGGCGCG | 20 | 7207 |
| HSV1-UL54-445 | + | GCUCCGGGGCCGGGGCGCGG | 20 | 7208 |
| HSV1-UL54-446 | + | GGCCGGGGCGCGGGGGUCCG | 20 | 7209 |
| HSV1-UL54-447 | + | GCCGGGGCGCGGGGGUCCGC | 20 | 7210 |
| HSV1-UL54-448 | + | GGGGCGCGGGGGUCCGCGGG | 20 | 7211 |
| HSV1-UL54-449 | + | GGGCGCGGGGGUCCGCGGGC | 20 | 7212 |
| HSV1-UL54-450 | + | GGCGCGGGGGUCCGCGGGCG | 20 | 7213 |
| HSV1-UL54-451 | + | GCGCGGGGGUCCGCGGGCGG | 20 | 7214 |
| HSV1-UL54-452 | + | CGCGGGGGUCCGCGGGCGGG | 20 | 7215 |
| HSV1-UL54-453 | + | GCGGGGGUCCGCGGGCGGGG | 20 | 7216 |
| HSV1-UL54-454 | + | GCAAUCGCCAGCGUCAUUAG | 20 | 7217 |
| HSV1-UL54-455 | + | CAAUCGCCAGCGUCAUUAGC | 20 | 7218 |
| HSV1-UL54-456 | + | AAUCGCCAGCGUCAUUAGCG | 20 | 7219 |
| HSV1-UL54-457 | + | AUCGCCAGCGUCAUUAGCGG | 20 | 7220 |
| HSV1-UL54-458 | + | UCGCCAGCGUCAUUAGCGGG | 20 | 7221 |
| HSV1-UL54-459 | + | CGCCAGCGUCAUUAGCGGGG | 20 | 7222 |
| HSV1-UL54-460 | + | GCCAGCGUCAUUAGCGGGGG | 20 | 7223 |
| HSV1-UL54-461 | + | UCAUUAGCGGGGGGGGUGCU | 20 | 7224 |
| HSV1-UL54-462 | + | GGCGCACGCCCCGUGUCCGC | 20 | 7225 |
| HSV1-UL54-463 | + | GCCCCGUGUCCGCUGGCCUC | 20 | 7226 |
| HSV1-UL54-464 | + | CCCCGUGUCCGCUGGCCUCC | 20 | 7227 |
| HSV1-UL54-465 | + | CGUGUCCGCUGGCCUCCGGG | 20 | 7228 |
| HSV1-UL54-466 | + | GUGUCCGCUGGCCUCCGGGU | 20 | 7229 |
| HSV1-UL54-467 | + | CCGCUGGCCUCCGGGUGGGU | 20 | 7230 |
| HSV1-UL54-468 | + | CGCUGGCCUCCGGGUGGGUC | 20 | 7231 |
| HSV1-UL54-469 | + | UGCCGCGCCACGCCUCGCCA | 20 | 7232 |
| HSV1-UL54-470 | + | GCCGCGCCACGCCUCGCCAU | 20 | 7233 |
| HSV1-UL54-471 | + | CCGCGCCACGCCUCGCCAUG | 20 | 7234 |
| HSV1-UL54-472 | + | CGCGCCACGCCUCGCCAUGG | 20 | 7235 |
| HSV1-UL54-473 | + | GCGCCACGCCUCGCCAUGGG | 20 | 7236 |
| HSV1-UL54-474 | + | CGCCUCGCCAUGGGGGGCGC | 20 | 7237 |
| HSV1-UL54-475 | + | GCCUCGCCAUGGGGGGCGCC | 20 | 7238 |
| HSV1-UL54-476 | + | CCUCGCCAUGGGGGGCGCCG | 20 | 7239 |
| HSV1-UL54-477 | + | GCCGUCCGUCCACCCCGCCC | 20 | 7240 |
| HSV1-UL54-478 | + | CCGUCCGUCCACCCCGCCCC | 20 | 7241 |
| HSV1-UL54-479 | + | CGUCCGUCCACCCCGCCCCG | 20 | 7242 |
| HSV1-UL54-480 | + | CCGUCCACCCCGCCCCGGGG | 20 | 7243 |
| HSV1-UL54-481 | + | CGUCCACCCCGCCCCGGGGC | 20 | 7244 |
| HSV1-UL54-482 | + | GUCCACCCCGCCCCGGGGCG | 20 | 7245 |
| HSV1-UL54-483 | + | GCCCCGGGGCGGGGUCCCCC | 20 | 7246 |
| HSV1-UL54-484 | + | CCCCGGGGCGGGGUCCCCCA | 20 | 7247 |
| HSV1-UL54-485 | + | GGGUCCCCCAGGGUUGCGAU | 20 | 7248 |
| HSV1-UL54-486 | - | AGAACCAAUCGCAACCC | 17 | 7249 |
| HSV1-UL54-487 | - | GAACCAAUCGCAACCCU | 17 | 7250 |
| HSV1-UL54-488 | - | AACCAAUCGCAACCCUG | 17 | 7251 |
| HSV1-UL54-489 | - | ACCAAUCGCAACCCUGG | 17 | 7252 |
| HSV1-UL54-490 | - | CCUGGGGGACCCCGCCC | 17 | 7253 |
| HSV1-UL54-491 | - | CUGGGGGACCCCGCCCC | 17 | 7254 |
| HSV1-UL54-492 | - | UGGGGGACCCCGCCCCG | 17 | 7255 |
| HSV1-UL54-493 | - | GGGACCCCGCCCCGGGG | 17 | 7256 |
| HSV1-UL54-494 | - | GGACCCCGCCCCGGGGC | 17 | 7257 |
| HSV1-UL54-495 | - | GACCCCGCCCCGGGGCG | 17 | 7258 |
| HSV1-UL54-496 | - | CCCGCCCCGGGGCGGGG | 17 | 7259 |
| HSV1-UL54-497 | - | CCCCGGGGCGGGGUGGA | 17 | 7260 |
| HSV1-UL54-498 | - | GGGGCGGGGUGGACGGA | 17 | 7261 |
| HSV1-UL54-499 | - | GGGUGGACGGACGGCCC | 17 | 7262 |
| HSV1-UL54-500 | - | GGCCCCGGCGCCCCCCA | 17 | 7263 |
| HSV1-UL54-501 | - | CGGCGCCCCCCAUGGCG | 17 | 7264 |
| HSV1-UL54-502 | - | CCCCCCAUGGCGAGGCG | 17 | 7265 |
| HSV1-UL54-503 | - | CAUGGCGAGGCGUGGCG | 17 | 7266 |
| HSV1-UL54-504 | - | GAGCAGCCCGACCCACC | 17 | 7267 |
| HSV1-UL54-505 | - | CAGCCCGACCCACCCGG | 17 | 7268 |
| HSV1-UL54-506 | - | ACCCACCCGGAGGCCAG | 17 | 7269 |
| HSV1-UL54-507 | - | CCGGAGGCCAGCGGACA | 17 | 7270 |
| HSV1-UL54-508 | - | CGGAGGCCAGCGGACAC | 17 | 7271 |
| HSV1-UL54-509 | - | GGAGGCCAGCGGACACG | 17 | 7272 |
| HSV1-UL54-510 | - | CCCCCCGCUAAUGACGC | 17 | 7273 |
| HSV1-UL54-511 | - | GAUUGCCCCCCCGCCCG | 17 | 7274 |
| HSV1-UL54-512 | - | CGCGGACCCCCGCGCCC | 17 | 7275 |
| HSV1-UL54-513 | - | CCCCCGCGCCCCGGCCC | 17 | 7276 |
| HSV1-UL54-514 | - | CCCGGCCCCGGAGCGAA | 17 | 7277 |
| HSV1-UL54-515 | - | CCAUCGACGCCACCACG | 17 | 7278 |
| HSV1-UL54-516 | - | CGACGCCACCACGCGGU | 17 | 7279 |
| HSV1-UL54-517 | - | CUCCAUCUCCGAGCGCG | 17 | 7280 |
| HSV1-UL54-518 | - | CAUCUCCGAGCGCGCGG | 17 | 7281 |
| HSV1-UL54-519 | - | CGCAUCAGCGAGAGCUU | 17 | 7282 |
| HSV1-UL54-520 | - | CUUUGGCCGCAGCGCAC | 17 | 7283 |
| HSV1-UL54-521 | - | GUCAUGCACGACCCCUU | 17 | 7284 |
| HSV1-UL54-522 | - | UCAUGCACGACCCCUUU | 17 | 7285 |
| HSV1-UL54-523 | - | CAUGCACGACCCCUUUG | 17 | 7286 |
| HSV1-UL54-524 | - | AUGCACGACCCCUUUGG | 17 | 7287 |
| HSV1-UL54-525 | - | UGCACGACCCCUUUGGG | 17 | 7288 |
| HSV1-UL54-526 | - | CCGCCGCGAAUAGCCCC | 17 | 7289 |
| HSV1-UL54-527 | - | CGCCGCGAAUAGCCCCU | 17 | 7290 |
| HSV1-UL54-528 | - | GAAUAGCCCCUGGGCCC | 17 | 7291 |
| HSV1-UL54-529 | - | CCCCUGGGCCCCGGUGC | 17 | 7292 |
| HSV1-UL54-530 | - | CUGGGCCCCGGUGCUGG | 17 | 7293 |
| HSV1-UL54-531 | - | UGGGCCCCGGUGCUGGC | 17 | 7294 |
| HSV1-UL54-532 | - | CCGGUGCUGGCGGGCCA | 17 | 7295 |
| HSV1-UL54-533 | - | GUGCUGGCGGGCCAAGG | 17 | 7296 |
| HSV1-UL54-534 | - | UGCUGGCGGGCCAAGGA | 17 | 7297 |
| HSV1-UL54-535 | - | UUGACGCCGAGACCAGA | 17 | 7298 |
| HSV1-UL54-536 | - | UGACGCCGAGACCAGAC | 17 | 7299 |
| HSV1-UL54-537 | - | AGACCAGACGGGUCUCC | 17 | 7300 |
| HSV1-UL54-538 | - | GACCAGACGGGUCUCCU | 17 | 7301 |
| HSV1-UL54-539 | - | GGUCUCCUGGGAAACCU | 17 | 7302 |
| HSV1-UL54-540 | - | GAAACCUUGGUCGCCCA | 17 | 7303 |
| HSV1-UL54-541 | - | CUCUAUCGCACUUUUGC | 17 | 7304 |
| HSV1-UL54-542 | - | CUUUUGCCGGCAAUCCU | 17 | 7305 |
| HSV1-UL54-543 | - | UUUUGCCGGCAAUCCUC | 17 | 7306 |
| HSV1-UL54-544 | - | GGCCGCAUCGACCGCCA | 17 | 7307 |
| HSV1-UL54-545 | - | CCAAGAAAAUUUCAUCG | 17 | 7308 |
| HSV1-UL54-546 | - | AAAUUUCAUCGAGGCGC | 17 | 7309 |
| HSV1-UL54-547 | - | CUCCGCCGACGAGACGC | 17 | 7310 |
| HSV1-UL54-548 | - | CCGACGAGACGCUGGCG | 17 | 7311 |
| HSV1-UL54-549 | - | CCUGCCGCUGCGCCCCC | 17 | 7312 |
| HSV1-UL54-550 | - | CCCCAGGACCCCAUUAU | 17 | 7313 |
| HSV1-UL54-551 | - | CCCAGGACCCCAUUAUC | 17 | 7314 |
| HSV1-UL54-552 | - | CAUUAUCGGGACGACCG | 17 | 7315 |
| HSV1-UL54-553 | - | GACGACCGCGGCUGUGC | 17 | 7316 |
| HSV1-UL54-554 | - | ACCUCGCCACGCGCCUG | 17 | 7317 |
| HSV1-UL54-555 | - | UCUCCAGUGCUACCUGA | 17 | 7318 |
| HSV1-UL54-556 | - | UGCUACCUGAAGGCGCG | 17 | 7319 |
| HSV1-UL54-557 | - | AAGGCGCGAGGCCUGUG | 17 | 7320 |
| HSV1-UL54-558 | - | GCGAGGCCUGUGCGGCC | 17 | 7321 |
| HSV1-UL54-559 | - | UGGACGAACUGUGUUCG | 17 | 7322 |
| HSV1-UL54-560 | - | ACGAACUGUGUUCGCGG | 17 | 7323 |
| HSV1-UL54-561 | - | GUGUUCGCGGCGGCGUC | 17 | 7324 |
| HSV1-UL54-562 | - | UUCGCGGCGGCGUCUGG | 17 | 7325 |
| HSV1-UL54-563 | - | GCGUCUGGCGGACAUUA | 17 | 7326 |
| HSV1-UL54-564 | - | CUUCGUGUUUGUCAUUC | 17 | 7327 |
| HSV1-UL54-565 | - | UGUUUGUCAUUCUGGCC | 17 | 7328 |
| HSV1-UL54-566 | - | GCCAACCGCGUCGAGCG | 17 | 7329 |
| HSV1-UL54-567 | - | CGUCGAGCGUGGCGUCG | 17 | 7330 |
| HSV1-UL54-568 | - | AUCGACUACGCGACCCU | 17 | 7331 |
| HSV1-UL54-569 | - | UACGCGACCCUUGGUGU | 17 | 7332 |
| HSV1-UL54-570 | - | ACGCGACCCUUGGUGUC | 17 | 7333 |
| HSV1-UL54-571 | - | CGCGACCCUUGGUGUCG | 17 | 7334 |
| HSV1-UL54-572 | - | ACCCUUGGUGUCGGGGU | 17 | 7335 |
| HSV1-UL54-573 | - | AUGCAUUUCUACCUCCC | 17 | 7336 |
| HSV1-UL54-574 | - | UGCAUUUCUACCUCCCC | 17 | 7337 |
| HSV1-UL54-575 | - | GCAUUUCUACCUCCCCG | 17 | 7338 |
| HSV1-UL54-576 | - | CCUCCCCGGGGCCUGCA | 17 | 7339 |
| HSV1-UL54-577 | - | CCCCGGGGCCUGCAUGG | 17 | 7340 |
| HSV1-UL54-578 | - | CCCGGGGCCUGCAUGGC | 17 | 7341 |
| HSV1-UL54-579 | - | CCUAGACACGCACCGCC | 17 | 7342 |
| HSV1-UL54-580 | - | UCGUGUCUGCGAGUUGA | 17 | 7343 |
| HSV1-UL54-581 | - | GCCCCCCCGUACGUGCA | 17 | 7344 |
| HSV1-UL54-582 | + | UAUUUGCCGUGCACGUA | 17 | 7345 |
| HSV1-UL54-583 | + | AUUUGCCGUGCACGUAC | 17 | 7346 |
| HSV1-UL54-584 | + | UUUGCCGUGCACGUACG | 17 | 7347 |
| HSV1-UL54-585 | + | UUGCCGUGCACGUACGG | 17 | 7348 |
| HSV1-UL54-586 | + | UGCCGUGCACGUACGGG | 17 | 7349 |
| HSV1-UL54-587 | + | GCCGUGCACGUACGGGG | 17 | 7350 |
| HSV1-UL54-588 | + | GGGGGCGACGAUGUGAC | 17 | 7351 |
| HSV1-UL54-589 | + | CACGACUCGAACACUCC | 17 | 7352 |
| HSV1-UL54-590 | + | GACUCGAACACUCCUGG | 17 | 7353 |
| HSV1-UL54-591 | + | CCUGGCGGUGCGUGUCU | 17 | 7354 |
| HSV1-UL54-592 | + | UGUCUAGGAUUUCGAUC | 17 | 7355 |
| HSV1-UL54-593 | + | GAUCAGGCCCGCCAUGC | 17 | 7356 |
| HSV1-UL54-594 | + | GCCCGCCAUGCAGGCCC | 17 | 7357 |
| HSV1-UL54-595 | + | CCCGCCAUGCAGGCCCC | 17 | 7358 |
| HSV1-UL54-596 | + | CCGCCAUGCAGGCCCCG | 17 | 7359 |
| HSV1-UL54-597 | + | CCAUGCAGGCCCCGGGG | 17 | 7360 |
| HSV1-UL54-598 | + | CUCCGACCCCGACACCA | 17 | 7361 |
| HSV1-UL54-599 | + | UCCGACCCCGACACCAA | 17 | 7362 |
| HSV1-UL54-600 | + | CGACGCCACGCUCGACG | 17 | 7363 |
| HSV1-UL54-601 | + | GCCACGCUCGACGCGGU | 17 | 7364 |
| HSV1-UL54-602 | + | GACGCGGUUGGCGAGCC | 17 | 7365 |
| HSV1-UL54-603 | + | CAGAAUGACAAACACGA | 17 | 7366 |
| HSV1-UL54-604 | + | GCGAACACAGUUCGUCC | 17 | 7367 |
| HSV1-UL54-605 | + | GUUCGUCCAGGCCGCAC | 17 | 7368 |
| HSV1-UL54-606 | + | ACAGGCCUCGCGCCUUC | 17 | 7369 |
| HSV1-UL54-607 | + | GCGCCUUCAGGUAGCAC | 17 | 7370 |
| HSV1-UL54-608 | + | CAGGUAGCACUGGAGAA | 17 | 7371 |
| HSV1-UL54-609 | + | AGGUAGCACUGGAGAAA | 17 | 7372 |
| HSV1-UL54-610 | + | ACUGGAGAAAGGGCCGC | 17 | 7373 |
| HSV1-UL54-611 | + | AAAGGGCCGCAGGCGCG | 17 | 7374 |
| HSV1-UL54-612 | + | GCCGCAGGCGCGUGGCG | 17 | 7375 |
| HSV1-UL54-613 | + | GUUAUCCAGCACAGCCG | 17 | 7376 |
| HSV1-UL54-614 | + | CGCGGUCGUCCCGAUAA | 17 | 7377 |
| HSV1-UL54-615 | + | GCGGUCGUCCCGAUAAU | 17 | 7378 |
| HSV1-UL54-616 | + | CGGUCGUCCCGAUAAUG | 17 | 7379 |
| HSV1-UL54-617 | + | UCCCGAUAAUGGGGUCC | 17 | 7380 |
| HSV1-UL54-618 | + | CCCGAUAAUGGGGUCCU | 17 | 7381 |
| HSV1-UL54-619 | + | CCGAUAAUGGGGUCCUG | 17 | 7382 |
| HSV1-UL54-620 | + | CGAUAAUGGGGUCCUGG | 17 | 7383 |
| HSV1-UL54-621 | + | GGGUCCUGGGGGCGCAG | 17 | 7384 |
| HSV1-UL54-622 | + | CCUGGGGGCGCAGCGGC | 17 | 7385 |
| HSV1-UL54-623 | + | GGCGCAGCGGCAGGUUG | 17 | 7386 |
| HSV1-UL54-624 | + | GCAGCGGCAGGUUGUGG | 17 | 7387 |
| HSV1-UL54-625 | + | CCACGCCAGCGUCUCGU | 17 | 7388 |
| HSV1-UL54-626 | + | CGCCAGCGUCUCGUCGG | 17 | 7389 |
| HSV1-UL54-627 | + | CAGCGUCUCGUCGGCGG | 17 | 7390 |
| HSV1-UL54-628 | + | CCUCGAUGAAAUUUUCU | 17 | 7391 |
| HSV1-UL54-629 | + | CAGCACGCAGUCGCGCA | 17 | 7392 |
| HSV1-UL54-630 | + | GCAGUCGCGCAUGGCCU | 17 | 7393 |
| HSV1-UL54-631 | + | GUCGCGCAUGGCCUUGG | 17 | 7394 |
| HSV1-UL54-632 | + | GGCCUUGGCGGUCGAUG | 17 | 7395 |
| HSV1-UL54-633 | + | GCGGUCGAUGCGGCCCG | 17 | 7396 |
| HSV1-UL54-634 | + | UGCGGCCCGAGGAUUGC | 17 | 7397 |
| HSV1-UL54-635 | + | CGGCAAAAGUGCGAUAG | 17 | 7398 |
| HSV1-UL54-636 | + | AAAGUGCGAUAGAGGCU | 17 | 7399 |
| HSV1-UL54-637 | + | AAGUGCGAUAGAGGCUC | 17 | 7400 |
| HSV1-UL54-638 | + | GAUAGAGGCUCGGGCCG | 17 | 7401 |
| HSV1-UL54-639 | + | AUAGAGGCUCGGGCCGU | 17 | 7402 |
| HSV1-UL54-640 | + | CGGGCCGUGGGCGACCA | 17 | 7403 |
| HSV1-UL54-641 | + | GGCGACCAAGGUUUCCC | 17 | 7404 |
| HSV1-UL54-642 | + | UUCCCAGGAGACCCGUC | 17 | 7405 |
| HSV1-UL54-643 | + | GGAGACCCGUCUGGUCU | 17 | 7406 |
| HSV1-UL54-644 | + | UCUGGUCUCGGCGUCAA | 17 | 7407 |
| HSV1-UL54-645 | + | CUGGUCUCGGCGUCAAA | 17 | 7408 |
| HSV1-UL54-646 | + | CGUCAAAGGGCCCUCCU | 17 | 7409 |
| HSV1-UL54-647 | + | CCUUGGCCCGCCAGCAC | 17 | 7410 |
| HSV1-UL54-648 | + | CUUGGCCCGCCAGCACC | 17 | 7411 |
| HSV1-UL54-649 | + | UUGGCCCGCCAGCACCG | 17 | 7412 |
| HSV1-UL54-650 | + | CGCCAGCACCGGGGCCC | 17 | 7413 |
| HSV1-UL54-651 | + | GCCAGCACCGGGGCCCA | 17 | 7414 |
| HSV1-UL54-652 | + | CCAGCACCGGGGCCCAG | 17 | 7415 |
| HSV1-UL54-653 | + | GGCCCAGGGGCUAUUCG | 17 | 7416 |
| HSV1-UL54-654 | + | CCAGGGGCUAUUCGCGG | 17 | 7417 |
| HSV1-UL54-655 | + | CAGGGGCUAUUCGCGGC | 17 | 7418 |
| HSV1-UL54-656 | + | CUAUUCGCGGCGGGAAA | 17 | 7419 |
| HSV1-UL54-657 | + | AAACGGCUGCCCCCCAA | 17 | 7420 |
| HSV1-UL54-658 | + | AACGGCUGCCCCCCAAA | 17 | 7421 |
| HSV1-UL54-659 | + | ACGGCUGCCCCCCAAAG | 17 | 7422 |
| HSV1-UL54-660 | + | GCAUGACCUGUGCGCUG | 17 | 7423 |
| HSV1-UL54-661 | + | CAAAGCUCUCGCUGAUG | 17 | 7424 |
| HSV1-UL54-662 | + | GUCGACCGCCGCGCGCU | 17 | 7425 |
| HSV1-UL54-663 | + | CGCCGCGCGCUCGGAGA | 17 | 7426 |
| HSV1-UL54-664 | + | GCUCGGAGAUGGAGCGC | 17 | 7427 |
| HSV1-UL54-665 | + | GCGCAGGACCAACCGCG | 17 | 7428 |
| HSV1-UL54-666 | + | CAGGACCAACCGCGUGG | 17 | 7429 |
| HSV1-UL54-667 | + | CCGCGUGGUGGCGUCGA | 17 | 7430 |
| HSV1-UL54-668 | + | GGUGGCGUCGAUGGUGU | 17 | 7431 |
| HSV1-UL54-669 | + | GGCGUCGAUGGUGUCGG | 17 | 7432 |
| HSV1-UL54-670 | + | GUCGAUGGUGUCGGCGG | 17 | 7433 |
| HSV1-UL54-671 | + | UCGAUGGUGUCGGCGGC | 17 | 7434 |
| HSV1-UL54-672 | + | GCGGGCGCCUUUCGCUC | 17 | 7435 |
| HSV1-UL54-673 | + | CGGGCGCCUUUCGCUCC | 17 | 7436 |
| HSV1-UL54-674 | + | GGGCGCCUUUCGCUCCG | 17 | 7437 |
| HSV1-UL54-675 | + | GCCUUUCGCUCCGGGGC | 17 | 7438 |
| HSV1-UL54-676 | + | CCUUUCGCUCCGGGGCC | 17 | 7439 |
| HSV1-UL54-677 | + | CUUUCGCUCCGGGGCCG | 17 | 7440 |
| HSV1-UL54-678 | + | GCUCCGGGGCCGGGGCG | 17 | 7441 |
| HSV1-UL54-679 | + | CUCCGGGGCCGGGGCGC | 17 | 7442 |
| HSV1-UL54-680 | + | UCCGGGGCCGGGGCGCG | 17 | 7443 |
| HSV1-UL54-681 | + | CCGGGGCCGGGGCGCGG | 17 | 7444 |
| HSV1-UL54-682 | + | CGGGGCGCGGGGGUCCG | 17 | 7445 |
| HSV1-UL54-683 | + | GGGGCGCGGGGGUCCGC | 17 | 7446 |
| HSV1-UL54-684 | + | GCGCGGGGGUCCGCGGG | 17 | 7447 |
| HSV1-UL54-685 | + | CGCGGGGGUCCGCGGGC | 17 | 7448 |
| HSV1-UL54-686 | + | GCGGGGGUCCGCGGGCG | 17 | 7449 |
| HSV1-UL54-687 | + | CGGGGGUCCGCGGGCGG | 17 | 7450 |
| HSV1-UL54-688 | + | GGGGGUCCGCGGGCGGG | 17 | 7451 |
| HSV1-UL54-689 | + | GGGGUCCGCGGGCGGGG | 17 | 7452 |
| HSV1-UL54-690 | + | AUCGCCAGCGUCAUUAG | 17 | 7453 |
| HSV1-UL54-691 | + | UCGCCAGCGUCAUUAGC | 17 | 7454 |
| HSV1-UL54-692 | + | CGCCAGCGUCAUUAGCG | 17 | 7455 |
| HSV1-UL54-693 | + | GCCAGCGUCAUUAGCGG | 17 | 7456 |
| HSV1-UL54-694 | + | CCAGCGUCAUUAGCGGG | 17 | 7457 |
| HSV1-UL54-695 | + | CAGCGUCAUUAGCGGGG | 17 | 7458 |
| HSV1-UL54-696 | + | AGCGUCAUUAGCGGGGG | 17 | 7459 |
| HSV1-UL54-697 | + | UUAGCGGGGGGGGUGCU | 17 | 7460 |
| HSV1-UL54-698 | + | GCACGCCCCGUGUCCGC | 17 | 7461 |
| HSV1-UL54-699 | + | CCGUGUCCGCUGGCCUC | 17 | 7462 |
| HSV1-UL54-700 | + | CGUGUCCGCUGGCCUCC | 17 | 7463 |
| HSV1-UL54-701 | + | GUCCGCUGGCCUCCGGG | 17 | 7464 |
| HSV1-UL54-702 | + | UCCGCUGGCCUCCGGGU | 17 | 7465 |
| HSV1-UL54-703 | + | CUGGCCUCCGGGUGGGU | 17 | 7466 |
| HSV1-UL54-704 | + | UGGCCUCCGGGUGGGUC | 17 | 7467 |
| HSV1-UL54-705 | + | CGCGCCACGCCUCGCCA | 17 | 7468 |
| HSV1-UL54-706 | + | GCGCCACGCCUCGCCAU | 17 | 7469 |
| HSV1-UL54-707 | + | CGCCACGCCUCGCCAUG | 17 | 7470 |
| HSV1-UL54-708 | + | GCCACGCCUCGCCAUGG | 17 | 7471 |
| HSV1-UL54-709 | + | CCACGCCUCGCCAUGGG | 17 | 7472 |
| HSV1-UL54-710 | + | CUCGCCAUGGGGGGCGC | 17 | 7473 |
| HSV1-UL54-711 | + | UCGCCAUGGGGGGCGCC | 17 | 7474 |
| HSV1-UL54-712 | + | CGCCAUGGGGGGCGCCG | 17 | 7475 |
| HSV1-UL54-713 | + | GUCCGUCCACCCCGCCC | 17 | 7476 |
| HSV1-UL54-714 | + | UCCGUCCACCCCGCCCC | 17 | 7477 |
| HSV1-UL54-715 | + | CCGUCCACCCCGCCCCG | 17 | 7478 |
| HSV1-UL54-716 | + | UCCACCCCGCCCCGGGG | 17 | 7479 |
| HSV1-UL54-717 | + | CCACCCCGCCCCGGGGC | 17 | 7480 |
| HSV1-UL54-718 | + | CACCCCGCCCCGGGGCG | 17 | 7481 |
| HSV1-UL54-719 | + | CCGGGGCGGGGUCCCCC | 17 | 7482 |
| HSV1-UL54-720 | + | CGGGGCGGGGUCCCCCA | 17 | 7483 |
| HSV1-UL54-721 | + | UCCCCCAGGGUUGCGAU | 17 | 7484 |

**Table 4D** provides exemplary targeting domains for knocking out the *UL54* gene selected according to the first tier parameters. The targeting domains are selected based on location within first 500bp of the coding sequence of the *UL54* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4D**

| 1st Tier | | | | |
|---|---|---|---|---|
| gRNA Name | DNA Strand | Targeting Domain | Target Site Length | Seq ID |
| HSV1-UL54-726 | - | CCUCUCCGACAGCGAUCUGG | 20 | 7487 |
| HSV1-UL54-727 | - | GGACAUGGAAGACCCCCACG | 20 | 7488 |
| HSV1-UL54-728 | - | GGAAGACCCCCACGGAGAGG | 20 | 7489 |
| HSV1-UL54-729 | - | ACGAGGACCCCCCCGAGCCG | 20 | 7490 |
| HSV1-UL54-730 | - | AGGACCCCCCCGAGCCGGCG | 20 | 7491 |
| HSV1-UL54-731 | - | CGGGGAGUGUUCCUCGUCGG | 20 | 7492 |
| HSV1-UL54-5 | - | UUCCUCGUCGGACGAGGACA | 20 | 6768 |
| HSV1-UL54-733 | - | AUAUGCUAAUUGACCUCGGC | 20 | 7493 |
| HSV1-UL54-734 | - | UGGACCUCUCCGACAGCGAU | 20 | 7494 |
| HSV1-UL54-735 | - | UCUCCGACAGCGAUCUGGAC | 20 | 7495 |
| HSV1-UL54-736 | - | CGAUCUGGACGAGGACCCCC | 20 | 7496 |
| HSV1-UL54-84 | - | CGAGGACCCCCCCGAGCCGG | 20 | 6847 |
| HSV1-UL54-738 | - | CGGAGAGCCGCCGCGACGAC | 20 | 7497 |
| HSV1-UL54-85 | - | GGAGAGCCGCCGCGACGACC | 20 | 6848 |
| HSV1-UL54-740 | - | GCCGCCGCGACGACCUGGAA | 20 | 7498 |
| HSV1-UL54-741 | - | CGACCUGGAAUCGGACAGCA | 20 | 7499 |
| HSV1-UL54-86 | - | GACCUGGAAUCGGACAGCAG | 20 | 6849 |
| HSV1-UL54-87 | - | ACCUGGAAUCGGACAGCAGC | 20 | 6850 |
| HSV1-UL54-88 | - | CCUGGAAUCGGACAGCAGCG | 20 | 6851 |
| HSV1-UL54-745 | - | GCAGCGGGGAGUGUUCCUCG | 20 | 7500 |
| HSV1-UL54-746 | - | GGGAGUGUUCCUCGUCGGAC | 20 | 7501 |
| HSV1-UL54-747 | - | GUUCCUCGUCGGACGAGGAC | 20 | 7502 |
| HSV1-UL54-748 | - | CGAGGACAUGGAAGACCCCC | 20 | 7503 |
| HSV1-UL54-90 | - | GAGGACAUGGAAGACCCCCA | 20 | 6853 |
| HSV1-UL54-750 | - | ACAUGGAAGACCCCCACGGA | 20 | 7504 |
| HSV1-UL54-751 | - | ACCCCCACGGAGAGGACGGA | 20 | 7505 |
| HSV1-UL54-93 | - | CCCCCACGGAGAGGACGGAC | 20 | 6856 |
| HSV1-UL54-753 | - | GGCGGUCCGCCCGUCUCGUC | 20 | 7506 |
| HSV1-UL54-754 | - | GCACCCAGACGCCUCGUCCG | 20 | 7507 |
| HSV1-UL54-8 | - | CACCCAGACGCCUCGUCCGA | 20 | 6771 |
| HSV1-UL54-756 | - | CGCCUCGUCCGACGGAGCGG | 20 | 7508 |
| HSV1-UL54-757 | - | CCACAGUGUGUGGUCGCGCC | 20 | 7509 |
| HSV1-UL54-98 | - | CACAGUGUGUGGUCGCGCCU | 20 | 6861 |
| HSV1-UL54-759 | - | CCGGCGACCGUCUUGCUCCC | 20 | 7510 |
| HSV1-UL54-760 | - | GUCUUGCUCCCCCGAGCAGC | 20 | 7511 |
| HSV1-UL54-99 | - | UCUUGCUCCCCCGAGCAGCA | 20 | 6862 |
| HSV1-UL54-100 | - | CUUGCUCCCCCGAGCAGCAC | 20 | 6863 |
| HSV1-UL54-763 | - | CAAAGCCCAGCCUGCCCGCG | 20 | 7512 |
| HSV1-UL54-764 | - | GCCUGCCCGCGGCGGACGCC | 20 | 7513 |
| HSV1-UL54-765 | - | GGCGGACGCCGUGGGCGUCG | 20 | 7514 |
| HSV1-UL54-108 | - | GCGGACGCCGUGGGCGUCGC | 20 | 6871 |
| HSV1-UL54-767 | - | CGCCGUGGGCGUCGCAGGGG | 20 | 7515 |
| HSV1-UL54-111 | - | GCCGUGGGCGUCGCAGGGGU | 20 | 6874 |
| HSV1-UL54-769 | - | GGGUCGGGGUCGCGGUGGUC | 20 | 7516 |
| HSV1-UL54-116 | - | GGUCGGGGUCGCGGUGGUCC | 20 | 6879 |
| HSV1-UL54-771 | - | CCGGGGCUGCCGAUGGUUUG | 20 | 7517 |
| HSV1-UL54-772 | + | GACCACCGCGACCCCGACCC | 20 | 7518 |
| HSV1-UL54-773 | + | GCGACGCCCACGGCGUCCGC | 20 | 7519 |
| HSV1-UL54-137 | + | UGCUGCUCGGGGGAGCAAGA | 20 | 6900 |
| HSV1-UL54-775 | + | GGCGCGACCACACACUGUGG | 20 | 7520 |
| HSV1-UL54-776 | + | CAAACCAUCGGCAGCCCCGG | 20 | 7521 |
| HSV1-UL54-777 | + | GGACCACCGCGACCCCGACC | 20 | 7522 |
| HSV1-UL54-778 | + | ACCCCUGCGACGCCCACGGC | 20 | 7523 |
| HSV1-UL54-779 | + | CCCCUGCGACGCCCACGGCG | 20 | 7524 |
| HSV1-UL54-780 | + | CGACGCCCACGGCGUCCGCC | 20 | 7525 |
| HSV1-UL54-123 | + | GGCGUCCGCCGCGGGCAGGC | 20 | 6886 |
| HSV1-UL54-782 | + | CGCGGGCAGGCUGGGCUUUG | 20 | 7526 |
| HSV1-UL54-783 | + | GUGCUGCUCGGGGGAGCAAG | 20 | 7527 |
| HSV1-UL54-784 | + | GCUGCUCGGGGGAGCAAGAC | 20 | 7528 |
| HSV1-UL54-785 | + | GGGAGCAAGACGGUCGCCGG | 20 | 7529 |
| HSV1-UL54-139 | + | AGGCGCGACCACACACUGUG | 20 | 6902 |
| HSV1-UL54-787 | + | CCGCUCCGUCGGACGAGGCG | 20 | 7530 |
| HSV1-UL54-788 | + | UCGGACGAGGCGUCUGGGUG | 20 | 7531 |
| HSV1-UL54-145 | + | CGGACGAGGCGUCUGGGUGC | 20 | 6908 |
| HSV1-UL54-154 | + | GACGAGACGGGCGGACCGCC | 20 | 6917 |
| HSV1-UL54-791 | + | GUCCGUCCUCUCCGUGGGGG | 20 | 7532 |
| HSV1-UL54-792 | + | UCCGUCCUCUCCGUGGGGGU | 20 | 7533 |
| HSV1-UL54-793 | + | CCUCUCCGUGGGGGUCUUCC | 20 | 7534 |
| HSV1-UL54-794 | + | UCCGUGGGGGUCUUCCAUGU | 20 | 7535 |
| HSV1-UL54-795 | + | CGUGGGGGUCUUCCAUGUCC | 20 | 7536 |
| HSV1-UL54-796 | + | UGGGGGUCUUCCAUGUCCUC | 20 | 7537 |
| HSV1-UL54-797 | + | GGGGGUCUUCCAUGUCCUCG | 20 | 7538 |
| HSV1-UL54-798 | + | UGUCCUCGUCCGACGAGGAA | 20 | 7539 |
| HSV1-UL54-799 | + | GUCCUCGUCCGACGAGGAAC | 20 | 7540 |
| HSV1-UL54-800 | + | GUCCGACGAGGAACACUCCC | 20 | 7541 |
| HSV1-UL54-801 | + | CCGACGAGGAACACUCCCCG | 20 | 7542 |
| HSV1-UL54-802 | + | CGAGGAACACUCCCCGCUGC | 20 | 7543 |
| HSV1-UL54-803 | + | CGCUGCUGUCCGAUUCCAGG | 20 | 7544 |
| HSV1-UL54-804 | + | GCUGCUGUCCGAUUCCAGGU | 20 | 7545 |
| HSV1-UL54-805 | + | CUGCUGUCCGAUUCCAGGUC | 20 | 7546 |
| HSV1-UL54-806 | + | UGCUGUCCGAUUCCAGGUCG | 20 | 7547 |
| HSV1-UL54-807 | + | UUCCAGGUCGUCGCGGCGGC | 20 | 7548 |
| HSV1-UL54-808 | + | GGUCGUCGCGGCGGCUCUCC | 20 | 7549 |
| HSV1-UL54-809 | + | GUCGUCGCGGCGGCUCUCCG | 20 | 7550 |
| HSV1-UL54-810 | + | CGCCGGCUCGGGGGGGUCCU | 20 | 7551 |
| HSV1-UL54-811 | + | CCGGCUCGGGGGGGUCCUCG | 20 | 7552 |
| HSV1-UL54-812 | + | CGGCUCGGGGGGGUCCUCGU | 20 | 7553 |
| HSV1-UL54-813 | + | GGGGGUCCUCGUCCAGAUCG | 20 | 7554 |
| HSV1-UL54-814 | + | GUCCAGAUCGCUGUCGGAGA | 20 | 7555 |
| HSV1-UL54-815 | + | CCAGAUCGCUGUCGGAGAGG | 20 | 7556 |
| HSV1-UL54-816 | + | AUCGCUGUCGGAGAGGUCCA | 20 | 7557 |
| HSV1-UL54-817 | + | GCCGAGGUCAAUUAGCAUAU | 20 | 7558 |
| HSV1-UL54-818 | - | CUCCGUGGGGGUCUUCCAUG | 17 | 7559 |
| HSV1-UL54-796 | - | UGGGGGUCUUCCAUGUCCUC | 20 | 7537 |
| HSV1-UL54-798 | - | UGUCCUCGUCCGACGAGGAA | 20 | 7539 |
| HSV1-UL54-821 | - | CGUCCGACGAGGAACACUCC | 20 | 7560 |
| HSV1-UL54-822 | - | ACGAGGAACACUCCCCGCUG | 20 | 7561 |
| HSV1-UL54-803 | - | CGCUGCUGUCCGAUUCCAGG | 20 | 7544 |
| HSV1-UL54-804 | + | GCUGCUGUCCGAUUCCAGGU | 20 | 7545 |
| HSV1-UL54-805 | + | CUGCUGUCCGAUUCCAGGUC | 20 | 7546 |
| HSV1-UL54-826 | + | AUUCCAGGUCGUCGCGGCGG | 20 | 7562 |
| HSV1-UL54-808 | + | GGUCGUCGCGGCGGCUCUCC | 20 | 7549 |
| HSV1-UL54-811 | + | CCGGCUCGGGGGGGUCCUCG | 20 | 7552 |
| HSV1-UL54-829 | + | GCUCGGGGGGGUCCUCGUCC | 20 | 7563 |
| HSV1-UL54-38 | + | CGUCCAGAUCGCUGUCGGAG | 20 | 6801 |
| HSV1-UL54-39 | + | GAUCGCUGUCGGAGAGGUCC | 20 | 6802 |
| HSV1-UL54-832 | + | GGCCGAGGUCAAUUAGCAUA | 20 | 7564 |
| HSV1-UL54-833 | - | AGGUCAAUUAGCAUAUCAAU | 20 | 7565 |
| HSV1-UL54-167 | - | GAUAUGCUAAUUGACCU | 17 | 6930 |
| HSV1-UL54-168 | - | GCUAAUUGACCUCGGCC | 17 | 6931 |
| HSV1-UL54-41 | - | CCUCUCCGACAGCGAUC | 17 | 6804 |
| HSV1-UL54-42 | - | CGACAGCGAUCUGGACG | 17 | 6805 |
| HSV1-UL54-169 | - | CGAGGACCCCCCCGAGC | 17 | 6932 |
| HSV1-UL54-170 | - | GGACCCCCCCGAGCCGG | 17 | 6933 |
| HSV1-UL54-171 | - | GAGCCGCCGCGACGACC | 17 | 6934 |
| HSV1-UL54-43 | - | CCGCGACGACCUGGAAU | 17 | 6806 |
| HSV1-UL54-172 | - | CUGGAAUCGGACAGCAG | 17 | 6935 |
| HSV1-UL54-173 | - | UGGAAUCGGACAGCAGC | 17 | 6936 |
| HSV1-UL54-174 | - | GGAAUCGGACAGCAGCG | 17 | 6937 |
| HSV1-UL54-44 | - | CGGGGAGUGUUCCUCGU | 17 | 6807 |
| HSV1-UL54-175 | - | GUGUUCCUCGUCGGACG | 17 | 6938 |
| HSV1-UL54-45 | - | CUCGUCGGACGAGGACA | 17 | 6808 |
| HSV1-UL54-176 | - | GACAUGGAAGACCCCCA | 17 | 6939 |
| HSV1-UL54-177 | - | GGAAGACCCCCACGGAG | 17 | 6940 |
| HSV1-UL54-178 | - | GACCCCCACGGAGAGGA | 17 | 6941 |
| HSV1-UL54-179 | - | CCACGGAGAGGACGGAC | 17 | 6942 |
| HSV1-UL54-46 | - | ACUCGACGCCGCUCGCC | 17 | 6809 |
| HSV1-UL54-47 | - | CGACGCCGCUCGCCCGG | 17 | 6810 |
| HSV1-UL54-180 | - | UCUCGUCCAGAAGACCC | 17 | 6943 |
| HSV1-UL54-48 | - | CCAGACGCCUCGUCCGA | 17 | 6811 |
| HSV1-UL54-181 | - | CGCCUCGUCCGACGGAG | 17 | 6944 |
| HSV1-UL54-49 | - | UCGUCCGACGGAGCGGC | 17 | 6812 |
| HSV1-UL54-182 | - | CGUCCGACGGAGCGGCA | 17 | 6945 |
| HSV1-UL54-183 | - | CAGCGCCCCACAGUGUG | 17 | 6946 |
| HSV1-UL54-184 | - | AGUGUGUGGUCGCGCCU | 17 | 6947 |
| HSV1-UL54-50 | - | GUGUGUGGUCGCGCCUC | 17 | 6813 |
| HSV1-UL54-51 | - | UGUGUGGUCGCGCCUCG | 17 | 6814 |
| HSV1-UL54-52 | - | GGUCGCGCCUCGGGGCC | 17 | 6815 |
| HSV1-UL54-185 | + | UGCUCCCCCGAGCAGCA | 17 | 6948 |
| HSV1-UL54-186 | + | GCUCCCCCGAGCAGCAC | 17 | 6949 |
| HSV1-UL54-53 | + | CUCCCCCGAGCAGCACG | 17 | 6816 |
| HSV1-UL54-187 | + | UCCCCCGAGCAGCACGG | 17 | 6950 |
| HSV1-UL54-188 | + | CGAGCAGCACGGGGGCA | 17 | 6951 |
| HSV1-UL54-189 | + | GCAGCACGGGGGCAAGG | 17 | 6952 |
| HSV1-UL54-190 | + | AAAGCCCAGCCUGCCCG | 17 | 6953 |
| HSV1-UL54-191 | + | GCCCAGCCUGCCCGCGG | 17 | 6954 |
| HSV1-UL54-192 | + | GCCCGCGGCGGACGCCG | 17 | 6955 |
| HSV1-UL54-193 | - | CCCGCGGCGGACGCCGU | 17 | 6956 |
| HSV1-UL54-194 | - | GACGCCGUGGGCGUCGC | 17 | 6957 |
| HSV1-UL54-195 | - | ACGCCGUGGGCGUCGCA | 17 | 6958 |
| HSV1-UL54-196 | - | CGCCGUGGGCGUCGCAG | 17 | 6959 |
| HSV1-UL54-197 | - | GUGGGCGUCGCAGGGGU | 17 | 6960 |
| HSV1-UL54-198 | - | UGGGCGUCGCAGGGGUC | 17 | 6961 |
| HSV1-UL54-199 | - | GGGCGUCGCAGGGGUCG | 17 | 6962 |
| HSV1-UL54-200 | - | CGCAGGGGUCGGGGUCG | 17 | 6963 |
| HSV1-UL54-201 | - | AGGGGUCGGGGUCGCGG | 17 | 6964 |
| HSV1-UL54-202 | - | CGGGGUCGCGGUGGUCC | 17 | 6965 |
| HSV1-UL54-203 | - | GGGGUCGCGGUGGUCCC | 17 | 6966 |
| HSV1-UL54-54 | - | GGGUCGCGGUGGUCCCG | 17 | 6817 |
| HSV1-UL54-204 | - | GGUCCCGGGGCUGCCGA | 17 | 6967 |
| HSV1-UL54-55 | - | GGCUGCCGAUGGUUUGU | 17 | 6818 |
| HSV1-UL54-56 | - | GUUUGUCGGACCCCCGC | 17 | 6819 |
| HSV1-UL54-888 | + | GCGGGGGUCCGACAAAC | 17 | 7566 |
| HSV1-UL54-889 | + | ACAAACCAUCGGCAGCC | 17 | 7567 |
| HSV1-UL54-890 | + | UCGGCAGCCCCGGGACC | 17 | 7568 |
| HSV1-UL54-891 | + | CGGGACCACCGCGACCC | 17 | 7569 |
| HSV1-UL54-892 | + | GGGACCACCGCGACCCC | 17 | 7570 |
| HSV1-UL54-893 | + | GGACCACCGCGACCCCG | 17 | 7571 |
| HSV1-UL54-894 | + | CCGCGACCCCGACCCCU | 17 | 7572 |
| HSV1-UL54-895 | + | CGACCCCGACCCCUGCG | 17 | 7573 |
| HSV1-UL54-896 | + | CGACCCCUGCGACGCCC | 17 | 7574 |
| HSV1-UL54-206 | + | GACCCCUGCGACGCCCA | 17 | 6969 |
| HSV1-UL54-898 | + | ACCCCUGCGACGCCCAC | 17 | 7575 |
| HSV1-UL54-899 | + | CUGCGACGCCCACGGCG | 17 | 7576 |
| HSV1-UL54-900 | + | UGCGACGCCCACGGCGU | 17 | 7577 |
| HSV1-UL54-901 | + | GCGACGCCCACGGCGUC | 17 | 7578 |
| HSV1-UL54-902 | + | ACGGCGUCCGCCGCGGG | 17 | 7579 |
| HSV1-UL54-208 | + | CGGCGUCCGCCGCGGGC | 17 | 6971 |
| HSV1-UL54-904 | + | CCGCGGGCAGGCUGGGC | 17 | 7580 |
| HSV1-UL54-905 | + | CGGGCAGGCUGGGCUUU | 17 | 7581 |
| HSV1-UL54-906 | + | CCUUGCCCCCGUGCUGC | 17 | 7582 |
| HSV1-UL54-60 | + | UGCCCCCGUGCUGCUCG | 17 | 6823 |
| HSV1-UL54-908 | + | CCGUGCUGCUCGGGGGA | 17 | 7583 |
| HSV1-UL54-909 | + | CGUGCUGCUCGGGGGAG | 17 | 7584 |

**Table 4E** provides exemplary targeting domains for knocking out the *UL54* gene selected according to the second tier parameters. The targeting domains are selected based on location within the coding sequence, but downstream of the first 500bp of the *UL54* gene. It is contemplated herein that the targeting domain hybridizes to the target domain through complementary base pairing. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4E**

| 2nd Tier | | | | |
|---|---|---|---|---|
| gRNA Name | DNA Strand | Targeting Domain | Target Site Length | Seq ID |
| HSV1-UL54-910 | - | CAGAACCAAUCGCAACCCUG | 20 | 7585 |
| HSV1-UL54-911 | - | CCACCCGGAGGCCAGCGGAC | 20 | 7586 |
| HSV1-UL54-912 | - | CAACCCUGGGGGACCCCGCC | 20 | 7587 |
| HSV1-UL54-254 | - | AACCCUGGGGGACCCCGCCC | 20 | 7017 |
| HSV1-UL54-914 | - | CUGGGGGACCCCGCCCCGGG | 20 | 7588 |
| HSV1-UL54-257 | - | UGGGGGACCCCGCCCCGGGG | 20 | 7020 |
| HSV1-UL54-916 | - | GGACCCCGCCCCGGGGCGGG | 20 | 7589 |
| HSV1-UL54-917 | - | CCCGCCCCGGGGCGGGGUGG | 20 | 7590 |
| HSV1-UL54-918 | - | CGGCCCCGGCGCCCCCCAUG | 20 | 7591 |
| HSV1-UL54-919 | - | UGGCGAGGCGUGGCGCGGCA | 20 | 7592 |
| HSV1-UL54-920 | - | CAGUGAGCAGCCCGACCCAC | 20 | 7593 |
| HSV1-UL54-268 | - | AGUGAGCAGCCCGACCCACC | 20 | 7031 |
| HSV1-UL54-922 | - | CCCGACCCACCCGGAGGCCA | 20 | 7594 |
| HSV1-UL54-271 | - | CACCCGGAGGCCAGCGGACA | 20 | 7034 |
| HSV1-UL54-924 | - | UGGCGAUUGCCCCCCCGCCC | 20 | 7595 |
| HSV1-UL54-925 | - | CGGACCCCCGCGCCCCGGCC | 20 | 7596 |
| HSV1-UL54-277 | - | GGACCCCCGCGCCCCGGCCC | 20 | 7040 |
| HSV1-UL54-927 | - | CCCCGCGCCCCGGCCCCGGA | 20 | 7597 |
| HSV1-UL54-928 | - | GUUGGUCCUGCGCUCCAUCU | 20 | 7598 |
| HSV1-UL54-929 | - | CGCGGCGGUCGACCGCAUCA | 20 | 7599 |
| HSV1-UL54-930 | - | CGGCGGUCGACCGCAUCAGC | 20 | 7600 |
| HSV1-UL54-931 | - | ACAGGUCAUGCACGACCCCU | 20 | 7601 |
| HSV1-UL54-285 | - | CAGGUCAUGCACGACCCCUU | 20 | 7048 |
| HSV1-UL54-286 | - | AGGUCAUGCACGACCCCUUU | 20 | 7049 |
| HSV1-UL54-287 | - | GGUCAUGCACGACCCCUUUG | 20 | 7050 |
| HSV1-UL54-935 | - | GGGGGCAGCCGUUUCCCGCC | 20 | 7602 |
| HSV1-UL54-936 | - | UUUCCCGCCGCGAAUAGCCC | 20 | 7603 |
| HSV1-UL54-937 | - | GCCCCUGGGCCCCGGUGCUG | 20 | 7604 |
| HSV1-UL54-938 | - | GGCCCCGGUGCUGGCGGGCC | 20 | 7605 |
| HSV1-UL54-296 | - | GCCCCGGUGCUGGCGGGCCA | 20 | 7059 |
| HSV1-UL54-940 | - | CCCGGUGCUGGCGGGCCAAG | 20 | 7606 |
| HSV1-UL54-941 | - | CCAAGGAGGGCCCUUUGACG | 20 | 7607 |
| HSV1-UL54-942 | - | CCCUUUGACGCCGAGACCAG | 20 | 7608 |
| HSV1-UL54-943 | - | GCCGAGACCAGACGGGUCUC | 20 | 7609 |
| HSV1-UL54-301 | - | CCGAGACCAGACGGGUCUCC | 20 | 7064 |
| HSV1-UL54-302 | - | CGAGACCAGACGGGUCUCCU | 20 | 7065 |
| HSV1-UL54-946 | - | AAACCUUGGUCGCCCACGGC | 20 | 7610 |
| HSV1-UL54-947 | - | CGCACUUUUGCCGGCAAUCC | 20 | 7611 |
| HSV1-UL54-948 | - | GCGCGACUGCGUGCUGCGCC | 20 | 7612 |
| HSV1-UL54-949 | - | GCUGCGCCAAGAAAAUUUCA | 20 | 7613 |
| HSV1-UL54-950 | - | CGAGGCGCUGGCCUCCGCCG | 20 | 7614 |
| HSV1-UL54-951 | - | ACAACCUGCCGCUGCGCCCC | 20 | 7615 |
| HSV1-UL54-952 | - | GCGCCCCCAGGACCCCAUUA | 20 | 7616 |
| HSV1-UL54-314 | - | CGCCCCCAGGACCCCAUUAU | 20 | 7077 |
| HSV1-UL54-954 | - | UCGGGACGACCGCGGCUGUG | 20 | 7617 |
| HSV1-UL54-955 | - | GGCCCUUUCUCCAGUGCUAC | 20 | 7618 |
| HSV1-UL54-956 | - | CUCCAGUGCUACCUGAAGGC | 20 | 7619 |
| HSV1-UL54-957 | - | AGGCGCGAGGCCUGUGCGGC | 20 | 7620 |
| HSV1-UL54-958 | - | GCGAGGCCUGUGCGGCCUGG | 20 | 7621 |
| HSV1-UL54-959 | - | UGUGUUCGCGGCGGCGUCUG | 20 | 7622 |
| HSV1-UL54-960 | - | GGCGGCGUCUGGCGGACAUU | 20 | 7623 |
| HSV1-UL54-961 | - | GGCCAGGCUCGCCAACCGCG | 20 | 7624 |
| HSV1-UL54-962 | - | ACCGCGUCGAGCGUGGCGUC | 20 | 7625 |
| HSV1-UL54-331 | - | CCGCGUCGAGCGUGGCGUCG | 20 | 7094 |
| HSV1-UL54-964 | - | CGACUACGCGACCCUUGGUG | 20 | 7626 |
| HSV1-UL54-333 | - | GACUACGCGACCCUUGGUGU | 20 | 7096 |
| HSV1-UL54-966 | - | CGCGACCCUUGGUGUCGGGG | 20 | 7627 |
| HSV1-UL54-336 | - | GCGACCCUUGGUGUCGGGGU | 20 | 7099 |
| HSV1-UL54-968 | - | GACCCUUGGUGUCGGGGUCG | 20 | 7628 |
| HSV1-UL54-969 | - | CCCUUGGUGUCGGGGUCGGA | 20 | 7629 |
| HSV1-UL54-970 | - | GAAGAUGCAUUUCUACCUCC | 20 | 7630 |
| HSV1-UL54-337 | - | AAGAUGCAUUUCUACCUCCC | 20 | 7100 |
| HSV1-UL54-972 | - | ACCUCCCCGGGGCCUGCAUG | 20 | 7631 |
| HSV1-UL54-973 | - | GGCCUGCAUGGCGGGCCUGA | 20 | 7632 |
| HSV1-UL54-974 | - | AAAUCCUAGACACGCACCGC | 20 | 7633 |
| HSV1-UL54-343 | - | AAUCCUAGACACGCACCGCC | 20 | 7106 |
| HSV1-UL54-976 | - | ACACGCACCGCCAGGAGUGU | 20 | 7634 |
| HSV1-UL54-977 | - | GGAGUGUUCGAGUCGUGUCU | 20 | 7635 |
| HSV1-UL54-978 | + | ACACUCCUGGCGGUGCGUGU | 20 | 7636 |
| HSV1-UL54-979 | + | GGGAGGUAGAAAUGCAUCUU | 20 | 7637 |
| HSV1-UL54-980 | + | ACACCAAGGGUCGCGUAGUC | 20 | 7638 |
| HSV1-UL54-353 | + | AGACACGACUCGAACACUCC | 20 | 7116 |
| HSV1-UL54-982 | + | GGCGGUGCGUGUCUAGGAUU | 20 | 7639 |
| HSV1-UL54-983 | + | GCGGUGCGUGUCUAGGAUUU | 20 | 7640 |
| HSV1-UL54-984 | + | UCAGGCCCGCCAUGCAGGCC | 20 | 7641 |
| HSV1-UL54-985 | + | CAUGCAGGCCCCGGGGAGGU | 20 | 7642 |
| HSV1-UL54-986 | + | GGAGGUAGAAAUGCAUCUUC | 20 | 7643 |
| HSV1-UL54-987 | + | UCCGACCCCGACACCAAGGG | 20 | 7644 |
| HSV1-UL54-988 | + | CGACCCCGACACCAAGGGUC | 20 | 7645 |
| HSV1-UL54-989 | + | ACCCCGACACCAAGGGUCGC | 20 | 7646 |
| HSV1-UL54-990 | + | CCCCGACACCAAGGGUCGCG | 20 | 7647 |
| HSV1-UL54-991 | + | CACCAAGGGUCGCGUAGUCG | 20 | 7648 |
| HSV1-UL54-992 | + | CGACGCCACGCUCGACGCGG | 20 | 7649 |
| HSV1-UL54-365 | + | GACGCCACGCUCGACGCGGU | 20 | 7128 |
| HSV1-UL54-994 | + | CGCGGUUGGCGAGCCUGGCC | 20 | 7650 |
| HSV1-UL54-995 | + | AAUGUCCGCCAGACGCCGCC | 20 | 7651 |
| HSV1-UL54-996 | + | CAGACGCCGCCGCGAACACA | 20 | 7652 |
| HSV1-UL54-997 | + | CCAGGCCGCACAGGCCUCGC | 20 | 7653 |
| HSV1-UL54-998 | + | GCCGCACAGGCCUCGCGCCU | 20 | 7654 |
| HSV1-UL54-999 | + | GCCUUCAGGUAGCACUGGAG | 20 | 7655 |
| HSV1-UL54-1000 | + | GUAGCACUGGAGAAAGGGCC | 20 | 7656 |
| HSV1-UL54-1001 | + | CACAGCCGCGGUCGUCCCGA | 20 | 7657 |
| HSV1-UL54-1002 | + | AUAAUGGGGUCCUGGGGGCG | 20 | 7658 |
| HSV1-UL54-1003 | + | UAAUGGGGUCCUGGGGGCGC | 20 | 7659 |
| HSV1-UL54-1004 | + | GGGGCGCAGCGGCAGGUUGU | 20 | 7660 |
| HSV1-UL54-1005 | + | CGGCGGAGGCCAGCGCCUCG | 20 | 7661 |
| HSV1-UL54-1006 | + | UGAAAUUUUCUUGGCGCAGC | 20 | 7662 |
| HSV1-UL54-1007 | + | GGCGCAGCACGCAGUCGCGC | 20 | 7663 |
| HSV1-UL54-1008 | + | GGAUUGCCGGCAAAAGUGCG | 20 | 7664 |
| HSV1-UL54-1009 | + | GCCGUGGGCGACCAAGGUUU | 20 | 7665 |
| HSV1-UL54-1010 | + | AGGAGACCCGUCUGGUCUCG | 20 | 7666 |
| HSV1-UL54-1011 | + | GGAGACCCGUCUGGUCUCGG | 20 | 7667 |
| HSV1-UL54-1012 | + | GAGACCCGUCUGGUCUCGGC | 20 | 7668 |
| HSV1-UL54-1013 | + | CUGGUCUCGGCGUCAAAGGG | 20 | 7669 |
| HSV1-UL54-1014 | + | CGUCAAAGGGCCCUCCUUGG | 20 | 7670 |
| HSV1-UL54-1015 | + | CUUGGCCCGCCAGCACCGGG | 20 | 7671 |
| HSV1-UL54-1016 | + | UGGCCCGCCAGCACCGGGGC | 20 | 7672 |
| HSV1-UL54-1017 | + | GGCCCGCCAGCACCGGGGCC | 20 | 7673 |
| HSV1-UL54-1018 | + | CAGCACCGGGGCCCAGGGGC | 20 | 7674 |
| HSV1-UL54-420 | + | GGGCUAUUCGCGGCGGGAAA | 20 | 7183 |
| HSV1-UL54-1020 | + | GGCGGGAAACGGCUGCCCCC | 20 | 7675 |
| HSV1-UL54-1021 | + | CAAAGGGGUCGUGCAUGACC | 20 | 7676 |
| HSV1-UL54-1022 | + | AAAGGGGUCGUGCAUGACCU | 20 | 7677 |
| HSV1-UL54-1023 | + | AAGGGGUCGUGCAUGACCUG | 20 | 7678 |
| HSV1-UL54-1024 | + | AGGGGUCGUGCAUGACCUGU | 20 | 7679 |
| HSV1-UL54-1025 | + | GCUGAUGCGGUCGACCGCCG | 20 | 7680 |
| HSV1-UL54-1026 | + | UGAUGCGGUCGACCGCCGCG | 20 | 7681 |
| HSV1-UL54-1027 | + | AGAUGGAGCGCAGGACCAAC | 20 | 7682 |
| HSV1-UL54-1028 | + | UCCGGGGCCGGGGCGCGGGG | 20 | 7683 |
| HSV1-UL54-1029 | + | GGGCCGGGGCGCGGGGGUCC | 20 | 7684 |
| HSV1-UL54-446 | + | GGCCGGGGCGCGGGGGUCCG | 20 | 7209 |
| HSV1-UL54-1031 | + | GGGCGGGGGGGCAAUCGCCA | 20 | 7685 |
| HSV1-UL54-1032 | + | UGUCCGCUGGCCUCCGGGUG | 20 | 7686 |
| HSV1-UL54-1033 | + | GUCCGCUGGCCUCCGGGUGG | 20 | 7687 |
| HSV1-UL54-1034 | + | UGGCCUCCGGGUGGGUCGGG | 20 | 7688 |
| HSV1-UL54-1035 | + | GGUGGGUCGGGCUGCUCACU | 20 | 7689 |
| HSV1-UL54-1036 | + | GUGGGUCGGGCUGCUCACUG | 20 | 7690 |
| HSV1-UL54-469 | + | UGCCGCGCCACGCCUCGCCA | 20 | 7232 |
| HSV1-UL54-1038 | + | CAUGGGGGGCGCCGGGGCCG | 20 | 7691 |
| HSV1-UL54-1039 | + | CCACCCCGCCCCGGGGCGGG | 20 | 7692 |
| HSV1-UL54-1040 | + | CCCGCCCCGGGGCGGGGUCC | 20 | 7693 |
| HSV1-UL54-484 | + | CCCCGGGGCGGGGUCCCCCA | 20 | 7247 |
| HSV1-UL54-1042 | + | CCCGGGGCGGGGUCCCCCAG | 20 | 7694 |
| HSV1-UL54-1043 | + | GGGCGGGGUCCCCCAGGGUU | 20 | 7695 |
| HSV1-UL54-1044 | + | GGCGGGGUCCCCCAGGGUUG | 20 | 7696 |
| HSV1-UL54-1045 | + | CAGGGUUGCGAUUGGUUCUG | 20 | 7697 |
| HSV1-UL54-1045 | + | CAGGGUUGCGAUUGGUUCUG | 20 | 7697 |
| HSV1-UL54-1045 | + | CAGGGUUGCGAUUGGUUCUG | 20 | 7697 |
| HSV1-UL54-1045 | + | CAGGGUUGCGAUUGGUUCUG | 20 | 7697 |
| HSV1-UL54-488 | - | AACCAAUCGCAACCCUG | 17 | 7251 |
| HSV1-UL54-1050 | - | CCCGGAGGCCAGCGGAC | 17 | 7698 |
| HSV1-UL54-1051 | - | CCCUGGGGGACCCCGCC | 17 | 7699 |
| HSV1-UL54-490 | - | CCUGGGGGACCCCGCCC | 17 | 7253 |
| HSV1-UL54-1053 | - | GGGGACCCCGCCCCGGG | 17 | 7700 |
| HSV1-UL54-493 | - | GGGACCCCGCCCCGGGG | 17 | 7256 |
| HSV1-UL54-1055 | - | CCCCGCCCCGGGGCGGG | 17 | 7701 |
| HSV1-UL54-1056 | - | GCCCCGGGGCGGGGUGG | 17 | 7702 |
| HSV1-UL54-1057 | - | CCCCGGCGCCCCCCAUG | 17 | 7703 |
| HSV1-UL54-1058 | - | CGAGGCGUGGCGCGGCA | 17 | 7704 |
| HSV1-UL54-1059 | - | UGAGCAGCCCGACCCAC | 17 | 7705 |
| HSV1-UL54-504 | - | GAGCAGCCCGACCCACC | 17 | 7267 |
| HSV1-UL54-1061 | - | GACCCACCCGGAGGCCA | 17 | 7706 |
| HSV1-UL54-507 | - | CCGGAGGCCAGCGGACA | 17 | 7270 |
| HSV1-UL54-1063 | - | CGAUUGCCCCCCCGCCC | 17 | 7707 |
| HSV1-UL54-1064 | - | ACCCCCGCGCCCCGGCC | 17 | 7708 |
| HSV1-UL54-513 | - | CCCCCGCGCCCCGGCCC | 17 | 7276 |
| HSV1-UL54-1066 | - | CGCGCCCCGGCCCCGGA | 17 | 7709 |
| HSV1-UL54-1067 | - | GGUCCUGCGCUCCAUCU | 17 | 7710 |
| HSV1-UL54-1068 | - | GGCGGUCGACCGCAUCA | 17 | 7711 |
| HSV1-UL54-1069 | - | CGGUCGACCGCAUCAGC | 17 | 7712 |
| HSV1-UL54-1070 | - | GGUCAUGCACGACCCCU | 17 | 7713 |
| HSV1-UL54-521 | - | GUCAUGCACGACCCCUU | 17 | 7284 |
| HSV1-UL54-522 | - | UCAUGCACGACCCCUUU | 17 | 7285 |
| HSV1-UL54-523 | - | CAUGCACGACCCCUUUG | 17 | 7286 |
| HSV1-UL54-1074 | - | GGCAGCCGUUUCCCGCC | 17 | 7714 |
| HSV1-UL54-1075 | - | CCCGCCGCGAAUAGCCC | 17 | 7715 |
| HSV1-UL54-1076 | - | CCUGGGCCCCGGUGCUG | 17 | 7716 |
| HSV1-UL54-1077 | - | CCCGGUGCUGGCGGGCC | 17 | 7717 |
| HSV1-UL54-532 | - | CCGGUGCUGGCGGGCCA | 17 | 7295 |
| HSV1-UL54-1079 | - | GGUGCUGGCGGGCCAAG | 17 | 7718 |
| HSV1-UL54-1080 | - | AGGAGGGCCCUUUGACG | 17 | 7719 |
| HSV1-UL54-1081 | - | UUUGACGCCGAGACCAG | 17 | 7720 |
| HSV1-UL54-1082 | - | GAGACCAGACGGGUCUC | 17 | 7721 |
| HSV1-UL54-537 | - | AGACCAGACGGGUCUCC | 17 | 7300 |
| HSV1-UL54-538 | - | GACCAGACGGGUCUCCU | 17 | 7301 |
| HSV1-UL54-1085 | - | CCUUGGUCGCCCACGGC | 17 | 7722 |
| HSV1-UL54-1086 | - | ACUUUUGCCGGCAAUCC | 17 | 7723 |
| HSV1-UL54-1087 | - | CGACUGCGUGCUGCGCC | 17 | 7724 |
| HSV1-UL54-1088 | - | GCGCCAAGAAAAUUUCA | 17 | 7725 |
| HSV1-UL54-1089 | - | GGCGCUGGCCUCCGCCG | 17 | 7726 |
| HSV1-UL54-1090 | - | ACCUGCCGCUGCGCCCC | 17 | 7727 |
| HSV1-UL54-1091 | - | CCCCCAGGACCCCAUUA | 17 | 7728 |
| HSV1-UL54-550 | - | CCCCAGGACCCCAUUAU | 17 | 7313 |
| HSV1-UL54-1093 | - | GGACGACCGCGGCUGUG | 17 | 7729 |
| HSV1-UL54-1094 | - | CCUUUCUCCAGUGCUAC | 17 | 7730 |
| HSV1-UL54-1095 | - | CAGUGCUACCUGAAGGC | 17 | 7731 |
| HSV1-UL54-1096 | - | CGCGAGGCCUGUGCGGC | 17 | 7732 |
| HSV1-UL54-1097 | - | AGGCCUGUGCGGCCUGG | 17 | 7733 |
| HSV1-UL54-1098 | - | GUUCGCGGCGGCGUCUG | 17 | 7734 |
| HSV1-UL54-1099 | - | GGCGUCUGGCGGACAUU | 17 | 7735 |
| HSV1-UL54-1100 | - | CAGGCUCGCCAACCGCG | 17 | 7736 |
| HSV1-UL54-1101 | - | GCGUCGAGCGUGGCGUC | 17 | 7737 |
| HSV1-UL54-567 | - | CGUCGAGCGUGGCGUCG | 17 | 7330 |
| HSV1-UL54-1103 | - | CUACGCGACCCUUGGUG | 17 | 7738 |
| HSV1-UL54-569 | - | UACGCGACCCUUGGUGU | 17 | 7332 |
| HSV1-UL54-1105 | - | GACCCUUGGUGUCGGGG | 17 | 7739 |
| HSV1-UL54-572 | - | ACCCUUGGUGUCGGGGU | 17 | 7335 |
| HSV1-UL54-1107 | - | CCUUGGUGUCGGGGUCG | 17 | 7740 |
| HSV1-UL54-1108 | - | UUGGUGUCGGGGUCGGA | 17 | 7741 |
| HSV1-UL54-1109 | - | GAUGCAUUUCUACCUCC | 17 | 7742 |
| HSV1-UL54-573 | - | AUGCAUUUCUACCUCCC | 17 | 7336 |
| HSV1-UL54-1111 | - | UCCCCGGGGCCUGCAUG | 17 | 7743 |
| HSV1-UL54-1112 | - | CUGCAUGGCGGGCCUGA | 17 | 7744 |
| HSV1-UL54-1113 | - | UCCUAGACACGCACCGC | 17 | 7745 |
| HSV1-UL54-579 | - | CCUAGACACGCACCGCC | 17 | 7342 |
| HSV1-UL54-1115 | - | CGCACCGCCAGGAGUGU | 17 | 7746 |
| HSV1-UL54-1116 | - | GUGUUCGAGUCGUGUCU | 17 | 7747 |
| HSV1-UL54-1117 | - | ACACUCCUGGCGGUGCG | 17 | 7748 |
| HSV1-UL54-1118 | - | GGGAGGUAGAAAUGCAU | 17 | 7749 |
| HSV1-UL54-1119 | - | ACACCAAGGGUCGCGUA | 17 | 7750 |
| HSV1-UL54-1120 | - | AGACACGACUCGAACAC | 17 | 7751 |
| HSV1-UL54-1121 | - | GGCGGUGCGUGUCUAGG | 17 | 7752 |
| HSV1-UL54-1122 | - | GCGGUGCGUGUCUAGGA | 17 | 7753 |
| HSV1-UL54-1123 | - | UCAGGCCCGCCAUGCAG | 17 | 7754 |
| HSV1-UL54-1124 | - | CAUGCAGGCCCCGGGGA | 17 | 7755 |
| HSV1-UL54-1125 | - | GGAGGUAGAAAUGCAUC | 17 | 7756 |
| HSV1-UL54-599 | - | UCCGACCCCGACACCAA | 17 | 7362 |
| HSV1-UL54-1127 | - | CGACCCCGACACCAAGG | 17 | 7757 |
| HSV1-UL54-1128 | - | ACCCCGACACCAAGGGU | 17 | 7758 |
| HSV1-UL54-1129 | - | CCCCGACACCAAGGGUC | 17 | 7759 |
| HSV1-UL54-1130 | - | CACCAAGGGUCGCGUAG | 17 | 7760 |
| HSV1-UL54-600 | - | CGACGCCACGCUCGACG | 17 | 7363 |
| HSV1-UL54-1132 | - | GACGCCACGCUCGACGC | 17 | 7761 |
| HSV1-UL54-1133 | - | CGCGGUUGGCGAGCCUG | 17 | 7762 |
| HSV1-UL54-1134 | - | AAUGUCCGCCAGACGCC | 17 | 7763 |
| HSV1-UL54-1135 | - | CAGACGCCGCCGCGAAC | 17 | 7764 |
| HSV1-UL54-1136 | - | CCAGGCCGCACAGGCCU | 17 | 7765 |
| HSV1-UL54-1137 | - | GCCGCACAGGCCUCGCG | 17 | 7766 |
| HSV1-UL54-1138 | - | GCCUUCAGGUAGCACUG | 17 | 7767 |
| HSV1-UL54-1139 | - | GUAGCACUGGAGAAAGG | 17 | 7768 |
| HSV1-UL54-1140 | - | CACAGCCGCGGUCGUCC | 17 | 7769 |
| HSV1-UL54-1141 | - | AUAAUGGGGUCCUGGGG | 17 | 7770 |
| HSV1-UL54-1142 | - | UAAUGGGGUCCUGGGGG | 17 | 7771 |
| HSV1-UL54-1143 | - | GGGGCGCAGCGGCAGGU | 17 | 7772 |
| HSV1-UL54-1144 | - | CGGCGGAGGCCAGCGCC | 17 | 7773 |
| HSV1-UL54-1145 | - | UGAAAUUUUCUUGGCGC | 17 | 7774 |
| HSV1-UL54-1146 | - | GGCGCAGCACGCAGUCG | 17 | 7775 |
| HSV1-UL54-1147 | - | GGAUUGCCGGCAAAAGU | 17 | 7776 |
| HSV1-UL54-1148 | - | GCCGUGGGCGACCAAGG | 17 | 7777 |
| HSV1-UL54-1149 | - | AGGAGACCCGUCUGGUC | 17 | 7778 |
| HSV1-UL54-643 | - | GGAGACCCGUCUGGUCU | 17 | 7406 |
| HSV1-UL54-1151 | - | GAGACCCGUCUGGUCUC | 17 | 7779 |
| HSV1-UL54-645 | - | CUGGUCUCGGCGUCAAA | 17 | 7408 |
| HSV1-UL54-646 | - | CGUCAAAGGGCCCUCCU | 17 | 7409 |
| HSV1-UL54-648 | - | CUUGGCCCGCCAGCACC | 17 | 7411 |
| HSV1-UL54-1155 | - | UGGCCCGCCAGCACCGG | 17 | 7780 |
| HSV1-UL54-1156 | - | GGCCCGCCAGCACCGGG | 17 | 7781 |
| HSV1-UL54-1157 | - | CAGCACCGGGGCCCAGG | 17 | 7782 |
| HSV1-UL54-1158 | - | GGGCUAUUCGCGGCGGG | 17 | 7783 |
| HSV1-UL54-1159 | - | GGCGGGAAACGGCUGCC | 17 | 7784 |
| HSV1-UL54-1160 | - | CAAAGGGGUCGUGCAUG | 17 | 7785 |
| HSV1-UL54-1161 | - | AAAGGGGUCGUGCAUGA | 17 | 7786 |
| HSV1-UL54-1162 | - | AAGGGGUCGUGCAUGAC | 17 | 7787 |
| HSV1-UL54-1163 | - | AGGGGUCGUGCAUGACC | 17 | 7788 |
| HSV1-UL54-1164 | - | GCUGAUGCGGUCGACCG | 17 | 7789 |
| HSV1-UL54-1165 | - | UGAUGCGGUCGACCGCC | 17 | 7790 |
| HSV1-UL54-1166 | - | AGAUGGAGCGCAGGACC | 17 | 7791 |
| HSV1-UL54-680 | - | UCCGGGGCCGGGGCGCG | 17 | 7443 |
| HSV1-UL54-1168 | - | GGGCCGGGGCGCGGGGG | 17 | 7792 |
| HSV1-UL54-1169 | - | GGCCGGGGCGCGGGGGU | 17 | 7793 |
| HSV1-UL54-1170 | - | GGGCGGGGGGGCAAUCG | 17 | 7794 |
| HSV1-UL54-1171 | - | UGUCCGCUGGCCUCCGG | 17 | 7795 |
| HSV1-UL54-701 | - | GUCCGCUGGCCUCCGGG | 17 | 7464 |
| HSV1-UL54-704 | - | UGGCCUCCGGGUGGGUC | 17 | 7467 |
| HSV1-UL54-1174 | - | GGUGGGUCGGGCUGCUC | 17 | 7796 |
| HSV1-UL54-1175 | - | GUGGGUCGGGCUGCUCA | 17 | 7797 |
| HSV1-UL54-1176 | - | UGCCGCGCCACGCCUCG | 17 | 7798 |
| HSV1-UL54-1177 | - | CAUGGGGGGCGCCGGGG | 17 | 7799 |
| HSV1-UL54-717 | - | CCACCCCGCCCCGGGGC | 17 | 7480 |
| HSV1-UL54-496 | - | CCCGCCCCGGGGCGGGG | 17 | 7259 |
| HSV1-UL54-1180 | - | CCCCGGGGCGGGGUCCC | 17 | 7800 |
| HSV1-UL54-1181 | - | CCCGGGGCGGGGUCCCC | 17 | 7801 |
| HSV1-UL54-1182 | - | GGGCGGGGUCCCCCAGG | 17 | 7802 |
| HSV1-UL54-1183 | - | GGCGGGGUCCCCCAGGG | 17 | 7803 |
| HSV1-UL54-1184 | - | CAGGGUUGCGAUUGGUU | 17 | 7804 |
| HSV1-UL54-1185 | - | AGGGUUGCGAUUGGUUC | 17 | 7805 |
| HSV1-UL54-1186 | - | GGGUUGCGAUUGGUUCU | 17 | 7806 |
| HSV1-UL54-1187 | - | GGUUGCGAUUGGUUCUG | 17 | 7807 |

**Table 4F** provides exemplary targeting domains for knocking out the *UL54* gene selected according to the first tier parameters. The targeting domains are selected based on location within first 500bp of the coding sequence of the *UL54* gene. It is contemplated herein that the targeting domain hybridizes to the target domain through complementary base pairing. Any of the targeting domains in the table can be used with a *N*. *meningitidis* Cas9 molecule that gives double stranded cleavage. Any of the targeting domains in the table can be used with a *N*. *meningitidis* Cas9 single-stranded break nucleases (nickases). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4F**

| 1st Tier | | | | |
|---|---|---|---|---|
| gRNA Name | DNA Strand | Targeting Domain | Target Site Length | Seq ID |
| HSV1-UL54-274 | - | ACCCCCCCCGCUAAUGACGC | 20 | 7037 |
| HSV1-UL54-723 | + | GCGUCAUUAGCGGGGGGGGU | 20 | 7485 |
| HSV1-UL54-510 | - | CCCCCCGCUAAUGACGC | 17 | 7273 |
| HSV1-UL54-725 | + | GCGUCAUUAGCGGGGGG | 17 | 7486 |

**Table 5A** provides exemplary targeting domains for knocking out the *UL19* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 5A**

| 1st Tier | | | | |
|---|---|---|---|---|
| gRNA Name | DNA Strand | Targeting Domain | Target Site Length | Seq ID NO: |
| HSV1-UL19-179 | + | GGACCCGGUCGGCACCA | 17 | 569 |
| HSV1-UL19-136 | + | GAUACCCGUCCCGUCCA | 17 | 540 |
| HSV1-UL19-168 | + | GCAAUACGACCCCAGCA | 17 | 448 |
| HSV1-UL19-3209 | + | GCGAGCGAGCUGUUGGA | 17 | 7808 |
| HSV1-UL19-134 | + | GUUGGAUGGCGCGCAAC | 17 | 422 |
| HSV1-UL19-165 | + | GAAAGCGCACGAGCGAC | 17 | 445 |
| HSV1-UL19-97 | - | GCGACCCUCCGGGAUAC | 17 | 390 |
| HSV1-UL19-121 | - | GAACGCCGCCUUCAGCC | 17 | 529 |
| HSV1-UL19-182 | + | GCGGCAUACCGGUAUCC | 17 | 571 |
| HSV1-UL19-3210 | - | GCUCGCUCGCAACGUCC | 17 | 7809 |
| HSV1-UL19-156 | + | GUUGCUCGAUGGGGUGC | 17 | 558 |
| HSV1-UL19-107 | - | GUCGCUCGUGCGCUUUC | 17 | 400 |
| HSV1-UL19-98 | - | GGGAUACCGGUAUGCCG | 17 | 391 |
| HSV1-UL19-117 | - | GGGGCGCGUACAGUUCG | 17 | 413 |
| HSV1-UL19-105 | - | GAGUUCGACGCCCUGCU | 17 | 398 |
| HSV1-UL19-85 | + | GAGGGACCCGGUCGGCACCA | 20 | 512 |
| HSV1-UL19-3211 | + | GUCGCGGUUGGGAGCGGCCA | 20 | 7810 |
| HSV1-UL19-42 | + | GCCGAUACCCGUCCCGUCCA | 20 | 483 |
| HSV1-UL19-74 | + | GUUGCAAUACGACCCCAGCA | 20 | 440 |
| HSV1-UL19-3212 | + | GUUGCGAGCGAGCUGUUGGA | 20 | 7811 |
| HSV1-UL19-40 | + | GCUGUUGGAUGGCGCGCAAC | 20 | 421 |
| HSV1-UL19-87 | + | GCACCAUGGCCGCGGCAUAC | 20 | 453 |
| HSV1-UL19-26 | - | GACCAAGAUUAUCGACCGCC | 20 | 412 |
| HSV1-UL19-48 | + | GGGCGAUGGCCUCGGUGGCC | 20 | 489 |
| HSV1-UL19-88 | + | GCCGCGGCAUACCGGUAUCC | 20 | 514 |
| HSV1-UL19-78 | + | GUUUGCAUCGGAGCGCACGC | 20 | 506 |
| HSV1-UL19-190 | - | GCAACGUCCAGGCCGUCCUC | 20 | 576 |
| HSV1-UL19-18 | - | GUGCGUGUGUACCAAGUUUC | 20 | 404 |
| HSV1-UL19-65 | + | GGGCCGUCGCGGGCAAUCAG | 20 | 430 |
| HSV1-UL19-8 | - | GUCCCUCCUUAGCACGAUCG | 20 | 465 |
| HSV1-UL19-32 | - | GGCCAUCGCCCUGCUCACGG | 20 | 476 |
| HSV1-UL19-89 | + | GCGGCAUACCGGUAUCCCGG | 20 | 515 |
| HSV1-UL19-61 | + | GUGGGUUGCUCGAUGGGGUG | 20 | 500 |
| HSV1-UL19-76 | + | GUCGUACAGGCUGUUUGCAU | 20 | 442 |
| HSV1-UL19-39 | - | GCCCUGGACGGGACGGGUAU | 20 | 482 |
| HSV1-UL19-70 | + | GUAGGCCAGCUCCGGAAACU | 20 | 436 |
| HSV1-UL19-11 | - | GUCGAGUUCGACGCCCUGCU | 20 | 393 |
| HSV1-UL19-55 | + | GGCCCGGCGGUCGAUAAUCU | 20 | 496 |
| HSV1-UL19-3213 | + | GGACGUUGCGAGCGAGCUGU | 20 | 7812 |

**Table** 5B provides exemplary targeting domains for knocking out the *UL19* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 5B**

| 2nd Tier | | | | |
|---|---|---|---|---|
| gRNA Name | DNA Strand | Targeting Domain | Target Site Length | Seq ID NO: |
| HSV1-UL19-166 | + | AAAGCGCACGAGCGACA | 17 | 446 |
| HSV1-UL19-3214 | - | AUCUCCACGCGACCCCA | 17 | 7813 |
| HSV1-UL19-99 | - | CCGGUAUGCCGCGGCCA | 17 | 392 |
| HSV1-UL19-115 | - | CUGGCCUACAUGAACGA | 17 | 527 |
| HSV1-UL19-118 | - | CCGCUGAUUGCCCGCGA | 17 | 414 |
| HSV1-UL19-176 | + | CUCGAUCGUGCUAAGGA | 17 | 452 |
| HSV1-UL19-184 | + | CAUACCGGUAUCCCGGA | 17 | 573 |
| HSV1-UL19-174 | + | CCACCUCGAUCGUGCUA | 17 | 565 |
| HSV1-UL19-181 | + | CCAUGGCCGCGGCAUAC | 17 | 454 |
| HSV1-UL19-169 | + | CGAACUCGACGUCGUAC | 17 | 449 |
| HSV1-UL19-1442 | + | ACGCCCCGAGGACGGCC | 17 | 1828 |
| HSV1-UL19-135 | + | CGAUACCCGUCCCGUCC | 17 | 424 |
| HSV1-UL19-172 | + | UGCAUCGGAGCGCACGC | 17 | 563 |
| HSV1-UL19-119 | - | CCAAGAUUAUCGACCGC | 17 | 415 |
| HSV1-UL19-160 | + | CGCGGGCAAUCAGCGGC | 17 | 560 |
| HSV1-UL19-104 | - | CGAGUUCGACGCCCUGC | 17 | 524 |
| HSV1-UL19-171 | + | UUGCAUCGGAGCGCACG | 17 | 562 |
| HSV1-UL19-125 | - | CCAUCGCCCUGCUCACG | 17 | 418 |
| HSV1-UL19-102 | - | CCUCCUUAGCACGAUCG | 17 | 522 |
| HSV1-UL19-175 | + | CCUCGAUCGUGCUAAGG | 17 | 566 |
| HSV1-UL19-103 | - | CCUUAGCACGAUCGAGG | 17 | 523 |
| HSV1-UL19-126 | - | CAUCGCCCUGCUCACGG | 17 | 533 |
| HSV1-UL19-106 | - | AGUUCGACGCCCUGCUG | 17 | 399 |
| HSV1-UL19-153 | + | UGUGUGGGUUGCUCGAU | 17 | 428 |
| HSV1-UL19-133 | - | CUGGACGGGACGGGUAU | 17 | 539 |
| HSV1-UL19-582 | - | AACGUCCAGGCCGUCCU | 17 | 968 |
| HSV1-UL19-149 | + | CCGGCGGUCGAUAAUCU | 17 | 553 |
| HSV1-UL19-3215 | + | CGUUGCGAGCGAGCUGU | 17 | 7814 |
| HSV1-UL19-187 | + | CCCGGAGGGUCGCGGUU | 17 | 460 |
| HSV1-UL19-72 | + | CAGAAAGCGCACGAGCGACA | 20 | 438 |
| HSV1-UL19-3216 | - | CCGAUCUCCACGCGACCCCA | 20 | 7815 |
| HSV1-UL19-5 | - | AUACCGGUAUGCCGCGGCCA | 20 | 389 |
| HSV1-UL19-29 | - | CGAGGCCAUCGCCCUGCUCA | 20 | 474 |
| HSV1-UL19-52 | + | CAGGCUGAAGGCGGCGUUCA | 20 | 493 |
| HSV1-UL19-24 | - | CAGCCGCUGAUUGCCCGCGA | 20 | 410 |
| HSV1-UL19-45 | + | CUCCCCCGUGAGCAGGGCGA | 20 | 486 |
| HSV1-UL19-82 | + | CACCUCGAUCGUGCUAAGGA | 20 | 451 |
| HSV1-UL19-90 | + | CGGCAUACCGGUAUCCCGGA | 20 | 516 |
| HSV1-UL19-80 | + | ACGCCACCUCGAUCGUGCUA | 20 | 508 |
| HSV1-UL19-71 | + | CCAGAAAGCGCACGAGCGAC | 20 | 437 |
| HSV1-UL19-3 | - | ACCGCGACCCUCCGGGAUAC | 20 | 387 |
| HSV1-UL19-75 | + | CGUCGAACUCGACGUCGUAC | 20 | 441 |
| HSV1-UL19-7 | - | CCGCGGCCAUGGUGCCGACC | 20 | 464 |
| HSV1-UL19-975 | + | CAAACGCCCCGAGGACGGCC | 20 | 1361 |
| HSV1-UL19-53 | + | UGAAGGCGGCGUUCAGGGCC | 20 | 494 |
| HSV1-UL19-3217 | - | ACAGCUCGCUCGCAACGUCC | 20 | 7816 |
| HSV1-UL19-41 | + | CGCCGAUACCCGUCCCGUCC | 20 | 423 |
| HSV1-UL19-73 | + | UGUUGCAAUACGACCCCAGC | 20 | 439 |
| HSV1-UL19-25 | - | UGACCAAGAUUAUCGACCGC | 20 | 411 |
| HSV1-UL19-66 | + | CGUCGCGGGCAAUCAGCGGC | 20 | 503 |
| HSV1-UL19-10 | - | CGUCGAGUUCGACGCCCUGC | 20 | 467 |
| HSV1-UL19-62 | + | UGGGUUGCUCGAUGGGGUGC | 20 | 501 |
| HSV1-UL19-1 | - | CGCUCCCAACCGCGACCCUC | 20 | 461 |
| HSV1-UL19-69 | + | UC GUUCAUGUAGGC CAGCUC | 20 | 435 |
| HSV1-UL19-15 | - | UCGUGCGCUUUCUGGAGCUC | 20 | 397 |
| HSV1-UL19-51 | + | CCAGGCUGAAGGCGGCGUUC | 20 | 492 |
| HSV1-UL19-13 | - | CCUGUCGCUCGUGCGCUUUC | 20 | 395 |
| HSV1-UL19-77 | + | UGUUUGCAUCGGAGCGCACG | 20 | 505 |
| HSV1-UL19-31 | - | AGGCCAUCGCCCUGCUCACG | 20 | 417 |
| HSV1-UL19-86 | + | CCCGGUCGGCACCAUGGCCG | 20 | 513 |
| HSV1-UL19-4 | - | UCCGGGAUACCGGUAUGCCG | 20 | 388 |
| HSV1-UL19-191 | - | CAACGUCCAGGCCGUCCUCG | 20 | 577 |
| HSV1-UL19-63 | + | CGAUGGGGUGCGGGCCGUCG | 20 | 502 |
| HSV1-UL19-91 | + | ACCGGUAUCCCGGAGGGUCG | 20 | 455 |
| HSV1-UL19-23 | - | CGAGGGGCGCGUACAGUUCG | 20 | 409 |
| HSV1-UL19-81 | + | CCACCUCGAUCGUGCUAAGG | 20 | 509 |
| HSV1-UL19-9 | - | CCUCCUUAGCACGAUCGAGG | 20 | 466 |
| HSV1-UL19-54 | + | AGGCGGCGUUCAGGGCCCGG | 20 | 495 |
| HSV1-UL19-17 | - | UCUGGAGCUCGGGUUGUCGG | 20 | 403 |
| HSV1-UL19-33 | - | CAUCGCCCUGCUCACGGGGG | 20 | 419 |
| HSV1-UL19-67 | + | CGCGGGCAAUCAGCGGCUGG | 20 | 433 |
| HSV1-UL19-3218 | + | CGCGGUUGGGAGCGGCCAUG | 20 | 7817 |
| HSV1-UL19-60 | + | UGUGUGUGGGUUGCUCGAUG | 20 | 499 |
| HSV1-UL19-12 | - | UCGAGUUCGACGCCCUGCUG | 20 | 394 |
| HSV1-UL19-3219 | + | UCGCGGUUGGGAGCGGCCAU | 20 | 7818 |
| HSV1-UL19-59 | + | UUGUGUGUGGGUUGCUCGAU | 20 | 426 |
| HSV1-UL19-189 | - | CGCAACGUCCAGGCCGUCCU | 20 | 575 |
| HSV1-UL19-14 | - | CUCGUGCGCUUUCUGGAGCU | 20 | 396 |
| HSV1-UL19-68 | + | UACGCGCCCCUCGUUCAUGU | 20 | 504 |
| HSV1-UL19-16 | - | CUUUCUGGAGCUCGGGUUGU | 20 | 468 |
| HSV1-UL19-93 | + | UAUCCCGGAGGGUCGCGGUU | 20 | 457 |

**Table** 5C provides exemplary targeting domains for knocking out the *UL19* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 5C**

| **3rd Tier** | | | | |
|---|---|---|---|---|
| **gRNA Name** | **DNA Strand** | **Targeting Domain** | **Target Site Length** | **Seq ID NO:** |
| HSV1-UL19-3220 | + | GCGGUUGGGAGCGGCCA | 17 | 7819 |
| HSV1-UL19-138 | + | GGCCUCCCCCGUGAGCA | 17 | 542 |
| HSV1-UL19-123 | - | GGCCAUCGCCCUGCUCA | 17 | 531 |
| HSV1-UL19-146 | + | GCUGAAGGCGGCGUUCA | 17 | 550 |
| HSV1-UL19-152 | + | GUGUGUGGGUUGCUCGA | 17 | 427 |
| HSV1-UL19-131 | - | GGAGGCCCUGGACGGGA | 17 | 537 |
| HSV1-UL19-173 | + | GGGAAAAAAAAUCAAAC | 17 | 564 |
| HSV1-UL19-124 | - | GCCAUCGCCCUGCUCAC | 17 | 532 |
| HSV1-UL19-100 | - | GCGGCCAUGGUGCCGAC | 17 | 520 |
| HSV1-UL19-132 | - | GAGGCCCUGGACGGGAC | 17 | 538 |
| HSV1-UL19-128 | - | GCUCACGGGGGAGGCCC | 17 | 534 |
| HSV1-UL19-137 | + | GGGCCUCCCCCGUGAGC | 17 | 541 |
| HSV1-UL19-158 | + | GGGGUGCGGGCCGUCGC | 17 | 431 |
| HSV1-UL19-145 | + | GGCUGAAGGCGGCGUUC | 17 | 549 |
| HSV1-UL19-114 | - | GCUGGCCUACAUGAACG | 17 | 526 |
| HSV1-UL19-180 | + | GGUCGGCACCAUGGCCG | 17 | 570 |
| HSV1-UL19-3221 | + | GCGGCCAUGGGGUCGCG | 17 | 7820 |
| HSV1-UL19-185 | + | GGUAUCCCGGAGGGUCG | 17 | 458 |
| HSV1-UL19-144 | + | GGUGGCCAGGCUGAAGG | 17 | 548 |
| HSV1-UL19-183 | + | GCAUACCGGUAUCCCGG | 17 | 572 |
| HSV1-UL19-111 | - | GGAGCUCGGGUUGUCGG | 17 | 406 |
| HSV1-UL19-161 | + | GGGCAAUCAGCGGCUGG | 17 | 434 |
| HSV1-UL19-3222 | + | GGUUGGGAGCGGCCAUG | 17 | 7821 |
| HSV1-UL19-154 | + | GUGUGGGUUGCUCGAUG | 17 | 556 |
| HSV1-UL19-155 | + | GGUUGCUCGAUGGGGUG | 17 | 557 |
| HSV1-UL19-150 | + | GGUCAUGUAAUUGUGUG | 17 | 554 |
| HSV1-UL19-170 | + | GUACAGGCUGUUUGCAU | 17 | 450 |
| HSV1-UL19-164 | + | GGCCAGCUCCGGAAACU | 17 | 444 |
| HSV1-UL19-140 | + | GAGCAGGGCGAUGGCCU | 17 | 544 |
| HSV1-UL19-108 | - | GUGCGCUUUCUGGAGCU | 17 | 401 |
| HSV1-UL19-162 | + | GCGCCCCUCGUUCAUGU | 17 | 561 |
| HSV1-UL19-151 | + | GUCAUGUAAUUGUGUGU | 17 | 555 |
| HSV1-UL19-21 | - | GAGCUGGCCUACAUGAACGA | 20 | 470 |
| HSV1-UL19-37 | - | GGGGGAGGCCCUGGACGGGA | 20 | 480 |
| HSV1-UL19-49 | + | GGCCUCGGUGGCCAGGCUGA | 20 | 490 |
| HSV1-UL19-30 | - | GAGGCCAUCGCCCUGCUCAC | 20 | 475 |
| HSV1-UL19-6 | - | GCCGCGGCCAUGGUGCCGAC | 20 | 463 |
| HSV1-UL19-38 | - | GGGGAGGCCCUGGACGGGAC | 20 | 481 |
| HSV1-UL19-83 | + | GAUCGUGCUAAGGAGGGACC | 20 | 510 |
| HSV1-UL19-2 | - | GCUCCCAACCGCGACCCUCC | 20 | 462 |
| HSV1-UL19-19 | - | GUGUACCAAGUUUCCGGAGC | 20 | 405 |
| HSV1-UL19-64 | + | GAUGGGGUGCGGGCCGUCGC | 20 | 429 |
| HSV1-UL19-20 | - | GGAGCUGGCCUACAUGAACG | 20 | 469 |
| HSV1-UL19-3223 | + | GGAGCGGCCAUGGGGUCGCG | 20 | 7822 |
| HSV1-UL19-47 | + | GAGCAGGGCGAUGGCCUCGG | 20 | 488 |
| HSV1-UL19-84 | + | GUGCUAAGGAGGGACCCGGU | 20 | 511 |
| HSV1-UL19-92 | + | GUAUCCCGGAGGGUCGCGGU | 20 | 456 |

**Table 5D** provides exemplary targeting domains for knocking out the *UL19* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 5D**

| **4th Tier** | | | | |
|---|---|---|---|---|
| **gRNA Name** | **DNA Strand** | **Targeting Domain** | **Target Site Length** | **Seq ID NO:** |
| HSV1-UL19-139 | + | CCCCGUGAGCAGGGCGA | 17 | 543 |
| HSV1-UL19-129 | - | ACGGGGGAGGCCCUGGA | 17 | 535 |
| HSV1-UL19-143 | + | CUCGGUGGCCAGGCUGA | 17 | 547 |
| HSV1-UL19-130 | - | CGGGGGAGGCCCUGGAC | 17 | 536 |
| HSV1-UL19-101 | - | CGGCCAUGGUGCCGACC | 17 | 521 |
| HSV1-UL19-177 | + | CGUGCUAAGGAGGGACC | 17 | 567 |
| HSV1-UL19-120 | - | CAAGAUUAUCGACCGCC | 17 | 416 |
| HSV1-UL19-147 | + | AGGCGGCGUUCAGGGCC | 17 | 551 |
| HSV1-UL19-142 | + | CGAUGGCCUCGGUGGCC | 17 | 546 |
| HSV1-UL19-96 | - | CCCAACCGCGACCCUCC | 17 | 519 |
| HSV1-UL19-167 | + | UGCAAUACGACCCCAGC | 17 | 447 |
| HSV1-UL19-113 | - | UACCAAGUUUCCGGAGC | 17 | 408 |
| HSV1-UL19-95 | - | UCCCAACCGCGACCCUC | 17 | 518 |
| HSV1-UL19-583 | - | ACGUCCAGGCCGUCCUC | 17 | 969 |
| HSV1-UL19-163 | + | UUCAUGUAGGCCAGCUC | 17 | 443 |
| HSV1-UL19-109 | - | UGCGCUUUCUGGAGCUC | 17 | 402 |
| HSV1-UL19-112 | - | CGUGUGUACCAAGUUUC | 17 | 407 |
| HSV1-UL19-159 | + | CCGUCGCGGGCAAUCAG | 17 | 432 |
| HSV1-UL19-116 | - | UGGCCUACAUGAACGAG | 17 | 528 |
| HSV1-UL19-188 | + | AGGGUCGCGGUUGGGAG | 17 | 574 |
| HSV1-UL19-122 | - | CUUCAGCCUGGCCACCG | 17 | 530 |
| HSV1-UL19-584 | - | CGUCCAGGCCGUCCUCG | 17 | 970 |
| HSV1-UL19-157 | + | UGGGGUGCGGGCCGUCG | 17 | 559 |
| HSV1-UL19-148 | + | CGGCGUUCAGGGCCCGG | 17 | 552 |
| HSV1-UL19-141 | + | CAGGGCGAUGGCCUCGG | 17 | 545 |
| HSV1-UL19-127 | - | CGCCCUGCUCACGGGGG | 17 | 420 |
| HSV1-UL19-3224 | + | CGGUUGGGAGCGGCCAU | 17 | 7823 |
| HSV1-UL19-3225 | + | UGGGGUCGCGUGGAGAU | 17 | 7824 |
| HSV1-UL19-178 | + | CUAAGGAGGGACCCGGU | 17 | 568 |
| HSV1-UL19-186 | + | UCCCGGAGGGUCGCGGU | 17 | 459 |
| HSV1-UL19-110 | - | UCUGGAGCUCGGGUUGU | 17 | 525 |
| HSV1-UL19-44 | + | CAGGGCCUCCCCCGUGAGCA | 20 | 485 |
| HSV1-UL19-58 | + | AUUGUGUGUGGGUUGCUCGA | 20 | 425 |
| HSV1-UL19-35 | - | CUCACGGGGGAGGCCCUGGA | 20 | 478 |
| HSV1-UL19-79 | + | CGCGGGAAAAAAAAUCAAAC | 20 | 507 |
| HSV1-UL19-36 | - | UCACGGGGGAGGCCCUGGAC | 20 | 479 |
| HSV1-UL19-34 | - | CCUGCUCACGGGGGAGGCCC | 20 | 477 |
| HSV1-UL19-27 | - | CCUGAACGCCGCCUUCAGCC | 20 | 472 |
| HSV1-UL19-43 | + | CCAGGGCCUCCCCCGUGAGC | 20 | 484 |
| HSV1-UL19-22 | - | AGCUGGCCUACAUGAACGAG | 20 | 471 |
| HSV1-UL19-94 | + | CGGAGGGUCGCGGUUGGGAG | 20 | 517 |
| HSV1-UL19-28 | - | CGCCUUCAGCCUGGCCACCG | 20 | 473 |
| HSV1-UL19-50 | + | CUCGGUGGCCAGGCUGAAGG | 20 | 491 |
| HSV1-UL19-56 | + | CUUGGUCAUGUAAUUGUGUG | 20 | 497 |
| HSV1-UL19-3226 | + | CCAUGGGGUCGCGUGGAGAU | 20 | 7825 |
| HSV1-UL19-46 | + | CGUGAGCAGGGCGAUGGCCU | 20 | 487 |
| HSV1-UL19-57 | + | UUGGUCAUGUAAUUGUGUGU | 20 | 498 |

**Table 5E** provides exemplary targeting domains for knocking out the *UL19* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a S. *pyogenes* Cas9 molecule that generates a double stranded break (Cas9 nuclease) or a single-stranded break (Cas9 nickase).

**Table 5E**

| **5th Tier** | | | | |
|---|---|---|---|---|
| **gRNA Name** | **DNA Strand** | **Targeting Domain** | **Target Site Length** | **Seq ID NO:** |
| HSV1-UL19-838 | - | GACCAUACCAUCCAAAA | 17 | 1224 |
| HSV1-UL19-588 | - | GCACGUGCUGCUGGAAA | 17 | 974 |
| HSV1-UL19-827 | - | CAUGGCCGAACGCACAA | 17 | 1213 |
| HSV1-UL19-818 | - | AACGCCAUGUUUCACAA | 17 | 1204 |
| HSV1-UL19-591 | - | CAACGAUACCUGGACAA | 17 | 977 |
| HSV1-UL19-967 | - | CUCCGCAGUGCCCGCAA | 17 | 1353 |
| HSV1-UL19-947 | - | GGGGCCUACCACCUCAA | 17 | 1333 |
| HSV1-UL19-973 | - | GACGCAUCCCCGCUCAA | 17 | 1359 |
| HSV1-UL19-1554 | + | CAACGUUCAUCAGCGAA | 17 | 1940 |
| HSV1-UL19-1617 | + | GUCGAACGCCCCGCGAA | 17 | 2003 |
| HSV1-UL19-655 | - | UCAAUCCGGUCAUGGAA | 17 | 1041 |
| HSV1-UL19-624 | - | GGCGAGAUGGUCCUGAA | 17 | 1010 |
| HSV1-UL19-720 | - | ACCUGGCGCGUGGUGAA | 17 | 1106 |
| HSV1-UL19-944 | - | GGGGACCUGCUCUAUAA | 17 | 1330 |
| HSV1-UL19-1712 | + | GCGCCCGUUGUGAAACA | 17 | 2098 |
| HSV1-UL19-826 | - | GGUGCUGGCCCACAACA | 17 | 1212 |
| HSV1-UL19-817 | - | CAAUCUGGUGGCCAACA | 17 | 1203 |
| HSV1-UL19-808 | - | CGCCACCCUGCAGAACA | 17 | 1194 |
| HSV1-UL19-1823 | + | CUGGGCGCACCCGAACA | 17 | 2209 |
| HSV1-UL19-1829 | + | GGCGAUAAACUCACACA | 17 | 2215 |
| HSV1-UL19-1886 | + | UCCUGAAACAGGCCACA | 17 | 2272 |
| HSV1-UL19-1552 | + | CAGCGAAGGGUGGCACA | 17 | 1938 |
| HSV1-UL19-890 | - | CAACCCCGUCACCGACA | 17 | 1276 |
| HSV1-UL19-1834 | + | GUUGACGUCGGUCGACA | 17 | 2220 |
| HSV1-UL19-929 | - | CGUUUACGCGGGGGACA | 17 | 1315 |
| HSV1-UL19-743 | - | CUGCAGGCCGCCAUACA | 17 | 1129 |
| HSV1-UL19-1665 | + | ACAUGCCGCCGCGUACA | 17 | 2051 |
| HSV1-UL19-1812 | + | CGCGUUCCGCAGGUACA | 17 | 2198 |
| HSV1-UL19-1635 | + | CACCAGGUCCCGGUACA | 17 | 2021 |
| HSV1-UL19-1516 | + | GUCCAUGGCGCCCACCA | 17 | 1902 |
| HSV1-UL19-594 | - | CGGCCGCCUGGCCACCA | 17 | 980 |
| HSV1-UL19-610 | - | GCUCGUGGACCUCACCA | 17 | 996 |
| HSV1-UL19-934 | - | GCCCUCAUGUACGACCA | 17 | 1320 |
| HSV1-UL19-1459 | + | CUUUAGCUCGGCGACCA | 17 | 1845 |
| HSV1-UL19-1759 | + | CAGAGCCGGGGGGACCA | 17 | 2145 |
| HSV1-UL19-915 | - | CGCGCAGGGAUGGACCA | 17 | 1301 |
| HSV1-UL19-1859 | + | AAUCGCUGCGACGCCCA | 17 | 2245 |
| HSV1-UL19-1522 | + | CGGAUUGAACAGGCCCA | 17 | 1908 |
| HSV1-UL19-688 | - | GGGCAGCGCCUGGCCCA | 17 | 1074 |
| HSV1-UL19-1449 | + | CGGCAACAACAAAGCCA | 17 | 1835 |
| HSV1-UL19-736 | - | GGUGGGACGCCACGCCA | 17 | 1122 |
| HSV1-UL19-1601 | + | CGCCGGACAUAGCGCCA | 17 | 1987 |
| HSV1-UL19-650 | - | CCCCCUGGUGGGCGCCA | 17 | 1036 |
| HSV1-UL19-1774 | + | CUGAUGAUGCAAGGCCA | 17 | 2160 |
| HSV1-UL19-1473 | + | CAGACGGGGCACGGCCA | 17 | 1859 |
| HSV1-UL19-938 | - | CCGGGCCUUCGCGGCCA | 17 | 1324 |
| HSV1-UL19-1624 | + | GACCACGAGCCGGGCCA | 17 | 2010 |
| HSV1-UL19-1609 | + | GAUGGUGGCGGGGGCCA | 17 | 1995 |
| HSV1-UL19-1638 | + | AUCAACCAGCUGGGCCA | 17 | 2024 |
| HSV1-UL19-889 | - | CACCGCCGUCGUGGCCA | 17 | 1275 |
| HSV1-UL19-1507 | + | GUAGAUGCGCUUCUCCA | 17 | 1893 |
| HSV1-UL19-1518 | + | GACGAACGUCAGGUCCA | 17 | 1904 |
| HSV1-UL19-1657 | + | GUCGCGAUGGCGGUCCA | 17 | 2043 |
| HSV1-UL19-958 | - | GGGUUCGGCGGUGUCCA | 17 | 1344 |
| HSV1-UL19-1867 | + | GUAAAGAACUUAAAGCA | 17 | 2253 |
| HSV1-UL19-1715 | + | CACCUGCAGCGUGAGCA | 17 | 2101 |
| HSV1-UL19-913 | - | AGGUGCCGCGGCGCGCA | 17 | 1299 |
| HSV1-UL19-1754 | + | CCGCGACAGGUCGCGCA | 17 | 2140 |
| HSV1-UL19-1495 | + | GUCGUCCAGACUUCGCA | 17 | 1881 |
| HSV1-UL19-586 | - | GGCGUUUGAGCGCGGCA | 17 | 972 |
| HSV1-UL19-1474 | + | AUGCGUCAGACGGGGCA | 17 | 1860 |
| HSV1-UL19-631 | - | GGCGCUCGUGAUGGGCA | 17 | 1017 |
| HSV1-UL19-601 | - | GAGCUUUUUCCUGGGCA | 17 | 987 |
| HSV1-UL19-1775 | + | CUUGAGCUGAUGAUGCA | 17 | 2161 |
| HSV1-UL19-1687 | + | GACCACCAUGUUCUGCA | 17 | 2073 |
| HSV1-UL19-756 | - | CCUUCCCGAGGAGUGCA | 17 | 1142 |
| HSV1-UL19-1706 | + | CAGAUUGGCCGGGUGCA | 17 | 2092 |
| HSV1-UL19-1678 | + | GUAAAUCUUGUGGUGCA | 17 | 2064 |
| HSV1-UL19-1838 | + | CGUCCCCGGGGGUUGCA | 17 | 2224 |
| HSV1-UL19-1655 | + | GGCCGCGCGCCGCAUCA | 17 | 2041 |
| HSV1-UL19-972 | - | UGACGCAUCCCCGCUCA | 17 | 1358 |
| HSV1-UL19-856 | - | GCUGACCUACGCGCUCA | 17 | 1242 |
| HSV1-UL19-1700 | + | GUGCGCGGGGUCCGUCA | 17 | 2086 |
| HSV1-UL19-1642 | + | CAGCUCCGGGCCGGUCA | 17 | 2028 |
| HSV1-UL19-654 | - | CCUGUUCAAUCCGGUCA | 17 | 1040 |
| HSV1-UL19-628 | - | CGGGGCCAACCUGGUCA | 17 | 1014 |
| HSV1-UL19-861 | - | GCAUCAUCAGCUCAAGA | 17 | 1247 |
| HSV1-UL19-1477 | + | UGUCGGCAUGCGUCAGA | 17 | 1863 |
| HSV1-UL19-1682 | + | GCAGCGGCCCCGCGAGA | 17 | 2068 |
| HSV1-UL19-623 | - | GGUGACGUACGGCGAGA | 17 | 1009 |
| HSV1-UL19-1809 | + | CCGCGUUGUCCAGGAGA | 17 | 2195 |
| HSV1-UL19-954 | - | GCGCCUGAUCGUGGAGA | 17 | 1340 |
| HSV1-UL19-788 | - | GACUUCAACCGCAACGA | 17 | 1174 |
| HSV1-UL19-1653 | + | GUCACAGUCCCACACGA | 17 | 2039 |
| HSV1-UL19-1793 | + | GGUUGCCGUGGCCACGA | 17 | 2179 |
| HSV1-UL19-1769 | + | GCGGACGUGCUGCACGA | 17 | 2155 |
| HSV1-UL19-1780 | + | GCGGUCCUGUCGGACGA | 17 | 2166 |
| HSV1-UL19-1506 | + | GACCAGCUUCUCGCCGA | 17 | 1892 |
| HSV1-UL19-692 | - | CCCACGGGCGGGUCCGA | 17 | 1078 |
| HSV1-UL19-1555 | + | UCAACGUUCAUCAGCGA | 17 | 1941 |
| HSV1-UL19-1855 | + | GUUGGCCGUGGCCGCGA | 17 | 2241 |
| HSV1-UL19-1660 | + | UAACCCGGCAGUCGCGA | 17 | 2046 |
| HSV1-UL19-1615 | + | CCCGCGAACGGCGGCGA | 17 | 2001 |
| HSV1-UL19-1466 | + | CACGAGCCAGGCCUCGA | 17 | 1852 |
| HSV1-UL19-616 | - | CGCGGGCGGCCGGUCGA | 17 | 1002 |
| HSV1-UL19-832 | - | AACAUGCGCAUAUUCGA | 17 | 1218 |
| HSV1-UL19-1560 | + | CGGAUUGGCGGCUUCGA | 17 | 1946 |
| HSV1-UL19-931 | - | UACGCGGGGGACAAGGA | 17 | 1317 |
| HSV1-UL19-1487 | + | GUCCUCCACUUUCAGGA | 17 | 1873 |
| HSV1-UL19-1443 | + | CUCAAACGCCCCGAGGA | 17 | 1829 |
| HSV1-UL19-1896 | + | GCGGGCACUGCGGAGGA | 17 | 2282 |
| HSV1-UL19-1471 | + | CGGGGCACGGCCACGGA | 17 | 1857 |
| HSV1-UL19-695 | - | GUCCGAUGGGUCGCGGA | 17 | 1081 |
| HSV1-UL19-1795 | + | GGUGACGGGGUUGCGGA | 17 | 2181 |
| HSV1-UL19-914 | - | GCCGCGGCGCGCAGGGA | 17 | 1300 |
| HSV1-UL19-1777 | + | UGAACCCAAACCCGGGA | 17 | 2163 |
| HSV1-UL19-1852 | + | GCCGCGAAGGCCCGGGA | 17 | 2238 |
| HSV1-UL19-1542 | + | CAGCUGGCGGGGCGGGA | 17 | 1928 |
| HSV1-UL19-1632 | + | CCAUGCACUCCUCGGGA | 17 | 2018 |
| HSV1-UL19-1820 | + | GCACCCGAACACGGGGA | 17 | 2206 |
| HSV1-UL19-1738 | + | CAGCGCGUGGACGGGGA | 17 | 2124 |
| HSV1-UL19-665 | - | GCUGUUUUUUUGGGGGA | 17 | 1051 |
| HSV1-UL19-1801 | + | GGUAAAAGUUUUGGGGA | 17 | 2187 |
| HSV1-UL19-822 | - | GGGCGCGUGGUAGUGGA | 17 | 1208 |
| HSV1-UL19-1742 | + | GGCGAACAGCGCGUGGA | 17 | 2128 |
| HSV1-UL19-3209 | + | GCGAGCGAGCUGUUGGA | 17 | 7808 |
| HSV1-UL19-1734 | + | AUAGUCGCCAUUUUGGA | 17 | 2120 |
| HSV1-UL19-1847 | + | GCCGUGGUCGUACAUGA | 17 | 2233 |
| HSV1-UL19-836 | - | CGCCGGAAUCCUGCUGA | 17 | 1222 |
| HSV1-UL19-800 | - | GAUUUACUACUACGUGA | 17 | 1186 |
| HSV1-UL19-813 | - | CCCCAGCGACCCCGUGA | 17 | 1199 |
| HSV1-UL19-629 | - | GGUCACGGCGCUCGUGA | 17 | 1015 |
| HSV1-UL19-1799 | + | GUUGCCCAUGUCGGUGA | 17 | 2185 |
| HSV1-UL19-622 | - | GACGUGCCGGUGACGUA | 17 | 1008 |
| HSV1-UL19-1563 | + | GCCACGUACGCCCCGUA | 17 | 1949 |
| HSV1-UL19-676 | - | GAAGCCGCCAAUCCGUA | 17 | 1062 |
| HSV1-UL19-941 | - | UCGCAGCGAUUUUCGUA | 17 | 1327 |
| HSV1-UL19-1512 | + | GCGCCCACCAGGGGGUA | 17 | 1898 |
| HSV1-UL19-1733 | + | CGCCAUUUUGGAUGGUA | 17 | 2119 |
| HSV1-UL19-1623 | + | GUGCUCGCUGCCGUGUA | 17 | 2009 |
| HSV1-UL19-1556 | + | CGACGGGGUCGCGGUUA | 17 | 1942 |
| HSV1-UL19-876 | - | GUGGCCCGGCACGAAAC | 17 | 1262 |
| HSV1-UL19-1887 | + | GGUAGGCCUCCUGAAAC | 17 | 2273 |
| HSV1-UL19-819 | - | ACGCCAUGUUUCACAAC | 17 | 1205 |
| HSV1-UL19-968 | - | UCCGCAGUGCCCGCAAC | 17 | 1354 |
| HSV1-UL19-134 | + | GUUGGAUGGCGCGCAAC | 17 | 422 |
| HSV1-UL19-948 | - | GGGCCUACCACCUCAAC | 17 | 1334 |
| HSV1-UL19-638 | - | AGCAGCUCGACCUGAAC | 17 | 1024 |
| HSV1-UL19-625 | - | GCGAGAUGGUCCUGAAC | 17 | 1011 |
| HSV1-UL19-1524 | + | CCAUGACCGGAUUGAAC | 17 | 1910 |
| HSV1-UL19-945 | - | GGGACCUGCUCUAUAAC | 17 | 1331 |
| HSV1-UL19-1822 | + | UGGGCGCACCCGAACAC | 17 | 2208 |
| HSV1-UL19-1885 | + | CCUGAAACAGGCCACAC | 17 | 2271 |
| HSV1-UL19-1833 | + | UUGACGUCGGUCGACAC | 17 | 2219 |
| HSV1-UL19-1664 | + | CAUGCCGCCGCGUACAC | 17 | 2050 |
| HSV1-UL19-1858 | + | AUCGCUGCGACGCCCAC | 17 | 2244 |
| HSV1-UL19-689 | - | GGCAGCGCCUGGCCCAC | 17 | 1075 |
| HSV1-UL19-1773 | + | UGAUGAUGCAAGGCCAC | 17 | 2159 |
| HSV1-UL19-1865 | + | AAGCAGGGGCUGAGCAC | 17 | 2251 |
| HSV1-UL19-793 | - | CGGAUGACCGGCCGCAC | 17 | 1179 |
| HSV1-UL19-1628 | + | AGUAGCUGGUGAUGCAC | 17 | 2014 |
| HSV1-UL19-1491 | + | AUCUCGCCGUACGUCAC | 17 | 1877 |
| HSV1-UL19-1699 | + | UGCGCGGGGUCCGUCAC | 17 | 2085 |
| HSV1-UL19-862 | - | CAUCAUCAGCUCAAGAC | 17 | 1248 |
| HSV1-UL19-1476 | + | GUCGGCAUGCGUCAGAC | 17 | 1862 |
| HSV1-UL19-1551 | + | UCGCGUGUUCCAGAGAC | 17 | 1937 |
| HSV1-UL19-955 | - | CGC CUGAUC GUGGAGAC | 17 | 1341 |
| HSV1-UL19-1768 | + | CGGACGUGCUGCACGAC | 17 | 2154 |
| HSV1-UL19-867 | - | GUUCACCGUCGUCCGAC | 17 | 1253 |
| HSV1-UL19-1756 | + | GGGGGACCUGCCGCGAC | 17 | 2142 |
| HSV1-UL19-1479 | + | ACGAGCACCCCGUCGAC | 17 | 1865 |
| HSV1-UL19-617 | - | GCGGGCGGCCGGUCGAC | 17 | 1003 |
| HSV1-UL19-833 | - | ACAUGCGCAUAUUCGAC | 17 | 1219 |
| HSV1-UL19-1559 | + | GGAUUGGCGGCUUCGAC | 17 | 1945 |
| HSV1-UL19-799 | - | CGCACCGGGGGGCGGAC | 17 | 1185 |
| HSV1-UL19-974 | - | AUCCCCGCUCAAGGGAC | 17 | 1360 |
| HSV1-UL19-1851 | + | CCGCGAAGGCCCGGGAC | 17 | 2237 |
| HSV1-UL19-1819 | + | CACCCGAACACGGGGAC | 17 | 2205 |
| HSV1-UL19-823 | - | GGCGCGUGGUAGUGGAC | 17 | 1209 |
| HSV1-UL19-1741 | + | GCGAACAGCGCGUGGAC | 17 | 2127 |
| HSV1-UL19-1525 | + | GCAAACCGUUCCAUGAC | 17 | 1911 |
| HSV1-UL19-792 | - | CGGACGCCGCGGAUGAC | 17 | 1178 |
| HSV1-UL19-764 | - | GAUGACUUUACCCUGAC | 17 | 1150 |
| HSV1-UL19-1798 | + | UUGCCCAUGUCGGUGAC | 17 | 2184 |
| HSV1-UL19-747 | - | AGUGCAUCACCAGCUAC | 17 | 1133 |
| HSV1-UL19-677 | - | AAGCCGCCAAUCCGUAC | 17 | 1063 |
| HSV1-UL19-942 | - | CGCAGCGAUUUUCGUAC | 17 | 1328 |
| HSV1-UL19-757 | - | AGUGCAUGGCCGUGUAC | 17 | 1143 |
| HSV1-UL19-1779 | + | GACGGUGAACCCAAACC | 17 | 2165 |
| HSV1-UL19-697 | - | GCCCGACAACGCCAACC | 17 | 1083 |
| HSV1-UL19-627 | - | CCUGAACGGGGCCAACC | 17 | 1013 |
| HSV1-UL19-1661 | + | GGCCCCCCGCGCUAACC | 17 | 2047 |
| HSV1-UL19-1517 | + | GGUCCAUGGCGCCCACC | 17 | 1903 |
| HSV1-UL19-1637 | + | CCUCGACGUGCGCCACC | 17 | 2023 |
| HSV1-UL19-718 | - | GGGAGAUCCAGGCCACC | 17 | 1104 |
| HSV1-UL19-660 | - | CCCCGCCCCCGGCCACC | 17 | 1046 |
| HSV1-UL19-1785 | + | UUUCGUGCCGGGCCACC | 17 | 2171 |
| HSV1-UL19-593 | - | ACGGCCGCCUGGCCACC | 17 | 979 |
| HSV1-UL19-1550 | + | GAGACAGGCGCAGCACC | 17 | 1936 |
| HSV1-UL19-794 | - | GGAUGACCGGCCGCACC | 17 | 1180 |
| HSV1-UL19-1825 | + | CUGCGCGCCGCGGCACC | 17 | 2211 |
| HSV1-UL19-814 | - | CGCGCACCCCCUGCACC | 17 | 1200 |
| HSV1-UL19-771 | - | UCACCUAAUGCGAGACC | 17 | 1157 |
| HSV1-UL19-1460 | + | GCUUUAGCUCGGCGACC | 17 | 1846 |
| HSV1-UL19-759 | - | GGCCGUGUACCGGGACC | 17 | 1145 |
| HSV1-UL19-1760 | + | CCAGAGCCGGGGGGACC | 17 | 2146 |
| HSV1-UL19-884 | - | GCGCGCGAACGUGGACC | 17 | 1270 |
| HSV1-UL19-1494 | + | UCACGAGCGCCGUGACC | 17 | 1880 |
| HSV1-UL19-590 | - | GCCGAUGCAACGAUACC | 17 | 976 |
| HSV1-UL19-740 | - | CGACGACCGCAACUACC | 17 | 1126 |
| HSV1-UL19-758 | - | GUGCAUGGCCGUGUACC | 17 | 1144 |
| HSV1-UL19-892 | - | UCCCCAAAACUUUUACC | 17 | 1278 |
| HSV1-UL19-1778 | + | ACGGUGAACCCAAACCC | 17 | 2164 |
| HSV1-UL19-789 | - | GCUGCUGCACAACACCC | 17 | 1175 |
| HSV1-UL19-1456 | + | GGUCGCCCGGGCCACCC | 17 | 1842 |
| HSV1-UL19-1883 | + | ACGAGUUCGCGGCACCC | 17 | 2269 |
| HSV1-UL19-598 | - | GGUGGCCCGGGCGACCC | 17 | 984 |
| HSV1-UL19-696 | - | GGAAGGCCAGAUGACCC | 17 | 1082 |
| HSV1-UL19-918 | - | GCCGGCCCUGCAACCCC | 17 | 1304 |
| HSV1-UL19-1882 | + | CGAGUUCGCGGCACCCC | 17 | 2268 |
| HSV1-UL19-919 | - | CCGGCCCUGCAACCCCC | 17 | 1305 |
| HSV1-UL19-647 | - | CAACGUUCCCUACCCCC | 17 | 1033 |
| HSV1-UL19-1589 | + | GGGGGAACGUCCCCCCC | 17 | 1975 |
| HSV1-UL19-960 | - | CAGUUUAAGCGCCCCCC | 17 | 1346 |
| HSV1-UL19-1677 | + | CGGUCCAGUCCGCCCCC | 17 | 2063 |
| HSV1-UL19-959 | - | ACAGUUUAAGCGCCCCC | 17 | 1345 |
| HSV1-UL19-1572 | + | GGCCAGGCGCUGCCCCC | 17 | 1958 |
| HSV1-UL19-1595 | + | UGGCCUGGAUCUCCCCC | 17 | 1981 |
| HSV1-UL19-846 | - | GUCGCGGCAGGUCCCCC | 17 | 1232 |
| HSV1-UL19-847 | - | GGUCCCCCUGGUCCCCC | 17 | 1233 |
| HSV1-UL19-843 | - | CGCGCCCAAUUUCCCCC | 17 | 1229 |
| HSV1-UL19-1845 | + | CCCCGCGUAAACGCCCC | 17 | 2231 |
| HSV1-UL19-659 | - | GACCUAGUCCCCGCCCC | 17 | 1045 |
| HSV1-UL19-589 | - | GGAAAAGGCGCCGCCCC | 17 | 975 |
| HSV1-UL19-811 | - | GUCCCGGAGAUCGCCCC | 17 | 1197 |
| HSV1-UL19-1605 | + | CCCCAGCUCCAGGCCCC | 17 | 1991 |
| HSV1-UL19-1815 | + | CGGGCUGGGCCGGCCCC | 17 | 2201 |
| HSV1-UL19-651 | - | GACGUUCGUCCUGCCCC | 17 | 1037 |
| HSV1-UL19-721 | - | GAACGGCAACUUGCCCC | 17 | 1107 |
| HSV1-UL19-1596 | + | GUGGCCUGGAUCUCCCC | 17 | 1982 |
| HSV1-UL19-1842 | + | CCCGGCGGCGCGUCCCC | 17 | 2228 |
| HSV1-UL19-711 | - | GGGGGGGGACGUUCCCC | 17 | 1097 |
| HSV1-UL19-1791 | + | CGGUGUAGCCCACGCCC | 17 | 2177 |
| HSV1-UL19-727 | - | GGCGUUCCGGGACGCCC | 17 | 1113 |
| HSV1-UL19-744 | - | GCACGUCUUCUGCGCCC | 17 | 1130 |
| HSV1-UL19-910 | - | CGUGUUCGGGUGCGCCC | 17 | 1296 |
| HSV1-UL19-1457 | + | GGCGACCAGGGUCGCCC | 17 | 1843 |
| HSV1-UL19-1853 | + | CGUGGCCGCGAAGGCCC | 17 | 2239 |
| HSV1-UL19-1523 | + | CCGGAUUGAACAGGCCC | 17 | 1909 |
| HSV1-UL19-1606 | + | CCCCCAGCUCCAGGCCC | 17 | 1992 |
| HSV1-UL19-762 | - | GGCGCACGUCGAGGCCC | 17 | 1148 |
| HSV1-UL19-646 | - | GGUCUUUCUGGAGGCCC | 17 | 1032 |
| HSV1-UL19-714 | - | GUUCCCCCGGCCGGCCC | 17 | 1100 |
| HSV1-UL19-1805 | + | GGGGAGCCCCGCGGCCC | 17 | 2191 |
| HSV1-UL19-776 | - | GAUGCGGCGCGCGGCCC | 17 | 1162 |
| HSV1-UL19-681 | - | GGCGUACGUGGCGGCCC | 17 | 1067 |
| HSV1-UL19-1744 | + | CGCCACGUGGUCGGCCC | 17 | 2130 |
| HSV1-UL19-1783 | + | CCACCUGGAGCUGGCCC | 17 | 2169 |
| HSV1-UL19-597 | - | GGCCACCAGGGUGGCCC | 17 | 983 |
| HSV1-UL19-1462 | + | GGUGGCCCGCCUUGCCC | 17 | 1848 |
| HSV1-UL19-864 | - | AGACGGGCCUCCAUCCC | 17 | 1250 |
| HSV1-UL19-1771 | + | GGUCAGCGCGUUCUCCC | 17 | 2157 |
| HSV1-UL19-936 | - | CCAGAGCGACCCGUCCC | 17 | 1322 |
| HSV1-UL19-1843 | + | CCCCGGCGGCGCGUCCC | 17 | 2229 |
| HSV1-UL19-1527 | + | GGCGGGGACUAGGUCCC | 17 | 1913 |
| HSV1-UL19-1450 | + | UCGGCAACAACAAAGCC | 17 | 1836 |
| HSV1-UL19-1584 | + | GGUGCAGCUCAAGAGCC | 17 | 1970 |
| HSV1-UL19-1764 | + | AGUUGGCCCCCAGAGCC | 17 | 2150 |
| HSV1-UL19-1626 | + | GCACUGGACCACGAGCC | 17 | 2012 |
| HSV1-UL19-1467 | + | GGUGAGGUCCACGAGCC | 17 | 1853 |
| HSV1-UL19-683 | - | AGCUGUUUUUGAACGCC | 17 | 1069 |
| HSV1-UL19-657 | - | GGUUUGCCGCGCACGCC | 17 | 1043 |
| HSV1-UL19-726 | - | CGGCGUUCCGGGACGCC | 17 | 1112 |
| HSV1-UL19-904 | - | CGUGGCGGGAAACCGCC | 17 | 1290 |
| HSV1-UL19-1619 | + | GCAGGUAAAACACCGCC | 17 | 2005 |
| HSV1-UL19-605 | - | CAAGGCGGGCCACCGCC | 17 | 991 |
| HSV1-UL19-666 | - | UUGGGGGAAGGACCGCC | 17 | 1052 |
| HSV1-UL19-922 | - | CCCGGGGACGCGCCGCC | 17 | 1308 |
| HSV1-UL19-592 | - | CCUGGACAACGGCCGCC | 17 | 978 |
| HSV1-UL19-1649 | + | GGGGUGGCAGCAGCGCC | 17 | 2035 |
| HSV1-UL19-687 | - | CGCCUGGGGGCAGCGCC | 17 | 1073 |
| HSV1-UL19-1602 | + | CCGCCGGACAUAGCGCC | 17 | 1988 |
| HSV1-UL19-1599 | + | GCCGUUCACCACGCGCC | 17 | 1985 |
| HSV1-UL19-1693 | + | CUGGGGCACUCCUCGCC | 17 | 2079 |
| HSV1-UL19-1458 | + | CGGCGACCAGGGUCGCC | 17 | 1844 |
| HSV1-UL19-840 | - | UCCACGCGCUGUUCGCC | 17 | 1226 |
| HSV1-UL19-1854 | + | CCGUGGCCGCGAAGGCC | 17 | 2240 |
| HSV1-UL19-608 | - | GGGAGGCCGUCGAGGCC | 17 | 994 |
| HSV1-UL19-916 | - | AGGGAUGGACCACGGCC | 17 | 1302 |
| HSV1-UL19-1442 | + | ACGCCCCGAGGACGGCC | 17 | 1828 |
| HSV1-UL19-765 | - | CUUUACCCUGACCGGCC | 17 | 1151 |
| HSV1-UL19-713 | - | CGUUCCCCCGGCCGGCC | 17 | 1099 |
| HSV1-UL19-1592 | + | UCUCCCCCGGGCCGGCC | 17 | 1978 |
| HSV1-UL19-1565 | + | CUGCGGCGGGGCCGGCC | 17 | 1951 |
| HSV1-UL19-854 | - | UCCGCGAGAGCGCGGCC | 17 | 1240 |
| HSV1-UL19-1752 | + | ACAGGUCGCGCAGGGCC | 17 | 2138 |
| HSV1-UL19-1625 | + | GGACCACGAGCCGGGCC | 17 | 2011 |
| HSV1-UL19-1670 | + | CGUCCGCGGCUCGGGCC | 17 | 2056 |
| HSV1-UL19-729 | - | CCGGGACGCCCGGGGCC | 17 | 1115 |
| HSV1-UL19-1639 | + | CAUCAACCAGCUGGGCC | 17 | 2025 |
| HSV1-UL19-1573 | + | GGACCCGCCCGUGGGCC | 17 | 1959 |
| HSV1-UL19-745 | - | UCUUCUGCGCCCUGGCC | 17 | 1131 |
| HSV1-UL19-1597 | + | CCACGCGCCAGGUGGCC | 17 | 1983 |
| HSV1-UL19-875 | - | GCCAGCUCCAGGUGGCC | 17 | 1261 |
| HSV1-UL19-596 | - | UGGCCACCAGGGUGGCC | 17 | 982 |
| HSV1-UL19-1534 | + | CGGGGAUCCGGGUGGCC | 17 | 1920 |
| HSV1-UL19-1454 | + | GGGCCACCCUGGUGGCC | 17 | 1840 |
| HSV1-UL19-1708 | + | UGGCCACCAGAUUGGCC | 17 | 2094 |
| HSV1-UL19-778 | - | CCGCCAUCGCGACUGCC | 17 | 1164 |
| HSV1-UL19-705 | - | GCCGACGUGGAGCUGCC | 17 | 1091 |
| HSV1-UL19-952 | - | CGCCAAACAUCGCUGCC | 17 | 1338 |
| HSV1-UL19-1786 | + | CCCGCCAGUUUCGUGCC | 17 | 2172 |
| HSV1-UL19-863 | - | AAGACGGGCCUCCAUCC | 17 | 1249 |
| HSV1-UL19-717 | - | CGGCCCGGGGGAGAUCC | 17 | 1103 |
| HSV1-UL19-1537 | + | GGAAGGCGCGGGGAUCC | 17 | 1923 |
| HSV1-UL19-1465 | + | AGGCCUCGACGGCCUCC | 17 | 1851 |
| HSV1-UL19-1607 | + | GUCCCACCCCCAGCUCC | 17 | 1993 |
| HSV1-UL19-1645 | + | CCUGCCCGCCCAGCUCC | 17 | 2031 |
| HSV1-UL19-873 | - | CUUCCUGGGCCAGCUCC | 17 | 1259 |
| HSV1-UL19-1872 | + | CCACGAUCAGGCGCUCC | 17 | 2258 |
| HSV1-UL19-1579 | + | GCUGCAUAAACUGCUCC | 17 | 1965 |
| HSV1-UL19-1689 | + | CGCCGGGGGCGAUCUCC | 17 | 2075 |
| HSV1-UL19-897 | - | CGGGGCUCCCCCUCUCC | 17 | 1283 |
| HSV1-UL19-1508 | + | CGUAGAUGCGCUUCUCC | 17 | 1894 |
| HSV1-UL19-3210 | - | GCUCGCUCGCAACGUCC | 17 | 7809 |
| HSV1-UL19-935 | - | GCCAGAGCGACCCGUCC | 17 | 1321 |
| HSV1-UL19-1828 | + | ACUCACACACGGCGUCC | 17 | 2214 |
| HSV1-UL19-1604 | + | CCAGGCCCCGGGCGUCC | 17 | 1990 |
| HSV1-UL19-1636 | + | CGUGCGCCACCAGGUCC | 17 | 2022 |
| HSV1-UL19-1658 | + | AGUCGCGAUGGCGGUCC | 17 | 2044 |
| HSV1-UL19-810 | - | GCAGAACAUGGUGGUCC | 17 | 1196 |
| HSV1-UL19-1452 | + | CCAGGCGGCCGUUGUCC | 17 | 1838 |
| HSV1-UL19-1811 | + | CGGCUGCCGCGUUGUCC | 17 | 2197 |
| HSV1-UL19-871 | - | GUCGGAGGCGUACUUCC | 17 | 1257 |
| HSV1-UL19-725 | - | AUGUCCGGCGGCGUUCC | 17 | 1111 |
| HSV1-UL19-599 | - | CGAGACGAGCUUUUUCC | 17 | 985 |
| HSV1-UL19-1868 | + | CGUAAAGAACUUAAAGC | 17 | 2254 |
| HSV1-UL19-643 | - | GUCCAUCGGCGAGAAGC | 17 | 1029 |
| HSV1-UL19-1548 | + | UCCCCCAAAAAAACAGC | 17 | 1934 |
| HSV1-UL19-1641 | + | UAAAGUCAUCAACCAGC | 17 | 2027 |
| HSV1-UL19-763 | - | CGAGGCCCUGGCCCAGC | 17 | 1149 |
| HSV1-UL19-1498 | + | CCUCCUGCAUCUCCAGC | 17 | 1884 |
| HSV1-UL19-1668 | + | GGGUGUUGUGCAGCAGC | 17 | 2054 |
| HSV1-UL19-1621 | + | GUAUGGCGGCCUGCAGC | 17 | 2007 |
| HSV1-UL19-1727 | + | GCUGGGGGGCCAUCAGC | 17 | 2113 |
| HSV1-UL19-1765 | + | UAGUUGGCCCCCAGAGC | 17 | 2151 |
| HSV1-UL19-1860 | + | AGGCCCCGUUAUAGAGC | 17 | 2246 |
| HSV1-UL19-1627 | + | UGCACUGGACCACGAGC | 17 | 2013 |
| HSV1-UL19-766 | - | CCUGACCGGCCCGGAGC | 17 | 1152 |
| HSV1-UL19-1784 | + | GCCGGGCCACCUGGAGC | 17 | 2170 |
| HSV1-UL19-730 | - | CGCCCGGGGCCUGGAGC | 17 | 1116 |
| HSV1-UL19-1889 | + | CGCUGGCGCAGGUGAGC | 17 | 2275 |
| HSV1-UL19-1907 | + | GAGCCAGUCCCUUGAGC | 17 | 2293 |
| HSV1-UL19-1629 | + | CGUGUUGUUCCAGUAGC | 17 | 2015 |
| HSV1-UL19-1586 | + | ACAAAGAAGUCGAACGC | 17 | 1972 |
| HSV1-UL19-656 | - | CGGUUUGCCGCGCACGC | 17 | 1042 |
| HSV1-UL19-835 | - | GACGGGGCGUUGCACGC | 17 | 1221 |
| HSV1-UL19-639 | - | CCUGAACCGGCAGACGC | 17 | 1025 |
| HSV1-UL19-831 | - | UCGGCGGCGCCCGACGC | 17 | 1217 |
| HSV1-UL19-926 | - | GCCGGGGGCGUUUACGC | 17 | 1312 |
| HSV1-UL19-1620 | + | AGCAGGUAAAACACCGC | 17 | 2006 |
| HSV1-UL19-604 | - | GCAAGGCGGGCCACCGC | 17 | 990 |
| HSV1-UL19-805 | - | GGCCGCUGCUGCACCGC | 17 | 1191 |
| HSV1-UL19-1603 | + | GCGUCCCGGAACGCCGC | 17 | 1989 |
| HSV1-UL19-921 | - | CCCCGGGGACGCGCCGC | 17 | 1307 |
| HSV1-UL19-895 | - | ACUUUUACCUGGGCCGC | 17 | 1281 |
| HSV1-UL19-917 | - | ACGUCAACUACUUCCGC | 17 | 1303 |
| HSV1-UL19-1813 | + | CCACGACCGCGUUCCGC | 17 | 2199 |
| HSV1-UL19-1650 | + | AGGGGUGGCAGCAGCGC | 17 | 2036 |
| HSV1-UL19-780 | - | GACUGCCGGGUUAGCGC | 17 | 1166 |
| HSV1-UL19-613 | - | CGCAUGCCGACACGCGC | 17 | 999 |
| HSV1-UL19-971 | - | CGGGGAAGCCCACGCGC | 17 | 1357 |
| HSV1-UL19-912 | - | CAGGUGCCGCGGCGCGC | 17 | 1298 |
| HSV1-UL19-1755 | + | GCCGCGACAGGUCGCGC | 17 | 2141 |
| HSV1-UL19-1540 | + | GCGGGGCGGGAAGGCGC | 17 | 1926 |
| HSV1-UL19-1891 | + | GGCGGGGUCGCUGGCGC | 17 | 2277 |
| HSV1-UL19-1702 | + | GGGUGCAGGGGGUGCGC | 17 | 2088 |
| HSV1-UL19-750 | - | CGUGAACGACUACUCGC | 17 | 1136 |
| HSV1-UL19-950 | - | CCUCAACGGGGCCUCGC | 17 | 1336 |
| HSV1-UL19-1694 | + | GCUGGGGCACUCCUCGC | 17 | 2080 |
| HSV1-UL19-802 | - | GGUGCCCGCCUUCUCGC | 17 | 1188 |
| HSV1-UL19-1878 | + | CUUAAACUGUACGUCGC | 17 | 2264 |
| HSV1-UL19-1697 | + | GUCCGUCACGGGGUCGC | 17 | 2083 |
| HSV1-UL19-1892 | + | GAGGAGGGCGGGGUCGC | 17 | 2278 |
| HSV1-UL19-738 | - | CCAUCGCCGCCGUUCGC | 17 | 1124 |
| HSV1-UL19-839 | - | GUCCACGCGCUGUUCGC | 17 | 1225 |
| HSV1-UL19-603 | - | UUUUUCCUGGGCAAGGC | 17 | 989 |
| HSV1-UL19-1680 | + | CGGCCCCGCGAGAAGGC | 17 | 2066 |
| HSV1-UL19-712 | - | GGGGACGUUCCCCCGGC | 17 | 1098 |
| HSV1-UL19-682 | - | UACGUGGCGGCCCCGGC | 17 | 1068 |
| HSV1-UL19-1593 | + | AUCUCCCCCGGGCCGGC | 17 | 1979 |
| HSV1-UL19-1566 | + | UCUGCGGCGGGGCCGGC | 17 | 1952 |
| HSV1-UL19-853 | - | GUCCGCGAGAGCGCGGC | 17 | 1239 |
| HSV1-UL19-1789 | + | CCACGUUCGCGCGCGGC | 17 | 2175 |
| HSV1-UL19-845 | - | GCGCGACCUGUCGCGGC | 17 | 1231 |
| HSV1-UL19-615 | - | CGACACGCGCGGGCGGC | 17 | 1001 |
| HSV1-UL19-1569 | + | UGCUGCAUGUCUGCGGC | 17 | 1955 |
| HSV1-UL19-1675 | + | CCGCCCCCCGGUGCGGC | 17 | 2061 |
| HSV1-UL19-3227 | + | CUACCCCAAGGAAGGGC | 17 | 7826 |
| HSV1-UL19-1753 | + | GACAGGUCGCGCAGGGC | 17 | 2139 |
| HSV1-UL19-1837 | + | CCCGGGGGUUGCAGGGC | 17 | 2223 |
| HSV1-UL19-1894 | + | GCACUGCGGAGGAGGGC | 17 | 2280 |
| HSV1-UL19-691 | - | GCGCCUGGCCCACGGGC | 17 | 1077 |
| HSV1-UL19-1818 | + | CGAACACGGGGACGGGC | 17 | 2204 |
| HSV1-UL19-1594 | + | CUGGAUCUCCCCCGGGC | 17 | 1980 |
| HSV1-UL19-1644 | + | CCCGCCCAGCUCCGGGC | 17 | 2030 |
| HSV1-UL19-770 | - | CCCGGAGCUGGGCGGGC | 17 | 1156 |
| HSV1-UL19-1520 | + | UGAACAGGCCCAGGGGC | 17 | 1906 |
| HSV1-UL19-1567 | + | CAUGUCUGCGGCGGGGC | 17 | 1953 |
| HSV1-UL19-1543 | + | AAAACAGCUGGCGGGGC | 17 | 1929 |
| HSV1-UL19-1530 | + | UCCGGGUGGCCGGGGGC | 17 | 1916 |
| HSV1-UL19-907 | - | GGGAAACCGCCUGGGGC | 17 | 1293 |
| HSV1-UL19-769 | - | CCGGCCCGGAGCUGGGC | 17 | 1155 |
| HSV1-UL19-1816 | + | ACGGGGACGGGCUGGGC | 17 | 2202 |
| HSV1-UL19-1713 | + | UGCAGCGUGAGCAUGGC | 17 | 2099 |
| HSV1-UL19-858 | - | ACCUACGCGCUCAUGGC | 17 | 1244 |
| HSV1-UL19-878 | - | CCCGGCACGAAACUGGC | 17 | 1264 |
| HSV1-UL19-1546 | + | CCAAAAAAACAGCUGGC | 17 | 1932 |
| HSV1-UL19-903 | - | CGGAACGCGGUCGUGGC | 17 | 1289 |
| HSV1-UL19-1535 | + | GCGGGGAUCCGGGUGGC | 17 | 1921 |
| HSV1-UL19-1612 | + | ACGGCGGCGAUGGUGGC | 17 | 1998 |
| HSV1-UL19-1709 | + | UUGGCCACCAGAUUGGC | 17 | 2095 |
| HSV1-UL19-1732 | + | UGGUAUGGUCCAGAUGC | 17 | 2118 |
| HSV1-UL19-636 | - | CCACCUGCUGGAGAUGC | 17 | 1022 |
| HSV1-UL19-777 | - | ACCGCCAUCGCGACUGC | 17 | 1163 |
| HSV1-UL19-1486 | + | CUUUCAGGAAGGACUGC | 17 | 1872 |
| HSV1-UL19-635 | - | CGUGGGCCGCCACCUGC | 17 | 1021 |
| HSV1-UL19-704 | - | GGCCGACGUGGAGCUGC | 17 | 1090 |
| HSV1-UL19-824 | - | CGCCAUGCUCACGCUGC | 17 | 1210 |
| HSV1-UL19-742 | - | GGUGUUUUACCUGCUGC | 17 | 1128 |
| HSV1-UL19-587 | - | GAUGCUGCACGUGCUGC | 17 | 973 |
| HSV1-UL19-1500 | + | GCUCGUCCAGCGUCUGC | 17 | 1886 |
| HSV1-UL19-1688 | + | GGACCACCAUGUUCUGC | 17 | 2074 |
| HSV1-UL19-621 | - | CACCGAAGCCGACGUGC | 17 | 1007 |
| HSV1-UL19-1787 | + | CCCCGCCAGUUUCGUGC | 17 | 2173 |
| HSV1-UL19-825 | - | GCUCACGCUGCAGGUGC | 17 | 1211 |
| HSV1-UL19-1707 | + | CCAGAUUGGCCGGGUGC | 17 | 2093 |
| HSV1-UL19-1900 | + | GUGGGCUUCCCCGUUGC | 17 | 2286 |
| HSV1-UL19-1839 | + | GCGUCCCCGGGGGUUGC | 17 | 2225 |
| HSV1-UL19-815 | - | CCUGCACCCGGCCAAUC | 17 | 1201 |
| HSV1-UL19-653 | - | CCUGGGCCUGUUCAAUC | 17 | 1039 |
| HSV1-UL19-837 | - | GAUGGCCCCCCAGCAUC | 17 | 1223 |
| HSV1-UL19-1446 | + | GCAGCACGUGCAGCAUC | 17 | 1832 |
| HSV1-UL19-1656 | + | GGGCCGCGCGCCGCAUC | 17 | 2042 |
| HSV1-UL19-1577 | + | ACUGCUCCGGGGUCAUC | 17 | 1963 |
| HSV1-UL19-1873 | + | AACCCGUCUCCACGAUC | 17 | 2259 |
| HSV1-UL19-669 | - | CUCUGGAACACGCGAUC | 17 | 1055 |
| HSV1-UL19-641 | - | GCGCGUGCGCGCGGAUC | 17 | 1027 |
| HSV1-UL19-1538 | + | GGGAAGGCGCGGGGAUC | 17 | 1924 |
| HSV1-UL19-1483 | + | GGAGGCGCUGUUUGAUC | 17 | 1869 |
| HSV1-UL19-752 | - | ACGUCGUGACCUACCUC | 17 | 1138 |
| HSV1-UL19-1633 | + | ACGGCCAUGCACUCCUC | 17 | 2019 |
| HSV1-UL19-583 | - | ACGUCCAGGCCGUCCUC | 17 | 969 |
| HSV1-UL19-1646 | + | GCCUGCCCGCCCAGCUC | 17 | 2032 |
| HSV1-UL19-1850 | + | CCCGGGACGGGUCGCUC | 17 | 2236 |
| HSV1-UL19-848 | - | CCUGGUCCCCCCGGCUC | 17 | 1234 |
| HSV1-UL19-1671 | + | CGCGGCGUCCGCGGCUC | 17 | 2057 |
| HSV1-UL19-1580 | + | GGCUGCAUAAACUGCUC | 17 | 1966 |
| HSV1-UL19-1690 | + | UCGCCGGGGGCGAUCUC | 17 | 2076 |
| HSV1-UL19-667 | - | GGUGCUGCGCCUGUCUC | 17 | 1053 |
| HSV1-UL19-632 | - | CAAGGCCGUGCGAAGUC | 17 | 1018 |
| HSV1-UL19-1519 | + | GGGGCAGGACGAACGUC | 17 | 1905 |
| HSV1-UL19-1719 | + | GUGUUGGCGCCCGCGUC | 17 | 2105 |
| HSV1-UL19-1505 | + | CGCGCACGCGCGUCGUC | 17 | 1891 |
| HSV1-UL19-1643 | + | CCAGCUCCGGGCCGGUC | 17 | 2029 |
| HSV1-UL19-673 | - | ACGUUGACGCGGCGGUC | 17 | 1059 |
| HSV1-UL19-722 | - | GCCCCUGGCGCUAUGUC | 17 | 1108 |
| HSV1-UL19-1581 | + | GC CAGGUUGGC GUUGUC | 17 | 1967 |
| HSV1-UL19-1726 | + | GGCCAUCAGCAGGAUUC | 17 | 2112 |
| HSV1-UL19-1492 | + | CCAGGUUGGCCCCGUUC | 17 | 1878 |
| HSV1-UL19-724 | - | UAUGUCCGGCGGCGUUC | 17 | 1110 |
| HSV1-UL19-1499 | + | CCAGCGUCUGCCGGUUC | 17 | 1885 |
| HSV1-UL19-909 | - | AGCCCGUCCCCGUGUUC | 17 | 1295 |
| HSV1-UL19-1488 | + | CGGUGUCCUCCACUUUC | 17 | 1874 |
| HSV1-UL19-644 | - | CGAGAAGCUGGUCUUUC | 17 | 1030 |
| HSV1-UL19-965 | - | CCCGUGUGGCCUGUUUC | 17 | 1351 |
| HSV1-UL19-1782 | + | CGUUCUCAGUCACAAAG | 17 | 2168 |
| HSV1-UL19-619 | - | GCAGUCCUUCCUGAAAG | 17 | 1005 |
| HSV1-UL19-1905 | + | CGAGAUACUGCGCGAAG | 17 | 2291 |
| HSV1-UL19-1515 | + | UCCAUGGCGCCCACCAG | 17 | 1901 |
| HSV1-UL19-1758 | + | AGAGCCGGGGGGACCAG | 17 | 2144 |
| HSV1-UL19-1521 | + | GGAUUGAACAGGCCCAG | 17 | 1907 |
| HSV1-UL19-1448 | + | GGCAACAACAAAGCCAG | 17 | 1834 |
| HSV1-UL19-1600 | + | GCCGGACAUAGCGCCAG | 17 | 1986 |
| HSV1-UL19-1866 | + | UAAAGAACUUAAAGCAG | 17 | 2252 |
| HSV1-UL19-1683 | + | CCCCCGCGGUGCAGCAG | 17 | 2069 |
| HSV1-UL19-1705 | + | AGAUUGGCCGGGUGCAG | 17 | 2091 |
| HSV1-UL19-1808 | + | CGCGUUGUCCAGGAGAG | 17 | 2194 |
| HSV1-UL19-1652 | + | UCACAGUCCCACACGAG | 17 | 2038 |
| HSV1-UL19-932 | - | ACGCGGGGGACAAGGAG | 17 | 1318 |
| HSV1-UL19-1810 | + | GCCGCGUUGUCCAGGAG | 17 | 2196 |
| HSV1-UL19-1737 | + | AGCGCGUGGACGGGGAG | 17 | 2123 |
| HSV1-UL19-1890 | + | UCGCUGGCGCAGGUGAG | 17 | 2276 |
| HSV1-UL19-1908 | + | AGAGCCAGUCCCUUGAG | 17 | 2294 |
| HSV1-UL19-821 | - | CAACGGGCGCGUGGUAG | 17 | 1207 |
| HSV1-UL19-898 | - | CCCUCUCCUGGACAACG | 17 | 1284 |
| HSV1-UL19-969 | - | CCGCAGUGCCCGCAACG | 17 | 1355 |
| HSV1-UL19-949 | - | GGCCUACCACCUCAACG | 17 | 1335 |
| HSV1-UL19-883 | - | CCAGCCGCGCGCGAACG | 17 | 1269 |
| HSV1-UL19-1587 | + | CACAAAGAAGUCGAACG | 17 | 1973 |
| HSV1-UL19-900 | - | CGUGUACCUGCGGAACG | 17 | 1286 |
| HSV1-UL19-626 | - | CGAGAUGGUCCUGAACG | 17 | 1012 |
| HSV1-UL19-946 | - | GGACCUGCUCUAUAACG | 17 | 1332 |
| HSV1-UL19-786 | - | CGCGGCGGCAUGUAACG | 17 | 1172 |
| HSV1-UL19-748 | - | GCUACUGGAACAACACG | 17 | 1134 |
| HSV1-UL19-1821 | + | GGGCGCACCCGAACACG | 17 | 2207 |
| HSV1-UL19-1654 | + | CGUCACAGUCCCACACG | 17 | 2040 |
| HSV1-UL19-1832 | + | UGACGUCGGUCGACACG | 17 | 2218 |
| HSV1-UL19-1663 | + | AUGCCGCCGCGUACACG | 17 | 2049 |
| HSV1-UL19-842 | - | CGCCGGGGCCGACCACG | 17 | 1228 |
| HSV1-UL19-1746 | + | UGGGCGCGUUCGCCACG | 17 | 2132 |
| HSV1-UL19-1698 | + | GCGCGGGGUCCGUCACG | 17 | 2084 |
| HSV1-UL19-1475 | + | UCGGCAUGCGUCAGACG | 17 | 1861 |
| HSV1-UL19-633 | - | GCGAAGUCUGGACGACG | 17 | 1019 |
| HSV1-UL19-830 | - | CUCGGCGGCGCCCGACG | 17 | 1216 |
| HSV1-UL19-703 | - | UGUGGGGGUGGCCGACG | 17 | 1089 |
| HSV1-UL19-618 | - | CGGGCGGCCGGUCGACG | 17 | 1004 |
| HSV1-UL19-834 | - | CAUGCGCAUAUUCGACG | 17 | 1220 |
| HSV1-UL19-1558 | + | GAUUGGCGGCUUCGACG | 17 | 1944 |
| HSV1-UL19-1740 | + | CGAACAGCGCGUGGACG | 17 | 2126 |
| HSV1-UL19-1722 | + | CAUGUUGGUGGUGGACG | 17 | 2108 |
| HSV1-UL19-1797 | + | UGCCCAUGUCGGUGACG | 17 | 2183 |
| HSV1-UL19-670 | - | GCUGAUGAACGUUGACG | 17 | 1056 |
| HSV1-UL19-1685 | + | GCAGGGUGGCGUAUACG | 17 | 2071 |
| HSV1-UL19-678 | - | AGCCGCCAAUCCGUACG | 17 | 1064 |
| HSV1-UL19-679 | - | UCCGUACGGGGCGUACG | 17 | 1065 |
| HSV1-UL19-943 | - | GCAGCGAUUUUCGUACG | 17 | 1329 |
| HSV1-UL19-784 | - | CCACGACCCCGUGUACG | 17 | 1170 |
| HSV1-UL19-925 | - | CGCCGGGGGCGUUUACG | 17 | 1311 |
| HSV1-UL19-795 | - | GAUGACCGGCCGCACCG | 17 | 1181 |
| HSV1-UL19-804 | - | GGGCCGCUGCUGCACCG | 17 | 1190 |
| HSV1-UL19-787 | - | AUGUAACGUGGCGACCG | 17 | 1173 |
| HSV1-UL19-939 | - | CGGCCACGGCCAACCCG | 17 | 1325 |
| HSV1-UL19-1881 | + | GAGUUCGCGGCACCCCG | 17 | 2267 |
| HSV1-UL19-920 | - | CGGCCCUGCAACCCCCG | 17 | 1306 |
| HSV1-UL19-1684 | + | GUCGAAGCGAACCCCCG | 17 | 2070 |
| HSV1-UL19-961 | - | AGUUUAAGCGCCCCCCG | 17 | 1347 |
| HSV1-UL19-1631 | + | CGGGAAGGUCUCCCCCG | 17 | 2017 |
| HSV1-UL19-1806 | + | GGAGAGGGGGAGCCCCG | 17 | 2192 |
| HSV1-UL19-1444 | + | CGCGCUCAAACGCCCCG | 17 | 1830 |
| HSV1-UL19-1841 | + | CCGGCGGCGCGUCCCCG | 17 | 2227 |
| HSV1-UL19-728 | - | GCGUUCCGGGACGCCCG | 17 | 1114 |
| HSV1-UL19-1575 | + | CCCAUCGGACCCGCCCG | 17 | 1961 |
| HSV1-UL19-715 | - | UUCCCCCGGCCGGCCCG | 17 | 1101 |
| HSV1-UL19-860 | - | CUUCAAGAUCAGUCCCG | 17 | 1246 |
| HSV1-UL19-755 | - | CGGGGGAGACCUUCCCG | 17 | 1141 |
| HSV1-UL19-1763 | + | GUUGGCCCCCAGAGCCG | 17 | 2149 |
| HSV1-UL19-790 | - | CACCCAGGCCCGAGCCG | 17 | 1176 |
| HSV1-UL19-658 | - | GUUUGCCGCGCACGCCG | 17 | 1044 |
| HSV1-UL19-791 | - | CCGAGCCGCGGACGCCG | 17 | 1177 |
| HSV1-UL19-923 | - | CCGGGGACGCGCCGCCG | 17 | 1309 |
| HSV1-UL19-1826 | + | UCCAUCCCUGCGCGCCG | 17 | 2212 |
| HSV1-UL19-1692 | + | UGGGGCACUCCUCGCCG | 17 | 2078 |
| HSV1-UL19-841 | - | CCACGCGCUGUUCGCCG | 17 | 1227 |
| HSV1-UL19-1591 | + | CUCCCCCGGGCCGGCCG | 17 | 1977 |
| HSV1-UL19-1564 | + | UGCGGCGGGGCCGGCCG | 17 | 1950 |
| HSV1-UL19-855 | - | CCGCGAGAGCGCGGCCG | 17 | 1241 |
| HSV1-UL19-1751 | + | CAGGUCGCGCAGGGCCG | 17 | 2137 |
| HSV1-UL19-894 | - | AACUUUUACCUGGGCCG | 17 | 1280 |
| HSV1-UL19-1827 | + | ACACGGCGUCCUGGCCG | 17 | 2213 |
| HSV1-UL19-1849 | + | ACGGGUCGCUCUGGCCG | 17 | 2235 |
| HSV1-UL19-1469 | + | GGAGGGCUGCGUGGCCG | 17 | 1855 |
| HSV1-UL19-1874 | + | GCUGGCGGCGGUGGCCG | 17 | 2260 |
| HSV1-UL19-1533 | + | GGGGAUCCGGGUGGCCG | 17 | 1919 |
| HSV1-UL19-1856 | + | CGCCCACGGGUUGGCCG | 17 | 2242 |
| HSV1-UL19-706 | - | CCGACGUGGAGCUGCCG | 17 | 1092 |
| HSV1-UL19-911 | - | GGUGCGCCCAGGUGCCG | 17 | 1297 |
| HSV1-UL19-1794 | + | GUUGCGGACGGUUGCCG | 17 | 2180 |
| HSV1-UL19-1674 | + | GUGCGGCCGGUCAUCCG | 17 | 2060 |
| HSV1-UL19-611 | - | GGCCACGCAGCCCUCCG | 17 | 997 |
| HSV1-UL19-1578 | + | CUGCAUAAACUGCUCCG | 17 | 1964 |
| HSV1-UL19-1666 | + | GUUGCGGUUGAAGUCCG | 17 | 2052 |
| HSV1-UL19-1673 | + | GUCAUCCGCGGCGUCCG | 17 | 2059 |
| HSV1-UL19-852 | - | GCACGUCCGCGAGAGCG | 17 | 1238 |
| HSV1-UL19-1906 | + | AGCCAGUCCCUUGAGCG | 17 | 2292 |
| HSV1-UL19-585 | - | CUCGGGGCGUUUGAGCG | 17 | 971 |
| HSV1-UL19-779 | - | CGACUGCCGGGUUAGCG | 17 | 1165 |
| HSV1-UL19-1585 | + | CAAAGAAGUCGAACGCG | 17 | 1971 |
| HSV1-UL19-612 | - | ACGCAUGCCGACACGCG | 17 | 998 |
| HSV1-UL19-970 | - | ACGGGGAAGCCCACGCG | 17 | 1356 |
| HSV1-UL19-927 | - | CCGGGGGCGUUUACGCG | 17 | 1313 |
| HSV1-UL19-806 | - | GCCGCUGCUGCACCGCG | 17 | 1192 |
| HSV1-UL19-896 | - | CUUUUACCUGGGCCGCG | 17 | 1282 |
| HSV1-UL19-1743 | + | CCCCGGCGAACAGCGCG | 17 | 2129 |
| HSV1-UL19-781 | - | ACUGCCGGGUUAGCGCG | 17 | 1167 |
| HSV1-UL19-1903 | + | AGUGGGUCUCCCGCGCG | 17 | 2289 |
| HSV1-UL19-1790 | + | AGGUCCACGUUCGCGCG | 17 | 2176 |
| HSV1-UL19-1539 | + | CGGGGCGGGAAGGCGCG | 17 | 1925 |
| HSV1-UL19-775 | - | CGCCCUGAUGCGGCGCG | 17 | 1161 |
| HSV1-UL19-820 | - | GUUUCACAACGGGCGCG | 17 | 1206 |
| HSV1-UL19-719 | - | CCAGGCCACCUGGCGCG | 17 | 1105 |
| HSV1-UL19-640 | - | GACGACGCGCGUGCGCG | 17 | 1026 |
| HSV1-UL19-1701 | + | GGUGCAGGGGGUGCGCG | 17 | 2087 |
| HSV1-UL19-803 | - | GUGCCCGCCUUCUCGCG | 17 | 1189 |
| HSV1-UL19-739 | - | CAUCGCCGCCGUUCGCG | 17 | 1125 |
| HSV1-UL19-1541 | + | GGCGGGGCGGGAAGGCG | 17 | 1927 |
| HSV1-UL19-1864 | + | GGGGCUGAGCACCGGCG | 17 | 2250 |
| HSV1-UL19-812 | - | GGAGAUCGCCCCCGGCG | 17 | 1198 |
| HSV1-UL19-1568 | + | GCUGCAUGUCUGCGGCG | 17 | 1954 |
| HSV1-UL19-3228 | + | UACCCCAAGGAAGGGCG | 17 | 7827 |
| HSV1-UL19-1893 | + | CACUGCGGAGGAGGGCG | 17 | 2279 |
| HSV1-UL19-1529 | + | CCGGGUGGCCGGGGGCG | 17 | 1915 |
| HSV1-UL19-886 | - | GAACGUGGACCUGGGCG | 17 | 1272 |
| HSV1-UL19-1608 | + | UGGCGGGGGCCAUGGCG | 17 | 1994 |
| HSV1-UL19-859 | - | CCUACGCGCUCAUGGCG | 17 | 1245 |
| HSV1-UL19-879 | - | CCGGCACGAAACUGGCG | 17 | 1265 |
| HSV1-UL19-1545 | + | CAAAAAAACAGCUGGCG | 17 | 1931 |
| HSV1-UL19-1611 | + | CGGCGGCGAUGGUGGCG | 17 | 1997 |
| HSV1-UL19-1470 | + | GGCCACGGAGGGCUGCG | 17 | 1856 |
| HSV1-UL19-1526 | + | UAGGUCCCCGGCGUGCG | 17 | 1912 |
| HSV1-UL19-749 | - | GAACAACACGCGGUGCG | 17 | 1135 |
| HSV1-UL19-1703 | + | CGGGUGCAGGGGGUGCG | 17 | 2089 |
| HSV1-UL19-953 | - | CCUGGAGCGCCUGAUCG | 17 | 1339 |
| HSV1-UL19-1482 | + | GAGGCGCUGUUUGAUCG | 17 | 1868 |
| HSV1-UL19-962 | - | GGGGUGCCGCGAACUCG | 17 | 1348 |
| HSV1-UL19-753 | - | CGUCGUGACCUACCUCG | 17 | 1139 |
| HSV1-UL19-584 | - | CGUCCAGGCCGUCCUCG | 17 | 970 |
| HSV1-UL19-746 | - | CGCCCUGGCCCGGCUCG | 17 | 1132 |
| HSV1-UL19-609 | - | CGUCGAGGCCUGGCUCG | 17 | 995 |
| HSV1-UL19-1770 | + | CCCCGGCCGCGCUCUCG | 17 | 2156 |
| HSV1-UL19-801 | - | UGGUGCCCGCCUUCUCG | 17 | 1187 |
| HSV1-UL19-761 | - | CCUGGUGGCGCACGUCG | 17 | 1147 |
| HSV1-UL19-888 | - | GGGCUACACCGCCGUCG | 17 | 1274 |
| HSV1-UL19-607 | - | CCGCCGGGAGGCCGUCG | 17 | 993 |
| HSV1-UL19-901 | - | CCUGCGGAACGCGGUCG | 17 | 1287 |
| HSV1-UL19-674 | - | CGUUGACGCGGCGGUCG | 17 | 1060 |
| HSV1-UL19-1662 | + | CCGCGUACACGGGGUCG | 17 | 2048 |
| HSV1-UL19-1557 | + | CGGCUUCGACGGGGUCG | 17 | 1943 |
| HSV1-UL19-694 | - | GCGGGUCCGAUGGGUCG | 17 | 1080 |
| HSV1-UL19-844 | - | CCCUGCGCGACCUGUCG | 17 | 1230 |
| HSV1-UL19-937 | - | CCCGUCCCGGGCCUUCG | 17 | 1323 |
| HSV1-UL19-1884 | + | GGUCCUCCACGAGUUCG | 17 | 2270 |
| HSV1-UL19-737 | - | ACCAUCGCCGCCGUUCG | 17 | 1123 |
| HSV1-UL19-930 | - | UUACGCGGGGGACAAGG | 17 | 1316 |
| HSV1-UL19-602 | - | CUUUUUCCUGGGCAAGG | 17 | 988 |
| HSV1-UL19-1681 | + | GCGGCCCCGCGAGAAGG | 17 | 2067 |
| HSV1-UL19-1514 | + | CCAUGGCGCCCACCAGG | 17 | 1900 |
| HSV1-UL19-1757 | + | GAGCCGGGGGGACCAGG | 17 | 2143 |
| HSV1-UL19-1814 | + | GCUGGGCCGGCCCCAGG | 17 | 2200 |
| HSV1-UL19-1598 | + | GUUCACCACGCGCCAGG | 17 | 1984 |
| HSV1-UL19-1453 | + | CCACCCUGGUGGCCAGG | 17 | 1839 |
| HSV1-UL19-874 | - | CCUGGGCCAGCUCCAGG | 17 | 1260 |
| HSV1-UL19-1497 | + | CCUGCAUCUCCAGCAGG | 17 | 1883 |
| HSV1-UL19-637 | - | CCUGCUGGAGAUGCAGG | 17 | 1023 |
| HSV1-UL19-1485 | + | UCAGGAAGGACUGCAGG | 17 | 1871 |
| HSV1-UL19-1704 | + | GAUUGGCCGGGUGCAGG | 17 | 2090 |
| HSV1-UL19-966 | - | GUGUGGCCUGUUUCAGG | 17 | 1352 |
| HSV1-UL19-1807 | + | GCGUUGUCCAGGAGAGG | 17 | 2193 |
| HSV1-UL19-933 | - | CGCGGGGGACAAGGAGG | 17 | 1319 |
| HSV1-UL19-1897 | + | UGCGGGCACUGCGGAGG | 17 | 2283 |
| HSV1-UL19-1736 | + | GCGCGUGGACGGGGAGG | 17 | 2122 |
| HSV1-UL19-1862 | + | GCGAGGCCCCGUUGAGG | 17 | 2248 |
| HSV1-UL19-1616 | + | GAACGCCCCGCGAACGG | 17 | 2002 |
| HSV1-UL19-1831 | + | GACGUCGGUCGACACGG | 17 | 2217 |
| HSV1-UL19-1472 | + | ACGGGGCACGGCCACGG | 17 | 1858 |
| HSV1-UL19-1792 | + | UGCCGUGGCCACGACGG | 17 | 2178 |
| HSV1-UL19-796 | - | AUGACCGGCCGCACCGG | 17 | 1182 |
| HSV1-UL19-741 | - | CGACCGCAACUACCCGG | 17 | 1127 |
| HSV1-UL19-1880 | + | AGUUCGCGGCACCCCGG | 17 | 2266 |
| HSV1-UL19-1844 | + | CGCGUAAACGCCCCCGG | 17 | 2230 |
| HSV1-UL19-1840 | + | CGGCGGCGCGUCCCCGG | 17 | 2226 |
| HSV1-UL19-716 | - | UCCCCCGGCCGGCCCGG | 17 | 1102 |
| HSV1-UL19-1464 | + | CCUCGACGGCCUCCCGG | 17 | 1850 |
| HSV1-UL19-1762 | + | UUGGCCCCCAGAGCCGG | 17 | 2148 |
| HSV1-UL19-924 | - | CGGGGACGCGCCGCCGG | 17 | 1310 |
| HSV1-UL19-1691 | + | GGGGCACUCCUCGCCGG | 17 | 2077 |
| HSV1-UL19-1590 | + | UCCCCCGGGCCGGCCGG | 17 | 1976 |
| HSV1-UL19-1750 | + | AGGUCGCGCAGGGCCGG | 17 | 2136 |
| HSV1-UL19-1836 | + | GGGGGUUGCAGGGCCGG | 17 | 2222 |
| HSV1-UL19-1532 | + | GGGAUCCGGGUGGCCGG | 17 | 1918 |
| HSV1-UL19-707 | - | CGACGUGGAGCUGCCGG | 17 | 1093 |
| HSV1-UL19-723 | - | CCUGGCGCUAUGUCCGG | 17 | 1109 |
| HSV1-UL19-671 | - | GAUGAACGUUGACGCGG | 17 | 1057 |
| HSV1-UL19-785 | - | CGACCCCGUGUACGCGG | 17 | 1171 |
| HSV1-UL19-928 | - | CGGGGGCGUUUACGCGG | 17 | 1314 |
| HSV1-UL19-807 | - | CCGCUGCUGCACCGCGG | 17 | 1193 |
| HSV1-UL19-782 | - | CUGCCGGGUUAGCGCGG | 17 | 1168 |
| HSV1-UL19-1463 | + | CGACGGCCUCCCGGCGG | 17 | 1849 |
| HSV1-UL19-1875 | + | UACGUCGCUGGCGGCGG | 17 | 2261 |
| HSV1-UL19-1480 | + | CUGUUUGAUCGGGGCGG | 17 | 1866 |
| HSV1-UL19-880 | - | CGGCACGAAACUGGCGG | 17 | 1266 |
| HSV1-UL19-1876 | + | CUGUACGUCGCUGGCGG | 17 | 2262 |
| HSV1-UL19-1610 | + | GGCGGCGAUGGUGGCGG | 17 | 1996 |
| HSV1-UL19-1898 | + | CGUUGCGGGCACUGCGG | 17 | 2284 |
| HSV1-UL19-1570 | + | CUGCUGCAUGUCUGCGG | 17 | 1956 |
| HSV1-UL19-754 | - | GUCGUGACCUACCUCGG | 17 | 1140 |
| HSV1-UL19-829 | - | GGCGCUGCUCUGCUCGG | 17 | 1215 |
| HSV1-UL19-870 | - | CUCGGAGCGCGCGUCGG | 17 | 1256 |
| HSV1-UL19-675 | - | GUUGACGCGGCGGUCGG | 17 | 1061 |
| HSV1-UL19-957 | - | CGUGGAGACGGGUUCGG | 17 | 1343 |
| HSV1-UL19-1553 | + | CGUUCAUCAGCGAAGGG | 17 | 1939 |
| HSV1-UL19-3229 | + | ACUACCCCAAGGAAGGG | 17 | 7828 |
| HSV1-UL19-595 | - | CCGCCUGGCCACCAGGG | 17 | 981 |
| HSV1-UL19-1686 | + | CACCAUGUUCUGCAGGG | 17 | 2072 |
| HSV1-UL19-1895 | + | GGCACUGCGGAGGAGGG | 17 | 2281 |
| HSV1-UL19-1846 | + | GUGGUCGUACAUGAGGG | 17 | 2232 |
| HSV1-UL19-690 | - | AGCGCCUGGCCCACGGG | 17 | 1076 |
| HSV1-UL19-797 | - | UGACCGGCCGCACCGGG | 17 | 1183 |
| HSV1-UL19-1879 | + | GUUCGCGGCACCCCGGG | 17 | 2265 |
| HSV1-UL19-1761 | + | UGGCCCCCAGAGCCGGG | 17 | 2147 |
| HSV1-UL19-606 | - | GGCGGGCCACCGCCGGG | 17 | 992 |
| HSV1-UL19-1749 | + | GGUCGCGCAGGGCCGGG | 17 | 2135 |
| HSV1-UL19-708 | - | GACGUGGAGCUGCCGGG | 17 | 1094 |
| HSV1-UL19-1536 | + | AGGCGCGGGGAUCCGGG | 17 | 1922 |
| HSV1-UL19-783 | - | UGCCGGGUUAGCGCGGG | 17 | 1169 |
| HSV1-UL19-614 | - | AUGCCGACACGCGCGGG | 17 | 1000 |
| HSV1-UL19-881 | - | GGCACGAAACUGGCGGG | 17 | 1267 |
| HSV1-UL19-1447 | + | AACAACAAAGCCAGGGG | 17 | 1833 |
| HSV1-UL19-1651 | + | CAGUCCCACACGAGGGG | 17 | 2037 |
| HSV1-UL19-1739 | + | ACAGCGCGUGGACGGGG | 17 | 2125 |
| HSV1-UL19-709 | - | ACGUGGAGCUGCCGGGG | 17 | 1095 |
| HSV1-UL19-3230 | + | CCCAAGGAAGGGCGGGG | 17 | 7829 |
| HSV1-UL19-882 | - | GCACGAAACUGGCGGGG | 17 | 1268 |
| HSV1-UL19-1544 | + | AAAAACAGCUGGCGGGG | 17 | 1930 |
| HSV1-UL19-1481 | + | GC GCUGUUUGAUC GGGG | 17 | 1867 |
| HSV1-UL19-1830 | + | GUCGGUCGACACGGGGG | 17 | 2216 |
| HSV1-UL19-1531 | + | AUCCGGGUGGCCGGGGG | 17 | 1917 |
| HSV1-UL19-710 | - | CGUGGAGCUGCCGGGGG | 17 | 1096 |
| HSV1-UL19-798 | - | CCGGCCGCACCGGGGGG | 17 | 1184 |
| HSV1-UL19-734 | - | GGGCCUGGAGCUGGGGG | 17 | 1120 |
| HSV1-UL19-702 | - | CGACUUCUUUGUGGGGG | 17 | 1088 |
| HSV1-UL19-768 | - | ACCGGCCCGGAGCUGGG | 17 | 1154 |
| HSV1-UL19-1728 | + | AUGGUCCAGAUGCUGGG | 17 | 2114 |
| HSV1-UL19-1501 | + | CACGCGCGUCGUCUGGG | 17 | 1887 |
| HSV1-UL19-809 | - | CACCCUGCAGAACAUGG | 17 | 1195 |
| HSV1-UL19-1714 | + | CUGCAGCGUGAGCAUGG | 17 | 2100 |
| HSV1-UL19-857 | - | GACCUACGCGCUCAUGG | 17 | 1243 |
| HSV1-UL19-1659 | + | CCCGGCAGUCGCGAUGG | 17 | 2045 |
| HSV1-UL19-1614 | + | GCGAACGGCGGCGAUGG | 17 | 2000 |
| HSV1-UL19-1776 | + | ACCCAAACCCGGGAUGG | 17 | 2162 |
| HSV1-UL19-1622 | + | CUCGCUGCCGUGUAUGG | 17 | 2008 |
| HSV1-UL19-877 | - | GCCCGGCACGAAACUGG | 17 | 1263 |
| HSV1-UL19-1549 | + | ACAGGCGCAGCACCUGG | 17 | 1935 |
| HSV1-UL19-760 | - | CGUGUACCGGGACCUGG | 17 | 1146 |
| HSV1-UL19-1455 | + | CGCCCGGGCCACCCUGG | 17 | 1841 |
| HSV1-UL19-648 | - | CGUUCCCUACCCCCUGG | 17 | 1034 |
| HSV1-UL19-686 | - | UGUUUUUGAACGCCUGG | 17 | 1072 |
| HSV1-UL19-1547 | + | CCCAAAAAAACAGCUGG | 17 | 1933 |
| HSV1-UL19-733 | - | CCGGGGCCUGGAGCUGG | 17 | 1119 |
| HSV1-UL19-1877 | + | AAACUGUACGUCGCUGG | 17 | 2263 |
| HSV1-UL19-1729 | + | UAUGGUCCAGAUGCUGG | 17 | 2115 |
| HSV1-UL19-816 | - | GCACCCGGCCAAUCUGG | 17 | 1202 |
| HSV1-UL19-851 | - | GGUCCCCCCGGCUCUGG | 17 | 1237 |
| HSV1-UL19-1502 | + | GCACGCGCGUCGUCUGG | 17 | 1888 |
| HSV1-UL19-645 | - | GAAGCUGGUCUUUCUGG | 17 | 1031 |
| HSV1-UL19-620 | - | GUCCUUCCUGAAAGUGG | 17 | 1006 |
| HSV1-UL19-680 | - | GUACGGGGCGUACGUGG | 17 | 1066 |
| HSV1-UL19-963 | - | GUGCCGCGAACUCGUGG | 17 | 1349 |
| HSV1-UL19-902 | - | GCGGAACGCGGUCGUGG | 17 | 1288 |
| HSV1-UL19-1496 | + | GCAUCUCCAGCAGGUGG | 17 | 1882 |
| HSV1-UL19-1613 | + | AACGGCGGCGAUGGUGG | 17 | 1999 |
| HSV1-UL19-1509 | + | GUAGGGAACGUUGGUGG | 17 | 1895 |
| HSV1-UL19-1723 | + | UAUGCGCAUGUUGGUGG | 17 | 2109 |
| HSV1-UL19-701 | - | GUUCGACUUCUUUGUGG | 17 | 1087 |
| HSV1-UL19-1561 | + | CGCCCCGUACGGAUUGG | 17 | 1947 |
| HSV1-UL19-1510 | + | GGGGUAGGGAACGUUGG | 17 | 1896 |
| HSV1-UL19-1724 | + | GAAUAUGCGCAUGUUGG | 17 | 2110 |
| HSV1-UL19-1870 | + | CAGGCAGCGAUGUUUGG | 17 | 2256 |
| HSV1-UL19-664 | - | GCCAGCUGUUUUUUUGG | 17 | 1050 |
| HSV1-UL19-1848 | + | GGCCGUGGUCGUACAUG | 17 | 2234 |
| HSV1-UL19-774 | - | ACUGUGACGCCCUGAUG | 17 | 1160 |
| HSV1-UL19-1899 | + | CCCCGUUGCGGGCACUG | 17 | 2285 |
| HSV1-UL19-899 | - | CGGCAGCCGUGUACCUG | 17 | 1285 |
| HSV1-UL19-685 | - | CUGUUUUUGAACGCCUG | 17 | 1071 |
| HSV1-UL19-906 | - | UGGCGGGAAACCGCCUG | 17 | 1292 |
| HSV1-UL19-732 | - | CCCGGGGCCUGGAGCUG | 17 | 1118 |
| HSV1-UL19-1695 | + | CCGUCACGGGGUCGCUG | 17 | 2081 |
| HSV1-UL19-1730 | + | GUAUGGUCCAGAUGCUG | 17 | 2116 |
| HSV1-UL19-850 | - | UGGUCCCCCCGGCUCUG | 17 | 1236 |
| HSV1-UL19-1503 | + | CGCACGCGCGUCGUCUG | 17 | 1889 |
| HSV1-UL19-1571 | + | CAGCUGCUGCAUGUCUG | 17 | 1957 |
| HSV1-UL19-964 | - | CUCGUGGAGGACCCGUG | 17 | 1350 |
| HSV1-UL19-772 | - | UGCUGCCACCCCUCGUG | 17 | 1158 |
| HSV1-UL19-1676 | + | CAGUCCGCCCCCCGGUG | 17 | 2062 |
| HSV1-UL19-1468 | + | GCUGCGUGGCCGUGGUG | 17 | 1854 |
| HSV1-UL19-700 | - | CGUUCGACUUCUUUGUG | 17 | 1086 |
| HSV1-UL19-3231 | - | CGCCCCGCCCUUCCUUG | 17 | 7830 |
| HSV1-UL19-1679 | + | CGUAGUAGUAAAUCUUG | 17 | 2065 |
| HSV1-UL19-1618 | + | ACACCGCCGGGUAGUUG | 17 | 2004 |
| HSV1-UL19-1863 | + | CCGGCGAGGCCCCGUUG | 17 | 2249 |
| HSV1-UL19-1901 | + | CGUGGGCUUCCCCGUUG | 17 | 2287 |
| HSV1-UL19-1667 | + | GCAGCUGGCCGUCGUUG | 17 | 2053 |
| HSV1-UL19-1796 | + | UGUCGGUGACGGGGUUG | 17 | 2182 |
| HSV1-UL19-1717 | + | UGCGUUCGGCCAUGUUG | 17 | 2103 |
| HSV1-UL19-698 | - | CGCGUUCGACUUCUUUG | 17 | 1084 |
| HSV1-UL19-1802 | + | CCCAGGUAAAAGUUUUG | 17 | 2188 |
| HSV1-UL19-663 | - | CGCCAGCUGUUUUUUUG | 17 | 1049 |
| HSV1-UL19-1748 | + | CAGGGCCGGGGGGAAAU | 17 | 2134 |
| HSV1-UL19-891 | - | AACCCCGUCACCGACAU | 17 | 1277 |
| HSV1-UL19-1576 | + | GGCCUUCCGCGACCCAU | 17 | 1962 |
| HSV1-UL19-642 | - | GCGGAUCUGGUGUCCAU | 17 | 1028 |
| HSV1-UL19-1451 | + | UCCAGGUAUCGUUGCAU | 17 | 1837 |
| HSV1-UL19-1710 | + | CGUGUUGGCCACCAGAU | 17 | 2096 |
| HSV1-UL19-693 | - | CCACGGGCGGGUCCGAU | 17 | 1079 |
| HSV1-UL19-668 | - | UCUCUGGAACACGCGAU | 17 | 1054 |
| HSV1-UL19-1562 | + | GUACGCCCCGUACGGAU | 17 | 1948 |
| HSV1-UL19-630 | - | GUCACGGCGCUCGUGAU | 17 | 1016 |
| HSV1-UL19-1484 | + | AGGAGGCGCUGUUUGAU | 17 | 1870 |
| HSV1-UL19-1528 | + | GGCCGGGGGCGGGGACU | 17 | 1914 |
| HSV1-UL19-1824 | + | UGCGCGCCGCGGCACCU | 17 | 2210 |
| HSV1-UL19-885 | - | CGCGCGAACGUGGACCU | 17 | 1271 |
| HSV1-UL19-751 | - | UACGUCGUGACCUACCU | 17 | 1137 |
| HSV1-UL19-893 | - | CCCCAAAACUUUUACCU | 17 | 1279 |
| HSV1-UL19-652 | - | ACGUUCGUCCUGCCCCU | 17 | 1038 |
| HSV1-UL19-684 | - | GCUGUUUUUGAACGCCU | 17 | 1070 |
| HSV1-UL19-905 | - | GUGGCGGGAAACCGCCU | 17 | 1291 |
| HSV1-UL19-1669 | + | GUCCGCGGCUCGGGCCU | 17 | 2055 |
| HSV1-UL19-1634 | + | CACGGCCAUGCACUCCU | 17 | 2020 |
| HSV1-UL19-582 | - | AACGUCCAGGCCGUCCU | 17 | 968 |
| HSV1-UL19-872 | - | UCGGAGGCGUACUUCCU | 17 | 1258 |
| HSV1-UL19-3232 | - | ACCGCCCCGCCCUUCCU | 17 | 7831 |
| HSV1-UL19-600 | - | GAGACGAGCUUUUUCCU | 17 | 986 |
| HSV1-UL19-1640 | + | AAAGUCAUCAACCAGCU | 17 | 2026 |
| HSV1-UL19-1647 | + | CAUUAGGUGAUUCAGCU | 17 | 2033 |
| HSV1-UL19-767 | - | CUGACCGGCCCGGAGCU | 17 | 1153 |
| HSV1-UL19-731 | - | GCCCGGGGCCUGGAGCU | 17 | 1117 |
| HSV1-UL19-1461 | + | GAAGCUUCGCUUUAGCU | 17 | 1847 |
| HSV1-UL19-1696 | + | UCCGUCACGGGGUCGCU | 17 | 2082 |
| HSV1-UL19-1672 | + | CCGCGGCGUCCGCGGCU | 17 | 2058 |
| HSV1-UL19-1788 | + | CACGUUCGCGCGCGGCU | 17 | 2174 |
| HSV1-UL19-1817 | + | GAACACGGGGACGGGCU | 17 | 2203 |
| HSV1-UL19-1731 | + | GGUAUGGUCCAGAUGCU | 17 | 2117 |
| HSV1-UL19-828 | - | AACGGCGCUGCUCUGCU | 17 | 1214 |
| HSV1-UL19-849 | - | CUGGUCCCCCCGGCUCU | 17 | 1235 |
| HSV1-UL19-1504 | + | GCGCACGCGCGUCGUCU | 17 | 1890 |
| HSV1-UL19-868 | - | UGAGAACGUGCUGUUCU | 17 | 1254 |
| HSV1-UL19-1904 | + | GAGAUACUGCGCGAAGU | 17 | 2290 |
| HSV1-UL19-1766 | + | GAUCGACGAAAAGUAGU | 17 | 2152 |
| HSV1-UL19-1511 | + | CAGGGGGUAGGGAACGU | 17 | 1897 |
| HSV1-UL19-1588 | + | CCCCGGCAGCUCCACGU | 17 | 1974 |
| HSV1-UL19-1490 | + | GUACGUCACCGGCACGU | 17 | 1876 |
| HSV1-UL19-634 | - | CGAAGUCUGGACGACGU | 17 | 1020 |
| HSV1-UL19-1835 | + | GCGGAAGUAGUUGACGU | 17 | 2221 |
| HSV1-UL19-951 | - | CUUUAAGUUCUUUACGU | 17 | 1337 |
| HSV1-UL19-940 | - | GGCCACGGCCAACCCGU | 17 | 1326 |
| HSV1-UL19-1574 | + | CCAUCGGACCCGCCCGU | 17 | 1960 |
| HSV1-UL19-1720 | + | GGUGUUGGCGCCCGCGU | 17 | 2106 |
| HSV1-UL19-1772 | + | CCCCGCCAUGAGCGCGU | 17 | 2158 |
| HSV1-UL19-1902 | + | GUGGGUCUCCCGCGCGU | 17 | 2288 |
| HSV1-UL19-869 | - | GUUCUCGGAGCGCGCGU | 17 | 1255 |
| HSV1-UL19-887 | - | AACGUGGACCUGGGCGU | 17 | 1273 |
| HSV1-UL19-1493 | + | GAGCGCCGUGACCAGGU | 17 | 1879 |
| HSV1-UL19-1583 | + | CAGCUCAAGAGCCAGGU | 17 | 1969 |
| HSV1-UL19-1630 | + | AAGGUCUCCCCCGAGGU | 17 | 2016 |
| HSV1-UL19-672 | - | AACGUUGACGCGGCGGU | 17 | 1058 |
| HSV1-UL19-1857 | + | CUGCGACGCCCACGGGU | 17 | 2243 |
| HSV1-UL19-1888 | + | GGCGCAGGUGAGCGGGU | 17 | 2274 |
| HSV1-UL19-1513 | + | GGCGCCCACCAGGGGGU | 17 | 1899 |
| HSV1-UL19-735 | - | GGCCUGGAGCUGGGGGU | 17 | 1121 |
| HSV1-UL19-649 | - | GUUCCCUACCCCCUGGU | 17 | 1035 |
| HSV1-UL19-1445 | + | CACGUGCAGCAUCUGGU | 17 | 1831 |
| HSV1-UL19-1745 | + | CGCGUUCGCCACGUGGU | 17 | 2131 |
| HSV1-UL19-1861 | + | GGCCCCGUUGAGGUGGU | 17 | 2247 |
| HSV1-UL19-1800 | + | GGGAAGGUUGCCCAUGU | 17 | 2186 |
| HSV1-UL19-1725 | + | GUC GAAUAUGC GCAUGU | 17 | 2111 |
| HSV1-UL19-1869 | + | AUGUUUGGCGGCGAUGU | 17 | 2255 |
| HSV1-UL19-1781 | + | UCACAAAGCGGUCCUGU | 17 | 2167 |
| HSV1-UL19-3215 | + | CGUUGCGAGCGAGCUGU | 17 | 7814 |
| HSV1-UL19-1767 | + | GCUGCACGACGGGCUGU | 17 | 2153 |
| HSV1-UL19-1711 | + | CAUGGCGUUGACCGUGU | 17 | 2097 |
| HSV1-UL19-1478 | + | CGGCCGCCCGCGCGUGU | 17 | 1864 |
| HSV1-UL19-773 | - | GCUGCCACCCCUCGUGU | 17 | 1159 |
| HSV1-UL19-1721 | + | GGUGGUGGACGCGGUGU | 17 | 2107 |
| HSV1-UL19-1582 | + | AGCCAGGUUGGCGUUGU | 17 | 1968 |
| HSV1-UL19-1716 | + | GCGUUCGGCCAUGUUGU | 17 | 2102 |
| HSV1-UL19-699 | - | GCGUUCGACUUCUUUGU | 17 | 1085 |
| HSV1-UL19-1747 | + | AGGGCCGGGGGGAAAUU | 17 | 2133 |
| HSV1-UL19-1648 | + | GCGCCGGGUCUCGCAUU | 17 | 2034 |
| HSV1-UL19-3233 | - | CCGCCCCGCCCUUCCUU | 17 | 7832 |
| HSV1-UL19-1489 | + | CACCGGCACGUCGGCUU | 17 | 1875 |
| HSV1-UL19-1718 | + | CAGCGCCGUUGUGCGUU | 17 | 2104 |
| HSV1-UL19-956 | - | GAUC GUGGAGAC GGGUU | 17 | 1342 |
| HSV1-UL19-865 | - | GGCCUCCAUCCCGGGUU | 17 | 1251 |
| HSV1-UL19-1871 | + | CUCCAGGCAGCGAUGUU | 17 | 2257 |
| HSV1-UL19-908 | - | CAGCCCGUCCCCGUGUU | 17 | 1294 |
| HSV1-UL19-1735 | + | AAAAAUAGUCGCCAUUU | 17 | 2121 |
| HSV1-UL19-1804 | + | GGCCCAGGUAAAAGUUU | 17 | 2190 |
| HSV1-UL19-866 | - | GCCUCCAUCCCGGGUUU | 17 | 1252 |
| HSV1-UL19-1803 | + | GCCCAGGUAAAAGUUUU | 17 | 2189 |
| HSV1-UL19-661 | - | CCCGCCAGCUGUUUUUU | 17 | 1047 |
| HSV1-UL19-662 | - | CCGCCAGCUGUUUUUUU | 17 | 1048 |
| HSV1-UL19-445 | - | CUGGACCAUACCAUCCAAAA | 20 | 831 |
| HSV1-UL19-195 | - | GCUGCACGUGCUGCUGGAAA | 20 | 581 |
| HSV1-UL19-434 | - | CAACAUGGCCGAACGCACAA | 20 | 820 |
| HSV1-UL19-425 | - | GUCAACGCCAUGUUUCACAA | 20 | 811 |
| HSV1-UL19-198 | - | AUGCAACGAUACCUGGACAA | 20 | 584 |
| HSV1-UL19-574 | - | CUCCUCCGCAGUGCCCGCAA | 20 | 960 |
| HSV1-UL19-554 | - | AACGGGGCCUACCACCUCAA | 20 | 940 |
| HSV1-UL19-580 | - | UAUGACGCAUCCCCGCUCAA | 20 | 966 |
| HSV1-UL19-1087 | + | CGUCAACGUUCAUCAGCGAA | 20 | 1473 |
| HSV1-UL19-1150 | + | GUCGUCGAACGCCCCGCGAA | 20 | 1536 |
| HSV1-UL19-262 | - | UGUUCAAUCCGGUCAUGGAA | 20 | 648 |
| HSV1-UL19-231 | - | UACGGCGAGAUGGUCCUGAA | 20 | 617 |
| HSV1-UL19-327 | - | GCCACCUGGCGCGUGGUGAA | 20 | 713 |
| HSV1-UL19-551 | - | UACGGGGACCUGCUCUAUAA | 20 | 937 |
| HSV1-UL19-1245 | + | CACGCGCCCGUUGUGAAACA | 20 | 1631 |
| HSV1-UL19-433 | - | GCAGGUGCUGGCCCACAACA | 20 | 819 |
| HSV1-UL19-424 | - | GGCCAAUCUGGUGGCCAACA | 20 | 810 |
| HSV1-UL19-415 | - | AUACGCCACCCUGCAGAACA | 20 | 801 |
| HSV1-UL19-1356 | + | CACCUGGGCGCACCCGAACA | 20 | 1742 |
| HSV1-UL19-1362 | + | GGUGGCGAUAAACUCACACA | 20 | 1748 |
| HSV1-UL19-1419 | + | GCCUCCUGAAACAGGCCACA | 20 | 1805 |
| HSV1-UL19-1085 | + | CAUCAGCGAAGGGUGGCACA | 20 | 1471 |
| HSV1-UL19-497 | - | CCGCAACCCCGUCACCGACA | 20 | 883 |
| HSV1-UL19-1367 | + | GUAGUUGACGUCGGUCGACA | 20 | 1753 |
| HSV1-UL19-536 | - | GGGCGUUUACGCGGGGGACA | 20 | 922 |
| HSV1-UL19-350 | - | CUGCUGCAGGCCGCCAUACA | 20 | 736 |
| HSV1-UL19-1198 | + | GUUACAUGCCGCCGCGUACA | 20 | 1584 |
| HSV1-UL19-1345 | + | GACCGCGUUCCGCAGGUACA | 20 | 1731 |
| HSV1-UL19-1168 | + | CGCCACCAGGUCCCGGUACA | 20 | 1554 |
| HSV1-UL19-1049 | + | CAGGUCCAUGGCGCCCACCA | 20 | 1435 |
| HSV1-UL19-201 | - | CAACGGCCGCCUGGCCACCA | 20 | 587 |
| HSV1-UL19-217 | - | CUGGCUCGUGGACCUCACCA | 20 | 603 |
| HSV1-UL19-541 | - | ACCGCCCUCAUGUACGACCA | 20 | 927 |
| HSV1-UL19-992 | + | UCGCUUUAGCUCGGCGACCA | 20 | 1378 |
| HSV1-UL19-1292 | + | CCCCAGAGCCGGGGGGACCA | 20 | 1678 |
| HSV1-UL19-522 | - | CGGCGCGCAGGGAUGGACCA | 20 | 908 |
| HSV1-UL19-1392 | + | GAAAAUCGCUGCGACGCCCA | 20 | 1778 |
| HSV1-UL19-1055 | + | GACCGGAUUGAACAGGCCCA | 20 | 1441 |
| HSV1-UL19-295 | - | UGGGGGCAGCGCCUGGCCCA | 20 | 681 |
| HSV1-UL19-982 | + | CAUCGGCAACAACAAAGCCA | 20 | 1368 |
| HSV1-UL19-343 | - | GGGGGUGGGACGCCACGCCA | 20 | 729 |
| HSV1-UL19-1134 | + | CGCCGCCGGACAUAGCGCCA | 20 | 1520 |
| HSV1-UL19-257 | - | CUACCCCCUGGUGGGCGCCA | 20 | 643 |
| HSV1-UL19-1307 | + | GAGCUGAUGAUGCAAGGCCA | 20 | 1693 |
| HSV1-UL19-1006 | + | CGUCAGACGGGGCACGGCCA | 20 | 1392 |
| HSV1-UL19-545 | - | GUCCCGGGCCUUCGCGGCCA | 20 | 931 |
| HSV1-UL19-1157 | + | CUGGACCACGAGCCGGGCCA | 20 | 1543 |
| HSV1-UL19-1142 | + | GGCGAUGGUGGCGGGGGCCA | 20 | 1528 |
| HSV1-UL19-1171 | + | GUCAUCAACCAGCUGGGCCA | 20 | 1557 |
| HSV1-UL19-496 | - | CUACACCGCCGUCGUGGCCA | 20 | 882 |
| HSV1-UL19-1040 | + | GGCGUAGAUGCGCUUCUCCA | 20 | 1426 |
| HSV1-UL19-1051 | + | CAGGACGAACGUCAGGUCCA | 20 | 1437 |
| HSV1-UL19-1190 | + | GCAGUCGCGAUGGCGGUCCA | 20 | 1576 |
| HSV1-UL19-565 | - | GACGGGUUCGGCGGUGUCCA | 20 | 951 |
| HSV1-UL19-1400 | + | GACGUAAAGAACUUAAAGCA | 20 | 1786 |
| HSV1-UL19-1248 | + | CAGCACCUGCAGCGUGAGCA | 20 | 1634 |
| HSV1-UL19-520 | - | CCCAGGUGCCGCGGCGCGCA | 20 | 906 |
| HSV1-UL19-1287 | + | CUGCCGCGACAGGUCGCGCA | 20 | 1673 |
| HSV1-UL19-1028 | + | CACGUCGUCCAGACUUCGCA | 20 | 1414 |
| HSV1-UL19-193 | - | CGGGGCGUUUGAGCGCGGCA | 20 | 579 |
| HSV1-UL19-1007 | + | GGCAUGCGUCAGACGGGGCA | 20 | 1393 |
| HSV1-UL19-238 | - | CACGGCGCUCGUGAUGGGCA | 20 | 624 |
| HSV1-UL19-208 | - | GACGAGCUUUUUCCUGGGCA | 20 | 594 |
| HSV1-UL19-1308 | + | CGUCUUGAGCUGAUGAUGCA | 20 | 1694 |
| HSV1-UL19-1220 | + | CGGGACCACCAUGUUCUGCA | 20 | 1606 |
| HSV1-UL19-363 | - | AGACCUUCCCGAGGAGUGCA | 20 | 749 |
| HSV1-UL19-1239 | + | CACCAGAUUGGCCGGGUGCA | 20 | 1625 |
| HSV1-UL19-1211 | + | GUAGUAAAUCUUGUGGUGCA | 20 | 1597 |
| HSV1-UL19-1371 | + | GCGCGUCCCCGGGGGUUGCA | 20 | 1757 |
| HSV1-UL19-1188 | + | CAGGGCCGCGCGCCGCAUCA | 20 | 1574 |
| HSV1-UL19-579 | - | CUAUGACGCAUCCCCGCUCA | 20 | 965 |
| HSV1-UL19-463 | - | CGCGCUGACCUACGCGCUCA | 20 | 849 |
| HSV1-UL19-1233 | + | GGGGUGCGCGGGGUCCGUCA | 20 | 1619 |
| HSV1-UL19-1175 | + | GCCCAGCUCCGGGCCGGUCA | 20 | 1561 |
| HSV1-UL19-261 | - | GGGCCUGUUCAAUCCGGUCA | 20 | 647 |
| HSV1-UL19-235 | - | GAACGGGGCCAACCUGGUCA | 20 | 621 |
| HSV1-UL19-468 | - | CUUGCAUCAUCAGCUCAAGA | 20 | 854 |
| HSV1-UL19-1010 | + | GCGUGUCGGCAUGCGUCAGA | 20 | 1396 |
| HSV1-UL19-1215 | + | GCAGCAGCGGCCCCGCGAGA | 20 | 1601 |
| HSV1-UL19-230 | - | GCCGGUGACGUACGGCGAGA | 20 | 616 |
| HSV1-UL19-1342 | + | CUGCCGCGUUGUCCAGGAGA | 20 | 1728 |
| HSV1-UL19-561 | - | GGAGCGCCUGAUCGUGGAGA | 20 | 947 |
| HSV1-UL19-395 | - | GCGGACUUCAACCGCAACGA | 20 | 781 |
| HSV1-UL19-1186 | + | GGCGUCACAGUCCCACACGA | 20 | 1572 |
| HSV1-UL19-1326 | + | GACGGUUGCCGUGGCCACGA | 20 | 1712 |
| HSV1-UL19-1302 | + | CUCGCGGACGUGCUGCACGA | 20 | 1688 |
| HSV1-UL19-1313 | + | AAAGCGGUCCUGUCGGACGA | 20 | 1699 |
| HSV1-UL19-1039 | + | AAAGACCAGCUUCUCGCCGA | 20 | 1425 |
| HSV1-UL19-299 | - | UGGCCCACGGGCGGGUCCGA | 20 | 685 |
| HSV1-UL19-1088 | + | GCGUCAACGUUCAUCAGCGA | 20 | 1474 |
| HSV1-UL19-1388 | + | CGGGUUGGCCGUGGCCGCGA | 20 | 1774 |
| HSV1-UL19-1193 | + | CGCUAACCCGGCAGUCGCGA | 20 | 1579 |
| HSV1-UL19-1148 | + | CGCCCCGCGAACGGCGGCGA | 20 | 1534 |
| HSV1-UL19-999 | + | GUCCACGAGCCAGGCCUCGA | 20 | 1385 |
| HSV1-UL19-223 | - | ACGCGCGGGCGGCCGGUCGA | 20 | 609 |
| HSV1-UL19-439 | - | ACCAACAUGCGCAUAUUCGA | 20 | 825 |
| HSV1-UL19-1093 | + | GUACGGAUUGGCGGCUUCGA | 20 | 1479 |
| HSV1-UL19-538 | - | GUUUACGCGGGGGACAAGGA | 20 | 924 |
| HSV1-UL19-1020 | + | GGUGUCCUCCACUUUCAGGA | 20 | 1406 |
| HSV1-UL19-976 | + | GCGCUCAAACGCCCCGAGGA | 20 | 1362 |
| HSV1-UL19-1429 | + | GUUGCGGGCACUGCGGAGGA | 20 | 1815 |
| HSV1-UL19-1004 | + | AGACGGGGCACGGCCACGGA | 20 | 1390 |
| HSV1-UL19-302 | - | CGGGUCCGAUGGGUCGCGGA | 20 | 688 |
| HSV1-UL19-1328 | + | GUCGGUGACGGGGUUGCGGA | 20 | 1714 |
| HSV1-UL19-521 | - | GGUGCCGCGGCGCGCAGGGA | 20 | 907 |
| HSV1-UL19-1310 | + | CGGUGAACCCAAACCCGGGA | 20 | 1696 |
| HSV1-UL19-1385 | + | GUGGCCGCGAAGGCCCGGGA | 20 | 1771 |
| HSV1-UL19-1075 | + | AAACAGCUGGCGGGGCGGGA | 20 | 1461 |
| HSV1-UL19-1165 | + | CGGCCAUGCACUCCUCGGGA | 20 | 1551 |
| HSV1-UL19-1353 | + | GGCGCACCCGAACACGGGGA | 20 | 1739 |
| HSV1-UL19-1271 | + | GAACAGCGCGUGGACGGGGA | 20 | 1657 |
| HSV1-UL19-272 | - | CCAGCUGUUUUUUUGGGGGA | 20 | 658 |
| HSV1-UL19-1334 | + | CCAGGUAAAAGUUUUGGGGA | 20 | 1720 |
| HSV1-UL19-429 | - | AACGGGCGCGUGGUAGUGGA | 20 | 815 |
| HSV1-UL19-1275 | + | CCCGGCGAACAGCGCGUGGA | 20 | 1661 |
| HSV1-UL19-3212 | + | GUUGCGAGCGAGCUGUUGGA | 20 | 7811 |
| HSV1-UL19-1267 | + | AAAAUAGUCGCCAUUUUGGA | 20 | 1653 |
| HSV1-UL19-1380 | + | CUGGCCGUGGUCGUACAUGA | 20 | 1766 |
| HSV1-UL19-443 | - | GCACGCCGGAAUCCUGCUGA | 20 | 829 |
| HSV1-UL19-407 | - | CAAGAUUUACUACUACGUGA | 20 | 793 |
| HSV1-UL19-420 | - | GUGCCCCAGCGACCCCGUGA | 20 | 806 |
| HSV1-UL19-236 | - | CCUGGUCACGGCGCUCGUGA | 20 | 622 |
| HSV1-UL19-1332 | + | AAGGUUGCCCAUGUCGGUGA | 20 | 1718 |
| HSV1-UL19-229 | - | GCCGACGUGCCGGUGACGUA | 20 | 615 |
| HSV1-UL19-1096 | + | GCCGCCACGUACGCCCCGUA | 20 | 1482 |
| HSV1-UL19-283 | - | GUCGAAGCCGCCAAUCCGUA | 20 | 669 |
| HSV1-UL19-548 | - | GCGUCGCAGCGAUUUUCGUA | 20 | 934 |
| HSV1-UL19-1045 | + | AUGGCGCCCACCAGGGGGUA | 20 | 1431 |
| HSV1-UL19-1266 | + | AGUCGCCAUUUUGGAUGGUA | 20 | 1652 |
| HSV1-UL19-1156 | + | GACGUGCUCGCUGCCGUGUA | 20 | 1542 |
| HSV1-UL19-1089 | + | CUUCGACGGGGUCGCGGUUA | 20 | 1475 |
| HSV1-UL19-483 | - | CAGGUGGCCCGGCACGAAAC | 20 | 869 |
| HSV1-UL19-1420 | + | GCGGGUAGGCCUCCUGAAAC | 20 | 1806 |
| HSV1-UL19-426 | - | UCAACGCCAUGUUUCACAAC | 20 | 812 |
| HSV1-UL19-575 | - | UCCUCCGCAGUGCCCGCAAC | 20 | 961 |
| HSV1-UL19-40 | + | GCUGUUGGAUGGCGCGCAAC | 20 | 421 |
| HSV1-UL19-555 | - | ACGGGGCCUACCACCUCAAC | 20 | 941 |
| HSV1-UL19-245 | - | AGGAGCAGCUCGACCUGAAC | 20 | 631 |
| HSV1-UL19-232 | - | ACGGCGAGAUGGUCCUGAAC | 20 | 618 |
| HSV1-UL19-1057 | + | GUUCCAUGACCGGAUUGAAC | 20 | 1443 |
| HSV1-UL19-552 | - | ACGGGGACCUGCUCUAUAAC | 20 | 938 |
| HSV1-UL19-1355 | + | ACCUGGGCGCACCCGAACAC | 20 | 1741 |
| HSV1-UL19-1418 | + | CCUCCUGAAACAGGCCACAC | 20 | 1804 |
| HSV1-UL19-1366 | + | UAGUUGACGUCGGUCGACAC | 20 | 1752 |
| HSV1-UL19-1197 | + | UUACAUGCCGCCGCGUACAC | 20 | 1583 |
| HSV1-UL19-1391 | + | AAAAUCGCUGCGACGCCCAC | 20 | 1777 |
| HSV1-UL19-296 | - | GGGGGCAGCGCCUGGCCCAC | 20 | 682 |
| HSV1-UL19-1306 | + | AGCUGAUGAUGCAAGGCCAC | 20 | 1692 |
| HSV1-UL19-1398 | + | UUAAAGCAGGGGCUGAGCAC | 20 | 1784 |
| HSV1-UL19-400 | - | CCGCGGAUGACCGGCCGCAC | 20 | 786 |
| HSV1-UL19-1161 | + | UCCAGUAGCUGGUGAUGCAC | 20 | 1547 |
| HSV1-UL19-1024 | + | ACCAUCUCGCCGUACGUCAC | 20 | 1410 |
| HSV1-UL19-1232 | + | GGGUGCGCGGGGUCCGUCAC | 20 | 1618 |
| HSV1-UL19-469 | - | UUGCAUCAUCAGCUCAAGAC | 20 | 855 |
| HSV1-UL19-1009 | + | CGUGUCGGCAUGCGUCAGAC | 20 | 1395 |
| HSV1-UL19-1084 | + | CGAUCGCGUGUUCCAGAGAC | 20 | 1470 |
| HSV1-UL19-562 | - | GAGCGCCUGAUCGUGGAGAC | 20 | 948 |
| HSV1-UL19-1301 | + | UCGCGGACGUGCUGCACGAC | 20 | 1687 |
| HSV1-UL19-474 | - | UGGGUUCACCGUCGUCCGAC | 20 | 860 |
| HSV1-UL19-1289 | + | CCAGGGGGACCUGCCGCGAC | 20 | 1675 |
| HSV1-UL19-1012 | + | GUGACGAGCACCCCGUCGAC | 20 | 1398 |
| HSV1-UL19-224 | - | CGCGCGGGCGGCCGGUCGAC | 20 | 610 |
| HSV1-UL19-440 | - | CCAACAUGCGCAUAUUCGAC | 20 | 826 |
| HSV1-UL19-1092 | + | UACGGAUUGGCGGCUUCGAC | 20 | 1478 |
| HSV1-UL19-406 | - | GGCCGCACCGGGGGGCGGAC | 20 | 792 |
| HSV1-UL19-581 | - | CGCAUCCCCGCUCAAGGGAC | 20 | 967 |
| HSV1-UL19-1384 | + | UGGCCGCGAAGGCCCGGGAC | 20 | 1770 |
| HSV1-UL19-1352 | + | GCGCACCCGAACACGGGGAC | 20 | 1738 |
| HSV1-UL19-430 | - | ACGGGCGCGUGGUAGUGGAC | 20 | 816 |
| HSV1-UL19-1274 | + | CCGGCGAACAGCGCGUGGAC | 20 | 1660 |
| HSV1-UL19-1058 | + | GCGGCAAACCGUUCCAUGAC | 20 | 1444 |
| HSV1-UL19-399 | - | CCGCGGACGCCGCGGAUGAC | 20 | 785 |
| HSV1-UL19-371 | - | GUUGAUGACUUUACCCUGAC | 20 | 757 |
| HSV1-UL19-1331 | + | AGGUUGCCCAUGUCGGUGAC | 20 | 1717 |
| HSV1-UL19-354 | - | UCCAGUGCAUCACCAGCUAC | 20 | 740 |
| HSV1-UL19-284 | - | UCGAAGCCGCCAAUCCGUAC | 20 | 670 |
| HSV1-UL19-549 | - | CGUCGCAGCGAUUUUCGUAC | 20 | 935 |
| HSV1-UL19-364 | - | AGGAGUGCAUGGCCGUGUAC | 20 | 750 |
| HSV1-UL19-1312 | + | GACGACGGUGAACCCAAACC | 20 | 1698 |
| HSV1-UL19-304 | - | GCAGCCCGACAACGCCAACC | 20 | 690 |
| HSV1-UL19-234 | - | GGUCCUGAACGGGGCCAACC | 20 | 620 |
| HSV1-UL19-1194 | + | CGUGGCCCCCCGCGCUAACC | 20 | 1580 |
| HSV1-UL19-1050 | + | UCAGGUCCAUGGCGCCCACC | 20 | 1436 |
| HSV1-UL19-1170 | + | GGGCCUCGACGUGCGCCACC | 20 | 1556 |
| HSV1-UL19-325 | - | CGGGGGAGAUCCAGGCCACC | 20 | 711 |
| HSV1-UL19-267 | - | AGUCCCCGCCCCCGGCCACC | 20 | 653 |
| HSV1-UL19-1318 | + | CAGUUUCGUGCCGGGCCACC | 20 | 1704 |
| HSV1-UL19-200 | - | ACAACGGCCGCCUGGCCACC | 20 | 586 |
| HSV1-UL19-1083 | + | CCAGAGACAGGCGCAGCACC | 20 | 1469 |
| HSV1-UL19-401 | - | CGCGGAUGACCGGCCGCACC | 20 | 787 |
| HSV1-UL19-1358 | + | UCCCUGCGCGCCGCGGCACC | 20 | 1744 |
| HSV1-UL19-421 | - | CCCCGCGCACCCCCUGCACC | 20 | 807 |
| HSV1-UL19-378 | - | GAAUCACCUAAUGCGAGACC | 20 | 764 |
| HSV1-UL19-993 | + | UUCGCUUUAGCUCGGCGACC | 20 | 1379 |
| HSV1-UL19-366 | - | CAUGGCCGUGUACCGGGACC | 20 | 752 |
| HSV1-UL19-1293 | + | CCCCCAGAGCCGGGGGGACC | 20 | 1679 |
| HSV1-UL19-491 | - | GCCGCGCGCGAACGUGGACC | 20 | 877 |
| HSV1-UL19-1027 | + | CCAUCACGAGCGCCGUGACC | 20 | 1413 |
| HSV1-UL19-197 | - | GUUGCCGAUGCAACGAUACC | 20 | 583 |
| HSV1-UL19-347 | - | GUUCGACGACCGCAACUACC | 20 | 733 |
| HSV1-UL19-365 | - | GGAGUGCAUGGCCGUGUACC | 20 | 751 |
| HSV1-UL19-499 | - | CCUUCCCCAAAACUUUUACC | 20 | 885 |
| HSV1-UL19-1311 | + | ACGACGGUGAACCCAAACCC | 20 | 1697 |
| HSV1-UL19-396 | - | CCAGCUGCUGCACAACACCC | 20 | 782 |
| HSV1-UL19-989 | + | CAGGGUCGCCCGGGCCACCC | 20 | 1375 |
| HSV1-UL19-1416 | + | UCCACGAGUUCGCGGCACCC | 20 | 1802 |
| HSV1-UL19-205 | - | CAGGGUGGCCCGGGCGACCC | 20 | 591 |
| HSV1-UL19-303 | - | CGCGGAAGGCCAGAUGACCC | 20 | 689 |
| HSV1-UL19-525 | - | UCCGCCGGCCCUGCAACCCC | 20 | 911 |
| HSV1-UL19-1415 | + | CCACGAGUUCGCGGCACCCC | 20 | 1801 |
| HSV1-UL19-526 | - | CCGCCGGCCCUGCAACCCCC | 20 | 912 |
| HSV1-UL19-254 | - | CACCAACGUUCCCUACCCCC | 20 | 640 |
| HSV1-UL19-1122 | + | GCCGGGGGAACGUCCCCCCC | 20 | 1508 |
| HSV1-UL19-567 | - | GUACAGUUUAAGCGCCCCCC | 20 | 953 |
| HSV1-UL19-1210 | + | GCACGGUCCAGUCCGCCCCC | 20 | 1596 |
| HSV1-UL19-566 | - | CGUACAGUUUAAGCGCCCCC | 20 | 952 |
| HSV1-UL19-1105 | + | GUGGGCCAGGCGCUGCCCCC | 20 | 1491 |
| HSV1-UL19-1128 | + | AGGUGGCCUGGAUCUCCCCC | 20 | 1514 |
| HSV1-UL19-453 | - | CCUGUCGCGGCAGGUCCCCC | 20 | 839 |
| HSV1-UL19-454 | - | GCAGGUCCCCCUGGUCCCCC | 20 | 840 |
| HSV1-UL19-450 | - | GAACGCGCCCAAUUUCCCCC | 20 | 836 |
| HSV1-UL19-1378 | + | GUCCCCCGCGUAAACGCCCC | 20 | 1764 |
| HSV1-UL19-266 | - | GGGGACCUAGUCCCCGCCCC | 20 | 652 |
| HSV1-UL19-196 | - | GCUGGAAAAGGCGCCGCCCC | 20 | 582 |
| HSV1-UL19-418 | - | GUGGUCCCGGAGAUCGCCCC | 20 | 804 |
| HSV1-UL19-1138 | + | CACCCCCAGCUCCAGGCCCC | 20 | 1524 |
| HSV1-UL19-1348 | + | GGACGGGCUGGGCCGGCCCC | 20 | 1734 |
| HSV1-UL19-258 | - | CCUGACGUUCGUCCUGCCCC | 20 | 644 |
| HSV1-UL19-328 | - | GGUGAACGGCAACUUGCCCC | 20 | 714 |
| HSV1-UL19-1129 | + | CAGGUGGCCUGGAUCUCCCC | 20 | 1515 |
| HSV1-UL19-1375 | + | GCCCCCGGCGGCGCGUCCCC | 20 | 1761 |
| HSV1-UL19-318 | - | GCCGGGGGGGGACGUUCCCC | 20 | 704 |
| HSV1-UL19-1324 | + | CGGCGGUGUAGCCCACGCCC | 20 | 1710 |
| HSV1-UL19-334 | - | GGCGGCGUUCCGGGACGCCC | 20 | 720 |
| HSV1-UL19-351 | - | CGAGCACGUCUUCUGCGCCC | 20 | 737 |
| HSV1-UL19-517 | - | CCCCGUGUUCGGGUGCGCCC | 20 | 903 |
| HSV1-UL19-990 | + | CUCGGCGACCAGGGUCGCCC | 20 | 1376 |
| HSV1-UL19-1386 | + | GGCCGUGGCCGCGAAGGCCC | 20 | 1772 |
| HSV1-UL19-1056 | + | UGACCGGAUUGAACAGGCCC | 20 | 1442 |
| HSV1-UL19-1139 | + | CCACCCCCAGCUCCAGGCCC | 20 | 1525 |
| HSV1-UL19-369 | - | GGUGGCGCACGUCGAGGCCC | 20 | 755 |
| HSV1-UL19-253 | - | GCUGGUCUUUCUGGAGGCCC | 20 | 639 |
| HSV1-UL19-321 | - | GACGUUCCCCCGGCCGGCCC | 20 | 707 |
| HSV1-UL19-1338 | + | GAGGGGGAGCCCCGCGGCCC | 20 | 1724 |
| HSV1-UL19-383 | - | CCUGAUGCGGCGCGCGGCCC | 20 | 769 |
| HSV1-UL19-288 | - | CGGGGCGUACGUGGCGGCCC | 20 | 674 |
| HSV1-UL19-1277 | + | GUUCGCCACGUGGUCGGCCC | 20 | 1663 |
| HSV1-UL19-1316 | + | GGGCCACCUGGAGCUGGCCC | 20 | 1702 |
| HSV1-UL19-204 | - | CCUGGCCACCAGGGUGGCCC | 20 | 590 |
| HSV1-UL19-995 | + | GGCGGUGGCCCGCCUUGCCC | 20 | 1381 |
| HSV1-UL19-471 | - | UCAAGACGGGCCUCCAUCCC | 20 | 857 |
| HSV1-UL19-1304 | + | GUAGGUCAGCGCGUUCUCCC | 20 | 1690 |
| HSV1-UL19-543 | - | CGGCCAGAGCGACCCGUCCC | 20 | 929 |
| HSV1-UL19-1376 | + | CGCCCCCGGCGGCGCGUCCC | 20 | 1762 |
| HSV1-UL19-1060 | + | GGGGGCGGGGACUAGGUCCC | 20 | 1446 |
| HSV1-UL19-983 | + | GCAUCGGCAACAACAAAGCC | 20 | 1369 |
| HSV1-UL19-1117 | + | CGGGGUGCAGCUCAAGAGCC | 20 | 1503 |
| HSV1-UL19-1297 | + | AGUAGUUGGCCCCCAGAGCC | 20 | 1683 |
| HSV1-UL19-1159 | + | GAUGCACUGGACCACGAGCC | 20 | 1545 |
| HSV1-UL19-1000 | + | CGUGGUGAGGUCCACGAGCC | 20 | 1386 |
| HSV1-UL19-290 | - | AGCAGCUGUUUUUGAACGCC | 20 | 676 |
| HSV1-UL19-264 | - | AACGGUUUGCCGCGCACGCC | 20 | 650 |
| HSV1-UL19-333 | - | CGGCGGCGUUCCGGGACGCC | 20 | 719 |
| HSV1-UL19-511 | - | GGUCGUGGCGGGAAACCGCC | 20 | 897 |
| HSV1-UL19-1152 | + | GCAGCAGGUAAAACACCGCC | 20 | 1538 |
| HSV1-UL19-212 | - | GGGCAAGGCGGGCCACCGCC | 20 | 598 |
| HSV1-UL19-273 | - | UUUUUGGGGGAAGGACCGCC | 20 | 659 |
| HSV1-UL19-529 | - | ACCCCCGGGGACGCGCCGCC | 20 | 915 |
| HSV1-UL19-199 | - | AUACCUGGACAACGGCCGCC | 20 | 585 |
| HSV1-UL19-1182 | + | CGAGGGGUGGCAGCAGCGCC | 20 | 1568 |
| HSV1-UL19-294 | - | GAACGCCUGGGGGCAGCGCC | 20 | 680 |
| HSV1-UL19-1135 | + | ACGCCGCCGGACAUAGCGCC | 20 | 1521 |
| HSV1-UL19-1132 | + | GUUGCCGUUCACCACGCGCC | 20 | 1518 |
| HSV1-UL19-1226 | + | UCGCUGGGGCACUCCUCGCC | 20 | 1612 |
| HSV1-UL19-991 | + | GCUCGGCGACCAGGGUCGCC | 20 | 1377 |
| HSV1-UL19-447 | - | CCGUCCACGCGCUGUUCGCC | 20 | 833 |
| HSV1-UL19-1387 | + | UGGCCGUGGCCGCGAAGGCC | 20 | 1773 |
| HSV1-UL19-215 | - | GCCGGGAGGCCGUCGAGGCC | 20 | 601 |
| HSV1-UL19-523 | - | CGCAGGGAUGGACCACGGCC | 20 | 909 |
| HSV1-UL19-975 | + | CAAACGCCCCGAGGACGGCC | 20 | 1361 |
| HSV1-UL19-372 | - | UGACUUUACCCUGACCGGCC | 20 | 758 |
| HSV1-UL19-320 | - | GGACGUUCCCCCGGCCGGCC | 20 | 706 |
| HSV1-UL19-1125 | + | GGAUCUCCCCCGGGCCGGCC | 20 | 1511 |
| HSV1-UL19-1098 | + | UGUCUGCGGCGGGGCCGGCC | 20 | 1484 |
| HSV1-UL19-461 | - | ACGUCCGCGAGAGCGCGGCC | 20 | 847 |
| HSV1-UL19-1285 | + | GCGACAGGUCGCGCAGGGCC | 20 | 1671 |
| HSV1-UL19-1158 | + | ACUGGACCACGAGCCGGGCC | 20 | 1544 |
| HSV1-UL19-1203 | + | CGGCGUCCGCGGCUCGGGCC | 20 | 1589 |
| HSV1-UL19-336 | - | GUUCCGGGACGCCCGGGGCC | 20 | 722 |
| HSV1-UL19-1172 | + | AGUCAUCAACCAGCUGGGCC | 20 | 1558 |
| HSV1-UL19-1106 | + | AUCGGACCCGCCCGUGGGCC | 20 | 1492 |
| HSV1-UL19-352 | - | ACGUCUUCUGCGCCCUGGCC | 20 | 738 |
| HSV1-UL19-1130 | + | UCACCACGCGCCAGGUGGCC | 20 | 1516 |
| HSV1-UL19-482 | - | UGGGCCAGCUCCAGGUGGCC | 20 | 868 |
| HSV1-UL19-203 | - | GCCUGGCCACCAGGGUGGCC | 20 | 589 |
| HSV1-UL19-1067 | + | GCGCGGGGAUCCGGGUGGCC | 20 | 1453 |
| HSV1-UL19-987 | + | CCCGGGCCACCCUGGUGGCC | 20 | 1373 |
| HSV1-UL19-1241 | + | UGUUGGCCACCAGAUUGGCC | 20 | 1627 |
| HSV1-UL19-385 | - | GGACCGCCAUCGCGACUGCC | 20 | 771 |
| HSV1-UL19-312 | - | GUGGCCGACGUGGAGCUGCC | 20 | 698 |
| HSV1-UL19-559 | - | CGCCGCCAAACAUCGCUGCC | 20 | 945 |
| HSV1-UL19-1319 | + | CCCCCCGCCAGUUUCGUGCC | 20 | 1705 |
| HSV1-UL19-470 | - | CUCAAGACGGGCCUCCAUCC | 20 | 856 |
| HSV1-UL19-324 | - | GGCCGGCCCGGGGGAGAUCC | 20 | 710 |
| HSV1-UL19-1070 | + | GCGGGAAGGCGCGGGGAUCC | 20 | 1456 |
| HSV1-UL19-998 | + | GCCAGGCCUCGACGGCCUCC | 20 | 1384 |
| HSV1-UL19-1140 | + | GGCGUCCCACCCCCAGCUCC | 20 | 1526 |
| HSV1-UL19-1178 | + | GCGCCUGCCCGCCCAGCUCC | 20 | 1564 |
| HSV1-UL19-480 | - | GUACUUCCUGGGCCAGCUCC | 20 | 866 |
| HSV1-UL19-1405 | + | UCUCCACGAUCAGGCGCUCC | 20 | 1791 |
| HSV1-UL19-1112 | + | CGGGCUGCAUAAACUGCUCC | 20 | 1498 |
| HSV1-UL19-1222 | + | CCUCGCCGGGGGCGAUCUCC | 20 | 1608 |
| HSV1-UL19-504 | - | CCGCGGGGCUCCCCCUCUCC | 20 | 890 |
| HSV1-UL19-1041 | + | CGGCGUAGAUGCGCUUCUCC | 20 | 1427 |
| HSV1-UL19-3217 | - | ACAGCUCGCUCGCAACGUCC | 20 | 7816 |
| HSV1-UL19-542 | - | ACGGCCAGAGCGACCCGUCC | 20 | 928 |
| HSV1-UL19-1361 | + | UAAACUCACACACGGCGUCC | 20 | 1747 |
| HSV1-UL19-1137 | + | GCUCCAGGCCCCGGGCGUCC | 20 | 1523 |
| HSV1-UL19-1169 | + | CGACGUGCGCCACCAGGUCC | 20 | 1555 |
| HSV1-UL19-1191 | + | GGCAGUCGCGAUGGCGGUCC | 20 | 1577 |
| HSV1-UL19-417 | - | CCUGCAGAACAUGGUGGUCC | 20 | 803 |
| HSV1-UL19-985 | + | UGGCCAGGCGGCCGUUGUCC | 20 | 1371 |
| HSV1-UL19-1344 | + | ACACGGCUGCCGCGUUGUCC | 20 | 1730 |
| HSV1-UL19-478 | - | CGCGUCGGAGGCGUACUUCC | 20 | 864 |
| HSV1-UL19-332 | - | GCUAUGUCCGGCGGCGUUCC | 20 | 718 |
| HSV1-UL19-206 | - | CUGCGAGACGAGCUUUUUCC | 20 | 592 |
| HSV1-UL19-1401 | + | CGACGUAAAGAACUUAAAGC | 20 | 1787 |
| HSV1-UL19-250 | - | GGUGUCCAUCGGCGAGAAGC | 20 | 636 |
| HSV1-UL19-1081 | + | CCUUCCCCCAAAAAAACAGC | 20 | 1467 |
| HSV1-UL19-1174 | + | GGGUAAAGUCAUCAACCAGC | 20 | 1560 |
| HSV1-UL19-370 | - | CGUCGAGGCCCUGGCCCAGC | 20 | 756 |
| HSV1-UL19-1031 | + | GCUCCUCCUGCAUCUCCAGC | 20 | 1417 |
| HSV1-UL19-1201 | + | CCUGGGUGUUGUGCAGCAGC | 20 | 1587 |
| HSV1-UL19-1154 | + | CGUGUAUGGCGGCCUGCAGC | 20 | 1540 |
| HSV1-UL19-1260 | + | GAUGCUGGGGGGCCAUCAGC | 20 | 1646 |
| HSV1-UL19-1298 | + | AAGUAGUUGGCCCCCAGAGC | 20 | 1684 |
| HSV1-UL19-1393 | + | GGUAGGCCCCGUUAUAGAGC | 20 | 1779 |
| HSV1-UL19-1160 | + | UGAUGCACUGGACCACGAGC | 20 | 1546 |
| HSV1-UL19-373 | - | UACCCUGACCGGCCCGGAGC | 20 | 759 |
| HSV1-UL19-1317 | + | CGUGCCGGGCCACCUGGAGC | 20 | 1703 |
| HSV1-UL19-337 | - | GGACGCCCGGGGCCUGGAGC | 20 | 723 |
| HSV1-UL19-1422 | + | GGUC GCUGGC GCAGGUGAGC | 20 | 1808 |
| HSV1-UL19-1440 | + | ACAGAGCCAGUCCCUUGAGC | 20 | 1826 |
| HSV1-UL19-1162 | + | CCGCGUGUUGUUCCAGUAGC | 20 | 1548 |
| HSV1-UL19-1119 | + | CCCACAAAGAAGUCGAACGC | 20 | 1505 |
| HSV1-UL19-263 | - | GAACGGUUUGCCGCGCACGC | 20 | 649 |
| HSV1-UL19-442 | - | UUCGACGGGGCGUUGCACGC | 20 | 828 |
| HSV1-UL19-246 | - | CGACCUGAACCGGCAGACGC | 20 | 632 |
| HSV1-UL19-438 | - | UGCUCGGCGGCGCCCGACGC | 20 | 824 |
| HSV1-UL19-533 | - | GCCGCCGGGGGCGUUUACGC | 20 | 919 |
| HSV1-UL19-1153 | + | UGCAGCAGGUAAAACACCGC | 20 | 1539 |
| HSV1-UL19-211 | - | UGGGCAAGGCGGGCCACCGC | 20 | 597 |
| HSV1-UL19-412 | - | CGGGGCCGCUGCUGCACCGC | 20 | 798 |
| HSV1-UL19-1136 | + | CGGGCGUCCCGGAACGCCGC | 20 | 1522 |
| HSV1-UL19-528 | - | AACCCCCGGGGACGCGCCGC | 20 | 914 |
| HSV1-UL19-502 | - | AAAACUUUUACCUGGGCCGC | 20 | 888 |
| HSV1-UL19-524 | - | CCGACGUCAACUACUUCCGC | 20 | 910 |
| HSV1-UL19-1346 | + | CCGCCACGACCGCGUUCCGC | 20 | 1732 |
| HSV1-UL19-1183 | + | ACGAGGGGUGGCAGCAGCGC | 20 | 1569 |
| HSV1-UL19-387 | - | CGCGACUGCCGGGUUAGCGC | 20 | 773 |
| HSV1-UL19-220 | - | UGACGCAUGCCGACACGCGC | 20 | 606 |
| HSV1-UL19-578 | - | CAACGGGGAAGCCCACGCGC | 20 | 964 |
| HSV1-UL19-519 | - | GCCCAGGUGCCGCGGCGCGC | 20 | 905 |
| HSV1-UL19-1288 | + | CCUGCCGCGACAGGUCGCGC | 20 | 1674 |
| HSV1-UL19-1073 | + | CUGGCGGGGCGGGAAGGCGC | 20 | 1459 |
| HSV1-UL19-1424 | + | GAGGGCGGGGUCGCUGGCGC | 20 | 1810 |
| HSV1-UL19-1235 | + | GCCGGGUGCAGGGGGUGCGC | 20 | 1621 |
| HSV1-UL19-357 | - | GUUCGUGAACGACUACUCGC | 20 | 743 |
| HSV1-UL19-557 | - | CCACCUCAACGGGGCCUCGC | 20 | 943 |
| HSV1-UL19-1227 | + | GUCGCUGGGGCACUCCUCGC | 20 | 1613 |
| HSV1-UL19-409 | - | GAUGGUGCCCGCCUUCUCGC | 20 | 795 |
| HSV1-UL19-1411 | + | GCGCUUAAACUGUACGUCGC | 20 | 1797 |
| HSV1-UL19-1230 | + | GGGGUCCGUCACGGGGUCGC | 20 | 1616 |
| HSV1-UL19-1425 | + | GCGGAGGAGGGCGGGGUCGC | 20 | 1811 |
| HSV1-UL19-345 | - | CCACCAUCGCCGCCGUUCGC | 20 | 731 |
| HSV1-UL19-446 | - | CCCGUCCACGCGCUGUUCGC | 20 | 832 |
| HSV1-UL19-210 | - | AGCUUUUUCCUGGGCAAGGC | 20 | 596 |
| HSV1-UL19-1213 | + | CAGCGGCCCCGCGAGAAGGC | 20 | 1599 |
| HSV1-UL19-319 | - | GGGGGGGACGUUCCCCCGGC | 20 | 705 |
| HSV1-UL19-289 | - | GCGUACGUGGCGGCCCCGGC | 20 | 675 |
| HSV1-UL19-1126 | + | UGGAUCUCCCCCGGGCCGGC | 20 | 1512 |
| HSV1-UL19-1099 | + | AUGUCUGCGGCGGGGCCGGC | 20 | 1485 |
| HSV1-UL19-460 | - | CACGUCCGCGAGAGCGCGGC | 20 | 846 |
| HSV1-UL19-1322 | + | GGUCCACGUUCGCGCGCGGC | 20 | 1708 |
| HSV1-UL19-452 | - | CCUGCGCGACCUGUCGCGGC | 20 | 838 |
| HSV1-UL19-222 | - | UGCCGACACGCGCGGGCGGC | 20 | 608 |
| HSV1-UL19-1102 | + | AGCUGCUGCAUGUCUGCGGC | 20 | 1488 |
| HSV1-UL19-1208 | + | AGUCCGCCCCCCGGUGCGGC | 20 | 1594 |
| HSV1-UL19-1286 | + | CGCGACAGGUCGCGCAGGGC | 20 | 1672 |
| HSV1-UL19-1370 | + | GUCCCCGGGGGUUGCAGGGC | 20 | 1756 |
| HSV1-UL19-1427 | + | CGGGCACUGCGGAGGAGGGC | 20 | 1813 |
| HSV1-UL19-298 | - | GCAGCGCCUGGCCCACGGGC | 20 | 684 |
| HSV1-UL19-1351 | + | ACCCGAACACGGGGACGGGC | 20 | 1737 |
| HSV1-UL19-1127 | + | GGCCUGGAUCUCCCCCGGGC | 20 | 1513 |
| HSV1-UL19-1177 | + | CUGCCCGCCCAGCUCCGGGC | 20 | 1563 |
| HSV1-UL19-377 | - | CGGCCCGGAGCUGGGCGGGC | 20 | 763 |
| HSV1-UL19-1053 | + | GAUUGAACAGGCCCAGGGGC | 20 | 1439 |
| HSV1-UL19-1100 | + | CUGCAUGUCUGCGGCGGGGC | 20 | 1486 |
| HSV1-UL19-1076 | + | AAAAAAACAGCUGGCGGGGC | 20 | 1462 |
| HSV1-UL19-1063 | + | GGAUCCGGGUGGCCGGGGGC | 20 | 1449 |
| HSV1-UL19-514 | - | GGCGGGAAACCGCCUGGGGC | 20 | 900 |
| HSV1-UL19-376 | - | UGACCGGCCCGGAGCUGGGC | 20 | 762 |
| HSV1-UL19-1349 | + | AACACGGGGACGGGCUGGGC | 20 | 1735 |
| HSV1-UL19-1246 | + | ACCUGCAGCGUGAGCAUGGC | 20 | 1632 |
| HSV1-UL19-465 | - | CUGACCUACGCGCUCAUGGC | 20 | 851 |
| HSV1-UL19-485 | - | UGGCCCGGCACGAAACUGGC | 20 | 871 |
| HSV1-UL19-1079 | + | CCCCCAAAAAAACAGCUGGC | 20 | 1465 |
| HSV1-UL19-510 | - | CUGCGGAACGCGGUCGUGGC | 20 | 896 |
| HSV1-UL19-1068 | + | GGCGCGGGGAUCCGGGUGGC | 20 | 1454 |
| HSV1-UL19-1145 | + | CGAACGGCGGCGAUGGUGGC | 20 | 1531 |
| HSV1-UL19-1242 | + | GUGUUGGCCACCAGAUUGGC | 20 | 1628 |
| HSV1-UL19-1265 | + | GGAUGGUAUGGUCCAGAUGC | 20 | 1651 |
| HSV1-UL19-243 | - | CCGCCACCUGCUGGAGAUGC | 20 | 629 |
| HSV1-UL19-384 | - | UGGACCGCCAUCGCGACUGC | 20 | 770 |
| HSV1-UL19-1019 | + | CCACUUUCAGGAAGGACUGC | 20 | 1405 |
| HSV1-UL19-242 | - | CGACGUGGGCCGCCACCUGC | 20 | 628 |
| HSV1-UL19-311 | - | GGUGGCCGACGUGGAGCUGC | 20 | 697 |
| HSV1-UL19-431 | - | GCCCGCCAUGCUCACGCUGC | 20 | 817 |
| HSV1-UL19-349 | - | GGCGGUGUUUUACCUGCUGC | 20 | 735 |
| HSV1-UL19-194 | - | CCAGAUGCUGCACGUGCUGC | 20 | 580 |
| HSV1-UL19-1033 | + | CGAGCUCGUCCAGCGUCUGC | 20 | 1419 |
| HSV1-UL19-1221 | + | CCGGGACCACCAUGUUCUGC | 20 | 1607 |
| HSV1-UL19-228 | - | GGACACCGAAGCCGACGUGC | 20 | 614 |
| HSV1-UL19-1320 | + | CCCCCCCGCCAGUUUCGUGC | 20 | 1706 |
| HSV1-UL19-432 | - | CAUGCUCACGCUGCAGGUGC | 20 | 818 |
| HSV1-UL19-1240 | + | CCACCAGAUUGGCCGGGUGC | 20 | 1626 |
| HSV1-UL19-1433 | + | CGCGUGGGCUUCCCCGUUGC | 20 | 1819 |
| HSV1-UL19-1372 | + | GGCGCGUCCCCGGGGGUUGC | 20 | 1758 |
| HSV1-UL19-422 | - | CCCCCUGCACCCGGCCAAUC | 20 | 808 |
| HSV1-UL19-260 | - | GCCCCUGGGCCUGUUCAAUC | 20 | 646 |
| HSV1-UL19-444 | - | GCUGAUGGCCCCCCAGCAUC | 20 | 830 |
| HSV1-UL19-979 | + | CCAGCAGCACGUGCAGCAUC | 20 | 1365 |
| HSV1-UL19-1189 | + | CCAGGGCCGCGCGCCGCAUC | 20 | 1575 |
| HSV1-UL19-1110 | + | UAAACUGCUCCGGGGUCAUC | 20 | 1496 |
| HSV1-UL19-1406 | + | CCGAACCCGUCUCCACGAUC | 20 | 1792 |
| HSV1-UL19-276 | - | UGUCUCUGGAACACGCGAUC | 20 | 662 |
| HSV1-UL19-248 | - | GACGCGCGUGCGCGCGGAUC | 20 | 634 |
| HSV1-UL19-1071 | + | GGCGGGAAGGCGCGGGGAUC | 20 | 1457 |
| HSV1-UL19-1016 | + | GCAGGAGGCGCUGUUUGAUC | 20 | 1402 |
| HSV1-UL19-359 | - | CGUACGUCGUGACCUACCUC | 20 | 745 |
| HSV1-UL19-1166 | + | UACACGGCCAUGCACUCCUC | 20 | 1552 |
| HSV1-UL19-190 | - | GCAACGUCCAGGCCGUCCUC | 20 | 576 |
| HSV1-UL19-1179 | + | UGCGCCUGCCCGCCCAGCUC | 20 | 1565 |
| HSV1-UL19-1383 | + | AGGCCCGGGACGGGUCGCUC | 20 | 1769 |
| HSV1-UL19-455 | - | CCCCCUGGUCCCCCCGGCUC | 20 | 841 |
| HSV1-UL19-1204 | + | AUCCGCGGCGUCCGCGGCUC | 20 | 1590 |
| HSV1-UL19-1113 | + | UCGGGCUGCAUAAACUGCUC | 20 | 1499 |
| HSV1-UL19-1223 | + | UCCUCGCCGGGGGCGAUCUC | 20 | 1609 |
| HSV1-UL19-274 | - | CCAGGUGCUGCGCCUGUCUC | 20 | 660 |
| HSV1-UL19-239 | - | GGGCAAGGCCGUGCGAAGUC | 20 | 625 |
| HSV1-UL19-1052 | + | CCAGGGGCAGGACGAACGUC | 20 | 1438 |
| HSV1-UL19-1252 | + | GCGGUGUUGGCGCCCGCGUC | 20 | 1638 |
| HSV1-UL19-1038 | + | CCGCGCGCACGCGCGUCGUC | 20 | 1424 |
| HSV1-UL19-1176 | + | CGCCCAGCUCCGGGCCGGUC | 20 | 1562 |
| HSV1-UL19-280 | - | UGAACGUUGACGCGGCGGUC | 20 | 666 |
| HSV1-UL19-329 | - | CUUGCCCCUGGCGCUAUGUC | 20 | 715 |
| HSV1-UL19-1114 | + | AGAGC CAGGUUGGC GUUGUC | 20 | 1500 |
| HSV1-UL19-1259 | + | GGGGGCCAUCAGCAGGAUUC | 20 | 1645 |
| HSV1-UL19-1025 | + | UGACCAGGUUGGCCCCGUUC | 20 | 1411 |
| HSV1-UL19-331 | - | CGCUAUGUCCGGCGGCGUUC | 20 | 717 |
| HSV1-UL19-1032 | + | CGUCCAGCGUCUGCCGGUUC | 20 | 1418 |
| HSV1-UL19-516 | - | CCCAGCCCGUCCCCGUGUUC | 20 | 902 |
| HSV1-UL19-1021 | + | CUUCGGUGUCCUCCACUUUC | 20 | 1407 |
| HSV1-UL19-251 | - | CGGCGAGAAGCUGGUCUUUC | 20 | 637 |
| HSV1-UL19-572 | - | GGACCCGUGUGGCCUGUUUC | 20 | 958 |
| HSV1-UL19-1315 | + | GCACGUUCUCAGUCACAAAG | 20 | 1701 |
| HSV1-UL19-226 | - | CCUGCAGUCCUUCCUGAAAG | 20 | 612 |
| HSV1-UL19-1438 | + | AGACGAGAUACUGCGCGAAG | 20 | 1824 |
| HSV1-UL19-1048 | + | AGGUCCAUGGCGCCCACCAG | 20 | 1434 |
| HSV1-UL19-1291 | + | CCCAGAGCCGGGGGGACCAG | 20 | 1677 |
| HSV1-UL19-1054 | + | ACCGGAUUGAACAGGCCCAG | 20 | 1440 |
| HSV1-UL19-981 | + | AUCGGCAACAACAAAGCCAG | 20 | 1367 |
| HSV1-UL19-1133 | + | GCCGCCGGACAUAGCGCCAG | 20 | 1519 |
| HSV1-UL19-1399 | + | ACGUAAAGAACUUAAAGCAG | 20 | 1785 |
| HSV1-UL19-1216 | + | GAACCCCCGCGGUGCAGCAG | 20 | 1602 |
| HSV1-UL19-1238 | + | ACCAGAUUGGCCGGGUGCAG | 20 | 1624 |
| HSV1-UL19-1341 | + | UGCCGCGUUGUCCAGGAGAG | 20 | 1727 |
| HSV1-UL19-1185 | + | GCGUCACAGUCCCACACGAG | 20 | 1571 |
| HSV1-UL19-539 | - | UUUACGCGGGGGACAAGGAG | 20 | 925 |
| HSV1-UL19-1343 | + | GCUGCCGCGUUGUCCAGGAG | 20 | 1729 |
| HSV1-UL19-1270 | + | AACAGCGCGUGGACGGGGAG | 20 | 1656 |
| HSV1-UL19-1423 | + | GGGUC GCUGGC GCAGGUGAG | 20 | 1809 |
| HSV1-UL19-1441 | + | UACAGAGCCAGUCCCUUGAG | 20 | 1827 |
| HSV1-UL19-428 | - | UCACAACGGGCGCGUGGUAG | 20 | 814 |
| HSV1-UL19-505 | - | UCCCCCUCUCCUGGACAACG | 20 | 891 |
| HSV1-UL19-576 | - | CCUCCGCAGUGCCCGCAACG | 20 | 962 |
| HSV1-UL19-556 | - | CGGGGCCUACCACCUCAACG | 20 | 942 |
| HSV1-UL19-490 | - | CACCCAGCCGCGCGCGAACG | 20 | 876 |
| HSV1-UL19-1120 | + | CCCCACAAAGAAGUCGAACG | 20 | 1506 |
| HSV1-UL19-507 | - | AGCCGUGUACCUGCGGAACG | 20 | 893 |
| HSV1-UL19-233 | - | CGGCGAGAUGGUCCUGAACG | 20 | 619 |
| HSV1-UL19-553 | - | CGGGGACCUGCUCUAUAACG | 20 | 939 |
| HSV1-UL19-393 | - | GUACGCGGCGGCAUGUAACG | 20 | 779 |
| HSV1-UL19-355 | - | CCAGCUACUGGAACAACACG | 20 | 741 |
| HSV1-UL19-1354 | + | CCUGGGCGCACCCGAACACG | 20 | 1740 |
| HSV1-UL19-1187 | + | GGGCGUCACAGUCCCACACG | 20 | 1573 |
| HSV1-UL19-1365 | + | AGUUGACGUCGGUCGACACG | 20 | 1751 |
| HSV1-UL19-1196 | + | UACAUGCCGCCGCGUACACG | 20 | 1582 |
| HSV1-UL19-449 | - | GUUCGCCGGGGCCGACCACG | 20 | 835 |
| HSV1-UL19-1279 | + | AAUUGGGCGCGUUCGCCACG | 20 | 1665 |
| HSV1-UL19-1231 | + | GGUGCGCGGGGUCCGUCACG | 20 | 1617 |
| HSV1-UL19-1008 | + | GUGUCGGCAUGCGUCAGACG | 20 | 1394 |
| HSV1-UL19-240 | - | CGUGCGAAGUCUGGACGACG | 20 | 626 |
| HSV1-UL19-437 | - | CUGCUCGGCGGCGCCCGACG | 20 | 823 |
| HSV1-UL19-310 | - | CUUUGUGGGGGUGGCCGACG | 20 | 696 |
| HSV1-UL19-225 | - | GCGCGGGCGGCCGGUCGACG | 20 | 611 |
| HSV1-UL19-441 | - | CAACAUGCGCAUAUUCGACG | 20 | 827 |
| HSV1-UL19-1091 | + | ACGGAUUGGCGGCUUCGACG | 20 | 1477 |
| HSV1-UL19-1273 | + | CGGCGAACAGCGCGUGGACG | 20 | 1659 |
| HSV1-UL19-1255 | + | GCGCAUGUUGGUGGUGGACG | 20 | 1641 |
| HSV1-UL19-1330 | + | GGUUGCCCAUGUCGGUGACG | 20 | 1716 |
| HSV1-UL19-277 | - | UUCGCUGAUGAACGUUGACG | 20 | 663 |
| HSV1-UL19-1218 | + | UCUGCAGGGUGGCGUAUACG | 20 | 1604 |
| HSV1-UL19-285 | - | CGAAGCCGCCAAUCCGUACG | 20 | 671 |
| HSV1-UL19-550 | - | GUCGCAGCGAUUUUCGUACG | 20 | 936 |
| HSV1-UL19-391 | - | GGGCCACGACCCCGUGUACG | 20 | 777 |
| HSV1-UL19-532 | - | CGCCGCCGGGGGCGUUUACG | 20 | 918 |
| HSV1-UL19-402 | - | GCGGAUGACCGGCCGCACCG | 20 | 788 |
| HSV1-UL19-411 | - | GCGGGGCCGCUGCUGCACCG | 20 | 797 |
| HSV1-UL19-394 | - | GGCAUGUAACGUGGCGACCG | 20 | 780 |
| HSV1-UL19-546 | - | UCGCGGCCACGGCCAACCCG | 20 | 932 |
| HSV1-UL19-1414 | + | CACGAGUUCGCGGCACCCCG | 20 | 1800 |
| HSV1-UL19-527 | - | CGCCGGCCCUGCAACCCCCG | 20 | 913 |
| HSV1-UL19-1217 | + | GCGGUCGAAGCGAACCCCCG | 20 | 1603 |
| HSV1-UL19-568 | - | UACAGUUUAAGCGCCCCCCG | 20 | 954 |
| HSV1-UL19-1164 | + | CCUCGGGAAGGUCUCCCCCG | 20 | 1550 |
| HSV1-UL19-1339 | + | CCAGGAGAGGGGGAGCCCCG | 20 | 1725 |
| HSV1-UL19-977 | + | UGCCGCGCUCAAACGCCCCG | 20 | 1363 |
| HSV1-UL19-1374 | + | CCCCCGGCGGCGCGUCCCCG | 20 | 1760 |
| HSV1-UL19-335 | - | GCGGCGUUCCGGGACGCCCG | 20 | 721 |
| HSV1-UL19-1108 | + | CGACCCAUCGGACCCGCCCG | 20 | 1494 |
| HSV1-UL19-322 | - | ACGUUCCCCCGGCCGGCCCG | 20 | 708 |
| HSV1-UL19-467 | - | GUACUUCAAGAUCAGUCCCG | 20 | 853 |
| HSV1-UL19-362 | - | CCUCGGGGGAGACCUUCCCG | 20 | 748 |
| HSV1-UL19-1296 | + | GUAGUUGGCCCCCAGAGCCG | 20 | 1682 |
| HSV1-UL19-397 | - | CAACACCCAGGCCCGAGCCG | 20 | 783 |
| HSV1-UL19-265 | - | ACGGUUUGCCGCGCACGCCG | 20 | 651 |
| HSV1-UL19-398 | - | GGCCCGAGCCGCGGACGCCG | 20 | 784 |
| HSV1-UL19-530 | - | CCCCCGGGGACGCGCCGCCG | 20 | 916 |
| HSV1-UL19-1359 | + | UGGUCCAUCCCUGCGCGCCG | 20 | 1745 |
| HSV1-UL19-1225 | + | CGCUGGGGCACUCCUCGCCG | 20 | 1611 |
| HSV1-UL19-448 | - | CGUCCACGCGCUGUUCGCCG | 20 | 834 |
| HSV1-UL19-1124 | + | GAUCUCCCCCGGGCCGGCCG | 20 | 1510 |
| HSV1-UL19-1097 | + | GUCUGCGGCGGGGCCGGCCG | 20 | 1483 |
| HSV1-UL19-462 | - | CGUCCGCGAGAGCGCGGCCG | 20 | 848 |
| HSV1-UL19-1284 | + | CGACAGGUCGCGCAGGGCCG | 20 | 1670 |
| HSV1-UL19-501 | - | CAAAACUUUUACCUGGGCCG | 20 | 887 |
| HSV1-UL19-1360 | + | CACACACGGCGUCCUGGCCG | 20 | 1746 |
| HSV1-UL19-1382 | + | GGGACGGGUCGCUCUGGCCG | 20 | 1768 |
| HSV1-UL19-1002 | + | CACGGAGGGCUGCGUGGCCG | 20 | 1388 |
| HSV1-UL19-1407 | + | GUCGCUGGCGGCGGUGGCCG | 20 | 1793 |
| HSV1-UL19-1066 | + | CGCGGGGAUCCGGGUGGCCG | 20 | 1452 |
| HSV1-UL19-1389 | + | CGACGCCCACGGGUUGGCCG | 20 | 1775 |
| HSV1-UL19-313 | - | UGGCCGACGUGGAGCUGCCG | 20 | 699 |
| HSV1-UL19-518 | - | UCGGGUGCGCCCAGGUGCCG | 20 | 904 |
| HSV1-UL19-1327 | + | GGGGUUGCGGACGGUUGCCG | 20 | 1713 |
| HSV1-UL19-1207 | + | CCGGUGCGGCCGGUCAUCCG | 20 | 1593 |
| HSV1-UL19-218 | - | CACGGCCACGCAGCCCUCCG | 20 | 604 |
| HSV1-UL19-1111 | + | GGGCUGCAUAAACUGCUCCG | 20 | 1497 |
| HSV1-UL19-1199 | + | GUCGUUGCGGUUGAAGUCCG | 20 | 1585 |
| HSV1-UL19-1206 | + | CCGGUCAUCCGCGGCGUCCG | 20 | 1592 |
| HSV1-UL19-459 | - | GCAGCACGUCCGCGAGAGCG | 20 | 845 |
| HSV1-UL19-1439 | + | CAGAGCCAGUCCCUUGAGCG | 20 | 1825 |
| HSV1-UL19-192 | - | GUCCUCGGGGCGUUUGAGCG | 20 | 578 |
| HSV1-UL19-386 | - | UCGCGACUGCCGGGUUAGCG | 20 | 772 |
| HSV1-UL19-1118 | + | CCACAAAGAAGUCGAACGCG | 20 | 1504 |
| HSV1-UL19-219 | - | CUGACGCAUGCCGACACGCG | 20 | 605 |
| HSV1-UL19-577 | - | GCAACGGGGAAGCCCACGCG | 20 | 963 |
| HSV1-UL19-534 | - | CCGCCGGGGGCGUUUACGCG | 20 | 920 |
| HSV1-UL19-413 | - | GGGGCCGCUGCUGCACCGCG | 20 | 799 |
| HSV1-UL19-503 | - | AAACUUUUACCUGGGCCGCG | 20 | 889 |
| HSV1-UL19-1276 | + | CGGCCCCGGCGAACAGCGCG | 20 | 1662 |
| HSV1-UL19-388 | - | GCGACUGCCGGGUUAGCGCG | 20 | 774 |
| HSV1-UL19-1436 | + | CGAAGUGGGUCUCCCGCGCG | 20 | 1822 |
| HSV1-UL19-1323 | + | CCCAGGUCCACGUUCGCGCG | 20 | 1709 |
| HSV1-UL19-1072 | + | UGGCGGGGCGGGAAGGCGCG | 20 | 1458 |
| HSV1-UL19-382 | - | UGACGCCCUGAUGCGGCGCG | 20 | 768 |
| HSV1-UL19-427 | - | CAUGUUUCACAACGGGCGCG | 20 | 813 |
| HSV1-UL19-326 | - | GAUCCAGGCCACCUGGCGCG | 20 | 712 |
| HSV1-UL19-247 | - | CCAGACGACGCGCGUGCGCG | 20 | 633 |
| HSV1-UL19-1234 | + | CCGGGUGCAGGGGGUGCGCG | 20 | 1620 |
| HSV1-UL19-410 | - | AUGGUGCCCGCCUUCUCGCG | 20 | 796 |
| HSV1-UL19-346 | - | CACCAUCGCCGCCGUUCGCG | 20 | 732 |
| HSV1-UL19-1074 | + | GCUGGCGGGGCGGGAAGGCG | 20 | 1460 |
| HSV1-UL19-1397 | + | GCAGGGGCUGAGCACCGGCG | 20 | 1783 |
| HSV1-UL19-419 | - | CCCGGAGAUCGCCCCCGGCG | 20 | 805 |
| HSV1-UL19-1101 | + | GCUGCUGCAUGUCUGCGGCG | 20 | 1487 |
| HSV1-UL19-3234 | + | CACUACCCCAAGGAAGGGCG | 20 | 7833 |
| HSV1-UL19-1426 | + | GGGCACUGCGGAGGAGGGCG | 20 | 1812 |
| HSV1-UL19-1062 | + | GAUCCGGGUGGCCGGGGGCG | 20 | 1448 |
| HSV1-UL19-493 | - | CGCGAACGUGGACCUGGGCG | 20 | 879 |
| HSV1-UL19-1141 | + | UGGUGGCGGGGGCCAUGGCG | 20 | 1527 |
| HSV1-UL19-466 | - | UGACCUACGCGCUCAUGGCG | 20 | 852 |
| HSV1-UL19-486 | - | GGCCCGGCACGAAACUGGCG | 20 | 872 |
| HSV1-UL19-1078 | + | CCCCAAAAAAACAGCUGGCG | 20 | 1464 |
| HSV1-UL19-1144 | + | GAACGGCGGCGAUGGUGGCG | 20 | 1530 |
| HSV1-UL19-1003 | + | CACGGCCACGGAGGGCUGCG | 20 | 1389 |
| HSV1-UL19-1059 | + | GACUAGGUCCCCGGCGUGCG | 20 | 1445 |
| HSV1-UL19-356 | - | CUGGAACAACACGCGGUGCG | 20 | 742 |
| HSV1-UL19-1236 | + | GGCCGGGUGCAGGGGGUGCG | 20 | 1622 |
| HSV1-UL19-560 | - | CUGCCUGGAGCGCCUGAUCG | 20 | 946 |
| HSV1-UL19-1015 | + | CAGGAGGCGCUGUUUGAUCG | 20 | 1401 |
| HSV1-UL19-569 | - | CCCGGGGUGCCGCGAACUCG | 20 | 955 |
| HSV1-UL19-360 | - | GUACGUCGUGACCUACCUCG | 20 | 746 |
| HSV1-UL19-191 | - | CAACGUCCAGGCCGUCCUCG | 20 | 577 |
| HSV1-UL19-353 | - | CUGCGCCCUGGCCCGGCUCG | 20 | 739 |
| HSV1-UL19-216 | - | GGCCGUCGAGGCCUGGCUCG | 20 | 602 |
| HSV1-UL19-1303 | + | UCUCCCCGGCCGCGCUCUCG | 20 | 1689 |
| HSV1-UL19-408 | - | UGAUGGUGCCCGCCUUCUCG | 20 | 794 |
| HSV1-UL19-368 | - | GGACCUGGUGGCGCACGUCG | 20 | 754 |
| HSV1-UL19-495 | - | CGUGGGCUACACCGCCGUCG | 20 | 881 |
| HSV1-UL19-214 | - | CCACCGCCGGGAGGCCGUCG | 20 | 600 |
| HSV1-UL19-508 | - | GUACCUGCGGAACGCGGUCG | 20 | 894 |
| HSV1-UL19-281 | - | GAACGUUGACGCGGCGGUCG | 20 | 667 |
| HSV1-UL19-1195 | + | CCGCCGCGUACACGGGGUCG | 20 | 1581 |
| HSV1-UL19-1090 | + | UGGCGGCUUCGACGGGGUCG | 20 | 1476 |
| HSV1-UL19-301 | - | CGGGCGGGUCCGAUGGGUCG | 20 | 687 |
| HSV1-UL19-451 | - | CGGCCCUGCGCGACCUGUCG | 20 | 837 |
| HSV1-UL19-544 | - | CGACCCGUCCCGGGCCUUCG | 20 | 930 |
| HSV1-UL19-1417 | + | ACGGGUCCUCCACGAGUUCG | 20 | 1803 |
| HSV1-UL19-344 | - | GCCACCAUCGCCGCCGUUCG | 20 | 730 |
| HSV1-UL19-537 | - | CGUUUACGCGGGGGACAAGG | 20 | 923 |
| HSV1-UL19-209 | - | GAGCUUUUUCCUGGGCAAGG | 20 | 595 |
| HSV1-UL19-1214 | + | GCAGCGGCCCCGCGAGAAGG | 20 | 1600 |
| HSV1-UL19-1047 | + | GGUCCAUGGCGCCCACCAGG | 20 | 1433 |
| HSV1-UL19-1290 | + | CCAGAGCCGGGGGGACCAGG | 20 | 1676 |
| HSV1-UL19-1347 | + | CGGGCUGGGCCGGCCCCAGG | 20 | 1733 |
| HSV1-UL19-1131 | + | GCCGUUCACCACGCGCCAGG | 20 | 1517 |
| HSV1-UL19-986 | + | GGGCCACCCUGGUGGCCAGG | 20 | 1372 |
| HSV1-UL19-481 | - | CUUCCUGGGCCAGCUCCAGG | 20 | 867 |
| HSV1-UL19-1030 | + | CCUCCUGCAUCUCCAGCAGG | 20 | 1416 |
| HSV1-UL19-244 | - | CCACCUGCUGGAGAUGCAGG | 20 | 630 |
| HSV1-UL19-1018 | + | CUUUCAGGAAGGACUGCAGG | 20 | 1404 |
| HSV1-UL19-1237 | + | CCAGAUUGGCCGGGUGCAGG | 20 | 1623 |
| HSV1-UL19-573 | - | CCCGUGUGGCCUGUUUCAGG | 20 | 959 |
| HSV1-UL19-1340 | + | GCCGCGUUGUCCAGGAGAGG | 20 | 1726 |
| HSV1-UL19-540 | - | UUACGCGGGGGACAAGGAGG | 20 | 926 |
| HSV1-UL19-1430 | + | CGUUGCGGGCACUGCGGAGG | 20 | 1816 |
| HSV1-UL19-1269 | + | ACAGCGCGUGGACGGGGAGG | 20 | 1655 |
| HSV1-UL19-1395 | + | CCGGCGAGGCCCCGUUGAGG | 20 | 1781 |
| HSV1-UL19-1149 | + | GUCGAACGCCCCGCGAACGG | 20 | 1535 |
| HSV1-UL19-1364 | + | GUUGACGUCGGUCGACACGG | 20 | 1750 |
| HSV1-UL19-1005 | + | CAGACGGGGCACGGCCACGG | 20 | 1391 |
| HSV1-UL19-1325 | + | GGUUGCCGUGGCCACGACGG | 20 | 1711 |
| HSV1-UL19-403 | - | CGGAUGACCGGCCGCACCGG | 20 | 789 |
| HSV1-UL19-348 | - | CGACGACCGCAACUACCCGG | 20 | 734 |
| HSV1-UL19-1413 | + | ACGAGUUCGCGGCACCCCGG | 20 | 1799 |
| HSV1-UL19-1377 | + | CCCCGCGUAAACGCCCCCGG | 20 | 1763 |
| HSV1-UL19-1373 | + | CCCCGGCGGCGCGUCCCCGG | 20 | 1759 |
| HSV1-UL19-323 | - | CGUUCCCCCGGCCGGCCCGG | 20 | 709 |
| HSV1-UL19-997 | + | AGGCCUCGACGGCCUCCCGG | 20 | 1383 |
| HSV1-UL19-1295 | + | UAGUUGGCCCCCAGAGCCGG | 20 | 1681 |
| HSV1-UL19-531 | - | CCCCGGGGACGCGCCGCCGG | 20 | 917 |
| HSV1-UL19-1224 | + | GCUGGGGCACUCCUCGCCGG | 20 | 1610 |
| HSV1-UL19-1123 | + | AUCUCCCCCGGGCCGGCCGG | 20 | 1509 |
| HSV1-UL19-1283 | + | GACAGGUCGCGCAGGGCCGG | 20 | 1669 |
| HSV1-UL19-1369 | + | CCCGGGGGUUGCAGGGCCGG | 20 | 1755 |
| HSV1-UL19-1065 | + | GCGGGGAUCCGGGUGGCCGG | 20 | 1451 |
| HSV1-UL19-314 | - | GGCCGACGUGGAGCUGCCGG | 20 | 700 |
| HSV1-UL19-330 | - | GCCCCUGGCGCUAUGUCCGG | 20 | 716 |
| HSV1-UL19-278 | - | GCUGAUGAACGUUGACGCGG | 20 | 664 |
| HSV1-UL19-392 | - | CCACGACCCCGUGUACGCGG | 20 | 778 |
| HSV1-UL19-535 | - | CGCCGGGGGCGUUUACGCGG | 20 | 921 |
| HSV1-UL19-414 | - | GGGCCGCUGCUGCACCGCGG | 20 | 800 |
| HSV1-UL19-389 | - | CGACUGCCGGGUUAGCGCGG | 20 | 775 |
| HSV1-UL19-996 | + | CCUCGACGGCCUCCCGGCGG | 20 | 1382 |
| HSV1-UL19-1408 | + | CUGUACGUCGCUGGCGGCGG | 20 | 1794 |
| HSV1-UL19-1013 | + | GCGCUGUUUGAUCGGGGCGG | 20 | 1399 |
| HSV1-UL19-487 | - | GCCCGGCACGAAACUGGCGG | 20 | 873 |
| HSV1-UL19-1409 | + | AAACUGUACGUCGCUGGCGG | 20 | 1795 |
| HSV1-UL19-1143 | + | AACGGCGGCGAUGGUGGCGG | 20 | 1529 |
| HSV1-UL19-1431 | + | CCCCGUUGCGGGCACUGCGG | 20 | 1817 |
| HSV1-UL19-1103 | + | CAGCUGCUGCAUGUCUGCGG | 20 | 1489 |
| HSV1-UL19-361 | - | UACGUCGUGACCUACCUCGG | 20 | 747 |
| HSV1-UL19-436 | - | AACGGCGCUGCUCUGCUCGG | 20 | 822 |
| HSV1-UL19-477 | - | GUUCUCGGAGCGCGCGUCGG | 20 | 863 |
| HSV1-UL19-282 | - | AACGUUGACGCGGCGGUCGG | 20 | 668 |
| HSV1-UL19-564 | - | GAUC GUGGAGACGGGUUC GG | 20 | 950 |
| HSV1-UL19-1086 | + | CAACGUUCAUCAGCGAAGGG | 20 | 1472 |
| HSV1-UL19-202 | - | CGGCCGCCUGGCCACCAGGG | 20 | 588 |
| HSV1-UL19-1219 | + | GACCACCAUGUUCUGCAGGG | 20 | 1605 |
| HSV1-UL19-1428 | + | GCGGGCACUGCGGAGGAGGG | 20 | 1814 |
| HSV1-UL19-1379 | + | GCCGUGGUCGUACAUGAGGG | 20 | 1765 |
| HSV1-UL19-297 | - | GGCAGCGCCUGGCCCACGGG | 20 | 683 |
| HSV1-UL19-404 | - | GGAUGACCGGCCGCACCGGG | 20 | 790 |
| HSV1-UL19-1412 | + | CGAGUUCGCGGCACCCCGGG | 20 | 1798 |
| HSV1-UL19-1294 | + | AGUUGGCCCCCAGAGCCGGG | 20 | 1680 |
| HSV1-UL19-213 | - | CAAGGCGGGCCACCGCCGGG | 20 | 599 |
| HSV1-UL19-1282 | + | ACAGGUCGCGCAGGGCCGGG | 20 | 1668 |
| HSV1-UL19-315 | - | GCCGACGUGGAGCUGCCGGG | 20 | 701 |
| HSV1-UL19-1069 | + | GGAAGGCGCGGGGAUCCGGG | 20 | 1455 |
| HSV1-UL19-390 | - | GACUGCCGGGUUAGCGCGGG | 20 | 776 |
| HSV1-UL19-221 | - | CGCAUGCCGACACGCGCGGG | 20 | 607 |
| HSV1-UL19-488 | - | CCCGGCACGAAACUGGCGGG | 20 | 874 |
| HSV1-UL19-980 | + | GGCAACAACAAAGCCAGGGG | 20 | 1366 |
| HSV1-UL19-1184 | + | UCACAGUCCCACACGAGGGG | 20 | 1570 |
| HSV1-UL19-1272 | + | CGAACAGCGCGUGGACGGGG | 20 | 1658 |
| HSV1-UL19-316 | - | CCGACGUGGAGCUGCCGGGG | 20 | 702 |
| HSV1-UL19-3235 | + | UACCCCAAGGAAGGGCGGGG | 20 | 7834 |
| HSV1-UL19-489 | - | CCGGCACGAAACUGGCGGGG | 20 | 875 |
| HSV1-UL19-1077 | + | CAAAAAAACAGCUGGCGGGG | 20 | 1463 |
| HSV1-UL19-1014 | + | GAGGC GCUGUUUGAUC GGGG | 20 | 1400 |
| HSV1-UL19-1363 | + | GACGUCGGUCGACACGGGGG | 20 | 1749 |
| HSV1-UL19-1064 | + | GGGAUCCGGGUGGCCGGGGG | 20 | 1450 |
| HSV1-UL19-317 | - | CGACGUGGAGCUGCCGGGGG | 20 | 703 |
| HSV1-UL19-405 | - | UGACCGGCCGCACCGGGGGG | 20 | 791 |
| HSV1-UL19-341 | - | CCGGGGCCUGGAGCUGGGGG | 20 | 727 |
| HSV1-UL19-309 | - | GUUCGACUUCUUUGUGGGGG | 20 | 695 |
| HSV1-UL19-375 | - | CUGACCGGCCCGGAGCUGGG | 20 | 761 |
| HSV1-UL19-1261 | + | GGUAUGGUCCAGAUGCUGGG | 20 | 1647 |
| HSV1-UL19-1034 | + | GCGCACGCGCGUCGUCUGGG | 20 | 1420 |
| HSV1-UL19-416 | - | CGCCACCCUGCAGAACAUGG | 20 | 802 |
| HSV1-UL19-1247 | + | CACCUGCAGCGUGAGCAUGG | 20 | 1633 |
| HSV1-UL19-464 | - | GCUGACCUACGCGCUCAUGG | 20 | 850 |
| HSV1-UL19-1192 | + | UAACCCGGCAGUCGCGAUGG | 20 | 1578 |
| HSV1-UL19-1147 | + | CCCGCGAACGGCGGCGAUGG | 20 | 1533 |
| HSV1-UL19-1309 | + | UGAACCCAAACCCGGGAUGG | 20 | 1695 |
| HSV1-UL19-1155 | + | GUGCUCGCUGCCGUGUAUGG | 20 | 1541 |
| HSV1-UL19-484 | - | GUGGCCCGGCACGAAACUGG | 20 | 870 |
| HSV1-UL19-1082 | + | GAGACAGGCGCAGCACCUGG | 20 | 1468 |
| HSV1-UL19-367 | - | GGCCGUGUACCGGGACCUGG | 20 | 753 |
| HSV1-UL19-988 | + | GGUCGCCCGGGCCACCCUGG | 20 | 1374 |
| HSV1-UL19-255 | - | CAACGUUCCCUACCCCCUGG | 20 | 641 |
| HSV1-UL19-293 | - | AGCUGUUUUUGAACGCCUGG | 20 | 679 |
| HSV1-UL19-1080 | + | UCCCCCAAAAAAACAGCUGG | 20 | 1466 |
| HSV1-UL19-340 | - | CGCCCGGGGCCUGGAGCUGG | 20 | 726 |
| HSV1-UL19-1410 | + | CUUAAACUGUACGUCGCUGG | 20 | 1796 |
| HSV1-UL19-1262 | + | UGGUAUGGUCCAGAUGCUGG | 20 | 1648 |
| HSV1-UL19-423 | - | CCUGCACCCGGCCAAUCUGG | 20 | 809 |
| HSV1-UL19-458 | - | CCUGGUCCCCCCGGCUCUGG | 20 | 844 |
| HSV1-UL19-1035 | + | CGCGCACGCGCGUCGUCUGG | 20 | 1421 |
| HSV1-UL19-252 | - | CGAGAAGCUGGUCUUUCUGG | 20 | 638 |
| HSV1-UL19-227 | - | GCAGUCCUUCCUGAAAGUGG | 20 | 613 |
| HSV1-UL19-287 | - | UCCGUACGGGGCGUACGUGG | 20 | 673 |
| HSV1-UL19-570 | - | GGGGUGCCGCGAACUCGUGG | 20 | 956 |
| HSV1-UL19-509 | - | CCUGCGGAACGCGGUCGUGG | 20 | 895 |
| HSV1-UL19-1029 | + | CCUGCAUCUCCAGCAGGUGG | 20 | 1415 |
| HSV1-UL19-1146 | + | GCGAACGGCGGCGAUGGUGG | 20 | 1532 |
| HSV1-UL19-1042 | + | GGGGUAGGGAACGUUGGUGG | 20 | 1428 |
| HSV1-UL19-1256 | + | GAAUAUGCGCAUGUUGGUGG | 20 | 1642 |
| HSV1-UL19-308 | - | CGCGUUCGACUUCUUUGUGG | 20 | 694 |
| HSV1-UL19-1094 | + | GUACGCCCCGUACGGAUUGG | 20 | 1480 |
| HSV1-UL19-1043 | + | CAGGGGGUAGGGAACGUUGG | 20 | 1429 |
| HSV1-UL19-1257 | + | GUCGAAUAUGCGCAUGUUGG | 20 | 1643 |
| HSV1-UL19-1403 | + | CUCCAGGCAGCGAUGUUUGG | 20 | 1789 |
| HSV1-UL19-271 | - | CCCGCCAGCUGUUUUUUUGG | 20 | 657 |
| HSV1-UL19-1381 | + | UCUGGCCGUGGUCGUACAUG | 20 | 1767 |
| HSV1-UL19-381 | - | GGGACUGUGACGCCCUGAUG | 20 | 767 |
| HSV1-UL19-1432 | + | CUUCCCCGUUGCGGGCACUG | 20 | 1818 |
| HSV1-UL19-506 | - | ACGCGGCAGCCGUGUACCUG | 20 | 892 |
| HSV1-UL19-292 | - | CAGCUGUUUUUGAACGCCUG | 20 | 678 |
| HSV1-UL19-513 | - | UCGUGGCGGGAAACCGCCUG | 20 | 899 |
| HSV1-UL19-339 | - | ACGCCCGGGGCCUGGAGCUG | 20 | 725 |
| HSV1-UL19-1228 | + | GGUCCGUCACGGGGUCGCUG | 20 | 1614 |
| HSV1-UL19-1263 | + | AUGGUAUGGUCCAGAUGCUG | 20 | 1649 |
| HSV1-UL19-457 | - | CCCUGGUCCCCCCGGCUCUG | 20 | 843 |
| HSV1-UL19-1036 | + | GCGCGCACGCGCGUCGUCUG | 20 | 1422 |
| HSV1-UL19-1104 | + | AAACAGCUGCUGCAUGUCUG | 20 | 1490 |
| HSV1-UL19-571 | - | GAACUCGUGGAGGACCCGUG | 20 | 957 |
| HSV1-UL19-379 | - | CGCUGCUGCCACCCCUCGUG | 20 | 765 |
| HSV1-UL19-1209 | + | GUCCAGUCCGCCCCCCGGUG | 20 | 1595 |
| HSV1-UL19-1001 | + | AGGGCUGCGUGGCCGUGGUG | 20 | 1387 |
| HSV1-UL19-307 | - | CCGCGUUCGACUUCUUUGUG | 20 | 693 |
| HSV1-UL19-3236 | - | AACCGCCCCGCCCUUCCUUG | 20 | 7835 |
| HSV1-UL19-1212 | + | UCACGUAGUAGUAAAUCUUG | 20 | 1598 |
| HSV1-UL19-1151 | + | AAAACACCGCCGGGUAGUUG | 20 | 1537 |
| HSV1-UL19-1396 | + | GCACCGGCGAGGCCCCGUUG | 20 | 1782 |
| HSV1-UL19-1434 | + | GCGCGUGGGCUUCCCCGUUG | 20 | 1820 |
| HSV1-UL19-1200 | + | GCAGCAGCUGGCCGUCGUUG | 20 | 1586 |
| HSV1-UL19-1329 | + | CCAUGUCGGUGACGGGGUUG | 20 | 1715 |
| HSV1-UL19-1250 | + | UUGUGCGUUCGGCCAUGUUG | 20 | 1636 |
| HSV1-UL19-305 | - | CCCCGCGUUCGACUUCUUUG | 20 | 691 |
| HSV1-UL19-1335 | + | CGGCCCAGGUAAAAGUUUUG | 20 | 1721 |
| HSV1-UL19-270 | - | CCCCGCCAGCUGUUUUUUUG | 20 | 656 |
| HSV1-UL19-1281 | + | GCGCAGGGCCGGGGGGAAAU | 20 | 1667 |
| HSV1-UL19-498 | - | CGCAACCCCGUCACCGACAU | 20 | 884 |
| HSV1-UL19-1109 | + | UCUGGCCUUCCGCGACCCAU | 20 | 1495 |
| HSV1-UL19-249 | - | CGCGCGGAUCUGGUGUCCAU | 20 | 635 |
| HSV1-UL19-984 | + | UUGUCCAGGUAUCGUUGCAU | 20 | 1370 |
| HSV1-UL19-1243 | + | GACCGUGUUGGCCACCAGAU | 20 | 1629 |
| HSV1-UL19-300 | - | GGCCCACGGGCGGGUCCGAU | 20 | 686 |
| HSV1-UL19-275 | - | CUGUCUCUGGAACACGCGAU | 20 | 661 |
| HSV1-UL19-1095 | + | CACGUACGCCCCGUACGGAU | 20 | 1481 |
| HSV1-UL19-237 | - | CUGGUCACGGCGCUCGUGAU | 20 | 623 |
| HSV1-UL19-1017 | + | UGCAGGAGGCGCUGUUUGAU | 20 | 1403 |
| HSV1-UL19-1061 | + | GGUGGCCGGGGGCGGGGACU | 20 | 1447 |
| HSV1-UL19-1357 | + | CCCUGCGCGCCGCGGCACCU | 20 | 1743 |
| HSV1-UL19-492 | - | CCGCGCGCGAACGUGGACCU | 20 | 878 |
| HSV1-UL19-358 | - | UCGUACGUCGUGACCUACCU | 20 | 744 |
| HSV1-UL19-500 | - | CUUCCCCAAAACUUUUACCU | 20 | 886 |
| HSV1-UL19-259 | - | CUGACGUUCGUCCUGCCCCU | 20 | 645 |
| HSV1-UL19-291 | - | GCAGCUGUUUUUGAACGCCU | 20 | 677 |
| HSV1-UL19-512 | - | GUCGUGGCGGGAAACCGCCU | 20 | 898 |
| HSV1-UL19-1202 | + | GGCGUCCGCGGCUCGGGCCU | 20 | 1588 |
| HSV1-UL19-1167 | + | GUACACGGCCAUGCACUCCU | 20 | 1553 |
| HSV1-UL19-189 | - | CGCAACGUCCAGGCCGUCCU | 20 | 575 |
| HSV1-UL19-479 | - | GCGUCGGAGGCGUACUUCCU | 20 | 865 |
| HSV1-UL19-3237 | - | UAAACCGCCCCGCCCUUCCU | 20 | 7836 |
| HSV1-UL19-207 | - | UGCGAGACGAGCUUUUUCCU | 20 | 593 |
| HSV1-UL19-1173 | + | GGUAAAGUCAUCAACCAGCU | 20 | 1559 |
| HSV1-UL19-1180 | + | UCGCAUUAGGUGAUUCAGCU | 20 | 1566 |
| HSV1-UL19-374 | - | ACCCUGACCGGCCCGGAGCU | 20 | 760 |
| HSV1-UL19-338 | - | GACGCCCGGGGCCUGGAGCU | 20 | 724 |
| HSV1-UL19-994 | + | GCAGAAGCUUCGCUUUAGCU | 20 | 1380 |
| HSV1-UL19-1229 | + | GGGUCCGUCACGGGGUCGCU | 20 | 1615 |
| HSV1-UL19-1205 | + | CAUCCGCGGCGUCCGCGGCU | 20 | 1591 |
| HSV1-UL19-1321 | + | GUCCACGUUCGCGCGCGGCU | 20 | 1707 |
| HSV1-UL19-1350 | + | CCCGAACACGGGGACGGGCU | 20 | 1736 |
| HSV1-UL19-1264 | + | GAUGGUAUGGUCCAGAUGCU | 20 | 1650 |
| HSV1-UL19-435 | - | CACAACGGCGCUGCUCUGCU | 20 | 821 |
| HSV1-UL19-456 | - | CCCCUGGUCCCCCCGGCUCU | 20 | 842 |
| HSV1-UL19-1037 | + | CGCGCGCACGCGCGUCGUCU | 20 | 1423 |
| HSV1-UL19-475 | - | GACUGAGAACGUGCUGUUCU | 20 | 861 |
| HSV1-UL19-1437 | + | GACGAGAUACUGCGCGAAGU | 20 | 1823 |
| HSV1-UL19-1299 | + | UCGGAUCGACGAAAAGUAGU | 20 | 1685 |
| HSV1-UL19-1044 | + | CACCAGGGGGUAGGGAACGU | 20 | 1430 |
| HSV1-UL19-1121 | + | CCCCCCCGGCAGCUCCACGU | 20 | 1507 |
| HSV1-UL19-1023 | + | GCCGUACGUCACCGGCACGU | 20 | 1409 |
| HSV1-UL19-241 | - | GUGCGAAGUCUGGACGACGU | 20 | 627 |
| HSV1-UL19-1368 | + | CCGGCGGAAGUAGUUGACGU | 20 | 1754 |
| HSV1-UL19-558 | - | CUGCUUUAAGUUCUUUACGU | 20 | 944 |
| HSV1-UL19-547 | - | CGCGGCCACGGCCAACCCGU | 20 | 933 |
| HSV1-UL19-1107 | + | GACCCAUCGGACCCGCCCGU | 20 | 1493 |
| HSV1-UL19-1253 | + | CGCGGUGUUGGCGCCCGCGU | 20 | 1639 |
| HSV1-UL19-1305 | + | GUACCCCGCCAUGAGCGCGU | 20 | 1691 |
| HSV1-UL19-1435 | + | GAAGUGGGUCUCCCGCGCGU | 20 | 1821 |
| HSV1-UL19-476 | - | GCUGUUCUCGGAGCGCGCGU | 20 | 862 |
| HSV1-UL19-494 | - | GCGAACGUGGACCUGGGCGU | 20 | 880 |
| HSV1-UL19-1026 | + | CACGAGCGCCGUGACCAGGU | 20 | 1412 |
| HSV1-UL19-1116 | + | GUGCAGCUCAAGAGCCAGGU | 20 | 1502 |
| HSV1-UL19-1163 | + | GGGAAGGUCUCCCCCGAGGU | 20 | 1549 |
| HSV1-UL19-279 | - | AUGAACGUUGACGCGGCGGU | 20 | 665 |
| HSV1-UL19-1390 | + | UCGCUGCGACGCCCACGGGU | 20 | 1776 |
| HSV1-UL19-1421 | + | GCUGGCGCAGGUGAGCGGGU | 20 | 1807 |
| HSV1-UL19-1046 | + | CAUGGCGCCCACCAGGGGGU | 20 | 1432 |
| HSV1-UL19-342 | - | CGGGGCCUGGAGCUGGGGGU | 20 | 728 |
| HSV1-UL19-256 | - | AACGUUCCCUACCCCCUGGU | 20 | 642 |
| HSV1-UL19-978 | + | CAGCACGUGCAGCAUCUGGU | 20 | 1364 |
| HSV1-UL19-1278 | + | GGGCGCGUUCGCCACGUGGU | 20 | 1664 |
| HSV1-UL19-1394 | + | CGAGGCCCCGUUGAGGUGGU | 20 | 1780 |
| HSV1-UL19-1333 | + | UUGGGGAAGGUUGCCCAUGU | 20 | 1719 |
| HSV1-UL19-1258 | + | CCCGUCGAAUAUGCGCAUGU | 20 | 1644 |
| HSV1-UL19-1402 | + | GCGAUGUUUGGCGGCGAUGU | 20 | 1788 |
| HSV1-UL19-1314 | + | CAGUCACAAAGCGGUCCUGU | 20 | 1700 |
| HSV1-UL19-3213 | + | GGACGUUGCGAGCGAGCUGU | 20 | 7812 |
| HSV1-UL19-1300 | + | CGUGCUGCACGACGGGCUGU | 20 | 1686 |
| HSV1-UL19-1244 | + | AAACAUGGCGUUGACCGUGU | 20 | 1630 |
| HSV1-UL19-1011 | + | GACCGGCCGCCCGCGCGUGU | 20 | 1397 |
| HSV1-UL19-380 | - | GCUGCUGCCACCCCUCGUGU | 20 | 766 |
| HSV1-UL19-1254 | + | GUUGGUGGUGGACGCGGUGU | 20 | 1640 |
| HSV1-UL19-1115 | + | AAGAGCCAGGUUGGCGUUGU | 20 | 1501 |
| HSV1-UL19-1249 | + | UGUGCGUUCGGCCAUGUUGU | 20 | 1635 |
| HSV1-UL19-306 | - | CCCGCGUUCGACUUCUUUGU | 20 | 692 |
| HSV1-UL19-1280 | + | CGCAGGGCCGGGGGGAAAUU | 20 | 1666 |
| HSV1-UL19-1181 | + | GCAGCGCCGGGUCUCGCAUU | 20 | 1567 |
| HSV1-UL19-3238 | - | AAACCGCCCCGCCCUUCCUU | 20 | 7837 |
| HSV1-UL19-1022 | + | CGUCACCGGCACGUCGGCUU | 20 | 1408 |
| HSV1-UL19-1251 | + | GAGCAGCGCCGUUGUGCGUU | 20 | 1637 |
| HSV1-UL19-563 | - | CCUGAUCGUGGAGACGGGUU | 20 | 949 |
| HSV1-UL19-472 | - | ACGGGCCUCCAUCCCGGGUU | 20 | 858 |
| HSV1-UL19-1404 | + | GCGCUCCAGGCAGCGAUGUU | 20 | 1790 |
| HSV1-UL19-515 | - | GCCCAGCCCGUCCCCGUGUU | 20 | 901 |
| HSV1-UL19-1268 | + | GGUAAAAAUAGUCGCCAUUU | 20 | 1654 |
| HSV1-UL19-1337 | + | CGCGGCCCAGGUAAAAGUUU | 20 | 1723 |
| HSV1-UL19-473 | - | CGGGCCUCCAUCCCGGGUUU | 20 | 859 |
| HSV1-UL19-1336 | + | GCGGCCCAGGUAAAAGUUUU | 20 | 1722 |
| HSV1-UL19-268 | - | CGCCCCGCCAGCUGUUUUUU | 20 | 654 |
| HSV1-UL19-269 | - | GCCCCGCCAGCUGUUUUUUU | 20 | 655 |

**Table 6A** provides exemplary targeting domains for knocking out the *UL19* gene selected according to the first tier parameters. The targeting domains bind within the first 500 bp of the coding sequence (e.g., within 500 bp downstream from the start codon), have a high level of orthogonality, and start with a 5'G. The PAM is NNGRRT. It is contemplated herein that in an embodiment the targeting domain hybridizes to the target domain through complementary base pairing. Any of the targeting domains in the table can be used with a S. *aureus* Cas9 molecule that generates a double stranded break (Cas9 nuclease) or a single-stranded break (Cas9 nickase).

**Table 6A**

| **1st Tier** | | | | |
|---|---|---|---|---|
| **gRNA Name** | **DNA Strand** | **Targeting Domain** | **Target Site Length** | **Seq ID NO:** |
| HSV1-UL19-3239 | + | GCUCCAGAAAGCGCACGAGCGA | 22 | 7838 |
| HSV1-UL19-3240 | + | GAGCUCCAGAAAGCGCACGAGCGA | 24 | 7839 |
| HSV1-UL19-3241 | + | GUAAUUGUGUGUGGGUUGCUCGA | 23 | 7840 |
| HSV1-UL19-3242 | + | GCGGCAUACCGGUAUCCCG | 19 | 7841 |
| HSV1-UL19-3243 | + | GCCGCGGCAUACCGGUAUCCCG | 22 | 7842 |
| HSV1-UL19-3244 | + | GGCCGCGGCAUACCGGUAUCCCG | 23 | 7843 |
| HSV1-UL19-3245 | + | GACGUUGCGAGCGAGCUG | 18 | 7844 |
| HSV1-UL19-3246 | + | GGACGUUGCGAGCGAGCUG | 19 | 7845 |
| HSV1-UL19-3247 | + | GCCUGGACGUUGCGAGCGAGCUG | 23 | 7846 |
| HSV1-UL19-3248 | + | GGCCUGGACGUUGCGAGCGAGCUG | 24 | 7847 |
| HSV1-UL19-3249 | - | GCCGCGGCCAUGGUGCCGA | 19 | 7848 |
| HSV1-UL19-3250 | - | GUAUGCCGCGGCCAUGGUGCCGA | 23 | 7849 |
| HSV1-UL19-3251 | - | GGUAUGCCGCGGCCAUGGUGCCGA | 24 | 7850 |
| HSV1-UL19-1918 | - | GCUCGUGCGCUUUCUGGAGC | 20 | 2301 |
| HSV1-UL19-3252 | - | GUCGCUCGUGCGCUUUCUGGAGC | 23 | 7851 |
| HSV1-UL19-3253 | - | GUCGAGUUCGACGCCCUGC | 19 | 7852 |
| HSV1-UL19-3254 | - | GACGUCGAGUUCGACGCCCUGC | 22 | 7853 |
| HSV1-UL19-3255 | - | GCUCCCAACCGCGACCCUC | 19 | 7854 |
| HSV1-UL19-3256 | - | GCCGCUCCCAACCGCGACCCUC | 22 | 7855 |
| HSV1-UL19-3257 | - | GGCCGCUCCCAACCGCGACCCUC | 23 | 7856 |
| HSV1-UL19-3258 | - | GCAAACAGCCUGUACGACG | 19 | 7857 |
| HSV1-UL19-3259 | - | GAUGCAAACAGCCUGUACGACG | 22 | 7858 |

**Table 6B** provides exemplary targeting domains for knocking out the *UL19* gene selected according to the second tier parameters. The targeting domains bind within the first 500 bp of the coding sequence (e.g., within 500 bp downstream from the start codon) and have a high level of orthogonality. The PAM is NNGRRT. It is contemplated herein that in an embodiment the targeting domain hybridizes to the target domain through complementary base pairing. Any of the targeting domains in the table can be used with a S. *aureus* Cas9 molecule that generates a double stranded break (Cas9 nuclease) or a single-stranded break (Cas9 nickase).

**Table 6B**

| **2nd Tier** | | | | |
|---|---|---|---|---|
| **gRNA Name** | **DNA Strand** | **Targeting Domain** | **Target Site Length** | **Seq ID NO:** |
| HSV1-UL19-3260 | + | CAGAAAGCGCACGAGCGA | 18 | 7859 |
| HSV1-UL19-3261 | + | CCAGAAAGCGCACGAGCGA | 19 | 7860 |
| HSV1-UL19-1986 | + | UCCAGAAAGCGCACGAGCGA | 20 | 2344 |
| HSV1-UL19-3262 | + | CUCCAGAAAGCGCACGAGCGA | 21 | 7861 |
| HSV1-UL19-3263 | + | AGCUCCAGAAAGCGCACGAGCGA | 23 | 7862 |
| HSV1-UL19-3264 | + | UGUGUGUGGGUUGCUCGA | 18 | 7863 |
| HSV1-UL19-3265 | + | UUGUGUGUGGGUUGCUCGA | 19 | 7864 |
| HSV1-UL19-58 | + | AUUGUGUGUGGGUUGCUCGA | 20 | 425 |
| HSV1-UL19-3266 | + | AAUUGUGUGUGGGUUGCUCGA | 21 | 7865 |
| HSV1-UL19-3267 | + | UAAUUGUGUGUGGGUUGCUCGA | 22 | 7866 |
| HSV1-UL19-3268 | + | UGUAAUUGUGUGUGGGUUGCUCGA | 24 | 7867 |
| HSV1-UL19-3269 | + | CGGCAUACCGGUAUCCCG | 18 | 7868 |
| HSV1-UL19-1972 | + | CGCGGCAUACCGGUAUCCCG | 20 | 2336 |
| HSV1-UL19-3270 | + | CCGCGGCAUACCGGUAUCCCG | 21 | 7869 |
| HSV1-UL19-3271 | + | UGGCCGCGGCAUACCGGUAUCCCG | 24 | 7870 |
| HSV1-UL19-3272 | + | UGGACGUUGCGAGCGAGCUG | 20 | 7871 |
| HSV1-UL19-3273 | + | CUGGACGUUGCGAGCGAGCUG | 21 | 7872 |
| HSV1-UL19-3274 | + | CCUGGACGUUGCGAGCGAGCUG | 22 | 7873 |
| HSV1-UL19-3275 | + | UUGGUCAUGUAAUUGUGU | 18 | 7874 |
| HSV1-UL19-3276 | + | CUUGGUCAUGUAAUUGUGU | 19 | 7875 |
| HSV1-UL19-1998 | + | UCUUGGUCAUGUAAUUGUGU | 20 | 2354 |
| HSV1-UL19-3277 | + | AUCUUGGUCAUGUAAUUGUGU | 21 | 7876 |
| HSV1-UL19-3278 | + | AAUCUUGGUCAUGUAAUUGUGU | 22 | 7877 |
| HSV1-UL19-3279 | + | UAAUCUUGGUCAUGUAAUUGUGU | 23 | 7878 |
| HSV1-UL19-3280 | + | AUAAUCUUGGUCAUGUAAUUGUGU | 24 | 7879 |
| HSV1-UL19-3281 | - | CCGCGGCCAUGGUGCCGA | 18 | 7880 |
| HSV1-UL19-1911 | - | UGCCGCGGCCAUGGUGCCGA | 20 | 2296 |
| HSV1-UL19-3282 | - | AUGCCGCGGCCAUGGUGCCGA | 21 | 7881 |
| HSV1-UL19-3283 | - | UAUGCCGCGGCCAUGGUGCCGA | 22 | 7882 |
| HSV1-UL19-3284 | - | UCGUGCGCUUUCUGGAGC | 18 | 7883 |
| HSV1-UL19-3285 | - | CUCGUGCGCUUUCUGGAGC | 19 | 7884 |
| HSV1-UL19-3286 | - | CGCUCGUGCGCUUUCUGGAGC | 21 | 7885 |
| HSV1-UL19-3287 | - | UCGCUCGUGCGCUUUCUGGAGC | 22 | 7886 |
| HSV1-UL19-3288 | - | UGUCGCUCGUGCGCUUUCUGGAGC | 24 | 7887 |
| HSV1-UL19-3289 | - | UCGAGUUCGACGCCCUGC | 18 | 7888 |
| HSV1-UL19-10 | - | CGUCGAGUUCGACGCCCUGC | 20 | 467 |
| HSV1-UL19-3290 | - | ACGUCGAGUUCGACGCCCUGC | 21 | 7889 |
| HSV1-UL19-3291 | - | CGACGUCGAGUUCGACGCCCUGC | 23 | 7890 |
| HSV1-UL19-3292 | - | ACGACGUCGAGUUCGACGCCCUGC | 24 | 7891 |
| HSV1-UL19-3293 | - | CUCCCAACCGCGACCCUC | 18 | 7892 |
| HSV1-UL19-1 | - | CGCUCCCAACCGCGACCCUC | 20 | 461 |
| HSV1-UL19-3294 | - | CCGCUCCCAACCGCGACCCUC | 21 | 7893 |
| HSV1-UL19-3295 | - | UGGCCGCUCCCAACCGCGACCCUC | 24 | 7894 |
| HSV1-UL19-3296 | - | CAAACAGCCUGUACGACG | 18 | 7895 |
| HSV1-UL19-1913 | - | UGCAAACAGCCUGUACGACG | 20 | 2298 |
| HSV1-UL19-3297 | - | AUGCAAACAGCCUGUACGACG | 21 | 7896 |
| HSV1-UL19-3298 | - | CGAUGCAAACAGCCUGUACGACG | 23 | 7897 |
| HSV1-UL19-3299 | - | CCGAUGCAAACAGCCUGUACGACG | 24 | 7898 |
| HSV1-UL19-3300 | - | ACGGGGGAGGCCCUGGACGGG | 21 | 7899 |
| HSV1-UL19-3301 | - | CACGGGGGAGGCCCUGGACGGG | 22 | 7900 |
| HSV1-UL19-3302 | - | UCACGGGGGAGGCCCUGGACGGG | 23 | 7901 |
| HSV1-UL19-3303 | - | CUCACGGGGGAGGCCCUGGACGGG | 24 | 7902 |

**Table 6C** provides exemplary targeting domains for knocking out the *UL19* gene selected according to the third tier parameters. The targeting domains bind within the first 500 bp of the coding sequence (e.g., within 500 bp downstream from the start codon). The PAM is NNGRRT. It is contemplated herein that in an embodiment the targeting domain hybridizes to the target domain through complementary base pairing. Any of the targeting domains in the table can be used with a S. *aureus* Cas9 molecule that generates a double stranded break (Cas9 nuclease) or a single-stranded break (Cas9 nickase).

**Table 6C**

| **3rd Tier** | | | | |
|---|---|---|---|---|
| **gRNA Name** | **DNA Strand** | **Targeting Domain** | **Target Site Length** | **Seq ID NO:** |
| HSV1-UL19-3304 | + | CGCGGUUGGGAGCGGCCA | 18 | 7903 |
| HSV1-UL19-3305 | + | UCGCGGUUGGGAGCGGCCA | 19 | 7904 |
| HSV1-UL19-3211 | + | GUCGCGGUUGGGAGCGGCCA | 20 | 7810 |
| HSV1-UL19-3306 | + | GGUCGCGGUUGGGAGCGGCCA | 21 | 7905 |
| HSV1-UL19-3307 | + | GGGUCGCGGUUGGGAGCGGCCA | 22 | 7906 |
| HSV1-UL19-3308 | + | AGGGUCGCGGUUGGGAGCGGCCA | 23 | 7907 |
| HSV1-UL19-3309 | + | GAGGGUCGCGGUUGGGAGCGGCCA | 24 | 7908 |
| HSV1-UL19-3310 | - | GGGGAGGCCCUGGACGGG | 18 | 7909 |
| HSV1-UL19-3311 | - | GGGGGAGGCCCUGGACGGG | 19 | 7910 |
| HSV1-UL19-1938 | - | CGGGGGAGGCCCUGGACGGG | 20 | 2314 |

**Table 6D** provides exemplary targeting domains for knocking out the *UL19* gene selected according to the fourth tier parameters. The targeting domains bind within the first 500 bp of the coding sequence (e.g., within 500 bp downstream from the start codon). PAM is NNGRRV. It is contemplated herein that in an embodiment the targeting domain hybridizes to the target domain through complementary base pairing. Any of the targeting domains in the table can be used with a S. *aureus* Cas9 molecule that generates a double stranded break (Cas9 nuclease) or a single-stranded break (Cas9 nickase).

**Table 6D**

| **4th Tier** | | | | |
|---|---|---|---|---|
| **gRNA Name** | **DNA Strand** | **Targeting Domain** | **Target Site Length** | **Seq ID NO:** |
| HSV1-UL19-3312 | + | UCGUACAGGCUGUUUGCA | 18 | 7911 |
| HSV1-UL19-3313 | + | GUCGUACAGGCUGUUUGCA | 19 | 7912 |
| HSV1-UL19-1983 | + | CGUCGUACAGGCUGUUUGCA | 20 | 2341 |
| HSV1-UL19-3314 | + | ACGUCGUACAGGCUGUUUGCA | 21 | 7913 |
| HSV1-UL19-3315 | + | GACGUCGUACAGGCUGUUUGCA | 22 | 7914 |
| HSV1-UL19-3316 | + | CGACGUCGUACAGGCUGUUUGCA | 23 | 7915 |
| HSV1-UL19-3317 | + | UCGACGUCGUACAGGCUGUUUGCA | 24 | 7916 |
| HSV1-UL19-3318 | + | ACGGCCUGGACGUUGCGA | 18 | 7917 |
| HSV1-UL19-3319 | + | GACGGCCUGGACGUUGCGA | 19 | 7918 |
| HSV1-UL19-3320 | + | GGACGGCCUGGACGUUGCGA | 20 | 7919 |
| HSV1-UL19-3321 | + | AGGACGGCCUGGACGUUGCGA | 21 | 7920 |
| HSV1-UL19-3322 | + | GAGGACGGCCUGGACGUUGCGA | 22 | 7921 |
| HSV1-UL19-3323 | + | CGAGGACGGCCUGGACGUUGCGA | 23 | 7922 |
| HSV1-UL19-3324 | + | CCGAGGACGGCCUGGACGUUGCGA | 24 | 7923 |
| HSV1-UL19-3325 | + | GCCACCUCGAUCGUGCUA | 18 | 7924 |
| HSV1-UL19-3326 | + | CGCCACCUCGAUCGUGCUA | 19 | 7925 |
| HSV1-UL19-80 | + | ACGCCACCUCGAUCGUGCUA | 20 | 508 |
| HSV1-UL19-3327 | + | GACGCCACCUCGAUCGUGCUA | 21 | 7926 |
| HSV1-UL19-3328 | + | CGACGCCACCUCGAUCGUGCUA | 22 | 7927 |
| HSV1-UL19-3329 | + | GCGACGCCACCUCGAUCGUGCUA | 23 | 7928 |
| HSV1-UL19-3330 | + | UGCGACGCCACCUCGAUCGUGCUA | 24 | 7929 |
| HSV1-UL19-3331 | + | ACGCACGCCACCGACAAC | 18 | 7930 |
| HSV1-UL19-3332 | + | CACGCACGCCACCGACAAC | 19 | 7931 |
| HSV1-UL19-1989 | + | ACACGCACGCCACCGACAAC | 20 | 2347 |
| HSV1-UL19-3333 | + | CACACGCACGCCACCGACAAC | 21 | 7932 |
| HSV1-UL19-3334 | + | ACACACGCACGCCACCGACAAC | 22 | 7933 |
| HSV1-UL19-3335 | + | UACACACGCACGCCACCGACAAC | 23 | 7934 |
| HSV1-UL19-3336 | + | GUACACACGCACGCCACCGACAAC | 24 | 7935 |
| HSV1-UL19-3337 | + | GUUUGCAUCGGAGCGCAC | 18 | 7936 |
| HSV1-UL19-3338 | + | UGUUUGCAUCGGAGCGCAC | 19 | 7937 |
| HSV1-UL19-1981 | + | CUGUUUGCAUCGGAGCGCAC | 20 | 2340 |
| HSV1-UL19-3339 | + | GCUGUUUGCAUCGGAGCGCAC | 21 | 7938 |
| HSV1-UL19-3340 | + | GGCUGUUUGCAUCGGAGCGCAC | 22 | 7939 |
| HSV1-UL19-3341 | + | AGGCUGUUUGCAUCGGAGCGCAC | 23 | 7940 |
| HSV1-UL19-3342 | + | CAGGCUGUUUGCAUCGGAGCGCAC | 24 | 7941 |
| HSV1-UL19-3343 | + | AUCAGCGGCUGGUGGACC | 18 | 7942 |
| HSV1-UL19-3344 | + | AAUCAGCGGCUGGUGGACC | 19 | 7943 |
| HSV1-UL19-1992 | + | CAAUCAGCGGCUGGUGGACC | 20 | 2349 |
| HSV1-UL19-3345 | + | GCAAUCAGCGGCUGGUGGACC | 21 | 7944 |
| HSV1-UL19-3346 | + | GGCAAUCAGCGGCUGGUGGACC | 22 | 7945 |
| HSV1-UL19-3347 | + | GGGCAAUCAGCGGCUGGUGGACC | 23 | 7946 |
| HSV1-UL19-3348 | + | CGGGCAAUCAGCGGCUGGUGGACC | 24 | 7947 |
| HSV1-UL19-3349 | + | CCGUCCAGGGCCUCCCCC | 18 | 7948 |
| HSV1-UL19-3350 | + | CCCGUCCAGGGCCUCCCCC | 19 | 7949 |
| HSV1-UL19-2002 | + | UCCCGUCCAGGGCCUCCCCC | 20 | 2358 |
| HSV1-UL19-3351 | + | GUCCCGUCCAGGGCCUCCCCC | 21 | 7950 |
| HSV1-UL19-3352 | + | CGUCCCGUCCAGGGCCUCCCCC | 22 | 7951 |
| HSV1-UL19-3353 | + | CCGUCCCGUCCAGGGCCUCCCCC | 23 | 7952 |
| HSV1-UL19-3354 | + | CCCGUCCCGUCCAGGGCCUCCCCC | 24 | 7953 |
| HSV1-UL19-3355 | + | UCGCGGUUGGGAGCGGCC | 18 | 7954 |
| HSV1-UL19-3356 | + | GUCGCGGUUGGGAGCGGCC | 19 | 7955 |
| HSV1-UL19-3357 | + | GGUCGCGGUUGGGAGCGGCC | 20 | 7956 |
| HSV1-UL19-3358 | + | GGGUCGCGGUUGGGAGCGGCC | 21 | 7957 |
| HSV1-UL19-3359 | + | AGGGUCGCGGUUGGGAGCGGCC | 22 | 7958 |
| HSV1-UL19-3360 | + | GAGGGUCGCGGUUGGGAGCGGCC | 23 | 7959 |
| HSV1-UL19-3361 | + | GGAGGGUCGCGGUUGGGAGCGGCC | 24 | 7960 |
| HSV1-UL19-3362 | + | CGCGGCAUACCGGUAUCC | 18 | 7961 |
| HSV1-UL19-3363 | + | CCGCGGCAUACCGGUAUCC | 19 | 7962 |
| HSV1-UL19-88 | + | GCCGCGGCAUACCGGUAUCC | 20 | 514 |
| HSV1-UL19-3364 | + | GGCCGCGGCAUACCGGUAUCC | 21 | 7963 |
| HSV1-UL19-3365 | + | UGGCCGCGGCAUACCGGUAUCC | 22 | 7964 |
| HSV1-UL19-3366 | + | AUGGCCGCGGCAUACCGGUAUCC | 23 | 7965 |
| HSV1-UL19-3367 | + | CAUGGCCGCGGCAUACCGGUAUCC | 24 | 7966 |
| HSV1-UL19-3368 | + | UUGCAUCGGAGCGCACGC | 18 | 7967 |
| HSV1-UL19-3369 | + | UUUGCAUCGGAGCGCACGC | 19 | 7968 |
| HSV1-UL19-78 | + | GUUUGCAUCGGAGCGCACGC | 20 | 506 |
| HSV1-UL19-3370 | + | UGUUUGCAUCGGAGCGCACGC | 21 | 7969 |
| HSV1-UL19-3371 | + | CUGUUUGCAUCGGAGCGCACGC | 22 | 7970 |
| HSV1-UL19-3372 | + | GCUGUUUGCAUCGGAGCGCACGC | 23 | 7971 |
| HSV1-UL19-3373 | + | GGCUGUUUGCAUCGGAGCGCACGC | 24 | 7972 |
| HSV1-UL19-3374 | + | CCGAGCUCCAGAAAGCGC | 18 | 7973 |
| HSV1-UL19-3375 | + | CCCGAGCUCCAGAAAGCGC | 19 | 7974 |
| HSV1-UL19-1987 | + | ACCCGAGCUCCAGAAAGCGC | 20 | 2345 |
| HSV1-UL19-3376 | + | AACCCGAGCUCCAGAAAGCGC | 21 | 7975 |
| HSV1-UL19-3377 | + | CAACCCGAGCUCCAGAAAGCGC | 22 | 7976 |
| HSV1-UL19-3378 | + | ACAACCCGAGCUCCAGAAAGCGC | 23 | 7977 |
| HSV1-UL19-3379 | + | GACAACCCGAGCUCCAGAAAGCGC | 24 | 7978 |
| HSV1-UL19-3380 | + | GAGCGGCCAUGGGGUCGC | 18 | 7979 |
| HSV1-UL19-3381 | + | GGAGCGGCCAUGGGGUCGC | 19 | 7980 |
| HSV1-UL19-3382 | + | GGGAGCGGCCAUGGGGUCGC | 20 | 7981 |
| HSV1-UL19-3383 | + | UGGGAGCGGCCAUGGGGUCGC | 21 | 7982 |
| HSV1-UL19-3384 | + | UUGGGAGCGGCCAUGGGGUCGC | 22 | 7983 |
| HSV1-UL19-3385 | + | GUUGGGAGCGGCCAUGGGGUCGC | 23 | 7984 |
| HSV1-UL19-3386 | + | GGUUGGGAGCGGCCAUGGGGUCGC | 24 | 7985 |
| HSV1-UL19-3387 | + | AAACGCCCCGAGGACGGC | 18 | 7986 |
| HSV1-UL19-3388 | + | CAAACGCCCCGAGGACGGC | 19 | 7987 |
| HSV1-UL19-2611 | + | UCAAACGCCCCGAGGACGGC | 20 | 2727 |
| HSV1-UL19-3389 | + | CCGCGGCAUACCGGUAUC | 18 | 7988 |
| HSV1-UL19-3390 | + | GCCGCGGCAUACCGGUAUC | 19 | 7989 |
| HSV1-UL19-1974 | + | GGCCGCGGCAUACCGGUAUC | 20 | 2337 |
| HSV1-UL19-3391 | + | UGGCCGCGGCAUACCGGUAUC | 21 | 7990 |
| HSV1-UL19-3392 | + | AUGGCCGCGGCAUACCGGUAUC | 22 | 7991 |
| HSV1-UL19-3393 | + | CAUGGCCGCGGCAUACCGGUAUC | 23 | 7992 |
| HSV1-UL19-3394 | + | CCAUGGCCGCGGCAUACCGGUAUC | 24 | 7993 |
| HSV1-UL19-3395 | + | GUUCAUGUAGGCCAGCUC | 18 | 7994 |
| HSV1-UL19-3396 | + | CGUUCAUGUAGGCCAGCUC | 19 | 7995 |
| HSV1-UL19-69 | + | UC GUUCAUGUAGGC CAGCUC | 20 | 435 |
| HSV1-UL19-3397 | + | CUCGUUCAUGUAGGCCAGCUC | 21 | 7996 |
| HSV1-UL19-3398 | + | CCUCGUUCAUGUAGGCCAGCUC | 22 | 7997 |
| HSV1-UL19-3399 | + | CCCUCGUUCAUGUAGGCCAGCUC | 23 | 7998 |
| HSV1-UL19-3400 | + | CCCCUCGUUCAUGUAGGCCAGCUC | 24 | 7999 |
| HSV1-UL19-3401 | + | CACCGACAACCCGAGCUC | 18 | 8000 |
| HSV1-UL19-3402 | + | CCACCGACAACCCGAGCUC | 19 | 8001 |
| HSV1-UL19-1988 | + | GCCACCGACAACCCGAGCUC | 20 | 2346 |
| HSV1-UL19-3403 | + | CGCCACCGACAACCCGAGCUC | 21 | 8002 |
| HSV1-UL19-3404 | + | ACGCCACCGACAACCCGAGCUC | 22 | 8003 |
| HSV1-UL19-3405 | + | CACGCCACCGACAACCCGAGCUC | 23 | 8004 |
| HSV1-UL19-3406 | + | GCACGCCACCGACAACCCGAGCUC | 24 | 8005 |
| HSV1-UL19-3407 | + | GCCGAUACCCGUCCCGUC | 18 | 8006 |
| HSV1-UL19-3408 | + | CGCCGAUACCCGUCCCGUC | 19 | 8007 |
| HSV1-UL19-2003 | + | GCGCCGAUACCCGUCCCGUC | 20 | 2359 |
| HSV1-UL19-3409 | + | CGCGCCGAUACCCGUCCCGUC | 21 | 8008 |
| HSV1-UL19-3410 | + | GCGCGCCGAUACCCGUCCCGUC | 22 | 8009 |
| HSV1-UL19-3411 | + | UGCGCGCCGAUACCCGUCCCGUC | 23 | 8010 |
| HSV1-UL19-3412 | + | AUGCGCGCCGAUACCCGUCCCGUC | 24 | 8011 |
| HSV1-UL19-3413 | + | GAUGGGGUGCGGGCCGUC | 18 | 8012 |
| HSV1-UL19-3414 | + | CGAUGGGGUGCGGGCCGUC | 19 | 8013 |
| HSV1-UL19-1994 | + | UCGAUGGGGUGCGGGCCGUC | 20 | 2351 |
| HSV1-UL19-3415 | + | CUCGAUGGGGUGCGGGCCGUC | 21 | 8014 |
| HSV1-UL19-3416 | + | GCUCGAUGGGGUGCGGGCCGUC | 22 | 8015 |
| HSV1-UL19-3417 | + | UGCUCGAUGGGGUGCGGGCCGUC | 23 | 8016 |
| HSV1-UL19-3418 | + | UUGCUCGAUGGGGUGCGGGCCGUC | 24 | 8017 |
| HSV1-UL19-3419 | + | CACCUCGAUCGUGCUAAG | 18 | 8018 |
| HSV1-UL19-3420 | + | CCACCUCGAUCGUGCUAAG | 19 | 8019 |
| HSV1-UL19-1976 | + | GCCACCUCGAUCGUGCUAAG | 20 | 2338 |
| HSV1-UL19-3421 | + | CGCCACCUCGAUCGUGCUAAG | 21 | 8020 |
| HSV1-UL19-3422 | + | ACGCCACCUCGAUCGUGCUAAG | 22 | 8021 |
| HSV1-UL19-3423 | + | GACGCCACCUCGAUCGUGCUAAG | 23 | 8022 |
| HSV1-UL19-3424 | + | CGACGCCACCUCGAUCGUGCUAAG | 24 | 8023 |
| HSV1-UL19-3425 | + | GUUGCAAUACGACCCCAG | 18 | 8024 |
| HSV1-UL19-3426 | + | UGUUGCAAUACGACCCCAG | 19 | 8025 |
| HSV1-UL19-1985 | + | GUGUUGCAAUACGACCCCAG | 20 | 2343 |
| HSV1-UL19-3427 | + | GGUGUUGCAAUACGACCCCAG | 21 | 8026 |
| HSV1-UL19-3428 | + | GGGUGUUGCAAUACGACCCCAG | 22 | 8027 |
| HSV1-UL19-3429 | + | AGGGUGUUGCAAUACGACCCCAG | 23 | 8028 |
| HSV1-UL19-3430 | + | CAGGGUGUUGCAAUACGACCCCAG | 24 | 8029 |
| HSV1-UL19-3431 | + | CAGGGCCUCCCCCGUGAG | 18 | 8030 |
| HSV1-UL19-3432 | + | CCAGGGCCUCCCCCGUGAG | 19 | 8031 |
| HSV1-UL19-2001 | + | UCCAGGGCCUCCCCCGUGAG | 20 | 2357 |
| HSV1-UL19-3433 | + | GUCCAGGGCCUCCCCCGUGAG | 21 | 8032 |
| HSV1-UL19-3434 | + | CGUCCAGGGCCUCCCCCGUGAG | 22 | 8033 |
| HSV1-UL19-3435 | + | CCGUCCAGGGCCUCCCCCGUGAG | 23 | 8034 |
| HSV1-UL19-3436 | + | CCCGUCCAGGGCCUCCCCCGUGAG | 24 | 8035 |
| HSV1-UL19-3437 | + | UUUGCAUCGGAGCGCACG | 18 | 8036 |
| HSV1-UL19-3438 | + | GUUUGCAUCGGAGCGCACG | 19 | 8037 |
| HSV1-UL19-77 | + | UGUUUGCAUCGGAGCGCACG | 20 | 505 |
| HSV1-UL19-3439 | + | CUGUUUGCAUCGGAGCGCACG | 21 | 8038 |
| HSV1-UL19-3440 | + | GCUGUUUGCAUCGGAGCGCACG | 22 | 8039 |
| HSV1-UL19-3441 | + | GGCUGUUUGCAUCGGAGCGCACG | 23 | 8040 |
| HSV1-UL19-3442 | + | AGGCUGUUUGCAUCGGAGCGCACG | 24 | 8041 |
| HSV1-UL19-3443 | + | AGCGGCCAUGGGGUCGCG | 18 | 8042 |
| HSV1-UL19-3444 | + | GAGCGGCCAUGGGGUCGCG | 19 | 8043 |
| HSV1-UL19-3223 | + | GGAGCGGCCAUGGGGUCGCG | 20 | 7822 |
| HSV1-UL19-3445 | + | GGGAGCGGCCAUGGGGUCGCG | 21 | 8044 |
| HSV1-UL19-3446 | + | UGGGAGCGGCCAUGGGGUCGCG | 22 | 8045 |
| HSV1-UL19-3447 | + | UUGGGAGCGGCCAUGGGGUCGCG | 23 | 8046 |
| HSV1-UL19-3448 | + | GUUGGGAGCGGCCAUGGGGUCGCG | 24 | 8047 |
| HSV1-UL19-3449 | + | UACGACCCCAGCAGGGCG | 18 | 8048 |
| HSV1-UL19-3450 | + | AUACGACCCCAGCAGGGCG | 19 | 8049 |
| HSV1-UL19-1984 | + | AAUACGACCCCAGCAGGGCG | 20 | 2342 |
| HSV1-UL19-3451 | + | CAAUACGACCCCAGCAGGGCG | 21 | 8050 |
| HSV1-UL19-3452 | + | GCAAUACGACCCCAGCAGGGCG | 22 | 8051 |
| HSV1-UL19-3453 | + | UGCAAUACGACCCCAGCAGGGCG | 23 | 8052 |
| HSV1-UL19-3454 | + | UUGCAAUACGACCCCAGCAGGGCG | 24 | 8053 |
| HSV1-UL19-3455 | + | UUGUGUGUGGGUUGCUCG | 18 | 8054 |
| HSV1-UL19-3456 | + | AUUGUGUGUGGGUUGCUCG | 19 | 8055 |
| HSV1-UL19-1997 | + | AAUUGUGUGUGGGUUGCUCG | 20 | 2353 |
| HSV1-UL19-3457 | + | UAAUUGUGUGUGGGUUGCUCG | 21 | 8056 |
| HSV1-UL19-3458 | + | GUAAUUGUGUGUGGGUUGCUCG | 22 | 8057 |
| HSV1-UL19-3459 | + | UGUAAUUGUGUGUGGGUUGCUCG | 23 | 8058 |
| HSV1-UL19-3460 | + | AUGUAAUUGUGUGUGGGUUGCUCG | 24 | 8059 |
| HSV1-UL19-3461 | + | ACCUCGAUCGUGCUAAGG | 18 | 8060 |
| HSV1-UL19-3462 | + | CACCUCGAUCGUGCUAAGG | 19 | 8061 |
| HSV1-UL19-81 | + | CCACCUCGAUCGUGCUAAGG | 20 | 509 |
| HSV1-UL19-3463 | + | GCCACCUCGAUCGUGCUAAGG | 21 | 8062 |
| HSV1-UL19-3464 | + | CGCCACCUCGAUCGUGCUAAGG | 22 | 8063 |
| HSV1-UL19-3465 | + | ACGCCACCUCGAUCGUGCUAAGG | 23 | 8064 |
| HSV1-UL19-3466 | + | GACGCCACCUCGAUCGUGCUAAGG | 24 | 8065 |
| HSV1-UL19-3467 | + | AUGGCCUCGGUGGCCAGG | 18 | 8066 |
| HSV1-UL19-3468 | + | GAUGGCCUCGGUGGCCAGG | 19 | 8067 |
| HSV1-UL19-2000 | + | CGAUGGCCUCGGUGGCCAGG | 20 | 2356 |
| HSV1-UL19-3469 | + | GCGAUGGCCUCGGUGGCCAGG | 21 | 8068 |
| HSV1-UL19-3470 | + | GGCGAUGGCCUCGGUGGCCAGG | 22 | 8069 |
| HSV1-UL19-3471 | + | GGGCGAUGGCCUCGGUGGCCAGG | 23 | 8070 |
| HSV1-UL19-3472 | + | AGGGCGAUGGCCUCGGUGGCCAGG | 24 | 8071 |
| HSV1-UL19-3473 | + | UAUCCCGGAGGGUCGCGG | 18 | 8072 |
| HSV1-UL19-3474 | + | GUAUCCCGGAGGGUCGCGG | 19 | 8073 |
| HSV1-UL19-1971 | + | GGUAUCCCGGAGGGUCGCGG | 20 | 2335 |
| HSV1-UL19-3475 | + | CGGUAUCCCGGAGGGUCGCGG | 21 | 8074 |
| HSV1-UL19-3476 | + | CCGGUAUCCCGGAGGGUCGCGG | 22 | 8075 |
| HSV1-UL19-3477 | + | ACCGGUAUCCCGGAGGGUCGCGG | 23 | 8076 |
| HSV1-UL19-3478 | + | UACCGGUAUCCCGGAGGGUCGCGG | 24 | 8077 |
| HSV1-UL19-3479 | + | GCGGGCAAUCAGCGGCUG | 18 | 8078 |
| HSV1-UL19-3480 | + | CGCGGGCAAUCAGCGGCUG | 19 | 8079 |
| HSV1-UL19-1993 | + | UCGCGGGCAAUCAGCGGCUG | 20 | 2350 |
| HSV1-UL19-3481 | + | GUCGCGGGCAAUCAGCGGCUG | 21 | 8080 |
| HSV1-UL19-3482 | + | CGUCGCGGGCAAUCAGCGGCUG | 22 | 8081 |
| HSV1-UL19-3483 | + | CCGUCGCGGGCAAUCAGCGGCUG | 23 | 8082 |
| HSV1-UL19-3484 | + | GCCGUCGCGGGCAAUCAGCGGCUG | 24 | 8083 |
| HSV1-UL19-3485 | + | CGUACAGGCUGUUUGCAU | 18 | 8084 |
| HSV1-UL19-3486 | + | UCGUACAGGCUGUUUGCAU | 19 | 8085 |
| HSV1-UL19-76 | + | GUCGUACAGGCUGUUUGCAU | 20 | 442 |
| HSV1-UL19-3487 | + | CGUCGUACAGGCUGUUUGCAU | 21 | 8086 |
| HSV1-UL19-3488 | + | ACGUCGUACAGGCUGUUUGCAU | 22 | 8087 |
| HSV1-UL19-3489 | + | GACGUCGUACAGGCUGUUUGCAU | 23 | 8088 |
| HSV1-UL19-3490 | + | CGACGUCGUACAGGCUGUUUGCAU | 24 | 8089 |
| HSV1-UL19-3491 | + | CGUUCAUGUAGGCCAGCU | 18 | 8090 |
| HSV1-UL19-3492 | + | UC GUUCAUGUAGGC CAGCU | 19 | 8091 |
| HSV1-UL19-1991 | + | CUCGUUCAUGUAGGCCAGCU | 20 | 2348 |
| HSV1-UL19-3493 | + | CCUCGUUCAUGUAGGCCAGCU | 21 | 8092 |
| HSV1-UL19-3494 | + | CCCUCGUUCAUGUAGGCCAGCU | 22 | 8093 |
| HSV1-UL19-3495 | + | CCCCUCGUUCAUGUAGGCCAGCU | 23 | 8094 |
| HSV1-UL19-3496 | + | GCCCCUCGUUCAUGUAGGCCAGCU | 24 | 8095 |
| HSV1-UL19-3497 | + | CGCCACCUCGAUCGUGCU | 18 | 8096 |
| HSV1-UL19-3498 | + | ACGCCACCUCGAUCGUGCU | 19 | 8097 |
| HSV1-UL19-1978 | + | GACGCCACCUCGAUCGUGCU | 20 | 2339 |
| HSV1-UL19-3499 | + | CGACGCCACCUCGAUCGUGCU | 21 | 8098 |
| HSV1-UL19-3500 | + | GCGACGCCACCUCGAUCGUGCU | 22 | 8099 |
| HSV1-UL19-3501 | + | UGCGACGCCACCUCGAUCGUGCU | 23 | 8100 |
| HSV1-UL19-3502 | + | AUGCGACGCCACCUCGAUCGUGCU | 24 | 8101 |
| HSV1-UL19-3503 | + | AUCCCGGAGGGUCGCGGU | 18 | 8102 |
| HSV1-UL19-3504 | + | UAUCCCGGAGGGUCGCGGU | 19 | 8103 |
| HSV1-UL19-92 | + | GUAUCCCGGAGGGUCGCGGU | 20 | 456 |
| HSV1-UL19-3505 | + | GGUAUCCCGGAGGGUCGCGGU | 21 | 8104 |
| HSV1-UL19-3506 | + | CGGUAUCCCGGAGGGUCGCGGU | 22 | 8105 |
| HSV1-UL19-3507 | + | CCGGUAUCCCGGAGGGUCGCGGU | 23 | 8106 |
| HSV1-UL19-3508 | + | ACCGGUAUCCCGGAGGGUCGCGGU | 24 | 8107 |
| HSV1-UL19-3509 | + | UGGGUUGCUCGAUGGGGU | 18 | 8108 |
| HSV1-UL19-3510 | + | GUGGGUUGCUCGAUGGGGU | 19 | 8109 |
| HSV1-UL19-1995 | + | UGUGGGUUGCUCGAUGGGGU | 20 | 2352 |
| HSV1-UL19-3511 | + | GUGUGGGUUGCUCGAUGGGGU | 21 | 8110 |
| HSV1-UL19-3512 | + | UGUGUGGGUUGCUCGAUGGGGU | 22 | 8111 |
| HSV1-UL19-3513 | + | GUGUGUGGGUUGCUCGAUGGGGU | 23 | 8112 |
| HSV1-UL19-3514 | + | UGUGUGUGGGUUGCUCGAUGGGGU | 24 | 8113 |
| HSV1-UL19-3515 | + | GAGGACGGCCUGGACGUU | 18 | 8114 |
| HSV1-UL19-3516 | + | CGAGGACGGCCUGGACGUU | 19 | 8115 |
| HSV1-UL19-3517 | + | CCGAGGACGGCCUGGACGUU | 20 | 8116 |
| HSV1-UL19-3518 | + | CCCGAGGACGGCCUGGACGUU | 21 | 8117 |
| HSV1-UL19-3519 | + | CCCCGAGGACGGCCUGGACGUU | 22 | 8118 |
| HSV1-UL19-3520 | + | GCCCCGAGGACGGCCUGGACGUU | 23 | 8119 |
| HSV1-UL19-3521 | + | CGCCCCGAGGACGGCCUGGACGUU | 24 | 8120 |
| HSV1-UL19-3522 | + | CAGGCUGAAGGCGGCGUU | 18 | 8121 |
| HSV1-UL19-3523 | + | CCAGGCUGAAGGCGGCGUU | 19 | 8122 |
| HSV1-UL19-1999 | + | GCCAGGCUGAAGGCGGCGUU | 20 | 2355 |
| HSV1-UL19-3524 | + | GGCCAGGCUGAAGGCGGCGUU | 21 | 8123 |
| HSV1-UL19-3525 | + | UGGCCAGGCUGAAGGCGGCGUU | 22 | 8124 |
| HSV1-UL19-3526 | + | GUGGCCAGGCUGAAGGCGGCGUU | 23 | 8125 |
| HSV1-UL19-3527 | + | GGUGGCCAGGCUGAAGGCGGCGUU | 24 | 8126 |
| HSV1-UL19-3528 | + | UCCCGGAGGGUCGCGGUU | 18 | 8127 |
| HSV1-UL19-3529 | + | AUCCCGGAGGGUCGCGGUU | 19 | 8128 |
| HSV1-UL19-93 | + | UAUCCCGGAGGGUCGCGGUU | 20 | 457 |
| HSV1-UL19-3530 | + | GUAUCCCGGAGGGUCGCGGUU | 21 | 8129 |
| HSV1-UL19-3531 | + | GGUAUCCCGGAGGGUCGCGGUU | 22 | 8130 |
| HSV1-UL19-3532 | + | CGGUAUCCCGGAGGGUCGCGGUU | 23 | 8131 |
| HSV1-UL19-3533 | + | CCGGUAUCCCGGAGGGUCGCGGUU | 24 | 8132 |
| HSV1-UL19-3534 | - | GCGACGGCCCGCACCCCA | 18 | 8133 |
| HSV1-UL19-3535 | - | CGCGACGGCCCGCACCCCA | 19 | 8134 |
| HSV1-UL19-1926 | - | CCGCGACGGCCCGCACCCCA | 20 | 2307 |
| HSV1-UL19-3536 | - | CCCGCGACGGCCCGCACCCCA | 21 | 8135 |
| HSV1-UL19-3537 | - | GCCCGCGACGGCCCGCACCCCA | 22 | 8136 |
| HSV1-UL19-3538 | - | UGCCCGCGACGGCCCGCACCCCA | 23 | 8137 |
| HSV1-UL19-3539 | - | UUGCCCGCGACGGCCCGCACCCCA | 24 | 8138 |
| HSV1-UL19-3540 | - | CCGCCUUCAGCCUGGCCA | 18 | 8139 |
| HSV1-UL19-3541 | - | GCCGCCUUCAGCCUGGCCA | 19 | 8140 |
| HSV1-UL19-1929 | - | CGCCGCCUUCAGCCUGGCCA | 20 | 2310 |
| HSV1-UL19-3542 | - | ACGCCGCCUUCAGCCUGGCCA | 21 | 8141 |
| HSV1-UL19-3543 | - | AACGCCGCCUUCAGCCUGGCCA | 22 | 8142 |
| HSV1-UL19-3544 | - | GAACGCCGCCUUCAGCCUGGCCA | 23 | 8143 |
| HSV1-UL19-3545 | - | UGAACGCCGCCUUCAGCCUGGCCA | 24 | 8144 |
| HSV1-UL19-3546 | - | AGGCCAUCGCCCUGCUCA | 18 | 8145 |
| HSV1-UL19-3547 | - | GAGGCCAUCGCCCUGCUCA | 19 | 8146 |
| HSV1-UL19-29 | - | CGAGGCCAUCGCCCUGCUCA | 20 | 474 |
| HSV1-UL19-3548 | - | CCGAGGCCAUCGCCCUGCUCA | 21 | 8147 |
| HSV1-UL19-3549 | - | ACCGAGGCCAUCGCCCUGCUCA | 22 | 8148 |
| HSV1-UL19-3550 | - | CACCGAGGCCAUCGCCCUGCUCA | 23 | 8149 |
| HSV1-UL19-3551 | - | CCACCGAGGCCAUCGCCCUGCUCA | 24 | 8150 |
| HSV1-UL19-3552 | - | GGUCCCUCCUUAGCACGA | 18 | 8151 |
| HSV1-UL19-3553 | - | GGGUCCCUCCUUAGCACGA | 19 | 8152 |
| HSV1-UL19-1912 | - | CGGGUCCCUCCUUAGCACGA | 20 | 2297 |
| HSV1-UL19-3554 | - | CCGGGUCCCUCCUUAGCACGA | 21 | 8153 |
| HSV1-UL19-3555 | - | ACCGGGUCCCUCCUUAGCACGA | 22 | 8154 |
| HSV1-UL19-3556 | - | GACCGGGUCCCUCCUUAGCACGA | 23 | 8155 |
| HSV1-UL19-3557 | - | CGACCGGGUCCCUCCUUAGCACGA | 24 | 8156 |
| HSV1-UL19-3558 | - | CACGGGGGAGGCCCUGGA | 18 | 8157 |
| HSV1-UL19-3559 | - | UCACGGGGGAGGCCCUGGA | 19 | 8158 |
| HSV1-UL19-35 | - | CUCACGGGGGAGGCCCUGGA | 20 | 478 |
| HSV1-UL19-3560 | - | GCUCACGGGGGAGGCCCUGGA | 21 | 8159 |
| HSV1-UL19-3561 | - | UGCUCACGGGGGAGGCCCUGGA | 22 | 8160 |
| HSV1-UL19-3562 | - | CUGCUCACGGGGGAGGCCCUGGA | 23 | 8161 |
| HSV1-UL19-3563 | - | CCUGCUCACGGGGGAGGCCCUGGA | 24 | 8162 |
| HSV1-UL19-3564 | - | CGGAGCUGGCCUACAUGA | 18 | 8163 |
| HSV1-UL19-3565 | - | CCGGAGCUGGCCUACAUGA | 19 | 8164 |
| HSV1-UL19-1922 | - | UCCGGAGCUGGCCUACAUGA | 20 | 2304 |
| HSV1-UL19-3566 | - | UUCCGGAGCUGGCCUACAUGA | 21 | 8165 |
| HSV1-UL19-3567 | - | UUUCCGGAGCUGGCCUACAUGA | 22 | 8166 |
| HSV1-UL19-3568 | - | GUUUCCGGAGCUGGCCUACAUGA | 23 | 8167 |
| HSV1-UL19-3569 | - | AGUUUCCGGAGCUGGCCUACAUGA | 24 | 8168 |
| HSV1-UL19-3570 | - | GAGCUGGCCUACAUGAAC | 18 | 8169 |
| HSV1-UL19-3571 | - | GGAGCUGGCCUACAUGAAC | 19 | 8170 |
| HSV1-UL19-1923 | - | CGGAGCUGGCCUACAUGAAC | 20 | 2305 |
| HSV1-UL19-3572 | - | CCGGAGCUGGCCUACAUGAAC | 21 | 8171 |
| HSV1-UL19-3573 | - | UCCGGAGCUGGCCUACAUGAAC | 22 | 8172 |
| HSV1-UL19-3574 | - | UUCCGGAGCUGGCCUACAUGAAC | 23 | 8173 |
| HSV1-UL19-3575 | - | UUUCCGGAGCUGGCCUACAUGAAC | 24 | 8174 |
| HSV1-UL19-3576 | - | GGCCAUCGCCCUGCUCAC | 18 | 8175 |
| HSV1-UL19-3577 | - | AGGCCAUCGCCCUGCUCAC | 19 | 8176 |
| HSV1-UL19-30 | - | GAGGCCAUCGCCCUGCUCAC | 20 | 475 |
| HSV1-UL19-3578 | - | CGAGGCCAUCGCCCUGCUCAC | 21 | 8177 |
| HSV1-UL19-3579 | - | CCGAGGCCAUCGCCCUGCUCAC | 22 | 8178 |
| HSV1-UL19-3580 | - | ACCGAGGCCAUCGCCCUGCUCAC | 23 | 8179 |
| HSV1-UL19-3581 | - | CACCGAGGCCAUCGCCCUGCUCAC | 24 | 8180 |
| HSV1-UL19-3582 | - | UUUCCGGAGCUGGCCUAC | 18 | 8181 |
| HSV1-UL19-3583 | - | GUUUCCGGAGCUGGCCUAC | 19 | 8182 |
| HSV1-UL19-1921 | - | AGUUUCCGGAGCUGGCCUAC | 20 | 2303 |
| HSV1-UL19-3584 | - | AAGUUUCCGGAGCUGGCCUAC | 21 | 8183 |
| HSV1-UL19-3585 | - | CAAGUUUCCGGAGCUGGCCUAC | 22 | 8184 |
| HSV1-UL19-3586 | - | CCAAGUUUCCGGAGCUGGCCUAC | 23 | 8185 |
| HSV1-UL19-3587 | - | ACCAAGUUUCCGGAGCUGGCCUAC | 24 | 8186 |
| HSV1-UL19-3588 | - | CUGCUCACGGGGGAGGCC | 18 | 8187 |
| HSV1-UL19-3589 | - | CCUGCUCACGGGGGAGGCC | 19 | 8188 |
| HSV1-UL19-1935 | - | CCCUGCUCACGGGGGAGGCC | 20 | 2312 |
| HSV1-UL19-3590 | - | GCCCUGCUCACGGGGGAGGCC | 21 | 8189 |
| HSV1-UL19-3591 | - | CGCCCUGCUCACGGGGGAGGCC | 22 | 8190 |
| HSV1-UL19-3592 | - | UCGCCCUGCUCACGGGGGAGGCC | 23 | 8191 |
| HSV1-UL19-3593 | - | AUCGCCCUGCUCACGGGGGAGGCC | 24 | 8192 |
| HSV1-UL19-3594 | - | AUUAUCGACCGCCGGGCC | 18 | 8193 |
| HSV1-UL19-3595 | - | GAUUAUCGACCGCCGGGCC | 19 | 8194 |
| HSV1-UL19-1928 | - | AGAUUAUCGACCGCCGGGCC | 20 | 2309 |
| HSV1-UL19-3596 | - | AAGAUUAUCGACCGCCGGGCC | 21 | 8195 |
| HSV1-UL19-3597 | - | CAAGAUUAUCGACCGCCGGGCC | 22 | 8196 |
| HSV1-UL19-3598 | - | CCAAGAUUAUCGACCGCCGGGCC | 23 | 8197 |
| HSV1-UL19-3599 | - | ACCAAGAUUAUCGACCGCCGGGCC | 24 | 8198 |
| HSV1-UL19-3600 | - | GCAACGUCCAGGCCGUCC | 18 | 8199 |
| HSV1-UL19-3601 | - | CGCAACGUCCAGGCCGUCC | 19 | 8200 |
| HSV1-UL19-3602 | - | UCGCAACGUCCAGGCCGUCC | 20 | 8201 |
| HSV1-UL19-3603 | - | CUCGCAACGUCCAGGCCGUCC | 21 | 8202 |
| HSV1-UL19-3604 | - | GCUCGCAACGUCCAGGCCGUCC | 22 | 8203 |
| HSV1-UL19-3605 | - | CGCUCGCAACGUCCAGGCCGUCC | 23 | 8204 |
| HSV1-UL19-3606 | - | UCGCUCGCAACGUCCAGGCCGUCC | 24 | 8205 |
| HSV1-UL19-3607 | - | GAGGCCAUCGCCCUGCUC | 18 | 8206 |
| HSV1-UL19-3608 | - | CGAGGCCAUCGCCCUGCUC | 19 | 8207 |
| HSV1-UL19-1930 | - | CCGAGGCCAUCGCCCUGCUC | 20 | 2311 |
| HSV1-UL19-3609 | - | ACCGAGGCCAUCGCCCUGCUC | 21 | 8208 |
| HSV1-UL19-3610 | - | CACCGAGGCCAUCGCCCUGCUC | 22 | 8209 |
| HSV1-UL19-3611 | - | CCACCGAGGCCAUCGCCCUGCUC | 23 | 8210 |
| HSV1-UL19-3612 | - | GCCACCGAGGCCAUCGCCCUGCUC | 24 | 8211 |
| HSV1-UL19-3613 | - | UGUCGCUCGUGCGCUUUC | 18 | 8212 |
| HSV1-UL19-3614 | - | CUGUCGCUCGUGCGCUUUC | 19 | 8213 |
| HSV1-UL19-13 | - | CCUGUCGCUCGUGCGCUUUC | 20 | 395 |
| HSV1-UL19-3615 | - | CCCUGUCGCUCGUGCGCUUUC | 21 | 8214 |
| HSV1-UL19-3616 | - | ACCCUGUCGCUCGUGCGCUUUC | 22 | 8215 |
| HSV1-UL19-3617 | - | CACCCUGUCGCUCGUGCGCUUUC | 23 | 8216 |
| HSV1-UL19-3618 | - | ACACCCUGUCGCUCGUGCGCUUUC | 24 | 8217 |
| HSV1-UL19-3619 | - | GCGUGUGUACCAAGUUUC | 18 | 8218 |
| HSV1-UL19-3620 | - | UGCGUGUGUACCAAGUUUC | 19 | 8219 |
| HSV1-UL19-18 | - | GUGCGUGUGUACCAAGUUUC | 20 | 404 |
| HSV1-UL19-3621 | - | CGUGCGUGUGUACCAAGUUUC | 21 | 8220 |
| HSV1-UL19-3622 | - | GCGUGCGUGUGUACCAAGUUUC | 22 | 8221 |
| HSV1-UL19-3623 | - | GGCGUGCGUGUGUACCAAGUUUC | 23 | 8222 |
| HSV1-UL19-3624 | - | UGGCGUGCGUGUGUACCAAGUUUC | 24 | 8223 |
| HSV1-UL19-3625 | - | AGCUGGCCUACAUGAACG | 18 | 8224 |
| HSV1-UL19-3626 | - | GAGCUGGCCUACAUGAACG | 19 | 8225 |
| HSV1-UL19-20 | - | GGAGCUGGCCUACAUGAACG | 20 | 469 |
| HSV1-UL19-3627 | - | CGGAGCUGGCCUACAUGAACG | 21 | 8226 |
| HSV1-UL19-3628 | - | CCGGAGCUGGCCUACAUGAACG | 22 | 8227 |
| HSV1-UL19-3629 | - | UCCGGAGCUGGCCUACAUGAACG | 23 | 8228 |
| HSV1-UL19-3630 | - | UUCCGGAGCUGGCCUACAUGAACG | 24 | 8229 |
| HSV1-UL19-3631 | - | GCCAUCGCCCUGCUCACG | 18 | 8230 |
| HSV1-UL19-3632 | - | GGCCAUCGCCCUGCUCACG | 19 | 8231 |
| HSV1-UL19-31 | - | AGGCCAUCGCCCUGCUCACG | 20 | 417 |
| HSV1-UL19-3633 | - | GAGGCCAUCGCCCUGCUCACG | 21 | 8232 |
| HSV1-UL19-3634 | - | CGAGGCCAUCGCCCUGCUCACG | 22 | 8233 |
| HSV1-UL19-3635 | - | CCGAGGCCAUCGCCCUGCUCACG | 23 | 8234 |
| HSV1-UL19-3636 | - | ACCGAGGCCAUCGCCCUGCUCACG | 24 | 8235 |
| HSV1-UL19-3637 | - | GACCAAGAUUAUCGACCG | 18 | 8236 |
| HSV1-UL19-3638 | - | UGACCAAGAUUAUCGACCG | 19 | 8237 |
| HSV1-UL19-1927 | - | AUGACCAAGAUUAUCGACCG | 20 | 2308 |
| HSV1-UL19-3639 | - | CAUGACCAAGAUUAUCGACCG | 21 | 8238 |
| HSV1-UL19-3640 | - | ACAUGACCAAGAUUAUCGACCG | 22 | 8239 |
| HSV1-UL19-3641 | - | UACAUGACCAAGAUUAUCGACCG | 23 | 8240 |
| HSV1-UL19-3642 | - | UUACAUGACCAAGAUUAUCGACCG | 24 | 8241 |
| HSV1-UL19-3643 | - | CCAUCGCCCUGCUCACGG | 18 | 8242 |
| HSV1-UL19-3644 | - | GCCAUCGCCCUGCUCACGG | 19 | 8243 |
| HSV1-UL19-32 | - | GGCCAUCGCCCUGCUCACGG | 20 | 476 |
| HSV1-UL19-3645 | - | AGGCCAUCGCCCUGCUCACGG | 21 | 8244 |
| HSV1-UL19-3646 | - | GAGGCCAUCGCCCUGCUCACGG | 22 | 8245 |
| HSV1-UL19-3647 | - | CGAGGCCAUCGCCCUGCUCACGG | 23 | 8246 |
| HSV1-UL19-3648 | - | CCGAGGCCAUCGCCCUGCUCACGG | 24 | 8247 |
| HSV1-UL19-3649 | - | UCACGGGGGAGGCCCUGG | 18 | 8248 |
| HSV1-UL19-3650 | - | CUCACGGGGGAGGCCCUGG | 19 | 8249 |
| HSV1-UL19-1936 | - | GCUCACGGGGGAGGCCCUGG | 20 | 2313 |
| HSV1-UL19-3651 | - | UGCUCACGGGGGAGGCCCUGG | 21 | 8250 |
| HSV1-UL19-3652 | - | CUGCUCACGGGGGAGGCCCUGG | 22 | 8251 |
| HSV1-UL19-3653 | - | CCUGCUCACGGGGGAGGCCCUGG | 23 | 8252 |
| HSV1-UL19-3654 | - | CCCUGCUCACGGGGGAGGCCCUGG | 24 | 8253 |
| HSV1-UL19-3655 | - | GUCGAGUUCGACGCCCUG | 18 | 8254 |
| HSV1-UL19-3656 | - | CGUCGAGUUCGACGCCCUG | 19 | 8255 |
| HSV1-UL19-1914 | - | ACGUCGAGUUCGACGCCCUG | 20 | 2299 |
| HSV1-UL19-3657 | - | GACGUCGAGUUCGACGCCCUG | 21 | 8256 |
| HSV1-UL19-3658 | - | CGACGUCGAGUUCGACGCCCUG | 22 | 8257 |
| HSV1-UL19-3659 | - | ACGACGUCGAGUUCGACGCCCUG | 23 | 8258 |
| HSV1-UL19-3660 | - | UACGACGUCGAGUUCGACGCCCUG | 24 | 8259 |
| HSV1-UL19-3661 | - | GCUCCCAACCGCGACCCU | 18 | 8260 |
| HSV1-UL19-3662 | - | CGCUCCCAACCGCGACCCU | 19 | 8261 |
| HSV1-UL19-1909 | - | CCGCUCCCAACCGCGACCCU | 20 | 2295 |
| HSV1-UL19-3663 | - | GCCGCUCCCAACCGCGACCCU | 21 | 8262 |
| HSV1-UL19-3664 | - | GGCCGCUCCCAACCGCGACCCU | 22 | 8263 |
| HSV1-UL19-3665 | - | UGGCCGCUCCCAACCGCGACCCU | 23 | 8264 |
| HSV1-UL19-3666 | - | AUGGCCGCUCCCAACCGCGACCCU | 24 | 8265 |
| HSV1-UL19-3667 | - | CAACGUCCAGGCCGUCCU | 18 | 8266 |
| HSV1-UL19-3668 | - | GCAACGUCCAGGCCGUCCU | 19 | 8267 |
| HSV1-UL19-189 | - | CGCAACGUCCAGGCCGUCCU | 20 | 575 |
| HSV1-UL19-3669 | - | UCGCAACGUCCAGGCCGUCCU | 21 | 8268 |
| HSV1-UL19-3670 | - | CUCGCAACGUCCAGGCCGUCCU | 22 | 8269 |
| HSV1-UL19-3671 | - | GCUCGCAACGUCCAGGCCGUCCU | 23 | 8270 |
| HSV1-UL19-3672 | - | CGCUCGCAACGUCCAGGCCGUCCU | 24 | 8271 |
| HSV1-UL19-3673 | - | ACGAGGGGCGCGUACAGU | 18 | 8272 |
| HSV1-UL19-3674 | - | AACGAGGGGCGCGUACAGU | 19 | 8273 |
| HSV1-UL19-1925 | - | GAACGAGGGGCGCGUACAGU | 20 | 2306 |
| HSV1-UL19-3675 | - | UGAACGAGGGGCGCGUACAGU | 21 | 8274 |
| HSV1-UL19-3676 | - | AUGAACGAGGGGCGCGUACAGU | 22 | 8275 |
| HSV1-UL19-3677 | - | CAUGAACGAGGGGCGCGUACAGU | 23 | 8276 |
| HSV1-UL19-3678 | - | ACAUGAACGAGGGGCGCGUACAGU | 24 | 8277 |
| HSV1-UL19-3679 | - | CUGUCGCUCGUGCGCUUU | 18 | 8278 |
| HSV1-UL19-3680 | - | CCUGUCGCUCGUGCGCUUU | 19 | 8279 |
| HSV1-UL19-1916 | - | CCCUGUCGCUCGUGCGCUUU | 20 | 2300 |
| HSV1-UL19-3681 | - | ACCCUGUCGCUCGUGCGCUUU | 21 | 8280 |
| HSV1-UL19-3682 | - | CACCCUGUCGCUCGUGCGCUUU | 22 | 8281 |
| HSV1-UL19-3683 | - | ACACCCUGUCGCUCGUGCGCUUU | 23 | 8282 |
| HSV1-UL19-3684 | - | AACACCCUGUCGCUCGUGCGCUUU | 24 | 8283 |
| HSV1-UL19-3685 | - | UGC GUGUGUAC CAAGUUU | 18 | 8284 |
| HSV1-UL19-3686 | - | GUGC GUGUGUAC CAAGUUU | 19 | 8285 |
| HSV1-UL19-1919 | - | CGUGCGUGUGUACCAAGUUU | 20 | 2302 |
| HSV1-UL19-3687 | - | GCGUGCGUGUGUACCAAGUUU | 21 | 8286 |
| HSV1-UL19-3688 | - | GGCGUGCGUGUGUACCAAGUUU | 22 | 8287 |
| HSV1-UL19-3689 | - | UGGCGUGCGUGUGUACCAAGUUU | 23 | 8288 |
| HSV1-UL19-3690 | - | GUGGCGUGCGUGUGUACCAAGUUU | 24 | 8289 |

**Table 6E** provides exemplary targeting domains for knocking out the *UL19* gene selected according to the fifth tier parameters. The targeting domains fall in the coding sequence of the gene, downstream of the first 500bp of coding sequence (e.g., anywhere from +500 (relative to the start codon) to the stop codon of the gene), and start with a 5'G. The PAM is NNGRRT. It is contemplated herein that in an embodiment the targeting domain hybridizes to the target domain through complementary base pairing. Any of the targeting domains in the table can be used with a S. *aureus* Cas9 molecule that generates a double stranded break (Cas9 nuclease) or a single-stranded break (Cas9 nickase).

**Table 6E**

| **5th Tier** | | | | |
|---|---|---|---|---|
| **gRNA Name** | **DNA Strand** | **Targeting Domain** | **Target Site Length** | S**eq ID NO:** |
| HSV1-UL19-3691 | + | GACGAGAUACUGCGCGAA | 18 | 8290 |
| HSV1-UL19-3692 | + | GUCAUAGACGAGAUACUGCGCGAA | 24 | 8291 |
| HSV1-UL19-1198 | + | GUUACAUGCCGCCGCGUACA | 20 | 1584 |
| HSV1-UL19-3693 | + | GGUCCAUGGCGCCCACCA | 18 | 8292 |
| HSV1-UL19-3694 | + | GUCAGGUCCAUGGCGCCCACCA | 22 | 8293 |
| HSV1-UL19-3695 | + | GUCACGACGUACGAGACCA | 19 | 8294 |
| HSV1-UL19-2719 | + | GGUCACGACGUACGAGACCA | 20 | 2798 |
| HSV1-UL19-3696 | + | GUAGGUCACGACGUACGAGACCA | 23 | 8295 |
| HSV1-UL19-3697 | + | GGUAGGUCACGACGUACGAGACCA | 24 | 8296 |
| HSV1-UL19-3698 | + | GCCACCAGAUUGGCCGGGUGCA | 22 | 8297 |
| HSV1-UL19-3699 | + | GGCCACCAGAUUGGCCGGGUGCA | 23 | 8298 |
| HSV1-UL19-3700 | + | GGUGCGCGGGGUCCGUCA | 18 | 8299 |
| HSV1-UL19-3701 | + | GGGUGCGCGGGGUCCGUCA | 19 | 8300 |
| HSV1-UL19-1233 | + | GGGGUGCGCGGGGUCCGUCA | 20 | 1619 |
| HSV1-UL19-3702 | + | GGGGGUGCGCGGGGUCCGUCA | 21 | 8301 |
| HSV1-UL19-3703 | + | GCAGGGGGUGCGCGGGGUCCGUCA | 24 | 8302 |
| HSV1-UL19-1093 | + | GUACGGAUUGGCGGCUUCGA | 20 | 1479 |
| HSV1-UL19-1271 | + | GAACAGCGCGUGGACGGGGA | 20 | 1657 |
| HSV1-UL19-3704 | + | GCGAACAGCGCGUGGACGGGGA | 22 | 8303 |
| HSV1-UL19-3705 | + | GGCGAACAGCGCGUGGACGGGGA | 23 | 8304 |
| HSV1-UL19-3706 | + | GCGGCAAACCGUUCCAUGA | 19 | 8305 |
| HSV1-UL19-3707 | + | GCGCGGCAAACCGUUCCAUGA | 21 | 8306 |
| HSV1-UL19-3708 | + | GUGCGCGGCAAACCGUUCCAUGA | 23 | 8307 |
| HSV1-UL19-3709 | + | GGUCGCUGGCGCAGGUGA | 18 | 8308 |
| HSV1-UL19-3710 | + | GGGUCGCUGGCGCAGGUGA | 19 | 8309 |
| HSV1-UL19-2883 | + | GGGGUCGCUGGCGCAGGUGA | 20 | 2904 |
| HSV1-UL19-3711 | + | GCGGGGUCGCUGGCGCAGGUGA | 22 | 8310 |
| HSV1-UL19-3712 | + | GGCGGGGUCGCUGGCGCAGGUGA | 23 | 8311 |
| HSV1-UL19-3713 | + | GGGCGGGGUCGCUGGCGCAGGUGA | 24 | 8312 |
| HSV1-UL19-3714 | + | GGUUGCCCAUGUCGGUGA | 18 | 8313 |
| HSV1-UL19-3715 | + | GAAGGUUGCCCAUGUCGGUGA | 21 | 8314 |
| HSV1-UL19-3716 | + | GGAAGGUUGCCCAUGUCGGUGA | 22 | 8315 |
| HSV1-UL19-3717 | + | GGGAAGGUUGCCCAUGUCGGUGA | 23 | 8316 |
| HSV1-UL19-3718 | + | GGGGAAGGUUGCCCAUGUCGGUGA | 24 | 8317 |
| HSV1-UL19-3719 | + | GUUGACGUCGGUCGACAC | 18 | 8318 |
| HSV1-UL19-3720 | + | GUAGUUGACGUCGGUCGACAC | 21 | 8319 |
| HSV1-UL19-3721 | + | GAAGUAGUUGACGUCGGUCGACAC | 24 | 8320 |
| HSV1-UL19-3722 | + | GCCUCCUGAAACAGGCCAC | 19 | 8321 |
| HSV1-UL19-2881 | + | GGCCUCCUGAAACAGGCCAC | 20 | 2902 |
| HSV1-UL19-3723 | + | GUAGGCCUCCUGAAACAGGCCAC | 23 | 8322 |
| HSV1-UL19-3724 | + | GGUAGGCCUCCUGAAACAGGCCAC | 24 | 8323 |
| HSV1-UL19-3725 | + | GCUUCGCUUUAGCUCGGCGAC | 21 | 8324 |
| HSV1-UL19-3726 | + | GAAGCUUCGCUUUAGCUCGGCGAC | 24 | 8325 |
| HSV1-UL19-1312 | + | GACGACGGUGAACCCAAACC | 20 | 1698 |
| HSV1-UL19-3727 | + | GGACGACGGUGAACCCAAACC | 21 | 8326 |
| HSV1-UL19-3728 | + | GUCGGACGACGGUGAACCCAAACC | 24 | 8327 |
| HSV1-UL19-1375 | + | GCCCCCGGCGGCGCGUCCCC | 20 | 1761 |
| HSV1-UL19-3729 | + | GAAAAUCGCUGCGACGCCC | 19 | 8328 |
| HSV1-UL19-3730 | + | GUACGAAAAUCGCUGCGACGCCC | 23 | 8329 |
| HSV1-UL19-3731 | + | GAAACAGGCCACACGGGUCCUCC | 23 | 8330 |
| HSV1-UL19-3732 | + | GGCGGGGCGGGAAGGCGC | 18 | 8331 |
| HSV1-UL19-3733 | + | GCUGGCGGGGCGGGAAGGCGC | 21 | 8332 |
| HSV1-UL19-3734 | + | GGCGUCCGCGGCUCGGGC | 18 | 8333 |
| HSV1-UL19-2738 | + | GCGGCGUCCGCGGCUCGGGC | 20 | 2813 |
| HSV1-UL19-3735 | + | GUUCACCACGCGCCAGGUGGC | 21 | 8334 |
| HSV1-UL19-3736 | + | GCCGUUCACCACGCGCCAGGUGGC | 24 | 8335 |
| HSV1-UL19-3737 | + | GGGCUGCAUAAACUGCUC | 18 | 8336 |
| HSV1-UL19-3738 | + | GUCGGGCUGCAUAAACUGCUC | 21 | 8337 |
| HSV1-UL19-3739 | + | GUUGUCGGGCUGCAUAAACUGCUC | 24 | 8338 |
| HSV1-UL19-3740 | + | GAUGCUGGGGGGCCAUCAG | 19 | 8339 |
| HSV1-UL19-3741 | + | GUCCAGAUGCUGGGGGGCCAUCAG | 24 | 8340 |
| HSV1-UL19-3742 | + | GCGUCACAGUCCCACACG | 18 | 8341 |
| HSV1-UL19-3743 | + | GGCGUCACAGUCCCACACG | 19 | 8342 |
| HSV1-UL19-1187 | + | GGGCGUCACAGUCCCACACG | 20 | 1573 |
| HSV1-UL19-3744 | + | GCAGCAGGUAAAACACCG | 18 | 8343 |
| HSV1-UL19-3745 | + | GCCUGCAGCAGGUAAAACACCG | 22 | 8344 |
| HSV1-UL19-3746 | + | GGCCUGCAGCAGGUAAAACACCG | 23 | 8345 |
| HSV1-UL19-3747 | + | GAUUCCGGCGUGCAACGCCCCG | 22 | 8346 |
| HSV1-UL19-3748 | + | GGAUUCCGGCGUGCAACGCCCCG | 23 | 8347 |
| HSV1-UL19-3749 | + | GCGUCAACGUUCAUCAGCG | 19 | 8348 |
| HSV1-UL19-3750 | + | GCCGCGUCAACGUUCAUCAGCG | 22 | 8349 |
| HSV1-UL19-3751 | + | GCCGGGUGCAGGGGGUGCG | 19 | 8350 |
| HSV1-UL19-1236 | + | GGCCGGGUGCAGGGGGUGCG | 20 | 1622 |
| HSV1-UL19-3752 | + | GAUUGGCCGGGUGCAGGGGGUGCG | 24 | 8351 |
| HSV1-UL19-3753 | + | GUCCACGUUCGCGCGCGG | 18 | 8352 |
| HSV1-UL19-3754 | + | GGUCCACGUUCGCGCGCGG | 19 | 8353 |
| HSV1-UL19-3755 | + | GCCCAGGUCCACGUUCGCGCGCGG | 24 | 8354 |
| HSV1-UL19-3756 | + | GGGCACUGCGGAGGAGGG | 18 | 8355 |
| HSV1-UL19-1428 | + | GCGGGCACUGCGGAGGAGGG | 20 | 1814 |
| HSV1-UL19-3757 | + | GUUGCGGGCACUGCGGAGGAGGG | 23 | 8356 |
| HSV1-UL19-3758 | + | GUGGCCGCGAAGGCCCGGG | 19 | 8357 |
| HSV1-UL19-3759 | + | GCCGUGGCCGCGAAGGCCCGGG | 22 | 8358 |
| HSV1-UL19-3760 | + | GGCCGUGGCCGCGAAGGCCCGGG | 23 | 8359 |
| HSV1-UL19-3761 | + | GUGUUGGCCACCAGAUUGG | 19 | 8360 |
| HSV1-UL19-3762 | + | GACCGUGUUGGCCACCAGAUUGG | 23 | 8361 |
| HSV1-UL19-3245 | + | GACGUUGCGAGCGAGCUG | 18 | 7844 |
| HSV1-UL19-3246 | + | GGACGUUGCGAGCGAGCUG | 19 | 7845 |
| HSV1-UL19-3247 | + | GCCUGGACGUUGCGAGCGAGCUG | 23 | 7846 |
| HSV1-UL19-3248 | + | GGCCUGGACGUUGCGAGCGAGCUG | 24 | 7847 |
| HSV1-UL19-3763 | + | GUGCUGCACGACGGGCUG | 18 | 8362 |
| HSV1-UL19-3764 | + | GACGUGCUGCACGACGGGCUG | 21 | 8363 |
| HSV1-UL19-3765 | + | GGACGUGCUGCACGACGGGCUG | 22 | 8364 |
| HSV1-UL19-3766 | + | GCGGACGUGCUGCACGACGGGCUG | 24 | 8365 |
| HSV1-UL19-3767 | + | GAUCUCCGGGACCACCAUGUUCUG | 24 | 8366 |
| HSV1-UL19-3768 | + | GCGGGAAGGCGCGGGGAU | 18 | 8367 |
| HSV1-UL19-3769 | + | GGCGGGAAGGCGCGGGGAU | 19 | 8368 |
| HSV1-UL19-2665 | + | GGGCGGGAAGGCGCGGGGAU | 20 | 2762 |
| HSV1-UL19-3770 | + | GGGGCGGGAAGGCGCGGGGAU | 21 | 8369 |
| HSV1-UL19-3771 | + | GCGGGGCGGGAAGGCGCGGGGAU | 23 | 8370 |
| HSV1-UL19-3772 | + | GGCGGGGCGGGAAGGCGCGGGGAU | 24 | 8371 |
| HSV1-UL19-3773 | + | GCCGCCACGUACGCCCCGU | 19 | 8372 |
| HSV1-UL19-2679 | + | GGCCGCCACGUACGCCCCGU | 20 | 2770 |
| HSV1-UL19-3774 | + | GGGCCGCCACGUACGCCCCGU | 21 | 8373 |
| HSV1-UL19-3775 | + | GGGGCCGCCACGUACGCCCCGU | 22 | 8374 |
| HSV1-UL19-3776 | + | GCCCAGCUCCGGGCCGGU | 18 | 8375 |
| HSV1-UL19-3777 | + | GCCCGCCCAGCUCCGGGCCGGU | 22 | 8376 |
| HSV1-UL19-3778 | + | GUAAAAAUAGUCGCCAUU | 18 | 8377 |
| HSV1-UL19-3779 | + | GGUAAAAAUAGUCGCCAUU | 19 | 8378 |
| HSV1-UL19-2775 | + | GGGUAAAAAUAGUCGCCAUU | 20 | 2839 |
| HSV1-UL19-3780 | + | GGGGUAAAAAUAGUCGCCAUU | 21 | 8379 |
| HSV1-UL19-3781 | + | GGGGGUAAAAAUAGUCGCCAUU | 22 | 8380 |
| HSV1-UL19-3782 | + | GAGGGGGUAAAAAUAGUCGCCAUU | 24 | 8381 |
| HSV1-UL19-3783 | - | GCGCGGGCGGCCGGUCGA | 18 | 8382 |
| HSV1-UL19-3784 | - | GACACGCGCGGGCGGCCGGUCGA | 23 | 8383 |
| HSV1-UL19-3785 | - | GUCCCCGCCCCCGGCCAC | 18 | 8384 |
| HSV1-UL19-3786 | - | GACCUAGUCCCCGCCCCCGGCCAC | 24 | 8385 |
| HSV1-UL19-2044 | - | GACAACGGCCGCCUGGCCAC | 20 | 2388 |
| HSV1-UL19-3787 | - | GGACAACGGCCGCCUGGCCAC | 21 | 8386 |
| HSV1-UL19-3788 | - | GUACAGUUUAAGCGCCCCC | 19 | 8387 |
| HSV1-UL19-3789 | - | GACGUACAGUUUAAGCGCCCCC | 22 | 8388 |
| HSV1-UL19-3790 | - | GCGACGUACAGUUUAAGCGCCCCC | 24 | 8389 |
| HSV1-UL19-3791 | - | GCCCGAGCCGCGGACGCC | 18 | 8390 |
| HSV1-UL19-3792 | - | GGCCCGAGCCGCGGACGCC | 19 | 8391 |
| HSV1-UL19-3793 | - | GGGCCGCUGCUGCACCGC | 18 | 8392 |
| HSV1-UL19-3794 | - | GGGGCCGCUGCUGCACCGC | 19 | 8393 |
| HSV1-UL19-3795 | - | GCGGGGCCGCUGCUGCACCGC | 21 | 8394 |
| HSV1-UL19-519 | - | GCCCAGGUGCCGCGGCGCGC | 20 | 905 |
| HSV1-UL19-3796 | - | GCGCCCAGGUGCCGCGGCGCGC | 22 | 8395 |
| HSV1-UL19-3797 | - | GUGCGCCCAGGUGCCGCGGCGCGC | 24 | 8396 |
| HSV1-UL19-3798 | - | GCCCCCCAGACGACGCGCGUGCGC | 24 | 8397 |
| HSV1-UL19-2239 | - | GCUCAAGACGGGCCUCCAUC | 20 | 2508 |
| HSV1-UL19-3799 | - | GGGCAGGCGCAAGCCGAG | 18 | 8398 |
| HSV1-UL19-2181 | - | GCGGGCAGGCGCAAGCCGAG | 20 | 2471 |
| HSV1-UL19-3800 | - | GGCGGGCAGGCGCAAGCCGAG | 21 | 8399 |
| HSV1-UL19-3801 | - | GGGCGGGCAGGCGCAAGCCGAG | 22 | 8400 |
| HSV1-UL19-3802 | - | GUUUACGCGGGGGACAAGGAG | 21 | 8401 |
| HSV1-UL19-3803 | - | GCGUUUACGCGGGGGACAAGGAG | 23 | 8402 |
| HSV1-UL19-3804 | - | GGCGUUUACGCGGGGGACAAGGAG | 24 | 8403 |
| HSV1-UL19-3805 | - | GAGCGCCUGAUCGUGGAG | 18 | 8404 |
| HSV1-UL19-3806 | - | GGAGCGCCUGAUCGUGGAG | 19 | 8405 |
| HSV1-UL19-3807 | - | GCCUGGAGCGCCUGAUCGUGGAG | 23 | 8406 |
| HSV1-UL19-3808 | - | GGCCCACGGGCGGGUCCG | 18 | 8407 |
| HSV1-UL19-3809 | - | GCCUGGCCCACGGGCGGGUCCG | 22 | 8408 |
| HSV1-UL19-3810 | - | GCGCCUGGCCCACGGGCGGGUCCG | 24 | 8409 |
| HSV1-UL19-3811 | - | GUCCCGGAGAUCGCCCCCGGCG | 22 | 8410 |
| HSV1-UL19-3812 | - | GGUCCCGGAGAUCGCCCCCGGCG | 23 | 8411 |
| HSV1-UL19-3813 | - | GCAGCGCCUGGCCCACGG | 18 | 8412 |
| HSV1-UL19-3814 | - | GGCAGCGCCUGGCCCACGG | 19 | 8413 |
| HSV1-UL19-2123 | - | GGGCAGCGCCUGGCCCACGG | 20 | 2443 |
| HSV1-UL19-3815 | - | GGGGCAGCGCCUGGCCCACGG | 21 | 8414 |
| HSV1-UL19-3816 | - | GGGGGCAGCGCCUGGCCCACGG | 22 | 8415 |
| HSV1-UL19-487 | - | GCCCGGCACGAAACUGGCGG | 20 | 873 |
| HSV1-UL19-3817 | - | GGCCCGGCACGAAACUGGCGG | 21 | 8416 |
| HSV1-UL19-3818 | - | GUGGCCCGGCACGAAACUGGCGG | 23 | 8417 |
| HSV1-UL19-3819 | - | GGUGGCCCGGCACGAAACUGGCGG | 24 | 8418 |
| HSV1-UL19-464 | - | GCUGACCUACGCGCUCAUGG | 20 | 850 |
| HSV1-UL19-3820 | - | GCGCUGACCUACGCGCUCAUGG | 22 | 8419 |
| HSV1-UL19-3821 | - | GGACCGCCAUCGCGACUG | 18 | 8420 |
| HSV1-UL19-3822 | - | GCCCUGGACCGCCAUCGCGACUG | 23 | 8421 |
| HSV1-UL19-3823 | - | GGCCCUGGACCGCCAUCGCGACUG | 24 | 8422 |
| HSV1-UL19-3824 | - | GUAAUAAACCGCCCCGCCCUUCCU | 24 | 8423 |
| HSV1-UL19-338 | - | GACGCCCGGGGCCUGGAGCU | 20 | 724 |
| HSV1-UL19-3825 | - | GGACGCCCGGGGCCUGGAGCU | 21 | 8424 |
| HSV1-UL19-3826 | - | GGGACGCCCGGGGCCUGGAGCU | 22 | 8425 |
| HSV1-UL19-2240 | - | GACGGGCCUCCAUCCCGGGU | 20 | 2509 |
| HSV1-UL19-3827 | - | GCCCAGCCCGUCCCCGUGU | 19 | 8426 |
| HSV1-UL19-2271 | - | GGCCCAGCCCGUCCCCGUGU | 20 | 2529 |
| HSV1-UL19-3828 | - | GCCGGCCCAGCCCGUCCCCGUGU | 23 | 8427 |
| HSV1-UL19-3829 | - | GGCCGGCCCAGCCCGUCCCCGUGU | 24 | 8428 |
| HSV1-UL19-3830 | - | GACCACGGCCAGGACGCCGUGU | 22 | 8429 |
| HSV1-UL19-3831 | - | GGACCACGGCCAGGACGCCGUGU | 23 | 8430 |
| HSV1-UL19-3832 | - | GCACCCCGCGUUCGACUUCUUUGU | 24 | 8431 |

**Table 6F** provides exemplary targeting domains for knocking out the *UL19* gene selected according to the six tier parameters. The targeting domains fall in the coding sequence of the gene, downstream of the first 500bp of coding sequence (e.g., anywhere from +500 (relative to the start codon) to the stop codon of the gene). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 6F**

| **6th Tier** | | | | |
|---|---|---|---|---|
| **gRNA Name** | **DNA Strand** | **Targeting Domain** | **Target Site Length** | **Seq ID NO:** |
| HSV1-UL19-3833 | + | AGACGAGAUACUGCGCGAA | 19 | 8432 |
| HSV1-UL19-2894 | + | UAGACGAGAUACUGCGCGAA | 20 | 2912 |
| HSV1-UL19-3834 | + | AUAGACGAGAUACUGCGCGAA | 21 | 8433 |
| HSV1-UL19-3835 | + | CAUAGACGAGAUACUGCGCGAA | 22 | 8434 |
| HSV1-UL19-3836 | + | UCAUAGACGAGAUACUGCGCGAA | 23 | 8435 |
| HSV1-UL19-3837 | + | UACAUGCCGCCGCGUACA | 18 | 8436 |
| HSV1-UL19-3838 | + | UUACAUGCCGCCGCGUACA | 19 | 8437 |
| HSV1-UL19-3839 | + | CGUUACAUGCCGCCGCGUACA | 21 | 8438 |
| HSV1-UL19-3840 | + | ACGUUACAUGCCGCCGCGUACA | 22 | 8439 |
| HSV1-UL19-3841 | + | CACGUUACAUGCCGCCGCGUACA | 23 | 8440 |
| HSV1-UL19-3842 | + | CCACGUUACAUGCCGCCGCGUACA | 24 | 8441 |
| HSV1-UL19-3843 | + | AGGUCCAUGGCGCCCACCA | 19 | 8442 |
| HSV1-UL19-1049 | + | CAGGUCCAUGGCGCCCACCA | 20 | 1435 |
| HSV1-UL19-3844 | + | UCAGGUCCAUGGCGCCCACCA | 21 | 8443 |
| HSV1-UL19-3845 | + | CGUCAGGUCCAUGGCGCCCACCA | 23 | 8444 |
| HSV1-UL19-3846 | + | ACGUCAGGUCCAUGGCGCCCACCA | 24 | 8445 |
| HSV1-UL19-3847 | + | UCACGACGUACGAGACCA | 18 | 8446 |
| HSV1-UL19-3848 | + | AGGUCACGACGUACGAGACCA | 21 | 8447 |
| HSV1-UL19-3849 | + | UAGGUCACGACGUACGAGACCA | 22 | 8448 |
| HSV1-UL19-3850 | + | CCAGAUUGGCCGGGUGCA | 18 | 8449 |
| HSV1-UL19-3851 | + | ACCAGAUUGGCCGGGUGCA | 19 | 8450 |
| HSV1-UL19-1239 | + | CACCAGAUUGGCCGGGUGCA | 20 | 1625 |
| HSV1-UL19-3852 | + | CCACCAGAUUGGCCGGGUGCA | 21 | 8451 |
| HSV1-UL19-3853 | + | UGGCCACCAGAUUGGCCGGGUGCA | 24 | 8452 |
| HSV1-UL19-3854 | + | AGGGGGUGCGCGGGGUCCGUCA | 22 | 8453 |
| HSV1-UL19-3855 | + | CAGGGGGUGCGCGGGGUCCGUCA | 23 | 8454 |
| HSV1-UL19-3856 | + | ACGGAUUGGCGGCUUCGA | 18 | 8455 |
| HSV1-UL19-3857 | + | UACGGAUUGGCGGCUUCGA | 19 | 8456 |
| HSV1-UL19-3858 | + | CGUACGGAUUGGCGGCUUCGA | 21 | 8457 |
| HSV1-UL19-3859 | + | CCGUACGGAUUGGCGGCUUCGA | 22 | 8458 |
| HSV1-UL19-3860 | + | CCCGUACGGAUUGGCGGCUUCGA | 23 | 8459 |
| HSV1-UL19-3861 | + | CCCCGUACGGAUUGGCGGCUUCGA | 24 | 8460 |
| HSV1-UL19-3862 | + | ACAGCGCGUGGACGGGGA | 18 | 8461 |
| HSV1-UL19-3863 | + | AACAGCGCGUGGACGGGGA | 19 | 8462 |
| HSV1-UL19-3864 | + | CGAACAGCGCGUGGACGGGGA | 21 | 8463 |
| HSV1-UL19-3865 | + | CGGCGAACAGCGCGUGGACGGGGA | 24 | 8464 |
| HSV1-UL19-3866 | + | CGGCAAACCGUUCCAUGA | 18 | 8465 |
| HSV1-UL19-2658 | + | CGCGGCAAACCGUUCCAUGA | 20 | 2759 |
| HSV1-UL19-3867 | + | UGCGCGGCAAACCGUUCCAUGA | 22 | 8466 |
| HSV1-UL19-3868 | + | CGUGCGCGGCAAACCGUUCCAUGA | 24 | 8467 |
| HSV1-UL19-3869 | + | CGGGGUCGCUGGCGCAGGUGA | 21 | 8468 |
| HSV1-UL19-3870 | + | AGGUUGCCCAUGUCGGUGA | 19 | 8469 |
| HSV1-UL19-1332 | + | AAGGUUGCCCAUGUCGGUGA | 20 | 1718 |
| HSV1-UL19-3871 | + | AGUUGACGUCGGUCGACAC | 19 | 8470 |
| HSV1-UL19-1366 | + | UAGUUGACGUCGGUCGACAC | 20 | 1752 |
| HSV1-UL19-3872 | + | AGUAGUUGACGUCGGUCGACAC | 22 | 8471 |
| HSV1-UL19-3873 | + | AAGUAGUUGACGUCGGUCGACAC | 23 | 8472 |
| HSV1-UL19-3874 | + | CCUCCUGAAACAGGCCAC | 18 | 8473 |
| HSV1-UL19-3875 | + | AGGCCUCCUGAAACAGGCCAC | 21 | 8474 |
| HSV1-UL19-3876 | + | UAGGCCUCCUGAAACAGGCCAC | 22 | 8475 |
| HSV1-UL19-3877 | + | UCGCUUUAGCUCGGCGAC | 18 | 8476 |
| HSV1-UL19-3878 | + | UUCGCUUUAGCUCGGCGAC | 19 | 8477 |
| HSV1-UL19-2617 | + | CUUCGCUUUAGCUCGGCGAC | 20 | 2732 |
| HSV1-UL19-3879 | + | AGCUUCGCUUUAGCUCGGCGAC | 22 | 8478 |
| HSV1-UL19-3880 | + | AAGCUUCGCUUUAGCUCGGCGAC | 23 | 8479 |
| HSV1-UL19-3881 | + | CGACGGUGAACCCAAACC | 18 | 8480 |
| HSV1-UL19-3882 | + | ACGACGGUGAACCCAAACC | 19 | 8481 |
| HSV1-UL19-3883 | + | CGGACGACGGUGAACCCAAACC | 22 | 8482 |
| HSV1-UL19-3884 | + | UCGGACGACGGUGAACCCAAACC | 23 | 8483 |
| HSV1-UL19-3885 | + | CCCCGGCGGCGCGUCCCC | 18 | 8484 |
| HSV1-UL19-3886 | + | CCCCCGGCGGCGCGUCCCC | 19 | 8485 |
| HSV1-UL19-3887 | + | CGCCCCCGGCGGCGCGUCCCC | 21 | 8486 |
| HSV1-UL19-3888 | + | ACGCCCCCGGCGGCGCGUCCCC | 22 | 8487 |
| HSV1-UL19-3889 | + | AACGCCCCCGGCGGCGCGUCCCC | 23 | 8488 |
| HSV1-UL19-3890 | + | AAACGCCCCCGGCGGCGCGUCCCC | 24 | 8489 |
| HSV1-UL19-3891 | + | AAAAUCGCUGCGACGCCC | 18 | 8490 |
| HSV1-UL19-2865 | + | CGAAAAUCGCUGCGACGCCC | 20 | 2890 |
| HSV1-UL19-3892 | + | ACGAAAAUCGCUGCGACGCCC | 21 | 8491 |
| HSV1-UL19-3893 | + | UACGAAAAUCGCUGCGACGCCC | 22 | 8492 |
| HSV1-UL19-3894 | + | CGUACGAAAAUCGCUGCGACGCCC | 24 | 8493 |
| HSV1-UL19-3895 | + | AGGCCACACGGGUCCUCC | 18 | 8494 |
| HSV1-UL19-3896 | + | CAGGCCACACGGGUCCUCC | 19 | 8495 |
| HSV1-UL19-2880 | + | ACAGGCCACACGGGUCCUCC | 20 | 2901 |
| HSV1-UL19-3897 | + | AACAGGCCACACGGGUCCUCC | 21 | 8496 |
| HSV1-UL19-3898 | + | AAACAGGCCACACGGGUCCUCC | 22 | 8497 |
| HSV1-UL19-3899 | + | UGAAACAGGCCACACGGGUCCUCC | 24 | 8498 |
| HSV1-UL19-3900 | + | AGAGCCAGUCCCUUGAGC | 18 | 8499 |
| HSV1-UL19-3901 | + | CAGAGCCAGUCCCUUGAGC | 19 | 8500 |
| HSV1-UL19-1440 | + | ACAGAGCCAGUCCCUUGAGC | 20 | 1826 |
| HSV1-UL19-3902 | + | UACAGAGCCAGUCCCUUGAGC | 21 | 8501 |
| HSV1-UL19-3903 | + | UUACAGAGCCAGUCCCUUGAGC | 22 | 8502 |
| HSV1-UL19-3904 | + | AUUACAGAGCCAGUCCCUUGAGC | 23 | 8503 |
| HSV1-UL19-3905 | + | UAUUACAGAGCCAGUCCCUUGAGC | 24 | 8504 |
| HSV1-UL19-3906 | + | UGGCGGGGCGGGAAGGCGC | 19 | 8505 |
| HSV1-UL19-1073 | + | CUGGCGGGGCGGGAAGGCGC | 20 | 1459 |
| HSV1-UL19-3907 | + | AGCUGGCGGGGCGGGAAGGCGC | 22 | 8506 |
| HSV1-UL19-3908 | + | CAGCUGGCGGGGCGGGAAGGCGC | 23 | 8507 |
| HSV1-UL19-3909 | + | ACAGCUGGCGGGGCGGGAAGGCGC | 24 | 8508 |
| HSV1-UL19-3910 | + | CGGCGUCCGCGGCUCGGGC | 19 | 8509 |
| HSV1-UL19-3911 | + | CGCGGCGUCCGCGGCUCGGGC | 21 | 8510 |
| HSV1-UL19-3912 | + | CCGCGGCGUCCGCGGCUCGGGC | 22 | 8511 |
| HSV1-UL19-3913 | + | UCCGCGGCGUCCGCGGCUCGGGC | 23 | 8512 |
| HSV1-UL19-3914 | + | AUCCGCGGCGUCCGCGGCUCGGGC | 24 | 8513 |
| HSV1-UL19-3915 | + | CACCACGCGCCAGGUGGC | 18 | 8514 |
| HSV1-UL19-3916 | + | UCACCACGCGCCAGGUGGC | 19 | 8515 |
| HSV1-UL19-2700 | + | UUCACCACGCGCCAGGUGGC | 20 | 2783 |
| HSV1-UL19-3917 | + | CGUUCACCACGCGCCAGGUGGC | 22 | 8516 |
| HSV1-UL19-3918 | + | CCGUUCACCACGCGCCAGGUGGC | 23 | 8517 |
| HSV1-UL19-3919 | + | CGGGCUGCAUAAACUGCUC | 19 | 8518 |
| HSV1-UL19-1113 | + | UCGGGCUGCAUAAACUGCUC | 20 | 1499 |
| HSV1-UL19-3920 | + | UGUCGGGCUGCAUAAACUGCUC | 22 | 8519 |
| HSV1-UL19-3921 | + | UUGUCGGGCUGCAUAAACUGCUC | 23 | 8520 |
| HSV1-UL19-3922 | + | AUGCUGGGGGGCCAUCAG | 18 | 8521 |
| HSV1-UL19-2770 | + | AGAUGCUGGGGGGCCAUCAG | 20 | 2837 |
| HSV1-UL19-3923 | + | CAGAUGCUGGGGGGCCAUCAG | 21 | 8522 |
| HSV1-UL19-3924 | + | CCAGAUGCUGGGGGGCCAUCAG | 22 | 8523 |
| HSV1-UL19-3925 | + | UCCAGAUGCUGGGGGGCCAUCAG | 23 | 8524 |
| HSV1-UL19-3926 | + | CCACAAAGAAGUCGAACG | 18 | 8525 |
| HSV1-UL19-3927 | + | CCCACAAAGAAGUCGAACG | 19 | 8526 |
| HSV1-UL19-1120 | + | CCCCACAAAGAAGUCGAACG | 20 | 1506 |
| HSV1-UL19-3928 | + | CCCCCACAAAGAAGUCGAACG | 21 | 8527 |
| HSV1-UL19-3929 | + | ACCCCCACAAAGAAGUCGAACG | 22 | 8528 |
| HSV1-UL19-3930 | + | CACCCCCACAAAGAAGUCGAACG | 23 | 8529 |
| HSV1-UL19-3931 | + | CCACCCCCACAAAGAAGUCGAACG | 24 | 8530 |
| HSV1-UL19-3932 | + | AGGGCGUCACAGUCCCACACG | 21 | 8531 |
| HSV1-UL19-3933 | + | CAGGGCGUCACAGUCCCACACG | 22 | 8532 |
| HSV1-UL19-3934 | + | UCAGGGCGUCACAGUCCCACACG | 23 | 8533 |
| HSV1-UL19-3935 | + | AUCAGGGCGUCACAGUCCCACACG | 24 | 8534 |
| HSV1-UL19-3936 | + | UGCAGCAGGUAAAACACCG | 19 | 8535 |
| HSV1-UL19-2712 | + | CUGCAGCAGGUAAAACACCG | 20 | 2791 |
| HSV1-UL19-3937 | + | CCUGCAGCAGGUAAAACACCG | 21 | 8536 |
| HSV1-UL19-3938 | + | CGGCCUGCAGCAGGUAAAACACCG | 24 | 8537 |
| HSV1-UL19-3939 | + | CCGGCGUGCAACGCCCCG | 18 | 8538 |
| HSV1-UL19-3940 | + | UCCGGCGUGCAACGCCCCG | 19 | 8539 |
| HSV1-UL19-2769 | + | UUCCGGCGUGCAACGCCCCG | 20 | 2836 |
| HSV1-UL19-3941 | + | AUUCCGGCGUGCAACGCCCCG | 21 | 8540 |
| HSV1-UL19-3942 | + | AGGAUUCCGGCGUGCAACGCCCCG | 24 | 8541 |
| HSV1-UL19-3943 | + | CGAGGGGUGGCAGCAGCG | 18 | 8542 |
| HSV1-UL19-3944 | + | ACGAGGGGUGGCAGCAGCG | 19 | 8543 |
| HSV1-UL19-2729 | + | CACGAGGGGUGGCAGCAGCG | 20 | 2806 |
| HSV1-UL19-3945 | + | ACACGAGGGGUGGCAGCAGCG | 21 | 8544 |
| HSV1-UL19-3946 | + | CACACGAGGGGUGGCAGCAGCG | 22 | 8545 |
| HSV1-UL19-3947 | + | CCACACGAGGGGUGGCAGCAGCG | 23 | 8546 |
| HSV1-UL19-3948 | + | CCCACACGAGGGGUGGCAGCAGCG | 24 | 8547 |
| HSV1-UL19-3949 | + | CGUCAACGUUCAUCAGCG | 18 | 8548 |
| HSV1-UL19-2675 | + | CGCGUCAACGUUCAUCAGCG | 20 | 2767 |
| HSV1-UL19-3950 | + | CCGCGUCAACGUUCAUCAGCG | 21 | 8549 |
| HSV1-UL19-3951 | + | CGCCGCGUCAACGUUCAUCAGCG | 23 | 8550 |
| HSV1-UL19-3952 | + | CCGCCGCGUCAACGUUCAUCAGCG | 24 | 8551 |
| HSV1-UL19-3953 | + | CCGGGUGCAGGGGGUGCG | 18 | 8552 |
| HSV1-UL19-3954 | + | UGGCCGGGUGCAGGGGGUGCG | 21 | 8553 |
| HSV1-UL19-3955 | + | UUGGCCGGGUGCAGGGGGUGCG | 22 | 8554 |
| HSV1-UL19-3956 | + | AUUGGCCGGGUGCAGGGGGUGCG | 23 | 8555 |
| HSV1-UL19-2827 | + | AGGUCCACGUUCGCGCGCGG | 20 | 2869 |
| HSV1-UL19-3957 | + | CAGGUCCACGUUCGCGCGCGG | 21 | 8556 |
| HSV1-UL19-3958 | + | CCAGGUCCACGUUCGCGCGCGG | 22 | 8557 |
| HSV1-UL19-3959 | + | CCCAGGUCCACGUUCGCGCGCGG | 23 | 8558 |
| HSV1-UL19-3960 | + | CGGGCACUGCGGAGGAGGG | 19 | 8559 |
| HSV1-UL19-3961 | + | UGCGGGCACUGCGGAGGAGGG | 21 | 8560 |
| HSV1-UL19-3962 | + | UUGCGGGCACUGCGGAGGAGGG | 22 | 8561 |
| HSV1-UL19-3963 | + | CGUUGCGGGCACUGCGGAGGAGGG | 24 | 8562 |
| HSV1-UL19-3964 | + | UGGCCGCGAAGGCCCGGG | 18 | 8563 |
| HSV1-UL19-2861 | + | CGUGGCCGCGAAGGCCCGGG | 20 | 2887 |
| HSV1-UL19-3965 | + | CCGUGGCCGCGAAGGCCCGGG | 21 | 8564 |
| HSV1-UL19-3966 | + | UGGCCGUGGCCGCGAAGGCCCGGG | 24 | 8565 |
| HSV1-UL19-3967 | + | UGUUGGCCACCAGAUUGG | 18 | 8566 |
| HSV1-UL19-2760 | + | CGUGUUGGCCACCAGAUUGG | 20 | 2827 |
| HSV1-UL19-3968 | + | CCGUGUUGGCCACCAGAUUGG | 21 | 8567 |
| HSV1-UL19-3969 | + | ACCGUGUUGGCCACCAGAUUGG | 22 | 8568 |
| HSV1-UL19-3970 | + | UGACCGUGUUGGCCACCAGAUUGG | 24 | 8569 |
| HSV1-UL19-3272 | + | UGGACGUUGCGAGCGAGCUG | 20 | 7871 |
| HSV1-UL19-3273 | + | CUGGACGUUGCGAGCGAGCUG | 21 | 7872 |
| HSV1-UL19-3274 | + | CCUGGACGUUGCGAGCGAGCUG | 22 | 7873 |
| HSV1-UL19-3971 | + | CGUGCUGCACGACGGGCUG | 19 | 8570 |
| HSV1-UL19-2804 | + | ACGUGCUGCACGACGGGCUG | 20 | 2851 |
| HSV1-UL19-3972 | + | CGGACGUGCUGCACGACGGGCUG | 23 | 8571 |
| HSV1-UL19-3973 | + | CGGGACCACCAUGUUCUG | 18 | 8572 |
| HSV1-UL19-3974 | + | CCGGGACCACCAUGUUCUG | 19 | 8573 |
| HSV1-UL19-2745 | + | UCCGGGACCACCAUGUUCUG | 20 | 2820 |
| HSV1-UL19-3975 | + | CUCCGGGACCACCAUGUUCUG | 21 | 8574 |
| HSV1-UL19-3976 | + | UCUCCGGGACCACCAUGUUCUG | 22 | 8575 |
| HSV1-UL19-3977 | + | AUCUCCGGGACCACCAUGUUCUG | 23 | 8576 |
| HSV1-UL19-3978 | + | CGGGGCGGGAAGGCGCGGGGAU | 22 | 8577 |
| HSV1-UL19-3979 | + | CCGCCACGUACGCCCCGU | 18 | 8578 |
| HSV1-UL19-3980 | + | CGGGGCCGCCACGUACGCCCCGU | 23 | 8579 |
| HSV1-UL19-3981 | + | CCGGGGCCGCCACGUACGCCCCGU | 24 | 8580 |
| HSV1-UL19-3982 | + | CGCCCAGCUCCGGGCCGGU | 19 | 8581 |
| HSV1-UL19-2727 | + | CCGCCCAGCUCCGGGCCGGU | 20 | 2804 |
| HSV1-UL19-3983 | + | CCCGCCCAGCUCCGGGCCGGU | 21 | 8582 |
| HSV1-UL19-3984 | + | UGCCCGCCCAGCUCCGGGCCGGU | 23 | 8583 |
| HSV1-UL19-3985 | + | CUGCCCGCCCAGCUCCGGGCCGGU | 24 | 8584 |
| HSV1-UL19-3986 | + | AGGGGGUAAAAAUAGUCGCCAUU | 23 | 8585 |
| HSV1-UL19-3987 | - | CGCGCGGGCGGCCGGUCGA | 19 | 8586 |
| HSV1-UL19-223 | - | ACGCGCGGGCGGCCGGUCGA | 20 | 609 |
| HSV1-UL19-3988 | - | CACGCGCGGGCGGCCGGUCGA | 21 | 8587 |
| HSV1-UL19-3989 | - | ACACGCGCGGGCGGCCGGUCGA | 22 | 8588 |
| HSV1-UL19-3990 | - | CGACACGCGCGGGCGGCCGGUCGA | 24 | 8589 |
| HSV1-UL19-3991 | - | AGUCCCCGCCCCCGGCCAC | 19 | 8590 |
| HSV1-UL19-2100 | - | UAGUCCCCGCCCCCGGCCAC | 20 | 2431 |
| HSV1-UL19-3992 | - | CUAGUCCCCGCCCCCGGCCAC | 21 | 8591 |
| HSV1-UL19-3993 | - | CCUAGUCCCCGCCCCCGGCCAC | 22 | 8592 |
| HSV1-UL19-3994 | - | ACCUAGUCCCCGCCCCCGGCCAC | 23 | 8593 |
| HSV1-UL19-3995 | - | CAACGGCCGCCUGGCCAC | 18 | 8594 |
| HSV1-UL19-3996 | - | ACAACGGCCGCCUGGCCAC | 19 | 8595 |
| HSV1-UL19-3997 | - | UGGACAACGGCCGCCUGGCCAC | 22 | 8596 |
| HSV1-UL19-3998 | - | CUGGACAACGGCCGCCUGGCCAC | 23 | 8597 |
| HSV1-UL19-3999 | - | CCUGGACAACGGCCGCCUGGCCAC | 24 | 8598 |
| HSV1-UL19-4000 | - | UACAGUUUAAGCGCCCCC | 18 | 8599 |
| HSV1-UL19-566 | - | CGUACAGUUUAAGCGCCCCC | 20 | 952 |
| HSV1-UL19-4001 | - | ACGUACAGUUUAAGCGCCCCC | 21 | 8600 |
| HSV1-UL19-4002 | - | CGACGUACAGUUUAAGCGCCCCC | 23 | 8601 |
| HSV1-UL19-2194 | - | AGGCCCGAGCCGCGGACGCC | 20 | 2480 |
| HSV1-UL19-4003 | - | CAGGCCCGAGCCGCGGACGCC | 21 | 8602 |
| HSV1-UL19-4004 | - | CCAGGCCCGAGCCGCGGACGCC | 22 | 8603 |
| HSV1-UL19-4005 | - | CCCAGGCCCGAGCCGCGGACGCC | 23 | 8604 |
| HSV1-UL19-4006 | - | ACCCAGGCCCGAGCCGCGGACGCC | 24 | 8605 |
| HSV1-UL19-4007 | - | CGACGGGGCGUUGCACGC | 18 | 8606 |
| HSV1-UL19-4008 | - | UCGACGGGGCGUUGCACGC | 19 | 8607 |
| HSV1-UL19-442 | - | UUCGACGGGGCGUUGCACGC | 20 | 828 |
| HSV1-UL19-4009 | - | AUUCGACGGGGCGUUGCACGC | 21 | 8608 |
| HSV1-UL19-4010 | - | UAUUCGACGGGGCGUUGCACGC | 22 | 8609 |
| HSV1-UL19-4011 | - | AUAUUCGACGGGGCGUUGCACGC | 23 | 8610 |
| HSV1-UL19-4012 | - | CAUAUUCGACGGGGCGUUGCACGC | 24 | 8611 |
| HSV1-UL19-412 | - | CGGGGCCGCUGCUGCACCGC | 20 | 798 |
| HSV1-UL19-4013 | - | CGCGGGGCCGCUGCUGCACCGC | 22 | 8612 |
| HSV1-UL19-4014 | - | UCGCGGGGCCGCUGCUGCACCGC | 23 | 8613 |
| HSV1-UL19-4015 | - | CUCGCGGGGCCGCUGCUGCACCGC | 24 | 8614 |
| HSV1-UL19-4016 | - | CCAGGUGCCGCGGCGCGC | 18 | 8615 |
| HSV1-UL19-4017 | - | CCCAGGUGCCGCGGCGCGC | 19 | 8616 |
| HSV1-UL19-4018 | - | CGCCCAGGUGCCGCGGCGCGC | 21 | 8617 |
| HSV1-UL19-4019 | - | UGCGCCCAGGUGCCGCGGCGCGC | 23 | 8618 |
| HSV1-UL19-4020 | - | CAGACGACGCGCGUGCGC | 18 | 8619 |
| HSV1-UL19-4021 | - | CCAGACGACGCGCGUGCGC | 19 | 8620 |
| HSV1-UL19-2084 | - | CCCAGACGACGCGCGUGCGC | 20 | 2420 |
| HSV1-UL19-4022 | - | CCCCAGACGACGCGCGUGCGC | 21 | 8621 |
| HSV1-UL19-4023 | - | CCCCCAGACGACGCGCGUGCGC | 22 | 8622 |
| HSV1-UL19-4024 | - | CCCCCCAGACGACGCGCGUGCGC | 23 | 8623 |
| HSV1-UL19-4025 | - | UCAAGACGGGCCUCCAUC | 18 | 8624 |
| HSV1-UL19-4026 | - | CUCAAGACGGGCCUCCAUC | 19 | 8625 |
| HSV1-UL19-4027 | - | AGCUCAAGACGGGCCUCCAUC | 21 | 8626 |
| HSV1-UL19-4028 | - | CAGCUCAAGACGGGCCUCCAUC | 22 | 8627 |
| HSV1-UL19-4029 | - | UCAGCUCAAGACGGGCCUCCAUC | 23 | 8628 |
| HSV1-UL19-4030 | - | AUCAGCUCAAGACGGGCCUCCAUC | 24 | 8629 |
| HSV1-UL19-4031 | - | CGGGCAGGCGCAAGCCGAG | 19 | 8630 |
| HSV1-UL19-4032 | - | UGGGCGGGCAGGCGCAAGCCGAG | 23 | 8631 |
| HSV1-UL19-4033 | - | CUGGGCGGGCAGGCGCAAGCCGAG | 24 | 8632 |
| HSV1-UL19-4034 | - | UACGCGGGGGACAAGGAG | 18 | 8633 |
| HSV1-UL19-4035 | - | UUACGCGGGGGACAAGGAG | 19 | 8634 |
| HSV1-UL19-539 | - | UUUACGCGGGGGACAAGGAG | 20 | 925 |
| HSV1-UL19-4036 | - | CGUUUACGCGGGGGACAAGGAG | 22 | 8635 |
| HSV1-UL19-2307 | - | UGGAGCGCCUGAUCGUGGAG | 20 | 2547 |
| HSV1-UL19-4037 | - | CUGGAGCGCCUGAUCGUGGAG | 21 | 8636 |
| HSV1-UL19-4038 | - | CCUGGAGCGCCUGAUCGUGGAG | 22 | 8637 |
| HSV1-UL19-4039 | - | UGCCUGGAGCGCCUGAUCGUGGAG | 24 | 8638 |
| HSV1-UL19-4040 | - | UCGGGGGAGACCUUCCCG | 18 | 8639 |
| HSV1-UL19-4041 | - | CUCGGGGGAGACCUUCCCG | 19 | 8640 |
| HSV1-UL19-362 | - | CCUCGGGGGAGACCUUCCCG | 20 | 748 |
| HSV1-UL19-4042 | - | ACCUCGGGGGAGACCUUCCCG | 21 | 8641 |
| HSV1-UL19-4043 | - | UACCUCGGGGGAGACCUUCCCG | 22 | 8642 |
| HSV1-UL19-4044 | - | CUACCUCGGGGGAGACCUUCCCG | 23 | 8643 |
| HSV1-UL19-4045 | - | CCUACCUCGGGGGAGACCUUCCCG | 24 | 8644 |
| HSV1-UL19-4046 | - | UGGCCCACGGGCGGGUCCG | 19 | 8645 |
| HSV1-UL19-2124 | - | CUGGCCCACGGGCGGGUCCG | 20 | 2444 |
| HSV1-UL19-4047 | - | CCUGGCCCACGGGCGGGUCCG | 21 | 8646 |
| HSV1-UL19-4048 | - | CGCCUGGCCCACGGGCGGGUCCG | 23 | 8647 |
| HSV1-UL19-4049 | - | CGGAGAUCGCCCCCGGCG | 18 | 8648 |
| HSV1-UL19-4050 | - | CCGGAGAUCGCCCCCGGCG | 19 | 8649 |
| HSV1-UL19-419 | - | CCCGGAGAUCGCCCCCGGCG | 20 | 805 |
| HSV1-UL19-4051 | - | UCCCGGAGAUCGCCCCCGGCG | 21 | 8650 |
| HSV1-UL19-4052 | - | UGGUCCCGGAGAUCGCCCCCGGCG | 24 | 8651 |
| HSV1-UL19-4053 | - | UGGGGGCAGCGCCUGGCCCACGG | 23 | 8652 |
| HSV1-UL19-4054 | - | CUGGGGGCAGCGCCUGGCCCACGG | 24 | 8653 |
| HSV1-UL19-4055 | - | CCGGCACGAAACUGGCGG | 18 | 8654 |
| HSV1-UL19-4056 | - | CCCGGCACGAAACUGGCGG | 19 | 8655 |
| HSV1-UL19-4057 | - | UGGCCCGGCACGAAACUGGCGG | 22 | 8656 |
| HSV1-UL19-4058 | - | UGACCUACGCGCUCAUGG | 18 | 8657 |
| HSV1-UL19-4059 | - | CUGACCUACGCGCUCAUGG | 19 | 8658 |
| HSV1-UL19-4060 | - | CGCUGACCUACGCGCUCAUGG | 21 | 8659 |
| HSV1-UL19-4061 | - | CGCGCUGACCUACGCGCUCAUGG | 23 | 8660 |
| HSV1-UL19-4062 | - | ACGCGCUGACCUACGCGCUCAUGG | 24 | 8661 |
| HSV1-UL19-4063 | - | UGGACCGCCAUCGCGACUG | 19 | 8662 |
| HSV1-UL19-2186 | - | CUGGACCGCCAUCGCGACUG | 20 | 2475 |
| HSV1-UL19-4064 | - | CCUGGACCGCCAUCGCGACUG | 21 | 8663 |
| HSV1-UL19-4065 | - | CCCUGGACCGCCAUCGCGACUG | 22 | 8664 |
| HSV1-UL19-4066 | - | AACCGCCCCGCCCUUCCU | 18 | 8665 |
| HSV1-UL19-4067 | - | AAACCGCCCCGCCCUUCCU | 19 | 8666 |
| HSV1-UL19-3237 | - | UAAACCGCCCCGCCCUUCCU | 20 | 7836 |
| HSV1-UL19-4068 | - | AUAAACCGCCCCGCCCUUCCU | 21 | 8667 |
| HSV1-UL19-4069 | - | AAUAAACCGCCCCGCCCUUCCU | 22 | 8668 |
| HSV1-UL19-4070 | - | UAAUAAACCGCCCCGCCCUUCCU | 23 | 8669 |
| HSV1-UL19-4071 | - | CGCCCGGGGCCUGGAGCU | 18 | 8670 |
| HSV1-UL19-4072 | - | ACGCCCGGGGCCUGGAGCU | 19 | 8671 |
| HSV1-UL19-4073 | - | CGGGACGCCCGGGGCCUGGAGCU | 23 | 8672 |
| HSV1-UL19-4074 | - | CCGGGACGCCCGGGGCCUGGAGCU | 24 | 8673 |
| HSV1-UL19-4075 | - | CGGGCCUCCAUCCCGGGU | 18 | 8674 |
| HSV1-UL19-4076 | - | ACGGGCCUCCAUCCCGGGU | 19 | 8675 |
| HSV1-UL19-4077 | - | AGACGGGCCUCCAUCCCGGGU | 21 | 8676 |
| HSV1-UL19-4078 | - | AAGACGGGCCUCCAUCCCGGGU | 22 | 8677 |
| HSV1-UL19-4079 | - | CAAGACGGGCCUCCAUCCCGGGU | 23 | 8678 |
| HSV1-UL19-4080 | - | UCAAGACGGGCCUCCAUCCCGGGU | 24 | 8679 |
| HSV1-UL19-4081 | - | CCCAGCCCGUCCCCGUGU | 18 | 8680 |
| HSV1-UL19-4082 | - | CGGCCCAGCCCGUCCCCGUGU | 21 | 8681 |
| HSV1-UL19-4083 | - | CCGGCCCAGCCCGUCCCCGUGU | 22 | 8682 |
| HSV1-UL19-4084 | - | ACGGCCAGGACGCCGUGU | 18 | 8683 |
| HSV1-UL19-4085 | - | CACGGCCAGGACGCCGUGU | 19 | 8684 |
| HSV1-UL19-2276 | - | CCACGGCCAGGACGCCGUGU | 20 | 2533 |
| HSV1-UL19-4086 | - | ACCACGGCCAGGACGCCGUGU | 21 | 8685 |
| HSV1-UL19-4087 | - | UGGACCACGGCCAGGACGCCGUGU | 24 | 8686 |
| HSV1-UL19-4088 | - | CGCGUUCGACUUCUUUGU | 18 | 8687 |
| HSV1-UL19-4089 | - | CCGCGUUCGACUUCUUUGU | 19 | 8688 |
| HSV1-UL19-306 | - | CCCGCGUUCGACUUCUUUGU | 20 | 692 |
| HSV1-UL19-4090 | - | CCCCGCGUUCGACUUCUUUGU | 21 | 8689 |
| HSV1-UL19-4091 | - | ACCCCGCGUUCGACUUCUUUGU | 22 | 8690 |
| HSV1-UL19-4092 | - | CACCCCGCGUUCGACUUCUUUGU | 23 | 8691 |

**Table 6G** provides exemplary targeting domains for knocking out the *UL19* gene selected according to the seven tier parameters. The targeting domains fall in the coding sequence of the gene, downstream of the first 500bp of coding sequence (e.g., anywhere from +500 (relative to the start codon) to the stop codon of the gene). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 6G**

| **7th Tier** | | | | |
|---|---|---|---|---|
| **gRNA Name** | **DNA Strand** | **Targeting Domain** | **Target Site Length** | **Seq ID NO:** |
| HSV1-UL19-4093 | + | CGCAGGGCCGGGGGGAAA | 18 | 8692 |
| HSV1-UL19-4094 | + | GCGCAGGGCCGGGGGGAAA | 19 | 8693 |
| HSV1-UL19-2785 | + | CGCGCAGGGCCGGGGGGAAA | 20 | 2844 |
| HSV1-UL19-4095 | + | UCGCGCAGGGCCGGGGGGAAA | 21 | 8694 |
| HSV1-UL19-4096 | + | GUCGCGCAGGGCCGGGGGGAAA | 22 | 8695 |
| HSV1-UL19-4097 | + | GGUCGCGCAGGGCCGGGGGGAAA | 23 | 8696 |
| HSV1-UL19-4098 | + | AGGUCGCGCAGGGCCGGGGGGAAA | 24 | 8697 |
| HSV1-UL19-4099 | + | GGCGUAUACGCGGUCGAA | 18 | 8698 |
| HSV1-UL19-4100 | + | UGGCGUAUACGCGGUCGAA | 19 | 8699 |
| HSV1-UL19-2743 | + | GUGGCGUAUACGCGGUCGAA | 20 | 2818 |
| HSV1-UL19-4101 | + | GGUGGCGUAUACGCGGUCGAA | 21 | 8700 |
| HSV1-UL19-4102 | + | GGGUGGCGUAUACGCGGUCGAA | 22 | 8701 |
| HSV1-UL19-4103 | + | AGGGUGGCGUAUACGCGGUCGAA | 23 | 8702 |
| HSV1-UL19-4104 | + | CAGGGUGGCGUAUACGCGGUCGAA | 24 | 8703 |
| HSV1-UL19-4105 | + | CCUGGGCGCACCCGAACA | 18 | 8704 |
| HSV1-UL19-4106 | + | ACCUGGGCGCACCCGAACA | 19 | 8705 |
| HSV1-UL19-1356 | + | CACCUGGGCGCACCCGAACA | 20 | 1742 |
| HSV1-UL19-4107 | + | GCACCUGGGCGCACCCGAACA | 21 | 8706 |
| HSV1-UL19-4108 | + | GGCACCUGGGCGCACCCGAACA | 22 | 8707 |
| HSV1-UL19-4109 | + | CGGCACCUGGGCGCACCCGAACA | 23 | 8708 |
| HSV1-UL19-4110 | + | GCGGCACCUGGGCGCACCCGAACA | 24 | 8709 |
| HSV1-UL19-4111 | + | ACGUCGGCCACCCCCACA | 18 | 8710 |
| HSV1-UL19-4112 | + | CACGUCGGCCACCCCCACA | 19 | 8711 |
| HSV1-UL19-2693 | + | CCACGUCGGCCACCCCCACA | 20 | 2780 |
| HSV1-UL19-4113 | + | UCCACGUCGGCCACCCCCACA | 21 | 8712 |
| HSV1-UL19-4114 | + | CUCCACGUCGGCCACCCCCACA | 22 | 8713 |
| HSV1-UL19-4115 | + | GCUCCACGUCGGCCACCCCCACA | 23 | 8714 |
| HSV1-UL19-4116 | + | AGCUCCACGUCGGCCACCCCCACA | 24 | 8715 |
| HSV1-UL19-4117 | + | AGUUGACGUCGGUCGACA | 18 | 8716 |
| HSV1-UL19-4118 | + | UAGUUGACGUCGGUCGACA | 19 | 8717 |
| HSV1-UL19-1367 | + | GUAGUUGACGUCGGUCGACA | 20 | 1753 |
| HSV1-UL19-4119 | + | AGUAGUUGACGUCGGUCGACA | 21 | 8718 |
| HSV1-UL19-4120 | + | AAGUAGUUGACGUCGGUCGACA | 22 | 8719 |
| HSV1-UL19-4121 | + | GAAGUAGUUGACGUCGGUCGACA | 23 | 8720 |
| HSV1-UL19-4122 | + | GGAAGUAGUUGACGUCGGUCGACA | 24 | 8721 |
| HSV1-UL19-4123 | + | CCAGAGCCGGGGGGACCA | 18 | 8722 |
| HSV1-UL19-4124 | + | CCCAGAGCCGGGGGGACCA | 19 | 8723 |
| HSV1-UL19-1292 | + | CCCCAGAGCCGGGGGGACCA | 20 | 1678 |
| HSV1-UL19-4125 | + | CCCCCAGAGCCGGGGGGACCA | 21 | 8724 |
| HSV1-UL19-4126 | + | GCCCCCAGAGCCGGGGGGACCA | 22 | 8725 |
| HSV1-UL19-4127 | + | GGCCCCCAGAGCCGGGGGGACCA | 23 | 8726 |
| HSV1-UL19-4128 | + | UGGCCCCCAGAGCCGGGGGGACCA | 24 | 8727 |
| HSV1-UL19-4129 | + | CUGGCCUUCCGCGACCCA | 18 | 8728 |
| HSV1-UL19-4130 | + | UCUGGCCUUCCGCGACCCA | 19 | 8729 |
| HSV1-UL19-2685 | + | AUCUGGCCUUCCGCGACCCA | 20 | 2774 |
| HSV1-UL19-4131 | + | CAUCUGGCCUUCCGCGACCCA | 21 | 8730 |
| HSV1-UL19-4132 | + | UCAUCUGGCCUUCCGCGACCCA | 22 | 8731 |
| HSV1-UL19-4133 | + | GUCAUCUGGCCUUCCGCGACCCA | 23 | 8732 |
| HSV1-UL19-4134 | + | GGUCAUCUGGCCUUCCGCGACCCA | 24 | 8733 |
| HSV1-UL19-4135 | + | GCUGAUGAUGCAAGGCCA | 18 | 8734 |
| HSV1-UL19-4136 | + | AGCUGAUGAUGCAAGGCCA | 19 | 8735 |
| HSV1-UL19-1307 | + | GAGCUGAUGAUGCAAGGCCA | 20 | 1693 |
| HSV1-UL19-4137 | + | UGAGCUGAUGAUGCAAGGCCA | 21 | 8736 |
| HSV1-UL19-4138 | + | UUGAGCUGAUGAUGCAAGGCCA | 22 | 8737 |
| HSV1-UL19-4139 | + | CUUGAGCUGAUGAUGCAAGGCCA | 23 | 8738 |
| HSV1-UL19-4140 | + | UCUUGAGCUGAUGAUGCAAGGCCA | 24 | 8739 |
| HSV1-UL19-4141 | + | UCAGACGGGGCACGGCCA | 18 | 8740 |
| HSV1-UL19-4142 | + | GUCAGACGGGGCACGGCCA | 19 | 8741 |
| HSV1-UL19-1006 | + | CGUCAGACGGGGCACGGCCA | 20 | 1392 |
| HSV1-UL19-4143 | + | GCGUCAGACGGGGCACGGCCA | 21 | 8742 |
| HSV1-UL19-4144 | + | UGCGUCAGACGGGGCACGGCCA | 22 | 8743 |
| HSV1-UL19-4145 | + | AUGCGUCAGACGGGGCACGGCCA | 23 | 8744 |
| HSV1-UL19-4146 | + | CAUGCGUCAGACGGGGCACGGCCA | 24 | 8745 |
| HSV1-UL19-4147 | + | CCAGUAGCUGGUGAUGCA | 18 | 8746 |
| HSV1-UL19-4148 | + | UCCAGUAGCUGGUGAUGCA | 19 | 8747 |
| HSV1-UL19-2717 | + | UUCCAGUAGCUGGUGAUGCA | 20 | 2796 |
| HSV1-UL19-4149 | + | GUUCCAGUAGCUGGUGAUGCA | 21 | 8748 |
| HSV1-UL19-4150 | + | UGUUCCAGUAGCUGGUGAUGCA | 22 | 8749 |
| HSV1-UL19-4151 | + | UUGUUCCAGUAGCUGGUGAUGCA | 23 | 8750 |
| HSV1-UL19-4152 | + | GUUGUUCCAGUAGCUGGUGAUGCA | 24 | 8751 |
| HSV1-UL19-4153 | + | CCGCGUCAACGUUCAUCA | 18 | 8752 |
| HSV1-UL19-4154 | + | GCCGCGUCAACGUUCAUCA | 19 | 8753 |
| HSV1-UL19-2676 | + | CGCCGCGUCAACGUUCAUCA | 20 | 2768 |
| HSV1-UL19-4155 | + | CCGCCGCGUCAACGUUCAUCA | 21 | 8754 |
| HSV1-UL19-4156 | + | ACCGCCGCGUCAACGUUCAUCA | 22 | 8755 |
| HSV1-UL19-4157 | + | GACCGCCGCGUCAACGUUCAUCA | 23 | 8756 |
| HSV1-UL19-4158 | + | CGACCGCCGCGUCAACGUUCAUCA | 24 | 8757 |
| HSV1-UL19-4159 | + | GUGUCGGCAUGCGUCAGA | 18 | 8758 |
| HSV1-UL19-4160 | + | CGUGUCGGCAUGCGUCAGA | 19 | 8759 |
| HSV1-UL19-1010 | + | GCGUGUCGGCAUGCGUCAGA | 20 | 1396 |
| HSV1-UL19-4161 | + | CGCGUGUCGGCAUGCGUCAGA | 21 | 8760 |
| HSV1-UL19-4162 | + | GCGCGUGUCGGCAUGCGUCAGA | 22 | 8761 |
| HSV1-UL19-4163 | + | CGCGCGUGUCGGCAUGCGUCAGA | 23 | 8762 |
| HSV1-UL19-4164 | + | CCGCGCGUGUCGGCAUGCGUCAGA | 24 | 8763 |
| HSV1-UL19-4165 | + | GCCGCGUUGUCCAGGAGA | 18 | 8764 |
| HSV1-UL19-4166 | + | UGCCGCGUUGUCCAGGAGA | 19 | 8765 |
| HSV1-UL19-1342 | + | CUGCCGCGUUGUCCAGGAGA | 20 | 1728 |
| HSV1-UL19-4167 | + | GCUGCCGCGUUGUCCAGGAGA | 21 | 8766 |
| HSV1-UL19-4168 | + | GGCUGCCGCGUUGUCCAGGAGA | 22 | 8767 |
| HSV1-UL19-4169 | + | CGGCUGCCGCGUUGUCCAGGAGA | 23 | 8768 |
| HSV1-UL19-4170 | + | ACGGCUGCCGCGUUGUCCAGGAGA | 24 | 8769 |
| HSV1-UL19-3318 | + | ACGGCCUGGACGUUGCGA | 18 | 7917 |
| HSV1-UL19-3319 | + | GACGGCCUGGACGUUGCGA | 19 | 7918 |
| HSV1-UL19-3320 | + | GGACGGCCUGGACGUUGCGA | 20 | 7919 |
| HSV1-UL19-3321 | + | AGGACGGCCUGGACGUUGCGA | 21 | 7920 |
| HSV1-UL19-3322 | + | GAGGACGGCCUGGACGUUGCGA | 22 | 7921 |
| HSV1-UL19-3323 | + | CGAGGACGGCCUGGACGUUGCGA | 23 | 7922 |
| HSV1-UL19-3324 | + | CCGAGGACGGCCUGGACGUUGCGA | 24 | 7923 |
| HSV1-UL19-4171 | + | CUGCCGCGUUGUCCAGGA | 18 | 8770 |
| HSV1-UL19-4172 | + | GCUGCCGCGUUGUCCAGGA | 19 | 8771 |
| HSV1-UL19-2839 | + | GGCUGCCGCGUUGUCCAGGA | 20 | 2873 |
| HSV1-UL19-4173 | + | CGGCUGCCGCGUUGUCCAGGA | 21 | 8772 |
| HSV1-UL19-4174 | + | ACGGCUGCCGCGUUGUCCAGGA | 22 | 8773 |
| HSV1-UL19-4175 | + | CACGGCUGCCGCGUUGUCCAGGA | 23 | 8774 |
| HSV1-UL19-4176 | + | ACACGGCUGCCGCGUUGUCCAGGA | 24 | 8775 |
| HSV1-UL19-4177 | + | AACCGUUCCAUGACCGGA | 18 | 8776 |
| HSV1-UL19-4178 | + | AAACCGUUCCAUGACCGGA | 19 | 8777 |
| HSV1-UL19-2657 | + | CAAACCGUUCCAUGACCGGA | 20 | 2758 |
| HSV1-UL19-4179 | + | GCAAACCGUUCCAUGACCGGA | 21 | 8778 |
| HSV1-UL19-4180 | + | GGCAAACCGUUCCAUGACCGGA | 22 | 8779 |
| HSV1-UL19-4181 | + | CGGCAAACCGUUCCAUGACCGGA | 23 | 8780 |
| HSV1-UL19-4182 | + | GCGGCAAACCGUUCCAUGACCGGA | 24 | 8781 |
| HSV1-UL19-4183 | + | GUGAACCCAAACCCGGGA | 18 | 8782 |
| HSV1-UL19-4184 | + | GGUGAACCCAAACCCGGGA | 19 | 8783 |
| HSV1-UL19-1310 | + | CGGUGAACCCAAACCCGGGA | 20 | 1696 |
| HSV1-UL19-4185 | + | ACGGUGAACCCAAACCCGGGA | 21 | 8784 |
| HSV1-UL19-4186 | + | GACGGUGAACCCAAACCCGGGA | 22 | 8785 |
| HSV1-UL19-4187 | + | CGACGGUGAACCCAAACCCGGGA | 23 | 8786 |
| HSV1-UL19-4188 | + | ACGACGGUGAACCCAAACCCGGGA | 24 | 8787 |
| HSV1-UL19-4189 | + | CGGCGAACAGCGCGUGGA | 18 | 8788 |
| HSV1-UL19-4190 | + | CCGGCGAACAGCGCGUGGA | 19 | 8789 |
| HSV1-UL19-1275 | + | CCCGGCGAACAGCGCGUGGA | 20 | 1661 |
| HSV1-UL19-4191 | + | CCCCGGCGAACAGCGCGUGGA | 21 | 8790 |
| HSV1-UL19-4192 | + | GCCCCGGCGAACAGCGCGUGGA | 22 | 8791 |
| HSV1-UL19-4193 | + | GGCCCCGGCGAACAGCGCGUGGA | 23 | 8792 |
| HSV1-UL19-4194 | + | CGGCCCCGGCGAACAGCGCGUGGA | 24 | 8793 |
| HSV1-UL19-4195 | + | ACAGAGCCAGUCCCUUGA | 18 | 8794 |
| HSV1-UL19-4196 | + | UACAGAGCCAGUCCCUUGA | 19 | 8795 |
| HSV1-UL19-2899 | + | UUACAGAGCCAGUCCCUUGA | 20 | 2915 |
| HSV1-UL19-4197 | + | AUUACAGAGCCAGUCCCUUGA | 21 | 8796 |
| HSV1-UL19-4198 | + | UAUUACAGAGCCAGUCCCUUGA | 22 | 8797 |
| HSV1-UL19-4199 | + | UUAUUACAGAGCCAGUCCCUUGA | 23 | 8798 |
| HSV1-UL19-4200 | + | UUUAUUACAGAGCCAGUCCCUUGA | 24 | 8799 |
| HSV1-UL19-4201 | + | GCAGGAGGCGCUGUUUGA | 18 | 8800 |
| HSV1-UL19-4202 | + | UGCAGGAGGCGCUGUUUGA | 19 | 8801 |
| HSV1-UL19-2630 | + | CUGCAGGAGGCGCUGUUUGA | 20 | 2741 |
| HSV1-UL19-4203 | + | ACUGCAGGAGGCGCUGUUUGA | 21 | 8802 |
| HSV1-UL19-4204 | + | GACUGCAGGAGGCGCUGUUUGA | 22 | 8803 |
| HSV1-UL19-4205 | + | GGACUGCAGGAGGCGCUGUUUGA | 23 | 8804 |
| HSV1-UL19-4206 | + | AGGACUGCAGGAGGCGCUGUUUGA | 24 | 8805 |
| HSV1-UL19-4207 | + | GCGAUGUCGGCCGACGUA | 18 | 8806 |
| HSV1-UL19-4208 | + | GGCGAUGUCGGCCGACGUA | 19 | 8807 |
| HSV1-UL19-2873 | + | CGGCGAUGUCGGCCGACGUA | 20 | 2897 |
| HSV1-UL19-4209 | + | GCGGCGAUGUCGGCCGACGUA | 21 | 8808 |
| HSV1-UL19-4210 | + | GGCGGCGAUGUCGGCCGACGUA | 22 | 8809 |
| HSV1-UL19-4211 | + | UGGCGGCGAUGUCGGCCGACGUA | 23 | 8810 |
| HSV1-UL19-4212 | + | UUGGCGGCGAUGUCGGCCGACGUA | 24 | 8811 |
| HSV1-UL19-4213 | + | GGCGCCCACCAGGGGGUA | 18 | 8812 |
| HSV1-UL19-4214 | + | UGGCGCCCACCAGGGGGUA | 19 | 8813 |
| HSV1-UL19-1045 | + | AUGGCGCCCACCAGGGGGUA | 20 | 1431 |
| HSV1-UL19-4215 | + | CAUGGCGCCCACCAGGGGGUA | 21 | 8814 |
| HSV1-UL19-4216 | + | CCAUGGCGCCCACCAGGGGGUA | 22 | 8815 |
| HSV1-UL19-4217 | + | UCCAUGGCGCCCACCAGGGGGUA | 23 | 8816 |
| HSV1-UL19-4218 | + | GUCCAUGGCGCCCACCAGGGGGUA | 24 | 8817 |
| HSV1-UL19-4219 | + | GGGCGGGGCGGUUUAUUA | 18 | 8818 |
| HSV1-UL19-4220 | + | AGGGCGGGGCGGUUUAUUA | 19 | 8819 |
| HSV1-UL19-4221 | + | AAGGGCGGGGCGGUUUAUUA | 20 | 8820 |
| HSV1-UL19-4222 | + | GAAGGGCGGGGCGGUUUAUUA | 21 | 8821 |
| HSV1-UL19-4223 | + | GGAAGGGCGGGGCGGUUUAUUA | 22 | 8822 |
| HSV1-UL19-4224 | + | AGGAAGGGCGGGGCGGUUUAUUA | 23 | 8823 |
| HSV1-UL19-4225 | + | AAGGAAGGGCGGGGCGGUUUAUUA | 24 | 8824 |
| HSV1-UL19-4226 | + | AGGUGGUAGGCCCCGUUA | 18 | 8825 |
| HSV1-UL19-4227 | + | GAGGUGGUAGGCCCCGUUA | 19 | 8826 |
| HSV1-UL19-2867 | + | UGAGGUGGUAGGCCCCGUUA | 20 | 2892 |
| HSV1-UL19-4228 | + | UUGAGGUGGUAGGCCCCGUUA | 21 | 8827 |
| HSV1-UL19-4229 | + | GUUGAGGUGGUAGGCCCCGUUA | 22 | 8828 |
| HSV1-UL19-4230 | + | CGUUGAGGUGGUAGGCCCCGUUA | 23 | 8829 |
| HSV1-UL19-4231 | + | CCGUUGAGGUGGUAGGCCCCGUUA | 24 | 8830 |
| HSV1-UL19-4232 | + | ACGACGGUGAACCCAAAC | 18 | 8831 |
| HSV1-UL19-4233 | + | GACGACGGUGAACCCAAAC | 19 | 8832 |
| HSV1-UL19-2815 | + | GGACGACGGUGAACCCAAAC | 20 | 2859 |
| HSV1-UL19-4234 | + | CGGACGACGGUGAACCCAAAC | 21 | 8833 |
| HSV1-UL19-4235 | + | UCGGACGACGGUGAACCCAAAC | 22 | 8834 |
| HSV1-UL19-4236 | + | GUCGGACGACGGUGAACCCAAAC | 23 | 8835 |
| HSV1-UL19-4237 | + | UGUCGGACGACGGUGAACCCAAAC | 24 | 8836 |
| HSV1-UL19-4238 | + | ACCUGGGCGCACCCGAAC | 18 | 8837 |
| HSV1-UL19-4239 | + | CACCUGGGCGCACCCGAAC | 19 | 8838 |
| HSV1-UL19-2847 | + | GCACCUGGGCGCACCCGAAC | 20 | 2878 |
| HSV1-UL19-4240 | + | GGCACCUGGGCGCACCCGAAC | 21 | 8839 |
| HSV1-UL19-4241 | + | CGGCACCUGGGCGCACCCGAAC | 22 | 8840 |
| HSV1-UL19-4242 | + | GCGGCACCUGGGCGCACCCGAAC | 23 | 8841 |
| HSV1-UL19-4243 | + | CGCGGCACCUGGGCGCACCCGAAC | 24 | 8842 |
| HSV1-UL19-4244 | + | CCCACAAAGAAGUCGAAC | 18 | 8843 |
| HSV1-UL19-4245 | + | CCCCACAAAGAAGUCGAAC | 19 | 8844 |
| HSV1-UL19-2691 | + | CCCCCACAAAGAAGUCGAAC | 20 | 2778 |
| HSV1-UL19-4246 | + | ACCCCCACAAAGAAGUCGAAC | 21 | 8845 |
| HSV1-UL19-4247 | + | CACCCCCACAAAGAAGUCGAAC | 22 | 8846 |
| HSV1-UL19-4248 | + | CCACCCCCACAAAGAAGUCGAAC | 23 | 8847 |
| HSV1-UL19-4249 | + | GCCACCCCCACAAAGAAGUCGAAC | 24 | 8848 |
| HSV1-UL19-4250 | + | CUGGGCGCACCCGAACAC | 18 | 8849 |
| HSV1-UL19-4251 | + | CCUGGGCGCACCCGAACAC | 19 | 8850 |
| HSV1-UL19-1355 | + | ACCUGGGCGCACCCGAACAC | 20 | 1741 |
| HSV1-UL19-4252 | + | CACCUGGGCGCACCCGAACAC | 21 | 8851 |
| HSV1-UL19-4253 | + | GCACCUGGGCGCACCCGAACAC | 22 | 8852 |
| HSV1-UL19-4254 | + | GGCACCUGGGCGCACCCGAACAC | 23 | 8853 |
| HSV1-UL19-4255 | + | CGGCACCUGGGCGCACCCGAACAC | 24 | 8854 |
| HSV1-UL19-4256 | + | GGCGUCACAGUCCCACAC | 18 | 8855 |
| HSV1-UL19-4257 | + | GGGCGUCACAGUCCCACAC | 19 | 8856 |
| HSV1-UL19-2731 | + | AGGGCGUCACAGUCCCACAC | 20 | 2807 |
| HSV1-UL19-4258 | + | CAGGGCGUCACAGUCCCACAC | 21 | 8857 |
| HSV1-UL19-4259 | + | UCAGGGCGUCACAGUCCCACAC | 22 | 8858 |
| HSV1-UL19-4260 | + | AUCAGGGCGUCACAGUCCCACAC | 23 | 8859 |
| HSV1-UL19-4261 | + | CAUCAGGGCGUCACAGUCCCACAC | 24 | 8860 |
| HSV1-UL19-4262 | + | CAGGUCCAUGGCGCCCAC | 18 | 8861 |
| HSV1-UL19-4263 | + | UCAGGUCCAUGGCGCCCAC | 19 | 8862 |
| HSV1-UL19-2652 | + | GUCAGGUCCAUGGCGCCCAC | 20 | 2754 |
| HSV1-UL19-4264 | + | CGUCAGGUCCAUGGCGCCCAC | 21 | 8863 |
| HSV1-UL19-4265 | + | ACGUCAGGUCCAUGGCGCCCAC | 22 | 8864 |
| HSV1-UL19-4266 | + | AACGUCAGGUCCAUGGCGCCCAC | 23 | 8865 |
| HSV1-UL19-4267 | + | GAACGUCAGGUCCAUGGCGCCCAC | 24 | 8866 |
| HSV1-UL19-4268 | + | AGGGCGUCACAGUCCCAC | 18 | 8867 |
| HSV1-UL19-4269 | + | CAGGGCGUCACAGUCCCAC | 19 | 8868 |
| HSV1-UL19-2732 | + | UCAGGGCGUCACAGUCCCAC | 20 | 2808 |
| HSV1-UL19-4270 | + | AUCAGGGCGUCACAGUCCCAC | 21 | 8869 |
| HSV1-UL19-4271 | + | CAUCAGGGCGUCACAGUCCCAC | 22 | 8870 |
| HSV1-UL19-4272 | + | GCAUCAGGGCGUCACAGUCCCAC | 23 | 8871 |
| HSV1-UL19-4273 | + | CGCAUCAGGGCGUCACAGUCCCAC | 24 | 8872 |
| HSV1-UL19-4274 | + | AGUUUCGUGCCGGGCCAC | 18 | 8873 |
| HSV1-UL19-4275 | + | CAGUUUCGUGCCGGGCCAC | 19 | 8874 |
| HSV1-UL19-2823 | + | CCAGUUUCGUGCCGGGCCAC | 20 | 2865 |
| HSV1-UL19-4276 | + | GCCAGUUUCGUGCCGGGCCAC | 21 | 8875 |
| HSV1-UL19-4277 | + | CGCCAGUUUCGUGCCGGGCCAC | 22 | 8876 |
| HSV1-UL19-4278 | + | CCGCCAGUUUCGUGCCGGGCCAC | 23 | 8877 |
| HSV1-UL19-4279 | + | CCCGCCAGUUUCGUGCCGGGCCAC | 24 | 8878 |
| HSV1-UL19-4280 | + | CGCGGCACCUGGGCGCAC | 18 | 8879 |
| HSV1-UL19-4281 | + | CCGCGGCACCUGGGCGCAC | 19 | 8880 |
| HSV1-UL19-2848 | + | GCCGCGGCACCUGGGCGCAC | 20 | 2879 |
| HSV1-UL19-4282 | + | CGCCGCGGCACCUGGGCGCAC | 21 | 8881 |
| HSV1-UL19-4283 | + | GCGCCGCGGCACCUGGGCGCAC | 22 | 8882 |
| HSV1-UL19-4284 | + | CGCGCCGCGGCACCUGGGCGCAC | 23 | 8883 |
| HSV1-UL19-4285 | + | GCGCGCCGCGGCACCUGGGCGCAC | 24 | 8884 |
| HSV1-UL19-4286 | + | CCCUGCGCGCCGCGGCAC | 18 | 8885 |
| HSV1-UL19-4287 | + | UCCCUGCGCGCCGCGGCAC | 19 | 8886 |
| HSV1-UL19-2849 | + | AUCCCUGCGCGCCGCGGCAC | 20 | 2880 |
| HSV1-UL19-4288 | + | CAUCCCUGCGCGCCGCGGCAC | 21 | 8887 |
| HSV1-UL19-4289 | + | CCAUCCCUGCGCGCCGCGGCAC | 22 | 8888 |
| HSV1-UL19-4290 | + | UCCAUCCCUGCGCGCCGCGGCAC | 23 | 8889 |
| HSV1-UL19-4291 | + | GUCCAUCCCUGCGCGCCGCGGCAC | 24 | 8890 |
| HSV1-UL19-4292 | + | UAGUUGACGUCGGUCGAC | 18 | 8891 |
| HSV1-UL19-4293 | + | GUAGUUGACGUCGGUCGAC | 19 | 8892 |
| HSV1-UL19-2852 | + | AGUAGUUGACGUCGGUCGAC | 20 | 2881 |
| HSV1-UL19-4294 | + | AAGUAGUUGACGUCGGUCGAC | 21 | 8893 |
| HSV1-UL19-4295 | + | GAAGUAGUUGAC GUCGGUC GAC | 22 | 8894 |
| HSV1-UL19-4296 | + | GGAAGUAGUUGACGUCGGUCGAC | 23 | 8895 |
| HSV1-UL19-4297 | + | CGGAAGUAGUUGACGUCGGUCGAC | 24 | 8896 |
| HSV1-UL19-4298 | + | CCCCAGAGCCGGGGGGAC | 18 | 8897 |
| HSV1-UL19-4299 | + | CCCCCAGAGCCGGGGGGAC | 19 | 8898 |
| HSV1-UL19-2796 | + | GCCCCCAGAGCCGGGGGGAC | 20 | 2847 |
| HSV1-UL19-4300 | + | GGCCCCCAGAGCCGGGGGGAC | 21 | 8899 |
| HSV1-UL19-4301 | + | UGGCCCCCAGAGCCGGGGGGAC | 22 | 8900 |
| HSV1-UL19-4302 | + | UUGGCCCCCAGAGCCGGGGGGAC | 23 | 8901 |
| HSV1-UL19-4303 | + | GUUGGCCCCCAGAGCCGGGGGGAC | 24 | 8902 |
| HSV1-UL19-4304 | + | GGCGAACAGCGCGUGGAC | 18 | 8903 |
| HSV1-UL19-4305 | + | CGGCGAACAGCGCGUGGAC | 19 | 8904 |
| HSV1-UL19-1274 | + | CCGGCGAACAGCGCGUGGAC | 20 | 1660 |
| HSV1-UL19-4306 | + | CCCGGCGAACAGCGCGUGGAC | 21 | 8905 |
| HSV1-UL19-4307 | + | CCCCGGCGAACAGCGCGUGGAC | 22 | 8906 |
| HSV1-UL19-4308 | + | GCCCCGGCGAACAGCGCGUGGAC | 23 | 8907 |
| HSV1-UL19-4309 | + | GGCCCCGGCGAACAGCGCGUGGAC | 24 | 8908 |
| HSV1-UL19-4310 | + | UUACAUGCCGCCGCGUAC | 18 | 8909 |
| HSV1-UL19-4311 | + | GUUACAUGCCGCCGCGUAC | 19 | 8910 |
| HSV1-UL19-2736 | + | CGUUACAUGCCGCCGCGUAC | 20 | 2811 |
| HSV1-UL19-4312 | + | ACGUUACAUGCCGCCGCGUAC | 21 | 8911 |
| HSV1-UL19-4313 | + | CACGUUACAUGCCGCCGCGUAC | 22 | 8912 |
| HSV1-UL19-4314 | + | CCACGUUACAUGCCGCCGCGUAC | 23 | 8913 |
| HSV1-UL19-4315 | + | GCCACGUUACAUGCCGCCGCGUAC | 24 | 8914 |
| HSV1-UL19-4316 | + | CUCUGGCCGUGGUCGUAC | 18 | 8915 |
| HSV1-UL19-4317 | + | GCUCUGGCCGUGGUCGUAC | 19 | 8916 |
| HSV1-UL19-2860 | + | CGCUCUGGCCGUGGUCGUAC | 20 | 2886 |
| HSV1-UL19-4318 | + | UCGCUCUGGCCGUGGUCGUAC | 21 | 8917 |
| HSV1-UL19-4319 | + | GUCGCUCUGGCCGUGGUCGUAC | 22 | 8918 |
| HSV1-UL19-4320 | + | GGUCGCUCUGGCCGUGGUCGUAC | 23 | 8919 |
| HSV1-UL19-4321 | + | GGGUCGCUCUGGCCGUGGUCGUAC | 24 | 8920 |
| HSV1-UL19-4322 | + | AGGUCCAUGGCGCCCACC | 18 | 8921 |
| HSV1-UL19-4323 | + | CAGGUCCAUGGCGCCCACC | 19 | 8922 |
| HSV1-UL19-1050 | + | UCAGGUCCAUGGCGCCCACC | 20 | 1436 |
| HSV1-UL19-4324 | + | GUCAGGUCCAUGGCGCCCACC | 21 | 8923 |
| HSV1-UL19-4325 | + | CGUCAGGUCCAUGGCGCCCACC | 22 | 8924 |
| HSV1-UL19-4326 | + | ACGUCAGGUCCAUGGCGCCCACC | 23 | 8925 |
| HSV1-UL19-4327 | + | AACGUCAGGUCCAUGGCGCCCACC | 24 | 8926 |
| HSV1-UL19-4328 | + | GUUUCGUGCCGGGCCACC | 18 | 8927 |
| HSV1-UL19-4329 | + | AGUUUCGUGCCGGGCCACC | 19 | 8928 |
| HSV1-UL19-1318 | + | CAGUUUCGUGCCGGGCCACC | 20 | 1704 |
| HSV1-UL19-4330 | + | CCAGUUUCGUGCCGGGCCACC | 21 | 8929 |
| HSV1-UL19-4331 | + | GCCAGUUUCGUGCCGGGCCACC | 22 | 8930 |
| HSV1-UL19-4332 | + | CGCCAGUUUCGUGCCGGGCCACC | 23 | 8931 |
| HSV1-UL19-4333 | + | CCGCCAGUUUCGUGCCGGGCCACC | 24 | 8932 |
| HSV1-UL19-4334 | + | CCACGAGUUCGCGGCACC | 18 | 8933 |
| HSV1-UL19-4335 | + | UCCACGAGUUCGCGGCACC | 19 | 8934 |
| HSV1-UL19-2879 | + | CUCCACGAGUUCGCGGCACC | 20 | 2900 |
| HSV1-UL19-4336 | + | CCUCCACGAGUUCGCGGCACC | 21 | 8935 |
| HSV1-UL19-4337 | + | UCCUCCACGAGUUCGCGGCACC | 22 | 8936 |
| HSV1-UL19-4338 | + | GUCCUCCACGAGUUCGCGGCACC | 23 | 8937 |
| HSV1-UL19-4339 | + | GGUCCUCCACGAGUUCGCGGCACC | 24 | 8938 |
| HSV1-UL19-4340 | + | CCCAGAGCCGGGGGGACC | 18 | 8939 |
| HSV1-UL19-4341 | + | CCCCAGAGCCGGGGGGACC | 19 | 8940 |
| HSV1-UL19-1293 | + | CCCCCAGAGCCGGGGGGACC | 20 | 1679 |
| HSV1-UL19-4342 | + | GCCCCCAGAGCCGGGGGGACC | 21 | 8941 |
| HSV1-UL19-4343 | + | GGCCCCCAGAGCCGGGGGGACC | 22 | 8942 |
| HSV1-UL19-4344 | + | UGGCCCCCAGAGCCGGGGGGACC | 23 | 8943 |
| HSV1-UL19-4345 | + | UUGGCCCCCAGAGCCGGGGGGACC | 24 | 8944 |
| HSV1-UL19-4346 | + | CUGGUGAUGCACUGGACC | 18 | 8945 |
| HSV1-UL19-4347 | + | GCUGGUGAUGCACUGGACC | 19 | 8946 |
| HSV1-UL19-2716 | + | AGCUGGUGAUGCACUGGACC | 20 | 2795 |
| HSV1-UL19-4348 | + | UAGCUGGUGAUGCACUGGACC | 21 | 8947 |
| HSV1-UL19-4349 | + | GUAGCUGGUGAUGCACUGGACC | 22 | 8948 |
| HSV1-UL19-4350 | + | AGUAGCUGGUGAUGCACUGGACC | 23 | 8949 |
| HSV1-UL19-4351 | + | CAGUAGCUGGUGAUGCACUGGACC | 24 | 8950 |
| HSV1-UL19-4352 | + | CACGAGUUCGCGGCACCC | 18 | 8951 |
| HSV1-UL19-4353 | + | CCACGAGUUCGCGGCACCC | 19 | 8952 |
| HSV1-UL19-1416 | + | UCCACGAGUUCGCGGCACCC | 20 | 1802 |
| HSV1-UL19-4354 | + | CUCCACGAGUUCGCGGCACCC | 21 | 8953 |
| HSV1-UL19-4355 | + | CCUCCACGAGUUCGCGGCACCC | 22 | 8954 |
| HSV1-UL19-4356 | + | UCCUCCACGAGUUCGCGGCACCC | 23 | 8955 |
| HSV1-UL19-4357 | + | GUCCUCCACGAGUUCGCGGCACCC | 24 | 8956 |
| HSV1-UL19-4358 | + | ACGAGUUCGCGGCACCCC | 18 | 8957 |
| HSV1-UL19-4359 | + | CACGAGUUCGCGGCACCCC | 19 | 8958 |
| HSV1-UL19-1415 | + | CCACGAGUUCGCGGCACCCC | 20 | 1801 |
| HSV1-UL19-4360 | + | UCCACGAGUUCGCGGCACCCC | 21 | 8959 |
| HSV1-UL19-4361 | + | CUCCACGAGUUCGCGGCACCCC | 22 | 8960 |
| HSV1-UL19-4362 | + | CCUCCACGAGUUCGCGGCACCCC | 23 | 8961 |
| HSV1-UL19-4363 | + | UCCUCCACGAGUUCGCGGCACCCC | 24 | 8962 |
| HSV1-UL19-4364 | + | GCCGCGCUCAAACGCCCC | 18 | 8963 |
| HSV1-UL19-4365 | + | UGCCGCGCUCAAACGCCCC | 19 | 8964 |
| HSV1-UL19-2612 | + | GUGCCGCGCUCAAACGCCCC | 20 | 2728 |
| HSV1-UL19-4366 | + | CGUGCCGCGCUCAAACGCCCC | 21 | 8965 |
| HSV1-UL19-4367 | + | CCGUGCCGCGCUCAAACGCCCC | 22 | 8966 |
| HSV1-UL19-4368 | + | GCCGUGCCGCGCUCAAACGCCCC | 23 | 8967 |
| HSV1-UL19-4369 | + | GGCCGUGCCGCGCUCAAACGCCCC | 24 | 8968 |
| HSV1-UL19-4370 | + | GCGGUCGUCGAACGCCCC | 18 | 8969 |
| HSV1-UL19-4371 | + | UGCGGUCGUCGAACGCCCC | 19 | 8970 |
| HSV1-UL19-2710 | + | UUGCGGUCGUCGAACGCCCC | 20 | 2789 |
| HSV1-UL19-4372 | + | GUUGCGGUCGUCGAACGCCCC | 21 | 8971 |
| HSV1-UL19-4373 | + | AGUUGCGGUCGUCGAACGCCCC | 22 | 8972 |
| HSV1-UL19-4374 | + | UAGUUGCGGUCGUCGAACGCCCC | 23 | 8973 |
| HSV1-UL19-4375 | + | GUAGUUGCGGUCGUCGAACGCCCC | 24 | 8974 |
| HSV1-UL19-4376 | + | CGGUGCAGCAGCGGCCCC | 18 | 8975 |
| HSV1-UL19-4377 | + | GCGGUGCAGCAGCGGCCCC | 19 | 8976 |
| HSV1-UL19-2742 | + | CGCGGUGCAGCAGCGGCCCC | 20 | 2817 |
| HSV1-UL19-4378 | + | CCGCGGUGCAGCAGCGGCCCC | 21 | 8977 |
| HSV1-UL19-4379 | + | CCCGCGGUGCAGCAGCGGCCCC | 22 | 8978 |
| HSV1-UL19-4380 | + | CCCCGCGGUGCAGCAGCGGCCCC | 23 | 8979 |
| HSV1-UL19-4381 | + | CCCCCGCGGUGCAGCAGCGGCCCC | 24 | 8980 |
| HSV1-UL19-4382 | + | CGAAAAGUAGUUGGCCCC | 18 | 8981 |
| HSV1-UL19-4383 | + | ACGAAAAGUAGUUGGCCCC | 19 | 8982 |
| HSV1-UL19-2802 | + | GACGAAAAGUAGUUGGCCCC | 20 | 2849 |
| HSV1-UL19-4384 | + | CGACGAAAAGUAGUUGGCCCC | 21 | 8983 |
| HSV1-UL19-4385 | + | UCGACGAAAAGUAGUUGGCCCC | 22 | 8984 |
| HSV1-UL19-4386 | + | AUCGACGAAAAGUAGUUGGCCCC | 23 | 8985 |
| HSV1-UL19-4387 | + | GAUCGACGAAAAGUAGUUGGCCCC | 24 | 8986 |
| HSV1-UL19-4388 | + | GACCCAUCGGACCCGCCC | 18 | 8987 |
| HSV1-UL19-4389 | + | CGACCCAUCGGACCCGCCC | 19 | 8988 |
| HSV1-UL19-2684 | + | GCGACCCAUCGGACCCGCCC | 20 | 2773 |
| HSV1-UL19-4390 | + | CGCGACCCAUCGGACCCGCCC | 21 | 8989 |
| HSV1-UL19-4391 | + | CCGCGACCCAUCGGACCCGCCC | 22 | 8990 |
| HSV1-UL19-4392 | + | UCCGCGACCCAUCGGACCCGCCC | 23 | 8991 |
| HSV1-UL19-4393 | + | UUCCGCGACCCAUCGGACCCGCCC | 24 | 8992 |
| HSV1-UL19-4394 | + | ACCGGAUUGAACAGGCCC | 18 | 8993 |
| HSV1-UL19-4395 | + | GACCGGAUUGAACAGGCCC | 19 | 8994 |
| HSV1-UL19-1056 | + | UGACCGGAUUGAACAGGCCC | 20 | 1442 |
| HSV1-UL19-4396 | + | AUGACCGGAUUGAACAGGCCC | 21 | 8995 |
| HSV1-UL19-4397 | + | CAUGACCGGAUUGAACAGGCCC | 22 | 8996 |
| HSV1-UL19-4398 | + | CCAUGACCGGAUUGAACAGGCCC | 23 | 8997 |
| HSV1-UL19-4399 | + | UCCAUGACCGGAUUGAACAGGCCC | 24 | 8998 |
| HSV1-UL19-4400 | + | GCCACCUGGAGCUGGCCC | 18 | 8999 |
| HSV1-UL19-4401 | + | GGCCACCUGGAGCUGGCCC | 19 | 9000 |
| HSV1-UL19-1316 | + | GGGCCACCUGGAGCUGGCCC | 20 | 1702 |
| HSV1-UL19-4402 | + | CGGGCCACCUGGAGCUGGCCC | 21 | 9001 |
| HSV1-UL19-4403 | + | CCGGGCCACCUGGAGCUGGCCC | 22 | 9002 |
| HSV1-UL19-4404 | + | GCCGGGCCACCUGGAGCUGGCCC | 23 | 9003 |
| HSV1-UL19-4405 | + | UGCCGGGCCACCUGGAGCUGGCCC | 24 | 9004 |
| HSV1-UL19-4406 | + | CGGUGGCCCGCCUUGCCC | 18 | 9005 |
| HSV1-UL19-4407 | + | GCGGUGGCCCGCCUUGCCC | 19 | 9006 |
| HSV1-UL19-995 | + | GGCGGUGGCCCGCCUUGCCC | 20 | 1381 |
| HSV1-UL19-4408 | + | CGGCGGUGGCCCGCCUUGCCC | 21 | 9007 |
| HSV1-UL19-4409 | + | CCGGCGGUGGCCCGCCUUGCCC | 22 | 9008 |
| HSV1-UL19-4410 | + | CCCGGCGGUGGCCCGCCUUGCCC | 23 | 9009 |
| HSV1-UL19-4411 | + | UCCCGGCGGUGGCCCGCCUUGCCC | 24 | 9010 |
| HSV1-UL19-4412 | + | AGGUGGCCUGGAUCUCCC | 18 | 9011 |
| HSV1-UL19-4413 | + | CAGGUGGCCUGGAUCUCCC | 19 | 9012 |
| HSV1-UL19-2699 | + | CCAGGUGGCCUGGAUCUCCC | 20 | 2782 |
| HSV1-UL19-4414 | + | GCCAGGUGGCCUGGAUCUCCC | 21 | 9013 |
| HSV1-UL19-4415 | + | CGCCAGGUGGCCUGGAUCUCCC | 22 | 9014 |
| HSV1-UL19-4416 | + | GCGCCAGGUGGCCUGGAUCUCCC | 23 | 9015 |
| HSV1-UL19-4417 | + | CGCGCCAGGUGGCCUGGAUCUCCC | 24 | 9016 |
| HSV1-UL19-4418 | + | UCCUCGGGAAGGUCUCCC | 18 | 9017 |
| HSV1-UL19-4419 | + | CUCCUCGGGAAGGUCUCCC | 19 | 9018 |
| HSV1-UL19-2721 | + | ACUCCUCGGGAAGGUCUCCC | 20 | 2800 |
| HSV1-UL19-4420 | + | CACUCCUCGGGAAGGUCUCCC | 21 | 9019 |
| HSV1-UL19-4421 | + | GCACUCCUCGGGAAGGUCUCCC | 22 | 9020 |
| HSV1-UL19-4422 | + | UGCACUCCUCGGGAAGGUCUCCC | 23 | 9021 |
| HSV1-UL19-4423 | + | AUGCACUCCUCGGGAAGGUCUCCC | 24 | 9022 |
| HSV1-UL19-4424 | + | UAUUACAGAGCCAGUCCC | 18 | 9023 |
| HSV1-UL19-4425 | + | UUAUUACAGAGCCAGUCCC | 19 | 9024 |
| HSV1-UL19-4426 | + | UUUAUUACAGAGCCAGUCCC | 20 | 9025 |
| HSV1-UL19-4427 | + | GUUUAUUACAGAGCCAGUCCC | 21 | 9026 |
| HSV1-UL19-4428 | + | GGUUUAUUACAGAGCCAGUCCC | 22 | 9027 |
| HSV1-UL19-4429 | + | CGGUUUAUUACAGAGCCAGUCCC | 23 | 9028 |
| HSV1-UL19-4430 | + | GCGGUUUAUUACAGAGCCAGUCCC | 24 | 9029 |
| HSV1-UL19-4431 | + | CCCCCGGCGGCGCGUCCC | 18 | 9030 |
| HSV1-UL19-4432 | + | GCCCCCGGCGGCGCGUCCC | 19 | 9031 |
| HSV1-UL19-1376 | + | CGCCCCCGGCGGCGCGUCCC | 20 | 1762 |
| HSV1-UL19-4433 | + | ACGCCCCCGGCGGCGCGUCCC | 21 | 9032 |
| HSV1-UL19-4434 | + | AACGCCCCCGGCGGCGCGUCCC | 22 | 9033 |
| HSV1-UL19-4435 | + | AAACGCCCCCGGCGGCGCGUCCC | 23 | 9034 |
| HSV1-UL19-4436 | + | UAAACGCCCCCGGCGGCGCGUCCC | 24 | 9035 |
| HSV1-UL19-4437 | + | AUCGGCAACAACAAAGCC | 18 | 9036 |
| HSV1-UL19-4438 | + | CAUCGGCAACAACAAAGCC | 19 | 9037 |
| HSV1-UL19-983 | + | GCAUCGGCAACAACAAAGCC | 20 | 1369 |
| HSV1-UL19-4439 | + | UGCAUCGGCAACAACAAAGCC | 21 | 9038 |
| HSV1-UL19-4440 | + | UUGCAUCGGCAACAACAAAGCC | 22 | 9039 |
| HSV1-UL19-4441 | + | GUUGCAUCGGCAACAACAAAGCC | 23 | 9040 |
| HSV1-UL19-4442 | + | CGUUGCAUCGGCAACAACAAAGCC | 24 | 9041 |
| HSV1-UL19-4443 | + | UAGUUGGCCCCCAGAGCC | 18 | 9042 |
| HSV1-UL19-4444 | + | GUAGUUGGCCCCCAGAGCC | 19 | 9043 |
| HSV1-UL19-1297 | + | AGUAGUUGGCCCCCAGAGCC | 20 | 1683 |
| HSV1-UL19-4445 | + | AAGUAGUUGGCCCCCAGAGCC | 21 | 9044 |
| HSV1-UL19-4446 | + | AAAGUAGUUGGCCCCCAGAGCC | 22 | 9045 |
| HSV1-UL19-4447 | + | AAAAGUAGUUGGCCCCCAGAGCC | 23 | 9046 |
| HSV1-UL19-4448 | + | GAAAAGUAGUUGGCCCCCAGAGCC | 24 | 9047 |
| HSV1-UL19-4449 | + | GUGCCGCGCUCAAACGCC | 18 | 9048 |
| HSV1-UL19-4450 | + | CGUGCCGCGCUCAAACGCC | 19 | 9049 |
| HSV1-UL19-2613 | + | CCGUGCCGCGCUCAAACGCC | 20 | 2729 |
| HSV1-UL19-4451 | + | GCCGUGCCGCGCUCAAACGCC | 21 | 9050 |
| HSV1-UL19-4452 | + | GGCCGUGCCGCGCUCAAACGCC | 22 | 9051 |
| HSV1-UL19-4453 | + | CGGCCGUGCCGCGCUCAAACGCC | 23 | 9052 |
| HSV1-UL19-4454 | + | UCGGCCGUGCCGCGCUCAAACGCC | 24 | 9053 |
| HSV1-UL19-4455 | + | AUCUUGAAGUACCCCGCC | 18 | 9054 |
| HSV1-UL19-4456 | + | GAUCUUGAAGUACCCCGCC | 19 | 9055 |
| HSV1-UL19-2807 | + | UGAUCUUGAAGUACCCCGCC | 20 | 2854 |
| HSV1-UL19-4457 | + | CUGAUCUUGAAGUACCCCGCC | 21 | 9056 |
| HSV1-UL19-4458 | + | ACUGAUCUUGAAGUACCCCGCC | 22 | 9057 |
| HSV1-UL19-4459 | + | GACUGAUCUUGAAGUACCCCGCC | 23 | 9058 |
| HSV1-UL19-4460 | + | GGACUGAUCUUGAAGUACCCCGCC | 24 | 9059 |
| HSV1-UL19-4461 | + | GCCGCCGGACAUAGCGCC | 18 | 9060 |
| HSV1-UL19-4462 | + | CGCCGCCGGACAUAGCGCC | 19 | 9061 |
| HSV1-UL19-1135 | + | ACGCCGCCGGACAUAGCGCC | 20 | 1521 |
| HSV1-UL19-4463 | + | AACGCCGCCGGACAUAGCGCC | 21 | 9062 |
| HSV1-UL19-4464 | + | GAACGCCGCCGGACAUAGCGCC | 22 | 9063 |
| HSV1-UL19-4465 | + | GGAACGCCGCCGGACAUAGCGCC | 23 | 9064 |
| HSV1-UL19-4466 | + | CGGAACGCCGCCGGACAUAGCGCC | 24 | 9065 |
| HSV1-UL19-4467 | + | GCUGGGGCACUCCUCGCC | 18 | 9066 |
| HSV1-UL19-4468 | + | CGCUGGGGCACUCCUCGCC | 19 | 9067 |
| HSV1-UL19-1226 | + | UCGCUGGGGCACUCCUCGCC | 20 | 1612 |
| HSV1-UL19-4469 | + | GUCGCUGGGGCACUCCUCGCC | 21 | 9068 |
| HSV1-UL19-4470 | + | GGUCGCUGGGGCACUCCUCGCC | 22 | 9069 |
| HSV1-UL19-4471 | + | GGGUCGCUGGGGCACUCCUCGCC | 23 | 9070 |
| HSV1-UL19-4472 | + | GGGGUCGCUGGGGCACUCCUCGCC | 24 | 9071 |
| HSV1-UL19-4473 | + | AGCUGAUGAUGCAAGGCC | 18 | 9072 |
| HSV1-UL19-4474 | + | GAGCUGAUGAUGCAAGGCC | 19 | 9073 |
| HSV1-UL19-2810 | + | UGAGCUGAUGAUGCAAGGCC | 20 | 2856 |
| HSV1-UL19-4475 | + | UUGAGCUGAUGAUGCAAGGCC | 21 | 9074 |
| HSV1-UL19-4476 | + | CUUGAGCUGAUGAUGCAAGGCC | 22 | 9075 |
| HSV1-UL19-4477 | + | UCUUGAGCUGAUGAUGCAAGGCC | 23 | 9076 |
| HSV1-UL19-4478 | + | GUCUUGAGCUGAUGAUGCAAGGCC | 24 | 9077 |
| HSV1-UL19-4479 | + | GCCGUGGCCGCGAAGGCC | 18 | 9078 |
| HSV1-UL19-4480 | + | GGCCGUGGCCGCGAAGGCC | 19 | 9079 |
| HSV1-UL19-1387 | + | UGGCCGUGGCCGCGAAGGCC | 20 | 1773 |
| HSV1-UL19-4481 | + | UUGGCCGUGGCCGCGAAGGCC | 21 | 9080 |
| HSV1-UL19-4482 | + | GUUGGCCGUGGCCGCGAAGGCC | 22 | 9081 |
| HSV1-UL19-4483 | + | GGUUGGCCGUGGCCGCGAAGGCC | 23 | 9082 |
| HSV1-UL19-4484 | + | GGGUUGGCCGUGGCCGCGAAGGCC | 24 | 9083 |
| HSV1-UL19-4485 | + | GACCGGAUUGAACAGGCC | 18 | 9084 |
| HSV1-UL19-4486 | + | UGACCGGAUUGAACAGGCC | 19 | 9085 |
| HSV1-UL19-2656 | + | AUGACCGGAUUGAACAGGCC | 20 | 2757 |
| HSV1-UL19-4487 | + | CAUGACCGGAUUGAACAGGCC | 21 | 9086 |
| HSV1-UL19-4488 | + | CCAUGACCGGAUUGAACAGGCC | 22 | 9087 |
| HSV1-UL19-4489 | + | UCCAUGACCGGAUUGAACAGGCC | 23 | 9088 |
| HSV1-UL19-4490 | + | UUCCAUGACCGGAUUGAACAGGCC | 24 | 9089 |
| HSV1-UL19-4491 | + | CACCCCCAGCUCCAGGCC | 18 | 9090 |
| HSV1-UL19-4492 | + | CCACCCCCAGCUCCAGGCC | 19 | 9091 |
| HSV1-UL19-2706 | + | CCCACCCCCAGCUCCAGGCC | 20 | 2787 |
| HSV1-UL19-4493 | + | UCCCACCCCCAGCUCCAGGCC | 21 | 9092 |
| HSV1-UL19-4494 | + | GUCCCACCCCCAGCUCCAGGCC | 22 | 9093 |
| HSV1-UL19-4495 | + | CGUCCCACCCCCAGCUCCAGGCC | 23 | 9094 |
| HSV1-UL19-4496 | + | GCGUCCCACCCCCAGCUCCAGGCC | 24 | 9095 |
| HSV1-UL19-4497 | + | GUCAGACGGGGCACGGCC | 18 | 9096 |
| HSV1-UL19-4498 | + | CGUCAGACGGGGCACGGCC | 19 | 9097 |
| HSV1-UL19-2625 | + | GCGUCAGACGGGGCACGGCC | 20 | 2738 |
| HSV1-UL19-4499 | + | UGCGUCAGACGGGGCACGGCC | 21 | 9098 |
| HSV1-UL19-4500 | + | AUGCGUCAGACGGGGCACGGCC | 22 | 9099 |
| HSV1-UL19-4501 | + | CAUGCGUCAGACGGGGCACGGCC | 23 | 9100 |
| HSV1-UL19-4502 | + | GCAUGCGUCAGACGGGGCACGGCC | 24 | 9101 |
| HSV1-UL19-4503 | + | AUCUCCCCCGGGCCGGCC | 18 | 9102 |
| HSV1-UL19-4504 | + | GAUCUCCCCCGGGCCGGCC | 19 | 9103 |
| HSV1-UL19-1125 | + | GGAUCUCCCCCGGGCCGGCC | 20 | 1511 |
| HSV1-UL19-4505 | + | UGGAUCUCCCCCGGGCCGGCC | 21 | 9104 |
| HSV1-UL19-4506 | + | CUGGAUCUCCCCCGGGCCGGCC | 22 | 9105 |
| HSV1-UL19-4507 | + | CCUGGAUCUCCCCCGGGCCGGCC | 23 | 9106 |
| HSV1-UL19-4508 | + | GCCUGGAUCUCCCCCGGGCCGGCC | 24 | 9107 |
| HSV1-UL19-4509 | + | GACAGGUCGCGCAGGGCC | 18 | 9108 |
| HSV1-UL19-4510 | + | CGACAGGUCGCGCAGGGCC | 19 | 9109 |
| HSV1-UL19-1285 | + | GCGACAGGUCGCGCAGGGCC | 20 | 1671 |
| HSV1-UL19-4511 | + | CGCGACAGGUCGCGCAGGGCC | 21 | 9110 |
| HSV1-UL19-4512 | + | CCGCGACAGGUCGCGCAGGGCC | 22 | 9111 |
| HSV1-UL19-4513 | + | GCCGCGACAGGUCGCGCAGGGCC | 23 | 9112 |
| HSV1-UL19-4514 | + | UGCCGCGACAGGUCGCGCAGGGCC | 24 | 9113 |
| HSV1-UL19-4515 | + | GGCCACCUGGAGCUGGCC | 18 | 9114 |
| HSV1-UL19-4516 | + | GGGCCACCUGGAGCUGGCC | 19 | 9115 |
| HSV1-UL19-2821 | + | CGGGCCACCUGGAGCUGGCC | 20 | 2864 |
| HSV1-UL19-4517 | + | CCGGGCCACCUGGAGCUGGCC | 21 | 9116 |
| HSV1-UL19-4518 | + | GCCGGGCCACCUGGAGCUGGCC | 22 | 9117 |
| HSV1-UL19-4519 | + | UGCCGGGCCACCUGGAGCUGGCC | 23 | 9118 |
| HSV1-UL19-4520 | + | GUGCCGGGCCACCUGGAGCUGGCC | 24 | 9119 |
| HSV1-UL19-4521 | + | CACGGGUUGGCCGUGGCC | 18 | 9120 |
| HSV1-UL19-4522 | + | CCACGGGUUGGCCGUGGCC | 19 | 9121 |
| HSV1-UL19-2864 | + | CCCACGGGUUGGCCGUGGCC | 20 | 2889 |
| HSV1-UL19-4523 | + | GCCCACGGGUUGGCCGUGGCC | 21 | 9122 |
| HSV1-UL19-4524 | + | CGCCCACGGGUUGGCCGUGGCC | 22 | 9123 |
| HSV1-UL19-4525 | + | ACGCCCACGGGUUGGCCGUGGCC | 23 | 9124 |
| HSV1-UL19-4526 | + | GACGCCCACGGGUUGGCCGUGGCC | 24 | 9125 |
| HSV1-UL19-4527 | + | UCGCUGGCGGCGGUGGCC | 18 | 9126 |
| HSV1-UL19-4528 | + | GUCGCUGGCGGCGGUGGCC | 19 | 9127 |
| HSV1-UL19-2875 | + | CGUCGCUGGCGGCGGUGGCC | 20 | 2899 |
| HSV1-UL19-4529 | + | ACGUCGCUGGCGGCGGUGGCC | 21 | 9128 |
| HSV1-UL19-4530 | + | UACGUCGCUGGCGGCGGUGGCC | 22 | 9129 |
| HSV1-UL19-4531 | + | GUACGUCGCUGGCGGCGGUGGCC | 23 | 9130 |
| HSV1-UL19-4532 | + | UGUACGUCGCUGGCGGCGGUGGCC | 24 | 9131 |
| HSV1-UL19-4533 | + | GCGGGGAUCCGGGUGGCC | 18 | 9132 |
| HSV1-UL19-4534 | + | CGCGGGGAUCCGGGUGGCC | 19 | 9133 |
| HSV1-UL19-1067 | + | GCGCGGGGAUCCGGGUGGCC | 20 | 1453 |
| HSV1-UL19-4535 | + | GGCGCGGGGAUCCGGGUGGCC | 21 | 9134 |
| HSV1-UL19-4536 | + | AGGCGCGGGGAUCCGGGUGGCC | 22 | 9135 |
| HSV1-UL19-4537 | + | AAGGCGCGGGGAUCCGGGUGGCC | 23 | 9136 |
| HSV1-UL19-4538 | + | GAAGGCGCGGGGAUCCGGGUGGCC | 24 | 9137 |
| HSV1-UL19-4539 | + | GCGGUGGCCCGCCUUGCC | 18 | 9138 |
| HSV1-UL19-4540 | + | GGCGGUGGCCCGCCUUGCC | 19 | 9139 |
| HSV1-UL19-2620 | + | CGGCGGUGGCCCGCCUUGCC | 20 | 2734 |
| HSV1-UL19-4541 | + | CCGGCGGUGGCCCGCCUUGCC | 21 | 9140 |
| HSV1-UL19-4542 | + | CCCGGCGGUGGCCCGCCUUGCC | 22 | 9141 |
| HSV1-UL19-4543 | + | UCCCGGCGGUGGCCCGCCUUGCC | 23 | 9142 |
| HSV1-UL19-4544 | + | CUCCCGGCGGUGGCCCGCCUUGCC | 24 | 9143 |
| HSV1-UL19-4545 | + | UACACGGCCAUGCACUCC | 18 | 9144 |
| HSV1-UL19-4546 | + | GUACACGGCCAUGCACUCC | 19 | 9145 |
| HSV1-UL19-2724 | + | GGUACACGGCCAUGCACUCC | 20 | 2801 |
| HSV1-UL19-4547 | + | CGGUACACGGCCAUGCACUCC | 21 | 9146 |
| HSV1-UL19-4548 | + | CCGGUACACGGCCAUGCACUCC | 22 | 9147 |
| HSV1-UL19-4549 | + | CCCGGUACACGGCCAUGCACUCC | 23 | 9148 |
| HSV1-UL19-4550 | + | UCCCGGUACACGGCCAUGCACUCC | 24 | 9149 |
| HSV1-UL19-4551 | + | GCCUCCGACGCGCGCUCC | 18 | 9150 |
| HSV1-UL19-4552 | + | CGCCUCCGACGCGCGCUCC | 19 | 9151 |
| HSV1-UL19-2818 | + | ACGCCUCCGACGCGCGCUCC | 20 | 2862 |
| HSV1-UL19-4553 | + | UACGCCUCCGACGCGCGCUCC | 21 | 9152 |
| HSV1-UL19-4554 | + | GUACGCCUCCGACGCGCGCUCC | 22 | 9153 |
| HSV1-UL19-4555 | + | AGUACGCCUCCGACGCGCGCUCC | 23 | 9154 |
| HSV1-UL19-4556 | + | AAGUACGCCUCCGACGCGCGCUCC | 24 | 9155 |
| HSV1-UL19-4557 | + | GCCCCCGGCGGCGCGUCC | 18 | 9156 |
| HSV1-UL19-4558 | + | CGCCCCCGGCGGCGCGUCC | 19 | 9157 |
| HSV1-UL19-2858 | + | ACGCCCCCGGCGGCGCGUCC | 20 | 2884 |
| HSV1-UL19-4559 | + | AACGCCCCCGGCGGCGCGUCC | 21 | 9158 |
| HSV1-UL19-4560 | + | AAACGCCCCCGGCGGCGCGUCC | 22 | 9159 |
| HSV1-UL19-4561 | + | UAAACGCCCCCGGCGGCGCGUCC | 23 | 9160 |
| HSV1-UL19-4562 | + | GUAAACGCCCCCGGCGGCGCGUCC | 24 | 9161 |
| HSV1-UL19-4563 | + | UCCAGGCCCCGGGCGUCC | 18 | 9162 |
| HSV1-UL19-4564 | + | CUCCAGGCCCCGGGCGUCC | 19 | 9163 |
| HSV1-UL19-1137 | + | GCUCCAGGCCCCGGGCGUCC | 20 | 1523 |
| HSV1-UL19-4565 | + | AGCUCCAGGCCCCGGGCGUCC | 21 | 9164 |
| HSV1-UL19-4566 | + | CAGCUCCAGGCCCCGGGCGUCC | 22 | 9165 |
| HSV1-UL19-4567 | + | CCAGCUCCAGGCCCCGGGCGUCC | 23 | 9166 |
| HSV1-UL19-4568 | + | CCCAGCUCCAGGCCCCGGGCGUCC | 24 | 9167 |
| HSV1-UL19-4569 | + | GUGGCCGUGGUGAGGUCC | 18 | 9168 |
| HSV1-UL19-4570 | + | CGUGGCCGUGGUGAGGUCC | 19 | 9169 |
| HSV1-UL19-2621 | + | GCGUGGCCGUGGUGAGGUCC | 20 | 2735 |
| HSV1-UL19-4571 | + | UGCGUGGCCGUGGUGAGGUCC | 21 | 9170 |
| HSV1-UL19-4572 | + | CUGCGUGGCCGUGGUGAGGUCC | 22 | 9171 |
| HSV1-UL19-4573 | + | GCUGCGUGGCCGUGGUGAGGUCC | 23 | 9172 |
| HSV1-UL19-4574 | + | GGCUGCGUGGCCGUGGUGAGGUCC | 24 | 9173 |
| HSV1-UL19-4575 | + | ACGGCUGCCGCGUUGUCC | 18 | 9174 |
| HSV1-UL19-4576 | + | CACGGCUGCCGCGUUGUCC | 19 | 9175 |
| HSV1-UL19-1344 | + | ACACGGCUGCCGCGUUGUCC | 20 | 1730 |
| HSV1-UL19-4577 | + | UACACGGCUGCCGCGUUGUCC | 21 | 9176 |
| HSV1-UL19-4578 | + | GUACACGGCUGCCGCGUUGUCC | 22 | 9177 |
| HSV1-UL19-4579 | + | GGUACACGGCUGCCGCGUUGUCC | 23 | 9178 |
| HSV1-UL19-4580 | + | AGGUACACGGCUGCCGCGUUGUCC | 24 | 9179 |
| HSV1-UL19-4581 | + | CAUCGGCAACAACAAAGC | 18 | 9180 |
| HSV1-UL19-4582 | + | GCAUCGGCAACAACAAAGC | 19 | 9181 |
| HSV1-UL19-2615 | + | UGCAUCGGCAACAACAAAGC | 20 | 2730 |
| HSV1-UL19-4583 | + | UUGCAUCGGCAACAACAAAGC | 21 | 9182 |
| HSV1-UL19-4584 | + | GUUGCAUCGGCAACAACAAAGC | 22 | 9183 |
| HSV1-UL19-4585 | + | CGUUGCAUCGGCAACAACAAAGC | 23 | 9184 |
| HSV1-UL19-4586 | + | UC GUUGCAUC GGCAACAACAAAGC | 24 | 9185 |
| HSV1-UL19-4587 | + | ACGUAAAGAACUUAAAGC | 18 | 9186 |
| HSV1-UL19-4588 | + | GACGUAAAGAACUUAAAGC | 19 | 9187 |
| HSV1-UL19-1401 | + | CGACGUAAAGAACUUAAAGC | 20 | 1787 |
| HSV1-UL19-4589 | + | CCGACGUAAAGAACUUAAAGC | 21 | 9188 |
| HSV1-UL19-4590 | + | GCCGACGUAAAGAACUUAAAGC | 22 | 9189 |
| HSV1-UL19-4591 | + | GGCCGACGUAAAGAACUUAAAGC | 23 | 9190 |
| HSV1-UL19-4592 | + | CGGCCGACGUAAAGAACUUAAAGC | 24 | 9191 |
| HSV1-UL19-4593 | + | UGGGCCAGCACCUGCAGC | 18 | 9192 |
| HSV1-UL19-4594 | + | GUGGGCCAGCACCUGCAGC | 19 | 9193 |
| HSV1-UL19-2763 | + | UGUGGGCCAGCACCUGCAGC | 20 | 2830 |
| HSV1-UL19-4595 | + | UUGUGGGCCAGCACCUGCAGC | 21 | 9194 |
| HSV1-UL19-4596 | + | GUUGUGGGCCAGCACCUGCAGC | 22 | 9195 |
| HSV1-UL19-4597 | + | UGUUGUGGGCCAGCACCUGCAGC | 23 | 9196 |
| HSV1-UL19-4598 | + | AUGUUGUGGGCCAGCACCUGCAGC | 24 | 9197 |
| HSV1-UL19-4599 | + | GUAGUUGGCCCCCAGAGC | 18 | 9198 |
| HSV1-UL19-4600 | + | AGUAGUUGGCCCCCAGAGC | 19 | 9199 |
| HSV1-UL19-1298 | + | AAGUAGUUGGCCCCCAGAGC | 20 | 1684 |
| HSV1-UL19-4601 | + | AAAGUAGUUGGCCCCCAGAGC | 21 | 9200 |
| HSV1-UL19-4602 | + | AAAAGUAGUUGGCCCCCAGAGC | 22 | 9201 |
| HSV1-UL19-4603 | + | GAAAAGUAGUUGGCCCCCAGAGC | 23 | 9202 |
| HSV1-UL19-4604 | + | CGAAAAGUAGUUGGCCCCCAGAGC | 24 | 9203 |
| HSV1-UL19-4605 | + | GCGCGCGGCUGGGUGAGC | 18 | 9204 |
| HSV1-UL19-4606 | + | CGCGCGCGGCUGGGUGAGC | 19 | 9205 |
| HSV1-UL19-2825 | + | UCGCGCGCGGCUGGGUGAGC | 20 | 2867 |
| HSV1-UL19-4607 | + | UUCGCGCGCGGCUGGGUGAGC | 21 | 9206 |
| HSV1-UL19-4608 | + | GUUCGCGCGCGGCUGGGUGAGC | 22 | 9207 |
| HSV1-UL19-4609 | + | CGUUCGCGCGCGGCUGGGUGAGC | 23 | 9208 |
| HSV1-UL19-4610 | + | ACGUUCGCGCGCGGCUGGGUGAGC | 24 | 9209 |
| HSV1-UL19-4611 | + | GUGCAGCAGCGGCCCCGC | 18 | 9210 |
| HSV1-UL19-4612 | + | GGUGCAGCAGCGGCCCCGC | 19 | 9211 |
| HSV1-UL19-2741 | + | CGGUGCAGCAGCGGCCCCGC | 20 | 2816 |
| HSV1-UL19-4613 | + | GCGGUGCAGCAGCGGCCCCGC | 21 | 9212 |
| HSV1-UL19-4614 | + | CGCGGUGCAGCAGCGGCCCCGC | 22 | 9213 |
| HSV1-UL19-4615 | + | CCGCGGUGCAGCAGCGGCCCCGC | 23 | 9214 |
| HSV1-UL19-4616 | + | CCCGCGGUGCAGCAGCGGCCCCGC | 24 | 9215 |
| HSV1-UL19-4617 | + | GGCCCCGGCGAACAGCGC | 18 | 9216 |
| HSV1-UL19-4618 | + | CGGCCCCGGCGAACAGCGC | 19 | 9217 |
| HSV1-UL19-2783 | + | UCGGCCCCGGCGAACAGCGC | 20 | 2842 |
| HSV1-UL19-4619 | + | GUCGGCCCCGGCGAACAGCGC | 21 | 9218 |
| HSV1-UL19-4620 | + | GGUCGGCCCCGGCGAACAGCGC | 22 | 9219 |
| HSV1-UL19-4621 | + | UGGUCGGCCCCGGCGAACAGCGC | 23 | 9220 |
| HSV1-UL19-4622 | + | GUGGUCGGCCCCGGCGAACAGCGC | 24 | 9221 |
| HSV1-UL19-4623 | + | CGCCGCCGGACAUAGCGC | 18 | 9222 |
| HSV1-UL19-4624 | + | ACGCCGCCGGACAUAGCGC | 19 | 9223 |
| HSV1-UL19-2702 | + | AACGCCGCCGGACAUAGCGC | 20 | 2784 |
| HSV1-UL19-4625 | + | GAACGCCGCCGGACAUAGCGC | 21 | 9224 |
| HSV1-UL19-4626 | + | GGAACGCCGCCGGACAUAGCGC | 22 | 9225 |
| HSV1-UL19-4627 | + | CGGAACGCCGCCGGACAUAGCGC | 23 | 9226 |
| HSV1-UL19-4628 | + | CCGGAACGCCGCCGGACAUAGCGC | 24 | 9227 |
| HSV1-UL19-4629 | + | GAAGUGGGUCUCCCGCGC | 18 | 9228 |
| HSV1-UL19-4630 | + | CGAAGUGGGUCUCCCGCGC | 19 | 9229 |
| HSV1-UL19-2893 | + | GCGAAGUGGGUCUCCCGCGC | 20 | 2911 |
| HSV1-UL19-4631 | + | CGCGAAGUGGGUCUCCCGCGC | 21 | 9230 |
| HSV1-UL19-4632 | + | GCGCGAAGUGGGUCUCCCGCGC | 22 | 9231 |
| HSV1-UL19-4633 | + | UGCGCGAAGUGGGUCUCCCGCGC | 23 | 9232 |
| HSV1-UL19-4634 | + | CUGCGCGAAGUGGGUCUCCCGCGC | 24 | 9233 |
| HSV1-UL19-4635 | + | CGCUGGGGCACUCCUCGC | 18 | 9234 |
| HSV1-UL19-4636 | + | UCGCUGGGGCACUCCUCGC | 19 | 9235 |
| HSV1-UL19-1227 | + | GUCGCUGGGGCACUCCUCGC | 20 | 1613 |
| HSV1-UL19-4637 | + | GGUCGCUGGGGCACUCCUCGC | 21 | 9236 |
| HSV1-UL19-4638 | + | GGGUCGCUGGGGCACUCCUCGC | 22 | 9237 |
| HSV1-UL19-4639 | + | GGGGUCGCUGGGGCACUCCUCGC | 23 | 9238 |
| HSV1-UL19-4640 | + | CGGGGUCGCUGGGGCACUCCUCGC | 24 | 9239 |
| HSV1-UL19-4641 | + | CUCGGCGACCAGGGUCGC | 18 | 9240 |
| HSV1-UL19-4642 | + | GCUCGGCGACCAGGGUCGC | 19 | 9241 |
| HSV1-UL19-2616 | + | AGCUCGGCGACCAGGGUCGC | 20 | 2731 |
| HSV1-UL19-4643 | + | UAGCUCGGCGACCAGGGUCGC | 21 | 9242 |
| HSV1-UL19-4644 | + | UUAGCUCGGCGACCAGGGUCGC | 22 | 9243 |
| HSV1-UL19-4645 | + | UUUAGCUCGGCGACCAGGGUCGC | 23 | 9244 |
| HSV1-UL19-4646 | + | CUUUAGCUCGGCGACCAGGGUCGC | 24 | 9245 |
| HSV1-UL19-4647 | + | GGUCCGUCACGGGGUCGC | 18 | 9246 |
| HSV1-UL19-4648 | + | GGGUCCGUCACGGGGUCGC | 19 | 9247 |
| HSV1-UL19-1230 | + | GGGGUCCGUCACGGGGUCGC | 20 | 1616 |
| HSV1-UL19-4649 | + | CGGGGUCCGUCACGGGGUCGC | 21 | 9248 |
| HSV1-UL19-4650 | + | GCGGGGUCCGUCACGGGGUCGC | 22 | 9249 |
| HSV1-UL19-4651 | + | CGCGGGGUCCGUCACGGGGUCGC | 23 | 9250 |
| HSV1-UL19-4652 | + | GCGCGGGGUCCGUCACGGGGUCGC | 24 | 9251 |
| HSV1-UL19-4653 | + | GGCCGUGGCCGCGAAGGC | 18 | 9252 |
| HSV1-UL19-4654 | + | UGGCCGUGGCCGCGAAGGC | 19 | 9253 |
| HSV1-UL19-2863 | + | UUGGCCGUGGCCGCGAAGGC | 20 | 2888 |
| HSV1-UL19-4655 | + | GUUGGCCGUGGCCGCGAAGGC | 21 | 9254 |
| HSV1-UL19-4656 | + | GGUUGGCCGUGGCCGCGAAGGC | 22 | 9255 |
| HSV1-UL19-4657 | + | GGGUUGGCCGUGGCCGCGAAGGC | 23 | 9256 |
| HSV1-UL19-4658 | + | CGGGUUGGCCGUGGCCGCGAAGGC | 24 | 9257 |
| HSV1-UL19-4659 | + | CUGGCGGGGCGGGAAGGC | 18 | 9258 |
| HSV1-UL19-4660 | + | GCUGGCGGGGCGGGAAGGC | 19 | 9259 |
| HSV1-UL19-2668 | + | AGCUGGCGGGGCGGGAAGGC | 20 | 2763 |
| HSV1-UL19-4661 | + | CAGCUGGCGGGGCGGGAAGGC | 21 | 9260 |
| HSV1-UL19-4662 | + | ACAGCUGGCGGGGCGGGAAGGC | 22 | 9261 |
| HSV1-UL19-4663 | + | AACAGCUGGCGGGGCGGGAAGGC | 23 | 9262 |
| HSV1-UL19-4664 | + | AAACAGCUGGCGGGGCGGGAAGGC | 24 | 9263 |
| HSV1-UL19-3387 | + | AAACGCCCCGAGGACGGC | 18 | 7986 |
| HSV1-UL19-3388 | + | CAAACGCCCCGAGGACGGC | 19 | 7987 |
| HSV1-UL19-2611 | + | UCAAACGCCCCGAGGACGGC | 20 | 2727 |
| HSV1-UL19-4665 | + | CUCAAACGCCCCGAGGACGGC | 21 | 9264 |
| HSV1-UL19-4666 | + | GCUCAAACGCCCCGAGGACGGC | 22 | 9265 |
| HSV1-UL19-4667 | + | CGCUCAAACGCCCCGAGGACGGC | 23 | 9266 |
| HSV1-UL19-4668 | + | GCGCUCAAACGCCCCGAGGACGGC | 24 | 9267 |
| HSV1-UL19-4669 | + | GAUCUCCCCCGGGCCGGC | 18 | 9268 |
| HSV1-UL19-4670 | + | GGAUCUCCCCCGGGCCGGC | 19 | 9269 |
| HSV1-UL19-1126 | + | UGGAUCUCCCCCGGGCCGGC | 20 | 1512 |
| HSV1-UL19-4671 | + | CUGGAUCUCCCCCGGGCCGGC | 21 | 9270 |
| HSV1-UL19-4672 | + | CCUGGAUCUCCCCCGGGCCGGC | 22 | 9271 |
| HSV1-UL19-4673 | + | GCCUGGAUCUCCCCCGGGCCGGC | 23 | 9272 |
| HSV1-UL19-4674 | + | GGCCUGGAUCUCCCCCGGGCCGGC | 24 | 9273 |
| HSV1-UL19-4675 | + | GUCUGCGGCGGGGCCGGC | 18 | 9274 |
| HSV1-UL19-4676 | + | UGUCUGCGGCGGGGCCGGC | 19 | 9275 |
| HSV1-UL19-1099 | + | AUGUCUGCGGCGGGGCCGGC | 20 | 1485 |
| HSV1-UL19-4677 | + | CAUGUCUGCGGCGGGGCCGGC | 21 | 9276 |
| HSV1-UL19-4678 | + | GCAUGUCUGCGGCGGGGCCGGC | 22 | 9277 |
| HSV1-UL19-4679 | + | UGCAUGUCUGCGGCGGGGCCGGC | 23 | 9278 |
| HSV1-UL19-4680 | + | CUGCAUGUCUGCGGCGGGGCCGGC | 24 | 9279 |
| HSV1-UL19-4681 | + | AUCCGCGGCGUCCGCGGC | 18 | 9280 |
| HSV1-UL19-4682 | + | CAUCCGCGGCGUCCGCGGC | 19 | 9281 |
| HSV1-UL19-2739 | + | UCAUCCGCGGCGUCCGCGGC | 20 | 2814 |
| HSV1-UL19-4683 | + | GUCAUCCGCGGCGUCCGCGGC | 21 | 9282 |
| HSV1-UL19-4684 | + | GGUCAUCCGCGGCGUCCGCGGC | 22 | 9283 |
| HSV1-UL19-4685 | + | CGGUCAUCCGCGGCGUCCGCGGC | 23 | 9284 |
| HSV1-UL19-4686 | + | CCGGUCAUCCGCGGCGUCCGCGGC | 24 | 9285 |
| HSV1-UL19-4687 | + | CGACAGGUCGCGCAGGGC | 18 | 9286 |
| HSV1-UL19-4688 | + | GCGACAGGUCGCGCAGGGC | 19 | 9287 |
| HSV1-UL19-1286 | + | CGCGACAGGUCGCGCAGGGC | 20 | 1672 |
| HSV1-UL19-4689 | + | CCGCGACAGGUCGCGCAGGGC | 21 | 9288 |
| HSV1-UL19-4690 | + | GCCGCGACAGGUCGCGCAGGGC | 22 | 9289 |
| HSV1-UL19-4691 | + | UGCCGCGACAGGUCGCGCAGGGC | 23 | 9290 |
| HSV1-UL19-4692 | + | CUGCCGCGACAGGUCGCGCAGGGC | 24 | 9291 |
| HSV1-UL19-4693 | + | CUGGACCACGAGCCGGGC | 18 | 9292 |
| HSV1-UL19-4694 | + | ACUGGACCACGAGCCGGGC | 19 | 9293 |
| HSV1-UL19-2714 | + | CACUGGACCACGAGCCGGGC | 20 | 2793 |
| HSV1-UL19-4695 | + | GCACUGGACCACGAGCCGGGC | 21 | 9294 |
| HSV1-UL19-4696 | + | UGCACUGGACCACGAGCCGGGC | 22 | 9295 |
| HSV1-UL19-4697 | + | AUGCACUGGACCACGAGCCGGGC | 23 | 9296 |
| HSV1-UL19-4698 | + | GAUGCACUGGACCACGAGCCGGGC | 24 | 9297 |
| HSV1-UL19-4699 | + | AAAAACAGCUGGCGGGGC | 18 | 9298 |
| HSV1-UL19-4700 | + | AAAAAACAGCUGGCGGGGC | 19 | 9299 |
| HSV1-UL19-1076 | + | AAAAAAACAGCUGGCGGGGC | 20 | 1462 |
| HSV1-UL19-4701 | + | CAAAAAAACAGCUGGCGGGGC | 21 | 9300 |
| HSV1-UL19-4702 | + | CCAAAAAAACAGCUGGCGGGGC | 22 | 9301 |
| HSV1-UL19-4703 | + | CCCAAAAAAACAGCUGGCGGGGC | 23 | 9302 |
| HSV1-UL19-4704 | + | CCCCAAAAAAACAGCUGGCGGGGC | 24 | 9303 |
| HSV1-UL19-4705 | + | AUCCGGGUGGCCGGGGGC | 18 | 9304 |
| HSV1-UL19-4706 | + | GAUCCGGGUGGCCGGGGGC | 19 | 9305 |
| HSV1-UL19-1063 | + | GGAUCCGGGUGGCCGGGGGC | 20 | 1449 |
| HSV1-UL19-4707 | + | GGGAUCCGGGUGGCCGGGGGC | 21 | 9306 |
| HSV1-UL19-4708 | + | GGGGAUCCGGGUGGCCGGGGGC | 22 | 9307 |
| HSV1-UL19-4709 | + | CGGGGAUCCGGGUGGCCGGGGGC | 23 | 9308 |
| HSV1-UL19-4710 | + | GCGGGGAUCCGGGUGGCCGGGGGC | 24 | 9309 |
| HSV1-UL19-4711 | + | GUCAUCAACCAGCUGGGC | 18 | 9310 |
| HSV1-UL19-4712 | + | AGUCAUCAACCAGCUGGGC | 19 | 9311 |
| HSV1-UL19-2725 | + | AAGUCAUCAACCAGCUGGGC | 20 | 2802 |
| HSV1-UL19-4713 | + | AAAGUCAUCAACCAGCUGGGC | 21 | 9312 |
| HSV1-UL19-4714 | + | UAAAGUCAUCAACCAGCUGGGC | 22 | 9313 |
| HSV1-UL19-4715 | + | GUAAAGUCAUCAACCAGCUGGGC | 23 | 9314 |
| HSV1-UL19-4716 | + | GGUAAAGUCAUCAACCAGCUGGGC | 24 | 9315 |
| HSV1-UL19-4717 | + | CGCGGGGAUCCGGGUGGC | 18 | 9316 |
| HSV1-UL19-4718 | + | GCGCGGGGAUCCGGGUGGC | 19 | 9317 |
| HSV1-UL19-1068 | + | GGCGCGGGGAUCCGGGUGGC | 20 | 1454 |
| HSV1-UL19-4719 | + | AGGCGCGGGGAUCCGGGUGGC | 21 | 9318 |
| HSV1-UL19-4720 | + | AAGGCGCGGGGAUCCGGGUGGC | 22 | 9319 |
| HSV1-UL19-4721 | + | GAAGGCGCGGGGAUCCGGGUGGC | 23 | 9320 |
| HSV1-UL19-4722 | + | GGAAGGCGCGGGGAUCCGGGUGGC | 24 | 9321 |
| HSV1-UL19-4723 | + | AACGGCGGCGAUGGUGGC | 18 | 9322 |
| HSV1-UL19-4724 | + | GAACGGCGGCGAUGGUGGC | 19 | 9323 |
| HSV1-UL19-1145 | + | CGAACGGCGGCGAUGGUGGC | 20 | 1531 |
| HSV1-UL19-4725 | + | GCGAACGGCGGCGAUGGUGGC | 21 | 9324 |
| HSV1-UL19-4726 | + | CGCGAACGGCGGCGAUGGUGGC | 22 | 9325 |
| HSV1-UL19-4727 | + | CCGCGAACGGCGGCGAUGGUGGC | 23 | 9326 |
| HSV1-UL19-4728 | + | CCCGCGAACGGCGGCGAUGGUGGC | 24 | 9327 |
| HSV1-UL19-4729 | + | AUGGUAUGGUCCAGAUGC | 18 | 9328 |
| HSV1-UL19-4730 | + | GAUGGUAUGGUCCAGAUGC | 19 | 9329 |
| HSV1-UL19-1265 | + | GGAUGGUAUGGUCCAGAUGC | 20 | 1651 |
| HSV1-UL19-4731 | + | UGGAUGGUAUGGUCCAGAUGC | 21 | 9330 |
| HSV1-UL19-4732 | + | UUGGAUGGUAUGGUCCAGAUGC | 22 | 9331 |
| HSV1-UL19-4733 | + | UUUGGAUGGUAUGGUCCAGAUGC | 23 | 9332 |
| HSV1-UL19-4734 | + | UUUUGGAUGGUAUGGUCCAGAUGC | 24 | 9333 |
| HSV1-UL19-4735 | + | ACUUUCAGGAAGGACUGC | 18 | 9334 |
| HSV1-UL19-4736 | + | CACUUUCAGGAAGGACUGC | 19 | 9335 |
| HSV1-UL19-1019 | + | CCACUUUCAGGAAGGACUGC | 20 | 1405 |
| HSV1-UL19-4737 | + | UCCACUUUCAGGAAGGACUGC | 21 | 9336 |
| HSV1-UL19-4738 | + | CUCCACUUUCAGGAAGGACUGC | 22 | 9337 |
| HSV1-UL19-4739 | + | CCUCCACUUUCAGGAAGGACUGC | 23 | 9338 |
| HSV1-UL19-4740 | + | UCCUCCACUUUCAGGAAGGACUGC | 24 | 9339 |
| HSV1-UL19-4741 | + | UCAUAGACGAGAUACUGC | 18 | 9340 |
| HSV1-UL19-4742 | + | GUCAUAGACGAGAUACUGC | 19 | 9341 |
| HSV1-UL19-2895 | + | CGUCAUAGACGAGAUACUGC | 20 | 2913 |
| HSV1-UL19-4743 | + | GC GUCAUAGAC GAGAUACUGC | 21 | 9342 |
| HSV1-UL19-4744 | + | UGC GUCAUAGAC GAGAUACUGC | 22 | 9343 |
| HSV1-UL19-4745 | + | AUGCGUCAUAGACGAGAUACUGC | 23 | 9344 |
| HSV1-UL19-4746 | + | GAUGCGUCAUAGACGAGAUACUGC | 24 | 9345 |
| HSV1-UL19-4747 | + | ACCAGAUUGGCCGGGUGC | 18 | 9346 |
| HSV1-UL19-4748 | + | CACCAGAUUGGCCGGGUGC | 19 | 9347 |
| HSV1-UL19-1240 | + | CCACCAGAUUGGCCGGGUGC | 20 | 1626 |
| HSV1-UL19-4749 | + | GCCACCAGAUUGGCCGGGUGC | 21 | 9348 |
| HSV1-UL19-4750 | + | GGCCACCAGAUUGGCCGGGUGC | 22 | 9349 |
| HSV1-UL19-4751 | + | UGGCCACCAGAUUGGCCGGGUGC | 23 | 9350 |
| HSV1-UL19-4752 | + | UUGGCCACCAGAUUGGCCGGGUGC | 24 | 9351 |
| HSV1-UL19-4753 | + | GCCGGGUGCAGGGGGUGC | 18 | 9352 |
| HSV1-UL19-4754 | + | GGCCGGGUGCAGGGGGUGC | 19 | 9353 |
| HSV1-UL19-2756 | + | UGGCCGGGUGCAGGGGGUGC | 20 | 2825 |
| HSV1-UL19-4755 | + | UUGGCCGGGUGCAGGGGGUGC | 21 | 9354 |
| HSV1-UL19-4756 | + | AUUGGCCGGGUGCAGGGGGUGC | 22 | 9355 |
| HSV1-UL19-4757 | + | GAUUGGCCGGGUGCAGGGGGUGC | 23 | 9356 |
| HSV1-UL19-4758 | + | AGAUUGGCCGGGUGCAGGGGGUGC | 24 | 9357 |
| HSV1-UL19-4759 | + | CGCACGGCCUUGCCCAUC | 18 | 9358 |
| HSV1-UL19-4760 | + | UCGCACGGCCUUGCCCAUC | 19 | 9359 |
| HSV1-UL19-2637 | + | UUCGCACGGCCUUGCCCAUC | 20 | 2746 |
| HSV1-UL19-4761 | + | CUUCGCACGGCCUUGCCCAUC | 21 | 9360 |
| HSV1-UL19-4762 | + | ACUUCGCACGGCCUUGCCCAUC | 22 | 9361 |
| HSV1-UL19-4763 | + | GACUUCGCACGGCCUUGCCCAUC | 23 | 9362 |
| HSV1-UL19-4764 | + | AGACUUCGCACGGCCUUGCCCAUC | 24 | 9363 |
| HSV1-UL19-4765 | + | AAGGCCACGGGACUGAUC | 18 | 9364 |
| HSV1-UL19-4766 | + | CAAGGCCACGGGACUGAUC | 19 | 9365 |
| HSV1-UL19-2808 | + | GCAAGGCCACGGGACUGAUC | 20 | 2855 |
| HSV1-UL19-4767 | + | UGCAAGGCCACGGGACUGAUC | 21 | 9366 |
| HSV1-UL19-4768 | + | AUGCAAGGCCACGGGACUGAUC | 22 | 9367 |
| HSV1-UL19-4769 | + | GAUGCAAGGCCACGGGACUGAUC | 23 | 9368 |
| HSV1-UL19-4770 | + | UGAUGCAAGGCCACGGGACUGAUC | 24 | 9369 |
| HSV1-UL19-4771 | + | GGUGAGCGGGUAGGCCUC | 18 | 9370 |
| HSV1-UL19-4772 | + | AGGUGAGCGGGUAGGCCUC | 19 | 9371 |
| HSV1-UL19-2882 | + | CAGGUGAGCGGGUAGGCCUC | 20 | 2903 |
| HSV1-UL19-4773 | + | GCAGGUGAGCGGGUAGGCCUC | 21 | 9372 |
| HSV1-UL19-4774 | + | CGCAGGUGAGCGGGUAGGCCUC | 22 | 9373 |
| HSV1-UL19-4775 | + | GCGCAGGUGAGCGGGUAGGCCUC | 23 | 9374 |
| HSV1-UL19-4776 | + | GGCGCAGGUGAGCGGGUAGGCCUC | 24 | 9375 |
| HSV1-UL19-4777 | + | GCGCUUCUCCAGGGCCUC | 18 | 9376 |
| HSV1-UL19-4778 | + | UGCGCUUCUCCAGGGCCUC | 19 | 9377 |
| HSV1-UL19-2645 | + | AUGCGCUUCUCCAGGGCCUC | 20 | 2751 |
| HSV1-UL19-4779 | + | GAUGCGCUUCUCCAGGGCCUC | 21 | 9378 |
| HSV1-UL19-4780 | + | AGAUGCGCUUCUCCAGGGCCUC | 22 | 9379 |
| HSV1-UL19-4781 | + | UAGAUGCGCUUCUCCAGGGCCUC | 23 | 9380 |
| HSV1-UL19-4782 | + | GUAGAUGCGCUUCUCCAGGGCCUC | 24 | 9381 |
| HSV1-UL19-4783 | + | CACGGCCAUGCACUCCUC | 18 | 9382 |
| HSV1-UL19-4784 | + | ACACGGCCAUGCACUCCUC | 19 | 9383 |
| HSV1-UL19-1166 | + | UACACGGCCAUGCACUCCUC | 20 | 1552 |
| HSV1-UL19-4785 | + | GUACACGGCCAUGCACUCCUC | 21 | 9384 |
| HSV1-UL19-4786 | + | GGUACACGGCCAUGCACUCCUC | 22 | 9385 |
| HSV1-UL19-4787 | + | CGGUACACGGCCAUGCACUCCUC | 23 | 9386 |
| HSV1-UL19-4788 | + | CCGGUACACGGCCAUGCACUCCUC | 24 | 9387 |
| HSV1-UL19-4789 | + | GAACGCGGGGUGCAGCUC | 18 | 9388 |
| HSV1-UL19-4790 | + | CGAACGCGGGGUGCAGCUC | 19 | 9389 |
| HSV1-UL19-2689 | + | UCGAACGCGGGGUGCAGCUC | 20 | 2777 |
| HSV1-UL19-4791 | + | GUCGAACGCGGGGUGCAGCUC | 21 | 9390 |
| HSV1-UL19-4792 | + | AGUCGAACGCGGGGUGCAGCUC | 22 | 9391 |
| HSV1-UL19-4793 | + | AAGUCGAACGCGGGGUGCAGCUC | 23 | 9392 |
| HSV1-UL19-4794 | + | GAAGUCGAACGCGGGGUGCAGCUC | 24 | 9393 |
| HSV1-UL19-4795 | + | GUCGUCUGGGGGGCGCUC | 18 | 9394 |
| HSV1-UL19-4796 | + | CGUCGUCUGGGGGGCGCUC | 19 | 9395 |
| HSV1-UL19-2639 | + | GCGUCGUCUGGGGGGCGCUC | 20 | 2748 |
| HSV1-UL19-4797 | + | CGCGUCGUCUGGGGGGCGCUC | 21 | 9396 |
| HSV1-UL19-4798 | + | GCGCGUCGUCUGGGGGGCGCUC | 22 | 9397 |
| HSV1-UL19-4799 | + | CGCGCGUCGUCUGGGGGGCGCUC | 23 | 9398 |
| HSV1-UL19-4800 | + | ACGCGCGUCGUCUGGGGGGCGCUC | 24 | 9399 |
| HSV1-UL19-4801 | + | CUCGCCGGGGGCGAUCUC | 18 | 9400 |
| HSV1-UL19-4802 | + | CCUCGCCGGGGGCGAUCUC | 19 | 9401 |
| HSV1-UL19-1223 | + | UCCUCGCCGGGGGCGAUCUC | 20 | 1609 |
| HSV1-UL19-4803 | + | CUCCUCGCCGGGGGCGAUCUC | 21 | 9402 |
| HSV1-UL19-4804 | + | ACUCCUCGCCGGGGGCGAUCUC | 22 | 9403 |
| HSV1-UL19-4805 | + | CACUCCUCGCCGGGGGCGAUCUC | 23 | 9404 |
| HSV1-UL19-4806 | + | GCACUCCUCGCCGGGGGCGAUCUC | 24 | 9405 |
| HSV1-UL19-4807 | + | CUCCCCGGCCGCGCUCUC | 18 | 9406 |
| HSV1-UL19-4808 | + | UCUCCCCGGCCGCGCUCUC | 19 | 9407 |
| HSV1-UL19-2806 | + | UUCUCCCCGGCCGCGCUCUC | 20 | 2853 |
| HSV1-UL19-4809 | + | GUUCUCCCCGGCCGCGCUCUC | 21 | 9408 |
| HSV1-UL19-4810 | + | CGUUCUCCCCGGCCGCGCUCUC | 22 | 9409 |
| HSV1-UL19-4811 | + | GCGUUCUCCCCGGCCGCGCUCUC | 23 | 9410 |
| HSV1-UL19-4812 | + | CGCGUUCUCCCCGGCCGCGCUCUC | 24 | 9411 |
| HSV1-UL19-4813 | + | GGCGUAGAUGCGCUUCUC | 18 | 9412 |
| HSV1-UL19-4814 | + | CGGCGUAGAUGCGCUUCUC | 19 | 9413 |
| HSV1-UL19-2646 | + | GCGGCGUAGAUGCGCUUCUC | 20 | 2752 |
| HSV1-UL19-4815 | + | GGCGGCGUAGAUGCGCUUCUC | 21 | 9414 |
| HSV1-UL19-4816 | + | UGGCGGCGUAGAUGCGCUUCUC | 22 | 9415 |
| HSV1-UL19-4817 | + | GUGGCGGCGUAGAUGCGCUUCUC | 23 | 9416 |
| HSV1-UL19-4818 | + | GGUGGCGGCGUAGAUGCGCUUCUC | 24 | 9417 |
| HSV1-UL19-4819 | + | CCGGGAUGGAGGCCCGUC | 18 | 9418 |
| HSV1-UL19-4820 | + | CCCGGGAUGGAGGCCCGUC | 19 | 9419 |
| HSV1-UL19-2811 | + | ACCCGGGAUGGAGGCCCGUC | 20 | 2857 |
| HSV1-UL19-4821 | + | AACCCGGGAUGGAGGCCCGUC | 21 | 9420 |
| HSV1-UL19-4822 | + | AAACCCGGGAUGGAGGCCCGUC | 22 | 9421 |
| HSV1-UL19-4823 | + | CAAACCCGGGAUGGAGGCCCGUC | 23 | 9422 |
| HSV1-UL19-4824 | + | CCAAACCCGGGAUGGAGGCCCGUC | 24 | 9423 |
| HSV1-UL19-4825 | + | GGGUGCGCGGGGUCCGUC | 18 | 9424 |
| HSV1-UL19-4826 | + | GGGGUGCGCGGGGUCCGUC | 19 | 9425 |
| HSV1-UL19-2754 | + | GGGGGUGCGCGGGGUCCGUC | 20 | 2824 |
| HSV1-UL19-4827 | + | AGGGGGUGCGCGGGGUCCGUC | 21 | 9426 |
| HSV1-UL19-4828 | + | CAGGGGGUGCGCGGGGUCCGUC | 22 | 9427 |
| HSV1-UL19-4829 | + | GCAGGGGGUGCGCGGGGUCCGUC | 23 | 9428 |
| HSV1-UL19-4830 | + | UGCAGGGGGUGCGCGGGGUCCGUC | 24 | 9429 |
| HSV1-UL19-4831 | + | CUCCAGGCCCCGGGCGUC | 18 | 9430 |
| HSV1-UL19-4832 | + | GCUCCAGGCCCCGGGCGUC | 19 | 9431 |
| HSV1-UL19-2705 | + | AGCUCCAGGCCCCGGGCGUC | 20 | 2786 |
| HSV1-UL19-4833 | + | CAGCUCCAGGCCCCGGGCGUC | 21 | 9432 |
| HSV1-UL19-4834 | + | CCAGCUCCAGGCCCCGGGCGUC | 22 | 9433 |
| HSV1-UL19-4835 | + | CCCAGCUCCAGGCCCCGGGCGUC | 23 | 9434 |
| HSV1-UL19-4836 | + | CCCCAGCUCCAGGCCCCGGGCGUC | 24 | 9435 |
| HSV1-UL19-4837 | + | GCGCGCACGCGCGUCGUC | 18 | 9436 |
| HSV1-UL19-4838 | + | CGCGCGCACGCGCGUCGUC | 19 | 9437 |
| HSV1-UL19-1038 | + | CCGCGCGCACGCGCGUCGUC | 20 | 1424 |
| HSV1-UL19-4839 | + | UCCGCGCGCACGCGCGUCGUC | 21 | 9438 |
| HSV1-UL19-4840 | + | AUCCGCGCGCACGCGCGUCGUC | 22 | 9439 |
| HSV1-UL19-4841 | + | GAUCCGCGCGCACGCGCGUCGUC | 23 | 9440 |
| HSV1-UL19-4842 | + | AGAUCCGCGCGCACGCGCGUCGUC | 24 | 9441 |
| HSV1-UL19-4843 | + | GCAGUCGCGAUGGCGGUC | 18 | 9442 |
| HSV1-UL19-4844 | + | GGCAGUCGCGAUGGCGGUC | 19 | 9443 |
| HSV1-UL19-2734 | + | CGGCAGUCGCGAUGGCGGUC | 20 | 2810 |
| HSV1-UL19-4845 | + | CCGGCAGUCGCGAUGGCGGUC | 21 | 9444 |
| HSV1-UL19-4846 | + | CCCGGCAGUCGCGAUGGCGGUC | 22 | 9445 |
| HSV1-UL19-4847 | + | ACCCGGCAGUCGCGAUGGCGGUC | 23 | 9446 |
| HSV1-UL19-4848 | + | AACCCGGCAGUCGCGAUGGCGGUC | 24 | 9447 |
| HSV1-UL19-4849 | + | CACGGCUGCCGCGUUGUC | 18 | 9448 |
| HSV1-UL19-4850 | + | ACACGGCUGCCGCGUUGUC | 19 | 9449 |
| HSV1-UL19-2842 | + | UACACGGCUGCCGCGUUGUC | 20 | 2875 |
| HSV1-UL19-4851 | + | GUACACGGCUGCCGCGUUGUC | 21 | 9450 |
| HSV1-UL19-4852 | + | GGUACACGGCUGCCGCGUUGUC | 22 | 9451 |
| HSV1-UL19-4853 | + | AGGUACACGGCUGCCGCGUUGUC | 23 | 9452 |
| HSV1-UL19-4854 | + | CAGGUACACGGCUGCCGCGUUGUC | 24 | 9453 |
| HSV1-UL19-4855 | + | ACCAGCGAGUAGUCGUUC | 18 | 9454 |
| HSV1-UL19-4856 | + | GACCAGCGAGUAGUCGUUC | 19 | 9455 |
| HSV1-UL19-2718 | + | AGACCAGCGAGUAGUCGUUC | 20 | 2797 |
| HSV1-UL19-4857 | + | GAGACCAGCGAGUAGUCGUUC | 21 | 9456 |
| HSV1-UL19-4858 | + | CGAGACCAGCGAGUAGUCGUUC | 22 | 9457 |
| HSV1-UL19-4859 | + | ACGAGACCAGCGAGUAGUCGUUC | 23 | 9458 |
| HSV1-UL19-4860 | + | UACGAGACCAGCGAGUAGUCGUUC | 24 | 9459 |
| HSV1-UL19-4861 | + | GGUCCCGAUCGCGUGUUC | 18 | 9460 |
| HSV1-UL19-4862 | + | CGGUCCCGAUCGCGUGUUC | 19 | 9461 |
| HSV1-UL19-2674 | + | ACGGUCCCGAUCGCGUGUUC | 20 | 2766 |
| HSV1-UL19-4863 | + | CACGGUCCCGAUCGCGUGUUC | 21 | 9462 |
| HSV1-UL19-4864 | + | ACACGGUCCCGAUCGCGUGUUC | 22 | 9463 |
| HSV1-UL19-4865 | + | CACACGGUCCCGAUCGCGUGUUC | 23 | 9464 |
| HSV1-UL19-4866 | + | GCACACGGUCCCGAUCGCGUGUUC | 24 | 9465 |
| HSV1-UL19-4867 | + | UCGGUGUCCUCCACUUUC | 18 | 9466 |
| HSV1-UL19-4868 | + | UUCGGUGUCCUCCACUUUC | 19 | 9467 |
| HSV1-UL19-1021 | + | CUUCGGUGUCCUCCACUUUC | 20 | 1407 |
| HSV1-UL19-4869 | + | GCUUCGGUGUCCUCCACUUUC | 21 | 9468 |
| HSV1-UL19-4870 | + | GGCUUCGGUGUCCUCCACUUUC | 22 | 9469 |
| HSV1-UL19-4871 | + | CGGCUUCGGUGUCCUCCACUUUC | 23 | 9470 |
| HSV1-UL19-4872 | + | UCGGCUUCGGUGUCCUCCACUUUC | 24 | 9471 |
| HSV1-UL19-4873 | + | GACGUAAAGAACUUAAAG | 18 | 9472 |
| HSV1-UL19-4874 | + | CGACGUAAAGAACUUAAAG | 19 | 9473 |
| HSV1-UL19-2872 | + | CCGACGUAAAGAACUUAAAG | 20 | 2896 |
| HSV1-UL19-4875 | + | GCCGACGUAAAGAACUUAAAG | 21 | 9474 |
| HSV1-UL19-4876 | + | GGCCGACGUAAAGAACUUAAAG | 22 | 9475 |
| HSV1-UL19-4877 | + | CGGCCGACGUAAAGAACUUAAAG | 23 | 9476 |
| HSV1-UL19-4878 | + | UCGGCCGACGUAAAGAACUUAAAG | 24 | 9477 |
| HSV1-UL19-4879 | + | GCCACCCCCACAAAGAAG | 18 | 9478 |
| HSV1-UL19-4880 | + | GGCCACCCCCACAAAGAAG | 19 | 9479 |
| HSV1-UL19-2692 | + | CGGCCACCCCCACAAAGAAG | 20 | 2779 |
| HSV1-UL19-4881 | + | UCGGCCACCCCCACAAAGAAG | 21 | 9480 |
| HSV1-UL19-4882 | + | GUCGGCCACCCCCACAAAGAAG | 22 | 9481 |
| HSV1-UL19-4883 | + | CGUCGGCCACCCCCACAAAGAAG | 23 | 9482 |
| HSV1-UL19-4884 | + | ACGUCGGCCACCCCCACAAAGAAG | 24 | 9483 |
| HSV1-UL19-4885 | + | CAGCGGCCCCGCGAGAAG | 18 | 9484 |
| HSV1-UL19-4886 | + | GCAGCGGCCCCGCGAGAAG | 19 | 9485 |
| HSV1-UL19-2740 | + | AGCAGCGGCCCCGCGAGAAG | 20 | 2815 |
| HSV1-UL19-4887 | + | CAGCAGCGGCCCCGCGAGAAG | 21 | 9486 |
| HSV1-UL19-4888 | + | GCAGCAGCGGCCCCGCGAGAAG | 22 | 9487 |
| HSV1-UL19-4889 | + | UGCAGCAGCGGCCCCGCGAGAAG | 23 | 9488 |
| HSV1-UL19-4890 | + | GUGCAGCAGCGGCCCCGCGAGAAG | 24 | 9489 |
| HSV1-UL19-4891 | + | GGUAAAGUCAUCAACCAG | 18 | 9490 |
| HSV1-UL19-4892 | + | GGGUAAAGUCAUCAACCAG | 19 | 9491 |
| HSV1-UL19-2726 | + | AGGGUAAAGUCAUCAACCAG | 20 | 2803 |
| HSV1-UL19-4893 | + | CAGGGUAAAGUCAUCAACCAG | 21 | 9492 |
| HSV1-UL19-4894 | + | UCAGGGUAAAGUCAUCAACCAG | 22 | 9493 |
| HSV1-UL19-4895 | + | GUCAGGGUAAAGUCAUCAACCAG | 23 | 9494 |
| HSV1-UL19-4896 | + | GGUCAGGGUAAAGUCAUCAACCAG | 24 | 9495 |
| HSV1-UL19-4897 | + | CAGAGCCGGGGGGACCAG | 18 | 9496 |
| HSV1-UL19-4898 | + | CCAGAGCCGGGGGGACCAG | 19 | 9497 |
| HSV1-UL19-1291 | + | CCCAGAGCCGGGGGGACCAG | 20 | 1677 |
| HSV1-UL19-4899 | + | CCCCAGAGCCGGGGGGACCAG | 21 | 9498 |
| HSV1-UL19-4900 | + | CCCCCAGAGCCGGGGGGACCAG | 22 | 9499 |
| HSV1-UL19-4901 | + | GCCCCCAGAGCCGGGGGGACCAG | 23 | 9500 |
| HSV1-UL19-4902 | + | GGCCCCCAGAGCCGGGGGGACCAG | 24 | 9501 |
| HSV1-UL19-4903 | + | GGCUGCCGCGUUGUCCAG | 18 | 9502 |
| HSV1-UL19-4904 | + | CGGCUGCCGCGUUGUCCAG | 19 | 9503 |
| HSV1-UL19-2840 | + | ACGGCUGCCGCGUUGUCCAG | 20 | 2874 |
| HSV1-UL19-4905 | + | CACGGCUGCCGCGUUGUCCAG | 21 | 9504 |
| HSV1-UL19-4906 | + | ACACGGCUGCCGCGUUGUCCAG | 22 | 9505 |
| HSV1-UL19-4907 | + | UACACGGCUGCCGCGUUGUCCAG | 23 | 9506 |
| HSV1-UL19-4908 | + | GUACACGGCUGCCGCGUUGUCCAG | 24 | 9507 |
| HSV1-UL19-4909 | + | GCGGGGUCGCUGGCGCAG | 18 | 9508 |
| HSV1-UL19-4910 | + | GGCGGGGUCGCUGGCGCAG | 19 | 9509 |
| HSV1-UL19-2884 | + | GGGCGGGGUCGCUGGCGCAG | 20 | 2905 |
| HSV1-UL19-4911 | + | AGGGCGGGGUCGCUGGCGCAG | 21 | 9510 |
| HSV1-UL19-4912 | + | GAGGGCGGGGUCGCUGGCGCAG | 22 | 9511 |
| HSV1-UL19-4913 | + | GGAGGGCGGGGUCGCUGGCGCAG | 23 | 9512 |
| HSV1-UL19-4914 | + | AGGAGGGCGGGGUCGCUGGCGCAG | 24 | 9513 |
| HSV1-UL19-4915 | + | CGUGUCGGCAUGCGUCAG | 18 | 9514 |
| HSV1-UL19-4916 | + | GCGUGUCGGCAUGCGUCAG | 19 | 9515 |
| HSV1-UL19-2627 | + | CGCGUGUCGGCAUGCGUCAG | 20 | 2739 |
| HSV1-UL19-4917 | + | GCGCGUGUCGGCAUGCGUCAG | 21 | 9516 |
| HSV1-UL19-4918 | + | CGCGCGUGUCGGCAUGCGUCAG | 22 | 9517 |
| HSV1-UL19-4919 | + | CCGCGCGUGUCGGCAUGCGUCAG | 23 | 9518 |
| HSV1-UL19-4920 | + | CCCGCGCGUGUCGGCAUGCGUCAG | 24 | 9519 |
| HSV1-UL19-4921 | + | AGUAGUUGGCCCCCAGAG | 18 | 9520 |
| HSV1-UL19-4922 | + | AAGUAGUUGGCCCCCAGAG | 19 | 9521 |
| HSV1-UL19-2801 | + | AAAGUAGUUGGCCCCCAGAG | 20 | 2848 |
| HSV1-UL19-4923 | + | AAAAGUAGUUGGCCCCCAGAG | 21 | 9522 |
| HSV1-UL19-4924 | + | GAAAAGUAGUUGGCCCCCAGAG | 22 | 9523 |
| HSV1-UL19-4925 | + | CGAAAAGUAGUUGGCCCCCAGAG | 23 | 9524 |
| HSV1-UL19-4926 | + | ACGAAAAGUAGUUGGCCCCCAGAG | 24 | 9525 |
| HSV1-UL19-4927 | + | CCGCGUUGUCCAGGAGAG | 18 | 9526 |
| HSV1-UL19-4928 | + | GCCGCGUUGUCCAGGAGAG | 19 | 9527 |
| HSV1-UL19-1341 | + | UGCCGCGUUGUCCAGGAGAG | 20 | 1727 |
| HSV1-UL19-4929 | + | CUGCCGCGUUGUCCAGGAGAG | 21 | 9528 |
| HSV1-UL19-4930 | + | GCUGCCGCGUUGUCCAGGAGAG | 22 | 9529 |
| HSV1-UL19-4931 | + | GGCUGCCGCGUUGUCCAGGAGAG | 23 | 9530 |
| HSV1-UL19-4932 | + | CGGCUGCCGCGUUGUCCAGGAGAG | 24 | 9531 |
| HSV1-UL19-4933 | + | GAUGCACUGGAC CAC GAG | 18 | 9532 |
| HSV1-UL19-4934 | + | UGAUGCACUGGAC CAC GAG | 19 | 9533 |
| HSV1-UL19-2715 | + | GUGAUGCACUGGAC CAC GAG | 20 | 2794 |
| HSV1-UL19-4935 | + | GGUGAUGCACUGGAC CAC GAG | 21 | 9534 |
| HSV1-UL19-4936 | + | UGGUGAUGCACUGGAC CAC GAG | 22 | 9535 |
| HSV1-UL19-4937 | + | CUGGUGAUGCACUGGAC CAC GAG | 23 | 9536 |
| HSV1-UL19-4938 | + | GCUGGUGAUGCACUGGACCACGAG | 24 | 9537 |
| HSV1-UL19-4939 | + | GCGUCGGGCGCCGCCGAG | 18 | 9538 |
| HSV1-UL19-4940 | + | CGCGUCGGGCGCCGCCGAG | 19 | 9539 |
| HSV1-UL19-2765 | + | CCGCGUCGGGCGCCGCCGAG | 20 | 2832 |
| HSV1-UL19-4941 | + | CCCGCGUCGGGCGCCGCCGAG | 21 | 9540 |
| HSV1-UL19-4942 | + | GCCCGCGUCGGGCGCCGCCGAG | 22 | 9541 |
| HSV1-UL19-4943 | + | CGCCCGCGUCGGGCGCCGCCGAG | 23 | 9542 |
| HSV1-UL19-4944 | + | GCGCCCGCGUCGGGCGCCGCCGAG | 24 | 9543 |
| HSV1-UL19-4945 | + | UGCCGCGUUGUCCAGGAG | 18 | 9544 |
| HSV1-UL19-4946 | + | CUGCCGCGUUGUCCAGGAG | 19 | 9545 |
| HSV1-UL19-1343 | + | GCUGCCGCGUUGUCCAGGAG | 20 | 1729 |
| HSV1-UL19-4947 | + | GGCUGCCGCGUUGUCCAGGAG | 21 | 9546 |
| HSV1-UL19-4948 | + | CGGCUGCCGCGUUGUCCAGGAG | 22 | 9547 |
| HSV1-UL19-4949 | + | ACGGCUGCCGCGUUGUCCAGGAG | 23 | 9548 |
| HSV1-UL19-4950 | + | CACGGCUGCCGCGUUGUCCAGGAG | 24 | 9549 |
| HSV1-UL19-4951 | + | GUUGCGGGCACUGCGGAG | 18 | 9550 |
| HSV1-UL19-4952 | + | CGUUGCGGGCACUGCGGAG | 19 | 9551 |
| HSV1-UL19-2887 | + | CCGUUGCGGGCACUGCGGAG | 20 | 2907 |
| HSV1-UL19-4953 | + | CCCGUUGCGGGCACUGCGGAG | 21 | 9552 |
| HSV1-UL19-4954 | + | CCCCGUUGCGGGCACUGCGGAG | 22 | 9553 |
| HSV1-UL19-4955 | + | UCCCCGUUGCGGGCACUGCGGAG | 23 | 9554 |
| HSV1-UL19-4956 | + | UUCCCCGUUGCGGGCACUGCGGAG | 24 | 9555 |
| HSV1-UL19-4957 | + | CAGAGCCAGUCCCUUGAG | 18 | 9556 |
| HSV1-UL19-4958 | + | ACAGAGCCAGUCCCUUGAG | 19 | 9557 |
| HSV1-UL19-1441 | + | UACAGAGCCAGUCCCUUGAG | 20 | 1827 |
| HSV1-UL19-4959 | + | UUACAGAGCCAGUCCCUUGAG | 21 | 9558 |
| HSV1-UL19-4960 | + | AUUACAGAGCCAGUCCCUUGAG | 22 | 9559 |
| HSV1-UL19-4961 | + | UAUUACAGAGCCAGUCCCUUGAG | 23 | 9560 |
| HSV1-UL19-4962 | + | UUAUUACAGAGCCAGUCCCUUGAG | 24 | 9561 |
| HSV1-UL19-4963 | + | AGCGGGGAUGCGUCAUAG | 18 | 9562 |
| HSV1-UL19-4964 | + | GAGCGGGGAUGCGUCAUAG | 19 | 9563 |
| HSV1-UL19-2896 | + | UGAGCGGGGAUGCGUCAUAG | 20 | 2914 |
| HSV1-UL19-4965 | + | UUGAGCGGGGAUGCGUCAUAG | 21 | 9564 |
| HSV1-UL19-4966 | + | CUUGAGCGGGGAUGCGUCAUAG | 22 | 9565 |
| HSV1-UL19-4967 | + | CCUUGAGCGGGGAUGCGUCAUAG | 23 | 9566 |
| HSV1-UL19-4968 | + | CCCUUGAGCGGGGAUGCGUCAUAG | 24 | 9567 |
| HSV1-UL19-4969 | + | AGACGGGGCACGGCCACG | 18 | 9568 |
| HSV1-UL19-4970 | + | CAGACGGGGCACGGCCACG | 19 | 9569 |
| HSV1-UL19-2623 | + | UCAGACGGGGCACGGCCACG | 20 | 2737 |
| HSV1-UL19-4971 | + | GUCAGACGGGGCACGGCCACG | 21 | 9570 |
| HSV1-UL19-4972 | + | CGUCAGACGGGGCACGGCCACG | 22 | 9571 |
| HSV1-UL19-4973 | + | GCGUCAGACGGGGCACGGCCACG | 23 | 9572 |
| HSV1-UL19-4974 | + | UGCGUCAGACGGGGCACGGCCACG | 24 | 9573 |
| HSV1-UL19-4975 | + | UCGCGGACGUGCUGCACG | 18 | 9574 |
| HSV1-UL19-4976 | + | CUCGCGGACGUGCUGCACG | 19 | 9575 |
| HSV1-UL19-2805 | + | UCUCGCGGACGUGCUGCACG | 20 | 2852 |
| HSV1-UL19-4977 | + | CUCUCGCGGACGUGCUGCACG | 21 | 9576 |
| HSV1-UL19-4978 | + | GCUCUCGCGGACGUGCUGCACG | 22 | 9577 |
| HSV1-UL19-4979 | + | CGCUCUCGCGGACGUGCUGCACG | 23 | 9578 |
| HSV1-UL19-4980 | + | GCGCUCUCGCGGACGUGCUGCACG | 24 | 9579 |
| HSV1-UL19-4981 | + | CGGUCCUGUCGGACGACG | 18 | 9580 |
| HSV1-UL19-4982 | + | GCGGUCCUGUCGGACGACG | 19 | 9581 |
| HSV1-UL19-2816 | + | AGCGGUCCUGUCGGACGACG | 20 | 2860 |
| HSV1-UL19-4983 | + | AAGCGGUCCUGUCGGACGACG | 21 | 9582 |
| HSV1-UL19-4984 | + | AAAGCGGUCCUGUCGGACGACG | 22 | 9583 |
| HSV1-UL19-4985 | + | CAAAGCGGUCCUGUCGGACGACG | 23 | 9584 |
| HSV1-UL19-4986 | + | ACAAAGCGGUCCUGUCGGACGACG | 24 | 9585 |
| HSV1-UL19-4987 | + | GCGAACAGCGCGUGGACG | 18 | 9586 |
| HSV1-UL19-4988 | + | GGCGAACAGCGCGUGGACG | 19 | 9587 |
| HSV1-UL19-1273 | + | CGGCGAACAGCGCGUGGACG | 20 | 1659 |
| HSV1-UL19-4989 | + | CCGGCGAACAGCGCGUGGACG | 21 | 9588 |
| HSV1-UL19-4990 | + | CCCGGCGAACAGCGCGUGGACG | 22 | 9589 |
| HSV1-UL19-4991 | + | CCCCGGCGAACAGCGCGUGGACG | 23 | 9590 |
| HSV1-UL19-4992 | + | GCCCCGGCGAACAGCGCGUGGACG | 24 | 9591 |
| HSV1-UL19-4993 | + | CGGUGGCCGUGGACACCG | 18 | 9592 |
| HSV1-UL19-4994 | + | GCGGUGGCCGUGGACACCG | 19 | 9593 |
| HSV1-UL19-2874 | + | GGCGGUGGCCGUGGACACCG | 20 | 2898 |
| HSV1-UL19-4995 | + | CGGCGGUGGCCGUGGACACCG | 21 | 9594 |
| HSV1-UL19-4996 | + | GCGGCGGUGGCCGUGGACACCG | 22 | 9595 |
| HSV1-UL19-4997 | + | GGCGGCGGUGGCCGUGGACACCG | 23 | 9596 |
| HSV1-UL19-4998 | + | UGGCGGCGGUGGCCGUGGACACCG | 24 | 9597 |
| HSV1-UL19-4999 | + | AGCAGGGGCUGAGCACCG | 18 | 9598 |
| HSV1-UL19-5000 | + | AAGCAGGGGCUGAGCACCG | 19 | 9599 |
| HSV1-UL19-2869 | + | AAAGCAGGGGCUGAGCACCG | 20 | 2894 |
| HSV1-UL19-5001 | + | UAAAGCAGGGGCUGAGCACCG | 21 | 9600 |
| HSV1-UL19-5002 | + | UUAAAGCAGGGGCUGAGCACCG | 22 | 9601 |
| HSV1-UL19-5003 | + | CUUAAAGCAGGGGCUGAGCACCG | 23 | 9602 |
| HSV1-UL19-5004 | + | ACUUAAAGCAGGGGCUGAGCACCG | 24 | 9603 |
| HSV1-UL19-5005 | + | CGAGUUCGCGGCACCCCG | 18 | 9604 |
| HSV1-UL19-5006 | + | ACGAGUUCGCGGCACCCCG | 19 | 9605 |
| HSV1-UL19-1414 | + | CACGAGUUCGCGGCACCCCG | 20 | 1800 |
| HSV1-UL19-5007 | + | CCACGAGUUCGCGGCACCCCG | 21 | 9606 |
| HSV1-UL19-5008 | + | UCCACGAGUUCGCGGCACCCCG | 22 | 9607 |
| HSV1-UL19-5009 | + | CUCCACGAGUUCGCGGCACCCCG | 23 | 9608 |
| HSV1-UL19-5010 | + | CCUCCACGAGUUCGCGGCACCCCG | 24 | 9609 |
| HSV1-UL19-5011 | + | AGCACCGGCGAGGCCCCG | 18 | 9610 |
| HSV1-UL19-5012 | + | GAGCACCGGCGAGGCCCCG | 19 | 9611 |
| HSV1-UL19-2868 | + | UGAGCACCGGCGAGGCCCCG | 20 | 2893 |
| HSV1-UL19-5013 | + | CUGAGCACCGGCGAGGCCCCG | 21 | 9612 |
| HSV1-UL19-5014 | + | GCUGAGCACCGGCGAGGCCCCG | 22 | 9613 |
| HSV1-UL19-5015 | + | GGCUGAGCACCGGCGAGGCCCCG | 23 | 9614 |
| HSV1-UL19-5016 | + | GGGCUGAGCACCGGCGAGGCCCCG | 24 | 9615 |
| HSV1-UL19-5017 | + | GCCACGUGGUCGGCCCCG | 18 | 9616 |
| HSV1-UL19-5018 | + | CGCCACGUGGUCGGCCCCG | 19 | 9617 |
| HSV1-UL19-2784 | + | UCGCCACGUGGUCGGCCCCG | 20 | 2843 |
| HSV1-UL19-5019 | + | UUCGCCACGUGGUCGGCCCCG | 21 | 9618 |
| HSV1-UL19-5020 | + | GUUCGCCACGUGGUCGGCCCCG | 22 | 9619 |
| HSV1-UL19-5021 | + | CGUUCGCCACGUGGUCGGCCCCG | 23 | 9620 |
| HSV1-UL19-5022 | + | GCGUUCGCCACGUGGUCGGCCCCG | 24 | 9621 |
| HSV1-UL19-5023 | + | AGUUGGCCCCCAGAGCCG | 18 | 9622 |
| HSV1-UL19-5024 | + | UAGUUGGCCCCCAGAGCCG | 19 | 9623 |
| HSV1-UL19-1296 | + | GUAGUUGGCCCCCAGAGCCG | 20 | 1682 |
| HSV1-UL19-5025 | + | AGUAGUUGGCCCCCAGAGCCG | 21 | 9624 |
| HSV1-UL19-5026 | + | AAGUAGUUGGCCCCCAGAGCCG | 22 | 9625 |
| HSV1-UL19-5027 | + | AAAGUAGUUGGCCCCCAGAGCCG | 23 | 9626 |
| HSV1-UL19-5028 | + | AAAAGUAGUUGGCCCCCAGAGCCG | 24 | 9627 |
| HSV1-UL19-5029 | + | GGGCGUCCCGGAACGCCG | 18 | 9628 |
| HSV1-UL19-5030 | + | CGGGCGUCCCGGAACGCCG | 19 | 9629 |
| HSV1-UL19-2703 | + | CCGGGCGUCCCGGAACGCCG | 20 | 2785 |
| HSV1-UL19-5031 | + | CCCGGGCGUCCCGGAACGCCG | 21 | 9630 |
| HSV1-UL19-5032 | + | CCCCGGGCGUCCCGGAACGCCG | 22 | 9631 |
| HSV1-UL19-5033 | + | GCCCCGGGCGUCCCGGAACGCCG | 23 | 9632 |
| HSV1-UL19-5034 | + | GGCCCCGGGCGUCCCGGAACGCCG | 24 | 9633 |
| HSV1-UL19-5035 | + | CGCCCGCGUCGGGCGCCG | 18 | 9634 |
| HSV1-UL19-5036 | + | GCGCCCGCGUCGGGCGCCG | 19 | 9635 |
| HSV1-UL19-2766 | + | GGCGCCCGCGUCGGGCGCCG | 20 | 2833 |
| HSV1-UL19-5037 | + | UGGCGCCCGCGUCGGGCGCCG | 21 | 9636 |
| HSV1-UL19-5038 | + | UUGGCGCCCGCGUCGGGCGCCG | 22 | 9637 |
| HSV1-UL19-5039 | + | GUUGGCGCCCGCGUCGGGCGCCG | 23 | 9638 |
| HSV1-UL19-5040 | + | UGUUGGCGCCCGCGUCGGGCGCCG | 24 | 9639 |
| HSV1-UL19-5041 | + | AAGACCAGCUUCUCGCCG | 18 | 9640 |
| HSV1-UL19-5042 | + | AAAGACCAGCUUCUCGCCG | 19 | 9641 |
| HSV1-UL19-2644 | + | GAAAGACCAGCUUCUCGCCG | 20 | 2750 |
| HSV1-UL19-5043 | + | AGAAAGACCAGCUUCUCGCCG | 21 | 9642 |
| HSV1-UL19-5044 | + | CAGAAAGACCAGCUUCUCGCCG | 22 | 9643 |
| HSV1-UL19-5045 | + | CCAGAAAGACCAGCUUCUCGCCG | 23 | 9644 |
| HSV1-UL19-5046 | + | UCCAGAAAGACCAGCUUCUCGCCG | 24 | 9645 |
| HSV1-UL19-5047 | + | UCUCCCCCGGGCCGGCCG | 18 | 9646 |
| HSV1-UL19-5048 | + | AUCUCCCCCGGGCCGGCCG | 19 | 9647 |
| HSV1-UL19-1124 | + | GAUCUCCCCCGGGCCGGCCG | 20 | 1510 |
| HSV1-UL19-5049 | + | GGAUCUCCCCCGGGCCGGCCG | 21 | 9648 |
| HSV1-UL19-5050 | + | UGGAUCUCCCCCGGGCCGGCCG | 22 | 9649 |
| HSV1-UL19-5051 | + | CUGGAUCUCCCCCGGGCCGGCCG | 23 | 9650 |
| HSV1-UL19-5052 | + | CCUGGAUCUCCCCCGGGCCGGCCG | 24 | 9651 |
| HSV1-UL19-5053 | + | ACAGGUCGCGCAGGGCCG | 18 | 9652 |
| HSV1-UL19-5054 | + | GACAGGUCGCGCAGGGCCG | 19 | 9653 |
| HSV1-UL19-1284 | + | CGACAGGUCGCGCAGGGCCG | 20 | 1670 |
| HSV1-UL19-5055 | + | GCGACAGGUCGCGCAGGGCCG | 21 | 9654 |
| HSV1-UL19-5056 | + | CGCGACAGGUCGCGCAGGGCCG | 22 | 9655 |
| HSV1-UL19-5057 | + | CCGCGACAGGUCGCGCAGGGCCG | 23 | 9656 |
| HSV1-UL19-5058 | + | GCCGCGACAGGUCGCGCAGGGCCG | 24 | 9657 |
| HSV1-UL19-5059 | + | CCGGGGGUUGCAGGGCCG | 18 | 9658 |
| HSV1-UL19-5060 | + | CCCGGGGGUUGCAGGGCCG | 19 | 9659 |
| HSV1-UL19-2854 | + | CCCCGGGGGUUGCAGGGCCG | 20 | 2882 |
| HSV1-UL19-5061 | + | UCCCCGGGGGUUGCAGGGCCG | 21 | 9660 |
| HSV1-UL19-5062 | + | GUCCCCGGGGGUUGCAGGGCCG | 22 | 9661 |
| HSV1-UL19-5063 | + | CGUCCCCGGGGGUUGCAGGGCCG | 23 | 9662 |
| HSV1-UL19-5064 | + | GCGUCCCCGGGGGUUGCAGGGCCG | 24 | 9663 |
| HSV1-UL19-5065 | + | GCGGUGUUGGCGCCCGCG | 18 | 9664 |
| HSV1-UL19-5066 | + | CGCGGUGUUGGCGCCCGCG | 19 | 9665 |
| HSV1-UL19-2767 | + | ACGCGGUGUUGGCGCCCGCG | 20 | 2834 |
| HSV1-UL19-5067 | + | GACGCGGUGUUGGCGCCCGCG | 21 | 9666 |
| HSV1-UL19-5068 | + | GGACGCGGUGUUGGCGCCCGCG | 22 | 9667 |
| HSV1-UL19-5069 | + | UGGACGCGGUGUUGGCGCCCGCG | 23 | 9668 |
| HSV1-UL19-5070 | + | GUGGACGCGGUGUUGGCGCCCGCG | 24 | 9669 |
| HSV1-UL19-5071 | + | CUGCCGCGACAGGUCGCG | 18 | 9670 |
| HSV1-UL19-5072 | + | CCUGCCGCGACAGGUCGCG | 19 | 9671 |
| HSV1-UL19-2792 | + | ACCUGCCGCGACAGGUCGCG | 20 | 2846 |
| HSV1-UL19-5073 | + | GACCUGCCGCGACAGGUCGCG | 21 | 9672 |
| HSV1-UL19-5074 | + | GGACCUGCCGCGACAGGUCGCG | 22 | 9673 |
| HSV1-UL19-5075 | + | GGGACCUGCCGCGACAGGUCGCG | 23 | 9674 |
| HSV1-UL19-5076 | + | GGGGACCUGCCGCGACAGGUCGCG | 24 | 9675 |
| HSV1-UL19-5077 | + | UGGCGGGGCGGGAAGGCG | 18 | 9676 |
| HSV1-UL19-5078 | + | CUGGCGGGGCGGGAAGGCG | 19 | 9677 |
| HSV1-UL19-1074 | + | GCUGGCGGGGCGGGAAGGCG | 20 | 1460 |
| HSV1-UL19-5079 | + | AGCUGGCGGGGCGGGAAGGCG | 21 | 9678 |
| HSV1-UL19-5080 | + | CAGCUGGCGGGGCGGGAAGGCG | 22 | 9679 |
| HSV1-UL19-5081 | + | ACAGCUGGCGGGGCGGGAAGGCG | 23 | 9680 |
| HSV1-UL19-5082 | + | AACAGCUGGCGGGGCGGGAAGGCG | 24 | 9681 |
| HSV1-UL19-5083 | + | ACGAGCCGGGCCAGGGCG | 18 | 9682 |
| HSV1-UL19-5084 | + | CACGAGCCGGGCCAGGGCG | 19 | 9683 |
| HSV1-UL19-2713 | + | CCACGAGCCGGGCCAGGGCG | 20 | 2792 |
| HSV1-UL19-5085 | + | ACCACGAGCCGGGCCAGGGCG | 21 | 9684 |
| HSV1-UL19-5086 | + | GACCACGAGCCGGGCCAGGGCG | 22 | 9685 |
| HSV1-UL19-5087 | + | GGACCACGAGCCGGGCCAGGGCG | 23 | 9686 |
| HSV1-UL19-5088 | + | UGGACCACGAGCCGGGCCAGGGCG | 24 | 9687 |
| HSV1-UL19-5089 | + | CCCGUUGCGGGCACUGCG | 18 | 9688 |
| HSV1-UL19-5090 | + | CCCCGUUGCGGGCACUGCG | 19 | 9689 |
| HSV1-UL19-2889 | + | UCCCCGUUGCGGGCACUGCG | 20 | 2908 |
| HSV1-UL19-5091 | + | UUCCCCGUUGCGGGCACUGCG | 21 | 9690 |
| HSV1-UL19-5092 | + | CUUCCCCGUUGCGGGCACUGCG | 22 | 9691 |
| HSV1-UL19-5093 | + | GCUUCCCCGUUGCGGGCACUGCG | 23 | 9692 |
| HSV1-UL19-5094 | + | GGCUUCCCCGUUGCGGGCACUGCG | 24 | 9693 |
| HSV1-UL19-5095 | + | AGCUGCUGCAUGUCUGCG | 18 | 9694 |
| HSV1-UL19-5096 | + | CAGCUGCUGCAUGUCUGCG | 19 | 9695 |
| HSV1-UL19-2683 | + | ACAGCUGCUGCAUGUCUGCG | 20 | 2772 |
| HSV1-UL19-5097 | + | AACAGCUGCUGCAUGUCUGCG | 21 | 9696 |
| HSV1-UL19-5098 | + | AAACAGCUGCUGCAUGUCUGCG | 22 | 9697 |
| HSV1-UL19-5099 | + | AAAACAGCUGCUGCAUGUCUGCG | 23 | 9698 |
| HSV1-UL19-5100 | + | AAAAACAGCUGCUGCAUGUCUGCG | 24 | 9699 |
| HSV1-UL19-5101 | + | CGACGGGCUGUCGGAUCG | 18 | 9700 |
| HSV1-UL19-5102 | + | ACGACGGGCUGUCGGAUCG | 19 | 9701 |
| HSV1-UL19-2803 | + | CACGACGGGCUGUCGGAUCG | 20 | 2850 |
| HSV1-UL19-5103 | + | GCACGACGGGCUGUCGGAUCG | 21 | 9702 |
| HSV1-UL19-5104 | + | UGCACGACGGGCUGUCGGAUCG | 22 | 9703 |
| HSV1-UL19-5105 | + | CUGCACGACGGGCUGUCGGAUCG | 23 | 9704 |
| HSV1-UL19-5106 | + | GCUGCACGACGGGCUGUCGGAUCG | 24 | 9705 |
| HSV1-UL19-5107 | + | UCGCUGGGGCACUCCUCG | 18 | 9706 |
| HSV1-UL19-5108 | + | GUCGCUGGGGCACUCCUCG | 19 | 9707 |
| HSV1-UL19-2750 | + | GGUCGCUGGGGCACUCCUCG | 20 | 2822 |
| HSV1-UL19-5109 | + | GGGUCGCUGGGGCACUCCUCG | 21 | 9708 |
| HSV1-UL19-5110 | + | GGGGUCGCUGGGGCACUCCUCG | 22 | 9709 |
| HSV1-UL19-5111 | + | CGGGGUCGCUGGGGCACUCCUCG | 23 | 9710 |
| HSV1-UL19-5112 | + | ACGGGGUCGCUGGGGCACUCCUCG | 24 | 9711 |
| HSV1-UL19-5113 | + | AGGAAAAAGCUCGUCUCG | 18 | 9712 |
| HSV1-UL19-5114 | + | CAGGAAAAAGCUCGUCUCG | 19 | 9713 |
| HSV1-UL19-2618 | + | CCAGGAAAAAGCUCGUCUCG | 20 | 2733 |
| HSV1-UL19-5115 | + | CCCAGGAAAAAGCUCGUCUCG | 21 | 9714 |
| HSV1-UL19-5116 | + | GCCCAGGAAAAAGCUCGUCUCG | 22 | 9715 |
| HSV1-UL19-5117 | + | UGCCCAGGAAAAAGCUCGUCUCG | 23 | 9716 |
| HSV1-UL19-5118 | + | UUGCCCAGGAAAAAGCUCGUCUCG | 24 | 9717 |
| HSV1-UL19-5119 | + | GCCGGGUAGUUGCGGUCG | 18 | 9718 |
| HSV1-UL19-5120 | + | CGCCGGGUAGUUGCGGUCG | 19 | 9719 |
| HSV1-UL19-2711 | + | CCGCCGGGUAGUUGCGGUCG | 20 | 2790 |
| HSV1-UL19-5121 | + | ACCGCCGGGUAGUUGCGGUCG | 21 | 9720 |
| HSV1-UL19-5122 | + | CACCGCCGGGUAGUUGCGGUCG | 22 | 9721 |
| HSV1-UL19-5123 | + | ACACCGCCGGGUAGUUGCGGUCG | 23 | 9722 |
| HSV1-UL19-5124 | + | AACACCGCCGGGUAGUUGCGGUCG | 24 | 9723 |
| HSV1-UL19-5125 | + | GGGUCCGUCACGGGGUCG | 18 | 9724 |
| HSV1-UL19-5126 | + | GGGGUCCGUCACGGGGUCG | 19 | 9725 |
| HSV1-UL19-2752 | + | CGGGGUCCGUCACGGGGUCG | 20 | 2823 |
| HSV1-UL19-5127 | + | GCGGGGUCCGUCACGGGGUCG | 21 | 9726 |
| HSV1-UL19-5128 | + | CGCGGGGUCCGUCACGGGGUCG | 22 | 9727 |
| HSV1-UL19-5129 | + | GCGCGGGGUCCGUCACGGGGUCG | 23 | 9728 |
| HSV1-UL19-5130 | + | UGCGCGGGGUCCGUCACGGGGUCG | 24 | 9729 |
| HSV1-UL19-5131 | + | UACGGAUUGGCGGCUUCG | 18 | 9730 |
| HSV1-UL19-5132 | + | GUACGGAUUGGCGGCUUCG | 19 | 9731 |
| HSV1-UL19-2678 | + | CGUACGGAUUGGCGGCUUCG | 20 | 2769 |
| HSV1-UL19-5133 | + | CCGUACGGAUUGGCGGCUUCG | 21 | 9732 |
| HSV1-UL19-5134 | + | CCCGUACGGAUUGGCGGCUUCG | 22 | 9733 |
| HSV1-UL19-5135 | + | CCCCGUACGGAUUGGCGGCUUCG | 23 | 9734 |
| HSV1-UL19-5136 | + | GCCCCGUACGGAUUGGCGGCUUCG | 24 | 9735 |
| HSV1-UL19-5137 | + | AACAGGCCCAGGGGCAGG | 18 | 9736 |
| HSV1-UL19-5138 | + | GAACAGGCCCAGGGGCAGG | 19 | 9737 |
| HSV1-UL19-2653 | + | UGAACAGGCCCAGGGGCAGG | 20 | 2755 |
| HSV1-UL19-5139 | + | UUGAACAGGCCCAGGGGCAGG | 21 | 9738 |
| HSV1-UL19-5140 | + | AUUGAACAGGCCCAGGGGCAGG | 22 | 9739 |
| HSV1-UL19-5141 | + | GAUUGAACAGGCCCAGGGGCAGG | 23 | 9740 |
| HSV1-UL19-5142 | + | GGAUUGAACAGGCCCAGGGGCAGG | 24 | 9741 |
| HSV1-UL19-5143 | + | AGCGUCUGCCGGUUCAGG | 18 | 9742 |
| HSV1-UL19-5144 | + | CAGCGUCUGCCGGUUCAGG | 19 | 9743 |
| HSV1-UL19-2638 | + | CCAGCGUCUGCCGGUUCAGG | 20 | 2747 |
| HSV1-UL19-5145 | + | UCCAGCGUCUGCCGGUUCAGG | 21 | 9744 |
| HSV1-UL19-5146 | + | GUCCAGCGUCUGCCGGUUCAGG | 22 | 9745 |
| HSV1-UL19-5147 | + | CGUCCAGCGUCUGCCGGUUCAGG | 23 | 9746 |
| HSV1-UL19-5148 | + | UCGUCCAGCGUCUGCCGGUUCAGG | 24 | 9747 |
| HSV1-UL19-5149 | + | GUGUCCUCCACUUUCAGG | 18 | 9748 |
| HSV1-UL19-5150 | + | GGUGUCCUCCACUUUCAGG | 19 | 9749 |
| HSV1-UL19-2633 | + | CGGUGUCCUCCACUUUCAGG | 20 | 2743 |
| HSV1-UL19-5151 | + | UCGGUGUCCUCCACUUUCAGG | 21 | 9750 |
| HSV1-UL19-5152 | + | UUCGGUGUCCUCCACUUUCAGG | 22 | 9751 |
| HSV1-UL19-5153 | + | CUUCGGUGUCCUCCACUUUCAGG | 23 | 9752 |
| HSV1-UL19-5154 | + | GCUUCGGUGUCCUCCACUUUCAGG | 24 | 9753 |
| HSV1-UL19-5155 | + | CGCGUUGUCCAGGAGAGG | 18 | 9754 |
| HSV1-UL19-5156 | + | CCGCGUUGUCCAGGAGAGG | 19 | 9755 |
| HSV1-UL19-1340 | + | GCCGCGUUGUCCAGGAGAGG | 20 | 1726 |
| HSV1-UL19-5157 | + | UGCCGCGUUGUCCAGGAGAGG | 21 | 9756 |
| HSV1-UL19-5158 | + | CUGCCGCGUUGUCCAGGAGAGG | 22 | 9757 |
| HSV1-UL19-5159 | + | GCUGCCGCGUUGUCCAGGAGAGG | 23 | 9758 |
| HSV1-UL19-5160 | + | GGCUGCCGCGUUGUCCAGGAGAGG | 24 | 9759 |
| HSV1-UL19-5161 | + | CGGGCACUGCGGAGGAGG | 18 | 9760 |
| HSV1-UL19-5162 | + | GCGGGCACUGCGGAGGAGG | 19 | 9761 |
| HSV1-UL19-2886 | + | UGCGGGCACUGCGGAGGAGG | 20 | 2906 |
| HSV1-UL19-5163 | + | UUGCGGGCACUGCGGAGGAGG | 21 | 9762 |
| HSV1-UL19-5164 | + | GUUGCGGGCACUGCGGAGGAGG | 22 | 9763 |
| HSV1-UL19-5165 | + | CGUUGCGGGCACUGCGGAGGAGG | 23 | 9764 |
| HSV1-UL19-5166 | + | CCGUUGCGGGCACUGCGGAGGAGG | 24 | 9765 |
| HSV1-UL19-5167 | + | GUUGGCCCCCAGAGCCGG | 18 | 9766 |
| HSV1-UL19-5168 | + | AGUUGGCCCCCAGAGCCGG | 19 | 9767 |
| HSV1-UL19-1295 | + | UAGUUGGCCCCCAGAGCCGG | 20 | 1681 |
| HSV1-UL19-5169 | + | GUAGUUGGCCCCCAGAGCCGG | 21 | 9768 |
| HSV1-UL19-5170 | + | AGUAGUUGGCCCCCAGAGCCGG | 22 | 9769 |
| HSV1-UL19-5171 | + | AAGUAGUUGGCCCCCAGAGCCGG | 23 | 9770 |
| HSV1-UL19-5172 | + | AAAGUAGUUGGCCCCCAGAGCCGG | 24 | 9771 |
| HSV1-UL19-5173 | + | CUCCCCCGGGCCGGCCGG | 18 | 9772 |
| HSV1-UL19-5174 | + | UCUCCCCCGGGCCGGCCGG | 19 | 9773 |
| HSV1-UL19-1123 | + | AUCUCCCCCGGGCCGGCCGG | 20 | 1509 |
| HSV1-UL19-5175 | + | GAUCUCCCCCGGGCCGGCCGG | 21 | 9774 |
| HSV1-UL19-5176 | + | GGAUCUCCCCCGGGCCGGCCGG | 22 | 9775 |
| HSV1-UL19-5177 | + | UGGAUCUCCCCCGGGCCGGCCGG | 23 | 9776 |
| HSV1-UL19-5178 | + | CUGGAUCUCCCCCGGGCCGGCCGG | 24 | 9777 |
| HSV1-UL19-5179 | + | CAGGUCGCGCAGGGCCGG | 18 | 9778 |
| HSV1-UL19-5180 | + | ACAGGUCGCGCAGGGCCGG | 19 | 9779 |
| HSV1-UL19-1283 | + | GACAGGUCGCGCAGGGCCGG | 20 | 1669 |
| HSV1-UL19-5181 | + | CGACAGGUCGCGCAGGGCCGG | 21 | 9780 |
| HSV1-UL19-5182 | + | GCGACAGGUCGCGCAGGGCCGG | 22 | 9781 |
| HSV1-UL19-5183 | + | CGCGACAGGUCGCGCAGGGCCGG | 23 | 9782 |
| HSV1-UL19-5184 | + | CCGCGACAGGUCGCGCAGGGCCGG | 24 | 9783 |
| HSV1-UL19-5185 | + | CGGGGGUUGCAGGGCCGG | 18 | 9784 |
| HSV1-UL19-5186 | + | CCGGGGGUUGCAGGGCCGG | 19 | 9785 |
| HSV1-UL19-1369 | + | CCCGGGGGUUGCAGGGCCGG | 20 | 1755 |
| HSV1-UL19-5187 | + | CCCCGGGGGUUGCAGGGCCGG | 21 | 9786 |
| HSV1-UL19-5188 | + | UCCCCGGGGGUUGCAGGGCCGG | 22 | 9787 |
| HSV1-UL19-5189 | + | GUCCCCGGGGGUUGCAGGGCCGG | 23 | 9788 |
| HSV1-UL19-5190 | + | CGUCCCCGGGGGUUGCAGGGCCGG | 24 | 9789 |
| HSV1-UL19-5191 | + | GGAUCUCCCCCGGGCCGG | 18 | 9790 |
| HSV1-UL19-5192 | + | UGGAUCUCCCCCGGGCCGG | 19 | 9791 |
| HSV1-UL19-2698 | + | CUGGAUCUCCCCCGGGCCGG | 20 | 2781 |
| HSV1-UL19-5193 | + | CCUGGAUCUCCCCCGGGCCGG | 21 | 9792 |
| HSV1-UL19-5194 | + | GCCUGGAUCUCCCCCGGGCCGG | 22 | 9793 |
| HSV1-UL19-5195 | + | GGCCUGGAUCUCCCCCGGGCCGG | 23 | 9794 |
| HSV1-UL19-5196 | + | UGGCCUGGAUCUCCCCCGGGCCGG | 24 | 9795 |
| HSV1-UL19-5197 | + | UGUCUGCGGCGGGGCCGG | 18 | 9796 |
| HSV1-UL19-5198 | + | AUGUCUGCGGCGGGGCCGG | 19 | 9797 |
| HSV1-UL19-2681 | + | CAUGUCUGCGGCGGGGCCGG | 20 | 2771 |
| HSV1-UL19-5199 | + | GCAUGUCUGCGGCGGGGCCGG | 21 | 9798 |
| HSV1-UL19-5200 | + | UGCAUGUCUGCGGCGGGGCCGG | 22 | 9799 |
| HSV1-UL19-5201 | + | CUGCAUGUCUGCGGCGGGGCCGG | 23 | 9800 |
| HSV1-UL19-5202 | + | GCUGCAUGUCUGCGGCGGGGCCGG | 24 | 9801 |
| HSV1-UL19-5203 | + | AGGGUGGCGUAUACGCGG | 18 | 9802 |
| HSV1-UL19-5204 | + | CAGGGUGGCGUAUACGCGG | 19 | 9803 |
| HSV1-UL19-2744 | + | GCAGGGUGGCGUAUACGCGG | 20 | 2819 |
| HSV1-UL19-5205 | + | UGCAGGGUGGCGUAUACGCGG | 21 | 9804 |
| HSV1-UL19-5206 | + | CUGCAGGGUGGCGUAUACGCGG | 22 | 9805 |
| HSV1-UL19-5207 | + | UCUGCAGGGUGGCGUAUACGCGG | 23 | 9806 |
| HSV1-UL19-5208 | + | UUCUGCAGGGUGGCGUAUACGCGG | 24 | 9807 |
| HSV1-UL19-5209 | + | CCGUUGCGGGCACUGCGG | 18 | 9808 |
| HSV1-UL19-5210 | + | CCCGUUGCGGGCACUGCGG | 19 | 9809 |
| HSV1-UL19-1431 | + | CCCCGUUGCGGGCACUGCGG | 20 | 1817 |
| HSV1-UL19-5211 | + | UCCCCGUUGCGGGCACUGCGG | 21 | 9810 |
| HSV1-UL19-5212 | + | UUCCCCGUUGCGGGCACUGCGG | 22 | 9811 |
| HSV1-UL19-5213 | + | CUUCCCCGUUGCGGGCACUGCGG | 23 | 9812 |
| HSV1-UL19-5214 | + | GCUUCCCCGUUGCGGGCACUGCGG | 24 | 9813 |
| HSV1-UL19-5215 | + | GCUGCUGCAUGUCUGCGG | 18 | 9814 |
| HSV1-UL19-5216 | + | AGCUGCUGCAUGUCUGCGG | 19 | 9815 |
| HSV1-UL19-1103 | + | CAGCUGCUGCAUGUCUGCGG | 20 | 1489 |
| HSV1-UL19-5217 | + | ACAGCUGCUGCAUGUCUGCGG | 21 | 9816 |
| HSV1-UL19-5218 | + | AACAGCUGCUGCAUGUCUGCGG | 22 | 9817 |
| HSV1-UL19-5219 | + | AAACAGCUGCUGCAUGUCUGCGG | 23 | 9818 |
| HSV1-UL19-5220 | + | AAAACAGCUGCUGCAUGUCUGCGG | 24 | 9819 |
| HSV1-UL19-5221 | + | AGCUGGCCGUCGUUGCGG | 18 | 9820 |
| HSV1-UL19-5222 | + | CAGCUGGCCGUCGUUGCGG | 19 | 9821 |
| HSV1-UL19-2737 | + | GCAGCUGGCCGUCGUUGCGG | 20 | 2812 |
| HSV1-UL19-5223 | + | AGCAGCUGGCCGUCGUUGCGG | 21 | 9822 |
| HSV1-UL19-5224 | + | CAGCAGCUGGCCGUCGUUGCGG | 22 | 9823 |
| HSV1-UL19-5225 | + | GCAGCAGCUGGCCGUCGUUGCGG | 23 | 9824 |
| HSV1-UL19-5226 | + | UGCAGCAGCUGGCCGUCGUUGCGG | 24 | 9825 |
| HSV1-UL19-5227 | + | GC GACAGGUC GCGCAGGG | 18 | 9826 |
| HSV1-UL19-5228 | + | CGCGACAGGUCGCGCAGGG | 19 | 9827 |
| HSV1-UL19-2791 | + | CCGCGACAGGUCGCGCAGGG | 20 | 2845 |
| HSV1-UL19-5229 | + | GCCGCGACAGGUCGCGCAGGG | 21 | 9828 |
| HSV1-UL19-5230 | + | UGCCGCGACAGGUCGCGCAGGG | 22 | 9829 |
| HSV1-UL19-5231 | + | CUGCCGCGACAGGUCGCGCAGGG | 23 | 9830 |
| HSV1-UL19-5232 | + | CCUGCCGCGACAGGUCGCGCAGGG | 24 | 9831 |
| HSV1-UL19-5233 | + | CCCGAACACGGGGACGGG | 18 | 9832 |
| HSV1-UL19-5234 | + | ACCCGAACACGGGGACGGG | 19 | 9833 |
| HSV1-UL19-2843 | + | CACCCGAACACGGGGACGGG | 20 | 2876 |
| HSV1-UL19-5235 | + | GCACCCGAACACGGGGACGGG | 21 | 9834 |
| HSV1-UL19-5236 | + | CGCACCCGAACACGGGGACGGG | 22 | 9835 |
| HSV1-UL19-5237 | + | GCGCACCCGAACACGGGGACGGG | 23 | 9836 |
| HSV1-UL19-5238 | + | GGCGCACCCGAACACGGGGACGGG | 24 | 9837 |
| HSV1-UL19-5239 | + | GAACAGCGCGUGGACGGG | 18 | 9838 |
| HSV1-UL19-5240 | + | CGAACAGCGCGUGGACGGG | 19 | 9839 |
| HSV1-UL19-2778 | + | GCGAACAGCGCGUGGACGGG | 20 | 2840 |
| HSV1-UL19-5241 | + | GGCGAACAGCGCGUGGACGGG | 21 | 9840 |
| HSV1-UL19-5242 | + | CGGCGAACAGCGCGUGGACGGG | 22 | 9841 |
| HSV1-UL19-5243 | + | CCGGCGAACAGCGCGUGGACGGG | 23 | 9842 |
| HSV1-UL19-5244 | + | CCCGGCGAACAGCGCGUGGACGGG | 24 | 9843 |
| HSV1-UL19-5245 | + | GGUGAACCCAAACCCGGG | 18 | 9844 |
| HSV1-UL19-5246 | + | CGGUGAACCCAAACCCGGG | 19 | 9845 |
| HSV1-UL19-2813 | + | ACGGUGAACCCAAACCCGGG | 20 | 2858 |
| HSV1-UL19-5247 | + | GACGGUGAACCCAAACCCGGG | 21 | 9846 |
| HSV1-UL19-5248 | + | CGACGGUGAACCCAAACCCGGG | 22 | 9847 |
| HSV1-UL19-5249 | + | ACGACGGUGAACCCAAACCCGGG | 23 | 9848 |
| HSV1-UL19-5250 | + | GACGACGGUGAACCCAAACCCGGG | 24 | 9849 |
| HSV1-UL19-5251 | + | AGGUCGCGCAGGGCCGGG | 18 | 9850 |
| HSV1-UL19-5252 | + | CAGGUCGCGCAGGGCCGGG | 19 | 9851 |
| HSV1-UL19-1282 | + | ACAGGUCGCGCAGGGCCGGG | 20 | 1668 |
| HSV1-UL19-5253 | + | GACAGGUCGCGCAGGGCCGGG | 21 | 9852 |
| HSV1-UL19-5254 | + | CGACAGGUCGCGCAGGGCCGGG | 22 | 9853 |
| HSV1-UL19-5255 | + | GCGACAGGUCGCGCAGGGCCGGG | 23 | 9854 |
| HSV1-UL19-5256 | + | CGCGACAGGUCGCGCAGGGCCGGG | 24 | 9855 |
| HSV1-UL19-5257 | + | AAAAAAACAGCUGGCGGG | 18 | 9856 |
| HSV1-UL19-5258 | + | CAAAAAAACAGCUGGCGGG | 19 | 9857 |
| HSV1-UL19-2671 | + | CCAAAAAAACAGCUGGCGGG | 20 | 2764 |
| HSV1-UL19-5259 | + | CCCAAAAAAACAGCUGGCGGG | 21 | 9858 |
| HSV1-UL19-5260 | + | CCCCAAAAAAACAGCUGGCGGG | 22 | 9859 |
| HSV1-UL19-5261 | + | CCCCCAAAAAAACAGCUGGCGGG | 23 | 9860 |
| HSV1-UL19-5262 | + | UCCCCCAAAAAAACAGCUGGCGGG | 24 | 9861 |
| HSV1-UL19-5263 | + | AUUGAACAGGCCCAGGGG | 18 | 9862 |
| HSV1-UL19-5264 | + | GAUUGAACAGGCCCAGGGG | 19 | 9863 |
| HSV1-UL19-2654 | + | GGAUUGAACAGGCCCAGGGG | 20 | 2756 |
| HSV1-UL19-5265 | + | CGGAUUGAACAGGCCCAGGGG | 21 | 9864 |
| HSV1-UL19-5266 | + | CCGGAUUGAACAGGCCCAGGGG | 22 | 9865 |
| HSV1-UL19-5267 | + | ACCGGAUUGAACAGGCCCAGGGG | 23 | 9866 |
| HSV1-UL19-5268 | + | GACCGGAUUGAACAGGCCCAGGGG | 24 | 9867 |
| HSV1-UL19-5269 | + | AAGAACUUAAAGCAGGGG | 18 | 9868 |
| HSV1-UL19-5270 | + | AAAGAACUUAAAGCAGGGG | 19 | 9869 |
| HSV1-UL19-2870 | + | UAAAGAACUUAAAGCAGGGG | 20 | 2895 |
| HSV1-UL19-5271 | + | GUAAAGAACUUAAAGCAGGGG | 21 | 9870 |
| HSV1-UL19-5272 | + | CGUAAAGAACUUAAAGCAGGGG | 22 | 9871 |
| HSV1-UL19-5273 | + | ACGUAAAGAACUUAAAGCAGGGG | 23 | 9872 |
| HSV1-UL19-5274 | + | GACGUAAAGAACUUAAAGCAGGGG | 24 | 9873 |
| HSV1-UL19-5275 | + | GCGCACCCGAACACGGGG | 18 | 9874 |
| HSV1-UL19-5276 | + | GGCGCACCCGAACACGGGG | 19 | 9875 |
| HSV1-UL19-2844 | + | GGGCGCACCCGAACACGGGG | 20 | 2877 |
| HSV1-UL19-5277 | + | UGGGCGCACCCGAACACGGGG | 21 | 9876 |
| HSV1-UL19-5278 | + | CUGGGCGCACCCGAACACGGGG | 22 | 9877 |
| HSV1-UL19-5279 | + | CCUGGGCGCACCCGAACACGGGG | 23 | 9878 |
| HSV1-UL19-5280 | + | ACCUGGGCGCACCCGAACACGGGG | 24 | 9879 |
| HSV1-UL19-5281 | + | AACAGCGCGUGGACGGGG | 18 | 9880 |
| HSV1-UL19-5282 | + | GAACAGCGCGUGGACGGGG | 19 | 9881 |
| HSV1-UL19-1272 | + | CGAACAGCGCGUGGACGGGG | 20 | 1658 |
| HSV1-UL19-5283 | + | GCGAACAGCGCGUGGACGGGG | 21 | 9882 |
| HSV1-UL19-5284 | + | GGCGAACAGCGCGUGGACGGGG | 22 | 9883 |
| HSV1-UL19-5285 | + | CGGCGAACAGCGCGUGGACGGGG | 23 | 9884 |
| HSV1-UL19-5286 | + | CCGGCGAACAGCGCGUGGACGGGG | 24 | 9885 |
| HSV1-UL19-5287 | + | GGAUCCGGGUGGCCGGGG | 18 | 9886 |
| HSV1-UL19-5288 | + | GGGAUCCGGGUGGCCGGGG | 19 | 9887 |
| HSV1-UL19-2661 | + | GGGGAUCCGGGUGGCCGGGG | 20 | 2760 |
| HSV1-UL19-5289 | + | CGGGGAUCCGGGUGGCCGGGG | 21 | 9888 |
| HSV1-UL19-5290 | + | GCGGGGAUCCGGGUGGCCGGGG | 22 | 9889 |
| HSV1-UL19-5291 | + | CGCGGGGAUCCGGGUGGCCGGGG | 23 | 9890 |
| HSV1-UL19-5292 | + | GCGCGGGGAUCCGGGUGGCCGGGG | 24 | 9891 |
| HSV1-UL19-5293 | + | AAAAAACAGCUGGCGGGG | 18 | 9892 |
| HSV1-UL19-5294 | + | AAAAAAACAGCUGGCGGGG | 19 | 9893 |
| HSV1-UL19-1077 | + | CAAAAAAACAGCUGGCGGGG | 20 | 1463 |
| HSV1-UL19-5295 | + | CCAAAAAAACAGCUGGCGGGG | 21 | 9894 |
| HSV1-UL19-5296 | + | CCCAAAAAAACAGCUGGCGGGG | 22 | 9895 |
| HSV1-UL19-5297 | + | CCCCAAAAAAACAGCUGGCGGGG | 23 | 9896 |
| HSV1-UL19-5298 | + | CCCCCAAAAAAACAGCUGGCGGGG | 24 | 9897 |
| HSV1-UL19-5299 | + | AUGGCGCCCACCAGGGGG | 18 | 9898 |
| HSV1-UL19-5300 | + | CAUGGCGCCCACCAGGGGG | 19 | 9899 |
| HSV1-UL19-2649 | + | CCAUGGCGCCCACCAGGGGG | 20 | 2753 |
| HSV1-UL19-5301 | + | UCCAUGGCGCCCACCAGGGGG | 21 | 9900 |
| HSV1-UL19-5302 | + | GUCCAUGGCGCCCACCAGGGGG | 22 | 9901 |
| HSV1-UL19-5303 | + | GGUCCAUGGCGCCCACCAGGGGG | 23 | 9902 |
| HSV1-UL19-5304 | + | AGGUCCAUGGCGCCCACCAGGGGG | 24 | 9903 |
| HSV1-UL19-5305 | + | GAUCCGGGUGGCCGGGGG | 18 | 9904 |
| HSV1-UL19-5306 | + | GGAUCCGGGUGGCCGGGGG | 19 | 9905 |
| HSV1-UL19-1064 | + | GGGAUCCGGGUGGCCGGGGG | 20 | 1450 |
| HSV1-UL19-5307 | + | GGGGAUCCGGGUGGCCGGGGG | 21 | 9906 |
| HSV1-UL19-5308 | + | CGGGGAUCCGGGUGGCCGGGGG | 22 | 9907 |
| HSV1-UL19-5309 | + | GCGGGGAUCCGGGUGGCCGGGGG | 23 | 9908 |
| HSV1-UL19-5310 | + | CGCGGGGAUCCGGGUGGCCGGGGG | 24 | 9909 |
| HSV1-UL19-5311 | + | CCCCAAAAAAACAGCUGG | 18 | 9910 |
| HSV1-UL19-5312 | + | CCCCCAAAAAAACAGCUGG | 19 | 9911 |
| HSV1-UL19-1080 | + | UCCCCCAAAAAAACAGCUGG | 20 | 1466 |
| HSV1-UL19-5313 | + | UUCCCCCAAAAAAACAGCUGG | 21 | 9912 |
| HSV1-UL19-5314 | + | CUUCCCCCAAAAAAACAGCUGG | 22 | 9913 |
| HSV1-UL19-5315 | + | CCUUCCCCCAAAAAAACAGCUGG | 23 | 9914 |
| HSV1-UL19-5316 | + | UCCUUCCCCCAAAAAAACAGCUGG | 24 | 9915 |
| HSV1-UL19-5317 | + | ACGUUCGCGCGCGGCUGG | 18 | 9916 |
| HSV1-UL19-5318 | + | CACGUUCGCGCGCGGCUGG | 19 | 9917 |
| HSV1-UL19-2826 | + | CCACGUUCGCGCGCGGCUGG | 20 | 2868 |
| HSV1-UL19-5319 | + | UCCACGUUCGCGCGCGGCUGG | 21 | 9918 |
| HSV1-UL19-5320 | + | GUCCACGUUCGCGCGCGGCUGG | 22 | 9919 |
| HSV1-UL19-5321 | + | GGUCCACGUUCGCGCGCGGCUGG | 23 | 9920 |
| HSV1-UL19-5322 | + | AGGUCCACGUUCGCGCGCGGCUGG | 24 | 9921 |
| HSV1-UL19-5323 | + | CCGGCGAACAGCGCGUGG | 18 | 9922 |
| HSV1-UL19-5324 | + | CCCGGCGAACAGCGCGUGG | 19 | 9923 |
| HSV1-UL19-2782 | + | CCCCGGCGAACAGCGCGUGG | 20 | 2841 |
| HSV1-UL19-5325 | + | GCCCCGGCGAACAGCGCGUGG | 21 | 9924 |
| HSV1-UL19-5326 | + | GGCCCCGGCGAACAGCGCGUGG | 22 | 9925 |
| HSV1-UL19-5327 | + | CGGCCCCGGCGAACAGCGCGUGG | 23 | 9926 |
| HSV1-UL19-5328 | + | UCGGCCCCGGCGAACAGCGCGUGG | 24 | 9927 |
| HSV1-UL19-5329 | + | GCGCGGGGAUCCGGGUGG | 18 | 9928 |
| HSV1-UL19-5330 | + | GGCGCGGGGAUCCGGGUGG | 19 | 9929 |
| HSV1-UL19-2664 | + | AGGCGCGGGGAUCCGGGUGG | 20 | 2761 |
| HSV1-UL19-5331 | + | AAGGCGCGGGGAUCCGGGUGG | 21 | 9930 |
| HSV1-UL19-5332 | + | GAAGGCGCGGGGAUCCGGGUGG | 22 | 9931 |
| HSV1-UL19-5333 | + | GGAAGGCGCGGGGAUCCGGGUGG | 23 | 9932 |
| HSV1-UL19-5334 | + | GGGAAGGCGCGGGGAUCCGGGUGG | 24 | 9933 |
| HSV1-UL19-5335 | + | GAACGGCGGCGAUGGUGG | 18 | 9934 |
| HSV1-UL19-5336 | + | CGAACGGCGGCGAUGGUGG | 19 | 9935 |
| HSV1-UL19-1146 | + | GCGAACGGCGGCGAUGGUGG | 20 | 1532 |
| HSV1-UL19-5337 | + | CGCGAACGGCGGCGAUGGUGG | 21 | 9936 |
| HSV1-UL19-5338 | + | CCGCGAACGGCGGCGAUGGUGG | 22 | 9937 |
| HSV1-UL19-5339 | + | CCCGCGAACGGCGGCGAUGGUGG | 23 | 9938 |
| HSV1-UL19-5340 | + | CCCCGCGAACGGCGGCGAUGGUGG | 24 | 9939 |
| HSV1-UL19-5341 | + | ACCUGCAGCGUGAGCAUG | 18 | 9940 |
| HSV1-UL19-5342 | + | CACCUGCAGCGUGAGCAUG | 19 | 9941 |
| HSVI-UL19-2762 | + | GCACCUGCAGCGUGAGCAUG | 20 | 2829 |
| HSV1-UL19-5343 | + | AGCACCUGCAGCGUGAGCAUG | 21 | 9942 |
| HSV1-UL19-5344 | + | CAGCACCUGCAGCGUGAGCAUG | 22 | 9943 |
| HSV1-UL19-5345 | + | CCAGCACCUGCAGCGUGAGCAUG | 23 | 9944 |
| HSV1-UL19-5346 | + | GCCAGCACCUGCAGCGUGAGCAUG | 24 | 9945 |
| HSV1-UL19-5347 | + | GAUGGUAUGGUCCAGAUG | 18 | 9946 |
| HSV1-UL19-5348 | + | GGAUGGUAUGGUCCAGAUG | 19 | 9947 |
| HSV1-UL19-2774 | + | UGGAUGGUAUGGUCCAGAUG | 20 | 2838 |
| HSV1-UL19-5349 | + | UUGGAUGGUAUGGUCCAGAUG | 21 | 9948 |
| HSV1-UL19-5350 | + | UUUGGAUGGUAUGGUCCAGAUG | 22 | 9949 |
| HSV1-UL19-5351 | + | UUUUGGAUGGUAUGGUCCAGAUG | 23 | 9950 |
| HSV1-UL19-5352 | + | AUUUUGGAUGGUAUGGUCCAGAUG | 24 | 9951 |
| HSV1-UL19-5353 | + | UCCCCGUUGCGGGCACUG | 18 | 9952 |
| HSV1-UL19-5354 | + | UUCCCCGUUGCGGGCACUG | 19 | 9953 |
| HSV1-UL19-1432 | + | CUUCCCCGUUGCGGGCACUG | 20 | 1818 |
| HSV1-UL19-5355 | + | GCUUCCCCGUUGCGGGCACUG | 21 | 9954 |
| HSV1-UL19-5356 | + | GGCUUCCCCGUUGCGGGCACUG | 22 | 9955 |
| HSV1-UL19-5357 | + | GGGCUUCCCCGUUGCGGGCACUG | 23 | 9956 |
| HSV1-UL19-5358 | + | UGGGCUUCCCCGUUGCGGGCACUG | 24 | 9957 |
| HSV1-UL19-5359 | + | CACUUUCAGGAAGGACUG | 18 | 9958 |
| HSV1-UL19-5360 | + | CCACUUUCAGGAAGGACUG | 19 | 9959 |
| HSV1-UL19-2632 | + | UCCACUUUCAGGAAGGACUG | 20 | 2742 |
| HSV1-UL19-5361 | + | CUCCACUUUCAGGAAGGACUG | 21 | 9960 |
| HSV1-UL19-5362 | + | CCUCCACUUUCAGGAAGGACUG | 22 | 9961 |
| HSV1-UL19-5363 | + | UCCUCCACUUUCAGGAAGGACUG | 23 | 9962 |
| HSV1-UL19-5364 | + | GUCCUCCACUUUCAGGAAGGACUG | 24 | 9963 |
| HSV1-UL19-5365 | + | AGUCACAAAGCGGUCCUG | 18 | 9964 |
| HSV1-UL19-5366 | + | CAGUCACAAAGCGGUCCUG | 19 | 9965 |
| HSV1-UL19-2817 | + | UCAGUCACAAAGCGGUCCUG | 20 | 2861 |
| HSV1-UL19-5367 | + | CUCAGUCACAAAGCGGUCCUG | 21 | 9966 |
| HSV1-UL19-5368 | + | UCUCAGUCACAAAGCGGUCCUG | 22 | 9967 |
| HSV1-UL19-5369 | + | UUCUCAGUCACAAAGCGGUCCUG | 23 | 9968 |
| HSV1-UL19-5370 | + | GUUCUCAGUCACAAAGCGGUCCUG | 24 | 9969 |
| HSV1-UL19-5371 | + | CCCCCAAAAAAACAGCUG | 18 | 9970 |
| HSV1-UL19-5372 | + | UCCCCCAAAAAAACAGCUG | 19 | 9971 |
| HSV1-UL19-2673 | + | UUCCCCCAAAAAAACAGCUG | 20 | 2765 |
| HSV1-UL19-5373 | + | CUUCCCCCAAAAAAACAGCUG | 21 | 9972 |
| HSV1-UL19-5374 | + | CCUUCCCCCAAAAAAACAGCUG | 22 | 9973 |
| HSV1-UL19-5375 | + | UCCUUCCCCCAAAAAAACAGCUG | 23 | 9974 |
| HSV1-UL19-5376 | + | GUCCUUCCCCCAAAAAAACAGCUG | 24 | 9975 |
| HSV1-UL19-5377 | + | GGUAUGGUCCAGAUGCUG | 18 | 9976 |
| HSV1-UL19-5378 | + | UGGUAUGGUCCAGAUGCUG | 19 | 9977 |
| HSV1-UL19-1263 | + | AUGGUAUGGUCCAGAUGCUG | 20 | 1649 |
| HSV1-UL19-5379 | + | GAUGGUAUGGUCCAGAUGCUG | 21 | 9978 |
| HSV1-UL19-5380 | + | GGAUGGUAUGGUCCAGAUGCUG | 22 | 9979 |
| HSV1-UL19-5381 | + | UGGAUGGUAUGGUCCAGAUGCUG | 23 | 9980 |
| HSV1-UL19-5382 | + | UUGGAUGGUAUGGUCCAGAUGCUG | 24 | 9981 |
| HSV1-UL19-5383 | + | GCGCACGCGCGUCGUCUG | 18 | 9982 |
| HSV1-UL19-5384 | + | CGCGCACGCGCGUCGUCUG | 19 | 9983 |
| HSV1-UL19-1036 | + | GCGCGCACGCGCGUCGUCUG | 20 | 1422 |
| HSV1-UL19-5385 | + | CGCGCGCACGCGCGUCGUCUG | 21 | 9984 |
| HSV1-UL19-5386 | + | CCGCGCGCACGCGCGUCGUCUG | 22 | 9985 |
| HSV1-UL19-5387 | + | UCCGCGCGCACGCGCGUCGUCUG | 23 | 9986 |
| HSV1-UL19-5388 | + | AUCCGCGCGCACGCGCGUCGUCUG | 24 | 9987 |
| HSV1-UL19-5389 | + | GAGGGCUGCGUGGCCGUG | 18 | 9988 |
| HSV1-UL19-5390 | + | GGAGGGCUGCGUGGCCGUG | 19 | 9989 |
| HSV1-UL19-2622 | + | CGGAGGGCUGCGUGGCCGUG | 20 | 2736 |
| HSV1-UL19-5391 | + | ACGGAGGGCUGCGUGGCCGUG | 21 | 9990 |
| HSV1-UL19-5392 | + | CACGGAGGGCUGCGUGGCCGUG | 22 | 9991 |
| HSV1-UL19-5393 | + | CCACGGAGGGCUGCGUGGCCGUG | 23 | 9992 |
| HSV1-UL19-5394 | + | GCCACGGAGGGCUGCGUGGCCGUG | 24 | 9993 |
| HSV1-UL19-5395 | + | CCCCCCGCCAGUUUCGUG | 18 | 9994 |
| HSV1-UL19-5396 | + | CCCCCCCGCCAGUUUCGUG | 19 | 9995 |
| HSV1-UL19-2824 | + | ACCCCCCCGCCAGUUUCGUG | 20 | 2866 |
| HSV1-UL19-5397 | + | UACCCCCCCGCCAGUUUCGUG | 21 | 9996 |
| HSV1-UL19-5398 | + | UUACCCCCCCGCCAGUUUCGUG | 22 | 9997 |
| HSV1-UL19-5399 | + | UUUACCCCCCCGCCAGUUUCGUG | 23 | 9998 |
| HSV1-UL19-5400 | + | GUUUACCCCCCCGCCAGUUUCGUG | 24 | 9999 |
| HSV1-UL19-5401 | + | AGGUUGCCCAUGUCGGUG | 18 | 10000 |
| HSV1-UL19-5402 | + | AAGGUUGCCCAUGUCGGUG | 19 | 10001 |
| HSV1-UL19-2830 | + | GAAGGUUGCCCAUGUCGGUG | 20 | 2871 |
| HSV1-UL19-5403 | + | GGAAGGUUGCCCAUGUCGGUG | 21 | 10002 |
| HSV1-UL19-5404 | + | GGGAAGGUUGCCCAUGUCGGUG | 22 | 10003 |
| HSV1-UL19-5405 | + | GGGGAAGGUUGCCCAUGUCGGUG | 23 | 10004 |
| HSV1-UL19-5406 | + | UGGGGAAGGUUGCCCAUGUCGGUG | 24 | 10005 |
| HSV1-UL19-5407 | + | CACCAGAUUGGCCGGGUG | 18 | 10006 |
| HSV1-UL19-5408 | + | CCACCAGAUUGGCCGGGUG | 19 | 10007 |
| HSV1-UL19-2759 | + | GCCACCAGAUUGGCCGGGUG | 20 | 2826 |
| HSV1-UL19-5409 | + | GGCCACCAGAUUGGCCGGGUG | 21 | 10008 |
| HSV1-UL19-5410 | + | UGGCCACCAGAUUGGCCGGGUG | 22 | 10009 |
| HSV1-UL19-5411 | + | UUGGCCACCAGAUUGGCCGGGUG | 23 | 10010 |
| HSV1-UL19-5412 | + | GUUGGCCACCAGAUUGGCCGGGUG | 24 | 10011 |
| HSV1-UL19-5413 | + | CGAACGGCGGCGAUGGUG | 18 | 10012 |
| HSV1-UL19-5414 | + | GCGAACGGCGGCGAUGGUG | 19 | 10013 |
| HSV1-UL19-2709 | + | CGCGAACGGCGGCGAUGGUG | 20 | 2788 |
| HSV1-UL19-5415 | + | CCGCGAACGGCGGCGAUGGUG | 21 | 10014 |
| HSV1-UL19-5416 | + | CCCGCGAACGGCGGCGAUGGUG | 22 | 10015 |
| HSV1-UL19-5417 | + | CCCCGCGAACGGCGGCGAUGGUG | 23 | 10016 |
| HSV1-UL19-5418 | + | GCCCCGCGAACGGCGGCGAUGGUG | 24 | 10017 |
| HSV1-UL19-5419 | + | UUGAUCGGGGCGGUGGUG | 18 | 10018 |
| HSV1-UL19-5420 | + | UUUGAUCGGGGCGGUGGUG | 19 | 10019 |
| HSV1-UL19-2628 | + | GUUUGAUCGGGGCGGUGGUG | 20 | 2740 |
| HSV1-UL19-5421 | + | UGUUUGAUCGGGGCGGUGGUG | 21 | 10020 |
| HSV1-UL19-5422 | + | CUGUUUGAUCGGGGCGGUGGUG | 22 | 10021 |
| HSV1-UL19-5423 | + | GCUGUUUGAUCGGGGCGGUGGUG | 23 | 10022 |
| HSV1-UL19-5424 | + | CGCUGUUUGAUCGGGGCGGUGGUG | 24 | 10023 |
| HSV1-UL19-5425 | + | AAUAUGCGCAUGUUGGUG | 18 | 10024 |
| HSV1-UL19-5426 | + | GAAUAUGCGCAUGUUGGUG | 19 | 10025 |
| HSV1-UL19-2768 | + | CGAAUAUGCGCAUGUUGGUG | 20 | 2835 |
| HSV1-UL19-5427 | + | UCGAAUAUGCGCAUGUUGGUG | 21 | 10026 |
| HSV1-UL19-5428 | + | GUCGAAUAUGCGCAUGUUGGUG | 22 | 10027 |
| HSV1-UL19-5429 | + | CGUCGAAUAUGCGCAUGUUGGUG | 23 | 10028 |
| HSV1-UL19-5430 | + | CCGUCGAAUAUGCGCAUGUUGGUG | 24 | 10029 |
| HSV1-UL19-5431 | + | AGAGCCAGGUUGGCGUUG | 18 | 10030 |
| HSV1-UL19-5432 | + | AAGAGCCAGGUUGGCGUUG | 19 | 10031 |
| HSV1-UL19-2688 | + | CAAGAGCCAGGUUGGCGUUG | 20 | 2776 |
| HSV1-UL19-5433 | + | UCAAGAGCCAGGUUGGCGUUG | 21 | 10032 |
| HSV1-UL19-5434 | + | CUCAAGAGCCAGGUUGGCGUUG | 22 | 10033 |
| HSV1-UL19-5435 | + | GCUCAAGAGCCAGGUUGGCGUUG | 23 | 10034 |
| HSV1-UL19-5436 | + | AGCUCAAGAGCCAGGUUGGCGUUG | 24 | 10035 |
| HSV1-UL19-5437 | + | GCGCGUCCCCGGGGGUUG | 18 | 10036 |
| HSV1-UL19-5438 | + | GGCGCGUCCCCGGGGGUUG | 19 | 10037 |
| HSV1-UL19-2855 | + | CGGCGCGUCCCCGGGGGUUG | 20 | 2883 |
| HSV1-UL19-5439 | + | GCGGCGCGUCCCCGGGGGUUG | 21 | 10038 |
| HSV1-UL19-5440 | + | GGCGGCGCGUCCCCGGGGGUUG | 22 | 10039 |
| HSV1-UL19-5441 | + | CGGCGGCGCGUCCCCGGGGGUUG | 23 | 10040 |
| HSV1-UL19-5442 | + | CCGGCGGCGCGUCCCCGGGGGUUG | 24 | 10041 |
| HSV1-UL19-5443 | + | GCCCAGGUAAAAGUUUUG | 18 | 10042 |
| HSV1-UL19-5444 | + | GGCCCAGGUAAAAGUUUUG | 19 | 10043 |
| HSV1-UL19-1335 | + | CGGCCCAGGUAAAAGUUUUG | 20 | 1721 |
| HSV1-UL19-5445 | + | GCGGCCCAGGUAAAAGUUUUG | 21 | 10044 |
| HSV1-UL19-5446 | + | CGCGGCCCAGGUAAAAGUUUUG | 22 | 10045 |
| HSV1-UL19-5447 | + | CCGCGGCCCAGGUAAAAGUUUUG | 23 | 10046 |
| HSV1-UL19-5448 | + | CCCGCGGCCCAGGUAAAAGUUUUG | 24 | 10047 |
| HSV1-UL19-5449 | + | CUGGCCGUGGUCGUACAU | 18 | 10048 |
| HSV1-UL19-5450 | + | UCUGGCCGUGGUCGUACAU | 19 | 10049 |
| HSV1-UL19-2859 | + | CUCUGGCCGUGGUCGUACAU | 20 | 2885 |
| HSV1-UL19-5451 | + | GCUCUGGCCGUGGUCGUACAU | 21 | 10050 |
| HSV1-UL19-5452 | + | CGCUCUGGCCGUGGUCGUACAU | 22 | 10051 |
| HSV1-UL19-5453 | + | UCGCUCUGGCCGUGGUCGUACAU | 23 | 10052 |
| HSV1-UL19-5454 | + | GUCGCUCUGGCCGUGGUCGUACAU | 24 | 10053 |
| HSV1-UL19-5455 | + | CAGGGCCGCGCGCCGCAU | 18 | 10054 |
| HSV1-UL19-5456 | + | CCAGGGCCGCGCGCCGCAU | 19 | 10055 |
| HSV1-UL19-2733 | + | UCCAGGGCCGCGCGCCGCAU | 20 | 2809 |
| HSV1-UL19-5457 | + | GUCCAGGGCCGCGCGCCGCAU | 21 | 10056 |
| HSV1-UL19-5458 | + | GGUCCAGGGCCGCGCGCCGCAU | 22 | 10057 |
| HSV1-UL19-5459 | + | CGGUCCAGGGCCGCGCGCCGCAU | 23 | 10058 |
| HSV1-UL19-5460 | + | GCGGUCCAGGGCCGCGCGCCGCAU | 24 | 10059 |
| HSV1-UL19-5461 | + | CAGGAGGCGCUGUUUGAU | 18 | 10060 |
| HSV1-UL19-5462 | + | GCAGGAGGCGCUGUUUGAU | 19 | 10061 |
| HSV1-UL19-1017 | + | UGCAGGAGGCGCUGUUUGAU | 20 | 1403 |
| HSV1-UL19-5463 | + | CUGCAGGAGGCGCUGUUUGAU | 21 | 10062 |
| HSV1-UL19-5464 | + | ACUGCAGGAGGCGCUGUUUGAU | 22 | 10063 |
| HSV1-UL19-5465 | + | GACUGCAGGAGGCGCUGUUUGAU | 23 | 10064 |
| HSV1-UL19-5466 | + | GGACUGCAGGAGGCGCUGUUUGAU | 24 | 10065 |
| HSV1-UL19-5467 | + | UUCCCCGUUGCGGGCACU | 18 | 10066 |
| HSV1-UL19-5468 | + | CUUCCCCGUUGCGGGCACU | 19 | 10067 |
| HSV1-UL19-2891 | + | GCUUCCCCGUUGCGGGCACU | 20 | 2909 |
| HSV1-UL19-5469 | + | GGCUUCCCCGUUGCGGGCACU | 21 | 10068 |
| HSV1-UL19-5470 | + | GGGCUUCCCCGUUGCGGGCACU | 22 | 10069 |
| HSV1-UL19-5471 | + | UGGGCUUCCCCGUUGCGGGCACU | 23 | 10070 |
| HSV1-UL19-5472 | + | GUGGGCUUCCCCGUUGCGGGCACU | 24 | 10071 |
| HSV1-UL19-5473 | + | ACACGGCCAUGCACUCCU | 18 | 10072 |
| HSV1-UL19-5474 | + | UACACGGCCAUGCACUCCU | 19 | 10073 |
| HSV1-UL19-1167 | + | GUACACGGCCAUGCACUCCU | 20 | 1553 |
| HSV1-UL19-5475 | + | GGUACACGGCCAUGCACUCCU | 21 | 10074 |
| HSV1-UL19-5476 | + | CGGUACACGGCCAUGCACUCCU | 22 | 10075 |
| HSV1-UL19-5477 | + | CCGGUACACGGCCAUGCACUCCU | 23 | 10076 |
| HSV1-UL19-5478 | + | CCCGGUACACGGCCAUGCACUCCU | 24 | 10077 |
| HSV1-UL19-5479 | + | GCGCCUGCCCGCCCAGCU | 18 | 10078 |
| HSV1-UL19-5480 | + | UGCGCCUGCCCGCCCAGCU | 19 | 10079 |
| HSV1-UL19-2728 | + | UUGCGCCUGCCCGCCCAGCU | 20 | 2805 |
| HSV1-UL19-5481 | + | CUUGCGCCUGCCCGCCCAGCU | 21 | 10080 |
| HSV1-UL19-5482 | + | GCUUGCGCCUGCCCGCCCAGCU | 22 | 10081 |
| HSV1-UL19-5483 | + | GGCUUGCGCCUGCCCGCCCAGCU | 23 | 10082 |
| HSV1-UL19-5484 | + | CGGCUUGCGCCUGCCCGCCCAGCU | 24 | 10083 |
| HSV1-UL19-5485 | + | ACGCCUCCGACGCGCGCU | 18 | 10084 |
| HSV1-UL19-5486 | + | UACGCCUCCGACGCGCGCU | 19 | 10085 |
| HSV1-UL19-2819 | + | GUACGCCUCCGACGCGCGCU | 20 | 2863 |
| HSV1-UL19-5487 | + | AGUACGCCUCCGACGCGCGCU | 21 | 10086 |
| HSV1-UL19-5488 | + | AAGUACGCCUCCGACGCGCGCU | 22 | 10087 |
| HSV1-UL19-5489 | + | GAAGUACGCCUCCGACGCGCGCU | 23 | 10088 |
| HSV1-UL19-5490 | + | GGAAGUACGCCUCCGACGCGCGCU | 24 | 10089 |
| HSV1-UL19-5491 | + | UGGUAUGGUCCAGAUGCU | 18 | 10090 |
| HSV1-UL19-5492 | + | AUGGUAUGGUCCAGAUGCU | 19 | 10091 |
| HSV1-UL19-1264 | + | GAUGGUAUGGUCCAGAUGCU | 20 | 1650 |
| HSV1-UL19-5493 | + | GGAUGGUAUGGUCCAGAUGCU | 21 | 10092 |
| HSV1-UL19-5494 | + | UGGAUGGUAUGGUCCAGAUGCU | 22 | 10093 |
| HSV1-UL19-5495 | + | UUGGAUGGUAUGGUCCAGAUGCU | 23 | 10094 |
| HSV1-UL19-5496 | + | UUUGGAUGGUAUGGUCCAGAUGCU | 24 | 10095 |
| HSV1-UL19-5497 | + | CGGGCUGCAUAAACUGCU | 18 | 10096 |
| HSV1-UL19-5498 | + | UCGGGCUGCAUAAACUGCU | 19 | 10097 |
| HSV1-UL19-2687 | + | GUCGGGCUGCAUAAACUGCU | 20 | 2775 |
| HSV1-UL19-5499 | + | UGUCGGGCUGCAUAAACUGCU | 21 | 10098 |
| HSV1-UL19-5500 | + | UUGUCGGGCUGCAUAAACUGCU | 22 | 10099 |
| HSV1-UL19-5501 | + | GUUGUCGGGCUGCAUAAACUGCU | 23 | 10100 |
| HSV1-UL19-5502 | + | CGUUGUCGGGCUGCAUAAACUGCU | 24 | 10101 |
| HSV1-UL19-5503 | + | CCUCGCCGGGGGCGAUCU | 18 | 10102 |
| HSV1-UL19-5504 | + | UCCUCGCCGGGGGCGAUCU | 19 | 10103 |
| HSV1-UL19-2747 | + | CUCCUCGCCGGGGGCGAUCU | 20 | 2821 |
| HSV1-UL19-5505 | + | ACUCCUCGCCGGGGGCGAUCU | 21 | 10104 |
| HSV1-UL19-5506 | + | CACUCCUCGCCGGGGGCGAUCU | 22 | 10105 |
| HSV1-UL19-5507 | + | GCACUCCUCGCCGGGGGCGAUCU | 23 | 10106 |
| HSV1-UL19-5508 | + | GGCACUCCUCGCCGGGGGCGAUCU | 24 | 10107 |
| HSV1-UL19-5509 | + | CGCGCACGCGCGUCGUCU | 18 | 10108 |
| HSV1-UL19-5510 | + | GCGCGCACGCGCGUCGUCU | 19 | 10109 |
| HSV1-UL19-1037 | + | CGCGCGCACGCGCGUCGUCU | 20 | 1423 |
| HSV1-UL19-5511 | + | CCGCGCGCACGCGCGUCGUCU | 21 | 10110 |
| HSV1-UL19-5512 | + | UCCGCGCGCACGCGCGUCGUCU | 22 | 10111 |
| HSV1-UL19-5513 | + | AUCCGCGCGCACGCGCGUCGUCU | 23 | 10112 |
| HSV1-UL19-5514 | + | GAUCCGCGCGCACGCGCGUCGUCU | 24 | 10113 |
| HSV1-UL19-5515 | + | CGAGGUAGGUCACGACGU | 18 | 10114 |
| HSV1-UL19-5516 | + | CCGAGGUAGGUCACGACGU | 19 | 10115 |
| HSV1-UL19-2720 | + | CCCGAGGUAGGUCACGACGU | 20 | 2799 |
| HSV1-UL19-5517 | + | CCCCGAGGUAGGUCACGACGU | 21 | 10116 |
| HSV1-UL19-5518 | + | CCCCCGAGGUAGGUCACGACGU | 22 | 10117 |
| HSV1-UL19-5519 | + | UCCCCCGAGGUAGGUCACGACGU | 23 | 10118 |
| HSV1-UL19-5520 | + | CUCCCCCGAGGUAGGUCACGACGU | 24 | 10119 |
| HSV1-UL19-5521 | + | UAUAGAGCAGGUCCCCGU | 18 | 10120 |
| HSV1-UL19-5522 | + | UUAUAGAGCAGGUCCCCGU | 19 | 10121 |
| HSV1-UL19-2866 | + | GUUAUAGAGCAGGUCCCCGU | 20 | 2891 |
| HSV1-UL19-5523 | + | CGUUAUAGAGCAGGUCCCCGU | 21 | 10122 |
| HSV1-UL19-5524 | + | CCGUUAUAGAGCAGGUCCCCGU | 22 | 10123 |
| HSV1-UL19-5525 | + | CCCGUUAUAGAGCAGGUCCCCGU | 23 | 10124 |
| HSV1-UL19-5526 | + | CCCCGUUAUAGAGCAGGUCCCCGU | 24 | 10125 |
| HSV1-UL19-5527 | + | CGCGCGCACGCGCGUCGU | 18 | 10126 |
| HSV1-UL19-5528 | + | CCGCGCGCACGCGCGUCGU | 19 | 10127 |
| HSV1-UL19-2643 | + | UCCGCGCGCACGCGCGUCGU | 20 | 2749 |
| HSV1-UL19-5529 | + | AUCCGCGCGCACGCGCGUCGU | 21 | 10128 |
| HSV1-UL19-5530 | + | GAUCCGCGCGCACGCGCGUCGU | 22 | 10129 |
| HSV1-UL19-5531 | + | AGAUCCGCGCGCACGCGCGUCGU | 23 | 10130 |
| HSV1-UL19-5532 | + | CAGAUCCGCGCGCACGCGCGUCGU | 24 | 10131 |
| HSV1-UL19-5533 | + | UGGCGCCCACCAGGGGGU | 18 | 10132 |
| HSV1-UL19-5534 | + | AUGGCGCCCACCAGGGGGU | 19 | 10133 |
| HSV1-UL19-1046 | + | CAUGGCGCCCACCAGGGGGU | 20 | 1432 |
| HSV1-UL19-5535 | + | CCAUGGCGCCCACCAGGGGGU | 21 | 10134 |
| HSV1-UL19-5536 | + | UCCAUGGCGCCCACCAGGGGGU | 22 | 10135 |
| HSV1-UL19-5537 | + | GUCCAUGGCGCCCACCAGGGGGU | 23 | 10136 |
| HSV1-UL19-5538 | + | GGUCCAUGGCGCCCACCAGGGGGU | 24 | 10137 |
| HSV1-UL19-5539 | + | GCGGCCCAGGUAAAAGUU | 18 | 10138 |
| HSV1-UL19-5540 | + | CGCGGCCCAGGUAAAAGUU | 19 | 10139 |
| HSV1-UL19-2834 | + | CCGCGGCCCAGGUAAAAGUU | 20 | 2872 |
| HSV1-UL19-5541 | + | CCCGCGGCCCAGGUAAAAGUU | 21 | 10140 |
| HSV1-UL19-5542 | + | CCCCGCGGCCCAGGUAAAAGUU | 22 | 10141 |
| HSV1-UL19-5543 | + | GCCCCGCGGCCCAGGUAAAAGUU | 23 | 10142 |
| HSV1-UL19-5544 | + | AGCCCCGCGGCCCAGGUAAAAGUU | 24 | 10143 |
| HSV1-UL19-3515 | + | GAGGACGGCCUGGACGUU | 18 | 8114 |
| HSV1-UL19-3516 | + | CGAGGACGGCCUGGACGUU | 19 | 8115 |
| HSV1-UL19-3517 | + | CCGAGGACGGCCUGGACGUU | 20 | 8116 |
| HSV1-UL19-3518 | + | CCCGAGGACGGCCUGGACGUU | 21 | 8117 |
| HSV1-UL19-3519 | + | CCCCGAGGACGGCCUGGACGUU | 22 | 8118 |
| HSV1-UL19-3520 | + | GCCCCGAGGACGGCCUGGACGUU | 23 | 8119 |
| HSV1-UL19-3521 | + | CGCCCCGAGGACGGCCUGGACGUU | 24 | 8120 |
| HSV1-UL19-5545 | + | GACCAGGUUGGCCCCGUU | 18 | 10144 |
| HSV1-UL19-5546 | + | UGACCAGGUUGGCCCCGUU | 19 | 10145 |
| HSV1-UL19-2636 | + | GUGACCAGGUUGGCCCCGUU | 20 | 2745 |
| HSV1-UL19-5547 | + | CGUGACCAGGUUGGCCCCGUU | 21 | 10146 |
| HSV1-UL19-5548 | + | CCGUGACCAGGUUGGCCCCGUU | 22 | 10147 |
| HSV1-UL19-5549 | + | GCCGUGACCAGGUUGGCCCCGUU | 23 | 10148 |
| HSV1-UL19-5550 | + | CGCCGUGACCAGGUUGGCCCCGUU | 24 | 10149 |
| HSV1-UL19-5551 | + | CGCGUGGGCUUCCCCGUU | 18 | 10150 |
| HSV1-UL19-5552 | + | GCGCGUGGGCUUCCCCGUU | 19 | 10151 |
| HSV1-UL19-2892 | + | CGCGCGUGGGCUUCCCCGUU | 20 | 2910 |
| HSV1-UL19-5553 | + | CCGCGCGUGGGCUUCCCCGUU | 21 | 10152 |
| HSV1-UL19-5554 | + | CCCGCGCGUGGGCUUCCCCGUU | 22 | 10153 |
| HSV1-UL19-5555 | + | UCCCGCGCGUGGGCUUCCCCGUU | 23 | 10154 |
| HSV1-UL19-5556 | + | CUCCCGCGCGUGGGCUUCCCCGUU | 24 | 10155 |
| HSV1-UL19-5557 | + | CACUACCACGCGCCCGUU | 18 | 10156 |
| HSV1-UL19-5558 | + | CCACUACCACGCGCCCGUU | 19 | 10157 |
| HSV1-UL19-2761 | + | UCCACUACCACGCGCCCGUU | 20 | 2828 |
| HSV1-UL19-5559 | + | GUCCACUACCACGCGCCCGUU | 21 | 10158 |
| HSV1-UL19-5560 | + | CGUCCACUACCACGCGCCCGUU | 22 | 10159 |
| HSV1-UL19-5561 | + | CCGUCCACUACCACGCGCCCGUU | 23 | 10160 |
| HSV1-UL19-5562 | + | CCCGUCCACUACCACGCGCCCGUU | 24 | 10161 |
| HSV1-UL19-5563 | + | CAUGUCGGUGACGGGGUU | 18 | 10162 |
| HSV1-UL19-5564 | + | CCAUGUCGGUGACGGGGUU | 19 | 10163 |
| HSV1-UL19-2828 | + | CCCAUGUCGGUGACGGGGUU | 20 | 2870 |
| HSV1-UL19-5565 | + | GCCCAUGUCGGUGACGGGGUU | 21 | 10164 |
| HSV1-UL19-5566 | + | UGCCCAUGUCGGUGACGGGGUU | 22 | 10165 |
| HSV1-UL19-5567 | + | UUGCCCAUGUCGGUGACGGGGUU | 23 | 10166 |
| HSV1-UL19-5568 | + | GUUGCCCAUGUCGGUGACGGGGUU | 24 | 10167 |
| HSV1-UL19-5569 | + | UGUGCGUUCGGCCAUGUU | 18 | 10168 |
| HSV1-UL19-5570 | + | UUGUGCGUUCGGCCAUGUU | 19 | 10169 |
| HSV1-UL19-2764 | + | GUUGUGCGUUCGGCCAUGUU | 20 | 2831 |
| HSV1-UL19-5571 | + | CGUUGUGCGUUCGGCCAUGUU | 21 | 10170 |
| HSV1-UL19-5572 | + | CCGUUGUGCGUUCGGCCAUGUU | 22 | 10171 |
| HSV1-UL19-5573 | + | GCCGUUGUGCGUUCGGCCAUGUU | 23 | 10172 |
| HSV1-UL19-5574 | + | CGCCGUUGUGCGUUCGGCCAUGUU | 24 | 10173 |
| HSV1-UL19-5575 | + | UUCGGUGUCCUCCACUUU | 18 | 10174 |
| HSV1-UL19-5576 | + | CUUCGGUGUCCUCCACUUU | 19 | 10175 |
| HSV1-UL19-2635 | + | GCUUCGGUGUCCUCCACUUU | 20 | 2744 |
| HSV1-UL19-5577 | + | GGCUUCGGUGUCCUCCACUUU | 21 | 10176 |
| HSV1-UL19-5578 | + | CGGCUUCGGUGUCCUCCACUUU | 22 | 10177 |
| HSV1-UL19-5579 | + | UCGGCUUCGGUGUCCUCCACUUU | 23 | 10178 |
| HSV1-UL19-5580 | + | GUCGGCUUCGGUGUCCUCCACUUU | 24 | 10179 |
| HSV1-UL19-5581 | + | CGGCCCAGGUAAAAGUUU | 18 | 10180 |
| HSV1-UL19-5582 | + | GCGGCCCAGGUAAAAGUUU | 19 | 10181 |
| HSV1-UL19-1337 | + | CGCGGCCCAGGUAAAAGUUU | 20 | 1723 |
| HSV1-UL19-5583 | + | CCGCGGCCCAGGUAAAAGUUU | 21 | 10182 |
| HSV1-UL19-5584 | + | CCCGCGGCCCAGGUAAAAGUUU | 22 | 10183 |
| HSV1-UL19-5585 | + | CCCCGCGGCCCAGGUAAAAGUUU | 23 | 10184 |
| HSV1-UL19-5586 | + | GCCCCGCGGCCCAGGUAAAAGUUU | 24 | 10185 |
| HSV1-UL19-5587 | + | GGCCCAGGUAAAAGUUUU | 18 | 10186 |
| HSV1-UL19-5588 | + | CGGCCCAGGUAAAAGUUUU | 19 | 10187 |
| HSV1-UL19-1336 | + | GCGGCCCAGGUAAAAGUUUU | 20 | 1722 |
| HSV1-UL19-5589 | + | CGCGGCCCAGGUAAAAGUUUU | 21 | 10188 |
| HSV1-UL19-5590 | + | CCGCGGCCCAGGUAAAAGUUUU | 22 | 10189 |
| HSV1-UL19-5591 | + | CCCGCGGCCCAGGUAAAAGUUUU | 23 | 10190 |
| HSV1-UL19-5592 | + | CCCCGCGGCCCAGGUAAAAGUUUU | 24 | 10191 |
| HSV1-UL19-5593 | - | CUGCAGUCCUUCCUGAAA | 18 | 10192 |
| HSV1-UL19-5594 | - | CCUGCAGUCCUUCCUGAAA | 19 | 10193 |
| HSV1-UL19-2061 | - | UCCUGCAGUCCUUCCUGAAA | 20 | 2402 |
| HSV1-UL19-5595 | - | CUCCUGCAGUCCUUCCUGAAA | 21 | 10194 |
| HSV1-UL19-5596 | - | CCUCCUGCAGUCCUUCCUGAAA | 22 | 10195 |
| HSV1-UL19-5597 | - | GCCUCCUGCAGUCCUUCCUGAAA | 23 | 10196 |
| HSV1-UL19-5598 | - | CGCCUCCUGCAGUCCUUCCUGAAA | 24 | 10197 |
| HSV1-UL19-5599 | - | CCUGGUCGCCGAGCUAAA | 18 | 10198 |
| HSV1-UL19-5600 | - | CCCUGGUCGCCGAGCUAAA | 19 | 10199 |
| HSV1-UL19-2047 | - | ACCCUGGUCGCCGAGCUAAA | 20 | 2391 |
| HSV1-UL19-5601 | - | GACCCUGGUCGCCGAGCUAAA | 21 | 10200 |
| HSV1-UL19-5602 | - | CGACCCUGGUCGCCGAGCUAAA | 22 | 10201 |
| HSV1-UL19-5603 | - | GCGACCCUGGUCGCCGAGCUAAA | 23 | 10202 |
| HSV1-UL19-5604 | - | GGCGACCCUGGUCGCCGAGCUAAA | 24 | 10203 |
| HSV1-UL19-5605 | - | CCUCCGCAGUGCCCGCAA | 18 | 10204 |
| HSV1-UL19-5606 | - | UCCUCCGCAGUGCCCGCAA | 19 | 10205 |
| HSV1-UL19-574 | - | CUCCUCCGCAGUGCCCGCAA | 20 | 960 |
| HSV1-UL19-5607 | - | CCUCCUCCGCAGUGCCCGCAA | 21 | 10206 |
| HSV1-UL19-5608 | - | CCCUCCUCCGCAGUGCCCGCAA | 22 | 10207 |
| HSV1-UL19-5609 | - | GCCCUCCUCCGCAGUGCCCGCAA | 23 | 10208 |
| HSV1-UL19-5610 | - | CGCCCUCCUCCGCAGUGCCCGCAA | 24 | 10209 |
| HSV1-UL19-5611 | - | CGGGGCCUACCACCUCAA | 18 | 10210 |
| HSV1-UL19-5612 | - | ACGGGGCCUACCACCUCAA | 19 | 10211 |
| HSV1-UL19-554 | - | AACGGGGCCUACCACCUCAA | 20 | 940 |
| HSV1-UL19-5613 | - | UAACGGGGCCUACCACCUCAA | 21 | 10212 |
| HSV1-UL19-5614 | - | AUAACGGGGCCUACCACCUCAA | 22 | 10213 |
| HSV1-UL19-5615 | - | UAUAACGGGGCCUACCACCUCAA | 23 | 10214 |
| HSV1-UL19-5616 | - | CUAUAACGGGGCCUACCACCUCAA | 24 | 10215 |
| HSV1-UL19-5617 | - | CGGCGAGAUGGUCCUGAA | 18 | 10216 |
| HSV1-UL19-5618 | - | ACGGCGAGAUGGUCCUGAA | 19 | 10217 |
| HSV1-UL19-231 | - | UACGGCGAGAUGGUCCUGAA | 20 | 617 |
| HSV1-UL19-5619 | - | GUACGGCGAGAUGGUCCUGAA | 21 | 10218 |
| HSV1-UL19-5620 | - | CGUACGGCGAGAUGGUCCUGAA | 22 | 10219 |
| HSV1-UL19-5621 | - | ACGUACGGCGAGAUGGUCCUGAA | 23 | 10220 |
| HSV1-UL19-5622 | - | GACGUACGGCGAGAUGGUCCUGAA | 24 | 10221 |
| HSV1-UL19-5623 | - | CGGGGACCUGCUCUAUAA | 18 | 10222 |
| HSV1-UL19-5624 | - | ACGGGGACCUGCUCUAUAA | 19 | 10223 |
| HSV1-UL19-551 | - | UACGGGGACCUGCUCUAUAA | 20 | 937 |
| HSV1-UL19-5625 | - | GUACGGGGACCUGCUCUAUAA | 21 | 10224 |
| HSV1-UL19-5626 | - | CGUACGGGGACCUGCUCUAUAA | 22 | 10225 |
| HSV1-UL19-5627 | - | UCGUACGGGGACCUGCUCUAUAA | 23 | 10226 |
| HSV1-UL19-5628 | - | UUCGUACGGGGACCUGCUCUAUAA | 24 | 10227 |
| HSV1-UL19-5629 | - | UCAACGCCAUGUUUCACA | 18 | 10228 |
| HSV1-UL19-5630 | - | GUCAACGCCAUGUUUCACA | 19 | 10229 |
| HSV1-UL19-2213 | - | GGUCAACGCCAUGUUUCACA | 20 | 2491 |
| HSV1-UL19-5631 | - | CGGUCAACGCCAUGUUUCACA | 21 | 10230 |
| HSV1-UL19-5632 | - | ACGGUCAACGCCAUGUUUCACA | 22 | 10231 |
| HSV1-UL19-5633 | - | CACGGUCAACGCCAUGUUUCACA | 23 | 10232 |
| HSV1-UL19-5634 | - | ACACGGUCAACGCCAUGUUUCACA | 24 | 10233 |
| HSV1-UL19-5635 | - | UCCUGAAAGUGGAGGACA | 18 | 10234 |
| HSV1-UL19-5636 | - | UUCCUGAAAGUGGAGGACA | 19 | 10235 |
| HSV1-UL19-2064 | - | CUUCCUGAAAGUGGAGGACA | 20 | 2404 |
| HSV1-UL19-5637 | - | CCUUCCUGAAAGUGGAGGACA | 21 | 10236 |
| HSV1-UL19-5638 | - | UCCUUCCUGAAAGUGGAGGACA | 22 | 10237 |
| HSV1-UL19-5639 | - | GUCCUUCCUGAAAGUGGAGGACA | 23 | 10238 |
| HSV1-UL19-5640 | - | AGUCCUUCCUGAAAGUGGAGGACA | 24 | 10239 |
| HSV1-UL19-5641 | - | GCGUUUACGCGGGGGACA | 18 | 10240 |
| HSV1-UL19-5642 | - | GGCGUUUACGCGGGGGACA | 19 | 10241 |
| HSV1-UL19-536 | - | GGGCGUUUACGCGGGGGACA | 20 | 922 |
| HSV1-UL19-5643 | - | GGGGCGUUUACGCGGGGGACA | 21 | 10242 |
| HSV1-UL19-5644 | - | GGGGGCGUUUACGCGGGGGACA | 22 | 10243 |
| HSV1-UL19-5645 | - | CGGGGGCGUUUACGCGGGGGACA | 23 | 10244 |
| HSV1-UL19-5646 | - | CCGGGGGCGUUUACGCGGGGGACA | 24 | 10245 |
| HSV1-UL19-5647 | - | UCCUCCGCAGUGCCCGCA | 18 | 10246 |
| HSV1-UL19-5648 | - | CUCCUCCGCAGUGCCCGCA | 19 | 10247 |
| HSV1-UL19-2316 | - | CCUCCUCCGCAGUGCCCGCA | 20 | 2553 |
| HSV1-UL19-5649 | - | CCCUCCUCCGCAGUGCCCGCA | 21 | 10248 |
| HSV1-UL19-5650 | - | GCCCUCCUCCGCAGUGCCCGCA | 22 | 10249 |
| HSV1-UL19-5651 | - | CGCCCUCCUCCGCAGUGCCCGCA | 23 | 10250 |
| HSV1-UL19-5652 | - | CCGCCCUCCUCCGCAGUGCCCGCA | 24 | 10251 |
| HSV1-UL19-5653 | - | CGCGGAUGACCGGCCGCA | 18 | 10252 |
| HSV1-UL19-5654 | - | CCGCGGAUGACCGGCCGCA | 19 | 10253 |
| HSV1-UL19-2195 | - | GCCGCGGAUGACCGGCCGCA | 20 | 2481 |
| HSV1-UL19-5655 | - | CGCCGCGGAUGACCGGCCGCA | 21 | 10254 |
| HSV1-UL19-5656 | - | ACGCCGCGGAUGACCGGCCGCA | 22 | 10255 |
| HSV1-UL19-5657 | - | GACGCCGCGGAUGACCGGCCGCA | 23 | 10256 |
| HSV1-UL19-5658 | - | GGACGCCGCGGAUGACCGGCCGCA | 24 | 10257 |
| HSV1-UL19-5659 | - | AGGCCGCCAUACACGGCA | 18 | 10258 |
| HSV1-UL19-5660 | - | CAGGCCGCCAUACACGGCA | 19 | 10259 |
| HSV1-UL19-2161 | - | GCAGGCCGCCAUACACGGCA | 20 | 2459 |
| HSV1-UL19-5661 | - | UGCAGGCCGCCAUACACGGCA | 21 | 10260 |
| HSV1-UL19-5662 | - | CUGCAGGCCGCCAUACACGGCA | 22 | 10261 |
| HSV1-UL19-5663 | - | GCUGCAGGCCGCCAUACACGGCA | 23 | 10262 |
| HSV1-UL19-5664 | - | UGCUGCAGGCCGCCAUACACGGCA | 24 | 10263 |
| HSV1-UL19-5665 | - | ACGGGGCCUACCACCUCA | 18 | 10264 |
| HSV1-UL19-5666 | - | AACGGGGCCUACCACCUCA | 19 | 10265 |
| HSV1-UL19-2301 | - | UAACGGGGCCUACCACCUCA | 20 | 2544 |
| HSV1-UL19-5667 | - | AUAACGGGGCCUACCACCUCA | 21 | 10266 |
| HSV1-UL19-5668 | - | UAUAACGGGGCCUACCACCUCA | 22 | 10267 |
| HSV1-UL19-5669 | - | CUAUAACGGGGCCUACCACCUCA | 23 | 10268 |
| HSV1-UL19-5670 | - | UCUAUAACGGGGCCUACCACCUCA | 24 | 10269 |
| HSV1-UL19-5671 | - | AUGACGCAUCCCCGCUCA | 18 | 10270 |
| HSV1-UL19-5672 | - | UAUGACGCAUCCCCGCUCA | 19 | 10271 |
| HSV1-UL19-579 | - | CUAUGACGCAUCCCCGCUCA | 20 | 965 |
| HSV1-UL19-5673 | - | UCUAUGACGCAUCCCCGCUCA | 21 | 10272 |
| HSV1-UL19-5674 | - | GUCUAUGACGCAUCCCCGCUCA | 22 | 10273 |
| HSV1-UL19-5675 | - | CGUCUAUGACGCAUCCCCGCUCA | 23 | 10274 |
| HSV1-UL19-5676 | - | UCGUCUAUGACGCAUCCCCGCUCA | 24 | 10275 |
| HSV1-UL19-5677 | - | GCCUGUUCAAUCCGGUCA | 18 | 10276 |
| HSV1-UL19-5678 | - | GGCCUGUUCAAUCCGGUCA | 19 | 10277 |
| HSV1-UL19-261 | - | GGGCCUGUUCAAUCCGGUCA | 20 | 647 |
| HSV1-UL19-5679 | - | UGGGCCUGUUCAAUCCGGUCA | 21 | 10278 |
| HSV1-UL19-5680 | - | CUGGGCCUGUUCAAUCCGGUCA | 22 | 10279 |
| HSV1-UL19-5681 | - | CCUGGGCCUGUUCAAUCCGGUCA | 23 | 10280 |
| HSV1-UL19-5682 | - | CCCUGGGCCUGUUCAAUCCGGUCA | 24 | 10281 |
| HSV1-UL19-5683 | - | GGGUUCACCGUCGUCCGA | 18 | 10282 |
| HSV1-UL19-5684 | - | UGGGUUCACCGUCGUCCGA | 19 | 10283 |
| HSV1-UL19-2241 | - | UUGGGUUCACCGUCGUCCGA | 20 | 2510 |
| HSV1-UL19-5685 | - | UUUGGGUUCACCGUCGUCCGA | 21 | 10284 |
| HSV1-UL19-5686 | - | GUUUGGGUUCACCGUCGUCCGA | 22 | 10285 |
| HSV1-UL19-5687 | - | GGUUUGGGUUCACCGUCGUCCGA | 23 | 10286 |
| HSV1-UL19-5688 | - | GGGUUUGGGUUCACCGUCGUCCGA | 24 | 10287 |
| HSV1-UL19-5689 | - | UGUCUCUGGAACACGCGA | 18 | 10288 |
| HSV1-UL19-5690 | - | CUGUCUCUGGAACACGCGA | 19 | 10289 |
| HSV1-UL19-2109 | - | CCUGUCUCUGGAACACGCGA | 20 | 2435 |
| HSV1-UL19-5691 | - | GCCUGUCUCUGGAACACGCGA | 21 | 10290 |
| HSV1-UL19-5692 | - | CGCCUGUCUCUGGAACACGCGA | 22 | 10291 |
| HSV1-UL19-5693 | - | GCGCCUGUCUCUGGAACACGCGA | 23 | 10292 |
| HSV1-UL19-5694 | - | UGCGCCUGUCUCUGGAACACGCGA | 24 | 10293 |
| HSV1-UL19-5695 | - | CAACAUGCGCAUAUUCGA | 18 | 10294 |
| HSV1-UL19-5696 | - | CCAACAUGCGCAUAUUCGA | 19 | 10295 |
| HSV1-UL19-439 | - | ACCAACAUGCGCAUAUUCGA | 20 | 825 |
| HSV1-UL19-5697 | - | CACCAACAUGCGCAUAUUCGA | 21 | 10296 |
| HSV1-UL19-5698 | - | CCACCAACAUGCGCAUAUUCGA | 22 | 10297 |
| HSV1-UL19-5699 | - | ACCACCAACAUGCGCAUAUUCGA | 23 | 10298 |
| HSV1-UL19-5700 | - | CACCACCAACAUGCGCAUAUUCGA | 24 | 10299 |
| HSV1-UL19-5701 | - | UUACGCGGGGGACAAGGA | 18 | 10300 |
| HSV1-UL19-5702 | - | UUUACGCGGGGGACAAGGA | 19 | 10301 |
| HSV1-UL19-538 | - | GUUUACGCGGGGGACAAGGA | 20 | 924 |
| HSV1-UL19-5703 | - | CGUUUACGCGGGGGACAAGGA | 21 | 10302 |
| HSV1-UL19-5704 | - | GCGUUUACGCGGGGGACAAGGA | 22 | 10303 |
| HSV1-UL19-5705 | - | GGCGUUUACGCGGGGGACAAGGA | 23 | 10304 |
| HSV1-UL19-5706 | - | GGGCGUUUACGCGGGGGACAAGGA | 24 | 10305 |
| HSV1-UL19-5707 | - | GCCGCACCGGGGGGCGGA | 18 | 10306 |
| HSV1-UL19-5708 | - | GGCCGCACCGGGGGGCGGA | 19 | 10307 |
| HSV1-UL19-2200 | - | CGGCCGCACCGGGGGGCGGA | 20 | 2483 |
| HSV1-UL19-5709 | - | CCGGCCGCACCGGGGGGCGGA | 21 | 10308 |
| HSV1-UL19-5710 | - | ACCGGCCGCACCGGGGGGCGGA | 22 | 10309 |
| HSV1-UL19-5711 | - | GACCGGCCGCACCGGGGGGCGGA | 23 | 10310 |
| HSV1-UL19-5712 | - | UGACCGGCCGCACCGGGGGGCGGA | 24 | 10311 |
| HSV1-UL19-5713 | - | ACGGCGAGAUGGUCCUGA | 18 | 10312 |
| HSV1-UL19-5714 | - | UACGGCGAGAUGGUCCUGA | 19 | 10313 |
| HSV1-UL19-2067 | - | GUACGGCGAGAUGGUCCUGA | 20 | 2407 |
| HSV1-UL19-5715 | - | CGUACGGCGAGAUGGUCCUGA | 21 | 10314 |
| HSV1-UL19-5716 | - | ACGUACGGCGAGAUGGUCCUGA | 22 | 10315 |
| HSV1-UL19-5717 | - | GACGUACGGCGAGAUGGUCCUGA | 23 | 10316 |
| HSV1-UL19-5718 | - | UGACGUACGGCGAGAUGGUCCUGA | 24 | 10317 |
| HSV1-UL19-5719 | - | GACAGGACCGCUUUGUGA | 18 | 10318 |
| HSV1-UL19-5720 | - | CGACAGGACCGCUUUGUGA | 19 | 10319 |
| HSV1-UL19-2242 | - | CCGACAGGACCGCUUUGUGA | 20 | 2511 |
| HSV1-UL19-5721 | - | UCCGACAGGACCGCUUUGUGA | 21 | 10320 |
| HSV1-UL19-5722 | - | GUCCGACAGGACCGCUUUGUGA | 22 | 10321 |
| HSV1-UL19-5723 | - | CGUCCGACAGGACCGCUUUGUGA | 23 | 10322 |
| HSV1-UL19-5724 | - | UCGUCCGACAGGACCGCUUUGUGA | 24 | 10323 |
| HSV1-UL19-5725 | - | ACGGGGACCUGCUCUAUA | 18 | 10324 |
| HSV1-UL19-5726 | - | UACGGGGACCUGCUCUAUA | 19 | 10325 |
| HSV1-UL19-2299 | - | GUACGGGGACCUGCUCUAUA | 20 | 2543 |
| HSV1-UL19-5727 | - | CGUACGGGGACCUGCUCUAUA | 21 | 10326 |
| HSV1-UL19-5728 | - | UCGUACGGGGACCUGCUCUAUA | 22 | 10327 |
| HSV1-UL19-5729 | - | UUCGUACGGGGACCUGCUCUAUA | 23 | 10328 |
| HSV1-UL19-5730 | - | UUUCGUACGGGGACCUGCUCUAUA | 24 | 10329 |
| HSV1-UL19-5731 | - | CCAGUGCAUCACCAGCUA | 18 | 10330 |
| HSV1-UL19-5732 | - | UCCAGUGCAUCACCAGCUA | 19 | 10331 |
| HSV1-UL19-2162 | - | GUCCAGUGCAUCACCAGCUA | 20 | 2460 |
| HSV1-UL19-5733 | - | GGUCCAGUGCAUCACCAGCUA | 21 | 10332 |
| HSV1-UL19-5734 | - | UGGUCCAGUGCAUCACCAGCUA | 22 | 10333 |
| HSV1-UL19-5735 | - | GUGGUCCAGUGCAUCACCAGCUA | 23 | 10334 |
| HSV1-UL19-5736 | - | CGUGGUCCAGUGCAUCACCAGCUA | 24 | 10335 |
| HSV1-UL19-5737 | - | CGAAGCCGCCAAUCCGUA | 18 | 10336 |
| HSV1-UL19-5738 | - | UCGAAGCCGCCAAUCCGUA | 19 | 10337 |
| HSV1-UL19-283 | - | GUCGAAGCCGCCAAUCCGUA | 20 | 669 |
| HSV1-UL19-5739 | - | CGUCGAAGCCGCCAAUCCGUA | 21 | 10338 |
| HSV1-UL19-5740 | - | CCGUCGAAGCCGCCAAUCCGUA | 22 | 10339 |
| HSV1-UL19-5741 | - | CCCGUCGAAGCCGCCAAUCCGUA | 23 | 10340 |
| HSV1-UL19-5742 | - | CCCCGUCGAAGCCGCCAAUCCGUA | 24 | 10341 |
| HSV1-UL19-5743 | - | GUCGCAGCGAUUUUCGUA | 18 | 10342 |
| HSV1-UL19-5744 | - | CGUCGCAGCGAUUUUCGUA | 19 | 10343 |
| HSV1-UL19-548 | - | GCGUCGCAGCGAUUUUCGUA | 20 | 934 |
| HSV1-UL19-5745 | - | GGCGUCGCAGCGAUUUUCGUA | 21 | 10344 |
| HSV1-UL19-5746 | - | GGGCGUCGCAGCGAUUUUCGUA | 22 | 10345 |
| HSV1-UL19-5747 | - | UGGGCGUCGCAGCGAUUUUCGUA | 23 | 10346 |
| HSV1-UL19-5748 | - | GUGGGCGUCGCAGCGAUUUUCGUA | 24 | 10347 |
| HSV1-UL19-5749 | - | CACAACGGGCGCGUGGUA | 18 | 10348 |
| HSV1-UL19-5750 | - | UCACAACGGGCGCGUGGUA | 19 | 10349 |
| HSV1-UL19-2214 | - | UUCACAACGGGCGCGUGGUA | 20 | 2492 |
| HSV1-UL19-5751 | - | UUUCACAACGGGCGCGUGGUA | 21 | 10350 |
| HSV1-UL19-5752 | - | GUUUCACAACGGGCGCGUGGUA | 22 | 10351 |
| HSV1-UL19-5753 | - | UGUUUCACAACGGGCGCGUGGUA | 23 | 10352 |
| HSV1-UL19-5754 | - | AUGUUUCACAACGGGCGCGUGGUA | 24 | 10353 |
| HSV1-UL19-5755 | - | GGAGUGCAUGGCCGUGUA | 18 | 10354 |
| HSV1-UL19-5756 | - | AGGAGUGCAUGGCCGUGUA | 19 | 10355 |
| HSV1-UL19-2173 | - | GAGGAGUGCAUGGCCGUGUA | 20 | 2465 |
| HSV1-UL19-5757 | - | CGAGGAGUGCAUGGCCGUGUA | 21 | 10356 |
| HSV1-UL19-5758 | - | CCGAGGAGUGCAUGGCCGUGUA | 22 | 10357 |
| HSV1-UL19-5759 | - | CCCGAGGAGUGCAUGGCCGUGUA | 23 | 10358 |
| HSV1-UL19-5760 | - | UCCCGAGGAGUGCAUGGCCGUGUA | 24 | 10359 |
| HSV1-UL19-5761 | - | CUCCGCAGUGCCCGCAAC | 18 | 10360 |
| HSV1-UL19-5762 | - | CCUCCGCAGUGCCCGCAAC | 19 | 10361 |
| HSV1-UL19-575 | - | UCCUCCGCAGUGCCCGCAAC | 20 | 961 |
| HSV1-UL19-5763 | - | CUCCUCCGCAGUGCCCGCAAC | 21 | 10362 |
| HSV1-UL19-5764 | - | CCUCCUCCGCAGUGCCCGCAAC | 22 | 10363 |
| HSV1-UL19-5765 | - | CCCUCCUCCGCAGUGCCCGCAAC | 23 | 10364 |
| HSV1-UL19-5766 | - | GCCCUCCUCCGCAGUGCCCGCAAC | 24 | 10365 |
| HSV1-UL19-5767 | - | ACCCAGCCGCGCGCGAAC | 18 | 10366 |
| HSV1-UL19-5768 | - | CACCCAGCCGCGCGCGAAC | 19 | 10367 |
| HSV1-UL19-2256 | - | UCACCCAGCCGCGCGCGAAC | 20 | 2519 |
| HSV1-UL19-5769 | - | CUCACCCAGCCGCGCGCGAAC | 21 | 10368 |
| HSV1-UL19-5770 | - | GCUCACCCAGCCGCGCGCGAAC | 22 | 10369 |
| HSV1-UL19-5771 | - | CGCUCACCCAGCCGCGCGCGAAC | 23 | 10370 |
| HSV1-UL19-5772 | - | ACGCUCACCCAGCCGCGCGCGAAC | 24 | 10371 |
| HSV1-UL19-5773 | - | GCGGAUGACCGGCCGCAC | 18 | 10372 |
| HSV1-UL19-5774 | - | CGCGGAUGACCGGCCGCAC | 19 | 10373 |
| HSV1-UL19-400 | - | CCGCGGAUGACCGGCCGCAC | 20 | 786 |
| HSV1-UL19-5775 | - | GCCGCGGAUGACCGGCCGCAC | 21 | 10374 |
| HSV1-UL19-5776 | - | CGCCGCGGAUGACCGGCCGCAC | 22 | 10375 |
| HSV1-UL19-5777 | - | ACGCCGCGGAUGACCGGCCGCAC | 23 | 10376 |
| HSV1-UL19-5778 | - | GACGCCGCGGAUGACCGGCCGCAC | 24 | 10377 |
| HSV1-UL19-5779 | - | GUGCGAAGUCUGGACGAC | 18 | 10378 |
| HSV1-UL19-5780 | - | CGUGCGAAGUCUGGACGAC | 19 | 10379 |
| HSV1-UL19-2072 | - | CCGUGCGAAGUCUGGACGAC | 20 | 2411 |
| HSV1-UL19-5781 | - | GCCGUGCGAAGUCUGGACGAC | 21 | 10380 |
| HSV1-UL19-5782 | - | GGCCGUGCGAAGUCUGGACGAC | 22 | 10381 |
| HSV1-UL19-5783 | - | AGGCCGUGCGAAGUCUGGACGAC | 23 | 10382 |
| HSV1-UL19-5784 | - | AAGGCCGUGCGAAGUCUGGACGAC | 24 | 10383 |
| HSV1-UL19-5785 | - | CGCAACCCCGUCACCGAC | 18 | 10384 |
| HSV1-UL19-5786 | - | CCGCAACCCCGUCACCGAC | 19 | 10385 |
| HSV1-UL19-2259 | - | UCCGCAACCCCGUCACCGAC | 20 | 2522 |
| HSV1-UL19-5787 | - | GUCCGCAACCCCGUCACCGAC | 21 | 10386 |
| HSV1-UL19-5788 | - | CGUCCGCAACCCCGUCACCGAC | 22 | 10387 |
| HSV1-UL19-5789 | - | CCGUCCGCAACCCCGUCACCGAC | 23 | 10388 |
| HSV1-UL19-5790 | - | ACCGUCCGCAACCCCGUCACCGAC | 24 | 10389 |
| HSV1-UL19-5791 | - | UGCUCGGCGGCGCCCGAC | 18 | 10390 |
| HSV1-UL19-5792 | - | CUGCUCGGCGGCGCCCGAC | 19 | 10391 |
| HSV1-UL19-2217 | - | UCUGCUCGGCGGCGCCCGAC | 20 | 2495 |
| HSV1-UL19-5793 | - | CUCUGCUCGGCGGCGCCCGAC | 21 | 10392 |
| HSV1-UL19-5794 | - | GCUCUGCUCGGCGGCGCCCGAC | 22 | 10393 |
| HSV1-UL19-5795 | - | UGCUCUGCUCGGCGGCGCCCGAC | 23 | 10394 |
| HSV1-UL19-5796 | - | CUGCUCUGCUCGGCGGCGCCCGAC | 24 | 10395 |
| HSV1-UL19-5797 | - | UUUGUGGGGGUGGCCGAC | 18 | 10396 |
| HSV1-UL19-5798 | - | CUUUGUGGGGGUGGCCGAC | 19 | 10397 |
| HSV1-UL19-2133 | - | UCUUUGUGGGGGUGGCCGAC | 20 | 2449 |
| HSV1-UL19-5799 | - | UUCUUUGUGGGGGUGGCCGAC | 21 | 10398 |
| HSV1-UL19-5800 | - | CUUCUUUGUGGGGGUGGCCGAC | 22 | 10399 |
| HSV1-UL19-5801 | - | ACUUCUUUGUGGGGGUGGCCGAC | 23 | 10400 |
| HSV1-UL19-5802 | - | GACUUCUUUGUGGGGGUGGCCGAC | 24 | 10401 |
| HSV1-UL19-5803 | - | CAGGAGGAGCAGCUCGAC | 18 | 10402 |
| HSV1-UL19-5804 | - | GCAGGAGGAGCAGCUCGAC | 19 | 10403 |
| HSV1-UL19-2079 | - | UGCAGGAGGAGCAGCUCGAC | 20 | 2415 |
| HSV1-UL19-5805 | - | AUGCAGGAGGAGCAGCUCGAC | 21 | 10404 |
| HSV1-UL19-5806 | - | GAUGCAGGAGGAGCAGCUCGAC | 22 | 10405 |
| HSV1-UL19-5807 | - | AGAUGCAGGAGGAGCAGCUCGAC | 23 | 10406 |
| HSV1-UL19-5808 | - | GAGAUGCAGGAGGAGCAGCUCGAC | 24 | 10407 |
| HSV1-UL19-5809 | - | GGCGUUUACGCGGGGGAC | 18 | 10408 |
| HSV1-UL19-5810 | - | GGGCGUUUACGCGGGGGAC | 19 | 10409 |
| HSV1-UL19-2287 | - | GGGGCGUUUACGCGGGGGAC | 20 | 2537 |
| HSV1-UL19-5811 | - | GGGGGCGUUUACGCGGGGGAC | 21 | 10410 |
| HSV1-UL19-5812 | - | CGGGGGCGUUUACGCGGGGGAC | 22 | 10411 |
| HSV1-UL19-5813 | - | CCGGGGGCGUUUACGCGGGGGAC | 23 | 10412 |
| HSV1-UL19-5814 | - | GCCGGGGGCGUUUACGCGGGGGAC | 24 | 10413 |
| HSV1-UL19-5815 | - | CCGCGCGCGAACGUGGAC | 18 | 10414 |
| HSV1-UL19-5816 | - | GCCGCGCGCGAACGUGGAC | 19 | 10415 |
| HSV1-UL19-2257 | - | AGCCGCGCGCGAACGUGGAC | 20 | 2520 |
| HSV1-UL19-5817 | - | CAGCCGCGCGCGAACGUGGAC | 21 | 10416 |
| HSV1-UL19-5818 | - | CCAGCCGCGCGCGAACGUGGAC | 22 | 10417 |
| HSV1-UL19-5819 | - | CCCAGCCGCGCGCGAACGUGGAC | 23 | 10418 |
| HSV1-UL19-5820 | - | ACCCAGCCGCGCGCGAACGUGGAC | 24 | 10419 |
| HSV1-UL19-5821 | - | UUGCCGAUGCAACGAUAC | 18 | 10420 |
| HSV1-UL19-5822 | - | GUUGCCGAUGCAACGAUAC | 19 | 10421 |
| HSV1-UL19-2043 | - | UGUUGCCGAUGCAACGAUAC | 20 | 2387 |
| HSV1-UL19-5823 | - | UUGUUGCCGAUGCAACGAUAC | 21 | 10422 |
| HSV1-UL19-5824 | - | GUUGUUGCCGAUGCAACGAUAC | 22 | 10423 |
| HSV1-UL19-5825 | - | UGUUGUUGCCGAUGCAACGAUAC | 23 | 10424 |
| HSV1-UL19-5826 | - | UUGUUGUUGCCGAUGCAACGAUAC | 24 | 10425 |
| HSV1-UL19-5827 | - | CAGUGCAUCACCAGCUAC | 18 | 10426 |
| HSV1-UL19-5828 | - | CCAGUGCAUCACCAGCUAC | 19 | 10427 |
| HSV1-UL19-354 | - | UCCAGUGCAUCACCAGCUAC | 20 | 740 |
| HSV1-UL19-5829 | - | GUCCAGUGCAUCACCAGCUAC | 21 | 10428 |
| HSV1-UL19-5830 | - | GGUCCAGUGCAUCACCAGCUAC | 22 | 10429 |
| HSV1-UL19-5831 | - | UGGUCCAGUGCAUCACCAGCUAC | 23 | 10430 |
| HSV1-UL19-5832 | - | GUGGUCCAGUGCAUCACCAGCUAC | 24 | 10431 |
| HSV1-UL19-5833 | - | UCGCAGCGAUUUUCGUAC | 18 | 10432 |
| HSV1-UL19-5834 | - | GUCGCAGCGAUUUUCGUAC | 19 | 10433 |
| HSV1-UL19-549 | - | CGUCGCAGCGAUUUUCGUAC | 20 | 935 |
| HSV1-UL19-5835 | - | GCGUCGCAGCGAUUUUCGUAC | 21 | 10434 |
| HSV1-UL19-5836 | - | GGCGUCGCAGCGAUUUUCGUAC | 22 | 10435 |
| HSV1-UL19-5837 | - | GGGCGUCGCAGCGAUUUUCGUAC | 23 | 10436 |
| HSV1-UL19-5838 | - | UGGGCGUCGCAGCGAUUUUCGUAC | 24 | 10437 |
| HSV1-UL19-5839 | - | GAGUGCAUGGCCGUGUAC | 18 | 10438 |
| HSV1-UL19-5840 | - | GGAGUGCAUGGCCGUGUAC | 19 | 10439 |
| HSV1-UL19-364 | - | AGGAGUGCAUGGCCGUGUAC | 20 | 750 |
| HSV1-UL19-5841 | - | GAGGAGUGCAUGGCCGUGUAC | 21 | 10440 |
| HSV1-UL19-5842 | - | CGAGGAGUGCAUGGCCGUGUAC | 22 | 10441 |
| HSV1-UL19-5843 | - | CCGAGGAGUGCAUGGCCGUGUAC | 23 | 10442 |
| HSV1-UL19-5844 | - | CCCGAGGAGUGCAUGGCCGUGUAC | 24 | 10443 |
| HSV1-UL19-5845 | - | GCCGCCGGGGGCGUUUAC | 18 | 10444 |
| HSV1-UL19-5846 | - | CGCCGCCGGGGGCGUUUAC | 19 | 10445 |
| HSV1-UL19-2283 | - | GCGCCGCCGGGGGCGUUUAC | 20 | 2536 |
| HSV1-UL19-5847 | - | CGCGCCGCCGGGGGCGUUUAC | 21 | 10446 |
| HSV1-UL19-5848 | - | ACGCGCCGCCGGGGGCGUUUAC | 22 | 10447 |
| HSV1-UL19-5849 | - | GACGCGCCGCCGGGGGCGUUUAC | 23 | 10448 |
| HSV1-UL19-5850 | - | GGACGCGCCGCCGGGGGCGUUUAC | 24 | 10449 |
| HSV1-UL19-5851 | - | CUUCCCCAAAACUUUUAC | 18 | 10450 |
| HSV1-UL19-5852 | - | CCUUCCCCAAAACUUUUAC | 19 | 10451 |
| HSV1-UL19-2260 | - | ACCUUCCCCAAAACUUUUAC | 20 | 2523 |
| HSV1-UL19-5853 | - | AACCUUCCCCAAAACUUUUAC | 21 | 10452 |
| HSV1-UL19-5854 | - | CAACCUUCCCCAAAACUUUUAC | 22 | 10453 |
| HSV1-UL19-5855 | - | GCAACCUUCCCCAAAACUUUUAC | 23 | 10454 |
| HSV1-UL19-5856 | - | GGCAACCUUCCCCAAAACUUUUAC | 24 | 10455 |
| HSV1-UL19-5857 | - | CGGAUGACCGGCCGCACC | 18 | 10456 |
| HSV1-UL19-5858 | - | GCGGAUGACCGGCCGCACC | 19 | 10457 |
| HSV1-UL19-401 | - | CGCGGAUGACCGGCCGCACC | 20 | 787 |
| HSV1-UL19-5859 | - | CCGCGGAUGACCGGCCGCACC | 21 | 10458 |
| HSV1-UL19-5860 | - | GCCGCGGAUGACCGGCCGCACC | 22 | 10459 |
| HSV1-UL19-5861 | - | CGCCGCGGAUGACCGGCCGCACC | 23 | 10460 |
| HSV1-UL19-5862 | - | ACGCCGCGGAUGACCGGCCGCACC | 24 | 10461 |
| HSV1-UL19-5863 | - | CGGGGCCGCUGCUGCACC | 18 | 10462 |
| HSV1-UL19-5864 | - | GCGGGGCCGCUGCUGCACC | 19 | 10463 |
| HSV1-UL19-2203 | - | CGCGGGGCCGCUGCUGCACC | 20 | 2485 |
| HSV1-UL19-5865 | - | UCGCGGGGCCGCUGCUGCACC | 21 | 10464 |
| HSV1-UL19-5866 | - | CUCGCGGGGCCGCUGCUGCACC | 22 | 10465 |
| HSV1-UL19-5867 | - | UCUCGCGGGGCCGCUGCUGCACC | 23 | 10466 |
| HSV1-UL19-5868 | - | UUCUCGCGGGGCCGCUGCUGCACC | 24 | 10467 |
| HSV1-UL19-5869 | - | GCAUGUAACGUGGCGACC | 18 | 10468 |
| HSV1-UL19-5870 | - | GGCAUGUAACGUGGCGACC | 19 | 10469 |
| HSV1-UL19-2191 | - | CGGCAUGUAACGUGGCGACC | 20 | 2477 |
| HSV1-UL19-5871 | - | GCGGCAUGUAACGUGGCGACC | 21 | 10470 |
| HSV1-UL19-5872 | - | GGCGGCAUGUAACGUGGCGACC | 22 | 10471 |
| HSV1-UL19-5873 | - | CGGCGGCAUGUAACGUGGCGACC | 23 | 10472 |
| HSV1-UL19-5874 | - | GCGGCGGCAUGUAACGUGGCGACC | 24 | 10473 |
| HSV1-UL19-5875 | - | GCGGAAGGCCAGAUGACC | 18 | 10474 |
| HSV1-UL19-5876 | - | CGCGGAAGGCCAGAUGACC | 19 | 10475 |
| HSV1-UL19-2127 | - | UCGCGGAAGGCCAGAUGACC | 20 | 2446 |
| HSV1-UL19-5877 | - | GUCGCGGAAGGCCAGAUGACC | 21 | 10476 |
| HSV1-UL19-5878 | - | GGUCGCGGAAGGCCAGAUGACC | 22 | 10477 |
| HSV1-UL19-5879 | - | GGGUCGCGGAAGGCCAGAUGACC | 23 | 10478 |
| HSV1-UL19-5880 | - | UGGGUCGCGGAAGGCCAGAUGACC | 24 | 10479 |
| HSV1-UL19-5881 | - | CGUACGUCGUGACCUACC | 18 | 10480 |
| HSV1-UL19-5882 | - | UCGUACGUCGUGACCUACC | 19 | 10481 |
| HSV1-UL19-2165 | - | CUCGUACGUCGUGACCUACC | 20 | 2462 |
| HSV1-UL19-5883 | - | UCUCGUACGUCGUGACCUACC | 21 | 10482 |
| HSV1-UL19-5884 | - | GUCUCGUACGUCGUGACCUACC | 22 | 10483 |
| HSV1-UL19-5885 | - | GGUCUCGUACGUCGUGACCUACC | 23 | 10484 |
| HSV1-UL19-5886 | - | UGGUCUCGUACGUCGUGACCUACC | 24 | 10485 |
| HSV1-UL19-5887 | - | CGCGGCCACGGCCAACCC | 18 | 10486 |
| HSV1-UL19-5888 | - | UCGCGGCCACGGCCAACCC | 19 | 10487 |
| HSV1-UL19-2295 | - | UUCGCGGCCACGGCCAACCC | 20 | 2541 |
| HSV1-UL19-5889 | - | CUUCGCGGCCACGGCCAACCC | 21 | 10488 |
| HSV1-UL19-5890 | - | CCUUCGCGGCCACGGCCAACCC | 22 | 10489 |
| HSV1-UL19-5891 | - | GCCUUCGCGGCCACGGCCAACCC | 23 | 10490 |
| HSV1-UL19-5892 | - | GGCCUUCGCGGCCACGGCCAACCC | 24 | 10491 |
| HSV1-UL19-5893 | - | CCGCCGGCCCUGCAACCC | 18 | 10492 |
| HSV1-UL19-5894 | - | UCCGCCGGCCCUGCAACCC | 19 | 10493 |
| HSV1-UL19-2277 | - | UUCCGCCGGCCCUGCAACCC | 20 | 2534 |
| HSV1-UL19-5895 | - | CUUCCGCCGGCCCUGCAACCC | 21 | 10494 |
| HSV1-UL19-5896 | - | ACUUCCGCCGGCCCUGCAACCC | 22 | 10495 |
| HSV1-UL19-5897 | - | UACUUCCGCCGGCCCUGCAACCC | 23 | 10496 |
| HSV1-UL19-5898 | - | CUACUUCCGCCGGCCCUGCAACCC | 24 | 10497 |
| HSV1-UL19-5899 | - | CGGAAGGCCAGAUGACCC | 18 | 10498 |
| HSV1-UL19-5900 | - | GCGGAAGGCCAGAUGACCC | 19 | 10499 |
| HSV1-UL19-303 | - | CGCGGAAGGCCAGAUGACCC | 20 | 689 |
| HSV1-UL19-5901 | - | UCGCGGAAGGCCAGAUGACCC | 21 | 10500 |
| HSV1-UL19-5902 | - | GUCGCGGAAGGCCAGAUGACCC | 22 | 10501 |
| HSV1-UL19-5903 | - | GGUCGCGGAAGGCCAGAUGACCC | 23 | 10502 |
| HSV1-UL19-5904 | - | GGGUCGCGGAAGGCCAGAUGACCC | 24 | 10503 |
| HSV1-UL19-5905 | - | CGCCGGCCCUGCAACCCC | 18 | 10504 |
| HSV1-UL19-5906 | - | CCGCCGGCCCUGCAACCCC | 19 | 10505 |
| HSV1-UL19-525 | - | UCCGCCGGCCCUGCAACCCC | 20 | 911 |
| HSV1-UL19-5907 | - | UUCCGCCGGCCCUGCAACCCC | 21 | 10506 |
| HSV1-UL19-5908 | - | CUUCCGCCGGCCCUGCAACCCC | 22 | 10507 |
| HSV1-UL19-5909 | - | ACUUCCGCCGGCCCUGCAACCCC | 23 | 10508 |
| HSV1-UL19-5910 | - | UACUUCCGCCGGCCCUGCAACCCC | 24 | 10509 |
| HSV1-UL19-5911 | - | GCCGGCCCUGCAACCCCC | 18 | 10510 |
| HSV1-UL19-5912 | - | CGCCGGCCCUGCAACCCCC | 19 | 10511 |
| HSV1-UL19-526 | - | CCGCCGGCCCUGCAACCCCC | 20 | 912 |
| HSV1-UL19-5913 | - | UCCGCCGGCCCUGCAACCCCC | 21 | 10512 |
| HSV1-UL19-5914 | - | UUCCGCCGGCCCUGCAACCCCC | 22 | 10513 |
| HSV1-UL19-5915 | - | CUUCCGCCGGCCCUGCAACCCCC | 23 | 10514 |
| HSV1-UL19-5916 | - | ACUUCCGCCGGCCCUGCAACCCCC | 24 | 10515 |
| HSV1-UL19-5917 | - | GUACAGUUUAAGCGCCCC | 18 | 10516 |
| HSV1-UL19-5918 | - | CGUACAGUUUAAGCGCCCC | 19 | 10517 |
| HSV1-UL19-2308 | - | ACGUACAGUUUAAGCGCCCC | 20 | 2548 |
| HSV1-UL19-5919 | - | GACGUACAGUUUAAGCGCCCC | 21 | 10518 |
| HSV1-UL19-5920 | - | CGACGUACAGUUUAAGCGCCCC | 22 | 10519 |
| HSV1-UL19-5921 | - | GCGACGUACAGUUUAAGCGCCCC | 23 | 10520 |
| HSV1-UL19-5922 | - | AGCGACGUACAGUUUAAGCGCCCC | 24 | 10521 |
| HSV1-UL19-5923 | - | UGGUCUUUCUGGAGGCCC | 18 | 10522 |
| HSV1-UL19-5924 | - | CUGGUCUUUCUGGAGGCCC | 19 | 10523 |
| HSV1-UL19-253 | - | GCUGGUCUUUCUGGAGGCCC | 20 | 639 |
| HSV1-UL19-5925 | - | AGCUGGUCUUUCUGGAGGCCC | 21 | 10524 |
| HSV1-UL19-5926 | - | AAGCUGGUCUUUCUGGAGGCCC | 22 | 10525 |
| HSV1-UL19-5927 | - | GAAGCUGGUCUUUCUGGAGGCCC | 23 | 10526 |
| HSV1-UL19-5928 | - | AGAAGCUGGUCUUUCUGGAGGCCC | 24 | 10527 |
| HSV1-UL19-5929 | - | CGUUCCCCCGGCCGGCCC | 18 | 10528 |
| HSV1-UL19-5930 | - | ACGUUCCCCCGGCCGGCCC | 19 | 10529 |
| HSV1-UL19-321 | - | GACGUUCCCCCGGCCGGCCC | 20 | 707 |
| HSV1-UL19-5931 | - | GGACGUUCCCCCGGCCGGCCC | 21 | 10530 |
| HSV1-UL19-5932 | - | GGGACGUUCCCCCGGCCGGCCC | 22 | 10531 |
| HSV1-UL19-5933 | - | GGGGACGUUCCCCCGGCCGGCCC | 23 | 10532 |
| HSV1-UL19-5934 | - | GGGGGACGUUCCCCCGGCCGGCCC | 24 | 10533 |
| HSV1-UL19-5935 | - | GGGGGCAGCGCCUGGCCC | 18 | 10534 |
| HSV1-UL19-5936 | - | UGGGGGCAGCGCCUGGCCC | 19 | 10535 |
| HSV1-UL19-2122 | - | CUGGGGGCAGCGCCUGGCCC | 20 | 2442 |
| HSV1-UL19-5937 | - | CCUGGGGGCAGCGCCUGGCCC | 21 | 10536 |
| HSV1-UL19-5938 | - | GCCUGGGGGCAGCGCCUGGCCC | 22 | 10537 |
| HSV1-UL19-5939 | - | CGCCUGGGGGCAGCGCCUGGCCC | 23 | 10538 |
| HSV1-UL19-5940 | - | ACGCCUGGGGGCAGCGCCUGGCCC | 24 | 10539 |
| HSV1-UL19-5941 | - | CUGACGUUCGUCCUGCCC | 18 | 10540 |
| HSV1-UL19-5942 | - | CCUGACGUUCGUCCUGCCC | 19 | 10541 |
| HSV1-UL19-2094 | - | ACCUGACGUUCGUCCUGCCC | 20 | 2428 |
| HSV1-UL19-5943 | - | GACCUGACGUUCGUCCUGCCC | 21 | 10542 |
| HSV1-UL19-5944 | - | GGACCUGACGUUCGUCCUGCCC | 22 | 10543 |
| HSV1-UL19-5945 | - | UGGACCUGACGUUCGUCCUGCCC | 23 | 10544 |
| HSV1-UL19-5946 | - | AUGGACCUGACGUUCGUCCUGCCC | 24 | 10545 |
| HSV1-UL19-5947 | - | CUCGGGGGAGACCUUCCC | 18 | 10546 |
| HSV1-UL19-5948 | - | CCUCGGGGGAGACCUUCCC | 19 | 10547 |
| HSV1-UL19-2171 | - | ACCUCGGGGGAGACCUUCCC | 20 | 2464 |
| HSV1-UL19-5949 | - | UACCUCGGGGGAGACCUUCCC | 21 | 10548 |
| HSV1-UL19-5950 | - | CUACCUCGGGGGAGACCUUCCC | 22 | 10549 |
| HSV1-UL19-5951 | - | CCUACCUCGGGGGAGACCUUCCC | 23 | 10550 |
| HSV1-UL19-5952 | - | ACCUACCUCGGGGGAGACCUUCCC | 24 | 10551 |
| HSV1-UL19-5953 | - | AACACCCAGGCCCGAGCC | 18 | 10552 |
| HSV1-UL19-5954 | - | CAACACCCAGGCCCGAGCC | 19 | 10553 |
| HSV1-UL19-2193 | - | ACAACACCCAGGCCCGAGCC | 20 | 2479 |
| HSV1-UL19-5955 | - | CACAACACCCAGGCCCGAGCC | 21 | 10554 |
| HSV1-UL19-5956 | - | GCACAACACCCAGGCCCGAGCC | 22 | 10555 |
| HSV1-UL19-5957 | - | UGCACAACACCCAGGCCCGAGCC | 23 | 10556 |
| HSV1-UL19-5958 | - | CUGCACAACACCCAGGCCCGAGCC | 24 | 10557 |
| HSV1-UL19-5959 | - | CAGCUGUUUUUGAACGCC | 18 | 10558 |
| HSV1-UL19-5960 | - | GCAGCUGUUUUUGAACGCC | 19 | 10559 |
| HSV1-UL19-290 | - | AGCAGCUGUUUUUGAACGCC | 20 | 676 |
| HSV1-UL19-5961 | - | CAGCAGCUGUUUUUGAACGCC | 21 | 10560 |
| HSV1-UL19-5962 | - | GCAGCAGCUGUUUUUGAACGCC | 22 | 10561 |
| HSV1-UL19-5963 | - | UGCAGCAGCUGUUUUUGAACGCC | 23 | 10562 |
| HSV1-UL19-5964 | - | AUGCAGCAGCUGUUUUUGAACGCC | 24 | 10563 |
| HSV1-UL19-5965 | - | CGGUUUGCCGCGCACGCC | 18 | 10564 |
| HSV1-UL19-5966 | - | ACGGUUUGCCGCGCACGCC | 19 | 10565 |
| HSV1-UL19-264 | - | AACGGUUUGCCGCGCACGCC | 20 | 650 |
| HSV1-UL19-5967 | - | GAACGGUUUGCCGCGCACGCC | 21 | 10566 |
| HSV1-UL19-5968 | - | GGAACGGUUUGCCGCGCACGCC | 22 | 10567 |
| HSV1-UL19-5969 | - | UGGAACGGUUUGCCGCGCACGCC | 23 | 10568 |
| HSV1-UL19-5970 | - | AUGGAACGGUUUGCCGCGCACGCC | 24 | 10569 |
| HSV1-UL19-5971 | - | GCGGCGUUCCGGGACGCC | 18 | 10570 |
| HSV1-UL19-5972 | - | GGCGGCGUUCCGGGACGCC | 19 | 10571 |
| HSV1-UL19-333 | - | CGGCGGCGUUCCGGGACGCC | 20 | 719 |
| HSV1-UL19-5973 | - | CCGGCGGCGUUCCGGGACGCC | 21 | 10572 |
| HSV1-UL19-5974 | - | UCCGGCGGCGUUCCGGGACGCC | 22 | 10573 |
| HSV1-UL19-5975 | - | GUCCGGCGGCGUUCCGGGACGCC | 23 | 10574 |
| HSV1-UL19-5976 | - | UGUCCGGCGGCGUUCCGGGACGCC | 24 | 10575 |
| HSV1-UL19-5977 | - | UCGUGGCGGGAAACCGCC | 18 | 10576 |
| HSV1-UL19-5978 | - | GUCGUGGCGGGAAACCGCC | 19 | 10577 |
| HSV1-UL19-511 | - | GGUCGUGGCGGGAAACCGCC | 20 | 897 |
| HSV1-UL19-5979 | - | CGGUCGUGGCGGGAAACCGCC | 21 | 10578 |
| HSV1-UL19-5980 | - | GCGGUCGUGGCGGGAAACCGCC | 22 | 10579 |
| HSV1-UL19-5981 | - | CGCGGUCGUGGCGGGAAACCGCC | 23 | 10580 |
| HSV1-UL19-5982 | - | ACGCGGUCGUGGCGGGAAACCGCC | 24 | 10581 |
| HSV1-UL19-5983 | - | GCAAGGCGGGCCACCGCC | 18 | 10582 |
| HSV1-UL19-5984 | - | GGCAAGGCGGGCCACCGCC | 19 | 10583 |
| HSV1-UL19-212 | - | GGGCAAGGCGGGCCACCGCC | 20 | 598 |
| HSV1-UL19-5985 | - | UGGGCAAGGCGGGCCACCGCC | 21 | 10584 |
| HSV1-UL19-5986 | - | CUGGGCAAGGCGGGCCACCGCC | 22 | 10585 |
| HSV1-UL19-5987 | - | CCUGGGCAAGGCGGGCCACCGCC | 23 | 10586 |
| HSV1-UL19-5988 | - | UCCUGGGCAAGGCGGGCCACCGCC | 24 | 10587 |
| HSV1-UL19-5989 | - | CCCCGGGGACGCGCCGCC | 18 | 10588 |
| HSV1-UL19-5990 | - | CCCCCGGGGACGCGCCGCC | 19 | 10589 |
| HSV1-UL19-529 | - | ACCCCCGGGGACGCGCCGCC | 20 | 915 |
| HSV1-UL19-5991 | - | AACCCCCGGGGACGCGCCGCC | 21 | 10590 |
| HSV1-UL19-5992 | - | CAACCCCCGGGGACGCGCCGCC | 22 | 10591 |
| HSV1-UL19-5993 | - | GCAACCCCCGGGGACGCGCCGCC | 23 | 10592 |
| HSV1-UL19-5994 | - | UGCAACCCCCGGGGACGCGCCGCC | 24 | 10593 |
| HSV1-UL19-5995 | - | UACCCCCUGGUGGGCGCC | 18 | 10594 |
| HSV1-UL19-5996 | - | CUACCCCCUGGUGGGCGCC | 19 | 10595 |
| HSV1-UL19-2093 | - | CCUACCCCCUGGUGGGCGCC | 20 | 2427 |
| HSV1-UL19-5997 | - | CCCUACCCCCUGGUGGGCGCC | 21 | 10596 |
| HSV1-UL19-5998 | - | UCCCUACCCCCUGGUGGGCGCC | 22 | 10597 |
| HSV1-UL19-5999 | - | UUCCCUACCCCCUGGUGGGCGCC | 23 | 10598 |
| HSV1-UL19-6000 | - | GUUCCCUACCCCCUGGUGGGCGCC | 24 | 10599 |
| HSV1-UL19-6001 | - | CUGGUCUUUCUGGAGGCC | 18 | 10600 |
| HSV1-UL19-6002 | - | GCUGGUCUUUCUGGAGGCC | 19 | 10601 |
| HSV1-UL19-2089 | - | AGCUGGUCUUUCUGGAGGCC | 20 | 2424 |
| HSV1-UL19-6003 | - | AAGCUGGUCUUUCUGGAGGCC | 21 | 10602 |
| HSV1-UL19-6004 | - | GAAGCUGGUCUUUCUGGAGGCC | 22 | 10603 |
| HSV1-UL19-6005 | - | AGAAGCUGGUCUUUCUGGAGGCC | 23 | 10604 |
| HSV1-UL19-6006 | - | GAGAAGCUGGUCUUUCUGGAGGCC | 24 | 10605 |
| HSV1-UL19-6007 | - | ACUUUACCCUGACCGGCC | 18 | 10606 |
| HSV1-UL19-6008 | - | GACUUUACCCUGACCGGCC | 19 | 10607 |
| HSV1-UL19-372 | - | UGACUUUACCCUGACCGGCC | 20 | 758 |
| HSV1-UL19-6009 | - | AUGACUUUACCCUGACCGGCC | 21 | 10608 |
| HSV1-UL19-6010 | - | GAUGACUUUACCCUGACCGGCC | 22 | 10609 |
| HSV1-UL19-6011 | - | UGAUGACUUUACCCUGACCGGCC | 23 | 10610 |
| HSV1-UL19-6012 | - | UUGAUGACUUUACCCUGACCGGCC | 24 | 10611 |
| HSV1-UL19-6013 | - | ACGUUCCCCCGGCCGGCC | 18 | 10612 |
| HSV1-UL19-6014 | - | GACGUUCCCCCGGCCGGCC | 19 | 10613 |
| HSV1-UL19-320 | - | GGACGUUCCCCCGGCCGGCC | 20 | 706 |
| HSV1-UL19-6015 | - | GGGACGUUCCCCCGGCCGGCC | 21 | 10614 |
| HSV1-UL19-6016 | - | GGGGACGUUCCCCCGGCCGGCC | 22 | 10615 |
| HSV1-UL19-6017 | - | GGGGGACGUUCCCCCGGCCGGCC | 23 | 10616 |
| HSV1-UL19-6018 | - | GGGGGGACGUUCCCCCGGCCGGCC | 24 | 10617 |
| HSV1-UL19-6019 | - | GUCCGCGAGAGCGCGGCC | 18 | 10618 |
| HSV1-UL19-6020 | - | CGUCCGCGAGAGCGCGGCC | 19 | 10619 |
| HSV1-UL19-461 | - | ACGUCCGCGAGAGCGCGGCC | 20 | 847 |
| HSV1-UL19-6021 | - | CACGUCCGCGAGAGCGCGGCC | 21 | 10620 |
| HSV1-UL19-6022 | - | GCACGUCCGCGAGAGCGCGGCC | 22 | 10621 |
| HSV1-UL19-6023 | - | AGCACGUCCGCGAGAGCGCGGCC | 23 | 10622 |
| HSV1-UL19-6024 | - | CAGCACGUCCGCGAGAGCGCGGCC | 24 | 10623 |
| HSV1-UL19-6025 | - | CUGAUGCGGCGCGCGGCC | 18 | 10624 |
| HSV1-UL19-6026 | - | CCUGAUGCGGCGCGCGGCC | 19 | 10625 |
| HSV1-UL19-2185 | - | CCCUGAUGCGGCGCGCGGCC | 20 | 2474 |
| HSV1-UL19-6027 | - | GCCCUGAUGCGGCGCGCGGCC | 21 | 10626 |
| HSV1-UL19-6028 | - | CGCCCUGAUGCGGCGCGCGGCC | 22 | 10627 |
| HSV1-UL19-6029 | - | ACGCCCUGAUGCGGCGCGCGGCC | 23 | 10628 |
| HSV1-UL19-6030 | - | GACGCCCUGAUGCGGCGCGCGGCC | 24 | 10629 |
| HSV1-UL19-6031 | - | UCCGGGACGCCCGGGGCC | 18 | 10630 |
| HSV1-UL19-6032 | - | UUCCGGGACGCCCGGGGCC | 19 | 10631 |
| HSV1-UL19-336 | - | GUUCCGGGACGCCCGGGGCC | 20 | 722 |
| HSV1-UL19-6033 | - | CGUUCCGGGACGCCCGGGGCC | 21 | 10632 |
| HSV1-UL19-6034 | - | GCGUUCCGGGACGCCCGGGGCC | 22 | 10633 |
| HSV1-UL19-6035 | - | GGCGUUCCGGGACGCCCGGGGCC | 23 | 10634 |
| HSV1-UL19-6036 | - | CGGCGUUCCGGGACGCCCGGGGCC | 24 | 10635 |
| HSV1-UL19-6037 | - | AAAACUUUUACCUGGGCC | 18 | 10636 |
| HSV1-UL19-6038 | - | CAAAACUUUUACCUGGGCC | 19 | 10637 |
| HSV1-UL19-2261 | - | CCAAAACUUUUACCUGGGCC | 20 | 2524 |
| HSV1-UL19-6039 | - | CCCAAAACUUUUACCUGGGCC | 21 | 10638 |
| HSV1-UL19-6040 | - | CCCCAAAACUUUUACCUGGGCC | 22 | 10639 |
| HSV1-UL19-6041 | - | UCCCCAAAACUUUUACCUGGGCC | 23 | 10640 |
| HSV1-UL19-6042 | - | UUCCCCAAAACUUUUACCUGGGCC | 24 | 10641 |
| HSV1-UL19-6043 | - | GGCCGACGUGGAGCUGCC | 18 | 10642 |
| HSV1-UL19-6044 | - | UGGCCGACGUGGAGCUGCC | 19 | 10643 |
| HSV1-UL19-312 | - | GUGGCCGACGUGGAGCUGCC | 20 | 698 |
| HSV1-UL19-6045 | - | GGUGGCCGACGUGGAGCUGCC | 21 | 10644 |
| HSV1-UL19-6046 | - | GGGUGGCCGACGUGGAGCUGCC | 22 | 10645 |
| HSV1-UL19-6047 | - | GGGGUGGCCGACGUGGAGCUGCC | 23 | 10646 |
| HSV1-UL19-6048 | - | GGGGGUGGCCGACGUGGAGCUGCC | 24 | 10647 |
| HSV1-UL19-6049 | - | CCGCCAAACAUCGCUGCC | 18 | 10648 |
| HSV1-UL19-6050 | - | GCCGCCAAACAUCGCUGCC | 19 | 10649 |
| HSV1-UL19-559 | - | CGCCGCCAAACAUCGCUGCC | 20 | 945 |
| HSV1-UL19-6051 | - | UCGCCGCCAAACAUCGCUGCC | 21 | 10650 |
| HSV1-UL19-6052 | - | AUCGCCGCCAAACAUCGCUGCC | 22 | 10651 |
| HSV1-UL19-6053 | - | CAUCGCCGCCAAACAUCGCUGCC | 23 | 10652 |
| HSV1-UL19-6054 | - | ACAUCGCCGCCAAACAUCGCUGCC | 24 | 10653 |
| HSV1-UL19-6055 | - | AGCGCCCCCCGGGGUGCC | 18 | 10654 |
| HSV1-UL19-6056 | - | AAGCGCCCCCCGGGGUGCC | 19 | 10655 |
| HSV1-UL19-2310 | - | UAAGCGCCCCCCGGGGUGCC | 20 | 2549 |
| HSV1-UL19-6057 | - | UUAAGCGCCCCCCGGGGUGCC | 21 | 10656 |
| HSV1-UL19-6058 | - | UUUAAGCGCCCCCCGGGGUGCC | 22 | 10657 |
| HSV1-UL19-6059 | - | GUUUAAGCGCCCCCCGGGGUGCC | 23 | 10658 |
| HSV1-UL19-6060 | - | AGUUUAAGCGCCCCCCGGGGUGCC | 24 | 10659 |
| HSV1-UL19-6061 | - | CCGUCGUGCAGCACGUCC | 18 | 10660 |
| HSV1-UL19-6062 | - | CCCGUCGUGCAGCACGUCC | 19 | 10661 |
| HSV1-UL19-2229 | - | GCCCGUCGUGCAGCACGUCC | 20 | 2502 |
| HSV1-UL19-6063 | - | AGCCCGUCGUGCAGCACGUCC | 21 | 10662 |
| HSV1-UL19-6064 | - | CAGCCCGUCGUGCAGCACGUCC | 22 | 10663 |
| HSV1-UL19-6065 | - | ACAGCCCGUCGUGCAGCACGUCC | 23 | 10664 |
| HSV1-UL19-6066 | - | GACAGCCCGUCGUGCAGCACGUCC | 24 | 10665 |
| HSV1-UL19-3600 | - | GCAACGUCCAGGCCGUCC | 18 | 8199 |
| HSV1-UL19-3601 | - | CGCAACGUCCAGGCCGUCC | 19 | 8200 |
| HSV1-UL19-3602 | - | UCGCAACGUCCAGGCCGUCC | 20 | 8201 |
| HSV1-UL19-3603 | - | CUCGCAACGUCCAGGCCGUCC | 21 | 8202 |
| HSV1-UL19-3604 | - | GCUCGCAACGUCCAGGCCGUCC | 22 | 8203 |
| HSV1-UL19-3605 | - | CGCUCGCAACGUCCAGGCCGUCC | 23 | 8204 |
| HSV1-UL19-3606 | - | UCGCUCGCAACGUCCAGGCCGUCC | 24 | 8205 |
| HSV1-UL19-6067 | - | UGCAGAACAUGGUGGUCC | 18 | 10666 |
| HSV1-UL19-6068 | - | CUGCAGAACAUGGUGGUCC | 19 | 10667 |
| HSV1-UL19-417 | - | CCUGCAGAACAUGGUGGUCC | 20 | 803 |
| HSV1-UL19-6069 | - | CCCUGCAGAACAUGGUGGUCC | 21 | 10668 |
| HSV1-UL19-6070 | - | ACCCUGCAGAACAUGGUGGUCC | 22 | 10669 |
| HSV1-UL19-6071 | - | CACCCUGCAGAACAUGGUGGUCC | 23 | 10670 |
| HSV1-UL19-6072 | - | CCACCCUGCAGAACAUGGUGGUCC | 24 | 10671 |
| HSV1-UL19-6073 | - | AAACCGCCCCGCCCUUCC | 18 | 10672 |
| HSV1-UL19-6074 | - | UAAACCGCCCCGCCCUUCC | 19 | 10673 |
| HSV1-UL19-6075 | - | AUAAACCGCCCCGCCCUUCC | 20 | 10674 |
| HSV1-UL19-6076 | - | AAUAAACCGCCCCGCCCUUCC | 21 | 10675 |
| HSV1-UL19-6077 | - | UAAUAAACCGCCCCGCCCUUCC | 22 | 10676 |
| HSV1-UL19-6078 | - | GUAAUAAACCGCCCCGCCCUUCC | 23 | 10677 |
| HSV1-UL19-6079 | - | UGUAAUAAACCGCCCCGCCCUUCC | 24 | 10678 |
| HSV1-UL19-6080 | - | GGCAGACGCUGGACGAGC | 18 | 10679 |
| HSV1-UL19-6081 | - | CGGCAGACGCUGGACGAGC | 19 | 10680 |
| HSV1-UL19-2082 | - | CCGGCAGACGCUGGACGAGC | 20 | 2418 |
| HSV1-UL19-6082 | - | ACCGGCAGACGCUGGACGAGC | 21 | 10681 |
| HSV1-UL19-6083 | - | AACCGGCAGACGCUGGACGAGC | 22 | 10682 |
| HSV1-UL19-6084 | - | GAACCGGCAGACGCUGGACGAGC | 23 | 10683 |
| HSV1-UL19-6085 | - | UGAACCGGCAGACGCUGGACGAGC | 24 | 10684 |
| HSV1-UL19-6086 | - | ACGCCCGGGGCCUGGAGC | 18 | 10685 |
| HSV1-UL19-6087 | - | GACGCCCGGGGCCUGGAGC | 19 | 10686 |
| HSV1-UL19-337 | - | GGACGCCCGGGGCCUGGAGC | 20 | 723 |
| HSV1-UL19-6088 | - | GGGACGCCCGGGGCCUGGAGC | 21 | 10687 |
| HSV1-UL19-6089 | - | CGGGACGCCCGGGGCCUGGAGC | 22 | 10688 |
| HSV1-UL19-6090 | - | CCGGGACGCCCGGGGCCUGGAGC | 23 | 10689 |
| HSV1-UL19-6091 | - | UCCGGGACGCCCGGGGCCUGGAGC | 24 | 10690 |
| HSV1-UL19-6092 | - | CGCGACUGCCGGGUUAGC | 18 | 10691 |
| HSV1-UL19-6093 | - | UCGCGACUGCCGGGUUAGC | 19 | 10692 |
| HSV1-UL19-2187 | - | AUCGCGACUGCCGGGUUAGC | 20 | 2476 |
| HSV1-UL19-6094 | - | CAUCGCGACUGCCGGGUUAGC | 21 | 10693 |
| HSV1-UL19-6095 | - | CCAUCGCGACUGCCGGGUUAGC | 22 | 10694 |
| HSV1-UL19-6096 | - | GCCAUCGCGACUGCCGGGUUAGC | 23 | 10695 |
| HSV1-UL19-6097 | - | CGCCAUCGCGACUGCCGGGUUAGC | 24 | 10696 |
| HSV1-UL19-6098 | - | GCAGCUGUUUUUGAACGC | 18 | 10697 |
| HSV1-UL19-6099 | - | AGCAGCUGUUUUUGAACGC | 19 | 10698 |
| HSV1-UL19-2119 | - | CAGCAGCUGUUUUUGAACGC | 20 | 2441 |
| HSV1-UL19-6100 | - | GCAGCAGCUGUUUUUGAACGC | 21 | 10699 |
| HSV1-UL19-6101 | - | UGCAGCAGCUGUUUUUGAACGC | 22 | 10700 |
| HSV1-UL19-6102 | - | AUGCAGCAGCUGUUUUUGAACGC | 23 | 10701 |
| HSV1-UL19-6103 | - | CAUGCAGCAGCUGUUUUUGAACGC | 24 | 10702 |
| HSV1-UL19-6104 | - | UGACGCAUGCCGACACGC | 18 | 10703 |
| HSV1-UL19-6105 | - | CUGACGCAUGCCGACACGC | 19 | 10704 |
| HSV1-UL19-2057 | - | UCUGACGCAUGCCGACACGC | 20 | 2399 |
| HSV1-UL19-6106 | - | GUCUGACGCAUGCCGACACGC | 21 | 10705 |
| HSV1-UL19-6107 | - | CGUCUGACGCAUGCCGACACGC | 22 | 10706 |
| HSV1-UL19-6108 | - | CCGUCUGACGCAUGCCGACACGC | 23 | 10707 |
| HSV1-UL19-6109 | - | CCCGUCUGACGCAUGCCGACACGC | 24 | 10708 |
| HSV1-UL19-6110 | - | CAACGGGGAAGCCCACGC | 18 | 10709 |
| HSV1-UL19-6111 | - | GCAACGGGGAAGCCCACGC | 19 | 10710 |
| HSV1-UL19-2320 | - | CGCAACGGGGAAGCCCACGC | 20 | 2554 |
| HSV1-UL19-6112 | - | CCGCAACGGGGAAGCCCACGC | 21 | 10711 |
| HSV1-UL19-6113 | - | CCCGCAACGGGGAAGCCCACGC | 22 | 10712 |
| HSV1-UL19-6114 | - | GCCCGCAACGGGGAAGCCCACGC | 23 | 10713 |
| HSV1-UL19-6115 | - | UGCCCGCAACGGGGAAGCCCACGC | 24 | 10714 |
| HSV1-UL19-6116 | - | ACGGUUUGCCGCGCACGC | 18 | 10715 |
| HSV1-UL19-6117 | - | AACGGUUUGCCGCGCACGC | 19 | 10716 |
| HSV1-UL19-263 | - | GAACGGUUUGCCGCGCACGC | 20 | 649 |
| HSV1-UL19-6118 | - | GGAACGGUUUGCCGCGCACGC | 21 | 10717 |
| HSV1-UL19-6119 | - | UGGAACGGUUUGCCGCGCACGC | 22 | 10718 |
| HSV1-UL19-6120 | - | AUGGAACGGUUUGCCGCGCACGC | 23 | 10719 |
| HSV1-UL19-6121 | - | CAUGGAACGGUUUGCCGCGCACGC | 24 | 10720 |
| HSV1-UL19-6122 | - | GGCGGCGUUCCGGGACGC | 18 | 10721 |
| HSV1-UL19-6123 | - | CGGCGGCGUUCCGGGACGC | 19 | 10722 |
| HSV1-UL19-2150 | - | CCGGCGGCGUUCCGGGACGC | 20 | 2454 |
| HSV1-UL19-6124 | - | UCCGGCGGCGUUCCGGGACGC | 21 | 10723 |
| HSV1-UL19-6125 | - | GUCCGGCGGCGUUCCGGGACGC | 22 | 10724 |
| HSV1-UL19-6126 | - | UGUCCGGCGGCGUUCCGGGACGC | 23 | 10725 |
| HSV1-UL19-6127 | - | AUGUCCGGCGGCGUUCCGGGACGC | 24 | 10726 |
| HSV1-UL19-6128 | - | CGCCGGGGGCGUUUACGC | 18 | 10727 |
| HSV1-UL19-6129 | - | CCGCCGGGGGCGUUUACGC | 19 | 10728 |
| HSV1-UL19-533 | - | GCCGCCGGGGGCGUUUACGC | 20 | 919 |
| HSV1-UL19-6130 | - | CGCCGCCGGGGGCGUUUACGC | 21 | 10729 |
| HSV1-UL19-6131 | - | GCGCCGCCGGGGGCGUUUACGC | 22 | 10730 |
| HSV1-UL19-6132 | - | CGCGCCGCCGGGGGCGUUUACGC | 23 | 10731 |
| HSV1-UL19-6133 | - | ACGCGCCGCCGGGGGCGUUUACGC | 24 | 10732 |
| HSV1-UL19-6134 | - | GUCGUGGCGGGAAACCGC | 18 | 10733 |
| HSV1-UL19-6135 | - | GGUCGUGGCGGGAAACCGC | 19 | 10734 |
| HSV1-UL19-2269 | - | CGGUCGUGGCGGGAAACCGC | 20 | 2528 |
| HSV1-UL19-6136 | - | GCGGUCGUGGCGGGAAACCGC | 21 | 10735 |
| HSV1-UL19-6137 | - | CGCGGUCGUGGCGGGAAACCGC | 22 | 10736 |
| HSV1-UL19-6138 | - | ACGCGGUCGUGGCGGGAAACCGC | 23 | 10737 |
| HSV1-UL19-6139 | - | AACGCGGUCGUGGCGGGAAACCGC | 24 | 10738 |
| HSV1-UL19-6140 | - | GGCAAGGCGGGCCACCGC | 18 | 10739 |
| HSV1-UL19-6141 | - | GGGCAAGGCGGGCCACCGC | 19 | 10740 |
| HSV1-UL19-211 | - | UGGGCAAGGCGGGCCACCGC | 20 | 597 |
| HSV1-UL19-6142 | - | CUGGGCAAGGCGGGCCACCGC | 21 | 10741 |
| HSV1-UL19-6143 | - | CCUGGGCAAGGCGGGCCACCGC | 22 | 10742 |
| HSV1-UL19-6144 | - | UCCUGGGCAAGGCGGGCCACCGC | 23 | 10743 |
| HSV1-UL19-6145 | - | UUCCUGGGCAAGGCGGGCCACCGC | 24 | 10744 |
| HSV1-UL19-6146 | - | CCCCCGGGGACGCGCCGC | 18 | 10745 |
| HSV1-UL19-6147 | - | ACCCCCGGGGACGCGCCGC | 19 | 10746 |
| HSV1-UL19-528 | - | AACCCCCGGGGACGCGCCGC | 20 | 914 |
| HSV1-UL19-6148 | - | CAACCCCCGGGGACGCGCCGC | 21 | 10747 |
| HSV1-UL19-6149 | - | GCAACCCCCGGGGACGCGCCGC | 22 | 10748 |
| HSV1-UL19-6150 | - | UGCAACCCCCGGGGACGCGCCGC | 23 | 10749 |
| HSV1-UL19-6151 | - | CUGCAACCCCCGGGGACGCGCCGC | 24 | 10750 |
| HSV1-UL19-6152 | - | GUCGUGCAGCACGUCCGC | 18 | 10751 |
| HSV1-UL19-6153 | - | CGUCGUGCAGCACGUCCGC | 19 | 10752 |
| HSV1-UL19-2230 | - | CCGUCGUGCAGCACGUCCGC | 20 | 2503 |
| HSV1-UL19-6154 | - | CCCGUCGUGCAGCACGUCCGC | 21 | 10753 |
| HSV1-UL19-6155 | - | GCCCGUCGUGCAGCACGUCCGC | 22 | 10754 |
| HSV1-UL19-6156 | - | AGCCCGUCGUGCAGCACGUCCGC | 23 | 10755 |
| HSV1-UL19-6157 | - | CAGCCCGUCGUGCAGCACGUCCGC | 24 | 10756 |
| HSV1-UL19-6158 | - | CGACUGCCGGGUUAGCGC | 18 | 10757 |
| HSV1-UL19-6159 | - | GCGACUGCCGGGUUAGCGC | 19 | 10758 |
| HSV1-UL19-387 | - | CGCGACUGCCGGGUUAGCGC | 20 | 773 |
| HSV1-UL19-6160 | - | UCGCGACUGCCGGGUUAGCGC | 21 | 10759 |
| HSV1-UL19-6161 | - | AUCGCGACUGCCGGGUUAGCGC | 22 | 10760 |
| HSV1-UL19-6162 | - | CAUCGCGACUGCCGGGUUAGCGC | 23 | 10761 |
| HSV1-UL19-6163 | - | CCAUCGCGACUGCCGGGUUAGCGC | 24 | 10762 |
| HSV1-UL19-6164 | - | ACGGGGAAGCCCACGCGC | 18 | 10763 |
| HSV1-UL19-6165 | - | AACGGGGAAGCCCACGCGC | 19 | 10764 |
| HSV1-UL19-578 | - | CAACGGGGAAGCCCACGCGC | 20 | 964 |
| HSV1-UL19-6166 | - | GCAACGGGGAAGCCCACGCGC | 21 | 10765 |
| HSV1-UL19-6167 | - | CGCAACGGGGAAGCCCACGCGC | 22 | 10766 |
| HSV1-UL19-6168 | - | CCGCAACGGGGAAGCCCACGCGC | 23 | 10767 |
| HSV1-UL19-6169 | - | CCCGCAACGGGGAAGCCCACGCGC | 24 | 10768 |
| HSV1-UL19-6170 | - | ACGCUCACCCAGCCGCGC | 18 | 10769 |
| HSV1-UL19-6171 | - | CACGCUCACCCAGCCGCGC | 19 | 10770 |
| HSV1-UL19-2255 | - | UCACGCUCACCCAGCCGCGC | 20 | 2518 |
| HSV1-UL19-6172 | - | UUCACGCUCACCCAGCCGCGC | 21 | 10771 |
| HSV1-UL19-6173 | - | CUUCACGCUCACCCAGCCGCGC | 22 | 10772 |
| HSV1-UL19-6174 | - | ACUUCACGCUCACCCAGCCGCGC | 23 | 10773 |
| HSV1-UL19-6175 | - | AACUUCACGCUCACCCAGCCGCGC | 24 | 10774 |
| HSV1-UL19-6176 | - | CGUCCACGCGCUGUUCGC | 18 | 10775 |
| HSV1-UL19-6177 | - | CCGUCCACGCGCUGUUCGC | 19 | 10776 |
| HSV1-UL19-446 | - | CCCGUCCACGCGCUGUUCGC | 20 | 832 |
| HSV1-UL19-6178 | - | CCCCGUCCACGCGCUGUUCGC | 21 | 10777 |
| HSV1-UL19-6179 | - | UCCCCGUCCACGCGCUGUUCGC | 22 | 10778 |
| HSV1-UL19-6180 | - | CUCCCCGUCCACGCGCUGUUCGC | 23 | 10779 |
| HSV1-UL19-6181 | - | CCUCCCCGUCCACGCGCUGUUCGC | 24 | 10780 |
| HSV1-UL19-6182 | - | GCUGCACAACACCCAGGC | 18 | 10781 |
| HSV1-UL19-6183 | - | UGCUGCACAACACCCAGGC | 19 | 10782 |
| HSV1-UL19-2192 | - | CUGCUGCACAACACCCAGGC | 20 | 2478 |
| HSV1-UL19-6184 | - | GCUGCUGCACAACACCCAGGC | 21 | 10783 |
| HSV1-UL19-6185 | - | AGCUGCUGCACAACACCCAGGC | 22 | 10784 |
| HSV1-UL19-6186 | - | CAGCUGCUGCACAACACCCAGGC | 23 | 10785 |
| HSV1-UL19-6187 | - | CCAGCUGCUGCACAACACCCAGGC | 24 | 10786 |
| HSV1-UL19-6188 | - | CUCAUGUACGACCACGGC | 18 | 10787 |
| HSV1-UL19-6189 | - | CCUCAUGUACGACCACGGC | 19 | 10788 |
| HSV1-UL19-2293 | - | CCCUCAUGUACGACCACGGC | 20 | 2539 |
| HSV1-UL19-6190 | - | GCCCUCAUGUACGACCACGGC | 21 | 10789 |
| HSV1-UL19-6191 | - | CGCCCUCAUGUACGACCACGGC | 22 | 10790 |
| HSV1-UL19-6192 | - | CCGCCCUCAUGUACGACCACGGC | 23 | 10791 |
| HSV1-UL19-6193 | - | ACCGCCCUCAUGUACGACCACGGC | 24 | 10792 |
| HSV1-UL19-6194 | - | GCAGGGAUGGACCACGGC | 18 | 10793 |
| HSV1-UL19-6195 | - | CGCAGGGAUGGACCACGGC | 19 | 10794 |
| HSV1-UL19-2275 | - | GCGCAGGGAUGGACCACGGC | 20 | 2532 |
| HSV1-UL19-6196 | - | CGCGCAGGGAUGGACCACGGC | 21 | 10795 |
| HSV1-UL19-6197 | - | GCGCGCAGGGAUGGACCACGGC | 22 | 10796 |
| HSV1-UL19-6198 | - | GGCGCGCAGGGAUGGACCACGGC | 23 | 10797 |
| HSV1-UL19-6199 | - | CGGCGCGCAGGGAUGGACCACGGC | 24 | 10798 |
| HSV1-UL19-6200 | - | GACUUUACCCUGACCGGC | 18 | 10799 |
| HSV1-UL19-6201 | - | UGACUUUACCCUGACCGGC | 19 | 10800 |
| HSV1-UL19-2176 | - | AUGACUUUACCCUGACCGGC | 20 | 2467 |
| HSV1-UL19-6202 | - | GAUGACUUUACCCUGACCGGC | 21 | 10801 |
| HSV1-UL19-6203 | - | UGAUGACUUUACCCUGACCGGC | 22 | 10802 |
| HSV1-UL19-6204 | - | UUGAUGACUUUACCCUGACCGGC | 23 | 10803 |
| HSV1-UL19-6205 | - | GUUGAUGACUUUACCCUGACCGGC | 24 | 10804 |
| HSV1-UL19-6206 | - | CCGGAGAUCGCCCCCGGC | 18 | 10805 |
| HSV1-UL19-6207 | - | CCCGGAGAUCGCCCCCGGC | 19 | 10806 |
| HSV1-UL19-2210 | - | UCCCGGAGAUCGCCCCCGGC | 20 | 2489 |
| HSV1-UL19-6208 | - | GUCCCGGAGAUCGCCCCCGGC | 21 | 10807 |
| HSV1-UL19-6209 | - | GGUCCCGGAGAUCGCCCCCGGC | 22 | 10808 |
| HSV1-UL19-6210 | - | UGGUCCCGGAGAUCGCCCCCGGC | 23 | 10809 |
| HSV1-UL19-6211 | - | GUGGUCCCGGAGAUCGCCCCCGGC | 24 | 10810 |
| HSV1-UL19-6212 | - | AGCUCCAGGUGGCCCGGC | 18 | 10811 |
| HSV1-UL19-6213 | - | CAGCUCCAGGUGGCCCGGC | 19 | 10812 |
| HSV1-UL19-2249 | - | CCAGCUCCAGGUGGCCCGGC | 20 | 2516 |
| HSV1-UL19-6214 | - | GCCAGCUCCAGGUGGCCCGGC | 21 | 10813 |
| HSV1-UL19-6215 | - | GGCCAGCUCCAGGUGGCCCGGC | 22 | 10814 |
| HSV1-UL19-6216 | - | GGGCCAGCUCCAGGUGGCCCGGC | 23 | 10815 |
| HSV1-UL19-6217 | - | UGGGCCAGCUCCAGGUGGCCCGGC | 24 | 10816 |
| HSV1-UL19-6218 | - | GACGUUCCCCCGGCCGGC | 18 | 10817 |
| HSV1-UL19-6219 | - | GGACGUUCCCCCGGCCGGC | 19 | 10818 |
| HSVI-UL19-2142 | - | GGGACGUUCCCCCGGCCGGC | 20 | 2451 |
| HSV1-UL19-6220 | - | GGGGACGUUCCCCCGGCCGGC | 21 | 10819 |
| HSV1-UL19-6221 | - | GGGGGACGUUCCCCCGGCCGGC | 22 | 10820 |
| HSV1-UL19-6222 | - | GGGGGGACGUUCCCCCGGCCGGC | 23 | 10821 |
| HSV1-UL19-6223 | - | GGGGGGGACGUUCCCCCGGCCGGC | 24 | 10822 |
| HSV1-UL19-6224 | - | CGUCCGCGAGAGCGCGGC | 18 | 10823 |
| HSV1-UL19-6225 | - | ACGUCCGCGAGAGCGCGGC | 19 | 10824 |
| HSV1-UL19-460 | - | CACGUCCGCGAGAGCGCGGC | 20 | 846 |
| HSV1-UL19-6226 | - | GCACGUCCGCGAGAGCGCGGC | 21 | 10825 |
| HSV1-UL19-6227 | - | AGCACGUCCGCGAGAGCGCGGC | 22 | 10826 |
| HSV1-UL19-6228 | - | CAGCACGUCCGCGAGAGCGCGGC | 23 | 10827 |
| HSV1-UL19-6229 | - | GCAGCACGUCCGCGAGAGCGCGGC | 24 | 10828 |
| HSV1-UL19-6230 | - | GAUCUGGUGUCCAUCGGC | 18 | 10829 |
| HSV1-UL19-6231 | - | GGAUCUGGUGUCCAUCGGC | 19 | 10830 |
| HSV1-UL19-2086 | - | CGGAUCUGGUGUCCAUCGGC | 20 | 2422 |
| HSV1-UL19-6232 | - | GCGGAUCUGGUGUCCAUCGGC | 21 | 10831 |
| HSV1-UL19-6233 | - | CGCGGAUCUGGUGUCCAUCGGC | 22 | 10832 |
| HSV1-UL19-6234 | - | GCGCGGAUCUGGUGUCCAUCGGC | 23 | 10833 |
| HSV1-UL19-6235 | - | CGCGCGGAUCUGGUGUCCAUCGGC | 24 | 10834 |
| HSV1-UL19-6236 | - | UUCCGGGACGCCCGGGGC | 18 | 10835 |
| HSV1-UL19-6237 | - | GUUCCGGGACGCCCGGGGC | 19 | 10836 |
| HSV1-UL19-2152 | - | CGUUCCGGGACGCCCGGGGC | 20 | 2455 |
| HSV1-UL19-6238 | - | GCGUUCCGGGACGCCCGGGGC | 21 | 10837 |
| HSV1-UL19-6239 | - | GGCGUUCCGGGACGCCCGGGGC | 22 | 10838 |
| HSV1-UL19-6240 | - | CGGCGUUCCGGGACGCCCGGGGC | 23 | 10839 |
| HSV1-UL19-6241 | - | GCGGCGUUCCGGGACGCCCGGGGC | 24 | 10840 |
| HSV1-UL19-6242 | - | GCGAACGUGGACCUGGGC | 18 | 10841 |
| HSV1-UL19-6243 | - | CGCGAACGUGGACCUGGGC | 19 | 10842 |
| HSV1-UL19-2258 | - | GCGCGAACGUGGACCUGGGC | 20 | 2521 |
| HSV1-UL19-6244 | - | CGCGCGAACGUGGACCUGGGC | 21 | 10843 |
| HSV1-UL19-6245 | - | GCGCGCGAACGUGGACCUGGGC | 22 | 10844 |
| HSV1-UL19-6246 | - | CGCGCGCGAACGUGGACCUGGGC | 23 | 10845 |
| HSV1-UL19-6247 | - | CCGCGCGCGAACGUGGACCUGGGC | 24 | 10846 |
| HSV1-UL19-6248 | - | GCCCGGCACGAAACUGGC | 18 | 10847 |
| HSV1-UL19-6249 | - | GGCCCGGCACGAAACUGGC | 19 | 10848 |
| HSV1-UL19-485 | - | UGGCCCGGCACGAAACUGGC | 20 | 871 |
| HSV1-UL19-6250 | - | GUGGCCCGGCACGAAACUGGC | 21 | 10849 |
| HSV1-UL19-6251 | - | GGUGGCCCGGCACGAAACUGGC | 22 | 10850 |
| HSV1-UL19-6252 | - | AGGUGGCCCGGCACGAAACUGGC | 23 | 10851 |
| HSV1-UL19-6253 | - | CAGGUGGCCCGGCACGAAACUGGC | 24 | 10852 |
| HSV1-UL19-6254 | - | GCGGAACGCGGUCGUGGC | 18 | 10853 |
| HSV1-UL19-6255 | - | UGCGGAACGCGGUCGUGGC | 19 | 10854 |
| HSV1-UL19-510 | - | CUGCGGAACGCGGUCGUGGC | 20 | 896 |
| HSV1-UL19-6256 | - | CCUGCGGAACGCGGUCGUGGC | 21 | 10855 |
| HSV1-UL19-6257 | - | ACCUGCGGAACGCGGUCGUGGC | 22 | 10856 |
| HSV1-UL19-6258 | - | UACCUGCGGAACGCGGUCGUGGC | 23 | 10857 |
| HSV1-UL19-6259 | - | GUACCUGCGGAACGCGGUCGUGGC | 24 | 10858 |
| HSV1-UL19-6260 | - | CCUGGCCACCAGGGUGGC | 18 | 10859 |
| HSV1-UL19-6261 | - | GCCUGGCCACCAGGGUGGC | 19 | 10860 |
| HSV1-UL19-2045 | - | CGCCUGGCCACCAGGGUGGC | 20 | 2389 |
| HSV1-UL19-6262 | - | CCGCCUGGCCACCAGGGUGGC | 21 | 10861 |
| HSV1-UL19-6263 | - | GCCGCCUGGCCACCAGGGUGGC | 22 | 10862 |
| HSV1-UL19-6264 | - | GGCCGCCUGGCCACCAGGGUGGC | 23 | 10863 |
| HSV1-UL19-6265 | - | CGGCCGCCUGGCCACCAGGGUGGC | 24 | 10864 |
| HSV1-UL19-6266 | - | GCCACCUGCUGGAGAUGC | 18 | 10865 |
| HSV1-UL19-6267 | - | CGCCACCUGCUGGAGAUGC | 19 | 10866 |
| HSV1-UL19-243 | - | CCGCCACCUGCUGGAGAUGC | 20 | 629 |
| HSV1-UL19-6268 | - | GCCGCCACCUGCUGGAGAUGC | 21 | 10867 |
| HSV1-UL19-6269 | - | GGCCGCCACCUGCUGGAGAUGC | 22 | 10868 |
| HSV1-UL19-6270 | - | GGGCCGCCACCUGCUGGAGAUGC | 23 | 10869 |
| HSV1-UL19-6271 | - | UGGGCCGCCACCUGCUGGAGAUGC | 24 | 10870 |
| HSV1-UL19-6272 | - | ACGUGGGCCGCCACCUGC | 18 | 10871 |
| HSV1-UL19-6273 | - | GACGUGGGCCGCCACCUGC | 19 | 10872 |
| HSV1-UL19-242 | - | CGACGUGGGCCGCCACCUGC | 20 | 628 |
| HSV1-UL19-6274 | - | ACGACGUGGGCCGCCACCUGC | 21 | 10873 |
| HSV1-UL19-6275 | - | GACGACGUGGGCCGCCACCUGC | 22 | 10874 |
| HSV1-UL19-6276 | - | GGACGACGUGGGCCGCCACCUGC | 23 | 10875 |
| HSV1-UL19-6277 | - | UGGACGACGUGGGCCGCCACCUGC | 24 | 10876 |
| HSV1-UL19-6278 | - | UGGCCGACGUGGAGCUGC | 18 | 10877 |
| HSV1-UL19-6279 | - | GUGGCCGACGUGGAGCUGC | 19 | 10878 |
| HSV1-UL19-311 | - | GGUGGCCGACGUGGAGCUGC | 20 | 697 |
| HSV1-UL19-6280 | - | GGGUGGCCGACGUGGAGCUGC | 21 | 10879 |
| HSV1-UL19-6281 | - | GGGGUGGCCGACGUGGAGCUGC | 22 | 10880 |
| HSV1-UL19-6282 | - | GGGGGUGGCCGACGUGGAGCUGC | 23 | 10881 |
| HSV1-UL19-6283 | - | UGGGGGUGGCCGACGUGGAGCUGC | 24 | 10882 |
| HSV1-UL19-6284 | - | GCCGCCAAACAUCGCUGC | 18 | 10883 |
| HSV1-UL19-6285 | - | CGCCGCCAAACAUCGCUGC | 19 | 10884 |
| HSV1-UL19-2303 | - | UCGCCGCCAAACAUCGCUGC | 20 | 2545 |
| HSV1-UL19-6286 | - | AUCGCCGCCAAACAUCGCUGC | 21 | 10885 |
| HSV1-UL19-6287 | - | CAUCGCCGCCAAACAUCGCUGC | 22 | 10886 |
| HSV1-UL19-6288 | - | ACAUCGCCGCCAAACAUCGCUGC | 23 | 10887 |
| HSV1-UL19-6289 | - | GACAUCGCCGCCAAACAUCGCUGC | 24 | 10888 |
| HSV1-UL19-6290 | - | AGAUGCUGCACGUGCUGC | 18 | 10889 |
| HSV1-UL19-6291 | - | CAGAUGCUGCACGUGCUGC | 19 | 10890 |
| HSV1-UL19-194 | - | CCAGAUGCUGCACGUGCUGC | 20 | 580 |
| HSV1-UL19-6292 | - | ACCAGAUGCUGCACGUGCUGC | 21 | 10891 |
| HSV1-UL19-6293 | - | GACCAGAUGCUGCACGUGCUGC | 22 | 10892 |
| HSV1-UL19-6294 | - | CGACCAGAUGCUGCACGUGCUGC | 23 | 10893 |
| HSV1-UL19-6295 | - | CCGACCAGAUGCUGCACGUGCUGC | 24 | 10894 |
| HSV1-UL19-6296 | - | UGC CUGGAGC GCCUGAUC | 18 | 10895 |
| HSV1-UL19-6297 | - | CUGCCUGGAGCGCCUGAUC | 19 | 10896 |
| HSV1-UL19-2305 | - | GCUGCCUGGAGCGCCUGAUC | 20 | 2546 |
| HSV1-UL19-6298 | - | CGCUGCCUGGAGCGCCUGAUC | 21 | 10897 |
| HSV1-UL19-6299 | - | UCGCUGCCUGGAGCGCCUGAUC | 22 | 10898 |
| HSV1-UL19-6300 | - | AUCGCUGCCUGGAGCGCCUGAUC | 23 | 10899 |
| HSV1-UL19-6301 | - | CAUCGCUGCCUGGAGCGCCUGAUC | 24 | 10900 |
| HSV1-UL19-6302 | - | CCGGGGUGCCGCGAACUC | 18 | 10901 |
| HSV1-UL19-6303 | - | CCCGGGGUGCCGCGAACUC | 19 | 10902 |
| HSV1-UL19-2311 | - | CCCCGGGGUGCCGCGAACUC | 20 | 2550 |
| HSV1-UL19-6304 | - | CCCCCGGGGUGCCGCGAACUC | 21 | 10903 |
| HSV1-UL19-6305 | - | CCCCCCGGGGUGCCGCGAACUC | 22 | 10904 |
| HSV1-UL19-6306 | - | GCCCCCCGGGGUGCCGCGAACUC | 23 | 10905 |
| HSV1-UL19-6307 | - | CGCCCCCCGGGGUGCCGCGAACUC | 24 | 10906 |
| HSV1-UL19-6308 | - | UACGUCGUGACCUACCUC | 18 | 10907 |
| HSV1-UL19-6309 | - | GUACGUCGUGACCUACCUC | 19 | 10908 |
| HSV1-UL19-359 | - | CGUACGUCGUGACCUACCUC | 20 | 745 |
| HSV1-UL19-6310 | - | UCGUACGUCGUGACCUACCUC | 21 | 10909 |
| HSV1-UL19-6311 | - | CUCGUACGUCGUGACCUACCUC | 22 | 10910 |
| HSV1-UL19-6312 | - | UCUCGUACGUCGUGACCUACCUC | 23 | 10911 |
| HSV1- UL19-6313 | - | GUCUCGUACGUCGUGACCUACCUC | 24 | 10912 |
| HSV1-UL19-6314 | - | CAGACGCUGGACGAGCUC | 18 | 10913 |
| HSV1-UL19-6315 | - | GCAGACGCUGGACGAGCUC | 19 | 10914 |
| HSV1-UL19-2083 | - | GGCAGACGCUGGACGAGCUC | 20 | 2419 |
| HSV1-UL19-6316 | - | CGGCAGACGCUGGACGAGCUC | 21 | 10915 |
| HSV1-UL19-6317 | - | CCGGCAGACGCUGGACGAGCUC | 22 | 10916 |
| HSV1-UL19-6318 | - | ACCGGCAGACGCUGGACGAGCUC | 23 | 10917 |
| HSV1-UL19-6319 | - | AACCGGCAGACGCUGGACGAGCUC | 24 | 10918 |
| HSV1-UL19-6320 | - | UAUGACGCAUCCCCGCUC | 18 | 10919 |
| HSV1-UL19-6321 | - | CUAUGACGCAUCCCCGCUC | 19 | 10920 |
| HSV1-UL19-2323 | - | UCUAUGACGCAUCCCCGCUC | 20 | 2555 |
| HSV1-UL19-6322 | - | GUCUAUGACGCAUCCCCGCUC | 21 | 10921 |
| HSV1-UL19-6323 | - | CGUCUAUGACGCAUCCCCGCUC | 22 | 10922 |
| HSV1-UL19-6324 | - | UCGUCUAUGACGCAUCCCCGCUC | 23 | 10923 |
| HSV1-UL19-6325 | - | CUCGUCUAUGACGCAUCCCCGCUC | 24 | 10924 |
| HSV1-UL19-6326 | - | CCCUGGUCCCCCCGGCUC | 18 | 10925 |
| HSV1-UL19-6327 | - | CCCCUGGUCCCCCCGGCUC | 19 | 10926 |
| HSV1-UL19-455 | - | CCCCCUGGUCCCCCCGGCUC | 20 | 841 |
| HSV1-UL19-6328 | - | UCCCCCUGGUCCCCCCGGCUC | 21 | 10927 |
| HSV1-UL19-6329 | - | GUCCCCCUGGUCCCCCCGGCUC | 22 | 10928 |
| HSV1-UL19-6330 | - | GGUCCCCCUGGUCCCCCCGGCUC | 23 | 10929 |
| HSV1-UL19-6331 | - | AGGUCCCCCUGGUCCCCCCGGCUC | 24 | 10930 |
| HSV1-UL19-6332 | - | ACAACGCCAACCUGGCUC | 18 | 10931 |
| HSV1-UL19-6333 | - | GACAACGCCAACCUGGCUC | 19 | 10932 |
| HSV1-UL19-2129 | - | CGACAACGCCAACCUGGCUC | 20 | 2447 |
| HSV1-UL19-6334 | - | CCGACAACGCCAACCUGGCUC | 21 | 10933 |
| HSV1-UL19-6335 | - | CCCGACAACGCCAACCUGGCUC | 22 | 10934 |
| HSV1-UL19-6336 | - | GCCCGACAACGCCAACCUGGCUC | 23 | 10935 |
| HSV1-UL19-6337 | - | AGCCCGACAACGCCAACCUGGCUC | 24 | 10936 |
| HSV1-UL19-6338 | - | GCCGUCGAGGCCUGGCUC | 18 | 10937 |
| HSV1-UL19-6339 | - | GGCCGUCGAGGCCUGGCUC | 19 | 10938 |
| HSV1-UL19-2056 | - | AGGCCGUCGAGGCCUGGCUC | 20 | 2398 |
| HSV1-UL19-6340 | - | GAGGCCGUCGAGGCCUGGCUC | 21 | 10939 |
| HSV1-UL19-6341 | - | GGAGGCCGUCGAGGCCUGGCUC | 22 | 10940 |
| HSV1-UL19-6342 | - | GGGAGGCCGUCGAGGCCUGGCUC | 23 | 10941 |
| HSV1-UL19-6343 | - | CGGGAGGCCGUCGAGGCCUGGCUC | 24 | 10942 |
| HSV1-UL19-6344 | - | CGCGGGGCUCCCCCUCUC | 18 | 10943 |
| HSV1-UL19-6345 | - | CCGCGGGGCUCCCCCUCUC | 19 | 10944 |
| HSV1-UL19-2263 | - | GCCGCGGGGCUCCCCCUCUC | 20 | 2525 |
| HSV1-UL19-6346 | - | GGCCGCGGGGCUCCCCCUCUC | 21 | 10945 |
| HSV1-UL19-6347 | - | GGGCCGCGGGGCUCCCCCUCUC | 22 | 10946 |
| HSV1-UL19-6348 | - | UGGGCCGCGGGGCUCCCCCUCUC | 23 | 10947 |
| HSV1-UL19-6349 | - | CUGGGCCGCGGGGCUCCCCCUCUC | 24 | 10948 |
| HSV1-UL19-6350 | - | AGGUGCUGCGCCUGUCUC | 18 | 10949 |
| HSV1-UL19-6351 | - | CAGGUGCUGCGCCUGUCUC | 19 | 10950 |
| HSV1-UL19-274 | - | CCAGGUGCUGCGCCUGUCUC | 20 | 660 |
| HSV1-UL19-6352 | - | GCCAGGUGCUGCGCCUGUCUC | 21 | 10951 |
| HSV1-UL19-6353 | - | CGCCAGGUGCUGCGCCUGUCUC | 22 | 10952 |
| HSV1-UL19-6354 | - | CCGCCAGGUGCUGCGCCUGUCUC | 23 | 10953 |
| HSV1-UL19-6355 | - | ACCGCCAGGUGCUGCGCCUGUCUC | 24 | 10954 |
| HSV1-UL19-6356 | - | GAUGGUGCCCGCCUUCUC | 18 | 10955 |
| HSV1-UL19-6357 | - | UGAUGGUGCCCGCCUUCUC | 19 | 10956 |
| HSV1-UL19-2201 | - | GUGAUGGUGCCCGCCUUCUC | 20 | 2484 |
| HSV1-UL19-6358 | - | CGUGAUGGUGCCCGCCUUCUC | 21 | 10957 |
| HSV1-UL19-6359 | - | ACGUGAUGGUGCCCGCCUUCUC | 22 | 10958 |
| HSV1-UL19-6360 | - | UACGUGAUGGUGCCCGCCUUCUC | 23 | 10959 |
| HSV1-UL19-6361 | - | CUACGUGAUGGUGCCCGCCUUCUC | 24 | 10960 |
| HSV1-UL19-6362 | - | CGGCCAGAGCGACCCGUC | 18 | 10961 |
| HSV1-UL19-6363 | - | ACGGCCAGAGCGACCCGUC | 19 | 10962 |
| HSV1-UL19-2294 | - | CACGGCCAGAGCGACCCGUC | 20 | 2540 |
| HSV1-UL19-6364 | - | CCACGGCCAGAGCGACCCGUC | 21 | 10963 |
| HSV1-UL19-6365 | - | ACCACGGCCAGAGCGACCCGUC | 22 | 10964 |
| HSV1-UL19-6366 | - | GACCACGGCCAGAGCGACCCGUC | 23 | 10965 |
| HSV1-UL19-6367 | - | CGACCACGGCCAGAGCGACCCGUC | 24 | 10966 |
| HSV1-UL19-6368 | - | GGCCUGUUCAAUCCGGUC | 18 | 10967 |
| HSV1-UL19-6369 | - | GGGCCUGUUCAAUCCGGUC | 19 | 10968 |
| HSV1-UL19-2095 | - | UGGGCCUGUUCAAUCCGGUC | 20 | 2429 |
| HSV1-UL19-6370 | - | CUGGGCCUGUUCAAUCCGGUC | 21 | 10969 |
| HSV1-UL19-6371 | - | CCUGGGCCUGUUCAAUCCGGUC | 22 | 10970 |
| HSV1-UL19-6372 | - | CCCUGGGCCUGUUCAAUCCGGUC | 23 | 10971 |
| HSV1-UL19-6373 | - | CCCCUGGGCCUGUUCAAUCCGGUC | 24 | 10972 |
| HSV1-UL19-6374 | - | AACGUUGACGCGGCGGUC | 18 | 10973 |
| HSV1-UL19-6375 | - | GAACGUUGACGCGGCGGUC | 19 | 10974 |
| HSV1-UL19-280 | - | UGAACGUUGACGCGGCGGUC | 20 | 666 |
| HSV1-UL19-6376 | - | AUGAACGUUGACGCGGCGGUC | 21 | 10975 |
| HSV1-UL19-6377 | - | GAUGAACGUUGACGCGGCGGUC | 22 | 10976 |
| HSV1-UL19-6378 | - | UGAUGAACGUUGACGCGGCGGUC | 23 | 10977 |
| HSV1-UL19-6379 | - | CUGAUGAACGUUGACGCGGCGGUC | 24 | 10978 |
| HSV1-UL19-6380 | - | GGGCGGGUCCGAUGGGUC | 18 | 10979 |
| HSV1-UL19-6381 | - | CGGGCGGGUCCGAUGGGUC | 19 | 10980 |
| HSV1-UL19-2125 | - | ACGGGCGGGUCCGAUGGGUC | 20 | 2445 |
| HSV1-UL19-6382 | - | CACGGGCGGGUCCGAUGGGUC | 21 | 10981 |
| HSV1-UL19-6383 | - | CCACGGGCGGGUCCGAUGGGUC | 22 | 10982 |
| HSV1-UL19-6384 | - | CCCACGGGCGGGUCCGAUGGGUC | 23 | 10983 |
| HSV1-UL19-6385 | - | GCCCACGGGCGGGUCCGAUGGGUC | 24 | 10984 |
| HSV1-UL19-6386 | - | ACGUACGGCGAGAUGGUC | 18 | 10985 |
| HSV1-UL19-6387 | - | GACGUACGGCGAGAUGGUC | 19 | 10986 |
| HSV1-UL19-2066 | - | UGACGUACGGCGAGAUGGUC | 20 | 2406 |
| HSV1-UL19-6388 | - | GUGACGUACGGCGAGAUGGUC | 21 | 10987 |
| HSV1-UL19-6389 | - | GGUGACGUACGGCGAGAUGGUC | 22 | 10988 |
| HSV1-UL19-6390 | - | CGGUGACGUACGGCGAGAUGGUC | 23 | 10989 |
| HSV1-UL19-6391 | - | CCGGUGACGUACGGCGAGAUGGUC | 24 | 10990 |
| HSV1-UL19-6392 | - | CUGCAGAACAUGGUGGUC | 18 | 10991 |
| HSV1-UL19-6393 | - | CCUGCAGAACAUGGUGGUC | 19 | 10992 |
| HSV1-UL19-2207 | - | CCCUGCAGAACAUGGUGGUC | 20 | 2487 |
| HSV1-UL19-6394 | - | ACCCUGCAGAACAUGGUGGUC | 21 | 10993 |
| HSV1-UL19-6395 | - | CACCCUGCAGAACAUGGUGGUC | 22 | 10994 |
| HSV1-UL19-6396 | - | CCACCCUGCAGAACAUGGUGGUC | 23 | 10995 |
| HSV1-UL19-6397 | - | GCCACCCUGCAGAACAUGGUGGUC | 24 | 10996 |
| HSV1-UL19-6398 | - | GCGUCGGAGGCGUACUUC | 18 | 10997 |
| HSV1-UL19-6399 | - | CGCGUCGGAGGCGUACUUC | 19 | 10998 |
| HSV1-UL19-2248 | - | GCGCGUCGGAGGCGUACUUC | 20 | 2515 |
| HSV1-UL19-6400 | - | CGCGCGUCGGAGGCGUACUUC | 21 | 10999 |
| HSV1-UL19-6401 | - | GCGCGCGUCGGAGGCGUACUUC | 22 | 11000 |
| HSV1-UL19-6402 | - | AGCGCGCGUCGGAGGCGUACUUC | 23 | 11001 |
| HSV1-UL19-6403 | - | GAGCGCGCGUCGGAGGCGUACUUC | 24 | 11002 |
| HSV1-UL19-6404 | - | ACCUCGGGGGAGACCUUC | 18 | 11003 |
| HSV1-UL19-6405 | - | UACCUCGGGGGAGACCUUC | 19 | 11004 |
| HSV1-UL19-2170 | - | CUACCUCGGGGGAGACCUUC | 20 | 2463 |
| HSV1-UL19-6406 | - | CCUACCUCGGGGGAGACCUUC | 21 | 11005 |
| HSV1-UL19-6407 | - | ACCUACCUCGGGGGAGACCUUC | 22 | 11006 |
| HSV1-UL19-6408 | - | GACCUACCUCGGGGGAGACCUUC | 23 | 11007 |
| HSV1-UL19-6409 | - | UGACCUACCUCGGGGGAGACCUUC | 24 | 11008 |
| HSV1-UL19-6410 | - | CGCCUCCUGCAGUCCUUC | 18 | 11009 |
| HSV1-UL19-6411 | - | GCGCCUCCUGCAGUCCUUC | 19 | 11010 |
| HSV1-UL19-2060 | - | AGCGCCUCCUGCAGUCCUUC | 20 | 2401 |
| HSV1-UL19-6412 | - | CAGCGCCUCCUGCAGUCCUUC | 21 | 11011 |
| HSV1-UL19-6413 | - | ACAGCGCCUCCUGCAGUCCUUC | 22 | 11012 |
| HSV1-UL19-6414 | - | AACAGCGCCUCCUGCAGUCCUUC | 23 | 11013 |
| HSV1-UL19-6415 | - | AAACAGCGCCUCCUGCAGUCCUUC | 24 | 11014 |
| HSV1-UL19-6416 | - | CCACCAUCGCCGCCGUUC | 18 | 11015 |
| HSV1-UL19-6417 | - | GCCACCAUCGCCGCCGUUC | 19 | 11016 |
| HSV1-UL19-2159 | - | CGCCACCAUCGCCGCCGUUC | 20 | 2458 |
| HSV1-UL19-6418 | - | CCGCCACCAUCGCCGCCGUUC | 21 | 11017 |
| HSV1-UL19-6419 | - | CCCGCCACCAUCGCCGCCGUUC | 22 | 11018 |
| HSV1-UL19-6420 | - | CCCCGCCACCAUCGCCGCCGUUC | 23 | 11019 |
| HSV1-UL19-6421 | - | CCCCCGCCACCAUCGCCGCCGUUC | 24 | 11020 |
| HSV1-UL19-6422 | - | ACGCGGUGCGCGGCGUUC | 18 | 11021 |
| HSV1-UL19-6423 | - | CACGCGGUGCGCGGCGUUC | 19 | 11022 |
| HSV1-UL19-2164 | - | ACACGCGGUGCGCGGCGUUC | 20 | 2461 |
| HSV1-UL19-6424 | - | AACACGCGGUGCGCGGCGUUC | 21 | 11023 |
| HSV1-UL19-6425 | - | CAACACGCGGUGCGCGGCGUUC | 22 | 11024 |
| HSV1-UL19-6426 | - | ACAACACGCGGUGCGCGGCGUUC | 23 | 11025 |
| HSV1-UL19-6427 | - | AACAACACGCGGUGCGCGGCGUUC | 24 | 11026 |
| HSV1-UL19-6428 | - | CUAUGUCCGGCGGCGUUC | 18 | 11027 |
| HSV1-UL19-6429 | - | GCUAUGUCCGGCGGCGUUC | 19 | 11028 |
| HSV1-UL19-331 | - | CGCUAUGUCCGGCGGCGUUC | 20 | 717 |
| HSV1-UL19-6430 | - | GCGCUAUGUCCGGCGGCGUUC | 21 | 11029 |
| HSV1-UL19-6431 | - | GGCGCUAUGUCCGGCGGCGUUC | 22 | 11030 |
| HSV1-UL19-6432 | - | UGGCGCUAUGUCCGGCGGCGUUC | 23 | 11031 |
| HSV1-UL19-6433 | - | CUGGCGCUAUGUCCGGCGGCGUUC | 24 | 11032 |
| HSV1-UL19-6434 | - | ACUGAGAACGUGCUGUUC | 18 | 11033 |
| HSV1-UL19-6435 | - | GACUGAGAACGUGCUGUUC | 19 | 11034 |
| HSV1-UL19-2244 | - | UGACUGAGAACGUGCUGUUC | 20 | 2513 |
| HSV1-UL19-6436 | - | GUGACUGAGAACGUGCUGUUC | 21 | 11035 |
| HSV1-UL19-6437 | - | UGUGACUGAGAACGUGCUGUUC | 22 | 11036 |
| HSV1-UL19-6438 | - | UUGUGACUGAGAACGUGCUGUUC | 23 | 11037 |
| HSV1-UL19-6439 | - | UUUGUGACUGAGAACGUGCUGUUC | 24 | 11038 |
| HSV1-UL19-6440 | - | GCGAGAAGCUGGUCUUUC | 18 | 11039 |
| HSV1-UL19-6441 | - | GGCGAGAAGCUGGUCUUUC | 19 | 11040 |
| HSV1-UL19-251 | - | CGGCGAGAAGCUGGUCUUUC | 20 | 637 |
| HSV1-UL19-6442 | - | UCGGCGAGAAGCUGGUCUUUC | 21 | 11041 |
| HSV1-UL19-6443 | - | AUCGGCGAGAAGCUGGUCUUUC | 22 | 11042 |
| HSV1-UL19-6444 | - | CAUCGGCGAGAAGCUGGUCUUUC | 23 | 11043 |
| HSV1-UL19-6445 | - | CCAUCGGCGAGAAGCUGGUCUUUC | 24 | 11044 |
| HSV1-UL19-6446 | - | ACCCGUGUGGCCUGUUUC | 18 | 11045 |
| HSV1-UL19-6447 | - | GACCCGUGUGGCCUGUUUC | 19 | 11046 |
| HSV1-UL19-572 | - | GGACCCGUGUGGCCUGUUUC | 20 | 958 |
| HSV1-UL19-6448 | - | AGGACCCGUGUGGCCUGUUUC | 21 | 11047 |
| HSV1-UL19-6449 | - | GAGGACCCGUGUGGCCUGUUUC | 22 | 11048 |
| HSV1-UL19-6450 | - | GGAGGACCCGUGUGGCCUGUUUC | 23 | 11049 |
| HSV1-UL19-6451 | - | UGGAGGACCCGUGUGGCCUGUUUC | 24 | 11050 |
| HSV1-UL19-6452 | - | UGCGAGACGAGCUUUUUC | 18 | 11051 |
| HSV1-UL19-6453 | - | CUGCGAGACGAGCUUUUUC | 19 | 11052 |
| HSV1-UL19-2050 | - | UCUGCGAGACGAGCUUUUUC | 20 | 2394 |
| HSV1-UL19-6454 | - | UUCUGCGAGACGAGCUUUUUC | 21 | 11053 |
| HSV1-UL19-6455 | - | CUUCUGCGAGACGAGCUUUUUC | 22 | 11054 |
| HSV1-UL19-6456 | - | GCUUCUGCGAGACGAGCUUUUUC | 23 | 11055 |
| HSV1-UL19-6457 | - | AGCUUCUGCGAGACGAGCUUUUUC | 24 | 11056 |
| HSV1-UL19-6458 | - | UGCAGUCCUUCCUGAAAG | 18 | 11057 |
| HSV1-UL19-6459 | - | CUGCAGUCCUUCCUGAAAG | 19 | 11058 |
| HSV1-UL19-226 | - | CCUGCAGUCCUUCCUGAAAG | 20 | 612 |
| HSV1-UL19-6460 | - | UCCUGCAGUCCUUCCUGAAAG | 21 | 11059 |
| HSV1-UL19-6461 | - | CUCCUGCAGUCCUUCCUGAAAG | 22 | 11060 |
| HSV1-UL19-6462 | - | CCUCCUGCAGUCCUUCCUGAAAG | 23 | 11061 |
| HSV1-UL19-6463 | - | GCCUCCUGCAGUCCUUCCUGAAAG | 24 | 11062 |
| HSV1-UL19-6464 | - | GUUUACGCGGGGGACAAG | 18 | 11063 |
| HSV1-UL19-6465 | - | CGUUUACGCGGGGGACAAG | 19 | 11064 |
| HSV1-UL19-2289 | - | GCGUUUACGCGGGGGACAAG | 20 | 2538 |
| HSV1-UL19-6466 | - | GGCGUUUACGCGGGGGACAAG | 21 | 11065 |
| HSV1-UL19-6467 | - | GGGCGUUUACGCGGGGGACAAG | 22 | 11066 |
| HSV1-UL19-6468 | - | GGGGCGUUUACGCGGGGGACAAG | 23 | 11067 |
| HSV1-UL19-6469 | - | GGGGGCGUUUACGCGGGGGACAAG | 24 | 11068 |
| HSV1-UL19-6470 | - | UGGGCGGGCAGGCGCAAG | 18 | 11069 |
| HSV1-UL19-6471 | - | CUGGGCGGGCAGGCGCAAG | 19 | 11070 |
| HSV1-UL19-2180 | - | GCUGGGCGGGCAGGCGCAAG | 20 | 2470 |
| HSV1-UL19-6472 | - | AGCUGGGCGGGCAGGCGCAAG | 21 | 11071 |
| HSV1-UL19-6473 | - | GAGCUGGGCGGGCAGGCGCAAG | 22 | 11072 |
| HSV1-UL19-6474 | - | GGAGCUGGGCGGGCAGGCGCAAG | 23 | 11073 |
| HSV1-UL19-6475 | - | CGGAGCUGGGCGGGCAGGCGCAAG | 24 | 11074 |
| HSV1-UL19-6476 | - | AGCUUUUUCCUGGGCAAG | 18 | 11075 |
| HSV1-UL19-6477 | - | GAGCUUUUUCCUGGGCAAG | 19 | 11076 |
| HSV1-UL19-2051 | - | CGAGCUUUUUCCUGGGCAAG | 20 | 2395 |
| HSV1-UL19-6478 | - | ACGAGCUUUUUCCUGGGCAAG | 21 | 11077 |
| HSV1-UL19-6479 | - | GACGAGCUUUUUCCUGGGCAAG | 22 | 11078 |
| HSV1-UL19-6480 | - | AGACGAGCUUUUUCCUGGGCAAG | 23 | 11079 |
| HSV1-UL19-6481 | - | GAGACGAGCUUUUUCCUGGGCAAG | 24 | 11080 |
| HSV1-UL19-6482 | - | UUGCAUCAUCAGCUCAAG | 18 | 11081 |
| HSV1-UL19-6483 | - | CUUGCAUCAUCAGCUCAAG | 19 | 11082 |
| HSV1-UL19-2238 | - | CCUUGCAUCAUCAGCUCAAG | 20 | 2507 |
| HSV1-UL19-6484 | - | GCCUUGCAUCAUCAGCUCAAG | 21 | 11083 |
| HSV1-UL19-6485 | - | GGCCUUGCAUCAUCAGCUCAAG | 22 | 11084 |
| HSV1-UL19-6486 | - | UGGCCUUGCAUCAUCAGCUCAAG | 23 | 11085 |
| HSV1-UL19-6487 | - | GUGGCCUUGCAUCAUCAGCUCAAG | 24 | 11086 |
| HSV1-UL19-6488 | - | CACCUGCUGGAGAUGCAG | 18 | 11087 |
| HSV1-UL19-6489 | - | CCACCUGCUGGAGAUGCAG | 19 | 11088 |
| HSV1-UL19-2077 | - | GCCACCUGCUGGAGAUGCAG | 20 | 2414 |
| HSV1-UL19-6490 | - | CGCCACCUGCUGGAGAUGCAG | 21 | 11089 |
| HSV1-UL19-6491 | - | CCGCCACCUGCUGGAGAUGCAG | 22 | 11090 |
| HSV1-UL19-6492 | - | GCCGCCACCUGCUGGAGAUGCAG | 23 | 11091 |
| HSV1-UL19-6493 | - | GGCCGCCACCUGCUGGAGAUGCAG | 24 | 11092 |
| HSV1-UL19-6494 | - | AAGC GAAGCUUCUGC GAG | 18 | 11093 |
| HSV1-UL19-6495 | - | AAAGC GAAGCUUCUGC GAG | 19 | 11094 |
| HSV1-UL19-2049 | - | UAAAGC GAAGCUUCUGC GAG | 20 | 2393 |
| HSV1-UL19-6496 | - | CUAAAGCGAAGCUUCUGCGAG | 21 | 11095 |
| HSV1-UL19-6497 | - | GCUAAAGCGAAGCUUCUGCGAG | 22 | 11096 |
| HSV1-UL19-6498 | - | AGCUAAAGCGAAGCUUCUGCGAG | 23 | 11097 |
| HSV1-UL19-6499 | - | GAGCUAAAGCGAAGCUUCUGCGAG | 24 | 11098 |
| HSV1-UL19-6500 | - | ACCCUGACCGGCCCGGAG | 18 | 11099 |
| HSV1-UL19-6501 | - | UACCCUGACCGGCCCGGAG | 19 | 11100 |
| HSV1-UL19-2178 | - | UUACCCUGACCGGCCCGGAG | 20 | 2468 |
| HSV1-UL19-6502 | - | UUUACCCUGACCGGCCCGGAG | 21 | 11101 |
| HSV1-UL19-6503 | - | CUUUACCCUGACCGGCCCGGAG | 22 | 11102 |
| HSV1-UL19-6504 | - | ACUUUACCCUGACCGGCCCGGAG | 23 | 11103 |
| HSV1-UL19-6505 | - | GACUUUACCCUGACCGGCCCGGAG | 24 | 11104 |
| HSV1-UL19-6506 | - | GACGCCCGGGGCCUGGAG | 18 | 11105 |
| HSV1-UL19-6507 | - | GGACGCCCGGGGCCUGGAG | 19 | 11106 |
| HSV1-UL19-2154 | - | GGGACGCCCGGGGCCUGGAG | 20 | 2456 |
| HSV1-UL19-6508 | - | CGGGACGCCCGGGGCCUGGAG | 21 | 11107 |
| HSV1-UL19-6509 | - | CCGGGACGCCCGGGGCCUGGAG | 22 | 11108 |
| HSV1-UL19-6510 | - | UCCGGGACGCCCGGGGCCUGGAG | 23 | 11109 |
| HSV1-UL19-6511 | - | UUCCGGGACGCCCGGGGCCUGGAG | 24 | 1111 0 |
| HSV1-UL19-6512 | - | UCCGCAGUGCCCGCAACG | 18 | 11111 |
| HSV1-UL19-6513 | - | CUCCGCAGUGCCCGCAACG | 19 | 11112 |
| HSV1-UL19-576 | - | CCUCCGCAGUGCCCGCAACG | 20 | 962 |
| HSV1-UL19-6514 | - | UCCUCCGCAGUGCCCGCAACG | 21 | 11113 |
| HSV1-UL19-6515 | - | CUCCUCCGCAGUGCCCGCAACG | 22 | 11114 |
| HSV1-UL19-6516 | - | CCUCCUCCGCAGUGCCCGCAACG | 23 | 11115 |
| HSV1-UL19-6517 | - | CCCUCCUCCGCAGUGCCCGCAACG | 24 | 11116 |
| HSV1-UL19-6518 | - | AACGGUUUGCCGCGCACG | 18 | 11117 |
| HSV1-UL19-6519 | - | GAACGGUUUGCCGCGCACG | 19 | 11118 |
| HSV1-UL19-2097 | - | GGAACGGUUUGCCGCGCACG | 20 | 2430 |
| HSV1-UL19-6520 | - | UGGAACGGUUUGCCGCGCACG | 21 | 11119 |
| HSV1-UL19-6521 | - | AUGGAACGGUUUGCCGCGCACG | 22 | 11120 |
| HSV1-UL19-6522 | - | CAUGGAACGGUUUGCCGCGCACG | 23 | 11121 |
| HSV1-UL19-6523 | - | UCAUGGAACGGUUUGCCGCGCACG | 24 | 11122 |
| HSV1-UL19-6524 | - | GGGACCUGGUGGCGCACG | 18 | 11123 |
| HSV1-UL19-6525 | - | CGGGACCUGGUGGCGCACG | 19 | 11124 |
| HSV1-UL19-2175 | - | CCGGGACCUGGUGGCGCACG | 20 | 2466 |
| HSV1-UL19-6526 | - | ACCGGGACCUGGUGGCGCACG | 21 | 11125 |
| HSV1-UL19-6527 | - | UACCGGGACCUGGUGGCGCACG | 22 | 11126 |
| HSV1-UL19-6528 | - | GUACCGGGACCUGGUGGCGCACG | 23 | 11127 |
| HSV1-UL19-6529 | - | UGUACCGGGACCUGGUGGCGCACG | 24 | 11128 |
| HSV1-UL19-6530 | - | UCGACGGGGCGUUGCACG | 18 | 11129 |
| HSV1-UL19-6531 | - | UUCGACGGGGCGUUGCACG | 19 | 11130 |
| HSV1-UL19-2220 | - | AUUCGACGGGGCGUUGCACG | 20 | 2497 |
| HSV1-UL19-6532 | - | UAUUCGACGGGGCGUUGCACG | 21 | 11131 |
| HSV1-UL19-6533 | - | AUAUUCGACGGGGCGUUGCACG | 22 | 11132 |
| HSV1-UL19-6534 | - | CAUAUUCGACGGGGCGUUGCACG | 23 | 11133 |
| HSV1-UL19-6535 | - | GCAUAUUCGACGGGGCGUUGCACG | 24 | 11134 |
| HSV1-UL19-6536 | - | GACCUGAACCGGCAGACG | 18 | 11135 |
| HSV1-UL19-6537 | - | CGACCUGAACCGGCAGACG | 19 | 11136 |
| HSV1-UL19-2080 | - | UCGACCUGAACCGGCAGACG | 20 | 2416 |
| HSV1-UL19-6538 | - | CUCGACCUGAACCGGCAGACG | 21 | 11137 |
| HSV1-UL19-6539 | - | GCUCGACCUGAACCGGCAGACG | 22 | 11138 |
| HSV1-UL19-6540 | - | AGCUCGACCUGAACCGGCAGACG | 23 | 11139 |
| HSV1-UL19-6541 | - | CAGCUCGACCUGAACCGGCAGACG | 24 | 11140 |
| HSV1-UL19-6542 | - | UUGUGGGGGUGGCCGACG | 18 | 11141 |
| HSV1-UL19-6543 | - | UUUGUGGGGGUGGCCGACG | 19 | 11142 |
| HSV1-UL19-310 | - | CUUUGUGGGGGUGGCCGACG | 20 | 696 |
| HSV1-UL19-6544 | - | UCUUUGUGGGGGUGGCCGACG | 21 | 11143 |
| HSV1-UL19-6545 | - | UUCUUUGUGGGGGUGGCCGACG | 22 | 11144 |
| HSV1-UL19-6546 | - | CUUCUUUGUGGGGGUGGCCGACG | 23 | 11145 |
| HSV1-UL19-6547 | - | ACUUCUUUGUGGGGGUGGCCGACG | 24 | 11146 |
| HSV1-UL19-6548 | - | ACGUGCCGGUGACGUACG | 18 | 11147 |
| HSV1-UL19-6549 | - | GACGUGCCGGUGACGUACG | 19 | 11148 |
| HSV1-UL19-2065 | - | CGACGUGCCGGUGACGUACG | 20 | 2405 |
| HSV1-UL19-6550 | - | CCGACGUGCCGGUGACGUACG | 21 | 11149 |
| HSV1-UL19-6551 | - | GCCGACGUGCCGGUGACGUACG | 22 | 11150 |
| HSV1-UL19-6552 | - | AGCCGACGUGCCGGUGACGUACG | 23 | 11151 |
| HSV1-UL19-6553 | - | AAGCCGACGUGCCGGUGACGUACG | 24 | 11152 |
| HSV1-UL19-6554 | - | CCGCCGGGGGCGUUUACG | 18 | 11153 |
| HSV1-UL19-6555 | - | GCCGCCGGGGGCGUUUACG | 19 | 11154 |
| HSV1-UL19-532 | - | CGCCGCCGGGGGCGUUUACG | 20 | 918 |
| HSV1-UL19-6556 | - | GCGCCGCCGGGGGCGUUUACG | 21 | 11155 |
| HSV1-UL19-6557 | - | CGCGCCGCCGGGGGCGUUUACG | 22 | 11156 |
| HSV1-UL19-6558 | - | ACGCGCCGCCGGGGGCGUUUACG | 23 | 11157 |
| HSV1-UL19-6559 | - | GACGCGCCGCCGGGGGCGUUUACG | 24 | 11158 |
| HSV1-UL19-6560 | - | GGGCAAGGCGGGCCACCG | 18 | 11159 |
| HSV1-UL19-6561 | - | UGGGCAAGGCGGGCCACCG | 19 | 11160 |
| HSV1-UL19-2052 | - | CUGGGCAAGGCGGGCCACCG | 20 | 2396 |
| HSV1-UL19-6562 | - | CCUGGGCAAGGCGGGCCACCG | 21 | 11161 |
| HSV1-UL19-6563 | - | UCCUGGGCAAGGCGGGCCACCG | 22 | 11162 |
| HSV1-UL19-6564 | - | UUCCUGGGCAAGGCGGGCCACCG | 23 | 11163 |
| HSV1-UL19-6565 | - | UUUCCUGGGCAAGGCGGGCCACCG | 24 | 11164 |
| HSV1-UL19-6566 | - | GGAUGACCGGCCGCACCG | 18 | 11165 |
| HSV1-UL19-6567 | - | CGGAUGACCGGCCGCACCG | 19 | 11166 |
| HSV1-UL19-402 | - | GCGGAUGACCGGCCGCACCG | 20 | 788 |
| HSV1-UL19-6568 | - | CGCGGAUGACCGGCCGCACCG | 21 | 11167 |
| HSV1-UL19-6569 | - | CCGCGGAUGACCGGCCGCACCG | 22 | 11168 |
| HSV1-UL19-6570 | - | GCCGCGGAUGACCGGCCGCACCG | 23 | 11169 |
| HSV1-UL19-6571 | - | CGCCGCGGAUGACCGGCCGCACCG | 24 | 11170 |
| HSV1-UL19-6572 | - | GGGGCCGCUGCUGCACCG | 18 | 11171 |
| HSV1-UL19-6573 | - | CGGGGCCGCUGCUGCACCG | 19 | 11172 |
| HSV1-UL19-411 | - | GCGGGGCCGCUGCUGCACCG | 20 | 797 |
| HSV1-UL19-6574 | - | CGCGGGGCCGCUGCUGCACCG | 21 | 11173 |
| HSV1-UL19-6575 | - | UCGCGGGGCCGCUGCUGCACCG | 22 | 11174 |
| HSV1-UL19-6576 | - | CUCGCGGGGCCGCUGCUGCACCG | 23 | 11175 |
| HSV1-UL19-6577 | - | UCUCGCGGGGCCGCUGCUGCACCG | 24 | 11176 |
| HSV1-UL19-6578 | - | GCCUUAACCGCGACCCCG | 18 | 11177 |
| HSV1-UL19-6579 | - | GGCCUUAACCGCGACCCCG | 19 | 11178 |
| HSV1-UL19-2115 | - | GGGCCUUAACCGCGACCCCG | 20 | 2438 |
| HSV1-UL19-6580 | - | GGGGCCUUAACCGCGACCCCG | 21 | 11179 |
| HSV1-UL19-6581 | - | GGGGGCCUUAACCGCGACCCCG | 22 | 11180 |
| HSV1-UL19-6582 | - | CGGGGGCCUUAACCGCGACCCCG | 23 | 11181 |
| HSV1-UL19-6583 | - | UCGGGGGCCUUAACCGCGACCCCG | 24 | 11182 |
| HSV1-UL19-6584 | - | UCCCGGAGAUCGCCCCCG | 18 | 11183 |
| HSV1-UL19-6585 | - | GUCCCGGAGAUCGCCCCCG | 19 | 11184 |
| HSV1-UL19-2209 | - | GGUCCCGGAGAUCGCCCCCG | 20 | 2488 |
| HSV1-UL19-6586 | - | UGGUCCCGGAGAUCGCCCCCG | 21 | 11185 |
| HSV1-UL19-6587 | - | GUGGUCCCGGAGAUCGCCCCCG | 22 | 11186 |
| HSV1-UL19-6588 | - | GGUGGUCCCGGAGAUCGCCCCCG | 23 | 11187 |
| HSV1-UL19-6589 | - | UGGUGGUCCCGGAGAUCGCCCCCG | 24 | 11188 |
| HSV1-UL19-6590 | - | GUUCCCCCGGCCGGCCCG | 18 | 11189 |
| HSV1-UL19-6591 | - | CGUUCCCCCGGCCGGCCCG | 19 | 11190 |
| HSV1-UL19-322 | - | ACGUUCCCCCGGCCGGCCCG | 20 | 708 |
| HSV1-UL19-6592 | - | GACGUUCCCCCGGCCGGCCCG | 21 | 11191 |
| HSV1-UL19-6593 | - | GGACGUUCCCCCGGCCGGCCCG | 22 | 11192 |
| HSV1-UL19-6594 | - | GGGACGUUCCCCCGGCCGGCCCG | 23 | 11193 |
| HSV1-UL19-6595 | - | GGGGACGUUCCCCCGGCCGGCCCG | 24 | 11194 |
| HSV1-UL19-6596 | - | ACCCCCGGGGACGCGCCG | 18 | 11195 |
| HSV1-UL19-6597 | - | AACCCCCGGGGACGCGCCG | 19 | 11196 |
| HSV1-UL19-2280 | - | CAACCCCCGGGGACGCGCCG | 20 | 2535 |
| HSV1-UL19-6598 | - | GCAACCCCCGGGGACGCGCCG | 21 | 11197 |
| HSV1-UL19-6599 | - | UGCAACCCCCGGGGACGCGCCG | 22 | 11198 |
| HSV1-UL19-6600 | - | CUGCAACCCCCGGGGACGCGCCG | 23 | 11199 |
| HSV1-UL19-6601 | - | CCUGCAACCCCCGGGGACGCGCCG | 24 | 11200 |
| HSV1-UL19-6602 | - | GCCACCGCCGGGAGGCCG | 18 | 11201 |
| HSV1-UL19-6603 | - | GGCCACCGCCGGGAGGCCG | 19 | 11202 |
| HSV1-UL19-2055 | - | GGGCCACCGCCGGGAGGCCG | 20 | 2397 |
| HSV1-UL19-6604 | - | CGGGCCACCGCCGGGAGGCCG | 21 | 11203 |
| HSV1-UL19-6605 | - | GCGGGCCACCGCCGGGAGGCCG | 22 | 11204 |
| HSV1-UL19-6606 | - | GGCGGGCCACCGCCGGGAGGCCG | 23 | 11205 |
| HSV1-UL19-6607 | - | AGGCGGGCCACCGCCGGGAGGCCG | 24 | 11206 |
| HSV1-UL19-6608 | - | UCCGCGAGAGCGCGGCCG | 18 | 11207 |
| HSV1-UL19-6609 | - | GUCCGCGAGAGCGCGGCCG | 19 | 11208 |
| HSV1-UL19-462 | - | CGUCCGCGAGAGCGCGGCCG | 20 | 848 |
| HSV1-UL19-6610 | - | ACGUCCGCGAGAGCGCGGCCG | 21 | 11209 |
| HSV1-UL19-6611 | - | CACGUCCGCGAGAGCGCGGCCG | 22 | 11210 |
| HSV1-UL19-6612 | - | GCACGUCCGCGAGAGCGCGGCCG | 23 | 11211 |
| HSV1-UL19-6613 | - | AGCACGUCCGCGAGAGCGCGGCCG | 24 | 11212 |
| HSV1-UL19-6614 | - | AAACUUUUACCUGGGCCG | 18 | 11213 |
| HSV1-UL19-6615 | - | AAAACUUUUACCUGGGCCG | 19 | 11214 |
| HSV1-UL19-501 | - | CAAAACUUUUACCUGGGCCG | 20 | 887 |
| HSV1-UL19-6616 | - | CCAAAACUUUUACCUGGGCCG | 21 | 11215 |
| HSV1-UL19-6617 | - | CCCAAAACUUUUACCUGGGCCG | 22 | 11216 |
| HSV1-UL19-6618 | - | CCCCAAAACUUUUACCUGGGCCG | 23 | 11217 |
| HSV1-UL19-6619 | - | UCCCCAAAACUUUUACCUGGGCCG | 24 | 11218 |
| HSV1-UL19-6620 | - | GCCGACGUGGAGCUGCCG | 18 | 11219 |
| HSV1-UL19-6621 | - | GGCCGACGUGGAGCUGCCG | 19 | 11220 |
| HSV1-UL19-313 | - | UGGCCGACGUGGAGCUGCCG | 20 | 699 |
| HSV1-UL19-6622 | - | GUGGCCGACGUGGAGCUGCCG | 21 | 11221 |
| HSV1-UL19-6623 | - | GGUGGCCGACGUGGAGCUGCCG | 22 | 11222 |
| HSV1-UL19-6624 | - | GGGUGGCCGACGUGGAGCUGCCG | 23 | 11223 |
| HSV1-UL19-6625 | - | GGGGUGGCCGACGUGGAGCUGCCG | 24 | 11224 |
| HSV1-UL19-6626 | - | GCGACUGCCGGGUUAGCG | 18 | 11225 |
| HSV1-UL19-6627 | - | CGCGACUGCCGGGUUAGCG | 19 | 11226 |
| HSV1-UL19-386 | - | UCGCGACUGCCGGGUUAGCG | 20 | 772 |
| HSV1-UL19-6628 | - | AUCGCGACUGCCGGGUUAGCG | 21 | 11227 |
| HSV1-UL19-6629 | - | CAUCGCGACUGCCGGGUUAGCG | 22 | 11228 |
| HSV1-UL19-6630 | - | CCAUCGCGACUGCCGGGUUAGCG | 23 | 11229 |
| HSV1-UL19-6631 | - | GCCAUCGCGACUGCCGGGUUAGCG | 24 | 11230 |
| HSV1-UL19-6632 | - | AACGGGGAAGCCCACGCG | 18 | 11231 |
| HSV1-UL19-6633 | - | CAACGGGGAAGCCCACGCG | 19 | 11232 |
| HSV1-UL19-577 | - | GCAACGGGGAAGCCCACGCG | 20 | 963 |
| HSV1-UL19-6634 | - | CGCAACGGGGAAGCCCACGCG | 21 | 11233 |
| HSV1-UL19-6635 | - | CCGCAACGGGGAAGCCCACGCG | 22 | 11234 |
| HSV1-UL19-6636 | - | CCCGCAACGGGGAAGCCCACGCG | 23 | 11235 |
| HSV1-UL19-6637 | - | GCCCGCAACGGGGAAGCCCACGCG | 24 | 11236 |
| HSV1-UL19-6638 | - | GCCGGGGGCGUUUACGCG | 18 | 11237 |
| HSV1-UL19-6639 | - | CGCCGGGGGCGUUUACGCG | 19 | 11238 |
| HSV1-UL19-534 | - | CCGCCGGGGGCGUUUACGCG | 20 | 920 |
| HSV1-UL19-6640 | - | GCCGCCGGGGGCGUUUACGCG | 21 | 11239 |
| HSV1-UL19-6641 | - | CGCCGCCGGGGGCGUUUACGCG | 22 | 11240 |
| HSV1-UL19-6642 | - | GCGCCGCCGGGGGCGUUUACGCG | 23 | 11241 |
| HSV1-UL19-6643 | - | CGCGCCGCCGGGGGCGUUUACGCG | 24 | 11242 |
| HSV1-UL19-6644 | - | GACUGCCGGGUUAGCGCG | 18 | 11243 |
| HSV1-UL19-6645 | - | CGACUGCCGGGUUAGCGCG | 19 | 11244 |
| HSV1-UL19-388 | - | GCGACUGCCGGGUUAGCGCG | 20 | 774 |
| HSV1-UL19-6646 | - | CGCGACUGCCGGGUUAGCGCG | 21 | 11245 |
| HSV1-UL19-6647 | - | UCGCGACUGCCGGGUUAGCGCG | 22 | 11246 |
| HSV1-UL19-6648 | - | AUCGCGACUGCCGGGUUAGCGCG | 23 | 11247 |
| HSV1-UL19-6649 | - | CAUCGCGACUGCCGGGUUAGCGCG | 24 | 11248 |
| HSV1-UL19-6650 | - | CUGUUCUCGGAGCGCGCG | 18 | 11249 |
| HSV1-UL19-6651 | - | GCUGUUCUCGGAGCGCGCG | 19 | 11250 |
| HSV1-UL19-2246 | - | UGCUGUUCUCGGAGCGCGCG | 20 | 2514 |
| HSV1-UL19-6652 | - | GUGCUGUUCUCGGAGCGCGCG | 21 | 11251 |
| HSV1-UL19-6653 | - | CGUGCUGUUCUCGGAGCGCGCG | 22 | 11252 |
| HSV1-UL19-6654 | - | ACGUGCUGUUCUCGGAGCGCGCG | 23 | 11253 |
| HSV1-UL19-6655 | - | AACGUGCUGUUCUCGGAGCGCGCG | 24 | 11254 |
| HSV1-UL19-6656 | - | CCCAGGUGCCGCGGCGCG | 18 | 11255 |
| HSV1-UL19-6657 | - | GCCCAGGUGCCGCGGCGCG | 19 | 11256 |
| HSV1-UL19-2272 | - | CGCCCAGGUGCCGCGGCGCG | 20 | 2530 |
| HSV1-UL19-6658 | - | GCGCCCAGGUGCCGCGGCGCG | 21 | 11257 |
| HSV1-UL19-6659 | - | UGCGCCCAGGUGCCGCGGCGCG | 22 | 11258 |
| HSV1-UL19-6660 | - | GUGCGCCCAGGUGCCGCGGCGCG | 23 | 11259 |
| HSV1-UL19-6661 | - | GGUGCGCCCAGGUGCCGCGGCGCG | 24 | 11260 |
| HSV1-UL19-6662 | - | CCCGGCACGAAACUGGCG | 18 | 11261 |
| HSV1-UL19-6663 | - | GCCCGGCACGAAACUGGCG | 19 | 11262 |
| HSV1-UL19-486 | - | GGCCCGGCACGAAACUGGCG | 20 | 872 |
| HSV1-UL19-6664 | - | UGGCCCGGCACGAAACUGGCG | 21 | 11263 |
| HSV1-UL19-6665 | - | GUGGCCCGGCACGAAACUGGCG | 22 | 11264 |
| HSV1-UL19-6666 | - | GGUGGCCCGGCACGAAACUGGCG | 23 | 11265 |
| HSV1-UL19-6667 | - | AGGUGGCCCGGCACGAAACUGGCG | 24 | 11266 |
| HSV1-UL19-6668 | - | CGGAUCUGGUGUCCAUCG | 18 | 11267 |
| HSV1-UL19-6669 | - | GCGGAUCUGGUGUCCAUCG | 19 | 11268 |
| HSV1-UL19-2085 | - | CGCGGAUCUGGUGUCCAUCG | 20 | 2421 |
| HSV1-UL19-6670 | - | GCGCGGAUCUGGUGUCCAUCG | 21 | 11269 |
| HSV1-UL19-6671 | - | CGCGCGGAUCUGGUGUCCAUCG | 22 | 11270 |
| HSV1-UL19-6672 | - | GCGCGCGGAUCUGGUGUCCAUCG | 23 | 11271 |
| HSV1-UL19-6673 | - | UGCGCGCGGAUCUGGUGUCCAUCG | 24 | 11272 |
| HSV1-UL19-6674 | - | GCCUGGAGCGCCUGAUCG | 18 | 11273 |
| HSV1-UL19-6675 | - | UGCCUGGAGCGCCUGAUCG | 19 | 11274 |
| HSV1-UL19-560 | - | CUGCCUGGAGCGCCUGAUCG | 20 | 946 |
| HSV1-UL19-6676 | - | GCUGCCUGGAGCGCCUGAUCG | 21 | 11275 |
| HSV1-UL19-6677 | - | CGCUGCCUGGAGCGCCUGAUCG | 22 | 11276 |
| HSV1-UL19-6678 | - | UCGCUGCCUGGAGCGCCUGAUCG | 23 | 11277 |
| HSV1-UL19-6679 | - | AUCGCUGCCUGGAGCGCCUGAUCG | 24 | 11278 |
| HSV1-UL19-6680 | - | CGGGGUGCCGCGAACUCG | 18 | 11279 |
| HSV1-UL19-6681 | - | CCGGGGUGCCGCGAACUCG | 19 | 11280 |
| HSV1-UL19-569 | - | CCCGGGGUGCCGCGAACUCG | 20 | 955 |
| HSV1-UL19-6682 | - | CCCCGGGGUGCCGCGAACUCG | 21 | 11281 |
| HSV1-UL19-6683 | - | CCCCCGGGGUGCCGCGAACUCG | 22 | 11282 |
| HSV1-UL19-6684 | - | CCCCCCGGGGUGCCGCGAACUCG | 23 | 11283 |
| HSV1-UL19-6685 | - | GCCCCCCGGGGUGCCGCGAACUCG | 24 | 11284 |
| HSV1-UL19-6686 | - | ACGUCGUGACCUACCUCG | 18 | 11285 |
| HSV1-UL19-6687 | - | UACGUCGUGACCUACCUCG | 19 | 11286 |
| HSV1-UL19-360 | - | GUACGUCGUGACCUACCUCG | 20 | 746 |
| HSV1-UL19-6688 | - | CGUACGUCGUGACCUACCUCG | 21 | 11287 |
| HSV1-UL19-6689 | - | UCGUACGUCGUGACCUACCUCG | 22 | 11288 |
| HSV1-UL19-6690 | - | CUCGUACGUCGUGACCUACCUCG | 23 | 11289 |
| HSV1-UL19-6691 | - | UCUCGUACGUCGUGACCUACCUCG | 24 | 11290 |
| HSV1-UL19-6692 | - | AUGGUGCCCGCCUUCUCG | 18 | 11291 |
| HSV1-UL19-6693 | - | GAUGGUGCCCGCCUUCUCG | 19 | 11292 |
| HSV1-UL19-408 | - | UGAUGGUGCCCGCCUUCUCG | 20 | 794 |
| HSV1-UL19-6694 | - | GUGAUGGUGCCCGCCUUCUCG | 21 | 11293 |
| HSV1-UL19-6695 | - | CGUGAUGGUGCCCGCCUUCUCG | 22 | 11294 |
| HSV1-UL19-6696 | - | ACGUGAUGGUGCCCGCCUUCUCG | 23 | 11295 |
| HSV1-UL19-6697 | - | UACGUGAUGGUGCCCGCCUUCUCG | 24 | 11296 |
| HSV1-UL19-6698 | - | CGCGCGGGCGGCCGGUCG | 18 | 11297 |
| HSV1-UL19-6699 | - | ACGCGCGGGCGGCCGGUCG | 19 | 11298 |
| HSV1-UL19-2058 | - | CACGCGCGGGCGGCCGGUCG | 20 | 2400 |
| HSV1-UL19-6700 | - | ACACGCGCGGGCGGCCGGUCG | 21 | 11299 |
| HSV1-UL19-6701 | - | GACACGCGCGGGCGGCCGGUCG | 22 | 11300 |
| HSV1-UL19-6702 | - | CGACACGCGCGGGCGGCCGGUCG | 23 | 11301 |
| HSV1-UL19-6703 | - | CCGACACGCGCGGGCGGCCGGUCG | 24 | 11302 |
| HSV1-UL19-6704 | - | GGCGGGUCCGAUGGGUCG | 18 | 11303 |
| HSV1-UL19-6705 | - | GGGCGGGUCCGAUGGGUCG | 19 | 11304 |
| HSV1-UL19-301 | - | CGGGCGGGUCCGAUGGGUCG | 20 | 687 |
| HSV1-UL19-6706 | - | ACGGGCGGGUCCGAUGGGUCG | 21 | 11305 |
| HSV1-UL19-6707 | - | CACGGGCGGGUCCGAUGGGUCG | 22 | 11306 |
| HSV1-UL19-6708 | - | CCACGGGCGGGUCCGAUGGGUCG | 23 | 11307 |
| HSV1-UL19-6709 | - | CCCACGGGCGGGUCCGAUGGGUCG | 24 | 11308 |
| HSV1-UL19-6710 | - | CCCGGGCGACCCUGGUCG | 18 | 11309 |
| HSV1-UL19-6711 | - | GCCCGGGCGACCCUGGUCG | 19 | 11310 |
| HSV1-UL19-2046 | - | GGCCCGGGCGACCCUGGUCG | 20 | 2390 |
| HSV1-UL19-6712 | - | UGGCCCGGGCGACCCUGGUCG | 21 | 11311 |
| HSV1-UL19-6713 | - | GUGGCCCGGGCGACCCUGGUCG | 22 | 11312 |
| HSV1-UL19-6714 | - | GGUGGCCCGGGCGACCCUGGUCG | 23 | 11313 |
| HSV1-UL19-6715 | - | GGGUGGCCCGGGCGACCCUGGUCG | 24 | 11314 |
| HSV1-UL19-6716 | - | CCAACAUGCGCAUAUUCG | 18 | 11315 |
| HSV1-UL19-6717 | - | ACCAACAUGCGCAUAUUCG | 19 | 11316 |
| HSV1-UL19-2218 | - | CACCAACAUGCGCAUAUUCG | 20 | 2496 |
| HSV1-UL19-6718 | - | CCACCAACAUGCGCAUAUUCG | 21 | 11317 |
| HSV1-UL19-6719 | - | ACCACCAACAUGCGCAUAUUCG | 22 | 11318 |
| HSV1-UL19-6720 | - | CACCACCAACAUGCGCAUAUUCG | 23 | 11319 |
| HSV1-UL19-6721 | - | CCACCACCAACAUGCGCAUAUUCG | 24 | 11320 |
| HSV1-UL19-6722 | - | CACCAUCGCCGCCGUUCG | 18 | 11321 |
| HSV1-UL19-6723 | - | CCACCAUCGCCGCCGUUCG | 19 | 11322 |
| HSV1-UL19-344 | - | GCCACCAUCGCCGCCGUUCG | 20 | 730 |
| HSV1-UL19-6724 | - | CGCCACCAUCGCCGCCGUUCG | 21 | 11323 |
| HSV1-UL19-6725 | - | CCGCCACCAUCGCCGCCGUUCG | 22 | 11324 |
| HSV1-UL19-6726 | - | CCCGCCACCAUCGCCGCCGUUCG | 23 | 11325 |
| HSV1-UL19-6727 | - | CCCCGCCACCAUCGCCGCCGUUCG | 24 | 11326 |
| HSV1-UL19-6728 | - | CCGUCCACGCGCUGUUCG | 18 | 11327 |
| HSV1-UL19-6729 | - | CCCGUCCACGCGCUGUUCG | 19 | 11328 |
| HSV1-UL19-2223 | - | CCCCGUCCACGCGCUGUUCG | 20 | 2499 |
| HSV1-UL19-6730 | - | UCCCCGUCCACGCGCUGUUCG | 21 | 11329 |
| HSV1-UL19-6731 | - | CUCCCCGUCCACGCGCUGUUCG | 22 | 11330 |
| HSV1-UL19-6732 | - | CCUCCCCGUCCACGCGCUGUUCG | 23 | 11331 |
| HSV1-UL19-6733 | - | CCCUCCCCGUCCACGCGCUGUUCG | 24 | 11332 |
| HSV1-UL19-6734 | - | UUUACGCGGGGGACAAGG | 18 | 11333 |
| HSV1-UL19-6735 | - | GUUUACGCGGGGGACAAGG | 19 | 11334 |
| HSV1-UL19-537 | - | CGUUUACGCGGGGGACAAGG | 20 | 923 |
| HSV1-UL19-6736 | - | GCGUUUACGCGGGGGACAAGG | 21 | 11335 |
| HSV1-UL19-6737 | - | GGCGUUUACGCGGGGGACAAGG | 22 | 11336 |
| HSV1-UL19-6738 | - | GGGCGUUUACGCGGGGGACAAGG | 23 | 11337 |
| HSV1-UL19-6739 | - | GGGGCGUUUACGCGGGGGACAAGG | 24 | 11338 |
| HSV1-UL19-6740 | - | ACCUGCUGGAGAUGCAGG | 18 | 11339 |
| HSV1-UL19-6741 | - | CACCUGCUGGAGAUGCAGG | 19 | 11340 |
| HSV1-UL19-244 | - | CCACCUGCUGGAGAUGCAGG | 20 | 630 |
| HSV1-UL19-6742 | - | GCCACCUGCUGGAGAUGCAGG | 21 | 11341 |
| HSV1-UL19-6743 | - | CGCCACCUGCUGGAGAUGCAGG | 22 | 11342 |
| HSV1-UL19-6744 | - | CCGCCACCUGCUGGAGAUGCAGG | 23 | 11343 |
| HSV1-UL19-6745 | - | GCCGCCACCUGCUGGAGAUGCAGG | 24 | 11344 |
| HSV1-UL19-6746 | - | UUCCCCCGGCCGGCCCGG | 18 | 11345 |
| HSV1-UL19-6747 | - | GUUCCCCCGGCCGGCCCGG | 19 | 11346 |
| HSV1-UL19-323 | - | CGUUCCCCCGGCCGGCCCGG | 20 | 709 |
| HSV1-UL19-6748 | - | ACGUUCCCCCGGCCGGCCCGG | 21 | 11347 |
| HSV1-UL19-6749 | - | GACGUUCCCCCGGCCGGCCCGG | 22 | 11348 |
| HSV1-UL19-6750 | - | GGACGUUCCCCCGGCCGGCCCGG | 23 | 11349 |
| HSV1-UL19-6751 | - | GGGACGUUCCCCCGGCCGGCCCGG | 24 | 11350 |
| HSV1-UL19-6752 | - | CCGACGUGGAGCUGCCGG | 18 | 11351 |
| HSV1-UL19-6753 | - | GCCGACGUGGAGCUGCCGG | 19 | 11352 |
| HSV1-UL19-314 | - | GGCCGACGUGGAGCUGCCGG | 20 | 700 |
| HSV1-UL19-6754 | - | UGGCCGACGUGGAGCUGCCGG | 21 | 11353 |
| HSV1-UL19-6755 | - | GUGGCCGACGUGGAGCUGCCGG | 22 | 11354 |
| HSV1-UL19-6756 | - | GGUGGCCGACGUGGAGCUGCCGG | 23 | 11355 |
| HSV1-UL19-6757 | - | GGGUGGCCGACGUGGAGCUGCCGG | 24 | 11356 |
| HSV1-UL19-6758 | - | ACGUCCGCGAGAGCGCGG | 18 | 11357 |
| HSV1-UL19-6759 | - | CACGUCCGCGAGAGCGCGG | 19 | 11358 |
| HSV1-UL19-2231 | - | GCACGUCCGCGAGAGCGCGG | 20 | 2504 |
| HSV1-UL19-6760 | - | AGCACGUCCGCGAGAGCGCGG | 21 | 11359 |
| HSV1-UL19-6761 | - | CAGCACGUCCGCGAGAGCGCGG | 22 | 11360 |
| HSV1-UL19-6762 | - | GCAGCACGUCCGCGAGAGCGCGG | 23 | 11361 |
| HSV1-UL19-6763 | - | UGCAGCACGUCCGCGAGAGCGCGG | 24 | 11362 |
| HSV1-UL19-6764 | - | UGAACGUUGACGCGGCGG | 18 | 11363 |
| HSV1-UL19-6765 | - | AUGAACGUUGACGCGGCGG | 19 | 11364 |
| HSV1-UL19-2112 | - | GAUGAACGUUGACGCGGCGG | 20 | 2437 |
| HSV1-UL19-6766 | - | UGAUGAACGUUGACGCGGCGG | 21 | 11365 |
| HSV1-UL19-6767 | - | CUGAUGAACGUUGACGCGGCGG | 22 | 11366 |
| HSV1-UL19-6768 | - | GCUGAUGAACGUUGACGCGGCGG | 23 | 11367 |
| HSV1-UL19-6769 | - | CGCUGAUGAACGUUGACGCGGCGG | 24 | 11368 |
| HSV1-UL19-6770 | - | CGUCGUGACCUACCUCGG | 18 | 11369 |
| HSV1-UL19-6771 | - | ACGUCGUGACCUACCUCGG | 19 | 11370 |
| HSV1-UL19-361 | - | UACGUCGUGACCUACCUCGG | 20 | 747 |
| HSV1-UL19-6772 | - | GUACGUCGUGACCUACCUCGG | 21 | 11371 |
| HSV1-UL19-6773 | - | CGUACGUCGUGACCUACCUCGG | 22 | 11372 |
| HSV1-UL19-6774 | - | UCGUACGUCGUGACCUACCUCGG | 23 | 11373 |
| HSV1-UL19-6775 | - | CUCGUACGUCGUGACCUACCUCGG | 24 | 11374 |
| HSV1-UL19-6776 | - | GUGCCGCGGCGCGCAGGG | 18 | 11375 |
| HSV1-UL19-6777 | - | GGUGCCGCGGCGCGCAGGG | 19 | 11376 |
| HSV1-UL19-2274 | - | AGGUGCCGCGGCGCGCAGGG | 20 | 2531 |
| HSV1-UL19-6778 | - | CAGGUGCCGCGGCGCGCAGGG | 21 | 11377 |
| HSV1-UL19-6779 | - | CCAGGUGCCGCGGCGCGCAGGG | 22 | 11378 |
| HSV1-UL19-6780 | - | CCCAGGUGCCGCGGCGCGCAGGG | 23 | 11379 |
| HSV1-UL19-6781 | - | GCCCAGGUGCCGCGGCGCGCAGGG | 24 | 11380 |
| HSV1-UL19-6782 | - | CGCGAGAGCGCGGCCGGG | 18 | 11381 |
| HSV1-UL19-6783 | - | CCGCGAGAGCGCGGCCGGG | 19 | 11382 |
| HSV1-UL19-2235 | - | UCCGCGAGAGCGCGGCCGGG | 20 | 2505 |
| HSV1-UL19-6784 | - | GUCCGCGAGAGCGCGGCCGGG | 21 | 11383 |
| HSV1-UL19-6785 | - | CGUCCGCGAGAGCGCGGCCGGG | 22 | 11384 |
| HSV1-UL19-6786 | - | ACGUCCGCGAGAGCGCGGCCGGG | 23 | 11385 |
| HSV1-UL19-6787 | - | CACGUCCGCGAGAGCGCGGCCGGG | 24 | 11386 |
| HSV1-UL19-6788 | - | CGACGUGGAGCUGCCGGG | 18 | 11387 |
| HSV1-UL19-6789 | - | CCGACGUGGAGCUGCCGGG | 19 | 11388 |
| HSV1-UL19-315 | - | GCCGACGUGGAGCUGCCGGG | 20 | 701 |
| HSV1-UL19-6790 | - | GGCCGACGUGGAGCUGCCGGG | 21 | 11389 |
| HSV1-UL19-6791 | - | UGGCCGACGUGGAGCUGCCGGG | 22 | 11390 |
| HSV1-UL19-6792 | - | GUGGCCGACGUGGAGCUGCCGGG | 23 | 11391 |
| HSV1-UL19-6793 | - | GGUGGCCGACGUGGAGCUGCCGGG | 24 | 11392 |
| HSV1-UL19-6794 | - | GACGUGGAGCUGCCGGGG | 18 | 11393 |
| HSV1-UL19-6795 | - | CGACGUGGAGCUGCCGGGG | 19 | 11394 |
| HSV1-UL19-316 | - | CCGACGUGGAGCUGCCGGGG | 20 | 702 |
| HSV1-UL19-6796 | - | GCCGACGUGGAGCUGCCGGGG | 21 | 11395 |
| HSV1-UL19-6797 | - | GGCCGACGUGGAGCUGCCGGGG | 22 | 11396 |
| HSV1-UL19-6798 | - | UGGCCGACGUGGAGCUGCCGGGG | 23 | 11397 |
| HSV1-UL19-6799 | - | GUGGCCGACGUGGAGCUGCCGGGG | 24 | 11398 |
| HSV1-UL19-6800 | - | GACCGGCCGCACCGGGGG | 18 | 11399 |
| HSV1-UL19-6801 | - | UGACCGGCCGCACCGGGGG | 19 | 11400 |
| HSV1-UL19-2199 | - | AUGACCGGCCGCACCGGGGG | 20 | 2482 |
| HSV1-UL19-6802 | - | GAUGACCGGCCGCACCGGGGG | 21 | 11401 |
| HSV1-UL19-6803 | - | GGAUGACCGGCCGCACCGGGGG | 22 | 11402 |
| HSV1-UL19-6804 | - | CGGAUGACCGGCCGCACCGGGGG | 23 | 11403 |
| HSV1-UL19-6805 | - | GCGGAUGACCGGCCGCACCGGGGG | 24 | 11404 |
| HSV1-UL19-6806 | - | GGGGCCUGGAGCUGGGGG | 18 | 11405 |
| HSV1-UL19-6807 | - | CGGGGCCUGGAGCUGGGGG | 19 | 11406 |
| HSV1-UL19-341 | - | CCGGGGCCUGGAGCUGGGGG | 20 | 727 |
| HSV1-UL19-6808 | - | CCCGGGGCCUGGAGCUGGGGG | 21 | 11407 |
| HSV1-UL19-6809 | - | GCCCGGGGCCUGGAGCUGGGGG | 22 | 11408 |
| HSV1-UL19-6810 | - | CGCCCGGGGCCUGGAGCUGGGGG | 23 | 11409 |
| HSV1-UL19-6811 | - | ACGCCCGGGGCCUGGAGCUGGGGG | 24 | 11410 |
| HSV1-UL19-6812 | - | CAGCUGUUUUUUUGGGGG | 18 | 11411 |
| HSV1-UL19-6813 | - | CCAGCUGUUUUUUUGGGGG | 19 | 11412 |
| HSV1-UL19-2106 | - | GCCAGCUGUUUUUUUGGGGG | 20 | 2433 |
| HSV1-UL19-6814 | - | CGCCAGCUGUUUUUUUGGGGG | 21 | 11413 |
| HSV1-UL19-6815 | - | CCGCCAGCUGUUUUUUUGGGGG | 22 | 11414 |
| HSV1-UL19-6816 | - | CCCGCCAGCUGUUUUUUUGGGGG | 23 | 11415 |
| HSV1-UL19-6817 | - | CCCCGCCAGCUGUUUUUUUGGGGG | 24 | 11416 |
| HSV1-UL19-6818 | - | CGGGGCCUGGAGCUGGGG | 18 | 11417 |
| HSV1-UL19-6819 | - | CCGGGGCCUGGAGCUGGGG | 19 | 11418 |
| HSV1-UL19-2157 | - | CCCGGGGCCUGGAGCUGGGG | 20 | 2457 |
| HSV1-UL19-6820 | - | GCCCGGGGCCUGGAGCUGGGG | 21 | 11419 |
| HSV1-UL19-6821 | - | CGCCCGGGGCCUGGAGCUGGGG | 22 | 11420 |
| HSV1-UL19-6822 | - | ACGCCCGGGGCCUGGAGCUGGGG | 23 | 11421 |
| HSV1-UL19-6823 | - | GACGCCCGGGGCCUGGAGCUGGGG | 24 | 11422 |
| HSV1-UL19-6824 | - | UGCUGGCCCACAACAUGG | 18 | 11423 |
| HSV1-UL19-6825 | - | GUGCUGGCCCACAACAUGG | 19 | 11424 |
| HSV1-UL19-2216 | - | GGUGCUGGCCCACAACAUGG | 20 | 2494 |
| HSV1-UL19-6826 | - | AGGUGCUGGCCCACAACAUGG | 21 | 11425 |
| HSV1-UL19-6827 | - | CAGGUGCUGGCCCACAACAUGG | 22 | 11426 |
| HSV1-UL19-6828 | - | GCAGGUGCUGGCCCACAACAUGG | 23 | 11427 |
| HSV1-UL19-6829 | - | UGCAGGUGCUGGCCCACAACAUGG | 24 | 11428 |
| HSV1-UL19-6830 | - | GGCCCGGCACGAAACUGG | 18 | 11429 |
| HSV1-UL19-6831 | - | UGGCCCGGCACGAAACUGG | 19 | 11430 |
| HSV1-UL19-484 | - | GUGGCCCGGCACGAAACUGG | 20 | 870 |
| HSV1-UL19-6832 | - | GGUGGCCCGGCACGAAACUGG | 21 | 11431 |
| HSV1-UL19-6833 | - | AGGUGGCCCGGCACGAAACUGG | 22 | 11432 |
| HSV1-UL19-6834 | - | CAGGUGGCCCGGCACGAAACUGG | 23 | 11433 |
| HSV1-UL19-6835 | - | CCAGGUGGCCCGGCACGAAACUGG | 24 | 11434 |
| HSV1-UL19-6836 | - | UGACCGGCCCGGAGCUGG | 18 | 11435 |
| HSV1-UL19-6837 | - | CUGACCGGCCCGGAGCUGG | 19 | 11436 |
| HSV1-UL19-2179 | - | CCUGACCGGCCCGGAGCUGG | 20 | 2469 |
| HSV1-UL19-6838 | - | CCCUGACCGGCCCGGAGCUGG | 21 | 11437 |
| HSV1-UL19-6839 | - | ACCCUGACCGGCCCGGAGCUGG | 22 | 11438 |
| HSV1-UL19-6840 | - | UACCCUGACCGGCCCGGAGCUGG | 23 | 11439 |
| HSV1-UL19-6841 | - | UUACCCUGACCGGCCCGGAGCUGG | 24 | 11440 |
| HSV1-UL19-6842 | - | UGAACCGGCAGACGCUGG | 18 | 11441 |
| HSV1-UL19-6843 | - | CUGAACCGGCAGACGCUGG | 19 | 11442 |
| HSV1-UL19-2081 | - | CCUGAACCGGCAGACGCUGG | 20 | 2417 |
| HSV1-UL19-6844 | - | ACCUGAACCGGCAGACGCUGG | 21 | 11443 |
| HSV1-UL19-6845 | - | GACCUGAACCGGCAGACGCUGG | 22 | 11444 |
| HSV1-UL19-6846 | - | CGACCUGAACCGGCAGACGCUGG | 23 | 11445 |
| HSV1-UL19-6847 | - | UCGACCUGAACCGGCAGACGCUGG | 24 | 11446 |
| HSV1-UL19-6848 | - | ACGGGCGCGUGGUAGUGG | 18 | 11447 |
| HSV1-UL19-6849 | - | AACGGGCGCGUGGUAGUGG | 19 | 11448 |
| HSV1-UL19-2215 | - | CAACGGGCGCGUGGUAGUGG | 20 | 2493 |
| HSV1-UL19-6850 | - | ACAACGGGCGCGUGGUAGUGG | 21 | 11449 |
| HSV1-UL19-6851 | - | CACAACGGGCGCGUGGUAGUGG | 22 | 11450 |
| HSV1-UL19-6852 | - | UCACAACGGGCGCGUGGUAGUGG | 23 | 11451 |
| HSV1-UL19-6853 | - | UUCACAACGGGCGCGUGGUAGUGG | 24 | 11452 |
| HSV1-UL19-6854 | - | UGCGGAACGCGGUCGUGG | 18 | 11453 |
| HSV1-UL19-6855 | - | CUGCGGAACGCGGUCGUGG | 19 | 11454 |
| HSV1-UL19-509 | - | CCUGCGGAACGCGGUCGUGG | 20 | 895 |
| HSV1-UL19-6856 | - | ACCUGCGGAACGCGGUCGUGG | 21 | 11455 |
| HSV1-UL19-6857 | - | UACCUGCGGAACGCGGUCGUGG | 22 | 11456 |
| HSV1-UL19-6858 | - | GUACCUGCGGAACGCGGUCGUGG | 23 | 11457 |
| HSV1-UL19-6859 | - | UGUACCUGCGGAACGCGGUCGUGG | 24 | 11458 |
| HSV1-UL19-6860 | - | CGCCAGCUGUUUUUUUGG | 18 | 11459 |
| HSV1-UL19-6861 | - | CCGCCAGCUGUUUUUUUGG | 19 | 11460 |
| HSV1-UL19-271 | - | CCCGCCAGCUGUUUUUUUGG | 20 | 657 |
| HSV1-UL19-6862 | - | CCCCGCCAGCUGUUUUUUUGG | 21 | 11461 |
| HSV1-UL19-6863 | - | GCCCCGCCAGCUGUUUUUUUGG | 22 | 11462 |
| HSV1-UL19-6864 | - | CGCCCCGCCAGCUGUUUUUUUGG | 23 | 11463 |
| HSV1-UL19-6865 | - | CCGCCCCGCCAGCUGUUUUUUUGG | 24 | 11464 |
| HSV1-UL19-6866 | - | CUGACCUACGCGCUCAUG | 18 | 11465 |
| HSV1-UL19-6867 | - | GCUGACCUACGCGCUCAUG | 19 | 11466 |
| HSV1-UL19-2236 | - | CGCUGACCUACGCGCUCAUG | 20 | 2506 |
| HSV1-UL19-6868 | - | GCGCUGACCUACGCGCUCAUG | 21 | 11467 |
| HSV1-UL19-6869 | - | CGCGCUGACCUACGCGCUCAUG | 22 | 11468 |
| HSV1-UL19-6870 | - | ACGCGCUGACCUACGCGCUCAUG | 23 | 11469 |
| HSV1-UL19-6871 | - | AACGCGCUGACCUACGCGCUCAUG | 24 | 11470 |
| HSV1-UL19-6872 | - | CGCCACCUGCUGGAGAUG | 18 | 11471 |
| HSV1-UL19-6873 | - | CCGCCACCUGCUGGAGAUG | 19 | 11472 |
| HSV1-UL19-2075 | - | GCCGCCACCUGCUGGAGAUG | 20 | 2413 |
| HSV1-UL19-6874 | - | GGCCGCCACCUGCUGGAGAUG | 21 | 11473 |
| HSV1-UL19-6875 | - | GGGCCGCCACCUGCUGGAGAUG | 22 | 11474 |
| HSV1-UL19-6876 | - | UGGGCCGCCACCUGCUGGAGAUG | 23 | 11475 |
| HSV1-UL19-6877 | - | GUGGGCCGCCACCUGCUGGAGAUG | 24 | 11476 |
| HSV1-UL19-6878 | - | UGGCCCGGCACGAAACUG | 18 | 11477 |
| HSV1-UL19-6879 | - | GUGGCCCGGCACGAAACUG | 19 | 11478 |
| HSV1-UL19-2250 | - | GGUGGCCCGGCACGAAACUG | 20 | 2517 |
| HSV1-UL19-6880 | - | AGGUGGCCCGGCACGAAACUG | 21 | 11479 |
| HSV1-UL19-6881 | - | CAGGUGGCCCGGCACGAAACUG | 22 | 11480 |
| HSV1-UL19-6882 | - | CCAGGUGGCCCGGCACGAAACUG | 23 | 11481 |
| HSV1-UL19-6883 | - | UCCAGGUGGCCCGGCACGAAACUG | 24 | 11482 |
| HSV1-UL19-6884 | - | GACGUGGGCCGCCACCUG | 18 | 11483 |
| HSV1-UL19-6885 | - | CGACGUGGGCCGCCACCUG | 19 | 11484 |
| HSV1-UL19-2073 | - | ACGACGUGGGCCGCCACCUG | 20 | 2412 |
| HSV1-UL19-6886 | - | GACGACGUGGGCCGCCACCUG | 21 | 11485 |
| HSV1-UL19-6887 | - | GGACGACGUGGGCCGCCACCUG | 22 | 11486 |
| HSV1-UL19-6888 | - | UGGACGACGUGGGCCGCCACCUG | 23 | 11487 |
| HSV1-UL19-6889 | - | CUGGACGACGUGGGCCGCCACCUG | 24 | 11488 |
| HSV1-UL19-6890 | - | GCGGCAGCCGUGUACCUG | 18 | 11489 |
| HSV1-UL19-6891 | - | CGCGGCAGCCGUGUACCUG | 19 | 11490 |
| HSV1-UL19-506 | - | ACGCGGCAGCCGUGUACCUG | 20 | 892 |
| HSV1-UL19-6892 | - | AACGCGGCAGCCGUGUACCUG | 21 | 11491 |
| HSV1-UL19-6893 | - | CAACGCGGCAGCCGUGUACCUG | 22 | 11492 |
| HSV1-UL19-6894 | - | ACAACGCGGCAGCCGUGUACCUG | 23 | 11493 |
| HSV1-UL19-6895 | - | GACAACGCGGCAGCCGUGUACCUG | 24 | 11494 |
| HSV1-UL19-6896 | - | CGCGUAUACGCCACCCUG | 18 | 11495 |
| HSV1-UL19-6897 | - | CCGCGUAUACGCCACCCUG | 19 | 11496 |
| HSV1-UL19-2206 | - | ACCGCGUAUACGCCACCCUG | 20 | 2486 |
| HSV1-UL19-6898 | - | GACCGCGUAUACGCCACCCUG | 21 | 11497 |
| HSV1-UL19-6899 | - | CGACCGCGUAUACGCCACCCUG | 22 | 11498 |
| HSV1-UL19-6900 | - | UCGACCGCGUAUACGCCACCCUG | 23 | 11499 |
| HSV1-UL19-6901 | - | UUCGACCGCGUAUACGCCACCCUG | 24 | 11500 |
| HSV1-UL19-6902 | - | AACGUUCCCUACCCCCUG | 18 | 11501 |
| HSV1-UL19-6903 | - | CAACGUUCCCUACCCCCUG | 19 | 11502 |
| HSV1-UL19-2092 | - | CCAACGUUCCCUACCCCCUG | 20 | 2426 |
| HSV1-UL19-6904 | - | ACCAACGUUCCCUACCCCCUG | 21 | 11503 |
| HSV1-UL19-6905 | - | CACCAACGUUCCCUACCCCCUG | 22 | 11504 |
| HSV1-UL19-6906 | - | CCACCAACGUUCCCUACCCCCUG | 23 | 11505 |
| HSV1-UL19-6907 | - | GCCACCAACGUUCCCUACCCCCUG | 24 | 11506 |
| HSV1-UL19-6908 | - | GUCUUUCUGGAGGCCCUG | 18 | 11507 |
| HSV1-UL19-6909 | - | GGUCUUUCUGGAGGCCCUG | 19 | 11508 |
| HSV1-UL19-2091 | - | UGGUCUUUCUGGAGGCCCUG | 20 | 2425 |
| HSV1-UL19-6910 | - | CUGGUCUUUCUGGAGGCCCUG | 21 | 11509 |
| HSV1-UL19-6911 | - | GCUGGUCUUUCUGGAGGCCCUG | 22 | 11510 |
| HSV1-UL19-6912 | - | AGCUGGUCUUUCUGGAGGCCCUG | 23 | 11511 |
| HSV1-UL19-6913 | - | AAGCUGGUCUUUCUGGAGGCCCUG | 24 | 11512 |
| HSV1-UL19-6914 | - | GUGGCCGACGUGGAGCUG | 18 | 11513 |
| HSV1-UL19-6915 | - | GGUGGCCGACGUGGAGCUG | 19 | 11514 |
| HSV1-UL19-2135 | - | GGGUGGCCGACGUGGAGCUG | 20 | 2450 |
| HSV1-UL19-6916 | - | GGGGUGGCCGACGUGGAGCUG | 21 | 11515 |
| HSV1-UL19-6917 | - | GGGGGUGGCCGACGUGGAGCUG | 22 | 11516 |
| HSV1-UL19-6918 | - | UGGGGGUGGCCGACGUGGAGCUG | 23 | 11517 |
| HSV1-UL19-6919 | - | GUGGGGGUGGCCGACGUGGAGCUG | 24 | 11518 |
| HSV1-UL19-6920 | - | GUGUGCCACCCUUCGCUG | 18 | 11519 |
| HSV1-UL19-6921 | - | CGUGUGCCACCCUUCGCUG | 19 | 11520 |
| HSV1-UL19-2111 | - | CCGUGUGCCACCCUUCGCUG | 20 | 2436 |
| HSV1-UL19-6922 | - | ACCGUGUGCCACCCUUCGCUG | 21 | 11521 |
| HSV1-UL19-6923 | - | GACCGUGUGCCACCCUUCGCUG | 22 | 11522 |
| HSV1-UL19-6924 | - | GGACCGUGUGCCACCCUUCGCUG | 23 | 11523 |
| HSV1-UL19-6925 | - | GGGACCGUGUGCCACCCUUCGCUG | 24 | 11524 |
| HSV1-UL19-6926 | - | CAGAUGCUGCACGUGCUG | 18 | 11525 |
| HSV1-UL19-6927 | - | CCAGAUGCUGCACGUGCUG | 19 | 11526 |
| HSV1-UL19-2041 | - | ACCAGAUGCUGCACGUGCUG | 20 | 2386 |
| HSV1-UL19-6928 | - | GACCAGAUGCUGCACGUGCUG | 21 | 11527 |
| HSV1-UL19-6929 | - | CGACCAGAUGCUGCACGUGCUG | 22 | 11528 |
| HSV1-UL19-6930 | - | CCGACCAGAUGCUGCACGUGCUG | 23 | 11529 |
| HSV1-UL19-6931 | - | GCCGACCAGAUGCUGCACGUGCUG | 24 | 11530 |
| HSV1-UL19-6932 | - | CAGUCCUUCCUGAAAGUG | 18 | 11531 |
| HSV1-UL19-6933 | - | GCAGUCCUUCCUGAAAGUG | 19 | 11532 |
| HSV1-UL19-2063 | - | UGCAGUCCUUCCUGAAAGUG | 20 | 2403 |
| HSV1-UL19-6934 | - | CUGCAGUCCUUCCUGAAAGUG | 21 | 11533 |
| HSV1-UL19-6935 | - | CCUGCAGUCCUUCCUGAAAGUG | 22 | 11534 |
| HSV1-UL19-6936 | - | UCCUGCAGUCCUUCCUGAAAGUG | 23 | 11535 |
| HSV1-UL19-6937 | - | CUCCUGCAGUCCUUCCUGAAAGUG | 24 | 11536 |
| HSV1-UL19-6938 | - | GCCGGGGCCGACCACGUG | 18 | 11537 |
| HSV1-UL19-6939 | - | CGCCGGGGCCGACCACGUG | 19 | 11538 |
| HSV1-UL19-2225 | - | UCGCCGGGGCCGACCACGUG | 20 | 2500 |
| HSV1-UL19-6940 | - | UUCGCCGGGGCCGACCACGUG | 21 | 11539 |
| HSV1-UL19-6941 | - | GUUCGCCGGGGCCGACCACGUG | 22 | 11540 |
| HSV1-UL19-6942 | - | UGUUCGCCGGGGCCGACCACGUG | 23 | 11541 |
| HSV1-UL19-6943 | - | CUGUUCGCCGGGGCCGACCACGUG | 24 | 11542 |
| HSV1-UL19-6944 | - | UGCCCCAGCGACCCCGUG | 18 | 11543 |
| HSV1-UL19-6945 | - | GUGCCCCAGCGACCCCGUG | 19 | 11544 |
| HSV1-UL19-2212 | - | AGUGCCCCAGCGACCCCGUG | 20 | 2490 |
| HSV1-UL19-6946 | - | GAGUGCCCCAGCGACCCCGUG | 21 | 11545 |
| HSV1-UL19-6947 | - | GGAGUGCCCCAGCGACCCCGUG | 22 | 11546 |
| HSV1-UL19-6948 | - | AGGAGUGCCCCAGCGACCCCGUG | 23 | 11547 |
| HSV1-UL19-6949 | - | GAGGAGUGCCCCAGCGACCCCGUG | 24 | 11548 |
| HSV1-UL19-6950 | - | CAGGCCACCUGGCGCGUG | 18 | 11549 |
| HSV1-UL19-6951 | - | CCAGGCCACCUGGCGCGUG | 19 | 11550 |
| HSV1-UL19-2147 | - | UCCAGGCCACCUGGCGCGUG | 20 | 2452 |
| HSV1-UL19-6952 | - | AUCCAGGCCACCUGGCGCGUG | 21 | 11551 |
| HSV1-UL19-6953 | - | GAUCCAGGCCACCUGGCGCGUG | 22 | 11552 |
| HSV1-UL19-6954 | - | AGAUCCAGGCCACCUGGCGCGUG | 23 | 11553 |
| HSV1-UL19-6955 | - | GAGAUCCAGGCCACCUGGCGCGUG | 24 | 11554 |
| HSV1-UL19-6956 | - | GGGUGCCGCGAACUCGUG | 18 | 11555 |
| HSV1-UL19-6957 | - | GGGGUGCCGCGAACUCGUG | 19 | 11556 |
| HSV1-UL19-2313 | - | CGGGGUGCCGCGAACUCGUG | 20 | 2551 |
| HSV1-UL19-6958 | - | CCGGGGUGCCGCGAACUCGUG | 21 | 11557 |
| HSV1-UL19-6959 | - | CCCGGGGUGCCGCGAACUCGUG | 22 | 11558 |
| HSV1-UL19-6960 | - | CCCCGGGGUGCCGCGAACUCGUG | 23 | 11559 |
| HSV1-UL19-6961 | - | CCCCCGGGGUGCCGCGAACUCGUG | 24 | 11560 |
| HSV1-UL19-6962 | - | CUGCUGCCACCCCUCGUG | 18 | 11561 |
| HSV1-UL19-6963 | - | GCUGCUGCCACCCCUCGUG | 19 | 11562 |
| HSV1-UL19-379 | - | CGCUGCUGCCACCCCUCGUG | 20 | 765 |
| HSV1-UL19-6964 | - | GCGCUGCUGCCACCCCUCGUG | 21 | 11563 |
| HSV1-UL19-6965 | - | GGCGCUGCUGCCACCCCUCGUG | 22 | 11564 |
| HSV1-UL19-6966 | - | CGGCGCUGCUGCCACCCCUCGUG | 23 | 11565 |
| HSV1-UL19-6967 | - | CCGGCGCUGCUGCCACCCCUCGUG | 24 | 11566 |
| HSV1-UL19-6968 | - | CUGGUCACGGCGCUCGUG | 18 | 11567 |
| HSV1-UL19-6969 | - | CCUGGUCACGGCGCUCGUG | 19 | 11568 |
| HSV1-UL19-2069 | - | ACCUGGUCACGGCGCUCGUG | 20 | 2408 |
| HSV1-UL19-6970 | - | AACCUGGUCACGGCGCUCGUG | 21 | 11569 |
| HSV1-UL19-6971 | - | CAACCUGGUCACGGCGCUCGUG | 22 | 11570 |
| HSV1-UL19-6972 | - | CCAACCUGGUCACGGCGCUCGUG | 23 | 11571 |
| HSV1-UL19-6973 | - | GCCAACCUGGUCACGGCGCUCGUG | 24 | 11572 |
| HSV1-UL19-6974 | - | CUGCGGAACGCGGUCGUG | 18 | 11573 |
| HSV1-UL19-6975 | - | CCUGCGGAACGCGGUCGUG | 19 | 11574 |
| HSV1-UL19-2266 | - | ACCUGCGGAACGCGGUCGUG | 20 | 2527 |
| HSV1-UL19-6976 | - | UACCUGCGGAACGCGGUCGUG | 21 | 11575 |
| HSV1-UL19-6977 | - | GUACCUGCGGAACGCGGUCGUG | 22 | 11576 |
| HSV1-UL19-6978 | - | UGUACCUGCGGAACGCGGUCGUG | 23 | 11577 |
| HSV1-UL19-6979 | - | GUGUACCUGCGGAACGCGGUCGUG | 24 | 11578 |
| HSV1-UL19-6980 | - | CCGCGUUCGACUUCUUUG | 18 | 11579 |
| HSV1-UL19-6981 | - | CCCGCGUUCGACUUCUUUG | 19 | 11580 |
| HSV1-UL19-305 | - | CCCCGCGUUCGACUUCUUUG | 20 | 691 |
| HSV1-UL19-6982 | - | ACCCCGCGUUCGACUUCUUUG | 21 | 11581 |
| HSV1-UL19-6983 | - | CACCCCGCGUUCGACUUCUUUG | 22 | 11582 |
| HSV1-UL19-6984 | - | GCACCCCGCGUUCGACUUCUUUG | 23 | 11583 |
| HSV1-UL19-6985 | - | UGCACCCCGCGUUCGACUUCUUUG | 24 | 11584 |
| HSV1-UL19-6986 | - | CCGCCAGCUGUUUUUUUG | 18 | 11585 |
| HSV1-UL19-6987 | - | CCCGCCAGCUGUUUUUUUG | 19 | 11586 |
| HSV1-UL19-270 | - | CCCCGCCAGCUGUUUUUUUG | 20 | 656 |
| HSV1-UL19-6988 | - | GCCCCGCCAGCUGUUUUUUUG | 21 | 11587 |
| HSV1-UL19-6989 | - | CGCCCCGCCAGCUGUUUUUUUG | 22 | 11588 |
| HSV1-UL19-6990 | - | CCGCCCCGCCAGCUGUUUUUUUG | 23 | 11589 |
| HSV1-UL19-6991 | - | CCCGCCCCGCCAGCUGUUUUUUUG | 24 | 11590 |
| HSV1-UL19-6992 | - | CGAGCUGAAUCACCUAAU | 18 | 11591 |
| HSV1-UL19-6993 | - | CCGAGCUGAAUCACCUAAU | 19 | 11592 |
| HSV1-UL19-2182 | - | GCCGAGCUGAAUCACCUAAU | 20 | 2472 |
| HSV1-UL19-6994 | - | AGCCGAGCUGAAUCACCUAAU | 21 | 11593 |
| HSV1-UL19-6995 | - | AAGCCGAGCUGAAUCACCUAAU | 22 | 11594 |
| HSV1-UL19-6996 | - | CAAGCCGAGCUGAAUCACCUAAU | 23 | 11595 |
| HSV1-UL19-6997 | - | GCAAGCCGAGCUGAAUCACCUAAU | 24 | 11596 |
| HSV1-UL19-6998 | - | CUGAUGGCCCCCCAGCAU | 18 | 11597 |
| HSV1-UL19-6999 | - | GCUGAUGGCCCCCCAGCAU | 19 | 11598 |
| HSV1-UL19-2222 | - | UGCUGAUGGCCCCCCAGCAU | 20 | 2498 |
| HSV1-UL19-7000 | - | CUGCUGAUGGCCCCCCAGCAU | 21 | 11599 |
| HSV1-UL19-7001 | - | CCUGCUGAUGGCCCCCCAGCAU | 22 | 11600 |
| HSV1-UL19-7002 | - | UCCUGCUGAUGGCCCCCCAGCAU | 23 | 11601 |
| HSV1-UL19-7003 | - | AUCCUGCUGAUGGCCCCCCAGCAU | 24 | 11602 |
| HSV1-UL19-7004 | - | GUCUCUGGAACACGCGAU | 18 | 11603 |
| HSV1-UL19-7005 | - | UGUCUCUGGAACACGCGAU | 19 | 11604 |
| HSV1-UL19-275 | - | CUGUCUCUGGAACACGCGAU | 20 | 661 |
| HSV1-UL19-7006 | - | CCUGUCUCUGGAACACGCGAU | 21 | 11605 |
| HSV1-UL19-7007 | - | GCCUGUCUCUGGAACACGCGAU | 22 | 11606 |
| HSV1-UL19-7008 | - | CGCCUGUCUCUGGAACACGCGAU | 23 | 11607 |
| HSV1-UL19-7009 | - | GCGCCUGUCUCUGGAACACGCGAU | 24 | 11608 |
| HSV1-UL19-7010 | - | CAGGACCGCUUUGUGACU | 18 | 11609 |
| HSV1-UL19-7011 | - | ACAGGACCGCUUUGUGACU | 19 | 11610 |
| HSV1-UL19-2243 | - | GACAGGACCGCUUUGUGACU | 20 | 2512 |
| HSV1-UL19-7012 | - | CGACAGGACCGCUUUGUGACU | 21 | 11611 |
| HSV1-UL19-7013 | - | CCGACAGGACCGCUUUGUGACU | 22 | 11612 |
| HSV1-UL19-7014 | - | UCCGACAGGACCGCUUUGUGACU | 23 | 11613 |
| HSV1-UL19-7015 | - | GUCCGACAGGACCGCUUUGUGACU | 24 | 11614 |
| HSV1-UL19-7016 | - | GUACGUCGUGACCUACCU | 18 | 11615 |
| HSV1-UL19-7017 | - | CGUACGUCGUGACCUACCU | 19 | 11616 |
| HSV1-UL19-358 | - | UCGUACGUCGUGACCUACCU | 20 | 744 |
| HSV1-UL19-7018 | - | CUCGUACGUCGUGACCUACCU | 21 | 11617 |
| HSV1-UL19-7019 | - | UCUCGUACGUCGUGACCUACCU | 22 | 11618 |
| HSV1-UL19-7020 | - | GUCUCGUACGUCGUGACCUACCU | 23 | 11619 |
| HSV1-UL19-7021 | - | GGUCUCGUACGUCGUGACCUACCU | 24 | 11620 |
| HSV1-UL19-7022 | - | CGCGGCAGCCGUGUACCU | 18 | 11621 |
| HSV1-UL19-7023 | - | ACGCGGCAGCCGUGUACCU | 19 | 11622 |
| HSV1-UL19-2264 | - | AACGCGGCAGCCGUGUACCU | 20 | 2526 |
| HSV1-UL19-7024 | - | CAACGCGGCAGCCGUGUACCU | 21 | 11623 |
| HSV1-UL19-7025 | - | ACAACGCGGCAGCCGUGUACCU | 22 | 11624 |
| HSV1-UL19-7026 | - | GACAACGCGGCAGCCGUGUACCU | 23 | 11625 |
| HSV1-UL19-7027 | - | GGACAACGCGGCAGCCGUGUACCU | 24 | 11626 |
| HSV1-UL19-7028 | - | AGCUGUUUUUGAACGCCU | 18 | 11627 |
| HSV1-UL19-7029 | - | CAGCUGUUUUUGAACGCCU | 19 | 11628 |
| HSV1-UL19-291 | - | GCAGCUGUUUUUGAACGCCU | 20 | 677 |
| HSV1-UL19-7030 | - | AGCAGCUGUUUUUGAACGCCU | 21 | 11629 |
| HSV1-UL19-7031 | - | CAGCAGCUGUUUUUGAACGCCU | 22 | 11630 |
| HSV1-UL19-7032 | - | GCAGCAGCUGUUUUUGAACGCCU | 23 | 11631 |
| HSV1-UL19-7033 | - | UGCAGCAGCUGUUUUUGAACGCCU | 24 | 11632 |
| HSV1-UL19-3667 | - | CAACGUCCAGGCCGUCCU | 18 | 8266 |
| HSV1-UL19-3668 | - | GCAACGUCCAGGCCGUCCU | 19 | 8267 |
| HSV1-UL19-189 | - | CGCAACGUCCAGGCCGUCCU | 20 | 575 |
| HSV1-UL19-3669 | - | UCGCAACGUCCAGGCCGUCCU | 21 | 8268 |
| HSV1-UL19-3670 | - | CUCGCAACGUCCAGGCCGUCCU | 22 | 8269 |
| HSV1-UL19-3671 | - | GCUCGCAACGUCCAGGCCGUCCU | 23 | 8270 |
| HSV1-UL19-3672 | - | CGCUCGCAACGUCCAGGCCGUCCU | 24 | 8271 |
| HSV1-UL19-7034 | - | CCCCUGGUCCCCCCGGCU | 18 | 11633 |
| HSV1-UL19-7035 | - | CCCCCUGGUCCCCCCGGCU | 19 | 11634 |
| HSV1-UL19-2226 | - | UCCCCCUGGUCCCCCCGGCU | 20 | 2501 |
| HSV1-UL19-7036 | - | GUCCCCCUGGUCCCCCCGGCU | 21 | 11635 |
| HSV1-UL19-7037 | - | GGUCCCCCUGGUCCCCCCGGCU | 22 | 11636 |
| HSV1-UL19-7038 | - | AGGUCCCCCUGGUCCCCCCGGCU | 23 | 11637 |
| HSV1-UL19-7039 | - | CAGGUCCCCCUGGUCCCCCCGGCU | 24 | 11638 |
| HSV1-UL19-7040 | - | CCUGGUCCCCCCGGCUCU | 18 | 11639 |
| HSV1-UL19-7041 | - | CCCUGGUCCCCCCGGCUCU | 19 | 11640 |
| HSV1-UL19-456 | - | CCCCUGGUCCCCCCGGCUCU | 20 | 842 |
| HSV1-UL19-7042 | - | CCCCCUGGUCCCCCCGGCUCU | 21 | 11641 |
| HSV1-UL19-7043 | - | UCCCCCUGGUCCCCCCGGCUCU | 22 | 11642 |
| HSV1-UL19-7044 | - | GUCCCCCUGGUCCCCCCGGCUCU | 23 | 11643 |
| HSV1-UL19-7045 | - | GGUCCCCCUGGUCCCCCCGGCUCU | 24 | 11644 |
| HSV1-UL19-7046 | - | CAGGUGCUGCGCCUGUCU | 18 | 11645 |
| HSV1-UL19-7047 | - | CCAGGUGCUGCGCCUGUCU | 19 | 11646 |
| HSV1-UL19-2107 | - | GCCAGGUGCUGCGCCUGUCU | 20 | 2434 |
| HSV1-UL19-7048 | - | CGCCAGGUGCUGCGCCUGUCU | 21 | 11647 |
| HSV1-UL19-7049 | - | CCGCCAGGUGCUGCGCCUGUCU | 22 | 11648 |
| HSV1-UL19-7050 | - | ACCGCCAGGUGCUGCGCCUGUCU | 23 | 11649 |
| HSV1-UL19-7051 | - | GACCGCCAGGUGCUGCGCCUGUCU | 24 | 11650 |
| HSV1-UL19-7052 | - | AGCUAAAGCGAAGCUUCU | 18 | 11651 |
| HSV1-UL19-7053 | - | GAGCUAAAGCGAAGCUUCU | 19 | 11652 |
| HSV1-UL19-2048 | - | CGAGCUAAAGCGAAGCUUCU | 20 | 2392 |
| HSV1-UL19-7054 | - | CCGAGCUAAAGCGAAGCUUCU | 21 | 11653 |
| HSV1-UL19-7055 | - | GCCGAGCUAAAGCGAAGCUUCU | 22 | 11654 |
| HSV1-UL19-7056 | - | CGCCGAGCUAAAGCGAAGCUUCU | 23 | 11655 |
| HSV1-UL19-7057 | - | UCGCCGAGCUAAAGCGAAGCUUCU | 24 | 11656 |
| HSV1-UL19-7058 | - | CUGAGAACGUGCUGUUCU | 18 | 11657 |
| HSV1-UL19-7059 | - | ACUGAGAACGUGCUGUUCU | 19 | 11658 |
| HSV1-UL19-475 | - | GACUGAGAACGUGCUGUUCU | 20 | 861 |
| HSV1-UL19-7060 | - | UGACUGAGAACGUGCUGUUCU | 21 | 11659 |
| HSV1-UL19-7061 | - | GUGACUGAGAACGUGCUGUUCU | 22 | 11660 |
| HSV1-UL19-7062 | - | UGUGACUGAGAACGUGCUGUUCU | 23 | 11661 |
| HSV1-UL19-7063 | - | UUGUGACUGAGAACGUGCUGUUCU | 24 | 11662 |
| HSV1-UL19-7064 | - | GGCAAGGCCGUGCGAAGU | 18 | 11663 |
| HSV1-UL19-7065 | - | GGGCAAGGCCGUGCGAAGU | 19 | 11664 |
| HSV1-UL19-2071 | - | UGGGCAAGGCCGUGCGAAGU | 20 | 2410 |
| HSV1-UL19-7066 | - | AUGGGCAAGGCCGUGCGAAGU | 21 | 11665 |
| HSV1-UL19-7067 | - | GAUGGGCAAGGCCGUGCGAAGU | 22 | 11666 |
| HSV1-UL19-7068 | - | UGAUGGGCAAGGCCGUGCGAAGU | 23 | 11667 |
| HSV1-UL19-7069 | - | GUGAUGGGCAAGGCCGUGCGAAGU | 24 | 11668 |
| HSV1-UL19-7070 | - | CGUGAUGGGCAAGGCCGU | 18 | 11669 |
| HSV1-UL19-7071 | - | UCGUGAUGGGCAAGGCCGU | 19 | 11670 |
| HSV1-UL19-2070 | - | CUCGUGAUGGGCAAGGCCGU | 20 | 2409 |
| HSV1-UL19-7072 | - | GCUCGUGAUGGGCAAGGCCGU | 21 | 11671 |
| HSV1-UL19-7073 | - | CGCUCGUGAUGGGCAAGGCCGU | 22 | 11672 |
| HSV1-UL19-7074 | - | GCGCUCGUGAUGGGCAAGGCCGU | 23 | 11673 |
| HSV1-UL19-7075 | - | GGCGCUCGUGAUGGGCAAGGCCGU | 24 | 11674 |
| HSV1-UL19-7076 | - | UCGAAGCCGCCAAUCCGU | 18 | 11675 |
| HSV1-UL19-7077 | - | GUCGAAGCCGCCAAUCCGU | 19 | 11676 |
| HSVI-UL19-2116 | - | CGUCGAAGCCGCCAAUCCGU | 20 | 2439 |
| HSV1-UL19-7078 | - | CCGUCGAAGCCGCCAAUCCGU | 21 | 11677 |
| HSV1-UL19-7079 | - | CCCGUCGAAGCCGCCAAUCCGU | 22 | 11678 |
| HSV1-UL19-7080 | - | CCCCGUCGAAGCCGCCAAUCCGU | 23 | 11679 |
| HSV1-UL19-7081 | - | ACCCCGUCGAAGCCGCCAAUCCGU | 24 | 11680 |
| HSV1-UL19-7082 | - | UGUUCUCGGAGCGCGCGU | 18 | 11681 |
| HSV1-UL19-7083 | - | CUGUUCUCGGAGCGCGCGU | 19 | 11682 |
| HSV1-UL19-476 | - | GCUGUUCUCGGAGCGCGCGU | 20 | 862 |
| HSV1-UL19-7084 | - | UGCUGUUCUCGGAGCGCGCGU | 21 | 11683 |
| HSV1-UL19-7085 | - | GUGCUGUUCUCGGAGCGCGCGU | 22 | 11684 |
| HSV1-UL19-7086 | - | CGUGCUGUUCUCGGAGCGCGCGU | 23 | 11685 |
| HSV1-UL19-7087 | - | ACGUGCUGUUCUCGGAGCGCGCGU | 24 | 11686 |
| HSV1-UL19-7088 | - | AGGCCGUCCUCGGGGCGU | 18 | 11687 |
| HSV1-UL19-7089 | - | CAGGCCGUCCUCGGGGCGU | 19 | 11688 |
| HSV1-UL19-2040 | - | CCAGGCCGUCCUCGGGGCGU | 20 | 2385 |
| HSV1-UL19-7090 | - | UCCAGGCCGUCCUCGGGGCGU | 21 | 11689 |
| HSV1-UL19-7091 | - | GUCCAGGCCGUCCUCGGGGCGU | 22 | 11690 |
| HSV1-UL19-7092 | - | CGUCCAGGCCGUCCUCGGGGCGU | 23 | 11691 |
| HSV1-UL19-7093 | - | ACGUCCAGGCCGUCCUCGGGGCGU | 24 | 11692 |
| HSV1-UL19-7094 | - | GCUGCUGCCACCCCUCGU | 18 | 11693 |
| HSV1-UL19-7095 | - | CGCUGCUGCCACCCCUCGU | 19 | 11694 |
| HSV1-UL19-2183 | - | GCGCUGCUGCCACCCCUCGU | 20 | 2473 |
| HSV1-UL19-7096 | - | GGCGCUGCUGCCACCCCUCGU | 21 | 11695 |
| HSV1-UL19-7097 | - | CGGCGCUGCUGCCACCCCUCGU | 22 | 11696 |
| HSV1-UL19-7098 | - | CCGGCGCUGCUGCCACCCCUCGU | 23 | 11697 |
| HSV1-UL19-7099 | - | CCCGGCGCUGCUGCCACCCCUCGU | 24 | 11698 |
| HSV1-UL19-7100 | - | CGUCGCAGCGAUUUUCGU | 18 | 11699 |
| HSV1-UL19-7101 | - | GCGUCGCAGCGAUUUUCGU | 19 | 11700 |
| HSV1-UL19-2296 | - | GGCGUCGCAGCGAUUUUCGU | 20 | 2542 |
| HSV1-UL19-7102 | - | GGGCGUCGCAGCGAUUUUCGU | 21 | 11701 |
| HSV1-UL19-7103 | - | UGGGCGUCGCAGCGAUUUUCGU | 22 | 11702 |
| HSV1-UL19-7104 | - | GUGGGCGUCGCAGCGAUUUUCGU | 23 | 11703 |
| HSV1-UL19-7105 | - | CGUGGGCGUCGCAGCGAUUUUCGU | 24 | 11704 |
| HSV1-UL19-7106 | - | GAACGUUGACGCGGCGGU | 18 | 11705 |
| HSV1-UL19-7107 | - | UGAACGUUGACGCGGCGGU | 19 | 11706 |
| HSV1-UL19-279 | - | AUGAACGUUGACGCGGCGGU | 20 | 665 |
| HSV1-UL19-7108 | - | GAUGAACGUUGACGCGGCGGU | 21 | 11707 |
| HSV1-UL19-7109 | - | UGAUGAACGUUGACGCGGCGGU | 22 | 11708 |
| HSV1-UL19-7110 | - | CUGAUGAACGUUGACGCGGCGGU | 23 | 11709 |
| HSV1-UL19-7111 | - | GCUGAUGAACGUUGACGCGGCGGU | 24 | 11710 |
| HSV1-UL19-7112 | - | GCUAUGUCCGGCGGCGUU | 18 | 11711 |
| HSV1-UL19-7113 | - | CGCUAUGUCCGGCGGCGUU | 19 | 11712 |
| HSV1-UL19-2148 | - | GCGCUAUGUCCGGCGGCGUU | 20 | 2453 |
| HSV1-UL19-7114 | - | GGCGCUAUGUCCGGCGGCGUU | 21 | 11713 |
| HSV1-UL19-7115 | - | UGGCGCUAUGUCCGGCGGCGUU | 22 | 11714 |
| HSV1-UL19-7116 | - | CUGGCGCUAUGUCCGGCGGCGUU | 23 | 11715 |
| HSV1-UL19-7117 | - | CCUGGCGCUAUGUCCGGCGGCGUU | 24 | 11716 |
| HSV1-UL19-7118 | - | GGCGAGAAGCUGGUCUUU | 18 | 11717 |
| HSV1-UL19-7119 | - | CGGCGAGAAGCUGGUCUUU | 19 | 11718 |
| HSV1-UL19-2087 | - | UCGGCGAGAAGCUGGUCUUU | 20 | 2423 |
| HSV1-UL19-7120 | - | AUCGGCGAGAAGCUGGUCUUU | 21 | 11719 |
| HSV1-UL19-7121 | - | CAUCGGCGAGAAGCUGGUCUUU | 22 | 11720 |
| HSV1-UL19-7122 | - | CCAUCGGCGAGAAGCUGGUCUUU | 23 | 11721 |
| HSV1-UL19-7123 | - | UCCAUCGGCGAGAAGCUGGUCUUU | 24 | 11722 |
| HSV1-UL19-7124 | - | CCCGCGUUCGACUUCUUU | 18 | 11723 |
| HSV1-UL19-7125 | - | CCCCGCGUUCGACUUCUUU | 19 | 11724 |
| HSV1-UL19-2130 | - | ACCCCGCGUUCGACUUCUUU | 20 | 2448 |
| HSV1-UL19-7126 | - | CACCCCGCGUUCGACUUCUUU | 21 | 11725 |
| HSV1-UL19-7127 | - | GCACCCCGCGUUCGACUUCUUU | 22 | 11726 |
| HSV1-UL19-7128 | - | UGCACCCCGCGUUCGACUUCUUU | 23 | 11727 |
| HSV1-UL19-7129 | - | CUGCACCCCGCGUUCGACUUCUUU | 24 | 11728 |
| HSV1-UL19-7130 | - | GACCCGUGUGGCCUGUUU | 18 | 11729 |
| HSV-UL19-7131 | - | GGACCCGUGUGGCCUGUUU | 19 | 11730 |
| HSV1-UL19-2314 | - | AGGACCCGUGUGGCCUGUUU | 20 | 2552 |
| HSV1-UL19-7132 | - | GAGGACCCGUGUGGCCUGUUU | 21 | 11731 |
| HSV1-UL19-7133 | - | GGAGGACCCGUGUGGCCUGUUU | 22 | 11732 |
| HSV1-UL19-7134 | - | UGGAGGACCCGUGUGGCCUGUUU | 23 | 11733 |
| HSV1-UL19-7135 | - | GUGGAGGACCCGUGUGGCCUGUUU | 24 | 11734 |
| HSV1-UL19-7136 | - | GACAUGCAGCAGCUGUUU | 18 | 11735 |
| HSV1-UL19-7137 | - | AGACAUGCAGCAGCUGUUU | 19 | 11736 |
| HSV1-UL19-2118 | - | CAGACAUGCAGCAGCUGUUU | 20 | 2440 |
| HSV1-UL19-7138 | - | GCAGACAUGCAGCAGCUGUUU | 21 | 11737 |
| HSV1-UL19-7139 | - | CGCAGACAUGCAGCAGCUGUUU | 22 | 11738 |
| HSV1-UL19-7140 | - | CCGCAGACAUGCAGCAGCUGUUU | 23 | 11739 |
| HSV1-UL19-7141 | - | GCCGCAGACAUGCAGCAGCUGUUU | 24 | 11740 |
| HSV1-UL19-7142 | - | GCCCCGCCAGCUGUUUUU | 18 | 11741 |
| HSV1-UL19-7143 | - | CGCCCCGCCAGCUGUUUUU | 19 | 11742 |
| HSV1-UL19-2101 | - | CCGCCCCGCCAGCUGUUUUU | 20 | 2432 |
| HSV1-UL19-7144 | - | CCCGCCCCGCCAGCUGUUUUU | 21 | 11743 |
| HSV1-UL19-7145 | - | UCCCGCCCCGCCAGCUGUUUUU | 22 | 11744 |
| HSV1-UL19-7146 | - | UUCCCGCCCCGCCAGCUGUUUUU | 23 | 11745 |
| HSV1-UL19-7147 | - | CUUCCCGCCCCGCCAGCUGUUUUU | 24 | 11746 |
| HSV1-UL19-7148 | - | CCCCGCCAGCUGUUUUUU | 18 | 11747 |
| HSV1-UL19-7149 | - | GCCCCGCCAGCUGUUUUUU | 19 | 11748 |
| HSV1-UL19-268 | - | CGCCCCGCCAGCUGUUUUUU | 20 | 654 |
| HSV1-UL19-7150 | - | CCGCCCCGCCAGCUGUUUUUU | 21 | 11749 |
| HSV1-UL19-7151 | - | CCCGCCCCGCCAGCUGUUUUUU | 22 | 11750 |
| HSV1-UL19-7152 | - | UCCCGCCCCGCCAGCUGUUUUUU | 23 | 11751 |
| HSV1-UL19-7153 | - | UUCCCGCCCCGCCAGCUGUUUUUU | 24 | 11752 |
| HSV1-UL19-7154 | - | CCCGCCAGCUGUUUUUUU | 18 | 11753 |
| HSV1-UL19-7155 | - | CCCCGCCAGCUGUUUUUUU | 19 | 11754 |
| HSV1-UL19-269 | - | GCCCCGCCAGCUGUUUUUUU | 20 | 655 |
| HSV1-UL19-7156 | - | CGCCCCGCCAGCUGUUUUUUU | 21 | 11755 |
| HSV1-UL19-7157 | - | CCGCCCCGCCAGCUGUUUUUUU | 22 | 11756 |
| HSV1-UL19-7158 | - | CCCGCCCCGCCAGCUGUUUUUUU | 23 | 11757 |
| HSV1-UL19-7159 | - | UCCCGCCCCGCCAGCUGUUUUUUU | 24 | 11758 |

**Table** 7A provides exemplary targeting domains for knocking out the *UL19* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 7A**

| **1st Tier** | | | | |
|---|---|---|---|---|
| **gRNA Name** | **DNA Strand** | **Targeting Domain** | **Target Site Length** | **Seq ID NO:** |
| HSV1-UL19-3193 | - | GUUCGAGGUCCACCAGC | 17 | 3109 |
| HSV1-UL19-7160 | - | GCAACACCCUGUCGCUC | 17 | 11759 |
| HSV1-UL19-3192 | - | GCGUCGCAUCGACGCCU | 17 | 3108 |

**Table 7B** provides exemplary targeting domains for knocking out the *UL19* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 7B**

| **2nd Tier** | | | | |
|---|---|---|---|---|
| **gRNA Name** | **DNA Strand** | **Targeting Domain** | **Target Site Length** | **Seq ID NO:** |
| HSV1-UL19-3191 | - | ACCCACACACAAUUACAUGA | 20 | 3107 |
| HSV1-UL19-3190 | - | ACAGUUCGAGGUCCACCAGC | 20 | 3106 |
| HSV1-UL19-7161 | - | AUUGCAACACCCUGUCGCUC | 20 | 11760 |

**Table** 7C provides exemplary targeting domains for knocking out the *UL19* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 7C**

| **4th Tier** | | | | |
|---|---|---|---|---|
| **gRNA Name** | **DNA Strand** | **Targeting Domain** | **Target Site Length** | **Seq ID NO:** |
| HSV1-UL19-3194 | - | CACACACAAUUACAUGA | 17 | 3110 |

**Table** 7D provides exemplary targeting domains for knocking out the *UL19* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 7D**

| 5th Tier | | | | |
|---|---|---|---|---|
| gRNA Name | DNA Strand | Targeting Domain | Target Site Length | Seq ID NO: |
| HSV1-UL19-3208 | + | AUGCUGGGGGGCCAUCA | 17 | 3122 |
| HSV1-UL19-7162 | + | CCGUACGUCACCGGCAC | 17 | 11761 |
| HSV1-UL19-3198 | - | GGACUGGACCGUGCACC | 17 | 3114 |
| HSV1-UL19-7163 | - | CUGGAAAAGGCGCCGCC | 17 | 11762 |
| HSV1-UL19-3207 | + | CGUUGACCGUGUUGGCC | 17 | 3121 |
| HSV1-UL19-7164 | - | CCUCGCCGGUGCUCAGC | 17 | 11763 |
| HSV1-UL19-3182 | + | AAGUGGGUCUCCCGCGC | 17 | 3100 |
| HSV1-UL19-7165 | + | GAAAAAGCUC GUCUC GC | 17 | 11764 |
| HSV1-UL19-7166 | + | CCAGGGCCUCCAGAAAG | 17 | 11765 |
| HSV1-UL19-7167 | - | UGGUCGCCGAGCUAAAG | 17 | 11766 |
| HSV1-UL19-7168 | + | AGCUCGUCUCGCAGAAG | 17 | 11767 |
| HSV1-UL19-7169 | + | CGCAUUAGGUGAUUCAG | 17 | 11768 |
| HSV1-UL19-2335 | - | AGCGAAGCUUCUGCGAG | 17 | 2564 |
| HSV1-UL19-7170 | + | CGUUGGUGGCGGCGUAG | 17 | 11769 |
| HSV1-UL19-3205 | + | CCGCCACGUACGCCCCG | 17 | 3120 |
| HSV1-UL19-3197 | - | CCAACCCGUGGGCGUCG | 17 | 3113 |
| HSV1-UL19-1879 | + | GUUCGCGGCACCCCGGG | 17 | 2265 |
| HSV1-UL19-7171 | - | GCUGCUGCACCGCGGGG | 17 | 11770 |
| HSV1-UL19-3204 | + | CGGCAAACCGUUCCAUG | 17 | 3119 |
| HSV1-UL19-1648 | + | GCGCCGGGUCUCGCAUU | 17 | 2034 |
| HSV1-UL19-7172 | + | UACGCCCCGUACGGAUU | 17 | 11771 |
| HSV1-UL19-3203 | + | CAGAUGCUGGGGGGCCAUCA | 20 | 3118 |
| HSV1-UL19-7173 | + | UCGCCGUACGUCACCGGCAC | 20 | 11772 |
| HSV1-UL19-3196 | - | GGCGGACUGGACCGUGCACC | 20 | 3112 |
| HSV1-UL19-7174 | - | CUGCUGGAAAAGGCGCCGCC | 20 | 11773 |
| HSV1-UL19-3202 | + | UGGCGUUGACCGUGUUGGCC | 20 | 3117 |
| HSV1-UL19-7175 | - | GGGCCUCGCCGGUGCUCAGC | 20 | 11774 |
| HSV1-UL19-2893 | + | GCGAAGUGGGUCUCCCGCGC | 20 | 2911 |
| HSV1-UL19-7176 | + | CAGGAAAAAGCUC GUCUC GC | 20 | 11775 |
| HSV1-UL19-7177 | + | UCUCCAGGGCCUCCAGAAAG | 20 | 11776 |
| HSV1-UL19-7178 | - | CCCUGGUCGCCGAGCUAAAG | 20 | 11777 |
| HSV1-UL19-7179 | + | AAAAGCUCGUCUCGCAGAAG | 20 | 11778 |
| HSV1-UL19-7180 | - | GGUUCACCGUCGUCCGACAG | 20 | 11779 |
| HSV1-UL19-7181 | + | UCUCGCAUUAGGUGAUUCAG | 20 | 11780 |
| HSV1-UL19-2049 | - | UAAAGC GAAGCUUCUGC GAG | 20 | 2393 |
| HSV1-UL19-7182 | + | GAACGUUGGUGGCGGCGUAG | 20 | 11781 |
| HSV1-UL19-3200 | + | GGGCCGCCACGUACGCCCCG | 20 | 3116 |
| HSV1-UL19-1412 | + | CGAGUUCGCGGCACCCCGGG | 20 | 1798 |
| HSV1-UL19-7183 | - | GCCGCUGCUGCACCGCGGGG | 20 | 11782 |
| HSV1-UL19-3199 | + | GCGCGGCAAACCGUUCCAUG | 20 | 3115 |
| HSV1-UL19-1181 | + | GCAGCGCCGGGUCUCGCAUU | 20 | 1567 |

**Table 8A** provides exemplary targeting domains for knocking out the *UL30* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 8A**

| **1st Tier** | | | | |
|---|---|---|---|---|
| **gRNA Name** | **DNA Strand** | **Targeting Domain** | **Target Site Length** | **Seq ID NO:** |
| HSV1-UL30-175 | + | GUCGUACACGUGAAAGA | 17 | 3203 |
| HSV1-UL30-2843 | - | GUUCCACGCGCGGUUUA | 17 | 11783 |
| HSV1-UL30-228 | + | GGCGGGCGCAAAAAACC | 17 | 3227 |
| HSV1-UL30-193 | + | GUGACCGUCGUGCACCC | 17 | 3212 |
| HSV1-UL30-128 | - | GGGUUUUUUGCGCCCGC | 17 | 3180 |
| HSV1-UL30-168 | - | GUGGACCACGCCCCGGC | 17 | 3198 |
| HSV1-UL30-230 | + | GGCCGCCGACUUUCCUC | 17 | 3228 |
| HSV1-UL30-155 | - | GCGACGUCCUCCGCGUC | 17 | 3191 |
| HSV1-UL30-157 | - | GUCCUCCGCGUCGGGUC | 17 | 3193 |
| HSV1-UL30-182 | + | GGGGUUGAACCCCGCCG | 17 | 3205 |
| HSV1-UL30-212 | + | GCGUCCCGACUGGGGCG | 17 | 3225 |
| HSV1-UL30-2844 | + | GCCACCGGAAAACAUCG | 17 | 11784 |
| HSV1-UL30-2845 | - | GCGAUGUUUUCCGGUGG | 17 | 11785 |
| HSV1-UL30-57 | + | GAUGUCGUACACGUGAAAGA | 20 | 3150 |
| HSV1-UL30-54 | - | GAGAACGUGGAGCACGCGUA | 20 | 3148 |
| HSV1-UL30-91 | + | GGCUUCUGUUGCGUCCCGAC | 20 | 3166 |
| HSV1-UL30-18 | - | GGGACGCAACAGAAGCCGAC | 20 | 3126 |
| HSV1-UL30-110 | + | GCCGGCGGGCGCAAAAAACC | 20 | 3174 |
| HSV1-UL30-19 | - | GGACGCAACAGAAGCCGACC | 20 | 3127 |
| HSV1-UL30-75 | + | GAGGUGACCGUCGUGCACCC | 20 | 3159 |
| HSV1-UL30-48 | - | GGGCGGCGUGGACCACGCCC | 20 | 3258 |
| HSV1-UL30-77 | + | GCACCCCGGCGCGCUUCUCC | 20 | 3271 |
| HSV1-UL30-25 | - | GCCCCCCCGGAGAAGCGCGC | 20 | 3131 |
| HSV1-UL30-50 | - | GGCGUGGACCACGCCCCGGC | 20 | 3144 |
| HSV1-UL30-31 | - | GCGCCCCCAAGGUGUACUGC | 20 | 3136 |
| HSV1-UL30-39 | - | GACGUCCUCCGCGUCGGGUC | 20 | 3142 |
| HSV1-UL30-2846 | - | GGAACAACCGCGAUGUUUUC | 20 | 11786 |
| HSV1-UL30-53 | - | GUACGACAUCCUGGAGAACG | 20 | 3147 |
| HSV1-UL30-82 | + | GCAUCCUCGUCCAGCACCCG | 20 | 3161 |
| HSV1-UL30-64 | + | GGUGGGGUUGAACCCCGCCG | 20 | 3153 |
| HSV1-UL30-2847 | - | GCGCGGCCCAGUUCCACGCG | 20 | 11787 |
| HSV1-UL30-55 | - | GCACGCGUACGGCAUGCGCG | 20 | 3260 |
| HSV1-UL30-101 | + | GUUUUGCCUCAAACAAGGCG | 20 | 3171 |
| HSV1-UL30-51 | - | GCGUGGACCACGCCCCGGCG | 20 | 3145 |
| HSV1-UL30-65 | + | GGUUGAACCCCGCCGGGGCG | 20 | 3154 |
| HSV1-UL30-94 | + | GUUGCGUCCCGACUGGGGCG | 20 | 3169 |
| HSV1-UL30-2848 | + | GCCGCCACCGGAAAACAUCG | 20 | 11788 |
| HSV1-UL30-108 | + | GCUGGCUCCGCGAGGGCCGG | 20 | 3288 |
| HSV1-UL30-115 | + | GGCCGCCGACUUUCCUCCGG | 20 | 3177 |
| HSV1-UL30-2849 | - | GCGAUGUUUUCCGGUGGCGG | 20 | 11789 |
| HSV1-UL30-4 | - | GUCCCCCGGAGGAAAGUCGG | 20 | 3123 |
| HSV1-UL30-40 | - | GUCCUCCGCGUCGGGUCGGG | 20 | 3143 |
| HSV1-UL30-92 | + | GCUUCUGUUGCGUCCCGACU | 20 | 3167 |
| HSV1-UL30-70 | + | GUCCCCCCCGCAGUACACCU | 20 | 3157 |
| HSV1-UL30-87 | + | GCUAUAGUACGUAUGGCGCU | 20 | 3163 |
| HSV1-UL30-3 | - | GCUGUCCCCCGGAGGAAAGU | 20 | 3234 |
| HSV1-UL30-36 | - | GAGCGCGACGUCCUCCGCGU | 20 | 3139 |

**Table 8B** provides exemplary targeting domains for knocking out the *UL30* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 8B**

| **2nd Tier** | | | | |
|---|---|---|---|---|
| **gRNA Name** | **DNA Strand** | **Targeting Domain** | **Target Site Length** | **Seq ID NO:** |
| HSV1-UL30-147 | - | CCUCAAGCGCGCCCCCA | 17 | 3188 |
| HSV1-UL30-203 | + | AUUC GCUAUAGUAC GUA | 17 | 3218 |
| HSV1-UL30-172 | - | AACGUGGAGCACGCGUA | 17 | 3202 |
| HSV1-UL30-2850 | + | UAAACCGCGCGUGGAAC | 17 | 11790 |
| HSV1-UL30-209 | + | UUCUGUUGCGUCCCGAC | 17 | 3222 |
| HSV1-UL30-136 | - | ACGCAACAGAAGCCGAC | 17 | 3181 |
| HSV1-UL30-137 | - | CGCAACAGAAGCCGACC | 17 | 3182 |
| HSV1-UL30-229 | + | AAACCCGGACGCCGCCC | 17 | 3355 |
| HSV1-UL30-170 | - | UCACGUGUACGACAUCC | 17 | 3200 |
| HSV1-UL30-139 | - | UCGAUUCAUCGCCCCGC | 17 | 3184 |
| HSV1-UL30-204 | + | UAUAGUACGUAUGGCGC | 17 | 3219 |
| HSV1-UL30-2851 | - | ACAACCGCGAUGUUUUC | 17 | 11791 |
| HSV1-UL30-215 | + | CGACUGGGGCGAGGUAG | 17 | 3226 |
| HSV1-UL30-186 | + | AGCCGCCCGACCCGACG | 17 | 3207 |
| HSV1-UL30-138 | - | UUCGAUUCAUCGCCCCG | 17 | 3183 |
| HSV1-UL30-185 | + | CACAGGCGCGAGCGCCG | 17 | 3206 |
| HSV1-UL30-232 | + | CCGCCGACUUUCCUCCG | 17 | 3230 |
| HSV1-UL30-196 | + | CCCGGCGCGCUUCUCCG | 17 | 3213 |
| HSV1-UL30-202 | + | CUCGUCCAGCACCCGCG | 17 | 3217 |
| HSV1-UL30-2852 | + | CGUCCAUAAACCGCGCG | 17 | 11792 |
| HSV1-UL30-173 | - | CGCGUACGGCAUGCGCG | 17 | 3324 |
| HSV1-UL30-169 | - | UGGACCACGCCCCGGCG | 17 | 3199 |
| HSV1-UL30-150 | - | CCCCAAGGUGUACUGCG | 17 | 3190 |
| HSV1-UL30-167 | - | CGUGGACCACGCCCCGG | 17 | 3197 |
| HSV1-UL30-233 | + | CGCCGACUUUCCUCCGG | 17 | 3231 |
| HSV1-UL30-2853 | - | ACCGCGAUGUUUUCCGG | 17 | 11793 |
| HSV1-UL30-187 | + | CGCCCGACCCGACGCGG | 17 | 3208 |
| HSV1-UL30-198 | + | CGGCGCGCUUCUCCGGG | 17 | 3215 |
| HSV1-UL30-158 | - | CUCCGCGUCGGGUCGGG | 17 | 3194 |
| HSV1-UL30-164 | - | CGCUCGCGCCUGUGGGG | 17 | 3196 |
| HSV1-UL30-211 | + | CUGUUGCGUCCCGACUG | 17 | 3224 |
| HSV1-UL30-163 | - | CGGCGCUCGCGCCUGUG | 17 | 3195 |
| HSV1-UL30-190 | + | CCCCGCAGUACACCUUG | 17 | 3211 |
| HSV1-UL30-192 | + | CCUUGGGGGCGCGCUUG | 17 | 3334 |
| HSV1-UL30-2854 | + | AAACCGCGCGUGGAACU | 17 | 11794 |
| HSV1-UL30-210 | + | UCUGUUGCGUCCCGACU | 17 | 3223 |
| HSV1-UL30-205 | + | AUAGUACGUAUGGCGCU | 17 | 3220 |
| HSV1-UL30-154 | - | CGCGACGUCCUCCGCGU | 17 | 3317 |
| HSV1-UL30-178 | + | UGAAAGACGGUGACGGU | 17 | 3204 |
| HSV1-UL30-156 | - | CGUCCUCCGCGUCGGGU | 17 | 3192 |
| HSV1-UL30-206 | + | UACGUAUGGCGCUGGGU | 17 | 3221 |
| HSV1-UL30-29 | - | UCACCUCAAGCGCGCCCCCA | 20 | 3134 |
| HSV1-UL30-6 | - | CGGAGGAAAGUCGGCGGCCA | 20 | 3235 |
| HSV1-UL30-85 | + | CGCAUUCGCUAUAGUACGUA | 20 | 3276 |
| HSV1-UL30-2855 | - | CCAGUUCCACGCGCGGUUUA | 20 | 11795 |
| HSV1-UL30-2856 | + | CCAUAAACCGCGCGUGGAAC | 20 | 11796 |
| HSV1-UL30-66 | + | CGUGGUCCACGCCGCCCCAC | 20 | 3265 |
| HSV1-UL30-103 | + | UCAAACAAGGCGGGGGUCCC | 20 | 3173 |
| HSV1-UL30-63 | + | CGGUGGGGUUGAACCCCGCC | 20 | 3264 |
| HSV1-UL30-89 | + | CGUAUGGCGCUGGGUUGGCC | 20 | 3165 |
| HSV1-UL30-52 | - | CUUUCACGUGUACGACAUCC | 20 | 3146 |
| HSV1-UL30-113 | + | CUGGCCGCCGACUUUCCUCC | 20 | 3291 |
| HSV1-UL30-56 | + | ACGCGUGCUCCACGUUCUCC | 20 | 3149 |
| HSV1-UL30-117 | + | ACUUUCCUCCGGGGGACAGC | 20 | 3293 |
| HSV1-UL30-12 | - | CCGGCCCUCGCGGAGCCAGC | 20 | 3240 |
| HSV1-UL30-83 | + | CAUCCUCGUCCAGCACCCGC | 20 | 3275 |
| HSV1-UL30-62 | + | ACGGUGGGGUUGAACCCCGC | 20 | 3152 |
| HSV1-UL30-21 | - | AUUUCGAUUCAUCGCCCCGC | 20 | 3129 |
| HSV1-UL30-10 | - | UCCGGGUUUUUUGCGCCCGC | 20 | 3239 |
| HSV1-UL30-86 | + | CGCUAUAGUACGUAUGGCGC | 20 | 3277 |
| HSV1-UL30-109 | + | CUGGCUCCGCGAGGGCCGGC | 20 | 3289 |
| HSV1-UL30-22 | - | AUUCAUCGCCCCGCGGGUGC | 20 | 3130 |
| HSV1-UL30-112 | + | CCUGGCCGCCGACUUUCCUC | 20 | 3290 |
| HSV1-UL30-76 | + | UGCACCCCGGCGCGCUUCUC | 20 | 3270 |
| HSV1-UL30-37 | - | AGCGCGACGUCCUCCGCGUC | 20 | 3140 |
| HSV1-UL30-68 | + | AGAAGCCGCCCGACCCGACG | 20 | 3155 |
| HSV1-UL30-23 | - | CGCCCCGCGGGUGCUGGACG | 20 | 3246 |
| HSV1-UL30-20 | - | AAUUUCGAUUCAUCGCCCCG | 20 | 3128 |
| HSV1-UL30-67 | + | CCCCACAGGCGCGAGCGCCG | 20 | 3266 |
| HSV1-UL30-27 | - | CCCCCCGGAGAAGCGCGCCG | 20 | 3132 |
| HSV1-UL30-114 | + | UGGCCGCCGACUUUCCUCCG | 20 | 3176 |
| HSV1-UL30-78 | + | CACCCCGGCGCGCUUCUCCG | 20 | 3272 |
| HSV1-UL30-84 | + | AUCCUCGUCCAGCACCCGCG | 20 | 3162 |
| HSV1-UL30-2857 | + | UGGCGUCCAUAAACCGCGCG | 20 | 11797 |
| HSV1-UL30-47 | - | CUCGCGCCUGUGGGGCGGCG | 20 | 3257 |
| HSV1-UL30-32 | - | CGCCCCCAAGGUGUACUGCG | 20 | 3137 |
| HSV1-UL30-11 | - | UUUGCGCCCGCCGGCCCUCG | 20 | 3125 |
| HSV1-UL30-99 | + | AAGUUUUGCCUCAAACAAGG | 20 | 3282 |
| HSV1-UL30-59 | + | CACGUGAAAGACGGUGACGG | 20 | 3262 |
| HSV1-UL30-2858 | - | ACAACCGCGAUGUUUUCCGG | 20 | 11798 |
| HSV1-UL30-69 | + | AGCCGCCCGACCCGACGCGG | 20 | 3156 |
| HSV1-UL30-102 | + | UUUUGCCUCAAACAAGGCGG | 20 | 3172 |
| HSV1-UL30-80 | + | CCCCGGCGCGCUUCUCCGGG | 20 | 3160 |
| HSV1-UL30-46 | - | CGGCGCUCGCGCCUGUGGGG | 20 | 3256 |
| HSV1-UL30-2859 | - | ACCGCGAUGUUUUCCGGUGG | 20 | 11799 |
| HSV1-UL30-73 | + | CCCCCCGCAGUACACCUUGG | 20 | 3269 |
| HSV1-UL30-93 | + | CUUCUGUUGCGUCCCGACUG | 20 | 3168 |
| HSV1-UL30-30 | - | CGCGCCCCCAAGGUGUACUG | 20 | 3135 |
| HSV1-UL30-61 | + | CGUGAAAGACGGUGACGGUG | 20 | 3151 |
| HSV1-UL30-45 | - | CCGCGGCGCUCGCGCCUGUG | 20 | 3255 |
| HSV1-UL30-74 | + | ACACCUUGGGGGCGCGCUUG | 20 | 3158 |
| HSV1-UL30-2860 | + | CAUAAACCGCGCGUGGAACU | 20 | 11800 |
| HSV1-UL30-60 | + | ACGUGAAAGACGGUGACGGU | 20 | 3263 |
| HSV1-UL30-90 | + | UGGCGCUGGGUUGGCCCGGU | 20 | 3278 |
| HSV1-UL30-38 | - | CGACGUCCUCCGCGUCGGGU | 20 | 3141 |
| HSV1-UL30-88 | + | UAGUACGUAUGGCGCUGGGU | 20 | 3164 |

**Table 8C** provides exemplary targeting domains for knocking out the *UL30* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 8C**

| **3rd Tier** | | | | |
|---|---|---|---|---|
| **gRNA Name** | **DNA Strand** | **Targeting Domain** | **Target Site Length** | **Seq ID NO:** |
| HSV1-UL30-184 | + | GGUCCACGCCGCCCCAC | 17 | 3332 |
| HSV1-UL30-142 | - | GGACGAGGAUGCCCCCC | 17 | 3310 |
| HSV1-UL30-119 | - | GGCGGCCCGCUGUCCCC | 17 | 3295 |
| HSV1-UL30-123 | - | GAGGAAAGUCGGCGGCC | 17 | 3298 |
| HSV1-UL30-231 | + | GCCGCCGACUUUCCUCC | 17 | 3229 |
| HSV1-UL30-130 | - | GCCCUCGCGGAGCCAGC | 17 | 3303 |
| HSV1-UL30-180 | + | GUGGGGUUGAACCCCGC | 17 | 3329 |
| HSV1-UL30-227 | + | GCUCCGCGAGGGCCGGC | 17 | 3354 |
| HSV1-UL30-225 | + | GCUGGCUCCGCGAGGGC | 17 | 3352 |
| HSV1-UL30-126 | - | GCGGCCAGGGCGGCGUC | 17 | 3300 |
| HSV1-UL30-165 | - | GCGCCUGUGGGGCGGCG | 17 | 3322 |
| HSV1-UL30-129 | - | GCGCCCGCCGGCCCUCG | 17 | 3302 |
| HSV1-UL30-177 | + | GUGAAAGACGGUGACGG | 17 | 3327 |
| HSV1-UL30-120 | - | GGCCCGCUGUCCCCCGG | 17 | 3296 |
| HSV1-UL30-226 | + | GGCUCCGCGAGGGCCGG | 17 | 3353 |
| HSV1-UL30-199 | + | GGCGCGCUUCUCCGGGG | 17 | 3216 |
| HSV1-UL30-148 | - | GCCCCCAAGGUGUACUG | 17 | 3189 |
| HSV1-UL30-179 | + | GAAAGACGGUGACGGUG | 17 | 3328 |
| HSV1-UL30-133 | - | GACCCCCGCCUUGUUUG | 17 | 3306 |
| HSV1-UL30-121 | - | GUCCCCCGGAGGAAAGU | 17 | 3178 |
| HSV1-UL30-162 | - | GCGGCGCUCGCGCCUGU | 17 | 3321 |
| HSV1-UL30-106 | + | GUCCCCGGCUGGCUCCGCGA | 20 | 3286 |
| HSV1-UL30-58 | + | GUACACGUGAAAGACGGUGA | 20 | 3261 |
| HSV1-UL30-96 | + | GUCCCGACUGGGGCGAGGUA | 20 | 3279 |
| HSV1-UL30-118 | + | GGACAGCGGGCCGCCGCCAC | 20 | 3294 |
| HSV1-UL30-24 | - | GCUGGACGAGGAUGCCCCCC | 20 | 3247 |
| HSV1-UL30-1 | - | GGCGGCGGCCCGCUGUCCCC | 20 | 3232 |
| HSV1-UL30-41 | - | GCGUCGGGUCGGGCGGCUUC | 20 | 3251 |
| HSV1-UL30-116 | + | GACUUUCCUCCGGGGGACAG | 20 | 3292 |
| HSV1-UL30-14 | - | GGCCCUCGCGGAGCCAGCCG | 20 | 3242 |
| HSV1-UL30-42 | - | GGUCGGGCGGCUUCUGGCCG | 20 | 3252 |
| HSV1-UL30-105 | + | GGUCCCCGGCUGGCUCCGCG | 20 | 3285 |
| HSV1-UL30-2 | - | GGCGGCCCGCUGUCCCCCGG | 20 | 3233 |
| HSV1-UL30-33 | - | GCCCCCAAGGUGUACUGCGG | 20 | 3138 |
| HSV1-UL30-43 | - | GGCCGCGGCGCUCGCGCCUG | 20 | 3253 |
| HSV1-UL30-15 | - | GGGGACCCCCGCCUUGUUUG | 20 | 3243 |
| HSV1-UL30-44 | - | GCCGCGGCGCUCGCGCCUGU | 20 | 3254 |

**Table 8D** provides exemplary targeting domains for knocking out the *UL30* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 8D**

| **4th Tier** | | | | |
|---|---|---|---|---|
| **gRNA Name** | **DNA Strand** | **Targeting Domain** | **Target Site Length** | **Seq ID NO:** |
| HSV1-UL30-216 | + | AAGUUUUGCCUCAAACA | 17 | 3343 |
| HSV1-UL30-124 | - | AGGAAAGUCGGCGGCCA | 17 | 3299 |
| HSV1-UL30-146 | - | CGCGCCGGGGUGCACGA | 17 | 3187 |
| HSV1-UL30-224 | + | CCCGGCUGGCUCCGCGA | 17 | 3351 |
| HSV1-UL30-176 | + | CACGUGAAAGACGGUGA | 17 | 3326 |
| HSV1-UL30-214 | + | CCGACUGGGGCGAGGUA | 17 | 3342 |
| HSV1-UL30-236 | + | CAGCGGGCCGCCGCCAC | 17 | 3358 |
| HSV1-UL30-166 | - | CGGCGUGGACCACGCCC | 17 | 3323 |
| HSV1-UL30-221 | + | AACAAGGCGGGGGUCCC | 17 | 3348 |
| HSV1-UL30-131 | - | CCCUCGCGGAGCCAGCC | 17 | 3304 |
| HSV1-UL30-181 | + | UGGGGUUGAACCCCGCC | 17 | 3330 |
| HSV1-UL30-144 | - | CCCCGGAGAAGCGCGCC | 17 | 3186 |
| HSV1-UL30-207 | + | AUGGCGCUGGGUUGGCC | 17 | 3339 |
| HSV1-UL30-195 | + | CCCCGGCGCGCUUCUCC | 17 | 3336 |
| HSV1-UL30-174 | + | CGUGCUCCACGUUCUCC | 17 | 3325 |
| HSV1-UL30-127 | - | CGGCCAGGGCGGCGUCC | 17 | 3301 |
| HSV1-UL30-235 | + | UUCCUCCGGGGGACAGC | 17 | 3357 |
| HSV1-UL30-201 | + | CCUCGUCCAGCACCCGC | 17 | 3338 |
| HSV1-UL30-143 | - | CCCCCGGAGAAGCGCGC | 17 | 3311 |
| HSV1-UL30-218 | + | UUUGCCUCAAACAAGGC | 17 | 3345 |
| HSV1-UL30-222 | + | AGGCGGGGGUCCCCGGC | 17 | 3349 |
| HSV1-UL30-149 | - | CCCCCAAGGUGUACUGC | 17 | 3313 |
| HSV1-UL30-140 | - | CAUCGCCCCGCGGGUGC | 17 | 3185 |
| HSV1-UL30-194 | + | ACCCCGGCGCGCUUCUC | 17 | 3335 |
| HSV1-UL30-135 | - | CCUACCUCGCCCCAGUC | 17 | 3308 |
| HSV1-UL30-159 | - | UCGGGUCGGGCGGCUUC | 17 | 3318 |
| HSV1-UL30-234 | + | UUUCCUCCGGGGGACAG | 17 | 3356 |
| HSV1-UL30-171 | - | CGACAUCCUGGAGAACG | 17 | 3201 |
| HSV1-UL30-141 | - | CCCGCGGGUGCUGGACG | 17 | 3309 |
| HSV1-UL30-200 | + | UCCUCGUCCAGCACCCG | 17 | 3337 |
| HSV1-UL30-132 | - | CCUCGCGGAGCCAGCCG | 17 | 3305 |
| HSV1-UL30-145 | - | CCCGGAGAAGCGCGCCG | 17 | 3312 |
| HSV1-UL30-160 | - | CGGGCGGCUUCUGGCCG | 17 | 3319 |
| HSV1-UL30-2861 | - | CGGCCCAGUUCCACGCG | 17 | 11801 |
| HSV1-UL30-223 | + | CCCCGGCUGGCUCCGCG | 17 | 3350 |
| HSV1-UL30-219 | + | UUGCCUCAAACAAGGCG | 17 | 3346 |
| HSV1-UL30-183 | + | UGAACCCCGCCGGGGCG | 17 | 3331 |
| HSV1-UL30-217 | + | UUUUGCCUCAAACAAGG | 17 | 3344 |
| HSV1-UL30-197 | + | CCGGCGCGCUUCUCCGG | 17 | 3214 |
| HSV1-UL30-220 | + | UGCCUCAAACAAGGCGG | 17 | 3347 |
| HSV1-UL30-2862 | - | AUGUUUUCCGGUGGCGG | 17 | 11802 |
| HSV1-UL30-151 | - | CCCAAGGUGUACUGCGG | 17 | 3314 |
| HSV1-UL30-122 | - | CCCCGGAGGAAAGUCGG | 17 | 3297 |
| HSV1-UL30-125 | - | AAAGUCGGCGGCCAGGG | 17 | 3179 |
| HSV1-UL30-152 | - | CCAAGGUGUACUGCGGG | 17 | 3315 |
| HSV1-UL30-153 | - | CAAGGUGUACUGCGGGG | 17 | 3316 |
| HSV1-UL30-191 | + | CCCGCAGUACACCUUGG | 17 | 3333 |
| HSV1-UL30-161 | - | CGCGGCGCUCGCGCCUG | 17 | 3320 |
| HSV1-UL30-188 | + | CCCCCCGCAGUACACCU | 17 | 3209 |
| HSV1-UL30-134 | - | CCCUACCUCGCCCCAGU | 17 | 3307 |
| HSV1-UL30-213 | + | CCCGACUGGGGCGAGGU | 17 | 3341 |
| HSV1-UL30-208 | + | CGCUGGGUUGGCCCGGU | 17 | 3340 |
| HSV1-UL30-2863 | - | UCUUUAGGGGUUCGGGU | 17 | 11803 |
| HSV1-UL30-189 | + | CCCCCGCAGUACACCUU | 17 | 3210 |
| HSV1-UL30-98 | + | UAAAAGUUUUGCCUCAAACA | 20 | 3281 |
| HSV1-UL30-28 | - | AAGCGCGCCGGGGUGCACGA | 20 | 3133 |
| HSV1-UL30-111 | + | AAAAAACCCGGACGCCGCCC | 20 | 3175 |
| HSV1-UL30-13 | - | CGGCCCUCGCGGAGCCAGCC | 20 | 3241 |
| HSV1-UL30-26 | - | CCCCCCCGGAGAAGCGCGCC | 20 | 3248 |
| HSV1-UL30-5 | - | CCGGAGGAAAGUCGGCGGCC | 20 | 3124 |
| HSV1-UL30-9 | - | CGGCGGCCAGGGCGGCGUCC | 20 | 3238 |
| HSV1-UL30-100 | + | AGUUUUGCCUCAAACAAGGC | 20 | 3283 |
| HSV1-UL30-104 | + | ACAAGGCGGGGGUCCCCGGC | 20 | 3284 |
| HSV1-UL30-107 | + | CCGGCUGGCUCCGCGAGGGC | 20 | 3287 |
| HSV1-UL30-17 | - | ACCCCUACCUCGCCCCAGUC | 20 | 3245 |
| HSV1-UL30-8 | - | UCGGCGGCCAGGGCGGCGUC | 20 | 3237 |
| HSV1-UL30-97 | + | UCCCGACUGGGGCGAGGUAG | 20 | 3280 |
| HSV1-UL30-49 | - | CGGCGUGGACCACGCCCCGG | 20 | 3259 |
| HSV1-UL30-79 | + | ACCCCGGCGCGCUUCUCCGG | 20 | 3273 |
| HSV1-UL30-7 | - | AGGAAAGUCGGCGGCCAGGG | 20 | 3236 |
| HSV1-UL30-34 | - | CCCCCAAGGUGUACUGCGGG | 20 | 3249 |
| HSV1-UL30-81 | + | CCCGGCGCGCUUCUCCGGGG | 20 | 3274 |
| HSV1-UL30-35 | - | CCCCAAGGUGUACUGCGGGG | 20 | 3250 |
| HSV1-UL30-72 | + | CCCCCCCGCAGUACACCUUG | 20 | 3268 |
| HSV1-UL30-16 | - | AACCCCUACCUCGCCCCAGU | 20 | 3244 |
| HSV1-UL30-95 | + | CGUCCCGACUGGGGCGAGGU | 20 | 3170 |
| HSV1-UL30-71 | + | UCCCCCCCGCAGUACACCUU | 20 | 3267 |

**Table 8E** provides exemplary targeting domains for knocking out the *UL30* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a *S*. *pyogenes* Cas9 molecule that generates a double stranded break (Cas9 nuclease) or a single-stranded break (Cas9 nickase).

**Table 8E**

| **5th Tier** | | | | |
|---|---|---|---|---|
| **gRNA Name** | **DNA Strand** | **Targeting Domain** | **Target Site Length** | **Seq ID NO:** |
| HSV1-UL30-2864 | - | CCGAGAGUCUGUUAAAA | 17 | 11804 |
| HSV1-UL30-2865 | + | GCCGUACUGUUUCACAA | 17 | 11805 |
| HSV1-UL30-2866 | + | GGCGACGCCGUGGGCAA | 17 | 11806 |
| HSV1-UL30-2867 | - | GGUAAGAUGCUCAUCAA | 17 | 11807 |
| HSV1-UL30-2868 | + | UCAUGCUAGAGUAUCAA | 17 | 11808 |
| HSV1-UL30-2869 | + | AGCUUGGCCAGCAAGAA | 17 | 11809 |
| HSV1-UL30-2870 | - | CGCCUGGCCGACCAGAA | 17 | 11810 |
| HSV1-UL30-2871 | + | GUGCCCGGCCACCGGAA | 17 | 11811 |
| HSV1-UL30-2872 | - | GGGUACGGCCGCAUGAA | 17 | 11812 |
| HSV1-UL30-2873 | + | CGUGCCGUAAACGUGAA | 17 | 11813 |
| HSV1-UL30-2874 | - | AGCAAGAUAAAGGUGAA | 17 | 11814 |
| HSV1-UL30-2875 | - | GAAGCGCAGCAAGAUAA | 17 | 11815 |
| HSV1-UL30-2876 | + | GGCGUUAUUCCCAAACA | 17 | 11816 |
| HSV1-UL30-2062 | - | GUACUUUUACAUGAACA | 17 | 5095 |
| HSV1-UL30-2877 | - | CGGCGUCGCCCUGAACA | 17 | 11817 |
| HSV1-UL30-2878 | + | GGCCGUACUGUUUCACA | 17 | 11818 |
| HSV1-UL30-2879 | - | CAUCAUCUACGGGGACA | 17 | 11819 |
| HSV1-UL30-2880 | - | GCUGACGGACAUUUACA | 17 | 11820 |
| HSV1-UL30-2881 | + | GUCGAAUUCCAGAACCA | 17 | 11821 |
| HSV1-UL30-2725 | + | AAAGUCGAACACCACCA | 17 | 5723 |
| HSV1-UL30-2882 | + | AAACAGCAGGUCGACCA | 17 | 11822 |
| HSV1-UL30-2214 | - | AGGCACGUGGGGUACCA | 17 | 5230 |
| HSV1-UL30-2883 | - | AUUCUGCCGGACACCCA | 17 | 11823 |
| HSV1-UL30-2301 | - | UAGACGCCGCCGCCCCA | 17 | 5316 |
| HSV1-UL30-2884 | + | CGCCAUCUUGUCGCCCA | 17 | 11824 |
| HSV1-UL30-2885 | - | GCAUACGCCAUUGCCCA | 17 | 11825 |
| HSV1-UL30-2886 | - | GAACGAGCUGGCGGCCA | 17 | 11826 |
| HSV1-UL30-2887 | + | CAGGACGGCUUCGGCCA | 17 | 11827 |
| HSV1-UL30-2888 | - | GGGGUACCAGGGGGCCA | 17 | 11828 |
| HSV1-UL30-2889 | - | UAUCAACCGCACCUCCA | 17 | 11829 |
| HSV1-UL30-2890 | + | CAGGAGGACGUGCUCCA | 17 | 11830 |
| HSV1-UL30-2759 | + | GCGGCCGUACCCGUCCA | 17 | 5757 |
| HSV1-UL30-2745 | + | CAGCUGGCCCACCAGCA | 17 | 5743 |
| HSV1-UL30-2891 | - | UUCACGGGAGUGCAGCA | 17 | 11831 |
| HSV1-UL30-2892 | + | CUGCUUGUCCAGGAGCA | 17 | 11832 |
| HSV1-UL30-2893 | - | CGCGGCUCCGGACCGCA | 17 | 11833 |
| HSV1-UL30-2894 | - | AGAUCUCUGCGAGCGCA | 17 | 11834 |
| HSV1-UL30-2895 | + | GCCCGGGGACUCGCGCA | 17 | 11835 |
| HSV1-UL30-2676 | + | CCCGUAGAUGAUGCGCA | 17 | 5674 |
| HSV1-UL30-2896 | + | GCGGAUCUGCUUUCGCA | 17 | 11836 |
| HSV1-UL30-2897 | + | GCGGCAACGUGCAGGCA | 17 | 11837 |
| HSV1-UL30-2898 | - | GAUAAAGGUGAACGGCA | 17 | 11838 |
| HSV1-UL30-2899 | + | CUUGGCCGGGGAGGGCA | 17 | 11839 |
| HSV1-UL30-2900 | - | GCACCUGGAGGCGGGCA | 17 | 11840 |
| HSV1-UL30-2901 | + | CUGCAGUCCCUCGGGCA | 17 | 11841 |
| HSV1-UL30-2902 | - | CUUCGAUAUCGAAUGCA | 17 | 11842 |
| HSV1-UL30-2903 | - | GCAGCAGGCCGCCAUCA | 17 | 11843 |
| HSV1-UL30-2904 | - | GCAGGUCCCGUCCAUCA | 17 | 11844 |
| HSV1-UL30-2905 | - | GGGUAAGAUGCUCAUCA | 17 | 11845 |
| HSV1-UL30-2716 | + | CACUGCGUCGGCCCUCA | 17 | 5714 |
| HSV1-UL30-2906 | - | GCUGUGCCGCGGCCUCA | 17 | 11846 |
| HSV1-UL30-2907 | - | GGUGUAUUACAAGCUCA | 17 | 11847 |
| HSV1-UL30-2908 | - | ACGGCUGUUCUUCGUCA | 17 | 11848 |
| HSV1-UL30-2909 | + | ACCCGGAAGUGGGGUCA | 17 | 11849 |
| HSV1-UL30-2910 | - | GGCGUGCGUGACAUUCA | 17 | 11850 |
| HSV1-UL30-2246 | - | CUCGGUGUACGGGUUCA | 17 | 5262 |
| HSV1-UL30-2911 | + | GUAAUAGUCCGUGUUCA | 17 | 11851 |
| HSV1-UL30-1554 | + | GCGGCACAGCACAAAGA | 17 | 4492 |
| HSV1-UL30-2912 | - | GGCCGUGGGCGACAAGA | 17 | 11852 |
| HSV1-UL30-2913 | + | CAGCUUGGCCAGCAAGA | 17 | 11853 |
| HSV1-UL30-2914 | - | CCUGAAGGACAAGAAGA | 17 | 11854 |
| HSV1-UL30-2915 | - | GCGCCUGGCCGACCAGA | 17 | 11855 |
| HSV1-UL30-2742 | + | CGGCCGAGAGCUCCAGA | 17 | 5740 |
| HSV1-UL30-2916 | - | CCGCGAGGUAGAGGAGA | 17 | 11856 |
| HSV1-UL30-2917 | - | CAUCACCGACCCGGAGA | 17 | 11857 |
| HSV1-UL30-2918 | + | GGCGGCCGCUCCGGAGA | 17 | 11858 |
| HSV1-UL30-2919 | + | UCCGGCCCGGUUUGAGA | 17 | 11859 |
| HSV1-UL30-2920 | + | CUGCUGGCCGUCGUAGA | 17 | 11860 |
| HSV1-UL30-2921 | - | ACCCGCACCAUCUACGA | 17 | 11861 |
| HSV1-UL30-2922 | - | GCCAUCAAGAAGUACGA | 17 | 11862 |
| HSV1-UL30-2054 | - | GGACGCCAUCACACCGA | 17 | 5087 |
| HSV1-UL30-793 | - | CCAGCCGGCGGCCCCGA | 17 | 3731 |
| HSV1-UL30-2923 | - | GCGCGACCCCUGCCCGA | 17 | 11863 |
| HSV1-UL30-2924 | + | GUCGCUGGAUGUCCCGA | 17 | 11864 |
| HSV1-UL30-2925 | + | GAGACGGUACCAGCCGA | 17 | 11865 |
| HSV1-UL30-2926 | + | GGUCGGUGAUGCGCCGA | 17 | 11866 |
| HSV1-UL30-2927 | + | CAGCAUCUCGCGGCCGA | 17 | 11867 |
| HSV1-UL30-2928 | - | CCUGCACGUUGCCGCGA | 17 | 11868 |
| HSV1-UL30-1640 | + | ACCCGCCAAGCGCGCGA | 17 | 4578 |
| HSV1-UL30-2929 | + | GGGGGGUCGGCAGGCGA | 17 | 11869 |
| HSV1-UL30-2930 | - | UCGAGGUGGGGGGGCGA | 17 | 11870 |
| HSV1-UL30-2095 | - | GACAACCUGGCCAUCGA | 17 | 5128 |
| HSV1-UL30-2931 | + | GCUCAUGCCCCCCUCGA | 17 | 11871 |
| HSV1-UL30-2932 | + | GGCCAGGAGCUGUUCGA | 17 | 11872 |
| HSV1-UL30-2933 | + | CUUCUUGUCCUUCAGGA | 17 | 11873 |
| HSV1-UL30-2934 | + | AUGGGCGUCUACGAGGA | 17 | 11874 |
| HSV1-UL30-907 | - | GAGGACGAACGCGAGGA | 17 | 3845 |
| HSV1-UL30-900 | - | GAGCGGCCAGAGGAGGA | 17 | 3838 |
| HSV1-UL30-916 | - | GGCGAGCGGGAGCCGGA | 17 | 3854 |
| HSV1-UL30-2935 | - | CUGGGCCUGACUCCGGA | 17 | 11875 |
| HSV1-UL30-2936 | + | GGCGUCUAGCUCGCGGA | 17 | 11876 |
| HSV1-UL30-2937 | + | GGGCGCUUGGCCGGGGA | 17 | 11877 |
| HSV1-UL30-2640 | + | AUCCGGUCCUUGAUGGA | 17 | 5639 |
| HSV1-UL30-2938 | - | UACAAGGUCCCCCUGGA | 17 | 11878 |
| HSV1-UL30-2939 | + | UUUCACAAGGGUCAUGA | 17 | 11879 |
| HSV1-UL30-2940 | + | GCCCAGGAGCGUGAUGA | 17 | 11880 |
| HSV1-UL30-2941 | - | GCGCCUGGCCCACCUGA | 17 | 11881 |
| HSV1-UL30-2942 | - | CAGCACGCUCUCCCUGA | 17 | 11882 |
| HSV1-UL30-2943 | - | GGCCGAAGCCGUCCUGA | 17 | 11883 |
| HSV1-UL30-2944 | - | CUUGCUGGCCAAGCUGA | 17 | 11884 |
| HSV1-UL30-2945 | - | CACGGCCGCCGGGCUGA | 17 | 11885 |
| HSV1-UL30-2946 | + | GGUCCCCGUCGGUGUGA | 17 | 11886 |
| HSV1-UL30-2947 | + | GUUACACACGACCUUGA | 17 | 11887 |
| HSV1-UL30-2948 | + | CGGGAUCCGGUCCUUGA | 17 | 11888 |
| HSV1-UL30-2949 | + | ACCCUCGUACUUCUUGA | 17 | 11889 |
| HSV1-UL30-2667 | + | UUCGCACUCGAGUUUGA | 17 | 5666 |
| HSV1-UL30-2950 | + | AUGAUGCGCAUGGAAUA | 17 | 11890 |
| HSV1-UL30-2951 | - | GGUGGGUGCCGGCGCUA | 17 | 11891 |
| HSV1-UL30-2257 | - | UC CAUGC GCAUCAUCUA | 17 | 5273 |
| HSV1-UL30-2267 | - | UACAUCGGCGUCAUCUA | 17 | 5283 |
| HSV1-UL30-2952 | - | ACCCUUGUGAAACAGUA | 17 | 11892 |
| HSV1-UL30-2637 | + | UGGGCCACGAUCACGUA | 17 | 5636 |
| HSV1-UL30-2953 | - | GUCCCCCUGGACGGGUA | 17 | 11893 |
| HSV1-UL30-2140 | - | GUGAACAUCGACAUGUA | 17 | 5173 |
| HSV1-UL30-2954 | - | CGUCGAGUUUAACUGUA | 17 | 11894 |
| HSV1-UL30-2955 | - | GUGUGUAACUCGGUGUA | 17 | 11895 |
| HSV1-UL30-2956 | - | CACCCAGGGGCGAUUUA | 17 | 11896 |
| HSV1-UL30-2957 | - | GUGGCCGUUCACGUUUA | 17 | 11897 |
| HSV1-UL30-2843 | - | GUUCCACGCGCGGUUUA | 17 | 11783 |
| HSV1-UL30-1694 | + | AGGAACCGAGCGAAAAC | 17 | 4632 |
| HSV1-UL30-2958 | + | UGGCGUUAUUCCCAAAC | 17 | 11898 |
| HSV1-UL30-2959 | - | CUGGUACCGUCUCAAAC | 17 | 11899 |
| HSV1-UL30-1437 | + | CGCACGCCGCCCCCAAC | 17 | 4375 |
| HSV1-UL30-2850 | + | UAAACCGCGCGUGGAAC | 17 | 11790 |
| HSV1-UL30-2960 | + | GGUUGUCCAGGAUGAAC | 17 | 11900 |
| HSV1-UL30-2961 | + | UCGAAUUCCAGAACCAC | 17 | 11901 |
| HSV1-UL30-2962 | + | AAGUCGAACACCACCAC | 17 | 11902 |
| HSV1-UL30-2963 | - | UGACUCCGGAAGGCCAC | 17 | 11903 |
| HSV1-UL30-2794 | + | UCCGGGUGCCCGGCCAC | 17 | 5791 |
| HSV1-UL30-2964 | + | AGAGCGUGCUGAAGCAC | 17 | 11904 |
| HSV1-UL30-2965 | + | GGCAGAAGUUGUCGCAC | 17 | 11905 |
| HSV1-UL30-2247 | - | UCGGUGUACGGGUUCAC | 17 | 5263 |
| HSV1-UL30-2966 | + | CAGGUCGUAGAGCAGAC | 17 | 11906 |
| HSV1-UL30-2055 | - | GACGCCAUCACACCGAC | 17 | 5088 |
| HSV1-UL30-2967 | - | ACAAGGAGGAGGUCGAC | 17 | 11907 |
| HSV1-UL30-2968 | - | ACAUCAUCAACUUCGAC | 17 | 11908 |
| HSV1-UL30-2969 | - | UCCCGUCCAUCAAGGAC | 17 | 11909 |
| HSV1-UL30-2970 | - | GCAUCCUCCUGCGGGAC | 17 | 11910 |
| HSV1-UL30-2971 | + | UCCGGUCCUUGAUGGAC | 17 | 11911 |
| HSV1-UL30-2972 | - | ACAAGGUCCCCCUGGAC | 17 | 11912 |
| HSV1-UL30-2973 | + | CCAGGGCGGUGGUGGAC | 17 | 11913 |
| HSV1-UL30-2974 | - | GGCCCCGAGUUCGUGAC | 17 | 11914 |
| HSV1-UL30-2975 | - | CGGCGAGUACUGCAUAC | 17 | 11915 |
| HSV1-UL30-2976 | - | GGGCAUGAGCGACCUAC | 17 | 11916 |
| HSV1-UL30-2258 | - | CCAUGCGCAUCAUCUAC | 17 | 5274 |
| HSV1-UL30-2268 | - | ACAUCGGCGUCAUCUAC | 17 | 5284 |
| HSV1-UL30-2977 | + | GGGCCACGAUCACGUAC | 17 | 11917 |
| HSV1-UL30-2978 | + | GGAUGAUGCUGGGGUAC | 17 | 11918 |
| HSV1-UL30-2979 | - | UGAACAUCGACAUGUAC | 17 | 11919 |
| HSV1-UL30-2980 | - | UGUGUAACUCGGUGUAC | 17 | 11920 |
| HSV1-UL30-2981 | - | UGGUACCGUCUCAAACC | 17 | 11921 |
| HSV1-UL30-2728 | + | GGGGUUCACGUGAAACC | 17 | 5726 |
| HSV1-UL30-2982 | - | CUGUACGGCGGACAACC | 17 | 11922 |
| HSV1-UL30-2806 | + | GCCGAAGGUGACGAACC | 17 | 5803 |
| HSV1-UL30-2983 | + | GUUGUCCAGGAUGAACC | 17 | 11923 |
| HSV1-UL30-2984 | - | GGGUCGACGCCACCACC | 17 | 11924 |
| HSV1-UL30-2985 | - | GACUCCGGAAGGCCACC | 17 | 11925 |
| HSV1-UL30-2986 | + | AAACGUGAACGGCCACC | 17 | 11926 |
| HSV1-UL30-2221 | - | CGACGCAGUGGCGCACC | 17 | 5237 |
| HSV1-UL30-2111 | - | UCCGGUGGCCGGGCACC | 17 | 5144 |
| HSV1-UL30-2285 | - | UCGGCGCAUCACCGACC | 17 | 5300 |
| HSV1-UL30-2987 | + | AAAACAGCAGGUCGACC | 17 | 11927 |
| HSV1-UL30-821 | - | CGGGCACCCGGAGGACC | 17 | 3759 |
| HSV1-UL30-2988 | + | AGGAUAUCUGGAUGACC | 17 | 11928 |
| HSV1-UL30-2989 | - | GCCCCGAGUUCGUGACC | 17 | 11929 |
| HSV1-UL30-2225 | - | GGCGGGCAAGGACUACC | 17 | 5241 |
| HSV1-UL30-2213 | - | CAGGCACGUGGGGUACC | 17 | 5229 |
| HSV1-UL30-2990 | + | GUUGAUGAUGUUGUACC | 17 | 11930 |
| HSV1-UL30-2991 | - | UUCAUCCUGGACAACCC | 17 | 11931 |
| HSV1-UL30-2992 | + | CCGAAGGUGACGAACCC | 17 | 11932 |
| HSV1-UL30-2162 | - | UAUUCUGCCGGACACCC | 17 | 5194 |
| HSV1-UL30-2993 | + | AGUGGGGUCAAGGACCC | 17 | 11933 |
| HSV1-UL30-2994 | - | UCAUCCUGGACAACCCC | 17 | 11934 |
| HSV1-UL30-2995 | - | CGAAGUGUGGCACCCCC | 17 | 11935 |
| HSV1-UL30-2307 | - | GUCGCCUGCCGACCCCC | 17 | 5320 |
| HSV1-UL30-2308 | - | UCGCCUGCCGACCCCCC | 17 | 5321 |
| HSV1-UL30-2996 | + | CUUGGGCGCCUCCCCCC | 17 | 11936 |
| HSV1-UL30-1038 | - | CCCGGCCAAGCGCCCCC | 17 | 3976 |
| HSV1-UL30-2997 | - | CCGACAUGCGCGCCCCC | 17 | 11937 |
| HSV1-UL30-2998 | + | CAAGGACCCUGGCCCCC | 17 | 11938 |
| HSV1-UL30-2999 | - | CAUUUACAAGGUCCCCC | 17 | 11939 |
| HSV1-UL30-2300 | - | CUAGACGCCGCCGCCCC | 17 | 5315 |
| HSV1-UL30-1037 | - | CCCCGGCCAAGCGCCCC | 17 | 3975 |
| HSV1-UL30-3000 | - | GCCGACAUGCGCGCCCC | 17 | 11940 |
| HSV1-UL30-3001 | + | CGCCCCUAAAUCGCCCC | 17 | 11941 |
| HSV1-UL30-3002 | + | GGGCGUGGGCAGGCCCC | 17 | 11942 |
| HSV1-UL30-3003 | - | GCCCUGCGCGAGUCCCC | 17 | 11943 |
| HSV1-UL30-2630 | + | GGGGCGGGCUCGUCCCC | 17 | 5629 |
| HSV1-UL30-2174 | - | GCGUCCGGCCGCAGCCC | 17 | 5206 |
| HSV1-UL30-1664 | + | ACACGCGAAACACGCCC | 17 | 4602 |
| HSV1-UL30-2114 | - | CCUGUCCACCACCGCCC | 17 | 5147 |
| HSV1-UL30-1748 | + | CGCGGAACGACGCGCCC | 17 | 4686 |
| HSV1-UL30-2832 | + | CUUCCGGAGUCAGGCCC | 17 | 5827 |
| HSV1-UL30-2662 | + | CAGGUCGACCAGGGCCC | 17 | 5661 |
| HSV1-UL30-3004 | - | CCGCACCUCCAGGGCCC | 17 | 11944 |
| HSV1-UL30-2680 | + | CGCAUGGAAUAGGGCCC | 17 | 5678 |
| HSV1-UL30-3005 | - | CGCGGCCCUGCCCUCCC | 17 | 11945 |
| HSV1-UL30-2611 | + | GCUUGGACGCGCCUCCC | 17 | 5615 |
| HSV1-UL30-3006 | + | CCGCUCCUCGUCCUCCC | 17 | 11946 |
| HSV1-UL30-2615 | + | AGGCGACGGCGUCUCCC | 17 | 5619 |
| HSV1-UL30-3007 | - | GGCCCUGCGCGAGUCCC | 17 | 11947 |
| HSV1-UL30-2173 | - | AGCGUCCGGCCGCAGCC | 17 | 5205 |
| HSV1-UL30-3008 | + | GCCCACGGCCGUCAGCC | 17 | 11948 |
| HSV1-UL30-3009 | - | CCGCCUACUACGCCGCC | 17 | 11949 |
| HSV1-UL30-3010 | - | GCGGCCUCACGGCCGCC | 17 | 11950 |
| HSV1-UL30-3011 | - | GUACACCAACAAGCGCC | 17 | 11951 |
| HSV1-UL30-1747 | + | CCGCGGAACGACGCGCC | 17 | 4685 |
| HSV1-UL30-2167 | - | GGCGAUUUAGGGGCGCC | 17 | 5199 |
| HSV1-UL30-3012 | - | UACGUGCCUGCUGCGCC | 17 | 11952 |
| HSV1-UL30-3013 | - | CGGCCGCAUGAACGGCC | 17 | 11953 |
| HSV1-UL30-3014 | + | AGCGUGUUGUUCCGGCC | 17 | 11954 |
| HSV1-UL30-2119 | - | UGAACGAGCUGGCGGCC | 17 | 5152 |
| HSV1-UL30-2685 | + | CCUCCGGGAAAUCGGCC | 17 | 5683 |
| HSV1-UL30-3015 | + | AGCCCUUCUGGUCGGCC | 17 | 11955 |
| HSV1-UL30-2679 | + | GCGCAUGGAAUAGGGCC | 17 | 5677 |
| HSV1-UL30-3016 | + | CCCGCGUUGCGCGGGCC | 17 | 11956 |
| HSV1-UL30-3017 | - | UGGGGUACCAGGGGGCC | 17 | 11957 |
| HSV1-UL30-3018 | + | ACACCGUCAGGUGGGCC | 17 | 11958 |
| HSV1-UL30-3019 | + | AGCACAGGUUGUGGGCC | 17 | 11959 |
| HSV1-UL30-3020 | + | UGCCCCCCUCGAUGGCC | 17 | 11960 |
| HSV1-UL30-3021 | + | CGUACUUCUUGAUGGCC | 17 | 11961 |
| HSV1-UL30-3022 | - | UGGCCUUUCCGGUGGCC | 17 | 11962 |
| HSV1-UL30-2618 | + | CCCGGGGGCGCUUGGCC | 17 | 5622 |
| HSV1-UL30-3023 | - | GUGCGACAACUUCUGCC | 17 | 11963 |
| HSV1-UL30-3024 | + | CAGGUCCUCCGGGUGCC | 17 | 11964 |
| HSV1-UL30-3025 | - | CCCGGAGAGGGACAUCC | 17 | 11965 |
| HSV1-UL30-3026 | - | GUACCCCAGCAUCAUCC | 17 | 11966 |
| HSV1-UL30-3027 | - | CACCACCCGGUUCAUCC | 17 | 11967 |
| HSV1-UL30-3028 | - | UUAUCAACCGCACCUCC | 17 | 11968 |
| HSV1-UL30-3029 | + | AGUCCUUGCCCGCCUCC | 17 | 11969 |
| HSV1-UL30-2683 | + | GCAUGUCGGCCGCCUCC | 17 | 5681 |
| HSV1-UL30-2610 | + | GGCUUGGACGCGCCUCC | 17 | 5614 |
| HSV1-UL30-3030 | + | GCCGCUCCUCGUCCUCC | 17 | 11970 |
| HSV1-UL30-3031 | + | GGAUGACCAGGUCCUCC | 17 | 11971 |
| HSV1-UL30-3032 | - | GGCCUUCGAACAGCUCC | 17 | 11972 |
| HSV1-UL30-3033 | - | GACCGUCAUCACGCUCC | 17 | 11973 |
| HSV1-UL30-3034 | + | ACAGGAGGACGUGCUCC | 17 | 11974 |
| HSV1-UL30-3035 | - | CGAGGAGGCCGUGCUCC | 17 | 11975 |
| HSV1-UL30-3036 | + | CCCCCACCUCGAUCUCC | 17 | 11976 |
| HSV1-UL30-3037 | + | CCUGGAUGUCCCUCUCC | 17 | 11977 |
| HSV1-UL30-2614 | + | CAGGCGACGGCGUCUCC | 17 | 5618 |
| HSV1-UL30-3038 | + | CGGUGAGGACAAAGUCC | 17 | 11978 |
| HSV1-UL30-2758 | + | UGCGGCCGUACCCGUCC | 17 | 5756 |
| HSV1-UL30-2590 | + | GCGCGGCCACGUCGUCC | 17 | 5595 |
| HSV1-UL30-2695 | + | CGGCCUGCUGCUUGUCC | 17 | 5693 |
| HSV1-UL30-3039 | + | CGAACCCGGGGUUGUCC | 17 | 11979 |
| HSV1-UL30-3040 | - | UCCUUGACCCCACUUCC | 17 | 11980 |
| HSV1-UL30-2254 | - | GCUCCUGGCCGAUUUCC | 17 | 5270 |
| HSV1-UL30-3041 | - | GCGUCUACGUCCGAAGC | 17 | 11981 |
| HSV1-UL30-3042 | + | AAAACUUAAAAAACAGC | 17 | 11982 |
| HSV1-UL30-3043 | + | UAUCGUCGUAAAACAGC | 17 | 11983 |
| HSV1-UL30-2744 | + | ACAGCUGGCCCACCAGC | 17 | 5742 |
| HSV1-UL30-3044 | - | CAACACGCUAGCCCAGC | 17 | 11984 |
| HSV1-UL30-3045 | - | GCUCCUGGACAAGCAGC | 17 | 11985 |
| HSV1-UL30-3046 | + | GGUCGGCCAGGCGCAGC | 17 | 11986 |
| HSV1-UL30-3047 | + | ACGCGGUAAAACAGAGC | 17 | 11987 |
| HSV1-UL30-3048 | - | AUCCCACCUGAACGAGC | 17 | 11988 |
| HSV1-UL30-814 | - | GGGGGGGGAGGACGAGC | 17 | 3752 |
| HSV1-UL30-3049 | - | GCUGCUGGUGUCCGAGC | 17 | 11989 |
| HSV1-UL30-913 | - | GGAGGGCGGGGGCGAGC | 17 | 3851 |
| HSV1-UL30-914 | - | CGGGGGCGAGCGGGAGC | 17 | 3852 |
| HSV1-UL30-861 | - | CCGGGCCCGCGCAACGC | 17 | 3799 |
| HSV1-UL30-919 | - | GGCGCGCGGGAGACCGC | 17 | 3857 |
| HSV1-UL30-3050 | - | GCGCGGCUCCGGACCGC | 17 | 11990 |
| HSV1-UL30-2706 | + | UGGCGAGCCAGUCCCGC | 17 | 5704 |
| HSV1-UL30-3051 | - | CCCGCCUACUACGCCGC | 17 | 11991 |
| HSV1-UL30-3052 | - | CGCGGCCUCACGGCCGC | 17 | 11992 |
| HSV1-UL30-1474 | + | GGGGAGGGCAGGGCCGC | 17 | 4412 |
| HSV1-UL30-3053 | + | AAGGCCAUCGGGGCCGC | 17 | 11993 |
| HSV1-UL30-3054 | + | GCCAGCCACUCCUCCGC | 17 | 11994 |
| HSV1-UL30-3055 | + | UUCCUCCGCCGUAGCGC | 17 | 11995 |
| HSV1-UL30-3056 | - | AGUACGUCCACGCGCGC | 17 | 11996 |
| HSV1-UL30-3057 | + | UGUUGGUGUACGCGCGC | 17 | 11997 |
| HSV1-UL30-3058 | - | CAUGGCCCGCCGCGCGC | 17 | 11998 |
| HSV1-UL30-918 | - | GGAGCCGGAGGGCGCGC | 17 | 3856 |
| HSV1-UL30-3059 | + | CGCCCGGGGACUCGCGC | 17 | 11999 |
| HSV1-UL30-2166 | - | GGGCGAUUUAGGGGCGC | 17 | 5198 |
| HSV1-UL30-3060 | + | AUCACCCCGCGUUGCGC | 17 | 12000 |
| HSV1-UL30-3061 | + | CGCGUGGACGUACUCGC | 17 | 12001 |
| HSV1-UL30-3062 | + | CGUCUCCUCUACCUCGC | 17 | 12002 |
| HSV1-UL30-3063 | + | GUUAAACUCGACGUCGC | 17 | 12003 |
| HSV1-UL30-2781 | + | GGAUUCGGGGAGGUCGC | 17 | 5779 |
| HSV1-UL30-2101 | - | GAUAUCGAAUGCAAGGC | 17 | 5134 |
| HSV1-UL30-3064 | + | GAUGCUGGGGUACAGGC | 17 | 12004 |
| HSV1-UL30-3065 | - | GUGGCGCACCUGGAGGC | 17 | 12005 |
| HSV1-UL30-2749 | + | CCGGCGGCGUAGUAGGC | 17 | 5747 |
| HSV1-UL30-2132 | - | ACGGCCGCAUGAACGGC | 17 | 5165 |
| HSV1-UL30-2691 | + | CAACGUGCAGGCACGGC | 17 | 5689 |
| HSV1-UL30-920 | - | CGCGGGAGACCGCCGGC | 17 | 3858 |
| HSV1-UL30-3066 | + | CCAUCGGGGCCGCCGGC | 17 | 12006 |
| HSV1-UL30-3067 | + | CGGGACCUGCGCGCGGC | 17 | 12007 |
| HSV1-UL30-3068 | - | GGAGACGGUCGCGCGGC | 17 | 12008 |
| HSV1-UL30-3069 | + | UCCUCCCGGGCUGCGGC | 17 | 12009 |
| HSV1-UL30-3070 | + | CCUCCCGGGGGGUCGGC | 17 | 12010 |
| HSV1-UL30-3071 | - | GGUUCGUCACCUUCGGC | 17 | 12011 |
| HSV1-UL30-909 | - | ACGAACGCGAGGAGGGC | 17 | 3847 |
| HSV1-UL30-3072 | + | GCUUGGCCGGGGAGGGC | 17 | 12012 |
| HSV1-UL30-2087 | - | ACCGUCUCAAACCGGGC | 17 | 5120 |
| HSV1-UL30-2655 | + | CCUGCAGUCCCUCGGGC | 17 | 5654 |
| HSV1-UL30-1481 | + | AGGGCCGCGGGGGGGGC | 17 | 4419 |
| HSV1-UL30-3073 | + | GAACCACGGGCGUGGGC | 17 | 12013 |
| HSV1-UL30-3074 | + | UCGUACUUCUUGAUGGC | 17 | 12014 |
| HSV1-UL30-3075 | - | CCCCGCGGAGGAGUGGC | 17 | 12015 |
| HSV1-UL30-3076 | - | CUGGCCUUUCCGGUGGC | 17 | 12016 |
| HSV1-UL30-3077 | - | GCCGUCGCGCGCUUGGC | 17 | 12017 |
| HSV1-UL30-2617 | + | UCCCGGGGGCGCUUGGC | 17 | 5621 |
| HSV1-UL30-2722 | + | GUGGGCCUGGAUGAUGC | 17 | 5720 |
| HSV1-UL30-3078 | + | UGGGCAAUGGCGUAUGC | 17 | 12018 |
| HSV1-UL30-3079 | + | CACAUGAGCUUGUAUGC | 17 | 12019 |
| HSV1-UL30-3080 | - | CCUGCCCGAGGGACUGC | 17 | 12020 |
| HSV1-UL30-3081 | - | CAUACAGGAUUCCCUGC | 17 | 12021 |
| HSV1-UL30-3082 | + | GUACCCCACGUGCCUGC | 17 | 12022 |
| HSV1-UL30-3083 | - | CCUCAGCAUCCUCCUGC | 17 | 12023 |
| HSV1-UL30-3084 | - | CAAGCCCCGCAAGCUGC | 17 | 12024 |
| HSV1-UL30-3085 | + | UAAAGACGCGGAUCUGC | 17 | 12025 |
| HSV1-UL30-3086 | - | GAAGGGCUUUAUUCUGC | 17 | 12026 |
| HSV1-UL30-3087 | + | CCGUCGCGGCAACGUGC | 17 | 12027 |
| HSV1-UL30-3088 | - | UUCGGGGCGGUGGGUGC | 17 | 12028 |
| HSV1-UL30-3089 | + | GCCGUCGUAGAUGGUGC | 17 | 12029 |
| HSV1-UL30-3090 | + | GGACACCAGCAGCUUGC | 17 | 12030 |
| HSV1-UL30-3091 | - | CGACUGGCCCUUCUUGC | 17 | 12031 |
| HSV1-UL30-2156 | - | UAAGUUUUUGCCCCAUC | 17 | 5188 |
| HSV1-UL30-3092 | + | AUGUCCCGAAGGCCAUC | 17 | 12032 |
| HSV1-UL30-3093 | + | CGAUCACGUACGGGAUC | 17 | 12033 |
| HSV1-UL30-3094 | - | CAUCAAGGGCGUGGAUC | 17 | 12034 |
| HSV1-UL30-3095 | + | AGAGCAGACAGGAUAUC | 17 | 12035 |
| HSV1-UL30-3096 | + | UACCCGGUCACGAACUC | 17 | 12036 |
| HSV1-UL30-2058 | - | GCUCCUGGGCCUGACUC | 17 | 5091 |
| HSV1-UL30-3097 | + | GUCUCCCGCGCGCCCUC | 17 | 12037 |
| HSV1-UL30-2715 | + | CCACUGCGUCGGCCCUC | 17 | 5713 |
| HSV1-UL30-3098 | + | AACGCCUGCAGUCCCUC | 17 | 12038 |
| HSV1-UL30-2682 | + | CGCAUGUCGGCCGCCUC | 17 | 5680 |
| HSV1-UL30-3099 | + | AGGAGCACGGCCUCCUC | 17 | 12039 |
| HSV1-UL30-3100 | + | UCCUCCCCCUCCUCCUC | 17 | 12040 |
| HSV1-UL30-3101 | + | UGGAUGACCAGGUCCUC | 17 | 12041 |
| HSV1-UL30-3102 | + | GGAUGUCGCGAUAGCUC | 17 | 12042 |
| HSV1-UL30-2659 | + | UAACGCGGCGGCCGCUC | 17 | 5658 |
| HSV1-UL30-3103 | - | ACGUGGCCGCGCGGCUC | 17 | 12043 |
| HSV1-UL30-3104 | + | GCUUCUGGAAGUGGCUC | 17 | 12044 |
| HSV1-UL30-2699 | + | CCUCCUCGGGGCUGCUC | 17 | 5697 |
| HSV1-UL30-3105 | + | UCCUGGAUGUCCCUCUC | 17 | 12045 |
| HSV1-UL30-2273 | - | UACGACGAUACCGUCUC | 17 | 5289 |
| HSV1-UL30-3106 | + | GGUGGCCUUCCGGAGUC | 17 | 12046 |
| HSV1-UL30-3107 | + | GCUUGUAAUACACCGUC | 17 | 12047 |
| HSV1-UL30-3108 | - | GGAGGCGCCCAAGCGUC | 17 | 12048 |
| HSV1-UL30-2589 | + | CGCGCGGCCACGUCGUC | 17 | 5594 |
| HSV1-UL30-2587 | + | CCCCGAACCCUGCGGUC | 17 | 5592 |
| HSV1-UL30-3109 | + | CCUCUACCUCGCGGGUC | 17 | 12049 |
| HSV1-UL30-3110 | + | AAUCGCCCCUGGGUGUC | 17 | 12050 |
| HSV1-UL30-2778 | + | UCGUUCAGGUGGGAUUC | 17 | 5776 |
| HSV1-UL30-2594 | + | GGGGGGUGCCACACUUC | 17 | 5599 |
| HSV1-UL30-3111 | - | GUCCUUGACCCCACUUC | 17 | 12051 |
| HSV1-UL30-3112 | + | GCAGAAUAAAGCCCUUC | 17 | 12052 |
| HSV1-UL30-3113 | - | CGGCCCCGAUGGCCUUC | 17 | 12053 |
| HSV1-UL30-2830 | + | GCCACCCGGUGGCCUUC | 17 | 5825 |
| HSV1-UL30-3114 | + | CCUUCUUCUUGUCCUUC | 17 | 12054 |
| HSV1-UL30-2755 | + | UUAUCUUGCUGCGCUUC | 17 | 5753 |
| HSV1-UL30-3115 | - | AGGGACUGCAGGCGUUC | 17 | 12055 |
| HSV1-UL30-3116 | + | UGGCCGCCAGCUCGUUC | 17 | 12056 |
| HSV1-UL30-3117 | - | UCCGGACCGCAGGGUUC | 17 | 12057 |
| HSV1-UL30-2121 | - | GCCCACGCCCGUGGUUC | 17 | 5154 |
| HSV1-UL30-3118 | + | AGUAAUAGUCCGUGUUC | 17 | 12058 |
| HSV1-UL30-3119 | + | GGGCUAGCGUGUUGUUC | 17 | 12059 |
| HSV1-UL30-3120 | - | GGACGAGCUGGCCUUUC | 17 | 12060 |
| HSV1-UL30-3121 | - | CGCGUCUACGUCCGAAG | 17 | 12061 |
| HSV1-UL30-3122 | + | CCACCUCCGCCUCGAAG | 17 | 12062 |
| HSV1-UL30-2730 | + | CACGUGAAACCCGGAAG | 17 | 5728 |
| HSV1-UL30-3123 | + | UGCUGCGCUUCUGGAAG | 17 | 12063 |
| HSV1-UL30-3124 | - | GGCACGUGGGGUACCAG | 17 | 12064 |
| HSV1-UL30-2840 | - | UUCUGCCGGACACCCAG | 17 | 5835 |
| HSV1-UL30-1034 | - | AGACGCCGCCGCCCCAG | 17 | 3972 |
| HSV1-UL30-897 | - | GGACGAGGAGCGGCCAG | 17 | 3835 |
| HSV1-UL30-3125 | - | AACGAGCUGGCGGCCAG | 17 | 12065 |
| HSV1-UL30-2760 | + | CGGCCGUACCCGUCCAG | 17 | 5758 |
| HSV1-UL30-3126 | - | CCUGAGGGCCGACGCAG | 17 | 12066 |
| HSV1-UL30-3127 | + | UGCAGUCCCUCGGGCAG | 17 | 12067 |
| HSV1-UL30-2815 | + | GACGCGGUAAAACAGAG | 17 | 5812 |
| HSV1-UL30-3128 | + | UCUGGGGAAUCCGCGAG | 17 | 12068 |
| HSV1-UL30-912 | - | AGGAGGGCGGGGGCGAG | 17 | 3850 |
| HSV1-UL30-2096 | - | ACAACCUGGCCAUCGAG | 17 | 5129 |
| HSV1-UL30-896 | - | CCCGGGAGGACGAGGAG | 17 | 3834 |
| HSV1-UL30-901 | - | AGCGGCCAGAGGAGGAG | 17 | 3839 |
| HSV1-UL30-1007 | - | AGCGCCCCGCGGAGGAG | 17 | 3945 |
| HSV1-UL30-2288 | - | GCAUCACCGACCCGGAG | 17 | 5303 |
| HSV1-UL30-3129 | - | CGAUACCGUCUCCGGAG | 17 | 12069 |
| HSV1-UL30-2297 | - | CCAGACCCGCGAGGUAG | 17 | 5312 |
| HSV1-UL30-3130 | - | ACCCAGGGGCGAUUUAG | 17 | 12070 |
| HSV1-UL30-860 | - | GCCGGGCCCGCGCAACG | 17 | 3798 |
| HSV1-UL30-3131 | + | GGGGCGCUCGGCUAACG | 17 | 12071 |
| HSV1-UL30-3132 | + | AGUCGAACACCACCACG | 17 | 12072 |
| HSV1-UL30-921 | - | GACCGCCGGCAGGCACG | 17 | 3859 |
| HSV1-UL30-3133 | - | UUCACGUUUACGGCACG | 17 | 12073 |
| HSV1-UL30-3134 | + | GCAGGCACGUAAAGACG | 17 | 12074 |
| HSV1-UL30-3135 | + | CGCUUCGGACGUAGACG | 17 | 12075 |
| HSV1-UL30-1058 | - | GCACCCCCCGGACGACG | 17 | 3996 |
| HSV1-UL30-3136 | - | ACGCCAUCACACCGACG | 17 | 12076 |
| HSV1-UL30-895 | - | CGCAGCCCGGGAGGACG | 17 | 3833 |
| HSV1-UL30-904 | - | GGAGGAGGGGGAGGACG | 17 | 3842 |
| HSV1-UL30-3137 | - | CAUGCGCAUCAUCUACG | 17 | 12077 |
| HSV1-UL30-3138 | - | CAUCGGCGUCAUCUACG | 17 | 12078 |
| HSV1-UL30-3139 | + | GCCGAUGGGCGUCUACG | 17 | 12079 |
| HSV1-UL30-3140 | - | GGCCAUCAAGAAGUACG | 17 | 12080 |
| HSV1-UL30-3141 | + | CGAAGGUGACGAACCCG | 17 | 12081 |
| HSV1-UL30-3142 | - | GUUUCACGUGAACCCCG | 17 | 12082 |
| HSV1-UL30-1035 | - | CGAGCCCGCCCCCCCCG | 17 | 3973 |
| HSV1-UL30-2242 | - | UCCCCAGAGCAGCCCCG | 17 | 5258 |
| HSV1-UL30-2276 | - | GUUAGCCGAGCGCCCCG | 17 | 5292 |
| HSV1-UL30-3143 | - | GGGCCUGCCCACGCCCG | 17 | 12083 |
| HSV1-UL30-1749 | + | GCGGAACGACGCGCCCG | 17 | 4687 |
| HSV1-UL30-3144 | + | GCAUGGAAUAGGGCCCG | 17 | 12084 |
| HSV1-UL30-2281 | - | GGCGCGACCCCUGCCCG | 17 | 5296 |
| HSV1-UL30-2612 | + | CUUGGACGCGCCUCCCG | 17 | 5616 |
| HSV1-UL30-3145 | + | GGCGACGGCGUCUCCCG | 17 | 12085 |
| HSV1-UL30-3146 | - | CUACAAGCUCAACGCCG | 17 | 12086 |
| HSV1-UL30-3147 | + | UGUUCAGGGCGACGCCG | 17 | 12087 |
| HSV1-UL30-2168 | - | GCGAUUUAGGGGCGCCG | 17 | 5200 |
| HSV1-UL30-3148 | + | CGGCGGCCGUGAGGCCG | 17 | 12088 |
| HSV1-UL30-3149 | - | GGCCGCAUGAACGGCCG | 17 | 12089 |
| HSV1-UL30-3150 | - | CGCCGGGCUGACGGCCG | 17 | 12090 |
| HSV1-UL30-3151 | + | GGACUCGCGCAGGGCCG | 17 | 12091 |
| HSV1-UL30-1473 | + | CGGGGAGGGCAGGGCCG | 17 | 4411 |
| HSV1-UL30-3152 | - | CUGCGAGCGCAUGGCCG | 17 | 12092 |
| HSV1-UL30-3153 | - | GGUGUCCGAGCUGGCCG | 17 | 12093 |
| HSV1-UL30-2619 | + | CCGGGGGCGCUUGGCCG | 17 | 5623 |
| HSV1-UL30-2820 | + | GGUCCGCGGAGAUGCCG | 17 | 5817 |
| HSV1-UL30-3154 | - | AUCUUUGUGCUGUGCCG | 17 | 12094 |
| HSV1-UL30-2657 | + | CGCCAGCCACUCCUCCG | 17 | 5656 |
| HSV1-UL30-3155 | - | GUUCCGCGGCAUCUCCG | 17 | 12095 |
| HSV1-UL30-2591 | + | CGCGGCCACGUCGUCCG | 17 | 5596 |
| HSV1-UL30-2818 | + | CGCCUCGAAGUGGUCCG | 17 | 5815 |
| HSV1-UL30-3156 | - | CCGGGCGCGUCGUUCCG | 17 | 12096 |
| HSV1-UL30-3157 | + | CGCGGUAAAACAGAGCG | 17 | 12097 |
| HSV1-UL30-862 | - | CGGGCCCGCGCAACGCG | 17 | 3800 |
| HSV1-UL30-905 | - | GGACGAGGACGAACGCG | 17 | 3843 |
| HSV1-UL30-2861 | - | CGGCCCAGUUCCACGCG | 17 | 11801 |
| HSV1-UL30-3158 | - | CGUGGCCCAGACCCGCG | 17 | 12098 |
| HSV1-UL30-3159 | + | UGCGGUCCGGAGCCGCG | 17 | 12099 |
| HSV1-UL30-1475 | + | GGGAGGGCAGGGCCGCG | 17 | 4413 |
| HSV1-UL30-3160 | - | CGGACGACGUGGCCGCG | 17 | 12100 |
| HSV1-UL30-3161 | + | CCAGCCACUCCUCCGCG | 17 | 12101 |
| HSV1-UL30-3162 | + | UUGUUGGUGUACGCGCG | 17 | 12102 |
| HSV1-UL30-2852 | + | CGUCCAUAAACCGCGCG | 17 | 11792 |
| HSV1-UL30-1568 | + | AGGCCGCCCAGCGCGCG | 17 | 4506 |
| HSV1-UL30-917 | - | GGGAGCCGGAGGGCGCG | 17 | 3855 |
| HSV1-UL30-173 | - | CGCGUACGGCAUGCGCG | 17 | 3324 |
| HSV1-UL30-3163 | + | UGGACGGGACCUGCGCG | 17 | 12103 |
| HSV1-UL30-3164 | - | UAGAGGAGACGGUCGCG | 17 | 12104 |
| HSV1-UL30-2102 | - | AUAUCGAAUGCAAGGCG | 17 | 5135 |
| HSV1-UL30-3165 | + | CGGCGGCGUAGUAGGCG | 17 | 12105 |
| HSV1-UL30-3166 | - | GAUGCUCAUCAAGGGCG | 17 | 12106 |
| HSV1-UL30-910 | - | CGAACGCGAGGAGGGCG | 17 | 3848 |
| HSV1-UL30-3167 | + | UUCCAGAACCACGGGCG | 17 | 12107 |
| HSV1-UL30-3168 | + | AGAGAUCUCGUGGGGCG | 17 | 12108 |
| HSV1-UL30-3169 | + | GACACCAGCAGCUUGCG | 17 | 12109 |
| HSV1-UL30-3170 | + | GAUCACCCCGCGUUGCG | 17 | 12110 |
| HSV1-UL30-3171 | + | UGUCCCGAAGGCCAUCG | 17 | 12111 |
| HSV1-UL30-2094 | - | GGACAACCUGGCCAUCG | 17 | 5127 |
| HSV1-UL30-3172 | - | GGACUACCUGGAGAUCG | 17 | 12112 |
| HSV1-UL30-3173 | - | GAUCCCGUACGUGAUCG | 17 | 12113 |
| HSV1-UL30-3174 | + | ACCCGGUCACGAACUCG | 17 | 12114 |
| HSV1-UL30-3175 | + | GCGCGUGGACGUACUCG | 17 | 12115 |
| HSV1-UL30-3176 | + | CCGUCUCCUCUACCUCG | 17 | 12116 |
| HSV1-UL30-3177 | + | GGAGCACGGCCUCCUCG | 17 | 12117 |
| HSV1-UL30-2632 | + | CGGCGGCGUCUAGCUCG | 17 | 5631 |
| HSV1-UL30-3178 | - | GAAAGCAGAUCCGCUCG | 17 | 12118 |
| HSV1-UL30-3179 | + | UCGCGAGCAGCAUCUCG | 17 | 12119 |
| HSV1-UL30-2827 | + | CGCUCGCAGAGAUCUCG | 17 | 5822 |
| HSV1-UL30-3180 | + | GAUGGUCGUCACCGUCG | 17 | 12120 |
| HSV1-UL30-2779 | + | CGUUCAGGUGGGAUUCG | 17 | 5777 |
| HSV1-UL30-3181 | - | CUCCGCGGACCACUUCG | 17 | 12121 |
| HSV1-UL30-3182 | - | GGGACUGCAGGCGUUCG | 17 | 12122 |
| HSV1-UL30-3183 | - | CCGGACCGCAGGGUUCG | 17 | 12123 |
| HSV1-UL30-2064 | - | CUUUUACAUGAACAAGG | 17 | 5097 |
| HSV1-UL30-2100 | - | CGAUAUCGAAUGCAAGG | 17 | 5133 |
| HSV1-UL30-1695 | + | AACCGAGCGAAAACAGG | 17 | 4633 |
| HSV1-UL30-1438 | + | ACGCCGCCCCCAACAGG | 17 | 4376 |
| HSV1-UL30-3184 | - | GCACGUGGGGUACCAGG | 17 | 12124 |
| HSV1-UL30-3185 | + | GGCCGUACCCGUCCAGG | 17 | 12125 |
| HSV1-UL30-3186 | + | CGAGCCAGUCCCGCAGG | 17 | 12126 |
| HSV1-UL30-3187 | + | UGUAAUACACCGUCAGG | 17 | 12127 |
| HSV1-UL30-2775 | + | CCGCCAGCUCGUUCAGG | 17 | 5773 |
| HSV1-UL30-898 | - | CGAGGAGCGGCCAGAGG | 17 | 3836 |
| HSV1-UL30-958 | - | CCAGAGCAGCCCCGAGG | 17 | 3896 |
| HSV1-UL30-906 | - | CGAGGACGAACGCGAGG | 17 | 3844 |
| HSV1-UL30-3188 | - | CAACCUGGCCAUCGAGG | 17 | 12128 |
| HSV1-UL30-2228 | - | CUACCUGGAGAUCGAGG | 17 | 5244 |
| HSV1-UL30-2072 | - | CGCGGACCACUUCGAGG | 17 | 5105 |
| HSV1-UL30-3189 | - | UUACAUGAACAAGGAGG | 17 | 12129 |
| HSV1-UL30-899 | - | GGAGCGGCCAGAGGAGG | 17 | 3837 |
| HSV1-UL30-902 | - | GCGGCCAGAGGAGGAGG | 17 | 3840 |
| HSV1-UL30-3190 | - | GGCCGAUUUCCCGGAGG | 17 | 12130 |
| HSV1-UL30-3191 | - | CCACUUCGAGGCGGAGG | 17 | 12131 |
| HSV1-UL30-3192 | - | AGUGGCGCACCUGGAGG | 17 | 12132 |
| HSV1-UL30-3193 | + | GCAGGAGGAUGCUGAGG | 17 | 12133 |
| HSV1-UL30-2748 | + | CCCGGCGGCGUAGUAGG | 17 | 5746 |
| HSV1-UL30-2330 | - | GGGUGCCGGCGCUACGG | 17 | 5343 |
| HSV1-UL30-3194 | - | AUCGGCGUCAUCUACGG | 17 | 12134 |
| HSV1-UL30-3195 | - | CGAGUUUAACUGUACGG | 17 | 12135 |
| HSV1-UL30-3196 | + | CGUGAACGGCCACCCGG | 17 | 12136 |
| HSV1-UL30-3197 | - | GGUGGCCGGGCACCCGG | 17 | 12137 |
| HSV1-UL30-3198 | + | CACGGCCGUCAGCCCGG | 17 | 12138 |
| HSV1-UL30-3199 | + | CAUGGAAUAGGGCCCGG | 17 | 12139 |
| HSV1-UL30-3200 | + | GCGUUGCGCGGGCCCGG | 17 | 12140 |
| HSV1-UL30-3201 | + | UUGGACGCGCCUCCCGG | 17 | 12141 |
| HSV1-UL30-3202 | + | GCGACGGCGUCUCCCGG | 17 | 12142 |
| HSV1-UL30-3203 | - | CCUGGCCGAUUUCCCGG | 17 | 12143 |
| HSV1-UL30-3204 | - | CACGCUAGCCCAGCCGG | 17 | 12144 |
| HSV1-UL30-915 | - | GGGCGAGCGGGAGCCGG | 17 | 3853 |
| HSV1-UL30-2169 | - | CGAUUUAGGGGCGCCGG | 17 | 5201 |
| HSV1-UL30-3205 | + | CCCCACGUGCCUGCCGG | 17 | 12145 |
| HSV1-UL30-3206 | + | GCGGCCACGUCGUCCGG | 17 | 12146 |
| HSV1-UL30-3207 | - | CGAGCUGGCCUUUCCGG | 17 | 12147 |
| HSV1-UL30-3208 | + | GCGCUCGGCUAACGCGG | 17 | 12148 |
| HSV1-UL30-1006 | - | AGCCGAGCGCCCCGCGG | 17 | 3944 |
| HSV1-UL30-1476 | + | GGAGGGCAGGGCCGCGG | 17 | 4414 |
| HSV1-UL30-3209 | + | ACGGGACCUGCGCGCGG | 17 | 12149 |
| HSV1-UL30-3210 | + | CGGCGUCUAGCUCGCGG | 17 | 12150 |
| HSV1-UL30-2103 | - | UAUCGAAUGCAAGGCGG | 17 | 5136 |
| HSV1-UL30-3211 | - | GGACCACUUCGAGGCGG | 17 | 12151 |
| HSV1-UL30-2332 | - | UGCCGGCGCUACGGCGG | 17 | 5345 |
| HSV1-UL30-3212 | + | GACGUGCUCCAGGGCGG | 17 | 12152 |
| HSV1-UL30-911 | - | GAACGCGAGGAGGGCGG | 17 | 3849 |
| HSV1-UL30-3213 | - | CGCAGGGUUCGGGGCGG | 17 | 12153 |
| HSV1-UL30-3214 | + | CUCGUCCCCUGGGGCGG | 17 | 12154 |
| HSV1-UL30-3215 | + | GUGUCUGCUCAGUUCGG | 17 | 12155 |
| HSV1-UL30-3216 | + | GAGGACGUGCUCCAGGG | 17 | 12156 |
| HSV1-UL30-2082 | - | AUCAAGAAGUACGAGGG | 17 | 5115 |
| HSV1-UL30-3217 | - | AACCUGGCCAUCGAGGG | 17 | 12157 |
| HSV1-UL30-908 | - | GACGAACGCGAGGAGGG | 17 | 3846 |
| HSV1-UL30-3218 | + | GUCUAGCUCGCGGAGGG | 17 | 12158 |
| HSV1-UL30-3219 | + | GUCCAGGAUGAACCGGG | 17 | 12159 |
| HSV1-UL30-3220 | - | UCCGGAAGGCCACCGGG | 17 | 12160 |
| HSV1-UL30-3221 | - | CCUGCCGACCCCCCGGG | 17 | 12161 |
| HSV1-UL30-3222 | - | UCCGGCCGCAGCCCGGG | 17 | 12162 |
| HSV1-UL30-3223 | + | UGGACGCGCCUCCCGGG | 17 | 12163 |
| HSV1-UL30-2170 | - | GAUUUAGGGGCGCCGGG | 17 | 5202 |
| HSV1-UL30-3224 | + | CGGCCACGUCGUCCGGG | 17 | 12164 |
| HSV1-UL30-1477 | + | GAGGGCAGGGCCGCGGG | 17 | 4415 |
| HSV1-UL30-2104 | - | AUCGAAUGCAAGGCGGG | 17 | 5137 |
| HSV1-UL30-2171 | - | AUUUAGGGGCGCCGGGG | 17 | 5203 |
| HSV1-UL30-3225 | + | GGGGCGCUUGGCCGGGG | 17 | 12165 |
| HSV1-UL30-1478 | + | AGGGCAGGGCCGCGGGG | 17 | 4416 |
| HSV1-UL30-2105 | - | UCGAAUGCAAGGCGGGG | 17 | 5138 |
| HSV1-UL30-3226 | + | UCAGGUGGGAUUCGGGG | 17 | 12166 |
| HSV1-UL30-3227 | - | GACCGCAGGGUUCGGGG | 17 | 12167 |
| HSV1-UL30-903 | - | GCCAGAGGAGGAGGGGG | 17 | 3841 |
| HSV1-UL30-1479 | + | GGGCAGGGCCGCGGGGG | 17 | 4417 |
| HSV1-UL30-2106 | - | CGAAUGCAAGGCGGGGG | 17 | 5139 |
| HSV1-UL30-3228 | - | UAGGGGCGCCGGGGGGG | 17 | 12168 |
| HSV1-UL30-1480 | + | CAGGGCCGCGGGGGGGG | 17 | 4418 |
| HSV1-UL30-3229 | - | AUGCAAGGCGGGGGGGG | 17 | 12169 |
| HSV1-UL30-3230 | - | CUCCCACCUGUUGGGGG | 17 | 12170 |
| HSV1-UL30-3231 | + | GGGCUCGUCCCCUGGGG | 17 | 12171 |
| HSV1-UL30-3232 | - | UGGAGAUCGAGGUGGGG | 17 | 12172 |
| HSV1-UL30-3233 | + | CACUCGAGUUUGAUGGG | 17 | 12173 |
| HSV1-UL30-3234 | - | CGUCCACGCGCGCUGGG | 17 | 12174 |
| HSV1-UL30-3235 | - | CUGGAGAUCGAGGUGGG | 17 | 12175 |
| HSV1-UL30-3236 | + | ACACACGACCUUGAUGG | 17 | 12176 |
| HSV1-UL30-3237 | + | GCACUCGAGUUUGAUGG | 17 | 12177 |
| HSV1-UL30-3238 | - | CGCAGUGGCGCACCUGG | 17 | 12178 |
| HSV1-UL30-3239 | + | GUCGACCAGGGCCCUGG | 17 | 12179 |
| HSV1-UL30-3240 | - | CCACCUGAACGAGCUGG | 17 | 12180 |
| HSV1-UL30-3241 | - | ACAGGAUUCCCUGCUGG | 17 | 12181 |
| HSV1-UL30-3242 | - | UCACGUGAACCCCGUGG | 17 | 12182 |
| HSV1-UL30-2231 | - | CCUGGAGAUCGAGGUGG | 17 | 5247 |
| HSV1-UL30-2074 | - | CUUCGAGGCGGAGGUGG | 17 | 5107 |
| HSV1-UL30-3243 | + | GUGCUCCAGGGCGGUGG | 17 | 12183 |
| HSV1-UL30-3244 | + | CAGGAUGAACCGGGUGG | 17 | 12184 |
| HSV1-UL30-3245 | - | GGCCGUCGCGCGCUUGG | 17 | 12185 |
| HSV1-UL30-3246 | - | CUUCUCCCACCUGUUGG | 17 | 12186 |
| HSV1-UL30-3247 | + | CGCCCCGGCCGUUCAUG | 17 | 12187 |
| HSV1-UL30-3248 | + | GCCGAGAGCUCCAGAUG | 17 | 12188 |
| HSV1-UL30-3249 | + | ACAGCGCGCGCGAGAUG | 17 | 12189 |
| HSV1-UL30-2669 | + | CGCACUCGAGUUUGAUG | 17 | 5668 |
| HSV1-UL30-2604 | + | GUGGGCAAUGGCGUAUG | 17 | 5608 |
| HSV1-UL30-3250 | + | CACCGCCCCGAACCCUG | 17 | 12190 |
| HSV1-UL30-3251 | + | GGCGGGCUCGUCCCCUG | 17 | 12191 |
| HSV1-UL30-3252 | - | UCAGCACGCUCUCCCUG | 17 | 12192 |
| HSV1-UL30-2236 | - | UCCUCAGCAUCCUCCUG | 17 | 5252 |
| HSV1-UL30-3253 | + | CUCGUCCUCCCGGGCUG | 17 | 12193 |
| HSV1-UL30-2710 | + | CCCGCAGGAGGAUGCUG | 17 | 5708 |
| HSV1-UL30-3254 | + | GGGCCUGGAUGAUGCUG | 17 | 12194 |
| HSV1-UL30-2701 | + | UCCUCGGGGCUGCUCUG | 17 | 5699 |
| HSV1-UL30-3255 | - | GGUUUAUUCCCGAAGUG | 17 | 12195 |
| HSV1-UL30-3256 | + | CGUGAAACCCGGAAGUG | 17 | 12196 |
| HSV1-UL30-3257 | - | CCGCCGGCAGGCACGUG | 17 | 12197 |
| HSV1-UL30-3258 | + | UCAGCCCGGCGGCCGUG | 17 | 12198 |
| HSV1-UL30-2134 | - | GGGGCGUGUUUCGCGUG | 17 | 5167 |
| HSV1-UL30-3259 | + | CUCGCAGAGAUCUCGUG | 17 | 12199 |
| HSV1-UL30-2230 | - | ACCUGGAGAUCGAGGUG | 17 | 5246 |
| HSV1-UL30-3260 | + | CCAGGGCCCUGGAGGUG | 17 | 12200 |
| HSV1-UL30-3261 | + | UGCUCAGUUCGGCGGUG | 17 | 12201 |
| HSV1-UL30-3262 | - | CAAGAAGUACGAGGGUG | 17 | 12202 |
| HSV1-UL30-3263 | + | GGCCGUCGUAGAUGGUG | 17 | 12203 |
| HSV1-UL30-2607 | + | CGGACACCAGCAGCUUG | 17 | 5611 |
| HSV1-UL30-3264 | + | UGCUGAAGCACAGGUUG | 17 | 12204 |
| HSV1-UL30-3265 | - | ACUUCUCCCACCUGUUG | 17 | 12205 |
| HSV1-UL30-3266 | + | GGCCGCCUCCGGGAAAU | 17 | 12206 |
| HSV1-UL30-3267 | + | GAUGAUGCGCAUGGAAU | 17 | 12207 |
| HSV1-UL30-3268 | - | UUUC GC GUGUGGGACAU | 17 | 12208 |
| HSV1-UL30-3269 | - | GCCAAGAAAAAGUACAU | 17 | 12209 |
| HSV1-UL30-3270 | - | GCGACGGUGACGACCAU | 17 | 12210 |
| HSV1-UL30-3271 | - | UCCUCGUAGACGCCCAU | 17 | 12211 |
| HSV1-UL30-2800 | + | GAUGUCCCGAAGGCCAU | 17 | 5797 |
| HSV1-UL30-3272 | + | GGCCGAGAGCUCCAGAU | 17 | 12212 |
| HSV1-UL30-3273 | + | GUCGGUGAUGCGCCGAU | 17 | 12213 |
| HSV1-UL30-3274 | - | GCGCAACGCGGGGUGAU | 17 | 12214 |
| HSV1-UL30-2668 | + | UCGCACUCGAGUUUGAU | 17 | 5667 |
| HSV1-UL30-2766 | + | GUACCCGGUCACGAACU | 17 | 5764 |
| HSV1-UL30-2854 | + | AAACCGCGCGUGGAACU | 17 | 11794 |
| HSV1-UL30-3275 | - | CAAGGUCGUGUGUAACU | 17 | 12215 |
| HSV1-UL30-3276 | + | GCCCCUAAAUCGCCCCU | 17 | 12216 |
| HSV1-UL30-3277 | + | GGGCGGGCUCGUCCCCU | 17 | 12217 |
| HSV1-UL30-3278 | + | GAACGCCUGCAGUCCCU | 17 | 12218 |
| HSV1-UL30-3279 | + | GGCGUAUGCGGGAUCCU | 17 | 12219 |
| HSV1-UL30-2697 | + | CAGGAGCACGGCCUCCU | 17 | 5695 |
| HSV1-UL30-3280 | - | ACCGUCAUCACGCUCCU | 17 | 12220 |
| HSV1-UL30-3281 | + | GGGAUCCUCGGCCAGCU | 17 | 12221 |
| HSV1-UL30-3282 | + | GGCGAUCAGCAGCAGCU | 17 | 12222 |
| HSV1-UL30-3283 | + | GUAAAUGUCCGUCAGCU | 17 | 12223 |
| HSV1-UL30-3284 | + | GGCUGCGGCCGGACGCU | 17 | 12224 |
| HSV1-UL30-3285 | - | GUACGUCCACGCGCGCU | 17 | 12225 |
| HSV1-UL30-3286 | - | CUCGGCCGUCGCGCGCU | 17 | 12226 |
| HSV1-UL30-3287 | + | CUCCUCCGCGGGGCGCU | 17 | 12227 |
| HSV1-UL30-3288 | + | CGUCUCCCGGGGGCGCU | 17 | 12228 |
| HSV1-UL30-3289 | - | GUCCUCCUGUUUUCGCU | 17 | 12229 |
| HSV1-UL30-3290 | + | CAUCGGGGCCGCCGGCU | 17 | 12230 |
| HSV1-UL30-3291 | + | CAGCAGCUUGCGGGGCU | 17 | 12231 |
| HSV1-UL30-3292 | + | UGGGCCUGGAUGAUGCU | 17 | 12232 |
| HSV1-UL30-3293 | + | CAGACUCUCGGUGAUCU | 17 | 12233 |
| HSV1-UL30-3294 | + | CCUUUUUAACAGACUCU | 17 | 12234 |
| HSV1-UL30-3295 | - | GCCCCAUCUGGAGCUCU | 17 | 12235 |
| HSV1-UL30-2700 | + | CUCCUCGGGGCUGCUCU | 17 | 5698 |
| HSV1-UL30-3296 | + | CUCUACCUCGCGGGUCU | 17 | 12236 |
| HSV1-UL30-3297 | + | GCCGAUGUACUUUUUCU | 17 | 12237 |
| HSV1-UL30-3298 | + | ACGUGAAACCCGGAAGU | 17 | 12238 |
| HSV1-UL30-3299 | + | GGGCCCGGCGGCGUAGU | 17 | 12239 |
| HSV1-UL30-3300 | + | CUCGUAGUAGUACACGU | 17 | 12240 |
| HSV1-UL30-3301 | - | ACCGCCGGCAGGCACGU | 17 | 12241 |
| HSV1-UL30-3302 | + | GUGAUGACGGUCCCCGU | 17 | 12242 |
| HSV1-UL30-3303 | + | GUUCAGGGCGACGCCGU | 17 | 12243 |
| HSV1-UL30-3304 | - | GCCGGGCUGACGGCCGU | 17 | 12244 |
| HSV1-UL30-3305 | + | UCCAGAACCACGGGCGU | 17 | 12245 |
| HSV1-UL30-3306 | + | CAGGUGCGCCACUGCGU | 17 | 12246 |
| HSV1-UL30-3307 | + | GCUCGCAGAGAUCUCGU | 17 | 12247 |
| HSV1-UL30-2599 | + | CGCCGCCCCCAACAGGU | 17 | 5603 |
| HSV1-UL30-3308 | + | GUAAUACACCGUCAGGU | 17 | 12248 |
| HSV1-UL30-3309 | + | CGCCAGCUCGUUCAGGU | 17 | 12249 |
| HSV1-UL30-2229 | - | UACCUGGAGAUCGAGGU | 17 | 5245 |
| HSV1-UL30-3310 | + | UGAGCUUGUAUGCCGGU | 17 | 12250 |
| HSV1-UL30-3311 | - | GCAGGGUUCGGGGCGGU | 17 | 12251 |
| HSV1-UL30-3312 | - | UCAAGAAGUACGAGGGU | 17 | 12252 |
| HSV1-UL30-3313 | + | GAUGUCCCUCUCCGGGU | 17 | 12253 |
| HSV1-UL30-3314 | + | CGCGCCUCCCGGGGGGU | 17 | 12254 |
| HSV1-UL30-3315 | - | CAGGAUUCCCUGCUGGU | 17 | 12255 |
| HSV1-UL30-3316 | + | AAUAAAGCCCUUCUGGU | 17 | 12256 |
| HSV1-UL30-3317 | + | CCCGGGGGCGCGCAUGU | 17 | 12257 |
| HSV1-UL30-2315 | - | UUACUUCUCCCACCUGU | 17 | 5328 |
| HSV1-UL30-3318 | - | CGAAUUCGAGAUGCUGU | 17 | 12258 |
| HSV1-UL30-3319 | - | GGGC GUGUUUC GC GUGU | 17 | 12259 |
| HSV1-UL30-3320 | + | GUGGGGCGCGGCAUUGU | 17 | 12260 |
| HSV1-UL30-3321 | + | GUGGGCCAGGCGCUUGU | 17 | 12261 |
| HSV1-UL30-3322 | + | CGAGAGCUUGAUCUUGU | 17 | 12262 |
| HSV1-UL30-3323 | + | GCUGAAGCACAGGUUGU | 17 | 12263 |
| HSV1-UL30-2777 | + | CUCGUUCAGGUGGGAUU | 17 | 5775 |
| HSV1-UL30-2593 | + | CGGGGGGUGCCACACUU | 17 | 5598 |
| HSV1-UL30-3324 | - | CCCGGGUUCGUCACCUU | 17 | 12264 |
| HSV1-UL30-2089 | - | GCGGCCCCGAUGGCCUU | 17 | 5122 |
| HSV1-UL30-3325 | + | GCUGCGGCCGGACGCUU | 17 | 12265 |
| HSV1-UL30-3326 | + | ACAGCACACGCCCGCUU | 17 | 12266 |
| HSV1-UL30-3327 | + | GUCCUUCAGGACGGCUU | 17 | 12267 |
| HSV1-UL30-3328 | + | CGGGUGUCUGCUCAGUU | 17 | 12268 |
| HSV1-UL30-2283 | - | GAGGGACUGCAGGCGUU | 17 | 5298 |
| HSV1-UL30-2327 | - | CUCCGGACCGCAGGGUU | 17 | 5340 |
| HSV1-UL30-2316 | - | UACUUCUCCCACCUGUU | 17 | 5329 |
| HSV1-UL30-2318 | - | ACAUUCAAGGCCCUGUU | 17 | 5331 |
| HSV1-UL30-2164 | - | ACACCCAGGGGCGAUUU | 17 | 5196 |
| HSV1-UL30-2319 | - | CAUUCAAGGCCCUGUUU | 17 | 5332 |
| HSV1-UL30-3329 | - | UCACCGAGAGUCUGUUAAAA | 20 | 12269 |
| HSV1-UL30-3330 | + | GGGGCCGUACUGUUUCACAA | 20 | 12270 |
| HSV1-UL30-3331 | + | CAGGGCGACGCCGUGGGCAA | 20 | 12271 |
| HSV1-UL30-3332 | - | GGGGGUAAGAUGCUCAUCAA | 20 | 12272 |
| HSV1-UL30-3333 | + | GUCAGCUUGGCCAGCAAGAA | 20 | 12273 |
| HSV1-UL30-3334 | - | CUGCGCCUGGCCGACCAGAA | 20 | 12274 |
| HSV1-UL30-3335 | + | CGGGUGCCCGGCCACCGGAA | 20 | 12275 |
| HSV1-UL30-3336 | - | GACGGGUACGGCCGCAUGAA | 20 | 12276 |
| HSV1-UL30-3337 | + | CCGCGUGCCGUAAACGUGAA | 20 | 12277 |
| HSV1-UL30-3338 | - | CGCAGCAAGAUAAAGGUGAA | 20 | 12278 |
| HSV1-UL30-3339 | - | CCAGAAGCGCAGCAAGAUAA | 20 | 12279 |
| HSV1-UL30-3340 | + | CUUGGCGUUAUUCCCAAACA | 20 | 12280 |
| HSV1-UL30-1780 | - | GCAGUACUUUUACAUGAACA | 20 | 4830 |
| HSV1-UL30-3341 | - | CCACGGCGUCGCCCUGAACA | 20 | 12281 |
| HSV1-UL30-3342 | + | CGGGGCCGUACUGUUUCACA | 20 | 12282 |
| HSV1-UL30-3343 | - | GC GCAUCAUCUAC GGGGACA | 20 | 12283 |
| HSV1-UL30-3344 | - | CAAGCUGACGGACAUUUACA | 20 | 12284 |
| HSV1-UL30-3345 | + | GCUGUCGAAUUCCAGAACCA | 20 | 12285 |
| HSV1-UL30-2476 | + | GGCAAAGUCGAACACCACCA | 20 | 5482 |
| HSV1-UL30-3346 | + | GUAAAACAGCAGGUCGACCA | 20 | 12286 |
| HSV1-UL30-1932 | - | GGCAGGCACGUGGGGUACCA | 20 | 4965 |
| HSV1-UL30-3347 | - | UUUAUUCUGCCGGACACCCA | 20 | 12287 |
| HSV1-UL30-2019 | - | AGCUAGACGCCGCCGCCCCA | 20 | 5052 |
| HSV1-UL30-3348 | + | GCUCGCCAUCUUGUCGCCCA | 20 | 12288 |
| HSV1-UL30-3349 | - | CCCGCAUACGCCAUUGCCCA | 20 | 12289 |
| HSV1-UL30-3350 | - | CCUGAACGAGCUGGCGGCCA | 20 | 12290 |
| HSV1-UL30-3351 | + | CUUCAGGACGGCUUCGGCCA | 20 | 12291 |
| HSV1-UL30-3352 | - | CGUGGGGUACCAGGGGGCCA | 20 | 12292 |
| HSV1-UL30-3353 | - | GUUUAUCAACCGCACCUCCA | 20 | 12293 |
| HSV1-UL30-3354 | + | AAACAGGAGGACGUGCUCCA | 20 | 12294 |
| HSV1-UL30-2510 | + | CAUGCGGCCGUACCCGUCCA | 20 | 5516 |
| HSV1-UL30-2496 | + | AAACAGCUGGCCCACCAGCA | 20 | 5502 |
| HSV1-UL30-3355 | - | GGGUUCACGGGAGUGCAGCA | 20 | 12295 |
| HSV1-UL30-3356 | + | CUGCUGCUUGUCCAGGAGCA | 20 | 12296 |
| HSV1-UL30-3357 | - | CCGCGCGGCUCCGGACCGCA | 20 | 12297 |
| HSV1-UL30-3358 | - | ACGAGAUCUCUGCGAGCGCA | 20 | 12298 |
| HSV1-UL30-3359 | + | CGCGCCCGGGGACUCGCGCA | 20 | 12299 |
| HSV1-UL30-2427 | + | GUCCCCGUAGAUGAUGCGCA | 20 | 5433 |
| HSV1-UL30-3360 | + | CGAGCGGAUCUGCUUUCGCA | 20 | 12300 |
| HSV1-UL30-3361 | + | GUCGCGGCAACGUGCAGGCA | 20 | 12301 |
| HSV1-UL30-3362 | - | CAAGAUAAAGGUGAACGGCA | 20 | 12302 |
| HSV1-UL30-3363 | + | GCGCUUGGCCGGGGAGGGCA | 20 | 12303 |
| HSV1-UL30-3364 | - | GGCGCACCUGGAGGCGGGCA | 20 | 12304 |
| HSV1-UL30-3365 | + | CGCCUGCAGUCCCUCGGGCA | 20 | 12305 |
| HSV1-UL30-3366 | - | GUGCUUCGAUAUCGAAUGCA | 20 | 12306 |
| HSV1-UL30-3367 | - | CAAGCAGCAGGCCGCCAUCA | 20 | 12307 |
| HSV1-UL30-3368 | - | CGCGCAGGUCCCGUCCAUCA | 20 | 12308 |
| HSV1-UL30-3369 | - | CGGGGGUAAGAUGCUCAUCA | 20 | 12309 |
| HSV1-UL30-2467 | + | CGCCACUGCGUCGGCCCUCA | 20 | 5473 |
| HSV1-UL30-3370 | - | UGUGCUGUGCCGCGGCCUCA | 20 | 12310 |
| HSV1-UL30-3371 | - | GACGGUGUAUUACAAGCUCA | 20 | 12311 |
| HSV1-UL30-3372 | - | GCGACGGCUGUUCUUCGUCA | 20 | 12312 |
| HSV1-UL30-3373 | + | GAAACCCGGAAGUGGGGUCA | 20 | 12313 |
| HSV1-UL30-3374 | - | GGCGGCGUGCGUGACAUUCA | 20 | 12314 |
| HSV1-UL30-1964 | - | UAACUCGGUGUACGGGUUCA | 20 | 4997 |
| HSV1-UL30-3375 | + | GAAGUAAUAGUCCGUGUUCA | 20 | 12315 |
| HSV1-UL30-1205 | + | GCCGCGGCACAGCACAAAGA | 20 | 4143 |
| HSV1-UL30-3376 | - | GACGGCCGUGGGCGACAAGA | 20 | 12316 |
| HSV1-UL30-3377 | + | CGUCAGCUUGGCCAGCAAGA | 20 | 12317 |
| HSV1-UL30-3378 | - | CGUCCUGAAGGACAAGAAGA | 20 | 12318 |
| HSV1-UL30-3379 | - | GCUGCGCCUGGCCGACCAGA | 20 | 12319 |
| HSV1-UL30-2493 | + | CGACGGCCGAGAGCUCCAGA | 20 | 5499 |
| HSV1-UL30-3380 | - | GACCCGCGAGGUAGAGGAGA | 20 | 12320 |
| HSV1-UL30-3381 | - | GCGCAUCACCGACCCGGAGA | 20 | 12321 |
| HSV1-UL30-3382 | + | CGCGGCGGCCGCUCCGGAGA | 20 | 12322 |
| HSV1-UL30-3383 | + | UGUUCCGGCCCGGUUUGAGA | 20 | 12323 |
| HSV1-UL30-3384 | + | GAUCUGCUGGCCGUCGUAGA | 20 | 12324 |
| HSV1-UL30-3385 | - | AUCACCCGCACCAUCUACGA | 20 | 12325 |
| HSV1-UL30-3386 | - | CCGGCCAUCAAGAAGUACGA | 20 | 12326 |
| HSV1-UL30-1772 | - | UAUGGACGCCAUCACACCGA | 20 | 4822 |
| HSV1-UL30-467 | - | AGCCCAGCCGGCGGCCCCGA | 20 | 3405 |
| HSV1-UL30-3387 | - | CUGGCGCGACCCCUGCCCGA | 20 | 12327 |
| HSV1-UL30-3388 | + | GACGUCGCUGGAUGUCCCGA | 20 | 12328 |
| HSV1-UL30-3389 | + | UUUGAGACGGUACCAGCCGA | 20 | 12329 |
| HSV1-UL30-3390 | + | CCGGGUCGGUGAUGCGCCGA | 20 | 12330 |
| HSV1-UL30-3391 | + | GAGCAGCAUCUCGCGGCCGA | 20 | 12331 |
| HSV1-UL30-3392 | - | GUGCCUGCACGUUGCCGCGA | 20 | 12332 |
| HSV1-UL30-3393 | + | AAUACCCGCCAAGCGCGCGA | 20 | 12333 |
| HSV1-UL30-3394 | + | CCCGGGGGGUCGGCAGGCGA | 20 | 12334 |
| HSV1-UL30-3395 | - | AGAUCGAGGUGGGGGGGCGA | 20 | 12335 |
| HSV1-UL30-1813 | - | GCGGACAACCUGGCCAUCGA | 20 | 4863 |
| HSV1-UL30-3396 | + | GUCGCUCAUGCCCCCCUCGA | 20 | 12336 |
| HSV1-UL30-3397 | + | AUCGGCCAGGAGCUGUUCGA | 20 | 12337 |
| HSV1-UL30-3398 | + | CUUCUUCUUGUCCUUCAGGA | 20 | 12338 |
| HSV1-UL30-3399 | + | CCGAUGGGCGUCUACGAGGA | 20 | 12339 |
| HSV1-UL30-581 | - | GACGAGGACGAACGCGAGGA | 20 | 3519 |
| HSV1-UL30-574 | - | GAGGAGCGGCCAGAGGAGGA | 20 | 3512 |
| HSV1-UL30-590 | - | GGGGGCGAGCGGGAGCCGGA | 20 | 3528 |
| HSV1-UL30-3400 | - | CUCCUGGGCCUGACUCCGGA | 20 | 12340 |
| HSV1-UL30-3401 | + | GGCGGCGUCUAGCUCGCGGA | 20 | 12341 |
| HSV1-UL30-3402 | + | CGGGGGCGCUUGGCCGGGGA | 20 | 12342 |
| HSV1-UL30-2391 | + | GGGAUCCGGUCCUUGAUGGA | 20 | 5398 |
| HSV1-UL30-3403 | - | AUUUACAAGGUCCCCCUGGA | 20 | 12343 |
| HSV1-UL30-3404 | + | CUGUUUCACAAGGGUCAUGA | 20 | 12344 |
| HSV1-UL30-3405 | + | CAGGCCCAGGAGCGUGAUGA | 20 | 12345 |
| HSV1-UL30-3406 | - | CAAGCGCCUGGCCCACCUGA | 20 | 12346 |
| HSV1-UL30-3407 | - | CUUCAGCACGCUCUCCCUGA | 20 | 12347 |
| HSV1-UL30-3408 | - | CGUGGCCGAAGCCGUCCUGA | 20 | 12348 |
| HSV1-UL30-3409 | - | CUUCUUGCUGGCCAAGCUGA | 20 | 12349 |
| HSV1-UL30-3410 | - | CCUCACGGCCGCCGGGCUGA | 20 | 12350 |
| HSV1-UL30-3411 | + | GACGGUCCCCGUCGGUGUGA | 20 | 12351 |
| HSV1-UL30-3412 | + | CGAGUUACACACGACCUUGA | 20 | 12352 |
| HSV1-UL30-3413 | + | GUACGGGAUCCGGUCCUUGA | 20 | 12353 |
| HSV1-UL30-3414 | + | CCCACCCUCGUACUUCUUGA | 20 | 12354 |
| HSV1-UL30-2418 | + | CUUUUCGCACUCGAGUUUGA | 20 | 5425 |
| HSV1-UL30-3415 | + | UAGAUGAUGCGCAUGGAAUA | 20 | 12355 |
| HSV1-UL30-3416 | - | GGCGGUGGGUGCCGGCGCUA | 20 | 12356 |
| HSV1-UL30-1975 | - | UAUUC CAUGC GCAUCAUCUA | 20 | 5008 |
| HSV1-UL30-1985 | - | AAGUACAUCGGCGUCAUCUA | 20 | 5018 |
| HSV1-UL30-3417 | - | AUGACCCUUGUGAAACAGUA | 20 | 12357 |
| HSV1-UL30-2388 | + | GUCUGGGCCACGAUCACGUA | 20 | 5395 |
| HSV1-UL30-3418 | - | AAGGUCCCCCUGGACGGGUA | 20 | 12358 |
| HSV1-UL30-1858 | - | AUGGUGAACAUCGACAUGUA | 20 | 4908 |
| HSV1-UL30-3419 | - | CGACGUCGAGUUUAACUGUA | 20 | 12359 |
| HSV1-UL30-3420 | - | GUC GUGUGUAACUC GGUGUA | 20 | 12360 |
| HSV1-UL30-3421 | - | GGACACCCAGGGGCGAUUUA | 20 | 12361 |
| HSV1-UL30-3422 | - | CGGGUGGCCGUUCACGUUUA | 20 | 12362 |
| HSV1-UL30-2855 | - | CCAGUUCCACGCGCGGUUUA | 20 | 11795 |
| HSV1-UL30-1345 | + | CGCAGGAACCGAGCGAAAAC | 20 | 4283 |
| HSV1-UL30-3423 | + | UCUUGGCGUUAUUCCCAAAC | 20 | 12363 |
| HSV1-UL30-3424 | - | CGGCUGGUACCGUCUCAAAC | 20 | 12364 |
| HSV1-UL30-3425 | + | UCACGCACGCCGCCCCCAAC | 20 | 12365 |
| HSV1-UL30-2856 | + | CCAUAAACCGCGCGUGGAAC | 20 | 11796 |
| HSV1-UL30-3426 | + | CGGGGUUGUCCAGGAUGAAC | 20 | 12366 |
| HSV1-UL30-3427 | + | CUGUCGAAUUCCAGAACCAC | 20 | 12367 |
| HSV1-UL30-3428 | + | GCAAAGUCGAACACCACCAC | 20 | 12368 |
| HSV1-UL30-3429 | - | GCCUGACUCCGGAAGGCCAC | 20 | 12369 |
| HSV1-UL30-2545 | + | UCCUCCGGGUGCCCGGCCAC | 20 | 5550 |
| HSV1-UL30-3430 | + | GGGAGAGCGUGCUGAAGCAC | 20 | 12370 |
| HSV1-UL30-3431 | + | CCGGGCAGAAGUUGUCGCAC | 20 | 12371 |
| HSV1-UL30-1965 | - | AACUCGGUGUACGGGUUCAC | 20 | 4998 |
| HSV1-UL30-3432 | + | GGACAGGUCGUAGAGCAGAC | 20 | 12372 |
| HSV1-UL30-1773 | - | AUGGACGCCAUCACACCGAC | 20 | 4823 |
| HSV1-UL30-3433 | - | UGAACAAGGAGGAGGUCGAC | 20 | 12373 |
| HSV1-UL30-3434 | - | ACAACAUCAUCAACUUCGAC | 20 | 12374 |
| HSV1-UL30-3435 | - | AGGUCCCGUCCAUCAAGGAC | 20 | 12375 |
| HSV1-UL30-3436 | - | UCAGCAUCCUCCUGCGGGAC | 20 | 12376 |
| HSV1-UL30-3437 | + | GGAUCCGGUCCUUGAUGGAC | 20 | 12377 |
| HSV1-UL30-3438 | - | UUUACAAGGUCCCCCUGGAC | 20 | 12378 |
| HSV1-UL30-3439 | + | GCUCCAGGGCGGUGGUGGAC | 20 | 12379 |
| HSV1-UL30-3440 | - | UACGGCCCCGAGUUCGUGAC | 20 | 12380 |
| HSV1-UL30-3441 | - | GAUCGGCGAGUACUGCAUAC | 20 | 12381 |
| HSV1-UL30-3442 | - | GGGGGGCAUGAGCGACCUAC | 20 | 12382 |
| HSV1-UL30-1976 | - | AUUC CAUGC GCAUCAUCUAC | 20 | 5009 |
| HSV1-UL30-1986 | - | AGUACAUCGGCGUCAUCUAC | 20 | 5019 |
| HSV1-UL30-3443 | + | UCUGGGCCACGAUCACGUAC | 20 | 12383 |
| HSV1-UL30-3444 | + | CCUGGAUGAUGCUGGGGUAC | 20 | 12384 |
| HSV1-UL30-3445 | - | UGGUGAACAUCGACAUGUAC | 20 | 12385 |
| HSV1-UL30-3446 | - | UC GUGUGUAACUC GGUGUAC | 20 | 12386 |
| HSV1-UL30-3447 | - | GGCUGGUACCGUCUCAAACC | 20 | 12387 |
| HSV1-UL30-2479 | + | CACGGGGUUCACGUGAAACC | 20 | 5485 |
| HSV1-UL30-3448 | - | UAACUGUACGGCGGACAACC | 20 | 12388 |
| HSV1-UL30-2557 | + | CCAGCCGAAGGUGACGAACC | 20 | 5562 |
| HSV1-UL30-3449 | + | GGGGUUGUCCAGGAUGAACC | 20 | 12389 |
| HSV1-UL30-3450 | - | GUGGGGUCGACGCCACCACC | 20 | 12390 |
| HSV1-UL30-3451 | - | CCUGACUCCGGAAGGCCACC | 20 | 12391 |
| HSV1-UL30-3452 | + | CGUAAACGUGAACGGCCACC | 20 | 12392 |
| HSV1-UL30-1939 | - | GGCCGACGCAGUGGCGCACC | 20 | 4972 |
| HSV1-UL30-1829 | - | CUUUCCGGUGGCCGGGCACC | 20 | 4879 |
| HSV1-UL30-2003 | - | CCAUCGGCGCAUCACCGACC | 20 | 5036 |
| HSV1-UL30-3453 | + | CGUAAAACAGCAGGUCGACC | 20 | 12393 |
| HSV1-UL30-495 | - | GGCCGGGCACCCGGAGGACC | 20 | 3433 |
| HSV1-UL30-3454 | + | GACAGGAUAUCUGGAUGACC | 20 | 12394 |
| HSV1-UL30-3455 | - | ACGGCCCCGAGUUCGUGACC | 20 | 12395 |
| HSV1-UL30-1943 | - | GGAGGCGGGCAAGGACUACC | 20 | 4976 |
| HSV1-UL30-1931 | - | CGGCAGGCACGUGGGGUACC | 20 | 4964 |
| HSV1-UL30-3456 | + | GAAGUUGAUGAUGUUGUACC | 20 | 12396 |
| HSV1-UL30-3457 | - | CGGUUCAUCCUGGACAACCC | 20 | 12397 |
| HSV1-UL30-3458 | + | CAGCCGAAGGUGACGAACCC | 20 | 12398 |
| HSV1-UL30-1880 | - | CUUUAUUCUGCCGGACACCC | 20 | 4929 |
| HSV1-UL30-3459 | + | GGAAGUGGGGUCAAGGACCC | 20 | 12399 |
| HSV1-UL30-3460 | - | GGUUCAUCCUGGACAACCCC | 20 | 12400 |
| HSV1-UL30-3461 | - | UCCCGAAGUGUGGCACCCCC | 20 | 12401 |
| HSV1-UL30-2025 | - | GCCGUCGCCUGCCGACCCCC | 20 | 5058 |
| HSV1-UL30-2026 | - | CCGUCGCCUGCCGACCCCCC | 20 | 5059 |
| HSV1-UL30-3462 | + | ACGCUUGGGCGCCUCCCCCC | 20 | 12402 |
| HSV1-UL30-2023 | - | CUCCCCGGCCAAGCGCCCCC | 20 | 5056 |
| HSV1-UL30-3463 | - | CGGCCGACAUGCGCGCCCCC | 20 | 12403 |
| HSV1-UL30-3464 | + | GGUCAAGGACCCUGGCCCCC | 20 | 12404 |
| HSV1-UL30-3465 | - | GGACAUUUACAAGGUCCCCC | 20 | 12405 |
| HSV1-UL30-2018 | - | GAGCUAGACGCCGCCGCCCC | 20 | 5051 |
| HSV1-UL30-2022 | - | CCUCCCCGGCCAAGCGCCCC | 20 | 5055 |
| HSV1-UL30-3466 | - | GCGGCCGACAUGCGCGCCCC | 20 | 12406 |
| HSV1-UL30-3467 | + | CGGCGCCCCUAAAUCGCCCC | 20 | 12407 |
| HSV1-UL30-3468 | + | CACGGGCGUGGGCAGGCCCC | 20 | 12408 |
| HSV1-UL30-3469 | - | GCGGCCCUGCGCGAGUCCCC | 20 | 12409 |
| HSV1-UL30-2381 | + | GGGGGGGCGGGCUCGUCCCC | 20 | 5388 |
| HSV1-UL30-1892 | - | CAAGCGUCCGGCCGCAGCCC | 20 | 4941 |
| HSV1-UL30-1315 | + | CCCACACGCGAAACACGCCC | 20 | 4253 |
| HSV1-UL30-1832 | - | CGACCUGUCCACCACCGCCC | 20 | 4882 |
| HSV1-UL30-2574 | + | UGCCGCGGAACGACGCGCCC | 20 | 5579 |
| HSV1-UL30-2583 | + | GGCCUUCCGGAGUCAGGCCC | 20 | 5588 |
| HSV1-UL30-2413 | + | CAGCAGGUCGACCAGGGCCC | 20 | 5420 |
| HSV1-UL30-3470 | - | CAACCGCACCUCCAGGGCCC | 20 | 12410 |
| HSV1-UL30-2431 | + | AUGCGCAUGGAAUAGGGCCC | 20 | 5437 |
| HSV1-UL30-3471 | - | CCCCGCGGCCCUGCCCUCCC | 20 | 12411 |
| HSV1-UL30-2362 | + | GGGGCUUGGACGCGCCUCCC | 20 | 5374 |
| HSV1-UL30-3472 | + | UGGCCGCUCCUCGUCCUCCC | 20 | 12412 |
| HSV1-UL30-2366 | + | GGCAGGCGACGGCGUCUCCC | 20 | 5378 |
| HSV1-UL30-3473 | - | CGCGGCCCUGCGCGAGUCCC | 20 | 12413 |
| HSV1-UL30-1891 | - | CCAAGCGUCCGGCCGCAGCC | 20 | 4940 |
| HSV1-UL30-3474 | + | GUCGCCCACGGCCGUCAGCC | 20 | 12414 |
| HSV1-UL30-3475 | - | UCCCCGCCUACUACGCCGCC | 20 | 12415 |
| HSV1-UL30-3476 | - | GCCGCGGCCUCACGGCCGCC | 20 | 12416 |
| HSV1-UL30-3477 | - | CGCGUACACCAACAAGCGCC | 20 | 12417 |
| HSV1-UL30-2573 | + | AUGCCGCGGAACGACGCGCC | 20 | 5578 |
| HSV1-UL30-1885 | - | AGGGGCGAUUUAGGGGCGCC | 20 | 4934 |
| HSV1-UL30-3478 | - | CUUUACGUGCCUGCUGCGCC | 20 | 12418 |
| HSV1-UL30-3479 | - | GUACGGCCGCAUGAACGGCC | 20 | 12419 |
| HSV1-UL30-3480 | + | GCUAGCGUGUUGUUCCGGCC | 20 | 12420 |
| HSV1-UL30-1837 | - | ACCUGAACGAGCUGGCGGCC | 20 | 4887 |
| HSV1-UL30-2436 | + | CCGCCUCCGGGAAAUCGGCC | 20 | 5442 |
| HSV1-UL30-3481 | + | UAAAGCCCUUCUGGUCGGCC | 20 | 12421 |
| HSV1-UL30-2430 | + | GAUGCGCAUGGAAUAGGGCC | 20 | 5436 |
| HSV1-UL30-3482 | + | CACCCCGCGUUGCGCGGGCC | 20 | 12422 |
| HSV1-UL30-3483 | - | ACGUGGGGUACCAGGGGGCC | 20 | 12423 |
| HSV1-UL30-3484 | + | AAUACACCGUCAGGUGGGCC | 20 | 12424 |
| HSV1-UL30-3485 | + | UGAAGCACAGGUUGUGGGCC | 20 | 12425 |
| HSV1-UL30-3486 | + | UCAUGCCCCCCUCGAUGGCC | 20 | 12426 |
| HSV1-UL30-3487 | + | CCUCGUACUUCUUGAUGGCC | 20 | 12427 |
| HSV1-UL30-3488 | - | AGCUGGCCUUUCCGGUGGCC | 20 | 12428 |
| HSV1-UL30-2369 | + | UCUCCCGGGGGCGCUUGGCC | 20 | 5381 |
| HSV1-UL30-3489 | - | CCUGUGCGACAACUUCUGCC | 20 | 12429 |
| HSV1-UL30-3490 | + | GACCAGGUCCUCCGGGUGCC | 20 | 12430 |
| HSV1-UL30-3491 | - | CGACCCGGAGAGGGACAUCC | 20 | 12431 |
| HSV1-UL30-3492 | - | CCUGUACCCCAGCAUCAUCC | 20 | 12432 |
| HSV1-UL30-3493 | - | CGCCACCACCCGGUUCAUCC | 20 | 12433 |
| HSV1-UL30-3494 | - | CGUUUAUCAACCGCACCUCC | 20 | 12434 |
| HSV1-UL30-3495 | + | GGUAGUCCUUGCCCGCCUCC | 20 | 12435 |
| HSV1-UL30-2434 | + | CGCGCAUGUCGGCCGCCUCC | 20 | 5440 |
| HSV1-UL30-2361 | + | CGGGGCUUGGACGCGCCUCC | 20 | 5373 |
| HSV1-UL30-3496 | + | CUGGCCGCUCCUCGUCCUCC | 20 | 12436 |
| HSV1-UL30-3497 | + | UCUGGAUGACCAGGUCCUCC | 20 | 12437 |
| HSV1-UL30-3498 | - | GGCGGCCUUCGAACAGCUCC | 20 | 12438 |
| HSV1-UL30-3499 | - | GGGGACCGUCAUCACGCUCC | 20 | 12439 |
| HSV1-UL30-3500 | + | AAAACAGGAGGACGUGCUCC | 20 | 12440 |
| HSV1-UL30-3501 | - | CCCCGAGGAGGCCGUGCUCC | 20 | 12441 |
| HSV1-UL30-3502 | + | GCCCCCCCACCUCGAUCUCC | 20 | 12442 |
| HSV1-UL30-3503 | + | AGUCCUGGAUGUCCCUCUCC | 20 | 12443 |
| HSV1-UL30-2365 | + | CGGCAGGCGACGGCGUCUCC | 20 | 5377 |
| HSV1-UL30-3504 | + | CGGCGGUGAGGACAAAGUCC | 20 | 12444 |
| HSV1-UL30-2509 | + | UCAUGCGGCCGUACCCGUCC | 20 | 5515 |
| HSV1-UL30-2341 | + | GCCGCGCGGCCACGUCGUCC | 20 | 5354 |
| HSV1-UL30-2446 | + | UGGCGGCCUGCUGCUUGUCC | 20 | 5452 |
| HSV1-UL30-3505 | + | UGACGAACCCGGGGUUGUCC | 20 | 12445 |
| HSV1-UL30-3506 | - | GGGUCCUUGACCCCACUUCC | 20 | 12446 |
| HSV1-UL30-1972 | - | ACAGCUCCUGGCCGAUUUCC | 20 | 5005 |
| HSV1-UL30-3507 | - | ACCGCGUCUACGUCCGAAGC | 20 | 12447 |
| HSV1-UL30-3508 | + | GCAAAAACUUAAAAAACAGC | 20 | 12448 |
| HSV1-UL30-3509 | + | CGGUAUCGUCGUAAAACAGC | 20 | 12449 |
| HSV1-UL30-2495 | + | AAAACAGCUGGCCCACCAGC | 20 | 5501 |
| HSV1-UL30-3510 | - | GAACAACACGCUAGCCCAGC | 20 | 12450 |
| HSV1-UL30-3511 | - | CGUGCUCCUGGACAAGCAGC | 20 | 12451 |
| HSV1-UL30-3512 | + | UCUGGUCGGCCAGGCGCAGC | 20 | 12452 |
| HSV1-UL30-3513 | + | UAGACGCGGUAAAACAGAGC | 20 | 12453 |
| HSV1-UL30-3514 | - | CGAAUCCCACCUGAACGAGC | 20 | 12454 |
| HSV1-UL30-488 | - | GGCGGGGGGGGAGGACGAGC | 20 | 3426 |
| HSV1-UL30-3515 | - | CAAGCUGCUGGUGUCCGAGC | 20 | 12455 |
| HSV1-UL30-587 | - | CGAGGAGGGCGGGGGCGAGC | 20 | 3525 |
| HSV1-UL30-588 | - | GGGCGGGGGCGAGCGGGAGC | 20 | 3526 |
| HSV1-UL30-535 | - | CCGCCGGGCCCGCGCAACGC | 20 | 3473 |
| HSV1-UL30-593 | - | GAGGGCGCGCGGGAGACCGC | 20 | 3531 |
| HSV1-UL30-3516 | - | GCCGCGCGGCUCCGGACCGC | 20 | 12456 |
| HSV1-UL30-2457 | + | GCAUGGCGAGCCAGUCCCGC | 20 | 5463 |
| HSV1-UL30-3517 | - | AUCCCCGCCUACUACGCCGC | 20 | 12457 |
| HSV1-UL30-3518 | - | UGCCGCGGCCUCACGGCCGC | 20 | 12458 |
| HSV1-UL30-1125 | + | GCCGGGGAGGGCAGGGCCGC | 20 | 4063 |
| HSV1-UL30-3519 | + | CCGAAGGCCAUCGGGGCCGC | 20 | 12459 |
| HSV1-UL30-3520 | + | CGCGCCAGCCACUCCUCCGC | 20 | 12460 |
| HSV1-UL30-3521 | + | AGUUUCCUCCGCCGUAGCGC | 20 | 12461 |
| HSV1-UL30-3522 | - | GCGAGUACGUCCACGCGCGC | 20 | 12462 |
| HSV1-UL30-3523 | + | GCUUGUUGGUGUACGCGCGC | 20 | 12463 |
| HSV1-UL30-3524 | - | GCUCAUGGCCCGCCGCGCGC | 20 | 12464 |
| HSV1-UL30-592 | - | GCGGGAGCCGGAGGGCGCGC | 20 | 3530 |
| HSV1-UL30-3525 | + | ACGCGCCCGGGGACUCGCGC | 20 | 12465 |
| HSV1-UL30-1884 | - | CAGGGGCGAUUUAGGGGCGC | 20 | 4933 |
| HSV1-UL30-3526 | + | CCGAUCACCCCGCGUUGCGC | 20 | 12466 |
| HSV1-UL30-3527 | + | GCGCGCGUGGACGUACUCGC | 20 | 12467 |
| HSV1-UL30-3528 | + | GACCGUCUCCUCUACCUCGC | 20 | 12468 |
| HSV1-UL30-3529 | + | ACAGUUAAACUCGACGUCGC | 20 | 12469 |
| HSV1-UL30-2532 | + | GUGGGAUUCGGGGAGGUCGC | 20 | 5538 |
| HSV1-UL30-1819 | - | UUCGAUAUCGAAUGCAAGGC | 20 | 4869 |
| HSV1-UL30-3530 | + | GAUGAUGCUGGGGUACAGGC | 20 | 12470 |
| HSV1-UL30-3531 | - | GCAGUGGCGCACCUGGAGGC | 20 | 12471 |
| HSV1-UL30-2500 | + | GGCCCGGCGGCGUAGUAGGC | 20 | 5506 |
| HSV1-UL30-1850 | - | GGUACGGCCGCAUGAACGGC | 20 | 4900 |
| HSV1-UL30-2442 | + | CGGCAACGUGCAGGCACGGC | 20 | 5448 |
| HSV1-UL30-594 | - | GCGCGCGGGAGACCGCCGGC | 20 | 3532 |
| HSV1-UL30-3532 | + | AGGCCAUCGGGGCCGCCGGC | 20 | 12472 |
| HSV1-UL30-3533 | + | GGACGGGACCUGCGCGCGGC | 20 | 12473 |
| HSV1-UL30-3534 | - | AGAGGAGACGGUCGCGCGGC | 20 | 12474 |
| HSV1-UL30-3535 | + | UCGUCCUCCCGGGCUGCGGC | 20 | 12475 |
| HSV1-UL30-3536 | + | GCGCCUCCCGGGGGGUCGGC | 20 | 12476 |
| HSV1-UL30-3537 | - | CCGGGUUCGUCACCUUCGGC | 20 | 12477 |
| HSV1-UL30-583 | - | AGGACGAACGCGAGGAGGGC | 20 | 3521 |
| HSV1-UL30-3538 | + | GGCGCUUGGCCGGGGAGGGC | 20 | 12478 |
| HSV1-UL30-1805 | - | GGUACCGUCUCAAACCGGGC | 20 | 4855 |
| HSV1-UL30-2406 | + | ACGCCUGCAGUCCCUCGGGC | 20 | 5413 |
| HSV1-UL30-1132 | + | GGCAGGGCCGCGGGGGGGGC | 20 | 4070 |
| HSV1-UL30-3539 | + | CCAGAACCACGGGCGUGGGC | 20 | 12479 |
| HSV1-UL30-3540 | + | CCCUCGUACUUCUUGAUGGC | 20 | 12480 |
| HSV1-UL30-3541 | - | GCGCCCCGCGGAGGAGUGGC | 20 | 12481 |
| HSV1-UL30-3542 | - | GAGCUGGCCUUUCCGGUGGC | 20 | 12482 |
| HSV1-UL30-3543 | - | UCGGCCGUCGCGCGCUUGGC | 20 | 12483 |
| HSV1-UL30-2368 | + | GUCUCCCGGGGGCGCUUGGC | 20 | 5380 |
| HSV1-UL30-2473 | + | GUUGUGGGCCUGGAUGAUGC | 20 | 5479 |
| HSV1-UL30-3544 | + | CCGUGGGCAAUGGCGUAUGC | 20 | 12484 |
| HSV1-UL30-3545 | + | AAGCACAUGAGCUUGUAUGC | 20 | 12485 |
| HSV1-UL30-3546 | - | ACCCCUGCCCGAGGGACUGC | 20 | 12486 |
| HSV1-UL30-3547 | - | CUGCAUACAGGAUUCCCUGC | 20 | 12487 |
| HSV1-UL30-3548 | + | CUGGUACCCCACGUGCCUGC | 20 | 12488 |
| HSV1-UL30-3549 | - | CCUCCUCAGCAUCCUCCUGC | 20 | 12489 |
| HSV1-UL30-3550 | - | GUCCAAGCCCCGCAAGCUGC | 20 | 12490 |
| HSV1-UL30-3551 | + | ACGUAAAGACGCGGAUCUGC | 20 | 12491 |
| HSV1-UL30-3552 | - | CCAGAAGGGCUUUAUUCUGC | 20 | 12492 |
| HSV1-UL30-3553 | + | UCACCGUCGCGGCAACGUGC | 20 | 12493 |
| HSV1-UL30-3554 | - | GGGUUCGGGGCGGUGGGUGC | 20 | 12494 |
| HSV1-UL30-3555 | + | CUGGCCGUCGUAGAUGGUGC | 20 | 12495 |
| HSV1-UL30-3556 | + | CUCGGACACCAGCAGCUUGC | 20 | 12496 |
| HSV1-UL30-3557 | - | CUUCGACUGGCCCUUCUUGC | 20 | 12497 |
| HSV1-UL30-1874 | - | UUUUAAGUUUUUGCCCCAUC | 20 | 4923 |
| HSV1-UL30-3558 | + | UGGAUGUCCCGAAGGCCAUC | 20 | 12498 |
| HSV1-UL30-3559 | + | CCACGAUCACGUACGGGAUC | 20 | 12499 |
| HSV1-UL30-3560 | - | GCUCAUCAAGGGCGUGGAUC | 20 | 12500 |
| HSV1-UL30-3561 | + | CGUAGAGCAGACAGGAUAUC | 20 | 12501 |
| HSV1-UL30-3562 | + | UUGUACCCGGUCACGAACUC | 20 | 12502 |
| HSV1-UL30-1776 | - | CACGCUCCUGGGCCUGACUC | 20 | 4826 |
| HSV1-UL30-3563 | + | GCGGUCUCCCGCGCGCCCUC | 20 | 12503 |
| HSV1-UL30-2466 | + | GCGCCACUGCGUCGGCCCUC | 20 | 5472 |
| HSV1-UL30-3564 | + | CCGAACGCCUGCAGUCCCUC | 20 | 12504 |
| HSV1-UL30-2433 | + | GCGCGCAUGUCGGCCGCCUC | 20 | 5439 |
| HSV1-UL30-3565 | + | UCCAGGAGCACGGCCUCCUC | 20 | 12505 |
| HSV1-UL30-3566 | + | UCGUCCUCCCCCUCCUCCUC | 20 | 12506 |
| HSV1-UL30-3567 | + | AUCUGGAUGACCAGGUCCUC | 20 | 12507 |
| HSV1-UL30-3568 | + | CGGGGAUGUCGCGAUAGCUC | 20 | 12508 |
| HSV1-UL30-2410 | + | GGCUAACGCGGCGGCCGCUC | 20 | 5417 |
| HSV1-UL30-3569 | - | ACGACGUGGCCGCGCGGCUC | 20 | 12509 |
| HSV1-UL30-3570 | + | UGCGCUUCUGGAAGUGGCUC | 20 | 12510 |
| HSV1-UL30-2450 | + | CGGCCUCCUCGGGGCUGCUC | 20 | 5456 |
| HSV1-UL30-3571 | + | AAGUCCUGGAUGUCCCUCUC | 20 | 12511 |
| HSV1-UL30-1991 | - | UUUUACGACGAUACCGUCUC | 20 | 5024 |
| HSV1-UL30-3572 | + | CCCGGUGGCCUUCCGGAGUC | 20 | 12512 |
| HSV1-UL30-3573 | + | UGAGCUUGUAAUACACCGUC | 20 | 12513 |
| HSV1-UL30-3574 | - | GGGGGAGGCGCCCAAGCGUC | 20 | 12514 |
| HSV1-UL30-2340 | + | AGCCGCGCGGCCACGUCGUC | 20 | 5353 |
| HSV1-UL30-2338 | + | CCGCCCCGAACCCUGCGGUC | 20 | 5351 |
| HSV1-UL30-3575 | + | UCUCCUCUACCUCGCGGGUC | 20 | 12515 |
| HSV1-UL30-3576 | + | CUAAAUCGCCCCUGGGUGUC | 20 | 12516 |
| HSV1-UL30-2529 | + | AGCUCGUUCAGGUGGGAUUC | 20 | 5535 |
| HSV1-UL30-2345 | + | UCCGGGGGGUGCCACACUUC | 20 | 5358 |
| HSV1-UL30-3577 | - | AGGGUCCUUGACCCCACUUC | 20 | 12517 |
| HSV1-UL30-3578 | + | CCGGCAGAAUAAAGCCCUUC | 20 | 12518 |
| HSV1-UL30-3579 | - | CGGCGGCCCCGAUGGCCUUC | 20 | 12519 |
| HSV1-UL30-2581 | + | ACGGCCACCCGGUGGCCUUC | 20 | 5586 |
| HSV1-UL30-3580 | + | GGUCCUUCUUCUUGUCCUUC | 20 | 12520 |
| HSV1-UL30-2506 | + | CCUUUAUCUUGCUGCGCUUC | 20 | 5512 |
| HSV1-UL30-3581 | - | CCGAGGGACUGCAGGCGUUC | 20 | 12521 |
| HSV1-UL30-3582 | + | CCCUGGCCGCCAGCUCGUUC | 20 | 12522 |
| HSV1-UL30-3583 | - | GGCUCCGGACCGCAGGGUUC | 20 | 12523 |
| HSV1-UL30-1839 | - | CCUGCCCACGCCCGUGGUUC | 20 | 4889 |
| HSV1-UL30-3584 | + | AGAAGUAAUAGUCCGUGUUC | 20 | 12524 |
| HSV1-UL30-3585 | + | GCUGGGCUAGCGUGUUGUUC | 20 | 12525 |
| HSV1-UL30-3586 | - | GGAGGACGAGCUGGCCUUUC | 20 | 12526 |
| HSV1-UL30-3587 | - | UACCGCGUCUACGUCCGAAG | 20 | 12527 |
| HSV1-UL30-3588 | + | CCACCACCUCCGCCUCGAAG | 20 | 12528 |
| HSV1-UL30-2481 | + | GUUCACGUGAAACCCGGAAG | 20 | 5487 |
| HSV1-UL30-3589 | + | UCUUGCUGCGCUUCUGGAAG | 20 | 12529 |
| HSV1-UL30-3590 | - | GCAGGCACGUGGGGUACCAG | 20 | 12530 |
| HSV1-UL30-2835 | - | UUAUUCUGCCGGACACCCAG | 20 | 5831 |
| HSV1-UL30-3591 | - | GCUAGACGCCGCCGCCCCAG | 20 | 12531 |
| HSV1-UL30-571 | - | GGAGGACGAGGAGCGGCCAG | 20 | 3509 |
| HSV1-UL30-3592 | - | CUGAACGAGCUGGCGGCCAG | 20 | 12532 |
| HSV1-UL30-2511 | + | AUGCGGCCGUACCCGUCCAG | 20 | 5517 |
| HSV1-UL30-3593 | - | CUCCCUGAGGGCCGACGCAG | 20 | 12533 |
| HSV1-UL30-3594 | + | GCCUGCAGUCCCUCGGGCAG | 20 | 12534 |
| HSV1-UL30-2566 | + | GUAGACGCGGUAAAACAGAG | 20 | 5571 |
| HSV1-UL30-3595 | + | UGCUCUGGGGAAUCCGCGAG | 20 | 12535 |
| HSV1-UL30-586 | - | GCGAGGAGGGCGGGGGCGAG | 20 | 3524 |
| HSV1-UL30-1814 | - | CGGACAACCUGGCCAUCGAG | 20 | 4864 |
| HSV1-UL30-570 | - | CAGCCCGGGAGGACGAGGAG | 20 | 3508 |
| HSV1-UL30-575 | - | AGGAGCGGCCAGAGGAGGAG | 20 | 3513 |
| HSV1-UL30-681 | - | CCGAGCGCCCCGCGGAGGAG | 20 | 3619 |
| HSV1-UL30-2006 | - | GGCGCAUCACCGACCCGGAG | 20 | 5039 |
| HSV1-UL30-3596 | - | CGACGAUACCGUCUCCGGAG | 20 | 12536 |
| HSV1-UL30-2015 | - | GGCCCAGACCCGCGAGGUAG | 20 | 5048 |
| HSV1-UL30-3597 | - | GACACCCAGGGGCGAUUUAG | 20 | 12537 |
| HSV1-UL30-534 | - | GCCGCCGGGCCCGCGCAACG | 20 | 3472 |
| HSV1-UL30-3598 | + | CGCGGGGCGCUCGGCUAACG | 20 | 12538 |
| HSV1-UL30-3599 | + | CAAAGUCGAACACCACCACG | 20 | 12539 |
| HSV1-UL30-595 | - | GGAGACCGCCGGCAGGCACG | 20 | 3533 |
| HSV1-UL30-3600 | - | CCGUUCACGUUUACGGCACG | 20 | 12540 |
| HSV1-UL30-3601 | + | GCAGCAGGCACGUAAAGACG | 20 | 12541 |
| HSV1-UL30-3602 | + | GCCCGCUUCGGACGUAGACG | 20 | 12542 |
| HSV1-UL30-3603 | - | GUGGCACCCCCCGGACGACG | 20 | 12543 |
| HSV1-UL30-3604 | - | UGGACGCCAUCACACCGACG | 20 | 12544 |
| HSV1-UL30-569 | - | GGCCGCAGCCCGGGAGGACG | 20 | 3507 |
| HSV1-UL30-578 | - | AGAGGAGGAGGGGGAGGACG | 20 | 3516 |
| HSV1-UL30-3605 | - | UUCCAUGCGCAUCAUCUACG | 20 | 12545 |
| HSV1-UL30-3606 | - | GUACAUCGGCGUCAUCUACG | 20 | 12546 |
| HSV1-UL30-3607 | + | UGCGCCGAUGGGCGUCUACG | 20 | 12547 |
| HSV1-UL30-3608 | - | CCCGGCCAUCAAGAAGUACG | 20 | 12548 |
| HSV1-UL30-3609 | + | AGCCGAAGGUGACGAACCCG | 20 | 12549 |
| HSV1-UL30-3610 | - | CGGGUUUCACGUGAACCCCG | 20 | 12550 |
| HSV1-UL30-709 | - | GGACGAGCCCGCCCCCCCCG | 20 | 3647 |
| HSV1-UL30-1960 | - | GAUUCCCCAGAGCAGCCCCG | 20 | 4993 |
| HSV1-UL30-1994 | - | CGCGUUAGCCGAGCGCCCCG | 20 | 5027 |
| HSV1-UL30-3611 | - | CAGGGGCCUGCCCACGCCCG | 20 | 12551 |
| HSV1-UL30-1400 | + | GCCGCGGAACGACGCGCCCG | 20 | 4338 |
| HSV1-UL30-3612 | + | UGCGCAUGGAAUAGGGCCCG | 20 | 12552 |
| HSV1-UL30-1999 | - | GCUGGCGCGACCCCUGCCCG | 20 | 5032 |
| HSV1-UL30-2363 | + | GGGCUUGGACGCGCCUCCCG | 20 | 5375 |
| HSV1-UL30-3613 | + | GCAGGCGACGGCGUCUCCCG | 20 | 12553 |
| HSV1-UL30-3614 | - | GAGCUACAAGCUCAACGCCG | 20 | 12554 |
| HSV1-UL30-3615 | + | CCGUGUUCAGGGCGACGCCG | 20 | 12555 |
| HSV1-UL30-1886 | - | GGGGCGAUUUAGGGGCGCCG | 20 | 4935 |
| HSV1-UL30-3616 | + | GCCCGGCGGCCGUGAGGCCG | 20 | 12556 |
| HSV1-UL30-3617 | - | UACGGCCGCAUGAACGGCCG | 20 | 12557 |
| HSV1-UL30-3618 | - | GGCCGCCGGGCUGACGGCCG | 20 | 12558 |
| HSV1-UL30-3619 | + | CGGGGACUCGCGCAGGGCCG | 20 | 12559 |
| HSV1-UL30-1124 | + | GGCCGGGGAGGGCAGGGCCG | 20 | 4062 |
| HSV1-UL30-3620 | - | UCUCUGCGAGCGCAUGGCCG | 20 | 12560 |
| HSV1-UL30-3621 | - | GCUGGUGUCCGAGCUGGCCG | 20 | 12561 |
| HSV1-UL30-2370 | + | CUCCCGGGGGCGCUUGGCCG | 20 | 5382 |
| HSV1-UL30-2571 | + | AGUGGUCCGCGGAGAUGCCG | 20 | 5576 |
| HSV1-UL30-3622 | - | UCCAUCUUUGUGCUGUGCCG | 20 | 12562 |
| HSV1-UL30-2408 | + | UCGCGCCAGCCACUCCUCCG | 20 | 5415 |
| HSV1-UL30-3623 | - | GUCGUUCCGCGGCAUCUCCG | 20 | 12563 |
| HSV1-UL30-2342 | + | CCGCGCGGCCACGUCGUCCG | 20 | 5355 |
| HSV1-UL30-2569 | + | CUCCGCCUCGAAGUGGUCCG | 20 | 5574 |
| HSV1-UL30-3624 | - | UCCCCGGGCGCGUCGUUCCG | 20 | 12564 |
| HSV1-UL30-3625 | + | AGACGCGGUAAAACAGAGCG | 20 | 12565 |
| HSV1-UL30-536 | - | CGCCGGGCCCGCGCAACGCG | 20 | 3474 |
| HSV1-UL30-579 | - | GGAGGACGAGGACGAACGCG | 20 | 3517 |
| HSV1-UL30-2847 | - | GCGCGGCCCAGUUCCACGCG | 20 | 11787 |
| HSV1-UL30-3626 | - | GAUCGUGGCCCAGACCCGCG | 20 | 12566 |
| HSV1-UL30-3627 | + | CCCUGCGGUCCGGAGCCGCG | 20 | 12567 |
| HSV1-UL30-1126 | + | CCGGGGAGGGCAGGGCCGCG | 20 | 4064 |
| HSV1-UL30-3628 | - | CCCCGGACGACGUGGCCGCG | 20 | 12568 |
| HSV1-UL30-3629 | + | GCGCCAGCCACUCCUCCGCG | 20 | 12569 |
| HSV1-UL30-3630 | + | CGCUUGUUGGUGUACGCGCG | 20 | 12570 |
| HSV1-UL30-2857 | + | UGGCGUCCAUAAACCGCGCG | 20 | 11797 |
| HSV1-UL30-1219 | + | CGAAGGCCGCCCAGCGCGCG | 20 | 4157 |
| HSV1-UL30-591 | - | AGCGGGAGCCGGAGGGCGCG | 20 | 3529 |
| HSV1-UL30-55 | - | GCACGCGUACGGCAUGCGCG | 20 | 3260 |
| HSV1-UL30-3631 | + | UGAUGGACGGGACCUGCGCG | 20 | 12571 |
| HSV1-UL30-3632 | - | AGGUAGAGGAGACGGUCGCG | 20 | 12572 |
| HSV1-UL30-1820 | - | UCGAUAUCGAAUGCAAGGCG | 20 | 4870 |
| HSV1-UL30-3633 | + | GCCCGGCGGCGUAGUAGGCG | 20 | 12573 |
| HSV1-UL30-3634 | - | UAAGAUGCUCAUCAAGGGCG | 20 | 12574 |
| HSV1-UL30-584 | - | GGACGAACGCGAGGAGGGCG | 20 | 3522 |
| HSV1-UL30-3635 | + | GAAUUCCAGAACCACGGGCG | 20 | 12575 |
| HSV1-UL30-3636 | + | CGCAGAGAUCUCGUGGGGCG | 20 | 12576 |
| HSV1-UL30-3637 | + | UCGGACACCAGCAGCUUGCG | 20 | 12577 |
| HSV1-UL30-3638 | + | GCCGAUCACCCCGCGUUGCG | 20 | 12578 |
| HSV1-UL30-3639 | + | GGAUGUCCCGAAGGCCAUCG | 20 | 12579 |
| HSV1-UL30-1812 | - | GGCGGACAACCUGGCCAUCG | 20 | 4862 |
| HSV1-UL30-3640 | - | CAAGGACUACCUGGAGAUCG | 20 | 12580 |
| HSV1-UL30-3641 | - | CCGGAUCCCGUACGUGAUCG | 20 | 12581 |
| HSV1-UL30-3642 | + | UGUACCCGGUCACGAACUCG | 20 | 12582 |
| HSV1-UL30-3643 | + | AGCGCGCGUGGACGUACUCG | 20 | 12583 |
| HSV1-UL30-3644 | + | CGACCGUCUCCUCUACCUCG | 20 | 12584 |
| HSV1-UL30-3645 | + | CCAGGAGCACGGCCUCCUCG | 20 | 12585 |
| HSV1-UL30-2383 | + | GGGCGGCGGCGUCUAGCUCG | 20 | 5390 |
| HSV1-UL30-3646 | - | UGCGAAAGCAGAUCCGCUCG | 20 | 12586 |
| HSV1-UL30-3647 | + | GGGUCGCGAGCAGCAUCUCG | 20 | 12587 |
| HSV1-UL30-2578 | + | AUGCGCUCGCAGAGAUCUCG | 20 | 5583 |
| HSV1-UL30-3648 | + | GCCGAUGGUCGUCACCGUCG | 20 | 12588 |
| HSV1-UL30-2530 | + | GCUCGUUCAGGUGGGAUUCG | 20 | 5536 |
| HSV1-UL30-3649 | - | CAUCUCCGCGGACCACUUCG | 20 | 12589 |
| HSV1-UL30-3650 | - | CGAGGGACUGCAGGCGUUCG | 20 | 12590 |
| HSV1-UL30-3651 | - | GCUCCGGACCGCAGGGUUCG | 20 | 12591 |
| HSV1-UL30-1782 | - | GUACUUUUACAUGAACAAGG | 20 | 4832 |
| HSV1-UL30-1818 | - | CUUCGAUAUCGAAUGCAAGG | 20 | 4868 |
| HSV1-UL30-1346 | + | AGGAACCGAGCGAAAACAGG | 20 | 4284 |
| HSV1-UL30-1089 | + | CGCACGCCGCCCCCAACAGG | 20 | 4027 |
| HSV1-UL30-3652 | - | CAGGCACGUGGGGUACCAGG | 20 | 12592 |
| HSV1-UL30-3653 | + | UGCGGCCGUACCCGUCCAGG | 20 | 12593 |
| HSV1-UL30-3654 | + | UGGCGAGCCAGUCCCGCAGG | 20 | 12594 |
| HSV1-UL30-3655 | + | GCUUGUAAUACACCGUCAGG | 20 | 12595 |
| HSV1-UL30-2526 | + | UGGCCGCCAGCUCGUUCAGG | 20 | 5532 |
| HSV1-UL30-572 | - | GGACGAGGAGCGGCCAGAGG | 20 | 3510 |
| HSV1-UL30-3656 | - | UCCCCAGAGCAGCCCCGAGG | 20 | 12596 |
| HSV1-UL30-580 | - | GGACGAGGACGAACGCGAGG | 20 | 3518 |
| HSV1-UL30-3657 | - | GGACAACCUGGCCAUCGAGG | 20 | 12597 |
| HSV1-UL30-1946 | - | GGACUACCUGGAGAUCGAGG | 20 | 4979 |
| HSV1-UL30-1790 | - | CUCCGCGGACCACUUCGAGG | 20 | 4840 |
| HSV1-UL30-3658 | - | CUUUUACAUGAACAAGGAGG | 20 | 12598 |
| HSV1-UL30-573 | - | CGAGGAGCGGCCAGAGGAGG | 20 | 3511 |
| HSV1-UL30-576 | - | GGAGCGGCCAGAGGAGGAGG | 20 | 3514 |
| HSV1-UL30-3659 | - | CCUGGCCGAUUUCCCGGAGG | 20 | 12599 |
| HSV1-UL30-3660 | - | GGACCACUUCGAGGCGGAGG | 20 | 12600 |
| HSV1-UL30-3661 | - | CGCAGUGGCGCACCUGGAGG | 20 | 12601 |
| HSV1-UL30-3662 | + | CCCGCAGGAGGAUGCUGAGG | 20 | 12602 |
| HSV1-UL30-2499 | + | GGGCCCGGCGGCGUAGUAGG | 20 | 5505 |
| HSV1-UL30-2048 | - | GGUGGGUGCCGGCGCUACGG | 20 | 5081 |
| HSV1-UL30-3663 | - | UACAUCGGCGUCAUCUACGG | 20 | 12603 |
| HSV1-UL30-3664 | - | CGUCGAGUUUAACUGUACGG | 20 | 12604 |
| HSV1-UL30-3665 | + | AAACGUGAACGGCCACCCGG | 20 | 12605 |
| HSV1-UL30-3666 | - | UCCGGUGGCCGGGCACCCGG | 20 | 12606 |
| HSV1-UL30-3667 | + | GCCCACGGCCGUCAGCCCGG | 20 | 12607 |
| HSV1-UL30-3668 | + | GCGCAUGGAAUAGGGCCCGG | 20 | 12608 |
| HSV1-UL30-3669 | + | CCCGCGUUGCGCGGGCCCGG | 20 | 12609 |
| HSV1-UL30-3670 | + | GGCUUGGACGCGCCUCCCGG | 20 | 12610 |
| HSV1-UL30-3671 | + | CAGGCGACGGCGUCUCCCGG | 20 | 12611 |
| HSV1-UL30-3672 | - | GCUCCUGGCCGAUUUCCCGG | 20 | 12612 |
| HSV1-UL30-3673 | - | CAACACGCUAGCCCAGCCGG | 20 | 12613 |
| HSV1-UL30-589 | - | CGGGGGCGAGCGGGAGCCGG | 20 | 3527 |
| HSV1-UL30-1887 | - | GGGCGAUUUAGGGGCGCCGG | 20 | 4936 |
| HSV1-UL30-3674 | + | GUACCCCACGUGCCUGCCGG | 20 | 12614 |
| HSV1-UL30-3675 | + | CGCGCGGCCACGUCGUCCGG | 20 | 12615 |
| HSV1-UL30-3676 | - | GGACGAGCUGGCCUUUCCGG | 20 | 12616 |
| HSV1-UL30-3677 | + | GGGGCGCUCGGCUAACGCGG | 20 | 12617 |
| HSV1-UL30-1996 | - | GUUAGCCGAGCGCCCCGCGG | 20 | 5029 |
| HSV1-UL30-1127 | + | CGGGGAGGGCAGGGCCGCGG | 20 | 4065 |
| HSV1-UL30-3678 | + | UGGACGGGACCUGCGCGCGG | 20 | 12618 |
| HSV1-UL30-3679 | + | CGGCGGCGUCUAGCUCGCGG | 20 | 12619 |
| HSV1-UL30-1821 | - | CGAUAUCGAAUGCAAGGCGG | 20 | 4871 |
| HSV1-UL30-3680 | - | CGCGGACCACUUCGAGGCGG | 20 | 12620 |
| HSV1-UL30-2050 | - | GGGUGCCGGCGCUACGGCGG | 20 | 5083 |
| HSV1-UL30-3681 | + | GAGGACGUGCUCCAGGGCGG | 20 | 12621 |
| HSV1-UL30-585 | - | GACGAACGCGAGGAGGGCGG | 20 | 3523 |
| HSV1-UL30-3682 | - | GACCGCAGGGUUCGGGGCGG | 20 | 12622 |
| HSV1-UL30-3683 | + | GGGCUCGUCCCCUGGGGCGG | 20 | 12623 |
| HSV1-UL30-3684 | + | CGGGUGUCUGCUCAGUUCGG | 20 | 12624 |
| HSV1-UL30-3685 | + | CAGGAGGACGUGCUCCAGGG | 20 | 12625 |
| HSV1-UL30-1800 | - | GCCAUCAAGAAGUACGAGGG | 20 | 4850 |
| HSV1-UL30-3686 | - | GACAACCUGGCCAUCGAGGG | 20 | 12626 |
| HSV1-UL30-582 | - | GAGGACGAACGCGAGGAGGG | 20 | 3520 |
| HSV1-UL30-3687 | + | GGCGUCUAGCUCGCGGAGGG | 20 | 12627 |
| HSV1-UL30-3688 | + | GUUGUCCAGGAUGAACCGGG | 20 | 12628 |
| HSV1-UL30-3689 | - | GACUCCGGAAGGCCACCGGG | 20 | 12629 |
| HSV1-UL30-3690 | - | UCGCCUGCCGACCCCCCGGG | 20 | 12630 |
| HSV1-UL30-3691 | - | GCGUCCGGCCGCAGCCCGGG | 20 | 12631 |
| HSV1-UL30-3692 | + | GCUUGGACGCGCCUCCCGGG | 20 | 12632 |
| HSV1-UL30-1888 | - | GGCGAUUUAGGGGCGCCGGG | 20 | 4937 |
| HSV1-UL30-3693 | + | GCGCGGCCACGUCGUCCGGG | 20 | 12633 |
| HSV1-UL30-1128 | + | GGGGAGGGCAGGGCCGCGGG | 20 | 4066 |
| HSV1-UL30-1822 | - | GAUAUCGAAUGCAAGGCGGG | 20 | 4872 |
| HSV1-UL30-1889 | - | GCGAUUUAGGGGCGCCGGGG | 20 | 4938 |
| HSV1-UL30-3694 | + | CCGGGGGCGCUUGGCCGGGG | 20 | 12634 |
| HSV1-UL30-1129 | + | GGGAGGGCAGGGCCGCGGGG | 20 | 4067 |
| HSV1-UL30-1823 | - | AUAUCGAAUGCAAGGCGGGG | 20 | 4873 |
| HSV1-UL30-3695 | + | CGUUCAGGUGGGAUUCGGGG | 20 | 12635 |
| HSV1-UL30-3696 | - | CCGGACCGCAGGGUUCGGGG | 20 | 12636 |
| HSV1-UL30-577 | - | GCGGCCAGAGGAGGAGGGGG | 20 | 3515 |
| HSV1-UL30-1130 | + | GGAGGGCAGGGCCGCGGGGG | 20 | 4068 |
| HSV1-UL30-1824 | - | UAUCGAAUGCAAGGCGGGGG | 20 | 4874 |
| HSV1-UL30-3697 | - | AUUUAGGGGCGCCGGGGGGG | 20 | 12637 |
| HSV1-UL30-1131 | + | GGGCAGGGCCGCGGGGGGGG | 20 | 4069 |
| HSV1-UL30-3698 | - | CGAAUGCAAGGCGGGGGGGG | 20 | 12638 |
| HSV1-UL30-3699 | - | CUUCUCCCACCUGUUGGGGG | 20 | 12639 |
| HSV1-UL30-3700 | + | GGCGGGCUCGUCCCCUGGGG | 20 | 12640 |
| HSV1-UL30-3701 | - | ACCUGGAGAUCGAGGUGGGG | 20 | 12641 |
| HSV1-UL30-3702 | + | UCGCACUCGAGUUUGAUGGG | 20 | 12642 |
| HSV1-UL30-3703 | - | GUACGUCCACGCGCGCUGGG | 20 | 12643 |
| HSV1-UL30-3704 | - | UACCUGGAGAUCGAGGUGGG | 20 | 12644 |
| HSV1-UL30-3705 | + | GUUACACACGACCUUGAUGG | 20 | 12645 |
| HSV1-UL30-3706 | + | UUCGCACUCGAGUUUGAUGG | 20 | 12646 |
| HSV1-UL30-3707 | - | CGACGCAGUGGCGCACCUGG | 20 | 12647 |
| HSV1-UL30-3708 | + | CAGGUCGACCAGGGCCCUGG | 20 | 12648 |
| HSV1-UL30-3709 | - | AUCCCACCUGAACGAGCUGG | 20 | 12649 |
| HSV1-UL30-3710 | - | CAUACAGGAUUCCCUGCUGG | 20 | 12650 |
| HSV1-UL30-3711 | - | GUUUCACGUGAACCCCGUGG | 20 | 12651 |
| HSV1-UL30-1949 | - | CUACCUGGAGAUCGAGGUGG | 20 | 4982 |
| HSV1-UL30-1792 | - | CCACUUCGAGGCGGAGGUGG | 20 | 4842 |
| HSV1-UL30-3712 | + | GACGUGCUCCAGGGCGGUGG | 20 | 12652 |
| HSV1-UL30-3713 | + | GUCCAGGAUGAACCGGGUGG | 20 | 12653 |
| HSV1-UL30-3714 | - | CUCGGCCGUCGCGCGCUUGG | 20 | 12654 |
| HSV1-UL30-3715 | - | UUACUUCUCCCACCUGUUGG | 20 | 12655 |
| HSV1-UL30-3716 | + | ACACGCCCCGGCCGUUCAUG | 20 | 12656 |
| HSV1-UL30-3717 | + | ACGGCCGAGAGCUCCAGAUG | 20 | 12657 |
| HSV1-UL30-3718 | + | GAAACAGCGCGCGCGAGAUG | 20 | 12658 |
| HSV1-UL30-2420 | + | UUUCGCACUCGAGUUUGAUG | 20 | 5427 |
| HSV1-UL30-2355 | + | GCCGUGGGCAAUGGCGUAUG | 20 | 5367 |
| HSV1-UL30-3719 | + | ACCCACCGCCCCGAACCCUG | 20 | 12659 |
| HSV1-UL30-3720 | + | GGGGGCGGGCUCGUCCCCUG | 20 | 12660 |
| HSV1-UL30-3721 | - | GCUUCAGCACGCUCUCCCUG | 20 | 12661 |
| HSV1-UL30-1954 | - | GCCUCCUCAGCAUCCUCCUG | 20 | 4987 |
| HSV1-UL30-3722 | + | CUCCUCGUCCUCCCGGGCUG | 20 | 12662 |
| HSV1-UL30-2461 | + | AGUCCCGCAGGAGGAUGCUG | 20 | 5467 |
| HSV1-UL30-3723 | + | UGUGGGCCUGGAUGAUGCUG | 20 | 12663 |
| HSV1-UL30-2452 | + | GCCUCCUCGGGGCUGCUCUG | 20 | 5458 |
| HSV1-UL30-3724 | - | AAAGGUUUAUUCCCGAAGUG | 20 | 12664 |
| HSV1-UL30-3725 | + | UCACGUGAAACCCGGAAGUG | 20 | 12665 |
| HSV1-UL30-3726 | - | AGACCGCCGGCAGGCACGUG | 20 | 12666 |
| HSV1-UL30-3727 | + | CCGUCAGCCCGGCGGCCGUG | 20 | 12667 |
| HSV1-UL30-1852 | - | GCCGGGGCGUGUUUCGCGUG | 20 | 4902 |
| HSV1-UL30-3728 | + | GCGCUCGCAGAGAUCUCGUG | 20 | 12668 |
| HSV1-UL30-1948 | - | ACUACCUGGAGAUCGAGGUG | 20 | 4981 |
| HSV1-UL30-3729 | + | CGACCAGGGCCCUGGAGGUG | 20 | 12669 |
| HSV1-UL30-3730 | + | GUCUGCUCAGUUCGGCGGUG | 20 | 12670 |
| HSV1-UL30-3731 | - | CAUCAAGAAGUACGAGGGUG | 20 | 12671 |
| HSV1-UL30-3732 | + | GCUGGCCGUCGUAGAUGGUG | 20 | 12672 |
| HSV1-UL30-2358 | + | GCUCGGACACCAGCAGCUUG | 20 | 5370 |
| HSV1-UL30-3733 | + | GCGUGCUGAAGCACAGGUUG | 20 | 12673 |
| HSV1-UL30-3734 | - | AUUACUUCUCCCACCUGUUG | 20 | 12674 |
| HSV1-UL30-3735 | + | GUCGGCCGCCUCCGGGAAAU | 20 | 12675 |
| HSV1-UL30-3736 | + | GUAGAUGAUGCGCAUGGAAU | 20 | 12676 |
| HSV1-UL30-3737 | - | GUGUUUC GC GUGUGGGACAU | 20 | 12677 |
| HSV1-UL30-3738 | - | AUCGCCAAGAAAAAGUACAU | 20 | 12678 |
| HSV1-UL30-3739 | - | GCCGCGACGGUGACGACCAU | 20 | 12679 |
| HSV1-UL30-3740 | - | CCGUCCUCGUAGACGCCCAU | 20 | 12680 |
| HSV1-UL30-2551 | + | CUGGAUGUCCCGAAGGCCAU | 20 | 5556 |
| HSV1-UL30-3741 | + | GACGGCCGAGAGCUCCAGAU | 20 | 12681 |
| HSV1-UL30-3742 | + | CGGGUCGGUGAUGCGCCGAU | 20 | 12682 |
| HSV1-UL30-3743 | - | CCCGCGCAACGCGGGGUGAU | 20 | 12683 |
| HSV1-UL30-2419 | + | UUUUCGCACUCGAGUUUGAU | 20 | 5426 |
| HSV1-UL30-2517 | + | GUUGUACCCGGUCACGAACU | 20 | 5523 |
| HSV1-UL30-2860 | + | CAUAAACCGCGCGUGGAACU | 20 | 11800 |
| HSV1-UL30-3744 | - | CAUCAAGGUCGUGUGUAACU | 20 | 12684 |
| HSV1-UL30-3745 | + | GGCGCCCCUAAAUCGCCCCU | 20 | 12685 |
| HSV1-UL30-3746 | + | GGGGGGCGGGCUCGUCCCCU | 20 | 12686 |
| HSV1-UL30-3747 | + | CCCGAACGCCUGCAGUCCCU | 20 | 12687 |
| HSV1-UL30-3748 | + | AAUGGCGUAUGCGGGAUCCU | 20 | 12688 |
| HSV1-UL30-2448 | + | GUCCAGGAGCACGGCCUCCU | 20 | 5454 |
| HSV1-UL30-3749 | - | GGGACCGUCAUCACGCUCCU | 20 | 12689 |
| HSV1-UL30-3750 | + | UGCGGGAUCCUCGGCCAGCU | 20 | 12690 |
| HSV1-UL30-3751 | + | CUUGGCGAUCAGCAGCAGCU | 20 | 12691 |
| HSV1-UL30-3752 | + | CUUGUAAAUGUCCGUCAGCU | 20 | 12692 |
| HSV1-UL30-3753 | + | CCGGGCUGCGGCCGGACGCU | 20 | 12693 |
| HSV1-UL30-3754 | - | CGAGUACGUCCACGCGCGCU | 20 | 12694 |
| HSV1-UL30-3755 | - | GCUCUCGGCCGUCGCGCGCU | 20 | 12695 |
| HSV1-UL30-3756 | + | CCACUCCUCCGCGGGGCGCU | 20 | 12696 |
| HSV1-UL30-3757 | + | CGGCGUCUCCCGGGGGCGCU | 20 | 12697 |
| HSV1-UL30-3758 | - | CACGUCCUCCUGUUUUCGCU | 20 | 12698 |
| HSV1-UL30-3759 | + | GGCCAUCGGGGCCGCCGGCU | 20 | 12699 |
| HSV1-UL30-3760 | + | CACCAGCAGCUUGCGGGGCU | 20 | 12700 |
| HSV1-UL30-3761 | + | UUGUGGGCCUGGAUGAUGCU | 20 | 12701 |
| HSV1-UL30-3762 | + | UAACAGACUCUCGGUGAUCU | 20 | 12702 |
| HSV1-UL30-3763 | + | AAACCUUUUUAACAGACUCU | 20 | 12703 |
| HSV1-UL30-3764 | - | UUUGCCCCAUCUGGAGCUCU | 20 | 12704 |
| HSV1-UL30-2451 | + | GGCCUCCUCGGGGCUGCUCU | 20 | 5457 |
| HSV1-UL30-3765 | + | CUCCUCUACCUCGCGGGUCU | 20 | 12705 |
| HSV1-UL30-3766 | + | GACGCCGAUGUACUUUUUCU | 20 | 12706 |
| HSV1-UL30-3767 | + | UUCACGUGAAACCCGGAAGU | 20 | 12707 |
| HSV1-UL30-3768 | + | CGCGGGCCCGGCGGCGUAGU | 20 | 12708 |
| HSV1-UL30-3769 | + | CGUCUCGUAGUAGUACACGU | 20 | 12709 |
| HSV1-UL30-3770 | - | GAGACCGCCGGCAGGCACGU | 20 | 12710 |
| HSV1-UL30-3771 | + | AGCGUGAUGACGGUCCCCGU | 20 | 12711 |
| HSV1-UL30-3772 | + | CGUGUUCAGGGCGACGCCGU | 20 | 12712 |
| HSV1-UL30-3773 | - | GCCGCCGGGCUGACGGCCGU | 20 | 12713 |
| HSV1-UL30-3774 | + | AAUUCCAGAACCACGGGCGU | 20 | 12714 |
| HSV1-UL30-3775 | + | CUCCAGGUGCGCCACUGCGU | 20 | 12715 |
| HSV1-UL30-3776 | + | UGCGCUCGCAGAGAUCUCGU | 20 | 12716 |
| HSV1-UL30-2350 | + | GCACGCCGCCCCCAACAGGU | 20 | 5362 |
| HSV1-UL30-3777 | + | CUUGUAAUACACCGUCAGGU | 20 | 12717 |
| HSV1-UL30-3778 | + | GGCCGCCAGCUCGUUCAGGU | 20 | 12718 |
| HSV1-UL30-1947 | - | GACUACCUGGAGAUCGAGGU | 20 | 4980 |
| HSV1-UL30-3779 | + | ACAUGAGCUUGUAUGCCGGU | 20 | 12719 |
| HSV1-UL30-3780 | - | ACCGCAGGGUUCGGGGCGGU | 20 | 12720 |
| HSV1-UL30-3781 | - | CCAUCAAGAAGUACGAGGGU | 20 | 12721 |
| HSV1-UL30-3782 | + | CUGGAUGUCCCUCUCCGGGU | 20 | 12722 |
| HSV1-UL30-3783 | + | GGACGCGCCUCCCGGGGGGU | 20 | 12723 |
| HSV1-UL30-3784 | - | AUACAGGAUUCCCUGCUGGU | 20 | 12724 |
| HSV1-UL30-3785 | + | CAGAAUAAAGCCCUUCUGGU | 20 | 12725 |
| HSV1-UL30-3786 | + | GGGCCCGGGGGCGCGCAUGU | 20 | 12726 |
| HSV1-UL30-2033 | - | CUAUUACUUCUCCCACCUGU | 20 | 5066 |
| HSV1-UL30-3787 | - | CAGCGAAUUCGAGAUGCUGU | 20 | 12727 |
| HSV1-UL30-3788 | - | CCGGGGCGUGUUUCGCGUGU | 20 | 12728 |
| HSV1-UL30-3789 | + | CUCGUGGGGCGCGGCAUUGU | 20 | 12729 |
| HSV1-UL30-3790 | + | CAGGUGGGC CAGGC GCUUGU | 20 | 12730 |
| HSV1-UL30-3791 | + | GCUCGAGAGCUUGAUCUUGU | 20 | 12731 |
| HSV1-UL30-3792 | + | CGUGCUGAAGCACAGGUUGU | 20 | 12732 |
| HSV1-UL30-2528 | + | CAGCUCGUUCAGGUGGGAUU | 20 | 5534 |
| HSV1-UL30-2344 | + | GUCCGGGGGGUGCCACACUU | 20 | 5357 |
| HSV1-UL30-3793 | - | AACCCCGGGUUCGUCACCUU | 20 | 12733 |
| HSV1-UL30-1807 | - | CCGGCGGCCCCGAUGGCCUU | 20 | 4857 |
| HSV1-UL30-3794 | + | CGGGCUGCGGCCGGACGCUU | 20 | 12734 |
| HSV1-UL30-3795 | + | ACGACAGCACACGCCCGCUU | 20 | 12735 |
| HSV1-UL30-3796 | + | CUUGUCCUUCAGGACGGCUU | 20 | 12736 |
| HSV1-UL30-3797 | + | GCGCGGGUGUCUGCUCAGUU | 20 | 12737 |
| HSV1-UL30-2001 | - | CCCGAGGGACUGCAGGCGUU | 20 | 5034 |
| HSV1-UL30-2045 | - | CGGCUCCGGACCGCAGGGUU | 20 | 5078 |
| HSV1-UL30-2034 | - | UAUUACUUCUCCCACCUGUU | 20 | 5067 |
| HSV1-UL30-2036 | - | GUGACAUUCAAGGCCCUGUU | 20 | 5069 |
| HSV1-UL30-1882 | - | CGGACACCCAGGGGCGAUUU | 20 | 4931 |
| HSV1-UL30-2037 | - | UGACAUUCAAGGCCCUGUUU | 20 | 5070 |

**Table 9A** provides exemplary targeting domains for knocking out the *UL30* gene selected according to the first tier parameters. The targeting domains bind within the first 500 bp of the coding sequence (e.g., within 500 bp downstream from the start codon), have a high level of orthogonality, and start with a 5'G. PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9A**

| **1st Tier** | | | | |
|---|---|---|---|---|
| **gRNA Name** | **DNA Stran d** | **Targeting Domain** | **Target Site Length** | **Seq ID NO:** |
| HSV1-UL30-3798 | + | GUUUUGCCUCAAACAAGGC | 19 | 12738 |
| HSV1-UL30-3799 | + | GUACGCGUGCUCCACGUUCUC | 21 | 12739 |
| HSV1-UL30-3800 | + | GCCGUACGCGUGCUCCACGUUCUC | 24 | 12740 |
| HSV1-UL30-3801 | + | GUCCAGCACCCGCGGGGCG | 19 | 12741 |
| HSV1-UL30-3802 | + | GCUAUAGUACGUAUGGCG | 18 | 12742 |
| HSV1-UL30-3803 | + | GCAUUCGCUAUAGUACGUAUGGCG | 24 | 12743 |
| HSV1-UL30-3804 | + | GUACACGUGAAAGACGGUGACGG | 23 | 12744 |
| HSV1-UL30-3805 | + | GCGUCCCGACUGGGGCGAGGU | 21 | 12745 |
| HSV1-UL30-3806 | + | GUUGCGUCCCGACUGGGGCGAGGU | 24 | 12746 |
| HSV1-UL30-3807 | - | GCCCCGCGGGUGCUGGAC | 18 | 12747 |
| HSV1-UL30-255 | - | GAAUUUCGAUUCAUCGCCCC | 20 | 4721 |
| HSV1-UL30-3808 | - | GAUGAAUUUCGAUUCAUCGCCCC | 23 | 12748 |
| HSV1-UL30-25 | - | GCCCCCCCGGAGAAGCGCGC | 20 | 3131 |
| HSV1-UL30-3809 | - | GAUGCCCCCCCGGAGAAGCGCGC | 23 | 12749 |
| HSV1-UL30-3810 | - | GGAUGCCCCCCCGGAGAAGCGCGC | 24 | 12750 |
| HSV1-UL30-3811 | - | GAGCGCGACGUCCUCCGCG | 19 | 12751 |
| HSV1-UL30-3812 | - | GACGAGCGCGACGUCCUCCGCG | 22 | 12752 |
| HSV1-UL30-3813 | - | GGACGAGCGCGACGUCCUCCGCG | 23 | 12753 |
| HSV1-UL30-3814 | - | GGGACGAGCGCGACGUCCUCCGCG | 24 | 12754 |
| HSV1-UL30-3815 | - | GCCAUACGUACUAUAGCGAAUGCG | 24 | 12755 |
| HSV1-UL30-3816 | - | GCGGCGUGGACCACGCCCCGG | 21 | 12756 |
| HSV1-UL30-3817 | - | GGCGGCGUGGACCACGCCCCGG | 22 | 12757 |
| HSV1-UL30-3818 | - | GGGCGGCGUGGACCACGCCCCGG | 23 | 12758 |
| HSV1-UL30-3819 | - | GGGGCGGCGUGGACCACGCCCCGG | 24 | 12759 |
| HSV1-UL30-3820 | - | GAAAGUCGGCGGCCAGGGCGGCGU | 24 | 12760 |

**Table 9B** provides exemplary targeting domains for knocking out the *UL30* gene selected according to the second tier parameters. The targeting domains bind within the first 500 bp of the coding sequence (e.g., within 500 bp downstream from the start codon) and have a high level of orthogonality. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9B**

| **2nd Tier** | | | | |
|---|---|---|---|---|
| **gRNA Name** | **DNA Strand** | **Targeting Domain** | **Target Site Length** | **Seq ID NO:** |
| HSV1-UL30-3821 | + | UUUUGCCUCAAACAAGGC | 18 | 12761 |
| HSV1-UL30-100 | + | AGUUUUGCCUCAAACAAGGC | 20 | 3283 |
| HSV1-UL30-3822 | + | AAGUUUUGCCUCAAACAAGGC | 21 | 12762 |
| HSV1-UL30-3823 | + | AAAGUUUUGCCUCAAACAAGGC | 22 | 12763 |
| HSV1-UL30-3824 | + | AAAAGUUUUGCCUCAAACAAGGC | 23 | 12764 |
| HSV1-UL30-3825 | + | UAAAAGUUUUGCCUCAAACAAGGC | 24 | 12765 |
| HSV1-UL30-3826 | + | CGCGUGCUCCACGUUCUC | 18 | 12766 |
| HSV1-UL30-3827 | + | ACGCGUGCUCCACGUUCUC | 19 | 12767 |
| HSV1-UL30-329 | + | UACGCGUGCUCCACGUUCUC | 20 | 4767 |
| HSV1-UL30-3828 | + | CGUACGCGUGCUCCACGUUCUC | 22 | 12768 |
| HSV1-UL30-3829 | + | CCGUACGCGUGCUCCACGUUCUC | 23 | 12769 |
| HSV1-UL30-3830 | + | UCCAGCACCCGCGGGGCG | 18 | 12770 |
| HSV1-UL30-352 | + | CGUCCAGCACCCGCGGGGCG | 20 | 4780 |
| HSV1-UL30-3831 | + | UCGUCCAGCACCCGCGGGGCG | 21 | 12771 |
| HSV1-UL30-3832 | + | CUCGUCCAGCACCCGCGGGGCG | 22 | 12772 |
| HSV1-UL30-3833 | + | CCUCGUCCAGCACCCGCGGGGCG | 23 | 12773 |
| HSV1-UL30-3834 | + | UCCUCGUCCAGCACCCGCGGGGCG | 24 | 12774 |
| HSV1-UL30-3835 | + | CGCUAUAGUACGUAUGGCG | 19 | 12775 |
| HSV1-UL30-354 | + | UCGCUAUAGUACGUAUGGCG | 20 | 4782 |
| HSV1-UL30-3836 | + | UUCGCUAUAGUACGUAUGGCG | 21 | 12776 |
| HSV1-UL30-3837 | + | AUUCGCUAUAGUACGUAUGGCG | 22 | 12777 |
| HSV1-UL30-3838 | + | CAUUCGCUAUAGUACGUAUGGCG | 23 | 12778 |
| HSV1-UL30-3839 | + | CGUGAAAGACGGUGACGG | 18 | 12779 |
| HSV1-UL30-3840 | + | ACGUGAAAGACGGUGACGG | 19 | 12780 |
| HSV1-UL30-59 | + | CACGUGAAAGACGGUGACGG | 20 | 3262 |
| HSV1-UL30-3841 | + | ACACGUGAAAGACGGUGACGG | 21 | 12781 |
| HSV1-UL30-3842 | + | UACACGUGAAAGACGGUGACGG | 22 | 12782 |
| HSV1-UL30-3843 | + | CGUACACGUGAAAGACGGUGACGG | 24 | 12783 |
| HSV1-UL30-3844 | + | UGCGUCCCGACUGGGGCGAGGU | 22 | 12784 |
| HSV1-UL30-3845 | + | UUGCGUCCCGACUGGGGCGAGGU | 23 | 12785 |
| HSV1-UL30-3846 | - | AGCGCCAUACGUACUAUA | 18 | 12786 |
| HSV1-UL30-3847 | - | CAGCGCCAUACGUACUAUA | 19 | 12787 |
| HSV1-UL30-253 | - | CCAGCGCCAUACGUACUAUA | 20 | 4719 |
| HSV1-UL30-3848 | - | CCCAGCGCCAUACGUACUAUA | 21 | 12788 |
| HSV1-UL30-3849 | - | ACCCAGCGCCAUACGUACUAUA | 22 | 12789 |
| HSV1-UL30-3850 | - | AACCCAGCGCCAUACGUACUAUA | 23 | 12790 |
| HSV1-UL30-3851 | - | CAACCCAGCGCCAUACGUACUAUA | 24 | 12791 |
| HSV1-UL30-3852 | - | CGCCCCGCGGGUGCUGGAC | 19 | 12792 |
| HSV1-UL30-258 | - | UCGCCCCGCGGGUGCUGGAC | 20 | 4724 |
| HSV1-UL30-3853 | - | AUCGCCCCGCGGGUGCUGGAC | 21 | 12793 |
| HSV1-UL30-3854 | - | CAUCGCCCCGCGGGUGCUGGAC | 22 | 12794 |
| HSV1-UL30-3855 | - | UCAUCGCCCCGCGGGUGCUGGAC | 23 | 12795 |
| HSV1-UL30-3856 | - | UUCAUCGCCCCGCGGGUGCUGGAC | 24 | 12796 |
| HSV1-UL30-3857 | - | AUUUCGAUUCAUCGCCCC | 18 | 12797 |
| HSV1-UL30-3858 | - | AAUUUCGAUUCAUCGCCCC | 19 | 12798 |
| HSV1-UL30-3859 | - | UGAAUUUCGAUUCAUCGCCCC | 21 | 12799 |
| HSV1-UL30-3860 | - | AUGAAUUUCGAUUCAUCGCCCC | 22 | 12800 |
| HSV1-UL30-3861 | - | CGAUGAAUUUCGAUUCAUCGCCCC | 24 | 12801 |
| HSV1-UL30-3862 | - | CCCCCCGGAGAAGCGCGC | 18 | 12802 |
| HSV1-UL30-3863 | - | CCCCCCCGGAGAAGCGCGC | 19 | 12803 |
| HSV1-UL30-3864 | - | UGCCCCCCCGGAGAAGCGCGC | 21 | 12804 |
| HSV1-UL30-3865 | - | AUGCCCCCCCGGAGAAGCGCGC | 22 | 12805 |
| HSV1-UL30-3866 | - | AGCGCGACGUCCUCCGCG | 18 | 12806 |
| HSV1-UL3 0-271 | - | CGAGCGCGACGUCCUCCGCG | 20 | 4730 |
| HSV1-UL30-3867 | - | ACGAGCGCGACGUCCUCCGCG | 21 | 12807 |
| HSV1-UL30-3868 | - | CGUACUAUAGCGAAUGCG | 18 | 12808 |
| HSV1-UL30-3869 | - | ACGUACUAUAGCGAAUGCG | 19 | 12809 |
| HSV1-UL30-254 | - | UACGUACUAUAGCGAAUGCG | 20 | 4720 |
| HSV1-UL30-3870 | - | AUACGUACUAUAGCGAAUGCG | 21 | 12810 |
| HSV1-UL30-3871 | - | CAUACGUACUAUAGCGAAUGCG | 22 | 12811 |
| HSV1-UL30-3872 | - | CCAUACGUACUAUAGCGAAUGCG | 23 | 12812 |
| HSV1-UL30-3873 | - | AGUCGGCGGCCAGGGCGGCGU | 21 | 12813 |
| HSV1-UL30-3874 | - | AAGUCGGCGGCCAGGGCGGCGU | 22 | 12814 |
| HSV1-UL30-3875 | - | AAAGUCGGCGGCCAGGGCGGCGU | 23 | 12815 |

**Table 9C** provides exemplary targeting domains for knocking out the *UL30* gene selected according to the third tier parameters. The targeting domains bind within the first 500 bp of the coding sequence (e.g., within 500 bp downstream from the start codon). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9C**

| **3rd Tier** | | | | |
|---|---|---|---|---|
| **gRNA Name** | **DNA Strand** | **Targeting Domain** | **Target Site Length** | **Seq ID NO:** |
| HSV1-UL30-3876 | + | UCCCGACUGGGGCGAGGU | 18 | 12816 |
| HSV1-UL30-3877 | + | GUCCCGACUGGGGCGAGGU | 19 | 12817 |
| HSV1-UL30-95 | + | CGUCCCGACUGGGGCGAGGU | 20 | 3170 |
| HSV1-UL30-3878 | - | GCGUGGACCACGCCCCGG | 18 | 12818 |
| HSV1-UL30-3879 | - | GGCGUGGACCACGCCCCGG | 19 | 12819 |
| HSV1-UL30-49 | - | CGGCGUGGACCACGCCCCGG | 20 | 3259 |
| HSV1-UL30-3880 | - | CGGCGGCCAGGGCGGCGU | 18 | 12820 |
| HSV1-UL30-3881 | - | UCGGCGGCCAGGGCGGCGU | 19 | 12821 |
| HSV1-UL30-242 | - | GUCGGCGGCCAGGGCGGCGU | 20 | 4712 |

**Table 9D** provides exemplary targeting domains for knocking out the *UL30* gene selected according to the fourth tier parameters. The targeting domains bind within the first 500 bp of the coding sequence (e.g., within 500 bp downstream from the start codon). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9D**

| **4th Tier** | | | | |
|---|---|---|---|---|
| **gRNA Name** | **DNA Strand** | **Targeting Domain** | **Target Site Length** | **Seq ID NO:** |
| HSV1-UL30-3882 | + | CAUAAACCGCGCGUGGAA | 18 | 12822 |
| HSV1-UL30-3883 | + | CCAUAAACCGCGCGUGGAA | 19 | 12823 |
| HSV1-UL30-3884 | + | UCCAUAAACCGCGCGUGGAA | 20 | 12824 |
| HSV1-UL30-3885 | + | GUCCAUAAACCGCGCGUGGAA | 21 | 12825 |
| HSV1-UL30-3886 | + | CGUCCAUAAACCGCGCGUGGAA | 22 | 12826 |
| HSV1-UL30-3887 | + | GCGUCCAUAAACCGCGCGUGGAA | 23 | 12827 |
| HSV1-UL30-3888 | + | GGCGUCCAUAAACCGCGCGUGGAA | 24 | 12828 |
| HSV1-UL30-3889 | + | CACCCGCGGGGCGAUGAA | 18 | 12829 |
| HSV1-UL30-3890 | + | GCACCCGCGGGGCGAUGAA | 19 | 12830 |
| HSV1-UL30-353 | + | AGCACCCGCGGGGCGAUGAA | 20 | 4781 |
| HSV1-UL30-3891 | + | CAGCACCCGCGGGGCGAUGAA | 21 | 12831 |
| HSV1-UL30-3892 | + | CCAGCACCCGCGGGGCGAUGAA | 22 | 12832 |
| HSV1-UL30-3893 | + | UCCAGCACCCGCGGGGCGAUGAA | 23 | 12833 |
| HSV1-UL30-3894 | + | GUCCAGCACCCGCGGGGCGAUGAA | 24 | 12834 |
| HSV1-UL30-3895 | + | ACUUUCCUCCGGGGGACA | 18 | 12835 |
| HSV1-UL30-3896 | + | GACUUUCCUCCGGGGGACA | 19 | 12836 |
| HSV1-UL30-371 | + | CGACUUUCCUCCGGGGGACA | 20 | 4792 |
| HSV1-UL30-3897 | + | CCGACUUUCCUCCGGGGGACA | 21 | 12837 |
| HSV1-UL30-3898 | + | GCCGACUUUCCUCCGGGGGACA | 22 | 12838 |
| HSV1-UL30-3899 | + | CGCCGACUUUCCUCCGGGGGACA | 23 | 12839 |
| HSV1-UL30-3900 | + | CCGCCGACUUUCCUCCGGGGGACA | 24 | 12840 |
| HSV1-UL30-3901 | + | GACAGCGGGCCGCCGCCA | 18 | 12841 |
| HSV1-UL30-3902 | + | GGACAGCGGGCCGCCGCCA | 19 | 12842 |
| HSV1-UL30-372 | + | GGGACAGCGGGCCGCCGCCA | 20 | 4793 |
| HSV1-UL30-3903 | + | GGGGACAGCGGGCCGCCGCCA | 21 | 12843 |
| HSV1-UL30-3904 | + | GGGGGACAGCGGGCCGCCGCCA | 22 | 12844 |
| HSV1-UL30-3905 | + | CGGGGGACAGCGGGCCGCCGCCA | 23 | 12845 |
| HSV1-UL30-3906 | + | CCGGGGGACAGCGGGCCGCCGCCA | 24 | 12846 |
| HSV1-UL30-3907 | + | GCUUCUGUUGCGUCCCGA | 18 | 12847 |
| HSV1-UL30-3908 | + | GGCUUCUGUUGCGUCCCGA | 19 | 12848 |
| HSV1-UL30-355 | + | CGGCUUCUGUUGCGUCCCGA | 20 | 4783 |
| HSV1-UL30-3909 | + | UCGGCUUCUGUUGCGUCCCGA | 21 | 12849 |
| HSV1-UL30-3910 | + | GUCGGCUUCUGUUGCGUCCCGA | 22 | 12850 |
| HSV1-UL30-3911 | + | GGUCGGCUUCUGUUGCGUCCCGA | 23 | 12851 |
| HSV1-UL30-3912 | + | CGGUCGGCUUCUGUUGCGUCCCGA | 24 | 12852 |
| HSV1-UL30-3913 | + | CCGGCGGGCGCAAAAAAC | 18 | 12853 |
| HSV1-UL30-3914 | + | GCCGGCGGGCGCAAAAAAC | 19 | 12854 |
| HSV1-UL30-366 | + | GGCCGGCGGGCGCAAAAAAC | 20 | 4790 |
| HSV1-UL30-3915 | + | GGGCCGGCGGGCGCAAAAAAC | 21 | 12855 |
| HSV1-UL30-3916 | + | AGGGCCGGCGGGCGCAAAAAAC | 22 | 12856 |
| HSV1-UL30-3917 | + | GAGGGCCGGCGGGCGCAAAAAAC | 23 | 12857 |
| HSV1-UL30-3918 | + | CGAGGGCCGGCGGGCGCAAAAAAC | 24 | 12858 |
| HSV1-UL30-3919 | + | CUCCAGGAUGUCGUACAC | 18 | 12859 |
| HSV1-UL30-3920 | + | UCUCCAGGAUGUCGUACAC | 19 | 12860 |
| HSV1-UL30-330 | + | UUCUCCAGGAUGUCGUACAC | 20 | 4768 |
| HSV1-UL30-3921 | + | GUUCUCCAGGAUGUCGUACAC | 21 | 12861 |
| HSV1-UL30-3922 | + | CGUUCUCCAGGAUGUCGUACAC | 22 | 12862 |
| HSV1-UL30-3923 | + | ACGUUCUCCAGGAUGUCGUACAC | 23 | 12863 |
| HSV1-UL30-3924 | + | CACGUUCUCCAGGAUGUCGUACAC | 24 | 12864 |
| HSV1-UL30-3925 | + | CAUCGCGGUUGUUCCCAC | 18 | 12865 |
| HSV1-UL30-3926 | + | ACAUCGCGGUUGUUCCCAC | 19 | 12866 |
| HSV1-UL30-3927 | + | AACAUCGCGGUUGUUCCCAC | 20 | 12867 |
| HSV1-UL30-3928 | + | AAACAUCGCGGUUGUUCCCAC | 21 | 12868 |
| HSV1-UL30-3929 | + | AAAACAUCGCGGUUGUUCCCAC | 22 | 12869 |
| HSV1-UL30-3930 | + | GAAAACAUCGCGGUUGUUCCCAC | 23 | 12870 |
| HSV1-UL30-3931 | + | GGAAAACAUCGCGGUUGUUCCCAC | 24 | 12871 |
| HSV1-UL30-3932 | + | ACAGCGGGCCGCCGCCAC | 18 | 12872 |
| HSV1-UL30-3933 | + | GACAGCGGGCCGCCGCCAC | 19 | 12873 |
| HSV1-UL30-118 | + | GGACAGCGGGCCGCCGCCAC | 20 | 3294 |
| HSV1-UL30-3934 | + | GGGACAGCGGGCCGCCGCCAC | 21 | 12874 |
| HSV1-UL30-3935 | + | GGGGACAGCGGGCCGCCGCCAC | 22 | 12875 |
| HSV1-UL30-3936 | + | GGGGGACAGCGGGCCGCCGCCAC | 23 | 12876 |
| HSV1-UL30-3937 | + | CGGGGGACAGCGGGCCGCCGCCAC | 24 | 12877 |
| HSV1-UL30-3938 | + | GAAGCCGCCCGACCCGAC | 18 | 12878 |
| HSV1-UL30-3939 | + | AGAAGCCGCCCGACCCGAC | 19 | 12879 |
| HSV1-UL30-338 | + | CAGAAGCCGCCCGACCCGAC | 20 | 4774 |
| HSV1-UL30-3940 | + | CCAGAAGCCGCCCGACCCGAC | 21 | 12880 |
| HSV1-UL30-3941 | + | GCCAGAAGCCGCCCGACCCGAC | 22 | 12881 |
| HSV1-UL30-3942 | + | GGCCAGAAGCCGCCCGACCCGAC | 23 | 12882 |
| HSV1-UL30-3943 | + | CGGCCAGAAGCCGCCCGACCCGAC | 24 | 12883 |
| HSV1-UL30-3944 | + | CUUCUGUUGCGUCCCGAC | 18 | 12884 |
| HSV1-UL30-3945 | + | GCUUCUGUUGCGUCCCGAC | 19 | 12885 |
| HSV1-UL30-91 | + | GGCUUCUGUUGCGUCCCGAC | 20 | 3166 |
| HSV1-UL30-3946 | + | CGGCUUCUGUUGCGUCCCGAC | 21 | 12886 |
| HSV1-UL30-3947 | + | UCGGCUUCUGUUGCGUCCCGAC | 22 | 12887 |
| HSV1-UL30-3948 | + | GUCGGCUUCUGUUGCGUCCCGAC | 23 | 12888 |
| HSV1-UL30-3949 | + | GGUCGGCUUCUGUUGCGUCCCGAC | 24 | 12889 |
| HSV1-UL30-3950 | + | UCCCCCCCGCAGUACACC | 18 | 12890 |
| HSV1-UL30-3951 | + | GUCCCCCCCGCAGUACACC | 19 | 12891 |
| HSV1-UL30-341 | + | CGUCCCCCCCGCAGUACACC | 20 | 4776 |
| HSV1-UL30-3952 | + | UCGUCCCCCCCGCAGUACACC | 21 | 12892 |
| HSV1-UL30-3953 | + | CUCGUCCCCCCCGCAGUACACC | 22 | 12893 |
| HSV1-UL30-3954 | + | GCUCGUCCCCCCCGCAGUACACC | 23 | 12894 |
| HSV1-UL30-3955 | + | CGCUCGUCCCCCCCGCAGUACACC | 24 | 12895 |
| HSV1-UL30-3956 | + | CAUCCUCGUCCAGCACCC | 18 | 12896 |
| HSV1-UL30-3957 | + | GCAUCCUCGUCCAGCACCC | 19 | 12897 |
| HSV1-UL30-350 | + | GGCAUCCUCGUCCAGCACCC | 20 | 4779 |
| HSV1-UL30-3958 | + | GGGCAUCCUCGUCCAGCACCC | 21 | 12898 |
| HSV1-UL30-3959 | + | GGGGCAUCCUCGUCCAGCACCC | 22 | 12899 |
| HSV1-UL30-3960 | + | GGGGGCAUCCUCGUCCAGCACCC | 23 | 12900 |
| HSV1-UL30-3961 | + | GGGGGGCAUCCUCGUCCAGCACCC | 24 | 12901 |
| HSV1-UL30-3962 | + | GGCCGCCGACUUUCCUCC | 18 | 12902 |
| HSV1-UL30-3963 | + | UGGCCGCCGACUUUCCUCC | 19 | 12903 |
| HSV1-UL30-113 | + | CUGGCCGCCGACUUUCCUCC | 20 | 3291 |
| HSV1-UL30-3964 | + | CCUGGCCGCCGACUUUCCUCC | 21 | 12904 |
| HSV1-UL30-3965 | + | CCCUGGCCGCCGACUUUCCUCC | 22 | 12905 |
| HSV1-UL30-3966 | + | GCCCUGGCCGCCGACUUUCCUCC | 23 | 12906 |
| HSV1-UL30-3967 | + | CGCCCUGGCCGCCGACUUUCCUCC | 24 | 12907 |
| HSV1-UL30-3968 | + | GGGUCCCCGGCUGGCUCC | 18 | 12908 |
| HSV1-UL30-3969 | + | GGGGUCCCCGGCUGGCUCC | 19 | 12909 |
| HSV1-UL30-363 | + | GGGGGUCCCCGGCUGGCUCC | 20 | 4787 |
| HSV1-UL30-3970 | + | CGGGGGUCCCCGGCUGGCUCC | 21 | 12910 |
| HSV1-UL30-3971 | + | GCGGGGGUCCCCGGCUGGCUCC | 22 | 12911 |
| HSV1-UL30-3972 | + | GGCGGGGGUCCCCGGCUGGCUCC | 23 | 12912 |
| HSV1-UL30-3973 | + | AGGCGGGGGUCCCCGGCUGGCUCC | 24 | 12913 |
| HSV1-UL30-3974 | + | ACCCCGGCGCGCUUCUCC | 18 | 12914 |
| HSV1-UL30-3975 | + | CACCCCGGCGCGCUUCUCC | 19 | 12915 |
| HSV1-UL30-77 | + | GCACCCCGGCGCGCUUCUCC | 20 | 3271 |
| HSV1-UL30-3976 | + | UGCACCCCGGCGCGCUUCUCC | 21 | 12916 |
| HSV1-UL30-3977 | + | GUGCACCCCGGCGCGCUUCUCC | 22 | 12917 |
| HSV1-UL30-3978 | + | CGUGCACCCCGGCGCGCUUCUCC | 23 | 12918 |
| HSV1-UL30-3979 | + | UCGUGCACCCCGGCGCGCUUCUCC | 24 | 12919 |
| HSV1-UL30-3980 | + | GGUGGGGUUGAACCCCGC | 18 | 12920 |
| HSV1-UL30-3981 | + | CGGUGGGGUUGAACCCCGC | 19 | 12921 |
| HSV1-UL30-62 | + | ACGGUGGGGUUGAACCCCGC | 20 | 3152 |
| HSV1-UL30-3982 | + | GACGGUGGGGUUGAACCCCGC | 21 | 12922 |
| HSV1-UL30-3983 | + | UGACGGUGGGGUUGAACCCCGC | 22 | 12923 |
| HSV1-UL30-3984 | + | GUGACGGUGGGGUUGAACCCCGC | 23 | 12924 |
| HSV1-UL30-3985 | + | GGUGACGGUGGGGUUGAACCCCGC | 24 | 12925 |
| HSV1-UL30-3986 | + | GUCCCCGGCUGGCUCCGC | 18 | 12926 |
| HSV1-UL30-3987 | + | GGUCCCCGGCUGGCUCCGC | 19 | 12927 |
| HSV1-UL30-364 | + | GGGUCCCCGGCUGGCUCCGC | 20 | 4788 |
| HSV1-UL30-3988 | + | GGGGUCCCCGGCUGGCUCCGC | 21 | 12928 |
| HSV1-UL30-3989 | + | GGGGGUCCCCGGCUGGCUCCGC | 22 | 12929 |
| HSV1-UL30-3990 | + | CGGGGGUCCCCGGCUGGCUCCGC | 23 | 12930 |
| HSV1-UL30-3991 | + | GCGGGGGUCCCCGGCUGGCUCCGC | 24 | 12931 |
| HSV1-UL30-3992 | + | GGCGUCCAUAAACCGCGC | 18 | 12932 |
| HSV1-UL30-3993 | + | UGGCGUCCAUAAACCGCGC | 19 | 12933 |
| HSV1-UL30-3994 | + | AUGGCGUCCAUAAACCGCGC | 20 | 12934 |
| HSV1-UL30-3995 | + | GAUGGCGUCCAUAAACCGCGC | 21 | 12935 |
| HSV1-UL30-3996 | + | UGAUGGCGUCCAUAAACCGCGC | 22 | 12936 |
| HSV1-UL30-3997 | + | GUGAUGGCGUCCAUAAACCGCGC | 23 | 12937 |
| HSV1-UL30-3998 | + | UGUGAUGGCGUCCAUAAACCGCGC | 24 | 12938 |
| HSV1-UL30-3999 | + | UACACCUUGGGGGCGCGC | 18 | 12939 |
| HSV1-UL30-4000 | + | GUACACCUUGGGGGCGCGC | 19 | 12940 |
| HSV1-UL30-344 | + | AGUACACCUUGGGGGCGCGC | 20 | 4777 |
| HSV1-UL30-4001 | + | CAGUACACCUUGGGGGCGCGC | 21 | 12941 |
| HSV1-UL30-4002 | + | GCAGUACACCUUGGGGGCGCGC | 22 | 12942 |
| HSV1-UL30-4003 | + | CGCAGUACACCUUGGGGGCGCGC | 23 | 12943 |
| HSV1-UL30-4004 | + | CCGCAGUACACCUUGGGGGCGCGC | 24 | 12944 |
| HSV1-UL30-4005 | + | CCACGCCGCCCCACAGGC | 18 | 12945 |
| HSV1-UL30-4006 | + | UCCACGCCGCCCCACAGGC | 19 | 12946 |
| HSV1-UL30-336 | + | GUCCACGCCGCCCCACAGGC | 20 | 4772 |
| HSV1-UL30-4007 | + | GGUCCACGCCGCCCCACAGGC | 21 | 12947 |
| HSV1-UL30-4008 | + | UGGUCCACGCCGCCCCACAGGC | 22 | 12948 |
| HSV1-UL30-4009 | + | GUGGUCCACGCCGCCCCACAGGC | 23 | 12949 |
| HSV1-UL30-4010 | + | CGUGGUCCACGCCGCCCCACAGGC | 24 | 12950 |
| HSV1-UL30-4011 | + | AGGCGCGAGCGCCGCGGC | 18 | 12951 |
| HSV1-UL30-4012 | + | CAGGCGCGAGCGCCGCGGC | 19 | 12952 |
| HSV1-UL30-337 | + | ACAGGCGCGAGCGCCGCGGC | 20 | 4773 |
| HSV1-UL30-4013 | + | CACAGGCGCGAGCGCCGCGGC | 21 | 12953 |
| HSV1-UL30-4014 | + | CCACAGGCGCGAGCGCCGCGGC | 22 | 12954 |
| HSV1-UL30-4015 | + | CCCACAGGCGCGAGCGCCGCGGC | 23 | 12955 |
| HSV1-UL30-4016 | + | CCCCACAGGCGCGAGCGCCGCGGC | 24 | 12956 |
| HSV1-UL30-4017 | + | UGGCCGCCGACUUUCCUC | 18 | 12957 |
| HSV1-UL30-4018 | + | CUGGCCGCCGACUUUCCUC | 19 | 12958 |
| HSV1-UL30-112 | + | CCUGGCCGCCGACUUUCCUC | 20 | 3290 |
| HSV1-UL30-4019 | + | CCCUGGCCGCCGACUUUCCUC | 21 | 12959 |
| HSV1-UL30-4020 | + | GCCCUGGCCGCCGACUUUCCUC | 22 | 12960 |
| HSV1-UL30-4021 | + | CGCCCUGGCCGCCGACUUUCCUC | 23 | 12961 |
| HSV1-UL30-4022 | + | CCGCCCUGGCCGCCGACUUUCCUC | 24 | 12962 |
| HSV1-UL30-4023 | + | CACCCCGGCGCGCUUCUC | 18 | 12963 |
| HSV1-UL30-4024 | + | GCACCCCGGCGCGCUUCUC | 19 | 12964 |
| HSV1-UL30-76 | + | UGCACCCCGGCGCGCUUCUC | 20 | 3270 |
| HSV1-UL30-4025 | + | GUGCACCCCGGCGCGCUUCUC | 21 | 12965 |
| HSV1-UL30-4026 | + | CGUGCACCCCGGCGCGCUUCUC | 22 | 12966 |
| HSV1-UL30-4027 | + | UCGUGCACCCCGGCGCGCUUCUC | 23 | 12967 |
| HSV1-UL30-4028 | + | GUCGUGCACCCCGGCGCGCUUCUC | 24 | 12968 |
| HSV1-UL30-4029 | + | AGUUUUGCCUCAAACAAG | 18 | 12969 |
| HSV1-UL30-4030 | + | AAGUUUUGCCUCAAACAAG | 19 | 12970 |
| HSV1-UL30-360 | + | AAAGUUUUGCCUCAAACAAG | 20 | 4786 |
| HSV1-UL30-4031 | + | AAAAGUUUUGCCUCAAACAAG | 21 | 12971 |
| HSV1-UL30-4032 | + | UAAAAGUUUUGCCUCAAACAAG | 22 | 12972 |
| HSV1-UL30-4033 | + | GUAAAAGUUUUGCCUCAAACAAG | 23 | 12973 |
| HSV1-UL30-4034 | + | UGUAAAAGUUUUGCCUCAAACAAG | 24 | 12974 |
| HSV1-UL30-4035 | + | AAGCCGCCCGACCCGACG | 18 | 12975 |
| HSV1-UL30-4036 | + | GAAGCCGCCCGACCCGACG | 19 | 12976 |
| HSV1-UL30-68 | + | AGAAGCCGCCCGACCCGACG | 20 | 3155 |
| HSV1-UL30-4037 | + | CAGAAGCCGCCCGACCCGACG | 21 | 12977 |
| HSV1-UL30-4038 | + | CCAGAAGCCGCCCGACCCGACG | 22 | 12978 |
| HSV1-UL30-4039 | + | GCCAGAAGCCGCCCGACCCGACG | 23 | 12979 |
| HSV1-UL30-4040 | + | GGCCAGAAGCCGCCCGACCCGACG | 24 | 12980 |
| HSV1-UL30-4041 | + | ACGUGAAAGACGGUGACG | 18 | 12981 |
| HSV1-UL30-4042 | + | CACGUGAAAGACGGUGACG | 19 | 12982 |
| HSV1-UL30-331 | + | ACACGUGAAAGACGGUGACG | 20 | 4769 |
| HSV1-UL30-4043 | + | UACACGUGAAAGACGGUGACG | 21 | 12983 |
| HSV1-UL30-4044 | + | GUACACGUGAAAGACGGUGACG | 22 | 12984 |
| HSV1-UL30-4045 | + | CGUACACGUGAAAGACGGUGACG | 23 | 12985 |
| HSV1-UL30-4046 | + | UCGUACACGUGAAAGACGGUGACG | 24 | 12986 |
| HSV1-UL30-4047 | + | AUCCUCGUCCAGCACCCG | 18 | 12987 |
| HSV1-UL30-4048 | + | CAUCCUCGUCCAGCACCCG | 19 | 12988 |
| HSV1-UL30-82 | + | GCAUCCUCGUCCAGCACCCG | 20 | 3161 |
| HSV1-UL30-4049 | + | GGCAUCCUCGUCCAGCACCCG | 21 | 12989 |
| HSV1-UL30-4050 | + | GGGCAUCCUCGUCCAGCACCCG | 22 | 12990 |
| HSV1-UL30-4051 | + | GGGGCAUCCUCGUCCAGCACCCG | 23 | 12991 |
| HSV1-UL30-4052 | + | GGGGGCAUCCUCGUCCAGCACCCG | 24 | 12992 |
| HSV1-UL30-4053 | + | CGGUGGGGUUGAACCCCG | 18 | 12993 |
| HSV1-UL30-4054 | + | ACGGUGGGGUUGAACCCCG | 19 | 12994 |
| HSV1-UL30-334 | + | GACGGUGGGGUUGAACCCCG | 20 | 4771 |
| HSV1-UL30-4055 | + | UGACGGUGGGGUUGAACCCCG | 21 | 12995 |
| HSV1-UL30-4056 | + | GUGACGGUGGGGUUGAACCCCG | 22 | 12996 |
| HSV1-UL30-4057 | + | GGUGACGGUGGGGUUGAACCCCG | 23 | 12997 |
| HSV1-UL30-4058 | + | CGGUGACGGUGGGGUUGAACCCCG | 24 | 12998 |
| HSV1-UL30-4059 | + | CUGGCUCCGCGAGGGCCG | 18 | 12999 |
| HSV1-UL30-4060 | + | GCUGGCUCCGCGAGGGCCG | 19 | 13000 |
| HSV1-UL30-365 | + | GGCUGGCUCCGCGAGGGCCG | 20 | 4789 |
| HSV1-UL30-4061 | + | CGGCUGGCUCCGCGAGGGCCG | 21 | 13001 |
| HSV1-UL30-4062 | + | CCGGCUGGCUCCGCGAGGGCCG | 22 | 13002 |
| HSV1-UL30-4063 | + | CCCGGCUGGCUCCGCGAGGGCCG | 23 | 13003 |
| HSV1-UL30-4064 | + | CCCCGGCUGGCUCCGCGAGGGCCG | 24 | 13004 |
| HSV1-UL30-4065 | + | GCCGCCGACUUUCCUCCG | 18 | 13005 |
| HSV1-UL30-4066 | + | GGCCGCCGACUUUCCUCCG | 19 | 13006 |
| HSV1-UL30-114 | + | UGGCCGCCGACUUUCCUCCG | 20 | 3176 |
| HSV1-UL30-4067 | + | CUGGCCGCCGACUUUCCUCCG | 21 | 13007 |
| HSV1-UL30-4068 | + | CCUGGCCGCCGACUUUCCUCCG | 22 | 13008 |
| HSV1-UL30-4069 | + | CCCUGGCCGCCGACUUUCCUCCG | 23 | 13009 |
| HSV1-UL30-4070 | + | GCCCUGGCCGCCGACUUUCCUCCG | 24 | 13010 |
| HSV1-UL30-4071 | + | CCCCGGCGCGCUUCUCCG | 18 | 13011 |
| HSV1-UL30-4072 | + | ACCCCGGCGCGCUUCUCCG | 19 | 13012 |
| HSV1-UL30-78 | + | CACCCCGGCGCGCUUCUCCG | 20 | 3272 |
| HSV1-UL30-4073 | + | GCACCCCGGCGCGCUUCUCCG | 21 | 13013 |
| HSV1-UL30-4074 | + | UGCACCCCGGCGCGCUUCUCCG | 22 | 13014 |
| HSV1-UL30-4075 | + | GUGCACCCCGGCGCGCUUCUCCG | 23 | 13015 |
| HSV1-UL30-4076 | + | CGUGCACCCCGGCGCGCUUCUCCG | 24 | 13016 |
| HSV1-UL30-4077 | + | GCCGCCCGACCCGACGCG | 18 | 13017 |
| HSV1-UL30-4078 | + | AGCCGCCCGACCCGACGCG | 19 | 13018 |
| HSV1-UL30-340 | + | AAGCCGCCCGACCCGACGCG | 20 | 4775 |
| HSV1-UL30-4079 | + | GAAGCCGCCCGACCCGACGCG | 21 | 13019 |
| HSV1-UL30-4080 | + | AGAAGCCGCCCGACCCGACGCG | 22 | 13020 |
| HSV1-UL30-4081 | + | CAGAAGCCGCCCGACCCGACGCG | 23 | 13021 |
| HSV1-UL30-4082 | + | CCAGAAGCCGCCCGACCCGACGCG | 24 | 13022 |
| HSV1-UL30-4083 | + | GCGUCCAUAAACCGCGCG | 18 | 13023 |
| HSV1-UL30-4084 | + | GGCGUCCAUAAACCGCGCG | 19 | 13024 |
| HSV1-UL30-2857 | + | UGGCGUCCAUAAACCGCGCG | 20 | 11797 |
| HSV1-UL30-4085 | + | AUGGCGUCCAUAAACCGCGCG | 21 | 13025 |
| HSV1-UL30-4086 | + | GAUGGCGUCCAUAAACCGCGCG | 22 | 13026 |
| HSV1-UL30-4087 | + | UGAUGGCGUCCAUAAACCGCGCG | 23 | 13027 |
| HSV1-UL30-4088 | + | GUGAUGGCGUCCAUAAACCGCGCG | 24 | 13028 |
| HSV1-UL30-4089 | + | GUUUUGCCUCAAACAAGG | 18 | 13029 |
| HSV1-UL30-4090 | + | AGUUUUGCCUCAAACAAGG | 19 | 13030 |
| HSV1-UL30-99 | + | AAGUUUUGCCUCAAACAAGG | 20 | 3282 |
| HSV1-UL30-4091 | + | AAAGUUUUGCCUCAAACAAGG | 21 | 13031 |
| HSV1-UL30-4092 | + | AAAAGUUUUGCCUCAAACAAGG | 22 | 13032 |
| HSV1-UL30-4093 | + | UAAAAGUUUUGCCUCAAACAAGG | 23 | 13033 |
| HSV1-UL30-4094 | + | GUAAAAGUUUUGCCUCAAACAAGG | 24 | 13034 |
| HSV1-UL30-4095 | + | GUCCCGACUGGGGCGAGG | 18 | 13035 |
| HSV1-UL30-4096 | + | CGUCCCGACUGGGGCGAGG | 19 | 13036 |
| HSV1-UL30-358 | + | GCGUCCCGACUGGGGCGAGG | 20 | 4785 |
| HSV1-UL30-4097 | + | UGCGUCCCGACUGGGGCGAGG | 21 | 13037 |
| HSV1-UL30-4098 | + | UUGCGUCCCGACUGGGGCGAGG | 22 | 13038 |
| HSV1-UL30-4099 | + | GUUGCGUCCCGACUGGGGCGAGG | 23 | 13039 |
| HSV1-UL30-4100 | + | UGUUGCGUCCCGACUGGGGCGAGG | 24 | 13040 |
| HSV1-UL30-4101 | + | CCCGGCGCGCUUCUCCGG | 18 | 13041 |
| HSV1-UL30-4102 | + | CCCCGGCGCGCUUCUCCGG | 19 | 13042 |
| HSV1-UL30-79 | + | ACCCCGGCGCGCUUCUCCGG | 20 | 3273 |
| HSV1-UL30-4103 | + | CACCCCGGCGCGCUUCUCCGG | 21 | 13043 |
| HSV1-UL30-4104 | + | GCACCCCGGCGCGCUUCUCCGG | 22 | 13044 |
| HSV1-UL30-4105 | + | UGCACCCCGGCGCGCUUCUCCGG | 23 | 13045 |
| HSV1-UL30-4106 | + | GUGCACCCCGGCGCGCUUCUCCGG | 24 | 13046 |
| HSV1-UL30-4107 | + | AAGACGGUGACGGUGGGG | 18 | 13047 |
| HSV1-UL30-4108 | + | AAAGACGGUGACGGUGGGG | 19 | 13048 |
| HSV1-UL30-333 | + | GAAAGACGGUGACGGUGGGG | 20 | 4770 |
| HSV1-UL30-4109 | + | UGAAAGACGGUGACGGUGGGG | 21 | 13049 |
| HSV1-UL30-4110 | + | GUGAAAGACGGUGACGGUGGGG | 22 | 13050 |
| HSV1-UL30-4111 | + | CGUGAAAGACGGUGACGGUGGGG | 23 | 13051 |
| HSV1-UL30-4112 | + | ACGUGAAAGACGGUGACGGUGGGG | 24 | 13052 |
| HSV1-UL30-4113 | + | UGUUGCGUCCCGACUGGG | 18 | 13053 |
| HSV1-UL30-4114 | + | CUGUUGCGUCCCGACUGGG | 19 | 13054 |
| HSV1-UL30-357 | + | UCUGUUGCGUCCCGACUGGG | 20 | 4784 |
| HSV1-UL30-4115 | + | UUCUGUUGCGUCCCGACUGGG | 21 | 13055 |
| HSV1-UL30-4116 | + | CUUCUGUUGCGUCCCGACUGGG | 22 | 13056 |
| HSV1-UL30-4117 | + | GCUUCUGUUGCGUCCCGACUGGG | 23 | 13057 |
| HSV1-UL30-4118 | + | GGCUUCUGUUGCGUCCCGACUGGG | 24 | 13058 |
| HSV1-UL30-4119 | + | CCCCCCCGCAGUACACCU | 18 | 13059 |
| HSV1-UL30-4120 | + | UCCCCCCCGCAGUACACCU | 19 | 13060 |
| HSV1-UL30-70 | + | GUCCCCCCCGCAGUACACCU | 20 | 3157 |
| HSV1-UL30-4121 | + | CGUCCCCCCCGCAGUACACCU | 21 | 13061 |
| HSV1-UL30-4122 | + | UCGUCCCCCCCGCAGUACACCU | 22 | 13062 |
| HSV1-UL30-4123 | + | CUCGUCCCCCCCGCAGUACACCU | 23 | 13063 |
| HSV1-UL30-4124 | + | GCUCGUCCCCCCCGCAGUACACCU | 24 | 13064 |
| HSV1-UL30-4125 | + | CUGGCCGCCGACUUUCCU | 18 | 13065 |
| HSV1-UL30-4126 | + | CCUGGCCGCCGACUUUCCU | 19 | 13066 |
| HSV1-UL30-367 | + | CCCUGGCCGCCGACUUUCCU | 20 | 4791 |
| HSV1-UL30-4127 | + | GCCCUGGCCGCCGACUUUCCU | 21 | 13067 |
| HSV1-UL30-4128 | + | CGCCCUGGCCGCCGACUUUCCU | 22 | 13068 |
| HSV1-UL30-4129 | + | CCGCCCUGGCCGCCGACUUUCCU | 23 | 13069 |
| HSV1-UL30-4130 | + | GCCGCCCUGGCCGCCGACUUUCCU | 24 | 13070 |
| HSV1-UL30-4131 | + | GCACCCCGGCGCGCUUCU | 18 | 13071 |
| HSV1-UL30-4132 | + | UGCACCCCGGCGCGCUUCU | 19 | 13072 |
| HSV1-UL30-345 | + | GUGCACCCCGGCGCGCUUCU | 20 | 4778 |
| HSV1-UL30-4133 | + | CGUGCACCCCGGCGCGCUUCU | 21 | 13073 |
| HSV1-UL30-4134 | + | UCGUGCACCCCGGCGCGCUUCU | 22 | 13074 |
| HSV1-UL30-4135 | + | GUCGUGCACCCCGGCGCGCUUCU | 23 | 13075 |
| HSV1-UL30-4136 | + | CGUCGUGCACCCCGGCGCGCUUCU | 24 | 13076 |
| HSV1-UL30-4137 | + | CCCCCCGCAGUACACCUU | 18 | 13077 |
| HSV1-UL30-4138 | + | CCCCCCCGCAGUACACCUU | 19 | 13078 |
| HSV1-UL30-71 | + | UCCCCCCCGCAGUACACCUU | 20 | 3267 |
| HSV1-UL30-4139 | + | GUCCCCCCCGCAGUACACCUU | 21 | 13079 |
| HSV1-UL30-4140 | + | CGUCCCCCCCGCAGUACACCUU | 22 | 13080 |
| HSV1-UL30-4141 | + | UCGUCCCCCCCGCAGUACACCUU | 23 | 13081 |
| HSV1-UL30-4142 | + | CUCGUCCCCCCCGCAGUACACCUU | 24 | 13082 |
| HSV1-UL30-4143 | - | GCCCCAGUCGGGACGCAA | 18 | 13083 |
| HSV1-UL30-4144 | - | CGCCCCAGUCGGGACGCAA | 19 | 13084 |
| HSV1-UL30-251 | - | UCGCCCCAGUCGGGACGCAA | 20 | 4717 |
| HSV1-UL30-4145 | - | CUCGCCCCAGUCGGGACGCAA | 21 | 13085 |
| HSV1-UL30-4146 | - | CCUCGCCCCAGUCGGGACGCAA | 22 | 13086 |
| HSV1-UL30-4147 | - | ACCUCGCCCCAGUCGGGACGCAA | 23 | 13087 |
| HSV1-UL30-4148 | - | UACCUCGCCCCAGUCGGGACGCAA | 24 | 13088 |
| HSV1-UL30-4149 | - | GGACGCAACAGAAGCCGA | 18 | 13089 |
| HSV1-UL30-4150 | - | GGGACGCAACAGAAGCCGA | 19 | 13090 |
| HSV1-UL30-252 | - | CGGGACGCAACAGAAGCCGA | 20 | 4718 |
| HSV1-UL30-4151 | - | UCGGGACGCAACAGAAGCCGA | 21 | 13091 |
| HSV1-UL30-4152 | - | GUCGGGACGCAACAGAAGCCGA | 22 | 13092 |
| HSV1-UL30-4153 | - | AGUCGGGACGCAACAGAAGCCGA | 23 | 13093 |
| HSV1-UL30-4154 | - | CAGUCGGGACGCAACAGAAGCCGA | 24 | 13094 |
| HSV1-UL30-4155 | - | UACGACAUCCUGGAGAAC | 18 | 13095 |
| HSV1-UL30-4156 | - | GUACGACAUCCUGGAGAAC | 19 | 13096 |
| HSV1-UL30-281 | - | UGUACGACAUCCUGGAGAAC | 20 | 4737 |
| HSV1-UL30-4157 | - | GUGUACGACAUCCUGGAGAAC | 21 | 13097 |
| HSV1-UL30-4158 | - | CGUGUACGACAUCCUGGAGAAC | 22 | 13098 |
| HSV1-UL30-4159 | - | ACGUGUACGACAUCCUGGAGAAC | 23 | 13099 |
| HSV1-UL30-4160 | - | CACGUGUACGACAUCCUGGAGAAC | 24 | 13100 |
| HSV1-UL30-4161 | - | UGGACGAGGAUGCCCCCC | 18 | 13101 |
| HSV1-UL30-4162 | - | CUGGACGAGGAUGCCCCCC | 19 | 13102 |
| HSV1-UL30-24 | - | GCUGGACGAGGAUGCCCCCC | 20 | 3247 |
| HSV1-UL30-4163 | - | UGCUGGACGAGGAUGCCCCCC | 21 | 13103 |
| HSV1-UL30-4164 | - | GUGCUGGACGAGGAUGCCCCCC | 22 | 13104 |
| HSV1-UL30-4165 | - | GGUGCUGGACGAGGAUGCCCCCC | 23 | 13105 |
| HSV1-UL30-4166 | - | GGGUGCUGGACGAGGAUGCCCCCC | 24 | 13106 |
| HSV1-UL30-4167 | - | CUGGACGAGGAUGCCCCC | 18 | 13107 |
| HSV1-UL30-4168 | - | GCUGGACGAGGAUGCCCCC | 19 | 13108 |
| HSV1-UL30-259 | - | UGCUGGACGAGGAUGCCCCC | 20 | 4725 |
| HSV1-UL30-4169 | - | GUGCUGGACGAGGAUGCCCCC | 21 | 13109 |
| HSV1-UL30-4170 | - | GGUGCUGGACGAGGAUGCCCCC | 22 | 13110 |
| HSV1-UL30-4171 | - | GGGUGCUGGACGAGGAUGCCCCC | 23 | 13111 |
| HSV1-UL30-4172 | - | CGGGUGCUGGACGAGGAUGCCCCC | 24 | 13112 |
| HSV1-UL30-4173 | - | CGGCGGCCCGCUGUCCCC | 18 | 13113 |
| HSV1-UL30-4174 | - | GCGGCGGCCCGCUGUCCCC | 19 | 13114 |
| HSV1-UL30-1 | - | GGCGGCGGCCCGCUGUCCCC | 20 | 3232 |
| HSV1-UL30-4175 | - | UGGCGGCGGCCCGCUGUCCCC | 21 | 13115 |
| HSV1-UL30-4176 | - | GUGGCGGCGGCCCGCUGUCCCC | 22 | 13116 |
| HSV1-UL30-4177 | - | GGUGGCGGCGGCCCGCUGUCCCC | 23 | 13117 |
| HSV1-UL30-4178 | - | CGGUGGCGGCGGCCCGCUGUCCCC | 24 | 13118 |
| HSV1-UL30-4179 | - | GCGGCGGCCCGCUGUCCC | 18 | 13119 |
| HSV1-UL30-4180 | - | GGCGGCGGCCCGCUGUCCC | 19 | 13120 |
| HSV1-UL30-237 | - | UGGCGGCGGCCCGCUGUCCC | 20 | 4709 |
| HSV1-UL30-4181 | - | GUGGCGGCGGCCCGCUGUCCC | 21 | 13121 |
| HSV1-UL30-4182 | - | GGUGGCGGCGGCCCGCUGUCCC | 22 | 13122 |
| HSV1-UL30-4183 | - | CGGUGGCGGCGGCCCGCUGUCCC | 23 | 13123 |
| HSV1-UL30-4184 | - | CCGGUGGCGGCGGCCCGCUGUCCC | 24 | 13124 |
| HSV1-UL30-4185 | - | GCCCUCGCGGAGCCAGCC | 18 | 13125 |
| HSV1-UL30-4186 | - | GGCCCUCGCGGAGCCAGCC | 19 | 13126 |
| HSV1-UL30-13 | - | CGGCCCUCGCGGAGCCAGCC | 20 | 3241 |
| HSV1-UL30-4187 | - | CCGGCCCUCGCGGAGCCAGCC | 21 | 13127 |
| HSV1-UL30-4188 | - | GCCGGCCCUCGCGGAGCCAGCC | 22 | 13128 |
| HSV1-UL30-4189 | - | CGCCGGCCCUCGCGGAGCCAGCC | 23 | 13129 |
| HSV1-UL30-4190 | - | CCGCCGGCCCUCGCGGAGCCAGCC | 24 | 13130 |
| HSV1-UL30-4191 | - | UUCACGUGUACGACAUCC | 18 | 13131 |
| HSV1-UL30-4192 | - | UUUCACGUGUACGACAUCC | 19 | 13132 |
| HSV1-UL30-52 | - | CUUUCACGUGUACGACAUCC | 20 | 3146 |
| HSV1-UL30-4193 | - | UCUUUCACGUGUACGACAUCC | 21 | 13133 |
| HSV1-UL30-4194 | - | GUCUUUCACGUGUACGACAUCC | 22 | 13134 |
| HSV1-UL30-4195 | - | CGUCUUUCACGUGUACGACAUCC | 23 | 13135 |
| HSV1-UL30-4196 | - | CCGUCUUUCACGUGUACGACAUCC | 24 | 13136 |
| HSV1-UL30-4197 | - | GGCCCUCGCGGAGCCAGC | 18 | 13137 |
| HSV1-UL30-4198 | - | CGGCCCUCGCGGAGCCAGC | 19 | 13138 |
| HSV1-UL30-12 | - | CCGGCCCUCGCGGAGCCAGC | 20 | 3240 |
| HSV1-UL30-4199 | - | GCCGGCCCUCGCGGAGCCAGC | 21 | 13139 |
| HSV1-UL30-4200 | - | CGCCGGCCCUCGCGGAGCCAGC | 22 | 13140 |
| HSV1-UL30-4201 | - | CCGCCGGCCCUCGCGGAGCCAGC | 23 | 13141 |
| HSV1-UL30-4202 | - | CCCGCCGGCCCUCGCGGAGCCAGC | 24 | 13142 |
| HSV1-UL30-4203 | - | CGGAGGAAAGUCGGCGGC | 18 | 13143 |
| HSV1-UL30-4204 | - | CCGGAGGAAAGUCGGCGGC | 19 | 13144 |
| HSV1-UL30-241 | - | CCCGGAGGAAAGUCGGCGGC | 20 | 4711 |
| HSV1-UL30-4205 | - | CCCCGGAGGAAAGUCGGCGGC | 21 | 13145 |
| HSV1-UL30-4206 | - | CCCCCGGAGGAAAGUCGGCGGC | 22 | 13146 |
| HSV1-UL30-4207 | - | UCCCCCGGAGGAAAGUCGGCGGC | 23 | 13147 |
| HSV1-UL30-4208 | - | GUCCCCCGGAGGAAAGUCGGCGGC | 24 | 13148 |
| HSV1-UL30-4209 | - | UCGCGCCUGUGGGGCGGC | 18 | 13149 |
| HSV1-UL30-4210 | - | CUCGCGCCUGUGGGGCGGC | 19 | 13150 |
| HSV1-UL30-275 | - | GCUCGCGCCUGUGGGGCGGC | 20 | 4733 |
| HSV1-UL30-4211 | - | CGCUCGCGCCUGUGGGGCGGC | 21 | 13151 |
| HSV1-UL30-4212 | - | GCGCUCGCGCCUGUGGGGCGGC | 22 | 13152 |
| HSV1-UL30-4213 | - | GGCGCUCGCGCCUGUGGGGCGGC | 23 | 13153 |
| HSV1-UL30-4214 | - | CGGCGCUCGCGCCUGUGGGGCGGC | 24 | 13154 |
| HSV1-UL30-4215 | - | GCCCCCAAGGUGUACUGC | 18 | 13155 |
| HSV1-UL30-4216 | - | CGCCCCCAAGGUGUACUGC | 19 | 13156 |
| HSV1-UL30-31 | - | GCGCCCCCAAGGUGUACUGC | 20 | 3136 |
| HSV1-UL30-4217 | - | CGCGCCCCCAAGGUGUACUGC | 21 | 13157 |
| HSV1-UL30-4218 | - | GCGCGCCCCCAAGGUGUACUGC | 22 | 13158 |
| HSV1-UL30-4219 | - | AGCGCGCCCCCAAGGUGUACUGC | 23 | 13159 |
| HSV1-UL30-4220 | - | AAGCGCGCCCCCAAGGUGUACUGC | 24 | 13160 |
| HSV1-UL30-4221 | - | UUUCACGUGUACGACAUC | 18 | 13161 |
| HSV1-UL30-4222 | - | CUUUCACGUGUACGACAUC | 19 | 13162 |
| HSV1-UL30-278 | - | UCUUUCACGUGUACGACAUC | 20 | 4735 |
| HSV1-UL30-4223 | - | GUCUUUCACGUGUACGACAUC | 21 | 13163 |
| HSV1-UL30-4224 | - | CGUCUUUCACGUGUACGACAUC | 22 | 13164 |
| HSV1-UL30-4225 | - | CCGUCUUUCACGUGUACGACAUC | 23 | 13165 |
| HSV1-UL30-4226 | - | ACCGUCUUUCACGUGUACGACAUC | 24 | 13166 |
| HSV1-UL30-4227 | - | UUGCGCCCGCCGGCCCUC | 18 | 13167 |
| HSV1-UL30-4228 | - | UUUGCGCCCGCCGGCCCUC | 19 | 13168 |
| HSV1-UL30-243 | - | UUUUGCGCCCGCCGGCCCUC | 20 | 4713 |
| HSV1-UL30-4229 | - | UUUUUGCGCCCGCCGGCCCUC | 21 | 13169 |
| HSV1-UL30-4230 | - | UUUUUUGCGCCCGCCGGCCCUC | 22 | 13170 |
| HSV1-UL30-4231 | - | GUUUUUUGCGCCCGCCGGCCCUC | 23 | 13171 |
| HSV1-UL30-4232 | - | GGUUUUUUGCGCCCGCCGGCCCUC | 24 | 13172 |
| HSV1-UL30-4233 | - | ACCCCUACCUCGCCCCAG | 18 | 13173 |
| HSV1-UL30-4234 | - | AACCCCUACCUCGCCCCAG | 19 | 13174 |
| HSV1-UL30-249 | - | CAACCCCUACCUCGCCCCAG | 20 | 4716 |
| HSV1-UL30-4235 | - | ACAACCCCUACCUCGCCCCAG | 21 | 13175 |
| HSV1-UL30-4236 | - | UACAACCCCUACCUCGCCCCAG | 22 | 13176 |
| HSV1-UL30-4237 | - | UUACAACCCCUACCUCGCCCCAG | 23 | 13177 |
| HSV1-UL30-4238 | - | UUUACAACCCCUACCUCGCCCCAG | 24 | 13178 |
| HSV1-UL30-4239 | - | CGGCCCUCGCGGAGCCAG | 18 | 13179 |
| HSV1-UL30-4240 | - | CCGGCCCUCGCGGAGCCAG | 19 | 13180 |
| HSV1-UL30-245 | - | GCCGGCCCUCGCGGAGCCAG | 20 | 4714 |
| HSV1-UL30-4241 | - | CGCCGGCCCUCGCGGAGCCAG | 21 | 13181 |
| HSV1-UL30-4242 | - | CCGCCGGCCCUCGCGGAGCCAG | 22 | 13182 |
| HSV1-UL30-4243 | - | CCCGCCGGCCCUCGCGGAGCCAG | 23 | 13183 |
| HSV1-UL30-4244 | - | GCCCGCCGGCCCUCGCGGAGCCAG | 24 | 13184 |
| HSV1-UL30-4245 | - | ACGACAUCCUGGAGAACG | 18 | 13185 |
| HSV1-UL30-4246 | - | UACGACAUCCUGGAGAACG | 19 | 13186 |
| HSV1-UL30-53 | - | GUACGACAUCCUGGAGAACG | 20 | 3147 |
| HSV1-UL30-4247 | - | UGUACGACAUCCUGGAGAACG | 21 | 13187 |
| HSV1-UL30-4248 | - | GUGUACGACAUCCUGGAGAACG | 22 | 13188 |
| HSV1-UL30-4249 | - | CGUGUACGACAUCCUGGAGAACG | 23 | 13189 |
| HSV1-UL30-4250 | - | ACGUGUACGACAUCCUGGAGAACG | 24 | 13190 |
| HSV1-UL30-4251 | - | GACGAGGAUGCCCCCCCG | 18 | 13191 |
| HSV1-UL30-4252 | - | GGACGAGGAUGCCCCCCCG | 19 | 13192 |
| HSV1-UL30-261 | - | UGGACGAGGAUGCCCCCCCG | 20 | 4726 |
| HSV1-UL30-4253 | - | CUGGACGAGGAUGCCCCCCCG | 21 | 13193 |
| HSV1-UL30-4254 | - | GCUGGACGAGGAUGCCCCCCCG | 22 | 13194 |
| HSV1-UL30-4255 | - | UGCUGGACGAGGAUGCCCCCCCG | 23 | 13195 |
| HSV1-UL30-4256 | - | GUGCUGGACGAGGAUGCCCCCCCG | 24 | 13196 |
| HSV1-UL30-4257 | - | GCGGCCCGCUGUCCCCCG | 18 | 13197 |
| HSV1-UL30-4258 | - | GGCGGCCCGCUGUCCCCCG | 19 | 13198 |
| HSV1-UL30-239 | - | CGGCGGCCCGCUGUCCCCCG | 20 | 4710 |
| HSV1-UL30-4259 | - | GCGGCGGCCCGCUGUCCCCCG | 21 | 13199 |
| HSV1-UL30-4260 | - | GGCGGCGGCCCGCUGUCCCCCG | 22 | 13200 |
| HSV1-UL30-4261 | - | UGGCGGCGGCCCGCUGUCCCCCG | 23 | 13201 |
| HSV1-UL30-4262 | - | GUGGCGGCGGCCCGCUGUCCCCCG | 24 | 13202 |
| HSV1-UL30-4263 | - | GGCGUGGACCACGCCCCG | 18 | 13203 |
| HSV1-UL30-4264 | - | CGGCGUGGACCACGCCCCG | 19 | 13204 |
| HSV1-UL30-276 | - | GCGGCGUGGACCACGCCCCG | 20 | 4734 |
| HSV1-UL30-4265 | - | GGCGGCGUGGACCACGCCCCG | 21 | 13205 |
| HSV1-UL30-4266 | - | GGGCGGCGUGGACCACGCCCCG | 22 | 13206 |
| HSV1-UL30-4267 | - | GGGGCGGCGUGGACCACGCCCCG | 23 | 13207 |
| HSV1-UL30-4268 | - | UGGGGCGGCGUGGACCACGCCCCG | 24 | 13208 |
| HSV1-UL30-4269 | - | CCCCCCCGGAGAAGCGCG | 18 | 13209 |
| HSV1-UL30-4270 | - | GCCCCCCCGGAGAAGCGCG | 19 | 13210 |
| HSV1-UL30-262 | - | UGCCCCCCCGGAGAAGCGCG | 20 | 4727 |
| HSV1-UL30-4271 | - | AUGCCCCCCCGGAGAAGCGCG | 21 | 13211 |
| HSV1-UL30-4272 | - | GAUGCCCCCCCGGAGAAGCGCG | 22 | 13212 |
| HSV1-UL30-4273 | - | GGAUGCCCCCCCGGAGAAGCGCG | 23 | 13213 |
| HSV1-UL30-4274 | - | AGGAUGCCCCCCCGGAGAAGCGCG | 24 | 13214 |
| HSV1-UL30-4275 | - | CCCCCAAGGUGUACUGCG | 18 | 13215 |
| HSV1-UL30-4276 | - | GCCCCCAAGGUGUACUGCG | 19 | 13216 |
| HSV1-UL30-32 | - | CGCCCCCAAGGUGUACUGCG | 20 | 3137 |
| HSV1-UL30-4277 | - | GCGCCCCCAAGGUGUACUGCG | 21 | 13217 |
| HSV1-UL30-4278 | - | CGCGCCCCCAAGGUGUACUGCG | 22 | 13218 |
| HSV1-UL30-4279 | - | GCGCGCCCCCAAGGUGUACUGCG | 23 | 13219 |
| HSV1-UL30-4280 | - | AGCGCGCCCCCAAGGUGUACUGCG | 24 | 13220 |
| HSV1-UL30-4281 | - | UGCGCCCGCCGGCCCUCG | 18 | 13221 |
| HSV1-UL30-4282 | - | UUGCGCCCGCCGGCCCUCG | 19 | 13222 |
| HSV1-UL30-11 | - | UUUGCGCCCGCCGGCCCUCG | 20 | 3125 |
| HSV1-UL30-4283 | - | UUUUGCGCCCGCCGGCCCUCG | 21 | 13223 |
| HSV1-UL30-4284 | - | UUUUUGCGCCCGCCGGCCCUCG | 22 | 13224 |
| HSV1-UL30-4285 | - | UUUUUUGCGCCCGCCGGCCCUCG | 23 | 13225 |
| HSV1-UL30-4286 | - | GUUUUUUGCGCCCGCCGGCCCUCG | 24 | 13226 |
| HSV1-UL30-4287 | - | CGGCCCGCUGUCCCCCGG | 18 | 13227 |
| HSV1-UL30-4288 | - | GCGGCCCGCUGUCCCCCGG | 19 | 13228 |
| HSV1-UL30-2 | - | GGCGGCCCGCUGUCCCCCGG | 20 | 3233 |
| HSV1-UL30-4289 | - | CGGCGGCCCGCUGUCCCCCGG | 21 | 13229 |
| HSV1-UL30-4290 | - | GCGGCGGCCCGCUGUCCCCCGG | 22 | 13230 |
| HSV1-UL30-4291 | - | GGCGGCGGCCCGCUGUCCCCCGG | 23 | 13231 |
| HSV1-UL30-4292 | - | UGGCGGCGGCCCGCUGUCCCCCGG | 24 | 13232 |
| HSV1-UL30-4293 | - | CCCCAAGGUGUACUGCGG | 18 | 13233 |
| HSV1-UL30-4294 | - | CCCCCAAGGUGUACUGCGG | 19 | 13234 |
| HSV1-UL30-33 | - | GCCCCCAAGGUGUACUGCGG | 20 | 3138 |
| HSV1-UL30-4295 | - | CGCCCCCAAGGUGUACUGCGG | 21 | 13235 |
| HSV1-UL30-4296 | - | GCGCCCCCAAGGUGUACUGCGG | 22 | 13236 |
| HSV1-UL30-4297 | - | CGCGCCCCCAAGGUGUACUGCGG | 23 | 13237 |
| HSV1-UL30-4298 | - | GCGCGCCCCCAAGGUGUACUGCGG | 24 | 13238 |
| HSV1-UL30-4299 | - | CCCAAGGUGUACUGCGGG | 18 | 13239 |
| HSV1-UL30-4300 | - | CCCCAAGGUGUACUGCGGG | 19 | 13240 |
| HSV1-UL30-34 | - | CCCCCAAGGUGUACUGCGGG | 20 | 3249 |
| HSV1-UL30-4301 | - | GCCCCCAAGGUGUACUGCGGG | 21 | 13241 |
| HSV1-UL30-4302 | - | CGCCCCCAAGGUGUACUGCGGG | 22 | 13242 |
| HSV1-UL30-4303 | - | GCGCCCCCAAGGUGUACUGCGGG | 23 | 13243 |
| HSV1-UL30-4304 | - | CGCGCCCCCAAGGUGUACUGCGGG | 24 | 13244 |
| HSV1-UL30-4305 | - | GACGUCCUCCGCGUCGGG | 18 | 13245 |
| HSV1-UL30-4306 | - | CGACGUCCUCCGCGUCGGG | 19 | 13246 |
| HSV1-UL30-272 | - | GCGACGUCCUCCGCGUCGGG | 20 | 4731 |
| HSV1-UL30-4307 | - | CGCGACGUCCUCCGCGUCGGG | 21 | 13247 |
| HSV1-UL30-4308 | - | GCGCGACGUCCUCCGCGUCGGG | 22 | 13248 |
| HSV1-UL30-4309 | - | AGCGCGACGUCCUCCGCGUCGGG | 23 | 13249 |
| HSV1-UL30-4310 | - | GAGCGCGACGUCCUCCGCGUCGGG | 24 | 13250 |
| HSV1-UL30-4311 | - | AGGUGUACUGCGGGGGGG | 18 | 13251 |
| HSV1-UL30-4312 | - | AAGGUGUACUGCGGGGGGG | 19 | 13252 |
| HSV1-UL30-270 | - | CAAGGUGUACUGCGGGGGGG | 20 | 4729 |
| HSV1-UL30-4313 | - | CCAAGGUGUACUGCGGGGGGG | 21 | 13253 |
| HSV1-UL30-4314 | - | CCCAAGGUGUACUGCGGGGGGG | 22 | 13254 |
| HSV1-UL30-4315 | - | CCCCAAGGUGUACUGCGGGGGGG | 23 | 13255 |
| HSV1-UL30-4316 | - | CCCCCAAGGUGUACUGCGGGGGGG | 24 | 13256 |
| HSV1-UL30-4317 | - | UCGCCCCGCGGGUGCUGG | 18 | 13257 |
| HSV1-UL30-4318 | - | AUCGCCCCGCGGGUGCUGG | 19 | 13258 |
| HSV1-UL30-257 | - | CAUCGCCCCGCGGGUGCUGG | 20 | 4723 |
| HSV1-UL30-4319 | - | UCAUCGCCCCGCGGGUGCUGG | 21 | 13259 |
| HSV1-UL30-4320 | - | UUCAUCGCCCCGCGGGUGCUGG | 22 | 13260 |
| HSV1-UL30-4321 | - | AUUCAUCGCCCCGCGGGUGCUGG | 23 | 13261 |
| HSV1-UL30-4322 | - | GAUUCAUCGCCCCGCGGGUGCUGG | 24 | 13262 |
| HSV1-UL30-4323 | - | CGCCCCCAAGGUGUACUG | 18 | 13263 |
| HSV1-UL30-4324 | - | GCGCCCCCAAGGUGUACUG | 19 | 13264 |
| HSV1-UL30-30 | - | CGCGCCCCCAAGGUGUACUG | 20 | 3135 |
| HSV1-UL30-4325 | - | GCGCGCCCCCAAGGUGUACUG | 21 | 13265 |
| HSV1-UL30-4326 | - | AGCGCGCCCCCAAGGUGUACUG | 22 | 13266 |
| HSV1-UL30-4327 | - | AAGCGCGCCCCCAAGGUGUACUG | 23 | 13267 |
| HSV1-UL30-4328 | - | CAAGCGCGCCCCCAAGGUGUACUG | 24 | 13268 |
| HSV1-UL30-4329 | - | CCGCGGCGCUCGCGCCUG | 18 | 13269 |
| HSV1-UL30-4330 | - | GCCGCGGCGCUCGCGCCUG | 19 | 13270 |
| HSV1-UL30-43 | - | GGCCGCGGCGCUCGCGCCUG | 20 | 3253 |
| HSV1-UL30-4331 | - | UGGCCGCGGCGCUCGCGCCUG | 21 | 13271 |
| HSV1-UL30-4332 | - | CUGGCCGCGGCGCUCGCGCCUG | 22 | 13272 |
| HSV1-UL30-4333 | - | UCUGGCCGCGGCGCUCGCGCCUG | 23 | 13273 |
| HSV1-UL30-4334 | - | UUCUGGCCGCGGCGCUCGCGCCUG | 24 | 13274 |
| HSV1-UL30-4335 | - | CACGUGUACGACAUCCUG | 18 | 13275 |
| HSV1-UL30-4336 | - | UCACGUGUACGACAUCCUG | 19 | 13276 |
| HSV1-UL30-280 | - | UUCACGUGUACGACAUCCUG | 20 | 4736 |
| HSV1-UL30-4337 | - | UUUCACGUGUACGACAUCCUG | 21 | 13277 |
| HSV1-UL30-4338 | - | CUUUCACGUGUACGACAUCCUG | 22 | 13278 |
| HSV1-UL30-4339 | - | UCUUUCACGUGUACGACAUCCUG | 23 | 13279 |
| HSV1-UL30-4340 | - | GUCUUUCACGUGUACGACAUCCUG | 24 | 13280 |
| HSV1-UL30-4341 | - | UUCAUCGCCCCGCGGGUG | 18 | 13281 |
| HSV1-UL30-4342 | - | AUUCAUCGCCCCGCGGGUG | 19 | 13282 |
| HSV1-UL30-256 | - | GAUUCAUCGCCCCGCGGGUG | 20 | 4722 |
| HSV1-UL30-4343 | - | CGAUUCAUCGCCCCGCGGGUG | 21 | 13283 |
| HSV1-UL30-4344 | - | UCGAUUCAUCGCCCCGCGGGUG | 22 | 13284 |
| HSV1-UL30-4345 | - | UUCGAUUCAUCGCCCCGCGGGUG | 23 | 13285 |
| HSV1-UL30-4346 | - | UUUCGAUUCAUCGCCCCGCGGGUG | 24 | 13286 |
| HSV1-UL30-4347 | - | GCGCCCCCAAGGUGUACU | 18 | 13287 |
| HSV1-UL30-4348 | - | CGCGCCCCCAAGGUGUACU | 19 | 13288 |
| HSV1-UL30-264 | - | GCGCGCCCCCAAGGUGUACU | 20 | 4728 |
| HSV1-UL30-4349 | - | AGCGCGCCCCCAAGGUGUACU | 21 | 13289 |
| HSV1-UL30-4350 | - | AAGCGCGCCCCCAAGGUGUACU | 22 | 13290 |
| HSV1-UL30-4351 | - | CAAGCGCGCCCCCAAGGUGUACU | 23 | 13291 |
| HSV1-UL30-4352 | - | UCAAGCGCGCCCCCAAGGUGUACU | 24 | 13292 |
| HSV1-UL30-4353 | - | GCCGCGGCGCUCGCGCCU | 18 | 13293 |
| HSV1-UL30-4354 | - | GGCCGCGGCGCUCGCGCCU | 19 | 13294 |
| HSV1-UL30-273 | - | UGGCCGCGGCGCUCGCGCCU | 20 | 4732 |
| HSV1-UL30-4355 | - | CUGGCCGCGGCGCUCGCGCCU | 21 | 13295 |
| HSV1-UL30-4356 | - | UCUGGCCGCGGCGCUCGCGCCU | 22 | 13296 |
| HSV1-UL30-4357 | - | UUCUGGCCGCGGCGCUCGCGCCU | 23 | 13297 |
| HSV1-UL30-4358 | - | CUUCUGGCCGCGGCGCUCGCGCCU | 24 | 13298 |
| HSV1-UL30-4359 | - | CCCCUACCUCGCCCCAGU | 18 | 13299 |
| HSV1-UL30-4360 | - | ACCCCUACCUCGCCCCAGU | 19 | 13300 |
| HSV1-UL30-16 | - | AACCCCUACCUCGCCCCAGU | 20 | 3244 |
| HSV1-UL30-4361 | - | CAACCCCUACCUCGCCCCAGU | 21 | 13301 |
| HSV1-UL30-4362 | - | ACAACCCCUACCUCGCCCCAGU | 22 | 13302 |
| HSV1-UL30-4363 | - | UACAACCCCUACCUCGCCCCAGU | 23 | 13303 |
| HSV1-UL30-4364 | - | UUACAACCCCUACCUCGCCCCAGU | 24 | 13304 |
| HSV1-UL30-4365 | - | CGGGGACCCCCGCCUUGU | 18 | 13305 |
| HSV1-UL30-4366 | - | CCGGGGACCCCCGCCUUGU | 19 | 13306 |
| HSV1-UL30-248 | - | GCCGGGGACCCCCGCCUUGU | 20 | 4715 |
| HSV1-UL30-4367 | - | AGCCGGGGACCCCCGCCUUGU | 21 | 13307 |
| HSV1-UL30-4368 | - | CAGCCGGGGACCCCCGCCUUGU | 22 | 13308 |
| HSV1-UL30-4369 | - | CCAGCCGGGGACCCCCGCCUUGU | 23 | 13309 |
| HSV1-UL30-4370 | - | GCCAGCCGGGGACCCCCGCCUUGU | 24 | 13310 |
| HSV1-UL30-4371 | - | CAGUUCCACGCGCGGUUU | 18 | 13311 |
| HSV1-UL30-4372 | - | CCAGUUCCACGCGCGGUUU | 19 | 13312 |
| HSV1-UL30-4373 | - | CCCAGUUCCACGCGCGGUUU | 20 | 13313 |
| HSV1-UL30-4374 | - | GCCCAGUUCCACGCGCGGUUU | 21 | 13314 |
| HSV1-UL30-4375 | - | GGCCCAGUUCCACGCGCGGUUU | 22 | 13315 |
| HSV1-UL30-4376 | - | CGGCCCAGUUCCACGCGCGGUUU | 23 | 13316 |
| HSV1-UL30-4377 | - | GCGGCCCAGUUCCACGCGCGGUUU | 24 | 13317 |

**Table 9E** provides exemplary targeting domains for knocking out the *UL30* gene selected according to the fifth tier parameters. The targeting domains fall in the coding sequence of the gene, downstream of the first 500bp of coding sequence (e.g., anywhere from +500 (relative to the start codon) to the stop codon of the gene), and start with a 5'G. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9E**

| **5th Tier** | | | | |
|---|---|---|---|---|
| **gRNA Name** | **DNA Strand** | **Targeting Domain** | **Target Site Length** | **Seq ID NO:** |
| HSV1-UL30-4378 | + | GGGGUUGUCCAGGAUGAA | 18 | 13318 |
| HSV1-UL30-4379 | + | GAACCCGGGGUUGUCCAGGAUGAA | 24 | 13319 |
| HSV1-UL30-4380 | + | GCACUCCCGUGAACCCGUACA | 21 | 13320 |
| HSV1-UL30-4381 | + | GCUGCACUCCCGUGAACCCGUACA | 24 | 13321 |
| HSV1-UL30-4382 | + | GCAAAGUCGAACACCACCA | 19 | 13322 |
| HSV1-UL30-2476 | + | GGCAAAGUCGAACACCACCA | 20 | 5482 |
| HSV1-UL30-4383 | + | GCUGGCAAAGUCGAACACCACCA | 23 | 13323 |
| HSV1-UL30-4384 | + | GGCUGGCAAAGUCGAACACCACCA | 24 | 13324 |
| HSV1-UL30-2427 | + | GUCCCCGUAGAUGAUGCGCA | 20 | 5433 |
| HSV1-UL30-4385 | + | GUGUCCCCGUAGAUGAUGCGCA | 22 | 13325 |
| HSV1-UL30-1205 | + | GCCGCGGCACAGCACAAAGA | 20 | 4143 |
| HSV1-UL30-4386 | + | GGCCGCGGCACAGCACAAAGA | 21 | 13326 |
| HSV1-UL30-4387 | + | GAGGCCGCGGCACAGCACAAAGA | 23 | 13327 |
| HSV1-UL30-4388 | + | GACAGGUCGUAGAGCAGA | 18 | 13328 |
| HSV1-UL30-4389 | + | GGACAGGUCGUAGAGCAGA | 19 | 13329 |
| HSV1-UL30-4390 | + | GUGGACAGGUCGUAGAGCAGA | 21 | 13330 |
| HSV1-UL30-4391 | + | GGUGGACAGGUCGUAGAGCAGA | 22 | 13331 |
| HSV1-UL30-4392 | + | GUGGUGGACAGGUCGUAGAGCAGA | 24 | 13332 |
| HSV1-UL30-4393 | + | GCUCUGGGGAAUCCGCGA | 18 | 13333 |
| HSV1-UL30-4394 | + | GCUGCUCUGGGGAAUCCGCGA | 21 | 13334 |
| HSV1-UL30-4395 | + | GGCUGCUCUGGGGAAUCCGCGA | 22 | 13335 |
| HSV1-UL30-4396 | + | GGGCUGCUCUGGGGAAUCCGCGA | 23 | 13336 |
| HSV1-UL30-4397 | + | GGGGCUGCUCUGGGGAAUCCGCGA | 24 | 13337 |
| HSV1-UL30-2388 | + | GUCUGGGCCACGAUCACGUA | 20 | 5395 |
| HSV1-UL30-4398 | + | GGUCUGGGCCACGAUCACGUA | 21 | 13338 |
| HSV1-UL30-4399 | + | GGGUCUGGGCCACGAUCACGUA | 22 | 13339 |
| HSV1-UL30-4400 | + | GCGGGUCUGGGCCACGAUCACGUA | 24 | 13340 |
| HSV1-UL30-4401 | + | GUGAACGUCUUUUCGCAC | 18 | 13341 |
| HSV1-UL30-4402 | + | GGUGAACGUCUUUUCGCAC | 19 | 13342 |
| HSV1-UL30-4403 | + | GCUUGGUGAACGUCUUUUCGCAC | 23 | 13343 |
| HSV1-UL30-4404 | + | GGGGCCGUACUGUUUCAC | 18 | 13344 |
| HSV1-UL30-4405 | + | GAACUCGGGGCCGUACUGUUUCAC | 24 | 13345 |
| HSV1-UL30-4406 | + | GCAGCAGGCACGUAAAGAC | 19 | 13346 |
| HSV1-UL30-4407 | + | GCGCAGCAGGCACGUAAAGAC | 21 | 13347 |
| HSV1-UL30-4408 | + | GGCGCAGCAGGCACGUAAAGAC | 22 | 13348 |
| HSV1-UL30-4409 | + | GUACCAGCCGAAGGUGACGAACC | 23 | 13349 |
| HSV1-UL30-4410 | + | GGUACCAGCCGAAGGUGACGAACC | 24 | 13350 |
| HSV1-UL30-4411 | + | GGCGCCCCUAAAUCGCCC | 18 | 13351 |
| HSV1-UL30-4412 | + | GUUAUUCCCAAACAGGGCC | 19 | 13352 |
| HSV1-UL30-4413 | + | GCGUUAUUCCCAAACAGGGCC | 21 | 13353 |
| HSV1-UL30-4414 | + | GGCGUUAUUCCCAAACAGGGCC | 22 | 13354 |
| HSV1-UL30-4415 | + | GCUUGUUGGUGUACGCGC | 18 | 13355 |
| HSV1-UL30-2395 | + | GCGCUUGUUGGUGUACGCGC | 20 | 5402 |
| HSV1-UL30-4416 | + | GGCGCUUGUUGGUGUACGCGC | 21 | 13356 |
| HSV1-UL30-4417 | + | GCCCGGCGGCGUAGUAGGC | 19 | 13357 |
| HSV1-UL30-2500 | + | GGCCCGGCGGCGUAGUAGGC | 20 | 5506 |
| HSV1-UL30-4418 | + | GGGCCCGGCGGCGUAGUAGGC | 21 | 13358 |
| HSV1-UL30-4419 | + | GCGGGCCCGGCGGCGUAGUAGGC | 23 | 13359 |
| HSV1-UL30-4420 | + | GCAACGUGCAGGCACGGC | 18 | 13360 |
| HSV1-UL30-4421 | + | GGCAACGUGCAGGCACGGC | 19 | 13361 |
| HSV1-UL30-4422 | + | GCGGCAACGUGCAGGCACGGC | 21 | 13362 |
| HSV1-UL30-4423 | + | GUCGCGGCAACGUGCAGGCACGGC | 24 | 13363 |
| HSV1-UL30-4424 | + | GCCUGCAGUCCCUCGGGC | 18 | 13364 |
| HSV1-UL30-4425 | + | GAACGCCUGCAGUCCCUCGGGC | 22 | 13365 |
| HSV1-UL30-4426 | + | GAAGCACAGGUUGUGGGC | 18 | 13366 |
| HSV1-UL30-4427 | + | GCUGAAGCACAGGUUGUGGGC | 21 | 13367 |
| HSV1-UL30-4428 | + | GUGCUGAAGCACAGGUUGUGGGC | 23 | 13368 |
| HSV1-UL30-2473 | + | GUUGUGGGCCUGGAUGAUGC | 20 | 5479 |
| HSV1-UL30-4429 | + | GGUUGUGGGCCUGGAUGAUGC | 21 | 13369 |
| HSV1-UL30-4430 | + | GUCAUGAAGGCCAACAGCAUC | 21 | 13370 |
| HSV1-UL30-4431 | + | GGUCAUGAAGGCCAACAGCAUC | 22 | 13371 |
| HSV1-UL30-4432 | + | GGGUCAUGAAGGCCAACAGCAUC | 23 | 13372 |
| HSV1-UL30-4433 | + | GCGCGCGUGGACGUACUC | 18 | 13373 |
| HSV1-UL30-4434 | + | GCCCAGCGCGCGUGGACGUACUC | 23 | 13374 |
| HSV1-UL30-4435 | + | GACCGUCUCCUCUACCUC | 18 | 13375 |
| HSV1-UL30-2385 | + | GCGACCGUCUCCUCUACCUC | 20 | 5392 |
| HSV1-UL30-4436 | + | GCGCGACCGUCUCCUCUACCUC | 22 | 13376 |
| HSV1-UL30-4437 | + | GGCGGUGAGGACAAAGUC | 18 | 13377 |
| HSV1-UL30-4438 | + | GUUCGGCGGUGAGGACAAAGUC | 22 | 13378 |
| HSV1-UL30-4439 | + | GACGAACCCGGGGUUGUC | 18 | 13379 |
| HSV1-UL30-2558 | + | GUGACGAACCCGGGGUUGUC | 20 | 5563 |
| HSV1-UL30-4440 | + | GGUGACGAACCCGGGGUUGUC | 21 | 13380 |
| HSV1-UL30-4441 | + | GAAGGUGACGAACCCGGGGUUGUC | 24 | 13381 |
| HSV1-UL30-4442 | + | GUCCGGGGGGUGCCACACUUC | 21 | 13382 |
| HSV1-UL30-4443 | + | GUCGUCCGGGGGGUGCCACACUUC | 24 | 13383 |
| HSV1-UL30-4444 | + | GGCCACCCGGUGGCCUUC | 18 | 13384 |
| HSV1-UL30-4445 | + | GAACGGCCACCCGGUGGCCUUC | 22 | 13385 |
| HSV1-UL30-4446 | + | GUGAACGGCCACCCGGUGGCCUUC | 24 | 13386 |
| HSV1-UL30-2481 | + | GUUCACGUGAAACCCGGAAG | 20 | 5487 |
| HSV1-UL30-4447 | + | GGUUCACGUGAAACCCGGAAG | 21 | 13387 |
| HSV1-UL30-4448 | + | GGGUUCACGUGAAACCCGGAAG | 22 | 13388 |
| HSV1-UL30-4449 | + | GGGGUUCACGUGAAACCCGGAAG | 23 | 13389 |
| HSV1-UL30-4450 | + | GGCGAGCCAGUCCCGCAG | 18 | 13390 |
| HSV1-UL30-4451 | + | GCAUGGCGAGCCAGUCCCGCAG | 22 | 13391 |
| HSV1-UL30-4452 | + | GCUUGGACGCGCCUCCCG | 18 | 13392 |
| HSV1-UL30-4453 | + | GGCUUGGACGCGCCUCCCG | 19 | 13393 |
| HSV1-UL30-2363 | + | GGGCUUGGACGCGCCUCCCG | 20 | 5375 |
| HSV1-UL30-4454 | + | GGGGCUUGGACGCGCCUCCCG | 21 | 13394 |
| HSV1-UL30-4455 | + | GCGGGGCUUGGACGCGCCUCCCG | 23 | 13395 |
| HSV1-UL30-4456 | + | GCGCGGCCACGUCGUCCG | 18 | 13396 |
| HSV1-UL30-4457 | + | GCCGCGCGGCCACGUCGUCCG | 21 | 13397 |
| HSV1-UL30-4458 | + | GAGCCGCGCGGCCACGUCGUCCG | 23 | 13398 |
| HSV1-UL30-4459 | + | GGAGCCGCGCGGCCACGUCGUCCG | 24 | 13399 |
| HSV1-UL30-4460 | + | GUACAGUUAAACUCGACGUCG | 21 | 13400 |
| HSV1-UL30-4461 | + | GCCGUACAGUUAAACUCGACGUCG | 24 | 13401 |
| HSV1-UL30-4462 | + | GGCUCUAUGCAACAUUCG | 18 | 13402 |
| HSV1-UL30-4463 | + | GCCGCCAGCUCGUUCAGG | 18 | 13403 |
| HSV1-UL30-4464 | + | GGCCGCCAGCUCGUUCAGG | 19 | 13404 |
| HSV1-UL30-2355 | + | GCCGUGGGCAAUGGCGUAUG | 20 | 5367 |
| HSV1-UL30-4465 | + | GACGCCGUGGGCAAUGGCGUAUG | 23 | 13405 |
| HSV1-UL30-4466 | + | GCCAACAGCAUCUCGAAUUCGCUG | 24 | 13406 |
| HSV1-UL30-2452 | + | GCCUCCUCGGGGCUGCUCUG | 20 | 5458 |
| HSV1-UL30-4467 | + | GGCCUCCUCGGGGCUGCUCUG | 21 | 13407 |
| HSV1-UL30-4468 | + | GUAGAGCAGACAGGAUAU | 18 | 13408 |
| HSV1-UL30-4469 | + | GUCGUAGAGCAGACAGGAUAU | 21 | 13409 |
| HSV1-UL30-4470 | + | GGUCGUAGAGCAGACAGGAUAU | 22 | 13410 |
| HSV1-UL30-4471 | + | GAUAUCUGGAUGACCAGGUCCU | 22 | 13411 |
| HSV1-UL30-4472 | + | GGAUAUCUGGAUGACCAGGUCCU | 23 | 13412 |
| HSV1-UL30-4473 | + | GACAAAGUCCUGGAUGUCCCUCU | 23 | 13413 |
| HSV1-UL30-4474 | + | GGACAAAGUCCUGGAUGUCCCUCU | 24 | 13414 |
| HSV1-UL30-4475 | + | GCUGGCCGUCGUAGAUGGU | 19 | 13415 |
| HSV1-UL30-4476 | + | GAUCUGCUGGCCGUCGUAGAUGGU | 24 | 13416 |
| HSV1-UL30-4477 | - | GUGGUUCUGGAAUUCGACA | 19 | 13417 |
| HSV1-UL30-4478 | - | GCCCGUGGUUCUGGAAUUCGACA | 23 | 13418 |
| HSV1-UL30-4479 | - | GCCUGACUCCGGAAGGCCA | 19 | 13419 |
| HSV1-UL30-1777 | - | GGCCUGACUCCGGAAGGCCA | 20 | 4827 |
| HSV1-UL30-4480 | - | GGGCCUGACUCCGGAAGGCCA | 21 | 13420 |
| HSV1-UL30-4481 | - | GGUCCCGUCCAUCAAGGA | 18 | 13421 |
| HSV1-UL30-4482 | - | GCAGGUCCCGUCCAUCAAGGA | 21 | 13422 |
| HSV1-UL30-4483 | - | GCGCAGGUCCCGUCCAUCAAGGA | 23 | 13423 |
| HSV1-UL30-1844 | - | GUACGGCCCCGAGUUCGUGA | 20 | 4894 |
| HSV1-UL30-4484 | - | GAUCGGCGAGUACUGCAUA | 19 | 13424 |
| HSV1-UL30-4485 | - | GUGAUCGGCGAGUACUGCAUA | 21 | 13425 |
| HSV1-UL30-4486 | - | GGUGAUCGGCGAGUACUGCAUA | 22 | 13426 |
| HSV1-UL30-4487 | - | GGGUGAUCGGCGAGUACUGCAUA | 23 | 13427 |
| HSV1-UL30-4488 | - | GGGGUGAUCGGCGAGUACUGCAUA | 24 | 13428 |
| HSV1-UL30-4489 | - | GGUGAACAUCGACAUGUA | 18 | 13429 |
| HSV1-UL30-4490 | - | GCAUGGUGAACAUCGACAUGUA | 22 | 13430 |
| HSV1-UL30-4491 | - | GGCAUGGUGAACAUCGACAUGUA | 23 | 13431 |
| HSV1-UL30-1981 | - | GUUUCUGCCCCCCAUCAAAC | 20 | 5014 |
| HSV1-UL30-4492 | - | GCUGUUUCUGCCCCCCAUCAAAC | 23 | 13432 |
| HSV1-UL30-4493 | - | GUAACUCGGUGUACGGGUUCAC | 22 | 13433 |
| HSV1-UL30-4494 | - | GUGUAACUCGGUGUACGGGUUCAC | 24 | 13434 |
| HSV1-UL30-4495 | - | GUACAUCGGCGUCAUCUAC | 19 | 13435 |
| HSV1-UL30-1798 | - | GCCCGGCCAUCAAGAAGUAC | 20 | 4848 |
| HSV1-UL30-4496 | - | GGUUCAUCCUGGACAACC | 18 | 13436 |
| HSV1-UL30-4497 | - | GAAUAACGCCAAGAUCACC | 19 | 13437 |
| HSV1-UL30-2039 | - | GGAAUAACGCCAAGAUCACC | 20 | 5072 |
| HSV1-UL30-4498 | - | GGGAAUAACGCCAAGAUCACC | 21 | 13438 |
| HSV1-UL30-4499 | - | GAGAUGCUGCUCGCGACCC | 19 | 13439 |
| HSV1-UL30-4500 | - | GCGAGAUGCUGCUCGCGACCC | 21 | 13440 |
| HSV1-UL30-4501 | - | GCCGCGAGAUGCUGCUCGCGACCC | 24 | 13441 |
| HSV1-UL30-4502 | - | GACCCUUGUGAAACAGUACGGCC | 23 | 13442 |
| HSV1-UL30-4503 | - | GCUGGUGUCCGAGCUGGCC | 19 | 13443 |
| HSV1-UL30-4504 | - | GCUGCUGGUGUCCGAGCUGGCC | 22 | 13444 |
| HSV1-UL30-1833 | - | GCUCGGUUCCUGCGACCUCC | 20 | 4883 |
| HSV1-UL30-1988 | - | GUAAGAUGCUCAUCAAGGGC | 20 | 5021 |
| HSV1-UL30-4505 | - | GGUAAGAUGCUCAUCAAGGGC | 21 | 13445 |
| HSV1-UL30-4506 | - | GGGUAAGAUGCUCAUCAAGGGC | 22 | 13446 |
| HSV1-UL30-4507 | - | GGGGUAAGAUGCUCAUCAAGGGC | 23 | 13447 |
| HSV1-UL30-4508 | - | GGGGGUAAGAUGCUCAUCAAGGGC | 24 | 13448 |
| HSV1-UL30-4509 | - | GCACGUGGGGUACCAGGGGGC | 21 | 13449 |
| HSV1-UL30-4510 | - | GGCACGUGGGGUACCAGGGGGC | 22 | 13450 |
| HSV1-UL30-4511 | - | GCAUGGCCGCGGCCCUGC | 18 | 13451 |
| HSV1-UL30-1785 | - | GCGCAUGGCCGCGGCCCUGC | 20 | 4835 |
| HSV1-UL30-4512 | - | GAGCGCAUGGCCGCGGCCCUGC | 22 | 13452 |
| HSV1-UL30-4513 | - | GCGAGCGCAUGGCCGCGGCCCUGC | 24 | 13453 |
| HSV1-UL30-4514 | - | GCGAAAGCAGAUCCGCUC | 18 | 13454 |
| HSV1-UL30-4515 | - | GCCAUGCGAAAGCAGAUCCGCUC | 23 | 13455 |
| HSV1-UL30-4516 | - | GCCUGCCCACGCCCGUGGUUC | 21 | 13456 |
| HSV1-UL30-4517 | - | GGCCUGCCCACGCCCGUGGUUC | 22 | 13457 |
| HSV1-UL30-4518 | - | GGGCCUGCCCACGCCCGUGGUUC | 23 | 13458 |
| HSV1-UL30-4519 | - | GGGGCCUGCCCACGCCCGUGGUUC | 24 | 13459 |
| HSV1-UL30-534 | - | GCCGCCGGGCCCGCGCAACG | 20 | 3472 |
| HSV1-UL30-4520 | - | GAGACCGCCGGCAGGCACG | 19 | 13460 |
| HSV1-UL30-595 | - | GGAGACCGCCGGCAGGCACG | 20 | 3533 |
| HSV1-UL30-4521 | - | GGGAGACCGCCGGCAGGCACG | 21 | 13461 |
| HSV1-UL30-4522 | - | GCGGGAGACCGCCGGCAGGCACG | 23 | 13462 |
| HSV1-UL30-4523 | - | GCCUUCGGGACAUCCAGCGACG | 22 | 13463 |
| HSV1-UL30-4524 | - | GGCCUUCGGGACAUCCAGCGACG | 23 | 13464 |
| HSV1-UL30-4525 | - | GCCGCGCGGCUCCGGACCG | 19 | 13465 |
| HSV1-UL30-2043 | - | GGCCGCGCGGCUCCGGACCG | 20 | 5076 |
| HSV1-UL30-4526 | - | GUGGCCGCGCGGCUCCGGACCG | 22 | 13466 |
| HSV1-UL30-4527 | - | GACCGCAGGGUUCGGGGCG | 19 | 13467 |
| HSV1-UL30-2046 | - | GGACCGCAGGGUUCGGGGCG | 20 | 5079 |
| HSV1-UL30-4528 | - | GCGCAACGCGGGGUGAUCG | 19 | 13468 |
| HSV1-UL30-4529 | - | GCCCGCGCAACGCGGGGUGAUCG | 23 | 13469 |
| HSV1-UL30-4530 | - | GGCCCGCGCAACGCGGGGUGAUCG | 24 | 13470 |
| HSV1-UL30-2051 | - | GCUACGGCGGAGGAAACUCG | 20 | 5084 |
| HSV1-UL30-4531 | - | GCGCUACGGCGGAGGAAACUCG | 22 | 13471 |
| HSV1-UL30-4532 | - | GGCGCUACGGCGGAGGAAACUCG | 23 | 13472 |
| HSV1-UL30-1996 | - | GUUAGCCGAGCGCCCCGCGG | 20 | 5029 |
| HSV1-UL30-4533 | - | GCGUUAGCCGAGCGCCCCGCGG | 22 | 13473 |
| HSV1-UL30-1800 | - | GCCAUCAAGAAGUACGAGGG | 20 | 4850 |
| HSV1-UL30-4534 | - | GGCCAUCAAGAAGUACGAGGG | 21 | 13474 |
| HSV1-UL30-4535 | - | GACAUUUACAAGGUCCCCCUGG | 22 | 13475 |
| HSV1-UL30-4536 | - | GGACAUUUACAAGGUCCCCCUGG | 23 | 13476 |
| HSV1-UL30-4537 | - | GCUCUCGGCCGUCGCGCGCUUG | 22 | 13477 |
| HSV1-UL30-4538 | - | GAGCUCUCGGCCGUCGCGCGCUUG | 24 | 13478 |
| HSV1-UL30-4539 | - | GUC GUGUGUAACUC GGUGU | 19 | 13479 |
| HSV1-UL30-1962 | - | GGUC GUGUGUAACUC GGUGU | 20 | 4995 |
| HSV1-UL30-4540 | - | GGGUCCUUGACCCCACUU | 18 | 13480 |
| HSV1-UL30-4541 | - | GCCAGGGUCCUUGACCCCACUU | 22 | 13481 |
| HSV1-UL30-4542 | - | GGCCAGGGUCCUUGACCCCACUU | 23 | 13482 |
| HSV1-UL30-4543 | - | GGGCCAGGGUCCUUGACCCCACUU | 24 | 13483 |
| HSV1-UL30-4544 | - | GACAUUCAAGGCCCUGUUU | 19 | 13484 |
| HSV1-UL30-4545 | - | GUGACAUUCAAGGCCCUGUUU | 21 | 13485 |
| HSV1-UL30-4546 | - | GCGUGACAUUCAAGGCCCUGUUU | 23 | 13486 |

**Table 9F** provides exemplary targeting domains for knocking out the *UL30* gene selected according to the six tier parameters. The targeting domains fall in the coding sequence of the gene, downstream of the first 500bp of coding sequence (e.g., anywhere from +500 (relative to the start codon) to the stop codon of the gene). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9F**

| **6th Tier** | | | | |
|---|---|---|---|---|
| **gRNA Name** | **DNA Strand** | **Targeting Domain** | **Target Site Length** | **Seq ID NO:** |
| HSV1-UL30-4547 | + | CGGGGUUGUCCAGGAUGAA | 19 | 13487 |
| HSV1-UL30-2560 | + | CCGGGGUUGUCCAGGAUGAA | 20 | 5565 |
| HSV1-UL30-4548 | + | CCCGGGGUUGUCCAGGAUGAA | 21 | 13488 |
| HSV1-UL30-4549 | + | ACCCGGGGUUGUCCAGGAUGAA | 22 | 13489 |
| HSV1-UL30-4550 | + | AACCCGGGGUUGUCCAGGAUGAA | 23 | 13490 |
| HSV1-UL30-4551 | + | CUCCCGUGAACCCGUACA | 18 | 13491 |
| HSV1-UL30-4552 | + | ACUCCCGUGAACCCGUACA | 19 | 13492 |
| HSV1-UL30-2444 | + | CACUCCCGUGAACCCGUACA | 20 | 5450 |
| HSV1-UL30-4553 | + | UGCACUCCCGUGAACCCGUACA | 22 | 13493 |
| HSV1-UL30-4554 | + | CUGCACUCCCGUGAACCCGUACA | 23 | 13494 |
| HSV1-UL30-4555 | + | CAAAGUCGAACACCACCA | 18 | 13495 |
| HSV1-UL30-4556 | + | UGGCAAAGUCGAACACCACCA | 21 | 13496 |
| HSV1-UL30-4557 | + | CUGGCAAAGUCGAACACCACCA | 22 | 13497 |
| HSV1-UL30-4558 | + | ACAGCUGGCCCACCAGCA | 18 | 13498 |
| HSV1-UL30-4559 | + | AACAGCUGGCCCACCAGCA | 19 | 13499 |
| HSV1-UL30-2496 | + | AAACAGCUGGCCCACCAGCA | 20 | 5502 |
| HSV1-UL30-4560 | + | AAAACAGCUGGCCCACCAGCA | 21 | 13500 |
| HSV1-UL30-4561 | + | AAAAACAGCUGGCCCACCAGCA | 22 | 13501 |
| HSV1-UL30-4562 | + | AAAAAACAGCUGGCCCACCAGCA | 23 | 13502 |
| HSV1-UL30-4563 | + | UAAAAAACAGCUGGCCCACCAGCA | 24 | 13503 |
| HSV1-UL30-4564 | + | CCCCGUAGAUGAUGCGCA | 18 | 13504 |
| HSV1-UL30-4565 | + | UCCCCGUAGAUGAUGCGCA | 19 | 13505 |
| HSV1-UL30-4566 | + | UGUCCCCGUAGAUGAUGCGCA | 21 | 13506 |
| HSV1-UL30-4567 | + | CGUGUCCCCGUAGAUGAUGCGCA | 23 | 13507 |
| HSV1-UL30-4568 | + | CCGUGUCCCCGUAGAUGAUGCGCA | 24 | 13508 |
| HSV1-UL30-4569 | + | CGCGGCACAGCACAAAGA | 18 | 13509 |
| HSV1-UL30-4570 | + | CCGCGGCACAGCACAAAGA | 19 | 13510 |
| HSV1-UL30-4571 | + | AGGCCGCGGCACAGCACAAAGA | 22 | 13511 |
| HSV1-UL30-4572 | + | UGAGGCCGCGGCACAGCACAAAGA | 24 | 13512 |
| HSV1-UL30-2541 | + | UGGACAGGUCGUAGAGCAGA | 20 | 5546 |
| HSV1-UL30-4573 | + | UGGUGGACAGGUCGUAGAGCAGA | 23 | 13513 |
| HSV1-UL30-4574 | + | UGCUCUGGGGAAUCCGCGA | 19 | 13514 |
| HSV1-UL30-2454 | + | CUGCUCUGGGGAAUCCGCGA | 20 | 5460 |
| HSV1-UL30-4575 | + | CUGGGCCACGAUCACGUA | 18 | 13515 |
| HSV1-UL30-4576 | + | UCUGGGCCACGAUCACGUA | 19 | 13516 |
| HSV1-UL30-4577 | + | CGGGUCUGGGCCACGAUCACGUA | 23 | 13517 |
| HSV1-UL30-2416 | + | UGGUGAACGUCUUUUCGCAC | 20 | 5423 |
| HSV1-UL30-4578 | + | UUGGUGAACGUCUUUUCGCAC | 21 | 13518 |
| HSV1-UL30-4579 | + | CUUGGUGAACGUCUUUUCGCAC | 22 | 13519 |
| HSV1-UL30-4580 | + | AGCUUGGUGAACGUCUUUUCGCAC | 24 | 13520 |
| HSV1-UL30-4581 | + | CGGGGCCGUACUGUUUCAC | 19 | 13521 |
| HSV1-UL30-2518 | + | UCGGGGCCGUACUGUUUCAC | 20 | 5524 |
| HSV1-UL30-4582 | + | CUCGGGGCCGUACUGUUUCAC | 21 | 13522 |
| HSV1-UL30-4583 | + | ACUCGGGGCCGUACUGUUUCAC | 22 | 13523 |
| HSV1-UL30-4584 | + | AACUCGGGGCCGUACUGUUUCAC | 23 | 13524 |
| HSV1-UL30-4585 | + | CAGCAGGCACGUAAAGAC | 18 | 13525 |
| HSV1-UL30-2488 | + | CGCAGCAGGCACGUAAAGAC | 20 | 5494 |
| HSV1-UL30-4586 | + | AGGCGCAGCAGGCACGUAAAGAC | 23 | 13526 |
| HSV1-UL30-4587 | + | CAGGCGCAGCAGGCACGUAAAGAC | 24 | 13527 |
| HSV1-UL30-4588 | + | AGCCGAAGGUGACGAACC | 18 | 13528 |
| HSV1-UL30-4589 | + | CAGCCGAAGGUGACGAACC | 19 | 13529 |
| HSV1-UL30-2557 | + | CCAGCCGAAGGUGACGAACC | 20 | 5562 |
| HSV1-UL30-4590 | + | ACCAGCCGAAGGUGACGAACC | 21 | 13530 |
| HSV1-UL30-4591 | + | UACCAGCCGAAGGUGACGAACC | 22 | 13531 |
| HSV1-UL30-4592 | + | CGGCGCCCCUAAAUCGCCC | 19 | 13532 |
| HSV1-UL30-2486 | + | CCGGCGCCCCUAAAUCGCCC | 20 | 5492 |
| HSV1-UL30-4593 | + | CCCGGCGCCCCUAAAUCGCCC | 21 | 13533 |
| HSV1-UL30-4594 | + | CCCCGGCGCCCCUAAAUCGCCC | 22 | 13534 |
| HSV1-UL30-4595 | + | CCCCCGGCGCCCCUAAAUCGCCC | 23 | 13535 |
| HSV1-UL30-4596 | + | CCCCCCGGCGCCCCUAAAUCGCCC | 24 | 13536 |
| HSV1-UL30-4597 | + | UUAUUCCCAAACAGGGCC | 18 | 13537 |
| HSV1-UL30-2347 | + | CGUUAUUCCCAAACAGGGCC | 20 | 5360 |
| HSV1-UL30-4598 | + | UGGCGUUAUUCCCAAACAGGGCC | 23 | 13538 |
| HSV1-UL30-4599 | + | UUGGCGUUAUUCCCAAACAGGGCC | 24 | 13539 |
| HSV1-UL30-4600 | + | CGCUUGUUGGUGUACGCGC | 19 | 13540 |
| HSV1-UL30-4601 | + | AGGCGCUUGUUGGUGUACGCGC | 22 | 13541 |
| HSV1-UL30-4602 | + | CAGGCGCUUGUUGGUGUACGCGC | 23 | 13542 |
| HSV1-UL30-4603 | + | CCAGGCGCUUGUUGGUGUACGCGC | 24 | 13543 |
| HSV1-UL30-4604 | + | CCCGGCGGCGUAGUAGGC | 18 | 13544 |
| HSV1-UL30-4605 | + | CGGGCCCGGCGGCGUAGUAGGC | 22 | 13545 |
| HSV1-UL30-4606 | + | CGCGGGCCCGGCGGCGUAGUAGGC | 24 | 13546 |
| HSV1-UL30-2442 | + | CGGCAACGUGCAGGCACGGC | 20 | 5448 |
| HSV1-UL30-4607 | + | CGCGGCAACGUGCAGGCACGGC | 22 | 13547 |
| HSV1-UL30-4608 | + | UCGCGGCAACGUGCAGGCACGGC | 23 | 13548 |
| HSV1-UL30-4609 | + | CGCCUGCAGUCCCUCGGGC | 19 | 13549 |
| HSV1-UL30-2406 | + | ACGCCUGCAGUCCCUCGGGC | 20 | 5413 |
| HSV1-UL30-4610 | + | AACGCCUGCAGUCCCUCGGGC | 21 | 13550 |
| HSV1-UL30-4611 | + | CGAACGCCUGCAGUCCCUCGGGC | 23 | 13551 |
| HSV1-UL30-4612 | + | CCGAACGCCUGCAGUCCCUCGGGC | 24 | 13552 |
| HSV1-UL30-4613 | + | UGAAGCACAGGUUGUGGGC | 19 | 13553 |
| HSV1-UL30-2471 | + | CUGAAGCACAGGUUGUGGGC | 20 | 5477 |
| HSV1-UL30-4614 | + | UGCUGAAGCACAGGUUGUGGGC | 22 | 13554 |
| HSV1-UL30-4615 | + | CGUGCUGAAGCACAGGUUGUGGGC | 24 | 13555 |
| HSV1-UL30-4616 | + | UGUGGGCCUGGAUGAUGC | 18 | 13556 |
| HSV1-UL30-4617 | + | UUGUGGGCCUGGAUGAUGC | 19 | 13557 |
| HSV1-UL30-4618 | + | AGGUUGUGGGCCUGGAUGAUGC | 22 | 13558 |
| HSV1-UL30-4619 | + | CAGGUUGUGGGCCUGGAUGAUGC | 23 | 13559 |
| HSV1-UL30-4620 | + | ACAGGUUGUGGGCCUGGAUGAUGC | 24 | 13560 |
| HSV1-UL30-4621 | + | AUGAAGGCCAACAGCAUC | 18 | 13561 |
| HSV1-UL30-4622 | + | CAUGAAGGCCAACAGCAUC | 19 | 13562 |
| HSV1-UL30-2520 | + | UCAUGAAGGCCAACAGCAUC | 20 | 5526 |
| HSV1-UL30-4623 | + | AGGGUCAUGAAGGCCAACAGCAUC | 24 | 13563 |
| HSV1-UL30-4624 | + | AGCGCGCGUGGACGUACUC | 19 | 13564 |
| HSV1-UL30-2439 | + | CAGCGCGCGUGGACGUACUC | 20 | 5445 |
| HSV1-UL30-4625 | + | CCAGCGCGCGUGGACGUACUC | 21 | 13565 |
| HSV1-UL30-4626 | + | CCCAGCGCGCGUGGACGUACUC | 22 | 13566 |
| HSV1-UL30-4627 | + | CGCCCAGCGCGCGUGGACGUACUC | 24 | 13567 |
| HSV1-UL30-4628 | + | CGACCGUCUCCUCUACCUC | 19 | 13568 |
| HSV1-UL30-4629 | + | CGCGACCGUCUCCUCUACCUC | 21 | 13569 |
| HSV1-UL30-4630 | + | CGCGCGACCGUCUCCUCUACCUC | 23 | 13570 |
| HSV1-UL30-4631 | + | CCGCGCGACCGUCUCCUCUACCUC | 24 | 13571 |
| HSV1-UL30-4632 | + | CGGCGGUGAGGACAAAGUC | 19 | 13572 |
| HSV1-UL30-2398 | + | UCGGCGGUGAGGACAAAGUC | 20 | 5405 |
| HSV1-UL30-4633 | + | UUCGGCGGUGAGGACAAAGUC | 21 | 13573 |
| HSV1-UL30-4634 | + | AGUUCGGCGGUGAGGACAAAGUC | 23 | 13574 |
| HSV1-UL30-4635 | + | CAGUUCGGCGGUGAGGACAAAGUC | 24 | 13575 |
| HSV1-UL30-4636 | + | UGACGAACCCGGGGUUGUC | 19 | 13576 |
| HSV1-UL30-4637 | + | AGGUGACGAACCCGGGGUUGUC | 22 | 13577 |
| HSV1-UL30-4638 | + | AAGGUGACGAACCCGGGGUUGUC | 23 | 13578 |
| HSV1-UL30-4639 | + | CGGGGGGUGCCACACUUC | 18 | 13579 |
| HSV1-UL30-4640 | + | CCGGGGGGUGCCACACUUC | 19 | 13580 |
| HSV1-UL30-2345 | + | UCCGGGGGGUGCCACACUUC | 20 | 5358 |
| HSV1-UL30-4641 | + | CGUCCGGGGGGUGCCACACUUC | 22 | 13581 |
| HSV1-UL30-4642 | + | UCGUCCGGGGGGUGCCACACUUC | 23 | 13582 |
| HSV1-UL30-4643 | + | CGGCCACCCGGUGGCCUUC | 19 | 13583 |
| HSV1-UL30-2581 | + | ACGGCCACCCGGUGGCCUUC | 20 | 5586 |
| HSV1-UL30-4644 | + | AACGGCCACCCGGUGGCCUUC | 21 | 13584 |
| HSV1-UL30-4645 | + | UGAACGGCCACCCGGUGGCCUUC | 23 | 13585 |
| HSV1-UL30-4646 | + | UCACGUGAAACCCGGAAG | 18 | 13586 |
| HSV1-UL30-4647 | + | UUCACGUGAAACCCGGAAG | 19 | 13587 |
| HSV1-UL30-4648 | + | CGGGGUUCACGUGAAACCCGGAAG | 24 | 13588 |
| HSV1-UL30-4649 | + | UGGCGAGCCAGUCCCGCAG | 19 | 13589 |
| HSV1-UL30-2458 | + | AUGGCGAGCCAGUCCCGCAG | 20 | 5464 |
| HSV1-UL30-4650 | + | CAUGGCGAGCCAGUCCCGCAG | 21 | 13590 |
| HSV1-UL30-4651 | + | CGCAUGGCGAGCCAGUCCCGCAG | 23 | 13591 |
| HSV1-UL30-4652 | + | UCGCAUGGCGAGCCAGUCCCGCAG | 24 | 13592 |
| HSV1-UL30-4653 | + | CGGGGCUUGGACGCGCCUCCCG | 22 | 13593 |
| HSV1-UL30-4654 | + | UGCGGGGCUUGGACGCGCCUCCCG | 24 | 13594 |
| HSV1-UL30-4655 | + | CGCGCGGCCACGUCGUCCG | 19 | 13595 |
| HSV1-UL30-2342 | + | CCGCGCGGCCACGUCGUCCG | 20 | 5355 |
| HSV1-UL30-4656 | + | AGCCGCGCGGCCACGUCGUCCG | 22 | 13596 |
| HSV1-UL30-4657 | + | CAGUUAAACUCGACGUCG | 18 | 13597 |
| HSV1-UL30-4658 | + | ACAGUUAAACUCGACGUCG | 19 | 13598 |
| HSV1-UL30-2548 | + | UACAGUUAAACUCGACGUCG | 20 | 5553 |
| HSV1-UL30-4659 | + | CGUACAGUUAAACUCGACGUCG | 22 | 13599 |
| HSV1-UL30-4660 | + | CCGUACAGUUAAACUCGACGUCG | 23 | 13600 |
| HSV1-UL30-4661 | + | AGGCUCUAUGCAACAUUCG | 19 | 13601 |
| HSV1-UL30-2335 | + | AAGGCUCUAUGCAACAUUCG | 20 | 5348 |
| HSV1-UL30-4662 | + | AAAGGCUCUAUGCAACAUUCG | 21 | 13602 |
| HSV1-UL30-4663 | + | CAAAGGCUCUAUGCAACAUUCG | 22 | 13603 |
| HSV1-UL30-4664 | + | UCAAAGGCUCUAUGCAACAUUCG | 23 | 13604 |
| HSV1-UL30-4665 | + | AUCAAAGGCUCUAUGCAACAUUCG | 24 | 13605 |
| HSV1-UL30-2526 | + | UGGCCGCCAGCUCGUUCAGG | 20 | 5532 |
| HSV1-UL30-4666 | + | CUGGCCGCCAGCUCGUUCAGG | 21 | 13606 |
| HSV1-UL30-4667 | + | CCUGGCCGCCAGCUCGUUCAGG | 22 | 13607 |
| HSV1-UL30-4668 | + | CCCUGGCCGCCAGCUCGUUCAGG | 23 | 13608 |
| HSV1-UL30-4669 | + | CCCCUGGCCGCCAGCUCGUUCAGG | 24 | 13609 |
| HSV1-UL30-4670 | + | UCGCCCCUGGGUGUCCGG | 18 | 13610 |
| HSV1-UL30-4671 | + | AUCGCCCCUGGGUGUCCGG | 19 | 13611 |
| HSV1-UL30-2487 | + | AAUCGCCCCUGGGUGUCCGG | 20 | 5493 |
| HSV1-UL30-4672 | + | AAAUCGCCCCUGGGUGUCCGG | 21 | 13612 |
| HSV1-UL30-4673 | + | UAAAUCGCCCCUGGGUGUCCGG | 22 | 13613 |
| HSV1-UL30-4674 | + | CUAAAUCGCCCCUGGGUGUCCGG | 23 | 13614 |
| HSV1-UL30-4675 | + | CCUAAAUCGCCCCUGGGUGUCCGG | 24 | 13615 |
| HSV1-UL30-4676 | + | CGUGGGCAAUGGCGUAUG | 18 | 13616 |
| HSV1-UL30-4677 | + | CCGUGGGCAAUGGCGUAUG | 19 | 13617 |
| HSV1-UL30-4678 | + | CGCCGUGGGCAAUGGCGUAUG | 21 | 13618 |
| HSV1-UL30-4679 | + | ACGCCGUGGGCAAUGGCGUAUG | 22 | 13619 |
| HSV1-UL30-4680 | + | CGACGCCGUGGGCAAUGGCGUAUG | 24 | 13620 |
| HSV1-UL30-4681 | + | AGCAUCUCGAAUUCGCUG | 18 | 13621 |
| HSV1-UL30-4682 | + | CAGCAUCUCGAAUUCGCUG | 19 | 13622 |
| HSV1-UL30-2521 | + | ACAGCAUCUCGAAUUCGCUG | 20 | 5527 |
| HSV1-UL30-4683 | + | AACAGCAUCUCGAAUUCGCUG | 21 | 13623 |
| HSV1-UL30-4684 | + | CAACAGCAUCUCGAAUUCGCUG | 22 | 13624 |
| HSV1-UL30-4685 | + | CCAACAGCAUCUCGAAUUCGCUG | 23 | 13625 |
| HSV1-UL30-4686 | + | CUCCUCGGGGCUGCUCUG | 18 | 13626 |
| HSV1-UL30-4687 | + | CCUCCUCGGGGCUGCUCUG | 19 | 13627 |
| HSV1-UL30-4688 | + | CGGCCUCCUCGGGGCUGCUCUG | 22 | 13628 |
| HSV1-UL30-4689 | + | ACGGCCUCCUCGGGGCUGCUCUG | 23 | 13629 |
| HSV1-UL30-4690 | + | CACGGCCUCCUCGGGGCUGCUCUG | 24 | 13630 |
| HSV1-UL30-4691 | + | CGUAGAGCAGACAGGAUAU | 19 | 13631 |
| HSV1-UL30-2542 | + | UCGUAGAGCAGACAGGAUAU | 20 | 5547 |
| HSV1-UL30-4692 | + | AGGUCGUAGAGCAGACAGGAUAU | 23 | 13632 |
| HSV1-UL30-4693 | + | CAGGUCGUAGAGCAGACAGGAUAU | 24 | 13633 |
| HSV1-UL30-4694 | + | UCUGGAUGACCAGGUCCU | 18 | 13634 |
| HSV1-UL30-4695 | + | AUCUGGAUGACCAGGUCCU | 19 | 13635 |
| HSV1-UL30-2543 | + | UAUCUGGAUGACCAGGUCCU | 20 | 5548 |
| HSV1-UL30-4696 | + | AUAUCUGGAUGACCAGGUCCU | 21 | 13636 |
| HSV1-UL30-4697 | + | AGGAUAUCUGGAUGACCAGGUCCU | 24 | 13637 |
| HSV1-UL30-4698 | + | AGUCCUGGAUGUCCCUCU | 18 | 13638 |
| HSV1-UL30-4699 | + | AAGUCCUGGAUGUCCCUCU | 19 | 13639 |
| HSV1-UL30-2399 | + | AAAGUCCUGGAUGUCCCUCU | 20 | 5406 |
| HSV1-UL30-4700 | + | CAAAGUCCUGGAUGUCCCUCU | 21 | 13640 |
| HSV1-UL30-4701 | + | ACAAAGUCCUGGAUGUCCCUCU | 22 | 13641 |
| HSV1-UL30-4702 | + | CUGGCCGUCGUAGAUGGU | 18 | 13642 |
| HSV1-UL30-2489 | + | UGCUGGCCGUCGUAGAUGGU | 20 | 5495 |
| HSV1-UL30-4703 | + | CUGCUGGCCGUCGUAGAUGGU | 21 | 13643 |
| HSV1-UL30-4704 | + | UCUGCUGGCCGUCGUAGAUGGU | 22 | 13644 |
| HSV1-UL30-4705 | + | AUCUGCUGGCCGUCGUAGAUGGU | 23 | 13645 |
| HSV1-UL30-4706 | - | UGGUUCUGGAAUUCGACA | 18 | 13646 |
| HSV1-UL30-1840 | - | CGUGGUUCUGGAAUUCGACA | 20 | 4890 |
| HSV1-UL30-4707 | - | CCGUGGUUCUGGAAUUCGACA | 21 | 13647 |
| HSV1-UL30-4708 | - | CCCGUGGUUCUGGAAUUCGACA | 22 | 13648 |
| HSV1-UL30-4709 | - | CGCCCGUGGUUCUGGAAUUCGACA | 24 | 13649 |
| HSV1-UL30-4710 | - | CCUGACUCCGGAAGGCCA | 18 | 13650 |
| HSV1-UL30-4711 | - | UGGGCCUGACUCCGGAAGGCCA | 22 | 13651 |
| HSV1-UL30-4712 | - | CUGGGCCUGACUCCGGAAGGCCA | 23 | 13652 |
| HSV1-UL30-4713 | - | CCUGGGCCUGACUCCGGAAGGCCA | 24 | 13653 |
| HSV1-UL30-4714 | - | AGGUCCCGUCCAUCAAGGA | 19 | 13654 |
| HSV1-UL30-2011 | - | CAGGUCCCGUCCAUCAAGGA | 20 | 5044 |
| HSV1-UL30-4715 | - | CGCAGGUCCCGUCCAUCAAGGA | 22 | 13655 |
| HSV1-UL30-4716 | - | CGCGCAGGUCCCGUCCAUCAAGGA | 24 | 13656 |
| HSV1-UL30-4717 | - | ACGGCCCCGAGUUCGUGA | 18 | 13657 |
| HSV1-UL30-4718 | - | UACGGCCCCGAGUUCGUGA | 19 | 13658 |
| HSV1-UL30-4719 | - | AGUACGGCCCCGAGUUCGUGA | 21 | 13659 |
| HSV1-UL30-4720 | - | CAGUACGGCCCCGAGUUCGUGA | 22 | 13660 |
| HSV1-UL30-4721 | - | ACAGUACGGCCCCGAGUUCGUGA | 23 | 13661 |
| HSV1-UL30-4722 | - | AACAGUACGGCCCCGAGUUCGUGA | 24 | 13662 |
| HSV1-UL30-4723 | - | AUCGGCGAGUACUGCAUA | 18 | 13663 |
| HSV1-UL30-1871 | - | UGAUCGGCGAGUACUGCAUA | 20 | 4920 |
| HSV1-UL30-4724 | - | AGCUCAUGUGCUUCGAUA | 18 | 13664 |
| HSV1-UL30-4725 | - | AAGCUCAUGUGCUUCGAUA | 19 | 13665 |
| HSV1-UL30-1816 | - | CAAGCUCAUGUGCUUCGAUA | 20 | 4866 |
| HSV1-UL30-4726 | - | ACAAGCUCAUGUGCUUCGAUA | 21 | 13666 |
| HSV1-UL30-4727 | - | UACAAGCUCAUGUGCUUCGAUA | 22 | 13667 |
| HSV1-UL30-4728 | - | AUACAAGCUCAUGUGCUUCGAUA | 23 | 13668 |
| HSV1-UL30-4729 | - | CAUACAAGCUCAUGUGCUUCGAUA | 24 | 13669 |
| HSV1-UL30-4730 | - | UGGUGAACAUCGACAUGUA | 19 | 13670 |
| HSV1-UL30-1858 | - | AUGGUGAACAUCGACAUGUA | 20 | 4908 |
| HSV1-UL30-4731 | - | CAUGGUGAACAUCGACAUGUA | 21 | 13671 |
| HSV1-UL30-4732 | - | CGGCAUGGUGAACAUCGACAUGUA | 24 | 13672 |
| HSV1-UL30-4733 | - | UUCUGCCCCCCAUCAAAC | 18 | 13673 |
| HSV1-UL30-4734 | - | UUUCUGCCCCCCAUCAAAC | 19 | 13674 |
| HSV1-UL30-4735 | - | UGUUUCUGCCCCCCAUCAAAC | 21 | 13675 |
| HSV1-UL30-4736 | - | CUGUUUCUGCCCCCCAUCAAAC | 22 | 13676 |
| HSV1-UL30-4737 | - | CGCUGUUUCUGCCCCCCAUCAAAC | 24 | 13677 |
| HSV1-UL30-4738 | - | CUCGGUGUACGGGUUCAC | 18 | 13678 |
| HSV1-UL30-4739 | - | ACUCGGUGUACGGGUUCAC | 19 | 13679 |
| HSV1-UL30-1965 | - | AACUCGGUGUACGGGUUCAC | 20 | 4998 |
| HSV1-UL30-4740 | - | UAACUCGGUGUACGGGUUCAC | 21 | 13680 |
| HSV1-UL30-4741 | - | UGUAACUCGGUGUACGGGUUCAC | 23 | 13681 |
| HSV1-UL30-4742 | - | UACAUCGGCGUCAUCUAC | 18 | 13682 |
| HSV1-UL30-1986 | - | AGUACAUCGGCGUCAUCUAC | 20 | 5019 |
| HSV1-UL30-4743 | - | AAGUACAUCGGCGUCAUCUAC | 21 | 13683 |
| HSV1-UL30-4744 | - | AAAGUACAUCGGCGUCAUCUAC | 22 | 13684 |
| HSV1-UL30-4745 | - | AAAAGUACAUCGGCGUCAUCUAC | 23 | 13685 |
| HSV1-UL30-4746 | - | AAAAAGUACAUCGGCGUCAUCUAC | 24 | 13686 |
| HSV1-UL30-4747 | - | CCGGCCAUCAAGAAGUAC | 18 | 13687 |
| HSV1-UL30-4748 | - | CCCGGCCAUCAAGAAGUAC | 19 | 13688 |
| HSV1-UL30-4749 | - | UGCCCGGCCAUCAAGAAGUAC | 21 | 13689 |
| HSV1-UL30-4750 | - | CUGCCCGGCCAUCAAGAAGUAC | 22 | 13690 |
| HSV1-UL30-4751 | - | UCUGCCCGGCCAUCAAGAAGUAC | 23 | 13691 |
| HSV1-UL30-4752 | - | UUCUGCCCGGCCAUCAAGAAGUAC | 24 | 13692 |
| HSV1-UL30-4753 | - | CGGUUCAUCCUGGACAACC | 19 | 13693 |
| HSV1-UL30-1802 | - | CCGGUUCAUCCUGGACAACC | 20 | 4852 |
| HSV1-UL30-4754 | - | CCCGGUUCAUCCUGGACAACC | 21 | 13694 |
| HSV1-UL30-4755 | - | ACCCGGUUCAUCCUGGACAACC | 22 | 13695 |
| HSV1-UL30-4756 | - | CACCCGGUUCAUCCUGGACAACC | 23 | 13696 |
| HSV1-UL30-4757 | - | CCACCCGGUUCAUCCUGGACAACC | 24 | 13697 |
| HSV1-UL30-4758 | - | AAUAACGCCAAGAUCACC | 18 | 13698 |
| HSV1-UL30-4759 | - | UGGGAAUAACGCCAAGAUCACC | 22 | 13699 |
| HSV1-UL30-4760 | - | UUGGGAAUAACGCCAAGAUCACC | 23 | 13700 |
| HSV1-UL30-4761 | - | UUUGGGAAUAACGCCAAGAUCACC | 24 | 13701 |
| HSV1-UL30-4762 | - | AGAUGCUGCUCGCGACCC | 18 | 13702 |
| HSV1-UL30-1968 | - | CGAGAUGCUGCUCGCGACCC | 20 | 5001 |
| HSV1-UL30-4763 | - | CGCGAGAUGCUGCUCGCGACCC | 22 | 13703 |
| HSV1-UL30-4764 | - | CCGCGAGAUGCUGCUCGCGACCC | 23 | 13704 |
| HSV1-UL30-4765 | - | UUGUGAAACAGUACGGCC | 18 | 13705 |
| HSV1-UL30-4766 | - | CUUGUGAAACAGUACGGCC | 19 | 13706 |
| HSV1-UL30-1843 | - | CCUUGUGAAACAGUACGGCC | 20 | 4893 |
| HSV1-UL30-4767 | - | CCCUUGUGAAACAGUACGGCC | 21 | 13707 |
| HSV1-UL30-4768 | - | ACCCUUGUGAAACAGUACGGCC | 22 | 13708 |
| HSV1-UL30-4769 | - | UGACCCUUGUGAAACAGUACGGCC | 24 | 13709 |
| HSV1-UL30-4770 | - | CUGGUGUCCGAGCUGGCC | 18 | 13710 |
| HSV1-UL30-2029 | - | UGCUGGUGUCCGAGCUGGCC | 20 | 5062 |
| HSV1-UL30-4771 | - | CUGCUGGUGUCCGAGCUGGCC | 21 | 13711 |
| HSV1-UL30-4772 | - | AGCUGCUGGUGUCCGAGCUGGCC | 23 | 13712 |
| HSV1-UL30-4773 | - | AAGCUGCUGGUGUCCGAGCUGGCC | 24 | 13713 |
| HSV1-UL30-4774 | - | UCGGUUCCUGCGACCUCC | 18 | 13714 |
| HSV1-UL30-4775 | - | CUCGGUUCCUGCGACCUCC | 19 | 13715 |
| HSV1-UL30-4776 | - | CGCUCGGUUCCUGCGACCUCC | 21 | 13716 |
| HSV1-UL30-4777 | - | UCGCUCGGUUCCUGCGACCUCC | 22 | 13717 |
| HSV1-UL30-4778 | - | UUCGCUCGGUUCCUGCGACCUCC | 23 | 13718 |
| HSV1-UL30-4779 | - | UUUCGCUCGGUUCCUGCGACCUCC | 24 | 13719 |
| HSV1-UL30-4780 | - | AAGAUGCUCAUCAAGGGC | 18 | 13720 |
| HSV1-UL30-4781 | - | UAAGAUGCUCAUCAAGGGC | 19 | 13721 |
| HSV1-UL30-4782 | - | CGUGGGGUACCAGGGGGC | 18 | 13722 |
| HSV1-UL30-4783 | - | ACGUGGGGUACCAGGGGGC | 19 | 13723 |
| HSV1-UL30-1933 | - | CACGUGGGGUACCAGGGGGC | 20 | 4966 |
| HSV1-UL30-4784 | - | AGGCACGUGGGGUACCAGGGGGC | 23 | 13724 |
| HSV1-UL30-4785 | - | CAGGCACGUGGGGUACCAGGGGGC | 24 | 13725 |
| HSV1-UL30-4786 | - | CGCAUGGCCGCGGCCCUGC | 19 | 13726 |
| HSV1-UL30-4787 | - | AGCGCAUGGCCGCGGCCCUGC | 21 | 13727 |
| HSV1-UL30-4788 | - | CGAGCGCAUGGCCGCGGCCCUGC | 23 | 13728 |
| HSV1-UL30-4789 | - | UGCGAAAGCAGAUCCGCUC | 19 | 13729 |
| HSV1-UL30-1956 | - | AUGCGAAAGCAGAUCCGCUC | 20 | 4989 |
| HSV1-UL30-4790 | - | CAUGCGAAAGCAGAUCCGCUC | 21 | 13730 |
| HSV1-UL30-4791 | - | CCAUGCGAAAGCAGAUCCGCUC | 22 | 13731 |
| HSV1-UL30-4792 | - | CGCCAUGCGAAAGCAGAUCCGCUC | 24 | 13732 |
| HSV1-UL30-4793 | - | UGCCCACGCCCGUGGUUC | 18 | 13733 |
| HSV1-UL30-4794 | - | CUGCCCACGCCCGUGGUUC | 19 | 13734 |
| HSV1-UL30-1839 | - | CCUGCCCACGCCCGUGGUUC | 20 | 4889 |
| HSV1-UL30-4795 | - | CGCCGGGCCCGCGCAACG | 18 | 13735 |
| HSV1-UL30-4796 | - | CCGCCGGGCCCGCGCAACG | 19 | 13736 |
| HSV1-UL30-4797 | - | CGCCGCCGGGCCCGCGCAACG | 21 | 13737 |
| HSV1-UL30-4798 | - | ACGCCGCCGGGCCCGCGCAACG | 22 | 13738 |
| HSV1-UL30-4799 | - | UACGCCGCCGGGCCCGCGCAACG | 23 | 13739 |
| HSV1-UL30-4800 | - | CUACGCCGCCGGGCCCGCGCAACG | 24 | 13740 |
| HSV1-UL30-4801 | - | AGACCGCCGGCAGGCACG | 18 | 13741 |
| HSV1-UL30-4802 | - | CGGGAGACCGCCGGCAGGCACG | 22 | 13742 |
| HSV1-UL30-4803 | - | CGCGGGAGACCGCCGGCAGGCACG | 24 | 13743 |
| HSV1-UL30-4804 | - | UCGGGACAUCCAGCGACG | 18 | 13744 |
| HSV1-UL30-4805 | - | UUCGGGACAUCCAGCGACG | 19 | 13745 |
| HSV1-UL30-1808 | - | CUUCGGGACAUCCAGCGACG | 20 | 4858 |
| HSV1-UL30-4806 | - | CCUUCGGGACAUCCAGCGACG | 21 | 13746 |
| HSV1-UL30-4807 | - | UGGCCUUCGGGACAUCCAGCGACG | 24 | 13747 |
| HSV1-UL30-4808 | - | CCGCGCGGCUCCGGACCG | 18 | 13748 |
| HSV1-UL30-4809 | - | UGGCCGCGCGGCUCCGGACCG | 21 | 13749 |
| HSV1-UL30-4810 | - | CGUGGCCGCGCGGCUCCGGACCG | 23 | 13750 |
| HSV1-UL30-4811 | - | ACGUGGCCGCGCGGCUCCGGACCG | 24 | 13751 |
| HSV1-UL30-4812 | - | ACCGCAGGGUUCGGGGCG | 18 | 13752 |
| HSV1-UL30-4813 | - | CGGACCGCAGGGUUCGGGGCG | 21 | 13753 |
| HSV1-UL30-4814 | - | CCGGACCGCAGGGUUCGGGGCG | 22 | 13754 |
| HSV1-UL30-4815 | - | UCCGGACCGCAGGGUUCGGGGCG | 23 | 13755 |
| HSV1-UL30-4816 | - | CUCCGGACCGCAGGGUUCGGGGCG | 24 | 13756 |
| HSV1-UL30-4817 | - | CGCAACGCGGGGUGAUCG | 18 | 13757 |
| HSV1-UL30-1870 | - | CGCGCAACGCGGGGUGAUCG | 20 | 4919 |
| HSV1-UL30-4818 | - | CCGCGCAACGCGGGGUGAUCG | 21 | 13758 |
| HSV1-UL30-4819 | - | CCCGCGCAACGCGGGGUGAUCG | 22 | 13759 |
| HSV1-UL30-4820 | - | UACGGCGGAGGAAACUCG | 18 | 13760 |
| HSV1-UL30-4821 | - | CUACGGCGGAGGAAACUCG | 19 | 13761 |
| HSV1-UL30-4822 | - | CGCUACGGCGGAGGAAACUCG | 21 | 13762 |
| HSV1-UL30-4823 | - | CGGCGCUACGGCGGAGGAAACUCG | 24 | 13763 |
| HSV1-UL30-4824 | - | UAGCCGAGCGCCCCGCGG | 18 | 13764 |
| HSV1-UL30-4825 | - | UUAGCCGAGCGCCCCGCGG | 19 | 13765 |
| HSV1-UL30-4826 | - | CGUUAGCCGAGCGCCCCGCGG | 21 | 13766 |
| HSV1-UL30-4827 | - | CGCGUUAGCCGAGCGCCCCGCGG | 23 | 13767 |
| HSV1-UL30-4828 | - | CCGCGUUAGCCGAGCGCCCCGCGG | 24 | 13768 |
| HSV1-UL30-4829 | - | CAUCAAGAAGUACGAGGG | 18 | 13769 |
| HSV1-UL30-4830 | - | CCAUCAAGAAGUACGAGGG | 19 | 13770 |
| HSV1-UL30-4831 | - | CGGCCAUCAAGAAGUACGAGGG | 22 | 13771 |
| HSV1-UL30-4832 | - | CCGGCCAUCAAGAAGUACGAGGG | 23 | 13772 |
| HSV1-UL30-4833 | - | CCCGGCCAUCAAGAAGUACGAGGG | 24 | 13773 |
| HSV1-UL30-4834 | - | UUUACAAGGUCCCCCUGG | 18 | 13774 |
| HSV1-UL30-4835 | - | AUUUACAAGGUCCCCCUGG | 19 | 13775 |
| HSV1-UL30-1847 | - | CAUUUACAAGGUCCCCCUGG | 20 | 4897 |
| HSV1-UL30-4836 | - | ACAUUUACAAGGUCCCCCUGG | 21 | 13776 |
| HSV1-UL30-4837 | - | CGGACAUUUACAAGGUCCCCCUGG | 24 | 13777 |
| HSV1-UL30-4838 | - | UCGGCCGUCGCGCGCUUG | 18 | 13778 |
| HSV1-UL30-4839 | - | CUCGGCCGUCGCGCGCUUG | 19 | 13779 |
| HSV1-UL30-1875 | - | UCUCGGCCGUCGCGCGCUUG | 20 | 4924 |
| HSV1-UL30-4840 | - | CUCUCGGCCGUCGCGCGCUUG | 21 | 13780 |
| HSV1-UL30-4841 | - | AGCUCUCGGCCGUCGCGCGCUUG | 23 | 13781 |
| HSV1-UL30-4842 | - | UC GUGUGUAACUC GGUGU | 18 | 13782 |
| HSV1-UL30-4843 | - | AGGUC GUGUGUAACUC GGUGU | 21 | 13783 |
| HSV1-UL30-4844 | - | AAGGUC GUGUGUAACUC GGUGU | 22 | 13784 |
| HSV1-UL30-4845 | - | CAAGGUCGUGUGUAACUCGGUGU | 23 | 13785 |
| HSV1-UL30-4846 | - | UCAAGGUCGUGUGUAACUCGGUGU | 24 | 13786 |
| HSV1-UL30-4847 | - | AGGGUCCUUGACCCCACUU | 19 | 13787 |
| HSV1-UL30-1934 | - | CAGGGUCCUUGACCCCACUU | 20 | 4967 |
| HSV1-UL30-4848 | - | CCAGGGUCCUUGACCCCACUU | 21 | 13788 |
| HSV1-UL30-4849 | - | ACAUUCAAGGCCCUGUUU | 18 | 13789 |
| HSV1-UL30-2037 | - | UGACAUUCAAGGCCCUGUUU | 20 | 5070 |
| HSV1-UL30-4850 | - | CGUGACAUUCAAGGCCCUGUUU | 22 | 13790 |
| HSV1-UL30-4851 | - | UGCGUGACAUUCAAGGCCCUGUUU | 24 | 13791 |

**Table 9G** provides exemplary targeting domains for knocking out the *UL30* gene selected according to the seven tier parameters. The targeting domains fall in the coding sequence of the gene, downstream of the first 500bp of coding sequence (e.g., anywhere from +500 (relative to the start codon) to the stop codon of the gene). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 9G**

| **7th Tier** | | | | |
|---|---|---|---|---|
| **gRNA Name** | **DNA Strand** | **Targeting Domain** | **Target Site Length** | **Seq ID NO:** |
| HSV1-UL30-4852 | + | GCAGGAACCGAGCGAAAA | 18 | 13792 |
| HSV1-UL30-4853 | + | CGCAGGAACCGAGCGAAAA | 19 | 13793 |
| HSV1-UL30-2535 | + | UCGCAGGAACCGAGCGAAAA | 20 | 5541 |
| HSV1-UL30-4854 | + | GUCGCAGGAACCGAGCGAAAA | 21 | 13794 |
| HSV1-UL30-4855 | + | GGUCGCAGGAACCGAGCGAAAA | 22 | 13795 |
| HSV1-UL30-4856 | + | AGGUCGCAGGAACCGAGCGAAAA | 23 | 13796 |
| HSV1-UL30-4857 | + | GAGGUCGCAGGAACCGAGCGAAAA | 24 | 13797 |
| HSV1-UL30-4858 | + | GACGUAGACGCGGUAAAA | 18 | 13798 |
| HSV1-UL30-4859 | + | GGACGUAGACGCGGUAAAA | 19 | 13799 |
| HSV1-UL30-2564 | + | CGGACGUAGACGCGGUAAAA | 20 | 5569 |
| HSV1-UL30-4860 | + | UCGGACGUAGACGCGGUAAAA | 21 | 13800 |
| HSV1-UL30-4861 | + | UUCGGACGUAGACGCGGUAAAA | 22 | 13801 |
| HSV1-UL30-4862 | + | CUUCGGACGUAGACGCGGUAAAA | 23 | 13802 |
| HSV1-UL30-4863 | + | GCUUCGGACGUAGACGCGGUAAAA | 24 | 13803 |
| HSV1-UL30-4864 | + | CUUGGCGUUAUUCCCAAA | 18 | 13804 |
| HSV1-UL30-4865 | + | UCUUGGCGUUAUUCCCAAA | 19 | 13805 |
| HSV1-UL30-2346 | + | AUCUUGGC GUUAUUC CCAAA | 20 | 5359 |
| HSV1-UL30-4866 | + | GAUCUUGGC GUUAUUC CCAAA | 21 | 13806 |
| HSV1-UL30-4867 | + | UGAUCUUGGC GUUAUUC CCAAA | 22 | 13807 |
| HSV1-UL30-4868 | + | GUGAUCUUGGC GUUAUUC CCAAA | 23 | 13808 |
| HSV1-UL30-4869 | + | GGUGAUCUUGGC GUUAUUC CCAAA | 24 | 13809 |
| HSV1-UL30-4870 | + | UC GGGGAGGUC GCAGGAA | 18 | 13810 |
| HSV1-UL30-4871 | + | UUC GGGGAGGUC GCAGGAA | 19 | 13811 |
| HSV1-UL30-2533 | + | AUUCGGGGAGGUCGCAGGAA | 20 | 5539 |
| HSV1-UL30-4872 | + | GAUUCGGGGAGGUCGCAGGAA | 21 | 13812 |
| HSV1-UL30-4873 | + | GGAUUC GGGGAGGUC GCAGGAA | 22 | 13813 |
| HSV1-UL30-4874 | + | GGGAUUCGGGGAGGUCGCAGGAA | 23 | 13814 |
| HSV1-UL30-4875 | + | UGGGAUUCGGGGAGGUCGCAGGAA | 24 | 13815 |
| HSV1-UL30-4876 | + | UUCACGUGAAACCCGGAA | 18 | 13816 |
| HSV1-UL30-4877 | + | GUUCACGUGAAACCCGGAA | 19 | 13817 |
| HSV1-UL30-2480 | + | GGUUCACGUGAAACCCGGAA | 20 | 5486 |
| HSV1-UL30-4878 | + | GGGUUCACGUGAAACCCGGAA | 21 | 13818 |
| HSV1-UL30-4879 | + | GGGGUUCACGUGAAACCCGGAA | 22 | 13819 |
| HSV1-UL30-4880 | + | CGGGGUUCACGUGAAACCCGGAA | 23 | 13820 |
| HSV1-UL30-4881 | + | ACGGGGUUCACGUGAAACCCGGAA | 24 | 13821 |
| HSV1-UL30-4882 | + | UAGAUGAUGCGCAUGGAA | 18 | 13822 |
| HSV1-UL30-4883 | + | GUAGAUGAUGCGCAUGGAA | 19 | 13823 |
| HSV1-UL30-2428 | + | CGUAGAUGAUGCGCAUGGAA | 20 | 5434 |
| HSV1-UL30-4884 | + | CCGUAGAUGAUGCGCAUGGAA | 21 | 13824 |
| HSV1-UL30-4885 | + | CCCGUAGAUGAUGCGCAUGGAA | 22 | 13825 |
| HSV1-UL30-4886 | + | CCCCGUAGAUGAUGCGCAUGGAA | 23 | 13826 |
| HSV1-UL30-4887 | + | UCCCCGUAGAUGAUGCGCAUGGAA | 24 | 13827 |
| HSV1-UL30-3882 | + | CAUAAACCGCGCGUGGAA | 18 | 12822 |
| HSV1-UL30-3883 | + | CCAUAAACCGCGCGUGGAA | 19 | 12823 |
| HSV1-UL30-3884 | + | UCCAUAAACCGCGCGUGGAA | 20 | 12824 |
| HSV1-UL30-3885 | + | GUCCAUAAACCGCGCGUGGAA | 21 | 12825 |
| HSV1-UL30-3886 | + | CGUCCAUAAACCGCGCGUGGAA | 22 | 12826 |
| HSV1-UL30-3887 | + | GCGUCCAUAAACCGCGCGUGGAA | 23 | 12827 |
| HSV1-UL30-3888 | + | GGCGUCCAUAAACCGCGCGUGGAA | 24 | 12828 |
| HSV1-UL30-4888 | + | UGGAUGUCCCGAAGGCCA | 18 | 13828 |
| HSV1-UL30-4889 | + | CUGGAUGUCCCGAAGGCCA | 19 | 13829 |
| HSV1-UL30-2550 | + | GCUGGAUGUCCCGAAGGCCA | 20 | 5555 |
| HSV1-UL30-4890 | + | CGCUGGAUGUCCCGAAGGCCA | 21 | 13830 |
| HSV1-UL30-4891 | + | UCGCUGGAUGUCCCGAAGGCCA | 22 | 13831 |
| HSV1-UL30-4892 | + | GUCGCUGGAUGUCCCGAAGGCCA | 23 | 13832 |
| HSV1-UL30-4893 | + | CGUCGCUGGAUGUCCCGAAGGCCA | 24 | 13833 |
| HSV1-UL30-4894 | + | CCUCCGGGUGCCCGGCCA | 18 | 13834 |
| HSV1-UL30-4895 | + | UCCUCCGGGUGCCCGGCCA | 19 | 13835 |
| HSV1-UL30-2544 | + | GUCCUCCGGGUGCCCGGCCA | 20 | 5549 |
| HSV1-UL30-4896 | + | GGUCCUCCGGGUGCCCGGCCA | 21 | 13836 |
| HSV1-UL30-4897 | + | AGGUCCUCCGGGUGCCCGGCCA | 22 | 13837 |
| HSV1-UL30-4898 | + | CAGGUCCUCCGGGUGCCCGGCCA | 23 | 13838 |
| HSV1-UL30-4899 | + | CCAGGUCCUCCGGGUGCCCGGCCA | 24 | 13839 |
| HSV1-UL30-4900 | + | UGCGGCCGUACCCGUCCA | 18 | 13840 |
| HSV1-UL30-4901 | + | AUGCGGCCGUACCCGUCCA | 19 | 13841 |
| HSV1-UL30-2510 | + | CAUGCGGCCGUACCCGUCCA | 20 | 5516 |
| HSV1-UL30-4902 | + | UCAUGCGGCCGUACCCGUCCA | 21 | 13842 |
| HSV1-UL30-4903 | + | UUCAUGCGGCCGUACCCGUCCA | 22 | 13843 |
| HSV1-UL30-4904 | + | GUUCAUGCGGCCGUACCCGUCCA | 23 | 13844 |
| HSV1-UL30-4905 | + | CGUUCAUGCGGCCGUACCCGUCCA | 24 | 13845 |
| HSV1-UL30-4906 | + | CCACUGCGUCGGCCCUCA | 18 | 13846 |
| HSV1-UL30-4907 | + | GCCACUGCGUCGGCCCUCA | 19 | 13847 |
| HSV1-UL30-2467 | + | CGCCACUGCGUCGGCCCUCA | 20 | 5473 |
| HSV1-UL30-4908 | + | GCGCCACUGCGUCGGCCCUCA | 21 | 13848 |
| HSV1-UL30-4909 | + | UGCGCCACUGCGUCGGCCCUCA | 22 | 13849 |
| HSV1-UL30-4910 | + | GUGCGCCACUGCGUCGGCCCUCA | 23 | 13850 |
| HSV1-UL30-4911 | + | GGUGCGCCACUGCGUCGGCCCUCA | 24 | 13851 |
| HSV1-UL30-4912 | + | UAGACGCGGUAAAACAGA | 18 | 13852 |
| HSV1-UL30-4913 | + | GUAGACGCGGUAAAACAGA | 19 | 13853 |
| HSV1-UL30-2565 | + | CGUAGACGCGGUAAAACAGA | 20 | 5570 |
| HSV1-UL30-4914 | + | ACGUAGACGCGGUAAAACAGA | 21 | 13854 |
| HSV1-UL30-4915 | + | GAC GUAGAC GCGGUAAAACAGA | 22 | 13855 |
| HSV1-UL30-4916 | + | GGACGUAGACGCGGUAAAACAGA | 23 | 13856 |
| HSV1-UL30-4917 | + | CGGACGUAGACGCGGUAAAACAGA | 24 | 13857 |
| HSV1-UL30-4918 | + | ACGGCCGAGAGCUCCAGA | 18 | 13858 |
| HSV1-UL30-4919 | + | GACGGCCGAGAGCUCCAGA | 19 | 13859 |
| HSV1-UL30-2493 | + | CGACGGCCGAGAGCUCCAGA | 20 | 5499 |
| HSV1-UL30-4920 | + | GCGACGGCCGAGAGCUCCAGA | 21 | 13860 |
| HSV1-UL30-4921 | + | CGCGACGGCCGAGAGCUCCAGA | 22 | 13861 |
| HSV1-UL30-4922 | + | GCGCGACGGCCGAGAGCUCCAGA | 23 | 13862 |
| HSV1-UL30-4923 | + | CGCGCGACGGCCGAGAGCUCCAGA | 24 | 13863 |
| HSV1-UL30-4924 | + | GGAGGUCGCAGGAACCGA | 18 | 13864 |
| HSV1-UL30-4925 | + | GGGAGGUCGCAGGAACCGA | 19 | 13865 |
| HSV1-UL30-2534 | + | GGGGAGGUCGCAGGAACCGA | 20 | 5540 |
| HSV1-UL30-4926 | + | CGGGGAGGUCGCAGGAACCGA | 21 | 13866 |
| HSV1-UL30-4927 | + | UCGGGGAGGUCGCAGGAACCGA | 22 | 13867 |
| HSV1-UL30-4928 | + | UUCGGGGAGGUCGCAGGAACCGA | 23 | 13868 |
| HSV1-UL30-4929 | + | AUUCGGGGAGGUCGCAGGAACCGA | 24 | 13869 |
| HSV1-UL30-4930 | + | GAUCCGGUCCUUGAUGGA | 18 | 13870 |
| HSV1-UL30-4931 | + | GGAUCCGGUCCUUGAUGGA | 19 | 13871 |
| HSV1-UL30-2391 | + | GGGAUCCGGUCCUUGAUGGA | 20 | 5398 |
| HSV1-UL30-4932 | + | CGGGAUCCGGUCCUUGAUGGA | 21 | 13872 |
| HSV1-UL30-4933 | + | ACGGGAUCCGGUCCUUGAUGGA | 22 | 13873 |
| HSV1-UL30-4934 | + | UACGGGAUCCGGUCCUUGAUGGA | 23 | 13874 |
| HSV1-UL30-4935 | + | GUACGGGAUCCGGUCCUUGAUGGA | 24 | 13875 |
| HSV1-UL30-4936 | + | UUUC GCACUC GAGUUUGA | 18 | 13876 |
| HSV1-UL30-4937 | + | UUUUCGCACUCGAGUUUGA | 19 | 13877 |
| HSV1-UL30-2418 | + | CUUUUCGCACUCGAGUUUGA | 20 | 5425 |
| HSV1-UL30-4938 | + | UCUUUUCGCACUCGAGUUUGA | 21 | 13878 |
| HSV1-UL30-4939 | + | GUCUUUUCGCACUCGAGUUUGA | 22 | 13879 |
| HSV1-UL30-4940 | + | CGUCUUUUCGCACUCGAGUUUGA | 23 | 13880 |
| HSV1-UL30-4941 | + | ACGUCUUUUCGCACUCGAGUUUGA | 24 | 13881 |
| HSV1-UL30-4942 | + | CCCGCCUUGCAUUCGAUA | 18 | 13882 |
| HSV1-UL30-4943 | + | CCCCGCCUUGCAUUCGAUA | 19 | 13883 |
| HSV1-UL30-2546 | + | CCCCCGCCUUGCAUUCGAUA | 20 | 5551 |
| HSV1-UL30-4944 | + | CCCCCCGCCUUGCAUUCGAUA | 21 | 13884 |
| HSV1-UL30-4945 | + | CCCCCCCGCCUUGCAUUCGAUA | 22 | 13885 |
| HSV1-UL30-4946 | + | CCCCCCCCGCCUUGCAUUCGAUA | 23 | 13886 |
| HSV1-UL30-4947 | + | UCCCCCCCCGCCUUGCAUUCGAUA | 24 | 13887 |
| HSV1-UL30-4948 | + | CAGGAACCGAGCGAAAAC | 18 | 13888 |
| HSV1-UL30-4949 | + | GCAGGAACCGAGCGAAAAC | 19 | 13889 |
| HSV1-UL30-1345 | + | CGCAGGAACCGAGCGAAAAC | 20 | 4283 |
| HSV1-UL30-4950 | + | UCGCAGGAACCGAGCGAAAAC | 21 | 13890 |
| HSV1-UL30-4951 | + | GUCGCAGGAACCGAGCGAAAAC | 22 | 13891 |
| HSV1-UL30-4952 | + | GGUCGCAGGAACCGAGCGAAAAC | 23 | 13892 |
| HSV1-UL30-4953 | + | AGGUCGCAGGAACCGAGCGAAAAC | 24 | 13893 |
| HSV1-UL30-4954 | + | AC GGGGUUCAC GUGAAAC | 18 | 13894 |
| HSV1-UL30-4955 | + | CAC GGGGUUCAC GUGAAAC | 19 | 13895 |
| HSV1-UL30-2478 | + | CCACGGGGUUCACGUGAAAC | 20 | 5484 |
| HSV1-UL30-4956 | + | ACCACGGGGUUCACGUGAAAC | 21 | 13896 |
| HSV1-UL30-4957 | + | CACCACGGGGUUCACGUGAAAC | 22 | 13897 |
| HSV1-UL30-4958 | + | CCACCACGGGGUUCACGUGAAAC | 23 | 13898 |
| HSV1-UL30-4959 | + | ACCACCACGGGGUUCACGUGAAAC | 24 | 13899 |
| HSV1-UL30-4960 | + | CUGCCGCGUGCCGUAAAC | 18 | 13900 |
| HSV1-UL30-4961 | + | ACUGCCGCGUGCCGUAAAC | 19 | 13901 |
| HSV1-UL30-2579 | + | UACUGCCGCGUGCCGUAAAC | 20 | 5584 |
| HSV1-UL30-4962 | + | GUACUGCCGCGUGCCGUAAAC | 21 | 13902 |
| HSV1-UL30-4963 | + | AGUACUGCCGCGUGCCGUAAAC | 22 | 13903 |
| HSV1-UL30-4964 | + | AAGUACUGCCGCGUGCCGUAAAC | 23 | 13904 |
| HSV1-UL30-4965 | + | AAAGUACUGCCGCGUGCCGUAAAC | 24 | 13905 |
| HSV1-UL30-4966 | + | UUGUACCCGGUCACGAAC | 18 | 13906 |
| HSV1-UL30-4967 | + | GUUGUACCCGGUCACGAAC | 19 | 13907 |
| HSV1-UL30-2516 | + | UGUUGUACCCGGUCACGAAC | 20 | 5522 |
| HSV1-UL30-4968 | + | AUGUUGUACCCGGUCACGAAC | 21 | 13908 |
| HSV1-UL30-4969 | + | GAUGUUGUACCCGGUCACGAAC | 22 | 13909 |
| HSV1-UL30-4970 | + | UGAUGUUGUACCCGGUCACGAAC | 23 | 13910 |
| HSV1-UL30-4971 | + | AUGAUGUUGUACCCGGUCACGAAC | 24 | 13911 |
| HSV1-UL30-4972 | + | CAGCCGAAGGUGACGAAC | 18 | 13912 |
| HSV1-UL30-4973 | + | CCAGCCGAAGGUGACGAAC | 19 | 13913 |
| HSV1-UL30-2556 | + | ACCAGCCGAAGGUGACGAAC | 20 | 5561 |
| HSV1-UL30-4974 | + | UACCAGCCGAAGGUGACGAAC | 21 | 13914 |
| HSV1-UL30-4975 | + | GUACCAGCCGAAGGUGACGAAC | 22 | 13915 |
| HSV1-UL30-4976 | + | GGUACCAGCCGAAGGUGACGAAC | 23 | 13916 |
| HSV1-UL30-4977 | + | CGGUACCAGCCGAAGGUGACGAAC | 24 | 13917 |
| HSV1-UL30-4978 | + | CUGGCCUAUGUCCCACAC | 18 | 13918 |
| HSV1-UL30-4979 | + | UCUGGCCUAUGUCCCACAC | 19 | 13919 |
| HSV1-UL30-2507 | + | CUCUGGCCUAUGUCCCACAC | 20 | 5513 |
| HSV1-UL30-4980 | + | GCUCUGGCCUAUGUCCCACAC | 21 | 13920 |
| HSV1-UL30-4981 | + | GGCUCUGGCCUAUGUCCCACAC | 22 | 13921 |
| HSV1-UL30-4982 | + | UGGCUCUGGCCUAUGUCCCACAC | 23 | 13922 |
| HSV1-UL30-4983 | + | GUGGCUCUGGCCUAUGUCCCACAC | 24 | 13923 |
| HSV1-UL30-4984 | + | CUCCGGGUGCCCGGCCAC | 18 | 13924 |
| HSV1-UL30-4985 | + | CCUCCGGGUGCCCGGCCAC | 19 | 13925 |
| HSV1-UL30-2545 | + | UCCUCCGGGUGCCCGGCCAC | 20 | 5550 |
| HSV1-UL30-4986 | + | GUCCUCCGGGUGCCCGGCCAC | 21 | 13926 |
| HSV1-UL30-4987 | + | GGUCCUCCGGGUGCCCGGCCAC | 22 | 13927 |
| HSV1-UL30-4988 | + | AGGUCCUCCGGGUGCCCGGCCAC | 23 | 13928 |
| HSV1-UL30-4989 | + | CAGGUCCUCCGGGUGCCCGGCCAC | 24 | 13929 |
| HSV1-UL30-4990 | + | CAUUCGAUAUCGAAGCAC | 18 | 13930 |
| HSV1-UL30-4991 | + | GCAUUCGAUAUCGAAGCAC | 19 | 13931 |
| HSV1-UL30-2547 | + | UGCAUUCGAUAUCGAAGCAC | 20 | 5552 |
| HSV1-UL30-4992 | + | UUGCAUUCGAUAUCGAAGCAC | 21 | 13932 |
| HSV1-UL30-4993 | + | CUUGCAUUCGAUAUCGAAGCAC | 22 | 13933 |
| HSV1-UL30-4994 | + | CCUUGCAUUCGAUAUCGAAGCAC | 23 | 13934 |
| HSV1-UL30-4995 | + | GCCUUGCAUUCGAUAUCGAAGCAC | 24 | 13935 |
| HSV1-UL30-4996 | + | GAGGAGGCUCUCUCGCAC | 18 | 13936 |
| HSV1-UL30-4997 | + | UGAGGAGGCUCUCUCGCAC | 19 | 13937 |
| HSV1-UL30-2462 | + | CUGAGGAGGCUCUCUCGCAC | 20 | 5468 |
| HSV1-UL30-4998 | + | GCUGAGGAGGCUCUCUCGCAC | 21 | 13938 |
| HSV1-UL30-4999 | + | UGCUGAGGAGGCUCUCUCGCAC | 22 | 13939 |
| HSV1-UL30-5000 | + | AUGCUGAGGAGGCUCUCUCGCAC | 23 | 13940 |
| HSV1-UL30-5001 | + | GAUGCUGAGGAGGCUCUCUCGCAC | 24 | 13941 |
| HSV1-UL30-5002 | + | CACCACCACGGGGUUCAC | 18 | 13942 |
| HSV1-UL30-5003 | + | ACACCACCACGGGGUUCAC | 19 | 13943 |
| HSV1-UL30-2477 | + | AACACCACCACGGGGUUCAC | 20 | 5483 |
| HSV1-UL30-5004 | + | GAACACCACCACGGGGUUCAC | 21 | 13944 |
| HSV1-UL30-5005 | + | CGAACACCACCACGGGGUUCAC | 22 | 13945 |
| HSV1-UL30-5006 | + | UCGAACACCACCACGGGGUUCAC | 23 | 13946 |
| HSV1-UL30-5007 | + | GUCGAACACCACCACGGGGUUCAC | 24 | 13947 |
| HSV1-UL30-5008 | + | GUAAAACAGCAGGUCGAC | 18 | 13948 |
| HSV1-UL30-5009 | + | CGUAAAACAGCAGGUCGAC | 19 | 13949 |
| HSV1-UL30-2411 | + | UCGUAAAACAGCAGGUCGAC | 20 | 5418 |
| HSV1-UL30-5010 | + | GUCGUAAAACAGCAGGUCGAC | 21 | 13950 |
| HSV1-UL30-5011 | + | CGUCGUAAAACAGCAGGUCGAC | 22 | 13951 |
| HSV1-UL30-5012 | + | UCGUCGUAAAACAGCAGGUCGAC | 23 | 13952 |
| HSV1-UL30-5013 | + | AUCGUCGUAAAACAGCAGGUCGAC | 24 | 13953 |
| HSV1-UL30-5014 | + | GCGCCGAUGGGCGUCUAC | 18 | 13954 |
| HSV1-UL30-5015 | + | UGCGCCGAUGGGCGUCUAC | 19 | 13955 |
| HSV1-UL30-2402 | + | AUGCGCCGAUGGGCGUCUAC | 20 | 5409 |
| HSV1-UL30-5016 | + | GAUGCGCCGAUGGGCGUCUAC | 21 | 13956 |
| HSV1-UL30-5017 | + | UGAUGCGCCGAUGGGCGUCUAC | 22 | 13957 |
| HSV1-UL30-5018 | + | GUGAUGCGCCGAUGGGCGUCUAC | 23 | 13958 |
| HSV1-UL30-5019 | + | GGUGAUGCGCCGAUGGGCGUCUAC | 24 | 13959 |
| HSV1-UL30-5020 | + | CGGGGUUCACGUGAAACC | 18 | 13960 |
| HSV1-UL30-5021 | + | ACGGGGUUCACGUGAAACC | 19 | 13961 |
| HSV1-UL30-2479 | + | CACGGGGUUCACGUGAAACC | 20 | 5485 |
| HSV1-UL30-5022 | + | CCACGGGGUUCACGUGAAACC | 21 | 13962 |
| HSV1-UL30-5023 | + | ACCACGGGGUUCACGUGAAACC | 22 | 13963 |
| HSV1-UL30-5024 | + | CACCACGGGGUUCACGUGAAACC | 23 | 13964 |
| HSV1-UL30-5025 | + | CCACCACGGGGUUCACGUGAAACC | 24 | 13965 |
| HSV1-UL30-5026 | + | CUGUCGAAUUCCAGAACC | 18 | 13966 |
| HSV1-UL30-5027 | + | GCUGUCGAAUUCCAGAACC | 19 | 13967 |
| HSV1-UL30-2523 | + | CGCUGUCGAAUUCCAGAACC | 20 | 5529 |
| HSV1-UL30-5028 | + | UCGCUGUCGAAUUCCAGAACC | 21 | 13968 |
| HSV1-UL30-5029 | + | UUCGCUGUCGAAUUCCAGAACC | 22 | 13969 |
| HSV1-UL30-5030 | + | AUUCGCUGUCGAAUUCCAGAACC | 23 | 13970 |
| HSV1-UL30-5031 | + | AAUUCGCUGUCGAAUUCCAGAACC | 24 | 13971 |
| HSV1-UL30-5032 | + | GCAAAGUCGAACACCACC | 18 | 13972 |
| HSV1-UL30-5033 | + | GGCAAAGUCGAACACCACC | 19 | 13973 |
| HSV1-UL30-2475 | + | UGGCAAAGUCGAACACCACC | 20 | 5481 |
| HSV1-UL30-5034 | + | CUGGCAAAGUCGAACACCACC | 21 | 13974 |
| HSV1-UL30-5035 | + | GCUGGCAAAGUCGAACACCACC | 22 | 13975 |
| HSV1-UL30-5036 | + | GGCUGGCAAAGUCGAACACCACC | 23 | 13976 |
| HSV1-UL30-5037 | + | AGGCUGGCAAAGUCGAACACCACC | 24 | 13977 |
| HSV1-UL30-5038 | + | GGGGGCGGGCUCGUCCCC | 18 | 13978 |
| HSV1-UL30-5039 | + | GGGGGGCGGGCUCGUCCCC | 19 | 13979 |
| HSV1-UL30-2381 | + | GGGGGGGCGGGCUCGUCCCC | 20 | 5388 |
| HSV1-UL30-5040 | + | GGGGGGGGCGGGCUCGUCCCC | 21 | 13980 |
| HSV1-UL30-5041 | + | CGGGGGGGGCGGGCUCGUCCCC | 22 | 13981 |
| HSV1-UL30-5042 | + | GCGGGGGGGGCGGGCUCGUCCCC | 23 | 13982 |
| HSV1-UL30-5043 | + | CGCGGGGGGGGCGGGCUCGUCCCC | 24 | 13983 |
| HSV1-UL30-5044 | + | CCGCGGAACGACGCGCCC | 18 | 13984 |
| HSV1-UL30-5045 | + | GCCGCGGAACGACGCGCCC | 19 | 13985 |
| HSV1-UL30-2574 | + | UGCCGCGGAACGACGCGCCC | 20 | 5579 |
| HSV1-UL30-5046 | + | AUGCCGCGGAACGACGCGCCC | 21 | 13986 |
| HSV1-UL30-5047 | + | GAUGCCGCGGAACGACGCGCCC | 22 | 13987 |
| HSV1-UL30-5048 | + | AGAUGCCGCGGAACGACGCGCCC | 23 | 13988 |
| HSV1-UL30-5049 | + | GAGAUGCCGCGGAACGACGCGCCC | 24 | 13989 |
| HSV1-UL30-5050 | + | CCUUCCGGAGUCAGGCCC | 18 | 13990 |
| HSV1-UL30-5051 | + | GCCUUCCGGAGUCAGGCCC | 19 | 13991 |
| HSV1-UL30-2583 | + | GGCCUUCCGGAGUCAGGCCC | 20 | 5588 |
| HSV1-UL30-5052 | + | UGGCCUUCCGGAGUCAGGCCC | 21 | 13992 |
| HSV1-UL30-5053 | + | GUGGCCUUCCGGAGUCAGGCCC | 22 | 13993 |
| HSV1-UL30-5054 | + | GGUGGCCUUCCGGAGUCAGGCCC | 23 | 13994 |
| HSV1-UL30-5055 | + | CGGUGGCCUUCCGGAGUCAGGCCC | 24 | 13995 |
| HSV1-UL30-5056 | + | GCAGGUCGACCAGGGCCC | 18 | 13996 |
| HSV1-UL30-5057 | + | AGCAGGUCGACCAGGGCCC | 19 | 13997 |
| HSV1-UL30-2413 | + | CAGCAGGUCGACCAGGGCCC | 20 | 5420 |
| HSV1-UL30-5058 | + | ACAGCAGGUCGACCAGGGCCC | 21 | 13998 |
| HSV1-UL30-5059 | + | AACAGCAGGUCGACCAGGGCCC | 22 | 13999 |
| HSV1-UL30-5060 | + | AAACAGCAGGUCGACCAGGGCCC | 23 | 14000 |
| HSV1-UL30-5061 | + | AAAACAGCAGGUCGACCAGGGCCC | 24 | 14001 |
| HSV1-UL30-5062 | + | GCGCAUGGAAUAGGGCCC | 18 | 14002 |
| HSV1-UL30-5063 | + | UGCGCAUGGAAUAGGGCCC | 19 | 14003 |
| HSV1-UL30-2431 | + | AUGCGCAUGGAAUAGGGCCC | 20 | 5437 |
| HSV1-UL30-5064 | + | GAUGCGCAUGGAAUAGGGCCC | 21 | 14004 |
| HSV1-UL30-5065 | + | UGAUGCGCAUGGAAUAGGGCCC | 22 | 14005 |
| HSV1-UL30-5066 | + | AUGAUGCGCAUGGAAUAGGGCCC | 23 | 14006 |
| HSV1-UL30-5067 | + | GAUGAUGCGCAUGGAAUAGGGCCC | 24 | 14007 |
| HSV1-UL30-5068 | + | AGUCCGUGCUGCACUCCC | 18 | 14008 |
| HSV1-UL30-5069 | + | GAGUCCGUGCUGCACUCCC | 19 | 14009 |
| HSV1-UL30-2443 | + | GGAGUCCGUGCUGCACUCCC | 20 | 5449 |
| HSV1-UL30-5070 | + | AGGAGUCCGUGCUGCACUCCC | 21 | 14010 |
| HSV1-UL30-5071 | + | CAGGAGUCCGUGCUGCACUCCC | 22 | 14011 |
| HSV1-UL30-5072 | + | GCAGGAGUCCGUGCUGCACUCCC | 23 | 14012 |
| HSV1-UL30-5073 | + | GGCAGGAGUCCGUGCUGCACUCCC | 24 | 14013 |
| HSV1-UL30-5074 | + | GGCUUGGACGCGCCUCCC | 18 | 14014 |
| HSV1-UL30-5075 | + | GGGCUUGGACGCGCCUCCC | 19 | 14015 |
| HSV1-UL30-2362 | + | GGGGCUUGGACGCGCCUCCC | 20 | 5374 |
| HSV1-UL30-5076 | + | CGGGGCUUGGACGCGCCUCCC | 21 | 14016 |
| HSV1-UL30-5077 | + | GCGGGGCUUGGACGCGCCUCCC | 22 | 14017 |
| HSV1-UL30-5078 | + | UGCGGGGCUUGGACGCGCCUCCC | 23 | 14018 |
| HSV1-UL30-5079 | + | UUGCGGGGCUUGGACGCGCCUCCC | 24 | 14019 |
| HSV1-UL30-5080 | + | CAGGCGACGGCGUCUCCC | 18 | 14020 |
| HSV1-UL30-5081 | + | GCAGGCGACGGCGUCUCCC | 19 | 14021 |
| HSV1-UL30-2366 | + | GGCAGGCGACGGCGUCUCCC | 20 | 5378 |
| HSV1-UL30-5082 | + | CGGCAGGCGACGGCGUCUCCC | 21 | 14022 |
| HSV1-UL30-5083 | + | UCGGCAGGCGACGGCGUCUCCC | 22 | 14023 |
| HSV1-UL30-5084 | + | GUCGGCAGGCGACGGCGUCUCCC | 23 | 14024 |
| HSV1-UL30-5085 | + | GGUCGGCAGGCGACGGCGUCUCCC | 24 | 14025 |
| HSV1-UL30-5086 | + | CCGAACGCCUGCAGUCCC | 18 | 14026 |
| HSV1-UL30-5087 | + | CCCGAACGCCUGCAGUCCC | 19 | 14027 |
| HSV1-UL30-2404 | + | CCCCGAACGCCUGCAGUCCC | 20 | 5411 |
| HSV1-UL30-5088 | + | GCCCCGAACGCCUGCAGUCCC | 21 | 14028 |
| HSV1-UL30-5089 | + | GGCCCCGAACGCCUGCAGUCCC | 22 | 14029 |
| HSV1-UL30-5090 | + | CGGCCCCGAACGCCUGCAGUCCC | 23 | 14030 |
| HSV1-UL30-5091 | + | ACGGCCCCGAACGCCUGCAGUCCC | 24 | 14031 |
| HSV1-UL30-5092 | + | GGGGGGCGGGCUCGUCCC | 18 | 14032 |
| HSV1-UL30-5093 | + | GGGGGGGCGGGCUCGUCCC | 19 | 14033 |
| HSV1-UL30-2380 | + | GGGGGGGGCGGGCUCGUCCC | 20 | 5387 |
| HSV1-UL30-5094 | + | CGGGGGGGGCGGGCUCGUCCC | 21 | 14034 |
| HSV1-UL30-5095 | + | GCGGGGGGGGCGGGCUCGUCCC | 22 | 14035 |
| HSV1-UL30-5096 | + | CGCGGGGGGGGCGGGCUCGUCCC | 23 | 14036 |
| HSV1-UL30-5097 | + | CCGCGGGGGGGGCGGGCUCGUCCC | 24 | 14037 |
| HSV1-UL30-5098 | + | CGUGUUCAGGGCGACGCC | 18 | 14038 |
| HSV1-UL30-5099 | + | CCGUGUUCAGGGCGACGCC | 19 | 14039 |
| HSV1-UL30-2353 | + | UCCGUGUUCAGGGCGACGCC | 20 | 5365 |
| HSV1-UL30-5100 | + | GUCCGUGUUCAGGGCGACGCC | 21 | 14040 |
| HSV1-UL30-5101 | + | AGUCCGUGUUCAGGGCGACGCC | 22 | 14041 |
| HSV1-UL30-5102 | + | UAGUCCGUGUUCAGGGCGACGCC | 23 | 14042 |
| HSV1-UL30-5103 | + | AUAGUCCGUGUUCAGGGCGACGCC | 24 | 14043 |
| HSV1-UL30-5104 | + | GCGCCGGCACCCACCGCC | 18 | 14044 |
| HSV1-UL30-5105 | + | AGCGCCGGCACCCACCGCC | 19 | 14045 |
| HSV1-UL30-2336 | + | UAGCGCCGGCACCCACCGCC | 20 | 5349 |
| HSV1-UL30-5106 | + | GUAGCGCCGGCACCCACCGCC | 21 | 14046 |
| HSV1-UL30-5107 | + | CGUAGCGCCGGCACCCACCGCC | 22 | 14047 |
| HSV1-UL30-5108 | + | CCGUAGCGCCGGCACCCACCGCC | 23 | 14048 |
| HSV1-UL30-5109 | + | GCCGUAGCGCCGGCACCCACCGCC | 24 | 14049 |
| HSV1-UL30-5110 | + | CGCUCCACCACCUCCGCC | 18 | 14050 |
| HSV1-UL30-5111 | + | GCGCUCCACCACCUCCGCC | 19 | 14051 |
| HSV1-UL30-2567 | + | UGCGCUCCACCACCUCCGCC | 20 | 5572 |
| HSV1-UL30-5112 | + | GUGCGCUCCACCACCUCCGCC | 21 | 14052 |
| HSV1-UL30-5113 | + | GGUGCGCUCCACCACCUCCGCC | 22 | 14053 |
| HSV1-UL30-5114 | + | CGGUGCGCUCCACCACCUCCGCC | 23 | 14054 |
| HSV1-UL30-5115 | + | UCGGUGCGCUCCACCACCUCCGCC | 24 | 14055 |
| HSV1-UL30-5116 | + | GCCGCGGAACGACGCGCC | 18 | 14056 |
| HSV1-UL30-5117 | + | UGCCGCGGAACGACGCGCC | 19 | 14057 |
| HSV1-UL30-2573 | + | AUGCCGCGGAACGACGCGCC | 20 | 5578 |
| HSV1-UL30-5118 | + | GAUGCCGCGGAACGACGCGCC | 21 | 14058 |
| HSV1-UL30-5119 | + | AGAUGCCGCGGAACGACGCGCC | 22 | 14059 |
| HSV1-UL30-5120 | + | GAGAUGCCGCGGAACGACGCGCC | 23 | 14060 |
| HSV1-UL30-5121 | + | GGAGAUGCCGCGGAACGACGCGCC | 24 | 14061 |
| HSV1-UL30-5122 | + | GCCUUCCGGAGUCAGGCC | 18 | 14062 |
| HSV1-UL30-5123 | + | GGCCUUCCGGAGUCAGGCC | 19 | 14063 |
| HSV1-UL30-2582 | + | UGGCCUUCCGGAGUCAGGCC | 20 | 5587 |
| HSV1-UL30-5124 | + | GUGGCCUUCCGGAGUCAGGCC | 21 | 14064 |
| HSV1-UL30-5125 | + | GGUGGCCUUCCGGAGUCAGGCC | 22 | 14065 |
| HSV1-UL30-5126 | + | CGGUGGCCUUCCGGAGUCAGGCC | 23 | 14066 |
| HSV1-UL30-5127 | + | CCGGUGGCCUUCCGGAGUCAGGCC | 24 | 14067 |
| HSV1-UL30-5128 | + | GCCAAGCGCGCGACGGCC | 18 | 14068 |
| HSV1-UL30-5129 | + | CGCCAAGCGCGCGACGGCC | 19 | 14069 |
| HSV1-UL30-2491 | + | CCGCCAAGCGCGCGACGGCC | 20 | 5497 |
| HSV1-UL30-5130 | + | CCCGCCAAGCGCGCGACGGCC | 21 | 14070 |
| HSV1-UL30-5131 | + | ACCCGCCAAGCGCGCGACGGCC | 22 | 14071 |
| HSV1-UL30-5132 | + | UACCCGCCAAGCGCGCGACGGCC | 23 | 14072 |
| HSV1-UL30-5133 | + | AUACCCGCCAAGCGCGCGACGGCC | 24 | 14073 |
| HSV1-UL30-5134 | + | GCGUCUACGAGGACGGCC | 18 | 14074 |
| HSV1-UL30-5135 | + | GGCGUCUACGAGGACGGCC | 19 | 14075 |
| HSV1-UL30-2403 | + | GGGCGUCUACGAGGACGGCC | 20 | 5410 |
| HSV1-UL30-5136 | + | UGGGCGUCUACGAGGACGGCC | 21 | 14076 |
| HSV1-UL30-5137 | + | AUGGGCGUCUACGAGGACGGCC | 22 | 14077 |
| HSV1-UL30-5138 | + | GAUGGGCGUCUACGAGGACGGCC | 23 | 14078 |
| HSV1-UL30-5139 | + | CGAUGGGCGUCUACGAGGACGGCC | 24 | 14079 |
| HSV1-UL30-5140 | + | GCCGUCAGCCCGGCGGCC | 18 | 14080 |
| HSV1-UL30-5141 | + | GGCCGUCAGCCCGGCGGCC | 19 | 14081 |
| HSV1-UL30-2423 | + | CGGCCGUCAGCCCGGCGGCC | 20 | 5430 |
| HSV1-UL30-5142 | + | ACGGCCGUCAGCCCGGCGGCC | 21 | 14082 |
| HSV1-UL30-5143 | + | CACGGCCGUCAGCCCGGCGGCC | 22 | 14083 |
| HSV1-UL30-5144 | + | CCACGGCCGUCAGCCCGGCGGCC | 23 | 14084 |
| HSV1-UL30-5145 | + | CCCACGGCCGUCAGCCCGGCGGCC | 24 | 14085 |
| HSV1-UL30-5146 | + | GCCUCCGGGAAAUCGGCC | 18 | 14086 |
| HSV1-UL30-5147 | + | CGCCUCCGGGAAAUCGGCC | 19 | 14087 |
| HSV1-UL30-2436 | + | CCGCCUCCGGGAAAUCGGCC | 20 | 5442 |
| HSV1-UL30-5148 | + | GCCGCCUCCGGGAAAUCGGCC | 21 | 14088 |
| HSV1-UL30-5149 | + | GGCCGCCUCCGGGAAAUCGGCC | 22 | 14089 |
| HSV1-UL30-5150 | + | CGGCCGCCUCCGGGAAAUCGGCC | 23 | 14090 |
| HSV1-UL30-5151 | + | UCGGCCGCCUCCGGGAAAUCGGCC | 24 | 14091 |
| HSV1-UL30-5152 | + | AGCAGGUCGACCAGGGCC | 18 | 14092 |
| HSV1-UL30-5153 | + | CAGCAGGUCGACCAGGGCC | 19 | 14093 |
| HSV1-UL30-2412 | + | ACAGCAGGUCGACCAGGGCC | 20 | 5419 |
| HSV1-UL30-5154 | + | AACAGCAGGUCGACCAGGGCC | 21 | 14094 |
| HSV1-UL30-5155 | + | AAACAGCAGGUCGACCAGGGCC | 22 | 14095 |
| HSV1-UL30-5156 | + | AAAACAGCAGGUCGACCAGGGCC | 23 | 14096 |
| HSV1-UL30-5157 | + | UAAAACAGCAGGUCGACCAGGGCC | 24 | 14097 |
| HSV1-UL30-5158 | + | GCCGGGGAGGGCAGGGCC | 18 | 14098 |
| HSV1-UL30-5159 | + | GGCCGGGGAGGGCAGGGCC | 19 | 14099 |
| HSV1-UL30-2373 | + | UGGCCGGGGAGGGCAGGGCC | 20 | 5385 |
| HSV1-UL30-5160 | + | UUGGCCGGGGAGGGCAGGGCC | 21 | 14100 |
| HSV1-UL30-5161 | + | CUUGGCCGGGGAGGGCAGGGCC | 22 | 14101 |
| HSV1-UL30-5162 | + | GCUUGGCCGGGGAGGGCAGGGCC | 23 | 14102 |
| HSV1-UL30-5163 | + | CGCUUGGCCGGGGAGGGCAGGGCC | 24 | 14103 |
| HSV1-UL30-5164 | + | UGCGCAUGGAAUAGGGCC | 18 | 14104 |
| HSV1-UL30-5165 | + | AUGCGCAUGGAAUAGGGCC | 19 | 14105 |
| HSV1-UL30-2430 | + | GAUGCGCAUGGAAUAGGGCC | 20 | 5436 |
| HSV1-UL30-5166 | + | UGAUGCGCAUGGAAUAGGGCC | 21 | 14106 |
| HSV1-UL30-5167 | + | AUGAUGCGCAUGGAAUAGGGCC | 22 | 14107 |
| HSV1-UL30-5168 | + | GAUGAUGCGCAUGGAAUAGGGCC | 23 | 14108 |
| HSV1-UL30-5169 | + | AGAUGAUGCGCAUGGAAUAGGGCC | 24 | 14109 |
| HSV1-UL30-5170 | + | ACCUGCGCGCGGCGGGCC | 18 | 14110 |
| HSV1-UL30-5171 | + | GACCUGCGCGCGGCGGGCC | 19 | 14111 |
| HSV1-UL30-2393 | + | GGACCUGCGCGCGGCGGGCC | 20 | 5400 |
| HSV1-UL30-5172 | + | GGGACCUGCGCGCGGCGGGCC | 21 | 14112 |
| HSV1-UL30-5173 | + | CGGGACCUGCGCGCGGCGGGCC | 22 | 14113 |
| HSV1-UL30-5174 | + | ACGGGACCUGCGCGCGGCGGGCC | 23 | 14114 |
| HSV1-UL30-5175 | + | GACGGGACCUGCGCGCGGCGGGCC | 24 | 14115 |
| HSV1-UL30-5176 | + | UCCCGGGGGCGCUUGGCC | 18 | 14116 |
| HSV1-UL30-5177 | + | CUCCCGGGGGCGCUUGGCC | 19 | 14117 |
| HSV1-UL30-2369 | + | UCUCCCGGGGGCGCUUGGCC | 20 | 5381 |
| HSV1-UL30-5178 | + | GUCUCCCGGGGGCGCUUGGCC | 21 | 14118 |
| HSV1-UL30-5179 | + | CGUCUCCCGGGGGCGCUUGGCC | 22 | 14119 |
| HSV1-UL30-5180 | + | GCGUCUCCCGGGGGCGCUUGGCC | 23 | 14120 |
| HSV1-UL30-5181 | + | GGCGUCUCCCGGGGGCGCUUGGCC | 24 | 14121 |
| HSV1-UL30-5182 | + | GUGGUCCGCGGAGAUGCC | 18 | 14122 |
| HSV1-UL30-5183 | + | AGUGGUCCGCGGAGAUGCC | 19 | 14123 |
| HSV1-UL30-2570 | + | AAGUGGUCCGCGGAGAUGCC | 20 | 5575 |
| HSV1-UL30-5184 | + | GAAGUGGUCCGCGGAGAUGCC | 21 | 14124 |
| HSV1-UL30-5185 | + | CGAAGUGGUCCGCGGAGAUGCC | 22 | 14125 |
| HSV1-UL30-5186 | + | UCGAAGUGGUCCGCGGAGAUGCC | 23 | 14126 |
| HSV1-UL30-5187 | + | CUCGAAGUGGUCCGCGGAGAUGCC | 24 | 14127 |
| HSV1-UL30-5188 | + | GGCUGCUCUGGGGAAUCC | 18 | 14128 |
| HSV1-UL30-5189 | + | GGGCUGCUCUGGGGAAUCC | 19 | 14129 |
| HSV1-UL30-2453 | + | GGGGCUGCUCUGGGGAAUCC | 20 | 5459 |
| HSV1-UL30-5190 | + | CGGGGCUGCUCUGGGGAAUCC | 21 | 14130 |
| HSV1-UL30-5191 | + | UCGGGGCUGCUCUGGGGAAUCC | 22 | 14131 |
| HSV1-UL30-5192 | + | CUCGGGGCUGCUCUGGGGAAUCC | 23 | 14132 |
| HSV1-UL30-5193 | + | CCUCGGGGCUGCUCUGGGGAAUCC | 24 | 14133 |
| HSV1-UL30-5194 | + | CGCAUGUCGGCCGCCUCC | 18 | 14134 |
| HSV1-UL30-5195 | + | GCGCAUGUCGGCCGCCUCC | 19 | 14135 |
| HSV1-UL30-2434 | + | CGCGCAUGUCGGCCGCCUCC | 20 | 5440 |
| HSV1-UL30-5196 | + | GCGCGCAUGUCGGCCGCCUCC | 21 | 14136 |
| HSV1-UL30-5197 | + | GGCGCGCAUGUCGGCCGCCUCC | 22 | 14137 |
| HSV1-UL30-5198 | + | GGGCGCGCAUGUCGGCCGCCUCC | 23 | 14138 |
| HSV1-UL30-5199 | + | GGGGCGCGCAUGUCGGCCGCCUCC | 24 | 14139 |
| HSV1-UL30-5200 | + | GGGCUUGGACGCGCCUCC | 18 | 14140 |
| HSV1-UL30-5201 | + | GGGGCUUGGACGCGCCUCC | 19 | 14141 |
| HSV1-UL30-2361 | + | CGGGGCUUGGACGCGCCUCC | 20 | 5373 |
| HSV1-UL30-5202 | + | GCGGGGCUUGGACGCGCCUCC | 21 | 14142 |
| HSV1-UL30-5203 | + | UGCGGGGCUUGGACGCGCCUCC | 22 | 14143 |
| HSV1-UL30-5204 | + | UUGCGGGGCUUGGACGCGCCUCC | 23 | 14144 |
| HSV1-UL30-5205 | + | CUUGCGGGGCUUGGACGCGCCUCC | 24 | 14145 |
| HSV1-UL30-5206 | + | UCCAGGAGCACGGCCUCC | 18 | 14146 |
| HSV1-UL30-5207 | + | GUCCAGGAGCACGGCCUCC | 19 | 14147 |
| HSV1-UL30-2447 | + | UGUCCAGGAGCACGGCCUCC | 20 | 5453 |
| HSV1-UL30-5208 | + | UUGUCCAGGAGCACGGCCUCC | 21 | 14148 |
| HSV1-UL30-5209 | + | CUUGUCCAGGAGCACGGCCUCC | 22 | 14149 |
| HSV1-UL30-5210 | + | GCUUGUCCAGGAGCACGGCCUCC | 23 | 14150 |
| HSV1-UL30-5211 | + | UGCUUGUCCAGGAGCACGGCCUCC | 24 | 14151 |
| HSV1-UL30-5212 | + | CGCGCCAGCCACUCCUCC | 18 | 14152 |
| HSV1-UL30-5213 | + | UCGCGCCAGCCACUCCUCC | 19 | 14153 |
| HSV1-UL30-2407 | + | GUCGCGCCAGCCACUCCUCC | 20 | 5414 |
| HSV1-UL30-5214 | + | GGUCGCGCCAGCCACUCCUCC | 21 | 14154 |
| HSV1-UL30-5215 | + | GGGUCGCGCCAGCCACUCCUCC | 22 | 14155 |
| HSV1-UL30-5216 | + | GGGGUCGCGCCAGCCACUCCUCC | 23 | 14156 |
| HSV1-UL30-5217 | + | AGGGGUCGCGCCAGCCACUCCUCC | 24 | 14157 |
| HSV1-UL30-5218 | + | GCAGGCGACGGCGUCUCC | 18 | 14158 |
| HSV1-UL30-5219 | + | GGCAGGCGACGGCGUCUCC | 19 | 14159 |
| HSV1-UL30-2365 | + | CGGCAGGCGACGGCGUCUCC | 20 | 5377 |
| HSV1-UL30-5220 | + | UCGGCAGGCGACGGCGUCUCC | 21 | 14160 |
| HSV1-UL30-5221 | + | GUCGGCAGGCGACGGCGUCUCC | 22 | 14161 |
| HSV1-UL30-5222 | + | GGUCGGCAGGCGACGGCGUCUCC | 23 | 14162 |
| HSV1-UL30-5223 | + | GGGUCGGCAGGCGACGGCGUCUCC | 24 | 14163 |
| HSV1-UL30-5224 | + | AUGCGGCCGUACCCGUCC | 18 | 14164 |
| HSV1-UL30-5225 | + | CAUGCGGCCGUACCCGUCC | 19 | 14165 |
| HSV1-UL30-2509 | + | UCAUGCGGCCGUACCCGUCC | 20 | 5515 |
| HSV1-UL30-5226 | + | UUCAUGCGGCCGUACCCGUCC | 21 | 14166 |
| HSV1-UL30-5227 | + | GUUCAUGCGGCCGUACCCGUCC | 22 | 14167 |
| HSV1-UL30-5228 | + | CGUUCAUGCGGCCGUACCCGUCC | 23 | 14168 |
| HSV1-UL30-5229 | + | CCGUUCAUGCGGCCGUACCCGUCC | 24 | 14169 |
| HSV1-UL30-5230 | + | CGCGCGGCCACGUCGUCC | 18 | 14170 |
| HSV1-UL30-5231 | + | CCGCGCGGCCACGUCGUCC | 19 | 14171 |
| HSV1-UL30-2341 | + | GCCGCGCGGCCACGUCGUCC | 20 | 5354 |
| HSV1-UL30-5232 | + | AGCCGCGCGGCCACGUCGUCC | 21 | 14172 |
| HSV1-UL30-5233 | + | GAGCCGCGCGGCCACGUCGUCC | 22 | 14173 |
| HSV1-UL30-5234 | + | GGAGCCGCGCGGCCACGUCGUCC | 23 | 14174 |
| HSV1-UL30-5235 | + | CGGAGCCGCGCGGCCACGUCGUCC | 24 | 14175 |
| HSV1-UL30-5236 | + | UCCGCCUCGAAGUGGUCC | 18 | 14176 |
| HSV1-UL30-5237 | + | CUCCGCCUCGAAGUGGUCC | 19 | 14177 |
| HSV1-UL30-2568 | + | CCUCCGCCUCGAAGUGGUCC | 20 | 5573 |
| HSV1-UL30-5238 | + | ACCUCCGCCUCGAAGUGGUCC | 21 | 14178 |
| HSV1-UL30-5239 | + | CACCUCCGCCUCGAAGUGGUCC | 22 | 14179 |
| HSV1-UL30-5240 | + | CCACCUCCGCCUCGAAGUGGUCC | 23 | 14180 |
| HSV1-UL30-5241 | + | ACCACCUCCGCCUCGAAGUGGUCC | 24 | 14181 |
| HSV1-UL30-5242 | + | GCGGCCUGCUGCUUGUCC | 18 | 14182 |
| HSV1-UL30-5243 | + | GGCGGCCUGCUGCUUGUCC | 19 | 14183 |
| HSV1-UL30-2446 | + | UGGCGGCCUGCUGCUUGUCC | 20 | 5452 |
| HSV1-UL30-5244 | + | AUGGCGGCCUGCUGCUUGUCC | 21 | 14184 |
| HSV1-UL30-5245 | + | GAUGGCGGCCUGCUGCUUGUCC | 22 | 14185 |
| HSV1-UL30-5246 | + | UGAUGGCGGCCUGCUGCUUGUCC | 23 | 14186 |
| HSV1-UL30-5247 | + | UUGAUGGCGGCCUGCUGCUUGUCC | 24 | 14187 |
| HSV1-UL30-5248 | + | AACAGCUGGCCCACCAGC | 18 | 14188 |
| HSV1-UL30-5249 | + | AAACAGCUGGCCCACCAGC | 19 | 14189 |
| HSV1-UL30-2495 | + | AAAACAGCUGGCCCACCAGC | 20 | 5501 |
| HSV1-UL30-5250 | + | AAAAACAGCUGGCCCACCAGC | 21 | 14190 |
| HSV1-UL30-5251 | + | AAAAAACAGCUGGCCCACCAGC | 22 | 14191 |
| HSV1-UL30-5252 | + | UAAAAAACAGCUGGCCCACCAGC | 23 | 14192 |
| HSV1-UL30-5253 | + | UUAAAAAACAGCUGGCCCACCAGC | 24 | 14193 |
| HSV1-UL30-5254 | + | GCGGGAUCCUCGGCCAGC | 18 | 14194 |
| HSV1-UL30-5255 | + | UGCGGGAUCCUCGGCCAGC | 19 | 14195 |
| HSV1-UL30-2356 | + | AUGCGGGAUCCUCGGCCAGC | 20 | 5368 |
| HSV1-UL30-5256 | + | UAUGCGGGAUCCUCGGCCAGC | 21 | 14196 |
| HSV1-UL30-5257 | + | GUAUGCGGGAUCCUCGGCCAGC | 22 | 14197 |
| HSV1-UL30-5258 | + | CGUAUGCGGGAUCCUCGGCCAGC | 23 | 14198 |
| HSV1-UL30-5259 | + | GCGUAUGCGGGAUCCUCGGCCAGC | 24 | 14199 |
| HSV1-UL30-5260 | + | UCCGUCAGCUUGGCCAGC | 18 | 14200 |
| HSV1-UL30-5261 | + | GUCCGUCAGCUUGGCCAGC | 19 | 14201 |
| HSV1-UL30-2512 | + | UGUCCGUCAGCUUGGCCAGC | 20 | 5518 |
| HSV1-UL30-5262 | + | AUGUCCGUCAGCUUGGCCAGC | 21 | 14202 |
| HSV1-UL30-5263 | + | AAUGUCCGUCAGCUUGGCCAGC | 22 | 14203 |
| HSV1-UL30-5264 | + | AAAUGUCCGUCAGCUUGGCCAGC | 23 | 14204 |
| HSV1-UL30-5265 | + | UAAAUGUCCGUCAGCUUGGCCAGC | 24 | 14205 |
| HSV1-UL30-5266 | + | CGGCGUUGAGCUUGUAGC | 18 | 14206 |
| HSV1-UL30-5267 | + | ACGGCGUUGAGCUUGUAGC | 19 | 14207 |
| HSV1-UL30-2503 | + | CACGGCGUUGAGCUUGUAGC | 20 | 5509 |
| HSV1-UL30-5268 | + | CCACGGCGUUGAGCUUGUAGC | 21 | 14208 |
| HSV1-UL30-5269 | + | GCCACGGCGUUGAGCUUGUAGC | 22 | 14209 |
| HSV1-UL30-5270 | + | GGCCACGGCGUUGAGCUUGUAGC | 23 | 14210 |
| HSV1-UL30-5271 | + | CGGCCACGGCGUUGAGCUUGUAGC | 24 | 14211 |
| HSV1-UL30-5272 | + | CGGGCUGCGGCCGGACGC | 18 | 14212 |
| HSV1-UL30-5273 | + | CCGGGCUGCGGCCGGACGC | 19 | 14213 |
| HSV1-UL30-2485 | + | CCCGGGCUGCGGCCGGACGC | 20 | 5491 |
| HSV1-UL30-5274 | + | UCCCGGGCUGCGGCCGGACGC | 21 | 14214 |
| HSV1-UL30-5275 | + | CUCCCGGGCUGCGGCCGGACGC | 22 | 14215 |
| HSV1-UL30-5276 | + | CCUCCCGGGCUGCGGCCGGACGC | 23 | 14216 |
| HSV1-UL30-5277 | + | UCCUCCCGGGCUGCGGCCGGACGC | 24 | 14217 |
| HSV1-UL30-5278 | + | AUGGCGAGCCAGUCCCGC | 18 | 14218 |
| HSV1-UL30-5279 | + | CAUGGCGAGCCAGUCCCGC | 19 | 14219 |
| HSV1-UL30-2457 | + | GCAUGGCGAGCCAGUCCCGC | 20 | 5463 |
| HSV1-UL30-5280 | + | CGCAUGGCGAGCCAGUCCCGC | 21 | 14220 |
| HSV1-UL30-5281 | + | UCGCAUGGCGAGCCAGUCCCGC | 22 | 14221 |
| HSV1-UL30-5282 | + | UUCGCAUGGCGAGCCAGUCCCGC | 23 | 14222 |
| HSV1-UL30-5283 | + | UUUCGCAUGGCGAGCCAGUCCCGC | 24 | 14223 |
| HSV1-UL30-5284 | + | CGGGGAGGGCAGGGCCGC | 18 | 14224 |
| HSV1-UL30-5285 | + | CCGGGGAGGGCAGGGCCGC | 19 | 14225 |
| HSV1-UL30-1125 | + | GCCGGGGAGGGCAGGGCCGC | 20 | 4063 |
| HSV1-UL30-5286 | + | GGCCGGGGAGGGCAGGGCCGC | 21 | 14226 |
| HSV1-UL30-5287 | + | UGGCCGGGGAGGGCAGGGCCGC | 22 | 14227 |
| HSV1-UL30-5288 | + | UUGGCCGGGGAGGGCAGGGCCGC | 23 | 14228 |
| HSV1-UL30-5289 | + | CUUGGCCGGGGAGGGCAGGGCCGC | 24 | 14229 |
| HSV1-UL30-5290 | + | UGCCGCGGAACGACGCGC | 18 | 14230 |
| HSV1-UL30-5291 | + | AUGCCGCGGAACGACGCGC | 19 | 14231 |
| HSV1-UL30-2572 | + | GAUGCCGCGGAACGACGCGC | 20 | 5577 |
| HSV1-UL30-5292 | + | AGAUGCCGCGGAACGACGCGC | 21 | 14232 |
| HSV1-UL30-5293 | + | GAGAUGCCGCGGAACGACGCGC | 22 | 14233 |
| HSV1-UL30-5294 | + | GGAGAUGCCGCGGAACGACGCGC | 23 | 14234 |
| HSV1-UL30-5295 | + | CGGAGAUGCCGCGGAACGACGCGC | 24 | 14235 |
| HSV1-UL30-3992 | + | GGCGUCCAUAAACCGCGC | 18 | 12932 |
| HSV1-UL30-3993 | + | UGGCGUCCAUAAACCGCGC | 19 | 12933 |
| HSV1-UL30-3994 | + | AUGGCGUCCAUAAACCGCGC | 20 | 12934 |
| HSV1-UL30-3995 | + | GAUGGCGUCCAUAAACCGCGC | 21 | 12935 |
| HSV1-UL30-3996 | + | UGAUGGCGUCCAUAAACCGCGC | 22 | 12936 |
| HSV1-UL30-3997 | + | GUGAUGGCGUCCAUAAACCGCGC | 23 | 12937 |
| HSV1-UL30-3998 | + | UGUGAUGGCGUCCAUAAACCGCGC | 24 | 12938 |
| HSV1-UL30-5296 | + | GGGGCAGAAACAGCGCGC | 18 | 14236 |
| HSV1-UL30-5297 | + | GGGGGCAGAAACAGCGCGC | 19 | 14237 |
| HSV1-UL30-2422 | + | GGGGGGCAGAAACAGCGCGC | 20 | 5429 |
| HSV1-UL30-5298 | + | UGGGGGGCAGAAACAGCGCGC | 21 | 14238 |
| HSV1-UL30-5299 | + | AUGGGGGGCAGAAACAGCGCGC | 22 | 14239 |
| HSV1-UL30-5300 | + | GAUGGGGGGCAGAAACAGCGCGC | 23 | 14240 |
| HSV1-UL30-5301 | + | UGAUGGGGGGCAGAAACAGCGCGC | 24 | 14241 |
| HSV1-UL30-5302 | + | GAAGGCCGCCCAGCGCGC | 18 | 14242 |
| HSV1-UL30-5303 | + | CGAAGGCCGCCCAGCGCGC | 19 | 14243 |
| HSV1-UL30-2438 | + | UCGAAGGCCGCCCAGCGCGC | 20 | 5444 |
| HSV1-UL30-5304 | + | UUCGAAGGCCGCCCAGCGCGC | 21 | 14244 |
| HSV1-UL30-5305 | + | GUUCGAAGGCCGCCCAGCGCGC | 22 | 14245 |
| HSV1-UL30-5306 | + | UGUUCGAAGGCCGCCCAGCGCGC | 23 | 14246 |
| HSV1-UL30-5307 | + | CUGUUCGAAGGCCGCCCAGCGCGC | 24 | 14247 |
| HSV1-UL30-5308 | + | UCCCCGUAGAUGAUGCGC | 18 | 14248 |
| HSV1-UL30-5309 | + | GUCCCCGUAGAUGAUGCGC | 19 | 14249 |
| HSV1-UL30-2426 | + | UGUCCCCGUAGAUGAUGCGC | 20 | 5432 |
| HSV1-UL30-5310 | + | GUGUCCCCGUAGAUGAUGCGC | 21 | 14250 |
| HSV1-UL30-5311 | + | CGUGUCCCCGUAGAUGAUGCGC | 22 | 14251 |
| HSV1-UL30-5312 | + | CCGUGUCCCCGUAGAUGAUGCGC | 23 | 14252 |
| HSV1-UL30-5313 | + | UCCGUGUCCCCGUAGAUGAUGCGC | 24 | 14253 |
| HSV1-UL30-5314 | + | GGGAUUCGGGGAGGUCGC | 18 | 14254 |
| HSV1-UL30-5315 | + | UGGGAUUCGGGGAGGUCGC | 19 | 14255 |
| HSV1-UL30-2532 | + | GUGGGAUUCGGGGAGGUCGC | 20 | 5538 |
| HSV1-UL30-5316 | + | GGUGGGAUUCGGGGAGGUCGC | 21 | 14256 |
| HSV1-UL30-5317 | + | AGGUGGGAUUCGGGGAGGUCGC | 22 | 14257 |
| HSV1-UL30-5318 | + | CAGGUGGGAUUCGGGGAGGUCGC | 23 | 14258 |
| HSV1-UL30-5319 | + | UCAGGUGGGAUUCGGGGAGGUCGC | 24 | 14259 |
| HSV1-UL30-5320 | + | CGCCUCCGGGAAAUCGGC | 18 | 14260 |
| HSV1-UL30-5321 | + | CCGCCUCCGGGAAAUCGGC | 19 | 14261 |
| HSV1-UL30-2435 | + | GCCGCCUCCGGGAAAUCGGC | 20 | 5441 |
| HSV1-UL30-5322 | + | GGCCGCCUCCGGGAAAUCGGC | 21 | 14262 |
| HSV1-UL30-5323 | + | CGGCCGCCUCCGGGAAAUCGGC | 22 | 14263 |
| HSV1-UL30-5324 | + | UCGGCCGCCUCCGGGAAAUCGGC | 23 | 14264 |
| HSV1-UL30-5325 | + | GUCGGCCGCCUCCGGGAAAUCGGC | 24 | 14265 |
| HSV1-UL30-5326 | + | AUGCGCAUGGAAUAGGGC | 18 | 14266 |
| HSV1-UL30-5327 | + | GAUGCGCAUGGAAUAGGGC | 19 | 14267 |
| HSV1-UL30-2429 | + | UGAUGCGCAUGGAAUAGGGC | 20 | 5435 |
| HSV1-UL30-5328 | + | AUGAUGCGCAUGGAAUAGGGC | 21 | 14268 |
| HSV1-UL30-5329 | + | GAUGAUGCGCAUGGAAUAGGGC | 22 | 14269 |
| HSV1-UL30-5330 | + | AGAUGAUGCGCAUGGAAUAGGGC | 23 | 14270 |
| HSV1-UL30-5331 | + | UAGAUGAUGCGCAUGGAAUAGGGC | 24 | 14271 |
| HSV1-UL30-5332 | + | AAUUCCAGAACCACGGGC | 18 | 14272 |
| HSV1-UL30-5333 | + | GAAUUCCAGAACCACGGGC | 19 | 14273 |
| HSV1-UL30-2524 | + | CGAAUUCCAGAACCACGGGC | 20 | 5530 |
| HSV1-UL30-5334 | + | UCGAAUUCCAGAACCACGGGC | 21 | 14274 |
| HSV1-UL30-5335 | + | GUCGAAUUCCAGAACCACGGGC | 22 | 14275 |
| HSV1-UL30-5336 | + | UGUCGAAUUCCAGAACCACGGGC | 23 | 14276 |
| HSV1-UL30-5337 | + | CUGUCGAAUUCCAGAACCACGGGC | 24 | 14277 |
| HSV1-UL30-5338 | + | ACCAGCAGCUUGCGGGGC | 18 | 14278 |
| HSV1-UL30-5339 | + | CACCAGCAGCUUGCGGGGC | 19 | 14279 |
| HSV1-UL30-2359 | + | ACACCAGCAGCUUGCGGGGC | 20 | 5371 |
| HSV1-UL30-5340 | + | GACACCAGCAGCUUGCGGGGC | 21 | 14280 |
| HSV1-UL30-5341 | + | GGACACCAGCAGCUUGCGGGGC | 22 | 14281 |
| HSV1-UL30-5342 | + | CGGACACCAGCAGCUUGCGGGGC | 23 | 14282 |
| HSV1-UL30-5343 | + | UCGGACACCAGCAGCUUGCGGGGC | 24 | 14283 |
| HSV1-UL30-5344 | + | CUCCCGGGGGCGCUUGGC | 18 | 14284 |
| HSV1-UL30-5345 | + | UCUCCCGGGGGCGCUUGGC | 19 | 14285 |
| HSV1-UL30-2368 | + | GUCUCCCGGGGGCGCUUGGC | 20 | 5380 |
| HSV1-UL30-5346 | + | CGUCUCCCGGGGGCGCUUGGC | 21 | 14286 |
| HSV1-UL30-5347 | + | GCGUCUCCCGGGGGCGCUUGGC | 22 | 14287 |
| HSV1-UL30-5348 | + | GGCGUCUCCCGGGGGCGCUUGGC | 23 | 14288 |
| HSV1-UL30-5349 | + | CGGCGUCUCCCGGGGGCGCUUGGC | 24 | 14289 |
| HSV1-UL30-5350 | + | CCGAUCACCCCGCGUUGC | 18 | 14290 |
| HSV1-UL30-5351 | + | GCCGAUCACCCCGCGUUGC | 19 | 14291 |
| HSV1-UL30-2497 | + | CGCCGAUCACCCCGCGUUGC | 20 | 5503 |
| HSV1-UL30-5352 | + | UCGCCGAUCACCCCGCGUUGC | 21 | 14292 |
| HSV1-UL30-5353 | + | CUCGCCGAUCACCCCGCGUUGC | 22 | 14293 |
| HSV1-UL30-5354 | + | ACUCGCCGAUCACCCCGCGUUGC | 23 | 14294 |
| HSV1-UL30-5355 | + | UACUCGCCGAUCACCCCGCGUUGC | 24 | 14295 |
| HSV1-UL30-5356 | + | GCCACUGCGUCGGCCCUC | 18 | 14296 |
| HSV1-UL30-5357 | + | CGCCACUGCGUCGGCCCUC | 19 | 14297 |
| HSV1-UL30-2466 | + | GCGCCACUGCGUCGGCCCUC | 20 | 5472 |
| HSV1-UL30-5358 | + | UGCGCCACUGCGUCGGCCCUC | 21 | 14298 |
| HSV1-UL30-5359 | + | GUGCGCCACUGCGUCGGCCCUC | 22 | 14299 |
| HSV1-UL30-5360 | + | GGUGCGCCACUGCGUCGGCCCUC | 23 | 14300 |
| HSV1-UL30-5361 | + | AGGUGCGCCACUGCGUCGGCCCUC | 24 | 14301 |
| HSV1-UL30-5362 | + | GCGCAUGUCGGCCGCCUC | 18 | 14302 |
| HSV1-UL30-5363 | + | CGCGCAUGUCGGCCGCCUC | 19 | 14303 |
| HSV1-UL30-2433 | + | GCGCGCAUGUCGGCCGCCUC | 20 | 5439 |
| HSV1-UL30-5364 | + | GGCGCGCAUGUCGGCCGCCUC | 21 | 14304 |
| HSV1-UL30-5365 | + | GGGCGCGCAUGUCGGCCGCCUC | 22 | 14305 |
| HSV1-UL30-5366 | + | GGGGCGCGCAUGUCGGCCGCCUC | 23 | 14306 |
| HSV1-UL30-5367 | + | GGGGGCGCGCAUGUCGGCCGCCUC | 24 | 14307 |
| HSV1-UL30-5368 | + | GGGGCUUGGACGCGCCUC | 18 | 14308 |
| HSV1-UL30-5369 | + | CGGGGCUUGGACGCGCCUC | 19 | 14309 |
| HSV1-UL30-2360 | + | GCGGGGCUUGGACGCGCCUC | 20 | 5372 |
| HSV1-UL30-5370 | + | UGCGGGGCUUGGACGCGCCUC | 21 | 14310 |
| HSV1-UL30-5371 | + | UUGCGGGGCUUGGACGCGCCUC | 22 | 14311 |
| HSV1-UL30-5372 | + | CUUGCGGGGCUUGGACGCGCCUC | 23 | 14312 |
| HSV1-UL30-5373 | + | GCUUGCGGGGCUUGGACGCGCCUC | 24 | 14313 |
| HSV1-UL30-5374 | + | UGGCCGCUCCUCGUCCUC | 18 | 14314 |
| HSV1-UL30-5375 | + | CUGGCCGCUCCUCGUCCUC | 19 | 14315 |
| HSV1-UL30-2483 | + | UCUGGCCGCUCCUCGUCCUC | 20 | 5489 |
| HSV1-UL30-5376 | + | CUCUGGCCGCUCCUCGUCCUC | 21 | 14316 |
| HSV1-UL30-5377 | + | CCUCUGGCCGCUCCUCGUCCUC | 22 | 14317 |
| HSV1-UL30-5378 | + | UCCUCUGGCCGCUCCUCGUCCUC | 23 | 14318 |
| HSV1-UL30-5379 | + | CUCCUCUGGCCGCUCCUCGUCCUC | 24 | 14319 |
| HSV1-UL30-5380 | + | GGCGGCGGCGUCUAGCUC | 18 | 14320 |
| HSV1-UL30-5381 | + | GGGCGGCGGCGUCUAGCUC | 19 | 14321 |
| HSV1-UL30-2382 | + | GGGGCGGCGGCGUCUAGCUC | 20 | 5389 |
| HSV1-UL30-5382 | + | UGGGGCGGCGGCGUCUAGCUC | 21 | 14322 |
| HSV1-UL30-5383 | + | CUGGGGCGGCGGCGUCUAGCUC | 22 | 14323 |
| HSV1-UL30-5384 | + | CCUGGGGCGGCGGCGUCUAGCUC | 23 | 14324 |
| HSV1-UL30-5385 | + | CCCUGGGGCGGCGGCGUCUAGCUC | 24 | 14325 |
| HSV1-UL30-5386 | + | GCGUUGAGCUUGUAGCUC | 18 | 14326 |
| HSV1-UL30-5387 | + | GGCGUUGAGCUUGUAGCUC | 19 | 14327 |
| HSV1-UL30-2504 | + | CGGCGUUGAGCUUGUAGCUC | 20 | 5510 |
| HSV1-UL30-5388 | + | ACGGCGUUGAGCUUGUAGCUC | 21 | 14328 |
| HSV1-UL30-5389 | + | CACGGCGUUGAGCUUGUAGCUC | 22 | 14329 |
| HSV1-UL30-5390 | + | CCACGGCGUUGAGCUUGUAGCUC | 23 | 14330 |
| HSV1-UL30-5391 | + | GCCACGGCGUUGAGCUUGUAGCUC | 24 | 14331 |
| HSV1-UL30-5392 | + | CUAACGCGGCGGCCGCUC | 18 | 14332 |
| HSV1-UL30-5393 | + | GCUAACGCGGCGGCCGCUC | 19 | 14333 |
| HSV1-UL30-2410 | + | GGCUAACGCGGCGGCCGCUC | 20 | 5417 |
| HSV1-UL30-5394 | + | CGGCUAACGCGGCGGCCGCUC | 21 | 14334 |
| HSV1-UL30-5395 | + | UCGGCUAACGCGGCGGCCGCUC | 22 | 14335 |
| HSV1-UL30-5396 | + | CUCGGCUAACGCGGCGGCCGCUC | 23 | 14336 |
| HSV1-UL30-5397 | + | GCUCGGCUAACGCGGCGGCCGCUC | 24 | 14337 |
| HSV1-UL30-5398 | + | GCCUCCUCGGGGCUGCUC | 18 | 14338 |
| HSV1-UL30-5399 | + | GGCCUCCUCGGGGCUGCUC | 19 | 14339 |
| HSV1-UL30-2450 | + | CGGCCUCCUCGGGGCUGCUC | 20 | 5456 |
| HSV1-UL30-5400 | + | ACGGCCUCCUCGGGGCUGCUC | 21 | 14340 |
| HSV1-UL30-5401 | + | CACGGCCUCCUCGGGGCUGCUC | 22 | 14341 |
| HSV1-UL30-5402 | + | GCACGGCCUCCUCGGGGCUGCUC | 23 | 14342 |
| HSV1-UL30-5403 | + | AGCACGGCCUCCUCGGGGCUGCUC | 24 | 14343 |
| HSV1-UL30-5404 | + | AAACAGGAGGACGUGCUC | 18 | 14344 |
| HSV1-UL30-5405 | + | AAAACAGGAGGACGUGCUC | 19 | 14345 |
| HSV1-UL30-2538 | + | GAAAACAGGAGGACGUGCUC | 20 | 5543 |
| HSV1-UL30-5406 | + | CGAAAACAGGAGGACGUGCUC | 21 | 14346 |
| HSV1-UL30-5407 | + | GCGAAAACAGGAGGACGUGCUC | 22 | 14347 |
| HSV1-UL30-5408 | + | AGCGAAAACAGGAGGACGUGCUC | 23 | 14348 |
| HSV1-UL30-5409 | + | GAGCGAAAACAGGAGGACGUGCUC | 24 | 14349 |
| HSV1-UL30-5410 | + | UGCGCUCGCAGAGAUCUC | 18 | 14350 |
| HSV1-UL30-5411 | + | AUGC GCUC GCAGAGAUCUC | 19 | 14351 |
| HSV1-UL30-2577 | + | CAUGCGCUCGCAGAGAUCUC | 20 | 5582 |
| HSV1-UL30-5412 | + | CCAUGCGCUCGCAGAGAUCUC | 21 | 14352 |
| HSV1-UL30-5413 | + | GC CAUGC GCUC GCAGAGAUCUC | 22 | 14353 |
| HSV1-UL30-5414 | + | GGC CAUGC GCUC GCAGAGAUCUC | 23 | 14354 |
| HSV1-UL30-5415 | + | CGGCCAUGCGCUCGCAGAGAUCUC | 24 | 14355 |
| HSV1-UL30-5416 | + | GGCAGGCGACGGCGUCUC | 18 | 14356 |
| HSV1-UL30-5417 | + | CGGCAGGCGACGGCGUCUC | 19 | 14357 |
| HSV1-UL30-2364 | + | UCGGCAGGCGACGGCGUCUC | 20 | 5376 |
| HSV1-UL30-5418 | + | GUCGGCAGGCGACGGCGUCUC | 21 | 14358 |
| HSV1-UL30-5419 | + | GGUCGGCAGGCGACGGCGUCUC | 22 | 14359 |
| HSV1-UL30-5420 | + | GGGUCGGCAGGCGACGGCGUCUC | 23 | 14360 |
| HSV1-UL30-5421 | + | GGGGUCGGCAGGCGACGGCGUCUC | 24 | 14361 |
| HSV1-UL30-5422 | + | CAUGCGGCCGUACCCGUC | 18 | 14362 |
| HSV1-UL30-5423 | + | UCAUGCGGCCGUACCCGUC | 19 | 14363 |
| HSV1-UL30-2508 | + | UUCAUGCGGCCGUACCCGUC | 20 | 5514 |
| HSV1-UL30-5424 | + | GUUCAUGCGGCCGUACCCGUC | 21 | 14364 |
| HSV1-UL30-5425 | + | CGUUCAUGCGGCCGUACCCGUC | 22 | 14365 |
| HSV1-UL30-5426 | + | CCGUUCAUGCGGCCGUACCCGUC | 23 | 14366 |
| HSV1-UL30-5427 | + | GCCGUUCAUGCGGCCGUACCCGUC | 24 | 14367 |
| HSV1-UL30-5428 | + | CCGCGCGGCCACGUCGUC | 18 | 14368 |
| HSV1-UL30-5429 | + | GCCGCGCGGCCACGUCGUC | 19 | 14369 |
| HSV1-UL30-2340 | + | AGCCGCGCGGCCACGUCGUC | 20 | 5353 |
| HSV1-UL30-5430 | + | GAGCCGCGCGGCCACGUCGUC | 21 | 14370 |
| HSV1-UL30-5431 | + | GGAGCCGCGCGGCCACGUCGUC | 22 | 14371 |
| HSV1-UL30-5432 | + | CGGAGCCGCGCGGCCACGUCGUC | 23 | 14372 |
| HSV1-UL30-5433 | + | CCGGAGCCGCGCGGCCACGUCGUC | 24 | 14373 |
| HSV1-UL30-5434 | + | AUGAUGUUGUACCCGGUC | 18 | 14374 |
| HSV1-UL30-5435 | + | GAUGAUGUUGUACCCGGUC | 19 | 14375 |
| HSV1-UL30-2515 | + | UGAUGAUGUUGUACCCGGUC | 20 | 5521 |
| HSV1-UL30-5436 | + | UUGAUGAUGUUGUACCCGGUC | 21 | 14376 |
| HSV1-UL30-5437 | + | GUUGAUGAUGUUGUACCCGGUC | 22 | 14377 |
| HSV1-UL30-5438 | + | AGUUGAUGAUGUUGUACCCGGUC | 23 | 14378 |
| HSV1-UL30-5439 | + | AAGUUGAUGAUGUUGUACCCGGUC | 24 | 14379 |
| HSV1-UL30-5440 | + | GCCCCGAACCCUGCGGUC | 18 | 14380 |
| HSV1-UL30-5441 | + | CGCCCCGAACCCUGCGGUC | 19 | 14381 |
| HSV1-UL30-2338 | + | CCGCCCCGAACCCUGCGGUC | 20 | 5351 |
| HSV1-UL30-5442 | + | ACCGCCCCGAACCCUGCGGUC | 21 | 14382 |
| HSV1-UL30-5443 | + | CACCGCCCCGAACCCUGCGGUC | 22 | 14383 |
| HSV1-UL30-5444 | + | CCACCGCCCCGAACCCUGCGGUC | 23 | 14384 |
| HSV1-UL30-5445 | + | CCCACCGCCCCGAACCCUGCGGUC | 24 | 14385 |
| HSV1-UL30-5446 | + | UACUGUUUCACAAGGGUC | 18 | 14386 |
| HSV1-UL30-5447 | + | GUACUGUUUCACAAGGGUC | 19 | 14387 |
| HSV1-UL30-2519 | + | CGUACUGUUUCACAAGGGUC | 20 | 5525 |
| HSV1-UL30-5448 | + | CCGUACUGUUUCACAAGGGUC | 21 | 14388 |
| HSV1-UL30-5449 | + | GCCGUACUGUUUCACAAGGGUC | 22 | 14389 |
| HSV1-UL30-5450 | + | GGCCGUACUGUUUCACAAGGGUC | 23 | 14390 |
| HSV1-UL30-5451 | + | GGGCCGUACUGUUUCACAAGGGUC | 24 | 14391 |
| HSV1-UL30-5452 | + | UGGACGUACUCGCGGGUC | 18 | 14392 |
| HSV1-UL30-5453 | + | GUGGACGUACUCGCGGGUC | 19 | 14393 |
| HSV1-UL30-2440 | + | CGUGGACGUACUCGCGGGUC | 20 | 5446 |
| HSV1-UL30-5454 | + | GCGUGGACGUACUCGCGGGUC | 21 | 14394 |
| HSV1-UL30-5455 | + | CGCGUGGACGUACUCGCGGGUC | 22 | 14395 |
| HSV1-UL30-5456 | + | GCGCGUGGACGUACUCGCGGGUC | 23 | 14396 |
| HSV1-UL30-5457 | + | CGCGCGUGGACGUACUCGCGGGUC | 24 | 14397 |
| HSV1-UL30-5458 | + | AAACCCGGAAGUGGGGUC | 18 | 14398 |
| HSV1-UL30-5459 | + | GAAACCCGGAAGUGGGGUC | 19 | 14399 |
| HSV1-UL30-2482 | + | UGAAACCCGGAAGUGGGGUC | 20 | 5488 |
| HSV1-UL30-5460 | + | GUGAAACCCGGAAGUGGGGUC | 21 | 14400 |
| HSV1-UL30-5461 | + | CGUGAAACCCGGAAGUGGGGUC | 22 | 14401 |
| HSV1-UL30-5462 | + | ACGUGAAACCCGGAAGUGGGGUC | 23 | 14402 |
| HSV1-UL30-5463 | + | CACGUGAAACCCGGAAGUGGGGUC | 24 | 14403 |
| HSV1-UL30-5464 | + | UCGACGUCGCUGGAUGUC | 18 | 14404 |
| HSV1-UL30-5465 | + | CUCGACGUCGCUGGAUGUC | 19 | 14405 |
| HSV1-UL30-2549 | + | ACUCGACGUCGCUGGAUGUC | 20 | 5554 |
| HSV1-UL30-5466 | + | AACUCGACGUCGCUGGAUGUC | 21 | 14406 |
| HSV1-UL30-5467 | + | AAACUCGACGUCGCUGGAUGUC | 22 | 14407 |
| HSV1-UL30-5468 | + | UAAACUCGACGUCGCUGGAUGUC | 23 | 14408 |
| HSV1-UL30-5469 | + | UUAAACUCGACGUCGCUGGAUGUC | 24 | 14409 |
| HSV1-UL30-5470 | + | GGCGGCCUGCUGCUUGUC | 18 | 14410 |
| HSV1-UL30-5471 | + | UGGCGGCCUGCUGCUUGUC | 19 | 14411 |
| HSV1-UL30-2445 | + | AUGGCGGCCUGCUGCUUGUC | 20 | 5451 |
| HSV1-UL30-5472 | + | GAUGGCGGCCUGCUGCUUGUC | 21 | 14412 |
| HSV1-UL30-5473 | + | UGAUGGCGGCCUGCUGCUUGUC | 22 | 14413 |
| HSV1-UL30-5474 | + | UUGAUGGCGGCCUGCUGCUUGUC | 23 | 14414 |
| HSV1-UL30-5475 | + | CUUGAUGGCGGCCUGCUGCUUGUC | 24 | 14415 |
| HSV1-UL30-5476 | + | GAAUUCGCUGUCGAAUUC | 18 | 14416 |
| HSV1-UL30-5477 | + | CGAAUUCGCUGUCGAAUUC | 19 | 14417 |
| HSV1-UL30-2522 | + | UCGAAUUC GCUGUC GAAUUC | 20 | 5528 |
| HSV1-UL30-5478 | + | CUCGAAUUCGCUGUCGAAUUC | 21 | 14418 |
| HSV1-UL30-5479 | + | UCUCGAAUUCGCUGUCGAAUUC | 22 | 14419 |
| HSV1-UL30-5480 | + | AUCUCGAAUUCGCUGUCGAAUUC | 23 | 14420 |
| HSV1-UL30-5481 | + | CAUCUCGAAUUCGCUGUCGAAUUC | 24 | 14421 |
| HSV1-UL30-5482 | + | CUCGUUCAGGUGGGAUUC | 18 | 14422 |
| HSV1-UL30-5483 | + | GCUCGUUCAGGUGGGAUUC | 19 | 14423 |
| HSV1-UL30-2529 | + | AGCUCGUUCAGGUGGGAUUC | 20 | 5535 |
| HSV1-UL30-5484 | + | CAGCUCGUUCAGGUGGGAUUC | 21 | 14424 |
| HSV1-UL30-5485 | + | CCAGCUCGUUCAGGUGGGAUUC | 22 | 14425 |
| HSV1-UL30-5486 | + | GCCAGCUCGUUCAGGUGGGAUUC | 23 | 14426 |
| HSV1-UL30-5487 | + | CGCCAGCUCGUUCAGGUGGGAUUC | 24 | 14427 |
| HSV1-UL30-5488 | + | UUUAUCUUGCUGCGCUUC | 18 | 14428 |
| HSV1-UL30-5489 | + | CUUUAUCUUGCUGCGCUUC | 19 | 14429 |
| HSV1-UL30-2506 | + | CCUUUAUCUUGCUGCGCUUC | 20 | 5512 |
| HSV1-UL30-5490 | + | ACCUUUAUCUUGCUGCGCUUC | 21 | 14430 |
| HSV1-UL30-5491 | + | CACCUUUAUCUUGCUGCGCUUC | 22 | 14431 |
| HSV1-UL30-5492 | + | UCACCUUUAUCUUGCUGCGCUUC | 23 | 14432 |
| HSV1-UL30-5493 | + | UUCACCUUUAUCUUGCUGCGCUUC | 24 | 14433 |
| HSV1-UL30-5494 | + | CCGCGGCACAGCACAAAG | 18 | 14434 |
| HSV1-UL30-5495 | + | GCCGCGGCACAGCACAAAG | 19 | 14435 |
| HSV1-UL30-2424 | + | GGCCGCGGCACAGCACAAAG | 20 | 5431 |
| HSV1-UL30-5496 | + | AGGCCGCGGCACAGCACAAAG | 21 | 14436 |
| HSV1-UL30-5497 | + | GAGGCCGCGGCACAGCACAAAG | 22 | 14437 |
| HSV1-UL30-5498 | + | UGAGGCCGCGGCACAGCACAAAG | 23 | 14438 |
| HSV1-UL30-5499 | + | GUGAGGCCGCGGCACAGCACAAAG | 24 | 14439 |
| HSV1-UL30-5500 | + | GGGUACAGGCUGGCAAAG | 18 | 14440 |
| HSV1-UL30-5501 | + | GGGGUACAGGCUGGCAAAG | 19 | 14441 |
| HSV1-UL30-2474 | + | UGGGGUACAGGCUGGCAAAG | 20 | 5480 |
| HSV1-UL30-5502 | + | CUGGGGUACAGGCUGGCAAAG | 21 | 14442 |
| HSV1-UL30-5503 | + | GCUGGGGUACAGGCUGGCAAAG | 22 | 14443 |
| HSV1-UL30-5504 | + | UGCUGGGGUACAGGCUGGCAAAG | 23 | 14444 |
| HSV1-UL30-5505 | + | AUGCUGGGGUACAGGCUGGCAAAG | 24 | 14445 |
| HSV1-UL30-5506 | + | GUCAGCUUGGCCAGCAAG | 18 | 14446 |
| HSV1-UL30-5507 | + | CGUCAGCUUGGCCAGCAAG | 19 | 14447 |
| HSV1-UL30-2513 | + | CCGUCAGCUUGGCCAGCAAG | 20 | 5519 |
| HSV1-UL30-5508 | + | UCCGUCAGCUUGGCCAGCAAG | 21 | 14448 |
| HSV1-UL30-5509 | + | GUCCGUCAGCUUGGCCAGCAAG | 22 | 14449 |
| HSV1-UL30-5510 | + | UGUCCGUCAGCUUGGCCAGCAAG | 23 | 14450 |
| HSV1-UL30-5511 | + | AUGUCCGUCAGCUUGGCCAGCAAG | 24 | 14451 |
| HSV1-UL30-5512 | + | GGAACCGAGCGAAAACAG | 18 | 14452 |
| HSV1-UL30-5513 | + | AGGAACCGAGCGAAAACAG | 19 | 14453 |
| HSV1-UL30-2537 | + | CAGGAACCGAGCGAAAACAG | 20 | 5542 |
| HSV1-UL30-5514 | + | GCAGGAACCGAGCGAAAACAG | 21 | 14454 |
| HSV1-UL30-5515 | + | CGCAGGAACCGAGCGAAAACAG | 22 | 14455 |
| HSV1-UL30-5516 | + | UCGCAGGAACCGAGCGAAAACAG | 23 | 14456 |
| HSV1-UL30-5517 | + | GUCGCAGGAACCGAGCGAAAACAG | 24 | 14457 |
| HSV1-UL30-5518 | + | GCACGCCGCCCCCAACAG | 18 | 14458 |
| HSV1-UL30-5519 | + | CGCACGCCGCCCCCAACAG | 19 | 14459 |
| HSV1-UL30-2348 | + | ACGCACGCCGCCCCCAACAG | 20 | 5361 |
| HSV1-UL30-5520 | + | CACGCACGCCGCCCCCAACAG | 21 | 14460 |
| HSV1-UL30-5521 | + | UCACGCACGCCGCCCCCAACAG | 22 | 14461 |
| HSV1-UL30-5522 | + | GUCACGCACGCCGCCCCCAACAG | 23 | 14462 |
| HSV1-UL30-5523 | + | UGUCACGCACGCCGCCCCCAACAG | 24 | 14463 |
| HSV1-UL30-5524 | + | AAACAGCUGGCCCACCAG | 18 | 14464 |
| HSV1-UL30-5525 | + | AAAACAGCUGGCCCACCAG | 19 | 14465 |
| HSV1-UL30-2494 | + | AAAAACAGCUGGCCCACCAG | 20 | 5500 |
| HSV1-UL30-5526 | + | AAAAAACAGCUGGCCCACCAG | 21 | 14466 |
| HSV1-UL30-5527 | + | UAAAAAACAGCUGGCCCACCAG | 22 | 14467 |
| HSV1-UL30-5528 | + | UUAAAAAACAGCUGGCCCACCAG | 23 | 14468 |
| HSV1-UL30-5529 | + | CUUAAAAAACAGCUGGCCCACCAG | 24 | 14469 |
| HSV1-UL30-5530 | + | GGUUUGAGACGGUACCAG | 18 | 14470 |
| HSV1-UL30-5531 | + | CGGUUUGAGACGGUACCAG | 19 | 14471 |
| HSV1-UL30-2554 | + | CCGGUUUGAGACGGUACCAG | 20 | 5559 |
| HSV1-UL30-5532 | + | CCCGGUUUGAGACGGUACCAG | 21 | 14472 |
| HSV1-UL30-5533 | + | GCCCGGUUUGAGACGGUACCAG | 22 | 14473 |
| HSV1-UL30-5534 | + | GGCCCGGUUUGAGACGGUACCAG | 23 | 14474 |
| HSV1-UL30-5535 | + | CGGCCCGGUUUGAGACGGUACCAG | 24 | 14475 |
| HSV1-UL30-5536 | + | GCCAGCAAGAAGGGCCAG | 18 | 14476 |
| HSV1-UL30-5537 | + | GGCCAGCAAGAAGGGCCAG | 19 | 14477 |
| HSV1-UL30-2514 | + | UGGCCAGCAAGAAGGGCCAG | 20 | 5520 |
| HSV1-UL30-5538 | + | UUGGCCAGCAAGAAGGGCCAG | 21 | 14478 |
| HSV1-UL30-5539 | + | CUUGGCCAGCAAGAAGGGCCAG | 22 | 14479 |
| HSV1-UL30-5540 | + | GCUUGGCCAGCAAGAAGGGCCAG | 23 | 14480 |
| HSV1-UL30-5541 | + | AGCUUGGCCAGCAAGAAGGGCCAG | 24 | 14481 |
| HSV1-UL30-5542 | + | GACGGCCGAGAGCUCCAG | 18 | 14482 |
| HSV1-UL30-5543 | + | CGACGGCCGAGAGCUCCAG | 19 | 14483 |
| HSV1-UL30-2492 | + | GCGACGGCCGAGAGCUCCAG | 20 | 5498 |
| HSV1-UL30-5544 | + | CGCGACGGCCGAGAGCUCCAG | 21 | 14484 |
| HSV1-UL30-5545 | + | GCGCGACGGCCGAGAGCUCCAG | 22 | 14485 |
| HSV1-UL30-5546 | + | CGCGCGACGGCCGAGAGCUCCAG | 23 | 14486 |
| HSV1-UL30-5547 | + | GCGCGCGACGGCCGAGAGCUCCAG | 24 | 14487 |
| HSV1-UL30-5548 | + | GCGGCCGUACCCGUCCAG | 18 | 14488 |
| HSV1-UL30-5549 | + | UGCGGCCGUACCCGUCCAG | 19 | 14489 |
| HSV1-UL30-2511 | + | AUGCGGCCGUACCCGUCCAG | 20 | 5517 |
| HSV1-UL30-5550 | + | CAUGCGGCCGUACCCGUCCAG | 21 | 14490 |
| HSV1-UL30-5551 | + | UCAUGCGGCCGUACCCGUCCAG | 22 | 14491 |
| HSV1-UL30-5552 | + | UUCAUGCGGCCGUACCCGUCCAG | 23 | 14492 |
| HSV1-UL30-5553 | + | GUUCAUGCGGCCGUACCCGUCCAG | 24 | 14493 |
| HSV1-UL30-5554 | + | CUUGUAAUACACCGUCAG | 18 | 14494 |
| HSV1-UL30-5555 | + | GCUUGUAAUACACCGUCAG | 19 | 14495 |
| HSV1-UL30-2394 | + | AGCUUGUAAUACACCGUCAG | 20 | 5401 |
| HSV1-UL30-5556 | + | GAGCUUGUAAUACACCGUCAG | 21 | 14496 |
| HSV1-UL30-5557 | + | UGAGCUUGUAAUACACCGUCAG | 22 | 14497 |
| HSV1-UL30-5558 | + | AUGAGCUUGUAAUACACCGUCAG | 23 | 14498 |
| HSV1-UL30-5559 | + | CAUGAGCUUGUAAUACACCGUCAG | 24 | 14499 |
| HSV1-UL30-5560 | + | GGCCGCCAGCUCGUUCAG | 18 | 14500 |
| HSV1-UL30-5561 | + | UGGCCGCCAGCUCGUUCAG | 19 | 14501 |
| HSV1-UL30-2525 | + | CUGGCCGCCAGCUCGUUCAG | 20 | 5531 |
| HSV1-UL30-5562 | + | CCUGGCCGCCAGCUCGUUCAG | 21 | 14502 |
| HSV1-UL30-5563 | + | CCCUGGCCGCCAGCUCGUUCAG | 22 | 14503 |
| HSV1-UL30-5564 | + | CCCCUGGCCGCCAGCUCGUUCAG | 23 | 14504 |
| HSV1-UL30-5565 | + | GCCCCUGGCCGCCAGCUCGUUCAG | 24 | 14505 |
| HSV1-UL30-5566 | + | AGACGCGGUAAAACAGAG | 18 | 14506 |
| HSV1-UL30-5567 | + | UAGACGCGGUAAAACAGAG | 19 | 14507 |
| HSV1-UL30-2566 | + | GUAGACGCGGUAAAACAGAG | 20 | 5571 |
| HSV1-UL30-5568 | + | CGUAGACGCGGUAAAACAGAG | 21 | 14508 |
| HSV1-UL30-5569 | + | ACGUAGACGCGGUAAAACAGAG | 22 | 14509 |
| HSV1-UL30-5570 | + | GACGUAGACGCGGUAAAACAGAG | 23 | 14510 |
| HSV1-UL30-5571 | + | GGACGUAGACGCGGUAAAACAGAG | 24 | 14511 |
| HSV1-UL30-5572 | + | GGCCCGGCGGCGUAGUAG | 18 | 14512 |
| HSV1-UL30-5573 | + | GGGCCCGGCGGCGUAGUAG | 19 | 14513 |
| HSV1-UL30-2498 | + | CGGGCCCGGCGGCGUAGUAG | 20 | 5504 |
| HSV1-UL30-5574 | + | GCGGGCCCGGCGGCGUAGUAG | 21 | 14514 |
| HSV1-UL30-5575 | + | CGCGGGCCCGGCGGCGUAGUAG | 22 | 14515 |
| HSV1-UL30-5576 | + | GCGCGGGCCCGGCGGCGUAGUAG | 23 | 14516 |
| HSV1-UL30-5577 | + | UGCGCGGGCCCGGCGGCGUAGUAG | 24 | 14517 |
| HSV1-UL30-5578 | + | CGCACGUGAGCCUUGACG | 18 | 14518 |
| HSV1-UL30-5579 | + | UCGCACGUGAGCCUUGACG | 19 | 14519 |
| HSV1-UL30-2464 | + | CUCGCACGUGAGCCUUGACG | 20 | 5470 |
| HSV1-UL30-5580 | + | UCUCGCACGUGAGCCUUGACG | 21 | 14520 |
| HSV1-UL30-5581 | + | CUCUCGCACGUGAGCCUUGACG | 22 | 14521 |
| HSV1-UL30-5582 | + | UCUCUCGCACGUGAGCCUUGACG | 23 | 14522 |
| HSV1-UL30-5583 | + | CUCUCUCGCACGUGAGCCUUGACG | 24 | 14523 |
| HSV1-UL30-5584 | + | CAUGGCGAGCCAGUCCCG | 18 | 14524 |
| HSV1-UL30-5585 | + | GCAUGGCGAGCCAGUCCCG | 19 | 14525 |
| HSV1-UL30-2456 | + | CGCAUGGCGAGCCAGUCCCG | 20 | 5462 |
| HSV1-UL30-5586 | + | UCGCAUGGCGAGCCAGUCCCG | 21 | 14526 |
| HSV1-UL30-5587 | + | UUCGCAUGGCGAGCCAGUCCCG | 22 | 14527 |
| HSV1-UL30-5588 | + | UUUCGCAUGGCGAGCCAGUCCCG | 23 | 14528 |
| HSV1-UL30-5589 | + | CUUUCGCAUGGCGAGCCAGUCCCG | 24 | 14529 |
| HSV1-UL30-5590 | + | CGGGUCGGUGAUGCGCCG | 18 | 14530 |
| HSV1-UL30-5591 | + | CCGGGUCGGUGAUGCGCCG | 19 | 14531 |
| HSV1-UL30-2400 | + | UCCGGGUCGGUGAUGCGCCG | 20 | 5407 |
| HSV1-UL30-5592 | + | CUCCGGGUCGGUGAUGCGCCG | 21 | 14532 |
| HSV1-UL30-5593 | + | UCUCCGGGUCGGUGAUGCGCCG | 22 | 14533 |
| HSV1-UL30-5594 | + | CUCUCCGGGUCGGUGAUGCGCCG | 23 | 14534 |
| HSV1-UL30-5595 | + | CCUCUCCGGGUCGGUGAUGCGCCG | 24 | 14535 |
| HSV1-UL30-5596 | + | CCGGGGAGGGCAGGGCCG | 18 | 14536 |
| HSV1-UL30-5597 | + | GCCGGGGAGGGCAGGGCCG | 19 | 14537 |
| HSV1-UL30-1124 | + | GGCCGGGGAGGGCAGGGCCG | 20 | 4062 |
| HSV1-UL30-5598 | + | UGGCCGGGGAGGGCAGGGCCG | 21 | 14538 |
| HSV1-UL30-5599 | + | UUGGCCGGGGAGGGCAGGGCCG | 22 | 14539 |
| HSV1-UL30-5600 | + | CUUGGCCGGGGAGGGCAGGGCCG | 23 | 14540 |
| HSV1-UL30-5601 | + | GCUUGGCCGGGGAGGGCAGGGCCG | 24 | 14541 |
| HSV1-UL30-5602 | + | CCCGGGGGCGCUUGGCCG | 18 | 14542 |
| HSV1-UL30-5603 | + | UCCCGGGGGCGCUUGGCCG | 19 | 14543 |
| HSV1-UL30-2370 | + | CUCCCGGGGGCGCUUGGCCG | 20 | 5382 |
| HSV1-UL30-5604 | + | UCUCCCGGGGGCGCUUGGCCG | 21 | 14544 |
| HSV1-UL30-5605 | + | GUCUCCCGGGGGCGCUUGGCCG | 22 | 14545 |
| HSV1-UL30-5606 | + | CGUCUCCCGGGGGCGCUUGGCCG | 23 | 14546 |
| HSV1-UL30-5607 | + | GCGUCUCCCGGGGGCGCUUGGCCG | 24 | 14547 |
| HSV1-UL30-5608 | + | UGGUCCGCGGAGAUGCCG | 18 | 14548 |
| HSV1-UL30-5609 | + | GUGGUCCGCGGAGAUGCCG | 19 | 14549 |
| HSV1-UL30-2571 | + | AGUGGUCCGCGGAGAUGCCG | 20 | 5576 |
| HSV1-UL30-5610 | + | AAGUGGUCCGCGGAGAUGCCG | 21 | 14550 |
| HSV1-UL30-5611 | + | GAAGUGGUCCGCGGAGAUGCCG | 22 | 14551 |
| HSV1-UL30-5612 | + | CGAAGUGGUCCGCGGAGAUGCCG | 23 | 14552 |
| HSV1-UL30-5613 | + | UCGAAGUGGUCCGCGGAGAUGCCG | 24 | 14553 |
| HSV1-UL30-5614 | + | GCGCCAGCCACUCCUCCG | 18 | 14554 |
| HSV1-UL30-5615 | + | CGCGCCAGCCACUCCUCCG | 19 | 14555 |
| HSV1-UL30-2408 | + | UCGCGCCAGCCACUCCUCCG | 20 | 5415 |
| HSV1-UL30-5616 | + | GUCGCGCCAGCCACUCCUCCG | 21 | 14556 |
| HSV1-UL30-5617 | + | GGUCGCGCCAGCCACUCCUCCG | 22 | 14557 |
| HSV1-UL30-5618 | + | GGGUCGCGCCAGCCACUCCUCCG | 23 | 14558 |
| HSV1-UL30-5619 | + | GGGGUCGCGCCAGCCACUCCUCCG | 24 | 14559 |
| HSV1-UL30-5620 | + | CCGCCUCGAAGUGGUCCG | 18 | 14560 |
| HSV1-UL30-5621 | + | UCCGCCUCGAAGUGGUCCG | 19 | 14561 |
| HSV1-UL30-2569 | + | CUCCGCCUCGAAGUGGUCCG | 20 | 5574 |
| HSV1-UL30-5622 | + | CCUCCGCCUCGAAGUGGUCCG | 21 | 14562 |
| HSV1-UL30-5623 | + | ACCUCCGCCUCGAAGUGGUCCG | 22 | 14563 |
| HSV1-UL30-5624 | + | CACCUCCGCCUCGAAGUGGUCCG | 23 | 14564 |
| HSV1-UL30-5625 | + | CCACCUCCGCCUCGAAGUGGUCCG | 24 | 14565 |
| HSV1-UL30-5626 | + | GGGGAGGGCAGGGCCGCG | 18 | 14566 |
| HSV1-UL30-5627 | + | CGGGGAGGGCAGGGCCGCG | 19 | 14567 |
| HSV1-UL30-1126 | + | CCGGGGAGGGCAGGGCCGCG | 20 | 4064 |
| HSV1-UL30-5628 | + | GCCGGGGAGGGCAGGGCCGCG | 21 | 14568 |
| HSV1-UL30-5629 | + | GGCCGGGGAGGGCAGGGCCGCG | 22 | 14569 |
| HSV1-UL30-5630 | + | UGGCCGGGGAGGGCAGGGCCGCG | 23 | 14570 |
| HSV1-UL30-5631 | + | UUGGCCGGGGAGGGCAGGGCCGCG | 24 | 14571 |
| HSV1-UL30-4083 | + | GCGUCCAUAAACCGCGCG | 18 | 13023 |
| HSV1-UL30-4084 | + | GGCGUCCAUAAACCGCGCG | 19 | 13024 |
| HSV1-UL30-2857 | + | UGGCGUCCAUAAACCGCGCG | 20 | 11797 |
| HSV1-UL30-4085 | + | AUGGCGUCCAUAAACCGCGCG | 21 | 13025 |
| HSV1-UL30-4086 | + | GAUGGCGUCCAUAAACCGCGCG | 22 | 13026 |
| HSV1-UL30-4087 | + | UGAUGGCGUCCAUAAACCGCGCG | 23 | 13027 |
| HSV1-UL30-4088 | + | GUGAUGGCGUCCAUAAACCGCGCG | 24 | 13028 |
| HSV1-UL30-5632 | + | GGACGGGACCUGCGCGCG | 18 | 14572 |
| HSV1-UL30-5633 | + | UGGACGGGACCUGCGCGCG | 19 | 14573 |
| HSV1-UL30-2392 | + | AUGGACGGGACCUGCGCGCG | 20 | 5399 |
| HSV1-UL30-5634 | + | GAUGGACGGGACCUGCGCGCG | 21 | 14574 |
| HSV1-UL30-5635 | + | UGAUGGACGGGACCUGCGCGCG | 22 | 14575 |
| HSV1-UL30-5636 | + | UUGAUGGACGGGACCUGCGCGCG | 23 | 14576 |
| HSV1-UL30-5637 | + | CUUGAUGGACGGGACCUGCGCGCG | 24 | 14577 |
| HSV1-UL30-5638 | + | CGCGCCCGGGGACUCGCG | 18 | 14578 |
| HSV1-UL30-5639 | + | ACGCGCCCGGGGACUCGCG | 19 | 14579 |
| HSV1-UL30-2575 | + | GACGCGCCCGGGGACUCGCG | 20 | 5580 |
| HSV1-UL30-5640 | + | CGACGCGCCCGGGGACUCGCG | 21 | 14580 |
| HSV1-UL30-5641 | + | ACGACGCGCCCGGGGACUCGCG | 22 | 14581 |
| HSV1-UL30-5642 | + | AACGACGCGCCCGGGGACUCGCG | 23 | 14582 |
| HSV1-UL30-5643 | + | GAACGACGCGCCCGGGGACUCGCG | 24 | 14583 |
| HSV1-UL30-5644 | + | GGCGGCGUCUAGCUCGCG | 18 | 14584 |
| HSV1-UL30-5645 | + | CGGCGGCGUCUAGCUCGCG | 19 | 14585 |
| HSV1-UL30-2384 | + | GCGGCGGCGUCUAGCUCGCG | 20 | 5391 |
| HSV1-UL30-5646 | + | GGCGGCGGCGUCUAGCUCGCG | 21 | 14586 |
| HSV1-UL30-5647 | + | GGGCGGCGGCGUCUAGCUCGCG | 22 | 14587 |
| HSV1-UL30-5648 | + | GGGGCGGCGGCGUCUAGCUCGCG | 23 | 14588 |
| HSV1-UL30-5649 | + | UGGGGCGGCGGCGUCUAGCUCGCG | 24 | 14589 |
| HSV1-UL30-5650 | + | ACGGCUUCGGCCACGGCG | 18 | 14590 |
| HSV1-UL30-5651 | + | GACGGCUUCGGCCACGGCG | 19 | 14591 |
| HSV1-UL30-2502 | + | GGACGGCUUCGGCCACGGCG | 20 | 5508 |
| HSV1-UL30-5652 | + | AGGACGGCUUCGGCCACGGCG | 21 | 14592 |
| HSV1-UL30-5653 | + | CAGGACGGCUUCGGCCACGGCG | 22 | 14593 |
| HSV1-UL30-5654 | + | UCAGGACGGCUUCGGCCACGGCG | 23 | 14594 |
| HSV1-UL30-5655 | + | UUCAGGACGGCUUCGGCCACGGCG | 24 | 14595 |
| HSV1-UL30-5656 | + | UGUCUGCUCAGUUCGGCG | 18 | 14596 |
| HSV1-UL30-5657 | + | GUGUCUGCUCAGUUCGGCG | 19 | 14597 |
| HSV1-UL30-2396 | + | GGUGUCUGCUCAGUUCGGCG | 20 | 5403 |
| HSV1-UL30-5658 | + | GGGUGUCUGCUCAGUUCGGCG | 21 | 14598 |
| HSV1-UL30-5659 | + | CGGGUGUCUGCUCAGUUCGGCG | 22 | 14599 |
| HSV1-UL30-5660 | + | GCGGGUGUCUGCUCAGUUCGGCG | 23 | 14600 |
| HSV1-UL30-5661 | + | CGCGGGUGUCUGCUCAGUUCGGCG | 24 | 14601 |
| HSV1-UL30-5662 | + | GCGGCGGCGUCUAGCUCG | 18 | 14602 |
| HSV1-UL30-5663 | + | GGCGGCGGCGUCUAGCUCG | 19 | 14603 |
| HSV1-UL30-2383 | + | GGGCGGCGGCGUCUAGCUCG | 20 | 5390 |
| HSV1-UL30-5664 | + | GGGGCGGCGGCGUCUAGCUCG | 21 | 14604 |
| HSV1-UL30-5665 | + | UGGGGCGGCGGCGUCUAGCUCG | 22 | 14605 |
| HSV1-UL30-5666 | + | CUGGGGCGGCGGCGUCUAGCUCG | 23 | 14606 |
| HSV1-UL30-5667 | + | CCUGGGGCGGCGGCGUCUAGCUCG | 24 | 14607 |
| HSV1-UL30-5668 | + | GCCGCGGCCAUGCGCUCG | 18 | 14608 |
| HSV1-UL30-5669 | + | GGCCGCGGCCAUGCGCUCG | 19 | 14609 |
| HSV1-UL30-2576 | + | GGGCCGCGGCCAUGCGCUCG | 20 | 5581 |
| HSV1-UL30-5670 | + | AGGGCCGCGGCCAUGCGCUCG | 21 | 14610 |
| HSV1-UL30-5671 | + | CAGGGCCGCGGCCAUGCGCUCG | 22 | 14611 |
| HSV1-UL30-5672 | + | GCAGGGCCGCGGCCAUGCGCUCG | 23 | 14612 |
| HSV1-UL30-5673 | + | CGCAGGGCCGCGGCCAUGCGCUCG | 24 | 14613 |
| HSV1-UL30-5674 | + | GCGCUCGCAGAGAUCUCG | 18 | 14614 |
| HSV1-UL30-5675 | + | UGCGCUCGCAGAGAUCUCG | 19 | 14615 |
| HSV1-UL30-2578 | + | AUGCGCUCGCAGAGAUCUCG | 20 | 5583 |
| HSV1-UL30-5676 | + | CAUGCGCUCGCAGAGAUCUCG | 21 | 14616 |
| HSV1-UL30-5677 | + | CCAUGCGCUCGCAGAGAUCUCG | 22 | 14617 |
| HSV1-UL30-5678 | + | GCCAUGCGCUCGCAGAGAUCUCG | 23 | 14618 |
| HSV1-UL30-5679 | + | GGCCAUGCGCUCGCAGAGAUCUCG | 24 | 14619 |
| HSV1-UL30-5680 | + | GCGGUGGUGGACAGGUCG | 18 | 14620 |
| HSV1-UL30-5681 | + | GGCGGUGGUGGACAGGUCG | 19 | 14621 |
| HSV1-UL30-2540 | + | GGGCGGUGGUGGACAGGUCG | 20 | 5545 |
| HSV1-UL30-5682 | + | AGGGCGGUGGUGGACAGGUCG | 21 | 14622 |
| HSV1-UL30-5683 | + | CAGGGCGGUGGUGGACAGGUCG | 22 | 14623 |
| HSV1-UL30-5684 | + | CCAGGGCGGUGGUGGACAGGUCG | 23 | 14624 |
| HSV1-UL30-5685 | + | UCCAGGGCGGUGGUGGACAGGUCG | 24 | 14625 |
| HSV1-UL30-5686 | + | UGGGAUUCGGGGAGGUCG | 18 | 14626 |
| HSV1-UL30-5687 | + | GUGGGAUUCGGGGAGGUCG | 19 | 14627 |
| HSV1-UL30-2531 | + | GGUGGGAUUCGGGGAGGUCG | 20 | 5537 |
| HSV1-UL30-5688 | + | AGGUGGGAUUCGGGGAGGUCG | 21 | 14628 |
| HSV1-UL30-5689 | + | CAGGUGGGAUUCGGGGAGGUCG | 22 | 14629 |
| HSV1-UL30-5690 | + | UCAGGUGGGAUUCGGGGAGGUCG | 23 | 14630 |
| HSV1-UL30-5691 | + | UUCAGGUGGGAUUCGGGGAGGUCG | 24 | 14631 |
| HSV1-UL30-5692 | + | UCGUUCAGGUGGGAUUCG | 18 | 14632 |
| HSV1-UL30-5693 | + | CUCGUUCAGGUGGGAUUCG | 19 | 14633 |
| HSV1-UL30-2530 | + | GCUCGUUCAGGUGGGAUUCG | 20 | 5536 |
| HSV1-UL30-5694 | + | AGCUCGUUCAGGUGGGAUUCG | 21 | 14634 |
| HSV1-UL30-5695 | + | CAGCUCGUUCAGGUGGGAUUCG | 22 | 14635 |
| HSV1-UL30-5696 | + | CCAGCUCGUUCAGGUGGGAUUCG | 23 | 14636 |
| HSV1-UL30-5697 | + | GCCAGCUCGUUCAGGUGGGAUUCG | 24 | 14637 |
| HSV1-UL30-5698 | + | CACGCCGCCCCCAACAGG | 18 | 14638 |
| HSV1-UL30-5699 | + | GCACGCCGCCCCCAACAGG | 19 | 14639 |
| HSV1-UL30-1089 | + | CGCACGCCGCCCCCAACAGG | 20 | 4027 |
| HSV1-UL30-5700 | + | ACGCACGCCGCCCCCAACAGG | 21 | 14640 |
| HSV1-UL30-5701 | + | CACGCACGCCGCCCCCAACAGG | 22 | 14641 |
| HSV1-UL30-5702 | + | UCACGCACGCCGCCCCCAACAGG | 23 | 14642 |
| HSV1-UL30-5703 | + | GUCACGCACGCCGCCCCCAACAGG | 24 | 14643 |
| HSV1-UL30-5704 | + | AACCCGGGGUUGUCCAGG | 18 | 14644 |
| HSV1-UL30-5705 | + | GAACCCGGGGUUGUCCAGG | 19 | 14645 |
| HSV1-UL30-2559 | + | CGAACCCGGGGUUGUCCAGG | 20 | 5564 |
| HSV1-UL30-5706 | + | ACGAACCCGGGGUUGUCCAGG | 21 | 14646 |
| HSV1-UL30-5707 | + | GACGAACCCGGGGUUGUCCAGG | 22 | 14647 |
| HSV1-UL30-5708 | + | UGACGAACCCGGGGUUGUCCAGG | 23 | 14648 |
| HSV1-UL30-5709 | + | GUGACGAACCCGGGGUUGUCCAGG | 24 | 14649 |
| HSV1-UL30-5710 | + | ACUGCGUCGGCCCUCAGG | 18 | 14650 |
| HSV1-UL30-5711 | + | CACUGCGUCGGCCCUCAGG | 19 | 14651 |
| HSV1-UL30-2468 | + | CCACUGCGUCGGCCCUCAGG | 20 | 5474 |
| HSV1-UL30-5712 | + | GCCACUGCGUCGGCCCUCAGG | 21 | 14652 |
| HSV1-UL30-5713 | + | CGCCACUGCGUCGGCCCUCAGG | 22 | 14653 |
| HSV1-UL30-5714 | + | GCGCCACUGCGUCGGCCCUCAGG | 23 | 14654 |
| HSV1-UL30-5715 | + | UGCGCCACUGCGUCGGCCCUCAGG | 24 | 14655 |
| HSV1-UL30-5716 | + | GCCCGGCGGCGUAGUAGG | 18 | 14656 |
| HSV1-UL30-5717 | + | GGCCCGGCGGCGUAGUAGG | 19 | 14657 |
| HSV1-UL30-2499 | + | GGGCCCGGCGGCGUAGUAGG | 20 | 5505 |
| HSV1-UL30-5718 | + | CGGGCCCGGCGGCGUAGUAGG | 21 | 14658 |
| HSV1-UL30-5719 | + | GCGGGCCCGGCGGCGUAGUAGG | 22 | 14659 |
| HSV1-UL30-5720 | + | CGCGGGCCCGGCGGCGUAGUAGG | 23 | 14660 |
| HSV1-UL30-5721 | + | GCGCGGGCCCGGCGGCGUAGUAGG | 24 | 14661 |
| HSV1-UL30-5722 | + | GGCAACGUGCAGGCACGG | 18 | 14662 |
| HSV1-UL30-5723 | + | CGGCAACGUGCAGGCACGG | 19 | 14663 |
| HSV1-UL30-2441 | + | GCGGCAACGUGCAGGCACGG | 20 | 5447 |
| HSV1-UL30-5724 | + | CGCGGCAACGUGCAGGCACGG | 21 | 14664 |
| HSV1-UL30-5725 | + | UCGCGGCAACGUGCAGGCACGG | 22 | 14665 |
| HSV1-UL30-5726 | + | GUCGCGGCAACGUGCAGGCACGG | 23 | 14666 |
| HSV1-UL30-5727 | + | CGUCGCGGCAACGUGCAGGCACGG | 24 | 14667 |
| HSV1-UL30-5728 | + | CCGCCAAGCGCGCGACGG | 18 | 14668 |
| HSV1-UL30-5729 | + | CCCGCCAAGCGCGCGACGG | 19 | 14669 |
| HSV1-UL30-2490 | + | ACCCGCCAAGCGCGCGACGG | 20 | 5496 |
| HSV1-UL30-5730 | + | UACCCGCCAAGCGCGCGACGG | 21 | 14670 |
| HSV1-UL30-5731 | + | AUACCCGCCAAGCGCGCGACGG | 22 | 14671 |
| HSV1-UL30-5732 | + | AAUACCCGCCAAGCGCGCGACGG | 23 | 14672 |
| HSV1-UL30-5733 | + | UAAUACCCGCCAAGCGCGCGACGG | 24 | 14673 |
| HSV1-UL30-5734 | + | GGCCAUCGGGGCCGCCGG | 18 | 14674 |
| HSV1-UL30-5735 | + | AGGCCAUCGGGGCCGCCGG | 19 | 14675 |
| HSV1-UL30-2552 | + | AAGGCCAUCGGGGCCGCCGG | 20 | 5557 |
| HSV1-UL30-5736 | + | GAAGGCCAUCGGGGCCGCCGG | 21 | 14676 |
| HSV1-UL30-5737 | + | CGAAGGCCAUCGGGGCCGCCGG | 22 | 14677 |
| HSV1-UL30-5738 | + | CCGAAGGCCAUCGGGGCCGCCGG | 23 | 14678 |
| HSV1-UL30-5739 | + | CCCGAAGGCCAUCGGGGCCGCCGG | 24 | 14679 |
| HSV1-UL30-5740 | + | GGGAGGGCAGGGCCGCGG | 18 | 14680 |
| HSV1-UL30-5741 | + | GGGGAGGGCAGGGCCGCGG | 19 | 14681 |
| HSV1-UL30-1127 | + | CGGGGAGGGCAGGGCCGCGG | 20 | 4065 |
| HSV1-UL30-5742 | + | CCGGGGAGGGCAGGGCCGCGG | 21 | 14682 |
| HSV1-UL30-5743 | + | GCCGGGGAGGGCAGGGCCGCGG | 22 | 14683 |
| HSV1-UL30-5744 | + | GGCCGGGGAGGGCAGGGCCGCGG | 23 | 14684 |
| HSV1-UL30-5745 | + | UGGCCGGGGAGGGCAGGGCCGCGG | 24 | 14685 |
| HSV1-UL30-5746 | + | CGUCCUCCCGGGCUGCGG | 18 | 14686 |
| HSV1-UL30-5747 | + | UCGUCCUCCCGGGCUGCGG | 19 | 14687 |
| HSV1-UL30-2484 | + | CUCGUCCUCCCGGGCUGCGG | 20 | 5490 |
| HSV1-UL30-5748 | + | CCUCGUCCUCCCGGGCUGCGG | 21 | 14688 |
| HSV1-UL30-5749 | + | UCCUCGUCCUCCCGGGCUGCGG | 22 | 14689 |
| HSV1-UL30-5750 | + | CUCCUCGUCCUCCCGGGCUGCGG | 23 | 14690 |
| HSV1-UL30-5751 | + | GCUCCUCGUCCUCCCGGGCUGCGG | 24 | 14691 |
| HSV1-UL30-5752 | + | GCGCUUGGCCGGGGAGGG | 18 | 14692 |
| HSV1-UL30-5753 | + | GGCGCUUGGCCGGGGAGGG | 19 | 14693 |
| HSV1-UL30-2372 | + | GGGCGCUUGGCCGGGGAGGG | 20 | 5384 |
| HSV1-UL30-5754 | + | GGGGCGCUUGGCCGGGGAGGG | 21 | 14694 |
| HSV1-UL30-5755 | + | GGGGGCGCUUGGCCGGGGAGGG | 22 | 14695 |
| HSV1-UL30-5756 | + | CGGGGGCGCUUGGCCGGGGAGGG | 23 | 14696 |
| HSV1-UL30-5757 | + | CCGGGGGCGCUUGGCCGGGGAGGG | 24 | 14697 |
| HSV1-UL30-5758 | + | UACUUCUUGAUGGCCGGG | 18 | 14698 |
| HSV1-UL30-5759 | + | GUACUUCUUGAUGGCCGGG | 19 | 14699 |
| HSV1-UL30-2562 | + | CGUACUUCUUGAUGGCCGGG | 20 | 5567 |
| HSV1-UL30-5760 | + | UCGUACUUCUUGAUGGCCGGG | 21 | 14700 |
| HSV1-UL30-5761 | + | CUCGUACUUCUUGAUGGCCGGG | 22 | 14701 |
| HSV1-UL30-5762 | + | CCUCGUACUUCUUGAUGGCCGGG | 23 | 14702 |
| HSV1-UL30-5763 | + | CCCUCGUACUUCUUGAUGGCCGGG | 24 | 14703 |
| HSV1-UL30-5764 | + | CGGGGGCGCUUGGCCGGG | 18 | 14704 |
| HSV1-UL30-5765 | + | CCGGGGGCGCUUGGCCGGG | 19 | 14705 |
| HSV1-UL30-2371 | + | CCCGGGGGCGCUUGGCCGGG | 20 | 5383 |
| HSV1-UL30-5766 | + | UCCCGGGGGCGCUUGGCCGGG | 21 | 14706 |
| HSV1-UL30-5767 | + | CUCCCGGGGGCGCUUGGCCGGG | 22 | 14707 |
| HSV1-UL30-5768 | + | UCUCCCGGGGGCGCUUGGCCGGG | 23 | 14708 |
| HSV1-UL30-5769 | + | GUCUCCCGGGGGCGCUUGGCCGGG | 24 | 14709 |
| HSV1-UL30-5770 | + | GGAGGGCAGGGCCGCGGG | 18 | 14710 |
| HSV1-UL30-5771 | + | GGGAGGGCAGGGCCGCGGG | 19 | 14711 |
| HSV1-UL30-1128 | + | GGGGAGGGCAGGGCCGCGGG | 20 | 4066 |
| HSV1-UL30-5772 | + | CGGGGAGGGCAGGGCCGCGGG | 21 | 14712 |
| HSV1-UL30-5773 | + | CCGGGGAGGGCAGGGCCGCGGG | 22 | 14713 |
| HSV1-UL30-5774 | + | GCCGGGGAGGGCAGGGCCGCGGG | 23 | 14714 |
| HSV1-UL30-5775 | + | GGCCGGGGAGGGCAGGGCCGCGGG | 24 | 14715 |
| HSV1-UL30-5776 | + | CGCCUGCAGUCCCUCGGG | 18 | 14716 |
| HSV1-UL30-5777 | + | ACGCCUGCAGUCCCUCGGG | 19 | 14717 |
| HSV1-UL30-2405 | + | AACGCCUGCAGUCCCUCGGG | 20 | 5412 |
| HSV1-UL30-5778 | + | GAACGCCUGCAGUCCCUCGGG | 21 | 14718 |
| HSV1-UL30-5779 | + | CGAACGCCUGCAGUCCCUCGGG | 22 | 14719 |
| HSV1-UL30-5780 | + | CCGAACGCCUGCAGUCCCUCGGG | 23 | 14720 |
| HSV1-UL30-5781 | + | CCCGAACGCCUGCAGUCCCUCGGG | 24 | 14721 |
| HSV1-UL30-5782 | + | GGCAGGGCCGCGGGGGGG | 18 | 14722 |
| HSV1-UL30-5783 | + | GGGCAGGGCCGCGGGGGGG | 19 | 14723 |
| HSV1-UL30-2379 | + | AGGGCAGGGCCGCGGGGGGG | 20 | 5386 |
| HSV1-UL30-5784 | + | GAGGGCAGGGCCGCGGGGGGG | 21 | 14724 |
| HSV1-UL30-5785 | + | GGAGGGCAGGGCCGCGGGGGGG | 22 | 14725 |
| HSV1-UL30-5786 | + | GGGAGGGCAGGGCCGCGGGGGGG | 23 | 14726 |
| HSV1-UL30-5787 | + | GGGGAGGGCAGGGCCGCGGGGGGG | 24 | 14727 |
| HSV1-UL30-5788 | + | CUCGAGUUUGAUGGGGGG | 18 | 14728 |
| HSV1-UL30-5789 | + | ACUCGAGUUUGAUGGGGGG | 19 | 14729 |
| HSV1-UL30-2421 | + | CACUCGAGUUUGAUGGGGGG | 20 | 5428 |
| HSV1-UL30-5790 | + | GCACUCGAGUUUGAUGGGGGG | 21 | 14730 |
| HSV1-UL30-5791 | + | CGCACUCGAGUUUGAUGGGGGG | 22 | 14731 |
| HSV1-UL30-5792 | + | UCGCACUCGAGUUUGAUGGGGGG | 23 | 14732 |
| HSV1-UL30-5793 | + | UUCGCACUCGAGUUUGAUGGGGGG | 24 | 14733 |
| HSV1-UL30-5794 | + | GGAUCCGGUCCUUGAUGG | 18 | 14734 |
| HSV1-UL30-5795 | + | GGGAUCCGGUCCUUGAUGG | 19 | 14735 |
| HSV1-UL30-2390 | + | CGGGAUCCGGUCCUUGAUGG | 20 | 5397 |
| HSV1-UL30-5796 | + | ACGGGAUCCGGUCCUUGAUGG | 21 | 14736 |
| HSV1-UL30-5797 | + | UACGGGAUCCGGUCCUUGAUGG | 22 | 14737 |
| HSV1-UL30-5798 | + | GUACGGGAUCCGGUCCUUGAUGG | 23 | 14738 |
| HSV1-UL30-5799 | + | CGUACGGGAUCCGGUCCUUGAUGG | 24 | 14739 |
| HSV1-UL30-5800 | + | CCUCGUACUUCUUGAUGG | 18 | 14740 |
| HSV1-UL30-5801 | + | CCCUCGUACUUCUUGAUGG | 19 | 14741 |
| HSV1-UL30-2561 | + | ACCCUCGUACUUCUUGAUGG | 20 | 5566 |
| HSV1-UL30-5802 | + | CACCCUCGUACUUCUUGAUGG | 21 | 14742 |
| HSV1-UL30-5803 | + | CCACCCUCGUACUUCUUGAUGG | 22 | 14743 |
| HSV1-UL30-5804 | + | CCCACCCUCGUACUUCUUGAUGG | 23 | 14744 |
| HSV1-UL30-5805 | + | CCCCACCCUCGUACUUCUUGAUGG | 24 | 14745 |
| HSV1-UL30-5806 | + | GCCGCCCCCAACAGGUGG | 18 | 14746 |
| HSV1-UL30-5807 | + | CGCCGCCCCCAACAGGUGG | 19 | 14747 |
| HSV1-UL30-2351 | + | ACGCCGCCCCCAACAGGUGG | 20 | 5363 |
| HSV1-UL30-5808 | + | CACGCCGCCCCCAACAGGUGG | 21 | 14748 |
| HSV1-UL30-5809 | + | GCACGCCGCCCCCAACAGGUGG | 22 | 14749 |
| HSV1-UL30-5810 | + | CGCACGCCGCCCCCAACAGGUGG | 23 | 14750 |
| HSV1-UL30-5811 | + | ACGCACGCCGCCCCCAACAGGUGG | 24 | 14751 |
| HSV1-UL30-5812 | + | UCUCCCGGGGGCGCUUGG | 18 | 14752 |
| HSV1-UL30-5813 | + | GUCUCCCGGGGGCGCUUGG | 19 | 14753 |
| HSV1-UL30-2367 | + | CGUCUCCCGGGGGCGCUUGG | 20 | 5379 |
| HSV1-UL30-5814 | + | GCGUCUCCCGGGGGCGCUUGG | 21 | 14754 |
| HSV1-UL30-5815 | + | GGCGUCUCCCGGGGGCGCUUGG | 22 | 14755 |
| HSV1-UL30-5816 | + | CGGCGUCUCCCGGGGGCGCUUGG | 23 | 14756 |
| HSV1-UL30-5817 | + | ACGGCGUCUCCCGGGGGCGCUUGG | 24 | 14757 |
| HSV1-UL30-5818 | + | CGGAUCUGCUUUCGCAUG | 18 | 14758 |
| HSV1-UL30-5819 | + | GCGGAUCUGCUUUCGCAUG | 19 | 14759 |
| HSV1-UL30-2455 | + | AGCGGAUCUGCUUUCGCAUG | 20 | 5461 |
| HSV1-UL30-5820 | + | GAGCGGAUCUGCUUUCGCAUG | 21 | 14760 |
| HSV1-UL30-5821 | + | CGAGCGGAUCUGCUUUCGCAUG | 22 | 14761 |
| HSV1-UL30-5822 | + | GCGAGCGGAUCUGCUUUCGCAUG | 23 | 14762 |
| HSV1-UL30-5823 | + | CGCGAGCGGAUCUGCUUUCGCAUG | 24 | 14763 |
| HSV1-UL30-5824 | + | CAGUCCCGCAGGAGGAUG | 18 | 14764 |
| HSV1-UL30-5825 | + | CCAGUCCCGCAGGAGGAUG | 19 | 14765 |
| HSV1-UL30-2459 | + | GCCAGUCCCGCAGGAGGAUG | 20 | 5465 |
| HSV1-UL30-5826 | + | AGCCAGUCCCGCAGGAGGAUG | 21 | 14766 |
| HSV1-UL30-5827 | + | GAGCCAGUCCCGCAGGAGGAUG | 22 | 14767 |
| HSV1-UL30-5828 | + | CGAGCCAGUCCCGCAGGAGGAUG | 23 | 14768 |
| HSV1-UL30-5829 | + | GCGAGCCAGUCCCGCAGGAGGAUG | 24 | 14769 |
| HSV1-UL30-5830 | + | UUGUGGGCCUGGAUGAUG | 18 | 14770 |
| HSV1-UL30-5831 | + | GUUGUGGGCCUGGAUGAUG | 19 | 14771 |
| HSV1-UL30-2472 | + | GGUUGUGGGCCUGGAUGAUG | 20 | 5478 |
| HSV1-UL30-5832 | + | AGGUUGUGGGCCUGGAUGAUG | 21 | 14772 |
| HSV1-UL30-5833 | + | CAGGUUGUGGGCCUGGAUGAUG | 22 | 14773 |
| HSV1-UL30-5834 | + | ACAGGUUGUGGGCCUGGAUGAUG | 23 | 14774 |
| HSV1-UL30-5835 | + | CACAGGUUGUGGGCCUGGAUGAUG | 24 | 14775 |
| HSV1-UL30-5836 | + | UCGCACUCGAGUUUGAUG | 18 | 14776 |
| HSV1-UL30-5837 | + | UUCGCACUCGAGUUUGAUG | 19 | 14777 |
| HSV1-UL30-2420 | + | UUUCGCACUCGAGUUUGAUG | 20 | 5427 |
| HSV1-UL30-5838 | + | UUUUCGCACUCGAGUUUGAUG | 21 | 14778 |
| HSV1-UL30-5839 | + | CUUUUCGCACUCGAGUUUGAUG | 22 | 14779 |
| HSV1-UL30-5840 | + | UCUUUUCGCACUCGAGUUUGAUG | 23 | 14780 |
| HSV1-UL30-5841 | + | GUCUUUUCGCACUCGAGUUUGAUG | 24 | 14781 |
| HSV1-UL30-5842 | + | UCCCGCAGGAGGAUGCUG | 18 | 14782 |
| HSV1-UL30-5843 | + | GUCCCGCAGGAGGAUGCUG | 19 | 14783 |
| HSV1-UL30-2461 | + | AGUCCCGCAGGAGGAUGCUG | 20 | 5467 |
| HSV1-UL30-5844 | + | CAGUCCCGCAGGAGGAUGCUG | 21 | 14784 |
| HSV1-UL30-5845 | + | CCAGUCCCGCAGGAGGAUGCUG | 22 | 14785 |
| HSV1-UL30-5846 | + | GCCAGUCCCGCAGGAGGAUGCUG | 23 | 14786 |
| HSV1-UL30-5847 | + | AGCCAGUCCCGCAGGAGGAUGCUG | 24 | 14787 |
| HSV1-UL30-5848 | + | GCCCUCAGGGAGAGCGUG | 18 | 14788 |
| HSV1-UL30-5849 | + | GGCCCUCAGGGAGAGCGUG | 19 | 14789 |
| HSV1-UL30-2469 | + | CGGCCCUCAGGGAGAGCGUG | 20 | 5475 |
| HSV1-UL30-5850 | + | UCGGCCCUCAGGGAGAGCGUG | 21 | 14790 |
| HSV1-UL30-5851 | + | GUCGGCCCUCAGGGAGAGCGUG | 22 | 14791 |
| HSV1-UL30-5852 | + | CGUCGGCCCUCAGGGAGAGCGUG | 23 | 14792 |
| HSV1-UL30-5853 | + | GCGUCGGCCCUCAGGGAGAGCGUG | 24 | 14793 |
| HSV1-UL30-5854 | + | CGGUACCAGCCGAAGGUG | 18 | 14794 |
| HSV1-UL30-5855 | + | ACGGUACCAGCCGAAGGUG | 19 | 14795 |
| HSV1-UL30-2555 | + | GACGGUACCAGCCGAAGGUG | 20 | 5560 |
| HSV1-UL30-5856 | + | AGACGGUACCAGCCGAAGGUG | 21 | 14796 |
| HSV1-UL30-5857 | + | GAGACGGUACCAGCCGAAGGUG | 22 | 14797 |
| HSV1-UL30-5858 | + | UGAGACGGUACCAGCCGAAGGUG | 23 | 14798 |
| HSV1-UL30-5859 | + | UUGAGACGGUACCAGCCGAAGGUG | 24 | 14799 |
| HSV1-UL30-5860 | + | ACGUGCUCCAGGGCGGUG | 18 | 14800 |
| HSV1-UL30-5861 | + | GACGUGCUCCAGGGCGGUG | 19 | 14801 |
| HSV1-UL30-2539 | + | GGACGUGCUCCAGGGCGGUG | 20 | 5544 |
| HSV1-UL30-5862 | + | AGGACGUGCUCCAGGGCGGUG | 21 | 14802 |
| HSV1-UL30-5863 | + | GAGGACGUGCUCCAGGGCGGUG | 22 | 14803 |
| HSV1-UL30-5864 | + | GGAGGACGUGCUCCAGGGCGGUG | 23 | 14804 |
| HSV1-UL30-5865 | + | AGGAGGACGUGCUCCAGGGCGGUG | 24 | 14805 |
| HSV1-UL30-5866 | + | ACCAGAUCCACGCCCUUG | 18 | 14806 |
| HSV1-UL30-5867 | + | CACCAGAUCCACGCCCUUG | 19 | 14807 |
| HSV1-UL30-2414 | + | GCACCAGAUCCACGCCCUUG | 20 | 5421 |
| HSV1-UL30-5868 | + | CGCACCAGAUCCACGCCCUUG | 21 | 14808 |
| HSV1-UL30-5869 | + | GCGCACCAGAUCCACGCCCUUG | 22 | 14809 |
| HSV1-UL30-5870 | + | UGCGCACCAGAUCCACGCCCUUG | 23 | 14810 |
| HSV1-UL30-5871 | + | UUGCGCACCAGAUCCACGCCCUUG | 24 | 14811 |
| HSV1-UL30-5872 | + | UCUCGCACGUGAGCCUUG | 18 | 14812 |
| HSV1-UL30-5873 | + | CUCUCGCACGUGAGCCUUG | 19 | 14813 |
| HSV1-UL30-2463 | + | UCUCUCGCACGUGAGCCUUG | 20 | 5469 |
| HSV1-UL30-5874 | + | CUCUCUCGCACGUGAGCCUUG | 21 | 14814 |
| HSV1-UL30-5875 | + | GCUCUCUCGCACGUGAGCCUUG | 22 | 14815 |
| HSV1-UL30-5876 | + | GGCUCUCUCGCACGUGAGCCUUG | 23 | 14816 |
| HSV1-UL30-5877 | + | AGGCUCUCUCGCACGUGAGCCUUG | 24 | 14817 |
| HSV1-UL30-5878 | + | UACGGGAUCCGGUCCUUG | 18 | 14818 |
| HSV1-UL30-5879 | + | GUACGGGAUCCGGUCCUUG | 19 | 14819 |
| HSV1-UL30-2389 | + | CGUACGGGAUCCGGUCCUUG | 20 | 5396 |
| HSV1-UL30-5880 | + | ACGUACGGGAUCCGGUCCUUG | 21 | 14820 |
| HSV1-UL30-5881 | + | CACGUACGGGAUCCGGUCCUUG | 22 | 14821 |
| HSV1-UL30-5882 | + | UCACGUACGGGAUCCGGUCCUUG | 23 | 14822 |
| HSV1-UL30-5883 | + | AUCACGUACGGGAUCCGGUCCUUG | 24 | 14823 |
| HSV1-UL30-5884 | + | UCGGACACCAGCAGCUUG | 18 | 14824 |
| HSV1-UL30-5885 | + | CUCGGACACCAGCAGCUUG | 19 | 14825 |
| HSV1-UL30-2358 | + | GCUCGGACACCAGCAGCUUG | 20 | 5370 |
| HSV1-UL30-5886 | + | AGCUCGGACACCAGCAGCUUG | 21 | 14826 |
| HSV1-UL30-5887 | + | CAGCUCGGACACCAGCAGCUUG | 22 | 14827 |
| HSV1-UL30-5888 | + | CCAGCUCGGACACCAGCAGCUUG | 23 | 14828 |
| HSV1-UL30-5889 | + | GCCAGCUCGGACACCAGCAGCUUG | 24 | 14829 |
| HSV1-UL30-5890 | + | GCGAUCAGCAGCAGCUUG | 18 | 14830 |
| HSV1-UL30-5891 | + | GGCGAUCAGCAGCAGCUUG | 19 | 14831 |
| HSV1-UL30-2415 | + | UGGCGAUCAGCAGCAGCUUG | 20 | 5422 |
| HSV1-UL30-5892 | + | UUGGCGAUCAGCAGCAGCUUG | 21 | 14832 |
| HSV1-UL30-5893 | + | CUUGGCGAUCAGCAGCAGCUUG | 22 | 14833 |
| HSV1-UL30-5894 | + | UCUUGGCGAUCAGCAGCAGCUUG | 23 | 14834 |
| HSV1-UL30-5895 | + | UUCUUGGCGAUCAGCAGCAGCUUG | 24 | 14835 |
| HSV1-UL30-5896 | + | UUUUC GCACUC GAGUUUG | 18 | 14836 |
| HSV1-UL30-5897 | + | CUUUUCGCACUCGAGUUUG | 19 | 14837 |
| HSV1-UL30-2417 | + | UCUUUUCGCACUCGAGUUUG | 20 | 5424 |
| HSV1-UL30-5898 | + | GUCUUUUCGCACUCGAGUUUG | 21 | 14838 |
| HSV1-UL30-5899 | + | CGUCUUUUCGCACUCGAGUUUG | 22 | 14839 |
| HSV1-UL30-5900 | + | ACGUCUUUUCGCACUCGAGUUUG | 23 | 14840 |
| HSV1-UL30-5901 | + | AAC GUCUUUUC GCACUC GAGUUUG | 24 | 14841 |
| HSV1-UL30-5902 | + | GGAUGUCCCGAAGGCCAU | 18 | 14842 |
| HSV1-UL30-5903 | + | UGGAUGUCCCGAAGGCCAU | 19 | 14843 |
| HSV1-UL30-2551 | + | CUGGAUGUCCCGAAGGCCAU | 20 | 5556 |
| HSV1-UL30-5904 | + | GCUGGAUGUCCCGAAGGCCAU | 21 | 14844 |
| HSV1-UL30-5905 | + | CGCUGGAUGUCCCGAAGGCCAU | 22 | 14845 |
| HSV1-UL30-5906 | + | UCGCUGGAUGUCCCGAAGGCCAU | 23 | 14846 |
| HSV1-UL30-5907 | + | GUCGCUGGAUGUCCCGAAGGCCAU | 24 | 14847 |
| HSV1-UL30-5908 | + | AGCUCGUUCAGGUGGGAU | 18 | 14848 |
| HSV1-UL30-5909 | + | CAGCUCGUUCAGGUGGGAU | 19 | 14849 |
| HSV1-UL30-2527 | + | CCAGCUCGUUCAGGUGGGAU | 20 | 5533 |
| HSV1-UL30-5910 | + | GCCAGCUCGUUCAGGUGGGAU | 21 | 14850 |
| HSV1-UL30-5911 | + | CGCCAGCUCGUUCAGGUGGGAU | 22 | 14851 |
| HSV1-UL30-5912 | + | CCGCCAGCUCGUUCAGGUGGGAU | 23 | 14852 |
| HSV1-UL30-5913 | + | GCCGCCAGCUCGUUCAGGUGGGAU | 24 | 14853 |
| HSV1-UL30-5914 | + | UUCGCACUCGAGUUUGAU | 18 | 14854 |
| HSV1-UL30-5915 | + | UUUCGCACUCGAGUUUGAU | 19 | 14855 |
| HSV1-UL30-2419 | + | UUUUCGCACUCGAGUUUGAU | 20 | 5426 |
| HSV1-UL30-5916 | + | CUUUUCGCACUCGAGUUUGAU | 21 | 14856 |
| HSV1-UL30-5917 | + | UCUUUUCGCACUCGAGUUUGAU | 22 | 14857 |
| HSV1-UL30-5918 | + | GUCUUUUCGCACUCGAGUUUGAU | 23 | 14858 |
| HSV1-UL30-5919 | + | CGUCUUUUCGCACUCGAGUUUGAU | 24 | 14859 |
| HSV1-UL30-5920 | + | CCGUGGGCAAUGGCGUAU | 18 | 14860 |
| HSV1-UL30-5921 | + | GCCGUGGGCAAUGGCGUAU | 19 | 14861 |
| HSV1-UL30-2354 | + | CGCCGUGGGCAAUGGCGUAU | 20 | 5366 |
| HSV1-UL30-5922 | + | ACGCCGUGGGCAAUGGCGUAU | 21 | 14862 |
| HSV1-UL30-5923 | + | GACGCCGUGGGCAAUGGCGUAU | 22 | 14863 |
| HSV1-UL30-5924 | + | CGACGCCGUGGGCAAUGGCGUAU | 23 | 14864 |
| HSV1-UL30-5925 | + | GCGACGCCGUGGGCAAUGGCGUAU | 24 | 14865 |
| HSV1-UL30-5926 | + | UGUACCCGGUCACGAACU | 18 | 14866 |
| HSV1-UL30-5927 | + | UUGUACCCGGUCACGAACU | 19 | 14867 |
| HSV1-UL30-2517 | + | GUUGUACCCGGUCACGAACU | 20 | 5523 |
| HSV1-UL30-5928 | + | UGUUGUACCCGGUCACGAACU | 21 | 14868 |
| HSV1-UL30-5929 | + | AUGUUGUACCCGGUCACGAACU | 22 | 14869 |
| HSV1-UL30-5930 | + | GAUGUUGUACCCGGUCACGAACU | 23 | 14870 |
| HSV1-UL30-5931 | + | UGAUGUUGUACCCGGUCACGAACU | 24 | 14871 |
| HSV1-UL30-5932 | + | UCCGGGGGGUGCCACACU | 18 | 14872 |
| HSV1-UL30-5933 | + | GUCCGGGGGGUGCCACACU | 19 | 14873 |
| HSV1-UL30-2343 | + | CGUCCGGGGGGUGCCACACU | 20 | 5356 |
| HSV1-UL30-5934 | + | UCGUCCGGGGGGUGCCACACU | 21 | 14874 |
| HSV1-UL30-5935 | + | GUCGUCCGGGGGGUGCCACACU | 22 | 14875 |
| HSV1-UL30-5936 | + | CGUCGUCCGGGGGGUGCCACACU | 23 | 14876 |
| HSV1-UL30-5937 | + | ACGUCGUCCGGGGGGUGCCACACU | 24 | 14877 |
| HSV1-UL30-5938 | + | CGCCACUGCGUCGGCCCU | 18 | 14878 |
| HSV1-UL30-5939 | + | GCGCCACUGCGUCGGCCCU | 19 | 14879 |
| HSV1-UL30-2465 | + | UGCGCCACUGCGUCGGCCCU | 20 | 5471 |
| HSV1-UL30-5940 | + | GUGCGCCACUGCGUCGGCCCU | 21 | 14880 |
| HSV1-UL30-5941 | + | GGUGCGCCACUGCGUCGGCCCU | 22 | 14881 |
| HSV1-UL30-5942 | + | AGGUGCGCCACUGCGUCGGCCCU | 23 | 14882 |
| HSV1-UL30-5943 | + | CAGGUGCGCCACUGCGUCGGCCCU | 24 | 14883 |
| HSV1-UL30-5944 | + | CGCGCAUGUCGGCCGCCU | 18 | 14884 |
| HSV1-UL30-5945 | + | GCGCGCAUGUCGGCCGCCU | 19 | 14885 |
| HSV1-UL30-2432 | + | GGCGCGCAUGUCGGCCGCCU | 20 | 5438 |
| HSV1-UL30-5946 | + | GGGCGCGCAUGUCGGCCGCCU | 21 | 14886 |
| HSV1-UL30-5947 | + | GGGGCGCGCAUGUCGGCCGCCU | 22 | 14887 |
| HSV1-UL30-5948 | + | GGGGGCGCGCAUGUCGGCCGCCU | 23 | 14888 |
| HSV1-UL30-5949 | + | CGGGGGCGCGCAUGUCGGCCGCCU | 24 | 14889 |
| HSV1-UL30-5950 | + | CCAGGAGCACGGCCUCCU | 18 | 14890 |
| HSV1-UL30-5951 | + | UCCAGGAGCACGGCCUCCU | 19 | 14891 |
| HSV1-UL30-2448 | + | GUCCAGGAGCACGGCCUCCU | 20 | 5454 |
| HSV1-UL30-5952 | + | UGUCCAGGAGCACGGCCUCCU | 21 | 14892 |
| HSV1-UL30-5953 | + | UUGUCCAGGAGCACGGCCUCCU | 22 | 14893 |
| HSV1-UL30-5954 | + | CUUGUCCAGGAGCACGGCCUCCU | 23 | 14894 |
| HSV1-UL30-5955 | + | GCUUGUCCAGGAGCACGGCCUCCU | 24 | 14895 |
| HSV1-UL30-5956 | + | CGACAGCACACGCCCGCU | 18 | 14896 |
| HSV1-UL30-5957 | + | ACGACAGCACACGCCCGCU | 19 | 14897 |
| HSV1-UL30-2563 | + | UACGACAGCACACGCCCGCU | 20 | 5568 |
| HSV1-UL30-5958 | + | GUACGACAGCACACGCCCGCU | 21 | 14898 |
| HSV1-UL30-5959 | + | GGUACGACAGCACACGCCCGCU | 22 | 14899 |
| HSV1-UL30-5960 | + | AGGUACGACAGCACACGCCCGCU | 23 | 14900 |
| HSV1-UL30-5961 | + | CAGGUACGACAGCACACGCCCGCU | 24 | 14901 |
| HSV1-UL30-5962 | + | GCUAACGCGGCGGCCGCU | 18 | 14902 |
| HSV1-UL30-5963 | + | GGCUAACGCGGCGGCCGCU | 19 | 14903 |
| HSV1-UL30-2409 | + | CGGCUAACGCGGCGGCCGCU | 20 | 5416 |
| HSV1-UL30-5964 | + | UCGGCUAACGCGGCGGCCGCU | 21 | 14904 |
| HSV1-UL30-5965 | + | CUCGGCUAACGCGGCGGCCGCU | 22 | 14905 |
| HSV1-UL30-5966 | + | GCUCGGCUAACGCGGCGGCCGCU | 23 | 14906 |
| HSV1-UL30-5967 | + | CGCUCGGCUAACGCGGCGGCCGCU | 24 | 14907 |
| HSV1-UL30-5968 | + | GUCCCGCAGGAGGAUGCU | 18 | 14908 |
| HSV1-UL30-5969 | + | AGUCCCGCAGGAGGAUGCU | 19 | 14909 |
| HSV1-UL30-2460 | + | CAGUCCCGCAGGAGGAUGCU | 20 | 5466 |
| HSV1-UL30-5970 | + | CCAGUCCCGCAGGAGGAUGCU | 21 | 14910 |
| HSV1-UL30-5971 | + | GCCAGUCCCGCAGGAGGAUGCU | 22 | 14911 |
| HSV1-UL30-5972 | + | AGCCAGUCCCGCAGGAGGAUGCU | 23 | 14912 |
| HSV1-UL30-5973 | + | GAGCCAGUCCCGCAGGAGGAUGCU | 24 | 14913 |
| HSV1-UL30-5974 | + | GGCCUCCUCGGGGCUGCU | 18 | 14914 |
| HSV1-UL30-5975 | + | CGGCCUCCUCGGGGCUGCU | 19 | 14915 |
| HSV1-UL30-2449 | + | ACGGCCUCCUCGGGGCUGCU | 20 | 5455 |
| HSV1-UL30-5976 | + | CACGGCCUCCUCGGGGCUGCU | 21 | 14916 |
| HSV1-UL30-5977 | + | GCACGGCCUCCUCGGGGCUGCU | 22 | 14917 |
| HSV1-UL30-5978 | + | AGCACGGCCUCCUCGGGGCUGCU | 23 | 14918 |
| HSV1-UL30-5979 | + | GAGCACGGCCUCCUCGGGGCUGCU | 24 | 14919 |
| HSV1-UL30-5980 | + | CCUCCUCGGGGCUGCUCU | 18 | 14920 |
| HSV1-UL30-5981 | + | GCCUCCUCGGGGCUGCUCU | 19 | 14921 |
| HSV1-UL30-2451 | + | GGCCUCCUCGGGGCUGCUCU | 20 | 5457 |
| HSV1-UL30-5982 | + | CGGCCUCCUCGGGGCUGCUCU | 21 | 14922 |
| HSV1-UL30-5983 | + | ACGGCCUCCUCGGGGCUGCUCU | 22 | 14923 |
| HSV1-UL30-5984 | + | CACGGCCUCCUCGGGGCUGCUCU | 23 | 14924 |
| HSV1-UL30-5985 | + | GCACGGCCUCCUCGGGGCUGCUCU | 24 | 14925 |
| HSV1-UL30-5986 | + | AUGCGCCGAUGGGCGUCU | 18 | 14926 |
| HSV1-UL30-5987 | + | GAUGCGCCGAUGGGCGUCU | 19 | 14927 |
| HSV1-UL30-2401 | + | UGAUGCGCCGAUGGGCGUCU | 20 | 5408 |
| HSV1-UL30-5988 | + | GUGAUGCGCCGAUGGGCGUCU | 21 | 14928 |
| HSV1-UL30-5989 | + | GGUGAUGCGCCGAUGGGCGUCU | 22 | 14929 |
| HSV1-UL30-5990 | + | CGGUGAUGCGCCGAUGGGCGUCU | 23 | 14930 |
| HSV1-UL30-5991 | + | UCGGUGAUGCGCCGAUGGGCGUCU | 24 | 14931 |
| HSV1-UL30-5992 | + | UCUGGGCCACGAUCACGU | 18 | 14932 |
| HSV1-UL30-5993 | + | GUCUGGGCCACGAUCACGU | 19 | 14933 |
| HSV1-UL30-2387 | + | GGUCUGGGCCACGAUCACGU | 20 | 5394 |
| HSV1-UL30-5994 | + | GGGUCUGGGCCACGAUCACGU | 21 | 14934 |
| HSV1-UL30-5995 | + | CGGGUCUGGGCCACGAUCACGU | 22 | 14935 |
| HSV1-UL30-5996 | + | GCGGGUCUGGGCCACGAUCACGU | 23 | 14936 |
| HSV1-UL30-5997 | + | CGCGGGUCUGGGCCACGAUCACGU | 24 | 14937 |
| HSV1-UL30-5998 | + | GCCGCGCGGCCACGUCGU | 18 | 14938 |
| HSV1-UL30-5999 | + | AGCCGCGCGGCCACGUCGU | 19 | 14939 |
| HSV1-UL30-2339 | + | GAGCCGCGCGGCCACGUCGU | 20 | 5352 |
| HSV1-UL30-6000 | + | GGAGCCGCGCGGCCACGUCGU | 21 | 14940 |
| HSV1-UL30-6001 | + | CGGAGCCGCGCGGCCACGUCGU | 22 | 14941 |
| HSV1-UL30-6002 | + | CCGGAGCCGCGCGGCCACGUCGU | 23 | 14942 |
| HSV1-UL30-6003 | + | UCCGGAGCCGCGCGGCCACGUCGU | 24 | 14943 |
| HSV1-UL30-6004 | + | ACGCCGCCCCCAACAGGU | 18 | 14944 |
| HSV1-UL30-6005 | + | CACGCCGCCCCCAACAGGU | 19 | 14945 |
| HSV1-UL30-2350 | + | GCACGCCGCCCCCAACAGGU | 20 | 5362 |
| HSV1-UL30-6006 | + | CGCACGCCGCCCCCAACAGGU | 21 | 14946 |
| HSV1-UL30-6007 | + | ACGCACGCCGCCCCCAACAGGU | 22 | 14947 |
| HSV1-UL30-6008 | + | CACGCACGCCGCCCCCAACAGGU | 23 | 14948 |
| HSV1-UL30-6009 | + | UCACGCACGCCGCCCCCAACAGGU | 24 | 14949 |
| HSV1-UL30-6010 | + | GUGUUGUUCCGGCCCGGU | 18 | 14950 |
| HSV1-UL30-6011 | + | CGUGUUGUUCCGGCCCGGU | 19 | 14951 |
| HSV1-UL30-2553 | + | GCGUGUUGUUCCGGCCCGGU | 20 | 5558 |
| HSV1-UL30-6012 | + | AGCGUGUUGUUCCGGCCCGGU | 21 | 14952 |
| HSV1-UL30-6013 | + | UAGCGUGUUGUUCCGGCCCGGU | 22 | 14953 |
| HSV1-UL30-6014 | + | CUAGCGUGUUGUUCCGGCCCGGU | 23 | 14954 |
| HSV1-UL30-6015 | + | GCUAGCGUGUUGUUCCGGCCCGGU | 24 | 14955 |
| HSV1-UL30-6016 | + | UCUGCUCAGUUCGGCGGU | 18 | 14956 |
| HSV1-UL30-6017 | + | GUCUGCUCAGUUCGGCGGU | 19 | 14957 |
| HSV1-UL30-2397 | + | UGUCUGCUCAGUUCGGCGGU | 20 | 5404 |
| HSV1-UL30-6018 | + | GUGUCUGCUCAGUUCGGCGGU | 21 | 14958 |
| HSV1-UL30-6019 | + | GGUGUCUGCUCAGUUCGGCGGU | 22 | 14959 |
| HSV1-UL30-6020 | + | GGGUGUCUGCUCAGUUCGGCGGU | 23 | 14960 |
| HSV1-UL30-6021 | + | CGGGUGUCUGCUCAGUUCGGCGGU | 24 | 14961 |
| HSV1-UL30-6022 | + | CGCCCCGAACCCUGCGGU | 18 | 14962 |
| HSV1-UL30-6023 | + | CCGCCCCGAACCCUGCGGU | 19 | 14963 |
| HSV1-UL30-2337 | + | ACCGCCCCGAACCCUGCGGU | 20 | 5350 |
| HSV1-UL30-6024 | + | CACCGCCCCGAACCCUGCGGU | 21 | 14964 |
| HSV1-UL30-6025 | + | CCACCGCCCCGAACCCUGCGGU | 22 | 14965 |
| HSV1-UL30-6026 | + | CCCACCGCCCCGAACCCUGCGGU | 23 | 14966 |
| HSV1-UL30-6027 | + | ACCCACCGCCCCGAACCCUGCGGU | 24 | 14967 |
| HSV1-UL30-6028 | + | CUCCUCUACCUCGCGGGU | 18 | 14968 |
| HSV1-UL30-6029 | + | UCUCCUCUACCUCGCGGGU | 19 | 14969 |
| HSV1-UL30-2386 | + | GUCUCCUCUACCUCGCGGGU | 20 | 5393 |
| HSV1-UL30-6030 | + | CGUCUCCUCUACCUCGCGGGU | 21 | 14970 |
| HSV1-UL30-6031 | + | CCGUCUCCUCUACCUCGCGGGU | 22 | 14971 |
| HSV1-UL30-6032 | + | ACCGUCUCCUCUACCUCGCGGGU | 23 | 14972 |
| HSV1-UL30-6033 | + | GACCGUCUCCUCUACCUCGCGGGU | 24 | 14973 |
| HSV1-UL30-6034 | + | AAAUCGGCCAGGAGCUGU | 18 | 14974 |
| HSV1-UL30-6035 | + | GAAAUCGGCCAGGAGCUGU | 19 | 14975 |
| HSV1-UL30-2437 | + | GGAAAUCGGCCAGGAGCUGU | 20 | 5443 |
| HSV1-UL30-6036 | + | GGGAAAUCGGCCAGGAGCUGU | 21 | 14976 |
| HSV1-UL30-6037 | + | CGGGAAAUCGGCCAGGAGCUGU | 22 | 14977 |
| HSV1-UL30-6038 | + | CCGGGAAAUCGGCCAGGAGCUGU | 23 | 14978 |
| HSV1-UL30-6039 | + | UCCGGGAAAUCGGCCAGGAGCUGU | 24 | 14979 |
| HSV1-UL30-6040 | + | GCUCGUUCAGGUGGGAUU | 18 | 14980 |
| HSV1-UL30-6041 | + | AGCUCGUUCAGGUGGGAUU | 19 | 14981 |
| HSV1-UL30-2528 | + | CAGCUCGUUCAGGUGGGAUU | 20 | 5534 |
| HSV1-UL30-6042 | + | CCAGCUCGUUCAGGUGGGAUU | 21 | 14982 |
| HSV1-UL30-6043 | + | GCCAGCUCGUUCAGGUGGGAUU | 22 | 14983 |
| HSV1-UL30-6044 | + | CGCCAGCUCGUUCAGGUGGGAUU | 23 | 14984 |
| HSV1-UL30-6045 | + | CCGCCAGCUCGUUCAGGUGGGAUU | 24 | 14985 |
| HSV1-UL30-6046 | + | CCGGGGGGUGCCACACUU | 18 | 14986 |
| HSV1-UL30-6047 | + | UCCGGGGGGUGCCACACUU | 19 | 14987 |
| HSV1-UL30-2344 | + | GUCCGGGGGGUGCCACACUU | 20 | 5357 |
| HSV1-UL30-6048 | + | CGUCCGGGGGGUGCCACACUU | 21 | 14988 |
| HSV1-UL30-6049 | + | UCGUCCGGGGGGUGCCACACUU | 22 | 14989 |
| HSV1-UL30-6050 | + | GUCGUCCGGGGGGUGCCACACUU | 23 | 14990 |
| HSV1-UL30-6051 | + | CGUCGUCCGGGGGGUGCCACACUU | 24 | 14991 |
| HSV1-UL30-6052 | + | CGGCCACCCGGUGGCCUU | 18 | 14992 |
| HSV1-UL30-6053 | + | ACGGCCACCCGGUGGCCUU | 19 | 14993 |
| HSV1-UL30-2580 | + | AACGGCCACCCGGUGGCCUU | 20 | 5585 |
| HSV1-UL30-6054 | + | GAACGGCCACCCGGUGGCCUU | 21 | 14994 |
| HSV1-UL30-6055 | + | UGAACGGCCACCCGGUGGCCUU | 22 | 14995 |
| HSV1-UL30-6056 | + | GUGAACGGCCACCCGGUGGCCUU | 23 | 14996 |
| HSV1-UL30-6057 | + | CGUGAACGGCCACCCGGUGGCCUU | 24 | 14997 |
| HSV1-UL30-6058 | + | GUCCUUCUUCUUGUCCUU | 18 | 14998 |
| HSV1-UL30-6059 | + | GGUCCUUCUUCUUGUCCUU | 19 | 14999 |
| HSV1-UL30-2501 | + | AGGUCCUUCUUCUUGUCCUU | 20 | 5507 |
| HSV1-UL30-6060 | + | CAGGUCCUUCUUCUUGUCCUU | 21 | 15000 |
| HSV1-UL30-6061 | + | UCAGGUCCUUCUUCUUGUCCUU | 22 | 15001 |
| HSV1-UL30-6062 | + | CUCAGGUCCUUCUUCUUGUCCUU | 23 | 15002 |
| HSV1-UL30-6063 | + | GCUCAGGUCCUUCUUCUUGUCCUU | 24 | 15003 |
| HSV1-UL30-6064 | + | CUCGGACACCAGCAGCUU | 18 | 15004 |
| HSV1-UL30-6065 | + | GCUCGGACACCAGCAGCUU | 19 | 15005 |
| HSV1-UL30-2357 | + | AGCUCGGACACCAGCAGCUU | 20 | 5369 |
| HSV1-UL30-6066 | + | CAGCUCGGACACCAGCAGCUU | 21 | 15006 |
| HSV1-UL30-6067 | + | CCAGCUCGGACACCAGCAGCUU | 22 | 15007 |
| HSV1-UL30-6068 | + | GCCAGCUCGGACACCAGCAGCUU | 23 | 15008 |
| HSV1-UL30-6069 | + | GGCCAGCUCGGACACCAGCAGCUU | 24 | 15009 |
| HSV1-UL30-6070 | + | CUUUAUCUUGCUGCGCUU | 18 | 15010 |
| HSV1-UL30-6071 | + | CCUUUAUCUUGCUGCGCUU | 19 | 15011 |
| HSV1-UL30-2505 | + | ACCUUUAUCUUGCUGCGCUU | 20 | 5511 |
| HSV1-UL30-6072 | + | CACCUUUAUCUUGCUGCGCUU | 21 | 15012 |
| HSV1-UL30-6073 | + | UCACCUUUAUCUUGCUGCGCUU | 22 | 15013 |
| HSV1-UL30-6074 | + | UUCACCUUUAUCUUGCUGCGCUU | 23 | 15014 |
| HSV1-UL30-6075 | + | GUUCACCUUUAUCUUGCUGCGCUU | 24 | 15015 |
| HSV1-UL30-6076 | + | CGUGCUGAAGCACAGGUU | 18 | 15016 |
| HSV1-UL30-6077 | + | GCGUGCUGAAGCACAGGUU | 19 | 15017 |
| HSV1-UL30-2470 | + | AGCGUGCUGAAGCACAGGUU | 20 | 5476 |
| HSV1-UL30-6078 | + | GAGCGUGCUGAAGCACAGGUU | 21 | 15018 |
| HSV1-UL30-6079 | + | AGAGCGUGCUGAAGCACAGGUU | 22 | 15019 |
| HSV1-UL30-6080 | + | GAGAGCGUGCUGAAGCACAGGUU | 23 | 15020 |
| HSV1-UL30-6081 | + | GGAGAGCGUGCUGAAGCACAGGUU | 24 | 15021 |
| HSV1-UL30-6082 | + | GAAGUAAUAGUCCGUGUU | 18 | 15022 |
| HSV1-UL30-6083 | + | AGAAGUAAUAGUCCGUGUU | 19 | 15023 |
| HSV1-UL30-2352 | + | GAGAAGUAAUAGUCCGUGUU | 20 | 5364 |
| HSV1-UL30-6084 | + | GGAGAAGUAAUAGUCCGUGUU | 21 | 15024 |
| HSV1-UL30-6085 | + | GGGAGAAGUAAUAGUCCGUGUU | 22 | 15025 |
| HSV1-UL30-6086 | + | UGGGAGAAGUAAUAGUCCGUGUU | 23 | 15026 |
| HSV1-UL30-6087 | + | GUGGGAGAAGUAAUAGUCCGUGUU | 24 | 15027 |
| HSV1-UL30-6088 | - | GGCUGGUACCGUCUCAAA | 18 | 15028 |
| HSV1-UL30-6089 | - | CGGCUGGUACCGUCUCAAA | 19 | 15029 |
| HSV1-UL30-1803 | - | UCGGCUGGUACCGUCUCAAA | 20 | 4853 |
| HSV1-UL30-6090 | - | UUCGGCUGGUACCGUCUCAAA | 21 | 15030 |
| HSV1-UL30-6091 | - | CUUCGGCUGGUACCGUCUCAAA | 22 | 15031 |
| HSV1-UL30-6092 | - | CCUUCGGCUGGUACCGUCUCAAA | 23 | 15032 |
| HSV1-UL30-6093 | - | ACCUUCGGCUGGUACCGUCUCAAA | 24 | 15033 |
| HSV1-UL30-6094 | - | UUUGUCCUCACCGCCGAA | 18 | 15034 |
| HSV1-UL30-6095 | - | CUUUGUCCUCACCGCCGAA | 19 | 15035 |
| HSV1-UL30-2009 | - | ACUUUGUCCUCACCGCCGAA | 20 | 5042 |
| HSV1-UL30-6096 | - | GACUUUGUCCUCACCGCCGAA | 21 | 15036 |
| HSV1-UL30-6097 | - | GGACUUUGUCCUCACCGCCGAA | 22 | 15037 |
| HSV1-UL30-6098 | - | AGGACUUUGUCCUCACCGCCGAA | 23 | 15038 |
| HSV1-UL30-6099 | - | CAGGACUUUGUCCUCACCGCCGAA | 24 | 15039 |
| HSV1-UL30-6100 | - | ACCGCGUCUACGUCCGAA | 18 | 15040 |
| HSV1-UL30-6101 | - | UACCGCGUCUACGUCCGAA | 19 | 15041 |
| HSV1-UL30-1795 | - | UUACCGCGUCUACGUCCGAA | 20 | 4845 |
| HSV1-UL30-6102 | - | UUUACCGCGUCUACGUCCGAA | 21 | 15042 |
| HSV1-UL30-6103 | - | UUUUACCGCGUCUACGUCCGAA | 22 | 15043 |
| HSV1-UL30-6104 | - | GUUUUACCGCGUCUACGUCCGAA | 23 | 15044 |
| HSV1-UL30-6105 | - | UGUUUUACCGCGUCUACGUCCGAA | 24 | 15045 |
| HSV1-UL30-6106 | - | AGUACUUUUACAUGAACA | 18 | 15046 |
| HSV1-UL30-6107 | - | CAGUACUUUUACAUGAACA | 19 | 15047 |
| HSV1-UL30-1780 | - | GCAGUACUUUUACAUGAACA | 20 | 4830 |
| HSV1-UL30-6108 | - | GGCAGUACUUUUACAUGAACA | 21 | 15048 |
| HSV1-UL30-6109 | - | CGGCAGUACUUUUACAUGAACA | 22 | 15049 |
| HSV1-UL30-6110 | - | GCGGCAGUACUUUUACAUGAACA | 23 | 15050 |
| HSV1-UL30-6111 | - | CGCGGCAGUACUUUUACAUGAACA | 24 | 15051 |
| HSV1-UL30-6112 | - | CAGGCACGUGGGGUACCA | 18 | 15052 |
| HSV1-UL30-6113 | - | GCAGGCACGUGGGGUACCA | 19 | 15053 |
| HSV1-UL30-1932 | - | GGCAGGCACGUGGGGUACCA | 20 | 4965 |
| HSV1-UL30-6114 | - | CGGCAGGCACGUGGGGUACCA | 21 | 15054 |
| HSV1-UL30-6115 | - | CCGGCAGGCACGUGGGGUACCA | 22 | 15055 |
| HSV1-UL30-6116 | - | GCCGGCAGGCACGUGGGGUACCA | 23 | 15056 |
| HSV1-UL30-6117 | - | CGCCGGCAGGCACGUGGGGUACCA | 24 | 15057 |
| HSV1-UL30-6118 | - | CUAGACGCCGCCGCCCCA | 18 | 15058 |
| HSV1-UL30-6119 | - | GCUAGACGCCGCCGCCCCA | 19 | 15059 |
| HSV1-UL30-2019 | - | AGCUAGACGCCGCCGCCCCA | 20 | 5052 |
| HSV1-UL30-6120 | - | GAGCUAGACGCCGCCGCCCCA | 21 | 15060 |
| HSV1-UL30-6121 | - | CGAGCUAGACGCCGCCGCCCCA | 22 | 15061 |
| HSV1-UL30-6122 | - | GCGAGCUAGACGCCGCCGCCCCA | 23 | 15062 |
| HSV1-UL30-6123 | - | CGCGAGCUAGACGCCGCCGCCCCA | 24 | 15063 |
| HSV1-UL30-6124 | - | GAGGACGAGGAGCGGCCA | 18 | 15064 |
| HSV1-UL30-6125 | - | GGAGGACGAGGAGCGGCCA | 19 | 15065 |
| HSV1-UL30-1898 | - | GGGAGGACGAGGAGCGGCCA | 20 | 4946 |
| HSV1-UL30-6126 | - | CGGGAGGACGAGGAGCGGCCA | 21 | 15066 |
| HSV1-UL30-6127 | - | CCGGGAGGACGAGGAGCGGCCA | 22 | 15067 |
| HSV1-UL30-6128 | - | CCCGGGAGGACGAGGAGCGGCCA | 23 | 15068 |
| HSV1-UL30-6129 | - | GCCCGGGAGGACGAGGAGCGGCCA | 24 | 15069 |
| HSV1-UL30-6130 | - | CGGCGGACAACCUGGCCA | 18 | 15070 |
| HSV1-UL30-6131 | - | ACGGCGGACAACCUGGCCA | 19 | 15071 |
| HSV1-UL30-1810 | - | UACGGCGGACAACCUGGCCA | 20 | 4860 |
| HSV1-UL30-6132 | - | GUACGGCGGACAACCUGGCCA | 21 | 15072 |
| HSV1-UL30-6133 | - | UGUACGGCGGACAACCUGGCCA | 22 | 15073 |
| HSV1-UL30-6134 | - | CUGUACGGCGGACAACCUGGCCA | 23 | 15074 |
| HSV1-UL30-6135 | - | ACUGUACGGCGGACAACCUGGCCA | 24 | 15075 |
| HSV1-UL30-6136 | - | AACUCGUCGAAUGUUGCA | 18 | 15076 |
| HSV1-UL30-6137 | - | AAACUCGUCGAAUGUUGCA | 19 | 15077 |
| HSV1-UL30-2052 | - | GAAACUCGUCGAAUGUUGCA | 20 | 5085 |
| HSV1-UL30-6138 | - | GGAAACUCGUCGAAUGUUGCA | 21 | 15078 |
| HSV1-UL30-6139 | - | AGGAAACUCGUCGAAUGUUGCA | 22 | 15079 |
| HSV1-UL30-6140 | - | GAGGAAACUCGUCGAAUGUUGCA | 23 | 15080 |
| HSV1-UL30-6141 | - | GGAGGAAACUCGUCGAAUGUUGCA | 24 | 15081 |
| HSV1-UL30-6142 | - | GGAAUAACGCCAAGAUCA | 18 | 15082 |
| HSV1-UL30-6143 | - | GGGAAUAACGCCAAGAUCA | 19 | 15083 |
| HSV1-UL30-2038 | - | UGGGAAUAACGCCAAGAUCA | 20 | 5071 |
| HSV1-UL30-6144 | - | UUGGGAAUAACGCCAAGAUCA | 21 | 15084 |
| HSV1-UL30-6145 | - | UUUGGGAAUAACGCCAAGAUCA | 22 | 15085 |
| HSV1-UL30-6146 | - | GUUUGGGAAUAACGCCAAGAUCA | 23 | 15086 |
| HSV1-UL30-6147 | - | UGUUUGGGAAUAACGCCAAGAUCA | 24 | 15087 |
| HSV1-UL30-6148 | - | ACUCGGUGUACGGGUUCA | 18 | 15088 |
| HSV1-UL30-6149 | - | AACUCGGUGUACGGGUUCA | 19 | 15089 |
| HSV1-UL30-1964 | - | UAACUCGGUGUACGGGUUCA | 20 | 4997 |
| HSV1-UL30-6150 | - | GUAACUCGGUGUACGGGUUCA | 21 | 15090 |
| HSV1-UL30-6151 | - | UGUAACUCGGUGUACGGGUUCA | 22 | 15091 |
| HSV1-UL30-6152 | - | GUGUAACUCGGUGUACGGGUUCA | 23 | 15092 |
| HSV1-UL30-6153 | - | UGUGUAACUCGGUGUACGGGUUCA | 24 | 15093 |
| HSV1-UL30-6154 | - | GCAAGGACUACCUGGAGA | 18 | 15094 |
| HSV1-UL30-6155 | - | GGCAAGGACUACCUGGAGA | 19 | 15095 |
| HSV1-UL30-1944 | - | GGGCAAGGACUACCUGGAGA | 20 | 4977 |
| HSV1-UL30-6156 | - | CGGGCAAGGACUACCUGGAGA | 21 | 15096 |
| HSV1-UL30-6157 | - | GCGGGCAAGGACUACCUGGAGA | 22 | 15097 |
| HSV1-UL30-6158 | - | GGCGGGCAAGGACUACCUGGAGA | 23 | 15098 |
| HSV1-UL30-6159 | - | AGGCGGGCAAGGACUACCUGGAGA | 24 | 15099 |
| HSV1-UL30-6160 | - | UGGACGCCAUCACACCGA | 18 | 15100 |
| HSV1-UL30-6161 | - | AUGGACGCCAUCACACCGA | 19 | 15101 |
| HSV1-UL30-1772 | - | UAUGGACGCCAUCACACCGA | 20 | 4822 |
| HSV1-UL30-6162 | - | UUAUGGACGCCAUCACACCGA | 21 | 15102 |
| HSV1-UL30-6163 | - | UUUAUGGACGCCAUCACACCGA | 22 | 15103 |
| HSV1-UL30-6164 | - | GUUUAUGGACGCCAUCACACCGA | 23 | 15104 |
| HSV1-UL30-6165 | - | GGUUUAUGGACGCCAUCACACCGA | 24 | 15105 |
| HSV1-UL30-6166 | - | CGAGGAGGGCGGGGGCGA | 18 | 15106 |
| HSV1-UL30-6167 | - | GCGAGGAGGGCGGGGGCGA | 19 | 15107 |
| HSV1-UL30-1919 | - | CGCGAGGAGGGCGGGGGCGA | 20 | 4958 |
| HSV1-UL30-6168 | - | ACGCGAGGAGGGCGGGGGCGA | 21 | 15108 |
| HSV1-UL30-6169 | - | AACGCGAGGAGGGCGGGGGCGA | 22 | 15109 |
| HSV1-UL30-6170 | - | GAACGCGAGGAGGGCGGGGGCGA | 23 | 15110 |
| HSV1-UL30-6171 | - | CGAACGCGAGGAGGGCGGGGGCGA | 24 | 15111 |
| HSV1-UL30-6172 | - | GUCAAGGCUCACGUGCGA | 18 | 15112 |
| HSV1-UL30-6173 | - | CGUCAAGGCUCACGUGCGA | 19 | 15113 |
| HSV1-UL30-1952 | - | UC GUCAAGGCUCAC GUGC GA | 20 | 4985 |
| HSV1-UL30-6174 | - | UUC GUCAAGGCUCAC GUGC GA | 21 | 15114 |
| HSV1-UL30-6175 | - | CUUCGUCAAGGCUCACGUGCGA | 22 | 15115 |
| HSV1-UL30-6176 | - | UCUUCGUCAAGGCUCACGUGCGA | 23 | 15116 |
| HSV1-UL30-6177 | - | UUCUUCGUCAAGGCUCACGUGCGA | 24 | 15117 |
| HSV1-UL30-6178 | - | GGACAACCUGGCCAUCGA | 18 | 15118 |
| HSV1-UL30-6179 | - | CGGACAACCUGGCCAUCGA | 19 | 15119 |
| HSV1-UL30-1813 | - | GCGGACAACCUGGCCAUCGA | 20 | 4863 |
| HSV1-UL30-6180 | - | GGCGGACAACCUGGCCAUCGA | 21 | 15120 |
| HSV1-UL30-6181 | - | CGGCGGACAACCUGGCCAUCGA | 22 | 15121 |
| HSV1-UL30-6182 | - | ACGGCGGACAACCUGGCCAUCGA | 23 | 15122 |
| HSV1-UL30-6183 | - | UACGGCGGACAACCUGGCCAUCGA | 24 | 15123 |
| HSV1-UL30-6184 | - | GGAGCGGCCAGAGGAGGA | 18 | 15124 |
| HSV1-UL30-6185 | - | AGGAGCGGCCAGAGGAGGA | 19 | 15125 |
| HSV1-UL30-574 | - | GAGGAGCGGCCAGAGGAGGA | 20 | 3512 |
| HSV1-UL30-6186 | - | CGAGGAGCGGCCAGAGGAGGA | 21 | 15126 |
| HSV1-UL30-6187 | - | ACGAGGAGCGGCCAGAGGAGGA | 22 | 15127 |
| HSV1-UL30-6188 | - | GACGAGGAGCGGCCAGAGGAGGA | 23 | 15128 |
| HSV1-UL30-6189 | - | GGACGAGGAGCGGCCAGAGGAGGA | 24 | 15129 |
| HSV1-UL30-6190 | - | GCGCAUCACCGACCCGGA | 18 | 15130 |
| HSV1-UL30-6191 | - | GGCGCAUCACCGACCCGGA | 19 | 15131 |
| HSV1-UL30-2005 | - | CGGCGCAUCACCGACCCGGA | 20 | 5038 |
| HSV1-UL30-6192 | - | UCGGCGCAUCACCGACCCGGA | 21 | 15132 |
| HSV1-UL30-6193 | - | AUCGGCGCAUCACCGACCCGGA | 22 | 15133 |
| HSV1-UL30-6194 | - | CAUCGGCGCAUCACCGACCCGGA | 23 | 15134 |
| HSV1-UL30-6195 | - | CCAUCGGCGCAUCACCGACCCGGA | 24 | 15135 |
| HSV1-UL30-6196 | - | UCCCCGAAUCCCACCUGA | 18 | 15136 |
| HSV1-UL30-6197 | - | CUCCCCGAAUCCCACCUGA | 19 | 15137 |
| HSV1-UL30-1835 | - | CCUCCCCGAAUCCCACCUGA | 20 | 4885 |
| HSV1-UL30-6198 | - | ACCUCCCCGAAUCCCACCUGA | 21 | 15138 |
| HSV1-UL30-6199 | - | GACCUCCCCGAAUCCCACCUGA | 22 | 15139 |
| HSV1-UL30-6200 | - | CGACCUCCCCGAAUCCCACCUGA | 23 | 15140 |
| HSV1-UL30-6201 | - | GCGACCUCCCCGAAUCCCACCUGA | 24 | 15141 |
| HSV1-UL30-6202 | - | UUC CAUGC GCAUCAUCUA | 18 | 15142 |
| HSV1-UL30-6203 | - | AUUCCAUGCGCAUCAUCUA | 19 | 15143 |
| HSV1-UL30-1975 | - | UAUUC CAUGC GCAUCAUCUA | 20 | 5008 |
| HSV1-UL30-6204 | - | CUAUUCCAUGCGCAUCAUCUA | 21 | 15144 |
| HSV1-UL30-6205 | - | CCUAUUCCAUGCGCAUCAUCUA | 22 | 15145 |
| HSV1-UL30-6206 | - | CCCUAUUCCAUGCGCAUCAUCUA | 23 | 15146 |
| HSV1-UL30-6207 | - | GCCCUAUUCCAUGCGCAUCAUCUA | 24 | 15147 |
| HSV1-UL30-6208 | - | GUACAUCGGCGUCAUCUA | 18 | 15148 |
| HSV1-UL30-6209 | - | AGUACAUCGGCGUCAUCUA | 19 | 15149 |
| HSV1-UL30-1985 | - | AAGUACAUCGGCGUCAUCUA | 20 | 5018 |
| HSV1-UL30-6210 | - | AAAGUACAUCGGCGUCAUCUA | 21 | 15150 |
| HSV1-UL30-6211 | - | AAAAGUACAUCGGCGUCAUCUA | 22 | 15151 |
| HSV1-UL30-6212 | - | AAAAAGUACAUCGGCGUCAUCUA | 23 | 15152 |
| HSV1-UL30-6213 | - | GAAAAAGUACAUCGGCGUCAUCUA | 24 | 15153 |
| HSV1-UL30-6214 | - | GCCCAGACCCGCGAGGUA | 18 | 15154 |
| HSV1-UL30-6215 | - | GGCCCAGACCCGCGAGGUA | 19 | 15155 |
| HSV1-UL30-2014 | - | UGGCCCAGACCCGCGAGGUA | 20 | 5047 |
| HSV1-UL30-6216 | - | GUGGCCCAGACCCGCGAGGUA | 21 | 15156 |
| HSV1-UL30-6217 | - | CGUGGCCCAGACCCGCGAGGUA | 22 | 15157 |
| HSV1-UL30-6218 | - | UCGUGGCCCAGACCCGCGAGGUA | 23 | 15158 |
| HSV1-UL30-6219 | - | AUCGUGGCCCAGACCCGCGAGGUA | 24 | 15159 |
| HSV1-UL30-6220 | - | CCGCCGGGCCCGCGCAAC | 18 | 15160 |
| HSV1-UL30-6221 | - | GCCGCCGGGCCCGCGCAAC | 19 | 15161 |
| HSV1-UL30-1868 | - | CGCCGCCGGGCCCGCGCAAC | 20 | 4918 |
| HSV1-UL30-6222 | - | ACGCCGCCGGGCCCGCGCAAC | 21 | 15162 |
| HSV1-UL30-6223 | - | UACGCCGCCGGGCCCGCGCAAC | 22 | 15163 |
| HSV1-UL30-6224 | - | CUACGCCGCCGGGCCCGCGCAAC | 23 | 15164 |
| HSV1-UL30-6225 | - | ACUACGCCGCCGGGCCCGCGCAAC | 24 | 15165 |
| HSV1-UL30-6226 | - | GGGAGGACGAGGACGAAC | 18 | 15166 |
| HSV1-UL30-6227 | - | GGGGAGGACGAGGACGAAC | 19 | 15167 |
| HSV1-UL30-1911 | - | GGGGGAGGACGAGGACGAAC | 20 | 4953 |
| HSV1-UL30-6228 | - | AGGGGGAGGACGAGGACGAAC | 21 | 15168 |
| HSV1-UL30-6229 | - | GAGGGGGAGGACGAGGACGAAC | 22 | 15169 |
| HSV1-UL30-6230 | - | GGAGGGGGAGGACGAGGACGAAC | 23 | 15170 |
| HSV1-UL30-6231 | - | AGGAGGGGGAGGACGAGGACGAAC | 24 | 15171 |
| HSV1-UL30-6232 | - | CAGUACUUUUACAUGAAC | 18 | 15172 |
| HSV1-UL30-6233 | - | GCAGUACUUUUACAUGAAC | 19 | 15173 |
| HSV1-UL30-1779 | - | GGCAGUACUUUUACAUGAAC | 20 | 4829 |
| HSV1-UL30-6234 | - | CGGCAGUACUUUUACAUGAAC | 21 | 15174 |
| HSV1-UL30-6235 | - | GCGGCAGUACUUUUACAUGAAC | 22 | 15175 |
| HSV1-UL30-6236 | - | CGCGGCAGUACUUUUACAUGAAC | 23 | 15176 |
| HSV1-UL30-6237 | - | ACGCGGCAGUACUUUUACAUGAAC | 24 | 15177 |
| HSV1-UL30-6238 | - | CACGGCGUCGCCCUGAAC | 18 | 15178 |
| HSV1-UL30-6239 | - | CCACGGCGUCGCCCUGAAC | 19 | 15179 |
| HSV1-UL30-2031 | - | CCCACGGCGUCGCCCUGAAC | 20 | 5064 |
| HSV1-UL30-6240 | - | GCCCACGGCGUCGCCCUGAAC | 21 | 15180 |
| HSV1-UL30-6241 | - | UGCCCACGGCGUCGCCCUGAAC | 22 | 15181 |
| HSV1-UL30-6242 | - | UUGCCCACGGCGUCGCCCUGAAC | 23 | 15182 |
| HSV1-UL30-6243 | - | AUUGCCCACGGCGUCGCCCUGAAC | 24 | 15183 |
| HSV1-UL30-6244 | - | GACCUCCCCGAAUCCCAC | 18 | 15184 |
| HSV1-UL30-6245 | - | CGACCUCCCCGAAUCCCAC | 19 | 15185 |
| HSV1-UL30-1834 | - | GCGACCUCCCCGAAUCCCAC | 20 | 4884 |
| HSV1-UL30-6246 | - | UGCGACCUCCCCGAAUCCCAC | 21 | 15186 |
| HSV1-UL30-6247 | - | CUGCGACCUCCCCGAAUCCCAC | 22 | 15187 |
| HSV1-UL30-6248 | - | CCUGCGACCUCCCCGAAUCCCAC | 23 | 15188 |
| HSV1-UL30-6249 | - | UCCUGCGACCUCCCCGAAUCCCAC | 24 | 15189 |
| HSV1-UL30-6250 | - | GCCGACGCAGUGGCGCAC | 18 | 15190 |
| HSV1-UL30-6251 | - | GGCCGACGCAGUGGCGCAC | 19 | 15191 |
| HSV1-UL30-1938 | - | GGGCCGACGCAGUGGCGCAC | 20 | 4971 |
| HSV1-UL30-6252 | - | AGGGCCGACGCAGUGGCGCAC | 21 | 15192 |
| HSV1-UL30-6253 | - | GAGGGCCGACGCAGUGGCGCAC | 22 | 15193 |
| HSV1-UL30-6254 | - | UGAGGGCCGACGCAGUGGCGCAC | 23 | 15194 |
| HSV1-UL30-6255 | - | CUGAGGGCCGACGCAGUGGCGCAC | 24 | 15195 |
| HSV1-UL30-6256 | - | GAGACCGCCGGCAGGCAC | 18 | 15196 |
| HSV1-UL30-6257 | - | GGAGACCGCCGGCAGGCAC | 19 | 15197 |
| HSV1-UL30-1928 | - | GGGAGACCGCCGGCAGGCAC | 20 | 4962 |
| HSV1-UL30-6258 | - | CGGGAGACCGCCGGCAGGCAC | 21 | 15198 |
| HSV1-UL30-6259 | - | GCGGGAGACCGCCGGCAGGCAC | 22 | 15199 |
| HSV1-UL30-6260 | - | CGCGGGAGACCGCCGGCAGGCAC | 23 | 15200 |
| HSV1-UL30-6261 | - | GCGCGGGAGACCGCCGGCAGGCAC | 24 | 15201 |
| HSV1-UL30-6262 | - | UUUCCGGUGGCCGGGCAC | 18 | 15202 |
| HSV1-UL30-6263 | - | CUUUCCGGUGGCCGGGCAC | 19 | 15203 |
| HSV1-UL30-1828 | - | CCUUUCCGGUGGCCGGGCAC | 20 | 4878 |
| HSV1-UL30-6264 | - | GCCUUUCCGGUGGCCGGGCAC | 21 | 15204 |
| HSV1-UL30-6265 | - | GGCCUUUCCGGUGGCCGGGCAC | 22 | 15205 |
| HSV1-UL30-6266 | - | UGGCCUUUCCGGUGGCCGGGCAC | 23 | 15206 |
| HSV1-UL30-6267 | - | CUGGCCUUUCCGGUGGCCGGGCAC | 24 | 15207 |
| HSV1-UL30-6268 | - | CCCACUUCCGGGUUUCAC | 18 | 15208 |
| HSV1-UL30-6269 | - | CCCCACUUCCGGGUUUCAC | 19 | 15209 |
| HSV1-UL30-1935 | - | ACCCCACUUCCGGGUUUCAC | 20 | 4968 |
| HSV1-UL30-6270 | - | GACCCCACUUCCGGGUUUCAC | 21 | 15210 |
| HSV1-UL30-6271 | - | UGACCCCACUUCCGGGUUUCAC | 22 | 15211 |
| HSV1-UL30-6272 | - | UUGACCCCACUUCCGGGUUUCAC | 23 | 15212 |
| HSV1-UL30-6273 | - | CUUGACCCCACUUCCGGGUUUCAC | 24 | 15213 |
| HSV1-UL30-6274 | - | GGACGCCAUCACACCGAC | 18 | 15214 |
| HSV1-UL30-6275 | - | UGGACGCCAUCACACCGAC | 19 | 15215 |
| HSV1-UL30-1773 | - | AUGGACGCCAUCACACCGAC | 20 | 4823 |
| HSV1-UL30-6276 | - | UAUGGACGCCAUCACACCGAC | 21 | 15216 |
| HSV1-UL30-6277 | - | UUAUGGACGCCAUCACACCGAC | 22 | 15217 |
| HSV1-UL30-6278 | - | UUUAUGGACGCCAUCACACCGAC | 23 | 15218 |
| HSV1-UL30-6279 | - | GUUUAUGGACGCCAUCACACCGAC | 24 | 15219 |
| HSV1-UL30-6280 | - | CAUCGGCGCAUCACCGAC | 18 | 15220 |
| HSV1-UL30-6281 | - | CCAUCGGCGCAUCACCGAC | 19 | 15221 |
| HSV1-UL30-2002 | - | CCCAUCGGCGCAUCACCGAC | 20 | 5035 |
| HSV1-UL30-6282 | - | GCCCAUCGGCGCAUCACCGAC | 21 | 15222 |
| HSV1-UL30-6283 | - | CGCCCAUCGGCGCAUCACCGAC | 22 | 15223 |
| HSV1-UL30-6284 | - | ACGCCCAUCGGCGCAUCACCGAC | 23 | 15224 |
| HSV1-UL30-6285 | - | GACGCCCAUCGGCGCAUCACCGAC | 24 | 15225 |
| HSV1-UL30-6286 | - | CUGCUGCGCCUGGCCGAC | 18 | 15226 |
| HSV1-UL30-6287 | - | CCUGCUGCGCCUGGCCGAC | 19 | 15227 |
| HSV1-UL30-1876 | - | GCCUGCUGCGCCUGGCCGAC | 20 | 4925 |
| HSV1-UL30-6288 | - | UGCCUGCUGCGCCUGGCCGAC | 21 | 15228 |
| HSV1-UL30-6289 | - | GUGCCUGCUGCGCCUGGCCGAC | 22 | 15229 |
| HSV1-UL30-6290 | - | CGUGCCUGCUGCGCCUGGCCGAC | 23 | 15230 |
| HSV1-UL30-6291 | - | ACGUGCCUGCUGCGCCUGGCCGAC | 24 | 15231 |
| HSV1-UL30-6292 | - | AAGGACAAGAAGAAGGAC | 18 | 15232 |
| HSV1-UL30-6293 | - | GAAGGACAAGAAGAAGGAC | 19 | 15233 |
| HSV1-UL30-1866 | - | UGAAGGACAAGAAGAAGGAC | 20 | 4916 |
| HSV1-UL30-6294 | - | CUGAAGGACAAGAAGAAGGAC | 21 | 15234 |
| HSV1-UL30-6295 | - | CCUGAAGGACAAGAAGAAGGAC | 22 | 15235 |
| HSV1-UL30-6296 | - | UCCUGAAGGACAAGAAGAAGGAC | 23 | 15236 |
| HSV1-UL30-6297 | - | GUCCUGAAGGACAAGAAGAAGGAC | 24 | 15237 |
| HSV1-UL30-6298 | - | GAAGCCGUCCUGAAGGAC | 18 | 15238 |
| HSV1-UL30-6299 | - | CGAAGCCGUCCUGAAGGAC | 19 | 15239 |
| HSV1-UL30-1863 | - | CCGAAGCCGUCCUGAAGGAC | 20 | 4913 |
| HSV1-UL30-6300 | - | GCCGAAGCCGUCCUGAAGGAC | 21 | 15240 |
| HSV1-UL30-6301 | - | GGCCGAAGCCGUCCUGAAGGAC | 22 | 15241 |
| HSV1-UL30-6302 | - | UGGCCGAAGCCGUCCUGAAGGAC | 23 | 15242 |
| HSV1-UL30-6303 | - | GUGGCCGAAGCCGUCCUGAAGGAC | 24 | 15243 |
| HSV1-UL30-6304 | - | GCCGCAGCCCGGGAGGAC | 18 | 15244 |
| HSV1-UL30-6305 | - | GGCCGCAGCCCGGGAGGAC | 19 | 15245 |
| HSV1-UL30-1895 | - | CGGCCGCAGCCCGGGAGGAC | 20 | 4944 |
| HSV1-UL30-6306 | - | CCGGCCGCAGCCCGGGAGGAC | 21 | 15246 |
| HSV1-UL30-6307 | - | UCCGGCCGCAGCCCGGGAGGAC | 22 | 15247 |
| HSV1-UL30-6308 | - | GUCCGGCCGCAGCCCGGGAGGAC | 23 | 15248 |
| HSV1-UL30-6309 | - | CGUCCGGCCGCAGCCCGGGAGGAC | 24 | 15249 |
| HSV1-UL30-6310 | - | GAGGAGGAGGGGGAGGAC | 18 | 15250 |
| HSV1-UL30-6311 | - | AGAGGAGGAGGGGGAGGAC | 19 | 15251 |
| HSV1-UL30-1909 | - | CAGAGGAGGAGGGGGAGGAC | 20 | 4951 |
| HSV1-UL30-6312 | - | CCAGAGGAGGAGGGGGAGGAC | 21 | 15252 |
| HSV1-UL30-6313 | - | GCCAGAGGAGGAGGGGGAGGAC | 22 | 15253 |
| HSV1-UL30-6314 | - | GGCCAGAGGAGGAGGGGGAGGAC | 23 | 15254 |
| HSV1-UL30-6315 | - | CGGCCAGAGGAGGAGGGGGAGGAC | 24 | 15255 |
| HSV1-UL30-6316 | - | CGCAUCAUCUACGGGGAC | 18 | 15256 |
| HSV1-UL30-6317 | - | GC GCAUCAUCUAC GGGGAC | 19 | 15257 |
| HSV1-UL30-1977 | - | UGC GCAUCAUCUAC GGGGAC | 20 | 5010 |
| HSV1-UL30-6318 | - | AUGC GCAUCAUCUAC GGGGAC | 21 | 15258 |
| HSV1-UL30-6319 | - | CAUGCGCAUCAUCUACGGGGAC | 22 | 15259 |
| HSV1-UL30-6320 | - | CCAUGCGCAUCAUCUACGGGGAC | 23 | 15260 |
| HSV1-UL30-6321 | - | UCCAUGCGCAUCAUCUACGGGGAC | 24 | 15261 |
| HSV1-UL30-6322 | - | GAGGCGGGCAAGGACUAC | 18 | 15262 |
| HSV1-UL30-6323 | - | GGAGGCGGGCAAGGACUAC | 19 | 15263 |
| HSV1-UL30-1942 | - | UGGAGGCGGGCAAGGACUAC | 20 | 4975 |
| HSV1-UL30-6324 | - | CUGGAGGCGGGCAAGGACUAC | 21 | 15264 |
| HSV1-UL30-6325 | - | CCUGGAGGCGGGCAAGGACUAC | 22 | 15265 |
| HSV1-UL30-6326 | - | ACCUGGAGGCGGGCAAGGACUAC | 23 | 15266 |
| HSV1-UL30-6327 | - | CACCUGGAGGCGGGCAAGGACUAC | 24 | 15267 |
| HSV1-UL30-6328 | - | UCCAUGCGCAUCAUCUAC | 18 | 15268 |
| HSV1-UL30-6329 | - | UUCCAUGCGCAUCAUCUAC | 19 | 15269 |
| HSV1-UL30-1976 | - | AUUC CAUGC GCAUCAUCUAC | 20 | 5009 |
| HSV1-UL30-6330 | - | UAUUC CAUGC GCAUCAUCUAC | 21 | 15270 |
| HSV1-UL30-6331 | - | CUAUUCCAUGCGCAUCAUCUAC | 22 | 15271 |
| HSV1-UL30-6332 | - | CCUAUUCCAUGCGCAUCAUCUAC | 23 | 15272 |
| HSV1-UL30-6333 | - | CCCUAUUCCAUGCGCAUCAUCUAC | 24 | 15273 |
| HSV1-UL30-6334 | - | GGCAGGCACGUGGGGUAC | 18 | 15274 |
| HSV1-UL30-6335 | - | CGGCAGGCACGUGGGGUAC | 19 | 15275 |
| HSV1-UL30-1930 | - | CCGGCAGGCACGUGGGGUAC | 20 | 4963 |
| HSV1-UL30-6336 | - | GCCGGCAGGCACGUGGGGUAC | 21 | 15276 |
| HSV1-UL30-6337 | - | CGCCGGCAGGCACGUGGGGUAC | 22 | 15277 |
| HSV1-UL30-6338 | - | CCGCCGGCAGGCACGUGGGGUAC | 23 | 15278 |
| HSV1-UL30-6339 | - | ACCGCCGGCAGGCACGUGGGGUAC | 24 | 15279 |
| HSV1-UL30-6340 | - | ACGCGGCAGUACUUUUAC | 18 | 15280 |
| HSV1-UL30-6341 | - | CACGCGGCAGUACUUUUAC | 19 | 15281 |
| HSV1-UL30-1778 | - | GCACGCGGCAGUACUUUUAC | 20 | 4828 |
| HSV1-UL30-6342 | - | GGCACGCGGCAGUACUUUUAC | 21 | 15282 |
| HSV1-UL30-6343 | - | CGGCACGCGGCAGUACUUUUAC | 22 | 15283 |
| HSV1-UL30-6344 | - | ACGGCACGCGGCAGUACUUUUAC | 23 | 15284 |
| HSV1-UL30-6345 | - | UACGGCACGCGGCAGUACUUUUAC | 24 | 15285 |
| HSV1-UL30-6346 | - | UUUAUUCUGCCGGACACC | 18 | 15286 |
| HSV1-UL30-6347 | - | CUUUAUUCUGCCGGACACC | 19 | 15287 |
| HSV1-UL30-1879 | - | GCUUUAUUCUGCCGGACACC | 20 | 4928 |
| HSV1-UL30-6348 | - | GGCUUUAUUCUGCCGGACACC | 21 | 15288 |
| HSV1-UL30-6349 | - | GGGCUUUAUUCUGCCGGACACC | 22 | 15289 |
| HSV1-UL30-6350 | - | AGGGCUUUAUUCUGCCGGACACC | 23 | 15290 |
| HSV1-UL30-6351 | - | AAGGGCUUUAUUCUGCCGGACACC | 24 | 15291 |
| HSV1-UL30-6352 | - | CCGACGCAGUGGCGCACC | 18 | 15292 |
| HSV1-UL30-6353 | - | GCCGACGCAGUGGCGCACC | 19 | 15293 |
| HSV1-UL30-1939 | - | GGCCGACGCAGUGGCGCACC | 20 | 4972 |
| HSV1-UL30-6354 | - | GGGCCGACGCAGUGGCGCACC | 21 | 15294 |
| HSV1-UL30-6355 | - | AGGGCCGACGCAGUGGCGCACC | 22 | 15295 |
| HSV1-UL30-6356 | - | GAGGGCCGACGCAGUGGCGCACC | 23 | 15296 |
| HSV1-UL30-6357 | - | UGAGGGCCGACGCAGUGGCGCACC | 24 | 15297 |
| HSV1-UL30-6358 | - | UUCCGGUGGCCGGGCACC | 18 | 15298 |
| HSV1-UL30-6359 | - | UUUCCGGUGGCCGGGCACC | 19 | 15299 |
| HSV1-UL30-1829 | - | CUUUCCGGUGGCCGGGCACC | 20 | 4879 |
| HSV1-UL30-6360 | - | CCUUUCCGGUGGCCGGGCACC | 21 | 15300 |
| HSV1-UL30-6361 | - | GCCUUUCCGGUGGCCGGGCACC | 22 | 15301 |
| HSV1-UL30-6362 | - | GGCCUUUCCGGUGGCCGGGCACC | 23 | 15302 |
| HSV1-UL30-6363 | - | UGGCCUUUCCGGUGGCCGGGCACC | 24 | 15303 |
| HSV1-UL30-6364 | - | AUCGGCGCAUCACCGACC | 18 | 15304 |
| HSV1-UL30-6365 | - | CAUCGGCGCAUCACCGACC | 19 | 15305 |
| HSV1-UL30-2003 | - | CCAUCGGCGCAUCACCGACC | 20 | 5036 |
| HSV1-UL30-6366 | - | CCCAUCGGCGCAUCACCGACC | 21 | 15306 |
| HSV1-UL30-6367 | - | GCCCAUCGGCGCAUCACCGACC | 22 | 15307 |
| HSV1-UL30-6368 | - | CGCCCAUCGGCGCAUCACCGACC | 23 | 15308 |
| HSV1-UL30-6369 | - | ACGCCCAUCGGCGCAUCACCGACC | 24 | 15309 |
| HSV1-UL30-6370 | - | AGGCGGGCAAGGACUACC | 18 | 15310 |
| HSV1-UL30-6371 | - | GAGGCGGGCAAGGACUACC | 19 | 15311 |
| HSV1-UL30-1943 | - | GGAGGCGGGCAAGGACUACC | 20 | 4976 |
| HSV1-UL30-6372 | - | UGGAGGCGGGCAAGGACUACC | 21 | 15312 |
| HSV1-UL30-6373 | - | CUGGAGGCGGGCAAGGACUACC | 22 | 15313 |
| HSV1-UL30-6374 | - | CCUGGAGGCGGGCAAGGACUACC | 23 | 15314 |
| HSV1-UL30-6375 | - | ACCUGGAGGCGGGCAAGGACUACC | 24 | 15315 |
| HSV1-UL30-6376 | - | GCAGGCACGUGGGGUACC | 18 | 15316 |
| HSV1-UL30-6377 | - | GGCAGGCACGUGGGGUACC | 19 | 15317 |
| HSV1-UL30-1931 | - | CGGCAGGCACGUGGGGUACC | 20 | 4964 |
| HSV1-UL30-6378 | - | CCGGCAGGCACGUGGGGUACC | 21 | 15318 |
| HSV1-UL30-6379 | - | GCCGGCAGGCACGUGGGGUACC | 22 | 15319 |
| HSV1-UL30-6380 | - | CGCCGGCAGGCACGUGGGGUACC | 23 | 15320 |
| HSV1-UL30-6381 | - | CCGCCGGCAGGCACGUGGGGUACC | 24 | 15321 |
| HSV1-UL30-6382 | - | UUAUUCUGCCGGACACCC | 18 | 15322 |
| HSV1-UL30-6383 | - | UUUAUUCUGCCGGACACCC | 19 | 15323 |
| HSV1-UL30-1880 | - | CUUUAUUCUGCCGGACACCC | 20 | 4929 |
| HSV1-UL30-6384 | - | GCUUUAUUCUGCCGGACACCC | 21 | 15324 |
| HSV1-UL30-6385 | - | GGCUUUAUUCUGCCGGACACCC | 22 | 15325 |
| HSV1-UL30-6386 | - | GGGCUUUAUUCUGCCGGACACCC | 23 | 15326 |
| HSV1-UL30-6387 | - | AGGGCUUUAUUCUGCCGGACACCC | 24 | 15327 |
| HSV1-UL30-6388 | - | UGAUCGUGGCCCAGACCC | 18 | 15328 |
| HSV1-UL30-6389 | - | GUGAUCGUGGCCCAGACCC | 19 | 15329 |
| HSV1-UL30-2012 | - | CGUGAUCGUGGCCCAGACCC | 20 | 5045 |
| HSV1-UL30-6390 | - | ACGUGAUCGUGGCCCAGACCC | 21 | 15330 |
| HSV1-UL30-6391 | - | UACGUGAUCGUGGCCCAGACCC | 22 | 15331 |
| HSV1-UL30-6392 | - | GUACGUGAUCGUGGCCCAGACCC | 23 | 15332 |
| HSV1-UL30-6393 | - | CGUACGUGAUCGUGGCCCAGACCC | 24 | 15333 |
| HSV1-UL30-6394 | - | CCCGAAGUGUGGCACCCC | 18 | 15334 |
| HSV1-UL30-6395 | - | UCCCGAAGUGUGGCACCCC | 19 | 15335 |
| HSV1-UL30-2041 | - | UUCCCGAAGUGUGGCACCCC | 20 | 5074 |
| HSV1-UL30-6396 | - | AUUCCCGAAGUGUGGCACCCC | 21 | 15336 |
| HSV1-UL30-6397 | - | UAUUCCCGAAGUGUGGCACCCC | 22 | 15337 |
| HSV1-UL30-6398 | - | UUAUUCCCGAAGUGUGGCACCCC | 23 | 15338 |
| HSV1-UL30-6399 | - | UUUAUUCCCGAAGUGUGGCACCCC | 24 | 15339 |
| HSV1-UL30-6400 | - | CCGUCGCCUGCCGACCCC | 18 | 15340 |
| HSV1-UL30-6401 | - | GCCGUCGCCUGCCGACCCC | 19 | 15341 |
| HSV1-UL30-2024 | - | CGCCGUCGCCUGCCGACCCC | 20 | 5057 |
| HSV1-UL30-6402 | - | ACGCCGUCGCCUGCCGACCCC | 21 | 15342 |
| HSV1-UL30-6403 | - | GACGCCGUCGCCUGCCGACCCC | 22 | 15343 |
| HSV1-UL30-6404 | - | AGACGCCGUCGCCUGCCGACCCC | 23 | 15344 |
| HSV1-UL30-6405 | - | GAGACGCCGUCGCCUGCCGACCCC | 24 | 15345 |
| HSV1-UL30-6406 | - | CGUCGCCUGCCGACCCCC | 18 | 15346 |
| HSV1-UL30-6407 | - | CCGUCGCCUGCCGACCCCC | 19 | 15347 |
| HSV1-UL30-2025 | - | GCCGUCGCCUGCCGACCCCC | 20 | 5058 |
| HSV1-UL30-6408 | - | CGCCGUCGCCUGCCGACCCCC | 21 | 15348 |
| HSV1-UL30-6409 | - | ACGCCGUCGCCUGCCGACCCCC | 22 | 15349 |
| HSV1-UL30-6410 | - | GACGCCGUCGCCUGCCGACCCCC | 23 | 15350 |
| HSV1-UL30-6411 | - | AGACGCCGUCGCCUGCCGACCCCC | 24 | 15351 |
| HSV1-UL30-6412 | - | GUCGCCUGCCGACCCCCC | 18 | 15352 |
| HSV1-UL30-6413 | - | CGUCGCCUGCCGACCCCCC | 19 | 15353 |
| HSV1-UL30-2026 | - | CCGUCGCCUGCCGACCCCCC | 20 | 5059 |
| HSV1-UL30-6414 | - | GCCGUCGCCUGCCGACCCCCC | 21 | 15354 |
| HSV1-UL30-6415 | - | CGCCGUCGCCUGCCGACCCCCC | 22 | 15355 |
| HSV1-UL30-6416 | - | ACGCCGUCGCCUGCCGACCCCCC | 23 | 15356 |
| HSV1-UL30-6417 | - | GACGCCGUCGCCUGCCGACCCCCC | 24 | 15357 |
| HSV1-UL30-6418 | - | CCCCGGCCAAGCGCCCCC | 18 | 15358 |
| HSV1-UL30-6419 | - | UCCCCGGCCAAGCGCCCCC | 19 | 15359 |
| HSV1-UL30-2023 | - | CUCCCCGGCCAAGCGCCCCC | 20 | 5056 |
| HSV1-UL30-6420 | - | CCUCCCCGGCCAAGCGCCCCC | 21 | 15360 |
| HSV1-UL30-6421 | - | CCCUCCCCGGCCAAGCGCCCCC | 22 | 15361 |
| HSV1-UL30-6422 | - | GCCCUCCCCGGCCAAGCGCCCCC | 23 | 15362 |
| HSV1-UL30-6423 | - | UGCCCUCCCCGGCCAAGCGCCCCC | 24 | 15363 |
| HSV1-UL30-6424 | - | AUUCCCCAGAGCAGCCCC | 18 | 15364 |
| HSV1-UL30-6425 | - | GAUUCCCCAGAGCAGCCCC | 19 | 15365 |
| HSV1-UL30-1959 | - | GGAUUCCCCAGAGCAGCCCC | 20 | 4992 |
| HSV1-UL30-6426 | - | CGGAUUCCCCAGAGCAGCCCC | 21 | 15366 |
| HSV1-UL30-6427 | - | GCGGAUUCCCCAGAGCAGCCCC | 22 | 15367 |
| HSV1-UL30-6428 | - | CGCGGAUUCCCCAGAGCAGCCCC | 23 | 15368 |
| HSV1-UL30-6429 | - | UCGCGGAUUCCCCAGAGCAGCCCC | 24 | 15369 |
| HSV1-UL30-6430 | - | GCUAGACGCCGCCGCCCC | 18 | 15370 |
| HSV1-UL30-6431 | - | AGCUAGACGCCGCCGCCCC | 19 | 15371 |
| HSV1-UL30-2018 | - | GAGCUAGACGCCGCCGCCCC | 20 | 5051 |
| HSV1-UL30-6432 | - | CGAGCUAGACGCCGCCGCCCC | 21 | 15372 |
| HSV1-UL30-6433 | - | GCGAGCUAGACGCCGCCGCCCC | 22 | 15373 |
| HSV1-UL30-6434 | - | CGCGAGCUAGACGCCGCCGCCCC | 23 | 15374 |
| HSV1-UL30-6435 | - | CCGCGAGCUAGACGCCGCCGCCCC | 24 | 15375 |
| HSV1-UL30-6436 | - | UCCCCGGCCAAGCGCCCC | 18 | 15376 |
| HSV1-UL30-6437 | - | CUCCCCGGCCAAGCGCCCC | 19 | 15377 |
| HSV1-UL30-2022 | - | CCUCCCCGGCCAAGCGCCCC | 20 | 5055 |
| HSV1-UL30-6438 | - | CCCUCCCCGGCCAAGCGCCCC | 21 | 15378 |
| HSV1-UL30-6439 | - | GCCCUCCCCGGCCAAGCGCCCC | 22 | 15379 |
| HSV1-UL30-6440 | - | UGCCCUCCCCGGCCAAGCGCCCC | 23 | 15380 |
| HSV1-UL30-6441 | - | CUGCCCUCCCCGGCCAAGCGCCCC | 24 | 15381 |
| HSV1-UL30-6442 | - | GCGUUAGCCGAGCGCCCC | 18 | 15382 |
| HSV1-UL30-6443 | - | CGCGUUAGCCGAGCGCCCC | 19 | 15383 |
| HSV1-UL30-1993 | - | CCGCGUUAGCCGAGCGCCCC | 20 | 5026 |
| HSV1-UL30-6444 | - | GCCGCGUUAGCCGAGCGCCCC | 21 | 15384 |
| HSV1-UL30-6445 | - | CGCCGCGUUAGCCGAGCGCCCC | 22 | 15385 |
| HSV1-UL30-6446 | - | CCGCCGCGUUAGCCGAGCGCCCC | 23 | 15386 |
| HSV1-UL30-6447 | - | GCCGCCGCGUUAGCCGAGCGCCCC | 24 | 15387 |
| HSV1-UL30-6448 | - | CCUACAAUGCCGCGCCCC | 18 | 15388 |
| HSV1-UL30-6449 | - | ACCUACAAUGCCGCGCCCC | 19 | 15389 |
| HSV1-UL30-1783 | - | CACCUACAAUGCCGCGCCCC | 20 | 4833 |
| HSV1-UL30-6450 | - | GCACCUACAAUGCCGCGCCCC | 21 | 15390 |
| HSV1-UL30-6451 | - | GGCACCUACAAUGCCGCGCCCC | 22 | 15391 |
| HSV1-UL30-6452 | - | AGGCACCUACAAUGCCGCGCCCC | 23 | 15392 |
| HSV1-UL30-6453 | - | CAGGCACCUACAAUGCCGCGCCCC | 24 | 15393 |
| HSV1-UL30-6454 | - | GACAUUUACAAGGUCCCC | 18 | 15394 |
| HSV1-UL30-6455 | - | GGACAUUUACAAGGUCCCC | 19 | 15395 |
| HSV1-UL30-1846 | - | CGGACAUUUACAAGGUCCCC | 20 | 4896 |
| HSV1-UL30-6456 | - | ACGGACAUUUACAAGGUCCCC | 21 | 15396 |
| HSV1-UL30-6457 | - | GACGGACAUUUACAAGGUCCCC | 22 | 15397 |
| HSV1-UL30-6458 | - | UGACGGACAUUUACAAGGUCCCC | 23 | 15398 |
| HSV1-UL30-6459 | - | CUGACGGACAUUUACAAGGUCCCC | 24 | 15399 |
| HSV1-UL30-6460 | - | AGCGUCCGGCCGCAGCCC | 18 | 15400 |
| HSV1-UL30-6461 | - | AAGCGUCCGGCCGCAGCCC | 19 | 15401 |
| HSV1-UL30-1892 | - | CAAGCGUCCGGCCGCAGCCC | 20 | 4941 |
| HSV1-UL30-6462 | - | CCAAGCGUCCGGCCGCAGCCC | 21 | 15402 |
| HSV1-UL30-6463 | - | CCCAAGCGUCCGGCCGCAGCCC | 22 | 15403 |
| HSV1-UL30-6464 | - | GCCCAAGCGUCCGGCCGCAGCCC | 23 | 15404 |
| HSV1-UL30-6465 | - | CGCCCAAGCGUCCGGCCGCAGCCC | 24 | 15405 |
| HSV1-UL30-6466 | - | ACCUGUCCACCACCGCCC | 18 | 15406 |
| HSV1-UL30-6467 | - | GACCUGUCCACCACCGCCC | 19 | 15407 |
| HSV1-UL30-1832 | - | CGACCUGUCCACCACCGCCC | 20 | 4882 |
| HSV1-UL30-6468 | - | ACGACCUGUCCACCACCGCCC | 21 | 15408 |
| HSV1-UL30-6469 | - | UACGACCUGUCCACCACCGCCC | 22 | 15409 |
| HSV1-UL30-6470 | - | CUACGACCUGUCCACCACCGCCC | 23 | 15410 |
| HSV1-UL30-6471 | - | UCUACGACCUGUCCACCACCGCCC | 24 | 15411 |
| HSV1-UL30-6472 | - | AGCUAGACGCCGCCGCCC | 18 | 15412 |
| HSV1-UL30-6473 | - | GAGCUAGACGCCGCCGCCC | 19 | 15413 |
| HSV1-UL30-2017 | - | CGAGCUAGACGCCGCCGCCC | 20 | 5050 |
| HSV1-UL30-6474 | - | GCGAGCUAGACGCCGCCGCCC | 21 | 15414 |
| HSV1-UL30-6475 | - | CGCGAGCUAGACGCCGCCGCCC | 22 | 15415 |
| HSV1-UL30-6476 | - | CCGCGAGCUAGACGCCGCCGCCC | 23 | 15416 |
| HSV1-UL30-6477 | - | UCCGCGAGCUAGACGCCGCCGCCC | 24 | 15417 |
| HSV1-UL30-6478 | - | CUCCCCGGCCAAGCGCCC | 18 | 15418 |
| HSV1-UL30-6479 | - | CCUCCCCGGCCAAGCGCCC | 19 | 15419 |
| HSV1-UL30-2021 | - | CCCUCCCCGGCCAAGCGCCC | 20 | 5054 |
| HSV1-UL30-6480 | - | GCCCUCCCCGGCCAAGCGCCC | 21 | 15420 |
| HSV1-UL30-6481 | - | UGCCCUCCCCGGCCAAGCGCCC | 22 | 15421 |
| HSV1-UL30-6482 | - | CUGCCCUCCCCGGCCAAGCGCCC | 23 | 15422 |
| HSV1-UL30-6483 | - | CCUGCCCUCCCCGGCCAAGCGCCC | 24 | 15423 |
| HSV1-UL30-6484 | - | CGGCCGACAUGCGCGCCC | 18 | 15424 |
| HSV1-UL30-6485 | - | GCGGCCGACAUGCGCGCCC | 19 | 15425 |
| HSV1-UL30-1973 | - | GGCGGCCGACAUGCGCGCCC | 20 | 5006 |
| HSV1-UL30-6486 | - | AGGCGGCCGACAUGCGCGCCC | 21 | 15426 |
| HSV1-UL30-6487 | - | GAGGCGGCCGACAUGCGCGCCC | 22 | 15427 |
| HSV1-UL30-6488 | - | GGAGGCGGCCGACAUGCGCGCCC | 23 | 15428 |
| HSV1-UL30-6489 | - | CGGAGGCGGCCGACAUGCGCGCCC | 24 | 15429 |
| HSV1-UL30-6490 | - | CUGGCGCGACCCCUGCCC | 18 | 15430 |
| HSV1-UL30-6491 | - | GCUGGCGCGACCCCUGCCC | 19 | 15431 |
| HSV1-UL30-1998 | - | GGCUGGCGCGACCCCUGCCC | 20 | 5031 |
| HSV1-UL30-6492 | - | UGGCUGGCGCGACCCCUGCCC | 21 | 15432 |
| HSV1-UL30-6493 | - | GUGGCUGGCGCGACCCCUGCCC | 22 | 15433 |
| HSV1-UL30-6494 | - | AGUGGCUGGCGCGACCCCUGCCC | 23 | 15434 |
| HSV1-UL30-6495 | - | GAGUGGCUGGCGCGACCCCUGCCC | 24 | 15435 |
| HSV1-UL30-6496 | - | AUCCGCUCGCGGAUUCCC | 18 | 15436 |
| HSV1-UL30-6497 | - | GAUCCGCUCGCGGAUUCCC | 19 | 15437 |
| HSV1-UL30-1957 | - | AGAUCCGCUCGCGGAUUCCC | 20 | 4990 |
| HSV1-UL30-6498 | - | CAGAUCCGCUCGCGGAUUCCC | 21 | 15438 |
| HSV1-UL30-6499 | - | GCAGAUCCGCUCGCGGAUUCCC | 22 | 15439 |
| HSV1-UL30-6500 | - | AGCAGAUCCGCUCGCGGAUUCCC | 23 | 15440 |
| HSV1-UL30-6501 | - | AAGCAGAUCCGCUCGCGGAUUCCC | 24 | 15441 |
| HSV1-UL30-6502 | - | GGAUUCCCCAGAGCAGCC | 18 | 15442 |
| HSV1-UL30-6503 | - | CGGAUUCCCCAGAGCAGCC | 19 | 15443 |
| HSV1-UL30-1958 | - | GCGGAUUCCCCAGAGCAGCC | 20 | 4991 |
| HSV1-UL30-6504 | - | CGCGGAUUCCCCAGAGCAGCC | 21 | 15444 |
| HSV1-UL30-6505 | - | UCGCGGAUUCCCCAGAGCAGCC | 22 | 15445 |
| HSV1-UL30-6506 | - | CUCGCGGAUUCCCCAGAGCAGCC | 23 | 15446 |
| HSV1-UL30-6507 | - | GCUCGCGGAUUCCCCAGAGCAGCC | 24 | 15447 |
| HSV1-UL30-6508 | - | AAGCGUCCGGCCGCAGCC | 18 | 15448 |
| HSV1-UL30-6509 | - | CAAGCGUCCGGCCGCAGCC | 19 | 15449 |
| HSV1-UL30-1891 | - | CCAAGCGUCCGGCCGCAGCC | 20 | 4940 |
| HSV1-UL30-6510 | - | CCCAAGCGUCCGGCCGCAGCC | 21 | 15450 |
| HSV1-UL30-6511 | - | GCCCAAGCGUCCGGCCGCAGCC | 22 | 15451 |
| HSV1-UL30-6512 | - | CGCCCAAGCGUCCGGCCGCAGCC | 23 | 15452 |
| HSV1-UL30-6513 | - | GCGCCCAAGCGUCCGGCCGCAGCC | 24 | 15453 |
| HSV1-UL30-6514 | - | GACCUGUCCACCACCGCC | 18 | 15454 |
| HSV1-UL30-6515 | - | CGACCUGUCCACCACCGCC | 19 | 15455 |
| HSV1-UL30-1831 | - | ACGACCUGUCCACCACCGCC | 20 | 4881 |
| HSV1-UL30-6516 | - | UACGACCUGUCCACCACCGCC | 21 | 15456 |
| HSV1-UL30-6517 | - | CUACGACCUGUCCACCACCGCC | 22 | 15457 |
| HSV1-UL30-6518 | - | UCUACGACCUGUCCACCACCGCC | 23 | 15458 |
| HSV1-UL30-6519 | - | CUCUACGACCUGUCCACCACCGCC | 24 | 15459 |
| HSV1-UL30-6520 | - | GGGCGAUUUAGGGGCGCC | 18 | 15460 |
| HSV1-UL30-6521 | - | GGGGCGAUUUAGGGGCGCC | 19 | 15461 |
| HSV1-UL30-1885 | - | AGGGGCGAUUUAGGGGCGCC | 20 | 4934 |
| HSV1-UL30-6522 | - | CAGGGGCGAUUUAGGGGCGCC | 21 | 15462 |
| HSV1-UL30-6523 | - | CCAGGGGCGAUUUAGGGGCGCC | 22 | 15463 |
| HSV1-UL30-6524 | - | CCCAGGGGCGAUUUAGGGGCGCC | 23 | 15464 |
| HSV1-UL30-6525 | - | ACCCAGGGGCGAUUUAGGGGCGCC | 24 | 15465 |
| HSV1-UL30-6526 | - | AAGCUGCUGCUGAUCGCC | 18 | 15466 |
| HSV1-UL30-6527 | - | CAAGCUGCUGCUGAUCGCC | 19 | 15467 |
| HSV1-UL30-1983 | - | CCAAGCUGCUGCUGAUCGCC | 20 | 5016 |
| HSV1-UL30-6528 | - | ACCAAGCUGCUGCUGAUCGCC | 21 | 15468 |
| HSV1-UL30-6529 | - | CACCAAGCUGCUGCUGAUCGCC | 22 | 15469 |
| HSV1-UL30-6530 | - | UCACCAAGCUGCUGCUGAUCGCC | 23 | 15470 |
| HSV1-UL30-6531 | - | UUCACCAAGCUGCUGCUGAUCGCC | 24 | 15471 |
| HSV1-UL30-6532 | - | AUUGCCCACGGCGUCGCC | 18 | 15472 |
| HSV1-UL30-6533 | - | CAUUGCCCACGGCGUCGCC | 19 | 15473 |
| HSV1-UL30-2030 | - | CCAUUGCCCACGGCGUCGCC | 20 | 5063 |
| HSV1-UL30-6534 | - | GCCAUUGCCCACGGCGUCGCC | 21 | 15474 |
| HSV1-UL30-6535 | - | CGCCAUUGCCCACGGCGUCGCC | 22 | 15475 |
| HSV1-UL30-6536 | - | ACGCCAUUGCCCACGGCGUCGCC | 23 | 15476 |
| HSV1-UL30-6537 | - | UACGCCAUUGCCCACGGCGUCGCC | 24 | 15477 |
| HSV1-UL30-6538 | - | GCCGCCGGGCUGACGGCC | 18 | 15478 |
| HSV1-UL30-6539 | - | GGCCGCCGGGCUGACGGCC | 19 | 15479 |
| HSV1-UL30-1979 | - | CGGCCGCCGGGCUGACGGCC | 20 | 5012 |
| HSV1-UL30-6540 | - | ACGGCCGCCGGGCUGACGGCC | 21 | 15480 |
| HSV1-UL30-6541 | - | CACGGCCGCCGGGCUGACGGCC | 22 | 15481 |
| HSV1-UL30-6542 | - | UCACGGCCGCCGGGCUGACGGCC | 23 | 15482 |
| HSV1-UL30-6543 | - | CUCACGGCCGCCGGGCUGACGGCC | 24 | 15483 |
| HSV1-UL30-6544 | - | CUGAACGAGCUGGCGGCC | 18 | 15484 |
| HSV1-UL30-6545 | - | CCUGAACGAGCUGGCGGCC | 19 | 15485 |
| HSV1-UL30-1837 | - | ACCUGAACGAGCUGGCGGCC | 20 | 4887 |
| HSV1-UL30-6546 | - | CACCUGAACGAGCUGGCGGCC | 21 | 15486 |
| HSV1-UL30-6547 | - | CCACCUGAACGAGCUGGCGGCC | 22 | 15487 |
| HSV1-UL30-6548 | - | CCCACCUGAACGAGCUGGCGGCC | 23 | 15488 |
| HSV1-UL30-6549 | - | UCCCACCUGAACGAGCUGGCGGCC | 24 | 15489 |
| HSV1-UL30-6550 | - | CGGUGACGACCAUCGGCC | 18 | 15490 |
| HSV1-UL30-6551 | - | ACGGUGACGACCAUCGGCC | 19 | 15491 |
| HSV1-UL30-1967 | - | GACGGUGACGACCAUCGGCC | 20 | 5000 |
| HSV1-UL30-6552 | - | CGACGGUGACGACCAUCGGCC | 21 | 15492 |
| HSV1-UL30-6553 | - | GCGACGGUGACGACCAUCGGCC | 22 | 15493 |
| HSV1-UL30-6554 | - | CGCGACGGUGACGACCAUCGGCC | 23 | 15494 |
| HSV1-UL30-6555 | - | CCGCGACGGUGACGACCAUCGGCC | 24 | 15495 |
| HSV1-UL30-6556 | - | CGCGGCUGGCCGCCCUCC | 18 | 15496 |
| HSV1-UL30-6557 | - | GCGCGGCUGGCCGCCCUCC | 19 | 15497 |
| HSV1-UL30-2016 | - | CGCGCGGCUGGCCGCCCUCC | 20 | 5049 |
| HSV1-UL30-6558 | - | UCGCGCGGCUGGCCGCCCUCC | 21 | 15498 |
| HSV1-UL30-6559 | - | GUCGCGCGGCUGGCCGCCCUCC | 22 | 15499 |
| HSV1-UL30-6560 | - | GGUCGCGCGGCUGGCCGCCCUCC | 23 | 15500 |
| HSV1-UL30-6561 | - | CGGUCGCGCGGCUGGCCGCCCUCC | 24 | 15501 |
| HSV1-UL30-6562 | - | UCGUUCCGCGGCAUCUCC | 18 | 15502 |
| HSV1-UL30-6563 | - | GUCGUUCCGCGGCAUCUCC | 19 | 15503 |
| HSV1-UL30-1787 | - | CGUCGUUCCGCGGCAUCUCC | 20 | 4837 |
| HSV1-UL30-6564 | - | GCGUCGUUCCGCGGCAUCUCC | 21 | 15504 |
| HSV1-UL30-6565 | - | CGCGUCGUUCCGCGGCAUCUCC | 22 | 15505 |
| HSV1-UL30-6566 | - | GCGCGUCGUUCCGCGGCAUCUCC | 23 | 15506 |
| HSV1-UL30-6567 | - | GGCGCGUCGUUCCGCGGCAUCUCC | 24 | 15507 |
| HSV1-UL30-6568 | - | UGCUUCAGCACGCUCUCC | 18 | 15508 |
| HSV1-UL30-6569 | - | GUGCUUCAGCACGCUCUCC | 19 | 15509 |
| HSV1-UL30-1936 | - | UGUGCUUCAGCACGCUCUCC | 20 | 4969 |
| HSV1-UL30-6570 | - | CUGUGCUUCAGCACGCUCUCC | 21 | 15510 |
| HSV1-UL30-6571 | - | CCUGUGCUUCAGCACGCUCUCC | 22 | 15511 |
| HSV1-UL30-6572 | - | ACCUGUGCUUCAGCACGCUCUCC | 23 | 15512 |
| HSV1-UL30-6573 | - | AACCUGUGCUUCAGCACGCUCUCC | 24 | 15513 |
| HSV1-UL30-6574 | - | GCGGCCCUGCGCGAGUCC | 18 | 15514 |
| HSV1-UL30-6575 | - | CGCGGCCCUGCGCGAGUCC | 19 | 15515 |
| HSV1-UL30-1786 | - | CCGCGGCCCUGCGCGAGUCC | 20 | 4836 |
| HSV1-UL30-6576 | - | GCCGCGGCCCUGCGCGAGUCC | 21 | 15516 |
| HSV1-UL30-6577 | - | GGCCGCGGCCCUGCGCGAGUCC | 22 | 15517 |
| HSV1-UL30-6578 | - | UGGCCGCGGCCCUGCGCGAGUCC | 23 | 15518 |
| HSV1-UL30-6579 | - | AUGGCCGCGGCCCUGCGCGAGUCC | 24 | 15519 |
| HSV1-UL30-6580 | - | AGCUCCUGGCCGAUUUCC | 18 | 15520 |
| HSV1-UL30-6581 | - | CAGCUCCUGGCCGAUUUCC | 19 | 15521 |
| HSV1-UL30-1972 | - | ACAGCUCCUGGCCGAUUUCC | 20 | 5005 |
| HSV1-UL30-6582 | - | AACAGCUCCUGGCCGAUUUCC | 21 | 15522 |
| HSV1-UL30-6583 | - | GAACAGCUCCUGGCCGAUUUCC | 22 | 15523 |
| HSV1-UL30-6584 | - | CGAACAGCUCCUGGCCGAUUUCC | 23 | 15524 |
| HSV1-UL30-6585 | - | UCGAACAGCUCCUGGCCGAUUUCC | 24 | 15525 |
| HSV1-UL30-6586 | - | CAAGCGUCCGGCCGCAGC | 18 | 15526 |
| HSV1-UL30-6587 | - | CCAAGCGUCCGGCCGCAGC | 19 | 15527 |
| HSV1-UL30-1890 | - | CCCAAGCGUCCGGCCGCAGC | 20 | 4939 |
| HSV1-UL30-6588 | - | GCCCAAGCGUCCGGCCGCAGC | 21 | 15528 |
| HSV1-UL30-6589 | - | CGCCCAAGCGUCCGGCCGCAGC | 22 | 15529 |
| HSV1-UL30-6590 | - | GCGCCCAAGCGUCCGGCCGCAGC | 23 | 15530 |
| HSV1-UL30-6591 | - | GGCGCCCAAGCGUCCGGCCGCAGC | 24 | 15531 |
| HSV1-UL30-6592 | - | GGUUCACGGGAGUGCAGC | 18 | 15532 |
| HSV1-UL30-6593 | - | GGGUUCACGGGAGUGCAGC | 19 | 15533 |
| HSV1-UL30-1966 | - | CGGGUUCACGGGAGUGCAGC | 20 | 4999 |
| HSV1-UL30-6594 | - | ACGGGUUCACGGGAGUGCAGC | 21 | 15534 |
| HSV1-UL30-6595 | - | UACGGGUUCACGGGAGUGCAGC | 22 | 15535 |
| HSV1-UL30-6596 | - | GUACGGGUUCACGGGAGUGCAGC | 23 | 15536 |
| HSV1-UL30-6597 | - | UGUACGGGUUCACGGGAGUGCAGC | 24 | 15537 |
| HSV1-UL30-6598 | - | AGGAGGGCGGGGGCGAGC | 18 | 15538 |
| HSV1-UL30-6599 | - | GAGGAGGGCGGGGGCGAGC | 19 | 15539 |
| HSV1-UL30-587 | - | CGAGGAGGGCGGGGGCGAGC | 20 | 3525 |
| HSV1-UL30-6600 | - | GCGAGGAGGGCGGGGGCGAGC | 21 | 15540 |
| HSV1-UL30-6601 | - | CGCGAGGAGGGCGGGGGCGAGC | 22 | 15541 |
| HSV1-UL30-6602 | - | ACGCGAGGAGGGCGGGGGCGAGC | 23 | 15542 |
| HSV1-UL30-6603 | - | AACGCGAGGAGGGCGGGGGCGAGC | 24 | 15543 |
| HSV1-UL30-6604 | - | GCGGGGGCGAGCGGGAGC | 18 | 15544 |
| HSV1-UL30-6605 | - | GGCGGGGGCGAGCGGGAGC | 19 | 15545 |
| HSV1-UL30-588 | - | GGGCGGGGGCGAGCGGGAGC | 20 | 3526 |
| HSV1-UL30-6606 | - | AGGGCGGGGGCGAGCGGGAGC | 21 | 15546 |
| HSV1-UL30-6607 | - | GAGGGCGGGGGCGAGCGGGAGC | 22 | 15547 |
| HSV1-UL30-6608 | - | GGAGGGCGGGGGCGAGCGGGAGC | 23 | 15548 |
| HSV1-UL30-6609 | - | AGGAGGGCGGGGGCGAGCGGGAGC | 24 | 15549 |
| HSV1-UL30-6610 | - | GAGGACGAGGACGAACGC | 18 | 15550 |
| HSV1-UL30-6611 | - | GGAGGACGAGGACGAACGC | 19 | 15551 |
| HSV1-UL30-1912 | - | GGGAGGACGAGGACGAACGC | 20 | 4954 |
| HSV1-UL30-6612 | - | GGGGAGGACGAGGACGAACGC | 21 | 15552 |
| HSV1-UL30-6613 | - | GGGGGAGGACGAGGACGAACGC | 22 | 15553 |
| HSV1-UL30-6614 | - | AGGGGGAGGACGAGGACGAACGC | 23 | 15554 |
| HSV1-UL30-6615 | - | GAGGGGGAGGACGAGGACGAACGC | 24 | 15555 |
| HSV1-UL30-6616 | - | CUGGACGGGUACGGCCGC | 18 | 15556 |
| HSV1-UL30-6617 | - | CCUGGACGGGUACGGCCGC | 19 | 15557 |
| HSV1-UL30-1848 | - | CCCUGGACGGGUACGGCCGC | 20 | 4898 |
| HSV1-UL30-6618 | - | CCCCUGGACGGGUACGGCCGC | 21 | 15558 |
| HSV1-UL30-6619 | - | CCCCCUGGACGGGUACGGCCGC | 22 | 15559 |
| HSV1-UL30-6620 | - | UCCCCCUGGACGGGUACGGCCGC | 23 | 15560 |
| HSV1-UL30-6621 | - | GUCCCCCUGGACGGGUACGGCCGC | 24 | 15561 |
| HSV1-UL30-6622 | - | GGGAGCCGGAGGGCGCGC | 18 | 15562 |
| HSV1-UL30-6623 | - | CGGGAGCCGGAGGGCGCGC | 19 | 15563 |
| HSV1-UL30-592 | - | GCGGGAGCCGGAGGGCGCGC | 20 | 3530 |
| HSV1-UL30-6624 | - | AGCGGGAGCCGGAGGGCGCGC | 21 | 15564 |
| HSV1-UL30-6625 | - | GAGCGGGAGCCGGAGGGCGCGC | 22 | 15565 |
| HSV1-UL30-6626 | - | CGAGCGGGAGCCGGAGGGCGCGC | 23 | 15566 |
| HSV1-UL30-6627 | - | GCGAGCGGGAGCCGGAGGGCGCGC | 24 | 15567 |
| HSV1-UL30-6628 | - | GCGGGAGCCGGAGGGCGC | 18 | 15568 |
| HSV1-UL30-6629 | - | AGCGGGAGCCGGAGGGCGC | 19 | 15569 |
| HSV1-UL30-1925 | - | GAGCGGGAGCCGGAGGGCGC | 20 | 4961 |
| HSV1-UL30-6630 | - | CGAGCGGGAGCCGGAGGGCGC | 21 | 15570 |
| HSV1-UL30-6631 | - | GCGAGCGGGAGCCGGAGGGCGC | 22 | 15571 |
| HSV1-UL30-6632 | - | GGCGAGCGGGAGCCGGAGGGCGC | 23 | 15572 |
| HSV1-UL30-6633 | - | GGGCGAGCGGGAGCCGGAGGGCGC | 24 | 15573 |
| HSV1-UL30-6634 | - | GGGGCGAUUUAGGGGCGC | 18 | 15574 |
| HSV1-UL30-6635 | - | AGGGGCGAUUUAGGGGCGC | 19 | 15575 |
| HSV1-UL30-1884 | - | CAGGGGCGAUUUAGGGGCGC | 20 | 4933 |
| HSV1-UL30-6636 | - | CCAGGGGCGAUUUAGGGGCGC | 21 | 15576 |
| HSV1-UL30-6637 | - | CCCAGGGGCGAUUUAGGGGCGC | 22 | 15577 |
| HSV1-UL30-6638 | - | ACCCAGGGGCGAUUUAGGGGCGC | 23 | 15578 |
| HSV1-UL30-6639 | - | CACCCAGGGGCGAUUUAGGGGCGC | 24 | 15579 |
| HSV1-UL30-6640 | - | CGAUAUCGAAUGCAAGGC | 18 | 15580 |
| HSV1-UL30-6641 | - | UCGAUAUCGAAUGCAAGGC | 19 | 15581 |
| HSV1-UL30-1819 | - | UUCGAUAUCGAAUGCAAGGC | 20 | 4869 |
| HSV1-UL30-6642 | - | CUUCGAUAUCGAAUGCAAGGC | 21 | 15582 |
| HSV1-UL30-6643 | - | GCUUCGAUAUCGAAUGCAAGGC | 22 | 15583 |
| HSV1-UL30-6644 | - | UGCUUCGAUAUCGAAUGCAAGGC | 23 | 15584 |
| HSV1-UL30-6645 | - | GUGCUUCGAUAUCGAAUGCAAGGC | 24 | 15585 |
| HSV1-UL30-6646 | - | CGCGUGUGGGACAUAGGC | 18 | 15586 |
| HSV1-UL30-6647 | - | UCGCGUGUGGGACAUAGGC | 19 | 15587 |
| HSV1-UL30-1853 | - | UUCGCGUGUGGGACAUAGGC | 20 | 4903 |
| HSV1-UL30-6648 | - | UUUCGCGUGUGGGACAUAGGC | 21 | 15588 |
| HSV1-UL30-6649 | - | GUUUCGCGUGUGGGACAUAGGC | 22 | 15589 |
| HSV1-UL30-6650 | - | UGUUUCGCGUGUGGGACAUAGGC | 23 | 15590 |
| HSV1-UL30-6651 | - | GUGUUUC GC GUGUGGGACAUAGGC | 24 | 15591 |
| HSV1-UL30-6652 | - | UACGGCCGCAUGAACGGC | 18 | 15592 |
| HSV1-UL30-6653 | - | GUACGGCCGCAUGAACGGC | 19 | 15593 |
| HSV1-UL30-1850 | - | GGUACGGCCGCAUGAACGGC | 20 | 4900 |
| HSV1-UL30-6654 | - | GGGUACGGCCGCAUGAACGGC | 21 | 15594 |
| HSV1-UL30-6655 | - | CGGGUACGGCCGCAUGAACGGC | 22 | 15595 |
| HSV1-UL30-6656 | - | ACGGGUACGGCCGCAUGAACGGC | 23 | 15596 |
| HSV1-UL30-6657 | - | GACGGGUACGGCCGCAUGAACGGC | 24 | 15597 |
| HSV1-UL30-6658 | - | GGGAGGACGAGGAGCGGC | 18 | 15598 |
| HSV1-UL30-6659 | - | CGGGAGGACGAGGAGCGGC | 19 | 15599 |
| HSV1-UL30-1897 | - | CCGGGAGGACGAGGAGCGGC | 20 | 4945 |
| HSV1-UL30-6660 | - | CCCGGGAGGACGAGGAGCGGC | 21 | 15600 |
| HSV1-UL30-6661 | - | GCCCGGGAGGACGAGGAGCGGC | 22 | 15601 |
| HSV1-UL30-6662 | - | AGCCCGGGAGGACGAGGAGCGGC | 23 | 15602 |
| HSV1-UL30-6663 | - | CAGCCCGGGAGGACGAGGAGCGGC | 24 | 15603 |
| HSV1-UL30-6664 | - | CCUGAACGAGCUGGCGGC | 18 | 15604 |
| HSV1-UL30-6665 | - | ACCUGAACGAGCUGGCGGC | 19 | 15605 |
| HSV1-UL30-1836 | - | CACCUGAACGAGCUGGCGGC | 20 | 4886 |
| HSV1-UL30-6666 | - | CCACCUGAACGAGCUGGCGGC | 21 | 15606 |
| HSV1-UL30-6667 | - | CCCACCUGAACGAGCUGGCGGC | 22 | 15607 |
| HSV1-UL30-6668 | - | UCCCACCUGAACGAGCUGGCGGC | 23 | 15608 |
| HSV1-UL30-6669 | - | AUCCCACCUGAACGAGCUGGCGGC | 24 | 15609 |
| HSV1-UL30-6670 | - | GACGAACGCGAGGAGGGC | 18 | 15610 |
| HSV1-UL30-6671 | - | GGACGAACGCGAGGAGGGC | 19 | 15611 |
| HSV1-UL30-583 | - | AGGACGAACGCGAGGAGGGC | 20 | 3521 |
| HSV1-UL30-6672 | - | GAGGACGAACGCGAGGAGGGC | 21 | 15612 |
| HSV1-UL30-6673 | - | CGAGGACGAACGCGAGGAGGGC | 22 | 15613 |
| HSV1-UL30-6674 | - | ACGAGGACGAACGCGAGGAGGGC | 23 | 15614 |
| HSV1-UL30-6675 | - | GACGAGGACGAACGCGAGGAGGGC | 24 | 15615 |
| HSV1-UL30-6676 | - | UACCGUCUCAAACCGGGC | 18 | 15616 |
| HSV1-UL30-6677 | - | GUACCGUCUCAAACCGGGC | 19 | 15617 |
| HSV1-UL30-1805 | - | GGUACCGUCUCAAACCGGGC | 20 | 4855 |
| HSV1-UL30-6678 | - | UGGUACCGUCUCAAACCGGGC | 21 | 15618 |
| HSV1-UL30-6679 | - | CUGGUACCGUCUCAAACCGGGC | 22 | 15619 |
| HSV1-UL30-6680 | - | GCUGGUACCGUCUCAAACCGGGC | 23 | 15620 |
| HSV1-UL30-6681 | - | GGCUGGUACCGUCUCAAACCGGGC | 24 | 15621 |
| HSV1-UL30-6682 | - | GCGCACCUGGAGGCGGGC | 18 | 15622 |
| HSV1-UL30-6683 | - | GGCGCACCUGGAGGCGGGC | 19 | 15623 |
| HSV1-UL30-1941 | - | UGGCGCACCUGGAGGCGGGC | 20 | 4974 |
| HSV1-UL30-6684 | - | GUGGCGCACCUGGAGGCGGGC | 21 | 15624 |
| HSV1-UL30-6685 | - | AGUGGCGCACCUGGAGGCGGGC | 22 | 15625 |
| HSV1-UL30-6686 | - | CAGUGGCGCACCUGGAGGCGGGC | 23 | 15626 |
| HSV1-UL30-6687 | - | GCAGUGGCGCACCUGGAGGCGGGC | 24 | 15627 |
| HSV1-UL30-6688 | - | CUGGCCAUCGAGGGGGGC | 18 | 15628 |
| HSV1-UL30-6689 | - | CCUGGCCAUCGAGGGGGGC | 19 | 15629 |
| HSV1-UL30-1815 | - | ACCUGGCCAUCGAGGGGGGC | 20 | 4865 |
| HSV1-UL30-6690 | - | AACCUGGCCAUCGAGGGGGGC | 21 | 15630 |
| HSV1-UL30-6691 | - | CAACCUGGCCAUCGAGGGGGGC | 22 | 15631 |
| HSV1-UL30-6692 | - | ACAACCUGGCCAUCGAGGGGGGC | 23 | 15632 |
| HSV1-UL30-6693 | - | GACAACCUGGCCAUCGAGGGGGGC | 24 | 15633 |
| HSV1-UL30-6694 | - | GGCUGGCGCGACCCCUGC | 18 | 15634 |
| HSV1-UL30-6695 | - | UGGCUGGCGCGACCCCUGC | 19 | 15635 |
| HSV1-UL30-1997 | - | GUGGCUGGCGCGACCCCUGC | 20 | 5030 |
| HSV1-UL30-6696 | - | AGUGGCUGGCGCGACCCCUGC | 21 | 15636 |
| HSV1-UL30-6697 | - | GAGUGGCUGGCGCGACCCCUGC | 22 | 15637 |
| HSV1-UL30-6698 | - | GGAGUGGCUGGCGCGACCCCUGC | 23 | 15638 |
| HSV1-UL30-6699 | - | AGGAGUGGCUGGCGCGACCCCUGC | 24 | 15639 |
| HSV1-UL30-6700 | - | UC GUCAAGGCUCAC GUGC | 18 | 15640 |
| HSV1-UL30-6701 | - | UUC GUCAAGGCUCAC GUGC | 19 | 15641 |
| HSV1-UL30-1951 | - | CUUCGUCAAGGCUCACGUGC | 20 | 4984 |
| HSV1-UL30-6702 | - | UCUUCGUCAAGGCUCACGUGC | 21 | 15642 |
| HSV1-UL30-6703 | - | UUCUUCGUCAAGGCUCACGUGC | 22 | 15643 |
| HSV1-UL30-6704 | - | GUUCUUCGUCAAGGCUCACGUGC | 23 | 15644 |
| HSV1-UL30-6705 | - | UGUUCUUCGUCAAGGCUCACGUGC | 24 | 15645 |
| HSV1-UL30-6706 | - | GACCCGGAGAGGGACAUC | 18 | 15646 |
| HSV1-UL30-6707 | - | CGACCCGGAGAGGGACAUC | 19 | 15647 |
| HSV1-UL30-2007 | - | CCGACCCGGAGAGGGACAUC | 20 | 5040 |
| HSV1-UL30-6708 | - | ACCGACCCGGAGAGGGACAUC | 21 | 15648 |
| HSV1-UL30-6709 | - | CACCGACCCGGAGAGGGACAUC | 22 | 15649 |
| HSV1-UL30-6710 | - | UCACCGACCCGGAGAGGGACAUC | 23 | 15650 |
| HSV1-UL30-6711 | - | AUCACCGACCCGGAGAGGGACAUC | 24 | 15651 |
| HSV1-UL30-6712 | - | UUAAGUUUUUGCCCCAUC | 18 | 15652 |
| HSV1-UL30-6713 | - | UUUAAGUUUUUGCCCCAUC | 19 | 15653 |
| HSV1-UL30-1874 | - | UUUUAAGUUUUUGCCCCAUC | 20 | 4923 |
| HSV1-UL30-6714 | - | UUUUUAAGUUUUUGCCCCAUC | 21 | 15654 |
| HSV1-UL30-6715 | - | UUUUUUAAGUUUUUGCCCCAUC | 22 | 15655 |
| HSV1-UL30-6716 | - | GUUUUUUAAGUUUUUGCCCCAUC | 23 | 15656 |
| HSV1-UL30-6717 | - | UGUUUUUUAAGUUUUUGCCCCAUC | 24 | 15657 |
| HSV1-UL30-6718 | - | AACUUCUGCCCGGCCAUC | 18 | 15658 |
| HSV1-UL30-6719 | - | CAACUUCUGCCCGGCCAUC | 19 | 15659 |
| HSV1-UL30-1796 | - | ACAACUUCUGCCCGGCCAUC | 20 | 4846 |
| HSV1-UL30-6720 | - | GACAACUUCUGCCCGGCCAUC | 21 | 15660 |
| HSV1-UL30-6721 | - | CGACAACUUCUGCCCGGCCAUC | 22 | 15661 |
| HSV1-UL30-6722 | - | GCGACAACUUCUGCCCGGCCAUC | 23 | 15662 |
| HSV1-UL30-6723 | - | UGCGACAACUUCUGCCCGGCCAUC | 24 | 15663 |
| HSV1-UL30-6724 | - | GCGGACAACCUGGCCAUC | 18 | 15664 |
| HSV1-UL30-6725 | - | GGCGGACAACCUGGCCAUC | 19 | 15665 |
| HSV1-UL30-1811 | - | CGGCGGACAACCUGGCCAUC | 20 | 4861 |
| HSV1-UL30-6726 | - | ACGGCGGACAACCUGGCCAUC | 21 | 15666 |
| HSV1-UL30-6727 | - | UACGGCGGACAACCUGGCCAUC | 22 | 15667 |
| HSV1-UL30-6728 | - | GUACGGCGGACAACCUGGCCAUC | 23 | 15668 |
| HSV1-UL30-6729 | - | UGUACGGCGGACAACCUGGCCAUC | 24 | 15669 |
| HSV1-UL30-6730 | - | GCGCAGGUCCCGUCCAUC | 18 | 15670 |
| HSV1-UL30-6731 | - | CGCGCAGGUCCCGUCCAUC | 19 | 15671 |
| HSV1-UL30-2010 | - | GCGCGCAGGUCCCGUCCAUC | 20 | 5043 |
| HSV1-UL30-6732 | - | CGCGCGCAGGUCCCGUCCAUC | 21 | 15672 |
| HSV1-UL30-6733 | - | CCGCGCGCAGGUCCCGUCCAUC | 22 | 15673 |
| HSV1-UL30-6734 | - | GCCGCGCGCAGGUCCCGUCCAUC | 23 | 15674 |
| HSV1-UL30-6735 | - | CGCCGCGCGCAGGUCCCGUCCAUC | 24 | 15675 |
| HSV1-UL30-6736 | - | GGGGGUAAGAUGCUCAUC | 18 | 15676 |
| HSV1-UL30-6737 | - | CGGGGGUAAGAUGCUCAUC | 19 | 15677 |
| HSV1-UL30-1987 | - | ACGGGGGUAAGAUGCUCAUC | 20 | 5020 |
| HSV1-UL30-6738 | - | UACGGGGGUAAGAUGCUCAUC | 21 | 15678 |
| HSV1-UL30-6739 | - | CUACGGGGGUAAGAUGCUCAUC | 22 | 15679 |
| HSV1-UL30-6740 | - | UCUACGGGGGUAAGAUGCUCAUC | 23 | 15680 |
| HSV1-UL30-6741 | - | AUCUACGGGGGUAAGAUGCUCAUC | 24 | 15681 |
| HSV1-UL30-6742 | - | GCCACCACCCGGUUCAUC | 18 | 15682 |
| HSV1-UL30-6743 | - | CGCCACCACCCGGUUCAUC | 19 | 15683 |
| HSV1-UL30-1801 | - | ACGCCACCACCCGGUUCAUC | 20 | 4851 |
| HSV1-UL30-6744 | - | GACGCCACCACCCGGUUCAUC | 21 | 15684 |
| HSV1-UL30-6745 | - | CGACGCCACCACCCGGUUCAUC | 22 | 15685 |
| HSV1-UL30-6746 | - | UCGACGCCACCACCCGGUUCAUC | 23 | 15686 |
| HSV1-UL30-6747 | - | GUCGACGCCACCACCCGGUUCAUC | 24 | 15687 |
| HSV1-UL30-6748 | - | CGCUCCUGGGCCUGACUC | 18 | 15688 |
| HSV1-UL30-6749 | - | ACGCUCCUGGGCCUGACUC | 19 | 15689 |
| HSV1-UL30-1776 | - | CACGCUCCUGGGCCUGACUC | 20 | 4826 |
| HSV1-UL30-6750 | - | UCACGCUCCUGGGCCUGACUC | 21 | 15690 |
| HSV1-UL30-6751 | - | AUCACGCUCCUGGGCCUGACUC | 22 | 15691 |
| HSV1-UL30-6752 | - | CAUCACGCUCCUGGGCCUGACUC | 23 | 15692 |
| HSV1-UL30-6753 | - | UCAUCACGCUCCUGGGCCUGACUC | 24 | 15693 |
| HSV1-UL30-6754 | - | GUUUAUCAACCGCACCUC | 18 | 15694 |
| HSV1-UL30-6755 | - | CGUUUAUCAACCGCACCUC | 19 | 15695 |
| HSV1-UL30-1989 | - | GCGUUUAUCAACCGCACCUC | 20 | 5022 |
| HSV1-UL30-6756 | - | CGCGUUUAUCAACCGCACCUC | 21 | 15696 |
| HSV1-UL30-6757 | - | GCGCGUUUAUCAACCGCACCUC | 22 | 15697 |
| HSV1-UL30-6758 | - | UGCGCGUUUAUCAACCGCACCUC | 23 | 15698 |
| HSV1-UL30-6759 | - | CUGCGCGUUUAUCAACCGCACCUC | 24 | 15699 |
| HSV1-UL30-6760 | - | ACCGACAAGAUCAAGCUC | 18 | 15700 |
| HSV1-UL30-6761 | - | AACCGACAAGAUCAAGCUC | 19 | 15701 |
| HSV1-UL30-1859 | - | UAACCGACAAGAUCAAGCUC | 20 | 4909 |
| HSV1-UL30-6762 | - | AUAACCGACAAGAUCAAGCUC | 21 | 15702 |
| HSV1-UL30-6763 | - | UAUAACCGACAAGAUCAAGCUC | 22 | 15703 |
| HSV1-UL30-6764 | - | UUAUAACCGACAAGAUCAAGCUC | 23 | 15704 |
| HSV1-UL30-6765 | - | AUUAUAACCGACAAGAUCAAGCUC | 24 | 15705 |
| HSV1-UL30-6766 | - | GGGACCGUCAUCACGCUC | 18 | 15706 |
| HSV1-UL30-6767 | - | GGGGACCGUCAUCACGCUC | 19 | 15707 |
| HSV1-UL30-1774 | - | CGGGGACCGUCAUCACGCUC | 20 | 4824 |
| HSV1-UL30-6768 | - | ACGGGGACCGUCAUCACGCUC | 21 | 15708 |
| HSV1-UL30-6769 | - | GACGGGGACCGUCAUCACGCUC | 22 | 15709 |
| HSV1-UL30-6770 | - | CGACGGGGACCGUCAUCACGCUC | 23 | 15710 |
| HSV1-UL30-6771 | - | CCGACGGGGACCGUCAUCACGCUC | 24 | 15711 |
| HSV1-UL30-6772 | - | CCCGAGGAGGCCGUGCUC | 18 | 15712 |
| HSV1-UL30-6773 | - | CCCCGAGGAGGCCGUGCUC | 19 | 15713 |
| HSV1-UL30-1961 | - | GCCCCGAGGAGGCCGUGCUC | 20 | 4994 |
| HSV1-UL30-6774 | - | AGCCCCGAGGAGGCCGUGCUC | 21 | 15714 |
| HSV1-UL30-6775 | - | CAGCCCCGAGGAGGCCGUGCUC | 22 | 15715 |
| HSV1-UL30-6776 | - | GCAGCCCCGAGGAGGCCGUGCUC | 23 | 15716 |
| HSV1-UL30-6777 | - | AGCAGCCCCGAGGAGGCCGUGCUC | 24 | 15717 |
| HSV1-UL30-6778 | - | UUACGACGAUACCGUCUC | 18 | 15718 |
| HSV1-UL30-6779 | - | UUUACGACGAUACCGUCUC | 19 | 15719 |
| HSV1-UL30-1991 | - | UUUUACGACGAUACCGUCUC | 20 | 5024 |
| HSV1-UL30-6780 | - | GUUUUACGACGAUACCGUCUC | 21 | 15720 |
| HSV1-UL30-6781 | - | UGUUUUACGACGAUACCGUCUC | 22 | 15721 |
| HSV1-UL30-6782 | - | CUGUUUUACGACGAUACCGUCUC | 23 | 15722 |
| HSV1-UL30-6783 | - | GCUGUUUUACGACGAUACCGUCUC | 24 | 15723 |
| HSV1-UL30-6784 | - | GCCGUGGCCGAAGCCGUC | 18 | 15724 |
| HSV1-UL30-6785 | - | CGCCGUGGCCGAAGCCGUC | 19 | 15725 |
| HSV1-UL30-1861 | - | ACGCCGUGGCCGAAGCCGUC | 20 | 4911 |
| HSV1-UL30-6786 | - | AACGCCGUGGCCGAAGCCGUC | 21 | 15726 |
| HSV1-UL30-6787 | - | CAACGCCGUGGCCGAAGCCGUC | 22 | 15727 |
| HSV1-UL30-6788 | - | UCAACGCCGUGGCCGAAGCCGUC | 23 | 15728 |
| HSV1-UL30-6789 | - | CUCAACGCCGUGGCCGAAGCCGUC | 24 | 15729 |
| HSV1-UL30-6790 | - | UGUUAAAAAGGUUUAUUC | 18 | 15730 |
| HSV1-UL30-6791 | - | CUGUUAAAAAGGUUUAUUC | 19 | 15731 |
| HSV1-UL30-2040 | - | UCUGUUAAAAAGGUUUAUUC | 20 | 5073 |
| HSV1-UL30-6792 | - | GUCUGUUAAAAAGGUUUAUUC | 21 | 15732 |
| HSV1-UL30-6793 | - | AGUCUGUUAAAAAGGUUUAUUC | 22 | 15733 |
| HSV1-UL30-6794 | - | GAGUCUGUUAAAAAGGUUUAUUC | 23 | 15734 |
| HSV1-UL30-6795 | - | AGAGUCUGUUAAAAAGGUUUAUUC | 24 | 15735 |
| HSV1-UL30-6796 | - | AUAGGCCAGAGCCACUUC | 18 | 15736 |
| HSV1-UL30-6797 | - | CAUAGGCCAGAGCCACUUC | 19 | 15737 |
| HSV1-UL30-1854 | - | ACAUAGGCCAGAGCCACUUC | 20 | 4904 |
| HSV1-UL30-6798 | - | GACAUAGGCCAGAGCCACUUC | 21 | 15738 |
| HSV1-UL30-6799 | - | GGACAUAGGCCAGAGCCACUUC | 22 | 15739 |
| HSV1-UL30-6800 | - | GGGACAUAGGCCAGAGCCACUUC | 23 | 15740 |
| HSV1-UL30-6801 | - | UGGGACAUAGGCCAGAGCCACUUC | 24 | 15741 |
| HSV1-UL30-6802 | - | AACUCGGUGUACGGGUUC | 18 | 15742 |
| HSV1-UL30-6803 | - | UAACUCGGUGUACGGGUUC | 19 | 15743 |
| HSV1-UL30-1963 | - | GUAACUCGGUGUACGGGUUC | 20 | 4996 |
| HSV1-UL30-6804 | - | UGUAACUCGGUGUACGGGUUC | 21 | 15744 |
| HSV1-UL30-6805 | - | GUGUAACUCGGUGUACGGGUUC | 22 | 15745 |
| HSV1-UL30-6806 | - | UGUGUAACUCGGUGUACGGGUUC | 23 | 15746 |
| HSV1-UL30-6807 | - | GUGUGUAACUCGGUGUACGGGUUC | 24 | 15747 |
| HSV1-UL30-6808 | - | CAGCUCCUGGCCGAUUUC | 18 | 15748 |
| HSV1-UL30-6809 | - | ACAGCUCCUGGCCGAUUUC | 19 | 15749 |
| HSV1-UL30-1971 | - | AACAGCUCCUGGCCGAUUUC | 20 | 5004 |
| HSV1-UL30-6810 | - | GAACAGCUCCUGGCCGAUUUC | 21 | 15750 |
| HSV1-UL30-6811 | - | CGAACAGCUCCUGGCCGAUUUC | 22 | 15751 |
| HSV1-UL30-6812 | - | UCGAACAGCUCCUGGCCGAUUUC | 23 | 15752 |
| HSV1-UL30-6813 | - | UUCGAACAGCUCCUGGCCGAUUUC | 24 | 15753 |
| HSV1-UL30-6814 | - | AAGCGCAGCAAGAUAAAG | 18 | 15754 |
| HSV1-UL30-6815 | - | GAAGCGCAGCAAGAUAAAG | 19 | 15755 |
| HSV1-UL30-1855 | - | AGAAGCGCAGCAAGAUAAAG | 20 | 4905 |
| HSV1-UL30-6816 | - | CAGAAGCGCAGCAAGAUAAAG | 21 | 15756 |
| HSV1-UL30-6817 | - | CCAGAAGCGCAGCAAGAUAAAG | 22 | 15757 |
| HSV1-UL30-6818 | - | UCCAGAAGCGCAGCAAGAUAAAG | 23 | 15758 |
| HSV1-UL30-6819 | - | UUCCAGAAGCGCAGCAAGAUAAAG | 24 | 15759 |
| HSV1-UL30-6820 | - | UACUUUUACAUGAACAAG | 18 | 15760 |
| HSV1-UL30-6821 | - | GUACUUUUACAUGAACAAG | 19 | 15761 |
| HSV1-UL30-1781 | - | AGUACUUUUACAUGAACAAG | 20 | 4831 |
| HSV1-UL30-6822 | - | CAGUACUUUUACAUGAACAAG | 21 | 15762 |
| HSV1-UL30-6823 | - | GCAGUACUUUUACAUGAACAAG | 22 | 15763 |
| HSV1-UL30-6824 | - | GGCAGUACUUUUACAUGAACAAG | 23 | 15764 |
| HSV1-UL30-6825 | - | CGGCAGUACUUUUACAUGAACAAG | 24 | 15765 |
| HSV1-UL30-6826 | - | GCCGUCCUGAAGGACAAG | 18 | 15766 |
| HSV1-UL30-6827 | - | AGCCGUCCUGAAGGACAAG | 19 | 15767 |
| HSV1-UL30-1864 | - | AAGCCGUCCUGAAGGACAAG | 20 | 4914 |
| HSV1-UL30-6828 | - | GAAGCCGUCCUGAAGGACAAG | 21 | 15768 |
| HSV1-UL30-6829 | - | CGAAGCCGUCCUGAAGGACAAG | 22 | 15769 |
| HSV1-UL30-6830 | - | CCGAAGCCGUCCUGAAGGACAAG | 23 | 15770 |
| HSV1-UL30-6831 | - | GCCGAAGCCGUCCUGAAGGACAAG | 24 | 15771 |
| HSV1-UL30-6832 | - | UUCGAUAUCGAAUGCAAG | 18 | 15772 |
| HSV1-UL30-6833 | - | CUUCGAUAUCGAAUGCAAG | 19 | 15773 |
| HSV1-UL30-1817 | - | GCUUCGAUAUCGAAUGCAAG | 20 | 4867 |
| HSV1-UL30-6834 | - | UGCUUCGAUAUCGAAUGCAAG | 21 | 15774 |
| HSV1-UL30-6835 | - | GUGCUUCGAUAUCGAAUGCAAG | 22 | 15775 |
| HSV1-UL30-6836 | - | UGUGCUUCGAUAUCGAAUGCAAG | 23 | 15776 |
| HSV1-UL30-6837 | - | AUGUGCUUCGAUAUCGAAUGCAAG | 24 | 15777 |
| HSV1-UL30-6838 | - | GUCCUGAAGGACAAGAAG | 18 | 15778 |
| HSV1-UL30-6839 | - | CGUCCUGAAGGACAAGAAG | 19 | 15779 |
| HSV1-UL30-1865 | - | CCGUCCUGAAGGACAAGAAG | 20 | 4915 |
| HSV1-UL30-6840 | - | GCCGUCCUGAAGGACAAGAAG | 21 | 15780 |
| HSV1-UL30-6841 | - | AGCCGUCCUGAAGGACAAGAAG | 22 | 15781 |
| HSV1-UL30-6842 | - | AAGCCGUCCUGAAGGACAAGAAG | 23 | 15782 |
| HSV1-UL30-6843 | - | GAAGCCGUCCUGAAGGACAAGAAG | 24 | 15783 |
| HSV1-UL30-6844 | - | CUGCGCCUGGCCGACCAG | 18 | 15784 |
| HSV1-UL30-6845 | - | GCUGCGCCUGGCCGACCAG | 19 | 15785 |
| HSV1-UL30-1877 | - | UGCUGCGCCUGGCCGACCAG | 20 | 4926 |
| HSV1-UL30-6846 | - | CUGCUGCGCCUGGCCGACCAG | 21 | 15786 |
| HSV1-UL30-6847 | - | CCUGCUGCGCCUGGCCGACCAG | 22 | 15787 |
| HSV1-UL30-6848 | - | GCCUGCUGCGCCUGGCCGACCAG | 23 | 15788 |
| HSV1-UL30-6849 | - | UGCCUGCUGCGCCUGGCCGACCAG | 24 | 15789 |
| HSV1-UL30-6850 | - | AGGACGAGGAGCGGCCAG | 18 | 15790 |
| HSV1-UL30-6851 | - | GAGGACGAGGAGCGGCCAG | 19 | 15791 |
| HSV1-UL30-571 | - | GGAGGACGAGGAGCGGCCAG | 20 | 3509 |
| HSV1-UL30-6852 | - | GGGAGGACGAGGAGCGGCCAG | 21 | 15792 |
| HSV1-UL30-6853 | - | CGGGAGGACGAGGAGCGGCCAG | 22 | 15793 |
| HSV1-UL30-6854 | - | CCGGGAGGACGAGGAGCGGCCAG | 23 | 15794 |
| HSV1-UL30-6855 | - | CCCGGGAGGACGAGGAGCGGCCAG | 24 | 15795 |
| HSV1-UL30-6856 | - | GACGAGGAGCGGCCAGAG | 18 | 15796 |
| HSV1-UL30-6857 | - | GGACGAGGAGCGGCCAGAG | 19 | 15797 |
| HSV1-UL30-1900 | - | AGGACGAGGAGCGGCCAGAG | 20 | 4947 |
| HSV1-UL30-6858 | - | GAGGACGAGGAGCGGCCAGAG | 21 | 15798 |
| HSV1-UL30-6859 | - | GGAGGACGAGGAGCGGCCAGAG | 22 | 15799 |
| HSV1-UL30-6860 | - | GGGAGGACGAGGAGCGGCCAGAG | 23 | 15800 |
| HSV1-UL30-6861 | - | CGGGAGGACGAGGAGCGGCCAGAG | 24 | 15801 |
| HSV1-UL30-6862 | - | GACGAGGACGAACGCGAG | 18 | 15802 |
| HSV1-UL30-6863 | - | GGACGAGGACGAACGCGAG | 19 | 15803 |
| HSV1-UL30-1914 | - | AGGACGAGGACGAACGCGAG | 20 | 4955 |
| HSV1-UL30-6864 | - | GAGGACGAGGACGAACGCGAG | 21 | 15804 |
| HSV1-UL30-6865 | - | GGAGGACGAGGACGAACGCGAG | 22 | 15805 |
| HSV1-UL30-6866 | - | GGGAGGACGAGGACGAACGCGAG | 23 | 15806 |
| HSV1-UL30-6867 | - | GGGGAGGACGAGGACGAACGCGAG | 24 | 15807 |
| HSV1-UL30-6868 | - | GAGGAGGGCGGGGGCGAG | 18 | 15808 |
| HSV1-UL30-6869 | - | CGAGGAGGGCGGGGGCGAG | 19 | 15809 |
| HSV1-UL30-586 | - | GCGAGGAGGGCGGGGGCGAG | 20 | 3524 |
| HSV1-UL30-6870 | - | CGCGAGGAGGGCGGGGGCGAG | 21 | 15810 |
| HSV1-UL30-6871 | - | ACGCGAGGAGGGCGGGGGCGAG | 22 | 15811 |
| HSV1-UL30-6872 | - | AACGCGAGGAGGGCGGGGGCGAG | 23 | 15812 |
| HSV1-UL30-6873 | - | GAACGCGAGGAGGGCGGGGGCGAG | 24 | 15813 |
| HSV1-UL30-6874 | - | GACAACCUGGCCAUCGAG | 18 | 15814 |
| HSV1-UL30-6875 | - | GGACAACCUGGCCAUCGAG | 19 | 15815 |
| HSV1-UL30-1814 | - | CGGACAACCUGGCCAUCGAG | 20 | 4864 |
| HSV1-UL30-6876 | - | GCGGACAACCUGGCCAUCGAG | 21 | 15816 |
| HSV1-UL30-6877 | - | GGCGGACAACCUGGCCAUCGAG | 22 | 15817 |
| HSV1-UL30-6878 | - | CGGCGGACAACCUGGCCAUCGAG | 23 | 15818 |
| HSV1-UL30-6879 | - | ACGGCGGACAACCUGGCCAUCGAG | 24 | 15819 |
| HSV1-UL30-6880 | - | GACUACCUGGAGAUCGAG | 18 | 15820 |
| HSV1-UL30-6881 | - | GGACUACCUGGAGAUCGAG | 19 | 15821 |
| HSV1-UL30-1945 | - | AGGACUACCUGGAGAUCGAG | 20 | 4978 |
| HSV1-UL30-6882 | - | AAGGACUACCUGGAGAUCGAG | 21 | 15822 |
| HSV1-UL30-6883 | - | CAAGGACUAC CUGGAGAUC GAG | 22 | 15823 |
| HSV1-UL30-6884 | - | GCAAGGACUACCUGGAGAUCGAG | 23 | 15824 |
| HSV1-UL30-6885 | - | GGCAAGGACUACCUGGAGAUCGAG | 24 | 15825 |
| HSV1-UL30-6886 | - | UCCGCGGACCACUUCGAG | 18 | 15826 |
| HSV1-UL30-6887 | - | CUCCGCGGACCACUUCGAG | 19 | 15827 |
| HSV1-UL30-1789 | - | UCUCCGCGGACCACUUCGAG | 20 | 4839 |
| HSV1-UL30-6888 | - | AUCUCCGCGGACCACUUCGAG | 21 | 15828 |
| HSV1-UL30-6889 | - | CAUCUCCGCGGACCACUUCGAG | 22 | 15829 |
| HSV1-UL30-6890 | - | GCAUCUCCGCGGACCACUUCGAG | 23 | 15830 |
| HSV1-UL30-6891 | - | GGCAUCUCCGCGGACCACUUCGAG | 24 | 15831 |
| HSV1-UL30-6892 | - | GAGGAGCGGCCAGAGGAG | 18 | 15832 |
| HSV1-UL30-6893 | - | CGAGGAGCGGCCAGAGGAG | 19 | 15833 |
| HSV1-UL30-1902 | - | ACGAGGAGCGGCCAGAGGAG | 20 | 4948 |
| HSV1-UL30-6894 | - | GACGAGGAGCGGCCAGAGGAG | 21 | 15834 |
| HSV1-UL30-6895 | - | GGACGAGGAGCGGCCAGAGGAG | 22 | 15835 |
| HSV1-UL30-6896 | - | AGGACGAGGAGCGGCCAGAGGAG | 23 | 15836 |
| HSV1-UL30-6897 | - | GAGGACGAGGAGCGGCCAGAGGAG | 24 | 15837 |
| HSV1-UL30-6898 | - | GAGCGGCCAGAGGAGGAG | 18 | 15838 |
| HSV1-UL30-6899 | - | GGAGCGGCCAGAGGAGGAG | 19 | 15839 |
| HSV1-UL30-575 | - | AGGAGCGGCCAGAGGAGGAG | 20 | 3513 |
| HSV1-UL30-6900 | - | GAGGAGCGGCCAGAGGAGGAG | 21 | 15840 |
| HSV1-UL30-6901 | - | CGAGGAGCGGCCAGAGGAGGAG | 22 | 15841 |
| HSV1-UL30-6902 | - | ACGAGGAGCGGCCAGAGGAGGAG | 23 | 15842 |
| HSV1-UL30-6903 | - | GACGAGGAGCGGCCAGAGGAGGAG | 24 | 15843 |
| HSV1-UL30-6904 | - | CGCAUCACCGACCCGGAG | 18 | 15844 |
| HSV1-UL30-6905 | - | GCGCAUCACCGACCCGGAG | 19 | 15845 |
| HSV1-UL30-2006 | - | GGCGCAUCACCGACCCGGAG | 20 | 5039 |
| HSV1-UL30-6906 | - | CGGCGCAUCACCGACCCGGAG | 21 | 15846 |
| HSV1-UL30-6907 | - | UCGGCGCAUCACCGACCCGGAG | 22 | 15847 |
| HSV1-UL30-6908 | - | AUCGGCGCAUCACCGACCCGGAG | 23 | 15848 |
| HSV1-UL30-6909 | - | CAUCGGCGCAUCACCGACCCGGAG | 24 | 15849 |
| HSV1-UL30-6910 | - | GGCGGGGGCGAGCGGGAG | 18 | 15850 |
| HSV1-UL30-6911 | - | GGGCGGGGGCGAGCGGGAG | 19 | 15851 |
| HSV1-UL30-1922 | - | AGGGCGGGGGCGAGCGGGAG | 20 | 4959 |
| HSV1-UL30-6912 | - | GAGGGCGGGGGCGAGCGGGAG | 21 | 15852 |
| HSV1-UL30-6913 | - | GGAGGGCGGGGGCGAGCGGGAG | 22 | 15853 |
| HSV1-UL30-6914 | - | AGGAGGGCGGGGGCGAGCGGGAG | 23 | 15854 |
| HSV1-UL30-6915 | - | GAGGAGGGCGGGGGCGAGCGGGAG | 24 | 15855 |
| HSV1-UL30-6916 | - | GCAGUGGCGCACCUGGAG | 18 | 15856 |
| HSV1-UL30-6917 | - | CGCAGUGGCGCACCUGGAG | 19 | 15857 |
| HSV1-UL30-1940 | - | ACGCAGUGGCGCACCUGGAG | 20 | 4973 |
| HSV1-UL30-6918 | - | GACGCAGUGGCGCACCUGGAG | 21 | 15858 |
| HSV1-UL30-6919 | - | CGACGCAGUGGCGCACCUGGAG | 22 | 15859 |
| HSV1-UL30-6920 | - | CCGACGCAGUGGCGCACCUGGAG | 23 | 15860 |
| HSV1-UL30-6921 | - | GCCGACGCAGUGGCGCACCUGGAG | 24 | 15861 |
| HSV1-UL30-6922 | - | CCCAGACCCGCGAGGUAG | 18 | 15862 |
| HSV1-UL30-6923 | - | GCCCAGACCCGCGAGGUAG | 19 | 15863 |
| HSV1-UL30-2015 | - | GGCCCAGACCCGCGAGGUAG | 20 | 5048 |
| HSV1-UL30-6924 | - | UGGCCCAGACCCGCGAGGUAG | 21 | 15864 |
| HSV1-UL30-6925 | - | GUGGCCCAGACCCGCGAGGUAG | 22 | 15865 |
| HSV1-UL30-6926 | - | CGUGGCCCAGACCCGCGAGGUAG | 23 | 15866 |
| HSV1-UL30-6927 | - | UCGUGGCCCAGACCCGCGAGGUAG | 24 | 15867 |
| HSV1-UL30-6928 | - | GAGCGGCCGCCGCGUUAG | 18 | 15868 |
| HSV1-UL30-6929 | - | GGAGCGGCCGCCGCGUUAG | 19 | 15869 |
| HSV1-UL30-1992 | - | CGGAGCGGCCGCCGCGUUAG | 20 | 5025 |
| HSV1-UL30-6930 | - | CCGGAGCGGCCGCCGCGUUAG | 21 | 15870 |
| HSV1-UL30-6931 | - | UCCGGAGCGGCCGCCGCGUUAG | 22 | 15871 |
| HSV1-UL30-6932 | - | CUCCGGAGCGGCCGCCGCGUUAG | 23 | 15872 |
| HSV1-UL30-6933 | - | UCUCCGGAGCGGCCGCCGCGUUAG | 24 | 15873 |
| HSV1-UL30-6934 | - | CCGCAGCCCGGGAGGACG | 18 | 15874 |
| HSV1-UL30-6935 | - | GCCGCAGCCCGGGAGGACG | 19 | 15875 |
| HSV1-UL30-569 | - | GGCCGCAGCCCGGGAGGACG | 20 | 3507 |
| HSV1-UL30-6936 | - | CGGCCGCAGCCCGGGAGGACG | 21 | 15876 |
| HSV1-UL30-6937 | - | CCGGCCGCAGCCCGGGAGGACG | 22 | 15877 |
| HSV1-UL30-6938 | - | UCCGGCCGCAGCCCGGGAGGACG | 23 | 15878 |
| HSV1-UL30-6939 | - | GUCCGGCCGCAGCCCGGGAGGACG | 24 | 15879 |
| HSV1-UL30-6940 | - | GUGGGUGCCGGCGCUACG | 18 | 15880 |
| HSV1-UL30-6941 | - | GGUGGGUGCCGGCGCUACG | 19 | 15881 |
| HSV1-UL30-2047 | - | CGGUGGGUGCCGGCGCUACG | 20 | 5080 |
| HSV1-UL30-6942 | - | GCGGUGGGUGCCGGCGCUACG | 21 | 15882 |
| HSV1-UL30-6943 | - | GGCGGUGGGUGCCGGCGCUACG | 22 | 15883 |
| HSV1-UL30-6944 | - | GGGCGGUGGGUGCCGGCGCUACG | 23 | 15884 |
| HSV1-UL30-6945 | - | GGGGCGGUGGGUGCCGGCGCUACG | 24 | 15885 |
| HSV1-UL30-6946 | - | GUCGAGUUUAACUGUACG | 18 | 15886 |
| HSV1-UL30-6947 | - | CGUCGAGUUUAACUGUACG | 19 | 15887 |
| HSV1-UL30-1809 | - | ACGUCGAGUUUAACUGUACG | 20 | 4859 |
| HSV1-UL30-6948 | - | GACGUCGAGUUUAACUGUACG | 21 | 15888 |
| HSV1-UL30-6949 | - | CGACGUCGAGUUUAACUGUACG | 22 | 15889 |
| HSV1-UL30-6950 | - | GCGACGUCGAGUUUAACUGUACG | 23 | 15890 |
| HSV1-UL30-6951 | - | AGCGACGUCGAGUUUAACUGUACG | 24 | 15891 |
| HSV1-UL30-6952 | - | AUGGACGCCAUCACACCG | 18 | 15892 |
| HSV1-UL30-6953 | - | UAUGGACGCCAUCACACCG | 19 | 15893 |
| HSV1-UL30-1771 | - | UUAUGGACGCCAUCACACCG | 20 | 4821 |
| HSV1-UL30-6954 | - | UUUAUGGACGCCAUCACACCG | 21 | 15894 |
| HSV1-UL30-6955 | - | GUUUAUGGACGCCAUCACACCG | 22 | 15895 |
| HSV1-UL30-6956 | - | GGUUUAUGGACGCCAUCACACCG | 23 | 15896 |
| HSV1-UL30-6957 | - | CGGUUUAUGGACGCCAUCACACCG | 24 | 15897 |
| HSV1-UL30-6958 | - | AGGACUUUGUCCUCACCG | 18 | 15898 |
| HSV1-UL30-6959 | - | CAGGACUUUGUCCUCACCG | 19 | 15899 |
| HSV1-UL30-2008 | - | CCAGGACUUUGUCCUCACCG | 20 | 5041 |
| HSV1-UL30-6960 | - | UCCAGGACUUUGUCCUCACCG | 21 | 15900 |
| HSV1-UL30-6961 | - | AUCCAGGACUUUGUCCUCACCG | 22 | 15901 |
| HSV1-UL30-6962 | - | CAUCCAGGACUUUGUCCUCACCG | 23 | 15902 |
| HSV1-UL30-6963 | - | ACAUCCAGGACUUUGUCCUCACCG | 24 | 15903 |
| HSV1-UL30-6964 | - | CCGGUGGCCGGGCACCCG | 18 | 15904 |
| HSV1-UL30-6965 | - | UCCGGUGGCCGGGCACCCG | 19 | 15905 |
| HSV1-UL30-1830 | - | UUCCGGUGGCCGGGCACCCG | 20 | 4880 |
| HSV1-UL30-6966 | - | UUUCCGGUGGCCGGGCACCCG | 21 | 15906 |
| HSV1-UL30-6967 | - | CUUUCCGGUGGCCGGGCACCCG | 22 | 15907 |
| HSV1-UL30-6968 | - | CCUUUCCGGUGGCCGGGCACCCG | 23 | 15908 |
| HSV1-UL30-6969 | - | GCCUUUCCGGUGGCCGGGCACCCG | 24 | 15909 |
| HSV1-UL30-6970 | - | CGGCGCAUCACCGACCCG | 18 | 15910 |
| HSV1-UL30-6971 | - | UCGGCGCAUCACCGACCCG | 19 | 15911 |
| HSV1-UL30-2004 | - | AUCGGCGCAUCACCGACCCG | 20 | 5037 |
| HSV1-UL30-6972 | - | CAUCGGCGCAUCACCGACCCG | 21 | 15912 |
| HSV1-UL30-6973 | - | CCAUCGGCGCAUCACCGACCCG | 22 | 15913 |
| HSV1-UL30-6974 | - | CCCAUCGGCGCAUCACCGACCCG | 23 | 15914 |
| HSV1-UL30-6975 | - | GCCCAUCGGCGCAUCACCGACCCG | 24 | 15915 |
| HSV1-UL30-6976 | - | UUCCCCAGAGCAGCCCCG | 18 | 15916 |
| HSV1-UL30-6977 | - | AUUCCCCAGAGCAGCCCCG | 19 | 15917 |
| HSV1-UL30-1960 | - | GAUUCCCCAGAGCAGCCCCG | 20 | 4993 |
| HSV1-UL30-6978 | - | GGAUUCCCCAGAGCAGCCCCG | 21 | 15918 |
| HSV1-UL30-6979 | - | CGGAUUCCCCAGAGCAGCCCCG | 22 | 15919 |
| HSV1-UL30-6980 | - | GCGGAUUCCCCAGAGCAGCCCCG | 23 | 15920 |
| HSV1-UL30-6981 | - | CGCGGAUUCCCCAGAGCAGCCCCG | 24 | 15921 |
| HSV1-UL30-6982 | - | CGUUAGCCGAGCGCCCCG | 18 | 15922 |
| HSV1-UL30-6983 | - | GCGUUAGCCGAGCGCCCCG | 19 | 15923 |
| HSV1-UL30-1994 | - | CGCGUUAGCCGAGCGCCCCG | 20 | 5027 |
| HSV1-UL30-6984 | - | CCGCGUUAGCCGAGCGCCCCG | 21 | 15924 |
| HSV1-UL30-6985 | - | GCCGCGUUAGCCGAGCGCCCCG | 22 | 15925 |
| HSV1-UL30-6986 | - | CGCCGCGUUAGCCGAGCGCCCCG | 23 | 15926 |
| HSV1-UL30-6987 | - | CCGCCGCGUUAGCCGAGCGCCCCG | 24 | 15927 |
| HSV1-UL30-6988 | - | UGGCGCGACCCCUGCCCG | 18 | 15928 |
| HSV1-UL30-6989 | - | CUGGCGCGACCCCUGCCCG | 19 | 15929 |
| HSV1-UL30-1999 | - | GCUGGCGCGACCCCUGCCCG | 20 | 5032 |
| HSV1-UL30-6990 | - | GGCUGGCGCGACCCCUGCCCG | 21 | 15930 |
| HSV1-UL30-6991 | - | UGGCUGGCGCGACCCCUGCCCG | 22 | 15931 |
| HSV1-UL30-6992 | - | GUGGCUGGCGCGACCCCUGCCCG | 23 | 15932 |
| HSV1-UL30-6993 | - | AGUGGCUGGCGCGACCCCUGCCCG | 24 | 15933 |
| HSV1-UL30-6994 | - | GGGGGCGAGCGGGAGCCG | 18 | 15934 |
| HSV1-UL30-6995 | - | CGGGGGCGAGCGGGAGCCG | 19 | 15935 |
| HSV1-UL30-1924 | - | GCGGGGGCGAGCGGGAGCCG | 20 | 4960 |
| HSV1-UL30-6996 | - | GGCGGGGGCGAGCGGGAGCCG | 21 | 15936 |
| HSV1-UL30-6997 | - | GGGCGGGGGCGAGCGGGAGCCG | 22 | 15937 |
| HSV1-UL30-6998 | - | AGGGCGGGGGCGAGCGGGAGCCG | 23 | 15938 |
| HSV1-UL30-6999 | - | GAGGGCGGGGGCGAGCGGGAGCCG | 24 | 15939 |
| HSV1-UL30-7000 | - | UCCCCGCCUACUACGCCG | 18 | 15940 |
| HSV1-UL30-7001 | - | AUCCCCGCCUACUACGCCG | 19 | 15941 |
| HSV1-UL30-1867 | - | CAUCCCCGCCUACUACGCCG | 20 | 4917 |
| HSV1-UL30-7002 | - | ACAUCCCCGCCUACUACGCCG | 21 | 15942 |
| HSV1-UL30-7003 | - | GACAUCCCCGCCUACUACGCCG | 22 | 15943 |
| HSV1-UL30-7004 | - | CGACAUCCCCGCCUACUACGCCG | 23 | 15944 |
| HSV1-UL30-7005 | - | GCGACAUCCCCGCCUACUACGCCG | 24 | 15945 |
| HSV1-UL30-7006 | - | GGCGAUUUAGGGGCGCCG | 18 | 15946 |
| HSV1-UL30-7007 | - | GGGCGAUUUAGGGGCGCCG | 19 | 15947 |
| HSV1-UL30-1886 | - | GGGGCGAUUUAGGGGCGCCG | 20 | 4935 |
| HSV1-UL30-7008 | - | AGGGGCGAUUUAGGGGCGCCG | 21 | 15948 |
| HSV1-UL30-7009 | - | CAGGGGCGAUUUAGGGGCGCCG | 22 | 15949 |
| HSV1-UL30-7010 | - | CCAGGGGCGAUUUAGGGGCGCCG | 23 | 15950 |
| HSV1-UL30-7011 | - | CCCAGGGGCGAUUUAGGGGCGCCG | 24 | 15951 |
| HSV1-UL30-7012 | - | GCCGCGGCCUCACGGCCG | 18 | 15952 |
| HSV1-UL30-7013 | - | UGCCGCGGCCUCACGGCCG | 19 | 15953 |
| HSV1-UL30-1978 | - | GUGCCGCGGCCUCACGGCCG | 20 | 5011 |
| HSV1-UL30-7014 | - | UGUGCCGCGGCCUCACGGCCG | 21 | 15954 |
| HSV1-UL30-7015 | - | CUGUGCCGCGGCCUCACGGCCG | 22 | 15955 |
| HSV1-UL30-7016 | - | GCUGUGCCGCGGCCUCACGGCCG | 23 | 15956 |
| HSV1-UL30-7017 | - | UGCUGUGCCGCGGCCUCACGGCCG | 24 | 15957 |
| HSV1-UL30-7018 | - | AGGACGAGGACGAACGCG | 18 | 15958 |
| HSV1-UL30-7019 | - | GAGGACGAGGACGAACGCG | 19 | 15959 |
| HSV1-UL30-579 | - | GGAGGACGAGGACGAACGCG | 20 | 3517 |
| HSV1-UL30-7020 | - | GGGAGGACGAGGACGAACGCG | 21 | 15960 |
| HSV1-UL30-7021 | - | GGGGAGGACGAGGACGAACGCG | 22 | 15961 |
| HSV1-UL30-7022 | - | GGGGGAGGACGAGGACGAACGCG | 23 | 15962 |
| HSV1-UL30-7023 | - | AGGGGGAGGACGAGGACGAACGCG | 24 | 15963 |
| HSV1-UL30-7024 | - | UUAGCCGAGCGCCCCGCG | 18 | 15964 |
| HSV1-UL30-7025 | - | GUUAGCCGAGCGCCCCGCG | 19 | 15965 |
| HSV1-UL30-1995 | - | CGUUAGCCGAGCGCCCCGCG | 20 | 5028 |
| HSV1-UL30-7026 | - | GCGUUAGCCGAGCGCCCCGCG | 21 | 15966 |
| HSV1-UL30-7027 | - | CGCGUUAGCCGAGCGCCCCGCG | 22 | 15967 |
| HSV1-UL30-7028 | - | CCGCGUUAGCCGAGCGCCCCGCG | 23 | 15968 |
| HSV1-UL3 0-7029 | - | GCCGCGUUAGCCGAGCGCCCCGCG | 24 | 15969 |
| HSV1-UL30-7030 | - | CGAGUACGUCCACGCGCG | 18 | 15970 |
| HSV1-UL30-7031 | - | GCGAGUACGUCCACGCGCG | 19 | 15971 |
| HSV1-UL30-1969 | - | CGCGAGUACGUCCACGCGCG | 20 | 5002 |
| HSV1-UL30-7032 | - | CCGCGAGUACGUCCACGCGCG | 21 | 15972 |
| HSV1-UL30-7033 | - | CCCGCGAGUACGUCCACGCGCG | 22 | 15973 |
| HSV1-UL30-7034 | - | ACCCGCGAGUACGUCCACGCGCG | 23 | 15974 |
| HSV1-UL30-7035 | - | GACCCGCGAGUACGUCCACGCGCG | 24 | 15975 |
| HSV1-UL30-7036 | - | CGGGAGCCGGAGGGCGCG | 18 | 15976 |
| HSV1-UL30-7037 | - | GCGGGAGCCGGAGGGCGCG | 19 | 15977 |
| HSV1-UL30-591 | - | AGCGGGAGCCGGAGGGCGCG | 20 | 3529 |
| HSV1-UL30-7038 | - | GAGCGGGAGCCGGAGGGCGCG | 21 | 15978 |
| HSV1-UL30-7039 | - | CGAGCGGGAGCCGGAGGGCGCG | 22 | 15979 |
| HSV1-UL30-7040 | - | GCGAGCGGGAGCCGGAGGGCGCG | 23 | 15980 |
| HSV1-UL30-7041 | - | GGCGAGCGGGAGCCGGAGGGCGCG | 24 | 15981 |
| HSV1-UL30-7042 | - | GAUAUCGAAUGCAAGGCG | 18 | 15982 |
| HSV1-UL30-7043 | - | CGAUAUCGAAUGCAAGGCG | 19 | 15983 |
| HSV1-UL30-1820 | - | UCGAUAUCGAAUGCAAGGCG | 20 | 4870 |
| HSV1-UL30-7044 | - | UUCGAUAUCGAAUGCAAGGCG | 21 | 15984 |
| HSV1-UL30-7045 | - | CUUCGAUAUCGAAUGCAAGGCG | 22 | 15985 |
| HSV1-UL30-7046 | - | GCUUCGAUAUCGAAUGCAAGGCG | 23 | 15986 |
| HSV1-UL30-7047 | - | UGCUUCGAUAUCGAAUGCAAGGCG | 24 | 15987 |
| HSV1-UL30-7048 | - | GGUGCCGGCGCUACGGCG | 18 | 15988 |
| HSV1-UL30-7049 | - | GGGUGCCGGCGCUACGGCG | 19 | 15989 |
| HSV1-UL30-2049 | - | UGGGUGCCGGCGCUACGGCG | 20 | 5082 |
| HSV1-UL30-7050 | - | GUGGGUGCCGGCGCUACGGCG | 21 | 15990 |
| HSV1-UL30-7051 | - | GGUGGGUGCCGGCGCUACGGCG | 22 | 15991 |
| HSV1-UL30-7052 | - | CGGUGGGUGCCGGCGCUACGGCG | 23 | 15992 |
| HSV1-UL30-7053 | - | GCGGUGGGUGCCGGCGCUACGGCG | 24 | 15993 |
| HSV1-UL30-7054 | - | AGGGGCGAUUUAGGGGCG | 18 | 15994 |
| HSV1-UL30-7055 | - | CAGGGGCGAUUUAGGGGCG | 19 | 15995 |
| HSV1-UL30-1883 | - | CCAGGGGCGAUUUAGGGGCG | 20 | 4932 |
| HSV1-UL30-7056 | - | CCCAGGGGCGAUUUAGGGGCG | 21 | 15996 |
| HSV1-UL30-7057 | - | ACCCAGGGGCGAUUUAGGGGCG | 22 | 15997 |
| HSV1-UL30-7058 | - | CACCCAGGGGCGAUUUAGGGGCG | 23 | 15998 |
| HSV1-UL30-7059 | - | ACACCCAGGGGCGAUUUAGGGGCG | 24 | 15999 |
| HSV1-UL30-7060 | - | CGGACAACCUGGCCAUCG | 18 | 16000 |
| HSV1-UL30-7061 | - | GCGGACAACCUGGCCAUCG | 19 | 16001 |
| HSV1-UL30-1812 | - | GGCGGACAACCUGGCCAUCG | 20 | 4862 |
| HSV1-UL30-7062 | - | CGGCGGACAACCUGGCCAUCG | 21 | 16002 |
| HSV1-UL30-7063 | - | ACGGCGGACAACCUGGCCAUCG | 22 | 16003 |
| HSV1-UL30-7064 | - | UACGGCGGACAACCUGGCCAUCG | 23 | 16004 |
| HSV1-UL30-7065 | - | GUACGGCGGACAACCUGGCCAUCG | 24 | 16005 |
| HSV1-UL30-7066 | - | ACUUUUACAUGAACAAGG | 18 | 16006 |
| HSV1-UL30-7067 | - | UACUUUUACAUGAACAAGG | 19 | 16007 |
| HSV1-UL30-1782 | - | GUACUUUUACAUGAACAAGG | 20 | 4832 |
| HSV1-UL30-7068 | - | AGUACUUUUACAUGAACAAGG | 21 | 16008 |
| HSV1-UL30-7069 | - | CAGUACUUUUACAUGAACAAGG | 22 | 16009 |
| HSV1-UL30-7070 | - | GCAGUACUUUUACAUGAACAAGG | 23 | 16010 |
| HSV1-UL30-7071 | - | GGCAGUACUUUUACAUGAACAAGG | 24 | 16011 |
| HSV1-UL30-7072 | - | UCGAUAUCGAAUGCAAGG | 18 | 16012 |
| HSV1-UL30-7073 | - | UUCGAUAUCGAAUGCAAGG | 19 | 16013 |
| HSV1-UL30-1818 | - | CUUCGAUAUCGAAUGCAAGG | 20 | 4868 |
| HSV1-UL30-7074 | - | GCUUCGAUAUCGAAUGCAAGG | 21 | 16014 |
| HSV1-UL30-7075 | - | UGCUUCGAUAUCGAAUGCAAGG | 22 | 16015 |
| HSV1-UL30-7076 | - | GUGCUUCGAUAUCGAAUGCAAGG | 23 | 16016 |
| HSV1-UL30-7077 | - | UGUGCUUCGAUAUCGAAUGCAAGG | 24 | 16017 |
| HSV1-UL30-7078 | - | ACGAGGAGCGGCCAGAGG | 18 | 16018 |
| HSV1-UL30-7079 | - | GACGAGGAGCGGCCAGAGG | 19 | 16019 |
| HSV1-UL30-572 | - | GGACGAGGAGCGGCCAGAGG | 20 | 3510 |
| HSV1-UL30-7080 | - | AGGACGAGGAGCGGCCAGAGG | 21 | 16020 |
| HSV1-UL30-7081 | - | GAGGACGAGGAGCGGCCAGAGG | 22 | 16021 |
| HSV1-UL30-7082 | - | GGAGGACGAGGAGCGGCCAGAGG | 23 | 16022 |
| HSV1-UL30-7083 | - | GGGAGGACGAGGAGCGGCCAGAGG | 24 | 16023 |
| HSV1-UL30-7084 | - | AGGAGGGGGAGGACGAGG | 18 | 16024 |
| HSV1-UL30-7085 | - | GAGGAGGGGGAGGACGAGG | 19 | 16025 |
| HSV1-UL30-1910 | - | GGAGGAGGGGGAGGACGAGG | 20 | 4952 |
| HSV1-UL30-7086 | - | AGGAGGAGGGGGAGGACGAGG | 21 | 16026 |
| HSV1-UL30-7087 | - | GAGGAGGAGGGGGAGGACGAGG | 22 | 16027 |
| HSV1-UL30-7088 | - | AGAGGAGGAGGGGGAGGACGAGG | 23 | 16028 |
| HSV1-UL30-7089 | - | CAGAGGAGGAGGGGGAGGACGAGG | 24 | 16029 |
| HSV1-UL30-7090 | - | CCAUCAAGAAGUACGAGG | 18 | 16030 |
| HSV1-UL30-7091 | - | GCCAUCAAGAAGUACGAGG | 19 | 16031 |
| HSV1-UL30-1799 | - | GGCCAUCAAGAAGUACGAGG | 20 | 4849 |
| HSV1-UL30-7092 | - | CGGCCAUCAAGAAGUACGAGG | 21 | 16032 |
| HSV1-UL30-7093 | - | CCGGCCAUCAAGAAGUACGAGG | 22 | 16033 |
| HSV1-UL30-7094 | - | CCCGGCCAUCAAGAAGUACGAGG | 23 | 16034 |
| HSV1-UL30-7095 | - | GCCCGGCCAUCAAGAAGUACGAGG | 24 | 16035 |
| HSV1-UL30-7096 | - | UGGCCCAGACCCGCGAGG | 18 | 16036 |
| HSV1-UL30-7097 | - | GUGGCCCAGACCCGCGAGG | 19 | 16037 |
| HSV1-UL30-2013 | - | CGUGGCCCAGACCCGCGAGG | 20 | 5046 |
| HSV1-UL30-7098 | - | UCGUGGCCCAGACCCGCGAGG | 21 | 16038 |
| HSV1-UL30-7099 | - | AUCGUGGCCCAGACCCGCGAGG | 22 | 16039 |
| HSV1-UL30-7100 | - | GAUCGUGGCCCAGACCCGCGAGG | 23 | 16040 |
| HSV1-UL30-7101 | - | UGAUCGUGGCCCAGACCCGCGAGG | 24 | 16041 |
| HSV1-UL30-7102 | - | ACUACCUGGAGAUCGAGG | 18 | 16042 |
| HSV1-UL30-7103 | - | GACUACCUGGAGAUCGAGG | 19 | 16043 |
| HSV1-UL30-1946 | - | GGACUACCUGGAGAUCGAGG | 20 | 4979 |
| HSV1-UL30-7104 | - | AGGACUACCUGGAGAUCGAGG | 21 | 16044 |
| HSV1-UL30-7105 | - | AAGGACUACCUGGAGAUCGAGG | 22 | 16045 |
| HSV1-UL30-7106 | - | CAAGGACUACCUGGAGAUCGAGG | 23 | 16046 |
| HSV1-UL30-7107 | - | GCAAGGACUACCUGGAGAUCGAGG | 24 | 16047 |
| HSV1-UL30-7108 | - | CCGCGGACCACUUCGAGG | 18 | 16048 |
| HSV1-UL30-7109 | - | UCCGCGGACCACUUCGAGG | 19 | 16049 |
| HSV1-UL30-1790 | - | CUCCGCGGACCACUUCGAGG | 20 | 4840 |
| HSV1-UL30-7110 | - | UCUCCGCGGACCACUUCGAGG | 21 | 16050 |
| HSV1-UL30-7111 | - | AUCUCCGCGGACCACUUCGAGG | 22 | 16051 |
| HSV1-UL30-7112 | - | CAUCUCCGCGGACCACUUCGAGG | 23 | 16052 |
| HSV1-UL30-7113 | - | GCAUCUCCGCGGACCACUUCGAGG | 24 | 16053 |
| HSV1-UL30-7114 | - | AGGAGCGGCCAGAGGAGG | 18 | 16054 |
| HSV1-UL30-7115 | - | GAGGAGCGGCCAGAGGAGG | 19 | 16055 |
| HSV1-UL30-573 | - | CGAGGAGCGGCCAGAGGAGG | 20 | 3511 |
| HSV1-UL30-7116 | - | ACGAGGAGCGGCCAGAGGAGG | 21 | 16056 |
| HSV1-UL30-7117 | - | GACGAGGAGCGGCCAGAGGAGG | 22 | 16057 |
| HSV1-UL30-7118 | - | GGACGAGGAGCGGCCAGAGGAGG | 23 | 16058 |
| HSV1-UL30-7119 | - | AGGACGAGGAGCGGCCAGAGGAGG | 24 | 16059 |
| HSV1-UL30-7120 | - | AGGACGAACGCGAGGAGG | 18 | 16060 |
| HSV1-UL30-7121 | - | GAGGACGAACGCGAGGAGG | 19 | 16061 |
| HSV1-UL30-1915 | - | CGAGGACGAACGCGAGGAGG | 20 | 4956 |
| HSV1-UL30-7122 | - | ACGAGGACGAACGCGAGGAGG | 21 | 16062 |
| HSV1-UL30-7123 | - | GACGAGGACGAACGCGAGGAGG | 22 | 16063 |
| HSV1-UL30-7124 | - | GGACGAGGACGAACGCGAGGAGG | 23 | 16064 |
| HSV1-UL30-7125 | - | AGGACGAGGACGAACGCGAGGAGG | 24 | 16065 |
| HSV1-UL30-7126 | - | AGCGGCCAGAGGAGGAGG | 18 | 16066 |
| HSV1-UL30-7127 | - | GAGCGGCCAGAGGAGGAGG | 19 | 16067 |
| HSV1-UL30-576 | - | GGAGCGGCCAGAGGAGGAGG | 20 | 3514 |
| HSV1-UL30-7128 | - | AGGAGCGGCCAGAGGAGGAGG | 21 | 16068 |
| HSV1-UL30-7129 | - | GAGGAGCGGCCAGAGGAGGAGG | 22 | 16069 |
| HSV1-UL30-7130 | - | CGAGGAGCGGCCAGAGGAGGAGG | 23 | 16070 |
| HSV1-UL30-7131 | - | ACGAGGAGCGGCCAGAGGAGGAGG | 24 | 16071 |
| HSV1-UL30-7132 | - | CGGCCGCAGCCCGGGAGG | 18 | 16072 |
| HSV1-UL30-7133 | - | CCGGCCGCAGCCCGGGAGG | 19 | 16073 |
| HSV1-UL30-1894 | - | UCCGGCCGCAGCCCGGGAGG | 20 | 4943 |
| HSV1-UL30-7134 | - | GUCCGGCCGCAGCCCGGGAGG | 21 | 16074 |
| HSV1-UL30-7135 | - | CGUCCGGCCGCAGCCCGGGAGG | 22 | 16075 |
| HSV1-UL30-7136 | - | GCGUCCGGCCGCAGCCCGGGAGG | 23 | 16076 |
| HSV1-UL30-7137 | - | AGCGUCCGGCCGCAGCCCGGGAGG | 24 | 16077 |
| HSV1-UL30-7138 | - | CAGAGGAGGAGGGGGAGG | 18 | 16078 |
| HSV1-UL30-7139 | - | CCAGAGGAGGAGGGGGAGG | 19 | 16079 |
| HSV1-UL30-1908 | - | GCCAGAGGAGGAGGGGGAGG | 20 | 4950 |
| HSV1-UL30-7140 | - | GGCCAGAGGAGGAGGGGGAGG | 21 | 16080 |
| HSV1-UL30-7141 | - | CGGCCAGAGGAGGAGGGGGAGG | 22 | 16081 |
| HSV1-UL30-7142 | - | GCGGCCAGAGGAGGAGGGGGAGG | 23 | 16082 |
| HSV1-UL30-7143 | - | AGCGGCCAGAGGAGGAGGGGGAGG | 24 | 16083 |
| HSV1-UL30-7144 | - | GCAAGGCGGGGGGGGAGG | 18 | 16084 |
| HSV1-UL30-7145 | - | UGCAAGGCGGGGGGGGAGG | 19 | 16085 |
| HSV1-UL30-1826 | - | AUGCAAGGCGGGGGGGGAGG | 20 | 4876 |
| HSV1-UL30-7146 | - | AAUGCAAGGCGGGGGGGGAGG | 21 | 16086 |
| HSV1-UL30-7147 | - | GAAUGCAAGGCGGGGGGGGAGG | 22 | 16087 |
| HSV1-UL30-7148 | - | CGAAUGCAAGGCGGGGGGGGAGG | 23 | 16088 |
| HSV1-UL30-7149 | - | UCGAAUGCAAGGCGGGGGGGGAGG | 24 | 16089 |
| HSV1-UL30-7150 | - | GUACGGCCGCAUGAACGG | 18 | 16090 |
| HSV1-UL30-7151 | - | GGUACGGCCGCAUGAACGG | 19 | 16091 |
| HSV1-UL30-1849 | - | GGGUACGGCCGCAUGAACGG | 20 | 4899 |
| HSV1-UL30-7152 | - | CGGGUACGGCCGCAUGAACGG | 21 | 16092 |
| HSV1-UL30-7153 | - | ACGGGUACGGCCGCAUGAACGG | 22 | 16093 |
| HSV1-UL30-7154 | - | GACGGGUACGGCCGCAUGAACGG | 23 | 16094 |
| HSV1-UL30-7155 | - | GGACGGGUACGGCCGCAUGAACGG | 24 | 16095 |
| HSV1-UL30-7156 | - | UGGGUGCCGGCGCUACGG | 18 | 16096 |
| HSV1-UL30-7157 | - | GUGGGUGCCGGCGCUACGG | 19 | 16097 |
| HSV1-UL30-2048 | - | GGUGGGUGCCGGCGCUACGG | 20 | 5081 |
| HSV1-UL30-7158 | - | CGGUGGGUGCCGGCGCUACGG | 21 | 16098 |
| HSV1-UL30-7159 | - | GCGGUGGGUGCCGGCGCUACGG | 22 | 16099 |
| HSV1-UL30-7160 | - | GGCGGUGGGUGCCGGCGCUACGG | 23 | 16100 |
| HSV1-UL30-7161 | - | GGGCGGUGGGUGCCGGCGCUACGG | 24 | 16101 |
| HSV1-UL30-7162 | - | CGUCCGGCCGCAGCCCGG | 18 | 16102 |
| HSV1-UL30-7163 | - | GCGUCCGGCCGCAGCCCGG | 19 | 16103 |
| HSV1-UL30-1893 | - | AGCGUCCGGCCGCAGCCCGG | 20 | 4942 |
| HSV1-UL30-7164 | - | AAGCGUCCGGCCGCAGCCCGG | 21 | 16104 |
| HSV1-UL30-7165 | - | CAAGCGUCCGGCCGCAGCCCGG | 22 | 16105 |
| HSV1-UL30-7166 | - | CCAAGCGUCCGGCCGCAGCCCGG | 23 | 16106 |
| HSV1-UL30-7167 | - | CCCAAGCGUCCGGCCGCAGCCCGG | 24 | 16107 |
| HSV1-UL30-7168 | - | GCGAUUUAGGGGCGCCGG | 18 | 16108 |
| HSV1-UL30-7169 | - | GGCGAUUUAGGGGCGCCGG | 19 | 16109 |
| HSV1-UL30-1887 | - | GGGCGAUUUAGGGGCGCCGG | 20 | 4936 |
| HSV1-UL30-7170 | - | GGGGCGAUUUAGGGGCGCCGG | 21 | 16110 |
| HSV1-UL30-7171 | - | AGGGGCGAUUUAGGGGCGCCGG | 22 | 16111 |
| HSV1-UL30-7172 | - | CAGGGGCGAUUUAGGGGCGCCGG | 23 | 16112 |
| HSV1-UL30-7173 | - | CCAGGGGCGAUUUAGGGGCGCCGG | 24 | 16113 |
| HSV1-UL30-7174 | - | AUAUCGAAUGCAAGGCGG | 18 | 16114 |
| HSV1-UL30-7175 | - | GAUAUCGAAUGCAAGGCGG | 19 | 16115 |
| HSV1-UL30-1821 | - | CGAUAUCGAAUGCAAGGCGG | 20 | 4871 |
| HSV1-UL30-7176 | - | UCGAUAUCGAAUGCAAGGCGG | 21 | 16116 |
| HSV1-UL30-7177 | - | UUCGAUAUCGAAUGCAAGGCGG | 22 | 16117 |
| HSV1-UL30-7178 | - | CUUCGAUAUCGAAUGCAAGGCGG | 23 | 16118 |
| HSV1-UL30-7179 | - | GCUUCGAUAUCGAAUGCAAGGCGG | 24 | 16119 |
| HSV1-UL30-7180 | - | GUGCCGGCGCUACGGCGG | 18 | 16120 |
| HSV1-UL30-7181 | - | GGUGCCGGCGCUACGGCGG | 19 | 16121 |
| HSV1-UL30-2050 | - | GGGUGCCGGCGCUACGGCGG | 20 | 5083 |
| HSV1-UL30-7182 | - | UGGGUGCCGGCGCUACGGCGG | 21 | 16122 |
| HSV1-UL30-7183 | - | GUGGGUGCCGGCGCUACGGCGG | 22 | 16123 |
| HSV1-UL30-7184 | - | GGUGGGUGCCGGCGCUACGGCGG | 23 | 16124 |
| HSV1-UL30-7185 | - | CGGUGGGUGCCGGCGCUACGGCGG | 24 | 16125 |
| HSV1-UL30-7186 | - | GGACGAACGCGAGGAGGG | 18 | 16126 |
| HSV1-UL30-7187 | - | AGGACGAACGCGAGGAGGG | 19 | 16127 |
| HSV1-UL30-582 | - | GAGGACGAACGCGAGGAGGG | 20 | 3520 |
| HSV1-UL30-7188 | - | CGAGGACGAACGCGAGGAGGG | 21 | 16128 |
| HSV1-UL30-7189 | - | ACGAGGACGAACGCGAGGAGGG | 22 | 16129 |
| HSV1-UL30-7190 | - | GACGAGGACGAACGCGAGGAGGG | 23 | 16130 |
| HSV1-UL30-7191 | - | GGACGAGGACGAACGCGAGGAGGG | 24 | 16131 |
| HSV1-UL30-7192 | - | GUACCGUCUCAAACCGGG | 18 | 16132 |
| HSV1-UL30-7193 | - | GGUACCGUCUCAAACCGGG | 19 | 16133 |
| HSV1-UL30-1804 | - | UGGUACCGUCUCAAACCGGG | 20 | 4854 |
| HSV1-UL30-7194 | - | CUGGUACCGUCUCAAACCGGG | 21 | 16134 |
| HSV1-UL30-7195 | - | GCUGGUACCGUCUCAAACCGGG | 22 | 16135 |
| HSV1-UL30-7196 | - | GGCUGGUACCGUCUCAAACCGGG | 23 | 16136 |
| HSV1-UL30-7197 | - | CGGCUGGUACCGUCUCAAACCGGG | 24 | 16137 |
| HSV1-UL30-7198 | - | CGAUUUAGGGGCGCCGGG | 18 | 16138 |
| HSV1-UL30-7199 | - | GCGAUUUAGGGGCGCCGGG | 19 | 16139 |
| HSV1-UL30-1888 | - | GGCGAUUUAGGGGCGCCGGG | 20 | 4937 |
| HSV1-UL30-7200 | - | GGGCGAUUUAGGGGCGCCGGG | 21 | 16140 |
| HSV1-UL30-7201 | - | GGGGCGAUUUAGGGGCGCCGGG | 22 | 16141 |
| HSV1-UL30-7202 | - | AGGGGCGAUUUAGGGGCGCCGGG | 23 | 16142 |
| HSV1-UL30-7203 | - | CAGGGGCGAUUUAGGGGCGCCGGG | 24 | 16143 |
| HSV1-UL30-7204 | - | UAUCGAAUGCAAGGCGGG | 18 | 16144 |
| HSV1-UL30-7205 | - | AUAUCGAAUGCAAGGCGGG | 19 | 16145 |
| HSV1-UL30-1822 | - | GAUAUCGAAUGCAAGGCGGG | 20 | 4872 |
| HSV1-UL30-7206 | - | CGAUAUCGAAUGCAAGGCGGG | 21 | 16146 |
| HSV1-UL30-7207 | - | UCGAUAUCGAAUGCAAGGCGGG | 22 | 16147 |
| HSV1-UL30-7208 | - | UUCGAUAUCGAAUGCAAGGCGGG | 23 | 16148 |
| HSV1-UL30-7209 | - | CUUCGAUAUCGAAUGCAAGGCGGG | 24 | 16149 |
| HSV1-UL30-7210 | - | ACGCCGCCGCCCCAGGGG | 18 | 16150 |
| HSV1-UL30-7211 | - | GACGCCGCCGCCCCAGGGG | 19 | 16151 |
| HSV1-UL30-2020 | - | AGACGCCGCCGCCCCAGGGG | 20 | 5053 |
| HSV1-UL30-7212 | - | UAGACGCCGCCGCCCCAGGGG | 21 | 16152 |
| HSV1-UL30-7213 | - | CUAGACGCCGCCGCCCCAGGGG | 22 | 16153 |
| HSV1-UL30-7214 | - | GCUAGACGCCGCCGCCCCAGGGG | 23 | 16154 |
| HSV1-UL30-7215 | - | AGCUAGACGCCGCCGCCCCAGGGG | 24 | 16155 |
| HSV1-UL30-7216 | - | CGGCCAGAGGAGGAGGGG | 18 | 16156 |
| HSV1-UL30-7217 | - | GCGGCCAGAGGAGGAGGGG | 19 | 16157 |
| HSV1-UL30-1907 | - | AGCGGCCAGAGGAGGAGGGG | 20 | 4949 |
| HSV1-UL30-7218 | - | GAGCGGCCAGAGGAGGAGGGG | 21 | 16158 |
| HSV1-UL30-7219 | - | GGAGCGGCCAGAGGAGGAGGGG | 22 | 16159 |
| HSV1-UL30-7220 | - | AGGAGCGGCCAGAGGAGGAGGGG | 23 | 16160 |
| HSV1-UL30-7221 | - | GAGGAGCGGCCAGAGGAGGAGGGG | 24 | 16161 |
| HSV1-UL30-7222 | - | GAUUUAGGGGCGCCGGGG | 18 | 16162 |
| HSV1-UL30-7223 | - | CGAUUUAGGGGCGCCGGGG | 19 | 16163 |
| HSV1-UL30-1889 | - | GCGAUUUAGGGGCGCCGGGG | 20 | 4938 |
| HSV1-UL30-7224 | - | GGCGAUUUAGGGGCGCCGGGG | 21 | 16164 |
| HSV1-UL30-7225 | - | GGGCGAUUUAGGGGCGCCGGGG | 22 | 16165 |
| HSV1-UL30-7226 | - | GGGGCGAUUUAGGGGCGCCGGGG | 23 | 16166 |
| HSV1-UL30-7227 | - | AGGGGCGAUUUAGGGGCGCCGGGG | 24 | 16167 |
| HSV1-UL30-7228 | - | AUCGAAUGCAAGGCGGGG | 18 | 16168 |
| HSV1-UL30-7229 | - | UAUCGAAUGCAAGGCGGGG | 19 | 16169 |
| HSV1-UL30-1823 | - | AUAUCGAAUGCAAGGCGGGG | 20 | 4873 |
| HSV1-UL30-7230 | - | GAUAUCGAAUGCAAGGCGGGG | 21 | 16170 |
| HSV1-UL30-7231 | - | CGAUAUCGAAUGCAAGGCGGGG | 22 | 16171 |
| HSV1-UL30-7232 | - | UCGAUAUCGAAUGCAAGGCGGGG | 23 | 16172 |
| HSV1-UL30-7233 | - | UUCGAUAUCGAAUGCAAGGCGGGG | 24 | 16173 |
| HSV1-UL30-7234 | - | AACGCGAGGAGGGCGGGG | 18 | 16174 |
| HSV1-UL30-7235 | - | GAACGCGAGGAGGGCGGGG | 19 | 16175 |
| HSV1-UL30-1918 | - | CGAACGCGAGGAGGGCGGGG | 20 | 4957 |
| HSV1-UL30-7236 | - | ACGAACGCGAGGAGGGCGGGG | 21 | 16176 |
| HSV1-UL30-7237 | - | GACGAACGCGAGGAGGGCGGGG | 22 | 16177 |
| HSV1-UL30-7238 | - | GGACGAACGCGAGGAGGGCGGGG | 23 | 16178 |
| HSV1-UL30-7239 | - | AGGACGAACGCGAGGAGGGCGGGG | 24 | 16179 |
| HSV1-UL30-7240 | - | UCGAAUGCAAGGCGGGGG | 18 | 16180 |
| HSV1-UL30-7241 | - | AUCGAAUGCAAGGCGGGGG | 19 | 16181 |
| HSV1-UL30-1824 | - | UAUCGAAUGCAAGGCGGGGG | 20 | 4874 |
| HSV1-UL30-7242 | - | AUAUCGAAUGCAAGGCGGGGG | 21 | 16182 |
| HSV1-UL30-7243 | - | GAUAUCGAAUGCAAGGCGGGGG | 22 | 16183 |
| HSV1-UL30-7244 | - | CGAUAUCGAAUGCAAGGCGGGGG | 23 | 16184 |
| HSV1-UL30-7245 | - | UCGAUAUCGAAUGCAAGGCGGGGG | 24 | 16185 |
| HSV1-UL30-7246 | - | GAAUGCAAGGCGGGGGGG | 18 | 16186 |
| HSV1-UL30-7247 | - | CGAAUGCAAGGCGGGGGGG | 19 | 16187 |
| HSV1-UL30-1825 | - | UCGAAUGCAAGGCGGGGGGG | 20 | 4875 |
| HSV1-UL30-7248 | - | AUCGAAUGCAAGGCGGGGGGG | 21 | 16188 |
| HSV1-UL30-7249 | - | UAUCGAAUGCAAGGCGGGGGGG | 22 | 16189 |
| HSV1-UL30-7250 | - | AUAUCGAAUGCAAGGCGGGGGGG | 23 | 16190 |
| HSV1-UL30-7251 | - | GAUAUCGAAUGCAAGGCGGGGGGG | 24 | 16191 |
| HSV1-UL30-7252 | - | UGCUGGUGUCCGAGCUGG | 18 | 16192 |
| HSV1-UL30-7253 | - | CUGCUGGUGUCCGAGCUGG | 19 | 16193 |
| HSV1-UL30-2028 | - | GCUGCUGGUGUCCGAGCUGG | 20 | 5061 |
| HSV1-UL30-7254 | - | AGCUGCUGGUGUCCGAGCUGG | 21 | 16194 |
| HSV1-UL30-7255 | - | AAGCUGCUGGUGUCCGAGCUGG | 22 | 16195 |
| HSV1-UL30-7256 | - | CAAGCUGCUGGUGUCCGAGCUGG | 23 | 16196 |
| HSV1-UL30-7257 | - | GCAAGCUGCUGGUGUCCGAGCUGG | 24 | 16197 |
| HSV1-UL30-7258 | - | ACAAGCUCAACGCCGUGG | 18 | 16198 |
| HSV1-UL30-7259 | - | UACAAGCUCAACGCCGUGG | 19 | 16199 |
| HSV1-UL30-1860 | - | CUACAAGCUCAACGCCGUGG | 20 | 4910 |
| HSV1-UL30-7260 | - | GCUACAAGCUCAACGCCGUGG | 21 | 16200 |
| HSV1-UL30-7261 | - | AGCUACAAGCUCAACGCCGUGG | 22 | 16201 |
| HSV1-UL30-7262 | - | GAGCUACAAGCUCAACGCCGUGG | 23 | 16202 |
| HSV1-UL30-7263 | - | CGAGCUACAAGCUCAACGCCGUGG | 24 | 16203 |
| HSV1-UL30-7264 | - | ACCUGGAGAUCGAGGUGG | 18 | 16204 |
| HSV1-UL30-7265 | - | UACCUGGAGAUCGAGGUGG | 19 | 16205 |
| HSV1-UL30-1949 | - | CUACCUGGAGAUCGAGGUGG | 20 | 4982 |
| HSV1-UL30-7266 | - | ACUACCUGGAGAUCGAGGUGG | 21 | 16206 |
| HSV1-UL30-7267 | - | GACUACCUGGAGAUCGAGGUGG | 22 | 16207 |
| HSV1-UL30-7268 | - | GGACUACCUGGAGAUCGAGGUGG | 23 | 16208 |
| HSV1-UL30-7269 | - | AGGACUACCUGGAGAUCGAGGUGG | 24 | 16209 |
| HSV1-UL30-7270 | - | ACUUCGAGGCGGAGGUGG | 18 | 16210 |
| HSV1-UL30-7271 | - | CACUUCGAGGCGGAGGUGG | 19 | 16211 |
| HSV1-UL30-1792 | - | CCACUUCGAGGCGGAGGUGG | 20 | 4842 |
| HSV1-UL30-7272 | - | ACCACUUCGAGGCGGAGGUGG | 21 | 16212 |
| HSV1-UL30-7273 | - | GACCACUUCGAGGCGGAGGUGG | 22 | 16213 |
| HSV1-UL30-7274 | - | GGACCACUUCGAGGCGGAGGUGG | 23 | 16214 |
| HSV1-UL30-7275 | - | CGGACCACUUCGAGGCGGAGGUGG | 24 | 16215 |
| HSV1-UL30-7276 | - | AGCUGGCCUUUCCGGUGG | 18 | 16216 |
| HSV1-UL30-7277 | - | GAGCUGGCCUUUCCGGUGG | 19 | 16217 |
| HSV1-UL30-1827 | - | CGAGCUGGCCUUUCCGGUGG | 20 | 4877 |
| HSV1-UL30-7278 | - | ACGAGCUGGCCUUUCCGGUGG | 21 | 16218 |
| HSV1-UL30-7279 | - | GACGAGCUGGCCUUUCCGGUGG | 22 | 16219 |
| HSV1-UL30-7280 | - | GGACGAGCUGGCCUUUCCGGUGG | 23 | 16220 |
| HSV1-UL30-7281 | - | AGGACGAGCUGGCCUUUCCGGUGG | 24 | 16221 |
| HSV1-UL30-7282 | - | AUAAAGGUGAACGGCAUG | 18 | 16222 |
| HSV1-UL30-7283 | - | GAUAAAGGUGAACGGCAUG | 19 | 16223 |
| HSV1-UL30-1856 | - | AGAUAAAGGUGAACGGCAUG | 20 | 4906 |
| HSV1-UL30-7284 | - | AAGAUAAAGGUGAACGGCAUG | 21 | 16224 |
| HSV1-UL30-7285 | - | CAAGAUAAAGGUGAACGGCAUG | 22 | 16225 |
| HSV1-UL30-7286 | - | GCAAGAUAAAGGUGAACGGCAUG | 23 | 16226 |
| HSV1-UL30-7287 | - | AGCAAGAUAAAGGUGAACGGCAUG | 24 | 16227 |
| HSV1-UL30-7288 | - | GCCGUGGGCGACAAGAUG | 18 | 16228 |
| HSV1-UL30-7289 | - | GGCCGUGGGCGACAAGAUG | 19 | 16229 |
| HSV1-UL30-1980 | - | CGGCCGUGGGCGACAAGAUG | 20 | 5013 |
| HSV1-UL30-7290 | - | ACGGCCGUGGGCGACAAGAUG | 21 | 16230 |
| HSV1-UL30-7291 | - | GACGGCCGUGGGCGACAAGAUG | 22 | 16231 |
| HSV1-UL30-7292 | - | UGACGGCCGUGGGCGACAAGAUG | 23 | 16232 |
| HSV1-UL30-7293 | - | CUGACGGCCGUGGGCGACAAGAUG | 24 | 16233 |
| HSV1-UL30-7294 | - | UAUUACUUCUCCCACCUG | 18 | 16234 |
| HSV1-UL30-7295 | - | CUAUUACUUCUCCCACCUG | 19 | 16235 |
| HSV1-UL30-2032 | - | ACUAUUACUUCUCCCACCUG | 20 | 5065 |
| HSV1-UL30-7296 | - | GACUAUUACUUCUCCCACCUG | 21 | 16236 |
| HSV1-UL30-7297 | - | GGACUAUUACUUCUCCCACCUG | 22 | 16237 |
| HSV1-UL30-7298 | - | CGGACUAUUACUUCUCCCACCUG | 23 | 16238 |
| HSV1-UL30-7299 | - | ACGGACUAUUACUUCUCCCACCUG | 24 | 16239 |
| HSV1-UL30-7300 | - | CUCCUCAGCAUCCUCCUG | 18 | 16240 |
| HSV1-UL30-7301 | - | CCUCCUCAGCAUCCUCCUG | 19 | 16241 |
| HSV1-UL30-1954 | - | GCCUCCUCAGCAUCCUCCUG | 20 | 4987 |
| HSV1-UL30-7302 | - | AGCCUCCUCAGCAUCCUCCUG | 21 | 16242 |
| HSV1-UL30-7303 | - | GAGCCUCCUCAGCAUCCUCCUG | 22 | 16243 |
| HSV1-UL30-7304 | - | AGAGCCUCCUCAGCAUCCUCCUG | 23 | 16244 |
| HSV1-UL30-7305 | - | GAGAGCCUCCUCAGCAUCCUCCUG | 24 | 16245 |
| HSV1-UL30-7306 | - | GUGGCCGAAGCCGUCCUG | 18 | 16246 |
| HSV1-UL30-7307 | - | CGUGGCCGAAGCCGUCCUG | 19 | 16247 |
| HSV1-UL30-1862 | - | CCGUGGCCGAAGCCGUCCUG | 20 | 4912 |
| HSV1-UL30-7308 | - | GCCGUGGCCGAAGCCGUCCUG | 21 | 16248 |
| HSV1-UL30-7309 | - | CGCCGUGGCCGAAGCCGUCCUG | 22 | 16249 |
| HSV1-UL30-7310 | - | ACGCCGUGGCCGAAGCCGUCCUG | 23 | 16250 |
| HSV1-UL30-7311 | - | AACGCCGUGGCCGAAGCCGUCCUG | 24 | 16251 |
| HSV1-UL30-7312 | - | UUCUUGCUGGCCAAGCUG | 18 | 16252 |
| HSV1-UL30-7313 | - | CUUCUUGCUGGCCAAGCUG | 19 | 16253 |
| HSV1-UL30-1845 | - | CCUUCUUGCUGGCCAAGCUG | 20 | 4895 |
| HSV1-UL30-7314 | - | CCCUUCUUGCUGGCCAAGCUG | 21 | 16254 |
| HSV1-UL30-7315 | - | GCCCUUCUUGCUGGCCAAGCUG | 22 | 16255 |
| HSV1-UL30-7316 | - | GGCCCUUCUUGCUGGCCAAGCUG | 23 | 16256 |
| HSV1-UL30-7317 | - | UGGCCCUUCUUGCUGGCCAAGCUG | 24 | 16257 |
| HSV1-UL30-7318 | - | AUACAGGAUUCCCUGCUG | 18 | 16258 |
| HSV1-UL30-7319 | - | CAUACAGGAUUCCCUGCUG | 19 | 16259 |
| HSV1-UL30-1872 | - | GCAUACAGGAUUCCCUGCUG | 20 | 4921 |
| HSV1-UL30-7320 | - | UGCAUACAGGAUUCCCUGCUG | 21 | 16260 |
| HSV1-UL30-7321 | - | CUGCAUACAGGAUUCCCUGCUG | 22 | 16261 |
| HSV1-UL30-7322 | - | ACUGCAUACAGGAUUCCCUGCUG | 23 | 16262 |
| HSV1-UL30-7323 | - | UACUGCAUACAGGAUUCCCUGCUG | 24 | 16263 |
| HSV1-UL30-7324 | - | CAGAAGGGCUUUAUUCUG | 18 | 16264 |
| HSV1-UL30-7325 | - | CCAGAAGGGCUUUAUUCUG | 19 | 16265 |
| HSV1-UL30-1878 | - | ACCAGAAGGGCUUUAUUCUG | 20 | 4927 |
| HSV1-UL30-7326 | - | GACCAGAAGGGCUUUAUUCUG | 21 | 16266 |
| HSV1-UL30-7327 | - | CGACCAGAAGGGCUUUAUUCUG | 22 | 16267 |
| HSV1-UL30-7328 | - | CCGACCAGAAGGGCUUUAUUCUG | 23 | 16268 |
| HSV1-UL30-7329 | - | GCCGACCAGAAGGGCUUUAUUCUG | 24 | 16269 |
| HSV1-UL30-7330 | - | CGGGGCGUGUUUCGCGUG | 18 | 16270 |
| HSV1-UL30-7331 | - | CCGGGGCGUGUUUCGCGUG | 19 | 16271 |
| HSV1-UL30-1852 | - | GCCGGGGCGUGUUUCGCGUG | 20 | 4902 |
| HSV1-UL30-7332 | - | GGCCGGGGCGUGUUUCGCGUG | 21 | 16272 |
| HSV1-UL30-7333 | - | CGGCCGGGGCGUGUUUCGCGUG | 22 | 16273 |
| HSV1-UL30-7334 | - | ACGGCCGGGGCGUGUUUCGCGUG | 23 | 16274 |
| HSV1-UL30-7335 | - | AACGGCCGGGGCGUGUUUCGCGUG | 24 | 16275 |
| HSV1-UL30-7336 | - | UACCUGGAGAUCGAGGUG | 18 | 16276 |
| HSV1-UL30-7337 | - | CUACCUGGAGAUCGAGGUG | 19 | 16277 |
| HSV1-UL30-1948 | - | ACUACCUGGAGAUCGAGGUG | 20 | 4981 |
| HSV1-UL30-7338 | - | GACUACCUGGAGAUCGAGGUG | 21 | 16278 |
| HSV1-UL30-7339 | - | GGACUACCUGGAGAUCGAGGUG | 22 | 16279 |
| HSV1-UL30-7340 | - | AGGACUACCUGGAGAUCGAGGUG | 23 | 16280 |
| HSV1-UL30-7341 | - | AAGGACUACCUGGAGAUCGAGGUG | 24 | 16281 |
| HSV1-UL30-7342 | - | CACUUCGAGGCGGAGGUG | 18 | 16282 |
| HSV1-UL30-7343 | - | CCACUUCGAGGCGGAGGUG | 19 | 16283 |
| HSV1-UL30-1791 | - | ACCACUUCGAGGCGGAGGUG | 20 | 4841 |
| HSV1-UL30-7344 | - | GACCACUUCGAGGCGGAGGUG | 21 | 16284 |
| HSV1-UL30-7345 | - | GGACCACUUCGAGGCGGAGGUG | 22 | 16285 |
| HSV1-UL30-7346 | - | CGGACCACUUCGAGGCGGAGGUG | 23 | 16286 |
| HSV1-UL30-7347 | - | GCGGACCACUUCGAGGCGGAGGUG | 24 | 16287 |
| HSV1-UL30-7348 | - | UGGAAUUCGACAGCGAAU | 18 | 16288 |
| HSV1-UL30-7349 | - | CUGGAAUUCGACAGCGAAU | 19 | 16289 |
| HSV1-UL30-1841 | - | UCUGGAAUUCGACAGCGAAU | 20 | 4891 |
| HSV1-UL30-7350 | - | UUCUGGAAUUCGACAGCGAAU | 21 | 16290 |
| HSV1-UL30-7351 | - | GUUCUGGAAUUCGACAGCGAAU | 22 | 16291 |
| HSV1-UL30-7352 | - | GGUUCUGGAAUUCGACAGCGAAU | 23 | 16292 |
| HSV1-UL30-7353 | - | UGGUUCUGGAAUUCGACAGCGAAU | 24 | 16293 |
| HSV1-UL30-7354 | - | UUUAAGUUUUUGCCCCAU | 18 | 16294 |
| HSV1-UL30-7355 | - | UUUUAAGUUUUUGCCCCAU | 19 | 16295 |
| HSV1-UL30-1873 | - | UUUUUAAGUUUUUGCCCCAU | 20 | 4922 |
| HSV1-UL30-7356 | - | UUUUUUAAGUUUUUGCCCCAU | 21 | 16296 |
| HSV1-UL30-7357 | - | GUUUUUUAAGUUUUUGCCCCAU | 22 | 16297 |
| HSV1-UL30-7358 | - | UGUUUUUUAAGUUUUUGCCCCAU | 23 | 16298 |
| HSV1-UL30-7359 | - | CUGUUUUUUAAGUUUUUGCCCCAU | 24 | 16299 |
| HSV1-UL30-7360 | - | GCGGGACUGGCUCGCCAU | 18 | 16300 |
| HSV1-UL30-7361 | - | UGCGGGACUGGCUCGCCAU | 19 | 16301 |
| HSV1-UL30-1955 | - | CUGCGGGACUGGCUCGCCAU | 20 | 4988 |
| HSV1-UL30-7362 | - | CCUGCGGGACUGGCUCGCCAU | 21 | 16302 |
| HSV1-UL30-7363 | - | UCCUGCGGGACUGGCUCGCCAU | 22 | 16303 |
| HSV1-UL30-7364 | - | CUCCUGCGGGACUGGCUCGCCAU | 23 | 16304 |
| HSV1-UL30-7365 | - | CCUCCUGCGGGACUGGCUCGCCAU | 24 | 16305 |
| HSV1-UL30-7366 | - | GCAUCUCCGCGGACCACU | 18 | 16306 |
| HSV1-UL30-7367 | - | GGCAUCUCCGCGGACCACU | 19 | 16307 |
| HSV1-UL30-1788 | - | CGGCAUCUCCGCGGACCACU | 20 | 4838 |
| HSV1-UL30-7368 | - | GCGGCAUCUCCGCGGACCACU | 21 | 16308 |
| HSV1-UL30-7369 | - | CGCGGCAUCUCCGCGGACCACU | 22 | 16309 |
| HSV1-UL30-7370 | - | CCGCGGCAUCUCCGCGGACCACU | 23 | 16310 |
| HSV1-UL30-7371 | - | UCCGCGGCAUCUCCGCGGACCACU | 24 | 16311 |
| HSV1-UL30-7372 | - | ACGCUCCUGGGCCUGACU | 18 | 16312 |
| HSV1-UL30-7373 | - | CACGCUCCUGGGCCUGACU | 19 | 16313 |
| HSV1-UL30-1775 | - | UCACGCUCCUGGGCCUGACU | 20 | 4825 |
| HSV1-UL30-7374 | - | AUCACGCUCCUGGGCCUGACU | 21 | 16314 |
| HSV1-UL30-7375 | - | CAUCACGCUCCUGGGCCUGACU | 22 | 16315 |
| HSV1-UL30-7376 | - | UCAUCACGCUCCUGGGCCUGACU | 23 | 16316 |
| HSV1-UL30-7377 | - | GUCAUCACGCUCCUGGGCCUGACU | 24 | 16317 |
| HSV1-UL30-7378 | - | GCACCGACGUGUACUACU | 18 | 16318 |
| HSV1-UL30-7379 | - | CGCACCGACGUGUACUACU | 19 | 16319 |
| HSV1-UL30-1793 | - | GCGCACCGACGUGUACUACU | 20 | 4843 |
| HSV1-UL30-7380 | - | AGCGCACCGACGUGUACUACU | 21 | 16320 |
| HSV1-UL30-7381 | - | GAGCGCACCGACGUGUACUACU | 22 | 16321 |
| HSV1-UL30-7382 | - | GGAGCGCACCGACGUGUACUACU | 23 | 16322 |
| HSV1-UL30-7383 | - | UGGAGCGCACCGACGUGUACUACU | 24 | 16323 |
| HSV1-UL30-7384 | - | CUUCAGCACGCUCUCCCU | 18 | 16324 |
| HSV1-UL30-7385 | - | GCUUCAGCACGCUCUCCCU | 19 | 16325 |
| HSV1-UL30-1937 | - | UGCUUCAGCACGCUCUCCCU | 20 | 4970 |
| HSV1-UL30-7386 | - | GUGCUUCAGCACGCUCUCCCU | 21 | 16326 |
| HSV1-UL30-7387 | - | UGUGCUUCAGCACGCUCUCCCU | 22 | 16327 |
| HSV1-UL30-7388 | - | CUGUGCUUCAGCACGCUCUCCCU | 23 | 16328 |
| HSV1-UL30-7389 | - | CCUGUGCUUCAGCACGCUCUCCCU | 24 | 16329 |
| HSV1-UL30-7390 | - | ACGCGCGCUGGGCGGCCU | 18 | 16330 |
| HSV1-UL30-7391 | - | CACGCGCGCUGGGCGGCCU | 19 | 16331 |
| HSV1-UL30-1970 | - | CCACGCGCGCUGGGCGGCCU | 20 | 5003 |
| HSV1-UL30-7392 | - | UCCACGCGCGCUGGGCGGCCU | 21 | 16332 |
| HSV1-UL30-7393 | - | GUCCACGCGCGCUGGGCGGCCU | 22 | 16333 |
| HSV1-UL30-7394 | - | CGUCCACGCGCGCUGGGCGGCCU | 23 | 16334 |
| HSV1-UL30-7395 | - | ACGUCCACGCGCGCUGGGCGGCCU | 24 | 16335 |
| HSV1-UL30-7396 | - | CGGCGGCCCCGAUGGCCU | 18 | 16336 |
| HSV1-UL30-7397 | - | CCGGCGGCCCCGAUGGCCU | 19 | 16337 |
| HSV1-UL30-1806 | - | GCCGGCGGCCCCGAUGGCCU | 20 | 4856 |
| HSV1-UL30-7398 | - | AGCCGGCGGCCCCGAUGGCCU | 21 | 16338 |
| HSV1-UL30-7399 | - | CAGCCGGCGGCCCCGAUGGCCU | 22 | 16339 |
| HSV1-UL30-7400 | - | CCAGCCGGCGGCCCCGAUGGCCU | 23 | 16340 |
| HSV1-UL30-7401 | - | CCCAGCCGGCGGCCCCGAUGGCCU | 24 | 16341 |
| HSV1-UL30-7402 | - | CCUCCUCAGCAUCCUCCU | 18 | 16342 |
| HSV1-UL30-7403 | - | GCCUCCUCAGCAUCCUCCU | 19 | 16343 |
| HSV1-UL30-1953 | - | AGCCUCCUCAGCAUCCUCCU | 20 | 4986 |
| HSV1-UL30-7404 | - | GAGCCUCCUCAGCAUCCUCCU | 21 | 16344 |
| HSV1-UL30-7405 | - | AGAGCCUCCUCAGCAUCCUCCU | 22 | 16345 |
| HSV1-UL30-7406 | - | GAGAGCCUCCUCAGCAUCCUCCU | 23 | 16346 |
| HSV1-UL30-7407 | - | AGAGAGCCUCCUCAGCAUCCUCCU | 24 | 16347 |
| HSV1-UL30-7408 | - | CGACGUGGCCGCGCGGCU | 18 | 16348 |
| HSV1-UL30-7409 | - | ACGACGUGGCCGCGCGGCU | 19 | 16349 |
| HSV1-UL30-2042 | - | GACGACGUGGCCGCGCGGCU | 20 | 5075 |
| HSV1-UL30-7410 | - | GGACGACGUGGCCGCGCGGCU | 21 | 16350 |
| HSV1-UL30-7411 | - | CGGACGACGUGGCCGCGCGGCU | 22 | 16351 |
| HSV1-UL30-7412 | - | CCGGACGACGUGGCCGCGCGGCU | 23 | 16352 |
| HSV1-UL30-7413 | - | CCCGGACGACGUGGCCGCGCGGCU | 24 | 16353 |
| HSV1-UL30-7414 | - | AUUCCAUGCGCAUCAUCU | 18 | 16354 |
| HSV1-UL30-7415 | - | UAUUCCAUGCGCAUCAUCU | 19 | 16355 |
| HSV1-UL30-1974 | - | CUAUUCCAUGCGCAUCAUCU | 20 | 5007 |
| HSV1-UL30-7416 | - | CCUAUUCCAUGCGCAUCAUCU | 21 | 16356 |
| HSV1-UL30-7417 | - | CCCUAUUCCAUGCGCAUCAUCU | 22 | 16357 |
| HSV1-UL30-7418 | - | GCCCUAUUCCAUGCGCAUCAUCU | 23 | 16358 |
| HSV1-UL30-7419 | - | GGCCCUAUUCCAUGCGCAUCAUCU | 24 | 16359 |
| HSV1-UL30-7420 | - | AGUACAUCGGCGUCAUCU | 18 | 16360 |
| HSV1-UL30-7421 | - | AAGUACAUCGGCGUCAUCU | 19 | 16361 |
| HSV1-UL30-1984 | - | AAAGUACAUCGGCGUCAUCU | 20 | 5017 |
| HSV1-UL30-7422 | - | AAAAGUACAUCGGCGUCAUCU | 21 | 16362 |
| HSV1-UL30-7423 | - | AAAAAGUACAUC GGC GUCAUCU | 22 | 16363 |
| HSV1-UL30-7424 | - | GAAAAAGUACAUCGGCGUCAUCU | 23 | 16364 |
| HSV1-UL30-7425 | - | AGAAAAAGUACAUCGGCGUCAUCU | 24 | 16365 |
| HSV1-UL30-7426 | - | GCGCCCCACGAGAUCUCU | 18 | 16366 |
| HSV1-UL30-7427 | - | CGCGCCCCACGAGAUCUCU | 19 | 16367 |
| HSV1-UL30-1784 | - | CCGCGCCCCACGAGAUCUCU | 20 | 4834 |
| HSV1-UL30-7428 | - | GCCGCGCCCCACGAGAUCUCU | 21 | 16368 |
| HSV1-UL30-7429 | - | UGCCGCGCCCCACGAGAUCUCU | 22 | 16369 |
| HSV1-UL30-7430 | - | AUGCCGCGCCCCACGAGAUCUCU | 23 | 16370 |
| HSV1-UL30-7431 | - | AAUGCCGCGCCCCACGAGAUCUCU | 24 | 16371 |
| HSV1-UL30-7432 | - | UUUACGACGAUACCGUCU | 18 | 16372 |
| HSV1-UL30-7433 | - | UUUUACGACGAUACCGUCU | 19 | 16373 |
| HSV1-UL30-1990 | - | GUUUUACGACGAUACCGUCU | 20 | 5023 |
| HSV1-UL30-7434 | - | UGUUUUACGACGAUACCGUCU | 21 | 16374 |
| HSV1-UL30-7435 | - | CUGUUUUACGACGAUACCGUCU | 22 | 16375 |
| HSV1-UL30-7436 | - | GCUGUUUUACGACGAUACCGUCU | 23 | 16376 |
| HSV1-UL30-7437 | - | UGCUGUUUUACGACGAUACCGUCU | 24 | 16377 |
| HSV1-UL30-7438 | - | GCCCGGCCAUCAAGAAGU | 18 | 16378 |
| HSV1-UL30-7439 | - | UGCCCGGCCAUCAAGAAGU | 19 | 16379 |
| HSV1-UL30-1797 | - | CUGCCCGGCCAUCAAGAAGU | 20 | 4847 |
| HSV1-UL30-7440 | - | UCUGCCCGGCCAUCAAGAAGU | 21 | 16380 |
| HSV1-UL30-7441 | - | UUCUGCCCGGCCAUCAAGAAGU | 22 | 16381 |
| HSV1-UL30-7442 | - | CUUCUGCCCGGCCAUCAAGAAGU | 23 | 16382 |
| HSV1-UL30-7443 | - | ACUUCUGCCCGGCCAUCAAGAAGU | 24 | 16383 |
| HSV1-UL30-7444 | - | CCCCCAUCAAACUCGAGU | 18 | 16384 |
| HSV1-UL30-7445 | - | CCCCCCAUCAAACUCGAGU | 19 | 16385 |
| HSV1-UL30-1982 | - | GCCCCCCAUCAAACUCGAGU | 20 | 5015 |
| HSV1-UL30-7446 | - | UGCCCCCCAUCAAACUCGAGU | 21 | 16386 |
| HSV1-UL30-7447 | - | CUGCCCCCCAUCAAACUCGAGU | 22 | 16387 |
| HSV1-UL30-7448 | - | UCUGCCCCCCAUCAAACUCGAGU | 23 | 16388 |
| HSV1-UL30-7449 | - | UUCUGCCCCCCAUCAAACUCGAGU | 24 | 16389 |
| HSV1-UL30-7450 | - | CUUCGUCAAGGCUCACGU | 18 | 16390 |
| HSV1-UL30-7451 | - | UCUUCGUCAAGGCUCACGU | 19 | 16391 |
| HSV1-UL30-1950 | - | UUCUUCGUCAAGGCUCACGU | 20 | 4983 |
| HSV1-UL30-7452 | - | GUUCUUC GUCAAGGCUCAC GU | 21 | 16392 |
| HSV1-UL30-7453 | - | UGUUCUUC GUCAAGGCUCAC GU | 22 | 16393 |
| HSV1-UL30-7454 | - | CUGUUCUUCGUCAAGGCUCACGU | 23 | 16394 |
| HSV1-UL30-7455 | - | GCUGUUCUUCGUCAAGGCUCACGU | 24 | 16395 |
| HSV1-UL30-7456 | - | GUUUUACCGCGUCUACGU | 18 | 16396 |
| HSV1-UL30-7457 | - | UGUUUUACCGCGUCUACGU | 19 | 16397 |
| HSV1-UL30-1794 | - | CUGUUUUACCGCGUCUACGU | 20 | 4844 |
| HSV1-UL30-7458 | - | UCUGUUUUACCGCGUCUACGU | 21 | 16398 |
| HSV1-UL30-7459 | - | CUCUGUUUUACCGCGUCUACGU | 22 | 16399 |
| HSV1-UL30-7460 | - | GCUCUGUUUUACCGCGUCUACGU | 23 | 16400 |
| HSV1-UL30-7461 | - | CGCUCUGUUUUACCGCGUCUACGU | 24 | 16401 |
| HSV1-UL30-7462 | - | CCGGGGCGUGUUUCGCGU | 18 | 16402 |
| HSV1-UL30-7463 | - | GCCGGGGCGUGUUUCGCGU | 19 | 16403 |
| HSV1-UL30-1851 | - | GGCCGGGGCGUGUUUCGCGU | 20 | 4901 |
| HSV1-UL30-7464 | - | CGGCCGGGGCGUGUUUCGCGU | 21 | 16404 |
| HSV1-UL30-7465 | - | ACGGCCGGGGCGUGUUUCGCGU | 22 | 16405 |
| HSV1-UL30-7466 | - | AACGGCCGGGGCGUGUUUCGCGU | 23 | 16406 |
| HSV1-UL30-7467 | - | GAACGGCCGGGGCGUGUUUCGCGU | 24 | 16407 |
| HSV1-UL30-7468 | - | CCGAGGGACUGCAGGCGU | 18 | 16408 |
| HSV1-UL30-7469 | - | CCCGAGGGACUGCAGGCGU | 19 | 16409 |
| HSV1-UL30-2000 | - | GCCCGAGGGACUGCAGGCGU | 20 | 5033 |
| HSV1-UL30-7470 | - | UGCCCGAGGGACUGCAGGCGU | 21 | 16410 |
| HSV1-UL30-7471 | - | CUGCCCGAGGGACUGCAGGCGU | 22 | 16411 |
| HSV1-UL30-7472 | - | CCUGCCCGAGGGACUGCAGGCGU | 23 | 16412 |
| HSV1-UL30-7473 | - | CCCUGCCCGAGGGACUGCAGGCGU | 24 | 16413 |
| HSV1-UL30-7474 | - | CUACCUGGAGAUCGAGGU | 18 | 16414 |
| HSV1-UL30-7475 | - | ACUACCUGGAGAUCGAGGU | 19 | 16415 |
| HSV1-UL30-1947 | - | GACUACCUGGAGAUCGAGGU | 20 | 4980 |
| HSV1-UL30-7476 | - | GGACUACCUGGAGAUCGAGGU | 21 | 16416 |
| HSV1-UL30-7477 | - | AGGACUACCUGGAGAUCGAGGU | 22 | 16417 |
| HSV1-UL30-7478 | - | AAGGACUACCUGGAGAUCGAGGU | 23 | 16418 |
| HSV1-UL30-7479 | - | CAAGGACUACCUGGAGAUCGAGGU | 24 | 16419 |
| HSV1-UL30-7480 | - | GGCUCCGGACCGCAGGGU | 18 | 16420 |
| HSV1-UL30-7481 | - | CGGCUCCGGACCGCAGGGU | 19 | 16421 |
| HSV1-UL30-2044 | - | GCGGCUCCGGACCGCAGGGU | 20 | 5077 |
| HSV1-UL30-7482 | - | CGCGGCUCCGGACCGCAGGGU | 21 | 16422 |
| HSV1-UL30-7483 | - | GCGCGGCUCCGGACCGCAGGGU | 22 | 16423 |
| HSV1-UL30-7484 | - | CGCGCGGCUCCGGACCGCAGGGU | 23 | 16424 |
| HSV1-UL30-7485 | - | CCGCGCGGCUCCGGACCGCAGGGU | 24 | 16425 |
| HSV1-UL30-7486 | - | UGGUGAACAUCGACAUGU | 18 | 16426 |
| HSV1-UL30-7487 | - | AUGGUGAACAUCGACAUGU | 19 | 16427 |
| HSV1-UL30-1857 | - | CAUGGUGAACAUCGACAUGU | 20 | 4907 |
| HSV1-UL30-7488 | - | GCAUGGUGAACAUCGACAUGU | 21 | 16428 |
| HSV1-UL30-7489 | - | GGCAUGGUGAACAUCGACAUGU | 22 | 16429 |
| HSV1-UL30-7490 | - | CGGCAUGGUGAACAUCGACAUGU | 23 | 16430 |
| HSV1-UL30-7491 | - | ACGGCAUGGUGAACAUCGACAUGU | 24 | 16431 |
| HSV1-UL30-7492 | - | AUUACUUCUCCCACCUGU | 18 | 16432 |
| HSV1-UL30-7493 | - | UAUUACUUCUCCCACCUGU | 19 | 16433 |
| HSV1-UL30-2033 | - | CUAUUACUUCUCCCACCUGU | 20 | 5066 |
| HSV1-UL30-7494 | - | ACUAUUACUUCUCCCACCUGU | 21 | 16434 |
| HSV1-UL30-7495 | - | GACUAUUACUUCUCCCACCUGU | 22 | 16435 |
| HSV1-UL30-7496 | - | GGACUAUUACUUCUCCCACCUGU | 23 | 16436 |
| HSV1-UL30-7497 | - | CGGACUAUUACUUCUCCCACCUGU | 24 | 16437 |
| HSV1-UL30-7498 | - | UGACAUUCAAGGCCCUGU | 18 | 16438 |
| HSV1-UL30-7499 | - | GUGACAUUCAAGGCCCUGU | 19 | 16439 |
| HSV1-UL30-2035 | - | CGUGACAUUCAAGGCCCUGU | 20 | 5068 |
| HSV1-UL30-7500 | - | GCGUGACAUUCAAGGCCCUGU | 21 | 16440 |
| HSV1-UL30-7501 | - | UGCGUGACAUUCAAGGCCCUGU | 22 | 16441 |
| HSV1-UL30-7502 | - | GUGCGUGACAUUCAAGGCCCUGU | 23 | 16442 |
| HSV1-UL30-7503 | - | CGUGCGUGACAUUCAAGGCCCUGU | 24 | 16443 |
| HSV1-UL30-7504 | - | CCCGCAAGCUGCUGGUGU | 18 | 16444 |
| HSV1-UL30-7505 | - | CCCCGCAAGCUGCUGGUGU | 19 | 16445 |
| HSV1-UL30-2027 | - | GCCCCGCAAGCUGCUGGUGU | 20 | 5060 |
| HSV1-UL30-7506 | - | AGCCCCGCAAGCUGCUGGUGU | 21 | 16446 |
| HSV1-UL30-7507 | - | AAGCCCCGCAAGCUGCUGGUGU | 22 | 16447 |
| HSV1-UL30-7508 | - | CAAGCCCCGCAAGCUGCUGGUGU | 23 | 16448 |
| HSV1-UL30-7509 | - | CCAAGCCCCGCAAGCUGCUGGUGU | 24 | 16449 |
| HSV1-UL30-7510 | - | GGACACCCAGGGGCGAUU | 18 | 16450 |
| HSV1-UL30-7511 | - | CGGACACCCAGGGGCGAUU | 19 | 16451 |
| HSV1-UL30-1881 | - | CCGGACACCCAGGGGCGAUU | 20 | 4930 |
| HSV1-UL30-7512 | - | GCCGGACACCCAGGGGCGAUU | 21 | 16452 |
| HSV1-UL30-7513 | - | UGCCGGACACCCAGGGGCGAUU | 22 | 16453 |
| HSV1-UL30-7514 | - | CUGCCGGACACCCAGGGGCGAUU | 23 | 16454 |
| HSV1-UL30-7515 | - | UCUGCCGGACACCCAGGGGCGAUU | 24 | 16455 |
| HSV1-UL30-7516 | - | UUGGCCUUCAUGACCCUU | 18 | 16456 |
| HSV1-UL30-7517 | - | GUUGGCCUUCAUGACCCUU | 19 | 16457 |
| HSV1-UL30-1842 | - | UGUUGGCCUUCAUGACCCUU | 20 | 4892 |
| HSV1-UL30-7518 | - | CUGUUGGCCUUCAUGACCCUU | 21 | 16458 |
| HSV1-UL30-7519 | - | GCUGUUGGCCUUCAUGACCCUU | 22 | 16459 |
| HSV1-UL30-7520 | - | UGCUGUUGGCCUUCAUGACCCUU | 23 | 16460 |
| HSV1-UL30-7521 | - | AUGCUGUUGGCCUUCAUGACCCUU | 24 | 16461 |
| HSV1-UL30-7522 | - | GGCGGCCCCGAUGGCCUU | 18 | 16462 |
| HSV1-UL30-7523 | - | CGGCGGCCCCGAUGGCCUU | 19 | 16463 |
| HSV1-UL30-1807 | - | CCGGCGGCCCCGAUGGCCUU | 20 | 4857 |
| HSV1-UL30-7524 | - | GCCGGCGGCCCCGAUGGCCUU | 21 | 16464 |
| HSV1-UL30-7525 | - | AGCCGGCGGCCCCGAUGGCCUU | 22 | 16465 |
| HSV1-UL30-7526 | - | CAGCCGGCGGCCCCGAUGGCCUU | 23 | 16466 |
| HSV1-UL30-7527 | - | CCAGCCGGCGGCCCCGAUGGCCUU | 24 | 16467 |
| HSV1-UL30-7528 | - | CGAGGGACUGCAGGCGUU | 18 | 16468 |
| HSV1-UL30-7529 | - | CCGAGGGACUGCAGGCGUU | 19 | 16469 |
| HSV1-UL30-2001 | - | CCCGAGGGACUGCAGGCGUU | 20 | 5034 |
| HSV1-UL30-7530 | - | GCCCGAGGGACUGCAGGCGUU | 21 | 16470 |
| HSV1-UL30-7531 | - | UGCCCGAGGGACUGCAGGCGUU | 22 | 16471 |
| HSV1-UL30-7532 | - | CUGCCCGAGGGACUGCAGGCGUU | 23 | 16472 |
| HSV1-UL30-7533 | - | CCUGCCCGAGGGACUGCAGGCGUU | 24 | 16473 |
| HSV1-UL30-7534 | - | GCUCCGGACCGCAGGGUU | 18 | 16474 |
| HSV1-UL30-7535 | - | GGCUCCGGACCGCAGGGUU | 19 | 16475 |
| HSV1-UL30-2045 | - | CGGCUCCGGACCGCAGGGUU | 20 | 5078 |
| HSV1-UL30-7536 | - | GCGGCUCCGGACCGCAGGGUU | 21 | 16476 |
| HSV1-UL30-7537 | - | CGCGGCUCCGGACCGCAGGGUU | 22 | 16477 |
| HSV1-UL30-7538 | - | GCGCGGCUCCGGACCGCAGGGUU | 23 | 16478 |
| HSV1-UL30-7539 | - | CGCGCGGCUCCGGACCGCAGGGUU | 24 | 16479 |
| HSV1-UL30-7540 | - | CUGCCCACGCCCGUGGUU | 18 | 16480 |
| HSV1-UL30-7541 | - | CCUGCCCACGCCCGUGGUU | 19 | 16481 |
| HSV1-UL30-1838 | - | GCCUGCCCACGCCCGUGGUU | 20 | 4888 |
| HSV1-UL30-7542 | - | GGCCUGCCCACGCCCGUGGUU | 21 | 16482 |
| HSV1-UL30-7543 | - | GGGCCUGCCCACGCCCGUGGUU | 22 | 16483 |
| HSV1-UL30-7544 | - | GGGGCCUGCCCACGCCCGUGGUU | 23 | 16484 |
| HSV1-UL30-7545 | - | AGGGGCCUGCCCACGCCCGUGGUU | 24 | 16485 |
| HSV1-UL30-7546 | - | UUACUUCUCCCACCUGUU | 18 | 16486 |
| HSV1-UL30-7547 | - | AUUACUUCUCCCACCUGUU | 19 | 16487 |
| HSV1-UL30-2034 | - | UAUUACUUCUCCCACCUGUU | 20 | 5067 |
| HSV1-UL30-7548 | - | CUAUUACUUCUCCCACCUGUU | 21 | 16488 |
| HSV1-UL30-7549 | - | ACUAUUACUUCUCCCACCUGUU | 22 | 16489 |
| HSV1-UL30-7550 | - | GACUAUUACUUCUCCCACCUGUU | 23 | 16490 |
| HSV1-UL30-7551 | - | GGACUAUUACUUCUCCCACCUGUU | 24 | 16491 |
| HSV1-UL30-7552 | - | GACAUUCAAGGCCCUGUU | 18 | 16492 |
| HSV1-UL30-7553 | - | UGACAUUCAAGGCCCUGUU | 19 | 16493 |
| HSV1-UL30-2036 | - | GUGACAUUCAAGGCCCUGUU | 20 | 5069 |
| HSV1-UL30-7554 | - | CGUGACAUUCAAGGCCCUGUU | 21 | 16494 |
| HSV1-UL30-7555 | - | GCGUGACAUUCAAGGCCCUGUU | 22 | 16495 |
| HSV1-UL30-7556 | - | UGCGUGACAUUCAAGGCCCUGUU | 23 | 16496 |
| HSV1-UL30-7557 | - | GUGCGUGACAUUCAAGGCCCUGUU | 24 | 16497 |
| HSV1-UL30-7558 | - | GACACCCAGGGGCGAUUU | 18 | 16498 |
| HSV1-UL30-7559 | - | GGACACCCAGGGGCGAUUU | 19 | 16499 |
| HSV1-UL30-1882 | - | CGGACACCCAGGGGCGAUUU | 20 | 4931 |
| HSV1-UL30-7560 | - | CCGGACACCCAGGGGCGAUUU | 21 | 16500 |
| HSV1-UL30-7561 | - | GCCGGACACCCAGGGGCGAUUU | 22 | 16501 |
| HSV1-UL30-7562 | - | UGCCGGACACCCAGGGGCGAUUU | 23 | 16502 |
| HSV1-UL30-7563 | - | CUGCCGGACACCCAGGGGCGAUUU | 24 | 16503 |
| HSV1-UL30-4371 | - | CAGUUCCACGCGCGGUUU | 18 | 13311 |
| HSV1-UL30-4372 | - | CCAGUUCCACGCGCGGUUU | 19 | 13312 |
| HSV1-UL30-4373 | - | CCCAGUUCCACGCGCGGUUU | 20 | 13313 |
| HSV1-UL30-4374 | - | GCCCAGUUCCACGCGCGGUUU | 21 | 13314 |
| HSV1-UL30-4375 | - | GGCCCAGUUCCACGCGCGGUUU | 22 | 13315 |
| HSV1-UL30-4376 | - | CGGCCCAGUUCCACGCGCGGUUU | 23 | 13316 |
| HSV1-UL30-4377 | - | GCGGCCCAGUUCCACGCGCGGUUU | 24 | 13317 |

**Table 10A** provides exemplary targeting domains for knocking out the *UL30* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 10A**

| **1st Tier** | | | | |
|---|---|---|---|---|
| **gRNA Name** | **DNA Strand** | **Targeting Domain** | **Target Site Length** | **Seq ID NO:** |
| HSV1-UL30-7564 | + | GACCGUCGUGCACCCCG | 17 | 16504 |
| HSV1-UL30-7565 | + | GGCGCUGGGUUGGCCCG | 17 | 16505 |
| HSV1-UL30-7566 | + | GGUGACCGUCGUGCACCCCG | 20 | 16506 |

**Table 10B** provides exemplary targeting domains for knocking out the *UL30* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 10B**

| **2nd Tier** | | | | |
|---|---|---|---|---|
| **gRNA Name** | **DNA Strand** | **Targeting Domain** | **Target Site Length** | **Seq ID NO:** |
| HSV1-UL30-420 | - | AUAGCGAAUGCGAUGAA | 17 | 5829 |
| HSV1-UL30-7567 | - | UCCUCCGCGUCGGGUCG | 17 | 16507 |
| HSV1-UL30-7568 | + | CGCAGUACACCUUGGGG | 17 | 16508 |
| HSV1-UL30-419 | - | ACUAUAGCGAAUGCGAUGAA | 20 | 5828 |
| HSV1-UL30-7569 | + | UAUGGCGCUGGGUUGGCCCG | 20 | 16509 |
| HSV1-UL30-7570 | - | ACGUCCUCCGCGUCGGGUCG | 20 | 16510 |
| HSV1-UL30-7571 | + | CCCCGCAGUACACCUUGGGG | 20 | 16511 |

**Table 10C** provides exemplary targeting domains for knocking out the *UL30* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 10C**

| **5th Tier** | | | | |
|---|---|---|---|---|
| **gRNA Name** | **DNA Strand** | **Targeting Domain** | **Target Site Length** | **Seq ID NO:** |
| HSV1-UL30-7572 | - | UCAUCCAGGCCCACAAC | 17 | 16512 |
| HSV1-UL30-7573 | + | CAGGUACGACAGCACAC | 17 | 16513 |
| HSV1-UL30-2796 | + | AUUCGAUAUCGAAGCAC | 17 | 5793 |
| HSV1-UL30-7574 | + | CGGCCAGCUCGGACACC | 17 | 16514 |
| HSV1-UL30-7575 | + | CGACCUUGAUGGCGGCC | 17 | 16515 |
| HSV1-UL30-7576 | + | CCUCCCGGGCUGCGGCC | 17 | 16516 |
| HSV1-UL30-2642 | + | CCUGCGCGCGGCGGGCC | 17 | 5641 |
| HSV1-UL30-3018 | + | ACACCGUCAGGUGGGCC | 17 | 11958 |
| HSV1-UL30-7577 | + | GGACCUUGUAAAUGUCC | 17 | 16517 |
| HSV1-UL30-7578 | + | UUUUCUUGGCGAUCAGC | 17 | 16518 |
| HSV1-UL30-7579 | - | UACCGGCAUACAAGCUC | 17 | 16519 |
| HSV1-UL30-2753 | + | CGUUGAGCUUGUAGCUC | 17 | 5751 |
| HSV1-UL30-7580 | - | UGGAGCUCUCGGCCGUC | 17 | 16520 |
| HSV1-UL30-2159 | - | UGCGCCUGGCCGACCAG | 17 | 5191 |
| HSV1-UL30-2840 | - | UUCUGCCGGACACCCAG | 17 | 5835 |
| HSV1-UL30-7581 | + | UUCUUCUUGUCCUUCAG | 17 | 16521 |
| HSV1-UL30-2774 | + | GCCGCCAGCUCGUUCAG | 17 | 5772 |
| HSV1-UL30-2751 | + | CGGCUUCGGCCACGGCG | 17 | 5749 |
| HSV1-UL30-3202 | + | GCGACGGCGUCUCCCGG | 17 | 12142 |
| HSV1-UL30-7582 | + | GGGGAAUCCGCGAGCGG | 17 | 16522 |
| HSV1-UL30-2607 | + | CGGACACCAGCAGCUUG | 17 | 5611 |
| HSV1-UL30-7583 | + | CGUUCACCUUUAUCUUG | 17 | 16523 |
| HSV1-UL30-2839 | - | AUCGGCGAGUACUGCAU | 17 | 5834 |
| HSV1-UL30-2842 | - | GCCUUCGAACAGCUCCU | 17 | 5837 |
| HSV1-UL30-2841 | - | GCGAAAGCAGAUCCGCU | 17 | 5836 |
| HSV1-UL30-2139 | - | GGUGAACAUCGACAUGU | 17 | 5172 |
| HSV1-UL30-7584 | - | GCAUCAUCCAGGCCCACAAC | 20 | 16524 |
| HSV1-UL30-7585 | + | GCACAGGUACGACAGCACAC | 20 | 16525 |
| HSV1-UL30-2547 | + | UGCAUUCGAUAUCGAAGCAC | 20 | 5552 |
| HSV1-UL30-7586 | + | CCUCGGCCAGCUCGGACACC | 20 | 16526 |
| HSV1-UL30-7587 | + | ACACGACCUUGAUGGCGGCC | 20 | 16527 |
| HSV1-UL30-7588 | + | CGUCCUCCCGGGCUGCGGCC | 20 | 16528 |
| HSV1-UL30-2393 | + | GGACCUGCGCGCGGCGGGCC | 20 | 5400 |
| HSV1-UL30-3484 | + | AAUACACCGUCAGGUGGGCC | 20 | 12424 |
| HSV1-UL30-7589 | + | GGGGGACCUUGUAAAUGUCC | 20 | 16529 |
| HSV1-UL30-7590 | + | ACUUUUUCUUGGCGAUCAGC | 20 | 16530 |
| HSV1-UL30-7591 | - | ACCUACCGGCAUACAAGCUC | 20 | 16531 |
| HSV1-UL30-2504 | + | CGGCGUUGAGCUUGUAGCUC | 20 | 5510 |
| HSV1-UL30-7592 | - | AUCUGGAGCUCUCGGCCGUC | 20 | 16532 |
| HSV1-UL30-1877 | - | UGCUGCGCCUGGCCGACCAG | 20 | 4926 |
| HSV1-UL30-2835 | - | UUAUUCUGCCGGACACCCAG | 20 | 5831 |
| HSV1-UL30-7593 | + | UCCUUCUUCUUGUCCUUCAG | 20 | 16533 |
| HSV1-UL30-2525 | + | CUGGCCGCCAGCUCGUUCAG | 20 | 5531 |
| HSV1-UL30-2502 | + | GGACGGCUUCGGCCACGGCG | 20 | 5508 |
| HSV1-UL30-3671 | + | CAGGCGACGGCGUCUCCCGG | 20 | 12611 |
| HSV1-UL30-7594 | + | UCUGGGGAAUCCGCGAGCGG | 20 | 16534 |
| HSV1-UL30-2358 | + | GCUCGGACACCAGCAGCUUG | 20 | 5370 |
| HSV1-UL30-7595 | + | UGCCGUUCACCUUUAUCUUG | 20 | 16535 |
| HSV1-UL30-2834 | - | GUGAUCGGCGAGUACUGCAU | 20 | 5830 |
| HSV1-UL30-2837 | - | GCGGCCUUCGAACAGCUCCU | 20 | 5833 |
| HSV1-UL30-2836 | - | CAUGCGAAAGCAGAUCCGCU | 20 | 5832 |
| HSV1-UL30-1857 | - | CAUGGUGAACAUCGACAUGU | 20 | 4907 |

**Table 11A** provides exemplary targeting domains for knocking out the *UL48* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 11A**

| 1st Tier | | | | |
|---|---|---|---|---|
| gRNA Name | DNA Strand | Targeting Domain | Target Site Length | Seq ID NO: |
| HSV1-UL48-120 | - | GCCGACAUGAACGCGGA | 17 | 5975 |
| HSV1-UL48-167 | + | GGGUGGCCGGAAGCGUA | 17 | 6035 |
| HSV1-UL48-214 | + | GCCCCGUUGCGUACAGC | 17 | 6062 |
| HSV1-UL48-119 | - | GUUUGCCGACAUGAACG | 17 | 5974 |
| HSV1-UL48-186 | + | GAGCAUGGUACAUAGCG | 17 | 5920 |
| HSV1-UL48-188 | + | GCAUGGUACAUAGCGCG | 17 | 5922 |
| HSV1-UL48-160 | + | GCGCCCGUAGCUCGGCG | 17 | 6031 |
| HSV1-UL48-152 | - | GCCACCCGCGACGGCCU | 17 | 5884 |
| HSV1-UL48-170 | + | GAAGGCCACGUCGCCGU | 17 | 5903 |
| HSV1-UL48-235 | + | GCCGUCCGCGUUCAUGU | 17 | 6073 |
| HSV1-UL48-1127 | - | GAGCUAUCGAACCGUGU | 17 | 16536 |
| HSV1-UL48-171 | + | GGGCGCGAAUGUCGAUU | 17 | 5904 |
| HSV1-UL48-173 | + | GUCGAUUUGGGUGCGUU | 17 | 6037 |
| HSV1-UL48-172 | + | GGCGCGAAUGUCGAUUU | 17 | 5905 |
| HSV1-UL48-223 | + | GCCGCCGGGGUGUUUUU | 17 | 5953 |
| HSV1-UL48-10 | - | GGCCCCCCCGCUGUACGCAA | 20 | 5840 |
| HSV1-UL48-50 | + | GGUGGCCGGAAGCGUAGGGA | 20 | 5993 |
| HSV1-UL48-36 | - | GUUUCUUCCACGCCGAGCUA | 20 | 5877 |
| HSV1-UL48-11 | - | GCCCCCCCGCUGUACGCAAC | 20 | 5841 |
| HSV1-UL48-62 | + | GGAAUUUACACUCCCGGUAC | 20 | 6000 |
| HSV1-UL48-96 | + | GGCGCCCCGUUGCGUACAGC | 20 | 6019 |
| HSV1-UL48-101 | + | GCGUACAGCGGGGGGGCCGC | 20 | 6020 |
| HSV1-UL48-45 | + | GUAGAGCCCGAGGCCGUCGC | 20 | 5892 |
| HSV1-UL48-107 | + | GUGUUUUUGGGACCCCCGGC | 20 | 5954 |
| HSV1-UL48-1 | - | GCUGUUUGCCGACAUGAACG | 20 | 5960 |
| HSV1-UL48-97 | + | GCGCCCCGUUGCGUACAGCG | 20 | 5938 |
| HSV1-UL48-78 | + | GACGGUUAAAGAGGGCGGCG | 20 | 5930 |
| HSV1-UL48-9 | - | GGGUCCCAAAAACACCCCGG | 20 | 5839 |
| HSV1-UL48-76 | + | GAGACGGUUAAAGAGGGCGG | 20 | 5928 |
| HSV1-UL48-99 | + | GCCCCGUUGCGUACAGCGGG | 20 | 5940 |
| HSV1-UL48-26 | - | GCCCAGCGAUGUGGUGGAAU | 20 | 5968 |
| HSV1-UL48-1128 | + | GACCAAGAGGUCCAUUGGGU | 20 | 16537 |
| HSV1-UL48-1129 | - | GGAGAGCUAUCGAACCGUGU | 20 | 16538 |
| HSV1-UL48-54 | + | GUGGGCGCGAAUGUCGAUUU | 20 | 5901 |
| HSV1-UL48-104 | + | GGGGGCCGCCGGGGUGUUUU | 20 | 5948 |
| HSV1-UL48-105 | + | GGGGCCGCCGGGGUGUUUUU | 20 | 5949 |

**Table 11B** provides exemplary targeting domains for knocking out the *UL48* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 11B**

| 2nd Tier | | | | |
|---|---|---|---|---|
| gRNA Name | DNA Strand | Targeting Domain | Target Site Length | Seq ID NO: |
| HSV1-UL48-128 | - | CCCCCCGCUGUACGCAA | 17 | 5850 |
| HSV1-UL48-148 | - | AUCGACAUUCGCGCCCA | 17 | 5986 |
| HSV1-UL48-190 | + | CCAAGUCGUCGAGGAGA | 17 | 6045 |
| HSV1-UL48-129 | - | CCCCCGCUGUACGCAAC | 17 | 5851 |
| HSV1-UL48-155 | - | CUUCCACGCCGAGCUAC | 17 | 5887 |
| HSV1-UL48-139 | - | CCAACGCCGACCUGUAC | 17 | 5867 |
| HSV1-UL48-140 | - | CAACGCCGACCUGUACC | 17 | 5868 |
| HSV1-UL48-187 | + | AGCAUGGUACAUAGCGC | 17 | 5921 |
| HSV1-UL48-195 | + | CGGUUAAAGAGGGCGGC | 17 | 5934 |
| HSV1-UL48-174 | + | UCGAUUUGGGUGCGUUC | 17 | 6038 |
| HSV1-UL48-191 | + | CGAGGAGACGGUUAAAG | 17 | 5926 |
| HSV1-UL48-236 | + | ACAGCUCGUCGACCAAG | 17 | 5959 |
| HSV1-UL48-213 | + | CGCCCCGUUGCGUACAG | 17 | 6061 |
| HSV1-UL48-130 | - | CCCCGCUGUACGCAACG | 17 | 5852 |
| HSV1-UL48-161 | + | CUUCGUAGUAGAGCCCG | 17 | 5894 |
| HSV1-UL48-215 | + | CCCCGUUGCGUACAGCG | 17 | 5942 |
| HSV1-UL48-134 | - | CGACUUGGGCUUUAGCG | 17 | 5856 |
| HSV1-UL48-156 | - | ACGCCGAGCUACGGGCG | 17 | 5888 |
| HSV1-UL48-162 | + | AGAGCCCGAGGCCGUCG | 17 | 6032 |
| HSV1-UL48-189 | + | UAAAGCCCAAGUCGUCG | 17 | 5923 |
| HSV1-UL48-175 | + | CGAUUUGGGUGCGUUCG | 17 | 6039 |
| HSV1-UL48-216 | + | CCCGUUGCGUACAGCGG | 17 | 5943 |
| HSV1-UL48-194 | + | ACGGUUAAAGAGGGCGG | 17 | 5933 |
| HSV1-UL48-217 | + | CCGUUGCGUACAGCGGG | 17 | 5944 |
| HSV1-UL48-158 | - | CGAGCUACGGGCGCGGG | 17 | 5987 |
| HSV1-UL48-218 | + | CGUUGCGUACAGCGGGG | 17 | 5945 |
| HSV1-UL48-1130 | + | UCGACCAAGAGGUCCAU | 17 | 16539 |
| HSV1-UL48-132 | - | CCGUCUCCUCGACGACU | 17 | 5854 |
| HSV1-UL48-159 | + | CUCCCGCGCCCGUAGCU | 17 | 5893 |
| HSV1-UL48-182 | + | CCGGUACAGGUCGGCGU | 17 | 5916 |
| HSV1-UL48-183 | + | UACAGGUCGGCGUUGGU | 17 | 5917 |
| HSV1-UL48-1131 | + | CGACCAAGAGGUCCAUU | 17 | 16540 |
| HSV1-UL48-133 | - | CGUCUCCUCGACGACUU | 17 | 5855 |
| HSV1-UL48-1132 | - | CCCGUAUCAACCCCACCCAA | 20 | 16541 |
| HSV1-UL48-30 | - | CAAAUCGACAUUCGCGCCCA | 20 | 5972 |
| HSV1-UL48-67 | + | CUCGUUCCAGGUAUCGAGCA | 20 | 5911 |
| HSV1-UL48-72 | + | AGCCCAAGUCGUCGAGGAGA | 20 | 6002 |
| HSV1-UL48-33 | - | ACGCUUCCGGCCACCCGCGA | 20 | 5874 |
| HSV1-UL48-2 | - | UUUGCCGACAUGAACGCGGA | 20 | 5961 |
| HSV1-UL48-1133 | + | UCCAUUGGGUGGGGUUGAUA | 20 | 16542 |
| HSV1-UL48-49 | + | CGCGGGUGGCCGGAAGCGUA | 20 | 5992 |
| HSV1-UL48-37 | - | UUUCUUCCACGCCGAGCUAC | 20 | 5878 |
| HSV1-UL48-21 | - | CGACCAACGCCGACCUGUAC | 20 | 5860 |
| HSV1-UL48-8 | - | CGGGGGUCCCAAAAACACCC | 20 | 5838 |
| HSV1-UL48-102 | + | CGUACAGCGGGGGGGCCGCC | 20 | 5946 |
| HSV1-UL48-108 | + | UGUUUUUGGGACCCCCGGCC | 20 | 5956 |
| HSV1-UL48-66 | + | CGAAAACAGAUCCUCGUUCC | 20 | 5910 |
| HSV1-UL48-69 | + | UCGAGCAUGGUACAUAGCGC | 20 | 5913 |
| HSV1-UL48-39 | - | CCACGCCGAGCUACGGGCGC | 20 | 5880 |
| HSV1-UL48-58 | + | CCCCCAUUCCACCACAUCGC | 20 | 5906 |
| HSV1-UL48-77 | + | AGACGGUUAAAGAGGGCGGC | 20 | 5929 |
| HSV1-UL48-110 | + | UUUGGGACCCCCGGCCGGGC | 20 | 6023 |
| HSV1-UL48-47 | + | CCGAGGCCGUCGCGGGUGGC | 20 | 5991 |
| HSV1-UL48-35 | - | CGGCCACCCGCGACGGCCUC | 20 | 5876 |
| HSV1-UL48-32 | - | CGUGGCCUUCCCUACGCUUC | 20 | 5873 |
| HSV1-UL48-56 | + | AUGUCGAUUUGGGUGCGUUC | 20 | 5995 |
| HSV1-UL48-73 | + | CGUCGAGGAGACGGUUAAAG | 20 | 5924 |
| HSV1-UL48-118 | + | CAAACAGCUCGUCGACCAAG | 20 | 5958 |
| HSV1-UL48-95 | + | AGGCGCCCCGUUGCGUACAG | 20 | 6018 |
| HSV1-UL48-12 | - | CCCCCCCGCUGUACGCAACG | 20 | 5842 |
| HSV1-UL48-19 | - | CAUGCUCGAUACCUGGAACG | 20 | 5858 |
| HSV1-UL48-31 | - | CAUUCGCGCCCACGGCGACG | 20 | 5872 |
| HSV1-UL48-51 | + | UAGGGAAGGCCACGUCGCCG | 20 | 5898 |
| HSV1-UL48-68 | + | AUCGAGCAUGGUACAUAGCG | 20 | 5912 |
| HSV1-UL48-16 | - | CGACGACUUGGGCUUUAGCG | 20 | 5846 |
| HSV1-UL48-70 | + | CGAGCAUGGUACAUAGCGCG | 20 | 5914 |
| HSV1-UL48-42 | + | CCCGCGCCCGUAGCUCGGCG | 20 | 5988 |
| HSV1-UL48-38 | - | UCCACGCCGAGCUACGGGCG | 20 | 5879 |
| HSV1-UL48-44 | + | AGUAGAGCCCGAGGCCGUCG | 20 | 5989 |
| HSV1-UL48-71 | + | CGCUAAAGCCCAAGUCGUCG | 20 | 5915 |
| HSV1-UL48-57 | + | UGUCGAUUUGGGUGCGUUCG | 20 | 5996 |
| HSV1-UL48-79 | + | ACGGUUAAAGAGGGCGGCGG | 20 | 5931 |
| HSV1-UL48-40 | - | CGCCGAGCUACGGGCGCGGG | 20 | 5973 |
| HSV1-UL48-80 | + | CGGUUAAAGAGGGCGGCGGG | 20 | 5932 |
| HSV1-UL48-100 | + | CCCCGUUGCGUACAGCGGGG | 20 | 5941 |
| HSV1-UL48-1134 | + | CGACCAAGAGGUCCAUUGGG | 20 | 16543 |
| HSV1-UL48-28 | - | CCAGCGAUGUGGUGGAAUGG | 20 | 5970 |
| HSV1-UL48-24 | - | AACGCUGCCCAGCGAUGUGG | 20 | 5863 |
| HSV1-UL48-23 | - | AUCAACGCUGCCCAGCGAUG | 20 | 5862 |
| HSV1-UL48-1135 | + | ACCAAGAGGUCCAUUGGGUG | 20 | 16544 |
| HSV1-UL48-1136 | + | UCGUCGACCAAGAGGUCCAU | 20 | 16545 |
| HSV1-UL48-1137 | - | ACCCCACCCAAUGGACCUCU | 20 | 16546 |
| HSV1-UL48-48 | + | UCGCGGGUGGCCGGAAGCGU | 20 | 5896 |
| HSV1-UL48-64 | + | CUCCCGGUACAGGUCGGCGU | 20 | 5908 |
| HSV1-UL48-63 | + | UUUACACUCCCGGUACAGGU | 20 | 6001 |
| HSV1-UL48-65 | + | CGGUACAGGUCGGCGUUGGU | 20 | 5909 |
| HSV1-UL48-117 | + | AGCGCCGUCCGCGUUCAUGU | 20 | 6030 |
| HSV1-UL48-1138 | + | CGUCGACCAAGAGGUCCAUU | 20 | 16547 |
| HSV1-UL48-53 | + | CGUGGGCGCGAAUGUCGAUU | 20 | 5900 |
| HSV1-UL48-15 | - | AACCGUCUCCUCGACGACUU | 20 | 5845 |
| HSV1-UL48-55 | + | AAUGUCGAUUUGGGUGCGUU | 20 | 5994 |
| HSV1-UL48-20 | - | CUGGAACGAGGAUCUGUUUU | 20 | 5859 |

**Table 11C** provides exemplary targeting domains for knocking out the *UL48* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 11C**

| 3rd Tier | | | | |
|---|---|---|---|---|
| gRNA Name | DNA Strand | Targeting Domain | Target Site Length | Seq ID NO: |
| HSV1-UL48-1139 | - | GUAUCAACCCCACCCAA | 17 | 16548 |
| HSV1-UL48-607 | + | GCAGAAGUUGGCCAACA | 17 | 6445 |
| HSV1-UL48-185 | + | GUUCCAGGUAUCGAGCA | 17 | 5919 |
| HSV1-UL48-202 | + | GGCGGGGGGGACGGGCA | 17 | 6050 |
| HSV1-UL48-192 | + | GAGGAGACGGUUAAAGA | 17 | 5927 |
| HSV1-UL48-168 | + | GGCCGGAAGCGUAGGGA | 17 | 6036 |
| HSV1-UL48-200 | + | GAGGGCGGCGGGGGGGA | 17 | 6048 |
| HSV1-UL48-136 | - | GUACCAUGCUCGAUACC | 17 | 5981 |
| HSV1-UL48-126 | - | GGGUCCCAAAAACACCC | 17 | 5848 |
| HSV1-UL48-224 | + | GGUGUUUUUGGGACCCC | 17 | 6064 |
| HSV1-UL48-121 | - | GCCACCGCCCCCCCGCC | 17 | 5976 |
| HSV1-UL48-209 | + | GUGGGGAGGGCAUGAGC | 17 | 6057 |
| HSV1-UL48-135 | - | GACUUGGGCUUUAGCGC | 17 | 5857 |
| HSV1-UL48-163 | + | GAGCCCGAGGCCGUCGC | 17 | 5895 |
| HSV1-UL48-228 | + | GGGACCCCCGGCCGGGC | 17 | 6066 |
| HSV1-UL48-150 | - | GGCCUUCCCUACGCUUC | 17 | 5882 |
| HSV1-UL48-137 | - | GCUCGAUACCUGGAACG | 17 | 5865 |
| HSV1-UL48-169 | + | GGAAGGCCACGUCGCCG | 17 | 5902 |
| HSV1-UL48-124 | - | GCCCCCCCGCCCGGCCG | 17 | 5979 |
| HSV1-UL48-196 | + | GGUUAAAGAGGGCGGCG | 17 | 5935 |
| HSV1-UL48-229 | + | GGACCCCCGGCCGGGCG | 17 | 6067 |
| HSV1-UL48-197 | + | GUUAAAGAGGGCGGCGG | 17 | 5936 |
| HSV1-UL48-230 | + | GACCCCCGGCCGGGCGG | 17 | 6068 |
| HSV1-UL48-234 | + | GCCGGGCGGGGGGGCGG | 17 | 6072 |
| HSV1-UL48-193 | + | GAGACGGUUAAAGAGGG | 17 | 6046 |
| HSV1-UL48-207 | + | GACGGGCAUGGGUGGGG | 17 | 6055 |
| HSV1-UL48-204 | + | GGGGGGACGGGCAUGGG | 17 | 6052 |
| HSV1-UL48-146 | - | GCGAUGUGGUGGAAUGG | 17 | 5984 |
| HSV1-UL48-142 | - | GCUGCCCAGCGAUGUGG | 17 | 5870 |
| HSV1-UL48-206 | + | GGGGACGGGCAUGGGUG | 17 | 6054 |
| HSV1-UL48-203 | + | GCGGGGGGGACGGGCAU | 17 | 6051 |
| HSV1-UL48-205 | + | GGGGGACGGGCAUGGGU | 17 | 6053 |
| HSV1-UL48-138 | - | GAACGAGGAUCUGUUUU | 17 | 5866 |
| HSV1-UL48-222 | + | GGCCGCCGGGGUGUUUU | 17 | 5952 |
| HSV1-UL48-84 | + | GGCGGCGGGGGGGACGGGCA | 20 | 6007 |
| HSV1-UL48-74 | + | GUCGAGGAGACGGUUAAAGA | 20 | 5925 |
| HSV1-UL48-90 | + | GGGACGGGCAUGGGUGGGGA | 20 | 6013 |
| HSV1-UL48-22 | - | GACCAACGCCGACCUGUACC | 20 | 5861 |
| HSV1-UL48-93 | + | GGGAGGGCAUGAGCUGGGCC | 20 | 6016 |
| HSV1-UL48-17 | - | GACGACUUGGGCUUUAGCGC | 20 | 5847 |
| HSV1-UL48-94 | + | GCAUGAGCUGGGCCUGGCUC | 20 | 6017 |
| HSV1-UL48-43 | + | GCGCUUCGUAGUAGAGCCCG | 20 | 5891 |
| HSV1-UL48-103 | + | GUACAGCGGGGGGGCCGCCG | 20 | 5947 |
| HSV1-UL48-75 | + | GAGGAGACGGUUAAAGAGGG | 20 | 6003 |
| HSV1-UL48-46 | + | GAGCCCGAGGCCGUCGCGGG | 20 | 5990 |
| HSV1-UL48-113 | + | GGGACCCCCGGCCGGGCGGG | 20 | 6026 |
| HSV1-UL48-81 | + | GGUUAAAGAGGGCGGCGGGG | 20 | 6004 |
| HSV1-UL48-114 | + | GGACCCCCGGCCGGGCGGGG | 20 | 6027 |
| HSV1-UL48-89 | + | GGGGACGGGCAUGGGUGGGG | 20 | 6012 |
| HSV1-UL48-86 | + | GCGGGGGGGACGGGCAUGGG | 20 | 6009 |
| HSV1-UL48-88 | + | GGGGGGGACGGGCAUGGGUG | 20 | 6011 |
| HSV1-UL48-85 | + | GCGGCGGGGGGGACGGGCAU | 20 | 6008 |
| HSV1-UL48-92 | + | GGGUGGGGAGGGCAUGAGCU | 20 | 6015 |

**Table 11D** provides exemplary targeting domains for knocking out the *UL48* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 11D**

| 4th Tier | | | | |
|---|---|---|---|---|
| gRNA Name | DNA Strand | Targeting Domain | Target Site Length | Seq ID NO: |
| HSV1-UL48-143 | - | CCAGCGAUGUGGUGGAA | 17 | 5871 |
| HSV1-UL48-151 | - | CUUCCGGCCACCCGCGA | 17 | 5883 |
| HSV1-UL48-208 | + | ACGGGCAUGGGUGGGGA | 17 | 6056 |
| HSV1-UL48-1140 | + | AUUGGGUGGGGUUGAUA | 17 | 16549 |
| HSV1-UL48-154 | - | UCUUCCACGCCGAGCUA | 17 | 5886 |
| HSV1-UL48-201 | + | AGGGCGGCGGGGGGGAC | 17 | 6049 |
| HSV1-UL48-1141 | + | UUGGGUGGGGUUGAUAC | 17 | 16550 |
| HSV1-UL48-180 | + | AUUUACACUCCCGGUAC | 17 | 6043 |
| HSV1-UL48-131 | - | AACGGGGCGCCUGAGCC | 17 | 5853 |
| HSV1-UL48-220 | + | ACAGCGGGGGGGCCGCC | 17 | 5950 |
| HSV1-UL48-226 | + | UUUUGGGACCCCCGGCC | 17 | 5957 |
| HSV1-UL48-123 | - | CGCCCCCCCGCCCGGCC | 17 | 5978 |
| HSV1-UL48-211 | + | AGGGCAUGAGCUGGGCC | 17 | 6059 |
| HSV1-UL48-179 | + | AUAGGAAUUUACACUCC | 17 | 6042 |
| HSV1-UL48-184 | + | AAACAGAUCCUCGUUCC | 17 | 5918 |
| HSV1-UL48-219 | + | UACAGCGGGGGGGCCGC | 17 | 6063 |
| HSV1-UL48-157 | - | CGCCGAGCUACGGGCGC | 17 | 5889 |
| HSV1-UL48-176 | + | CCAUUCCACCACAUCGC | 17 | 5907 |
| HSV1-UL48-225 | + | UUUUUGGGACCCCCGGC | 17 | 5955 |
| HSV1-UL48-122 | - | CCGCCCCCCCGCCCGGC | 17 | 5977 |
| HSV1-UL48-165 | + | AGGCCGUCGCGGGUGGC | 17 | 6034 |
| HSV1-UL48-153 | - | CCACCCGCGACGGCCUC | 17 | 5885 |
| HSV1-UL48-212 | + | UGAGCUGGGCCUGGCUC | 17 | 6060 |
| HSV1-UL48-149 | - | UCGCGCCCACGGCGACG | 17 | 5881 |
| HSV1-UL48-221 | + | CAGCGGGGGGGCCGCCG | 17 | 5951 |
| HSV1-UL48-127 | - | UCCCAAAAACACCCCGG | 17 | 5849 |
| HSV1-UL48-125 | - | CCCCCCCGCCCGGCCGG | 17 | 5980 |
| HSV1-UL48-227 | + | UGGGACCCCCGGCCGGG | 17 | 6065 |
| HSV1-UL48-164 | + | CCCGAGGCCGUCGCGGG | 17 | 6033 |
| HSV1-UL48-198 | + | UUAAAGAGGGCGGCGGG | 17 | 5937 |
| HSV1-UL48-231 | + | ACCCCCGGCCGGGCGGG | 17 | 6069 |
| HSV1-UL48-199 | + | UAAAGAGGGCGGCGGGG | 17 | 6047 |
| HSV1-UL48-232 | + | CCCCCGGCCGGGCGGGG | 17 | 6070 |
| HSV1-UL48-233 | + | CCGGCCGGGCGGGGGGG | 17 | 6071 |
| HSV1-UL48-147 | - | CGAUGUGGUGGAAUGGG | 17 | 5985 |
| HSV1-UL48-1142 | + | CCAAGAGGUCCAUUGGG | 17 | 16551 |
| HSV1-UL48-145 | - | AGCGAUGUGGUGGAAUG | 17 | 5983 |
| HSV1-UL48-141 | - | AACGCUGCCCAGCGAUG | 17 | 5869 |
| HSV1-UL48-1143 | + | AAGAGGUCCAUUGGGUG | 17 | 16552 |
| HSV1-UL48-144 | - | CAGCGAUGUGGUGGAAU | 17 | 5982 |
| HSV1-UL48-178 | + | CGCUGGGCAGCGUUGAU | 17 | 6041 |
| HSV1-UL48-210 | + | UGGGGAGGGCAUGAGCU | 17 | 6058 |
| HSV1-UL48-177 | + | CAUUCCACCACAUCGCU | 17 | 6040 |
| HSV1-UL48-1144 | - | CCACCCAAUGGACCUCU | 17 | 16553 |
| HSV1-UL48-166 | + | CGGGUGGCCGGAAGCGU | 17 | 5897 |
| HSV1-UL48-181 | + | ACACUCCCGGUACAGGU | 17 | 6044 |
| HSV1-UL48-1145 | + | CAAGAGGUCCAUUGGGU | 17 | 16554 |
| HSV1-UL48-25 | - | UGCCCAGCGAUGUGGUGGAA | 20 | 5864 |
| HSV1-UL48-82 | + | AAAGAGGGCGGCGGGGGGGA | 20 | 6005 |
| HSV1-UL48-83 | + | AAGAGGGCGGCGGGGGGGAC | 20 | 6006 |
| HSV1-UL48-1146 | + | CCAUUGGGUGGGGUUGAUAC | 20 | 16555 |
| HSV1-UL48-18 | - | UAUGUACCAUGCUCGAUACC | 20 | 5967 |
| HSV1-UL48-106 | + | CGGGGUGUUUUUGGGACCCC | 20 | 6021 |
| HSV1-UL48-13 | - | CGCAACGGGGCGCCUGAGCC | 20 | 5843 |
| HSV1-UL48-3 | - | UUCGCCACCGCCCCCCCGCC | 20 | 5962 |
| HSV1-UL48-5 | - | CACCGCCCCCCCGCCCGGCC | 20 | 5964 |
| HSV1-UL48-61 | + | UUGAUAGGAAUUUACACUCC | 20 | 5999 |
| HSV1-UL48-91 | + | UGGGUGGGGAGGGCAUGAGC | 20 | 6014 |
| HSV1-UL48-4 | - | CCACCGCCCCCCCGCCCGGC | 20 | 5963 |
| HSV1-UL48-6 | - | ACCGCCCCCCCGCCCGGCCG | 20 | 5965 |
| HSV1-UL48-111 | + | UUGGGACCCCCGGCCGGGCG | 20 | 6024 |
| HSV1-UL48-7 | - | CCGCCCCCCCGCCCGGCCGG | 20 | 5966 |
| HSV1-UL48-98 | + | CGCCCCGUUGCGUACAGCGG | 20 | 5939 |
| HSV1-UL48-112 | + | UGGGACCCCCGGCCGGGCGG | 20 | 6025 |
| HSV1-UL48-116 | + | CCGGCCGGGCGGGGGGGCGG | 20 | 6029 |
| HSV1-UL48-109 | + | UUUUGGGACCCCCGGCCGGG | 20 | 6022 |
| HSV1-UL48-115 | + | CCCCCGGCCGGGCGGGGGGG | 20 | 6028 |
| HSV1-UL48-29 | - | CAGCGAUGUGGUGGAAUGGG | 20 | 5971 |
| HSV1-UL48-27 | - | CCCAGCGAUGUGGUGGAAUG | 20 | 5969 |
| HSV1-UL48-60 | + | CAUCGCUGGGCAGCGUUGAU | 20 | 5998 |
| HSV1-UL48-14 | - | UAACCGUCUCCUCGACGACU | 20 | 5844 |
| HSV1-UL48-34 | - | CCGGCCACCCGCGACGGCCU | 20 | 5875 |
| HSV1-UL48-41 | + | CUCCUCCCGCGCCCGUAGCU | 20 | 5890 |
| HSV1-UL48-59 | + | CCCCAUUCCACCACAUCGCU | 20 | 5997 |
| HSV1-UL48-52 | + | AGGGAAGGCCACGUCGCCGU | 20 | 5899 |
| HSV1-UL48-87 | + | CGGGGGGGACGGGCAUGGGU | 20 | 6010 |

**Table 11E** provides exemplary targeting domains for knocking out the *UL48* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a *S*. *pyogenes* Cas9 molecule that generates a double stranded break (Cas9 nuclease) or a single-stranded break (Cas9 nickase).

**Table 11E**

| 5th Tier | | | | |
|---|---|---|---|---|
| gRNA Name | DNA Strand | Targeting Domain | Target Site Length | Seq ID NO: |
| HSV1-UL48-510 | + | GUACUCGUCAAUUCCAA | 17 | 6348 |
| HSV1-UL48-568 | + | GUGGGAGGGGUCGUCAA | 17 | 6406 |
| HSV1-UL48-440 | - | CCCUUCAUGUUCGUCAA | 17 | 6278 |
| HSV1-UL48-540 | + | GUGUCCCGCGGGGAGAA | 17 | 6378 |
| HSV1-UL48-582 | + | CCGUUGACGAACAUGAA | 17 | 6420 |
| HSV1-UL48-607 | + | GCAGAAGUUGGCCAACA | 17 | 6445 |
| HSV1-UL48-605 | + | GCGCAGGUACCGGUACA | 17 | 6443 |
| HSV1-UL48-453 | - | CGGCUACGGAGGAGCCA | 17 | 6291 |
| HSV1-UL48-509 | + | CGUACUCGUCAAUUCCA | 17 | 6347 |
| HSV1-UL48-562 | + | GGCCUGGUUGCCAUGCA | 17 | 6400 |
| HSV1-UL48-455 | - | ACCCCUCCCACCCUGCA | 17 | 6293 |
| HSV1-UL48-485 | - | GACGAGCUCCACUUAGA | 17 | 6323 |
| HSV1-UL48-488 | - | CGGCGAGGACGUGGCGA | 17 | 6326 |
| HSV1-UL48-470 | - | UACGGGUCUACCAUCGA | 17 | 6308 |
| HSV1-UL48-550 | + | AUCGAGCAGGCCCUCGA | 17 | 6388 |
| HSV1-UL48-480 | - | CACGCGCAGACUGUCGA | 17 | 6318 |
| HSV1-UL48-580 | + | GAUUGGCACUCCCCGGA | 17 | 6418 |
| HSV1-UL48-554 | + | CGCAUGACCGCCUCGGA | 17 | 6392 |
| HSV1-UL48-494 | - | CUGGACAUGUUGGGGGA | 17 | 6332 |
| HSV1-UL48-566 | + | UGCCAUGCAGGGUGGGA | 17 | 6404 |
| HSV1-UL48-581 | + | UCCGUUGACGAACAUGA | 17 | 6419 |
| HSV1-UL48-504 | - | CUACGGCGCUCUGGAUA | 17 | 6342 |
| HSV1-UL48-502 | - | CACGACUCCGCCCCCUA | 17 | 6340 |
| HSV1-UL48-432 | - | UGACCCGCGAGAUCCUA | 17 | 6270 |
| HSV1-UL48-450 | - | GGUGCGCAGCGCGGCUA | 17 | 6288 |
| HSV1-UL48-506 | - | CUUGGAAUUGACGAGUA | 17 | 6344 |
| HSV1-UL48-514 | + | AUAUCCAGAGCGCCGUA | 17 | 6352 |
| HSV1-UL48-1147 | - | GACGAGUACGGUGGGUA | 17 | 16556 |
| HSV1-UL48-467 | - | CGUACGAAAAACAAUUA | 17 | 6305 |
| HSV1-UL48-573 | + | GCACCAGCGGGAGGUUA | 17 | 6411 |
| HSV1-UL48-459 | - | CGCCUCUGGGUACUUUA | 17 | 6297 |
| HSV1-UL48-586 | + | AACAGAGGCAGUCAAAC | 17 | 6424 |
| HSV1-UL48-1148 | - | UGGGUAGGGGGCGCGAC | 17 | 16557 |
| HSV1-UL48-541 | + | CCGCGGGGAGAAAGGAC | 17 | 6379 |
| HSV1-UL48-430 | - | GCGCCACGAUCGCGGAC | 17 | 6268 |
| HSV1-UL48-495 | - | UGGACAUGUUGGGGGAC | 17 | 6333 |
| HSV1-UL48-603 | + | CGCUGGCGCGCAGGUAC | 17 | 6441 |
| HSV1-UL48-604 | + | CGCGCAGGUACCGGUAC | 17 | 6442 |
| HSV1-UL48-423 | - | UCUGCUCGGCCCUGUAC | 17 | 6261 |
| HSV1-UL48-468 | - | GUACGAAAAACAAUUAC | 17 | 6306 |
| HSV1-UL48-456 | - | CACCCUGCAUGGCAACC | 17 | 6294 |
| HSV1-UL48-427 | - | CGGACGCGAUCGCGACC | 17 | 6265 |
| HSV1-UL48-435 | - | CUGCCUCUGUUGCGACC | 17 | 6273 |
| HSV1-UL48-529 | + | AGUGGAGCUCGUCCCCC | 17 | 6367 |
| HSV1-UL48-569 | + | GUCGUCAACGGCGCCCC | 17 | 6407 |
| HSV1-UL48-525 | + | GGGUAAAUCCCGGCCCC | 17 | 6363 |
| HSV1-UL48-498 | - | GGGGACGGGGAUUCCCC | 17 | 6336 |
| HSV1-UL48-524 | + | GGGGUAAAUCCCGGCCC | 17 | 6362 |
| HSV1-UL48-579 | + | CCUCGAUUGGCACUCCC | 17 | 6417 |
| HSV1-UL48-597 | + | CGCGCAGCAUUUCUCCC | 17 | 6435 |
| HSV1-UL48-497 | - | GGGGGACGGGGAUUCCC | 17 | 6335 |
| HSV1-UL48-481 | - | CCCGACCGAUGUCAGCC | 17 | 6319 |
| HSV1-UL48-452 | - | GCGGCUACGGAGGAGCC | 17 | 6290 |
| HSV1-UL48-425 | - | CCGGCAGCUGCACCGCC | 17 | 6263 |
| HSV1-UL48-598 | + | GUCCGCGCAUGUGCGCC | 17 | 6436 |
| HSV1-UL48-560 | + | ACCCAGAGGCGCGGGCC | 17 | 6398 |
| HSV1-UL48-501 | - | GGGGAUUCCCCGGGGCC | 17 | 6339 |
| HSV1-UL48-523 | + | UCGUGGGGGGUAAAUCC | 17 | 6361 |
| HSV1-UL48-471 | - | GGGCCUGCUCGAUCUCC | 17 | 6309 |
| HSV1-UL48-584 | + | ACUGACGCCAGCUCUCC | 17 | 6422 |
| HSV1-UL48-442 | - | CGGAGCGCUCACCGUCC | 17 | 6280 |
| HSV1-UL48-548 | + | CGGGGGCGUCGUCGUCC | 17 | 6386 |
| HSV1-UL48-556 | + | GGGCGAGGUCGUGAAGC | 17 | 6394 |
| HSV1-UL48-571 | + | CCGCGCUGCGCACCAGC | 17 | 6409 |
| HSV1-UL48-575 | + | GGUUUAGCUCCCGCAGC | 17 | 6413 |
| HSV1-UL48-436 | - | GUUGCGACCUGGAGAGC | 17 | 6274 |
| HSV1-UL48-549 | + | CGUCCGGGAGAUCGAGC | 17 | 6387 |
| HSV1-UL48-601 | + | GUGCAGCUGCCGGACGC | 17 | 6439 |
| HSV1-UL48-479 | - | CUGUCCUUUCUCCCCGC | 17 | 6317 |
| HSV1-UL48-445 | - | UGCCAAUCGAGGCCCGC | 17 | 6283 |
| HSV1-UL48-448 | - | GCACCUUAACCUCCCGC | 17 | 6286 |
| HSV1-UL48-538 | + | CUGCGCGUGUGUCCCGC | 17 | 6376 |
| HSV1-UL48-602 | + | GCCGGACGCUGGCGCGC | 17 | 6440 |
| HSV1-UL48-559 | + | AAAGUACCCAGAGGCGC | 17 | 6397 |
| HSV1-UL48-157 | - | CGCCGAGCUACGGGCGC | 17 | 5889 |
| HSV1-UL48-593 | + | GGCCCAUAGGAUCUCGC | 17 | 6431 |
| HSV1-UL48-474 | - | GACGCCCCCGAAGAGGC | 17 | 6312 |
| HSV1-UL48-577 | + | UAGCUCCCGCAGCCGGC | 17 | 6415 |
| HSV1-UL48-583 | + | UGACGAACAUGAAGGGC | 17 | 6421 |
| HSV1-UL48-500 | - | CGGGGAUUCCCCGGGGC | 17 | 6338 |
| HSV1-UL48-476 | - | CCCCGAAGAGGCGGGGC | 17 | 6314 |
| HSV1-UL48-439 | - | AGCUGGCGUCAGUUGGC | 17 | 6277 |
| HSV1-UL48-561 | + | GGGCCUGGUUGCCAUGC | 17 | 6399 |
| HSV1-UL48-466 | - | CUCCGAGGCGGUCAUGC | 17 | 6304 |
| HSV1-UL48-600 | + | CCUGGCGGUGCAGCUGC | 17 | 6438 |
| HSV1-UL48-447 | - | CGAGGCCCGCCGGCUGC | 17 | 6285 |
| HSV1-UL48-489 | - | GCUAGACGAUUUCGAUC | 17 | 6327 |
| HSV1-UL48-457 | - | AACCAGGCCCGCGCCUC | 17 | 6295 |
| HSV1-UL48-503 | - | CGCCCCCUACGGCGCUC | 17 | 6341 |
| HSV1-UL48-441 | - | ACGGAGCGCUCACCGUC | 17 | 6279 |
| HSV1-UL48-424 | - | ACCUGCGCGCCAGCGUC | 17 | 6262 |
| HSV1-UL48-431 | - | CCGAGAGACCGCUCGUC | 17 | 6269 |
| HSV1-UL48-547 | + | UCGGGGGCGUCGUCGUC | 17 | 6385 |
| HSV1-UL48-588 | + | AGGCAGUCAAACAGGUC | 17 | 6426 |
| HSV1-UL48-532 | + | CCAGGCUGACAUCGGUC | 17 | 6370 |
| HSV1-UL48-1149 | + | GACGGGGGAUGCGGGUC | 17 | 16558 |
| HSV1-UL48-461 | - | CUUUAUGGUGUUGAUUC | 17 | 6299 |
| HSV1-UL48-544 | + | GCCAGCCCCGCCUCUUC | 17 | 6382 |
| HSV1-UL48-472 | - | CGACGACGCCCCCGAAG | 17 | 6310 |
| HSV1-UL48-528 | + | CGUCCUCGCCGUCUAAG | 17 | 6366 |
| HSV1-UL48-585 | + | UCUCCAGGUCGCAACAG | 17 | 6423 |
| HSV1-UL48-570 | + | GCCGCGCUGCGCACCAG | 17 | 6408 |
| HSV1-UL48-557 | + | CACCAUAAAGUACCCAG | 17 | 6395 |
| HSV1-UL48-454 | - | GGCUACGGAGGAGCCAG | 17 | 6292 |
| HSV1-UL48-594 | + | AACACGCGCCAGACGAG | 17 | 6432 |
| HSV1-UL48-567 | + | GCCAUGCAGGGUGGGAG | 17 | 6405 |
| HSV1-UL48-515 | + | UAUCCAGAGCGCCGUAG | 17 | 6353 |
| HSV1-UL48-1150 | - | ACGAGUACGGUGGGUAG | 17 | 16559 |
| HSV1-UL48-487 | - | CUUAGACGGCGAGGACG | 17 | 6325 |
| HSV1-UL48-496 | - | GGACAUGUUGGGGGACG | 17 | 6334 |
| HSV1-UL48-590 | + | CAUCUGCUCGGCGUACG | 17 | 6428 |
| HSV1-UL48-526 | + | GGUAAAUCCCGGCCCCG | 17 | 6364 |
| HSV1-UL48-478 | - | CCUGUCCUUUCUCCCCG | 17 | 6316 |
| HSV1-UL48-499 | - | GGGACGGGGAUUCCCCG | 17 | 6337 |
| HSV1-UL48-537 | + | UCUGCGCGUGUGUCCCG | 17 | 6375 |
| HSV1-UL48-463 | - | CACGACCUCGCCCUCCG | 17 | 6301 |
| HSV1-UL48-443 | - | GGAGCGCUCACCGUCCG | 17 | 6281 |
| HSV1-UL48-449 | - | CCCGCUGGUGCGCAGCG | 17 | 6287 |
| HSV1-UL48-539 | + | UGCGCGUGUGUCCCGCG | 17 | 6377 |
| HSV1-UL48-551 | + | UGUUUUUCGUACGCGCG | 17 | 6389 |
| HSV1-UL48-542 | + | GGGAGAAAGGACAGGCG | 17 | 6380 |
| HSV1-UL48-475 | - | ACGCCCCCGAAGAGGCG | 17 | 6313 |
| HSV1-UL48-558 | + | UAAAGUACCCAGAGGCG | 17 | 6396 |
| HSV1-UL48-486 | - | GCUCCACUUAGACGGCG | 17 | 6324 |
| HSV1-UL48-160 | + | GCGCCCGUAGCUCGGCG | 17 | 6031 |
| HSV1-UL48-555 | + | GACCGCCUCGGAGGGCG | 17 | 6393 |
| HSV1-UL48-156 | - | ACGCCGAGCUACGGGCG | 17 | 5888 |
| HSV1-UL48-426 | - | CGCCAGGCGCACAUGCG | 17 | 6264 |
| HSV1-UL48-444 | - | CCGGGGAGUGCCAAUCG | 17 | 6282 |
| HSV1-UL48-469 | - | UUACGGGUCUACCAUCG | 17 | 6307 |
| HSV1-UL48-429 | - | GCUGCGCGCCACGAUCG | 17 | 6267 |
| HSV1-UL48-596 | + | GUACCUGUCCGCGAUCG | 17 | 6434 |
| HSV1-UL48-592 | + | CGGCCCAUAGGAUCUCG | 17 | 6430 |
| HSV1-UL48-518 | + | CGUAGGGGGCGGAGUCG | 17 | 6356 |
| HSV1-UL48-533 | + | CAGGCUGACAUCGGUCG | 17 | 6371 |
| HSV1-UL48-545 | + | CCAGCCCCGCCUCUUCG | 17 | 6383 |
| HSV1-UL48-473 | - | CGACGCCCCCGAAGAGG | 17 | 6311 |
| HSV1-UL48-464 | - | GACCUCGCCCUCCGAGG | 17 | 6302 |
| HSV1-UL48-516 | + | AUCCAGAGCGCCGUAGG | 17 | 6354 |
| HSV1-UL48-1151 | - | CGAGUACGGUGGGUAGG | 17 | 16560 |
| HSV1-UL48-451 | - | GCGCAGCGCGGCUACGG | 17 | 6289 |
| HSV1-UL48-507 | - | GGAAUUGACGAGUACGG | 17 | 6345 |
| HSV1-UL48-576 | + | UUAGCUCCCGCAGCCGG | 17 | 6414 |
| HSV1-UL48-553 | + | CCGCAUGACCGCCUCGG | 17 | 6391 |
| HSV1-UL48-534 | + | AGGCUGACAUCGGUCGG | 17 | 6372 |
| HSV1-UL48-546 | + | CAGCCCCGCCUCUUCGG | 17 | 6384 |
| HSV1-UL48-563 | + | CUGGUUGCCAUGCAGGG | 17 | 6401 |
| HSV1-UL48-572 | + | CGCUGCGCACCAGCGGG | 17 | 6410 |
| HSV1-UL48-158 | - | CGAGCUACGGGCGCGGG | 17 | 5987 |
| HSV1-UL48-535 | + | GGCUGACAUCGGUCGGG | 17 | 6373 |
| HSV1-UL48-536 | + | GCUGACAUCGGUCGGGG | 17 | 6374 |
| HSV1-UL48-517 | + | CAGAGCGCCGUAGGGGG | 17 | 6355 |
| HSV1-UL48-522 | + | GGGGGCGGAGUCGUGGG | 17 | 6360 |
| HSV1-UL48-565 | + | UUGCCAUGCAGGGUGGG | 17 | 6403 |
| HSV1-UL48-484 | - | GACCGAUGUCAGCCUGG | 17 | 6322 |
| HSV1-UL48-599 | + | CGCGCAUGUGCGCCUGG | 17 | 6437 |
| HSV1-UL48-477 | - | CGAAGAGGCGGGGCUGG | 17 | 6315 |
| HSV1-UL48-521 | + | AGGGGGCGGAGUCGUGG | 17 | 6359 |
| HSV1-UL48-438 | - | GAGCUGGCGUCAGUUGG | 17 | 6276 |
| HSV1-UL48-493 | - | CGAUCUGGACAUGUUGG | 17 | 6331 |
| HSV1-UL48-574 | + | UAAGGUGCUCGCGAAUG | 17 | 6412 |
| HSV1-UL48-465 | - | CCUCCGAGGCGGUCAUG | 17 | 6303 |
| HSV1-UL48-434 | - | ACGCCGAGCAGAUGAUG | 17 | 6272 |
| HSV1-UL48-483 | - | CGACCGAUGUCAGCCUG | 17 | 6321 |
| HSV1-UL48-446 | - | UCGAGGCCCGCCGGCUG | 17 | 6284 |
| HSV1-UL48-520 | + | UAGGGGGCGGAGUCGUG | 17 | 6358 |
| HSV1-UL48-492 | - | UCGAUCUGGACAUGUUG | 17 | 6330 |
| HSV1-UL48-530 | + | GUCCCCCAGGCUGACAU | 17 | 6368 |
| HSV1-UL48-591 | + | CGGCGUACGCGGCCCAU | 17 | 6429 |
| HSV1-UL48-511 | + | GUCAAUUCCAAGGGCAU | 17 | 6349 |
| HSV1-UL48-578 | + | AGCCGGCGGGCCUCGAU | 17 | 6416 |
| HSV1-UL48-433 | - | GACCCGCGAGAUCCUAU | 17 | 6271 |
| HSV1-UL48-428 | - | GGACGCGAUCGCGACCU | 17 | 6266 |
| HSV1-UL48-505 | - | AUGUUUACCGAUGCCCU | 17 | 6343 |
| HSV1-UL48-482 | - | CCGACCGAUGUCAGCCU | 17 | 6320 |
| HSV1-UL48-552 | + | UUCCCGCAUGACCGCCU | 17 | 6390 |
| HSV1-UL48-159 | + | CUCCCGCGCCCGUAGCU | 17 | 5893 |
| HSV1-UL48-589 | + | GGGCCGCAUCAUCUGCU | 17 | 6427 |
| HSV1-UL48-422 | - | GUUGGCCAACUUCUGCU | 17 | 6260 |
| HSV1-UL48-458 | - | ACCAGGCCCGCGCCUCU | 17 | 6296 |
| HSV1-UL48-595 | + | CCAGACGAGCGGUCUCU | 17 | 6433 |
| HSV1-UL48-606 | + | CAGGGCCGAGCAGAAGU | 17 | 6444 |
| HSV1-UL48-462 | - | GUUGAUUCGGGCGAAGU | 17 | 6300 |
| HSV1-UL48-512 | + | CUGCUCAAACUCGAAGU | 17 | 6350 |
| HSV1-UL48-437 | - | GGAGAGCUGGCGUCAGU | 17 | 6275 |
| HSV1-UL48-513 | + | CAUAUCCAGAGCGCCGU | 17 | 6351 |
| HSV1-UL48-527 | + | GAAAUCGUCUAGCGCGU | 17 | 6365 |
| HSV1-UL48-519 | + | GUAGGGGGCGGAGUCGU | 17 | 6357 |
| HSV1-UL48-587 | + | GAGGCAGUCAAACAGGU | 17 | 6425 |
| HSV1-UL48-508 | - | GAAUUGACGAGUACGGU | 17 | 6346 |
| HSV1-UL48-531 | + | CCCAGGCUGACAUCGGU | 17 | 6369 |
| HSV1-UL48-564 | + | UGGUUGCCAUGCAGGGU | 17 | 6402 |
| HSV1-UL48-1152 | - | UGACGAGUACGGUGGGU | 17 | 16561 |
| HSV1-UL48-490 | - | UUUCGAUCUGGACAUGU | 17 | 6328 |
| HSV1-UL48-1127 | - | GAGCUAUCGAACCGUGU | 17 | 16536 |
| HSV1-UL48-460 | - | ACUUUAUGGUGUUGAUU | 17 | 6298 |
| HSV1-UL48-543 | + | CGCCAGCCCCGCCUCUU | 17 | 6381 |
| HSV1-UL48-491 | - | UUCGAUCUGGACAUGUU | 17 | 6329 |
| HSV1-UL48-324 | + | ACCGUACUCGUCAAUUCCAA | 20 | 6162 |
| HSV1-UL48-382 | + | AGGGUGGGAGGGGUCGUCAA | 20 | 6220 |
| HSV1-UL48-254 | - | CAGCCCUUCAUGUUCGUCAA | 20 | 6092 |
| HSV1-UL48-354 | + | CGUGUGUCCCGCGGGGAGAA | 20 | 6192 |
| HSV1-UL48-396 | + | GCUCCGUUGACGAACAUGAA | 20 | 6234 |
| HSV1-UL48-421 | + | CGAGCAGAAGUUGGCCAACA | 20 | 6259 |
| HSV1-UL48-419 | + | GGCGCGCAGGUACCGGUACA | 20 | 6257 |
| HSV1-UL48-267 | - | GCGCGGCUACGGAGGAGCCA | 20 | 6105 |
| HSV1-UL48-323 | + | CACCGUACUCGUCAAUUCCA | 20 | 6161 |
| HSV1-UL48-376 | + | GCGGGCCUGGUUGCCAUGCA | 20 | 6214 |
| HSV1-UL48-269 | - | ACGACCCCUCCCACCCUGCA | 20 | 6107 |
| HSV1-UL48-299 | - | GGGGACGAGCUCCACUUAGA | 20 | 6137 |
| HSV1-UL48-302 | - | AGACGGCGAGGACGUGGCGA | 20 | 6140 |
| HSV1-UL48-284 | - | AAUUACGGGUCUACCAUCGA | 20 | 6122 |
| HSV1-UL48-364 | + | GAGAUCGAGCAGGCCCUCGA | 20 | 6202 |
| HSV1-UL48-294 | - | ACACACGCGCAGACUGUCGA | 20 | 6132 |
| HSV1-UL48-394 | + | CUCGAUUGGCACUCCCCGGA | 20 | 6232 |
| HSV1-UL48-368 | + | UCCCGCAUGACCGCCUCGGA | 20 | 6206 |
| HSV1-UL48-308 | - | GAUCUGGACAUGUUGGGGGA | 20 | 6146 |
| HSV1-UL48-380 | + | GGUUGCCAUGCAGGGUGGGA | 20 | 6218 |
| HSV1-UL48-395 | + | CGCUCCGUUGACGAACAUGA | 20 | 6233 |
| HSV1-UL48-318 | - | CCCCUACGGCGCUCUGGAUA | 20 | 6156 |
| HSV1-UL48-316 | - | CCCCACGACUCCGCCCCCUA | 20 | 6154 |
| HSV1-UL48-246 | - | UUUUGACCCGCGAGAUCCUA | 20 | 6084 |
| HSV1-UL48-264 | - | GCUGGUGCGCAGCGCGGCUA | 20 | 6102 |
| HSV1-UL48-320 | - | GCCCUUGGAAUUGACGAGUA | 20 | 6158 |
| HSV1-UL48-328 | + | GCCAUAUCCAGAGCGCCGUA | 20 | 6166 |
| HSV1-UL48-1153 | - | AUUGACGAGUACGGUGGGUA | 20 | 16562 |
| HSV1-UL48-281 | - | GC GCGUAC GAAAAACAAUUA | 20 | 6119 |
| HSV1-UL48-387 | + | UGCGCACCAGCGGGAGGUUA | 20 | 6225 |
| HSV1-UL48-273 | - | CCGCGCCUCUGGGUACUUUA | 20 | 6111 |
| HSV1-UL48-400 | + | CGCAACAGAGGCAGUCAAAC | 20 | 6238 |
| HSV1-UL48-1154 | - | CGGUGGGUAGGGGGCGCGAC | 20 | 16563 |
| HSV1-UL48-355 | + | GUCCCGCGGGGAGAAAGGAC | 20 | 6193 |
| HSV1-UL48-244 | - | UGCGCGCCACGAUCGCGGAC | 20 | 6082 |
| HSV1-UL48-309 | - | AUCUGGACAUGUUGGGGGAC | 20 | 6147 |
| HSV1-UL48-417 | + | GGACGCUGGCGCGCAGGUAC | 20 | 6255 |
| HSV1-UL48-418 | + | UGGCGCGCAGGUACCGGUAC | 20 | 6256 |
| HSV1-UL48-237 | - | ACUUCUGCUCGGCCCUGUAC | 20 | 6075 |
| HSV1-UL48-282 | - | CGCGUACGAAAAACAAUUAC | 20 | 6120 |
| HSV1-UL48-270 | - | UCCCACCCUGCAUGGCAACC | 20 | 6108 |
| HSV1-UL48-241 | - | GCGCGGACGCGAUCGCGACC | 20 | 6079 |
| HSV1-UL48-249 | - | UGACUGCCUCUGUUGCGACC | 20 | 6087 |
| HSV1-UL48-343 | + | CUAAGUGGAGCUCGUCCCCC | 20 | 6181 |
| HSV1-UL48-383 | + | GGGGUCGUCAACGGCGCCCC | 20 | 6221 |
| HSV1-UL48-339 | + | GGGGGGUAAAUCCCGGCCCC | 20 | 6177 |
| HSV1-UL48-312 | - | UUGGGGGACGGGGAUUCCCC | 20 | 6150 |
| HSV1-UL48-338 | + | UGGGGGGUAAAUCCCGGCCC | 20 | 6176 |
| HSV1-UL48-393 | + | GGGCCUCGAUUGGCACUCCC | 20 | 6231 |
| HSV1-UL48-411 | + | UGGCGCGCAGCAUUUCUCCC | 20 | 6249 |
| HSV1-UL48-311 | - | GUUGGGGGACGGGGAUUCCC | 20 | 6149 |
| HSV1-UL48-295 | - | CCCCCCGACCGAUGUCAGCC | 20 | 6133 |
| HSV1-UL48-266 | - | AGCGCGGCUACGGAGGAGCC | 20 | 6104 |
| HSV1-UL48-239 | - | CGUCCGGCAGCUGCACCGCC | 20 | 6077 |
| HSV1-UL48-412 | + | CGCGUCCGCGCAUGUGCGCC | 20 | 6250 |
| HSV1-UL48-374 | + | AGUACCCAGAGGCGCGGGCC | 20 | 6212 |
| HSV1-UL48-315 | - | GACGGGGAUUCCCCGGGGCC | 20 | 6153 |
| HSV1-UL48-337 | + | GAGUCGUGGGGGGUAAAUCC | 20 | 6175 |
| HSV1-UL48-285 | - | CGAGGGCCUGCUCGAUCUCC | 20 | 6123 |
| HSV1-UL48-398 | + | CCAACUGACGCCAGCUCUCC | 20 | 6236 |
| HSV1-UL48-256 | - | CAACGGAGCGCUCACCGUCC | 20 | 6094 |
| HSV1-UL48-362 | + | CUUCGGGGGCGUCGUCGUCC | 20 | 6200 |
| HSV1-UL48-370 | + | GGAGGGCGAGGUCGUGAAGC | 20 | 6208 |
| HSV1-UL48-385 | + | UAGCCGCGCUGCGCACCAGC | 20 | 6223 |
| HSV1-UL48-389 | + | UGUGGUUUAGCUCCCGCAGC | 20 | 6227 |
| HSV1-UL48-250 | - | UCUGUUGCGACCUGGAGAGC | 20 | 6088 |
| HSV1-UL48-363 | + | CGUCGUCCGGGAGAUCGAGC | 20 | 6201 |
| HSV1-UL48-415 | + | GCGGUGCAGCUGCCGGACGC | 20 | 6253 |
| HSV1-UL48-293 | - | CGCCUGUCCUUUCUCCCCGC | 20 | 6131 |
| HSV1-UL48-259 | - | GAGUGCCAAUCGAGGCCCGC | 20 | 6097 |
| HSV1-UL48-262 | - | CGAGCACCUUAACCUCCCGC | 20 | 6100 |
| HSV1-UL48-352 | + | AGUCUGCGCGUGUGUCCCGC | 20 | 6190 |
| HSV1-UL48-416 | + | GCUGCCGGACGCUGGCGCGC | 20 | 6254 |
| HSV1-UL48-373 | + | CAUAAAGUACCCAGAGGCGC | 20 | 6211 |
| HSV1-UL48-39 | - | CCACGCCGAGCUACGGGCGC | 20 | 5880 |
| HSV1-UL48-407 | + | CGCGGCCCAUAGGAUCUCGC | 20 | 6245 |
| HSV1-UL48-288 | - | GACGACGCCCCCGAAGAGGC | 20 | 6126 |
| HSV1-UL48-391 | + | GUUUAGCUCCCGCAGCCGGC | 20 | 6229 |
| HSV1-UL48-397 | + | CGUUGACGAACAUGAAGGGC | 20 | 6235 |
| HSV1-UL48-314 | - | GGACGGGGAUUCCCCGGGGC | 20 | 6152 |
| HSV1-UL48-290 | - | CGCCCCCGAAGAGGCGGGGC | 20 | 6128 |
| HSV1-UL48-253 | - | GAGAGCUGGCGUCAGUUGGC | 20 | 6091 |
| HSV1-UL48-375 | + | CGCGGGCCUGGUUGCCAUGC | 20 | 6213 |
| HSV1-UL48-280 | - | GCCCUCCGAGGCGGUCAUGC | 20 | 6118 |
| HSV1-UL48-414 | + | GCGCCUGGCGGUGCAGCUGC | 20 | 6252 |
| HSV1-UL48-261 | - | AAUCGAGGCCCGCCGGCUGC | 20 | 6099 |
| HSV1-UL48-303 | - | CGCGCUAGACGAUUUCGAUC | 20 | 6141 |
| HSV1-UL48-271 | - | GGCAACCAGGCCCGCGCCUC | 20 | 6109 |
| HSV1-UL48-317 | - | CUCCGCCCCCUACGGCGCUC | 20 | 6155 |
| HSV1-UL48-255 | - | UCAACGGAGCGCUCACCGUC | 20 | 6093 |
| HSV1-UL48-238 | - | GGUACCUGCGCGCCAGCGUC | 20 | 6076 |
| HSV1-UL48-245 | - | CUACCGAGAGACCGCUCGUC | 20 | 6083 |
| HSV1-UL48-361 | + | UCUUCGGGGGCGUCGUCGUC | 20 | 6199 |
| HSV1-UL48-402 | + | CAGAGGCAGUCAAACAGGUC | 20 | 6240 |
| HSV1-UL48-346 | + | CCCCCAGGCUGACAUCGGUC | 20 | 6184 |
| HSV1-UL48-275 | - | GUACUUUAUGGUGUUGAUUC | 20 | 6113 |
| HSV1-UL48-358 | + | GCCGCCAGCCCCGCCUCUUC | 20 | 6196 |
| HSV1-UL48-286 | - | GGACGACGACGCCCCCGAAG | 20 | 6124 |
| HSV1-UL48-342 | + | CCACGUCCUCGCCGUCUAAG | 20 | 6180 |
| HSV1-UL48-399 | + | AGCUCUCCAGGUCGCAACAG | 20 | 6237 |
| HSV1-UL48-384 | + | GUAGCCGCGCUGCGCACCAG | 20 | 6222 |
| HSV1-UL48-371 | + | CAACACCAUAAAGUACCCAG | 20 | 6209 |
| HSV1-UL48-268 | - | CGCGGCUACGGAGGAGCCAG | 20 | 6106 |
| HSV1-UL48-408 | + | CAAAACACGCGCCAGACGAG | 20 | 6246 |
| HSV1-UL48-381 | + | GUUGCCAUGCAGGGUGGGAG | 20 | 6219 |
| HSV1-UL48-329 | + | CCAUAUCCAGAGCGCCGUAG | 20 | 6167 |
| HSV1-UL48-1155 | - | UUGACGAGUACGGUGGGUAG | 20 | 16564 |
| HSV1-UL48-301 | - | CCACUUAGACGGCGAGGACG | 20 | 6139 |
| HSV1-UL48-310 | - | UCUGGACAUGUUGGGGGACG | 20 | 6148 |
| HSV1-UL48-404 | + | CAUCAUCUGCUCGGCGUACG | 20 | 6242 |
| HSV1-UL48-340 | + | GGGGGUAAAUCCCGGCCCCG | 20 | 6178 |
| HSV1-UL48-292 | - | GCGCCUGUCCUUUCUCCCCG | 20 | 6130 |
| HSV1-UL48-313 | - | UGGGGGACGGGGAUUCCCCG | 20 | 6151 |
| HSV1-UL48-351 | + | CAGUCUGCGCGUGUGUCCCG | 20 | 6189 |
| HSV1-UL48-277 | - | CUUCACGACCUCGCCCUCCG | 20 | 6115 |
| HSV1-UL48-257 | - | AACGGAGCGCUCACCGUCCG | 20 | 6095 |
| HSV1-UL48-263 | - | CCUCCCGCUGGUGCGCAGCG | 20 | 6101 |
| HSV1-UL48-353 | + | GUCUGCGCGUGUGUCCCGCG | 20 | 6191 |
| HSV1-UL48-365 | + | AAUUGUUUUUCGUACGCGCG | 20 | 6203 |
| HSV1-UL48-356 | + | GCGGGGAGAAAGGACAGGCG | 20 | 6194 |
| HSV1-UL48-289 | - | ACGACGCCCCCGAAGAGGCG | 20 | 6127 |
| HSV1-UL48-372 | + | CCAUAAAGUACCCAGAGGCG | 20 | 6210 |
| HSV1-UL48-300 | - | CGAGCUCCACUUAGACGGCG | 20 | 6138 |
| HSV1-UL48-42 | + | CCCGCGCCCGUAGCUCGGCG | 20 | 5988 |
| HSV1-UL48-369 | + | CAUGACCGCCUCGGAGGGCG | 20 | 6207 |
| HSV1-UL48-240 | - | CACCGCCAGGCGCACAUGCG | 20 | 6078 |
| HSV1-UL48-258 | - | CGUCCGGGGAGUGCCAAUCG | 20 | 6096 |
| HSV1-UL48-283 | - | CAAUUACGGGUCUACCAUCG | 20 | 6121 |
| HSV1-UL48-243 | - | AAUGCUGCGCGCCACGAUCG | 20 | 6081 |
| HSV1-UL48-410 | + | GUAGUACCUGUCCGCGAUCG | 20 | 6248 |
| HSV1-UL48-406 | + | ACGCGGCCCAUAGGAUCUCG | 20 | 6244 |
| HSV1-UL48-332 | + | CGCCGUAGGGGGCGGAGUCG | 20 | 6170 |
| HSV1-UL48-347 | + | CCCCAGGCUGACAUCGGUCG | 20 | 6185 |
| HSV1-UL48-359 | + | CCGCCAGCCCCGCCUCUUCG | 20 | 6197 |
| HSV1-UL48-287 | - | CGACGACGCCCCCGAAGAGG | 20 | 6125 |
| HSV1-UL48-278 | - | CACGACCUCGCCCUCCGAGG | 20 | 6116 |
| HSV1-UL48-330 | + | CAUAUCCAGAGCGCCGUAGG | 20 | 6168 |
| HSV1-UL48-1156 | - | UGACGAGUACGGUGGGUAGG | 20 | 16565 |
| HSV1-UL48-265 | - | GGUGCGCAGCGCGGCUACGG | 20 | 6103 |
| HSV1-UL48-321 | - | CUUGGAAUUGACGAGUACGG | 20 | 6159 |
| HSV1-UL48-390 | + | GGUUUAGCUCCCGCAGCCGG | 20 | 6228 |
| HSV1-UL48-367 | + | UUCCCGCAUGACCGCCUCGG | 20 | 6205 |
| HSV1-UL48-348 | + | CCCAGGCUGACAUCGGUCGG | 20 | 6186 |
| HSV1-UL48-360 | + | CGCCAGCCCCGCCUCUUCGG | 20 | 6198 |
| HSV1-UL48-377 | + | GGCCUGGUUGCCAUGCAGGG | 20 | 6215 |
| HSV1-UL48-386 | + | CCGCGCUGCGCACCAGCGGG | 20 | 6224 |
| HSV1-UL48-40 | - | CGCCGAGCUACGGGCGCGGG | 20 | 5973 |
| HSV1-UL48-349 | + | CCAGGCUGACAUCGGUCGGG | 20 | 6187 |
| HSV1-UL48-350 | + | CAGGCUGACAUCGGUCGGGG | 20 | 6188 |
| HSV1-UL48-331 | + | AUCCAGAGCGCCGUAGGGGG | 20 | 6169 |
| HSV1-UL48-336 | + | GUAGGGGGCGGAGUCGUGGG | 20 | 6174 |
| HSV1-UL48-379 | + | UGGUUGCCAUGCAGGGUGGG | 20 | 6217 |
| HSV1-UL48-298 | - | CCCGACCGAUGUCAGCCUGG | 20 | 6136 |
| HSV1-UL48-413 | + | GUCCGCGCAUGUGCGCCUGG | 20 | 6251 |
| HSV1-UL48-291 | - | CCCCGAAGAGGCGGGGCUGG | 20 | 6129 |
| HSV1-UL48-335 | + | CGUAGGGGGCGGAGUCGUGG | 20 | 6173 |
| HSV1-UL48-252 | - | GGAGAGCUGGCGUCAGUUGG | 20 | 6090 |
| HSV1-UL48-307 | - | UUUCGAUCUGGACAUGUUGG | 20 | 6145 |
| HSV1-UL48-388 | + | GGUUAAGGUGCUCGCGAAUG | 20 | 6226 |
| HSV1-UL48-279 | - | CGCCCUCCGAGGCGGUCAUG | 20 | 6117 |
| HSV1-UL48-248 | - | CGUACGCCGAGCAGAUGAUG | 20 | 6086 |
| HSV1-UL48-297 | - | CCCCGACCGAUGUCAGCCUG | 20 | 6135 |
| HSV1-UL48-260 | - | CAAUCGAGGCCCGCCGGCUG | 20 | 6098 |
| HSV1-UL48-334 | + | CCGUAGGGGGCGGAGUCGUG | 20 | 6172 |
| HSV1-UL48-306 | - | AUUUCGAUCUGGACAUGUUG | 20 | 6144 |
| HSV1-UL48-344 | + | CUCGUCCCCCAGGCUGACAU | 20 | 6182 |
| HSV1-UL48-405 | + | GCUCGGCGUACGCGGCCCAU | 20 | 6243 |
| HSV1-UL48-325 | + | CUCGUCAAUUCCAAGGGCAU | 20 | 6163 |
| HSV1-UL48-392 | + | CGCAGCCGGCGGGCCUCGAU | 20 | 6230 |
| HSV1-UL48-247 | - | UUUGACCCGCGAGAUCCUAU | 20 | 6085 |
| HSV1-UL48-242 | - | CGCGGACGCGAUCGCGACCU | 20 | 6080 |
| HSV1-UL48-319 | - | CAGAUGUUUACCGAUGCCCU | 20 | 6157 |
| HSV1-UL48-296 | - | CCCCCGACCGAUGUCAGCCU | 20 | 6134 |
| HSV1-UL48-366 | + | GUGUUCCCGCAUGACCGCCU | 20 | 6204 |
| HSV1-UL48-41 | + | CUCCUCCCGCGCCCGUAGCU | 20 | 5890 |
| HSV1-UL48-403 | + | GUCGGGCCGCAUCAUCUGCU | 20 | 6241 |
| HSV1-UL48-1126 | - | CGUGUUGGCCAACUUCUGCU | 20 | 6074 |
| HSV1-UL48-272 | - | GCAACCAGGCCCGCGCCUCU | 20 | 6110 |
| HSV1-UL48-409 | + | GCGCCAGACGAGCGGUCUCU | 20 | 6247 |
| HSV1-UL48-420 | + | GUACAGGGCCGAGCAGAAGU | 20 | 6258 |
| HSV1-UL48-276 | - | GGUGUUGAUUCGGGCGAAGU | 20 | 6114 |
| HSV1-UL48-326 | + | CAUCUGCUCAAACUCGAAGU | 20 | 6164 |
| HSV1-UL48-251 | - | CCUGGAGAGCUGGCGUCAGU | 20 | 6089 |
| HSV1-UL48-327 | + | GGCCAUAUCCAGAGCGCCGU | 20 | 6165 |
| HSV1-UL48-341 | + | AUCGAAAUCGUCUAGCGCGU | 20 | 6179 |
| HSV1-UL48-333 | + | GCCGUAGGGGGCGGAGUCGU | 20 | 6171 |
| HSV1-UL48-401 | + | ACAGAGGCAGUCAAACAGGU | 20 | 6239 |
| HSV1-UL48-322 | - | UUGGAAUUGACGAGUACGGU | 20 | 6160 |
| HSV1-UL48-345 | + | UCCCCCAGGCUGACAUCGGU | 20 | 6183 |
| HSV1-UL48-378 | + | GCCUGGUUGCCAUGCAGGGU | 20 | 6216 |
| HSV1-UL48-1157 | - | AAUUGACGAGUACGGUGGGU | 20 | 16566 |
| HSV1-UL48-304 | - | CGAUUUCGAUCUGGACAUGU | 20 | 6142 |
| HSV1-UL48-1129 | - | GGAGAGCUAUCGAACCGUGU | 20 | 16538 |
| HSV1-UL48-274 | - | GGUACUUUAUGGUGUUGAUU | 20 | 6112 |
| HSV1-UL48-357 | + | AGCCGCCAGCCCCGCCUCUU | 20 | 6195 |
| HSV1-UL48-305 | - | GAUUUCGAUCUGGACAUGUU | 20 | 6143 |

**Table 12A** provides exemplary targeting domains for knocking out the *UL48* gene selected according to the first tier parameters. The targeting domains bind within the first 500 bp of the coding sequence (e.g., within 500 bp downstream from the start codon), have a high level of orthogonality and start with a 5'G. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12A**

| 1st Tier | | | | |
|---|---|---|---|---|
| gRNA Name | DNA Strand | Targeting Domain | Target Site Length | Seq ID NO: |
| HSV1-UL48-1158 | + | GCUCGUCGACCAAGAGGUCCA | 21 | 16567 |
| HSV1-UL48-1159 | + | GUACAGCGGGGGGGCCGC | 18 | 16568 |
| HSV1-UL48-101 | + | GCGUACAGCGGGGGGGCCGC | 20 | 6020 |
| HSV1-UL48-1160 | + | GUUGCGUACAGCGGGGGGGCCGC | 23 | 16569 |
| HSV1-UL48-1161 | + | GAGGGCGGCGGGGGGGACGGGC | 22 | 16570 |
| HSV1-UL48-1162 | + | GGCCACGUCGCCGUGGGC | 18 | 16571 |
| HSV1-UL48-1163 | + | GAAGGCCACGUCGCCGUGGGC | 21 | 16572 |
| HSV1-UL48-1164 | + | GGAAGGCCACGUCGCCGUGGGC | 22 | 16573 |
| HSV1-UL48-1165 | + | GGGAAGGCCACGUCGCCGUGGGC | 23 | 16574 |
| HSV1-UL48-1166 | + | GUAGAGCCCGAGGCCGUC | 18 | 16575 |
| HSV1-UL48-1167 | + | GUAGUAGAGCCCGAGGCCGUC | 21 | 16576 |
| HSV1-UL48-1168 | + | GACCAAGAGGUCCAUUGGG | 19 | 16577 |
| HSV1-UL48-1169 | + | GUCGACCAAGAGGUCCAUUGGG | 22 | 16578 |
| HSV1-UL48-1170 | + | GUGGGCGCGAAUGUCGAU | 18 | 16579 |
| HSV1-UL48-1171 | + | GCCGUGGGCGCGAAUGUCGAU | 21 | 16580 |
| HSV1-UL48-1172 | + | GUCGCCGUGGGCGCGAAUGUCGAU | 24 | 16581 |
| HSV1-UL48-1173 | - | GUACCAUGCUCGAUACCUGGAAC | 23 | 16582 |

**Table 12B** provides exemplary targeting domains for knocking out the *UL48* gene selected according to the second tier parameters. The targeting domains bind within the first 500 bp of the coding sequence (e.g., within 500 bp downstream from the start codon), and have a high level of orthogonality. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12B**

| 2nd Tier | | | | |
|---|---|---|---|---|
| gRNA Name | DNA Strand | Targeting Domain | Target Site Length | Seq ID NO: |
| HSV1-UL48-1174 | + | CGUCGACCAAGAGGUCCA | 18 | 16583 |
| HSV1-UL48-1175 | + | UCGUCGACCAAGAGGUCCA | 19 | 16584 |
| HSV1-UL48-1176 | + | CUCGUCGACCAAGAGGUCCA | 20 | 16585 |
| HSV1-UL48-1177 | + | AGCUCGUCGACCAAGAGGUCCA | 22 | 16586 |
| HSV1-UL48-1178 | + | CAGCUCGUCGACCAAGAGGUCCA | 23 | 16587 |
| HSV1-UL48-1179 | + | ACAGCUCGUCGACCAAGAGGUCCA | 24 | 16588 |
| HSV1-UL48-1180 | + | CGUACAGCGGGGGGGCCGC | 19 | 16589 |
| HSV1-UL48-1181 | + | UGCGUACAGCGGGGGGGCCGC | 21 | 16590 |
| HSV1-UL48-1182 | + | UUGCGUACAGCGGGGGGGCCGC | 22 | 16591 |
| HSV1-UL48-1183 | + | CGUUGCGUACAGCGGGGGGGCCGC | 24 | 16592 |
| HSV1-UL48-1184 | + | AGGGCGGCGGGGGGGACGGGC | 21 | 16593 |
| HSV1-UL48-1185 | + | AGAGGGCGGCGGGGGGGACGGGC | 23 | 16594 |
| HSV1-UL48-1186 | + | AAGAGGGCGGCGGGGGGGACGGGC | 24 | 16595 |
| HSV1-UL48-1187 | + | AGGCCACGUCGCCGUGGGC | 19 | 16596 |
| HSV1-UL48-726 | + | AAGGCCACGUCGCCGUGGGC | 20 | 6513 |
| HSV1-UL48-1188 | + | AGGGAAGGCCACGUCGCCGUGGGC | 24 | 16597 |
| HSV1-UL48-1189 | + | AGUAGAGCCCGAGGCCGUC | 19 | 16598 |
| HSV1-UL48-733 | + | UAGUAGAGCCCGAGGCCGUC | 20 | 6517 |
| HSV1-UL48-1190 | + | CGUAGUAGAGCCCGAGGCCGUC | 22 | 16599 |
| HSV1-UL48-1191 | + | UCGUAGUAGAGCCCGAGGCCGUC | 23 | 16600 |
| HSV1-UL48-1192 | + | UUCGUAGUAGAGCCCGAGGCCGUC | 24 | 16601 |
| HSV1-UL48-1193 | + | ACCAAGAGGUCCAUUGGG | 18 | 16602 |
| HSV1-UL48-1134 | + | CGACCAAGAGGUCCAUUGGG | 20 | 16543 |
| HSV1-UL48-1194 | + | UCGACCAAGAGGUCCAUUGGG | 21 | 16603 |
| HSV1-UL48-1195 | + | CGUCGACCAAGAGGUCCAUUGGG | 23 | 16604 |
| HSV1-UL48-1196 | + | UCGUCGACCAAGAGGUCCAUUGGG | 24 | 16605 |
| HSV1-UL48-1197 | + | CGUGGGCGCGAAUGUCGAU | 19 | 16606 |
| HSV1-UL48-725 | + | CCGUGGGCGCGAAUGUCGAU | 20 | 6512 |
| HSV1-UL48-1198 | + | CGCCGUGGGCGCGAAUGUCGAU | 22 | 16607 |
| HSV1-UL48-1199 | + | UCGCCGUGGGCGCGAAUGUCGAU | 23 | 16608 |
| HSV1-UL48-1200 | + | ACAUCGCUGGGCAGCGUUGAU | 21 | 16609 |
| HSV1-UL48-1201 | + | CACAUCGCUGGGCAGCGUUGAU | 22 | 16610 |
| HSV1-UL48-1202 | + | CCACAUCGCUGGGCAGCGUUGAU | 23 | 16611 |
| HSV1-UL48-1203 | + | ACCACAUCGCUGGGCAGCGUUGAU | 24 | 16612 |
| HSV1-UL48-1204 | - | AUGCUCGAUACCUGGAAC | 18 | 16613 |
| HSV1-UL48-1205 | - | CAUGCUCGAUACCUGGAAC | 19 | 16614 |
| HSV1-UL48-621 | - | CCAUGCUCGAUACCUGGAAC | 20 | 6455 |
| HSV1-UL48-1206 | - | ACCAUGCUCGAUACCUGGAAC | 21 | 16615 |
| HSV1-UL48-1207 | - | UACCAUGCUCGAUACCUGGAAC | 22 | 16616 |
| HSV1-UL48-1208 | - | UGUACCAUGCUCGAUACCUGGAAC | 24 | 16617 |
| HSV1-UL48-1209 | - | CGACCAACGCCGACCUGUACC | 21 | 16618 |
| HSV1-UL48-1210 | - | CCGACCAACGCCGACCUGUACC | 22 | 16619 |
| HSV1-UL48-1211 | - | ACCGACCAACGCCGACCUGUACC | 23 | 16620 |
| HSV1-UL48-1212 | - | UACCGACCAACGCCGACCUGUACC | 24 | 16621 |
| HSV1-UL48-1213 | - | CAACGCUGCCCAGCGAUGUGG | 21 | 16622 |
| HSV1-UL48-1214 | - | UCAACGCUGCCCAGCGAUGUGG | 22 | 16623 |
| HSV1-UL48-1215 | - | AUCAACGCUGCCCAGCGAUGUGG | 23 | 16624 |
| HSV1-UL48-1216 | - | UAUCAACGCUGCCCAGCGAUGUGG | 24 | 16625 |

**Table 12C** provides exemplary targeting domains for knocking out the *UL48* gene selected according to the third tier parameters. The targeting domains bind within the first 500 bp of the coding sequence (e.g., within 500 bp downstream from the start codon). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12C**

| 3rd Tier | | | | |
|---|---|---|---|---|
| gRNA Name | DNA Strand | Targeting Domain | Target Site Length | Seq ID NO: |
| HSV1-UL48-1217 | + | GCGGCGGGGGGGACGGGC | 18 | 16626 |
| HSV1-UL48-1218 | + | GGCGGCGGGGGGGACGGGC | 19 | 16627 |
| HSV1-UL48-702 | + | GGGCGGCGGGGGGGACGGGC | 20 | 6499 |
| HSV1-UL48-1219 | + | UCGCUGGGCAGCGUUGAU | 18 | 16628 |
| HSV1-UL48-1220 | + | AUCGCUGGGCAGCGUUGAU | 19 | 16629 |
| HSV1-UL48-60 | + | CAUCGCUGGGCAGCGUUGAU | 20 | 5998 |
| HSV1-UL48-1221 | - | CCAACGCCGACCUGUACC | 18 | 16630 |
| HSV1-UL48-1222 | - | ACCAACGCCGACCUGUACC | 19 | 16631 |
| HSV1-UL48-22 | - | GACCAACGCCGACCUGUACC | 20 | 5861 |
| HSV1-UL48-1223 | - | CCGCCCCCCCGCCCGGCC | 18 | 16632 |
| HSV1-UL48-1224 | - | ACCGCCCCCCCGCCCGGCC | 19 | 16633 |
| HSV1-UL48-5 | - | CACCGCCCCCCCGCCCGGCC | 20 | 5964 |
| HSV1-UL48-1225 | - | CCACCGCCCCCCCGCCCGGCC | 21 | 16634 |
| HSV1-UL48-1226 | - | GCCACCGCCCCCCCGCCCGGCC | 22 | 16635 |
| HSV1-UL48-1227 | - | CGCCACCGCCCCCCCGCCCGGCC | 23 | 16636 |
| HSV1-UL48-1228 | - | UCGCCACCGCCCCCCCGCCCGGCC | 24 | 16637 |
| HSV1-UL48-1229 | - | CGCUGCCCAGCGAUGUGG | 18 | 16638 |
| HSV1-UL48-1230 | - | ACGCUGCCCAGCGAUGUGG | 19 | 16639 |
| HSV1-UL48-24 | - | AACGCUGCCCAGCGAUGUGG | 20 | 5863 |

**Table 12D** provides exemplary targeting domains for knocking out the *UL48* gene selected according to the fourth tier parameters. The targeting domains bind within the first 500 bp of the coding sequence (e.g., within 500 bp downstream from the start codon). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12D**

| 4th Tier | | | | |
|---|---|---|---|---|
| gRNA Name | DNA Strand | Targeting Domain | Target Site Length | Seq ID NO: |
| HSV1-UL48-1231 | + | GUCGAGGAGACGGUUAAA | 18 | 16640 |
| HSV1-UL48-1232 | + | CGUCGAGGAGACGGUUAAA | 19 | 16641 |
| HSV1-UL48-710 | + | UCGUCGAGGAGACGGUUAAA | 20 | 6502 |
| HSV1-UL48-1233 | + | GUCGUCGAGGAGACGGUUAAA | 21 | 16642 |
| HSV1-UL48-1234 | + | AGUCGUCGAGGAGACGGUUAAA | 22 | 16643 |
| HSV1-UL48-1235 | + | AAGUCGUCGAGGAGACGGUUAAA | 23 | 16644 |
| HSV1-UL48-1236 | + | CAAGUCGUCGAGGAGACGGUUAAA | 24 | 16645 |
| HSV1-UL48-1237 | + | UAGCUCGGCGUGGAAGAA | 18 | 16646 |
| HSV1-UL48-1238 | + | GUAGCUCGGCGUGGAAGAA | 19 | 16647 |
| HSV1-UL48-738 | + | CGUAGCUCGGCGUGGAAGAA | 20 | 6522 |
| HSV1-UL48-1239 | + | CCGUAGCUCGGCGUGGAAGAA | 21 | 16648 |
| HSV1-UL48-1240 | + | CCCGUAGCUCGGCGUGGAAGAA | 22 | 16649 |
| HSV1-UL48-1241 | + | GCCCGUAGCUCGGCGUGGAAGAA | 23 | 16650 |
| HSV1-UL48-1242 | + | CGCCCGUAGCUCGGCGUGGAAGAA | 24 | 16651 |
| HSV1-UL48-1243 | + | GGCGCCCCGUUGCGUACA | 18 | 16652 |
| HSV1-UL48-1244 | + | AGGCGCCCCGUUGCGUACA | 19 | 16653 |
| HSV1-UL48-694 | + | CAGGCGCCCCGUUGCGUACA | 20 | 6494 |
| HSV1-UL48-1245 | + | UCAGGCGCCCCGUUGCGUACA | 21 | 16654 |
| HSV1-UL48-1246 | + | CUCAGGCGCCCCGUUGCGUACA | 22 | 16655 |
| HSV1-UL48-1247 | + | GCUCAGGCGCCCCGUUGCGUACA | 23 | 16656 |
| HSV1-UL48-1248 | + | GGCUCAGGCGCCCCGUUGCGUACA | 24 | 16657 |
| HSV1-UL48-1249 | + | UCGGCGUGGAAGAAACGA | 18 | 16658 |
| HSV1-UL48-1250 | + | CUCGGCGUGGAAGAAACGA | 19 | 16659 |
| HSV1-UL48-736 | + | GCUCGGCGUGGAAGAAACGA | 20 | 6520 |
| HSV1-UL48-1251 | + | AGCUCGGCGUGGAAGAAACGA | 21 | 16660 |
| HSV1-UL48-1252 | + | UAGCUCGGCGUGGAAGAAACGA | 22 | 16661 |
| HSV1-UL48-1253 | + | GUAGCUCGGCGUGGAAGAAACGA | 23 | 16662 |
| HSV1-UL48-1254 | + | CGUAGCUCGGCGUGGAAGAAACGA | 24 | 16663 |
| HSV1-UL48-1255 | + | AUCGCUGGGCAGCGUUGA | 18 | 16664 |
| HSV1-UL48-1256 | + | CAUCGCUGGGCAGCGUUGA | 19 | 16665 |
| HSV1-UL48-720 | + | ACAUCGCUGGGCAGCGUUGA | 20 | 6509 |
| HSV1-UL48-1257 | + | CACAUC GCUGGGCAGC GUUGA | 21 | 16666 |
| HSV1-UL48-1258 | + | CCACAUCGCUGGGCAGCGUUGA | 22 | 16667 |
| HSV1-UL48-1259 | + | ACCACAUCGCUGGGCAGCGUUGA | 23 | 16668 |
| HSV1-UL48-1260 | + | CACCACAUCGCUGGGCAGCGUUGA | 24 | 16669 |
| HSV1-UL48-1261 | + | CAUUGGGUGGGGUUGAUA | 18 | 16670 |
| HSV1-UL48-1262 | + | CCAUUGGGUGGGGUUGAUA | 19 | 16671 |
| HSV1-UL48-1133 | + | UCCAUUGGGUGGGGUUGAUA | 20 | 16542 |
| HSV1-UL48-1263 | + | GUCCAUUGGGUGGGGUUGAUA | 21 | 16672 |
| HSV1-UL48-1264 | + | GGUCCAUUGGGUGGGGUUGAUA | 22 | 16673 |
| HSV1-UL48-1265 | + | AGGUCCAUUGGGUGGGGUUGAUA | 23 | 16674 |
| HSV1-UL48-1266 | + | GAGGUCCAUUGGGUGGGGUUGAUA | 24 | 16675 |
| HSV1-UL48-1267 | + | CGGGUGGCCGGAAGCGUA | 18 | 16676 |
| HSV1-UL48-1268 | + | GCGGGUGGCCGGAAGCGUA | 19 | 16677 |
| HSV1-UL48-49 | + | CGCGGGUGGCCGGAAGCGUA | 20 | 5992 |
| HSV1-UL48-1269 | + | UCGCGGGUGGCCGGAAGCGUA | 21 | 16678 |
| HSV1-UL48-1270 | + | GUCGCGGGUGGCCGGAAGCGUA | 22 | 16679 |
| HSV1-UL48-1271 | + | CGUCGCGGGUGGCCGGAAGCGUA | 23 | 16680 |
| HSV1-UL48-1272 | + | CCGUCGCGGGUGGCCGGAAGCGUA | 24 | 16681 |
| HSV1-UL48-1273 | + | AGAUCCUCGUUCCAGGUA | 18 | 16682 |
| HSV1-UL48-1274 | + | CAGAUCCUCGUUCCAGGUA | 19 | 16683 |
| HSV1-UL48-717 | + | ACAGAUCCUCGUUCCAGGUA | 20 | 6507 |
| HSV1-UL48-1275 | + | AACAGAUCCUCGUUCCAGGUA | 21 | 16684 |
| HSV1-UL48-1276 | + | AAACAGAUCCUCGUUCCAGGUA | 22 | 16685 |
| HSV1-UL48-1277 | + | AAAACAGAUCCUCGUUCCAGGUA | 23 | 16686 |
| HSV1-UL48-1278 | + | GAAAACAGAUCCUCGUUCCAGGUA | 24 | 16687 |
| HSV1-UL48-1279 | + | UCGUCGAGGAGACGGUUA | 18 | 16688 |
| HSV1-UL48-1280 | + | GUCGUCGAGGAGACGGUUA | 19 | 16689 |
| HSV1-UL48-711 | + | AGUCGUCGAGGAGACGGUUA | 20 | 6503 |
| HSV1-UL48-1281 | + | AAGUCGUCGAGGAGACGGUUA | 21 | 16690 |
| HSV1-UL48-1282 | + | CAAGUCGUCGAGGAGACGGUUA | 22 | 16691 |
| HSV1-UL48-1283 | + | CCAAGUCGUCGAGGAGACGGUUA | 23 | 16692 |
| HSV1-UL48-1284 | + | CCCAAGUCGUCGAGGAGACGGUUA | 24 | 16693 |
| HSV1-UL48-1285 | + | GCUCGGCGUGGAAGAAAC | 18 | 16694 |
| HSV1-UL48-1286 | + | AGCUCGGCGUGGAAGAAAC | 19 | 16695 |
| HSV1-UL48-737 | + | UAGCUCGGCGUGGAAGAAAC | 20 | 6521 |
| HSV1-UL48-1287 | + | GUAGCUCGGCGUGGAAGAAAC | 21 | 16696 |
| HSV1-UL48-1288 | + | CGUAGCUCGGCGUGGAAGAAAC | 22 | 16697 |
| HSV1-UL48-1289 | + | CCGUAGCUCGGCGUGGAAGAAAC | 23 | 16698 |
| HSV1-UL48-1290 | + | CCCGUAGCUCGGCGUGGAAGAAAC | 24 | 16699 |
| HSV1-UL48-1291 | + | AUUGGGUGGGGUUGAUAC | 18 | 16700 |
| HSV1-UL48-1292 | + | CAUUGGGUGGGGUUGAUAC | 19 | 16701 |
| HSV1-UL48-1146 | + | CCAUUGGGUGGGGUUGAUAC | 20 | 16555 |
| HSV1-UL48-1293 | + | UCCAUUGGGUGGGGUUGAUAC | 21 | 16702 |
| HSV1-UL48-1294 | + | GUCCAUUGGGUGGGGUUGAUAC | 22 | 16703 |
| HSV1-UL48-1295 | + | GGUCCAUUGGGUGGGGUUGAUAC | 23 | 16704 |
| HSV1-UL48-1296 | + | AGGUCCAUUGGGUGGGGUUGAUAC | 24 | 16705 |
| HSV1-UL48-1297 | + | GCAAACAGCUCGUCGACC | 18 | 16706 |
| HSV1-UL48-1298 | + | GGCAAACAGCUCGUCGACC | 19 | 16707 |
| HSV1-UL48-678 | + | CGGCAAACAGCUCGUCGACC | 20 | 6488 |
| HSV1-UL48-1299 | + | UCGGCAAACAGCUCGUCGACC | 21 | 16708 |
| HSV1-UL48-1300 | + | GUCGGCAAACAGCUCGUCGACC | 22 | 16709 |
| HSV1-UL48-1301 | + | UGUCGGCAAACAGCUCGUCGACC | 23 | 16710 |
| HSV1-UL48-1302 | + | AUGUCGGCAAACAGCUCGUCGACC | 24 | 16711 |
| HSV1-UL48-1303 | + | AGGGAAGGCCACGUCGCC | 18 | 16712 |
| HSV1-UL48-1304 | + | UAGGGAAGGCCACGUCGCC | 19 | 16713 |
| HSV1-UL48-727 | + | GUAGGGAAGGCCACGUCGCC | 20 | 6514 |
| HSV1-UL48-1305 | + | CGUAGGGAAGGCCACGUCGCC | 21 | 16714 |
| HSV1-UL48-1306 | + | GCGUAGGGAAGGCCACGUCGCC | 22 | 16715 |
| HSV1-UL48-1307 | + | AGCGUAGGGAAGGCCACGUCGCC | 23 | 16716 |
| HSV1-UL48-1308 | + | AAGCGUAGGGAAGGCCACGUCGCC | 24 | 16717 |
| HSV1-UL48-1309 | + | CGCCCCGUUGCGUACAGC | 18 | 16718 |
| HSV1-UL48-1310 | + | GCGCCCCGUUGCGUACAGC | 19 | 16719 |
| HSV1-UL48-96 | + | GGCGCCCCGUUGCGUACAGC | 20 | 6019 |
| HSV1-UL48-1311 | + | AGGCGCCCCGUUGCGUACAGC | 21 | 16720 |
| HSV1-UL48-1312 | + | CAGGCGCCCCGUUGCGUACAGC | 22 | 16721 |
| HSV1-UL48-1313 | + | UCAGGCGCCCCGUUGCGUACAGC | 23 | 16722 |
| HSV1-UL48-1314 | + | CUCAGGCGCCCCGUUGCGUACAGC | 24 | 16723 |
| HSV1-UL48-1315 | + | AGC GCUUC GUAGUAGAGC | 18 | 16724 |
| HSV1-UL48-1316 | + | GAGC GCUUC GUAGUAGAGC | 19 | 16725 |
| HSV1-UL48-734 | + | AGAGC GCUUC GUAGUAGAGC | 20 | 6518 |
| HSV1-UL48-1317 | + | GAGAGC GCUUC GUAGUAGAGC | 21 | 16726 |
| HSV1-UL48-1318 | + | AGAGAGC GCUUC GUAGUAGAGC | 22 | 16727 |
| HSV1-UL48-1319 | + | GAGAGAGC GCUUC GUAGUAGAGC | 23 | 16728 |
| HSV1-UL48-1320 | + | CGAGAGAGCGCUUCGUAGUAGAGC | 24 | 16729 |
| HSV1-UL48-1321 | + | UCGAGCAUGGUACAUAGC | 18 | 16730 |
| HSV1-UL48-1322 | + | AUCGAGCAUGGUACAUAGC | 19 | 16731 |
| HSV1-UL48-716 | + | UAUCGAGCAUGGUACAUAGC | 20 | 6506 |
| HSV1-UL48-1323 | + | GUAUCGAGCAUGGUACAUAGC | 21 | 16732 |
| HSV1-UL48-1324 | + | GGUAUCGAGCAUGGUACAUAGC | 22 | 16733 |
| HSV1-UL48-1325 | + | AGGUAUCGAGCAUGGUACAUAGC | 23 | 16734 |
| HSV1-UL48-1326 | + | CAGGUAUCGAGCAUGGUACAUAGC | 24 | 16735 |
| HSV1-UL48-1327 | + | ACGGUUAAAGAGGGCGGC | 18 | 16736 |
| HSV1-UL48-1328 | + | GACGGUUAAAGAGGGCGGC | 19 | 16737 |
| HSV1-UL48-77 | + | AGACGGUUAAAGAGGGCGGC | 20 | 5929 |
| HSV1-UL48-1329 | + | GAGACGGUUAAAGAGGGCGGC | 21 | 16738 |
| HSV1-UL48-1330 | + | GGAGACGGUUAAAGAGGGCGGC | 22 | 16739 |
| HSV1-UL48-1331 | + | AGGAGACGGUUAAAGAGGGCGGC | 23 | 16740 |
| HSV1-UL48-1332 | + | GAGGAGACGGUUAAAGAGGGCGGC | 24 | 16741 |
| HSV1-UL48-1333 | + | CCGCGCCCGUAGCUCGGC | 18 | 16742 |
| HSV1-UL48-1334 | + | CCCGCGCCCGUAGCUCGGC | 19 | 16743 |
| HSV1-UL48-741 | + | UCCCGCGCCCGUAGCUCGGC | 20 | 6524 |
| HSV1-UL48-1335 | + | CUCCCGCGCCCGUAGCUCGGC | 21 | 16744 |
| HSV1-UL48-1336 | + | CCUCCCGCGCCCGUAGCUCGGC | 22 | 16745 |
| HSV1-UL48-1337 | + | UCCUCCCGCGCCCGUAGCUCGGC | 23 | 16746 |
| HSV1-UL48-1338 | + | CUCCUCCCGCGCCCGUAGCUCGGC | 24 | 16747 |
| HSV1-UL48-1339 | + | GGCAUGGGUGGGGAGGGC | 18 | 16748 |
| HSV1-UL48-1340 | + | GGGCAUGGGUGGGGAGGGC | 19 | 16749 |
| HSV1-UL48-696 | + | CGGGCAUGGGUGGGGAGGGC | 20 | 6496 |
| HSV1-UL48-1341 | + | ACGGGCAUGGGUGGGGAGGGC | 21 | 16750 |
| HSV1-UL48-1342 | + | GACGGGCAUGGGUGGGGAGGGC | 22 | 16751 |
| HSV1-UL48-1343 | + | GGACGGGCAUGGGUGGGGAGGGC | 23 | 16752 |
| HSV1-UL48-1344 | + | GGGACGGGCAUGGGUGGGGAGGGC | 24 | 16753 |
| HSV1-UL48-1345 | + | UGGGACCCCCGGCCGGGC | 18 | 16754 |
| HSV1-UL48-1346 | + | UUGGGACCCCCGGCCGGGC | 19 | 16755 |
| HSV1-UL48-110 | + | UUUGGGACCCCCGGCCGGGC | 20 | 6023 |
| HSV1-UL48-1347 | + | UUUUGGGACCCCCGGCCGGGC | 21 | 16756 |
| HSV1-UL48-1348 | + | UUUUUGGGACCCCCGGCCGGGC | 22 | 16757 |
| HSV1-UL48-1349 | + | GUUUUUGGGACCCCCGGCCGGGC | 23 | 16758 |
| HSV1-UL48-1350 | + | UGUUUUUGGGACCCCCGGCCGGGC | 24 | 16759 |
| HSV1-UL48-1351 | + | GAGGCCGUCGCGGGUGGC | 18 | 16760 |
| HSV1-UL48-1352 | + | CGAGGCCGUCGCGGGUGGC | 19 | 16761 |
| HSV1-UL48-47 | + | CCGAGGCCGUCGCGGGUGGC | 20 | 5991 |
| HSV1-UL48-1353 | + | CCCGAGGCCGUCGCGGGUGGC | 21 | 16762 |
| HSV1-UL48-1354 | + | GCCCGAGGCCGUCGCGGGUGGC | 22 | 16763 |
| HSV1-UL48-1355 | + | AGCCCGAGGCCGUCGCGGGUGGC | 23 | 16764 |
| HSV1-UL48-1356 | + | GAGCCCGAGGCCGUCGCGGGUGGC | 24 | 16765 |
| HSV1-UL48-1357 | + | GCUAAAGCCCAAGUCGUC | 18 | 16766 |
| HSV1-UL48-1358 | + | CGCUAAAGCCCAAGUCGUC | 19 | 16767 |
| HSV1-UL48-713 | + | GCGCUAAAGCCCAAGUCGUC | 20 | 6504 |
| HSV1-UL48-1359 | + | CGCGCUAAAGCCCAAGUCGUC | 21 | 16768 |
| HSV1-UL48-1360 | + | CCGCGCUAAAGCCCAAGUCGUC | 22 | 16769 |
| HSV1-UL48-1361 | + | CCCGCGCUAAAGCCCAAGUCGUC | 23 | 16770 |
| HSV1-UL48-1362 | + | GCCCGCGCUAAAGCCCAAGUCGUC | 24 | 16771 |
| HSV1-UL48-1363 | + | GUCGAUUUGGGUGCGUUC | 18 | 16772 |
| HSV1-UL48-1364 | + | UGUCGAUUUGGGUGCGUUC | 19 | 16773 |
| HSV1-UL48-56 | + | AUGUCGAUUUGGGUGCGUUC | 20 | 5995 |
| HSV1-UL48-1365 | + | AAUGUCGAUUUGGGUGCGUUC | 21 | 16774 |
| HSV1-UL48-1366 | + | GAAUGUCGAUUUGGGUGCGUUC | 22 | 16775 |
| HSV1-UL48-1367 | + | CGAAUGUCGAUUUGGGUGCGUUC | 23 | 16776 |
| HSV1-UL48-1368 | + | GCGAAUGUCGAUUUGGGUGCGUUC | 24 | 16777 |
| HSV1-UL48-1369 | + | GCGCCCCGUUGCGUACAG | 18 | 16778 |
| HSV1-UL48-1370 | + | GGCGCCCCGUUGCGUACAG | 19 | 16779 |
| HSV1-UL48-95 | + | AGGCGCCCCGUUGCGUACAG | 20 | 6018 |
| HSV1-UL48-1371 | + | CAGGCGCCCCGUUGCGUACAG | 21 | 16780 |
| HSV1-UL48-1372 | + | UCAGGCGCCCCGUUGCGUACAG | 22 | 16781 |
| HSV1-UL48-1373 | + | CUCAGGCGCCCCGUUGCGUACAG | 23 | 16782 |
| HSV1-UL48-1374 | + | GCUCAGGCGCCCCGUUGCGUACAG | 24 | 16783 |
| HSV1-UL48-1375 | + | GGGUGGGGAGGGCAUGAG | 18 | 16784 |
| HSV1-UL48-1376 | + | UGGGUGGGGAGGGCAUGAG | 19 | 16785 |
| HSV1-UL48-695 | + | AUGGGUGGGGAGGGCAUGAG | 20 | 6495 |
| HSV1-UL48-1377 | + | CAUGGGUGGGGAGGGCAUGAG | 21 | 16786 |
| HSV1-UL48-1378 | + | GCAUGGGUGGGGAGGGCAUGAG | 22 | 16787 |
| HSV1-UL48-1379 | + | GGCAUGGGUGGGGAGGGCAUGAG | 23 | 16788 |
| HSV1-UL48-1380 | + | GGGCAUGGGUGGGGAGGGCAUGAG | 24 | 16789 |
| HSV1-UL48-1381 | + | CGAGAGAGCGCUUCGUAG | 18 | 16790 |
| HSV1-UL48-1382 | + | AC GAGAGAGC GCUUC GUAG | 19 | 16791 |
| HSV1-UL48-735 | + | AAC GAGAGAGC GCUUC GUAG | 20 | 6519 |
| HSV1-UL48-1383 | + | AAAC GAGAGAGC GCUUC GUAG | 21 | 16792 |
| HSV1-UL48-1384 | + | GAAACGAGAGAGCGCUUCGUAG | 22 | 16793 |
| HSV1-UL48-1385 | + | AGAAACGAGAGAGCGCUUCGUAG | 23 | 16794 |
| HSV1-UL48-1386 | + | AAGAAAC GAGAGAGC GCUUC GUAG | 24 | 16795 |
| HSV1-UL48-1387 | + | CGUACAGCGGGGGGGCCG | 18 | 16796 |
| HSV1-UL48-1388 | + | GCGUACAGCGGGGGGGCCG | 19 | 16797 |
| HSV1-UL48-689 | + | UGCGUACAGCGGGGGGGCCG | 20 | 6493 |
| HSV1-UL48-1389 | + | UUGCGUACAGCGGGGGGGCCG | 21 | 16798 |
| HSV1-UL48-1390 | + | GUUGCGUACAGCGGGGGGGCCG | 22 | 16799 |
| HSV1-UL48-1391 | + | CGUUGCGUACAGCGGGGGGGCCG | 23 | 16800 |
| HSV1-UL48-1392 | + | CCGUUGCGUACAGCGGGGGGGCCG | 24 | 16801 |
| HSV1-UL48-1393 | + | CGCGGGUGGCCGGAAGCG | 18 | 16802 |
| HSV1-UL48-1394 | + | UCGCGGGUGGCCGGAAGCG | 19 | 16803 |
| HSV1-UL48-730 | + | GUCGCGGGUGGCCGGAAGCG | 20 | 6515 |
| HSV1-UL48-1395 | + | CGUCGCGGGUGGCCGGAAGCG | 21 | 16804 |
| HSV1-UL48-1396 | + | CCGUCGCGGGUGGCCGGAAGCG | 22 | 16805 |
| HSV1-UL48-1397 | + | GCCGUCGCGGGUGGCCGGAAGCG | 23 | 16806 |
| HSV1-UL48-1398 | + | GGCCGUCGCGGGUGGCCGGAAGCG | 24 | 16807 |
| HSV1-UL48-1399 | + | GCCCCGUUGCGUACAGCG | 18 | 16808 |
| HSV1-UL48-1400 | + | CGCCCCGUUGCGUACAGCG | 19 | 16809 |
| HSV1-UL48-97 | + | GCGCCCCGUUGCGUACAGCG | 20 | 5938 |
| HSV1-UL48-1401 | + | GGCGCCCCGUUGCGUACAGCG | 21 | 16810 |
| HSV1-UL48-1402 | + | AGGCGCCCCGUUGCGUACAGCG | 22 | 16811 |
| HSV1-UL48-1403 | + | CAGGCGCCCCGUUGCGUACAGCG | 23 | 16812 |
| HSV1-UL48-1404 | + | UCAGGCGCCCCGUUGCGUACAGCG | 24 | 16813 |
| HSV1-UL48-1405 | + | CGAGCAUGGUACAUAGCG | 18 | 16814 |
| HSV1-UL48-1406 | + | UCGAGCAUGGUACAUAGCG | 19 | 16815 |
| HSV1-UL48-68 | + | AUCGAGCAUGGUACAUAGCG | 20 | 5912 |
| HSV1-UL48-1407 | + | UAUCGAGCAUGGUACAUAGCG | 21 | 16816 |
| HSV1-UL48-1408 | + | GUAUCGAGCAUGGUACAUAGCG | 22 | 16817 |
| HSV1-UL48-1409 | + | GGUAUCGAGCAUGGUACAUAGCG | 23 | 16818 |
| HSV1-UL48-1410 | + | AGGUAUCGAGCAUGGUACAUAGCG | 24 | 16819 |
| HSV1-UL48-1411 | + | CGGCGUUGGUCGGUAGCG | 18 | 16820 |
| HSV1-UL48-1412 | + | UCGGCGUUGGUCGGUAGCG | 19 | 16821 |
| HSV1-UL48-718 | + | GUCGGCGUUGGUCGGUAGCG | 20 | 6508 |
| HSV1-UL48-1413 | + | GGUCGGCGUUGGUCGGUAGCG | 21 | 16822 |
| HSV1-UL48-1414 | + | AGGUCGGCGUUGGUCGGUAGCG | 22 | 16823 |
| HSV1-UL48-1415 | + | CAGGUCGGCGUUGGUCGGUAGCG | 23 | 16824 |
| HSV1-UL48-1416 | + | ACAGGUCGGCGUUGGUCGGUAGCG | 24 | 16825 |
| HSV1-UL48-1417 | + | CGGUUAAAGAGGGCGGCG | 18 | 16826 |
| HSV1-UL48-1418 | + | ACGGUUAAAGAGGGCGGCG | 19 | 16827 |
| HSV1-UL48-78 | + | GACGGUUAAAGAGGGCGGCG | 20 | 5930 |
| HSV1-UL48-1419 | + | AGACGGUUAAAGAGGGCGGCG | 21 | 16828 |
| HSV1-UL48-1420 | + | GAGACGGUUAAAGAGGGCGGCG | 22 | 16829 |
| HSV1-UL48-1421 | + | GGAGACGGUUAAAGAGGGCGGCG | 23 | 16830 |
| HSV1-UL48-1422 | + | AGGAGACGGUUAAAGAGGGCGGCG | 24 | 16831 |
| HSV1-UL48-1423 | + | CGCGCCCGUAGCUCGGCG | 18 | 16832 |
| HSV1-UL48-1424 | + | CCGCGCCCGUAGCUCGGCG | 19 | 16833 |
| HSV1-UL48-42 | + | CCCGCGCCCGUAGCUCGGCG | 20 | 5988 |
| HSV1-UL48-1425 | + | UCCCGCGCCCGUAGCUCGGCG | 21 | 16834 |
| HSV1-UL48-1426 | + | CUCCCGCGCCCGUAGCUCGGCG | 22 | 16835 |
| HSV1-UL48-1427 | + | CCUCCCGCGCCCGUAGCUCGGCG | 23 | 16836 |
| HSV1-UL48-1428 | + | UCCUCCCGCGCCCGUAGCUCGGCG | 24 | 16837 |
| HSV1-UL48-1429 | + | AGACGGUUAAAGAGGGCG | 18 | 16838 |
| HSV1-UL48-1430 | + | GAGACGGUUAAAGAGGGCG | 19 | 16839 |
| HSV1-UL48-709 | + | GGAGACGGUUAAAGAGGGCG | 20 | 6501 |
| HSV1-UL48-1431 | + | AGGAGACGGUUAAAGAGGGCG | 21 | 16840 |
| HSV1-UL48-1432 | + | GAGGAGACGGUUAAAGAGGGCG | 22 | 16841 |
| HSV1-UL48-1433 | + | CGAGGAGACGGUUAAAGAGGGCG | 23 | 16842 |
| HSV1-UL48-1434 | + | UCGAGGAGACGGUUAAAGAGGGCG | 24 | 16843 |
| HSV1-UL48-1435 | + | GGGACCCCCGGCCGGGCG | 18 | 16844 |
| HSV1-UL48-1436 | + | UGGGACCCCCGGCCGGGCG | 19 | 16845 |
| HSV1-UL48-111 | + | UUGGGACCCCCGGCCGGGCG | 20 | 6024 |
| HSV1-UL48-1437 | + | UUUGGGACCCCCGGCCGGGCG | 21 | 16846 |
| HSV1-UL48-1438 | + | UUUUGGGACCCCCGGCCGGGCG | 22 | 16847 |
| HSV1-UL48-1439 | + | UUUUUGGGACCCCCGGCCGGGCG | 23 | 16848 |
| HSV1-UL48-1440 | + | GUUUUUGGGACCCCCGGCCGGGCG | 24 | 16849 |
| HSV1-UL48-1441 | + | CCCCAUUCCACCACAUCG | 18 | 16850 |
| HSV1-UL48-1442 | + | CCCCCAUUCCACCACAUCG | 19 | 16851 |
| HSV1-UL48-721 | + | CCCCCCAUUCCACCACAUCG | 20 | 6510 |
| HSV1-UL48-1443 | + | UCCCCCCAUUCCACCACAUCG | 21 | 16852 |
| HSV1-UL48-1444 | + | GUCCCCCCAUUCCACCACAUCG | 22 | 16853 |
| HSV1-UL48-1445 | + | CGUCCCCCCAUUCCACCACAUCG | 23 | 16854 |
| HSV1-UL48-1446 | + | GCGUCCCCCCAUUCCACCACAUCG | 24 | 16855 |
| HSV1-UL48-1447 | + | GCGCUAAAGCCCAAGUCG | 18 | 16856 |
| HSV1-UL48-1448 | + | CGCGCUAAAGCCCAAGUCG | 19 | 16857 |
| HSV1-UL48-714 | + | CCGCGCUAAAGCCCAAGUCG | 20 | 6505 |
| HSV1-UL48-1449 | + | CCCGCGCUAAAGCCCAAGUCG | 21 | 16858 |
| HSV1-UL48-1450 | + | GCCCGCGCUAAAGCCCAAGUCG | 22 | 16859 |
| HSV1-UL48-1451 | + | GGCCCGCGCUAAAGCCCAAGUCG | 23 | 16860 |
| HSV1-UL48-1452 | + | GGGCCCGCGCUAAAGCCCAAGUCG | 24 | 16861 |
| HSV1-UL48-1453 | + | CUAAAGCCCAAGUCGUCG | 18 | 16862 |
| HSV1-UL48-1454 | + | GCUAAAGCCCAAGUCGUCG | 19 | 16863 |
| HSV1-UL48-71 | + | CGCUAAAGCCCAAGUCGUCG | 20 | 5915 |
| HSV1-UL48-1455 | + | GCGCUAAAGCCCAAGUCGUCG | 21 | 16864 |
| HSV1-UL48-1456 | + | CGCGCUAAAGCCCAAGUCGUCG | 22 | 16865 |
| HSV1-UL48-1457 | + | CCGCGCUAAAGCCCAAGUCGUCG | 23 | 16866 |
| HSV1-UL48-1458 | + | CCCGCGCUAAAGCCCAAGUCGUCG | 24 | 16867 |
| HSV1-UL48-1459 | + | UGUUUUUGGGACCCCCGG | 18 | 16868 |
| HSV1-UL48-1460 | + | GUGUUUUUGGGACCCCCGG | 19 | 16869 |
| HSV1-UL48-685 | + | GGUGUUUUUGGGACCCCCGG | 20 | 6491 |
| HSV1-UL48-1461 | + | GGGUGUUUUUGGGACCCCCGG | 21 | 16870 |
| HSV1-UL48-1462 | + | GGGGUGUUUUUGGGACCCCCGG | 22 | 16871 |
| HSV1-UL48-1463 | + | CGGGGUGUUUUUGGGACCCCCGG | 23 | 16872 |
| HSV1-UL48-1464 | + | CCGGGGUGUUUUUGGGACCCCCGG | 24 | 16873 |
| HSV1-UL48-1465 | + | UUUGGGACCCCCGGCCGG | 18 | 16874 |
| HSV1-UL48-1466 | + | UUUUGGGACCCCCGGCCGG | 19 | 16875 |
| HSV1-UL48-684 | + | UUUUUGGGACCCCCGGCCGG | 20 | 6490 |
| HSV1-UL48-1467 | + | GUUUUUGGGACCCCCGGCCGG | 21 | 16876 |
| HSV1-UL48-1468 | + | UGUUUUUGGGACCCCCGGCCGG | 22 | 16877 |
| HSV1-UL48-1469 | + | GUGUUUUUGGGACCCCCGGCCGG | 23 | 16878 |
| HSV1-UL48-1470 | + | GGUGUUUUUGGGACCCCCGGCCGG | 24 | 16879 |
| HSV1-UL48-1471 | + | CCCCGUUGCGUACAGCGG | 18 | 16880 |
| HSV1-UL48-1472 | + | GCCCCGUUGCGUACAGCGG | 19 | 16881 |
| HSV1-UL48-98 | + | CGCCCCGUUGCGUACAGCGG | 20 | 5939 |
| HSV1-UL48-1473 | + | GCGCCCCGUUGCGUACAGCGG | 21 | 16882 |
| HSV1-UL48-1474 | + | GGCGCCCCGUUGCGUACAGCGG | 22 | 16883 |
| HSV1-UL48-1475 | + | AGGCGCCCCGUUGCGUACAGCGG | 23 | 16884 |
| HSV1-UL48-1476 | + | CAGGCGCCCCGUUGCGUACAGCGG | 24 | 16885 |
| HSV1-UL48-1477 | + | GGUUAAAGAGGGCGGCGG | 18 | 16886 |
| HSV1-UL48-1478 | + | CGGUUAAAGAGGGCGGCGG | 19 | 16887 |
| HSV1-UL48-79 | + | ACGGUUAAAGAGGGCGGCGG | 20 | 5931 |
| HSV1-UL48-1479 | + | GACGGUUAAAGAGGGCGGCGG | 21 | 16888 |
| HSV1-UL48-1480 | + | AGACGGUUAAAGAGGGCGGCGG | 22 | 16889 |
| HSV1-UL48-1481 | + | GAGACGGUUAAAGAGGGCGGCGG | 23 | 16890 |
| HSV1-UL48-1482 | + | GGAGACGGUUAAAGAGGGCGGCGG | 24 | 16891 |
| HSV1-UL48-1483 | + | GACGGUUAAAGAGGGCGG | 18 | 16892 |
| HSV1-UL48-1484 | + | AGACGGUUAAAGAGGGCGG | 19 | 16893 |
| HSV1-UL48-76 | + | GAGACGGUUAAAGAGGGCGG | 20 | 5928 |
| HSV1-UL48-1485 | + | GGAGACGGUUAAAGAGGGCGG | 21 | 16894 |
| HSV1-UL48-1486 | + | AGGAGACGGUUAAAGAGGGCGG | 22 | 16895 |
| HSV1-UL48-1487 | + | GAGGAGACGGUUAAAGAGGGCGG | 23 | 16896 |
| HSV1-UL48-1488 | + | CGAGGAGACGGUUAAAGAGGGCGG | 24 | 16897 |
| HSV1-UL48-1489 | + | GGACCCCCGGCCGGGCGG | 18 | 16898 |
| HSV1-UL48-1490 | + | GGGACCCCCGGCCGGGCGG | 19 | 16899 |
| HSV1-UL48-112 | + | UGGGACCCCCGGCCGGGCGG | 20 | 6025 |
| HSV1-UL48-1491 | + | UUGGGACCCCCGGCCGGGCGG | 21 | 16900 |
| HSV1-UL48-1492 | + | UUUGGGACCCCCGGCCGGGCGG | 22 | 16901 |
| HSV1-UL48-1493 | + | UUUUGGGACCCCCGGCCGGGCGG | 23 | 16902 |
| HSV1-UL48-1494 | + | UUUUUGGGACCCCCGGCCGGGCGG | 24 | 16903 |
| HSV1-UL48-1495 | + | UUGGGACCCCCGGCCGGG | 18 | 16904 |
| HSV1-UL48-1496 | + | UUUGGGACCCCCGGCCGGG | 19 | 16905 |
| HSV1-UL48-109 | + | UUUUGGGACCCCCGGCCGGG | 20 | 6022 |
| HSV1-UL48-1497 | + | UUUUUGGGACCCCCGGCCGGG | 21 | 16906 |
| HSV1-UL48-1498 | + | GUUUUUGGGACCCCCGGCCGGG | 22 | 16907 |
| HSV1-UL48-1499 | + | UGUUUUUGGGACCCCCGGCCGGG | 23 | 16908 |
| HSV1-UL48-1500 | + | GUGUUUUUGGGACCCCCGGCCGGG | 24 | 16909 |
| HSV1-UL48-1501 | + | GUUAAAGAGGGCGGCGGG | 18 | 16910 |
| HSV1-UL48-1502 | + | GGUUAAAGAGGGCGGCGGG | 19 | 16911 |
| HSV1-UL48-80 | + | CGGUUAAAGAGGGCGGCGGG | 20 | 5932 |
| HSV1-UL48-1503 | + | ACGGUUAAAGAGGGCGGCGGG | 21 | 16912 |
| HSV1-UL48-1504 | + | GACGGUUAAAGAGGGCGGCGGG | 22 | 16913 |
| HSV1-UL48-1505 | + | AGACGGUUAAAGAGGGCGGCGGG | 23 | 16914 |
| HSV1-UL48-1506 | + | GAGACGGUUAAAGAGGGCGGCGGG | 24 | 16915 |
| HSV1-UL48-1507 | + | AAGAGGGCGGCGGGGGGG | 18 | 16916 |
| HSV1-UL48-1508 | + | AAAGAGGGCGGCGGGGGGG | 19 | 16917 |
| HSV1-UL48-703 | + | UAAAGAGGGCGGCGGGGGGG | 20 | 6500 |
| HSV1-UL48-1509 | + | UUAAAGAGGGCGGCGGGGGGG | 21 | 16918 |
| HSV1-UL48-1510 | + | GUUAAAGAGGGCGGCGGGGGGG | 22 | 16919 |
| HSV1-UL48-1511 | + | GGUUAAAGAGGGCGGCGGGGGGG | 23 | 16920 |
| HSV1-UL48-1512 | + | CGGUUAAAGAGGGCGGCGGGGGGG | 24 | 16921 |
| HSV1-UL48-1513 | + | GGGGGGGACGGGCAUGGG | 18 | 16922 |
| HSV1-UL48-1514 | + | CGGGGGGGACGGGCAUGGG | 19 | 16923 |
| HSV1-UL48-86 | + | GCGGGGGGGACGGGCAUGGG | 20 | 6009 |
| HSV1-UL48-1515 | + | GGCGGGGGGGACGGGCAUGGG | 21 | 16924 |
| HSV1-UL48-1516 | + | CGGCGGGGGGGACGGGCAUGGG | 22 | 16925 |
| HSV1-UL48-1517 | + | GCGGCGGGGGGGACGGGCAUGGG | 23 | 16926 |
| HSV1-UL48-1518 | + | GGCGGCGGGGGGGACGGGCAUGGG | 24 | 16927 |
| HSV1-UL48-1519 | + | GGGACGGGCAUGGGUGGG | 18 | 16928 |
| HSV1-UL48-1520 | + | GGGGACGGGCAUGGGUGGG | 19 | 16929 |
| HSV1-UL48-697 | + | GGGGGACGGGCAUGGGUGGG | 20 | 6497 |
| HSV1-UL48-1521 | + | GGGGGGACGGGCAUGGGUGGG | 21 | 16930 |
| HSV1-UL48-1522 | + | GGGGGGGACGGGCAUGGGUGGG | 22 | 16931 |
| HSV1-UL48-1523 | + | CGGGGGGGACGGGCAUGGGUGGG | 23 | 16932 |
| HSV1-UL48-1524 | + | GCGGGGGGGACGGGCAUGGGUGGG | 24 | 16933 |
| HSV1-UL48-1525 | + | CGGGGGGGACGGGCAUGG | 18 | 16934 |
| HSV1-UL48-1526 | + | GCGGGGGGGACGGGCAUGG | 19 | 16935 |
| HSV1-UL48-701 | + | GGCGGGGGGGACGGGCAUGG | 20 | 6498 |
| HSV1-UL48-1527 | + | CGGCGGGGGGGACGGGCAUGG | 21 | 16936 |
| HSV1-UL48-1528 | + | GCGGCGGGGGGGACGGGCAUGG | 22 | 16937 |
| HSV1-UL48-1529 | + | GGCGGCGGGGGGGACGGGCAUGG | 23 | 16938 |
| HSV1-UL48-1530 | + | GGGCGGCGGGGGGGACGGGCAUGG | 24 | 16939 |
| HSV1-UL48-1531 | + | GCCCGUAGCUCGGCGUGG | 18 | 16940 |
| HSV1-UL48-1532 | + | CGCCCGUAGCUCGGCGUGG | 19 | 16941 |
| HSV1-UL48-739 | + | GCGCCCGUAGCUCGGCGUGG | 20 | 6523 |
| HSV1-UL48-1533 | + | CGCGCCCGUAGCUCGGCGUGG | 21 | 16942 |
| HSV1-UL48-1534 | + | CCGCGCCCGUAGCUCGGCGUGG | 22 | 16943 |
| HSV1-UL48-1535 | + | CCCGCGCCCGUAGCUCGGCGUGG | 23 | 16944 |
| HSV1-UL48-1536 | + | UCCCGCGCCCGUAGCUCGGCGUGG | 24 | 16945 |
| HSV1-UL48-1537 | + | CGAGGCCGUCGCGGGUGG | 18 | 16946 |
| HSV1-UL48-1538 | + | CCGAGGCCGUCGCGGGUGG | 19 | 16947 |
| HSV1-UL48-732 | + | CCCGAGGCCGUCGCGGGUGG | 20 | 6516 |
| HSV1-UL48-1539 | + | GCCCGAGGCCGUCGCGGGUGG | 21 | 16948 |
| HSV1-UL48-1540 | + | AGCCCGAGGCCGUCGCGGGUGG | 22 | 16949 |
| HSV1-UL48-1541 | + | GAGCCCGAGGCCGUCGCGGGUGG | 23 | 16950 |
| HSV1-UL48-1542 | + | AGAGCCCGAGGCCGUCGCGGGUGG | 24 | 16951 |
| HSV1-UL48-1543 | + | GACCAAGAGGUCCAUUGG | 18 | 16952 |
| HSV1-UL48-1544 | + | CGACCAAGAGGUCCAUUGG | 19 | 16953 |
| HSV1-UL48-1545 | + | UCGACCAAGAGGUCCAUUGG | 20 | 16954 |
| HSV1-UL48-1546 | + | GUCGACCAAGAGGUCCAUUGG | 21 | 16955 |
| HSV1-UL48-1547 | + | CGUCGACCAAGAGGUCCAUUGG | 22 | 16956 |
| HSV1-UL48-1548 | + | UCGUCGACCAAGAGGUCCAUUGG | 23 | 16957 |
| HSV1-UL48-1549 | + | CUCGUCGACCAAGAGGUCCAUUGG | 24 | 16958 |
| HSV1-UL48-1550 | + | CCGGGCGGGGGGGCGGUG | 18 | 16959 |
| HSV1-UL48-1551 | + | GCCGGGCGGGGGGGCGGUG | 19 | 16960 |
| HSV1-UL48-679 | + | GGCCGGGCGGGGGGGCGGUG | 20 | 6489 |
| HSV1-UL48-1552 | + | CGGCCGGGCGGGGGGGCGGUG | 21 | 16961 |
| HSV1-UL48-1553 | + | CCGGCCGGGCGGGGGGGCGGUG | 22 | 16962 |
| HSV1-UL48-1554 | + | CCCGGCCGGGCGGGGGGGCGGUG | 23 | 16963 |
| HSV1-UL48-1555 | + | CCCCGGCCGGGCGGGGGGGCGGUG | 24 | 16964 |
| HSV1-UL48-1556 | + | GGGGGACGGGCAUGGGUG | 18 | 16965 |
| HSV1-UL48-1557 | + | GGGGGGACGGGCAUGGGUG | 19 | 16966 |
| HSV1-UL48-88 | + | GGGGGGGACGGGCAUGGGUG | 20 | 6011 |
| HSV1-UL48-1558 | + | CGGGGGGGACGGGCAUGGGUG | 21 | 16967 |
| HSV1-UL48-1559 | + | GCGGGGGGGACGGGCAUGGGUG | 22 | 16968 |
| HSV1-UL48-1560 | + | GGCGGGGGGGACGGGCAUGGGUG | 23 | 16969 |
| HSV1-UL48-1561 | + | CGGCGGGGGGGACGGGCAUGGGUG | 24 | 16970 |
| HSV1-UL48-1562 | + | CCAUUGGGUGGGGUUGAU | 18 | 16971 |
| HSV1-UL48-1563 | + | UCCAUUGGGUGGGGUUGAU | 19 | 16972 |
| HSV1-UL48-1564 | + | GUCCAUUGGGUGGGGUUGAU | 20 | 16973 |
| HSV1-UL48-1565 | + | GGUCCAUUGGGUGGGGUUGAU | 21 | 16974 |
| HSV1-UL48-1566 | + | AGGUCCAUUGGGUGGGGUUGAU | 22 | 16975 |
| HSV1-UL48-1567 | + | GAGGUCCAUUGGGUGGGGUUGAU | 23 | 16976 |
| HSV1-UL48-1568 | + | AGAGGUCCAUUGGGUGGGGUUGAU | 24 | 16977 |
| HSV1-UL48-1569 | + | GCGGGUGGCCGGAAGCGU | 18 | 16978 |
| HSV1-UL48-1570 | + | CGCGGGUGGCCGGAAGCGU | 19 | 16979 |
| HSV1-UL48-48 | + | UCGCGGGUGGCCGGAAGCGU | 20 | 5896 |
| HSV1-UL48-1571 | + | GUCGCGGGUGGCCGGAAGCGU | 21 | 16980 |
| HSV1-UL48-1572 | + | CGUCGCGGGUGGCCGGAAGCGU | 22 | 16981 |
| HSV1-UL48-1573 | + | CCGUCGCGGGUGGCCGGAAGCGU | 23 | 16982 |
| HSV1-UL48-1574 | + | GCCGUCGCGGGUGGCCGGAAGCGU | 24 | 16983 |
| HSV1-UL48-1575 | + | AUGUCGAUUUGGGUGCGU | 18 | 16984 |
| HSV1-UL48-1576 | + | AAUGUCGAUUUGGGUGCGU | 19 | 16985 |
| HSV1-UL48-724 | + | GAAUGUCGAUUUGGGUGCGU | 20 | 6511 |
| HSV1-UL48-1577 | + | CGAAUGUCGAUUUGGGUGCGU | 21 | 16986 |
| HSV1-UL48-1578 | + | GCGAAUGUCGAUUUGGGUGCGU | 22 | 16987 |
| HSV1-UL48-1579 | + | CGCGAAUGUCGAUUUGGGUGCGU | 23 | 16988 |
| HSV1-UL48-1580 | + | GCGCGAAUGUCGAUUUGGGUGCGU | 24 | 16989 |
| HSV1-UL48-1581 | + | GGGGGGACGGGCAUGGGU | 18 | 16990 |
| HSV1-UL48-1582 | + | GGGGGGGACGGGCAUGGGU | 19 | 16991 |
| HSV1-UL48-87 | + | CGGGGGGGACGGGCAUGGGU | 20 | 6010 |
| HSV1-UL48-1583 | + | GCGGGGGGGACGGGCAUGGGU | 21 | 16992 |
| HSV1-UL48-1584 | + | GGCGGGGGGGACGGGCAUGGGU | 22 | 16993 |
| HSV1-UL48-1585 | + | CGGCGGGGGGGACGGGCAUGGGU | 23 | 16994 |
| HSV1-UL48-1586 | + | GCGGCGGGGGGGACGGGCAUGGGU | 24 | 16995 |
| HSV1-UL48-1587 | + | UGUCGAUUUGGGUGCGUU | 18 | 16996 |
| HSV1-UL48-1588 | + | AUGUCGAUUUGGGUGCGUU | 19 | 16997 |
| HSV1-UL48-55 | + | AAUGUCGAUUUGGGUGCGUU | 20 | 5994 |
| HSV1-UL48-1589 | + | GAAUGUCGAUUUGGGUGCGUU | 21 | 16998 |
| HSV1-UL48-1590 | + | CGAAUGUCGAUUUGGGUGCGUU | 22 | 16999 |
| HSV1-UL48-1591 | + | GCGAAUGUCGAUUUGGGUGCGUU | 23 | 17000 |
| HSV1-UL48-1592 | + | CGCGAAUGUCGAUUUGGGUGCGUU | 24 | 17001 |
| HSV1-UL48-1593 | + | GGGGCCGCCGGGGUGUUU | 18 | 17002 |
| HSV1-UL48-1594 | + | GGGGGCCGCCGGGGUGUUU | 19 | 17003 |
| HSV1-UL48-687 | + | GGGGGGCCGCCGGGGUGUUU | 20 | 6492 |
| HSV1-UL48-1595 | + | GGGGGGGCCGCCGGGGUGUUU | 21 | 17004 |
| HSV1-UL48-1596 | + | CGGGGGGGCCGCCGGGGUGUUU | 22 | 17005 |
| HSV1-UL48-1597 | + | GCGGGGGGGCCGCCGGGGUGUUU | 23 | 17006 |
| HSV1-UL48-1598 | + | AGCGGGGGGGCCGCCGGGGUGUUU | 24 | 17007 |
| HSV1-UL48-1599 | + | GGGCCGCCGGGGUGUUUU | 18 | 17008 |
| HSV1-UL48-1600 | + | GGGGCCGCCGGGGUGUUUU | 19 | 17009 |
| HSV1-UL48-104 | + | GGGGGCCGCCGGGGUGUUUU | 20 | 5948 |
| HSV1-UL48-1601 | + | GGGGGGCCGCCGGGGUGUUUU | 21 | 17010 |
| HSV1-UL48-1602 | + | GGGGGGGCCGCCGGGGUGUUUU | 22 | 17011 |
| HSV1-UL48-1603 | + | CGGGGGGGCCGCCGGGGUGUUUU | 23 | 17012 |
| HSV1-UL48-1604 | + | GCGGGGGGGCCGCCGGGGUGUUUU | 24 | 17013 |
| HSV1-UL48-1605 | - | CCCCCCCGCUGUACGCAA | 18 | 17014 |
| HSV1-UL48-1606 | - | GCCCCCCCGCUGUACGCAA | 19 | 17015 |
| HSV1-UL48-10 | - | GGCCCCCCCGCUGUACGCAA | 20 | 5840 |
| HSV1-UL48-1607 | - | CGGCCCCCCCGCUGUACGCAA | 21 | 17016 |
| HSV1-UL48-1608 | - | GCGGCCCCCCCGCUGUACGCAA | 22 | 17017 |
| HSV1-UL48-1609 | - | GGCGGCCCCCCCGCUGUACGCAA | 23 | 17018 |
| HSV1-UL48-1610 | - | CGGCGGCCCCCCCGCUGUACGCAA | 24 | 17019 |
| HSV1-UL48-1611 | - | CCCAGCGAUGUGGUGGAA | 18 | 17020 |
| HSV1-UL48-1612 | - | GCCCAGCGAUGUGGUGGAA | 19 | 17021 |
| HSV1-UL48-25 | - | UGCCCAGCGAUGUGGUGGAA | 20 | 5864 |
| HSV1-UL48-1613 | - | CUGCCCAGCGAUGUGGUGGAA | 21 | 17022 |
| HSV1-UL48-1614 | - | GCUGCCCAGCGAUGUGGUGGAA | 22 | 17023 |
| HSV1-UL48-1615 | - | CGCUGCCCAGCGAUGUGGUGGAA | 23 | 17024 |
| HSV1-UL48-1616 | - | ACGCUGCCCAGCGAUGUGGUGGAA | 24 | 17025 |
| HSV1-UL48-1617 | - | CCGUAUCAACCCCACCCA | 18 | 17026 |
| HSV1-UL48-1618 | - | CCCGUAUCAACCCCACCCA | 19 | 17027 |
| HSV1-UL48-1619 | - | UCCCGUAUCAACCCCACCCA | 20 | 17028 |
| HSV1-UL48-1620 | - | UUCCCGUAUCAACCCCACCCA | 21 | 17029 |
| HSV1-UL48-1621 | - | UUUCCCGUAUCAACCCCACCCA | 22 | 17030 |
| HSV1-UL48-1622 | - | CUUUCCCGUAUCAACCCCACCCA | 23 | 17031 |
| HSV1-UL48-1623 | - | UCUUUCCCGUAUCAACCCCACCCA | 24 | 17032 |
| HSV1-UL48-1624 | - | GCCCCCCCGCUGUACGCA | 18 | 17033 |
| HSV1-UL48-1625 | - | GGCCCCCCCGCUGUACGCA | 19 | 17034 |
| HSV1-UL48-613 | - | CGGCCCCCCCGCUGUACGCA | 20 | 6449 |
| HSV1-UL48-1626 | - | GCGGCCCCCCCGCUGUACGCA | 21 | 17035 |
| HSV1-UL48-1627 | - | GGCGGCCCCCCCGCUGUACGCA | 22 | 17036 |
| HSV1-UL48-1628 | - | CGGCGGCCCCCCCGCUGUACGCA | 23 | 17037 |
| HSV1-UL48-1629 | - | CCGGCGGCCCCCCCGCUGUACGCA | 24 | 17038 |
| HSV1-UL48-1630 | - | CCAUGCUCGAUACCUGGA | 18 | 17039 |
| HSV1-UL48-1631 | - | ACCAUGCUCGAUACCUGGA | 19 | 17040 |
| HSV1-UL48-620 | - | UACCAUGCUCGAUACCUGGA | 20 | 6454 |
| HSV1-UL48-1632 | - | GUACCAUGCUCGAUACCUGGA | 21 | 17041 |
| HSV1-UL48-1633 | - | UGUACCAUGCUCGAUACCUGGA | 22 | 17042 |
| HSV1-UL48-1634 | - | AUGUACCAUGCUCGAUACCUGGA | 23 | 17043 |
| HSV1-UL48-1635 | - | UAUGUACCAUGCUCGAUACCUGGA | 24 | 17044 |
| HSV1-UL48-1636 | - | GCCCAGCGAUGUGGUGGA | 18 | 17045 |
| HSV1-UL48-1637 | - | UGCCCAGCGAUGUGGUGGA | 19 | 17046 |
| HSV1-UL48-627 | - | CUGCCCAGCGAUGUGGUGGA | 20 | 6458 |
| HSV1-UL48-1638 | - | GCUGCCCAGCGAUGUGGUGGA | 21 | 17047 |
| HSV1-UL48-1639 | - | CGCUGCCCAGCGAUGUGGUGGA | 22 | 17048 |
| HSV1-UL48-1640 | - | ACGCUGCCCAGCGAUGUGGUGGA | 23 | 17049 |
| HSV1-UL48-1641 | - | AACGCUGCCCAGCGAUGUGGUGGA | 24 | 17050 |
| HSV1-UL48-1642 | - | GGCGCGGGAGGAGAGCUA | 18 | 17051 |
| HSV1-UL48-1643 | - | GGGCGCGGGAGGAGAGCUA | 19 | 17052 |
| HSV1-UL48-1644 | - | CGGGCGCGGGAGGAGAGCUA | 20 | 17053 |
| HSV1-UL48-1645 | - | ACGGGCGCGGGAGGAGAGCUA | 21 | 17054 |
| HSV1-UL48-1646 | - | UACGGGCGCGGGAGGAGAGCUA | 22 | 17055 |
| HSV1-UL48-1647 | - | CUACGGGCGCGGGAGGAGAGCUA | 23 | 17056 |
| HSV1-UL48-1648 | - | GCUACGGGCGCGGGAGGAGAGCUA | 24 | 17057 |
| HSV1-UL48-1649 | - | GACCAACGCCGACCUGUA | 18 | 17058 |
| HSV1-UL48-1650 | - | CGACCAACGCCGACCUGUA | 19 | 17059 |
| HSV1-UL48-622 | - | CCGACCAACGCCGACCUGUA | 20 | 6456 |
| HSV1-UL48-1651 | - | ACCGACCAACGCCGACCUGUA | 21 | 17060 |
| HSV1-UL48-1652 | - | UACCGACCAACGCCGACCUGUA | 22 | 17061 |
| HSV1-UL48-1653 | - | CUACCGACCAACGCCGACCUGUA | 23 | 17062 |
| HSV1-UL48-1654 | - | GCUACCGACCAACGCCGACCUGUA | 24 | 17063 |
| HSV1-UL48-1655 | - | CUGUUUGCCGACAUGAAC | 18 | 17064 |
| HSV1-UL48-1656 | - | GCUGUUUGCCGACAUGAAC | 19 | 17065 |
| HSV1-UL48-609 | - | AGCUGUUUGCCGACAUGAAC | 20 | 6447 |
| HSV1-UL48-1657 | - | GAGCUGUUUGCCGACAUGAAC | 21 | 17066 |
| HSV1-UL48-1658 | - | CGAGCUGUUUGCCGACAUGAAC | 22 | 17067 |
| HSV1-UL48-1659 | - | ACGAGCUGUUUGCCGACAUGAAC | 23 | 17068 |
| HSV1-UL48-1660 | - | GACGAGCUGUUUGCCGACAUGAAC | 24 | 17069 |
| HSV1-UL48-1661 | - | AACCGUCUCCUCGACGAC | 18 | 17070 |
| HSV1-UL48-1662 | - | UAACCGUCUCCUCGACGAC | 19 | 17071 |
| HSV1-UL48-616 | - | UUAACCGUCUCCUCGACGAC | 20 | 6451 |
| HSV1-UL48-1663 | - | UUUAACCGUCUCCUCGACGAC | 21 | 17072 |
| HSV1-UL48-1664 | - | CUUUAACCGUCUCCUCGACGAC | 22 | 17073 |
| HSV1-UL48-1665 | - | UCUUUAACCGUCUCCUCGACGAC | 23 | 17074 |
| HSV1-UL48-1666 | - | CUCUUUAACCGUCUCCUCGACGAC | 24 | 17075 |
| HSV1-UL48-1667 | - | GACGAGCUGUUUGCCGAC | 18 | 17076 |
| HSV1-UL48-1668 | - | CGACGAGCUGUUUGCCGAC | 19 | 17077 |
| HSV1-UL48-608 | - | UCGACGAGCUGUUUGCCGAC | 20 | 6446 |
| HSV1-UL48-1669 | - | GUCGACGAGCUGUUUGCCGAC | 21 | 17078 |
| HSV1-UL48-1670 | - | GGUCGACGAGCUGUUUGCCGAC | 22 | 17079 |
| HSV1-UL48-1671 | - | UGGUCGACGAGCUGUUUGCCGAC | 23 | 17080 |
| HSV1-UL48-1672 | - | UUGGUCGACGAGCUGUUUGCCGAC | 24 | 17081 |
| HSV1-UL48-1673 | - | AUGUACCAUGCUCGAUAC | 18 | 17082 |
| HSV1-UL48-1674 | - | UAUGUACCAUGCUCGAUAC | 19 | 17083 |
| HSV1-UL48-618 | - | CUAUGUACCAUGCUCGAUAC | 20 | 6453 |
| HSV1-UL48-1675 | - | GCUAUGUACCAUGCUCGAUAC | 21 | 17084 |
| HSV1-UL48-1676 | - | CGCUAUGUACCAUGCUCGAUAC | 22 | 17085 |
| HSV1-UL48-1677 | - | GCGCUAUGUACCAUGCUCGAUAC | 23 | 17086 |
| HSV1-UL48-1678 | - | CGCGCUAUGUACCAUGCUCGAUAC | 24 | 17087 |
| HSV1-UL48-1679 | - | ACCAACGCCGACCUGUAC | 18 | 17088 |
| HSV1-UL48-1680 | - | GACCAACGCCGACCUGUAC | 19 | 17089 |
| HSV1-UL48-21 | - | CGACCAACGCCGACCUGUAC | 20 | 5860 |
| HSV1-UL48-1681 | - | CCGACCAACGCCGACCUGUAC | 21 | 17090 |
| HSV1-UL48-1682 | - | ACCGACCAACGCCGACCUGUAC | 22 | 17091 |
| HSV1-UL48-1683 | - | UACCGACCAACGCCGACCUGUAC | 23 | 17092 |
| HSV1-UL48-1684 | - | CUACCGACCAACGCCGACCUGUAC | 24 | 17093 |
| HSV1-UL48-1685 | - | UGUACCAUGCUCGAUACC | 18 | 17094 |
| HSV1-UL48-1686 | - | AUGUACCAUGCUCGAUACC | 19 | 17095 |
| HSV1-UL48-18 | - | UAUGUACCAUGCUCGAUACC | 20 | 5967 |
| HSV1-UL48-1687 | - | CUAUGUACCAUGCUCGAUACC | 21 | 17096 |
| HSV1-UL48-1688 | - | GCUAUGUACCAUGCUCGAUACC | 22 | 17097 |
| HSV1-UL48-1689 | - | CGCUAUGUACCAUGCUCGAUACC | 23 | 17098 |
| HSV1-UL48-1690 | - | GCGCUAUGUACCAUGCUCGAUACC | 24 | 17099 |
| HSV1-UL48-1691 | - | CGGCCACCCGCGACGGCC | 18 | 17100 |
| HSV1-UL48-1692 | - | CCGGCCACCCGCGACGGCC | 19 | 17101 |
| HSV1-UL48-633 | - | UCCGGCCACCCGCGACGGCC | 20 | 6460 |
| HSV1-UL48-1693 | - | UUCCGGCCACCCGCGACGGCC | 21 | 17102 |
| HSV1-UL48-1694 | - | CUUCCGGCCACCCGCGACGGCC | 22 | 17103 |
| HSV1-UL48-1695 | - | GCUUCCGGCCACCCGCGACGGCC | 23 | 17104 |
| HSV1-UL48-1696 | - | CGCUUCCGGCCACCCGCGACGGCC | 24 | 17105 |
| HSV1-UL48-1697 | - | GGGGGGACGCGUACGUCC | 18 | 17106 |
| HSV1-UL48-1698 | - | UGGGGGGACGCGUACGUCC | 19 | 17107 |
| HSV1-UL48-632 | - | AUGGGGGGACGCGUACGUCC | 20 | 6459 |
| HSV1-UL48-1699 | - | AAUGGGGGGACGCGUACGUCC | 21 | 17108 |
| HSV1-UL48-1700 | - | GAAUGGGGGGACGCGUACGUCC | 22 | 17109 |
| HSV1-UL48-1701 | - | GGAAUGGGGGGACGCGUACGUCC | 23 | 17110 |
| HSV1-UL48-1702 | - | UGGAAUGGGGGGACGCGUACGUCC | 24 | 17111 |
| HSV1-UL48-1703 | - | GACGACUUGGGCUUUAGC | 18 | 17112 |
| HSV1-UL48-1704 | - | CGACGACUUGGGCUUUAGC | 19 | 17113 |
| HSV1-UL48-617 | - | UCGACGACUUGGGCUUUAGC | 20 | 6452 |
| HSV1-UL48-1705 | - | CUCGACGACUUGGGCUUUAGC | 21 | 17114 |
| HSV1-UL48-1706 | - | CCUCGACGACUUGGGCUUUAGC | 22 | 17115 |
| HSV1-UL48-1707 | - | UCCUCGACGACUUGGGCUUUAGC | 23 | 17116 |
| HSV1-UL48-1708 | - | CUCCUCGACGACUUGGGCUUUAGC | 24 | 17117 |
| HSV1-UL48-1709 | - | ACGCCGAGCUACGGGCGC | 18 | 17118 |
| HSV1-UL48-1710 | - | CACGCCGAGCUACGGGCGC | 19 | 17119 |
| HSV1-UL48-39 | - | CCACGCCGAGCUACGGGCGC | 20 | 5880 |
| HSV1-UL48-1711 | - | UCCACGCCGAGCUACGGGCGC | 21 | 17120 |
| HSV1-UL48-1712 | - | UUCCACGCCGAGCUACGGGCGC | 22 | 17121 |
| HSV1-UL48-1713 | - | CUUCCACGCCGAGCUACGGGCGC | 23 | 17122 |
| HSV1-UL48-1714 | - | UCUUCCACGCCGAGCUACGGGCGC | 24 | 17123 |
| HSV1-UL48-1715 | - | CUGUACGCAACGGGGCGC | 18 | 17124 |
| HSV1-UL48-1716 | - | GCUGUACGCAACGGGGCGC | 19 | 17125 |
| HSV1-UL48-615 | - | CGCUGUACGCAACGGGGCGC | 20 | 6450 |
| HSV1-UL48-1717 | - | CCGCUGUACGCAACGGGGCGC | 21 | 17126 |
| HSV1-UL48-1718 | - | CCCGCUGUACGCAACGGGGCGC | 22 | 17127 |
| HSV1-UL48-1719 | - | CCCCGCUGUACGCAACGGGGCGC | 23 | 17128 |
| HSV1-UL48-1720 | - | CCCCCGCUGUACGCAACGGGGCGC | 24 | 17129 |
| HSV1-UL48-1721 | - | ACCGCCCCCCCGCCCGGC | 18 | 17130 |
| HSV1-UL48-1722 | - | CACCGCCCCCCCGCCCGGC | 19 | 17131 |
| HSV1-UL48-4 | - | CCACCGCCCCCCCGCCCGGC | 20 | 5963 |
| HSV1-UL48-1723 | - | GCCACCGCCCCCCCGCCCGGC | 21 | 17132 |
| HSV1-UL48-1724 | - | CGCCACCGCCCCCCCGCCCGGC | 22 | 17133 |
| HSV1-UL48-1725 | - | UCGCCACCGCCCCCCCGCCCGGC | 23 | 17134 |
| HSV1-UL48-1726 | - | UUCGCCACCGCCCCCCCGCCCGGC | 24 | 17135 |
| HSV1-UL48-1727 | - | CCACGCCGAGCUACGGGC | 18 | 17136 |
| HSV1-UL48-1728 | - | UCCACGCCGAGCUACGGGC | 19 | 17137 |
| HSV1-UL48-637 | - | UUCCACGCCGAGCUACGGGC | 20 | 6464 |
| HSV1-UL48-1729 | - | CUUCCACGCCGAGCUACGGGC | 21 | 17138 |
| HSV1-UL48-1730 | - | UCUUCCACGCCGAGCUACGGGC | 22 | 17139 |
| HSV1-UL48-1731 | - | UUCUUCCACGCCGAGCUACGGGC | 23 | 17140 |
| HSV1-UL48-1732 | - | UUUCUUCCACGCCGAGCUACGGGC | 24 | 17141 |
| HSV1-UL48-1733 | - | GAGCUACGGGCGCGGGAG | 18 | 17142 |
| HSV1-UL48-1734 | - | CGAGCUACGGGCGCGGGAG | 19 | 17143 |
| HSV1-UL48-642 | - | CCGAGCUACGGGCGCGGGAG | 20 | 6466 |
| HSV1-UL48-1735 | - | GCCGAGCUACGGGCGCGGGAG | 21 | 17144 |
| HSV1-UL48-1736 | - | CGCCGAGCUACGGGCGCGGGAG | 22 | 17145 |
| HSV1-UL48-1737 | - | ACGCCGAGCUACGGGCGCGGGAG | 23 | 17146 |
| HSV1-UL48-1738 | - | CACGCCGAGCUACGGGCGCGGGAG | 24 | 17147 |
| HSV1-UL48-1739 | - | UCUCUCGUUUCUUCCACG | 18 | 17148 |
| HSV1-UL48-1740 | - | CUCUCUCGUUUCUUCCACG | 19 | 17149 |
| HSV1-UL48-635 | - | GCUCUCUCGUUUCUUCCACG | 20 | 6462 |
| HSV1-UL48-1741 | - | CGCUCUCUCGUUUCUUCCACG | 21 | 17150 |
| HSV1-UL48-1742 | - | GCGCUCUCUCGUUUCUUCCACG | 22 | 17151 |
| HSV1-UL48-1743 | - | AGCGCUCUCUCGUUUCUUCCACG | 23 | 17152 |
| HSV1-UL48-1744 | - | AAGCGCUCUCUCGUUUCUUCCACG | 24 | 17153 |
| HSV1-UL48-1745 | - | CACGCCGAGCUACGGGCG | 18 | 17154 |
| HSV1-UL48-1746 | - | CCACGCCGAGCUACGGGCG | 19 | 17155 |
| HSV1-UL48-38 | - | UCCACGCCGAGCUACGGGCG | 20 | 5879 |
| HSV1-UL48-1747 | - | UUCCACGCCGAGCUACGGGCG | 21 | 17156 |
| HSV1-UL48-1748 | - | CUUCCACGCCGAGCUACGGGCG | 22 | 17157 |
| HSV1-UL48-1749 | - | UCUUCCACGCCGAGCUACGGGCG | 23 | 17158 |
| HSV1-UL48-1750 | - | UUCUUCCACGCCGAGCUACGGGCG | 24 | 17159 |
| HSV1-UL48-1751 | - | CAAUGGACCUCUUGGUCG | 18 | 17160 |
| HSV1-UL48-1752 | - | CCAAUGGACCUCUUGGUCG | 19 | 17161 |
| HSV1-UL48-1753 | - | CCCAAUGGACCUCUUGGUCG | 20 | 17162 |
| HSV1-UL48-1754 | - | ACCCAAUGGACCUCUUGGUCG | 21 | 17163 |
| HSV1-UL48-1755 | - | CACCCAAUGGACCUCUUGGUCG | 22 | 17164 |
| HSV1-UL48-1756 | - | CCACCCAAUGGACCUCUUGGUCG | 23 | 17165 |
| HSV1-UL48-1757 | - | CCCACCCAAUGGACCUCUUGGUCG | 24 | 17166 |
| HSV1-UL48-1758 | - | CACCGCCCCCCCGCCCGG | 18 | 17167 |
| HSV1-UL48-1759 | - | CCACCGCCCCCCCGCCCGG | 19 | 17168 |
| HSV1-UL48-610 | - | GCCACCGCCCCCCCGCCCGG | 20 | 6448 |
| HSV1-UL48-1760 | - | CGCCACCGCCCCCCCGCCCGG | 21 | 17169 |
| HSV1-UL48-1761 | - | UCGCCACCGCCCCCCCGCCCGG | 22 | 17170 |
| HSV1-UL48-1762 | - | UUCGCCACCGCCCCCCCGCCCGG | 23 | 17171 |
| HSV1-UL48-1763 | - | CUUCGCCACCGCCCCCCCGCCCGG | 24 | 17172 |
| HSV1-UL48-1764 | - | GCCGAGCUACGGGCGCGG | 18 | 17173 |
| HSV1-UL48-1765 | - | CGCCGAGCUACGGGCGCGG | 19 | 17174 |
| HSV1-UL48-640 | - | ACGCCGAGCUACGGGCGCGG | 20 | 6465 |
| HSV1-UL48-1766 | - | CACGCCGAGCUACGGGCGCGG | 21 | 17175 |
| HSV1-UL48-1767 | - | CCACGCCGAGCUACGGGCGCGG | 22 | 17176 |
| HSV1-UL48-1768 | - | UCCACGCCGAGCUACGGGCGCGG | 23 | 17177 |
| HSV1-UL48-1769 | - | UUCCACGCCGAGCUACGGGCGCGG | 24 | 17178 |
| HSV1-UL48-1770 | - | CCGAGCUACGGGCGCGGG | 18 | 17179 |
| HSV1-UL48-1771 | - | GCCGAGCUACGGGCGCGGG | 19 | 17180 |
| HSV1-UL48-40 | - | CGCCGAGCUACGGGCGCGGG | 20 | 5973 |
| HSV1-UL48-1772 | - | ACGCCGAGCUACGGGCGCGGG | 21 | 17181 |
| HSV1-UL48-1773 | - | CACGCCGAGCUACGGGCGCGGG | 22 | 17182 |
| HSV1-UL48-1774 | - | CCACGCCGAGCUACGGGCGCGGG | 23 | 17183 |
| HSV1-UL48-1775 | - | UCCACGCCGAGCUACGGGCGCGGG | 24 | 17184 |
| HSV1-UL48-1776 | - | AGCGAUGUGGUGGAAUGG | 18 | 17185 |
| HSV1-UL48-1777 | - | CAGCGAUGUGGUGGAAUGG | 19 | 17186 |
| HSV1-UL48-28 | - | CCAGCGAUGUGGUGGAAUGG | 20 | 5970 |
| HSV1-UL48-1778 | - | CCCAGCGAUGUGGUGGAAUGG | 21 | 17187 |
| HSV1-UL48-1779 | - | GCCCAGCGAUGUGGUGGAAUGG | 22 | 17188 |
| HSV1-UL48-1780 | - | UGCCCAGCGAUGUGGUGGAAUGG | 23 | 17189 |
| HSV1-UL48-1781 | - | CUGCCCAGCGAUGUGGUGGAAUGG | 24 | 17190 |
| HSV1-UL48-1782 | - | CAGCGAUGUGGUGGAAUG | 18 | 17191 |
| HSV1-UL48-1783 | - | CCAGCGAUGUGGUGGAAUG | 19 | 17192 |
| HSV1-UL48-27 | - | CCCAGCGAUGUGGUGGAAUG | 20 | 5969 |
| HSV1-UL48-1784 | - | GCCCAGCGAUGUGGUGGAAUG | 21 | 17193 |
| HSV1-UL48-1785 | - | UGCCCAGCGAUGUGGUGGAAUG | 22 | 17194 |
| HSV1-UL48-1786 | - | CUGCCCAGCGAUGUGGUGGAAUG | 23 | 17195 |
| HSV1-UL48-1787 | - | GCUGCCCAGCGAUGUGGUGGAAUG | 24 | 17196 |
| HSV1-UL48-1788 | - | ACGCUGCCCAGCGAUGUG | 18 | 17197 |
| HSV1-UL48-1789 | - | AACGCUGCCCAGCGAUGUG | 19 | 17198 |
| HSV1-UL48-625 | - | CAACGCUGCCCAGCGAUGUG | 20 | 6457 |
| HSV1-UL48-1790 | - | UCAACGCUGCCCAGCGAUGUG | 21 | 17199 |
| HSV1-UL48-1791 | - | AUCAACGCUGCCCAGCGAUGUG | 22 | 17200 |
| HSV1-UL48-1792 | - | UAUCAACGCUGCCCAGCGAUGUG | 23 | 17201 |
| HSV1-UL48-1793 | - | CUAUCAACGCUGCCCAGCGAUGUG | 24 | 17202 |
| HSV1-UL48-1794 | - | CCAGCGAUGUGGUGGAAU | 18 | 17203 |
| HSV1-UL48-1795 | - | CCCAGCGAUGUGGUGGAAU | 19 | 17204 |
| HSV1-UL48-26 | - | GCCCAGCGAUGUGGUGGAAU | 20 | 5968 |
| HSV1-UL48-1796 | - | UGCCCAGCGAUGUGGUGGAAU | 21 | 17205 |
| HSV1-UL48-1797 | - | CUGCCCAGCGAUGUGGUGGAAU | 22 | 17206 |
| HSV1-UL48-1798 | - | GCUGCCCAGCGAUGUGGUGGAAU | 23 | 17207 |
| HSV1-UL48-1799 | - | CGCUGCCCAGCGAUGUGGUGGAAU | 24 | 17208 |
| HSV1-UL48-1800 | - | ACGGCCUCGGGCUCUACU | 18 | 17209 |
| HSV1-UL48-1801 | - | GACGGCCUCGGGCUCUACU | 19 | 17210 |
| HSV1-UL48-634 | - | CGACGGCCUCGGGCUCUACU | 20 | 6461 |
| HSV1-UL48-1802 | - | GCGACGGCCUCGGGCUCUACU | 21 | 17211 |
| HSV1-UL48-1803 | - | CGCGACGGCCUCGGGCUCUACU | 22 | 17212 |
| HSV1-UL48-1804 | - | CCGCGACGGCCUCGGGCUCUACU | 23 | 17213 |
| HSV1-UL48-1805 | - | CCCGCGACGGCCUCGGGCUCUACU | 24 | 17214 |
| HSV1-UL48-1806 | - | UUUCUUCCACGCCGAGCU | 18 | 17215 |
| HSV1-UL48-1807 | - | GUUUCUUCCACGCCGAGCU | 19 | 17216 |
| HSV1-UL48-636 | - | CGUUUCUUCCACGCCGAGCU | 20 | 6463 |
| HSV1-UL48-1808 | - | UCGUUUCUUCCACGCCGAGCU | 21 | 17217 |
| HSV1-UL48-1809 | - | CUCGUUUCUUCCACGCCGAGCU | 22 | 17218 |
| HSV1-UL48-1810 | - | UCUCGUUUCUUCCACGCCGAGCU | 23 | 17219 |
| HSV1-UL48-1811 | - | CUCUCGUUUCUUCCACGCCGAGCU | 24 | 17220 |

**Table 12E** provides exemplary targeting domains for knocking out the *UL48* gene selected according to the fifth tier parameters. The targeting domains fall in the coding sequence of the gene, downstream of the first 500bp of coding sequence (e.g., anywhere from +500 (relative to the start codon) to the stop codon of the gene), and start with a 5'G. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12E**

| 5th Tier | | | | |
|---|---|---|---|---|
| gRNA Name | DNA Strand | Targeting Domain | Target Site Length | Seq ID NO: |
| HSV1-UL48-1812 | + | GCUCGGCGUACGCGGCCCA | 19 | 17221 |
| HSV1-UL48-1813 | + | GAGGGCGAGGUCGUGAAGC | 19 | 17222 |
| HSV1-UL48-370 | + | GGAGGGCGAGGUCGUGAAGC | 20 | 6208 |
| HSV1-UL48-989 | + | GAAUACGAGUCCAACUUCGC | 20 | 6685 |
| HSV1-UL48-1814 | + | GGAAUACGAGUCCAACUUCGC | 21 | 17223 |
| HSV1-UL48-1815 | + | GCUGGAAUACGAGUCCAACUUCGC | 24 | 17224 |
| HSV1-UL48-1816 | + | GCGGGAGGUUAAGGUGCUC | 19 | 17225 |
| HSV1-UL48-1817 | + | GUACGCGGCCCAUAGGAUCUC | 21 | 17226 |
| HSV1-UL48-1818 | + | GCGUACGCGGCCCAUAGGAUCUC | 23 | 17227 |
| HSV1-UL48-1819 | + | GGCGUACGCGGCCCAUAGGAUCUC | 24 | 17228 |
| HSV1-UL48-1820 | + | GGGUAAAUCCCGGCCCCG | 18 | 17229 |
| HSV1-UL48-1821 | + | GGGGUAAAUCCCGGCCCCG | 19 | 17230 |
| HSV1-UL48-340 | + | GGGGGUAAAUCCCGGCCCCG | 20 | 6178 |
| HSV1-UL48-1822 | + | GGGGGGUAAAUCCCGGCCCCG | 21 | 17231 |
| HSV1-UL48-1823 | + | GUGGGGGGUAAAUCCCGGCCCCG | 23 | 17232 |
| HSV1-UL48-1824 | + | GUUGCCAUGCAGGGUGGG | 18 | 17233 |
| HSV1-UL48-1825 | + | GGUUGCCAUGCAGGGUGGG | 19 | 17234 |
| HSV1-UL48-1826 | + | GCCUGGUUGCCAUGCAGGGUGGG | 23 | 17235 |
| HSV1-UL48-1827 | + | GGCCUGGUUGCCAUGCAGGGUGGG | 24 | 17236 |
| HSV1-UL48-1828 | + | GCGGGCCUGGUUGCCAUG | 18 | 17237 |
| HSV1-UL48-986 | + | GCGCGGGCCUGGUUGCCAUG | 20 | 6682 |
| HSV1-UL48-1829 | + | GGCGCGGGCCUGGUUGCCAUG | 21 | 17238 |
| HSV1-UL48-1830 | + | GAGGCGCGGGCCUGGUUGCCAUG | 23 | 17239 |
| HSV1-UL48-1831 | + | GUAGGGGGCGGAGUCGUG | 18 | 17240 |
| HSV1-UL48-1832 | + | GCCGUAGGGGGCGGAGUCGUG | 21 | 17241 |
| HSV1-UL48-1833 | + | GCGCCGUAGGGGGCGGAGUCGUG | 23 | 17242 |
| HSV1-UL48-1834 | + | GAGGUCGUGAAGCUGGAAU | 19 | 17243 |
| HSV1-UL48-1835 | + | GC GAGGUC GUGAAGCUGGAAU | 21 | 17244 |
| HSV1-UL48-1836 | + | GGC GAGGUC GUGAAGCUGGAAU | 22 | 17245 |
| HSV1-UL48-1837 | + | GGGCGAGGUCGUGAAGCUGGAAU | 23 | 17246 |
| HSV1-UL48-1838 | - | GAUCUGGACAUGUUGGGGGAC | 21 | 17247 |
| HSV1-UL48-1839 | - | GACGGGGAUUCCCCGGGGC | 19 | 17248 |
| HSV1-UL48-314 | - | GGACGGGGAUUCCCCGGGGC | 20 | 6152 |
| HSV1-UL48-1840 | - | GGGACGGGGAUUCCCCGGGGC | 21 | 17249 |
| HSV1-UL48-1841 | - | GGGGACGGGGAUUCCCCGGGGC | 22 | 17250 |
| HSV1-UL48-1842 | - | GGGGGACGGGGAUUCCCCGGGGC | 23 | 17251 |
| HSV1-UL48-1843 | - | GCCCUUGGAAUUGACGAGUACG | 22 | 17252 |
| HSV1-UL48-1844 | - | GUCAACGGAGCGCUCACCGUCCG | 23 | 17253 |
| HSV1-UL48-1845 | - | GUUUACCGAUGCCCUUGGAAUUG | 23 | 17254 |
| HSV1-UL48-1846 | - | GAGAGCUGGCGUCAGUUG | 18 | 17255 |
| HSV1-UL48-1847 | - | GGAGAGCUGGCGUCAGUUG | 19 | 17256 |
| HSV1-UL48-1848 | - | GACCUGGAGAGCUGGCGUCAGUUG | 24 | 17257 |
| HSV1-UL48-1849 | - | GCUCUGGAUAUGGCCGACU | 19 | 17258 |
| HSV1-UL48-1850 | - | GCGCUCUGGAUAUGGCCGACU | 21 | 17259 |
| HSV1-UL48-1851 | - | GGCGCUCUGGAUAUGGCCGACU | 22 | 17260 |
| HSV1-UL48-1852 | - | GAUGUUUACCGAUGCCCU | 18 | 17261 |
| HSV1-UL48-1853 | - | GCAGAUGUUUACCGAUGCCCU | 21 | 17262 |
| HSV1-UL48-1854 | - | GAGCAGAUGUUUACCGAUGCCCU | 23 | 17263 |
| HSV1-UL48-1855 | - | GCAACCAGGCCCGCGCCU | 18 | 17264 |
| HSV1-UL48-1856 | - | GGCAACCAGGCCCGCGCCU | 19 | 17265 |
| HSV1-UL48-1857 | - | GCAUGGCAACCAGGCCCGCGCCU | 23 | 17266 |
| HSV1-UL48-1858 | - | GACUCCGCCCCCUACGGCGCU | 21 | 17267 |
| HSV1-UL48-1859 | - | GC GCGUAC GAAAAACAAUU | 19 | 17268 |
| HSV1-UL48-1860 | - | GCGCGCGUACGAAAAACAAUU | 21 | 17269 |
| HSV1-UL48-1861 | - | GCCGCGCGCGUACGAAAAACAAUU | 24 | 17270 |

**Table 12F** provides exemplary targeting domains for knocking out the *UL48* gene selected according to the six tier parameters. The targeting domains fall in the coding sequence of the gene, downstream of the first 500bp of coding sequence (e.g., anywhere from +500 (relative to the start codon) to the stop codon of the gene). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12F**

| 6th Tier | | | | |
|---|---|---|---|---|
| gRNA Name | DNA Strand | Targeting Domain | Target Site Length | Seq ID NO: |
| HSV1-UL48-1862 | + | CUCGGCGUACGCGGCCCA | 18 | 17271 |
| HSV1-UL48-966 | + | UGCUCGGCGUACGCGGCCCA | 20 | 6668 |
| HSV1-UL48-1863 | + | CUGCUCGGCGUACGCGGCCCA | 21 | 17272 |
| HSV1-UL48-1864 | + | UCUGCUCGGCGUACGCGGCCCA | 22 | 17273 |
| HSV1-UL48-1865 | + | AUCUGCUCGGCGUACGCGGCCCA | 23 | 17274 |
| HSV1-UL48-1866 | + | CAUCUGCUCGGCGUACGCGGCCCA | 24 | 17275 |
| HSV1-UL48-1867 | + | AGGGC GAGGUC GUGAAGC | 18 | 17276 |
| HSV1-UL48-1868 | + | CGGAGGGCGAGGUCGUGAAGC | 21 | 17277 |
| HSV1-UL48-1869 | + | UCGGAGGGCGAGGUCGUGAAGC | 22 | 17278 |
| HSV1-UL48-1870 | + | CUCGGAGGGCGAGGUCGUGAAGC | 23 | 17279 |
| HSV1-UL48-1871 | + | CCUCGGAGGGCGAGGUCGUGAAGC | 24 | 17280 |
| HSV1-UL48-1872 | + | AUACGAGUCCAACUUCGC | 18 | 17281 |
| HSV1-UL48-1873 | + | AAUACGAGUCCAACUUCGC | 19 | 17282 |
| HSV1-UL48-1874 | + | UGGAAUACGAGUCCAACUUCGC | 22 | 17283 |
| HSV1-UL48-1875 | + | CUGGAAUACGAGUCCAACUUCGC | 23 | 17284 |
| HSV1-UL48-1876 | + | CGGGAGGUUAAGGUGCUC | 18 | 17285 |
| HSV1-UL48-977 | + | AGCGGGAGGUUAAGGUGCUC | 20 | 6678 |
| HSV1-UL48-1877 | + | CAGCGGGAGGUUAAGGUGCUC | 21 | 17286 |
| HSV1-UL48-1878 | + | CCAGCGGGAGGUUAAGGUGCUC | 22 | 17287 |
| HSV1-UL48-1879 | + | ACCAGCGGGAGGUUAAGGUGCUC | 23 | 17288 |
| HSV1-UL48-1880 | + | CACCAGCGGGAGGUUAAGGUGCUC | 24 | 17289 |
| HSV1-UL48-1881 | + | CGCGGCCCAUAGGAUCUC | 18 | 17290 |
| HSV1-UL48-1882 | + | ACGCGGCCCAUAGGAUCUC | 19 | 17291 |
| HSV1-UL48-965 | + | UACGCGGCCCAUAGGAUCUC | 20 | 6667 |
| HSV1-UL48-1883 | + | CGUACGCGGCCCAUAGGAUCUC | 22 | 17292 |
| HSV1-UL48-1884 | + | UGGGGGGUAAAUCCCGGCCCCG | 22 | 17293 |
| HSV1-UL48-1885 | + | CGUGGGGGGUAAAUCCCGGCCCCG | 24 | 17294 |
| HSV1-UL48-1886 | + | CCAGAGCGCCGUAGGGGG | 18 | 17295 |
| HSV1-UL48-1887 | + | UCCAGAGCGCCGUAGGGGG | 19 | 17296 |
| HSV1-UL48-331 | + | AUCCAGAGCGCCGUAGGGGG | 20 | 6169 |
| HSV1-UL48-1888 | + | UAUCCAGAGCGCCGUAGGGGG | 21 | 17297 |
| HSV1-UL48-1889 | + | AUAUCCAGAGCGCCGUAGGGGG | 22 | 17298 |
| HSV1-UL48-1890 | + | CAUAUCCAGAGCGCCGUAGGGGG | 23 | 17299 |
| HSV1-UL48-1891 | + | CCAUAUCCAGAGCGCCGUAGGGGG | 24 | 17300 |
| HSV1-UL48-379 | + | UGGUUGCCAUGCAGGGUGGG | 20 | 6217 |
| HSV1-UL48-1892 | + | CUGGUUGCCAUGCAGGGUGGG | 21 | 17301 |
| HSV1-UL48-1893 | + | CCUGGUUGCCAUGCAGGGUGGG | 22 | 17302 |
| HSV1-UL48-1894 | + | CGCGGGCCUGGUUGCCAUG | 19 | 17303 |
| HSV1-UL48-1895 | + | AGGCGCGGGCCUGGUUGCCAUG | 22 | 17304 |
| HSV1-UL48-1896 | + | AGAGGCGCGGGCCUGGUUGCCAUG | 24 | 17305 |
| HSV1-UL48-1897 | + | CGUAGGGGGCGGAGUCGUG | 19 | 17306 |
| HSV1-UL48-334 | + | CCGUAGGGGGCGGAGUCGUG | 20 | 6172 |
| HSV1-UL48-1898 | + | CGCCGUAGGGGGCGGAGUCGUG | 22 | 17307 |
| HSV1-UL48-1899 | + | AGCGCCGUAGGGGGCGGAGUCGUG | 24 | 17308 |
| HSV1-UL48-1900 | + | AGGUCGUGAAGCUGGAAU | 18 | 17309 |
| HSV1-UL48-990 | + | CGAGGUCGUGAAGCUGGAAU | 20 | 6686 |
| HSV1-UL48-1901 | + | AGGGCGAGGUCGUGAAGCUGGAAU | 24 | 17310 |
| HSV1-UL48-1902 | - | CUGGACAUGUUGGGGGAC | 18 | 17311 |
| HSV1-UL48-1903 | - | UCUGGACAUGUUGGGGGAC | 19 | 17312 |
| HSV1-UL48-309 | - | AUCUGGACAUGUUGGGGGAC | 20 | 6147 |
| HSV1-UL48-1904 | - | CGAUCUGGACAUGUUGGGGGAC | 22 | 17313 |
| HSV1-UL48-1905 | - | UCGAUCUGGACAUGUUGGGGGAC | 23 | 17314 |
| HSV1-UL48-1906 | - | UUCGAUCUGGACAUGUUGGGGGAC | 24 | 17315 |
| HSV1-UL48-1907 | - | ACGGGGAUUCCCCGGGGC | 18 | 17316 |
| HSV1-UL48-1908 | - | UGGGGGACGGGGAUUCCCCGGGGC | 24 | 17317 |
| HSV1-UL48-1909 | - | UUGGAAUUGACGAGUACG | 18 | 17318 |
| HSV1-UL48-1910 | - | CUUGGAAUUGACGAGUACG | 19 | 17319 |
| HSV1-UL48-882 | - | CCUUGGAAUUGACGAGUACG | 20 | 6611 |
| HSV1-UL48-1911 | - | CCCUUGGAAUUGACGAGUACG | 21 | 17320 |
| HSV1-UL48-1912 | - | UGCCCUUGGAAUUGACGAGUACG | 23 | 17321 |
| HSV1-UL48-1913 | - | AUGCCCUUGGAAUUGACGAGUACG | 24 | 17322 |
| HSV1-UL48-1914 | - | CGGAGCGCUCACCGUCCG | 18 | 17323 |
| HSV1-UL48-1915 | - | ACGGAGCGCUCACCGUCCG | 19 | 17324 |
| HSV1-UL48-257 | - | AACGGAGCGCUCACCGUCCG | 20 | 6095 |
| HSV1-UL48-1916 | - | CAACGGAGCGCUCACCGUCCG | 21 | 17325 |
| HSV1-UL48-1917 | - | UCAACGGAGCGCUCACCGUCCG | 22 | 17326 |
| HSV1-UL48-1918 | - | CGUCAACGGAGCGCUCACCGUCCG | 24 | 17327 |
| HSV1-UL48-1919 | - | CCGAUGCCCUUGGAAUUG | 18 | 17328 |
| HSV1-UL48-1920 | - | ACCGAUGCCCUUGGAAUUG | 19 | 17329 |
| HSV1-UL48-881 | - | UACCGAUGCCCUUGGAAUUG | 20 | 6610 |
| HSV1-UL48-1921 | - | UUACCGAUGCCCUUGGAAUUG | 21 | 17330 |
| HSV1-UL48-1922 | - | UUUACCGAUGCCCUUGGAAUUG | 22 | 17331 |
| HSV1-UL48-1923 | - | UGUUUACCGAUGCCCUUGGAAUUG | 24 | 17332 |
| HSV1-UL48-820 | - | UGGAGAGCUGGCGUCAGUUG | 20 | 6573 |
| HSV1-UL48-1924 | - | CUGGAGAGCUGGCGUCAGUUG | 21 | 17333 |
| HSV1-UL48-1925 | - | CCUGGAGAGCUGGCGUCAGUUG | 22 | 17334 |
| HSV1-UL48-1926 | - | AC CUGGAGAGCUGGC GUCAGUUG | 23 | 17335 |
| HSV1-UL48-1927 | - | CUCUGGAUAUGGCCGACU | 18 | 17336 |
| HSV1-UL48-877 | - | CGCUCUGGAUAUGGCCGACU | 20 | 6607 |
| HSV1-UL48-1928 | - | CGGCGCUCUGGAUAUGGCCGACU | 23 | 17337 |
| HSV1-UL48-1929 | - | ACGGCGCUCUGGAUAUGGCCGACU | 24 | 17338 |
| HSV1-UL48-1930 | - | AGAUGUUUACCGAUGCCCU | 19 | 17339 |
| HSV1-UL48-319 | - | CAGAUGUUUACCGAUGCCCU | 20 | 6157 |
| HSV1-UL48-1931 | - | AGCAGAUGUUUACCGAUGCCCU | 22 | 17340 |
| HSV1-UL48-1932 | - | UGAGCAGAUGUUUACCGAUGCCCU | 24 | 17341 |
| HSV1-UL48-838 | - | UGGCAACCAGGCCCGCGCCU | 20 | 6582 |
| HSV1-UL48-1933 | - | AUGGCAACCAGGCCCGCGCCU | 21 | 17342 |
| HSV1-UL48-1934 | - | CAUGGCAACCAGGCCCGCGCCU | 22 | 17343 |
| HSV1-UL48-1935 | - | UGCAUGGCAACCAGGCCCGCGCCU | 24 | 17344 |
| HSV1-UL48-1936 | - | UCCGCCCCCUACGGCGCU | 18 | 17345 |
| HSV1-UL48-1937 | - | CUCCGCCCCCUACGGCGCU | 19 | 17346 |
| HSV1-UL48-876 | - | ACUCCGCCCCCUACGGCGCU | 20 | 6606 |
| HSV1-UL48-1938 | - | CGACUCCGCCCCCUACGGCGCU | 22 | 17347 |
| HSV1-UL48-1939 | - | ACGACUCCGCCCCCUACGGCGCU | 23 | 17348 |
| HSV1-UL48-1940 | - | CACGACUCCGCCCCCUACGGCGCU | 24 | 17349 |
| HSV1-UL48-1941 | - | CGCGUACGAAAAACAAUU | 18 | 17350 |
| HSV1-UL48-847 | - | CGCGCGUACGAAAAACAAUU | 20 | 6589 |
| HSV1-UL48-1942 | - | CGCGCGCGUACGAAAAACAAUU | 22 | 17351 |
| HSV1-UL48-1943 | - | CCGCGCGCGUACGAAAAACAAUU | 23 | 17352 |

**Table 12G** provides exemplary targeting domains for knocking out the *UL48* gene selected according to the seven tier parameters. The targeting domains fall in the coding sequence of the gene, downstream of the first 500bp of coding sequence (e.g., anywhere from +500 (relative to the start codon) to the stop codon of the gene). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 12G**

| 7th Tier | | | | |
|---|---|---|---|---|
| gRNA Name | DNA Strand | Targeting Domain | Target Site Length | Seq ID NO: |
| HSV1-UL48-1944 | + | CAGCUCUCCAGGUCGCAA | 18 | 17353 |
| HSV1-UL48-1945 | + | CCAGCUCUCCAGGUCGCAA | 19 | 17354 |
| HSV1-UL48-968 | + | GCCAGCUCUCCAGGUCGCAA | 20 | 6670 |
| HSV1-UL48-1946 | + | CGCCAGCUCUCCAGGUCGCAA | 21 | 17355 |
| HSV1-UL48-1947 | + | ACGCCAGCUCUCCAGGUCGCAA | 22 | 17356 |
| HSV1-UL48-1948 | + | GACGCCAGCUCUCCAGGUCGCAA | 23 | 17357 |
| HSV1-UL48-1949 | + | UGACGCCAGCUCUCCAGGUCGCAA | 24 | 17358 |
| HSV1-UL48-1950 | + | CACGUCCUCGCCGUCUAA | 18 | 17359 |
| HSV1-UL48-1951 | + | CCACGUCCUCGCCGUCUAA | 19 | 17360 |
| HSV1-UL48-1019 | + | GCCACGUCCUCGCCGUCUAA | 20 | 6700 |
| HSV1-UL48-1952 | + | CGCCACGUCCUCGCCGUCUAA | 21 | 17361 |
| HSV1-UL48-1953 | + | UCGCCACGUCCUCGCCGUCUAA | 22 | 17362 |
| HSV1-UL48-1954 | + | AUCGCCACGUCCUCGCCGUCUAA | 23 | 17363 |
| HSV1-UL48-1955 | + | CAUCGCCACGUCCUCGCCGUCUAA | 24 | 17364 |
| HSV1-UL48-1956 | + | UAGCCGCGCUGCGCACCA | 18 | 17365 |
| HSV1-UL48-1957 | + | GUAGCCGCGCUGCGCACCA | 19 | 17366 |
| HSV1-UL48-980 | + | CGUAGCCGCGCUGCGCACCA | 20 | 6679 |
| HSV1-UL48-1958 | + | CCGUAGCCGCGCUGCGCACCA | 21 | 17367 |
| HSV1-UL48-1959 | + | UCCGUAGCCGCGCUGCGCACCA | 22 | 17368 |
| HSV1-UL48-1960 | + | CUCCGUAGCCGCGCUGCGCACCA | 23 | 17369 |
| HSV1-UL48-1961 | + | CCUCCGUAGCCGCGCUGCGCACCA | 24 | 17370 |
| HSV1-UL48-1962 | + | UCCCCCAACAUGUCCAGA | 18 | 17371 |
| HSV1-UL48-1963 | + | GUCCCCCAACAUGUCCAGA | 19 | 17372 |
| HSV1-UL48-1020 | + | CGUCCCCCAACAUGUCCAGA | 20 | 6701 |
| HSV1-UL48-1964 | + | CCGUCCCCCAACAUGUCCAGA | 21 | 17373 |
| HSV1-UL48-1965 | + | CCCGUCCCCCAACAUGUCCAGA | 22 | 17374 |
| HSV1-UL48-1966 | + | CCCCGUCCCCCAACAUGUCCAGA | 23 | 17375 |
| HSV1-UL48-1967 | + | UCCCCGUCCCCCAACAUGUCCAGA | 24 | 17376 |
| HSV1-UL48-1968 | + | GCGUCGUCGUCCGGGAGA | 18 | 17377 |
| HSV1-UL48-1969 | + | GGCGUCGUCGUCCGGGAGA | 19 | 17378 |
| HSV1-UL48-998 | + | GGGCGUCGUCGUCCGGGAGA | 20 | 6692 |
| HSV1-UL48-1970 | + | GGGGCGUCGUCGUCCGGGAGA | 21 | 17379 |
| HSV1-UL48-1971 | + | GGGGGCGUCGUCGUCCGGGAGA | 22 | 17380 |
| HSV1-UL48-1972 | + | CGGGGGCGUCGUCGUCCGGGAGA | 23 | 17381 |
| HSV1-UL48-1973 | + | UCGGGGGCGUCGUCGUCCGGGAGA | 24 | 17382 |
| HSV1-UL48-1974 | + | GUGUGUCCCGCGGGGAGA | 18 | 17383 |
| HSV1-UL48-1975 | + | CGUGUGUCCCGCGGGGAGA | 19 | 17384 |
| HSV1-UL48-1007 | + | GCGUGUGUCCCGCGGGGAGA | 20 | 6696 |
| HSV1-UL48-1976 | + | CGCGUGUGUCCCGCGGGGAGA | 21 | 17385 |
| HSV1-UL48-1977 | + | GCGCGUGUGUCCCGCGGGGAGA | 22 | 17386 |
| HSV1-UL48-1978 | + | UGCGCGUGUGUCCCGCGGGGAGA | 23 | 17387 |
| HSV1-UL48-1979 | + | CUGCGCGUGUGUCCCGCGGGGAGA | 24 | 17388 |
| HSV1-UL48-1980 | + | CAUAUCCAGAGCGCCGUA | 18 | 17389 |
| HSV1-UL48-1981 | + | CCAUAUCCAGAGCGCCGUA | 19 | 17390 |
| HSV1-UL48-328 | + | GCCAUAUCCAGAGCGCCGUA | 20 | 6166 |
| HSV1-UL48-1982 | + | GGCCAUAUCCAGAGCGCCGUA | 21 | 17391 |
| HSV1-UL48-1983 | + | CGGCCAUAUCCAGAGCGCCGUA | 22 | 17392 |
| HSV1-UL48-1984 | + | UCGGCCAUAUCCAGAGCGCCGUA | 23 | 17393 |
| HSV1-UL48-1985 | + | GUCGGCCAUAUCCAGAGCGCCGUA | 24 | 17394 |
| HSV1-UL48-1986 | + | GGCGCGCAGGUACCGGUA | 18 | 17395 |
| HSV1-UL48-1987 | + | UGGCGCGCAGGUACCGGUA | 19 | 17396 |
| HSV1-UL48-962 | + | CUGGCGCGCAGGUACCGGUA | 20 | 6664 |
| HSV1-UL48-1988 | + | GCUGGCGCGCAGGUACCGGUA | 21 | 17397 |
| HSV1-UL48-1989 | + | CGCUGGCGCGCAGGUACCGGUA | 22 | 17398 |
| HSV1-UL48-1990 | + | ACGCUGGCGCGCAGGUACCGGUA | 23 | 17399 |
| HSV1-UL48-1991 | + | GACGCUGGCGCGCAGGUACCGGUA | 24 | 17400 |
| HSV1-UL48-1992 | + | GUAAACAUCUGCUCAAAC | 18 | 17401 |
| HSV1-UL48-1993 | + | GGUAAACAUCUGCUCAAAC | 19 | 17402 |
| HSV1-UL48-1035 | + | CGGUAAACAUCUGCUCAAAC | 20 | 6707 |
| HSV1-UL48-1994 | + | UCGGUAAACAUCUGCUCAAAC | 21 | 17403 |
| HSV1-UL48-1995 | + | AUCGGUAAACAUCUGCUCAAAC | 22 | 17404 |
| HSV1-UL48-1996 | + | CAUCGGUAAACAUCUGCUCAAAC | 23 | 17405 |
| HSV1-UL48-1997 | + | GCAUCGGUAAACAUCUGCUCAAAC | 24 | 17406 |
| HSV1-UL48-1998 | + | AGCGCUCCGUUGACGAAC | 18 | 17407 |
| HSV1-UL48-1999 | + | GAGCGCUCCGUUGACGAAC | 19 | 17408 |
| HSV1-UL48-972 | + | UGAGCGCUCCGUUGACGAAC | 20 | 6673 |
| HSV1-UL48-2000 | + | GUGAGCGCUCCGUUGACGAAC | 21 | 17409 |
| HSV1-UL48-2001 | + | GGUGAGCGCUCCGUUGACGAAC | 22 | 17410 |
| HSV1-UL48-2002 | + | CGGUGAGCGCUCCGUUGACGAAC | 23 | 17411 |
| HSV1-UL48-2003 | + | ACGGUGAGCGCUCCGUUGACGAAC | 24 | 17412 |
| HSV1-UL48-2004 | + | UCAACACCAUAAAGUACC | 18 | 17413 |
| HSV1-UL48-2005 | + | AUCAACACCAUAAAGUACC | 19 | 17414 |
| HSV1-UL48-988 | + | AAUCAACACCAUAAAGUACC | 20 | 6684 |
| HSV1-UL48-2006 | + | GAAUCAACACCAUAAAGUACC | 21 | 17415 |
| HSV1-UL48-2007 | + | CGAAUCAACACCAUAAAGUACC | 22 | 17416 |
| HSV1-UL48-2008 | + | CCGAAUCAACACCAUAAAGUACC | 23 | 17417 |
| HSV1-UL48-2009 | + | CCCGAAUCAACACCAUAAAGUACC | 24 | 17418 |
| HSV1-UL48-2010 | + | GGGGUAAAUCCCGGCCCC | 18 | 17419 |
| HSV1-UL48-2011 | + | GGGGGUAAAUCCCGGCCCC | 19 | 17420 |
| HSV1-UL48-339 | + | GGGGGGUAAAUCCCGGCCCC | 20 | 6177 |
| HSV1-UL48-2012 | + | UGGGGGGUAAAUCCCGGCCCC | 21 | 17421 |
| HSV1-UL48-2013 | + | GUGGGGGGUAAAUCCCGGCCCC | 22 | 17422 |
| HSV1-UL48-2014 | + | CGUGGGGGGUAAAUCCCGGCCCC | 23 | 17423 |
| HSV1-UL48-2015 | + | UCGUGGGGGGUAAAUCCCGGCCCC | 24 | 17424 |
| HSV1-UL48-2016 | + | GGGGGUAAAUCCCGGCCC | 18 | 17425 |
| HSV1-UL48-2017 | + | GGGGGGUAAAUCCCGGCCC | 19 | 17426 |
| HSV1-UL48-338 | + | UGGGGGGUAAAUCCCGGCCC | 20 | 6176 |
| HSV1-UL48-2018 | + | GUGGGGGGUAAAUCCCGGCCC | 21 | 17427 |
| HSV1-UL48-2019 | + | CGUGGGGGGUAAAUCCCGGCCC | 22 | 17428 |
| HSV1-UL48-2020 | + | UCGUGGGGGGUAAAUCCCGGCCC | 23 | 17429 |
| HSV1-UL48-2021 | + | GUCGUGGGGGGUAAAUCCCGGCCC | 24 | 17430 |
| HSV1-UL48-2022 | + | AGUCUGCGCGUGUGUCCC | 18 | 17431 |
| HSV1-UL48-2023 | + | CAGUCUGCGCGUGUGUCCC | 19 | 17432 |
| HSV1-UL48-1012 | + | ACAGUCUGCGCGUGUGUCCC | 20 | 6698 |
| HSV1-UL48-2024 | + | GACAGUCUGCGCGUGUGUCCC | 21 | 17433 |
| HSV1-UL48-2025 | + | CGACAGUCUGCGCGUGUGUCCC | 22 | 17434 |
| HSV1-UL48-2026 | + | UCGACAGUCUGCGCGUGUGUCCC | 23 | 17435 |
| HSV1-UL48-2027 | + | GUCGACAGUCUGCGCGUGUGUCCC | 24 | 17436 |
| HSV1-UL48-2028 | + | UGUUCCCGCAUGACCGCC | 18 | 17437 |
| HSV1-UL48-2029 | + | GUGUUCCCGCAUGACCGCC | 19 | 17438 |
| HSV1-UL48-997 | + | CGUGUUCCCGCAUGACCGCC | 20 | 6691 |
| HSV1-UL48-2030 | + | GCGUGUUCCCGCAUGACCGCC | 21 | 17439 |
| HSV1-UL48-2031 | + | CGCGUGUUCCCGCAUGACCGCC | 22 | 17440 |
| HSV1-UL48-2032 | + | ACGCGUGUUCCCGCAUGACCGCC | 23 | 17441 |
| HSV1-UL48-2033 | + | UACGCGUGUUCCCGCAUGACCGCC | 24 | 17442 |
| HSV1-UL48-2034 | + | GGGGGGUAAAUCCCGGCC | 18 | 17443 |
| HSV1-UL48-2035 | + | UGGGGGGUAAAUCCCGGCC | 19 | 17444 |
| HSV1-UL48-1024 | + | GUGGGGGGUAAAUCCCGGCC | 20 | 6702 |
| HSV1-UL48-2036 | + | CGUGGGGGGUAAAUCCCGGCC | 21 | 17445 |
| HSV1-UL48-2037 | + | UCGUGGGGGGUAAAUCCCGGCC | 22 | 17446 |
| HSV1-UL48-2038 | + | GUCGUGGGGGGUAAAUCCCGGCC | 23 | 17447 |
| HSV1-UL48-2039 | + | AGUCGUGGGGGGUAAAUCCCGGCC | 24 | 17448 |
| HSV1-UL48-2040 | + | GGCCUCGAUUGGCACUCC | 18 | 17449 |
| HSV1-UL48-2041 | + | GGGCCUCGAUUGGCACUCC | 19 | 17450 |
| HSV1-UL48-975 | + | CGGGCCUCGAUUGGCACUCC | 20 | 6676 |
| HSV1-UL48-2042 | + | GCGGGCCUCGAUUGGCACUCC | 21 | 17451 |
| HSV1-UL48-2043 | + | GGCGGGCCUCGAUUGGCACUCC | 22 | 17452 |
| HSV1-UL48-2044 | + | CGGCGGGCCUCGAUUGGCACUCC | 23 | 17453 |
| HSV1-UL48-2045 | + | CCGGCGGGCCUCGAUUGGCACUCC | 24 | 17454 |
| HSV1-UL48-2046 | + | UCGGGGGCGUCGUCGUCC | 18 | 17455 |
| HSV1-UL48-2047 | + | UUCGGGGGCGUCGUCGUCC | 19 | 17456 |
| HSV1-UL48-362 | + | CUUCGGGGGCGUCGUCGUCC | 20 | 6200 |
| HSV1-UL48-2048 | + | UCUUCGGGGGCGUCGUCGUCC | 21 | 17457 |
| HSV1-UL48-2049 | + | CUCUUCGGGGGCGUCGUCGUCC | 22 | 17458 |
| HSV1-UL48-2050 | + | CCUCUUCGGGGGCGUCGUCGUCC | 23 | 17459 |
| HSV1-UL48-2051 | + | GCCUCUUCGGGGGCGUCGUCGUCC | 24 | 17460 |
| HSV1-UL48-2052 | + | ACCGUACUCGUCAAUUCC | 18 | 17461 |
| HSV1-UL48-2053 | + | CACCGUACUCGUCAAUUCC | 19 | 17462 |
| HSV1-UL48-1036 | + | CCACCGUACUCGUCAAUUCC | 20 | 6708 |
| HSV1-UL48-2054 | + | CCCACCGUACUCGUCAAUUCC | 21 | 17463 |
| HSV1-UL48-2055 | + | ACCCACCGUACUCGUCAAUUCC | 22 | 17464 |
| HSV1-UL48-2056 | + | UACCCACCGUACUCGUCAAUUCC | 23 | 17465 |
| HSV1-UL48-2057 | + | CUACCCACCGUACUCGUCAAUUCC | 24 | 17466 |
| HSV1-UL48-2058 | + | GCCGCGCUGCGCACCAGC | 18 | 17467 |
| HSV1-UL48-2059 | + | AGCCGCGCUGCGCACCAGC | 19 | 17468 |
| HSV1-UL48-385 | + | UAGCCGCGCUGCGCACCAGC | 20 | 6223 |
| HSV1-UL48-2060 | + | GUAGCCGCGCUGCGCACCAGC | 21 | 17469 |
| HSV1-UL48-2061 | + | CGUAGCCGCGCUGCGCACCAGC | 22 | 17470 |
| HSV1-UL48-2062 | + | CCGUAGCCGCGCUGCGCACCAGC | 23 | 17471 |
| HSV1-UL48-2063 | + | UCCGUAGCCGCGCUGCGCACCAGC | 24 | 17472 |
| HSV1-UL48-2064 | + | UCUGCGCGUGUGUCCCGC | 18 | 17473 |
| HSV1-UL48-2065 | + | GUCUGCGCGUGUGUCCCGC | 19 | 17474 |
| HSV1-UL48-352 | + | AGUCUGCGCGUGUGUCCCGC | 20 | 6190 |
| HSV1-UL48-2066 | + | CAGUCUGCGCGUGUGUCCCGC | 21 | 17475 |
| HSV1-UL48-2067 | + | ACAGUCUGCGCGUGUGUCCCGC | 22 | 17476 |
| HSV1-UL48-2068 | + | GACAGUCUGCGCGUGUGUCCCGC | 23 | 17477 |
| HSV1-UL48-2069 | + | CGACAGUCUGCGCGUGUGUCCCGC | 24 | 17478 |
| HSV1-UL48-2070 | + | CGGGGAGAAAGGACAGGC | 18 | 17479 |
| HSV1-UL48-2071 | + | GCGGGGAGAAAGGACAGGC | 19 | 17480 |
| HSV1-UL48-1006 | + | CGCGGGGAGAAAGGACAGGC | 20 | 6695 |
| HSV1-UL48-2072 | + | CCGCGGGGAGAAAGGACAGGC | 21 | 17481 |
| HSV1-UL48-2073 | + | CCCGCGGGGAGAAAGGACAGGC | 22 | 17482 |
| HSV1-UL48-2074 | + | UCCCGCGGGGAGAAAGGACAGGC | 23 | 17483 |
| HSV1-UL48-2075 | + | GUCCCGCGGGGAGAAAGGACAGGC | 24 | 17484 |
| HSV1-UL48-2076 | + | CAUAAAGUACCCAGAGGC | 18 | 17485 |
| HSV1-UL48-2077 | + | CCAUAAAGUACCCAGAGGC | 19 | 17486 |
| HSV1-UL48-987 | + | ACCAUAAAGUACCCAGAGGC | 20 | 6683 |
| HSV1-UL48-2078 | + | CACCAUAAAGUACCCAGAGGC | 21 | 17487 |
| HSV1-UL48-2079 | + | ACACCAUAAAGUACCCAGAGGC | 22 | 17488 |
| HSV1-UL48-2080 | + | AACACCAUAAAGUACCCAGAGGC | 23 | 17489 |
| HSV1-UL48-2081 | + | CAACACCAUAAAGUACCCAGAGGC | 24 | 17490 |
| HSV1-UL48-2082 | + | UUGACGAACAUGAAGGGC | 18 | 17491 |
| HSV1-UL48-2083 | + | GUUGACGAACAUGAAGGGC | 19 | 17492 |
| HSV1-UL48-397 | + | CGUUGACGAACAUGAAGGGC | 20 | 6235 |
| HSV1-UL48-2084 | + | CCGUUGACGAACAUGAAGGGC | 21 | 17493 |
| HSV1-UL48-2085 | + | UCCGUUGACGAACAUGAAGGGC | 22 | 17494 |
| HSV1-UL48-2086 | + | CUCCGUUGACGAACAUGAAGGGC | 23 | 17495 |
| HSV1-UL48-2087 | + | GCUCCGUUGACGAACAUGAAGGGC | 24 | 17496 |
| HSV1-UL48-2088 | + | CUCGAAGUCGGCCAUAUC | 18 | 17497 |
| HSV1-UL48-2089 | + | ACUCGAAGUCGGCCAUAUC | 19 | 17498 |
| HSV1-UL48-1034 | + | AACUCGAAGUCGGCCAUAUC | 20 | 6706 |
| HSV1-UL48-2090 | + | AAACUCGAAGUCGGCCAUAUC | 21 | 17499 |
| HSV1-UL48-2091 | + | CAAACUCGAAGUCGGCCAUAUC | 22 | 17500 |
| HSV1-UL48-2092 | + | UCAAACUCGAAGUCGGCCAUAUC | 23 | 17501 |
| HSV1-UL48-2093 | + | CUCAAACUCGAAGUCGGCCAUAUC | 24 | 17502 |
| HSV1-UL48-2094 | + | GCCGUAGGGGGCGGAGUC | 18 | 17503 |
| HSV1-UL48-2095 | + | CGCCGUAGGGGGCGGAGUC | 19 | 17504 |
| HSV1-UL48-1028 | + | GCGCCGUAGGGGGCGGAGUC | 20 | 6703 |
| HSV1-UL48-2096 | + | AGCGCCGUAGGGGGCGGAGUC | 21 | 17505 |
| HSV1-UL48-2097 | + | GAGCGCCGUAGGGGGCGGAGUC | 22 | 17506 |
| HSV1-UL48-2098 | + | AGAGCGCCGUAGGGGGCGGAGUC | 23 | 17507 |
| HSV1-UL48-2099 | + | CAGAGCGCCGUAGGGGGCGGAGUC | 24 | 17508 |
| HSV1-UL48-2100 | + | UUCGGGGGCGUCGUCGUC | 18 | 17509 |
| HSV1-UL48-2101 | + | CUUCGGGGGCGUCGUCGUC | 19 | 17510 |
| HSV1-UL48-361 | + | UCUUCGGGGGCGUCGUCGUC | 20 | 6199 |
| HSV1-UL48-2102 | + | CUCUUCGGGGGCGUCGUCGUC | 21 | 17511 |
| HSV1-UL48-2103 | + | CCUCUUCGGGGGCGUCGUCGUC | 22 | 17512 |
| HSV1-UL48-2104 | + | GCCUCUUCGGGGGCGUCGUCGUC | 23 | 17513 |
| HSV1-UL48-2105 | + | CGCCUCUUCGGGGGCGUCGUCGUC | 24 | 17514 |
| HSV1-UL48-2106 | + | GCCUCGGAGGGCGAGGUC | 18 | 17515 |
| HSV1-UL48-2107 | + | CGCCUCGGAGGGCGAGGUC | 19 | 17516 |
| HSV1-UL48-993 | + | CCGCCUCGGAGGGCGAGGUC | 20 | 6688 |
| HSV1-UL48-2108 | + | ACCGCCUCGGAGGGCGAGGUC | 21 | 17517 |
| HSV1-UL48-2109 | + | GACCGCCUCGGAGGGCGAGGUC | 22 | 17518 |
| HSV1-UL48-2110 | + | UGACCGCCUCGGAGGGCGAGGUC | 23 | 17519 |
| HSV1-UL48-2111 | + | AUGACCGCCUCGGAGGGCGAGGUC | 24 | 17520 |
| HSV1-UL48-2112 | + | CCCAGGCUGACAUCGGUC | 18 | 17521 |
| HSV1-UL48-2113 | + | CCCCAGGCUGACAUCGGUC | 19 | 17522 |
| HSV1-UL48-346 | + | CCCCCAGGCUGACAUCGGUC | 20 | 6184 |
| HSV1-UL48-2114 | + | UCCCCCAGGCUGACAUCGGUC | 21 | 17523 |
| HSV1-UL48-2115 | + | GUCCCCCAGGCUGACAUCGGUC | 22 | 17524 |
| HSV1-UL48-2116 | + | CGUCCCCCAGGCUGACAUCGGUC | 23 | 17525 |
| HSV1-UL48-2117 | + | UCGUCCCCCAGGCUGACAUCGGUC | 24 | 17526 |
| HSV1-UL48-2118 | + | CGCCAGCCCCGCCUCUUC | 18 | 17527 |
| HSV1-UL48-2119 | + | CCGCCAGCCCCGCCUCUUC | 19 | 17528 |
| HSV1-UL48-358 | + | GCCGCCAGCCCCGCCUCUUC | 20 | 6196 |
| HSV1-UL48-2120 | + | AGCCGCCAGCCCCGCCUCUUC | 21 | 17529 |
| HSV1-UL48-2121 | + | GAGCCGCCAGCCCCGCCUCUUC | 22 | 17530 |
| HSV1-UL48-2122 | + | GGAGCCGCCAGCCCCGCCUCUUC | 23 | 17531 |
| HSV1-UL48-2123 | + | CGGAGCCGCCAGCCCCGCCUCUUC | 24 | 17532 |
| HSV1-UL48-2124 | + | GAGGGCGAGGUCGUGAAG | 18 | 17533 |
| HSV1-UL48-2125 | + | GGAGGGCGAGGUCGUGAAG | 19 | 17534 |
| HSV1-UL48-992 | + | CGGAGGGCGAGGUCGUGAAG | 20 | 6687 |
| HSV1-UL48-2126 | + | UCGGAGGGCGAGGUCGUGAAG | 21 | 17535 |
| HSV1-UL48-2127 | + | CUCGGAGGGCGAGGUCGUGAAG | 22 | 17536 |
| HSV1-UL48-2128 | + | CCUCGGAGGGCGAGGUCGUGAAG | 23 | 17537 |
| HSV1-UL48-2129 | + | GCCUCGGAGGGCGAGGUCGUGAAG | 24 | 17538 |
| HSV1-UL48-2130 | + | ACGUCCUCGCCGUCUAAG | 18 | 17539 |
| HSV1-UL48-2131 | + | CACGUCCUCGCCGUCUAAG | 19 | 17540 |
| HSV1-UL48-342 | + | CCACGUCCUCGCCGUCUAAG | 20 | 6180 |
| HSV1-UL48-2132 | + | GCCACGUCCUCGCCGUCUAAG | 21 | 17541 |
| HSV1-UL48-2133 | + | CGCCACGUCCUCGCCGUCUAAG | 22 | 17542 |
| HSV1-UL48-2134 | + | UCGCCACGUCCUCGCCGUCUAAG | 23 | 17543 |
| HSV1-UL48-2135 | + | AUCGCCACGUCCUCGCCGUCUAAG | 24 | 17544 |
| HSV1-UL48-2136 | + | AGCCGCGCUGCGCACCAG | 18 | 17545 |
| HSV1-UL48-2137 | + | UAGCCGCGCUGCGCACCAG | 19 | 17546 |
| HSV1-UL48-384 | + | GUAGCCGCGCUGCGCACCAG | 20 | 6222 |
| HSV1-UL48-2138 | + | CGUAGCCGCGCUGCGCACCAG | 21 | 17547 |
| HSV1-UL48-2139 | + | CCGUAGCCGCGCUGCGCACCAG | 22 | 17548 |
| HSV1-UL48-2140 | + | UCCGUAGCCGCGCUGCGCACCAG | 23 | 17549 |
| HSV1-UL48-2141 | + | CUCCGUAGCCGCGCUGCGCACCAG | 24 | 17550 |
| HSV1-UL48-2142 | + | AAACAAAACACGCGCCAG | 18 | 17551 |
| HSV1-UL48-2143 | + | AAAACAAAACACGCGCCAG | 19 | 17552 |
| HSV1-UL48-964 | + | AAAAACAAAACACGCGCCAG | 20 | 6666 |
| HSV1-UL48-2144 | + | CAAAAACAAAACACGCGCCAG | 21 | 17553 |
| HSV1-UL48-2145 | + | GCAAAAACAAAACACGCGCCAG | 22 | 17554 |
| HSV1-UL48-2146 | + | UGCAAAAACAAAACACGCGCCAG | 23 | 17555 |
| HSV1-UL48-2147 | + | AUGCAAAAACAAAACACGCGCCAG | 24 | 17556 |
| HSV1-UL48-2148 | + | UACCGGUACAGGGCCGAG | 18 | 17557 |
| HSV1-UL48-2149 | + | GUACCGGUACAGGGCCGAG | 19 | 17558 |
| HSV1-UL48-960 | + | GGUACCGGUACAGGGCCGAG | 20 | 6662 |
| HSV1-UL48-2150 | + | AGGUACCGGUACAGGGCCGAG | 21 | 17559 |
| HSV1-UL48-2151 | + | CAGGUACCGGUACAGGGCCGAG | 22 | 17560 |
| HSV1-UL48-2152 | + | GCAGGUACCGGUACAGGGCCGAG | 23 | 17561 |
| HSV1-UL48-2153 | + | CGCAGGUACCGGUACAGGGCCGAG | 24 | 17562 |
| HSV1-UL48-2154 | + | AUUGGCACUCCCCGGACG | 18 | 17563 |
| HSV1-UL48-2155 | + | GAUUGGCACUCCCCGGACG | 19 | 17564 |
| HSV1-UL48-974 | + | CGAUUGGCACUCCCCGGACG | 20 | 6675 |
| HSV1-UL48-2156 | + | UCGAUUGGCACUCCCCGGACG | 21 | 17565 |
| HSV1-UL48-2157 | + | CUCGAUUGGCACUCCCCGGACG | 22 | 17566 |
| HSV1-UL48-2158 | + | CCUCGAUUGGCACUCCCCGGACG | 23 | 17567 |
| HSV1-UL48-2159 | + | GCCUCGAUUGGCACUCCCCGGACG | 24 | 17568 |
| HSV1-UL48-2160 | + | GUCUGCGCGUGUGUCCCG | 18 | 17569 |
| HSV1-UL48-2161 | + | AGUCUGCGCGUGUGUCCCG | 19 | 17570 |
| HSV1-UL48-351 | + | CAGUCUGCGCGUGUGUCCCG | 20 | 6189 |
| HSV1-UL48-2162 | + | ACAGUCUGCGCGUGUGUCCCG | 21 | 17571 |
| HSV1-UL48-2163 | + | GACAGUCUGCGCGUGUGUCCCG | 22 | 17572 |
| HSV1-UL48-2164 | + | CGACAGUCUGCGCGUGUGUCCCG | 23 | 17573 |
| HSV1-UL48-2165 | + | UCGACAGUCUGCGCGUGUGUCCCG | 24 | 17574 |
| HSV1-UL48-2166 | + | GUUUAGCUCCCGCAGCCG | 18 | 17575 |
| HSV1-UL48-2167 | + | GGUUUAGCUCCCGCAGCCG | 19 | 17576 |
| HSV1-UL48-976 | + | UGGUUUAGCUCCCGCAGCCG | 20 | 6677 |
| HSV1-UL48-2168 | + | GUGGUUUAGCUCCCGCAGCCG | 21 | 17577 |
| HSV1-UL48-2169 | + | UGUGGUUUAGCUCCCGCAGCCG | 22 | 17578 |
| HSV1-UL48-2170 | + | AUGUGGUUUAGCUCCCGCAGCCG | 23 | 17579 |
| HSV1-UL48-2171 | + | AAUGUGGUUUAGCUCCCGCAGCCG | 24 | 17580 |
| HSV1-UL48-2172 | + | GCCAUAUCCAGAGCGCCG | 18 | 17581 |
| HSV1-UL48-2173 | + | GGCCAUAUCCAGAGCGCCG | 19 | 17582 |
| HSV1-UL48-1033 | + | CGGCCAUAUCCAGAGCGCCG | 20 | 6705 |
| HSV1-UL48-2174 | + | UCGGCCAUAUCCAGAGCGCCG | 21 | 17583 |
| HSV1-UL48-2175 | + | GUCGGCCAUAUCCAGAGCGCCG | 22 | 17584 |
| HSV1-UL48-2176 | + | AGUCGGCCAUAUCCAGAGCGCCG | 23 | 17585 |
| HSV1-UL48-2177 | + | AAGUCGGCCAUAUCCAGAGCGCCG | 24 | 17586 |
| HSV1-UL48-2178 | + | CUGCGCGUGUGUCCCGCG | 18 | 17587 |
| HSV1-UL48-2179 | + | UCUGCGCGUGUGUCCCGCG | 19 | 17588 |
| HSV1-UL48-353 | + | GUCUGCGCGUGUGUCCCGCG | 20 | 6191 |
| HSV1-UL48-2180 | + | AGUCUGCGCGUGUGUCCCGCG | 21 | 17589 |
| HSV1-UL48-2181 | + | CAGUCUGCGCGUGUGUCCCGCG | 22 | 17590 |
| HSV1-UL48-2182 | + | ACAGUCUGCGCGUGUGUCCCGCG | 23 | 17591 |
| HSV1-UL48-2183 | + | GACAGUCUGCGCGUGUGUCCCGCG | 24 | 17592 |
| HSV1-UL48-2184 | + | GGGGAGAAAGGACAGGCG | 18 | 17593 |
| HSV1-UL48-2185 | + | CGGGGAGAAAGGACAGGCG | 19 | 17594 |
| HSV1-UL48-356 | + | GCGGGGAGAAAGGACAGGCG | 20 | 6194 |
| HSV1-UL48-2186 | + | CGCGGGGAGAAAGGACAGGCG | 21 | 17595 |
| HSV1-UL48-2187 | + | CCGCGGGGAGAAAGGACAGGCG | 22 | 17596 |
| HSV1-UL48-2188 | + | CCCGCGGGGAGAAAGGACAGGCG | 23 | 17597 |
| HSV1-UL48-2189 | + | UCCCGCGGGGAGAAAGGACAGGCG | 24 | 17598 |
| HSV1-UL48-2190 | + | UCCCGCAUGACCGCCUCG | 18 | 17599 |
| HSV1-UL48-2191 | + | UUCCCGCAUGACCGCCUCG | 19 | 17600 |
| HSV1-UL48-995 | + | GUUCCCGCAUGACCGCCUCG | 20 | 6690 |
| HSV1-UL48-2192 | + | UGUUCCCGCAUGACCGCCUCG | 21 | 17601 |
| HSV1-UL48-2193 | + | GUGUUCCCGCAUGACCGCCUCG | 22 | 17602 |
| HSV1-UL48-2194 | + | CGUGUUCCCGCAUGACCGCCUCG | 23 | 17603 |
| HSV1-UL48-2195 | + | GCGUGUUCCCGCAUGACCGCCUCG | 24 | 17604 |
| HSV1-UL48-2196 | + | CCGUAGGGGGCGGAGUCG | 18 | 17605 |
| HSV1-UL48-2197 | + | GCCGUAGGGGGCGGAGUCG | 19 | 17606 |
| HSV1-UL48-332 | + | CGCCGUAGGGGGCGGAGUCG | 20 | 6170 |
| HSV1-UL48-2198 | + | GCGCCGUAGGGGGCGGAGUCG | 21 | 17607 |
| HSV1-UL48-2199 | + | AGCGCCGUAGGGGGCGGAGUCG | 22 | 17608 |
| HSV1-UL48-2200 | + | GAGCGCCGUAGGGGGCGGAGUCG | 23 | 17609 |
| HSV1-UL48-2201 | + | AGAGCGCCGUAGGGGGCGGAGUCG | 24 | 17610 |
| HSV1-UL48-2202 | + | CCAGGCUGACAUCGGUCG | 18 | 17611 |
| HSV1-UL48-2203 | + | CCCAGGCUGACAUCGGUCG | 19 | 17612 |
| HSV1-UL48-347 | + | CCCCAGGCUGACAUCGGUCG | 20 | 6185 |
| HSV1-UL48-2204 | + | CCCCCAGGCUGACAUCGGUCG | 21 | 17613 |
| HSV1-UL48-2205 | + | UCCCCCAGGCUGACAUCGGUCG | 22 | 17614 |
| HSV1-UL48-2206 | + | GUCCCCCAGGCUGACAUCGGUCG | 23 | 17615 |
| HSV1-UL48-2207 | + | CGUCCCCCAGGCUGACAUCGGUCG | 24 | 17616 |
| HSV1-UL48-2208 | + | CAGAGGCAGUCAAACAGG | 18 | 17617 |
| HSV1-UL48-2209 | + | ACAGAGGCAGUCAAACAGG | 19 | 17618 |
| HSV1-UL48-967 | + | AACAGAGGCAGUCAAACAGG | 20 | 6669 |
| HSV1-UL48-2210 | + | CAACAGAGGCAGUCAAACAGG | 21 | 17619 |
| HSV1-UL48-2211 | + | GCAACAGAGGCAGUCAAACAGG | 22 | 17620 |
| HSV1-UL48-2212 | + | CGCAACAGAGGCAGUCAAACAGG | 23 | 17621 |
| HSV1-UL48-2213 | + | UCGCAACAGAGGCAGUCAAACAGG | 24 | 17622 |
| HSV1-UL48-2214 | + | GCCUGGUUGCCAUGCAGG | 18 | 17623 |
| HSV1-UL48-2215 | + | GGCCUGGUUGCCAUGCAGG | 19 | 17624 |
| HSV1-UL48-985 | + | GGGCCUGGUUGCCAUGCAGG | 20 | 6681 |
| HSV1-UL48-2216 | + | CGGGCCUGGUUGCCAUGCAGG | 21 | 17625 |
| HSV1-UL48-2217 | + | GCGGGCCUGGUUGCCAUGCAGG | 22 | 17626 |
| HSV1-UL48-2218 | + | CGCGGGCCUGGUUGCCAUGCAGG | 23 | 17627 |
| HSV1-UL48-2219 | + | GCGCGGGCCUGGUUGCCAUGCAGG | 24 | 17628 |
| HSV1-UL48-2220 | + | GCAUGACCGCCUCGGAGG | 18 | 17629 |
| HSV1-UL48-2221 | + | CGCAUGACCGCCUCGGAGG | 19 | 17630 |
| HSV1-UL48-994 | + | CCGCAUGACCGCCUCGGAGG | 20 | 6689 |
| HSV1-UL48-2222 | + | CCCGCAUGACCGCCUCGGAGG | 21 | 17631 |
| HSV1-UL48-2223 | + | UCCCGCAUGACCGCCUCGGAGG | 22 | 17632 |
| HSV1-UL48-2224 | + | UUCCCGCAUGACCGCCUCGGAGG | 23 | 17633 |
| HSV1-UL48-2225 | + | GUUCCCGCAUGACCGCCUCGGAGG | 24 | 17634 |
| HSV1-UL48-2226 | + | CCCCCAGGCUGACAUCGG | 18 | 17635 |
| HSV1-UL48-2227 | + | UCCCCCAGGCUGACAUCGG | 19 | 17636 |
| HSV1-UL48-1017 | + | GUCCCCCAGGCUGACAUCGG | 20 | 6699 |
| HSV1-UL48-2228 | + | CGUCCCCCAGGCUGACAUCGG | 21 | 17637 |
| HSV1-UL48-2229 | + | UCGUCCCCCAGGCUGACAUCGG | 22 | 17638 |
| HSV1-UL48-2230 | + | CUCGUCCCCCAGGCUGACAUCGG | 23 | 17639 |
| HSV1-UL48-2231 | + | GCUCGUCCCCCAGGCUGACAUCGG | 24 | 17640 |
| HSV1-UL48-2232 | + | CAGGCUGACAUCGGUCGG | 18 | 17641 |
| HSV1-UL48-2233 | + | CCAGGCUGACAUCGGUCGG | 19 | 17642 |
| HSV1-UL48-348 | + | CCCAGGCUGACAUCGGUCGG | 20 | 6186 |
| HSV1-UL48-2234 | + | CCCCAGGCUGACAUCGGUCGG | 21 | 17643 |
| HSV1-UL48-2235 | + | CCCCCAGGCUGACAUCGGUCGG | 22 | 17644 |
| HSV1-UL48-2236 | + | UCCCCCAGGCUGACAUCGGUCGG | 23 | 17645 |
| HSV1-UL48-2237 | + | GUCCCCCAGGCUGACAUCGGUCGG | 24 | 17646 |
| HSV1-UL48-2238 | + | GUUGACGAACAUGAAGGG | 18 | 17647 |
| HSV1-UL48-2239 | + | CGUUGACGAACAUGAAGGG | 19 | 17648 |
| HSV1-UL48-970 | + | CCGUUGACGAACAUGAAGGG | 20 | 6671 |
| HSV1-UL48-2240 | + | UCCGUUGACGAACAUGAAGGG | 21 | 17649 |
| HSV1-UL48-2241 | + | CUCCGUUGACGAACAUGAAGGG | 22 | 17650 |
| HSV1-UL48-2242 | + | GCUCCGUUGACGAACAUGAAGGG | 23 | 17651 |
| HSV1-UL48-2243 | + | CGCUCCGUUGACGAACAUGAAGGG | 24 | 17652 |
| HSV1-UL48-2244 | + | GCAGGUACCGGUACAGGG | 18 | 17653 |
| HSV1-UL48-2245 | + | CGCAGGUACCGGUACAGGG | 19 | 17654 |
| HSV1-UL48-961 | + | GCGCAGGUACCGGUACAGGG | 20 | 6663 |
| HSV1-UL48-2246 | + | CGCGCAGGUACCGGUACAGGG | 21 | 17655 |
| HSV1-UL48-2247 | + | GCGCGCAGGUACCGGUACAGGG | 22 | 17656 |
| HSV1-UL48-2248 | + | GGCGCGCAGGUACCGGUACAGGG | 23 | 17657 |
| HSV1-UL48-2249 | + | UGGCGCGCAGGUACCGGUACAGGG | 24 | 17658 |
| HSV1-UL48-2250 | + | CCUGGUUGCCAUGCAGGG | 18 | 17659 |
| HSV1-UL48-2251 | + | GCCUGGUUGCCAUGCAGGG | 19 | 17660 |
| HSV1-UL48-377 | + | GGCCUGGUUGCCAUGCAGGG | 20 | 6215 |
| HSV1-UL48-2252 | + | GGGCCUGGUUGCCAUGCAGGG | 21 | 17661 |
| HSV1-UL48-2253 | + | CGGGCCUGGUUGCCAUGCAGGG | 22 | 17662 |
| HSV1-UL48-2254 | + | GCGGGCCUGGUUGCCAUGCAGGG | 23 | 17663 |
| HSV1-UL48-2255 | + | CGCGGGCCUGGUUGCCAUGCAGGG | 24 | 17664 |
| HSV1-UL48-2256 | + | GCGCGUGUGUCCCGCGGG | 18 | 17665 |
| HSV1-UL48-2257 | + | UGCGCGUGUGUCCCGCGGG | 19 | 17666 |
| HSV1-UL48-1008 | + | CUGCGCGUGUGUCCCGCGGG | 20 | 6697 |
| HSV1-UL48-2258 | + | UCUGCGCGUGUGUCCCGCGGG | 21 | 17667 |
| HSV1-UL48-2259 | + | GUCUGCGCGUGUGUCCCGCGGG | 22 | 17668 |
| HSV1-UL48-2260 | + | AGUCUGCGCGUGUGUCCCGCGGG | 23 | 17669 |
| HSV1-UL48-2261 | + | CAGUCUGCGCGUGUGUCCCGCGGG | 24 | 17670 |
| HSV1-UL48-2262 | + | UCCAGAGCGCCGUAGGGG | 18 | 17671 |
| HSV1-UL48-2263 | + | AUCCAGAGCGCCGUAGGGG | 19 | 17672 |
| HSV1-UL48-1030 | + | UAUCCAGAGCGCCGUAGGGG | 20 | 6704 |
| HSV1-UL48-2264 | + | AUAUCCAGAGCGCCGUAGGGG | 21 | 17673 |
| HSV1-UL48-2265 | + | CAUAUCCAGAGCGCCGUAGGGG | 22 | 17674 |
| HSV1-UL48-2266 | + | CCAUAUCCAGAGCGCCGUAGGGG | 23 | 17675 |
| HSV1-UL48-2267 | + | GCCAUAUCCAGAGCGCCGUAGGGG | 24 | 17676 |
| HSV1-UL48-2268 | + | GGUUGCCAUGCAGGGUGG | 18 | 17677 |
| HSV1-UL48-2269 | + | UGGUUGCCAUGCAGGGUGG | 19 | 17678 |
| HSV1-UL48-982 | + | CUGGUUGCCAUGCAGGGUGG | 20 | 6680 |
| HSV1-UL48-2270 | + | CCUGGUUGCCAUGCAGGGUGG | 21 | 17679 |
| HSV1-UL48-2271 | + | GCCUGGUUGCCAUGCAGGGUGG | 22 | 17680 |
| HSV1-UL48-2272 | + | GGCCUGGUUGCCAUGCAGGGUGG | 23 | 17681 |
| HSV1-UL48-2273 | + | GGGCCUGGUUGCCAUGCAGGGUGG | 24 | 17682 |
| HSV1-UL48-2274 | + | GCUCCGUUGACGAACAUG | 18 | 17683 |
| HSV1-UL48-2275 | + | CGCUCCGUUGACGAACAUG | 19 | 17684 |
| HSV1-UL48-971 | + | GCGCUCCGUUGACGAACAUG | 20 | 6672 |
| HSV1-UL48-2276 | + | AGCGCUCCGUUGACGAACAUG | 21 | 17685 |
| HSV1-UL48-2277 | + | GAGCGCUCCGUUGACGAACAUG | 22 | 17686 |
| HSV1-UL48-2278 | + | UGAGCGCUCCGUUGACGAACAUG | 23 | 17687 |
| HSV1-UL48-2279 | + | GUGAGCGCUCCGUUGACGAACAUG | 24 | 17688 |
| HSV1-UL48-2280 | + | CGCCUGGCGGUGCAGCUG | 18 | 17689 |
| HSV1-UL48-2281 | + | GCGCCUGGCGGUGCAGCUG | 19 | 17690 |
| HSV1-UL48-963 | + | UGCGCCUGGCGGUGCAGCUG | 20 | 6665 |
| HSV1-UL48-2282 | + | GUGCGCCUGGCGGUGCAGCUG | 21 | 17691 |
| HSV1-UL48-2283 | + | UGUGCGCCUGGCGGUGCAGCUG | 22 | 17692 |
| HSV1-UL48-2284 | + | AUGUGCGCCUGGCGGUGCAGCUG | 23 | 17693 |
| HSV1-UL48-2285 | + | CAUGUGCGCCUGGCGGUGCAGCUG | 24 | 17694 |
| HSV1-UL48-2286 | + | ACGGUGAGCGCUCCGUUG | 18 | 17695 |
| HSV1-UL48-2287 | + | GACGGUGAGCGCUCCGUUG | 19 | 17696 |
| HSV1-UL48-973 | + | GGACGGUGAGCGCUCCGUUG | 20 | 6674 |
| HSV1-UL48-2288 | + | CGGACGGUGAGCGCUCCGUUG | 21 | 17697 |
| HSV1-UL48-2289 | + | CCGGACGGUGAGCGCUCCGUUG | 22 | 17698 |
| HSV1-UL48-2290 | + | CCCGGACGGUGAGCGCUCCGUUG | 23 | 17699 |
| HSV1-UL48-2291 | + | CCCCGGACGGUGAGCGCUCCGUUG | 24 | 17700 |
| HSV1-UL48-2292 | + | GUUCCCGCAUGACCGCCU | 18 | 17701 |
| HSV1-UL48-2293 | + | UGUUCCCGCAUGACCGCCU | 19 | 17702 |
| HSV1-UL48-366 | + | GUGUUCCCGCAUGACCGCCU | 20 | 6204 |
| HSV1-UL48-2294 | + | CGUGUUCCCGCAUGACCGCCU | 21 | 17703 |
| HSV1-UL48-2295 | + | GCGUGUUCCCGCAUGACCGCCU | 22 | 17704 |
| HSV1-UL48-2296 | + | CGCGUGUUCCCGCAUGACCGCCU | 23 | 17705 |
| HSV1-UL48-2297 | + | ACGCGUGUUCCCGCAUGACCGCCU | 24 | 17706 |
| HSV1-UL48-2298 | + | GCCGCCAGCCCCGCCUCU | 18 | 17707 |
| HSV1-UL48-2299 | + | AGCCGCCAGCCCCGCCUCU | 19 | 17708 |
| HSV1-UL48-1004 | + | GAGCCGCCAGCCCCGCCUCU | 20 | 6694 |
| HSV1-UL48-2300 | + | GGAGCCGCCAGCCCCGCCUCU | 21 | 17709 |
| HSV1-UL48-2301 | + | CGGAGCCGCCAGCCCCGCCUCU | 22 | 17710 |
| HSV1-UL48-2302 | + | GCGGAGCCGCCAGCCCCGCCUCU | 23 | 17711 |
| HSV1-UL48-2303 | + | CGCGGAGCCGCCAGCCCCGCCUCU | 24 | 17712 |
| HSV1-UL48-2304 | + | CCAUAUCCAGAGCGCCGU | 18 | 17713 |
| HSV1-UL48-2305 | + | GCCAUAUCCAGAGCGCCGU | 19 | 17714 |
| HSV1-UL48-327 | + | GGCCAUAUCCAGAGCGCCGU | 20 | 6165 |
| HSV1-UL48-2306 | + | CGGCCAUAUCCAGAGCGCCGU | 21 | 17715 |
| HSV1-UL48-2307 | + | UCGGCCAUAUCCAGAGCGCCGU | 22 | 17716 |
| HSV1-UL48-2308 | + | GUCGGCCAUAUCCAGAGCGCCGU | 23 | 17717 |
| HSV1-UL48-2309 | + | AGUCGGCCAUAUCCAGAGCGCCGU | 24 | 17718 |
| HSV1-UL48-2310 | + | CGUAGGGGGCGGAGUCGU | 18 | 17719 |
| HSV1-UL48-2311 | + | CCGUAGGGGGCGGAGUCGU | 19 | 17720 |
| HSV1-UL48-333 | + | GCCGUAGGGGGCGGAGUCGU | 20 | 6171 |
| HSV1-UL48-2312 | + | CGCCGUAGGGGGCGGAGUCGU | 21 | 17721 |
| HSV1-UL48-2313 | + | GCGCCGUAGGGGGCGGAGUCGU | 22 | 17722 |
| HSV1-UL48-2314 | + | AGCGCCGUAGGGGGCGGAGUCGU | 23 | 17723 |
| HSV1-UL48-2315 | + | GAGCGCCGUAGGGGGCGGAGUCGU | 24 | 17724 |
| HSV1-UL48-2316 | + | CUUCGGGGGCGUCGUCGU | 18 | 17725 |
| HSV1-UL48-2317 | + | UCUUCGGGGGCGUCGUCGU | 19 | 17726 |
| HSV1-UL48-1001 | + | CUCUUCGGGGGCGUCGUCGU | 20 | 6693 |
| HSV1-UL48-2318 | + | CCUCUUCGGGGGCGUCGUCGU | 21 | 17727 |
| HSV1-UL48-2319 | + | GCCUCUUCGGGGGCGUCGUCGU | 22 | 17728 |
| HSV1-UL48-2320 | + | CGCCUCUUCGGGGGCGUCGUCGU | 23 | 17729 |
| HSV1-UL48-2321 | + | CCGCCUCUUCGGGGGCGUCGUCGU | 24 | 17730 |
| HSV1-UL48-2322 | + | CCCCAGGCUGACAUCGGU | 18 | 17731 |
| HSV1-UL48-2323 | + | CCCCCAGGCUGACAUCGGU | 19 | 17732 |
| HSV1-UL48-345 | + | UCCCCCAGGCUGACAUCGGU | 20 | 6183 |
| HSV1-UL48-2324 | + | GUCCCCCAGGCUGACAUCGGU | 21 | 17733 |
| HSV1-UL48-2325 | + | CGUCCCCCAGGCUGACAUCGGU | 22 | 17734 |
| HSV1-UL48-2326 | + | UCGUCCCCCAGGCUGACAUCGGU | 23 | 17735 |
| HSV1-UL48-2327 | + | CUCGUCCCCCAGGCUGACAUCGGU | 24 | 17736 |
| HSV1-UL48-2328 | + | CUGGUUGCCAUGCAGGGU | 18 | 17737 |
| HSV1-UL48-2329 | + | CCUGGUUGCCAUGCAGGGU | 19 | 17738 |
| HSV1-UL48-378 | + | GCCUGGUUGCCAUGCAGGGU | 20 | 6216 |
| HSV1-UL48-2330 | + | GGCCUGGUUGCCAUGCAGGGU | 21 | 17739 |
| HSV1-UL48-2331 | + | GGGCCUGGUUGCCAUGCAGGGU | 22 | 17740 |
| HSV1-UL48-2332 | + | CGGGCCUGGUUGCCAUGCAGGGU | 23 | 17741 |
| HSV1-UL48-2333 | + | GCGGGCCUGGUUGCCAUGCAGGGU | 24 | 17742 |
| HSV1-UL48-2334 | + | CCGCCAGCCCCGCCUCUU | 18 | 17743 |
| HSV1-UL48-2335 | + | GCCGCCAGCCCCGCCUCUU | 19 | 17744 |
| HSV1-UL48-357 | + | AGCCGCCAGCCCCGCCUCUU | 20 | 6195 |
| HSV1-UL48-2336 | + | GAGCCGCCAGCCCCGCCUCUU | 21 | 17745 |
| HSV1-UL48-2337 | + | GGAGCCGCCAGCCCCGCCUCUU | 22 | 17746 |
| HSV1-UL48-2338 | + | CGGAGCCGCCAGCCCCGCCUCUU | 23 | 17747 |
| HSV1-UL48-2339 | + | GCGGAGCCGCCAGCCCCGCCUCUU | 24 | 17748 |
| HSV1-UL48-2340 | - | CCGUCCGGGGAGUGCCAA | 18 | 17749 |
| HSV1-UL48-2341 | - | ACCGUCCGGGGAGUGCCAA | 19 | 17750 |
| HSV1-UL48-827 | - | CACCGUCCGGGGAGUGCCAA | 20 | 6576 |
| HSV1-UL48-2342 | - | UCACCGUCCGGGGAGUGCCAA | 21 | 17751 |
| HSV1-UL48-2343 | - | CUCACCGUCCGGGGAGUGCCAA | 22 | 17752 |
| HSV1-UL48-2344 | - | GCUCACCGUCCGGGGAGUGCCAA | 23 | 17753 |
| HSV1-UL48-2345 | - | CGCUCACCGUCCGGGGAGUGCCAA | 24 | 17754 |
| HSV1-UL48-2346 | - | GCCCUUCAUGUUCGUCAA | 18 | 17755 |
| HSV1-UL48-2347 | - | AGCCCUUCAUGUUCGUCAA | 19 | 17756 |
| HSV1-UL48-254 | - | CAGCCCUUCAUGUUCGUCAA | 20 | 6092 |
| HSV1-UL48-2348 | - | CCAGCCCUUCAUGUUCGUCAA | 21 | 17757 |
| HSV1-UL48-2349 | - | UCCAGCCCUUCAUGUUCGUCAA | 22 | 17758 |
| HSV1-UL48-2350 | - | UUCCAGCCCUUCAUGUUCGUCAA | 23 | 17759 |
| HSV1-UL48-2351 | - | GUUCCAGCCCUUCAUGUUCGUCAA | 24 | 17760 |
| HSV1-UL48-2352 | - | ACAAUUACGGGUCUACCA | 18 | 17761 |
| HSV1-UL48-2353 | - | AACAAUUACGGGUCUACCA | 19 | 17762 |
| HSV1-UL48-848 | - | AAACAAUUACGGGUCUACCA | 20 | 6590 |
| HSV1-UL48-2354 | - | AAAACAAUUACGGGUCUACCA | 21 | 17763 |
| HSV1-UL48-2355 | - | AAAAACAAUUACGGGUCUACCA | 22 | 17764 |
| HSV1-UL48-2356 | - | GAAAAACAAUUACGGGUCUACCA | 23 | 17765 |
| HSV1-UL48-2357 | - | CGAAAAACAAUUACGGGUCUACCA | 24 | 17766 |
| HSV1-UL48-2358 | - | AGCCCUUCAUGUUCGUCA | 18 | 17767 |
| HSV1-UL48-2359 | - | CAGCCCUUCAUGUUCGUCA | 19 | 17768 |
| HSV1-UL48-821 | - | CCAGCCCUUCAUGUUCGUCA | 20 | 6574 |
| HSV1-UL48-2360 | - | UCCAGCCCUUCAUGUUCGUCA | 21 | 17769 |
| HSV1-UL48-2361 | - | UUCCAGCCCUUCAUGUUCGUCA | 22 | 17770 |
| HSV1-UL48-2362 | - | GUUCCAGCCCUUCAUGUUCGUCA | 23 | 17771 |
| HSV1-UL48-2363 | - | UGUUCCAGCCCUUCAUGUUCGUCA | 24 | 17772 |
| HSV1-UL48-2364 | - | GGUGGGUAGGGGGCGCGA | 18 | 17773 |
| HSV1-UL48-2365 | - | CGGUGGGUAGGGGGCGCGA | 19 | 17774 |
| HSV1-UL48-2366 | - | ACGGUGGGUAGGGGGCGCGA | 20 | 17775 |
| HSV1-UL48-2367 | - | UACGGUGGGUAGGGGGCGCGA | 21 | 17776 |
| HSV1-UL48-2368 | - | GUACGGUGGGUAGGGGGCGCGA | 22 | 17777 |
| HSV1-UL48-2369 | - | AGUACGGUGGGUAGGGGGCGCGA | 23 | 17778 |
| HSV1-UL48-2370 | - | GAGUACGGUGGGUAGGGGGCGCGA | 24 | 17779 |
| HSV1-UL48-2371 | - | UCUGGACAUGUUGGGGGA | 18 | 17780 |
| HSV1-UL48-2372 | - | AUCUGGACAUGUUGGGGGA | 19 | 17781 |
| HSV1-UL48-308 | - | GAUCUGGACAUGUUGGGGGA | 20 | 6146 |
| HSV1-UL48-2373 | - | CGAUCUGGACAUGUUGGGGGA | 21 | 17782 |
| HSV1-UL48-2374 | - | UCGAUCUGGACAUGUUGGGGGA | 22 | 17783 |
| HSV1-UL48-2375 | - | UUCGAUCUGGACAUGUUGGGGGA | 23 | 17784 |
| HSV1-UL48-2376 | - | UUUCGAUCUGGACAUGUUGGGGGA | 24 | 17785 |
| HSV1-UL48-2377 | - | GAUCGCGGACAGGUACUA | 18 | 17786 |
| HSV1-UL48-2378 | - | CGAUCGCGGACAGGUACUA | 19 | 17787 |
| HSV1-UL48-812 | - | ACGAUCGCGGACAGGUACUA | 20 | 6566 |
| HSV1-UL48-2379 | - | CACGAUCGCGGACAGGUACUA | 21 | 17788 |
| HSV1-UL48-2380 | - | CCACGAUCGCGGACAGGUACUA | 22 | 17789 |
| HSV1-UL48-2381 | - | GCCACGAUCGCGGACAGGUACUA | 23 | 17790 |
| HSV1-UL48-2382 | - | CGCCACGAUCGCGGACAGGUACUA | 24 | 17791 |
| HSVI-UL48-1642 | - | GGCGCGGGAGGAGAGCUA | 18 | 17051 |
| HSV1-UL48-1643 | - | GGGCGCGGGAGGAGAGCUA | 19 | 17052 |
| HSVI-UL48-1644 | - | CGGGCGCGGGAGGAGAGCUA | 20 | 17053 |
| HSV1-UL48-1645 | - | ACGGGCGCGGGAGGAGAGCUA | 21 | 17054 |
| HSV1-UL48-1646 | - | UACGGGCGCGGGAGGAGAGCUA | 22 | 17055 |
| HSV1-UL48-1647 | - | CUACGGGCGCGGGAGGAGAGCUA | 23 | 17056 |
| HSV1-UL48-1648 | - | GCUACGGGCGCGGGAGGAGAGCUA | 24 | 17057 |
| HSV1-UL48-2383 | - | UGGUGCGCAGCGCGGCUA | 18 | 17792 |
| HSV1-UL48-2384 | - | CUGGUGCGCAGCGCGGCUA | 19 | 17793 |
| HSV1-UL48-264 | - | GCUGGUGCGCAGCGCGGCUA | 20 | 6102 |
| HSV1-UL48-2385 | - | CGCUGGUGCGCAGCGCGGCUA | 21 | 17794 |
| HSV1-UL48-2386 | - | CCGCUGGUGCGCAGCGCGGCUA | 22 | 17795 |
| HSV1-UL48-2387 | - | CCCGCUGGUGCGCAGCGCGGCUA | 23 | 17796 |
| HSV1-UL48-2388 | - | UCCCGCUGGUGCGCAGCGCGGCUA | 24 | 17797 |
| HSV1-UL48-2389 | - | UGACGAGUACGGUGGGUA | 18 | 17798 |
| HSV1-UL48-2390 | - | UUGACGAGUACGGUGGGUA | 19 | 17799 |
| HSV1-UL48-1153 | - | AUUGACGAGUACGGUGGGUA | 20 | 16562 |
| HSV1-UL48-2391 | - | AAUUGACGAGUACGGUGGGUA | 21 | 17800 |
| HSV1-UL48-2392 | - | GAAUUGACGAGUACGGUGGGUA | 22 | 17801 |
| HSV1-UL48-2393 | - | GGAAUUGACGAGUACGGUGGGUA | 23 | 17802 |
| HSV1-UL48-2394 | - | UGGAAUUGACGAGUACGGUGGGUA | 24 | 17803 |
| HSV1-UL48-2395 | - | CGCGGACGCGAUCGCGAC | 18 | 17804 |
| HSV1-UL48-2396 | - | GCGCGGACGCGAUCGCGAC | 19 | 17805 |
| HSV1-UL48-807 | - | UGCGCGGACGCGAUCGCGAC | 20 | 6563 |
| HSV1-UL48-2397 | - | AUGCGCGGACGCGAUCGCGAC | 21 | 17806 |
| HSV1-UL48-2398 | - | CAUGCGCGGACGCGAUCGCGAC | 22 | 17807 |
| HSV1-UL48-2399 | - | ACAUGCGCGGACGCGAUCGCGAC | 23 | 17808 |
| HSV1-UL48-2400 | - | CACAUGCGCGGACGCGAUCGCGAC | 24 | 17809 |
| HSV1-UL48-2401 | - | GACUGCCUCUGUUGCGAC | 18 | 17810 |
| HSV1-UL48-2402 | - | UGACUGCCUCUGUUGCGAC | 19 | 17811 |
| HSV1-UL48-817 | - | UUGACUGCCUCUGUUGCGAC | 20 | 6571 |
| HSV1-UL48-2403 | - | UUUGACUGCCUCUGUUGCGAC | 21 | 17812 |
| HSV1-UL48-2404 | - | GUUUGACUGCCUCUGUUGCGAC | 22 | 17813 |
| HSV1-UL48-2405 | - | UGUUUGACUGCCUCUGUUGCGAC | 23 | 17814 |
| HSV1-UL48-2406 | - | CUGUUUGACUGCCUCUGUUGCGAC | 24 | 17815 |
| HSV1-UL48-2407 | - | GCGGACGCGAUCGCGACC | 18 | 17816 |
| HSV1-UL48-2408 | - | CGCGGACGCGAUCGCGACC | 19 | 17817 |
| HSV1-UL48-241 | - | GCGCGGACGCGAUCGCGACC | 20 | 6079 |
| HSV1-UL48-2409 | - | UGCGCGGACGCGAUCGCGACC | 21 | 17818 |
| HSV1-UL48-2410 | - | AUGCGCGGACGCGAUCGCGACC | 22 | 17819 |
| HSV1-UL48-2411 | - | CAUGCGCGGACGCGAUCGCGACC | 23 | 17820 |
| HSV1-UL48-2412 | - | ACAUGCGCGGACGCGAUCGCGACC | 24 | 17821 |
| HSV1-UL48-2413 | - | ACUGCCUCUGUUGCGACC | 18 | 17822 |
| HSV1-UL48-2414 | - | GACUGCCUCUGUUGCGACC | 19 | 17823 |
| HSV1-UL48-249 | - | UGACUGCCUCUGUUGCGACC | 20 | 6087 |
| HSV1-UL48-2415 | - | UUGACUGCCUCUGUUGCGACC | 21 | 17824 |
| HSV1-UL48-2416 | - | UUUGACUGCCUCUGUUGCGACC | 22 | 17825 |
| HSV1-UL48-2417 | - | GUUUGACUGCCUCUGUUGCGACC | 23 | 17826 |
| HSV1-UL48-2418 | - | UGUUUGACUGCCUCUGUUGCGACC | 24 | 17827 |
| HSV1-UL48-2419 | - | UCGCGGACAGGUACUACC | 18 | 17828 |
| HSV1-UL48-2420 | - | AUCGCGGACAGGUACUACC | 19 | 17829 |
| HSV1-UL48-813 | - | GAUCGCGGACAGGUACUACC | 20 | 6567 |
| HSV1-UL48-2421 | - | CGAUCGCGGACAGGUACUACC | 21 | 17830 |
| HSV1-UL48-2422 | - | ACGAUCGCGGACAGGUACUACC | 22 | 17831 |
| HSV1-UL48-2423 | - | CACGAUCGCGGACAGGUACUACC | 23 | 17832 |
| HSV1-UL48-2424 | - | CCACGAUCGCGGACAGGUACUACC | 24 | 17833 |
| HSV1-UL48-2425 | - | UGUAUCUAUUUUUGACCC | 18 | 17834 |
| HSV1-UL48-2426 | - | UUGUAUCUAUUUUUGACCC | 19 | 17835 |
| HSV1-UL48-814 | - | UUUGUAUCUAUUUUUGACCC | 20 | 6568 |
| HSV1-UL48-2427 | - | AUUUGUAUCUAUUUUUGACCC | 21 | 17836 |
| HSV1-UL48-2428 | - | CAUUUGUAUCUAUUUUUGACCC | 22 | 17837 |
| HSV1-UL48-2429 | - | GCAUUUGUAUCUAUUUUUGACCC | 23 | 17838 |
| HSV1-UL48-2430 | - | UGCAUUUGUAUCUAUUUUUGACCC | 24 | 17839 |
| HSV1-UL48-2431 | - | CGCCUGUCCUUUCUCCCC | 18 | 17840 |
| HSV1-UL48-2432 | - | GCGCCUGUCCUUUCUCCCC | 19 | 17841 |
| HSV1-UL48-855 | - | CGCGCCUGUCCUUUCUCCCC | 20 | 6596 |
| HSV1-UL48-2433 | - | CCGCGCCUGUCCUUUCUCCCC | 21 | 17842 |
| HSV1-UL48-2434 | - | UCCGCGCCUGUCCUUUCUCCCC | 22 | 17843 |
| HSV1-UL48-2435 | - | CUCCGCGCCUGUCCUUUCUCCCC | 23 | 17844 |
| HSV1-UL48-2436 | - | GCUCCGCGCCUGUCCUUUCUCCCC | 24 | 17845 |
| HSV1-UL48-2437 | - | UCCCGGACGACGACGCCC | 18 | 17846 |
| HSV1-UL48-2438 | - | CUCCCGGACGACGACGCCC | 19 | 17847 |
| HSV1-UL48-851 | - | UCUCCCGGACGACGACGCCC | 20 | 6593 |
| HSV1-UL48-2439 | - | AUCUCCCGGACGACGACGCCC | 21 | 17848 |
| HSV1-UL48-2440 | - | GAUCUCCCGGACGACGACGCCC | 22 | 17849 |
| HSV1-UL48-2441 | - | CGAUCUCCCGGACGACGACGCCC | 23 | 17850 |
| HSV1-UL48-2442 | - | UCGAUCUCCCGGACGACGACGCCC | 24 | 17851 |
| HSV1-UL48-2443 | - | AGAUGUUUACCGAUGCCC | 18 | 17852 |
| HSV1-UL48-2444 | - | CAGAUGUUUACCGAUGCCC | 19 | 17853 |
| HSV1-UL48-879 | - | GCAGAUGUUUACCGAUGCCC | 20 | 6609 |
| HSV1-UL48-2445 | - | AGCAGAUGUUUACCGAUGCCC | 21 | 17854 |
| HSV1-UL48-2446 | - | GAGCAGAUGUUUACCGAUGCCC | 22 | 17855 |
| HSV1-UL48-2447 | - | UGAGCAGAUGUUUACCGAUGCCC | 23 | 17856 |
| HSV1-UL48-2448 | - | UUGAGCAGAUGUUUACCGAUGCCC | 24 | 17857 |
| HSV1-UL48-2449 | - | UGGGGGACGGGGAUUCCC | 18 | 17858 |
| HSV1-UL48-2450 | - | UUGGGGGACGGGGAUUCCC | 19 | 17859 |
| HSV1-UL48-311 | - | GUUGGGGGACGGGGAUUCCC | 20 | 6149 |
| HSV1-UL48-2451 | - | UGUUGGGGGACGGGGAUUCCC | 21 | 17860 |
| HSV1-UL48-2452 | - | AUGUUGGGGGACGGGGAUUCCC | 22 | 17861 |
| HSV1-UL48-2453 | - | CAUGUUGGGGGACGGGGAUUCCC | 23 | 17862 |
| HSV1-UL48-2454 | - | ACAUGUUGGGGGACGGGGAUUCCC | 24 | 17863 |
| HSV1-UL48-2455 | - | CCCCGACCGAUGUCAGCC | 18 | 17864 |
| HSV1-UL48-2456 | - | CCCCCGACCGAUGUCAGCC | 19 | 17865 |
| HSV1-UL48-295 | - | CCCCCCGACCGAUGUCAGCC | 20 | 6133 |
| HSV1-UL48-2457 | - | CCCCCCCGACCGAUGUCAGCC | 21 | 17866 |
| HSV1-UL48-2458 | - | GCCCCCCCGACCGAUGUCAGCC | 22 | 17867 |
| HSV1-UL48-2459 | - | GGCCCCCCCGACCGAUGUCAGCC | 23 | 17868 |
| HSV1-UL48-2460 | - | CGGCCCCCCCGACCGAUGUCAGCC | 24 | 17869 |
| HSV1-UL48-2461 | - | CGCGGCUACGGAGGAGCC | 18 | 17870 |
| HSV1-UL48-2462 | - | GCGCGGCUACGGAGGAGCC | 19 | 17871 |
| HSV1-UL48-266 | - | AGCGCGGCUACGGAGGAGCC | 20 | 6104 |
| HSV1-UL48-2463 | - | CAGCGCGGCUACGGAGGAGCC | 21 | 17872 |
| HSV1-UL48-2464 | - | GCAGCGCGGCUACGGAGGAGCC | 22 | 17873 |
| HSV1-UL48-2465 | - | CGCAGCGCGGCUACGGAGGAGCC | 23 | 17874 |
| HSV1-UL48-2466 | - | GCGCAGCGCGGCUACGGAGGAGCC | 24 | 17875 |
| HSV1-UL48-2467 | - | ACGGAGCGCUCACCGUCC | 18 | 17876 |
| HSV1-UL48-2468 | - | AACGGAGCGCUCACCGUCC | 19 | 17877 |
| HSV1-UL48-256 | - | CAACGGAGCGCUCACCGUCC | 20 | 6094 |
| HSV1-UL48-2469 | - | UCAACGGAGCGCUCACCGUCC | 21 | 17878 |
| HSV1-UL48-2470 | - | GUCAACGGAGCGCUCACCGUCC | 22 | 17879 |
| HSV1-UL48-2471 | - | CGUCAACGGAGCGCUCACCGUCC | 23 | 17880 |
| HSV1-UL48-2472 | - | UCGUCAACGGAGCGCUCACCGUCC | 24 | 17881 |
| HSV1-UL48-2473 | - | UUGGGGGACGGGGAUUCC | 18 | 17882 |
| HSV1-UL48-2474 | - | GUUGGGGGACGGGGAUUCC | 19 | 17883 |
| HSV1-UL48-872 | - | UGUUGGGGGACGGGGAUUCC | 20 | 6604 |
| HSV1-UL48-2475 | - | AUGUUGGGGGACGGGGAUUCC | 21 | 17884 |
| HSV1-UL48-2476 | - | CAUGUUGGGGGACGGGGAUUCC | 22 | 17885 |
| HSV1-UL48-2477 | - | ACAUGUUGGGGGACGGGGAUUCC | 23 | 17886 |
| HSV1-UL48-2478 | - | GACAUGUUGGGGGACGGGGAUUCC | 24 | 17887 |
| HSV1-UL48-2479 | - | CCCCCGACCGAUGUCAGC | 18 | 17888 |
| HSV1-UL48-2480 | - | CCCCCCGACCGAUGUCAGC | 19 | 17889 |
| HSV1-UL48-857 | - | CCCCCCCGACCGAUGUCAGC | 20 | 6597 |
| HSV1-UL48-2481 | - | GCCCCCCCGACCGAUGUCAGC | 21 | 17890 |
| HSV1-UL48-2482 | - | GGCCCCCCCGACCGAUGUCAGC | 22 | 17891 |
| HSV1-UL48-2483 | - | CGGCCCCCCCGACCGAUGUCAGC | 23 | 17892 |
| HSV1-UL48-2484 | - | ACGGCCCCCCCGACCGAUGUCAGC | 24 | 17893 |
| HSV1-UL48-2485 | - | GCGCGGCUACGGAGGAGC | 18 | 17894 |
| HSV1-UL48-2486 | - | AGCGCGGCUACGGAGGAGC | 19 | 17895 |
| HSV1-UL48-836 | - | CAGCGCGGCUACGGAGGAGC | 20 | 6581 |
| HSV1-UL48-2487 | - | GCAGCGCGGCUACGGAGGAGC | 21 | 17896 |
| HSV1-UL48-2488 | - | CGCAGCGCGGCUACGGAGGAGC | 22 | 17897 |
| HSV1-UL48-2489 | - | GCGCAGCGCGGCUACGGAGGAGC | 23 | 17898 |
| HSV1-UL48-2490 | - | UGCGCAGCGCGGCUACGGAGGAGC | 24 | 17899 |
| HSV1-UL48-1709 | - | ACGCCGAGCUACGGGCGC | 18 | 17118 |
| HSV1-UL48-1710 | - | CACGCCGAGCUACGGGCGC | 19 | 17119 |
| HSV1-UL48-39 | - | CCACGCCGAGCUACGGGCGC | 20 | 5880 |
| HSV1-UL48-2491 | - | GAGCUCCACUUAGACGGC | 18 | 17900 |
| HSV1-UL48-2492 | - | CGAGCUCCACUUAGACGGC | 19 | 17901 |
| HSV1-UL48-863 | - | ACGAGCUCCACUUAGACGGC | 20 | 6600 |
| HSV1-UL48-2493 | - | GACGAGCUCCACUUAGACGGC | 21 | 17902 |
| HSV1-UL48-2494 | - | GGACGAGCUCCACUUAGACGGC | 22 | 17903 |
| HSV1-UL48-2495 | - | GGGACGAGCUCCACUUAGACGGC | 23 | 17904 |
| HSV1-UL48-2496 | - | GGGGACGAGCUCCACUUAGACGGC | 24 | 17905 |
| HSV1-UL48-2497 | - | ACCGCCAGGCGCACAUGC | 18 | 17906 |
| HSV1-UL48-2498 | - | CACCGCCAGGCGCACAUGC | 19 | 17907 |
| HSV1-UL48-806 | - | GCACCGCCAGGCGCACAUGC | 20 | 6562 |
| HSV1-UL48-2499 | - | UGCACCGCCAGGCGCACAUGC | 21 | 17908 |
| HSV1-UL48-2500 | - | CUGCACCGCCAGGCGCACAUGC | 22 | 17909 |
| HSV1-UL48-2501 | - | GCUGCACCGCCAGGCGCACAUGC | 23 | 17910 |
| HSV1-UL48-2502 | - | AGCUGCACCGCCAGGCGCACAUGC | 24 | 17911 |
| HSV1-UL48-2503 | - | CCUCCGAGGCGGUCAUGC | 18 | 17912 |
| HSV1-UL48-2504 | - | CCCUCCGAGGCGGUCAUGC | 19 | 17913 |
| HSV1-UL48-280 | - | GCCCUCCGAGGCGGUCAUGC | 20 | 6118 |
| HSV1-UL48-2505 | - | CGCCCUCCGAGGCGGUCAUGC | 21 | 17914 |
| HSV1-UL48-2506 | - | UCGCCCUCCGAGGCGGUCAUGC | 22 | 17915 |
| HSV1-UL48-2507 | - | CUCGCCCUCCGAGGCGGUCAUGC | 23 | 17916 |
| HSV1-UL48-2508 | - | CCUCGCCCUCCGAGGCGGUCAUGC | 24 | 17917 |
| HSV1-UL48-2509 | - | UCGAGGCCCGCCGGCUGC | 18 | 17918 |
| HSV1-UL48-2510 | - | AUCGAGGCCCGCCGGCUGC | 19 | 17919 |
| HSV1-UL48-261 | - | AAUCGAGGCCCGCCGGCUGC | 20 | 6099 |
| HSV1-UL48-2511 | - | CAAUCGAGGCCCGCCGGCUGC | 21 | 17920 |
| HSV1-UL48-2512 | - | CCAAUCGAGGCCCGCCGGCUGC | 22 | 17921 |
| HSV1-UL48-2513 | - | GCCAAUCGAGGCCCGCCGGCUGC | 23 | 17922 |
| HSV1-UL48-2514 | - | UGCCAAUCGAGGCCCGCCGGCUGC | 24 | 17923 |
| HSV1-UL48-2515 | - | AAUUACGGGUCUACCAUC | 18 | 17924 |
| HSV1-UL48-2516 | - | CAAUUACGGGUCUACCAUC | 19 | 17925 |
| HSV1-UL48-849 | - | ACAAUUACGGGUCUACCAUC | 20 | 6591 |
| HSV1-UL48-2517 | - | AACAAUUACGGGUCUACCAUC | 21 | 17926 |
| HSV1-UL48-2518 | - | AAACAAUUACGGGUCUACCAUC | 22 | 17927 |
| HSV1-UL48-2519 | - | AAAACAAUUACGGGUCUACCAUC | 23 | 17928 |
| HSV1-UL48-2520 | - | AAAAACAAUUACGGGUCUACCAUC | 24 | 17929 |
| HSV1-UL48-2521 | - | AUGCUGCGCGCCACGAUC | 18 | 17930 |
| HSV1-UL48-2522 | - | AAUGCUGCGCGCCACGAUC | 19 | 17931 |
| HSV1-UL48-811 | - | AAAUGCUGCGCGCCACGAUC | 20 | 6565 |
| HSV1-UL48-2523 | - | GAAAUGCUGCGCGCCACGAUC | 21 | 17932 |
| HSV1-UL48-2524 | - | AGAAAUGCUGCGCGCCACGAUC | 22 | 17933 |
| HSV1-UL48-2525 | - | GAGAAAUGCUGCGCGCCACGAUC | 23 | 17934 |
| HSV1-UL48-2526 | - | GGAGAAAUGCUGCGCGCCACGAUC | 24 | 17935 |
| HSV1-UL48-2527 | - | GAGGGCCUGCUCGAUCUC | 18 | 17936 |
| HSV1-UL48-2528 | - | CGAGGGCCUGCUCGAUCUC | 19 | 17937 |
| HSV1-UL48-850 | - | UCGAGGGCCUGCUCGAUCUC | 20 | 6592 |
| HSV1-UL48-2529 | - | AUCGAGGGCCUGCUCGAUCUC | 21 | 17938 |
| HSV1-UL48-2530 | - | CAUCGAGGGCCUGCUCGAUCUC | 22 | 17939 |
| HSV1-UL48-2531 | - | CCAUCGAGGGCCUGCUCGAUCUC | 23 | 17940 |
| HSV1-UL48-2532 | - | ACCAUCGAGGGCCUGCUCGAUCUC | 24 | 17941 |
| HSV1-UL48-2533 | - | AACGGAGCGCUCACCGUC | 18 | 17942 |
| HSV1-UL48-2534 | - | CAACGGAGCGCUCACCGUC | 19 | 17943 |
| HSV1-UL48-255 | - | UCAACGGAGCGCUCACCGUC | 20 | 6093 |
| HSV1-UL48-2535 | - | GUCAACGGAGCGCUCACCGUC | 21 | 17944 |
| HSV1-UL48-2536 | - | CGUCAACGGAGCGCUCACCGUC | 22 | 17945 |
| HSV1-UL48-2537 | - | UCGUCAACGGAGCGCUCACCGUC | 23 | 17946 |
| HSV1-UL48-2538 | - | UUCGUCAACGGAGCGCUCACCGUC | 24 | 17947 |
| HSV1-UL48-2539 | - | GGGAGCUAAACCACAUUC | 18 | 17948 |
| HSV1-UL48-2540 | - | CGGGAGCUAAACCACAUUC | 19 | 17949 |
| HSV1-UL48-831 | - | GCGGGAGCUAAACCACAUUC | 20 | 6578 |
| HSV1-UL48-2541 | - | UGCGGGAGCUAAACCACAUUC | 21 | 17950 |
| HSV1-UL48-2542 | - | CUGCGGGAGCUAAACCACAUUC | 22 | 17951 |
| HSV1-UL48-2543 | - | GCUGCGGGAGCUAAACCACAUUC | 23 | 17952 |
| HSV1-UL48-2544 | - | GGCUGCGGGAGCUAAACCACAUUC | 24 | 17953 |
| HSV1-UL48-2545 | - | GUGUUGAUUCGGGCGAAG | 18 | 17954 |
| HSV1-UL48-2546 | - | GGUGUUGAUUCGGGCGAAG | 19 | 17955 |
| HSV1-UL48-841 | - | UGGUGUUGAUUCGGGCGAAG | 20 | 6585 |
| HSV1-UL48-2547 | - | AUGGUGUUGAUUCGGGCGAAG | 21 | 17956 |
| HSV1-UL48-2548 | - | UAUGGUGUUGAUUCGGGCGAAG | 22 | 17957 |
| HSV1-UL48-2549 | - | UUAUGGUGUUGAUUCGGGCGAAG | 23 | 17958 |
| HSV1-UL48-2550 | - | UUUAUGGUGUUGAUUCGGGCGAAG | 24 | 17959 |
| HSV1-UL48-2551 | - | GACGACGCCCCCGAAGAG | 18 | 17960 |
| HSV1-UL48-2552 | - | CGACGACGCCCCCGAAGAG | 19 | 17961 |
| HSV1-UL48-853 | - | ACGACGACGCCCCCGAAGAG | 20 | 6595 |
| HSV1-UL48-2553 | - | GACGACGACGCCCCCGAAGAG | 21 | 17962 |
| HSV1-UL48-2554 | - | GGACGACGACGCCCCCGAAGAG | 22 | 17963 |
| HSV1-UL48-2555 | - | CGGACGACGACGCCCCCGAAGAG | 23 | 17964 |
| HSV1-UL48-2556 | - | CCGGACGACGACGCCCCCGAAGAG | 24 | 17965 |
| HSV1-UL48-1733 | - | GAGCUACGGGCGCGGGAG | 18 | 17142 |
| HSV1-UL48-1734 | - | CGAGCUACGGGCGCGGGAG | 19 | 17143 |
| HSV1-UL48-642 | - | CCGAGCUACGGGCGCGGGAG | 20 | 6466 |
| HSV1-UL48-1735 | - | GCCGAGCUACGGGCGCGGGAG | 21 | 17144 |
| HSV1-UL48-1736 | - | CGCCGAGCUACGGGCGCGGGAG | 22 | 17145 |
| HSV1-UL48-1737 | - | ACGCCGAGCUACGGGCGCGGGAG | 23 | 17146 |
| HSV1-UL48-1738 | - | CACGCCGAGCUACGGGCGCGGGAG | 24 | 17147 |
| HSV1-UL48-2557 | - | ACGAGCUCCACUUAGACG | 18 | 17966 |
| HSV1-UL48-2558 | - | GACGAGCUCCACUUAGACG | 19 | 17967 |
| HSV1-UL48-862 | - | GGACGAGCUCCACUUAGACG | 20 | 6599 |
| HSV1-UL48-2559 | - | GGGACGAGCUCCACUUAGACG | 21 | 17968 |
| HSV1-UL48-2560 | - | GGGGACGAGCUCCACUUAGACG | 22 | 17969 |
| HSV1-UL48-2561 | - | GGGGGACGAGCUCCACUUAGACG | 23 | 17970 |
| HSV1-UL48-2562 | - | UGGGGGACGAGCUCCACUUAGACG | 24 | 17971 |
| HSV1-UL48-2563 | - | GUGCGCAGCGCGGCUACG | 18 | 17972 |
| HSV1-UL48-2564 | - | GGUGCGCAGCGCGGCUACG | 19 | 17973 |
| HSV1-UL48-834 | - | UGGUGCGCAGCGCGGCUACG | 20 | 6580 |
| HSV1-UL48-2565 | - | CUGGUGCGCAGCGCGGCUACG | 21 | 17974 |
| HSV1-UL48-2566 | - | GCUGGUGCGCAGCGCGGCUACG | 22 | 17975 |
| HSV1-UL48-2567 | - | CGCUGGUGCGCAGCGCGGCUACG | 23 | 17976 |
| HSV1-UL48-2568 | - | CCGCUGGUGCGCAGCGCGGCUACG | 24 | 17977 |
| HSV1-UL48-2569 | - | UCCUAUGGGCCGCGUACG | 18 | 17978 |
| HSV1-UL48-2570 | - | AUCCUAUGGGCCGCGUACG | 19 | 17979 |
| HSV1-UL48-816 | - | GAUCCUAUGGGCCGCGUACG | 20 | 6570 |
| HSV1-UL48-2571 | - | AGAUCCUAUGGGCCGCGUACG | 21 | 17980 |
| HSV1-UL48-2572 | - | GAGAUCCUAUGGGCCGCGUACG | 22 | 17981 |
| HSV1-UL48-2573 | - | CGAGAUCCUAUGGGCCGCGUACG | 23 | 17982 |
| HSV1-UL48-2574 | - | GCGAGAUCCUAUGGGCCGCGUACG | 24 | 17983 |
| HSV1-UL48-2575 | - | CGGACGACGACGCCCCCG | 18 | 17984 |
| HSV1-UL48-2576 | - | CCGGACGACGACGCCCCCG | 19 | 17985 |
| HSV1-UL48-852 | - | CCCGGACGACGACGCCCCCG | 20 | 6594 |
| HSV1-UL48-2577 | - | UCCCGGACGACGACGCCCCCG | 21 | 17986 |
| HSV1-UL48-2578 | - | CUCCCGGACGACGACGCCCCCG | 22 | 17987 |
| HSV1-UL48-2579 | - | UCUCCCGGACGACGACGCCCCCG | 23 | 17988 |
| HSV1-UL48-2580 | - | AUCUCCCGGACGACGACGCCCCCG | 24 | 17989 |
| HSV1-UL48-2581 | - | GCCUGUCCUUUCUCCCCG | 18 | 17990 |
| HSV1-UL48-2582 | - | CGCCUGUCCUUUCUCCCCG | 19 | 17991 |
| HSV1-UL48-292 | - | GCGCCUGUCCUUUCUCCCCG | 20 | 6130 |
| HSV1-UL48-2583 | - | CGCGCCUGUCCUUUCUCCCCG | 21 | 17992 |
| HSV1-UL48-2584 | - | CCGCGCCUGUCCUUUCUCCCCG | 22 | 17993 |
| HSV1-UL48-2585 | - | UCCGCGCCUGUCCUUUCUCCCCG | 23 | 17994 |
| HSV1-UL48-2586 | - | CUCCGCGCCUGUCCUUUCUCCCCG | 24 | 17995 |
| HSV1-UL48-2587 | - | ACGACGCCCCCGAAGAGG | 18 | 17996 |
| HSV1-UL48-2588 | - | GACGACGCCCCCGAAGAGG | 19 | 17997 |
| HSV1-UL48-287 | - | CGACGACGCCCCCGAAGAGG | 20 | 6125 |
| HSV1-UL48-2589 | - | ACGACGACGCCCCCGAAGAGG | 21 | 17998 |
| HSV1-UL48-2590 | - | GACGACGACGCCCCCGAAGAGG | 22 | 17999 |
| HSV1-UL48-2591 | - | GGACGACGACGCCCCCGAAGAGG | 23 | 18000 |
| HSV1-UL48-2592 | - | CGGACGACGACGCCCCCGAAGAGG | 24 | 18001 |
| HSV1-UL48-2593 | - | UGCGCAGCGCGGCUACGG | 18 | 18002 |
| HSV1-UL48-2594 | - | GUGCGCAGCGCGGCUACGG | 19 | 18003 |
| HSV1-UL48-265 | - | GGUGCGCAGCGCGGCUACGG | 20 | 6103 |
| HSV1-UL48-2595 | - | UGGUGCGCAGCGCGGCUACGG | 21 | 18004 |
| HSV1-UL48-2596 | - | CUGGUGCGCAGCGCGGCUACGG | 22 | 18005 |
| HSV1-UL48-2597 | - | GCUGGUGCGCAGCGCGGCUACGG | 23 | 18006 |
| HSV1-UL48-2598 | - | CGCUGGUGCGCAGCGCGGCUACGG | 24 | 18007 |
| HSV1-UL48-1764 | - | GCCGAGCUACGGGCGCGG | 18 | 17173 |
| HSV1-UL48-1765 | - | CGCCGAGCUACGGGCGCGG | 19 | 17174 |
| HSV1-UL48-640 | - | ACGCCGAGCUACGGGCGCGG | 20 | 6465 |
| HSV1-UL48-2599 | - | UUUAUGGUGUUGAUUCGG | 18 | 18008 |
| HSV1-UL48-2600 | - | CUUUAUGGUGUUGAUUCGG | 19 | 18009 |
| HSV1-UL48-840 | - | ACUUUAUGGUGUUGAUUCGG | 20 | 6584 |
| HSV1-UL48-2601 | - | UACUUUAUGGUGUUGAUUCGG | 21 | 18010 |
| HSV1-UL48-2602 | - | GUACUUUAUGGUGUUGAUUCGG | 22 | 18011 |
| HSV1-UL48-2603 | - | GGUACUUUAUGGUGUUGAUUCGG | 23 | 18012 |
| HSV1-UL48-2604 | - | GGGUACUUUAUGGUGUUGAUUCGG | 24 | 18013 |
| HSV1-UL48-1770 | - | CCGAGCUACGGGCGCGGG | 18 | 17179 |
| HSV1-UL48-1771 | - | GCCGAGCUACGGGCGCGGG | 19 | 17180 |
| HSV1-UL48-40 | - | CGCCGAGCUACGGGCGCGGG | 20 | 5973 |
| HSV1-UL48-2605 | - | GACGGGGAUUCCCCGGGG | 18 | 18014 |
| HSV1-UL48-2606 | - | GGACGGGGAUUCCCCGGGG | 19 | 18015 |
| HSV1-UL48-874 | - | GGGACGGGGAUUCCCCGGGG | 20 | 6605 |
| HSV1-UL48-2607 | - | GGGGACGGGGAUUCCCCGGGG | 21 | 18016 |
| HSV1-UL48-2608 | - | GGGGGACGGGGAUUCCCCGGGG | 22 | 18017 |
| HSV1-UL48-2609 | - | UGGGGGACGGGGAUUCCCCGGGG | 23 | 18018 |
| HSV1-UL48-2610 | - | UUGGGGGACGGGGAUUCCCCGGGG | 24 | 18019 |
| HSV1-UL48-2611 | - | CCGAUGUCAGCCUGGGGG | 18 | 18020 |
| HSV1-UL48-2612 | - | ACCGAUGUCAGCCUGGGGG | 19 | 18021 |
| HSV1-UL48-861 | - | GACCGAUGUCAGCCUGGGGG | 20 | 6598 |
| HSV1-UL48-2613 | - | CGACCGAUGUCAGCCUGGGGG | 21 | 18022 |
| HSV1-UL48-2614 | - | CCGACCGAUGUCAGCCUGGGGG | 22 | 18023 |
| HSV1-UL48-2615 | - | CCCGACCGAUGUCAGCCUGGGGG | 23 | 18024 |
| HSV1-UL48-2616 | - | CCCCGACCGAUGUCAGCCUGGGGG | 24 | 18025 |
| HSV1-UL48-2617 | - | AUCUGGACAUGUUGGGGG | 18 | 18026 |
| HSV1-UL48-2618 | - | GAUCUGGACAUGUUGGGGG | 19 | 18027 |
| HSV1-UL48-869 | - | CGAUCUGGACAUGUUGGGGG | 20 | 6603 |
| HSV1-UL48-2619 | - | UCGAUCUGGACAUGUUGGGGG | 21 | 18028 |
| HSV1-UL48-2620 | - | UUCGAUCUGGACAUGUUGGGGG | 22 | 18029 |
| HSV1-UL48-2621 | - | UUUCGAUCUGGACAUGUUGGGGG | 23 | 18030 |
| HSV1-UL48-2622 | - | AUUUCGAUCUGGACAUGUUGGGGG | 24 | 18031 |
| HSV1-UL48-2623 | - | AUUGACGAGUACGGUGGG | 18 | 18032 |
| HSV1-UL48-2624 | - | AAUUGACGAGUACGGUGGG | 19 | 18033 |
| HSV1-UL48-2625 | - | GAAUUGACGAGUACGGUGGG | 20 | 18034 |
| HSV1-UL48-2626 | - | GGAAUUGACGAGUACGGUGGG | 21 | 18035 |
| HSV1-UL48-2627 | - | UGGAAUUGACGAGUACGGUGGG | 22 | 18036 |
| HSV1-UL48-2628 | - | UUGGAAUUGACGAGUACGGUGGG | 23 | 18037 |
| HSV1-UL48-2629 | - | CUUGGAAUUGACGAGUACGGUGGG | 24 | 18038 |
| HSV1-UL48-2630 | - | GACGCGAUCGCGACCUGG | 18 | 18039 |
| HSV1-UL48-2631 | - | GGACGCGAUCGCGACCUGG | 19 | 18040 |
| HSV1-UL48-810 | - | CGGACGCGAUCGCGACCUGG | 20 | 6564 |
| HSV1-UL48-2632 | - | GCGGACGCGAUCGCGACCUGG | 21 | 18041 |
| HSV1-UL48-2633 | - | CGCGGACGCGAUCGCGACCUGG | 22 | 18042 |
| HSV1-UL48-2634 | - | GCGCGGACGCGAUCGCGACCUGG | 23 | 18043 |
| HSV1-UL48-2635 | - | UGCGCGGACGCGAUCGCGACCUGG | 24 | 18044 |
| HSV1-UL48-2636 | - | GAUUUCGAUCUGGACAUG | 18 | 18045 |
| HSV1-UL48-2637 | - | CGAUUUCGAUCUGGACAUG | 19 | 18046 |
| HSV1-UL48-865 | - | ACGAUUUCGAUCUGGACAUG | 20 | 6602 |
| HSV1-UL48-2638 | - | GACGAUUUCGAUCUGGACAUG | 21 | 18047 |
| HSV1-UL48-2639 | - | AGACGAUUUCGAUCUGGACAUG | 22 | 18048 |
| HSV1-UL48-2640 | - | UAGACGAUUUCGAUCUGGACAUG | 23 | 18049 |
| HSV1-UL48-2641 | - | CUAGACGAUUUCGAUCUGGACAUG | 24 | 18050 |
| HSV1-UL48-2642 | - | CCCUCCGAGGCGGUCAUG | 18 | 18051 |
| HSV1-UL48-2643 | - | GCCCUCCGAGGCGGUCAUG | 19 | 18052 |
| HSV1-UL48-279 | - | CGCCCUCCGAGGCGGUCAUG | 20 | 6117 |
| HSV1-UL48-2644 | - | UCGCCCUCCGAGGCGGUCAUG | 21 | 18053 |
| HSV1-UL48-2645 | - | CUCGCCCUCCGAGGCGGUCAUG | 22 | 18054 |
| HSV1-UL48-2646 | - | CCUCGCCCUCCGAGGCGGUCAUG | 23 | 18055 |
| HSV1-UL48-2647 | - | ACCUCGCCCUCCGAGGCGGUCAUG | 24 | 18056 |
| HSV1-UL48-2648 | - | UGCCUCUGUUGCGACCUG | 18 | 18057 |
| HSV1-UL48-2649 | - | CUGCCUCUGUUGCGACCUG | 19 | 18058 |
| HSV1-UL48-819 | - | ACUGCCUCUGUUGCGACCUG | 20 | 6572 |
| HSV1-UL48-2650 | - | GACUGCCUCUGUUGCGACCUG | 21 | 18059 |
| HSV1-UL48-2651 | - | UGACUGCCUCUGUUGCGACCUG | 22 | 18060 |
| HSV1-UL48-2652 | - | UUGACUGCCUCUGUUGCGACCUG | 23 | 18061 |
| HSV1-UL48-2653 | - | UUUGACUGCCUCUGUUGCGACCUG | 24 | 18062 |
| HSV1-UL48-2654 | - | CCGACCGAUGUCAGCCUG | 18 | 18063 |
| HSV1-UL48-2655 | - | CCCGACCGAUGUCAGCCUG | 19 | 18064 |
| HSV1-UL48-297 | - | CCCCGACCGAUGUCAGCCUG | 20 | 6135 |
| HSV1-UL48-2656 | - | CCCCCGACCGAUGUCAGCCUG | 21 | 18065 |
| HSV1-UL48-2657 | - | CCCCCCGACCGAUGUCAGCCUG | 22 | 18066 |
| HSV1-UL48-2658 | - | CCCCCCCGACCGAUGUCAGCCUG | 23 | 18067 |
| HSV1-UL48-2659 | - | GCCCCCCCGACCGAUGUCAGCCUG | 24 | 18068 |
| HSV1-UL48-2660 | - | AUCGAGGCCCGCCGGCUG | 18 | 18069 |
| HSV1-UL48-2661 | - | AAUCGAGGCCCGCCGGCUG | 19 | 18070 |
| HSV1-UL48-260 | - | CAAUCGAGGCCCGCCGGCUG | 20 | 6098 |
| HSV1-UL48-2662 | - | CCAAUCGAGGCCCGCCGGCUG | 21 | 18071 |
| HSV1-UL48-2663 | - | GCCAAUCGAGGCCCGCCGGCUG | 22 | 18072 |
| HSV1-UL48-2664 | - | UGCCAAUCGAGGCCCGCCGGCUG | 23 | 18073 |
| HSV1-UL48-2665 | - | GUGCCAAUCGAGGCCCGCCGGCUG | 24 | 18074 |
| HSV1-UL48-2666 | - | UUCGAUCUGGACAUGUUG | 18 | 18075 |
| HSV1-UL48-2667 | - | UUUCGAUCUGGACAUGUUG | 19 | 18076 |
| HSV1-UL48-306 | - | AUUUCGAUCUGGACAUGUUG | 20 | 6144 |
| HSV1-UL48-2668 | - | GAUUUCGAUCUGGACAUGUUG | 21 | 18077 |
| HSV1-UL48-2669 | - | CGAUUUCGAUCUGGACAUGUUG | 22 | 18078 |
| HSV1-UL48-2670 | - | ACGAUUUCGAUCUGGACAUGUUG | 23 | 18079 |
| HSV1-UL48-2671 | - | GACGAUUUCGAUCUGGACAUGUUG | 24 | 18080 |
| HSV1-UL48-2672 | - | GCCCUCCGAGGCGGUCAU | 18 | 18081 |
| HSV1-UL48-2673 | - | CGCCCUCCGAGGCGGUCAU | 19 | 18082 |
| HSV1-UL48-843 | - | UCGCCCUCCGAGGCGGUCAU | 20 | 6587 |
| HSV1-UL48-2674 | - | CUCGCCCUCCGAGGCGGUCAU | 21 | 18083 |
| HSV1-UL48-2675 | - | CCUCGCCCUCCGAGGCGGUCAU | 22 | 18084 |
| HSV1-UL48-2676 | - | ACCUCGCCCUCCGAGGCGGUCAU | 23 | 18085 |
| HSV1-UL48-2677 | - | GACCUCGCCCUCCGAGGCGGUCAU | 24 | 18086 |
| HSV1-UL48-2678 | - | GCGCUAGACGAUUUCGAU | 18 | 18087 |
| HSV1-UL48-2679 | - | CGCGCUAGACGAUUUCGAU | 19 | 18088 |
| HSV1-UL48-864 | - | ACGCGCUAGACGAUUUCGAU | 20 | 6601 |
| HSV1-UL48-2680 | - | GACGCGCUAGACGAUUUCGAU | 21 | 18089 |
| HSV1-UL48-2681 | - | CGACGCGCUAGACGAUUUCGAU | 22 | 18090 |
| HSV1-UL48-2682 | - | CCGACGCGCUAGACGAUUUCGAU | 23 | 18091 |
| HSV1-UL48-2683 | - | GCCGACGCGCUAGACGAUUUCGAU | 24 | 18092 |
| HSV1-UL48-2684 | - | GUACUUUAUGGUGUUGAU | 18 | 18093 |
| HSV1-UL48-2685 | - | GGUACUUUAUGGUGUUGAU | 19 | 18094 |
| HSV1-UL48-839 | - | GGGUACUUUAUGGUGUUGAU | 20 | 6583 |
| HSV1-UL48-2686 | - | UGGGUACUUUAUGGUGUUGAU | 21 | 18095 |
| HSV1-UL48-2687 | - | CUGGGUACUUUAUGGUGUUGAU | 22 | 18096 |
| HSV1-UL48-2688 | - | UCUGGGUACUUUAUGGUGUUGAU | 23 | 18097 |
| HSV1-UL48-2689 | - | CUCUGGGUACUUUAUGGUGUUGAU | 24 | 18098 |
| HSV1-UL48-2690 | - | CGGACGCGAUCGCGACCU | 18 | 18099 |
| HSV1-UL48-2691 | - | GCGGACGCGAUCGCGACCU | 19 | 18100 |
| HSV1-UL48-242 | - | CGCGGACGCGAUCGCGACCU | 20 | 6080 |
| HSV1-UL48-2692 | - | GCGCGGACGCGAUCGCGACCU | 21 | 18101 |
| HSV1-UL48-2693 | - | UGCGCGGACGCGAUCGCGACCU | 22 | 18102 |
| HSV1-UL48-2694 | - | AUGCGCGGACGCGAUCGCGACCU | 23 | 18103 |
| HSV1-UL48-2695 | - | CAUGCGCGGACGCGAUCGCGACCU | 24 | 18104 |
| HSV1-UL48-2696 | - | GCUUCACGACCUCGCCCU | 18 | 18105 |
| HSV1-UL48-2697 | - | AGCUUCACGACCUCGCCCU | 19 | 18106 |
| HSV1-UL48-842 | - | CAGCUUCACGACCUCGCCCU | 20 | 6586 |
| HSV1-UL48-2698 | - | CCAGCUUCACGACCUCGCCCU | 21 | 18107 |
| HSV1-UL48-2699 | - | UCCAGCUUCACGACCUCGCCCU | 22 | 18108 |
| HSV1-UL48-2700 | - | UUCCAGCUUCACGACCUCGCCCU | 23 | 18109 |
| HSV1-UL48-2701 | - | AUUCCAGCUUCACGACCUCGCCCU | 24 | 18110 |
| HSV1-UL48-2702 | - | CCCGACCGAUGUCAGCCU | 18 | 18111 |
| HSV1-UL48-2703 | - | CCCCGACCGAUGUCAGCCU | 19 | 18112 |
| HSV1-UL48-296 | - | CCCCCGACCGAUGUCAGCCU | 20 | 6134 |
| HSV1-UL48-2704 | - | CCCCCCGACCGAUGUCAGCCU | 21 | 18113 |
| HSV1-UL48-2705 | - | CCCCCCCGACCGAUGUCAGCCU | 22 | 18114 |
| HSV1-UL48-2706 | - | GCCCCCCCGACCGAUGUCAGCCU | 23 | 18115 |
| HSV1-UL48-2707 | - | GGCCCCCCCGACCGAUGUCAGCCU | 24 | 18116 |
| HSV1-UL48-2708 | - | UUUGACCCGCGAGAUCCU | 18 | 18117 |
| HSV1-UL48-2709 | - | UUUUGACCCGCGAGAUCCU | 19 | 18118 |
| HSV1-UL48-815 | - | UUUUUGACCCGCGAGAUCCU | 20 | 6569 |
| HSV1-UL48-2710 | - | AUUUUUGACCCGCGAGAUCCU | 21 | 18119 |
| HSV1-UL48-2711 | - | UAUUUUUGACCCGCGAGAUCCU | 22 | 18120 |
| HSV1-UL48-2712 | - | CUAUUUUUGACCCGCGAGAUCCU | 23 | 18121 |
| HSV1-UL48-2713 | - | UCUAUUUUUGACCCGCGAGAUCCU | 24 | 18122 |
| HSV1-UL48-2714 | - | AAUCGAGGCCCGCCGGCU | 18 | 18123 |
| HSV1-UL48-2715 | - | CAAUCGAGGCCCGCCGGCU | 19 | 18124 |
| HSV1-UL48-828 | - | CCAAUCGAGGCCCGCCGGCU | 20 | 6577 |
| HSV1-UL48-2716 | - | GCCAAUCGAGGCCCGCCGGCU | 21 | 18125 |
| HSV1-UL48-2717 | - | UGCCAAUCGAGGCCCGCCGGCU | 22 | 18126 |
| HSV1-UL48-2718 | - | GUGCCAAUCGAGGCCCGCCGGCU | 23 | 18127 |
| HSV1-UL48-2719 | - | AGUGCCAAUCGAGGCCCGCCGGCU | 24 | 18128 |
| HSV1-UL48-2720 | - | CUGGUGCGCAGCGCGGCU | 18 | 18129 |
| HSV1-UL48-2721 | - | GCUGGUGCGCAGCGCGGCU | 19 | 18130 |
| HSV1-UL48-832 | - | CGCUGGUGCGCAGCGCGGCU | 20 | 6579 |
| HSV1-UL48-2722 | - | CCGCUGGUGCGCAGCGCGGCU | 21 | 18131 |
| HSV1-UL48-2723 | - | CCCGCUGGUGCGCAGCGCGGCU | 22 | 18132 |
| HSV1-UL48-2724 | - | UCCCGCUGGUGCGCAGCGCGGCU | 23 | 18133 |
| HSV1-UL48-2725 | - | CUCCCGCUGGUGCGCAGCGCGGCU | 24 | 18134 |
| HSV1-UL48-2726 | - | AUAUGGCCGACUUCGAGU | 18 | 18135 |
| HSV1-UL48-2727 | - | GAUAUGGCCGACUUCGAGU | 19 | 18136 |
| HSV1-UL48-878 | - | GGAUAUGGCCGACUUCGAGU | 20 | 6608 |
| HSV1-UL48-2728 | - | UGGAUAUGGCCGACUUCGAGU | 21 | 18137 |
| HSV1-UL48-2729 | - | CUGGAUAUGGCCGACUUCGAGU | 22 | 18138 |
| HSV1-UL48-2730 | - | UCUGGAUAUGGCCGACUUCGAGU | 23 | 18139 |
| HSV1-UL48-2731 | - | CUCUGGAUAUGGCCGACUUCGAGU | 24 | 18140 |
| HSV1-UL48-2732 | - | CAACGGAGCGCUCACCGU | 18 | 18141 |
| HSV1-UL48-2733 | - | UCAACGGAGCGCUCACCGU | 19 | 18142 |
| HSV1-UL48-823 | - | GUCAACGGAGCGCUCACCGU | 20 | 6575 |
| HSV1-UL48-2734 | - | CGUCAACGGAGCGCUCACCGU | 21 | 18143 |
| HSV1-UL48-2735 | - | UCGUCAACGGAGCGCUCACCGU | 22 | 18144 |
| HSV1-UL48-2736 | - | UUCGUCAACGGAGCGCUCACCGU | 23 | 18145 |
| HSV1-UL48-2737 | - | GUUCGUCAACGGAGCGCUCACCGU | 24 | 18146 |
| HSV1-UL48-2738 | - | GCGUACAGCCGCGCGCGU | 18 | 18147 |
| HSV1-UL48-2739 | - | CGCGUACAGCCGCGCGCGU | 19 | 18148 |
| HSV1-UL48-846 | - | ACGCGUACAGCCGCGCGCGU | 20 | 6588 |
| HSV1-UL48-2740 | - | CACGCGUACAGCCGCGCGCGU | 21 | 18149 |
| HSV1-UL48-2741 | - | ACACGCGUACAGCCGCGCGCGU | 22 | 18150 |
| HSV1-UL48-2742 | - | AACACGCGUACAGCCGCGCGCGU | 23 | 18151 |
| HSV1-UL48-2743 | - | GAACACGCGUACAGCCGCGCGCGU | 24 | 18152 |
| HSV1-UL48-2744 | - | UUGACGAGUACGGUGGGU | 18 | 18153 |
| HSV1-UL48-2745 | - | AUUGACGAGUACGGUGGGU | 19 | 18154 |
| HSV1-UL48-1157 | - | AAUUGACGAGUACGGUGGGU | 20 | 16566 |
| HSV1-UL48-2746 | - | GAAUUGACGAGUACGGUGGGU | 21 | 18155 |
| HSV1-UL48-2747 | - | GGAAUUGACGAGUACGGUGGGU | 22 | 18156 |
| HSV1-UL48-2748 | - | UGGAAUUGACGAGUACGGUGGGU | 23 | 18157 |
| HSV1-UL48-2749 | - | UUGGAAUUGACGAGUACGGUGGGU | 24 | 18158 |
| HSV1-UL48-2750 | - | AUUUCGAUCUGGACAUGU | 18 | 18159 |
| HSV1-UL48-2751 | - | GAUUUCGAUCUGGACAUGU | 19 | 18160 |
| HSV1-UL48-304 | - | CGAUUUCGAUCUGGACAUGU | 20 | 6142 |
| HSV1-UL48-2752 | - | ACGAUUUCGAUCUGGACAUGU | 21 | 18161 |
| HSV1-UL48-2753 | - | GACGAUUUCGAUCUGGACAUGU | 22 | 18162 |
| HSV1-UL48-2754 | - | AGACGAUUUCGAUCUGGACAUGU | 23 | 18163 |
| HSV1-UL48-2755 | - | UAGACGAUUUCGAUCUGGACAUGU | 24 | 18164 |
| HSV1-UL48-2756 | - | UUUCGAUCUGGACAUGUU | 18 | 18165 |
| HSV1-UL48-2757 | - | AUUUCGAUCUGGACAUGUU | 19 | 18166 |
| HSV1-UL48-305 | - | GAUUUCGAUCUGGACAUGUU | 20 | 6143 |
| HSV1-UL48-2758 | - | CGAUUUCGAUCUGGACAUGUU | 21 | 18167 |
| HSV1-UL48-2759 | - | ACGAUUUCGAUCUGGACAUGUU | 22 | 18168 |
| HSV1-UL48-2760 | - | GACGAUUUCGAUCUGGACAUGUU | 23 | 18169 |
| HSV1-UL48-2761 | - | AGACGAUUUCGAUCUGGACAUGUU | 24 | 18170 |

**Table 13A** provides exemplary targeting domains for knocking out the *UL48* gene selected according to the first tier parameters. The targeting domains bind within the first 500 bp of the coding sequence (e.g., within 500 bp downstream from the start codon), have a high level of orthogonality, and start with a 5'G. It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 13A**

| 1st Tier | | | | |
|---|---|---|---|---|
| gRNA Name | DNA Strand | Targeting Domain | Target Site Length | Seq ID NO: |
| HSV1-UL48-1115 | + | GUCGCCGUGGGCGCGAA | 17 | 6757 |
| HSV1-UL48-2762 | + | GC GUGGAAGAAAC GAGA | 17 | 18171 |
| HSV1-UL48-120 | - | GCCGACAUGAACGCGGA | 17 | 5975 |

**Table 13B** provides exemplary targeting domains for knocking out the *UL48* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 13B**

| 2nd Tier | | | | |
|---|---|---|---|---|
| gRNA Name | DNA Strand | Targeting Domain | Target Site Length | Seq ID NO: |
| HSV1-UL48-651 | - | ACCGUCUCCUCGACGAC | 17 | 6472 |
| HSV1-UL48-2763 | - | CGGCGACGUGGCCUUCC | 17 | 18172 |
| HSV1-UL48-2764 | + | UCGGCGUGGAAGAAACGAGA | 20 | 18173 |
| HSV1-UL48-2 | - | UUUGCCGACAUGAACGCGGA | 20 | 5961 |
| HSV1-UL48-2765 | - | CCACGGCGACGUGGCCUUCC | 20 | 18174 |

**Table 13C** provides exemplary targeting domains for knocking out the *UL48* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase).

**Table 13C**

| 5th Tier | | | | |
|---|---|---|---|---|
| gRNA Name | DNA Strand | Targeting Domain | Target Site Length | Seq ID NO: |
| HSV1-UL48-494 | - | CUGGACAUGUUGGGGGA | 17 | 6332 |
| HSV1-UL48-1125 | + | CUCCCGCAGCCGGCGGG | 17 | 6763 |
| HSV1-UL48-951 | - | ACGGGGAUUCCCCGGGG | 17 | 6655 |
| HSV1-UL48-1120 | - | CUCUGGGUACUUUAUGG | 17 | 6760 |
| HSV1-UL48-2766 | - | CGAAGUUGGACUCGUAU | 17 | 18175 |
| HSV1-UL48-1121 | - | GCGCAUGCCGACGCGCU | 17 | 6761 |
| HSV1-UL48-308 | - | GAUCUGGACAUGUUGGGGGA | 20 | 6146 |
| HSV1-UL48-1124 | + | UAGCUCCCGCAGCCGGCGGG | 20 | 6762 |
| HSV1-UL48-874 | - | GGGACGGGGAUUCCCCGGGG | 20 | 6605 |
| HSV1-UL48-1116 | - | CGCCUCUGGGUACUUUAUGG | 20 | 6758 |
| HSV1-UL48-2767 | - | GGGC GAAGUUGGACUC GUAU | 20 | 18176 |

**Table 14A** provides exemplary targeting domains for knocking out the *UL54* gene selected according to the first tier parameters. The targeting domains bind within the first 500 bp of the coding sequence (e.g., within 500 bp downstream from the start codon), have a high level of orthogonality and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 14A**

| 1st Tier | | | | |
|---|---|---|---|---|
| gRNA Name | DNA Strand | Targeting Domain | Target Site Length | Seq ID NO: |
| HSV1-UL54-63 | + | GGAGCAAGACGGUCGCC | 17 | 6826 |
| HSV1-UL54-168 | - | GCUAAUUGACCUCGGCC | 17 | 6931 |
| HSV1-UL54-56 | - | GUUUGUCGGACCCCCGC | 17 | 6819 |
| HSV1-UL54-62 | + | GGGAGCAAGACGGUCGC | 17 | 6825 |
| HSV1-UL54-242 | + | GCGUCGAGUAUCGGCUC | 17 | 7005 |
| HSV1-UL54-236 | + | GGUACGCCGGGGUCUUC | 17 | 6999 |
| HSV1-UL54-175 | - | GUGUUCCUCGUCGGACG | 17 | 6938 |
| HSV1-UL54-59 | + | GCCCACGGCGUCCGCCG | 17 | 6822 |
| HSV1-UL54-64 | + | GGCGCGACCACACACUG | 17 | 6827 |
| HSV1-UL54-487 | - | GAACCAAUCGCAACCCU | 17 | 7250 |
| HSV1-UL54-65 | + | GCGCGACCACACACUGU | 17 | 6828 |
| HSV1-UL54-229 | + | GCUGGUUGAGGAUCGUU | 17 | 6992 |
| HSV1-UL54-118 | - | GGUGGUCCCGGGGCUGCCGA | 20 | 6881 |
| HSV1-UL54-117 | - | GUCGGGGUCGCGGUGGUCCC | 20 | 6880 |
| HSV1-UL54-6 | - | GAUACUCGACGCCGCUCGCC | 20 | 6769 |
| HSV1-UL54-23 | + | GGGGGAGCAAGACGGUCGCC | 20 | 6786 |
| HSV1-UL54-39 | + | GAUCGCUGUCGGAGAGGUCC | 20 | 6802 |
| HSV1-UL54-116 | - | GGUCGGGGUCGCGGUGGUCC | 20 | 6879 |
| HSV1-UL54-83 | - | GGACGAGGACCCCCCCGAGC | 20 | 6846 |
| HSV1-UL54-1188 | + | GAUUGGUUCUGGGGGCACGC | 20 | 18177 |
| HSV1-UL54-140 | + | GACCACACACUGUGGGGCGC | 20 | 6903 |
| HSV1-UL54-108 | - | GCGGACGCCGUGGGCGUCGC | 20 | 6871 |
| HSV1-UL54-123 | + | GGCGUCCGCCGCGGGCAGGC | 20 | 6886 |
| HSV1-UL54-1189 | + | GGUUCUGGGGGCACGCCGGC | 20 | 18178 |
| HSV1-UL54-9 | - | GCCUCGUCCGACGGAGCGGC | 20 | 6772 |
| HSV1-UL54-1 | - | GGACCUCUCCGACAGCGAUC | 20 | 6764 |
| HSV1-UL54-156 | + | GCGGCGUCGAGUAUCGGCUC | 20 | 6919 |
| HSV1-UL54-28 | + | GCUCCGUCGGACGAGGCGUC | 20 | 6791 |
| HSV1-UL54-110 | - | GGACGCCGUGGGCGUCGCAG | 20 | 6873 |
| HSV1-UL54-89 | - | GGAGUGUUCCUCGUCGGACG | 20 | 6852 |
| HSV1-UL54-19 | + | GACGCCCACGGCGUCCGCCG | 20 | 6782 |
| HSV1-UL54-1190 | + | GUUCUGGGGGCACGCCGGCG | 20 | 18179 |
| HSV1-UL54-30 | + | GGUCUUCUGGACGAGACGGG | 20 | 6793 |
| HSV1-UL54-144 | + | GCGCUGGUUGAGGAUCGUUG | 20 | 6907 |
| HSV1-UL54-17 | + | GCGGGGGUCCGACAAACCAU | 20 | 6780 |
| HSV1-UL54-31 | + | GGGCGAGCGGCGUCGAGUAU | 20 | 6794 |
| HSV1-UL54-124 | + | GCGUCCGCCGCGGGCAGGCU | 20 | 6887 |
| HSV1-UL54-162 | + | GCGGCGGCUCUCCGCCGGCU | 20 | 6925 |
| HSV1-UL54-146 | + | GGACGAGGCGUCUGGGUGCU | 20 | 6909 |
| HSV1-UL54-111 | - | GCCGUGGGCGUCGCAGGGGU | 20 | 6874 |
| HSV1-UL54-25 | + | GAGGCGCGACCACACACUGU | 20 | 6788 |
| HSV1-UL54-37 | + | GUCCUCGUCCAGAUCGCUGU | 20 | 6800 |
| HSV1-UL54-143 | + | GGC GCUGGUUGAGGAUC GUU | 20 | 6906 |

**Table 14B** provides exemplary targeting domains for knocking out the *UL54* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 14B**

| 2nd Tier | | | | |
|---|---|---|---|---|
| gRNA Name | DNA Strand | Targeting Domain | Target Site Length | Seq ID NO: |
| HSV1-UL54-45 | - | CUCGUCGGACGAGGACA | 17 | 6808 |
| HSV1-UL54-1191 | - | AGCCGACAGCCCGGUCA | 17 | 18180 |
| HSV1-UL54-46 | - | ACUCGACGCCGCUCGCC | 17 | 6809 |
| HSV1-UL54-79 | + | CGCUGUCGGAGAGGUCC | 17 | 6842 |
| HSV1-UL54-169 | - | CGAGGACCCCCCCGAGC | 17 | 6932 |
| HSV1-UL54-247 | + | UCGCGGCGGCUCUCCGC | 17 | 7010 |
| HSV1-UL54-1192 | + | UCUGGGGGCACGCCGGC | 17 | 18181 |
| HSV1-UL54-49 | - | UCGUCCGACGGAGCGGC | 17 | 6812 |
| HSV1-UL54-41 | - | CCUCUCCGACAGCGAUC | 17 | 6804 |
| HSV1-UL54-68 | + | CCGUCGGACGAGGCGUC | 17 | 6831 |
| HSV1-UL54-1185 | + | AGGGUUGCGAUUGGUUC | 17 | 7805 |
| HSV1-UL54-196 | - | CGCCGUGGGCGUCGCAG | 17 | 6959 |
| HSV1-UL54-181 | - | CGCCUCGUCCGACGGAG | 17 | 6944 |
| HSV1-UL54-78 | + | CCAGAUCGCUGUCGGAG | 17 | 6841 |
| HSV1-UL54-73 | + | CAUGUCCUCGUCCGACG | 17 | 6836 |
| HSV1-UL54-67 | + | UGCCGCUCCGUCGGACG | 17 | 6830 |
| HSV1-UL54-42 | - | CGACAGCGAUCUGGACG | 17 | 6805 |
| HSV1-UL54-243 | + | CCGGUCCGUCCUCUCCG | 17 | 7006 |
| HSV1-UL54-51 | - | UGUGUGGUCGCGCCUCG | 17 | 6814 |
| HSV1-UL54-74 | + | CCGAUUCCAGGUCGUCG | 17 | 6837 |
| HSV1-UL54-75 | + | AUUCCAGGUCGUCGCGG | 17 | 6838 |
| HSV1-UL54-70 | + | CUUCUGGACGAGACGGG | 17 | 6833 |
| HSV1-UL54-76 | + | CUCUCCGCCGGCUCGGG | 17 | 6839 |
| HSV1-UL54-43 | - | CCGCGACGACCUGGAAU | 17 | 6806 |
| HSV1-UL54-71 | + | CGAGCGGCGUCGAGUAU | 17 | 6834 |
| HSV1-UL54-184 | - | AGUGUGUGGUCGCGCCU | 17 | 6947 |
| HSV1-UL54-69 | + | CGUCGGACGAGGCGUCU | 17 | 6832 |
| HSV1-UL54-193 | - | CCCGCGGCGGACGCCGU | 17 | 6956 |
| HSV1-UL54-244 | + | CGGUCCGUCCUCUCCGU | 17 | 7007 |
| HSV1-UL54-228 | + | CGCUGGUUGAGGAUCGU | 17 | 6991 |
| HSV1-UL54-44 | - | CGGGGAGUGUUCCUCGU | 17 | 6807 |
| HSV1-UL54-77 | + | CUCGUCCAGAUCGCUGU | 17 | 6840 |
| HSV1-UL54-5 | - | UUCCUCGUCGGACGAGGACA | 20 | 6768 |
| HSV1-UL54-99 | - | UCUUGCUCCCCCGAGCAGCA | 20 | 6862 |
| HSV1-UL54-96 | - | CCUCGUCCGACGGAGCGGCA | 20 | 6859 |
| HSV1-UL54-151 | + | CCGGGGUCUUCUGGACGAGA | 20 | 6914 |
| HSV1-UL54-152 | + | CGGGGUCUUCUGGACGAGAC | 20 | 6915 |
| HSV1-UL54-1193 | + | UCAAUGUCAGUCGCCAUGAC | 20 | 18182 |
| HSV1-UL54-1194 | + | CAAUGUCAGUCGCCAUGACC | 20 | 18183 |
| HSV1-UL54-1195 | - | CCCAGAACCAAUCGCAACCC | 20 | 18184 |
| HSV1-UL54-18 | + | CGACAAACCAUCGGCAGCCC | 20 | 6781 |
| HSV1-UL54-1196 | - | CCCGACGGCAGCCGACAGCC | 20 | 18185 |
| HSV1-UL54-12 | - | UGUGGUCGCGCCUCGGGGCC | 20 | 6775 |
| HSV1-UL54-87 | - | ACCUGGAAUCGGACAGCAGC | 20 | 6850 |
| HSV1-UL54-16 | - | AUGGUUUGUCGGACCCCCGC | 20 | 6779 |
| HSV1-UL54-161 | + | UCGUCGCGGCGGCUCUCCGC | 20 | 6924 |
| HSV1-UL54-22 | + | CGGGGGAGCAAGACGGUCGC | 20 | 6785 |
| HSV1-UL54-1197 | + | CUGUCGGCUGCCGUCGGGGC | 20 | 18186 |
| HSV1-UL54-145 | + | CGGACGAGGCGUCUGGGUGC | 20 | 6908 |
| HSV1-UL54-10 | - | ACAGUGUGUGGUCGCGCCUC | 20 | 6773 |
| HSV1-UL54-136 | + | ACCUUGCCCCCGUGCUGCUC | 20 | 6899 |
| HSV1-UL54-1198 | + | CCGGGCUGUCGGCUGCCGUC | 20 | 18187 |
| HSV1-UL54-1199 | + | CCCAGGGUUGCGAUUGGUUC | 20 | 18188 |
| HSV1-UL54-155 | + | CGGGCGGACCGCCGGGCGAG | 20 | 6918 |
| HSV1-UL54-95 | - | AGACGCCUCGUCCGACGGAG | 20 | 6858 |
| HSV1-UL54-13 | - | UUGCUCCCCCGAGCAGCACG | 20 | 6776 |
| HSV1-UL54-33 | + | UUCCAUGUCCUCGUCCGACG | 20 | 6796 |
| HSV1-UL54-27 | + | CCCUGCCGCUCCGUCGGACG | 20 | 6790 |
| HSV1-UL54-2 | - | CUCCGACAGCGAUCUGGACG | 20 | 6765 |
| HSV1-UL54-138 | + | AAGACGGUCGCCGGGCCCCG | 20 | 6901 |
| HSV1-UL54-14 | - | UCGGGGUCGCGGUGGUCCCG | 20 | 6777 |
| HSV1-UL54-149 | + | UCUGGGUGCUGGGUACGCCG | 20 | 6912 |
| HSV1-UL54-40 | + | UGUCGGAGAGGUCCAGGCCG | 20 | 6803 |
| HSV1-UL54-88 | - | CCUGGAAUCGGACAGCAGCG | 20 | 6851 |
| HSV1-UL54-11 | - | CAGUGUGUGGUCGCGCCUCG | 20 | 6774 |
| HSV1-UL54-20 | + | CCUUGCCCCCGUGCUGCUCG | 20 | 6783 |
| HSV1-UL54-1200 | + | CGGGCUGUCGGCUGCCGUCG | 20 | 18189 |
| HSV1-UL54-34 | + | UGUCCGAUUCCAGGUCGUCG | 20 | 6797 |
| HSV1-UL54-103 | - | CGAGCAGCACGGGGGCAAGG | 20 | 6866 |
| HSV1-UL54-101 | - | UGCUCCCCCGAGCAGCACGG | 20 | 6864 |
| HSV1-UL54-7 | - | ACUCGACGCCGCUCGCCCGG | 20 | 6770 |
| HSV1-UL54-84 | - | CGAGGACCCCCCCGAGCCGG | 20 | 6847 |
| HSV1-UL54-1201 | + | UGGUUCUGGGGGCACGCCGG | 20 | 18190 |
| HSV1-UL54-105 | - | AAAGCCCAGCCUGCCCGCGG | 20 | 6868 |
| HSV1-UL54-35 | + | CCGAUUCCAGGUCGUCGCGG | 20 | 6798 |
| HSV1-UL54-1202 | + | UUCUGGGGGCACGCCGGCGG | 20 | 18191 |
| HSV1-UL54-21 | + | CUUGCCCCCGUGCUGCUCGG | 20 | 6784 |
| HSV1-UL54-36 | + | CGGCUCUCCGCCGGCUCGGG | 20 | 6799 |
| HSV1-UL54-1203 | + | AGGGUUGCGAUUGGUUCUGG | 20 | 18192 |
| HSV1-UL54-159 | + | CCGGUCCGUCCUCUCCGUGG | 20 | 6922 |
| HSV1-UL54-24 | + | CGAGGCGCGACCACACACUG | 20 | 6787 |
| HSV1-UL54-32 | + | UCCGGUCCGUCCUCUCCGUG | 20 | 6795 |
| HSV1-UL54-3 | - | CCGCCGCGACGACCUGGAAU | 20 | 6766 |
| HSV1-UL54-1204 | - | CCAGAACCAAUCGCAACCCU | 20 | 18193 |
| HSV1-UL54-98 | - | CACAGUGUGUGGUCGCGCCU | 20 | 6861 |
| HSV1-UL54-135 | + | CACCUUGCCCCCGUGCUGCU | 20 | 6898 |
| HSV1-UL54-29 | + | CUCCGUCGGACGAGGCGUCU | 20 | 6792 |
| HSV1-UL54-1205 | + | ACCGGGCUGUCGGCUGCCGU | 20 | 18194 |
| HSV1-UL54-26 | + | UUGGGGCCCUGCCGCUCCGU | 20 | 6789 |
| HSV1-UL54-158 | + | CUCCGGUCCGUCCUCUCCGU | 20 | 6921 |
| HSV1-UL54-4 | - | CAGCGGGGAGUGUUCCUCGU | 20 | 6767 |
| HSV1-UL54-1206 | + | AGUCGCCAUGACCGGGCUGU | 20 | 18195 |
| HSV1-UL54-15 | - | CGGGGCUGCCGAUGGUUUGU | 20 | 6778 |

**Table 14C** provides exemplary targeting domains for knocking out the *UL54* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 14C**

| 3rd Tier | | | | |
|---|---|---|---|---|
| gRNA Name | DNA Strand | Targeting Domain | Target Site Length | Seq ID NO: |
| HSV1-UL54-176 | - | GACAUGGAAGACCCCCA | 17 | 6939 |
| HSV1-UL54-206 | + | GACCCCUGCGACGCCCA | 17 | 6969 |
| HSV1-UL54-237 | + | GGGUCUUCUGGACGAGA | 17 | 7000 |
| HSV1-UL54-204 | - | GGUCCCGGGGCUGCCGA | 17 | 6967 |
| HSV1-UL54-178 | - | GACCCCCACGGAGAGGA | 17 | 6941 |
| HSV1-UL54-186 | - | GCUCCCCCGAGCAGCAC | 17 | 6949 |
| HSV1-UL54-238 | + | GGUCUUCUGGACGAGAC | 17 | 7001 |
| HSV1-UL54-171 | - | GAGCCGCCGCGACGACC | 17 | 6934 |
| HSV1-UL54-203 | - | GGGGUCGCGGUGGUCCC | 17 | 6966 |
| HSV1-UL54-1207 | - | GACGGCAGCCGACAGCC | 17 | 18196 |
| HSV1-UL54-240 | + | GAGACGGGCGGACCGCC | 17 | 7003 |
| HSV1-UL54-52 | - | GGUCGCGCCUCGGGGCC | 17 | 6815 |
| HSV1-UL54-194 | - | GACGCCGUGGGCGUCGC | 17 | 6957 |
| HSV1-UL54-209 | + | GUCCGCCGCGGGCAGGC | 17 | 6972 |
| HSV1-UL54-50 | - | GUGUGUGGUCGCGCCUC | 17 | 6813 |
| HSV1-UL54-1208 | + | GGCUGUCGGCUGCCGUC | 17 | 18197 |
| HSV1-UL54-241 | + | GCGGACCGCCGGGCGAG | 17 | 7004 |
| HSV1-UL54-177 | - | GGAAGACCCCCACGGAG | 17 | 6940 |
| HSV1-UL54-54 | - | GGGUCGCGGUGGUCCCG | 17 | 6817 |
| HSV1-UL54-192 | - | GCCCGCGGCGGACGCCG | 17 | 6955 |
| HSV1-UL54-235 | + | GGGUGCUGGGUACGCCG | 17 | 6998 |
| HSV1-UL54-174 | - | GGAAUCGGACAGCAGCG | 17 | 6937 |
| HSV1-UL54-250 | + | GGCUCUCCGCCGGCUCG | 17 | 7013 |
| HSV1-UL54-1209 | + | GCUGUCGGCUGCCGUCG | 17 | 18198 |
| HSV1-UL54-199 | - | GGGCGUCGCAGGGGUCG | 17 | 6962 |
| HSV1-UL54-189 | - | GCAGCACGGGGGCAAGG | 17 | 6952 |
| HSV1-UL54-170 | - | GGACCCCCCCGAGCCGG | 17 | 6933 |
| HSV1-UL54-191 | - | GCCCAGCCUGCCCGCGG | 17 | 6954 |
| HSV1-UL54-251 | + | GCUCUCCGCCGGCUCGG | 17 | 7014 |
| HSV1-UL54-61 | + | GCCCCCGUGCUGCUCGG | 17 | 6824 |
| HSV1-UL54-213 | + | GGCUGGGCUUUGGUCGG | 17 | 6976 |
| HSV1-UL54-1210 | + | GUUGCGAUUGGUUCUGG | 17 | 18199 |
| HSV1-UL54-245 | + | GUCCGUCCUCUCCGUGG | 17 | 7008 |
| HSV1-UL54-1187 | + | GGUUGCGAUUGGUUCUG | 17 | 7807 |
| HSV1-UL54-72 | + | GGUCCGUCCUCUCCGUG | 17 | 6835 |
| HSV1-UL54-57 | + | GGGGUCCGACAAACCAU | 17 | 6820 |
| HSV1-UL54-167 | - | GAUAUGCUAAUUGACCU | 17 | 6930 |
| HSV1-UL54-248 | + | GCGGCUCUCCGCCGGCU | 17 | 7011 |
| HSV1-UL54-1186 | + | GGGUUGCGAUUGGUUCU | 17 | 7806 |
| HSV1-UL54-1211 | + | GGGCUGUCGGCUGCCGU | 17 | 18200 |
| HSV1-UL54-66 | + | GGGCCCUGCCGCUCCGU | 17 | 6829 |
| HSV1-UL54-214 | + | GCUGGGCUUUGGUCGGU | 17 | 6977 |
| HSV1-UL54-197 | - | GUGGGCGUCGCAGGGGU | 17 | 6960 |
| HSV1-UL54-212 | + | GCAGGCUGGGCUUUGGU | 17 | 6975 |
| HSV1-UL54-55 | - | GGCUGCCGAUGGUUUGU | 17 | 6818 |
| HSV1-UL54-211 | + | GCGGGCAGGCUGGGCUU | 17 | 6974 |
| HSV1-UL54-90 | - | GAGGACAUGGAAGACCCCCA | 20 | 6853 |
| HSV1-UL54-1212 | - | GGCAGCCGACAGCCCGGUCA | 20 | 18201 |
| HSV1-UL54-92 | - | GAAGACCCCCACGGAGAGGA | 20 | 6855 |
| HSV1-UL54-85 | - | GGAGAGCCGCCGCGACGACC | 20 | 6848 |
| HSV1-UL54-119 | + | GACAAACCAUCGGCAGCCCC | 20 | 6882 |
| HSV1-UL54-148 | + | GUCUGGGUGCUGGGUACGCC | 20 | 6911 |
| HSV1-UL54-154 | + | GACGAGACGGGCGGACCGCC | 20 | 6917 |
| HSV1-UL54-153 | + | GGACGAGACGGGCGGACCGC | 20 | 6916 |
| HSV1-UL54-134 | + | GGUCGGUGGGGGUUGGAGGC | 20 | 6897 |
| HSV1-UL54-86 | - | GACCUGGAAUCGGACAGCAG | 20 | 6849 |
| HSV1-UL54-157 | + | GCUCCGGUCCGUCCUCUCCG | 20 | 6920 |
| HSV1-UL54-164 | + | GGCGGCUCUCCGCCGGCUCG | 20 | 6927 |
| HSV1-UL54-165 | + | GCGGCUCUCCGCCGGCUCGG | 20 | 6928 |
| HSV1-UL54-127 | + | GCAGGCUGGGCUUUGGUCGG | 20 | 6890 |
| HSV1-UL54-166 | + | GGCUCUCCGCCGGCUCGGGG | 20 | 6929 |
| HSV1-UL54-130 | + | GGCUGGGCUUUGGUCGGUGG | 20 | 6893 |
| HSV1-UL54-485 | + | GGGUCCCCCAGGGUUGCGAU | 20 | 7248 |
| HSV1-UL54-142 | + | GGGCGCUGGUUGAGGAUCGU | 20 | 6905 |
| HSV1-UL54-131 | + | GGGCUUUGGUCGGUGGGGGU | 20 | 6894 |
| HSV1-UL54-125 | + | GCCGCGGGCAGGCUGGGCUU | 20 | 6888 |

**Table 14D** provides exemplary targeting domains for knocking out the *UL54* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 14D**

| 4th Tier | | | | |
|---|---|---|---|---|
| gRNA Name | DNA Strand | Targeting Domain | Target Site Length | Seq ID NO: |
| HSV1-UL54-185 | - | UGCUCCCCCGAGCAGCA | 17 | 6948 |
| HSV1-UL54-195 | - | ACGCCGUGGGCGUCGCA | 17 | 6958 |
| HSV1-UL54-182 | - | CGUCCGACGGAGCGGCA | 17 | 6945 |
| HSV1-UL54-188 | - | CGAGCAGCACGGGGGCA | 17 | 6951 |
| HSV1-UL54-223 | + | UGCUCGGGGGAGCAAGA | 17 | 6986 |
| HSV1-UL54-48 | - | CCAGACGCCUCGUCCGA | 17 | 6811 |
| HSV1-UL54-179 | - | CCACGGAGAGGACGGAC | 17 | 6942 |
| HSV1-UL54-1213 | + | AUGUCAGUCGCCAUGAC | 17 | 18202 |
| HSV1-UL54-1214 | + | UGUCAGUCGCCAUGACC | 17 | 18203 |
| HSV1-UL54-486 | - | AGAACCAAUCGCAACCC | 17 | 7249 |
| HSV1-UL54-180 | - | UCUCGUCCAGAAGACCC | 17 | 6943 |
| HSV1-UL54-205 | + | AAACCAUCGGCAGCCCC | 17 | 6968 |
| HSV1-UL54-58 | + | CAAACCAUCGGCAGCCC | 17 | 6821 |
| HSV1-UL54-234 | + | UGGGUGCUGGGUACGCC | 17 | 6997 |
| HSV1-UL54-202 | - | CGGGGUCGCGGUGGUCC | 17 | 6965 |
| HSV1-UL54-246 | + | CGCUGCUGUCCGAUUCC | 17 | 7009 |
| HSV1-UL54-173 | - | UGGAAUCGGACAGCAGC | 17 | 6936 |
| HSV1-UL54-1215 | + | UGGUUCUGGGGGCACGC | 17 | 18204 |
| HSV1-UL54-233 | + | CUGGGUGCUGGGUACGC | 17 | 6996 |
| HSV1-UL54-239 | + | CGAGACGGGCGGACCGC | 17 | 7002 |
| HSV1-UL54-207 | + | CCCACGGCGUCCGCCGC | 17 | 6970 |
| HSV1-UL54-226 | + | CACACACUGUGGGGCGC | 17 | 6989 |
| HSV1-UL54-220 | + | CGGUGGGGGUUGGAGGC | 17 | 6983 |
| HSV1-UL54-208 | + | CGGCGUCCGCCGCGGGC | 17 | 6971 |
| HSV1-UL54-1216 | + | UCGGCUGCCGUCGGGGC | 17 | 18205 |
| HSV1-UL54-231 | + | ACGAGGCGUCUGGGUGC | 17 | 6994 |
| HSV1-UL54-249 | + | CGGCUCUCCGCCGGCUC | 17 | 7012 |
| HSV1-UL54-222 | + | UUGCCCCCGUGCUGCUC | 17 | 6985 |
| HSV1-UL54-198 | - | UGGGCGUCGCAGGGGUC | 17 | 6961 |
| HSV1-UL54-172 | - | CUGGAAUCGGACAGCAG | 17 | 6935 |
| HSV1-UL54-53 | - | CUCCCCCGAGCAGCACG | 17 | 6816 |
| HSV1-UL54-224 | + | ACGGUCGCCGGGCCCCG | 17 | 6987 |
| HSV1-UL54-190 | - | AAAGCCCAGCCUGCCCG | 17 | 6953 |
| HSV1-UL54-80 | + | CGGAGAGGUCCAGGCCG | 17 | 6843 |
| HSV1-UL54-1217 | + | CUGGGGGCACGCCGGCG | 17 | 18206 |
| HSV1-UL54-60 | + | UGCCCCCGUGCUGCUCG | 17 | 6823 |
| HSV1-UL54-200 | - | CGCAGGGGUCGGGGUCG | 17 | 6963 |
| HSV1-UL54-219 | + | UCGGUGGGGGUUGGAGG | 17 | 6982 |
| HSV1-UL54-187 | - | UCCCCCGAGCAGCACGG | 17 | 6950 |
| HSV1-UL54-47 | - | CGACGCCGCUCGCCCGG | 17 | 6810 |
| HSV1-UL54-1218 | + | UUCUGGGGGCACGCCGG | 17 | 18207 |
| HSV1-UL54-201 | - | AGGGGUCGGGGUCGCGG | 17 | 6964 |
| HSV1-UL54-1219 | + | UGGGGGCACGCCGGCGG | 17 | 18208 |
| HSV1-UL54-252 | + | UCUCCGCCGGCUCGGGG | 17 | 7015 |
| HSV1-UL54-489 | - | ACCAAUCGCAACCCUGG | 17 | 7252 |
| HSV1-UL54-216 | + | UGGGCUUUGGUCGGUGG | 17 | 6979 |
| HSV1-UL54-218 | + | UGGUCGGUGGGGGUUGG | 17 | 6981 |
| HSV1-UL54-488 | - | AACCAAUCGCAACCCUG | 17 | 7251 |
| HSV1-UL54-215 | + | CUGGGCUUUGGUCGGUG | 17 | 6978 |
| HSV1-UL54-225 | + | CGCGACCACACACUGUG | 17 | 6988 |
| HSV1-UL54-183 | - | CAGCGCCCCACAGUGUG | 17 | 6946 |
| HSV1-UL54-230 | + | CUGGUUGAGGAUCGUUG | 17 | 6993 |
| HSV1-UL54-227 | + | CUGUGGGGCGCUGGUUG | 17 | 6990 |
| HSV1-UL54-721 | + | UCCCCCAGGGUUGCGAU | 17 | 7484 |
| HSV1-UL54-210 | + | UCCGCCGCGGGCAGGCU | 17 | 6973 |
| HSV1-UL54-221 | + | CUUGCCCCCGUGCUGCU | 17 | 6984 |
| HSV1-UL54-232 | + | CGAGGCGUCUGGGUGCU | 17 | 6995 |
| HSV1-UL54-217 | + | CUUUGGUCGGUGGGGGU | 17 | 6980 |
| HSV1-UL54-1220 | + | CGCCAUGACCGGGCUGU | 17 | 18209 |
| HSV1-UL54-120 | + | CCCGACCCCUGCGACGCCCA | 20 | 6883 |
| HSV1-UL54-109 | - | CGGACGCCGUGGGCGUCGCA | 20 | 6872 |
| HSV1-UL54-102 | - | CCCCGAGCAGCACGGGGGCA | 20 | 6865 |
| HSV1-UL54-137 | + | UGCUGCUCGGGGGAGCAAGA | 20 | 6900 |
| HSV1-UL54-8 | - | CACCCAGACGCCUCGUCCGA | 20 | 6771 |
| HSV1-UL54-100 | - | CUUGCUCCCCCGAGCAGCAC | 20 | 6863 |
| HSV1-UL54-93 | - | CCCCCACGGAGAGGACGGAC | 20 | 6856 |
| HSV1-UL54-94 | - | CCGUCUCGUCCAGAAGACCC | 20 | 6857 |
| HSV1-UL54-82 | - | UAUGCUAAUUGACCUCGGCC | 20 | 6845 |
| HSV1-UL54-160 | + | CCCCGCUGCUGUCCGAUUCC | 20 | 6923 |
| HSV1-UL54-147 | + | CGUCUGGGUGCUGGGUACGC | 20 | 6910 |
| HSV1-UL54-121 | + | ACGCCCACGGCGUCCGCCGC | 20 | 6884 |
| HSV1-UL54-122 | + | CCACGGCGUCCGCCGCGGGC | 20 | 6885 |
| HSV1-UL54-163 | + | CGGCGGCUCUCCGCCGGCUC | 20 | 6926 |
| HSV1-UL54-112 | - | CCGUGGGCGUCGCAGGGGUC | 20 | 6875 |
| HSV1-UL54-150 | + | CUGGGUACGCCGGGGUCUUC | 20 | 6913 |
| HSV1-UL54-91 | - | CAUGGAAGACCCCCACGGAG | 20 | 6854 |
| HSV1-UL54-38 | + | CGUCCAGAUCGCUGUCGGAG | 20 | 6801 |
| HSV1-UL54-104 | - | ACCAAAGCCCAGCCUGCCCG | 20 | 6867 |
| HSV1-UL54-106 | - | CCUGCCCGCGGCGGACGCCG | 20 | 6869 |
| HSV1-UL54-113 | - | CGUGGGCGUCGCAGGGGUCG | 20 | 6876 |
| HSV1-UL54-114 | - | CGUCGCAGGGGUCGGGGUCG | 20 | 6877 |
| HSV1-UL54-133 | + | UGGUCGGUGGGGGUUGGAGG | 20 | 6896 |
| HSV1-UL54-115 | - | CGCAGGGGUCGGGGUCGCGG | 20 | 6878 |
| HSV1-UL54-253 | - | AGAACCAAUCGCAACCCUGG | 20 | 7016 |
| HSV1-UL54-132 | + | CUUUGGUCGGUGGGGGUUGG | 20 | 6895 |
| HSV1-UL54-910 | - | CAGAACCAAUCGCAACCCUG | 20 | 7585 |
| HSV1-UL54-1045 | + | CAGGGUUGCGAUUGGUUCUG | 20 | 7697 |
| HSV1-UL54-129 | + | AGGCUGGGCUUUGGUCGGUG | 20 | 6892 |
| HSV1-UL54-139 | + | AGGCGCGACCACACACUGUG | 20 | 6902 |
| HSV1-UL54-97 | - | AACCAGCGCCCCACAGUGUG | 20 | 6860 |
| HSV1-UL54-141 | + | ACACUGUGGGGCGCUGGUUG | 20 | 6904 |
| HSV1-UL54-81 | - | AUUGAUAUGCUAAUUGACCU | 20 | 6844 |
| HSV1-UL54-1221 | + | CCAGGGUUGCGAUUGGUUCU | 20 | 18210 |
| HSV1-UL54-107 | - | CUGCCCGCGGCGGACGCCGU | 20 | 6870 |
| HSV1-UL54-128 | + | CAGGCUGGGCUUUGGUCGGU | 20 | 6891 |
| HSV1-UL54-126 | + | CGGGCAGGCUGGGCUUUGGU | 20 | 6889 |

**Table 14E** provides exemplary targeting domains for knocking out the *UL54* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a *S*. *pyogenes* Cas9 molecule that generates a double stranded break (Cas9 nuclease) or a single-stranded break (Cas9 nickase).

**Table 14E**

| 5th Tier | | | | |
|---|---|---|---|---|
| gRNA Name | DNA Strand | Targeting Domain | Target Site Length | Seq ID NO: |
| HSV1-UL54-658 | + | AACGGCUGCCCCCCAAA | 17 | 7421 |
| HSV1-UL54-645 | + | CUGGUCUCGGCGUCAAA | 17 | 7408 |
| HSV1-UL54-609 | + | AGGUAGCACUGGAGAAA | 17 | 7372 |
| HSV1-UL54-656 | + | CUAUUCGCGGCGGGAAA | 17 | 7419 |
| HSV1-UL54-599 | + | UCCGACCCCGACACCAA | 17 | 7362 |
| HSV1-UL54-657 | + | AAACGGCUGCCCCCCAA | 17 | 7420 |
| HSV1-UL54-644 | + | UCUGGUCUCGGCGUCAA | 17 | 7407 |
| HSV1-UL54-608 | + | CAGGUAGCACUGGAGAA | 17 | 7371 |
| HSV1-UL54-514 | - | CCCGGCCCCGGAGCGAA | 17 | 7277 |
| HSV1-UL54-614 | + | CGCGGUCGUCCCGAUAA | 17 | 7377 |
| HSV1-UL54-1222 | + | UUAUUGUACCUAAAACA | 17 | 18211 |
| HSV1-UL54-507 | - | CCGGAGGCCAGCGGACA | 17 | 7270 |
| HSV1-UL54-598 | + | CUCCGACCCCGACACCA | 17 | 7361 |
| HSV1-UL54-640 | + | CGGGCCGUGGGCGACCA | 17 | 7403 |
| HSV1-UL54-500 | - | GGCCCCGGCGCCCCCCA | 17 | 7263 |
| HSV1-UL54-720 | + | CGGGGCGGGGUCCCCCA | 17 | 7483 |
| HSV1-UL54-540 | - | GAAACCUUGGUCGCCCA | 17 | 7303 |
| HSV1-UL54-651 | + | GCCAGCACCGGGGCCCA | 17 | 7414 |
| HSV1-UL54-544 | - | GGCCGCAUCGACCGCCA | 17 | 7307 |
| HSV1-UL54-705 | + | CGCGCCACGCCUCGCCA | 17 | 7468 |
| HSV1-UL54-532 | - | CCGGUGCUGGCGGGCCA | 17 | 7295 |
| HSV1-UL54-629 | + | CAGCACGCAGUCGCGCA | 17 | 7392 |
| HSV1-UL54-576 | - | CCUCCCCGGGGCCUGCA | 17 | 7339 |
| HSV1-UL54-581 | - | GCCCCCCCGUACGUGCA | 17 | 7344 |
| HSV1-UL54-535 | - | UUGACGCCGAGACCAGA | 17 | 7298 |
| HSV1-UL54-663 | + | CGCCGCGCGCUCGGAGA | 17 | 7426 |
| HSV1-UL54-603 | + | CAGAAUGACAAACACGA | 17 | 7366 |
| HSV1-UL54-667 | + | CCGCGUGGUGGCGUCGA | 17 | 7430 |
| HSV1-UL54-534 | - | UGCUGGCGGGCCAAGGA | 17 | 7297 |
| HSV1-UL54-498 | - | GGGGCGGGGUGGACGGA | 17 | 7261 |
| HSV1-UL54-497 | - | CCCCGGGGCGGGGUGGA | 17 | 7260 |
| HSV1-UL54-555 | - | UCUCCAGUGCUACCUGA | 17 | 7318 |
| HSV1-UL54-580 | - | UCGUGUCUGCGAGUUGA | 17 | 7343 |
| HSV1-UL54-582 | + | UAUUUGCCGUGCACGUA | 17 | 7345 |
| HSV1-UL54-563 | - | GC GUCUGGC GGACAUUA | 17 | 7326 |
| HSV1-UL54-1223 | + | UUUAUUGUACCUAAAAC | 17 | 18212 |
| HSV1-UL54-508 | - | CGGAGGCCAGCGGACAC | 17 | 7271 |
| HSV1-UL54-647 | + | CCUUGGCCCGCCAGCAC | 17 | 7410 |
| HSV1-UL54-607 | + | GCGCCUUCAGGUAGCAC | 17 | 7370 |
| HSV1-UL54-605 | + | GUUCGUCCAGGCCGCAC | 17 | 7368 |
| HSV1-UL54-520 | - | CUUUGGCCGCAGCGCAC | 17 | 7283 |
| HSV1-UL54-536 | - | UGACGCCGAGACCAGAC | 17 | 7299 |
| HSV1-UL54-588 | + | GGGGGCGACGAUGUGAC | 17 | 7351 |
| HSV1-UL54-583 | + | AUUUGCCGUGCACGUAC | 17 | 7346 |
| HSV1-UL54-504 | - | GAGCAGCCCGACCCACC | 17 | 7267 |
| HSV1-UL54-648 | + | CUUGGCCCGCCAGCACC | 17 | 7411 |
| HSV1-UL54-486 | - | AGAACCAAUCGCAACCC | 17 | 7249 |
| HSV1-UL54-549 | - | CCUGCCGCUGCGCCCCC | 17 | 7312 |
| HSV1-UL54-719 | + | CCGGGGCGGGGUCCCCC | 17 | 7482 |
| HSV1-UL54-526 | - | CCGCCGCGAAUAGCCCC | 17 | 7289 |
| HSV1-UL54-714 | + | UCCGUCCACCCCGCCCC | 17 | 7477 |
| HSV1-UL54-491 | - | CUGGGGGACCCCGCCCC | 17 | 7254 |
| HSV1-UL54-595 | + | CCCGCCAUGCAGGCCCC | 17 | 7358 |
| HSV1-UL54-574 | - | UGCAUUUCUACCUCCCC | 17 | 7337 |
| HSV1-UL54-713 | + | GUCCGUCCACCCCGCCC | 17 | 7476 |
| HSV1-UL54-490 | - | CCUGGGGGACCCCGCCC | 17 | 7253 |
| HSV1-UL54-512 | - | CGCGGACCCCCGCGCCC | 17 | 7275 |
| HSV1-UL54-594 | + | GCCCGCCAUGCAGGCCC | 17 | 7357 |
| HSV1-UL54-499 | - | GGGUGGACGGACGGCCC | 17 | 7262 |
| HSV1-UL54-513 | - | CCCCCGCGCCCCGGCCC | 17 | 7276 |
| HSV1-UL54-650 | + | CGCCAGCACCGGGGCCC | 17 | 7413 |
| HSV1-UL54-528 | - | GAAUAGCCCCUGGGCCC | 17 | 7291 |
| HSV1-UL54-573 | - | AUGCAUUUCUACCUCCC | 17 | 7336 |
| HSV1-UL54-641 | + | GGCGACCAAGGUUUCCC | 17 | 7404 |
| HSV1-UL54-602 | + | GACGCGGUUGGCGAGCC | 17 | 7365 |
| HSV1-UL54-579 | - | CCUAGACACGCACCGCC | 17 | 7342 |
| HSV1-UL54-711 | + | UCGCCAUGGGGGGCGCC | 17 | 7474 |
| HSV1-UL54-558 | - | GCGAGGCCUGUGCGGCC | 17 | 7321 |
| HSV1-UL54-676 | + | CCUUUCGCUCCGGGGCC | 17 | 7439 |
| HSV1-UL54-565 | - | UGUUUGUCAUUCUGGCC | 17 | 7328 |
| HSV1-UL54-589 | + | CACGACUCGAACACUCC | 17 | 7352 |
| HSV1-UL54-700 | + | CGUGUCCGCUGGCCUCC | 17 | 7463 |
| HSV1-UL54-673 | + | CGGGCGCCUUUCGCUCC | 17 | 7436 |
| HSV1-UL54-537 | - | AGACCAGACGGGUCUCC | 17 | 7300 |
| HSV1-UL54-604 | + | GCGAACACAGUUCGUCC | 17 | 7367 |
| HSV1-UL54-617 | + | UCCCGAUAAUGGGGUCC | 17 | 7380 |
| HSV1-UL54-691 | + | UCGCCAGCGUCAUUAGC | 17 | 7454 |
| HSV1-UL54-1215 | + | UGGUUCUGGGGGCACGC | 17 | 18204 |
| HSV1-UL54-547 | - | CUCCGCCGACGAGACGC | 17 | 7310 |
| HSV1-UL54-510 | - | CCCCCCGCUAAUGACGC | 17 | 7273 |
| HSV1-UL54-56 | - | GUUUGUCGGACCCCCGC | 17 | 6819 |
| HSV1-UL54-610 | + | ACUGGAGAAAGGGCCGC | 17 | 7373 |
| HSV1-UL54-683 | + | GGGGCGCGGGGGUCCGC | 17 | 7446 |
| HSV1-UL54-698 | + | GCACGCCCCGUGUCCGC | 17 | 7461 |
| HSV1-UL54-664 | + | GCUCGGAGAUGGAGCGC | 17 | 7427 |
| HSV1-UL54-546 | - | AAAUUUCAUCGAGGCGC | 17 | 7309 |
| HSV1-UL54-679 | + | CUCCGGGGCCGGGGCGC | 17 | 7442 |
| HSV1-UL54-710 | + | CUCGCCAUGGGGGGCGC | 17 | 7473 |
| HSV1-UL54-1192 | + | UCUGGGGGCACGCCGGC | 17 | 18181 |
| HSV1-UL54-622 | + | CCUGGGGGCGCAGCGGC | 17 | 7385 |
| HSV1-UL54-655 | + | CAGGGGCUAUUCGCGGC | 17 | 7418 |
| HSV1-UL54-671 | + | UCGAUGGUGUCGGCGGC | 17 | 7434 |
| HSV1-UL54-685 | + | CGCGGGGGUCCGCGGGC | 17 | 7448 |
| HSV1-UL54-717 | + | CCACCCCGCCCCGGGGC | 17 | 7480 |
| HSV1-UL54-494 | - | GGACCCCGCCCCGGGGC | 17 | 7257 |
| HSV1-UL54-675 | + | GCCUUUCGCUCCGGGGC | 17 | 7438 |
| HSV1-UL54-578 | - | CCCGGGGCCUGCAUGGC | 17 | 7341 |
| HSV1-UL54-531 | - | UGGGCCCCGGUGCUGGC | 17 | 7294 |
| HSV1-UL54-593 | + | GAUCAGGCCCGCCAUGC | 17 | 7356 |
| HSV1-UL54-529 | - | CCCCUGGGCCCCGGUGC | 17 | 7292 |
| HSV1-UL54-553 | - | GACGACCGCGGCUGUGC | 17 | 7316 |
| HSV1-UL54-634 | + | UGCGGCCCGAGGAUUGC | 17 | 7397 |
| HSV1-UL54-541 | - | CUCUAUCGCACUUUUGC | 17 | 7304 |
| HSV1-UL54-592 | + | UGUCUAGGAUUUCGAUC | 17 | 7355 |
| HSV1-UL54-551 | - | CCCAGGACCCCAUUAUC | 17 | 7314 |
| HSV1-UL54-699 | + | CCGUGUCCGCUGGCCUC | 17 | 7462 |
| HSV1-UL54-543 | - | UUUUGCCGGCAAUCCUC | 17 | 7306 |
| HSV1-UL54-672 | + | GCGGGCGCCUUUCGCUC | 17 | 7435 |
| HSV1-UL54-637 | + | AAGUGCGAUAGAGGCUC | 17 | 7400 |
| HSV1-UL54-642 | + | UUCCCAGGAGACCCGUC | 17 | 7405 |
| HSV1-UL54-561 | - | GUGUUCGCGGCGGCGUC | 17 | 7324 |
| HSV1-UL54-704 | + | UGGCCUCCGGGUGGGUC | 17 | 7467 |
| HSV1-UL54-570 | - | ACGCGACCCUUGGUGUC | 17 | 7333 |
| HSV1-UL54-564 | - | CUUCGUGUUUGUCAUUC | 17 | 7327 |
| HSV1-UL54-606 | + | ACAGGCCUCGCGCCUUC | 17 | 7369 |
| HSV1-UL54-1185 | + | AGGGUUGCGAUUGGUUC | 17 | 7805 |
| HSV1-UL54-659 | + | ACGGCUGCCCCCCAAAG | 17 | 7422 |
| HSV1-UL54-652 | + | CCAGCACCGGGGCCCAG | 17 | 7415 |
| HSV1-UL54-506 | - | ACCCACCCGGAGGCCAG | 17 | 7269 |
| HSV1-UL54-621 | + | GGGUCCUGGGGGCGCAG | 17 | 7384 |
| HSV1-UL54-635 | + | CGGCAAAAGUGCGAUAG | 17 | 7398 |
| HSV1-UL54-690 | + | AUCGCCAGCGUCAUUAG | 17 | 7453 |
| HSV1-UL54-509 | - | GGAGGCCAGCGGACACG | 17 | 7272 |
| HSV1-UL54-515 | - | CCAUCGACGCCACCACG | 17 | 7278 |
| HSV1-UL54-600 | + | CGACGCCACGCUCGACG | 17 | 7363 |
| HSV1-UL54-584 | + | UUUGCCGUGCACGUACG | 17 | 7347 |
| HSV1-UL54-649 | + | UUGGCCCGCCAGCACCG | 17 | 7412 |
| HSV1-UL54-552 | - | CAUUAUCGGGACGACCG | 17 | 7315 |
| HSV1-UL54-715 | + | CCGUCCACCCCGCCCCG | 17 | 7478 |
| HSV1-UL54-492 | - | UGGGGGACCCCGCCCCG | 17 | 7255 |
| HSV1-UL54-596 | + | CCGCCAUGCAGGCCCCG | 17 | 7359 |
| HSV1-UL54-575 | - | GCAUUUCUACCUCCCCG | 17 | 7338 |
| HSV1-UL54-511 | - | GAUUGCCCCCCCGCCCG | 17 | 7274 |
| HSV1-UL54-633 | + | GCGGUCGAUGCGGCCCG | 17 | 7396 |
| HSV1-UL54-613 | + | GUUAUCCAGCACAGCCG | 17 | 7376 |
| HSV1-UL54-712 | + | CGCCAUGGGGGGCGCCG | 17 | 7475 |
| HSV1-UL54-638 | + | GAUAGAGGCUCGGGCCG | 17 | 7401 |
| HSV1-UL54-677 | + | CUUUCGCUCCGGGGCCG | 17 | 7440 |
| HSV1-UL54-674 | + | GGGCGCCUUUCGCUCCG | 17 | 7437 |
| HSV1-UL54-682 | + | CGGGGCGCGGGGGUCCG | 17 | 7445 |
| HSV1-UL54-566 | - | GCCAACCGCGUCGAGCG | 17 | 7329 |
| HSV1-UL54-692 | + | CGCCAGCGUCAUUAGCG | 17 | 7455 |
| HSV1-UL54-665 | + | GCGCAGGACCAACCGCG | 17 | 7428 |
| HSV1-UL54-517 | - | CUCCAUCUCCGAGCGCG | 17 | 7280 |
| HSV1-UL54-556 | - | UGCUACCUGAAGGCGCG | 17 | 7319 |
| HSV1-UL54-611 | + | AAAGGGCCGCAGGCGCG | 17 | 7374 |
| HSV1-UL54-680 | + | UCCGGGGCCGGGGCGCG | 17 | 7443 |
| HSV1-UL54-502 | - | CCCCCCAUGGCGAGGCG | 17 | 7265 |
| HSV1-UL54-1217 | + | CUGGGGGCACGCCGGCG | 17 | 18206 |
| HSV1-UL54-686 | + | GCGGGGGUCCGCGGGCG | 17 | 7449 |
| HSV1-UL54-718 | + | CACCCCGCCCCGGGGCG | 17 | 7481 |
| HSV1-UL54-495 | - | GACCCCGCCCCGGGGCG | 17 | 7258 |
| HSV1-UL54-678 | + | GCUCCGGGGCCGGGGCG | 17 | 7441 |
| HSV1-UL54-501 | - | CGGCGCCCCCCAUGGCG | 17 | 7264 |
| HSV1-UL54-548 | - | CCGACGAGACGCUGGCG | 17 | 7311 |
| HSV1-UL54-612 | + | GCCGCAGGCGCGUGGCG | 17 | 7375 |
| HSV1-UL54-503 | - | CAUGGCGAGGCGUGGCG | 17 | 7266 |
| HSV1-UL54-545 | - | CCAAGAAAAUUUCAUCG | 17 | 7308 |
| HSV1-UL54-567 | - | CGUCGAGCGUGGCGUCG | 17 | 7330 |
| HSV1-UL54-571 | - | CGCGACCCUUGGUGUCG | 17 | 7334 |
| HSV1-UL54-653 | + | GGCCCAGGGGCUAUUCG | 17 | 7416 |
| HSV1-UL54-559 | - | UGGACGAACUGUGUUCG | 17 | 7322 |
| HSV1-UL54-533 | - | GUGCUGGCGGGCCAAGG | 17 | 7296 |
| HSV1-UL54-585 | + | UUGCCGUGCACGUACGG | 17 | 7348 |
| HSV1-UL54-505 | - | CAGCCCGACCCACCCGG | 17 | 7268 |
| HSV1-UL54-1218 | + | UUCUGGGGGCACGCCGG | 17 | 18207 |
| HSV1-UL54-693 | + | GCCAGCGUCAUUAGCGG | 17 | 7456 |
| HSV1-UL54-518 | - | CAUCUCCGAGCGCGCGG | 17 | 7281 |
| HSV1-UL54-681 | + | CCGGGGCCGGGGCGCGG | 17 | 7444 |
| HSV1-UL54-654 | + | CCAGGGGCUAUUCGCGG | 17 | 7417 |
| HSV1-UL54-560 | - | ACGAACUGUGUUCGCGG | 17 | 7323 |
| HSV1-UL54-1219 | + | UGGGGGCACGCCGGCGG | 17 | 18208 |
| HSV1-UL54-627 | + | CAGCGUCUCGUCGGCGG | 17 | 7390 |
| HSV1-UL54-670 | + | GUCGAUGGUGUCGGCGG | 17 | 7433 |
| HSV1-UL54-687 | + | CGGGGGUCCGCGGGCGG | 17 | 7450 |
| HSV1-UL54-626 | + | CGCCAGCGUCUCGUCGG | 17 | 7389 |
| HSV1-UL54-669 | + | GGCGUCGAUGGUGUCGG | 17 | 7432 |
| HSV1-UL54-586 | + | UGCCGUGCACGUACGGG | 17 | 7349 |
| HSV1-UL54-701 | + | GUCCGCUGGCCUCCGGG | 17 | 7464 |
| HSV1-UL54-694 | + | CCAGCGUCAUUAGCGGG | 17 | 7457 |
| HSV1-UL54-684 | + | GCGCGGGGGUCCGCGGG | 17 | 7447 |
| HSV1-UL54-688 | + | GGGGGUCCGCGGGCGGG | 17 | 7451 |
| HSV1-UL54-587 | + | GCCGUGCACGUACGGGG | 17 | 7350 |
| HSV1-UL54-716 | + | UCCACCCCGCCCCGGGG | 17 | 7479 |
| HSV1-UL54-493 | - | GGGACCCCGCCCCGGGG | 17 | 7256 |
| HSV1-UL54-597 | + | CCAUGCAGGCCCCGGGG | 17 | 7360 |
| HSV1-UL54-695 | + | CAGCGUCAUUAGCGGGG | 17 | 7458 |
| HSV1-UL54-689 | + | GGGGUCCGCGGGCGGGG | 17 | 7452 |
| HSV1-UL54-496 | - | CCCGCCCCGGGGCGGGG | 17 | 7259 |
| HSV1-UL54-696 | + | AGC GUCAUUAGC GGGGG | 17 | 7459 |
| HSV1-UL54-709 | + | CCACGCCUCGCCAUGGG | 17 | 7472 |
| HSV1-UL54-525 | - | UGCACGACCCCUUUGGG | 17 | 7288 |
| HSV1-UL54-708 | + | GCCACGCCUCGCCAUGG | 17 | 7471 |
| HSV1-UL54-577 | - | CCCCGGGGCCUGCAUGG | 17 | 7340 |
| HSV1-UL54-489 | - | ACCAAUCGCAACCCUGG | 17 | 7252 |
| HSV1-UL54-590 | + | GACUCGAACACUCCUGG | 17 | 7353 |
| HSV1-UL54-620 | + | CGAUAAUGGGGUCCUGG | 17 | 7383 |
| HSV1-UL54-530 | - | CUGGGCCCCGGUGCUGG | 17 | 7293 |
| HSV1-UL54-562 | - | UUCGCGGCGGCGUCUGG | 17 | 7325 |
| HSV1-UL54-1210 | + | GUUGCGAUUGGUUCUGG | 17 | 18199 |
| HSV1-UL54-666 | + | CAGGACCAACCGCGUGG | 17 | 7429 |
| HSV1-UL54-624 | + | GCAGCGGCAGGUUGUGG | 17 | 7387 |
| HSV1-UL54-631 | + | GUCGCGCAUGGCCUUGG | 17 | 7394 |
| HSV1-UL54-524 | - | AUGCACGACCCCUUUGG | 17 | 7287 |
| HSV1-UL54-616 | + | CGGUCGUCCCGAUAAUG | 17 | 7379 |
| HSV1-UL54-707 | + | CGCCACGCCUCGCCAUG | 17 | 7470 |
| HSV1-UL54-632 | + | GGCCUUGGCGGUCGAUG | 17 | 7395 |
| HSV1-UL54-661 | + | CAAAGCUCUCGCUGAUG | 17 | 7424 |
| HSV1-UL54-488 | - | AACCAAUCGCAACCCUG | 17 | 7251 |
| HSV1-UL54-554 | - | ACCUCGCCACGCGCCUG | 17 | 7317 |
| HSV1-UL54-619 | + | CCGAUAAUGGGGUCCUG | 17 | 7382 |
| HSV1-UL54-660 | + | GCAUGACCUGUGCGCUG | 17 | 7423 |
| HSV1-UL54-1187 | + | GGUUGCGAUUGGUUCUG | 17 | 7807 |
| HSV1-UL54-557 | - | AAGGCGCGAGGCCUGUG | 17 | 7320 |
| HSV1-UL54-623 | + | GGCGCAGCGGCAGGUUG | 17 | 7386 |
| HSV1-UL54-523 | - | CAUGCACGACCCCUUUG | 17 | 7286 |
| HSV1-UL54-615 | + | GCGGUCGUCCCGAUAAU | 17 | 7378 |
| HSV1-UL54-706 | + | GCGCCACGCCUCGCCAU | 17 | 7469 |
| HSV1-UL54-721 | + | UCCCCCAGGGUUGCGAU | 17 | 7484 |
| HSV1-UL54-550 | - | CCCCAGGACCCCAUUAU | 17 | 7313 |
| HSV1-UL54-539 | - | GGUCUCCUGGGAAACCU | 17 | 7302 |
| HSV1-UL54-487 | - | GAACCAAUCGCAACCCU | 17 | 7250 |
| HSV1-UL54-568 | - | AUCGACUACGCGACCCU | 17 | 7331 |
| HSV1-UL54-527 | - | CGCCGCGAAUAGCCCCU | 17 | 7290 |
| HSV1-UL54-630 | + | GCAGUCGCGCAUGGCCU | 17 | 7393 |
| HSV1-UL54-542 | - | CUUUUGCCGGCAAUCCU | 17 | 7305 |
| HSV1-UL54-646 | + | CGUCAAAGGGCCCUCCU | 17 | 7409 |
| HSV1-UL54-538 | - | GACCAGACGGGUCUCCU | 17 | 7301 |
| HSV1-UL54-618 | + | CCCGAUAAUGGGGUCCU | 17 | 7381 |
| HSV1-UL54-662 | + | GUCGACCGCCGCGCGCU | 17 | 7425 |
| HSV1-UL54-636 | + | AAAGUGCGAUAGAGGCU | 17 | 7399 |
| HSV1-UL54-697 | + | UUAGCGGGGGGGGUGCU | 17 | 7460 |
| HSV1-UL54-643 | + | GGAGACCCGUCUGGUCU | 17 | 7406 |
| HSV1-UL54-591 | + | CCUGGCGGUGCGUGUCU | 17 | 7354 |
| HSV1-UL54-1186 | + | GGGUUGCGAUUGGUUCU | 17 | 7806 |
| HSV1-UL54-628 | + | CCUCGAUGAAAUUUUCU | 17 | 7391 |
| HSV1-UL54-639 | + | AUAGAGGCUCGGGCCGU | 17 | 7402 |
| HSV1-UL54-625 | + | CCACGCCAGCGUCUCGU | 17 | 7388 |
| HSV1-UL54-516 | - | CGACGCCACCACGCGGU | 17 | 7279 |
| HSV1-UL54-601 | + | GCCACGCUCGACGCGGU | 17 | 7364 |
| HSV1-UL54-702 | + | UCCGCUGGCCUCCGGGU | 17 | 7465 |
| HSV1-UL54-572 | - | ACCCUUGGUGUCGGGGU | 17 | 7335 |
| HSV1-UL54-703 | + | CUGGCCUCCGGGUGGGU | 17 | 7466 |
| HSV1-UL54-668 | + | GGUGGCGUCGAUGGUGU | 17 | 7431 |
| HSV1-UL54-569 | - | UACGCGACCCUUGGUGU | 17 | 7332 |
| HSV1-UL54-521 | - | GUCAUGCACGACCCCUU | 17 | 7284 |
| HSV1-UL54-519 | - | CGCAUCAGCGAGAGCUU | 17 | 7282 |
| HSV1-UL54-522 | - | UCAUGCACGACCCCUUU | 17 | 7285 |
| HSV1-UL54-1224 | - | UUGCAACUCCCUGUUUU | 17 | 18213 |
| HSV1-UL54-422 | + | GGAAACGGCUGCCCCCCAAA | 20 | 7185 |
| HSV1-UL54-409 | + | CGUCUGGUCUCGGCGUCAAA | 20 | 7172 |
| HSV1-UL54-373 | + | UUCAGGUAGCACUGGAGAAA | 20 | 7136 |
| HSV1-UL54-420 | + | GGGCUAUUCGCGGCGGGAAA | 20 | 7183 |
| HSV1-UL54-363 | + | CUCUCCGACCCCGACACCAA | 20 | 7126 |
| HSV1-UL54-421 | + | GGGAAACGGCUGCCCCCCAA | 20 | 7184 |
| HSV1-UL54-408 | + | CCGUCUGGUCUCGGCGUCAA | 20 | 7171 |
| HSV1-UL54-372 | + | CUUCAGGUAGCACUGGAGAA | 20 | 7135 |
| HSV1-UL54-278 | - | CGCCCCGGCCCCGGAGCGAA | 20 | 7041 |
| HSV1-UL54-378 | + | AGCCGCGGUCGUCCCGAUAA | 20 | 7141 |
| HSV1-UL54-1225 | + | UUUUUAUUGUACCUAAAACA | 20 | 18214 |
| HSV1-UL54-271 | - | CACCCGGAGGCCAGCGGACA | 20 | 7034 |
| HSV1-UL54-362 | + | UCUCUCCGACCCCGACACCA | 20 | 7125 |
| HSV1-UL54-404 | + | GCUCGGGCCGUGGGCGACCA | 20 | 7167 |
| HSV1-UL54-264 | - | GACGGCCCCGGCGCCCCCCA | 20 | 7027 |
| HSV1-UL54-484 | + | CCCCGGGGCGGGGUCCCCCA | 20 | 7247 |
| HSV1-UL54-304 | - | UGGGAAACCUUGGUCGCCCA | 20 | 7067 |
| HSV1-UL54-415 | + | CCCGCCAGCACCGGGGCCCA | 20 | 7178 |
| HSV1-UL54-308 | - | UCGGGCCGCAUCGACCGCCA | 20 | 7071 |
| HSV1-UL54-469 | + | UGCCGCGCCACGCCUCGCCA | 20 | 7232 |
| HSV1-UL54-296 | - | GCCCCGGUGCUGGCGGGCCA | 20 | 7059 |
| HSV1-UL54-393 | + | GCGCAGCACGCAGUCGCGCA | 20 | 7156 |
| HSV1-UL54-340 | - | CUACCUCCCCGGGGCCUGCA | 20 | 7103 |
| HSV1-UL54-345 | - | GUCGCCCCCCCGUACGUGCA | 20 | 7108 |
| HSV1-UL54-299 | - | CCUUUGACGCCGAGACCAGA | 20 | 7062 |
| HSV1-UL54-427 | + | GACCGCCGCGCGCUCGGAGA | 20 | 7190 |
| HSV1-UL54-367 | + | GGCCAGAAUGACAAACACGA | 20 | 7130 |
| HSV1-UL54-431 | + | CAACCGCGUGGUGGCGUCGA | 20 | 7194 |
| HSV1-UL54-298 | - | CGGUGCUGGCGGGCCAAGGA | 20 | 7061 |
| HSV1-UL54-262 | - | CCCGGGGCGGGGUGGACGGA | 20 | 7025 |
| HSV1-UL54-261 | - | CCGCCCCGGGGCGGGGUGGA | 20 | 7024 |
| HSV1-UL54-319 | - | CUUUCUCCAGUGCUACCUGA | 20 | 7082 |
| HSV1-UL54-344 | - | GAGUCGUGUCUGCGAGUUGA | 20 | 7107 |
| HSV1-UL54-346 | + | AAAUAUUUGCCGUGCACGUA | 20 | 7109 |
| HSV1-UL54-327 | - | GCGGCGUCUGGCGGACAUUA | 20 | 7090 |
| HSV1-UL54-1226 | + | GUUUUUAUUGUACCUAAAAC | 20 | 18215 |
| HSV1-UL54-272 | - | ACCCGGAGGCCAGCGGACAC | 20 | 7035 |
| HSV1-UL54-411 | + | CCUCCUUGGCCCGCCAGCAC | 20 | 7174 |
| HSV1-UL54-371 | + | CUCGCGCCUUCAGGUAGCAC | 20 | 7134 |
| HSV1-UL54-369 | + | ACAGUUCGUCCAGGCCGCAC | 20 | 7132 |
| HSV1-UL54-284 | - | GAGCUUUGGCCGCAGCGCAC | 20 | 7047 |
| HSV1-UL54-300 | - | CUUUGACGCCGAGACCAGAC | 20 | 7063 |
| HSV1-UL54-352 | + | CGGGGGGGCGACGAUGUGAC | 20 | 7115 |
| HSV1-UL54-347 | + | AAUAUUUGCCGUGCACGUAC | 20 | 7110 |
| HSV1-UL54-268 | - | AGUGAGCAGCCCGACCCACC | 20 | 7031 |
| HSV1-UL54-412 | + | CUCCUUGGCCCGCCAGCACC | 20 | 7175 |
| HSV1-UL54-1195 | - | CCCAGAACCAAUCGCAACCC | 20 | 18184 |
| HSV1-UL54-313 | - | CAACCUGCCGCUGCGCCCCC | 20 | 7076 |
| HSV1-UL54-483 | + | GCCCCGGGGCGGGGUCCCCC | 20 | 7246 |
| HSV1-UL54-290 | - | UUCCCGCCGCGAAUAGCCCC | 20 | 7053 |
| HSV1-UL54-478 | + | CCGUCCGUCCACCCCGCCCC | 20 | 7241 |
| HSV1-UL54-255 | - | ACCCUGGGGGACCCCGCCCC | 20 | 7018 |
| HSV1-UL54-359 | + | AGGCCCGCCAUGCAGGCCCC | 20 | 7122 |
| HSV1-UL54-338 | - | AGAUGCAUUUCUACCUCCCC | 20 | 7101 |
| HSV1-UL54-477 | + | GCCGUCCGUCCACCCCGCCC | 20 | 7240 |
| HSV1-UL54-254 | - | AACCCUGGGGGACCCCGCCC | 20 | 7017 |
| HSV1-UL54-276 | - | GCCCGCGGACCCCCGCGCCC | 20 | 7039 |
| HSV1-UL54-358 | + | CAGGCCCGCCAUGCAGGCCC | 20 | 7121 |
| HSV1-UL54-263 | - | GCGGGGUGGACGGACGGCCC | 20 | 7026 |
| HSV1-UL54-277 | - | GGACCCCCGCGCCCCGGCCC | 20 | 7040 |
| HSV1-UL54-414 | + | GCCCGCCAGCACCGGGGCCC | 20 | 7177 |
| HSV1-UL54-292 | - | CGCGAAUAGCCCCUGGGCCC | 20 | 7055 |
| HSV1-UL54-337 | - | AAGAUGCAUUUCUACCUCCC | 20 | 7100 |
| HSV1-UL54-405 | + | GUGGGCGACCAAGGUUUCCC | 20 | 7168 |
| HSV1-UL54-366 | + | CUCGACGCGGUUGGCGAGCC | 20 | 7129 |
| HSV1-UL54-343 | - | AAUCCUAGACACGCACCGCC | 20 | 7106 |
| HSV1-UL54-475 | + | GCCUCGCCAUGGGGGGCGCC | 20 | 7238 |
| HSV1-UL54-322 | - | GGCGCGAGGCCUGUGCGGCC | 20 | 7085 |
| HSV1-UL54-440 | + | GCGCCUUUCGCUCCGGGGCC | 20 | 7203 |
| HSV1-UL54-329 | - | UCGUGUUUGUCAUUCUGGCC | 20 | 7092 |
| HSV1-UL54-353 | + | AGACACGACUCGAACACUCC | 20 | 7116 |
| HSV1-UL54-464 | + | CCCCGUGUCCGCUGGCCUCC | 20 | 7227 |
| HSV1-UL54-437 | + | CGGCGGGCGCCUUUCGCUCC | 20 | 7200 |
| HSV1-UL54-301 | - | CCGAGACCAGACGGGUCUCC | 20 | 7064 |
| HSV1-UL54-368 | + | GCCGCGAACACAGUUCGUCC | 20 | 7131 |
| HSV1-UL54-381 | + | UCGUCCCGAUAAUGGGGUCC | 20 | 7144 |
| HSV1-UL54-455 | + | CAAUCGCCAGCGUCAUUAGC | 20 | 7218 |
| HSV1-UL54-1188 | + | GAUUGGUUCUGGGGGCACGC | 20 | 18177 |
| HSV1-UL54-311 | - | GGCCUCCGCCGACGAGACGC | 20 | 7074 |
| HSV1-UL54-274 | - | ACCCCCCCCGCUAAUGACGC | 20 | 7037 |
| HSV1-UL54-374 | + | AGCACUGGAGAAAGGGCCGC | 20 | 7137 |
| HSV1-UL54-447 | + | GCCGGGGCGCGGGGGUCCGC | 20 | 7210 |
| HSV1-UL54-462 | + | GGCGCACGCCCCGUGUCCGC | 20 | 7225 |
| HSV1-UL54-428 | + | CGCGCUCGGAGAUGGAGCGC | 20 | 7191 |
| HSV1-UL54-310 | - | AGAAAAUUUCAUCGAGGCGC | 20 | 7073 |
| HSV1-UL54-443 | + | UCGCUCCGGGGCCGGGGCGC | 20 | 7206 |
| HSV1-UL54-474 | + | CGCCUCGCCAUGGGGGGCGC | 20 | 7237 |
| HSV1-UL54-1189 | + | GGUUCUGGGGGCACGCCGGC | 20 | 18178 |
| HSV1-UL54-386 | + | GGUCCUGGGGGCGCAGCGGC | 20 | 7149 |
| HSV1-UL54-419 | + | GCCCAGGGGCUAUUCGCGGC | 20 | 7182 |
| HSV1-UL54-435 | + | GCGUCGAUGGUGUCGGCGGC | 20 | 7198 |
| HSV1-UL54-449 | + | GGGCGCGGGGGUCCGCGGGC | 20 | 7212 |
| HSV1-UL54-481 | + | CGUCCACCCCGCCCCGGGGC | 20 | 7244 |
| HSV1-UL54-258 | - | GGGGGACCCCGCCCCGGGGC | 20 | 7021 |
| HSV1-UL54-439 | + | GGCGCCUUUCGCUCCGGGGC | 20 | 7202 |
| HSV1-UL54-342 | - | CUCCCCGGGGCCUGCAUGGC | 20 | 7105 |
| HSV1-UL54-295 | - | CCCUGGGCCCCGGUGCUGGC | 20 | 7058 |
| HSV1-UL54-357 | + | UUCGAUCAGGCCCGCCAUGC | 20 | 7120 |
| HSV1-UL54-293 | - | UAGCCCCUGGGCCCCGGUGC | 20 | 7056 |
| HSV1-UL54-317 | - | CGGGACGACCGCGGCUGUGC | 20 | 7080 |
| HSV1-UL54-398 | + | CGAUGCGGCCCGAGGAUUGC | 20 | 7161 |
| HSV1-UL54-305 | - | AGCCUCUAUCGCACUUUUGC | 20 | 7068 |
| HSV1-UL54-356 | + | GC GUGUCUAGGAUUUC GAUC | 20 | 7119 |
| HSV1-UL54-315 | - | GCCCCCAGGACCCCAUUAUC | 20 | 7078 |
| HSV1-UL54-463 | + | GCCCCGUGUCCGCUGGCCUC | 20 | 7226 |
| HSV1-UL54-307 | - | CACUUUUGCCGGCAAUCCUC | 20 | 7070 |
| HSV1-UL54-436 | + | GCGGCGGGCGCCUUUCGCUC | 20 | 7199 |
| HSV1-UL54-401 | + | CAAAAGUGCGAUAGAGGCUC | 20 | 7164 |
| HSV1-UL54-406 | + | GGUUUCCCAGGAGACCCGUC | 20 | 7169 |
| HSV1-UL54-325 | - | ACUGUGUUCGCGGCGGCGUC | 20 | 7088 |
| HSV1-UL54-468 | + | CGCUGGCCUCCGGGUGGGUC | 20 | 7231 |
| HSV1-UL54-334 | - | ACUACGCGACCCUUGGUGUC | 20 | 7097 |
| HSV1-UL54-328 | - | AUCCUUCGUGUUUGUCAUUC | 20 | 7091 |
| HSV1-UL54-370 | + | CGCACAGGCCUCGCGCCUUC | 20 | 7133 |
| HSV1-UL54-1199 | + | CCCAGGGUUGCGAUUGGUUC | 20 | 18188 |
| HSV1-UL54-423 | + | GAAACGGCUGCCCCCCAAAG | 20 | 7186 |
| HSV1-UL54-416 | + | CCGCCAGCACCGGGGCCCAG | 20 | 7179 |
| HSV1-UL54-270 | - | CCGACCCACCCGGAGGCCAG | 20 | 7033 |
| HSV1-UL54-385 | + | AUGGGGUCCUGGGGGCGCAG | 20 | 7148 |
| HSV1-UL54-399 | + | UGCCGGCAAAAGUGCGAUAG | 20 | 7162 |
| HSV1-UL54-454 | + | GCAAUCGCCAGCGUCAUUAG | 20 | 7217 |
| HSV1-UL54-273 | - | CCCGGAGGCCAGCGGACACG | 20 | 7036 |
| HSV1-UL54-279 | - | ACACCAUCGACGCCACCACG | 20 | 7042 |
| HSV1-UL54-364 | + | CCGCGACGCCACGCUCGACG | 20 | 7127 |
| HSV1-UL54-348 | + | AUAUUUGCCGUGCACGUACG | 20 | 7111 |
| HSV1-UL54-413 | + | UCCUUGGCCCGCCAGCACCG | 20 | 7176 |
| HSV1-UL54-316 | - | CCCCAUUAUCGGGACGACCG | 20 | 7079 |
| HSV1-UL54-479 | + | CGUCCGUCCACCCCGCCCCG | 20 | 7242 |
| HSV1-UL54-256 | - | CCCUGGGGGACCCCGCCCCG | 20 | 7019 |
| HSV1-UL54-360 | + | GGCCCGCCAUGCAGGCCCCG | 20 | 7123 |
| HSV1-UL54-339 | - | GAUGCAUUUCUACCUCCCCG | 20 | 7102 |
| HSV1-UL54-275 | - | GGCGAUUGCCCCCCCGCCCG | 20 | 7038 |
| HSV1-UL54-397 | + | UUGGCGGUCGAUGCGGCCCG | 20 | 7160 |
| HSV1-UL54-377 | + | GAGGUUAUCCAGCACAGCCG | 20 | 7140 |
| HSV1-UL54-476 | + | CCUCGCCAUGGGGGGCGCCG | 20 | 7239 |
| HSV1-UL54-402 | + | UGCGAUAGAGGCUCGGGCCG | 20 | 7165 |
| HSV1-UL54-441 | + | CGCCUUUCGCUCCGGGGCCG | 20 | 7204 |
| HSV1-UL54-438 | + | GGCGGGCGCCUUUCGCUCCG | 20 | 7201 |
| HSV1-UL54-446 | + | GGCCGGGGCGCGGGGGUCCG | 20 | 7209 |
| HSV1-UL54-330 | - | CUCGCCAACCGCGUCGAGCG | 20 | 7093 |
| HSV1-UL54-456 | + | AAUCGCCAGCGUCAUUAGCG | 20 | 7219 |
| HSV1-UL54-429 | + | GGAGCGCAGGACCAACCGCG | 20 | 7192 |
| HSV1-UL54-281 | - | GCGCUCCAUCUCCGAGCGCG | 20 | 7044 |
| HSV1-UL54-320 | - | CAGUGCUACCUGAAGGCGCG | 20 | 7083 |
| HSV1-UL54-375 | + | GAGAAAGGGCCGCAGGCGCG | 20 | 7138 |
| HSV1-UL54-444 | + | CGCUCCGGGGCCGGGGCGCG | 20 | 7207 |
| HSV1-UL54-266 | - | GCGCCCCCCAUGGCGAGGCG | 20 | 7029 |
| HSV1-UL54-1190 | + | GUUCUGGGGGCACGCCGGCG | 20 | 18179 |
| HSV1-UL54-450 | + | GGCGCGGGGGUCCGCGGGCG | 20 | 7213 |
| HSV1-UL54-482 | + | GUCCACCCCGCCCCGGGGCG | 20 | 7245 |
| HSV1-UL54-259 | - | GGGGACCCCGCCCCGGGGCG | 20 | 7022 |
| HSV1-UL54-442 | + | UUCGCUCCGGGGCCGGGGCG | 20 | 7205 |
| HSV1-UL54-265 | - | CCCCGGCGCCCCCCAUGGCG | 20 | 7028 |
| HSV1-UL54-312 | - | CCGCCGACGAGACGCUGGCG | 20 | 7075 |
| HSV1-UL54-376 | + | AGGGCCGCAGGCGCGUGGCG | 20 | 7139 |
| HSV1-UL54-267 | - | CCCCAUGGCGAGGCGUGGCG | 20 | 7030 |
| HSV1-UL54-309 | - | GCGCCAAGAAAAUUUCAUCG | 20 | 7072 |
| HSV1-UL54-331 | - | CCGCGUCGAGCGUGGCGUCG | 20 | 7094 |
| HSV1-UL54-335 | - | CUACGCGACCCUUGGUGUCG | 20 | 7098 |
| HSV1-UL54-417 | + | CGGGGCCCAGGGGCUAUUCG | 20 | 7180 |
| HSV1-UL54-323 | - | GCCUGGACGAACUGUGUUCG | 20 | 7086 |
| HSV1-UL54-297 | - | CCGGUGCUGGCGGGCCAAGG | 20 | 7060 |
| HSV1-UL54-349 | + | UAUUUGCCGUGCACGUACGG | 20 | 7112 |
| HSV1-UL54-269 | - | GAGCAGCCCGACCCACCCGG | 20 | 7032 |
| HSV1-UL54-1201 | + | UGGUUCUGGGGGCACGCCGG | 20 | 18190 |
| HSV1-UL54-457 | + | AUCGCCAGCGUCAUUAGCGG | 20 | 7220 |
| HSV1-UL54-282 | - | CUCCAUCUCCGAGCGCGCGG | 20 | 7045 |
| HSV1-UL54-445 | + | GCUCCGGGGCCGGGGCGCGG | 20 | 7208 |
| HSV1-UL54-418 | + | GGCCCAGGGGCUAUUCGCGG | 20 | 7181 |
| HSV1-UL54-324 | - | UGGACGAACUGUGUUCGCGG | 20 | 7087 |
| HSV1-UL54-1202 | + | UUCUGGGGGCACGCCGGCGG | 20 | 18191 |
| HSV1-UL54-391 | + | CGCCAGCGUCUCGUCGGCGG | 20 | 7154 |
| HSV1-UL54-434 | + | GGCGUCGAUGGUGUCGGCGG | 20 | 7197 |
| HSV1-UL54-451 | + | GCGCGGGGGUCCGCGGGCGG | 20 | 7214 |
| HSV1-UL54-390 | + | CCACGCCAGCGUCUCGUCGG | 20 | 7153 |
| HSV1-UL54-433 | + | GGUGGCGUCGAUGGUGUCGG | 20 | 7196 |
| HSV1-UL54-350 | + | AUUUGCCGUGCACGUACGGG | 20 | 7113 |
| HSV1-UL54-465 | + | CGUGUCCGCUGGCCUCCGGG | 20 | 7228 |
| HSV1-UL54-458 | + | UCGCCAGCGUCAUUAGCGGG | 20 | 7221 |
| HSV1-UL54-448 | + | GGGGCGCGGGGGUCCGCGGG | 20 | 7211 |
| HSV1-UL54-452 | + | CGCGGGGGUCCGCGGGCGGG | 20 | 7215 |
| HSV1-UL54-351 | + | UUUGCCGUGCACGUACGGGG | 20 | 7114 |
| HSV1-UL54-480 | + | CCGUCCACCCCGCCCCGGGG | 20 | 7243 |
| HSV1-UL54-257 | - | UGGGGGACCCCGCCCCGGGG | 20 | 7020 |
| HSV1-UL54-361 | + | CCGCCAUGCAGGCCCCGGGG | 20 | 7124 |
| HSV1-UL54-459 | + | CGCCAGCGUCAUUAGCGGGG | 20 | 7222 |
| HSV1-UL54-453 | + | GCGGGGGUCCGCGGGCGGGG | 20 | 7216 |
| HSV1-UL54-260 | - | GACCCCGCCCCGGGGCGGGG | 20 | 7023 |
| HSV1-UL54-460 | + | GCCAGCGUCAUUAGCGGGGG | 20 | 7223 |
| HSV1-UL54-473 | + | GCGCCACGCCUCGCCAUGGG | 20 | 7236 |
| HSV1-UL54-289 | - | UCAUGCACGACCCCUUUGGG | 20 | 7052 |
| HSV1-UL54-472 | + | CGCGCCACGCCUCGCCAUGG | 20 | 7235 |
| HSV1-UL54-341 | - | CCUCCCCGGGGCCUGCAUGG | 20 | 7104 |
| HSV1-UL54-253 | - | AGAACCAAUCGCAACCCUGG | 20 | 7016 |
| HSV1-UL54-354 | + | CACGACUCGAACACUCCUGG | 20 | 7117 |
| HSV1-UL54-384 | + | UCCCGAUAAUGGGGUCCUGG | 20 | 7147 |
| HSV1-UL54-294 | - | CCCCUGGGCCCCGGUGCUGG | 20 | 7057 |
| HSV1-UL54-326 | - | GUGUUCGCGGCGGCGUCUGG | 20 | 7089 |
| HSV1-UL54-1203 | + | AGGGUUGCGAUUGGUUCUGG | 20 | 18192 |
| HSV1-UL54-430 | + | GCGCAGGACCAACCGCGUGG | 20 | 7193 |
| HSV1-UL54-388 | + | GGCGCAGCGGCAGGUUGUGG | 20 | 7151 |
| HSV1-UL54-395 | + | GCAGUCGCGCAUGGCCUUGG | 20 | 7158 |
| HSV1-UL54-288 | - | GUCAUGCACGACCCCUUUGG | 20 | 7051 |
| HSV1-UL54-380 | + | CCGCGGUCGUCCCGAUAAUG | 20 | 7143 |
| HSV1-UL54-471 | + | CCGCGCCACGCCUCGCCAUG | 20 | 7234 |
| HSV1-UL54-396 | + | CAUGGCCUUGGCGGUCGAUG | 20 | 7159 |
| HSV1-UL54-425 | + | GGCCAAAGCUCUCGCUGAUG | 20 | 7188 |
| HSV1-UL54-910 | - | CAGAACCAAUCGCAACCCUG | 20 | 7585 |
| HSV1-UL54-318 | - | AUAACCUCGCCACGCGCCUG | 20 | 7081 |
| HSV1-UL54-383 | + | GUCCCGAUAAUGGGGUCCUG | 20 | 7146 |
| HSV1-UL54-424 | + | CGUGCAUGACCUGUGCGCUG | 20 | 7187 |
| HSV1-UL54-1045 | + | CAGGGUUGCGAUUGGUUCUG | 20 | 7697 |
| HSV1-UL54-321 | - | CUGAAGGCGCGAGGCCUGUG | 20 | 7084 |
| HSV1-UL54-387 | + | GGGGGCGCAGCGGCAGGUUG | 20 | 7150 |
| HSV1-UL54-287 | - | GGUCAUGCACGACCCCUUUG | 20 | 7050 |
| HSV1-UL54-379 | + | GCCGCGGUCGUCCCGAUAAU | 20 | 7142 |
| HSV1-UL54-470 | + | GCCGCGCCACGCCUCGCCAU | 20 | 7233 |
| HSV1-UL54-485 | + | GGGUCCCCCAGGGUUGCGAU | 20 | 7248 |
| HSV1-UL54-314 | - | CGCCCCCAGGACCCCAUUAU | 20 | 7077 |
| HSV1-UL54-303 | - | ACGGGUCUCCUGGGAAACCU | 20 | 7066 |
| HSV1-UL54-1204 | - | CCAGAACCAAUCGCAACCCU | 20 | 18193 |
| HSV1-UL54-332 | - | GAGAUCGACUACGCGACCCU | 20 | 7095 |
| HSV1-UL54-291 | - | UCCCGCCGCGAAUAGCCCCU | 20 | 7054 |
| HSV1-UL54-394 | + | CACGCAGUCGCGCAUGGCCU | 20 | 7157 |
| HSV1-UL54-306 | - | GCACUUUUGCCGGCAAUCCU | 20 | 7069 |
| HSV1-UL54-410 | + | CGGCGUCAAAGGGCCCUCCU | 20 | 7173 |
| HSV1-UL54-302 | - | CGAGACCAGACGGGUCUCCU | 20 | 7065 |
| HSV1-UL54-382 | + | CGUCCCGAUAAUGGGGUCCU | 20 | 7145 |
| HSV1-UL54-426 | + | GCGGUCGACCGCCGCGCGCU | 20 | 7189 |
| HSV1-UL54-400 | + | GCAAAAGUGCGAUAGAGGCU | 20 | 7163 |
| HSV1-UL54-461 | + | UCAUUAGCGGGGGGGGUGCU | 20 | 7224 |
| HSV1-UL54-407 | + | CCAGGAGACCCGUCUGGUCU | 20 | 7170 |
| HSV1-UL54-355 | + | ACUCCUGGCGGUGCGUGUCU | 20 | 7118 |
| HSV1-UL54-1221 | + | CCAGGGUUGCGAUUGGUUCU | 20 | 18210 |
| HSV1-UL54-392 | + | GCGCCUCGAUGAAAUUUUCU | 20 | 7155 |
| HSV1-UL54-403 | + | GCGAUAGAGGCUCGGGCCGU | 20 | 7166 |
| HSV1-UL54-389 | + | GCACCACGCCAGCGUCUCGU | 20 | 7152 |
| HSV1-UL54-280 | - | CAUCGACGCCACCACGCGGU | 20 | 7043 |
| HSV1-UL54-365 | + | GACGCCACGCUCGACGCGGU | 20 | 7128 |
| HSV1-UL54-466 | + | GUGUCCGCUGGCCUCCGGGU | 20 | 7229 |
| HSV1-UL54-336 | - | GCGACCCUUGGUGUCGGGGU | 20 | 7099 |
| HSV1-UL54-467 | + | CCGCUGGCCUCCGGGUGGGU | 20 | 7230 |
| HSV1-UL54-432 | + | CGUGGUGGCGUCGAUGGUGU | 20 | 7195 |
| HSV1-UL54-333 | - | GACUACGCGACCCUUGGUGU | 20 | 7096 |
| HSV1-UL54-285 | - | CAGGUCAUGCACGACCCCUU | 20 | 7048 |
| HSV1-UL54-283 | - | GACCGCAUCAGCGAGAGCUU | 20 | 7046 |
| HSV1-UL54-286 | - | AGGUCAUGCACGACCCCUUU | 20 | 7049 |
| HSV1-UL54-1227 | - | UUAUUGCAACUCCCUGUUUU | 20 | 18216 |

**Table 15A** provides exemplary targeting domains for knocking out the *UL54* gene selected according to the first tier parameters. The targeting domains bind within the first 500 bp of the coding sequence (e.g., within 500 bp downstream from the start codon), have a high level of orthogonality, and start with a 5'G. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15A**

| 1st Tier | | | | |
|---|---|---|---|---|
| gRNA Name | DNA Strand | Targeting Domain | Target Site Length | Seq ID NO: |
| HSV1-UL54-1228 | + | GCGUCUGGGUGCUGGGUACGC | 21 | 18217 |
| HSV1-UL54-1229 | + | GGCGUCUGGGUGCUGGGUACGC | 22 | 18218 |
| HSV1-UL54-1230 | + | GAGGCGUCUGGGUGCUGGGUACGC | 24 | 18219 |
| HSV1-UL54-1231 | + | GUUCUGGGGGCACGCCGGC | 19 | 18220 |
| HSV1-UL54-1189 | + | GGUUCUGGGGGCACGCCGGC | 20 | 18178 |
| HSV1-UL54-1232 | + | GAUUGGUUCUGGGGGCACGCCGGC | 24 | 18221 |
| HSV1-UL54-1233 | + | GACCGCCGGGCGAGCGGCG | 19 | 18222 |
| HSV1-UL54-1234 | + | GGACCGCCGGGCGAGCGGCG | 20 | 18223 |
| HSV1-UL54-1235 | + | GCGGACCGCCGGGCGAGCGGCG | 22 | 18224 |
| HSV1-UL54-1236 | + | GGCGGACCGCCGGGCGAGCGGCG | 23 | 18225 |
| HSV1-UL54-1237 | + | GGGCGGACCGCCGGGCGAGCGGCG | 24 | 18226 |
| HSV1-UL54-1238 | + | GGCUCUCCGCCGGCUCGG | 18 | 18227 |
| HSV1-UL54-165 | + | GCGGCUCUCCGCCGGCUCGG | 20 | 6928 |
| HSV1-UL54-1239 | + | GGCGGCUCUCCGCCGGCUCGG | 21 | 18228 |
| HSV1-UL54-1240 | + | GCGGCGGCUCUCCGCCGGCUCGG | 23 | 18229 |
| HSV1-UL54-1241 | + | GGACGAGGCGUCUGGGUG | 18 | 18230 |
| HSV1-UL54-1242 | + | GUCGGACGAGGCGUCUGGGUG | 21 | 18231 |
| HSV1-UL54-1243 | + | GCUCCGGUCCGUCCUCUCCGU | 21 | 18232 |
| HSV1-UL54-1244 | + | GGCUCCGGUCCGUCCUCUCCGU | 22 | 18233 |
| HSV1-UL54-1245 | + | GCUCCGUCGGACGAGGCGU | 19 | 18234 |
| HSV1-UL54-1246 | + | GCCGCUCCGUCGGACGAGGCGU | 22 | 18235 |
| HSV1-UL54-1247 | + | GGCUGGGCUUUGGUCGGU | 18 | 18236 |
| HSV1-UL54-1248 | + | GCAGGCUGGGCUUUGGUCGGU | 21 | 18237 |
| HSV1-UL54-1249 | + | GGCAGGCUGGGCUUUGGUCGGU | 22 | 18238 |
| HSV1-UL54-1250 | + | GGGCAGGCUGGGCUUUGGUCGGU | 23 | 18239 |
| HSV1-UL54-1251 | - | GGCGGACGCCGUGGGCGUCGC | 21 | 18240 |
| HSV1-UL54-1252 | - | GCGGCGGACGCCGUGGGCGUCGC | 23 | 18241 |
| HSV1-UL54-1253 | - | GACCUGGAAUCGGACAGCAGCG | 22 | 18242 |
| HSV1-UL54-1254 | - | GACGCCGUGGGCGUCGCAGGGGU | 23 | 18243 |
| HSV1-UL54-1255 | - | GGACGCCGUGGGCGUCGCAGGGGU | 24 | 18244 |

**Table 15B** provides exemplary targeting domains for knocking out the *UL54* gene selected according to the second tier parameters. The targeting domains bind within the first 500 bp of the coding sequence (e.g., within 500 bp downstream from the start codon), and have a high level of orthogonality. The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15B**

| 2nd Tier | | | | |
|---|---|---|---|---|
| gRNA Name | DNA Strand | Targeting Domain | Target Site Length | Seq ID NO: |
| HSV1-UL54-1256 | + | AGGCGUCUGGGUGCUGGGUACGC | 23 | 18245 |
| HSV1-UL54-1257 | + | UUCUGGGGGCACGCCGGC | 18 | 18246 |
| HSV1-UL54-1258 | + | UGGUUCUGGGGGCACGCCGGC | 21 | 18247 |
| HSV1-UL54-1259 | + | UUGGUUCUGGGGGCACGCCGGC | 22 | 18248 |
| HSV1-UL54-1260 | + | AUUGGUUCUGGGGGCACGCCGGC | 23 | 18249 |
| HSV1-UL54-1261 | + | ACCGCCGGGCGAGCGGCG | 18 | 18250 |
| HSV1-UL54-1262 | + | CGGACCGCCGGGCGAGCGGCG | 21 | 18251 |
| HSV1-UL54-1263 | + | CGGCUCUCCGCCGGCUCGG | 19 | 18252 |
| HSV1-UL54-1264 | + | CGGCGGCUCUCCGCCGGCUCGG | 22 | 18253 |
| HSV1-UL54-1265 | + | CGCGGCGGCUCUCCGCCGGCUCGG | 24 | 18254 |
| HSV1-UL54-1266 | + | CGGACGAGGCGUCUGGGUG | 19 | 18255 |
| HSV1-UL54-788 | + | UCGGACGAGGCGUCUGGGUG | 20 | 7531 |
| HSV1-UL54-1267 | + | CGUCGGACGAGGCGUCUGGGUG | 22 | 18256 |
| HSV1-UL54-1268 | + | CCGUCGGACGAGGCGUCUGGGUG | 23 | 18257 |
| HSV1-UL54-1269 | + | UCCGUCGGACGAGGCGUCUGGGUG | 24 | 18258 |
| HSV1-UL54-1270 | + | CCGGUCCGUCCUCUCCGU | 18 | 18259 |
| HSV1-UL54-1271 | + | UCCGGUCCGUCCUCUCCGU | 19 | 18260 |
| HSV1-UL54-158 | + | CUCCGGUCCGUCCUCUCCGU | 20 | 6921 |
| HSV1-UL54-1272 | + | CGGCUCCGGUCCGUCCUCUCCGU | 23 | 18261 |
| HSV1-UL54-1273 | + | UCGGCUCCGGUCCGUCCUCUCCGU | 24 | 18262 |
| HSV1-UL54-1274 | + | CUCCGUCGGACGAGGCGU | 18 | 18263 |
| HSV1-UL54-1275 | + | CGCUCCGUCGGACGAGGCGU | 20 | 18264 |
| HSV1-UL54-1276 | + | CCGCUCCGUCGGACGAGGCGU | 21 | 18265 |
| HSV1-UL54-1277 | + | UGCCGCUCCGUCGGACGAGGCGU | 23 | 18266 |
| HSV1-UL54-1278 | + | CUGCCGCUCCGUCGGACGAGGCGU | 24 | 18267 |
| HSV1-UL54-1279 | + | AGGCUGGGCUUUGGUCGGU | 19 | 18268 |
| HSV1-UL54-128 | + | CAGGCUGGGCUUUGGUCGGU | 20 | 6891 |
| HSV1-UL54-1280 | + | CGGGCAGGCUGGGCUUUGGUCGGU | 24 | 18269 |
| HSV1-UL54-1281 | + | ACACACUGUGGGGCGCUGGUU | 21 | 18270 |
| HSV1-UL54-1282 | + | CACACACUGUGGGGCGCUGGUU | 22 | 18271 |
| HSV1-UL54-1283 | + | CCACACACUGUGGGGCGCUGGUU | 23 | 18272 |
| HSV1-UL54-1284 | + | AC CACACACUGUGGGGC GCUGGUU | 24 | 18273 |
| HSV1-UL54-1285 | - | CGGCGGACGCCGUGGGCGUCGC | 22 | 18274 |
| HSV1-UL54-1286 | - | CGCGGCGGACGCCGUGGGCGUCGC | 24 | 18275 |
| HSV1-UL54-1287 | - | UGGAAUCGGACAGCAGCG | 18 | 18276 |
| HSV1-UL54-1288 | - | CUGGAAUCGGACAGCAGCG | 19 | 18277 |
| HSV1-UL54-88 | - | CCUGGAAUCGGACAGCAGCG | 20 | 6851 |
| HSV1-UL54-1289 | - | ACCUGGAAUCGGACAGCAGCG | 21 | 18278 |
| HSV1-UL54-1290 | - | CGACCUGGAAUCGGACAGCAGCG | 23 | 18279 |
| HSV1-UL54-1291 | - | ACGACCUGGAAUCGGACAGCAGCG | 24 | 18280 |
| HSV1-UL54-1292 | - | CGCCGUGGGCGUCGCAGGGGU | 21 | 18281 |
| HSV1-UL54-1293 | - | ACGCCGUGGGCGUCGCAGGGGU | 22 | 18282 |

**Table 15C** provides exemplary targeting domains for knocking out the *UL54* gene selected according to the third tier parameters. The targeting domains bind within the first 500 bp of the coding sequence (e.g., within 500 bp downstream from the start codon). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15C**

| 3rd Tier | | | | |
|---|---|---|---|---|
| gRNA Name | DNA Strand | Targeting Domain | Target Site Length | Seq ID NO: |
| HSV1-UL54-1294 | + | UCUGGGUGCUGGGUACGC | 18 | 18283 |
| HSV1-UL54-1295 | + | GUCUGGGUGCUGGGUACGC | 19 | 18284 |
| HSV1-UL54-147 | + | CGUCUGGGUGCUGGGUACGC | 20 | 6910 |
| HSV1-UL54-1296 | + | CACUGUGGGGCGCUGGUU | 18 | 18285 |
| HSV1-UL54-1297 | + | ACACUGUGGGGCGCUGGUU | 19 | 18286 |
| HSV1-UL54-1298 | + | CACACUGUGGGGCGCUGGUU | 20 | 18287 |
| HSV1-UL54-1299 | - | AGAGCCGCCGCGACGACC | 18 | 18288 |
| HSV1-UL54-1300 | - | GAGAGCCGCCGCGACGACC | 19 | 18289 |
| HSV1-UL54-85 | - | GGAGAGCCGCCGCGACGACC | 20 | 6848 |
| HSV1-UL54-1301 | - | CGGAGAGCCGCCGCGACGACC | 21 | 18290 |
| HSV1-UL54-1302 | - | GCGGAGAGCCGCCGCGACGACC | 22 | 18291 |
| HSV1-UL54-1303 | - | GGCGGAGAGCCGCCGCGACGACC | 23 | 18292 |
| HSV1-UL54-1304 | - | CGGCGGAGAGCCGCCGCGACGACC | 24 | 18293 |
| HSV1-UL54-1305 | - | GGACGCCGUGGGCGUCGC | 18 | 18294 |
| HSV1-UL54-1306 | - | CGGACGCCGUGGGCGUCGC | 19 | 18295 |
| HSV1-UL54-108 | - | GCGGACGCCGUGGGCGUCGC | 20 | 6871 |
| HSV1-UL54-1307 | - | CGUGGGCGUCGCAGGGGU | 18 | 18296 |
| HSV1-UL54-1308 | - | CCGUGGGCGUCGCAGGGGU | 19 | 18297 |
| HSV1-UL54-111 | - | GCCGUGGGCGUCGCAGGGGU | 20 | 6874 |

**Table 15D** provides exemplary targeting domains for knocking out the *UL54* gene selected according to the fourth tier parameters. The targeting domains bind within the first 500 bp of the coding sequence (e.g., within 500 bp downstream from the start codon). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15D**

| 4th Tier | | | | |
|---|---|---|---|---|
| gRNA Name | DNA Strand | Targeting Domain | Target Site Length | Seq ID NO: |
| HSV1-UL54-1309 | + | CAAUGUCAGUCGCCAUGA | 18 | 18298 |
| HSV1-UL54-1310 | + | UCAAUGUCAGUCGCCAUGA | 19 | 18299 |
| HSV1-UL54-1311 | + | AUCAAUGUCAGUCGCCAUGA | 20 | 18300 |
| HSV1-UL54-1312 | + | UAUCAAUGUCAGUCGCCAUGA | 21 | 18301 |
| HSV1-UL54-1313 | + | AUAUCAAUGUCAGUCGCCAUGA | 22 | 18302 |
| HSV1-UL54-1314 | + | CAUAUCAAUGUCAGUCGCCAUGA | 23 | 18303 |
| HSV1-UL54-1315 | + | GCAUAUCAAUGUCAGUCGCCAUGA | 24 | 18304 |
| HSV1-UL54-1316 | + | UCCAUGUCCUCGUCCGAC | 18 | 18305 |
| HSV1-UL54-1317 | + | UUCCAUGUCCUCGUCCGAC | 19 | 18306 |
| HSV1-UL54-1318 | + | CUUCCAUGUCCUCGUCCGAC | 20 | 18307 |
| HSV1-UL54-1319 | + | UCUUCCAUGUCCUCGUCCGAC | 21 | 18308 |
| HSV1-UL54-1320 | + | GUCUUCCAUGUCCUCGUCCGAC | 22 | 18309 |
| HSV1-UL54-1321 | + | GGUCUUCCAUGUCCUCGUCCGAC | 23 | 18310 |
| HSV1-UL54-1322 | + | GGGUCUUCCAUGUCCUCGUCCGAC | 24 | 18311 |
| HSV1-UL54-1323 | + | ACAAACCAUCGGCAGCCC | 18 | 18312 |
| HSV1-UL54-1324 | + | GACAAACCAUCGGCAGCCC | 19 | 18313 |
| HSV1-UL54-18 | + | CGACAAACCAUCGGCAGCCC | 20 | 6781 |
| HSV1-UL54-1325 | + | CCGACAAACCAUCGGCAGCCC | 21 | 18314 |
| HSV1-UL54-1326 | + | UCCGACAAACCAUCGGCAGCCC | 22 | 18315 |
| HSV1-UL54-1327 | + | GUCCGACAAACCAUCGGCAGCCC | 23 | 18316 |
| HSV1-UL54-1328 | + | GGUCCGACAAACCAUCGGCAGCCC | 24 | 18317 |
| HSV1-UL54-1329 | + | GACAAACCAUCGGCAGCC | 18 | 18318 |
| HSV1-UL54-1330 | + | CGACAAACCAUCGGCAGCC | 19 | 18319 |
| HSV1-UL54-1331 | + | CCGACAAACCAUCGGCAGCC | 20 | 18320 |
| HSV1-UL54-1332 | + | UCCGACAAACCAUCGGCAGCC | 21 | 18321 |
| HSV1-UL54-1333 | + | GUCCGACAAACCAUCGGCAGCC | 22 | 18322 |
| HSV1-UL54-1334 | + | GGUCCGACAAACCAUCGGCAGCC | 23 | 18323 |
| HSV1-UL54-1335 | + | GGGUCCGACAAACCAUCGGCAGCC | 24 | 18324 |
| HSV1-UL54-1336 | + | ACGCCCACGGCGUCCGCC | 18 | 18325 |
| HSV1-UL54-1337 | + | GACGCCCACGGCGUCCGCC | 19 | 18326 |
| HSV1-UL54-780 | + | CGACGCCCACGGCGUCCGCC | 20 | 7525 |
| HSV1-UL54-1338 | + | GCGACGCCCACGGCGUCCGCC | 21 | 18327 |
| HSV1-UL54-1339 | + | UGCGACGCCCACGGCGUCCGCC | 22 | 18328 |
| HSV1-UL54-1340 | + | CUGCGACGCCCACGGCGUCCGCC | 23 | 18329 |
| HSV1-UL54-1341 | + | CCUGCGACGCCCACGGCGUCCGCC | 24 | 18330 |
| HSV1-UL54-1342 | + | CAAGACGGUCGCCGGGCC | 18 | 18331 |
| HSV1-UL54-1343 | + | GCAAGACGGUCGCCGGGCC | 19 | 18332 |
| HSV1-UL54-1344 | + | AGCAAGACGGUCGCCGGGCC | 20 | 18333 |
| HSV1-UL54-1345 | + | GAGCAAGACGGUCGCCGGGCC | 21 | 18334 |
| HSV1-UL54-1346 | + | GGAGCAAGACGGUCGCCGGGCC | 22 | 18335 |
| HSV1-UL54-1347 | + | GGGAGCAAGACGGUCGCCGGGCC | 23 | 18336 |
| HSV1-UL54-1348 | + | GGGGAGCAAGACGGUCGCCGGGCC | 24 | 18337 |
| HSV1-UL54-1349 | + | CUCCGGUCCGUCCUCUCC | 18 | 18338 |
| HSV1-UL54-1350 | + | GCUCCGGUCCGUCCUCUCC | 19 | 18339 |
| HSV1-UL54-1351 | + | GGCUCCGGUCCGUCCUCUCC | 20 | 18340 |
| HSV1-UL54-1352 | + | CGGCUCCGGUCCGUCCUCUCC | 21 | 18341 |
| HSV1-UL54-1353 | + | UCGGCUCCGGUCCGUCCUCUCC | 22 | 18342 |
| HSV1-UL54-1354 | + | AUCGGCUCCGGUCCGUCCUCUCC | 23 | 18343 |
| HSV1-UL54-1355 | + | UAUCGGCUCCGGUCCGUCCUCUCC | 24 | 18344 |
| HSV1-UL54-1356 | + | CGGCGGCUCUCCGCCGGC | 18 | 18345 |
| HSV1-UL54-1357 | + | GCGGCGGCUCUCCGCCGGC | 19 | 18346 |
| HSV1-UL54-1358 | + | CGCGGCGGCUCUCCGCCGGC | 20 | 18347 |
| HSV1-UL54-1359 | + | UCGCGGCGGCUCUCCGCCGGC | 21 | 18348 |
| HSV1-UL54-1360 | + | GUCGCGGCGGCUCUCCGCCGGC | 22 | 18349 |
| HSV1-UL54-1361 | + | CGUCGCGGCGGCUCUCCGCCGGC | 23 | 18350 |
| HSV1-UL54-1362 | + | UCGUCGCGGCGGCUCUCCGCCGGC | 24 | 18351 |
| HSV1-UL54-1363 | + | ACCUUGCCCCCGUGCUGC | 18 | 18352 |
| HSV1-UL54-1364 | + | CACCUUGCCCCCGUGCUGC | 19 | 18353 |
| HSV1-UL54-1365 | + | CCACCUUGCCCCCGUGCUGC | 20 | 18354 |
| HSV1-UL54-1366 | + | GCCACCUUGCCCCCGUGCUGC | 21 | 18355 |
| HSV1-UL54-1367 | + | GGCCACCUUGCCCCCGUGCUGC | 22 | 18356 |
| HSV1-UL54-1368 | + | GGGCCACCUUGCCCCCGUGCUGC | 23 | 18357 |
| HSV1-UL54-1369 | + | CGGGCCACCUUGCCCCCGUGCUGC | 24 | 18358 |
| HSV1-UL54-1370 | + | GCGGCUCUCCGCCGGCUC | 18 | 18359 |
| HSV1-UL54-1371 | + | GGCGGCUCUCCGCCGGCUC | 19 | 18360 |
| HSV1-UL54-163 | + | CGGCGGCUCUCCGCCGGCUC | 20 | 6926 |
| HSV1-UL54-1372 | + | GCGGCGGCUCUCCGCCGGCUC | 21 | 18361 |
| HSV1-UL54-1373 | + | CGCGGCGGCUCUCCGCCGGCUC | 22 | 18362 |
| HSV1-UL54-1374 | + | UCGCGGCGGCUCUCCGCCGGCUC | 23 | 18363 |
| HSV1-UL54-1375 | + | GUCGCGGCGGCUCUCCGCCGGCUC | 24 | 18364 |
| HSV1-UL54-1376 | + | CUUGCCCCCGUGCUGCUC | 18 | 18365 |
| HSV1-UL54-1377 | + | CCUUGCCCCCGUGCUGCUC | 19 | 18366 |
| HSV1-UL54-136 | + | ACCUUGCCCCCGUGCUGCUC | 20 | 6899 |
| HSV1-UL54-1378 | + | CACCUUGCCCCCGUGCUGCUC | 21 | 18367 |
| HSV1-UL54-1379 | + | CCACCUUGCCCCCGUGCUGCUC | 22 | 18368 |
| HSV1-UL54-1380 | + | GCCACCUUGCCCCCGUGCUGCUC | 23 | 18369 |
| HSV1-UL54-1381 | + | GGCCACCUUGCCCCCGUGCUGCUC | 24 | 18370 |
| HSV1-UL54-1382 | + | CAGGGUUGCGAUUGGUUC | 18 | 18371 |
| HSV1-UL54-1383 | + | CCAGGGUUGCGAUUGGUUC | 19 | 18372 |
| HSV1-UL54-1199 | + | CCCAGGGUUGCGAUUGGUUC | 20 | 18188 |
| HSV1-UL54-1384 | + | CCCCAGGGUUGCGAUUGGUUC | 21 | 18373 |
| HSV1-UL54-1385 | + | CCCCCAGGGUUGCGAUUGGUUC | 22 | 18374 |
| HSV1-UL54-1386 | + | UCCCCCAGGGUUGCGAUUGGUUC | 23 | 18375 |
| HSV1-UL54-1387 | + | GUCCCCCAGGGUUGCGAUUGGUUC | 24 | 18376 |
| HSV1-UL54-1388 | + | CGGGGUCUUCUGGACGAG | 18 | 18377 |
| HSV1-UL54-1389 | + | CCGGGGUCUUCUGGACGAG | 19 | 18378 |
| HSV1-UL54-1390 | + | GCCGGGGUCUUCUGGACGAG | 20 | 18379 |
| HSV1-UL54-1391 | + | CGCCGGGGUCUUCUGGACGAG | 21 | 18380 |
| HSV1-UL54-1392 | + | ACGCCGGGGUCUUCUGGACGAG | 22 | 18381 |
| HSV1-UL54-1393 | + | UACGCCGGGGUCUUCUGGACGAG | 23 | 18382 |
| HSV1-UL54-1394 | + | GUACGCCGGGGUCUUCUGGACGAG | 24 | 18383 |
| HSV1-UL54-1395 | + | GGUCGGUGGGGGUUGGAG | 18 | 18384 |
| HSV1-UL54-1396 | + | UGGUCGGUGGGGGUUGGAG | 19 | 18385 |
| HSV1-UL54-1397 | + | UUGGUCGGUGGGGGUUGGAG | 20 | 18386 |
| HSV1-UL54-1398 | + | UUUGGUCGGUGGGGGUUGGAG | 21 | 18387 |
| HSV1-UL54-1399 | + | CUUUGGUCGGUGGGGGUUGGAG | 22 | 18388 |
| HSV1-UL54-1400 | + | GCUUUGGUCGGUGGGGGUUGGAG | 23 | 18389 |
| HSV1-UL54-1401 | + | GGCUUUGGUCGGUGGGGGUUGGAG | 24 | 18390 |
| HSV1-UL54-1402 | + | CCAUGUCCUCGUCCGACG | 18 | 18391 |
| HSV1-UL54-1403 | + | UCCAUGUCCUCGUCCGACG | 19 | 18392 |
| HSV1-UL54-33 | + | UUCCAUGUCCUCGUCCGACG | 20 | 6796 |
| HSV1-UL54-1404 | + | CUUCCAUGUCCUCGUCCGACG | 21 | 18393 |
| HSV1-UL54-1405 | + | UCUUCCAUGUCCUCGUCCGACG | 22 | 18394 |
| HSV1-UL54-1406 | + | GUCUUCCAUGUCCUCGUCCGACG | 23 | 18395 |
| HSV1-UL54-1407 | + | GGUCUUCCAUGUCCUCGUCCGACG | 24 | 18396 |
| HSV1-UL54-1408 | + | GUCUGGGUGCUGGGUACG | 18 | 18397 |
| HSV1-UL54-1409 | + | CGUCUGGGUGCUGGGUACG | 19 | 18398 |
| HSV1-UL54-1410 | + | GCGUCUGGGUGCUGGGUACG | 20 | 18399 |
| HSV1-UL54-1411 | + | GGCGUCUGGGUGCUGGGUACG | 21 | 18400 |
| HSV1-UL54-1412 | + | AGGCGUCUGGGUGCUGGGUACG | 22 | 18401 |
| HSV1-UL54-1413 | + | GAGGCGUCUGGGUGCUGGGUACG | 23 | 18402 |
| HSV1-UL54-1414 | + | CGAGGCGUCUGGGUGCUGGGUACG | 24 | 18403 |
| HSV1-UL54-1415 | + | GACGAGACGGGCGGACCG | 18 | 18404 |
| HSV1-UL54-1416 | + | GGACGAGACGGGCGGACCG | 19 | 18405 |
| HSV1-UL54-1417 | + | UGGACGAGACGGGCGGACCG | 20 | 18406 |
| HSV1-UL54-1418 | + | CUGGACGAGACGGGCGGACCG | 21 | 18407 |
| HSV1-UL54-1419 | + | UCUGGACGAGACGGGCGGACCG | 22 | 18408 |
| HSV1-UL54-1420 | + | UUCUGGACGAGACGGGCGGACCG | 23 | 18409 |
| HSV1-UL54-1421 | + | CUUCUGGACGAGACGGGCGGACCG | 24 | 18410 |
| HSV1-UL54-1422 | + | GGUUCUGGGGGCACGCCG | 18 | 18411 |
| HSV1-UL54-1423 | + | UGGUUCUGGGGGCACGCCG | 19 | 18412 |
| HSV1-UL54-1424 | + | UUGGUUCUGGGGGCACGCCG | 20 | 18413 |
| HSV1-UL54-1425 | + | AUUGGUUCUGGGGGCACGCCG | 21 | 18414 |
| HSV1-UL54-1426 | + | GAUUGGUUCUGGGGGCACGCCG | 22 | 18415 |
| HSV1-UL54-1427 | + | CGAUUGGUUCUGGGGGCACGCCG | 23 | 18416 |
| HSV1-UL54-1428 | + | GCGAUUGGUUCUGGGGGCACGCCG | 24 | 18417 |
| HSV1-UL54-1429 | + | CCGGGCUGUCGGCUGCCG | 18 | 18418 |
| HSV1-UL54-1430 | + | ACCGGGCUGUCGGCUGCCG | 19 | 18419 |
| HSV1-UL54-1431 | + | GACCGGGCUGUCGGCUGCCG | 20 | 18420 |
| HSV1-UL54-1432 | + | UGACCGGGCUGUCGGCUGCCG | 21 | 18421 |
| HSV1-UL54-1433 | + | AUGACCGGGCUGUCGGCUGCCG | 22 | 18422 |
| HSV1-UL54-1434 | + | CAUGACCGGGCUGUCGGCUGCCG | 23 | 18423 |
| HSV1-UL54-1435 | + | CCAUGACCGGGCUGUCGGCUGCCG | 24 | 18424 |
| HSV1-UL54-1436 | + | UGGGGCCCUGCCGCUCCG | 18 | 18425 |
| HSV1-UL54-1437 | + | UUGGGGCCCUGCCGCUCCG | 19 | 18426 |
| HSV1-UL54-1438 | + | GUUGGGGCCCUGCCGCUCCG | 20 | 18427 |
| HSV1-UL54-1439 | + | CGUUGGGGCCCUGCCGCUCCG | 21 | 18428 |
| HSV1-UL54-1440 | + | UCGUUGGGGCCCUGCCGCUCCG | 22 | 18429 |
| HSV1-UL54-1441 | + | AUCGUUGGGGCCCUGCCGCUCCG | 23 | 18430 |
| HSV1-UL54-1442 | + | GAUCGUUGGGGCCCUGCCGCUCCG | 24 | 18431 |
| HSV1-UL54-1443 | + | UCCGGUCCGUCCUCUCCG | 18 | 18432 |
| HSV1-UL54-1444 | + | CUCCGGUCCGUCCUCUCCG | 19 | 18433 |
| HSV1-UL54-157 | + | GCUCCGGUCCGUCCUCUCCG | 20 | 6920 |
| HSV1-UL54-1445 | + | GGCUCCGGUCCGUCCUCUCCG | 21 | 18434 |
| HSV1-UL54-1446 | + | CGGCUCCGGUCCGUCCUCUCCG | 22 | 18435 |
| HSV1-UL54-1447 | + | UCGGCUCCGGUCCGUCCUCUCCG | 23 | 18436 |
| HSV1-UL54-1448 | + | AUCGGCUCCGGUCCGUCCUCUCCG | 24 | 18437 |
| HSV1-UL54-1449 | + | CUUCCAUGUCCUCGUCCG | 18 | 18438 |
| HSV1-UL54-1450 | + | UCUUCCAUGUCCUCGUCCG | 19 | 18439 |
| HSV1-UL54-1451 | + | GUCUUCCAUGUCCUCGUCCG | 20 | 18440 |
| HSV1-UL54-1452 | + | GGUCUUCCAUGUCCUCGUCCG | 21 | 18441 |
| HSV1-UL54-1453 | + | GGGUCUUCCAUGUCCUCGUCCG | 22 | 18442 |
| HSV1-UL54-1454 | + | GGGGUCUUCCAUGUCCUCGUCCG | 23 | 18443 |
| HSV1-UL54-1455 | + | GGGGGUCUUCCAUGUCCUCGUCCG | 24 | 18444 |
| HSV1-UL54-1456 | + | GGCGCUGGUUGAGGAUCG | 18 | 18445 |
| HSV1-UL54-1457 | + | GGGCGCUGGUUGAGGAUCG | 19 | 18446 |
| HSV1-UL54-1458 | + | GGGGCGCUGGUUGAGGAUCG | 20 | 18447 |
| HSV1-UL54-1459 | + | UGGGGCGCUGGUUGAGGAUCG | 21 | 18448 |
| HSV1-UL54-1460 | + | GUGGGGCGCUGGUUGAGGAUCG | 22 | 18449 |
| HSV1-UL54-1461 | + | UGUGGGGCGCUGGUUGAGGAUCG | 23 | 18450 |
| HSV1-UL54-1462 | + | CUGUGGGGCGCUGGUUGAGGAUCG | 24 | 18451 |
| HSV1-UL54-1463 | + | CGGCUCUCCGCCGGCUCG | 18 | 18452 |
| HSV1-UL54-1464 | + | GCGGCUCUCCGCCGGCUCG | 19 | 18453 |
| HSV1-UL54-164 | + | GGCGGCUCUCCGCCGGCUCG | 20 | 6927 |
| HSV1-UL54-1465 | + | CGGCGGCUCUCCGCCGGCUCG | 21 | 18454 |
| HSV1-UL54-1466 | + | GCGGCGGCUCUCCGCCGGCUCG | 22 | 18455 |
| HSV1-UL54-1467 | + | CGCGGCGGCUCUCCGCCGGCUCG | 23 | 18456 |
| HSV1-UL54-1468 | + | UCGCGGCGGCUCUCCGCCGGCUCG | 24 | 18457 |
| HSV1-UL54-1469 | + | UUGCCCCCGUGCUGCUCG | 18 | 18458 |
| HSV1-UL54-1470 | + | CUUGCCCCCGUGCUGCUCG | 19 | 18459 |
| HSV1-UL54-20 | + | CCUUGCCCCCGUGCUGCUCG | 20 | 6783 |
| HSV1-UL54-1471 | + | ACCUUGCCCCCGUGCUGCUCG | 21 | 18460 |
| HSV1-UL54-1472 | + | CACCUUGCCCCCGUGCUGCUCG | 22 | 18461 |
| HSV1-UL54-1473 | + | CCACCUUGCCCCCGUGCUGCUCG | 23 | 18462 |
| HSV1-UL54-1474 | + | GCCACCUUGCCCCCGUGCUGCUCG | 24 | 18463 |
| HSV1-UL54-1475 | + | GGGGGAGCAAGACGGUCG | 18 | 18464 |
| HSV1-UL54-1476 | + | CGGGGGAGCAAGACGGUCG | 19 | 18465 |
| HSV1-UL54-1477 | + | UCGGGGGAGCAAGACGGUCG | 20 | 18466 |
| HSV1-UL54-1478 | + | CUCGGGGGAGCAAGACGGUCG | 21 | 18467 |
| HSV1-UL54-1479 | + | GCUCGGGGGAGCAAGACGGUCG | 22 | 18468 |
| HSV1-UL54-1480 | + | UGCUCGGGGGAGCAAGACGGUCG | 23 | 18469 |
| HSV1-UL54-1481 | + | CUGCUCGGGGGAGCAAGACGGUCG | 24 | 18470 |
| HSV1-UL54-1482 | + | CAGGCUGGGCUUUGGUCG | 18 | 18471 |
| HSV1-UL54-1483 | + | GCAGGCUGGGCUUUGGUCG | 19 | 18472 |
| HSV1-UL54-1484 | + | GGCAGGCUGGGCUUUGGUCG | 20 | 18473 |
| HSV1-UL54-1485 | + | GGGCAGGCUGGGCUUUGGUCG | 21 | 18474 |
| HSV1-UL54-1486 | + | CGGGCAGGCUGGGCUUUGGUCG | 22 | 18475 |
| HSV1-UL54-1487 | + | GCGGGCAGGCUGGGCUUUGGUCG | 23 | 18476 |
| HSV1-UL54-1488 | + | CGCGGGCAGGCUGGGCUUUGGUCG | 24 | 18477 |
| HSV1-UL54-1489 | + | UCGUCCAGAUCGCUGUCG | 18 | 18478 |
| HSV1-UL54-1490 | + | CUCGUCCAGAUCGCUGUCG | 19 | 18479 |
| HSV1-UL54-1491 | + | CCUCGUCCAGAUCGCUGUCG | 20 | 18480 |
| HSV1-UL54-1492 | + | UCCUCGUCCAGAUCGCUGUCG | 21 | 18481 |
| HSV1-UL54-1493 | + | GUCCUCGUCCAGAUCGCUGUCG | 22 | 18482 |
| HSV1-UL54-1494 | + | GGUCCUCGUCCAGAUCGCUGUCG | 23 | 18483 |
| HSV1-UL54-1495 | + | GGGUCCUCGUCCAGAUCGCUGUCG | 24 | 18484 |
| HSV1-UL54-1496 | + | CUGUCGGAGAGGUCCAGG | 18 | 18485 |
| HSV1-UL54-1497 | + | GCUGUCGGAGAGGUCCAGG | 19 | 18486 |
| HSV1-UL54-1498 | + | CGCUGUCGGAGAGGUCCAGG | 20 | 18487 |
| HSV1-UL54-1499 | + | UCGCUGUCGGAGAGGUCCAGG | 21 | 18488 |
| HSV1-UL54-1500 | + | AUCGCUGUCGGAGAGGUCCAGG | 22 | 18489 |
| HSV1-UL54-1501 | + | GAUCGCUGUCGGAGAGGUCCAGG | 23 | 18490 |
| HSV1-UL54-1502 | + | AGAUCGCUGUCGGAGAGGUCCAGG | 24 | 18491 |
| HSV1-UL54-1503 | + | GCGUCCGCCGCGGGCAGG | 18 | 18492 |
| HSV1-UL54-1504 | + | GGCGUCCGCCGCGGGCAGG | 19 | 18493 |
| HSV1-UL54-1505 | + | CGGCGUCCGCCGCGGGCAGG | 20 | 18494 |
| HSV1-UL54-1506 | + | ACGGCGUCCGCCGCGGGCAGG | 21 | 18495 |
| HSV1-UL54-1507 | + | CACGGCGUCCGCCGCGGGCAGG | 22 | 18496 |
| HSV1-UL54-1508 | + | CCACGGCGUCCGCCGCGGGCAGG | 23 | 18497 |
| HSV1-UL54-1509 | + | CCCACGGCGUCCGCCGCGGGCAGG | 24 | 18498 |
| HSV1-UL54-1510 | + | GUCUUCUGGACGAGACGG | 18 | 18499 |
| HSV1-UL54-1511 | + | GGUCUUCUGGACGAGACGG | 19 | 18500 |
| HSV1-UL54-1512 | + | GGGUCUUCUGGACGAGACGG | 20 | 18501 |
| HSV1-UL54-1513 | + | GGGGUCUUCUGGACGAGACGG | 21 | 18502 |
| HSV1-UL54-1514 | + | CGGGGUCUUCUGGACGAGACGG | 22 | 18503 |
| HSV1-UL54-1515 | + | CCGGGGUCUUCUGGACGAGACGG | 23 | 18504 |
| HSV1-UL54-1516 | + | GCCGGGGUCUUCUGGACGAGACGG | 24 | 18505 |
| HSV1-UL54-1517 | + | GUUCUGGGGGCACGCCGG | 18 | 18506 |
| HSV1-UL54-1518 | + | GGUUCUGGGGGCACGCCGG | 19 | 18507 |
| HSV1-UL54-1201 | + | UGGUUCUGGGGGCACGCCGG | 20 | 18190 |
| HSV1-UL54-1519 | + | UUGGUUCUGGGGGCACGCCGG | 21 | 18508 |
| HSV1-UL54-1520 | + | AUUGGUUCUGGGGGCACGCCGG | 22 | 18509 |
| HSV1-UL54-1521 | + | GAUUGGUUCUGGGGGCACGCCGG | 23 | 18510 |
| HSV1-UL54-1522 | + | CGAUUGGUUCUGGGGGCACGCCGG | 24 | 18511 |
| HSV1-UL54-1523 | + | AGACGGGCGGACCGCCGG | 18 | 18512 |
| HSV1-UL54-1524 | + | GAGACGGGCGGACCGCCGG | 19 | 18513 |
| HSV1-UL54-1525 | + | CGAGACGGGCGGACCGCCGG | 20 | 18514 |
| HSV1-UL54-1526 | + | ACGAGACGGGCGGACCGCCGG | 21 | 18515 |
| HSV1-UL54-1527 | + | GACGAGACGGGCGGACCGCCGG | 22 | 18516 |
| HSV1-UL54-1528 | + | GGACGAGACGGGCGGACCGCCGG | 23 | 18517 |
| HSV1-UL54-1529 | + | UGGACGAGACGGGCGGACCGCCGG | 24 | 18518 |
| HSV1-UL54-1530 | + | UGCCCCCGUGCUGCUCGG | 18 | 18519 |
| HSV1-UL54-1531 | + | UUGCCCCCGUGCUGCUCGG | 19 | 18520 |
| HSV1-UL54-21 | + | CUUGCCCCCGUGCUGCUCGG | 20 | 6784 |
| HSV1-UL54-1532 | + | CCUUGCCCCCGUGCUGCUCGG | 21 | 18521 |
| HSV1-UL54-1533 | + | ACCUUGCCCCCGUGCUGCUCGG | 22 | 18522 |
| HSV1-UL54-1534 | + | CACCUUGCCCCCGUGCUGCUCGG | 23 | 18523 |
| HSV1-UL54-1535 | + | CCACCUUGCCCCCGUGCUGCUCGG | 24 | 18524 |
| HSV1-UL54-1536 | + | GCCCUGCCGCUCCGUCGG | 18 | 18525 |
| HSV1-UL54-1537 | + | GGCCCUGCCGCUCCGUCGG | 19 | 18526 |
| HSV1-UL54-1538 | + | GGGCCCUGCCGCUCCGUCGG | 20 | 18527 |
| HSV1-UL54-1539 | + | GGGGCCCUGCCGCUCCGUCGG | 21 | 18528 |
| HSV1-UL54-1540 | + | UGGGGCCCUGCCGCUCCGUCGG | 22 | 18529 |
| HSV1-UL54-1541 | + | UUGGGGCCCUGCCGCUCCGUCGG | 23 | 18530 |
| HSV1-UL54-1542 | + | GUUGGGGCCCUGCCGCUCCGUCGG | 24 | 18531 |
| HSV1-UL54-1543 | + | AGGCUGGGCUUUGGUCGG | 18 | 18532 |
| HSV1-UL54-1544 | + | CAGGCUGGGCUUUGGUCGG | 19 | 18533 |
| HSV1-UL54-127 | + | GCAGGCUGGGCUUUGGUCGG | 20 | 6890 |
| HSV1-UL54-1545 | + | GGCAGGCUGGGCUUUGGUCGG | 21 | 18534 |
| HSV1-UL54-1546 | + | GGGCAGGCUGGGCUUUGGUCGG | 22 | 18535 |
| HSV1-UL54-1547 | + | CGGGCAGGCUGGGCUUUGGUCGG | 23 | 18536 |
| HSV1-UL54-1548 | + | GCGGGCAGGCUGGGCUUUGGUCGG | 24 | 18537 |
| HSV1-UL54-1549 | + | GGCUUUGGUCGGUGGGGG | 18 | 18538 |
| HSV1-UL54-1550 | + | GGGCUUUGGUCGGUGGGGG | 19 | 18539 |
| HSV1-UL54-1551 | + | UGGGCUUUGGUCGGUGGGGG | 20 | 18540 |
| HSV1-UL54-1552 | + | CUGGGCUUUGGUCGGUGGGGG | 21 | 18541 |
| HSV1-UL54-1553 | + | GCUGGGCUUUGGUCGGUGGGGG | 22 | 18542 |
| HSV1-UL54-1554 | + | GGCUGGGCUUUGGUCGGUGGGGG | 23 | 18543 |
| HSV1-UL54-1555 | + | AGGCUGGGCUUUGGUCGGUGGGGG | 24 | 18544 |
| HSV1-UL54-1556 | + | CACACUGUGGGGCGCUGG | 18 | 18545 |
| HSV1-UL54-1557 | + | ACACACUGUGGGGCGCUGG | 19 | 18546 |
| HSV1-UL54-1558 | + | CACACACUGUGGGGCGCUGG | 20 | 18547 |
| HSV1-UL54-1559 | + | CCACACACUGUGGGGCGCUGG | 21 | 18548 |
| HSV1-UL54-1560 | + | ACCACACACUGUGGGGCGCUGG | 22 | 18549 |
| HSV1-UL54-1561 | + | GACCACACACUGUGGGGCGCUGG | 23 | 18550 |
| HSV1-UL54-1562 | + | CGACCACACACUGUGGGGCGCUGG | 24 | 18551 |
| HSV1-UL54-1563 | + | UACGCCGGGGUCUUCUGG | 18 | 18552 |
| HSV1-UL54-1564 | + | GUACGCCGGGGUCUUCUGG | 19 | 18553 |
| HSV1-UL54-1565 | + | GGUACGCCGGGGUCUUCUGG | 20 | 18554 |
| HSV1-UL54-1566 | + | GGGUACGCCGGGGUCUUCUGG | 21 | 18555 |
| HSV1-UL54-1567 | + | UGGGUACGCCGGGGUCUUCUGG | 22 | 18556 |
| HSV1-UL54-1568 | + | CUGGGUACGCCGGGGUCUUCUGG | 23 | 18557 |
| HSV1-UL54-1569 | + | GCUGGGUACGCCGGGGUCUUCUGG | 24 | 18558 |
| HSV1-UL54-1570 | + | AGGCGCGACCACACACUG | 18 | 18559 |
| HSV1-UL54-1571 | + | GAGGCGCGACCACACACUG | 19 | 18560 |
| HSV1-UL54-24 | + | CGAGGCGCGACCACACACUG | 20 | 6787 |
| HSV1-UL54-1572 | + | CCGAGGCGCGACCACACACUG | 21 | 18561 |
| HSV1-UL54-1573 | + | CCCGAGGCGCGACCACACACUG | 22 | 18562 |
| HSV1-UL54-1574 | + | CCCCGAGGCGCGACCACACACUG | 23 | 18563 |
| HSV1-UL54-1575 | + | GCCCCGAGGCGCGACCACACACUG | 24 | 18564 |
| HSV1-UL54-1576 | + | UCCUCGUCCAGAUCGCUG | 18 | 18565 |
| HSV1-UL54-1577 | + | GUCCUCGUCCAGAUCGCUG | 19 | 18566 |
| HSV1-UL54-1578 | + | GGUCCUCGUCCAGAUCGCUG | 20 | 18567 |
| HSV1-UL54-1579 | + | GGGUCCUCGUCCAGAUCGCUG | 21 | 18568 |
| HSV1-UL54-1580 | + | GGGGUCCUCGUCCAGAUCGCUG | 22 | 18569 |
| HSV1-UL54-1581 | + | GGGGGUCCUCGUCCAGAUCGCUG | 23 | 18570 |
| HSV1-UL54-1582 | + | GGGGGGUCCUCGUCCAGAUCGCUG | 24 | 18571 |
| HSV1-UL54-1583 | + | GAGGCGCGACCACACACU | 18 | 18572 |
| HSV1-UL54-1584 | + | CGAGGCGCGACCACACACU | 19 | 18573 |
| HSV1-UL54-1585 | + | CCGAGGCGCGACCACACACU | 20 | 18574 |
| HSV1-UL54-1586 | + | CCCGAGGCGCGACCACACACU | 21 | 18575 |
| HSV1-UL54-1587 | + | CCCCGAGGCGCGACCACACACU | 22 | 18576 |
| HSV1-UL54-1588 | + | GCCCCGAGGCGCGACCACACACU | 23 | 18577 |
| HSV1-UL54-1589 | + | GGCCCCGAGGCGCGACCACACACU | 24 | 18578 |
| HSV1-UL54-1590 | + | GGCGGCUCUCCGCCGGCU | 18 | 18579 |
| HSV1-UL54-1591 | + | CGGCGGCUCUCCGCCGGCU | 19 | 18580 |
| HSV1-UL54-162 | + | GCGGCGGCUCUCCGCCGGCU | 20 | 6925 |
| HSV1-UL54-1592 | + | CGCGGCGGCUCUCCGCCGGCU | 21 | 18581 |
| HSV1-UL54-1593 | + | UCGCGGCGGCUCUCCGCCGGCU | 22 | 18582 |
| HSV1-UL54-1594 | + | GUCGCGGCGGCUCUCCGCCGGCU | 23 | 18583 |
| HSV1-UL54-1595 | + | CGUCGCGGCGGCUCUCCGCCGGCU | 24 | 18584 |
| HSV1-UL54-1596 | + | CCUUGCCCCCGUGCUGCU | 18 | 18585 |
| HSV1-UL54-1597 | + | ACCUUGCCCCCGUGCUGCU | 19 | 18586 |
| HSV1-UL54-135 | + | CACCUUGCCCCCGUGCUGCU | 20 | 6898 |
| HSV1-UL54-1598 | + | CCACCUUGCCCCCGUGCUGCU | 21 | 18587 |
| HSV1-UL54-1599 | + | GCCACCUUGCCCCCGUGCUGCU | 22 | 18588 |
| HSV1-UL54-1600 | + | GGCCACCUUGCCCCCGUGCUGCU | 23 | 18589 |
| HSV1-UL54-1601 | + | GGGCCACCUUGCCCCCGUGCUGCU | 24 | 18590 |
| HSV1-UL54-1602 | + | AGGGUUGCGAUUGGUUCU | 18 | 18591 |
| HSV1-UL54-1603 | + | CAGGGUUGCGAUUGGUUCU | 19 | 18592 |
| HSV1-UL54-1221 | + | CCAGGGUUGCGAUUGGUUCU | 20 | 18210 |
| HSV1-UL54-1604 | + | CCCAGGGUUGCGAUUGGUUCU | 21 | 18593 |
| HSV1-UL54-1605 | + | CCCCAGGGUUGCGAUUGGUUCU | 22 | 18594 |
| HSV1-UL54-1606 | + | CCCCCAGGGUUGCGAUUGGUUCU | 23 | 18595 |
| HSV1-UL54-1607 | + | UCCCCCAGGGUUGCGAUUGGUUCU | 24 | 18596 |
| HSV1-UL54-1608 | + | CGGGCUGUCGGCUGCCGU | 18 | 18597 |
| HSV1-UL54-1609 | + | CCGGGCUGUCGGCUGCCGU | 19 | 18598 |
| HSV1-UL54-1205 | + | ACCGGGCUGUCGGCUGCCGU | 20 | 18194 |
| HSV1-UL54-1610 | + | GACCGGGCUGUCGGCUGCCGU | 21 | 18599 |
| HSV1-UL54-1611 | + | UGACCGGGCUGUCGGCUGCCGU | 22 | 18600 |
| HSV1-UL54-1612 | + | AUGACCGGGCUGUCGGCUGCCGU | 23 | 18601 |
| HSV1-UL54-1613 | + | CAUGACCGGGCUGUCGGCUGCCGU | 24 | 18602 |
| HSV1-UL54-1614 | + | GCGCUGGUUGAGGAUCGU | 18 | 18603 |
| HSV1-UL54-1615 | + | GGCGCUGGUUGAGGAUCGU | 19 | 18604 |
| HSV1-UL54-142 | + | GGGCGCUGGUUGAGGAUCGU | 20 | 6905 |
| HSV1-UL54-1616 | + | GGGGCGCUGGUUGAGGAUCGU | 21 | 18605 |
| HSV1-UL54-1617 | + | UGGGGCGCUGGUUGAGGAUCGU | 22 | 18606 |
| HSV1-UL54-1618 | + | GUGGGGCGCUGGUUGAGGAUCGU | 23 | 18607 |
| HSV1-UL54-1619 | + | UGUGGGGCGCUGGUUGAGGAUCGU | 24 | 18608 |
| HSV1-UL54-1620 | + | GCUUUGGUCGGUGGGGGU | 18 | 18609 |
| HSV1-UL54-1621 | + | GGCUUUGGUCGGUGGGGGU | 19 | 18610 |
| HSV1-UL54-131 | + | GGGCUUUGGUCGGUGGGGGU | 20 | 6894 |
| HSV1-UL54-1622 | + | UGGGCUUUGGUCGGUGGGGGU | 21 | 18611 |
| HSV1-UL54-1623 | + | CUGGGCUUUGGUCGGUGGGGGU | 22 | 18612 |
| HSV1-UL54-1624 | + | GCUGGGCUUUGGUCGGUGGGGGU | 23 | 18613 |
| HSV1-UL54-1625 | + | GGCUGGGCUUUGGUCGGUGGGGGU | 24 | 18614 |
| HSV1-UL54-1626 | + | CCUCGUCCAGAUCGCUGU | 18 | 18615 |
| HSV1-UL54-1627 | + | UCCUCGUCCAGAUCGCUGU | 19 | 18616 |
| HSV1-UL54-37 | + | GUCCUCGUCCAGAUCGCUGU | 20 | 6800 |
| HSV1-UL54-1628 | + | GGUCCUCGUCCAGAUCGCUGU | 21 | 18617 |
| HSV1-UL54-1629 | + | GGGUCCUCGUCCAGAUCGCUGU | 22 | 18618 |
| HSV1-UL54-1630 | + | GGGGUCCUCGUCCAGAUCGCUGU | 23 | 18619 |
| HSV1-UL54-1631 | + | GGGGGUCCUCGUCCAGAUCGCUGU | 24 | 18620 |
| HSV1-UL54-1632 | + | UGGGUACGCCGGGGUCUU | 18 | 18621 |
| HSV1-UL54-1633 | + | CUGGGUACGCCGGGGUCUU | 19 | 18622 |
| HSV1-UL54-1634 | + | GCUGGGUACGCCGGGGUCUU | 20 | 18623 |
| HSV1-UL54-1635 | + | UGCUGGGUACGCCGGGGUCUU | 21 | 18624 |
| HSV1-UL54-1636 | + | GUGCUGGGUACGCCGGGGUCUU | 22 | 18625 |
| HSV1-UL54-1637 | + | GGUGCUGGGUACGCCGGGGUCUU | 23 | 18626 |
| HSV1-UL54-1638 | + | GGGUGCUGGGUACGCCGGGGUCUU | 24 | 18627 |
| HSV1-UL54-1639 | + | CCAGGGUUGCGAUUGGUU | 18 | 18628 |
| HSV1-UL54-1640 | + | CCCAGGGUUGCGAUUGGUU | 19 | 18629 |
| HSV1-UL54-1641 | + | CCCCAGGGUUGCGAUUGGUU | 20 | 18630 |
| HSV1-UL54-1642 | + | CCCCCAGGGUUGCGAUUGGUU | 21 | 18631 |
| HSV1-UL54-1643 | + | UCCCCCAGGGUUGCGAUUGGUU | 22 | 18632 |
| HSV1-UL54-1644 | + | GUCCCCCAGGGUUGCGAUUGGUU | 23 | 18633 |
| HSV1-UL54-1645 | + | GGUCCCCCAGGGUUGCGAUUGGUU | 24 | 18634 |
| HSV1-UL54-1646 | - | CGCCGCGACGACCUGGAA | 18 | 18635 |
| HSV1-UL54-1647 | - | CCGCCGCGACGACCUGGAA | 19 | 18636 |
| HSV1-UL54-740 | - | GCCGCCGCGACGACCUGGAA | 20 | 7498 |
| HSV1-UL54-1648 | - | AGCCGCCGCGACGACCUGGAA | 21 | 18637 |
| HSV1-UL54-1649 | - | GAGCCGCCGCGACGACCUGGAA | 22 | 18638 |
| HSV1-UL54-1650 | - | AGAGCCGCCGCGACGACCUGGAA | 23 | 18639 |
| HSV1-UL54-1651 | - | GAGAGCCGCCGCGACGACCUGGAA | 24 | 18640 |
| HSV1-UL54-1652 | - | CCUCGUCGGACGAGGACA | 18 | 18641 |
| HSV1-UL54-1653 | - | UCCUCGUCGGACGAGGACA | 19 | 18642 |
| HSV1-UL54-5 | - | UUCCUCGUCGGACGAGGACA | 20 | 6768 |
| HSV1-UL54-1654 | - | GUUCCUCGUCGGACGAGGACA | 21 | 18643 |
| HSV1-UL54-1655 | - | UGUUCCUCGUCGGACGAGGACA | 22 | 18644 |
| HSV1-UL54-1656 | - | GUGUUCCUCGUCGGACGAGGACA | 23 | 18645 |
| HSV1-UL54-1657 | - | AGUGUUCCUCGUCGGACGAGGACA | 24 | 18646 |
| HSV1-UL54-1658 | - | GGACAUGGAAGACCCCCA | 18 | 18647 |
| HSV1-UL54-1659 | - | AGGACAUGGAAGACCCCCA | 19 | 18648 |
| HSV1-UL54-90 | - | GAGGACAUGGAAGACCCCCA | 20 | 6853 |
| HSV1-UL54-1660 | - | CGAGGACAUGGAAGACCCCCA | 21 | 18649 |
| HSV1-UL54-1661 | - | ACGAGGACAUGGAAGACCCCCA | 22 | 18650 |
| HSV1-UL54-1662 | - | GACGAGGACAUGGAAGACCCCCA | 23 | 18651 |
| HSV1-UL54-1663 | - | GGACGAGGACAUGGAAGACCCCCA | 24 | 18652 |
| HSV1-UL54-1664 | - | ACCUGGAAUCGGACAGCA | 18 | 18653 |
| HSV1-UL54-1665 | - | GACCUGGAAUCGGACAGCA | 19 | 18654 |
| HSV1-UL54-741 | - | CGACCUGGAAUCGGACAGCA | 20 | 7499 |
| HSV1-UL54-1666 | - | ACGACCUGGAAUCGGACAGCA | 21 | 18655 |
| HSV1-UL54-1667 | - | GACGACCUGGAAUCGGACAGCA | 22 | 18656 |
| HSV1-UL54-1668 | - | CGACGACCUGGAAUCGGACAGCA | 23 | 18657 |
| HSV1-UL54-1669 | - | GCGACGACCUGGAAUCGGACAGCA | 24 | 18658 |
| HSV1-UL54-1670 | - | UUGCUCCCCCGAGCAGCA | 18 | 18659 |
| HSV1-UL54-1671 | - | CUUGCUCCCCCGAGCAGCA | 19 | 18660 |
| HSV1-UL54-99 | - | UCUUGCUCCCCCGAGCAGCA | 20 | 6862 |
| HSV1-UL54-1672 | - | GUCUUGCUCCCCCGAGCAGCA | 21 | 18661 |
| HSV1-UL54-1673 | - | CGUCUUGCUCCCCCGAGCAGCA | 22 | 18662 |
| HSV1-UL54-1674 | - | CCGUCUUGCUCCCCCGAGCAGCA | 23 | 18663 |
| HSV1-UL54-1675 | - | ACCGUCUUGCUCCCCCGAGCAGCA | 24 | 18664 |
| HSV1-UL54-1676 | - | CCCAGACGCCUCGUCCGA | 18 | 18665 |
| HSV1-UL54-1677 | - | ACCCAGACGCCUCGUCCGA | 19 | 18666 |
| HSV1-UL54-8 | - | CACCCAGACGCCUCGUCCGA | 20 | 6771 |
| HSV1-UL54-1678 | - | GCACCCAGACGCCUCGUCCGA | 21 | 18667 |
| HSV1-UL54-1679 | - | AGCACCCAGACGCCUCGUCCGA | 22 | 18668 |
| HSV1-UL54-1680 | - | CAGCACCCAGACGCCUCGUCCGA | 23 | 18669 |
| HSV1-UL54-1681 | - | CCAGCACCCAGACGCCUCGUCCGA | 24 | 18670 |
| HSV1-UL54-1682 | - | AUGGAAGACCCCCACGGA | 18 | 18671 |
| HSV1-UL54-1683 | - | CAUGGAAGACCCCCACGGA | 19 | 18672 |
| HSV1-UL54-750 | - | ACAUGGAAGACCCCCACGGA | 20 | 7504 |
| HSV1-UL54-1684 | - | GACAUGGAAGACCCCCACGGA | 21 | 18673 |
| HSV1-UL54-1685 | - | GGACAUGGAAGACCCCCACGGA | 22 | 18674 |
| HSV1-UL54-1686 | - | AGGACAUGGAAGACCCCCACGGA | 23 | 18675 |
| HSV1-UL54-1687 | - | GAGGACAUGGAAGACCCCCACGGA | 24 | 18676 |
| HSV1-UL54-1688 | - | CCCCACGGAGAGGACGGA | 18 | 18677 |
| HSV1-UL54-1689 | - | CCCCCACGGAGAGGACGGA | 19 | 18678 |
| HSV1-UL54-751 | - | ACCCCCACGGAGAGGACGGA | 20 | 7505 |
| HSV1-UL54-1690 | - | GACCCCCACGGAGAGGACGGA | 21 | 18679 |
| HSV1-UL54-1691 | - | AGACCCCCACGGAGAGGACGGA | 22 | 18680 |
| HSV1-UL54-1692 | - | AAGACCCCCACGGAGAGGACGGA | 23 | 18681 |
| HSV1-UL54-1693 | - | GAAGACCCCCACGGAGAGGACGGA | 24 | 18682 |
| HSV1-UL54-1694 | - | UGCUCCCCCGAGCAGCAC | 18 | 18683 |
| HSV1-UL54-1695 | - | UUGCUCCCCCGAGCAGCAC | 19 | 18684 |
| HSV1-UL54-100 | - | CUUGCUCCCCCGAGCAGCAC | 20 | 6863 |
| HSV1-UL54-1696 | - | UCUUGCUCCCCCGAGCAGCAC | 21 | 18685 |
| HSV1-UL54-1697 | - | GUCUUGCUCCCCCGAGCAGCAC | 22 | 18686 |
| HSV1-UL54-1698 | - | CGUCUUGCUCCCCCGAGCAGCAC | 23 | 18687 |
| HSV1-UL54-1699 | - | CCGUCUUGCUCCCCCGAGCAGCAC | 24 | 18688 |
| HSV1-UL54-1700 | - | GAGAGCCGCCGCGACGAC | 18 | 18689 |
| HSV1-UL54-1701 | - | GGAGAGCCGCCGCGACGAC | 19 | 18690 |
| HSV1-UL54-738 | - | CGGAGAGCCGCCGCGACGAC | 20 | 7497 |
| HSV1-UL54-1702 | - | GCGGAGAGCCGCCGCGACGAC | 21 | 18691 |
| HSV1-UL54-1703 | - | GGCGGAGAGCCGCCGCGACGAC | 22 | 18692 |
| HSV1-UL54-1704 | - | CGGCGGAGAGCCGCCGCGACGAC | 23 | 18693 |
| HSV1-UL54-1705 | - | CCGGCGGAGAGCCGCCGCGACGAC | 24 | 18694 |
| HSV1-UL54-1706 | - | UCCUCGUCGGACGAGGAC | 18 | 18695 |
| HSV1-UL54-1707 | - | UUCCUCGUCGGACGAGGAC | 19 | 18696 |
| HSV1-UL54-747 | - | GUUCCUCGUCGGACGAGGAC | 20 | 7502 |
| HSV1-UL54-1708 | - | UGUUCCUCGUCGGACGAGGAC | 21 | 18697 |
| HSV1-UL54-1709 | - | GUGUUCCUCGUCGGACGAGGAC | 22 | 18698 |
| HSV1-UL54-1710 | - | AGUGUUCCUCGUCGGACGAGGAC | 23 | 18699 |
| HSV1-UL54-1711 | - | GAGUGUUCCUCGUCGGACGAGGAC | 24 | 18700 |
| HSV1-UL54-1712 | - | CCCACGGAGAGGACGGAC | 18 | 18701 |
| HSV1-UL54-1713 | - | CCCCACGGAGAGGACGGAC | 19 | 18702 |
| HSV1-UL54-93 | - | CCCCCACGGAGAGGACGGAC | 20 | 6856 |
| HSV1-UL54-1714 | - | ACCCCCACGGAGAGGACGGAC | 21 | 18703 |
| HSV1-UL54-1715 | - | GACCCCCACGGAGAGGACGGAC | 22 | 18704 |
| HSV1-UL54-1716 | - | AGACCCCCACGGAGAGGACGGAC | 23 | 18705 |
| HSV1-UL54-1717 | - | AAGACCCCCACGGAGAGGACGGAC | 24 | 18706 |
| HSV1-UL54-1718 | - | GAGUGUUCCUCGUCGGAC | 18 | 18707 |
| HSV1-UL54-1719 | - | GGAGUGUUCCUCGUCGGAC | 19 | 18708 |
| HSV1-UL54-746 | - | GGGAGUGUUCCUCGUCGGAC | 20 | 7501 |
| HSV1-UL54-1720 | - | GGGGAGUGUUCCUCGUCGGAC | 21 | 18709 |
| HSV1-UL54-1721 | - | CGGGGAGUGUUCCUCGUCGGAC | 22 | 18710 |
| HSV1-UL54-1722 | - | GCGGGGAGUGUUCCUCGUCGGAC | 23 | 18711 |
| HSV1-UL54-1723 | - | AGCGGGGAGUGUUCCUCGUCGGAC | 24 | 18712 |
| HSV1-UL54-1724 | - | UCCGACAGCGAUCUGGAC | 18 | 18713 |
| HSV1-UL54-1725 | - | CUCCGACAGCGAUCUGGAC | 19 | 18714 |
| HSV1-UL54-735 | - | UCUCCGACAGCGAUCUGGAC | 20 | 7495 |
| HSV1-UL54-1726 | - | CUCUCCGACAGCGAUCUGGAC | 21 | 18715 |
| HSV1-UL54-1727 | - | CCUCUCCGACAGCGAUCUGGAC | 22 | 18716 |
| HSV1-UL54-1728 | - | ACCUCUCCGACAGCGAUCUGGAC | 23 | 18717 |
| HSV1-UL54-1729 | - | GACCUCUCCGACAGCGAUCUGGAC | 24 | 18718 |
| HSV1-UL54-1730 | - | CCAGAACCAAUCGCAACC | 18 | 18719 |
| HSV1-UL54-1731 | - | CCCAGAACCAAUCGCAACC | 19 | 18720 |
| HSV1-UL54-1732 | - | CCCCAGAACCAAUCGCAACC | 20 | 18721 |
| HSV1-UL54-1733 | - | CCCCCAGAACCAAUCGCAACC | 21 | 18722 |
| HSV1-UL54-1734 | - | GCCCCCAGAACCAAUCGCAACC | 22 | 18723 |
| HSV1-UL54-1735 | - | UGCCCCCAGAACCAAUCGCAACC | 23 | 18724 |
| HSV1-UL54-1736 | - | GUGCCCCCAGAACCAAUCGCAACC | 24 | 18725 |
| HSV1-UL54-1737 | - | CAGAACCAAUCGCAACCC | 18 | 18726 |
| HSV1-UL54-1738 | - | CCAGAACCAAUCGCAACCC | 19 | 18727 |
| HSV1-UL54-1195 | - | CCCAGAACCAAUCGCAACCC | 20 | 18184 |
| HSV1-UL54-1739 | - | CCCCAGAACCAAUCGCAACCC | 21 | 18728 |
| HSV1-UL54-1740 | - | CCCCCAGAACCAAUCGCAACCC | 22 | 18729 |
| HSV1-UL54-1741 | - | GCCCCCAGAACCAAUCGCAACCC | 23 | 18730 |
| HSV1-UL54-1742 | - | UGCCCCCAGAACCAAUCGCAACCC | 24 | 18731 |
| HSV1-UL54-1743 | - | AGGACAUGGAAGACCCCC | 18 | 18732 |
| HSV1-UL54-1744 | - | GAGGACAUGGAAGACCCCC | 19 | 18733 |
| HSV1-UL54-748 | - | CGAGGACAUGGAAGACCCCC | 20 | 7503 |
| HSV1-UL54-1745 | - | ACGAGGACAUGGAAGACCCCC | 21 | 18734 |
| HSV1-UL54-1746 | - | GACGAGGACAUGGAAGACCCCC | 22 | 18735 |
| HSV1-UL54-1747 | - | GGACGAGGACAUGGAAGACCCCC | 23 | 18736 |
| HSV1-UL54-1748 | - | CGGACGAGGACAUGGAAGACCCCC | 24 | 18737 |
| HSV1-UL54-1749 | - | AUCUGGACGAGGACCCCC | 18 | 18738 |
| HSV1-UL54-1750 | - | GAUCUGGACGAGGACCCCC | 19 | 18739 |
| HSV1-UL54-736 | - | CGAUCUGGACGAGGACCCCC | 20 | 7496 |
| HSV1-UL54-1751 | - | GCGAUCUGGACGAGGACCCCC | 21 | 18740 |
| HSV1-UL54-1752 | - | AGCGAUCUGGACGAGGACCCCC | 22 | 18741 |
| HSV1-UL54-1753 | - | CAGCGAUCUGGACGAGGACCCCC | 23 | 18742 |
| HSV1-UL54-1754 | - | ACAGCGAUCUGGACGAGGACCCCC | 24 | 18743 |
| HSV1-UL54-1755 | - | CCCCCGCCGGCGUGCCCC | 18 | 18744 |
| HSV1-UL54-1756 | - | ACCCCCGCCGGCGUGCCCC | 19 | 18745 |
| HSV1-UL54-1757 | - | GACCCCCGCCGGCGUGCCCC | 20 | 18746 |
| HSV1-UL54-1758 | - | GGACCCCCGCCGGCGUGCCCC | 21 | 18747 |
| HSV1-UL54-1759 | - | CGGACCCCCGCCGGCGUGCCCC | 22 | 18748 |
| HSV1-UL54-1760 | - | UCGGACCCCCGCCGGCGUGCCCC | 23 | 18749 |
| HSV1-UL54-1761 | - | GUCGGACCCCCGCCGGCGUGCCCC | 24 | 18750 |
| HSV1-UL54-1762 | - | GGCGACCGUCUUGCUCCC | 18 | 18751 |
| HSV1-UL54-1763 | - | CGGCGACCGUCUUGCUCCC | 19 | 18752 |
| HSV1-UL54-759 | - | CCGGCGACCGUCUUGCUCCC | 20 | 7510 |
| HSV1-UL54-1764 | - | CCCGGCGACCGUCUUGCUCCC | 21 | 18753 |
| HSV1-UL54-1765 | - | GCCCGGCGACCGUCUUGCUCCC | 22 | 18754 |
| HSV1-UL54-1766 | - | GGCCCGGCGACCGUCUUGCUCCC | 23 | 18755 |
| HSV1-UL54-1767 | - | GGGCCCGGCGACCGUCUUGCUCCC | 24 | 18756 |
| HSV1-UL54-1768 | - | CUGCCCGCGGCGGACGCC | 18 | 18757 |
| HSV1-UL54-1769 | - | CCUGCCCGCGGCGGACGCC | 19 | 18758 |
| HSV1-UL54-764 | - | GCCUGCCCGCGGCGGACGCC | 20 | 7513 |
| HSV1-UL54-1770 | - | AGCCUGCCCGCGGCGGACGCC | 21 | 18759 |
| HSV1-UL54-1771 | - | CAGCCUGCCCGCGGCGGACGCC | 22 | 18760 |
| HSV1-UL54-1772 | - | CCAGCCUGCCCGCGGCGGACGCC | 23 | 18761 |
| HSV1-UL54-1773 | - | CCCAGCCUGCCCGCGGCGGACGCC | 24 | 18762 |
| HSV1-UL54-1774 | - | ACAGUGUGUGGUCGCGCC | 18 | 18763 |
| HSV1-UL54-1775 | - | CACAGUGUGUGGUCGCGCC | 19 | 18764 |
| HSV1-UL54-757 | - | CCACAGUGUGUGGUCGCGCC | 20 | 7509 |
| HSV1-UL54-1776 | - | CCCACAGUGUGUGGUCGCGCC | 21 | 18765 |
| HSV1-UL54-1777 | - | CCCCACAGUGUGUGGUCGCGCC | 22 | 18766 |
| HSV1-UL54-1778 | - | GCCCCACAGUGUGUGGUCGCGCC | 23 | 18767 |
| HSV1-UL54-1779 | - | CGCCCCACAGUGUGUGGUCGCGCC | 24 | 18768 |
| HSV1-UL54-1780 | - | UCGGGGUCGCGGUGGUCC | 18 | 18769 |
| HSV1-UL54-1781 | - | GUCGGGGUCGCGGUGGUCC | 19 | 18770 |
| HSV1-UL54-116 | - | GGUCGGGGUCGCGGUGGUCC | 20 | 6879 |
| HSV1-UL54-1782 | - | GGGUCGGGGUCGCGGUGGUCC | 21 | 18771 |
| HSV1-UL54-1783 | - | GGGGUCGGGGUCGCGGUGGUCC | 22 | 18772 |
| HSV1-UL54-1784 | - | AGGGGUCGGGGUCGCGGUGGUCC | 23 | 18773 |
| HSV1-UL54-1785 | - | CAGGGGUCGGGGUCGCGGUGGUCC | 24 | 18774 |
| HSV1-UL54-1786 | - | CUGGAAUCGGACAGCAGC | 18 | 18775 |
| HSV1-UL54-1787 | - | CCUGGAAUCGGACAGCAGC | 19 | 18776 |
| HSV1-UL54-87 | - | ACCUGGAAUCGGACAGCAGC | 20 | 6850 |
| HSV1-UL54-1788 | - | GACCUGGAAUCGGACAGCAGC | 21 | 18777 |
| HSV1-UL54-1789 | - | CGACCUGGAAUCGGACAGCAGC | 22 | 18778 |
| HSV1-UL54-1790 | - | ACGACCUGGAAUCGGACAGCAGC | 23 | 18779 |
| HSV1-UL54-1791 | - | GACGACCUGGAAUCGGACAGCAGC | 24 | 18780 |
| HSV1-UL54-1792 | - | CUUGCUCCCCCGAGCAGC | 18 | 18781 |
| HSV1-UL54-1793 | - | UCUUGCUCCCCCGAGCAGC | 19 | 18782 |
| HSV1-UL54-760 | - | GUCUUGCUCCCCCGAGCAGC | 20 | 7511 |
| HSV1-UL54-1794 | - | CGUCUUGCUCCCCCGAGCAGC | 21 | 18783 |
| HSV1-UL54-1795 | - | CCGUCUUGCUCCCCCGAGCAGC | 22 | 18784 |
| HSV1-UL54-1796 | - | ACCGUCUUGCUCCCCCGAGCAGC | 23 | 18785 |
| HSV1-UL54-1797 | - | GACCGUCUUGCUCCCCCGAGCAGC | 24 | 18786 |
| HSV1-UL54-1798 | - | AUGCUAAUUGACCUCGGC | 18 | 18787 |
| HSV1-UL54-1799 | - | UAUGCUAAUUGACCUCGGC | 19 | 18788 |
| HSV1-UL54-733 | - | AUAUGCUAAUUGACCUCGGC | 20 | 7493 |
| HSV1-UL54-1800 | - | GAUAUGCUAAUUGACCUCGGC | 21 | 18789 |
| HSV1-UL54-1801 | - | UGAUAUGCUAAUUGACCUCGGC | 22 | 18790 |
| HSV1-UL54-1802 | - | UUGAUAUGCUAAUUGACCUCGGC | 23 | 18791 |
| HSV1-UL54-1803 | - | AUUGAUAUGCUAAUUGACCUCGGC | 24 | 18792 |
| HSV1-UL54-1804 | - | CGGUCCGCCCGUCUCGUC | 18 | 18793 |
| HSV1-UL54-1805 | - | GCGGUCCGCCCGUCUCGUC | 19 | 18794 |
| HSV1-UL54-753 | - | GGCGGUCCGCCCGUCUCGUC | 20 | 7506 |
| HSV1-UL54-1806 | - | CGGCGGUCCGCCCGUCUCGUC | 21 | 18795 |
| HSV1-UL54-1807 | - | CCGGCGGUCCGCCCGUCUCGUC | 22 | 18796 |
| HSV1-UL54-1808 | - | CCCGGCGGUCCGCCCGUCUCGUC | 23 | 18797 |
| HSV1-UL54-1809 | - | GCCCGGCGGUCCGCCCGUCUCGUC | 24 | 18798 |
| HSV1-UL54-1810 | - | GUCGGGGUCGCGGUGGUC | 18 | 18799 |
| HSV1-UL54-1811 | - | GGUCGGGGUCGCGGUGGUC | 19 | 18800 |
| HSV1-UL54-769 | - | GGGUCGGGGUCGCGGUGGUC | 20 | 7516 |
| HSV1-UL54-1812 | - | GGGGUCGGGGUCGCGGUGGUC | 21 | 18801 |
| HSV1-UL54-1813 | - | AGGGGUCGGGGUCGCGGUGGUC | 22 | 18802 |
| HSV1-UL54-1814 | - | CAGGGGUCGGGGUCGCGGUGGUC | 23 | 18803 |
| HSV1-UL54-1815 | - | GCAGGGGUCGGGGUCGCGGUGGUC | 24 | 18804 |
| HSV1-UL54-1816 | - | CCUGGAAUCGGACAGCAG | 18 | 18805 |
| HSV1-UL54-1817 | - | ACCUGGAAUCGGACAGCAG | 19 | 18806 |
| HSV1-UL54-86 | - | GACCUGGAAUCGGACAGCAG | 20 | 6849 |
| HSV1-UL54-1818 | - | CGACCUGGAAUCGGACAGCAG | 21 | 18807 |
| HSV1-UL54-1819 | - | ACGACCUGGAAUCGGACAGCAG | 22 | 18808 |
| HSV1-UL54-1820 | - | GACGACCUGGAAUCGGACAGCAG | 23 | 18809 |
| HSV1-UL54-1821 | - | CGACGACCUGGAAUCGGACAGCAG | 24 | 18810 |
| HSV1-UL54-1822 | - | ACAUGGAAGACCCCCACG | 18 | 18811 |
| HSV1-UL54-1823 | - | GACAUGGAAGACCCCCACG | 19 | 18812 |
| HSV1-UL54-727 | - | GGACAUGGAAGACCCCCACG | 20 | 7488 |
| HSV1-UL54-1824 | - | AGGACAUGGAAGACCCCCACG | 21 | 18813 |
| HSV1-UL54-1825 | - | GAGGACAUGGAAGACCCCCACG | 22 | 18814 |
| HSV1-UL54-1826 | - | CGAGGACAUGGAAGACCCCCACG | 23 | 18815 |
| HSV1-UL54-1827 | - | ACGAGGACAUGGAAGACCCCCACG | 24 | 18816 |
| HSV1-UL54-1828 | - | GAGGACCCCCCCGAGCCG | 18 | 18817 |
| HSV1-UL54-1829 | - | CGAGGACCCCCCCGAGCCG | 19 | 18818 |
| HSV1-UL54-729 | - | ACGAGGACCCCCCCGAGCCG | 20 | 7490 |
| HSV1-UL54-1830 | - | GACGAGGACCCCCCCGAGCCG | 21 | 18819 |
| HSV1-UL54-1831 | - | GGACGAGGACCCCCCCGAGCCG | 22 | 18820 |
| HSV1-UL54-1832 | - | UGGACGAGGACCCCCCCGAGCCG | 23 | 18821 |
| HSV1-UL54-1833 | - | CUGGACGAGGACCCCCCCGAGCCG | 24 | 18822 |
| HSV1-UL54-1834 | - | ACCCAGACGCCUCGUCCG | 18 | 18823 |
| HSV1-UL54-1835 | - | CACCCAGACGCCUCGUCCG | 19 | 18824 |
| HSV1-UL54-754 | - | GCACCCAGACGCCUCGUCCG | 20 | 7507 |
| HSV1-UL54-1836 | - | AGCACCCAGACGCCUCGUCCG | 21 | 18825 |
| HSV1-UL54-1837 | - | CAGCACCCAGACGCCUCGUCCG | 22 | 18826 |
| HSV1-UL54-1838 | - | CCAGCACCCAGACGCCUCGUCCG | 23 | 18827 |
| HSV1-UL54-1839 | - | CCCAGCACCCAGACGCCUCGUCCG | 24 | 18828 |
| HSV1-UL54-1840 | - | AAGCCCAGCCUGCCCGCG | 18 | 18829 |
| HSV1-UL54-1841 | - | AAAGCCCAGCCUGCCCGCG | 19 | 18830 |
| HSV1-UL54-763 | - | CAAAGCCCAGCCUGCCCGCG | 20 | 7512 |
| HSV1-UL54-1842 | - | CCAAAGCCCAGCCUGCCCGCG | 21 | 18831 |
| HSV1-UL54-1843 | - | ACCAAAGCCCAGCCUGCCCGCG | 22 | 18832 |
| HSV1-UL54-1844 | - | GACCAAAGCCCAGCCUGCCCGCG | 23 | 18833 |
| HSV1-UL54-1845 | - | CGACCAAAGCCCAGCCUGCCCGCG | 24 | 18834 |
| HSV1-UL54-1846 | - | GACCCCCCCGAGCCGGCG | 18 | 18835 |
| HSV1-UL54-1847 | - | GGACCCCCCCGAGCCGGCG | 19 | 18836 |
| HSV1-UL54-730 | - | AGGACCCCCCCGAGCCGGCG | 20 | 7491 |
| HSV1-UL54-1848 | - | GAGGACCCCCCCGAGCCGGCG | 21 | 18837 |
| HSV1-UL54-1849 | - | CGAGGACCCCCCCGAGCCGGCG | 22 | 18838 |
| HSV1-UL54-1850 | - | ACGAGGACCCCCCCGAGCCGGCG | 23 | 18839 |
| HSV1-UL54-1851 | - | GACGAGGACCCCCCCGAGCCGGCG | 24 | 18840 |
| HSV1-UL54-1852 | - | AGCGGGGAGUGUUCCUCG | 18 | 18841 |
| HSV1-UL54-1853 | - | CAGCGGGGAGUGUUCCUCG | 19 | 18842 |
| HSV1-UL54-745 | - | GCAGCGGGGAGUGUUCCUCG | 20 | 7500 |
| HSV1-UL54-1854 | - | AGCAGCGGGGAGUGUUCCUCG | 21 | 18843 |
| HSV1-UL54-1855 | - | CAGCAGCGGGGAGUGUUCCUCG | 22 | 18844 |
| HSV1-UL54-1856 | - | ACAGCAGCGGGGAGUGUUCCUCG | 23 | 18845 |
| HSV1-UL54-1857 | - | GACAGCAGCGGGGAGUGUUCCUCG | 24 | 18846 |
| HSV1-UL54-1858 | - | CGGACGCCGUGGGCGUCG | 18 | 18847 |
| HSV1-UL54-1859 | - | GCGGACGCCGUGGGCGUCG | 19 | 18848 |
| HSV1-UL54-765 | - | GGCGGACGCCGUGGGCGUCG | 20 | 7514 |
| HSV1-UL54-1860 | - | CGGCGGACGCCGUGGGCGUCG | 21 | 18849 |
| HSV1-UL54-1861 | - | GCGGCGGACGCCGUGGGCGUCG | 22 | 18850 |
| HSV1-UL54-1862 | - | CGCGGCGGACGCCGUGGGCGUCG | 23 | 18851 |
| HSV1-UL54-1863 | - | CCGCGGCGGACGCCGUGGGCGUCG | 24 | 18852 |
| HSV1-UL54-1864 | - | AAGACCCCCACGGAGAGG | 18 | 18853 |
| HSV1-UL54-1865 | - | GAAGACCCCCACGGAGAGG | 19 | 18854 |
| HSV1-UL54-728 | - | GGAAGACCCCCACGGAGAGG | 20 | 7489 |
| HSV1-UL54-1866 | - | UGGAAGACCCCCACGGAGAGG | 21 | 18855 |
| HSV1-UL54-1867 | - | AUGGAAGACCCCCACGGAGAGG | 22 | 18856 |
| HSV1-UL54-1868 | - | CAUGGAAGACCCCCACGGAGAGG | 23 | 18857 |
| HSV1-UL54-1869 | - | ACAUGGAAGACCCCCACGGAGAGG | 24 | 18858 |
| HSV1-UL54-1870 | - | AGGACCCCCCCGAGCCGG | 18 | 18859 |
| HSV1-UL54-1871 | - | GAGGACCCCCCCGAGCCGG | 19 | 18860 |
| HSV1-UL54-84 | - | CGAGGACCCCCCCGAGCCGG | 20 | 6847 |
| HSV1-UL54-1872 | - | ACGAGGACCCCCCCGAGCCGG | 21 | 18861 |
| HSV1-UL54-1873 | - | GACGAGGACCCCCCCGAGCCGG | 22 | 18862 |
| HSV1-UL54-1874 | - | GGACGAGGACCCCCCCGAGCCGG | 23 | 18863 |
| HSV1-UL54-1875 | - | UGGACGAGGACCCCCCCGAGCCGG | 24 | 18864 |
| HSV1-UL54-1876 | - | CCUCGUCCGACGGAGCGG | 18 | 18865 |
| HSV1-UL54-1877 | - | GCCUCGUCCGACGGAGCGG | 19 | 18866 |
| HSV1-UL54-756 | - | CGCCUCGUCCGACGGAGCGG | 20 | 7508 |
| HSV1-UL54-1878 | - | ACGCCUCGUCCGACGGAGCGG | 21 | 18867 |
| HSV1-UL54-1879 | - | GACGCCUCGUCCGACGGAGCGG | 22 | 18868 |
| HSV1-UL54-1880 | - | AGACGCCUCGUCCGACGGAGCGG | 23 | 18869 |
| HSV1-UL54-1881 | - | CAGACGCCUCGUCCGACGGAGCGG | 24 | 18870 |
| HSV1-UL54-1882 | - | GGGAGUGUUCCUCGUCGG | 18 | 18871 |
| HSV1-UL54-1883 | - | GGGGAGUGUUCCUCGUCGG | 19 | 18872 |
| HSV1-UL54-731 | - | CGGGGAGUGUUCCUCGUCGG | 20 | 7492 |
| HSV1-UL54-1884 | - | GCGGGGAGUGUUCCUCGUCGG | 21 | 18873 |
| HSV1-UL54-1885 | - | AGCGGGGAGUGUUCCUCGUCGG | 22 | 18874 |
| HSV1-UL54-1886 | - | CAGCGGGGAGUGUUCCUCGUCGG | 23 | 18875 |
| HSV1-UL54-1887 | - | GCAGCGGGGAGUGUUCCUCGUCGG | 24 | 18876 |
| HSV1-UL54-1888 | - | CCGUGGGCGUCGCAGGGG | 18 | 18877 |
| HSV1-UL54-1889 | - | GCCGUGGGCGUCGCAGGGG | 19 | 18878 |
| HSV1-UL54-767 | - | CGCCGUGGGCGUCGCAGGGG | 20 | 7515 |
| HSV1-UL54-1890 | - | ACGCCGUGGGCGUCGCAGGGG | 21 | 18879 |
| HSV1-UL54-1891 | - | GACGCCGUGGGCGUCGCAGGGG | 22 | 18880 |
| HSV1-UL54-1892 | - | GGACGCCGUGGGCGUCGCAGGGG | 23 | 18881 |
| HSV1-UL54-1893 | - | CGGACGCCGUGGGCGUCGCAGGGG | 24 | 18882 |
| HSV1-UL54-1894 | - | UCUCCGACAGCGAUCUGG | 18 | 18883 |
| HSV1-UL54-1895 | - | CUCUCCGACAGCGAUCUGG | 19 | 18884 |
| HSV1-UL54-726 | - | CCUCUCCGACAGCGAUCUGG | 20 | 7487 |
| HSV1-UL54-1896 | - | ACCUCUCCGACAGCGAUCUGG | 21 | 18885 |
| HSV1-UL54-1897 | - | GACCUCUCCGACAGCGAUCUGG | 22 | 18886 |
| HSV1-UL54-1898 | - | GGACCUCUCCGACAGCGAUCUGG | 23 | 18887 |
| HSV1-UL54-1899 | - | UGGACCUCUCCGACAGCGAUCUGG | 24 | 18888 |
| HSV1-UL54-1900 | - | GAACCAAUCGCAACCCUG | 18 | 18889 |
| HSV1-UL54-1901 | - | AGAACCAAUCGCAACCCUG | 19 | 18890 |
| HSV1-UL54-910 | - | CAGAACCAAUCGCAACCCUG | 20 | 7585 |
| HSV1-UL54-1902 | - | CCAGAACCAAUCGCAACCCUG | 21 | 18891 |
| HSV1-UL54-1903 | - | CCCAGAACCAAUCGCAACCCUG | 22 | 18892 |
| HSV1-UL54-1904 | - | CCCCAGAACCAAUCGCAACCCUG | 23 | 18893 |
| HSV1-UL54-1905 | - | CCCCCAGAACCAAUCGCAACCCUG | 24 | 18894 |
| HSV1-UL54-1906 | - | GGGGCUGCCGAUGGUUUG | 18 | 18895 |
| HSV1-UL54-1907 | - | CGGGGCUGCCGAUGGUUUG | 19 | 18896 |
| HSV1-UL54-771 | - | CCGGGGCUGCCGAUGGUUUG | 20 | 7517 |
| HSV1-UL54-1908 | - | CCCGGGGCUGCCGAUGGUUUG | 21 | 18897 |
| HSV1-UL54-1909 | - | UCCCGGGGCUGCCGAUGGUUUG | 22 | 18898 |
| HSV1-UL54-1910 | - | GUCCCGGGGCUGCCGAUGGUUUG | 23 | 18899 |
| HSV1-UL54-1911 | - | GGUCCCGGGGCUGCCGAUGGUUUG | 24 | 18900 |
| HSV1-UL54-1912 | - | GACCUCUCCGACAGCGAU | 18 | 18901 |
| HSV1-UL54-1913 | - | GGACCUCUCCGACAGCGAU | 19 | 18902 |
| HSV1-UL54-734 | - | UGGACCUCUCCGACAGCGAU | 20 | 7494 |
| HSV1-UL54-1914 | - | CUGGACCUCUCCGACAGCGAU | 21 | 18903 |
| HSV1-UL54-1915 | - | CCUGGACCUCUCCGACAGCGAU | 22 | 18904 |
| HSV1-UL54-1916 | - | GCCUGGACCUCUCCGACAGCGAU | 23 | 18905 |
| HSV1-UL54-1917 | - | GGCCUGGACCUCUCCGACAGCGAU | 24 | 18906 |
| HSV1-UL54-1918 | - | AGAACCAAUCGCAACCCU | 18 | 18907 |
| HSV1-UL54-1919 | - | CAGAACCAAUCGCAACCCU | 19 | 18908 |
| HSV1-UL54-1204 | - | CCAGAACCAAUCGCAACCCU | 20 | 18193 |
| HSV1-UL54-1920 | - | CCCAGAACCAAUCGCAACCCU | 21 | 18909 |
| HSV1-UL54-1921 | - | CCCCAGAACCAAUCGCAACCCU | 22 | 18910 |
| HSV1-UL54-1922 | - | CCCCCAGAACCAAUCGCAACCCU | 23 | 18911 |
| HSV1-UL54-1923 | - | GCCCCCAGAACCAAUCGCAACCCU | 24 | 18912 |
| HSV1-UL54-1924 | - | CAGUGUGUGGUCGCGCCU | 18 | 18913 |
| HSV1-UL54-1925 | - | ACAGUGUGUGGUCGCGCCU | 19 | 18914 |
| HSV1-UL54-98 | - | CACAGUGUGUGGUCGCGCCU | 20 | 6861 |
| HSV1-UL54-1926 | - | CCACAGUGUGUGGUCGCGCCU | 21 | 18915 |
| HSV1-UL54-1927 | - | CCCACAGUGUGUGGUCGCGCCU | 22 | 18916 |
| HSV1-UL54-1928 | - | CCCCACAGUGUGUGGUCGCGCCU | 23 | 18917 |
| HSV1-UL54-1929 | - | GCCCCACAGUGUGUGGUCGCGCCU | 24 | 18918 |

**Table 15E** provides exemplary targeting domains for knocking out the *UL54* gene selected according to the fifth tier parameters. The targeting domains fall in the coding sequence of the gene, downstream of the first 500bp of coding sequence (e.g., anywhere from +500 (relative to the start codon) to the stop codon of the gene), and start with a 5' G.The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15E**

| 5th Tier | | | | |
|---|---|---|---|---|
| gRNA Name | DNA Strand | Targeting Domain | Target Site Length | Seq ID NO: |
| HSV1-UL54-1930 | + | GAAACGGCUGCCCCCCAA | 18 | 18919 |
| HSV1-UL54-1931 | + | GGAAACGGCUGCCCCCCAA | 19 | 18920 |
| HSV1-UL54-421 | + | GGGAAACGGCUGCCCCCCAA | 20 | 7184 |
| HSV1-UL54-1932 | + | GCGGGAAACGGCUGCCCCCCAA | 22 | 18921 |
| HSV1-UL54-1933 | + | GGCGGGAAACGGCUGCCCCCCAA | 23 | 18922 |
| HSV1-UL54-1934 | + | GCCGCGGUCGUCCCGAUAA | 19 | 18923 |
| HSV1-UL54-1935 | + | GCACAGCCGCGGUCGUCCCGAUAA | 24 | 18924 |
| HSV1-UL54-1936 | + | GUUUUUAUUGUACCUAAAACA | 21 | 18925 |
| HSV1-UL54-1937 | + | GCCCCGGGGCGGGGUCCCC | 19 | 18926 |
| HSV1-UL54-1938 | + | GCCUUGGCGGUCGAUGCGGCCC | 22 | 18927 |
| HSV1-UL54-1939 | + | GGCCUUGGCGGUCGAUGCGGCCC | 23 | 18928 |
| HSV1-UL54-1940 | + | GCUCCGGGGCCGGGGCGC | 18 | 18929 |
| HSV1-UL54-1231 | + | GUUCUGGGGGCACGCCGGC | 19 | 18220 |
| HSV1-UL54-1189 | + | GGUUCUGGGGGCACGCCGGC | 20 | 18178 |
| HSV1-UL54-1232 | + | GAUUGGUUCUGGGGGCACGCCGGC | 24 | 18221 |
| HSV1-UL54-1941 | + | GCGGUUGGCGAGCCUGGC | 18 | 18930 |
| HSV1-UL54-1942 | + | GACGCGGUUGGCGAGCCUGGC | 21 | 18931 |
| HSV1-UL54-1943 | + | GAACACUCCUGGCGGUGCGUGUC | 23 | 18932 |
| HSV1-UL54-1944 | + | GCCAGAAUGACAAACACG | 18 | 18933 |
| HSV1-UL54-1945 | + | GGCCAGAAUGACAAACACG | 19 | 18934 |
| HSV1-UL54-1946 | + | GCCUGGCCAGAAUGACAAACACG | 23 | 18935 |
| HSV1-UL54-1947 | + | GUGUCCGCUGGCCUCCGG | 18 | 18936 |
| HSV1-UL54-1948 | + | GCCCCGUGUCCGCUGGCCUCCGG | 23 | 18937 |
| HSV1-UL54-1949 | + | GCCAGCGUCAUUAGCGGG | 18 | 18938 |
| HSV1-UL54-1950 | + | GCAAUCGCCAGCGUCAUUAGCGGG | 24 | 18939 |
| HSV1-UL54-1951 | + | GUCCACCCCGCCCCGGGG | 18 | 18940 |
| HSV1-UL54-1952 | + | GUCCGUCCACCCCGCCCCGGGG | 22 | 18941 |
| HSV1-UL54-1953 | + | GCGCAGCGGCAGGUUGUG | 18 | 18942 |
| HSV1-UL54-1954 | + | GGCGCAGCGGCAGGUUGUG | 19 | 18943 |
| HSV1-UL54-1955 | + | GGGCGCAGCGGCAGGUUGUG | 20 | 18944 |
| HSV1-UL54-1956 | + | GGGGCGCAGCGGCAGGUUGUG | 21 | 18945 |
| HSV1-UL54-1957 | + | GGGGGCGCAGCGGCAGGUUGUG | 22 | 18946 |
| HSV1-UL54-1958 | + | GCCCCGUGUCCGCUGGCCU | 19 | 18947 |
| HSV1-UL54-1959 | + | GCACGCCCCGUGUCCGCUGGCCU | 23 | 18948 |
| HSV1-UL54-1960 | - | GAAAUCCUAGACACGCACCGCC | 22 | 18949 |
| HSV1-UL54-1961 | - | GGGCAGCCGUUUCCCGCC | 18 | 18950 |
| HSV1-UL54-1962 | - | GGGGCAGCCGUUUCCCGCC | 19 | 18951 |
| HSV1-UL54-935 | - | GGGGGCAGCCGUUUCCCGCC | 20 | 7602 |
| HSV1-UL54-1963 | - | GGGGGGCAGCCGUUUCCCGCC | 21 | 18952 |
| HSV1-UL54-1964 | - | GCCCUUUGACGCCGAGACCAG | 21 | 18953 |
| HSV1-UL54-1965 | - | GGCCCUUUGACGCCGAGACCAG | 22 | 18954 |
| HSV1-UL54-1966 | - | GGGCCCUUUGACGCCGAGACCAG | 23 | 18955 |
| HSV1-UL54-1967 | - | GGGGACCCCGCCCCGGGG | 18 | 18956 |
| HSV1-UL54-1968 | - | GGGGGACCCCGCCCCGGGG | 19 | 18957 |
| HSV1-UL54-1969 | - | GGGACGACCGCGGCUGUG | 18 | 18958 |
| HSV1-UL54-1970 | - | GAGUGUUCGAGUCGUGUCU | 19 | 18959 |
| HSV1-UL54-977 | - | GGAGUGUUCGAGUCGUGUCU | 20 | 7635 |
| HSV1-UL54-1971 | - | GCCAGGAGUGUUCGAGUCGUGUCU | 24 | 18960 |
| HSV1-UL54-1972 | - | GACACGCACCGCCAGGAGUGU | 21 | 18961 |
| HSV1-UL54-333 | - | GACUACGCGACCCUUGGUGU | 20 | 7096 |
| HSV1-UL54-1973 | - | GAUCGACUACGCGACCCUUGGUGU | 24 | 18962 |

**Table 15F** provides exemplary targeting domains for knocking out the *UL54* gene selected according to the six tier parameters. The targeting domains fall in the coding sequence of the gene, downstream of the first 500bp of coding sequence (e.g., anywhere from +500 (relative to the start codon) to the stop codon of the gene). The PAM is NNGRRT. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15F**

| 6th Tier | | | | |
|---|---|---|---|---|
| gRNA Name | DNA Strand | Targeting Domain | Target Site Length | Seq ID NO: |
| HSV1-UL54-1974 | + | CGGGAAACGGCUGCCCCCCAA | 21 | 18963 |
| HSV1-UL54-1975 | + | CGGCGGGAAACGGCUGCCCCCCAA | 24 | 18964 |
| HSV1-UL54-1976 | + | CCGCGGUCGUCCCGAUAA | 18 | 18965 |
| HSV1-UL54-378 | + | AGCCGCGGUCGUCCCGAUAA | 20 | 7141 |
| HSV1-UL54-1977 | + | CAGCCGCGGUCGUCCCGAUAA | 21 | 18966 |
| HSV1-UL54-1978 | + | ACAGCCGCGGUCGUCCCGAUAA | 22 | 18967 |
| HSV1-UL54-1979 | + | CACAGCCGCGGUCGUCCCGAUAA | 23 | 18968 |
| HSV1-UL54-1980 | + | UUUAUUGUACCUAAAACA | 18 | 18969 |
| HSV1-UL54-1981 | + | UUUUAUUGUACCUAAAACA | 19 | 18970 |
| HSV1-UL54-1225 | + | UUUUUAUUGUACCUAAAACA | 20 | 18214 |
| HSV1-UL54-1982 | + | UGUUUUUAUUGUACCUAAAACA | 22 | 18971 |
| HSV1-UL54-1983 | + | UUGUUUUUAUUGUACCUAAAACA | 23 | 18972 |
| HSV1-UL54-1984 | + | UUUGUUUUUAUUGUACCUAAAACA | 24 | 18973 |
| HSV1-UL54-1985 | + | CUCUCCGACCCCGACACC | 18 | 18974 |
| HSV1-UL54-1986 | + | UCUCUCCGACCCCGACACC | 19 | 18975 |
| HSV1-UL54-1987 | + | UUCUCUCCGACCCCGACACC | 20 | 18976 |
| HSV1-UL54-1988 | + | CUUCUCUCCGACCCCGACACC | 21 | 18977 |
| HSV1-UL54-1989 | + | UCUUCUCUCCGACCCCGACACC | 22 | 18978 |
| HSV1-UL54-1990 | + | AUCUUCUCUCCGACCCCGACACC | 23 | 18979 |
| HSV1-UL54-1991 | + | CAUCUUCUCUCCGACCCCGACACC | 24 | 18980 |
| HSV1-UL54-1992 | + | CCCCGGGGCGGGGUCCCC | 18 | 18981 |
| HSV1-UL54-1993 | + | CGCCCCGGGGCGGGGUCCCC | 20 | 18982 |
| HSV1-UL54-1994 | + | CCGCCCCGGGGCGGGGUCCCC | 21 | 18983 |
| HSV1-UL54-1995 | + | CCCGCCCCGGGGCGGGGUCCCC | 22 | 18984 |
| HSV1-UL54-1996 | + | CCCCGCCCCGGGGCGGGGUCCCC | 23 | 18985 |
| HSV1-UL54-1997 | + | ACCCCGCCCCGGGGCGGGGUCCCC | 24 | 18986 |
| HSV1-UL54-1998 | + | UGGCGGUCGAUGCGGCCC | 18 | 18987 |
| HSV1-UL54-1999 | + | UUGGCGGUCGAUGCGGCCC | 19 | 18988 |
| HSV1-UL54-2000 | + | CUUGGCGGUCGAUGCGGCCC | 20 | 18989 |
| HSV1-UL54-2001 | + | CCUUGGCGGUCGAUGCGGCCC | 21 | 18990 |
| HSV1-UL54-2002 | + | UGGCCUUGGCGGUCGAUGCGGCCC | 24 | 18991 |
| HSV1-UL54-2003 | + | CGCUCCGGGGCCGGGGCGC | 19 | 18992 |
| HSV1-UL54-443 | + | UCGCUCCGGGGCCGGGGCGC | 20 | 7206 |
| HSV1-UL54-2004 | + | UUCGCUCCGGGGCCGGGGCGC | 21 | 18993 |
| HSV1-UL54-2005 | + | UUUCGCUCCGGGGCCGGGGCGC | 22 | 18994 |
| HSV1-UL54-2006 | + | CUUUCGCUCCGGGGCCGGGGCGC | 23 | 18995 |
| HSV1-UL54-2007 | + | CCUUUCGCUCCGGGGCCGGGGCGC | 24 | 18996 |
| HSV1-UL54-1257 | + | UUCUGGGGGCACGCCGGC | 18 | 18246 |
| HSV1-UL54-1258 | + | UGGUUCUGGGGGCACGCCGGC | 21 | 18247 |
| HSV1-UL54-1259 | + | UUGGUUCUGGGGGCACGCCGGC | 22 | 18248 |
| HSV1-UL54-1260 | + | AUUGGUUCUGGGGGCACGCCGGC | 23 | 18249 |
| HSV1-UL54-2008 | + | CGCGGUUGGCGAGCCUGGC | 19 | 18997 |
| HSV1-UL54-2009 | + | ACGCGGUUGGCGAGCCUGGC | 20 | 18998 |
| HSV1-UL54-2010 | + | CGACGCGGUUGGCGAGCCUGGC | 22 | 18999 |
| HSV1-UL54-2011 | + | UCGACGCGGUUGGCGAGCCUGGC | 23 | 19000 |
| HSV1-UL54-2012 | + | CUCGACGCGGUUGGCGAGCCUGGC | 24 | 19001 |
| HSV1-UL54-2013 | + | CUCCUGGCGGUGCGUGUC | 18 | 19002 |
| HSV1-UL54-2014 | + | ACUCCUGGCGGUGCGUGUC | 19 | 19003 |
| HSV1-UL54-2015 | + | CACUCCUGGCGGUGCGUGUC | 20 | 19004 |
| HSV1-UL54-2016 | + | ACACUCCUGGCGGUGCGUGUC | 21 | 19005 |
| HSV1-UL54-2017 | + | AACACUCCUGGCGGUGCGUGUC | 22 | 19006 |
| HSV1-UL54-2018 | + | CGAACACUCCUGGCGGUGCGUGUC | 24 | 19007 |
| HSV1-UL54-2019 | + | UGGCCAGAAUGACAAACACG | 20 | 19008 |
| HSV1-UL54-2020 | + | CUGGCCAGAAUGACAAACACG | 21 | 19009 |
| HSV1-UL54-2021 | + | CCUGGCCAGAAUGACAAACACG | 22 | 19010 |
| HSV1-UL54-2022 | + | AGCCUGGCCAGAAUGACAAACACG | 24 | 19011 |
| HSV1-UL54-2023 | + | CGUGUCCGCUGGCCUCCGG | 19 | 19012 |
| HSV1-UL54-2024 | + | CCGUGUCCGCUGGCCUCCGG | 20 | 19013 |
| HSV1-UL54-2025 | + | CCCGUGUCCGCUGGCCUCCGG | 21 | 19014 |
| HSV1-UL54-2026 | + | CCCCGUGUCCGCUGGCCUCCGG | 22 | 19015 |
| HSV1-UL54-2027 | + | CGCCCCGUGUCCGCUGGCCUCCGG | 24 | 19016 |
| HSV1-UL54-2028 | + | CGCCAGCGUCAUUAGCGGG | 19 | 19017 |
| HSV1-UL54-458 | + | UCGCCAGCGUCAUUAGCGGG | 20 | 7221 |
| HSV1-UL54-2029 | + | AUCGCCAGCGUCAUUAGCGGG | 21 | 19018 |
| HSV1-UL54-2030 | + | AAUCGCCAGCGUCAUUAGCGGG | 22 | 19019 |
| HSV1-UL54-2031 | + | CAAUCGCCAGCGUCAUUAGCGGG | 23 | 19020 |
| HSV1-UL54-2032 | + | CGUCCACCCCGCCCCGGGG | 19 | 19021 |
| HSV1-UL54-480 | + | CCGUCCACCCCGCCCCGGGG | 20 | 7243 |
| HSV1-UL54-2033 | + | UCCGUCCACCCCGCCCCGGGG | 21 | 19022 |
| HSV1-UL54-2034 | + | CGUCCGUCCACCCCGCCCCGGGG | 23 | 19023 |
| HSV1-UL54-2035 | + | CCGUCCGUCCACCCCGCCCCGGGG | 24 | 19024 |
| HSV1-UL54-2036 | + | UGGGGGCGCAGCGGCAGGUUGUG | 23 | 19025 |
| HSV1-UL54-2037 | + | CUGGGGGCGCAGCGGCAGGUUGUG | 24 | 19026 |
| HSV1-UL54-2038 | + | CCCCGUGUCCGCUGGCCU | 18 | 19027 |
| HSV1-UL54-2039 | + | CGCCCCGUGUCCGCUGGCCU | 20 | 19028 |
| HSV1-UL54-2040 | + | ACGCCCCGUGUCCGCUGGCCU | 21 | 19029 |
| HSV1-UL54-2041 | + | CACGCCCCGUGUCCGCUGGCCU | 22 | 19030 |
| HSV1-UL54-2042 | + | CGCACGCCCCGUGUCCGCUGGCCU | 24 | 19031 |
| HSV1-UL54-2043 | - | UCCUAGACACGCACCGCC | 18 | 19032 |
| HSV1-UL54-2044 | - | AUCCUAGACACGCACCGCC | 19 | 19033 |
| HSV1-UL54-343 | - | AAUCCUAGACACGCACCGCC | 20 | 7106 |
| HSV1-UL54-2045 | - | AAAUCCUAGACACGCACCGCC | 21 | 19034 |
| HSV1-UL54-2046 | - | CGAAAUCCUAGACACGCACCGCC | 23 | 19035 |
| HSV1-UL54-2047 | - | UCGAAAUCCUAGACACGCACCGCC | 24 | 19036 |
| HSV1-UL54-2048 | - | UGGGGGGCAGCCGUUUCCCGCC | 22 | 19037 |
| HSV1-UL54-2049 | - | UUGGGGGGCAGCCGUUUCCCGCC | 23 | 19038 |
| HSV1-UL54-2050 | - | UUUGGGGGGCAGCCGUUUCCCGCC | 24 | 19039 |
| HSV1-UL54-2051 | - | CUUUGACGCCGAGACCAG | 18 | 19040 |
| HSV1-UL54-2052 | - | CCUUUGACGCCGAGACCAG | 19 | 19041 |
| HSV1-UL54-942 | - | CCCUUUGACGCCGAGACCAG | 20 | 7608 |
| HSV1-UL54-2053 | - | AGGGCCCUUUGACGCCGAGACCAG | 24 | 19042 |
| HSV1-UL54-257 | - | UGGGGGACCCCGCCCCGGGG | 20 | 7020 |
| HSV1-UL54-2054 | - | CUGGGGGACCCCGCCCCGGGG | 21 | 19043 |
| HSV1-UL54-2055 | - | CCUGGGGGACCCCGCCCCGGGG | 22 | 19044 |
| HSV1-UL54-2056 | - | CCCUGGGGGACCCCGCCCCGGGG | 23 | 19045 |
| HSV1-UL54-2057 | - | ACCCUGGGGGACCCCGCCCCGGGG | 24 | 19046 |
| HSV1-UL54-2058 | - | CGGGACGACCGCGGCUGUG | 19 | 19047 |
| HSV1-UL54-954 | - | UCGGGACGACCGCGGCUGUG | 20 | 7617 |
| HSV1-UL54-2059 | - | AUCGGGACGACCGCGGCUGUG | 21 | 19048 |
| HSV1-UL54-2060 | - | UAUCGGGACGACCGCGGCUGUG | 22 | 19049 |
| HSV1-UL54-2061 | - | UUAUCGGGACGACCGCGGCUGUG | 23 | 19050 |
| HSV1-UL54-2062 | - | AUUAUCGGGACGACCGCGGCUGUG | 24 | 19051 |
| HSV1-UL54-2063 | - | AGUGUUCGAGUCGUGUCU | 18 | 19052 |
| HSV1-UL54-2064 | - | AGGAGUGUUCGAGUCGUGUCU | 21 | 19053 |
| HSV1-UL54-2065 | - | CAGGAGUGUUC GAGUC GUGUCU | 22 | 19054 |
| HSV1-UL54-2066 | - | CCAGGAGUGUUCGAGUCGUGUCU | 23 | 19055 |
| HSV1-UL54-2067 | - | ACGCACCGCCAGGAGUGU | 18 | 19056 |
| HSV1-UL54-2068 | - | CACGCACCGCCAGGAGUGU | 19 | 19057 |
| HSV1-UL54-976 | - | ACACGCACCGCCAGGAGUGU | 20 | 7634 |
| HSV1-UL54-2069 | - | AGACACGCACCGCCAGGAGUGU | 22 | 19058 |
| HSV1-UL54-2070 | - | UAGACACGCACCGCCAGGAGUGU | 23 | 19059 |
| HSV1-UL54-2071 | - | CUAGACACGCACCGCCAGGAGUGU | 24 | 19060 |
| HSV1-UL54-2072 | - | CUACGCGACCCUUGGUGU | 18 | 19061 |
| HSV1-UL54-2073 | - | ACUACGCGACCCUUGGUGU | 19 | 19062 |
| HSV1-UL54-2074 | - | CGACUACGCGACCCUUGGUGU | 21 | 19063 |
| HSV1-UL54-2075 | - | UCGACUACGCGACCCUUGGUGU | 22 | 19064 |
| HSV1-UL54-2076 | - | AUCGACUACGCGACCCUUGGUGU | 23 | 19065 |

**Table 15G** provides exemplary targeting domains for knocking out the *UL54* gene selected according to the seven tier parameters. The targeting domains fall in the coding sequence of the gene, downstream of the first 500bp of coding sequence (e.g., anywhere from +500 (relative to the start codon) to the stop codon of the gene). The PAM is NNGRRV. It is contemplated herein that in an embodiment the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase).

**Table 15G**

| 7th Tier | | | | |
|---|---|---|---|---|
| gRNA Name | DNA Strand | Targeting Domain | Target Site Length | Seq ID NO: |
| HSV1-UL54-2077 | + | UUUUUAUUGUACCUAAAA | 18 | 19066 |
| HSV1-UL54-2078 | + | GUUUUUAUUGUACCUAAAA | 19 | 19067 |
| HSV1-UL54-2079 | + | UGUUUUUAUUGUACCUAAAA | 20 | 19068 |
| HSV1-UL54-2080 | + | UUGUUUUUAUUGUACCUAAAA | 21 | 19069 |
| HSV1-UL54-2081 | + | UUUGUUUUUAUUGUACCUAAAA | 22 | 19070 |
| HSV1-UL54-2082 | + | UUUUGUUUUUAUUGUACCUAAAA | 23 | 19071 |
| HSV1-UL54-2083 | + | GUUUUGUUUUUAUUGUACCUAAAA | 24 | 19072 |
| HSV1-UL54-2084 | + | GGAAACGGCUGCCCCCCA | 18 | 19073 |
| HSV1-UL54-2085 | + | GGGAAACGGCUGCCCCCCA | 19 | 19074 |
| HSV1-UL54-2086 | + | CGGGAAACGGCUGCCCCCCA | 20 | 19075 |
| HSV1-UL54-2087 | + | GCGGGAAACGGCUGCCCCCCA | 21 | 19076 |
| HSV1-UL54-2088 | + | GGCGGGAAACGGCUGCCCCCCA | 22 | 19077 |
| HSV1-UL54-2089 | + | CGGCGGGAAACGGCUGCCCCCCA | 23 | 19078 |
| HSV1-UL54-2090 | + | GCGGCGGGAAACGGCUGCCCCCCA | 24 | 19079 |
| HSV1-UL54-2091 | + | CCGCGCCACGCCUCGCCA | 18 | 19080 |
| HSV1-UL54-2092 | + | GCCGCGCCACGCCUCGCCA | 19 | 19081 |
| HSV1-UL54-469 | + | UGCCGCGCCACGCCUCGCCA | 20 | 7232 |
| HSV1-UL54-2093 | + | CUGCCGCGCCACGCCUCGCCA | 21 | 19082 |
| HSV1-UL54-2094 | + | ACUGCCGCGCCACGCCUCGCCA | 22 | 19083 |
| HSV1-UL54-2095 | + | CACUGCCGCGCCACGCCUCGCCA | 23 | 19084 |
| HSV1-UL54-2096 | + | UCACUGCCGCGCCACGCCUCGCCA | 24 | 19085 |
| HSV1-UL54-2097 | + | CUCCUUGGCCCGCCAGCA | 18 | 19086 |
| HSV1-UL54-2098 | + | CCUCCUUGGCCCGCCAGCA | 19 | 19087 |
| HSV1-UL54-2099 | + | CCCUCCUUGGCCCGCCAGCA | 20 | 19088 |
| HSV1-UL54-2100 | + | GCCCUCCUUGGCCCGCCAGCA | 21 | 19089 |
| HSV1-UL54-2101 | + | GGCCCUCCUUGGCCCGCCAGCA | 22 | 19090 |
| HSV1-UL54-2102 | + | GGGCCCUCCUUGGCCCGCCAGCA | 23 | 19091 |
| HSV1-UL54-2103 | + | AGGGCCCUCCUUGGCCCGCCAGCA | 24 | 19092 |
| HSV1-UL54-2104 | + | UCGCGCCUUCAGGUAGCA | 18 | 19093 |
| HSV1-UL54-2105 | + | CUCGCGCCUUCAGGUAGCA | 19 | 19094 |
| HSV1-UL54-2106 | + | CCUCGCGCCUUCAGGUAGCA | 20 | 19095 |
| HSV1-UL54-2107 | + | GCCUCGCGCCUUCAGGUAGCA | 21 | 19096 |
| HSV1-UL54-2108 | + | GGCCUCGCGCCUUCAGGUAGCA | 22 | 19097 |
| HSV1-UL54-2109 | + | AGGCCUCGCGCCUUCAGGUAGCA | 23 | 19098 |
| HSV1-UL54-2110 | + | CAGGCCUCGCGCCUUCAGGUAGCA | 24 | 19099 |
| HSV1-UL54-2111 | + | CGUCUGGUCUCGGCGUCA | 18 | 19100 |
| HSV1-UL54-2112 | + | CCGUCUGGUCUCGGCGUCA | 19 | 19101 |
| HSV1-UL54-2113 | + | CCCGUCUGGUCUCGGCGUCA | 20 | 19102 |
| HSV1-UL54-2114 | + | ACCCGUCUGGUCUCGGCGUCA | 21 | 19103 |
| HSV1-UL54-2115 | + | GACCCGUCUGGUCUCGGCGUCA | 22 | 19104 |
| HSV1-UL54-2116 | + | AGACCCGUCUGGUCUCGGCGUCA | 23 | 19105 |
| HSV1-UL54-2117 | + | GAGACCCGUCUGGUCUCGGCGUCA | 24 | 19106 |
| HSV1-UL54-2118 | + | CCGCCGCGCGCUCGGAGA | 18 | 19107 |
| HSV1-UL54-2119 | + | ACCGCCGCGCGCUCGGAGA | 19 | 19108 |
| HSV1-UL54-427 | + | GACCGCCGCGCGCUCGGAGA | 20 | 7190 |
| HSV1-UL54-2120 | + | CGACCGCCGCGCGCUCGGAGA | 21 | 19109 |
| HSV1-UL54-2121 | + | UCGACCGCCGCGCGCUCGGAGA | 22 | 19110 |
| HSV1-UL54-2122 | + | GUCGACCGCCGCGCGCUCGGAGA | 23 | 19111 |
| HSV1-UL54-2123 | + | GGUCGACCGCCGCGCGCUCGGAGA | 24 | 19112 |
| HSV1-UL54-2124 | + | UUCAGGUAGCACUGGAGA | 18 | 19113 |
| HSV1-UL54-2125 | + | CUUCAGGUAGCACUGGAGA | 19 | 19114 |
| HSV1-UL54-2126 | + | CCUUCAGGUAGCACUGGAGA | 20 | 19115 |
| HSV1-UL54-2127 | + | GCCUUCAGGUAGCACUGGAGA | 21 | 19116 |
| HSV1-UL54-2128 | + | CGCCUUCAGGUAGCACUGGAGA | 22 | 19117 |
| HSV1-UL54-2129 | + | GCGCCUUCAGGUAGCACUGGAGA | 23 | 19118 |
| HSV1-UL54-2130 | + | CGCGCCUUCAGGUAGCACUGGAGA | 24 | 19119 |
| HSV1-UL54-2131 | + | UUGCCGGCAAAAGUGCGA | 18 | 19120 |
| HSV1-UL54-2132 | + | AUUGCCGGCAAAAGUGCGA | 19 | 19121 |
| HSV1-UL54-2133 | + | GAUUGCCGGCAAAAGUGCGA | 20 | 19122 |
| HSV1-UL54-2134 | + | GGAUUGCCGGCAAAAGUGCGA | 21 | 19123 |
| HSV1-UL54-2135 | + | AGGAUUGCCGGCAAAAGUGCGA | 22 | 19124 |
| HSV1-UL54-2136 | + | GAGGAUUGCCGGCAAAAGUGCGA | 23 | 19125 |
| HSV1-UL54-2137 | + | CGAGGAUUGCCGGCAAAAGUGCGA | 24 | 19126 |
| HSV1-UL54-2138 | + | GCCGCGGUCGUCCCGAUA | 18 | 19127 |
| HSV1-UL54-2139 | + | AGCCGCGGUCGUCCCGAUA | 19 | 19128 |
| HSV1-UL54-2140 | + | CAGCCGCGGUCGUCCCGAUA | 20 | 19129 |
| HSV1-UL54-2141 | + | ACAGCCGCGGUCGUCCCGAUA | 21 | 19130 |
| HSV1-UL54-2142 | + | CACAGCCGCGGUCGUCCCGAUA | 22 | 19131 |
| HSV1-UL54-2143 | + | GCACAGCCGCGGUCGUCCCGAUA | 23 | 19132 |
| HSV1-UL54-2144 | + | AGCACAGCCGCGGUCGUCCCGAUA | 24 | 19133 |
| HSV1-UL54-2145 | + | AUAUUUGCCGUGCACGUA | 18 | 19134 |
| HSV1-UL54-2146 | + | AAUAUUUGCCGUGCACGUA | 19 | 19135 |
| HSV1-UL54-346 | + | AAAUAUUUGCCGUGCACGUA | 20 | 7109 |
| HSV1-UL54-2147 | + | AAAAUAUUUGCCGUGCACGUA | 21 | 19136 |
| HSV1-UL54-2148 | + | AAAAAUAUUUGCCGUGCACGUA | 22 | 19137 |
| HSV1-UL54-2149 | + | UAAAAAUAUUUGCCGUGCACGUA | 23 | 19138 |
| HSV1-UL54-2150 | + | AUAAAAAUAUUUGCCGUGCACGUA | 24 | 19139 |
| HSV1-UL54-2151 | + | CAAUCGCCAGCGUCAUUA | 18 | 19140 |
| HSV1-UL54-2152 | + | GCAAUCGCCAGCGUCAUUA | 19 | 19141 |
| HSV1-UL54-2153 | + | GGCAAUCGCCAGCGUCAUUA | 20 | 19142 |
| HSV1-UL54-2154 | + | GGGCAAUCGCCAGCGUCAUUA | 21 | 19143 |
| HSV1-UL54-2155 | + | GGGGCAAUCGCCAGCGUCAUUA | 22 | 19144 |
| HSV1-UL54-2156 | + | GGGGGCAAUCGCCAGCGUCAUUA | 23 | 19145 |
| HSV1-UL54-2157 | + | GGGGGGCAAUCGCCAGCGUCAUUA | 24 | 19146 |
| HSV1-UL54-2158 | + | UUUUAUUGUACCUAAAAC | 18 | 19147 |
| HSV1-UL54-2159 | + | UUUUUAUUGUACCUAAAAC | 19 | 19148 |
| HSV1-UL54-1226 | + | GUUUUUAUUGUACCUAAAAC | 20 | 18215 |
| HSV1-UL54-2160 | + | UGUUUUUAUUGUACCUAAAAC | 21 | 19149 |
| HSV1-UL54-2161 | + | UUGUUUUUAUUGUACCUAAAAC | 22 | 19150 |
| HSV1-UL54-2162 | + | UUUGUUUUUAUUGUACCUAAAAC | 23 | 19151 |
| HSV1-UL54-2163 | + | UUUUGUUUUUAUUGUACCUAAAAC | 24 | 19152 |
| HSV1-UL54-2164 | + | CUGGCCAGAAUGACAAAC | 18 | 19153 |
| HSV1-UL54-2165 | + | CCUGGCCAGAAUGACAAAC | 19 | 19154 |
| HSV1-UL54-2166 | + | GCCUGGCCAGAAUGACAAAC | 20 | 19155 |
| HSV1-UL54-2167 | + | AGCCUGGCCAGAAUGACAAAC | 21 | 19156 |
| HSV1-UL54-2168 | + | GAGCCUGGCCAGAAUGACAAAC | 22 | 19157 |
| HSV1-UL54-2169 | + | CGAGCCUGGCCAGAAUGACAAAC | 23 | 19158 |
| HSV1-UL54-2170 | + | GCGAGCCUGGCCAGAAUGACAAAC | 24 | 19159 |
| HSV1-UL54-2171 | + | UCCUUGGCCCGCCAGCAC | 18 | 19160 |
| HSV1-UL54-2172 | + | CUCCUUGGCCCGCCAGCAC | 19 | 19161 |
| HSV1-UL54-411 | + | CCUCCUUGGCCCGCCAGCAC | 20 | 7174 |
| HSV1-UL54-2173 | + | CCCUCCUUGGCCCGCCAGCAC | 21 | 19162 |
| HSV1-UL54-2174 | + | GCCCUCCUUGGCCCGCCAGCAC | 22 | 19163 |
| HSV1-UL54-2175 | + | GGCCCUCCUUGGCCCGCCAGCAC | 23 | 19164 |
| HSV1-UL54-2176 | + | GGGCCCUCCUUGGCCCGCCAGCAC | 24 | 19165 |
| HSV1-UL54-2177 | + | CGCGCCUUCAGGUAGCAC | 18 | 19166 |
| HSV1-UL54-2178 | + | UCGCGCCUUCAGGUAGCAC | 19 | 19167 |
| HSV1-UL54-371 | + | CUCGCGCCUUCAGGUAGCAC | 20 | 7134 |
| HSV1-UL54-2179 | + | CCUCGCGCCUUCAGGUAGCAC | 21 | 19168 |
| HSV1-UL54-2180 | + | GCCUCGCGCCUUCAGGUAGCAC | 22 | 19169 |
| HSV1-UL54-2181 | + | GGCCUCGCGCCUUCAGGUAGCAC | 23 | 19170 |
| HSV1-UL54-2182 | + | AGGCCUCGCGCCUUCAGGUAGCAC | 24 | 19171 |
| HSV1-UL54-2183 | + | UCAACUCGCAGACACGAC | 18 | 19172 |
| HSV1-UL54-2184 | + | GUCAACUCGCAGACACGAC | 19 | 19173 |
| HSV1-UL54-2185 | + | CGUCAACUCGCAGACACGAC | 20 | 19174 |
| HSV1-UL54-2186 | + | CCGUCAACUCGCAGACACGAC | 21 | 19175 |
| HSV1-UL54-2187 | + | GCCGUCAACUCGCAGACACGAC | 22 | 19176 |
| HSV1-UL54-2188 | + | GGCCGUCAACUCGCAGACACGAC | 23 | 19177 |
| HSV1-UL54-2189 | + | UGGCCGUCAACUCGCAGACACGAC | 24 | 19178 |
| HSV1-UL54-2190 | + | UAUUUGCCGUGCACGUAC | 18 | 19179 |
| HSV1-UL54-2191 | + | AUAUUUGCCGUGCACGUAC | 19 | 19180 |
| HSV1-UL54-347 | + | AAUAUUUGCCGUGCACGUAC | 20 | 7110 |
| HSV1-UL54-2192 | + | AAAUAUUUGCCGUGCACGUAC | 21 | 19181 |
| HSV1-UL54-2193 | + | AAAAUAUUUGCC GUGCAC GUAC | 22 | 19182 |
| HSV1-UL54-2194 | + | AAAAAUAUUUGCC GUGCAC GUAC | 23 | 19183 |
| HSV1-UL54-2195 | + | UAAAAAUAUUUGCC GUGCAC GUAC | 24 | 19184 |
| HSV1-UL54-2196 | + | GCCCGCCAUGCAGGCCCC | 18 | 19185 |
| HSV1-UL54-2197 | + | GGCCCGCCAUGCAGGCCCC | 19 | 19186 |
| HSV1-UL54-359 | + | AGGCCCGCCAUGCAGGCCCC | 20 | 7122 |
| HSV1-UL54-2198 | + | CAGGCCCGCCAUGCAGGCCCC | 21 | 19187 |
| HSV1-UL54-2199 | + | UCAGGCCCGCCAUGCAGGCCCC | 22 | 19188 |
| HSV1-UL54-2200 | + | AUCAGGCCCGCCAUGCAGGCCCC | 23 | 19189 |
| HSV1-UL54-2201 | + | GAUCAGGCCCGCCAUGCAGGCCCC | 24 | 19190 |
| HSV1-UL54-2202 | + | CGUCCGUCCACCCCGCCC | 18 | 19191 |
| HSV1-UL54-2203 | + | CCGUCCGUCCACCCCGCCC | 19 | 19192 |
| HSV1-UL54-477 | + | GCCGUCCGUCCACCCCGCCC | 20 | 7240 |
| HSV1-UL54-2204 | + | GGCCGUCCGUCCACCCCGCCC | 21 | 19193 |
| HSV1-UL54-2205 | + | GGGCCGUCCGUCCACCCCGCCC | 22 | 19194 |
| HSV1-UL54-2206 | + | GGGGCCGUCCGUCCACCCCGCCC | 23 | 19195 |
| HSV1-UL54-2207 | + | CGGGGCCGUCCGUCCACCCCGCCC | 24 | 19196 |
| HSV1-UL54-2208 | + | GGCCCGCCAUGCAGGCCC | 18 | 19197 |
| HSV1-UL54-2209 | + | AGGCCCGCCAUGCAGGCCC | 19 | 19198 |
| HSV1-UL54-358 | + | CAGGCCCGCCAUGCAGGCCC | 20 | 7121 |
| HSV1-UL54-2210 | + | UCAGGCCCGCCAUGCAGGCCC | 21 | 19199 |
| HSV1-UL54-2211 | + | AUCAGGCCCGCCAUGCAGGCCC | 22 | 19200 |
| HSV1-UL54-2212 | + | GAUCAGGCCCGCCAUGCAGGCCC | 23 | 19201 |
| HSV1-UL54-2213 | + | CGAUCAGGCCCGCCAUGCAGGCCC | 24 | 19202 |
| HSV1-UL54-2214 | + | CCGCCAGCACCGGGGCCC | 18 | 19203 |
| HSV1-UL54-2215 | + | CCCGCCAGCACCGGGGCCC | 19 | 19204 |
| HSV1-UL54-414 | + | GCCCGCCAGCACCGGGGCCC | 20 | 7177 |
| HSV1-UL54-2216 | + | GGCCCGCCAGCACCGGGGCCC | 21 | 19205 |
| HSV1-UL54-2217 | + | UGGCCCGCCAGCACCGGGGCCC | 22 | 19206 |
| HSV1-UL54-2218 | + | UUGGCCCGCCAGCACCGGGGCCC | 23 | 19207 |
| HSV1-UL54-2219 | + | CUUGGCCCGCCAGCACCGGGGCCC | 24 | 19208 |
| HSV1-UL54-2220 | + | GGGCGACCAAGGUUUCCC | 18 | 19209 |
| HSV1-UL54-2221 | + | UGGGCGACCAAGGUUUCCC | 19 | 19210 |
| HSV1-UL54-405 | + | GUGGGCGACCAAGGUUUCCC | 20 | 7168 |
| HSV1-UL54-2222 | + | CGUGGGCGACCAAGGUUUCCC | 21 | 19211 |
| HSV1-UL54-2223 | + | CCGUGGGCGACCAAGGUUUCCC | 22 | 19212 |
| HSV1-UL54-2224 | + | GCCGUGGGCGACCAAGGUUUCCC | 23 | 19213 |
| HSV1-UL54-2225 | + | GGCCGUGGGCGACCAAGGUUUCCC | 24 | 19214 |
| HSV1-UL54-2226 | + | CCGUCCGUCCACCCCGCC | 18 | 19215 |
| HSV1-UL54-2227 | + | GCCGUCCGUCCACCCCGCC | 19 | 19216 |
| HSV1-UL54-2228 | + | GGCCGUCCGUCCACCCCGCC | 20 | 19217 |
| HSV1-UL54-2229 | + | GGGCCGUCCGUCCACCCCGCC | 21 | 19218 |
| HSV1-UL54-2230 | + | GGGGCCGUCCGUCCACCCCGCC | 22 | 19219 |
| HSV1-UL54-2231 | + | CGGGGCCGUCCGUCCACCCCGCC | 23 | 19220 |
| HSV1-UL54-2232 | + | CCGGGGCCGUCCGUCCACCCCGCC | 24 | 19221 |
| HSV1-UL54-2233 | + | UGUCCGCCAGACGCCGCC | 18 | 19222 |
| HSV1-UL54-2234 | + | AUGUCCGCCAGACGCCGCC | 19 | 19223 |
| HSV1-UL54-995 | + | AAUGUCCGCCAGACGCCGCC | 20 | 7651 |
| HSV1-UL54-2235 | + | UAAUGUCCGCCAGACGCCGCC | 21 | 19224 |
| HSV1-UL54-2236 | + | UUAAUGUCCGCCAGACGCCGCC | 22 | 19225 |
| HSV1-UL54-2237 | + | CUUAAUGUCCGCCAGACGCCGCC | 23 | 19226 |
| HSV1-UL54-2238 | + | CCUUAAUGUCCGCCAGACGCCGCC | 24 | 19227 |
| HSV1-UL54-2239 | + | GCCGCGCCACGCCUCGCC | 18 | 19228 |
| HSV1-UL54-2240 | + | UGCCGCGCCACGCCUCGCC | 19 | 19229 |
| HSV1-UL54-2241 | + | CUGCCGCGCCACGCCUCGCC | 20 | 19230 |
| HSV1-UL54-2242 | + | ACUGCCGCGCCACGCCUCGCC | 21 | 19231 |
| HSV1-UL54-2243 | + | CACUGCCGCGCCACGCCUCGCC | 22 | 19232 |
| HSV1-UL54-2244 | + | UCACUGCCGCGCCACGCCUCGCC | 23 | 19233 |
| HSV1-UL54-2245 | + | CUCACUGCCGCGCCACGCCUCGCC | 24 | 19234 |
| HSV1-UL54-2246 | + | AGGCCCGCCAUGCAGGCC | 18 | 19235 |
| HSV1-UL54-2247 | + | CAGGCCCGCCAUGCAGGCC | 19 | 19236 |
| HSV1-UL54-984 | + | UCAGGCCCGCCAUGCAGGCC | 20 | 7641 |
| HSV1-UL54-2248 | + | AUCAGGCCCGCCAUGCAGGCC | 21 | 19237 |
| HSV1-UL54-2249 | + | GAUCAGGCCCGCCAUGCAGGCC | 22 | 19238 |
| HSV1-UL54-2250 | + | CGAUCAGGCCCGCCAUGCAGGCC | 23 | 19239 |
| HSV1-UL54-2251 | + | UCGAUCAGGCCCGCCAUGCAGGCC | 24 | 19240 |
| HSV1-UL54-2252 | + | GCGAUAGAGGCUCGGGCC | 18 | 19241 |
| HSV1-UL54-2253 | + | UGCGAUAGAGGCUCGGGCC | 19 | 19242 |
| HSV1-UL54-2254 | + | GUGCGAUAGAGGCUCGGGCC | 20 | 19243 |
| HSV1-UL54-2255 | + | AGUGCGAUAGAGGCUCGGGCC | 21 | 19244 |
| HSV1-UL54-2256 | + | AAGUGCGAUAGAGGCUCGGGCC | 22 | 19245 |
| HSV1-UL54-2257 | + | AAAGUGCGAUAGAGGCUCGGGCC | 23 | 19246 |
| HSV1-UL54-2258 | + | AAAAGUGCGAUAGAGGCUCGGGCC | 24 | 19247 |
| HSV1-UL54-2259 | + | CCCGCCAGCACCGGGGCC | 18 | 19248 |
| HSV1-UL54-2260 | + | GCCCGCCAGCACCGGGGCC | 19 | 19249 |
| HSV1-UL54-1017 | + | GGCCCGCCAGCACCGGGGCC | 20 | 7673 |
| HSV1-UL54-2261 | + | UGGCCCGCCAGCACCGGGGCC | 21 | 19250 |
| HSV1-UL54-2262 | + | UUGGCCCGCCAGCACCGGGGCC | 22 | 19251 |
| HSV1-UL54-2263 | + | CUUGGCCCGCCAGCACCGGGGCC | 23 | 19252 |
| HSV1-UL54-2264 | + | CCUUGGCCCGCCAGCACCGGGGCC | 24 | 19253 |
| HSV1-UL54-2265 | + | GCCGGGGCGCGGGGGUCC | 18 | 19254 |
| HSV1-UL54-2266 | + | GGCCGGGGCGCGGGGGUCC | 19 | 19255 |
| HSV1-UL54-1029 | + | GGGCCGGGGCGCGGGGGUCC | 20 | 7684 |
| HSV1-UL54-2267 | + | GGGGCCGGGGCGCGGGGGUCC | 21 | 19256 |
| HSV1-UL54-2268 | + | CGGGGCCGGGGCGCGGGGGUCC | 22 | 19257 |
| HSV1-UL54-2269 | + | CCGGGGCCGGGGCGCGGGGGUCC | 23 | 19258 |
| HSV1-UL54-2270 | + | UCCGGGGCCGGGGCGCGGGGGUCC | 24 | 19259 |
| HSV1-UL54-2271 | + | GUCCCGAUAAUGGGGUCC | 18 | 19260 |
| HSV1-UL54-2272 | + | CGUCCCGAUAAUGGGGUCC | 19 | 19261 |
| HSV1-UL54-381 | + | UCGUCCCGAUAAUGGGGUCC | 20 | 7144 |
| HSV1-UL54-2273 | + | GUCGUCCCGAUAAUGGGGUCC | 21 | 19262 |
| HSV1-UL54-2274 | + | GGUCGUCCCGAUAAUGGGGUCC | 22 | 19263 |
| HSV1-UL54-2275 | + | CGGUCGUCCCGAUAAUGGGGUCC | 23 | 19264 |
| HSV1-UL54-2276 | + | GCGGUCGUCCCGAUAAUGGGGUCC | 24 | 19265 |
| HSV1-UL54-2277 | + | UGGGCGACCAAGGUUUCC | 18 | 19266 |
| HSV1-UL54-2278 | + | GUGGGCGACCAAGGUUUCC | 19 | 19267 |
| HSV1-UL54-2279 | + | CGUGGGCGACCAAGGUUUCC | 20 | 19268 |
| HSV1-UL54-2280 | + | CCGUGGGCGACCAAGGUUUCC | 21 | 19269 |
| HSV1-UL54-2281 | + | GCCGUGGGCGACCAAGGUUUCC | 22 | 19270 |
| HSV1-UL54-2282 | + | GGCCGUGGGCGACCAAGGUUUCC | 23 | 19271 |
| HSV1-UL54-2283 | + | GGGCCGUGGGCGACCAAGGUUUCC | 24 | 19272 |
| HSV1-UL54-2284 | + | AUCGCCAGCGUCAUUAGC | 18 | 19273 |
| HSV1-UL54-2285 | + | AAUCGCCAGCGUCAUUAGC | 19 | 19274 |
| HSV1-UL54-455 | + | CAAUCGCCAGCGUCAUUAGC | 20 | 7218 |
| HSV1-UL54-2286 | + | GCAAUCGCCAGCGUCAUUAGC | 21 | 19275 |
| HSV1-UL54-2287 | + | GGCAAUCGCCAGCGUCAUUAGC | 22 | 19276 |
| HSV1-UL54-2288 | + | GGGCAAUCGCCAGCGUCAUUAGC | 23 | 19277 |
| HSV1-UL54-2289 | + | GGGGCAAUCGCCAGCGUCAUUAGC | 24 | 19278 |
| HSV1-UL54-2290 | + | CGGUCGACCGCCGCGCGC | 18 | 19279 |
| HSV1-UL54-2291 | + | GCGGUCGACCGCCGCGCGC | 19 | 19280 |
| HSV1-UL54-2292 | + | UGCGGUCGACCGCCGCGCGC | 20 | 19281 |
| HSV1-UL54-2293 | + | AUGCGGUCGACCGCCGCGCGC | 21 | 19282 |
| HSV1-UL54-2294 | + | GAUGCGGUCGACCGCCGCGCGC | 22 | 19283 |
| HSV1-UL54-2295 | + | UGAUGCGGUCGACCGCCGCGCGC | 23 | 19284 |
| HSV1-UL54-2296 | + | CUGAUGCGGUCGACCGCCGCGCGC | 24 | 19285 |
| HSV1-UL54-2297 | + | CCUCGCCAUGGGGGGCGC | 18 | 19286 |
| HSV1-UL54-2298 | + | GCCUCGCCAUGGGGGGCGC | 19 | 19287 |
| HSV1-UL54-474 | + | CGCCUCGCCAUGGGGGGCGC | 20 | 7237 |
| HSV1-UL54-2299 | + | ACGCCUCGCCAUGGGGGGCGC | 21 | 19288 |
| HSV1-UL54-2300 | + | CACGCCUCGCCAUGGGGGGCGC | 22 | 19289 |
| HSV1-UL54-2301 | + | CCACGCCUCGCCAUGGGGGGCGC | 23 | 19290 |
| HSV1-UL54-2302 | + | GCCACGCCUCGCCAUGGGGGGCGC | 24 | 19291 |
| HSV1-UL54-2303 | + | CAAAAGUGCGAUAGAGGC | 18 | 19292 |
| HSV1-UL54-2304 | + | GCAAAAGUGCGAUAGAGGC | 19 | 19293 |
| HSV1-UL54-2305 | + | GGCAAAAGUGCGAUAGAGGC | 20 | 19294 |
| HSV1-UL54-2306 | + | CGGCAAAAGUGCGAUAGAGGC | 21 | 19295 |
| HSV1-UL54-2307 | + | CCGGCAAAAGUGCGAUAGAGGC | 22 | 19296 |
| HSV1-UL54-2308 | + | GCCGGCAAAAGUGCGAUAGAGGC | 23 | 19297 |
| HSV1-UL54-2309 | + | UGCCGGCAAAAGUGCGAUAGAGGC | 24 | 19298 |
| HSV1-UL54-2310 | + | CCAGGGGCUAUUCGCGGC | 18 | 19299 |
| HSV1-UL54-2311 | + | CCCAGGGGCUAUUCGCGGC | 19 | 19300 |
| HSV1-UL54-419 | + | GCCCAGGGGCUAUUCGCGGC | 20 | 7182 |
| HSV1-UL54-2312 | + | GGCCCAGGGGCUAUUCGCGGC | 21 | 19301 |
| HSV1-UL54-2313 | + | GGGCCCAGGGGCUAUUCGCGGC | 22 | 19302 |
| HSV1-UL54-2314 | + | GGGGCCCAGGGGCUAUUCGCGGC | 23 | 19303 |
| HSV1-UL54-2315 | + | CGGGGCCCAGGGGCUAUUCGCGGC | 24 | 19304 |
| HSV1-UL54-2316 | + | CUUGGCGGUCGAUGCGGC | 18 | 19305 |
| HSV1-UL54-2317 | + | CCUUGGCGGUCGAUGCGGC | 19 | 19306 |
| HSV1-UL54-2318 | + | GCCUUGGCGGUCGAUGCGGC | 20 | 19307 |
| HSV1-UL54-2319 | + | GGCCUUGGCGGUCGAUGCGGC | 21 | 19308 |
| HSV1-UL54-2320 | + | UGGCCUUGGCGGUCGAUGCGGC | 22 | 19309 |
| HSV1-UL54-2321 | + | AUGGCCUUGGCGGUCGAUGCGGC | 23 | 19310 |
| HSV1-UL54-2322 | + | CAUGGCCUUGGCGGUCGAUGCGGC | 24 | 19311 |
| HSV1-UL54-2323 | + | GCGCGGGGGUCCGCGGGC | 18 | 19312 |
| HSV1-UL54-2324 | + | GGCGCGGGGGUCCGCGGGC | 19 | 19313 |
| HSV1-UL54-449 | + | GGGCGCGGGGGUCCGCGGGC | 20 | 7212 |
| HSV1-UL54-2325 | + | GGGGCGCGGGGGUCCGCGGGC | 21 | 19314 |
| HSV1-UL54-2326 | + | CGGGGCGCGGGGGUCCGCGGGC | 22 | 19315 |
| HSV1-UL54-2327 | + | CCGGGGCGCGGGGGUCCGCGGGC | 23 | 19316 |
| HSV1-UL54-2328 | + | GCCGGGGCGCGGGGGUCCGCGGGC | 24 | 19317 |
| HSV1-UL54-2329 | + | UCGCUCCGGGGCCGGGGC | 18 | 19318 |
| HSV1-UL54-2330 | + | UUCGCUCCGGGGCCGGGGC | 19 | 19319 |
| HSV1-UL54-2331 | + | UUUCGCUCCGGGGCCGGGGC | 20 | 19320 |
| HSV1-UL54-2332 | + | CUUUCGCUCCGGGGCCGGGGC | 21 | 19321 |
| HSV1-UL54-2333 | + | CCUUUCGCUCCGGGGCCGGGGC | 22 | 19322 |
| HSV1-UL54-2334 | + | GCCUUUCGCUCCGGGGCCGGGGC | 23 | 19323 |
| HSV1-UL54-2335 | + | CGCCUUUCGCUCCGGGGCCGGGGC | 24 | 19324 |
| HSV1-UL54-2336 | + | CGCCUUUCGCUCCGGGGC | 18 | 19325 |
| HSV1-UL54-2337 | + | GCGCCUUUCGCUCCGGGGC | 19 | 19326 |
| HSV1-UL54-439 | + | GGCGCCUUUCGCUCCGGGGC | 20 | 7202 |
| HSV1-UL54-2338 | + | GGGCGCCUUUCGCUCCGGGGC | 21 | 19327 |
| HSV1-UL54-2339 | + | CGGGCGCCUUUCGCUCCGGGGC | 22 | 19328 |
| HSV1-UL54-2340 | + | GCGGGCGCCUUUCGCUCCGGGGC | 23 | 19329 |
| HSV1-UL54-2341 | + | GGCGGGCGCCUUUCGCUCCGGGGC | 24 | 19330 |
| HSV1-UL54-2342 | + | GGCGGGCGCCUUUCGCUC | 18 | 19331 |
| HSV1-UL54-2343 | + | CGGCGGGCGCCUUUCGCUC | 19 | 19332 |
| HSV1-UL54-436 | + | GCGGCGGGCGCCUUUCGCUC | 20 | 7199 |
| HSV1-UL54-2344 | + | GGCGGCGGGCGCCUUUCGCUC | 21 | 19333 |
| HSV1-UL54-2345 | + | CGGCGGCGGGCGCCUUUCGCUC | 22 | 19334 |
| HSV1-UL54-2346 | + | UCGGCGGCGGGCGCCUUUCGCUC | 23 | 19335 |
| HSV1-UL54-2347 | + | GUCGGCGGCGGGCGCCUUUCGCUC | 24 | 19336 |
| HSV1-UL54-2348 | + | CGUCCCGAUAAUGGGGUC | 18 | 19337 |
| HSV1-UL54-2349 | + | UCGUCCCGAUAAUGGGGUC | 19 | 19338 |
| HSV1-UL54-2350 | + | GUCGUCCCGAUAAUGGGGUC | 20 | 19339 |
| HSV1-UL54-2351 | + | GGUCGUCCCGAUAAUGGGGUC | 21 | 19340 |
| HSV1-UL54-2352 | + | CGGUCGUCCCGAUAAUGGGGUC | 22 | 19341 |
| HSV1-UL54-2353 | + | GCGGUCGUCCCGAUAAUGGGGUC | 23 | 19342 |
| HSV1-UL54-2354 | + | CGCGGUCGUCCCGAUAAUGGGGUC | 24 | 19343 |
| HSV1-UL54-1382 | + | CAGGGUUGCGAUUGGUUC | 18 | 18371 |
| HSV1-UL54-1383 | + | CCAGGGUUGCGAUUGGUUC | 19 | 18372 |
| HSV1-UL54-1199 | + | CCCAGGGUUGCGAUUGGUUC | 20 | 18188 |
| HSV1-UL54-1384 | + | CCCCAGGGUUGCGAUUGGUUC | 21 | 18373 |
| HSV1-UL54-1385 | + | CCCCCAGGGUUGCGAUUGGUUC | 22 | 18374 |
| HSV1-UL54-1386 | + | UCCCCCAGGGUUGCGAUUGGUUC | 23 | 18375 |
| HSV1-UL54-1387 | + | GUCCCCCAGGGUUGCGAUUGGUUC | 24 | 18376 |
| HSV1-UL54-2355 | + | ACCGCCGCGCGCUCGGAG | 18 | 19344 |
| HSV1-UL54-2356 | + | GACCGCCGCGCGCUCGGAG | 19 | 19345 |
| HSV1-UL54-2357 | + | CGACCGCCGCGCGCUCGGAG | 20 | 19346 |
| HSV1-UL54-2358 | + | UCGACCGCCGCGCGCUCGGAG | 21 | 19347 |
| HSV1-UL54-2359 | + | GUCGACCGCCGCGCGCUCGGAG | 22 | 19348 |
| HSV1-UL54-2360 | + | GGUCGACCGCCGCGCGCUCGGAG | 23 | 19349 |
| HSV1-UL54-2361 | + | CGGUCGACCGCCGCGCGCUCGGAG | 24 | 19350 |
| HSV1-UL54-2362 | + | AAUCGCCAGCGUCAUUAG | 18 | 19351 |
| HSV1-UL54-2363 | + | CAAUCGCCAGCGUCAUUAG | 19 | 19352 |
| HSV1-UL54-454 | + | GCAAUCGCCAGCGUCAUUAG | 20 | 7217 |
| HSV1-UL54-2364 | + | GGCAAUCGCCAGCGUCAUUAG | 21 | 19353 |
| HSV1-UL54-2365 | + | GGGCAAUCGCCAGCGUCAUUAG | 22 | 19354 |
| HSV1-UL54-2366 | + | GGGGCAAUCGCCAGCGUCAUUAG | 23 | 19355 |
| HSV1-UL54-2367 | + | GGGGGCAAUCGCCAGCGUCAUUAG | 24 | 19356 |
| HSV1-UL54-2368 | + | AUUUGCCGUGCACGUACG | 18 | 19357 |
| HSV1-UL54-2369 | + | UAUUUGCCGUGCACGUACG | 19 | 19358 |
| HSV1-UL54-348 | + | AUAUUUGCCGUGCACGUACG | 20 | 7111 |
| HSV1-UL54-2370 | + | AAUAUUUGCCGUGCACGUACG | 21 | 19359 |
| HSV1-UL54-2371 | + | AAAUAUUUGCCGUGCACGUACG | 22 | 19360 |
| HSV1-UL54-2372 | + | AAAAUAUUUGCCGUGCACGUACG | 23 | 19361 |
| HSV1-UL54-2373 | + | AAAAAUAUUUGCCGUGCACGUACG | 24 | 19362 |
| HSV1-UL54-2374 | + | CCCGCCAUGCAGGCCCCG | 18 | 19363 |
| HSV1-UL54-2375 | + | GCCCGCCAUGCAGGCCCCG | 19 | 19364 |
| HSV1-UL54-360 | + | GGCCCGCCAUGCAGGCCCCG | 20 | 7123 |
| HSV1-UL54-2376 | + | AGGCCCGCCAUGCAGGCCCCG | 21 | 19365 |
| HSV1-UL54-2377 | + | CAGGCCCGCCAUGCAGGCCCCG | 22 | 19366 |
| HSV1-UL54-2378 | + | UCAGGCCCGCCAUGCAGGCCCCG | 23 | 19367 |
| HSV1-UL54-2379 | + | AUCAGGCCCGCCAUGCAGGCCCCG | 24 | 19368 |
| HSV1-UL54-1422 | + | GGUUCUGGGGGCACGCCG | 18 | 18411 |
| HSV1-UL54-1423 | + | UGGUUCUGGGGGCACGCCG | 19 | 18412 |
| HSV1-UL54-1424 | + | UUGGUUCUGGGGGCACGCCG | 20 | 18413 |
| HSV1-UL54-1425 | + | AUUGGUUCUGGGGGCACGCCG | 21 | 18414 |
| HSV1-UL54-1426 | + | GAUUGGUUCUGGGGGCACGCCG | 22 | 18415 |
| HSV1-UL54-1427 | + | CGAUUGGUUCUGGGGGCACGCCG | 23 | 18416 |
| HSV1-UL54-1428 | + | GCGAUUGGUUCUGGGGGCACGCCG | 24 | 18417 |
| HSV1-UL54-2380 | + | GCGCUCGGAGAUGGAGCG | 18 | 19369 |
| HSV1-UL54-2381 | + | CGCGCUCGGAGAUGGAGCG | 19 | 19370 |
| HSV1-UL54-2382 | + | GCGCGCUCGGAGAUGGAGCG | 20 | 19371 |
| HSV1-UL54-2383 | + | CGCGCGCUCGGAGAUGGAGCG | 21 | 19372 |
| HSV1-UL54-2384 | + | CCGCGCGCUCGGAGAUGGAGCG | 22 | 19373 |
| HSV1-UL54-2385 | + | GCCGCGCGCUCGGAGAUGGAGCG | 23 | 19374 |
| HSV1-UL54-2386 | + | CGCCGCGCGCUCGGAGAUGGAGCG | 24 | 19375 |
| HSV1-UL54-2387 | + | UCGCCAGCGUCAUUAGCG | 18 | 19376 |
| HSV1-UL54-2388 | + | AUCGCCAGCGUCAUUAGCG | 19 | 19377 |
| HSV1-UL54-456 | + | AAUCGCCAGCGUCAUUAGCG | 20 | 7219 |
| HSV1-UL54-2389 | + | CAAUCGCCAGCGUCAUUAGCG | 21 | 19378 |
| HSV1-UL54-2390 | + | GCAAUCGCCAGCGUCAUUAGCG | 22 | 19379 |
| HSV1-UL54-2391 | + | GGCAAUCGCCAGCGUCAUUAGCG | 23 | 19380 |
| HSV1-UL54-2392 | + | GGGCAAUCGCCAGCGUCAUUAGCG | 24 | 19381 |
| HSV1-UL54-2393 | + | GCCCAGGGGCUAUUCGCG | 18 | 19382 |
| HSV1-UL54-2394 | + | GGCCCAGGGGCUAUUCGCG | 19 | 19383 |
| HSV1-UL54-2395 | + | GGGCCCAGGGGCUAUUCGCG | 20 | 19384 |
| HSV1-UL54-2396 | + | GGGGCCCAGGGGCUAUUCGCG | 21 | 19385 |
| HSV1-UL54-2397 | + | CGGGGCCCAGGGGCUAUUCGCG | 22 | 19386 |
| HSV1-UL54-2398 | + | CCGGGGCCCAGGGGCUAUUCGCG | 23 | 19387 |
| HSV1-UL54-2399 | + | ACCGGGGCCCAGGGGCUAUUCGCG | 24 | 19388 |
| HSV1-UL54-2400 | + | GCGUCGAUGGUGUCGGCG | 18 | 19389 |
| HSV1-UL54-2401 | + | GGCGUCGAUGGUGUCGGCG | 19 | 19390 |
| HSV1-UL54-2402 | + | UGGCGUCGAUGGUGUCGGCG | 20 | 19391 |
| HSV1-UL54-2403 | + | GUGGCGUCGAUGGUGUCGGCG | 21 | 19392 |
| HSV1-UL54-2404 | + | GGUGGCGUCGAUGGUGUCGGCG | 22 | 19393 |
| HSV1-UL54-2405 | + | UGGUGGCGUCGAUGGUGUCGGCG | 23 | 19394 |
| HSV1-UL54-2406 | + | GUGGUGGCGUCGAUGGUGUCGGCG | 24 | 19395 |
| HSV1-UL54-2407 | + | CGCGGGGGUCCGCGGGCG | 18 | 19396 |
| HSV1-UL54-2408 | + | GCGCGGGGGUCCGCGGGCG | 19 | 19397 |
| HSV1-UL54-450 | + | GGCGCGGGGGUCCGCGGGCG | 20 | 7213 |
| HSV1-UL54-2409 | + | GGGCGCGGGGGUCCGCGGGCG | 21 | 19398 |
| HSV1-UL54-2410 | + | GGGGCGCGGGGGUCCGCGGGCG | 22 | 19399 |
| HSV1-UL54-2411 | + | CGGGGCGCGGGGGUCCGCGGGCG | 23 | 19400 |
| HSV1-UL54-2412 | + | CCGGGGCGCGGGGGUCCGCGGGCG | 24 | 19401 |
| HSV1-UL54-2413 | + | CGCUCCGGGGCCGGGGCG | 18 | 19402 |
| HSV1-UL54-2414 | + | UCGCUCCGGGGCCGGGGCG | 19 | 19403 |
| HSV1-UL54-442 | + | UUCGCUCCGGGGCCGGGGCG | 20 | 7205 |
| HSV1-UL54-2415 | + | UUUCGCUCCGGGGCCGGGGCG | 21 | 19404 |
| HSV1-UL54-2416 | + | CUUUCGCUCCGGGGCCGGGGCG | 22 | 19405 |
| HSV1-UL54-2417 | + | CCUUUCGCUCCGGGGCCGGGGCG | 23 | 19406 |
| HSV1-UL54-2418 | + | GCCUUUCGCUCCGGGGCCGGGGCG | 24 | 19407 |
| HSV1-UL54-2419 | + | GCCUCGCCAUGGGGGGCG | 18 | 19408 |
| HSV1-UL54-2420 | + | CGCCUCGCCAUGGGGGGCG | 19 | 19409 |
| HSV1-UL54-2421 | + | ACGCCUCGCCAUGGGGGGCG | 20 | 19410 |
| HSV1-UL54-2422 | + | CACGCCUCGCCAUGGGGGGCG | 21 | 19411 |
| HSV1-UL54-2423 | + | CCACGCCUCGCCAUGGGGGGCG | 22 | 19412 |
| HSV1-UL54-2424 | + | GCCACGCCUCGCCAUGGGGGGCG | 23 | 19413 |
| HSV1-UL54-2425 | + | CGCCACGCCUCGCCAUGGGGGGCG | 24 | 19414 |
| HSV1-UL54-2426 | + | GCGGAGGCCAGCGCCUCG | 18 | 19415 |
| HSV1-UL54-2427 | + | GGCGGAGGCCAGCGCCUCG | 19 | 19416 |
| HSV1-UL54-1005 | + | CGGCGGAGGCCAGCGCCUCG | 20 | 7661 |
| HSV1-UL54-2428 | + | UCGGCGGAGGCCAGCGCCUCG | 21 | 19417 |
| HSV1-UL54-2429 | + | GUCGGCGGAGGCCAGCGCCUCG | 22 | 19418 |
| HSV1-UL54-2430 | + | CGUCGGCGGAGGCCAGCGCCUCG | 23 | 19419 |
| HSV1-UL54-2431 | + | UCGUCGGCGGAGGCCAGCGCCUCG | 24 | 19420 |
| HSV1-UL54-2432 | + | CACGCCAGCGUCUCGUCG | 18 | 19421 |
| HSV1-UL54-2433 | + | CCACGCCAGCGUCUCGUCG | 19 | 19422 |
| HSV1-UL54-2434 | + | ACCACGCCAGCGUCUCGUCG | 20 | 19423 |
| HSV1-UL54-2435 | + | CACCACGCCAGCGUCUCGUCG | 21 | 19424 |
| HSV1-UL54-2436 | + | GCACCACGCCAGCGUCUCGUCG | 22 | 19425 |
| HSV1-UL54-2437 | + | UGCACCACGCCAGCGUCUCGUCG | 23 | 19426 |
| HSV1-UL54-2438 | + | UUGCACCACGCCAGCGUCUCGUCG | 24 | 19427 |
| HSV1-UL54-2439 | + | AUGCAGGCCCCGGGGAGG | 18 | 19428 |
| HSV1-UL54-2440 | + | CAUGCAGGCCCCGGGGAGG | 19 | 19429 |
| HSV1-UL54-2441 | + | CCAUGCAGGCCCCGGGGAGG | 20 | 19430 |
| HSV1-UL54-2442 | + | GCCAUGCAGGCCCCGGGGAGG | 21 | 19431 |
| HSV1-UL54-2443 | + | CGCCAUGCAGGCCCCGGGGAGG | 22 | 19432 |
| HSV1-UL54-2444 | + | CCGCCAUGCAGGCCCCGGGGAGG | 23 | 19433 |
| HSV1-UL54-2445 | + | CCCGCCAUGCAGGCCCCGGGGAGG | 24 | 19434 |
| HSV1-UL54-2446 | + | UUUGCCGUGCACGUACGG | 18 | 19435 |
| HSV1-UL54-2447 | + | AUUUGCCGUGCACGUACGG | 19 | 19436 |
| HSV1-UL54-349 | + | UAUUUGCCGUGCACGUACGG | 20 | 7112 |
| HSV1-UL54-2448 | + | AUAUUUGCCGUGCACGUACGG | 21 | 19437 |
| HSV1-UL54-2449 | + | AAUAUUUGCCGUGCACGUACGG | 22 | 19438 |
| HSV1-UL54-2450 | + | AAAUAUUUGCCGUGCACGUACGG | 23 | 19439 |
| HSV1-UL54-2451 | + | AAAAUAUUUGCCGUGCACGUACGG | 24 | 19440 |
| HSV1-UL54-1517 | + | GUUCUGGGGGCACGCCGG | 18 | 18506 |
| HSV1-UL54-1518 | + | GGUUCUGGGGGCACGCCGG | 19 | 18507 |
| HSV1-UL54-1201 | + | UGGUUCUGGGGGCACGCCGG | 20 | 18190 |
| HSV1-UL54-1519 | + | UUGGUUCUGGGGGCACGCCGG | 21 | 18508 |
| HSV1-UL54-1520 | + | AUUGGUUCUGGGGGCACGCCGG | 22 | 18509 |
| HSV1-UL54-1521 | + | GAUUGGUUCUGGGGGCACGCCGG | 23 | 18510 |
| HSV1-UL54-1522 | + | CGAUUGGUUCUGGGGGCACGCCGG | 24 | 18511 |
| HSV1-UL54-2452 | + | CGCCAGCGUCAUUAGCGG | 18 | 19441 |
| HSV1-UL54-2453 | + | UCGCCAGCGUCAUUAGCGG | 19 | 19442 |
| HSV1-UL54-457 | + | AUCGCCAGCGUCAUUAGCGG | 20 | 7220 |
| HSV1-UL54-2454 | + | AAUCGCCAGCGUCAUUAGCGG | 21 | 19443 |
| HSV1-UL54-2455 | + | CAAUCGCCAGCGUCAUUAGCGG | 22 | 19444 |
| HSV1-UL54-2456 | + | GCAAUCGCCAGCGUCAUUAGCGG | 23 | 19445 |
| HSV1-UL54-2457 | + | GGCAAUCGCCAGCGUCAUUAGCGG | 24 | 19446 |
| HSV1-UL54-2458 | + | GGGCGCGGGGGUCCGCGG | 18 | 19447 |
| HSV1-UL54-2459 | + | GGGGCGCGGGGGUCCGCGG | 19 | 19448 |
| HSV1-UL54-2460 | + | CGGGGCGCGGGGGUCCGCGG | 20 | 19449 |
| HSV1-UL54-2461 | + | CCGGGGCGCGGGGGUCCGCGG | 21 | 19450 |
| HSV1-UL54-2462 | + | GCCGGGGCGCGGGGGUCCGCGG | 22 | 19451 |
| HSV1-UL54-2463 | + | GGCCGGGGCGCGGGGGUCCGCGG | 23 | 19452 |
| HSV1-UL54-2464 | + | GGGCCGGGGCGCGGGGGUCCGCGG | 24 | 19453 |
| HSV1-UL54-2465 | + | CCCAGGGGCUAUUCGCGG | 18 | 19454 |
| HSV1-UL54-2466 | + | GCCCAGGGGCUAUUCGCGG | 19 | 19455 |
| HSV1-UL54-418 | + | GGCCCAGGGGCUAUUCGCGG | 20 | 7181 |
| HSV1-UL54-2467 | + | GGGCCCAGGGGCUAUUCGCGG | 21 | 19456 |
| HSV1-UL54-2468 | + | GGGGCCCAGGGGCUAUUCGCGG | 22 | 19457 |
| HSV1-UL54-2469 | + | CGGGGCCCAGGGGCUAUUCGCGG | 23 | 19458 |
| HSV1-UL54-2470 | + | CCGGGGCCCAGGGGCUAUUCGCGG | 24 | 19459 |
| HSV1-UL54-2471 | + | GCGGGGGUCCGCGGGCGG | 18 | 19460 |
| HSV1-UL54-2472 | + | CGCGGGGGUCCGCGGGCGG | 19 | 19461 |
| HSV1-UL54-451 | + | GCGCGGGGGUCCGCGGGCGG | 20 | 7214 |
| HSV1-UL54-2473 | + | GGCGCGGGGGUCCGCGGGCGG | 21 | 19462 |
| HSV1-UL54-2474 | + | GGGCGCGGGGGUCCGCGGGCGG | 22 | 19463 |
| HSV1-UL54-2475 | + | GGGGCGCGGGGGUCCGCGGGCGG | 23 | 19464 |
| HSV1-UL54-2476 | + | CGGGGCGCGGGGGUCCGCGGGCGG | 24 | 19465 |
| HSV1-UL54-2477 | + | ACGCCAGCGUCUCGUCGG | 18 | 19466 |
| HSV1-UL54-2478 | + | CACGCCAGCGUCUCGUCGG | 19 | 19467 |
| HSV1-UL54-390 | + | CCACGCCAGCGUCUCGUCGG | 20 | 7153 |
| HSV1-UL54-2479 | + | ACCACGCCAGCGUCUCGUCGG | 21 | 19468 |
| HSV1-UL54-2480 | + | CACCACGCCAGCGUCUCGUCGG | 22 | 19469 |
| HSV1-UL54-2481 | + | GCACCACGCCAGCGUCUCGUCGG | 23 | 19470 |
| HSV1-UL54-2482 | + | UGCACCACGCCAGCGUCUCGUCGG | 24 | 19471 |
| HSV1-UL54-2483 | + | CGUCCACCCCGCCCCGGG | 18 | 19472 |
| HSV1-UL54-2484 | + | CCGUCCACCCCGCCCCGGG | 19 | 19473 |
| HSV1-UL54-2485 | + | UCCGUCCACCCCGCCCCGGG | 20 | 19474 |
| HSV1-UL54-2486 | + | GUCCGUCCACCCCGCCCCGGG | 21 | 19475 |
| HSV1-UL54-2487 | + | CGUCCGUCCACCCCGCCCCGGG | 22 | 19476 |
| HSV1-UL54-2488 | + | CCGUCCGUCCACCCCGCCCCGGG | 23 | 19477 |
| HSV1-UL54-2489 | + | GCCGUCCGUCCACCCCGCCCCGGG | 24 | 19478 |
| HSV1-UL54-2490 | + | GGCGCGGGGGUCCGCGGG | 18 | 19479 |
| HSV1-UL54-2491 | + | GGGCGCGGGGGUCCGCGGG | 19 | 19480 |
| HSV1-UL54-448 | + | GGGGCGCGGGGGUCCGCGGG | 20 | 7211 |
| HSV1-UL54-2492 | + | CGGGGCGCGGGGGUCCGCGGG | 21 | 19481 |
| HSV1-UL54-2493 | + | CCGGGGCGCGGGGGUCCGCGGG | 22 | 19482 |
| HSV1-UL54-2494 | + | GCCGGGGCGCGGGGGUCCGCGGG | 23 | 19483 |
| HSV1-UL54-2495 | + | GGCCGGGGCGCGGGGGUCCGCGGG | 24 | 19484 |
| HSV1-UL54-2496 | + | GCGCCUUUCGCUCCGGGG | 18 | 19485 |
| HSV1-UL54-2497 | + | GGCGCCUUUCGCUCCGGGG | 19 | 19486 |
| HSV1-UL54-2498 | + | GGGCGCCUUUCGCUCCGGGG | 20 | 19487 |
| HSV1-UL54-2499 | + | CGGGCGCCUUUCGCUCCGGGG | 21 | 19488 |
| HSV1-UL54-2500 | + | GCGGGCGCCUUUCGCUCCGGGG | 22 | 19489 |
| HSV1-UL54-2501 | + | GGCGGGCGCCUUUCGCUCCGGGG | 23 | 19490 |
| HSV1-UL54-2502 | + | CGGCGGGCGCCUUUCGCUCCGGGG | 24 | 19491 |
| HSV1-UL54-2503 | + | CGCUGGCCUCCGGGUGGG | 18 | 19492 |
| HSV1-UL54-2504 | + | CCGCUGGCCUCCGGGUGGG | 19 | 19493 |
| HSV1-UL54-2505 | + | UCCGCUGGCCUCCGGGUGGG | 20 | 19494 |
| HSV1-UL54-2506 | + | GUCCGCUGGCCUCCGGGUGGG | 21 | 19495 |
| HSV1-UL54-2507 | + | UGUCCGCUGGCCUCCGGGUGGG | 22 | 19496 |
| HSV1-UL54-2508 | + | GUGUCCGCUGGCCUCCGGGUGGG | 23 | 19497 |
| HSV1-UL54-2509 | + | CGUGUCCGCUGGCCUCCGGGUGGG | 24 | 19498 |
| HSV1-UL54-2510 | + | GCGCCACGCCUCGCCAUG | 18 | 19499 |
| HSV1-UL54-2511 | + | CGCGCCACGCCUCGCCAUG | 19 | 19500 |
| HSV1-UL54-471 | + | CCGCGCCACGCCUCGCCAUG | 20 | 7234 |
| HSV1-UL54-2512 | + | GCCGCGCCACGCCUCGCCAUG | 21 | 19501 |
| HSV1-UL54-2513 | + | UGCCGCGCCACGCCUCGCCAUG | 22 | 19502 |
| HSV1-UL54-2514 | + | CUGCCGCGCCACGCCUCGCCAUG | 23 | 19503 |
| HSV1-UL54-2515 | + | ACUGCCGCGCCACGCCUCGCCAUG | 24 | 19504 |
| HSV1-UL54-2516 | + | CGCCUUCAGGUAGCACUG | 18 | 19505 |
| HSV1-UL54-2517 | + | GCGCCUUCAGGUAGCACUG | 19 | 19506 |
| HSV1-UL54-2518 | + | CGCGCCUUCAGGUAGCACUG | 20 | 19507 |
| HSV1-UL54-2519 | + | UCGCGCCUUCAGGUAGCACUG | 21 | 19508 |
| HSV1-UL54-2520 | + | CUCGCGCCUUCAGGUAGCACUG | 22 | 19509 |
| HSV1-UL54-2521 | + | CCUCGCGCCUUCAGGUAGCACUG | 23 | 19510 |
| HSV1-UL54-2522 | + | GCCUCGCGCCUUCAGGUAGCACUG | 24 | 19511 |
| HSV1-UL54-2523 | + | AAGGGCCGCAGGCGCGUG | 18 | 19512 |
| HSV1-UL54-2524 | + | AAAGGGCCGCAGGCGCGUG | 19 | 19513 |
| HSV1-UL54-2525 | + | GAAAGGGCCGCAGGCGCGUG | 20 | 19514 |
| HSV1-UL54-2526 | + | AGAAAGGGCCGCAGGCGCGUG | 21 | 19515 |
| HSV1-UL54-2527 | + | GAGAAAGGGCCGCAGGCGCGUG | 22 | 19516 |
| HSV1-UL54-2528 | + | GGAGAAAGGGCCGCAGGCGCGUG | 23 | 19517 |
| HSV1-UL54-2529 | + | UGGAGAAAGGGCCGCAGGCGCGUG | 24 | 19518 |
| HSV1-UL54-2530 | + | CCACGCUCGACGCGGUUG | 18 | 19519 |
| HSV1-UL54-2531 | + | GCCACGCUCGACGCGGUUG | 19 | 19520 |
| HSV1-UL54-2532 | + | CGCCACGCUCGACGCGGUUG | 20 | 19521 |
| HSV1-UL54-2533 | + | ACGCCACGCUCGACGCGGUUG | 21 | 19522 |
| HSV1-UL54-2534 | + | GACGCCACGCUCGACGCGGUUG | 22 | 19523 |
| HSV1-UL54-2535 | + | CGACGCCACGCUCGACGCGGUUG | 23 | 19524 |
| HSV1-UL54-2536 | + | GCGACGCCACGCUCGACGCGGUUG | 24 | 19525 |
| HSV1-UL54-2537 | + | CGCGCCACGCCUCGCCAU | 18 | 19526 |
| HSV1-UL54-2538 | + | CCGCGCCACGCCUCGCCAU | 19 | 19527 |
| HSV1-UL54-470 | + | GCCGCGCCACGCCUCGCCAU | 20 | 7233 |
| HSV1-UL54-2539 | + | UGCCGCGCCACGCCUCGCCAU | 21 | 19528 |
| HSV1-UL54-2540 | + | CUGCCGCGCCACGCCUCGCCAU | 22 | 19529 |
| HSV1-UL54-2541 | + | ACUGCCGCGCCACGCCUCGCCAU | 23 | 19530 |
| HSV1-UL54-2542 | + | CACUGCCGCGCCACGCCUCGCCAU | 24 | 19531 |
| HSV1-UL54-2543 | + | UCCCGAUAAUGGGGUCCU | 18 | 19532 |
| HSV1-UL54-2544 | + | GUCCCGAUAAUGGGGUCCU | 19 | 19533 |
| HSV1-UL54-382 | + | CGUCCCGAUAAUGGGGUCCU | 20 | 7145 |
| HSV1-UL54-2545 | + | UCGUCCCGAUAAUGGGGUCCU | 21 | 19534 |
| HSV1-UL54-2546 | + | GUCGUCCCGAUAAUGGGGUCCU | 22 | 19535 |
| HSV1-UL54-2547 | + | GGUCGUCCCGAUAAUGGGGUCCU | 23 | 19536 |
| HSV1-UL54-2548 | + | CGGUCGUCCCGAUAAUGGGGUCCU | 24 | 19537 |
| HSV1-UL54-2549 | + | GGUCGACCGCCGCGCGCU | 18 | 19538 |
| HSV1-UL54-2550 | + | CGGUCGACCGCCGCGCGCU | 19 | 19539 |
| HSV1-UL54-426 | + | GCGGUCGACCGCCGCGCGCU | 20 | 7189 |
| HSV1-UL54-2551 | + | UGCGGUCGACCGCCGCGCGCU | 21 | 19540 |
| HSV1-UL54-2552 | + | AUGCGGUCGACCGCCGCGCGCU | 22 | 19541 |
| HSV1-UL54-2553 | + | GAUGCGGUCGACCGCCGCGCGCU | 23 | 19542 |
| HSV1-UL54-2554 | + | UGAUGCGGUCGACCGCCGCGCGCU | 24 | 19543 |
| HSV1-UL54-2555 | + | CGGCGGGCGCCUUUCGCU | 18 | 19544 |
| HSV1-UL54-2556 | + | GCGGCGGGCGCCUUUCGCU | 19 | 19545 |
| HSV1-UL54-2557 | + | GGCGGCGGGCGCCUUUCGCU | 20 | 19546 |
| HSV1-UL54-2558 | + | CGGCGGCGGGCGCCUUUCGCU | 21 | 19547 |
| HSV1-UL54-2559 | + | UCGGCGGCGGGCGCCUUUCGCU | 22 | 19548 |
| HSV1-UL54-2560 | + | GUCGGCGGCGGGCGCCUUUCGCU | 23 | 19549 |
| HSV1-UL54-2561 | + | UGUCGGCGGCGGGCGCCUUUCGCU | 24 | 19550 |
| HSV1-UL54-1602 | + | AGGGUUGCGAUUGGUUCU | 18 | 18591 |
| HSV1-UL54-1603 | + | CAGGGUUGCGAUUGGUUCU | 19 | 18592 |
| HSV1-UL54-1221 | + | CCAGGGUUGCGAUUGGUUCU | 20 | 18210 |
| HSV1-UL54-1604 | + | CCCAGGGUUGCGAUUGGUUCU | 21 | 18593 |
| HSV1-UL54-1605 | + | CCCCAGGGUUGCGAUUGGUUCU | 22 | 18594 |
| HSV1-UL54-1606 | + | CCCCCAGGGUUGCGAUUGGUUCU | 23 | 18595 |
| HSV1-UL54-1607 | + | UCCCCCAGGGUUGCGAUUGGUUCU | 24 | 18596 |
| HSV1-UL54-2562 | + | AAUAUUUGCCGUGCACGU | 18 | 19551 |
| HSV1-UL54-2563 | + | AAAUAUUUGCCGUGCACGU | 19 | 19552 |
| HSV1-UL54-2564 | + | AAAAUAUUUGCCGUGCACGU | 20 | 19553 |
| HSV1-UL54-2565 | + | AAAAAUAUUUGCCGUGCACGU | 21 | 19554 |
| HSV1-UL54-2566 | + | UAAAAAUAUUUGCCGUGCACGU | 22 | 19555 |
| HSV1-UL54-2567 | + | AUAAAAAUAUUUGCCGUGCACGU | 23 | 19556 |
| HSV1-UL54-2568 | + | AAUAAAAAUAUUUGCCGUGCACGU | 24 | 19557 |
| HSV1-UL54-1639 | + | CCAGGGUUGCGAUUGGUU | 18 | 18628 |
| HSV1-UL54-1640 | + | CCCAGGGUUGCGAUUGGUU | 19 | 18629 |
| HSV1-UL54-1641 | + | CCCCAGGGUUGCGAUUGGUU | 20 | 18630 |
| HSV1-UL54-1642 | + | CCCCCAGGGUUGCGAUUGGUU | 21 | 18631 |
| HSV1-UL54-1643 | + | UCCCCCAGGGUUGCGAUUGGUU | 22 | 18632 |
| HSV1-UL54-1644 | + | GUCCCCCAGGGUUGCGAUUGGUU | 23 | 18633 |
| HSV1-UL54-1645 | + | GGUCCCCCAGGGUUGCGAUUGGUU | 24 | 18634 |
| HSV1-UL54-2569 | - | CCCGGAGGCCAGCGGACA | 18 | 19558 |
| HSV1-UL54-2570 | - | ACCCGGAGGCCAGCGGACA | 19 | 19559 |
| HSV1-UL54-271 | - | CACCCGGAGGCCAGCGGACA | 20 | 7034 |
| HSV1-UL54-2571 | - | CCACCCGGAGGCCAGCGGACA | 21 | 19560 |
| HSV1-UL54-2572 | - | CCCACCCGGAGGCCAGCGGACA | 22 | 19561 |
| HSV1-UL54-2573 | - | ACCCACCCGGAGGCCAGCGGACA | 23 | 19562 |
| HSV1-UL54-2574 | - | GACCCACCCGGAGGCCAGCGGACA | 24 | 19563 |
| HSV1-UL54-2575 | - | CGACCCACCCGGAGGCCA | 18 | 19564 |
| HSV1-UL54-2576 | - | CCGACCCACCCGGAGGCCA | 19 | 19565 |
| HSV1-UL54-922 | - | CCCGACCCACCCGGAGGCCA | 20 | 7594 |
| HSV1-UL54-2577 | - | GCCCGACCCACCCGGAGGCCA | 21 | 19566 |
| HSV1-UL54-2578 | - | AGCCCGACCCACCCGGAGGCCA | 22 | 19567 |
| HSV1-UL54-2579 | - | CAGCCCGACCCACCCGGAGGCCA | 23 | 19568 |
| HSV1-UL54-2580 | - | GCAGCCCGACCCACCCGGAGGCCA | 24 | 19569 |
| HSV1-UL54-2581 | - | CCCGGUGCUGGCGGGCCA | 18 | 19570 |
| HSV1-UL54-2582 | - | CCCCGGUGCUGGCGGGCCA | 19 | 19571 |
| HSV1-UL54-296 | - | GCCCCGGUGCUGGCGGGCCA | 20 | 7059 |
| HSV1-UL54-2583 | - | GGCCCCGGUGCUGGCGGGCCA | 21 | 19572 |
| HSV1-UL54-2584 | - | GGGCCCCGGUGCUGGCGGGCCA | 22 | 19573 |
| HSV1-UL54-2585 | - | UGGGCCCCGGUGCUGGCGGGCCA | 23 | 19574 |
| HSV1-UL54-2586 | - | CUGGGCCCCGGUGCUGGCGGGCCA | 24 | 19575 |
| HSV1-UL54-2587 | - | GCGAGGCGUGGCGCGGCA | 18 | 19576 |
| HSV1-UL54-2588 | - | GGCGAGGCGUGGCGCGGCA | 19 | 19577 |
| HSV1-UL54-919 | - | UGGCGAGGCGUGGCGCGGCA | 20 | 7592 |
| HSV1-UL54-2589 | - | AUGGCGAGGCGUGGCGCGGCA | 21 | 19578 |
| HSV1-UL54-2590 | - | CAUGGCGAGGCGUGGCGCGGCA | 22 | 19579 |
| HSV1-UL54-2591 | - | CCAUGGCGAGGCGUGGCGCGGCA | 23 | 19580 |
| HSV1-UL54-2592 | - | CCCAUGGCGAGGCGUGGCGCGGCA | 24 | 19581 |
| HSV1-UL54-2593 | - | CGGCGGUCGACCGCAUCA | 18 | 19582 |
| HSV1-UL54-2594 | - | GCGGCGGUCGACCGCAUCA | 19 | 19583 |
| HSV1-UL54-929 | - | CGCGGCGGUCGACCGCAUCA | 20 | 7599 |
| HSV1-UL54-2595 | - | GCGCGGCGGUCGACCGCAUCA | 21 | 19584 |
| HSV1-UL54-2596 | - | CGCGCGGCGGUCGACCGCAUCA | 22 | 19585 |
| HSV1-UL54-2597 | - | GCGCGCGGCGGUCGACCGCAUCA | 23 | 19586 |
| HSV1-UL54-2598 | - | AGCGCGCGGCGGUCGACCGCAUCA | 24 | 19587 |
| HSV1-UL54-2599 | - | UGCGCCAAGAAAAUUUCA | 18 | 19588 |
| HSV1-UL54-2600 | - | CUGCGCCAAGAAAAUUUCA | 19 | 19589 |
| HSV1-UL54-949 | - | GCUGCGCCAAGAAAAUUUCA | 20 | 7613 |
| HSV1-UL54-2601 | - | UGCUGCGCCAAGAAAAUUUCA | 21 | 19590 |
| HSV1-UL54-2602 | - | GUGCUGCGCCAAGAAAAUUUCA | 22 | 19591 |
| HSV1-UL54-2603 | - | CGUGCUGCGCCAAGAAAAUUUCA | 23 | 19592 |
| HSV1-UL54-2604 | - | GCGUGCUGCGCCAAGAAAAUUUCA | 24 | 19593 |
| HSV1-UL54-2605 | - | CCGCGCCCCGGCCCCGGA | 18 | 19594 |
| HSV1-UL54-2606 | - | CCCGCGCCCCGGCCCCGGA | 19 | 19595 |
| HSV1-UL54-927 | - | CCCCGCGCCCCGGCCCCGGA | 20 | 7597 |
| HSV1-UL54-2607 | - | CCCCCGCGCCCCGGCCCCGGA | 21 | 19596 |
| HSV1-UL54-2608 | - | ACCCCCGCGCCCCGGCCCCGGA | 22 | 19597 |
| HSV1-UL54-2609 | - | GACCCCCGCGCCCCGGCCCCGGA | 23 | 19598 |
| HSV1-UL54-2610 | - | GGACCCCCGCGCCCCGGCCCCGGA | 24 | 19599 |
| HSV1-UL54-2611 | - | CUUGGUGUCGGGGUCGGA | 18 | 19600 |
| HSV1-UL54-2612 | - | CCUUGGUGUCGGGGUCGGA | 19 | 19601 |
| HSV1-UL54-969 | - | CCCUUGGUGUCGGGGUCGGA | 20 | 7629 |
| HSV1-UL54-2613 | - | ACCCUUGGUGUCGGGGUCGGA | 21 | 19602 |
| HSV1-UL54-2614 | - | GACCCUUGGUGUCGGGGUCGGA | 22 | 19603 |
| HSV1-UL54-2615 | - | CGACCCUUGGUGUCGGGGUCGGA | 23 | 19604 |
| HSV1-UL54-2616 | - | GCGACCCUUGGUGUCGGGGUCGGA | 24 | 19605 |
| HSV1-UL54-2617 | - | CCUGCAUGGCGGGCCUGA | 18 | 19606 |
| HSV1-UL54-2618 | - | GCCUGCAUGGCGGGCCUGA | 19 | 19607 |
| HSV1-UL54-973 | - | GGCCUGCAUGGCGGGCCUGA | 20 | 7632 |
| HSV1-UL54-2619 | - | GGGCCUGCAUGGCGGGCCUGA | 21 | 19608 |
| HSV1-UL54-2620 | - | GGGGCCUGCAUGGCGGGCCUGA | 22 | 19609 |
| HSV1-UL54-2621 | - | CGGGGCCUGCAUGGCGGGCCUGA | 23 | 19610 |
| HSV1-UL54-2622 | - | CCGGGGCCUGCAUGGCGGGCCUGA | 24 | 19611 |
| HSV1-UL54-2623 | - | GCCCCCAGGACCCCAUUA | 18 | 19612 |
| HSV1-UL54-2624 | - | CGCCCCCAGGACCCCAUUA | 19 | 19613 |
| HSV1-UL54-952 | - | GCGCCCCCAGGACCCCAUUA | 20 | 7616 |
| HSV1-UL54-2625 | - | UGCGCCCCCAGGACCCCAUUA | 21 | 19614 |
| HSV1-UL54-2626 | - | CUGCGCCCCCAGGACCCCAUUA | 22 | 19615 |
| HSV1-UL54-2627 | - | GCUGCGCCCCCAGGACCCCAUUA | 23 | 19616 |
| HSV1-UL54-2628 | - | CGCUGCGCCCCCAGGACCCCAUUA | 24 | 19617 |
| HSV1-UL54-2629 | - | GUGAGCAGCCCGACCCAC | 18 | 19618 |
| HSV1-UL54-2630 | - | AGUGAGCAGCCCGACCCAC | 19 | 19619 |
| HSV1-UL54-920 | - | CAGUGAGCAGCCCGACCCAC | 20 | 7593 |
| HSV1-UL54-2631 | - | GCAGUGAGCAGCCCGACCCAC | 21 | 19620 |
| HSV1-UL54-2632 | - | GGCAGUGAGCAGCCCGACCCAC | 22 | 19621 |
| HSV1-UL54-2633 | - | CGGCAGUGAGCAGCCCGACCCAC | 23 | 19622 |
| HSV1-UL54-2634 | - | GCGGCAGUGAGCAGCCCGACCCAC | 24 | 19623 |
| HSV1-UL54-2635 | - | ACCCGGAGGCCAGCGGAC | 18 | 19624 |
| HSV1-UL54-2636 | - | CACCCGGAGGCCAGCGGAC | 19 | 19625 |
| HSV1-UL54-911 | - | CCACCCGGAGGCCAGCGGAC | 20 | 7586 |
| HSV1-UL54-2637 | - | CCCACCCGGAGGCCAGCGGAC | 21 | 19626 |
| HSV1-UL54-2638 | - | ACCCACCCGGAGGCCAGCGGAC | 22 | 19627 |
| HSV1-UL54-2639 | - | GACCCACCCGGAGGCCAGCGGAC | 23 | 19628 |
| HSV1-UL54-2640 | - | CGACCCACCCGGAGGCCAGCGGAC | 24 | 19629 |
| HSV1-UL54-2641 | - | CCCUUUCUCCAGUGCUAC | 18 | 19630 |
| HSV1-UL54-2642 | - | GCCCUUUCUCCAGUGCUAC | 19 | 19631 |
| HSV1-UL54-955 | - | GGCCCUUUCUCCAGUGCUAC | 20 | 7618 |
| HSV1-UL54-2643 | - | CGGCCCUUUCUCCAGUGCUAC | 21 | 19632 |
| HSV1-UL54-2644 | - | GCGGCCCUUUCUCCAGUGCUAC | 22 | 19633 |
| HSV1-UL54-2645 | - | UGCGGCCCUUUCUCCAGUGCUAC | 23 | 19634 |
| HSV1-UL54-2646 | - | CUGCGGCCCUUUCUCCAGUGCUAC | 24 | 19635 |
| HSV1-UL54-1730 | - | CCAGAACCAAUCGCAACC | 18 | 18719 |
| HSV1-UL54-1731 | - | CCCAGAACCAAUCGCAACC | 19 | 18720 |
| HSV1-UL54-1732 | - | CCCCAGAACCAAUCGCAACC | 20 | 18721 |
| HSV1-UL54-1733 | - | CCCCCAGAACCAAUCGCAACC | 21 | 18722 |
| HSV1-UL54-1734 | - | GCCCCCAGAACCAAUCGCAACC | 22 | 18723 |
| HSV1-UL54-1735 | - | UGCCCCCAGAACCAAUCGCAACC | 23 | 18724 |
| HSV1-UL54-1736 | - | GUGCCCCCAGAACCAAUCGCAACC | 24 | 18725 |
| HSV1-UL54-2647 | - | UGAGCAGCCCGACCCACC | 18 | 19636 |
| HSV1-UL54-2648 | - | GUGAGCAGCCCGACCCACC | 19 | 19637 |
| HSV1-UL54-268 | - | AGUGAGCAGCCCGACCCACC | 20 | 7031 |
| HSV1-UL54-2649 | - | CAGUGAGCAGCCCGACCCACC | 21 | 19638 |
| HSV1-UL54-2650 | - | GCAGUGAGCAGCCCGACCCACC | 22 | 19639 |
| HSV1-UL54-2651 | - | GGCAGUGAGCAGCCCGACCCACC | 23 | 19640 |
| HSV1-UL54-2652 | - | CGGCAGUGAGCAGCCCGACCCACC | 24 | 19641 |
| HSV1-UL54-1737 | - | CAGAACCAAUCGCAACCC | 18 | 18726 |
| HSV1-UL54-1738 | - | CCAGAACCAAUCGCAACCC | 19 | 18727 |
| HSV1-UL54-1195 | - | CCCAGAACCAAUCGCAACCC | 20 | 18184 |
| HSV1-UL54-1739 | - | CCCCAGAACCAAUCGCAACCC | 21 | 18728 |
| HSV1-UL54-1740 | - | CCCCCAGAACCAAUCGCAACCC | 22 | 18729 |
| HSV1-UL54-1741 | - | GCCCCCAGAACCAAUCGCAACCC | 23 | 18730 |
| HSV1-UL54-1742 | - | UGCCCCCAGAACCAAUCGCAACCC | 24 | 18731 |
| HSV1-UL54-2653 | - | AACCUGCCGCUGCGCCCC | 18 | 19642 |
| HSV1-UL54-2654 | - | CAACCUGCCGCUGCGCCCC | 19 | 19643 |
| HSV1-UL54-951 | - | ACAACCUGCCGCUGCGCCCC | 20 | 7615 |
| HSV1-UL54-2655 | - | CACAACCUGCCGCUGCGCCCC | 21 | 19644 |
| HSV1-UL54-2656 | - | CCACAACCUGCCGCUGCGCCCC | 22 | 19645 |
| HSV1-UL54-2657 | - | ACCACAACCUGCCGCUGCGCCCC | 23 | 19646 |
| HSV1-UL54-2658 | - | CACCACAACCUGCCGCUGCGCCCC | 24 | 19647 |
| HSV1-UL54-1755 | - | CCCCCGCCGGCGUGCCCC | 18 | 18744 |
| HSV1-UL54-1756 | - | ACCCCCGCCGGCGUGCCCC | 19 | 18745 |
| HSV1-UL54-1757 | - | GACCCCCGCCGGCGUGCCCC | 20 | 18746 |
| HSV1-UL54-1758 | - | GGACCCCCGCCGGCGUGCCCC | 21 | 18747 |
| HSV1-UL54-1759 | - | CGGACCCCCGCCGGCGUGCCCC | 22 | 18748 |
| HSV1-UL54-1760 | - | UCGGACCCCCGCCGGCGUGCCCC | 23 | 18749 |
| HSV1-UL54-1761 | - | GUCGGACCCCCGCCGGCGUGCCCC | 24 | 18750 |
| HSV1-UL54-2659 | - | UCCCGCCGCGAAUAGCCC | 18 | 19648 |
| HSV1-UL54-2660 | - | UUCCCGCCGCGAAUAGCCC | 19 | 19649 |
| HSV1-UL54-936 | - | UUUCCCGCCGCGAAUAGCCC | 20 | 7603 |
| HSV1-UL54-2661 | - | GUUUCCCGCCGCGAAUAGCCC | 21 | 19650 |
| HSV1-UL54-2662 | - | CGUUUCCCGCCGCGAAUAGCCC | 22 | 19651 |
| HSV1-UL54-2663 | - | CCGUUUCCCGCCGCGAAUAGCCC | 23 | 19652 |
| HSV1-UL54-2664 | - | GCCGUUUCCCGCCGCGAAUAGCCC | 24 | 19653 |
| HSV1-UL54-2665 | - | CCCUGGGGGACCCCGCCC | 18 | 19654 |
| HSV1-UL54-2666 | - | ACCCUGGGGGACCCCGCCC | 19 | 19655 |
| HSV1-UL54-254 | - | AACCCUGGGGGACCCCGCCC | 20 | 7017 |
| HSV1-UL54-2667 | - | CAACCCUGGGGGACCCCGCCC | 21 | 19656 |
| HSV1-UL54-2668 | - | GCAACCCUGGGGGACCCCGCCC | 22 | 19657 |
| HSV1-UL54-2669 | - | CGCAACCCUGGGGGACCCCGCCC | 23 | 19658 |
| HSV1-UL54-2670 | - | UCGCAACCCUGGGGGACCCCGCCC | 24 | 19659 |
| HSV1-UL54-2671 | - | GCGAUUGCCCCCCCGCCC | 18 | 19660 |
| HSV1-UL54-2672 | - | GGCGAUUGCCCCCCCGCCC | 19 | 19661 |
| HSV1-UL54-924 | - | UGGCGAUUGCCCCCCCGCCC | 20 | 7595 |
| HSV1-UL54-2673 | - | CUGGCGAUUGCCCCCCCGCCC | 21 | 19662 |
| HSV1-UL54-2674 | - | GCUGGCGAUUGCCCCCCCGCCC | 22 | 19663 |
| HSV1-UL54-2675 | - | CGCUGGCGAUUGCCCCCCCGCCC | 23 | 19664 |
| HSV1-UL54-2676 | - | ACGCUGGCGAUUGCCCCCCCGCCC | 24 | 19665 |
| HSV1-UL54-2677 | - | ACCCCCGCGCCCCGGCCC | 18 | 19666 |
| HSV1-UL54-2678 | - | GACCCCCGCGCCCCGGCCC | 19 | 19667 |
| HSV1-UL54-277 | - | GGACCCCCGCGCCCCGGCCC | 20 | 7040 |
| HSV1-UL54-2679 | - | CGGACCCCCGCGCCCCGGCCC | 21 | 19668 |
| HSV1-UL54-2680 | - | GCGGACCCCCGCGCCCCGGCCC | 22 | 19669 |
| HSV1-UL54-2681 | - | CGCGGACCCCCGCGCCCCGGCCC | 23 | 19670 |
| HSV1-UL54-2682 | - | CCGCGGACCCCCGCGCCCCGGCCC | 24 | 19671 |
| HSV1-UL54-2683 | - | GAUGCAUUUCUACCUCCC | 18 | 19672 |
| HSV1-UL54-2684 | - | AGAUGCAUUUCUACCUCCC | 19 | 19673 |
| HSV1-UL54-337 | - | AAGAUGCAUUUCUACCUCCC | 20 | 7100 |
| HSV1-UL54-2685 | - | GAAGAUGCAUUUCUACCUCCC | 21 | 19674 |
| HSV1-UL54-2686 | - | AGAAGAUGCAUUUCUACCUCCC | 22 | 19675 |
| HSV1-UL54-2687 | - | GAGAAGAUGCAUUUCUACCUCCC | 23 | 19676 |
| HSV1-UL54-2688 | - | AGAGAAGAUGCAUUUCUACCUCCC | 24 | 19677 |
| HSV1-UL54-2689 | - | ACCCUGGGGGACCCCGCC | 18 | 19678 |
| HSV1-UL54-2690 | - | AACCCUGGGGGACCCCGCC | 19 | 19679 |
| HSV1-UL54-912 | - | CAACCCUGGGGGACCCCGCC | 20 | 7587 |
| HSV1-UL54-2691 | - | GCAACCCUGGGGGACCCCGCC | 21 | 19680 |
| HSV1-UL54-2692 | - | CGCAACCCUGGGGGACCCCGCC | 22 | 19681 |
| HSV1-UL54-2693 | - | UCGCAACCCUGGGGGACCCCGCC | 23 | 19682 |
| HSV1-UL54-2694 | - | AUCGCAACCCUGGGGGACCCCGCC | 24 | 19683 |
| HSV1-UL54-2695 | - | GCGACUGCGUGCUGCGCC | 18 | 19684 |
| HSV1-UL54-2696 | - | CGCGACUGCGUGCUGCGCC | 19 | 19685 |
| HSV1-UL54-948 | - | GCGCGACUGCGUGCUGCGCC | 20 | 7612 |
| HSV1-UL54-2697 | - | UGCGCGACUGCGUGCUGCGCC | 21 | 19686 |
| HSV1-UL54-2698 | - | AUGCGCGACUGCGUGCUGCGCC | 22 | 19687 |
| HSV1-UL54-2699 | - | CAUGCGCGACUGCGUGCUGCGCC | 23 | 19688 |
| HSV1-UL54-2700 | - | CCAUGCGCGACUGCGUGCUGCGCC | 24 | 19689 |
| HSV1-UL54-2701 | - | GACCCCCGCGCCCCGGCC | 18 | 19690 |
| HSV1-UL54-2702 | - | GGACCCCCGCGCCCCGGCC | 19 | 19691 |
| HSV1-UL54-925 | - | CGGACCCCCGCGCCCCGGCC | 20 | 7596 |
| HSV1-UL54-2703 | - | GCGGACCCCCGCGCCCCGGCC | 21 | 19692 |
| HSV1-UL54-2704 | - | CGCGGACCCCCGCGCCCCGGCC | 22 | 19693 |
| HSV1-UL54-2705 | - | CCGCGGACCCCCGCGCCCCGGCC | 23 | 19694 |
| HSV1-UL54-2706 | - | CCCGCGGACCCCCGCGCCCCGGCC | 24 | 19695 |
| HSV1-UL54-2707 | - | CCCCGGUGCUGGCGGGCC | 18 | 19696 |
| HSV1-UL54-2708 | - | GCCCCGGUGCUGGCGGGCC | 19 | 19697 |
| HSV1-UL54-938 | - | GGCCCCGGUGCUGGCGGGCC | 20 | 7605 |
| HSV1-UL54-2709 | - | GGGCCCCGGUGCUGGCGGGCC | 21 | 19698 |
| HSV1-UL54-2710 | - | UGGGCCCCGGUGCUGGCGGGCC | 22 | 19699 |
| HSV1-UL54-2711 | - | CUGGGCCCCGGUGCUGGCGGGCC | 23 | 19700 |
| HSV1-UL54-2712 | - | CCUGGGCCCCGGUGCUGGCGGGCC | 24 | 19701 |
| HSV1-UL54-2713 | - | CACUUUUGCCGGCAAUCC | 18 | 19702 |
| HSV1-UL54-2714 | - | GCACUUUUGCCGGCAAUCC | 19 | 19703 |
| HSV1-UL54-947 | - | CGCACUUUUGCCGGCAAUCC | 20 | 7611 |
| HSV1-UL54-2715 | - | UCGCACUUUUGCCGGCAAUCC | 21 | 19704 |
| HSV1-UL54-2716 | - | AUCGCACUUUUGCCGGCAAUCC | 22 | 19705 |
| HSV1-UL54-2717 | - | UAUCGCACUUUUGCCGGCAAUCC | 23 | 19706 |
| HSV1-UL54-2718 | - | CUAUCGCACUUUUGCCGGCAAUCC | 24 | 19707 |
| HSV1-UL54-2719 | - | AGAUGCAUUUCUACCUCC | 18 | 19708 |
| HSV1-UL54-2720 | - | AAGAUGCAUUUCUACCUCC | 19 | 19709 |
| HSV1-UL54-970 | - | GAAGAUGCAUUUCUACCUCC | 20 | 7630 |
| HSV1-UL54-2721 | - | AGAAGAUGCAUUUCUACCUCC | 21 | 19710 |
| HSV1-UL54-2722 | - | GAGAAGAUGCAUUUCUACCUCC | 22 | 19711 |
| HSV1-UL54-2723 | - | AGAGAAGAUGCAUUUCUACCUCC | 23 | 19712 |
| HSV1-UL54-2724 | - | GAGAGAAGAUGCAUUUCUACCUCC | 24 | 19713 |
| HSV1-UL54-2725 | - | GAGACCAGACGGGUCUCC | 18 | 19714 |
| HSV1-UL54-2726 | - | CGAGACCAGACGGGUCUCC | 19 | 19715 |
| HSV1-UL54-301 | - | CCGAGACCAGACGGGUCUCC | 20 | 7064 |
| HSV1-UL54-2727 | - | GCCGAGACCAGACGGGUCUCC | 21 | 19716 |
| HSV1-UL54-2728 | - | CGCCGAGACCAGACGGGUCUCC | 22 | 19717 |
| HSV1-UL54-2729 | - | ACGCCGAGACCAGACGGGUCUCC | 23 | 19718 |
| HSV1-UL54-2730 | - | GACGCCGAGACCAGACGGGUCUCC | 24 | 19719 |
| HSV1-UL54-2731 | - | GCGGUCGACCGCAUCAGC | 18 | 19720 |
| HSV1-UL54-2732 | - | GGCGGUCGACCGCAUCAGC | 19 | 19721 |
| HSV1-UL54-930 | - | CGGCGGUCGACCGCAUCAGC | 20 | 7600 |
| HSV1-UL54-2733 | - | GCGGCGGUCGACCGCAUCAGC | 21 | 19722 |
| HSV1-UL54-2734 | - | CGCGGCGGUCGACCGCAUCAGC | 22 | 19723 |
| HSV1-UL54-2735 | - | GCGCGGCGGUCGACCGCAUCAGC | 23 | 19724 |
| HSV1-UL54-2736 | - | CGCGCGGCGGUCGACCGCAUCAGC | 24 | 19725 |
| HSV1-UL54-2737 | - | AUCCUAGACACGCACCGC | 18 | 19726 |
| HSV1-UL54-2738 | - | AAUCCUAGACACGCACCGC | 19 | 19727 |
| HSV1-UL54-974 | - | AAAUCCUAGACACGCACCGC | 20 | 7633 |
| HSV1-UL54-2739 | - | GAAAUCCUAGACACGCACCGC | 21 | 19728 |
| HSV1-UL54-2740 | - | CGAAAUCCUAGACACGCACCGC | 22 | 19729 |
| HSV1-UL54-2741 | - | UCGAAAUCCUAGACACGCACCGC | 23 | 19730 |
| HSV1-UL54-2742 | - | AUCGAAAUCCUAGACACGCACCGC | 24 | 19731 |
| HSV1-UL54-2743 | - | CCAGUGCUACCUGAAGGC | 18 | 19732 |
| HSV1-UL54-2744 | - | UCCAGUGCUACCUGAAGGC | 19 | 19733 |
| HSV1-UL54-956 | - | CUCCAGUGCUACCUGAAGGC | 20 | 7619 |
| HSV1-UL54-2745 | - | UCUCCAGUGCUACCUGAAGGC | 21 | 19734 |
| HSV1-UL54-2746 | - | UUCUCCAGUGCUACCUGAAGGC | 22 | 19735 |
| HSV1-UL54-2747 | - | UUUCUCCAGUGCUACCUGAAGGC | 23 | 19736 |
| HSV1-UL54-2748 | - | CUUUCUCCAGUGCUACCUGAAGGC | 24 | 19737 |
| HSV1-UL54-2749 | - | ACCUUGGUCGCCCACGGC | 18 | 19738 |
| HSV1-UL54-2750 | - | AACCUUGGUCGCCCACGGC | 19 | 19739 |
| HSV1-UL54-946 | - | AAACCUUGGUCGCCCACGGC | 20 | 7610 |
| HSV1-UL54-2751 | - | GAAACCUUGGUCGCCCACGGC | 21 | 19740 |
| HSV1-UL54-2752 | - | GGAAACCUUGGUCGCCCACGGC | 22 | 19741 |
| HSV1-UL54-2753 | - | GGGAAACCUUGGUCGCCCACGGC | 23 | 19742 |
| HSV1-UL54-2754 | - | UGGGAAACCUUGGUCGCCCACGGC | 24 | 19743 |
| HSV1-UL54-2755 | - | GCGCGAGGCCUGUGCGGC | 18 | 19744 |
| HSV1-UL54-2756 | - | GGCGCGAGGCCUGUGCGGC | 19 | 19745 |
| HSV1-UL54-957 | - | AGGCGCGAGGCCUGUGCGGC | 20 | 7620 |
| HSV1-UL54-2757 | - | AAGGCGCGAGGCCUGUGCGGC | 21 | 19746 |
| HSV1-UL54-2758 | - | GAAGGCGCGAGGCCUGUGCGGC | 22 | 19747 |
| HSV1-UL54-2759 | - | UGAAGGCGCGAGGCCUGUGCGGC | 23 | 19748 |
| HSV1-UL54-2760 | - | CUGAAGGCGCGAGGCCUGUGCGGC | 24 | 19749 |
| HSV1-UL54-2761 | - | CGAGACCAGACGGGUCUC | 18 | 19750 |
| HSV1-UL54-2762 | - | CCGAGACCAGACGGGUCUC | 19 | 19751 |
| HSV1-UL54-943 | - | GCCGAGACCAGACGGGUCUC | 20 | 7609 |
| HSV1-UL54-2763 | - | CGCCGAGACCAGACGGGUCUC | 21 | 19752 |
| HSV1-UL54-2764 | - | ACGCCGAGACCAGACGGGUCUC | 22 | 19753 |
| HSV1-UL54-2765 | - | GACGCCGAGACCAGACGGGUCUC | 23 | 19754 |
| HSV1-UL54-2766 | - | UGACGCCGAGACCAGACGGGUCUC | 24 | 19755 |
| HSV1-UL54-2767 | - | CGCGUCGAGCGUGGCGUC | 18 | 19756 |
| HSV1-UL54-2768 | - | CCGCGUCGAGCGUGGCGUC | 19 | 19757 |
| HSV1-UL54-962 | - | ACCGCGUCGAGCGUGGCGUC | 20 | 7625 |
| HSV1-UL54-2769 | - | AACCGCGUCGAGCGUGGCGUC | 21 | 19758 |
| HSV1-UL54-2770 | - | CAACCGCGUCGAGCGUGGCGUC | 22 | 19759 |
| HSV1-UL54-2771 | - | CCAACCGCGUCGAGCGUGGCGUC | 23 | 19760 |
| HSV1-UL54-2772 | - | GCCAACCGCGUCGAGCGUGGCGUC | 24 | 19761 |
| HSV1-UL54-2773 | - | CGGUGCUGGCGGGCCAAG | 18 | 19762 |
| HSV1-UL54-2774 | - | CCGGUGCUGGCGGGCCAAG | 19 | 19763 |
| HSV1-UL54-940 | - | CCCGGUGCUGGCGGGCCAAG | 20 | 7606 |
| HSV1-UL54-2775 | - | CCCCGGUGCUGGCGGGCCAAG | 21 | 19764 |
| HSV1-UL54-2776 | - | GCCCCGGUGCUGGCGGGCCAAG | 22 | 19765 |
| HSV1-UL54-2777 | - | GGCCCCGGUGCUGGCGGGCCAAG | 23 | 19766 |
| HSV1-UL54-2778 | - | GGGCCCCGGUGCUGGCGGGCCAAG | 24 | 19767 |
| HSV1-UL54-2779 | - | AAGGAGGGCCCUUUGACG | 18 | 19768 |
| HSV1-UL54-2780 | - | CAAGGAGGGCCCUUUGACG | 19 | 19769 |
| HSV1-UL54-941 | - | CCAAGGAGGGCCCUUUGACG | 20 | 7607 |
| HSV1-UL54-2781 | - | GCCAAGGAGGGCCCUUUGACG | 21 | 19770 |
| HSV1-UL54-2782 | - | GGCCAAGGAGGGCCCUUUGACG | 22 | 19771 |
| HSV1-UL54-2783 | - | GGGCCAAGGAGGGCCCUUUGACG | 23 | 19772 |
| HSV1-UL54-2784 | - | CGGGCCAAGGAGGGCCCUUUGACG | 24 | 19773 |
| HSV1-UL54-2785 | - | AGGCGCUGGCCUCCGCCG | 18 | 19774 |
| HSV1-UL54-2786 | - | GAGGCGCUGGCCUCCGCCG | 19 | 19775 |
| HSV1-UL54-950 | - | CGAGGCGCUGGCCUCCGCCG | 20 | 7614 |
| HSV1-UL54-2787 | - | UCGAGGCGCUGGCCUCCGCCG | 21 | 19776 |
| HSV1-UL54-2788 | - | AUCGAGGCGCUGGCCUCCGCCG | 22 | 19777 |
| HSV1-UL54-2789 | - | CAUCGAGGCGCUGGCCUCCGCCG | 23 | 19778 |
| HSV1-UL54-2790 | - | UCAUCGAGGCGCUGGCCUCCGCCG | 24 | 19779 |
| HSV1-UL54-2791 | - | CCAGGCUCGCCAACCGCG | 18 | 19780 |
| HSV1-UL54-2792 | - | GCCAGGCUCGCCAACCGCG | 19 | 19781 |
| HSV1-UL54-961 | - | GGCCAGGCUCGCCAACCGCG | 20 | 7624 |
| HSV1-UL54-2793 | - | UGGCCAGGCUCGCCAACCGCG | 21 | 19782 |
| HSV1-UL54-2794 | - | CUGGCCAGGCUCGCCAACCGCG | 22 | 19783 |
| HSV1-UL54-2795 | - | UCUGGCCAGGCUCGCCAACCGCG | 23 | 19784 |
| HSV1-UL54-2796 | - | UUCUGGCCAGGCUCGCCAACCGCG | 24 | 19785 |
| HSV1-UL54-2797 | - | GCGUCGAGCGUGGCGUCG | 18 | 19786 |
| HSV1-UL54-2798 | - | CGCGUCGAGCGUGGCGUCG | 19 | 19787 |
| HSV1-UL54-331 | - | CCGCGUCGAGCGUGGCGUCG | 20 | 7094 |
| HSV1-UL54-2799 | - | ACCGCGUCGAGCGUGGCGUCG | 21 | 19788 |
| HSV1-UL54-2800 | - | AACCGCGUCGAGCGUGGCGUCG | 22 | 19789 |
| HSV1-UL54-2801 | - | CAACCGCGUCGAGCGUGGCGUCG | 23 | 19790 |
| HSV1-UL54-2802 | - | CCAACCGCGUCGAGCGUGGCGUCG | 24 | 19791 |
| HSV1-UL54-2803 | - | CCCUUGGUGUCGGGGUCG | 18 | 19792 |
| HSV1-UL54-2804 | - | ACCCUUGGUGUCGGGGUCG | 19 | 19793 |
| HSV1-UL54-968 | - | GACCCUUGGUGUCGGGGUCG | 20 | 7628 |
| HSV1-UL54-2805 | - | CGACCCUUGGUGUCGGGGUCG | 21 | 19794 |
| HSV1-UL54-2806 | - | GCGACCCUUGGUGUCGGGGUCG | 22 | 19795 |
| HSV1-UL54-2807 | - | CGCGACCCUUGGUGUCGGGGUCG | 23 | 19796 |
| HSV1-UL54-2808 | - | ACGCGACCCUUGGUGUCGGGGUCG | 24 | 19797 |
| HSV1-UL54-2809 | - | GGGGGACCCCGCCCCGGG | 18 | 19798 |
| HSV1-UL54-2810 | - | UGGGGGACCCCGCCCCGGG | 19 | 19799 |
| HSV1-UL54-914 | - | CUGGGGGACCCCGCCCCGGG | 20 | 7588 |
| HSV1-UL54-2811 | - | CCUGGGGGACCCCGCCCCGGG | 21 | 19800 |
| HSV1-UL54-2812 | - | CCCUGGGGGACCCCGCCCCGGG | 22 | 19801 |
| HSV1-UL54-2813 | - | ACCCUGGGGGACCCCGCCCCGGG | 23 | 19802 |
| HSV1-UL54-2814 | - | AACCCUGGGGGACCCCGCCCCGGG | 24 | 19803 |
| HSV1-UL54-2815 | - | ACCCCGCCCCGGGGCGGG | 18 | 19804 |
| HSV1-UL54-2816 | - | GACCCCGCCCCGGGGCGGG | 19 | 19805 |
| HSV1-UL54-916 | - | GGACCCCGCCCCGGGGCGGG | 20 | 7589 |
| HSV1-UL54-2817 | - | GGGACCCCGCCCCGGGGCGGG | 21 | 19806 |
| HSV1-UL54-2818 | - | GGGGACCCCGCCCCGGGGCGGG | 22 | 19807 |
| HSV1-UL54-2819 | - | GGGGGACCCCGCCCCGGGGCGGG | 23 | 19808 |
| HSV1-UL54-2820 | - | UGGGGGACCCCGCCCCGGGGCGGG | 24 | 19809 |
| HSV1-UL54-2821 | - | CGACCCUUGGUGUCGGGG | 18 | 19810 |
| HSV1-UL54-2822 | - | GCGACCCUUGGUGUCGGGG | 19 | 19811 |
| HSV1-UL54-966 | - | CGCGACCCUUGGUGUCGGGG | 20 | 7627 |
| HSV1-UL54-2823 | - | ACGCGACCCUUGGUGUCGGGG | 21 | 19812 |
| HSV1-UL54-2824 | - | UACGCGACCCUUGGUGUCGGGG | 22 | 19813 |
| HSV1-UL54-2825 | - | CUACGCGACCCUUGGUGUCGGGG | 23 | 19814 |
| HSV1-UL54-2826 | - | ACUACGCGACCCUUGGUGUCGGGG | 24 | 19815 |
| HSV1-UL54-2827 | - | GAGGCCUGUGCGGCCUGG | 18 | 19816 |
| HSV1-UL54-2828 | - | CGAGGCCUGUGCGGCCUGG | 19 | 19817 |
| HSV1-UL54-958 | - | GCGAGGCCUGUGCGGCCUGG | 20 | 7621 |
| HSV1-UL54-2829 | - | CGCGAGGCCUGUGCGGCCUGG | 21 | 19818 |
| HSV1-UL54-2830 | - | GCGCGAGGCCUGUGCGGCCUGG | 22 | 19819 |
| HSV1-UL54-2831 | - | GGCGCGAGGCCUGUGCGGCCUGG | 23 | 19820 |
| HSV1-UL54-2832 | - | AGGCGCGAGGCCUGUGCGGCCUGG | 24 | 19821 |
| HSV1-UL54-2833 | - | CGCCCCGGGGCGGGGUGG | 18 | 19822 |
| HSV1-UL54-2834 | - | CCGCCCCGGGGCGGGGUGG | 19 | 19823 |
| HSV1-UL54-917 | - | CCCGCCCCGGGGCGGGGUGG | 20 | 7590 |
| HSV1-UL54-2835 | - | CCCCGCCCCGGGGCGGGGUGG | 21 | 19824 |
| HSV1-UL54-2836 | - | ACCCCGCCCCGGGGCGGGGUGG | 22 | 19825 |
| HSV1-UL54-2837 | - | GACCCCGCCCCGGGGCGGGGUGG | 23 | 19826 |
| HSV1-UL54-2838 | - | GGACCCCGCCCCGGGGCGGGGUGG | 24 | 19827 |
| HSV1-UL54-2839 | - | GCCCCGGCGCCCCCCAUG | 18 | 19828 |
| HSV1-UL54-2840 | - | GGCCCCGGCGCCCCCCAUG | 19 | 19829 |
| HSV1-UL54-918 | - | CGGCCCCGGCGCCCCCCAUG | 20 | 7591 |
| HSV1-UL54-2841 | - | ACGGCCCCGGCGCCCCCCAUG | 21 | 19830 |
| HSV1-UL54-2842 | - | GACGGCCCCGGCGCCCCCCAUG | 22 | 19831 |
| HSV1-UL54-2843 | - | GGACGGCCCCGGCGCCCCCCAUG | 23 | 19832 |
| HSV1-UL54-2844 | - | CGGACGGCCCCGGCGCCCCCCAUG | 24 | 19833 |
| HSV1-UL54-2845 | - | CUCCCCGGGGCCUGCAUG | 18 | 19834 |
| HSV1-UL54-2846 | - | CCUCCCCGGGGCCUGCAUG | 19 | 19835 |
| HSV1-UL54-972 | - | ACCUCCCCGGGGCCUGCAUG | 20 | 7631 |
| HSV1-UL54-2847 | - | UACCUCCCCGGGGCCUGCAUG | 21 | 19836 |
| HSV1-UL54-2848 | - | CUACCUCCCCGGGGCCUGCAUG | 22 | 19837 |
| HSV1-UL54-2849 | - | UCUACCUCCCCGGGGCCUGCAUG | 23 | 19838 |
| HSV1-UL54-2850 | - | UUCUACCUCCCCGGGGCCUGCAUG | 24 | 19839 |
| HSV1-UL54-1900 | - | GAACCAAUCGCAACCCUG | 18 | 18889 |
| HSV1-UL54-1901 | - | AGAACCAAUCGCAACCCUG | 19 | 18890 |
| HSV1-UL54-910 | - | CAGAACCAAUCGCAACCCUG | 20 | 7585 |
| HSV1-UL54-1902 | - | CCAGAACCAAUCGCAACCCUG | 21 | 18891 |
| HSV1-UL54-1903 | - | CCCAGAACCAAUCGCAACCCUG | 22 | 18892 |
| HSV1-UL54-1904 | - | CCCCAGAACCAAUCGCAACCCUG | 23 | 18893 |
| HSV1-UL54-1905 | - | CCCCCAGAACCAAUCGCAACCCUG | 24 | 18894 |
| HSV1-UL54-2851 | - | CCCUGGGCCCCGGUGCUG | 18 | 19840 |
| HSV1-UL54-2852 | - | CCCCUGGGCCCCGGUGCUG | 19 | 19841 |
| HSV1-UL54-937 | - | GCCCCUGGGCCCCGGUGCUG | 20 | 7604 |
| HSV1-UL54-2853 | - | AGCCCCUGGGCCCCGGUGCUG | 21 | 19842 |
| HSV1-UL54-2854 | - | UAGCCCCUGGGCCCCGGUGCUG | 22 | 19843 |
| HSV1-UL54-2855 | - | AUAGCCCCUGGGCCCCGGUGCUG | 23 | 19844 |
| HSV1-UL54-2856 | - | AAUAGCCCCUGGGCCCCGGUGCUG | 24 | 19845 |
| HSV1-UL54-2857 | - | UGUUCGCGGCGGCGUCUG | 18 | 19846 |
| HSV1-UL54-2858 | - | GUGUUCGCGGCGGCGUCUG | 19 | 19847 |
| HSV1-UL54-959 | - | UGUGUUCGCGGCGGCGUCUG | 20 | 7622 |
| HSV1-UL54-2859 | - | CUGUGUUCGCGGCGGCGUCUG | 21 | 19848 |
| HSV1-UL54-2860 | - | ACUGUGUUCGCGGCGGCGUCUG | 22 | 19849 |
| HSV1-UL54-2861 | - | AACUGUGUUCGCGGCGGCGUCUG | 23 | 19850 |
| HSV1-UL54-2862 | - | GAACUGUGUUCGCGGCGGCGUCUG | 24 | 19851 |
| HSV1-UL54-2863 | - | ACUACGCGACCCUUGGUG | 18 | 19852 |
| HSV1-UL54-2864 | - | GACUACGCGACCCUUGGUG | 19 | 19853 |
| HSV1-UL54-964 | - | CGACUACGCGACCCUUGGUG | 20 | 7626 |
| HSV1-UL54-2865 | - | UCGACUACGCGACCCUUGGUG | 21 | 19854 |
| HSV1-UL54-2866 | - | AUCGACUACGCGACCCUUGGUG | 22 | 19855 |
| HSV1-UL54-2867 | - | GAUCGACUACGCGACCCUUGGUG | 23 | 19856 |
| HSV1-UL54-2868 | - | AGAUCGACUACGCGACCCUUGGUG | 24 | 19857 |
| HSV1-UL54-2869 | - | UCAUGCACGACCCCUUUG | 18 | 19858 |
| HSV1-UL54-2870 | - | GUCAUGCACGACCCCUUUG | 19 | 19859 |
| HSV1-UL54-287 | - | GGUCAUGCACGACCCCUUUG | 20 | 7050 |
| HSV1-UL54-2871 | - | AGGUCAUGCACGACCCCUUUG | 21 | 19860 |
| HSV1-UL54-2872 | - | CAGGUCAUGCACGACCCCUUUG | 22 | 19861 |
| HSV1-UL54-2873 | - | ACAGGUCAUGCACGACCCCUUUG | 23 | 19862 |
| HSV1-UL54-2874 | - | CACAGGUCAUGCACGACCCCUUUG | 24 | 19863 |
| HSV1-UL54-2875 | - | CCCCCAGGACCCCAUUAU | 18 | 19864 |
| HSV1-UL54-2876 | - | GCCCCCAGGACCCCAUUAU | 19 | 19865 |
| HSV1-UL54-314 | - | CGCCCCCAGGACCCCAUUAU | 20 | 7077 |
| HSV1-UL54-2877 | - | GCGCCCCCAGGACCCCAUUAU | 21 | 19866 |
| HSV1-UL54-2878 | - | UGCGCCCCCAGGACCCCAUUAU | 22 | 19867 |
| HSV1-UL54-2879 | - | CUGCGCCCCCAGGACCCCAUUAU | 23 | 19868 |
| HSV1-UL54-2880 | - | GCUGCGCCCCCAGGACCCCAUUAU | 24 | 19869 |
| HSV1-UL54-1918 | - | AGAACCAAUCGCAACCCU | 18 | 18907 |
| HSV1-UL54-1919 | - | CAGAACCAAUCGCAACCCU | 19 | 18908 |
| HSV1-UL54-1204 | - | CCAGAACCAAUCGCAACCCU | 20 | 18193 |
| HSV1-UL54-1920 | - | CCCAGAACCAAUCGCAACCCU | 21 | 18909 |
| HSV1-UL54-1921 | - | CCCCAGAACCAAUCGCAACCCU | 22 | 18910 |
| HSV1-UL54-1922 | - | CCCCCAGAACCAAUCGCAACCCU | 23 | 18911 |
| HSV1-UL54-1923 | - | GCCCCCAGAACCAAUCGCAACCCU | 24 | 18912 |
| HSV1-UL54-2881 | - | AGGUCAUGCACGACCCCU | 18 | 19870 |
| HSV1-UL54-2882 | - | CAGGUCAUGCACGACCCCU | 19 | 19871 |
| HSV1-UL54-931 | - | ACAGGUCAUGCACGACCCCU | 20 | 7601 |
| HSV1-UL54-2883 | - | CACAGGUCAUGCACGACCCCU | 21 | 19872 |
| HSV1-UL54-2884 | - | GCACAGGUCAUGCACGACCCCU | 22 | 19873 |
| HSV1-UL54-2885 | - | CGCACAGGUCAUGCACGACCCCU | 23 | 19874 |
| HSV1-UL54-2886 | - | GCGCACAGGUCAUGCACGACCCCU | 24 | 19875 |
| HSV1-UL54-2887 | - | AGACCAGACGGGUCUCCU | 18 | 19876 |
| HSV1-UL54-2888 | - | GAGACCAGACGGGUCUCCU | 19 | 19877 |
| HSV1-UL54-302 | - | CGAGACCAGACGGGUCUCCU | 20 | 7065 |
| HSV1-UL54-2889 | - | CCGAGACCAGACGGGUCUCCU | 21 | 19878 |
| HSV1-UL54-2890 | - | GCCGAGACCAGACGGGUCUCCU | 22 | 19879 |
| HSV1-UL54-2891 | - | CGCCGAGACCAGACGGGUCUCCU | 23 | 19880 |
| HSV1-UL54-2892 | - | ACGCCGAGACCAGACGGGUCUCCU | 24 | 19881 |
| HSV1-UL54-2893 | - | UGGUCCUGCGCUCCAUCU | 18 | 19882 |
| HSV1-UL54-2894 | - | UUGGUCCUGCGCUCCAUCU | 19 | 19883 |
| HSV1-UL54-928 | - | GUUGGUCCUGCGCUCCAUCU | 20 | 7598 |
| HSV1-UL54-2895 | - | GGUUGGUCCUGCGCUCCAUCU | 21 | 19884 |
| HSV1-UL54-2896 | - | CGGUUGGUCCUGCGCUCCAUCU | 22 | 19885 |
| HSV1-UL54-2897 | - | GCGGUUGGUCCUGCGCUCCAUCU | 23 | 19886 |
| HSV1-UL54-2898 | - | CGCGGUUGGUCCUGCGCUCCAUCU | 24 | 19887 |
| HSV1-UL54-2899 | - | GACCCUUGGUGUCGGGGU | 18 | 19888 |
| HSV1-UL54-2900 | - | CGACCCUUGGUGUCGGGGU | 19 | 19889 |
| HSV1-UL54-336 | - | GCGACCCUUGGUGUCGGGGU | 20 | 7099 |
| HSV1-UL54-2901 | - | CGCGACCCUUGGUGUCGGGGU | 21 | 19890 |
| HSV1-UL54-2902 | - | ACGCGACCCUUGGUGUCGGGGU | 22 | 19891 |
| HSV1-UL54-2903 | - | UACGCGACCCUUGGUGUCGGGGU | 23 | 19892 |
| HSV1-UL54-2904 | - | CUACGCGACCCUUGGUGUCGGGGU | 24 | 19893 |
| HSV1-UL54-2905 | - | CGGCGUCUGGCGGACAUU | 18 | 19894 |
| HSV1-UL54-2906 | - | GCGGCGUCUGGCGGACAUU | 19 | 19895 |
| HSV1-UL54-960 | - | GGCGGCGUCUGGCGGACAUU | 20 | 7623 |
| HSV1-UL54-2907 | - | CGGCGGCGUCUGGCGGACAUU | 21 | 19896 |
| HSV1-UL54-2908 | - | GCGGCGGCGUCUGGCGGACAUU | 22 | 19897 |
| HSV1-UL54-2909 | - | CGCGGCGGCGUCUGGCGGACAUU | 23 | 19898 |
| HSV1-UL54-2910 | - | UCGCGGCGGCGUCUGGCGGACAUU | 24 | 19899 |
| HSV1-UL54-2911 | - | GGUCAUGCACGACCCCUU | 18 | 19900 |
| HSV1-UL54-2912 | - | AGGUCAUGCACGACCCCUU | 19 | 19901 |
| HSV1-UL54-285 | - | CAGGUCAUGCACGACCCCUU | 20 | 7048 |
| HSV1-UL54-2913 | - | ACAGGUCAUGCACGACCCCUU | 21 | 19902 |
| HSV1-UL54-2914 | - | CACAGGUCAUGCACGACCCCUU | 22 | 19903 |
| HSV1-UL54-2915 | - | GCACAGGUCAUGCACGACCCCUU | 23 | 19904 |
| HSV1-UL54-2916 | - | CGCACAGGUCAUGCACGACCCCUU | 24 | 19905 |
| HSV1-UL54-2917 | - | GUCAUGCACGACCCCUUU | 18 | 19906 |
| HSV1-UL54-2918 | - | GGUCAUGCACGACCCCUUU | 19 | 19907 |
| HSV1-UL54-286 | - | AGGUCAUGCACGACCCCUUU | 20 | 7049 |
| HSV1-UL54-2919 | - | CAGGUCAUGCACGACCCCUUU | 21 | 19908 |
| HSV1-UL54-2920 | - | ACAGGUCAUGCACGACCCCUUU | 22 | 19909 |
| HSV1-UL54-2921 | - | CACAGGUCAUGCACGACCCCUUU | 23 | 19910 |
| HSV1-UL54-2922 | - | GCACAGGUCAUGCACGACCCCUUU | 24 | 19911 |

**Table 16A** provides exemplary targeting domains for knocking out the *UL54* gene selected according to the first tier parameters. The targeting domains bind within the first 500 bp of the coding sequence (e.g., within 500 bp downstream from the start codon), have a high level of orthogonality and start with a 5'G. It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 16A**

| 1 st Tier | | | | |
|---|---|---|---|---|
| gRNA Name | DNA Strand | Targeting Domain | Target Site Length | Seq ID NO: |
| HSV1-UL54-2923 | + | GAACACUCCCCGCUGCU | 17 | 19912 |
| HSV1-UL54-2924 | + | GAGGAACACUCCCCGCUGCU | 20 | 19913 |

**Table 16B** provides exemplary targeting domains for knocking out the *UL54* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 16B**

| 2nd Tier | | | | |
|---|---|---|---|---|
| gRNA Name | DNA Strand | Targeting Domain | Target Site Length | Seq ID NO: |
| HSV1-UL54-2925 | + | CGUCCGCCGCGGGCAGG | 17 | 19914 |
| HSV1-UL54-1505 | + | CGGCGUCCGCCGCGGGCAGG | 20 | 18494 |

**Table 16C** provides exemplary targeting domains for knocking out the *UL54* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase).

**Table 16C**

| 5th Tier | | | | |
|---|---|---|---|---|
| gRNA Name | DNA Strand | Targeting Domain | Target Site Length | Seq ID NO: |
| HSV1-UL54-2926 | + | UGGCGGUCGAUGCGGCC | 17 | 19915 |
| HSV1-UL54-1069 | - | CGGUCGACCGCAUCAGC | 17 | 7712 |
| HSV1-UL54-510 | - | CCCCCCGCUAAUGACGC | 17 | 7273 |
| HSV1-UL54-1183 | + | GGCGGGGUCCCCCAGGG | 17 | 7803 |
| HSV1-UL54-696 | + | AGC GUCAUUAGC GGGGG | 17 | 7459 |
| HSV1-UL54-2927 | + | CUCCUGGCGGUGCGUGU | 17 | 19916 |
| HSV1-UL54-2928 | + | CCUUGGCGGUCGAUGCGGCC | 20 | 19917 |
| HSV1-UL54-274 | - | ACCCCCCCCGCUAAUGACGC | 20 | 7037 |
| HSV1-UL54-2929 | + | CGGGGCGGGGUCCCCCAGGG | 20 | 19918 |
| HSV1-UL54-460 | + | GCCAGCGUCAUUAGCGGGGG | 20 | 7223 |
| HSV1-UL54-978 | + | ACACUCCUGGCGGUGCGUGU | 20 | 7636 |

### III. Cas9 Molecules

Cas9 molecules of a variety of species can be used in the methods and compositions described herein. While the *S. pyogenes, S. aureus* and *S. thermophilus* Cas9 molecules are the subject of much of the disclosure herein, Cas9 molecules of, derived from, or based on the Cas9 proteins of other species listed herein can be used as well. In other words, while the much of the description herein uses *S. pyogenes* and *S. thermophilus* Cas9 molecules, Cas9 molecules from the other species can replace them, e.g., *Staphylococcus aureus* and *Neisseria meningitidis* Cas9 molecules. Additional Cas9 species include: *Acidov*or*ax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Din*or*oseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsw*or*thii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp.,* or *Verminephrobacter eiseniae.*

A Cas9 molecule, or Cas9 polypeptide as that term is used herein, refers to a molecule or polypeptide that can interact with a guide RNA (gRNA) molecule and, in concert with the gRNA molecule, home or localizes to a site which comprises a target domain and PAM sequence. Cas9 molecule and Cas9 polypeptide, as those terms are used herein, refer to naturally occurring Cas9 molecules and to engineered, altered, or modified Cas9 molecules or Cas9 polypeptides that differ, e.g., by at least one amino acid residue, from a reference sequence, e.g., the most similar naturally occurring Cas9 molecule or a sequence of **Table 17** or 18.

### Cas9 Domains

Crystal structures have been determined for two different naturally occurring bacterial Cas9 molecules (Jinek et al., Science, 343(6176): 1247997, 2014) and for *S. pyogenes* Cas9 with a guide RNA (e.g., a synthetic fusion of crRNA and tracrRNA) (Nishimasu et al., Cell, 156:935-949, 2014; and Anders et al., Nature, 2014, doi: 10.1038/nature13579).

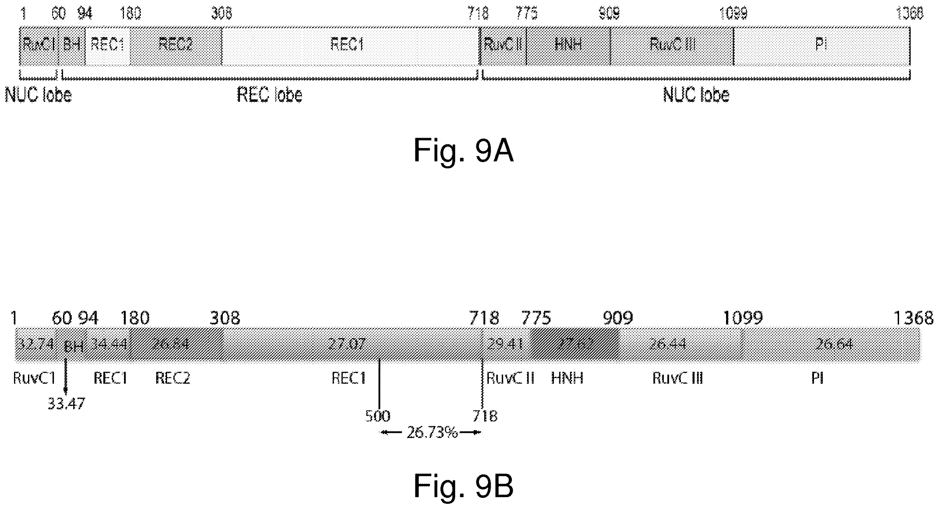

A naturally occurring Cas9 molecule comprises two lobes: a recognition (REC) lobe and a nuclease (NUC) lobe; each of which further comprises domains described herein. **Figs. 9A-9B** provide a schematic of the organization of important Cas9 domains in the primary structure. The domain nomenclature and the numbering of the amino acid residues encompassed by each domain used throughout this disclosure is as described in Nishimasu et al. The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long α helix and arginine rich region and comprises amino acids 60-93 of the sequence of *S*. *pyogenes* Cas9. The REC1 domain is important for recognition of the repeat:anti-repeat duplex, e.g., of a gRNA or a tracrRNA, and is therefore critical for Cas9 activity by recognizing the target sequence. The REC1 domain comprises two REC1 motifs at amino acids 94 to 179 and 308 to 717 of the sequence *of S. pyogenes* Cas9. These two REC1 domains, though separated by the REC2 domain in the linear primary structure, assemble in the tertiary structure to form the REC 1 domain. The REC2 domain, or parts thereof, may also play a role in the recognition of the repeat:anti-repeat duplex. The REC2 domain comprises amino acids 180-307 of the sequence *of S. pyogenes* Cas9.

The NUC lobe comprises the RuvC domain (also referred to herein as RuvC-like domain), the HNH domain (also referred to herein as HNH-like domain), and the PAM-interacting (PI) domain. The RuvC domain shares structural similarity to retroviral integrase superfamily members and cleaves a single strand, e.g., the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, e.g., the complementary strand of the target nucleic acid molecule. The HNH domain lies between the RuvC II-III motifs and comprises amino acids 775-908 of the sequence *of S. pyogenes* Cas9. The PI domain interacts with the PAM of the target nucleic acid molecule, and comprises amino acids 1099-1368 of the sequence *of S. pyogenes* Cas9.

### A RuvC-like domain and an HNH-like domain

In an embodiment, a Cas9 molecule or Cas9 polypeptide comprises an HNH-like domain and a RuvC-like domain. In an embodiment, cleavage activity is dependent on a RuvC-like domain and an HNH-like domain. A Cas9 molecule or Cas9 polypeptide, e.g., an eaCas9 molecule or eaCas9 polypeptide, can comprise one or more of the following domains: a RuvC-like domain and an HNH-like domain. In an embodiment, a Cas9 molecule or Cas9 polypeptide is an eaCas9 molecule or eaCas9 polypeptide and the eaCas9 molecule or eaCas9 polypeptide comprises a RuvC-like domain, e.g., a RuvC-like domain described below, and/or an HNH-like domain, e.g., an HNH-like domain described below.

### RuvC-like domains

In an embodiment, a RuvC-like domain cleaves, a single strand, e.g., the non-complementary strand of the target nucleic acid molecule. The Cas9 molecule or Cas9 polypeptide can include more than one RuvC-like domain (e.g., one, two, three or more RuvC-like domains). In an embodiment, a RuvC-like domain is at least 5, 6, 7, 8 amino acids in length but not more than 20, 19, 18, 17, 16 or 15 amino acids in length. In an embodiment, the Cas9 molecule or Cas9 polypeptide comprises an N-terminal RuvC-like domain of about 10 to 20 amino acids, e.g., about 15 amino acids in length.

### N-terminal RuvC-like domains

Some naturally occurring Cas9 molecules comprise more than one RuvC-like domain with cleavage being dependent on the N-terminal RuvC-like domain. Accordingly, Cas9 molecules or Cas9 polypeptide can comprise an N-terminal RuvC-like domain. Exemplary N-terminal RuvC-like domains are described below.

In an embodiment, an eaCas9 molecule or eaCas9 polypeptide comprises an N-terminal RuvC-like domain comprising an amino acid sequence of formula I:
D-X1-G-X2-X3-X4-X5-G-X6-X7-X8-X9 (SEQ ID NO: 8),
wherein,
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X4 is selected from S, Y, N and F (e.g., S);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R, or, e.g., selected from T, V, I, L and Δ).

In an embodiment, the N-terminal RuvC-like domain differs from a sequence of SEQ ID NO:8, by as many as 1 but no more than 2, 3, 4, or 5 residues.

In embodiment, the N-terminal RuvC-like domain is cleavage competent.

In embodiment, the N-terminal RuvC-like domain is cleavage incompetent.

In an embodiment, a eaCas9 molecule or eaCas9 polypeptide comprises an N-terminal RuvC-like domain comprising an amino acid sequence of formula II:
D-X1-G-X2-X3-S-X5-G-X6-X7-X8-X9, (SEQ ID NO: 9),
wherein
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:9 by as many as 1 but no more than 2, 3, 4, or 5 residues.

In an embodiment, the N-terminal RuvC-like domain comprises an amino acid sequence of formula III:
D-I-G-X2-X3-S-V-G-W-A-X8-X9 (SEQ ID NO: 10),
wherein
X2 is selected from T, I, V, S, N, Y, E and L (e.g., selected from T, V, and I);
X3 is selected from N, S, G, A, D, T, R, M and F (e.g., A or N);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:10 by as many as 1 but no more than, 2, 3, 4, or 5 residues.

In an embodiment, the N-terminal RuvC-like domain comprises an amino acid sequence of formula III:
D-I-G-T-N-S-V-G-W-A-V-X (SEQ ID NO: 11),
wherein
X is a non-polar alkyl amino acid or a hydroxyl amino acid, e.g., X is selected from V, I, L and T (e.g., the eaCas9 molecule can comprise an N-terminal RuvC-like domain shown in **Figs. 2A-2G** (is depicted as Y)).

In an embodiment, the N-terminal RuvC-like domain differs from a sequence of SEQ ID NO:11 by as many as 1 but no more than, 2, 3, 4, or 5 residues.

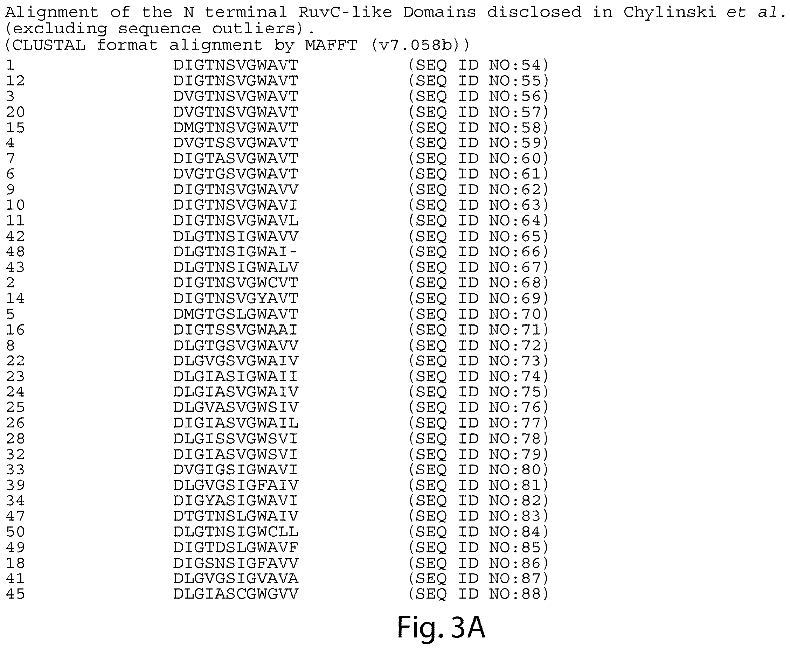

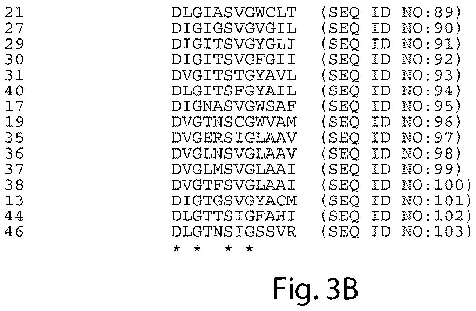

In an embodiment, the N-terminal RuvC-like domain differs from a sequence of an N-terminal RuvC like domain disclosed herein, e.g., in **Figs. 3A-3B** or **Figs. 7A-7B****,** as many as 1 but no more than 2, 3, 4, or 5 residues. In an embodiment, 1, 2, 3 or all of the highly conserved residues identified in **Figs. 3A-3B** or **Figs. 7A-7B** are present.

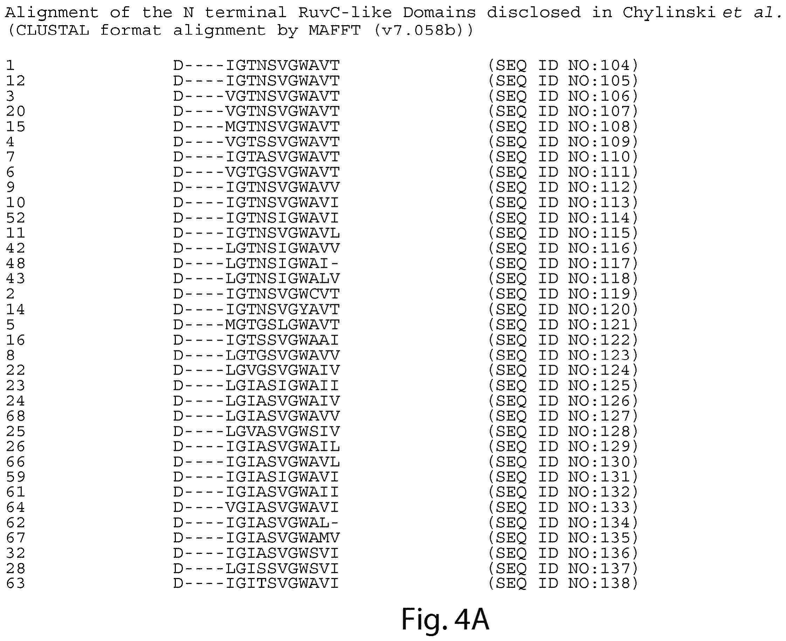

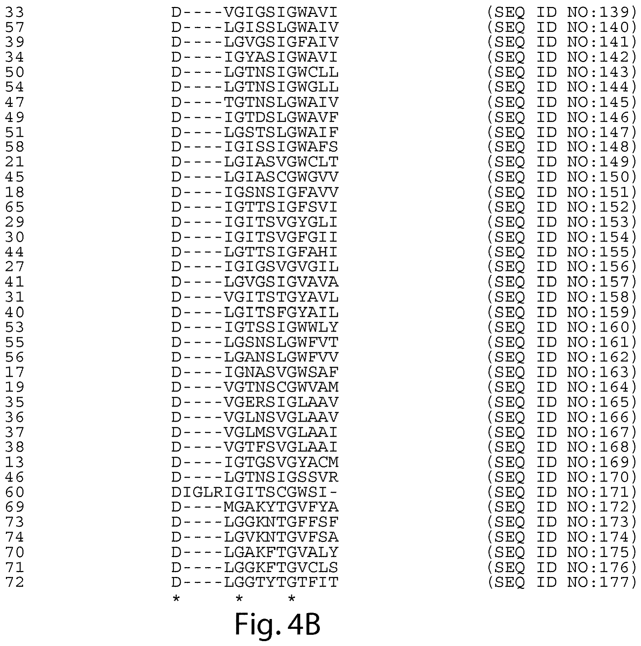

In an embodiment, the N-terminal RuvC-like domain differs from a sequence of an N-terminal RuvC-like domain disclosed herein, e.g., in **Figs. 4A-4B** or **Figs. 7A-7B****,** as many as 1 but no more than 2, 3, 4, or 5 residues. In an embodiment, 1, 2, or all of the highly conserved residues identified in **Figs. 4A-4B** or **Figs. 7A-7B** are present.

### Additional RuvC-like domains

In addition to the N-terminal RuvC-like domain, the Cas9 molecule or Cas9 polypeptide, e.g., an eaCas9 molecule or eaCas9 polypeptide, can comprise one or more additional RuvC-like domains. In an embodiment, the Cas9 molecule or Cas9 polypeptide can comprise two additional RuvC-like domains. Preferably, the additional RuvC-like domain is at least 5 amino acids in length and, e.g., less than 15 amino acids in length, e.g., 5 to 10 amino acids in length, e.g., 8 amino acids in length.

An additional RuvC-like domain can comprise an amino acid sequence:
I-X1-X2-E-X3-A-R-E (SEQ ID NO:12), wherein
X1 is V or H,
X2 is I, L or V (e.g., I or V); and
X3 is M or T.

In an embodiment, the additional RuvC-like domain comprises the amino acid sequence:
I-V-X2-E-M-A-R-E (SEQ ID NO:13), wherein
X2 is I, L or V (e.g., I or V) (e.g., the eaCas9 molecule or eaCas9 polypeptide can comprise an additional RuvC-like domain shown in **Fig. 2A-2G** or **Figs. 7A-7B** (depicted as B)).

An additional RuvC-like domain can comprise an amino acid sequence: H-H-A-X1-D-A-X2-X3 (SEQ ID NO: 14), wherein
X1 is H or L;
X2 is R or V; and
X3 is E or V.

In an embodiment, the additional RuvC-like domain comprises the amino acid sequence: H-H-A-H-D-A-Y-L (SEQ ID NO:15).

In an embodiment, the additional RuvC-like domain differs from a sequence of SEQ ID NO: 12, 13, 14 or 15 by as many as 1 but no more than 2, 3, 4, or 5 residues.

In some embodiments, the sequence flanking the N-terminal RuvC-like domain is a sequences of formula V:
K-X1'-Y-X2'-X3'-X4'-Z-T-D-X9'-Y, (SEQ ID NO: 16).
wherein
X1' is selected from K and P,
X2' is selected from V, L, I, and F (e.g., V, I and L);
X3' is selected from G, A and S (e.g., G),
X4' is selected from L, I, V and F (e.g., L);
X9' is selected from D, E, N and Q; and
Z is an N-terminal RuvC-like domain, e.g., as described above.

### HNH-like domains

In an embodiment, an HNH-like domain cleaves a single stranded complementary domain, e.g., a complementary strand of a double stranded nucleic acid molecule. In an embodiment, an HNH-like domain is at least 15, 20, 25 amino acids in length but not more than 40, 35 or 30 amino acids in length, e.g., 20 to 35 amino acids in length, e.g., 25 to 30 amino acids in length. Exemplary HNH-like domains are described below.

In an embodiment, an eaCas9 molecule or eaCas9 polypeptide comprises an HNH-like domain having an amino acid sequence of formula VI:
X1-X2-X3-H-X4-X5-P-X6-X7-X8-X9-X10-X11-X12-X13-X14-X15-N-X16-X17-X18-X19-X20-X21-X22-X23-N (SEQ ID NO: 17), wherein
X1 is selected from D, E, Q and N (e.g., D and E);
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X7 is selected from S, A, D, T and K (e.g., S and A);
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X11 is selected from D, S, N, R, L and T (e.g., D);
X12 is selected from D, N and S;
X13 is selected from S, A, T, G and R (e.g., S);
X14 is selected from I, L, F, S, R, Y, Q, W, D, K and H (e.g., I, L and F);
X15 is selected from D, S, I, N, E, A, H, F, L, Q, M, G, Y and V;
X16 is selected from K, L, R, M, T and F (e.g., L, R and K);
X17 is selected from V, L, I, A and T;
X18 is selected from L, I, V and A (e.g., L and I);
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, a HNH-like domain differs from a sequence of SEQ ID NO: 17 by at least one but no more than, 2, 3, 4, or 5 residues.

In an embodiment, the HNH-like domain is cleavage competent.

In an embodiment, the HNH-like domain is cleavage incompetent.

In an embodiment, an eaCas9 molecule or eaCas9 polypeptide comprises an HNH-like domain comprising an amino acid sequence of formula VII:
X1-X2-X3-H-X4-X5-P-X6-S-X8-X9-X10-D-D-S-X14-X15-N-K-V-L-X19-X20-X21-X22-X23-N (SEQ ID NO: 18),
wherein
X1 is selected from D and E;
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO: 18 by 1, 2, 3, 4, or 5 residues.

In an embodiment, an eaCas9 molecule or eaCas9 polypeptide comprises an HNH-like domain comprising an amino acid sequence of formula VII:
X1-V-X3-H-I-V-P-X6-S-X8-X9-X10-D-D-S-X14-X15-N-K-V-L-T-X20-X21-X22-X23-N (SEQ ID NO:19),
wherein
X1 is selected from D and E;
X3 is selected from D and E;
X6 is selected from Q, H, R, K, Y, I, L and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO: 19 by 1, 2, 3, 4, or 5 residues.

In an embodiment, an eaCas9 molecule or eaCas9 polypeptide comprises an HNH-like domain having an amino acid sequence of formula VIII:
D-X2-D-H-I-X5-P-Q-X7-F-X9-X10-D-X12-S-I-D-N-X16-V-L-X19-X20-S-X22-X23-N (SEQ ID NO:20),
wherein
X2 is selected from I and V;
X5 is selected from I and V;
X7 is selected from A and S;
X9 is selected from I and L;
X10 is selected from K and T;
X12 is selected from D and N;
X16 is selected from R, K and L; X19 is selected from T and V;
X20 is selected from S and R;
X22 is selected from K, D and A; and
X23 is selected from E, K, G and N (e.g., the eaCas9 molecule or eaCas9 polypeptide can comprise an HNH-like domain as described herein).

In an embodiment, the HNH-like domain differs from a sequence of SEQ ID NO: 20 by as many as 1 but no more than 2, 3, 4, or 5 residues.

In an embodiment, an eaCas9 molecule or eaCas9 polypeptide comprises the amino acid sequence of formula IX:
L-Y-Y-L-Q-N-G-X1'-D-M-Y-X2'-X3'-X4'-X5'-L-D-I-X6'-X7'-L-S-X8'-Y-Z-N-R-X9'-K-X10'-D-X11'-V-P (SEQ ID NO: 21),
wherein
X1' is selected from K and R;
X2' is selected from V and T;
X3' is selected from G and D;
X4' is selected from E, Q and D;
X5' is selected from E and D;
X6' is selected from D, N and H;
X7' is selected from Y, R and N;
X8' is selected from Q, D and N; X9' is selected from G and E;
X10' is selected from S and G;
X11' is selected from D and N; and
Z is an HNH-like domain, e.g., as described above.

In an embodiment, the eaCas9 molecule or eaCas9 polypeptide comprises an amino acid sequence that differs from a sequence of SEQ ID NO:21 by as many as 1 but no more than 2, 3, 4, or 5 residues.

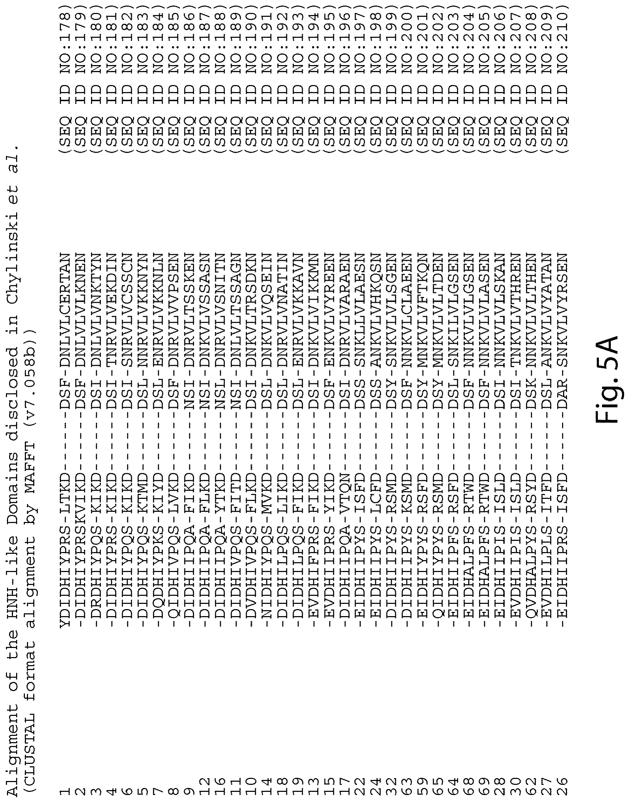

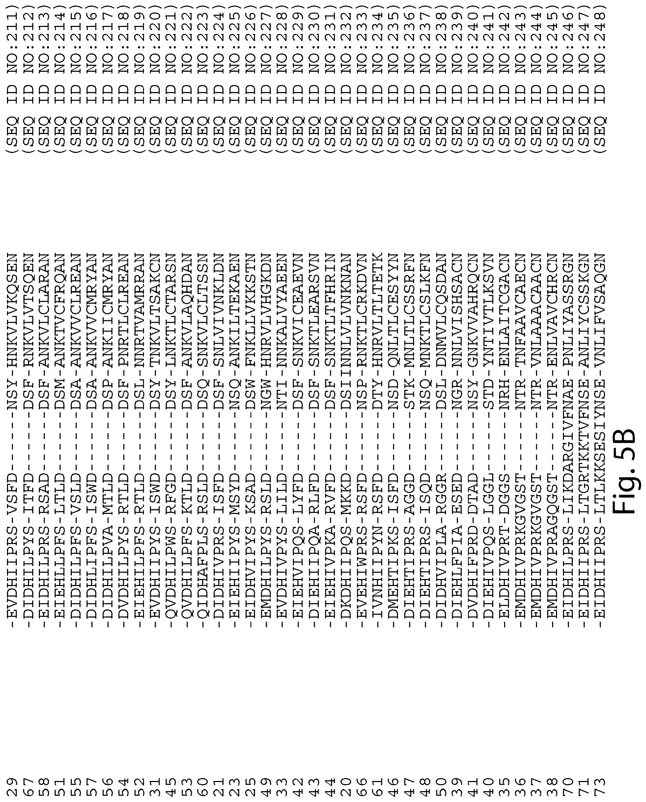

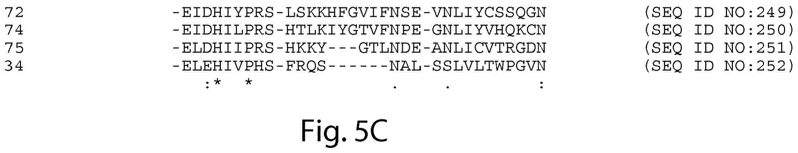

In an embodiment, the HNH-like domain differs from a sequence of an HNH-like domain disclosed herein, e.g., in **Figs. 5A-5C** or **Figs. 7A-7B****,** as many as 1 but no more than 2, 3, 4, or 5 residues. In an embodiment, 1 or both of the highly conserved residues identified in **Figs. 5A-5C** or **Figs. 7A-7B** are present.

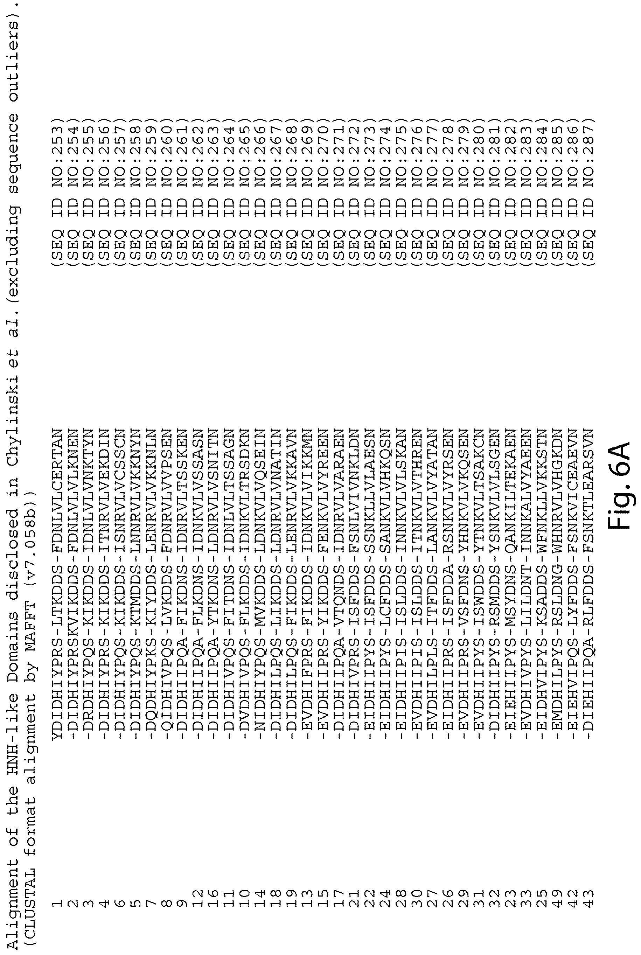

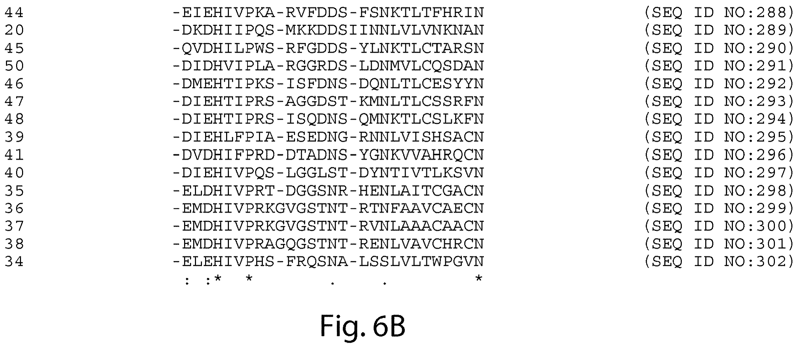

In an embodiment, the HNH -like domain differs from a sequence of an HNH-like domain disclosed herein, e.g., in **Figs. 6A-6B** or **Figs. 7A-7B****,** as many as 1 but no more than 2, 3, 4, or 5 residues. In an embodiment, 1, 2, all 3 of the highly conserved residues identified in **Figs. 6A-6B** or **Figs. 7A-7B** are present.

### Cas9 Activities

### Nuclease and Helicase Activities

In an embodiment, the Cas9 molecule or Cas9 polypeptide is capable of cleaving a target nucleic acid molecule. Typically wild type Cas9 molecules cleave both strands of a target nucleic acid molecule. Cas9 molecules and Cas9 polypeptides can be engineered to alter nuclease cleavage (or other properties), e.g., to provide a Cas9 molecule or Cas9 peolypeptide which is a nickase, or which lacks the ability to cleave target nucleic acid. A Cas9 molecule or Cas9 polypeptide that is capable of cleaving a target nucleic acid molecule is referred to herein as an eaCas9 (an enzymatically active Cas9) molecule or eaCas9 polypeptide. In an embodiment, an eaCas9 molecule or eaCas9 polypeptide, comprises one or more of the following activities:
a nickase activity, i.e., the ability to cleave a single strand, e.g., the non-complementary strand or the complementary strand, of a nucleic acid molecule;
a double stranded nuclease activity, i.e., the ability to cleave both strands of a double stranded nucleic acid and create a double stranded break, which in an embodiment is the presence of two nickase activities;
an endonuclease activity;
an exonuclease activity; and
a helicase activity, i.e., the ability to unwind the helical structure of a double stranded nucleic acid.

In an embodiment, an enzymatically active Cas9 or an eaCas9 molecule or an eaCas9 polypeptide cleaves both DNA strands and results in a double stranded break. In an embodiment, an eaCas9 molecule or eaCas9 polypeptide cleaves only one strand, e.g., the strand to which the gRNA hybridizes to, or the strand complementary to the strand the gRNA hybridizes with. In an embodiment, an eaCas9 molecule or eaCas9 polypeptide comprises cleavage activity associated with an HNH-like domain. In an embodiment, an eaCas9 molecule or eaCas9 polypeptide comprises cleavage activity associated with an N-terminal RuvC-like domain. In an embodiment, an eaCas9 molecule or eaCas9 polypeptide comprises cleavage activity associated with an HNH-like domain and cleavage activity associated with an N-terminal RuvC-like domain. In an embodiment, an eaCas9 molecule or eaCas9 polypeptide comprises an active, or cleavage competent, HNH-like domain and an inactive, or cleavage incompetent, N-terminal RuvC-like domain. In an embodiment, an eaCas9 molecule or eaCas9 polypeptide comprises an inactive, or cleavage incompetent, HNH-like domain and an active, or cleavage competent, N-terminal RuvC-like domain.

Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, e.g., less than 20, 10, 5, 1 or 0.1 % of the cleavage activity of a reference Cas9 molecule or eiCas9 polypeptide, as measured by an assay described herein.

### Targeting and PAMs

A Cas9 molecule or Cas9 polypeptide, is a polypeptide that can interact with a guide RNA (gRNA) molecule and, in concert with the gRNA molecule, localizes to a site which comprises a target domain and PAM sequence.

In an embodiment, the ability of an eaCas9 molecule or eaCas9 polypeptide to interact with and cleave a target nucleic acid is PAM sequence dependent. A PAM sequence is a sequence in the target nucleic acid. In an embodiment, cleavage of the target nucleic acid occurs upstream from the PAM sequence. EaCas9 molecules from different bacterial species can recognize different sequence motifs (e.g., PAM sequences). In an embodiment, an eaCas9 molecule *of S. pyogenes* recognizes the sequence motif NGG and directs cleavage of a target nucleic acid sequence 1 to 10, e.g., 3 to 5, base pairs upstream from that sequence. See, e.g., Mali et al., SCIENCE 2013; 339(6121): 823-826. In an embodiment, an eaCas9 molecule of *S. thermophilus* recognizes the sequence motif NGGNG and NNAGAAW (W = A or T) and directs cleavage of a core target nucleic acid sequence 1 to 10, e.g., 3 to 5, base pairs upstream from these sequences. See, e.g., Horvath et al., SCIENCE 2010; 327(5962):167-170, and Deveau et al., J BACTERIOL 2008; 190(4): 1390-1400. In an embodiment, an eaCas9 molecule of *S. mutans* recognizes the sequence motif NGG and/or NAAR (R = A or G) and directs cleavage of a core target nucleic acid sequence 1 to 10, e.g., 3 to 5 base pairs, upstream from this sequence. See, e.g., Deveau et al., J BACTERIOL 2008; 190(4): 1390-1400. In an embodiment, an eaCas9 molecule of *S. aureus* recognizes the sequence motif NNGRR (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRN (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRT (R = A or G) and directs cleavage of a target nucleic acid sequence 1 to 10, e.g., 3 to 5, base pairs upstream from that sequence. In an embodiment, an eaCas9 molecule of *S*. *aureus* recognizes the sequence motif NNGRRV (R = A or G, V = A, G or C) and directs cleavage of a target nucleic acid sequence 1 to 10, e.g., 3 to 5, base pairs upstream from that sequence. In an embodiment, an eaCas9 molecule of *Neisseria meningitidis* recognizes the sequence motif NNNNGATT or NNNGCTT and directs cleavage of a target nucleic acid sequence 1 to 10, e.g., 3 to 5, base pairs upstream from that sequence. See, e.g., Hou et al., PNAS Early Edition 2013, 1-6. The ability of a Cas9 molecule to recognize a PAM sequence can be determined, e.g., using a transformation assay described in Jinek et al., SCIENCE 2012 337:816. In the aforementioned embodiments, N can be any nucleotide residue, e.g., any of A, G, C or T.

As is discussed herein, Cas9 molecules can be engineered to alter the PAM specificity of the Cas9 molecule.

Exemplary naturally occurring Cas9 molecules are described in Chylinski et al., RNA BIOLOGY 2013 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. pyogenes* (e.g., strain SF370, MGAS10270, MGAS10750, MGAS2096, MGAS315, MGAS5005, MGAS6180, MGAS9429, NZ131 and SSI-1), *S. thermophilus* (e.g., strain LMD-9), *S. pseudoporcinus* (e.g., strain SPIN 20026), *S. mutans* (e.g., strain UA159, NN2025), *S. macacae* (e.g., strain NCTC11558), *S. gallolyticus* (e.g., strain UCN34, ATCC BAA-2069), *S. equines* (e.g., strain ATCC 9812, MGCS 124), *S. dysdalactiae* (e.g., strain GGS 124), *S. bovis* (e.g., strain ATCC 700338), *S. anginosus* (e.g., strain F0211), *S*. *agalactiae* (e.g., strain NEM316, A909), *Listeria monocytogenes* (e.g., strain F6854), *Listeria innocua* (*L. innocua,* e.g., strain Clip11262), *Enterococcus italicus* (e.g., strain DSM 15952), or *Enterococcus faecium* (e.g., strain 1,231,408). Additional exemplary Cas9 molecules are a Cas9 molecule of *Neisseria meningitidis* (Hou et al., PNAS Early Edition 2013, 1-6 and a *S*. *aureus* cas9 molecule.

In an embodiment, a Cas9 molecule or Cas9 polypeptide, e.g., an eaCas9 molecule or eaCas9 polypeptide, comprises an amino acid sequence:
having 60%, 65%, 70%, 75%, 80%, 85%, 90%, 95%, 96%, 97%, 98% or 99% homology with;
differs at no more than, 2, 5, 10, 15, 20, 30, or 40% of the amino acid residues when compared with;
differs by at least 1, 2, 5, 10 or 20 amino acids, but by no more than 100, 80, 70, 60, 50, 40 or 30 amino acids from; or
is identical to any Cas9 molecule sequence described herein, or a naturally occurring Cas9 molecule sequence, e.g., a Cas9 molecule from a species listed herein or described in Chylinski et al., RNA BIOLOGY 2013 10:5, 727-737; Hou et al., PNAS Early Edition 2013, 1-6; SEQ ID NO:1-4. In an embodiment, the Cas9 molecule or Cas9 polypeptide comprises one or more of the following activities: a nickase activity; a double stranded cleavage activity (e.g., an endonuclease and/or exonuclease activity); a helicase activity; or the ability, together with a gRNA molecule, to localize to a target nucleic acid.

In an embodiment, a Cas9 molecule or Cas9 polypeptide comprises any of the amino acid sequence of the consensus sequence of **Figs. 2A-2G****,** wherein "^{∗}" indicates any amino acid found in the corresponding position in the amino acid sequence of a Cas9 molecule or Cas9 polypeptide of *S. pyogenes, S. thermophilus, S. mutans* and *L. innocua,* and "-" indicates any amino acid. In an embodiment, a Cas9 molecule differs from the sequence of the consensus sequence of **Figs. 2A-2G** by at least 1, but no more than 2, 3, 4, 5, 6, 7, 8, 9, or 10 amino acid residues. In an embodiment, a Cas9 molecule or Cas9 polypeptide comprises the amino acid sequence of SEQ ID NO:7 of **Figs. 7A-7B****,** wherein "^{∗}" indicates any amino acid found in the corresponding position in the amino acid sequence of a Cas9 molecule or Cas9 polypeptide *of S. pyogenes,* or *N. meningitidis,* "-" indicates any amino acid, and "-" indicates any amino acid or absent. In an embodiment, a Cas9 molecule or Cas9 polypeptide differs from the sequence of SEQ ID NO:6 or 7 disclosed in **Figs. 7A-7B** by at least 1, but no more than 2, 3, 4, 5, 6, 7, 8, 9, or 10 amino acid residues.

A comparison of the sequence of a number of Cas9 molecules indicate that certain regions are conserved. These are identified below as:
region 1 (residues1 to 180, or in the case of region 1'residues 120 to 180)
region 2 ( residues360 to 480);
region 3 ( residues 660 to 720);
region 4 ( residues 817 to 900); and
region 5 (residues 900 to 960);

In an embodiment, a Cas9 molecule or Cas9 polypeptide comprises regions 1-5, together with sufficient additional Cas9 molecule sequence to provide a biologically active molecule, e.g., a Cas9 molecule having at least one activity described herein. In an embodiment, each of regions 1-5, independently, have 50%, 60%, 70%, 80%, 85%, 90%, 95%, 96%, 97%, 98% or 99% homology with the corresponding residues of a Cas9 molecule or Cas9 polypeptide described herein, e.g., a sequence from **Figs. 2A-2G** or from **Figs. 7A-****7B.**

In an embodiment, a Cas9 molecule or Cas9 polypeptide, e.g., an eaCas9 molecule or eaCas9 polypeptide, comprises an amino acid sequence referred to as region 1:
having 50%, 60%, 70%, 80%, 85%, 90%, 95%, 96%, 97%, 98% or 99% homology with amino acids 1-180 (the numbering is according to the motif sequence in **Figs. 2A-2G****;** 52% of residues in the four Cas9 sequences in **Figs. 2A-2G** are conserved) of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 of *S*. *pyogenes, S. thermophilus, S. mutans* or *Listeria innocua;* or
is identical to 1-180 of the amino acid sequence of Cas9 *of S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 1':
having 55%, 60%, 65%, 70%, 75%, 80%, 85%, 90%, 95%, 96%, 97%, 98% or 99% homology with amino acids 120-180 (55% of residues in the four Cas9 sequences in **Figs. 2A-2G** are conserved) of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 120-180 of the amino acid sequence of Cas9 *of S. pyogenes, S. thermophilus, S. mutans* or *L. innocua* ; or
is identical to 120-180 of the amino acid sequence of Cas9 *of S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 2:
having 50%, 55%, 60%, 65%, 70%, 75%, 80%, 85%, 90%, 95%, 96%, 97%, 98% or 99% homology with amino acids 360-480 (52% of residues in the four Cas9 sequences in **Figs. 2A-2G** are conserved) of the amino acid sequence of Cas9 *of S. pyogenes, S. thermophilus, S. mutans* or *L. innocua*;
differs by at least 1, 2, or 5 amino acids but by no more than 35, 30, 25, 20 or 10 amino acids from amino acids 360-480 of the amino acid sequence of Cas9 *of S. pyogenes, S. thermophilus, S. mutans* or *L. innocua*; or
is identical to 360-480 of the amino acid sequence of Cas9 *of S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 3:
having 55%, 60%, 65%, 70%, 75%, 80%, 85%, 90%, 95%, 96%, 97%, 98%, or 99% homology with amino acids 660-720 (56% of residues in the four Cas9 sequences in **Figs. 2A-2G** are conserved) of the amino acid sequence of Cas9 of *S. pyogenes, S. thermophilus, S. mutans* or *L. innocua*;
differs by at least 1, 2, or 5 amino acids but by no more than 35, 30, 25, 20 or 10 amino acids from amino acids 660-720 of the amino acid sequence of Cas9 *of S. pyogenes, S. thermophilus, S. mutans* or *L. innocua*; or
is identical to 660-720 of the amino acid sequence of Cas9 *of S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 4:
having 50%, 55%, 60%, 65%, 70%, 75%, 80%, 85%, 90%, 95%, 96%, 97%, 98%, or 99% homology with amino acids 817-900 (55% of residues in the four Cas9 sequences in **Figs. 2A-2G** are conserved) of the amino acid sequence of Cas9 *of S. pyogenes, S. thermophilus, S. mutans* or *L. innocua;*
differs by at least 1, 2, or 5 amino acids but by no more than 35, 30, 25, 20 or 10 amino acids from amino acids 817-900 of the amino acid sequence of Cas9 *of S. pyogenes, S. thermophilus, S. mutans* or *L. innocua;* or
is identical to 817-900 of the amino acid sequence of Cas9 *of S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 5:
having 50%, 55%, 60%, 65%, 70%, 75%, 80%, 85%, 90%, 95%, 96%, 97%, 98%, or 99% homology with amino acids 900-960 (60% of residues in the four Cas9 sequences in **Figs. 2A-2G** are conserved) of the amino acid sequence of Cas9 *of S. pyogenes, S. thermophilus, S. mutans* or *L. innocua*;
differs by at least 1, 2, or 5 amino acids but by no more than 35, 30, 25, 20 or 10 amino acids from amino acids 900-960 of the amino acid sequence of Cas9 *of S. pyogenes, S. thermophilus, S. mutans* or *L. innocua*; or
is identical to 900-960 of the amino acid sequence of Cas9 *of S. pyogenes, S. thermophilus, S. mutans* or *L. innocua.*

### Engineered or Altered Cas9 Molecules and Cas9 Polypeptides

Cas9 molecules and Cas9 polypeptides described herein, e.g., naturally occurring Cas9 molecules can possess any of a number of properties, including: nickase activity, nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In an embodiment, a Cas9 molecule or Cas9 polypeptide can include all or a subset of these properties. In typical embodiments, a Cas9 molecule or Cas9 polypeptide have the ability to interact with a gRNA molecule and, in concert with the gRNA molecule, localize to a site in a nucleic acid. Other activities, e.g., PAM specificity, cleavage activity, or helicase activity can vary more widely in Cas9 molecules and Cas9 polypeptides.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, e.g., altered nuclease activity, (as compared with a naturally occurring or other reference Cas9 molecule) or altered helicase activity. As discussed herein, an engineered Cas9 molecule or Cas9 polypeptide can have nickase activity (as opposed to double strand nuclease activity). In an embodiment an engineered Cas9 molecule or Cas9 polypeptide can have an alteration that alters its size, e.g., a deletion of amino acid sequence that reduces its size, e.g., without significant effect on one or more, or any Cas9 activity. In an embodiment, an engineered Cas9 molecule or Cas9 polypeptide can comprise an alteration that affects PAM recognition. E.g., an engineered Cas9 molecule can be altered to recognize a PAM sequence other than that recognized by the endogenous wild-type PI domain. In an embodiment, a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, e.g., by alteration of a parental, e.g., naturally occurring Cas9 molecules or Cas9 poypeptides to provide an altered Cas9 molecule or Cas9 polypeptide having a desired property. For example, one or more mutations or differences relative to a parental Cas9 molecule, e.g., a naturally occurring or engineered Cas9 molecule, can be introduced. Such mutations and differences comprise: substitutions (e.g., conservative substitutions or substitutions of non-essential amino acids); insertions; or deletions. In an embodiment, a Cas9 molecule or Cas9 polypeptide can comprises one or more mutations or differences, e.g., at least 1, 2, 3, 4, 5, 10, 15, 20, 30, 40 or 50 mutations, but less than 200, 100, or 80 mutations relative to a reference, e.g., a parental, Cas9 molecule.

In an embodiment, a mutation or mutations do not have a substantial effect on a Cas9 activity, e.g. a Cas9 activity described herein. In an embodiment, a mutation or mutations have a substantial effect on a Cas9 activity, e.g. a Cas9 activity described herein.

### Non-Cleaving and Modified-Cleavage Cas9 Molecules and Cas9 Polypeptides

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule *of S. pyogenes,* as follows: its ability to modulate, e.g., decreased or increased, cleavage of a double stranded nucleic acid (endonuclease and/or exonuclease activity), e.g., as compared to a naturally occurring Cas9 molecule (e.g., a Cas9 molecule of *S. pyogenes)*; its ability to modulate, e.g., decreased or increased, cleavage of a single strand of a nucleic acid, e.g., a non-complementary strand of a nucleic acid molecule or a complementary strand of a nucleic acid molecule (nickase activity) , e.g., as compared to a naturally occurring Cas9 molecule (e.g., a Cas9 molecule of *S. pyogenes);* or the ability to cleave a nucleic acid molecule, e.g., a double stranded or single stranded nucleic acid molecule, can be eliminated.

### Modified Cleavage eaCas9 Molecules and eaCas9 Polypeptides

In an embodiment, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In an embodiment, an eaCas9 molecule or eaCas9 polypeptide comprises an active, or cleavage competent, HNH-like domain (e.g., an HNH-like domain described herein, e.g., SEQ ID NO: 17, SEQ ID NO: 18, SEQ ID NO: 19, SEQ ID NO: 20 or SEQ ID NO: 21) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, e.g., an aspartic acid at position 9 of the consensus sequence disclosed in **Figs. 2A-2G** or an aspartic acid at position 10 of SEQ ID NO: 7, e.g., can be substituted with an alanine. In an embodiment, the eaCas9 molecule or eaCas9 polypeptide differs from wild type in the N-terminal RuvC-like domain and does not cleave the target nucleic acid, or cleaves with significantly less efficiency, e.g., less than 20, 10, 5, 1 or . 1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S*. *pyogenes,* or *S. thermophilus.* In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., a RuvC-like domain described herein, e.g., SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, or SEQ ID NO: 16). Exemplary inactive, or cleavage incompetent HNH-like domains can have a mutation at one or more of: a histidine in an HNH-like domain, e.g., a histidine shown at position 856 of the consensus sequence disclosed in **Figs. 2A-2G****,** e.g., can be substituted with an alanine; and one or more asparagines in an HNH-like domain, e.g., an asparagine shown at position 870 of the consensus sequence disclosed in **Figs. 2A-2G** and/or at position 879 of the consensus sequence disclosed in **Figs. 2A-2G****,** e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule *of S. pyogenes,* or *S. thermophilus.* In an embodiment, the reference Cas9 molecule is the naturally occurring Cas9 molecule having the closest sequence identity or homology.

### Alterations in the Ability to Cleave One or Both Strands of a Target Nucleic Acid

In an embodiment, exemplary Cas9 activities comprise one or more of PAM specificity, cleavage activity, and helicase activity. A mutation(s) can be present, e.g., in one or more RuvC-like domain, e.g., an N-terminal RuvC-like domain; an HNH-like domain; a region outside the RuvC-like domains and the HNH-like domain. In some embodiments, a mutation(s) is present in a RuvC-like domain, e.g., an N-terminal RuvC-like domain. In some embodiments, a mutation(s) is present in an HNH-like domain. In some embodiments, mutations are present in both a RuvC-like domain, e.g., an N-terminal RuvC-like domain and an HNH-like domain.

Exemplary mutations that may be made in the RuvC domain or HNH domain with reference to the *S. pyogenes* sequence include: D10A, E762A, H840A, N854A, N863A and/or D986A.

In an embodiment, a Cas9 molecule or Cas9 polypeptide is an eiCas9 molecule or eiCas9 polypeptide comprising one or more differences in a RuvC domain and/or in an HNH domain as compared to a reference Cas9 molecule, and the eiCas9 molecule or eiCas9 polypeptide does not cleave a nucleic acid, or cleaves with significantly less efficiency than does wildype, e.g., when compared with wild type in a cleavage assay, e.g., as described herein, cuts with less than 50, 25, 10, or 1% of a reference Cas9 molecule, as measured by an assay described herein.

Whether or not a particular sequence, e.g., a substitution, may affect one or more activity, such as targeting activity, cleavage activity, etc., can be evaluated or predicted, e.g., by evaluating whether the mutation is conservative or by the method described in Section IV. In an embodiment, a "non-essential" amino acid residue, as used in the context of a Cas9 molecule, is a residue that can be altered from the wild-type sequence of a Cas9 molecule, e.g., a naturally occurring Cas9 molecule, e.g., an eaCas9 molecule, without abolishing or more preferably, without substantially altering a Cas9 activity (e.g., cleavage activity), whereas changing an "essential" amino acid residue results in a substantial loss of activity (e.g., cleavage activity).

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S aureus, S. pyogenes,* or C. *jejuni* as follows: its ability to modulate, e.g., decreased or increased, cleavage of a double stranded break (endonuclease and/or exonuclease activity), e.g., as compared to a naturally occurring Cas9 molecule (e.g., a Cas9 molecule of *S aureus, S. pyogenes,* or *C. jejuni)*; its ability to modulate, e.g., decreased or increased, cleavage of a single strand of a nucleic acid, e.g., a non-complimentary strand of a nucleic acid molecule or a complementary strand of a nucleic acid molecule (nickase activity), e.g., as compared to a naturally occurring Cas9 molecule (e.g., a Cas9 molecule of *S aureus, S. pyogenes,* or *C. jejuni*); or the ability to cleave a nucleic acid molecule, e.g., a double stranded or single stranded nucleic acid molecule, can be eliminated.

In an embodiment, the altered Cas9 molecule or Cas9 polypeptide is an eaCas9 molecule or eaCas9 polypeptide comprising one or more of the following activities: cleavage activity associated with a RuvC domain; cleavage activity associated with an HNH domain; cleavage activity associated with an HNH domain and cleavage activity associated with a RuvC domain.

In an embodiment, the altered Cas9 molecule or Cas9 polypeptide is an eiCas9 molecule or eiCas9 polypeptide which does not cleave a nucleic acid molecule (either double stranded or single stranded nucleic acid molecules) or cleaves a nucleic acid molecule with significantly less efficiency, e.g., less than 20, 10, 5, 1 or 0.1% of the cleavage activity of a reference Cas9 molecule, e.g., as measured by an assay described herein. The reference Cas9 molecule can be a naturally occurring unmodified Cas9 molecule, e.g., a naturally occurring Cas9 molecule such as a Cas9 molecule of *S. pyogenes, S. thermophilus, S. aureus, C. jejuni* or *N. meningitidis.* In an embodiment, the reference Cas9 molecule is the naturally occurring Cas9 molecule having the closest sequence identity or homology. In an embodiment, the eiCas9 molecule or eiCas9 polypeptide lacks substantial cleavage activity associated with a RuvC domain and cleavage activity associated with an HNH domain.

In an embodiment, the altered Cas9 molecule or Cas9 polypeptide is an eaCas9 molecule or eaCas9 polypeptide comprising the fixed amino acid residues *of S. pyogenes* shown in the consensus sequence disclosed in **Figs. 2A-2G****,** and has one or more amino acids that differ from the amino acid sequence of *S*. *pyogenes* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G** or SEQ ID NO:7.

In an embodiment, the altered Cas9 molecule or Cas9 polypeptide comprises a sequence in which:
the sequence corresponding to the fixed sequence of the consensus sequence disclosed in **Figs. 2A-2G****differs** at no more than 1, 2, 3, 4, 5, 10, 15, or 20% of the fixed residues in the consensus sequence disclosed in **Figs. 2A-2G****;**
the sequence corresponding to the residues identified by "^{∗}" in the consensus sequence disclosed in **Figs. 2A-2G** differ at no more than 1, 2, 3, 4, 5, 10, 15, 20, 25, 30, 35, or 40% of the "^{∗}" residues from the corresponding sequence of naturally occurring Cas9 molecule, e.g., an *S. pyogenes* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. pyogenes* Cas9 molecule.

In an embodiment, the altered Cas9 molecule or Cas9 polypeptide is an eaCas9 molecule or eaCas9 polypeptide comprising the fixed amino acid residues of *S. thermophilus* shown in the consensus sequence disclosed in **Figs. 2A-2G****,** and has one or more amino acids that differ from the amino acid sequence of *S. thermophilus* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "^{∗}" in the consensus sequence disclosed in **Figs. 2A-2G** differ at no more than 1, 2, 3, 4, 5, 10, 15, 20, 25, 30, 35, or 40% of the "^{∗}" residues from the corresponding sequence of naturally occurring Cas9 molecule, e.g., an *S. thermophilus* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. thermophilus* Cas9 molecule.

In an embodiment, the altered Cas9 molecule or Cas9 polypeptide is an eaCas9 molecule or eaCas9 polypeptide comprising the fixed amino acid residues of *S. mutans* shown in the consensus sequence disclosed in **Figs. 2A-2G****,** and has one or more amino acids that differ from the amino acid sequence of *S. mutans* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "^{∗}" in the consensus sequence disclosed in **Figs. 2A-2G** differ at no more than 1, 2, 3, 4, 5, 10, 15, 20, 25, 30, 35, or 40% of the "^{∗}" residues from the corresponding sequence of naturally occurring Cas9 molecule, e.g., an *S. mutans* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. mutans* Cas9 molecule.

In an embodiment, the altered Cas9 molecule or Cas9 polypeptide is an eaCas9 molecule or eaCas9 polypeptide comprising the fixed amino acid residues of *L. innocula* shown in the consensus sequence disclosed in **Figs. 2A-2G****,** and has one or more amino acids that differ from the amino acid sequence of *L. innocula* (e.g., has a substitution) at one or more residue (e.g., 2, 3, 5, 10, 15, 20, 30, 50, 70, 80, 90, 100, 200 amino acid residues) represented by an "-"in the consensus sequence disclosed in **Figs. 2A-2G****.**

In an embodiment, the altered Cas9 molecule or Cas9 polpeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "^{∗}" in the consensus sequence disclosed in **Figs. 2A-2G** differ at no more than 1, 2, 3, 4, 5, 10, 15, 20, 25, 30, 35, or 40% of the "^{∗}" residues from the corresponding sequence of naturally occurring Cas9 molecule, e.g., an *L. innocula* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *L. innocula* Cas9 molecule.

In an embodiment, the altered Cas9 molecule or Cas9 polypeptide, e.g., an eaCas9 molecule or eaCas9 polypeptide, can be a fusion, e.g., of two of more different Cas9 molecules, e.g., of two or more naturally occurring Cas9 molecules of different species. For example, a fragment of a naturally occurring Cas9 molecule of one species can be fused to a fragment of a Cas9 molecule of a second species. As an example, a fragment of a Cas9 molecule *of S. pyogenes* comprising an N-terminal RuvC-like domain can be fused to a fragment of a Cas9 molecule of a species other than *S. pyogenes* (e.g., *S. thermophilus*) comprising an HNH-like domain.

### Cas9 Molecules and Cas9 Polypeptides with Altered PAM Recognition or No PAM Recognition

Naturally occurring Cas9 molecules can recognize specific PAM sequences, for example, the PAM recognition sequences described above for *S. pyogenes, S. thermophiles, S. mutans, S. aureus* and *N. meningitidis.*

In an embodiment, a Cas9 molecule or Cas9 polypeptide has the same PAM specificities as a naturally occurring Cas9 molecule. In another embodiment, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule or Cas9 polypeptide can be altered, e.g., to alter PAM recognition, e.g., to alter the PAM sequence that the Cas9 molecule recognizes to decrease off target sites and/or improve specificity; or eliminate a PAM recognition requirement. In an embodiment, a Cas9 molecule or Cas9 polypeptide can be altered, e.g., to increase length of PAM recognition sequence and/or improve Cas9 specificity to high level of identity (e.g., 98%, 99% or 100% match between gRNA and a PAM sequence), e.g., to decrease off target sites and increase specificity. In an embodiment, the length of the PAM recognition sequence is at least 4, 5, 6, 7, 8, 9, 10 or 15 amino acids in length. In an embodiment, the Cas9 specificity requires at least 90%, 95%, 96%, 97%, 98%, 99% or more homology between the gRNA and the PAM sequence. Cas9 molecules or Cas9 polypeptides that recognize different PAM sequences and/or have reduced off-target activity can be generated using directed evolution. Exemplary methods and systems that can be used for directed evolution of Cas9 molecules are described, e.g., in Esvelt et al. NATURE 2011, 472(7344): 499-503. Candidate Cas9 molecules can be evaluated, e.g., by methods described in Section IV.

Alterations of the PI domain, which mediates PAM recognition, are discussed below.

### Synthetic Cas9 Molecules and Cas9 Polypeptides with Altered PI Domains

Current genome-editing methods are limited in the diversity of target sequences that can be targeted by the PAM sequence that is recognized by the Cas9 molecule utilized. A synthetic Cas9 molecule (or Syn-Cas9 molecule), or synthetic Cas9 polypeptide (or Syn-Cas9 polypeptide), as that term is used herein, refers to a Cas9 molecule or Cas9 polypeptide that comprises a Cas9 core domain from one bacterial species and a functional altered PI domain, i.e., a PI domain other than that naturally associated with the Cas9 core domain, e.g., from a different bacterial species.

In an embodiment, the altered PI domain recognizes a PAM sequence that is different from the PAM sequence recognized by the naturally-occurring Cas9 from which the Cas9 core domain is derived. In an embodiment, the altered PI domain recognizes the same PAM sequence recognized by the naturally-occurring Cas9 from which the Cas9 core domain is derived, but with different affinity or specificity. A Syn-Cas9 molecule or Syn-Cas9 polypetide can be, respectively, a Syn-eaCas9 molecule or Syn-eaCas9 polypeptide or a Syn-eiCas9 molecule Syn-eiCas9 polypeptide.

An exemplary Syn-Cas9 molecule or Syn-Cas9 polypetide comprises:
a) a Cas9 core domain, e.g., a Cas9 core domain from **Table 17** or **18,** e.g., a *S*. *aureus, S. pyogenes*, or *C. jejuni* Cas9 core domain; and
b) an altered PI domain from a species X Cas9 sequence selected from **Tables 20** and **21.**

In an embodiment, the RKR motif (the PAM binding motif) of said altered PI domain comprises: differences at 1, 2, or 3 amino acid residues; a difference in amino acid sequence at the first, second, or third position; differences in amino acid sequence at the first and second positions, the first and third positions, or the second and third positions; as compared with the sequence of the RKR motif of the native or endogenous PI domain associated with the Cas9 core domain.

In an embodiment, the Cas9 core domain comprises the Cas9 core domain from a species X Cas9 from **Table 17** and said altered PI domain comprises a PI domain from a species Y Cas9 from **Table 17.**

In an embodiment, the RKR motif of the species X Cas9 is other than the RKR motif of the species Y Cas9.

In an embodiment, the RKR motif of the altered PI domain is selected from XXY, XNG, and XNQ.

In an embodiment, the altered PI domain has at least 60, 70, 80, 90, 95, or 100% homology with the amino acid sequence of a naturally occurring PI domain of said species Y from **Table 17.**

In an embodiment, the altered PI domain differs by no more than 50, 40, 30, 25, 20, 15, 10, 5, 4, 3, 2, or 1 amino acid residue from the amino acid sequence of a naturally occurring PI domain of said second species from **Table 17**.

In an embodiment, the Cas9 core domain comprises a *S*. *aureus* core domain and altered PI domain comprises: an *A. denitrificans* PI domain; a *C. jejuni* PI domain; a *H. mustelae* PI domain; or an altered PI domain of species X PI domain, wherein species X is selected from **Table 21.**

In an embodiment, the Cas9 core domain comprises a *S. pyogenes* core domain and the altered PI domain comprises: an *A. denitrificans* PI domain; a *C. jejuni* PI domain; a *H. mustelae* PI domain; or an altered PI domain of species X PI domain, wherein species X is selected from **Table 21.**

In an embodiment, the Cas9 core domain comprises a *C. jejuni* core domain and the altered PI domain comprises: an *A. denitrificans* PI domain; a *H. mustelae* PI domain; or an altered PI domain of species X PI domain, wherein species X is selected from **Table 21.**

In an embodiment, the Cas9 molecule or Cas9 polypeptide further comprises a linker disposed between said Cas9 core domain and said altered PI domain.

In an embodiment, the linker comprises: a linker described elsewhere herein disposed between the Cas9 core domain and the heterologous PI domain. Suitable linkers are further described in Section V.

Exemplary altered PI domains for use in Syn-Cas9 molecules are described in **Tables 20** and **21.** The sequences for the 83 Cas9 orthologs referenced in **Tables 20** and **21** are provided in **Table 17. Table 19** provides the Cas9 orthologs with known PAM sequences and the corresponding RKR motif.

In an embodiment, a Syn-Cas9 molecule or Syn-Cas9 polypeptide may also be size-optimized, e.g., the Syn-Cas9 molecule or Syn-Cas9 polypeptide comprises one or more deletions, and optionally one or more linkers disposed between the amino acid residues flanking the deletions. In an embodiment, a Syn-Cas9 molecule or Syn-Cas9 polypeptide comprises a REC deletion.

### Size-Optimized Cas9 Molecules and Cas9 Polypeptides

Engineered Cas9 molecules and engineered Cas9 polypeptides described herein include a Cas9 molecule or Cas9 polypeptide comprising a deletion that reduces the size of the molecule while still retaining desired Cas9 properties, e.g., essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, e.g., a *S*. *aureus, S. pyogenes,* or *C. jejuni,* Cas9 molecule, having a deletion is smaller, e.g., has reduced number of amino acids, than the corresponding naturally-occurring Cas9 molecule. The smaller size of the Cas9 molecules allows increased flexibility for delivery methods, and thereby increases utility for genome-editing. A Cas9 molecule or Cas9 polypeptide can comprise one or more deletions that do not substantially affect or decrease the activity of the resultant Cas9 molecules or Cas9 polypeptides described herein. Activities that are retained in the Cas9 molecules or Cas9 polypeptides comprising a deletion as described herein include one or more of the following:
a nickase activity, i.e., the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities;
an endonuclease activity;
an exonuclease activity;
a helicase activity, i.e., the ability to unwind the helical structure of a double stranded nucleic acid;
and recognition activity of a nucleic acid molecule, e.g., a target nucleic acid or a gRNA.

Activity of the Cas9 molecules or Cas9 polypeptides described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species, e.g., any one of those listed in **Table 17,** can be modeled onto the crystal structure of *S*. *pyogenes* Cas9 (Nishimasu et al., Cell, 156:935-949, 2014) to examine the level of conservation across the selected Cas9 orthologs with respect to the three-dimensional conformation of the protein. Less conserved or unconserved regions that are spatially located distant from regions involved in Cas9 activity, e.g., interface with the target nucleic acid molecule and/or gRNA, represent regions or domains are candidates for deletion without substantially affecting or decreasing Cas9 activity.

### REC-Optimized Cas9 Molecules and Cas9 Polypeptides

A REC-optimized Cas9 molecule, or a REC-optimized Cas9 polypeptide, as that term is used herein, refers to a Cas9 molecule or Cas9 polypeptide that comprises a deletion in one or both of the REC2 domain and the RE1_{CT} domain (collectively a REC deletion), wherein the deletion comprises at least 10% of the amino acid residues in the cognate domain. A REC-optimized Cas9 molecule or Cas9 polypeptide can be an eaCas9 molecule or eaCas9 polypetide, or an eiCas9 molecule or eiCas9 polypeptide. An exemplary REC-optimized Cas9 molecule or REC-optimized Cas9 polypeptide comprises:
a) a deletion selected from:
   i) a REC2 deletion;
   ii) a REC1_{CT} deletion; or
   iii) a REC1_{SUB} deletion.

Optionally, a linker is disposed between the amino acid residues that flank the deletion. In an embodiment, a Cas9 molecule or Cas9 polypeptide includes only one deletion, or only two deletions. A Cas9 molecule or Cas9 polypeptide can comprise a REC2 deletion and a REC1_{CT} deletion. A Cas9 molecule or Cas9 polypeptide can comprise a REC2 deletion and a REC1_{SUB} deletion.

Generally, the deletion will contain at least 10% of the amino acids in the cognate domain, e.g., a REC2 deletion will include at least 10% of the amino acids in the REC2 domain. A deletion can comprise: at least 10, 20, 30, 40, 50, 60, 70, 80, or 90% of the amino acid residues of its cognate domain; all of the amino acid residues of its cognate domain; an amino acid residue outside its cognate domain; a plurality of amino acid residues outside its cognate domain; the amino acid residue immediately N terminal to its cognate domain; the amino acid residue immediately C terminal to its cognate domain; the amino acid residue immediately N terminal to its cognate and the amino acid residue immediately C terminal to its cognate domain; a plurality of, e.g., up to 5, 10, 15, or 20, amino acid residues N terminal to its cognate domain; a plurality of, e.g., up to 5, 10, 15, or 20, amino acid residues C terminal to its cognate domain; a plurality of, e.g., up to 5, 10, 15, or 20, amino acid residues N terminal to to its cognate domain and a plurality of e.g., up to 5, 10, 15, or 20, amino acid residues C terminal to its cognate domain.

In an embodiment, a deletion does not extend beyond: its cognate domain; the N terminal amino acid residue of its cognate domain; the C terminal amino acid residue of its cognate domain.

A REC-optimized Cas9 molecule or REC-optimized Cas9 polypeptide can include a linker disposed between the amino acid residues that flank the deletion. Any linkers known in the art that maintain the conformation or native fold of the Cas9 molecule (thereby retaining Cas9 activity) can be used between the amino acid resides that flank a REC deletion in a REC-optimized Cas9 molecule or REC-optimized Cas9 polypeptide. Linkers for use in generating recombinant proteins, e.g., multi-domain proteins, are known in the art (Chen et al., Adv Drug Delivery Rev, 65:1357-69, 2013).

In an embodiment, a REC-optimized Cas9 molecule or REC-optimized Cas9 polypeptide comprises an amino acid sequence that, other than any REC deletion and associated linker, has at least 50, 55, 60, 65, 70, 75, 80, 85, 90, 95, 99, or 100% homology with the amino acid sequence of a naturally occurring Cas 9, e.g., a Cas9 molecule described in **Table 17,** e.g., a *S*. *aureus* Cas9 molecule, a *S. pyogenes* Cas9 molecule, or a *C. jejuni* Cas9 molecule.

In an embodiment, a a REC-optimized Cas9 molecule or REC-optimized Cas9 polypeptide comprises an amino acid sequence that, other than any REC deletion and associated linker, differs by no more than 1, 2, 3, 4, 5, 6, 7, 8, 9, 10, 15, 20, or 25, amino acid residues from the amino acid sequence of a naturally occurring Cas 9, e.g., a Cas9 molecule described in **Table 17,** e.g., a *S*. *aureus* Cas9 molecule, a *S. pyogenes* Cas9 molecule, or a C. *jejuni* Cas9 molecule.

In an embodiment, a REC-optimized Cas9 molecule or REC-optimized Cas9 polypeptide comprises an amino acid sequence that, other than any REC deletion and associate linker, differs by no more than 1, 2, 3, 4, 5, 6, 7, 8, 9, 10, 15, 20, or 25% of the, amino acid residues from the amino acid sequence of a naturally occurring Cas 9, e.g., a Cas9 molecule described in **Table 17,** e.g., a *S*. *aureus* Cas9 molecule, a *S. pyogenes* Cas9 molecule, or a C. *jejuni* Cas9 molecule.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'1. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Sequence information for exemplary REC deletions are provided for 83 naturally-occurring Cas9 orthologs in **Table 17.**

The amino acid sequences of exemplary Cas9 molecules from different bacterial species are shown below.

**Table 17. Amino Acid Sequence of Cas9 Orthologs**

| | | REC2 | | | REC1_{CT} | | | Rec_{sub} | | |
|---|---|---|---|---|---|---|---|---|---|---|
| **Species** / **Composite ID** | **Amino acid sequence** | start (AA pos) | stop (AA pos) | # AA delete d (n) | start (AA pos) | stop (AA pos) | # AA delete d (n) | start (AA pos) | stop (AA pos) | # AA delete d (n) |
| Staphylococcus Aureus tr\|J7RUA5\|J7RUA5_STAAU | SEQ ID NO: 304 | 126 | 166 | 41 | 296 | 352 | 57 | 296 | 352 | 57 |
| Streptococcus Pyogenes sp\|Q99ZW2\|CAS9_STRP1 | SEQ ID NO: 305 | 176 | 314 | 139 | 511 | 592 | 82 | 511 | 592 | 82 |
| Campylobacter jejuni NCTC 11168 gi\|218563121\|ref]YP_00234490 0.1 | SEQ ID NO: 306 | 137 | 181 | 45 | 316 | 360 | 45 | 316 | 360 | 45 |
| Bacteroides fragilis NCTC 9343 gi\|60683389\|ref\|YP_213533.1\| | SEQ ID NO: 307 | 148 | 339 | 192 | 524 | 617 | 84 | 524 | 617 | 84 |
| Bifidobacterium bifidum S17 gi\|310286728\|ref\|YP_00393798 6. | SEQ ID NO: 308 | 173 | 335 | 163 | 516 | 607 | 87 | 516 | 607 | 87 |
| Veillonella atypica ACS-134-V-Col7a gi\|303229466\|ref\|ZP_07316256. 1 | SEQ ID NO: 309 | 185 | 339 | 155 | 574 | 663 | 79 | 574 | 663 | 79 |
| Lactobacillus rhamnosus GG gi\|258509199\|ref\|YP_00317195 0.1 | SEQ ID NO: 310 | 169 | 320 | 152 | 559 | 645 | 78 | 559 | 645 | 78 |
| Filifactor alocis ATCC 35896 gi\|374307738\|ref\|YP_00505416 9.1 | SEQ ID NO: 311 | 166 | 314 | 149 | 508 | 592 | 76 | 508 | 592 | 76 |
| Oenococcus kitaharae DSM 17330 gi\|366983953\|gb\|EHN59352.1\| | SEQ ID NO: 312 | 169 | 317 | 149 | 555 | 639 | 80 | 555 | 639 | 80 |
| Fructobacillus fructosus KCTC 3544 gi\|339625081\|ref\|ZP _08660870. 1 | SEQ ID NO: 313 | 168 | 314 | 147 | 488 | 571 | 76 | 488 | 571 | 76 |
| Catenibacterium mitsuokai DSM 15897 gi\|224543312\|ref\|ZP_03683851. 1 | SEQ ID NO: 314 | 173 | 318 | 146 | 511 | 594 | 78 | 511 | 594 | 78 |
| Finegoldia magna ATCC 29328 gi\|169823755\|ref\|YP_00169136 6.1 | SEQ ID NO: 315 | 168 | 313 | 146 | 452 | 534 | 77 | 452 | 534 | 77 |
| CoriobacteriumglomeransPW2 gi\|328956315\|ref\|YP_00437364 8.1 | SEQ ID NO: 316 | 175 | 318 | 144 | 511 | 592 | 82 | 511 | 592 | 82 |
| Eubacterium yurii ATCC 43715 gi\|306821691\|ref\|ZP_07455288. 1 | SEQ ID NO: 317 | 169 | 310 | 142 | 552 | 633 | 76 | 552 | 633 | 76 |
| Peptoniphilus duerdenii ATCC BAA-1640 gi\|304438954\|ref\|ZP _07398877. 1 | SEQ ID NO: 318 | 171 | 311 | 141 | 535 | 615 | 76 | 535 | 615 | 76 |
| Acidaminococcus sp. D21 gi\|227824983\|ref\|ZP _03989815. 1 | SEQ ID NO: 319 | 167 | 306 | 140 | 511 | 591 | 75 | 511 | 591 | 75 |
| Lactobacillus farciminis KCTC 3681 gi\|336394882\|ref\|ZP_08576281. 1 | SEQ ID NO: 320 | 171 | 310 | 140 | 542 | 621 | 85 | 542 | 621 | 85 |
| Streptococcus sanguinis SK49 gi\|422884106\|ref\|ZP_16930555. 1 | SEQ ID NO: 321 | 185 | 324 | 140 | 411 | 490 | 85 | 411 | 490 | 85 |
| Coprococcus catus GD-7 gi\|291520705\|emb\|CBK78998.1 \| | SEQ ID NO: 322 | 172 | 310 | 139 | 556 | 634 | 76 | 556 | 634 | 76 |
| Streptococcus mutans UA159 gi\|24379809\|ref\|NP_721764.1\| | SEQ ID NO: 323 | 176 | 314 | 139 | 392 | 470 | 84 | 392 | 470 | 84 |
| Streptococcus pyogenes M1 GAS gi\|13622193\|gb\|AAK33936.1\| | SEQ ID NO: 324 | 176 | 314 | 139 | 523 | 600 | 82 | 523 | 600 | 82 |
| Streptococcus thermophilus LMD-9 gi\|116628213\|ref\|YP_820832.1\| | SEQ ID NO: 325 | 176 | 314 | 139 | 481 | 558 | 81 | 481 | 558 | 81 |
| Fusobacteriumnucleatum ATCC49256 gi\|34762592\|ref\|ZP_00143587.1 \| | SEQ ID NO: 326 | 171 | 308 | 138 | 537 | 614 | 76 | 537 | 614 | 76 |
| Planococcus antarcticus DSM 14505 gi\|389815359\|ref\|ZP_10206685. 1 | SEQ ID NO: 327 | 162 | 299 | 138 | 538 | 614 | 94 | 538 | 614 | 94 |
| Treponema denticola ATCC 35405 gi\|42525843\|ref\|NP_970941.1\| | SEQ ID NO: 328 | 169 | 305 | 137 | 524 | 600 | 81 | 524 | 600 | 81 |
| Solobacterium moorei F0204 gi\|320528778\|ref\|ZP_08029929. 1 | SEQ ID NO: 329 | 179 | 314 | 136 | 544 | 619 | 77 | 544 | 619 | 77 |
| Staphylococcus pseudintermedius ED99 gi\|323463801\|gb\|ADX75954.1\| | SEQ ID NO: 330 | 164 | 299 | 136 | 531 | 606 | 92 | 531 | 606 | 92 |
| Flavobacterium branchiophilum FL-15 gi\|347536497\|ref\|YP_00484392 2.1 | SEQ ID NO: 331 | 162 | 286 | 125 | 538 | 613 | 63 | 538 | 613 | 63 |
| Ignavibacterium album JCM | SEQ ID NO: 332 | 223 | 329 | 107 | 357 | 432 | 90 | 357 | 432 | 90 |
| 16511 gi\|385811609\|ref\|YP_00584800 5.1 | | | | | | | | | | |
| Bergeyella zoohelcum ATCC 43767 gi\|423317190\|relZP _17295095. 1 | SEQ ID NO: 333 | 165 | 261 | 97 | 529 | 604 | 56 | 529 | 604 | 56 |
| Nitrobacter hamburgensis X14 gi\|92109262\|ref\|YP_571550.1\| | SEQ ID NO: 334 | 169 | 253 | 85 | 536 | 611 | 48 | 536 | 611 | 48 |
| Odoribacter laneus YIT 12061 gi\|374384763\|ref\|ZP_09642280. 1 | SEQ ID NO: 335 | 164 | 242 | 79 | 535 | 610 | 63 | 535 | 610 | 63 |
| Legionella pneumophila str. Paris gi\|54296138\|ref\|YP_122507.1\| | SEQ ID NO: 336 | 164 | 239 | 76 | 402 | 476 | 67 | 402 | 476 | 67 |
| Bacteroides sp. 20 3 gi\|301311869\|ref\|ZP_07217791. 1 | SEQ ID NO: 337 | 198 | 269 | 72 | 530 | 604 | 83 | 530 | 604 | 83 |
| Akkermansia muciniphila ATCC BAA-835 gi\|187736489\|ref\|YP_00187860 1. | SEQ ID NO: 338 | 136 | 202 | 67 | 348 | 418 | 62 | 348 | 418 | 62 |
| Prevotella sp. C561 gi\|345885718\|ref\|ZP_08837074. 1 | SEQ ID NO: 339 | 184 | 250 | 67 | 357 | 425 | 78 | 357 | 425 | 78 |
| Wolinella succinogenes DSM 1740 gi\|34557932\|ref\|NP_907747.1\| | SEQ ID NO: 340 | 157 | 218 | 36 | 401 | 468 | 60 | 401 | 468 | 60 |
| Alicyclobacillus hesperidum URH17-3-68 gi\|403744858\|ref\|ZP_10953934. 1 | SEQ ID NO: 341 | 142 | 196 | 55 | 416 | 482 | 61 | 416 | 482 | 61 |
| Caenispirillum salinarum AK4 gi\|427429481\|ref\|ZP_18919511. 1 | SEQ ID NO: 342 | 161 | 214 | 54 | 330 | 393 | 68 | 330 | 393 | 68 |
| Eubacterium rectale ATCC 33656 gi\|238924075\|ref\|YP_00293759 1.1 | SEQ ID NO: 343 | 133 | 185 | 53 | 322 | 384 | 60 | 322 | 384 | 60 |
| Mycoplasma synoviae 53 gi\|71894592\|ref\|YP_278700.1\| | SEQ ID NO: 344 | 187 | 239 | 53 | 319 | 381 | 80 | 319 | 381 | 80 |
| Porphyromonas sp. oral taxon 279 str. F0450 gi\|402847315\|relZP_10895610. 1 | SEQ ID NO: 345 | 150 | 202 | 53 | 309 | 371 | 60 | 309 | 371 | 60 |
| Streptococcus thermophilus LMD-9 gi\|116627542\|re1YP_820161.1\| | SEQ ID NO: 346 | 127 | 178 | 139 | 424 | 486 | 81 | 424 | 486 | 81 |
| Roseburia inulinivorans DSM 16841 gi\|225377804\|ref\|ZP_03755025. 1 | SEQ ID NO: 347 | 154 | 204 | 51 | 318 | 380 | 69 | 318 | 380 | 69 |
| Methylosinus trichosporium OB3b gi\|296446027\|relZP_06887976. 1 | SEQ ID NO: 348 | 144 | 193 | 50 | 426 | 488 | 64 | 426 | 488 | 64 |
| Ruminococcus albus 8 gi\|325677756\|ref\|ZP_08157403.1 | SEQ ID NO: 349 | 139 | 187 | 49 | 351 | 412 | 55 | 351 | 412 | 55 |
| Bifidobacterium longum DJO10A gi\|189440764\|ref\|YP_00195584 5. | SEQ ID NO: 350 | 183 | 230 | 48 | 370 | 431 | 44 | 370 | 431 | 44 |
| Enterococcus faecalis TX0012 gi\|315149830\|gb\|EFT93846.1\| | SEQ ID NO: 351 | 123 | 170 | 48 | 327 | 387 | 60 | 327 | 387 | 60 |
| Mycoplasma mobile 163K gi\|47458868\|ref\|YP_015730.11 | SEQ ID NO: 352 | 179 | 226 | 48 | 314 | 374 | 79 | 314 | 374 | 79 |
| Actinomyces coleocanis DSM 15436 gi\|227494853\|ref\|ZP_03925169. 1 | SEQ ID NO: 353 | 147 | 193 | 47 | 358 | 418 | 40 | 358 | 418 | 40 |
| Dinoroseobacter shibae DFL 12 gi\|159042956\|ref\|YP_00153175 0.1 | SEQ ID NO: 354 | 138 | 184 | 47 | 338 | 398 | 48 | 338 | 398 | 48 |
| Actinomyces sp. oral taxon 180 str. F031 0 gi\|315605738\|ref\|ZP _07880770. 1 | SEQ ID NO: 355 | 183 | 228 | 46 | 349 | 409 | 40 | 349 | 409 | 40 |
| Alcanivorax sp. W11-5 gi\|407803669\|ref\|ZP_11150502. 1 | SEQ ID NO: 356 | 139 | 183 | 45 | 344 | 404 | 61 | 344 | 404 | 61 |
| Aminomonas paucivorans DSM 12260 gi\|312879015\|ref\|ZP_07738815. 1 | SEQ ID NO: 357 | 134 | 178 | 45 | 341 | 401 | 63 | 341 | 401 | 63 |
| Mycoplasma canis PG 14 gi\|384393286\|gb\|EIE39736.1\| | SEQ ID NO: 358 | 139 | 183 | 45 | 319 | 379 | 76 | 319 | 379 | 76 |
| Lactobacillus coryniformis KCTC 3535 gi\|336393381\|ref\|ZP_08574780. 1 | SEQ ID NO: 359 | 141 | 184 | 44 | 328 | 387 | 61 | 328 | 387 | 61 |
| Elusimicrobium minutum Pei191 gi\|187250660\|ref\|YP_00187514 2.1 | SEQ ID NO: 360 | 177 | 219 | 43 | 322 | 381 | 47 | 322 | 381 | 47 |
| Neisseria meningitidis Z2491 gi\|218767588\|ref\|YP _00234210 0.1 | SEQ ID NO: 361 | 147 | 189 | 43 | 360 | 419 | 61 | 360 | 419 | 61 |
| Pasteurella multocida str. Pm70 gi\|15602992\|reflNP_246064.11 | SEQ ID NO: 362 | 139 | 181 | 43 | 319 | 378 | 61 | 319 | 378 | 61 |
| Rhodovulum sp. PH10 gi\|402849997\|ref\|ZP_10898214. 1 | SEQ ID NO: 363 | 141 | 183 | 43 | 319 | 378 | 48 | 319 | 378 | 48 |
| Eubacterium dolichum DSM 3991 gi\|160915782\|ref\|ZP_02077990. 1 | SEQ ID NO: 364 | 131 | 172 | 42 | 303 | 361 | 59 | 303 | 361 | 59 |
| Nitratifractor salsuginis DSM 16511 gi\|319957206\|ref\|YP_00416846 9.1 | SEQ ID NO: 365 | 143 | 184 | 42 | 347 | 404 | 61 | 347 | 404 | 61 |
| Rhodospirillum rubrum ATCC 11170 gi\|83591793\|ref\|YP_425545.11 | SEQ ID NO: 366 | 139 | 180 | 42 | 314 | 371 | 55 | 314 | 371 | 55 |
| Clostridium cellulolyticum H10 gi\|220930482\|reflYP_00250739 1.1 | SEQ ID NO: 367 | 137 | 176 | 40 | 320 | 376 | 61 | 320 | 376 | 61 |
| Helicobacter mustelae 12198 gi\|291276265\|ref\|YP_00351603 7.1 | SEQ ID NO: 368 | 148 | 187 | 40 | 298 | 354 | 48 | 298 | 354 | 48 |
| Ilyobacter polytropus DSM 2926 gi\|310780384\|ref\|YP_00396871 6.1 | SEQ ID NO: 369 | 134 | 173 | 40 | 462 | 517 | 63 | 462 | 517 | 63 |
| Sphaerochaeta globus str. Buddy gi\|325972003\|ref\|YP_00424819 4.1 | SEQ ID NO: 370 | 163 | 202 | 40 | 335 | 389 | 45 | 335 | 389 | 45 |
| Staphylococcus lugdunensis M23590 gi\|315659848\|ref\|ZP_07912707. 1 | SEQ ID NO: 371 | 128 | 167 | 40 | 337 | 391 | 57 | 337 | 391 | 57 |
| Treponema sp. JC4 gi\|384109266\|ref\|ZP_10010146. 1 | SEQ ID NO: 372 | 144 | 183 | 40 | 328 | 382 | 63 | 328 | 382 | 63 |
| uncultured delta proteobacterium HF0070 07E19 gi\|297182908\|gb\|ADI19058.1\| | SEQ ID NO: 373 | 154 | 193 | 40 | 313 | 365 | 55 | 313 | 365 | 55 |
| Alicycliphilus denitrificans K601 gi\|330822845\|ref[YP_00438614 8.1 | SEQ ID NO: 374 | 140 | 178 | 39 | 317 | 366 | 48 | 317 | 366 | 48 |
| Azospirillum sp. B510 gi\|288957741\|ref\|YP_00344808 2.1 | SEQ ID NO: 375 | 205 | 243 | 39 | 342 | 389 | 46 | 342 | 389 | 46 |
| Bradyrhizobium sp. BTAil gi\|148255343\|ref\|YP _00123992 8.1 | SEQ ID NO: 376 | 143 | 181 | 39 | 323 | 370 | 48 | 323 | 370 | 48 |
| Parvibaculum lavamentivorans DS-1 gi\|154250555\|ref\|YP_00141137 9.1 | SEQ ID NO: 377 | 138 | 176 | 39 | 327 | 374 | 58 | 327 | 374 | 58 |
| Prevotella timonensis CRIS 5C-B1 gi\|282880052\|ref\|ZP_06288774. 1 | SEQ ID NO: 378 | 170 | 208 | 39 | 328 | 375 | 61 | 328 | 375 | 61 |
| Bacillus smithii 7 3 47FAA gi\|365156657\|ref\|ZP_09352959. 1 | SEQ ID NO: 379 | 134 | 171 | 38 | 401 | 448 | 63 | 401 | 448 | 63 |
| Cand. Puniceispirillum marinum IMCC1322 gi\|294086111\|reflYP_00355287 1.1 | SEQ ID NO: 380 | 135 | 172 | 38 | 344 | 391 | 53 | 344 | 391 | 53 |
| Bamesiella intestinihominis YIT 11860 gil4044872281reflZP_l 1022414. 1 | SEQ ID NO: 381 | 140 | 176 | 37 | 371 | 417 | 60 | 371 | 417 | 60 |
| Ralstonia syzygii R24 gi\|344171927\|emb\|CCA84553.1 \| | SEQ ID NO: 382 | 140 | 176 | 37 | 395 | 440 | 50 | 395 | 440 | 50 |
| Wolinella succinogenes DSM 1740 gi\|34557790\|ref\|NP_907605.11 | SEQ ID NO: 383 | 145 | 180 | 36 | 348 | 392 | 60 | 348 | 392 | 60 |
| Mycoplasma gallisepticum str. F gi\|284931710\|gb\|ADC31648.11 | SEQ ID NO: 384 | 144 | 177 | 34 | 373 | 416 | 71 | 373 | 416 | 71 |
| Acidothermus cellulolyticus 11B gi\|117929158\|ref\|YP_873709.11 | SEQ ID NO: 385 | 150 | 182 | 33 | 341 | 380 | 58 | 341 | 380 | 58 |
| Mycoplasma ovipneumoniae SC01 gi\|363542550\|ref\|ZP_09312133. 1 | SEQ ID NO: 386 | 156 | 184 | 29 | 381 | 420 | 62 | 381 | 420 | 62 |

**Table 18. Amino Acid Sequence of Cas9 Core Domains**

| **Strain Name** | **Cas9 Start (AA pos)** | **Cas9 Stop (AA pos)** |
|---|---|---|
| | Start and Stop numbers refer to the sequence in **Table 17** | |
| Staphylococcus Aureus | 1 | 772 |
| Streptococcus Pyogenes | 1 | 1099 |
| Campulobacter Jejuni | 1 | 741 |

**Table 19. Identified PAM sequences and corresponding RKR motifs.**

| **Strain Name** | **PAM sequence (NA)** | **RKR motif (AA)** |
|---|---|---|
| Streptococcus pyogenes | NGG | RKR |
| Streptococcus mutans | NGG | RKR |
| Streptococcus thermophilus A | NGGNG | RYR |
| Treponema denticola | NAAAAN | VAK |
| Streptococcus thermophilus B | NNAAAAW | IYK |
| Campylobacter jejuni | NNNNACA | NLK |
| Pasteurella multocida | GNNNCNNA | KDG |
| Neisseria meningitidis | NNNNGATT or | IGK |
| Staphylococcus aureus | NNGRRV (R = A or G; V = A, G or C) NNGRRT (R = A or G) | NDK |

PI domains are provided in **Tables 20** and **21.**

**Table 20. Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 17** | | | |
| Alicycliphilus denitrificans K601 | 837 | 1029 | 193 | --Y |
| Campylobacter jejuni NCTC 11168 | 741 | 984 | 244 | -NG |
| Helicobacter mustelae 12198 | 771 | 1024 | 254 | -NQ |

**Table 21. Other Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 17** | | | |
| Akkermansia muciniphila ATCC BAA-835 | 871 | 1101 | 231 | ALK |
| Ralstonia syzygii R24 | 821 | 1062 | 242 | APY |
| Cand. Puniceispirillum marinum IMCC1322 | 815 | 1035 | 221 | AYK |
| Fructobacillus fructosus KCTC 3544 | 1074 | 1323 | 250 | DGN |
| Eubacterium yurii ATCC 43715 | 1107 | 1391 | 285 | DGY |
| Eubacterium dolichum DSM 3991 | 779 | 1096 | 318 | DKK |
| Dinoroseobacter shibae DFL 12 | 851 | 1079 | 229 | DPI |
| Clostridium cellulolyticum H10 | 767 | 1021 | 255 | EGK |
| Pasteurella multocida str. Pm70 | 815 | 1056 | 242 | ENN |
| Mycoplasma canis PG 14 | 907 | 1233 | 327 | EPK |
| Porphyromonas sp. oral taxon 279 str. F0450 | 935 | 1197 | 263 | EPT |
| Filifactor alocis ATCC 35896 | 1094 | 1365 | 272 | EVD |
| Aminomonas paucivorans DSM 12260 | 801 | 1052 | 252 | EVY |
| Wolinella succinogenes DSM 1740 | 1034 | 1409 | 376 | EYK |
| Oenococcus kitaharae DSM 17330 | 1119 | 1389 | 271 | GAL |
| CoriobacteriumglomeransPW2 | 1126 | 1384 | 259 | GDR |
| Peptoniphilus duerdenii ATCC BAA-1640 | 1091 | 1364 | 274 | GDS |
| Bifidobacterium bifidum S17 | 1138 | 1420 | 283 | GGL |
| Alicyclobacillus hesperidum URH17-3-68 | 876 | 1146 | 271 | GGR |
| Roseburia inulinivorans DSM 16841 | 895 | 1152 | 258 | GGT |
| Actinomyces coleocanis DSM 15436 | 843 | 1105 | 263 | GKK |
| Odoribacter laneus YIT 12061 | 1103 | 1498 | 396 | GKV |
| Coprococcus catus GD-7 | 1063 | 1338 | 276 | GNQ |
| Enterococcus faecalis TX0012 | 829 | 1150 | 322 | GRK |
| Bacillus smithii 7 3 47FAA | 809 | 1088 | 280 | GSK |
| Legionella pneumophila str. Paris | 1021 | 1372 | 352 | GTM |
| Bacteroides fragilis NCTC 9343 | 1140 | 1436 | 297 | IPV |
| Mycoplasma ovipneumoniae SC01 | 923 | 1265 | 343 | IRI |
| Actinomyces sp. oral taxon 180 str. F0310 | 895 | 1181 | 287 | KEK |
| Treponema sp. JC4 | 832 | 1062 | 231 | KIS |
| Fusobacteriumnucleatum ATCC49256 | 1073 | 1374 | 302 | KKV |
| Lactobacillus farciminis KCTC 3681 | 1101 | 1356 | 256 | KKV |
| Nitratifractor salsuginis DSM 16511 | 840 | 1132 | 293 | KMR |
| Lactobacillus coryniformis KCTC 3535 | 850 | 1119 | 270 | KNK |
| Mycoplasma mobile 163K | 916 | 1236 | 321 | KNY |
| Flavobacterium branchiophilum FL-15 | 1182 | 1473 | 292 | KQK |
| Prevotella timonensis CRIS 5C-B1 | 957 | 1218 | 262 | KQQ |
| Methylosinus trichosporium 0B3b | 830 | 1082 | 253 | KRP |
| Prevotella sp. C561 | 1099 | 1424 | 326 | KRY |
| Mycoplasma gallisepticum str. F | 911 | 1269 | 359 | KTA |
| Lactobacillus rhamnosus GG | 1077 | 1363 | 287 | KYG |
| Wolinella succinogenes DSM 1740 | 811 | 1059 | 249 | LPN |
| Streptococcus thermophilus LMD-9 | 1099 | 1388 | 290 | MLA |
| Treponema denticola ATCC 35405 | 1092 | 1395 | 304 | NDS |
| Bergeyella zoohelcum ATCC 43767 | 1098 | 1415 | 318 | NEK |
| Veillonella atypica ACS-134-V-Col7a | 1107 | 1398 | 292 | NGF |
| Neisseria meningitidis Z2491 | 835 | 1082 | 248 | NHN |
| Ignavibacterium album JCM 16511 | 1296 | 1688 | 393 | NKK |
| Ruminococcus albus 8 | 853 | 1156 | 304 | NNF |
| Streptococcus thermophilus LMD-9 | 811 | 1121 | 311 | NNK |
| Bamesiella intestinihominis YIT 11860 | 871 | 1153 | 283 | NPV |
| Azospirillum sp. B510 | 911 | 1168 | 258 | PFH |
| Rhodospirillum rubrum ATCC 11170 | 863 | 1173 | 311 | PRG |
| Planococcus antarcticus DSM 14505 | 1087 | 1333 | 247 | PYY |
| Staphylococcus pseudintermedius ED99 | 1073 | 1334 | 262 | QIV |
| Alcanivorax sp. W11-5 | 843 | 1113 | 271 | RIE |
| Bradyrhizobium sp. BTAil | 811 | 1064 | 254 | RIY |
| Streptococcus pyogenes M1 GAS | 1099 | 1368 | 270 | RKR |
| Streptococcus mutans UA159 | 1078 | 1345 | 268 | RKR |
| Streptococcus Pyogenes | 1099 | 1368 | 270 | RKR |
| Bacteroides sp. 20 3 | 1147 | 1517 | 371 | RNI |
| S. aureus | 772 | 1053 | 282 | RNK |
| Solobacterium moorei F0204 | 1062 | 1327 | 266 | RSG |
| Finegoldia magna ATCC 29328 | 1081 | 1348 | 268 | RTE |
| uncultured delta proteobacterium HF0070 07E19 | 770 | 1011 | 242 | SGG |
| Acidaminococcus sp. D21 | 1064 | 1358 | 295 | SIG |
| Eubacterium rectale ATCC 33656 | 824 | 1114 | 291 | SKK |
| Caenispirillum salinarum AK4 | 1048 | 1442 | 395 | SLV |
| Acidothermus cellulolyticus 11B | 830 | 1138 | 309 | SPS |
| Catenibacterium mitsuokai DSM 15897 | 1068 | 1329 | 262 | SPT |
| Parvibaculum lavamentivorans DS-1 | 827 | 1037 | 211 | TGN |
| Staphylococcus lugdmensis M23590 | 772 | 1054 | 283 | TKK |
| Streptococcus sanguinis SK49 | 1123 | 1421 | 299 | TRM |
| Elusimicrobium minutum Pei191 | 910 | 1195 | 286 | TTG |
| Nitrobacter hamburgensis X14 | 914 | 1166 | 253 | VAY |
| Mycoplasma synoviae 53 | 991 | 1314 | 324 | VGF |
| Sphaerochaeta globus str. Buddy | 877 | 1179 | 303 | VKG |
| Ilyobacter polytropus DSM 2926 | 837 | 1092 | 256 | VNG |
| Rhodovulum sp. PH10 | 821 | 1059 | 239 | VPY |
| Bifidobacterium longum DJO10A | 904 | 1187 | 284 | VRK |

**Amino acid sequences described in Table 17:**
SEQ ID NO: 304 
MKRNYILGLDIGITSVGYGIIDYETRDVIDAGVRLFKEANVENNEGRRSKRGARRLKRRRRH
RIQRVKKLLFDYNLLTDHSELSGINPYEARVKGLSQKLSEEEFSAALLHLAKRRGVHNVNEV
EEDTGNELSTKEQISRNSKALEEKYVAELQLERLKKDGEVRGSINRFKTSDYVKEAKQLLKV
QKAYHQLDQSFIDTYIDLLETRRTYYEGPGEGSPFGWKDIKEWYEMLMGHCTYFPEELRSVK
YAYNADLYNALNDLNNLVITRDENEKLEYYEKFQIIENVFKQKKKPTLKQIAKEILVNEEDI
KGYRVTSTGKPEFTNLKVYHDIKDITARKEIIENAELLDQIAKILTIYQSSEDIQEELTNLN
SELTQEEIEQISNLKGYTGTHNLSLKAINLILDELWHTNDNQIAIFNRLKLVPKKVDLSQQK
EIPTTLVDDFILSPVVKRSFIQSIKVINAIIKKYGLPNDIIIELAREKNSKDAQKMINEMQK
RNRQTNERIEEIIRTTGKENAKYLIEKIKLHDMQEGKCLYSLEAIPLEDLLNNPFNYEVDHI
IPRSVSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSINGGFTS
FLRRKWKFKKERNKGYKHHAEDALIIANADFIFKEWKKLDKAKKVMENQMFEEKQAESMPEI
ETEQEYKEIFITPHQIKHIKDFKDYKYSHRVDKKPNRELINDTLYSTRKDDKGNTLIVNNLN
GLYDKDNDKLKKLINKSPEKLLMYHHDPQTYQKLKLIMEQYGDEKNPLYKYYEETGNYLTKY
SKKDNGPVIKKIKYYGNKLNAHLDITDDYPNSRNKVVKLSLKPYRFDVYLDNGVYKFVTVKN
LDVIKKENYYEVNSKCYEEAKKLKKISNQAEFIASFYNNDLIKINGELYRVIGVNNDLLNRI
EVNMIDITYREYLENMNDKRPPRIIKTIASKTQSIKKYSTDILGNLYEVKSKKHPQIIKKG
SEQ ID NO: 305
SEQ ID NO: 306 
MARILAFDIGISSIGWAFSENDELKDCGVRIFTKVENPKTGESLALPRRLARSARKRLARRK
ARLNHLKHLIANEFKLNYEDYQSFDESLAKAYKGSLISPYELRFRALNELLSKQDFARVILH
 
IAKRRGYDDIKNSDDKEKGAILKAIKQNEEKLANYQSVGEYLYKEYFQKFKENSKEFTNVRN
KKESYERCIAQSFLKDELKLIFKKQREFGFSFSKKFEEEVLSVAFYKRALKDFSHLVGNCSF
FTDEKRAPKNSPLAFMFVALTRIINLLNNLKNTEGILYTKDDLNALLNEVLKNGTLTYKQTK
KLLGLSDDYEFKGEKGTYFIEFKKYKEFIKALGEHNLSQDDLNEIAKDITLIKDEIKLKKAL
AKYDLNQNQIDSLSKLEFKDHLNISFKALKLVTPLMLEGKKYDEACNELNLKVAINEDKKDF
LPAFNETYYKDEVTNPVVLRAIKEYRKVLNALLKKYGKVHKINIELAREVGKNHSQRAKIEK
EQNENYKAKKDAELECEKLGLKINSKNILKLRLFKEQKEFCAYSGEKIKISDLQDEKMLEID
HIYPYSRSFDDSYMNKVLVFTKQNQEKLNQTPFEAFGNDSAKWQKIEVLAKNLPTKKQKRIL
DKNYKDKEQKNFKDRNLNDTRYIARLVLNYTKDYLDFLPLSDDENTKLNDTQKGSKVHVEAK
SGMLTSALRHTWGFSAKDRNNHLHHAIDAVIIAYANNSIVKAFSDFKKEQESNSAELYAKKI
SELDYKNKRKFFEPFSGFRQKVLDKIDEIFVSKPERKKPSGALHEETFRKEEEFYQSYGGKE
GVLKALELGKIRKVNGKIVKNGDMFRVDIFKHKKTNKFYAVPIYTMDFALKVLPNKAVARSK
KGEIKDWILMDENYEFCFSLYKDSLILIQTKDMQEPEFVYYNAFTSSTVSLIVSKHDNKFET
LSKNQKILFKNANEKEVIAKSIGIQNLKVFEKYIVSALGEVTKAEFRQREDFKK
SEQ ID NO: 307 
MKRILGLDLGTNSIGWALVNEAENKDERSSIVKLGVRVNPLTVDELTNFEKGKSITTNADRT
LKRGMRRNLQRYKLRRETLTEVLKEHKLITEDTILSENGNRTTFETYRLRAKAVTEEISLEE
FARVLLMINKKRGYKSSRKAKGVEEGTLIDGMDIARELYNNNLTPGELCLQLLDAGKKFLPD
FYRSDLQNELDRIWEKQKEYYPEILTDVLKEELRGKKRDAVWAICAKYFVWKENYTEWNKEK
GKTEQQEREHKLEGIYSKRKRDEAKRENLQWRVNGLKEKLSLEQLVIVFQEMNTQINNSSGY
LGAISDRSKELYFNKQTVGQYQMEMLDKNPNASLRNMVFYRQDYLDEFNMLWEKQAVYHKEL
TEELKKEIRDIIIFYQRRLKSQKGLIGFCEFESRQIEVDIDGKKKIKTVGNRVISRSSPLFQ
EFKIWQILNNIEVTVVGKKRKRRKLKENYSALFEELNDAEQLELNGSRRLCQEEKELLAQEL
FIRDKMTKSEVLKLLFDNPQELDLNFKTIDGNKTGYALFQAYSKMIEMSGHEPVDFKKPVEK
VVEYIKAVFDLLNWNTDILGFNSNEELDNQPYYKLWHLLYSFEGDNTPTGNGRLIQKMTELY
GFEKEYATILANVSFQDDYGSLSAKAIHKILPHLKEGNRYDVACVYAGYRHSESSLTREEIA
NKVLKDRLMLLPKNSLHNPVVEKILNQMVNVINVIIDIYGKPDEIRVELARELKKNAKEREE
LTKSIAQTTKAHEEYKTLLQTEFGLTNVSRTDILRYKLYKELESCGYKTLYSNTYISREKLF
SKEFDIEHIIPQARLFDDSFSNKTLEARSVNIEKGNKTAYDFVKEKFGESGADNSLEHYLNN
IEDLFKSGKISKTKYNKLKMAEQDIPDGFIERDLRNTQYIAKKALSMLNEISHRVVATSGSV
TDKLREDWQLIDVMKELNWEKYKALGLVEYFEDRDGRQIGRIKDWTKRNDHRHHAMDALTVA
FTKDVFIQYFNNKNASLDPNANEHAIKNKYFQNGRAIAPMPLREFRAEAKKHLENTLISIKA
KNKVITGNINKTRKKGGVNKNMQQTPRGQLHLETIYGSGKQYLTKEEKVNASFDMRKIGTVS
KSAYRDALLKRLYENDNDPKKAFAGKNSLDKQPIWLDKEQMRKVPEKVKIVTLEAIYTIRKE
ISPDLKVDKVIDVGVRKILIDRLNEYGNDAKKAFSNLDKNPIWLNKEKGISIKRVTISGISN
AQSLHVKKDKDGKPILDENGRNIPVDFVNTGNNHHVAVYYRPVIDKRGQLVVDEAGNPKYEL
EEVVVSFFEAVTRANLGLPIIDKDYKTTEGWQFLFSMKQNEYFVFPNEKTGFNPKEIDLLDV
ENYGLISPNLFRVQKFSLKNYVFRHHLETTIKDTSSILRGITWIDFRSSKGLDTIVKVRVNH
IGQIVSVGEY
SEQ ID NO: 308 
MSRKNYVDDYAISLDIGNASVGWSAFTPNYRLVRAKGHELIGVRLFDPADTAESRRMARTTR
RRYSRRRWRLRLLDALFDQALSEIDPSFLARRKYSWVHPDDENNADCWYGSVLFDSNEQDKR
FYEKYPTIYHLRKALMEDDSQHDIREIYLAIHHMVKYRGNFLVEGTLESSNAFKEDELLKLL
GRITRYEMSEGEQNSDIEQDDENKLVAPANGQLADALCATRGSRSMRVDNALEALSAVNDLS
REQRAIVKAIFAGLEGNKLDLAKIFVSKEFSSENKKILGIYFNKSDYEEKCVQIVDSGLLDD
EEREFLDRMQGQYNAIALKQLLGRSTSVSDSKCASYDAHRANWNLIKLQLRTKENEKDINEN
YGILVGWKIDSGQRKSVRGESAYENMRKKANVFFKKMIETSDLSETDKNRLIHDIEEDKLFP
IQRDSDNGVIPHQLHQNELKQIIKKQGKYYPFLLDAFEKDGKQINKIEGLLTFRVPYFVGPL
VVPEDLQKSDNSENHWMVRKKKGEITPWNFDEMVDKDASGRKFIERLVGTDSYLLGEPTLPK
 
NSLLYQEYEVLNELNNVRLSVRTGNHWNDKRRMRLGREEKTLLCQRLFMKGQTVTKRTAENL
LRKEYGRTYELSGLSDESKFTSSLSTYGKMCRIFGEKYVNEHRDLMEKIVELQTVFEDKETL
LHQLRQLEGISEADCALLVNTHYTGWGRLSRKLLTTKAGECKISDDFAPRKHSIIEIMRAED
RNLMEIITDKQLGFSDWIEQENLGAENGSSLMEVVDDLRVSPKVKRGIIQSIRLIDDISKAV
GKRPSRIFLELADDIQPSGRTISRKSRLQDLYRNANLGKEFKGIADELNACSDKDLQDDRLF
LYYTQLGKDMYTGEELDLDRLSSAYDIDHIIPQAVTQNDSIDNRVLVARAENARKTDSFTYM
PQIADRMRNFWQILLDNGLISRVKFERLTRQNEFSEREKERFVQRSLVETRQIMKNVATLMR
QRYGNSAAVIGLNAELTKEMHRYLGFSHKNRDINDYHHAQDALCVGIAGQFAANRGFFADGE
VSDGAQNSYNQYLRDYLRGYREKLSAEDRKQGRAFGFIVGSMRSQDEQKRVNPRTGEVVWSE
EDKDYLRKVMNYRKMLVTQKVGDDFGALYDETRYAATDPKGIKGIPFDGAKQDTSLYGGFSS
AKPAYAVLIESKGKTRLVNVTMQEYSLLGDRPSDDELRKVLAKKKSEYAKANILLRHVPKMQ
LIRYGGGLMVIKSAGELNNAQQLWLPYEEYCYFDDLSQGKGSLEKDDLKKLLDSILGSVQCL
YPWHRFTEEELADLHVAFDKLPEDEKKNVITGIVSALHADAKTANLSIVGMTGSWRRMNNKS
GYTFSDEDEFIFQSPSGLFEKRVTVGELKRKAKKEVNSKYRTNEKRLPTLSGASQP
SEQ ID NO: 309 
METQTSNQLITSHLKDYPKQDYFVGLDIGTNSVGWAVTNTSYELLKFHSHKMWGSRLFEEGE
SAVTRRGFRSMRRRLERRKLRLKLLEELFADAMAQVDSTFFIRLHESKYHYEDKTTGHSSKH
ILFIDEDYTDQDYFTEYPTIYHLRKDLMENGTDDIRKLFLAVHHILKYRGNFLYEGATFNSN
AFTFEDVLKQALVNITFNCFDTNSAISSISNILMESGKTKSDKAKAIERLVDTYTVFDEVNT
PDKPQKEQVKEDKKTLKAFANLVLGLSANLIDLFGSVEDIDDDLKKLQIVGDTYDEKRDELA
KVWGDEIHIIDDCKSVYDAIILMSIKEPGLTISQSKVKAFDKHKEDLVILKSLLKLDRNVYN
EMFKSDKKGLHNYVHYIKQGRTEETSCSREDFYKYTKKIVEGLADSKDKEYILNEIELQTLL
PLQRIKDNGVIPYQLHLEELKVILDKCGPKFPFLHTVSDGFSVTEKLIKMLEFRIPYYVGPL
NTHHNIDNGGFSWAVRKQAGRVTPWNFEEKIDREKSAAAFIKNLTNKCTYLFGEDVLPKSSL
LYSEFMLLNELNNVRIDGKALAQGVKQHLIDSIFKQDHKKMTKNRIELFLKDNNYITKKHKP
EITGLDGEIKNDLTSYRDMVRILGNNFDVSMAEDIITDITIFGESKKMLRQTLRNKFGSQLN
DETIKKLSKLRYRDWGRLSKKLLKGIDGCDKAGNGAPKTIIELMRNDSYNLMEILGDKFSFM
ECIEEENAKLAQGQVVNPHDIIDELALSPAVKRAVWQALRIVDEVAHIKKALPSRIFVEVAR
TNKSEKKKKDSRQKRLSDLYSAIKKDDVLQSGLQDKEFGALKSGLANYDDAALRSKKLYLYY
TQMGRCAYTGNIIDLNQLNTDNYDIDHIYPRSLTKDDSFDNLVLCERTANAKKSDIYPIDNR
IQTKQKPFWAFLKHQGLISERKYERLTRIAPLTADDLSGFIARQLVETNQSVKATTTLLRRL
YPDIDVVFVKAENVSDFRHNNNFIKVRSLNHHHAKDAYLNIVVGNVYHEKFTRNFRLFFKK
NGANRTYNLAKMFNYDVICTNAQDGKAWDVKTSMNTVKKMMASNDVRVTRRLLEQSGALADA
TIYKASVAAKAKDGAYIGMKTKYSVFADVTKYGGMTKIKNAYSIIVQYTGKKGEEIKEIVPL
PIYLINRNATDIELIDYVKSVIPKAKDISIKYRKLCINQLVKVNGFYYYLGGKTNDKIYIDN
AIELVVPHDIATYIKLLDKYDLLRKENKTLKASSITTSIYNINTSTVVSLNKVGIDVFDYFM
SKLRTPLYMKMKGNKVDELSSTGRSKFIKMTLEEQSIYLLEVLNLLTNSKTTFDVKPLGITG
SRSTIGVKIHNLDEFKIINESITGLYSNEVTIV
SEQ ID NO: 310 
MTKLNQPYGIGLDIGSNSIGFAVVDANSHLLRLKGETAIGARLFREGQSAADRRGSRTTRRR
LSRTRWRLSFLRDFFAPHITKIDPDFFLRQKYSEISPKDKDRFKYEKRLFNDRTDAEFYEDY
PSMYHLRLHLMTHTHKADPREIFLAIHHILKSRGHFLTPGAAKDFNTDKVDLEDIFPALTEA
YAQVYPDLELTFDLAKADDFKAKLLDEQATPSDTQKALVNLLLSSDGEKEIVKKRKQVLTEF
AKAITGLKTKFNLALGTEVDEADASNWQFSMGQLDDKWSNIETSMTDQGTEIFEQIQELYRA
RLLNGIVPAGMSLSQAKVADYGQHKEDLELFKTYLKKLNDHELAKTIRGLYDRYINGDDAKP
FLREDFVKALTKEVTAHPNEVSEQLLNRMGQANFMLKQRTKANGAIPIQLQQRELDQIIANQ
SKYYDWLAAPNPVEAHRWKMPYQLDELLNFHIPYYVGPLITPKQQAESGENVFAWMVRKDPS
GNITPYNFDEKVDREASANTFIQRMKTTDTYLIGEDVLPKQSLLYQKYEVLNELNNVRINNE
CLGTDQKQRLIREVFERHSSVTIKQVADNLVAHGDFARRPEIRGLADEKRFLSSLSTYHQLK
 
EILHEAIDDPTKLLDIENIITWSTVFEDHTIFETKLAEIEWLDPKKINELSGIRYRGWGQFS
RKLLDGLKLGNGHTVIQELMLSNHNLMQILADETLKETMTELNQDKLKTDDIEDVINDAYTS
PSNKKALRQVLRVVEDIKHAANGQDPSWLFIETADGTGTAGKRTQSRQKQIQTVYANAAQEL
IDSAVRGELEDKIADKASFTDRLVLYFMQGGRDIYTGAPLNIDQLSHYDIDHILPQSLIKDD
SLDNRVLVNATINREKNNVFASTLFAGKMKATWRKWHEAGLISGRKLRNLMLRPDEIDKFAK
GFVARQLVETRQIIKLTEQIAAAQYPNTKIIAVKAGLSHQLREELDFPKNRDVNHYHHAFDA
FLAARIGTYLLKRYPKLAPFFTYGEFAKVDVKKFREFNFIGALTHAKKNIIAKDTGEIVWDK
ERDIRELDRIYNFKRMLITHEVYFETADLFKQTIYAAKDSKERGGSKQLIPKKQGYPTQVYG
GYTQESGSYNALVRVAEADTTAYQVIKISAQNASKIASANLKSREKGKQLLNEIVVKQLAKR
RKNWKPSANSFKIVIPRFGMGTLFQNAKYGLFMVNSDTYYRNYQELWLSRENQKLLKKLFSI
KYEKTQMNHDALQVYKAIIDQVEKFFKLYDINQFRAKLSDAIERFEKLPINTDGNKIGKTET
LRQILIGLQANGTRSNVKNLGIKTDLGLLQVGSGIKLDKDTQIVYQSPSGLFKRRIPLADL
SEQ ID NO: 311 
MTKEYYLGLDVGTNSVGWAVTDSQYNLCKFKKKDMWGIRLFESANTAKDRRLQRGNRRRLER
KKQRIDLLQEIFSPEICKIDPTFFIRLNESRLHLEDKSNDFKYPLFIEKDYSDIEYYKEFPT
IFHLRKHLIESEEKQDIRLIYLALHNIIKTRGHFLIDGDLQSAKQLRPILDTFLLSLQEEQN
LSVSLSENQKDEYEEILKNRSIAKSEKVKKLKNLFEISDELEKEEKKAQSAVIENFCKFIVG
NKGDVCKFLRVSKEELEIDSFSFSEGKYEDDIVKNLEEKVPEKVYLFEQMKAMYDWNILVDI
LETEEYISFAKVKQYEKHKTNLRLLRDIILKYCTKDEYNRMFNDEKEAGSYTAYVGKLKKNN
KKYWIEKKRNPEEFYKSLGKLLDKIEPLKEDLEVLTMMIEECKNHTLLPIQKNKDNGVIPHQ
VHEVELKKILENAKKYYSFLTETDKDGYSVVQKIESIFRFRIPYYVGPLSTRHQEKGSNVWM
VRKPGREDRIYPWNMEEIIDFEKSNENFITRMTNKCTYLIGEDVLPKHSLLYSKYMVLNELN
NVKVRGKKLPTSLKQKVFEDLFENKSKVTGKNLLEYLQIQDKDIQIDDLSGFDKDFKTSLKS
YLDFKKQIFGEEIEKESIQNMIEDIIKWITIYGNDKEMLKRVIRANYSNQLTEEQMKKITGF
QYSGWGNFSKMFLKGISGSDVSTGETFDIITAMWETDNNLMQILSKKFTFMDNVEDFNSGKV
GKIDKITYDSTVKEMFLSPENKRAVWQTIQVAEEIKKVMGCEPKKIFIEMARGGEKVKKRTK
SRKAQLLELYAACEEDCRELIKEIEDRDERDFNSMKLFLYYTQFGKCMYSGDDIDINELIRG
NSKWDRDHIYPQSKIKDDSIDNLVLVNKTYNAKKSNELLSEDIQKKMHSFWLSLLNKKLITK
SKYDRLTRKGDFTDEELSGFIARQLVETRQSTKAIADIFKQIYSSEVVYVKSSLVSDFRKKP
LNYLKSRRVNDYHHAKDAYLNIVVGNVYNKKFTSNPIQWMKKNRDTNYSLNKVFEHDVVING
EVIWEKCTYHEDTNTYDGGTLDRIRKIVERDNILYTEYAYCEKGELFNATIQNKNGNSTVSL
KKGLDVKKYGGYFSANTSYFSLIEFEDKKGDRARHIIGVPIYIANMLEHSPSAFLEYCEQKG
YQNVRILVEKIKKNSLLIINGYPLRIRGENEVDTSFKRAIQLKLDQKNYELVRNIEKFLEKY
VEKKGNYPIDENRDHITHEKMNQLYEVLLSKMKKFNKKGMADPSDRIEKSKPKFIKLEDLID
KINVINKMLNLLRCDNDTKADLSLIELPKNAGSFVVKKNTIGKSKIILVNQSVTGLYENRRE
L
SEQ ID NO: 312 
MARDYSVGLDIGTSSVGWAAIDNKYHLIRAKSKNLIGVRLFDSAVTAEKRRGYRTTRRRLSR
RHWRLRLLNDIFAGPLTDFGDENFLARLKYSWVHPQDQSNQAHFAAGLLFDSKEQDKDFYRK
YPTIYHLRLALMNDDQKHDLREVYLAIHHLVKYRGHFLIEGDVKADSAFDVHTFADAIQRYA
ESNNSDENLLGKIDEKKLSAALTDKHGSKSQRAETAETAFDILDLQSKKQIQAILKSVVGNQ
ANLMAIFGLDSSAISKDEQKNYKFSFDDADIDEKIADSEALLSDTEFEFLCDLKAAFDGLTL
KMLLGDDKTVSAAMVRRFNEHQKDWEYIKSHIRNAKNAGNGLYEKSKKFDGINAAYLALQSD
NEDDRKKAKKIFQDEISSADIPDDVKADFLKKIDDDQFLPIQRTKNNGTIPHQLHRNELEQI
IEKQGIYYPFLKDTYQENSHELNKITALINFRVPYYVGPLVEEEQKIADDGKNIPDPTNHWM
VRKSNDTITPWNLSQVVDLDKSGRRFIERLTGTDTYLIGEPTLPKNSLLYQKFDVLQELNNI
RVSGRRLDIRAKQDAFEHLFKVQKTVSATNLKDFLVQAGYISEDTQIEGLADVNGKNFNNAL
TTYNYLVSVLGREFVENPSNEELLEEITELQTVFEDKKVLRRQLDQLDGLSDHNREKLSRKH
YTGWGRISKKLLTTKIVQNADKIDNQTFDVPRMNQSIIDTLYNTKMNLMEIINNAEDDFGVR
 
AWIDKQNTTDGDEQDVYSLIDELAGPKEIKRGIVQSFRILDDITKAVGYAPKRVYLEFARKT
QESHLTNSRKNQLSTLLKNAGLSELVTQVSQYDAAALQNDRLYLYFLQQGKDMYSGEKLNLD
NLSNYDIDHIIPQAYTKDNSLDNRVLVSNITNRRKSDSSNYLPALIDKMRPFWSVLSKQGLL
SKHKFANLTRTRDFDDMEKERFIARSLVETRQIIKNVASLIDSHFGGETKAVAIRSSLTADM
RRYVDIPKNRDINDYHHAFDALLFSTVGQYTENSGLMKKGQLSDSAGNQYNRYIKEWIHAAR
LNAQSQRVNPFGFVVGSMRNAAPGKLNPETGEITPEENADWSIADLDYLHKVMNFRKITVTR
RLKDQKGQLYDESRYPSVLHDAKSKASINFDKHKPVDLYGGFSSAKPAYAALIKFKNKFRLV
NVLRQWTYSDKNSEDYILEQIRGKYPKAEMVLSHIPYGQLVKKDGALVTISSATELHNFEQL
WLPLADYKLINTLLKTKEDNLVDILHNRLDLPEMTIESAFYKAFDSILSFAFNRYALHQNAL
VKLQAHRDDFNALNYEDKQQTLERILDALHASPASSDLKKINLSSGFGRLFSPSHFTLADTD
EFIFQSVTGLFSTQKTVAQLYQETK
SEQ ID NO: 313 
MVYDVGLDIGTGSVGWVALDENGKLARAKGKNLVGVRLFDTAQTAADRRGFRTTRRRLSRRK
WRLRLLDELFSAEINEIDSSFFQRLKYSYVHPKDEENKAHYYGGYLFPTEEETKKFHRSYPT
IYHLRQELMAQPNKRFDIREIYLAIHHLVKYRGHFLSSQEKITIGSTYNPEDLANAIEVYAD
EKGLSWELNNPEQLTEIISGEAGYGLNKSMKADEALKLFEFDNNQDKVAIKTLLAGLTGNQI
DFAKLFGKDISDKDEAKLWKLKLDDEALEEKSQTILSQLTDEEIELFHAVVQAYDGFVLIGL
LNGADSVSAAMVQLYDQHREDRKLLKSLAQKAGLKHKRFSEIYEQLALATDEATIKNGISTA
RELVEESNLSKEVKEDTLRRLDENEFLPKQRTKANSVIPHQLHLAELQKILQNQGQYYPFLL
DTFEKEDGQDNKIEELLRFRIPYYVGPLVTKKDVEHAGGDADNHWVERNEGFEKSRVTPWNF
DKVFNRDKAARDFIERLTGNDTYLIGEKTLPQNSLRYQLFTVLNELNNVRVNGKKFDSKTKA
DLINDLFKARKTVSLSALKDYLKAQGKGDVTITGLADESKFNSSLSSYNDLKKTFDAEYLEN
EDNQETLEKIIEIQTVFEDSKIASRELSKLPLDDDQVKKLSQTHYTGWGRLSEKLLDSKIID
ERGQKVSILDKLKSTSQNFMSIINNDKYGVQAWITEQNTGSSKLTFDEKVNELTTSPANKRG
IKQSFAVLNDIKKAMKEEPRRVYLEFAREDQTSVRSVPRYNQLKEKYQSKSLSEEAKVLKKT
LDGNKNKMSDDRYFLYFQQQGKDMYTGRPINFERLSQDYDIDHIIPQAFTKDDSLDNRVLVS
RPENARKSDSFAYTDEVQKQDGSLWTSLLKSGFINRKKYERLTKAGKYLDGQKTGFIARQLV
ETRQIIKNVASLIEGEYENSKAVAIRSEITADMRLLVGIKKHREINSFHHAFDALLITAAGQ
YMQNRYPDRDSTNVYNEFDRYTNDYLKNLRQLSSRDEVRRLKSFGFVVGTMRKGNEDWSEEN
TSYLRKVMMFKNILTTKKTEKDRGPLNKETIFSPKSGKKLIPLNSKRSDTALYGGYSNVYSA
YMTLVRANGKNLLIKIPISIANQIEVGNLKINDYIVNNPAIKKFEKILISKLPLGQLVNEDG
NLIYLASNEYRHNAKQLWLSTTDADKIASISENSSDEELLEAYDILTSENVKNRFPFFKKDI
DKLSQVRDEFLDSDKRIAVIQTILRGLQIDAAYQAPVKIISKKVSDWHKLQQSGGIKLSDNS
EMIYQSATGIFETRVKISDLL
SEQ ID NO: 314 
IVDYCIGLDLGTGSVGWAVVDMNHRLMKRNGKHLWGSRLFSNAETAANRRASRSIRRRYNKR
RERIRLLRAILQDMVLEKDPTFFIRLEHTSFLDEEDKAKYLGTDYKDNYNLFIDEDFNDYTY
YHKYPTIYHLRKALCESTEKADPRLIYLALHHIVKYRGNFLYEGQKFNMDASNIEDKLSDIF
TQFTSFNNIPYEDDEKKNLEILEILKKPLSKKAKVDEVMTLIAPEKDYKSAFKELVTGIAGN
KMNVTKMILCEPIKQGDSEIKLKFSDSNYDDQFSEVEKDLGEYVEFVDALHNVYSWVELQTI
MGATHTDNASISEAMVSRYNKHHDDLKLLKDCIKNNVPNKYFDMFRNDSEKSKGYYNYINRP
SKAPVDEFYKYVKKCIEKVDTPEAKQILNDIELENFLLKQNSRTNGSVPYQMQLDEMIKIID
NQAEYYPILKEKREQLLSILTFRIPYYFGPLNETSEHAWIKRLEGKENQRILPWNYQDIVDV
DATAEGFIKRMRSYCTYFPDEEVLPKNSLIVSKYEVYNELNKIRVDDKLLEVDVKNDIYNEL
FMKNKTVTEKKLKNWLVNNQCCSKDAEIKGFQKENQFSTSLTPWIDFTNIFGKIDQSNFDLI
ENIIYDLTVFEDKKIMKRRLKKKYALPDDKVKQILKLKYKDWSRLSKKLLDGIVADNRFGSS
VTVLDVLEMSRLNLMEIINDKDLGYAQMIEEATSCPEDGKFTYEEVERLAGSPALKRGIWQS
LQIVEEITKVMKCRPKYIYIEFERSEEAKERTESKIKKLENVYKDLDEQTKKEYKSVLEELK
GFDNTKKISSDSLFLYFTQLGKCMYSGKKLDIDSLDKYQIDHIVPQSLVKDDSFDNRVLVVP
 
SENQRKLDDLVVPFDIRDKMYRFWKLLFDHELISPKKFYSLIKTEYTERDEERFINRQLVET
RQITKNVTQIIEDHYSTTKVAAIRANLSHEFRVKNHIYKNRDINDYHHAHDAYIVALIGGFM
RDRYPNMHDSKAVYSEYMKMFRKNKNDQKRWKDGFVINSMNYPYEVDGKLIWNPDLINEIKK
CFYYKDCYCTTKLDQKSGQLFNLTVLSNDAHADKGVTKAVVPVNKNRSDVHKYGGFSGLQYT
IVAIEGQKKKGKKTELVKKISGVPLHLKAASINEKINYIEEKEGLSDVRIIKDNIPVNQMIE
MDGGEYLLTSPTEYVNARQLVLNEKQCALIADIYNAIYKQDYDNLDDILMIQLYIELTNKMK
VLYPAYRGIAEKFESMNENYVVISKEEKANIIKQMLIVMHRGPQNGNIVYDDFKISDRIGRL
KTKNHNLNNIVFISQSPTGIYTKKYKL
SEQ ID NO: 315 
MKSEKKYYIGLDVGTNSVGWAVTDEFYNILRAKGKDLWGVRLFEKADTAANTRIFRSGRRRN
DRKGMRLQILREIFEDEIKKVDKDFYDRLDESKFWAEDKKVSGKYSLFNDKNFSDKQYFEKF
PTIFHLRKYLMEEHGKVDIRYYFLAINQMMKRRGHFLIDGQISHVTDDKPLKEQLILLINDL
LKIELEEELMDSIFEILADVNEKRTDKKNNLKELIKGQDFNKQEGNILNSIFESIVTGKAKI
KNIISDEDILEKIKEDNKEDFVLTGDSYEENLQYFEEVLQENITLFNTLKSTYDFLILQSIL
KGKSTLSDAQVERYDEHKKDLEILKKVIKKYDEDGKLFKQVFKEDNGNGYVSYIGYYLNKNK
KITAKKKISNIEFTKYVKGILEKQCDCEDEDVKYLLGKIEQENFLLKQISSINSVIPHQIHL
FELDKILENLAKNYPSFNNKKEEFTKIEKIRKTFTFRIPYYVGPLNDYHKNNGGNAWIFRNK
GEKIRPWNFEKIVDLHKSEEEFIKRMLNQCTYLPEETVLPKSSILYSEYMVLNELNNLRING
KPLDTDVKLKLIEELFKKKTKVTLKSIRDYMVRNNFADKEDFDNSEKNLEIASNMKSYIDFN
NILEDKFDVEMVEDLIEKITIHTGNKKLLKKYIEETYPDLSSSQIQKIINLKYKDWGRLSRK
LLDGIKGTKKETEKTDTVINFLRNSSDNLMQIIGSQNYSFNEYIDKLRKKYIPQEISYEVVE
NLYVSPSVKKMIWQVIRVTEEITKVMGYDPDKIFIEMAKSEEEKKTTISRKNKLLDLYKAIK
KDERDSQYEKLLTGLNKLDDSDLRSRKLYLYYTQMGRDMYTGEKIDLDKLFDSTHYDKDHII
PQSMKKDDSIINNLVLVNKNANQTTKGNIYPVPSSIRNNPKIYNYWKYLMEKEFISKEKYNR
LIRNTPLTNEELGGFINRQLVETRQSTKAIKELFEKFYQKSKIIPVKASLASDLRKDMNTLK
SREVNDLHHAHDAFLNIVAGDVWNREFTSNPINYVKENREGDKVKYSLSKDFTRPRKSKGKV
IWTPEKGRKLIVDTLNKPSVLISNESHVKKGELFNATIAGKKDYKKGKIYLPLKKDDRLQDV
SKYGGYKAINGAFFFLVEHTKSKKRIRSIELFPLHLLSKFYEDKNTVLDYAINVLQLQDPKI
IIDKINYRTEIIIDNFSYLISTKSNDGSITVKPNEQMYWRVDEISNLKKIENKYKKDAILTE
EDRKIMESYIDKIYQQFKAGKYKNRRTTDTIIEKYEIIDLDTLDNKQLYQLLVAFISLSYKT
SNNAVDFTVIGLGTECGKPRITNLPDNTYLVYKSITGIYEKRIRIK
SEQ ID NO: 316 
MKLRGIEDDYSIGLDMGTSSVGWAVTDERGTLAHFKRKPTWGSRLFREAQTAAVARMPRGQR
RRYVRRRWRLDLLQKLFEQQMEQADPDFFIRLRQSRLLRDDRAEEHADYRWPLFNDCKFTER
DYYQRFPTIYHVRSWLMETDEQADIRLIYLALHNIVKHRGNFLREGQSLSAKSARPDEALNH
LRETLRVWSSERGFECSIADNGSILAMLTHPDLSPSDRRKKIAPLFDVKSDDAAADKKLGIA
LAGAVIGLKTEFKNIFGDFPCEDSSIYLSNDEAVDAVRSACPDDCAELFDRLCEVYSAYVLQ
GLLSYAPGQTISANMVEKYRRYGEDLALLKKLVKIYAPDQYRMFFSGATYPGTGIYDAAQAR
GYTKYNLGPKKSEYKPSESMQYDDFRKAVEKLFAKTDARADERYRMMMDRFDKQQFLRRLKT
SDNGSIYHQLHLEELKAIVENQGRFYPFLKRDADKLVSLVSFRIPYYVGPLSTRNARTDQHG
ENRFAWSERKPGMQDEPIFPWNWESIIDRSKSAEKFILRMTGMCTYLQQEPVLPKSSLLYEE
FCVLNELNGAHWSIDGDDEHRFDAADREGIIEELFRRKRTVSYGDVAGWMERERNQIGAHVC
GGQGEKGFESKLGSYIFFCKDVFKVERLEQSDYPMIERIILWNTLFEDRKILSQRLKEEYGS
RLSAEQIKTICKKRFTGWGRLSEKFLTGITVQVDEDSVSIMDVLREGCPVSGKRGRAMVMME
ILRDEELGFQKKVDDFNRAFFAENAQALGVNELPGSPAVRRSLNQSIRIVDEIASIAGKAPA
NIFIEVTRDEDPKKKGRRTKRRYNDLKDALEAFKKEDPELWRELCETAPNDMDERLSLYFMQ
RGKCLYSGRAIDIHQLSNAGIYEVDHIIPRTYVKDDSLENKALVYREENQRKTDMLLIDPEI
RRRMSGYWRMLHEAKLIGDKKFRNLLRSRIDDKALKGFIARQLVETGQMVKLVRSLLEARYP
ETNIISVKASISHDLRTAAELVKCREANDFHHAHDAFLACRVGLFIQKRHPCVYENPIGLSQ
 
VVRNYVRQQADIFKRCRTIPGSSGFIVNSFMTSGFDKETGEIFKDDWDAEAVEGIRRSLNF
RQCFISRMPFEDHGVFWDATIYSPRAKKTAALPLKQGLNPSRYGSFSREQFAYFFIYKARNP
RKEQTLFEFAQVPVRLSAQIRQDENALERYARELAKDQGLEFIRIERSKILKNQLIEIDGDR
LCITGKEEVRNACELAFAQDEMRVIRMLVSEKPVSRECVISLFNRILLHGDQASRRLSKQLK
LALLSEAFSEASDNVQRNVVLGLIAIFNGSTNMVNLSDIGGSKFAGNVRIKYKKELASPKVN
VHLIDQSVTGMFERRTKIGL
SEQ ID NO: 317 
MENKQYYIGLDVGTNSVGWAVTDTSYNLLRAKGKDMWGARLFEKANTAAERRTKRTSRRRSE
REKARKAMLKELFADEINRVDPSFFIRLEESKFFLDDRSENNRQRYTLFNDATFTDKDYYEK
YKTIFHLRSALINSDEKFDVRLVFLAILNLFSHRGHFLNASLKGDGDIQGMDVFYNDLVESC
EYFEIELPRITNIDNFEKILSQKGKSRTKILEELSEELSISKKDKSKYNLIKLISGLEASVV
ELYNIEDIQDENKKIKIGFRESDYEESSLKVKEIIGDEYFDLVERAKSVHDMGLLSNIIGNS
KYLCEARVEAYENHHKDLLKIKELLKKYDKKAYNDMFRKMTDKNYSAYVGSVNSNIAKERRS
VDKRKIEDLYKYIEDTALKNIPDDNKDKIEILEKIKLGEFLKKQLTASNGVIPNQLQSRELR
AILKKAENYLPFLKEKGEKNLTVSEMIIQLFEFQIPYYVGPLDKNPKKDNKANSWAKIKQGG
RILPWNFEDKVDVKGSRKEFIEKMVRKCTYISDEHTLPKQSLLYEKFMVLNEINNIKIDGEK
ISVEAKQKIYNDLFVKGKKVSQKDIKKELISLNIMDKDSVLSGTDTVCNAYLSSIGKFTGVF
KEEINKQSIVDMIEDIIFLKTVYGDEKRFVKEEIVEKYGDEIDKDKIKRILGFKFSNWGNLS
KSFLELEGADVGTGEVRSIIQSLWETNFNLMELLSSRFTYMDELEKRVKKLEKPLSEWTIED
LDDMYLSSPVKRMIWQSMKIVDEIQTVIGYAPKRIFVEMTRSEGEKVRTKSRKDRLKELYNG
IKEDSKQWVKELDSKDESYFRSKKMYLYYLQKGRCMYSGEVIELDKLMDDNLYDIDHIYPRS
FVKDDSLDNLVLVKKEINNRKQNDPITPQIQASCQGFWKILHDQGFMSNEKYSRLTRKTQEF
SDEEKLSFINRQIVETGQATKCMAQILQKSMGEDVDVVFSKARLVSEFRHKFELFKSRLIND
FHHANDAYLNIVVGNSYFVKFTRNPANFIKDARKNPDNPVYKYHMDRFFERDVKSKSEVAWI
GQSEGNSGTIVIVKKTMAKNSPLITKKVEEGHGSITKETIVGVKEIKFGRNKVEKADKTPKK
PNLQAYRPIKTSDERLCNILRYGGRTSISISGYCLVEYVKKRKTIRSLEAIPVYLGRKDSLS
EEKLLNYFRYNLNDGGKDSVSDIRLCLPFISTNSLVKIDGYLYYLGGKNDDRIQLYNAYQLK
MKKEEVEYIRKIEKAVSMSKFDEIDREKNPVLTEEKNIELYNKIQDKFENTVFSKRMSLVKY
NKKDLSFGDFLKNKKSKFEEIDLEKQCKVLYNIIFNLSNLKEVDLSDIGGSKSTGKCRCKKN
ITNYKEFKLIQQSITGLYSCEKDLMTI
SEQ ID NO: 318 
MKNLKEYYIGLDIGTASVGWAVTDESYNIPKFNGKKMWGVRLFDDAKTAEERRTQRGSRRRL
NRRKERINLLQDLFATEISKVDPNFFLRLDNSDLYREDKDEKLKSKYTLFNDKDFKDRDYHK
KYPTIHHLIMDLIEDEGKKDIRLLYLACHYLLKNRGHFIFEGQKFDTKNSFDKSINDLKIHL
RDEYNIDLEFNNEDLIEIITDTTLNKTNKKKELKNIVGDTKFLKAISAIMIGSSQKLVDLFE
DGEFEETTVKSVDFSTTAFDDKYSEYEEALGDTISLLNILKSIYDSSILENLLKDADKSKDG
NKYISKAFVKKFNKHGKDLKTLKRIIKKYLPSEYANIFRNKSINDNYVAYTKSNITSNKRTK
ASKFTKQEDFYKFIKKHLDTIKETKLNSSENEDLKLIDEMLTDIEFKTFIPKLKSSDNGVIP
YQLKLMELKKILDNQSKYYDFLNESDEYGTVKDKVESIMEFRIPYYVGPLNPDSKYAWIKRE
NTKITPWNFKDIVDLDSSREEFIDRLIGRCTYLKEEKVLPKASLIYNEFMVLNELNNLKLNE
FLITEEMKKAIFEELFKTKKKVTLKAVSNLLKKEFNLTGDILLSGTDGDFKQGLNSYIDFKN
IIGDKVDRDDYRIKIEEIIKLIVLYEDDKTYLKKKIKSAYKNDFTDDEIKKIAALNYKDWGR
LSKRFLTGIEGVDKTTGEKGSIIYFMREYNLNLMELMSGHYTFTEEVEKLNPVENRELCYEM
VDELYLSPSVKRMLWQSLRVVDEIKRIIGKDPKKIFIEMARAKEAKNSRKESRKNKLLEFYK
FGKKAFINEIGEERYNYLLNEINSEEESKFRWDNLYLYYTQLGRCMYSLEPIDLADLKSNNI
YDQDHIYPKSKIYDDSLENRVLVKKNLNHEKGNQYPIPEKVLNKNAYGFWKILFDKGLIGQK
KYTRLTRRTPFEERELAEFIERQIVETRQATKETANLLKNICQDSEIVYSKAENASRFRQEF
DIIKCRTVNDLHHMHDAYLNIVVGNVYNTKFTKNPLNFIKDKDNVRSYNLENMFKYDVVRGS
YTAWIADDSEGNVKAATIKKVKRELEGKNYRFTRMSYIGTGGLYDQNLMRKGKGQIPQKENT
 
NKSNIEKYGGYNKASSAYFALIESDGKAGRERTLETIPIMVYNQEKYGNTEAVDKYLKDNLE
LQDPKILKDKIKINSLIKLDGFLYNIKGKTGDSLSIAGSVQLIVNKEEQKLIKKMDKFLVKK
KDNKDIKVTSFDNIKEEELIKLYKTLSDKLNNGIYSNKRNNQAKNISEALDKFKEISIEEKI
DVLNQIILLFQSYNNGCNLKSIGLSAKTGVVFIPKKLNYKECKLINQSITGLFENEVDLLNL
SEQ ID NO: 319 
MGKMYYLGLDIGTNSVGYAVTDPSYHLLKFKGEPMWGAHVFAAGNQSAERRSFRTSRRRLDR
RQQRVKLVQEIFAPVISPIDPRFFIRLHESALWRDDVAETDKHIFFNDPTYTDKEYYSDYPT
IHHLIVDLMESSEKHDPRLVYLAVAWLVAHRGHFLNEVDKDNIGDVLSFDAFYPEFLAFLSD
NGVSPWVCESKALQATLLSRNSVNDKYKALKSLIFGSQKPEDNFDANISEDGLIQLLAGKKV
KVNKLFPQESNDASFTLNDKEDAIEEILGTLTPDECEWIAHIRRLFDWAIMKHALKDGRTIS
ESKVKLYEQHHDLTQLKYFVKTYLAKEYDDIFRNVDSETTKNYVAYSYHVKEVKGTLPKNK
ATQEEFCKYVLGKVKNIECSEADKVDFDEMIQRLTDNSFMPKQVSGENRVIPYQLYYYELKT
ILNKAASYLPFLTQCGKDAISNQDKLLSIMTFRIPYFVGPLRKDNSEHAWLERKAGKIYPWN
FNDKVDLDKSEEAFIRRMTNTCTYYPGEDVLPLDSLIYEKFMILNEINNIRIDGYPISVDVK
QQVFGLFEKKRRVTVKDIQNLLLSLGALDKHGKLTGIDTTIHSNYNTYHHFKSLMERGVLTR
DDVERIVERMTYSDDTKRVRLWLNNNYGTLTADDVKHISRLRKHDFGRLSKMFLTGLKGVHK
ETGERASILDFMWNTNDNLMQLLSECYTFSDEITKLQEAYYAKAQLSLNDFLDSMYISNAVK
RPIYRTLAVVNDIRKACGTAPKRIFIEMARDGESKKKRSVTRREQIKNLYRSIRKDFQQEVD
FLEKILENKSDGQLQSDALYLYFAQLGRDMYTGDPIKLEHIKDQSFYNIDHIYPQSMVKDDS
LDNKVLVQSEINGEKSSRYPLDAAIRNKMKPLWDAYYNHGLISLKKYQRLTRSTPFTDDEKW
DFINRQLVETRQSTKALAILLKRKFPDTEIVYSKAGLSSDFRHEFGLVKSRNINDLHHAKDA
FLAIVTGNVYHERFNRRWFMVNQPYSVKTKTLFTHSIKNGNFVAWNGEEDLGRIVKMLKQNK
NTIHFTRFSFDRKEGLFDIQPLKASTGLVPRKAGLDVVKYGGYDKSTAAYYLLVRFTLEDKK
TQHKLMMIPVEGLYKARIDHDKEFLTDYAQTTISEILQKDKQKVINIMFPMGTRHIKLNSMI
SIDGFYLSIGGKSSKGKSVLCHAMVPLIVPHKIECYIKAMESFARKFKENNKLRIVEKFDKI
TVEDNLNLYELFLQKLQHNPYNKFFSTQFDVLTNGRSTFTKLSPEEQVQTLLNILSIFKTCR
SSGCDLKSINGSAQAARIMISADLTGLSKKYSDIRLVEQSASGLFVSKSQNLLEYL
SEQ ID NO: 320 
MTKKEQPYNIGLDIGTSSVGWAVTNDNYDLLNIKKKNLWGVRLFEEAQTAKETRLNRSTRRR
YRRRKNRINWLNEIFSEELAKTDPSFLIRLQNSWVSKKDPDRKRDKYNLFIDGPYTDKEYYR
EFPTIFHLRKELILNKDKADIRLIYLALHNILKYRGNFTYEHQKFNISNLNNNLSKELIELN
QQLIKYDISFPDDCDWNHISDILIGRGNATQKSSNILKDFTLDKETKKLLKEVINLILGNVA
HLNTIFKTSLTKDEEKLNFSGKDIESKLDDLDSILDDDQFTVLDAANRIYSTITLNEILNGE
SYFSMAKVNQYENHAIDLCKLRDMWHTTKNEEAVEQSRQAYDDYINKPKYGTKELYTSLKKF
LKVALPTNLAKEAEEKISKGTYLVKPRNSENGVVPYQLNKIEMEKIIDNQSQYYPFLKENKE
KLLSILSFRIPYYVGPLQSAEKNPFAWMERKSNGHARPWNFDEIVDREKSSNKFIRRMTVTD
SYLVGEPVLPKNSLIYQRYEVLNELNNIRITENLKTNPIGSRLTVETKQRIYNELFKKYKKV
TVKKLTKWLIAQGYYKNPILIGLSQKDEFNSTLTTYLDMKKIFGSSFMEDNKNYDQIEELIE
WLTIFEDKQILNEKLHSSKYSYTPDQIKKISNMRYKGWGRLSKKILMDITTETNTPQLLQLS
NYSILDLMWATNNNFISIMSNDKYDFKNYIENHNLNKNEDQNISDLVNDIHVSPALKRGITQ
SIKIVQEIVKFMGHAPKHIFIEVTRETKKSEITTSREKRIKRLQSKLLNKANDFKPQLREYL
VPNKKIQEELKKHKNDLSSERIMLYFLQNGKSLYSEESLNINKLSDYQVDHILPRTYIPDDS
LENKALVLAKENQRKADDLLLNSNVIDRNLERWTYMLNNNMIGLKKFKNLTRRVITDKDKLG
FIHRQLVQTSQMVKGVANILDNMYKNQGTTCIQARANLSTAFRKALSGQDDTYHFKHPELVK
NRNVNDFHHAQDAYLASFLGTYRLRRFPTNEMLLMNGEYNKFYGQVKELYSKKKKLPDSRKN
GFIISPLVNGTTQYDRNTGEIIWNVGFRDKILKIFNYHQCNVTRKTEIKTGQFYDQTIYSPK
NPKYKKLIAQKKDMDPNIYGGFSGDNKSSITIVKIDNNKIKPVAIPIRLINDLKDKKTLQNW
LEENVKHKKSIQIIKNNVPIGQIIYSKKVGLLSLNSDREVANRQQLILPPEHSALLRLLQIP
 
DEDLDQILAFYDKNILVEILQELITKMKKFYPFYKGEREFLIANIENFNQATTSEKVNSLEE
LITLLHANSTSAHLIFNNIEKKAFGRKTHGLTLNNTDFIYQSVTGLYETRIHIE
SEQ ID NO: 321 
MTKFNKNYSIGLDIGVSSVGYAVVTEDYRVPAFKFKVLGNTEKEKIKKNLIGSTTFVSAQPA
KGTRVFRVNRRRIDRRNHRITYLRDIFQKEIEKVDKNFYRRLDESFRVLGDKSEDLQIKQPF
FGDKELETAYHKKYPTIYHLRKHLADADKNSPVADIREVYMAISHILKYRGHFLTLDKINPN
NINMQNSWIDFIESCQEVFDLEISDESKNIADIFKSSENRQEKVKKILPYFQQELLKKDKSI
FKQLLQLLFGLKTKFKDCFELEEEPDLNFSKENYDENLENFLGSLEEDFSDVFAKLKVLRDT
ILLSGMLTYTGATHARFSATMVERYEEHRKDLQRFKFFIKQNLSEQDYLDIFGRKTQNGFDV
DKETKGYVGYITNKMVLTNPQKQKTIQQNFYDYISGKITGIEGAEYFLNKISDGTFLRKLRT
SDNGAIPNQIHAYELEKIIERQGKDYPFLLENKDKLLSILTFKIPYYVGPLAKGSNSRFAWI
KRATSSDILDDNDEDTRNGKIRPWNYQKLINMDETRDAFITNLIGNDIILLNEKVLPKRSLI
YEEVMLQNELTRVKYKDKYGKAHFFDSELRQNIINGLFKNNSKRVNAKSLIKYLSDNHKDLN
AIEIVSGVEKGKSFNSTLKTYNDLKTIFSEELLDSEIYQKELEEIIKVITVFDDKKSIKNYL
TKFFGHLEILDEEKINQLSKLRYSGWGRYSAKLLLDIRDEDTGFNLLQFLRNDEENRNLTKL
ISDNTLSFEPKIKDIQSKSTIEDDIFDEIKKLAGSPAIKRGILNSIKIVDELVQIIGYPPHN
IVIEMARENMTTEEGQKKAKTRKTKLESALKNIENSLLENGKVPHSDEQLQSEKLYLYYLQN
GKDMYTLDKTGSPAPLYLDQLDQYEVDHIIPYSFLPIDSIDNKVLTHRENNQQKLNNIPDKE
TVANMKPFWEKLYNAKLISQTKYQRLTTSERTPDGVLTESMKAGFIERQLVETRQIIKHVAR
ILDNRFSDTKIITLKSQLITNFRNTFHIAKIRELNDYHHAHDAYLAVVVGQTLLKVYPKLAP
ELIYGHHAHFNRHEENKATLRKHLYSNIMRFFNNPDSKVSKDIWDCNRDLPIIKDVIYNSQI
NFVKRTMIKKGAFYNQNPVGKFNKQLAANNRYPLKTKALCLDTSIYGGYGPMNSALSIIIIA
ERFNEKKGKIETVKEFHDIFIIDYEKFNNNPFQFLNDTSENGFLKKNNINRVLGFYRIPKYS
LMQKIDGTRMLFESKSNLHKATQFKLTKTQNELFFHMKRLLTKSNLMDLKSKSAIKESQNFI
LKHKEEFDNISNQLSAFSQKMLGNTTSLKNLIKGYNERKIKEIDIRDETIKYFYDNFIKMFS
FVKSGAPKDINDFFDNKCTVARMRPKPDKKLLNATLIHQSITGLYETRIDLSKLGED
SEQ ID NO: 322 
MKQEYFLGLDMGTGSLGWAVTDSTYQVMRKHGKALWGTRLFESASTAEERRMFRTARRRLDR
RNWRIQVLQEIFSEEISKVDPGFFLRMKESKYYPEDKRDAEGNCPELPYALFVDDNYTDKNY
HKDYPTIYHLRKMLMETTEIPDIRLVYLVLHHMMKHRGHFLLSGDISQIKEFKSTFEQLIQN
IQDEELEWHISLDDAAIQFVEHVLKDRNLTRSTKKSRLIKQLNAKSACEKAILNLLSGGTVK
LSDIFNNKELDESERPKVSFADSGYDDYIGIVEAELAEQYYIIASAKAVYDWSVLVEILGNS
VSISEAKIKVYQKHQADLKTLKKIVRQYMTKEDYKRVFVDTEEKLNNYSAYIGMTKKNGKKV
DLKSKQCTQADFYDFLKKNVIKVIDHKEITQEIESEIEKENFLPKQVTKDNGVIPYQVHDYE
LKKILDNLGTRMPFIKENAEKIQQLFEFRIPYYVGPLNRVDDGKDGKFTWSVRKSDARIYPW
NFTEVIDVEASAEKFIRRMTNKCTYLVGEDVLPKDSLVYSKFMVLNELNNLRLNGEKISVEL
KQRIYEELFCKYRKVTRKKLERYLVIEGIAKKGVEITGIDGDFKASLTAYHDFKERLTDVQL
SQRAKEAIVLNVVLFGDDKKLLKQRLSKMYPNLTTGQLKGICSLSYQGWGRLSKTFLEEITV
PAPGTGEVWNIMTALWQTNDNLMQLLSRNYGFTNEVEEFNTLKKETDLSYKTVDELYVSPAV
KRQIWQTLKVVKEIQKVMGNAPKRVFVEMAREKQEGKRSDSRKKQLVELYRACKNEERDWIT
ELNAQSDQQLRSDKLFLYYIQKGRCMYSGETIQLDELWDNTKYDIDHIYPQSKTMDDSLNNR
VLVKKNYNAIKSDTYPLSLDIQKKMMSFWKMLQQQGFITKEKYVRLVRSDELSADELAGFIE
RQIVETRQSTKAVATILKEALPDTEIVYVKAGNVSNFRQTYELLKVREMNDLHHAKDAYLNI
VVGNAYFVKFTKNAAWFIRNNPGRSYNLKRMFEFDIERSGEIAWKAGNKGSIVTVKKVMQKN
NILVTRKAYEVKGGLFDQQIMKKGKGQVPIKGNDERLADIEKYGGYNKAAGTYFMLVKSLDK
KGKEIRTIEFVPLYLKNQIEINHESAIQYLAQERGLNSPEILLSKIKIDTLFKVDGFKMWLS
GRTGNQLIFKGANQLILSHQEAAILKGVVKYVNRKNENKDAKLSERDGMTEEKLLQLYDTFL
DKLSNTVYSIRLSAQIKTLTEKRAKFIGLSNEDQCIVLNEILHMFQCQSGSANLKLIGGPGS
AGILVMNNNITACKQISVINQSPTGIYEKEIDLIKL
SEQ ID NO: 323 
MKKPYSIGLDIGTNSVGWAVVTDDYKVPAKKMKVLGNTDKSHIEKNLLGALLFDSGNTAEDR
RLKRTARRRYTRRRNRILYLQEIFSEEMGKVDDSFFHRLEDSFLVTEDKRGERHPIFGNLEE
EVKYHENFPTIYHLRQYLADNPEKVDLRLVYLALAHIIKFRGHFLIEGKFDTRNNDVQRLFQ
EFLAVYDNTFENSSLQEQNVQVEEILTDKISKSAKKDRVLKLFPNEKSNGRFAEFLKLIVGN
QADFKKHFELEEKAPLQFSKDTYEEELEVLLAQIGDNYAELFLSAKKLYDSILLSGILTVTD
VGTKAPLSASMIQRYNEHQMDLAQLKQFIRQKLSDKYNEVFSDVSKDGYAGYIDGKTNQEAF
YKYLKGLLNKIEGSGYFLDKIEREDFLRKQRTFDNGSIPHQIHLQEMRAIIRRQAEFYPFLA
DNQDRIEKLLTFRIPYYVGPLARGKSDFAWLSRKSADKITPWNFDEIVDKESSAEAFINRMT
NYDLYLPNQKVLPKHSLLYEKFTVYNELTKVKYKTEQGKTAFFDANMKQEIFDGVFKVYRKV
TKDKLMDFLEKEFDEFRIVDLTGLDKENKVFNASYGTYHDLCKILDKDFLDNSKNEKILEDI
VLTLTLFEDREMIRKRLENYSDLLTKEQVKKLERRHYTGWGRLSAELIHGIRNKESRKTILD
YLIDDGNSNRNFMQLINDDALSFKEEIAKAQVIGETDNLNQVVSDIAGSPAIKKGILQSLKI
VDELVKIMGHQPENIVVEMARENQFTNQGRRNSQQRLKGLTDSIKEFGSQILKEHPVENSQL
QNDRLFLYYLQNGRDMYTGEELDIDYLSQYDIDHIIPQAFIKDNSIDNRVLTSSKENRGKSD
DVPSKDVVRKMKSYWSKLLSAKLITQRKFDNLTKAERGGLTDDDKAGFIKRQLVETRQITKH
VARILDERFNTETDENNKKIRQVKIVTLKSNLVSNFRKEFELYKVREINDYHHAHDAYLNAV
IGKALLGVYPQLEPEFVYGDYPHFHGHKENKATAKKFFYSNIMNFFKKDDVRTDKNGEIIWK
KDEHISNIKKVLSYPQVNIVKKVEEQTGGFSKESILPKGNSDKLIPRKTKKFYWDTKKYGGF
DSPIVAYSILVIADIEKGKSKKLKTVKALVGVTIMEKMTFERDPVAFLERKGYRNVQEENII
KLPKYSLFKLENGRKRLLASARELQKGNEIVLPNHLGTLLYHAKNIHKVDEPKHLDYVDKHK
DEFKELLDVVSNFSKKYTLAEGNLEKIKELYAQNNGEDLKELASSFINLLTFTAIGAPATFK
FFDKNIDRKRYTSTTEILNATLIHQSITGLYETRIDLNKLGGD
SEQ ID NO: 324
SEQ ID NO: 325 
MTKPYSIGLDIGTNSVGWAVTTDNYKVPSKKMKVLGNTSKKYIKKNLLGVLLFDSGITAEGR
RLKRTARRRYTRRRNRILYLQEIFSTEMATLDDAFFQRLDDSFLVPDDKRDSKYPIFGNLVE
EKAYHDEFPTIYHLRKYLADSTKKADLRLVYLALAHMIKYRGHFLIEGEFNSKNNDIQKNFQ
DFLDTYNAIFESDLSLENSKQLEEIVKDKISKLEKKDRILKLFPGEKNSGIFSEFLKLIVGN
QADFRKCFNLDEKASLHFSKESYDEDLETLLGYIGDDYSDVFLKAKKLYDAILLSGFLTVTD
NETEAPLSSAMIKRYNEHKEDLALLKEYIRNISLKTYNEVFKDDTKNGYAGYIDGKTNQEDF
YVYLKKLLAEFEGADYFLEKIDREDFLRKQRTFDNGSIPYQIHLQEMRAILDKQAKFYPFLA
KNKERIEKILTFRIPYYVGPLARGNSDFAWSIRKRNEKITPWNFEDVIDKESSAEAFINRMT
SFDLYLPEEKVLPKHSLLYETFNVYNELTKVRFIAESMRDYQFLDSKQKKDIVRLYFKDKRK
VTDKDIIEYLHAIYGYDGIELKGIEKQFNSSLSTYHDLLNIINDKEFLDDSSNEAIIEEIIH
TLTIFEDREMIKQRLSKFENIFDKSVLKKLSRRHYTGWGKLSAKLINGIRDEKSGNTILDYL
IDDGISNRNFMQLIHDDALSFKKKIQKAQIIGDEDKGNIKEVVKSLPGSPAIKKGILQSIKI
VDELVKVMGGRKPESIVVEMARENQYTNQGKSNSQQRLKRLEKSLKELGSKILKENIPAKLS
KIDNNALQNDRLYLYYLQNGKDMYTGDDLDIDRLSNYDIDHIIPQAFLKDNSIDNKVLVSSA
SNRGKSDDVPSLEVVKKRKTFWYQLLKSKLISQRKFDNLTKAERGGLSPEDKAGFIQRQLVE
TRQITKHVARLLDEKFNNKKDENNRAVRTVKIITLKSTLVSQFRKDFELYKVREINDFHHAH
DAYLNAVVASALLKKYPKLEPEFVYGDYPKYNSFRERKSATEKVYFYSNIMNIFKKSISLAD
GRVIERPLIEVNEETGESVWNKESDLATVRRVLSYPQVNVVKKVEEQNHGLDRGKPKGLFNA
NLSSKPKPNSNENLVGAKEYLDPKKYGGYAGISNSFTVLVKGTIEKGAKKKITNVLEFQGIS
ILDRINYRKDKLNFLLEKGYKDIELIIELPKYSLFELSDGSRRMLASILSTNNKRGEIHKGN
QIFLSQKFVKLLYHAKRISNTINENHRKYVENHKKEFEELFYYILEFNENYVGAKKNGKLLN
SAFQSWQNHSIDELCSSFIGPTGSERKGLFELTSRGSAADFEFLGVKIPRYRDYTPSSLLKD
ATLIHQSVTGLYETRIDLAKLGEG
SEQ ID NO: 326 
MKKQKFSDYYLGFDIGTNSVGWCVTDLDYNVLRFNKKDMWGSRLFDEAKTAAERRVQRNSRR
RLKRRKWRLNLLEEIFSDEIMKIDSNFFRRLKESSLWLEDKNSKEKFTLFNDDNYKDYDFYK
QYPTIFHLRDELIKNPEKKDIRLIYLALHSIFKSRGHFLFEGQNLKEIKNFETLYNNLISFL
EDNGINKSIDKDNIEKLEKIICDSGKGLKDKEKEFKGIFNSDKQLVAIFKLSVGSSVSLNDL
FDTDEYKKEEVEKEKISFREQIYEDDKPIYYSILGEKIELLDIAKSFYDFMVLNNILSDSNY
ISEAKVKLYEEHKKDLKNLKYIIRKYNKENYDKLFKDKNENNYPAYIGLNKEKDKKEVVEKS
RLKIDDLIKVIKGYLPKPERIEEKDKTIFNEILNKIELKTILPKQRISDNGTLPYQIHEVEL
EKILENQSKYYDFLNYEENGVSTKDKLLKTFKFRIPYYVGPLNSYHKDKGGNSWIVRKEEGK
ILPWNFEQKVDIEKSAEEFIKRMTNKCTYLNGEDVIPKDSFLYSEYIILNELNKVQVNDEFL
NEENKRKIIDELFKENKKVSEKKFKEYLLVNQIANRTVELKGIKDSFNSNYVSYIKFKDIFG
EKLNLDIYKEISEKSILWKCLYGDDKKIFEKKIKNEYGDILNKDEIKKINSFKFNTWGRLSE
KLLTGIEFINLETGECYSSVMEALRRTNYNLMELLSSKFTLQESIDNENKEMNEVSYRDLIE
ESYVSPSLKRAILQTLKIYEEIKKITGRVPKKVFIEMARGGDESMKNKKIPARQEQLKKLYD
SCGNDIANFSIDIKEMKNSLSSYDNNSLRQKKLYLYYLQFGKCMYTGREIDLDRLLQNNDTY
DIDHIYPRSKVIKDDSFDNLVLVLKNENAEKSNEYPVKKEIQEKMKSFWRFLKEKNFISDEK
YKRLTGKDDFELRGFMARQLVNVRQTTKEVGKILQQIEPEIKIVYSKAEIASSFREMFDFIK
VRELNDTHHAKDAYLNIVAGNVYNTKFTEKPYRYLQEIKENYDVKKIYNYDIKNAWDKENSL
EIVKKNMEKNTVNITRFIKEEKGELFNLNPIKKGETSNEIISIKPKLYDGKDNKLNEKYGYY
TSLKAAYFIYVEHEKKNKKVKTFERITRIDSTLIKNEKNLIKYLVSQKKLLNPKIIKKIYKE
QTLIIDSYPYTFTGVDSNKKVELKNKKQLYLEKKYEQILKNALKFVEDNQGETEENYKFIYL
KKRNNNEKNETIDAVKERYNIEFNEMYDKFLEKLSSKDYKNYINNKLYTNFLNSKEKFKKLK
LWEKSLILREFLKIFNKNTYGKYEIKDSQTKEKLFSFPEDTGRIRLGQSSLGNNKELLEESV
TGLFVKKIKL
SEQ ID NO: 327 
MKNYTIGLDIGVASVGWVCIDENYKILNYNNRHAFGVHEFESAESAAGRRLKRGMRRRYNRR
KKRLQLLQSLFDSYITDSGFFSKTDSQHFWKNNNEFENRSLTEVLSSLRISSRKYPTIYHLR
SDLIESNKKMDLRLVYLALHNLVKYRGHFLQEGNWSEAASAEGMDDQLLELVTRYAELENLS
PLDLSESQWKAAETLLLNRNLTKTDQSKELTAMFGKEYEPFCKLVAGLGVSLHQLFPSSEQA
LAYKETKTKVQLSNENVEEVMELLLEEESALLEAVQPFYQQVVLYELLKGETYVAKAKVSAF
KQYQKDMASLKNLLDKTFGEKVYRSYFISDKNSQREYQKSHKVEVLCKLDQFNKEAKFAETF
YKDLKKLLEDKSKTSIGTTEKDEMLRIIKAIDSNQFLQKQKGIQNAAIPHQNSLYEAEKILR
NQQAHYPFITTEWIEKVKQILAFRIPYYIGPLVKDTTQSPFSWVERKGDAPITPWNFDEQID
KAASAEAFISRMRKTCTYLKGQEVLPKSSLTYERFEVLNELNGIQLRTTGAESDFRHRLSYE
MKCWIIDNVFKQYKTVSTKRLLQELKKSPYADELYDEHTGEIKEVFGTQKENAFATSLSGYI
SMKSILGAVVDDNPAMTEELIYWIAVFEDREILHLKIQEKYPSITDVQRQKLALVKLPGWGR
FSRLLIDGLPLDEQGQSVLDHMEQYSSVFMEVLKNKGFGLEKKIQKMNQHQVDGTKKIRYED
IEELAGSPALKRGIWRSVKIVEELVSIFGEPANIVLEVAREDGEKKRTKSRKDQWEELTKTT
LKNDPDLKSFIGEIKSQGDQRFNEQRFWLYVTQQGKCLYTGKALDIQNLSMYEVDHILPQNF
VKDDSLDNLALVMPEANQRKNQVGQNKMPLEIIEANQQYAMRTLWERLHELKLISSGKLGRL
KKPSFDEVDKDKFIARQLVETRQIIKHVRDLLDERFSKSDIHLVKAGIVSKFRRFSEIPKIR
DYNNKHHAMDALFAAALIQSILGKYGKNFLAFDLSKKDRQKQWRSVKGSNKEFFLFKNFGNL
RLQSPVTGEEVSGVEYMKHVYFELPWQTTKMTQTGDGMFYKESIFSPKVKQAKYVSPKTEKF
VHDEVKNHSICLVEFTFMKKEKEVQETKFIDLKVIEHHQFLKEPESQLAKFLAEKETNSPII
HARIIRTIPKYQKIWIEHFPYYFISTRELHNARQFEISYELMEKVKQLSERSSVEELKIVFG
LLIDQMNDNYPIYTKSSIQDRVQKFVDTQLYDFKSFEIGFEELKKAVAANAQRSDTFGSRIS
KKPKPEEVAIGYESITGLKYRKPRSVVGTKR
SEQ ID NO: 328 
MKKEIKDYFLGLDVGTGSVGWAVTDTDYKLLKANRKDLWGMRCFETAETAEVRRLHRGARRR
IERRKKRIKLLQELFSQEIAKTDEGFFQRMKESPFYAEDKTILQENTLFNDKDFADKTYHKA
YPTINHLIKAWIENKVKPDPRLLYLACHNIIKKRGHFLFEGDFDSENQFDTSIQALFEYLRE
DMEVDIDADSQKVKEILKDSSLKNSEKQSRLNKILGLKPSDKQKKAITNLISGNKINFADLY
DNPDLKDAEKNSISFSKDDFDALSDDLASILGDSFELLLKAKAVYNCSVLSKVIGDEQYLSF
AKVKIYEKHKTDLTKLKNVIKKHFPKDYKKVFGYNKNEKNNNNYSGYVGVCKTKSKKLIINN
SVNQEDFYKFLKTILSAKSEIKEVNDILTEIETGTFLPKQISKSNAEIPYQLRKMELEKILS
NAEKHFSFLKQKDEKGLSHSEKIIMLLTFKIPYYIGPINDNHKKFFPDRCWVVKKEKSPSGK
TTPWNFFDHIDKEKTAEAFITSRTNFCTYLVGESVLPKSSLLYSEYTVLNEINNLQIIIDGK
NICDIKLKQKIYEDLFKKYKKITQKQISTFIKHEGICNKTDEVIILGIDKECTSSLKSYIEL
KNIFGKQVDEISTKNMLEEIIRWATIYDEGEGKTILKTKIKAEYGKYCSDEQIKKILNLKFS
GWGRLSRKFLETVTSEMPGFSEPVNIITAMRETQNNLMELLSSEFTFTENIKKINSGFEDAE
KQFSYDGLVKPLFLSPSVKKMLWQTLKLVKEISHITQAPPKKIFIEMAKGAELEPARTKTRL
KILQDLYNNCKNDADAFSSEIKDLSGKIENEDNLRLRSDKLYLYYTQLGKCMYCGKPIEIGH
VFDTSNYDIDHIYPQSKIKDDSISNRVLVCSSCNKNKEDKYPLKSEIQSKQRGFWNFLQRNN
FISLEKLNRLTRATPISDDETAKFIARQLVETRQATKVAAKVLEKMFPETKIVYSKAETVSM
FRNKFDIVKCREINDFHHAHDAYLNIVVGNVYNTKFTNNPWNFIKEKRDNPKIADTYNYYKV
FDYDVKRNNITAWEKGKTIITVKDMLKRNTPIYTRQAACKKGELFNQTIMKKGLGQHPLKKE
GPFSNISKYGGYNKVSAAYYTLIEYEEKGNKIRSLETIPLYLVKDIQKDQDVLKSYLTDLLG
KKEFKILVPKIKINSLLKINGFPCHITGKTNDSFLLRPAVQFCCSNNEVLYFKKIIRFSEIR
SQREKIGKTISPYEDLSFRSYIKENLWKKTKNDEIGEKEFYDLLQKKNLEIYDMLLTKHKDT
IYKKRPNSATIDILVKGKEKFKSLIIENQFEVILEILKLFSATRNVSDLQHIGGSKYSGVAK
IGNKISSLDNCILIYQSITGIFEKRIDLLKV
SEQ ID NO: 329 
MEGQMKNNGNNLQQGNYYLGLDVGTSSVGWAVTDTDYNVLKFRGKSMWGARLFDEASTAEER
RTHRGNRRRLARRKYRLLLLEQLFEKEIRKIDDNFFVRLHESNLWADDKSKPSKFLLFNDTN
 
FTDKDYLKKYPTIYHLRSDLIHNSTEHDIRLVFLALHHLIKYRGHFIYDNSANGDVKTLDEA
VSDFEEYLNENDIEFNIENKKEFINVLSDKHLTKKEKKISLKKLYGDITDSENINISVLIEM
LSGSSISLSNLFKDIEFDGKQNLSLDSDIEETLNDVVDILGDNIDLLIHAKEVYDIAVLTSS
LGKHKYLCDAKVELFEKNKKDLMILKKYIKKNHPEDYKKIFSSPTEKKNYAAYSQTNSKNVC
SQEEFCLFIKPYIRDMVKSENEDEVRIAKEVEDKSFLTKLKGTNNSVVPYQIHERELNQILK
NIVAYLPFMNDEQEDISVVDKIKLIFKFKIPYYVGPLNTKSTRSWVYRSDEKIYPWNFSNVI
DLDKTAHEFMNRLIGRCTYTNDPVLPMDSLLYSKYNVLNEINPIKVNGKAIPVEVKQAIYTD
LFENSKKKVTRKSIYIYLLKNGYIEKEDIVSGIDIEIKSKLKSHHDFTQIVQENKCTPEEIE
RIIKGILVYSDDKSMLRRWLKNNIKGLSENDVKYLAKLNYKEWGRLSKTLLTDIYTINPEDG
EACSILDIMWNTNATLMEILSNEKYQFKQNIENYKAENYDEKQNLHEELDDMYISPAARRSI
WQALRIVDEIVDIKKSAPKKIFIEMAREKKSAMKKKRTESRKDTLLELYKSCKSQADGFYDE
ELFEKLSNESNSRLRRDQLYLYYTQMGRSMYTGKRIDFDKLINDKNTYDIDHIYPRSKIKDD
SITNRVLVEKDINGEKTDIYPISEDIRQKMQPFWKILKEKGLINEEKYKRLTRNYELTDEEL
SSFVARQLVETQQSTKALATLLKKEYPSAKIVYSKAGNVSEFRNRKDKELPKFREINDLHHA
KDAYLNIVVGNVYDTKFTEKFFNNIRNENYSLKRVFDFSVPGAWDAKGSTFNTIKKYMAKNN
PIIAFAPYEVKGELFDQQIVPKGKGQFPIKQGKDIEKYGGYNKLSSAFLFAVEYKGKKARER
SLETVYIKDVELYLQDPIKYCESVLGLKEPQIIKPKILMGSLFSINNKKLVVTGRSGKQYVC
HHIYQLSINDEDSQYLKNIAKYLQEEPDGNIERQNILNITSVNNIKLFDVLCTKFNSNTYEI
ILNSLKNDVNEGREKFSELDILEQCNILLQLLKAFKCNRESSNLEKLNNKKQAGVIVIPHLF
TKCSVFKVIHQSITGLFEKEMDLLK
SEQ ID NO: 330 
MGRKPYILSLDIGTGSVGYACMDKGFNVLKYHDKDALGVYLFDGALTAQERRQFRTSRRRKN
RRIKRLGLLQELLAPLVQNPNFYQFQRQFAWKNDNMDFKNKSLSEVLSFLGYESKKYPTIYH
LQEALLLKDEKFDPELIYMALYHLVKYRGHFLFDHLKIENLTNNDNMHDFVELIETYENLNN
IKLNLDYEKTKVIYEILKDNEMTKNDRAKRVKNMEKKLEQFSIMLLGLKFNEGKLFNHADNA
EELKGANQSHTFADNYEENLTPFLTVEQSEFIERANKIYLSLTLQDILKGKKSMAMSKVAAY
DKFRNELKQVKDIVYKADSTRTQFKKIFVSSKKSLKQYDATPNDQTFSSLCLFDQYLIRPKK
QYSLLIKELKKIIPQDSELYFEAENDTLLKVLNTTDNASIPMQINLYEAETILRNQQKYHAE
ITDEMIEKVLSLIQFRIPYYVGPLVNDHTASKFGWMERKSNESIKPWNFDEVVDRSKSATQF
IRRMTNKCSYLINEDVLPKNSLLYQEMEVLNELNATQIRLQTDPKNRKYRMMPQIKLFAVEH
IFKKYKTVSHSKFLEIMLNSNHRENFMNHGEKLSIFGTQDDKKFASKLSSYQDMTKIFGDIE
GKRAQIEEIIQWITIFEDKKILVQKLKECYPELTSKQINQLKKLNYSGWGRLSEKLLTHAYQ
GHSIIELLRHSDENFMEILTNDVYGFQNFIKEENQVQSNKIQHQDIANLTTSPALKKGIWST
IKLVRELTSIFGEPEKIIMEFATEDQQKGKKQKSRKQLWDDNIKKNKLKSVDEYKYIIDVAN
KLNNEQLQQEKLWLYLSQNGKCMYSGQSIDLDALLSPNATKHYEVDHIFPRSFIKDDSIDNK
VLVIKKMNQTKGDQVPLQFIQQPYERIAYWKSLNKAGLISDSKLHKLMKPEFTAMDKEGFIQ
RQLVETRQISVHVRDFLKEEYPNTKVIPMKAKMVSEFRKKFDIPKIRQMNDAHHAIDAYLNG
VVYHGAQLAYPNVDLFDFNFKWEKVREKWKALGEFNTKQKSRELFFFKKLEKMEVSQGERLI
SKIKLDMNHFKINYSRKLANIPQQFYNQTAVSPKTAELKYESNKSNEVVYKGLTPYQTYVVA
IKSVNKKGKEKMEYQMIDHYVFDFYKFQNGNEKELALYLAQRENKDEVLDAQIVYSLNKGDL
LYINNHPCYFVSRKEVINAKQFELTVEQQLSLYNVMNNKETNVEKLLIEYDFIAEKVINEYH
HYLNSKLKEKRVRTFFSESNQTHEDFIKALDELFKVVTASATRSDKIGSRKNSMTHRAFLGK
GKDVKIAYTSISGLKTTKPKSLFKLAESRNEL
SEQ ID NO: 331 
MAKILGLDLGTNSIGWAVVERENIDFSLIDKGVRIFSEGVKSEKGIESSRAAERTGYRSARK
IKYRRKLRKYETLKVLSLNRMCPLSIEEVEEWKKSGFKDYPLNPEFLKWLSTDEESNVNPYF
FRDRASKHKVSLFELGRAFYHIAQRRGFLSNRLDQSAEGILEEHCPKIEAIVEDLISIDEIS
TNITDYFFETGILDSNEKNGYAKDLDEGDKKLVSLYKSLLAILKKNESDFENCKSEIIERLN
KKDVLGKVKGKIKDISQAMLDGNYKTLGQYFYSLYSKEKIRNQYTSREEHYLSEFITICKVQ
 
GIDQINEEEKINEKKFDGLAKDLYKAIFFQRPLKSQKGLIGKCSFEKSKSRCAISHPDFEEY
RMWTYLNTIKIGTQSDKKLRFLTQDEKLKLVPKFYRKNDFNFDVLAKELIEKGSSFGFYKSS
KKNDFFYWFNYKPTDTVAACQVAASLKNAIGEDWKTKSFKYQTINSNKEQVSRTVDYKDLWH
LLTVATSDVYLYEFAIDKLGLDEKNAKAFSKTKLKKDFASLSLSAINKILPYLKEGLLYSHA
VFVANIENIVDENIWKDEKQRDYIKTQISEIIENYTLEKSRFEIINGLLKEYKSENEDGKRV
YYSKEAEQSFENDLKKKLVLFYKSNEIENKEQQETIFNELLPIFIQQLKDYEFIKIQRLDQK
VLIFLKGKNETGQIFCTEEKGTAEEKEKKIKNRLKKLYHPSDIEKFKKKIIKDEFGNEKIVL
GSPLTPSIKNPMAMRALHQLRKVLNALILEGQIDEKTIIHIEMARELNDANKRKGIQDYQND
NKKFREDAIKEIKKLYFEDCKKEVEPTEDDILRYQLWMEQNRSEIYEEGKNISICDIIGSNP
AYDIEHTIPRSRSQDNSQMNKTLCSQRFNREVKKQSMPIELNNHLEILPRIAHWKEEADNLT
REIEIISRSIKAAATKEIKDKKIRRRHYLTLKRDYLQGKYDRFIWEEPKVGFKNSQIPDTGI
ITKYAQAYLKSYFKKVESVKGGMVAEFRKIWGIQESFIDENGMKHYKVKDRSKHTHHTIDAI
TIACMTKEKYDVLAHAWTLEDQQNKKEARSIIEASKPWKTFKEDLLKIEEEILVSHYTPDNV
KKQAKKIVRVRGKKQFVAEVERDVNGKAVPKKAASGKTIYKLDGEGKKLPRLQQGDTIRGSL
HQDSIYGAIKNPLNTDEIKYVIRKDLESIKGSDVESIVDEVVKEKIKEAIANKVLLLSSNAQ
QKNKLVGTVWMNEEKRIAINKVRIYANSVKNPLHIKEHSLLSKSKHVHKQKVYGQNDENYAM
AIYELDGKRDFELINIFNLAKLIKQGQGFYPLHKKKEIKGKIVFVPIEKRNKRDVVLKRGQQ
VVFYDKEVENPKDISEIVDFKGRIYIIEGLSIQRIVRPSGKVDEYGVIMLRYFKEARKADDI
KQDNFKPDGVFKLGENKPTRKMNHQFTAFVEGIDFKVLPSGKFEKI
SEQ ID NO: 332 
MEFKKVLGLDIGTNSIGCALLSLPKSIQDYGKGGRLEWLTSRVIPLDADYMKAFIDGKNGLP
QVITPAGKRRQKRGSRRLKHRYKLRRSRLIRVFKTLNWLPEDFPLDNPKRIKETISTEGKFS
FRISDYVPISDESYREFYREFGYPENEIEQVIEEINFRRKTKGKNKNPMIKLLPEDWVVYYL
RKKALIKPTTKEELIRIIYLFNQRRGFKSSRKDLTETAILDYDEFAKRLAEKEKYSAENYET
KFVSITKVKEVVELKTDGRKGKKRFKVILEDSRIEPYEIERKEKPDWEGKEYTFLVTQKLEK
GKFKQNKPDLPKEEDWALCTTALDNRMGSKHPGEFFFDELLKAFKEKRGYKIRQYPVNRWRY
KKELEFIWTKQCQLNPELNNLNINKEILRKLATVLYPSQSKFFGPKIKEFENSDVLHIISED
IIYYQRDLKSQKSLISECRYEKRKGIDGEIYGLKCIPKSSPLYQEFRIWQDIHNIKVIRKES
EVNGKKKINIDETQLYINENIKEKLFELFNSKDSLSEKDILELISLNIINSGIKISKKEEET
THRINLFANRKELKGNETKSRYRKVFKKLGFDGEYILNHPSKLNRLWHSDYSNDYADKEKTE
KSILSSLGWKNRNGKWEKSKNYDVFNLPLEVAKAIANLPPLKKEYGSYSALAIRKMLVVMRD
GKYWQHPDQIAKDQENTSLMLFDKNLIQLTNNQRKVLNKYLLTLAEVQKRSTLIKQKLNEIE
HNPYKLELVSDQDLEKQVLKSFLEKKNESDYLKGLKTYQAGYLIYGKHSEKDVPIVNSPDEL
GEYIRKKLPNNSLRNPIVEQVIRETIFIVRDVWKSFGIIDEIHIELGRELKNNSEERKKTSE
SQEKNFQEKERARKLLKELLNSSNFEHYDENGNKIFSSFTVNPNPDSPLDIEKFRIWKNQSG
LTDEELNKKLKDEKIPTEIEVKKYILWLTQKCRSPYTGKIIPLSKLFDSNVYEIEHIIPRSK
MKNDSTNNLVICELGVNKAKGDRLAANFISESNGKCKFGEVEYTLLKYGDYLQYCKDTFKYQ
KAKYKNLLATEPPEDFIERQINDTRYIGRKLAELLTPVVKDSKNIIFTIGSITSELKITWGL
NGVWKDILRPRFKRLESIINKKLIFQDEDDPNKYHFDLSINPQLDKEGLKRLDHRHHALDAT
IIAATTREHVRYLNSLNAADNDEEKREYFLSLCNHKIRDFKLPWENFTSEVKSKLLSCVVSY
KESKPILSDPFNKYLKWEYKNGKWQKVFAIQIKNDRWKAVRRSMFKEPIGTVWIKKIKEVSL
KEAIKIQAIWEEVKNDPVRKKKEKYIYDDYAQKVIAKIVQELGLSSSMRKQDDEKLNKFINE
AKVSAGVNKNLNTTNKTIYNLEGRFYEKIKVAEYVLYKAKRMPLNKKEYIEKLSLQKMFNDL
PNFILEKSILDNYPEILKELESDNKYIIEPHKKNNPVNRLLLEHILEYHNNPKEAFSTEGLE
KLNKKAINKIGKPIKYITRLDGDINEEEIFRGAVFETDKGSNVYFVMYENNQTKDREFLKPN
PSISVLKAIEHKNKIDFFAPNRLGFSRIILSPGDLVYVPTNDQYVLIKDNSSNETIINWDDN
EFISNRIYQVKKFTGNSCYFLKNDIASLILSYSASNGVGEFGSQNISEYSVDDPPIRIKDVC
IKIRVDRLGNVRPL
SEQ ID NO: 333 
MKHILGLDLGTNSIGWALIERNIEEKYGKIIGMGSRIVPMGAELSKFEQGQAQTKNADRRTN
RGARRLNKRYKQRRNKLIYILQKLDMLPSQIKLKEDFSDPNKIDKITILPISKKQEQLTAFD
LVSLRVKALTEKVGLEDLGKIIYKYNQLRGYAGGSLEPEKEDIFDEEQSKDKKNKSFIAFSK
IVFLGEPQEEIFKNKKLNRRAIIVETEEGNFEGSTFLENIKVGDSLELLINISASKSGDTIT
IKLPNKTNWRKKMENIENQLKEKSKEMGREFYISEFLLELLKENRWAKIRNNTILRARYESE
FEAIWNEQVKHYPFLENLDKKTLIEIVSFIFPGEKESQKKYRELGLEKGLKYIIKNQVVFYQ
RELKDQSHLISDCRYEPNEKAIAKSHPVFQEYKVWEQINKLIVNTKIEAGTNRKGEKKYKYI
DRPIPTALKEWIFEELQNKKEITFSAIFKKLKAEFDLREGIDFLNGMSPKDKLGNETKLQL
QKSLGELWDVLGLDSINRQIELWNILYNEKGNEYDLTSDRTSKVLEFINKYGNNIVDDNAEE
TAIRISKIKFARAYSSLSLKAVERILPLVRAGKYFNNDFSQQLQSKILKLLNENVEDPFAKA
AQTYLDNNQSVLSEGGVGNSIATILVYDKHTAKEYSHDELYKSYKEINLLKQGDLRNPLVEQ
IINEALVLIRDIWKNYGIKPNEIRVELARDLKNSAKERATIHKRNKDNQTINNKIKETLVKN
KKELSLANIEKVKLWEAQRHLSPYTGQPIPLSDLFDKEKYDVDHIIPISRYFDDSFTNKVIS
EKSVNQEKANRTAMEYFEVGSLKYSIFTKEQFIAHVNEYFSGVKRKNLLATSIPEDPVQRQI
KDTQYIAIRVKEELNKIVGNENVKTTTGSITDYLRNHWGLTDKFKLLLKERYEALLESEKFL
EAEYDNYKKDFDSRKKEYEEKEVLFEEQELTREEFIKEYKENYIRYKKNKLIIKGWSKRIDH
RHHAIDALIVACTEPAHIKRLNDLNKVLQDWLVEHKSEFMPNFEGSNSELLEEILSLPENER
TEIFTQIEKFRAIEMPWKGFPEQVEQKLKEIIISHKPKDKLLLQYNKAGDRQIKLRGQLHEG
TLYGISQGKEAYRIPLTKFGGSKFATEKNIQKIVSPFLSGFIANHLKEYNNKKEEAFSAEGI
MDLNNKLAQYRNEKGELKPHTPISTVKIYYKDPSKNKKKKDEEDLSLQKLDREKAFNEKLYV
KTGDNYLFAVLEGEIKTKKTSQIKRLYDIISFFDATNFLKEEFRNAPDKKTFDKDLLFRQYF
EERNKAKLLFTLKQGDFVYLPNENEEVILDKESPLYNQYWGDLKERGKNIYVVQKFSKKQIY
FIKHTIADIIKKDVEFGSQNCYETVEGRSIKENCFKLEIDRLGNIVKVIKR
SEQ ID NO: 334 
MHVEIDFPHFSRGDSHLAMNKNEILRGSSVLYRLGLDLGSNSLGWFVTHLEKRGDRHEPVAL
GPGGVRIFPDGRDPQSGTSNAVDRRMARGARKRRDRFVERRKELIAALIKYNLLPDDARERR
ALEVLDPYALRKTALTDTLPAHHVGRALFHLNQRRGFQSNRKTDSKQSEDGAIKQAASRLAT
DKGNETLGVFFADMHLRKSYEDRQTAIRAELVRLGKDHLTGNARKKIWAKVRKRLFGDEVLP
RADAPHGVRARATITGTKASYDYYPTRDMLRDEFNAIWAGQSAHHATITDEARTEIEHIIFY
QRPLKPAIVGKCTLDPATRPFKEDPEGYRAPWSHPLAQRFRILSEARNLEIRDTGKGSRRLT
KEQSDLVVAALLANREVKFDKLRTLLKLPAEARFNLESDRRAALDGDQTAARLSDKKGFNKA
WRGFPPERQIAIVARLEETEDENELIAWLEKECALDGAAAARVANTTLPDGHCRLGLRAIKK
IVPIMQDGLDEDGVAGAGYHIAAKRAGYDHAKLPTGEQLGRLPYYGQWLQDAVVGSGDARDQ
KEKQYGQFPNPTVHIGLGQLRRVVNDLIDKYGPPTEISIEFTRALKLSEQQKAERQREQRRN
QDKNKARAEELAKFGRPANPRNLLKMRLWEELAHDPLDRKCVYTGEQISIERLLSDEVDIDH
ILPVAMTLDDSPANKIICMRYANRHKRKQTPSEAFGSSPTLQGHRYNWDDIAARATGLPRNK
RWRFDANAREEFDKRGGFLARQLNETGWLARLAKQYLGAVTDPNQIWVVPGRLTSMLRGKWG
LNGLLPSDNYAGVQDKAEEFLASTDDMEFSGVKNRADHRHHAIDGLVTALTDRSLLWKMANA
YDEEHEKFVIEPPWPTMRDDLKAALEKMVVSHKPDHGIEGKLHEDSAYGFVKPLDATGLKEE
EAGNLVYRKAIESLNENEVDRIRDIQLRTIVRDHVNVEKTKGVALADALRQLQAPSDDYPQF
KHGLRHVRILKKEKGDYLVPIANRASGVAYKAYSAGENFCVEVFETAGGKWDGEAVRRFDAN
KKNAGPKIAHAPQWRDANEGAKLVMRIHKGDLIRLDHEGRARIMVVHRLDAAAGRFKLADHN
ETGNLDKRHATNNDIDPFRWLMASYNTLKKLAAVPVRVDELGRVWRVMPN
SEQ ID NO: 335 
METTLGIDLGTNSIGLALVDQEEHQILYSGVRIFPEGINKDTIGLGEKEESRNATRRAKRQM
RRQYFRKKLRKAKLLELLIAYDMCPLKPEDVRRWKNWDKQQKSTVRQFPDTPAFREWLKQNP
YELRKQAVTEDVTRPELGRILYQMIQRRGFLSSRKGKEEGKIFTGKDRMVGIDETRKNLQKQ
TLGAYLYDIAPKNGEKYRFRTERVRARYTLRDMYIREFEIIWQRQAGHLGLAHEQATRKKNI
FLEGSATNVRNSKLITHLQAKYGRGHVLIEDTRITVTFQLPLKEVLGGKIEIEEEQLKFKSN
 
ESVLFWQRPLRSQKSLLSKCVFEGRNFYDPVHQKWIIAGPTPAPLSHPEFEEFRAYQFINNI
IYGKNEHLTAIQREAVFELMCTESKDFNFEKIPKHLKLFEKFNFDDTTKVPACTTISQLRKL
FPHPVWEEKREEIWHCFYFYDDNTLLFEKLQKDYALQTNDLEKIKKIRLSESYGNVSLKAIR
RINPYLKKGYAYSTAVLLGGIRNSFGKRFEYFKEYEPEIEKAVCRILKEKNAEGEVIRKIKD
YLVHNRFGFAKNDRAFQKLYHHSQAITTQAQKERLPETGNLRNPIVQQGLNELRRTVNKLLA
TCREKYGPSFKFDHIHVEMGRELRSSKTEREKQSRQIRENEKKNEAAKVKLAEYGLKAYRDN
IQKYLLYKEIEEKGGTVCCPYTGKTLNISHTLGSDNSVQIEHIIPYSISLDDSLANKTLCDA
TFNREKGELTPYDFYQKDPSPEKWGASSWEEIEDRAFRLLPYAKAQRFIRRKPQESNEFISR
QLNDTRYISKKAVEYLSAICSDVKAFPGQLTAELRHLWGLNNILQSAPDITFPLPVSATENH
REYYVITNEQNEVIRLFPKQGETPRTEKGELLLTGEVERKVFRCKGMQEFQTDVSDGKYWRR
IKLSSSVTWSPLFAPKPISADGQIVLKGRIEKGVFVCNQLKQKLKTGLPDGSYWISLPVISQ
TFKEGESVNNSKLTSQQVQLFGRVREGIFRCHNYQCPASGADGNFWCTLDTDTAQPAFTPIK
NAPPGVGGGQIILTGDVDDKGIFHADDDLHYELPASLPKGKYYGIFTVESCDPTLIPIELSA
PKTSKGENLIEGNIWVDEHTGEVRFDPKKNREDQRHHAIDAIVIALSSQSLFQRLSTYNARR
ENKKRGLDSTEHFPSPWPGFAQDVRQSVVPLLVSYKQNPKTLCKISKTLYKDGKKIHSCGNA
VRGQLHKETVYGQRTAPGATEKSYHIRKDIRELKTSKHIGKVVDITIRQMLLKHLQENYHID
ITQEFNIPSNAFFKEGVYRIFLPNKHGEPVPIKKIRMKEELGNAERLKDNINQYVNPRNNHH
VMIYQDADGNLKEEIVSFWSVIERQNQGQPIYQLPREGRNIVSILQINDTFLIGLKEEEPEV
YRNDLSTLSKHLYRVQKLSGMYYTFRHHLASTLNNEREEFRIQSLEAWKRANPVKVQIDEIG
RITFLNGPLC
SEQ ID NO: 336 
MESSQILSPIGIDLGGKFTGVCLSHLEAFAELPNHANTKYSVILIDHNNFQLSQAQRRATRH
RVRNKKRNQFVKRVALQLFQHILSRDLNAKEETALCHYLNNRGYTYVDTDLDEYIKDETTIN
LLKELLPSESEHNFIDWFLQKMQSSEFRKILVSKVEEKKDDKELKNAVKNIKNFITGFEKNS
VEGHRHRKVYFENIKSDITKDNQLDSIKKKIPSVCLSNLLGHLSNLQWKNLHRYLAKNPKQF
DEQTFGNEFLRMLKNFRHLKGSQESLAVRNLIQQLEQSQDYISILEKTPPEITIPPYEARTN
TGMEKDQSLLLNPEKLNNLYPNWRNLIPGIIDAHPFLEKDLEHTKLRDRKRIISPSKQDEKR
DSYILQRYLDLNKKIDKFKIKKQLSFLGQGKQLPANLIETQKEMETHFNSSLVSVLIQIASA
YNKEREDAAQGIWFDNAFSLCELSNINPPRKQKILPLLVGAILSEDFINNKDKWAKFKIFWN
THKIGRTSLKSKCKEIEEARKNSGNAFKIDYEEALNHPEHSNNKALIKIIQTIPDIIQAIQS
HLGHNDSQALIYHNPFSLSQLYTILETKRDGFHKNCVAVTCENYWRSQKTEIDPEISYASRL
PADSVRPFDGVLARMMQRLAYEIAMAKWEQIKHIPDNSSLLIPIYLEQNRFEFEESFKKIKG
SSSDKTLEQAIEKQNIQWEEKFQRIINASMNICPYKGASIGGQGEIDHIYPRSLSKKHFGVI
FNSEVNLIYCSSQGNREKKEEHYLLEHLSPLYLKHQFGTDNVSDIKNFISQNVANIKKYISF
HLLTPEQQKAARHALFLDYDDEAFKTITKFLMSQQKARVNGTQKFLGKQIMEFLSTLADSKQ
LQLEFSIKQITAEEVHDHRELLSKQEPKLVKSRQQSFPSHAIDATLTMSIGLKEFPQFSQEL
DNSWFINHLMPDEVHLNPVRSKEKYNKPNISSTPLFKDSLYAERFIPVWVKGETFAIGFSEK
DLFEIKPSNKEKLFTLLKTYSTKNPGESLQELQAKSKAKWLYFPINKTLALEFLHHYFHKEI
VTPDDTTVCHFINSLRYYTKKESITVKILKEPMPVLSVKFESSKKNVLGSFKHTIALPATKD
WERLFNHPNFLALKANPAPNPKEFNEFIRKYFLSDNNPNSDIPNNGHNIKPQKHKAVRKVFS
LPVIPGNAGTMMRIRRKDNKGQPLYQLQTIDDTPSMGIQINEDRLVKQEVLMDAYKTRNLST
IDGINNSEGQAYATFDNWLTLPVSTFKPEIIKLEMKPHSKTRRYIRITQSLADFIKTIDEAL
MIKPSDSIDDPLNMPNEIVCKNKLFGNELKPRDGKMKIVSTGKIVTYEFESDSTPQWIQTLY
VTQLKKQP
SEQ ID NO: 337 
MKKIVGLDLGTNSIGWALINAYINKEHLYGIEACGSRIIPMDAAILGNFDKGNSISQTADRT
SYRGIRRLRERHLLRRERLHRILDLLGFLPKHYSDSLNRYGKFLNDIECKLPWVKDETGSYK
FIFQESFKEMLANFTEHHPILIANNKKVPYDWTIYYLRKKALTQKISKEELAWILLNFNQKR
GYYQLRGEEEETPNKLVEYYSLKVEKVEDSGERKGKDTWYNVHLENGMIYRRTSNIPLDWEG
 
KTKEFIVTTDLEADGSPKKDKEGNIKRSFRAPKDDDWTLIKKKTEADIDKIKMTVGAYIYDT
LLQKPDQKIRGKLVRTIERKYYKNELYQILKTQSEFHEELRDKQLYIACLNELYPNNEPRRN
SISTRDFCHLFIEDIIFYQRPLKSKKSLIDNCPYEENRYIDKESGEIKHASIKCIAKSHPLY
QEFRLWQFIVNLRIYRKETDVDVTQELLPTEADYVTLFEWLNEKKEIDQKAFFKYPPFGFKK
TTSNYRWNYVEDKPYPCNETHAQIIARLGKAHIPKAFLSKEKEETLWHILYSIEDKQEIEKA
LHSFANKNNLSEEFIEQFKNFPPFKKEYGSYSAKAIKKLLPLMRMGKYWSIENIDNGTRIRI
NKIIDGEYDENIRERVRQKAINLTDITHFRALPLWLACYLVYDRHSEVKDIVKWKTPKDIDL
YLKSFKQHSLRNPIVEQVITETLRTVRDIWQQVGHIDEIHIELGREMKNPADKRARMSQQMI
KNENTNLRIKALLTEFLNPEFGIENVRPYSPSQQDLLRIYEEGVLNSILELPEDIGIILGKF
NQTDTLKRPTRSEILRYKLWLEQKYRSPYTGEMIPLSKLFTPAYEIEHIIPQSRYFDDSLSN
KVICESEINKLKDRSLGYEFIKNHHGEKVELAFDKPVEVLSVEAYEKLVHESYSHNRSKMKK
LLMEDIPDQFIERQLNDSRYISKVVKSLLSNIVREENEQEAISKNVIPCTGGITDRLKKDWG
INDVWNKIVLPRFIRLNELTESTRFTSINTNNTMIPSMPLELQKGFNKKRIDHRHHAMDAII
IACANRNIVNYLNNVSASKNTKITRRDLQTLLCHKDKTDNNGNYKWVIDKPWETFTQDTLTA
LQKITVSFKQNLRVINKTTNHYQHYENGKKIVSNQSKGDSWAIRKSMHKETVHGEVNLRMIK
TVSFNEALKKPQAIVEMDLKKKILAMLELGYDTKRIKNYFEENKDTWQDINPSKIKVYYFTK
ETKDRYFAVRKPIDTSFDKKKIKESITDTGIQQIMLRHLETKDNDPTLAFSPDGIDEMNRNI
LILNKGKKHQPIYKVRVYEKAEKFTVGQKGNKRTKFVEAAKGTNLFFAIYETEEIDKDTKKV
IRKRSYSTIPLNVVIERQKQGLSSAPEDENGNLPKYILSPNDLVYVPTQEEINKGEVVMPID
RDRIYKMVDSSGITANFIPASTANLIFALPKATAEIYCNGENCIQNEYGIGSPQSKNQKAIT
GEMVKEICFPIKVDRLGNIIQVGSCILTN
SEQ ID NO: 338 
MSRSLTFSFDIGYASIGWAVIASASHDDADPSVCGCGTVLFPKDDCQAFKRREYRRLRRNIR
SRRVRIERIGRLLVQAQIITPEMKETSGHPAPFYLASEALKGHRTLAPIELWHVLRWYAHNR
GYDNNASWSNSLSEDGGNGEDTERVKHAQDLMDKHGTATMAETICRELKLEEGKADAPMEVS
TPAYKNLNTAFPRLIVEKEVRRILELSAPLIPGLTAEIIELIAQHHPLTTEQRGVLLQHGIK
LARRYRGSLLFGQLIPRFDNRIISRCPVTWAQVYEAELKKGNSEQSARERAEKLSKVPTANC
PEFYEYRMARILCNIRADGEPLSAEIRRELMNQARQEGKLTKASLEKAISSRLGKETETNVS
NYFTLHPDSEEALYLNPAVEVLQRSGIGQILSPSVYRIAANRLRRGKSVTPNYLLNLLKSRG
ESGEALEKKIEKESKKKEADYADTPLKPKYATGRAPYARTVLKKVVEEILDGEDPTRPARGE
AHPDGELKAHDGCLYCLLDTDSSVNQHQKERRLDTMTNNHLVRHRMLILDRLLKDLIQDFAD
GQKDRISRVCVEVGKELTTFSAMDSKKIQRELTLRQKSHTDAVNRLKRKLPGKALSANLIRK
CRIAMDMNWTCPFTGATYGDHELENLELEHIVPHSFRQSNALSSLVLTWPGVNRMKGQRTGY
DFVEQEQENPVPDKPNLHICSLNNYRELVEKLDDKKGHEDDRRRKKKRKALLMVRGLSHKHQ
SQNHEAMKEIGMTEGMMTQSSHLMKLACKSIKTSLPDAHIDMIPGAVTAEVRKAWDVFGVFK
ELCPEAADPDSGKILKENLRSLTHLHHALDACVLGLIPYIIPAHHNGLLRRVLAMRRIPEKL
IPQVRPVANQRHYVLNDDGRMMLRDLSASLKENIREQLMEQRVIQHVPADMGGALLKETMQR
VLSVDGSGEDAMVSLSKKKDGKKEKNQVKASKLVGVFPEGPSKLKALKAAIEIDGNYGVALD
PKPVVIRHIKVFKRIMALKEQNGGKPVRILKKGMLIHLTSSKDPKHAGVWRIESIQDSKGGV
KLDLQRAHCAVPKNKTHECNWREVDLISLLKKYQMKRYPTSYTGTPR
SEQ ID NO: 339 
MTQKVLGLDLGTNSIGSAVRNLDLSDDLQWQLEFFSSDIFRSSVNKESNGREYSLAAQRSAH
RRSRGLNEVRRRRLWATLNLLIKHGFCPMSSESLMRWCTYDKRKGLFREYPIDDKDFNAWIL
LDFNGDGRPDYSSPYQLRRELVTRQFDFEQPIERYKLGRALYHIAQHRGFKSSKGETLSQQE
TNSKPSSTDEIPDVAGAMKASEEKLSKGLSTYMKEHNLLTVGAAFAQLEDEGVRVRNNNDYR
AIRSQFQHEIETIFKFQQGLSVESELYERLISEKKNVGTIFYKRPLRSQRGNVGKCTLERSK
PRCAIGHPLFEKFRAWTLINNIKVRMSVDTLDEQLPMKLRLDLYNECFLAFVRTEFKFEDIR
KYLEKRLGIHFSYNDKTINYKDSTSVAGCPITARFRKMLGEEWESFRVEGQKERQAHSKNNI
SFHRVSYSIEDIWHFCYDAEEPEAVLAFAQETLRLERKKAEELVRIWSAMPQGYAMLSQKAI
 
RNINKILMLGLKYSDAVILAKVPELVDVSDEELLSIAKDYYLVEAQVNYDKRINSIVNGLIA
KYKSVSEEYRFADHNYEYLLDESDEKDIIRQIENSLGARRWSLMDANEQTDILQKVRDRYQD
FFRSHERKFVESPKLGESFENYLTKKFPMVEREQWKKLYHPSQITIYRPVSVGKDRSVLRLG
NPDIGAIKNPTVLRVLNTLRRRVNQLLDDGVISPDETRVVVETARELNDANRKWALDTYNRI
RHDENEKIKKILEEFYPKRDGISTDDIDKARYVIDQREVDYFTGSKTYNKDIKKYKFWLEQG
GQCMYTGRTINLSNLFDPNAFDIEHTIPESLSFDSSDMNLTLCDAHYNRFIKKNHIPTDMPN
YDKAITIDGKEYPAITSQLQRWVERVERLNRNVEYWKGQARRAQNKDRKDQCMREMHLWKME
LEYWKKKLERFTVTEVTDGFKNSQLVDTRVITRHAVLYLKSIFPHVDVQRGDVTAKFRKILG
IQSVDEKKDRSLHSHHAIDATTLTIIPVSAKRDRMLELFAKIEEINKMLSFSGSEDRTGLIQ
ELEGLKNKLQMEVKVCRIGHNVSEIGTFINDNIIVNHHIKNQALTPVRRRLRKKGYIVGGVD
NPRWQTGDALRGEIHKASYYGAITQFAKDDEGKVLMKEGRPQVNPTIKFVIRRELKYKKSAA
DSGFASWDDLGKAIVDKELFALMKGQFPAETSFKDACEQGIYMIKKGKNGMPDIKLHHIRHV
RCEAPQSGLKIKEQTYKSEKEYKRYFYAAVGDLYAMCCYTNGKIREFRIYSLYDVSCHRKSD
IEDIPEFITDKKGNRLMLDYKLRTGDMILLYKDNPAELYDLDNVNLSRRLYKINRFESQSNL
VLMTHHLSTSKERGRSLGKTVDYQNLPESIRSSVKSLNFLIMGENRDFVIKNGKIIFNHR
SEQ ID NO: 340 
MLVSPISVDLGGKNTGFFSFTDSLDNSQSGTVIYDESFVLSQVGRRSKRHSKRNNLRNKLVK
RLFLLILQEHHGLSIDVLPDEIRGLFNKRGYTYAGFELDEKKKDALESDTLKEFLSEKLQSI
DRDSDVEDFLNQIASNAESFKDYKKGFEAVFASATHSPNKKLELKDELKSEYGENAKELLAG
LRVTKEILDEFDKQENQGNLPRAKYFEELGEYIATNEKVKSFFDSNSLKLTDMTKLIGNISN
YQLKELRRYFNDKEMEKGDIWIPNKLHKITERFVRSWHPKNDADRQRRAELMKDLKSKEIME
LLTTTEPVMTIPPYDDMNNRGAVKCQTLRLNEEYLDKHLPNWRDIAKRLNHGKFNDDLADST
VKGYSEDSTLLHRLLDTSKEIDIYELRGKKPNELLVKTLGQSDANRLYGFAQNYYELIRQKV
RAGIWVPVKNKDDSLNLEDNSNMLKRCNHNPPHKKNQIHNLVAGILGVKLDEAKFAEFEKEL
WSAKVGNKKLSAYCKNIEELRKTHGNTFKIDIEELRKKDPAELSKEEKAKLRLTDDVILNEW
SQKIANFFDIDDKHRQRFNNLFSMAQLHTVIDTPRSGFSSTCKRCTAENRFRSETAFYNDET
GEFHKKATATCQRLPADTQRPFSGKIERYIDKLGYELAKIKAKELEGMEAKEIKVPIILEQN
AFEYEESLRKSKTGSNDRVINSKKDRDGKKLAKAKENAEDRLKDKDKRIKAFSSGICPYCGD
TIGDDGEIDHILPRSHTLKIYGTVFNPEGNLIYVHQKCNQAKADSIYKLSDIKAGVSAQWIE
EQVANIKGYKTFSVLSAEQQKAFRYALFLQNDNEAYKKVVDWLRTDQSARVNGTQKYLAKKI
QEKLTKMLPNKHLSFEFILADATEVSELRRQYARQNPLLAKAEKQAPSSHAIDAVMAFVARY
QKVFKDGTPPNADEVAKLAMLDSWNPASNEPLTKGLSTNQKIEKMIKSGDYGQKNMREVFGK
SIFGENAIGERYKPIVVQEGGYYIGYPATVKKGYELKNCKVVTSKNDIAKLEKIIKNQDLIS
LKENQYIKIFSINKQTISELSNRYFNMNYKNLVERDKEIVGLLEFIVENCRYYTKKVDVKFA
PKYIHETKYPFYDDWRRFDEAWRYLQENQNKTSSKDRFVIDKSSLNEYYQPDKNEYKLDVDT
QPIWDDFCRWYFLDRYKTANDKKSIRIKARKTFSLLAESGVQGKVFRAKRKIPTGYAYQALP
MDNNVIAGDYANILLEANSKTLSLVPKSGISIEKQLDKKLDVIKKTDVRGLAIDNNSFFNAD
FDTHGIRLIVENTSVKVGNFPISAIDKSAKRMIFRALFEKEKGKRKKKTTISFKESGPVQDY
LKVFLKKIVKIQLRTDGSISNIVVRKNAADFTLSFRSEHIQKLLK
SEQ ID NO: 341 
MAYRLGLDIGITSVGWAVVALEKDESGLKPVRIQDLGVRIFDKAEDSKTGASLALPRREARS
ARRRTRRRRHRLWRVKRLLEQHGILSMEQIEALYAQRTSSPDVYALRVAGLDRCLIAEEIAR
VLIHIAHRRGFQSNRKSEIKDSDAGKLLKAVQENENLMQSKGYRTVAEMLVSEATKTDAEGK
LVHGKKHGYVSNVRNKAGEYRHTVSRQAIVDEVRKIFAAQRALGNDVMSEELEDSYLKILCS
QRNFDDGPGGDSPYGHGSVSPDGVRQSIYERMVGSCTFETGEKRAPRSSYSFERFQLLTKVV
NLRIYRQQEDGGRYPCELTQTERARVIDCAYEQTKITYGKLRKLLDMKDTESFAGLTYGLNR
SRNKTEDTVFVEMKFYHEVRKALQRAGVFIQDLSIETLDQIGWILSVWKSDDNRRKKLSTLG
LSDNVIEELLPLNGSKFGHLSLKAIRKILPFLEDGYSYDVACELAGYQFQGKTEYVKQRLLP
PLGEGEVTNPVVRRALSQAIKVVNAVIRKHGSPESIHIELARELSKNLDERRKIEKAQKENQ
 
KNNEQIKDEIREILGSAHVTGRDIVKYKLFKQQQEFCMYSGEKLDVTRLFEPGYAEVDHIIP
YGISFDDSYDNKVLVKTEQNRQKGNRTPLEYLRDKPEQKAKFIALVESIPLSQKKKNHLLMD
KRAIDLEQEGFRERNLSDTRYITRALMNHIQAWLLFDETASTRSKRVVCVNGAVTAYMRARW
GLTKDRDAGDKHHAADAVVVACIGDSLIQRVTKYDKFKRNALADRNRYVQQVSKSEGITQYV
DKETGEVFTWESFDERKFLPNEPLEPWPFFRDELLARLSDDPSKNIRAIGLLTYSETEQIDP
IFVSRMPTRKVTGAAHKETIRSPRIVKVDDNKGTEIQVVVSKVALTELKLTKDGEIKDYFRP
EDDPRLYNTLRERLVQFGGDAKAAFKEPVYKISKDGSVRTPVRKVKIQEKLTLGVPVHGGRG
IAENGGMVRIDVFAKGGKYYFVPIYVADVLKRELPNRLATAHKPYSEWRVVDDSYQFKFSLY
PNDAVMIKPSREVDITYKDRKEPVGCRIMYFVSANIASASISLRTHDNSGELEGLGIQGLEV
FEKYVVGPLGDTHPVYKERRMPFRVERKMN
SEQ ID NO: 342 
MPVLSPLSPNAAQGRRRWSLALDIGEGSIGWAVAEVDAEGRVLQLTGTGVTLFPSAWSNENG
TYVAHGAADRAVRGQQQRHDSRRRRLAGLARLCAPVLERSPEDLKDLTRTPPKADPRAIFFL
RADAARRPLDGPELFRVLHHMAAHRGIRLAELQEVDPPPESDADDAAPAATEDEDGTRRAAA
DERAFRRLMAEHMHRHGTQPTCGEIMAGRLRETPAGAQPVTRARDGLRVGGGVAVPTRALIE
QEFDAIRAIQAPRHPDLPWDSLRRLVLDQAPIAVPPATPCLFLEELRRRGETFQGRTITREA
IDRGLTVDPLIQALRIRETVGNLRLHERITEPDGRQRYVPRAMPELGLSHGELTAPERDTLV
RALMHDPDGLAAKDGRIPYTRLRKLIGYDNSPVCFAQERDTSGGGITVNPTDPLMARWIDGW
VDLPLKARSLYVRDVVARGADSAALARLLAEGAHGVPPVAAAAVPAATAAILESDIMQPGRY
SVCPWAAEAILDAWANAPTEGFYDVTRGLFGFAPGEIVLEDLRRARGALLAHLPRTMAAART
PNRAAQQRGPLPAYESVIPSQLITSLRRAHKGRAADWSAADPEERNPFLRTWTGNAATDHIL
NQVRKTANEVITKYGNRRGWDPLPSRITVELAREAKHGVIRRNEIAKENRENEGRRKKESAA
LDTFCQDNTVSWQAGGLPKERAALRLRLAQRQEFFCPYCAERPKLRATDLFSPAETEIDHVI
ERRMGGDGPDNLVLAHKDCNNAKGKKTPHEHAGDLLDSPALAALWQGWRKENADRLKGKGHK
ARTPREDKDFMDRVGWRFEEDARAKAEENQERRGRRMLHDTARATRLARLYLAAAVMPEDPA
EIGAPPVETPPSPEDPTGYTAIYRTISRVQPVNGSVTHMLRQRLLQRDKNRDYQTHHAEDAC
LLLLAGPAVVQAFNTEAAQHGADAPDDRPVDLMPTSDAYHQQRRARALGRVPLATVDAALAD
IVMPESDRQDPETGRVHWRLTRAGRGLKRRIDDLTRNCVILSRPRRPSETGTPGALHNATHY
GRREITVDGRTDTVVTQRMNARDLVALLDNAKIVPAARLDAAAPGDTILKEICTEIADRHDR
VVDPEGTHARRWISARLAALVPAHAEAVARDIAELADLDALADADRTPEQEARRSALRQSPY
LGRAISAKKADGRARAREQEILTRALLDPHWGPRGLRHLIMREARAPSLVRIRANKTDAFGR
PVPDAAVWVKTDGNAVSQLWRLTSVVTDDGRRIPLPKPIEKRIEISNLEYARLNGLDEGAGV
TGNNAPPRPLRQDIDRLTPLWRDHGTAPGGYLGTAVGELEDKARSALRGKAMRQTLTDAGIT
AEAGWRLDSEGAVCDLEVAKGDTVKKDGKTYKVGVITQGIFGMPVDAAGSAPRTPEDCEKFE
EQYGIKPWKAKGIPLA
SEQ ID NO: 343 
MNYTEKEKLFMKYILALDIGIASVGWAILDKESETVIEAGSNIFPEASAADNQLRRDMRGAK
RNNRRLKTRINDFIKLWENNNLSIPQFKSTEIVGLKVRAITEEITLDELYLILYSYLKHRGI
SYLEDALDDTVSGSSAYANGLKLNAKELETHYPCEIQQERLNTIGKYRGQSQIINENGEVLD
LSNVFTIGAYRKEIQRVFEIQKKYHPELTDEFCDGYMLIFNRKRKYYEGPGNEKSRTDYGRF
TTKLDANGNYITEDNIFEKLIGKCSVYPDELRAAAASYTAQEYNVLNDLNNLTINGRKLEEN
EKHEIVERIKSSNTINMRKIISDCMGENIDDFAGARIDKSGKEIFHKFEVYNKMRKALLEIG
IDISNYSREELDEIGYIMTINTDKEAMMEAFQKSWIDLSDDVKQCLINMRKTNGALFNKWQS
FSLKIMNELIPEMYAQPKEQMTLLTEMGVTKGTQEEFAGLKYIPVDVVSEDIFNPVVRRSVR
ISFKILNAVLKKYKALDTIVIEMPRDRNSEEQKKRINDSQKLNEKEMEYIEKKLAVTYGIKL
SPSDFSSQKQLSLKLKLWNEQDGICLYSGKTIDPNDIINNPQLFEIDHIIPRSISFDDARSN
KVLVYRSENQKKGNQTPYYYLTHSHSEWSFEQYKATVMNLSKKKEYAISRKKIQNLLYSEDI
TKMDVLKGFINRNINDTSYASRLVLNTIQNFFMANEADTKVKVIKGSYTHQMRCNLKLDKNR
DESYSHHAVDAMLIGYSELGYEAYHKLQGEFIDFETGEILRKDMWDENMSDEVYADYLYGKK
 
WANIRNEVVKAEKNVKYWHYVMRKSNRGLCNQTIRGTREYDGKQYKINKLDIRTKEGIKVFA
KLAFSKKDSDRERLLVYLNDRRTFDDLCKIYEDYSDAANPFVQYEKETGDIIRKYSKKHNGP
RIDKLKYKDGEVGACIDISHKYGFEKGSKKVILESLVPYRMDVYYKEENHSYYLVGVKQSDI
KFEKGRNVIDEEAYARILVNEKMIQPGQSRADLENLGFKFKLSFYKNDIIEYEKDGKIYTER
LVSRTMPKQRNYIETKPIDKAKFEKQNLVGLGKTKFIKKYRYDILGNKYSCSEEKFTSFC
SEQ ID NO: 344 
MLRLYCANNLVLNNVQNLWKYLLLLIFDKKIIFLFKIKVILIRRYMENNNKEKIVIGFDLGV
ASVGWSIVNAETKEVIDLGVRLFSEPEKADYRRAKRTTRRLLRRKKFKREKFHKLILKNAEI
FGLQSRNEILNVYKDQSSKYRNILKLKINALKEEIKPSELVWILRDYLQNRGYFYKNEKLTD
EFVSNSFPSKKLHEHYEKYGFFRGSVKLDNKLDNKKDKAKEKDEEEESDAKKESEELIFSNK
QWINEIVKVFENQSYLTESFKEEYLKLFNYVRPFNKGPGSKNSRTAYGVFSTDIDPETNKFK
DYSNIWDKTIGKCSLFEEEIRAPKNLPSALIFNLQNEICTIKNEFTEFKNWWLNAEQKSEIL
KFVFTELFNWKDKKYSDKKFNKNLQDKIKKYLLNFALENFNLNEEILKNRDLENDTVLGLKG
VKYYEKSNATADAALEFSSLKPLYVFIKFLKEKKLDLNYLLGLENTEILYFLDSIYLAISYS
SDLKERNEWFKKLLKELYPKIKNNNLEIIENVEDIFEITDQEKFESFSKTHSLSREAFNHII
PLLLSNNEGKNYESLKHSNEELKKRTEKAELKAQQNQKYLKDNFLKEALVPLSVKTSVLQAI
KIFNQIIKNFGKKYEISQVVIEMARELTKPNLEKLLNNATNSNIKILKEKLDQTEKFDDFTK
KKFIDKIENSVVFRNKLFLWFEQDRKDPYTQLDIKINEIEDETEIDHVIPYSKSADDSWFNK
LLVKKSTNQLKKNKTVWEYYQNESDPEAKWNKFVAWAKRIYLVQKSDKESKDNSEKNSIFKN
KKPNLKFKNITKKLFDPYKDLGFLARNLNDTRYATKVFRDQLNNYSKHHSKDDENKLFKVVC
MNGSITSFLRKSMWRKNEEQVYRFNFWKKDRDQFFHHAVDASIIAIFSLLTKTLYNKLRVYE
SYDVQRREDGVYLINKETGEVKKADKDYWKDQHNFLKIRENAIEIKNVLNNVDFQNQVRYSR
KANTKLNTQLFNETLYGVKEFENNFYKLEKVNLFSRKDLRKFILEDLNEESEKNKKNENGSR
KRILTEKYIVDEILQILENEEFKDSKSDINALNKYMDSLPSKFSEFFSQDFINKCKKENSLI
LTFDAIKHNDPKKVIKIKNLKFFREDATLKNKQAVHKDSKNQIKSFYESYKCVGFIWLKNKN
DLEESIFVPINSRVIHFGDKDKDIFDFDSYNKEKLLNEINLKRPENKKFNSINEIEFVKFVK
PGALLLNFENQQIYYISTLESSSLRAKIKLLNKMDKGKAVSMKKITNPDEYKIIEHVNPLGI
NLNWTKKLENNN
SEQ ID NO: 345 
MLMSKHVLGLDLGVGSIGWCLIALDAQGDPAEILGMGSRVVPLNNATKAIEAFNAGAAFTAS
QERTARRTMRRGFARYQLRRYRLRRELEKVGMLPDAALIQLPLLELWELRERAATAGRRLTL
PELGRVLCHINQKRGYRHVKSDAAAIVGDEGEKKKDSNSAYLAGIRANDEKLQAEHKTVGQY
FAEQLRQNQSESPTGGISYRIKDQIFSRQCYIDEYDQIMAVQRVHYPDILTDEFIRMLRDEV
IFMQRPLKSCKHLVSLCEFEKQERVMRVQQDDGKGGWQLVERRVKFGPKVAPKSSPLFQLCC
IYEAVNNIRLTRPNGSPCDITPEERAKIVAHLQSSASLSFAALKKLLKEKALIADQLTSKSG
LKGNSTRVALASALQPYPQYHHLLDMELETRMMTVQLTDEETGEVTEREVAVVTDSYVRKPL
YRLWHILYSIEEREAMRRALITQLGMKEEDLDGGLLDQLYRLDFVKPGYGNKSAKFICKLLP
QLQQGLGYSEACAAVGYRHSNSPTSEEITERTLLEKIPLLQRNELRQPLVEKILNQMINLVN
ALKAEYGIDEVRVELARELKMSREERERMARNNKDREERNKGVAAKIRECGLYPTKPRIQKY
MLWKEAGRQCLYCGRSIEEEQCLREGGMEVEHIIPKSVLYDDSYGNKTCACRRCNKEKGNRT
ALEYIRAKGREAEYMKRINDLLKEKKISYSKHQRLRWLKEDIPSDFLERQLRLTQYISRQAM
AILQQGIRRVSASEGGVTARLRSLWGYGKILHTLNLDRYDSMGETERVSREGEATEELHITN
WSKRMDHRHHAIDALVVACTRQSYIQRLNRLSSEFGREDKKKEDQEAQEQQATETGRLSNLE
RWLTQRPHFSVRTVSDKVAEILISYRPGQRVVTRGRNIYRKKMADGREVSCVQRGVLVPRGE
LMEASFYGKILSQGRVRIVKRYPLHDLKGEVVDPHLRELITTYNQELKSREKGAPIPPLCLD
KDKKQEVRSVRCYAKTLSLDKAIPMCFDEKGEPTAFVKSASNHHLALYRTPKGKLVESIVTF
WDAVDRARYGIPLVITHPREVMEQVLQRGDIPEQVLSLLPPSDWVFVDSLQQDEMVVIGLSD
EELQRALEAQNYRKISEHLYRVQKMSSSYYVFRYHLETSVADDKNTSGRIPKFHRVQSLKAY
EERNIRKVRVDLLGRISLL
SEQ ID NO: 346 
MSDLVLGLDIGIGSVGVGILNKVTGEIIHKNSRIFPAAQAENNLVRRTNRQGRRLARRKKHR
RVRLNRLFEESGLITDFTKISINLNPYQLRVKGLTDELSNEELFIALKNMVKHRGISYLDDA
SDDGNSSVGDYAQIVKENSKQLETKTPGQIQLERYQTYGQLRGDFTVEKDGKKHRLINVFPT
SAYRSEALRILQTQQEFNPQITDEFINRYLEILTGKRKYYHGPGNEKSRTDYGRYRTSGETL
DNIFGILIGKCTFYPDEFRAAKASYTAQEFNLLNDLNNLTVPTETKKLSKEQKNQIINYVKN
EKAMGPAKLFKYIAKLLSCDVADIKGYRIDKSGKAEIHTFEAYRKMKTLETLDIEQMDRETL
DKLAYVLTLNTEREGIQEALEHEFADGSFSQKQVDELVQFRKANSSIFGKGWHNFSVKLMME
LIPELYETSEEQMTILTRLGKQKTTSSSNKTKYIDEKLLTEEIYNPVVAKSVRQAIKIVNAA
IKEYGDFDNIVIEMARETNEDDEKKAIQKIQKANKDEKDAAMLKAANQYNGKAELPHSVFHG
HKQLATKIRLWHQQGERCLYTGKTISIHDLINNSNQFEVDHILPLSITFDDSLANKVLVYAT
ANQEKGQRTPYQALDSMDDAWSFRELKAFVRESKTLSNKKKEYLLTEEDISKFDVRKKFIER
NLVDTRYASRVVLNALQEHFRAHKIDTKVSVVRGQFTSQLRRHWGIEKTRDTYHHHAVDALI
IAASSQLNLWKKQKNTLVSYSEDQLLDIETGELISDDEYKESVFKAPYQHFVDTLKSKEFED
SILFSYQVDSKFNRKISDATIYATRQAKVGKDKADETYVLGKIKDIYTQDGYDAFMKIYKKD
KSKFLMYRHDPQTFEKVIEPILENYPNKQINEKGKEVPCNPFLKYKEEHGYIRKYSKKGNGP
EIKSLKYYDSKLGNHIDITPKDSNNKVVLQSVSPWRADVYFNKTTGKYEILGLKYADLQFEK
GTGTYKISQEKYNDIKKKEGVDSDSEFKFTLYKNDLLLVKDTETKEQQLFRFLSRTMPKQKH
YVELKPYDKQKFEGGEALIKVLGNVANSGQCKKGLGKSNISIYKVRTDVLGNQHIIKNEGDK
PKLDF
SEQ ID NO: 347 
MNAEHGKEGLLIMEENFQYRIGLDIGITSVGWAVLQNNSQDEPVRITDLGVRIFDVAENPKN
GDALAAPRRDARTTRRRLRRRRHRLERIKFLLQENGLIEMDSFMERYYKGNLPDVYQLRYEG
LDRKLKDEELAQVLIHIAKHRGFRSTRKAETKEKEGGAVLKATTENQKIMQEKGYRTVGEML
YLDEAFHTECLWNEKGYVLTPRNRPDDYKHTILRSMLVEEVHAIFAAQRAHGNQKATEGLEE
AYVEIMTSQRSFDMGPGLQPDGKPSPYAMEGFGDRVGKCTFEKDEYRAPKATYTAELFVALQ
KINHTKLIDEFGTGRFFSEEERKTIIGLLLSSKELKYGTIRKKLNIDPSLKFNSLNYSAKKE
GETEEERVLDTEKAKFASMFWTYEYSKCLKDRTEEMPVGEKADLFDRIGEILTAYKNDDSRS
SRLKELGLSGEEIDGLLDLSPAKYQRVSLKAMRKMQPYLEDGLIYDKACEAAGYDFRALNDG
NKKHLLKGEEINAIVNDITNPVVKRSVSQTIKVINAIIQKYGSPQAVNIELAREMSKNFQDR
TNLEKEMKKRQQENERAKQQIIELGKQNPTGQDILKYRLWNDQGGYCLYSGKKIPLEELFDG
GYDIDHILPYSITFDDSYRNKVLVTAQENRQKGNRTPYEYFGADEKRWEDYEASVRLLVRDY
KKQQKLLKKNFTEEERKEFKERNLNDTKYITRVVYNMIRQNLELEPFNHPEKKKQVWAVNGA
VTSYLRKRWGLMQKDRSTDRHHAMDAVVIACCTDGMIHKISRYMQGRELAYSRNFKFPDEET
GEILNRDNFTREQWDEKFGVKVPLPWNSFRDELDIRLLNEDPKNFLLTHADVQRELDYPGWM
YGEEESPIEEGRYINYIRPLFVSRMPNHKVTGSAHDATIRSARDYETRGVVITKVPLTDLKL
NKDNEIEGYYDKDSDRLLYQALVRQLLLHGNDGKKAFAEDFHKPKADGTEGPVVRKVKIEKK
QTSGVMVRGGTGIAANGEMVRIDVFRENGKYYFVPVYTADVVRKVLPNRAATHTKPYSEWRV
MDDANFVFSLYSRDLIHVKSKKDIKTNLVNGGLLLQKEIFAYYTGADIATASIAGFANDSNF
KFRGLGIQSLEIFEKCQVDILGNISVVRHENRQEFH
SEQ ID NO: 348 
MRVLGLDAGIASLGWALIEIEESNRGELSQGTIIGAGTWMFDAPEEKTQAGAKLKSEQRRTF
RGQRRVVRRRRQRMNEVRRILHSHGLLPSSDRDALKQPGLDPWRIRAEALDRLLGPVELAVA
LGHIARHRGFKSNSKGAKTNDPADDTSKMKRAVNETREKLARFGSAAKMLVEDESFVLRQTP
TKNGASEIVRRFRNREGDYSRSLLRDDLAAEMRALFTAQARFQSAIATADLQTAFTKAAFFQ
RPLQDSEKLVGPCPFEVDEKRAPKRGYSFELFRFLSRLNHVTLRDGKQERTLTRDELALAAA
DFGAAAKVSFTALRKKLKLPETTVFVGVKADEESKLDVVARSGKAAEGTARLRSVIVDALGE
LAWGALLCSPEKLDKIAEVISFRSDIGRISEGLAQAGCNAPLVDALTAAASDGRFDPFTGAG
 
HISSKAARNILSGLRQGMTYDKACCAADYDHTASRERGAFDVGGHGREALKRILQEERISRE
LVGSPTARKALIESIKQVKAIVERYGVPDRIHVELARDVGKSIEEREEITRGIEKRNRQKDK
LRGLFEKEVGRPPQDGARGKEELLRFELWSEQMGRCLYTDDYISPSQLVATDDAVQVDHILP
WSRFADDSYANKTLCMAKANQDKKGRTPYEWFKAEKTDTEWDAFIVRVEALADMKGFKKRNY
KLRNAEEAAAKFRNRNLNDTRWACRLLAEALKQLYPKGEKDKDGKERRRVFSRPGALTDRLR
RAWGLQWMKKSTKGDRIPDDRHHALDAIVIAATTESLLQRATREVQEIEDKGLHYDLVKNVT
PPWPGFREQAVEAVEKVFVARAERRRARGKAHDATIRHIAVREGEQRVYERRKVAELKLADL
DRVKDAERNARLIEKLRNWIEAGSPKDDPPLSPKGDPIFKVRLVTKSKVNIALDTGNPKRPG
TVDRGEMARVDVFRKASKKGKYEYYLVPIYPHDIATMKTPPIRAVQAYKPEDEWPEMDSSYE
FCWSLVPMTYLQVISSKGEIFEGYYRGMNRSVGAIQLSAHSNSSDVVQGIGARTLTEFKKFN
VDRFGRKHEVERELRTWRGETWRGKAYI
SEQ ID NO: 349 
MGNYYLGLDVGIGSIGWAVINIEKKRIEDFNVRIFKSGEIQEKNRNSRASQQCRRSRGLRRL
YRRKSHRKLRLKNYLSIIGLTTSEKIDYYYETADNNVIQLRNKGLSEKLTPEEIAACLIHIC
NNRGYKDFYEVNVEDIEDPDERNEYKEEHDSIVLISNLMNEGGYCTPAEMICNCREFDEPNS
VYRKFHNSAASKNHYLITRHMLVKEVDLILENQSKYYGILDDKTIAKIKDIIFAQRDFEIGP
GKNERFRRFTGYLDSIGKCQFFKDQERGSRFTVIADIYAFVNVLSQYTYTNNRGESVFDTSF
ANDLINSALKNGSMDKRELKAIAKSYHIDISDKNSDTSLTKCFKYIKVVKPLFEKYGYDWDK
LIENYTDTDNNVLNRIGIVLSQAQTPKRRREKLKALNIGLDDGLINELTKLKLSGTANVSYK
YMQGSIEAFCEGDLYGKYQAKFNKEIPDIDENAKPQKLPPFKNEDDCEFFKNPVVFRSINET
RKLINAIIDKYGYPAAVNIETADELNKTFEDRAIDTKRNNDNQKENDRIVKEIIECIKCDEV
HARHLIEKYKLWEAQEGKCLYSGETITKEDMLRDKDKLFEVDHIVPYSLILDNTINNKALVY
AEENQKKGQRTPLMYMNEAQAADYRVRVNTMFKSKKCSKKKYQYLMLPDLNDQELLGGWRSR
NLNDTRYICKYLVNYLRKNLRFDRSYESSDEDDLKIRDHYRVFPVKSRFTSMFRRWWLNEKT
WGRYDKAELKKLTYLDHAADAIIIANCRPEYVVLAGEKLKLNKMYHQAGKRITPEYEQSKKA
CIDNLYKLFRMDRRTAEKLLSGHGRLTPIIPNLSEEVDKRLWDKNIYEQFWKDDKDKKSCEE
LYRENVASLYKGDPKFASSLSMPVISLKPDHKYRGTITGEEAIRVKEIDGKLIKLKRKSISE
ITAESINSIYTDDKILIDSLKTIFEQADYKDVGDYLKKTNQHFFTTSSGKRVNKVTVIEKVP
SRWLRKEIDDNNFSLLNDSSYYCIELYKDSKGDNNLQGIAMSDIVHDRKTKKLYLKPDFNYP
DDYYTHVMYIFPGDYLRIKSTSKKSGEQLKFEGYFISVKNVNENSFRFISDNKPCAKDKRVS
ITKKDIVIKLAVDLMGKVQGENNGKGISCGEPLSLLKEKN
SEQ ID NO: 350 
MLSRQLLGASHLARPVSYSYNVQDNDVHCSYGERCFMRGKRYRIGIDVGLNSVGLAAVEVSD
ENSPVRLLNAQSVIHDGGVDPQKNKEAITRKNMSGVARRTRRMRRRKRERLHKLDMLLGKFG
YPVIEPESLDKPFEEWHVRAELATRYIEDDELRRESISIALRHMARHRGWRNPYRQVDSLIS
DNPYSKQYGELKEKAKAYNDDATAAEEESTPAQLVVAMLDAGYAEAPRLRWRTGSKKPDAEG
YLPVRLMQEDNANELKQIFRVQRVPADEWKPLFRSVFYAVSPKGSAEQRVGQDPLAPEQARA
LKASLAFQEYRIANVITNLRIKDASAELRKLTVDEKQSIYDQLVSPSSEDITWSDLCDFLGF
KRSQLKGVGSLTEDGEERISSRPPRLTSVQRIYESDNKIRKPLVAWWKSASDNEHEAMIRLL
SNTVDIDKVREDVAYASAIEFIDGLDDDALTKLDSVDLPSGRAAYSVETLQKLTRQMLTTDD
DLHEARKTLFNVTDSWRPPADPIGEPLGNPSVDRVLKNVNRYLMNCQQRWGNPVSVNIEHVR
SSFSSVAFARKDKREYEKNNEKRSIFRSSLSEQLRADEQMEKVRESDLRRLEAIQRQNGQCL
YCGRTITFRTCEMDHIVPRKGVGSTNTRTNFAAVCAECNRMKSNTPFAIWARSEDAQTRGVS
LAEAKKRVTMFTFNPKSYAPREVKAFKQAVIARLQQTEDDAAIDNRSIESVAWMADELHRRI
DWYFNAKQYVNSASIDDAEAETMKTTVSVFQGRVTASARRAAGIEGKIHFIGQQSKTRLDRR
HHAVDASVIAMMNTAAAQTLMERESLRESQRLIGLMPGERSWKEYPYEGTSRYESFHLWLDN
MDVLLELLNDALDNDRIAVMQSQRYVLGNSIAHDATIHPLEKVPLGSAMSADLIRRASTPAL
WCALTRLPDYDEKEGLPEDSHREIRVHDTRYSADDEMGFFASQAAQIAVQEGSADIGSAIHH
ARVYRCWKTNAKGVRKYFYGMIRVFQTDLLRACHDDLFTVPLPPQSISMRYGEPRVVQALQS
 
GNAQYLGSLVVGDEIEMDFSSLDVDGQIGEYLQFFSQFSGGNLAWKHWVVDGFFNQTQLRIR
PRYLAAEGLAKAFSDDVVPDGVQKIVTKQGWLPPVNTASKTAVRIVRRNAFGEPRLSSAHHM
PCSWQWRHE
SEQ ID NO: 351 
MYSIGLDLGISSVGWSVIDERTGNVIDLGVRLFSAKNSEKNLERRTNRGGRRLIRRKTNRLK
DAKKILAAVGFYEDKSLKNSCPYQLRVKGLTEPLSRGEIYKVTLHILKKRGISYLDEVDTEA
AKESQDYKEQVRKNAQLLTKYTPGQIQLQRLKENNRVKTGINAQGNYQLNVFKVSAYANELA
TILKTQQAFYPNELTDDWIALFVQPGIAEEAGLIYRKRPYYHGPGNEANNSPYGRWSDFQKT
GEPATNIFDKLIGKDFQGELRASGLSLSAQQYNLLNDLTNLKIDGEVPLSSEQKEYILTELM
TKEFTRFGVNDVVKLLGVKKERLSGWRLDKKGKPEIHTLKGYRNWRKIFAEAGIDLATLPTE
TIDCLAKVLTLNTEREGIENTLAFELPELSESVKLLVLDRYKELSQSISTQSWHRFSLKTLH
LLIPELMNATSEQNTLLEQFQLKSDVRKRYSEYKKLPTKDVLAEIYNPTVNKTVSQAFKVID
ALLVKYGKEQIRYITIEMPRDDNEEDEKKRIKELHAKNSQRKNDSQSYFMQKSGWSQEKFQT
TIQKNRRFLAKLLYYYEQDGICAYTGLPISPELLVSDSTEIDHIIPISISLDDSINNKVLVL
SKANQVKGQQTPYDAWMDGSFKKINGKFSNWDDYQKWVESRHFSHKKENNLLETRNIFDSEQ
VEKFLARNLNDTRYASRLVLNTLQSFFTNQETKVRVVNGSFTHTLRKKWGADLDKTRETHHH
HAVDATLCAVTSFVKVSRYHYAVKEETGEKVMREIDFETGEIVNEMSYWEFKKSKKYERKTY
QVKWPNFREQLKPVNLHPRIKFSHQVDRKANRKLSDATIYSVREKTEVKTLKSGKQKITTDE
YTIGKIKDIYTLDGWEAFKKKQDKLLMKDLDEKTYERLLSIAETTPDFQEVEEKNGKVKRVK
RSPFAVYCEENDIPAIQKYAKKNNGPLIRSLKYYDGKLNKHINITKDSQGRPVEKTKNGRKV
TLQSLKPYRYDIYQDLETKAYYTVQLYYSDLRFVEGKYGITEKEYMKKVAEQTKGQVVRFCF
SLQKNDGLEIEWKDSQRYDVRFYNFQSANSINFKGLEQEMMPAENQFKQKPYNNGAINLNIA
KYGKEGKKLRKFNTDILGKKHYLFYEKEPKNIIK
SEQ ID NO: 352 
MYFYKNKENKLNKKVVLGLDLGIASVGWCLTDISQKEDNKFPIILHGVRLFETVDDSDDKLL
NETRRKKRGQRRRNRRLFTRKRDFIKYLIDNNIIELEFDKNPKILVRNFIEKYINPFSKNLE
LKYKSVTNLPIGFHNLRKAAINEKYKLDKSELIVLLYFYLSLRGAFFDNPEDTKSKEMNKNE
IEIFDKNESIKNAEFPIDKIIEFYKISGKIRSTINLKFGHQDYLKEIKQVFEKQNIDFMNYE
KFAMEEKSFFSRIRNYSEGPGNEKSFSKYGLYANENGNPELIINEKGQKIYTKIFKTLWESK
IGKCSYDKKLYRAPKNSFSAKVFDITNKLTDWKHKNEYISERLKRKILLSRFLNKDSKSAVE
KILKEENIKFENLSEIAYNKDDNKINLPIINAYHSLTTIFKKHLINFENYLISNENDLSKLM
SFYKQQSEKLFVPNEKGSYEINQNNNVLHIFDAISNILNKFSTIQDRIRILEGYFEFSNLKK
DVKSSEIYSEIAKLREFSGTSSLSFGAYYKFIPNLISEGSKNYSTISYEEKALQNQKNNFSH
SNLFEKTWVEDLIASPTVKRSLRQTMNLLKEIFKYSEKNNLEIEKIVVEVTRSSNNKHERKK
IEGINKYRKEKYEELKKVYDLPNENTTLLKKLWLLRQQQGYDAYSLRKIEANDVINKPWNYD
IDHIVPRSISFDDSFSNLVIVNKLDNAKKSNDLSAKQFIEKIYGIEKLKEAKENWGNWYLRN
ANGKAFNDKGKFIKLYTIDNLDEFDNSDFINRNLSDTSYITNALVNHLTFSNSKYKYSVVSV
NGKQTSNLRNQIAFVGIKNNKETEREWKRPEGFKSINSNDFLIREEGKNDVKDDVLIKDRSF
NGHHAEDAYFITIISQYFRSFKRIERLNVNYRKETRELDDLEKNNIKFKEKASFDNFLLINA
LDELNEKLNQMRFSRMVITKKNTQLFNETLYSGKYDKGKNTIKKVEKLNLLDNRTDKIKKIE
EFFDEDKLKENELTKLHIFNHDKNLYETLKIIWNEVKIEIKNKNLNEKNYFKYFVNKKLQEG
KISFNEWVPILDNDFKIIRKIRYIKFSSEEKETDEIIFSQSNFLKIDQRQNFSFHNTLYWVQ
IWVYKNQKDQYCFISIDARNSKFEKDEIKINYEKLKTQKEKLQIINEEPILKINKGDLFENE
EKELFYIVGRDEKPQKLEIKYILGKKIKDQKQIQKPVKKYFPNWKKVNLTYMGEIFKK
SEQ ID NO: 353 
MDNKNYRIGIDVGLNSIGFCAVEVDQHDTPLGFLNLSVYRHDAGIDPNGKKTNTTRLAMSGV
ARRTRRLFRKRKRRLAALDRFIEAQGWTLPDHADYKDPYTPWLVRAELAQTPIRDENDLHEK
LAIAVRHIARHRGWRSPWVPVRSLHVEQPPSDQYLALKERVEAKTLLQMPEGATPAEMVVAL
 
DLSVDVNLRPKNREKTDTRPENKKPGFLGGKLMQSDNANELRKIAKIQGLDDALLRELIELV
FAADSPKGASGELVGYDVLPGQHGKRRAEKAHPAFQRYRIASIVSNLRIRHLGSGADERLDV
ETQKRVFEYLLNAKPTADITWSDVAEEIGVERNLLMGTATQTADGERASAKPPVDVTNVAFA
TCKIKPLKEWWLNADYEARCVMVSALSHAEKLTEGTAAEVEVAEFLQNLSDEDNEKLDSFSL
PIGRAAYSVDSLERLTKRMIENGEDLFEARVNEFGVSEDWRPPAEPIGARVGNPAVDRVLKA
VNRYLMAAEAEWGAPLSVNIEHVREGFISKRQAVEIDRENQKRYQRNQAVRSQIADHINATS
GVRGSDVTRYLAIQRQNGECLYCGTAITFVNSEMDHIVPRAGLGSTNTRDNLVATCERCNKS
KSNKPFAVWAAECGIPGVSVAEALKRVDFWIADGFASSKEHRELQKGVKDRLKRKVSDPEID
NRSMESVAWMARELAHRVQYYFDEKHTGTKVRVFRGSLTSAARKASGFESRVNFIGGNGKTR
LDRRHHAMDAATVAMLRNSVAKTLVLRGNIRASERAIGAAETWKSFRGENVADRQIFESWSE
NMRVLVEKFNLALYNDEVSIFSSLRLQLGNGKAHDDTITKLQMHKVGDAWSLTEIDRASTPA
LWCALTRQPDFTWKDGLPANEDRTIIVNGTHYGPLDKVGIFGKAAASLLVRGGSVDIGSAIH
HARIYRIAGKKPTYGMVRVFAPDLLRYRNEDLFNVELPPQSVSMRYAEPKVREAIREGKAEY
LGWLVVGDELLLDLSSETSGQIAELQQDFPGTTHWTVAGFFSPSRLRLRPVYLAQEGLGEDV
SEGSKSIIAGQGWRPAVNKVFGSAMPEVIRRDGLGRKRRFSYSGLPVSWQG
SEQ ID NO: 354 
MRLGLDIGTSSIGWWLYETDGAGSDARITGVVDGGVRIFSDGRDPKSGASLAVDRRAARAMR
RRRDRYLRRRATLMKVLAETGLMPADPAEAKALEALDPFALRAAGLDEPLPLPHLGRALFHL
NQRRGFKSNRKTDRGDNESGKIKDATARLDMEMMANGARTYGEFLHKRRQKATDPRHVPSVR
TRLSIANRGGPDGKEEAGYDFYPDRRHLEEEFHKLWAAQGAHHPELTETLRDLLFEKIFFQR
PLKEPEVGLCLFSGHHGVPPKDPRLPKAHPLTQRRVLYETVNQLRVTADGREARPLTREERD
QVIHALDNKKPTKSLSSMVLKLPALAKVLKLRDGERFTLETGVRDAIACDPLRASPAHPDRF
GPRWSILDADAQWEVISRIRRVQSDAEHAALVDWLTEAHGLDRAHAEATAHAPLPDGYGRLG
LTATTRILYQLTADVVTYADAVKACGWHHSDGRTGECFDRLPYYGEVLERHVIPGSYHPDDD
DITRFGRITNPTVHIGLNQLRRLVNRIIETHGKPHQIVVELARDLKKSEEQKRADIKRIRDT
TEAAKKRSEKLEELEIEDNGRNRMLLRLWEDLNPDDAMRRFCPYTGTRISAAMIFDGSCDVD
HILPYSRTLDDSFPNRTLCLREANRQKRNQTPWQAWGDTPHWHAIAANLKNLPENKRWFAP
DAMTRFEGENGFLDRALKDTQYLARISRSYLDTLFTKGGHVWVVPGRFTEMLRRHWGLNSLL
SDAGRGAVKAKNRTDHRHHAIDAAVIAATDPGLLNRISRAAGQGEAAGQSAELIARDTPPPW
EGFRDDLRVRLDRIIVSHRADHGRIDHAARKQGRDSTAGQLHQETAYSIVDDIHVASRTDLL
SLKPAQLLDEPGRSGQVRDPQLRKALRVATGGKTGKDFENALRYFASKPGPYQAIRRVRIIK
PLQAQARVPVPAQDPIKAYQGGSNHLFEIWRLPDGEIEAQVITSFEAHTLEGEKRPHPAAKR
LLRVHKGDMVALERDGRRVVGHVQKMDIANGLFIVPHNEANADTRNNDKSDPFKWIQIGARP
AIASGIRRVSVDEIGRLRDGGTRPI
SEQ ID NO: 355 
MLHCIAVIRVPPSEEPGFFETHADSCALCHHGCMTYAANDKAIRYRVGIDVGLRSIGFCAVE
VDDEDHPIRILNSVVHVHDAGTGGPGETESLRKRSGVAARARRGRAEKQRLKKLDVLLEEL
GWGVSSNELLDSHAPWHIRKRLVSEYIEDETERRQCLSVAMAHIARHRGWRNSFSKVDTLLL
EQAPSDRMQGLKERVEDRTGLQFSEEVTQGELVATLLEHDGDVTIRGFVRKGGKATKVHGVL
EGKYMQSDLVAELRQICRTQRVSETTFEKLVLSIFHSKEPAPSAARQRERVGLDELQLALDP
AAKQPRAERAHPAFQKFKVVATLANMRIREQSAGERSLTSEELNRVARYLLNHTESESPTWD
DVARKLEVPRHRLRGSSRASLETGGGLTYPPVDDTTVRVMSAEVDWLADWWDCANDESRGHM
IDAISNGCGSEPDDVEDEEVNELISSATAEDMLKLELLAKKLPSGRVAYSLKTLREVTAAIL
ETGDDLSQAITRLYGVDPGWVPTPAPIEAPVGNPSVDRVLKQVARWLKFASKRWGVPQTVNI
EHTREGLKSASLLEEERERWERFEARREIRQKEMYKRLGISGPFRRSDQVRYEILDLQDCAC
LYCGNEINFQTFEVDHIIPRVDASSDSRRTNLAAVCHSCNSAKGGLAFGQWVKRGDCPSGVS
LENAIKRVRSWSKDRLGLTEKAMGKRKSEVISRLKTEMPYEEFDGRSMESVAWMAIELKKRI
EGYFNSDRPEGCAAVQVNAYSGRLTACARRAAHVDKRVRLIRLKGDDGHHKNRFDRRNHAMD
ALVIALMTPAIARTIAVREDRREAQQLTRAFESWKNFLGSEERMQDRWESWIGDVEYACDRL
 
NELIDADKIPVTENLRLRNSGKLHADQPESLKKARRGSKRPRPQRYVLGDALPADVINRVTD
PGLWTALVRAPGFDSQLGLPADLNRGLKLRGKRISADFPIDYFPTDSPALAVQGGYVGLEFH
HARLYRIIGPKEKVKYALLRVCAIDLCGIDCDDLFEVELKPSSISMRTADAKLKEAMGNGSA
KQIGWLVLGDEIQIDPTKFPKQSIGKFLKECGPVSSWRVSALDTPSKITLKPRLLSNEPLLK
TSRVGGHESDLVVAECVEKIMKKTGWVVEINALCQSGLIRVIRRNALGEVRTSPKSGLPISL
NLR
SEQ ID NO: 356 
MRYRVGLDLGTASVGAAVFSMDEQGNPMELIWHYERLFSEPLVPDMGQLKPKKAARRLARQQ
RRQIDRRASRLRRIAIVSRRLGIAPGRNDSGVHGNDVPTLRAMAVNERIELGQLRAVLLRMG
KKRGYGGTFKAVRKVGEAGEVASGASRLEEEMVALASVQNKDSVTVGEYLAARVEHGLPSKL
KVAANNEYYAPEYALFRQYLGLPAIKGRPDCLPNMYALRHQIEHEFERIWATQSQFHDVMKD
HGVKEEIRNAIFFQRPLKSPADKVGRCSLQTNLPRAPRAQIAAQNFRIEKQMADLRWGMGRR
AEMLNDHQKAVIRELLNQQKELSFRKIYKELERAGCPGPEGKGLNMDRAALGGRDDLSGNTT
LAAWRKLGLEDRWQELDEVTQIQVINFLADLGSPEQLDTDDWSCRFMGKNGRPRNFSDEFVA
FMNELRMTDGFDRLSKMGFEGGRSSYSIKALKALTEWMIAPHWRETPETHRVDEEAAIRECY
PESLATPAQGGRQSKLEPPPLTGNEVVDVALRQVRHTINMMIDDLGSVPAQIVVEMAREMKG
GVTRRNDIEKQNKRFASERKKAAQSIEENGKTPTPARILRYQLWIEQGHQCPYCESNISLEQ
ALSGAYTNFEHILPRTLTQIGRKRSELVLAHRECNDEKGNRTPYQAFGHDDRRWRIVEQRAN
ALPKKSSRKTRLLLLKDFEGEALTDESIDEFADRQLHESSWLAKVTTQWLSSLGSDVYVSRG
SLTAELRRRWGLDTVIPQVRFESGMPVVDEEGAEITPEEFEKFRLQWEGHRVTREMRTDRRP
DKRIDHRHHLVDAIVTALTSRSLYQQYAKAWKVADEKQRHGRVDVKVELPMPILTIRDIALE
AVRSVRISHKPDRYPDGRFFEATAYGIAQRLDERSGEKVDWLVSRKSLTDLAPEKKSIDVDK
VRANISRIVGEAIRLHISNIFEKRVSKGMTPQQALREPIEFQGNILRKVRCFYSKADDCVRI
EHSSRRGHHYKMLLNDGFAYMEVPCKEGILYGVPNLVRPSEAVGIKRAPESGDFIRFYKGDT
VKNIKTGRVYTIKQILGDGGGKLILTPVTETKPADLLSAKWGRLKVGGRNIHLLRLCAE
SEQ ID NO: 357 
MIGEHVRGGCLFDDHWTPNWGAFRLPNTVRTFTKAENPKDGSSLAEPRRQARGLRRRLRRKT
QRLEDLRRLLAKEGVLSLSDLETLFRETPAKDPYQLRAEGLDRPLSFPEWVRVLYHITKHRG
FQSNRRNPVEDGQERSRQEEEGKLLSGVGENERLLREGGYRTAGEMLARDPKFQDHRRNRAG
DYSHTLSRSLLLEEARRLFQSQRTLGNPHASSNLEEAFLHLVAFQNPFASGEDIRNKAGHCS
LEPDQIRAPRRSASAETFMLLQKTGNLRLIHRRTGEERPLTDKEREQIHLLAWKQEKVTHKT
LRRHLEIPEEWLFTGLPYHRSGDKAEEKLFVHLAGIHEIRKALDKGPDPAVWDTLRSRRDLL
DSIADTLTFYKNEDEILPRLESLGLSPENARALAPLSFSGTAHLSLSALGKLLPHLEEGKSY
TQARADAGYAAPPPDRHPKLPPLEEADWRNPVVFRALTQTRKVVNALVRRYGPPWCIHLETA
RELSQPAKVRRRIETEQQANEKKKQQAEREFLDIVGTAPGPGDLLKMRLWREQGGFCPYCEE
YLNPTRLAEPGYAEMDHILPYSRSLDNGWHNRVLVHGKDNRDKGNRTPFEAFGGDTARWDRL
VAWVQASHLSAPKKRNLLREDFGEEAERELKDRNLTDTRFITKTAATLLRDRLTFHPEAPKD
PVMTLNGRLTAFLRKQWGLHKNRKNGDLHHALDAAVLAVASRSFVYRLSSHNAAWGELPRGR
EAENGFSLPYPAFRSEVLARLCPTREEILLRLDQGGVGYDEAFRNGLRPVFVSRAPSRRLRG
KAHMETLRSPKWKDHPEGPRTASRIPLKDLNLEKLERMVGKDRDRKLYEALRERLAAFGGNG
KKAFVAPFRKPCRSGEGPLVRSLRIFDSGYSGVELRDGGEVYAVADHESMVRVDVYAKKNRF
YLVPVYVADVARGIVKNRAIVAHKSEEEWDLVDGSFDFRFSLFPGDLVEIEKKDGAYLGYYK
SCHRGDGRLLLDRHDRMPRESDCGTFYVSTRKDVLSMSKYQVDPLGEIRLVGSEKPPFVL
SEQ ID NO: 358 
MEKKRKVTLGFDLGIASVGWAIVDSETNQVYKLGSRLFDAPDTNLERRTQRGTRRLLRRRKY
RNQKFYNLVKRTEVFGLSSREAIENRFRELSIKYPNIIELKTKALSQEVCPDEIAWILHDYL
KNRGYFYDEKETKEDFDQQTVESMPSYKLNEFYKKYGYFKGALSQPTESEMKDNKDLKEAFF
FDFSNKEWLKEINYFFNVQKNILSETFIEEFKKIFSFTRDISKGPGSDNMPSPYGIFGEFGD
 
NGQGGRYEHIWDKNIGKCSIFTNEQRAPKYLPSALIFNFLNELANIRLYSTDKKNIQPLWKL
SSVDKLNILLNLFNLPISEKKKKLTSTNINDIVKKESIKSIMISVEDIDMIKDEWAGKEPNV
YGVGLSGLNIEESAKENKFKFQDLKILNVLINLLDNVGIKFEFKDRNDIIKNLELLDNLYLF
LIYQKESNNKDSSIDLFIAKNESLNIENLKLKLKEFLLGAGNEFENHNSKTHSLSKKAIDEI
LPKLLDNNEGWNLEAIKNYDEEIKSQIEDNSSLMAKQDKKYLNDNFLKDAILPPNVKVTFQQ
AILIFNKIIQKFSKDFEIDKVVIELAREMTQDQENDALKGIAKAQKSKKSLVEERLEANNID
KSVFNDKYEKLIYKIFLWISQDFKDPYTGAQISVNEIVNNKVEIDHIIPYSLCFDDSSANKV
LVHKQSNQEKSNSLPYEYIKQGHSGWNWDEFTKYVKRVFVNNVDSILSKKERLKKSENLLTA
SYDGYDKLGFLARNLNDTRYATILFRDQLNNYAEHHLIDNKKMFKVIAMNGAVTSFIRKNMS
YDNKLRLKDRSDFSHHAYDAAIIALFSNKTKTLYNLIDPSLNGIISKRSEGYWVIEDRYTGE
IKELKKEDWTSIKNNVQARKIAKEIEEYLIDLDDEVFFSRKTKRKTNRQLYNETIYGIATKT
DEDGITNYYKKEKFSILDDKDIYLRLLREREKFVINQSNPEVIDQIIEIIESYGKENNIPSR
DEAINIKYTKNKINYNLYLKQYMRSLTKSLDQFSEEFINQMIANKTFVLYNPTKNTTRKIKF
LRLVNDVKINDIRKNQVINKFNGKNNEPKAFYENINSLGAIVFKNSANNFKTLSINTQIAIF
GDKNWDIEDFKTYNMEKIEKYKEIYGIDKTYNFHSFIFPGTILLDKQNKEFYYISSIQTVRD
IIEIKFLNKIEFKDENKNQDTSKTPKRLMFGIKSIMNNYEQVDISPFGINKKIFE
SEQ ID NO: 359 
MGYRIGLDVGITSTGYAVLKTDKNGLPYKILTLDSVIYPRAENPQTGASLAEPRRIKRGLRR
RTRRTKFRKQRTQQLFIHSGLLSKPEIEQILATPQAKYSVYELRVAGLDRRLTNSELFRVLY
FFIGHRGFKSNRKAELNPENEADKKQMGQLLNSIEEIRKAIAEKGYRTVGELYLKDPKYNDH
KRNKGYIDGYLSTPNRQMLVDEIKQILDKQRELGNEKLTDEFYATYLLGDENRAGIFQAQRD
FDEGPGAGPYAGDQIKKMVGKDIFEPTEDRAAKATYTFQYFNLLQKMTSLNYQNTTGDTWHT
LNGLDRQAIIDAVFAKAEKPTKTYKPTDFGELRKLLKLPDDARFNLVNYGSLQTQKEIETVE
KKTRFVDFKAYHDLVKVLPEEMWQSRQLLDHIGTALTLYSSDKRRRRYFAEELNLPAELIEK
LLPLNFSKFGHLSIKSMQNIIPYLEMGQVYSEATTNTGYDFRKKQISKDTIREEITNPVVRR
AVTKTIKIVEQIIRRYGKPDGINIELARELGRNFKERGDIQKRQDKNRQTNDKIAAELTELG
IPVNGQNIIRYKLHKEQNGVDPYTGDQIPFERAFSEGYEVDHIIPYSISWDDSYTNKVLTSA
KCNREKGNRIPMVYLANNEQRLNALTNIADNIIRNSRKRQKLLKQKLSDEELKDWKQRNIND
TRFITRVLYNYFRQAIEFNPELEKKQRVLPLNGEVTSKIRSRWGFLKVREDGDLHHAIDATV
IAAITPKFIQQVTKYSQHQEVKNNQALWHDAEIKDAEYAAEAQRMDADLFNKIFNGFPLPWP
EFLDELLARISDNPVEMMKSRSWNTYTPIEIAKLKPVFVVRLANHKISGPAHLDTIRSAKLF
DEKGIVLSRVSITKLKINKKGQVATGDGIYDPENSNNGDKVVYSAIRQALEAHNGSGELAFP
DGYLEYVDHGTKKLVRKVRVAKKVSLPVRLKNKAAADNGSMVRIDVFNTGKKFVFVPIYIKD
TVEQVLPNKAIARGKSLWYQITESDQFCFSLYPGDMVHIESKTGIKPKYSNKENNTSVVPIK
NFYGYFDGADIATASILVRAHDSSYTARSIGIAGLLKFEKYQVDYFGRYHKVHEKKRQLFVK
RDE
SEQ ID NO: 360 
MQKNINTKQNHIYIKQAQKIKEKLGDKPYRIGLDLGVGSIGFAIVSMEENDGNVLLPKEIIM
VGSRIFKASAGAADRKLSRGQRNNHRHTRERMRYLWKVLAEQKLALPVPADLDRKENSSEGE
TSAKRFLGDVLQKDIYELRVKSLDERLSLQELGYVLYHIAGHRGSSAIRTFENDSEEAQKEN
TENKKIAGNIKRLMAKKNYRTYGEYLYKEFFENKEKHKREKISNAANNHKFSPTRDLVIKEA
EAILKKQAGKDGFHKELTEEYIEKLTKAIGYESEKLIPESGFCPYLKDEKRLPASHKLNEER
RLWETLNNARYSDPIVDIVTGEITGYYEKQFTKEQKQKLFDYLLTGSELTPAQTKKLLGLKN
TNFEDIILQGRDKKAQKIKGYKLIKLESMPFWARLSEAQQDSFLYDWNSCPDEKLLTEKLSN
EYHLTEEEIDNAFNEIVLSSSYAPLGKSAMLIILEKIKNDLSYTEAVEEALKEGKLTKEKQA
IKDRLPYYGAVLQESTQKIIAKGFSPQFKDKGYKTPHTNKYELEYGRIANPVVHQTLNELRK
LVNEIIDILGKKPCEIGLETARELKKSAEDRSKLSREQNDNESNRNRIYEIYIRPQQQVIIT
RRENPRNYILKFELLEEQKSQCPFCGGQISPNDIINNQADIEHLFPIAESEDNGRNNLVISH
SACNADKAKRSPWAAFASAAKDSKYDYNRILSNVKENIPHKAWRFNQGAFEKFIENKPMAAR
 
FKTDNSYISKVAHKYLACLFEKPNIICVKGSLTAQLRMAWGLQGLMIPFAKQLITEKESESF
NKDVNSNKKIRLDNRHHALDAIVIAYASRGYGNLLNKMAGKDYKINYSERNWLSKILLPPNN
IVWENIDADLESFESSVKTALKNAFISVKHDHSDNGELVKGTMYKIFYSERGYTLTTYKKLS
ALKLTDPQKKKTPKDFLETALLKFKGRESEMKNEKIKSAIENNKRLFDVIQDNLEKAKKLLE
EENEKSKAEGKKEKNINDASIYQKAISLSGDKYVQLSKKEPGKFFAISKPTPTTTGYGYDTG
DSLCVDLYYDNKGKLCGEIIRKIDAQQKNPLKYKEQGFTLFERIYGGDILEVDFDIHSDKNS
FRNNTGSAPENRVFIKVGTFTEITNNNIQIWFGNIIKSTGGQDDSFTINSMQQYNPRKLILS
SCGFIKYRSPILKNKEG
SEQ ID NO: 361 
MAAFKPNPINYILGLDIGIASVGWAMVEIDEDENPICLIDLGVRVFERAEVPKTGDSLAMAR
RLARSVRRLTRRRAHRLLRARRLLKREGVLQAADFDENGLIKSLPNTPWQLRAAALDRKLTP
LEWSAVLLHLIKHRGYLSQRKNEGETADKELGALLKGVADNAHALQTGDFRTPAELALNKFE
KESGHIRNQRGDYSHTFSRKDLQAELILLFEKQKEFGNPHVSGGLKEGIETLLMTQRPALSG
DAVQKMLGHCTFEPAEPKAAKNTYTAERFIWLTKLNNLRILEQGSERPLTDTERATLMDEPY
RKSKLTYAQARKLLGLEDTAFFKGLRYGKDNAEASTLMEMKAYHAISRALEKEGLKDKKSPL
NLSPELQDEIGTAFSLFKTDEDITGRLKDRIQPEILEALLKHISFDKFVQISLKALRRIVPL
MEQGKRYDEACAEIYGDHYGKKNTEEKIYLPPIPADEIRNPVVLRALSQARKVINGVVRRYG
SPARIHIETAREVGKSFKDRKEIEKRQEENRKDREKAAAKFREYFPNFVGEPKSKDILKLRL
YEQQHGKCLYSGKEINLGRLNEKGYVEIDHALPFSRTWDDSFNNKVLVLGSENQNKGNQTPY
EYFNGKDNSREWQEFKARVETSRFPRSKKQRILLQKFDEDGFKERNLNDTRYVNRFLCQFVA
DRMRLTGKGKKRVFASNGQITNLLRGFWGLRKVRAENDRHHALDAVVVACSTVAMQQKITRF
VRYKEMNAFDGKTIDKETGEVLHQKTHFPQPWEFFAQEVMIRVFGKPDGKPEFEEADTPEKL
RTLLAEKLSSRPEAVHEYVTPLFVSRAPNRKMSGQGHMETVKSAKRLDEGVSVLRVPLTQLK
LKDLEKMVNREREPKLYEALKARLEAHKDDPAKAFAEPFYKYDKAGNRTQQVKAVRVEQVQK
TGVWVRNHNGIADNATMVRVDVFEKGDKYYLVPIYSWQVAKGILPDRAVVQGKDEEDWQLID
DSFNFKFSLHPNDLVEVITKKARMFGYFASCHRGTGNINIRIHDLDHKIGKNGILEGIGVKT
ALSFQKYQIDELGKEIRPCRLKKRPPVR
SEQ ID NO: 362 
MQTTNLSYILGLDLGIASVGWAVVEINENEDPIGLIDVGVRIFERAEVPKTGESLALSRRLA
RSTRRLIRRRAHRLLLAKRFLKREGILSTIDLEKGLPNQAWELRVAGLERRLSAIEWGAVLL
HLIKHRGYLSKRKNESQTNNKELGALLSGVAQNHQLLQSDDYRTPAELALKKFAKEEGHIRN
QRGAYTHTFNRLDLLAELNLLFAQQHQFGNPHCKEHIQQYMTELLMWQKPALSGEAILKMLG
KCTHEKNEFKAAKHTYSAERFVWLTKLNNLRILEDGAERALNEEERQLLINHPYEKSKLTYA
QVRKLLGLSEQAIFKHLRYSKENAESATFMELKAWHAIRKALENQGLKDTWQDLAKKPDLLD
EIGTAFSLYKTDEDIQQYLTNKVPNSVINALLVSLNFDKFIELSLKSLRKILPLMEQGKRYD
QACREIYGHHYGEANQKTSQLLPAIPAQEIRNPVVLRTLSQARKVINAIIRQYGSPARVHIE
TGRELGKSFKERREIQKQQEDNRTKRESAVQKFKELFSDFSSEPKSKDILKFRLYEQQHGKC
LYSGKEINIHRLNEKGYVEIDHALPFSRTWDDSFNNKVLVLASENQNKGNQTPYEWLQGKIN
SERWKNFVALVLGSQCSAAKKQRLLTQVIDDNKFIDRNLNDTRYIARFLSNYIQENLLLVGK
NKKNVFTPNGQITALLRSRWGLIKARENNNRHHALDAIVVACATPSMQQKITRFIRFKEVHP
YKIENRYEMVDQESGEIISPHFPEPWAYFRQEVNIRVFDNHPDTVLKEMLPDRPQANHQFVQ
PLFVSRAPTRKMSGQGHMETIKSAKRLAEGISVLRIPLTQLKPNLLENMVNKEREPALYAGL
KARLAEFNQDPAKAFATPFYKQGGQQVKAIRVEQVQKSGVLVRENNGVADNASIVRTDVFIK
NNKFFLVPIYTWQVAKGILPNKAIVAHKNEDEWEEMDEGAKFKFSLFPNDLVELKTKKEYFF
GYYIGLDRATGNISLKEHDGEISKGKDGVYRVGVKLALSFEKYQVDELGKNRQICRPQQRQP
VR
SEQ ID NO: 363 
MGIRFAFDLGTNSIGWAVWRTGPGVFGEDTAASLDGSGVLIFKDGRNPKDGQSLATMRRVPR
QSRKRRDRFVLRRRDLLAALRKAGLFPVDVEEGRRLAATDPYHLRAKALDESLTPHEMGRVI
FHLNQRRGFRSNRKADRQDREKGKIAEGSKRLAETLAATNCRTLGEFLWSRHRGTPRTRSPT
RIRMEGEGAKALYAFYPTREMVRAEFERLWTAQSRFAPDLLTPERHEEIAGILFRQRDLAPP
KIGCCTFEPSERRLPRALPSVEARGIYERLAHLRITTGPVSDRGLTRPERDVLASALLAGKS
LTFKAVRKTLKILPHALVNFEEAGEKGLDGALTAKLLSKPDHYGAAWHGLSFAEKDTFVGKL
LDEADEERLIRRLVTENRLSEDAARRCASIPLADGYGRLGRTANTEILAALVEETDETGTVV
TYAEAVRRAGERTGRNWHHSDERDGVILDRLPYYGEILQRHVVPGSGEPEEKNEAARWGRLA
NPTVHIGLNQLRKVVNRLIAAHGRPDQIVVELARELKLNREQKERLDRENRKNREENERRTA
ILAEHGQRDTAENKIRLRLFEEQARANAGIALCPYTGRAIGIAELFTSEVEIDHILPVSLTL
DDSLANRVLCRREANREKRRQTPFQAFGATPAWNDIVARAAKLPPNKRWRFDPAALERFERE
GGFLGRQLNETKYLSRLAKIYLGKICDPDRVYVTPGTLTGLLRARWGLNSILSDSNFKNRSD
HRHHAVDAVVIGVLTRGMIQRIAHDAARAEDQDLDRVFRDVPVPFEDFRDHVRERVSTITVA
VKPEHGKGGALHEDTSYGLVPDTDPNAALGNLVVRKPIRSLTAGEVDRVRDRALRARLGALA
APFRDESGRVRDAKGLAQALEAFGAENGIRRVRILKPDASVVTIADRRTGVPYRAVAPGENH
HVDIVQMRDGSWRGFAASVFEVNRPGWRPEWEVKKLGGKLVMRLHKGDMVELSDKDGQRRVK
VVQQIEISANRVRLSPHNDGGKLQDRHADADDPFRWDLATIPLLKDRGCVAVRVDPIGVVTL
RRSNV
SEQ ID NO: 364 
MMEVFMGRLVLGLDIGITSVGFGIIDLDESEIVDYGVRLFKEGTAAENETRRTKRGGRRLKR
RRVTRREDMLHLLKQAGIISTSFHPLNNPYDVRVKGLNERLNGEELATALLHLCKHRGSSVE
TIEDDEAKAKEAGETKKVLSMNDQLLKSGKYVCEIQKERLRTNGHIRGHENNFKTRAYVDEA
FQILSHQDLSNELKSAIITIISRKRMYYDGPGGPLSPTPYGRYTYFGQKEPIDLIEKMRGKC
SLFPNEPRAPKLAYSAELFNLLNDLNNLSIEGEKLTSEQKAMILKIVHEKGKITPKQLAKEV
GVSLEQIRGFRIDTKGSPLLSELTGYKMIREVLEKSNDEHLEDHVFYDEIAEILTKTKDIEG
RKKQISELSSDLNEESVHQLAGLTKFTAYHSLSFKALRLINEEMLKTELNQMQSITLFGLKQ
NNELSVKGMKNIQADDTAILSPVAKRAQRETFKVVNRLREIYGEFDSIVVEMAREKNSEEQR
KAIRERQKFFEMRNKQVADIIGDDRKINAKLREKLVLYQEQDGKTAYSLEPIDLKLLIDDPN
AYEVDHIIPISISLDDSITNKVLVTHRENQEKGNLTPISAFVKGRFTKGSLAQYKAYCLKLK
EKNIKTNKGYRKKVEQYLLNENDIYKYDIQKEFINRNLVDTSYASRVVLNTLTTYFKQNEIP
TKVFTVKGSLTNAFRRKINLKKDRDEDYGHHAIDALIIASMPKMRLLSTIFSRYKIEDIYDE
STGEVFSSGDDSMYYDDRYFAFIASLKAIKVRKFSHKIDTKPNRSVADETIYSTRVIDGKEK
VVKKYKDIYDPKFTALAEDILNNAYQEKYLMALHDPQTFDQIVKVVNYYFEEMSKSEKYFTK
DKKGRIKISGMNPLSLYRDEHGMLKKYSKKGDGPAITQMKYFDGVLGNHIDISAHYQVRDKK
VVLQQISPYRTDFYYSKENGYKFVTIRYKDVRWSEKKKKYVIDQQDYAMKKAEKKIDDTYEF
QFSMHRDELIGITKAEGEALIYPDETWHNFNFFFHAGETPEILKFTATNNDKSNKIEVKPIH
CYCKMRLMPTISKKIVRIDKYATDVVGNLYKVKKNTLKFEFD
SEQ ID NO: 365 
MKKILGVDLGITSFGYAILQETGKDLYRCLDNSVVMRNNPYDEKSGESSQSIRSTQKSMRRL
IEKRKKRIRCVAQTMERYGILDYSETMKINDPKNNPIKNRWQLRAVDAWKRPLSPQELFAIF
AHMAKHRGYKSIATEDLIYELELELGLNDPEKESEKKADERRQVYNALRHLEELRKKYGGET
IAQTIHRAVEAGDLRSYRNHDDYEKMIRREDIEEEIEKVLLRQAELGALGLPEEQVSELIDE
LKACITDQEMPTIDESLFGKCTFYKDELAAPAYSYLYDLYRLYKKLADLNIDGYEVTQEDRE
KVIEWVEKKIAQGKNLKKITHKDLRKILGLAPEQKIFGVEDERIVKGKKEPRTFVPFFFLAD
IAKFKELFASIQKHPDALQIFRELAEILQRSKTPQEALDRLRALMAGKGIDTDDRELLELFK
NKRSGTRELSHRYILEALPLFLEGYDEKEVQRILGFDDREDYSRYPKSLRHLHLREGNLFEK
EENPINNHAVKSLASWALGLIADLSWRYGPFDEIILETTRDALPEKIRKEIDKAMREREKAL
DKIIGKYKKEFPSIDKRLARKIQLWERQKGLDLYSGKVINLSQLLDGSADIEHIVPQSLGGL
STDYNTIVTLKSVNAAKGNRLPGDWLAGNPDYRERIGMLSEKGLIDWKKRKNLLAQSLDEIY
 
TENTHSKGIRATSYLEALVAQVLKRYYPFPDPELRKNGIGVRMIPGKVTSKTRSLLGIKSKS
RETNFHHAEDALILSTLTRGWQNRLHRMLRDNYGKSEAELKELWKKYMPHIEGLTLADYIDE
AFRRFMSKGEESLFYRDMFDTIRSISYWVDKKPLSASSHKETVYSSRHEVPTLRKNILEAFD
SLNVIKDRHKLTTEEFMKRYDKEIRQKLWLHRIGNTNDESYRAVEERATQIAQILTRYQLMD
AQNDKEIDEKFQQALKELITSPIEVTGKLLRKMRFVYDKLNAMQIDRGLVETDKNMLGIHIS
KGPNEKLIFRRMDVNNAHELQKERSGILCYLNEMLFIFNKKGLIHYGCLRSYLEKGQGSKYI
ALFNPRFPANPKAQPSKFTSDSKIKQVGIGSATGIIKAHLDLDGHVRSYEVFGTLPEGSIEW
FKEESGYGRVEDDPHH
SEQ ID NO: 366 
MRPIEPWILGLDIGTDSLGWAVFSCEEKGPPTAKELLGGGVRLFDSGRDAKDHTSRQAERGA
FRRARRQTRTWPWRRDRLIALFQAAGLTPPAAETRQIALALRREAVSRPLAPDALWAALLHL
AHHRGFRSNRIDKRERAAAKALAKAKPAKATAKATAPAKEADDEAGFWEGAEAALRQRMAAS
GAPTVGALLADDLDRGQPVRMRYNQSDRDGVVAPTRALIAEELAEIVARQSSAYPGLDWPAV
TRLVLDQRPLRSKGAGPCAFLPGEDRALRALPTVQDFIIRQTLANLRLPSTSADEPRPLTDE
EHAKALALLSTARFVEWPALRRALGLKRGVKFTAETERNGAKQAARGTAGNLTEAILAPLIP
GWSGWDLDRKDRVFSDLWAARQDRSALLALIGDPRGPTRVTEDETAEAVADAIQIVLPTGRA
SLSAKAARAIAQAMAPGIGYDEAVTLALGLHHSHRPRQERLARLPYYAAALPDVGLDGDPVG
PPPAEDDGAAAEAYYGRIGNISVHIALNETRKIVNALLHRHGPILRLVMVETTRELKAGADE
RKRMIAEQAERERENAEIDVELRKSDRWMANARERRQRVRLARRQNNLCPYTSTPIGHADLL
GDAYDIDHVIPLARGGRDSLDNMVLCQSDANKTKGDKTPWEAFHDKPGWIAQRDDFLARLDP
QTAKALAWRFADDAGERVARKSAEDEDQGFLPRQLTDTGYIARVALRYLSLVTNEPNAVVAT
NGRLTGLLRLAWDITPGPAPRDLLPTPRDALRDDTAARRFLDGLTPPPLAKAVEGAVQARLA
ALGRSRVADAGLADALGLTLASLGGGGKNRADHRHHFIDAAMIAVTTRGLINQINQASGAGR
ILDLRKWPRTNFEPPYPTFRAEVMKQWDHIHPSIRPAHRDGGSLHAATVFGVRNRPDARVLV
QRKPVEKLFLDANAKPLPADKIAEIIDGFASPRMAKRFKALLARYQAAHPEVPPALAALAVA
RDPAFGPRGMTANTVIAGRSDGDGEDAGLITPFRANPKAAVRTMGNAVYEVWEIQVKGRPRW
THRVLTRFDRTQPAPPPPPENARLVMRLRRGDLVYWPLESGDRLFLVKKMAVDGRLALWPAR
LATGKATALYAQLSCPNINLNGDQGYCVQSAEGIRKEKIRTTSCTALGRLRLSKKAT
SEQ ID NO: 367 
MKYTLGLDVGIASVGWAVIDKDNNKIIDLGVRCFDKAEESKTGESLATARRIARGMRRRISR
RSQRLRLVKKLFVQYEIIKDSSEFNRIFDTSRDGWKDPWELRYNALSRILKPYELVQVLTHI
TKRRGFKSNRKEDLSTTKEGVVITSIKNNSEMLRTKNYRTIGEMIFMETPENSNKRNKVDEY
IHTIAREDLLNEIKYIFSIQRKLGSPFVTEKLEHDFLNIWEFQRPFASGDSILSKVGKCTLL
KEELRAPTSCYTSEYFGLLQSINNLVLVEDNNTLTLNNDQRAKIIEYAHFKNEIKYSEIRKL
LDIEPEILFKAHNLTHKNPSGNNESKKFYEMKSYHKLKSTLPTDIWGKLHSNKESLDNLFYC
LTVYKNDNEIKDYLQANNLDYLIEYIAKLPTFNKFKHLSLVAMKRIIPFMEKGYKYSDACNM
AELDFTGSSKLEKCNKLTVEPIIENVTNPVVIRALTQARKVINAIIQKYGLPYMVNIELARE
AGMTRQDRDNLKKEHENNRKAREKISDLIRQNGRVASGLDILKWRLWEDQGGRCAYSGKPIP
VCDLLNDSLTQIDHIYPYSRSMDDSYMNKVLVLTDENQNKRSYTPYEVWGSTEKWEDFEARI
YSMHLPQSKEKRLLNRNFITKDLDSFISRNLNDTRYISRFLKNYIESYLQFSNDSPKSCVVC
VNGQCTAQLRSRWGLNKNREESDLHHALDAAVIACADRKIIKEITNYYNERENHNYKVKYPL
PWHSFRQDLMETLAGVFISRAPRRKITGPAHDETIRSPKHFNKGLTSVKIPLTTVTLEKLET
MVKNTKGGISDKAVYNVLKNRLIEHNNKPLKAFAEKIYKPLKNGTNGAIIRSIRVETPSYTG
VFRNEGKGISDNSLMVRVDVFKKKDKYYLVPIYVAHMIKKELPSKAIVPLKPESQWELIDST
HEFLFSLYQNDYLVIKTKKGITEGYYRSCHRGTGSLSLMPHFANNKNVKIDIGVRTAISIEK
YNVDILGNKSIVKGEPRRGMEKYNSFKSN
SEQ ID NO: 368 
MIRTLGIDIGIASIGWAVIEGEYTDKGLENKEIVASGVRVFTKAENPKNKESLALPRTLARS
ARRRNARKKGRIQQVKHYLSKALGLDLECFVQGEKLATLFQTSKDFLSPWELRERALYRVLD
KEELARVILHIAKRRGYDDITYGVEDNDSGKIKKAIAENSKRIKEEQCKTIGEMMYKLYFQK
SLNVRNKKESYNRCVGRSELREELKTIFQIQQELKSPWVNEELIYKLLGNPDAQSKQEREGL
IFYQRPLKGFGDKIGKCSHIKKGENSPYRACKHAPSAEEFVALTKSINFLKNLTNRHGLCFS
QEDMCVYLGKILQEAQKNEKGLTYSKLKLLLDLPSDFEFLGLDYSGKNPEKAVFLSLPSTFK
LNKITQDRKTQDKIANILGANKDWEAILKELESLQLSKEQIQTIKDAKLNFSKHINLSLEAL
YHLLPLMREGKRYDEGVEILQERGIFSKPQPKNRQLLPPLSELAKEESYFDIPNPVLRRALS
EFRKVVNALLEKYGGFHYFHIELTRDVCKAKSARMQLEKINKKNKSENDAASQLLEVLGLPN
TYNNRLKCKLWKQQEEYCLYSGEKITIDHLKDQRALQIDHAFPLSRSLDDSQSNKVLCLTSS
NQEKSNKTPYEWLGSDEKKWDMYVGRVYSSNFSPSKKRKLTQKNFKERNEEDFLARNLVDTG
YIGRVTKEYIKHSLSFLPLPDGKKEHIRIISGSMTSTMRSFWGVQEKNRDHHLHHAQDAIII
ACIEPSMIQKYTTYLKDKETHRLKSHQKAQILREGDHKLSLRWPMSNFKDKIQESIQNIIPS
HHVSHKVTGELHQETVRTKEFYYQAFGGEEGVKKALKFGKIREINQGIVDNGAMVRVDIFKS
KDKGKFYAVPIYTYDFAIGKLPNKAIVQGKKNGIIKDWLEMDENYEFCFSLFKNDCIKIQTK
EMQEAVLAIYKSTNSAKATIELEHLSKYALKNEDEEKMFTDTDKEKNKTMTRESCGIQGLKV
FQKVKLSVLGEVLEHKPRNRQNIALKTTPKHV
SEQ ID NO: 369 
MKYSIGLDIGIASVGWSVINKDKERIEDMGVRIFQKAENPKDGSSLASSRREKRGSRRRNRR
KKHRLDRIKNILCESGLVKKNEIEKIYKNAYLKSPWELRAKSLEAKISNKEIAQILLHIAKR
RGFKSFRKTDRNADDTGKLLSGIQENKKIMEEKGYLTIGDMVAKDPKFNTHVRNKAGSYLFS
FSRKLLEDEVRKIQAKQKELGNTHFTDDVLEKYIEVFNSQRNFDEGPSKPSPYYSEIGQIAK
MIGNCTFESSEKRTAKNTWSGERFVFLQKLNNFRIVGLSGKRPLTEEERDIVEKEVYLKKEV
RYEKLRKILYLKEEERFGDLNYSKDEKQDKKTEKTKFISLIGNYTIKKLNLSEKLKSEIEED
KSKLDKIIEILTFNKSDKTIESNLKKLELSREDIEILLSEEFSGTLNLSLKAIKKILPYLEK
GLSYNEACEKADYDYKNNGIKFKRGELLPVVDKDLIANPVVLRAISQTRKVVNAIIRKYGTP
HTIHVEVARDLAKSYDDRQTIIKENKKRELENEKTKKFISEEFGIKNVKGKLLLKYRLYQEQ
EGRCAYSRKELSLSEVILDESMTDIDHIIPYSRSMDDSYSNKVLVLSGENRKKSNLLPKEYF
DRQGRDWDTFVLNVKAMKIHPRKKSNLLKEKFTREDNKDWKSRALNDTRYISRFVANYLENA
LEYRDDSPKKRVFMIPGQLTAQLRARWRLNKVRENGDLHHALDAAVVAVTDQKAINNISNIS
RYKELKNCKDVIPSIEYHADEETGEVYFEEVKDTRFPMPWSGFDLELQKRLESENPREEFYN
LLSDKRYLGWFNYEEGFIEKLRPVFVSRMPNRGVKGQAHQETIRSSKKISNQIAVSKKPLNS
IKLKDLEKMQGRDTDRKLYEALKNRLEEYDDKPEKAFAEPFYKPTNSGKRGPLVRGIKVEEK
QNVGVYVNGGQASNGSMVRIDVFRKNGKFYTVPIYVHQTLLKELPNRAINGKPYKDWDLIDG
SFEFLYSFYPNDLIEIEFGKSKSIKNDNKLTKTEIPEVNLSEVLGYYRGMDTSTGAATIDTQ
DGKIQMRIGIKTVKNIKKYQVDVLGNVYKVKREKRQTF
SEQ ID NO: 370 
MSKKVSRRYEEQAQEICQRLGSRPYSIGLDLGVGSIGVAVAAYDPIKKQPSDLVFVSSRIFI
PSTGAAERRQKRGQRNSLRHRANRLKFLWKLLAERNLMLSYSEQDVPDPARLRFEDAVVRAN
PYELRLKGLNEQLTLSELGYALYHIANHRGSSSVRTFLDEEKSSDDKKLEEQQAMTEQLAKE
KGISTFIEVLTAFNTNGLIGYRNSESVKSKGVPVPTRDIISNEIDVLLQTQKQFYQEILSDE
YCDRIVSAILFENEKIVPEAGCCPYFPDEKKLPRCHFLNEERRLWEAINNARIKMPMQEGAA
KRYQSASFSDEQRHILFHIARSGTDITPKLVQKEFPALKTSIIVLQGKEKAIQKIAGFRFRR
LEEKSFWKRLSEEQKDDFFSAWTNTPDDKRLSKYLMKHLLLTENEVVDALKTVSLIGDYGPI
GKTATQLLMKHLEDGLTYTEALERGMETGEFQELSVWEQQSLLPYYGQILTGSTQALMGKYW
HSAFKEKRDSEGFFKPNTNSDEEKYGRIANPVVHQTLNELRKLMNELITILGAKPQEITVEL
ARELKVGAEKREDIIKQQTKQEKEAVLAYSKYCEPNNLDKRYIERFRLLEDQAFVCPYCLEH
ISVADIAAGRADVDHIFPRDDTADNSYGNKVVAHRQCNDIKGKRTPYAAFSNTSAWGPIMHY
LDETPGMWRKRRKFETNEEEYAKYLQSKGFVSRFESDNSYIAKAAKEYLRCLFNPNNVTAVG
 
SLKGMETSILRKAWNLQGIDDLLGSRHWSKDADTSPTMRKNRDDNRHHGLDAIVALYCSRSL
VQMINTMSEQGKRAVEIEAMIPIPGYASEPNLSFEAQRELFRKKILEFMDLHAFVSMKTDND
ANGALLKDTVYSILGADTQGEDLVFVVKKKIKDIGVKIGDYEEVASAIRGRITDKQPKWYPM
EMKDKIEQLQSKNEAALQKYKESLVQAAAVLEESNRKLIESGKKPIQLSEKTISKKALELVG
GYYYLISNNKRTKTFVVKEPSNEVKGFAFDTGSNLCLDFYHDAQGKLCGEIIRKIQAMNPSY
KPAYMKQGYSLYVRLYQGDVCELRASDLTEAESNLAKTTHVRLPNAKPGRTFVIIITFTEMG
SGYQIYFSNLAKSKKGQDTSFTLTTIKNYDVRKVQLSSAGLVRYVSPLLVDKIEKDEVALCG
E
SEQ ID NO: 371 
MNQKFILGLDIGITSVGYGLIDYETKNIIDAGVRLFPEANVENNEGRRSKRGSRRLKRRRIH
RLERVKKLLEDYNLLDQSQIPQSTNPYAIRVKGLSEALSKDELVIALLHIAKRRGIHKIDVI
DSNDDVGNELSTKEQLNKNSKLLKDKFVCQIQLERMNEGQVRGEKNRFKTADIIKEIIQLLN
VQKNFHQLDENFINKYIELVEMRREYFEGPGKGSPYGWEGDPKAWYETLMGHCTYFPDELRS
VKYAYSADLFNALNDLNNLVIQRDGLSKLEYHEKYHIIENVFKQKKKPTLKQIANEINVNPE
DIKGYRITKSGKPQFTEFKLYHDLKSVLFDQSILENEDVLDQIAEILTIYQDKDSIKSKLTE
LDILLNEEDKENIAQLTGYTGTHRLSLKCIRLVLEEQWYSSRNQMEIFTHLNIKPKKINLTA
ANKIPKAMIDEFILSPVVKRTFGQAINLINKIIEKYGVPEDIIIELARENNSKDKQKFINEM
QKKNENTRKRINEIIGKYGNQNAKRLVEKIRLHDEQEGKCLYSLESIPLEDLLNNPNHYEVD
HIIPRSVSFDNSYHNKVLVKQSENSKKSNLTPYQYFNSGKSKLSYNQFKQHILNLSKSQDRI
SKKKKEYLLEERDINKFEVQKEFINRNLVDTRYATRELTNYLKAYFSANNMNVKVKTINGSF
TDYLRKVWKFKKERNHGYKHHAEDALIIANADFLFKENKKLKAVNSVLEKPEIESKQLDIQV
DSEDNYSEMFIIPKQVQDIKDFRNFKYSHRVDKKPNRQLINDTLYSTRKKDNSTYIVQTIKD
IYAKDNTTLKKQFDKSPEKFLMYQHDPRTFEKLEVIMKQYANEKNPLAKYHEETGEYLTKYS
KKNNGPIVKSLKYIGNKLGSHLDVTHQFKSSTKKLVKLSIKPYRFDVYLTDKGYKFITISYL
DVLKKDNYYYIPEQKYDKLKLGKAIDKNAKFIASFYKNDLIKLDGEIYKIIGVNSDTRNMIE
LDLPDIRYKEYCELNNIKGEPRIKKTIGKKVNSIEKLTTDVLGNVFTNTQYTKPQLLFKRGN
SEQ ID NO: 372 
MIMKLEKWRLGLDLGTNSIGWSVFSLDKDNSVQDLIDMGVRIFSDGRDPKTKEPLAVARRTA
RSQRKLIYRRKLRRKQVFKFLQEQGLFPKTKEECMTLKSLNPYELRIKALDEKLEPYELGRA
LFNLAVRRGFKSNRKDGSREEVSEKKSPDEIKTQADMQTHLEKAIKENGCRTITEFLYKNQG
ENGGIRFAPGRMTYYPTRKMYEEEFNLIRSKQEKYYPQVDWDDIYKAIFYQRPLKPQQRGYC
IYENDKERTFKAMPCSQKLRILQDIGNLAYYEGGSKKRVELNDNQDKVLYELLNSKDKVTFD
QMRKALCLADSNSFNLEENRDFLIGNPTAVKMRSKNRFGKLWDEIPLEEQDLIIETIITADE
DDAVYEVIKKYDLTQEQRDFIVKNTILQSGTSMLCKEVSEKLVKRLEEIADLKYHEAVESLG
YKFADQTVEKYDLLPYYGKVLPGSTMEIDLSAPETNPEKHYGKISNPTVHVALNQTRVVVNA
LIKEYGKPSQIAIELSRDLKNNVEKKAEIARKQNQRAKENIAINDTISALYHTAFPGKSFYP
NRNDRMKYRLWSELGLGNKCIYCGKGISGAELFTKEIEIEHILPFSRTLLDAESNLTVAHSS
CNAFKAERSPFEAFGTNPSGYSWQEIIQRANQLKNTSKKNKFSPNAMDSFEKDSSFIARQLS
DNQYIAKAALRYLKCLVENPSDVWTTNGSMTKLLRDKWEMDSILCRKFTEKEVALLGLKPEQ
IGNYKKNRFDHRHHAIDAVVIGLTDRSMVQKLATKNSHKGNRIEIPEFPILRSDLIEKVKNI
VVSFKPDHGAEGKLSKETLLGKIKLHGKETFVCRENIVSLSEKNLDDIVDEIKSKVKDYVAK
HKGQKIEAVLSDFSKENGIKKVRCVNRVQTPIEITSGKISRYLSPEDYFAAVIWEIPGEKKT
FKAQYIRRNEVEKNSKGLNVVKPAVLENGKPHPAAKQVCLLHKDDYLEFSDKGKMYFCRIAG
YAATNNKLDIRPVYAVSYCADWINSTNETMLTGYWKPTPTQNWVSVNVLFDKQKARLVTVSP
IGRVFRK
SEQ ID NO: 373 
MSSKAIDSLEQLDLFKPQEYTLGDLGIKSIGWAILSGERIANAGVYLFETAEELNSTGNKL
ISKAAERGRKRRIRRMLDRKARRGRHIRYLLEREGLPTDELEEVVVHQSNRTLWDVRAEAVE
 
RKLTKQELAAVLFHLVRHGYFPNTKKLPPDDESDSADEEQGKINRATSRLREELKASDCKT
IGQFLAQNRDRQRNREGDYSNLMARKLVFEEALQILAFQRKQGHELSKDFEKTYLDVLMGQR
SGRSPKLGNCSLIPSELRAPSSAPSTEWFKFLQNLGNLQISNAYREEWSIDAPRRAQIIDAC
SQRSTSSYWQIRRDFQIPDEYRFNLVNYERRDPDVDLQEYLQQQERKTLANFRNWKQLEKII
GTGHPIQTLDEAARLITLIKDDEKLSDQLADLLPEASDKAITQLCELDFTTAAKISLEAMYR
ILPHMNQGMGFFDACQQESLPEIGVPPAGDRVPPFDEMYNPVVNRVLSQSRKLINAVIDEYG
MPAKIRVELARDLGKGRELRERIKLDQLDKSKQNDQRAEDFRAEFQQAPRGDQSLRYRLWKE
QNCTCPYSGRMIPVNSVLSEDTQIDHILPISQSFDNSLSNKVLCFTEENAQKSNRTPFEYLD
AADFQRLEAISGNWPEAKRNKLLHKSFGKVAEEWKSRALNDTRYLTSALADHLRHHLPDSKI
QTVNGRITGYLRKQWGLEKDRDKHTHHAVDAIVVACTTPAIVQQVTLYHQDIRRYKKLGEKR
PTPWPETFRQDVLDVEEEIFITRQPKKVSGGIQTKDTLRKHRSKPDRQRVALTKVKLADLER
LVEKDASNRNLYEHLKQCLEESGDQPTKAFKAPFYMPSGPEAKQRPILSKVTLLREKPEPPK
QLTELSGGRRYDSMAQGRLDIYRYKPGGKRKDEYRVVLQRMIDLMRGEENVHVFQKGVPYDQ
GPEIEQNYTFLFSLYFDDLVEFQRSADSEVIRGYYRTFNIANGQLKISTYLEGRQDFDFFGA
NRLAHFAKVQVNLLGKVIK
SEQ ID NO: 374 
MRSLRYRLALDLGSTSLGWALFRLDACNRPTAVIKAGVRIFSDGRNPKDGSSLAVTRRAARA
MRRRRDRLLKRKTRMQAKLVEHGFFPADAGKRKALEQLNPYALRAKGLQEALLPGEFARALF
HINQRRGFKSNRKTDKKDNDSGVLKKAIGQLRQQMAEQGSRTVGEYLWTRLQQGQGVRARYR
EKPYTTEEGKKRIDKSYDLYIDRAMIEQEFDALWAAQAAFNPTLFHEAARADLKDTLLHQRP
LRPVKPGRCTLLPEEERAPLALPSTQRFRIHQEVNHLRLLDENLREVALTLAQRDAVVTALE
TKAKLSFEQIRKLLKLSGSVQFNLEDAKRTELKGNATSAALARKELFGAAWSGFDEALQDEI
VWQLVTEEGEGALIAWLQTHTGVDEARAQAIVDVSLPEGYGNLSRKALARIVPALRAAVITY
DKAVQAAGFDHHSQLGFEYDASEVEDLVHPETGEIRSVFKQLPYYGKALQRHVAFGSGKPED
PDEKRYGKIANPTVHIGLNQVRMVVNALIRRYGRPTEVVIELARDLKQSREQKVEAQRRQAD
NQRRNARIRRSIAEVLGIGEERVRGSDIQKWICWEELSFDAADRRCPYSGVQISAAMLLSDE
VEVEHILPFSKTLDDSLNNRTVAMRQANRIKRNRTPWDARAEFEAQGWSYEDILQRAERMPL
RKRYRFAPDGYERWLGDDKDFLARALNDTRYLSRVAAEYLRLVCPGTRVIPGQLTALLRGKF
GLNDVLGLDGEKNRNDHRHHAVDACVIGVTDQGLMQRFATASAQARGDGLTRLVDGMPMPWP
TYRDHVERAVRHIWVSHRPDHGFEGAMMEETSYGIRKDGSIKQRRKADGSAGREISNLIRIH
EATQPLRHGVSADGQPLAYKGYVGGSNYCIEITVNDKGKWEGEVISTFRAYGVVRAGGMGRL
RNPHEGQNGRKLIMRLVIGDSVRLEVDGAERTMRIVKISGSNGQIFMAPIHEANVDARNTDK
QDAFTYTSKYAGSLQKAKTRRVTISPIGEVRDPGFKG
SEQ ID NO: 375 
MARPAFRAPRREHVNGWTPDPHRISKPFFILVSWHLLSRVVIDSSSGCFPGTSRDHTDKFAE
WECAVQPYRLSFDLGTNSIGWGLLNLDRQGKPREIRALGSRIFSDGRDPQDKASLAVARRLA
RQMRRRRDRYLTRRTRLMGALVRFGLMPADPAARKRLEVAVDPYLARERATRERLEPFEIGR
ALFHLNQRRGYKPVRTATKPDEEAGKVKEAVERLEAAIAAAGAPTLGAWFAWRKTRGETLRA
RLAGKGKEAAYPFYPARRMLEAEFDTLWAEQARHHPDLLTAEAREILRHRIFHQRPLKPPPV
GRCTLYPDDGRAPRALPSAQRLRLFQELASLRVIHLDLSERPLTPAERDRIVAFVQGRPPKA
GRKPGKVQKSVPFEKLRGLLELPPGTGFSLESDKRPELLGDETGARIAPAFGPGWTALPLEE
QDALVELLLTEAEPERAIAALTARWALDEATAAKLAGATLPDFHGRYGRRAVAELLPVLERE
TRGDPDGRVRPIRLDEAVKLLRGGKDHSDFSREGALLDALPYYGAVLERHVAFGTGNPADPE
EKRVGRVANPTVHIALNQLRHLVNAILARHGRPEEIVIELARDLKRSAEDRRREDKRQADNQ
KRNEERKRLILSLGERPTPRNLLKLRLWEEQGPVENRRCPYSGETISMRMLLSEQVDIDHIL
PFSVSLDDSAANKVVCLREANRIKRNRSPWEAFGHDSERWAGILARAEALPKNKRWRFAPDA
LEKLEGEGGLRARHLNDTRHLSRLAVEYLRCVCPKVRVSPGRLTALLRRRWGIDAILAEADG
PPPEVPAETLDPSPAEKNRADHRHHALDAVVIGCIDRSMVQRVQLAAASAEREAAAREDNIR
RVLEGFKEEPWDGFRAELERRARTIVVSHRPEHGIGGALHKETAYGPVDPPEEGFNLVVRKP
 
IDGLSKDEINSVRDPRLRRALIDRLAIRRRDANDPATALAKAAEDLAAQPASRGIRRVRVLK
KESNPIRVEHGGNPSGPRSGGPFHKLLLAGEVHHVDVALRADGRRWVGHWVTLFEAHGGRGA
DGAAAPPRLGDGERFLMRLHKGDCLKLEHKGRVRVMQVVKLEPSSNSVVVVEPHQVKTDRSK
HVKISCDQLRARGARRVTVDPLGRVRVHAPGARVGIGGDAGRTAMEPAEDIS
SEQ ID NO: 376 
MKRTSLRAYRLGVDLGANSLGWFVVWLDDHGQPEGLGPGGVRIFPDGRNPQSKQSNAAGRRL
ARSARRRRDRYLQRRGKLMGLLVKHGLMPADEPARKRLECLDPYGLRAKALDEVLPLHHVGR
ALFHLNQRRGLFANRAIEQGDKDASAIKAAAGRLQTSMQACGARTLGEFLNRRHQLRATVRA
RSPVGGDVQARYEFYPTRAMVDAEFEAIWAAQAPHHPTMTAEAHDTIREAIFSQRAMKRPSI
GKCSLDPATSQDDVDGFRCAWSHPLAQRFRIWQDVRNLAVVETGPTSSRLGKEDQDKVARAL
LQTDQLSFDEIRGLLGLPSDARFNLESDRRDHLKGDATGAILSARRHFGPAWHDRSLDRQID
IVALLESALDEAAIIASLGTTHSLDEAAAQRALSALLPDGYCRLGLRAIKRVLPLMEAGRTY
AEAASAAGYDHALLPGGKLSPTGYLPYYGQWLQNDVVGSDDERDTNERRWGRLPNPTVHIGI
GQLRRVVNELIRWHGPPAEITVELTRDLKLSPRRLAELEREQAENQRKNDKRTSLLRKLGLP
ASTHNLLKLRLWDEQGDVASECPYTGEAIGLERLVSDDVDIDHLIPFSISWDDSAANKVVCM
RYANREKGNRTPFEAFGHRQGRPYDWADIAERAARLPRGKRWRFGPGARAQFEELGDFQARL
LNETSWLARVAKQYLAAVTHPHRIHVLPGRLTALLRATWELNDLLPGSDDRAAKSRKDHRHH
AIDALVAALTDQALLRRMANAHDDTRRKIEVLLPWPTFRIDLETRLKAMLVSHKPDHGLQAR
LHEDTAYGTVEHPETEDGANLVYRKTFVDISEKEIDRIRDRRLRDLVRAHVAGERQQGKTLK
AAVLSFAQRRDIAGHPNGIRHVRLTKSIKPDYLVPIRDKAGRIYKSYNAGENAFVDILQAES
GRWIARATTVFQANQANESHDAPAAQPIMRVFKGDMLRIDHAGAEKFVKIVRLSPSNNLLYL
VEHHQAGVFQTRHDDPEDSFRWLFASFDKLREWNAELVRIDTLGQPWRRKRGLETGSEDATR
IGWTRPKKWP
SEQ ID NO: 377 
MERIFGFDIGTTSIGFSVIDYSSTQSAGNIQRLGVRIFPEARDPDGTPLNQQRRQKRMMRRQ
LRRRRIRRKALNETLHEAGFLPAYGSADWPVVMADEPYELRRRGLEEGLSAYEFGRAIYHLA
QHRHFKGRELEESDTPDPDVDDEKEAANERAATLKALKNEQTTLGAWLARRPPSDRKRGIHA
HRNVVAEEFERLWEVQSKFHPALKSEEMRARISDTIFAQRPVFWRKNTLGECRFMPGEPLCP
KGSWLSQQRRMLEKLNNLAIAGGNARPLDAEERDAILSKLQQQASMSWPGVRSALKALYKQR
GEPGAEKSLKFNLELGGESKLLGNALEAKLADMFGPDWPAHPRKQEIRHAVHERLWAADYGE
TPDKKRVIILSEKDRKAHREAAANSFVADFGITGEQAAQLQALKLPTGWEPYSIPALNLFLA
ELEKGERFGALVNGPDWEGWRRTNFPHRNQPTGEILDKLPSPASKEERERISQLRNPTVVRT
QNELRKVVNNLIGLYGKPDRIRIEVGRDVGKSKREREEIQSGIRRNEKQRKKATEDLIKNGI
ANPSRDDVEKWILWKEGQERCPYTGDQIGFNALFREGRYEVEHIWPRSRSFDNSPRNKTLCR
KDVNIEKGNRMPFEAFGHDEDRWSAIQIRLQGMVSAKGGTGMSPGKVKRFLAKTMPEDFAAR
QLNDTRYAAKQILAQLKRLWPDMGPEAPVKVEAVTGQVTAQLRKLWTLNNILADDGEKTRAD
HRHHAIDALTVACTHPGMTNKLSRYWQLRDDPRAEKPALTPPWDTIRADAEKAVSEIVVSHR
VRKKVSGPLHKETTYGDTGTDIKTKSGTYRQFVTRKKIESLSKGELDEIRDPRIKEIVAAHV
AGRGGDPKKAFPPYPCVSPGGPEIRKVRLTSKQQLNLMAQTGNGYADLGSNHHIAIYRLPDG
KADFEIVSLFDASRRLAQRNPIVQRTRADGASFVMSLAAGEAIMIPEGSKKGIWIVQGVWAS
GQVVLERDTDADHSTTTRPMPNPILKDDAKKVSIDPIGRVRPSND
SEQ ID NO: 378 
MNKRILGLDTGTNSLGWAVVDWDEHAQSYELIKYGDVIFQEGVKIEKGIESSKAAERSGYKA
IRKQYFRRRLRKIQVLKVLVKYHLCPYLSDDDLRQWHLQKQYPKSDELMLWQRTSDEEGKNP
YYDRHRCLHEKLDLTVEADRYTLGRALYHLTQRRGFLSNRLDTSADNKEDGVVKSGISQLST
EMEEAGCEYLGDYFYKLYDAQGNKVRIRQRYTDRNKHYQHEFDAICEKQELSSELIEDLQRA
IFFQLPLKSQRHGVGRCTFERGKPRCADSHPDYEEFRMLCFVNNIQVKGPHDLELRPLTYEE
REKIEPLFFRKSKPNFDFEDIAKALAGKKNYAWIHDKEERAYKFNYRMTQGVPGCPTIAQLK
 
SIFGDDWKTGIAETYTLIQKKNGSKSLQEMVDDVWNVLYSFSSVEKLKEFAHHKLQLDEESA
EKFAKIKLSHSFAALSLKAIRKFLPFLRKGMYYTHASFFANIPTIVGKEIWNKEQNRKYIME
NVGELVFNYQPKHREVQGTIEMLIKDFLANNFELPAGATDKLYHPSMIETYPNAQRNEFGIL
QLGSPRTNAIRNPMAMRSLHILRRVVNQLLKESIIDENTEVHVEYARELNDANKRRAIADRQ
KEQDKQHKKYGDEIRKLYKEETGKDIEPTQTDVLKFQLWEEQNHHCLYTGEQIGITDFIGSN
PKFDIEHTIPQSVGGDSTQMNLTLCDNRFNREVKKAKLPTELANHEEILTRIEPWKNKYEQL
VKERDKQRTFAGMDKAVKDIRIQKRHKLQMEIDYWRGKYERFTMTEVPEGFSRRQGTGIGLI
SRYAGLYLKSLFHQADSRNKSNVYVVKGVATAEFRKMWGLQSEYEKKCRDNHSHHCMDAITI
ACIGKREYDLMAEYYRMEETFKQGRGSKPKFSKPWATFTEDVLNIYKNLLVVHDTPNNMPKH
TKKYVQTSIGKVLAQGDTARGSLHLDTYYGAIERDGEIRYVVRRPLSSFTKPEELENIVDET
VKRTIKEAIADKNFKQAIAEPIYMNEEKGILIKKVRCFAKSVKQPINIRQHRDLSKKEYKQQ
YHVMNENNYLLAIYEGLVKNKVVREFEIVSYIEAAKYYKRSQDRNIFSSIVPTHSTKYGLPL
KTKLLMGQLVLMFEENPDEIQVDNTKDLVKRLYKVVGIEKDGRIKFKYHQEARKEGLPIFST
PYKNNDDYAPIFRQSINNINILVDGIDFTIDILGKVTLKE
SEQ ID NO: 379 
MNYKMGLDIGIASVGWAVINLDLKRIEDLGVRIFDKAEHPQNGESLALPRRIARSARRRLRR
RKHRLERIRRLLVSENVLTKEEMNLLFKQKKQIDVWQLRVDALERKLNNDELARVLLHLAKR
RGFKSNRKSERNSKESSEFLKNIEENQSILAQYRSVGEMIVKDSKFAYHKRNKLDSYSNMIA
RDDLEREIKLIFEKQREFNNPVCTERLEEKYLNIWSSQRPFASKEDIEKKVGFCTFEPKEKR
APKATYTFQSFIVWEHINKLRLVSPDETRALTEIERNLLYKQAFSKNKMTYYDIRKLLNLSD
DIHFKGLLYDPKSSLKQIENIRFLELDSYHKIRKCIENVYGKDGIRMFNETDIDTFGYALTI
FKDDEDIVAYLQNEYITKNGKRVSNLANKVYDKSLIDELLNLSFSKFAHLSMKAIRNILPYM
EQGEIYSKACELAGYNFTGPKKKEKALLLPVIPNIANPVVMRALTQSRKVVNAIIKKYGSPV
SIHIELARDLSHSFDERKKIQKDQTENRKKNETAIKQLIEYELTKNPTGLDIVKFKLWSEQQ
GRCMYSLKPIELERLLEPGYVEVDHILPYSRSLDDSYANKVLVLTKENREKGNHTPVEYLGL
GSERWKKFEKFVLANKQFSKKKKQNLLRLRYEETEEKEFKERNLNDTRYISKFFANFIKEHL
KFADGDGGQKVYTINGKITAHLRSRWDFNKNREESDLHHAVDAVIVACATQGMIKKITEFYK
AREQNKESAKKKEPIFPQPWPHFADELKARLSKFPQESIEAFALGNYDRKKLESLRPVFVSR
MPKRSVTGAAHQETLRRCVGIDEQSGKIQTAVKTKLSDIKLDKDGHFPMYQKESDPRTYEAI
RQRLLEHNNDPKKAFQEPLYKPKKNGEPGPVIRTVKIIDTKNKVVHLDGSKTVAYNSNIVRT
DVFEKDGKYYCVPVYTMDIMKGTLPNKAIEANKPYSEWKEMTEEYTFQFSLFPNDLVRIVLP
REKTIKTSTNEEIIIKDIFAYYKTIDSATGGLELISHDRNFSLRGVGSKTLKRFEKYQVDVL
GNIHKVKGEKRVGLAAPTNQKKGKTVDSLQSVSD
SEQ ID NO: 380 
MRRLGLDLGTNSIGWCLLDLGDDGEPVSIFRTGARIFSDGRDPKSLGSLKATRREARLTRRR
RDRFIQRQKNLINALVKYGLMPADEIQRQALAYKDPYPIRKKALDEAIDPYEMGRAIFHINQ
RRGFKSNRKSADNEAGVVKQSIADLEMKLGEAGARTIGEFLADRQATNDTVRARRLSGTNAL
YEFYPDRYMLEQEFDTLWAKQAAFNPSLYIEAARERLKEIVFFQRKLKPQEVGRCIFLSDED
RISKALPSFQRFRIYQELSNLAWIDHDGVAHRITASLALRDHLFDELEHKKKLTFKAMRAIL
RKQGVVDYPVGFNLESDNRDHLIGNLTSCIMRDAKKMIGSAWDRLDEEEQDSFILMLQDDQK
GDDEVRSILTQQYGLSDDVAEDCLDVRLPDGHGSLSKKAIDRILPVLRDQGLIYYDAVKEAG
LGEANLYDPYAALSDKLDYYGKALAGHVMGASGKFEDSDEKRYGTISNPTVHIALNQVRAVV
NELIRLHGKPDEVVIEIGRDLPMGADGKRELERFQKEGRAKNERARDELKKLGHIDSRESRQ
KFQLWEQLAKEPVDRCCPFTGKMMSISDLFSDKVEIEHLLPFSLTLDDSMANKTVCFRQANR
DKGNRAPFDAFGNSPAGYDWQEILGRSQNLPYAKRWRFLPDAMKRFEADGGFLERQLNDTRY
ISRYTTEYISTIIPKNKIWVVTGRLTSLLRGFWGLNSILRGHNTDDGTPAKKSRDDHRHHAI
DAIVVGMTSRGLLQKVSKAARRSEDLDLTRLFEGRIDPWDGFRDEVKKHIDAIIVSHRPRKK
SQGALHNDTAYGIVEHAENGASTVVHRVPITSLGKQSDIEKVRDPLIKSALLNETAGLSGKS
FENAVQKWCADNSIKSLRIVETVSIIPITDKEGVAYKGYKGDGNAYMDIYQDPTSSKWKGEI
 
VSRFDANQKGFIPSWQSQFPTARLIMRLRINDLLKLQDGEIEEIYRVQRLSGSKILMAPHTE
ANVDARDRDKNDTFKLTSKSPGKLQSASARKVHISPTGLIREG
SEQ ID NO: 381 
MKNILGLDLGLSSIGWSVIRENSEEQELVAMGSRVVSLTAAELSSFTQGNGVSINSQRTQKR
TQRKGYDRYQLRRTLLRNKLDTLGMLPDDSLSYLPKLQLWGLRAKAVTQRIELNELGRVLLH
LNQKRGYKSIKSDFSGDKKITDYVKTVKTRYDELKEMRLTIGELFFRRLTENAFFRCKEQVY
PRQAYVEEFDCIMNCQRKFYPDILTDETIRCIRDEIIYYQRPLKSCKYLVSRCEFEKRFYLN
AAGKKTEAGPKVSPRTSPLFQVCRLWESINNIVVKDRRNEIVFISAEQRAALFDFLNTHEKL
KGSDLLKLLGLSKTYGYRLGEQFKTGIQGNKTRVEIERALGNYPDKKRLLQFNLQEESSSMV
NTETGEIIPMISLSFEQEPLYRLWHVLYSIDDREQLQSVLRQKFGIDDDEVLERLSAIDLVK
AGFGNKSSKAIRRILPFLQLGMNYAEACEAAGYNHSNNYTKAENEARALLDRLPAIKKNELR
QPVVEKILNQMVNVVNALMEKYGRFDEIRVELARELKQSKEERSNTYKSINKNQRENEQIAK
RIVEYGVPTRSRIQKYKMWEESKHCCIYCGQPVDVGDFLRGFDVEVEHIIPKSLYFDDSFAN
KVCSCRSCNKEKNNRTAYDYMKSKGEKALSDYVERVNTMYTNNQISKTKWQNLLTPVDKISI
DFIDRQLRESQYIARKAKEILTSICYNVTATSGSVTSFLRHVWGWDTVLHDLNFDRYKKVGL
TEVIEVNHRGSVIRREQIKDWSKRFDHRHHAIDALTIACTKQAYIQRLNNLRAEEGPDFNKM
SLERYIQSQPHFSVAQVREAVDRILVSFRAGKRAVTPGKRYIRKNRKRISVQSVLIPRGALS
EESVYGVIHVWEKDEQGHVIQKQRAVMKYPITSINREMLDKEKVVDKRIHRILSGRLAQYND
NPKEAFAKPVYIDKECRIPIRTVRCFAKPAINTLVPLKKDDKGNPVAWVNPGNNHHVAIYRD
EDGKYKERTVTFWEAVDRCRVGIPAIVTQPDTIWDNILQRNDISENVLESLPDVKWQFVLSL
QQNEMFILGMNEEDYRYAMDQQDYALLNKYLYRVQKLSKSDYSFRYHTETSVEDKYDGKPNL
KLSMQMGKLKRVSIKSLLGLNPHKVHISVLGEIKEIS
SEQ ID NO: 382 
MAEKQHRWGLDIGTNSIGWAVIALIEGRPAGLVATGSRIFSDGRNPKDGSSLAVERRGPRQM
RRRRDRYLRRRDRFMQALINVGLMPGDAAARKALVTENPYVLRQRGLDQALTLPEFGRALFH
LNQRRGFQSNRKTDRATAKESGKVKNAIAAFRAGMGNARTVGEALARRLEDGRPVRARMVGQ
GKDEHYELYIAREWIAQEFDALWASQQRFHAEVLADAARDRLRAILLFQRKLLPVPVGKCFL
EPNQPRVAAALPSAQRFRLMQELNHLRVMTLADKRERPLSFQERNDLLAQLVARPKCGFDML
RKIVFGANKEAYRFTIESERRKELKGCDTAAKLAKVNALGTRWQALSLDEQDRLVCLLLDGE
NDAVLADALREHYGLTDAQIDTLLGLSFEDGHMRLGRSALLRVLDALESGRDEQGLPLSYDK
AVVAAGYPAHTADLENGERDALPYYGELLWRYTQDAPTAKNDAERKFGKIANPTVHIGLNQL
RKLVNALIQRYGKPAQIVVELARNLKAGLEEKERIKKQQTANLERNERIRQKLQDAGVPDNR
ENRLRMRLFEELGQGNGLGTPCIYSGRQISLQRLFSNDVQVDHILPFSKTLDDSFANKVLAQ
HDANRYKGNRGPFEAFGANRDGYAWDDIRARAAVLPRNKRNRFAETAMQDWLHNETDFLARQ
LTDTAYLSRVARQYLTAICSKDDVYVSPGRLTAMLRAKWGLNRVLDGVMEEQGRPAVKNRDD
HRHHAIDAVVIGATDRAMLQQVATLAARAREQDAERLIGDMPTPWPNFLEDVRAAVARCVVS
HKPDHGPEGGLHNDTAYGIVAGPFEDGRYRVRHRVSLFDLKPGDLSNVRCDAPLQAELEPIF
EQDDARAREVALTALAERYRQRKVWLEELMSVLPIRPRGEDGKTLPDSAPYKAYKGDSNYCY
ELFINERGRWDGELISTFRANQAAYRRFRNDPARFRRYTAGGRPLLMRLCINDYIAVGTAAE
RTIFRVVKMSENKITLAEHFEGGTLKQRDADKDDPFKYLTKSPGALRDLGARRIFVDLIGRV
LDPGIKGD
SEQ ID NO: 383 
MIERILGVDLGISSLGWAIVEYDKDDEAANRIIDCGVRLFTAAETPKKKESPNKARREARGI
RRVLNRRRVRMNMIKKLFLRAGLIQDVDLDGEGGMFYSKANRADVWELRHDGLYRLLKGDEL
ARVLIHIAKHRGYKFIGDDEADEESGKVKKAGVVLRQNFEAAGCRTVGEWLWRERGANGKKR
NKHGDYEISIHRDLLVEEVEAIFVAQQEMRSTIATDALKAAYREIAFFVRPMQRIEKMVGHC
TYFPEERRAPKSAPTAEKFIAISKFFSTVIIDNEGWEQKIIERKTLEELLDFAVSREKVEFR
HLRKFLDLSDNEIFKGLHYKGKPKTAKKREATLFDPNEPTELEFDKVEAEKKAWISLRGAAK
 
LREALGNEFYGRFVALGKHADEATKILTYYKDEGQKRRELTKLPLEAEMVERLVKIGFSDFL
KLSLKAIRDILPAMESGARYDEAVLMLGVPHKEKSAILPPLNKTDIDILNPTVIRAFAQFRK
VANALVRKYGAFDRVHFELAREINTKGEIEDIKESQRKNEKERKEAADWIAETSFQVPLTRK
NILKKRLYIQQDGRCAYTGDVIELERLFDEGYCEIDHILPRSRSADDSFANKVLCLARANQQ
KTDRTPYEWFGHDAARWNAFETRTSAPSNRVRTGKGKIDRLLKKNFDENSEMAFKDRNLNDT
RYMARAIKTYCEQYWVFKNSHTKAPVQVRSGKLTSVLRYQWGLESKDRESHTHHAVDAIIIA
FSTQGMVQKLSEYYRFKETHREKERPKLAVPLANFRDAVEEATRIENTETVKEGVEVKRLLI
SRPPRARVTGQAHEQTAKPYPRIKQVKNKKKWRLAPIDEEKFESFKADRVASANQKNFYETS
TIPRVDVYHKKGKFHLVPIYLHEMVLNELPNLSLGTNPEAMDENFFKFSIFKDDLISIQTQG
TPKKPAKIIMGYFKNMHGANMVLSSINNSPCEGFTCTPVSMDKKHKDKCKLCPEENRIAGRC
LQGFLDYWSQEGLRPPRKEFECDQGVKFALDVKKYQIDPLGYYYEVKQEKRLGTIPQMRSAK
KLVKK
SEQ ID NO: 384 
MNNSIKSKPEVTIGLDLGVGSVGWAIVDNETNIIHHLGSRLFSQAKTAEDRRSFRGVRRLIR
RRKYKLKRFVNLIWKYNSYFGFKNKEDILNNYQEQQKLHNTVLNLKSEALNAKIDPKALSWI
LHDYLKNRGHFYEDNRDFNVYPTKELAKYFDKYGYYKGIIDSKEDNDNKLEEELTKYKFSNK
HWLEEVKKVLSNQTGLPEKFKEEYESLFSYVRNYSEGPGSINSVSPYGIYHLDEKEGKVVQK
YNNIWDKTIGKCNIFPDEYRAPKNSPIAMIFNEINELSTIRSYSIYLTGWFINQEFKKAYLN
KLLDLLIKTNGEKPIDARQFKKLREETIAESIGKETLKDVENEEKLEKEDHKWKLKGLKLNT
NGKIQYNDLSSLAKFVHKLKQHLKLDFLLEDQYATLDKINFLQSLFVYLGKHLRYSNRVDSA
NLKEFSDSNKLFERILQKQKDGLFKLFEQTDKDDEKILAQTHSLSTKAMLLAITRMTNLDND
EDNQKNNDKGWNFEAIKNFDQKFIDITKKNNNLSLKQNKRYLDDRFINDAILSPGVKRILRE
ATKVFNAILKQFSEEYDVTKVVIELARELSEEKELENTKNYKKLIKKNGDKISEGLKALGIS
EDEIKDILKSPTKSYKFLLWLQQDHIDPYSLKEIAFDDIFTKTEKFEIDHIIPYSISFDDSS
SNKLLVLAESNQAKSNQTPYEFISSGNAGIKWEDYEAYCRKFKDGDSSLLDSTQRSKKFAKM
MKTDTSSKYDIGFLARNLNDTRYATIVFRDALEDYANNHLVEDKPMFKVVCINGSVTSFLRK
NFDDSSYAKKDRDKNIHHAVDASIISIFSNETKTLFNQLTQFADYKLFKNTDGSWKKIDPKT
GVVTEVTDENWKQIRVRNQVSEIAKVIEKYIQDSNIERKARYSRKIENKTNISLFNDTVYSA
KKVGYEDQIKRKNLKTLDIHESAKENKNSKVKRQFVYRKLVNVSLLNNDKLADLFAEKEDIL
MYRANPWVINLAEQIFNEYTENKKIKSQNVFEKYMLDLTKEFPEKFSEFLVKSMLRNKTAII
YDDKKNIVHRIKRLKMLSSELKENKLSNVIIRSKNQSGTKLSYQDTINSLALMIMRSIDPTA
KKQYIRVPLNTLNLHLGDHDFDLHNMDAYLKKPKFVKYLKANEIGDEYKPWRVLTSGTLLIH
KKDKKLMYISSFQNLNDVIEIKNLIETEYKENDDSDSKKKKKANRFLMTLSTILNDYILLDA
KDNFDILGLSKNRIDEILNSKLGLDKIVK
SEQ ID NO: 385 
MGGSEVGTVPVTWRLGVDVGERSIGLAAVSYEEDKPKEILAAVSWIHDGGVGDERSGASRLA
LRGMARRARRLRRFRRARLRDLDMLLSELGWTPLPDKNVSPVDAWLARKRLAEEYVVDETER
RRLLGYAVSHMARHRGWRNPWTTIKDLKNLPQPSDSWERTRESLEARYSVSLEPGTVGQWAG
YLLQRAPGIRLNPTQQSAGRRAELSNATAFETRLRQEDVLWELRCIADVQGLPEDVVSNVID
AVFCQKRPSVPAERIGRDPLDPSQLRASRACLEFQEYRIVAAVANLRIRDGSGSRPLSLEER
NAVIEALLAQTERSLTWSDIALEILKLPNESDLTSVPEEDGPSSLAYSQFAPFDETSARIAE
FIAKNRRKIPTFAQWWQEQDRTSRSDLVAALADNSIAGEEEQELLVHLPDAELEALEGLALP
SGRVAYSRLTLSGLTRVMRDDGVDVHNARKTCFGVDDNWRPPLPALHEATGHPVVDRNLAIL
RKFLSSATMRWGPPQSIVVELARGASESRERQAEEEAARRAHRKANDRIRAELRASGLSDPS
PADLVRARLLELYDCHCMYCGAPISWENSELDHIVPRTDGGSNRHENLAITCGACNKEKGRR
PFASWAETSNRVQLRDVIDRVQKLKYSGNMYWTRDEFSRYKKSVVARLKRRTSDPEVIQSIE
STGYAAVALRDRLLSYGEKNGVAQVAVFRGGVTAEARRWLDISIERLFSRVAIFAQSTSTKR
LDRRHHAVDAVVLTTLTPGVAKTLADARSRRVSAEFWRRPSDVNRHSTEEPQSPAYRQWKES
CSGLGDLLISTAARDSIAVAAPLRLRPTGALHEETLRAFSEHTVGAAWKGAELRRIVEPEVY
 
AAFLALTDPGGRFLKVSPSEDVLPADENRHIVLSDRVLGPRDRVKLFPDDRGSIRVRGGAAY
IASFHHARVFRWGSSHSPSFALLRVSLADLAVAGLLRDGVDVFTAELPPWTPAWRYASIALV
KAVESGDAKQVGWLVPGDELDFGPEGVTTAAGDLSMFLKYFPERHWVVTGFEDDKRINLKPA
FLSAEQAEVLRTERSDRPDTLTEAGEILAQFFPRCWRATVAKVLCHPGLTVIRRTALGQPRW
RRGHLPYSWRPWSADPWSGGTP
SEQ ID NO: 386

MHNKKNITIGFDLGIASIGWAIIDSTTSKILDWGTRTFEERKTANERRAFRSTRRNIRRKAY
RNQRFINLILKYKDLFELKNISDIQRANKKDTENYEKIISFFTEIYKKCAAKHSNILEVKVK
ALDSKIEKLDLIWILHDYLENRGFFYDLEEENVADKYEGIEHPSILLYDFFKKNGFFKSNSS
IPKDLGGYSFSNLQWVNEIKKLFEVQEINPEFSEKFLNLFTSVRDYAKGPGSEHSASEYGIF
QKDEKGKVFKKYDNIWDKTIGKCSFFVEENRSPVNYPSYEIFNLLNQLINLSTDLKTTNKKI
WQLSSNDRNELLDELLKVKEKAKIISISLKKNEIKKIILKDFGFEKSDIDDQDTIEGRKIIK
EEPTTKLEVTKHLLATIYSHSSDSNWININNNILEFLPYLDAICIILDREKSRGQDEVLKKLT
EKNIFEVLKIDREKQLDFVKSIFSNTKFNFKKIGNFSLKAIREFLPKMFEQNKNSEYLKWKD
EEIRRKWEEQKSKLGKTDKKTKYLNPRIFQDEIISPGTKNTFEQAVLVLNQIIKKYSKENII
DAIIIESPREKNDKKTIEEIKKRNKKGKGKTLEKLFQILNLENKGYKLSDLETKPAKLLDRL
RFYHQQDGIDLYTLDKINIDQLINGSQKYEIEHIIPYSMSYDNSQANKILTEKAENLKKGKL
IASEYIKRNGDEFYNKYYEKAKELFINKYKKNKKLDSYVDLDEDSAKNRFRFLTLQDYDEFQ
VEFLARNLNDTRYSTKLFYHALVEHFENNEFFTYIDENSSKHKVKISTIKGHVTKYFRAKPV
QKNNGPNENLNNNKPEKIEKNRENNEHHAVDAAIVAIIGNKNPQIANLLTLADNKTDKKFLL
HDENYKENIETGELVKIPKFEVDKLAKVEDLKKIIQEKYEEAKKHTAIKFSRKTRTILNGGL
SDETLYGFKYDEKEDKYFKIIKKKLVTSKNEELKKYFENPFGKKADGKSEYTVLMAQSHLSE
FNKLKEIFEKYNGFSNKTGNAFVEYMNDLALKEPTLKAEIESAKSVEKLLYYNFKPSDQFTY
HDNINNKSFKRFYKNIRIIEYKSIPIKFKILSKHDGGKSFKDTLFSLYSLVYKVYENGKESY
KSIPVTSQMRNFGIDEFDFLDENLYNKEKLDIYKSDFAKPIPVNCKPVFVLKKGSILKKKSL
DIDDFKETKETEEGNYYFISTISKRFNRDTAYGLKPLKLSVVKPVAEPSTNPIFKEYIPIHL
DELGNEYPVKIKEHTDDEKLMCTIK

### Nucleic Acids Encoding Cas9 Molecules

Nucleic acids encoding the Cas9 molecules or Cas9 polypeptides, e.g., an eaCas9 molecule or eaCas9 polypeptide are provided herein.

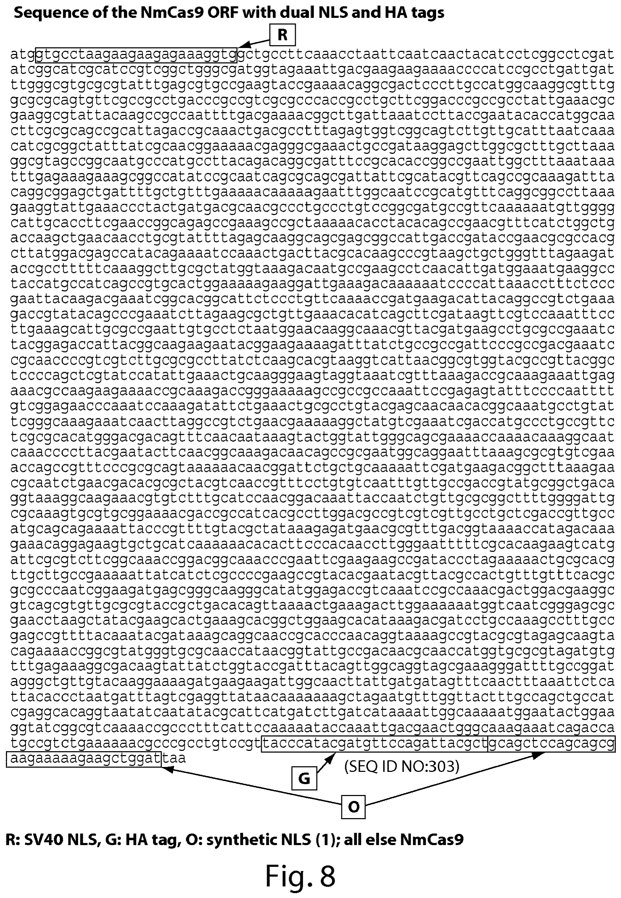

Exemplary nucleic acids encoding Cas9 molecules or Cas9 polypeptides are described in Cong et al., SCIENCE 2013, 399(6121):819-823; Wang et al., CELL 2013, 153(4):910-918; Mali et al., SCIENCE 2013, 399(6121):823-826; Jinek et al., SCIENCE 2012, 337(6096):816-821. Another exemplary nucleic acid encoding a Cas9 molecule or Cas9 polypeptide is shown in **Fig. 8**.

In an embodiment, a nucleic acid encoding a Cas9 molecule or Cas9 polypeptide can be a synthetic nucleic acid sequence. For example, the synthetic nucleic acid molecule can be chemically modified, e.g., as described in Section VIII. In an embodiment, the Cas9 mRNA has one or more (e.g., all of the following properties: it is capped, polyadenylated, substituted with 5-methylcytidine and/or pseudouridine.

In addition, or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

In addition, or alternatively, a nucleic acid encoding a Cas9 molecule or Cas9 polypeptide may comprise a nuclear localization sequence (NLS). Nuclear localization sequences are known in the art.

Provided below is an exemplary codon optimized nucleic acid sequence encoding a Cas9 molecule of *S. pyogenes.* 
ATGGATAAAA AGTACAGCAT CGGGCTGGAC ATCGGTACAA ACTCAGTGGG
GTGGGCCGTG ATTACGGACG AGTACAAGGT ACCCTCCAAA AAATTTAAAG
TGCTGGGTAA CACGGACAGA CACTCTATAA AGAAAAATCT TATTGGAGCC
TTGCTGTTCG ACTCAGGCGA GACAGCCGAA GCCACAAGGT TGAAGCGGAC
CGCCAGGAGG CGGTATACCA GGAGAAAGAA CCGCATATGC TACCTGCAAG
AAATCTTCAG TAACGAGATG GCAAAGGTTG ACGATAGCTT TTTCCATCGC
CTGGAAGAAT CCTTTCTTGT TGAGGAAGAC AAGAAGCACG AACGGCACCC
CATCTTTGGC AATATTGTCG ACGAAGTGGC ATATCACGAA AAGTACCCGA
CTATCTACCA CCTCAGGAAG AAGCTGGTGG ACTCTACCGA TAAGGCGGAC
CTCAGACTTA TTTATTTGGC ACTCGCCCAC ATGATTAAAT TTAGAGGACA
TTTCTTGATC GAGGGCGACC TGAACCCGGA CAACAGTGAC GTCGATAAGC
TGTTCATCCA ACTTGTGCAG ACCTACAATC AACTGTTCGA AGAAAACCCT
ATAAATGCTT CAGGAGTCGA CGCTAAAGCA ATCCTGTCCG CGCGCCTCTC
AAAATCTAGA AGACTTGAGA ATCTGATTGC TCAGTTGCCC GGGGAAAAGA
AAAATGGATT GTTTGGCAAC CTGATCGCCC TCAGTCTCGG ACTGACCCCA
AATTTCAAAA GTAACTTCGA CCTGGCCGAA GACGCTAAGC TCCAGCTGTC
CAAGGACACA TACGATGACG ACCTCGACAA TCTGCTGGCC CAGATTGGGG
ATCAGTACGC CGATCTCTTT TTGGCAGCAA AGAACCTGTC CGACGCCATC
CTGTTGAGCG ATATCTTGAG AGTGAACACC GAAATTACTA AAGCACCCCT
TAGCGCATCT ATGATCAAGC GGTACGACGA GCATCATCAG GATCTGACCC
TGCTGAAGGC TCTTGTGAGG CAACAGCTCC CCGAAAAATA CAAGGAAATC
TTCTTTGACC AGAGCAAAAA CGGCTACGCT GGCTATATAG ATGGTGGGGC
CAGTCAGGAG GAATTCTATA AATTCATCAA GCCCATTCTC GAGAAAATGG
ACGGCACAGA GGAGTTGCTG GTCAAACTTA ACAGGGAGGA CCTGCTGCGG
AAGCAGCGGA CCTTTGACAA CGGGTCTATC CCCCACCAGA TTCATCTGGG
CGAACTGCAC GCAATCCTGA GGAGGCAGGA GGATTTTTAT CCTTTTCTTA
AAGATAACCG CGAGAAAATA GAAAAGATTC TTACATTCAG GATCCCGTAC
TACGTGGGAC CTCTCGCCCG GGGCAATTCA CGGTTTGCCT GGATGACAAG
GAAGTCAGAG GAGACTATTA CACCTTGGAA CTTCGAAGAA GTGGTGGACA
AGGGTGCATC TGCCCAGTCT TTCATCGAGC GGATGACAAA TTTTGACAAG
AACCTCCCTA ATGAGAAGGT GCTGCCCAAA CATTCTCTGC TCTACGAGTA
CTTTACCGTC TACAATGAAC TGACTAAAGT CAAGTACGTC ACCGAGGGAA
TGAGGAAGCC GGCATTCCTT AGTGGAGAAC AGAAGAAGGC GATTGTAGAC
CTGTTGTTCA AGACCAACAG GAAGGTGACT GTGAAGCAAC TTAAAGAAGA
CTACTTTAAG AAGATCGAAT GTTTTGACAG TGTGGAAATT TCAGGGGTTG
AAGACCGCTT CAATGCGTCA TTGGGGACTT ACCATGATCT TCTCAAGATC
 
ATAAAGGACA AAGACTTCCT GGACAACGAA GAAAATGAGG ATATTCTCGA
AGACATCGTC CTCACCCTGA CCCTGTTCGA AGACAGGGAA ATGATAGAAG
AGCGCTTGAA AACCTATGCC CACCTCTTCG ACGATAAAGT TATGAAGCAG
CTGAAGCGCA GGAGATACAC AGGATGGGGA AGATTGTCAA GGAAGCTGAT
CAATGGAATT AGGGATAAAC AGAGTGGCAA GACCATACTG GATTTCCTCA
AATCTGATGG CTTCGCCAAT AGGAACTTCA TGCAACTGAT TCACGATGAC
TCTCTTACCT TCAAGGAGGA CATTCAAAAG GCTCAGGTGA GCGGGCAGGG
AGACTCCCTT CATGAACACA TCGCGAATTT GGCAGGTTCC CCCGCTATTA
AAAAGGGCAT CCTTCAAACT GTCAAGGTGG TGGATGAATT GGTCAAGGTA
ATGGGCAGAC ATAAGCCAGA AAATATTGTG ATCGAGATGG CCCGCGAAAA
CCAGACCACA CAGAAGGGCC AGAAAAATAG TAGAGAGCGG ATGAAGAGGA
TCGAGGAGGG CATCAAAGAG CTGGGATCTC AGATTCTCAA AGAACACCCC
GTAGAAAACA CACAGCTGCA GAACGAAAAA TTGTACTTGT ACTATCTGCA
GAACGGCAGA GACATGTACG TCGACCAAGA ACTTGATATT AATAGACTGT
CCGACTATGA CGTAGACCAT ATCGTGCCCC AGTCCTTCCT GAAGGACGAC
TCCATTGATA ACAAAGTCTT GACAAGAAGC GACAAGAACA GGGGTAAAAG
TGATAATGTG CCTAGCGAGG AGGTGGTGAA AAAAATGAAG AACTACTGGC
GACAGCTGCT TAATGCAAAG CTCATTACAC AACGGAAGTT CGATAATCTG
ACGAAAGCAG AGAGAGGTGG CTTGTCTGAG TTGGACAAGG CAGGGTTTAT
TAAGCGGCAG CTGGTGGAAA CTAGGCAGAT CACAAAGCAC GTGGCGCAGA
TTTTGGACAG CCGGATGAAC ACAAAATACG ACGAAAATGA TAAACTGATA
CGAGAGGTCA AAGTTATCAC GCTGAAAAGC AAGCTGGTGT CCGATTTTCG
GAAAGACTTC CAGTTCTACA AAGTTCGCGA GATTAATAAC TACCATCATG
CTCACGATGC GTACCTGAAC GCTGTTGTCG GGACCGCCTT GATAAAGAAG
TACCCAAAGC TGGAATCCGA GTTCGTATAC GGGGATTACA AAGTGTACGA
TGTGAGGAAA ATGATAGCCA AGTCCGAGCA GGAGATTGGA AAGGCCACAG
CTAAGTACTT CTTTTATTCT AACATCATGA ATTTTTTTAA GACGGAAATT
ACCCTGGCCA ACGGAGAGAT CAGAAAGCGG CCCCTTATAG AGACAAATGG
TGAAACAGGT GAAATCGTCT GGGATAAGGG CAGGGATTTC GCTACTGTGA
GGAAGGTGCT GAGTATGCCA CAGGTAAATA TCGTGAAAAA AACCGAAGTA
CAGACCGGAG GATTTTCCAA GGAAAGCATT TTGCCTAAAA GAAACTCAGA
CAAGCTCATC GCCCGCAAGA AAGATTGGGA CCCTAAGAAA TACGGGGGAT
TTGACTCACC CACCGTAGCC TATTCTGTGC TGGTGGTAGC TAAGGTGGAA
AAAGGAAAGT CTAAGAAGCT GAAGTCCGTG AAGGAACTCT TGGGAATCAC
TATCATGGAA AGATCATCCT TTGAAAAGAA CCCTATCGAT TTCCTGGAGG
CTAAGGGTTA CAAGGAGGTC AAGAAAGACC TCATCATTAA ACTGCCAAAA
TACTCTCTCT TCGAGCTGGA AAATGGCAGG AAGAGAATGT TGGCCAGCGC
CGGAGAGCTG CAAAAGGGAA ACGAGCTTGC TCTGCCCTCC AAATATGTTA
ATTTTCTCTA TCTCGCTTCC CACTATGAAA AGCTGAAAGG GTCTCCCGAA
GATAACGAGC AGAAGCAGCT GTTCGTCGAA CAGCACAAGC ACTATCTGGA
TGAAATAATC GAACAAATAA GCGAGTTCAG CAAAAGGGTT ATCCTGGCGG
ATGCTAATTT GGACAAAGTA CTGTCTGCTT ATAACAAGCA CCGGGATAAG
CCTATTAGGG AACAAGCCGA GAATATAATT CACCTCTTTA CACTCACGAA
TCTCGGAGCC CCCGCCGCCT TCAAATACTT TGATACGACT ATCGACCGGA
AACGGTATAC CAGTACCAAA GAGGTCCTCG ATGCCACCCT CATCCACCAG
TCAATTACTG GCCTGTACGA AACACGGATC GACCTCTCTC AACTGGGCGG
CGACTAG
 (SEQ ID NO: 22)

Provided below is the corresponding amino acid sequence of a *S*. *pyogenes* Cas9 molecule. 






















*
 (SEQ ID NO: 23)

Provided below is an exemplary codon optimized nucleic acid sequence encoding a Cas9 molecule of *N. meningitidis.* 
ATGGCCGCCTTCAAGCCCAACCCCATCAACTACATCCTGGGCCTGGACATCGGCATCGCCAG
CGTGGGCTGGGCCATGGTGGAGATCGACGAGGACGAGAACCCCATCTGCCTGATCGACCTGG
GTGTGCGCGTGTTCGAGCGCGCTGAGGTGCCCAAGACTGGTGACAGTCTGGCTATGGCTCGC
CGGCTTGCTCGCTCTGTTCGGCGCCTTACTCGCCGGCGCGCTCACCGCCTTCTGCGCGCTCG
CCGCCTGCTGAAGCGCGAGGGTGTGCTGCAGGCTGCCGACTTCGACGAGAACGGCCTGATCA
AGAGCCTGCCCAACACTCCTTGGCAGCTGCGCGCTGCCGCTCTGGACCGCAAGCTGACTCCT
CTGGAGTGGAGCGCCGTGCTGCTGCACCTGATCAAGCACCGCGGCTACCTGAGCCAGCGCAA
GAACGAGGGCGAGACCGCCGACAAGGAGCTGGGTGCTCTGCTGAAGGGCGTGGCCGACAACG
CCCACGCCCTGCAGACTGGTGACTTCCGCACTCCTGCTGAGCTGGCCCTGAACAAGTTCGAG
AAGGAGAGCGGCCACATCCGCAACCAGCGCGGCGACTACAGCCACACCTTCAGCCGCAAGGA
CCTGCAGGCCGAGCTGATCCTGCTGTTCGAGAAGCAGAAGGAGTTCGGCAACCCCCACGTGA
GCGGCGGCCTGAAGGAGGGCATCGAGACCCTGCTGATGACCCAGCGCCCCGCCCTGAGCGGC
GACGCCGTGCAGAAGATGCTGGGCCACTGCACCTTCGAGCCAGCCGAGCCCAAGGCCGCCAA
GAACACCTACACCGCCGAGCGCTTCATCTGGCTGACCAAGCTGAACAACCTGCGCATCCTGG
AGCAGGGCAGCGAGCGCCCCCTGACCGACACCGAGCGCGCCACCCTGATGGACGAGCCCTAC
CGCAAGAGCAAGCTGACCTACGCCCAGGCCCGCAAGCTGCTGGGTCTGGAGGACACCGCCTT
CTTCAAGGGCCTGCGCTACGGCAAGGACAACGCCGAGGCCAGCACCCTGATGGAGATGAAGG
CCTACCACGCCATCAGCCGCGCCCTGGAGAAGGAGGGCCTGAAGGACAAGAAGAGTCCTCTG
AACCTGAGCCCCGAGCTGCAGGACGAGATCGGCACCGCCTTCAGCCTGTTCAAGACCGACGA
GGACATCACCGGCCGCCTGAAGGACCGCATCCAGCCCGAGATCCTGGAGGCCCTGCTGAAGC
 
ACATCAGCTTCGACAAGTTCGTGCAGATCAGCCTGAAGGCCCTGCGCCGCATCGTGCCCCTG
ATGGAGCAGGGCAAGCGCTACGACGAGGCCTGCGCCGAGATCTACGGCGACCACTACGGCAA
GAAGAACACCGAGGAGAAGATCTACCTGCCTCCTATCCCCGCCGACGAGATCCGCAACCCCG
TGGTGCTGCGCGCCCTGAGCCAGGCCCGCAAGGTGATCAACGGCGTGGTGCGCCGCTACGGC
AGCCCCGCCCGCATCCACATCGAGACCGCCCGCGAGGTGGGCAAGAGCTTCAAGGACCGCAA
GGAGATCGAGAAGCGCCAGGAGGAGAACCGCAAGGACCGCGAGAAGGCCGCCGCCAAGTTCC
GCGAGTACTTCCCCAACTTCGTGGGCGAGCCCAAGAGCAAGGACATCCTGAAGCTGCGCCTG
TACGAGCAGCAGCACGGCAAGTGCCTGTACAGCGGCAAGGAGATCAACCTGGGCCGCCTGAA
CGAGAAGGGCTACGTGGAGATCGACCACGCCCTGCCCTTCAGCCGCACCTGGGACGACAGCT
TCAACAACAAGGTGCTGGTGCTGGGCAGCGAGAACCAGAACAAGGGCAACCAGACCCCCTAC
GAGTACTTCAACGGCAAGGACAACAGCCGCGAGTGGCAGGAGTTCAAGGCCCGCGTGGAGAC
CAGCCGCTTCCCCCGCAGCAAGAAGCAGCGCATCCTGCTGCAGAAGTTCGACGAGGACGGCT
TCAAGGAGCGCAACCTGAACGACACCCGCTACGTGAACCGCTTCCTGTGCCAGTTCGTGGCC
GACCGCATGCGCCTGACCGGCAAGGGCAAGAAGCGCGTGTTCGCCAGCAACGGCCAGATCAC
CAACCTGCTGCGCGGCTTCTGGGGCCTGCGCAAGGTGCGCGCCGAGAACGACCGCCACCACG
CCCTGGACGCCGTGGTGGTGGCCTGCAGCACCGTGGCCATGCAGCAGAAGATCACCCGCTTC
GTGCGCTACAAGGAGATGAACGCCTTCGACGGTAAAACCATCGACAAGGAGACCGGCGAGGT
GCTGCACCAGAAGACCCACTTCCCCCAGCCCTGGGAGTTCTTCGCCCAGGAGGTGATGATCC
GCGTGTTCGGCAAGCCCGACGGCAAGCCCGAGTTCGAGGAGGCCGACACCCCCGAGAAGCTG
CGCACCCTGCTGGCCGAGAAGCTGAGCAGCCGCCCTGAGGCCGTGCACGAGTACGTGACTCC
TCTGTTCGTGAGCCGCGCCCCCAACCGCAAGATGAGCGGTCAGGGTCACATGGAGACCGTGA
AGAGCGCCAAGCGCCTGGACGAGGGCGTGAGCGTGCTGCGCGTGCCCCTGACCCAGCTGAAG
CTGAAGGACCTGGAGAAGATGGTGAACCGCGAGCGCGAGCCCAAGCTGTACGAGGCCCTGAA
GGCCCGCCTGGAGGCCCACAAGGACGACCCCGCCAAGGCCTTCGCCGAGCCCTTCTACAAGT
ACGACAAGGCCGGCAACCGCACCCAGCAGGTGAAGGCCGTGCGCGTGGAGCAGGTGCAGAAG
ACCGGCGTGTGGGTGCGCAACCACAACGGCATCGCCGACAACGCCACCATGGTGCGCGTGGA
CGTGTTCGAGAAGGGCGACAAGTACTACCTGGTGCCCATCTACAGCTGGCAGGTGGCCAAGG
GCATCCTGCCCGACCGCGCCGTGGTGCAGGGCAAGGACGAGGAGGACTGGCAGCTGATCGAC
GACAGCTTCAACTTCAAGTTCAGCCTGCACCCCAACGACCTGGTGGAGGTGATCACCAAGAA
GGCCCGCATGTTCGGCTACTTCGCCAGCTGCCACCGCGGCACCGGCAACATCAACATCCGCA
TCCACGACCTGGACCACAAGATCGGCAAGAACGGCATCCTGGAGGGCATCGGCGTGAAGACC
GCCCTGAGCTTCCAGAAGTACCAGATCGACGAGCTGGGCAAGGAGATCCGCCCCTGCCGCCT
GAAGAAGCGCCCTCCTGTGCGCTAA
 (SEQ ID NO: 24)

Provided below is the corresponding amino acid sequence of a *N*. *meningitidis* Cas9 molecule. 
MAAFKPNPINYILGLDIGIASVGWAMVEIDEDENPICLIDLGVRVFERAEVPKTGDSLAMAR
RLARSVRRLTRRRAHRLLRARRLLKREGVLQAADFDENGLIKSLPNTPWQLRAAALDRKLTP
LEWSAVLLHLIKHRGYLSQRKNEGETADKELGALLKGVADNAHALQTGDFRTPAELALNKFE
KESGHIRNQRGDYSHTFSRKDLQAELILLFEKQKEFGNPHVSGGLKEGIETLLMTQRPALSG
DAVQKMLGHCTFEPAEPKAAKNTYTAERFIWLTKLNNLRILEQGSERPLTDTERATLMDEPY
RKSKLTYAQARKLLGLEDTAFFKGLRYGKDNAEASTLMEMKAYHAISRALEKEGLKDKKSPL
NLSPELQDEIGTAFSLFKTDEDITGRLKDRIQPEILEALLKHISFDKFVQISLKALRRIVPL
MEQGKRYDEACAEIYGDHYGKKNTEEKIYLPPIPADEIRNPVVLRALSQARKVINGVVRRYG
SPARIHIETAREVGKSFKDRKEIEKRQEENRKDREKAAAKFREYFPNFVGEPKSKDILKLRL
YEQQHGKCLYSGKEINLGRLNEKGYVEIDHALPFSRTWDDSFNNKVLVLGSENQNKGNQTPY
EYFNGKDNSREWQEFKARVETSRFPRSKKQRILLQKFDEDGFKERNLNDTRYVNRFLCQFVA
DRMRLTGKGKKRVFASNGQITNLLRGFWGLRKVRAENDRHHALDAVVVACSTVAMQQKITRF
VRYKEMNAFDGKTIDKETGEVLHQKTHFPQPWEFFAQEVMIRVFGKPDGKPEFEEADTPEKL
 
RTLLAEKLSSRPEAVHEYVTPLFVSRAPNRKMSGQGHMETVKSAKRLDEGVSVLRVPLTQLK
LKDLEKMVNREREPKLYEALKARLEAHKDDPAKAFAEPFYKYDKAGNRTQQVKAVRVEQVQK
TGVWVRNHNGIADNATMVRVDVFEKGDKYYLVPIYSWQVAKGILPDRAVVQGKDEEDWQLID
DSFNFKFSLHPNDLVEVITKKARMFGYFASCHRGTGNINIRIHDLDHKIGKNGILEGIGVKT
ALSFQKYQIDELGKEIRPCRLKKRPPVR*
 (SEQ ID NO: 25)

Provided below is an amino acid sequence of a *S*. *aureus* Cas9 molecule. 
MKRNYILGLDIGITSVGYGIIDYETRDVIDAGVRLFKEANVENNEGRRSKRGARRLKRRRRH
RIQRVKKLLFDYNLLTDHSELSGINPYEARVKGLSQKLSEEEFSAALLHLAKRRGVHNVNEV
EEDTGNELSTKEQISRNSKALEEKYVAELQLERLKKDGEVRGSINRFKTSDYVKEAKQLLKV
QKAYHQLDQSFIDTYIDLLETRRTYYEGPGEGSPFGWKDIKEWYEMLMGHCTYFPEELRSVK
YAYNADLYNALNDLNNLVITRDENEKLEYYEKFQIIENVFKQKKKPTLKQIAKEILVNEEDI
KGYRVTSTGKPEFTNLKVYHDIKDITARKEIIENAELLDQIAKILTIYQSSEDIQEELTNLN
SELTQEEIEQISNLKGYTGTHNLSLKAINLILDELWHTNDNQIAIFNRLKLVPKKVDLSQQK
EIPTTLVDDFILSPVVKRSFIQSIKVINAIIKKYGLPNDIIIELAREKNSKDAQKMINEMQK
RNRQTNERIEEIIRTTGKENAKYLIEKIKLHDMQEGKCLYSLEAIPLEDLLNNPFNYEVDHI
IPRSVSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSINGGFTS
FLRRKWKFKKERNKGYKHHAEDALIIANADFIFKEWKKLDKAKKVMENQMFEEKQAESMPEI
ETEQEYKEIFITPHQIKHIKDFKDYKYSHRVDKKPNRELINDTLYSTRKDDKGNTLIVNNLN
GLYDKDNDKLKKLINKSPEKLLMYHHDPQTYQKLKLIMEQYGDEKNPLYKYYEETGNYLTKY
SKKDNGPVIKKIKYYGNKLNAHLDITDDYPNSRNKVVKLSLKPYRFDVYLDNGVYKFVTVKN
LDVIKKENYYEVNSKCYEEAKKLKKISNQAEFIASFYNNDLIKINGELYRVIGVNNDLLNRI
EVNMIDITYREYLENMNDKRPPRIIKTIASKTQSIKKYSTDILGNLYEVKSKKHPQIIKKG*
 (SEQ ID NO: 26)

Provided below is an exemplary codon optimized nucleic acid sequence encoding a Cas9 molecule of *S. aureus* Cas9. 
ATGAAAAGGAACTACATTCTGGGGCTGGACATCGGGATTACAAGCGTGGGGTATGGATTAT
TGACTATGAAACAAGGGACGTGATCGACGCAGGCGTCAGACTGTTCAAGGAGGCCAACGTGG
AAAACAATGAGGGACGGAGAAGCAAGAGGGGAGCCAGGCGCCTGAAACGACGGAGAAGGCAC
AGAATCCAGAGGGTGAAGAAACTGCTGTTCGATTACAACCTGCTGACCGACCATTCTGAGCT
GAGTGGAATTAATCCTTATGAAGCCAGGGTGAAAGGCCTGAGTCAGAAGCTGTCAGAGGAAG
AGTTTTCCGCAGCTCTGCTGCACCTGGCTAAGCGCCGAGGAGTGCATAACGTCAATGAGGTG
GAAGAGGACACCGGCAACGAGCTGTCTACAAAGGAACAGATCTCACGCAATAGCAAAGCTCT
GGAAGAGAAGTATGTCGCAGAGCTGCAGCTGGAACGGCTGAAGAAAGATGGCGAGGTGAGAG
GGTCAATTAATAGGTTCAAGACAAGCGACTACGTCAAAGAAGCCAAGCAGCTGCTGAAAGTG
CAGAAGGCTTACCACCAGCTGGATCAGAGCTTCATCGATACTTATATCGACCTGCTGGAGAC
TCGGAGAACCTACTATGAGGGACCAGGAGAAGGGAGCCCCTTCGGATGGAAAGACATCAAGG
AATGGTACGAGATGCTGATGGGACATTGCACCTATTTTCCAGAAGAGCTGAGAAGCGTCAAG
TACGCTTATAACGCAGATCTGTACAACGCCCTGAATGACCTGAACAACCTGGTCATCACCAG
GGATGAAAACGAGAAACTGGAATACTATGAGAAGTTCCAGATCATCGAAAACGTGTTTAAGC
AGAAGAAAAAGCCTACACTGAAACAGATTGCTAAGGAGATCCTGGTCAACGAAGAGGACATC
AAGGGCTACCGGGTGACAAGCACTGGAAAACCAGAGTTCACCAATCTGAAAGTGTATCACGA
TATTAAGGACATCACAGCACGGAAAGAAATCATTGAGAACGCCGAACTGCTGGATCAGATTG
CTAAGATCCTGACTATCTACCAGAGCTCCGAGGACATCCAGGAAGAGCTGACTAACCTGAAC
AGCGAGCTGACCCAGGAAGAGATCGAACAGATTAGTAATCTGAAGGGGTACACCGGAACACA
CAACCTGTCCCTGAAAGCTATCAATCTGATTCTGGATGAGCTGTGGCATACAAACGACAATC
 
AGATTGCAATCTTTAACCGGCTGAAGCTGGTCCCAAAAAGGTGGACCTGAGTCAGCAGAAA
GAGATCCCAACCACACTGGTGGACGATTTCATTCTGTCACCCGTGGTCAAGCGGAGCTTCAT
CCAGAGCATCAAAGTGATCAACGCCATCATCAAGAAGTACGGCCTGCCCAATGATATCATTA
TCGAGCTGGCTAGGGAGAAGAACAGCAAGGACGCACAGAAGATGATCAATGAGATGCAGAAA
CGAAACCGGCAGACCAATGAACGCATTGAAGAGATTATCCGAACTACCGGGAAAGAGAACGC
AAAGTACCTGATTGAAAAAATCAAGCTGCACGATATGCAGGAGGGAAAGTGTCTGTATTCTC
TGGAGGCCATCCCCCTGGAGGACCTGCTGAACAATCCATTCAACTACGAGGTCGATCATATT
ATCCCCAGAAGCGTGTCCTTCGACAATTCCTTTAACAACAAGGTGCTGGTCAAGCAGGAAGA
GAACTCTAAAAAGGGCAATAGGACTCCTTTCCAGTACCTGTCTAGTTCAGATTCCAAGATCT
CTTACGAAACCTTTAAAAAGCACATTCTGAATCTGGCCAAAGGAAAGGGCCGCATCAGCAAG
ACCAAAAAGGAGTACCTGCTGGAAGAGCGGGACATCAACAGATTCTCCGTCCAGAAGGATTT
TATTAACCGGAATCTGGTGGACACAAGATACGCTACTCGCGGCCTGATGAATCTGCTGCGAT
CCTATTTCCGGGTGAACAATCTGGATGTGAAAGTCAAGTCCATCAACGGCGGGTTCACATCT
TTTCTGAGGCGCAAATGGAAGTTTAAAAAGGAGCGCAACAAAGGGTACAAGCACCATGCCGA
AGATGCTCTGATTATCGCAAATGCCGACTTCATCTTTAAGGAGTGGAAAAAGCTGGACAAAG
CCAAGAAAGTGATGGAGAACCAGATGTTCGAAGAGAAGCAGGCCGAATCTATGCCCGAAATC
GAGACAGAACAGGAGTACAAGGAGATTTTCATCACTCCTCACCAGATCAAGCATATCAAGGA
TTTCAAGGACTACAAGTACTCTCACCGGGTGGATAAAAAGCCCAACAGAGAGCTGATCAATG
ACACCCTGTATAGTACAAGAAAAGACGATAAGGGGAATACCCTGATTGTGAACAATCTGAAC
GGACTGTACGACAAAGATAATGACAAGCTGAAAAAGCTGATCAACAAAAGTCCCGAGAAGCT
GCTGATGTACCACCATGATCCTCAGACATATCAGAAACTGAAGCTGATTATGGAGCAGTACG
GCGACGAGAAGAACCCACTGTATAAGTACTATGAAGAGACTGGGAACTACCTGACCAAGTAT
AGCAAAAAGGATAATGGCCCCGTGATCAAGAAGATCAAGTACTATGGGAACAAGCTGAATGC
CCATCTGGACATCACAGACGATTACCCTAACAGTCGCAACAAGGTGGTCAAGCTGTCACTGA
AGCCATACAGATTCGATGTCTATCTGGACAACGGCGTGTATAAATTTGTGACTGTCAAGAAT
CTGGATGTCATCAAAAAGGAGAACTACTATGAAGTGAATAGCAAGTGCTACGAAGAGGCTAA
AAAGCTGAAAAAGATTAGCAACCAGGCAGAGTTCATCGCCTCCTTTTACAACAACGACCTGA
TTAAGATCAATGGCGAACTGTATAGGGTCATCGGGGTGAACAATGATCTGCTGAACCGCATT
GAAGTGAATATGATTGACATCACTTACCGAGAGTATCTGGAAAACATGAATGATAAGCGCCC
CCCTCGAATTATCAAAACAATTGCCTCTAAGACTCAGAGTATCAAAAAGTACTCAACCGACA
TTCTGGGAAACCTGTATGAGGTGAAGAGCAAAAAGCACCCTCAGATTATCAAAAAGGGC
 (SEQ ID NO: 39)

If any of the above Cas9 sequences are fused with a peptide or polypeptide at the C-terminus, it is understood that the stop codon will be removed.

### Other Cas Molecules and Cas9 Polypeptides

Various types of Cas molecules or Cas9 polypeptides can be used to practice the inventions disclosed herein. In some embodiments, Cas molecules of Type II Cas systems are used. In other embodiments, Cas molecules of other Cas systems are used. For example, Type I or Type III Cas molecules may be used. Exemplary Cas molecules (and Cas systems) are described, e.g., in Haft et al., PLOS COMPUTATIONAL BIOLOGY 2005, 1(6): e60 and Makarova et al., NATURE REVIEW MICROBIOLOGY 2011, 9:467-477. Exemplary Cas molecules (and Cas systems) are also shown in **Table 22.**

| **Table 22: Cas Systems** | | | | | |
|---|---|---|---|---|---|
| Gene **name^{‡}** | **System type or subtype** | **Name from Haft *et al.*^{§}** | **Structure of encoded protein (PDB accessions)^{¶}** | **Families (and superfamily) of encoded protein^{#**}** | **Rep resentatives** |
| *cas1* | • Type I | *cas1* | 3GOD, 3LFX and 2YZS | COG1518 | SERP2463, SPy1047 *andygbT* |
| | • Type II | | | | |
| | • Type III | | | | |
| *cas2* | • Type I | *cas2* | 2IVY, 2I8E and 3EXC | COG1343 and COG3512 | SERP2462, SPy1048, SPy1723 (N-terminal domain) and *ygbF* |
| | • Type II | | | | |
| | • Type III | | | | |
| *cas3'* | • Type I^{‡‡} | *cas3* | NA | COG1203 | APE1232 and *ygcB* |
| *cas3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a*, *cas5d*, *cas5e*, *cas5h*, *cas5p, cas5t* and *cmx5* | 3KG4 | COG1688 (RAMP) | APE1234, BH0337, *devS* and *ygcI* |
| | • Subtype I-B | | | | |
| | • Subtype I-C | | | | |
| | • Subtype I-E | | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III-A• Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A | *csa2*, *csd2*, *cse4*, *csh2*, *csp1* and *cst2* | NA | COG1857 and COG3649 (RAMP) | *devR* and *ygcJ* |
| | • Subtype I-B | | | | |
| | • Subtype I-C | | | | |
| | • Subtype I-E | | | | |
| *cas8a1* | • Subtype I-A^{‡‡} | *cmx1*, *cst1*, *csx8, csx13* and CXXC-CXXC | NA | BH0338-like | LA3191^{§§} and PG2018^{§§} |
| *cas8a2* | • Subtype I-A^{‡‡} | *csa4* and *csx9* | NA | PH0918 | AF0070, AF1873, MJ0385, PF0637, PH0918 and SSO1401 |
| *cas8b* | • Subtype I-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |

| Gene **name^{‡}** | **System type** or **subtype** | **Name from Haft *et al.*^{§}** | **Structure of encoded protein (PDB accessions)^{¶}** | **Families (and superfamily) of encoded protein^{#**}** | **Representatives** |
|---|---|---|---|---|---|
| *cas9* | • Type II^{‡‡} | *csn1* and *csx12* | NA | COG3513 | FTN_0757 and SPy1046 |
| *cas10* | • Type III^{‡‡} | *cmr2*, *csm1* and *csx11* | NA | COG1353 | MTH326, Rv2823c^{§§} and TM1794^{§§} |
| *cas10d* | • Subtype I-D^{‡‡} | *csc3* | NA | COG1353 | slr7011 |
| *csy1* | • Subtype I-F^{‡‡} | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype I-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy1049-like | SPy1049 |
| *csm2* | • Subtype III-A^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 2OEB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and |
| | | | | | GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3*, *csx1*, *csx2*, DXTHG, NE0113 and TIGR02710 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

### IV. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule are described, e.g., in Jinek *et al.*, SCIENCE 2012, 337(6096):816-821.

### Binding and Cleavage Assay: Testing the endonuclease activity of Cas9 molecule

The ability of a Cas9 molecule/gRNA molecule complex to bind to and cleave a target nucleic acid can be evaluated in a plasmid cleavage assay. In this assay, synthetic or *in vitro-*transcribed gRNA molecule is pre-annealed prior to the reaction by heating to 95°C and slowly cooling down to room temperature. Native or restriction digest-linearized plasmid DNA (300 ng (~8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radio labeled by incubating with 5 units T4 polynucleotide kinase and ~3-6 pmol (~20-40 mCi) [γ-32P]-ATP in 1X T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgCl2, 1 mM DTT, 5% glycerol) in a total volume of 9 µl. Reactions are initiated by the addition of 1 µl target DNA (10 nM) and incubated for 1 h at 37°C. Reactions are quenched by the addition of 20 µl of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### Binding Assay: Testing the binding of Cas9 molecule to target DNA

Exemplary methods for evaluating the binding of Cas9 molecule to target DNA are described, e.g., in Jinek et al., SCIENCE 2012; 337(6096):816-821.

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing 1X TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [γ-32P]-ATP using T4 polynucleotide kinase for 30 min at 37°C. Polynucleotide kinase is heat denatured at 65°C for 20 min, and unincorporated radio label is removed using a column. Binding assays are performed in buffer containing 20 mM HEPES pH 7.5, 100 mM KCl, 5 mM MgCl₂, 1 mM DTT and 10% glycerol in a total volume of 10 µl. Cas9 protein molecule is programmed with equimolar amounts of pre-annealed gRNA molecule and titrated from 100 pM to 1 µM. Radiolabeled DNA is added to a final concentration of 20 pM. Samples are incubated for 1 h at 37°C and resolved at 4°C on an 8% native polyacrylamide gel containing 1X TBE and 5 mM MgCl₂. Gels are dried and DNA visualized by phosphorimaging.

### Differential Scanning Flourimetry (DSF)

The thermostability of Cas9-gRNA ribonucleoprotein (RNP) complexes can be measured via DSF. This technique measures the thermostability of a protein, which can increase under favorable conditions such as the addition of a binding RNA molecule, e.g., a gRNA.

The assay is performed using two different protocols, one to test the best stoichiometric ratio of gRNA:Cas9 protein and another to determine the best solution conditions for RNP formation.

To determine the best solution to form RNP complexes, a 2uM solution of Cas9 in water+10x SYPRO Orange^{®} (Life Techonologies cat#S-6650) and dispensed into a 384 well plate. An equimolar amount of gRNA diluted in solutions with varied pH and salt is then added. After incubating at room temperature for 10'and brief centrifugation to remove any bubbles,a Bio-Rad CFX384^{™} Real-Time System C1000 Touch^{™} Thermal Cycler with the Bio-Rad CFX Manager software is used to run a gradient from 20°C to 90°C with a 1° increase in temperature every 10seconds.

The second assay consists of mixing various concentrations of gRNA with 2uM Cas9 in optimal buffer from assay 1 above and incubating at RT for 10' in a 384 well plate. An equal volume of optimal buffer + 10x SYPRO Orange^{®} (Life Techonologies cat#S-6650) is added and the plate sealed with Microseal^{®} B adhesive (MSB-1001). Following brief centrifugation to remove any bubbles, a Bio-Rad CFX384^{™} Real-Time System C1000 Touch^{™} Thermal Cycler with the Bio-Rad CFX Manager software is used to run a gradient from 20°C to 90°C with a 1° increase in temperature every 10seconds.

### V. Genome Editing Approaches

Described herein are methods for targeted knockout of one or more copies (e.g., 1, 2, 3, 4, 5, 6, 7, 8, 9, 10 or more copies) of the *UL19, UL30, UL48* and/or *UL54* gene, e.g., using one or more of the approaches or pathways described herein, e.g., using NHEJ.

### V.1 NHEJ Approaches for Gene Targeting

As described herein, nuclease-induced non-homologous end-joining (NHEJ) can be used to target gene-specific knockouts. Nuclease-induced NHEJ can also be used to remove (e.g., delete) sequence (e.g., coding sequence, non-coding sequence, or sequence insertions) in a gene of interest.

While not wishing to be bound by theory, it is believed that, in an embodiment, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double strand cleaving eaCas9 molecules and single strand, or nickase, eaCas9 molecules can be used in the methods and compositions described herein to generate NHEJ-mediated indels. NHEJ-mediated indels targeted to the the gene, e.g., a coding region, e.g., an early coding region of a gene, of interest can be used to knockout (i.e., eliminate expression of) a gene of interest. For example, early coding region of a gene of interest includes sequence immediately following a transcription start site, within a first exon of the coding sequence, or within 500 bp of the transcription start site (e.g., less than 500, 450, 400, 350, 300, 250, 200, 150, 100 or 50 bp).

In some embodiments, NHEJ-mediated indels are introduced into more than one HSV-1 target gene. In an embodiment, in which Cas9 double-stranded nuclease or Cas9 single-stranded nickase is used to introduce mutations into two HSV-1 genes, e.g., UL19 and UL30, UL19 and UL48, UL19 and UL54, UL30 and UL48, UL30 and UL54, or UL48 and UL54, individual gRNAs or gRNA pairs targeting both genes are provided together with Cas9 double-stranded nuclease or single-stranded nickase. In an embodiment, in which Cas9 double-stranded nuclease or Cas9 single-stranded nickase is used to introduce mutations into three HSV-1 genes, e.g., UL19 and UL30 and UL48, or UL19 and UL30 and UL54, UL19 and UL48 and UL54, or UL30 and UL48 and UL54, individual gRNAs or gRNA pairs targeting all three genes are provided together with Cas9 double-stranded nuclease or single-stranded nickase. In an embodiment, in which Cas9 double-stranded nuclease or Cas9 single-stranded nickase is used to introduce mutations into four HSV-1 genes, e.g., UL19 and UL30 and UL48 and UL54, individual gRNAs or gRNA pairs targeting all four genes are provided together with Cas9 double-stranded nuclease or single-stranded nickase.

### Placement of double strand or single strand breaks relative to the target position

In an embodiment, in which a gRNA and Cas9 nuclease generate a double strand break for the purpose of inducing NHEJ-mediated indels, a gRNA, e.g., a unimolecular (or chimeric) or modular gRNA molecule, is configured to position one double-strand break in close proximity to a nucleotide of the target position, e.g., a HSV-1 target UL19 position, a HSV-1 target UL30 position, a HSV-1 target UL48 position or a HSV-1 target UL54 position. In an embodiment, the cleavage site is between 0-30 bp away from the target position (e.g., less than 30, 25, 20, 15, 10, 9, 8, 7, 6, 5, 4, 3, 2 or 1 bp from the target position).

In an embodiment, in which two gRNAs complexing with Cas9 nickases induce two single strand breaks for the purpose of inducing NHEJ-mediated indels, two gRNAs, e.g., independently, unimolecular (or chimeric) or modular gRNA, are configured to position two single-strand breaks to provide for NHEJ repair a nucleotide of the target position. In an embodiment, the gRNAs are configured to position cuts at the same position, or within a few nucleotides of one another, on different strands, essentially mimicking a double strand break. In an embodiment, the closer nick is between 0-30 bp away from the target position (e.g., less than 30, 25, 20, 15, 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of a nucleotide of the target position.

Both double strand cleaving eaCas9 molecules and single strand, or nickase, eaCas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks in deleted). In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

### V.2 Single-Strand Annealing

Single strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single strand overhangs containing the repeat sequences are coated with RPA protein to prevent the repeats sequences from inappropriate annealing, e.g., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### V. 3 Other DNA Repair Pathways

### SSBR (single strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott, Nature Reviews Genetics 9, 619-631 (August 2008), and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are 'damaged'. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonculease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Pol *β* , are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase b, DNA polymerase d, DNA polymerase e, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways hace a common feature in that they typically recognize a lesion on one strand of the DNA, then exo/endonucleaseases remove the lesion and leave a 1-30 nucleotide gap that is sub-sequentially filled in by DNA polymerase and finally sealed with ligase. A more complete picture is given in Li, Cell Research (2008) 18:85-98, and a summary is provided here.

Mismatch repair (MMR) operates on mispaired DNA bases.

The MSH2/6 or MSH2/3 complexes both have ATPases activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutL *α* which possesses an ATPase activity and is important for multiple steps of MMR. It possesses a PCNA/replication factor C (RFC)-dependent endonuclease activity which plays an important role in 3' nick-directed MMR involving EXO1. (EXO1 is a participant in both HR and MMR.) It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol d, RPA, HMGB1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott, Nature Reviews Genetics 9, 619-631 (August 2008), and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the correct nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incised the phosphodiester backbone to create a DNA single strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (~80%) of damaged DNA bases are repaired. However, if the 5' -ends in step 3 are resistant to end processing activity, following one nucleotide insertion by Pol β there is then a polymerase switch to the replicative DNA polymerases, Pol δ/ε, which then add ~2-8 more nucleotides into the DNA repair gap. This creates a 5' -flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polb, Pold, Pole, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn et al., Nature Reviews Molecular Cell Biology 15, 465-481 (2014), and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, e.g., XPF and RAD51C, endonucleases such as RAD51, translesion polymerases, e.g., DNA polymerase zeta and Rev1), and the Fanconi anemia (FA) proteins, e.g., FancJ.

### Other pathways

Several other DNA repair pathways exist in mammals.

Translesion synthesis (TLS) is a pathway for repairing a single stranded break left after a defective replication event and involves translesion polymerases, e.g., DNA pol□ and Rev1..

Error-free postreplication repair (PRR) is another pathway for repairing a single stranded break left after a defective replication event.

### VI. Target Cells

Cas9 molecules and gRNA molecules, e.g., a Cas9 molecule/gRNA molecule complex, can be used to manipulate a cell, e.g., to edit a target nucleic acid, in a wide variety of cells.

In an embodiment, a cell is manipulated by editing (e.g., introducing a mutation in) one or more target genes, e.g., HSV-1 target genes, e.g., target genes as described herein, e.g., the *UL19, UL30, UL48* and/or *UL54* gene. In an embodiment, the expression of one or more target genes (e.g., one or more target genes described herein) is modulated, e.g., *in vivo.*

The Cas9 and gRNA molecules described herein can be delivered to a target cell. In an embodiment, the target cell is an epithelial cell, e.g., an epithelial cell of the oropharynx (including, e.g., an epithelial cell of the nose, gums, lips, tongue or pharynx), an epithelial cell of the finger or fingernail bed, or an epithelial cell of the ano-genital area (including, e.g., an epithelial cell of the penis, scrotum, vulva, vagina, cervix, anus or thighs). In an embodiment, the target cell is a neuronal cell, e.g., a cranial ganglion neuron (e.g. a trigeminal ganglion neuron, e.g., an oculomotor nerve ganglion neuron, e.g., an abducens nerve ganglion neuron, e.g., a trochlear nerve ganglion neuron), e.g. a cervical ganglion neuron, e.g., a sacral ganglion neuron, a sensory ganglion neuron, a cortical neuron, a cerebellar neuron or a hippocampal neuron. In an embodiment, the target cell is an optic cell, e.g. an epithelial cell of the eye, e.g. an epithelial cell of the eyelid, e.g., a conjunctival cell, e.g., a conjunctival epithelial cell, e.g., a corneal keratocyte, e.g., a limbus cell, e.g., a corneal epithelial cell, e.g., a corneal stromal cell, e.g., a ciliary body cell, e.g., a scleral cell, e.g., a lens cell, e.g., a choroidal cell, e.g., a retinal cell, e.g., a rod photoreceptor cell, e.g., a cone photoreceptor cell, e.g., a retinal pigment epithelium cell, e.g., a horizontal cell, e.g., an amacrine cell, e.g., a ganglion cell.

### VII. Delivery, Formulations and Routes of Administration

The components, e.g., a Cas9 molecule and gRNA molecule can be delivered or formulated in a variety of forms, see, e.g., **Tables 23-24.** In an embodiment, one Cas9 molecule and two or more (e.g., 2, 3, 4, or more) different gRNA molecules are delivered, e.g., by an AAV vector. In an embodiment, the sequence encoding the Cas9 molecule and the sequence(s) encoding the two or more (e.g., 2, 3, 4, or more) different gRNA molecules are present on the same nucleic acid molecule, e.g., an AAV vector. When a Cas9 or gRNA component is encoded as DNA for delivery, the DNA will typically but not necessarily include a control region, e.g., comprising a promoter, to effect expression. Useful promoters for Cas9 molecule sequences include CMV, EFS, EF-1a, MSCV, PGK, CAG control promoters. In an embodiment, the promoter is a constitutive promoter. In another embodiment, the promoter is a tissue specific promoter. Useful promoters for gRNAs include H1, 7SK, tRNA, and U6 promoters. Promoters with similar or dissimilar strengths can be selected to tune the expression of components. Sequences encoding a Cas9 molecule can comprise a nuclear localization signal (NLS), e.g., an SV40 NLS. In an embodiment, the sequence encoding a Cas9 molecule comprises at least two nuclear localization signals. In an embodiment a promoter for a Cas9 molecule or a gRNA molecule can be, independently, inducible, tissue specific, or cell specific.

**Table 23** provides examples of how the components can be formulated, delivered, or administered.

**Table 23**

| Elements | | |
|---|---|---|
| Cas9 Molecule(s) | gRNA molecule(s) | Comments |
| DNA | DNA | In this embodiment, a Cas9 molecule, typically an eaCas9 molecule, and a gRNA are transcribed from DNA. In this embodiment, they are encoded on separate molecules. |
| DNA | | In this embodiment, a Cas9 molecule, typically an eaCas9 molecule, and a gRNA are transcribed from DNA, here from a single molecule. |
| DNA | RNA | In this embodiment, a Cas9 molecule, typically an eaCas9 molecule, is transcribed from DNA,and a gRNA is provided as in vitro transcribed or synthesized RNA |
| mRNA | RNA | In this embodiment, a Cas9 molecule, typically an eaCas9 molecule, is translated from in vitro transcribed mRNA, and a gRNA is provided as in vitro transcribed or synthesized RNA. |
| mRNA | DNA | In this embodiment, a Cas9 molecule, typically an eaCas9 molecule, is translated from in vitro transcribed mRNA, and a gRNA is transcribed from DNA. |
| Protein | DNA | In this embodiment, a Cas9 molecule, typically an eaCas9 molecule, is provided as a protein, and a gRNA is transcribed from DNA. |
| Protein | RNA | In this embodiment, an eaCas9 molecule is provided as a protein, and a gRNA is provided as transcribed or synthesized RNA. |

**Table 24** summarizes various delivery methods for the components of a Cas system, e.g., the Cas9 molecule component and the gRNA molecule component, as described herein.

**Table 24**

| **Delivery Vector/Mode** | | **Delivery into Non-Dividing Cells** | **Duration of Expression** | **Genome Integration** | **Type of Molecule Delivered** |
|---|---|---|---|---|---|
| **Physical (eg, electroporation, particle gun, Calcium Phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with modifications | RNA |
| | **Adenovirus** | YES | Transient | NO | DNA |
| | **Adeno-Associated Virus (AAV)** | YES | Stable | NO | DNA |
| | **Vaccinia Virus** | YES | Very Transient | NO | DNA |
| | **Herpes Simplex Virus** | YES | Stable | NO | DNA |
| ***Non-Viral*** | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like Particles** | YES | Transient | NO | Nucleic Acids |
| | **Biological liposomes: Erythrocyte Ghosts and Exosomes** | YES | Transient | NO | Nucleic Acids |

### DNA-based Delivery of a Cas9 molecule and/or one or more gRNA molecule

Nucleic acids encoding Cas9 molecules (e.g., eaCas9 molecules) and/or gRNA molecules, can be administered to subjects or delivered into cells by art-known methods or as described herein. For example, Cas9-encoding and/or gRNA-encoding DNA can be delivered, e.g., by vectors (e.g., viral or non-viral vectors), non-vector based methods (e.g., using naked DNA or DNA complexes), or a combination thereof.

DNA encoding Cas9 molecules (e.g., eaCas9 molecules) and/or gRNA molecules can be conjugated to molecules promoting uptake by the target cells (e.g., the target cells described herein).

In some embodiments, the Cas9- and/or gRNA-encoding DNA is delivered by a vector (e.g., viral vector/virus or plasmid).

A vector can comprise a sequence that encodes a Cas9 molecule and/or a gRNA molecule. A vector can also comprise a sequence encoding a signal peptide *(e.g.,* for nuclear localization, nucleolar localization, mitochondrial localization), fused, e.g., to a Cas9 molecule sequence. For example, ae vector can comprise a nuclear localization sequence (e.g., from SV40) fused to the sequence encoding the Cas9 molecule.

One or more regulatory/control elements, e.g., a promoter, an enhancer, an intron, a polyadenylation signal, a Kozak consensus sequence, internal ribosome entry sites (IRES), a 2A sequence, and splice acceptor or donor can be included in the vectors. In some embodiments, the promoter is recognized by RNA polymerase II (e.g., a CMV promoter). In other embodiments, the promoter is recognized by RNA polymerase III (e.g., a U6 promoter). In some embodiments, the promoter is a regulated promoter (e.g., inducible promoter). In other embodiments, the promoter is a constitutive promoter. In some embodiments, the promoter is a tissue specific promoter. In some embodiments, the promoter is a viral promoter. In other embodiments, the promoter is a non-viral promoter.

In some embodiments, the vector or delivery vehicle is a viral vector (e.g., for generation of recombinant viruses). In some embodiments, the virus is a DNA virus (e.g., dsDNA or ssDNA virus). In other embodiments, the virus is an RNA virus (e.g., an ssRNA virus). Exemplary viral vectors/viruses include, e.g., retroviruses, lentiviruses, adenovirus, adeno-associated virus (AAV), vaccinia viruses, poxviruses, and herpes simplex viruses.

In some embodiments, the virus infects dividing cells. In other embodiments, the virus infects non-dividing cells. In some embodiments, the virus infects both dividing and non-dividing cells. In some embodiments, the virus can integrate into the host genome. In some embodiments, the virus is engineered to have reduced immunity, e.g., in human. In some embodiments, the virus is replication-competent. In other embodiments, the virus is replication-defective, e.g., having one or more coding regions for the genes necessary for additional rounds of virion replication and/or packaging replaced with other genes or deleted. In some embodiments, the virus causes transient expression of the Cas9 molecule and/or the gRNA molecule. In other embodiments, the virus causes long-lasting, e.g., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In an embodiment, the viral vector recognizes a specific cell type or tissue. For example, the viral vector can be pseudotyped with a different/alternative viral envelope glycoprotein; engineered with a cell type-specific receptor (e.g., genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (e.g., a ligand-receptor, monoclonal antibody, avidin-biotin and chemical conjugation).

Exemplary viral vectors/viruses include, e.g., retroviruses, lentiviruses, adenovirus, adeno-associated virus (AAV), vaccinia viruses, poxviruses, and herpes simplex viruses.

In some embodiments, the Cas9- and/or gRNA-encoding DNA is delivered by a recombinant retrovirus. In some embodiments, the retrovirus (e.g., Moloney murine leukemia virus) comprises a reverse transcriptase, e.g., that allows integration into the host genome. In some embodiments, the retrovirus is replication-competent. In other embodiments, the retrovirus is replication-defective, e.g., having one of more coding regions for the genes necessary for additional rounds of virion replication and packaging replaced with other genes, or deleted.

In some embodiments, the Cas9- and/or gRNA-encoding DNA is delivered by a recombinant lentivirus. For example, the lentivirus is replication-defective, e.g., does not comprise one or more genes required for viral replication.

In some embodiments, the Cas9- and/or gRNA-encoding DNA is delivered by a recombinant adenovirus. In some embodiments, the adenovirus is engineered to have reduced immunity in human.

In some embodiments, the Cas9- and/or gRNA-encoding DNA is delivered by a recombinant AAV. In some embodiments, the AAV does not incorporate its genome into that of a host cell, e.g., a target cell as describe herein. In some embodiments, the AAV can incorporate at least part of its genome into that of a host cell, e.g., a target cell as described herein. In some embodiments, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh 10, and pseudotyped AAV, such as AAV2/8, AAV2/5 and AAV2/6 can also be used in the disclosed methods. In an embodiment, an AAV capsid that can be used in the methods described herein is a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

In an embodiment, the Cas9- and/or gRNA-encoding DNA is delivered in a re-engineered AAV capsid, e.g., with 50% or greater, e.g., 60% or greater, 70% or greater, 80% or greater, 90% or greater, or 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

In an embodiment, the Cas9- and/or gRNA-encoding DNA is delivered by a chimeric AAV capsid. Exemplary chimeric AAV capsids include, but are not limited to, AAV9i1, AAV2i8, AAV-DJ, AAV2G9, AAV2i8G9, or AAV8G9.

In an embodiment, the AAV is a self-complementary adeno-associated virus (scAAV), e.g., a scAAV that packages both strands which anneal together to form double stranded DNA.

In some embodiments, the Cas9- and/or gRNA-encoding DNA is delivered by a hybrid virus, e.g., a hybrid of one or more of the viruses described herein. In an embodiment, the hybrid virus is hybrid of an AAV (e.g., of any AAV serotype), with a Bocavirus, B19 virus, porcine AAV, goose AAV, feline AAV, canine AAV, or MVM.

A Packaging cell is used to form a virus particle that is capable of infecting a target cell. Such a cell includes a 293 cell, which can package adenovirus, and a ψ2 cell or a PA317 cell, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, eg. Cas9. For example, an AAV vector used in gene therapy typically only possesses inverted terminal repeat (ITR) sequences from the AAV genome which are required for packaging and gene expression in the host or target cell. The missing viral functions can be supplied in trans by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. In embodiment, the viral DNA is packaged in a producer cell line, which contains E1A and/or E1B genes from adenovirus. The cell line is also infected with adenovirus as a helper. The helper virus (e.g., adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, e.g., heat treatment to which adenovirus is more sensitive than AAV.

In an embodiment, the viral vector has the ability of cell type and/or tissue type recognition. For example, the viral vector can be pseudotyped with a different/alternative viral envelope glycoprotein; engineered with a cell type-specific receptor (e.g., geneticmodification of the viral envelope glycoproteins to incorporate targeting ligands such as a peptide ligand, a single chain antibodie, a growth factor); and/or engineered to have a molecular bridge with dual specificities with one end recognizing a viral glycoprotein and the other end recognizing a moiety of the target cell surface (e.g., ligand-receptor, monoclonal antibody, avidin-biotin and chemical conjugation).

In an embodiment, the viral vector achieves cell type specific expression. For example, a tissue-specific promoter can be constructed to restrict expression of the transgene (Cas 9 and gRNA) in only the target cell. The specificity of the vector can also be mediated by microRNA-dependent control of transgene expression. In an embodiment, the viral vector has increased efficiency of fusion of the viral vector and a target cell membrane. For example, a fusion protein such as fusion-competent hemagglutin (HA) can be incorporated to increase viral uptake into cells. In an embodiment, the viral vector has the ability of nuclear localization. For example, aviruse that requires the breakdown of the nuclear envelope (during cell division) and therefore will not infect a non-diving cell can be altered to incorporate a nuclear localization peptide in the matrix protein of the virus thereby enabling the transduction of non-proliferating cells.

In some embodiments, the Cas9- and/or gRNA-encoding DNA is delivered by a non-vector based method (e.g., using naked DNA or DNA complexes). For example, the DNA can be delivered, e.g., by organically modified silica or silicate (Ormosil), electroporation, gene gun, sonoporation, magnetofection, lipid-mediated transfection, dendrimers, inorganic nanoparticles, calcium phosphates, or a combination thereof.

In some embodiments, the Cas9- and/or gRNA-encoding DNA is delivered by a combination of a vector and a non-vector based method. For example, a virosome comprises a liposome combined with an inactivated virus (e.g., HIV or influenza virus), which can result in more efficient gene transfer, e.g., in a respiratory epithelial cell than either a viral or a liposomal method alone.

In an embodiment, the delivery vehicle is a non-viral vector. In an embodiment, the non-viral vector is an inorganic nanoparticle. Exemplary inorganic nanoparticles include, e.g., magnetic nanoparticles (e.g., Fe₃MnO₂) or silica. The outer surface of the nanoparticle can be conjugated with a positively charged polymer (e.g., polyethylenimine, polylysine, polyserine) which allows for attachment (e.g., conjugation or entrapment) of payload.. In an embodiment, the non-viral vector is an organic nanoparticle (e.g., entrapment of the payload inside the nanoparticle). Exemplary organic nanoparticles include, e.g., SNALP liposomes that contain cationic lipids together with neutral helper lipids which are coated with polyethylene glycol (PEG) and protamine and nucleic acid complex coated with lipid coating.

Exemplary lipids for gene transfer are shown below in **Table 25.**

**Table 25: Lipids Used for Gene Transfer**

| **Lipid** | **Abbreviation** | **Feature** |
|---|---|---|
| 1,2-Dioleoyl-sn- glycero- 3 -phosphatidylcholine | DOPC | Helper |
| 1,2-Dioleoyl-sn- glycero- 3 -phosphatidylethanolamine | DOPE | Helper |
| Cholesterol | | Helper |
| *N*-[1-(2,3-Dioleyloxy)prophyl]*N*,*N*,*N*-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| *N*-(3-Aminopropyl)-*N*,*N*-dimethyl-2,3-bis(dodecyloxy)-1-propanaminium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-*N*-[2(sperminecarboxamido-ethyl]-*N*,*N*-dimethyl-1-propanaminium trifluoroacetate | DOSPA | Cationic |
| 1,2-Dioleyl-3-trimethylammonium-propane | DOPA | Cationic |
| *N*-(2-Hydroxyethyl)-*N*,*N*-dimethyl-2,3-bis(tetradecyloxy)-1-propanaminium bromide | MDRIE | Cationic |
| Dimyristooxypropyl dimethyl hydroxyethyl ammonium bromide | DMRI | Cationic |
| 3β-[*N*-(*N*',*N*'-Dimethylaminoethane)-carbamoyl]cholesterol | DC-Chol | Cationic |
| Bis- guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac- [(2,3-Dioctadecyloxypropyl)(2-hydroxyethyl)] -dimethylammonium chloride | CLIP-1 | Cationic |
| rac- [2(2,3 -Dihexadecyloxypropyl-oxymethyloxy)ethyl]trimethylammonium bromide | CLIP-6 | Cationic |
| Ethyldimyristoylphosphatidylcholine | EDMPC | Cationic |
| 1,2-Distearyloxy-*N*,*N*-dimethyl-3-aminopropane | DSDMA | Cationic |
| 1,2-Dimyristoyl-trimethylammonium propane | DMTAP | Cationic |
| *O*,*O*'-Dimyristyl-N-lysyl aspartate | DMKE | Cationic |
| 1,2- Distearoyl-sn-glycero-3-ethylphosphocholine | DSEPC | Cationic |
| *N*-PalmitoylD-erythro-sphingosyl carbamoyl-spermine | CCS | Cationic |
| *N*-*t*-Butyl-*N*0-tetradecyl-3-tetradecylaminopropionamidine | diC14-amidine | Cationic |
| Octadecenolyoxy[ethyl-2-heptadecenyl-3 hydroxyethyl] imidazolinium chloride | DOTIM | Cationic |
| *N*1-Cholesteryloxycarbonyl-3,7-diazanonane-1,9-diamine | CDAN | Cationic |
| 2-(3-[Bis(3-amino-propyl)-amino]propylamino)-*N-*ditetradecylcarbamoylme-ethyl-acetamide | RPR209120 | Cationic |
| 1,2-dilinoleyloxy-3- dimethylaminopropane | DLinDMA | Cationic |
| 2,2-dilinoleyl-4-dimethylaminoethyl-[1,3]- dioxolane | DLin-KC2-DMA | Cationic |
| dilinoleyl- methyl-4-dimethylaminobutyrate | DLin-MC3-DMA | Cationic |

Exemplary polymers for gene transfer are shown below in **Table 26.**

**Table 26: Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3 '-dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In an embodiment, the vehicle has targeting modifications to increase target cell update of nanoparticles and liposomes, e.g., cell specific antigens, monoclonal antibodies, single chain antibodies, aptamers, polymers, sugars, and cell penetrating peptides. In an embodiment, the vehicle uses fusogenic and endosome-destabilizing peptides/polymers. In an embodiment, the vehicle undergoes acid-triggered conformational changes (e.g., to accelerate endosomal escape of the cargo). In an embodiment, a stimuli-cleavable polymer is used, e.g., for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (e.g., naturally or artificially engineered to be invasive but attenuated to prevent pathogenesis and expressing the transgene (e.g., *Listeria monocytogenes*, certain *Salmonella strains*, *Bifidobacterium longum*, and modified *Escherichia coli*), bacteria having nutritional and tissue-specific tropism to target specific tissues, bacteria having modified surface proteins to alter target tissue specificity). In an embodiment, the vehicle is a genetically modified bacteriophage (e.g., engineered phages having large packaging capacity, less immunogenic, containing mammalian plasmid maintenance sequences and having incorporated targeting ligands). In an embodiment, the vehicle is a mammalian virus-like particle. For example, modified viral particles can be generated (e.g., by purification of the "empty" particles followed by *ex vivo* assembly of the virus with the desired cargo). The vehicle can also be engineered to incorporate targeting ligands to alter target tissue specificity. In an embodiment, the vehicle is a biological liposome. For example, the biological liposome is a phospholipid-based particle derived from human cells (e.g., erythrocyte ghosts, which are red blood cells broken down into spherical structures derived from the subject (e.g., tissue targeting can be achieved by attachment of various tissue or cell-specific ligands), or secretory exosomes -subject (i.e., patient) derived membrane-bound nanovescicle (30 -100 nm) of endocytic origin (e.g., can be produced from various cell types and can therefore be taken up by cells without the need of for targeting ligands).

In an embodiment, one or more nucleic acid molecules (e.g., DNA molecules) other than the components of a Cas system, e.g., the Cas9 molecule component and/or the gRNA molecule component described herein, are delivered. In an embodiment, the nucleic acid molecule is delivered at the same time as one or more of the components of the Cas system are delivered. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means than one or more of the components of the Cas system, e.g., the Cas9 molecule component and/or the gRNA molecule component, are delivered. The nucleic acid molecule can be delivered by any of the delivery methods described herein. For example, the nucleic acid molecule can be delivered by a viral vector, e.g., an integration-deficient lentivirus, and the Cas9 molecule component and/or the gRNA molecule component can be delivered by electroporation, e.g., such that the toxicity caused by nucleic acids (e.g., DNAs) can be reduced. In an embodiment, the nucleic acid molecule encodes a therapeutic protein, e.g., a protein described herein. In an embodiment, the nucleic acid molecule encodes an RNA molecule, e.g., an RNA molecule described herein.

### Delivery of RNA encoding a Cas9 molecule

RNA encoding Cas9 molecules (e.g., eaCas9 molecules or eiCas9 molecules) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, lipid-mediated transfection, peptide-mediated delivery, or a combination thereof. Cas9-encoding and/or gRNA-encoding RNA can be conjugated to molecules promoting uptake by the target cells (e.g., target cells described herein).

### Delivery Cas9 molecule protein

Cas9 molecules (e.g., eaCas9 molecules or eiCas9 molecules) can be delivered into cells by art-known methods or as described herein. For example, Cas9 protein molecules can be delivered, e.g., by microinjection, electroporation, lipid-mediated transfection, peptide-mediated delivery, or a combination thereof. Delivery can be accompanied by DNA encoding a gRNA or by a gRNA. Cas9 protein can be conjugated to molecules promoting uptake by the target cells (e.g., target cells described herein).

### Multiplex Delivery of gRNAs

In some embodiments, the Cas9 molecule, e.g., double-stranded nuclease or single-stranded nickase, is delivered together with gRNAs or gRNA pairs targeting more than one HSV-1 target position. The gRNAs can be provided in different forms, e.g., the forms described in **Table 23.** The gRNAs can be delivered by different methods, e.g., the methods described in **Table 24.** It is contemplated herein that the Cas9 molecule, and gRNAs or gRNA pairs targeting more than one HSV-1 target position could be contained within a single or multiple delivery vector or formulation including, e.g., those described above.

### Route of Administration

Systemic modes of administration include oral and parenteral routes. Parenteral routes include, by way of example, intravenous, intrarterial, intraosseous, intramuscular, intradermal, subcutaneous, intranasal and intraperitoneal routes. Components administered systemically may be modified or formulated to target the components to epithelial or neuronal cells.

Local modes of administration include, by way of example, topical, intrathecal, intraspinal, intra-cerebroventricular, intraparenchymal (e.g., into the parenchyma of the brain or spinal cord), and intraocular, including intravitreal, intracorneal, and intraretinal routes. In an embodiment, local modes of administration include intraocular, e.g., intrascleral, e.g., intracorneal, e.g., intravitreal, e.g., intraretinal, e.g. submacular, e.g., intra-lens. In an embodiment, components described herein are delivered subretinally, e.g., by subretinal injection. In an embodiment, components described herein are delivered by intravitreal injection.

In an embodiment, local modes of administration include topical, e.g., topical optic delivery, e.g., topical dermal delivery.

In an embodiment, local modes of administration include intra-parenchymal into the dorsal root ganglion at the level of the trigeminal nerve. In an embodiment, local modes of administration include intra-parenchymal into the dorsal root ganglion at the level of the sacral ganglia. In an embodiment, local modes of administration include intra-parenchymal into the dorsal root ganglion at the level of the lumbar ganglia. In an embodiment, local modes of administration include intra-parenchymal into the dorsal root ganglion at the level of the thoracic ganglia. In an embodiment, local modes of administration include intra-parenchymal into the dorsal root ganglion at the level of the cervical ganglia, e.g., superior cervical ganglion, e.g., middle cervical ganglion, e.g., inferior cervical ganglion.

In an embodiment, significantly smaller amounts of the components (compared with systemic approaches) may exert an effect when administered locally (for example, topically; for example intracorneally) compared to when administered systemically (for example, intravenously). Local modes of administration can reduce or eliminate the incidence of potentially toxic side effects that may occur when therapeutically effective amounts of a component are administered systemically.

In an embodiment, components described herein are delivered by topical administration to epithelial cells, e.g., epithelial cells of the oropharynx (including, e.g., epithelial cells of the nose, gums, lips, tongue or pharynx), epithelial cells of the finger or fingernail bed, or epithelial cells of the ano-genital area (including, e.g., epithelial cells of the penis, vulva, vagina or anus). In an embodiment, components described herein are delivered by topical administration to the eye (including, e.g., corneal epithelium, e.g., corneal stroma, e.g., epithelium of upper and lower eyelid, e.g., lens).

In an embodiment, nanoparticle or viral, e.g., AAV vector, delivery is via topical administration.

Administration may be provided as a periodic bolus or as continuous infusion from an internal reservoir or from an external reservoir (for example, from an intravenous bag). Components may be administered locally, for example, by continuous release from a sustained release drug delivery device.

Administration may be provided as continuous infusion from an internal reservoir (for example, from an implant disposed at an intra- or extra-ocular location (see, U.S. Pat. Nos. 5,443,505 and 5,766,242) or from an external reservoir (for example, from an intravenous bag). Components may be administered locally, for example, by continuous release from a sustained release drug delivery device immobilized to an inner wall of the eye or via targeted transscleral controlled release into the choroid (see, for example, PCT/USOO/00207, PCT/US02/14279, Ambati et al. (2000) Invest. Ophthalmol. Vis. Sci.41:1181-1185, and Ambati et al. (2000) Invest. Ophthalmol. Vis. Sci.41:1186-1191). A variety of devices suitable for administering components locally to the inside of the eye are known in the art. See, for example, U.S. Pat. Nos. 6,251,090, 6,299,895, 6,416,777, 6,413,540, and PCT/US00/28187.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for injection. Typically the microspheres are composed of a polymer of lactic acid and glycolic acid, which are structured to form hollow spheres. The spheres can be approximately 15-30 microns in diameter and can be loaded with components described herein.

### Bi-Modal or Differential Delivery of Components

Separate delivery of the components of a Cas system, e.g., the Cas9 molecule component and the gRNA molecule component, and more particularly, delivery of the components by differing modes, can enhance performance, e.g., by improving tissue specificity and safety.

In an embodiment, the Cas9 molecule and the gRNA molecule are delivered by different modes, or as sometimes referred to herein as differential modes. Different or differential modes, as used herein, refer modes of delivery that confer different pharmacodynamic or pharmacokinetic properties on the subject component molecule, e.g., a Cas9 molecule, or gRNA molecule,. For example, the modes of delivery can result in different tissue distribution, different half-life, or different temporal distribution, e.g., in a selected compartment, tissue, or organ.

Some modes of delivery, e.g., delivery by a nucleic acid vector that persists in a cell, or in progeny of a cell, e.g., by autonomous replication or insertion into cellular nucleic acid, result in more persistent expression of and presence of a component. Examples include viral, e.g., adeno- associated virus or lentivirus, delivery.

By way of example, the components, e.g., a Cas9 molecule and a gRNA molecule, can be delivered by modes that differ in terms of resulting half-life or persistent of the delivered component the body, or in a particular compartment, tissue or organ. In an embodiment, a gRNA molecule can be delivered by such modes. The Cas9 molecule component can be delivered by a mode which results in less persistence or less exposure to the body or a particular compartment or tissue or organ.

More generally, in an embodiment, a first mode of delivery is used to deliver a first component and a second mode of delivery is used to deliver a second component. The first mode of delivery confers a first pharmacodynamic or pharmacokinetic property. The first pharmacodynamic property can be, e.g., distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, e.g., distribution, persistence, or exposure, of the component, or of a nucleic acid that encodes the component, in the body, a compartment, tissue or organ.

In an embodiment, the first pharmacodynamic or pharmacokinetic property, e.g., distribution, persistence or exposure, is more limited than the second pharmacodynamic or pharmacokinetic property.

In an embodiment, the first mode of delivery is selected to optimize, e.g., minimize, a pharmacodynamic or pharmacokinetic property, e.g., distribution, persistence or exposure.

In an embodiment, the second mode of delivery is selected to optimize, e.g., maximize, a pharmacodynamic or pharmcokinetic property, e.g., distribution, persistence or exposure.

In an embodiment, the first mode of delivery comprises the use of a relatively persistent element, e.g., a nucleic acid, e.g., a plasmid or viral vector, e.g., an AAV or lentivirus. As such vectors are relatively persistent product transcribed from them would be relatively persistent.

In an embodiment, the second mode of delivery comprises a relatively transient element, e.g., an RNA or protein.

In an embodiment, the first component comprises gRNA, and the delivery mode is relatively persistent, e.g., the gRNA is transcribed from a plasmid or viral vector, e.g., an

AAV or lentivirus. Transcription of these genes would be of little physiological consequence because the genes do not encode for a protein product, and the gRNAs are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance, safety and efficacy. E.g., the likelihood of an eventual off-target modification can be reduced. Delivery of immunogenic components, e.g., Cas9 molecules, by less persistent modes can reduce immunogenicity, as peptides from the bacterially-derived Cas enzyme are displayed on the surface of the cell by MHC molecules. A two-part delivery system can alleviate these drawbacks.

Differential delivery modes can be used to deliver components to different, but overlapping target regions. The formation active complex is minimized outside the overlap of the target regions. Thus, in an embodiment, a first component, e.g., a gRNA molecule is delivered by a first delivery mode that results in a first spatial, e.g., tissue, distribution. A second component, e.g., a Cas9 molecule is delivered by a second delivery mode that results in a second spatial, e.g., tissue, distribution. In an embodiment, the first mode comprises a first element selected from a liposome, nanoparticle, e.g., polymeric nanoparticle, and a nucleic acid, e.g., viral vector. The second mode comprises a second element selected from the group. In an embodiment, the first mode of delivery comprises a first targeting element, e.g., a cell specific receptor or an antibody, and the second mode of delivery does not include that element. In embodiment, the second mode of delivery comprises a second targeting element, e.g., a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

### Ex vivo delivery

In some embodiments, components described in **Table 23** are introduced into cells which are then introduced into the subject, e.g., cells are removed from a subject, manipulated ex vivo and then introduced into the subject. Methods of introducing the components can include, e.g., any of the delivery methods described in **Table 24.**

### VIII. Modified Nucleosides, Nucleotides, and Nucleic Acids

Modified nucleosides and modified nucleotides can be present in nucleic acids, e.g., particularly gRNA, but also other forms of RNA, e.g., mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, e.g., all bases have a modified phosphate group, e.g., all are phosphorothioate groups. In an embodiment, all, or substantially all, of the phosphate groups of a unimolecular or modular gRNA molecule are replaced with phosphorothioate groups.

In an embodiment, modified nucleotides, e.g., nucleotides having modifications as described herein, can be incorporated into a nucleic acid, e.g., a "modified nucleic acid." In some embodiments, the modified nucleic acids comprise one, two, three or more modified nucleotides. In some embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in one aspect the modified nucleic acids described herein can contain one or more modified nucleosides or nucleotides, e.g., to introduce stability toward nucleases.

In some embodiments, the modified nucleosides, modified nucleotides, and modified nucleic acids described herein can exhibit a reduced innate immune response when introduced into a population of cells, e.g., *in vivo.* The term "innate immune response" includes a cellular response to exogenous nucleic acids, including single stranded nucleic acids, generally of viral or bacterial origin, which involves the induction of cytokine expression and release, particularly the interferons, and cell death. In some embodiments, the modified nucleosides, modified nucleotides, and modified nucleic acids described herein can disrupt binding of a major groove interacting partner with the nucleic acid. In some embodiments, the modified nucleosides, modified nucleotides, and modified nucleic acids described herein can exhibit a reduced innate immune response when introduced into a population of cells, e.g., *in vivo,* and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl *(e.g.,* n-propyl and isopropyl), butyl *(e.g.,* n-butyl, isobutyl, t-butyl), pentyl *(e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (e.g., having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic nonaromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### The Phosphate Group

In some embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In some embodiments, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In some embodiments, one of the non-bridging phosphate oxygen atoms in the phosphate backbone moiety can be replaced by any of the following groups: sulfur (S), selenium (Se), BR₃ (wherein R can be, e.g., hydrogen, alkyl, or aryl), C (e.g., an alkyl group, an aryl group, and the like), H, NR₂ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In some embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In some embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In some embodiments, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), O(CH₂CH₂O)ₙCH₂CH₂OR wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the "oxy"-2' hydroxyl group modification can include "locked" nucleic acids (LNA) in which the 2' hydroxyl can be connected, e.g., by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy, O(CH₂)ₙ-amino, (wherein amino can be, e.g., NH₂; alkylamino, dialkylamino, heterocyclyl, arylamino, diarylamino, heteroarylamino, or diheteroarylamino, ethylenediamine, or polyamino). In some embodiments, the "oxy"-2' hydroxyl group modification can include the methoxyethyl group (MOE), (OCH₂CH₂OCH₃, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., NH₂; alkylamino, dialkylamino, heterocyclyl, arylamino, diarylamino, heteroarylamino, diheteroarylamino, or amino acid); NH(CH₂CH₂NH)ₙCH₂CH₂-amino (wherein amino can be, e.g., as described herein), - NHC(O)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the 1' position on the sugar, e.g., alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, e.g. L-nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In some embodiments, the modified nucleotides can include multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In some embodiments, the nucleobase can include, for example, naturally-occurring and synthetic derivatives of a base.

### Uracil

In some embodiments, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm⁵s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm ⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(τm⁵s2U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm⁵Um), 5-carbamoylmethyl-2'-O-methyl-uridine (ncm ⁵Um), 5-carboxymethylaminomethyl-2'-O-methyl-uridine (cmnm ⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

In some embodiments, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

In some embodiments, the modified nucleobase is a modified adenine. Exemplary nucleobases and nucleosides having a modified adenine include without limitation 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶2Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

In some embodiments, the modified nucleobase is a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW^{∗}), 7-deaza-guanosine, queuosine (Q), epoxyqueuosine (oQ), galactosyl-queuosine (galQ), mannosyl-queuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### Exemplary Modified gRNAs

In some embodiments, the modified nucleic acids can be modified gRNAs. It is to be understood that any of the gRNAs described herein can be modified in accordance with this section, including any gRNA that comprises a targeting domain from any of **Tables 1A-1G, Tables 2A-2G, Tables 3A-3G, Tables 4A-4F, Tables 5A-5E, Tables 6A-6G, Tables 7A-7D, Tables 8A-8E, Tables 9A-9G, Tables 10A-10C, Tables 11A-11E, Tables 12A-12G, Tables 13A-13C, Tables 14A-14E, Tables 15A-15G, Tables 16A-16C, or Table 27**.

As discussed above, transiently expressed or delivered nucleic acids can be prone to degradation by, e.g., cellular nucleases. Accordingly, in one aspect the modified gRNAs described herein can contain one or more modified nucleosides or nucleotides which introduce stability toward nucleases. While not wishing to be bound by theory it is also believed that certain modified gRNAs described herein can exhibit a reduced innate immune response when introduced into a population of cells, particularly the cells of the present invention. As noted above, the term "innate immune response" includes a cellular response to exogenous nucleic acids, including single stranded nucleic acids, generally of viral or bacterial origin, which involves the induction of cytokine expression and release, particularly the interferons, and cell death.

While some of the exemplary modification discussed in this section may be included at any position within the gRNA sequence, in some embodiments, a gRNA comprises a modification at or near its 5' end (e.g., within 1-10, 1-5, or 1-2 nucleotides of its 5' end). In some embodiments, a gRNA comprises a modification at or near its 3' end (e.g., within 1-10, 1-5, or 1-2 nucleotides of its 3' end). In some embodiments, a gRNA comprises both a modification at or near its 5' end and a modification at or near its 3' end.

In an embodiment, the 5' end of a gRNA is modified by the inclusion of a eukaryotic mRNA cap structure or cap analog (e.g., a *G(5')ppp(5')G* cap analog, a *m7G(5')ppp(5')G* cap analog, or a 3 *'-O-Me-m7G(5')ppp(5')G* anti reverse cap analog (ARCA)). The cap or cap analog can be included during either chemical synthesis or *in vitro* transcription of the gRNA.

In an embodiment, an *in vitro* transcribed gRNA is modified by treatment with a phosphatase (e.g., calf intestinal alkaline phosphatase) to remove the 5' triphosphate group.

In an embodiment, the 3' end of a gRNA is modified by the addition of one or more (e.g., 25-200) adenine (A) residues. The polyA tract can be contained in the nucleic acid (e.g., plasmid, PCR product, viral genome) encoding the gRNA, or can be added to the gRNA during chemical synthesis, or following *in vitro* transcription using a polyadenosine polymerase (e.g., *E. coli* Poly(A)Polymerase).

In an embodiment, *in vitro* transcribed gRNA contains both a 5' cap structure or cap analog and a 3' polyA tract. In an embodiment, an *in vitro* transcribed gRNA is modified by treatment with a phosphatase (e.g., calf intestinal alkaline phosphatase) to remove the 5' triphosphate group and comprises a 3' polyA tract.

In some embodiments, gRNAs can be modified at a 3' terminal U ribose. For example, the two terminal hydroxyl groups of the U ribose can be oxidized to aldehyde groups and a concomitant opening of the ribose ring to afford a modified nucleoside as shown below: 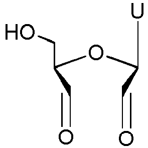 wherein "U" can be an unmodified or modified uridine.

In another embodiment, the 3' terminal U can be modified with a 2'3' cyclic phosphate as shown below: 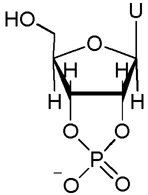 wherein "U" can be an unmodified or modified uridine.

In some embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines and guanosines can be replaced with modified adenosines and guanosines, e.g., with modifications at the 8-position, e.g., 8-bromo guanosine, or with any of the modified adenosines or guanosines described herein.

In some embodiments, sugar-modified ribonucleotides can be incorporated into the gRNA, e.g., wherein the 2' OH-group is replaced by a group selected from H, -OR, -R (wherein R can be, e.g., alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., NH₂; alkylamino, dialkylamino, heterocyclyl, arylamino, diarylamino, heteroarylamino, diheteroarylamino, or amino acid); or cyano (-CN). In some embodiments, the phosphate backbone can be modified as described herein, e.g., with a phosphothioate group. In some embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, e.g., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

In some embodiments, a gRNA can include "locked" nucleic acids (LNA) in which the 2' OH-group can be connected, e.g., by a C1-6 alkylene or C1-6 heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy or O(CH₂)ₙ-amino (wherein amino can be, e.g., NH₂; alkylamino, dialkylamino, heterocyclyl, arylamino, diarylamino, heteroarylamino, or diheteroarylamino, ethylenediamine, or polyamino).

In some embodiments, a gRNA can include a modified nucleotide which is multicyclic (e.g., tricyclo; and "unlocked" forms, such as glycol nucleic acid (GNA) (e.g., R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In an embodiment, a gRNA comprises a 4'-S, 4'-Se or a 4'-C-aminomethyl-2'-O-Me modification.

In some embodiments, deaza nucleotides, e.g., 7-deaza-adenosine, can be incorporated into the gRNA. In some embodiments, O- and N-alkylated nucleotides, e.g., N6-methyl adenosine, can be incorporated into the gRNA. In some embodiments, one or more or all of the nucleotides in a gRNA molecule are deoxynucleotides.

### miRNA binding sites

microrNAs (or miRNAs) are naturally occurring cellular 19-25 nucleotide long noncoding RNAs. They bind to nucleic acid molecules having an appropriate miRNA binding site, e.g., in the 3' UTR of an mRNA, and down-regulate gene expression. While not wishing to be bound by theory it is believed that the down regulation is either by reducing nucleic acid molecule stability or by inhibiting translation. An RNA species disclosed herein, e.g., an mRNA encoding Cas9 can comprise an miRNA binding site, e.g., in its 3'UTR. The miRNA binding site can be selected to promote down regulation of expression is a selected cell type. By way of example, the incorporation of a binding site for miR-122, a microrNA abundant in liver, can inhibit the expression of the gene of interest in the liver.

### EXAMPLES

The following Examples are merely illustrative and are not intended to limit the scope or content of the invention in any way.

### Example 1: Evaluation of candidate guide RNAs (gRNAs)

The suitability of candidate gRNAs can be evaluated as described in this example. Although described for a chimeric gRNA, the approach can also be used to evaluate modular gRNAs.

### Cloning gRNAs into Vectors

For each gRNA, a pair of overlapping oligonucleotides is designed and obtained. Oligonucleotides are annealed and ligated into a digested vector backbone containing an upstream U6 promoter and the remaining sequence of a long chimeric gRNA. Plasmid is sequence-verified and prepped to generate sufficient amounts of transfection-quality DNA. Alternate promoters maybe used to drive *in vivo* transcription (e.g. H1 promoter) or for *in vitro* transcription (e.g., a T7 promoter).

### Cloning gRNAs in linear dsDNA molecule (STITCHR)

For each gRNA, a single oligonucleotide is designed and obtained. The U6 promoter and the gRNA scaffold (e.g. including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive in vivo transcription (e.g., H1 promoter) or for in vitro transcription (e.g., T7 promoter). Any gRNA scaffold may be used to create gRNAs compatible with Cas9s from any bacterial species.

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as CelI/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700bp long. For next generation sequencing, PCR amplicons may be 300-500bp long. If the goal is to knockout gene function, sequencing may be used to assess what percent of viral copies have undergone NHEJ-induced indels that result in a frameshift or large deletion or insertion that would be expected to destroy gene function. If the goal is to remove a specific sequence motif, sequencing may be used to assess what percent of viral copies have undergone NHEJ-induced deletions that span this sequence.

### Example 3: Assessment of Activity of Individual gRNAs Targeting a Synthetic HSV-1 Construct

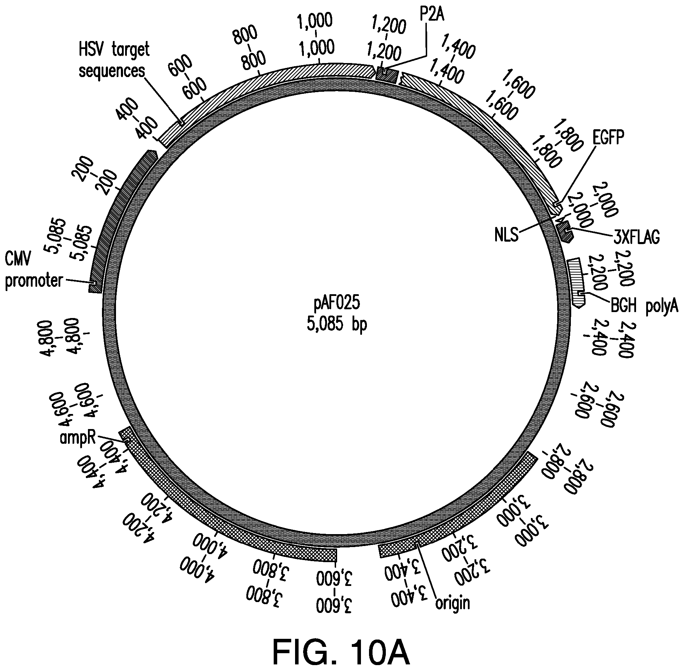

A plasmid containing HSV-1 sequences was constructed as a reporter to measure Cas9-mediated cleavage of target DNA. This reporter plasmid, pAF025, encodes a Green Fluorescent Protein (GFP) driven by a CMV promoter. The target HSV-1 sequences were inserted in frame with the GFP, at its N-terminus, with a P2A self-cleaving peptide sequence between the GFP and the target HSV-1 sequence. **Fig. 10A** shows the plasmid map for pAF025.

gRNAs were identified using a custom guide RNA design software based on the public tool cas-offinder (Bae et al. Bioinformatics. 2014; 30(10): 1473-1475). Each gRNA to be tested was generated as a STITCHR product and co-transfected with a plasmid expressing the *S. aureus* Cas9 (pAF003) into HEK293FT cells. The pAF003 plasmid encodes the *S*. *aureus* Cas9, with N-terminal and C-terminal nuclear localization signals (NLS) and a C-terminal triple flag tag, driven by a CMV promoter. gRNA and Cas9-encoding DNA was introduced into cells along with target plasmid pAF025 by Mirus TransIT-293 transfection reagent. Two days post-transfection, cells were removed from their growth plates by trypsinization, washed in PBS buffer, and analyzed with a BD Accuri Flow Cytometer.

The gRNAs used in the experiments is shown in **Table 27** below.

**Table 27**

| gRNA # | Target sequence | Size | Species | SEQ ID NO: |
|---|---|---|---|---|
| HSV1-UL48-370 | GGAGGGCGAGGTCGTGAAGC | 20 | S. aureus | 6208 |
| HSV1-UL48-314 | GGACGGGGATTCCCCGGGGC | 20 | S. aureus | 6152 |
| HSV1-UL48-340 | GGGGGTAAATCCCGGCCCCG | 20 | S. aureus | 6178 |
| HSV1-UL48-331 | ATCCAGAGCGCCGTAGGGGG | 20 | S. aureus | 6169 |
| HSV1-UL48-877 | CG CTCTGGATATG GCCGACT | 20 | S. aureus | 6607 |
| HSV1-UL54-1960 | GAAATCCTAGACACGCACCGCC | 22 | S. aureus | 18949 |

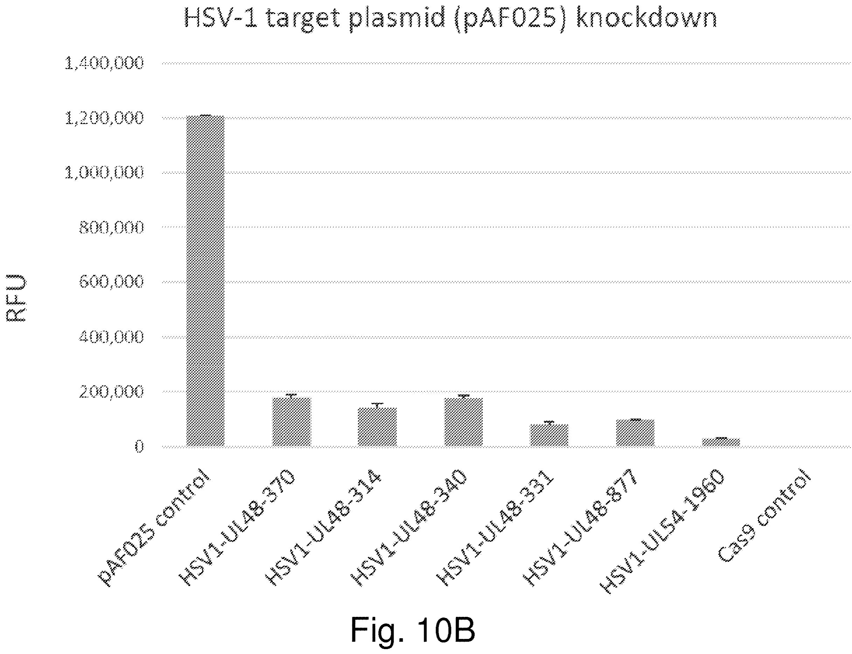

As shown in **Fig. 10B**, there was a reduction in GFP expression as measured by mean fluorescence (or relative fluorescence units, RFU) in the transfected cell population due to Cas9-mediated cleavage of the HSV-1 target sequences in the plasmid pAF025.

In case of conflict, the present application, including any definitions herein, will control.

## Claims

1. A CRISPR/Cas system that is capable of knocking out a herpes simplex virus (HSV) viral *UL48* gene, comprising:
a gRNA molecule comprising a targeting domain, wherein said targeting domain is configured to target a target position that is within the coding region that is downstream of the first 500bp of said HSV viral *UL48* gene; and
an *S. aureus* Cas9 molecule.

2. The system of claim 1, wherein said system is configured to form a double strand break or a single strand break within 200 nts, 150 nts, 100 nts, 50 nts, 25 nts, or 10 nts of the target position.

3. The system of claim 1 or 2, wherein said Cas9 molecule is an enzymatically active Cas9 (eaCas9) molecule, optionally wherein said eaCas9 molecule (a) is a nickase molecule, or (b) has HNH-like domain cleavage activity but has no, or no significant, N-terminal RuvC-like domain cleavage activity, optionally wherein said eaCas9 molecule is an HNH-like domain nickase or comprises a mutation at D10, or (c) N-terminal RuvC-like domain cleavage activity but has no, or no significant, HNH-like domain cleavage activity, optionally wherein said eaCas9 molecule is an N-terminal RuvC-like domain nickase.

4. The system of any one of claims 1-3, wherein said targeting domain consists of a targeting domain nucleotide sequence selected from Table 3E; and/or
wherein said gRNA is a modular gRNA molecule or a chimeric gRNA molecule.

5. The system of any one of claims 1-4, wherein said targeting domain comprises or consists of the nucleotide sequence set forth in SEQ ID NO: 6208, SEQ ID NO: 6152, SEQ ID NO: 6178, SEQ ID NO: 6169, or SEQ ID NO: 6607.

6. A nucleic acid that is capable of knocking out a herpes simplex virus (HSV) viral *UL48* gene, comprising: (a) a first nucleotide sequence that encodes a gRNA molecule comprising a targeting domain, wherein said targeting domain is configured to target a target sequence that is within the coding region that is downstream of the first 500bp of the HSV viral *UL48* gene, and (b) a second nucleotide sequence that encodes an *S. aureus* Cas9 molecule.

7. The nucleic acid of claim 6, wherein said Cas9 molecule is an eaCas9 molecule.

8. A combination comprising (a) a first nucleic acid molecule comprising a nucleotide sequence that encodes a gRNA molecule comprising a targeting domain, wherein said targeting domain is configured to target a target sequence that is within the coding region that is downstream of the first 500bp of the HSV viral *UL48* gene; and (b) a second nucleic acid molecule comprising a nucleotide sequence that encodes an *S. aureus* Cas9 molecule.

9. The combination of claim 8, wherein said Cas9 molecule is an eaCas9 molecule.

10. A cell comprising the CRISPR/Cas system of any one of claims 1-5, the nucleic acid of claim 6 or 7, or the combination of claim 8 or 9.

11. The CRISPR/Cas system of any one of claims 1-5, the nucleic acid of claim 6 or 7, or the combination of claim 8 or 9 for use in therapeutically altering an HSV viral *UL48* gene in a cell.

12. The CRISPR/Cas system, nucleic acid or combination for use of claim 11, wherein said cell is selected from the group consisting of an epithelial cell, a neuronal cell, and an optic cell.

13. A vector comprising (a) the nucleic acid of claim 6 or 7 or (b) the first or second nucleic acid of the combination of claim 8 or 9.

## Patentansprüche

1. CRISPR/Cas-System, das zum Abschalten eines viralen *UL48-Gens* eines Herpes-Simplex-Virus (HSV) fähig ist, das Folgendes umfasst:
ein gRNA-Molekül, das eine Targeting-Domäne umfasst, wobei die Targeting-Domäne so konfiguriert ist, dass sie auf eine Zielposition gerichtet ist, die sich innerhalb der codierenden Region befindet, die sich stromabwärts von den ersten 500 bp des viralen HSV-*UL48-Gens* befindet; und
ein Cas9-Molekül von *S. aureus.*

2. System nach Anspruch 1, wobei das System so konfiguriert ist, dass es einen Doppelstrangbruch oder einen Einzelstrangbruch innerhalb von 200 nts, 150 nts, 100 nts, 50 nts, 25 nts oder 10 nts von der Zielposition bewirkt.

3. System nach Anspruch 1 oder 2, wobei das Cas9-Molekül ein enzymatisch aktives Cas9- (eaCas9-)Molekül ist, wobei das eaCas9-Molekül gegebenenfalls (a) ein Nickase-Molekül ist oder (b) eine HNH-ähnliche Domänenspaltungsaktivität aufweist, aber keine oder keine signifikante N-terminale RuvC-ähnliche Domänenspaltungsaktivität aufweist, wobei das eaCas9-Molekül gegebenenfalls eine Nickase mit einer HNH-ähnlichen Domäne ist oder eine Mutation an D10 aufweist, oder (c) eine N-terminale RuvC-ähnliche Domänenspaltungsaktivität, aber keine oder keine signifikante HNH-ähnliche Domänenspaltungsaktivität aufweist, wobei das eaCas9-Molekül gegebenenfalls eine Nickase mit einer N-terminalen RuvC-ähnlichen Domäne ist.

4. System nach einem der Ansprüche 1-3, wobei die Targeting-Domäne aus einer Targeting-Domänen-Nucleotidsequenz besteht, die aus Tabelle 3E ausgewählt ist; und/oder
wobei die gRNA ein modulares gRNA-Molekül oder ein chimäres gRNA-Molekül ist.

5. System nach einem der Ansprüche 1-4, wobei die Targeting-Domäne die in SEQ ID Nr.: 6208, SEQ ID Nr.: 6152, SEQ ID Nr.: 6178, SEQ ID Nr.: 6169 oder SEQ ID Nr.: 6607 aufgeführte Nucleotidsequenz umfasst oder daraus besteht.

6. Nucleinsäure, die zum Abschalten eines viralen *UL48-Gens* eines Herpes-Simplex-Virus (HSV) fähig ist, die Folgendes umfasst: (a) eine erste Nucleotidsequenz, die ein gRNA-Molekül codiert, das eine Targeting-Domäne umfasst, wobei die Targeting-Domäne so konfiguriert ist, dass sie auf eine Zielsequenz gerichtet ist, die sich innerhalb der codierenden Region befindet, die sich stromabwärts von den ersten 500 bp des viralen HSV-*UL48-Gens* befindet; und (b) eine zweite Nucleotidsequenz, die ein Cas9-Molekül von *S. aureus* codiert.

7. Nucleinsäure nach Anspruch 6, wobei das Cas9-Molekül ein eaCas9-Molekül ist.

8. Kombination, die (a) ein erstes Nucleinsäuremolekül, das eine Nucleotidsequenz umfasst, die ein gRNA-Molekül codiert, das eine Targeting-Domäne umfasst, wobei die Targeting-Domäne so konfiguriert ist, dass sie auf eine Zielsequenz gerichtet ist, die sich innerhalb der codierenden Region befindet, die sich stromabwärts von den ersten 500 bp des viralen HSV-*UL48-Gens* befindet; und (b) ein zweites Nucleinsäuremolekül umfasst, das eine Nucleotidsequenz umfasst, die ein Cas9-Molekül von *S. aureus* codiert.

9. Kombination nach Anspruch 8, wobei das Cas9-Molekül ein eaCas9-Molekül ist.

10. Zelle, die das CRISPR/Cas-System nach einem der Ansprüche 1-5, die Nucleinsäure nach Anspruch 6 oder 7 oder die Kombination nach Anspruch 8 oder 9 umfasst.

11. CRISPR/Cas-System nach einem der Ansprüche 1-5, Nucleinsäure nach Anspruch 6 oder 7 oder Kombination nach Anspruch 8 oder 9 zur Verwendung bei einer therapeutischen Veränderung eines viralen HSV-UL48-Gens in einer Zelle.

12. CRISPR/Cas-System, Nucleinsäure oder Kombination zur Verwendung nach Anspruch 11, wobei die Zelle ausgewählt ist aus der Gruppe bestehend aus einer Epithelzelle, einer neuronalen Zelle und einer Augenzelle.

13. Vektor, der (a) die Nucleinsäure nach Anspruch 6 oder 7 oder (b) die erste oder zweite Nucleinsäure der Kombination nach Anspruch 8 oder 9 umfasst.

## Revendications

1. Système CRISPR/Cas qui est capable de désactiver un gène *UL48* viral du virus de l'herpès simplex (VHS), comprenant :
une molécule d'ARNg comprenant un domaine de ciblage, ledit domaine de ciblage étant configuré pour cibler une position cible qui se trouve à l'intérieur de la région codante qui est en aval des 500 premières pb dudit gène *UL48* viral du VHS ; et
une molécule Cas9 de *S. aureus.*

2. Système selon la revendication 1, ledit système étant configuré pour former une cassure double brin ou une cassure simple brin dans les 200 nucléotides, 150 nucléotides, 100 nucléotides, 50 nucléotides, 25 nucléotides ou 10 nucléotides de la position cible.

3. Système selon la revendication 1 ou 2, ladite molécule de Cas9 étant une molécule de Cas9 enzymatiquement active (eaCas9), éventuellement ladite molécule d'eaCas9 (a) étant une molécule de nickase, ou (b) possédant une activité de clivage de domaine de type HNH mais ne possédant pas d'activité de clivage de domaine de type RuvC N-terminal, ou pas d'activité significative de clivage de domaine de type RuvC N-terminal, éventuellement ladite molécule d'eaCas9 étant une nickase à domaine de type HNH ou comprenant une mutation au niveau de D10, ou (c) possédant une activité de clivage de domaine de type RuvC N-terminal, mais ne possédant pas d'activité de clivage de domaine de type HNH, ou pas d'activité significative de clivage de domaine de type HNH, éventuellement ladite molécule d'eaCas9 étant une nickase à domaine de type RuvC N-terminal.

4. Système selon l'une quelconque des revendications 1 à 3, ledit domaine de ciblage étant constitué d'une séquence de nucléotides de domaine de ciblage choisie parmi le tableau 3E ; et/ou ledit ARNg étant une molécule d'ARNg modulaire ou une molécule d'ARNg chimérique.

5. Système selon l'une quelconque des revendications 1 à 4, ledit domaine de ciblage comprenant ou étant constitué de la séquence de nucléotides indiquée dans la SEQ ID NO: 6 208, la SEQ ID NO: 6 152, la SEQ ID NO: 6 178, la SEQ ID NO: 6 169 ou la SEQ ID NO: 6 607.

6. Acide nucléique qui est capable de désactiver un gène *UL48* viral du virus de l'herpès simplex (VHS), comprenant : (a) une première séquence de nucléotides qui code pour une molécule d'ARNg comprenant un domaine de ciblage, ledit domaine de ciblage étant configuré pour cibler une séquence cible qui est à l'intérieur de la région codante qui est en aval des 500 premières pb dudit gène *UL48* viral du VHS, et (b) une deuxième séquence de nucléotides qui code pour une molécule de Cas9 de *S. aureus.*

7. Acide nucléique selon la revendication 6, ladite molécule de Cas9 étant une molécule d'eaCas9.

8. Combinaison comprenant (a) une première molécule d'acide nucléique comprenant une séquence de nucléotides qui code pour une molécule d'ARNg comprenant un domaine de ciblage, ledit domaine de ciblage étant configuré pour cibler une séquence cible qui est à l'intérieur de la région codante qui est en aval des 500 premières pb dudit gène *UL48* viral du VHS ; et (b) une deuxième molécule d'acide nucléique comprenant une séquence de nucléotides qui code pour une molécule de Cas9 de *S. aureus.*

9. Combinaison selon la revendication 8, la molécule de Cas9 étant une molécule d'eaCas9.

10. Cellule comprenant le système CRISPR/Cas selon l'une quelconque des revendications 1 à 5, l'acide nucléique selon la revendication 6 ou 7, ou la combinaison selon la revendication 8 ou 9.

11. Système CRISPR/Cas selon l'une quelconque des revendications 1 à 5, acide nucléique selon la revendication 6 ou 7, ou combinaison selon la revendication 8 ou 9 pour une utilisation dans l'altération de manière thérapeutique d'un gène *UL48* viral du VHS dans une cellule.

12. Système CRISPR/Cas, acide nucléique ou combinaison pour une utilisation selon la revendication 11, ladite cellule étant choisie dans le groupe constitué par une cellule épithéliale, une cellule neuronale et une cellule optique.

13. Vecteur comprenant (a) l'acide nucléique selon la revendication 6 ou 7 ou (b) le premier ou deuxième acide nucléique de la combinaison selon la revendication 8 ou 9.
